(19) Europäisches Patentamt
European Patent Office
Office européen des brevets

(11) EP 2 944 637 B1

(12) EUROPEAN PATENT SPECIFICATION

(45) Date of publication and mention of the grant of the patent:
**19.02.2020 Bulletin 2020/08**

(21) Application number: **14737606.5**

(22) Date of filing: **14.01.2014**

(51) Int Cl.:
***C07D 471/04*** *(2006.01)* ***A61K 31/4375*** *(2006.01)*
***A61K 31/444*** *(2006.01)* ***A61K 31/4545*** *(2006.01)*
***A61K 31/4709*** *(2006.01)* ***A61K 31/4725*** *(2006.01)*
***A61K 31/496*** *(2006.01)* ***A61K 31/497*** *(2006.01)*
***A61K 31/4985*** *(2006.01)* ***A61K 31/501*** *(2006.01)*
***A61K 31/5025*** *(2006.01)* ***A61K 31/506*** *(2006.01)*
***A61K 31/5377*** *(2006.01)* ***A61K 31/551*** *(2006.01)*
***A61P 3/00*** *(2006.01)* ***A61P 5/00*** *(2006.01)*
***A61P 9/00*** *(2006.01)* ***A61P 25/28*** *(2006.01)*
***A61P 29/00*** *(2006.01)*

(86) International application number:
**PCT/JP2014/050873**

(87) International publication number:
**WO 2014/109414 (17.07.2014 Gazette 2014/29)**

(54) **NITROGEN-CONTAINING HETEROCYLIC COMPOUND OR SALT THEREOF**

STICKSTOFFHALTIGE HETEROCYCLISCHE VERBINDUNG ODER SALZ DAVON

COMPOSÉ HÉTÉROCYCLIQUE CONTENANT DE L'AZOTE OU SEL DE CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.01.2013 JP 2013003832**

(43) Date of publication of application:
**18.11.2015 Bulletin 2015/47**

(73) Proprietor: **FUJIFILM Corporation**
**Tokyo 106-8620 (JP)**

(72) Inventors:
- **FURUYAMA, Hidetomo**
  **Ashigarakami-gun**
  **Kanagawa 258-8577 (JP)**
- **KURIHARA, Hideki**
  **Ashigarakami-gun**
  **Kanagawa 258-8577 (JP)**
- **TERAO, Takahiro**
  **Ashigarakami-gun**
  **Kanagawa 258-8577 (JP)**
- **NAKAGAWA, Daisuke**
  **Ashigarakami-gun**
  **Kanagawa 258-8577 (JP)**
- **TANABE, Shintaro**
  **Ashigarakami-gun**
  **Kanagawa 258-8577 (JP)**
- **KATO, Takayuki**
  **Ashigarakami-gun**
  **Kanagawa 258-8577 (JP)**
- **YAMAMOTO, Masahiko**
  **Ashigarakami-gun**
  **Kanagawa 258-8577 (JP)**
- **SEKINE, Shinichiro**
  **Ashigarakami-gun**
  **Kanagawa 258-8577 (JP)**
- **MASHIKO, Tomoyuki**
  **Ashigarakami-gun**
  **Kanagawa 258-8577 (JP)**
- **INUKI, Shinsuke**
  **Ashigarakami-gun**
  **Kanagawa 258-8577 (JP)**
- **UEDA, Satoshi**
  **Ashigarakami-gun**
  **Kanagawa 258-8577 (JP)**

(74) Representative: **Hoffmann Eitle**
**Patent- und Rechtsanwälte PartmbB**
**Arabellastraße 30**
**81925 München (DE)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 944 637 B1

(56) References cited:

**EP-A1- 1 990 342**
**WO-A1-2009/155156**
**WO-A1-2011/064250**
**WO-A1-2011/079804**
**WO-A1-2014/074657**
**WO-A2-99/17759**
**WO-A2-2007/079999**
**WO-A2-2013/192049**
**JP-A- 2001 518 496**

- **MARIAN WOZNIAK ET AL.: 'Amination and Synthesis of Some Nitronaphthyridines' LIEBIGS ANNALEN DER CHEMIE no. 5, 1993, pages 471 - 475, XP002114654**

**Description**

Technical Field

The present invention relates to a nitrogen-containing heterocyclic compound or salt thereof.

Background Art

The PI3K (phosphatidylinositol 3-kinase)-AKT(protein kinase B) pathway is an important signal transduction pathway that plays a central role in cell growth, proliferation, differentiation, invasion, migration, apoptosis, glucose metabolism, or the like. It is known that the PI3K-AKT pathway is constantly activated in plural malignant tumors (Nature Reviews Drug Discovery, Vol. 8, No. 8, pp. 627-644, 2009) by activation of a receptor on the upstream of the PI3K-AKT pathway, or mutation, defect, or amplification of molecules constituting the PI3K-AKT pathway.

It is reported that the PI3K-AKT pathway is involved in not only malignant tumors but also in other diseases, for example, a cell proliferative disease, an allergic disease, an autoimmune disease, a neurodegenerative disease, a circulatory system disease, an inflammatory disease, an endocrine disorder, a metabolic disorder, or an infection (Biochimica et Biophysica Acta Vol. 1784, No. 1, pp. 159-185, 2008).

Therefore, it is considered that regulating the PI3K-AKT pathway is beneficial in a treatment for various diseases.

In addition, the Ras-Raf-MEK (MAP kinase kinase)-ERK (extracellular signal-regulated kinase) pathway is located on the downstream of various receptors, and plays an important role in cell physiological functions, for example, cell proliferation, apoptosis, or cell differentiation (ChemMedChem Vol. 6, No. 1, pp. 38-48, 2011).

Examples of diseases in which the Ras-Raf-MEK-ERK pathway is involved include a malignant tumor, an allergic disease, an autoimmune disease, a neurodegenerative disease, and a circulatory system disease.

Therefore, it is expected that regulating the Ras-Raf-MEK-ERK pathway is beneficial in a treatment for various diseases.

Here, it is reported that the PI3K-AKT pathway and the Ras-Raf-MEK-ERK pathway complementarily functions regarding cell proliferation, and regulating both the pathways at the same time is beneficial in a treatment for malignant tumors (Cancer Biology & Therapy Vol. 7, No. 2, pp. 307-315, 2008 and Nature medicine, Vol. 14, No. 12, pp. 1351-1356, 2008).

In the treatment of diseases, since the PI3K-AKT pathway and the Ras-Raf-MEK-ERK pathway are very important, PI3K-AKT pathway inhibitors or Ras-Raf-MEK-ERK pathway inhibitors have been developed thus far. However, the number thereof which became commercially available is very small (Pamphlet of International Publication No. WO2011/064250). In addition, regarding compounds that inhibit both the signal pathways directly and at the same time, there are only a few reports (Japanese National-Phase Publication (JP-A) No. 2009-515854) thus far.

On the other hand, 1,5-naphthyridine derivatives having a urea structure in the molecule that inhibits AurolaB, the Ras-Raf-MEK-ERK pathway, and Erk2 have been known (Pamphlet of International Publication No. WO2011/064250).

EP 1 990 342 describes pyrido[2,3-b]pyrazine derivatives, their preparation and use as medicament especially for treating malignant disorders or other disorders based on pathological cell proliferations. WO 2009/155156 discloses compounds having a thiazole scaffold that inhibit mammalian JAK kinases and PDK1. It further discloses these compounds for use in a method of treating myeloproliferative disorders or cancer. WO 99/17759 relates to modulating the function of serine/threonine protein kinase with a 5-azaquinoxaline-based compound by contacting cells that express the serine/threonine protein kinase with these compounds. WO 2014/074657 describes pyridiyl compounds substituted with a bicyclic heterocycle which are useful as kinase modulators, including the modulation of IRAK-4.

SUMMARY OF INVENTION

Technical Problem

A compound or a pharmaceutical composition having excellent inhibitory activity with respect to the PI3K-AKT pathway and/or the Ras-Raf-MEK-ERK pathway is desired.

Solution to Problem

Under such circumstances, the present inventors have conducted extensive studies. As a result, they have found that the nitrogen-containing heterocyclic compound represented by the following Formula [1] or salt thereof has excellent inhibitory activity with respect to the PI3K-AKT pathway and/or the Ras-Raf-MEK-ERK pathway, and completed the invention.

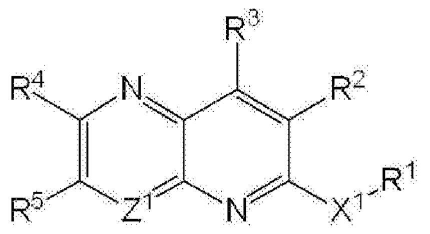

[1]

(In the formula [1]:

$Z^1$ represents $CR^6$;
$R^6$ represents a hydrogen atom, a halogen atom, a hydroxyl group, a $C_{1-6}$ alkyl group, a $C_{2-6}$ alkenyl group, a $C_{2-6}$ alkynyl group, a $C_{3-8}$ cycloalkyl group, an aryl group which may be substituted with one or more substituents selected from a substituent group $\alpha_2$, a $C_{1-6}$ alkoxy group, an aryloxy group, a $C_{1-6}$ alkylthio group, an arylthio group, a heteroaryl group which may be substituted with one or more substituents selected from the substituent group $\alpha_2$, a heteroaryloxy group, a heteroarylthio, a heterocyclyl group which may be substituted with one or more substituents selected from the substituent group $\alpha_2$, or $NR^7R^8$;
each of $R^7$ and $R^8$ independently represents a hydrogen atom, a $C_{1-6}$ alkyl group, a $C_{2-6}$ alkenyl group, a $C_{2-6}$ alkynyl group, a $C_{3-8}$ cycloalkyl group, an aryl group which may be substituted with one or more substituents selected from the substituent group $\alpha_2$, a heteroaryl group which may be substituted with one or more substituents selected from the substituent group $\alpha_2$, or a heterocyclyl group which may be substituted with one or more substituents from the substituent group $\alpha_2$;
the substituent group $\alpha_2$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with one or more substituents selected from a substituent group $\beta_2$, a $C_{1-6}$ alkyl group which may be substituted with one or more substituents selected from the substituent group $\beta_2$, a $C_{1-6}$ alkoxy group which may be substituted with one or more substituents selected from the substituent group $\beta_2$, and an oxo group;
the substituent group $\beta_2$ consists of a halogen atom, a $C_{1-6}$ alkyl group which may be substituted with one or more substituents selected from a substituent group $\gamma_2$, a $C_{3-8}$ cycloalkyl group which may be substituted with one or more substituents selected from the substituent group $\gamma_2$, an aryl group which may be substituted with one or more substituents selected from the substituent group $\gamma_2$, a heteroaryl group which may be substituted with one or more substituents selected from the substituent group $\gamma_2$, and a heterocyclyl group which may be substituted with one or more substituents selected from the substituent group $\gamma_2$; and
the substituent group $\gamma_2$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a $C_{1-6}$ alkyl group which may be substituted with a halogen atom, a $C_{1-6}$ alkoxy group which may be substituted with a halogen atom, and an oxo group;
$X^1$ represents $NR^9$;
$R^9$ represents a hydrogen atom;
$R^1$ represents a monocyclic nitrogen-containing heteroaryl group which may be substituted with one or more substituents selected from a substituent group $A_1$, a monocyclic nitrogen- and oxygen-containing heteroaryl group which may be substituted with one or more substituents selected from a substituent group $A_1$, a monocyclic nitrogen- and sulfur-containing heteroaryl group which may be substituted with one or more substituents selected from a substituent group $A_1$, a bicyclic nitrogen-containing heteroaryl group which may be substituted with one or more substituents

selected from a substituent group $A_1$, a bicyclic nitrogen- and oxygen-containing heteroaryl group which may be substituted with one or more substituents selected from a substituent group $A_1$, or a bicyclic nitrogen- and sulfur-containing heteroaryl group which may be substituted with one or more substituents selected from a substituent group $A_1$;

the substituent group $A_1$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with one or more substituents selected from a substituent group $B_1$, a sulfamoyl group which may be substituted with one or more substituents selected from the substituent group $B_1$, a $C_{1-6}$ alkyl group which may be substituted with one or more substituents selected from the substituent group $B_1$, a $C_{2-6}$ alkenyl group which may be substituted with one or more substituents selected from the substituent group $B_1$, a $C_{3-8}$ cycloalkyl group which may be substituted with one or more substituents selected from the substituent group $B_1$, an aryl group which may be substituted with one or more substituents selected from the substituent group $B_1$, a $C_{1-6}$ alkoxy group which may be substituted with one or more substituents selected from the substituent group $B_1$, a $C_{3-8}$ cycloalkoxy group which may be substituted with one or more substituents selected from the substituent group $B_1$, an aryloxy group which may be substituted with one or more substituents selected from the substituent group $B_1$, a $C_{1-6}$ alkylthio group which may be substituted with one or more substituents selected from the substituent group $B_1$, an arylthio group which may be substituted with one or more substituents selected from the substituent group $B_1$, a $C_{1-6}$ alkylamino group which may be substituted with one or more substituents selected from the substituent group $B_1$, a di($C_{1-6}$ alkyl)amino group which may be substituted with one or more substituents selected from the substituent group $B_1$, an acyl group which may be substituted with one or more substituents selected from the substituent group $B_1$, a heteroaryl group which may be substituted with one or more substituents selected from the substituent group $B_1$, a heterocyclyl group which may be substituted with one or more substituents selected from the substituent group $B_1$, and an oxo group;

the substituent group $B_1$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with one or more substituents selected from a substituent group $C_1$, a sulfamoyl group which may be substituted with one or more substituents selected from the substituent group $C_1$, a $C_{1-6}$ alkyl group which may be substituted with one or more substituents selected from the substituent group $C_1$, a $C_{3-8}$ cycloalkyl group which may be substituted with one or more substituents selected from the substituent group $C_1$, an aryl group which may be substituted with one or more substituents selected from the substituent group $C_1$, a $C_{1-6}$ alkoxy group which may be substituted with one or more substituents selected from the substituent group $C_1$, a $C_{1-6}$ alkylamino group which may be substituted with one or more substituents selected from the substituent group $C_1$, a di($C_{1-6}$ alkyl)amino group which may be substituted with one or more substituents selected from the substituent group $C_1$, a $C_{1-6}$ alkylsulfonyl group which may be substituted with one or more substituents selected from the substituent group $C_1$, an arylsulfonyl group which may be substituted with one or more substituents selected from the substituent group $C_1$, an acyl group which may be substituted with one or more substituents selected from the substituent group $C_1$, a hetero aryl group which may be substituted with one or more substituents selected from the substituent group $C_1$, a heterocyclyl group which may be substituted with one or more substituents selected from the substituent group $C_1$, and an oxo group; and

the substituent group $C_1$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected

from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with a $C_{1-6}$ alkyl group, a $C_{1-6}$ alkyl group which may be substituted with a halogen atom, an aryl group which may be substituted with a halogen atom or a $C_{1-6}$ alkyl group, a $C_{1-6}$ alkoxy group which may be substituted with a halogen atom, and an oxo group;

$R^2$ represents a hydrogen atom;

$R^3$ represents a hydrogen atom;

$R^4$ represents a hydrogen atom;

$R^5$ represents a heteroaryl group which may be substituted with one or more substituents selected from a substituent group $A_3$, a heteroaryloxy group which may be substituted with one or more substituents selected from a substituent group $A_3$, a heteroarylthio group which may be substituted with one or more substituents selected from a substituent group $A_3$, a heterocyclyl group which may be substituted with one or more substituents selected from a substituent group $A_3$, or $NR^{12}R^{13}$;

each of $R^{12}$ and $R^{13}$ independently represents a hydrogen atom, a $C_{1-6}$ alkyl group which may be substituted with one or more substituents selected from the substituent group $A_3$, a $C_{2-6}$ alkenyl group which may be substituted with one or more substituents selected from the substituent group $A_3$, a $C_{2-6}$ alkynyl group which may be substituted with one or more substituents selected from the substituent group $A_3$, a $C_{3-8}$ cycloalkyl group which may be substituted with one or more substituents selected from the substituent group $A_3$, an aryl group which may be substituted with one or more substituents selected from the substituent group $A_3$, a heteroaryl group which may be substituted with one or more substituents selected from the substituent group $A_3$, a heterocyclyl group which may be substituted with one or more substituents selected from the substituent group $A_3$, or an amino-protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group,

the substituent group $A_3$ consists of a halogen atom, a hydroxyl group , an amino group , an amino group which may be substituted with one or more substituents selected from a substituent group $E_3$, a carboxyl group , a cyano group, a carbamoyl group which may be substituted with one or more substituents selected from a substituent group $B_3$, a sulfamoyl group which may be substituted with one or more substituents selected from the substituent group $B_3$, a $C_{1-6}$ alkyl group which may be substituted with one or more substituents selected from the substituent group $B_3$, a $C_{2-6}$ alkenyl group which may be substituted with one or more substituents selected from the substituent group $B_3$, a $C_{3-8}$ cycloalkyl group which may be substituted with one or more substituents selected from the substituent group $B_3$, an aryl group which may be substituted with one or more substituents selected from the substituent group $B_3$, a $C_{1-6}$ alkoxy group which may be substituted with one or more substituents selected from the substituent group $B_3$, an aryloxy group which may be substituted with one or more substituents selected from the substituent group $B_3$, a $C_{1-6}$ alkylthio group which may be substituted with one or more substituents selected from the substituent group $B_3$, an arylthio group which may be substituted with one or more substituents selected from the substituent group $B_3$, a $C_{1-6}$ alkyl amino group which may be substituted with one or more substituents selected from the substituent group $B_3$, a di($C_{1-6}$ alkyl)amino group which may be substituted with one or more substituents selected from the substituent group $B_3$, a $C_{1-6}$ alkylsulfonyl group which may be substituted with one or more substituents selected from the substituent group $B_3$, an arylsulfonyl group which may be substituted with one or more substituents selected from the substituent group $B_3$, an acyl group which may be substituted with one or more substituents selected from the substituent group $B_3$, a heteroaryl group which may be substituted with one or more substituents selected from the substituent group $B_3$, a heterocyclyl group which may be substituted with one or more substituents selected from the substituent group $B_3$, a heterocyclyloxy group which may be substituted with one or more substituents selected from the substituent group $B_3$, and an oxo group;

the substituent group $B_3$ consists of a halogen atom, a hydroxyl group which may be protected by a protecting group selected from a $C_{1-6}$ alkyl group, a $C_{1-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with one or more substituents selected from a substituent group $C_3$, a $C_{1-6}$ alkyl group which may be substituted with one or more substituents selected from the substituent group $C_3$, a $C_{3-8}$ cycloalkyl group which may be substituted with one or more substituents selected from the substituent group $C_3$, an aryl group which may be substituted with one or more substituents selected from the substituent group $C_3$, a $C_{1-6}$ alkoxy group which may be substituted with one or more substituents selected from the substituent group $C_3$, an aryloxy group which may be substituted with one or more substituents selected from the substituent group $C_3$, a $C_{1-6}$ alkylamino group which may be substituted with one or more substituents selected from the substituent group $C_3$, a di($C_{1-6}$ alkyl)amino group which may be substituted with one or more substituents selected from the substituent group $C_3$, a $C_{1-6}$ alkylsulfonyl group which may be substituted with one or more substituents selected from the substituent group $C_3$, an arylsulfonyl group which may be substituted with one or more substituents selected from the substituent group $C_3$, an acyl group which may be substituted with one or more substituents selected from the substituent group $C_3$, a heteroaryl group which may be substituted with one or more substituents selected from the substituent group $C_3$, a heterocyclyl group which may be substituted with one or more substituents selected from the substituent group $C_3$, a heteroaryloxy group which may be substituted with one or more substituents selected from the substituent group $C_3$, a heterocyclyloxy group which may be substituted with one or more substituents selected from the substituent group $C_3$, and an oxo group;

the substituent group $C_3$ consists of a halogen atom, a hydroxyl group , an amino group , a carboxyl group , a nitro group, cyano group, a carbamoyl group which may be substituted with one or more substituents selected from a substituent group $D_3$, a $C_{1-6}$ alkyl group which may be substituted with one or more substituents selected from the substituent group $D_3$, a $C_{3-8}$ cycloalkyl group which may be substituted with one or more substituents selected from the substituent group $D_3$, an aryl group which may be substituted with one or more substituents selected from the substituent group $D_3$, a $C_{1-6}$ alkoxy group which may be substituted with one or more substituents selected from the substituent group $D_3$, an aryloxy group which may be substituted with one or more substituents selected from the substituent group $D_3$, a $C_{1-6}$ alkylamino group which may be substituted with one or more substituents selected from the substituent group $D_3$, a di($C_{1-6}$ alkyl)amino group which may be substituted with one or more substituents selected from the substituent group $D_3$, a $C_{1-6}$ alkylsulfonyl group which may be substituted with one or more substituents selected from the substituent group $D_3$, an acyl group which may be substituted with one or more substituents selected from the substituent group $D_3$, a heteroaryl group which may be substituted with one or more substituents selected from the substituent group $D_3$, a heterocyclyl group which may be substituted with one or more substituents selected from the substituent group $D_3$, a silyl group which may be substituted with one or more substituents selected from the substituent group $D_3$, and an oxo group;

the substituent group $D_3$ consists of a halogen atom, a hydroxyl group, a $C_{1-6}$ alkyl group, a $C_{3-8}$ cycloalkyl group, an aryl group, a $C_{1-6}$ alkoxy group, a $C_{1-6}$ alkylamino group, a di($C_{1-6}$ alkyl)amino group, a $C_{1-6}$ alkylsulfonyl group, a heteroaryl group, a heterocyclyl group, and an oxo group;

the substituent group $E_3$ consists of a $C_{3-8}$ cycloalkyl group which may be substituted with one or more substituents selected from a substituent group $F_3$, an aryl group which may be substituted with one or more substituents selected from the substituent group $F_3$, a heteroaryl group which may be substituted with one or more substituents selected from the substituent group $F_3$, and a heterocyclyl group which may be substituted with one or more substituents selected from the substituent group $F_3$; and the substituent group $F_3$ consists of a halogen atom, a carboxyl group, and a $C_{1-6}$ alkyl group.

Another aspect of the invention provides a pharmaceutical composition containing the nitrogen-containing heterocyclic compound or salt thereof of the invention as defined in claim 10. Still another aspect of the invention provides the nitrogen-containing heterocyclic compound of the invention or the pharmaceutical composition of the invention for use in the treatment of a disease, preferably of a disease in which the PI3K and/or the ERK is involved, wherein the disease is preferably selected from the group consisting of a malignant tumor, a cell proliferative disease, an allergic disease, an autoimmune disease, a neurodegenerative disease, a circulatory system disease, an inflammatory disease, an endocrine disorder, a metabolic disorder, and an infection.

That is, the invention is as defined in claim 1 and the appended claims.

Effects of Invention

The nitrogen-containing heterocyclic compound or salt thereof of the invention has excellent inhibitory activity with respect to the PI3K-AKT pathway and/or the Ras-Raf-MEK-ERK pathway, is useful as a compound for use in a treatment such as prevention of or a cure for a disease such as a malignant tumor, a cell proliferative disease, an allergic disease, an autoimmune disease, a neurodegenerative disease, a circulatory system disease, an inflammatory disease, an endocrine disorder, a metabolic disorder, or an infection, and, in particular useful for prevention of or cure for a malignant tumor.

DESCRIPTION OF EMBODIMENTS

In the present specification, "A and/or B" means "A and B, or A or B".

Hereinafter, the invention will be described in detail.

In the specification, each term has the following meaning, unless specified otherwise.

A "halogen atom" means a fluorine atom, a chlorine atom, a bromine atom, or an iodine atom.

A $C_{1-6}$ alkyl group means a linear or branched $C_{1-6}$ alkyl group such as a methyl group, an ethyl group, a propyl group, an isopropyl group, a butyl group, a sec-butyl group, an isobutyl group, a tert-butyl group, a pentyl group, an isopentyl group, or a hexyl group.

A $C_{2-6}$ alkenyl group means a linear or branched $C_{2-6}$ alkenyl group such as a vinyl group, an allyl group, a propenyl group, an isopropenyl group, a butenyl group, an isobutenyl group, a 1,3-butadienyl group, a pentenyl group, or a hexenyl group.

A $C_{2-6}$ alkynyl group means a linear or branched $C_{2-6}$ alkynyl group such as an ethynyl group, a propynyl group, a butynyl group, a pentynyl group, or a hexynyl group.

A $C_{3-8}$ cycloalkyl group means a monocyclic $C_{3-8}$ cycloalkyl group such as a cyclopropyl group, a cyclobutyl group, a cyclopentyl group, or a cyclohexyl group, or a $C_{4-8}$ crosslinked cyclic hydrocarbon ring group such as a bicyclo[2.1.0]pentyl group, a bicyclo[2.2.0]hexyl group, or a bicyclo[3.2.1]octyl group.

A condensed polycyclic hydrocarbon ring group means a bi- to tetra- cyclic hydrocarbon ring group such as a naphthyl group, an anthryl group, a phenanthryl group, a pyrenyl group, a fluorenyl group, an indenyl group, or an acenaphthylenyl group.

A partially-saturated condensed polycyclic hydrocarbon ring group is a condensed polycyclic hydrocarbon ring group which was partially hydrogenated, and means an indanyl group, an acenaphthenyl group, or the like.

An aryl group means a phenyl group, a condensed polycyclic hydrocarbon ring group, or a partially saturated condensed polycyclic hydrocarbon ring group.

An ar $C_{1-6}$ alkyl group means an ar $C_{1-6}$ alkyl group such as a benzyl group, a diphenylmethyl group, a trityl group, a phenethyl group, or a naphthylmethyl group.

A $C_{1-6}$ alkylene group means a linear or branched $C_{1-6}$ alkylene group such as a methylene group, an ethylene group, a propylene group, a butylene group, or a hexylene group.

A $C_{1-6}$ alkoxy group means a linear or branched $C_{1-6}$ alkyloxy group such as a methoxy group, an ethoxy group, a propyl oxy group, an isopropyl oxy group, a butoxy group, an isobutoxy group, a sec-butoxy group, a tert-butoxy group, a pentyloxy group, or a hexyloxy group.

A $C_{3-8}$ cycloalkoxy group means a $C_{3-8}$ cycloalkyloxy group such as a cyclopropyloxy group, a cyclobutyloxy group, a cyclopentyloxy group, or a cyclohexyloxy group.

A $C_{1-6}$ alkoxy $C_{1-6}$ alkyl group means a $C_{1-6}$ alkyloxy $C_{1-6}$ alkyl group such as a methoxymethyl group or a 1-ethoxyethyl group.

The aryloxy group means a phenyloxy group, a naphthyloxy group, an indanyloxy group, or an indenyloxy group.

A $C_{1-6}$ alkylthio group means a linear or branched $C_{1-6}$ alkylthio group such as a methylthio group, an ethylthio group, a propylthio group, an isopropylthio group, a butylthio group, an isobutylthio group, a sec-butylthio group, a tert-butylthio group, a pentylthio group, or a hexylthio group.

An arylthio group means a phenylthio group, a naphthylthio group, an indanylthio group, or an indenylthio group.

A $C_{1-6}$ alkylsulfonyl group means a linear or branched $C_{1-6}$ alkylsulfonyl group such as a methylsulfonyl group, an ethylsulfonyl group, a propylsulfonyl group, an isopropylsulfonyl group, a butylsulfonyl group, an isobutylsulfonyl group, a sec-butylsulfonyl group, a tert-butylsulfonyl group, a pentylsulfonyl group, or a hexylsulfonyl group.

An arylsulfonyl group means a benzenesulfonyl group, a p-toluenesulfonyl group, a naphthylsulfonyl group, an indanylsulfonyl group, or an indenylsulfonyl group.

A $C_{1-6}$ alkylsulfonyloxy group means a $C_{1-6}$ alkylsulfonyloxy group such as a methylsulfonyloxy group or an ethylsulfonyloxy group.

An arylsulfonyloxy group means a benzenesulfonyloxy group or a p-toluenesulfonyloxy group.

A $C_{2-6}$ alkanoyl group means a linear or branched $C_{2-6}$ alkanoyl group such as an acetyl group, a propionyl group, a valeryl group, an isovaleryl group, or a pivaloyl group.

An aroyl group means a benzoyl group, or a naphthoyl group.

A heterocyclic carbonyl group means a nicotinoyl group, a thenoyl group, a pyrrolidinocarbonyl group, or a furoyl group.

An (a-substituted) aminoacetyl group means an (a-substituted) aminoacetyl group of which the N-terminal may be protected, derived from an amino acid (glycine, alanine, valine, leucine, isoleucine, serine, threonine, cysteine, methionine, aspartic acid, glutamic acid, asparagine, glutamine, arginine, lysine, histidine, hydroxylysine, phenylalanine, tyrosine, tryptophan, proline, or hydroxyproline).

An acyl group means a formyl group, a succinyl group, a glutaryl group, a maleoyl group, a phthaloyl group, a $C_{2-6}$ alkanoyl group, an aroyl group, a heterocyclic carbonyl group, or an ($\alpha$-substituted) aminoacetyl group.

A $C_{1-6}$ alkoxycarbonyl group means a linear or branched $C_{1-6}$ alkyloxycarbonyl group such as a methoxycarbonyl group, an ethoxycarbonyl group, an isopropyloxycarbonyl group, a tert-butoxycarbonyl group, or a 1,1-dimethylpropoxycarbonyl group.

An aryloxycarbonyl group means a phenyloxycarbonyl group or a naphthyloxycarbonyl group.

An ar $C_{1-6}$ alkoxycarbonyl group means an ar $C_{1-6}$ alkyloxycarbonyl group such as a benzyloxycarbonyl group

or a phenethyloxycarbonyl group.

A $C_{1-6}$ alkylamino group means a linear or branched $C_{1-6}$ alkylamino group such as a methylamino group, an ethylamino group, a propylamino group, an isopropylamino group, a butylamino group, a sec-butylamino group, a tert-butylamino group, a pentylamino group, or a hexylamino group.

A di($C_{1-6}$ alkyl)amino group means a linear or branched di($C_{1-6}$ alkyl)amino group such as a dimethylamino group, a diethylamino group, a dipropylamino group, a diisopropylamino group, a dibutylamino group, a di(tert-butyl)amino group, a dipentylamino group, a dihexylamino group, an (ethyl)(methyl)amino group, a (methyl)(propyl)amino group, an (ethyl)(propyl)amino group, or an (ethyl)(isopropyl)amino group.

A nitrogen-containing heterocyclyl group means a heterocyclyl group of which a ring including at least one nitrogen atom does not have aromatic properties, and examples thereof include an azetidinyl group, a pyrrolidinyl group, a piperidinyl group, a homopiperidinyl group, an octahydroazocinyl group, an imidazolidinyl group, a pyrazolidinyl group, a piperazinyl group, and a homopiperazinyl group. The heterocyclyl group may be further condensed with another aromatic ring or another aliphatic ring.

An oxygen-containing heterocyclyl group means a tetrahydrofuranyl group, a tetrahydropyranyl group, an oxetanyl group, a 1,3-dioxanyl group or the like. The heterocyclyl group may be further condensed with another aromatic ring or another aliphatic ring.

A sulfur-containing heterocyclyl group means a tetrahydrothienyl group, a tetrahydrothiopyranyl group or the like. This heterocyclyl group includes a group of which the sulfur atom is oxidized, and may be further condensed with another aromatic ring or another aliphatic ring.

A nitrogen- and oxygen- containing heterocyclyl group means a morpholinyl group, a 1,4-oxazepanyl group or the like. This heterocyclyl group may be further condensed with another aromatic ring or another aliphatic ring.

A nitrogen- and sulfur- containing heterocyclyl group means a thiomorpholinyl or the like. This heterocyclyl group includes a group of which the sulfur atom is oxidized, and may be further condensed with another aromatic ring or another aliphatic ring.

A hetero, crosslinked ring group means a hetero, crosslinked ring group including at least one heteroatom (for example, an oxygen atom, a nitrogen atom, or a sulfur atom), and examples thereof include a 3-aza-6-oxabicyclo[3.1.1]heptyl group, a 3-aza-8-oxabicyclo[3.2.1]octyl group, and a 8-aza-3-oxabicyclo[3.2.1]octyl group.

A heterospiro ring group means a heterospiro ring group including at least one heteroatom (for example, an oxygen atom, a nitrogen atom, or a sulfur atom), and examples thereof include a 2-azaspiro[3.3]heptyl group, a 2-oxaspiro[3.3]heptyl group, a 6-aza-2-oxaspiro[3.3]heptyl group, a 1-azaspiro[4.5]decyl group, and a 1-oxaspiro[4.5]decyl group.

A heterocyclyl group means the nitrogen-containing heterocyclyl group, the oxygen-containing heterocyclyl group, the sulfur-containing heterocyclyl group, the nitrogen- and oxygen-containing heterocyclyl group, the nitrogen- and sulfur- containing heterocyclyl group, the hetero, crosslinked ring group, or the heterospiro ring group.

A heterocyclyloxy group means a group in which an oxy group is bonded to a heterocyclyl group, and examples thereof include an azetidinyloxy group, an oxetanyloxy group, a pyrrolidinyloxy group, a piperidinyloxy group, and a tetrahydropyranyloxy group.

A monocyclic, nitrogen-containing heteroaryl group means a heteroaryl group (which may be partially saturated) of which a ring including at least one nitrogen atom has aromatic properties, and examples thereof include a pyrrolinyl group, a pyrrolyl group, a tetrahydropyridyl group, a pyridyl group, an imidazolinyl group, an imidazolyl group, a pyrazolinyl group, a pyrazolyl group, a pyrazinyl group, a pyridazinyl group, a pyrimidinyl group, a triazolyl group, and a tetrazolyl group. This heteroaryl group may be further condensed with another aromatic ring or another aliphatic ring.

A monocyclic, oxygen-containing heteroaryl group means a heteroaryl group (which may be partially saturated) of which a ring including at least one oxygen atom has aromatic properties, and examples thereof include a furanyl group and a pyranyl group. This heteroaryl group may be further condensed with another aromatic ring or another aliphatic ring.

A monocyclic, sulfur-containing heteroaryl group means a heteroaryl group (which may be partially saturated) of which a ring including at least one sulfur atom has aromatic properties, and examples thereof include a thienyl group. This heteroaryl group may be further condensed with another aromatic ring or another aliphatic ring.

A monocyclic, nitrogen- and oxygen- containing heteroaryl group means an oxazolyl group, an isoxazolyl group, an oxadiazolyl group or the like. This heteroaryl group may be further condensed with another aromatic ring or another aliphatic ring.

A monocyclic, nitrogen- and sulfur- containing heteroaryl group means a thiazolyl group, an isothiazolyl group, a thiadiazolyl group or the like. This heteroaryl group may be further condensed with another aromatic ring or another aliphatic ring.

A bicyclic, nitrogen-containing heteroaryl group means a bicyclic heteroaryl group (which may be partially saturated) of which a ring including at least one nitrogen atom has aromatic properties, and examples thereof include an indolyl group, an isoindolyl group, a benzimidazolyl group, an indazolyl group, a benzotriazolyl group, a quinolyl group, an isoquinolyl group, a tetrahydroquinolyl group, a tetrahydroisoquinolyl group, a quinolizinyl group, a cinnolinyl

group, a phthalazinyl group, a quinazolinyl group, a quinoxalinyl group, a naphthyridinyl group, a pyrrolopyridyl group, an imidazopyridyl group, a pyrazolopyridyl group, a pyridopyrazyl group, a purinyl group, a pteridinyl group, a 5,6,7,8-tetrahydrophthalazinyl group, a 5,6,7,8-tetrahydrocinnolinyl group, a 1,2,3,4-tetrahydropyrido[2,3-d]pyridazinyl group, a 5,6,7,8-tetrahydro-[1,2,4]triazolo[4,3-a]pyrazinyl group, a 5,6,7,8-tetrahydropyrido[3,4-d]pyridazinyl group, a 5,6,7,8-tetrahydropyrido[3,2-c]pyridazinyl group, a 5,6,7,8-tetrahydropyrido[4,3-c] pyridazinyl group, a 6,7-dihydro-5H-cyclopenta[d]pyridazinyl group, a 6,7-dihydro-5H-cyclopenta[c]pyridazinyl group, a 2,3-dihydro-1H-pyrrolo[2,3-d]pyridazinyl group, a 6,7-dihydro-5H-pyrrolo[3,4-d]pyridazinyl group, a 6,7-dihydro-5H-pyrrolo[3,2-c]pyridazinyl group, a 6,7-dihydro-5H-pyrrolo[3,4-c]pyridazinyl group, and a 6,7-dihydro-5H-pyrrolo[2,3-c]pyridazinyl group.

A bicyclic, oxygen-containing heteroaryl group means a bicyclic heteroaryl group (which may be partially saturated) of which a ring including at least one oxygen atom has aromatic properties, and examples thereof include a benzofuranyl group, an isobenzofuranyl group, and a chromenyl group.

A bicyclic, sulfur-containing heteroaryl group means a bicyclic heteroaryl group (which may be partially saturated) of which a ring including at least one sulfur atom has aromatic properties, and examples thereof include a benzothienyl group.

A bicyclic, nitrogen- and oxygen- containing heteroaryl group means a bicyclic heteroaryl group (which may be partially saturated) of which a ring including at least one nitrogen atom and at least one oxygen atom has aromatic properties, and examples thereof include a benzoxazolyl group, a benzoisoxazolyl group, a benzoxadiazolyl group, a dihydropyranopyridyl group, a dihydrodioxynopyridyl group, a dihydropyridooxadienyl group, a 3,4-dihydro-2H-pyrano[2,3-d]pyridazinyl group, a 7,8-dihydro-5H-pyrano[3,4-d]pyridazinyl group, a 7,8-dihydro-6H-pyrano[3,2-c]pyridazinyl group, a 7,8-dihydro-5H-pyrano[4,3-c]pyridazinyl group, a 2,3-dihydrofuro[2,3-d]pyridazinyl group, a 5,7-dihydrofuro[3,4-d]pyridazinyl group, a 6,7-dihydrofuro[3,2-c]pyridazinyl group, a 5,7-dihydrofuro[3,4-c]pyridazinyl group, and a 5,6-dihydrofuro[2,3-c]pyridazinyl group.

A bicyclic, nitrogen- and sulfur- containing heteroaryl group means a bicyclic heteroaryl group (which may be partially saturated) of which a ring including at least one nitrogen atom and at least one sulfur atom has aromatic properties, and examples thereof include a benzothiazolyl group, a benzoisothiazolyl group, a benzothiadiazolyl group, and a thiazolopyridyl group.

A heteroaryl group means the monocyclic, nitrogen-containing heteroaryl group, the monocyclic, oxygen-containing heteroaryl group, the monocyclic, sulfur-containing heteroaryl group, the monocyclic, nitrogen- and oxygen-containing heteroaryl group, the monocyclic, nitrogen- and sulfur- containing heteroaryl group, the bicyclic, nitrogen-containing heteroaryl group, the bicyclic, oxygen-containing heteroaryl group, the bicyclic, sulfur-containing heteroaryl group, the bicyclic, nitrogen- and oxygen- containing heteroaryl group, or the bicyclic, nitrogen- and sulfur- containing heteroaryl group.

A heteroaryloxy group means a group in which an oxy group is bonded to a heteroaryl group, and examples thereof include a pyridyloxy group, a pyridazinyloxy group, and a pyrimidinyloxy group.

A heteroarylthio group means a group in which a thio group is bonded to a heteroaryl group, and examples thereof include a pyridylthio group, a pyridazinylthio group, andr a pyrimidinylthio group.

A silyl group means a trimethylsilyl group, a triethylsilyl group, a tributylsilyl group, a tert-butyldimethylsilyl group or the like.

A leaving group means a halogen atom, a $C_{1-6}$ alkylsulfonyloxy group, an arylsulfonyloxy group or the like. The $C_{1-6}$ alkylsulfonyloxy group or the arylsulfonyloxy group may have a substituent.

Examples of a hydroxyl protecting group include any group that can be usually used as a protecting group of a hydroxyl group, and examples thereof include the groups described in "Protective Groups in Organic Synthesis" written by W. Greene et al., 4th edition, pp.16-366, 2007 (John Wiley & Sons, INC.).

Specific examples thereof include a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group. These groups may have a substituent.

Examples of an amino protecting group include any group that can be usually used as a protecting group of a amino group, and examples thereof include the groups described in "Protective Groups in Organic Synthesis" written by W. Greene et al., 4th edition, pp. 696-926, 2007 (John Wiley & Sons, INC.).

Specific examples thereof include an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group. These groups may have a substituent.

Examples of a carboxyl protecting group include any group that can be usually used as a protecting group of a carboxyl group, and examples thereof include the groups described in "Protective Groups in Organic Synthesis" written by W. Greene et al., 4th edition, pp. 533-646, 2007 (John Wiley & Sons, INC.).

Specific examples thereof include a $C_{1-6}$ alkyl group, a $C_{2-6}$ alkenyl group, an aryl group, an ar $C_{1-6}$ alkyl group, a $C_{1-6}$ alkoxy $C_{1-6}$ alkyl group, and a silyl group. These groups may have a substituent.

An aliphatic hydrocarbon means pentane, hexane, cyclohexane or the like.

A halogenated hydrocarbon means methylene chloride, chloroform, dichloroethane or the like.

An alcohol means methanol, ethanol, propanol, 2-propanol, butanol, 2-methyl-2-propanol, ethylene glycol, diethylene glycol, triethylene glycol, propylene glycol or the like.

An ether means diethyl ether, diisopropyl ether, 1,4-dioxane, tetrahydrofuran, anisole, ethylene glycol dimethyl ether, diethylene glycol dimethyl ether, diethylene glycol diethyl ether or the like.

A ketone means acetone, 2-butanone, 4-methyl-2-pentanone or the like.

An ester means methyl acetate, ethyl acetate, propyl acetate, butyl acetate, cyclohexyl acetate, amyl acetate or the like.

An amide means N,N-dimethylformamide, N,N-dimethylacetamide, N-methylpyrrolidone or the like.

A sulfoxide means dimethyl sulfoxide or the like.

A carboxylic acid means formic acid, acetic acid, trifluoroacetic acid or the like.

An aromatic hydrocarbon means benzene, toluene, xylene or the like.

A palladium catalyst means a metal palladium such as palladium-carbon or palladium black; an inorganic palladium salt such as palladium chloride; an organic palladium salt such as palladium acetate; an organic palladium complex such as tetrakis(triphenylphosphine)palladium(0), bis(tri-tert-butylphosphine)palladium(0), bis(triphenylphosphine)palladium(II) dichloride, (1,1'-bis(diphenylphosphino)ferrocene)palladium(II) dichloride, bis(di-tert-butyl(4-dimethylaminophenyl)phosphine)dichloropalladium(II), (E)-di(μ-acetate)bis(o-(di-o-tolylphosphino)benzyl)dipalladium(II), or tris(dibenzylideneacetone)dipalladium(0); a ploymer-immobilized organic palladium complex such as polymer-supported bis(acetate)triphenylphosphine palladium(II) or polymer-supported di(acetate)dicyclohexylphenylphosphine palladium(II); or the like. These may be used in combination.

Examples of a ligand include: a trialkylphosphine such as trimethylphosphine or tri-tert-butylphosphine; a tricycloalkylphosphine such as tricyclohexylphosphine; a triarylphosphine such as triphenylphosphine or tritolylphosphine; a trialkylphosphite such as trimethylphosphite, triethylphosphite, or tributylphosphite; a tricycloalkylphosphite such as tricyclohexylphosphite; a triarylphosphite such as triphenylphosphite; a imidazolium salt such as 1,3-bis(2,4,6-trimethylphenyl)imidazolium chloride; a diketone such as acetylacetone or octafluoroacetyl acetone; an amine such as trimethylamine, triethylamine, tripropylamine, or triisopropylamine; and 1,1'-bis(diphenylphosphino)ferrocene, 2,2'-bis(diphenylphosphino)-1,1'-binaphthyl, 2-dicyclohexylphosphino-2',6'-dimethoxybiphenyl, 2-dicyclohexylphosphino-2',4',6'-triisopropylbiphenyl, 2-(di-tert-butylphosphino)-2',4',6'-triisopropylbiphenyl, 4,5-bis(diphenylphosphino)-9,9-dimethylxanthene, and 2-(di-tert-butylphosphino)biphenyl. These may be used in combination.

The monocyclic, nitrogen-containing heteroaryl group, the monocyclic, nitrogen- and oxygen- containing heteroaryl group, the monocyclic, nitrogen- and sulfur- containing heteroaryl group, the bicyclic, nitrogen-containing heteroaryl group, the bicyclic, nitrogen- and oxygen-containing heteroaryl group, or the bicyclic, nitrogen- and sulfur- containing heteroaryl group of $R^1$ may be substituted with one or more substituents selected from the substituent group $A_1$.

Preferably, the monocyclic, nitrogen-containing heteroaryl group, the monocyclic, nitrogen- and oxygen- containing heteroaryl group, the monocyclic, nitrogen- and sulfur- containing heteroaryl group, the bicyclic, nitrogen-containing heteroaryl group, the bicyclic, nitrogen- and oxygen-containing heteroaryl group, or the bicyclic, nitrogen- and sulfur- containing heteroaryl group of $R^1$ may be substituted with one or more substituents selected from the substituent group $\alpha_1$.

The $C_{1-6}$ alkyl group or the $C_{1-6}$ alkoxy group of $R^4$ may be substituted with one or more substituents selected from the substituent group $\alpha_2$.

The carbamoyl group, the $C_{1-6}$ alkyl group, the $C_{2-6}$ alkenyl group, the $C_{2-6}$ alkynyl group, the $C_{3-8}$ cycloalkyl group, the aryl group, the $C_{1-6}$ alkoxy group, the aryloxy group, the $C_{1-6}$ alkylthio group, the arylthio group, the heteroaryl group, the heteroaryloxy group, the heteroarylthio group, or the heterocyclyl group of $R^5$ may be substituted with one or more substituents selected from the substituent group $A_3$.

Preferably, the carbamoyl group, the $C_{1-6}$ alkyl group, the $C_{2-6}$ alkenyl group, the $C_{2-6}$ alkynyl group, the $C_{3-8}$ cycloalkyl group, the aryl group, the $C_{1-6}$ alkoxy group, the aryloxy group, the $C_{1-6}$ alkylthio group, the arylthio group, the heteroaryl group, the heteroaryloxy group, the heteroarylthio group, or the heterocyclyl group of $R^5$ may be substituted with one or more substituents selected from the substituent group $\alpha_3$.

The $C_{1-6}$ alkyl group, the $C_{2-6}$ alkenyl group, the $C_{2-6}$ alkynyl group, the $C_{3-8}$ cycloalkyl group, the aryl group, the $C_{1-6}$ alkoxy group, the aryloxy group, the $C_{1-6}$ alkylthio group, the arylthio group, the heteroaryl group, the heteroaryloxy group, the heteroarylthio group, or the heterocyclyl group of $R^6$ may be substituted with one or more substituents selected from the substituent group $\alpha_2$.

The $C_{1-6}$ alkyl group, the $C_{2-6}$ alkenyl group, the $C_{2-6}$ alkynyl group, the $C_{3-8}$ cycloalkyl group, the aryl group, the heteroaryl group, or the heterocyclyl group of $R^7$ or $R^8$ may be substituted with one or more substituents selected from the substituent group $\alpha_2$.

The $C_{1-6}$ alkyl group of $R^9$ may be substituted with one or more substituents selected from the substituent group $\alpha_2$.

The $C_{1-6}$ alkyl group of $R^{10}$ or $R^{11}$ may be substituted with one or more substituents selected from the substituent group $\alpha_2$.

The $C_{1-6}$ alkyl group, the $C_{2-6}$ alkenyl group, the $C_{2-6}$ alkynyl group, the $C_{3-8}$ cycloalkyl group, the aryl group, the heteroaryl group, or the heterocyclyl group of $R^{12}$ or $R^{13}$ may be substituted with one or more substituents selected from the substituent group $A_3$.

Preferably, the $C_{1-6}$ alkyl group, the $C_{2-6}$ alkenyl group, the $C_{2-6}$ alkynyl group, the $C_{3-8}$ cycloalkyl group, the aryl group, the heteroaryl group, or the heterocyclyl group of $R^{12}$ or $R^{13}$ may be substituted with one or more substituents selected from the substituent group $\alpha_3$.

Substituent group $A_1$: A halogen atom, a hydroxyl group which may be protected, an amino group which may be protected, a carboxyl group which may be protected, a cyano group, a carbamoyl group which may be substituted with one or more substituents selected from a substituent group $B_1$, a sulfamoyl group which may be substituted with one or more substituents selected from the substituent group $B_1$, a $C_{1-6}$ alkyl group which may be substituted with one or more substituents selected from the substituent group $B_1$, a $C_{2-6}$ alkenyl group which may be substituted with one or more substituents selected from the substituent group $B_1$, a $C_{3-8}$ cycloalkyl group which may be substituted with one or more substituents selected from the substituent group $B_1$, an aryl group which may be substituted with one or more substituents selected from the substituent group $B_1$, a $C_{1-6}$ alkoxy group which may be substituted with one or more substituents selected from the substituent group $B_1$, a $C_{3-8}$ cycloalkoxy group which may be substituted with one or more substituents selected from the substituent group $B_1$, an aryloxy group which may be substituted with one or more substituents selected from the substituent group $B_1$, a $C_{1-6}$ alkylthio group which may be substituted with one or more substituents selected from the substituent group $B_1$, an arylthio group which may be substituted with one or more substituents selected from the substituent group $B_1$, a $C_{1-6}$ alkylamino group which may be substituted with one or more substituents selected from the substituent group $B_1$, a di($C_{1-6}$ alkyl)amino group which may be substituted with one or more substituents selected from the substituent group $B_1$, an acyl group which may be substituted with one or more substituents selected from the substituent group $B_1$, a heteroaryl group which may be substituted with one or more substituents selected from the substituent group $B_1$, a heterocyclyl group which may be substituted with one or more substituents selected from the substituent group $B_1$, and an oxo group.

Substituent group $B_1$: A halogen atom, a hydroxyl group which may be protected, an amino group which may be protected, a carboxyl group which may be protected, a cyano group, a carbamoyl group which may be substituted with one or more substituents selected from a substituent group $C_1$, a sulfamoyl group which may be substituted with one or more substituents selected from the substituent group $C_1$, a $C_{1-6}$ alkyl group which may be substituted with one or more substituents selected from the substituent group $C_1$, a $C_{3-8}$ cycloalkyl group which may be substituted with one or more substituents selected from the substituent group $C_1$, an aryl group which may be substituted with one or more substituents selected from the substituent group $C_1$, a $C_{1-6}$ alkoxy group which may be substituted with one or more substituents selected from the substituent group $C_1$, a $C_{1-6}$ alkylamino group which may be substituted with one or more substituents selected from the substituent group $C_1$, a di($C_{1-6}$ alkyl)amino group which may be substituted with one or more substituents selected from the substituent group $C_1$, a $C_{1-6}$ alkylsulfonyl group which may be substituted with one or more substituents selected from the substituent group $C_1$, an arylsulfonyl group which may be substituted with one or more substituents selected from the substituent group $C_1$, an acyl group which may be substituted with one or more substituents selected from the substituent group $C_1$, a heteroaryl group which may be substituted with one or more substituents selected from the substituent group $C_1$, a heterocyclyl group which may be substituted with one or more substituents selected from the substituent group $C_1$, and an oxo group.

Substituent group $C_1$: A halogen atom, a hydroxyl group which may be protected, an amino group which may be protected, a carboxyl group which may be protected, a cyano group, a carbamoyl group which may be substituted with a $C_{1-6}$ alkyl group, a $C_{1-6}$ alkyl group which may be substituted with a halogen atom, an aryl group which may be substituted with a halogen atom or a $C_{1-6}$ alkyl group, a $C_{1-6}$ alkoxy group which may be substituted with a halogen atom, and an oxo group.

Substituent group $\alpha_1$: A halogen atom, a hydroxyl group which may be protected, an amino group which may be protected, a carboxyl group which may be protected, a cyano group, a carbamoyl group which may be substituted with one or more substituents selected from a substituent group $\beta_1$, a sulfamoyl group which may be substituted with one or more substituents selected from the substituent group $\beta_1$, a $C_{1-6}$ alkyl group which may be substituted with one or more substituents selected from the substituent group $\beta_1$, a $C_{3-8}$ cycloalkyl group which may be substituted with one or more substituents selected from the substituent group $\beta_1$, an aryl group which may be substituted with one or more substituents selected from the substituent group $\beta_1$, a $C_{1-6}$ alkoxy group which may be substituted with one or more substituents selected from the substituent group $\beta_1$, an aryloxy group which may be substituted with one or more substituents selected from the substituent group $\beta_1$, a $C_{1-6}$ alkylthio group which may be substituted with one or more substituents selected from the substituent group $\beta_1$, an arylthio group which may be substituted with one or more substituents selected from the substituent group $\beta_1$, a $C_{1-6}$ alkylamino group which may be substituted with one or more substituents selected from the substituent group $\beta_1$, a di($C_{1-6}$ alkyl)amino group which may be substituted with one or

more substituents selected from the substituent group $\beta_1$, an acyl group which may be substituted with one or more substituents selected from the substituent group $\beta_1$, a heteroaryl group which may be substituted with one or more substituents selected from the substituent group $\beta_1$, a heterocyclyl group which may be substituted with one or more substituents selected from the substituent group $\beta_1$, and an oxo group.

Substituent group $\beta_1$: A halogen atom, a hydroxyl group which may be protected, an amino group which may be protected, a carboxyl group which may be protected, a cyano group, a carbamoyl group which may be substituted with one or more substituents selected from a substituent group $\gamma_1$, a $C_{1-6}$ alkyl group which may be substituted with one or more substituents selected from the substituent group $\gamma_1$, a $C_{3-8}$ cycloalkyl group which may be substituted with one or more substituents selected from the substituent group $\gamma_1$, an aryl group which may be substituted with one or more substituents selected from the substituent group $\gamma_1$, a $C_{1-6}$ alkoxy group which may be substituted with one or more substituents selected from the substituent group $\gamma_1$, a $C_{1-6}$ alkylamino group which may be substituted with one or more substituents selected from the substituent group $\gamma_1$, a di($C_{1-6}$ alkyl)amino group which may be substituted with one or more substituents selected from the substituent group $\gamma_1$, a $C_{1-6}$ alkylsulfonyl group which may be substituted with one or more substituents selected from the substituent group $\gamma_1$, an arylsulfonyl group which may be substituted with one or more substituents selected from the substituent group $\gamma_1$, an acyl group which may be substituted with one or more substituents selected from the substituent group $\gamma_1$, a heteroaryl group which may be substituted with one or more substituents selected from the substituent group $\gamma_1$, a heterocyclyl group which may be substituted with one or more substituents selected from the substituent group $\gamma_1$, and an oxo group.

Substituent group $\gamma_1$: A halogen atom, a hydroxyl group which may be protected, an amino group which may be protected, a carboxyl group which may be protected, a cyano group, a carbamoyl group which may be substituted with a $C_{1-6}$ alkyl group, a $C_{1-6}$ alkyl group which may be substituted with a halogen atom, a $C_{1-6}$ alkoxy group which may be substituted with a halogen atom, and an oxo group.

Substituent group $\alpha_2$: A halogen atom, a hydroxyl group which may be protected, an amino group which may be protected, a carboxyl group which may be protected, a cyano group, a carbamoyl group which may be substituted with one or more substituents selected from the substituent group $\beta_2$, a $C_{1-6}$ alkyl group which may be substituted with one or more substituents selected from the substituent group $\beta_2$, a $C_{1-6}$ alkoxy group which may be substituted with one or more substituents selected from the substituent group $\beta_2$, and an oxo group.

Substituent group $\beta_2$: A halogen atom, a $C_{1-6}$ alkyl group which may be substituted with one or more substituents selected from the substituent group $\gamma_2$, a $C_{3-8}$ cycloalkyl group which may be substituted with one or more substituents selected from the substituent group $\gamma_2$, an aryl group which may be substituted with one or more substituents selected from the substituent group $\gamma_2$, a heteroaryl group which may be substituted with one or more substituents selected from the substituent group $\gamma_2$, and a heterocyclyl group which may be substituted with one or more substituents selected from the substituent group $\gamma_2$.

Substituent group $\gamma_2$: A halogen atom, a hydroxyl group which may be protected, an amino group which may be protected, a carboxyl group which may be protected, a cyano group, a $C_{1-6}$ alkyl group which may be substituted with a halogen atom, a $C_{1-6}$ alkoxy group which may be substituted with a halogen atom, and an oxo group.

Substituent group $A_3$: A halogen atom, a hydroxyl group which may be protected, an amino group which may be protected, an amino group which may be substituted with one or more substituents selected from a substituent group $E_3$, a carboxyl group which may be protected, a cyano group, a carbamoyl group which may be substituted with one or more substituents selected from a substituent group $B_3$, a sulfamoyl group which may be substituted with one or more substituents selected from the substituent group $B_3$, a $C_{1-6}$ alkyl group which may be substituted with one or more substituents selected from the substituent group $B_3$, a $C_{2-6}$ alkenyl group which may be substituted with one or more substituents selected from the substituent group $B_3$, a $C_{3-8}$ cycloalkyl group which may be substituted with one or more substituents selected from the substituent group $B_3$, an aryl group which may be substituted with one or more substituents selected from the substituent group $B_3$, a $C_{1-6}$ alkoxy group which may be substituted with one or more substituents selected from the substituent group $B_3$, an aryloxy group which may be substituted with one or more substituents selected from the substituent group $B_3$, a $C_{1-6}$ alkylthio group which may be substituted with one or more substituents selected from the substituent group $B_3$, an arylthio group which may be substituted with one or more substituents selected from the substituent group $B_3$, a $C_{1-6}$ alkyl amino group which may be substituted with one or more substituents selected from the substituent group $B_3$, a di($C_{1-6}$ alkyl)amino group which may be substituted with one or more substituents selected from the substituent group $B_3$, a $C_{1-6}$ alkylsulfonyl group which may be substituted with one or more substituents selected from the substituent group $B_3$, an arylsulfonyl group which may be substituted with one or more substituents selected from the substituent group $B_3$, an acyl group which may be substituted with one or more substituents selected from the substituent group $B_3$, a heteroaryl group which may be substituted with one or more substituents selected from the substituent group $B_3$, a heterocyclyl group which may be substituted with one or more substituents selected from the substituent group $B_3$, a heterocyclyloxy group which may be substituted with one or more substituents selected from the substituent group $B_3$, and an oxo group.

Substituent group $B_3$: A halogen atom, a hydroxyl group which may be protected, an amino group which may

be protected, a carboxyl group which may be protected, a cyano group, a carbamoyl group which may be substituted with one or more substituents selected from a substituent group $C_3$, a $C_{1-6}$ alkyl group which may be substituted with one or more substituents selected from the substituent group $C_3$, a $C_{3-8}$ cycloalkyl group which may be substituted with one or more substituents selected from the substituent group $C_3$, an aryl group which may be substituted with one or more substituents selected from the substituent group $C_3$, a $C_{1-6}$ alkoxy group which may be substituted with one or more substituents selected from the substituent group $C_3$, an aryloxy group which may be substituted with one or more substituents selected from the substituent group $C_3$, a $C_{1-6}$ alkylamino group which may be substituted with one or more substituents selected from the substituent group $C_3$, a di($C_{1-6}$ alkyl)amino group which may be substituted with one or more substituents selected from the substituent group $C_3$, a $C_{1-6}$ alkylsulfonyl group which may be substituted with one or more substituents selected from the substituent group $C_3$, an arylsulfonyl group which may be substituted with one or more substituents selected from the substituent group $C_3$, an acyl group which may be substituted with one or more substituents selected from the substituent group $C_3$, a heteroaryl group which may be substituted with one or more substituents selected from the substituent group $C_3$, a heterocyclyl group which may be substituted with one or more substituents selected from the substituent group $C_3$, a heteroaryloxy group which may be substituted with one or more substituents selected from the substituent group $C_3$, a heterocyclyloxy group which may be substituted with one or more substituents selected from the substituent group $C_3$, and an oxo group.

Substituent group $C_3$: A halogen atom, a hydroxyl group which may be protected, an amino group which may be protected, a carboxyl group which may be protected, a nitro group, cyano group, a carbamoyl group which may be substituted with one or more substituents selected from a substituent group $D_3$, a $C_{1-6}$ alkyl group which may be substituted with one or more substituents selected from the substituent group $D_3$, a $C_{3-8}$ cycloalkyl group which may be substituted with one or more substituents selected from the substituent group $D_3$, an aryl group which may be substituted with one or more substituents selected from the substituent group $D_3$, a $C_{1-6}$ alkoxy group which may be substituted with one or more substituents selected from the substituent group $D_3$, an aryloxy group which may be substituted with one or more substituents selected from the substituent group $D_3$, a $C_{1-6}$ alkylamino group which may be substituted with one or more substituents selected from the substituent group $D_3$, a di($C_{1-6}$ alkyl)amino group which may be substituted with one or more substituents selected from the substituent group $D_3$, a $C_{1-6}$ alkylsulfonyl group which may be substituted with one or more substituents selected from the substituent group $D_3$, an acyl group which may be substituted with one or more substituents selected from the substituent group $D_3$, a heteroaryl group which may be substituted with one or more substituents selected from the substituent group $D_3$, a heterocyclyl group which may be substituted with one or more substituents selected from the substituent group $D_3$, a silyl group which may be substituted with one or more substituents selected from the substituent group $D_3$, and an oxo group.

Substituent group $D_3$: A halogen atom, a hydroxyl group which may be protected, a $C_{1-6}$ alkyl group, a $C_{3-8}$ cycloalkyl group, an aryl group, a $C_{1-6}$ alkoxy group, a $C_{1-6}$ alkylamino group, a di($C_{1-6}$ alkyl)amino group, a $C_{1-6}$ alkylsulfonyl group, a heteroaryl group, a heterocyclyl group, and an oxo group.

Substituent group $E_3$: A $C_{3-8}$ cycloalkyl group which may be substituted with one or more substituents selected from a substituent group $F_3$, an aryl group which may be substituted with one or more substituents selected from the substituent group $F_3$, a heteroaryl group which may be substituted with one or more substituents selected from the substituent group $F_3$, and a heterocyclyl group which may be substituted with one or more substituents selected from the substituent group $F_3$.

Substituent group $F_3$: A halogen atom, a carboxyl group which may be protected, and a $C_{1-6}$ alkyl group.

Substituent group $\alpha_3$: A halogen atom, a hydroxyl group which may be protected, an amino group which may be protected, a carboxyl group which may be protected, a cyano group, a carbamoyl group which may be substituted with one or more substituents selected from a substituent group $\beta_3$, a $C_{1-6}$ alkyl group which may be substituted with one or more substituents selected from the substituent group $\beta_3$, a $C_{3-8}$ cycloalkyl group which may be substituted with one or more substituents selected from the substituent group $\beta_3$, an aryl group which may be substituted with one or more substituents selected from the substituent group $\beta_3$, a $C_{1-6}$ alkoxy group which may be substituted with one or more substituents selected from the substituent group $\beta_3$, an aryloxy group which may be substituted with one or more substituents selected from the substituent group $\beta_3$, a $C_{1-6}$ alkylthio group which may be substituted with one or more substituents selected from the substituent group $\beta_3$, an arylthio group which may be substituted with one or more substituents selected from the substituent group $\beta_3$, a $C_{1-6}$ alkylamino group which may be substituted with one or more substituents selected from the substituent group $\beta_3$, a di($C_{1-6}$ alkyl)amino group which may be substituted with one or more substituents selected from the substituent group $\beta_3$, a $C_{1-6}$ alkylsulfonyl group which may be substituted with one or more substituents selected from the substituent group $\beta_3$, an arylsulfonyl group which may be substituted with one or more substituents selected from the substituent group $\beta_3$, an acyl group which may be substituted with one or more substituents selected from the substituent group $\beta_3$, a heteroaryl group which may be substituted with one or more substituents selected from the substituent group $\beta_3$, a heterocyclyl group which may be substituted with one or more substituents selected from the substituent group $\beta_3$, a heterocyclyloxy group which may be substituted with one or more substituents selected from the substituent group $\beta_3$, and an oxo group.

Substituent group $\beta_3$: A halogen atom, a hydroxyl group which may be protected, an amino group which may be protected, a carboxyl group which may be protected, a cyano group, a carbamoyl group which may be substituted with one or more substituents selected from a substituent group $\gamma_3$, a $C_{1-6}$ alkyl group which may be substituted with one or more substituents selected from the substituent group $\gamma_3$, a $C_{3-8}$ cycloalkyl group which may be substituted with one or more substituents selected from the substituent group $\gamma_3$, an aryl group which may be substituted with one or more substituents selected from the substituent group $\gamma_3$, a $C_{1-6}$ alkoxy group which may be substituted with one or more substituents selected from the substituent group $\gamma_3$, a $C_{1-6}$ alkylamino group which may be substituted with one or more substituents selected from the substituent group $\gamma_3$, a di($C_{1-6}$ alkyl)amino group which may be substituted with one or more substituents selected from the substituent group $\gamma_3$, a $C_{1-6}$ alkylsulfonyl group which may be substituted with one or more substituents selected from the substituent group $\gamma_3$, an arylsulfonyl group which may be substituted with one or more substituents selected from the substituent group $\gamma_3$, a heteroaryl group which may be substituted with one or more substituents selected from the substituent group $\gamma_3$, a heterocyclyl group which may be substituted with one or more substituents selected from the substituent group $\gamma_3$, a heterocyclyloxy group which may be substituted with one or more substituents selected from the substituent group $\gamma_3$, and an oxo group.

Substituent group $\gamma_3$: A halogen atom, a hydroxyl group which may be protected, an amino group which may be protected, a carboxyl group which may be protected, a cyano group, a $C_{1-6}$ alkyl group which may be substituted with a halogen atom, a $C_{1-6}$ alkoxy group which may be substituted with a halogen atom, and an oxo group.

As the compound represented by Formula [1], the following compound is preferable.

$X^1$ is $NR^9$, whereinin the formula, $R^9$ represents a hydrogen atom.

$Z^1$ is $CR^6$ (in the formula, $R^6$ has the same meaning as that described above), preferably $CR^{6a}$ (in the formula, $R^{6a}$ represents an aryl group which may be substituted, a heteroaryl group which may be substituted, a heterocyclyl group which may be substituted, or $NHR^{8a}$ (in the formula, $R^{8a}$ represents an aryl group which may be substituted, a heteroaryl group which may be substituted, or a heterocyclyl group which may be substituted)), more preferably $CR^{6b}$ (in the formula, $R^{6b}$ represents an aryl group which may be substituted with one or more substituents selected from a substituent group $\alpha_2$, a heteroaryl group which may be substituted with one or more substituents selected from a substituent group $\alpha_2$, a heterocyclyl group which may be substituted with one or more substituents selected from a substituent group $\alpha_2$, or $NHR^{8b}$ (in the formula, $R^{8b}$ represents an aryl group which may be substituted with one or more substituents selected from a substituent group $\alpha_2$, a heteroaryl group which may be substituted with one or more substituents selected from a substituent group $\alpha_2$, or a heterocyclyl group which may be substituted with one or more substituents selected from a substituent group $\alpha_2$)), and most preferably $CR^{6c}$ (in the formula, $R^{6c}$ represents a phenyl group which may be substituted with one or more substituents selected from a substituent group $\alpha_2$, a pyrazolyl group which may be substituted with one or more substituents selected from a substituent group $\alpha_2$, a pyridyl group which may be substituted with one or more substituents selected from a substituent group $\alpha_2$, a pyrimidinyl group which may be substituted with one or more substituents selected from a substituent group $\alpha_2$, a pyrazinyl group which may be substituted with one or more substituents selected from a substituent group $\alpha_2$, a pyridazinyl group which may be substituted with one or more substituents selected from a substituent group $\alpha_2$, a morpholinyl group which may be substituted with one or more substituents selected from a substituent group $\alpha_2$, or $NHR^{8c}$ (in the formula, $R^{8c}$ represents a phenyl group which may be substituted with one or more substituents selected from a substituent group $\alpha_2$, a pyridyl group which may be substituted with one or more substituents selected from a substituent group $\alpha_2$, a pyrimidinyl group which may be substituted with one or more substituents selected from a substituent group $\alpha_2$, a pyrazinyl group which may be substituted with one or more substituents selected from a substituent group $\alpha_2$, a pyridazinyl group which may be substituted with one or more substituents selected from a substituent group $\alpha_2$)).

$R^1$ is a monocyclic, nitrogen-containing heteroaryl group which may be substituted, a monocyclic, nitrogen- and oxygen- containing heteroaryl group which may be substituted, a monocyclic, nitrogen- and sulfur- containing heteroaryl group which may be substituted, a bicyclic, nitrogen-containing heteroaryl group which may be substituted, a bicyclic, nitrogen- and oxygen-containing heteroaryl group which may be substituted, or a bicyclic, nitrogen- and sulfur-containing heteroaryl group which may be substituted.

$R^1$ is preferably a pyrazolyl group which may be substituted, an imidazolyl group which may be substituted, a triazolyl group which may be substituted, a thiazolyl group which may be substituted, an oxadiazolyl group which may be substituted, a thiadiazolyl group which may be substituted, a pyridyl group which may be substituted, or a pyridazinyl group which may be substituted, more preferably a pyrazolyl group which may be substituted with one or more substituents selected from a substituent group $A_1$, an imidazolyl group which may be substituted with one or more substituents selected from the substituent group $A_1$, a triazolyl group which may be substituted with one or more substituents selected from the substituent group $A_1$, a thiazolyl group which may be substituted with one or more substituents selected from the substituent group $A_1$, an oxadiazolyl group which may be substituted with one or more substituents selected from the substituent group $A_1$, a thiadiazolyl group which may be substituted with one or more substituents selected from the substituent group $A_1$, a pyridyl group which may be substituted with one or more substituents selected from the substituent group $A_1$, or a pyridazinyl group which may be substituted with one or more substituents selected from the substituent

group $A_1$, still more preferably a pyrazolyl group which may be substituted with one or more substituents selected from the substituent group $\alpha_1$, an imidazolyl group which may be substituted with one or more substituents selected from the substituent group $\alpha_1$, a triazolyl group which may be substituted with one or more substituents selected from the substituent group $\alpha_1$, a thiazolyl group which may be substituted with one or more substituents selected from the substituent group $\alpha_1$, an oxadiazolyl group which may be substituted with one or more substituents selected from the substituent group $\alpha_1$, a thiadiazolyl group which may be substituted with one or more substituents selected from the substituent group $\alpha_1$, a pyridyl group which may be substituted with one or more substituents selected from the substituent group $\alpha_1$, or a pyridazinyl group which may be substituted with one or more substituents selected from the substituent group $\alpha_1$, and most preferably a thiadiazolyl group which may be substituted with one or more substituents selected from the substituent group $\alpha_1$ or a pyridazinyl group which may be substituted with one or more substituents selected from the substituent group $\alpha_1$.

$R^2$ is a hydrogen atom.

$R^3$ is a hydrogen atom.

$R^4$ is a hydrogen atom.

$R^5$ is a heteroaryl group which may be substituted, a heteroaryloxy group which may be substituted, a heteroarylthio group which may be substituted, a heterocyclyl group which may be substituted, or $NR^{12}R^{13}$ (in the formula, each of $R^{12}$ and $R^{13}$ has the same meaning as that described above).

As the compound represented by Formula [1], the compound represented by Formula [1a] is preferable.

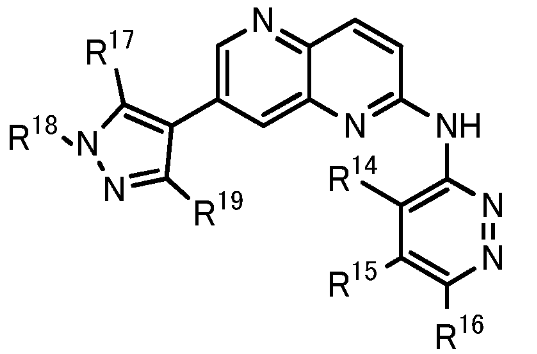

[1a]

(In the formula, each of $R^{14}$, $R^{15}$, $R^{16}$, $R^{17}$, $R^{18}$, and $R^{19}$ has the same meaning as that described above.)

More preferably, each of $R^{14}$ and $R^{16}$ is a hydrogen atom, and $R^{17}$ represents a hydrogen atom in the compound.

Examples of a salt of the compound represented by Formula [1] include salts of a basic group such as an amino group or an acidic group such as a phenolic hydroxyl group or a carboxyl group, which are typically known.

Examples of the salt of the basic group include a salt of a mineral acid such as hydrochloric acid, hydrogen bromide, or sulfuric acid, a salt of an organic carboxylic acid such as tartaric acid, formic acid, acetic acid, citric acid, trichloroacetic acid, or trifluoroacetic acid, and a salt of a sulfonic acid such as methanesulfonic acid, benzenesulfonic acid, p-toluenesulfonic acid, mesitylenesulfonic acid, or naphthalene sulfonic acid.

Examples of the salt of the acidic group include a salt of an alkali metal such as sodium or potassium; a salt of an alkali earth metal such as calcium or magnesium; an ammonium salt; and a salt of a nitrogen-containing organic base such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, or N,N'-dibenzylethylenediamine.

Furthermore, among the above-described salts, preferable examples of the compound represented by Formula[1] include pharmacologically acceptable salts.

In a case in which an isomer (for example, a tautomer, an optical isomes, or a geometric isomer) of the compound represented by Formula [1] or salt thereof is present, the invention includes the isomer, and further includes a solvate, a hydrate, and various shapes of crystal of the compound represented by Formula [1] or salt thereof.

Furthermore, the present disclosure includes a prodrug of the compound represented by Formula [1].

The prodrug of the compound represented by Formula [1] may have characteristics such as the followings.

(1) The prodrug of the compound represented by Formula [1] may have excellent inhibitory activity with respect to the PI3K-AKT pathway and/or the Ras-Raf-MEK-ERK pathway, while this is not necessary.

(2) The prodrug of the compound represented by Formula [1] is converted to the compound represented by Formula [1] by cleavage of a functional group that functions as a prodrug by an enzyme in a body after administration. In this case, the compound represented by Formula [1] and the prodrug thereof may be coexsist in a mixed manner.

(3) To the prodrug of the compound represented by Formula [1], for example, enhancement of a drug efficacy action, duration of a drug efficacy action, reduction of side effects, reduction of toxicity and/or improvement of stability are expected.

Next, the method of preparing the compound of the invention will be described.

The compound of the invention is prepared by combining known methods, and for example, can be prepared according to the following preparation method.

[Preparation Method 1]

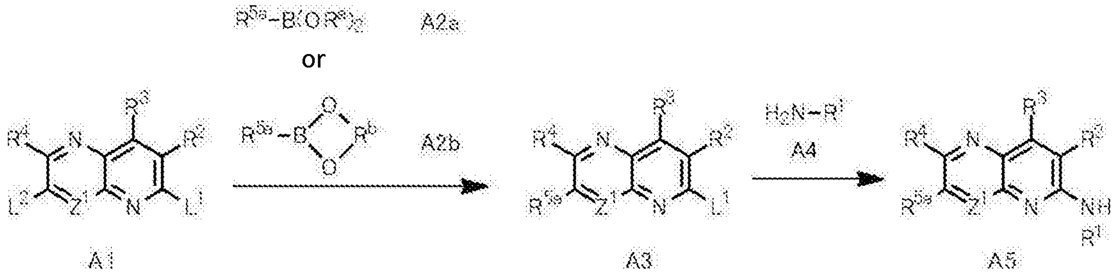

(In the formulae, $R^a$ represents a hydrogen atom or a $C_{1-6}$ alkyl group which may be substituted, $R^b$ represents a $C_{1-6}$ alkylene group which may be substituted, $R^{5a}$ represents an aryl group which may be substituted or a heteroaryl group which may be substituted, $L^1$ represents a leaving group, $L^2$ represents a leaving group, and each of $R^1$, $R^2$, $R^3$, $R^4$, and $Z^1$ has the same meaning as that described above.)

(1-1)

As the compound represented by Formula A2a, for example, pyridine-3-boronic acid, 3-(methanesulfonamide)phenylboronic acid, thiophene-2-boronic acid, benzofuran-2-boronic acid, and 3-methoxyphenylboronic acid are known.

As the compound represented by Formula A2b, for example, 1-(3-(pyrrolidin-1-yl)propyl)-4-(4,4,5,5-tetramethyl-1,3,2-dioxaborolan-2-yl)-1H-pyrazole, and 3-(4,4,5,5-tetramethyl-1,3,2-dioxaborolan-2-yl)furan are known.

The compound represented by Formula A2a and the compound represented by Formula A2b can be prepared from a corresponding halogeno material, for example, according to the method described in Japanese Patent Application Laid-Open (JP-A) No. 2003-206290, or "The Journal of Organic Chemistry", vol. 60, pp. 7508-7510, 1995.

The compound represented by Formula A3 can be prepared by reacting the compound represented by Formula A1 with the compound represented by Formula A2a or the compound represented by Formula A2b in the presence of a base, in the presence of a palladium catalyst, and in the presence or absence of a ligand.

A solvent used in the reaction is not particularly limited as long as the solvent does not adversely affect the reaction. Examples thereof include an aliphatic hydrocarbon, a halogenated hydrocarbon, an ether, a ketone, an ester, an alcohol, an amide, a sulfoxide, an aromatic hydrocarbon, acetonitrile, and water. These may be used in a mixed manner.

Examples of the base used in the reaction include an inorganic base such as sodium hydrogen carbonate, sodium carbonate, potassium carbonate, cesium carbonate, sodium hydroxide, potassium hydroxide, potassium acetate, or tripotassium phosphate, and an organic base such as 1,8-diazabicyclo[5.4.0]-7-undecene, triethylamine, or N,N-diisopropylethylamine.

An amount of the base used is from 1-fold by mole to 50-fold by mole, preferably from 1-fold by mole to 10-fold by mole, and more preferably from 2-fold by mole to 5-fold by mole, with respect to the compound represented by Formula A1.

An amount of the palladium catalyst used in the reaction is from 0.00001 -fold by mole to 1-fold by mole, and preferably from 0.001-fold by mole to 0.1-fold by mole, with respect to the compound represented by Formula A1.

An amount of the ligand, which is used in the reaction if desired, is from 0.00001-fold by mole to 1-fold by mole, and preferably from 0.001-fold by mole to 0.1-fold by mole, with respect to the compound represented by Formula A1.

An amount of the compound represented by Formula A2a or the compound represented by Formula A2b used is from 1-fold by mole to 50-fold by mole, and preferably from 1-fold by mole to 2-fold by mole, with respect to the compound represented by Formula A1.

Preferably, the reaction may be performed at a temperature of from room temperature to 250°C for from 10

minutes to 24 hours in an inert gas (for example, nitrogen or argon) atmosphere.

The reaction can also be performed using a tin reagent or a zinc reagent instead of the compound represented by Formula A2a or the compound represented by Formula A2b. The reaction may be performed, for example, according to the method described in "Organometallics in Synthesis)" written by M. Schlosser et al., 2nd edition, pp. 1123-1217, 2002 (John Wiley & Sons, INC.).

(1-2)

As the compound represented by Formula A4, for example, 2-aminothiazole, 2-aminopyridine, 3-aminopyridazine, or 2-aminothiadiazole is known.

The compound represented by Formula A5 can be prepared by reacting the compound represented by Formula A3 with the compound represented by Formula A4 in the presence of a base, in the presence of a palladium catalyst, and in the presence or absence of a ligand.

A solvent used in the reaction is not particularly limited as long as the solvent does not adversely affect the reaction. Examples thereof include an aliphatic hydrocarbon, a halogenated hydrocarbon, an ether, an ester, a sulfoxide, an aromatic hydrocarbon, and acetonitrile. These may be used in a mixed manner.

Examples of the base used in the reaction include an inorganic base such as sodium hydrogen carbonate, sodium carbonate, potassium carbonate, cesium carbonate, or tripotassium phosphate, and an organic base such as 1,8-diazabicyclo[5.4.0]-7-undecene, triethylamine, N,N-diisopropylethylamine, sodium tert-butoxide, potassium tert-butoxide, lithium bis(trimethylsilyl)amide, or lithium 2,2,6,6-tetramethylpiperidide.

An amount of the base used is from 1-fold by mole to 50-fold by mole, preferably from 1-fold by mole to 10-fold by mole, and more preferably from 2-fold by mole to 5-fold by mole, with respect to the compound represented by Formula A3.

An amount of the palladium catalyst used in the reaction is from 0.00001 -fold by mole to 1-fold by mole, and preferably from 0.001-fold by mole to 0.1-fold by mole, with respect to the compound represented by Formula A3.

An amount of the ligand, which is used in the reaction if desired, is from 0.00001-fold by mole to 1-fold by mole, and preferably from 0.001-fold by mole to 0.1-fold by mole, with respect to the compound represented by Formula A3.

An amount of the compound represented by Formula A4 used is from 1-fold by mole to 50-fold by mole, and preferably from 1-fold by mole to 2-fold by mole, with respect to the compound represented by Formula A3.

Preferably, the reaction may be performed at a temperature of from room temperature to 250°C for from 10 minutes to 24 hours in an inert gas (for example, nitrogen or argon) atmosphere.

[Preparation Method 2]

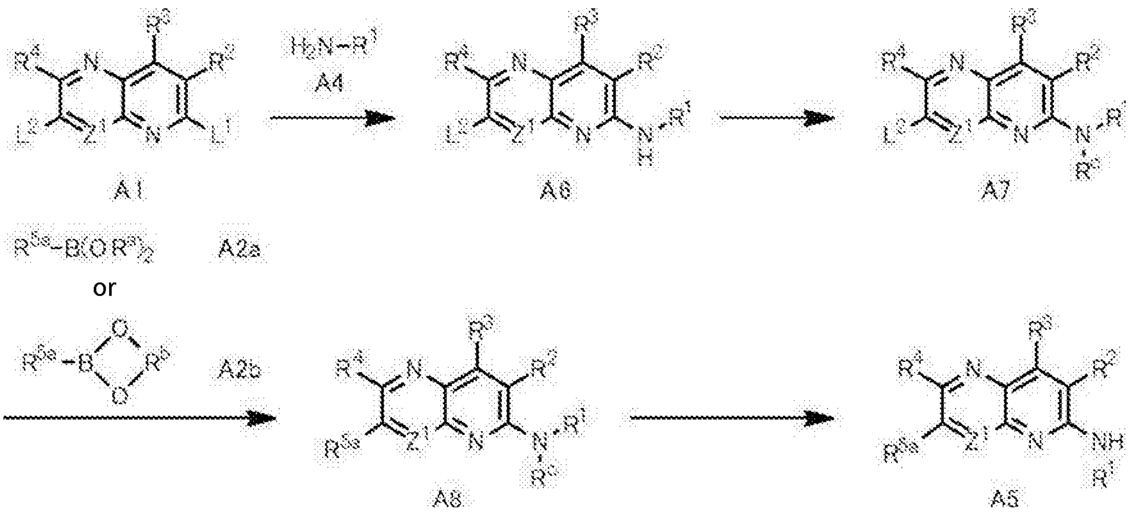


(In the formulae, $R^c$ represents an amino protecting group, and each of $R^a$, $R^b$, $R^1$, $R^2$, $R^3$, $R^4$, $R^{5a}$, $L^1$, $L^2$, and $Z^1$ has the same meaning as that described above.)

(2-1)

The compound represented by Formula A6 can be prepared by reacting the compound represented by Formula A1 with the compound represented by Formula A4 in the presence of a base or in the absence thereof, or in the presence of an acid or in the absence thereof.

A solvent used in the reaction is not particularly limited as long as the solvent does not adversely affect the reaction. Examples thereof include an aliphatic hydrocarbon, a halogenated hydrocarbon, an ether, an ester, a sulfoxide, an aromatic hydrocarbon, an amide, and acetonitrile. These may be used in a mixed manner.

Examples of the base, which is used in the reaction if desired, include an inorganic base such as sodium hydride, potassium carbonate, or cesium carbonate, and an organic base such as 1,8-diazabicyclo[5.4.0]-7-undecene, triethylamine, or N,N-diisopropylethylamine.

A amount of the base used is from 1 -fold by mole to 50-fold by mole, preferably from 1-fold by mole to 10-fold by mole, and more preferably from 2-fold by mole to 5-fold by mole, with respect to the compound represented by Formula A1.

Examples of the acid, which is used in the reaction if desired, include an inorganic acid such as hydrochloric acid or sulfuric acid, and an organic acid such as p-toluenesulfonic acid, acetic acid, or trifluoroacetic acid.

An amount of the acid used is from 0.001-fold by mole to 10-fold by mole with respect to the compound represented by Formula A1.

An amount of the compound represented by Formula A4 used is from 1-fold by mole to 50-fold by mole, and preferably from 1-fold by mole to 2-fold by mole, with respect to the compound represented by Formula A1.

Preferably, the reaction may be performed at a temperature of from room temperature to 250°C for from 10 minutes to 24 hours.

(2-2)

The compound represented by Formula A7 can be prepared by protecting the amino group of the compound represented by Formula 6 in the presence of a base.

The reaction may be performed, for example, according to the method described in "Protective Groups in Organic Synthesis" written by W. Greene et al., 4th edition, pp. 696-926, 2007 (John Wiley & Sons, INC.).

(2-3)

The compound represented by Formula A8 can be prepared by reacting the compound represented by Formula A7 with the compound represented by Formula A2a or the compound represented by Formula A2b in the presence of a base, in the presence of a palladium catalyst, and in the presence or absence of a ligand.

The reaction may be performed according to Preparation Method (1-1).

(2-4)

The compound represented by Formula A5 can be prepared by deprotecting the compound represented by Formula A8.

The reaction may be performed, for example, according to the method described in "Protective Groups in Organic Synthesis" written by W. Greene et al., 4th edition, pp. 696-926, 2007 (John Wiley & Sons, INC.).

[Preparation Method 3]

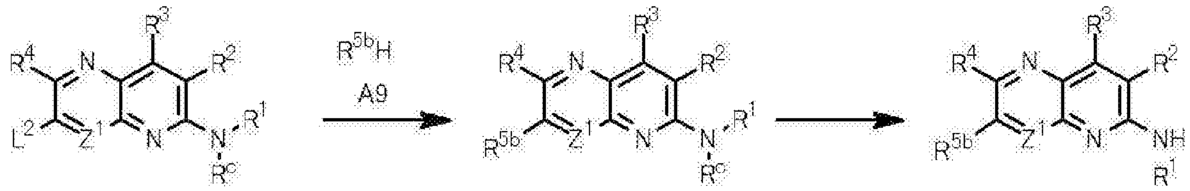


A7 A10 A11

(In the formulae, $R^{5b}$ represents a heterocyclyl group which may be substituted or $NR^{12a}R^{13a}$, in which each

of $R^{12a}$ and $R^{13a}$ has the same meaning as that described above, and each of $R^C$, $R^1$, $R^2$, $R^3$, $R^4$, $L^2$, and $Z^1$ has the same meaning as that described above.)

(3-1)

As the compound represented by Formula A9, for example, morpholine, 1-methyl-piperazine, 4-aminopyridine, or 4-methoxyaniline is known.

The compound represented by Formula A10 can be prepared by reacting the compound represented by Formula A7 with the compound represented by Formula A9 in the presence of a base, in the presence of a palladium catalyst, and in the presence or absence of a ligand.

The solvent used in the reaction is not particularly limited as long as the solvent does not adversely affect the reaction. Examples thereof include an aliphatic hydrocarbon, an ether, an ester, an aromatic hydrocarbon, and acetonitrile. These may be used in a mixed manner.

Examples of the base used in the reaction include an inorganic base such as sodium hydrogen carbonate, sodium carbonate, potassium carbonate, cesium carbonate, or tripotassium phosphate, and an organic base such as 1,8-diazabicyclo[5.4.0]-7-undecene, triethylamine, sodium tert-butoxide, potassium tert-butoxide, or N,N-diisopropylethylamine.

An amount of the base used is from 1-fold by mole to 50-fold by mole, preferably from 1-fold by mole to 10-fold by mole, and more preferably from 2-fold by mole to 5-fold by mole, with respect to the compound represented by Formula A7.

An amount of the palladium catalyst used in the reaction is from 0.00001 -fold by mole to 1-fold by mole, and preferably from 0.001-fold by mole to 0.1-fold by mole, with respect to the compound represented by Formula A7.

An amount of the ligand, which is used in the reaction if desired, is from 0.00001-fold by mole to 1-fold by mole, and preferably from 0.001-fold by mole to 0.1-fold by mole, with respect to the compound represented by Formula A7.

An amount of the compound represented by Formula A9 used is from 1 -fold by mole to 50-fold by mole, and preferably from 1-fold by mole to 2-fold by mole, with respect to the compound represented by Formula A7.

Preferably, the reaction may be performed at a temperature of from room temperature to 250°C for from 10 minutes to 24 hours in an inert gas (for example, nitrogen or argon) atmosphere.

(3-2)

The compound represented by Formula A11 can be prepared by deprotecting the compound represented by Formula A10.

The reaction may be performed according to Preparation Method (2-4).

[Preparation Method 4]

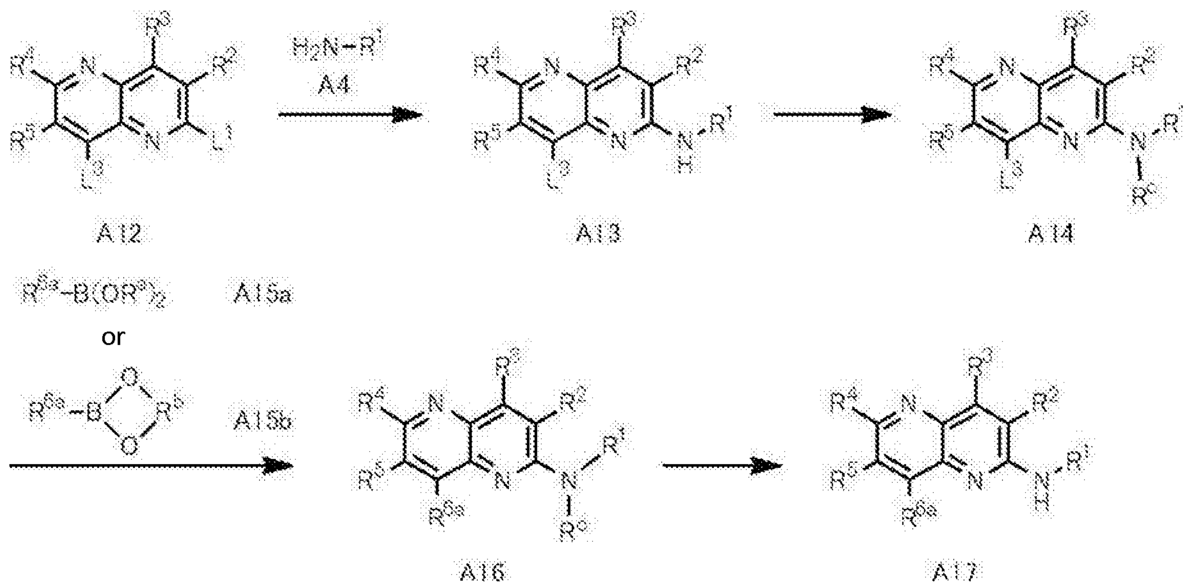


A12 A13 A14

or

A16 A17

(In the formulae, $R^{6a}$ represents an aryl group which may be substituted or a heteroaryl group which may be substituted, $L^3$ represents a leaving group, and each of $R^a$, $R^b$, $R^c$, $R^1$, $R^2$, $R^3$, $R^4$, $R^5$, and $L^1$ has the same meaning as that described above.)

(4-1)

The compound represented by Formula A13 can be prepared by reacting the compound represented by Formula A12 with the compound represented by Formula A4 in the presence of a base and a palladium catalyst.
The reaction may be performed according to Preparation Method (1-2).

(4-2)

The compound represented by Formula A14 can be prepared by protecting the amino group of the compound represented by Formula A13.
The reaction may be performed according to Preparation Method (2-2).

(4-3)

As the compound represented by Formula A15a, for example, 3-aminocarbonylphenylboronic acid, and 3-methoxypyridine-4-boronic acid are known.
As the compound represented by Formula A15b, for example, 5-(4,4,5,5-tetramethyl-1,3,2-dioxaborolan-2-yl)pyrimidine, and 1-(tert-butoxycarbonyl)-4-(4,4,5,5-tetramethyl-1,3,2-dioxaborolan-2-yl)pyrazole are known.
In addition, the compound represented by Formula A15a and the compound represented by Formula A15b can be prepared from a corresponding halogeno material, for example, according to the method described in JP-ANo. 2003-206290, or "The Journal of Organic Chemistry", vol. 60, pp. 7508-7510, 1995.
The compound represented by Formula A16 can be prepared by reacting the compound represented by Formula A14 with the compound represented by Formula A15a or the compound represented by Formula A15b in the presence of a base, in the presence of a palladium catalyst, and in the presence or absence of a ligand.
The reaction may be performed according to Preparation Method (1-1).

(4-4)

The compound represented by Formula A17 can be prepared by deprotecting the compound represented by Formula A16.
The reaction may be performed according to Preparation Method (2-4).

[Preparation Method 5]

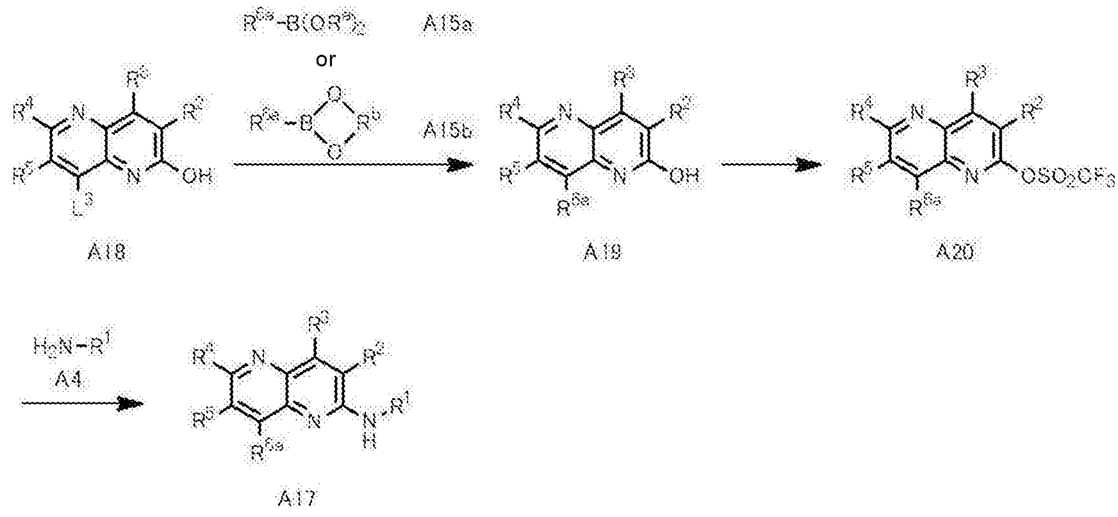


A18 A19 A20

A17

(In the formulae, each of $R^a$, $R^b$, $R^1$, $R^2$, $R^3$, $R^4$, $R^5$, $R^{6a}$, and $L^3$ has the same meaning as that described above.)

(5-1)

The compound represented by Formula A19 can be prepared by reacting the compound represented by Formula A18 with the compound represented by Formula A15a or the compound represented by Formula A15b in the presence of a base, in the presence of a palladium catalyst, and in the presence or absence of a ligand.

The reaction may be performed according to Preparation Method (1-1).

(5-2)

The compound represented by Formula A20 can be prepared by reacting the compound represented by Formula A19 with trifluoromethanesulfonic acid anhydride or N-phenyl-bis(trifluoromethanesulfonimide) in the presence of a base.

The solvent used in the reaction is not particularly limited as long as the solvent does not adversely affect the reaction. Examples thereof include an aliphatic hydrocarbon, a halogenated hydrocarbon, an ether, a ketone, an ester, an amide, a sulfoxide, and an aromatic hydrocarbon. These may be used in a mixed manner.

Examples of the base used in the reaction include an inorganic base such as potassium carbonate or sodium hydride, and an organic base such as pyridine 2,6-lutidine, triethylamine, or N,N-diisopropylethylamine.

An amount of the base used is from 1-fold by mole to 50-fold by mole, preferably from 1-fold by mole to 10-fold by mole, and more preferably from 2-fold by mole to 5-fold by mole, with respect to the compound represented by Formula A19.

An amount of trifluoromethanesulfonic acid anhydride or N-phenyl-bis(trifluoromethanesulfonimide) used in the reaction is from 1-fold by mole to 10-fold by mole, and preferably from 1-fold by mole to 2-fold by mole, with respect to the compound represented by Formula A19.

Preferably, the reaction may be performed at a temperature of from 0°C to 30°C for from 30 minutes to 24 hours in an inert gas (for example, nitrogen or argon) atmosphere.

(5-3)

The compound represented by Formula A17 can be prepared by reacting the compound represented by Formula A20 with the compound represented by Formula A4 in the presence of a base and a palladium catalyst.

The reaction may be performed according to Preparation Method (1-2).

[Preparation Method 6]

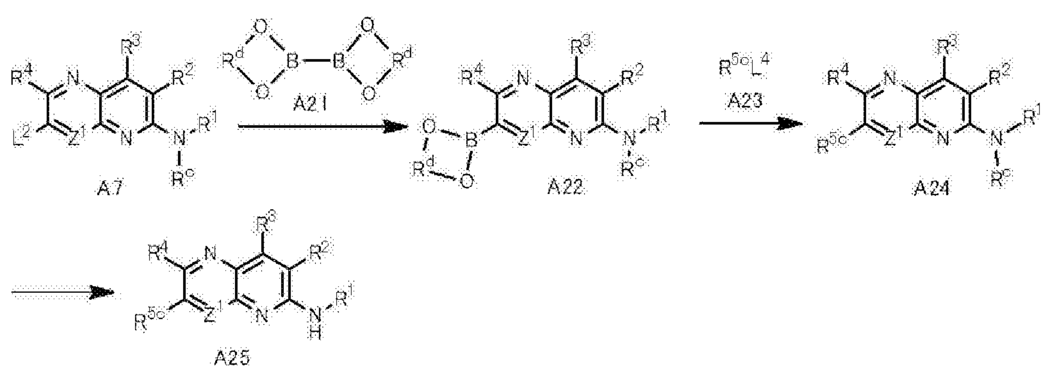

(In the formulae, $R^d$ represents a $C_{1-6}$ alkylene group which may be substituted; $R^{5c}$ represents an aryl group which may be substituted or a heteroaryl group which may be substituted; $L^4$ represents a leaving group; and each of $R^c$, $R^1$, $R^2$, $R^3$, $R^4$, $L^2$, and $Z^1$ has the same meaning as that described above.)

(6-1)

As the compound represented by Formula A21, for example, bis(pinacolato)diboron is known.

The compound represented by Formula A22 can be prepared by reacting the compound represented by Formula

A7 with the compound represented by Formula A21 in the presence of a base, in the presence of a palladium catalyst, and in the presence or absence of a ligand.

The reaction may be performed according to Preparation Method (1-1).

The compound represented by Formula A22 may be used in the following reaction as it is without being isolated.

(6-2)

As the compound represented by Formula A23, for example, 4-iodo-3-(2-methoxyethyl)-1-methyl-1H-pyrazole is known.

The compound represented by Formula A24 can be prepared by reacting the compound represented by Formula A22 with the compound represented by Formula A23 in the presence of a base, in the presence of a palladium catalyst, and in the presence or absence of a ligand.

The solvent used in the reaction is not particularly limited as long as the solvent does not adversely affect the reaction. Examples thereof include an aliphatic hydrocarbon, a halogenated hydrocarbon, an ether, a ketone, an ester, a sulfoxide, an aromatic hydrocarbon, acetonitrile, an alcohol, an amide, and water. These may be used in a mixed manner.

Examples of the base used in the reaction include an inorganic base such as sodium hydrogen carbonate, sodium carbonate, potassium carbonate, cesium carbonate, or tripotassium phosphate, and an organic base such as 1,8-diazabicyclo[5.4.0]-7-undecene, triethylamine, or N,N-diisopropylethylamine.

An amount of the base used is from 1-fold by mole to 50-fold by mole, preferably from 1-fold by mole to 10-fold by mole, and more preferably from 2-fold by mole to 5-fold by mole, with respect to the compound represented by Formula A22.

An amount of the palladium catalyst used in the reaction is from 0.00001 -fold by mole to 1-fold by mole, and preferably from 0.001-fold by mole to 0.1-fold by mole, with respect to the compound represented by Formula A22.

An amount of the ligand, which is used in the reaction if desired, is from 0.00001-fold by mole to 1-fold by mole, and preferably from 0.001-fold by mole to 0.1-fold by mole, with respect to the compound represented by Formula A22.

An amount of the compound represented by Formula A23 used is from 1-fold by mole to 5-fold by mole, and preferably from 1-fold by mole to 2-fold by mole, with respect to the compound represented by Formula A22.

Preferably, the reaction may be performed at a temperature of from room temperature to 250°C for from 1 hour to 24 hours in an inert gas (for example, nitrogen or argon) atmosphere.

(6-3)

The compound represented by Formula A25 can be prepared by deprotecting the compound represented by Formula A24.

The reaction may be performed according to Preparation Method (2-4).

[Preparation Method 7]

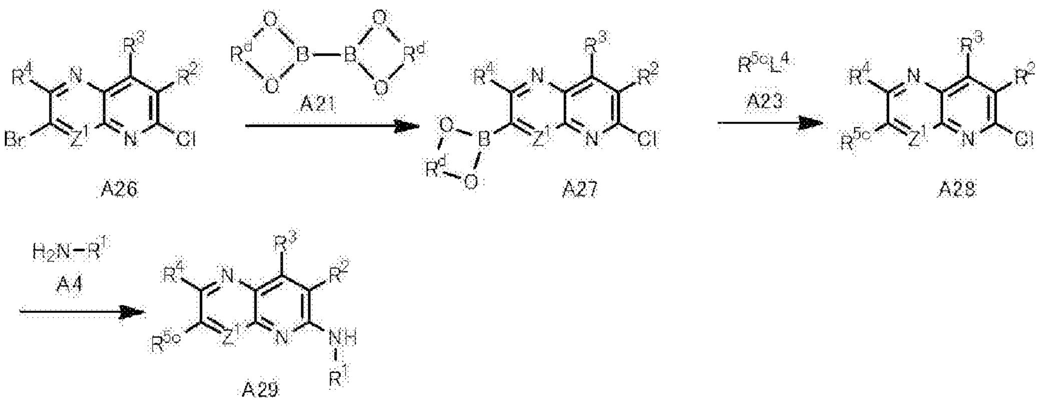


A26 A27 A28 A29

(In the formulae, each of $R^d$, $R^1$, $R^2$, $R^3$, $R^4$, $R^{5c}$, $L^4$, and $Z^1$ has the same meaning as that described above.)

(7-1)

The compound represented by Formula A27 can be prepared by reacting the compound represented by Formula A26 with the compound represented by Formula A21 in the presence of a base, in the presence of a palladium catalyst, and in the presence or absence of a ligand.

The reaction may be performed according to Preparation Method (6-1).

(7-2)

The compound represented by Formula A28 can be prepared by reacting the compound represented by Formula A27 with the compound represented by Formula A23 in the presence of a base, in the presence of a palladium catalyst, and in the presence or absence of a ligand.

The reaction may be performed according to Preparation Method (6-2).

(7-3)

The compound represented by Formula A29 can be prepared by reacting the compound represented by Formula A28 with the compound represented by Formula A4 in the presence of a base and a palladium catalyst.

The reaction may be performed according to Preparation Method (1-2).

Next, methods for preparing a compound which is a raw material for the compound of the invention will be described.

[Preparation Method A]

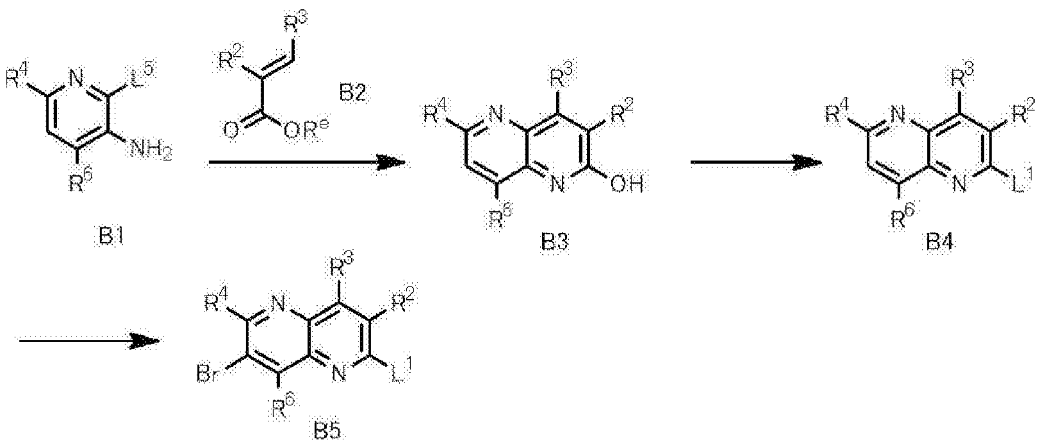

B1 B2 B3 B4 B5

(In the formulae, $R^e$ represents a $C_{1-6}$ alkyl group, $L^5$ represents a leaving group,; and each of $R^2$, $R^3$, $R^4$, $R^6$, and $L^1$ has the same meaning as that described above.)

(A-1)

As the compound represented by Formula B1, for example, 2-chloro-3-aminopyridine is known.

As the compound represented by Formula B2, for example, butyl acrylate, methyl acrylate, ethyl acrylate, and tert-butyl acrylate is known.

The compound represented by Formula B3 can be prepared by reacting the compound represented by Formula B1 with the compound represented by Formula B2 in the presence of a base, in the presence of a palladium catalyst, and in the presence or absence of a ligand.

The solvent used in the reaction is not particularly limited as long as the solvent does not adversely affect the reaction. Examples thereof include an aliphatic hydrocarbon, a halogenated hydrocarbon, an alcohol, an ether, a ketone, an ester, an amide, a sulfoxide, an aromatic hydrocarbon, and water. These may be used in a mixed manner.

Preferable examples of the solvent include an ester, and a more preferable example thereof is cyclohexyl acetate.

Examples of the base used in the reaction include an inorganic base such as sodium hydrogen carbonate, sodium carbonate, potassium carbonate, cesium carbonate, or tripotassium phosphate, and an organic base such as pyridine, 4-(dimethylamino)pyridine, triethylamine, N,N-diisopropylethylamine, or sodium tert-butoxide.
Preferable examples of the base include an organic base, and a more preferable example thereof is triethylamine.
An amount of the base used is from 1-fold by mole to 50-fold by mole, preferably from 1-fold by mole to 10-fold by mole, and more preferably from 1-fold by mole to 4-fold by mole,with respect to the compound represented by Formula B1.
Preferable examples of the palladium catalyst used in the reaction include an organic palladium complex, and a more preferable example thereof is bis(tri-tert-butylphosphine)palladium(0).
An amount of the palladium catalyst used is from 0.001-fold by mole to 1-fold by mole, preferably from 0.002-fold by mole to 0.5-fold by mole, and more preferably from 0.005-fold by mole to 0.1-fold by mole, with respect to the compound represented by Formula B1.
An amount of the ligand, which is used in the reaction if desired, is from 0.00001-fold by mole to 1-fold by mole, preferably from 0.0001-fold by mole to 0.5-fold by mole, and more preferably from 0.001-fold by mole to 0.5-fold by mole, with respect to the compound represented by Formula B1.
An amount of the compound represent by Formula B2 used is from 1-fold by mole to 10-fold by mole, preferably from 1-fold by mole to 5-fold by mole, and more preferably from 1 -fold by mole to 2-fold by mole, with respect to the compound represented by Formula B1.
Preferably, the reaction may be performed at a temperature of from room temperature to 180°C for from 30 minutes to 96 hours in an inert gas (for example, nitrogen or argon) atmosphere.

(A-2)

The compound represented by Formula B4 can be prepared by reacting the compound represented by Formula B3 with a halogenating agent, sulfonic acid anhydride, or a sulfonic halide.
The solvent used in the reaction is not particularly limited as long as the solvent does not adversely affect the reaction. Examples thereof include an aliphatic hydrocarbon, a halogenated hydrocarbon, and an aromatic hydrocarbon. These may be used in a mixed manner.
A halogenating agent or sulfonic acid anhydride may be used as the solvent.
Examples of the halogenating agent used in the reaction include phosphorus oxychloride, thionyl chloride, phosphorus tribromide, and thionyl bromide.
Preferable examples of the halogenating agent include phosphorus oxychloride and thionyl chloride, and a more preferable example thereof is phosphorus oxychloride.
An amount of the halogenating agent used is from 1-fold by mole to 10-fold by mole, preferably from 2-fold by mole to 8-fold by mole, and more preferably from 4-fold by mole to 6-fold by mole, with respect to the compound represented by Formula B3.
Examples of the sulfonic acid anhydride used in the reaction include trifluoromethanesulfonic acid anhydride.
Examples of the sulfonic halide used in the reaction include trifluoromethanesulfonyl chloride.
In the case of using the sulfonic halide, the reaction is preferably performed in the presence of a base.
Examples of the base used in the reaction include an organic base such as pyridine, 4-(dimethylamino)pyridine, triethylamine, or N,N-diisopropylethylamine.
An amount of the base used is from 1-fold by mole to 50-fold by mole, preferably from 1-fold by mole to 10-fold by mole, and more preferably from 1-fold by mole to 4-fold by mole, with respect to the compound represented by Formula B3.
In the reaction, the halogenating agent is preferably used as the solvent, and more preferably, phosphorus oxychloride is used as the solvent.
The reaction may be performed at a temperature of from room temperature to the boiling temperature of the solvent, that is preferably at the boiling temperature of the solvent, for from 30 minutes to 24 hours.

(A-3)

The compound represented by Formula B5 can be prepared by reacting the compound represented by Formula B4 with a brominating agent in the presence or absence of a base.
A solvent used in the reaction is not particularly limited as long as the solvent does not adversely affect the reaction. Examples thereof include an aliphatic hydrocarbon, a halogenated hydrocarbon, a carboxylic acid, and water. These may be used in a mixed manner.
Preferable examples of the solvent include a carboxylic acid, and a more preferable example thereof is acetic

acid.

Examples of the brominating agent used in the reaction include bromine and thionyl bromide, and a more preferable example thereof is bromine.

An amount of the brominating agent used in the reaction is from 1-fold by mole to 2-fold by mole, and preferably from 1.0-fold by mole to 1.2-fold by mole, with respect to the compound represented by Formula B4.

Examples of the base, which is used in the reaction if desired, include preferably sodium acetate and potassium acetate, and a more preferable example thereof is sodium acetate.

An amount of the base used is from 1-fold by mole to 5-fold by mole, preferably from 1-fold by mole to 2-fold by mole, and more preferably from 1.0-fold by mole to 1.2-fold by mole, with respect to the compound represented by Formula B4.

The reaction may be performed at a temperature of from 60°C to 120°C, that is preferably at a temperature of from 80°C to 100°C, for from 30 minutes to 24 hours.

[Preparation Method B]

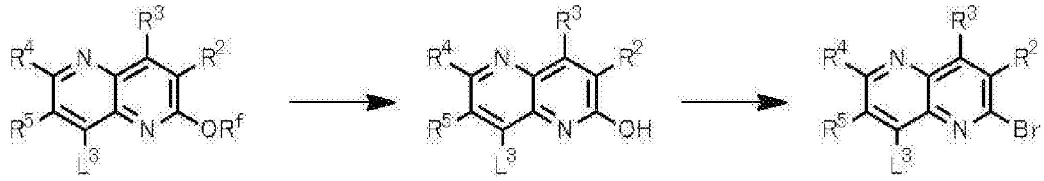

B6 B7 B8

(In the formulae, $R^f$ represents a $C_{1-6}$ alkyl group, and each of $R^2$, $R^3$, $R^4$, $R^5$, and $L^3$ has the same meaning as that described above.)

(B-1)

As the compound represented by Formula B6, for example, 6-methoxy-1,5-naphthyridin-4(1H)-one is known.

The compound represented by Formula B7 can be prepared by reacting the compound represented by Formula B6 with an acid.

A solvent used in the reaction is not particularly limited as long as the solvent does not adversely affect the reaction. Examples thereof include an aliphatic hydrocarbon, a halogenated hydrocarbon, an alcohol, an ether, a ketone, an ester, an amide, a sulfoxide, an aromatic hydrocarbon, and water. These may be used in a mixed manner.

Examples of the acid used in the reaction include an inorganic acid such as hydrobromic acid, hydrochloric acid, sulfuric acid, nitric acid, or phosphoric acid, and an organic acid such as acetic acid or trifluoroacetic acid.

An amount of the acid used is from 0.001-fold by mole to 50-fold by mole with respect to the compound represented by Formula B6.

Preferably, the reaction may be performed at a temperature of from 50°C to 180°C for from 10 minutes to 24 hours.

(B-2)

The compound represented by Formula B8 can be prepared by reacting the compound represented by Formula B7 with a brominating agent.

A solvent used in the reaction is not particularly limited as long as the solvent does not adversely affect the reaction. Examples thereof include an aliphatic hydrocarbon, a halogenated hydrocarbon, and an aromatic hydrocarbon. These may be used in a mixed manner.

Examples of the brominating agent used in the reaction include phosphorous oxybromide and phosphorus tribromide, and a more preferable example thereof is phosphorous oxybromide.

An amount of the brominating agent used in the reaction is from 1-fold by mole to 2-fold by mole, and preferably from 1.0-fold by mole to 1.2-fold by mole, with respect to the compound represented by Formula B7.

Preferably, the reaction may be performed at a temperature of from 50°C to 180°C for from 10 minutes to 24 hours in an inert gas (for example, nitrogen or argon) atmosphere.

The compounds obtained in the preparation methods described above can be derived to other compounds by

a known reaction such as condensation, addition, oxidation, reduction, rearrangement, substitution, halogenation, dehydration, or hydrolysis, or by appropriately combining these reactions.

In a case in which an amino group, a hydroxyl group, or a carboxyl group is present in the compounds obtained by the preparation methods described above, the reaction can be performed after appropriately changing the protecting group thereof. In addition, in a case in which two or more protecting groups are present, the protecting groups can be selectively deprotected by a known reaction.

In a case in which isomers (for example, optical isomers, geometric isomers, or tautomers) are present in compounds used in the preparation methods described above, these isomers can also be used. In addition, in a case in which a solvate, a hydrate, or various shapes of crystal are present, the solvate, hydrate, or various shapes of crystal can also be used.

In a case in which the compound represented by Formula [1] or salt thereof is used as a medicine, a pharmaceutic aid such as an excipient, a carrier, or a diluent which is typically used in formulation may be used in an appropriately mixing manner.

Examples of an additive include an excipient, a disintegrating agent, a binding agent, a lubricant, a flavoring agent, a colorant, an aromatizer, a surfactant, a coating agent, and a plasticizer.

Examples of the excipient include a sugar alcohol such as erythritol, mannitol, xylitol, or sorbitol; a sugar such as white sugar, powdered sugar, lactose, or glucose; a cyclodextrin such as α-cyclodextrin, β-cyclodextrin, γ-cyclodextrin, hydroxypropyl β-cyclodextrin, or sodium sulfobutylether β-cyclodextrin; a cellulose such as crystalline cellulose or microcrystalline cellulose; and a starch such as a corn starch, a potato starch, or a pregelatinized starch.

Examples of the disintegrating agent include carmellose, carmellose calcium, croscarmellose sodium, sodium carboxymethyl starch, crospovidone, low substituted hydroxypropyl cellulose, and a partly pregelatinized starch.

Examples of the binding agent include hydroxypropyl cellulose, croscarmellose sodium, and methylcellulose.

Examples of the lubricant include stearic acid, magnesium stearate, calcium stearate, talc, hydrated silicon dioxide, light anhydrous silicic acid, and sucrose fatty acid ester.

Examples of the flavoring agent include aspartame, saccharin, stevia, thaumatin, and acesulfame potassium.

Examples of the colorant include titanium dioxide, ferric oxide, yellow ferric oxide, black iron oxide, Food Red No. 102, Food Yellow No. 4, and Food Yellow No. 5.

Examples of the aromatizer include an essential oil such as an orange oil, a lemon oil, a peppermint oil, or a pine oil; an essence such as an orange essence or a peppermint essence; a flavor such as a cherry flavor, a vanilla flavor, or a fruit flavor; a powder fragrance such as an apple micron, a banana micron, a peach micron, a strawberry micron, or an orange micron; vanillin; and ethyl vanillin.

Examples of the surfactant include sodium lauryl sulfate, dioctyl sodium sulfosuccinate, polysorbate, and polyoxyethylene hydrogenated castor oil.

Examples of the coating agent include hydroxypropyl methyl cellulose, aminoalkyl methacrylate copolymer E, aminoalkyl methacrylate copolymer RS, ethyl cellulose, cellulose acetate phthalate, hydroxypropyl methyl cellulose phthalate, a methacrylic acid copolymer L, a methacrylic acid copolymer LD, and a methacrylic acid copolymer S.

Examples of the plasticizer include triethyl citrate, macrogol, triacetin, and propylene glycol.

These additives may be used singly, or in combination of two or more kinds thereof.

A blending amount thereof is not particularly limited, and the additives may be appropriately blended such that the effect thereof is sufficiently exhibited depending on the respective purposes.

These additives can be administered orally or parenterally according to a usual method in a form such as a tablet, a capsule, a powder, a syrup, a granule, a pill, a suspension, an emulsion, a solution, a powdered formulation, a suppository, an eye drop, a nasal drop, an ear drop, a patch, an ointment, or an injection. In addition, an administration method, a dose, and a number of administration can be appropriately selected depending on a patient's age, body weight, and symptom. Typically, for an adult, 0.01 mg/kg to 1000 mg/kg may be administered orally or parenterally once or several times per day.

The compound or salt thereof of the invention is a compound for use in a treatment such as prevention of or cure for diseases associated with PI3K and/or ERK.

Examples of the diseases associated with PI3K and/or ERK include a cell proliferative disease, an allergic disease, an autoimmune disease, a neurodegenerative disease, a circulatory system disease, an inflammatory disease, an endocrine disorder, a metabolic disorder, and an infection.

Preferable examples of diseases to which the compound or salt thereof of the invention can be applied include malignant tumors in which the PI3K-AKT pathway and/or the Ras-Raf-MEK-ERK pathway is accelerated.

Specifically, the compound or salt thereof of the invention is a compound for use in a treatment such as prevention of or cure for malignant tumors which exhibit resistance with respect to a PI3K-AKT pathway inhibitor and/or a Ras-Raf-MEK-ERK pathway inhibitor.

EXAMPLES

The invention will be described with reference to Reference Examples, Examples, and Test Examples, but the invention is not limited thereto.

Unless otherwise specified, in purification by column chromatography, an automated purification apparatus ISOLERA (manufactured by Biotage Japan Ltd.) or a medium-pressure liquid chromatograph YFLC W-PREP 2XY (manufactured by YAMAZEN CORPORATION) was used.

Unless otherwise specified, as a carrier in silica gel column chromatography, SNAP KP-Sil CARTRIDGE (manufactured by Biotage Japan Ltd.), or HIGH FLASH COLUMN W001, W002, W003, W004, or W005 (manufactured by YAMAZEN CORPORATION) was used.

As NH silica, SNAP KP-NH CARTRIDGE (manufactured by Biotage Japan Ltd.) was used.

In preparative thin layer silica gel chromatography, PLC GLASS PLATE SILICA GEL $F_{60}$ (manufactured by Merck KGaA) was used.

As a microwave reaction apparatus, INITIATOR SIXTY (manufactured by Biotage Japan Ltd.) was used.

As a flow-type hydrogenation reaction apparatus, H-CUBE (manufactured by ThalesNano Inc.) was used.

In preparative reversed phase HPLC, WATERS 2998 PHOTODIODE ARRAY (PDA) DETECTOR (manufactured by Waters), WATERS 600 CONTROLLER (manufactured by Waters), a WATERS 2767 SAMPLE MANAGER (manufactured by Waters) set, and a YMC-ACTUS PROC18 (30 x 50 mm column) (manufactured by YMC Co., Ltd.) were used.

A MS spectrum was measured by an ionization method in which ACQUITY SQD LC / MS SYSTEM (manufactured by Waters, ionization method: ElectroSpray Ionization (ESI) method) and LCMS-2010EV (manufactured by Shimadzu Corporation, ionization method: ESI and Atomospheric Pressure Chemical Ionization (APCI)) were performed at the same time.

In the measurement of an NMR spectrum, tetramethylsilane was used as an internal standard, BRUKER AV300 (manufactured by Bruker Corporation) was used, and all δ values were shown in ppm.

Abbreviations in NMR measurement have the following meanings.

s: Singlet; br: Broad; d: Doublet; dd: Double doublet; t: Triplet; q: Quartet; quin: Quintet; sext: Sextet; sep: Septet; m: Multiplet; DMSO-$d_6$: Hexadeuterodimethylsulfoxide

Abbreviations in Reference Examples and Examples have the following meanings.

Ac: Acetyl; Bn: Benzyl; Boc: tert-Butoxycarbonyl; Bu: Butyl; $^t$Bu: tert-Butyl; Et: Ethyl; Fmoc: 9-Fluorenylmethyloxycarbonyl; Me: Methyl; Ms: Methylsulfonyl; Ph: Phenyl; SEM: (2-(Trimethylsilyl)ethoxy)methyl; TBS: tert-Butyldimethylsilyl; Tf: Trifluoromethylsulfonyl; TFA: Trifluoroacetic acid; THP: Tetrahydropyranyl; TMS: Trimethylsilyl; Ts: Toluenesulfonyl

<Example 0001>

<0001-1>

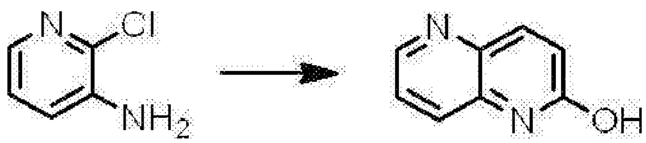

Triethylamine (13 mL), butyl acrylate (10 mL), and bis(tri-tert-butylphosphine)palladium(0) (350 mg) were added to a solution of 2-chloro-3-aminopyridine (6.00 g) in cyclohexyl acetate (60 mL), followed by stirring at 150°C for 40 hours in a nitrogen atmosphere. Water (30 ml) was added to the reaction mixture at 70°C, and the resultant product was cooled to room temperature while stirring. The reaction mixture was subjected to an ultrasonic treatment for 30 minutes, and the solid matter was collected by filtration and washed with water. Ethyl acetate (3 mL)/2-propanol (4 mL) was added to the obtained solid matter, and the resultant product was subjected to an ultrasonic treatment. The solid matter was collected by filtration, thereby obtaining 1,5-naphthyridin-2-ol (2.51 g) as a pale yellow solid.
MSm/z(M+H):147.

<0001-2>

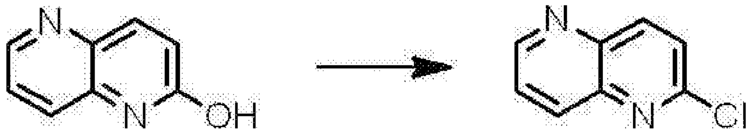

Phosphorus oxychloride (8.3 mL) was added to 1,5-naphthyridin-2-ol (2.76 g), followed by stirring at 100°C for 5 hours. The reaction mixture was cooled to room temperature, and added dropwise to a mixture of ethyl acetate (30 mL), water (30 mL), and sodium carbonate (9.57 g) over a period of 1 hour in an ice bath. Water (10 mL) was added thereto, and sodium carbonate was added thereto, followed by adjusting the pH of the resultant product to 8.3. The resultant product was stirred at room temperature for 10 minutes, and ethyl acetate (270 mL) and water (200 mL) were added thereto. The organic layer was collected by separation, and the aqueous layer was extracted two times with ethyl acetate (200 mL). The organic layer and the extraction liquid were combined, the resultant product was dried over anhydrous sodium sulfate, and the solvent was distilled off under reduced pressure, thereby obtaining 2-chloro-1,5-naphthyridine (2.86 g) as a pale yellow solid.
MSm/z(M+H): 165.

<0001-3>

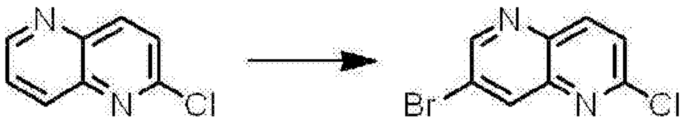

A solution of bromine (0.99 mL) in acetic acid (2.5 mL) was added dropwise to a mixture of 2-chloro-1,5-naphthyridine (2.88 g) and sodium acetate (2.89 g) in acetic acid (15 mL) at 85°C, and acetic acid (2 mL) was added thereto, followed by stirring at 85°C for 3 hours. The reaction mixture was cooled to room temperature, and added dropwise to a 6 mol/L sodium hydroxide aqueous solution (60 mL) under ice-cooling. The solid matter was collected by filtration, suspended in methanol (5 mL), and subjected to an ultrasonic treatment. The solid matter was collected by filtration, and washed with methanol (3 mL). The obtained solid was suspended in a 75 v/v% methanol aqueous solution (8 mL), the resultant product was subjected to an ultrasonic treatment, and the solid matter was collected by filtration, thereby obtaining 7-bromo-2-chloro-1,5-naphthyridine (3.33 g) as a pale yellow solid.
MSm/z(M+H): 243.

<0001-4>

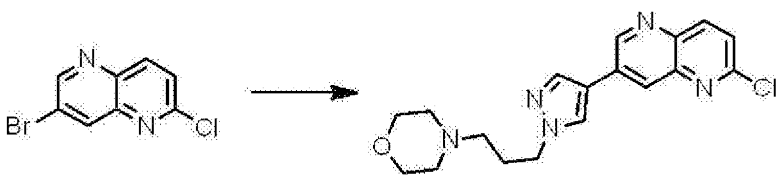

A mixture of 7-bromo-2-chloro-1,5-naphthyridine (30 mg), 1-(3-morpholinopropyl)-1H-pyrazole-4-boronic acid pinacol ester (59 mg), bis(di-tert-butyl (4-dimethylaminophenyl)phosphine)dichloropalladium(II) (5 mg) and sodium carbonate (20 mg) in 1,4-dioxane (1.9 mL) and water (0.1 mL) was stirred at 100°C for 7.5 hours in a nitrogen atmosphere. The reaction mixture was cooled to room temperature, ethanol (4 mL) was added thereto, and the solvent was distilled off under reduced pressure. The obtained residue was purified by silica gel column chromatography (chloroform-ethyl acetate, NH silica), thereby obtaining 4-(3-(4-(6-chloro-1,5-naphthyridin-3-yl)-1H-pyrazol-1-yl)propyl)morpholine (45.6 mg) as a white solid.
MSm/z(M+H): 358.

<0001-5>

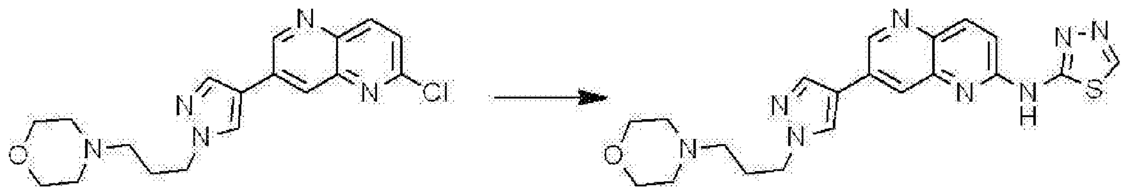

A mixture of 4-(3-(4-(6-chloro-1,5-naphthyridin-3-yl)-1H-pyrazol-1-yl)propyl)morpholine (45.6 mg), 1,3,4-thiadiazole-2-amine (19 mg), tris(dibenzylideneacetone)dipalladium(0) (23 mg), 4,5-bis(diphenylphosphino)-9,9-dimethylxanthene (28 mg) and cesium carbonate (120 mg) in 1,4-dioxane (1.9 mL) was stirred at 150°C for 30 minutes using a microwave reaction apparatus. The reaction mixture was cooled to room temperature, and ethanol was added thereto. The insolubles were filtered off using celite, the filtrate was concentrated under reduced pressure, and the obtained residue was purified by silica gel column chromatography (chloroform-methanol, NH silica), thereby obtaining N-(7-(1-(3-morpholinopropyl)-1H-pyrazol-4-yl)-1,5-naphthyridin-2-yl)-1,3,4-thiadiazole-2-amine (11 mg) as a white solid.
$^1$H-NMR(DMSO-$d_6$)$\delta$:12.23(1H,s),9.17(1H,s),9.09(1H,d,J=2.0Hz),8.57(1H,s),
8.33(1H,d,J=2.0Hz),8.28(1H,d,J=9.2Hz),8.21(1H,s),7.44(1H,d,J=9.2Hz),4.21(2H,t,J=7.1Hz),3.58(4H,t ,J=4.6Hz),2.36-2.28(6H,m),2.05-1.99(2H,m).
MSm/z(M+H):423.

<Example 0002>

<0002-1>

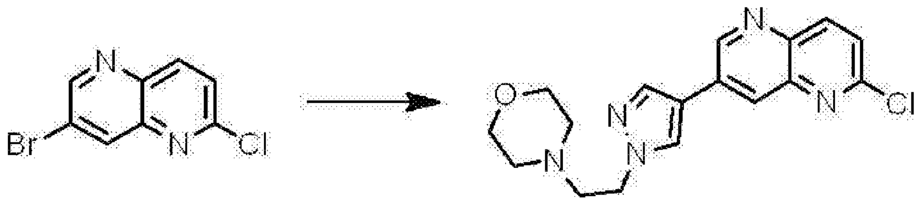

4-(2-(4-(6-Chloro-1,5-naphthyridin-3-yl)-1H-pyrazol-1-yl)ethyl)morpholine (96.9 mg) was obtained in the same manner as in Example 0001 except that 1-(2-morpholinoethyl)-1H-pyrazole-4-boronic acid pinacol ester was used instead of the 1-(3-morpholinopropyl)-1H-pyrazole-4-boronic acid pinacol ester used in Example 0001.
MSm/z(M+H): 344.

<0002-1>

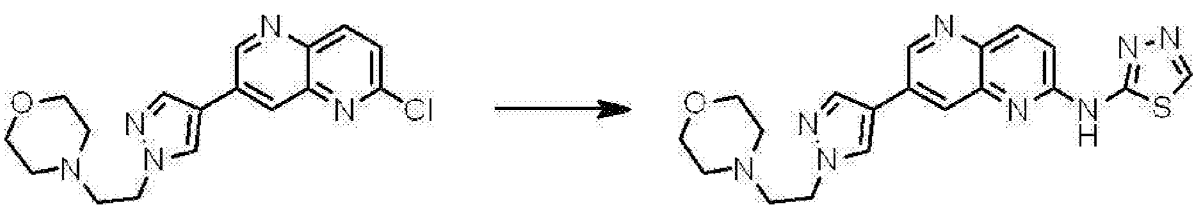

N-(7-(1-(2-morpholinoethyl)-1H-pyrazol-4-yl)-1,5-naphthyridin-2-yl)-1,3,4-thiadiazole-2-amine (16.6 mg) was obtained as a pale yellow solid in the same manner as in Example 0001 except that 4-(2-(4-(6-chloro-1,5-naphthyridin-3-yl)-1H-pyrazol-1-yl)ethyl)morpholine was used instead of the 4-(3-(4-(6-chloro-1,5-naphthyridin-3-yl)-1H-pyrazol-1-yl)propyl)morpholine used in Example 0001.
$^{1}$H-NMR(DMSO-$d_6$)δ:12.24(1H,s),9.17(1H,s),9.09(1H,d,J=2.0Hz),8.57(1H,s), 8.32(1H,d,J=2.0Hz),8.28(1H,d,J=8.9Hz),8.21(1H,s),7.44(1H,d,J=8.9Hz),4.30(2H,t,J=6.6Hz),3.56(4H,t ,J=4.5Hz),2.79(2H,t,J=6.6Hz),2.50-2.40(4H,m).
MSm/z(M+H):409.

<Example 0003>

<0003-1>

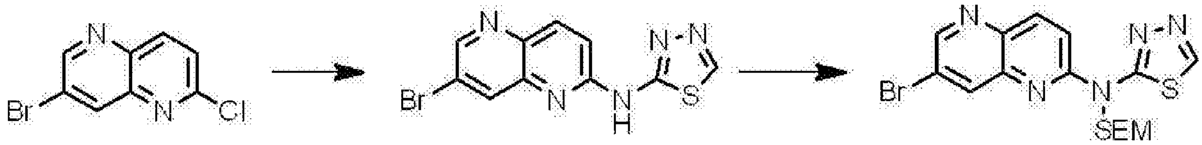

A mixture of 7-bromo-2-chloro-1,5-naphthyridine (30 mg), 1,3,4-thiadiazole-2-amine (25 mg) and potassium carbonate (17 mg) in dimethylsulfoxide (0.5 mL) was stirred at 150°C for 30 minutes using a microwave reaction apparatus. The reaction mixture was cooled to room temperature, and water was added thereto. The solid matter was collected by filtration, thereby obtaining N-(7-bromo-1,5-naphthyridin-2-yl)-1,3,4-thiadiazole-2-amine.

The same reaction was performed using 7-bromo-2-chloro-1,5-naphthyridine (60 mg), thereby obtaining N-(7-bromo-1,5-naphthyridin-2-yl)-1,3,4-thiadiazole-2-amine.

The obtained N-(7-bromo-1,5-naphthyridin-2-yl)-1,3,4-thiadiazole-2-amines were combined, and N,N-dimethylformamide (1.8 mL), 2-(chloromethoxy)ethyltrimethylsilane (86 μL), and N,N-diisopropylethylamine (172 μL) were added thereto, followed by stirring at room temperature for 18 hours. 2-(Chloromethoxy)ethyltrimethylsilane (36 μL) was added thereto, followed by stirring at 50°C for 2 hours. Water was added to the reaction mixture, the solid matter was collected by filtration, and washed with water and methanol, thereby obtaining N-(7-bromo-1,5-naphthyridin-2-yl)-N-((2-(trimethylsilyl)ethoxy)methyl)-1,3,4-thiadiazole-2-amine (78.9 mg) as a pale yellow solid.
MSm/z(M+H):438.

<0003-2>

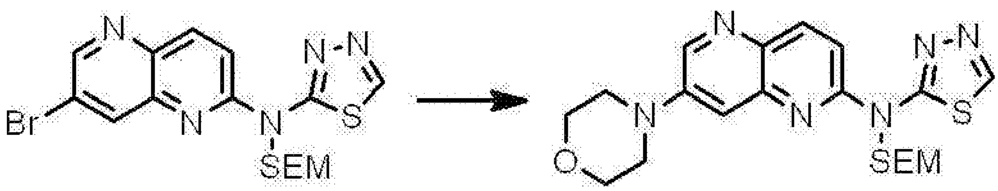

1,4-Dioxane (1 mL) and toluene (3 mL) were added to a mixture of N-(7-bromo-1,5-naphthyridin-2-yl)-N-((2-(trimethylsilyl)ethoxy)methyl)-1,3,4-thiadiazole-2-amine (78.9 mg), morpholine (47 μL), tris(dibenzylideneacetone)dipalladium(0) (33 mg), 4,5-bis(diphenylphosphino)-9,9-dimethylxanthene (42 mg), and cesium carbonate (175 mg), followed by stirring at 100°C for 5 hours. The reaction mixture was cooled to room temperature, and water and ethyl acetate were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate, NH silica), thereby obtaining N-(7-morpholno-1,5-naphthyridin-2-yl)-N-((2-(trimethylsilyl)ethoxy)methyl)-1,3,4-thiadiazole-2-amine (17.1 mg) as a pale yellow solid.
MSm/z(M+H):445.

<0003-3>

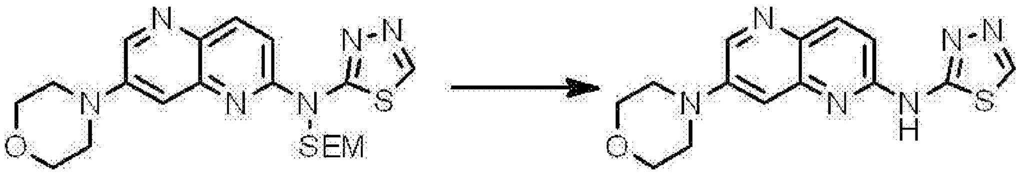

Concentrated hydrochloric acid (1 mL) was added to a solution of N-(7-morpholino-1,5-naphthyridin-2-yl)-N-((2-(trimethylsilyl)ethoxy)methyl)-1,3,4-thiadiazole-2-amine (50 mg) in ethanol (3 mL), followed by stirring at 90°C for 17 hours. The reaction mixture was cooled to room temperature, and neutralized by the addition of a 6.0 mol/L sodium hydroxide aqueous solution under ice-cooling. The solvent was distilled off under reduced pressure, and the obtained residue was purified by silica gel column chromatography (chloroform-ethyl acetate, chloroform-methanol, NH silica), thereby obtaining N-(7-morpholino-1,5-naphthyridin-2-yl)-1,3,4-thiadiazole-2-amine (1.4 mg) as a pale yellow solid.
$^1$H-NMR(DMSO-$d_6$)$\delta$:12.07(1H,s),9.09(1H,s),8,74(1H,d,J=2.6Hz),8.16(1H,d,J=8.9Hz),
7.37(1H,d,J=2.6Hz),7.24(1H,d,J=8.9Hz),3.81(4H,t,J=4.8Hz),3.40-3.35(4H,m).
MSm/z(M+H): 315.

<Example 0004> (not in accordance with the present invention)

<0004-1>

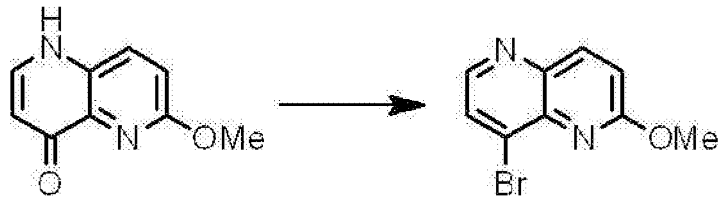

Phosphorous oxybromide (5.60 g) was added to a solution of 6-methoxy-1,5-naphthyridin-4(1H)-one (3.51 g) inN,N-dimethylformamide (20 mL), followed by stirring at 80°C for 30 minutes. The reaction mixture was cooled to room temperature, and added dropwise to a mixture solution of methanol-water (1:10). The resultant product was neutralized by the addition of a sodium hydroxide aqueous solution, and ethyl acetate was added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining 8-bromo-2-methoxy-1,5-naphthyridine (3.20 g) as a yellow solid.
MSm/z(M+H):239,241.

<0004-2>

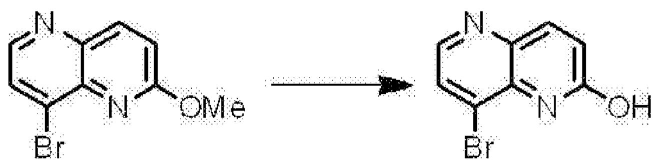

A mixture of 8-bromo-2-methoxy-1,5-naphthyridine (500 mg) in 5.1 mol/L hydrobromic acid (5 mL) was stirred at 80°C for 3 hours. The reaction mixture was allowed to cool to room temperature, and the solvent was distilled off under reduced pressure. The residue was neutralized by the addition of a saturated sodium hydrogen carbonate aqueous solution, and the solvent was distilled off under reduced pressure. The obtained residue was washed with water, thereby obtaining 8-bromo-1,5-naphthyridin-2-ol (360 mg) as a white solid.
MSm/z(M+H): 225,227.

<0004-3>

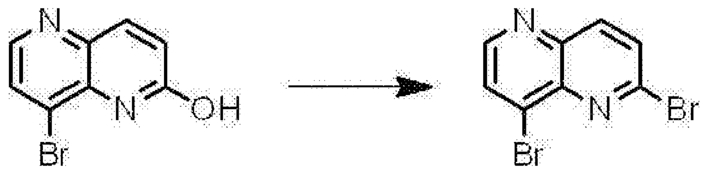

A mixture solution of 8-bromo-1,5-naphthyridin-2-ol (6.0 g) in toluene (100 mL)/N,N-dimethylformamide (10 mL) was heated to 110°C, and a suspension of phosphorous oxybromide (10.13 g) in toluene (50 mL) was added dropwise thereto. The reaction mixture was stirred at 110°C for 30 minutes, allowed to cool to room temperature, added dropwise to water, and neutralized by the addition of a sodium hydroxide aqueous solution, and ethyl acetate was added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining 2,8-dibromo-1,5-naphthyridine (3.53 g) as a pale yellow solid.
MSm/z(M+H):287,289,291.

<0004-4>

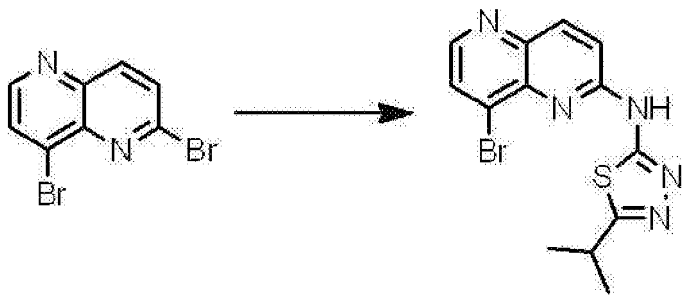

A mixture of 2,8-dibromo-1,5-naphthyridine (3.54 g), 5-isopropyl-1,3,4-thiadiazole-2-amine (1.85 g), tris(dibenzylideneacetone)dipalladium(0) (0.56 g), 4,5-bis(diphenylphosphino)-9,9-dimethylxanthene (0.71 g) and cesium carbonate (8.03 g) in 1,4-dioxane (17.6 mL) was reacted at 130°C for 20 minutes using a microwave reaction apparatus. Ethyl acetate and water were added to the reaction mixture. The organic layer was collected by separation, washed with a saturated sodium chloride aqueous solution, and dried over anhydrous sodium sulfate, and the solvent was distilled off under reduced pressure. The obtained residue was purified by silica gel column chromatography (methanol-ethyl acetate), and the resultant product was washed with methanol and chloroform, thereby obtaining N-(8-bromo-1,5-naphthyridin-2-yl)-5-isopropyl-1,3,4-thiadiazole-2-amine (333 mg) as a pale yellow solid.
MSm/z(M+H):350,352.

<0004-5>

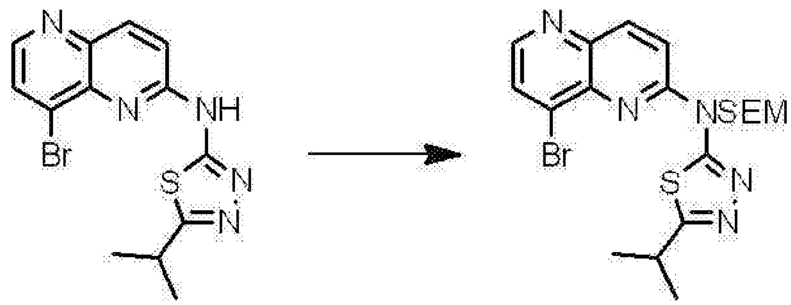

(2-(Chloromethoxy)ethyl)trimethylsilane (0.43 mL) was added to a suspension of N-(8-bromo-1,5-naphthyridin-2-yl)-5-isopropyl-1,3,4-thiadiazole-2-amine (564 mg) in N-methylpyrrolidone (16.1 mL), and 60% sodium hydride (136 mg) was added thereto under ice-cooling, followed by stirring for 2 hours in a nitrogen atmosphere. The reaction was stopped by the addition of ethanol to the reaction mixture, and ethyl acetate and water were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (methanol-ethyl acetate), thereby obtaining N-(8-bromo-1,5-naphthyridin-2-yl)-5-isopropyl-N-((2-trimethylsilyl)ethoxy)methyl)-1,3,4-thiadiazole-2-amine (398 mg) as a pale yellow solid.
MSm/z(M+H): 480,482.

<0004-6>

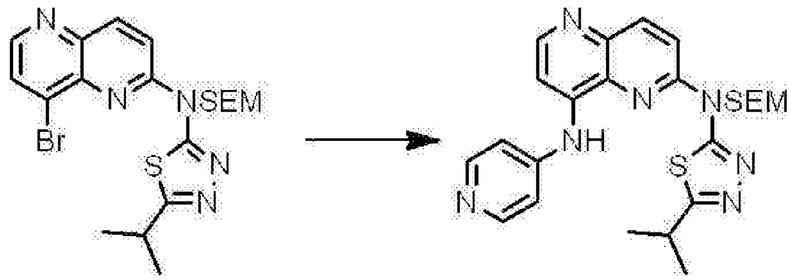

4-Aminopyridine (4.8 mg), tris(dibenzylideneacetone)dipalladium(0) (3.1 mg), 2-(dicyclohexylphosphino)-2',4',6'-triisopropylbiphenyl (3.3 mg), and sodium tert-butoxide (8.1 mg) were added to a solution of N-(8-bromo-1,5-naphthyridin-2-yl)-5-isopropyl-N-((2-(trimethylsilyl)ethoxy)methyl)-1,3,4-thiadiazole-2-amine (16.5 mg) in 1,4-dioxane (1.4 mL), followed by reacting at 150°C for 30 minutes using a microwave reaction apparatus. Ethyl acetate and water were added to the reaction mixture. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane, methanol-ethyl acetate), thereby obtaining $N^2$-(5-isopropyl-1,3,4-thiadiazol-2-yl)-$N^8$-(pyridin-4-yl)-$N^2$-((2-(trimethylsilyl)ethoxy)methyl)-1,5-naphthyridine-2,8-diamine (8.0 mg) as an orange solid.
MSm/z(M+H):494.

<0004-7>

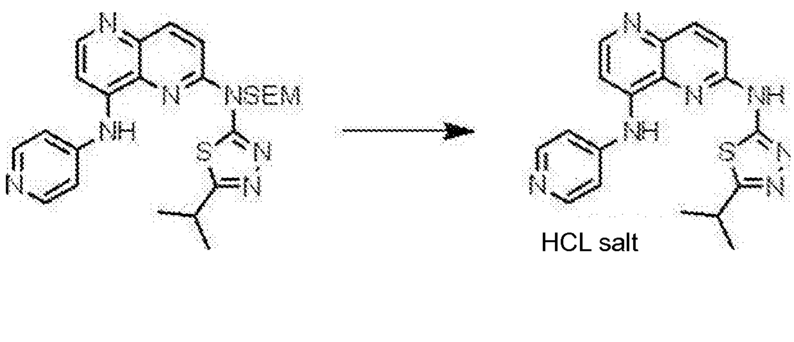

A solution (3.0 mL) of 4.0 mol/L hydrogen chloride/1,4-dioxane was added to $N^2$-(5-isopropyl-1,3,4-thiadiazol-2-yl)-$N^8$-(pyridin-4-yl)-$N^2$-((2-(trimethylsilyl)ethoxy)methyl)-1,5-naphthyridine-2,8-diamine (8.0 mg), followed by stirring at room temperature for 1 hour. The solvent was distilled off under reduced pressure, thereby obtaining $N^2$-(5-isopropyl-1,3,4-thiadiazol-2-yl)-$N^8$-(pyridin-4-yl)-1,5-naphthyridine-2,8-diamine (5.1 mg) hydrochloride as a yellow solid. $^1$H-NMR($CD_3OD$)δ:8.93(1H,d,J=5.9Hz),8.68(2H,d,J=6.6Hz),8.48(1H,d,J=9.2Hz), 8.20(1H,d,J=5.9Hz),7.84(3H,m,J=16.2,7.6Hz),3.41(1H,t,J=6.9Hz),1.47(6H,d,J=14.9Hz).
MSm/z(M+H): 364.

<Example 0005> (not in accordance with the present invention) <0005-1>

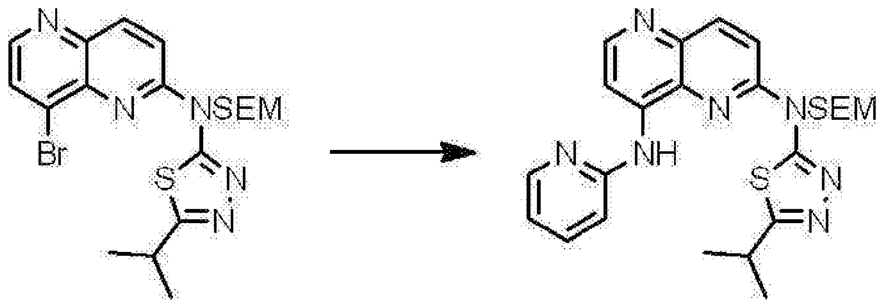

2-Aminopyridine (3.0 mg), tris(dibenzylideneacetone)dipalladium(0) (1.9 mg), 2-(dicyclohexylphosphino)-2',4',6'-triisopropylbiphenyl (2.0 mg), and sodium tert-butoxide (5.0 mg) were added to a solution of N-(8-bromo-1,5-naphthyridin-2-yl)-5-isopropyl-N-((2-(trimethylsilyl)ethoxy)methyl)-1,3,4-thiadiazole-2-amine (10 mg) in 1,4-dioxane (1 mL), followed by reacting at 150°C for 30 minutes using a microwave reaction apparatus. Ethyl acetate and water were added to the reaction mixture. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining $N^2$-(5-isopropyl-1,3,4-thiadiazol-2-yl)-$N^8$-(pyridin-2-yl)-$N^2$-((2-(trimethylsilyl)ethoxy)methyl)-1,5-naphthyridine-2,8-diamine (7.2 mg).
MSm/z(M+H):494.

<0005-2>

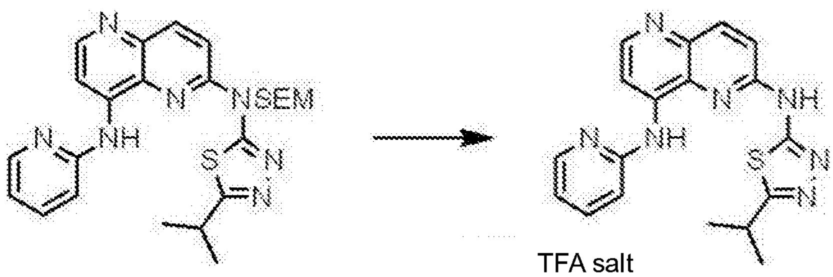

TFA salt

Trifluoroacetic acid (2 mL) and water (0.1 mL) were added to $N^2$-(5-isopropyl-1,3,4-thiadiazol-2-yl)-$N^8$-(pyridin-2-yl)-$N^2$-((2-(trimethylsilyl)ethoxy)methyl)-1,5-naphthyridine-2,8-diamine (7.2 mg), followed by stirring at room temperature for 30 minutes. The solvent was distilled off under reduced pressure, and ethyl acetate was added to the obtained solid. The solid matter was collected by filtration, and washed with ethyl acetate, thereby obtaining $N^2$-(5-isopropyl-1,3,4-thiadiazol-2-yl)-$N^8$-(pyridin-2-yl)-1,5-naphthyridine-2,8-diamine trifluoroacetate (5.9 mg) as a pale yellow solid.
$^1$H-NMR(DMSO-$d_6$)δ:9.57(1H,s),8.85-8.75(2H,m),8.52(1H,d,J=5.3Hz),8.40(1H,d,J=9.2Hz), 7.98(1H,t,J=6.9Hz),7.73(1H,d,J=9.2Hz),7.43(1H,d,J=8.6Hz),7.25(1H,t,J=5.9Hz),1.46(6H,d,J=6.6Hz).
MSm/z(M+H):364.

<Example 0006> (not in accordance with the present invention) <0006-1>

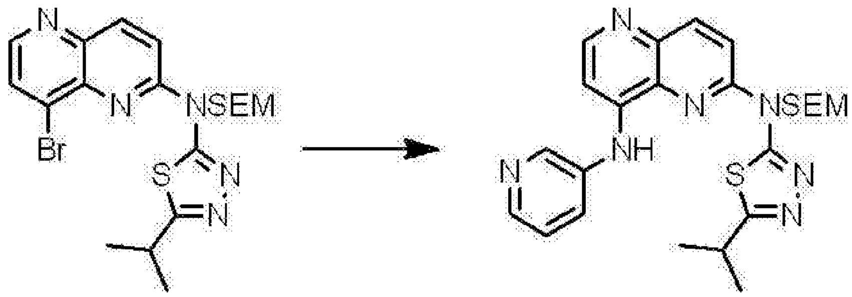

$N^2$-(5-isopropyl-1,3,4-thiadiazol-2-yl)-$N^8$-(pyridin-3-yl)-$N^2$-((2-(trimethylsilyl)ethoxy)methyl)-1,5-naphthyridine-2,8-diamine (5.0 mg) was obtained in the same manner as in Example 0005 except that 3-aminopyridine was used instead of the 2-aminopyridine used in Example 0005.
MSm/z(M+H): 494.

<0006-2>

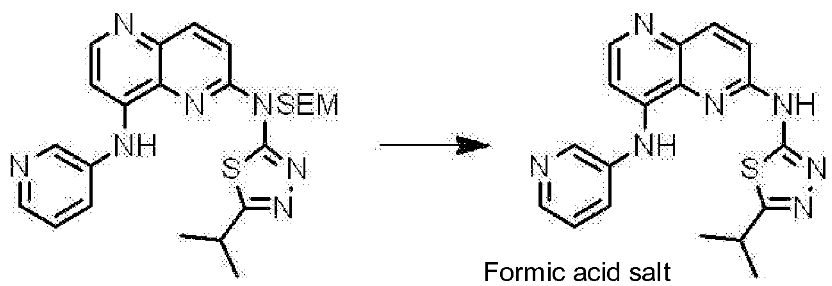


Trifluoroacetic acid (2 mL) and water (0.1 mL) were added to $N^2$-(5-isopropyl-1,3,4-thiadiazol-2-yl)-$N^8$-(pyridin-3-yl)-$N^2$-((2-(trimethylsilyl)ethoxy)methyl)-1,5-naphthyridine-2,8-diamine (5.0 mg), followed by stirring at room temperature for 30 minutes. After the solvent was distilled off under reduced pressure, the obtained solid was purified by preparative reversed phase HPLC (a 0.1% formic acid aqueous solution-a 0.1% formic acid acetonitrile solution), and the solvent was distilled off under reduced pressure, thereby obtaining $N^2$-(5-isopropyl-1,3,4-thiadiazol-2-yl)-$N^8$-(pyridin-3-yl)-1,5-naphthyridine-2,8-diamine (0.4 mg) formate as a yellow solid.
$^1$H-NMR($CD_3OD$)$\delta$:8.88(1H,s),8.65(1H,d,J=4.6Hz),8.49(1H,d,J=7.3Hz),8.32(1H,d,J=9.2Hz), 8.20(1H,d,J=7.9Hz),7.79-7.75(2H,m),7.36(1H,d,J=7.3Hz),2.18(1H,d,J=7.9Hz), 1.45(6H,d,J=7.3Hz).
MSm/z(M+H): 364.

<Example 0007> (not in accordance with the present invention) <0007-1>

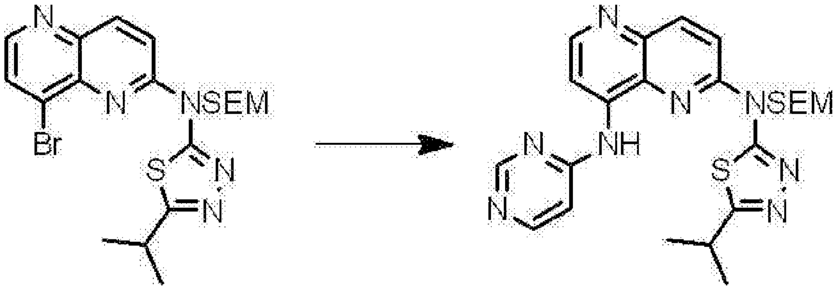

$N^2$-(5-isopropyl-1,3,4-thiadiazol-2-yl)-$N^8$-(pyrimidin-4-yl)-$N^2$-((2-(trimethylsilyl)ethoxy)methyl)-1,5-naphthyridine-2,8-diamine (2.2 mg) was obtained in the same manner as in Example 0005 except that 4-aminopyridine was used instead of the 2-aminopyridine used in Example 0005.
MSm/z(M+H): 495.

<0007-2>

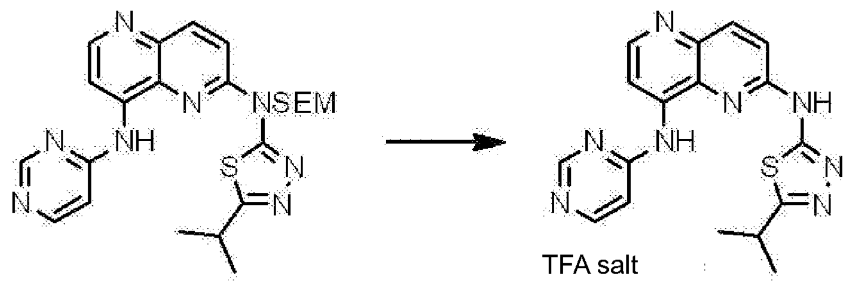


$N^2$-(5-isopropyl-1,3,4-thiadiazol-2-yl)-$N^8$-(pyrimidin-4-yl)-1,5-naphthyridine-2,8-diamine trifluoroacetate (0.9 mg) was obtained as a pale yellow solid in the same manner as in Example 0005 except that $N^2$-(5-isopropyl-1,3,4-thiadiazol-2-yl)-$N^8$-(pyrimidin-4-yl)-$N^2$-((2-(trimethylsilyl)ethoxy)methyl)-1,5-naphthyridine-2,8-diamine was used instead of the $N^2$-(5-isopropyl-1,3,4-thiadiazol-2-yl)-$N^8$-(pyridin-2-yl)-$N^2$-((2-(trimethylsilyl)ethoxy)methyl)-1,5-naphthyridine-2,8-diamine used in Example 0005, and hexane was used instead of the ethyl acetate used in Example 0005 as a washing solvent of the crude product.
$^1$H-NMR($CD_3OD$)δ:9.19(1H,d,J=6.6Hz),9.09(1H,s),8.80-8.76(2H,m),8.39(1H,d,J=9.2Hz), 7.77(1H,d,J=9.2Hz),7.42(1H,d,J=5.3Hz),3.54-3.51(1H,m),1.54(6H,d,J=6.6Hz).
MSm/z(M+H): 365.

<Example 0008> (not in accordance with the present invention) <0008-1>

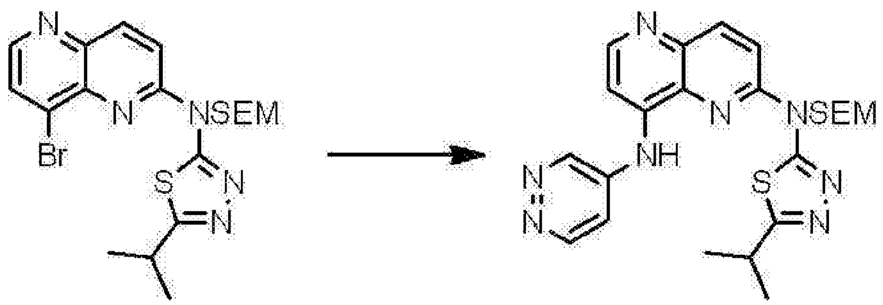

$N^2$-(5-isopropyl-1,3,4-thiadiazol-2-yl)-$N^8$-(pyridazin-4-yl)-$N^2$-((2-(trimethylsilyl)ethoxy)methyl)-1,5-naphthyridine-2,8-diamine (4.0 mg) was obtained in the same manner as in Example 0005 except that 4-aminopyridine was used instead of the 2-aminopyridine used in Example 0005.
MSm/z(M+H): 495.

<0008-2>

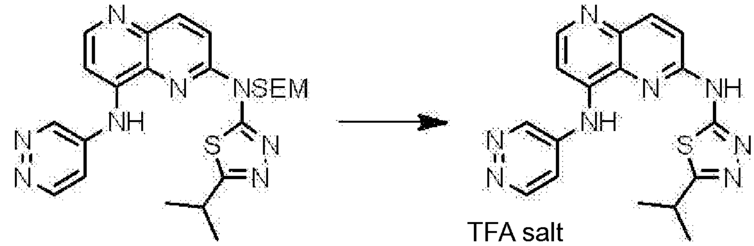


$N^2$-(5-isopropyl-1,3,4-thiadiazol-2-yl)-$N^8$-(pyridazin-4-yl)-1,5-naphthyridine-2,8-diamine trifluoroacetate (2.2 mg) was obtained as an orange solid in the same manner as in Example 0005 except that $N^2$-(5-isopropyl-1,3,4-thiadiazol-2-yl)-$N^8$-(pyridazin-4-yl)-$N^2$-((2-(trimethylsilyl)ethoxy)methyl)-1,5-naphthyridine-2,8-diamine was used instead of the $N^2$-(5-isopropyl-1,3,4-thiadiazol-2-yl)-$N^8$-(pyridin-2-yl)-$N^2$-((2-(trimethylsilyl)ethoxy)methyl)-1,5-naphthyridine-2,8-diamine used in Example 0005, and a mixture solution of hexane/ethyl acetate was used instead of the ethyl acetate used in Example 0005 as a washing solvent of the crude product.
$^1$H-NMR($CD_3OD$)$\delta$:9.02(1H,d,J=3.3Hz),8.86-8.84(2H,m),8.39(1H,d,J=9.2Hz), 7.82(1H,d,J=5.3Hz),7.59(1H,d,J=8.6Hz),7.33-7.31(1H,m),2.19(1H,t,J=7.6Hz), 1.39(6H,d,J=7.3Hz).
MSm/z(M+H): 365.

<Example 0009> (not in accordance with the present invention)

<0009-1>

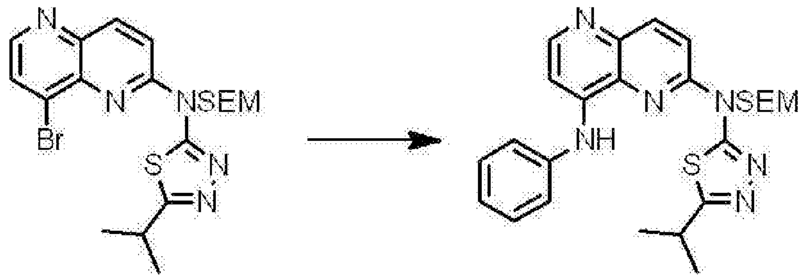

$N^2$-(5-isopropyl-1,3,4-thiadiazol-2-yl)-$N^8$-phenyl-$N^2$-((2-(trimethylsilyl)ethoxy)methyl)-1 ,5-naphthyridine-2,8-diamine (8.0 mg) was obtained in the same manner as in Example 0005 except that aniline was used instead of the 2-aminopyridine used in Example 0005.
MSm/z(M+H): 493.

<0009-2>

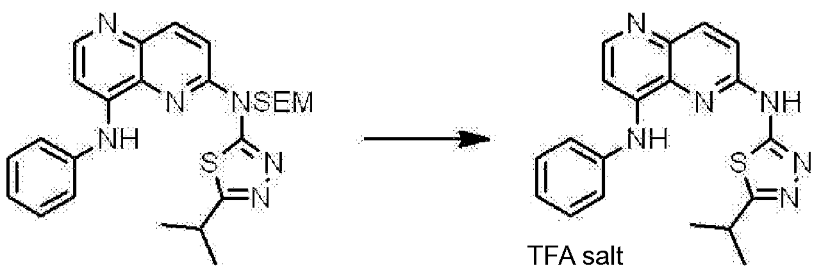


$N^2$-(5-isopropyl-1,3,4-thiadiazol-2-yl)-$N^8$-phenyl-1,5-naphthyridine-2,8-diamine trifluoroacetate (0.9 mg) was obtained as a yellow solid in the same manner as in Example 0005 except that $N^2$-(5-isopropyl-1,3,4-thiadiazol-2-yl)-$N^8$-phenyl-$N^2$-((2-(trimethylsilyl)ethoxy)methyl)-1,5-naphthyridine-2,8-diamine was used instead of the $N^2$-(5-isopropyl-1,3,4-thiadiazol-2-yl)-$N^8$-(pyridin-2-yl)-$N^2$-((2-(trimethylsilyl)ethoxy)methyl)-1,5-naphthyridine-2,8-diamine used in

Example 0005.
$^{1}$H-NMR($CD_3OD$)δ:8.42(1H,d,J=6.6Hz),8.29(1H,d,J=9.2Hz),7.76(1H,d,J=9.2Hz),7.62(2H,s), 7.61(2H,s),7.47(1H,d,J=4.6Hz),7.34(1H,d,J=7.3Hz),3.44(1H,t,J=6.9Hz),1.45(6H,d,J=6.6Hz).
MSm/z(M+H): 363.

<Example 0010>

<0010-1>

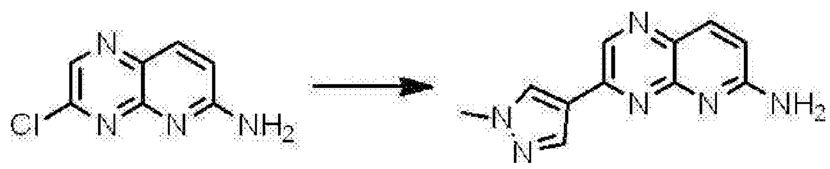

1-Methyl-4-(4,4,5,5-tetramethyl-1,3,2-dioxaborolan-2-yl)-1H-pyrazole (866 mg), tris(dibenzylideneacetone)dipalladium(0) (79.1 mg), tricyclohexylphosphine (92.5 mg), and potassium phosphate (1 g) were added to a mixture solution of 3-chloropyrido[2,3-b]pyrazine-6-amine (500 mg) in 1,4-dioxane (12.4 mL)/water (1.4 mL), followed by reacting at 120°C for 10 minutes using a microwave reaction apparatus. Ethyl acetate/chloroform was added to the reaction mixture, the solid matter was collected by filtration, and washed with ethyl acetate, thereby obtaining 3-(1-methyl-1H-pyrazol-4-yl)pyrido[2,3-b]pyrazine-6-amine (326.6 mg).
MSm/z(M+H): 227.

<0010-2>

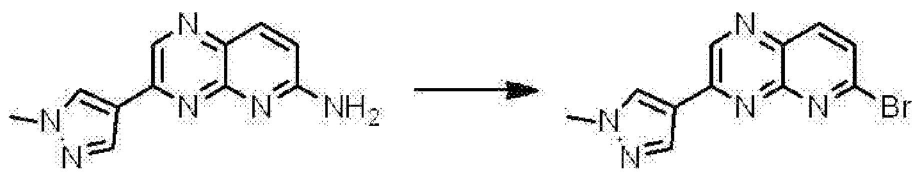

Antimony bromide (127.8 mg) and tert-butyl nitrite (0.26 mL) were added to a solution of 3-(1-methyl-1H-pyrazol-4-yl)pyrido[2,3-b]pyrazine-6-amine (50 mg) in dibromomethane (1.26 mL) at 0°C in a nitrogen atmosphere, followed by stirring at room temperature for 7 hours. The insolubles were filtered off, and the solid was washed with ethyl acetate/water. The filtrate and the washings were combined, and ethyl acetate was added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane, methanol-ethyl acetate), thereby obtaining 6-bromo-3-(1-methyl-1H-pyrazol-4-yl)pyrido[2,3-b]pyrazine (10.8 mg) as a pale yellow solid.
MSm/z(M+H): 290,292.

<0010-3>

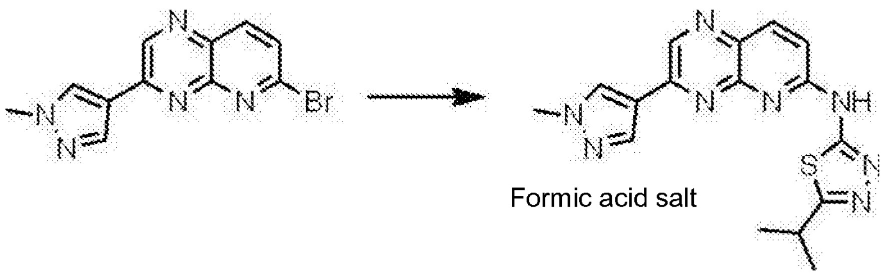


5-Isopropyl-1,3,4-thiadiazole-2-amine (2.5 mg) and potassium carbonate (3.6 mg) were added to a solution of 6-bromo-3-(1-methyl-1H-pyrazol-4-yl)pyrido[2,3-b]pyrazine (5.0 mg) in dimethylsulfoxide (0.35 mL), followed by stirring at 130°C for 8 hours. After the reaction mixture was cooled to room temperature, ethyl acetate and water were added thereto. The organic layer was collected by separation, washed with a saturated sodium chloride aqueous solution, and dried over anhydrous sodium sulfate, and the solvent was distilled off under reduced pressure. The obtained residue was purified by preparative reversed phase HPLC (a 0.1% formic acid aqueous solution-a 0.1% formic acid acetonitrile solution), and the solvent was distilled off under reduced pressure, thereby obtaining 5-isopropyl-N-(3-(1-methyl-1H-pyrazol-4-yl)pyrido[2,3-b]pyrazine-6-yl)-1,3,4-thiadiazole-2-amineformate (1.1 mg) as a yellow solid.
$^1$H-NMR($CD_3OD$)δ:9.09(1H,s),8.56(1H,s),8.36(1H,s),8.29(1H,d,J=9.2Hz), 7.41(1H,d,J=8.6Hz),4.03(3H,s),3.98-3.96(1H,m),1.50(6H,d,J=6.6Hz).
MSm/z(M+H): 353.

<Example 0011> (not in accordance with the present invention)

<0011-1>

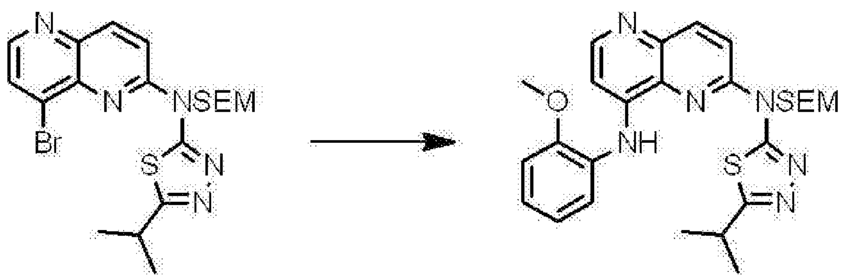

N2-(5-isopropyl-1,3,4-thiadiazol-2-yl)-N8-(2-methoxyphenyl)-N2-((2-(trimethylsilyl)ethoxy)methyl)-1,5-naphthyridine-2,8-diamine (3.3 mg) was obtained in the same manner as in Example 0005 except that 2-methoxyaniline was used instead of the 2-aminopyridine used in Example 0005.
MSm/z(M+H):523.

<0011-2>

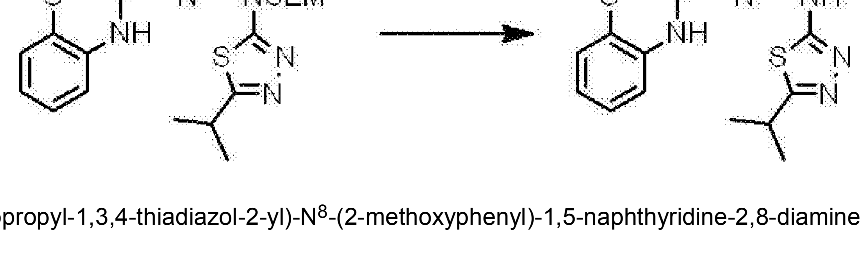

N2-(5-isopropyl-1,3,4-thiadiazol-2-yl)-N8-(2-methoxyphenyl)-1,5-naphthyridine-2,8-diamine (2.1 mg) was obtained as a yellow solid in the same manner as in Example 0005 except that N2-(5-isopropyl-1,3,4-thiadiazol-2-yl)-N8-(2-methoxyphenyl)-N2-((2-(trimethylsilyl)ethoxy)methyl)-1,5-naphthyridine-2,8-diamine was used instead of the N2-(5-isopropyl-1,3,4-thiadiazol-2-yl)-N8-(pyridin-2-yl)-N2-((2-(trimethylsilyl)ethoxy)methyl)-1,5-naphthyridine-2,8-diamine used in Example 0005. $^1H$-NMR($CD_3OD$)δ:8.41(1H,d,J=6.6Hz),8.28(1H,d,J=9.2Hz),7.73-7.68(2H,m),7.40-7.37(2H,m),7.29(1H,d,J=8.6Hz),7.21-7.15(1H,m),3.97(3H,s),3.43(1H,t,J=6.9Hz),1.45(6H,d,J=7.3Hz).
MSm/z(M+H):393.

<Example 0012> (not in accordance with the present invention)

<0012-1>

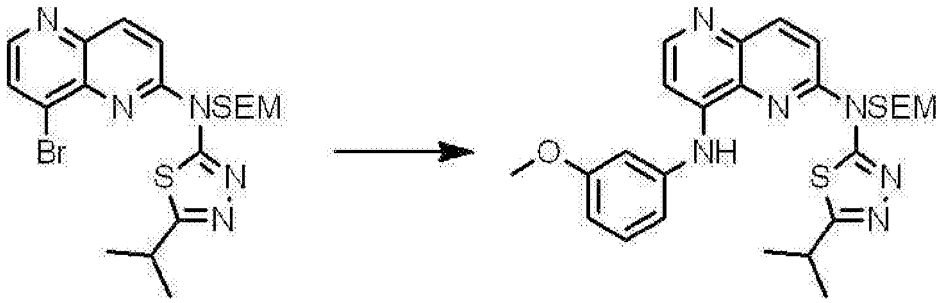

N2-(5-isopropyl-1,3,4-thiadiazol-2-yl)-N8-(3-methoxyphenyl)-N2-((2-(trimethylsilyl)ethoxy)methyl)-1,5-naphthyridine-2,8-diamine (5.8 mg) was obtained in the same manner as in Example 0005 except that 3-methoxyaniline was used instead of the 2-aminopyridine used in Example 0005.
MSm/z(M+H):523.

<0012-2>

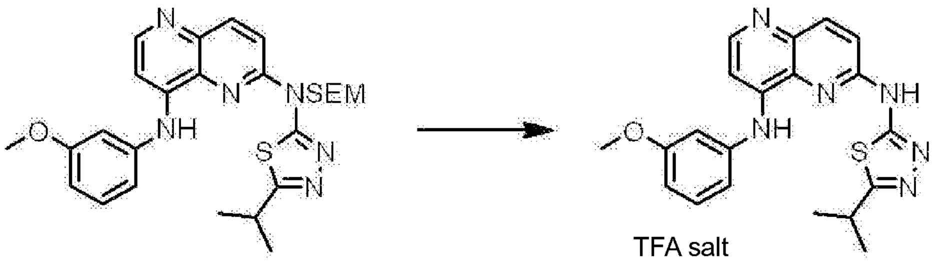

TFA salt

N2-(5-isopropyl-1,3,4-thiadiazol-2-yl)-N8-(3-methoxyphenyl)-1,5-naphthyridine-2,8-diamine trifluoroacetate (5.0 mg) was obtained as a brown solid in the same manner as in Example 0005 except that N2-(5-isopropyl-1,3,4-thiadiazol-2-yl)-N8-(3-methoxyphenyl)-N2-((2-(trimethylsilyl)ethoxy)methyl)-1,5-naphthyridine-2,8-diamine was used instead of the N2-(5-isopropyl-1,3,4-thiadiazol-2-yl)-N8-(pyridin-2-yl)-N2-((2-(trimethylsilyl)ethoxy)methyl)-1,5-naphthyridine-2,8-diamine used in Example 0005, and hexane was used instead of the ethyl acetate used in Example 0005 as a washing solvent of the crude product.
$^1H$-NMR($CD_3OD$)δ:8.42(1H,d,J=7.3Hz),8.29(1H,d,J=9.2Hz),7.74(1H,d,J=9.2Hz), 7.52(1H,t,J=7.9Hz),7.40(1H,d,J=6.6Hz),7.18(2H,m,J=4.6Hz),7.02(1H,dd,J=8.3,1.7Hz), 3.88(3H,d,J=2.0Hz),3.44(1H,t,J=6.9Hz),1.45(6H,d,J=6.9Hz).
MSm/z(M+H):393.

<Example 0013> (not in accordance with the present invention)

<0013-1>

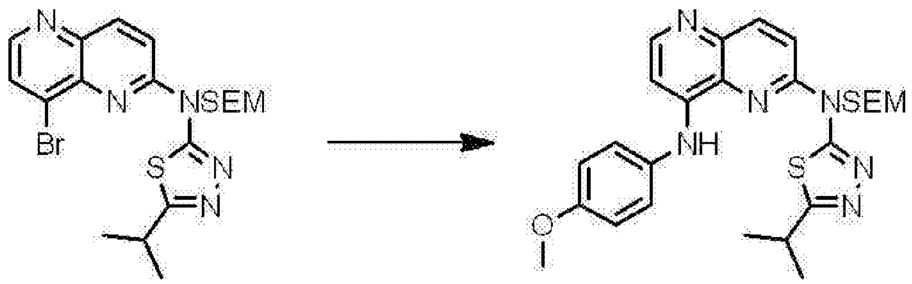

$N^2$-(5-isopropyl-1,3,4-thiadiazol-2-yl)-$N^8$-(4-methoxyphenyl)-$N^2$-((2-(trimethylsilyl)ethoxy)methyl)-1,5-naphthyridine-2,8-diamine (4.0 mg) was obtained in the same manner as in Example 0005 except that 4-methoxyaniline was used instead of the 2-aminopyridine used in Example 0005.
MSm/z(M+H):523.

<0013-2>

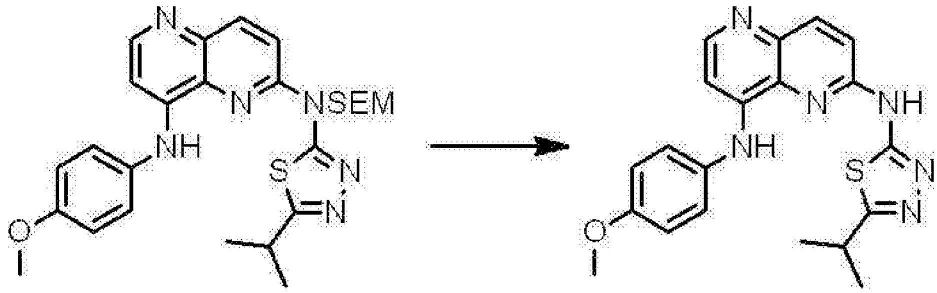

$N^2$-(5-isopropyl-1,3,4-thiadiazol-2-yl)-$N^8$-(4-methoxyphenyl)-1,5-naphthyridine-2,8-diamine (3.0 mg) was obtained as a yellow solid in the same manner as in Example 0005 except that $N^2$-(5-isopropyl-1,3,4-thiadiazol-2-yl)-$N^8$-(4-methoxyphenyl)-$N^8$-((2-(trimethylsilyl)ethoxy)methyl)-1,5-naphthyridine-2,8-diamine was used instead of the $N^2$-(5-isopropyl-1,3,4-thiadiazol-2-yl)-$N^8$-(pyridin-2-yl)-$N^2$-((2-(trimethylsilyl)ethoxy)methyl)-1,5-naphthyridine-2,8-diamine used in Example 0005, and the crude product was purified by silica gel column chromatography (ethyl acetate-hexane, methanol-ethyl acetate).
$^1$H-NMR(DMSO-$d_6$)$\delta$:8.40(1H,d,J=5.3Hz),8.24(1H,d,J=9.2Hz),8.12(1H,s),
7.52(1H,d,J=9.2Hz),7.45(2H,d,J=9.2Hz),7.07(3H,m,J=7.9,3.7Hz),3.80(3H,s), 3.41(1H,t,J=6.9Hz), 1.38(6H,d,J=6.6Hz).
MSm/z(M+H):393.

<Example 0014>

<0014-1>

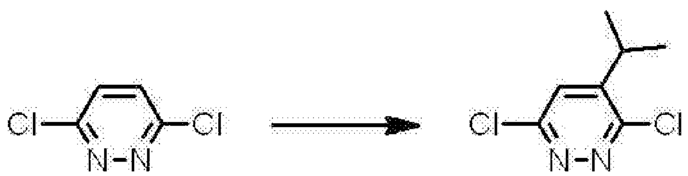

Isobutyric acid (4.67 mL) and silver nitrate (1.15 g) were added to a solution of 3,6-dichloropyridazine (5 g) in water (168 mL)/sulfuric acid (7.4 mL), and a solution of ammonium peroxodisulfate (26 g) in water (84 mL) was added dropwise thereto at room temperature over a period of 20 minutes, followed by stirring at 70°C for 30 minutes. The reaction mixture was cooled to room temperature, adjusted to have a pH of 8 by the addition of ammonia water, and ethyl acetate was added thereto. The organic layer was collected by separation, washed with a saturated sodium chloride aqueous solution, and dried over anhydrous sodium sulfate, and the solvent was distilled off under reduced pressure, thereby obtaining 3,6-dichloro-4-isopropylpyridazine (6.33 g) as pale yellow oily substance.
MSm/z(M+H):191.

<0014-2>

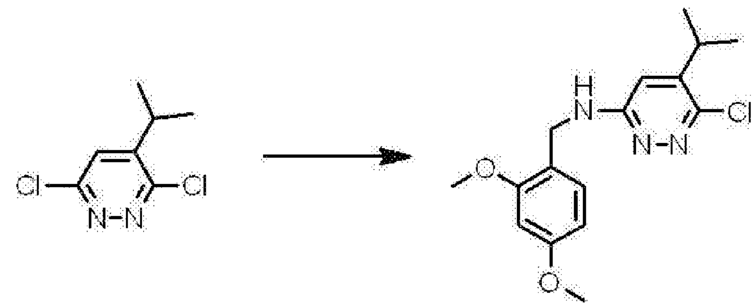

2,4-Dimethoxybenzylamine (10 mL) and 1,8-diazabicyclo[5.4.0]undeca-7-ene (14.9 mL) were added to a solution of 3,6-dichloro-4-isopropylpyridazine (6.33 g) in 1,4-dioxane (66 mL), followed by stirring at 100°C for 19 hours. The reaction mixture was cooled to room temperature, and ethyl acetate was added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining 6-chloro-N-(2,4-dimethoxybenzyl)-5-isopropylpyridazine-3-amine (2.64 g) as pale yellow oily substance.
MSm/z(M+H):322.

<0014-3>

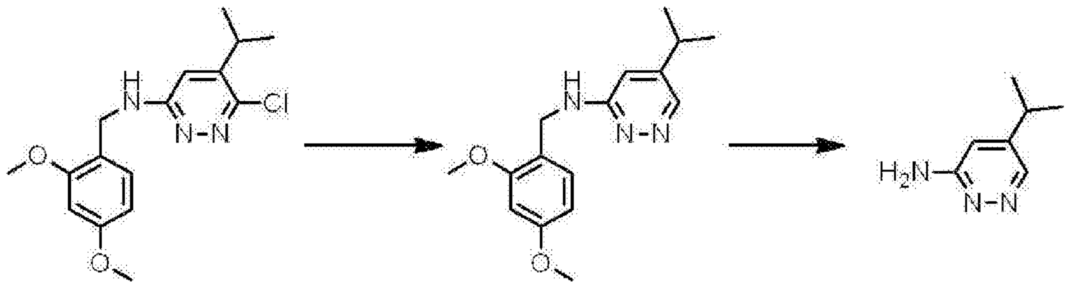

A mixture of 10% palladium/carbon (0.5 g), 6-chloro-N-(2,4-dimethoxybenzyl)-5-isopropylpyridazine-3-amine (2.64 g) and methanol (27.3 mL) in acetic acid (0.94 mL) was stirred at room temperature for 3 hours under pressurized hydrogen (0.8 MPa). Furthermore, acetic acid (3.76 mL) was added thereto, followed by stirring at 50°C for 2.5 hours under pressurized hydrogen (0.8 MPa). The insolubles were filtered off using celite, and the solvent was distilled off under reduced pressure, thereby obtaining orange oily substance (4.33 g).

Water (0.82 mL) and trifluoroacetic acid (8.2 mL) were added to the obtained orange oily substance (4.33 g), followed by stirring at room temperature for 30 minutes. The insolubles were filtered off using celite, and the solid was washed with ethyl acetate/methanol. The filtrate and the washings were combined, and the solvent was distilled off under reduced pressure. The obtained residue was purified by silica gel column chromatography (methanol-ethyl acetate, NH silica), thereby obtaining 5-isopropylpyridazine-3-amine (0.97 g) as a pale yellow solid.

MSm/z(M+H):138.

<0014-4>

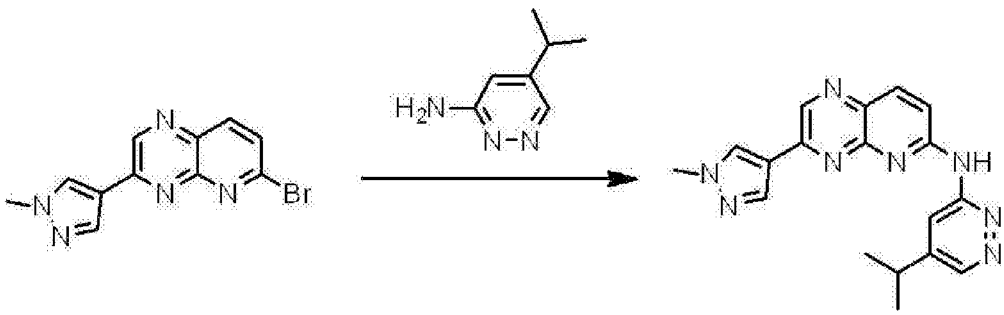

1,4-Dioxane (0.79 mL) was added to a mixture of 6-bromo-3-(1-methyl-1H-pyrazol-4-yl)pyrido[2,3-b]pyrazine (11.4 mg), 5-isopropylpyridazine-3-amine (8.1 mg), tris(dibenzylideneacetone)dipalladium(0) (3.6 mg), 4,5-bis(diphenylphosphino)-9,9-dimethylxanthene (4.5 mg), and cesium carbonate (32 mg), followed by reacting at 150°C for 30 minutes using a microwave reaction apparatus. Ethyl acetate and water were added to the reaction mixture. The organic layer was collected by separation, washed with a saturated sodium chloride aqueous solution, and dried over anhydrous sodium sulfate, and the solvent was distilled off under reduced pressure. The obtained residue was purified by silica gel column chromatography (methanol-ethyl acetate), thereby obtaining N-(5-isopropylpyridazin-3-yl)-3-(1-methyl-1H-pyrazol-4-yl)pyrido[2,3-b]pyrazine-6-amine (1.3 mg) as a yellow solid.
$^1$H-NMR(DMSO-$d_6$)$\delta$:10.93(1H,s),9.13(1H,s),8.91(1H,d,J=2.0Hz),8.69(1H,s),8.60(1H,s), 8.28(2H,m,J=4.6Hz),7.82(1H,d,J=9.2Hz),3.96(3H,s),1.33(6H,d,J=6.6Hz).
MSm/z(M+H):347.

<Example 0015>

<0015-1>

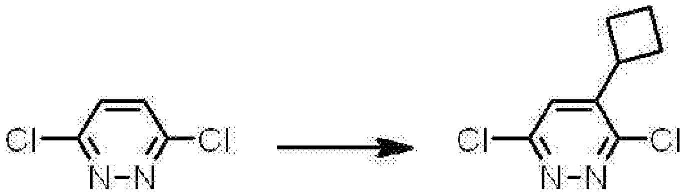

3,6-Dichloro-4-cyclobutylpyridazine (1.92 g) was obtained as pale yellow oily substance in the same manner as in Example 0014 except that cyclobutanecarboxylic acid was used instead of the isobutyric acid used in Example 0014.
MSm/z(M+H):204.

<0015-2>

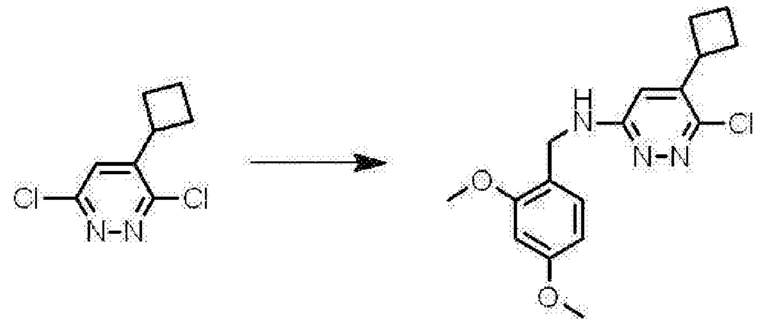

2,4-Dimethoxybenzylamine (1.48 mL) and 1,8-diazabicyclo[5.4.0]undeca-7-ene (2.2 mL) were added to a solution of 3,6-dichloro-4-cyclobutylpyridazine (0.99 g) in 1,4-dioxane (7 mL), followed by reacting at 145°C for 45 minutes using a microwave reaction apparatus. Ethyl acetate and water were added to the reaction mixture. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining 6-chloro-N-(2,4-dimethoxybenzyl)-5-cyclobutylpyridazine-3-amine (274 mg).
MSm/z(M+H):334.

<0015-3>

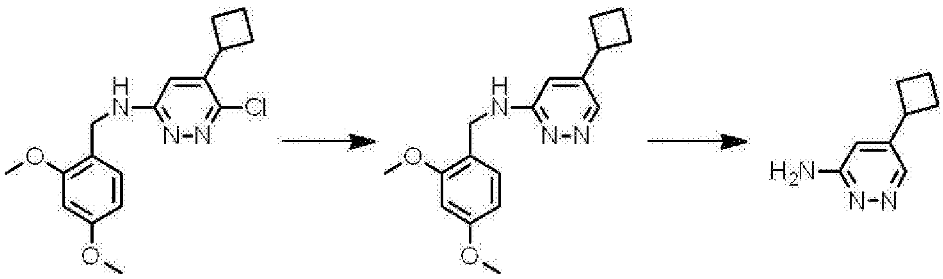

A mixture solution of 6-chloro-N-(2,4-dimethoxybenzyl)-5-cyclobutylpyridazine-3-amine (274 mg) in methanol (20.5 mL)/acetic acid (2 mL) was reacted using a flow-type hydrogenation reaction apparatus (30 bar, 1.0 mL/min, 55°C, 10% Pd/C). The solvent was distilled off under reduced pressure, thereby obtaining pale yellow oily substance.

Water (0.25 mL) and trifluoroacetic acid (5 mL) were added to the obtained pale yellow oily substance, followed by stirring at room temperature for 30 minutes. The insolubles were filtered off using celite, and the solid was washed with ethyl acetate/methanol. The filtrate and the washings were combined, and the solvent was distilled off under reduced pressure. The obtained residue was purified by silica gel column chromatography (methanol-chloroform, NH silica), thereby obtaining 5-cyclobutylpyridazine-3-amine (129 mg) as a pale yellow solid.
MSm/z(M+H):150.

<0015-4>

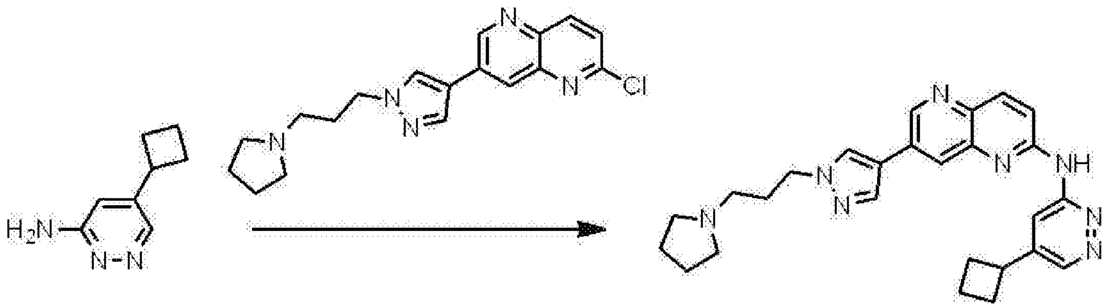

1,4-Dioxane (0.89 mL) was added to a mixture of 2-chloro-7-(1-(3-(pyrrolidin-1-yl)propyl)-1H-pyrazol-4-yl)-1,5-naphthyridine (15.2 mg), 5-cyclobutylpyridazine-3-amine (10.1 mg), tris(dibenzylideneacetone)dipalladium(0) (4.2 mg), 4,5-bis(diphenylphosphino)-9,9-dimethylxanthene (5.3 mg), and cesium carbonate (36.3 mg), followed by reacting at 150°C for 30 minutes using a microwave reaction apparatus. Ethyl acetate and water were added to the reaction mixture. The organic layer was collected by separation, washed with a saturated sodium chloride aqueous solution, and dried over anhydrous sodium sulfate, and the solvent was distilled off under reduced pressure. The obtained residue was purified by silica gel column chromatography (methanol-ethyl acetate, NH silica) and preparative reversed phase HPLC (a 0.1% formic acid aqueous solution-a 0.1% formic acid acetonitrile solution), and the solvent was distilled off under reduced pressure, thereby obtaining N-(5-cyclobutylpyridazin-3-yl)-7-(1-(3-(pyrrolidin-1-yl)propyl)-1H-pyrazol-4-yl)-1,5-naphthyridine-2-amineformate (0.8 mg) as a brown solid.
$^1$H-NMR(DMSO-$d_6$)$\delta$ 10.71(1H,s),9.05(1H,d,J=2.0Hz),8.83(1H,d,J=1.3Hz), 8.69(1H,d,J=1.3Hz),8.52(1H,s),8.21(3H,d,J=2.0Hz),7.70(1H,d,J=9.2Hz),4.24(2H,t,J=6.6Hz),3.67(1H,t ,J=8.6Hz),2.71( 8H,d,J=9.2Hz),2.47-2.41(2H,m),2.24(2H,m,J=9.1,2.2Hz),2.11-2.05(4H,m),1.78-1.75(2H,m).
MSm/z(M+H):455.

<Example 0016>

<0016-1>

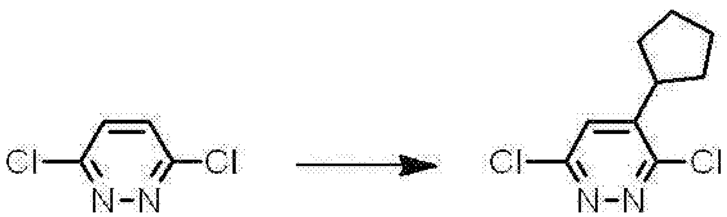

3,6-Dichloro-4-cyclopentylpyridazine (1.96 g) was obtained as pale yellow oily substance in the same manner as in Example 0014 except that cyclopentanecarboxylic acid was used instead of the isobutyric acid used in Example 0014.
MSm/z(M+H):217.

<0016-2>

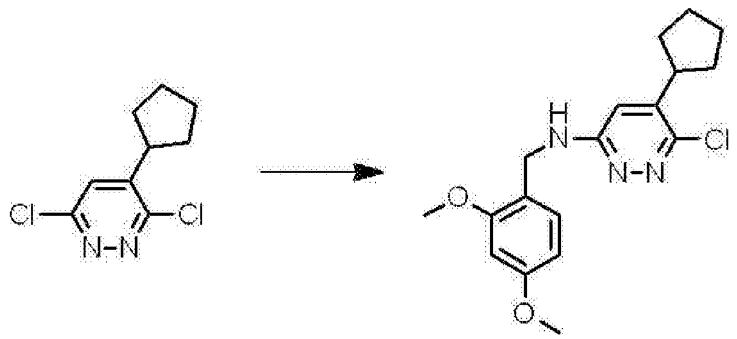

6-Chloro-N-(2,4-dimethoxybenzyl)-5-cyclopentylpyridazine-3-amine (391 mg) was obtained in the same manner as in Example 0015 except that 3,6-dichloro-4-cyclopentylpyridazine was used instead of the 3,6-dichloro-4-cyclobutylpyridazine used in Example 0015.
MSm/z(M+H):348.

<0016-3>

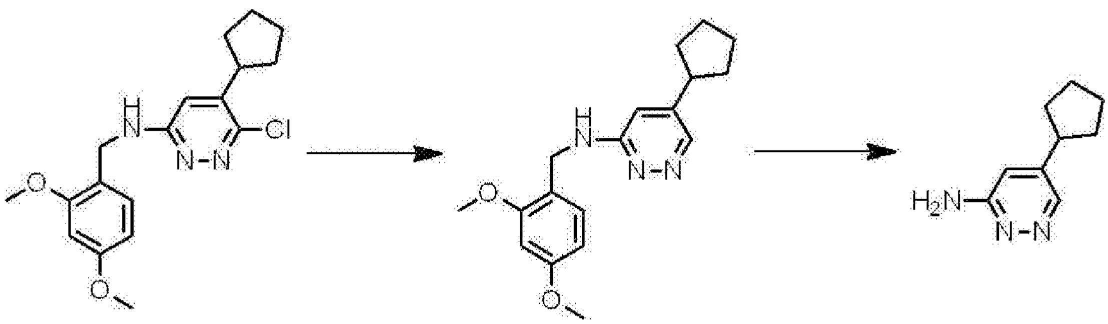

5-Cyclopentylpyridazine-3-amine (153 mg) was obtained as a pale yellow solid in the same manner as in Example 0015 except that 6-chloro-N-(2,4-dimethoxybenzyl)-5-cyclopentylpyridazine-3-amine was used instead of the 6-chloro-N-(2,4-dimethoxybenzyl)-5-cyclobutylpyridazine-3-amine used in Example 0015, and the hydrogen pressure during the reaction was set to 20 bar.
MSm/z(M+H):164.

<0016-4>

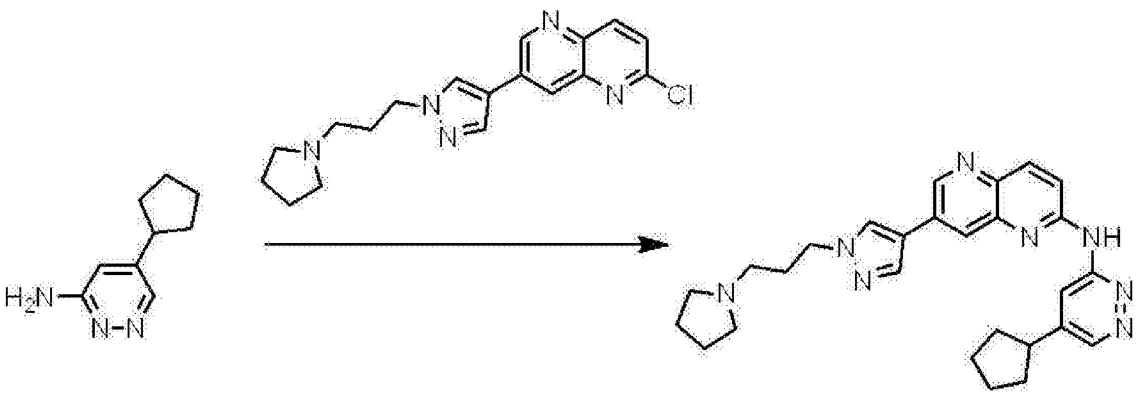

N-(5-cyclopentylpyridazin-3-yl)-7-(1-(3-(pyrrolidin-1-yl)propyl)-1H-pyrazol-4-yl)-1,5-naphthyridine-2-amine

(2.8 mg) was obtained as a pale yellow solid in the same manner as in Example 0015 except that 5-cyclopentylpyridazine-3-amine was used instead of the 5-cyclobutylpyridazine-3-amine used in Example 0015.
$^{1}$H-NMR(DMSO-$d_6$)δ:10.69(1H,s),9.04(1H,d,J=2.0Hz),8.84(1H,d,J=2.0Hz), 8.70(1H,d,J=2.0Hz),8.50(1H,s),8.23-8.19(3H,m),7.71(1H,d,J=8.6Hz),4.23(2H,t,J=6.9Hz), 3.12(1H,t,J=7.9Hz),2.55-2.52(6H,m),2.17-1.63(14H,m).
MSm/z(M+H):469.

<Example 0017>

<0017-1>

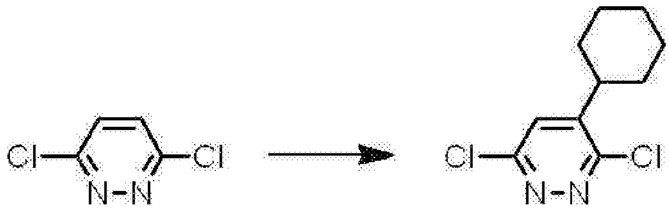

3,6-Dichloro-4-cyclohexylpyridazine (2.27 g) was obtained as a pale yellow solid in the same manner as in Example 0014 except that cyclohexanecarboxylic acid was used instead of the isobutyric acid used in Example 0014.
MSm/z(M+H):231.

<0017-2>

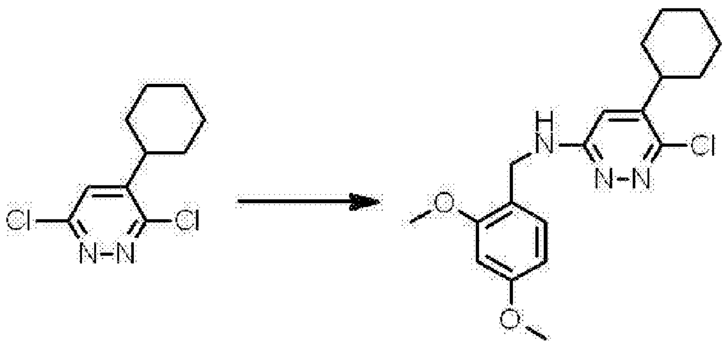

6-Chloro-N-(2,4-dimethoxybenzyl)-5-cyclohexylpyridazine-3-amine (361 mg) was obtained in the same manner as in Example 0015 except that 3,6-dichloro-4-cyclohexylpyridazine was used instead of the 3,6-dichloro-4-cyclobutylpyridazine used in Example 0015.
MSm/z(M+H):362.

<0017-3>

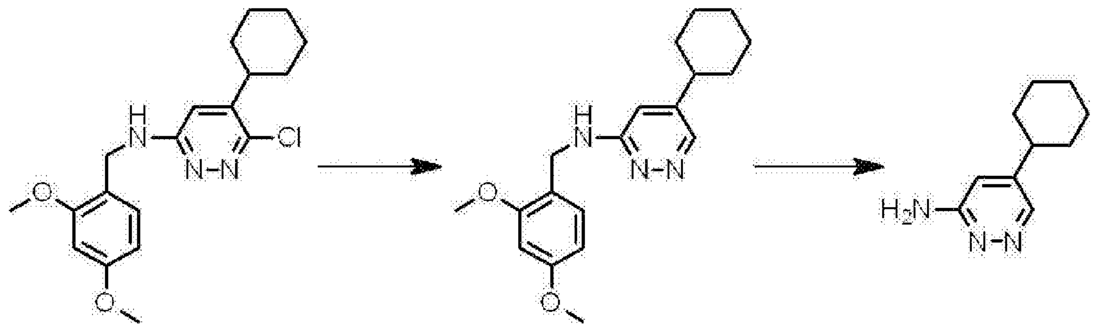

5-Cyclohexylpyridazine-3-amine (153 mg) was obtained as a white solid in the same manner as in Example 0016 except that 6-chloro-N-(2,4-dimethoxybenzyl)-5-cyclohexylpyridazine-3-amine was used instead of the 6-chloro-N-(2,4-dimethoxybenzyl)-5-cyclopentylpyridazine-3-amine used in Example 0016.
MSm/z(M+H):178.

<0017-4>

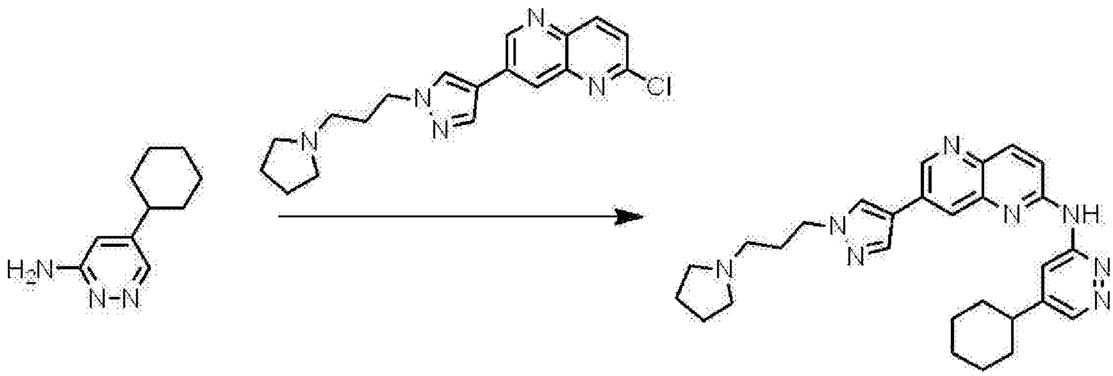

N-(5-cyclohexylpyridazin-3-yl)-7-(1-(3-(pyrrolidin-1-yl)propyl)-1H-pyrazol-4-yl)-1,5-naphthyridine-2-amine (2.8 mg) was obtained as a yellow solid in the same manner as in Example 0015 except that 5-cyclohexylpyridazine-3-amine was used instead of the 5-cyclobutylpyridazine-3-amine used in Example 0015.
$^1$H-NMR($CDCl_3$)δ:8.93(1H,d,J=2.0Hz),8.75(1H,brs),8.48(1H,s),8.24(1H,d,J=9.2Hz), 8.13(1H,d,J=2.0Hz),7.96(2H,d,J=5.9Hz),7.54(1H,d,J=9.2Hz),4.36(2H,t,J=6.6Hz),3.10-3.06(3H,m),2.96(2H,t,J=7.6Hz), 2.62(2H,s),2.45-2.35(2H,m),2.25-1.82(14H,m), 1.50-1.37(3H,m).
MSm/z(M+H):483.

<Example 0018>

<0018-1>

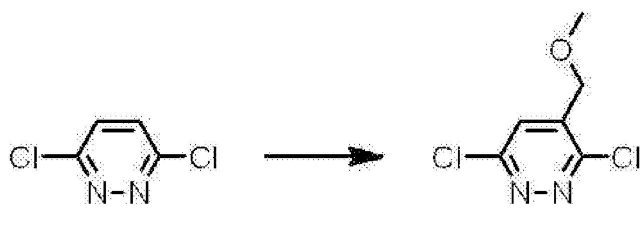

3,6-Dichloro-4-methoxymethylpyridazine (487 mg) was obtained as a white solid in the same manner as in Example 0014 except that methoxyacetic acid was used instead of the isobutyric acid used in Example 0014.

MSm/z(M+H):193.

<0018-2>

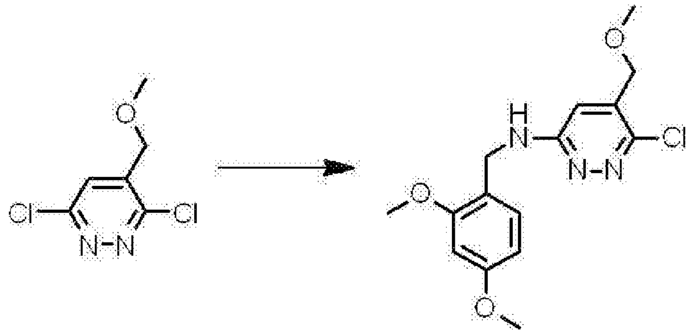

6-Chloro-N-(2,4-dimethoxybenzyl)-5-(methoxymethyl)pyridazine-3-amine (110 mg) was obtained in the same manner as in Example 0015 except that 3,6-dichloro-4-methoxymethylpyridazine was used instead of the 3,6-dichloro-4-cyclobutylpyridazine used in Example 0015.
MSm/z(M+H):324.

<0018-3>

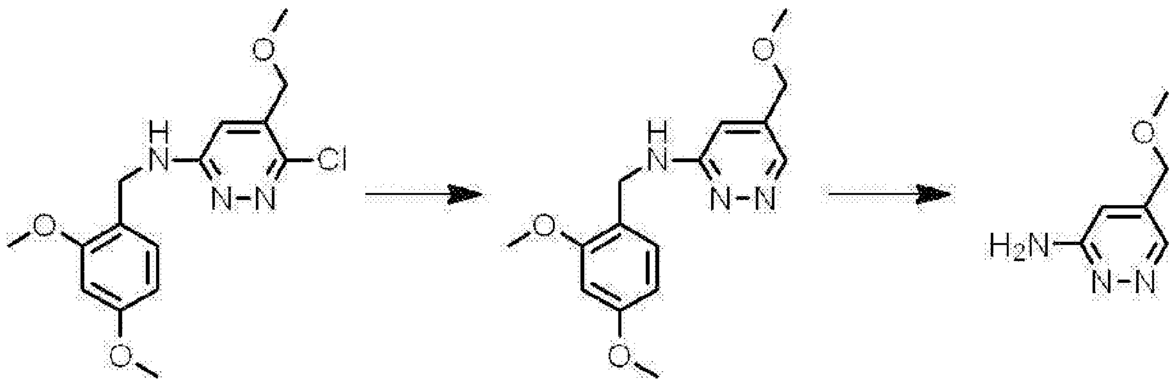

5-(Methoxymethyl)pyridazine-3-amine (29 mg) was obtained in the same manner as in Example 0016 except that 6-chloro-N-(2,4-dimethoxybenzyl)-5-(methoxymethyl)pyridazine-3-amine was used instead of the 6-chloro-N-(2,4-dimethoxybenzyl)-5-cyclopentylpyridazine-3-amine used in Example 0016.
MSm/z(M+H):140.

<0018-4>

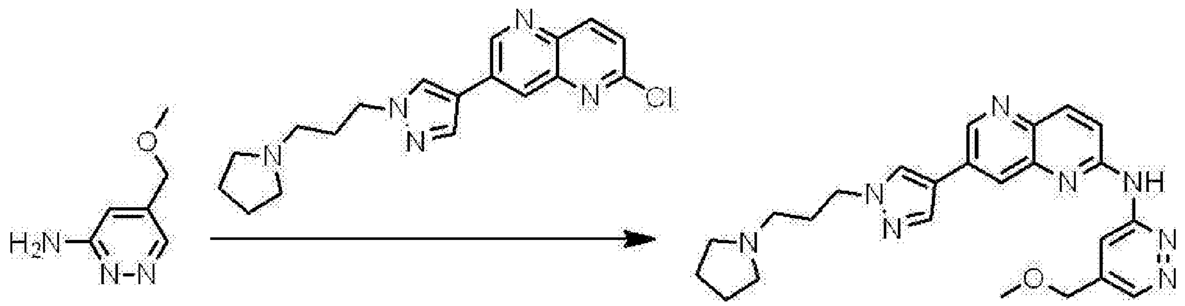

1,4-Dioxane (0.83 mL) was added to a mixture of 2-chloro-7-(1-(3-(pyrrolidin-1-yl)propyl)-1H-pyrazol-4-yl)-1,5-naphthyridine (14.2 mg), 5-(methoxymethyl)pyridazine-3-amine (5.8 mg), tris(dibenzylideneacetone)dipalladium(0) (3.8 mg), 4,5-bis(diphenylphosphino)-9,9-dimethylxanthene (4.8 mg), and cesium carbonate (33.8 mg), followed by reacting at 150°C for 30 minutes using a microwave reaction apparatus. Ethyl acetate and water were added to the reaction mixture. The organic layer was collected by separation, washed with a saturated sodium chloride aqueous solution, and dried over anhydrous sodium sulfate, and the solvent was distilled off under reduced pressure. The obtained residue was purified by silica gel column chromatography (methanol-ethyl acetate, NH silica), thereby obtaining N-(5-(methoxymethyl)pyridazin-3-yl)-7-(1-(3-(pyrrolidin-1-yl)propyl)-1H-pyrazol-4-yl)-1,5-naphthyridine-2-amine (1.1 mg) as a pale yellow solid.
$^1$H-NMR(DMSO-$d_6$)$\delta$:10.81(1H,s),9.05(1H,d,J=2.0Hz),8.83(1H,d,J=2.0Hz),8.79(1H,s),
8.51(1H,s),8.23(2H,m),8.18(1H,s),7.70(1H,d,J=9.2Hz),4.61(2H,s),4.22(2H,t,J=6.9Hz),
3.44(3H,s),2.41-2.39(6H,m),2.01(2H,t,J=6.9Hz),1.71-1.67(4H,m).
MSm/z(M+H):445.

<Example 0019>

<0019-1>

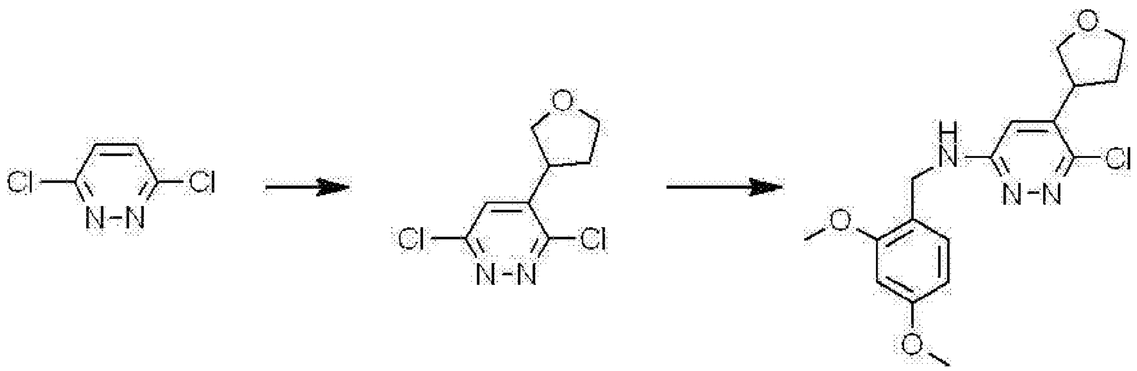

Brown oily substance (1.54 g) was obtained in the same manner as in Example 0014 except that tetrahydrofuran-3-carboxylic acid was used instead of the isobutyric acid used in Example 0014.

2,4-Dimethoxybenzylamine (2.1 mL) and 1,8-diazabicyclo[5.4.0]undeca-7-ene (3.2 mL) were added to a solution of the obtained brown oily substance (1.54 g) in 1,4-dioxane (14 mL), followed by stirring at 100°C overnight. Ethyl acetate and water were added to the reaction mixture. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining 6-chloro-N-(2,4-dimethoxybenzyl)-5-(tetrahydrofuran-3-yl)pyridazine-3-amine (348 mg) as an orange solid.
MSm/z(M+H):350.

<0019-2>

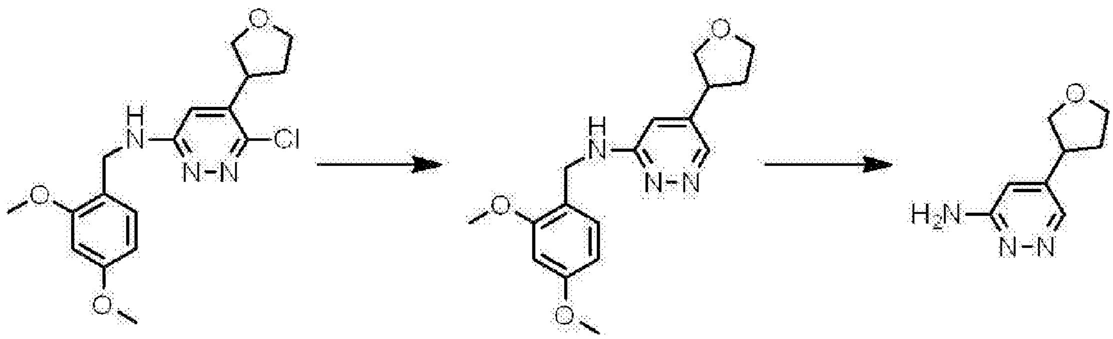

5-(Tetrahydrofuran-3-yl)pyridazine-3-amine (116 mg) was obtained as a pale orange solid in the same manner as in Example 0016 except that 6-chloro-N-(2,4-dimethoxybenzyl)-5-(tetrahydrofuran-3-yl)pyridazine-3-amine was used instead of the 6-chloro-N-(2,4-dimethoxybenzyl)-5-cyclopentylpyridazine-3-amine used in Example 0016.
MSm/z(M+H):166.

<0019-3>

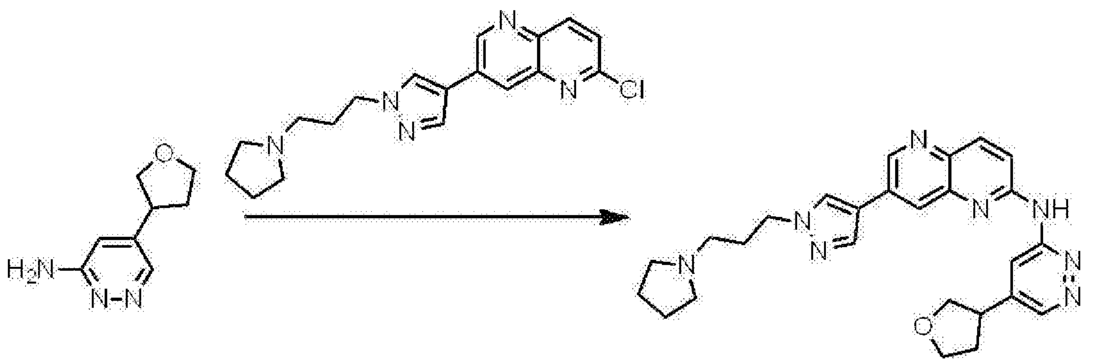

7-(1-(3-(Pyrrolidin-1-yl)propyl)-1H-pyrazol-4-yl)-N-(5-(tetrahydrofuran-3-yl)pyridazin-3-yl)-1,5-naphthyridine-2-amine (1.1 mg) was obtained as an orange solid in the same manner as in Example 0018 except that 5-(tetrahydrofuran-3-yl)pyridazine-3-amine was used instead of the 5-(methoxymethyl)pyridazine-3-amine used in Example 0018.
$^1$H-NMR($CDCl_3$)$\delta$:8.96(2H,m),8.84(1H,d,J=2.0Hz),8.25(1H,d,J=9.2Hz),8.11(1H,d,J=2.0Hz),7.97(1H,s),7.89(1H,s),7.51(1H,d,J=8.6Hz),4.29-4.24(4H,m),4.07-3.93(3H,m),2.57-2.47(7H,m),2.16-2.13(3H,m),1.82-1.80(4H,m).
MSm/z(M+H):471.

<Example 0020>

<0020-1>

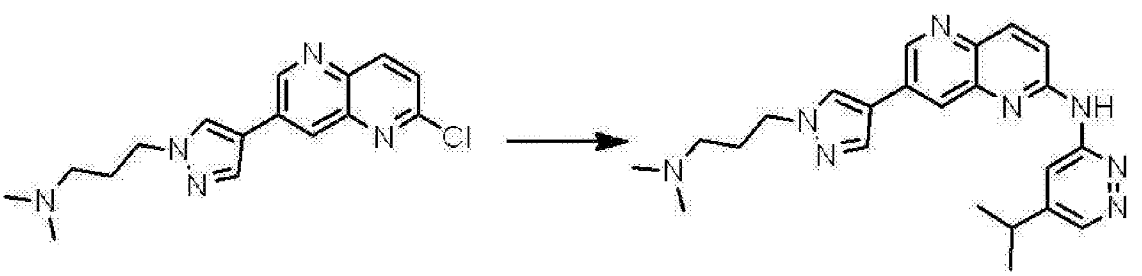

7-(1-(3-(Dimethylamino)propyl)-1H-pyrazol-4-yl)-N-(5-isopropylpyridazin-3-yl)-1,5-naphthyridine-2-amine (0.7 mg) was obtained as an orange solid in the same manner as in Example 0018 except that 3-(4-(6-chloro-1,5-naphthyridin-3-yl)-1H-pyrazol-1-yl)-N,N-dimethylpropane-1-amine was used instead of the 2-chloro-7-(-(3-(pyrrolidin-1-yl)propyl)-1H-pyrazol-4-yl)-1,5-naphthyridine used in Example 0018, and 5-isopropylpyridazine-3-amine was used instead of the 5-(methoxymethyl)pyridazine-3-amine used in Example 0018.
$^1$H-NMR($CDCl_3$)$\delta$:8.93-8.81(3H,m),8.24(1H,d,J=8.6Hz),8.10(1H,d,J=1.3Hz),7.97(1H,s), 7.90(1H,s),7.53(1H,d,J=8.6Hz),5.34(1H,t,J=5.6Hz),4.31(3H,t,J=6.9Hz),3.45(1H,s),3.06-3.03(1H,m),2.41-2.37(2H,m),2.32(6H,s),1.42(6H,d,J=7.3Hz).
MSm/z(M+H):417.

<Example 0021>

<0021-1>

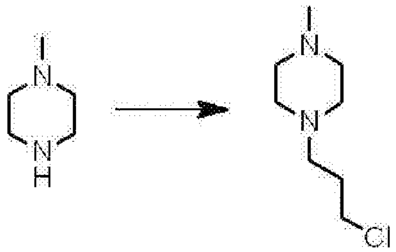

1-Bromo-3-chloropropane (0.25 mL) was added to a solution of 1-methylpiperazine (0.55 mL) in toluene (2.5 mL), followed by stirring at 80°C for 2 hours. The reaction mixture was cooled to room temperature, and 2 mol/L hydrochloric acid was added thereto. The aqueous layer was collected by separation, adjusted to have a pH of 12 by the addition of a 2 mol/L sodium hydroxide aqueous solution, and ethyl acetate was added thereto. The organic layer was collected by separation, washed with a saturated sodium chloride aqueous solution, and dried over anhydrous sodium sulfate, and the solvent was distilled off under reduced pressure, thereby obtaining 1-(3-chloropropyl)-4-methylpiperazine (187 mg) as a white solid.
$^1$H-NMR($CDCl_3$)$\delta$:3.59(2H,t,J=6.6Hz),2.49(8H,t,J=7.3Hz),2.29(3H,s),1.95(2H,t,J=6.9Hz).

<0021-2>

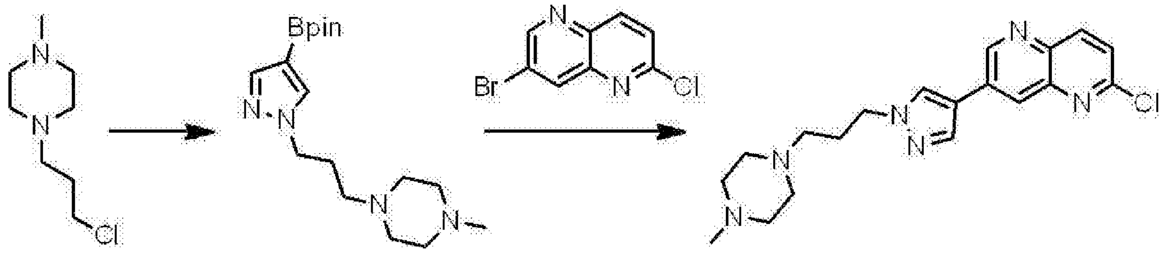

4-(4,4,5,5-Tetramethyl-1,3,2-dioxaborolan-2-yl)-1H-pyrazole (351.4 mg), cesium carbonate (1.17 g), and sodium iodide (58.0 mg) were added to a mixture solution of 1-(3-chloropropyl)-4-methylpiperazine (0.64 g) in acetonitrile (2.4 mL)/tetrahydrofuran (1.0 mL), followed by reacting at 80°C for 19 hours. After the reaction mixture was cooled to room temperature, the insolubles were filtered off, and the residue was washed with ethyl acetate. The filtrate and the washings were combined, and the solvent was distilled off under reduced pressure, thereby obtaining orange oily substance (137 mg).
7-Bromo-2-chloro-1,5-naphthyridine (50 mg), bis(di-tert-butyl (4-dimethylaminophenyl)phosphine)dichloropalladium(II) (14.5 mg), and sodium carbonate (43.4 mg) were added to a mixture solution of the obtained orange oily substance (137 mg) in 1,4-dioxane (2.0 mL)/water (0.2 mL), followed by stirring at 100°C for 2 hours. After the reaction

mixture was cooled to room temperature, the insolubles were filtered off using celite, and the residue was washed with ethyl acetate. The filtrate and the washings were combined, and the solvent was distilled off under reduced pressure. The obtained residue was purified by silica gel column chromatography (ethyl acetate-hexane, methanol-ethyl acetate, NH silica), thereby obtaining 2-chloro-7-(1-(3-(4-methylpiperazin-1-yl)propyl)-1H-pyrazol-4-yl)-1,5-naphthyridine (44.1 mg) as a pale yellow solid.
MSm/z(M+H):371.

<0021-3>

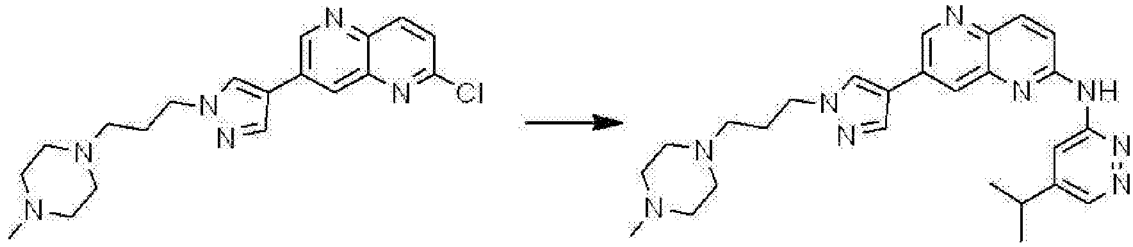

N-(5-isopropylpyridazin-3-yl)-7-(1-(3-(4-methylpiperazin-1-yl)propyl)-1H-pyrazol-4-yl)-1,5-naphthyridine-2-amine (2.5 mg) was obtained as a pale yellow solid in the same manner as in Example 0018 except that 2-chloro-7-(1-(3-(4-methylpiperazin-1-yl)propyl)-1H-pyrazol-4-yl)-1,5-naphthyridine was used instead of the 2-chloro-7-(1-(3-(pyrrolidin-1-yl)propyl)-1H-pyrazol-4-yl)-l,5-naphthyridine used in Example 0018, and 3-amino-5-isopropylpyridazine was used instead of the 3-amino-5-methoxymethylpyridazine used in Example 0018.
$^1$H-NMR($CDCl_3$)δ:8.93(1H,d,J=2.0Hz),8.83-8.81(2H,m),8.24(1H,d,J=9.2Hz),8.10(1H,s),
7.97(1H,s),7.87(1H,s),7.52(1H,d,J=9.2Hz),4.29(2H,t,J=6.6Hz),3.06(1H,t,J=6.9Hz),2.51-2.47(7H,m),2.38(3H,t,J=6.9Hz),2.31(3H,s),2.13(2H,q,J=6.8Hz),1.42(6H,d,J=7.3Hz).
MSm/z(M+H):472.

<Example 0022>

<0022-1>

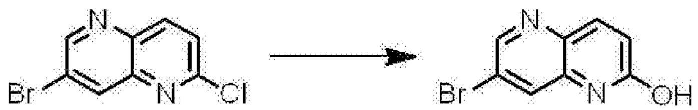

Several drops of a 4 mol/L hydrogen chloride-1,4-dioxane solution (3 mL) and water were added to 7-bromo-2-chloro-1,5-naphthyridine (250 mg), followed by stirring at 100°C overnight. The reaction mixture was cooled to room temperature, and water was added thereto. The solid matter was collected by filtration, and washed with a mixture solution of water and hexane-ethyl acetate (1:1), thereby obtaining 7-bromo-1,5-naphthyridin-2-ol (190 mg) as a grey solid.
$^1$H-NMR(DMSO-$d_6$)δ:11.96(1H,brs),8.56(1H,d,J=2.3Hz),7.93(1H,d,J=9.9Hz),
7.85(1H,d,J=2.30Hz),6.79(1H,d,J=9.9Hz).

<0022-2>

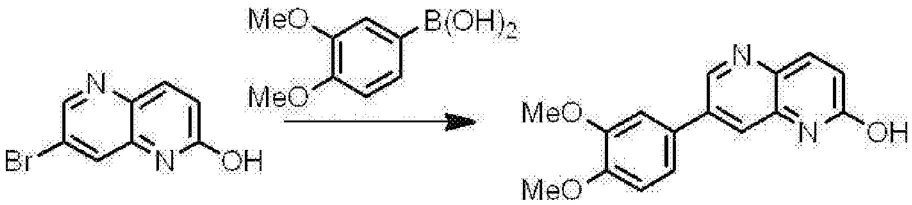

1,4-Dioxane (5 mL), 2 mol/L sodium carbonate aqueous solution (0.99 mL), and bis(di-tert-butyl (4-dimethylaminophenyl)phosphine)dichloropalladium(II) (46 mg) were added to a mixture of 7-bromo-1,5-naphthyridin-2-ol (150 mg) and 3,4-dimethoxyphenylboronic acid (150 mg) in a nitrogen atmosphere, followed by stirring at 100°C for 2 days. The reaction mixture was cooled to room temperature, a mixed solvent of chloroform-methanol (10:1) was added thereto, and the solid matter was collected by filtration, thereby obtaining 7-(3,4-dimethoxyphenyl)-1,5-naphthyridin-2-ol (137 mg) as a yellow solid.
MSm/z(M+H):283.

<0022-3>

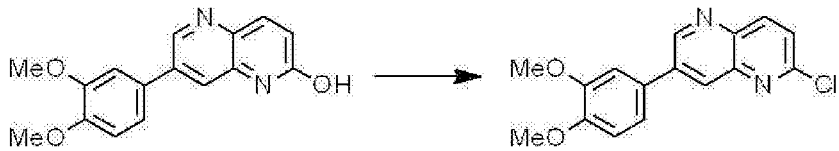

Phosphorus oxychloride (4 g) was added to 7-(3,4-dimethoxyphenyl)-1,5-naphthyridin-2-ol (134 mg), followed by stirring at 100°C for 2 hours. After the reaction mixture was cooled to room temperature, water was added dropwise thereto, the resultant product was neutralized with a 5 mol/L sodium hydroxide aqueous solution, and chloroform was added thereto. The organic layer was collected by separation, and dried over anhydrous sodium sulfate, and the solvent was distilled off under reduced pressure. The obtained residue was purified by silica gel column chromatography (hexane-ethyl acetate), thereby obtaining 2-chloro-7-(3,4-dimethoxyphenyl)-1,5-naphthyridine (134 mg) as an orange solid.
$^1$H-NMR($CDCl_3$)δ:9.24(1H,d,J=2.6Hz),8.43(1H,d,J=2.6Hz),8.37(1H,d,J=9.2Hz),7.60(1H,d, J=9.2Hz),7.33(1H,dd,J=8.3,2.1Hz),7.24(1H,d,J=2.1Hz),7.05(1H,d,J=8.3Hz),3.99(3H,s),3.97 (3H,s).

<0022-4>

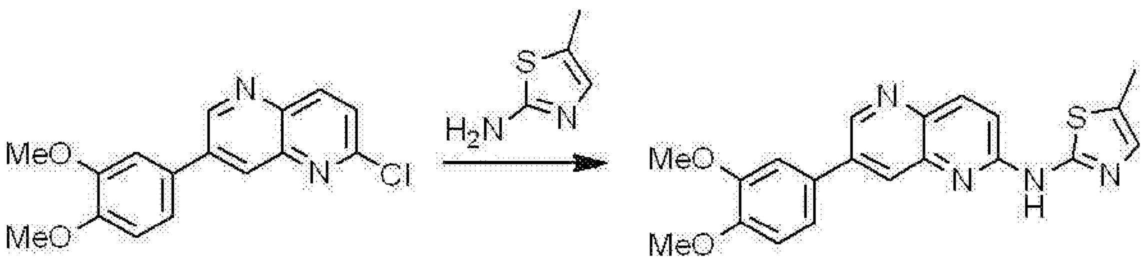

1,4-Dioxane (2 mL) was added to a mixture of 2-chloro-7-(3,4-dimethoxyphenyl)-1,5-naphthyridine (30 mg), 5-methylthiazole-2-amine (17 mg), tris(dibenzylideneacetone)dipalladium(0) (9.2 mg), 4,5-bis(diphenylphosphino)-9,9-dimethylxanthene (12 mg), and cesium carbonate (98 mg) in a nitrogen atmosphere, and the reaction vessel was sealed, followed by stirring at 100°C overnight. The reaction mixture was cooled to room temperature, and water was added thereto. The solid matter was collected by filtration, and purified by silica gel column chromatography (chloroform-methanol), thereby obtaining N-(7-(3,4-dimethoxyphenyl)-1,5-naphthyridin-2-yl)-5-methylthiazole-2-amine (32 mg) as a yellow solid.
$^1$H-NMR($CDCl_3$)δ:10.58(1H,brs),9.00(1H,d,J=2.0Hz),8.35(1H,d,J=2.0Hz), 8.25(1H,d,J=8.6Hz),7.35(1H,dd,J=8.6,2.0Hz),7.30-7.27(1H,m),7.24-7.18(2H,m),

7.05(1H,d,J=8.6Hz),4.03(3H,s),3.98(3H,s),2.51(3H,s).
MSm/z(M+H):379.

<Example 0023>

<0023-1>

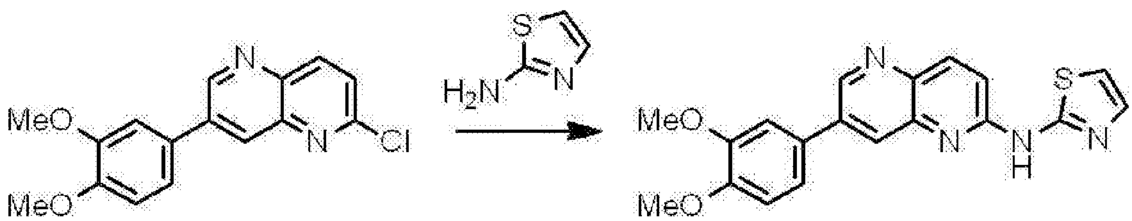

1,4-Dioxane (2 mL) was added to a mixture of 2-chloro-7-(3,4-dimethoxyphenyl)-1,5-naphthyridine (30 mg), thiazole-2-amine (15 mg), tris(dibenzylideneacetone)dipalladium(0) (9.2 mg), 4,5-bis(diphenylphosphino)-9,9-dimethylxanthene (12 mg), and cesium carbonate (98 mg) in a nitrogen atmosphere, and the reaction vessel was sealed, followed by stirring at 100°C overnight. The reaction mixture was cooled to room temperature, and water was added thereto. The solid matter was collected by filtration, and purified by silica gel column chromatography (chloroform-methanol), thereby obtaining N-(7-(3,4-dimethoxyphenyl)-1,5-naphthyridin-2-yl)thiazole-2-amine (10 mg) as a yellow solid.
$^1$H-NMR($CDCl_3$)δ:10.60(1H,brs),9.02(1H,d,J=2.0Hz),8.36(1H,d,J=2.0Hz),
8.28(1H,d,J=9.2Hz),7.59(1H,d,J=3.7Hz),7.36-7.22(3H,m),7.05(1H,d,J=7.9Hz),
7.00(1H,d,J=3.7Hz),4.03(3H,s),3.98(3H,s).
MSm/z(M+H):365.

<Example 0024>

<0024-1>

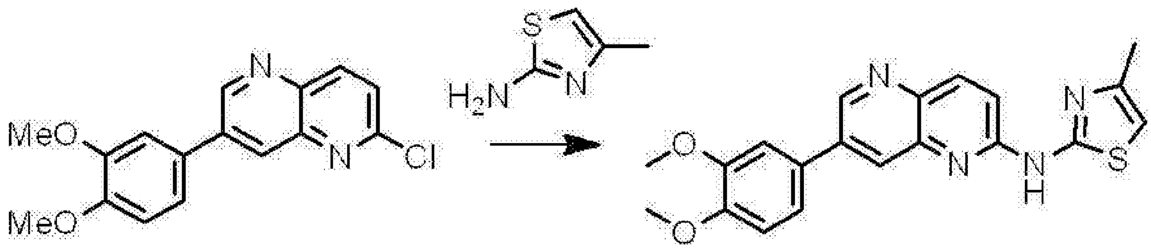

1,4-Dioxane (2 mL) was added to a mixture of 2-chloro-7-(3,4-dimethoxyphenyl)-1,5-naphthyridine (30 mg), 4-methylthiazole-2-amine (17 mg), tris(dibenzylideneacetone)dipalladium(0) (9.2 mg), 4,5-bis(diphenylphosphino)-9,9-dimethylxanthene (12 mg), and cesium carbonate (98 mg) in a nitrogen atmosphere, and the reaction vessel was sealed, followed by stirring at 100°C overnight. The reaction mixture was cooled to room temperature, water was added thereto, and ethyl acetate was added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (chloroform-methanol), thereby obtaining N-(7-(3,4-dimethoxyphenyl)-1,5-naphthyridin-2-yl)-4-methylthiazole-2-amine (10 mg) as a yellow solid.
$^1$H-NMR($CDCl_3$)δ:9.01(1H,s),8.33(1H,s),8.24(1H,d,J=8.3Hz),7.33(1H,d,J=8.3Hz),
7.13(1H,d,J=8.6Hz),7.04(1H,d,J=8.6Hz),6.52(1H,s),6.09(1H,s),4.02(3H,s),3.97(4H,s), 2.40(3H,s).
MSm/z(M+H):379.

<Example 0025>

<0025-1>

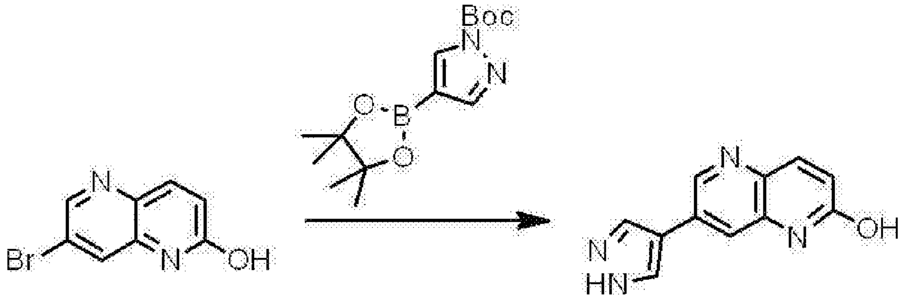

A2 mol/L sodium carbonate aqueous solution (1.3 mL) and 1,4-dioxane (6.0 mL) were added to a mixture of 7-bromo-1,5-naphthyridin-2-ol (190 mg), tert-butyl 4-(4,4,5,5-tetramethyl-1,3,2-dioxaborolan-2-yl)-1H-pyrazole-1-carboxylate (376 mg), and bis(di-tert-butyl (4-dimethylaminophenyl)phosphine)dichloropalladium(II) (60 mg) in a nitrogen atmosphere, followed by stirring at 100°C overnight. The reaction mixture was cooled to room temperature, and water was added thereto. The solid matter was collected by filtration, thereby obtaining 7-(1H-pyrazol-4-yl)-1,5-naphthyridin-2-ol (190 mg) as a grey solid.
$^1$H-NMR(DMSO-$d_6$)$\delta$:13.23(1H,brs),11.84(1H,brs),8.79(1H,d,J=2.0Hz),8.20(2H,brs), 7.90(1H,d,J=9.9Hz),7.73(1H,d,J=2.0Hz),6.66(1H,d,J=9.9Hz).

<0025-2>

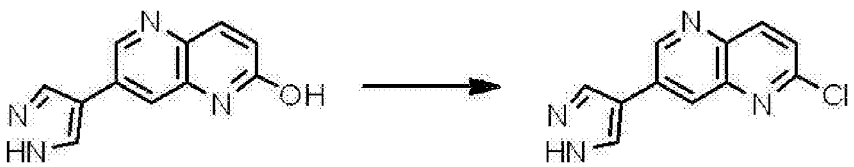

Phosphorus oxychloride (1.7 g) was added to 7-(1H-pyrazol-4-yl)-1,5-naphthyridin-2-ol (50 mg) at room temperature, followed by stirring at 80°C for 30 minutes, and stirring at 100°C for 1.5 hours. The reaction mixture was cooled to room temperature, water was added thereto, and the resultant product was neutralized with a 5 mol/L sodium hydroxide aqueous solution. The solid matter was collected by filtration, thereby obtaining 2-chloro-7-(1H-pyrazol-4-yl)-1,5-naphthyridine (40 mg) as a grey solid.
MSm/z(M+H):231,233.

<0025-3>

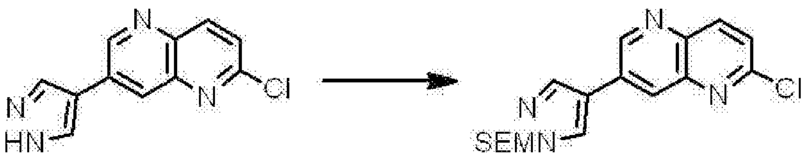

N,N-dimethylformamide (2 mL) was added to 2-chloro-7-(1H-pyrazol-4-yl)-1,5-naphthyridine (15 mg) in a nitrogen atmosphere, and 60% sodium hydride (3 mg) was added thereto under ice-cooling, followed by stirring at the same temperature for 30 minutes. (2-(Chloromethoxy)ethyl)trimethylsilane (14 $\mu$L) was added to the reaction mixture under ice-cooling, followed by stirring at room temperature for 1 hour. Water and ethyl acetate were added to the reaction

mixture. The organic layer was collected by separation, washed with water and a saturated sodium chloride aqueous solution, and dried over anhydrous sodium sulfate, and the solvent was distilled off under reduced pressure. The obtained residue was purified by preparative thin layer silica gel chromatography (hexane-ethyl acetate), thereby obtaining 2-chloro-7-(1-((2-(trimethylsilyl)ethoxy)methyl)-1H-pyrazol-4-yl)-1,5-naphthyridine (6 mg) as a white solid.
MSm/z(M+H):361,363.

<0025-4>

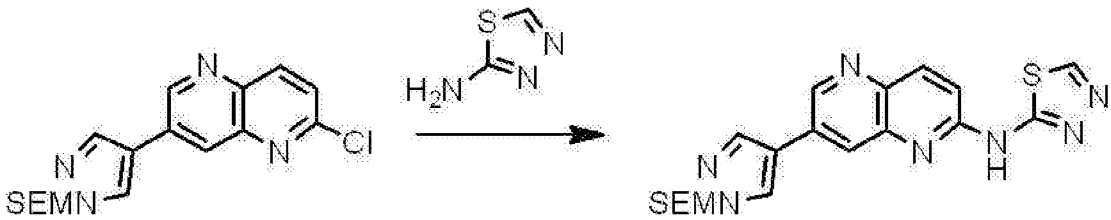

1,4-Dioxane (1.5 mL) was added to a mixture of 2-chloro-7-(1-((2-(trimethylsilyl)ethoxy)methyl)-1H-pyrazol-4-yl)-1,5-naphthyridine (6 mg), 1,3,4-thiadiazole-2-amine (3.2 mg), tris(dibenzylideneacetone)dipalladium(0) (3.0 mg), 4,5-bis(diphenylphosphino)-9,9-dimethylxanthene (4 mg), and cesium carbonate (17 mg) in a nitrogen atmosphere, and the reaction vessel was sealed, followed by stirring at 150°C for 30 minutes using a microwave reaction apparatus. The reaction mixture was cooled to room temperature, and water and ethyl acetate were added thereto. The organic layer was collected by separation, washed with a saturated sodium chloride aqueous solution, and dried over anhydrous sodium sulfate, and the solvent was distilled off under reduced pressure. The obtained residue was purified by preparative thin layer silica gel chromatography (chloroform-methanol), thereby obtaining N-(7-(1-((2-(trimethylsilyl)ethoxy)methyl)-1H-pyrazol-4-yl)-1,5-naphthyridin-2-yl)-1,3,4-thiadiazole-2-amine (4 mg) as a white solid.
$^1$H-NMR($CDCl_3$)δ:13.10(1H,brs),8.98(1H,d,J=2.0Hz),8.85(1H,s),8.35(1H,d,J=9.2Hz),
8.28(1H,d,J=2.0Hz),8.06(1H,s),8.03(1H,s),7.80(1H,d,J=9.2Hz),5.53(2H,s),3.68-3.63(2H,m),0.99-0.94(2H,m),0.01(9H, s).
MSm/z(M+H):426.

<Example 0026>

<0026-1>

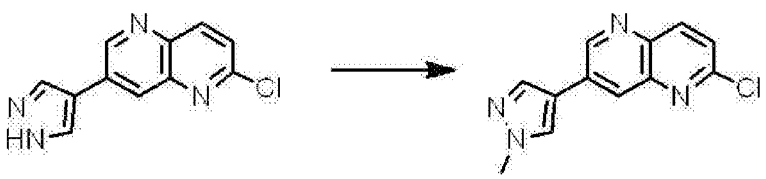

2-Chloro-7-(1H-pyrazol-4-yl)-1,5-naphthyridine (65 mg) was dissolved inN,N-dimethylformamide in a nitrogen atmosphere, and 60% sodium hydride (17 mg) was added thereto under ice-cooling, followed by stirring for 30 minutes under ice-cooling. Methyl iodide (36 μL) was added to the reaction mixture, followed by stirring at room temperature for 50 minutes. Water and ethyl acetate were added to the reaction mixture. The organic layer was collected by separation, washed with water and a saturated sodium chloride aqueous solution, and dried over anhydrous sodium sulfate, and the solvent was distilled off under reduced pressure. The obtained residue was purified by silica gel column chromatography (chloroform-methanol), thereby obtaining 2-chloro-7-(1-methyl-1H-pyrazol-4-yl)-1,5-naphthyridine (20 mg) as a pale yellow solid.
MSm/z(M+H):245,247.

<0026-2>

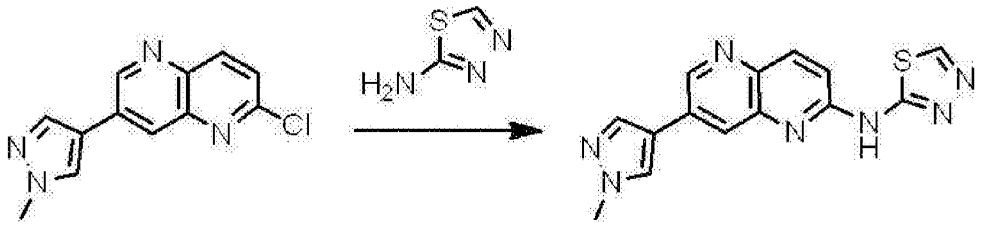

1,4-Dioxane (2 mL) was added to a mixture of 2-chloro-7-(1-methyl-1H-pyrazol-4-yl)-1,5-naphthyridine (20 mg), 1,3,4-thiadiazole-2-amine (16 mg), tris(dibenzylideneacetone)dipalladium(0) (7.3 mg), 4,5-bis(diphenylphosphino)-9,9-dimethylxanthene (9.2 mg), and cesium carbonate (78 mg) in a nitrogen atmosphere, and the reaction vessel was sealed, followed by stirring at 150°C for 30 minutes using a microwave reaction apparatus. The reaction mixture was cooled to room temperature, and water, chloroform, and methanol were added thereto. The organic layer was collected by separation, and dried over anhydrous sodium sulfate, and the solvent was distilled off under reduced pressure. A mixed solvent of chloroform-methanol was added to the obtained residue, and the solid matter was collected by filtration, thereby obtaining N-(7-(1-methyl-1H-pyrazol-4-yl)-1,5-naphthyridin-2-yl)-1,3,4-thiadiazole-2-amine (1.4 mg) as a pale yellow solid.
$^1$H-NMR(DMSO-$d_6$)$\delta$:9.17(1H,s),9.08(1H,d,J=2.6Hz),8.52(1H,s),8.32(1H,d,J=2.6Hz), 8.28(1H,d,J=9.2Hz),8.20(1H,s),7.44(1H,d,J=9.2Hz),3.93(3H,s).
MSm/z(M+H):310.

<Example 0027>

<0027-1>

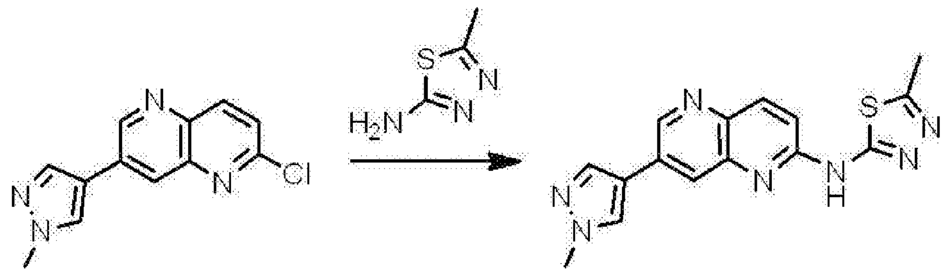

1,4-Dioxane (2 mL) was added to a mixture of 2-chloro-7-(1-methyl-1H-pyrazol-4-yl)-1,5-naphthyridine (20 mg), 5-methyl-1,3,4-thiadiazole-2-amine (14 mg), tris(dibenzylideneacetone)dipalladium(0) (7.5 mg), 4,5-bis(diphenylphosphino)-9,9-dimethylxanthene (9.5 mg), and cesium carbonate (80 mg) in a nitrogen atmosphere, and the reaction vessel was sealed, followed by stirring at 150°C for 30 minutes using a microwave reaction apparatus. Tris(dibenzylideneacetone)dipalladium(0) (7.5 mg) and 4,5-bis(diphenylphosphino)-9,9-dimethylxanthene (9.5 mg) were added thereto, followed by stirring at 150°C for 30 minutes using a microwave reaction apparatus. The reaction mixture was cooled to room temperature, and water and chloroform were added thereto. The organic layer was collected by separation, and dried over anhydrous sodium sulfate, and the solvent was distilled off under reduced pressure. Chloroform was added to the obtained residue, and the solid matter was collected by filtration, thereby obtaining 5-methyl-N-(7-(1-methyl-1H-pyrazol-4-yl)-1,5-naphthyridin-2-yl)-1,3,4-thiadiazole-2-amine (2.3 mg) as a pale yellow solid.
$^1$H-NMR(DMSO-$d_6$)$\delta$: 12.01(1H,brs),9.06(1H,d,J=2.0Hz),8. 52(1H,s),8.31 (1H,d,J=2.0Hz), 8.25(1H,d,J=8.9Hz),8.20(1H,s),7.39(1H,d,J=8.9Hz),3.92(3H,s),2.66(3H,s).
MSm/z(M+H):324.

<Example 0028>

<0028-1>

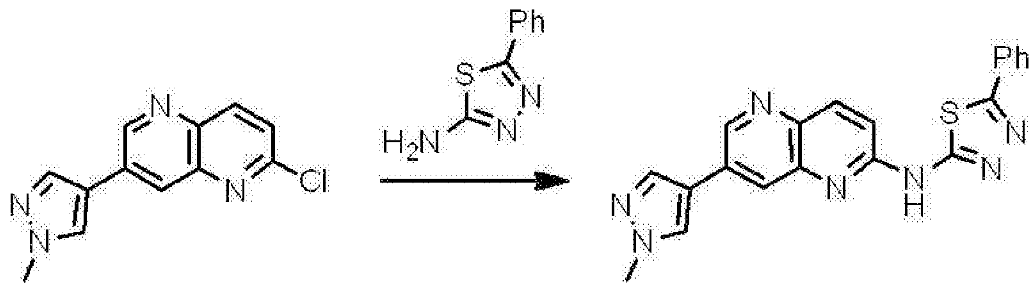

1,4-Dioxane (2 mL) was added to a mixture of 2-chloro-7-(1-methyl-1H-pyrazol-4-yl)-1,5-naphthyridine (20 mg), 5-phenyl-1,3,4-thiadiazole-2-amine (22 mg), tris(dibenzylideneacetone)dipalladium(0) (7.5 mg), 4,5-bis(diphenylphosphino)-9,9-dimethylxanthene (9.5 mg), and cesium carbonate (80 mg) in a nitrogen atmosphere, and the reaction vessel was sealed, followed by stirring at 150°C for 30 minutes using a microwave reaction apparatus. The reaction mixture was cooled to room temperature, and water was added thereto. The solid matter was collected by filtration, thereby obtaining N-(7-(1-methyl-1H-pyrazol-4-yl)-1,5-naphthyridin-2-yl)-5-phenyl-1,3,4-thiadiazole-2-amine (22 mg) as a grey solid.
$^1$H-NMR(DMSO-$d_6$)$\delta$:12.34(1H,brs),9.09(1H,d,J=2.0Hz),8.54(1H,s),8.46(1H,s), 8.27(1H,d,J=9.2Hz),8.25(1H,s),8.06-8.03(2H,m),7.60-7.52(3H,m),7.43(1H,d,J=9.2Hz), 3.94(3H,s).
MSm/z(M+H):386.

<Example 0029>

<0029-1>

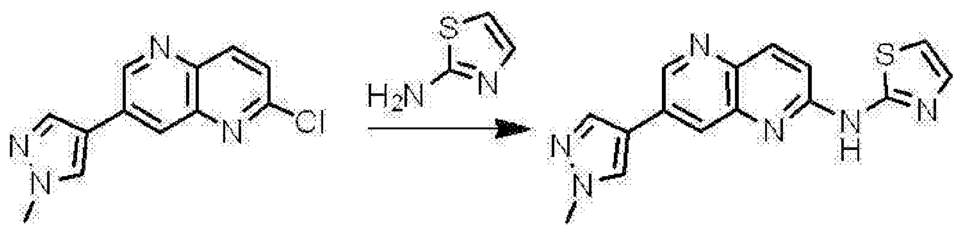

1,4-Dioxane (2 mL) was added to a mixture of 2-chloro-7-(1-methyl-1H-pyrazol-4-yl)-1,5-naphthyridine (20 mg), thiazole-2-amine (12 mg), tris(dibenzylideneacetone)dipalladium(0) (7.5 mg), 4,5-bis(diphenylphosphino)-9,9-dimethylxanthene (9.5 mg), and cesium carbonate (80 mg) in a nitrogen atmosphere, and the reaction vessel was sealed, followed by stirring at 150°C for 30 minutes using a microwave reaction apparatus. The reaction mixture was cooled to room temperature, and water was added thereto. The solid matter was collected by filtration, thereby obtaining N-(7-(1-methyl-1H-pyrazol-4-yl)-1,5-naphthyridin-2-yl)thiazole-2-amine (9 mg) as a gray solid.
$^1$H-NMR(DMSO-$d_6$)$\delta$:11.80(1H,brs),9.04(1H,d,J=1.7Hz),8.51(1H,s),8.28(1H,d,J=1.7Hz), 8.22-8.19(2H,m),7.50(1H,d,J=3.3Hz),7.39(1H,d,J=9.2Hz),7.18(1H,d,J=3.3Hz),3.92(3H,s).
MSm/z(M+H):309.

<Example 0030>

<0030-1>

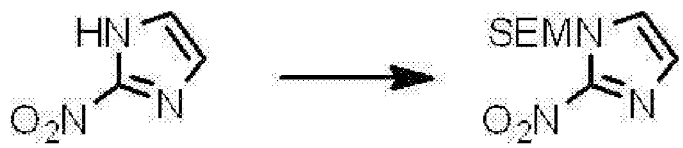

Tetrahydrofuran (10 mL) was added to 2-nitro-1H-imidazole (226 mg) in a nitrogen atmosphere, 60% sodium hydride (96 mg) was added thereto under ice-cooling, followed by stirring for 30 minutes under ice-cooling, and, thereafter, (2-(chloromethoxy)ethyl)trimethylsilane (421 μL) was added thereto, followed by stirring at room temperature for 2 hours. Water and ethyl acetate were added to the reaction mixture. The organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate), thereby obtaining 2-nitro-1-((2-(trimethylsilyl)ethoxy)methyl)-1H-imidazole (460 mg) as a yellow solid.
$^1$H-NMR($CDCl_3$)δ:7.37-7.36(2H,m),5.84(2H,s),3.73-3.68(2H,m),1.06-1.003(2H,m),0.07(9H,s).

<0030-2>

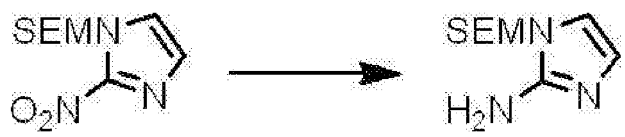

2-Nitro-1-((2-(trimethylsilyl)ethoxy)methyl)-1H-imidazole (176 mg) was dissolved in methanol (15 mL), and the resultant product was reacted using a flow-type hydrogenation reaction apparatus (atmospheric pressure, 1.0 mL/min, room temperature, 10% Pd/C). The solvent was distilled off under reduced pressure, thereby obtaining 1-((2-(trimethylsilyl)ethoxy)methyl)-1H-imidazole-2-amine (150 mg) as brown oily substance.
$^1$H-NMR($CDCl_3$)δ:6.61(1H,d,J=1.7Hz),6.56(1H,d,J=1.7Hz),5.07(2H,s),4.19(2H,brs),3.54-4.48(2H,m),0.93-0.87(2H,m),0.00(9H,s).

<0030-3>

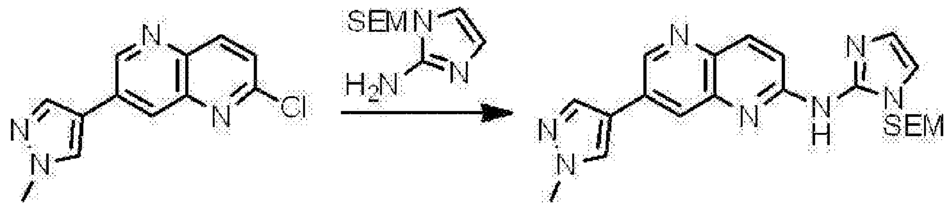

1,4-Dioxane (2 mL) was added to a mixture of 2-chloro-7-(1-methyl-1H-pyrazol-4-yl)-1,5-naphthyridine (20 mg), 1-((2-(trimethylsilyl)ethoxy)methyl)-1H-imidazole-2-amine (26 mg), tris(dibenzylideneacetone)dipalladium(0) (7.5 mg), 4,5-bis(diphenylphosphino)-9,9-dimethylxanthene (9.5 mg), and cesium carbonate (80 mg) in a nitrogen atmosphere, and the reaction vessel was sealed, followed by stirring at 150°C for 30 minutes using a microwave reaction apparatus. The reaction mixture was cooled to room temperature, and water and ethyl acetate were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate, NH silica), thereby obtaining 7-(1-methyl-1H-pyrazol-4-yl)-N-(1-((2-(trimethylsilyl)ethoxy)methyl)-1H-imidazol-2-yl)-1,5-naphthyridine-2-amine (15 mg) as an orange solid.
MSm/z(M+H):422.

<0030-4>

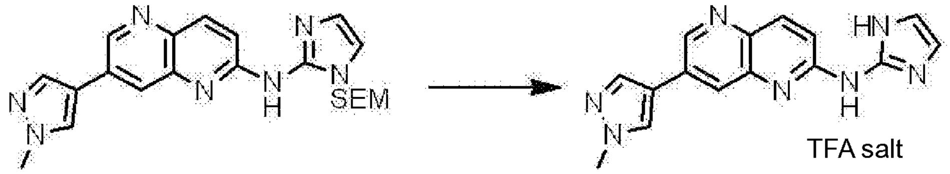


7-(1-Methyl-1H-pyrazol-4-yl)-N-(1-((2-(trimethylsilyl)ethoxy)methyl)-1H-imidazol-2-yl)-1,5-naphthyridine-2-amine (15 mg) was dissolved in a mixed solvent (4.1 mL) of methylene chloride-ethanol-trifluoroacetic acid (3:0.1:1), followed by stirring at room temperature for 7 hours. The reaction mixture was distilled off under reduced pressure, thereby obtaining trifluoroacetate (1.8 mg) of N-(1H-imidazol-2-yl)-7-(1-methyl-1H-pyrazol-4-yl)-1,5-naphthyridine-2-amine as a pale yellow solid.
$^1$H-NMR($CD_3OD$)δ:9.10(1H,d,J=2.OHz),8.69(1H,d,J=2.OHz),8.35(1H,d,J=9.2Hz),8.28(1H,s), 8.08(1H,s),7.40(1H,d,J=9.2Hz),7.23(2H,s),4.01(3H,s).
MSm/z(M+H):292.

<Example 0031>

<0031-1>

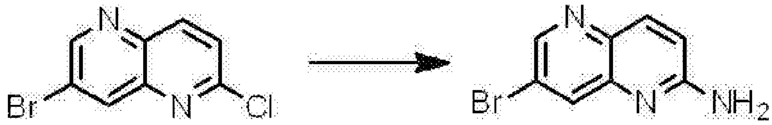

A mixture solution of 7-bromo-2-chloro-1,5-naphthyridine (100 mg) in 1,4-dioxane (2 mL) and a 25% ammonia aqueous solution was stirred at 120°C for 3 hours using a microwave reaction apparatus. The reaction mixture was cooled to room temperature, and a saturated sodium chloride aqueous solution and ethyl acetate were added thereto. The organic layer was collected by separation, and dried over anhydrous sodium sulfate, and the solvent was distilled off under reduced pressure, thereby obtaining 7-bromo-1,5-naphthyridine-2-amine (90 mg) as a white solid.
$^1$H-NMR(DMSO-$d_6$)δ:8.53(1H,d),8.02(1H,d),7.92(1H,d),7.01(1H,d),6.94(1H,s).
MSm/z(M+H):224,226.

<0031-2>

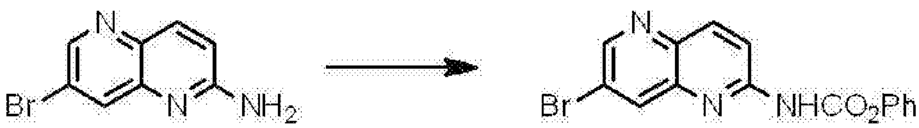

Pyridine (9 mL) was added to a mixture of 7-bromo-1,5-naphthyridine-2-amine (300 mg) and phenyl chloroformate (280 μL), followed by stirring at room temperature for 2 hours. Phenyl chloroformate (140 μL) was added thereto, followed by stirring at room temperature for 1 hour. The reaction mixture was distilled off under reduced pressure, and the obtained residue was purified by silica gel column chromatography (chloroform-methanol), thereby obtaining phenyl (7-bromo-1,5-naphthyridin-2-yl)carbamate (310 mg) as a white solid.
MSm/z(M+H):344,346.

<0031-3>

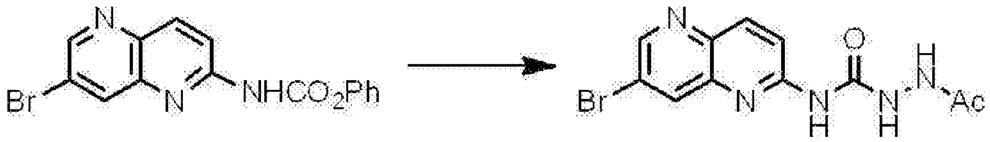

Phenyl (7-bromo-1,5-naphthyridin-2-yl)carbamate (20 mg) was dissolved in 1,4-dioxane (2 mL), and acetohydrazide (6.5 mg) was added thereto, followed by stirring at 150°C for 30 minutes using a microwave reaction apparatus. The reaction mixture was cooled to room temperature, the solid matter was collected by filtration, thereby obtaining 2-acetyl-N-(7-bromo-1,5-naphthyridin-2-yl)hydrazinecarboxamide (14 mg) as a white solid.
MSm/z(M+H):324,326.

<0031-4>

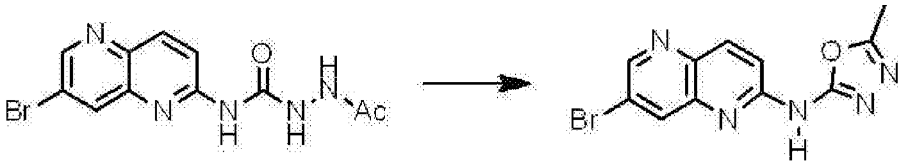

Phosphorus oxychloride (2.5 g) was added to 2-acetyl-N-(7-bromo-1,5-naphthyridin-2-yl)hydrazinecarboxamide (50 mg), followed by stirring at 80°C for 2 hours. The reaction mixture was cooled to room temperature, and water was added dropwise thereto. The resultant product was neutralized with a 5 mol/L sodium hydroxide aqueous solution, and chloroform was added thereto. The organic layer was collected by separation, and dried over anhydrous sodium sulfate, and the solvent was distilled off under reduced pressure, thereby obtaining N-(7-bromo-1,5-naphthyridin-2-yl)-5-methyl-1,3,4-oxadiazole-2-amine (34 mg) as a yellow solid.
MSm/z(M+H):306,308.

<0031-5>

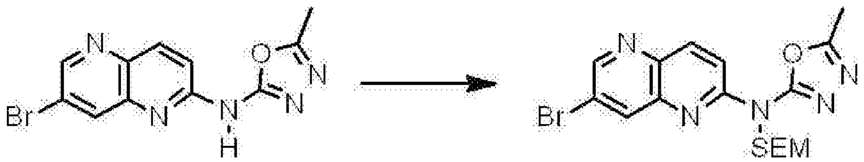

N-(7-bromo-1,5-naphthyridin-2-yl)-5-methyl-1,3,4-oxadiazole-2-amine (31 mg) was dissolved in N,N-dimethylformamide (2 mL) in a nitrogen atmosphere, and 60% sodium hydride (6 mg) was added thereto under ice-cooling, followed by stirring for 1 hour. (2-(Chloromethoxy)ethyl)trimethylsilane (14 μL) was added to the reaction mixture, followed by stirring at room temperature for 1 hour. Water and ethyl acetate were added to the reaction mixture. The organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate), thereby obtaining N-(7-bromo-1,5-naphthyridin-2-yl)-5-methyl-N-((2-(trimethylsilyl)ethoxy)methyl)-1,3,4-oxadiazole-2-amine (20 mg).
$^1$H-NMR(CDCl$_3$)δ:8.85(1H,d,J=2.0Hz),8.35(1H,d,J=2.0Hz),8.32(1H,d,J=9.9Hz),
8.19(1H,d,J=9.9Hz),5.77(2H,s),3.82-3.75(2H,m),2.56(3H,s),0.99-0.94(2H,m),-0.05(9H,s).

<0031-6>

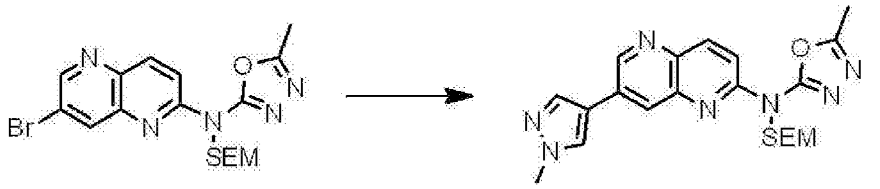

1,4-Dioxane (2 mL) and a 2 mol/L sodium carbonate aqueous solution (69 μL) were added to a mixture of N-(7-bromo-1,5-naphthyridin-2-yl)-5-methyl-N-((2-(trimethylsilyl)ethoxy)methyl)-1,3,4-oxadiazole-2-amine (20 mg), 1-methyl-4-(4,4,5,5-tetramethyl-1,3,2-dioxaborolan-2-yl)-1H-pyrazole (14 mg), and bis(di-tert-butyl (4-dimethylaminophenyl)phosphine)dichloropalladium(II) (3.2 mg) in a nitrogen atmosphere, and the reaction vessel was sealed, followed by stirring at 100°C overnight. The reaction mixture was cooled to room temperature, and water and ethyl acetate were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate, NH silica), thereby obtaining 5-methyl-N-(7-(1-methyl-1H-pyrazol-4-yl)-1,5-naphthyridin-2-yl)-N-((2-(trimethylsilyl)ethoxy)methyl)-1,3,4-oxadiazole-2-amine (18 mg) as a white solid.
MSm/z(M+H):438.

<0031-7>

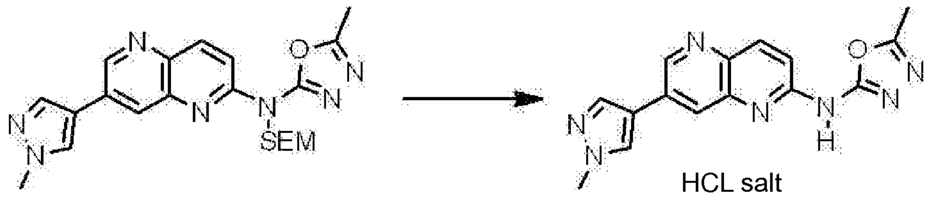


A mixed solvent (4.1 mL) of chloroform-ethanol-trifluoroacetic acid (3:0.1:1) was added to 5-methyl-N-(7-(1-methyl-1H-pyrazol-4-yl)-1,5-naphthyridin-2-yl)-N-((2-(trimethylsilyl)ethoxy)methyl)-1,3,4-oxadiazole-2-amine (18 mg), followed by stirring at room temperature for 3 hours. The solvent was distilled off under reduced pressure, and a mixed solvent of methanol-ethyl acetate was added to the obtained residue. The solid matter was collected by filtration, thereby obtaining 5-methyl-N-(7-(1-methyl-1H-pyrazol-4-yl)-1,5-naphthyridin-2-yl)-1,3,4-oxadiazole-2-amine hydrochloride (5 mg) as a yellow solid.
$^1$H-NMR(DMSO-$d_6$)δ:9.07(1H,d,J=2.0Hz),8.49(1H,s),8.29-8.26(2H,m),8.18(1H,s),7.96-7.84(1H,m),3.92(3H,s),2.46(3H,s).
MSm/z(M+H):308.

<Example 0032>

<0032-1>

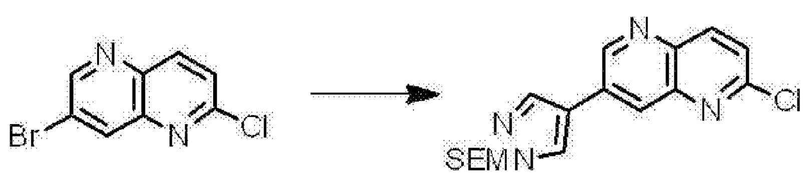

1,4-Dioxane (2 mL) and a 2 mol/L sodium carbonate aqueous solution (315 μL) were added to a mixture of 7-bromo-2-chloro-1,5-naphthyridine (50 mg), 4-(4,4,5,5-tetramethyl-1,3,2-dioxaborolan-2-yl)-1-((2-(trimethylsilyl)ethoxy)methyl)-1H-pyrazole (77 mg), and bis(di-tert-butyl (4-dimethylaminophenyl)phosphino)dichloropalladium(II)

(15 mg) in a nitrogen atmosphere, and the reaction vessel was sealed, followed by stirring at 100°C for 5 hours. The reaction mixture was cooled to room temperature, and water and ethyl acetate were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate), thereby obtaining 2-chloro-7-(1-((2-(trimethylsilyl)ethoxy)methyl)-1H-pyrazol-4-yl)-1,5-naphthyridine (57 mg) as a white solid.
$^1$H-NMR($CDCl_3$)δ:9.15(1H,d,J=2.0Hz),8.35-8.31(2H,m),8.04(1H,s),8.00(1H,s),7.57(1H,d,J=8.6Hz),5.53(2H,s),3.68-3.63(2H,m),0.99-0.93(2H,m),0.00(9H,s).

<0032-2>

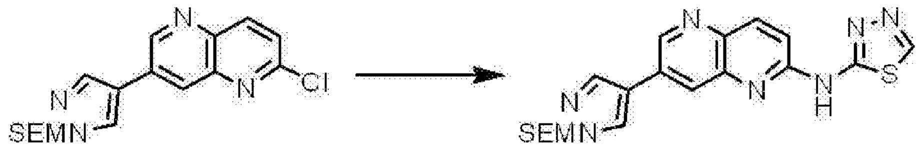

1,4-Dioxane (3 mL) was added to a mixture of 2-chloro-7-(1-((2-(trimethylsilyl)ethoxy)methyl)-1H-pyrazol-4-yl)-1,5-naphthyridine (57 mg), 1,3,4-thiadiazole-2-amine (24 mg), tris(dibenzylideneacetone)dipalladium(0) (14 mg), 4,5-bis(diphenylphosphino)-9,9-dimethylxanthene (18 mg), and cesium carbonate (154 mg) in a nitrogen atmosphere, followed by stirring at 100°C for 7 hours. The reaction mixture was cooled to room temperature, and water and ethyl acetate were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (chloroform-methanol, NH silica), thereby obtaining N-(7-(1-((2-trimethylsilyl)ethoxy)methyl)-1 H-pyrazol-4-yl)-1,5-naphthyridin-2-yl)-1,3,4-thiadiazole-2-amine (23 mg).
MSm/z(M+H):426.

<0032-3>

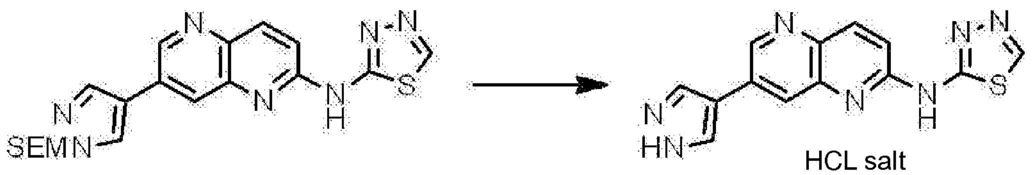


Ethanol (3 mL) and 3 mol/L hydrochloric acid (3 mL) were added to N-(7-(1-((2-trimethylsilyl)ethoxy)methyl)-1 H-pyrazol-4-yl)-1,5-naphthyridin-2-yl)-1,3,4-thiadiazole-2-amine (23 mg), followed by stirring at 80°C for 4 hours. The reaction mixture was cooled to room temperature, and the solvent was concentrated under reduced pressure. Methanol and ethyl acetate were added to the obtained residue, and the produced solid matter was collected by filtration, thereby obtaining N-(7-(1H-pyrazol-4-yl)-l,5-naphthyridin-2-yl)-1,3,4-thiadiazole-2-amine hydrochloride (6 mg) as a pale yellow solid.
$^1$H-NMR(DMSO-$d_6$)δ:12.30(1H,brs),9.19-9.18(2H,m),8.46(1H,d,J=1.2Hz),8.44(2H,s),8.33(1H,d,J=9.2Hz),7.49(1H,d,J=9.2Hz).
MSm/z(M+H):296.

<Example 0033>

<0033-1>

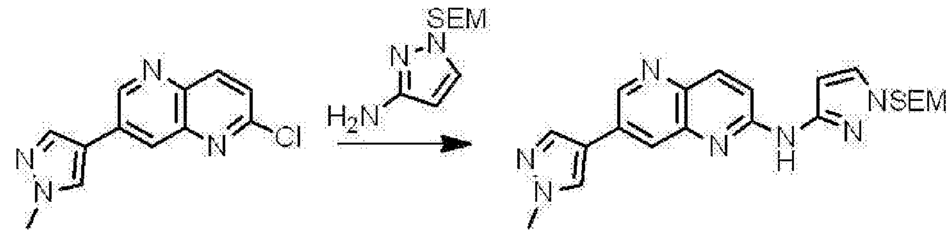

1,4-Dioxane (3 mL) was added to a mixture of 2-chloro-7-(1-methyl-1H-pyrazol-4-yl)-1,5-naphthyridine (15 mg), 1-((2-(trimethylsilyl)ethoxy)methyl)-1H-pyrazole-3-amine (20 mg), tris(dibenzylideneacetone)dipalladium(0) (5.5 mg), 4,5-bis(diphenylphosphino)-9,9-dimethylxanthene (6.9 mg), and cesium carbonate (59 mg) in a nitrogen atmosphere, and the reaction vessel was sealed, followed by stirring at 150°C for 30 minutes using a microwave reaction apparatus. Tris(dibenzylideneacetone)dipalladium(0) (11 mg) and 4,5-bis(diphenylphosphino)-9,9-dimethylxanthene (14 mg) were added thereto, followed by stirring at 150°C for 1 hour using a microwave reaction apparatus. The reaction mixture was cooled to room temperature, and water and ethyl acetate were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate, chloroform-methanol, NH silica), and further purified by preparative thin layer silica gel chromatography (chloroform-methanol), thereby obtaining 7-(1-methyl-1H-pyrazol-4-yl)-N-(1-((2-(trimethylsilyl)ethoxy)methyl)-1H-pyrazol-3-yl)-1,5-naphthyridine-2-amine (4 mg) as oily substance.
MSm/z(M+H):422.

<0033-2>

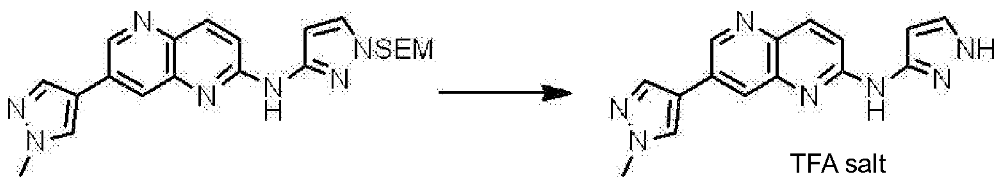


7-(1-Methyl-1H-pyrazol-4-yl)-N-(1-((2-(trimethylsilyl)ethoxy)methyl)-1H-pyrazol-3-yl)-1,5-naphthyridine-2-amine (4 mg) was dissolved in a mixed solvent (2.3 mL) of chloroform-trifluoroacetic acid-ethanol (1:1:0.3), followed by stirring at 50°C for 6 hours. The reaction mixture was cooled to room temperature, and the solvent was distilled off under reduced pressure. The obtained residue was washed with ethyl acetate, thereby obtaining 7-(1-methyl-1H-pyrazol-4-yl)-N-(1H-pyrazol-3-yl)-1,5-naphthyridine-2-aminetrifluoroacetate (2 mg) as a yellow solid.
$^1$H-NMR(DMSO-$d_6$)$\delta$:10.84(1H,brs),9.02(1H,d,J=2.0Hz),8.48(1H,s),8.39(1H,br),8.20-8.17(2H,m),7.78(1H,d,J=2.0Hz),7.40(1H,d,J=9.2 Hz),6.75(1H,s),3.95(3H,s).
MSm/z(M+H):292.

<Example 0034>

<0034-1>

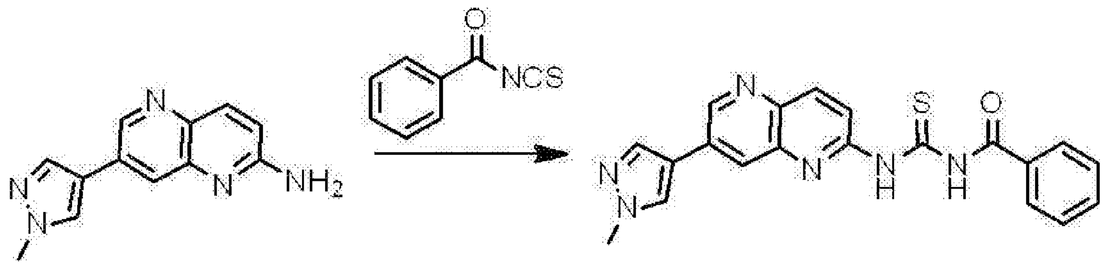

7-(1-Methyl-1H-pyrazol-4-yl)-1,5-naphthyridine-2-amine (201 mg) was dissolved in 1,4-dioxane (8.9 mL), and benzoyl isothiocyanate (0.24 mL) was added thereto under ice-cooling, followed by stirring at room temperature for 1 hour, and stirring at 55°C for 2 hours. The reaction mixture was cooled to room temperature, and the solvent was distilled off under reduced pressure. Ethyl acetate was added to the obtained residue, and the solid matter was collected by filtration, thereby obtaining N-((7-(1-methyl-1H-pyrazol-4-yl)-1,5-naphthyridin-2-yl)carbamothioyl)benzamide (232 mg) as a yellow solid.
MSm/z(M+H):389.

<0034-2>

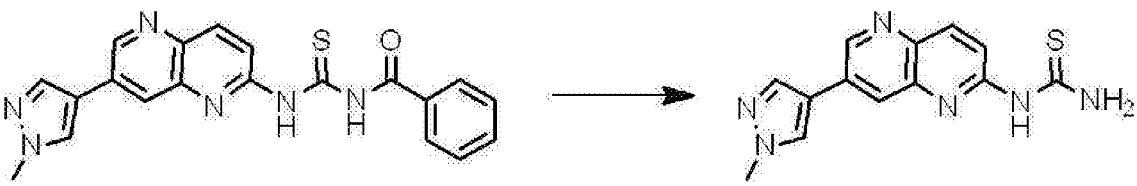

N-((7-(1-methyl-1H-pyrazol-4-yl)-1,5-naphthyridin-2-yl)carbamothioyl)benzamide (50 mg) was dissolved in 1,4-dioxane (1.3 mL), and a 2 mol/L sodium hydroxide aqueous solution (0.14 mL) was added thereto, followed by stirring at 80°C for 1.5 hours. The reaction mixture was cooled to room temperature, and water was added thereto. The solid matter was collected by filtration, thereby obtaining 1-(7-(1-methyl-1H-pyrazol-4-yl)-1,5-naphthyridin-2-yl)thiourea (22 mg) as a pale yellow solid.
MSm/z(M+H):285.

<0034-3>

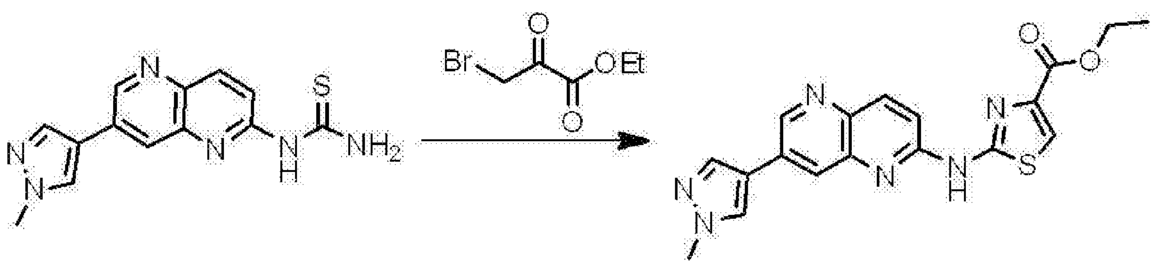

1-(7-(1-Methyl-1H-pyrazol-4-yl)-1,5-naphthyridin-2-yl)thiourea (20 mg) and ethyl 3-bromo-2-oxopropanoate (8.8 mL) were dissolved in N,N-dimethylformamide (2 mL), followed by stirring at 60°C for 2 hours. The reaction mixture was cooled to room temperature, and water and ethyl acetate were added thereto. The organic layer was collected by separation, washed sequentially with water and a saturated sodium chloride aqueous solution, and dried over anhydrous sodium sulfate, and the solvent was distilled off under reduced pressure. The obtained residue was purified by silica gel column chromatography (chloroform-methanol), thereby obtaining ethyl 2-((7-(1-methyl-1H-pyrazol-4-yl)-1,5-naphthyridin-2-yl)amino)thiazole-4-carboxylate (16 mg) as a yellow solid.
$^1$H-NMR(DMSO-$d_6$)δ:12.28(1H,s),9.07(1H,d,J=2.3Hz),8.51(1H,s),8.31(1H,d,J=2.3Hz),

8.24(1H,d,J=9.2Hz),8.19(1H,s),8.04(1H,s),7.33(1H,d,J=9.2Hz),4.30(2H,q,J=7.0Hz), 3.92(3H,s),1.32(3H,t,J=7.0Hz). MSm/z(M+H):381.

<Example 0035>

<0035-1>

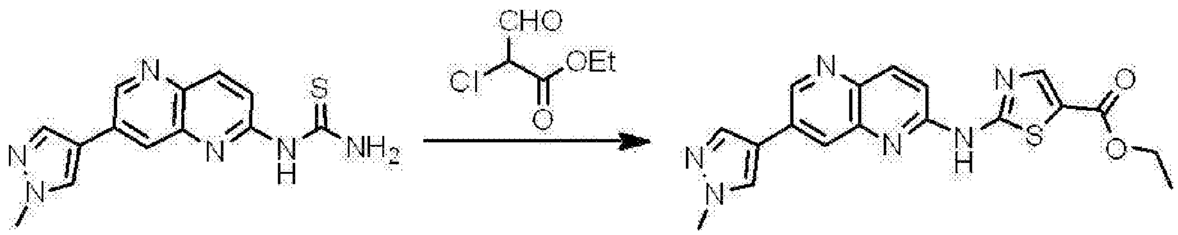

1-(7-(1-Methyl-1H-pyrazol-4-yl)-1,5-naphthyridin-2-yl)thiourea (20 mg) and ethyl 2-chloro-3-oxopropanoate (10 mg) were dissolved in N,N-dimethylformamide (2 mL), followed by stirring at 80°C overnight. The reaction mixture was cooled to room temperature, and water and ethyl acetate were added thereto. The organic layer was collected by separation, washed sequentially with water and a saturated sodium chloride aqueous solution, and dried over anhydrous sodium sulfate, and the solvent was distilled off under reduced pressure. The obtained residue was dissolved in a mixed solvent of chloroform-methanol, and ethyl acetate was added thereto. The solid matter was collected by filtration, thereby obtaining ethyl 2-((7-(1-methyl-1H-pyrazol-4-yl)-1,5-naphthyridin-2-yl)amino)thiazole-5-carboxylate (8 mg) as a pale yellow solid.
$^1$H-NMR(DMSO-$d_6$)δ:12.35(1H,s),9.12(1H,d,J=2.0Hz),8.58(1H,s),8.34(1H,d,J=2.0Hz), 8.30(1H,d,J=9.2Hz),8.24(1H,s),8.17(1H,s),7.45(1H,d,J=9.2Hz),4.33(2H,q,J=7.0Hz), 3.93(3H,s),1.34(3H,t,J=7.0Hz). MSm/z(M+H):381.

<Example 0036>

<0036-1>

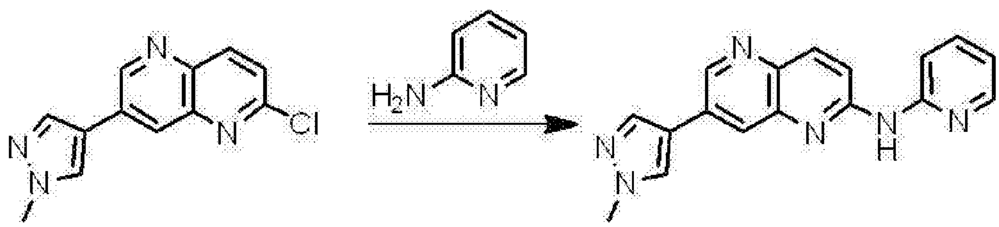

1,4-Dioxane (2 mL) was added to a mixture of 2-chloro-7-(1-methyl-1H-pyrazol-4-yl)-1,5-naphthyridine (12 mg), 2-aminopyridine (7 mg), potassium tert-butoxide (16 mg), tris(dibenzylideneacetone)dipalladium(0) (4.5 mg), and 4,5-bis(diphenylphosphino)-9,9-dimethylxanthene (5.6 mg) in a nitrogen atmosphere, and the reaction vessel was sealed, followed by stirring at 150°C for 30 minutes using a microwave reaction apparatus. The reaction mixture was cooled to room temperature, and water and ethyl acetate were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (chloroform-methanol), thereby obtaining 7-(1-methyl-1H-pyrazol-4-yl)-N-(pyridin-2-yl)-1,5-naphthyridine-2-amine (4 mg) as a pale yellow solid.
$^1$H-NMR(DMSO-$d_6$)δ:10.25(1H,s),9.00(1H,d,J=2.0Hz),8.62(1H,d,J=8.6Hz),8.48(1H,s),8.32-8.30(1H,m),8.25(1H,d,J=2.0Hz),8.17(1H,s), 8.15(1H,d,J=9.2Hz), 7.83-7.77(1H,m),7.67(1H,d,J=9.2Hz),7.03-6.99(1H,m),3.92(3H,s).
MSm/z(M+H):303.

<Example 0037>

<0037-1>

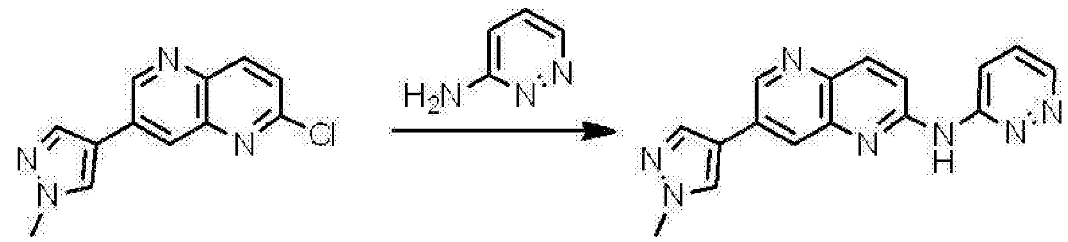

1,4-Dioxane (2 mL) was added to a mixture of 2-chloro-7-(1-methyl-1H-pyrazol-4-yl)-1,5-naphthyridine (12 mg), 2-aminopyridazine (7 mg), potassium tert-butoxide (16 mg), tris(dibenzylideneacetone)dipalladium(0) (4.5 mg), and 4,5-bis(diphenylphosphino)-9,9-dimethylxanthene (5.6 mg) in a nitrogen atmosphere, and the reaction vessel was sealed, followed by stirring at 150°C for 30 minutes using a microwave reaction apparatus. The reaction mixture was cooled to room temperature, and water and ethyl acetate were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (chloroform-methanol), thereby obtaining 7-(1-methyl-1H-pyrazol-4-yl)-N-(pyridazin-3-yl)-1,5-naphthyridine-2-amine (5 mg) as a pale yellow solid.
$^1$H-NMR(DMSO-$d_6$)$\delta$:10.81(1H,s),9.04(1H,d,J=2.0Hz),8.95-8.87(2H,m),8.47(1H,s),8.29(1H,d,J=2.0Hz),8.22(1H,d,J=9.2Hz),8.17(1H,s),7.70-7.64(2H,m),3.92(3H,s).
MSm/z(M+H):304.

<Example 0038>

<0038-1>

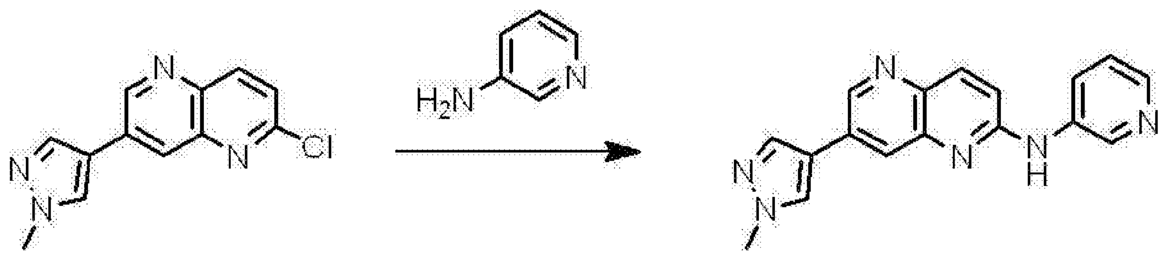

1,4-Dioxane (2 mL) was added to a mixture of 2-chloro-7-(1-methyl-1H-pyrazol-4-yl)-1,5-naphthyridine (12 mg), 3-aminopyridine (7 mg), potassium tert-butoxide (16 mg), tris(dibenzylideneacetone)dipalladium(0) (4.5 mg), and 4,5-bis(diphenylphosphino)-9,9-dimethylxanthene (5.6 mg) in a nitrogen atmosphere, and the reaction vessel was sealed, followed by stirring at 150°C for 30 minutes using a microwave reaction apparatus. The reaction mixture was cooled to room temperature, and water and ethyl acetate were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (chloroform-methanol), thereby obtaining 7-(1-methyl-1H-pyrazol-4-yl)-N-(pyridin-3-yl)-1,5-naphthyridine-2-amine (5 mg) as a pale yellow solid.
$^1$H-NMR(DMSO-$d_6$)$\delta$:9.82(1H,s),9.11(1H,d,J=2.6Hz),8.96(1H,d,J=2.0Hz),8.57-8.53(1H,m),8.48(1H,s),8.24(1H,d,J=2.0Hz),8.21(1H,dd,J=4.6,1.3Hz),8.17(1H,s),8.11(1H,d,J=9.2Hz),7 .38(H,dd,J=8.6,4.6Hz),7.24(1H,d,J=9.2Hz),3.91(3H,s).
MSm/z(M+H):303.

<Example 0039>

<0039-1>

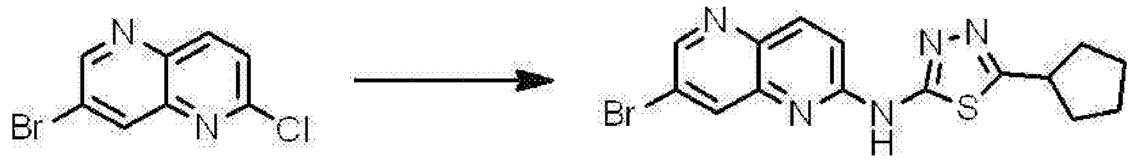

A solution of 7-bromo-2-chloro-1,5-naphthyridine (2.04 g), 5-cyclopentyl-1,3,4-thiadiazole-2-amine (1.41 g), and potassium carbonate (1.73 g) in dimethylsulfoxide (16 mL) was stirred at 130°C for 3 hours. After the reaction mixture was cooled to room temperature, water was added thereto, and the solid matter was collected by filtration, thereby obtaining N-(7-bromo-1,5-naphthyridin-2-yl)-5-cyclopentyl-1,3,4-thiadiazole-2-amine (1.92 g).
$^1$H-NMR(DMSO-$d_6$)$\delta$:12.20(1H,s),8.84(1H,d,J=2.7Hz),8.56(1H,d,J=2.7Hz),
8.31(1H,d,J=9.3Hz),7.51(1H,d,J=9.3Hz),3.56-3.40(1H,m), 2.22-2.08(2H,m),1.94-1.34(6H,m).
MSm/z(M+H):376,378.

<Example 0040>

<0040-1>

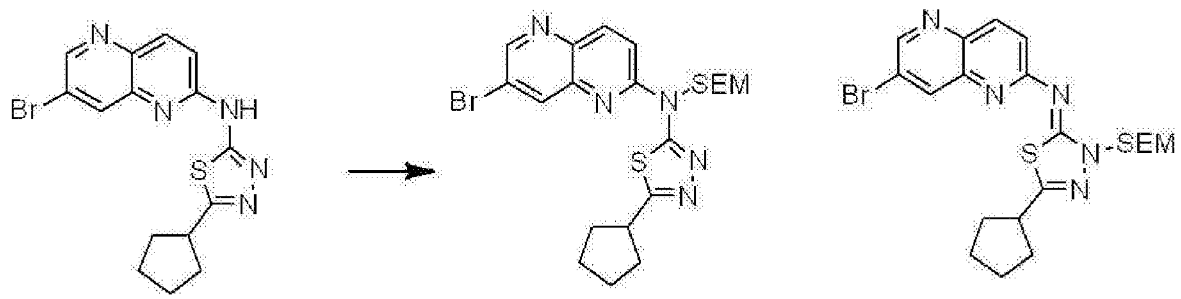

60% sodium hydride (256 mg) was added to a solution of N-(7-bromo-1,5-naphthyridin-2-yl)-5-cyclopentyl-1,3,4-thiadiazole-2-amine (1.21 g) and 2-(chloromethoxy)ethyltrimethylsilane (0.846 mL) in N-methylpyrrolidone (32 mL) under ice-cooling, followed by stirring at the same temperature for 1 hour, and stirring at room temperature for 1 hour. Ethyl acetate and water were added to the reaction mixture. The organic layer was collected by separation, washed with a saturated sodium chloride aqueous solution, and and dried over anhydrous sodium sulfate. The obtained residue was purified by silica gel column chromatography (ethyl acetate-hexane), thereby obtaining N-(7-bromo-1,5-naphthyridin-2-yl)-5-cyclopentyl-N-((2-(trimethylsilyl)ethoxy)methyl)-1,3,4-thiadiazole-2-amine (601 mg) and (Z)-7-bromo-N-(5-cyclopentyl-3-((2-(trimethylsilyl)ethoxy)methyl)-1,3,4-thiadiazol-2(3H)-ylidene)-1,5-naphthyridine-2-amine (206 mg).
MSm/z(M+H):506,508, 506,508.

<0040-2>

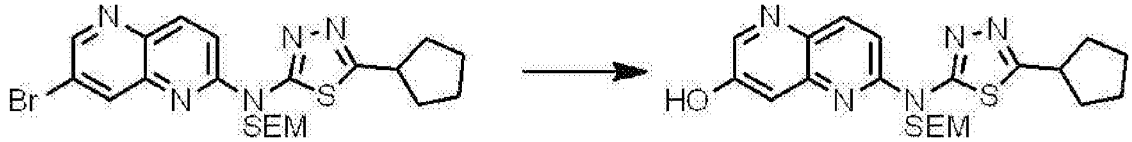

A mixture ofN-(7-bromo-1,5-naphthyridin-2-yl)-5-cyclopentyl-N-((2-(trimethylsilyl)ethoxy)methyl)-1,3,4-thiadiazole-2-amine (200 mg), bis(pinacolato)diboron (150 mg), (1,1'-bis(diphenylphosphino)ferrocene)palladium(II) dichloride (32 mg) and potassium acetate (77 mg) in 1,4-dioxane (2.0 mL) was stirred at 80°C for 2 hours in a nitrogen atmosphere. The reaction mixture was cooled to room temperature, and 30% hydrogen peroxide water (0.1 mL) and a saturated sodium hydrogen carbonate aqueous solution (1.5 mL) were added thereto, followed by stirring at room temperature for 3 hours. A saturated sodium chloride aqueous solution and ethyl acetate were added to the reaction mixture. The organic layer was collected by separation, and dried over anhydrous sodium sulfate, and the solvent was distilled off under reduced pressure. The obtained residue was purified by silica gel column chromatography (ethyl acetate-hexane), thereby obtaining 6-((5-cyclopentyl-1,3,4-thiadiazol-2-yl)((2-(trimethylsilyl)ethoxy)methyl)amino)-1,5-naphthyridin-3-ol (99 mg) as a white solid.
MSm/z(M+H):444.

<0040-3>

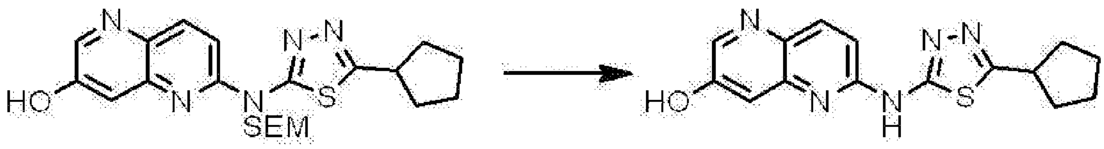

HCL salt

Methanol (0.5 mL), 12 mol/L hydrochloric acid (0.5 mL), 1,4-dioxane (0.25 mL), N-methylpyrrolidone (0.125 mL), and methanol (0.5 mL) were added to 6-((5-cyclopentyl-1,3,4-thiadiazol-2-yl)((2-(trimethylsilyl)ethoxy)methyl)amino)-1,5-naphthyridin-3-ol (10 mg), followed by stirring at 50°C for 1 hour. The reaction mixture was cooled to room temperature, and the solvent was distilled off under reduced pressure. Diisopropyl ether and water were added to the obtained residue, and the solid matter was collected by filtration, thereby obtaining 6-((5-cyclopentyl-1,3,4-thiadiazol-2-yl)amino)-1,5-naphthyridin-3-ol hydrochloride (2.9 mg).
$^1$H-NMR(DMSO-$d_6$)$\delta$:12.06-11.80(1H,brs),10.74-10.56(1H,brs),8.44(1H,d,J=2.1Hz),8.17(1H,d,J=9.0Hz),7.43(1H,d,J=2.1Hz),7.25(1H,d,J=9.0Hz),3.54-3.38(1H,m),2.22-2.08(2H,m),1.90-1.62(6H,m).
MSm/z(M+H):314.

<Example 0041>

<0041-1>

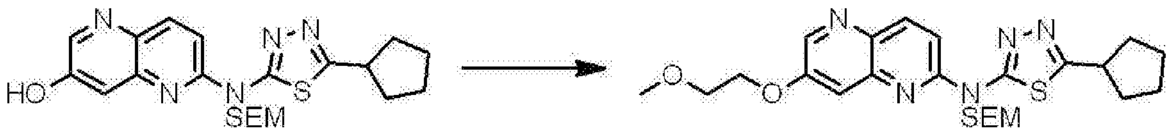

1-Bromo-2-methoxyethane (0.0064 mL) was added to a mixture of 6-((5-cyclopentyl-1,3,4-thiadiazol-2-yl)((2-(trimethylsilyl)ethoxy)methyl)amino)-1,5-naphthyridin-3-ol (20 mg) and potassium carbonate (19 mg) in N-methylpyrrolidone (0.5 mL), followed by stirring at 70°C for 3 hours. The reaction mixture was cooled to room temperature, and ethyl acetate and water were added thereto. The organic layer was collected by separation, washed sequentially with water and a saturated sodium chloride aqueous solution, and dried over anhydrous sodium sulfate, and the solvent was distilled off under reduced pressure, thereby obtaining 5-cyclopentyl-N-(7-(2-methoxyethoxy)-1,5-naphthyridin-2-yl)-N-((2-(trimethylsilyl)ethoxy)methyl)-1,3,4-thiadiazole-2-amine (13 mg).
MSm/z(M+H):502.

<0041-2>

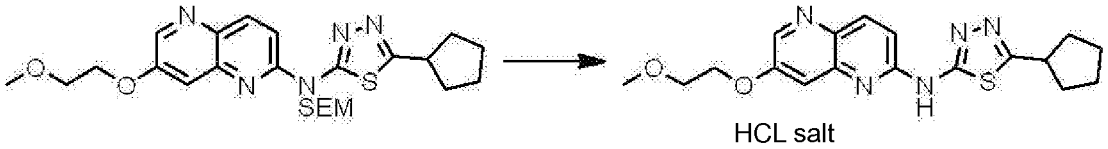


12 mol/L hydrochloric acid (0.4 mL) was added to a solution of 5-cyclopentyl-N-(7-(2-methoxyethoxy)-1,5-naphthyridin-2-yl)-N-((2-trimethylsilyl)ethoxy)methyl)-1,3,4-thiadiazole-2-amine (13 mg) in methanol (1.2 mL), followed by stirring at room temperature for 1.5 hours. The solvent was distilled off under reduced pressure, and diisopropyl ether and water were added to the obtained residue. The solid matter was collected by filtration, thereby obtaining 5-cyclopentyl-N-(7-(2-methoxyethoxy)-1,5-naphthyridin-2-yl)-1,3,4-thiadiazole-2-amine hydrochloride (3.3 mg).
$^1$H-NMR(DMSO-$d_6$)$\delta$:11.98(1H,brs),8.52(1H,d,J=2.7Hz),8.21(1H,d,J=9.3Hz),
7.63(1H,d,J=2.7Hz),7.30(1H,d,J=9.3Hz),4.38-4.33(2H,m),3.78-3.73(2H,m),3.52-3.36(1H,m),3.38(3H,s),2.22-2.08(2H,m), 1.94-1.64(6H,m).
MSm/z(M+H):372.

<Example 0042> (not in accordance with the present invention)

<0042-1>

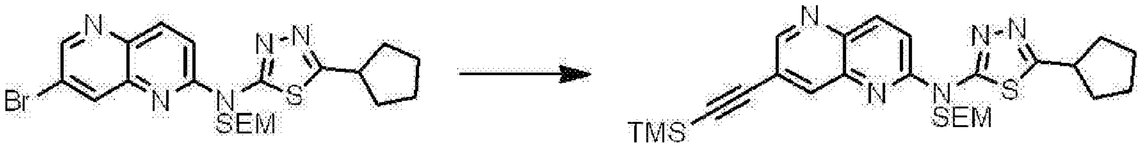

Tetrakis(triphenylphosphine)palladium(0) (16 mg), copper iodide (I) (5 mg), triethylamine (0.049 mL), and trimethylsilylacetylene (0.039 mL) were added to a solution of N-(7-bromo-1,5-naphthyridin-2-yl)-5-cyclopentyl-N-((2-(trimethylsilyl)ethoxy)methyl)-1,3,4-thiadiazole-2-amine (70 mg) in tetrahydrofuran (1.5 mL), followed by stirring for 1.5 hours under heating to reflux. N-methylpyrrolidone (0.75 mL) and trimethylsilylacetylene (0.2 mL) were added thereto, followed by stirring at the same temperature for 1 hour. The reaction mixture was cooled to room temperature, and ethyl acetate and water were added thereto. The organic layer was collected by separation, washed sequentially with water and a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining 5-cyclopentyl-N-((2-(trimethylsilyl)ethoxy)methyl)-N-(7-((trimethylsilyl)ethynyl)-1,5-naphthyridin-2-yl)-1,3,4-thiadiazole-2-amine (65 mg).
MSm/z(M+H):524.

<0042-2>

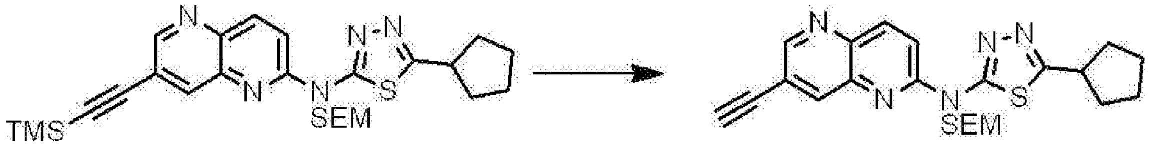

Methanol (1.2 mL) was added to 5-cyclopentyl-N-((2-(trimethylsilyl)ethoxy)methyl)-N-(7-((trimethylsilyl)ethynyl)-1,5-naphthyridin-2-yl)-1,3,4-thiadiazole-2-amine (65 mg) and potassium carbonate (34 mg), followed by stirring at room temperature for 2 hours. Ethyl acetate and a saturated sodium chloride aqueous solution were added to the reaction mixture. The organic layer was collected by separation, and dried over anhydrous sodium sulfate. The obtained solution was purified by silica gel column chromatography (ethyl acetate-hexane), thereby obtaining 5-cyclopentyl-N-(7-ethynyl-1,5-naphthyridin-2-yl)-N-((2-(trimethylsilyl)ethoxy)methyl)-1,3,4-thiadiazole-2-amine (30 mg).
MSm/z(M+H):452.

<0042-3>

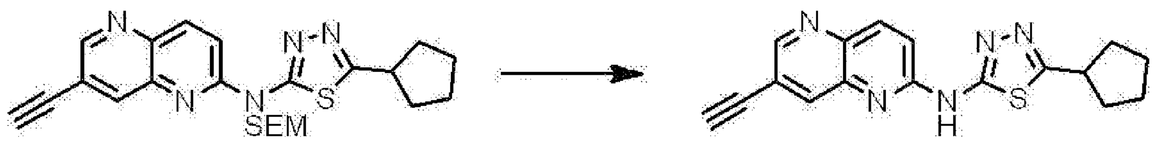

HCL salt

12 mol/L hydrochloric acid (0.4 mL) was added to a solution of 5-cyclopentyl-N-(7-ethynyl-1,5-naphthyridin-2-yl)-N-((2-(trimethylsilyl)ethoxy)methyl)-1,3,4-thiadiazole-2-amine (13 mg) in methanol (1.2 mL), followed by stirring at room temperature for 1.5 hours. The solvent was distilled off under reduced pressure, diisopropyl ether and water were added to the obtained residue, and the solid matter was collected by filtration, thereby obtaining 5-cyclopentyl-N-(7-ethynyl-1,5-naphthyridin-2-yl)-1,3,4-thiadiazole-2-amine hydrochloride (2.3 mg).
$^1$H-NMR(DMSO-$d_6$)δ:12.15(1H,brs),8.78(1H,d,J=1.8Hz),8.36(1H,d,1.8Hz),
8.31(1H,J=9.3Hz),7049(1H,d,J=9.3Hz),4.64(1H,s),3.54-3.38(1H,m),2.22-2.08(2H,m),1.92-1.64(6H,m).
MSm/z(M+H):322.

<Example 0043> (not in accordance with the present invention)

<0043-1>

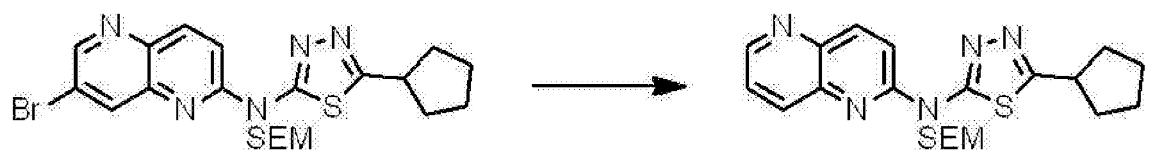

A mixture of N-(7-bromo-1,5-naphthyridin-2-yl)-5-cyclopentyl-N-((2-(trimethylsilyl)ethoxy)methyl)-1,3,4-thiadiazole-2-amine (37 mg), tetrakis(triphenylphosphine)palladium(0) (8 mg), ammonium formate (18 mg) and triethylamine (0.082 mL) in N-methylpyrrolidone (1 mL) was stirred at 120°C for 60 minutes using a microwave reaction apparatus. The reaction mixture was cooled to room temperature, and ethyl acetate and water were added thereto. The organic layer was collected by separation, washed sequentially with water and a saturated sodium chloride aqueous solution, and dried over anhydrous sodium sulfate. The obtained solution was purified by silica gel column chromatography (ethyl acetate-hexane), thereby obtaining 5-cyclopentyl-N-(1,5-naphthyridin-2-yl)-N-((2-(trimethylsilyl)ethoxy)methyl)-1,3,4-thiadiazole-2-amine (21 mg).
MSm/z(M+H):427.

<0043-2>

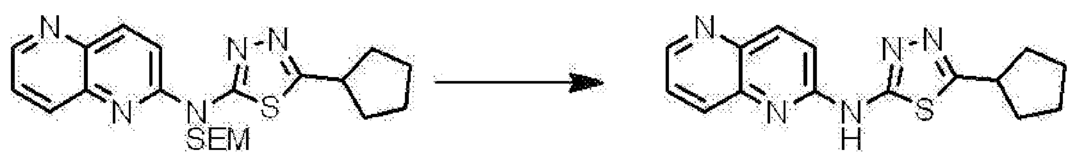

HCL salt

12 mol/L hydrochloric acid (0.4 mL) was added to a solution of 5-cyclopentyl-N-(1,5-naphthyridin-2-yl)-N-((2-(trimethylsilyl)ethoxy)methyl)-1,3,4-thiadiazole-2-amine (21 mg) in methanol (1.2 mL), followed by stirring at room temperature for 2 hours. The solvent was distilled off under reduced pressure, diisopropyl ether and water were added thereto, and the solid matter was collected by filtration, thereby obtaining 5-cyclopentyl-N-(1,5-naphthyridin-2-yl)-1,3,4-thiadiazole-2-amine hydrochloride (3.3 mg).
$^1$H-NMR(DMSO-$d_6$)δ:12.07(1H,brs),8.79-8.76(1H,m),8.30(1H,d,J=9.0Hz),8.24(1H,d,8.1Hz),7.72-7.67(1H,m),7049(1H,d,9.3Hz),3.54-3.38(1H,m), 2.22-2.08(2H,m),1.92-1.64(6H,m).
MSm/z(M+H):298.

<Example 0044>

<0044-1>

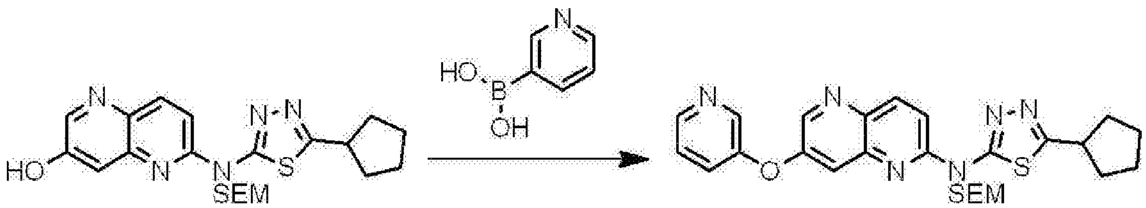

A mixture of 6-((5-cyclopentyl-1,3,4-thiadiazol-2-yl)((2-(trimethylsilyl)ethoxy)methyl)amino)-1,5-naphthyridin-3-ol (20 mg), 3-pyridylboronic acid (8 mg), copper(II) acetate (25 mg), molecular sieve 4A (20 mg) and triethylamine (0.063 mL) in dichloromethane (0.5 mL) was stirred at room temperature for 3 hours, and stirred at 50°C for 5 days. After the reaction mixture was cooled to room temperature, the insolubles were filtered off using celite, and ethyl acetate and water were added thereto. The organic layer was collected by separation, washed with a saturated sodium chloride aqueous solution, and dried over anhydrous sodium sulfate. The obtained solution was purified by silica gel column chromatography (ethyl acetate-hexane), thereby obtaining 5-cyclopentyl-N-(7-((pyridin-3-yl)oxy)-1,5-naphthyridin-2-yl)-N-((2-(trimethylsilyl)ethoxy)methyl)-1,3,4-thiadiazole-2-amine (3.5 mg).
MSm/z(M+H):521.

<0044-2>

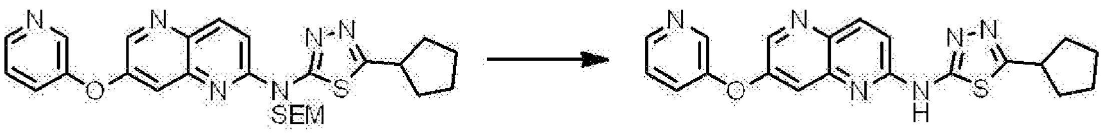

HCL salt

12 mol/L hydrochloric acid (0.4 mL) was added to a solution of 5-cyclopentyl-N-(7-((pyridin-3-yl)oxy)-1,5-naphthyridin-2-yl)-N-((2-(trimethylsilyl)ethoxy)methyl)-1,3,4-thiadiazole-2-amine (3.5 mg) in methanol (1.2 mL), followed by stirring at room temperature for 1.5 hours. The solvent was distilled off under reduced pressure, and diisopropyl ether, water, sodium hydroxide aqueous solution, and dichloromethane were added thereto. The organic layer was collected by separation, and dried over anhydrous sodium sulfate, and the solvent was distilled off under reduced pressure, thereby obtaining 5-cyclopentyl-N-(7-((pyridin-3-yl)oxy)-1,5-naphthyridin-2-yl)-1,3,4-thiadiazole-2-amine hydrochloride (1.68 mg).
$^1$H-NMR($CDCl_3$)δ:8.69(1H,d,J=2.7Hz),8.60-8.52(2H,m),8.31(1H,d,J=9.3Hz),7.65-7.60(2H,m),7.50-7.38(2H,m),3.58-3.50(1H,m),2.32-2.18(2H,m),2.00-1.72(6H,m).
MSm/z(M+H):391.

<Example 0045>

<0045-1>

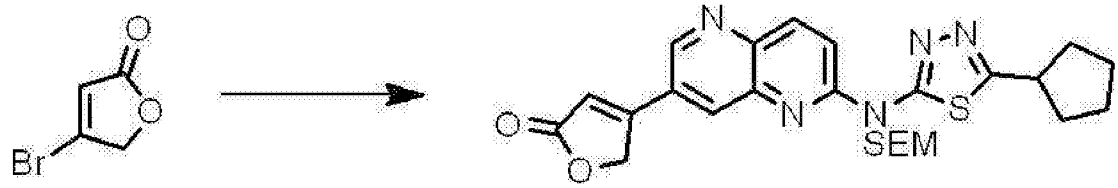

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II) dichloride (3 mg) and potassium acetate (8 mg) in 1,4-dioxane (0.6 mL) was stirred at 120°C for 50 minutes using a microwave reaction apparatus. 4-Bromofuran-2(5H)-one (13 mg), sodium carbonate (8 mg), and bis(di-tert-butyl (4-dimethylaminophenyl)phosphine)dichloropalladium(II) (7 mg) were added to the reaction mixture, followed by stirring at 120°C for 30 minutes using a microwave reaction apparatus. After the reaction mixture was cooled to room temperature, the insolubles were filtered off using celite, and ethyl acetate and water were added thereto. The organic layer was collected by separation, washed with a saturated sodium chloride aqueous solution, and dried over anhydrous sodium sulfate. The obtained solution was purified by silica gel column chromatography (ethyl acetate-hexane), thereby obtaining 4-(6-((5-cyclopentyl-1,3,4-thiadiazol-2-yl)((2-(trimethylsilyl)ethoxy)methyl)amino)-1,5-naphthyridin-3-yl)furan-2(5H)-one (20 mg).
MSm/z(M+H):510.

<0045-2>

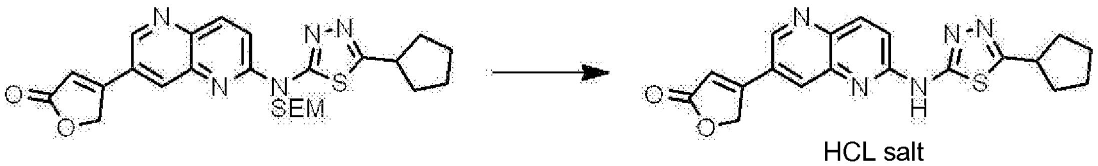


A4 mol/L hydrogen chloride/1,4-dioxane solution (0.4 mL) was added to a solution of 4-(6-((5-cyclopentyl-1,3,4-thiadiazol-2-yl)((2-(trimethylsilyl)ethoxy)methyl)amino)-1,5-naphthyridin-3-yl)furan-2(5H)-one (20 mg) in 1,4-dioxane (1.2 mL), followed by stirring at room temperature for 6 hours. The solvent was distilled off under reduced pressure, diisopropyl ether was added thereto, and the solid matter was collected by filtration, thereby obtaining 4-(6-((5-cyclopentyl-1,3,4-thiadiazol-2-yl)amino)-1,5-naphthyridin-3-yl)furan-2(5H)-one hydrochloride (8.0 mg).
$^1$H-NMR(DMSO-$d_6$)$\delta$:9.16(1H,d,J=2.7Hz),8.46(1H,d,J=2.7Hz),8.36(1H,d,9.3Hz), 7.57(1H,d,J=9.3Hz),7.14(1H,s),5.61(2H,s),3.54-3.44(1H,m),2.22-2.10(2H,m),1.94-1.66(6H,m).
MSm/z(M+H):380.

<Example 0046>

<0046-1>

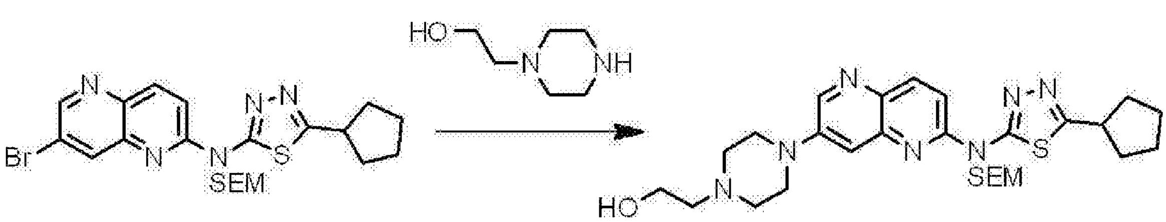

A mixture of N-(7-bromo-1,5-naphthyridin-2-yl)-5-cyclopentyl-N-((2-(trimethylsilyl)ethoxy)methyl)-1,3,4-thiadiazole-2-amine (27 mg), 2-(piperazin-1-yl)ethanol (0.013 mL), tris(dibenzylideneacetone)dipalladium(0) (5 mg), 2-(dicyclohexylphosphino)-2',4',6'-triisopropylbiphenyl (5 mg) and sodium tert-butoxide (20 mg) in 1,4-dioxane (1 mL) was

stirred at 100°C for 7 hours. After the reaction mixture was cooled to room temperature, the insolubles were filtered off using celite, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate), thereby obtaining 2-(4-(6-((5-cyclopentyl-1,3,4-thiadiazol-2-yl)((2-(trimethylsilyl)ethoxy)methyl)amino)-1,5-naphthyridin-3-yl)piperazin-1-yl)ethanol (10 mg).
MSm/z(M+H):556.

<0046-2>

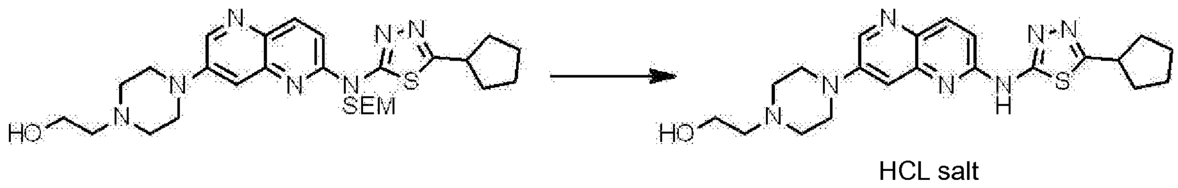


12 mol/L hydrochloric acid (0.4 mL) was added to a solution of 2-(4-(6-((5-cyclopentyl-1,3,4-thiadiazol-2-yl)((2-(trimethylsilyl)ethoxy)methyl)amino)-1,5-naphthyridin-3-yl)piperazin-1-yl)ethanol (10 mg) in methanol (1.2 mL), followed by stirring at room temperature for 3 hours. The solvent was distilled off under reduced pressure, diisopropyl ether and water were added to the obtained residue, and the solvent was distilled off under reduced pressure. Chloroform, methanol, and ethyl acetate were added to the obtained residue. The solid matter was collected by filtration, thereby obtaining 2-(4-(6-((5-cyclopentyl-1,3,4-thiadiazol-2-yl)amino)-1,5-naphthyridin-3-yl)piperazin-1-yl)ethanol hydrochloride (2.4 mg).
$^1$H-NMR(DMSO-$d_6$)δ:8.76(1H,d,J=2.7Hz),8.16(1H,d,J=8.7Hz),7.49(1H,d,J=2.7Hz),
7.25(1H,d,J=8.7Hz),4.23-4.15(2H,m),3.84-3.77(2H,m),3.66-3.52(3H,m),3.36-3.24(6H,m),2.22-2.10(2H,m),1.94-1.66(6H,m).
MSm/z(M+H):426.

<Example 0047>

<0047-1>

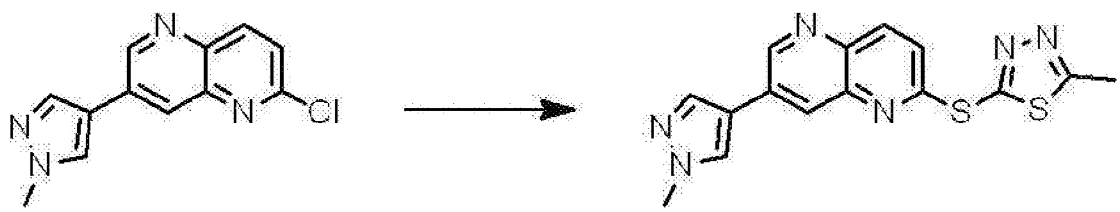

A mixture of 2-chloro-7-(1-methyl-1H-pyrazol-4-yl)-1,5-naphthyridine (10 mg), 5-methyl-1,3,4-thiadiazole-2-thiol (17 mg) and potassium carbonate (23 mg) in dimethylsulfoxide (0.5 mL) was stirred at 50°C for 4.5 hours, and stirred at 100°C for 4.5 hours. The reaction mixture was cooled to room temperature, and ethyl acetate and water were added thereto. The organic layer was collected by separation, washed sequentially with water and a saturated sodium chloride aqueous solution, and dried over anhydrous sodium sulfate. The obtained solution was purified by silica gel column chromatography (methanol-ethyl acetate), thereby obtaining 2-methyl-5-((7-(1-methyl-1H-pyrazol-4-yl)-1,5-naphthyridin-2-yl)thio)-1,3,4-thiadiazole (5.9 mg).
$^1$H-NMR(DMSO-$d_6$)δ:9.31(1H,d,J=2.1Hz),8.57(1H,s),8.52(1H,d,J=2.1Hz),
8.39(1H,d,J=8.4Hz),8.26(1H,s),7.83(1H,d,J=8.4Hz),3.93(3H,s),2.83(3H,s).
MSm/z(M+H):341.

<Example 0048>

<0048-1>

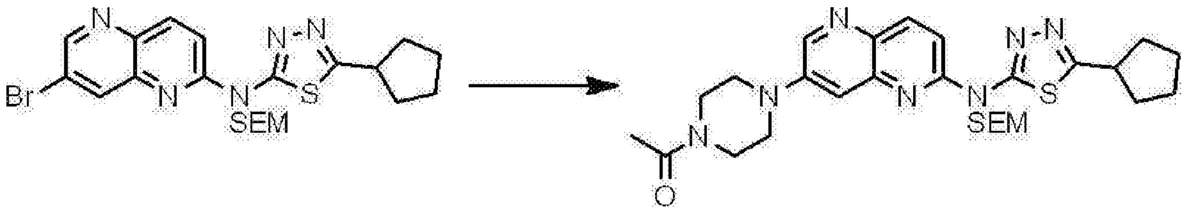

A mixture of N-(7-bromo-1,5-naphthyridin-2-yl)-5-cyclopentyl-N-((2-(trimethylsilyl)ethoxy)methyl)-1,3,4-thiadiazole-2-amine (35 mg), 1-(piperazin-1-yl)ethanone (18 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 22 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate), thereby obtaining 1-(4-(6-((5-cyclopentyl-1,3,4-thiadiazol-2-yl)((2-(trimethylsilyl)ethoxy)methyl)amino)-1,5-naphthyridin-3-yl)piperazin-1-yl)ethanone (16 mg).
MSm/z(M+H):554.

<0048-2>

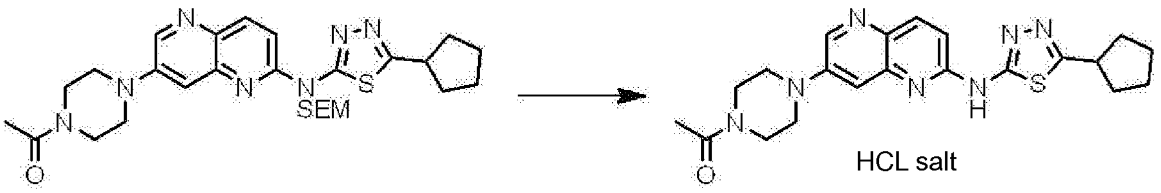


12 mol/L hydrochloric acid (0.4 mL) was added to a solution of 1-(4-(6-((5-cyclopentyl-1,3,4-thiadiazol-2-yl)((2-(trimethylsilyl)ethoxy)methyl)amino)-1,5-naphthyridin-3-yl)piperazin-1-yl)ethanone (16 mg) in methanol (1.2 mL), followed by stirring at room temperature for 3 hours. The solvent was distilled off under reduced pressure, diisopropyl ether and water were added to the obtained residue, and the solid matter was collected by filtration, thereby obtaining 1-(4-(6-((5-cyclopentyl-1,3,4-thiadiazol-2-yl)amino)-1,5-naphthyridin-3-yl)piperazin-1-yl)ethanone hydrochloride (12 mg).
$^1$H-NMR(DMSO-$d_6$)$\delta$:8.78(1H,d,J=2.7Hz),8.18(1H,d,J=9.3Hz),7.52(1H,d,J=2.7Hz),
7.26(1H,d,J=9.3Hz),3.69-3.28(9H,m),2.22-2.08(2H,m),2.08(3H,s),1.93-1.66(6H.m).
MSm/z(M+H):424.

<Example 0049>

<0049-1>

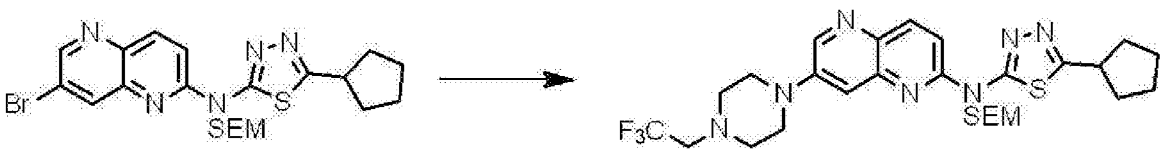

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 1-(2,2,2-trifluoroethyl)piperazine (13 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 21 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate), thereby obtaining 5-cyclopentyl-N-(7-(4-(2,2,2-trifluoroethyl)piperazin-1-yl)-1,5-naphthyridin-2-yl)-N-((2-(trimethylsilyl)ethoxy)methyl)-1,3,4-thiadiazole-2-amine (22 mg).
MSm/z(M+H):595.

<0049-2>

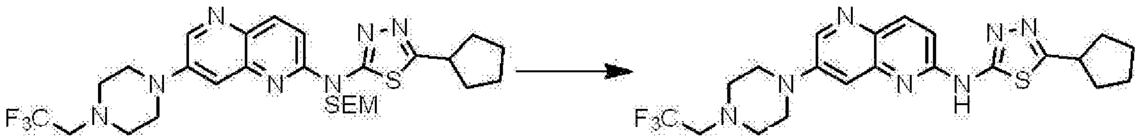

HCL salt

12 mol/L hydrochloric acid (0.4 mL) was added to a solution of 5-cyclopentyl-N-(7-(4-(2,2,2-trifluoroethyl)piperazin-1-yl)-1,5-naphthyridin-2-yl)-N-((2-(trimethylsilyl)ethoxy)methyl)-1,3,4-thiadiazole-2-amine (22 mg) in methanol (1.2 mL), followed by stirring at room temperature for 3 hours. The solvent was distilled off under reduced pressure, diisopropyl ether and water were added to the obtained residue, and the solid matter was collected by filtration, thereby obtaining 5-cyclopentyl-N-(7-(4-(2,2,2-trifluoroethyl)piperazin-1-yl)-1,5-naphthyridin-2-yl)-1,3,4-thiadiazole-2-amine hydrochloride (12 mg).
$^1$H-NMR(DMSO-d$^6$)δ:8.75(1H,d,J=2.7Hz),8.16(1H,d,J=9.3Hz),7.47(1H,d,J=2.7Hz),
7.24(1H,d,J=9.3Hz),3.66-3.28(7H,m),2.88-2.83(4H,m),2.22-2.06(2H,m),1.93-1.66(6H.m).
MSm/z(M+H):464.

<Example 0050>

<0050-1>

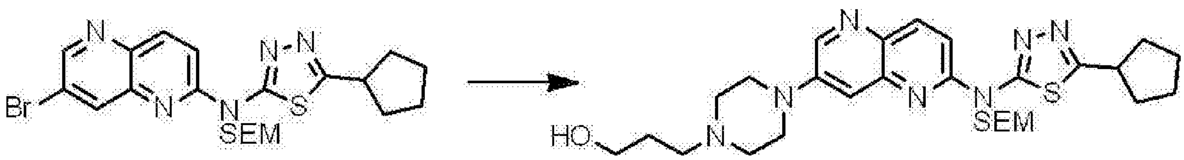

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 3-(piperazin-1-yl)propan-1-ol (12 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 21 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate), thereby obtaining 3-(4-(6-((5-cyclopentyl-1,3,4-thiadiazol-2-yl)((2-(trimethylsilyl)ethoxy)methyl)amino)-1,5-naphthyridin-3-yl)piperazin-1-yl)propan-1-ol (4.8 mg).
MSm/z(M+H):570.

<0050-2>

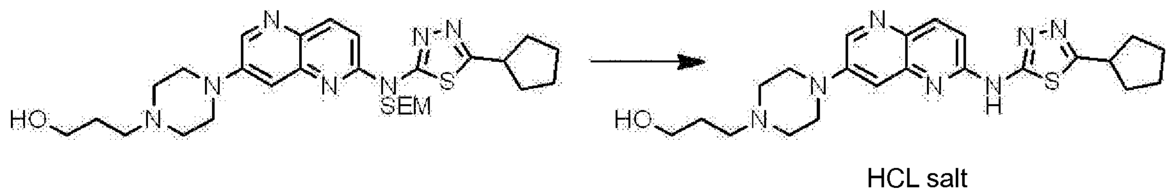


12 mol/L hydrochloric acid (0.4 mL) was added to a solution of 3-(4-(6-((5-cyclopentyl-1,3,4-thiadiazol-2-yl)((2-(trimethylsilyl)ethoxy)methyl)amino)-1,5-naphthyridin-3-yl)piperazin-1-yl)propan-1-ol (4.8 mg) in methanol (1.2 mL), followed by stirring at room temperature for 4.5 hours. The solvent was distilled off under reduced pressure, and diisopropyl ether and water were added to the obtained residue. The solid matter was collected by filtration, and purified by silica gel column chromatography (methanol-ethyl acetate), thereby obtaining 3-(4-(6-((5-cyclopentyl-1,3,4-thiadiazol-2-yl)amino)-1,5-naphthyridin-3-yl)piperazin-1-yl)propan-1-ol hydrochloride (0.7 mg).
$^1$H-NMR($CD_3OD$)$\delta$:8.64(1H,d,J=2.7Hz),8.09(1H,d,J=9.3Hz),7.53(1H,d,J=2.7Hz), 7.16(1H,d,J=9.3Hz),3.70-3.45(8H,m),2.81-2.74(4H,m),2.65-2.58(2H,m),2.30-2.18(2H,m),1.96-1.74(8H,m).
MSm/z(M+H):440.

<Example 0051>

<0051-1>

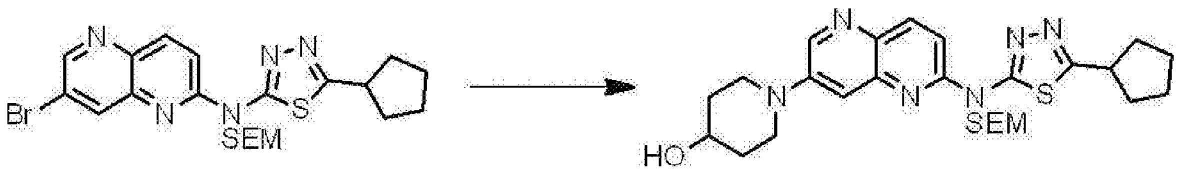

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), piperidin-4-ol (8 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 22 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate), thereby obtaining 1-(6-((5-cyclopentyl-1,3,4-thiadiazol-2-yl)((2-(trimethylsilyl)ethoxy)methyl)amino)-1,5-naphthyridin-3-yl)piperidin-4-ol (4.2 mg).
MSm/z(M+H):527.

<0051-2>

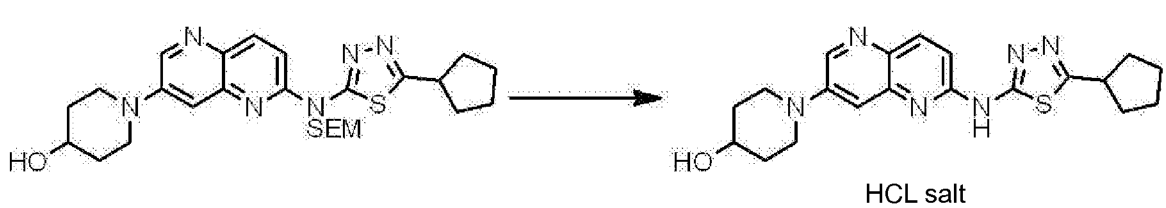


12 mol/L hydrochloric acid (0.4 mL) was added to a solution of 1-(6-((5-cyclopentyl-1,3,4-thiadiazol-2-yl)((2-(trimethylsilyl)ethoxy)methyl)amino)-1,5-naphthyridin-3-yl)piperidin-4-ol (4.8 mg) in methanol (1.2 mL), followed by stirring at room temperature for 4.5 hours. The solvent was distilled off under reduced pressure, and diisopropyl ether and water were added to the obtained residue. The solid matter was collected by filtration, and purified by silica gel column chromatography (methanol-ethyl acetate), thereby obtaining 1-(6-((5-cyclopentyl-1,3,4-thiadiazol-2-yl)amino)-1,5-naphthyridin-3-yl)piperidin-4-ol hydrochloride (1.3 mg).
$^1$H-NMR(DMSO-$d_6$)δ:11.83(1H,brs),8.69(1H,d,J=2.7Hz),8.09(1Hd,J=8.4Hz), 8.34(1H,d,J=2.7Hz),7.16(1H,d,J=8.4Hz),4.77(1H,d,J=4.5Hz),3.83-3.66(4H,m),3.54-3.33(1H,m),3.16-3.03(1H,m),2.20-2.08(2H,m), 1.94-1.46(10H,m).
MSm/z(M+H):397.

<Example 0052>

<0052-1>

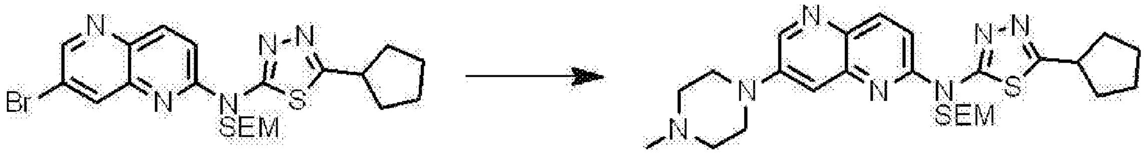

A mixture ofN-(7-bromo-1,5-naphthyridin-2-yl)-5-cyclopentyl-N-((2-(trimethylsilyl)ethoxy)methyl)-1,3,4-thiadiazole-2-amine (20 mg), 1-methylpiperazine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 21 hours. After the reaction mixture was cooled to room temperature, the insolubles were filtered off using celite, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate), thereby obtaining 5-cyclopentyl-N-(7-(4-methylpiperazin-1-yl)-1,5-naphthyridin-2-yl)-N-((2-(trimethylsilyl)ethoxy)methyl)-1,3,4-thiadiazole-2-amine (15 mg).
MSm/z(M+H):526.

<0052-2>

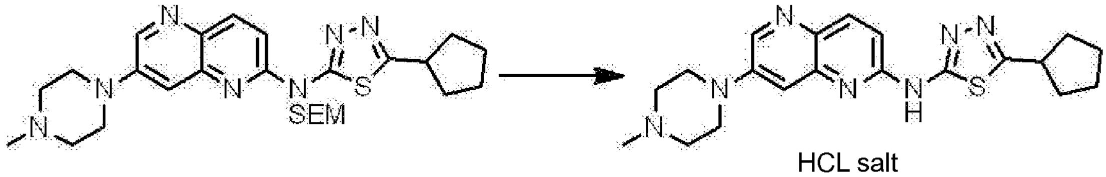

HCL salt

12 mol/L hydrochloric acid (0.8 mL) was added to a solution of 5-cyclopentyl-N-(7-(4-methylpiperazin-1-yl)-1,5-naphthyridin-2-yl)-N-((2-(trimethylsilyl)ethoxy)methyl)-1,3,4-thiadiazole-2-amine (15 mg) in methanol (2.4 mL), followed by stirring at room temperature for 3 days. The solid matter was collected by filtration, thereby obtaining 5-cyclopentyl-N-(7-(4-methylpiperazin-1-yl)-1,5-naphthyridin-2-yl)-1,3,4-thiadiazole-2-amine hydrochloride (8.7 mg). $^1$H-NMR(DMSO-$d_6$)δ:10.72(1H,brs),8.78(1H,d,J=2.7Hz),8.19(1H,d,J=9.0Hz), 7.58(1H,d,J=2.7Hz),7.28(1H,d,J=9.0Hz),4.25-4.16(2H,m),3.68-3.14(7H,m),2.86(3H,s),2.23-2.08(2H,m),1.92-1.64(6H, m).
MSm/z(M+H):396.

<Example 0053>

<0053-1>

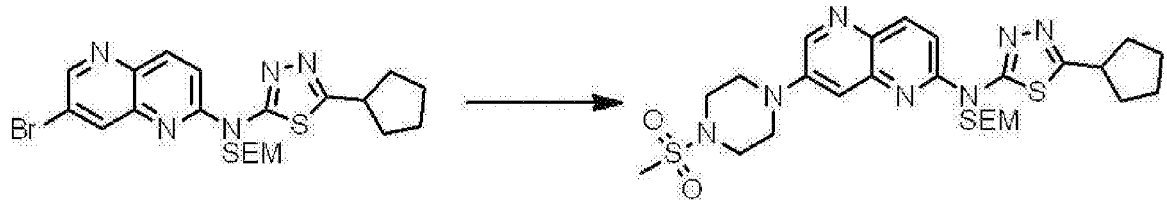

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 1-(methylsulfonyl)piperazine (15 mg), tris(dibenzylideneacetone)dipalladium(0) (6 mg), 2-(dicyclohexylphosphino)-2',4',6'-triisopropylbiphenyl (7 mg) and cesium carbonate (32 mg) in 1,4-dioxane (1 mL) was stirred at 100°C for 19 hours. After the reaction mixture was cooled to room temperature, the insolubles were filtered off using celite, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate), thereby obtaining 5-cyclopentyl-N-(7-(4-(methylsulfonyl)piperazin-1-yl)-1,5-naphthyridin-2-yl)-N-((2-(trimethylsilyl)ethoxy)methyl)-1,3,4-thiadiazole-2-amine (12 mg).
MSm/z(M+H):590.

<0053-2>

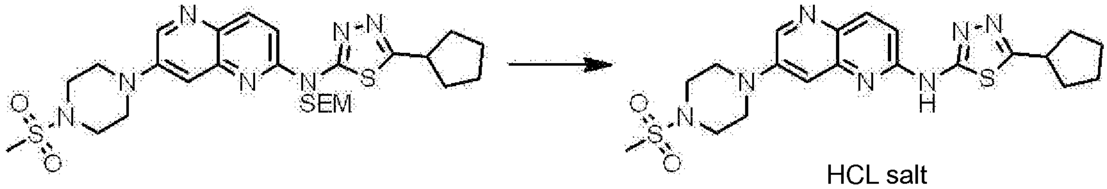

HCL salt

12 mol/L hydrochloric acid (0.4 mL) was added to a solution of 5-cyclopentyl-N-(7-(4-(methylsulfonyl)piperazin-1-yl)-1,5-naphthyridin-2-yl)-N-((2-(trimethylsilyl)ethoxy)methyl)-1,3,4-thiadiazole-2-amine (12 mg) in methanol (1.2 mL), followed by stirring at room temperature for 14 hours. The solvent was distilled off under reduced pressure, diisopropyl ether and methanol were added to the obtained residue, and the solid matter was collected by filtration, thereby obtaining 5-cyclopentyl-N-(7-(4-(methylsulfonyl)piperazin-1-yl)-1,5-naphthyridin-2-yl)-1,3,4-thiadiazole-2-amine hydrochloride (10 mg).
$^1$H-NMR(DMSO-$d_6$)δ:8.76(1H,d,J=2.7Hz),8.15(1H,d,J=9.3Hz),7.46(1H,d,J=2.7Hz),7.23(1H,d,J=9.3Hz),3.58-3.22(9H,m),2.96(3H,s),2.20-2.08(2H,m),1.94-1.64(6H,m).
MSm/z(M+H):460.

<Example 0054>

<0054-1>

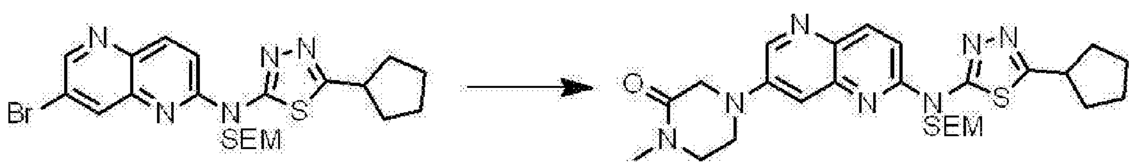

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 1-methylpiperazin-2-one (15 mg), tris(dibenzylideneacetone)dipalladium(0) (6 mg), 2-(dicyclohexylphosphino)-2',4',6'-triisopropylbiphenyl (7 mg) and cesium carbonate (32 mg) in 1,4-dioxane (1 mL) was stirred at 100°C for 19 hours. After the reaction mixture was cooled to room temperature, the insolubles were filtered off using celite, and ethyl acetate and water were added thereto. The organic layer was collected by separation, washed with a

saturated sodium chloride aqueous solution, and dried over anhydrous sodium sulfate. The obtained solution was purified by silica gel column chromatography (methanol-ethyl acetate), thereby obtaining 4-(6-((5-cyclopentyl-1,3,4-thiadiazol-2-yl)((2-(trimethylsilyl)ethoxy)methyl)amino)-1,5-naphthyridin-3-yl)-1-methylpiperazin-2-one (11 mg).
MSm/z(M+H):540.

<0054-2>

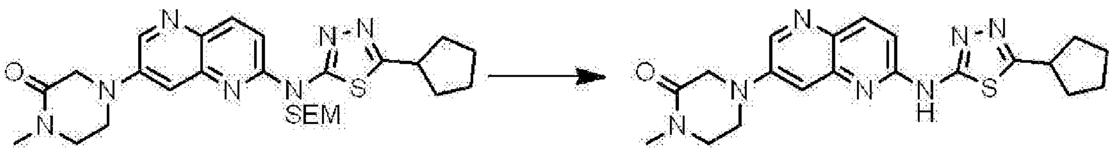

HCL salt

12 mol/L hydrochloric acid (0.4 mL) was added to a solution of 4-(6-((5-cyclopentyl-1,3,4-thiadiazol-2-yl)((2-(trimethylsilyl)ethoxy)methyl)amino)-1,5-naphthyridin-3-yl)-1-methylpiperazin-2-one (11 mg) in methanol (1.2 mL), followed by stirring at room temperature for 14 hours. The solvent was distilled off under reduced pressure, diisopropyl ether and methanol were added to the obtained residue, and the solid matter was collected by filtration, thereby obtaining 4-(6-((5-cyclopentyl-1,3,4-thiadiazol-2-yl)amino)-1,5-naphthyridin-3-yl)-1-methylpiperazin-2-one hydrochloride (4.1 mg).
$^1$H-NMR(DMSO-$d_6$)$\delta$:8.76(1H,d,J=2.7Hz),8.17(1H,d,J=8.7Hz),7.49(1H,d,J=2.7Hz), 7.24(1H,d,J=8.7Hz),4.06(2H,brs),3.81-3.75(2H,m),3.57-3.44(3H,m),2.94(3H,s),2.21-2.08(2H,m),1.94-1.64(6H,m).
MSm/z(M+H):410.

<Example 0055>

<0055-1>

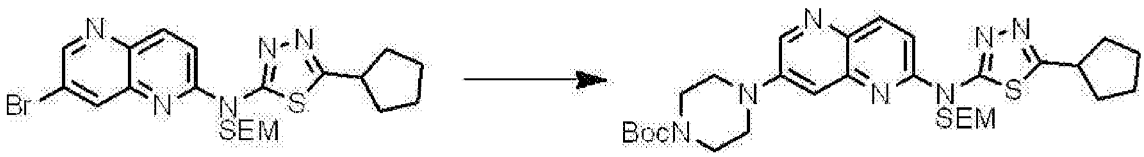

A mixture of N-(7-bromo-1,5-naphthyridin-2-yl)-5-cyclopentyl-N-((2-(trimethylsilyl)ethoxy)methyl)-1,3,4-thiadiazole-2-amine (94 mg), tert-butyl piperazine-1-carboxylate (52 mg), tris(dibenzylideneacetone)dipalladium(0) (17 mg), 2-(dicyclohexylphosphino)-2',4',6'-triisopropylbiphenyl (18 mg) and cesium carbonate (150 mg) in 1,4-dioxane (1 mL) was stirred at 100°C for 17 hours. After the reaction mixture was cooled to room temperature, the insolubles were filtered off using celite, and ethyl acetate and water were added thereto. The organic layer was collected by separation, washed with a saturated sodium chloride aqueous solution, and dried over anhydrous sodium sulfate. The obtained solution was purified by silica gel column chromatography (ethyl acetate-hexane), thereby obtaining tert-butyl 4-(6-((5-cyclopentyl-1,3,4-thiadiazol-2-yl)((2-(trimethylsilyl)ethoxy)methyl)amino)-1,5-naphthyridin-3-yl)piperazine-1-carboxylate (64 mg).
MSm/z(M+H):612.

<0055-2>

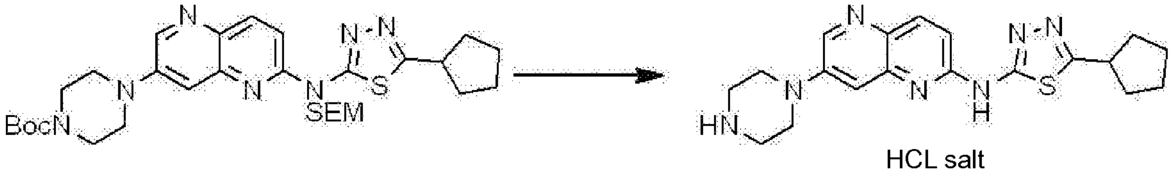

HCL salt

12 mol/L hydrochloric acid (2.4 mL) was added to a solution of tert-butyl 4-(6-((5-cyclopentyl-1,3,4-thiadiazol-2-yl)((2-(trimethylsilyl)ethoxy)methyl)amino)-1,5-naphthyridin-3-yl)piperazine-1-carboxylate (64 mg) in methanol (2.4 mL), followed by stirring at room temperature for 10 hours. The solvent was distilled off under reduced pressure, diisopropyl ether and methanol were added to the obtained residue, and the solid matter was collected by filtration, thereby obtaining 5-cyclopentyl-N-(7-(piperazin-1-yl)-1,5-naphthyridin-2-yl)-1,3,4-thiadiazole-2-amine hydrochloride (51 mg).
$^1$H-NMR(DMSO-$d_6$)$\delta$:9.02(1H.brs),8.76(1H,d,J=2.7Hz),8.17(1H,d,J=8.4Hz), 7.51(1H.d.J=2.7Hz),7.26(1H,d,J=804Hz),3.68-3.26(9H,m), 2.21-2.08(2H,m),1.94-1.64(6H,m).
MSm/z(M+H):381.

<Example 0056>

<0056-1>

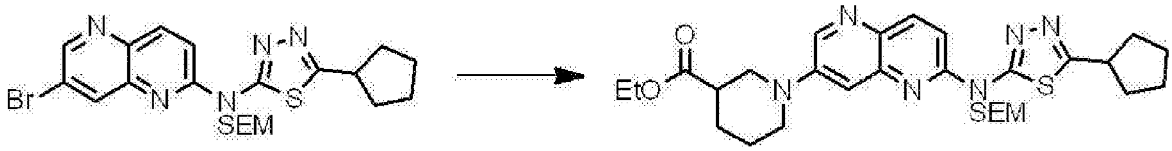

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), ethyl piperidine-3-carboxylate (15 mg), tris(dibenzylideneacetone)dipalladium(0) (6 mg), 2-(dicyclohexylphosphino)-2',4',6'-triisopropylbiphenyl (7 mg) and cesium carbonate (32 mg) in 1,4-dioxane (1 mL) was stirred at 100°C for 19 hours. After the reaction mixture was cooled to room temperature, the insolubles were filtered off using celite, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate), thereby obtaining ethyl 1-(6-((5-cyclopentyl-1,3,4-thiadiazol-2-yl)((2-(trimethylsilyl)ethoxy)methyl)amino)-1,5-naphthyridin-3-yl)piperidine-3-carboxylate (4.5 mg).
MSm/z(M+H):583.

<0056-2>

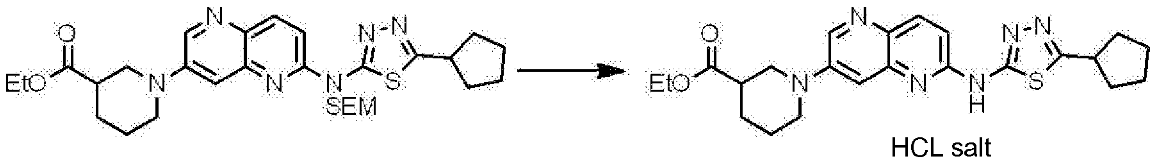


12 mol/L hydrochloric acid (0.4 mL) was added to a solution of ethyl 1-(6-((5-cyclopentyl-1,3,4-thiadiazol-2-yl)((2-(trimethylsilyl)ethoxy)methyl)amino)-1,5-naphthyridin-3-yl)piperidine-3-carboxylate (4.5 mg) in 1,4-dioxane (1.2 mL) and ethanol (0.08 mL), followed by stirring at room temperature for 8 hours. Diisopropyl ether was added to the reaction mixture, and the solid matter was collected by filtration, thereby obtaining ethyl 1-(6-((5-cyclopentyl-1,3,4-thiadiazol-2-yl)amino)-1,5-naphthyridin-3-yl)piperidine-3-carboxylate hydrochloride (1.0 mg).
$^1$H-NMR(DMSO-$d_6$)$\delta$:8.76(1H,d,J=2.7Hz),8.17(1H,d,J=9.0Hz),7.52(1H,d,J=2.7Hz), 7.25(1H,d,J=9.0Hz),4.10(2H,q,J=7.5Hz),3.95-3.20(6H,m),2.30-1.58(10H,m),2.08(3H,s),1.21(3H,t,J=7.5Hz).
MSm/z(M+H):453.

<Example 0057>

<0057-1>

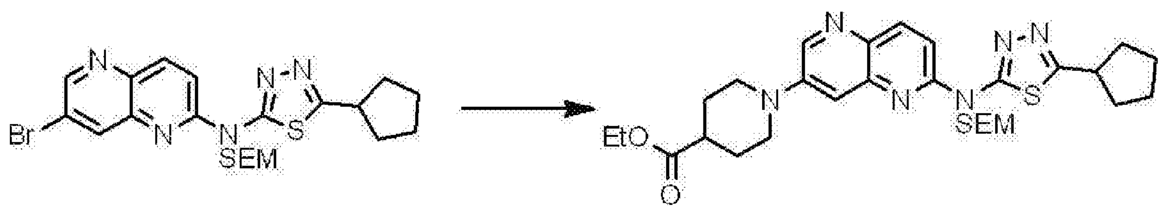

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), ethyl piperidine-4-carboxylate (15 mg), tris(dibenzylideneacetone)dipalladium(0) (6 mg), 2-(dicyclohexylphosphino)-2',4',6'-triisopropylbiphenyl (7 mg) and cesium carbonate (32 mg) in 1,4-dioxane (1 mL) was stirred at 100°C for 19 hours. After the obtained reaction mixture was cooled to room temperature, the insolubles were filtered off using celite, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate), thereby obtaining ethyl 1-(6-((5-cyclopentyl-1,3,4-thiadiazol-2-yl) ((2-(trimethylsilyl)ethoxy)methyl)amino)-1,5-naphthyridin-3-yl)piperidine-4-carboxylate (11 mg).
MSm/z(M+H):583.

<0057-2>

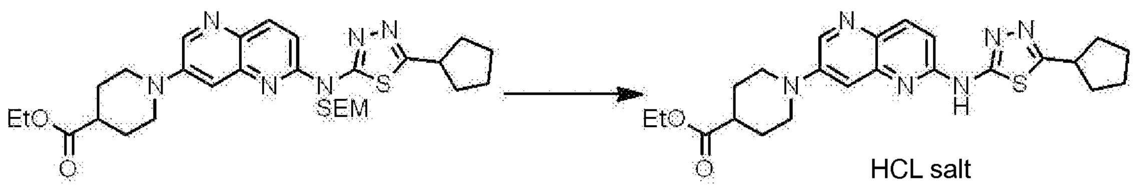


12 mol/L hydrochloric acid (0.4 mL) was added to a solution of ethyl 1-(6-((5-cyclopentyl-1,3,4-thiadiazol-2-yl)((2-(trimethylsilyl)ethoxy)methyl)amino)-1,5-naphthyridin-3-yl)piperidine-4-carboxylate (11 mg) in 1,4-dioxane (1.2 mL) and ethanol (0.08 mL), followed by stirring at room temperature for 8 hours. Diisopropyl ether was added to the reaction mixture, and the solid matter was collected by filtration, thereby obtaining ethyl 1-(6-((5-cyclopentyl-1,3,4-thiadiazol-2-yl)amino)-1,5-naphthyridin-3-yl)piperidine-4-carboxylate hydrochloride (7.3 mg).
$^1$H-NMR(DMSO-$d_6$)$\delta$:8.79(1H,d,J=2.7Hz),8.19(1H,d,J=8.4Hz),7.57(1H,d,J=2.7Hz),
7.26(1H,d,J=8.4Hz),4.10(2H,q,J=7.5Hz),4.02-3.94(2H,m),3.64-3.50(1H,m),3.12-3.00(2H,m),2.22-1.64(13H,m),1.21(3H,t,J=7.5Hz).
MSm/z(M+H):453.

<Example 0058>

<0058-1>

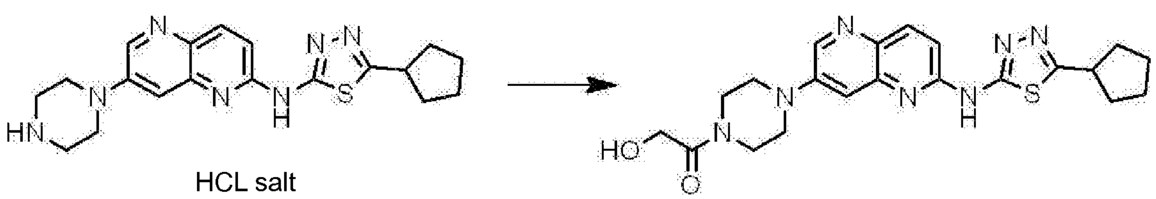


After a solution of 2-hydroxyacetic acid (6 mg), 1-ethyl-3-(3-dimethylaminopropyl)carbodiimide hydrochloride (15 mg), and 1-hydroxy-1H-benzotriazolemonohydrate (11 mg) in N,N-dimethylformamide (0.5 mL) was stirred at room temperature for 15 minutes, 5-cyclopentyl-N-(7-(piperazin-1-yl)-1,5-naphthyridin-2-yl)-1,3,4-thiadiazole-2-amine (10 mg) and triethylamine (0.037 mL) were added thereto, followed by stirring at room temperature for 2 days. Water was added to the reaction mixture, the solid matter was collected by filtration, thereby obtaining 1-(4-(6-((5-cyclopentyl-1,3,4-thiadiazol-2-yl)amino)-1,5-naphthyridin-3-yl)piperazin-1-yl)-2-hydroxyethanone (5.7 mg).
$^1$H-NMR($CD_3OD$)δ:8.66(1H,d,2.7Hz),8.10(1H,d,J=9.3Hz),7.55(1H,d,2.7Hz),
7.18(1H,d,J=9.3Hz),4.60(2H,brs),3.87-3.81(2H,m),3.70-3.64(2H,m),3.54-3.44(5H,m),2.32-2.18(2H,m),1.98-1.74(6H,m).
MSm/z(M+H):440.

<Example 0059>

<0059-1>

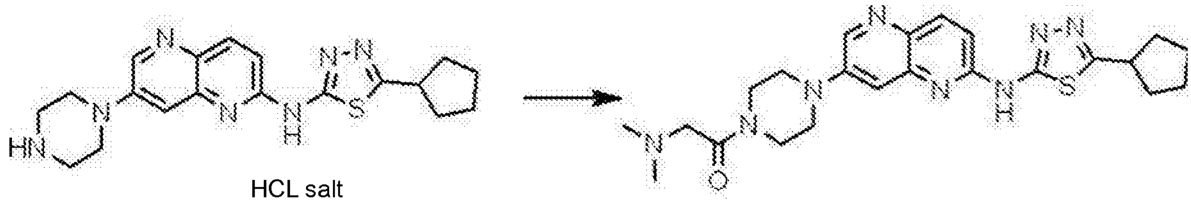


After a solution of 2-dimethylaminoacetic acid (8 mg), 1-ethyl-3-(3-dimethylaminopropyl)carbodiimide hydrochloride (15 mg), and 1-hydroxy-1H-benzotriazolemonohydrate (11 mg) in N,N-dimethylformamide (0.5 mL) was stirred at room temperature for 15 minutes, 5-cyclopentyl-N-(7-(piperazin-1-yl)-1,5-naphthyridin-2-yl)-1,3,4-thiadiazole-2-amine (10 mg) and triethylamine (0.037 mL) were added thereto, followed by stirring at room temperature for 2 days. Water was added to the reaction mixture, the solid matter was collected by filtration, thereby obtaining 1-(4-(6-((5-cyclopentyl-1,3,4-thiadiazol-2-yl)amino)-1,5-naphthyridin-3-yl)piperazin-1-yl)-2-(dimethylamino)ethanone (3.7 mg).
$^1$H-NMR($CD_3OD$)δ:8.65(1H,brs),8.09(1H,d,J=8.7Hz),7.53(1H,brs),7.16(1H,d,J=8.7Hz),
4.06(2H,s),3.86-3.78(4H,m),3.56-3.42(5H,m),2.35(6H,s),2.31-2.18(2H,m),1.98-1.72(6H,m).
MSm/z(M+H):467.

<Example 0060>

<0060-1>

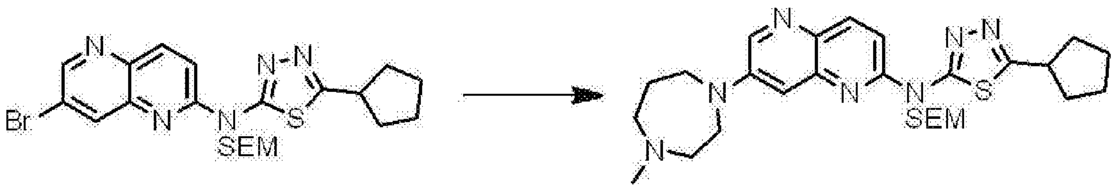

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 1-methyl-1,4-diazepane (15 mg), tris(dibenzylideneacetone)dipalladium(0) (6 mg), 2-(dicyclohexylphosphino)-2',4',6'-triisopropylbiphenyl (7 mg) and cesium carbonate (32 mg) in 1,4-dioxane (1 mL) was stirred at 100°C for 19 hours. After the reaction mixture was cooled to room temperature, the insolubles were filtered off using celite, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate, NH silica), thereby obtaining 5-cyclopentyl-N-(7-(4-methyl-1,4-diazepan-1-yl)-1,5-naphthyridin-2-yl)-N-((2-(trimethylsilyl)ethoxy)methyl)-1,3,4-thiadiazole-2-amine (5.2 mg).
MSm/z(M+H):540.

<0060-2>

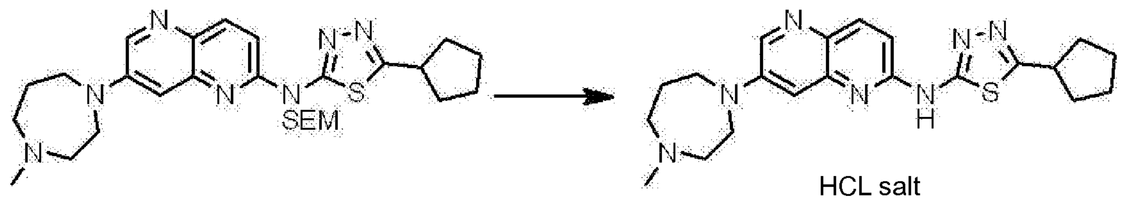


12 mol/L hydrochloric acid (0.4 mL) was added to a solution of 5-cyclopentyl-N-(7-(4-methyl-1,4-diazepan-1-yl)-1,5-naphthyridin-2-yl)-N-((2-(trimethylsilyl)ethoxy)methyl)-1,3,4-thiadiazole-2-amine (11 mg) in methanol (1.2 mL), followed by stirring at room temperature for 14 hours. The solvent was distilled off under reduced pressure, diisopropyl ether and methanol were added to the obtained residue, and the solid matter was collected by filtration, thereby obtaining 5-cyclopentyl-N-(7-(4-methyl-1,4-diazepan-1-yl)-1,5-naphthyridin-2-yl)-1,3,4-thiadiazole-2-amine hydrochloride (6.6 mg).
$^1$H-NMR($CD_3OD$)$\delta$:8.48(1H,d,J=2.7Hz),8.04(1H,d,J=8.7Hz),7.30(1H,d,J=2.7Hz), 7.06(1H,d,J=8.7Hz),3.81-3.75(2H,m),3.73-3.64(2H,m),3.57-3.44(1H,m),2.89-2.83(2H,m),2.71-2.65(2H,m),2.41(3H,s), 2.29-1.76(10H,m).
MSm/z(M+H):410.

<Example 0061>

<0061-1>

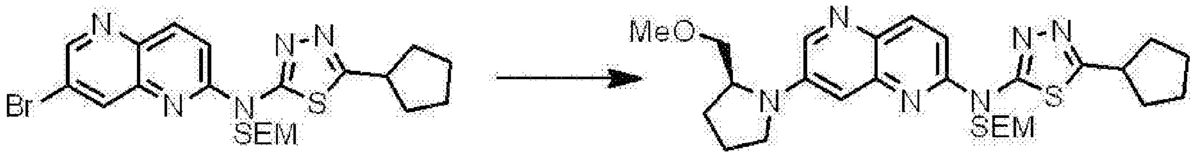

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), (S)-2-(methoxymethyl)pyrrolidine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 20 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained solution was purified by silica gel column chromatography (ethyl acetate-hexane), thereby obtaining (S)-5-cyclopentyl-N-(7-(2-(methoxymethyl)pyrrolidin-1-yl)-1,5-naphthyridin-2-yl)-N-((2-(trimethylsilyl)ethoxy)methyl)-1,3,4-thiadiazole-2-amine (6.5 mg).
MSm/z(M+H):541.

<0061-2>

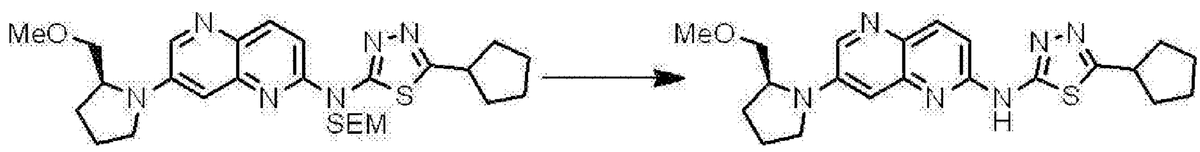

12 mol/L hydrochloric acid (0.4 mL) was added to a solution of (S)-5-cyclopentyl-N-(7-(2-(methoxymethyl)pyrrolidin-1-yl)-1,5-naphthyridin-2-yl)-N-((2-(trimethylsilyl)ethoxy)methyl)-1,3,4-thiadiazole-2-amine (6.5 mg) in methanol (1.2 mL), followed by stirring at room temperature for 4 days. The solvent was distilled off under reduced pressure, diisopropyl ether and methanol were added to the obtained residue, and the solid matter was collected by filtration, thereby obtaining (S)-5-cyclopentyl-N-(7-(2-(methoxymethyl)pyrrolidin-1-yl)-1,5-naphthyridin-2-yl)-1,3,4-thiadiazole-2-amine hydrochloride (2.9 mg).
$^1$H-NMR($CD_3OD$)$\delta$:8.53(1H,d,J=2.7Hz),8.17(1H,d,J=9.3Hz),7.26(1H,d,J=2.7Hz),

7.19(1H,d,J=9.3Hz),4.31-4.23(1H,m),3.30(3H,s),2.21-1.64(17H,m).
MSm/z(M+H):411.

<Example 0062>

<0062-1>

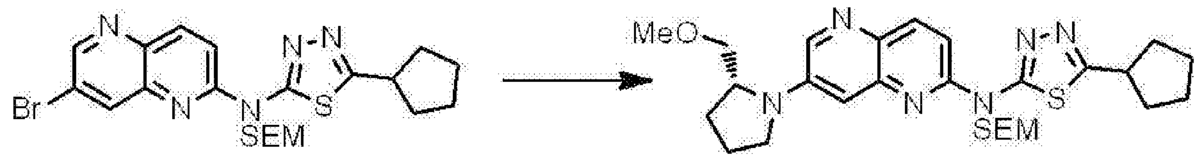

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), (R)-2-(methoxymethyl)pyrrolidine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 20 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained solution was purified by silica gel column chromatography (ethyl acetate-hexane), thereby obtaining (R)-5-cyclopentyl-N-(7-(2-(methoxymethyl)pyrrolidin-1-yl)-1,5-naphthyridin-2-yl)-N-((2-(trimethylsilyl)ethoxy)methyl)-1,3,4-thiadiazole-2-amine (5.4 mg).
MSm/z(M+H):411.

<0062-2>

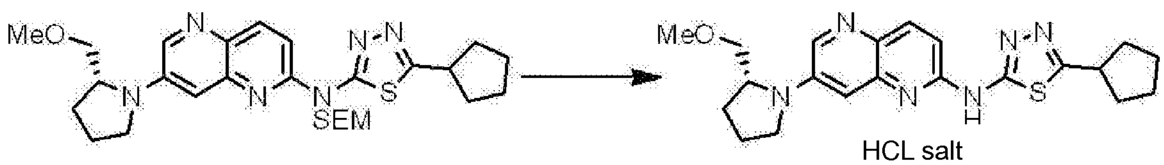

HCL salt

12 mol/L hydrochloric acid (0.4 mL) was added to a solution of (R)-5-cyclopentyl-N-(7-(2-(methoxymethyl)pyrrolidin-1-yl)-1,5-naphthyridin-2-yl)-N-((2-(trimethylsilyl)ethoxy)methyl)-1,3,4-thiadiazole-2-amine (5.4 mg) in methanol (1.2 mL), followed by stirring at room temperature for 4 days. The solvent was distilled off under reduced pressure, diisopropyl ether and methanol were added to the obtained residue, and the solid matter was collected by filtration, thereby obtaining (R)-5-cyclopentyl-N-(7-(2-(methoxymethyl)pyrrolidin-1-yl)-1,5-naphthyridin-2-yl)-1,3,4-thiadiazole-2-amine hydrochloride (1.5 mg).
$^1$H-NMR($CD_3OD$)$\delta$:8.53(1H,d,J=2.7Hz),8.17(1H,d,J=9.3Hz),7.26(1H,d,J=2.7Hz),
7.19(1H,d,J=9.3Hz),4.31-4.23(1H,m),3.30(3H,s),2.21-1.64(17H,m).
MSm/z(M+H):411.

<Example 0063>

<0063-1>

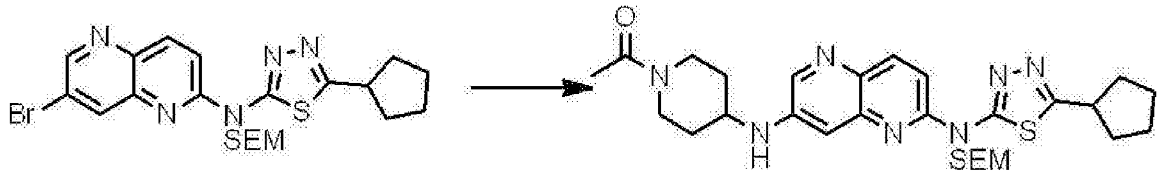

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 1-(4-aminopiperidin-1-yl)ethanone (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 6 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate), thereby obtaining 1-(4-((6-((5-cyclopentyl-1,3,4-thiadiazol-2-yl)((2-(trimethylsilyl)ethoxy)methyl)amino)-1,5-naphthyridin-3-yl)amino)piperidin-1-yl)ethanone (20 mg).
MSm/z(M+H):568.

<0063-2>

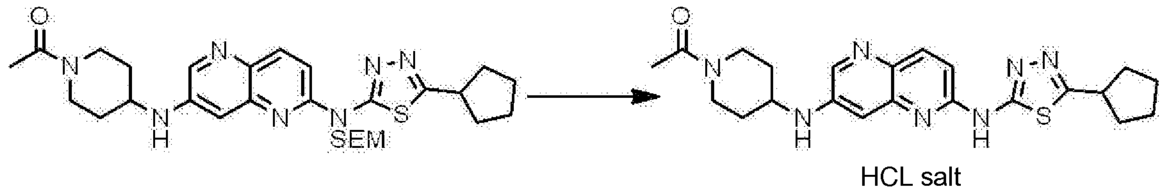

12 mol/L hydrochloric acid (0.4 mL) was added to a solution of 1-(4-((6-((5-cyclopentyl-1,3,4-thiadiazol-2-yl)((2-(trimethylsilyl)ethoxy)methyl)amino)-1,5-naphthyridin-3-yl)amino)piperidin-1-yl)ethanone (20 mg) in methanol (1.2 mL), followed by stirring at room temperature for 16 hours. The solvent was distilled off under reduced pressure, diisopropyl ether and ethanol were added to the obtained residue, and the solid matter was collected by filtration, thereby obtaining 1-(4-((6-((5-cyclopentyl-1,3,4-thiadiazol-2-yl)amino)-1,5-naphthyridin-3-yl)amino)piperidin-1-yl)ethanone hydrochloride (14 mg).
$^1$H-NMR(DMSO-$d_6$)δ:8.37(1H,d,J=2.7Hz),8.07(1H,d,J=8.4Hz),7.17(1H,d,J=2.7Hz), 7.11(1H,d,J=8.4Hz),4.28-4.17(2H,m),3.86-3.74(4H,m),2.99-2.87(2H,m),2.22-1.64(10H,m),2.03(3H,s).
MSm/z(M+H):438.

<Example 0064>

<0064-1>

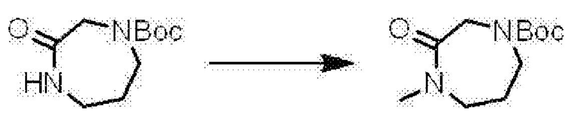

Iodomethane (0.078 mL) and 60% sodium hydride (50 mg) were added sequentially to a mixture solution of tert-butyl 3-oxo-1,4-diazepane-1-carboxylate (180 mg) in tetrahydrofuran (2 mL) and acetonitrile (2 mL) at room temperature, followed by stirring for 5 hours. Ethyl acetate and water were added sequentially to the reaction mixture. The organic layer was collected by separation, washed with a saturated sodium chloride aqueous solution, and dried over anhydrous sodium sulfate, and the solvent was distilled off under reduced pressure, thereby obtaining tert-butyl 4-methyl-3-oxo-1,4-diazepane-1-carboxylate (225 mg).
$^1$H-NMR($CDCl_3$)δ:3.62-3.53(4H,m),3.41(2H,t,J=4.8Hz),3.01(3H,s),2.65(2H,t,J=5.4Hz), 1.47(9H,s).

<0064-2>

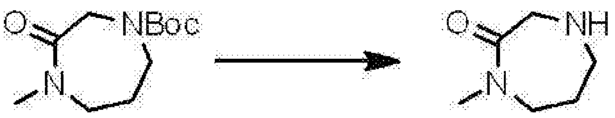

Water (0.4 mL) and trifluoroacetic acid (4 mL) were added sequentially to a solution of tert-butyl 4-methyl-3-oxo-1,4-diazepane-1-carboxylate (225 mg) in dichloromethane (4 mL) at room temperature, followed by stirring for 21 hours. The solvent was distilled off under reduced pressure, and the obtained residue was purified by silica gel column chromatography (methanol-ethyl acetate-hexane, NH silica), thereby obtaining 1-methyl-1,4-diazepan-2-one (85 mg).
$^1$H-NMR($CDCl_3$)$\delta$:3.45-3.40(2H,m),3.00(3H,s),2.97-2.92(4H,m),2.69-2.64(2H,m).

<0064-3>

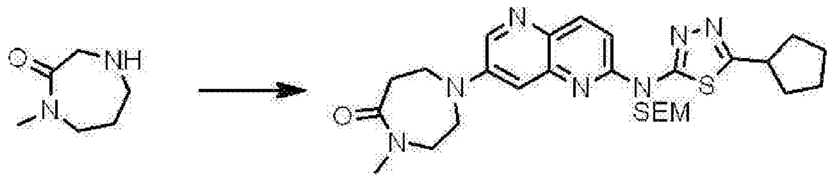

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 1-methyl-1,4-diazepan-2-one (15 mg), tris(dibenzylideneacetone)dipalladium(0) (6 mg), 2-(dicyclohexylphosphino)-2',4',6'-triisopropylbiphenyl (7 mg) and cesium carbonate (32 mg) in 1,4-dioxane (1 mL) was stirred at 100°C for 6 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate), thereby obtaining 1-(6-((5-cyclopentyl-1,3,4-thiadiazol-2-yl)((2-(trimethylsilyl)ethoxy)methyl)amino)-1,5-naphthyridin-3-yl)-4-methyl-1,4-diazepan-5-one (14 mg).
MSm/z(M+H):554.

<0064-4>

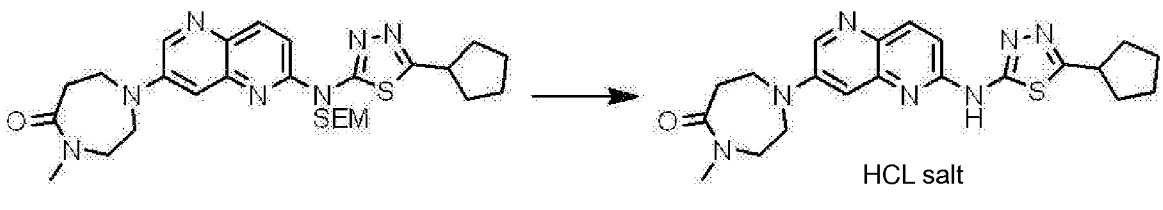

A 4 mol/L hydrogen chloride/1,4-dioxane solution (0.4 mL) was added to a solution of 1-(6-((5-cyclopentyl-1,3,4-thiadiazol-2-yl)((2-(trimethylsilyl)ethoxy)methyl)amino)-1,5-naphthyridin-3-yl)-4-methyl-1,4-diazepan-5-one (14 mg) in 1,4-dioxane (1.2 mL), followed by stirring at room temperature for 16 hours. Diisopropyl ether and ethanol were added to the reaction mixture, and the solid matter was collected by filtration, thereby obtaining 1-(6-((5-cyclopentyl-1,3,4-thiadiazol-2-yl)amino)-1,5-naphthyridin-3-yl)-4-methyl-1,4-diazepan-5-one hydrochloride (11 mg).
$^1$H-NMR(DMSO-$d_6$)$\delta$:8.71(1H,2.4Hz),8.15(1H,d,J=9.3Hz),7.43(1H,d,J=2.4Hz), 7.20(1H,d,J=9.3Hz),3.76-3.42(7H,m),2.80-2.71(2H,m),2.22-2.08(2H,m),2.89(3H,s), 1.94-1.64(6H,m).
MSm/z(M+H):424.

<Example 0065>

<0065-1>

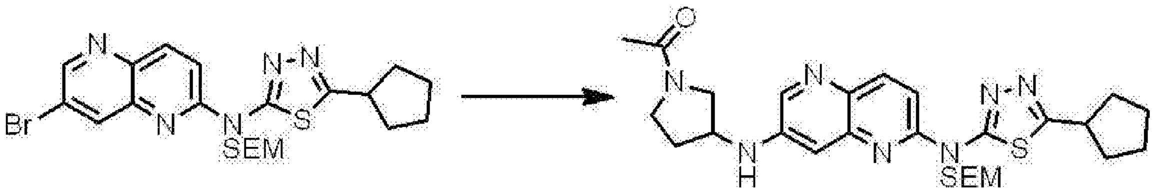

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 1-(3-aminopyrrolidin-1-yl)ethanone (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 6 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate), thereby obtaining 1-(3-((6-((5-cyclopentyl-1,3,4-thiadiazol-2-yl)((2-(trimethylsilyl)ethoxy)methyl)amino)-1,5-naphthyridin-3-yl)amino)pyrrolidin-1-yl)ethanone (17 mg).
MSm/z(M+H):554.

<0065-2>

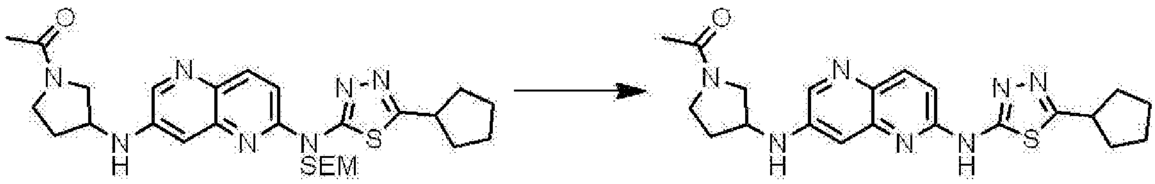

HCL salt

A 4 mol/L hydrogen chloride/1,4-dioxane solution (0.4 mL) was added to a solution of 1-(3-((6-((5-cyclopentyl-1,3,4-thiadiazol-2-yl)((2-(trimethylsilyl)ethoxy)methyl)amino)-1,5-naphthyridin-3-yl)amino)pyrrolidin-1-yl)ethanone (17 mg) in 1,4-dioxane (1.2 mL), followed by stirring at room temperature for 13 hours. The solvent was distilled off under reduced pressure, diisopropyl ether and methanol were added to the obtained residue, and the solid matter was collected by filtration, thereby obtaining 1-(3-((6-((5-cyclopentyl-1,3,4-thiadiazol-2-yl)amino)-1,5-naphthyridin-3-yl)amino)pyrrolidin-1-yl)ethanone hydrochloride (11 mg).
$^1$H-NMR(DMSO-$d_6$)δ:8.38-8.33(1H,m),8.08(1H,d,J=9.3Hz),7.15-7.11(1H,m),7.13(1H,d,J=9.3Hz),3.68-3.42(6H,m),2.22-2.08(2H,m),1.97(3H,s),1.92-1.64(8H,m).
MSm/z(M+H):424.

<Example 0066>

<0066-1>

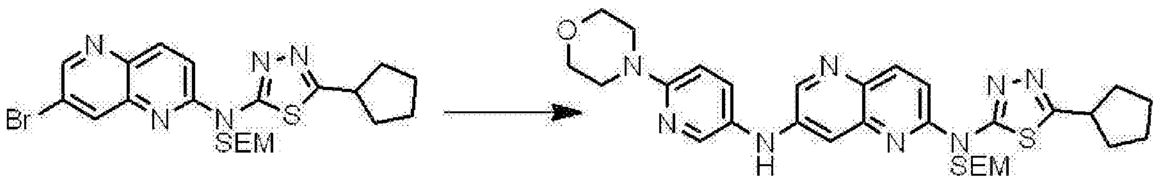

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 6-morpholinopyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 6 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate-hexane), thereby obtaining $N^2$-(5-cyclopentyl-1,3,4-thiadiazol-2-yl)-$N^7$-(6-morpholinopyridin-3-yl)-$N^2$-((2-(trimethylsilyl)ethoxy)methyl)-1,5-naphthyridine-2,7-diamine (15 mg).
MSm/z(M+H):605.

<0066-2>

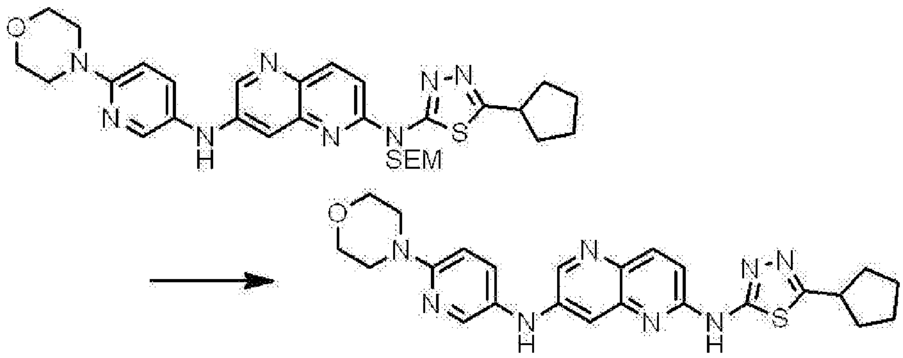

HCL salt

12 mol/L hydrochloric acid (0.4 mL) was added to a solution of $N^2$-(5-cyclopentyl-1,3,4-thiadiazol-2-yl)-$N^7$-(6-morpholinopyridin-3-yl)-$N^2$-((2-(trimethylsilyl)ethoxy)methyl)-1,5-naphthyridine-2,7-diamine (17 mg) in methanol (1.2 mL), followed by stirring at room temperature for 14 hours. The solvent was distilled off under reduced pressure, diisopropyl ether and methanol were added to the obtained residue, and the solid matter was collected by filtration, thereby obtaining $N^2$-(5-cyclopentyl-1,3,4-thiadiazol-2-yl)-$N^7$-(6-morpholinopyridin-3-yl)-1,5-naphthyridine-2,7-diamine hydrochloride (13 mg).
$^1$H-NMR(DMSO-$d_6$)$\delta$:8.97(1H,brs),8.52(1H,d,J=2.7Hz),8.16(1H,d,J=9.3Hz),
8.07(1H,d,J=2.7Hz),7.90(1H,dd,J=9.9,2.7Hz),7.41(1H,d,J=2.7Hz),7.28(1H,d,J=9.9Hz),
7.22(1H.d.J=9.3Hz),3.80-3.68(7H,m),2.18-2.04(2H,m),1.88-1.62(8H,m).
MSm/z(M+H):475.

<Example 0067>

<0067-1>

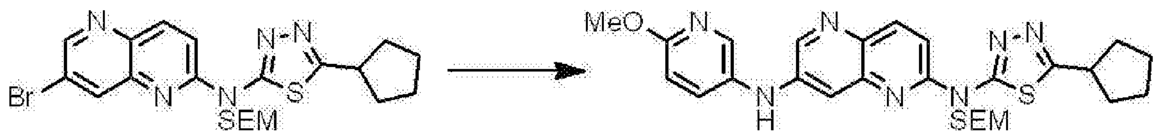

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 6-methoxypyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 6 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained solution was purified by silica gel column chromatography (ethyl acetate-hexane), thereby obtaining $N^2$-(5-cyclopentyl-1,3,4-thiadiazol-2-yl)-$N^7$-(6-methoxypyridin-3-yl)-$N^2$-((2-(trimethylsilyl)ethoxy)methyl)-1,5-naphthyridine-2,7-diamine (10 mg).
MSm/z(M+H):550.

<0067-2>

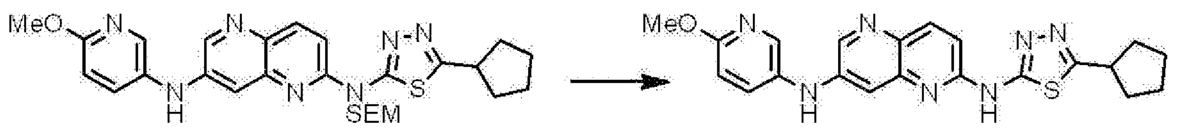


12 mol/L hydrochloric acid (0.4 mL) was added to a solution of $N^2$-(5-cyclopentyl-1,3,4-thiadiazol-2-yl)-$N^7$-(6-methoxypyridin-3-yl)-$N^2$-((2-(trimethylsilyl)ethoxy)methyl)-1,5-naphthyridine-2,7-diamine (10 mg) in methanol (1.2 mL), followed by stirring at room temperature for 2 days. The solvent was distilled off under reduced pressure, diisopropyl ether and methanol were added to the obtained residue, and the solid matter was collected by filtration, thereby obtaining $N^2$-(5-cyclopentyl-1,3,4-thiadiazol-2-yl)-$N^7$-(6-methoxypyridin-3-yl)-1,5-naphthyridine-2,7-diamine hydrochloride (5.3 mg).
$^1$H-NMR(DMSO-$d_6$)δ:8.75(1H,brs),8.49(1H,d,2.4Hz),8.16(1H,d,J=2.7Hz),
8.11(1H,d,J=8.4Hz),7.73(1H,dd,J=8.7,2.7Hz),7.32(1H,d,2.4Hz),7.17(1H,d,J=8.7Hz),
6.93(1H,d,J=8.4Hz),3.87(3H,s),3.54-3.42(1H,m),2.16-2.04(2H,m),1.88-1.60(6H,m).
MSm/z(M+H):420.

<Example 0068>

<0068-1>

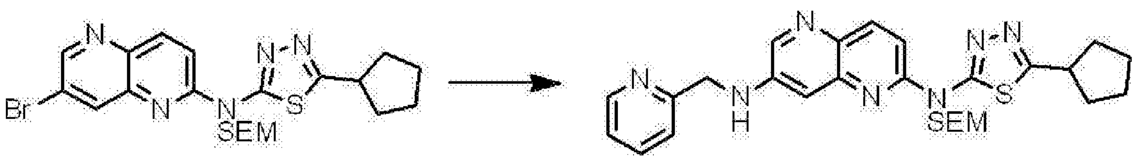

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), (pyridin-2-yl)methanamine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 16 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate-hexane), thereby obtaining $N^2$-(5-cyclopentyl-1,3,4-thiadiazol-2-yl)-$N^7$-((pyridin-2-yl)methyl)-$N^2$-((2-(trimethylsilyl)ethoxy)methyl)-1,5-naphthyridine-2,7-diamine (3.2 mg).
MSm/z(M+H):534.

<0068-2>

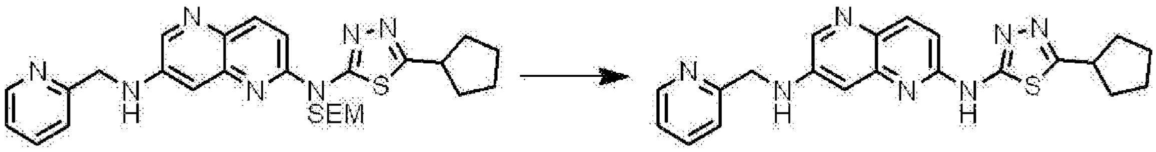

HCL salt

12 mol/L hydrochloric acid (0.4 mL) was added to a solution of N2-(5-cyclopentyl-1,3,4-thiadiazol-2-yl)-N7-((pyridin-2-yl)methyl)-N2-((2-(trimethylsilyl)ethoxy)methyl)-1,5-naphthyridine-2,7-diamine (3.2 mg) in methanol (1.2 mL), followed by stirring at room temperature for 6 hours. The solvent was distilled off under reduced pressure, diisopropyl ether and methanol were added to the obtained residue, and the solid matter was collected by filtration, thereby obtaining N2-(5-cyclopentyl-1,3,4-thiadiazol-2-yl)-N7-((pyridin-2-yl)methyl)-1,5-naphthyridine-2,7-diamine hydrochloride (2.5 mg). $^1$H-NMR(DMSO-$d_6$)δ:8.67-8.63(1H,m),8.45(1H,d,J=2.4Hz),8.05(1H,d,J=8.7Hz),7.99-7.93(1H,m),7.63-7.38(2H,m),7.08(1H,d,J=8.7Hz), 7.02(1H,d,J=2.4Hz),3.54-3.42(3H,m),2.20-2.06(2H,m),1.88-1.62(6H,m).
MSm/z(M+H):404.

<Example 0069>

<0069-1>

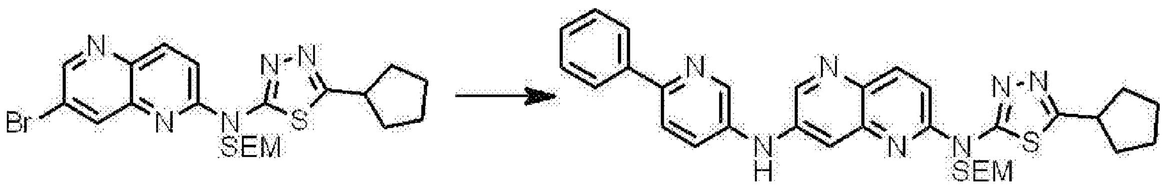

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 6-phenylpyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 6 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained solution was purified by silica gel column chromatography (ethyl acetate-hexane), thereby obtaining N2-(5-cyclopentyl-1,3,4-thiadiazol-2-yl)-N7-(6-phenylpyridin-3-yl)-N2-((2-(trimethylsilyl)ethoxy)methyl)-1,5-naphthyridine-2,7-diamine (11 mg).
MSm/z(M+H):596.

<0069-2>

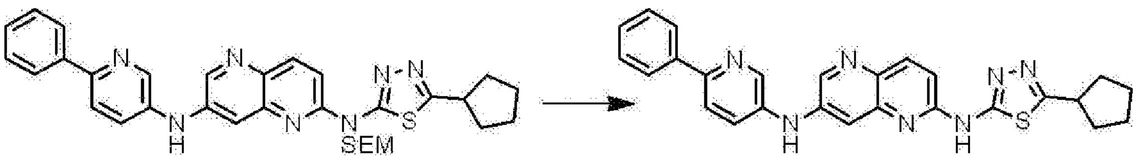

HCL salt

12 mol/L hydrochloric acid (0.6 mL) was added to a solution of N2-(5-cyclopentyl-1,3,4-thiadiazol-2-yl)-N7-(6-phenylpyridin-3-yl)-N2-((2-(trimethylsilyl)ethoxy)methyl)-1,5-naphthyridine-2,7-diamine (11 mg) in methanol (0.6 mL), followed by stirring at room temperature for 15 hours. The solvent was distilled off under reduced pressure, ethyl acetate and methanol were added to the obtained residue, and the solid matter was collected by filtration, thereby obtaining N2-(5-cyclopentyl-1,3,4-thiadiazol-2-yl)-N7-(6-phenylpyridin-3-yl)-1,5-naphthyridine-2,7-diamine hydrochloride (5.8 mg).

$^1$H-NMR(DMSO-$d_6$)δ:13.85(1H,brs),11.72(1H,brs),9.90(1H,brs),9.69(1H,brs),8.71-8.61(2H,m),8.35-8.17(3H,m),8.10-8.00(2H,m),7.58-7.42(3H,m),4.31-4.23(1H,m),1.92-1.58(8H,m).
MSm/z(M+H):466.

<Example 0070>

<0070-1>

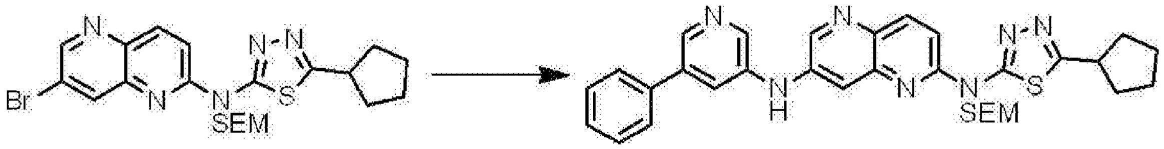

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 5-phenylpyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 6 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate-hexane), thereby obtaining $N^2$-(5-cyclopentyl-1,3,4-thiadiazol-2-yl)-$N^7$-(5-phenylpyridin-3-yl)-$N^2$-((2-(trimethylsilyl)ethoxy)methyl)-1,5-naphthyridine-2,7-diamine (8.6 mg).
MSm/z(M+H):596.

<0070-2>

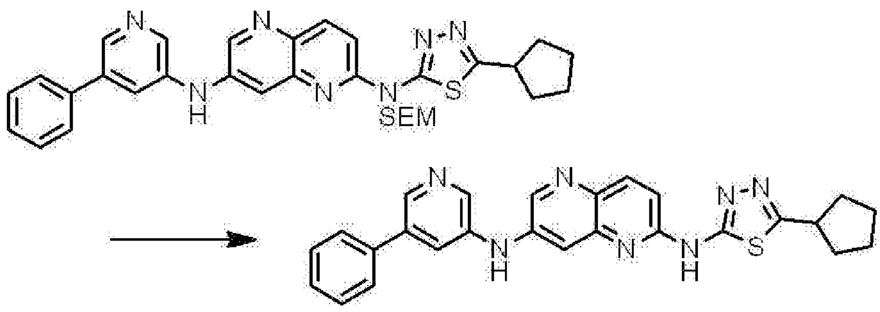

HCL salt

12 mol/L hydrochloric acid (0.6 mL) was added to a solution of $N^2$-(5-cyclopentyl-1,3,4-thiadiazol-2-yl)-$N^7$-(5-phenylpyridin-3-yl)-$N^2$-((2-(trimethylsilyl)ethoxy)methyl)-1,5-naphthyridine-2,7-diamine (8 mg) in methanol (0.6 mL), followed by stirring at room temperature for 15 hours. The solvent was distilled off under reduced pressure, ethyl acetate and methanol were added to the obtained residue, and the solid matter was collected by filtration, thereby obtaining $N^2$-(5-cyclopentyl-1,3,4-thiadiazol-2-yl)-$N^7$-(5-phenylpyridin-3-yl)-1,5-naphthyridine-2,7-diamine hydrochloride (2.7 mg).
$^1$H-NMR(DMSO-$d_6$)δ:9.60(1H,brs),8.72-8.62(3H,m),8.21(1H,d,J=9.0Hz),7.92-7.78(3H,m),7.60-7.46(4H,m),7.29(1H,d,J=9.0Hz),4.31-4.23(1H,m),1.92-1.58(8H,m).
MSm/z(M+H):466.

<Example 0071>

<0071-1>

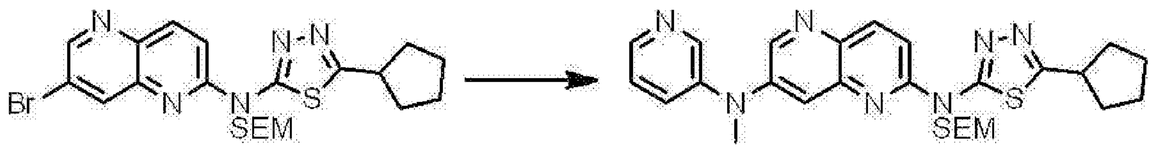

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), N-methylpyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 6 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate-hexane), thereby obtaining $N^2$-(5-cyclopentyl-1,3,4-thiadiazol-2-yl)-$N^7$-methyl-$N^7$-(pyridin-3-yl)-$N^2$-((2-(trimethylsilyl)ethoxy)methyl)-1,5-naphthyridine-2,7-diamine (27 mg).
MSm/z(M+H):534.

<0071-2>

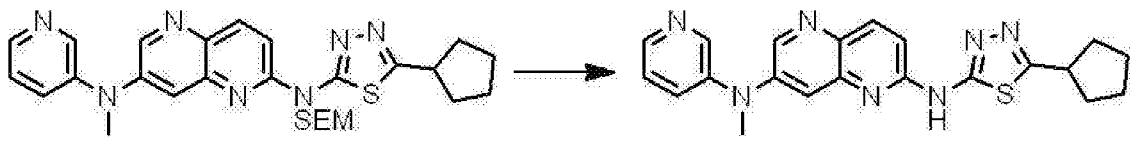

Water (0.1 mL) and trifluoroacetic acid (2 mL) were added to $N^2$-(5-cyclopentyl-1,3,4-thiadiazol-2-yl)-$N^7$-methyl-$N^7$-(pyridin-3-yl)-$N^2$-((2-(trimethylsilyl)ethoxy)methyl)-1,5-naphthyridine-2,7-diamine (27 mg), followed by stirring at room temperature for 3 hours. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-methyl-$N^7$-(pyridin-3-yl)-1,5-naphthyridine-2,7-diamine (10 mg).
$^1$H-NMR($CDCl_3$)$\delta$:8.60-8.42(3H,m),8.26-8.16(2H,m),7.64-7.50(2H,m),7.40-7.32(1H,m),3.56-3.48(1H,m),3.54(3H,s),2.30-2.18(2H,m),2.04-1.66(6H,m).
MSm/z(M+H):404.

<Example 0072>

<0072-1>

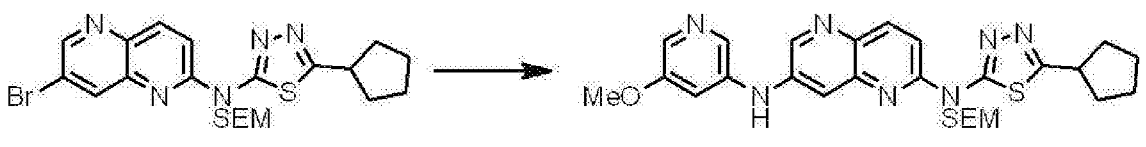

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 5-methoxypyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 6 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate-hexane), thereby obtaining $N^2$-(5-cyclopentyl-1,3,4-thiadiazol-2-yl)-$N^7$-(5-methoxypyridin-3-yl)-$N^2$-((2-(trimethylsilyl)ethoxy)methyl)-1,5-naphthyridine-2,7-diamine (15 mg).
MSm/z(M+H):550.

<0072-2>

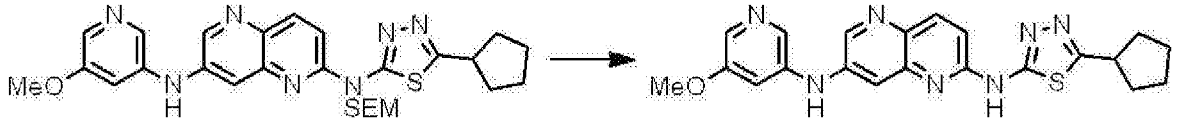

Water (0.1 mL) and trifluoroacetic acid (2 mL) were added to $N^2$-(5-cyclopentyl-1,3,4-thiadiazol-2-yl)-$N^7$-(5-methoxypyridin-3-yl)-$N^2$-((2-(trimethylsilyl)ethoxy)methyl)-1,5-naphthyridine-2,7-diamine (15 mg), followed by stirring at room temperature for 3 hours. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-(5-methoxypyridin-3-yl)-1,5-naphthyridine-2,7-diamine (7.4 mg).
$^1$H-NMR($CDCl_3$/$CD_3OD$=4/1)$\delta$:8.50(1H,brs),8.14(1H,brs),8.11(1H,d,J=9.3Hz),7.93(1H,brs), 7.86(1H,brs),7.40(1H,brs),7.18(1H,d,J=9.3Hz),3.91(3H,s),3.55-3.46(1H,m),2.32-2.18(2H,m),1.94-1.72(6H,m).
MSm/z(M+H):420.

<Example 0073>

<0073-1>

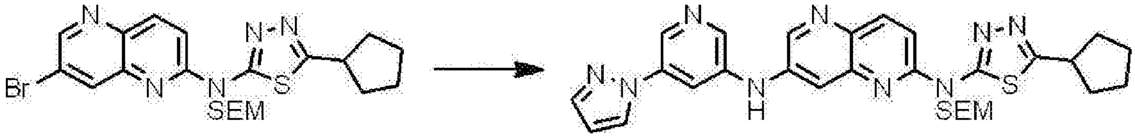

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 5-(1H-pyrazol-1-yl)pyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 3.5 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^7$-(5-(1H-pyrazol-1-yl)pyridin-3-yl)-$N^2$-(5-cyclopentyl-1,3,4-thiadiazol-2-yl)-$N^2$-((2-(trimethylsilyl)ethoxy)methyl)-1,5-naphthyridine-2,7-diamine (42 mg).
MSm/z(M+H):586.

<0073-2>

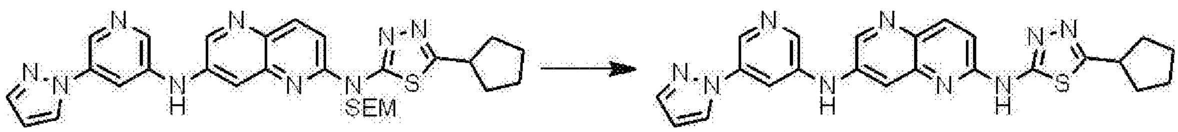

Water (0.1 mL) and trifluoroacetic acid (2 mL) were added to $N^7$-(5-(1H-pyrazol-1-yl)pyridin-3-yl)-$N^2$-(5-cyclopentyl-1,3,4-thiadiazol-2-yl)-$N^2$-((2-(trimethylsilyl)ethoxy)methyl)-1,5-naphthyridine-2,7-diamine (42 mg), followed by stirring at room temperature for 1.5 hours. The solvent was distilled off under reduced pressure, and the obtained residue was purified by silica gel column chromatography (methanol-ethyl acetate, NH silica), thereby obtaining $N^7$-(5-(1H-pyrazol-1-yl)pyridin-3-yl)-$N^2$-(5-cyclopentyl-1,3,4-thiadiazol-2-yl)-1,5-naphthyridine-2,7-diamine (5.3 mg). $^1$H-NMR(DMSO-$d_6$)$\delta$:11.96(1H,brs),9.33(1H,brs),8.73(1H,d,1.8Hz),8.65(1H,s),8.64(1H,s), 8.45(1H,d,J=2.1Hz),8.18(1H,d,J=9.0Hz),8.17(1H,d,J=2.1Hz),7.81(1H,d,J=1.8Hz),7.78(1H,d,J=2.7Hz) ,7.26(1H,9.0Hz) ,6.63-6.60(1H,m),3.50-3.33(1H,m),2.16-2.04(2H,m),1.84-1.62(6H,m).

MSm/z(M+H):456.

<Example 0074>

<0074-1>

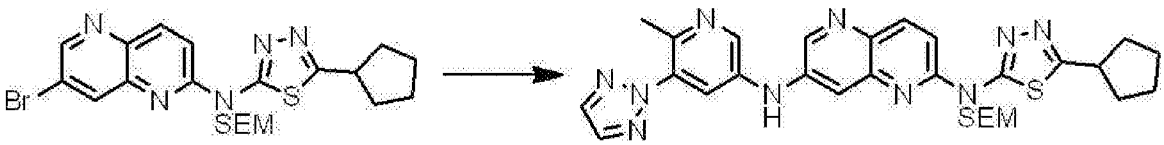

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 6-methyl-5-(2H-1,2,3-triazol-2-yl)pyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 3.5 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(6-methyl-5-(2H-1,2,3-triazol-2-yl)pyridin-3-yl)-$N^2$-((2-(trimethylsilyl)ethoxy)methyl)-1,5-naphthyridine-2,7-diamine (62 mg).
MSm/z(M+H):601.

<0074-2>

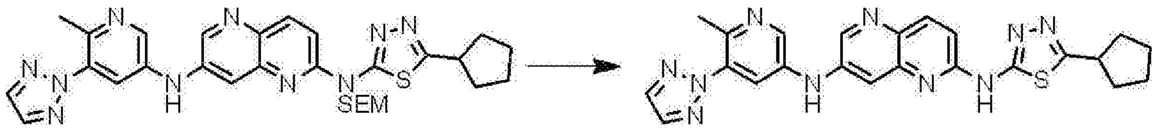

Water (0.1 mL) and trifluoroacetic acid (2 mL) were added to $N^2$-(5-cyclopentyl-1,3,4-thiadiazol-2-yl)-$N^7$-(6-methyl-5-(2H-1,2,3-triazol-2-yl)pyridin-3-yl)-$N^2$-((2-(trimethylsilyl)ethoxy)methyl)-1,5-naphthyridine-2,7-diamine (62 mg), followed by stirring at room temperature for 1.5 hours. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-(6-methyl-5-(2H-1,2,3-triazol-2-yl)pyridin-3-yl)-1,5-naphthyridine-2,7-diamine (12 mg).
$^1$H-NMR(DMSO-$d_6$)$\delta$:11.94(1H,brs),9.26(1H,brs),8.61(1H,d,J=2.7Hz),8.57(1H,d,J=2.7Hz),
8.18(2H,s),8.16(1H,d,J=9.3Hz),7.95(1H,d,J=2.4Hz),7.71(1H,d,J=2.4Hz),7.24(1H,d,J=9.3Hz),3.50-3.33(1H,m),2.33(3H,s),2.16-2.04(2H,m),1.84-1.62(6H,m).
MSm/z(M+H):471.

<Example 0075>

<0075-1>

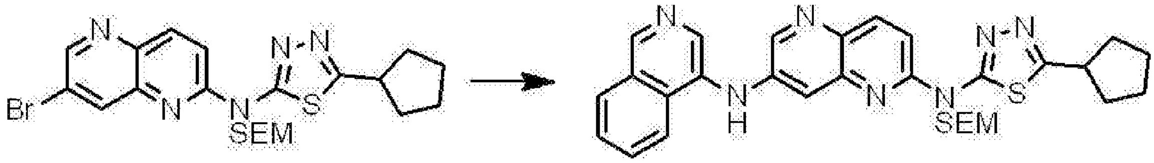

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), isoquinoline-4-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 7 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(isoquinolin-4-yl)-$N^2$-((2-(trimethylsilyl)ethoxy)methyl)-1,5-naphthyridine-2,7-diamine (70 mg).
MSm/z(M+H):570.

<0075-2>

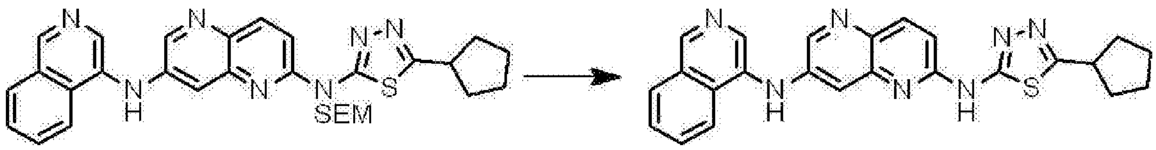

Water (0.1 mL) and trifluoroacetic acid (2 mL) were added to $N^2$-(5-cyclopentyl-1,3,4-thiadiazol-2-yl)-$N^7$-(isoquinolin-4-yl)-$N^2$-((2-(trimethylsilyl)ethoxy)methyl)-1,5-naphthyridine-2,7-diamine (70 mg), followed by stirring at room temperature for 2 hours. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-(isoquinolin-4-yl)-1,5-naphthyridine-2,7-diamine (8.4 mg).
$^1$H-NMR(DMSO-$d_6$)$\delta$:11.88(1H,brs),9.15(1H,s),9.00(1H,brs),8.68(1H,d,1.8Hz),8.61(1H,s), 8.24-8.12(3H,m),7.88-7.74(2H,m),7.38(1H,d,J=2.7Hz),7.20(1H,d,J=9.3Hz),3.50-3.33(1H,m),2.16-2.04(2H,m),1.84-1.62(6H,m).
MSm/z(M+H):440.

<Example 0076>

<0076-1>

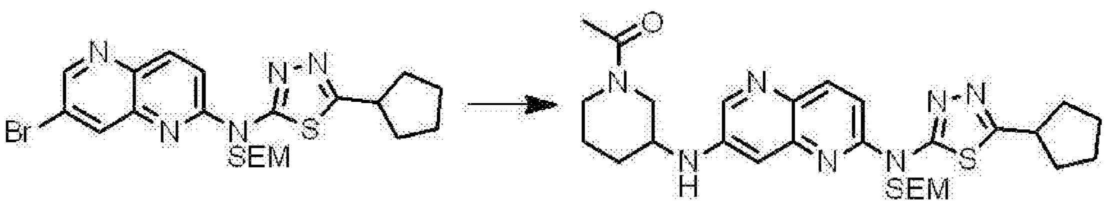

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 1-(3-aminopiperidin-1-yl)ethanone (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 7 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate), thereby obtaining 1-(3-((6-((5-cyclopentyl-1,3,4-thiadiazol-2-yl)((2-(trimethylsilyl)ethoxy)methyl)amino)-1,5-naphthyridin-3-yl)amino)piperidin-1-yl)ethanone (5.5 mg).
MSm/z(M+H):568.

<0076-2>

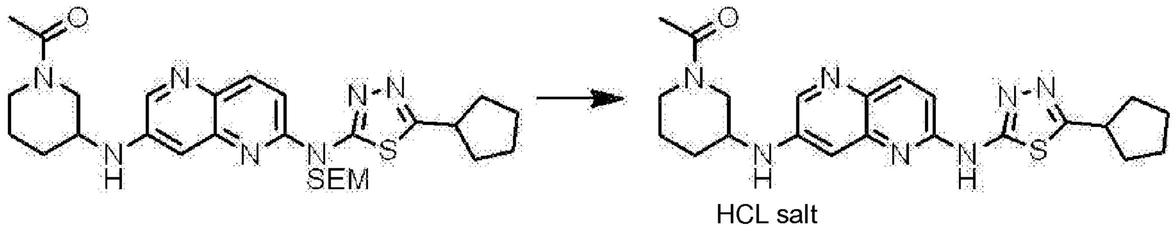


A 4 mol/L hydrogen chloride/1,4-dioxane solution (0.4 mL) was added to a solution of 1-(3-((6-((5-cyclopentyl-1,3,4-thiadiazol-2-yl)((2-(trimethylsilyl)ethoxy)methyl)amino-1,5-naphthyridin-3-yl)amino)piperidin-1-yl)ethanone (5.5 mg) in 1,4-dioxane (1.2 mL), followed by stirring at room temperature for 16 hours. The solvent was distilled off under reduced pressure, diisopropyl ether and methanol were added to the obtained residue, and the solid matter was collected by filtration, thereby obtaining 1-(3-((6-((5-cyclopentyl-1,3,4-thiadiazol-2-yl)amino)-1,5-naphthyridin-3-yl)amino)piperidin-1-yl)ethanone hydrochloride (1.7 mg).
$^1$H-NMR(DMSO-$d_6$)$\delta$:8.38(1H,dd,J=12Hz,2.7Hz),8.07(1H,d,J=8.7Hz), 7.25(1H,ddd,J=12Hz,2.7Hz,2.1Hz),7.11(1H,dd,J=8.7Hz,2.1Hz),3.96-3.44(6H,m),2.16-2.04(2H,m),2.08(3H,s),1.84-1.62(10H,m).
MSm/z(M+H):438.

<Example 0077>

<0077-1>

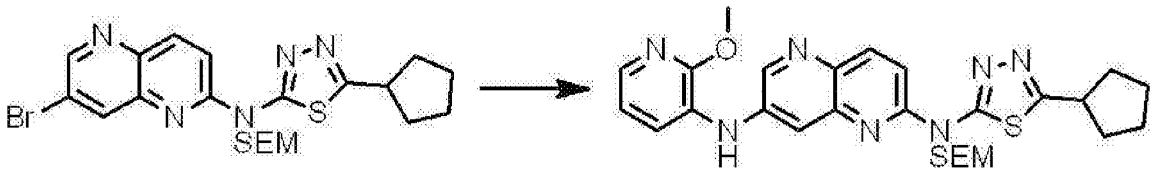

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 2-methoxypyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 7 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining N$^2$-(5-cyclopentyl-1,3,4-thiadiazol-2-yl)-N$^7$-(2-methoxypyridin-3-yl)-N$^2$-((2-(trimethylsilyl)ethoxy)methyl)-1,5-naphthyridine-2,7-diamine (62 mg).
MSm/z(M+H):550.

<0077-2>

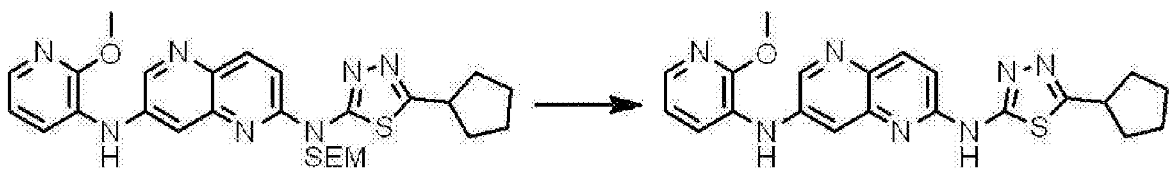

Water (0.1 mL) and trifluoroacetic acid (2 mL) were added to N$^2$-(5-cyclopentyl-1,3,4-thiadiazol-2-yl)-N$^7$-(2-methoxypyridin-3-yl)-N$^2$-((2-(trimethylsilyl)ethoxy)methyl)-1,5-naphthyridine-2,7-diamine (62 mg), followed by stirring at room temperature for 3 hours. The solvent was distilled off under reduced pressure, and the obtained residue was purified by silica gel column chromatography (methanol-ethyl acetate), thereby obtaining N$^2$-(5-cyclopentyl-1,3,4-thia-

diazol-2-yl)-$N^7$-(2-methoxypyridin-3-yl)-1,5-naphthyridine-2,7-diamine (15 mg).
$^1$H-NMR(DMSO-$d_6$)$\delta$:11.88(1H,brs),8.62(1H,d,J=2.4Hz),8.43(1H,brs),8.12(1H,d,J=8.7Hz), 7.90-7.87(1H,m),7.76-7.72(1H,m),7.45(1H,d,J=2.7Hz),7.20(1H,d,J=9.3Hz),7.06-7.00(1H,m),3.95(3H,s),3.50-3.33(1H, m),2.16-2.04(2H,m),1.84-1.62(6H,m).
MSm/z(M+H):420.

<Example 0078>

<0078-1>

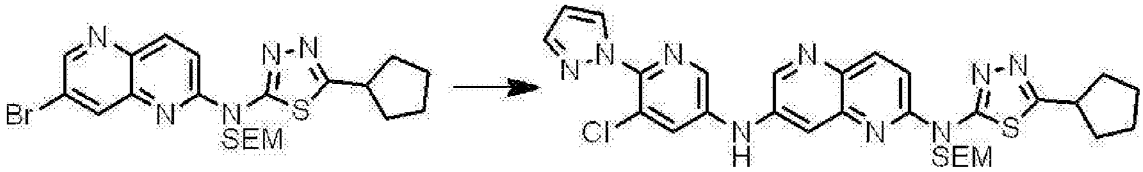

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 5-chloro-6-(1H-pyrazol-1-yl)pyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 7 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^7$-(5-chloro-6-(1H-pyrazol-1-yl)pyridin-3-yl)-$N^2$-(5-cyclopentyl-1,3,4-thiadiazol-2-yl)-$N^2$-((2-(trimethylsilyl)ethoxy)methyl)-1,5-naphthyridine-2,7-diamine (54 mg).
MSm/z(M+H):620.

<0078-2>

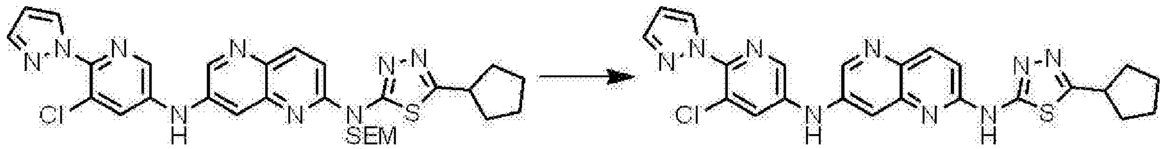

Water (0.1 mL) and trifluoroacetic acid (2 mL) were added to $N^7$-(5-chloro-6-(1H-pyrazol-1-yl)pyridin-3-yl)-$N^2$-(5-cyclopentyl-1,3,4-thiadiazol-2-yl)-$N^2$-((2-(trimethylsilyl)ethoxy)methyl)-1,5-naphthyridine-2,7-diamine (54 mg), followed by stirring at room temperature for 2 hours. The solvent was distilled off under reduced pressure, and the obtained residue was purified by silica gel column chromatography (methanol-ethyl acetate, NH silica), thereby obtaining $N^7$-(5-chloro-6-(1H-pyrazol-1-yl)pyridin-3-yl)-$N^2$-(5-cyclopentyl-1,3,4-thiadiazol-2-yl)-1,5-naphthyridine-2,7-diamine (0.55 mg).
$^1$H-NMR(($CD_3$)$_2$CO)$\delta$:8.72(1H,d,J=2.1Hz),8.64(1H,brs),8.47(1H,d,2.4Hz),8.22(1H,d,J=9.3Hz), 8.12(1H,d,J=2.4Hz),8.03-7.95(1H,m),7.74(1H,brs), 7.44(1H,d,J=9.3Hz),6.53-6.50(1H,m),3.50-3.33(1H,m),2.16-2.04(2H,m),1.84-1.62(6H,m).
MSm/z(M+H):490.

<Example 0079>

<0079-1>

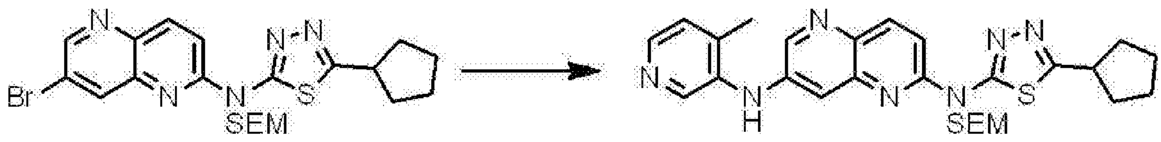

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 4-methylpyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 10 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(4-methylpyridin-3-yl)-$N^2$-((2-(trimethylsilyl)ethoxy)methyl)-1,5-naphthyridine-2,7-diamine (43 mg).
MSm/z(M+H):534.

<0079-2>

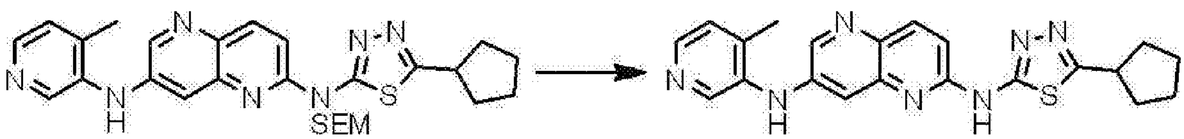

Water (0.1 mL) and trifluoroacetic acid (2 mL) were added to $N^2$-(5-cyclopentyl-1,3,4-thiadiazol-2-yl)-$N^7$-(4-methylpyridin-3-yl)-$N^2$-((2-(trimethylsilyl)ethoxy)methyl)-1,5-naphthyridine-2,7-diamine (43 mg), followed by stirring at room temperature for 2 hours. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-(4-methylpyridin-3-yl)-1,5-naphthyridine-2,7-diamine (0.95 mg). $^1$H-NMR($CDCl_3$)$\delta$:8.56(1H,brs),8.47(1H,d,J=2.7Hz),8.31-8.27(1H,m),8.09(1H,d,J=9.3Hz),7.38-7.32(2H,m),7.10(1H,d,9.3Hz),3.71-3.62(1H,m),2.35(3H,s),1.92-1.68(8H,m).
MSm/z(M+H):404.

<Example 0080>

<0080-1>

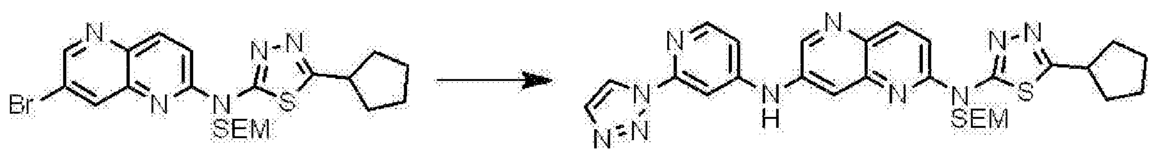

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 2-(1H-1,2,3-triazol-1-yl)pyridine-4-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 10 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^7$-(2-(1H-1,2,3-triazol-1-yl)pyridin-4-yl)-$N^2$-(5-cyclopentyl-1,3,4-thiadiazol-2-yl)-$N^2$-((2-(trimethylsilyl)ethoxy)methyl)-1,5-naphthyridine-2,7-diamine (41 mg).
MSm/z(M+H):587.

<0080-2>

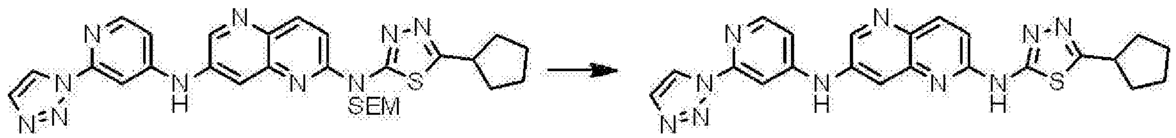

Water (0.1 mL) and trifluoroacetic acid (2 mL) were added to $N^7$-(2-(1H-1,2,3-triazol-1-yl)pyridin-4-yl)-$N^2$-(5-cyclopentyl-1,3,4-thiadiazol-2-yl)-$N^2$-((2-(trimethylsilyl)ethoxy)methyl)-1,5-naphthyridine-2,7-diamine (41 mg), followed by stirring at room temperature for 2 hours. The solvent was distilled off under reduced pressure, and the obtained residue was purified by silica gel column chromatography (methanol-ethyl acetate, NH silica), thereby obtaining $N^7$-(2-(1H-1,2,3-triazol-1-yl)pyridin-4-yl)-$N^2$-(5-cyclopentyl-1,3,4-thiadiazol-2-yl)-1,5-naphthyridine-2,7-diamine (7.3 mg).
$^1$H-NMR($CDCl_3$)$\delta$:8.65-8.63(2H,m),8.29(1H,d,J=6.0Hz),8.19(1H,d,J=9.3Hz),8.11(1H,d,J=2.1Hz),7.88-7.85(2H,m),7.27(1H,d,J=9.3Hz),7.22-7.18(1H,m),3.53-3.38(1H,m),1.94-1.68(8H,m).
MSm/z(M+H):457.

<Example 0081>

<0081-1>

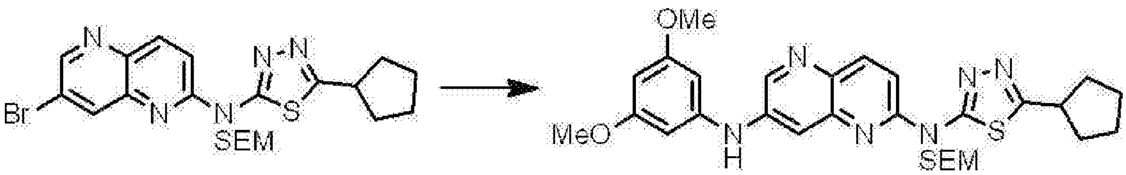

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 3,5-dimethoxyaniline (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 8 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(3,5-dimethoxyphenyl)-$N^2$-((2-(trimethylsilyl)ethoxy)methyl)-1,5-naphthyridine-2,7-diamine (68 mg).
MSm/z(M+H):579.

<0081-2>

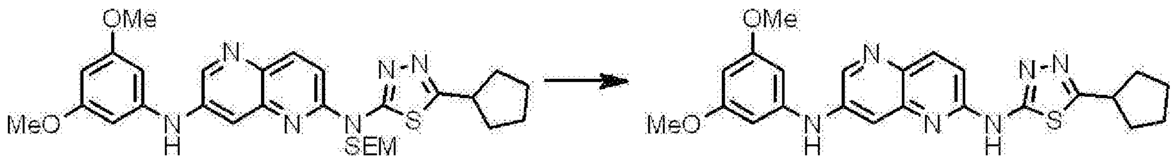

Water (0.1 mL) and trifluoroacetic acid (2 mL) were added to $N^2$-(5-cyclopentyl-1,3,4-thiadiazol-2-yl)-$N^7$-(3,5-dimethoxyphenyl)-$N^2$-((2-(trimethylsilyl)ethoxy)methyl)-1,5-naphthyridine-2,7-diamine (68 mg), followed by stirring at room temperature for 1.5 hours. The solvent was distilled off under reduced pressure, and the obtained residue was

purified by silica gel column chromatography (methanol-ethyl acetate, NH silica), thereby obtaining $N^2$-(5-cyclopentyl-1,3,4-thiadiazol-2-yl)-$N^7$-(3,5-dimethoxyphenyl)-1,5-naphthyridine-2,7-diamine (4.1 mg). $^1H$-NMR($CDCl_3$/$CD_3OD$=4/1)δ:8.44(1H,d,J=2.7Hz),8.07(1H,d,J=8.7Hz),7.88(1H,d,J=2.7Hz), 7.09(1H,d,J=8.7Hz),6.53(3H,s),3.82(3H,s),3.75(3H,s),3.52-3.42(1H,m),2.28-2.16(2H,m),1.92-1.72(6H,m). MSm/z(M+H):449.

<Example 0082>

<0082-1>

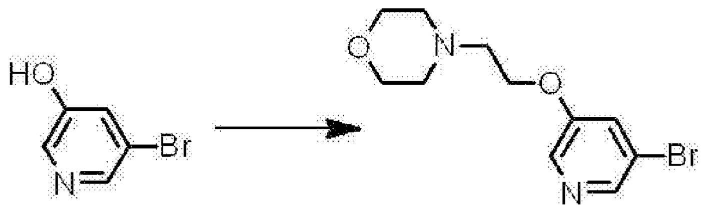

A mixture of 5-bromopyridin-3-ol (500 mg), 4-(2-bromoethyl)morpholine hydrochloride (641 mg) and potassium carbonate (1.18 g) in acetonitrile (6 mL) was stirred for 5 hours under heating to reflux. The reaction mixture was cooled to room temperature, and ethyl acetate and water were added thereto. The organic layer was collected by separation, and the aqueous layer was extracted with chloroform. The organic layer and the extraction liquid were combined, the resultant product was dried over anhydrous sodium sulfate, and the solvent was distilled off under reduced pressure, thereby obtaining 4-(2-((5-bromopyridin-3-yl)oxy)ethyl)morpholine (0.75 g).
MSm/z(M+H):287,289.

<0082-2>

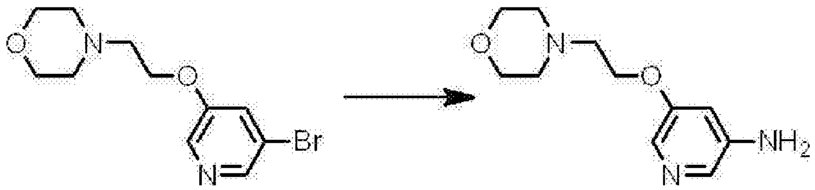

A 25% ammonia aqueous solution (2 mL) was added to a mixture of 4-(2-((5-bromopyridin-3-yl)oxy)ethyl)morpholine (0.75 g) and copper(I) oxide (205 mg) in N-methylpyrrolidone (2 mL), followed by stirring at 120°C for 30 minutes using a microwave reaction apparatus. The insolubles were filtered off using celite, and chloroform was added thereto. The organic layer was collected by separation, and dried over anhydrous sodium sulfate, and the obtained solution was purified by silica gel column chromatography (methanol-ethyl acetate), thereby obtaining 5-(2-morpholinoethoxy)pyridine-3-amine (226 mg).
MSm/z(M+H):224.

<0082-3>

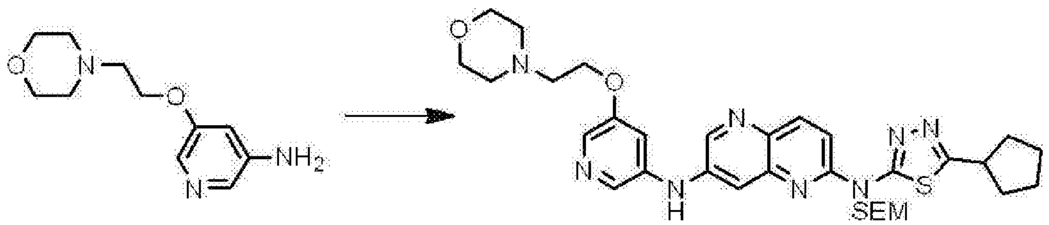

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 5-(2-morpholinoethoxy)pyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 7 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(5-(2-morpholinoethoxy)pyridin-3-yl)-$N^2$-((2-(trimethylsilyl)ethoxy)methyl)-1,5-naphthyridine-2,7-diamine (69 mg).
MSm/z(M+H):649.

<0082-4>

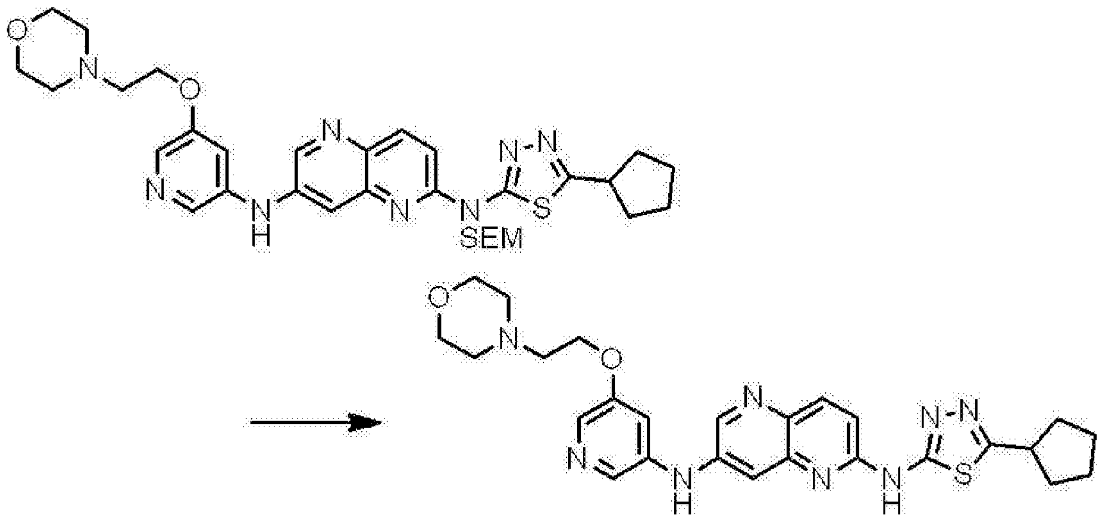

Water (0.1 mL) and trifluoroacetic acid (2 mL) were added to $N^2$-(5-cyclopentyl-1,3,4-thiadiazol-2-yl)-$N^7$-(5-(2-morpholinoethoxy)pyridin-3-yl)-$N^2$-((2-(trimethylsilyl)ethoxy)methyl)-1,5-naphthyridine-2,7-diamine (69 mg), followed by stirring at room temperature for 1 day. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-(5-(2-morpholinoethoxy)pyridin-3-yl)-1,5-naphthyridine-2,7-diamine (13 mg). $^1$H-NMR(DMSO-$d_6$)$\delta$:11.93(1H,brs),9.07(1H,brs),8.59(1H,d,J=2.7Hz),8.15(1H,d,J=9.3Hz), 8.13(1H,s),7.96(1H,d,J=2.7Hz),7.68(1H,d,J=2.7Hz),7.29(1H,brs),7.22(1H,d,J=9.3Hz), 4.19(2H,t,J=5.1Hz),3.56-3.28(7H,m),2.75-2.68(4H,m),2.18-2.06(2H,m), 1.88-1.62(6H,m).
MSm/z(M+H):519.

<Example 0083>

<0083-1>

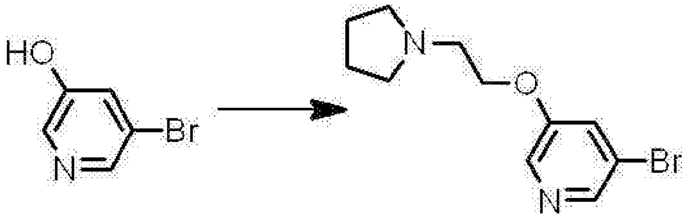

A mixture of 5-bromopyridin-3-ol (500 mg), 1-(2-bromoethyl)pyrrolidine hydrochloride (585 mg) and potassium carbonate (1.18 g) in acetonitrile (6 mL) was stirred for 5 hours under heating to reflux. The reaction mixture was cooled to room temperature, and ethyl acetate and water were added thereto. The organic layer was collected by separation, and the aqueous layer was extracted with chloroform. The organic layer and the extraction liquid were combined, the resultant product was dried over anhydrous sodium sulfate, and the solvent was distilled off under reduced pressure, thereby obtaining 3-bromo-5-(2-(pyrrolidin-1-yl)ethoxy)pyridine (0.54 g).
MSm/z(M+H):271,273.

<0083-2>

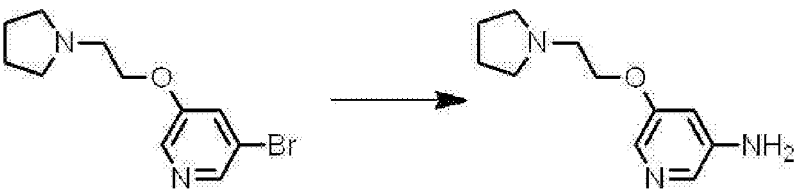

A 25% ammonia aqueous solution (2 mL) was added to a mixture of 3-bromo-5-(2-(pyrrolidin-1-yl)ethoxy)pyridine (0.54 g), copper(I) oxide (205 mg), and N-methylpyrrolidone (2 mL), followed by stirring at 120°C for 30 minutes using a microwave reaction apparatus. The insolubles were filtered off using celite, and dichloromethane was added thereto. The organic layer was collected by separation, and dried over anhydrous sodium sulfate, and the obtained solution was purified by silica gel column chromatography (methanol-ethyl acetate, NH silica), thereby obtaining 5-(2-(pyrrolidin-1-yl)ethoxy)pyridine-3-amine (104 mg).
MSm/z(M+H):208.

<0083-3>

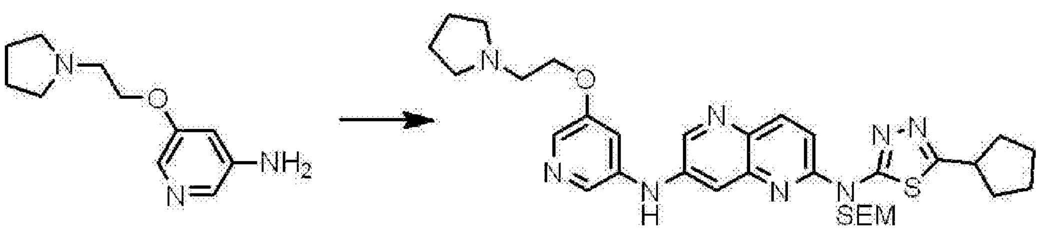

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 5-(2-(pyrrolidin-1-yl)ethoxy)pyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 7 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(5-(2-(pyrrolidin-1-yl)ethoxy)pyridin-3-yl)-$N^2$-((2-(trimethylsilyl)ethoxy)methyl)-1,5-naphthyridine-2,7-diamine (85 mg).
MSm/z(M+H):633.

<0083-4>

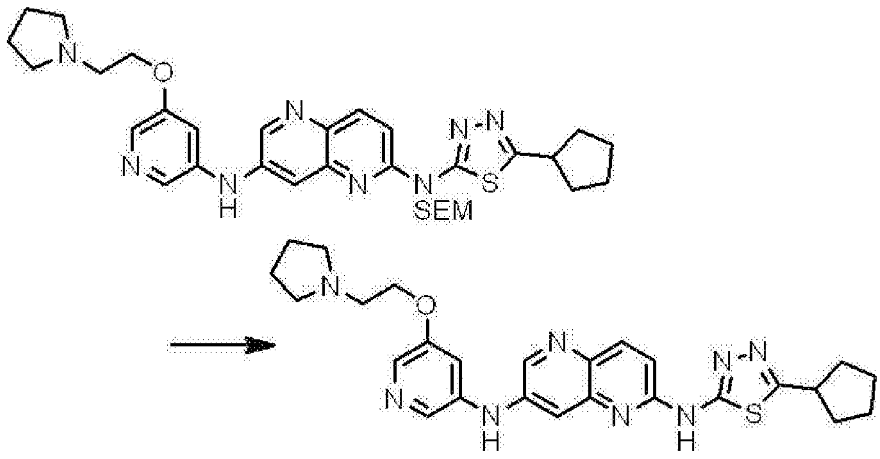

Water (0.1 mL) and trifluoroacetic acid (2 mL) were added to $N^2$-(5-cyclopentyl-1,3,4-thiadiazol-2-yl)-$N^7$-(5-(2-(pyrrolidin-1-yl)ethoxy)pyridin-3-yl)-$N^2$-((2-(trimethylsilyl)ethoxy)methyl)-1,5-naphthyridine-2,7-diamine (85 mg), followed by stirring at room temperature for 1.5 hours. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-(5-(2-(pyrrolidin-1-yl)ethoxy)pyridin-3-yl)-1,5-naphthyridine-2,7-diamine (6.5 mg).
$^1$H-NMR(DMSO-$d_6$)$\delta$:11.94(1H,brs),9.08(1H,brs),8.59(1H,d,J=2.7Hz),8.15(1H,d,J=9.3Hz), 8.13(1H,s),7.96(1H,d,J=2.7Hz),7.68(1H,brs),7.29(1H,brs),7.23(1H,d,J=9.3Hz),4.20-4.14(2H,m),2.85-2.48(5H,m),2.29-2.25(2H,m),2.18-2.06(2H,m),1.88-1.62(10H,m).
MSm/z(M+H):503.

<Example 0084>

<0084-1>

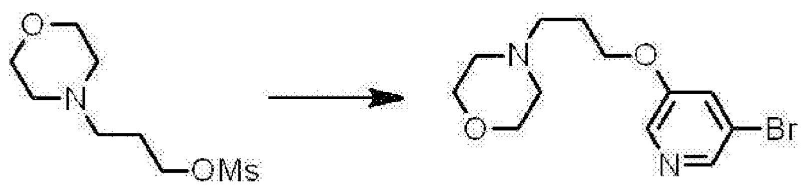

A mixture of 5-bromopyridin-3-ol (500 mg), 3-morpholinopropyl methanesulfonate (832 mg) and cesium carbonate (2.80 g) in acetonitrile (8 mL) and tetrahydrofuran (4 mL) was stirred for 1.5 hours under heating to reflux. The reaction mixture was cooled to room temperature, and ethyl acetate and water were added thereto. The organic layer was collected by separation, and dried over anhydrous sodium sulfate, and the solvent was distilled off under reduced pressure, thereby obtaining 4-(3-((5-bromopyridin-3-yl)oxy)propyl)morpholine (788 mg).
MSm/z(M+H):301,303.

<0084-2>

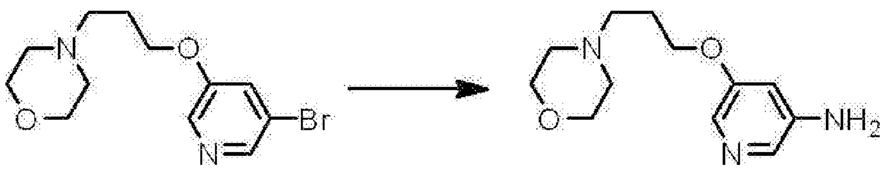

A 25% ammonia aqueous solution (2 mL) was added to a mixture of 4-(3-((5-bromopyridin-3-yl)oxy)propyl)morpholine (788 mg) and copper(I) oxide (186 mg) in N-methylpyrrolidone (2 mL), followed by stirring at 120°C for 60 minutes using a microwave reaction apparatus. Dichloromethane and water were added to the reaction mixture. The organic layer was collected by separation, and dried over anhydrous sodium sulfate, and the obtained solution was purified by silica gel column chromatography (methanol-ethyl acetate), thereby obtaining 5-(3-morpholinopropoxy)pyridine-3-amine (297 mg).
MSm/z(M+H):238.

<0084-3>

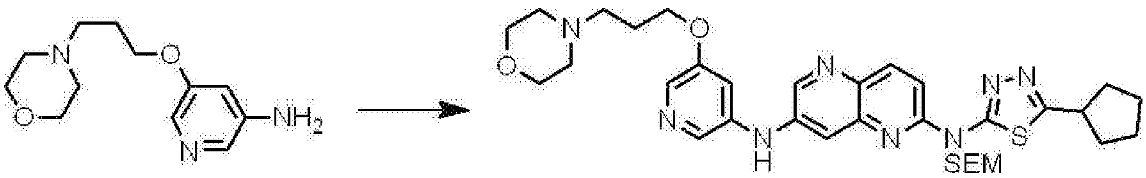

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 5-(3-morpholinopropoxy)pyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 6 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(5-(3-morpholinopropoxy)pyridin-3-yl)-$N^2$-((2-(trimethylsilyl)ethoxy)methyl)-1,5-naphthyridine-2,7-diamine (62 mg).
MSm/z(M+H):663.

<0084-4>

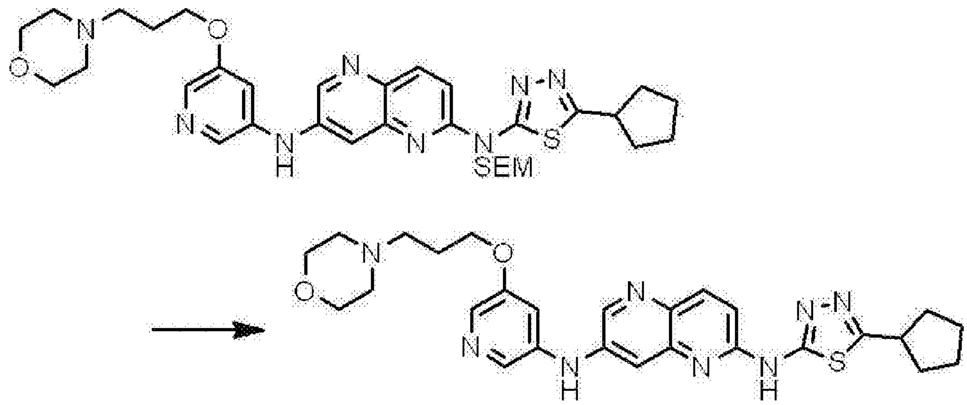

Water (0.1 mL) and trifluoroacetic acid (2 mL) were added to $N^2$-(5-cyclopentyl-1,3,4-thiadiazol-2-yl)-$N^7$-(5-(3-morpholinopropoxy)pyridin-3-yl)-$N^2$-((2-(trimethylsilyl)ethoxy)methyl)-1,5-naphthyridine-2,7-diamine (62 mg), followed by stirring at room temperature for 6 hours. The solvent was distilled off under reduced pressure, and the obtained residue was purified by silica gel column chromatography (methanol-ethyl acetate, NH silica), thereby obtaining $N^2$-(5-

cyclopentyl-1,3,4-thiadiazol-2-yl)-$N^7$-(5-(3-morpholinopropoxy)pyridin-3-yl)-1,5-naphthyridine-2,7-diamine (8.3 mg).
$^1H$-NMR($CDCl_3$/$CD_3OD$=4/1)δ:8.51(1H,brs),8.18-8.08(2H,m),7.93(1H,brs),7.84(1H,brs),7.23-7.16(2H,m),4.15-4.08(2H,m),3.98-3.91(7H,m),3.77-3.71(4H,m),2.32-1.72(10H,m).
MSm/z(M+H):533.

<Example 0085>

<0085-1>

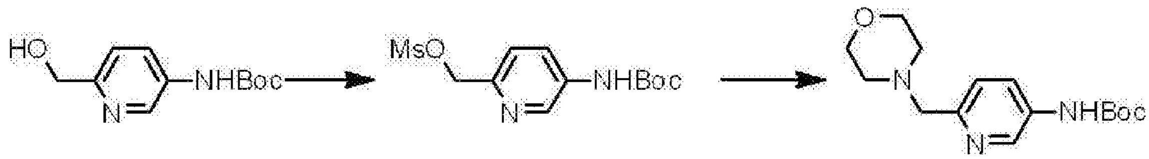

Methanesulfonyl chloride (0.104 mL) was added to a solution of tert-butyl (6-(hydroxymethyl)pyridin-3-yl)carbamate (202 mg) and triethylamine (0.189 mL) in dichloromethane (9 mL) under ice-cooling, followed by stirring at the same temperature for 30 minutes. The reaction mixture was divided into two.

Morpholine (0.117 mL) was added to half the amount of the reaction mixture, followed by stirring at room temperature for 9 hours. Water and ethyl acetate were added to the reaction mixture. The organic layer was collected by separation, washed with a saturated sodium chloride aqueous solution, and dried over anhydrous sodium sulfate, and the solvent was distilled off under reduced pressure, thereby obtaining tert-butyl (6-(morpholinomethyl)pyridin-3-yl)carbamate (121 mg).
MSm/z(M+H):294.

<0085-2>

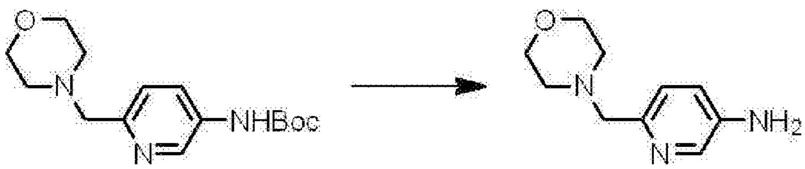

Water (0.1 mL) and trifluoroacetic acid (1 mL) were added to a solution of tert-butyl (6-(morpholinomethyl)pyridin-3-yl)carbamate (42 mg) in dichloromethane (1 mL), followed by stirring at room temperature for 3 hours. The solvent was distilled off under reduced pressure, and the obtained residue was purified by silica gel column chromatography (methanol-ethyl acetate, NH silica), thereby obtaining 6-(morpholinomethyl)pyridine-3-amine (20 mg).
MSm/z(M+H):194.

<0085-3>

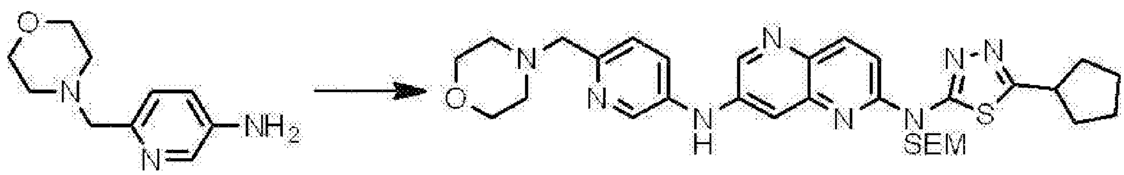

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 6-(morpholinomethyl)pyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 10 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(6-(morpholinomethyl)pyridin-3-yl)-$N^2$-((2-(trimethylsilyl)ethoxy)methyl)-1,5-naphthyridine-2,7-diamine (51 mg).
MSm/z(M+H):619.

<0085-4>

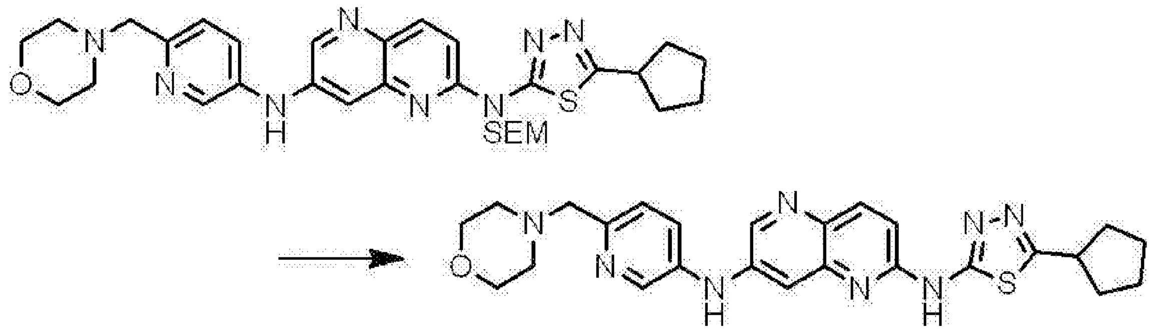

Water (0.1 mL) and trifluoroacetic acid (2 mL) were added to $N^2$-(5-cyclopentyl-1,3,4-thiadiazol-2-yl)-$N^7$-(6-(morpholinomethyl)pyridin-3-yl)-$N^2$-((2-(trimethylsilyl)ethoxy)methyl)-1,5-naphthyridine-2,7-diamine (51 mg), followed by stirring at room temperature for 1 hour. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-(6-(morpholinomethyl)pyridin-3-yl)-1,5-naphthyridine-2,7-diamine (4.8 mg). $^1$H-NMR($CDCl_3$/$CD_3OD$=4/1)δ:8.48(2H,brs),8.13-8.07(1H,m),7.82-7.78(1H,m),7.75-7.67(1H,m),7.50-7.44(1H,m),7.16-7.10(1H,m),3.81-3.75(5H,m),3.68-3.65(2H,m),2.62-2.55(4H,m),2.31-2.18(2H,m),1.96-1.72(6H,m).
MSm/z(M+H):489.

<Example 0086>

<0086-1>

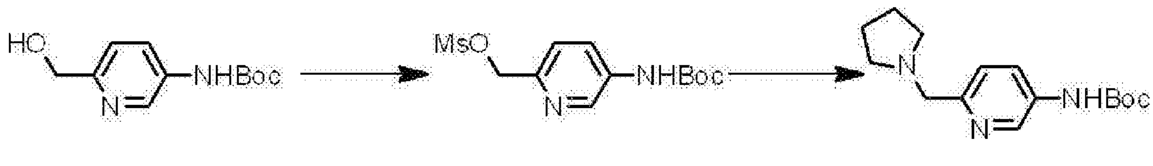

Methanesulfonyl chloride (0.104 mL) was added to a solution of tert-butyl (6-(hydroxymethyl)pyridin-3-yl)carbamate (202 mg) and triethylamine (0.189 mL) in dichloromethane (9 mL) under ice-cooling, followed by stirring at the same temperature for 30 minutes. The reaction mixture was divided into two.

Pyrrolidine (0.111 mL) was added to half the amount of the reaction mixture, followed by stirring at room temperature for 9 hours. Water and ethyl acetate were added to the reaction mixture. The organic layer was collected by separation, washed with a saturated sodium chloride aqueous solution, and dried over anhydrous sodium sulfate, and the solvent was distilled off under reduced pressure, thereby obtaining tert-butyl (6-(pyrrolidin-1-ylmethyl)pyridin-3-yl)carbamate (94 mg).
MSm/z(M+H):278.

<0086-2>

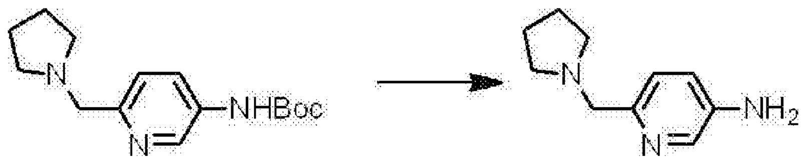

Water (0.1 mL) and trifluoroacetic acid (1 mL) were added to a solution of tert-butyl (6-(pyrrolidin-1-ylmethyl)pyridin-3-yl)carbamate (49 mg) in dichloromethane (1 mL), followed by stirring at room temperature for 1 hour. The solvent was distilled off under reduced pressure, and the obtained residue was purified by silica gel column chromatography (methanol-ethyl acetate, NH silica), thereby obtaining 6-(pyrrolidin-1-ylmethyl)pyridine-3-amine (15 mg).
MSm/z(M+H):178.

<0086-3>

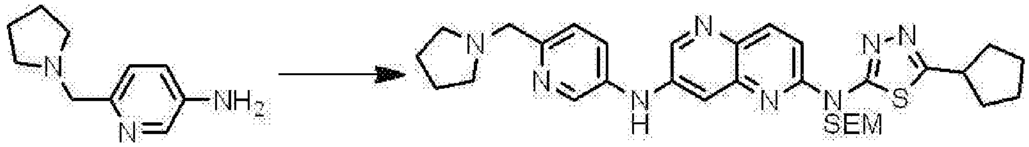

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 6-(pyrrolidin-1-ylmethyl)pyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 10 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(6-(pyrrolidin-1-ylmethyl)pyridin-3-yl)-$N^2$-((2-(trimethylsilyl)ethoxy)methyl)-1,5-naphthyridine-2,7-diamine (43 mg).
MSm/z(M+H):603.

<0086-4>

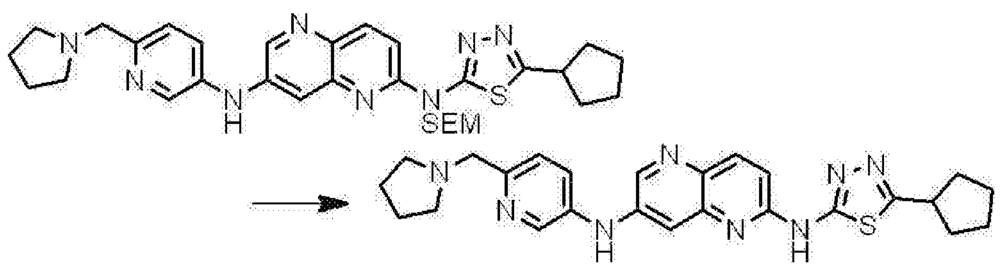

Water (0.1 mL) and trifluoroacetic acid (2 mL) were added to $N^2$-(5-cyclopentyl-1,3,4-thiadiazol-2-yl)-$N^7$-(6-(pyrrolidin-1-ylmethyl)pyridin-3-yl)-$N^2$-((2-(trimethylsilyl)ethoxy)methyl)-1,5-naphthyridine-2,7-diamine (43 mg), followed by stirring at room temperature for 1 hour. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-(6-(pyrrolidin-1-ylmethyl)pyridin-3-yl)-1,5-naphthyridine-2,7-diamine (7.3 mg). $^1$H-NMR($CDCl_3$/$CD_3OD$=4/1)$\delta$:8.48(1H,d,J=2.7Hz),8.46(1H,d,J=2.7Hz),8.10(1H,d,J=8.4Hz), 7.79(1H,d,J=2.7Hz),7.72(1H,dd,J=8.7Hz,2.7Hz),7.47(1H,d,J=8.4Hz),7.13(1H,d,J=8.7Hz),

3.80(2H,brs),3.53-3.40(1H,m),2.69-2.61(4H,m),2.28-2.17(2H,m),1.94-1.68(10H,m).
MSm/z(M+H):473.

<Example 0087>

<0087-1>

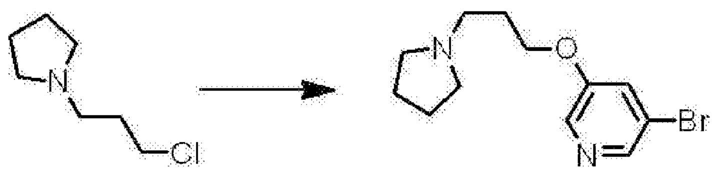

A mixture of 5-bromopyridin-3-ol (500 mg), 1-(3-chloropropyl)pyrrolidine (1.0 g) and potassium carbonate (1.18 g) in acetonitrile (4 mL) and tetrahydrofuran (4 mL) was stirred for 5 hours under heating to reflux. The reaction mixture was cooled to room temperature, and ethyl acetate and water were added thereto. The organic layer was collected by separation, and dried over anhydrous sodium sulfate, and the solvent was distilled off under reduced pressure, thereby obtaining 3-bromo-5-(3-(pyrrolidin-1-yl)propoxy)pyridine (740 mg).
MSm/z(M+H):285,287.

<0087-2>

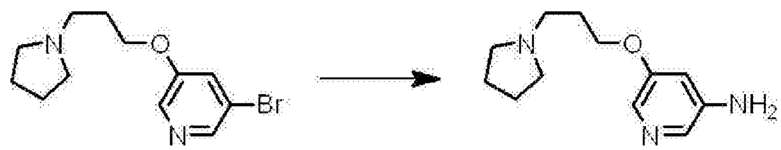

A25% ammonia aqueous solution (2 mL) was added to a mixture of 3-bromo-5-(3-(pyrrolidin-1-yl)propoxy)pyridine (740 mg) and copper(I) oxide (185 mg) in N-methylpyrrolidone (2 mL), followed by stirring at 120°C for 60 minutes using a microwave reaction apparatus. The reaction mixture was cooled to room temperature, and dichloromethane and water were added thereto. The organic layer was collected by separation, and dried over anhydrous sodium sulfate. The obtained solution was purified by silica gel column chromatography (methanol-ethyl acetate), thereby obtaining 5-(3-(pyrrolidin-1-yl)propoxy)pyridine-3-amine (139 mg).
MSm/z(M+H):222.

<0087-3>

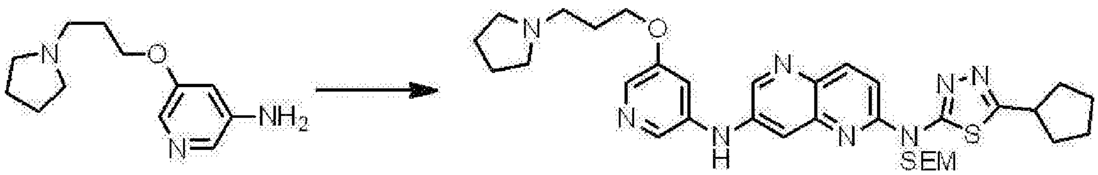

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 5-(3-(pyrrolidin-1-yl)propoxy)pyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in and 1,4-dioxane (1 mL) was stirred at 100°C for 10 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced

pressure, thereby obtaining $N^2$-(5-cyclopentyl-1,3,4-thiadiazol-2-yl)-$N^7$-(5-(3-(pyrrolidin-1-yl)propoxy)pyridin-3-yl)-$N^2$-((2-(trimethylsilyl)ethoxy)methyl)-1,5-naphthyridine-2,7-diamine (47 mg).
MSm/z(M+H):647.

<0087-4>

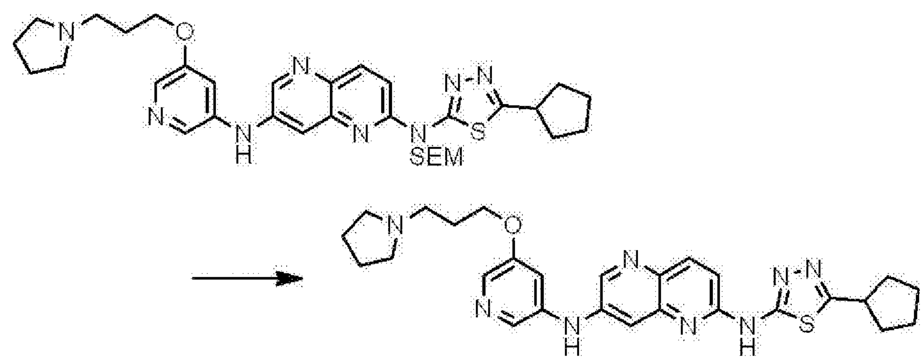

Water (0.1 mL) and trifluoroacetic acid (2 mL) were added to $N^2$-(5-cyclopentyl-1,3,4-thiadiazol-2-yl)-$N^7$-(5-(3-(pyrrolidin-1-yl)propoxy)pyridin-3-yl)-$N^2$-((2-(trimethylsilyl)ethoxy)methyl)-1,5-naphthyridine-2,7-diamine (47 mg), followed by stirring at room temperature for 1 hour. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-(5-(3-(pyrrolidin-1-yl)propoxy)pyridin-3-yl)-1,5-naphthyridine-2,7-diamine (2.8 mg).
$^1$H-NMR($CDCl_3$/$CD_3OD$=4/1)$\delta$:8.50(1H,brs),8.18(1H,brs),8.11(1H,d,J=9.3Hz),7.92(1H,brs),
7.83(1H,brs),7.16(1H,brs),7.15(1H,d,J=9.3Hz),4.10(1H,t,J=6.0Hz),2.84-2.61(7H,m),2.26-2.04(4H,m),1.94-1.70(12H,m).
MSm/z(M+H):517.

<Example 0088>

<0088-1>

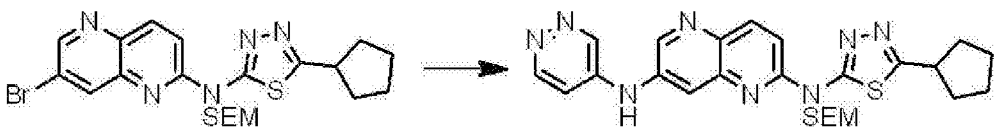

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), pyridazine-4-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 5.5 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(pyridazin-4-yl)-$N^2$-((2-(trimethylsilyl)ethoxy)methyl)-1,5-naphthyridine-2,7-diamine (28 mg).
MSm/z(M+H):521.

<0088-2>

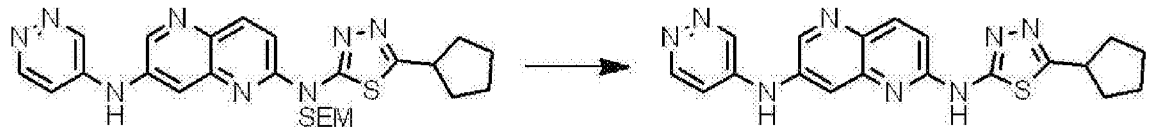

Water (0.1 mL) and trifluoroacetic acid (2 mL) were added to $N^2$-(5-cyclopentyl-1,3,4-thiadiazol-2-yl)-$N^7$-(pyridazin-4-yl)-$N^2$-((2-(trimethylsilyl)ethoxy)methyl)-1,5-naphthyridine-2,7-diamine (28 mg), followed by stirring at room temperature for 1 hour. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-(pyridazin-4-yl)-1,5-naphthyridine-2,7-diamine (1.2 mg).
$^1$H-NMR($CDCl_3$/$CD_3OD$=4/1)δ:8.97(1H,d,J=2.7Hz),8.79(1H,6.6Hz),8.60(1H,2.7Hz), 8.19(1H,d,J=9.3Hz),8.06(1H,d,J=1.8Hz),7.34-7.26(2H,m),3.54-3.44(1H,m),2.32-2.18(2H,m),1.94-1.70(6H,m).
MSm/z(M+H):391.

<Example 0089>

<0089-1>

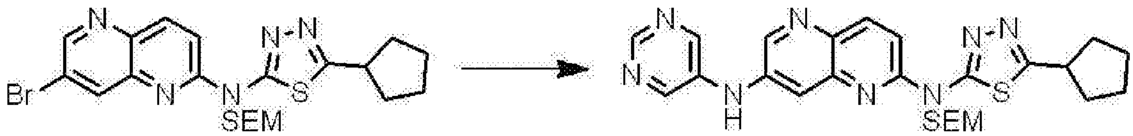

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), pyrimidine-5-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 5.5 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(pyrimidin-5-yl)-$N^2$-((2-(trimethylsilyl)ethoxy)methyl)-1,5-naphthyridine-2,7-diamine (36 mg).
MSm/z(M+H):521.

<0089-2>

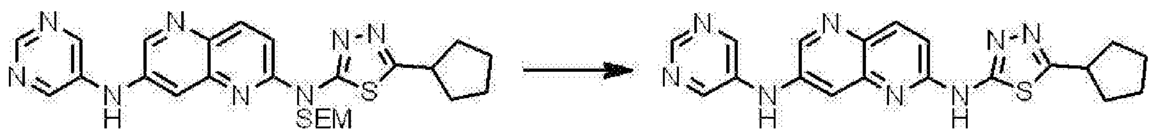

Water (0.1 mL) and trifluoroacetic acid (2 mL) were added to $N^2$-(5-cyclopentyl-1,3,4-thiadiazol-2-yl)-$N^7$-(pyrimidin-5-yl)-$N^2$-((2-(trimethylsilyl)ethoxy)methyl)-1,5-naphthyridine-2,7-diamine (36 mg), followed by stirring at room temperature for 1 hour. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-(pyrimidin-5-yl)-1,5-naphthyridine-2,7-diamine (1.8 mg).
$^1$H-NMR($CDCl_3$/$CD_3OD$=4/1)δ:8.83(1H,s),8.77(2H,s),8.52(1H,d,J=2.7Hz), 8.13(1H,d,J=8.4Hz),7.88(1H,d,J=2.7Hz),7.19(1H,d,J=8.4Hz),3.54-3.44(1H,m),2.32-2.18(2H,m),1.94-1.70(6H,m).
MSm/z(M+H):391.

<Example 0090>

<0090-1>

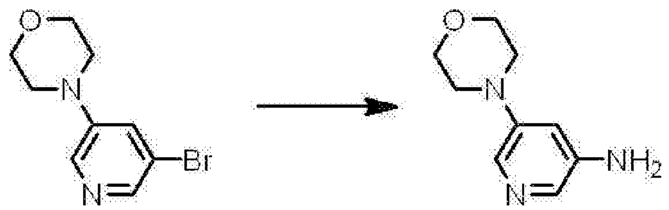

A 25% ammonia aqueous solution (2 mL) was added to a mixture of 4-(5-bromopyridin-3-yl)morpholine (447 mg) and copper(I) oxide (130 mg) inN-methylpyrrolidone (2 mL), followed by stirring at 120°C for 60 minutes using a microwave reaction apparatus. Dichloromethane and water were added to the reaction mixture. The organic layer was collected by separation, and dried over anhydrous sodium sulfate. The obtained solution was purified by silica gel column chromatography (methanol-ethyl acetate), thereby obtaining 5-morpholinopyridine-3-amine (34 mg).
MSm/z(M+H):180.

<0090-2>

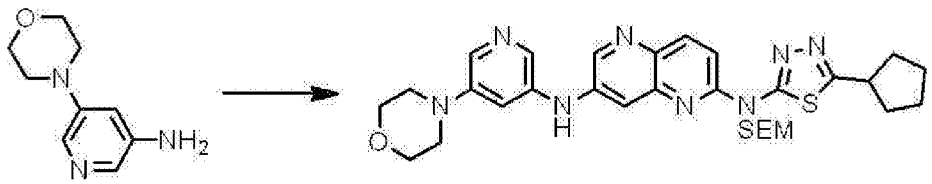

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 5-morpholinopyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 5.5 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(5-morpholinopyridin-3-yl)-$N^2$-((2-(trimethylsilyl)ethoxy)methyl)-1,5-naphthyridine-2,7-diamine (48 mg).
MSm/z(M+H):605.

<0090-3>

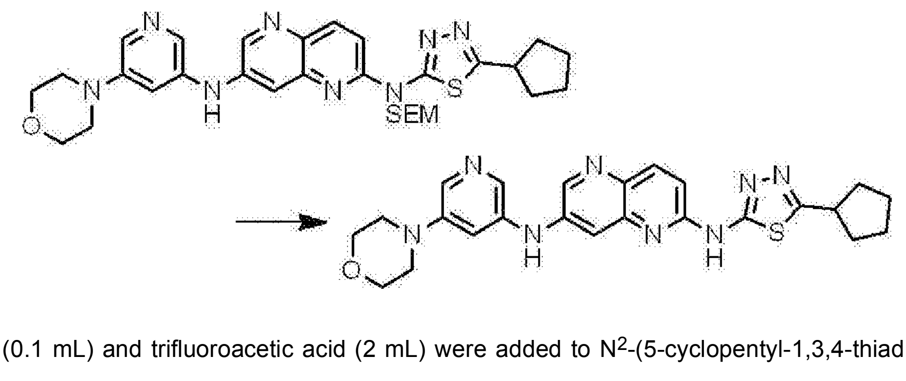

Water (0.1 mL) and trifluoroacetic acid (2 mL) were added to $N^2$-(5-cyclopentyl-1,3,4-thiadiazol-2-yl)-$N^7$-(5-

morpholinopyridin-3-yl)-$N^2$-((2-(trimethylsilyl)ethoxy)methyl)-1,5-naphthyridine-2,7-diamine (48 mg), followed by stirring at room temperature for 2 hours. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-(5-morpholinopyridin-3-yl)-1,5-naphthyridine-2,7-diamine (3.4 mg). $^1$H-NMR($CDCl_3$/$CD_3OD$=4/1)δ:8.48(1H,brs),8.10(1H,d,J=8.4Hz),8.03(1H,brs),7.93(1H,brs), 7.83(1H,brs),7.58(1H,brs),7.13(1H,d,J=8.4Hz),3.92-3.83(4H,m),3.28-3.16(5H,m),2.29-2.18(2H,m),1.94-1.70(6H,m).
MSm/z(M+H):475.

<Example 0091>

<0091-1>

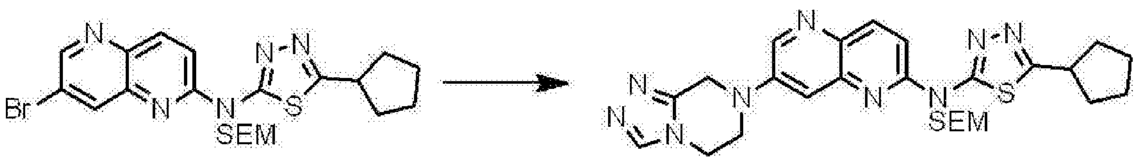

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 5,6,7,8-tetrahydro-[1,2,4]triazolo[4,3-a]pyrazine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 5 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining 5-cyclopentyl-N-(7-(5,6-dihydro-[1,2,4]triazolo[4,3-a]pyrazine-7 (8H)-yl)-1,5-naphthyridin-2-yl)-N-((2-(trimethylsilyl)ethoxy)methyl)-1,3,4-thiadiazole-2-amine (44 mg).
MSm/z(M+H):550.

<0091-2>

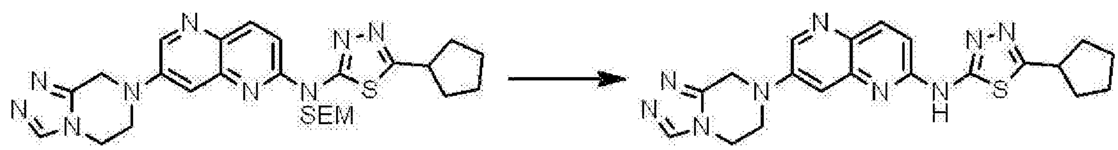

Water (0.1 mL) and trifluoroacetic acid (2 mL) were added to 5-cyclopentyl-N-(7-(5,6-dihydro-[1,2,4]triazolo[4,3-a]pyrazine-7 (8H)-yl)-1,5-naphthyridin-2-yl)-N-((2-(trimethylsilyl)ethoxy)methyl)-1,3,4-thiadiazole-2-amine (44 mg), followed by stirring at room temperature for 1 hour. The solvent was distilled off under reduced pressure, and the obtained residue was purified by silica gel column chromatography (methanol-ethyl acetate, NH silica), thereby obtaining 5-cyclopentyl-N-(7-(5,6-dihydro-[1,2,4]triazolo[4,3-a]pyrazine-7 (8H)-yl)-1,5-naphthyridin-2-yl)-1,3,4-thiadiazole-2-amine (5.9 mg).
$^1$H-NMR($CDCl_3$/$CD_3OD$=4/1)δ:8.68(1H,d,J=2.7Hz),8.35(1H,s),8.15(1H,d,J=9.3Hz), 7.60(1H,d,J=2.7Hz),7.20(1H,d,J=9.3Hz),4.84(2H,s),4.41-4.35(2H,m),3.70-3.66(2H,m),3.56-3.46(1H,m),2.34-2.22(2H,m),1.98-1.74(6H,m).
MSm/z(M+H):420.

<Example 0092>

<0092-1>

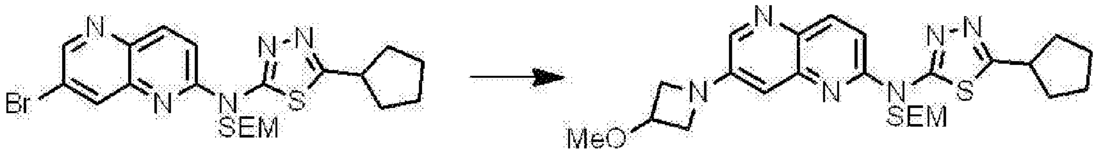

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 3-methoxyazetidine (15 mg), tris(dibenzylideneacetone)dipalladium(0) (6 mg), 2-(dicyclohexylphosphino)-2',4',6'-triisopropylbiphenyl (7 mg), sodium tert-butoxide (26 mg), and 1,4-dioxane (1 mL) was stirred at 100°C for 5 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining 5-cyclopentyl-N-(7-(3-methoxyazetidin-1-yl)-1,5-naphthyridin-2-yl)-N-((2-(trimethylsilyl)ethoxy)methyl)-1,3,4-thiadiazole-2-amine (44 mg).
MSm/z(M+H):513.

<0092-2>

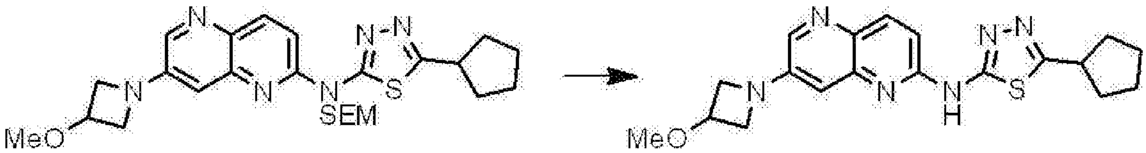

Water (0.1 mL) and trifluoroacetic acid (2 mL) were added to 5-cyclopentyl-N-(7-(3-methoxyazetidin-1-yl)-1,5-naphthyridin-2-yl)-N-((2-(trimethylsilyl)ethoxy)methyl)-1,3,4-thiadiazole-2-amine (44 mg), followed by stirring at room temperature for 1 hour. The solvent was distilled off under reduced pressure, and the obtained residue was purified by silica gel column chromatography (methanol-ethyl acetate, NH silica), thereby obtaining 5-cyclopentyl-N-(7-(3-methoxyazetidin-1-yl)-1,5-naphthyridin-2-yl)-1,3,4-thiadiazole-2-amine (10 mg).
$^1$H-NMR($CDCl_3$/$CD_3OD$=4/1)δ:8.11(1H,d,J=2.4Hz),8.08(1H,d,J=8.7Hz),7.07(1H,d,J=8.7Hz), 7.03(1H,d,J=2.4Hz),4.54-4.45(1H,m),4.40-4.33(2H,m),4.03-3.96(2H,m),3.52-3.43(1H,m),3.36(3H,s),2.34-2.20(2H,m), 1.98-1.74(6H,m).
MSm/z(M+H):383.

<Example 0093>

<0093-1>

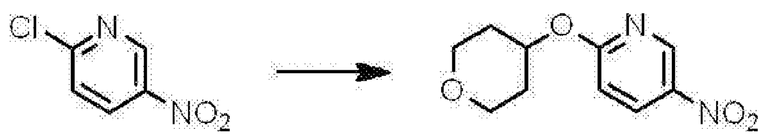

60% sodium hydride (189 mg) was added to a solution of 2-chloro-5-nitropyridine (500 mg) and tetrahydro-2H-pyran-4-ol (386 mg) in tetrahydrofuran (6 mL) under ice-cooling, followed by stirring at the same temperature for 1 hour. A 1 mol/L potassium hydrogen sulfate solution and ethyl acetate were added to the reaction mixture. The organic layer was collected by separation, washed with a saturated sodium chloride aqueous solution, and dried over anhydrous sodium sulfate, and the solvent was distilled off under reduced pressure, thereby obtaining 5-nitro-2-((tetrahydro-2H-pyran-4-yl)oxy)pyridine (838 mg).
MSm/z(M+H):225.

<0093-2>

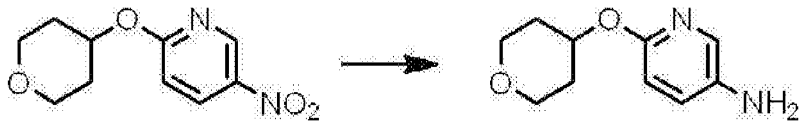

5-Nitro-2-((tetrahydro-2H-pyran-4-yl)oxy)pyridine (838 mg) and ammonium formate (1.98 g) were added to a mixture of 10% palladium-carbon (100 mg) in methanol (15 mL), followed by stirring at room temperature for 6 hours. The insolubles were filtered off using celite, and the solvent was distilled off under reduced pressure. The obtained residue was purified by silica gel column chromatography (ethyl acetate-hexane), thereby obtaining 6-((tetrahydro-2H-pyran-4-yl)oxy)pyridine-3-amine (349 mg).
$^1$H-NMR($CDCl_3$)$\delta$:7.63(1H,d,J=2.7Hz),7.03(1H,dd,J=9.2Hz,2.7Hz),6.60(1H,d,J=9.0Hz), 5.13-5.03(1H,m),4.02-3.93(2H,m),3.64-3.54(2H,m),2.09-1.98(2H,m),1.81-1.68(2H,m).

<0093-3>

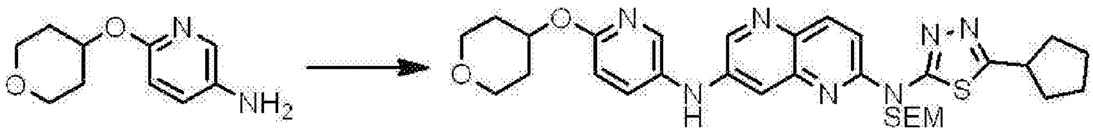

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 6-((tetrahydro-2H-pyran-4-yl)oxy)pyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 5 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(6-((tetrahydro-2H-pyran-4-yl)oxy)pyridin-3-yl)-$N^2$-((2-(trimethylsilyl)ethoxy)methyl)-1,5-naphthyridine-2,7-diamine (60 mg).
MSm/z(M+H):620.

<0093-4>

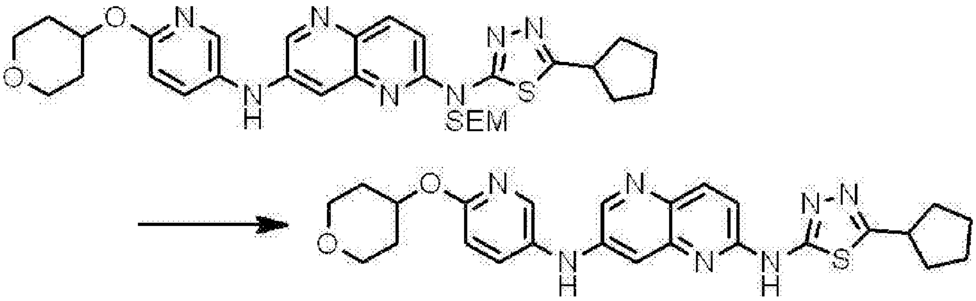

Water (0.1 mL) and trifluoroacetic acid (2 mL) were added to $N^2$-(5-cyclopentyl-1,3,4-thiadiazol-2-yl)-$N^7$-6-((tetrahydro-2H-pyran-4-yl)oxy)pyridin-3-yl)-$N^2$-((2-(trimethylsilyl)ethoxy)methyl)-1,5-naphthyridine-2,7-diamine (60 mg), followed by stirring at room temperature for 1 hour. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-(6-((tetrahydro-2H-pyran-4-yl)oxy)pyridin-3-yl)-1,5-naphthyridine-2,7-diamine (7.3 mg).

$^1$H-NMR($CDCl_3$/$CD_3OD$=4/1)δ:8.41(1H,d,J=2.7Hz),8.15(1H,d,J=2.7Hz),8.06(1H,d,J=8.4Hz),7.65(1H,dd,J=8.4Hz,2.7Hz),7.49(1H,brs),7.08(1H,d,J=8.4Hz),6.86(1H,d,J=8.4Hz),5.26-5.15(1H,m),3.72-3.62(4H,m),3.53-3.42(1H,m),2.29-2.04(4H,m),1.93-1.68(8H,m).
MSm/z(M+H):490.

<Example 0094>

<0094-1>

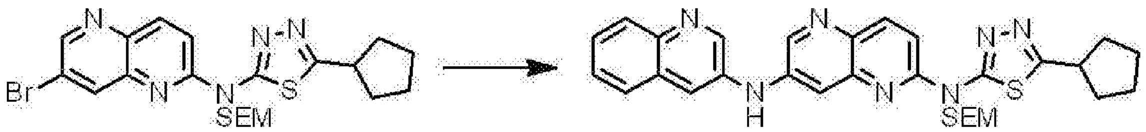

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), quinoline-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 8 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(quinolin-3-yl)-$N^2$-((2-(trimethylsilyl)ethoxy)methyl)-1,5-naphthyridine-2,7-diamine (55 mg).
MSm/z(M+H):570.

<0094-2>

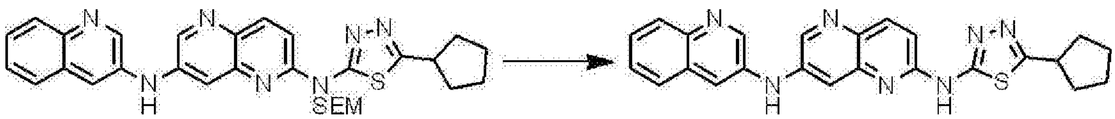

Water (0.1 mL) and trifluoroacetic acid (2 mL) were added to $N^2$-(5-cyclopentyl-1,3,4-thiadiazol-2-yl)-$N^7$-(quinolin-3-yl)-$N^2$-((2-(trimethylsilyl)ethoxy)methyl)-1,5-naphthyridine-2,7-diamine (55 mg), followed by stirring at room temperature for 1 day. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-(quinolin-3-yl)-1,5-naphthyridine-2,7-diamine (8.9 mg).
$^1$H-NMR($CDCl_3$/$CD_3OD$=4/1)δ:8.82(1H,d,J=2.7Hz),8.60(1H,d,J=1.8Hz),8.14(1H,d,J=9.3Hz),8.12(1H,d,J=2.7Hz),8.05(1H,d,J=4.8Hz),7.95(1H,d,J=2.7Hz),7.83-7.54(3H,m),7.17(1H,d,J=9.3Hz),3.46(1H,m),2.34-2.22(2H,m),1.98-1.74(6H,m).
MSm/z(M+H):440.

<Example 0095>

<0095-1>

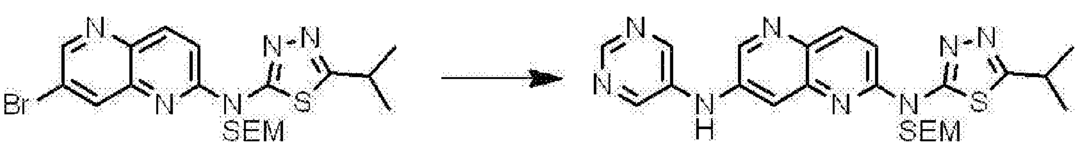

A mixture of N-(7-bromo-1,5-naphthyridin-2-yl)-5-isopropyl-N-((2-(trimethylsilyl)ethoxy)methyl)-1,3,4-thiadiazole-2-amine (20 mg), pyrimidine-5-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 9 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-isopropyl-1,3,4-thiadiazol-2-yl)-$N^7$-(pyrimidin-5-yl)-$N^2$-((2-(trimethylsilyl)ethoxy)methyl)-1,5-naphthyridine-2,7-diamine (58 mg).
MSm/z(M+H):495.

<0095-2>

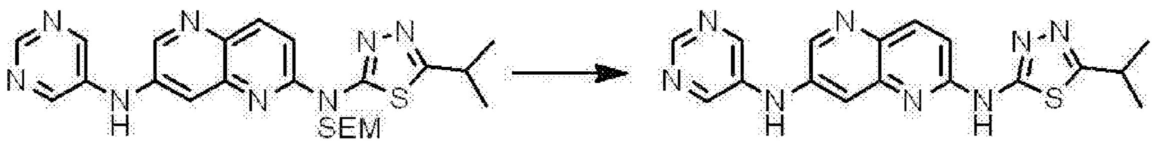

Water (0.1 mL) and trifluoroacetic acid (2 mL) were added to $N^2$-(5-isopropyl-1,3,4-thiadiazol-2-yl)-$N^7$-(pyrimidin-5-yl)-$N^2$-((2-(trimethylsilyl)ethoxy)methyl)-1,5-naphthyridine-2,7-diamine (58 mg), followed by stirring at room temperature for 3 hours. The solvent was distilled off under reduced pressure, and the obtained residue was purified by silica gel column chromatography (methanol-ethyl acetate, NH silica), thereby obtaining $N^2$-(5-isopropyl-1,3,4-thiadiazol-2-yl)-$N^7$-(pyrimidin-5-yl)-1,5-naphthyridine-2,7-diamine (7.1 mg).
$^1$H-NMR($CDCl_3$/$CD_3OD$=4/1)δ:8.83(1H,s),8.77(2H,s),8.53(1H,d,J=2.7Hz), 8.14(1H,d,J=9.3Hz),7.88(1H,d,J=2.7Hz),7.20(1H,d,J=9.3Hz),3.47-3.35(1H,m),1.47(6H,d,J=6.6Hz).
MSm/z(M+H):365.

<Example 0096>

<0096-1>

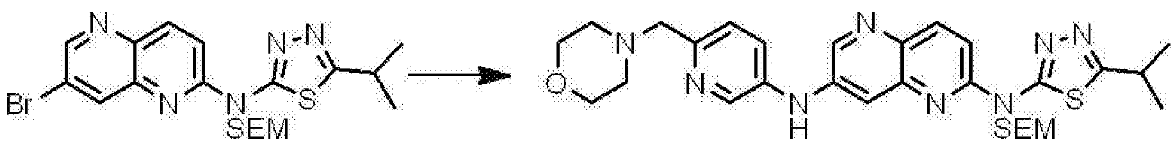

A mixture of N-(7-bromo-1,5-naphthyridin-2-yl)-5-isopropyl-N-((2-(trimethylsilyl)ethoxy)methyl)-1,3,4-thiadiazole-2-amine (20 mg), 6-(morpholinomethyl)pyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 9 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-isopropyl-1,3,4-thiadiazol-2-yl)-$N^7$-(6-(morpholinomethyl)pyridin-3-yl)-$N^2$-((2-(trimethylsilyl)ethoxy)methyl)-1,5-naphthyridine-2,7-diamine (55 mg).
MSm/z(M+H):593.

<0096-2>

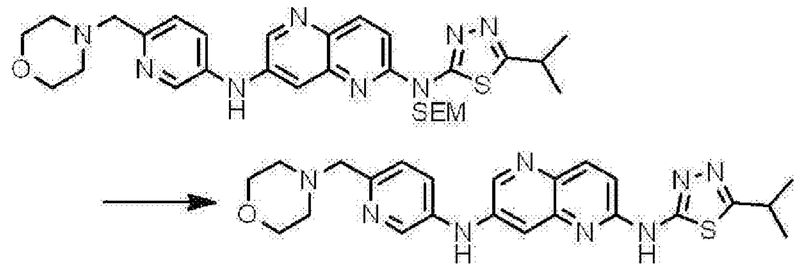

Water (0.1 mL) and trifluoroacetic acid (2 mL) were added to $N^2$-(5-isopropyl-1,3,4-thiadiazol-2-yl)-$N^7$-(6-(morpholinomethyl)pyridin-3-yl)-$N^2$-((2-(trimethylsilyl)ethoxy)methyl)-1,5-naphthyridine-2,7-diamine (55 mg), followed by stirring at room temperature for 3 hours. The solvent was distilled off under reduced pressure, and the obtained residue was purified by silica gel column chromatography (methanol-ethyl acetate, NH silica), thereby obtaining $N^2$-(5-isopropyl-1,3,4-thiadiazol-2-yl)-$N^7$-(6-(morpholinomethyl)pyridin-3-yl)-1,5-naphthyridine-2,7-diamine (9.1 mg). $^1$H-NMR($CDCl_3$/$CD_3OD$=4/1)δ:8.49(2H,d,J=2.7Hz),8.11(1H,d,J=9.0Hz),7.81(1H,d,J=2.7Hz), 7.72(1H,dd,J=8.7Hz,2.7Hz),7.48(1H,d,J=8.7Hz),7.17(1H,d,J=9.0Hz),3.80-3.75(4H,m),3.67(2H,s),3.48-3.36(1H,m),2.60-2.56(4H,m),1.47(6H,d,J=6.6Hz).
MSm/z(M+H):463.

<Example 0097>

<0097-1>

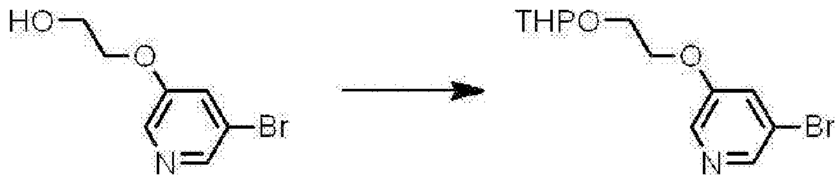

Dihydropyran (0.262 mL) and camphorsulfonic acid (505 mg) were added sequentially to a solution of 2-((5-bromopyridin-3-yl)oxy)ethanol (318 mg) in dichloromethane (7 mL) at room temperature, followed by stirring at the same temperature for 26 hours. A saturated sodium hydrogen carbonate aqueous solution and ethyl acetate were added to the reaction mixture. The organic layer was collected by separation, washed with a saturated sodium chloride aqueous solution, and dried over anhydrous sodium sulfate, and the solvent was distilled off under reduced pressure, thereby obtaining 3-bromo-5-(2-((tetrahydro-2H-pyran-2-yl)oxy)ethoxy)pyridine (779 mg).
MSm/z(M+H):302.

<0097-2>

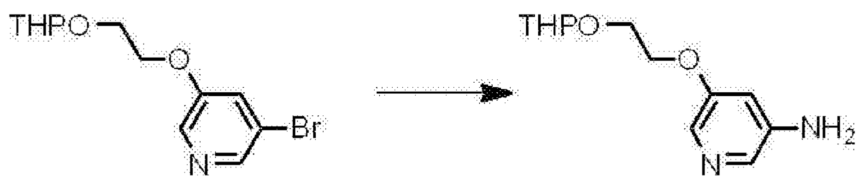

A25% ammonia aqueous solution (2 mL) was added to a mixture of 3-bromo-5-(2-((tetrahydro-2H-pyran-2-yl)oxy)ethoxy)pyridine (779 mg) and copper(I) oxide (103 mg) in N-methylpyrrolidone (2 mL), followed by stirring at 120°C for 60 minutes using a microwave reaction apparatus. Dichloromethane and water were added to the reaction mixture. The organic layer was collected by separation, and dried over anhydrous sodium sulfate. The obtained solution was purified by silica gel column chromatography (methanol-ethyl acetate), thereby obtaining 5-(2-((tetrahydro-2H-pyran-2-yl)oxy)ethoxy)pyridine-3-amine (456 mg).

MSm/z(M+H):239.

<0097-3>

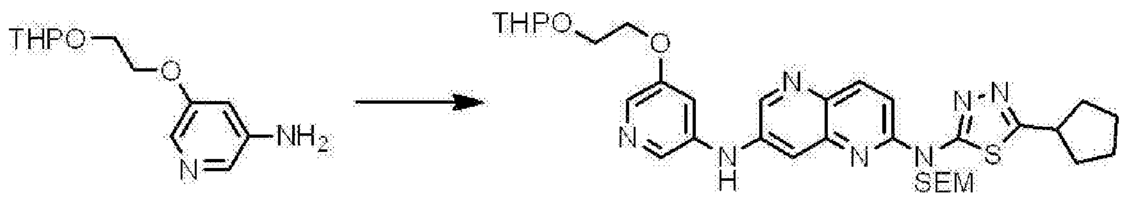

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 5-(2-((tetrahydro-2H-pyran-2-yl)oxy)ethoxy)pyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 9 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(5-(2-((tetrahydro-2H-pyran-2-yl)oxy)ethoxy)pyridin-3-yl)-$N^2$-((2-(trimethylsilyl)ethoxy)methyl)-1,5-naphthyridine-2,7-diamine (45 mg).
MSm/z(M+H):664.

<0097-4>

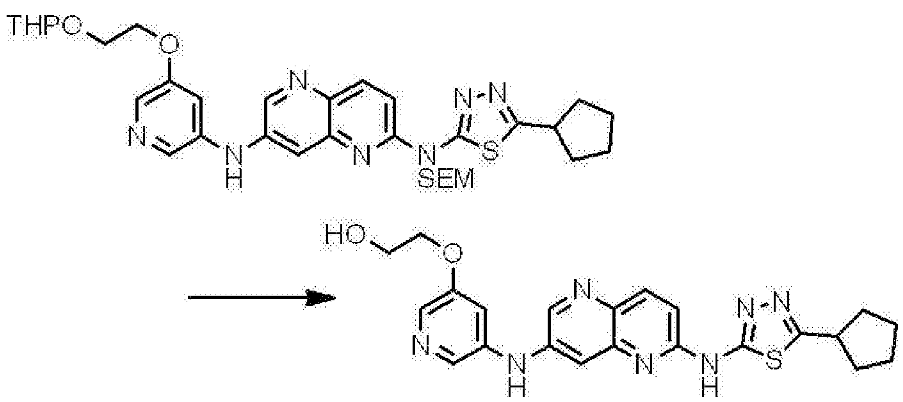

Water (0.1 mL) and trifluoroacetic acid (2 mL) were added to $N^2$-(5-cyclopentyl-1,3,4-thiadiazol-2-yl)-$N^7$-(5-(2-((tetrahydro-2H-pyran-2-yl)oxy)ethoxy)pyridin-3-yl)-$N^2$-((2-(trimethylsilyl)ethoxy)methyl)-1,5-naphthyridine-2,7-diamine (45 mg), followed by stirring at room temperature for 4 hours. The solvent was distilled off under reduced pressure, and the obtained residue was purified by silica gel column chromatography (methanol-ethyl acetate, NH silica), thereby obtaining 2-((5-((6-((5-cyclopentyl-1,3,4-thiadiazol-2-yl)amino)-1,5-naphthyridin-3-yl)amino)pyridin-3-yl)oxy)ethanol (4.1 mg).
$^1$H-NMR($CDCl_3$/$CD_3OD$=4/1)δ:8.51(1H,d,J=2.7Hz),8.18(1H,d,J=2.1Hz),8.11(1H,d,J=9.0Hz), 7.95(1H,d,J=1.8Hz),7.85(1H,d,J=2.1Hz),7.25(1H,dd,J=2.7Hz,1.8Hz),7.16(1H,d,J=9.0Hz),4.18-4.09(2H,m),3.98-3.90(2H,m),3.46(1H,m),2.34-2.22(2H,m),1.98-1.74(6H,m).
MSm/z(M+H):450.

<Example 0098>

<0098-1>

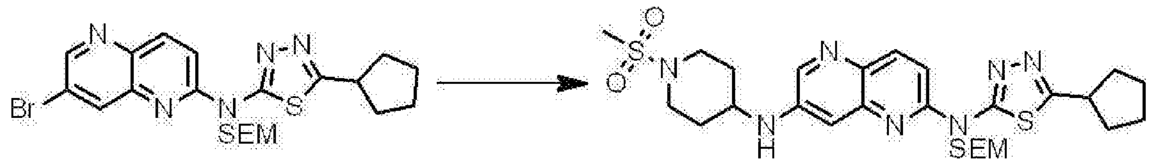

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 1-(methylsulfonyl)piperidine-4-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 20 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate-hexane), thereby obtaining $N^2$-(5-cyclopentyl-1,3,4-thiadiazol-2-yl)-$N^7$-(1-(methylsulfonyl)piperidin-4-yl)-$N^2$-((2-(trimethylsilyl)ethoxy)methyl)-1,5-naphthyridine-2,7-diamine (4.1 mg).
MSm/z(M+H):604.

<0098-2>

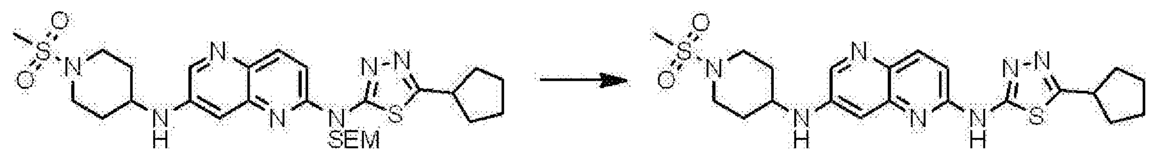

Water (0.1 mL) and trifluoroacetic acid (2 mL) were added to $N^2$-(5-cyclopentyl-1,3,4-thiadiazol-2-yl)-$N^7$-(1-(methylsulfonyl)piperidin-4-yl)-$N^2$-((2-(trimethylsilyl)ethoxy)methyl)-1,5-naphthyridine-2,7-diamine (4.1 mg), followed by stirring at room temperature for 6 hours. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-(1-(methylsulfonyl)piperidin-4-yl)-1,5-naphthyridine-2,7-diamine (2.4 mg).
$^1$H-NMR(DMSO-$d_6$)$\delta$:8.35(1H,d,J=2.7Hz),8.05(1H,d,J=9.3Hz),7.11(1H,d,J=2.7Hz),
7.09(1H,d,J=9.3Hz),3.74-3.44(4H,m),3.07-2.96(2H,m),2.91(3H,s),2.20-2.01(4H,m),1.93-1.62(6H,m),1.59-1.44(2H,m).
MSm/z(M+H):474.

<Example 0099>

<0099-1>

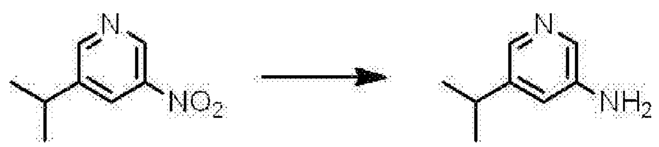

3-Isopropyl-5-nitropyridine (103 mg) and ammonium formate (390 mg) were added to a mixture of 10% palladium-carbon (20 mg) in methanol (6 mL), followed by stirring at room temperature for 1 day. The insolubles were filtered off using celite, the solvent was distilled off under reduced pressure, and the obtained residue was purified by silica gel column chromatography (methanol-ethyl acetate-hexane), thereby obtaining 5-isopropylpyridine-3-amine (18 mg).
MSm/z(M+H):137.

<0099-2>

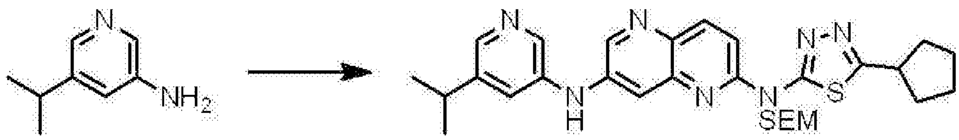

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 5-isopropylpyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 9 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(5-isopropylpyridin-3-yl)-$N^2$-((2-(trimethylsilyl)ethoxy)methyl)-1,5-naphthyridine-2,7-diamine (57 mg).
MSm/z(M+H):562.

<0099-3>

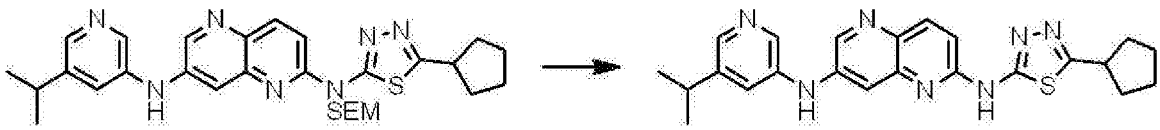

Water (0.1 mL) and trifluoroacetic acid (2 mL) were added to $N^2$-(5-cyclopentyl-1,3,4-thiadiazol-2-yl)-$N^7$-(5-isopropylpyridin-3-yl)-$N^2$-((2-(trimethylsilyl)ethoxy)methyl)-1,5-naphthyridine-2,7-diamine (57 mg), followed by stirring at room temperature for 1 day. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-(5-isopropylpyridin-3-yl)-1,5-naphthyridine-2,7-diamine (14 mg).
$^1$H-NMR($CDCl_3$/$CD_3OD$=4/1)$\delta$:8.51(1H,d,J=2.7Hz),8.34(1H,d,J=2.7Hz),8.12(1H,d,J=2.7Hz), 8.11(1H,d,J=9.0Hz),7.94(1H,d,J=2.7Hz),7.86(1H,d,J=2.7Hz),7.17(1H,d,J=9.0Hz),3.54-3.40(1H,m),3.10-2.90(1H,m),2.28-2.17(2H,m),1.90-1.72(6H,m),1.30(6H,d,J=4.2Hz).
MSm/z(M+H):432.

<Example 0100>

<0100-1>

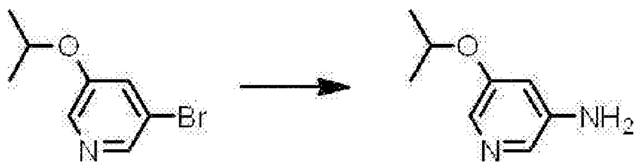

A 25% ammonia aqueous solution (2 mL) was added to a mixture of 3-bromo-5-isopropyloxypyridine (590 mg) and copper(I) oxide (195 mg) in N-methylpyrrolidone (2 mL), followed by stirring at 120°C for 60 minutes using a microwave reaction apparatus. Ethyl acetate and water were added to the reaction mixture. The organic layer was collected by separation, and dried over anhydrous sodium sulfate. The obtained residue was purified by silica gel column chromatography (methanol-ethyl acetate), thereby obtaining 5-isopropyloxypyridine-3-amine (302 mg).
MSm/z(M+H):153.

<0100-2>

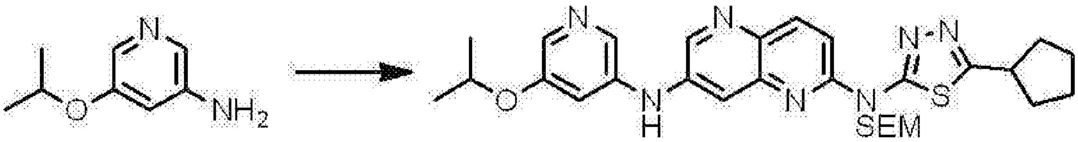

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 5-isopropyloxypyridine-3-amine (15 mg), tris(dibenzylideneacetone)dipalladium(0) (6 mg), 2-(dicyclohexylphosphino)-2',4',6'-triisopropylbiphenyl (7 mg) and sodium tert-butoxide (26 mg) in 1,4-dioxane (1 mL) was stirred at 100°C for 9 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(5-isopropyloxypyridin-3-yl)-$N^2$-((2-(trimethylsilyl)ethoxy)methyl)-1,5-naphthyridine-2,7-diamine (76 mg).
MSm/z(M+H):578.

<0100-3>

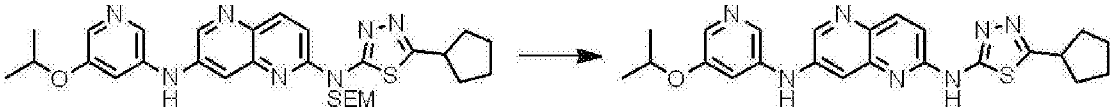

Water (0.1 mL) and trifluoroacetic acid (2 mL) were added to $N^2$-(5-cyclopentyl-1,3,4-thiadiazol-2-yl)-$N^7$-(5-isopropyloxypyridin-3-yl)-$N^2$-((2-(trimethylsilyl)ethoxy)methyl)-1,5-naphthyridine-2,7-diamine (76 mg), followed by stirring at room temperature for 1 day. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-(5-isopropyloxypyridin-3-yl)-1,5-naphthyridine-2,7-diamine (14 mg).
$^1$H-NMR($CDCl_3$/$CD_3OD$=4/1)$\delta$:8.52(1H,d,J=2.7Hz),8.13(1H,d,J=9.0Hz),8.10(1H,d,J=2.1Hz), 7.91(1H,d,J=2.7Hz),7.88(1H,d,J=2.1Hz),7.34(1H,dd,J=2.7Hz,2.1Hz),7.19(1H,d,J=9.0Hz), 4.74-4.57(1H,m),3.54-3.42(1H,m),2.30-2.18(2H,m),1.94-1.72(6H,m),1.42(6H,d,J=8.7Hz).
MSm/z(M+H):448.

<Example 0101>

<0101-1>

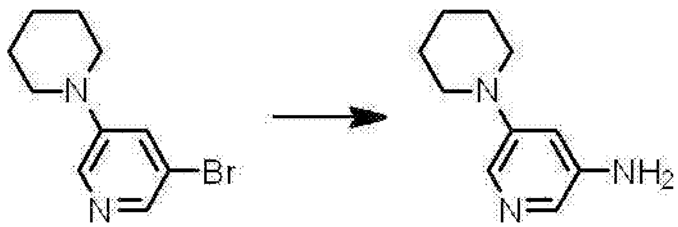

A 25% ammonia aqueous solution (2 mL) was added to a mixture of 3-bromo-5-(piperidin-1-yl)pyridine (119 mg), and copper(I) oxide (36 mg) in N-methylpyrrolidone (2 mL), followed by stirring at 120°C for 60 minutes using a microwave reaction apparatus. Ethyl acetate and water were added to the reaction mixture. The organic layer was collected by separation, and dried over anhydrous sodium sulfate. The obtained residue was purified by silica gel column

chromatography (methanol-ethyl acetate), thereby obtaining 5-(piperidin-1-yl)pyridine-3-amine (28 mg).
MSm/z(M+H):178.

<0101-2>

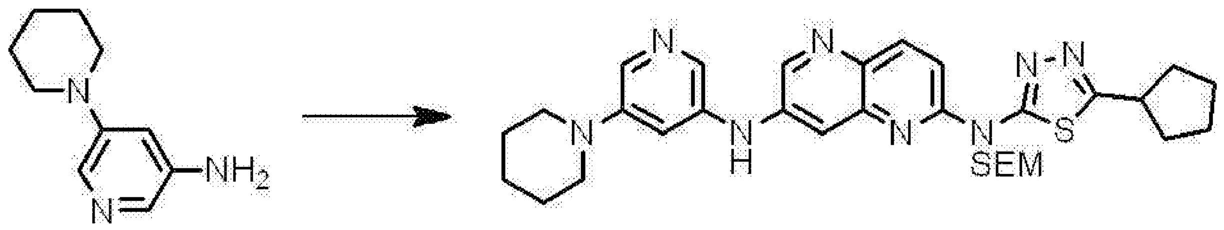

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 5-(piperidin-1-yl)pyridine-3-amine (15 mg), tris(dibenzylideneacetone)dipalladium(0) (6 mg), 2-(dicyclohexylphosphino)-2',4',6'-triisopropylbiphenyl (7 mg), and sodium tert-butoxide (26 mg) in 1,4-dioxane (1 mL) was stirred at 100°C for 9 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(5-(piperidin-1-yl)pyridin-3-yl)-$N^2$-((2-(trimethylsilyl)ethoxy)methyl)-1,5-naphthyridine-2,7-diamine (56 mg).
MSm/z(M+H):603.

<0101-3>

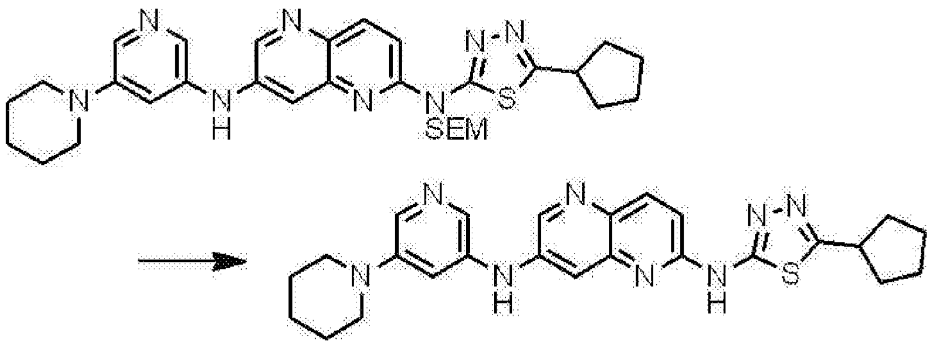

Water (0.1 mL) and trifluoroacetic acid (2 mL) were added to $N^2$-(5-cyclopentyl-1,3,4-thiadiazol-2-yl)-$N^7$-(5-(piperidin-1-yl)pyridin-3-yl)-$N^2$-((2-(trimethylsilyl)ethoxy)methyl)-1,5-naphthyridine-2,7-diamine (56 mg), followed by stirring at room temperature for 4 hours. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-(5-(piperidin-1-yl)pyridin-3-yl)-1,5-naphthyridine-2,7-diamine (4.7 mg).
$^1$H-NMR($CDCl_3$/$CD_3OD$=4/1)δ:8.49(1H,d,J=2.7Hz),8.11(1H,d,J=8.7Hz),7.95-7.91(2H,m),7.86-7.83(1H,m),7.28-7.25(1H,m),7.14(1H,d,J=8.7Hz),3.51-3.42(1H,m),3.31-3.25(4H,m),2.30-2.18(2H,m),1.94-1.60(12H,m).
MSm/z(M+H):473.

<Example 0102>

<0102-1>

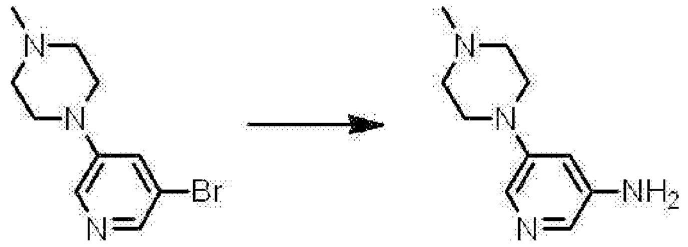

A 25% ammonia aqueous solution (1 mL) was added to a mixture of 1-(5-bromopyridin-3-yl)-4-methylpiperazine (28 mg), and copper(I) oxide (8 mg) in N-methylpyrrolidone (1 mL), followed by stirring at 120°C for 2.5 hours using a microwave reaction apparatus. Dichloromethane and water were added to the reaction mixture. The organic layer was collected by separation, and dried over anhydrous sodium sulfate. The obtained residue was purified by silica gel column chromatography (methanol-ethyl acetate), thereby obtaining 5-(4-methylpiperazin-1-yl)pyridine-3-amine (11 mg).
MSm/z(M+H):193.

<0102-2>

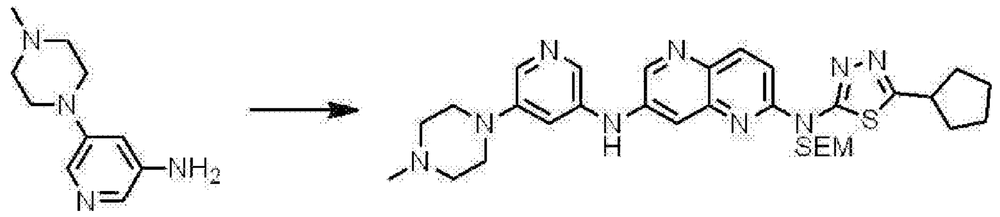

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 5-(4-methylpiperazin-1-yl)pyridine-3-amine (11 mg), tris(dibenzylideneacetone)dipalladium(0) (6 mg), 2-(dicyclohexylphosphino)-2',4',6'-triisopropylbiphenyl (7 mg), and sodium tert-butoxide (26 mg) in 1,4-dioxane (1 mL) was stirred at 100°C for 9 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(5-(4-methylpiperazin-1-yl)pyridin-3-yl)-$N^2$-((2-(trimethylsilyl)ethoxy)methyl)-1,5-naphthyridine-2,7-diamine (47 mg).
MSm/z(M+H):618.

<0102-3>

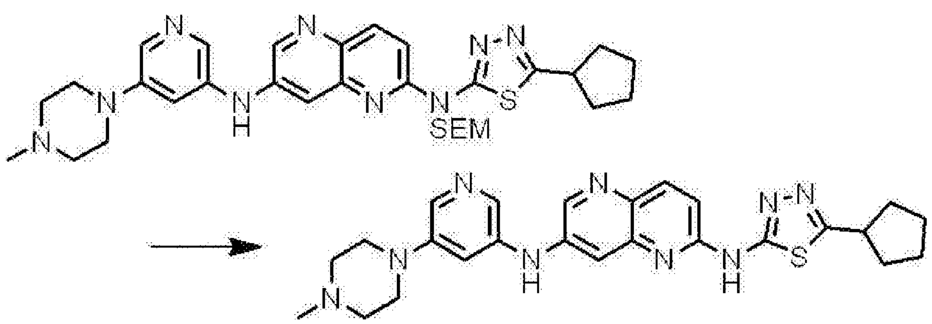

Water (0.1 mL) and trifluoroacetic acid (2 mL) were added to $N^2$-(5-cyclopentyl-1,3,4-thiadiazol-2-yl)-$N^7$-(5-(4-methylpiperazin-1-yl)pyridin-3-yl)-$N^2$-((2-(trimethylsilyl)ethoxy)methyl)-1,5-naphthyridine-2,7-diamine (47 mg), followed by stirring at room temperature for 4 hours. The solvent was distilled off under reduced pressure, and the obtained residue was purified by silica gel column chromatography (methanol-ethyl acetate, NH silica), thereby obtaining $N^2$-(5-

cyclopentyl-1,3,4-thiadiazol-2-yl)-$N^7$-(5-(4-methylpiperazin-1-yl)pyridin-3-yl)-1,5-naphthyridine-2,7-diamine (2.4 mg).
$^1$H-NMR($CDCl_3$/$CD_3OD$=4/1)δ:8.49(1H,d,J=2.7Hz),8.11(1H,d,J=9.0Hz),8.04(1H,d,J=1.8Hz),7.94(1H,d,J =2.7Hz),7.82(1H,d,J=2.4Hz),7.16(1H,dd,J=2.4Hz,1.8Hz),7.14(1H,d,J=9.0Hz),3.54-3.42(1H,m),3.33-3.27(4H,m),2.67-2.61(4H,m),2.38(3H,s),2.30-2.18(2H,m),1.94-1.72(6H,m).
MSm/z(M+H):488.

<Example 0103>

<0103-1>

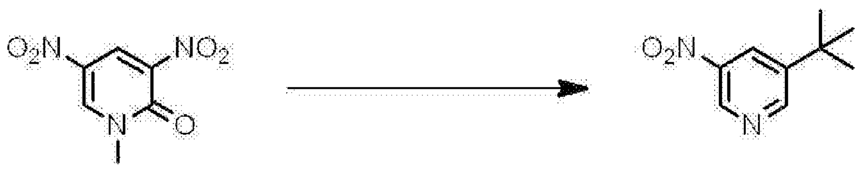

3,3-Dimethylbutanal (200 mg) was added to a solution of 1-methyl-3,5-dinitropyridin-2(1H)-one (200 mg) and ammonium acetate (790 mg) in methanol (18 mL) and 7 mol/L ammonia/methanol (2 mL), followed by stirring at 100°C for 1 hour using a microwave reaction apparatus. The reaction mixture was cooled to room temperature, the solvent was distilled off under reduced pressure, and ethyl acetate and water were added thereto. The organic layer was collected by separation, washed with a saturated sodium chloride aqueous solution, and dried over anhydrous sodium hydrogen sulfate. The obtained residue was purified by silica gel column chromatography (ethyl acetate-hexane), thereby obtaining 3-(tert-butyl)-5-nitropyridine (72 mg).
$^1$H-NMR($CDCl_3$)δ:9.28(1H,d,J=2.7Hz),8.96(1H,d,J=2.1Hz),8.47-8.44(1H,m),1.43(9H,s).

<0103-2>

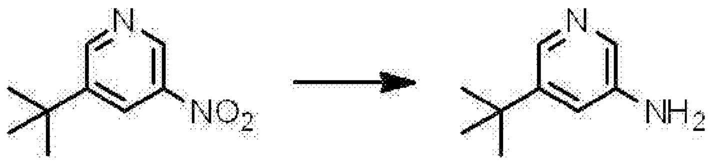

3-(tert-Butyl)-5-nitropyridine (72 mg) and ammonium formate (503 mg) were added to a mixture of 10% palladium-carbon (20 mg) in methanol (4 mL), followed by stirring at room temperature for 6 hours, and for 1.5 hours under heating to reflux. The insolubles were filtered off using celite, and the solvent was distilled off under reduced pressure. The obtained residue was purified by silica gel column chromatography (methanol-ethyl acetate-hexane), thereby obtaining 5-(tert-butyl)pyridine-3-amine (48 mg).
MSm/z(M+H):151.

<0103-3>

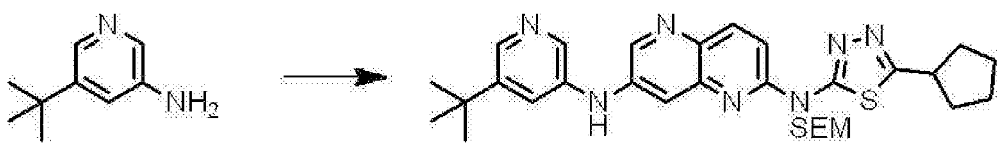

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 5-(tert-butyl)pyridine-3-amine (15 mg), tris(dibenzylideneacetone)dipalladium(0) (6 mg), 2-(di-

cyclohexylphosphino)-2',4',6'-triisopropylbiphenyl (7 mg), and sodium tert-butoxide (26 mg) in 1,4-dioxane (1 mL) was stirred at 100°C for 4 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^7$-(5-(tert-butyl)pyridin-3-yl)-$N^2$-(5-cyclopentyl-1,3,4-thiadiazol-2-yl)-$N^2$-((2-(trimethylsilyl)ethoxy)methyl)-1,5-naphthyridine-2,7-diamine (75 mg).
MSm/z(M+H):576.

<0103-4>

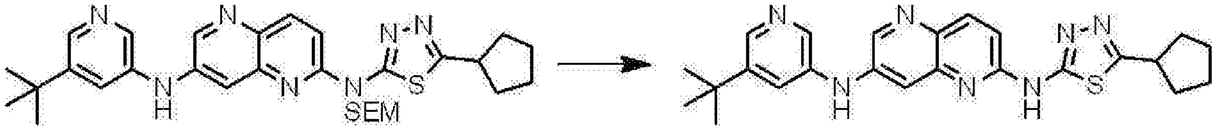

Water (0.1 mL) and trifluoroacetic acid (2 mL) were added to $N^7$-(5-(tert-butyl)pyridin-3-yl)-$N^2$-(5-cyclopentyl-1,3,4-thiadiazol-2-yl)-$N^2$-((2-(trimethylsilyl)ethoxy)methyl)-1,5-naphthyridine-2,7-diamine (75 mg), followed by stirring at room temperature for 1 hour. The solvent was distilled off under reduced pressure, and the obtained residue was purified by silica gel column chromatography (methanol-ethyl acetate, NH silica), thereby obtaining $N^7$-(5-(tert-butyl)pyridin-3-yl)-$N^2$-(5-cyclopentyl-1,3,4-thiadiazol-2-yl)-1,5-naphthyridine-2,7-diamine (15 mg).
$^1$H-NMR($CDCl_3$/$CD_3OD$=4/1)δ:8.49(1H,brs),8.32(1H,brs),8.28(1H,brs),8.12(1H,d,J=8.7Hz),7.94-7.83(2H,m),7.17(1H,d,J=8.7Hz),3.54-3.42(1H,m),2.30-2.18(2H,m),1.94-1.72(6H,m),1.42(9H,s).
MSm/z(M+H):446.

<Example 0104>

<0104-1>

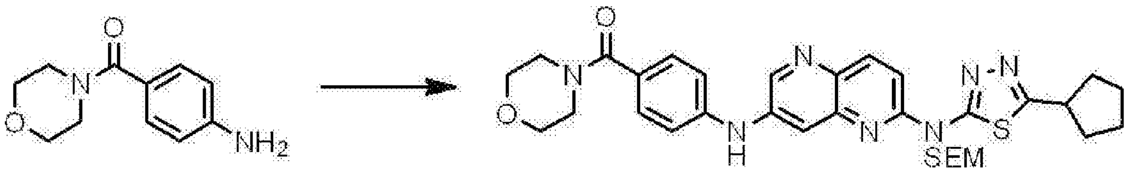

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), (4-aminophenyl) (morpholino)methanone (15 mg), tris(dibenzylideneacetone)dipalladium(0) (6 mg), 2-(dicyclohexylphosphino)-2',4',6'-triisopropylbiphenyl (7 mg), and sodium tert-butoxide (26 mg) in 1,4-dioxane (1 mL) was stirred at 100°C for 6 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining (4-((6-((5-cyclopentyl-1,3,4-thiadiazol-2-yl)((2-(trimethylsilyl)ethoxy)methyl)amino)-1,5-naphthyridin-3-yl)amino)phenyl) (morpholino)methanone (44 mg).
MSm/z(M+H):632.

<0104-2>

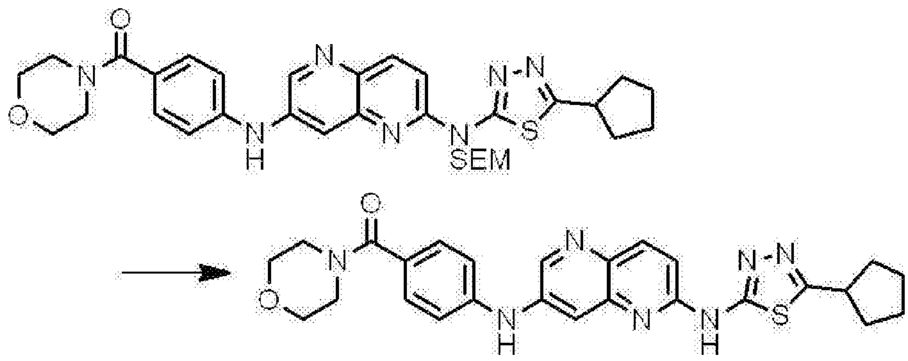

Water (0.1 mL) and trifluoroacetic acid (2 mL) were added to (4-((6-((5-cyclopentyl-1,3,4-thiadiazol-2-yl)((2-(trimethylsilyl)ethoxy)methyl)amino)-1,5-naphthyridin-3 -yl)amino)phenyl) (morpholino)methanone (44 mg), followed by stirring at room temperature for 30 minutes. The solvent was distilled off under reduced pressure, and the obtained residue was purified by silica gel column chromatography (methanol-ethyl acetate, NH silica), thereby obtaining (4-((6-((5-cyclopentyl-1,3,4-thiadiazol-2-yl)amino)-1,5-naphthyridin-3-yl)amino)phenyl) (morpholino)methanone (13 mg).
$^1$H-NMR($CDCl_3$/$CD_3OD$=4/1)δ:8.52(1H,brs),8.12(1H,d,J=8.7Hz),7.89(1H,brs),7.46(2H,d,J=8.7Hz),7.32( 2H,d,J=8.7Hz),7.16(1H,d,J=8.7Hz),3.82-3.63(8H,m),3.54-3.42(1H,m),2.30-2.18(2H,m),1.94-1.72(6H,m).
MSm/z(M+H):502.

<Example 0105>

<0105-1>

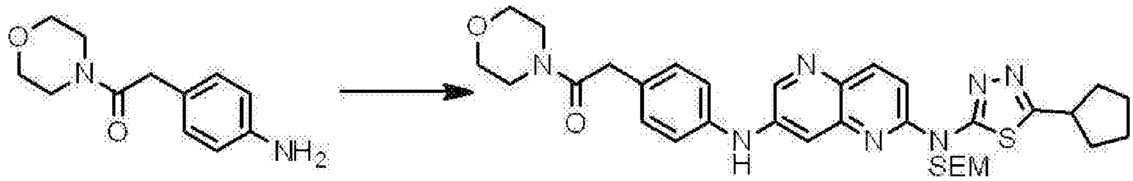

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 2-(4-aminophenyl)-1-morpholinoethanone (15 mg), tris(dibenzylideneacetone)dipalladium(0) (6 mg), 2-(dicyclohexylphosphino)-2',4',6'-triisopropylbiphenyl (7 mg), and sodium tert-butoxide (26 mg) in 1,4-dioxane (1 mL) was stirred at 100°C for 6 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining 2-(4-((6-((5-cyclopentyl-1,3,4-thiadiazol-2-yl)((2-(trimethylsilyl)ethoxy)methyl)amino)-1,5-naphthyridin-3-yl)amino)phenyl)-1-morpholinoethanone (47 mg).
MSm/z(M+H):646.

<0105-2>

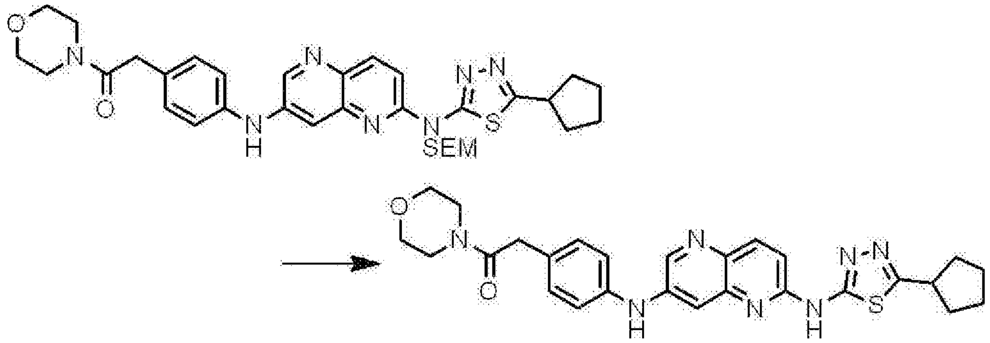

Water (0.1 mL) and trifluoroacetic acid (2 mL) were added to 2-(4-((6-((5-cyclopentyl-1,3,4-thiadiazol-2-yl)((2-(trimethylsilyl)ethoxy)methyl)amino)-1,5-naphthyridin-3-yl)amino)phenyl)-1-morpholinoethanone (47 mg), followed by stirring at room temperature for 30 minutes. The solvent was distilled off under reduced pressure, and the obtained residue was purified by silica gel column chromatography (methanol-ethyl acetate, NH silica), thereby obtaining 2-(4-((6-((5-cyclopentyl-1,3,4-thiadiazol-2-yl)amino)-1,5-naphthyridin-3-yl)amino)phenyl)-1-morpholinoethanone (17 mg).
$^1$H-NMR($CDCl_3$/$CD_3OD$=4/1)$\delta$:8.49(1H,brs),8.11(1H,d,J=8.7Hz),7.87(1H,brs),7.28(2H,d,J=8.1Hz),7.25( 2H,d,J=8.1Hz),7.13(1H,d,J=8.7Hz),3.75(2H,brs),3.72-3.42(9H,m),2.30-2.18(2H,m),1.94-1.72(6H,m).
MSm/z(M+H):516.

<Example 0106>

<0106-1>

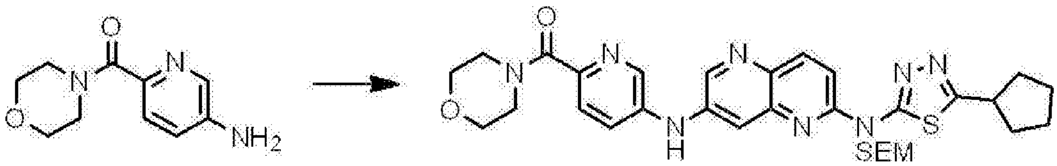

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), (5-aminopyridin-2-yl) (morpholino)methanone (15 mg), tris(dibenzylideneacetone)dipalladium(0) (6 mg), 2-(dicyclohexylphosphino)-2',4',6'-triisopropylbiphenyl (7 mg), and sodium tert-butoxide (26 mg) in 1,4-dioxane (1 mL) was stirred at 100°C for 6 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained residue was purified by silica gel column chromatography (methanol-ethyl acetate-hexane), thereby obtaining (5-((6-((5-cyclopentyl-1,3,4-thiadiazol-2-yl)((2-(trimethylsilyl)ethoxy)methyl)amino)-1,5-naphthyridin-3-yl)amino)pyridin-2-yl) (morpholino)methanone (1.7 mg).
MSm/z(M+H):633.

<0106-2>

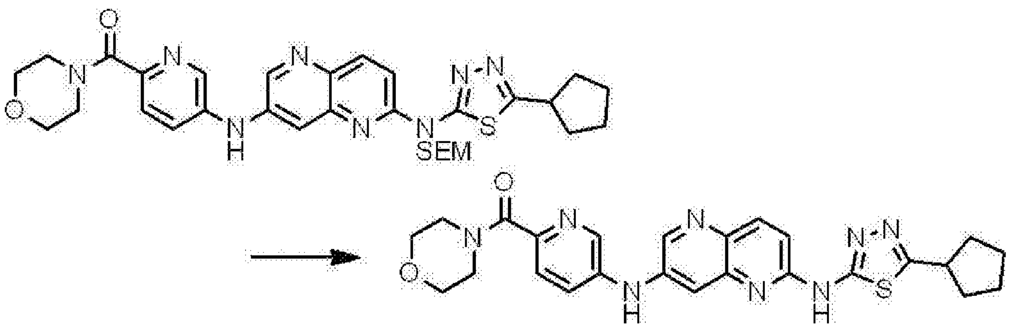

Water (0.1 mL) and a 4 mol/L hydrogen chloride-1,4-dioxane solution (0.5 mL) were added to a solution of (5-((6-((5-cyclopentyl-1,3,4-thiadiazol-2-yl)((2-(trimethylsilyl)ethoxy)methyl)amino)-1,5-naphthyridin-3-yl)amino)pyridin-2-yl) (morpholino)methanone (1.7 mg) in 1,4-dioxane (1.5 mL) at room temperature, followed by stirring at room temperature for 1 day. The solvent was distilled off under reduced pressure, and the obtained residue was purified by silica gel column chromatography (methanol-ethyl acetate, NH silica), thereby obtaining (5-((6-((5-cyclopentyl-1,3,4-thiadiazol-2-yl)amino)-1,5-naphthyridin-3-yl)amino)pyridin-2-yl) (morpholino)methanone (0.87 mg).
$^1$H-NMR($CDCl_3$/$CD_3OD$=4/1)$\delta$:8.53(1H,d,J=2.7Hz),8.50(1H,brs),8.13(1H,d,J=9.0Hz),7.93(1H,d,J=2.7H) ,7.76-7.73(2H,m) ,7.18(1H,d,J=9.0Hz),3.86-3.64(8H,m),3.54-3.42(1H,m),2.30-2.18(2H,m),1.94-1.72(6H,m).
MSm/z(M+H):503.

<Example 0107>

<0107-1>

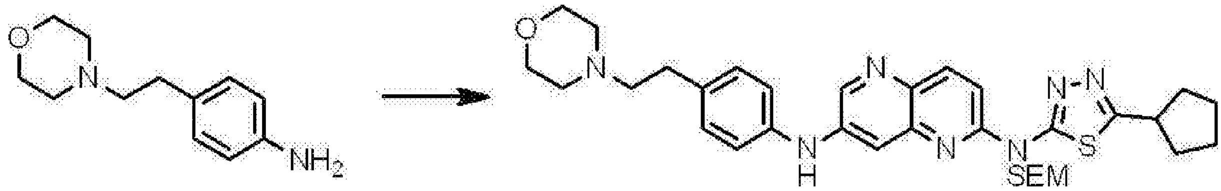

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 4-(2-morpholinoethyl)aniline (15 mg), tris(dibenzylideneacetone)dipalladium(0) (6 mg), 2-(dicyclohexylphosphino)-2',4',6'-triisopropylbiphenyl (7 mg), and sodium tert-butoxide (26 mg) in 1,4-dioxane (1 mL) was stirred at 100°C for 6 hours. The obtained reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(4-(2-morpholinoethyl)phenyl)-$N^2$-((2-(trimethylsilyl)ethoxy)methyl)-1,5-naphthyridine-2,7-diamine (44 mg).
MSm/z(M+H):632.

<0107-2>

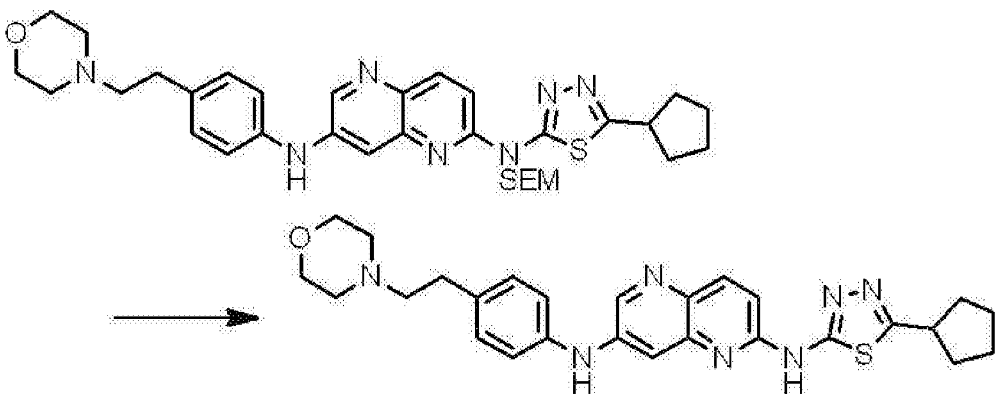

Water (0.1 mL) and trifluoroacetic acid (2 mL) were added to $N^2$-(5-cyclopentyl-1,3,4-thiadiazol-2-yl)-$N^7$-(4-(2-morpholinoethyl)phenyl)-$N^2$-((2-(trimethylsilyl)ethoxy)methyl)-1,5-naphthyridine-2,7-diamine (44 mg), followed by stirring at room temperature for 4 hours. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-(4-(2-morpholinoethyl)phenyl)-1,5-naphthyridine-2,7-diamine(7.1mg). $^1$H-NMR($CDCl_3$/$CD_3OD$=4/1)$\delta$:8.44(1H,brs),8.07(1H,d,J=9.0Hz),7.77(1H,brs),7.41-7.37(2H,brs),7.27-7.23(2H,brs),7.08(1H,d,J=9.0Hz),3.83-3.75(4H,m),3.54-3.42(1H,m),2.90-2.81(2H,m),2.732.56(6H,m),2.18(2H,m),1.94-1.72(6H,m). MSm/z(M+H):502.

<Example 0108>

<0108-1>

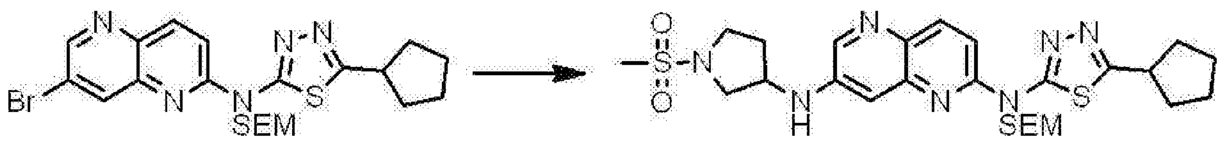

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 1-(methylsulfonyl)pyrrolidine-3-amine hydrochloride (15 mg), tris(dibenzylideneacetone)dipalladium(0) (6 mg), 2-(dicyclohexylphosphino)-2',4',6'-triisopropylbiphenyl (7 mg), and sodium tert-butoxide (26 mg) in 1,4-dioxane (1 mL) was stirred at 100°C for 6 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained residue was purified by silica gel column chromatography (methanol-ethyl acetate-hexane), thereby obtaining $N^2$-(5-cyclopentyl-1,3,4-thiadiazol-2-yl)-$N^7$-(1-(methylsulfonyl)pyrrolidin-3-yl)-$N^2$-((2-(trimethylsilyl)ethoxy)methyl)-1,5-naphthyridine-2,7-diamine (8.9 mg). MSm/z(M+H):590.

<0108-2>

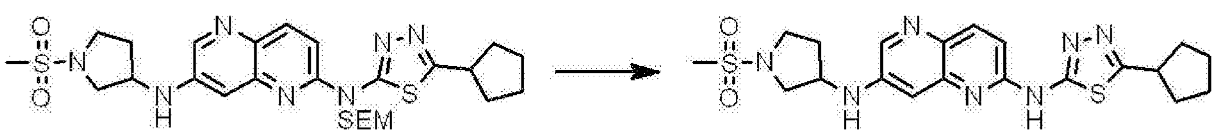

Water (0.1 mL) and a 4 mol/L hydrogen chloride-1,4-dioxane solution (0.5 mL) were added to a solution of $N^2$-(5-cyclopentyl-1,3,4-thiadiazol-2-yl)-$N^7$-(1-(methylsulfonyl)pyrrolidin-3-yl)-$N^2$-((2-(trimethylsilyl)ethoxy)methyl)-1,5-naphthyridine-2,7-diamine (8.9 mg) in 1,4-dioxane (1.5 mL) at room temperature, followed by stirring at room temperature for 14 hours. The solvent was distilled off under reduced pressure, methanol and hexane were added thereto, and the

solid matter was collected by filtration, thereby obtaining $N^2$-(5-cyclopentyl-1,3,4-thiadiazol-2-yl)-$N^7$-(1-(methylsulfonyl)pyrrolidin-3-yl)-1,5-naphthyridine-2,7-diamine (3.1 mg).
$^1$H-NMR(DMSO-$d_6$)δ:8.35(1H,d,J=2.7Hz),8.06(1H,d,J=8.7Hz),7.10(1H,d,J=8.7Hz),7.07(1H,d,J=2.7Hz),3.70-3.14(6H,m),2.93(3H,s),2.38-2.25(2H,m),2.21-2.08(2H,m),2.00-1.64(6H,m).
MSm/z(M+H):460.

<Example 0109>

<0109-1>

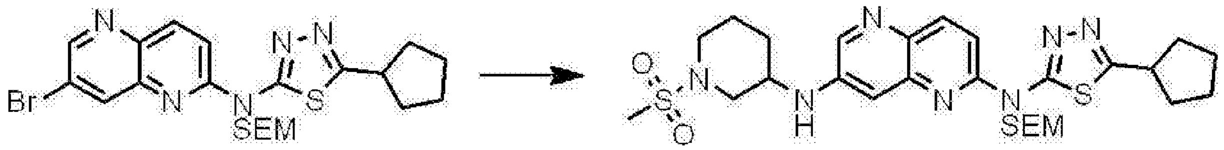

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 1-(methylsulfonyl)piperidine-3-amine hydrochloride (15 mg), tris(dibenzylideneacetone)dipalladium(0) (6 mg), 2-(dicyclohexylphosphino)-2',4',6'-triisopropylbiphenyl (7 mg), and sodium tert-butoxide (26 mg) in 1,4-dioxane (1 mL) was stirred at 100°C for 6 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained residue was purified by silica gel column chromatography (methanol-ethyl acetate-hexane), thereby obtaining $N^2$-(5-cyclopentyl-1,3,4-thiadiazol-2-yl)-$N^7$-(1-(methylsulfonyl)piperidin-3-yl)-$N^2$-((2-(trimethylsilyl)ethoxy)methyl)-1,5-naphthyridine-2,7-diamine (3.8 mg).
MSm/z(M+H):604.

<0109-2>

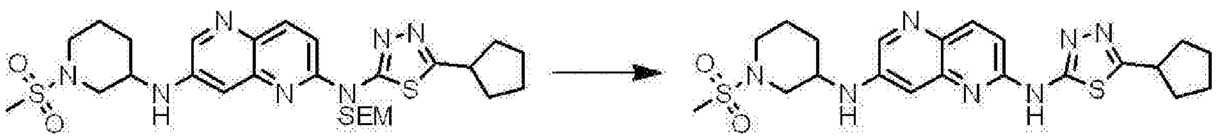

Water (0.1 mL) and a 4 mol/L hydrogen chloride-1,4-dioxane solution (0.5 mL) were added to a solution of $N^2$-(5-cyclopentyl-1,3,4-thiadiazol-2-yl)-$N^7$-(1-(methylsulfonyl)piperidin-3-yl)-$N^2$-((2-(trimethylsilyl)ethoxy)methyl)-1,5-naphthyridine-2,7-diamine (8.9 mg) in 1,4-dioxane (1.5 mL) at room temperature, followed by stirring at room temperature for 14 hours. The solvent was distilled off under reduced pressure, methanol and hexane were added thereto, and the solid matter was collected by filtration, thereby obtaining $N^2$-(5-cyclopentyl-1,3,4-thiadiazol-2-yl)-$N^7$-(1-(methylsulfonyl)piperidin-3-yl)-1,5-naphthyridine-2,7-diamine (0.22 mg).
$^1$H-NMR(DMSO-$d_6$)δ:8.37(1H,d,J=2.1Hz),8.04(1H,d,J=8.4Hz),7.10(1H,d,J=2.1Hz),7.08(1H,d,J=8.4Hz),3.77-3.24(6H,m),2.90(3H,s),2.19-1.43(12H,m).
MSm/z(M+H):474.

<Example 0110>

<0110-1>

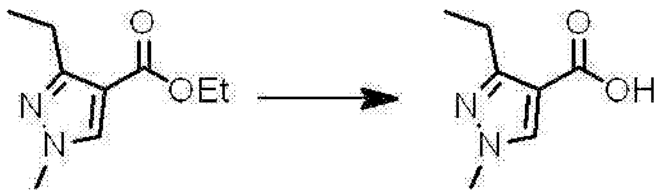

A4 mol/L sodium hydroxide aqueous solution (2 mL) was added to a solution of ethyl 3-ethyl-1-methyl-1H-pyrazole-4-carboxylate (100 mg) in ethanol (2 mL), followed by stirring at room temperature for 3 hours, and stirring at 70°C for 2 hours. After the reaction mixture was cooled to room temperature, the solvent was distilled off under reduced pressure, the resultant product was adjusted to pH 2 by the addition of 2 mol/L hydrochloric acid, and ethyl acetate was added thereto. The organic layer was collected by separation, washed with a saturated sodium chloride aqueous solution, and dried over anhydrous sodium sulfate. The solvent was distilled off under reduced pressure, thereby obtaining 3-ethyl-1-methyl-1H-pyrazole-4-carboxylic acid (71 mg).
MSm/z(M+H):155.

<0110-2>

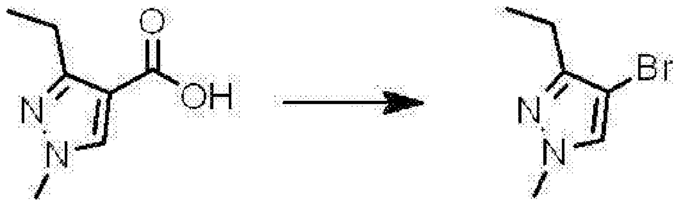

N-bromosuccinimide (90 mg) was added to a mixture of 3-ethyl-1-methyl-1H-pyrazole-4-carboxylic acid (71 mg), and sodium hydrogen carbonate (132 mg) in N,N-dimethylformamide (2.3 mL), followed by stirring at room temperature for 16 hours. Ethyl acetate and water were added to the reaction mixture. The organic layer was collected by separation, washed with a saturated sodium chloride aqueous solution, and dried over anhydrous sodium sulfate, and the solvent was distilled off under reduced pressure, thereby obtaining 4-bromo-3-ethyl-1-methyl-1H-pyrazole (74 mg).
MSm/z(M+H):189.

<0110-3>

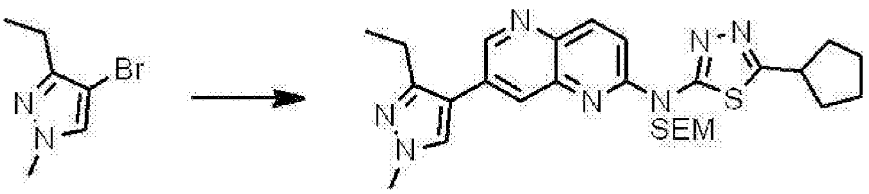

A mixture of N-(7-bromo-1,5-naphthyridin-2-yl)-5-cyclopentyl-N-((2-(trimethylsilyl)ethoxy)methyl)-1,3,4-thiadiazole-2-amine) (20 mg), bis(pinacolato)diboron (15 mg), (1,1'-bis(diphenylphosphino)ferrocene)palladium(II) dichloride (3 mg), and potassium acetate (8 mg) in 1,4-dioxane (0.8 mL) was stirred at 100°C for 2 hours in a nitrogen atmosphere. 4-Bromo-3-ethyl-1-methyl-1H-pyrazole (14 mg), sodium carbonate (8 mg), and bis(di-tert-butyl (4-dimethylaminophenyl)phosphine)dichloropalladium(II) (5 mg) were added to the reaction mixture, followed by reacting at 100°C for 5 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining 5-cyclopentyl-N-(7-(3-ethyl-1-methyl-1H-pyrazol-4-yl)-1,5-naphthyridin-2-yl)-N-((2-(trimethylsilyl)ethoxy)methyl)-1,3,4-thiadiazole-2-amine (33 mg).
MSm/z(M+H):536.

<0110-4>

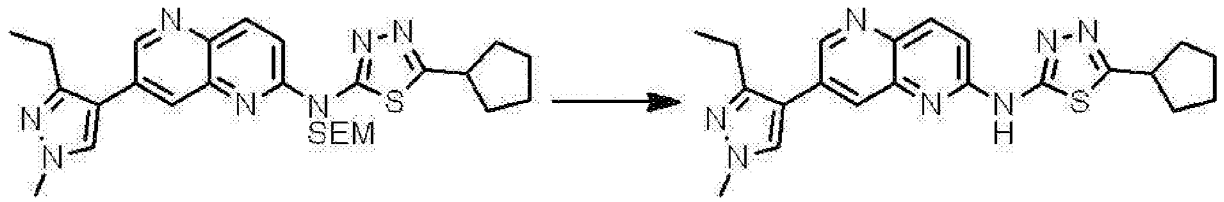

Water (0.1 mL) and trifluoroacetic acid (2 mL) were added to 5-cyclopentyl-N-(7-(3-ethyl-1-methyl-1H-pyrazol-4-yl)-1,5-naphthyridin-2-yl)-N-((2-(trimethylsilyl)ethoxy)methyl)-1,3,4-thiadiazole-2-amine (33 mg), followed by stirring at room temperature for 4 hours. The solvent was distilled off under reduced pressure, and the obtained residue was purified by silica gel column chromatography (methanol-ethyl acetate, NH silica), thereby obtaining 5-cyclopentyl-N-(7-(3-ethyl-1-methyl-1H-pyrazol-4-yl)-1,5-naphthyridin-2-yl)-1,3,4-thiadiazole-2-amine (10 mg).
$^1$H-NMR($CDCl_3$/$CD_3OD$=4/1)$\delta$:8.78(1H,d,J=2.1Hz),8.24(1H,d,J=9.0Hz),8.18(1H,d,J=2.1Hz),7.70(1H,s), 7.36(1H,d,J=9.0Hz),3.54-3.42(1H,m),3.37(3H,s),2.89(2H,q,7.8Hz),2.30-2.18(2H,m),1.94-1.72(6H,m).1.31 (3H,t,J=7.8Hz).
MSm/z(M+H):406.

<Example 0111>

<0111-1>

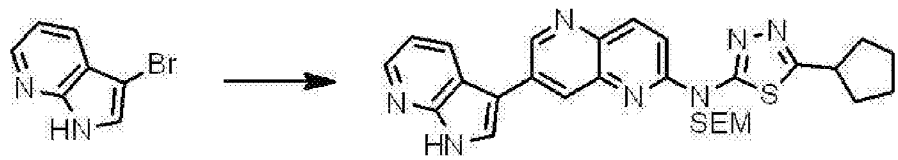

A mixture ofN-(7-bromo-1,5-naphthyridin-2-yl)-5-cyclopentyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II) dichloride (3 mg), and potassium acetate (8 mg) in 1,4-dioxane (0.8 mL) was stirred at 100°C for 2 hours in a nitrogen atmosphere. 3-Bromo-1H-pyrrolo[2,3-b]pyridine (15 mg), sodium carbonate (8 mg), and bis(di-tert-butyl (4-dimethylaminophenyl)phosphine)dichloropalladium(II) (5 mg) were added to the reaction mixture, followed by reacting at 100°C for 3 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained residue was purified by silica gel column chromatography (methanol-ethyl acetate-hexane), thereby obtaining N-(7-(1H-pyrrolo[2,3-b]pyridin-3-yl)-1,5-naphthyridin-2-yl)-5-cyclopentyl-N-((2-(trimethylsilyl)ethoxy)methyl)-1,3,4-thiadiazole-2-amine (3.5 mg).
MSm/z(M+H):544.

<0111-2>

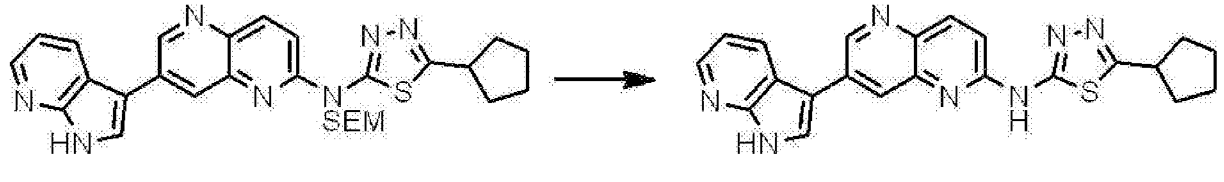

Water (0.1 mL) and trifluoroacetic acid (2 mL) were added to N-(7-(1H-pyrrolo[2,3-b]pyridin-3-yl)-1,5-naphthyridin-2-yl)-5-cyclopentyl-N-((2-(trimethylsilyl)ethoxy)methyl)-1,3,4-thiadiazole-2-amine (3.5 mg), followed by stirring at room temperature for 12 hours. The solvent was distilled off under reduced pressure, and the obtained residue was purified by silica gel column chromatography (methanol-ethyl acetate, NH silica), thereby obtaining N-(7-(1H-pyrrolo[2,3-b]pyridin-3-yl)-1,5-naphthyridin-2-yl)-5-cyclopentyl-1,3,4-thiadiazole-2-amine (3.3 mg).
$^1$H-NMR($CDCl_3$/$CD_3OD$=4/1)δ:9.07(1H,d,J=2.1Hz),8.46-8.34(3H,m),8.26(1H,d,J=9.0Hz),7.84(1H,s),7.35(1H,d,J=9.0Hz),7.33-7.27(1H,m),3.54-3.42(1H,m),2.30-2.18(2H,m),1.94-1.72(6H,m).
MSm/z(M+H):414.

<Example 0112>

<0112-1>

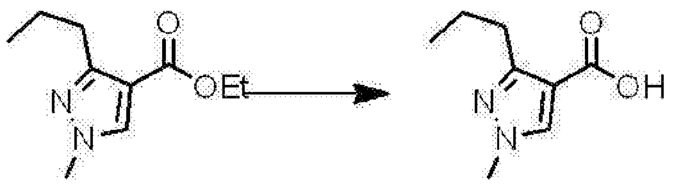

A4 mol/L sodium hydroxide aqueous solution (4 mL) was added to a solution of ethyl 1-methyl-3-propyl-1H-pyrazole-4-carboxylate (684 mg) in ethanol (4 mL), followed by stirring at 70°C for 4 hours. The solvent was distilled off under reduced pressure, the resultant product was adjusted to pH 2 by the addition of a 3 mol/L potassium hydrogen sulfate aqueous solution, and ethyl acetate was added thereto. The organic layer was collected by separation, washed with a saturated sodium chloride aqueous solution, and dried over anhydrous sodium sulfate, and the solvent was distilled off under reduced pressure, thereby obtaining 1-methyl-3-propyl-1H-pyrazole-4-carboxylic acid (506 mg).
MSm/z(M+H):169.

<0112-2>

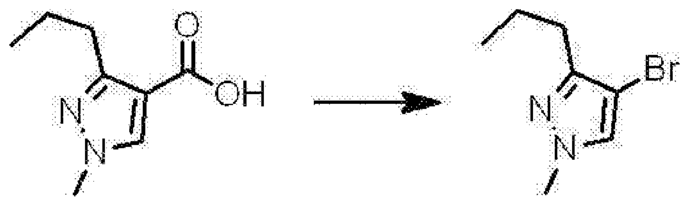

N-bromosuccinimide (589 mg) was added to a mixture of 1-methyl-3-propyl-1H-pyrazole-4-carboxylic acid (506 mg), and sodium hydrogen carbonate (859 mg) in N,N-dimethylformamide (10 mL), followed by stirring at room temperature for 18 hours. Ethyl acetate and water were added to the reaction mixture. The organic layer was collected by separation, washed with a saturated sodium chloride aqueous solution, and dried over anhydrous sodium sulfate, and the solvent was distilled off under reduced pressure, thereby obtaining 4-bromo-1-methyl-3-propyl-1H-pyrazole (535 mg).
MSm/z(M+H):203.

<0112-3>

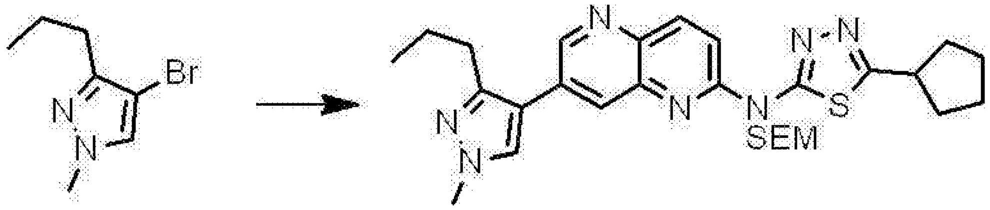

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II) dichloride (3 mg), and potassium acetate (8 mg) in 1,4-dioxane (0.8 mL) was stirred at 100°C for 2 hours in a nitrogen atmosphere. 4-Bromo-1-methyl-3-propyl-1H-pyrazole (15 mg), sodium carbonate (8 mg), and bis(di-tert-butyl (4-dimethylaminophenyl)phosphine)dichloropalladium(II) (5 mg) were added to the reaction mixture, followed by reacting at 100°C for 5 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining 5-cyclopentyl-N-(7-(1-methyl-3-propyl-1H-pyrazol-4-yl)-1,5-naphthyridin-2-yl)-N-((2-(trimethylsilyl)ethoxy)methyl)-1,3,4-thiadiazole-2-amine (30 mg).
MSm/z(M+H):550.

<0112-4>

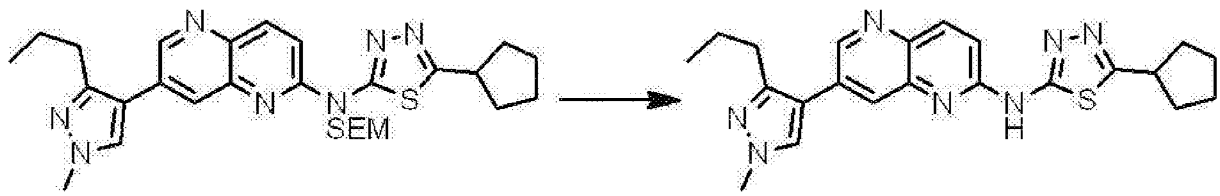

Water (0.1 mL) and trifluoroacetic acid (2 mL) were added to 5-cyclopentyl-N-(7-(1-methyl-3-propyl-1H-pyrazol-4-yl)-1,5-naphthyridin-2-yl)-N-((2-(trimethylsilyl)ethoxy)methyl)-1,3,4-thiadiazole-2-amine (30 mg), followed by stirring at room temperature for 1 hour. The solvent was distilled off under reduced pressure, and the obtained residue was purified by silica gel column chromatography (methanol-ethyl acetate, NH silica), thereby obtaining 5-cyclopentyl-N-(7-(1-methyl-3-propyl-1H-pyrazol-4-yl)-1,5-naphthyridin-2-yl)-1,3,4-thiadiazole-2-amine (8.7 mg).
$^1$H-NMR($CDCl_3$/$CD_3OD$=4/1)δ:8.78(1H,d,J=2.1Hz),8.23(1H,d,J=8.7Hz),8.19(1H,d,J=2.1Hz),7.73(1H,s), 7.35(1H,d,J=8.7Hz),3.95(3H,s),3.56-3.46(1H,m),2.87-2.80(2H,m),2.33-2.22(4H,m),1.98-1.66(9H,m).
MSm/z(M+H):420.

<Example 0113>

<0113-1>

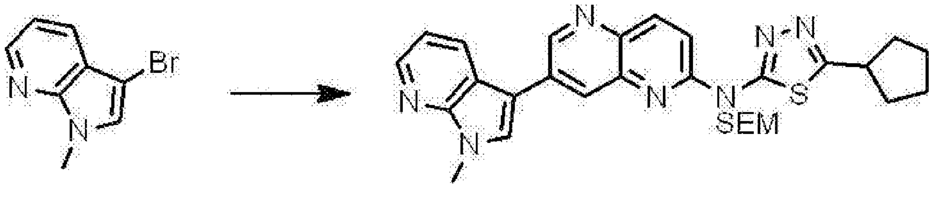

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium (II) dichloride (3 mg), and potassium acetate (8 mg) in 1,4-dioxane (0.8 mL) was stirred at 100°C for 2 hours in a nitrogen atmosphere. 3-Bromo-1-methyl-1H-pyrrolo[2,3-b]pyridine (15 mg), sodium carbonate (8 mg), and bis(di-tert-butyl (4-dimethylaminophenyl)phosphine)dichloropalladium(II) (5 mg) were added to the reaction mixture, followed by reacting at 100°C for 3 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining 5-cyclopentyl-N-(7-(1-methyl-1H-pyrrolo[2,3-b]pyridin-3-yl)-1,5-naphthyridin-2-yl)-N-((2-(trimethylsilyl)ethoxy)methyl)-1,3,4-thiadiazole-2-amine (51 mg).
MSm/z(M+H):558.

<0113-2>

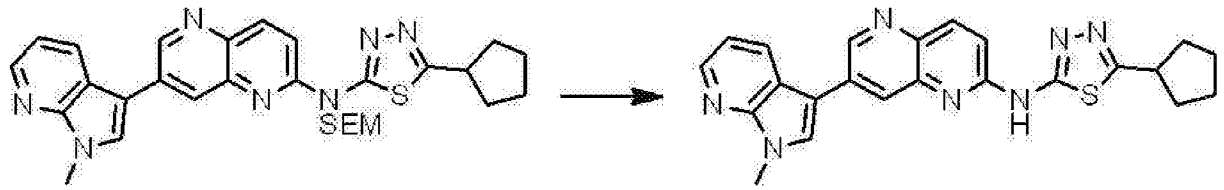

Water (0.1 mL) and trifluoroacetic acid (2 mL) were added to 5-cyclopentyl-N-(7-(1-methyl-1H-pyrrolo[2,3-b]pyridin-3-yl)-1,5-naphthyridin-2-yl)-N-((2-(trimethylsilyl)ethoxy)methyl)-1,3,4-thiadiazole-2-amine (51 mg), followed by stirring at room temperature for 15 hours. The solvent was distilled off under reduced pressure, and the obtained residue was purified by silica gel column chromatography (methanol-ethyl acetate, NH silica), thereby obtaining 5-cyclopentyl-N-(7-(1-methyl-1H-pyrrolo[2,3-b]pyridin-3-yl)-1,5-naphthyridin-2-yl)-1,3,4-thiadiazole-2-amine(2.8 mg).
$^1H$-NMR($CDCl_3/CD_3OD$=4/1)δ:9.05(1H,d,J=2.1Hz),8.45-8.23(4H,m),7.76(1H,s),7.37-7.22(2H,m),4.03(1H,s),3.54-3.42(1H,m),2.30-2.18(2H,m),1.94-1.72(6H,m).
MSm/z(M+H):428.

<Example 0114>

<0114-1>

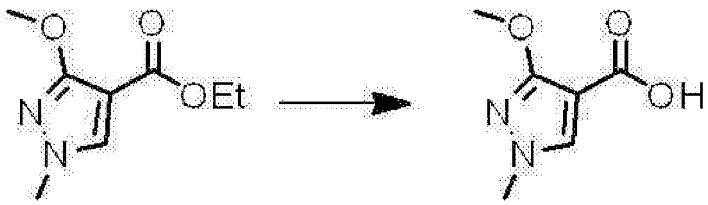

A4 mol/L sodium hydroxide aqueous solution (4 mL) was added to a solution of ethyl 3-methoxy-1-methyl-1H-pyrazole-4-carboxylate (72 mg) in ethanol (4 mL), followed by stirring at 70°C for 3 hours. The solvent was distilled off under reduced pressure, the resultant product was adjusted to pH 2 by the addition of a 3 mol/L potassium hydrogen sulfate aqueous solution, and ethyl acetate was added thereto. The organic layer was collected by separation, washed with a saturated sodium chloride aqueous solution, and dried over anhydrous sodium sulfate, and the solvent was distilled off under reduced pressure, thereby obtaining 3-methoxy-1-methyl-1H-pyrazole-4-carboxylic acid (55 mg).
MSm/z(M+H):157.

<0114-2>

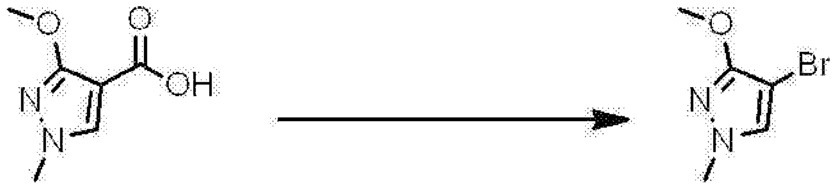

N-bromosuccinimide (70 mg) was added to a mixture of 3-methoxy-1-methyl-1H-pyrazole-4-carboxylic acid (55 mg), and sodium hydrogen carbonate (101 mg) in N,N-dimethylformamide (2 mL), followed by stirring at room temperature for 1 day. Ethyl acetate and water were added to the reaction mixture. The organic layer was collected by separation, washed with a saturated sodium chloride aqueous solution, and dried over anhydrous sodium sulfate, and the solvent was distilled off under reduced pressure, thereby obtaining 4-bromo-3-methoxy-1-methyl-1H-pyrazole (53 mg).
MSm/z(M+H):191.

<0114-3>

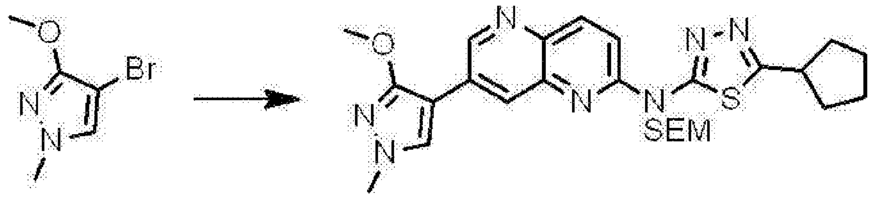

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II)dichloride (3 mg), and potassium acetate (8 mg) in 1,4-dioxane (0.8 mL) was stirred at 100°C for 2 hours in a nitrogen atmosphere. 4-Bromo-3-methoxy-1-methyl-1H-pyrazole (15 mg), sodium carbonate (8 mg), and bis(di-tert-butyl (4-dimethylaminophenyl)phosphine)dichloropalladium(II) (5 mg) were added to the reaction mixture, followed by reacting at 100°C for 3 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining 5-cyclopentyl-N-(7-(3-methoxy-1-methyl-1H-pyrazol-4-yl)-1,5-naphthyridin-2-yl)-N-((2-(trimethylsilyl)ethoxy)methyl)-1,3,4-thiadiazole-2-amine (46 mg).
MSm/z(M+H):538.

<0114-4>

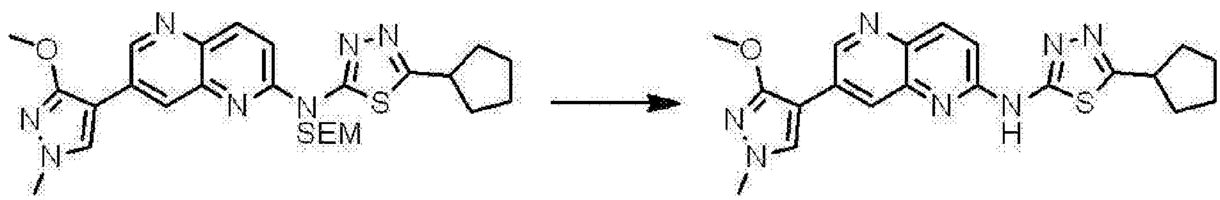

Water (0.1 mL) and trifluoroacetic acid (2 mL) were added to 5-cyclopentyl-N-(7-(3-methoxy-1-methyl-1H-pyrazol-4-yl)-1,5-naphthyridin-2-yl)-N-((2-(trimethylsilyl)ethoxy)methyl)-1,3,4-thiadiazole-2-amine (46 mg), followed by stirring at room temperature for 15 hours. The solvent was distilled off under reduced pressure, and the obtained residue was purified by silica gel column chromatography (methanol-ethyl acetate, NH silica), thereby obtaining 5-cyclopentyl-N-(7-(3-methoxy-1-methyl-1H-pyrazol-4-yl)-1,5-naphthyridin-2-yl)-1,3,4-thiadiazole-2-amine (1.8 mg).
$^1$H-NMR($CDCl_3$/$CD_3OD$=4/1)δ:8.60(1H,brs),8.40(1H,brs),8.33(1H,d,J=9.0Hz),7.81(1H,s),7.27(1H,d,J=9.

0Hz),4.16(3H,s),3.87(3H,s),3.54-3.42(1H,m),2.30-2.18(2H,m),1.94-1.72(6H,m).
MSm/z(M+H):408.

<Example 0115>

<0115-1>

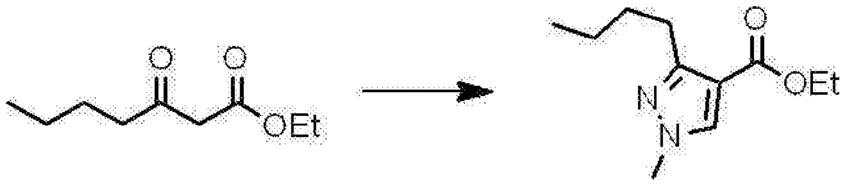

A solution of methylhydrazine (0.37 mL) and ethyl formate (0.90 mL) in ethanol (3.5 mL) was stirred for 4 hours under heating to reflux, and ethyl 3-oxoheptanoate (2.4 mL) was added thereto, followed by stirring at the same temperature for 4 hours. The reaction mixture was cooled to room temperature, and a 20% sodium ethoxide-ethanol solution (3.5 mL) was added thereto, followed by stirring for 1.5 hours under heating to reflux. The reaction mixture was cooled to room temperature, and ethyl acetate and a 3 mol/L potassium hydrogen sulfate aqueous solution were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining ethyl 3-butyl-1-methyl-1H-pyrazole-4-carboxylate (669 mg).
MSm/z(M+H):211.

<0115-2>

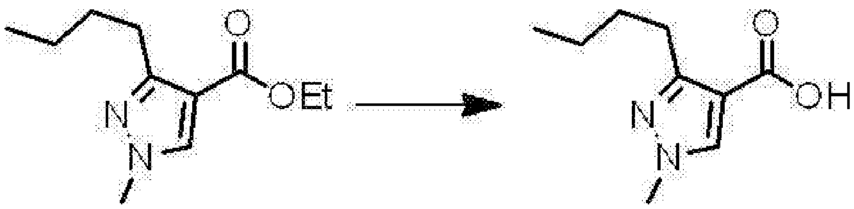

A4 mol/L sodium hydroxide aqueous solution (4 mL) was added to a solution of ethyl 3-butyl-1-methyl-1H-pyrazole-4-carboxylate (669 mg) in ethanol (4 mL), followed by stirring at 70°C for 3 hours. The solvent was distilled off under reduced pressure, the resultant product was adjusted to pH 2 by the addition of a 3 mol/L potassium hydrogen sulfate aqueous solution, and ethyl acetate was added thereto. The organic layer was collected by separation, washed with a saturated sodium chloride aqueous solution, and dried over anhydrous sodium sulfate, and the solvent was distilled off under reduced pressure, thereby obtaining 3-butyl-1-methyl-1H-pyrazole-4-carboxylic acid (513 mg).
MSm/z(M+H):183.

<0115-3>

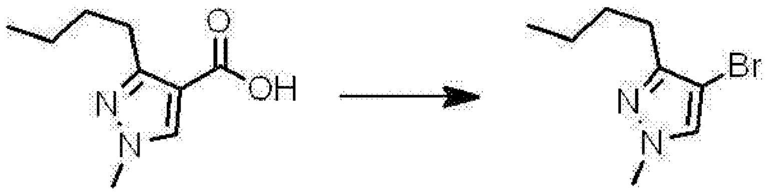

N-bromosuccinimide (550 mg) was added to a mixture of 3-butyl-1-methyl-1H-pyrazole-4-carboxylic acid (513 mg), and sodium hydrogen carbonate (802 mg) in N,N-dimethylformamide (10 mL), followed by stirring at room temperature for 4 hours. Ethyl acetate and water were added to the reaction mixture. The organic layer was collected by separation, washed with a saturated sodium chloride aqueous solution, and dried over anhydrous sodium sulfate, and the solvent was distilled off under reduced pressure, thereby obtaining 4-bromo-3-butyl-1-methyl-1H-pyrazole (606 mg).
MSm/z(M+H):217.

<0115-4>

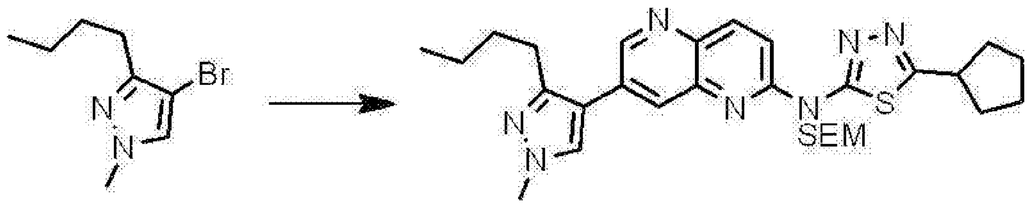

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II)dichloride (3 mg), and potassium acetate (8 mg) in 1,4-dioxane (0.8 mL) was stirred at 100°C for 2.5 hours in a nitrogen atmosphere. 4-Bromo-3-butyl-1-methyl-1H-pyrazole (15 mg), sodium carbonate (8 mg), and bis(di-tert-butyl (4-dimethylaminophenyl)phosphine)dichloropalladium(II) (5 mg) were added to the reaction mixture, followed by stirring at 100°C for 2 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining N-(7-(3-butyl-1-methyl-1H-pyrazol-4-yl)-1,5-naphthyridin-2-yl)-5-cyclopentyl-N-((2-(trimethylsilyl)ethoxy)methyl)-1,3,4-thiadiazole-2-amine (5.4 mg).
MSm/z(M+H):564.

<0115-5>

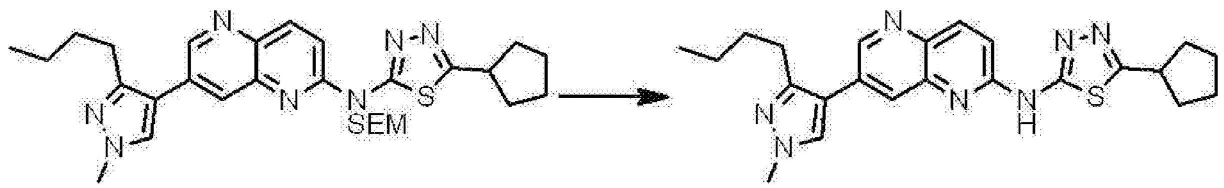

Water (0.1 mL) and trifluoroacetic acid (2 mL) were added to N-(7-(3-butyl-1-methyl-1H-pyrazol-4-yl)-1,5-naphthyridin-2-yl)-5-cyclopentyl-N-((2-(trimethylsilyl)ethoxy)methyl)-1,3,4-thiadiazole-2-amine (5.4 mg), followed by stirring at room temperature for 30 minutes. The solvent was distilled off under reduced pressure, and the obtained residue was purified by silica gel column chromatography (methanol-ethyl acetate, NH silica), thereby obtaining N-(7-(3-butyl-1-methyl-1H-pyrazol-4-yl)-1,5-naphthyridin-2-yl)-5-cyclopentyl-1,3,4-thiadiazole-2-amine (1.9 mg).
$^1$H-NMR($CDCl_3$/$CD_3OD$=4/1)δ:8.78(1H,brs),8.24(1H,d,J=9.0Hz),8.20(1H,brs),7.73(1H,s),7.35(1H,d,J=9.0Hz),3.95(3H,s),3.57-3.42(1H,m),2.90-2.82(2H,m),2.33-2.21(2H,m),1.96-1.63(8H,m),1.52-1.48(2H,m),0.95(3H,t,J=7.2 Hz).
MSm/z(M+H):434.

<Example 0116>

<0116-1>

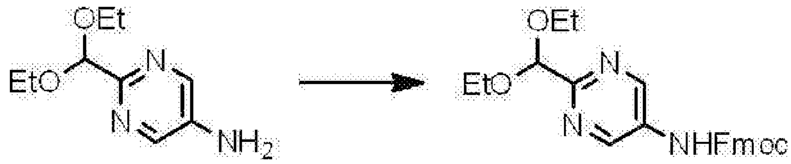

A solution of (9H-fluoren-9-ylmethoxy)carbonyl chloride (826 mg) in 1,4-dioxane (4 mL) was added to a mixture of 2-(diethoxymethyl)pyrimidine-5-amine (629 mg), and sodium hydrogen carbonate (800 mg) in 1,4-dioxane (4 mL), and water (4 mL) at room temperature, followed by stirring at the same temperature for 3 hours. Ethyl acetate and water were added to the reaction mixture. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining 9H-fluoren-9-yl)methyl (2-(diethoxymethyl)pyrimidin-5-yl)carbamate (810 mg).
MSm/z(M+H):420.

<0116-2>

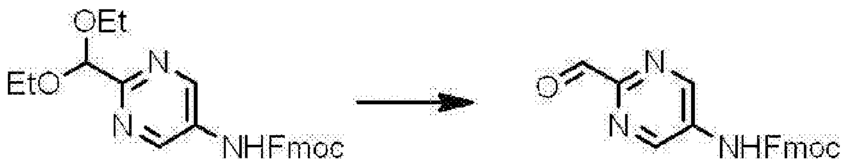

1 mol/L hydrochloric acid was added to a solution of (9H-fluoren-9-yl)methyl (2-(diethoxymethyl)pyrimidin-5-yl)carbamate (810 mg) in acetone (2.4 mL), followed by stirring at room temperature for 2 hours, and (+)-10-camphorsulfonic acid (66 mg) was added thereto, followed by stirring at the same temperature for 1 hour. Ethyl acetate and water were added to the reaction mixture. The organic layer was collected by separation, washed with a saturated sodium chloride aqueous solution, and dried over anhydrous sodium sulfate, and the solvent was distilled off under reduced pressure, thereby obtaining (9H-fluoren-9-yl)methyl (2-formylpyrimidin-5-yl)carbamate (99 mg).
MSm/z(M+H):346.

<0116-3>

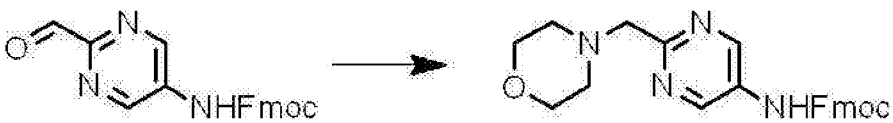

Morpholine (0.037 mL), sodium triacetoxyborohydride (150 mg), and acetic acid (0.024 mL) were added to a solution of (9H-fluoren-9-yl)methyl (2-formylpyrimidin-5-yl)carbamate (99 mg) in dichloromethane (2.8 mL), followed by stirring at room temperature for 1 hour. Ethyl acetate and a saturated sodium hydrogen carbonate aqueous solution were added to the reaction mixture. The organic layer was collected by separation, washed with a saturated sodium chloride aqueous solution, and dried over anhydrous sodium sulfate, and the solvent was distilled off under reduced pressure. The obtained residue was purified by silica gel column chromatography (methanol-ethyl acetate), thereby obtaining (9H-fluoren-9-yl)methyl (2-(morpholinomethyl)pyrimidin-5-yl)carbamate (89 mg).
MSm/z(M+H):417.

<0116-4>

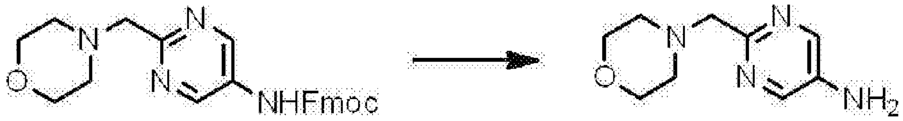

Diethylamine (1 mL) was added to a solution of (9H-fluoren-9-yl)methyl (2-(morpholinomethyl)pyrimidin-5-yl)carbamate (89 mg) in dichloromethane (2 mL), followed by stirring at room temperature for 14 hours. The solvent was distilled off under reduced pressure, and the obtained residue was purified by silica gel column chromatography (methanol-ethyl acetate, NH silica), thereby obtaining 2-(morpholinomethyl)pyrimidine-5-amine (30 mg).
MSm/z(M+H):195.

<0116-5>

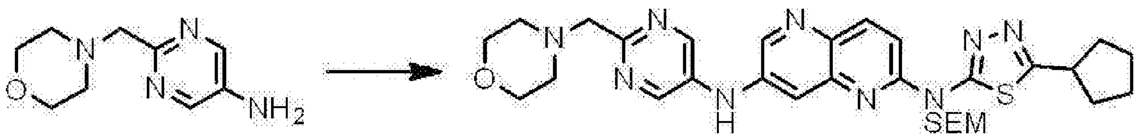

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), 2-(morpholinomethyl)pyrimidine-5-amine (15 mg), tris(dibenzylideneacetone)dipalladium(0) (6 mg), 2-(dicyclohexylphosphino)-2',4',6'-triisopropylbiphenyl (7 mg), and sodium tert-butoxide (26 mg) in 1,4-dioxane (1 mL) was stirred at 100°C for 5 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining $N^2$-(5-cyclopentyl-1,3,4-thiadiazol-2-yl)-$N^7$-(2-(morpholinomethyl)pyrimidin-5-yl)-$N^2$-((2-(trimethylsilyl)ethoxy)methyl)-1,5-naphthyridine-2,7-diamine (38 mg).
MSm/z(M+H):620.

<0116-6>

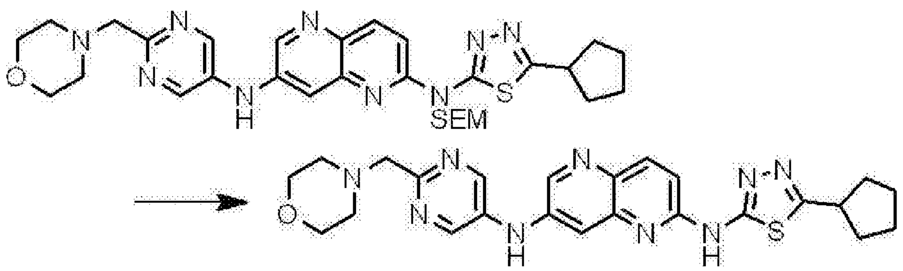

Water (0.1 mL) and trifluoroacetic acid (2 mL) were added to $N^2$-(5-cyclopentyl-1,3,4-thiadiazol-2-yl)-$N^7$-(2-(morpholinomethyl)pyrimidin-5-yl)-$N^2$-((2-(trimethylsilyl)ethoxy)methyl)-1,5-naphthyridine-2,7-diamine (38 mg), followed by stirring at room temperature for 30 minutes. The solvent was distilled off under reduced pressure, and the obtained residue was purified by silica gel column chromatography (methanol-ethyl acetate, NH silica), thereby obtaining $N^2$-(5-cyclopentyl-1,3,4-thiadiazol-2-yl)-$N^7$-(2-(morpholinomethyl)pyrimidin-5-yl)-1,5-naphthyridine-2,7-diamine (4.2 mg).
$^1$H-NMR($CDCl_3$/$CD_3OD$=4/1)δ:8.77(2H,s),8.49(1H,brs),8.12(1H,d,J=9.0Hz),7.85(1H,m),7.19(1H,d,J=9.0 Hz),3.84-3.78(4H,m),3.39-3.34(2H,s),2.69-2.63(4H,m),3.54-3.42(1H,m),2.30-2.18(2H,m),1.94-1.72(6H,m).
MSm/z(M+H):490.

<Example 0117>

<0117-1>

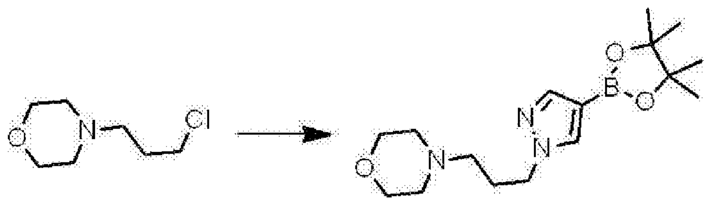

A mixture of 4-(3-chloropropyl)morpholine (648 mg), 4-(4,4,5,5-tetramethyl-1,3,2-dioxaborolan-2-yl)-1H-pyrazole (500 mg), cesium carbonate (837 mg), and sodium iodide (77 mg) in acetonitrile (3 mL) and tetrahydrofuran (1 mL) was stirred at 80°C for 18 hours. The reaction mixture was cooled to room temperature, and the solvent was distilled off under reduced pressure. The obtained residue was purified by silica gel column chromatography (methanol-ethyl acetate, NH silica), thereby obtaining 4-(3-(4-(4,4,5,5-tetramethyl-1,3,2-dioxaborolan-2-yl)-1H-pyrazol-1-yl)propyl)morpholine (485 mg).
$^1$H-NMR($CDCl_3$)$\delta$:7.78(1H,s),7.68(1H,s),4.19(2H,t,J=6.6Hz),3.76-3.66(4H,m),3.70(2H,t,J=4.8Hz),2.45-2.35(4H,m),2.10-1.98(2H,m),1.31(12H,s).

<0117-2>

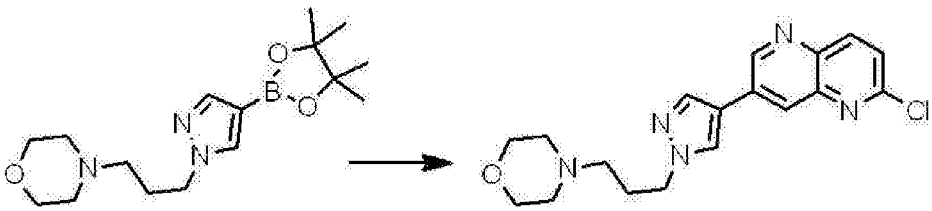

A mixture of 7-bromo-2-chloro-1,5-naphthyridine (188 mg), 4-(3-(4-(4,4,5,5-tetramethyl-1,3,2-dioxaborolan-2-yl)-1H-pyrazol-1-yl)propyl)morpholine (297 mg), sodium carbonate (204 mg), bis(di-tert-butyl (4-dimethylaminophenyl)phosphine)dichloropalladium(II) (55 mg), and water (0.8 mL) in 1,4-dioxane (8 mL) was stirred at 100°C for 2 hours. The reaction mixture was cooled to room temperature, the insolubles were filtered off using celite, and the solvent was distilled off under reduced pressure. The obtained residue was purified by silica gel column chromatography (methanol-ethyl acetate), thereby obtaining 4-(3-(4-(6-chloro-1,5-naphthyridin-3-yl)-1H-pyrazol-1-yl)propyl)morpholine (211 mg).
MSm/z(M+H):358.

<0117-3>

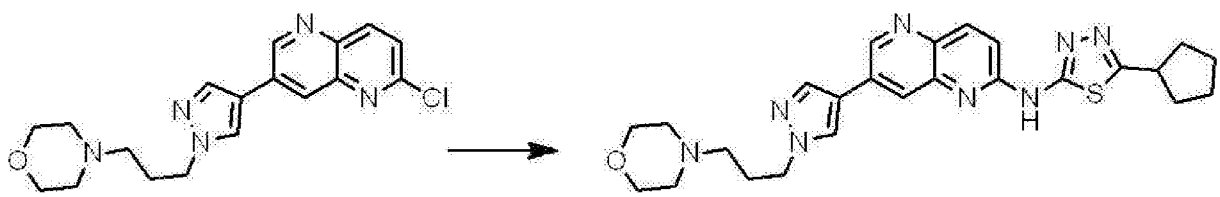

A mixture of 4-(3-(4-(6-chloro-1,5-naphthyridin-3-yl)-1H-pyrazol-1-yl)propyl)morpholine (20 mg), 5-cyclopentyl-1,3,4-thiadiazole-2-amine (14 mg), tris(dibenzylideneacetone)dipalladium(0) (5 mg), 4,5-bis(diphenylphosphino)-9,9-dimethylxanthene (6 mg), and cesium carbonate (45 mg) in 1,4-dioxane (1 mL) was stirred at 150°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining 5-cyclopentyl-N-(7-(1-(3-morpholinopropyl)-1H-pyrazol-4-yl)-1,5-naphthyridin-2-yl)-1,3,4-thiadiazole-2-amine (1.5 mg).
$^1$H-NMR($CDCl_3$/$CD_3OD$=4/1)δ:8.90(1H,d,J=2.1Hz),8.27(1H,d,2.1Hz),8.21(1H,d,J=8.7Hz),8.02(2H,s),7.32(1H,d,J=8.7Hz),4.35-4.28(2H,m),3.78-3.74(4H,m),3.56-3.46(1H,m),2.52-2.46(4H,m),2.45-2.37(2H,m),2.34-2.23(2H,m),2.20-2.09(2H,m),1.98-1.75(6H,m).
MSm/z(M+H):491.

<Example 0118>

<0118-1>

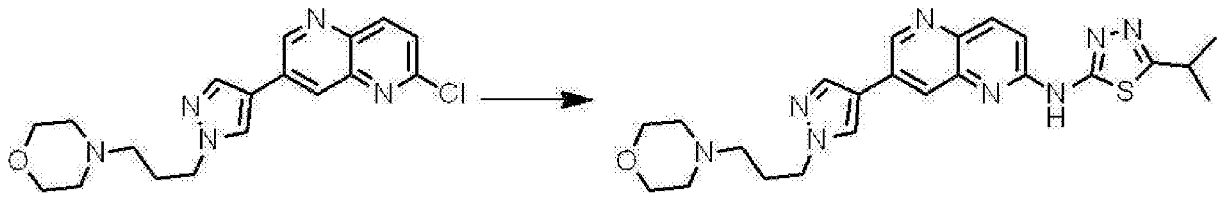

A mixture of 4-(3-(4-(6-chloro-1,5-naphthyridin-3-yl)-1H-pyrazol-1-yl)propyl)morpholine (20 mg), 5-isopropyl-1,3,4-thiadiazole-2-amine (12 mg), tris(dibenzylideneacetone)dipalladium(0) (5 mg), 4,5-bis(diphenylphosphino)-9,9-dimethylxanthene (6 mg), and cesium carbonate (45 mg) in 1,4-dioxane (1 mL) was stirred at 150°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining 5-isopropyl-N-(7-(1-(3-morpholinopropyl)-1H-pyrazol-4-yl)-1,5-naphthyridin-2-yl)-1,3,4-thiadiazole-2-amine (7.3 mg).
$^1$H-NMR($CDCl_3$/$CD_3OD$=4/1)δ:8.90(1H,d,J=2.1Hz),8.28(1H,d,J=2.1Hz),8.22(1H,d,J=9.0Hz),8.03(2H,s),7.34(1H,d,J=9.0Hz),4.34-4.27(2H,m),3.77-3.72(4H,m),3.51-3.39(1H,m),2.51-2.46(4H,m),2.44-2.37(2H,m),2.20-2.08(2H,m),1.52(6H,d,J=7.5Hz).
MSm/z(M+H):465.

<Example 0119>

<0119-1>

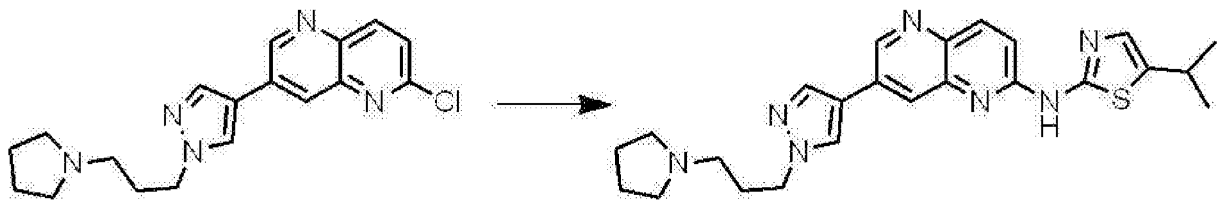

A mixture of 2-chloro-7-(1-(3-(pyrrolidin-1-yl)propyl)-1H-pyrazol-4-yl)-1,5-naphthyridine (20 mg), 5-isopropylthiazole-2-amine (12 mg), tris(dibenzylideneacetone)dipalladium(0) (5 mg), 4,5-bis(diphenylphosphino)-9,9-dimethylxanthene (7 mg), and cesium carbonate (48 mg) in 1,4-dioxane (1 mL) was stirred at 150°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining 5-isopropyl-N-(7-(1-(3-(pyrrolidin-1-yl)propyl)-1H-pyrazol-4-yl)-1,5-naphthyridin-2-yl)thiazole-2-amine (7.3 mg).
$^1$H-NMR($CDCl_3$/$CD_3OD$=4/1):8.86(1H,brs),8.29(1H,brs),8.15(1H,d,J=9.0Hz),8.04(1H,s),8.01(1H,s),7.26

(1H,d,J=9.0Hz),7.09(1H,s),4.33-4.26(2H,m),3.27-3.1S(1H,m),2.60-2.47(4H,m),2.23-2.11(2H,m),1.91-1.78(6H,m),1.42 (6H,d,J=6.3Hz).
MSm/z(M+H):448.

<Example 0120>

<0120-1>

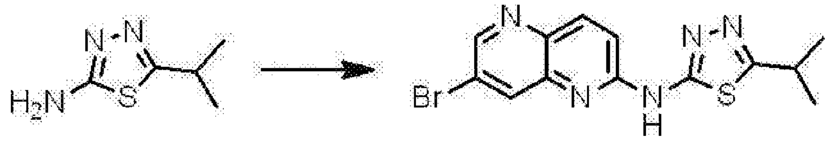

A mixture of 7-bromo-2-chloro-1,5-naphthyridine (2.01 g), 5-isopropyl-1,3,4-thiadiazole-2-amine (1.18 g), and potassium carbonate (1.71 g) in dimethylsulfoxide (16 mL) was stirred at 130°C for 2 hours. After the obtained reaction mixture was cooled to room temperature, the solid matter was collected by filtration, thereby obtaining N-(7-bromo-1,5-naphthyridin-2-yl)-5-isopropyl-1,3,4-thiadiazole-2-amine (2.21 g).
MSm/z(M+H):351.

<0120-2>

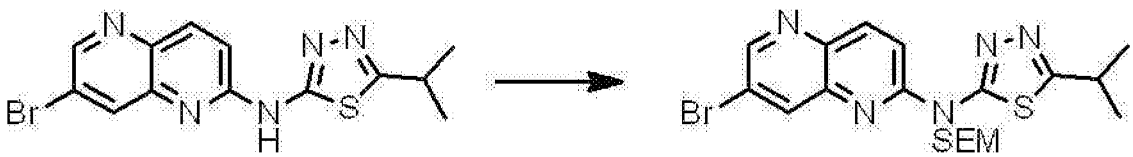

(2-(Trimethylsilyl)ethoxy)methyl chloride (1.66 mL) was added to a solution of N-(7-bromo-1,5-naphthyridin-2-yl)-5-isopropyl-1,3,4-thiadiazole-2-amine (2.21 g) in N-methylpyrrolidone (60 mL), followed by stirring for 15 minutes under ice-cooling, and 60% sodium hydride (505 mg) was added thereto, followed by stirring at the same temperature for 2 hours. Ethyl acetate and water were added to the reaction mixture. The organic layer was collected by separation, washed sequentially with water and a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining N-(7-bromo-1,5-naphthyridin-2-yl)-5-isopropyl-N-((2-(trimethylsilyl)ethoxy)methyl)-1,3,4-thiadiazole-2-amine (1.71 g).
MSm/z(M+H):480.

<0120-3>

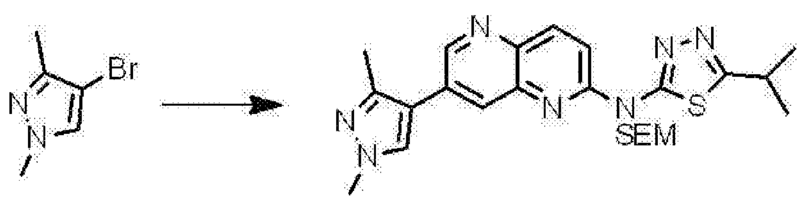

A mixture of N-(7-bromo-1,5-naphthyridin-2-yl)-5-isopropyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II)dichloride (3 mg), and potassium acetate (8 mg) in 1,4-dioxane (0.8 mL) was stirred at 100°C for 3 hours in a nitrogen atmosphere. 4-Bromo-1,3-dimethyl-1H-pyrazole (15 mg), sodium carbonate (8 mg), and bis(di-tert-butyl (4-dimethylaminophenyl)phosphine)dichloropalladium(II) (5 mg) were added to the reaction mixture, followed by stirring at 100°C for 2 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (methanol-ethyl acetate-hexane), thereby obtaining N-(7-(1,3-dimethyl-1H-pyrazol-4-yl)-1,5-naphthyridin-2-yl)-5-isopropyl-N-((2-(trimethylsilyl)ethoxy)methyl)-1,3,4-thiadiazole-2-amine (9.7 mg).
MSm/z(M+H):496.

<0120-4>

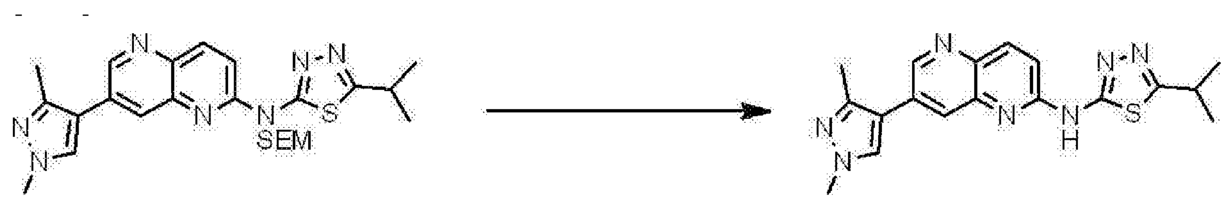

Water (0.1 mL) and trifluoroacetic acid (2 mL) were added to N-(7-(1,3-dimethyl-1H-pyrazol-4-yl)-1,5-naphthyridin-2-yl)-5-isopropyl-N-((2-(trimethylsilyl)ethoxy)methyl)-1,3,4-thiadiazole-2-amine (9.7 mg), followed by stirring at room temperature for 2 hours. The solvent was distilled off under reduced pressure, and the obtained residue was purified by silica gel column chromatography (methanol-ethyl acetate, NH silica), thereby obtaining N-(7-(1,3-dimethyl-1H-pyrazol-4-yl)-1,5-naphthyridin-2-yl)-5-isopropyl-1,3,4-thiadiazole-2-amine (4.3 mg).
$^1$H-NMR($CDCl_3$/$CD_3OD$=4/1)δ:8.79(1H,brs),8.24(1H,d,J=8.7Hz),8.21(1H,brs),7.76(1H,s),7.36(1H,d,J=8.7Hz),3.95(3H,s),3.49-3.37(1H,m),2.52(3H,s),1.50(6H,d,J=7.2Hz).
MSm/z(M+H):366.

<Example 0121>

<0121-1>

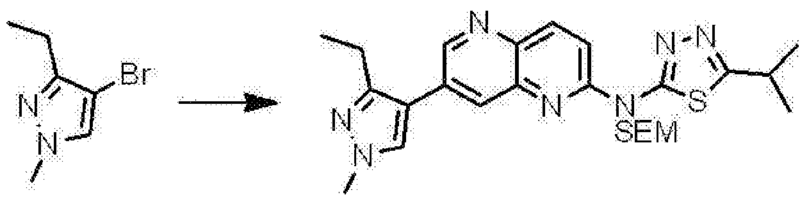

A mixture of N-(7-bromo-1,5-naphthyridin-2-yl)-5-isopropyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II)dichloride (3 mg), and potassium acetate (8 mg) in 1,4-dioxane (0.8 mL) was stirred at 100°C for 3 hours in a nitrogen atmosphere. 4-Bromo-3-ethyl-1-methyl-1H-pyrazole (15 mg), sodium carbonate (8 mg), and bis(di-tert-butyl (4-dimethylaminophenyl)phosphine)dichloropalladium(II) (5 mg) were added to the reaction mixture, followed by stirring at 100°C for 2 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (methanol-ethyl acetate-hexane), thereby obtaining N-(7-(3-ethyl-1-methyl-1H-pyrazol-4-yl)-1,5-naphthyridin-2-yl)-5-isopropyl-N-((2-(trimethylsilyl)ethoxy)methyl)-1,3,4-thiadiazole-2-amine (12 mg).
MSm/z(M+H):510.

<0121-2>

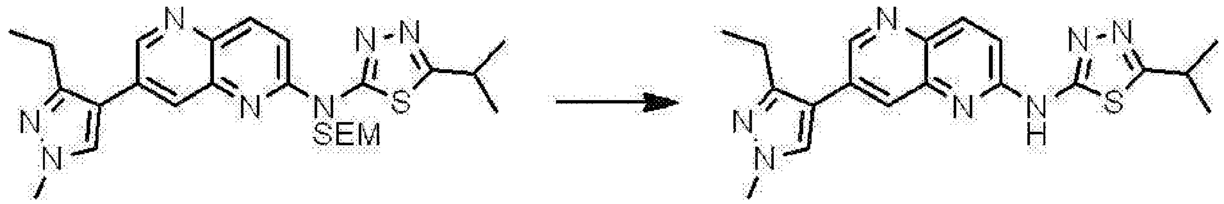

Water (0.1 mL) and trifluoroacetic acid (2 mL) were added to N-(7-(3-ethyl-1-methyl-1H-pyrazol-4-yl)-1,5-naphthyridin-2-yl)-5-isopropyl-N-((2-(trimethylsilyl)ethoxy)methyl)-1,3,4-thiadiazole-2-amine (12 mg), followed by stirring at room temperature for 2 hours. The solvent was distilled off under reduced pressure, and the obtained residue was purified by silica gel column chromatography (methanol-ethyl acetate, NH silica), thereby obtaining N-(7-(3-ethyl-1-methyl-1H-pyrazol-4-yl)-1,5-naphthyridin-2-yl)-5-isopropyl-1,3,4-thiadiazole-2-amine (5.5 mg).
$^1$H-NMR($CDCl_3$/$CD_3OD$=4/1):8.78(1H,d,J=2.1Hz),8.24(1H,d,J=8.7Hz),8.20(1H,d,J=2.1Hz),7.71(1H,s),7.37(1H,d,J=8.7Hz),3.96(3H,s),3.49-3.36(1H,m),2.90(2H,q,J=7.8Hz),1.50(6H,d,J=6.6Hz),1.31(3H,t,J=7.8Hz).
MSm/z(M+H):380.

<Example 0122>

<0122-1>

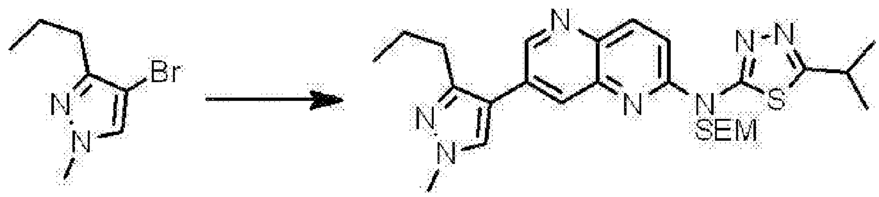

A mixture of N-(7-bromo-1,5-naphthyridin-2-yl)-5-isopropyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II) dichloride (3 mg), and potassium acetate (8 mg) in 1,4-dioxane (0.8 mL) was stirred at 100°C for 3 hours in a nitrogen atmosphere. 4-Bromo-1-methyl-3-propyl-1H-pyrazole (15 mg), sodium carbonate (8 mg), and bis(di-tert-butyl (4-dimethylaminophenyl)phosphine)dichloropalladium(II) (5 mg) were added to the reaction mixture, followed by stirring at 100°C for 2 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (methanol-ethyl acetate-hexane), thereby obtaining 5-isopropyl-N-(7-(1-methyl-3-propyl-1H-pyrazol-4-yl)-1,5-naphthyridin-2-yl)-N-((2-(trimethylsilyl)ethoxy)methyl)-1,3,4-thiadiazole-2-amine (7.4 mg).
MSm/z(M+H):524.

<0122-2>

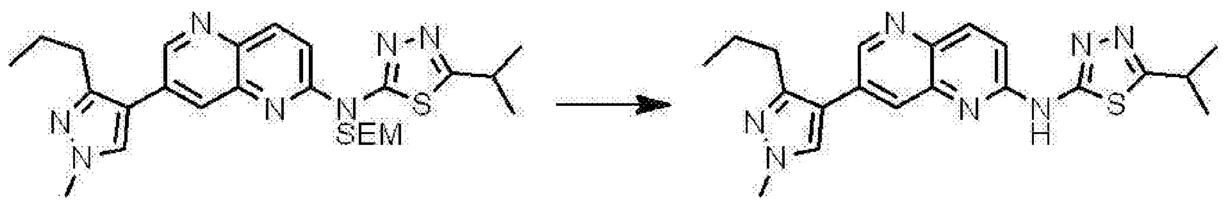

Water (0.1 mL) and trifluoroacetic acid (2 mL) were added to 5-isopropyl-N-(7-(1-methyl-3-propyl-1H-pyrazol-4-yl)-1,5-naphthyridin-2-yl)-N-((2-(trimethylsilyl)ethoxy)methyl)-1,3,4-thiadiazole-2-amine (7.4 mg), followed by stirring at room temperature for 2 hours. The solvent was distilled off under reduced pressure, and the obtained residue was

purified by silica gel column chromatography (methanol-ethyl acetate, NH silica), thereby obtaining 5-isopropyl-N-(7-(1-methyl-3-propyl-1H-pyrazol-4-yl)-1,5-naphthyridin-2-yl)-1,3,4-thiadiazole-2-amine (7.4 mg).
$^1$H-NMR($CDCl_3$/$CD_3OD$=4/1)δ:8.78(1H,brs),8.24(1H,d,J=9.0Hz),8.19(1H,brs),7.73(1H,s),7.35(1H,d,J=9.0Hz),3.95(3H,s),3.49-3.37(1H,m),2.88-2.80(2H,m),1.78-1.68(2H,m),1.50(6H,d,J=6.6Hz),1.06-0.97(3H,t,J=6.6Hz).
MSm/z(M+H):394.

<Example 0123>

<0123-1>

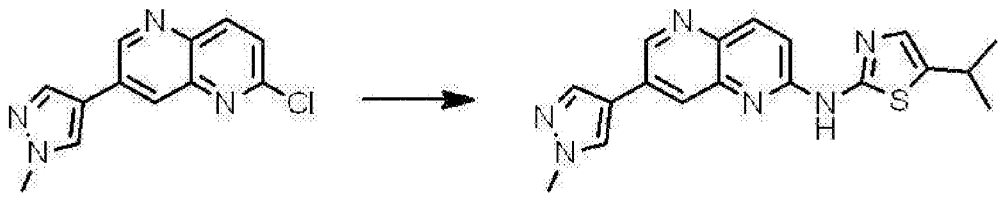

A mixture of 2-chloro-7-(1-methyl-1H-pyrazol-4-yl)-1,5-naphthyridine (14 mg), 5-isopropylthiazole-2-amine (10 mg), tris (dibenzylideneacetone)dipalladium(0) (5 mg), 4,5-bis(diphenylphosphino)-9,9-dimethylxanthene (7 mg), and cesium carbonate (48 mg) in 1,4-dioxane (1 mL) was stirred at 150°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining 5-isopropyl-N-(7-(1-methyl-1H-pyrazol-4-yl)-1,5-naphthyridin-2-yl)thiazole-2-amine (3.8 mg).
$^1$H-NMR($CDCl_3$/$CD_3OD$=4/1)δ:8.85(1H,brs),8.29(1H,brs),8.14(1H,d,J=8.7Hz),8.00(1H,s),7.98(1H,s),7.26(1H,d,J=8.7Hz),7.08(1H,s),3.36(3H,s),3.25-3.15(1H,m),1.42(6H,d,J=6.6Hz).
MSm/z(M+H):351.

<Example 0124>

<0124-1>

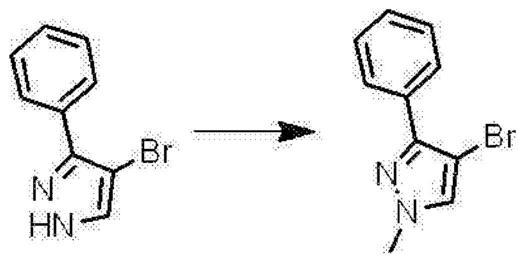

60% sodium hydride (516 mg) was added to a solution of 4-bromo-3-phenyl-1H-pyrazole (1.44 g) and iodomethane (0.80 mL) in N-methylpyrrolidone (13 mL) under ice-cooling, followed by stirring at the same temperature for 1.5 hours. Ethyl acetate and water were added to the reaction mixture at the same temperature. The organic layer was collected by separation, washed with a saturated sodium chloride aqueous solution, and dried over anhydrous sodium sulfate, and the solvent was distilled off under reduced pressure, thereby obtaining 4-bromo-1-methyl-3-phenyl-1H-pyrazole (811 mg).
MSm/z(M+H):237,239.

<0124-2>

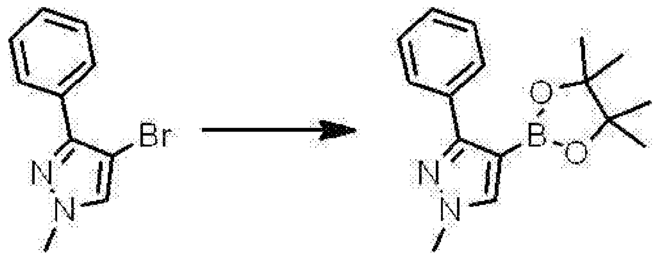

A 1.6 mol/L n-butyllithium-hexane solution (1.2 mL) was added to a solution of 4-bromo-1-methyl-3-phenyl-1H-pyrazole (300 mg) in tetrahydrofuran (6 mL) at -80°C, followed by stirring at the same temperature for 30 minutes, and 2-isopropyloxy-4,4,5,5-tetramethyl-1,3,2-dioxaborolane (470 mg) was added thereto at the same temperature, followed by stirring while heating to room temperature over a period of 4 hours. A saturated ammonium chloride aqueous solution and ethyl acetate were added to the reaction mixture. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining 1-methyl-3-phenyl-4-(4,4,5,5-tetramethyl-1,3,2-dioxaborolan-2-yl)-1H-pyrazole (184 mg).
MSm/z(M+H):285.

<0124-3>

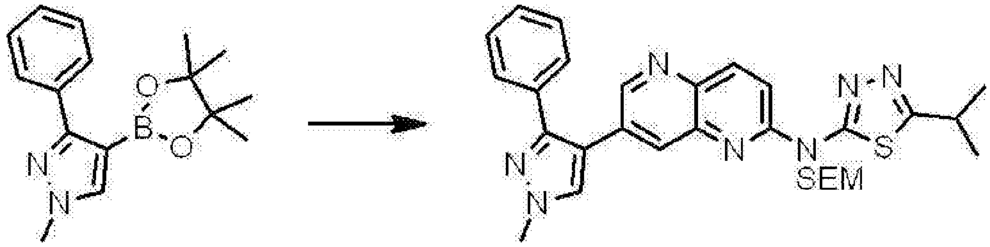

A mixture of N-(7-bromo-1,5-naphthyridin-2-yl)-5-isopropyl-N-((2-(trimethylsilyl)ethoxy)methyl)-1,3,4-thiadiazole-2-amine (20 mg), 1-methyl-3-phenyl-4-(4,4,5,5-tetramethyl-1,3,2-dioxaborolan-2-yl)-1H-pyrazole (15 mg), sodium carbonate (8 mg), bis(di-tert-butyl (4-dimethylaminophenyl)phosphine)dichloropalladium (II) (5 mg), and water (0.1 mL) in 1,4-dioxane (1 mL) was stirred at 100°C for 2 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (methanol-ethyl acetate-hexane), thereby obtaining 5-isopropyl-N-(7-(1-methyl-3-phenyl-1H-pyrazol-4-yl)-1,5-naphthyridin-2-yl)-N-((2-(trimethylsilyl)ethoxy)methyl)-1,3,4-thiadiazole-2-amine (11 mg).
MSm/z(M+H):558.

<0124-4>

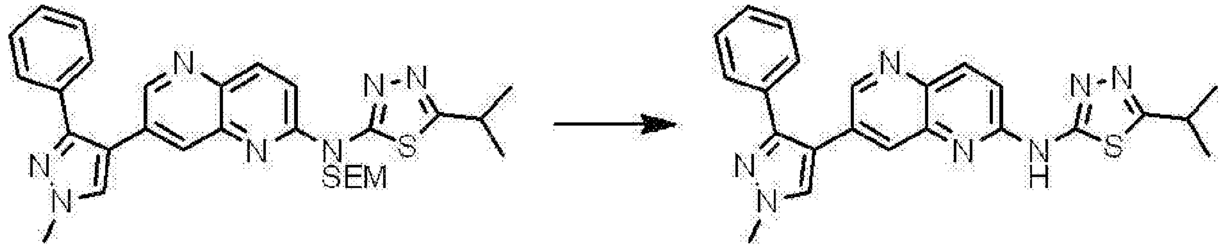

Water (0.1 mL) and trifluoroacetic acid (2 mL) were added to 5-isopropyl-N-(7-(1-methyl-3-phenyl-1H-pyrazol-4-yl)-1,5-naphthyridin-2-yl)-N-((2-(trimethylsilyl)ethoxy)methyl)-1,3,4-thiadiazole-2-amine (11 mg), followed by stirring at room temperature for 1 hour. The solvent was distilled off under reduced pressure, and the obtained residue was purified by silica gel column chromatography (methanol-ethyl acetate, NH silica), thereby obtaining 5-isopropyl-N-(7-(1-methyl-3-phenyl-1H-pyrazol-4-yl)-1,5-naphthyridin-2-yl)-1,3,4-thiadiazole-2-amine (5.2 mg). $^1$H-NMR($CDCl_3$/$CD_3OD$=4/1)$\delta$:8.59(1H,brs),8.18(1H,d,J=8.7Hz),8.13(1H,brs),7.84(1H,s),7.56-7.24(6H,m),3.47-3.34(1H, m),3.36(3H,s),1.48(6H,d,J=7.2Hz).
MSm/z(M+H):428.

<Example 0125>

<0125-1>

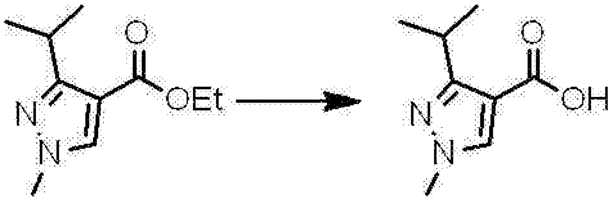

A4 mol/L sodium hydroxide aqueous solution (4 mL) was added to a solution of ethyl 3-isopropyl-1-methyl-1H-pyrazole-4-carboxylate (495 mg) in ethanol (4 mL), followed by stirring at 70°C for 4 hours. After the reaction mixture was cooled to room temperature, the solvent was distilled off under reduced pressure, the resultant product was adjusted to pH 2 by the addition of a 3 mol/L potassium hydrogen sulfate aqueous solution, and ethyl acetate was added thereto. The organic layer was collected by separation, washed with a saturated sodium chloride aqueous solution, and dried over anhydrous sodium sulfate, and the solvent was distilled off under reduced pressure, thereby obtaining 3-isopropyl-1-methyl-1H-pyrazole-4-carboxylic acid (346 mg).
MSm/z(M+H):169.

<0125-2>

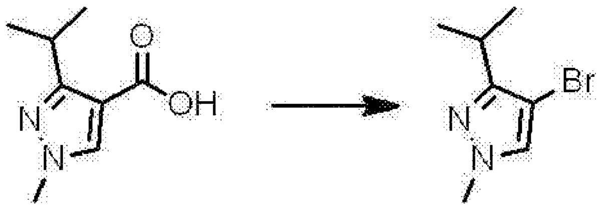

N-bromosuccinimide (401 mg) was added to a mixture of 3-isopropyl-1-methyl-1H-pyrazole-4-carboxylic acid (346 mg), and sodium hydrogen carbonate (585 mg) in N,N-dimethylformamide (6 mL), followed by stirring at room temperature for 4 hours. Ethyl acetate and water were added to the reaction mixture. The organic layer was collected by separation, washed sequentially with a 1 mol/L sodium hydroxide aqueous solution and a saturated sodium chloride aqueous solution, and dried over anhydrous sodium sulfate, and the solvent was distilled off under reduced pressure, thereby obtaining 4-bromo-3-isopropyl-1-methyl-1H-pyrazole (367 mg).
MSm/z(M+H):203,205.

<0125-3>

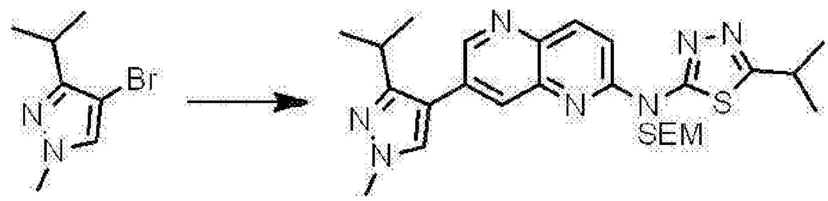

A mixture of N-(7-bromo-1,5-naphthyridin-2-yl)-5-isopropyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II)dichloride (3 mg), and potassium acetate (8 mg) in 1,4-dioxane (0.8 mL) was stirred at 100°C for 2 hours in a nitrogen atmosphere. 4-Bromo-3-isopropyl-1-methyl-1H-pyrazole (15 mg), sodium carbonate (8 mg), and bis(di-tert-butyl (4-dimethylaminophenyl)phosphine)dichloropalladium(II) (5 mg) were added to the reaction mixture, followed by stirring at 100°C for 2 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (methanol-ethyl acetate-hexane), thereby obtaining 5-isopropyl-N-(7-(3-isopropyl-1-methyl-1H-pyrazol-4-yl)-1,5-naphthyridin-2-yl)-N-((2-(trimethylsilyl)ethoxy)methyl)-1,3,4-thiadiazole-2-amine (4.7 mg).
MSm/z(M+H):524.

<0125-4>

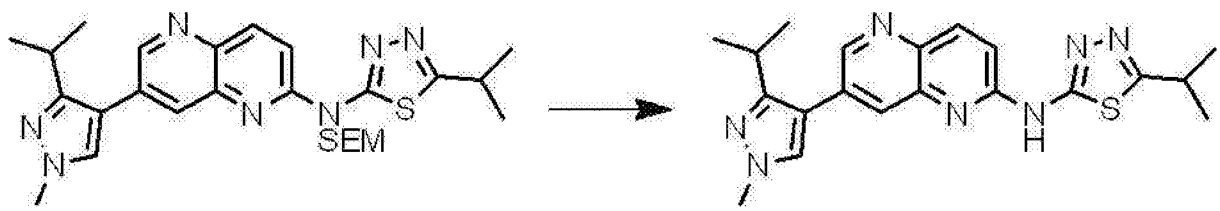

Water (0.1 mL) and trifluoroacetic acid (2 mL) were added to 5-isopropyl-N-(7-(3-isopropyl-1-methyl-1H-pyrazol-4-yl)-1,5-naphthyridin-2-yl)-N-((2-(trimethylsilyl)ethoxy)methyl)-1,3,4-thiadiazole-2-amine (4.7 mg), followed by stirring at room temperature for 30 minutes. The solvent was distilled off under reduced pressure, and the obtained residue was purified by silica gel column chromatography (methanol-ethyl acetate, NH silica), thereby obtaining 5-isopropyl-N-(7-(3-isopropyl-1-methyl-1H-pyrazol-4-yl)-1,5-naphthyridin-2-yl)-1,3,4-thiadiazole-2-amine (2.1 mg).
$^1$H-NMR($CDCl_3$/$CD_3OD$=4/1)δ:8.75(1H,d,J=2.1Hz),8.25(1H,d,J=8.7Hz),8.18(1H,d,J=2.1Hz),7.63(1H,s), 7.36(1H,d,J=8.7Hz),3.48-3.21(2H,m),3.36(3H,s),1.49(6H,d,J=7.2Hz),1.34(6H,d,J=6.6Hz).
MSm/z(M+H):394.

<Example 0126>

<0126-1>

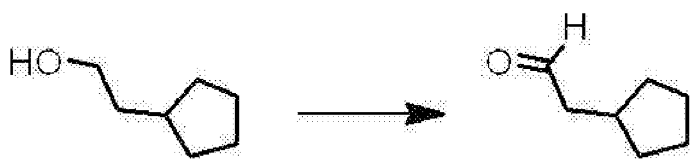

A6% sodium hypochlorite aqueous solution (7 mL) was added to a mixture of 2-cyclopentylethanol (1.4 g), sodium hydrogen carbonate (3.1 g), and 4-hydroxy-2,2,6,6-tetramethylpiperidine-1-oxyl free radical (19 mg) in toluene (20 mL), ethyl acetate (20 mL) and water (3.5 mL) under ice-cooling, followed by stirring at the same temperature for 10 minutes. A3 mol/L potassium hydrogen sulfate aqueous solution (10 mL), potassium iodide (120 mg), and ethyl

acetate were added to the reaction mixture. The organic layer was collected by separation, washed with a saturated sodium chloride aqueous solution, and dried over anhydrous sodium sulfate, and the solvent was distilled off under reduced pressure, thereby obtaining 2-cyclopentyl acetaldehyde (2.5 g).
$^1$H-NMR($CDCl_3$)$\delta$:9.75(1H,t,J=1.8Hz),2.44(2H,dd,J=7.2Hz,1.8Hz),2.34-2.19(1H,m),1.92-1.46(6H,m),1.22-1.05(2H,m).

<0126-2>

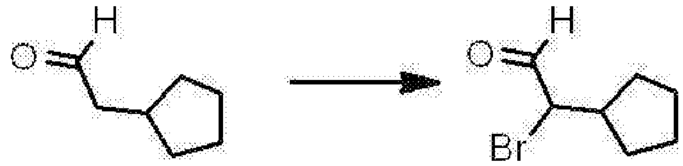

Trimethylphenylammonium tribromide (4.7 g) was added to a solution of 2-cyclopentyl acetaldehyde (2.5 g) in tetrahydrofuran (60 mL) under ice-cooling, followed by stirring while slowly heating to room temperature for 1 day. Ethyl acetate and water were added to the reaction mixture. The organic layer was collected by separation, washed sequentially with a 10% sodium hydrogen sulfite aqueous solution and a saturated sodium chloride aqueous solution, and dried over anhydrous sodium sulfate, and the solvent was distilled off under reduced pressure, thereby obtaining 2-bromo-2-cyclopentyl acetaldehyde (2.4 g).
$^1$H-NMR($CDCl_3$):9.39(1H,d,J=3.9Hz),3.73-3.64(1H,m),2.03-1.46(7H,m),1.22-1.05(2H,m).

<0126-3>

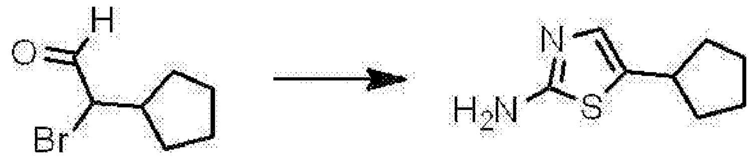

Thiourea (936 mg) was added to a solution of 2-bromo-2-cyclopentyl acetaldehyde (2.4 g) in ethanol (12 mL), followed by stirring for 4 hours under heating to reflux. The reaction mixture was cooled to room temperature, and ethyl acetate and water were added thereto. The organic layer was collected by separation, washed sequentially with a 4 mol/L sodium hydroxide aqueous solution and a saturated sodium chloride aqueous solution, and dried over anhydrous sodium sulfate, and the solvent was distilled off under reduced pressure. The obtained residue was purified by silica gel column chromatography (methanol-ethyl acetate), thereby obtaining 5-cyclopentylthiazole-2-amine (412 mg).
MSm/z(M+H):169.

<0126-4>

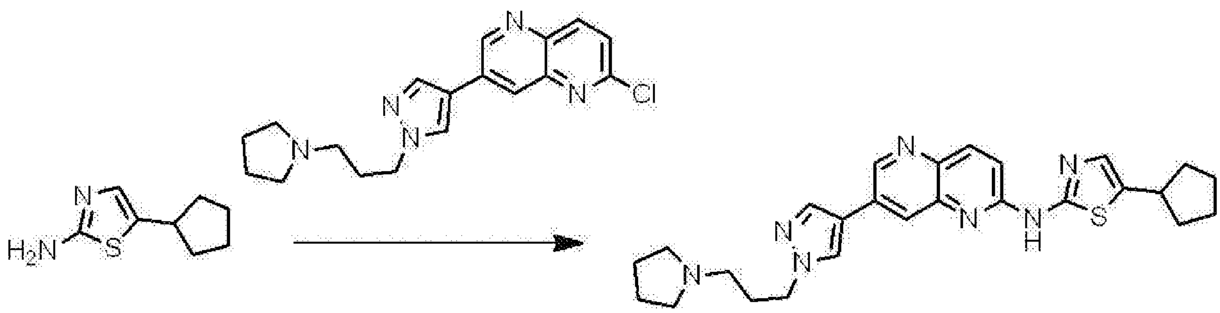

A mixture of 2-chloro-7-(1-(3-(pyrrolidin-1-yl)propyl)-1H-pyrazol-4-yl)-1,5-naphthyridine (10 mg), 5-cyclopentylthiazole-2-amine (6 mg), tris (dibenzylideneacetone)dipalladium(0) (3 mg), 4,5-bis(diphenylphosphino)-9,9-dimethylxanthene (4 mg), and cesium carbonate (24 mg) in 1,4-dioxane (1 mL) was stirred at 150°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining 5-cyclopentyl-N-(7-(1-(3-(pyrrolidin-1-yl)propyl)-1H-pyrazol-4-yl)-1,5-naphthyridin-2-yl)thiazole-2-amine (5.1 mg).
$^1$H-NMR(CDCl$_3$/CD$_3$OD=4/1)δ:8.86(1H,brs),8.28(1H,brs),8.14(1H,d,J=9.0Hz),8.04(1H,s),8.01(1H,s),7.25(1H,d,J=9.0Hz),7.09(1H,s),4.33-4.25(2H,m),3.30-3.19(1H,m),2.61-2.46(6H,m),2.24-2.11(4H,m),1.94-1.67(10H,m).
MSm/z(M+H):474.

<Example 0127>

<0127-1>

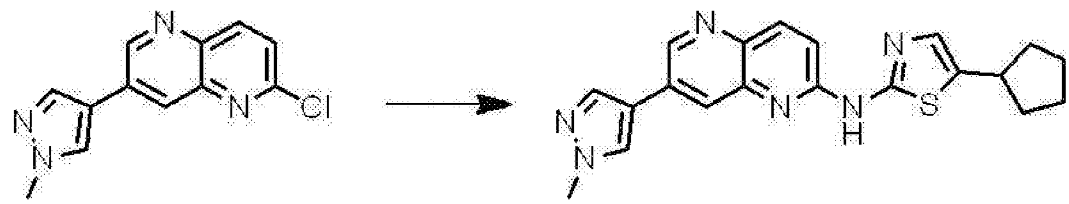

A mixture of 2-chloro-7-(1-methyl-1H-pyrazol-4-yl)-1,5-naphthyridine (14 mg), 5-cyclopentylthiazole-2-amine (12 mg), tris (dibenzylideneacetone)dipalladium(0) (5 mg), 4,5-bis(diphenylphosphino)-9,9-dimethylxanthene (7 mg), and cesium carbonate (48 mg) in 1,4-dioxane (1 mL) was stirred at 150°C for 30 minutes using a microwave reaction apparatus. The reaction mixture was cooled to room temperature, and ethyl acetate and water were added thereto. The solid matter was collected by filtration, thereby obtaining 5-cyclopentyl-N-(7-(1-methyl-1H-pyrazol-4-yl)-1,5-naphthyridin-2-yl)thiazole-2-amine (10 mg).
$^1$H-NMR(DMSO-d$_6$)δ:11.57(1H,brs),9.02(1H,d,J=2.1Hz),8.52(1H,s),8.25(1H,d,J=2.1Hz),8.20(1H,s),8.17(1H,d,J=8.7Hz ),7.35(1H,d,J=8.7Hz),7.18(1H,s),3.92(3H,s),3.44-3.35(1H,m),2.16-2.05(2H,m),1.84-1.58(6H,m).
MSm/z(M+H):377.

<Example 0128>

<0128-1>

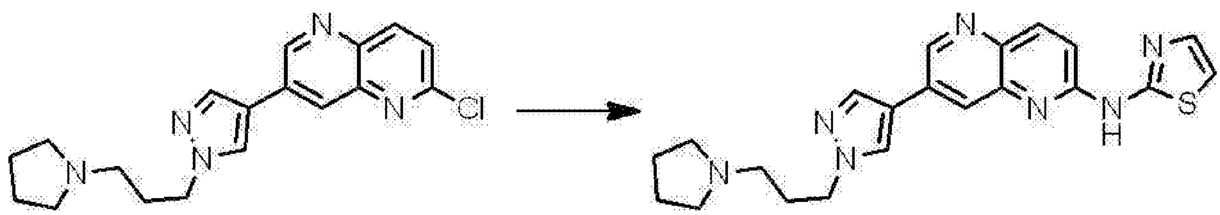

A mixture of 2-chloro-7-(1-(3-(pyrrolidin-1-yl)propyl)-1H-pyrazol-4-yl)-1,5-naphthyridine (10 mg), thiazole-2-amine (6 mg), tris(dibenzylideneacetone)dipalladium(0) (3 mg), 4,5-bis(diphenylphosphino)-9,9-dimethylxanthene (4 mg), and cesium carbonate (24 mg) in 1,4-dioxane (1 mL) was stirred at 150°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining N-(7-(1-(3-(pyrrolidin-1-yl)propyl)-1H-pyrazol-4-yl)-1,5-naphthyridin-2-yl)thiazole-2-amine (3.7 mg).
$^1$H-NMR(CDCl$_3$/CD$_3$OD=4/1)δ:8.88(1H,brs),8.31(1H,brs),8.17(1H,d,J=9.0Hz),8.04(1H,s),8.00(1H,s),7.45(1H,d,J=3.9Hz),7.30(1H,d,J=9.0Hz),6.97(1H,d,J=3.9Hz),4.33-4.26(2H,m),2.60-2.47(6H,m),2.23-2.11(2H,m),1.88-1.78(4H,m).

MSm/z(M+H):406.

<Example 0129>

<0129-1>

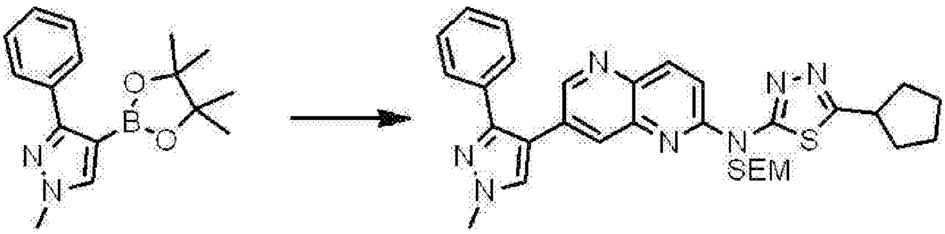

A mixture of N-(7-bromo-1,5-naphthyridin-2-yl)-5-cyclopentyl-N-((2-(trimethylsilyl)ethoxy)methyl)-1,3,4-thiadiazole-2-amine (10 mg), 1-methyl-3-phenyl-4-(4,4,5,5-tetramethyl-1,3,2-dioxaborolan-2-yl)-1H-pyrazole (8 mg), sodium carbonate (5 mg), bis(di-tert-butyl (4-dimethylaminophenyl)phosphine)dichloropalladium(II) (2 mg), and water (0.07 mL) in 1,4-dioxane (0.7 mL) was stirred at 100°C for 6 hours. The reaction mixture was cooled to room temperature, the insolubles were filtered off using celite, and the solvent was distilled off under reduced pressure. The obtained residue was purified by silica gel column chromatography (ethyl acetate-hexane), thereby obtaining 5-cyclopentyl-N-(7-(1-methyl-3-phenyl-1H-pyrazol-4-yl)-1,5-naphthyridin-2-yl)-N-((2-(trimethylsilyl)ethoxy)methyl)-1,3,4-thiadiazole-2-amine (4.7 mg).
MSm/z(M+H):584.

<0129-2>

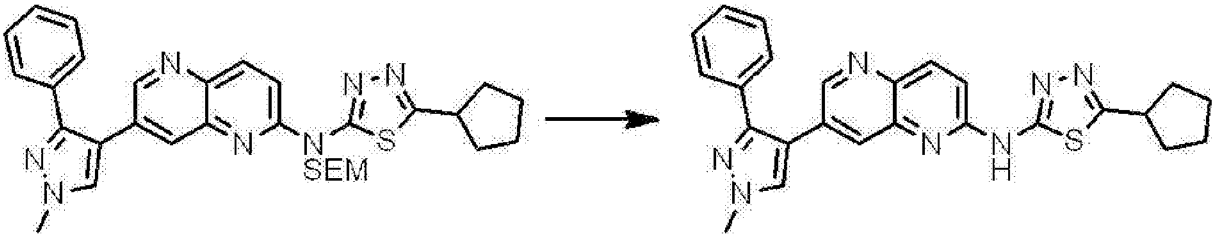

Water (0.1 mL) and trifluoroacetic acid (2 mL) were added to 5-cyclopentyl-N-(7-(1-methyl-3-phenyl-1H-pyrazol-4-yl)-1,5-naphthyridin-2-yl)-N-((2-(trimethylsilyl)ethoxy)methyl)-1,3,4-thiadiazole-2-amine (4.7 mg), followed by stirring at room temperature for 30 minutes. The solvent was distilled off under reduced pressure, and the obtained residue was purified by silica gel column chromatography (methanol-ethyl acetate, NH silica), thereby obtaining 5-cyclopentyl-N-(7-(1-methyl-3-phenyl-1H-pyrazol-4-yl)-1,5-naphthyridin-2-yl)-1,3,4-thiadiazole-2-amine (2.6 mg). $^1$H-NMR($CDCl_3$/$CD_3OD$=4/1)δ:8.59(1H,brs),8.18(1H,d,J=8.7Hz),8.13(1H,brs),7.84(1H,s),7.56-7.24(6H,m),3.56-3.3.43(1H,m),3.36(3H,s),2.32-2.20(2H,m),1.96-1.74(6H,m).
MSm/z(M+H):454.

<Example 0130>

<0130-1>

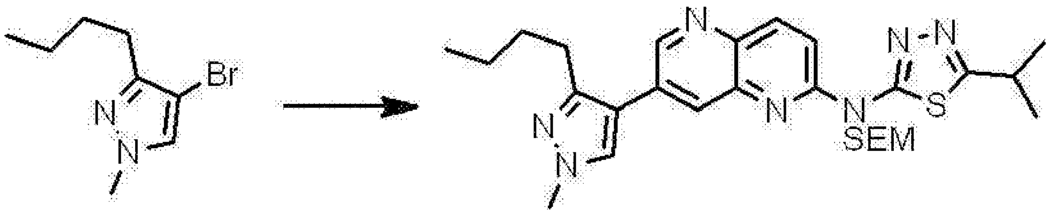

A mixture of N-(7-bromo-1,5-naphthyridin-2-yl)-5-isopropyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II)dichloride (3 mg), and potassium acetate (8 mg) in 1,4-dioxane (0.8 mL) was stirred at 100°C for 2 hours in a nitrogen atmosphere. 4-Bromo-3-butyl-1-methyl-1H-pyrazole (15 mg), sodium carbonate (8 mg), and bis(di-tert-butyl (4-dimethylaminophenyl)phosphine)dichloropalladium(II) (5 mg) were added to the reaction mixture, followed by stirring at 100°C for 2 hours. The reaction mixture was cooled to room temperature, the insolubles were filtered off using celite, and the solvent was distilled off under reduced pressure. The obtained residue was purified by silica gel column chromatography (methanol-ethyl acetate), thereby obtaining N-(7-(3-butyl-1-methyl-1H-pyrazol-4-yl)-1,5-naphthyridin-2-yl)-5-isopropyl-N-((2-(trimethylsilyl)ethoxy)methyl)-1,3,4-thiadiazole-2-amine (7.4 mg).
MSm/z(M+H):538.

<0130-2>

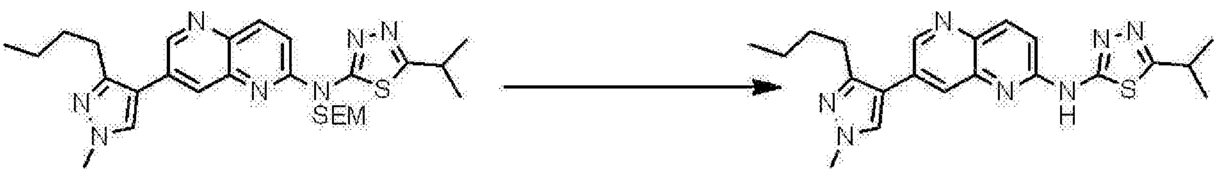

Water (0.1 mL) and trifluoroacetic acid (2 mL) were added to N-(7-(3-butyl-1-methyl-1H-pyrazol-4-yl)-1,5-naphthyridin-2-yl)-5-isopropyl-N-((2-(trimethylsilyl)ethoxy)methyl)-1,3,4-thiadiazole-2-amine (7.4 mg), followed by stirring at room temperature for 30 minutes. The solvent was distilled off under reduced pressure, and the obtained residue was purified by silica gel column chromatography (methanol-ethyl acetate, NH silica), thereby obtaining N-(7-(3-butyl-1-methyl-1H-pyrazol-4-yl)-1,5-naphthyridin-2-yl)-5-isopropyl-1,3,4-thiadiazole-2-amine (7.4 mg).
$^1$H-NMR($CDCl_3$/$CD_3OD$=4/1)$\delta$:8.78(1H,brs),8.24(1H,d,J=9.0Hz),8.21(1H,brs),7.73(1H,s),7.36(1H,d,J=9. 0Hz),3.47-3.38(1H,m),3.36(3H,s),2.91-2.84(2H,m),1.76-1.63(2H,m),1.51-1.40(2H,m), 1.49(6H,d,J=6.6Hz),0.96(3H,t,J=7.2Hz).
MSm/z(M+H):408.

<Example 0131>

<0131-1>

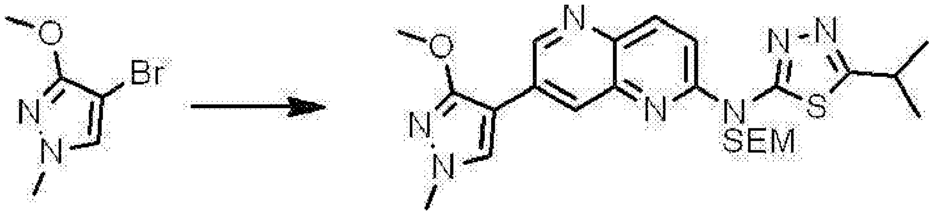

A mixture of N-(7-bromo-1,5-naphthyridin-2-yl)-5-isopropyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II) dichloride (3 mg), and potassium acetate (8 mg) in 1,4-dioxane (0.8 mL) was stirred at 100°C for 2 hours in a nitrogen atmosphere. 4-Bromo-3-methoxy-1-methyl-1H-pyrazole (15 mg), sodium carbonate (8 mg), and bis(di-tert-butyl (4-dimethylaminophenyl)phosphine)dichloropalladium(II) (5 mg) were added to the reaction mixture, followed by stirring at 100°C for 2 hours. The reaction mixture was cooled to room temperature, the insolubles were filtered off using celite, and the solvent was distilled off under reduced pressure. The obtained residue was purified by silica gel column chromatography (methanol-ethyl acetate), thereby obtaining 5-isopropyl-N-(7-(3-methoxy-1-methyl-1H-pyrazol-4-yl)-1,5-naphthyridin-2-yl)-N-((2-(trimethylsilyl)ethoxy)methyl)-1,3,4-thiadiazole-2-amine (12 mg).
MSm/z(M+H):512.

<0131-2>

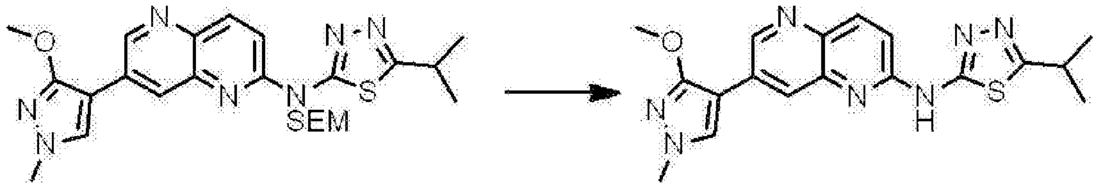

Water (0.1 mL) and trifluoroacetic acid (2 mL) were added to 5-isopropyl-N-(7-(3-methoxy-1-methyl-1H-pyrazol-4-yl)-1,5-naphthyridin-2-yl)-N-((2-(trimethylsilyl)ethoxy)methyl)-1,3,4-thiadiazole-2-amine (12 mg), followed by stirring at room temperature for 30 minutes. The solvent was distilled off under reduced pressure, and the obtained residue was purified by silica gel column chromatography (methanol-ethyl acetate, NH silica), thereby obtaining 5-isopropyl-N-(7-(3-methoxy-1-methyl-1H-pyrazol-4-yl)-1,5-naphthyridin-2-yl)-1,3,4-thiadiazole-2-amine (2.8 mg).
$^1$H-NMR($CDCl_3$/$CD_3OD$=4/1)δ:9.01(1H,d,J=1.8Hz),8.40(1H,d,J=1.8Hz),8.19(1H,d,J=9.0Hz),7.80(1H,s), 7.30(1H,d,J=9.0Hz),4.09(3H,s),3.87(3H,s),3.50-3.38(1H,m),1.51(6H,d,J=6.6Hz).
MSm/z(M+H):382.

<Example 0132>

<0132-1>

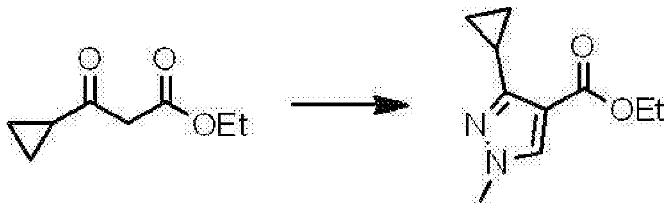

0829] A solution of methylhydrazine (0.18 mL) and ethyl formate (0.45 mL) in ethanol (2 mL) was stirred for 4 hours under heating to reflux, and ethyl 3-cyclopropyl-3-oxopropanoate (1.0 g) was added thereto, followed by stirring at the same temperature for 4 hours. The reaction mixture was cooled to room temperature, and a 20% sodium ethoxide-ethanol solution (2 mL) was added thereto, followed by stirring for 1.5 hours under heating to reflux. The reaction mixture was cooled to room temperature, and ethyl acetate and a 3 mol/L potassium hydrogen sulfate aqueous solution were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining ethyl 3-cyclopropyl-1-methyl-1H-pyrazole-4-carboxylate (167 mg).
$^1$H-NMR($CDCl_3$/$CD_3OD$=4/1):7.74(1H,s),4.29(2H,q,J=6.6Hz),3.79(3H,s),2.56-2.46(1H,m),1.34(3H,t,J=6.6Hz),1.00-0.87(4H,m).

<0132-2>

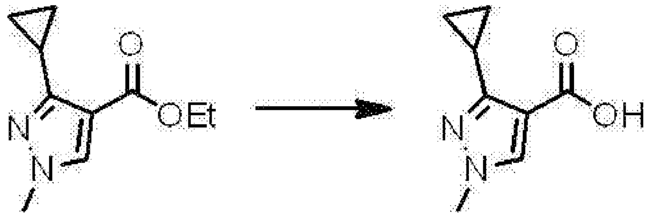

A4 mol/L sodium hydroxide aqueous solution (4 mL) was added to a solution of ethyl 3-cyclopropyl-1-methyl-1H-pyrazole-4-carboxylate (167 mg) in ethanol (4 mL), followed by stirring at 70°C for 2 hours. After the reaction mixture was cooled to room temperature, the solvent was distilled off under reduced pressure, the resultant product was adjusted to pH 2 by the addition of a 3 mol/L potassium hydrogen sulfate aqueous solution, and ethyl acetate was added thereto. The organic layer was collected by separation, washed with a saturated sodium chloride aqueous solution, and dried over anhydrous sodium sulfate, and the solvent was distilled off under reduced pressure, thereby obtaining 3-cyclopropyl-1-methyl-1H-pyrazole-4-carboxylic acid (131 mg). MSm/z(M+H):167.

<0132-3>

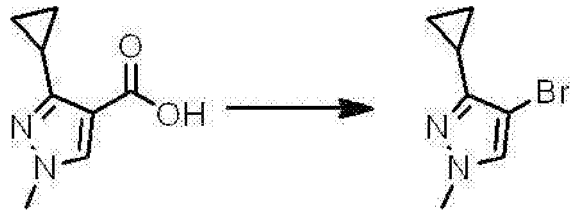

N-bromosuccinimide (155 mg) was added to a mixture of 3-cyclopropyl-1-methyl-1H-pyrazole-4-carboxylic acid (131 mg), and sodium hydrogen carbonate (226 mg) in N,N-dimethylformamide (4 mL), followed by stirring at room temperature for 4 hours. Ethyl acetate and water were added to the reaction mixture. The organic layer was collected by separation, washed with a saturated sodium chloride aqueous solution, and dried over anhydrous sodium sulfate, and the solvent was distilled off under reduced pressure, thereby obtaining 4-bromo-3-cyclopropyl-1-methyl-1H-pyrazole (146 mg).
MSm/z(M+H):201,203.

<0132-4>

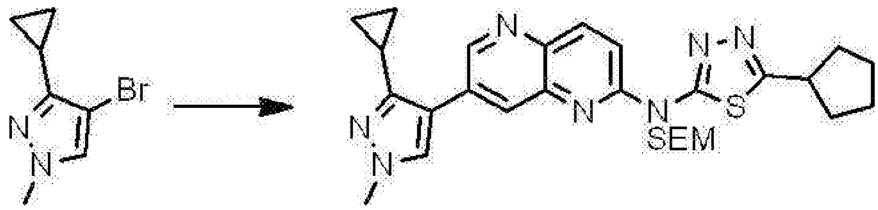

A mixture of N-(7-bromo-1,5-naphthyridin-2-yl)-5-cyclopentyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II) dichloride (3 mg), and potassium acetate (8 mg) in 1,4-dioxane (0.8 mL) was stirred at 100°C for 2 hours in a nitrogen atmosphere. 4-Bromo-3-cyclopropyl-1-methyl-1H-pyrazole (15 mg), sodium carbonate (8 mg), and bis(di-tert-butyl (4-dimethylaminophenyl)phosphine)dichloropalladium(II) (5 mg) were added to the reaction mixture, followed by stirring at 100°C for 3 hours. The reaction mixture was cooled to room temperature, the insolubles were filtered off using celite, and the solvent was distilled off under reduced pressure. The obtained residue was purified by silica gel column chromatography (methanol-ethyl acetate), thereby obtaining 5-cyclopentyl-N-(7-(3-cyclopropyl-1-methyl-1H-pyrazol-4-yl)-1,5-naphthyridin-2-yl)-N-((2-(trimethylsilyl)ethoxy)methyl)-1,3,4-thiadiazole-2-amine (7.6 mg).
MSm/z(M+H):548.

<0132-5>

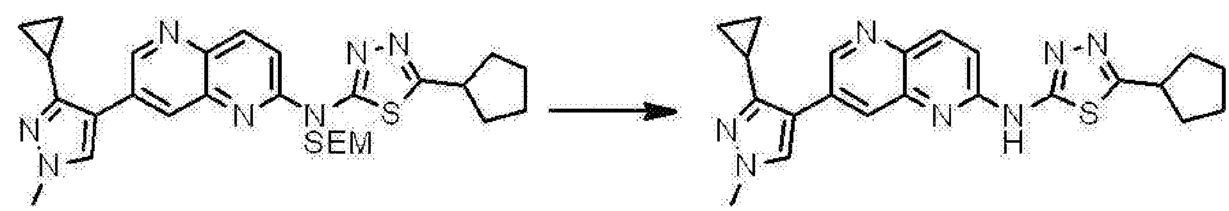

Water (0.1 mL) and trifluoroacetic acid (2 mL) were added to 5-cyclopentyl-N-(7-(3-cyclopropyl-1-methyl-1H-pyrazol-4-yl)-1,5-naphthyridin-2-yl)-N-((2-(trimethylsilyl)ethoxy)methyl)-1,3,4-thiadiazole-2-amine (7.6 mg), followed by stirring at room temperature for 30 minutes. The solvent was distilled off under reduced pressure, and the obtained residue was purified by silica gel column chromatography (methanol-ethyl acetate, NH silica), thereby obtaining 5-cyclopentyl-N-(7-(3-cyclopropyl-1-methyl-1H-pyrazol-4-yl)-1,5-naphthyridin-2-yl)-1,3,4-thiadiazole-2-amine (5.6 mg).
$^1$H-NMR($CDCl_3$/$CD_3OD$=4/1)δ:8.96(1H,d,J=2.1Hz),8.42(1H,d,J=2.1Hz),8.25(1H,d,J=8.7Hz),7.75(1H,s), 7.35(1H,d,J=9.0Hz),3.92(3H,s),3.58-3.46(1H,m),2.34-2.18(2H,m),2.09-1.74(7H,m),1.06-0.97(4H,m).
MSm/z(M+H):418.

<Example 0133>

<0133-1>

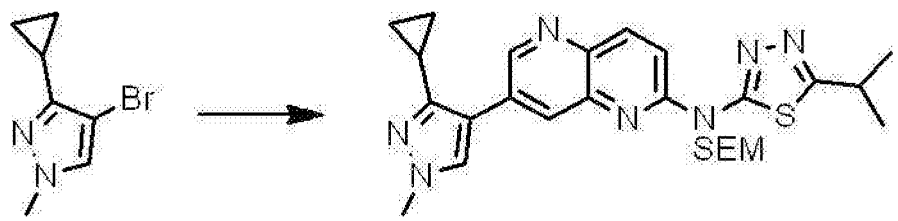

A mixture of N-(7-bromo-1,5-naphthyridin-2-yl)-5-isopropyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II) dichloride (3 mg), and potassium acetate (8 mg) in 1,4-dioxane (0.8 mL) was stirred at 100°C for 2 hours in a nitrogen atmosphere. 4-Bromo-3-cyclopropyl-1-methyl-1H-pyrazole (15 mg), sodium carbonate (8 mg), and bis(di-tert-butyl (4-dimethylaminophenyl)phosphine)dichloropalladium(II) (5 mg) were added to the reaction mixture, followed by stirring at 100°C for 2 hours. The reaction mixture was cooled to room temperature, the insolubles were filtered off using celite, and the solvent was distilled off under reduced pressure. The obtained residue was purified by silica gel column chromatography (methanol-ethyl acetate), thereby obtaining N-(7-(3-cyclopropyl-1-methyl-1H-pyrazol-4-yl)-1,5-naphthyridin-2-yl)-5-isopropyl-N-((2-(trimethylsilyl)ethoxy)methyl)-1,3,4-thiadiazole-2-amine (12 mg).
MSm/z(M+H):522.

<0133-2>

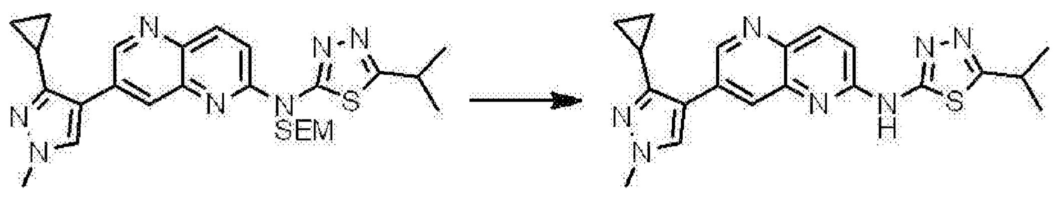

Water (0.1 mL) and trifluoroacetic acid (2 mL) were added to N-(7-(3-cyclopropyl-1-methyl-1H-pyrazol-4-yl)-1,5-naphthyridin-2-yl)-5-isopropyl-N-((2-(trimethylsilyl)ethoxy)methyl)-1,3,4-thiadiazole-2-amine (12 mg), followed by stirring at room temperature for 30 minutes. The solvent was distilled off under reduced pressure, and the obtained residue was purified by silica gel column chromatography (methanol-ethyl acetate, NH silica), thereby obtaining N-(7-(3-cyclopropyl-1-methyl-1H-pyrazol-4-yl)-1,5-naphthyridin-2-yl)-5-isopropyl-1,3,4-thiadiazole-2-amine (6.1 mg).
$^1$H-NMR($CDCl_3$/$CD_3OD$=4/1)δ:8.95(1H,brs),8.41(1H,brs),8.24(1H,d,J=9.0Hz),7.74(1H,s),7.34(1H,d,J=9.0Hz),3.92(3H,s),3.49-3.34(1H,m),2.09-1.98(1H,m),1.49(6H,d,J=7.2Hz),1.07-0.96(4H,m).
MSm/z(M+H):392.

<Example 0134>

<0134-1>

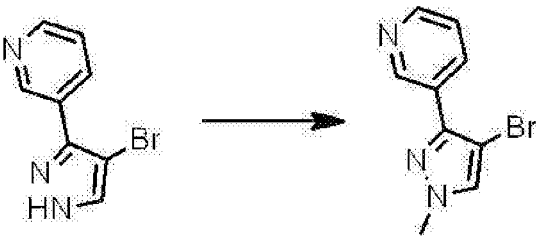

60% sodium hydride (412 mg) was added to a solution of 3-(4-bromo-1H-pyrazol-3-yl)pyridine (1.90 g) in N-methylpyrrolidone (7 mL) under ice-cooling, followed by stirring at the same temperature for 10 minutes, and iodomethane (0.64 mL) was added thereto, followed by stirring at the same temperature for 1 hour. Ethyl acetate and water were added to the reaction mixture. The organic layer was collected by separation, washed with a saturated sodium chloride aqueous solution, and dried over anhydrous sodium sulfate, and the solvent was distilled off under reduced pressure, thereby obtaining 3-(4-bromo-1-methyl-1H-pyrazol-3-yl)pyridine (284 mg).
MSm/z(M+H):238,240.

<0134-2>

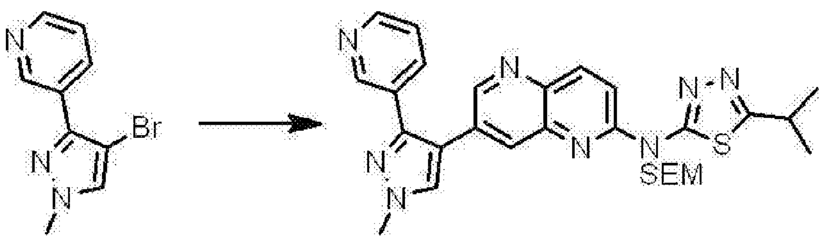

A mixture of N-(7-bromo-1,5-naphthyridin-2-yl)-5-isopropyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II) dichloride (3 mg), and potassium acetate (8 mg) in 1,4-dioxane (0.8 mL) was stirred at 100°C for 2 hours in a nitrogen atmosphere. 3-(4-Bromo-1-methyl-1H-pyrazol-3-yl)pyridine (15 mg), sodium carbonate (8 mg), and bis(di-tert-butyl (4-dimethylaminophenyl)phosphine)dichloropalladium(II) (5 mg) were added to the reaction mixture, followed by stirring at 100°C for 2 hours. The reaction mixture was cooled to room temperature, the insolubles were filtered off using celite, and the solvent was distilled off under reduced pressure. The obtained residue was purified by silica gel column chromatography (methanol-ethyl acetate), thereby obtaining 5-isopropyl-N-(7-(1-methyl-3-(pyridin-3-yl)-1H-pyrazol-4-yl)-1,5-naphthyridin-2-yl)-N-((2-(trimethylsilyl)ethoxy)methyl)-1,3,4-thiadiazole-2-amine (26 mg).
MSm/z(M+H):559.

<0134-3>

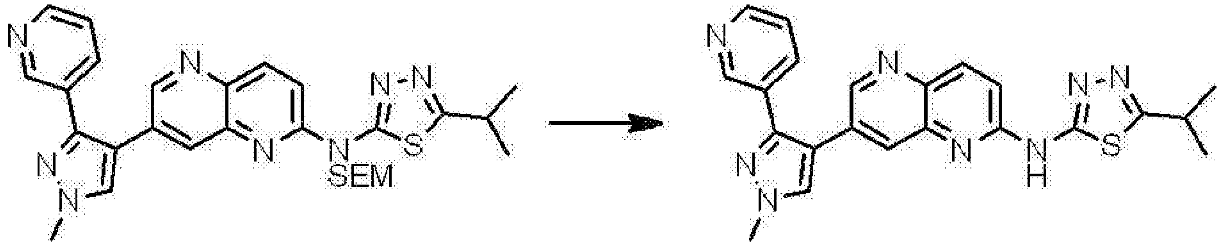

Water (0.1 mL) and trifluoroacetic acid (2 mL) were added to 5-isopropyl-N-(7-(1-methyl-3-(pyridin-3-yl)-1H-pyrazol-4-yl)-1,5-naphthyridin-2-yl)-N-((2-(trimethylsilyl)ethoxy)methyl)-1,3,4-thiadiazole-2-amine (26 mg), followed by stirring at room temperature for 30 minutes. The solvent was distilled off under reduced pressure, and the obtained residue was purified by silica gel column chromatography (methanol-ethyl acetate, NH silica), thereby obtaining 5-isopropyl-N-(7-(1-methyl-3-(pyridin-3-yl)-1H-pyrazol-4-yl)-1,5-naphthyridin-2-yl)-1,3,4-thiadiazole-2-amine(8.0mg).
$^1$H-NMR($CDCl_3$/$CD_3OD$=4/1)δ:8.73(1H,d,J=1.8Hz),8.57(1H,d,J=1.8Hz),8.55-8.52(1H,m),8.23(1H,d,J=9.0Hz),8.14(1H,d,J=2.1hz), 7.86(1H,s), 7.89-7.83(1H,m), 7.41(1H,d,J=9.0Hz), 7.40- 7.34(1H,m),3.50-3.36(1H,m),3.37(3H,s), 1.48(6H,d,J=7.2Hz).
MSm/z(M+H):429.

<Example 0135>

<0135-1>

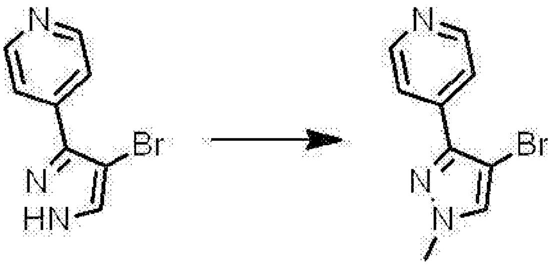

60% sodium hydride (412 mg) was added to a solution of 4-(4-bromo-1H-pyrazol-3-yl)pyridine (2.77g) in N-methylpyrrolidone (7 mL) under ice-cooling, followed by stirring at the same temperature for 10 minutes, and iodomethane (0.64 mL) was added thereto, followed by stirring at the same temperature for 1 hour. Ethyl acetate and water were added to the reaction mixture. The organic layer was collected by separation, washed with a saturated sodium chloride aqueous solution, and dried over anhydrous sodium sulfate, and the solvent was distilled off under reduced pressure, thereby obtaining 4-(4-bromo-1-methyl-1H-pyrazol-3-yl)pyridine (177 mg).
MSm/z(M+H):238,240.

<0135-2>

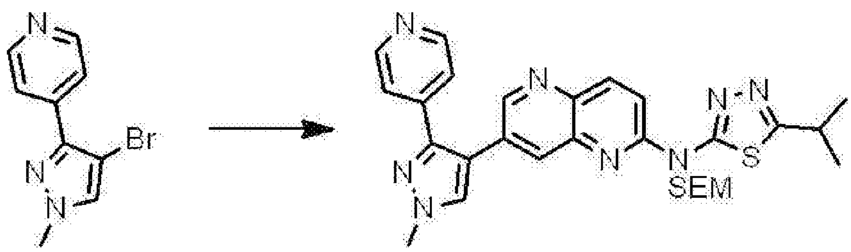

A mixture of N-(7-bromo-1,5-naphthyridin-2-yl)-5-isopropyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II) dichloride (3 mg), and potassium acetate (8 mg) in 1,4-dioxane (0.8 mL) was stirred at 100°C for 2 hours in a nitrogen atmosphere. 4-(4-Bromo-1-methyl-1H-pyrazol-3-yl)pyridine (15 mg), sodium carbonate (8 mg), and bis(di-tert-butyl (4-dimethylaminophenyl)phosphine)dichloropalladium(II) (5 mg) were added to the reaction mixture, followed by stirring at 100°C for 2 hours. The reaction mixture was cooled to room temperature, the insolubles were filtered off using celite, and the solvent was distilled off under reduced pressure. The obtained residue was purified by silica gel column chromatography (methanol-ethyl acetate), thereby obtaining 5-isopropyl-N-(7-(1-methyl-3-(pyridin-4-yl)-1H-pyrazol-4-yl)-1,5-naphthyridin-2-yl)-N-((2-(trimethylsilyl)ethoxy)methyl)-1,3,4-thiadiazole-2-amine (10 mg).
MSm/z(M+H):559.

<0135-3>

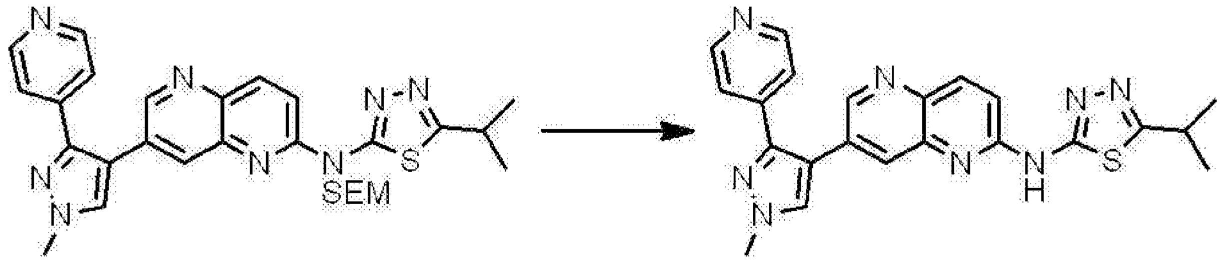

Water (0.1 mL) and trifluoroacetic acid (2 mL) were added to 5-isopropyl-N-(7-(1-methyl-3-(pyridin-4-yl)-1H-pyrazol-4-yl)-1,5-naphthyridin-2-yl)-N-((2-(trimethylsilyl)ethoxy)methyl)-1,3,4-thiadiazole-2-amine (10 mg), followed by stirring at room temperature for 30 minutes. The solvent was distilled off under reduced pressure, and the obtained residue was purified by silica gel column chromatography (methanol-ethyl acetate, NH silica), thereby obtaining 5-isopropyl-N-(7-(1-methyl-3-(pyridin-4-yl)-1H-pyrazol-4-yl)-1,5-naphthyridin-2-yl)-1,3,4-thiadiazole-2-amine (2.4 mg).
$^1$H-NMR($CDCl_3$/$CD_3OD$=4/1)δ:8.59(1H,d,J=2.1Hz),8.50(2H,d,J=6.0Hz),8.25(1H,d,J=9.0Hz),8.19(1H,d,J =2.1Hz),7.82(1H,s),7.49(2H,d,J=6.0Hz),7.38(1H,d,J=9.0Hz),3.47-3.36(1H,m),3.36(3H,s),1.47(6H,d,J=7.2Hz).
MSm/z(M+H):429.

<Example 0136>

<0136-1>

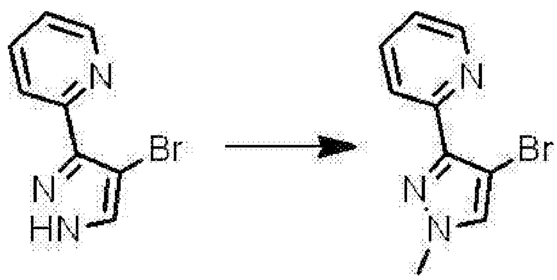

60% sodium hydride (412 mg) was added to a solution of 2-(4-bromo-1H-pyrazol-3-yl)pyridine (1.59 g) in N-methylpyrrolidone (7 mL) under ice-cooling, followed by stirring at the same temperature for 10 minutes, and iodomethane (0.64 mL) was added thereto, followed by stirring at the same temperature for 1 hour. Ethyl acetate and water were added to the reaction mixture. The organic layer was collected by separation, washed with a saturated sodium chloride aqueous solution, and dried over anhydrous sodium sulfate, and the solvent was distilled off under reduced pressure, thereby obtaining 2-(4-bromo-1-methyl-1H-pyrazol-3-yl)pyridine (770 mg).
MSm/z(M+H):238,240.

<0136-2>

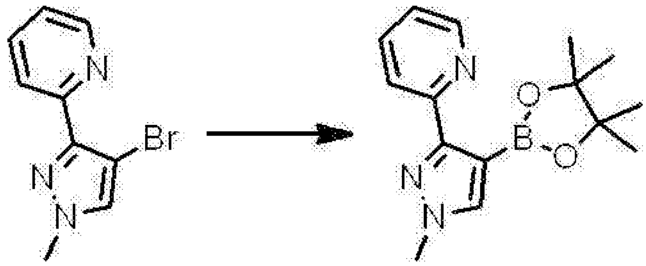

A 1.6 mol/L n-butyllithium-hexane solution (1.45 mL) was added to a solution of 2-(4-bromo-1-methyl-1H-pyrazol-3-yl)pyridine (370 mg) in tetrahydrofuran (8 mL) at -80°C, followed by stirring at the same temperature for 30 minutes, and 2-isopropyloxy-4,4,5,5-tetramethyl-1,3,2-dioxaborolane (576 mg) was added thereto at the same temperature, followed by stirring while slowly heating to room temperature over a period of 2.5 hours. A saturated ammonium chloride aqueous solution and ethyl acetate were added to the reaction mixture. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining 2-(1-methyl-4-(4,4,5,5-tetramethyl-1,3,2-dioxaborolan-2-yl)-1H-pyrazol-3-yl)pyridine (20 mg).
$^1$H-NMR($CDCl_3$/$CD_3OD$=4/1):8.54-8.51(1H,m),8.34-8.23(1H,m),7.84-7.78(1H,m),7.79(1H,s),7.30-7.25(1H,m),3.97(3H,s), 1.56(12H,s).

<0136-3>

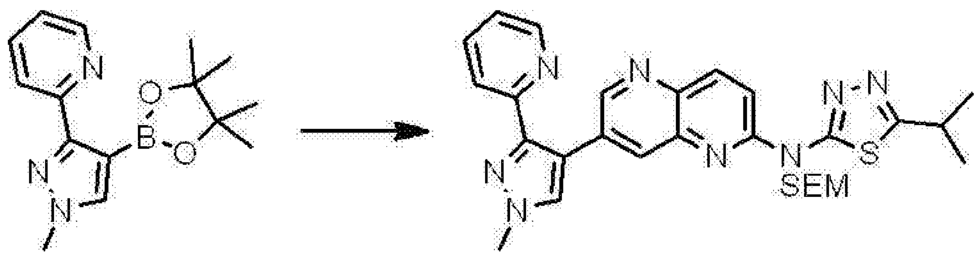

A mixture of N-(7-bromo-1,5-naphthyridin-2-yl)-5-isopropyl-N-((2-(trimethylsilyl)ethoxy)methyl)-1,3,4-thiadiazole-2-amine (20 mg), 2-(1-methyl-4-(4,4,5,5-tetramethyl-1,3,2-dioxaborolan-2-yl)-1H-pyrazol-3-yl)pyridine(15 mg), sodium carbonate (8 mg), bis(di-tert-butyl (4-dimethylaminophenyl)phosphine)dichloropalladium (II) (5 mg), and water (0.1 mL) in 1,4-dioxane (1 mL) was stirred at 100°C for 2 hours. The reaction mixture was cooled to room temperature, the insolubles were filtered off using celite, and the solvent was distilled off under reduced pressure. The obtained residue was purified by silica gel column chromatography (methanol-ethyl acetate-hexane), thereby obtaining 5-isopropyl-N-(7-(1-methyl-3-(pyridin-2-yl)-1H-pyrazol-4-yl)-1,5-naphthyridin-2-yl)-N-((2-(trimethylsilyl)ethoxy)methyl)-1,3,4-thiadiazole-2-amine (12 mg).
MSm/z(M+H):559.

<0136-4>

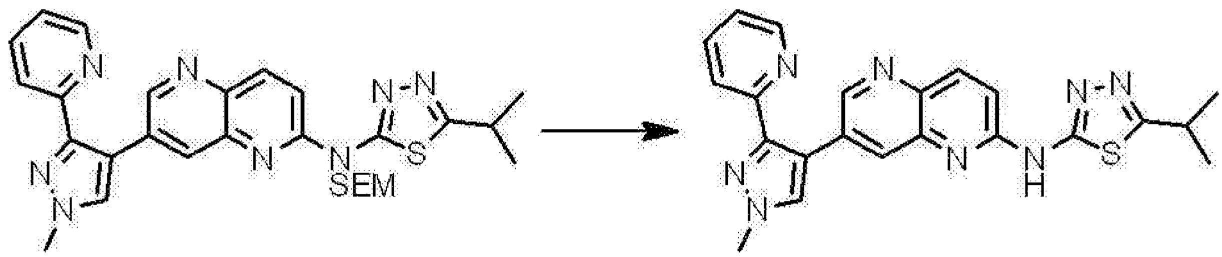

Water (0.1 mL) and trifluoroacetic acid (2 mL) were added to 5-isopropyl-N-(7-(1-methyl-3-(pyridin-2-yl)-1H-pyrazol-4-yl)-1,5-naphthyridin-2-yl)-N-((2-(trimethylsilyl)ethoxy)methyl)-1,3,4-thiadiazole-2-amine (12 mg), followed by stirring at room temperature for 12 hours. The solvent was distilled off under reduced pressure, and the obtained residue was purified by silica gel column chromatography (methanol-ethyl acetate, NH silica), thereby obtaining 5-isopropyl-N-(7-(1-methyl-3-(pyridin-2-yl)-1H-pyrazol-4-yl)-1,5-naphthyridin-2-yl)-1,3,4-thiadiazole-2-amine (6.4 mg).
$^1$H-NMR($CDCl_3$/$CD_3OD$=4/1)δ:8.67(1H,brs),8.54(1H,d,J=3.9Hz),8.24(1H,brs),8.21(1H,d,J=8.7Hz),7.85( 1H,s),7.82-7.69( 2H,m),7.35-7.27(1H,m),7.33(1H,d,J=8.7Hz),3.47-3.36(1H,m),3.37(3H,s),1.47(6H,d,J=6.6Hz).
MSm/z(M+H):429.

<Example 0137>

<0137-1>

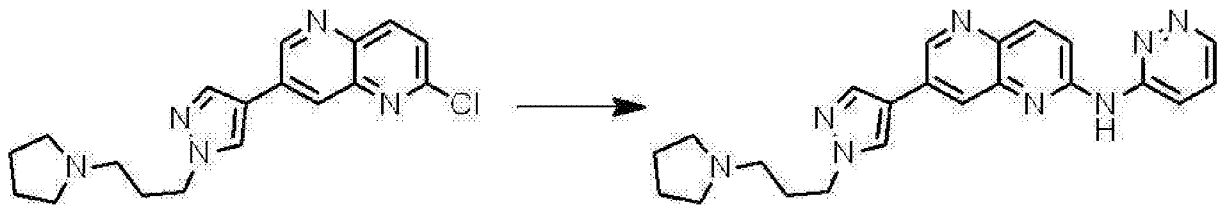

A mixture of 2-chloro-7-(1-(3-(pyrrolidin-1-yl)propyl)-1H-pyrazol-4-yl)-1,5-naphthyridine (10 mg), pyridazine-3-amine (3.3 mg), tris(dibenzylideneacetone)dipalladium(0) (3 mg), 4,5-bis(diphenylphosphino)-9,9-dimethylxanthene (4 mg), and cesium carbonate (24 mg) in 1,4-dioxane (1 mL) was stirred at 150°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining N-(pyridazin-3-yl)-7-(1-(3-(pyrrolidin-1-yl)propyl)-1H-pyrazol-4-yl)-1,5-naphthyridine-2-amine (4.4 mg).
$^1$H-NMR($CDCl_3$/$CD_3OD$=4/1)δ:9.12(1H,d,J=9.0Hz),8.89(1H,s),8.81(1H,d,J=1.8Hz),8.22(1H,s),8.21(1H, d,J=9.0Hz),8.02(1H,s),7.98(1H,s),7.61(1H,dd,J=9.0Hz,1.8Hz),7.47(1H,d,J=9.0Hz),4.33-4.25(2H,m),2.61-2.46(6H,m), 2.23-2.15(2H,m),1.86-1.77(4H,m).
MSm/z(M+H):401.

<Example 0138>

<0138-1>

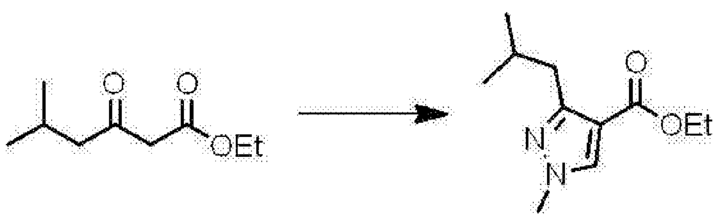

A solution of methylhydrazine (0.29 mL) and ethyl formate (0.65 mL) in ethanol (3 mL) was stirred for 4 hours under heating to reflux, and ethyl 5-methyl-3-oxohexanoate (1.91 g) was added thereto, followed by stirring at the same temperature for 4 hours. The reaction mixture was cooled to room temperature, and a 20% sodium ethoxide-ethanol solution (3 mL) was added thereto, followed by stirring for 1.5 hours under heating to reflux. The reaction mixture was cooled to room temperature, and ethyl acetate and a 3 mol/L potassium hydrogen sulfate aqueous solution were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining ethyl 3-isobutyl-1-methyl-1H-pyrazole-4-carboxylate (280 mg).
MSm/z(M+H):211.

<0138-2>

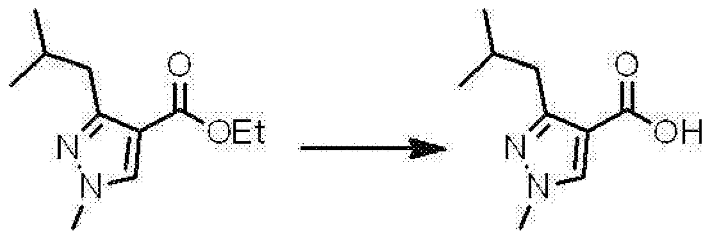

A4 mol/L sodium hydroxide aqueous solution (2 mL) was added to a solution of ethyl 3-isobutyl-1-methyl-1H-pyrazole-4-carboxylate (280 mg) in ethanol (2 mL), followed by stirring at 70°C for 30 minutes. The solvent was distilled off under reduced pressure, the resultant product was adjusted to pH 2 by the addition of a 3 mol/L potassium hydrogen sulfate aqueous solution, and ethyl acetate was added thereto. The organic layer was collected by separation, washed with a saturated sodium chloride aqueous solution, and dried over anhydrous sodium sulfate, and the solvent was distilled off under reduced pressure, thereby obtaining 3-isobutyl-1-methyl-1H-pyrazole-4-carboxylic acid (229 mg).
MSm/z(M+H):183.

<0138-3>

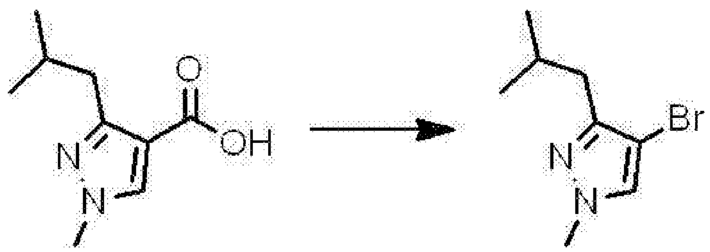

N-bromosuccinimide (260 mg) was added to a mixture of 3-isobutyl-1-methyl-1H-pyrazole-4-carboxylic acid (229 mg), and sodium hydrogen carbonate (380 mg) in N,N-dimethylformamide (6 mL), followed by stirring at room temperature for 4 hours. Ethyl acetate and water were added to the reaction mixture. The organic layer was collected by separation, washed with a saturated sodium chloride aqueous solution, and dried over anhydrous sodium sulfate, and the solvent was distilled off under reduced pressure, thereby obtaining 4-bromo-3-isobutyl-1-methyl-1H-pyrazole (146 mg).
MSm/z(M+H):217,219.

<0138-4>

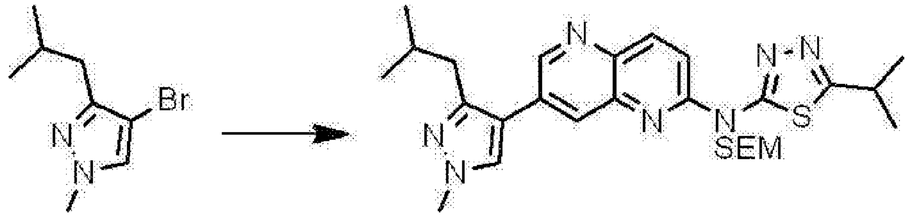

A mixture of N-(7-bromo-1,5-naphthyridin-2-yl)-5-isopropyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II) dichloride (3 mg), and potassium acetate (8 mg) in 1,4-dioxane (0.8 mL) was stirred at 100°C for 2 hours in a nitrogen atmosphere. 4-Bromo-3-isobutyl-1-methyl-1H-pyrazole (15 mg), sodium carbonate (8 mg), and bis(di-tert-butyl (4-dimethylaminophenyl)phosphine)dichloropalladium(II) (5 mg) were added to the reaction mixture, followed by stirring at 100°C for 2 hours. The reaction mixture was cooled to room temperature, the insolubles were filtered off using celite, and the solvent was distilled off under reduced pressure. The obtained residue was purified by silica gel column chromatography (methanol-ethyl acetate), thereby obtaining N-(7-(3-isobutyl-1-methyl-1H-pyrazol-4-yl)-1,5-naphthyridin-2-yl)-5-isopropyl-N-((2-(trimethylsilyl)ethoxy)methyl)-1,3,4-thiadiazole-2-amine (10 mg).
MSm/z(M+H):538.

<0138-5>

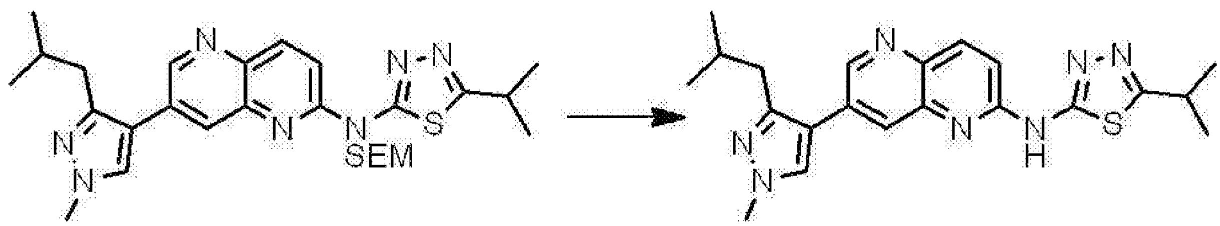

Water (0.1 mL) and trifluoroacetic acid (2 mL) were added to N-(7-(3-isobutyl-1-methyl-1H-pyrazol-4-yl)-1,5-naphthyridin-2-yl)-5-isopropyl-N-((2-(trimethylsilyl)ethoxy)methyl)-1,3,4-thiadiazole-2-amine (10 mg), followed by stirring at room temperature for 12 hours. The solvent was distilled off under reduced pressure, and the obtained residue was purified by silica gel column chromatography (methanol-ethyl acetate, NH silica), thereby obtaining N-(7-(3-isobutyl-1-methyl-1H-pyrazol-4-yl)-1,5-naphthyridin-2-yl)-5-isopropyl-1,3,4-thiadiazole-2-amine (4.5 mg).
$^1$H-NMR($CDCl_3$/$CD_3OD$=4/1)δ:8.79(1H,d,J=2.1Hz),8.24(1H,d,J=9.0Hz),8.22(1H,d,J=2.1Hz),7.74(1H,s), 7.37(1H,d,J=9.0Hz),3.96(3H,s),3.49-3.36(1H,m),2.75(2H,d,J=7.2Hz),2.07-1.92(1H,m),1.49(6H,d,J=7.2Hz),0.97(6H,d, J=7.2Hz).
MSm/z(M+H):408.

<Example 0139>

<0139-1>

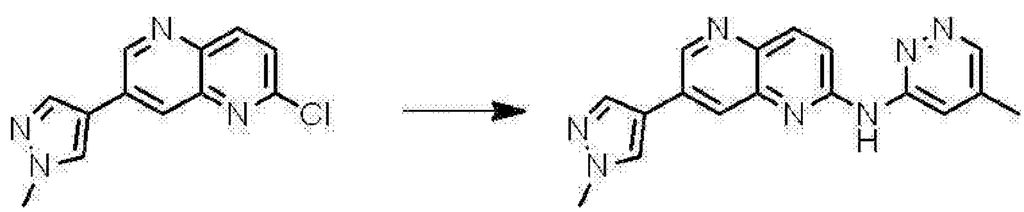

A mixture of 2-chloro-7-(1-methyl-1H-pyrazol-4-yl)-1,5-naphthyridine (10 mg), 5-methylpyridazine-3-amine (7 mg), tris(dibenzylideneacetone)dipalladium(0) (4 mg), 4,5-bis(diphenylphosphino)-9,9-dimethylxanthene (5 mg), and

cesium carbonate (33 mg) in 1,4-dioxane (1 mL) was stirred at 150°C for 30 minutes using a microwave reaction apparatus. The reaction mixture was cooled to room temperature, and ethyl acetate and water were added thereto. The solid matter was collected by filtration, thereby obtaining 7-(1-methyl-1H-pyrazol-4-yl)-N-(5-methylpyridazin-3-yl)-1,5-naphthyridine-2-amine (2.0 mg).
$^1$H-NMR(DMSO-$d_6$)δ:10.69(1H,brs),9.03(1H,d,J=2.7Hz),8.76(2H,brs),8.47(1H,brs),8.31(1H,d,J=2.7Hz),8.21(1H,d,J=9.0Hz),8.18(1H,s),7.66(1H,d,J=9.0Hz),3.92(3H,s),2.42(3H,s).
MSm/z(M+H):318.

<Example 0140>

<0140-1>

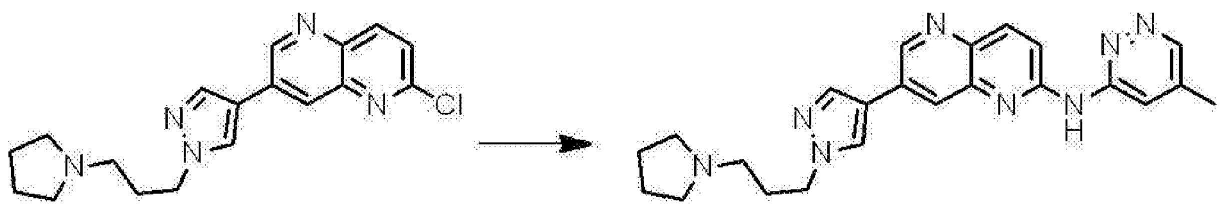

A mixture of 2-chloro-7-(1-(3-(pyrrolidin-1-yl)propyl)-1H-pyrazol-4-yl)-1,5-naphthyridine (10 mg), 5-methylpyridazine-3-amine (7 mg), tris(dibenzylideneacetone)dipalladium(0) (4 mg), 4,5-bis(diphenylphosphino)-9,9-dimethylxanthene (5 mg), and cesium carbonate (33 mg) in 1,4-dioxane (1 mL) was stirred at 150°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining N-(5-methylpyridazin-3-yl)-7-(1-(3-(pyrrolidin-1-yl)propyl)-1H-pyrazol-4-yl)-1,5-naphthyridine-2-amine (4.4 mg).
$^1$H-NMR($CDCl_3$/$CD_3OD$=4/1)δ:8.89(1H,brs),8.66(1H,brs),8.20(1H,d,J=9.0Hz),8.04(1H,s),7.99(1H,s),7.46(1H,d,J=9.0Hz),7.35(1H,brs),4.34-4.25(2H,m),3.73-3.66(2H,m),2.61-2.49(4H,m),2.50(3H,s),2.23-2.10(2H,m),1.88-1.78(4H,m).
MSm/z(M+H):415.

<Example 0141>

<0141-1>

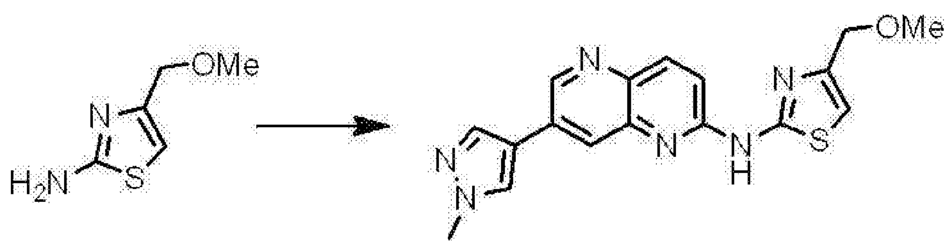

A mixture of 2-chloro-7-(1-methyl-1H-pyrazol-4-yl)-1,5-naphthyridine (10 mg), 4-(methoxymethyl)thiazole-2-amine (9 mg), tris(dibenzylideneacetone)dipalladium(0) (4 mg), 4,5-bis(diphenylphosphino)-9,9-dimethylxanthene (5 mg), and cesium carbonate (33 mg) in 1,4-dioxane (1 mL) was stirred at 150°C for 30 minutes using a microwave reaction apparatus. The reaction mixture was cooled to room temperature, the insolubles were filtered off using celite, and the obtained solution was purified by silica gel column chromatography (methanol-ethyl acetate), thereby obtaining 4-(methoxymethyl)-N-(7-(1-methyl-1H-pyrazol-4-yl)-1,5-naphthyridin-2-yl)thiazole-2-amine (5.8 mg).
$^1$H-NMR($CDCl_3$/$CD_3OD$=4/1)δ:8.86(1H,brs),8.28(1H,brs),8.17(1H,d,J=9.0Hz),7.98(1H,s),7.96(1H,s),7.27(1H,d,J=9.0Hz),6.85(1H,s),4.48(2H,s),4.02(3H,s),3.46(3H,s).

MSm/z(M+H):353.

<Example 0142>

<0142-1>

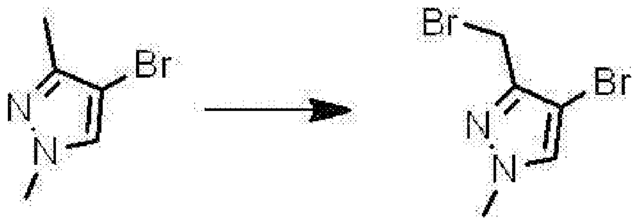

2,2'-Azobis(isobutyronitrile) (27 mg) was added to a solution of 4-bromo-1,3-dimethyl-1H-pyrazole (286 mg) and N-bromosuccinimide (320 mg) in chlorobenzene (6 mL), followed by stirring at 80°C for 7 hours. The reaction mixture was cooled to room temperature, and ethyl acetate and a saturated sodium hydrogen carbonate aqueous solution were added thereto. The organic layer was collected by separation, washed with a saturated sodium chloride aqueous solution, and dried over anhydrous sodium sulfate, and the solvent was distilled off under reduced pressure, thereby obtaining 4-bromo-3-(bromomethyl)-1-methyl-1H-pyrazole (465 mg) as a white solid.
MSm/z(M+H):253.

<0142-2>

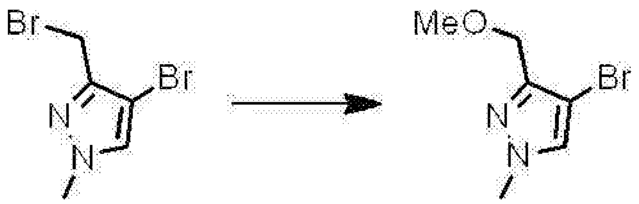

A 28% sodium methoxide-methanol solution (2 mL) was added to a solution of 4-bromo-3-(bromomethyl)-1-methyl-1H-pyrazole (84 mg) in methanol (2 mL), followed by stirring at room temperature for 3 hours. The solvent was distilled off under reduced pressure, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining 4-bromo-3-(methoxymethyl)-1-methyl-1H-pyrazole (42 mg).
MSm/z(M+H):205.

<0142-3>

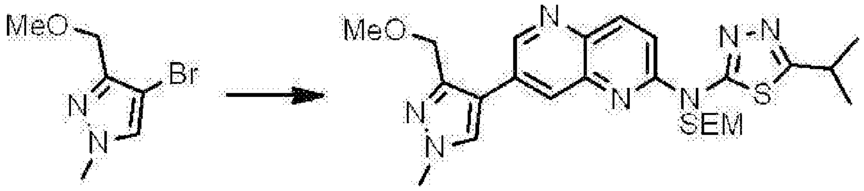

A mixture of N-(7-bromo-1,5-naphthyridin-2-yl)-5-isopropyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II) dichloride (3 mg), and potassium acetate (8 mg) in 1,4-dioxane (0.8 mL) was stirred at 100°C for 3 hours in a nitrogen atmosphere. 4-Bromo-3-(methoxymethyl)-1-methyl-1H-pyrazole (15 mg), sodium carbonate (8 mg), and bis(di-tert-butyl (4-dimethylaminophenyl)phosphine)dichloropalladium(II) (5 mg) were added to the reaction mixture, followed by stirring at 100°C for 4 hours. The reaction mixture was cooled to room temperature, the insolubles were filtered off using celite, and the solvent was distilled off under reduced pressure. The obtained residue was purified by silica gel column chromatography (methanol-ethyl acetate), thereby obtaining 5-isopropyl-N-(7-(3-(methoxymethyl)-1-methyl-1H-pyrazol-4-yl)-1,5-naphthyridin-2-yl)-N-((2-(trimethylsilyl)ethoxy)methyl)-1,3,4-thiadiazole-2-amine (8.2 mg).
MSm/z(M+H):526.

<0142-4>

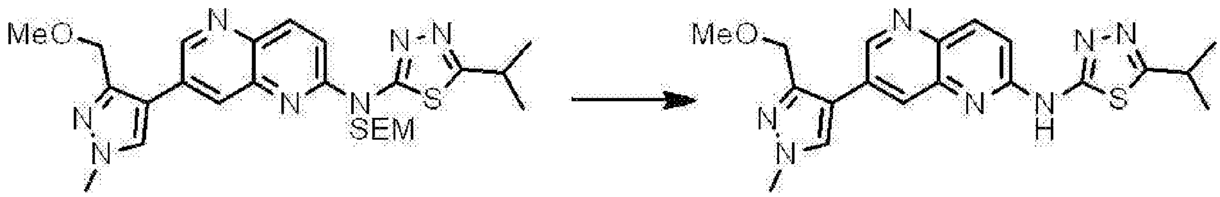

Water (0.1 mL) and trifluoroacetic acid (2 mL) were added to 5-isopropyl-N-(7-(3-(methoxymethyl)-1-methyl-1H-pyrazol-4-yl)-1,5-naphthyridin-2-yl)-N-((2-(trimethylsilyl)ethoxy)methyl)-1,3,4-thiadiazole-2-amine (8.2 mg), followed by stirring at room temperature for 30 minutes. The solvent was distilled off under reduced pressure, and the obtained residue was purified by silica gel column chromatography (methanol-ethyl acetate, NH silica), thereby obtaining 5-isopropyl-N-(7-(3-(methoxymethyl)-1-methyl-1H-pyrazol-4-yl)-1,5-naphthyridin-2-yl)-1,3,4-thiadiazole-2-amine (3.4 mg).
$^1$H-NMR($CDCl_3$/$CD_3OD$=4/1)δ:8.89(1H,d,J=2.1Hz),8.42(1H,d,J=2.1Hz),8.24(1H,d,J=8.4Hz), 7.86(1H,s),7.35(1H,d,J=8.4Hz),4.58(2H,s),3.58(3H,s),3.48-3.37(1H,m),3.36(3H,s),1.48(6H,d,J=6.6Hz).
MSm/z(M+H):396.

<Example 0143>

<0143-1>

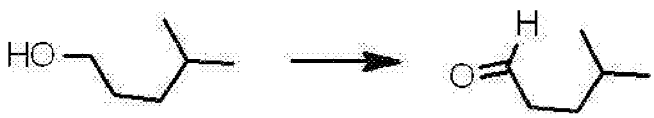

Tetrapropylammoniumperruthenate (171 mg) was added to a mixture of 4-methylpentan-1-ol (1.0 g), molecular sieve 4A(1.0 g), and N-methylmorpholine-N-oxide (1.26 g) in dichloromethane (30 mL) under ice-cooling, followed by stirring at the same temperature for 5 minutes. The reaction mixture was purified by silica gel column chromatography (dichloromethane), thereby obtaining 4-methylpentanal (2.79 g).
$^1$H-NMR($CDCl_3$)δ:9.77(1H,t,J=2.1Hz),2.43(2H,td,J=5.1Hz,2.1Hz), 1.66-1.48(1H,m),1.28-1.18(2H,m),0.91(6H,d,J=6.0Hz).

<0143-2>

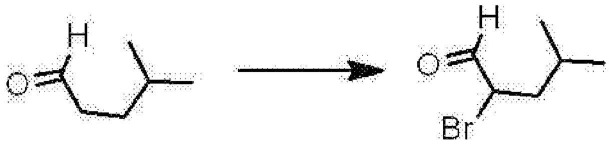

Trimethylphenylammonium tribromide (4.0 g) was added to a solution of 4-methylpentanal (2.79 g) in tetrahydrofuran (30 mL) under ice-cooling, followed by stirring while slowly heating to room temperature for 1 day. Ethyl acetate and water were added to the reaction mixture. The organic layer was collected by separation, washed sequentially with a 10% sodium hydrogen sulfite aqueous solution and a saturated sodium chloride aqueous solution, and dried over anhydrous sodium sulfate, and the solvent was distilled off under reduced pressure, thereby obtaining 2-bromo-4-methylpentanal (2.84 g).
$^1$H-NMR($CDCl_3$)δ:9.13(1H,s),3.46(1H,t.J=6.6Hz),1.80-1.46(3H,m),1.04(6H,d,J=6.6Hz).

<0143-3>

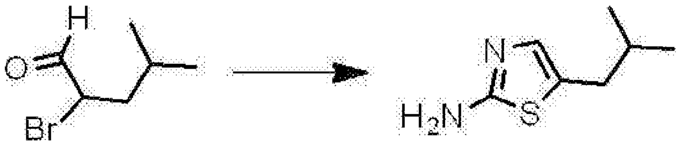

Thiourea (744 mg) was added to a solution of 2-bromo-4-methylpentanal (2.84 g) in ethanol (10 mL), followed by stirring for 3 hours under heating to reflux. The reaction mixture was cooled to room temperature, and ethyl acetate and water were added thereto. The organic layer was collected by separation, washed sequentially with a saturated sodium hydrogen carbonate aqueous solution and a saturated sodium chloride aqueous solution, and dried over anhydrous sodium sulfate, and the solvent was distilled off under reduced pressure. The obtained residue was purified by silica gel column chromatography (methanol-ethyl acetate), thereby obtaining 5-isobutylthiazole-2-amine (449 mg).
MSm/z(M+H):157.

<0143-4>

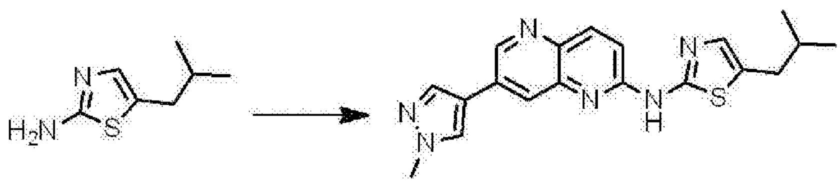

A mixture of 2-chloro-7-(1-methyl-1H-pyrazol-4-yl)-1,5-naphthyridine (10 mg), 5-isobutylthiazole-2-amine (10 mg), tris(dibenzylideneacetone)dipalladium(0) (4 mg), 4,5-bis(diphenylphosphino)-9,9-dimethylxanthene (5 mg), and cesium carbonate (33 mg) in 1,4-dioxane (1 mL) was stirred at 150°C for 30 minutes using a microwave reaction apparatus. The reaction mixture was cooled to room temperature, the insolubles were filtered off using celite, and the obtained solution was purified by silica gel column chromatography (methanol-ethyl acetate), thereby obtaining 5-isobutyl-N-(7-(1-methyl-1H-pyrazol-4-yl)-1,5-naphthyridin-2-yl)thiazole-2-amine (7.7 mg).
$^1$H-NMR($CDCl_3$/$CD_3OD$=4/1)δ:8.85(1H,brs),8.26(1H,brs),8.15(1H,d,J=9.0Hz),7.99(1H,s),7.93(1H,s),7.2 4(1H,d,J=9.0Hz),7.07(1H,s),4.02(3H,s),2.68(2H,d,J=7.5Hz),2.02-1.90(1H,m),1.01(6H,d,J=6.36Hz).
MSm/z(M+H):365.

<Example 0144>

<0144-1>

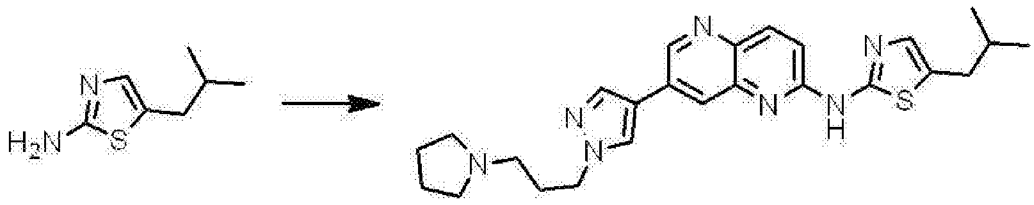

A mixture of 2-chloro-7-(1-(3-(pyrrolidin-1-yl)propyl)-1H-pyrazol-4-yl)-1,5-naphthyridine (10 mg), 5-isobutylthiazole-2-amine (10 mg), tris(dibenzylideneacetone)dipalladium(0) (4 mg), 4,5-bis(diphenylphosphino)-9,9-dimethylxanthene (5 mg), and cesium carbonate (33 mg) in 1,4-dioxane (1 mL) was stirred at 150°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining 5-isobutyl-N-(7-(1-(3-(pyrrolidin-1-yl)propyl)-1H-pyrazol-4-yl)-1,5-naphthyridin-2-yl)thiazole-2-amine (6.2 g).
$^1$H-NMR($CDCl_3$/$CD_3OD$=4/1)$\delta$:8.86(1H,brs),8.27(1H,brs),8.15(1H,d,J=8.4Hz),8.00(2H,s),7.24(1H,d,J=8.4Hz),7.07(1H,s),4.29(2H,t,J=6.6Hz),2.68(2H,d,J=6.6Hz),2.59-2.47(6H,m),2.22-2.11(2H,m),2.02-1.91(1H,m),1.86-1.78(4H,m),1.01(6H,d,J=6.6Hz).
MSm/z(M+H):462.

<Example 0145>

<0145-1>

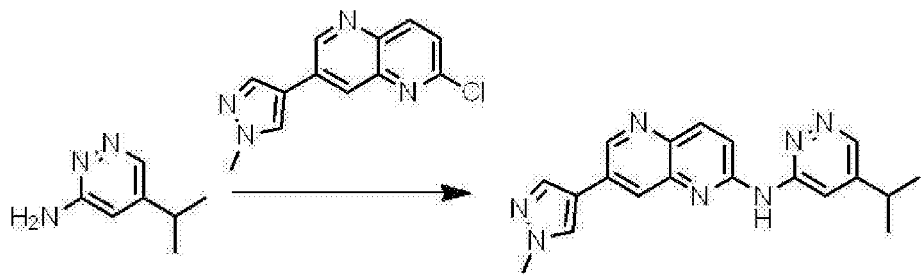

A mixture of 2-chloro-7-(1-methyl-1H-pyrazol-4-yl)-1,5-naphthyridine (10 mg), 5-isopropylpyridazine-3-amine (7 mg), tris(dibenzylideneacetone)dipalladium(0) (4 mg), 4,5-bis(diphenylphosphino)-9,9-dimethylxanthene (5 mg), and cesium carbonate (33 mg) in 1,4-dioxane (1 mL) was stirred at 140°C for 30 minutes using a microwave reaction apparatus. The reaction mixture was cooled to room temperature, and ethyl acetate and water were added thereto. The solid matter was collected by filtration, thereby obtaining N-(5-isopropylpyridazin-3-yl)-7-(1-methyl-1H-pyrazol-4-yl)-1,5-naphthyridine-2-amine (9.3 mg).
$^1$H-NMR(DMSO-$d_6$)$\delta$:10.72(1H,brs),9.04(1H,d,J=1.8Hz),8.87(1H,d,J=1.8Hz),8.72(1H,brs),8.46(1H,s),8.23-8.19(2H,m),8.17(1H,s),7.70(1H,d,J=9.0Hz),3.92(3H,s),3.11-2.98(1H,m),1.33(6H,d,J=6.6Hz).
MSm/z(M+H):346.

<Example 0146>

<0146-1>

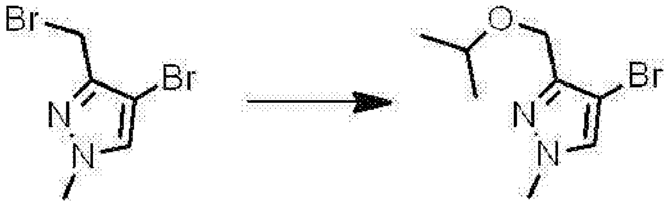

60% sodium hydride (23 mg) was added to a mixture solution of 4-bromo-3-(bromomethyl)-1-methyl-1H-pyrazole (72 mg) in 1,4-dioxane (0.5 mL) and 2-propanol (0.5 mL), followed by stirring at 120°C for 5 hours. The reaction mixture was cooled to room temperature, and ethyl acetate and water were added thereto. The organic layer was collected by separation, washed sequentially with a saturated sodium hydrogen carbonate aqueous solution and a saturated sodium chloride aqueous solution, and dried over anhydrous sodium sulfate, and the solvent was distilled off under reduced pressure, thereby obtaining 4-bromo-3-(isopropyloxymethyl)-1-methyl-1H-pyrazole (57 mg).
$^1$H-NMR($CDCl_3$)δ:7.34(1H,s),4.46(2H,s),3.85(3H,s),3.78-3.68(1H,m),1.22(6H,d,J=6.6Hz).

<0146-2>

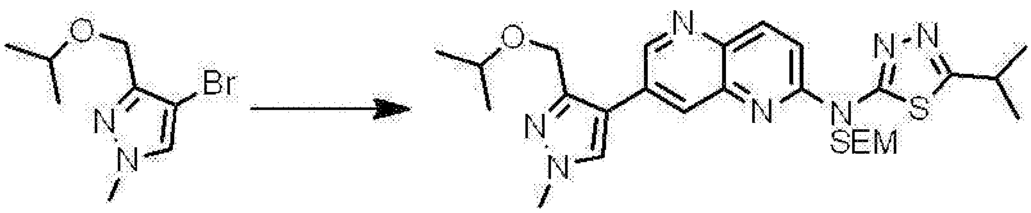

A mixture of N-(7-bromo-1,5-naphthyridin-2-yl)-5-isopropyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II) dichloride (3 mg), and potassium acetate (8 mg) in 1,4-dioxane (0.8 mL) was stirred at 100°C for 3 hours in a nitrogen atmosphere. 4-Bromo-3-(isopropyloxymethyl)-1-methyl-1H-pyrazole (15 mg), sodium carbonate (8 mg), and bis(di-tert-butyl (4-dimethylaminophenyl)phosphine)dichloropalladium(II) (5 mg) were added to the reaction mixture, followed by stirring at 100°C for 3 hours. The reaction mixture was cooled to room temperature, the insolubles were filtered off using celite, and the solvent was distilled off under reduced pressure. The obtained residue was purified by silica gel column chromatography (methanol-ethyl acetate), thereby obtaining N-(7-(3-(isopropyloxymethyl)-1-methyl-1H-pyrazol-4-yl)-1,5-naphthyridin-2-yl)-5-isopropyl-N-((2-(trimethylsilyl)ethoxy)methyl)-1,3,4-thiadiazole-2-amine (6.8 mg).
MSm/z(M+H):554.

<0146-3>

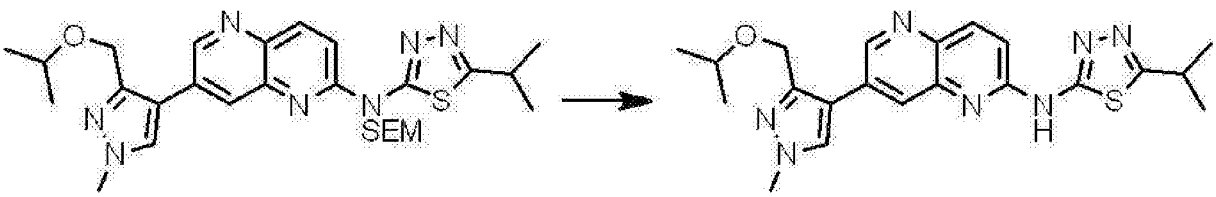

Water (0.1 mL) and trifluoroacetic acid (2 mL) were added to N-(7-(3-(isopropyloxymethyl)-1-methyl-1H-pyrazol-4-yl)-1,5-naphthyridin-2-yl)-5-isopropyl-N-((2-(trimethylsilyl)ethoxy)methyl)-1,3,4-thiadiazole-2-amine (6.8 mg), followed by stirring at room temperature for 30 minutes. The solvent was distilled off under reduced pressure, and the obtained residue was purified by silica gel column chromatography (methanol-ethyl acetate, NH silica), thereby obtaining N-(7-(3-(isopropyloxymethyl)-1-methyl-1H-pyrazol-4-yl)-1,5-naphthyridin-2-yl)-5-isopropyl-1,3,4-thiadiazole-2-amine (3.0 mg).
$^1$H-NMR($CDCl_3$/$CD_3OD$=4/1)δ:8.88(1H,brs),8.52(1H,brs),8.24(1H,d,J=9.0Hz),7.85(1H,s),7.37(1H,d,J=9.

0Hz),4.61(2H,s),4.00(3H,s),3.97-3.88(1H,m),3.47-3.36(1H,m),1.48(6H,d,J=6.6Hz),1.37(6H,d,J=6.0Hz).
MSm/z(M+H):424.

<Example 0147>

<0147-1>

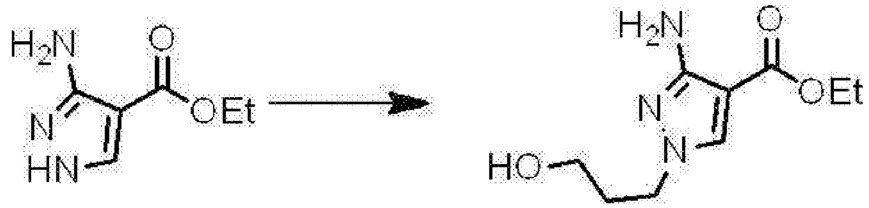

After a mixture of ethyl 3-amino-1H-pyrazole-4-carboxylate (2.00 g), trioctylmethylammonium chloride (0.31 mg), and potassium carbonate (3.25 g) in toluene (25 mL) was stirred for 15 minutes under heating to reflux, 3-bromopropanol (1.45 mL) was added to the mixture, followed by stirring at the same temperature for 7 hours. The reaction mixture was cooled to room temperature, and ethyl acetate and water were added thereto. The organic layer was collected by separation, washed sequentially with a saturated sodium hydrogen carbonate aqueous solution and a saturated sodium chloride aqueous solution, and dried over anhydrous sodium sulfate, and the solvent was distilled off under reduced pressure, thereby obtaining ethyl 3-amino-1-(3-hydroxypropyl)-1H-pyrazole-4-carboxylate (3.01 g).
MSm/z(M+H):214.

<0147-2>

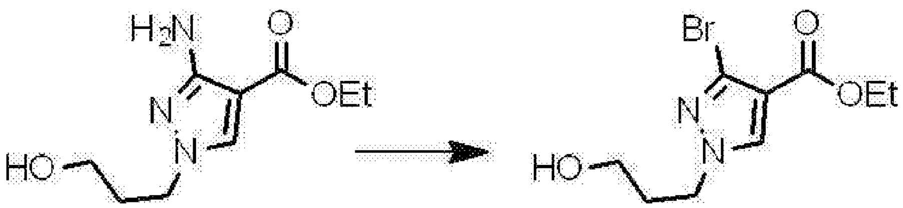

tert-Butyl nitrite (2.12 mL) was added to a mixture of copper(II) bromide (3.71 g) in acetonitrile (30 mL) under ice-cooling, followed by stirring at the same temperature for 5 minutes, and ethyl 3-amino-1-(3-hydroxypropyl)-1H-pyrazole-4-carboxylate (3.01 g) was added thereto, followed by stirring at room temperature for 5 hours. Ethyl acetate and water were added to the reaction mixture. The organic layer was collected by separation, washed sequentially with a saturated sodium hydrogen carbonate aqueous solution and a saturated sodium chloride aqueous solution, and dried over anhydrous sodium sulfate, and the solvent was distilled off under reduced pressure. The obtained residue was purified by silica gel column chromatography (methanol-ethyl acetate), thereby obtaining ethyl 3-bromo-1-(3-hydroxypropyl)-1H-pyrazole-4-carboxylate (1.58 g).
MSm/z(M+H):277.

<0147-3>

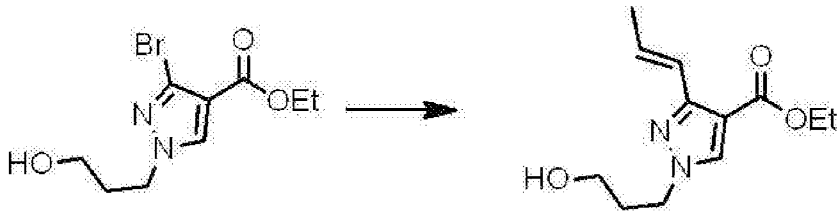

A mixture of ethyl 3-bromo-1-(3-hydroxypropyl)-1H-pyrazole-4-carboxylate (383 mg), (E)-4,4,5,5-tetramethyl-2-(prop-1-en-1-yl)-1,3,2-dioxaborolane (278 mg), sodium carbonate (366 mg), bis(di-tert-butyl (4-dimethylaminophenyl)phosphine)dichloropalladium(II) (97 mg), and water (1 mL) in 1,4-dioxane (10 mL) was stirred for 2 hours under heating to reflux. The reaction mixture was cooled to room temperature, the insolubles were filtered off using celite, and the solvent was distilled off under reduced pressure. The obtained residue was purified by silica gel column chromatography (ethyl acetate-hexane), thereby obtaining (E)-ethyl 1-(3-hydroxypropyl)-3-(prop-1-en-1-yl)-1H-pyrazole-4-carboxylate (236 mg).
MSm/z(M+H):239.

<0147-4>

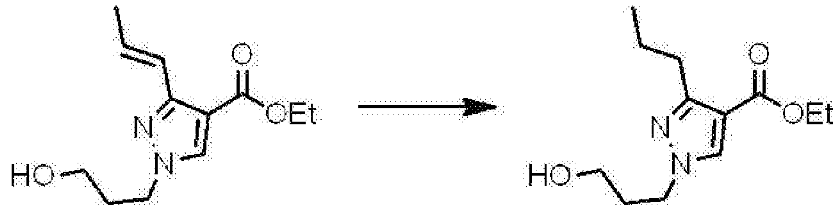

(E)-ethyl 1-(3-hydroxypropyl)-3-(prop-1-en-1-yl)-1H-pyrazole-4-carboxylate (236 mg) was added to a mixture of 10% palladium-carbon (50 mg) in methanol (10 mL), followed by stirring for 1.5 hours in a hydrogen atmosphere. The insolubles were filtered off using celite, and the solvent was distilled off under reduced pressure, thereby obtaining ethyl 1-(3-hydroxypropyl)-3-propyl-1H-pyrazole-4-carboxylate (206 mg).
MSm/z(M+H):241.

<0147-5>

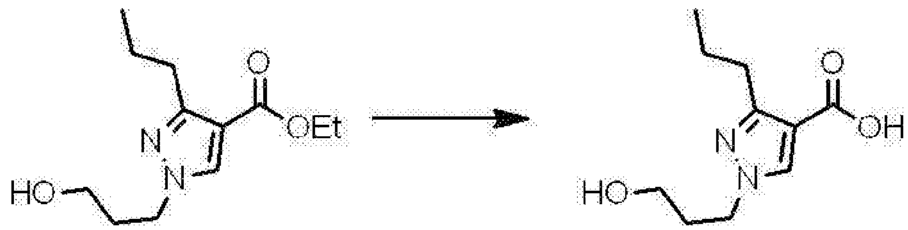

A4 mol/L sodium hydroxide aqueous solution (2 mL) was added to a solution of ethyl 1-(3-hydroxypropyl)-3-propyl-1H-pyrazole-4-carboxylate (206 mg) in ethanol (2 mL), followed by stirring at 70°C for 1 hour. After the reaction mixture was cooled to room temperature, the solvent was distilled off under reduced pressure, the resultant product was adjusted to pH 2 by the addition of a 3 mol/L potassium hydrogen sulfate aqueous solution, and ethyl acetate was added thereto. The organic layer was collected by separation, washed with a saturated sodium chloride aqueous solution, and dried over anhydrous sodium sulfate, and the solvent was distilled off under reduced pressure, thereby obtaining 1-(3-hydroxypropyl)-3-propyl-1H-pyrazole-4-carboxylic acid (171 mg).
MSm/z(M+H):213.

<0147-6>

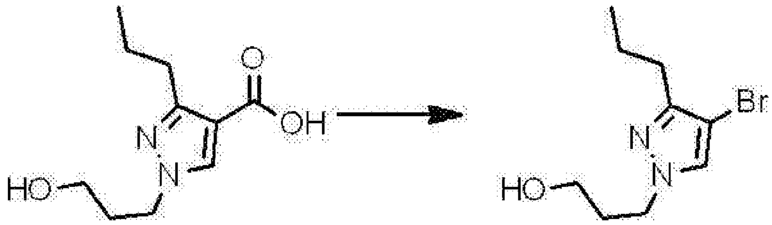

N-bromosuccinimide (158 mg) was added to a mixture of 1-(3-hydroxypropyl)-3-propyl-1H-pyrazole-4-carboxylic acid (206 mg), and sodium hydrogen carbonate (291 mg) in N,N-dimethylformamide (4 mL), followed by stirring at room temperature for 2.5 hours. Ethyl acetate and water were added to the reaction mixture. The organic layer was collected by separation, washed with a saturated sodium chloride aqueous solution, and dried over anhydrous sodium sulfate, and the solvent was distilled off under reduced pressure, thereby obtaining 3-(4-bromo-3-propyl-1H-pyrazol-1-yl)propan-1-ol (146 mg).
$^1$H-NMR($CDCl_3$)$\delta$:7.34(1H,s),4.20(2H,t,J=6.6Hz),2.56(2H,t,J=6.6Hz),3.67-3.56(2H,m),2.06-1.96(2H,m),1.75-1.61(2H,m), 0.95(3H,t,J=7.2Hz).

<0147-7>

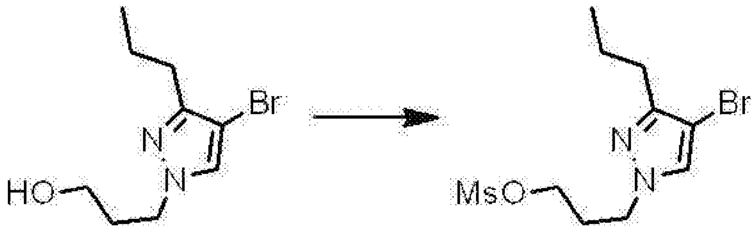

Methanesulfonyl chloride (0.043 mL) was added to a solution of 3-(4-bromo-3-propyl-1H-pyrazol-1-yl)propan-1-ol (90 mg) and triethylamine (0.10 mL) in dichloromethane (3 mL) under ice-cooling, followed by stirring at the same temperature for 30 minutes. Ethyl acetate and water were added to the reaction mixture. The organic layer was collected by separation, washed with a saturated sodium chloride aqueous solution, and dried over anhydrous sodium sulfate, and the solvent was distilled off under reduced pressure, thereby obtaining 3-(4-bromo-3-propyl-1H-pyrazol-1-yl)propyl methanesulfonate (136 mg).
$^1$H-NMR($CDCl_3$)$\delta$:7.35(1H,s),4.27-4.08(4H,m),3.03(3H,s),2.56(2H,t,J=7.2Hz),2.34-2.23(2H,m),1.74-1.59(2H,m),0.95(3H,t ,J=7.2Hz).

<0147-8>

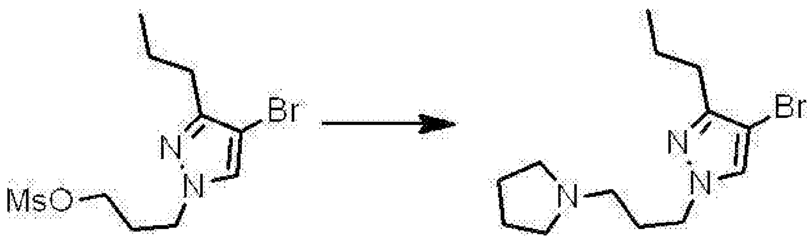

Pyrrolidine (0.037 mL) was added to a mixture of 3-(4-bromo-3-propyl-1H-pyrazol-1-yl)propyl methanesulfonate (136 mg), and potassium carbonate (127 mg) in acetonitrile (2 mL), followed by stirring at 50°C for 10 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining 4-bromo-3-propyl-1-(3-(pyrrolidin-1-yl)propyl)-1H-pyrazole (63 mg).

$^1$H-NMR($CDCl_3$)δ:7.33(1H,s),4.10(2H,t,J=6.6Hz),2.56(2H,t,J=7.2Hz),2.50-2.43(4H,m),2.40(2H,t,J=7.5Hz),2.06-1.96(2H,m),1.82-1.73(4H,m),1.72-1.60(2H,m),0.95(3H,t,J=7.2Hz).

<0147-9>

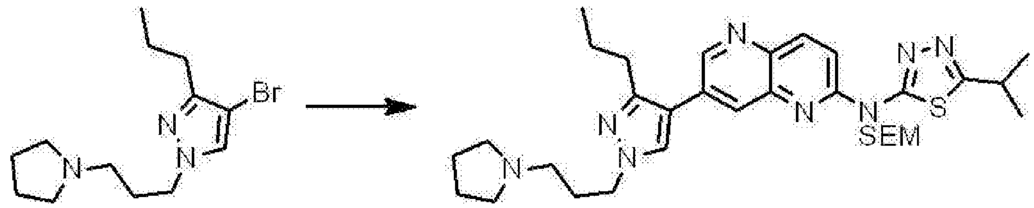

A mixture of N-(7-bromo-1,5-naphthyridin-2-yl)-5-isopropyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II) dichloride (3 mg), and potassium acetate (8 mg) in 1,4-dioxane (0.8 mL) was stirred at 100°C for 2 hours in a nitrogen atmosphere. 4-Bromo-3-propyl-1-(3-(pyrrolidin-1-yl)propyl)-1H-pyrazole (15 mg), sodium carbonate (8 mg), and bis(di-tert-butyl (4-dimethylaminophenyl)phosphine)dichloropalladium(II) (5 mg) were added to the reaction mixture, followed by stirring at 100°C for 3 hours. The reaction mixture was cooled to room temperature, the insolubles were filtered off using celite, and the solvent was distilled off under reduced pressure. The obtained residue was purified by silica gel column chromatography (methanol-ethyl acetate, NH silica), thereby obtaining 5-isopropyl-N-(7-(3-propyl-1-(3-(pyrrolidin-1-yl)propyl)-1H-pyrazol-4-yl)-1,5-naphthyridin-2-yl)-N-((2-(trimethylsilyl)ethoxy)methyl)-1,3,4-thiadiazole-2-amine (11 mg).
MSm/z(M+H):621.

<0147-10>

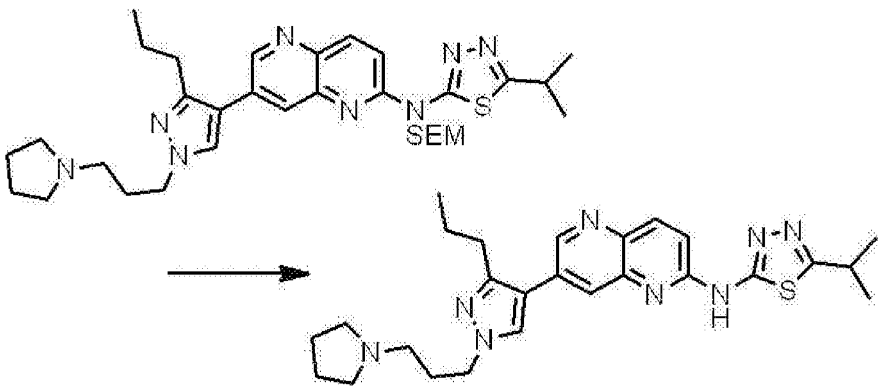

Water (0.1 mL) and trifluoroacetic acid (2 mL) were added to 5-isopropyl-N-(7-(3-propyl-1-(3-(pyrrolidin-1-yl)propyl)-1H-pyrazol-4-yl)-1,5-naphthyridin-2-yl)-N-((2-(trimethylsilyl)ethoxy)methyl)-1,3,4-thiadiazole-2-amine (11 mg), followed by stirring at room temperature for 2.5 hours. The solvent was distilled off under reduced pressure, and the obtained residue was purified by silica gel column chromatography (methanol-ethyl acetate, NH silica), thereby obtaining 5-isopropyl-N-(7-(3-propyl-1-(3-(pyrrolidin-1-yl)propyl)-1H-pyrazol-4-yl)-1,5-naphthyridin-2-yl)-1,3,4-thiadiazole-2-amine (1.7 mg).

$^1$H-NMR($CDCl_3$/$CD_3OD$=4/1)δ:8.80(1H,brs),8.24(1H,J=9.0Hz),8.21(1H,brs),7.80(1H,s),7.36(1H,d,J=9.0

Hz),4.27-4.18(2H,m),3.78-3.64(2H,m),3.49-3.36(1H,m),2.92-2.81(2H,m),2.63-2.48(4H,m),2.22-2.08(2H,m),1.87-1.66(6H,m),1.49(6H,d,J=6.0Hz),1.10-0.96(3H,m).
MSm/z(M+H):491.

<Example 0148>

<0148-1>

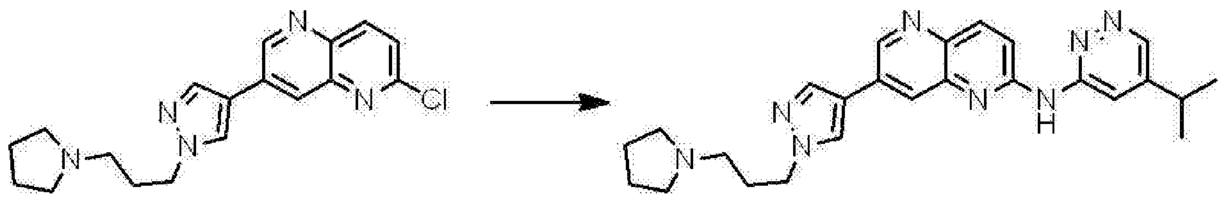

A mixture of 2-chloro-7-(1-(3-(pyrrolidin-1-yl)propyl)-1H-pyrazol-4-yl)-1,5-naphthyridine (10 mg), 5-isopropylpyridazine-3-amine (9 mg), tris(dibenzylideneacetone)dipalladium(0) (4 mg), 4,5-bis(diphenylphosphino)-9,9-dimethylxanthene (5 mg), and cesium carbonate (33 mg) in 1,4-dioxane (1 mL) was stirred at 140°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining N-(5-isopropylpyridazin-3-yl)-7-(1-(3-(pyrrolidin-1-yl)propyl)-1H-pyrazol-4-yl)-1,5-naphthyridine-2-amine (5.4 mg).
$^1$H-NMR($CDCl_3$/$CD_3OD$=4/1)δ:8.89(2H,brs),8.72(1H,brs),8.20(1H,d,J=9.0Hz),8.15(1H,brs),8.02(1H,s),7.98(1H,s),7.51(1H.d.J=9.0Hz),4.33-4.26(2H,m),3.14-3.00(1H,m),2.60-2.48(6H,m),2.24-2.10(2H,m),1.86-1.78(4H,m),1.42(6H,d,J=6.6Hz).
MSm/z(M+H):443.

<Example 0149>

<0149-1>

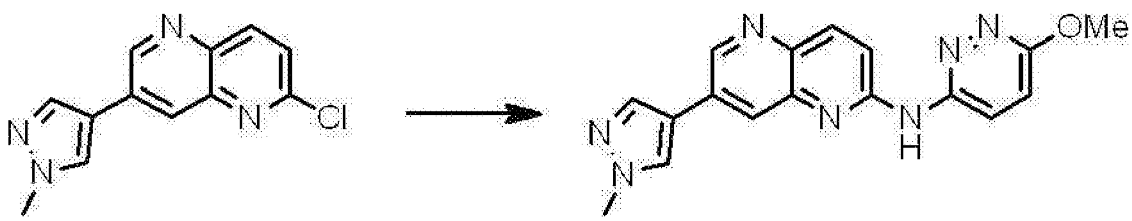

A mixture of 2-chloro-7-(1-methyl-1H-pyrazol-4-yl)-1,5-naphthyridine (10 mg), 6-methoxypyridazine-3-amine (10 mg), tris(dibenzylideneacetone)dipalladium(0) (4 mg), 4,5-bis(diphenylphosphino)-9,9-dimethylxanthene (5 mg), and cesium carbonate (33 mg) in 1,4-dioxane (1 mL) was stirred at 140°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining N-(6-methoxypyridazin-3-yl)-7-(1-methyl-1H-pyrazol-4-yl)-1,5-naphthyridine-2-amine (2.3 mg).
$^1$H-NMR($CDCl_3$/$CD_3OD$=4/1)δ:8.84(1H,d,J=2.1Hz),8.17(1H,d,J=2.1Hz),8.16(1H,d,J=8.7Hz),7.96(1H,s),7.94(1H,s),7.48(1H,d,J=8.7Hz),7.38(1H,d,9.3Hz),7.14(1H,d,J=9.3Hz),6.86(3H,s),3.97(3H,s).
MSm/z(M+H):334.

<Example 0150>

<0150-1>

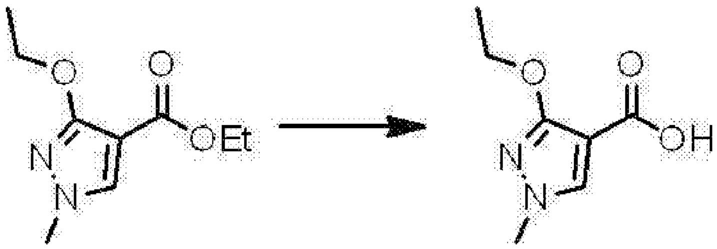

A 4 mol/L sodium hydroxide aqueous solution (1 mL) was added to a solution of ethyl 3-ethoxy-1-methyl-1H-pyrazole-4-carboxylate (25 mg) in ethanol (1 mL), followed by stirring at 70°C for 2 hours. The solvent was distilled off under reduced pressure, the resultant product was adjusted to pH 2 by the addition of a 3 mol/L potassium hydrogen sulfate aqueous solution, and ethyl acetate was added thereto. The organic layer was collected by separation, washed with a saturated sodium chloride aqueous solution, and dried over anhydrous sodium sulfate, and the solvent was distilled off under reduced pressure, thereby obtaining 3-ethoxy-1-methyl-1H-pyrazole-4-carboxylic acid (18 mg).
MSm/z(M+H):171.

<0150-2>

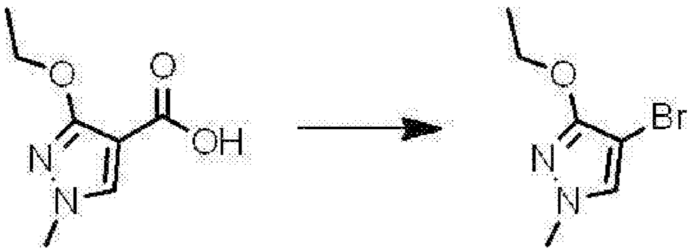

N-bromosuccinimide (21 mg) was added to 3-ethoxy-1-methyl-1H-pyrazole-4-carboxylic acid (18 mg) and sodium hydrogen carbonate (38 mg) in N,N-dimethylformamide (1 mL), followed by stirring at room temperature for 19 hours. Ethyl acetate and water were added to the reaction mixture. The organic layer was collected by separation, washed with a saturated sodium chloride aqueous solution, and dried over anhydrous sodium sulfate, and the solvent was distilled off under reduced pressure, thereby obtaining 4-bromo-3-ethoxy-1H-pyrazole (18 mg).
MSm/z(M+H):205.

<0150-3>

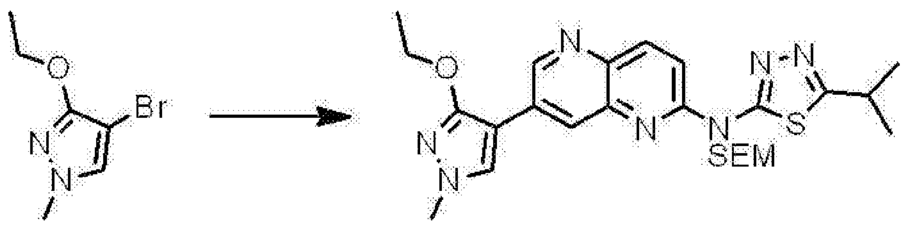

A mixture of N-(7-bromo-1,5-naphthyridin-2-yl)-5-isopropyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II) dichloride (3

mg), and potassium acetate (8 mg) in 1,4-dioxane (0.8 mL) was stirred at 100°C for 2 hours in a nitrogen atmosphere. 4-Bromo-3-ethoxy-1H-pyrazole (15 mg), sodium carbonate (8 mg), and bis(di-tert-butyl (4-dimethylaminophenyl)phosphine)dichloropalladium(II) (5 mg) were added to the reaction mixture, followed by reacting at 100°C for 2 hours. The reaction mixture was cooled to room temperature, the insolubles were filtered off using celite, and the solvent was distilled off under reduced pressure. The obtained residue was purified by silica gel column chromatography (methanol-ethyl acetate), thereby obtaining N-(7-(3-ethoxy-1-methyl-1H-pyrazol-4-yl)-1,5-naphthyridin-2-yl)-5-isopropyl-N-((2-(trimethylsilyl)ethoxy)methyl)-1,3,4-thiadiazole-2-amine (4.6 mg).
MSm/z(M+H):526.

<0150-4>

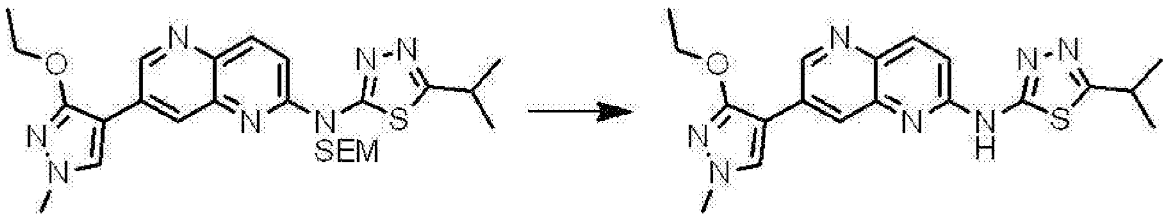

Water (0.1 mL) and trifluoroacetic acid (2 mL) were added to N-(7-(3-ethoxy-1-methyl-1H-pyrazol-4-yl)-1,5-naphthyridin-2-yl)-5-isopropyl-N-((2-(trimethylsilyl)ethoxy)methyl)-1,3,4-thiadiazole-2-amine (4.6 mg), followed by stirring at room temperature for 30 minutes. The solvent was distilled off under reduced pressure, and the obtained residue was purified by silica gel column chromatography (methanol-ethyl acetate, NH silica), thereby obtaining N-(7-(3-ethoxy-1-methyl-1H-pyrazol-4-yl)-1,5-naphthyridin-2-yl)-5-isopropyl-1,3,4-thiadiazole-2-amine (1.9 mg).
$^1$H-NMR($CDCl_3$/$CD_3OD$=4/1)δ:8.97(1H,d,J=2.1Hz),8.35(1H,d,J=2.1Hz),8.23(1H,d,J=9.0Hz),7.84(1H,s), 7.33(1H,d,J9.0Hz),4.13(2H,q,6.6Hz),3.82(3H,s),3.49-3.37(1H,m),1.50(6H,d,J=6.6Hz),1.47(3H,t,J=6.6Hz).
MSm/z(M+H):396.

<Example 0151>

<0151-1>

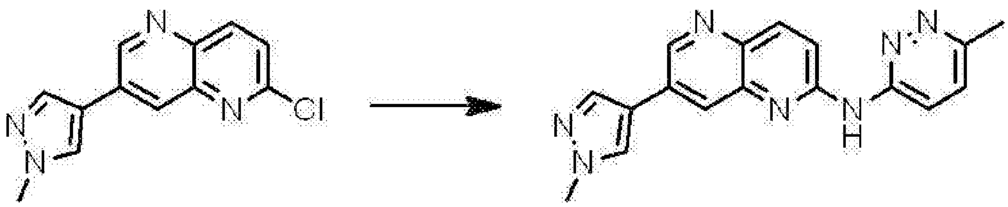

A mixture of 2-chloro-7-(1-methyl-1H-pyrazol-4-yl)-1,5-naphthyridine (10 mg), 6-methylpyridazine-3-amine (10 mg), tris(dibenzylideneacetone)dipalladium(0) (4 mg), 4,5-bis(diphenylphosphino)-9,9-dimethylxanthene (5 mg), and cesium carbonate (33 mg) in 1,4-dioxane (1 mL) was stirred at 140°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining 7-(1-methyl-1H-pyrazol-4-yl)-N-(6-methylpyridazin-3-yl)-1,5-naphthyridine-2-amine (1.1 mg).
$^1$H-NMR($CDCl_3$/$CD_3OD$=4/1)δ:8.86(1H,d,J=1.8Hz),8.19(1H,d,J=1.8Hz),8.17(1H,d,J=9.0Hz),7.96 (1H,s),7.95(1H,s),7.47(1H,d,J=9.0Hz),7.15(1H,d,J=9.0Hz),6.83(1H,d,J=9.0Hz),4.06(3H,s),2.49(3H,s).
MSm/z(M+H):318.

<Example 0152>

<0152-1>

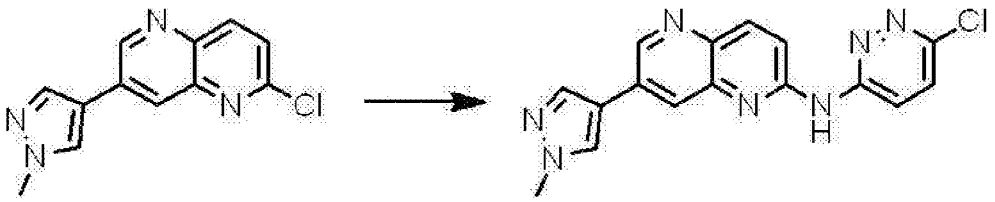

A mixture of 2-chloro-7-(1-methyl-1H-pyrazol-4-yl)-1,5-naphthyridine (10 mg), 6-chloropyridazine-3-amine (10 mg), tris(dibenzylideneacetone)dipalladium(0) (4 mg), 4,5-bis(diphenylphosphino)-9,9-dimethylxanthene (5 mg), and cesium carbonate (33 mg) in 1,4-dioxane (1 mL) was stirred at 140°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining N-(6-chloropyridazin-3-yl)-7-(1-methyl-1H-pyrazol-4-yl)-1,5-naphthyridine-2-amine (3.7 mg).
$^1$H-NMR($CDCl_3$/$CD_3OD$=4/1)δ:9.15(1H,d,J=9.0Hz),8.89(1H,d,J=2.1Hz),8.22(1H,d,J=2.1Hz),8.21 (1H,d,J=9.0Hz),7.97(1H,s),7.96(1H,s),7.60(1H,d,J=9.0Hz),7.44(1H,d,J=9.0Hz),3.63(3H,s).
MSm/z(M+H):338.

<Example 0153>

<0153-1>

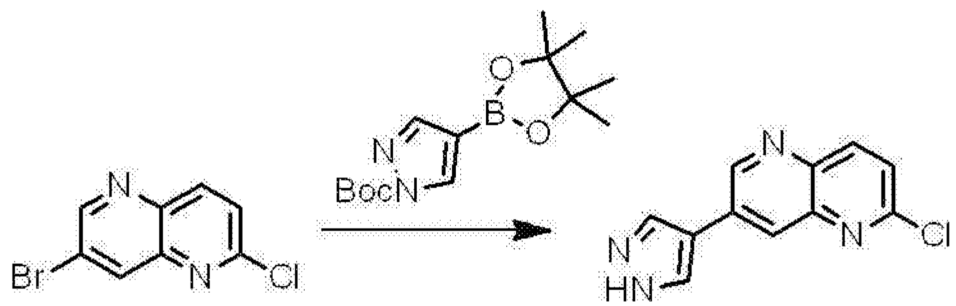

A mixture of 7-bromo-2-chloro-1,5-naphthyridine (100 mg), tert-butyl 4-(4,4,5,5-tetramethyl-1,3,2-dioxaborolan-2-yl)-1H-pyrazole-1-carboxylate (145 mg), sodium carbonate (109 mg), bis(di-tert-butyl (4-dimethylaminophenyl)phosphine)dichloropalladium(II) (29 mg), and water (0.2 mL) in 1,4-dioxane (2 mL) was stirred at 80°C for 2 hours. The reaction mixture was cooled to room temperature, the insolubles were filtered off using celite, and the solvent was distilled off under reduced pressure. The obtained residue was washed with ethyl acetate, thereby obtaining 2-chloro-7-(1H-pyrazol-4-yl)-1,5-naphthyridine (49 mg).
MSm/z(M+H):231.

<0153-2>

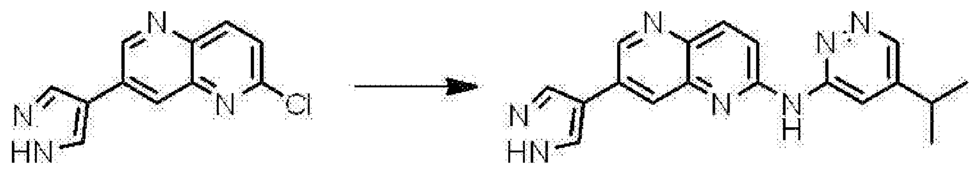

A mixture of 2-chloro-7-(1H-pyrazol-4-yl)-1,5-naphthyridine (10 mg), 5-isopropylpyridazine-3-amine (7 mg), tris(dibenzylideneacetone)dipalladium(0) (4 mg), 4,5-bis(diphenylphosphino)-9,9-dimethylxanthene (5 mg), and cesium carbonate (33 mg) in 1,4-dioxane (1 mL) was stirred at 140°C for 30 minutes using a microwave reaction apparatus. The reaction mixture was cooled to room temperature, and ethyl acetate and water were added thereto. The solid matter was collected by filtration, thereby obtaining N-(5-isopropylpyridazin-3-yl)-7-(1H-pyrazol-4-yl)-1,5-naphthyridine-2-amine (15 mg).
$^1$H-NMR(DMSO-$d_6$)δ:8.86(1H,brs),8.72(1H,brs),8.24(1H,d,J=8.7Hz),8.20(1H,brs),7.39(2H,s),7.35 (1H,d,J=8.7Hz),7.46(1H,brs),3.31-3.23(1H,m),1.33(6H,d,J=6.6Hz).
MSm/z(M+H):332.

<Example 0154>

<0154-1>

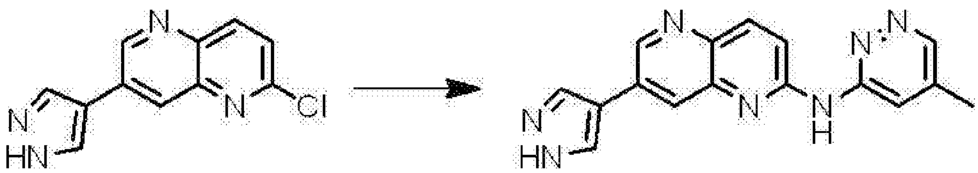

A mixture of 2-chloro-7-(1H-pyrazol-4-yl)-1,5-naphthyridine (10 mg), 5-methylpyridazine-3-amine (7 mg), tris(dibenzylideneacetone)dipalladium(0) (4 mg), 4,5-bis(diphenylphosphino)-9,9-dimethylxanthene (5 mg), and cesium carbonate (33 mg) in 1,4-dioxane (1 mL) was stirred at 140°C for 30 minutes using a microwave reaction apparatus. The reaction mixture was cooled to room temperature, and ethyl acetate and water were added thereto. The solid matter was collected by filtration, thereby obtaining N-(5-methylpyridazin-3-yl)-7-(1H-pyrazol-4-yl)-1,5-naphthyridine-2-amine (14 mg).
$^1$H-NMR(DMSO-$d_6$)δ:8.86(1H,brs),8.72(1H,brs),8.24(1H,d,J=8.7Hz),8.20(1H,brs),7.39(2H,s),7.35(1H,d,J=8.7Hz),7.46( 1H,brs),4.06(3 H,s).
MSm/z(M+H):304.

<Example 0155>

<0155-1>

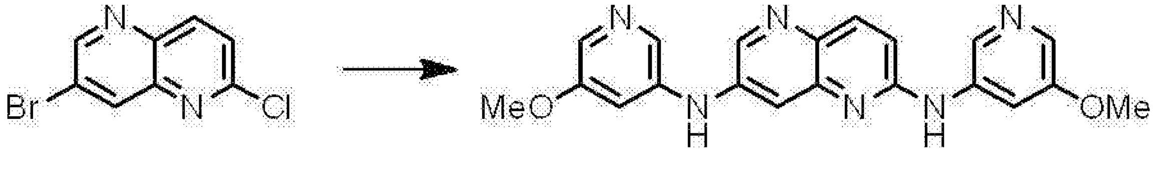

A mixture of 7-bromo-2-chloro-1,5-naphthyridine (50 mg), 5-methoxypyridine-3-amine (25 mg), tris(dibenzylideneacetone)dipalladium(0) (19 mg), 4,5-bis(diphenylphosphino)-9,9-dimethylxanthene (24 mg), and cesium carbonate (33 mg) in 1,4-dioxane (1 mL) was stirred at 140°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining $N^2$,$N^7$-bis(5-meth-

oxypyridin-3-yl)-1,5-naphthyridine-2,7-diamine (15 mg).
$^1$H-NMR($CDCl_3$/$CD_3OD$=4/1)δ:8.44(1H,d,J=2.7Hz),8.37(2H,brs),8.07(1H,d,J=2.7Hz),7.99(1H,d,J=9.0H) ,7.88(2H,brs),7.21(2H,brs),7.02(1H,d,J=9.0Hz),3.91(3H,s),3.89(3H,s).
MSm/z(M+H):375.

<Example 0156>

<0156-1>

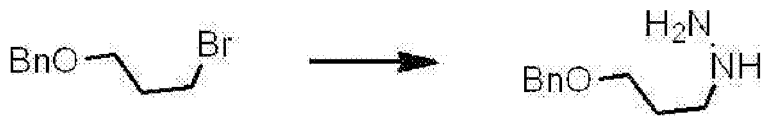

A solution of ((3-bromopropyloxy)methyl)benzene (5.0 g) in ethanol (5 mL) was added to hydrazine monohydrate (6.4 mL) at 65°C, followed by stirring at the same temperature for 1 hour. The reaction mixture was cooled to room temperature, and filtered through DOWEX MONOSPHERE 550A (OH) (product name, manufactured by Wako Pure Chemical Industries, Ltd.). The solvent was distilled off under reduced pressure, thereby obtaining (3-(benzyloxy)propyl)hydrazine (3.7 g).
MSm/z(M+H):181.

<0156-2>

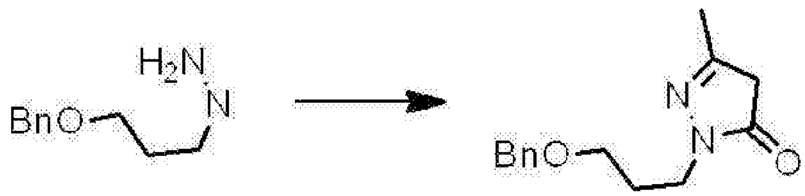

Ethyl 3-oxobutanoate (0.254 mL) was added to a solution of (3-(benzyloxy)propyl)hydrazine (500 mg) in ethanol (5 mL), followed by stirring for 3 hours under heating to reflux. The reaction mixture was cooled to room temperature, and the solvent was distilled off under reduced pressure. The obtained residue was purified by silica gel column chromatography (ethyl acetate-hexane), thereby obtaining 1-(3-(benzyloxy)propyl)-3-methyl-1H-pyrazol-5(4H)-one (261 mg).
MSm/z(M+H):247.

<0156-3>

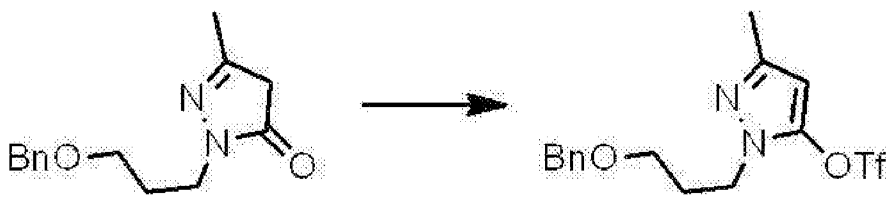

Trifluoromethanesulfonic aicd anhydride (0.260 mL) was added to a solution of 1-(3-(benzyloxy)propyl)-3-methyl-1H-pyrazol-5(4H)-one (261 mg) and pyridine (0.154 mL) in dichloromethane (10 mL) under ice-cooling, followed

by stirring at the same temperature for 30 minutes. A saturated sodium hydrogen carbonate aqueous solution and ethyl acetate were added to the reaction mixture. The organic layer was collected by separation, washed with a saturated sodium chloride aqueous solution, and dried over anhydrous sodium sulfate, and the solvent was distilled off under reduced pressure. The obtained residue was purified by silica gel column chromatography (methanol-ethyl acetate-hexane), thereby obtaining (1-(3-(benzyloxy)propyl)-3-methyl-1H-pyrazol-5-yl) trifluoromethanesulfonate (237 mg).
MSm/z(M+H):379.

<0156-4>

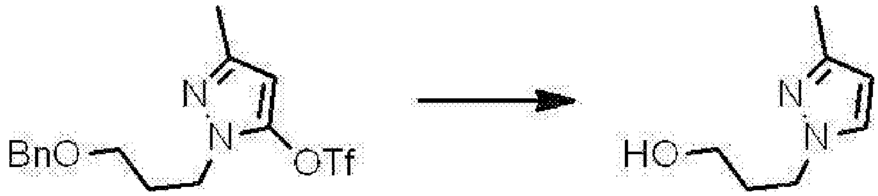

(1-(3-(Benzyloxy)propyl)-3-methyl-1H-pyrazol-5-yl) trifluoromethanesulfonate (237 mg) was added to a mixture of 20%-palladium hydroxide-carbon (50 mg) in methanol (10 mL), followed by stirring for 5 hours in a hydrogen atmosphere. The insolubles were filtered off using celite, and the solvent was distilled off under reduced pressure, thereby obtaining 3-(3-methyl-1H-pyrazol-1-yl)propan-1-ol (177 mg).
$^1$H-NMR($CDCl_3$)$\delta$:7.73(1H,d,J=2.7Hz),6.40(1H,d,J=2.7Hz),4.58(2H,t,J=6.6Hz),3.73(2H,5.1Hz), 2.52(3H,s),2.26-2.15(2H,m).

<0156-5>

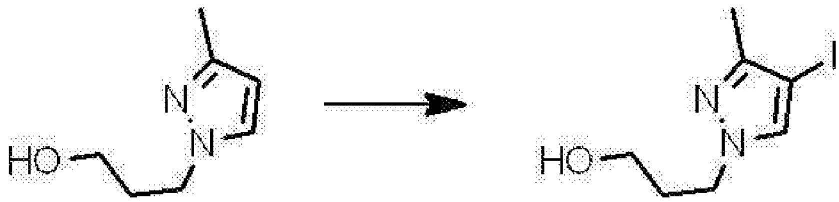

Iodine (95 mg) and ammonium cerium nitrate (206 mg) were added to a solution of 3-(3-methyl-1H-pyrazol-1-yl)propan-1-ol (177 mg) in acetonitrile (6 mL), followed by stirring at room temperature for 9 hours. Ethyl acetate and water were added to the reaction mixture. The organic layer was collected by separation, washed sequentially with a 10% sodium hydrogen sulfite aqueous solution and a saturated sodium chloride aqueous solution, and dried over anhydrous sodium sulfate, and the solvent was distilled off under reduced pressure, thereby obtaining 3-(4-iodo-3-methyl-1H-pyrazol-1-yl)propan-1-ol (134 mg).
MSm/z(M+H):267.

<0156-6>

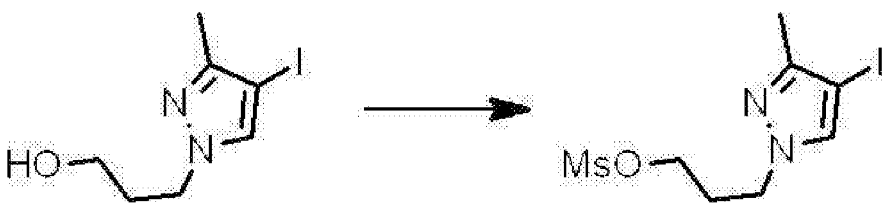

Methanesulfonyl chloride (0.059 mL) was added to a solution of 3-(4-iodo-3-methyl-1H-pyrazol-1-yl)propan-1-ol (134 mg) and triethylamine (0.14 mL) in dichloromethane (5 mL) under ice-cooling, followed by stirring at the same temperature for 30 minutes. Ethyl acetate and water were added to the reaction mixture. The organic layer was collected by separation, washed with a saturated sodium chloride aqueous solution, and dried over anhydrous sodium sulfate, and the solvent was distilled off under reduced pressure, thereby obtaining 3-(4-iodo-3-methyl-1H-pyrazol-1-yl)propyl methanesulfonate (165 mg).
MSm/z(M+H):345.

<0156-7>

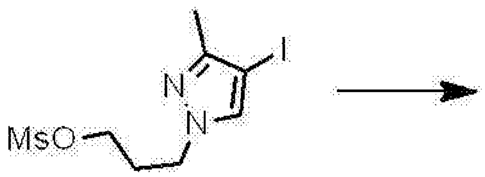

Pyrrolidine (0.048 mL) was added to a mixture of 3-(4-iodo-3-methyl-1H-pyrazol-1-yl)propyl methanesulfonate (165 mg), and potassium carbonate (100 mg) in acetonitrile (2.4 mL), followed by stirring at 50°C for 10 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining 4-iodo-3-methyl-1-(3-(pyrrolidin-1-yl)propyl)-1H-pyrazole (73 mg).
MSm/z(M+H):320.

<0156-8>

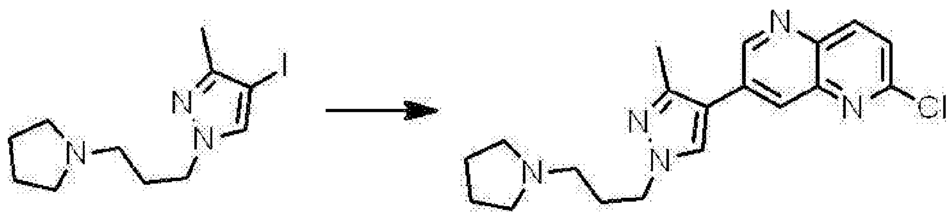

A mixture of 7-bromo-2-chloro-1,5-naphthyridine (56 mg), bis(pinacolato)diboron (87 mg), (1,1'-bis(diphenylphosphino)ferrocene)palladium(II) dichloride (19 mg), and potassium acetate (45 mg) in 1,4-dioxane (2 mL) was stirred at 100°C for 2 hours in a nitrogen atmosphere. 4-Iodo-3-methyl-1-(3-(pyrrolidin-1-yl)propyl)-1H-pyrazole (73 mg), sodium carbonate (49 mg), and bis(di-tert-butyl (4-dimethylaminophenyl)phosphine)dichloropalladium(II) (16 mg) were added to the reaction mixture, followed by stirring at 100°C for 2 hours. The reaction mixture was cooled to room temperature, the insolubles were filtered off using celite, and the solvent was distilled off under reduced pressure. The obtained residue was purified by silica gel column chromatography (methanol-ethyl acetate, NH silica), thereby obtaining 2-chloro-7-(3-methyl-1-(3-(pyrrolidin-1-yl)-1H-pyrazol-4-yl)-1,5-naphthyridine (13 mg).
MSm/z(M+H):356.

<0156-9>

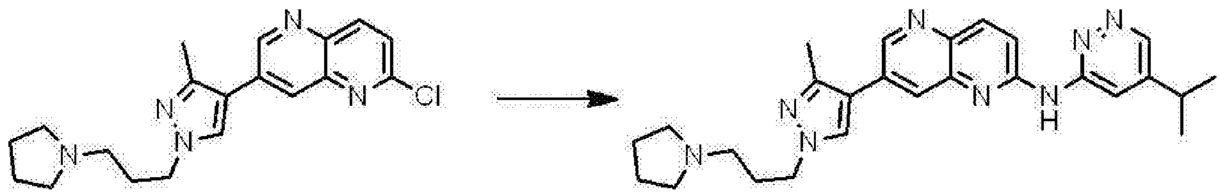

A mixture of 2-chloro-7-(3-methyl-1-(3-(pyrrolidin-1-yl)-1H-pyrazol-4-yl)-1,5-naphthyridine (13 mg), 5-isopropylpyridazine-3-amine (8 mg), tris(dibenzylideneacetone)dipalladium(0) (4 mg), 4,5-bis(diphenylphosphino)-9,9-dimethylxanthene (5 mg), and cesium carbonate (30 mg) in 1,4-dioxane (1 mL) was stirred at 140°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining N-(5-isopropylpyridazin-3-yl)-7-(3-methyl-1-(3-(pyrrolidin-1-yl)propyl)-1H-pyrazol-4-yl)-1,5-naphthyridine-2-amine (4.4 mg).
$^1$H-NMR($CDCl_3$/$CD_3OD$=4/1)$\delta$:8.92(1H,brs),8.79(1H,d,J=1.8Hz),8.72(1H,brs),8.22(1H,d,J=9.0Hz),8.10( 1H,d,J=1.8Hz),7.81(1H,s),7.52(1H,d,J=9.0Hz),4.25-4.18(2H,m),3.38-3.35(2H,m),3.10-2.99(1H,m),2.61-2.49(4H,m),2.50(3H,s),2.20-2.07(2H,m),1.89-1.79(4H,m),1.40(6H,d,J=6.6Hz).
MSm/z(M+H):457.

<Example 0157>

<0157-1>

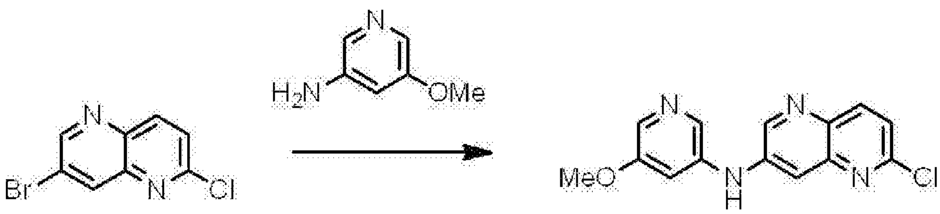

A mixture of 7-bromo-2-chloro-1,5-naphthyridine (50 mg), 5-methoxypyridine-3-amine (25 mg), tris(dibenzylideneacetone)dipalladium(0) (19 mg), 4,5-bis(diphenylphosphino)-9,9-dimethylxanthene (24 mg), and cesium carbonate (33 mg) in 1,4-dioxane (1 mL) was stirred at 140°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining 6-chloro-N-(5-methoxypyridin-3-yl)-1,5-naphthyridine-3-amine (5.4 mg).
MSm/z(M+H):287.

<0157-2>

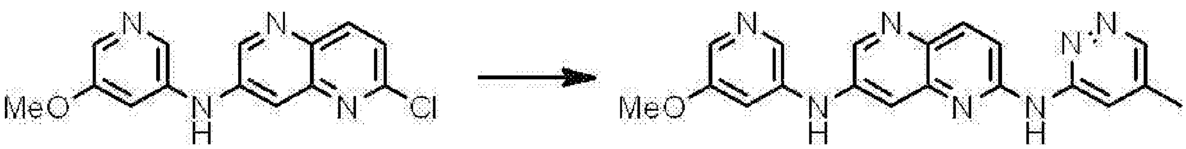

A mixture of 6-chloro-N-(5-methoxypyridin-3-yl)-1,5-naphthyridine-3-amine (5 mg), 5-methylpyridazine-3-amine (3 mg), tris(dibenzylideneacetone)dipalladium(0) (5 mg), 4,5-bis(diphenylphosphino)-9,9-dimethylxanthene (10 mg), and cesium carbonate (20 mg) in 1,4-dioxane (1 mL) was stirred at 140°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining

N$^7$-(5-methoxypyridin-3-yl)-N$^2$-(5-methylpyridazin-3-yl)-1,5-naphthyridine-2,7-diamine (15 mg).
$^1$H-NMR($CDCl_3$/$CD_3OD$=4/1)δ:8.69(1H,brs),8.63(1H,brs),8.50(1H,d,J=2.7Hz),8.13(1H,d,J=2.7Hz), 8.10(1H,d,J=9.3Hz),7.92(1H,d,J=2.7Hz),7.78(1H,brs),7.35(1H,d,J=9.0Hz),7.22(1H,d,J=2.7Hz),3.90(3 H,s),2.43(3H,s).
MSm/z(M+H):360.

<Example 0158>

<0158-1>

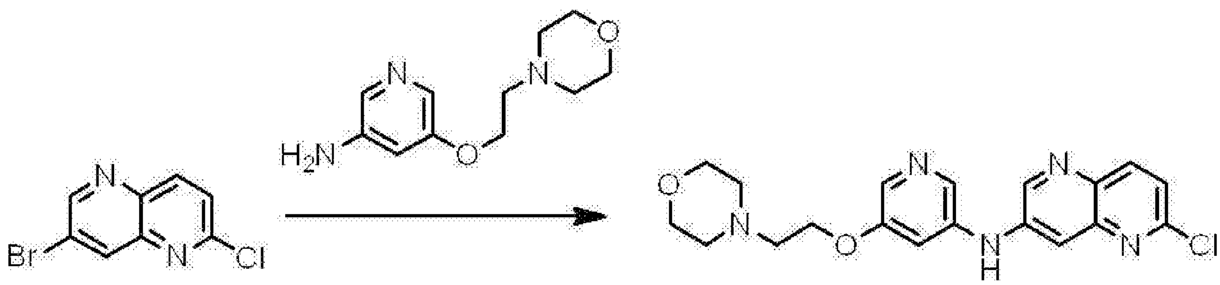

A mixture of 7-bromo-2-chloro-1,5-naphthyridine (100 mg), 5-(2-morpholinoethoxy)pyridine-3-amine (92 mg), tris(dibenzylideneacetone)dipalladium(0) (37 mg), 4,5-bis(diphenylphosphino)-9,9-dimethylxanthene (47 mg), and cesium carbonate (267 mg) in 1,4-dioxane (2 mL) was stirred at 140°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining 6-chloro-N-(5-(2-morpholinoethoxy)pyridin-3-yl)-1,5-naphthyridine-3-amine (5.4 mg).
MSm/z(M+H):386.

<0158-2>

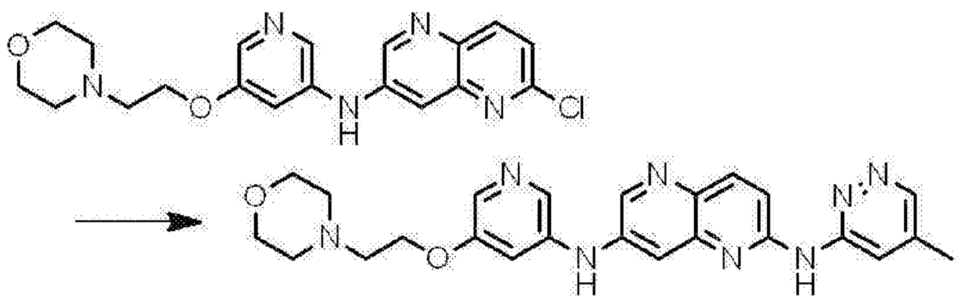

A mixture of 6-chloro-N-(5-(2-morpholinoethoxy)pyridin-3-yl)-1,5-naphthyridine-3-amine (10 mg), 5-methylpyridazine-3-amine (4 mg), tris(dibenzylideneacetone)dipalladium(0) (5 mg), 4,5-bis(diphenylphosphino)-9,9-dimethylxanthene (10 mg), and cesium carbonate (20 mg) in 1,4-dioxane (1 mL) was stirred at 140°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining N$^2$-(5-methylpyridazin-3-yl)-N$^7$-(5-(2-morpholinoethoxy)pyridin-3-yl)-1,5-naphthyridine-2,7-diamine (1.9 mg).
$^1$H-NMR($CDCl_3$/$CD_3OD$=4/1)δ:8.67(1H,brs),8.63(1H,brs),8.50(1H,brs),8.14(1H,brs),8.11 (1H,d,J=9.0Hz),7.91(1H,brs),7.78(1H,brs),7.24(1H,d,J=9.0Hz),7.21(1H,brs),4.20(2H,t,J=5.4Hz),3.79-3.71(4H,m),2.85 (2H,t,J=5.4Hz),2.66-2.56(4H,m),2.43(3H,s).
MSm/z(M+H):459.

<Example 0159>

<0159-1>

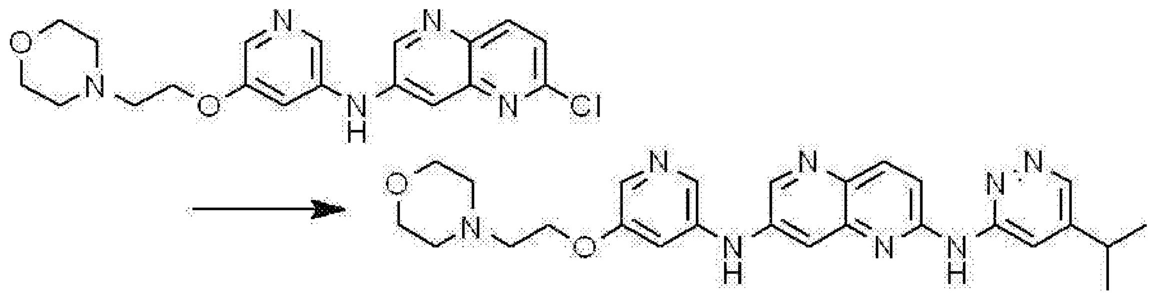

A mixture of 6-chloro-N-(5-(2-morpholinoethoxy)pyridin-3-yl)-1,5-naphthyridine-3-amine (10 mg), 5-isopropylpyridazine-3-amine (5 mg), tris(dibenzylideneacetone)dipalladium(0) (5 mg), 4,5-bis(diphenylphosphino)-9,9-dimethylxanthene (10 mg), and cesium carbonate (20 mg) in 1,4-dioxane (1 mL) was stirred at 140°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining $N^2$-(5-isopropylpyridazin-3-yl)-$N^7$-(5-(2-morpholinoethoxy)pyridin-3-yl)-1,5-naphthyridine-2,7-diamine (3.8 mg).
$^1$H-NMR($CDCl_3$/$CD_3OD$=4/1)$\delta$:8.75(1H,brs),8.69(1H,brs),8.52(1H,d,J=2.7Hz),8.12(1H,brs), 8.11(1H,d,J=8.7Hz),7.91(1H,brs),7.73(1H,brs),7.40(1H,d,J=8.7Hz),7.22(1H,brs),4.18(2H,t,J=5.4Hz),3 .77-3.72(4H,m), 3.06-2.95(1H,m),2.84(2H,t,J=5.4Hz),2.63-2.57(4H,m),1.37(6H,d,J=7.4Hz).
MSm/z(M+H):487.

<Example 0160>

<0160-1>

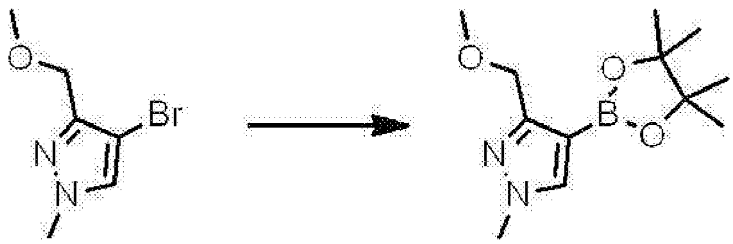

A2.6 mol/L n-butyllithium-hexane solution (0.52 mL) was added to a solution of 4-bromo-3-(methoxymethyl)-1-methyl-1H-pyrazole (185 mg) in tetrahydrofuran (4.5 mL) at -80°C, followed by stirring at the same temperature for 30 minutes, and 2-isopropyloxy-4,4,5,5-tetramethyl-1,3,2-dioxaborolane (369 mg) was added thereto at the same temperature, followed by stirring while slowly heating to room temperature over a period of 2.5 hours. A saturated ammonium chloride aqueous solution and ethyl acetate were added to the reaction mixture. The organic layer was collected by separation, washed with a saturated sodium chloride aqueous solution, and dried over anhydrous sodium sulfate, and the solvent was distilled off under reduced pressure, thereby obtaining 3-(methoxymethyl)-1-methyl-4-(4,4,5,5-tetramethyl-1,3,2-dioxaborolan-2-yl)-1H-pyrazole (304 mg).
$^1$H-NMR($CDCl_3$)$\delta$:7.59(1H,s),4.60(2H,s),3.87(3H,s),3.43(3H,s),1.24(12H,s).

<0160-2>

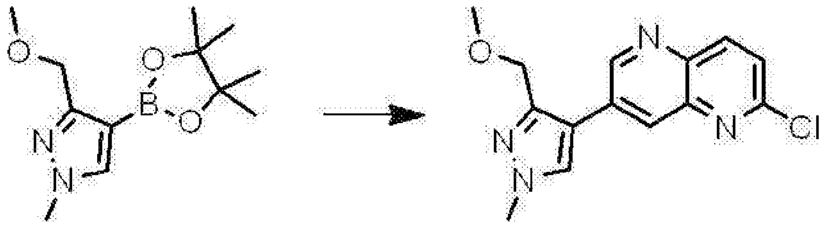

A mixture of 7-bromo-2-chloro-1,5-naphthyridine (31 mg), 3-(methoxymethyl)-1-methyl-4-(4,4,5,5-tetramethyl-1,3,2-dioxaborolan-2-yl)-1H-pyrazole (43 mg), sodium carbonate (34 mg), bis(di-tert-butyl (4-dimethylaminophenyl)phosphine)dichloropalladium(II) (9 mg), and water (0.1 mL) in 1,4-dioxane (1 mL) was stirred at 80°C for 5 hours. The reaction mixture was cooled to room temperature, the insolubles were filtered off using celite, and the solvent was distilled off under reduced pressure. The obtained residue was purified by silica gel column chromatography (methanol-ethyl acetate), thereby obtaining 2-chloro-7-(3-(methoxymethyl)-1-methyl-1H-pyrazol-4-yl)-1,5-naphthyridine (13 mg).
MSm/z(M+H):289.

<0160-3>

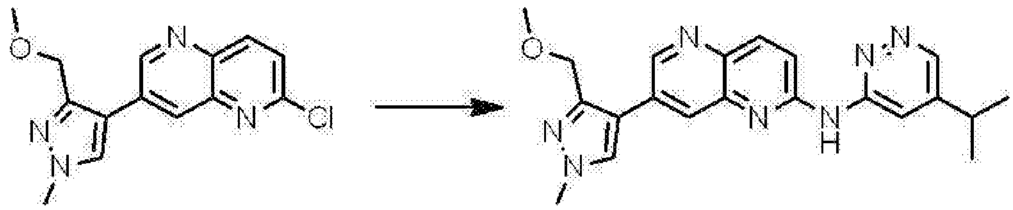

A mixture of 2-chloro-7-(3-(methoxymethyl)-1-methyl-1H-pyrazol-4-yl)-1,5-naphthyridine (6 mg), 5-isopropylpyridazine-3-amine (5 mg), tris(dibenzylideneacetone)dipalladium(0) (5 mg), 4,5-bis(diphenylphosphino)-9,9-dimethylxanthene (10 mg), and cesium carbonate (20 mg) in 1,4-dioxane (1 mL) was stirred at 140°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining N-(5-isopropylpyridazin-3-yl)-7-(3-(methoxymethyl)-1-methyl-1H-pyrazol-4-yl)-1,5-naphthyridine-2-amine (2.1 mg).
$^1$H-NMR($CDCl_3$/$CD_3OD$=4/1)δ:8.98(1H,brs),8.85(1H,d,J=1.8Hz),8.70(1H,brs),8.37(1H,d,J=1.8Hz),8.22(1H,d,J=9.3Hz),7.84(1H,s),7.50(1H,d,J=9.3Hz),4.57(2H,s),3.97(3H,s),3.51(3H,s),3.08-2.98(1H,m),1.41(6H,d,J=7.2Hz).
MSm/z(M+H):390.

<Example 0161>

<0161-1>

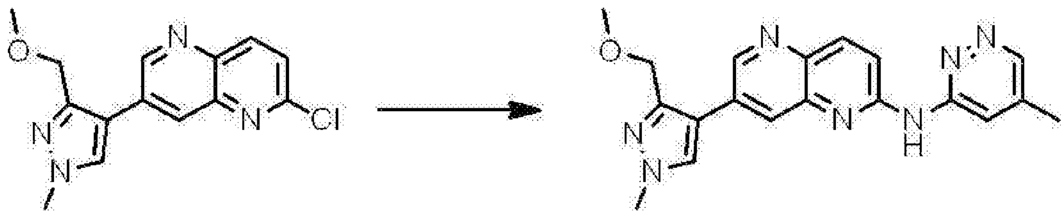

A mixture of 2-chloro-7-(3-(methoxymethyl)-1-methyl-1H-pyrazol-4-yl)-1,5-naphthyridine (20 mg), 5-methylpyridazine-3-amine (7 mg), tris(dibenzylideneacetone)dipalladium(0) (5 mg), 4,5-bis(diphenylphosphino)-9,9-dimethylxanthene (10 mg), and cesium carbonate (20 mg) in 1,4-dioxane (1 mL) was stirred at 140°C for 30 minutes using a microwave

reaction apparatus. The reaction mixture was cooled to room temperature, the insolubles were filtered off using celite, and the obtained solution was purified by silica gel column chromatography (methanol-ethyl acetate, NH silica), thereby obtaining 7-(3-(methoxymethyl)-1-methyl-1H-pyrazol-4-yl)-N-(5-methylpyridazin-3-yl)-1,5-naphthyridine-2-amine (2.1 mg).
$^1$H-NMR($CDCl_3$/$CD_3OD$=4/1)δ:8.98(1H,brs),8.87(1H,brs),8.66(1H,brs),8.32(1H,brs),8.22 (1H,d,J=9.3Hz),7.85(1H,s),7.51(1H,d,J=9.3Hz),4.58(2H,s),4.00(3H,s),3.54(3H,s),2.46(3H,s).
MSm/z(M+H):362.

<Example 0162>

<0162-1>

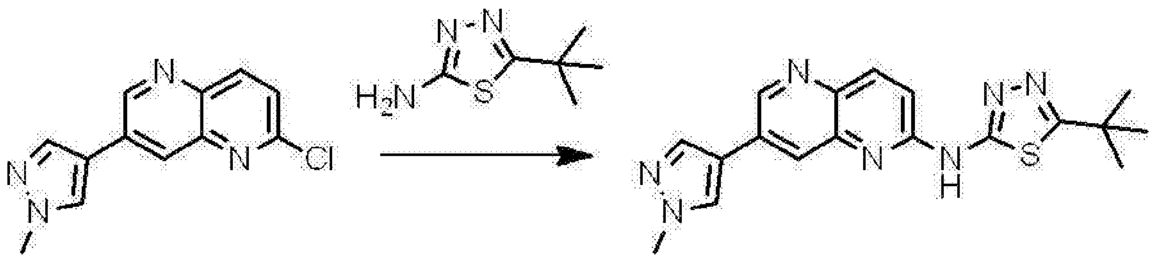

2-Amino-5-tert-butyl-1,3,4-thiadiazole (15 mg), 4,5-bis(diphenylphosphino)-9,9-dimethylxanthene (5 mg), cesium carbonate (40 mg), and tris(dibenzylideneacetone)dipalladium(0) (5 mg) were added to a solution of 2-chloro-7-(1-methyl-1H-pyrazol-4-yl)-1,5-naphthyridine (15 mg) in 1,4-dioxane (1.2 mL), followed by stirring at 150°C for 1 hour using a microwave reaction apparatus. The reaction liquid was cooled to room temperature, a mixed solvent of chloroform-methanol was added thereto, and the resultant product was purified by silica gel column chromatography (chloroform-methanol, NH silica), thereby obtaining 5-(tert-butyl)-N-(7-(1-methyl-1H-pyrazol-4-yl)-1,5-naphthyridin-2-yl)-1,3,4-thiadiazole-2-amine (5 mg) as a white solid.
$^1$H-NMR(DMSO-$d_6$)δ:12.03(1H,s),9.07(1H,d,J=2.0Hz),8.53(1H,s),8.33(1H,d,J=1.3Hz),8.26-8.23(2H,m), 7.39(1H,d,J=8.9Hz),3.93(3H,s), 1.48(9H,s).
MSm/z(M+H):366.

<Example 0163>

<0163-1>

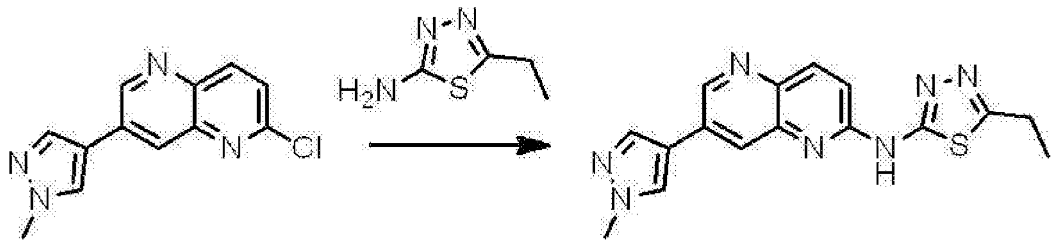

5-Ethyl-N-(7-(1-methyl-1H-pyrazol-4-yl)-1,5-naphthyridin-2-yl)-1,3,4-thiadiazole-2-amine (6.5 mg) was obtained as a yellow solid in the same manner as in Example 0162 except that 2-amino-5-ethyl-1,3,4-thiadiazole was used instead of the 2-amino-5-tert-butyl-1,3,4-thiadiazole used in Example 0162.
$^1$H-NMR(DMSO-$d_6$)δ:12.04(1H,s),9.07(1H,d,J=2.0Hz),8.52(1H,s),8.32(1H,d,J=1.3Hz),8.25(1H,d,J=8.9Hz),8.21(1H,s), 7.39(1H,d,J=8.9Hz),3.93(3H,s),3.03(2H,q,J=7.5Hz),1.38(3H,t,J=7.4Hz).
MSm/z(M+H):338.

<Example 0164>

<0164-1>

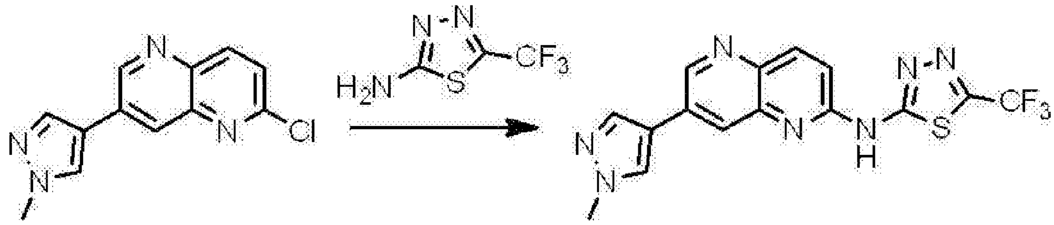

N-(7-(1-methyl-1H-pyrazol-4-yl)-1,5-naphthyridin-2-yl)-5-(trifluoromethyl)-1,3,4-thiadiazole-2-amine (3.5 mg) was obtained as a yellow solid in the same manner as in Example 0162 except that 2-amino-5-trifluoromethyl-1,3,4-thiadiazole was used instead of the 2-amino-5-tert-butyl-1,3,4-thiadiazole used in Example 0162.
$^1$H-NMR(DMSO-d$_6$)δ:12.94(1H,s),9.15(1H,d,J=2.0Hz),8.57(1H,s),8.53(1H,s),8.37(1H,d,J=8.9Hz),8.26(1H,s),7.50(1H,d ,J=9.2Hz),3.93(3H,s).
MSm/z(M+H):378.

<Example 0165>

<0165-1>

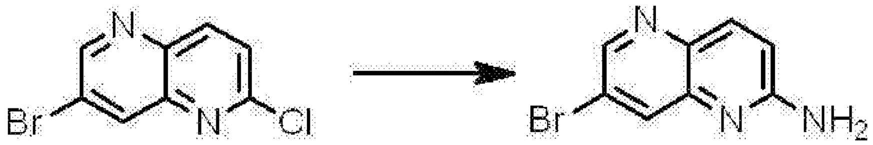

A mixture solution of 7-bromo-2-chloro-1,5-naphthyridine (100 mg) in 1,4-dioxane (2 mL) and a 25% ammonia aqueous solution was stirred at 120°C for 3 hours using a microwave reaction apparatus. The reaction mixture was cooled to room temperature, and a saturated sodium chloride aqueous solution and ethyl acetate were added thereto. The organic layer was collected by separation, and dried over anhydrous sodium sulfate, and the solvent was distilled off under reduced pressure, thereby obtaining 7-bromo-1,5-naphthyridine-2-amine (90 mg) as a white solid.
MSm/z(M+H):224,226.

<0165-2>

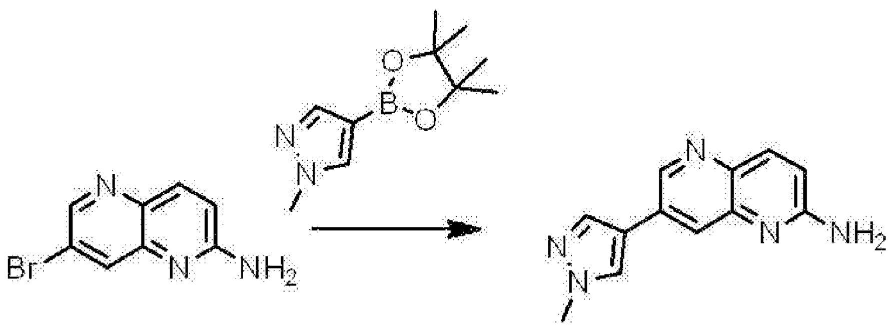

1-Methylpyrazole-4-boronic acid pinacol ester (460 mg), bis(di-tert-butyl (4-dimethylaminophenyl)phosphine)dichloropalladium(II) (70 mg), sodium carbonate (440 mg), 1,4-dioxane (1 mL), and water (200 μL) were added to a solution of 7-bromo-1,5-naphthyridine-2-amine (500 mg) in 1,4-dioxane (2 mL), followed by stirring at 100°C for 2 hours in a nitrogen atmosphere. The reaction liquid was cooled to room temperature, a solution of chloroform-methanol was added thereto, and the resultant product was purified by silica gel column chromatography (chloroform-methanol, NH silica), thereby obtaining 7-(1-methyl-1H-pyrazol-4-yl)-1,5-naphthyridine-2-amine (180 mg).
MSm/z(M+H):226.

<0165-3>

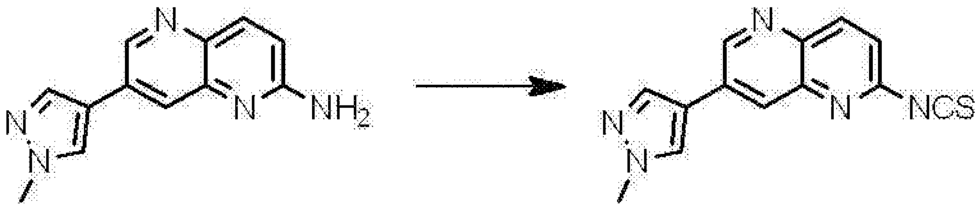

Pyridine (5 mL) was added to a mixture of 7-(1-methyl-1H-pyrazol-4-yl)-1,5-naphthyridine-2-amine (100 mg) and phenyl chlorothioformate (200 mg), followed by stirring at room temperature for 1 hour. The solvent was distilled off at 60°C under reduced pressure, and the obtained residue was purified by silica gel column chromatography (hexane-ethyl acetate), thereby obtaining 2-isothiocyanate-7-(1-methyl-1H-pyrazol-4-yl)-1,5-naphthyridine (105 mg).
$^1$H-NMR($CDCl_3$)δ:9.11(1H,d,J=2.3Hz),8.37(1H,d,J=8.6Hz),8.26(1H,t,J=1.0Hz),7.96(1H,s),7.84(1H,s),7.38(1H,d,J=8.9Hz),4.02(3H,s).

<0165-4>

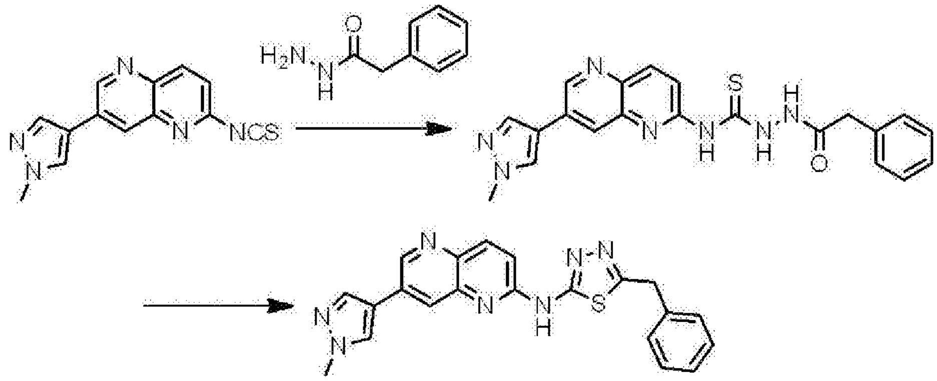

A solution of 2-isothiocyanate-7-(1-methyl-1H-pyrazol-4-yl)-1,5-naphthyridine (10 mg) and benzeneacetic acid hydrazide (10 mg) in 1,4-dioxane (1 mL) was stirred at 120°C for 30 minutes using a microwave reaction apparatus. The solvent was distilled off under reduced pressure, and sulfuric acid (0.6 mL) was added thereto under ice-coolong, followed by stirring at room temperature for 30 minutes. The reaction mixture was added dropwise to ice water, and the resultant product was neutralized with a sodium hydroxide aqueous solution. The solid matter was collected by filtration, thereby obtaining 5-benzyl-N-(7-(1-methyl-1H-pyrazol-4-yl)-1,5-naphthyridin-2-yl)-1,3,4-thiadiazole-2-amine (10 mg) as a yellow solid.
$^1$H-NMR(DMSO-$d_6$)δ:12.10(1H,s),9.06(1H,d,J=2.0Hz),8.49(1H,s),8.27-8.23(2H,m),8.18(1H,s),7.42-7.34(5H,m),7.31-7.25(1H,m),4.40(2H,s),3.92(3H,s).
MSm/z(M+H):400.

<Example 0166>

<0166-1>

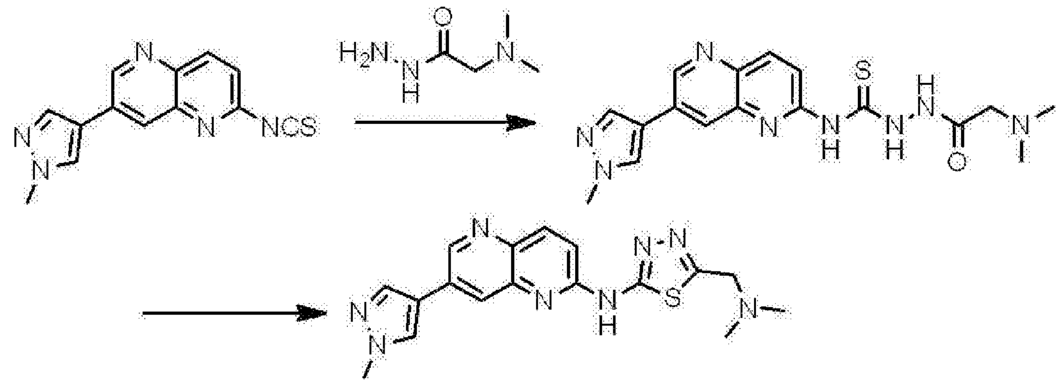

5-((Dimethylamino)methyl)-N-(7-(1-methyl-1H-pyrazol-4-yl)-1,5-naphthyridin-2-yl)-1,3,4-thiadiazole-2-amine (1.1 mg) was obtained as a yellow solid in the same manner as in Example 0165 except that N-amino-2-(dimethylamino)acetamide was used instead of the benzeneacetic acid hydrazide used in Example 0165.
$^1$H-NMR($CDCl_3$)δ:8.96(1H,s),8.36-8.28(2H,m),8.020(1H,s),7.90(1H,s),7.63-7.59(1H,m),4.04(3H,s),3.96(2H,s),2.45(6H,s).
MSm/z(M+H):367.

<Example 0167>

<0167-1>

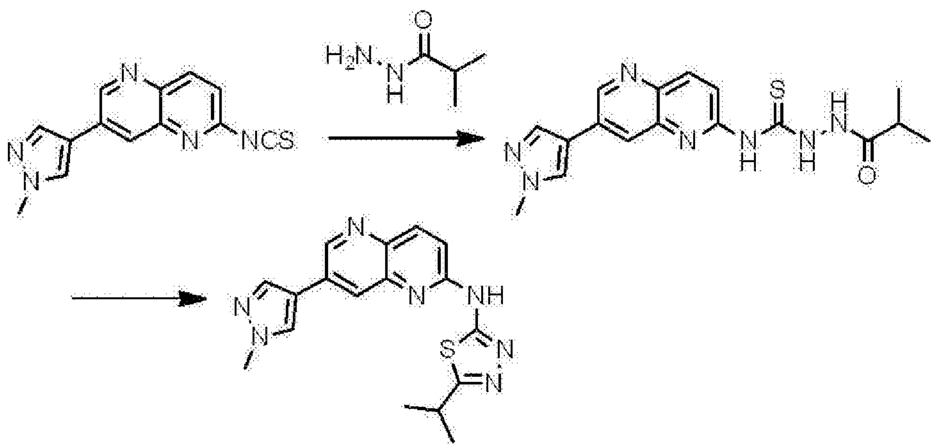

5-Isopropyl-N-(7-(1-methyl-1H-pyrazol-4-yl)-1,5-naphthyridin-2-yl)-1,3,4-thiadiazole-2-amine (3 mg) was obtained as a white solid in the same manner as in Example 0165 except that isobutyric acid hydrazide was used instead of the benzeneacetic acid hydrazide used in Example 0165.
$^1$H-NMR(DMSO-$d_6$)δ:12.04(1H,s),9.05(1H,d,J=2.0Hz),8.52(1H,s),8.31(1H,d,J=1.7Hz),8.24-8.21(2H,m),7.38(1H,d,J=9.2Hz),3.93(3H,s),3.41-3.35(1H,m),1.42(6H,d,J=6.9Hz).
MSm/z(M+H):352.

<Example 0168>

<0168-1>

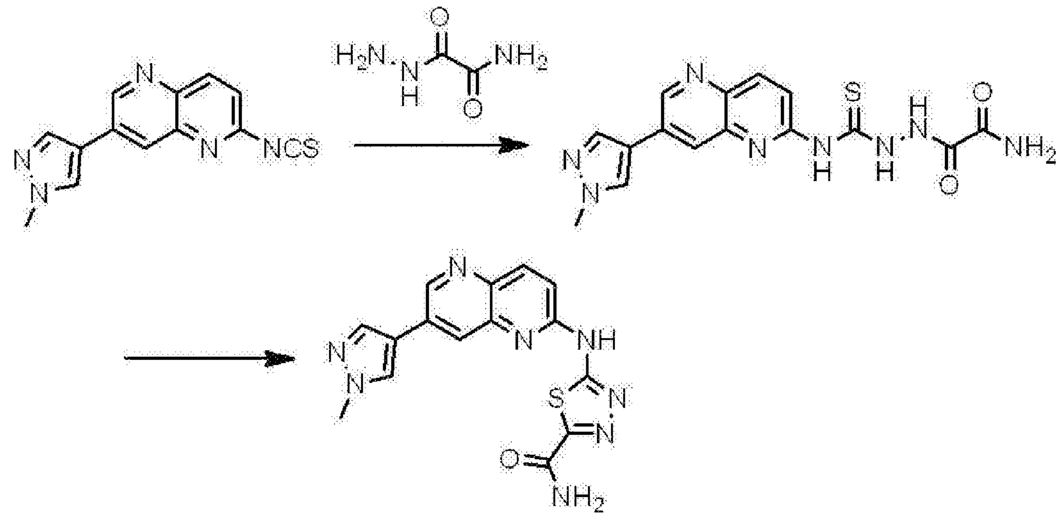

5-((7-(1-Methyl-1H-pyrazol-4-yl)-1,5-naphthyridin-2-yl)amino)-1,3,4-thiadiazole-2-carboxamide (2.5 mg) was obtained as a pale yellow solid in the same manner as in Example 0165 except that oxamic acid hydrazide was used instead of the benzeneacetic acid hydrazide used in Example 0165.
$^1$H-NMR(DMSO-$d_6$)$\delta$:12.56(1H,s),9.12(1H,d,J=2.3Hz),8.59(1H,s),8.37-8.35(2H,m),8.32(1H,d,J=9.2Hz),8.25(1H,s),7.91(1H,s),7.45(1H,d,J=8.9Hz),3.92(3H,s).
MSm/z(M+H):353.

<Example 0169>

<0169-1>

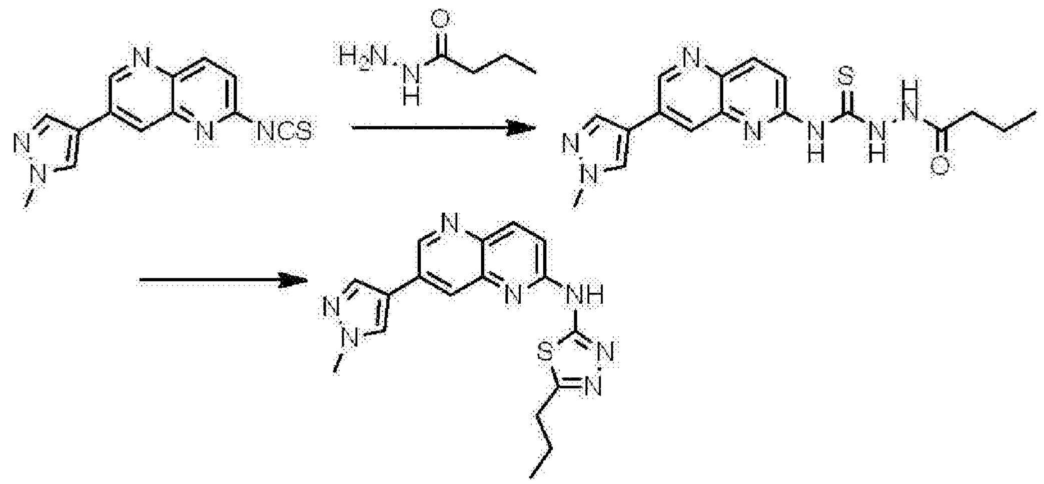

N-(7-(1-methyl-1H-pyrazol-4-yl)-1,5-naphthyridin-2-yl)-5-propyl-1,3,4-thiadiazole-2-amine (3.5 mg) was obtained as a white solid in the same manner as in Example 0165 except that butyric acid hydrazide was used instead of the benzeneacetic acid hydrazide used in Example 0165.

$^1$H-NMR($CDCl_3$)δ:12.04(1H,s),9.07(1H,d,J=2.0Hz),8.53(1H,s),8.32(1H,d,J=1.7Hz),8.25 (1H,d,J=8.9Hz),8.21(1H,s),7.39(1H,d,J=8.9Hz),3.93(3H,s),2.99(2H,t,J=7.4Hz),1.87-1.75(2H,m),1.00(3H,t,J=7.4Hz).
MSm/z(M+H):352.

<Example 0170>

<0170-1>

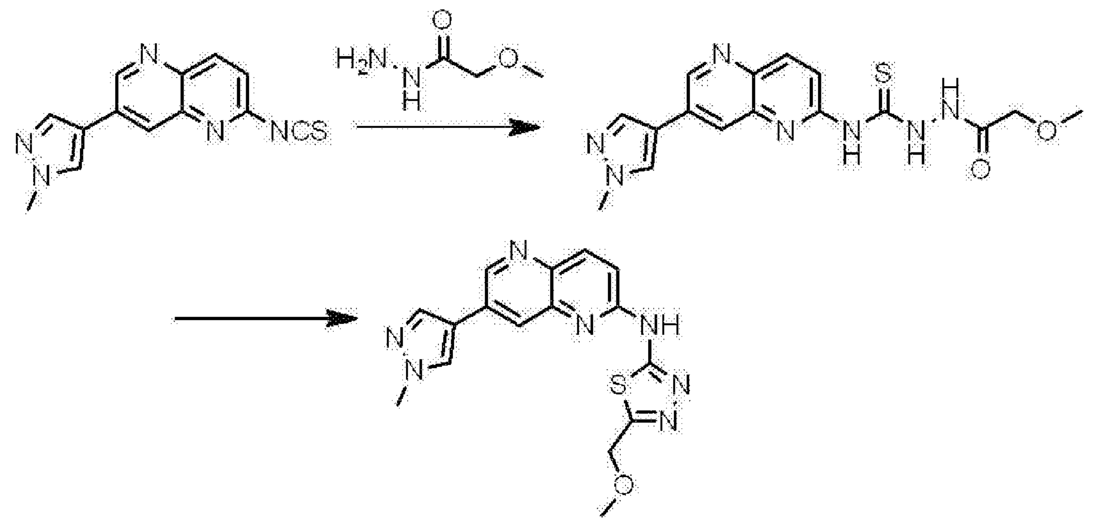

5-(Methoxymethyl)-N-(7-(1-methyl-1H-pyrazol-4-yl)-1,5-naphthyridin-2-yl)-1,3,4-thiadiazole-2-amine (3.5 mg) was obtained as a white solid in the same manner as in Example 0165 except that methoxyacetic acid hydrazide was used instead of the benzeneacetic acid hydrazide used in Example 0165.
$^1$H-NMR($CDCl_3$)δ:12.20(1H,s),9.09(1H,d,J=2.3Hz),8.55(1H,s),8.34(1H,d,J=1.7Hz),8.28(1H,d,J=8.9Hz),8 .23(1H,s),7.42(1 H,d,J=8.9Hz),4.79(2H,s),3.92(3H,s),3.39(3H,s).
MSm/z(M+H):354.

<Example 0171>

<0171-1>

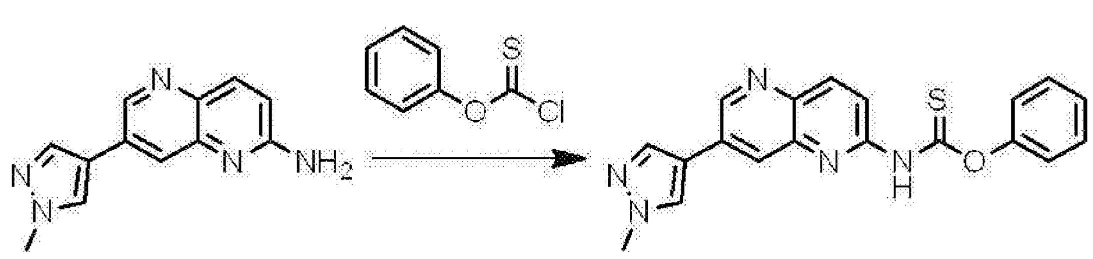

Pyridine (5 mL) was added to a mixture of 7-(1-methyl-1H-pyrazol-4-yl)-1,5-naphthyridine-2-amine (150 mg) and phenyl chlorothioformate (200 μL), followed by stirring at room temperature for 1 hour. Phenyl chlorothioformate (200 μL) was added thereto, followed by stirring at room temperature for 1 hour. The solvent was distilled off at 40°C under reduced pressure. A solution of ethanol-chloroform was added to the obtained residue, and the resultant product was purified by silica gel column chromatography (hexane-ethyl acetate), thereby obtaining (7-(1-methyl-1H-pyrazol-4-yl)-1,5-naphthyridin-2-yl)thiocarbamic acid phenyl (100 mg).
MSm/z(M+H):362.

<0171-2>

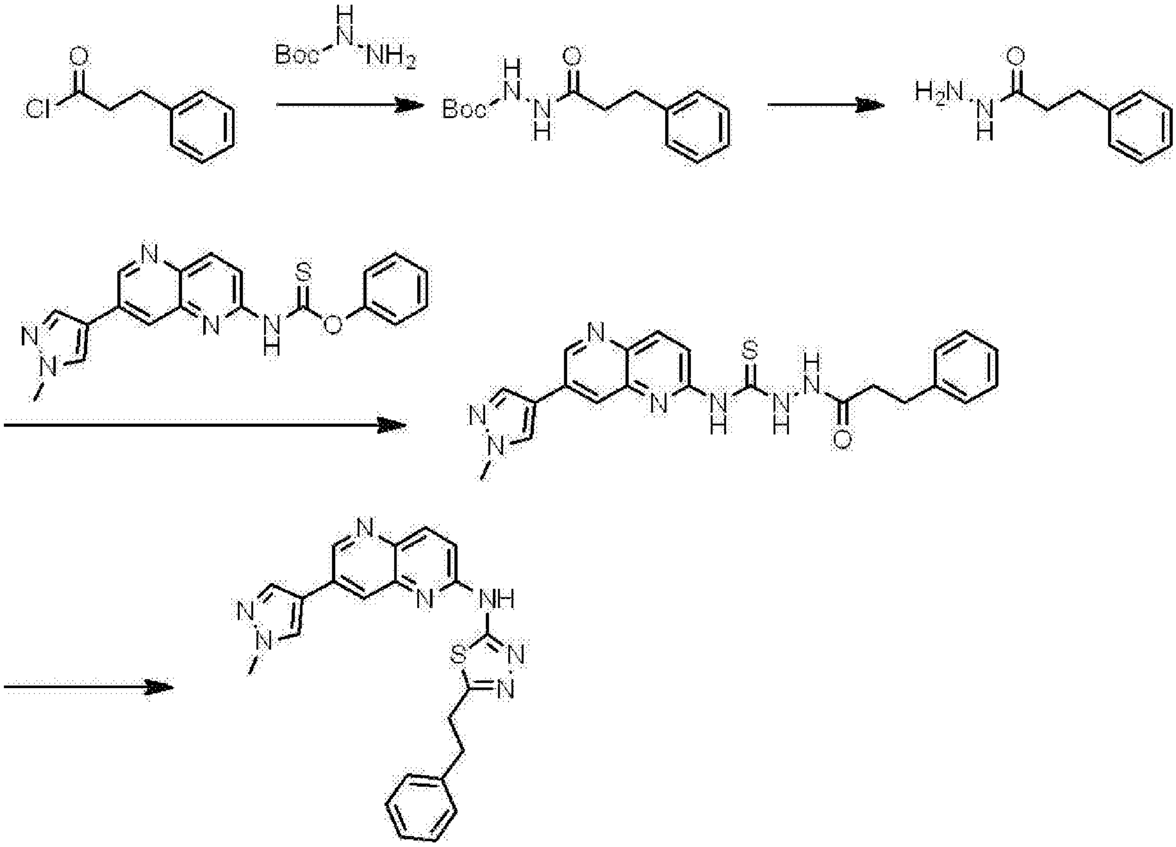

N,N-diisopropylethylamine (400 μL) was added to a solution of 3-phenylpropionyl chloride (170 mg) and tert-butyl carbazate (150 mg) in 1,4-dioxane (2 mL), followed by stirring at room temperature for 2 hours. A4 mol/L hydrogen chloride/1,4-dioxane solution (2 mL) was added to the reaction mixture, followed by allowing to stand at room temperature overnight. The solvent was distilled off under reduced pressure, and ethyl acetate and water were added to the obtained residue. The organic layer was collected by separation, washed with a saturated sodium chloride aqueous solution, and dried over anhydrous sodium sulfate, and the solvent was distilled off under reduced pressure. The obtained residue was purified by silica gel column chromatography (chloroform-methanol, NH silica), thereby obtaining 3-phenylpropanoic acid hydrazide.

(7-(1-Methyl-1H-pyrazol-4-yl)-1,5-naphthyridin-2-yl)thiocarbamic acid phenyl (10 mg) and 1,4-dioxane (1 mL) were added to 3-phenylpropanoic acid hydrazide, followed by stirring at room temperature for 30 minutes. The solvent was distilled off under reduced pressure, and the obtained residue was washed with ethyl acetate, thereby obtaining N-(7-(1-methyl-1H-pyrazol-4-yl)-1,5-naphthyridin-2-yl)-2-(3-phenylpropanoyl)hydrazine carbothioamide.

Sulfuric acid (0.6 mL) was added to N-(7-(1-methyl-1H-pyrazol-4-yl)-1,5-naphthyridin-2-yl)-2-(3-phenylpropanoyl)hydrazine carbothioamide under ice-cooling, followed by stirring at room temperature for 30 minutes. The reaction mixture was added dropwise to ice water, and the resultant product was neutralized with a sodium hydroxide aqueous solution. The solid matter was collected by filtration, and washed with a solution of chloroform-methanol, thereby obtaining N-(7-(1-methyl-1H-pyrazol-4-yl)-1,5-naphthyridin-2-yl)-5-phenethyl-1,3,4-thiadiazole-2-amine (6 mg) as a white solid.
$^1$H-NMR(DMSO-$d_6$)δ:12.05(1H,s),9.07(1H,d,J=2.0Hz),8.51(1H,s),8.27-8.23(2H,m),8.20(1H,s),7.39 (1H,d,J=9.2Hz), 7.34-7.32(4H,m),7.26-7.19(1H,m),3.94(3H,s), 3.38-3.34(2H,m),3.12(2H,t,J=7.8Hz).
MSm/z(M+H):414.

<Example 0172>

<0172-1>

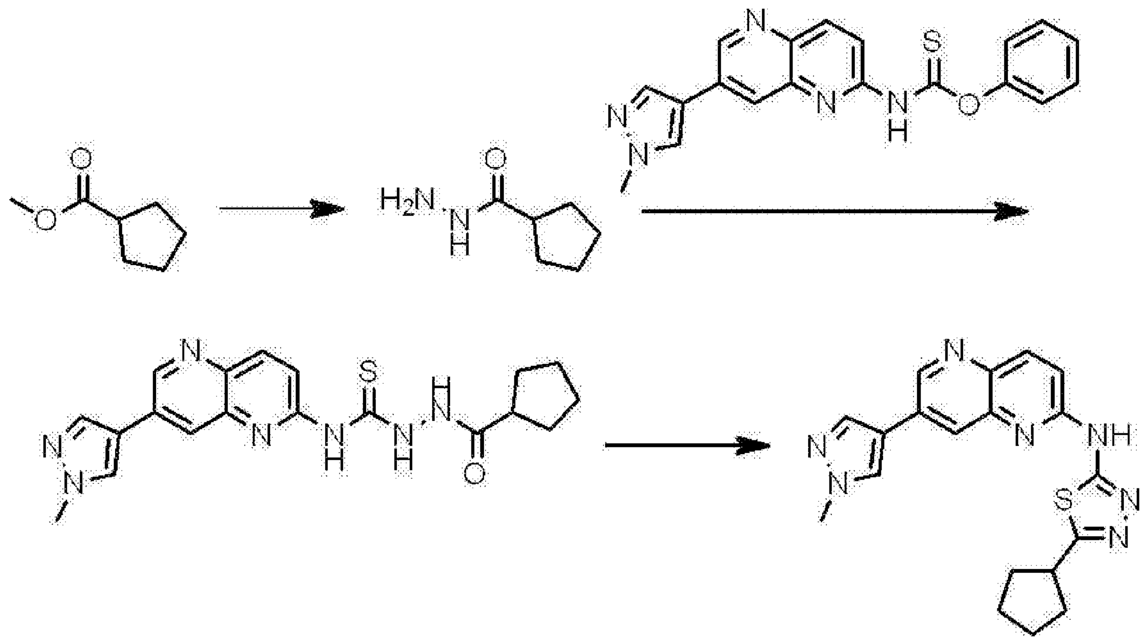

A solution of cyclopentanecarboxylic acid methyl (20 μL) and hydrazine monohydrate (20 μL) in methanol (1 mL) was stirred at 100°C for 1 hour using a microwave reaction apparatus. The solvent was distilled off under reduced pressure, and (7-(1-methyl-1H-pyrazol-4-yl)-1,5-naphthyridin-2-yl)thiocarbamic acid phenyl (10 mg) and 1,4-dioxane (1 mL) were added to the obtained residue, followed by stirring at 140°C for 30 minutes using a microwave reaction apparatus. The solvent was distilled off under reduced pressure, and ethanol (1 mL) and sulfuric acid (10 μL) were added to the obtained residue, followed by stirring at 100°C for 30 minutes using a microwave reaction apparatus. Water was added to the reaction mixture, and the resultant product was neutralized with a sodium hydroxide aqueous solution. The solid matter was collected by filtration, and washed with a solution of chloroform-methanol, thereby obtaining 5-cyclopentyl-N-(7-(1-methyl-1H-pyrazol-4-yl)-1,5-naphthyridin-2-yl)-1,3,4-thiadiazole-2-amine (2 mg) as a yellow solid.
$^{1}$H-NMR(DMSO-$d_6$)δ:12.03(1H,s),9.06(1H,d,J=2.0Hz),8.53(1H,s),8.31(1H,d,J=1.7Hz),8.26-8.20(2H,m),7.38(1H,d,J=8.9Hz),3.93(3H,s), 3.52-3.43(1H,m),2.21-2.13(2H,m),1.93-1.65(6H,m).
MSm/z(M+H):378.

<Example0173>

<0173-1>

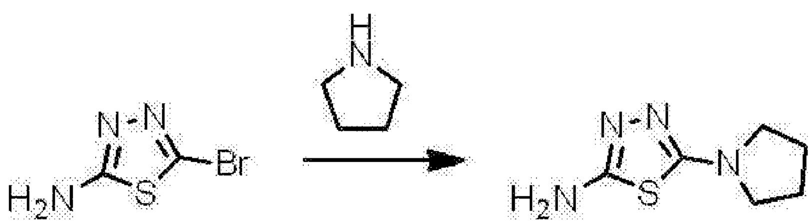

A solution of 5-bromo-1,3,4-thiadiazole-2-amine (20 mg), potassium carbonate (20 mg), and pyrrolidine (20 μL) in 1,4-dioxane (1 mL) was stirred at 100°C for 30 minutes using a microwave reaction apparatus. Water was added

to the reaction mixture, the solid matter was collected by filtration, and the obtained residue was purified by silica gel column chromatography (chloroform-methanol, NH silica), thereby obtaining 2-amino-5-(1-pyrrolidinyl)-1,3,4-thiadiazole (10 mg).
$^1$H-NMR(DMSO-$d_6$)$\delta$:6.27(2H,s),3.30-3.23(4H,m),1.93-1.88(4H,m).

<0173-2>

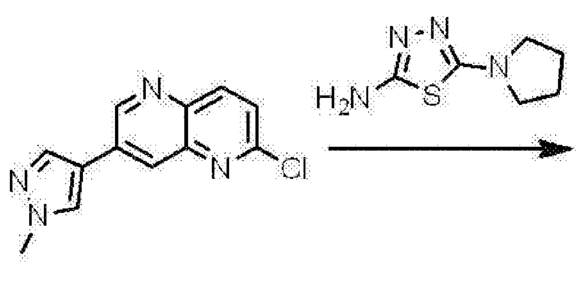

N-(7-(1-methyl-1H-pyrazol-4-yl)-1,5-naphthyridin-2-yl)-5-(pyrrolidin-1-yl)-1,3,4-thiadiazole-2-amine (5 mg) was obtained as a yellow solid in the same manner as in Example 0162 except that 2-amino-5-(1-pyrrolidinyl)-1,3,4-thiadiazole was used instead of the 2-amino-5-tert-butyl-1,3,4-thiadiazole used in Example 0162.
$^1$H-NMR(DMSO-$d_6$)$\delta$:11.55(1H,s),9.01(1H,d,J=2.3Hz),8.50(1H,s),8.22(1H,d,J=1.3Hz),8.20(1H,s),8.16(1H,d,J=8.9Hz), 7 .29(1H,d,J=9.2Hz),3.92(3H,s),3.47(4H,t,J=6.6Hz),2.01(4H,t,J=6.4Hz).
MSm/z(M+H):379.

<Example0174>

<0174-1>

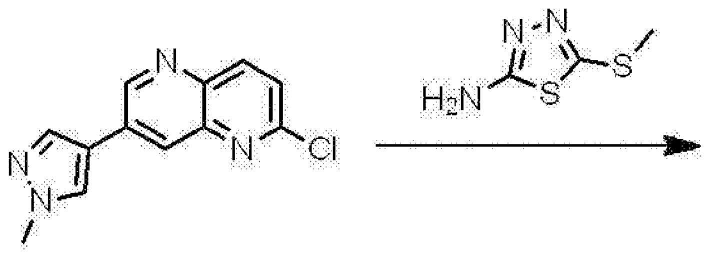

N-(7-(1-methyl-1H-pyrazol-4-yl)-1,5-naphthyridin-2-yl)-5-(methylthio)-1,3,4-thiadiazole-2-amine (2 mg) was obtained as a yellow solid in the same manner as in Example 0162 except that 2-amino-5-(methylthio)-1,3,4-thiadiazole was used instead of the 2-amino-5-tert-butyl-1,3,4-thiadiazole used in Example 0162.
$^1$H-NMR(DMSO-$d_6$)$\delta$:9.08(1H,d,J=2.3Hz),8.54(1H,s),8.39(1H,d,J=1.7Hz),8.26(1H,d,J=9.2Hz),8.23(1H,s),7.38(1H,d,J= 8.9Hz),3.92(3H,s),2.76(3H,s).
MSm/z(M+H):356.

<Example0175>

<0175-1>

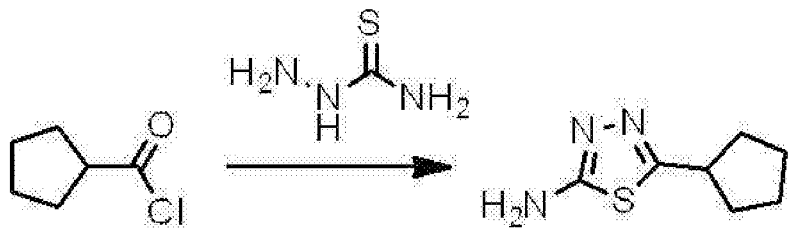

Cyclopentanecarbonyl chloride (3 mL) was added to a solution of thiosemicarbazide (2.0 g) in hydrochloric acid (10 mL), followed by stirring for 60 hours under heating to reflux. The reaction mixture was cooled to room temperature, and neutralized by the addition of a sodium hydroxide aqueous solution under ice-cooling. The solid matter was collected by filtration, and dissolved by the addition of ethyl acetate and methanol. The resultant product was dried over anhydrous sodium sulfate, and passed through silica gel column chromatography (NH silica). The solvent was distilled off under reduced pressure, and the obtained residue was washed with a solution of ethyl acetate-hexane, thereby obtaining 2-amino-5-cyclopentyl-1,3,4-thiadiazole (2.83 g).
$^1$H-NMR(DMSO-$d_6$)δ:6.98(2H,s),3.30-3.20(1H,m),2.07-1.95(2H,m),1.69-1.59(6H,m).

<0175-2>

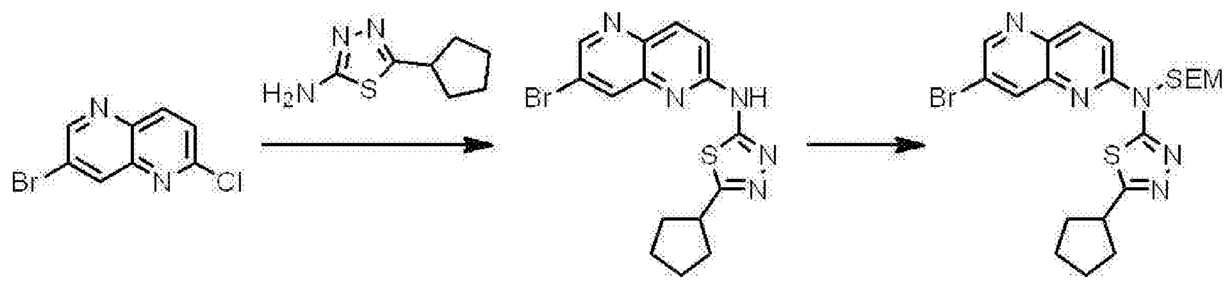

A mixture of 2-amino-5-cyclopentyl-1,3,4-thiadiazole (1.73 g), 7-bromo-2-chloro-1,5-naphthyridine (2.40 g), and potassium carbonate (1.99 g) in dimethylsulfoxide (20 mL) was stirred at 150°C for 1.5 hours. The reaction mixture was cooled to room temperature, and water (300 mL) was added thereto. The solid matter was collected by filtration, thereby obtaining N-(7-bromo-1,5-naphthyridin-2-yl)-5-cyclopentyl-1,3,4-thiadiazole-2-amine (1.55 g).

60% sodium hydride (150 mg) was added to a solution of the obtained N-(7-bromo-1,5-naphthyridin-2-yl)-5-cyclopentyl-1,3,4-thiadiazole-2-amine in N,N-dimethylformamide (25 mL) under ice-cooling, followed by stirring at 0°C for 15 minutes. 2-(Chloromethoxy)ethyltrimethylsilane (700 μL) was added to the reaction mixture, followed by stirring at room temperature for 30 minutes. Methanol (2 mL) was added to the reaction mixture at 0°C, and a saturated sodium chloride aqueous solution and ethyl acetate were added thereto. The organic layer was collected by separation, and dried over anhydrous sodium sulfate, and the solvent was distilled off under reduced pressure. The obtained residue was purified by silica gel column chromatography (hexane-ethyl acetate, NH silica), thereby obtaining N-(7-bromo-1,5-naphthyridin-2-yl)-5-cyclopentyl-N-((2-(trimethylsilyl)ethoxy)methyl)-1,3,4-thiadiazole-2-amine (1.82 g) as brown oily substance.
$^1$H-NMR(DMSO-$d_6$)δ:8.92(1H,d,J=2.0Hz),8.70(1H,d,J=2.0Hz),8.34(1H,d,J=8.9Hz),7.55(1H,d,J=9.2Hz),5.73(2H,s),3.81 (2H,td,J=7.9,3.2Hz),3.58(1H,t,J=7.9Hz),2.28-2.16(2H,m),2.01-1.76(8H,m),0.03(9H,s).

<0175-3>

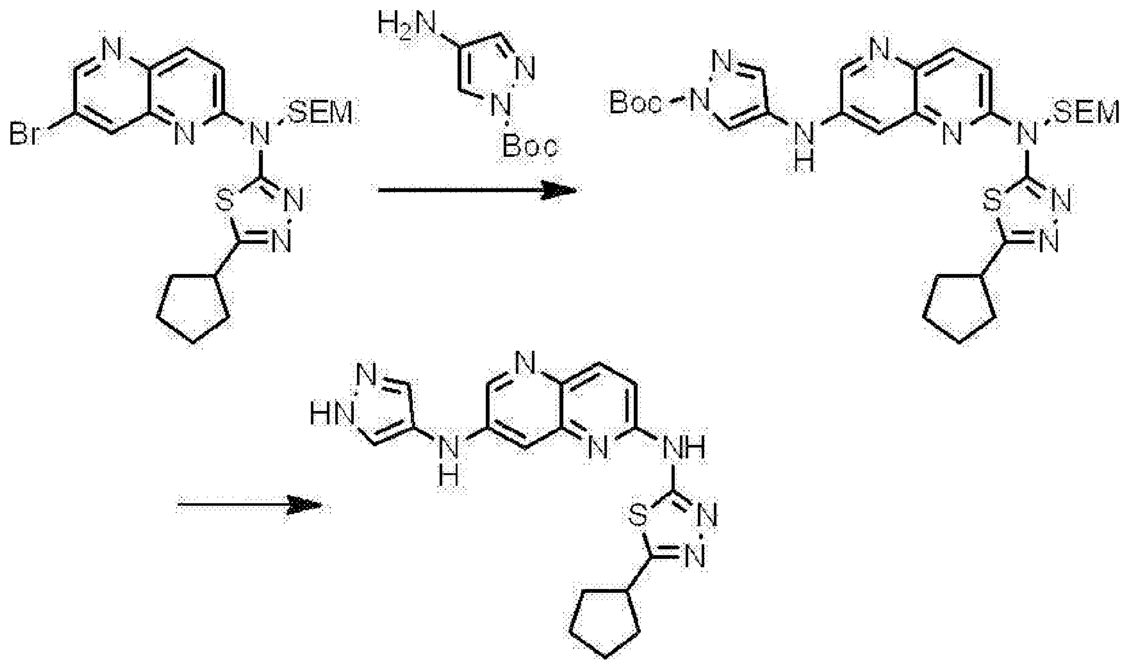

tert-Butyl=4-amino-1H-pyrazole-1-carboxylate (25 mg), 4,5-bis(diphenylphosphino)-9,9-dimethylxanthene (7 mg), cesium carbonate (65 mg), and tris(dibenzylideneacetone)dipalladium(0) (6 mg) were added to a solution of N-(7-bromo-1,5-naphthyridin-2-yl)-5-cyclopentyl-N-((2-(trimethylsilyl)ethoxy)methyl)-1,3,4-thiadiazole-2-amine (50 mg) in 1,4-dioxane (1 mL), followed by stirring at 100°C for 8 hours in a nitrogen atmosphere. 4,5-Bis(diphenylphosphino)-9,9-dimethylxanthene (5 mg) and tris(dibenzylideneacetone)dipalladium(0) (5 mg) were added thereto, followed by stirring at 100°C for 3 hours in a nitrogen atmosphere. The reaction mixture was cooled to room temperature, and water was added thereto. The solid matter was collected by filtration, thereby obtaining tert-butyl 4-((6-((5-cyclopentyl-1,3,4-thiadiazol-2-yl)((2-(trimethylsilyl)ethoxy)methyl)amino)-1,5-naphthyridin-3-yl)amino)--carboxylate.

Hydrochloric acid (1 mL) was added to the obtained tert-butyl 4-((6-((5-cyclopentyl-1,3,4-thiadiazol-2-yl)((2-(trimethylsilyl)ethoxy)methyl)amino)-1,5-naphthyridin-3-yl)amino)-1H-pyrazole-1-carboxylate, followed by stirring at 90°C for 1 hour. The reaction mixture was cooled to room temperature, and the resultant product was neutralized with a sodium hydroxide aqueous solution. The solid matter was collected by filtration, and purified by preparative reversed phase HPLC (a 0.1% formic acid aqueous solution-a 0.1% formic acid acetonitrile solution), thereby obtaining $N^2$-(5-cyclopentyl-1,3,4-thiadiazol-2-yl)-$N^7$-(1H-pyrazol-4-yl)-1,5-naphthyridine-2,7-diamine (4.5 mg) as a yellow solid.

$^1$H-NMR(DMSO-$d_6$)$\delta$:12.82(1H,s),11.79(1H,s),8.42(1H,d,J=2.6Hz),8.36(1H,s),8.05(1H,d,J=8.9Hz), 7.90(1H,s), 7.56(1H,s),7.12(1H,d,J=2.3Hz),7.09(1H,d,J=8.9Hz),3.52-3.40(1H,m),2.16-2.07(2H,m), 1.88-1.62 (6H,m).

MSm/z(M+H):379.

<Example0176>

<0176-1>

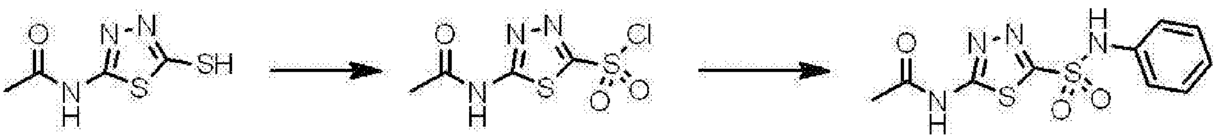

A sodium hypochlorite solution (13 mL) was added dropwise to a mixture solution of dichloromethane (25 mL) and 2 mol/L hydrochloric acid (15 mL) at -10°C, and a solution of 5-acetamide-2-mercapto-1,3,4-thiadiazole (300 mg) in dichloromethane (5 mL) was added dropwise thereto. Then, sodium hydrogen sulfite aqueous solution was added thereto until potassium iodide starch paper was decolorized. The organic layer was collected by separation, washed with a saturated sodium chloride aqueous solution, and dried over anhydrous sodium sulfate, and aniline (400 $\mu$L) was

added thereto under ice-cooling. A saturated ammonium chloride aqueous solution was added to the reaction mixture. The organic layer was collected by separation, and dried over anhydrous sodium sulfate, and the solvent was distilled off under reduced pressure. The obtained residue was purified by silica gel column chromatography (chloroform-methanol), thereby obtaining N-(5-(N-phenylsulfamoyl)-1,3,4-thiadiazol-2-yl)acetamide (161 mg).
$^1$H-NMR(DMSO-$d_6$)δ:13.10(1H,s),11.16(1H,s),7.35-7.29(2H,m),7.20-7.12(3H,m),2.21(3H,s).

<0176-2>

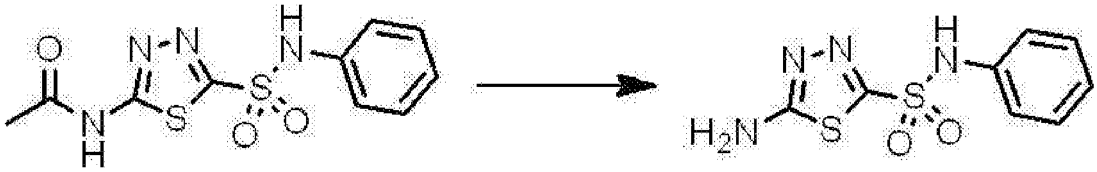

Hydrochloric acid (2 mL) was added to a solution of N-(5-(N-phenylsulfamoyl)-1,3,4-thiadiazol-2-yl)acetamide (155 mg) in ethanol (2 mL), followed by stirring at 90°C for 30 minutes. The reaction mixture was cooled to room temperature, and neutralized with a sodium hydroxide aqueous solution under ice-cooling. Liquid-liquid separation was performed by the addition of chloroform thereto, and the water of the aqueous layer was distilled off under reduced pressure. A solution of chloroform-methanol was added to the residue, the solid was separated by filtration, and the filtrate was purified by silica gel column chromatography (ethyl acetate, NH silica), thereby obtaining 5-amino-N-phenyl-1,3,4-thiadiazole-2-sulfonamide (98 mg).
$^1$H-NMR(DMSO-$d_6$)δ:10.91(1H,s),7.95(2H,s),7.35-7.28(2H,m),7.19-7.10(3H,m).

<0176-3>

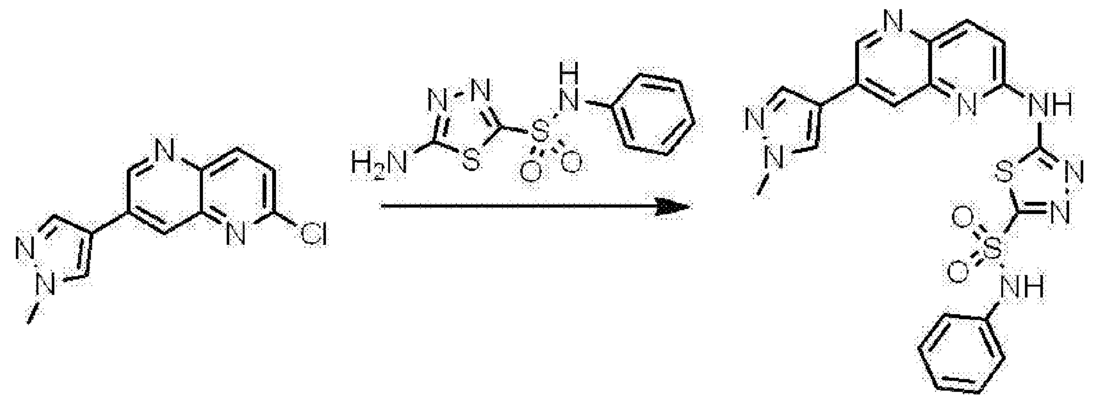

5-Amino-N-phenyl-1,3,4-thiadiazole-2-sulfonamide (20 mg), 4,5-bis(diphenylphosphino)-9,9-dimethylxanthene (6 mg), potassium tert-butoxide (30 mg), and tris(dibenzylideneacetone)dipalladium(0) (5 mg) were added to a solution of 2-chloro-7-(1-methyl-1H-pyrazol-4-yl)-1,5-naphthyridine (15 mg) in 1,4-dioxane (1 mL), followed by stirring at 150°C for 1 hour using a microwave reaction apparatus. The reaction mixture was cooled to room temperature, and chloroform and water were added thereto. The organic layer was collected by separation, and water of the aqueous layer was distilled off under reduced pressure. The obtained residue was purified by preparative reversed phase HPLC (a 0.1% formic acid aqueous solution-a 0.1% formic acid acetonitrile solution), thereby obtaining 5-((7-(1-methyl-1H-pyrazol-4-yl)-1,5-naphthyridin-2-yl)amino)-N-phenyl-1,3,4-thiadiazole-2-sulfonamide (1.2 mg) as a pale yellow solid.
$^1$H-NMR(DMSO-$d_6$)δ:12.81(1H,s),11.15(1H,s),9.14(1H,d,J=2.3Hz),8.56(1H,s),8.33-8.32(2H,m),8.24(1H,s),7.46(1H,d,J=8.9Hz),7.36-7.29(2H,m),7.27-7.23(2H,m),7.11(1H,t,J=7.1Hz),3.94(3H,s).
MSm/z(M+H):465.

<Example 0177>

<0177-1>

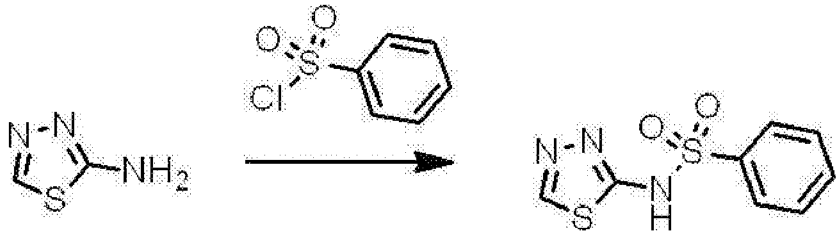

Benzenesulfonyl chloride (300 μL) was added dropwise to a solution of 2-amino-1,3,4-thiadiazole (202 mg) in pyridine (4 mL) over a period of 5 minutes under ice-cooling, followed by stirring at 0°C for 30 minutes. Benzenesulfonyl chloride (150 μL) was added dropwise thereto over a period of 5 minutes, followed by stirring at 80°C for 30 minutes. The solvent was distilled off under reduced pressure, and the resultant product was neutralized by the addition of hydrochloric acid. The organic layer was collected by separation, and dried over anhydrous sodium sulfate, and the solvent was distilled off under reduced pressure. The obtained residue was purified by silica gel column chromatography (hexane-ethyl acetate), thereby obtaining N-(1,3,4-thiadiazol-2-yl)benzene sulfonamide (240 mg).
$^1$H-NMR(DMSO-$d_6$)δ:8.76(1H,s),7.83-7.77(2H,m),7.63-7.53(3H,m).

<0177-2>

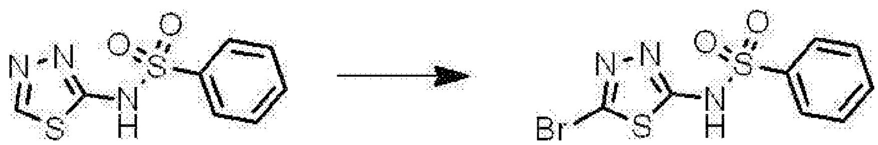

A mixture of N-(1,3,4-thiadiazol-2-yl)benzene sulfonamide (120 mg), and sodium acetate (80 mg) in acetic acid (3 mL) was stirred at 80°C for 5 minutes. Bromine (30 μL) was added dropwise thereto, followed by stirring at 80°C for 30 minutes. Bromine (10 μL) was added dropwise thereto, followed by stirring at 80°C for 30 minutes. After the reaction mixture was cooled to room temperature, a sodium hydrogen sulfite aqueous solution was added thereto until potassium iodide starch paper was decolorized, and the resultant product was neutralized by the addition of a sodium hydroxide aqueous solution. The solid matter was collected by filtration, thereby obtaining N-(5-bromo-1,3,4-thiadiazol-2-yl)benzene sulfonamide (190 mg).
$^1$H-NMR(DMSO-$d_6$)δ:7.84-7.77(2H,m),7.67-7.54(3H,m).

<0177-3>

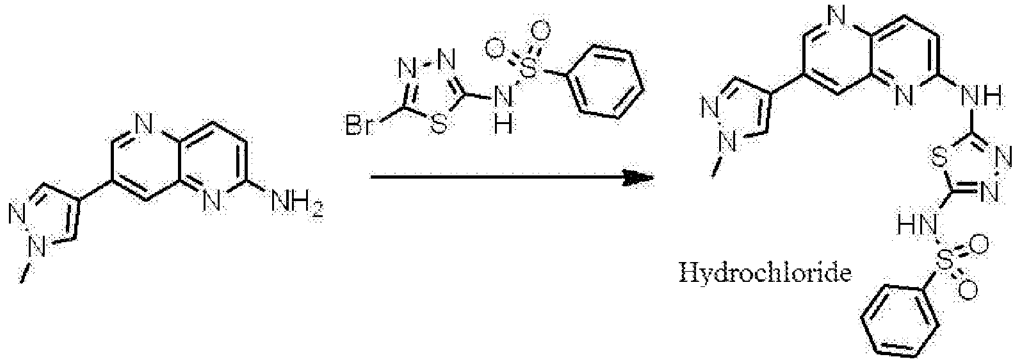


4,5-Bis(diphenylphosphino)-9,9-dimethylxanthene (10 mg), potassium tert-butoxide (60 mg), and tris(dibenzylideneacetone)dipalladium(0) (10 mg) were added to a solution of 7-(1-methyl-1H-pyrazol-4-yl)-1,5-naphthyridine-2-amine (20 mg) and N-(5-bromo-1,3,4-thiadiazol-2-yl)benzene sulfonamide (30 mg) in 1,4-dioxane (1 mL), followed by stirring at 170°C for 2 hours using a microwave reaction apparatus. The reaction mixture was cooled to room temperature, and ethyl acetate and water were added thereto. Liquid-liquid separation was performed, and the water of the aqueous layer was distilled off under reduced pressure. The obtained residue was purified by preparative reversed phase HPLC (a 0.1% formic acid aqueous solution-a 0.1% formic acid acetonitrile solution), thereby obtaining N-(5-((7-(1-methyl-1H-pyrazol-4-yl)-1,5-naphthyridin-2-yl)amino)-1,3,4-thiadiazol-2-yl)benzene sulfonamide (5 mg).

A solution (1 mL) of 4 mol/L hydrogen chloride/1,4-dioxane was added to the obtained N-(5-((7-(1-methyl-1H-pyrazol-4-yl)-1,5-naphthyridin-2-yl)amino)-1,3,4-thiadiazol-2-yl)benzene sulfonamide, and the solvent was distilled off under reduced pressure, thereby obtaining N-(5-((7-(1-methyl-1H-pyrazol-4-yl)-1,5-naphthyridin-2-yl)amino)-1,3,4-thiadiazol-2-yl)benzene sulfonamide hydrochloride (3 mg) as a white solid.

$^1$H-NMR(DMSO-$d_6$)δ:9.09(1H,d,J=2.0Hz),8.51(1H,s),8.28(1H,d,J=9.6Hz),8.18(1H,s),8.09(1H,d,J=1.7Hz),7.90-7.87(2H,m),7.79-7.42(4H,m),7.32(1H,d,J=8.9Hz),3.94(3H,s).

MSm/z(M+H):465.

<Example 0178>

<0178-1>

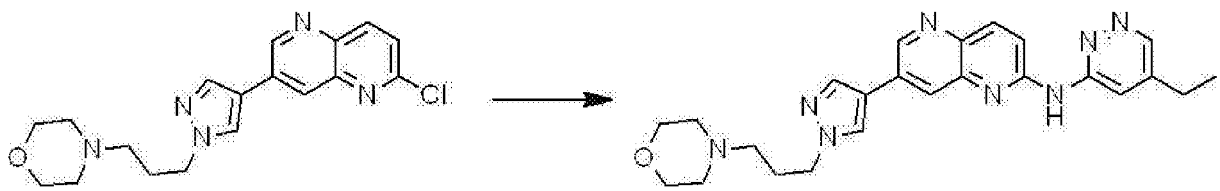

N-(5-ethylpyridazin-3-yl)-7-(1-(3-morpholinopropyl)-1H-pyrazol-4-yl)-1,5-naphthyridine-2-amine was obtained as a white solid in the same manner as in Example 0001 except that 5-ethylpyridazine-3-amine was used instead of the 1,3,4-thiadiazole-2-amine used in Example 0001.

$^1$H-NMR($CDCl_3$)δ:9.07(1H,brs),8.93(1H,d,J=2.1Hz),8.84(1H,m),8.79(1H,d,J=1.5Hz),8.24(1H,d,J=9.3Hz) ,8.13(1H,d,J=1.5Hz),7.97(1H,s),7.88(1H,s),7.58(1H,d,J=9.3Hz),4.31(2H,m),3.73(4H,m),2.81(2H,m),2 .50-2.34(6H,m),2.13(2H,m),1.42(3H,t,J=8.1Hz).

MSm/z(M+H):445.

<Example 0179>

<0179-1>

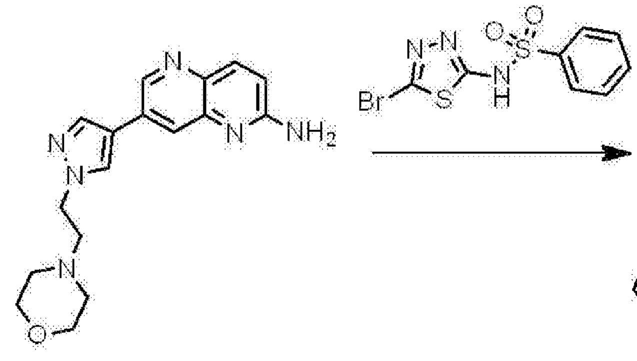

N-(5-((7-(1-(2-morpholinoethyl)-1H-pyrazol-4-yl)-1,5-naphthyridin-2-yl)amino)-1,3,4-thiadiazol-2-yl)benzene sulfonamide (1.2 mg) was obtained as a yellow solid in the same manner as in Example 0177 except that 7-(1-(2-morpholinoethyl)-1H-pyrazol-4-yl)-1,5-naphthyridine-2-amine was used instead of the 7-(1-methyl-1H-pyrazol-4-yl)-1,5-naphthyridine-2-amine used in Example 0177.
$^1$H-NMR(DMSO-$d_6$)δ:11.86(1H,s),9.09(1H,d,J=2.0Hz),8.55(1H,s),8.27(1H,d,J=9.2Hz),8.18(1H,s),8.08(1H,d,J=1.7Hz),7 .90-7.87(2H,m),7.79-7.42(4H,m),7.32(1H,d,J=8.9Hz),4.33(2H,t,J=6.4Hz),3.56(4H,t,J=4.6Hz),2.80(2H,t,J=6.6Hz),2.50-2.42(4H,m).
MSm/z(M+H):564.

<Example 0180>

<0180-1>

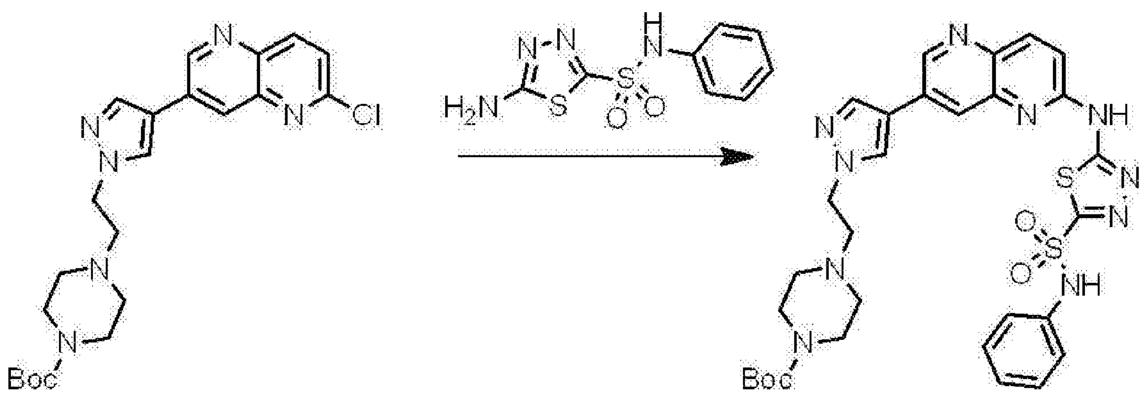

tert-Butyl 4-(2-(4-(6-((5-(N-phenyl sulfamoyl)-1,3,4-thiadiazol-2-yl)amino)-1,5-naphthyridin-3-yl)-1H-pyrazol-1-yl)ethyl)piperazine-1-carboxylate (55 mg) was obtained as a pale yellow solid in the same manner as in Example 0176 except that tert-butyl 4-(2-(4-(6-chloro-1,5-naphthyridin-3-yl)-1H-pyrazol-1-yl)ethyl)piperazine-1-carboxylate was used instead of the 2-chloro-7-(1-methyl-1H-pyrazol-4-yl)-1,5-naphthyridine used in Example 0176.
$^1$H-NMR(DMSO-$d_6$)δ:9.06(1H,d,J=2.0Hz),8.62(1H,s),8.37-8.32(2H,m),8.24(1H,s),7.37(1H,d,J=9.2Hz),7.03-6.94(5H,m),4.34-4.27(2H,m),3.33-3.25(4H,m),2.86-2.78(2H,m),2.44-2.38(4H,m)1.38(9H,s).
MSm/z(M+H):663.

<Example 0181>

<0181-1>

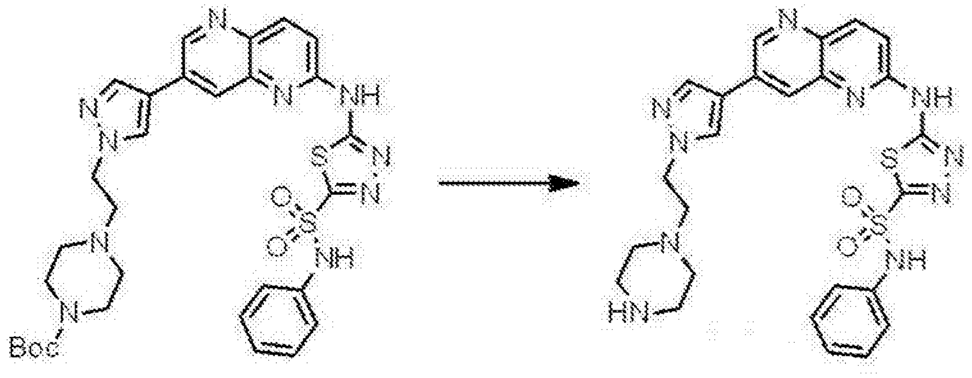

HCL salt

A solution (2 mL) of 4 mol/L hydrogen chloride/1,4-dioxane was added to tert-butyl 4-(2-(4-(6-((5-(N-phenylsulfamoyl)-1,3,4-thiadiazol-2-yl)amino)-1,5-naphthyridin-3-yl)-1H-pyrazol-1-yl)ethyl)piperazine-1-carboxylate (52 mg), followed by stirring at room temperature for 30 minutes. The solvent was distilled off under reduced pressure, and the obtained residue was washed with a solution of ethyl acetate-methanol-chloroform, thereby obtaining N-phenyl-5-((7-(1-(2-(piperazin-1-yl)ethyl)-1H-pyrazol-4-yl)-1,5-naphthyridin-2-yl)amino)-1,3,4-thiadiazole-2-sulfonamide hydrochloride (41 mg) as a pale yellow solid.

$^{1}$H-NMR(DMSO-$d_6$)δ:12.90(1H,s),11.21(1H,s),9.17(1H,d,J=2.0Hz),8.69(1H,s),8.37-8.32(2H,m),8.24(1H,s),7.52(1H,d,J=8.9Hz),7.36-7.29(2H,m),7.27-7.23(2H,m),7.11(1H,t,J=7.1Hz),4.66-4.56(2H,m),3.57-3.56(4H,m),3.39-3.27(6H,m).
MSm/z(M+H):563.

<Example 0182>

<0182-1>

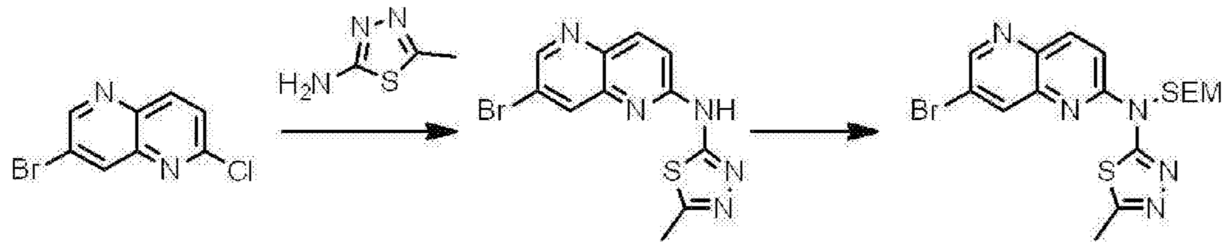

N-(7-bromo-1,5-naphthyridin-2-yl)-5-methyl-N-((2-(trimethylsilyl)ethoxy)methyl)-1,3,4-thiadiazole-2-amine (660 mg) was obtained as brown oily substance in the same manner as in Example 0175 except that 2-amino-5-methyl-1,3,4-thiadiazole was used instead of the 2-amino-5-cyclopentyl-1,3,4-thiadiazole used in Example 0175.
MSm/z(M+H):452.

<0182-2>

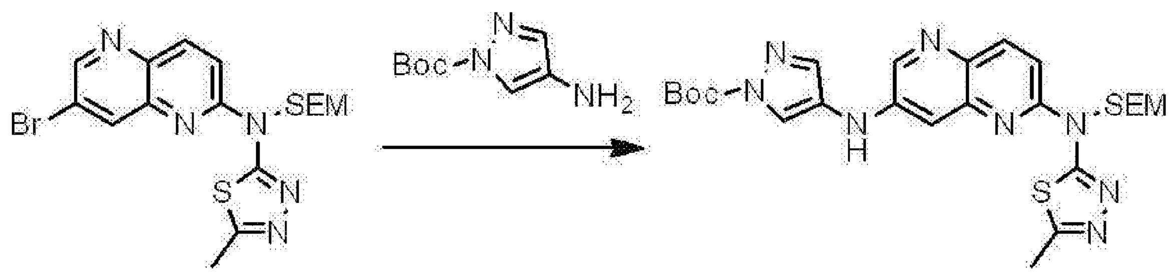

tert-Butyl=4-amino-1H-pyrazole-1-carboxylate (17 mg), 4,5-bis(diphenylphosphino)-9,9-dimethylxanthene (6 mg), cesium carbonate (65 mg), and tris(dibenzylideneacetone)dipalladium(0) (6 mg) were added to a solution of N-(7-bromo-1,5-naphthyridin-2-yl)-5-methyl-N-((2-(trimethylsilyl)ethoxy)methyl)-1,3,4-thiadiazole-2-amine (49 mg) in 1,4-dioxane (1 mL), followed by stirring at 100°C in a nitrogen atmosphere overnight. The reaction liquid was cooled to room temperature, and water and ethyl acetate were added thereto. The organic layer was collected by separation, and dried over anhydrous sodium sulfate, and the solvent was distilled off under reduced pressure. The obtained residue was purified by silica gel column chromatography (chloroform-methanol, NH silica), thereby obtaining tert-butyl 4-((6-((5-methyl-1,3,4-thiadiazol-2-yl)((2-(trimethylsilyl)ethoxy)methyl)amino)-1,5-naphthyridin-3-yl)amino)-1H-pyrazole-1-carboxylate (25 mg).
MSm/z(M+H):555.

<0182-3>

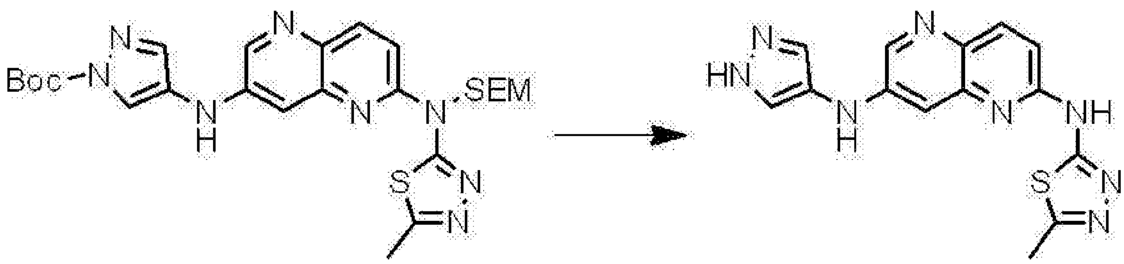

A 4 mol/L hydrogen chloride/1,4-dioxane solution (1 mL) was added to a solution of tert-butyl 4-((6-((5-methyl-1,3,4-thiadiazol-2-yl)((2-(trimethylsilyl)ethoxy)methyl)amino)-1,5-naphthyridin-3-yl)amino)-1H-pyrazole-1-carboxylate (10 mg) in methanol (500 μL), followed by stirring at 60°C for 1.5 hours. The solvent was distilled off under reduced pressure, and a saturated sodium hydrogen carbonate aqueous solution was added to the obtained residue. The solid matter was collected by filtration, thereby obtaining $N^2$-(5-methyl-1,3,4-thiadiazol-2-yl)-$N^7$-(1H-pyrazol-4-yl)-1,5-naphthyridine-2,7-diamine (3.6 mg) as a yellow solid.
$^1$H-NMR(DMSO-$d_6$)δ:12.32(1H,s),11.82(1H,s),9.27(1H,s),8.56(1H,d,J=2.6Hz),8.34(1H,s),8.06(1H,d,J=8.9Hz),7.68(1H,d,J=2.3Hz),7.12(1H,d,J=8.9Hz),5.95(1H,d,J=2.3Hz),2.64(3H,s).
MSm/z(M+H):325.

<Example 0183>

<0183-1>

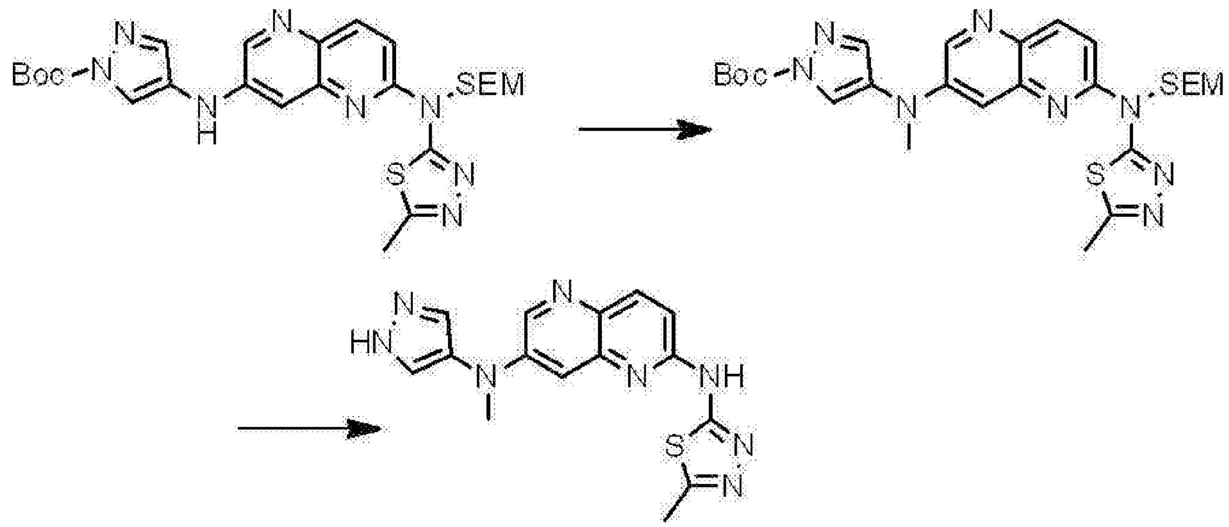

60% sodium hydride (3 mg) was added to a solution of tert-butyl 4-((6-((5-methyl-1,3,4-thiadiazol-2-yl)((2-(trimethylsilyl)ethoxy)methyl)amino)-1,5-naphthyridin-3-yl)amino)-1H-pyrazole-1-carboxylate (10 mg) in N,N-dimethylformamide (1 mL) under ice-cooling, and iodomethane (10 $\mu$L) was added thereto, followed by stirring at room temperature for 30 minutes. Methanol (1 drop) was added to the reaction mixture, and water and ethyl acetate were added thereto. The organic layer was collected by separation, and dried over anhydrous sodium sulfate, the solvent was distilled off under reduced pressure, and the obtained residue was purified by silica gel column chromatography (hexane-ethyl acetate, NH silica), thereby obtaining tert-butyl 4-(methyl (6-((5-methyl-1,3,4-thiadiazol-2-yl)((2-(trimethylsilyl)ethoxy)methyl)amino)-1,5-naphthyridin-3-yl)amino)-1H-pyrazole-1-carboxylate.

Methanol (500 $\mu$L) and a 4 mol/L hydrogen chloride/1,4-dioxane solution (1 mL) were added to the obtained tert-butyl 4-(methyl (6-((5-methyl-1,3,4-thiadiazol-2-yl)((2-(trimethylsilyl)ethoxy)methyl)amino)-1,5-naphthyridin-3-yl)amino)-1H-pyrazole-1-carboxylate, followed by stirring at 60°C for 1.5 hours. The solvent was distilled off under reduced pressure, the obtained residue was neutralized by the addition of a saturated sodium hydrogen carbonate aqueous solution, and a solution of methanol-chloroform was added thereto. The organic layer was collected by separation, and dried over anhydrous sodium sulfate, and the solvent was distilled off under reduced pressure. The obtained residue was purified by silica gel column chromatography (hexane-ethyl acetate, NH silica), thereby obtaining $N^7$-methyl-$N^2$-(5-methyl-1,3,4-thiadiazol-2-yl)-$N^7$-(1H-pyrazol-4-yl)-1,5-naphthyridine-2,7-diamine (8.5 mg) as a yellow solid.

$^1$H-NMR(DMSO-$d_6$)$\delta$:12.54(1H,s),11.86(1H,s),8.66(1H,d,J=2.6Hz),8.12(1H,d,J=8.9Hz),7.77(1H,d,J=2.3Hz),7.52(1H,d,J=2.3Hz),7.19(1H,d,J=8.9Hz),6.19(1H,d,J=2.3Hz),3.43(3H,s),2.64(3H,s).

MSm/z(M+H):339.

<Example 0184>

<0184-1>

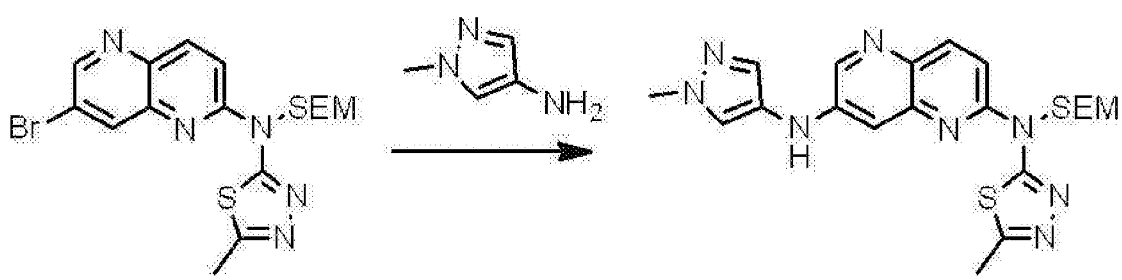

$N^2$-(5-methyl-1,3,4-thiadiazol-2-yl)-$N^7$-(1-methyl-1H-pyrazol-4-yl)-$N^2$-((2-(trimethylsilyl)ethoxy)methyl)-1,5-naphthyridine-2,7-diamine (67 mg) was obtained as a yellow solid in the same manner as in Example 0182 except that 1-methyl-1H-pyrazole-4-amine was used instead of the tert-butyl=4-amino-1H-pyrazole-1-carboxylate used in Example

0182.
MSm/z(M+H):469.

<0184-2>

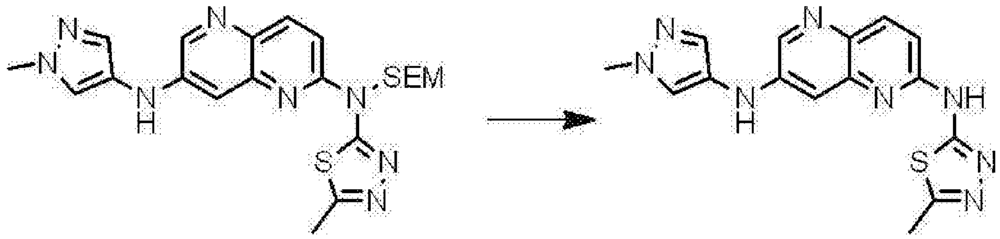

$N^2$-(5-methyl-1,3,4-thiadiazol-2-yl)-$N^7$-(-(1-methyl-1H-pyrazol-4-yl)-1,5-naphthyridine-2,7-diamine (15 mg) was obtained as a yellow solid in the same manner as in Example 0182 except that $N^2$-(5-methyl-1,3,4-thiadiazol-2-yl)-$N^7$-(-(1-methyl-1H-pyrazol-4-yl)-$N^2$-((2-(trimethylsilyl)ethoxy)methyl)-1,5-naphthyridine-2,7-diamine was used instead of the tert-butyl 4-((6-((5-methyl-1,3,4-thiadiazol-2-yl)((2-(trimethylsilyl)ethoxy)methyl)amino)-1,5-naphthyridin-3-yl)amino)-1H-pyrazole-1-carboxylate used in Example 0182.
$^1$H-NMR(DMSO-$d_6$)δ:11.77(1H,s),9.21(1H,s),8.58(1H,d,J=2.6Hz),8.17(1H,d,J=2.3Hz),8.04(1H,d,J=8.9Hz),7.63(1H,d,J =2.3Hz),7.11(1H,d,J=8.9Hz),5.93(1H,d,J=2.0Hz),3.85(3H,s),2.64(3H,s).
MSm/z(M+H):339.

<Example 0185>

<0185-1>

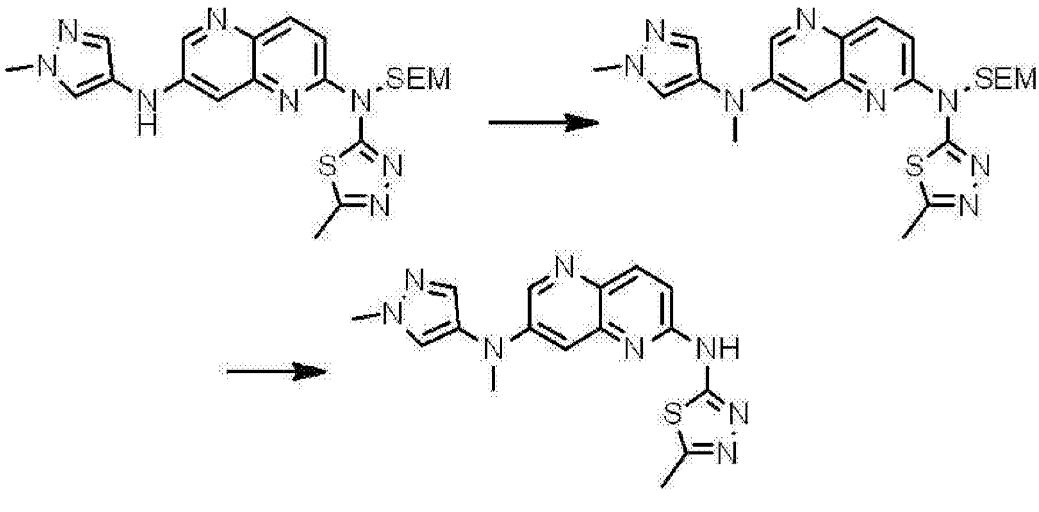

$N^7$-methyl-$N^2$-(5-methyl-1,3,4-thiadiazol-2-yl)-$N^7$-(1-methyl-1H-pyrazol-4-yl)-1,5-naphthyridine-2,7-diamine (3.5 mg) was obtained as a yellow solid in the same manner as in Example 0183 except that $N^2$-(5-methyl-1,3,4-thiadiazol-2-yl)-$N^7$-(1-methyl-1H-pyrazol-4-yl)-$N^2$-((2-(trimethylsilyl)ethoxy)methyl)-1,5-naphthyridine-2,7-diamine was used instead of the tert-butyl 4-((6-((5-methyl-1,3,4-thiadiazol-2-yl)((2-(trimethylsilyl)ethoxy)methyl)amino)-1,5-naphthyridin-3-yl)amino)-1H-pyrazole-1-carboxylate used in Example 0183.
$^1$H-NMR(DMSO-$d_6$)δ:11.87(1H,s),8.68(1H,d,J=2.6Hz),8.12(1H,d,J=8.9Hz),7.71(1H,d,J=2.0Hz),7.49(1H,d,J=2.6Hz),7.2 0(1H,d,J=8.9Hz),6.14(1H,d,J=2.3Hz),3.81(3H,s),3.41(3H,s),2.64(3H,s).
MSm/z(M+H):353.

<Example 0186>

<0186-1>

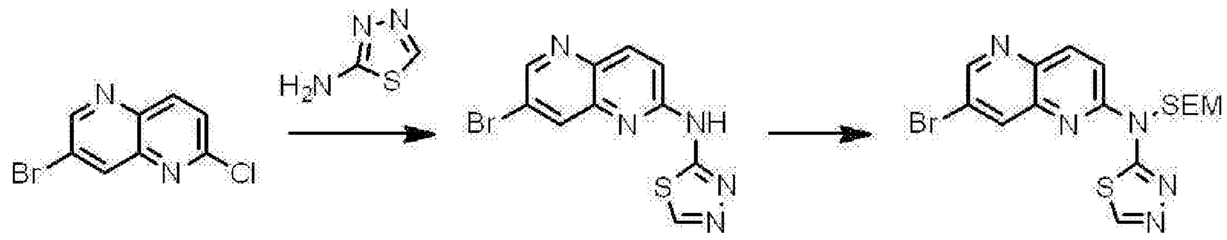

N-(7-bromo-1,5-naphthyridin-2-yl)-N-((2-(trimethylsilyl)ethoxy)methyl)-1,3,4-thiadiazole-2-amine (175 mg) was obtained in the same manner as in Example 0175 except that 2-amino-1,3,4-thiadiazole was used instead of the 2-amino-5-cyclopentyl-1,3,4-thiadiazole used in Example 0175.
MSm/z(M+H):438,440.

<0186-2>

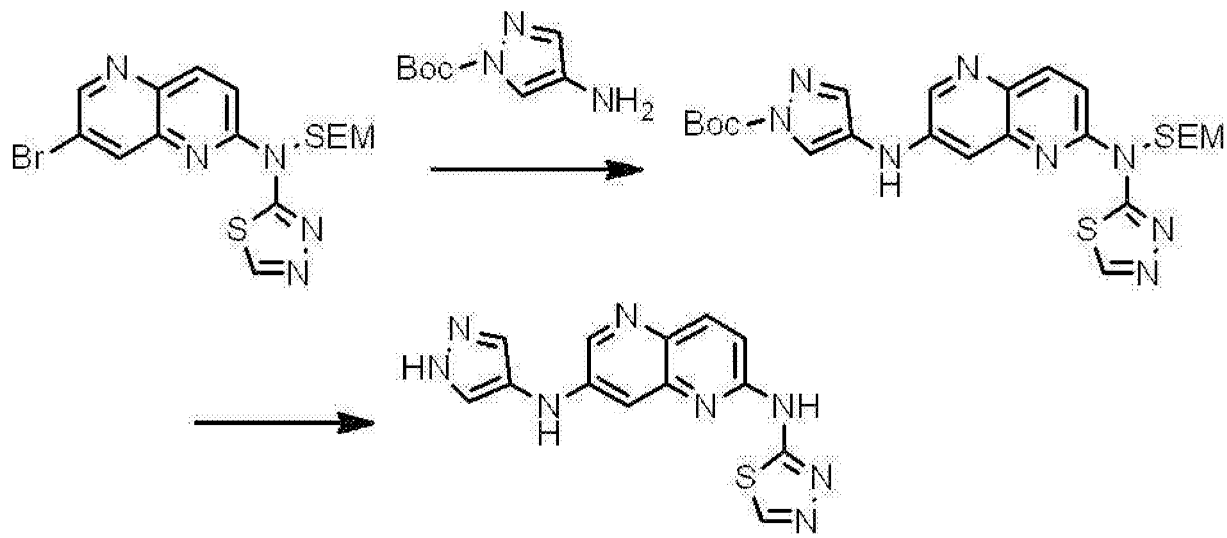

$N^7$-(1H-pyrazol-4-yl)-$N^2$-(1,3,4-thiadiazol-2-yl)-1,5-naphthyridine-2,7-diamine (11.9 mg) was obtained as a yellow solid in the same manner as in Example 0175 except that N-(7-bromo-1,5-naphthyridin-2-yl)-N-((2-(trimethylsilyl)ethoxy)methyl)-1,3,4-thiadiazole-2-amine was used instead of the N-(7-bromo-1,5-naphthyridin-2-yl)-5-cyclopentyl-N-((2-(trimethylsilyl)ethoxy)methyl)-1,3,4-thiadiazole-2-amine used in Example 0175.
$^1$H-NMR(DMSO-$d_6$)$\delta$:12.32(1H,s),12.03(1H,s),9.32(1H,s),9.10(1H,s),8.60(1H,d,J=2.6Hz),8.40(1H,d,J=2.3Hz),8.12(1H, d,J=8.9Hz),7.69(1H,t,J=2.0Hz),7.19(1H,d,J=8.9Hz),5.96(1H,t,J=2.1Hz).
MSm/z(M+H):311.

<Example 0187>

<0187-1>

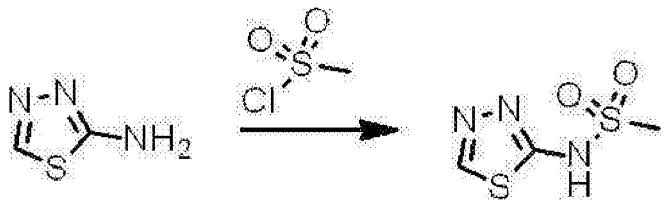

N-(1,3,4-thiadiazol-2-yl)methane sulfonamide (310 mg) was obtained in the same manner as in Example 0177 except that methanesulfonyl chloride was used instead of the benzenesulfonyl chloride used in Example 0177. MSm/z(M+H):180.

<0187-2>

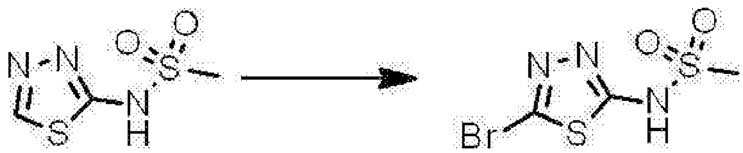

N-(5-bromo-1,3,4-thiadiazol-2-yl)methane sulfonamide (145 mg) was obtained in the same manner as in Example 0177 except that N-(1,3,4-thiadiazol-2-yl)methane sulfonamide was used instead of the N-(1,3,4-thiadiazol-2-yl)benzene sulfonamide used in Example 0177.
MSm/z(M+H):258,260.

<0187-3>

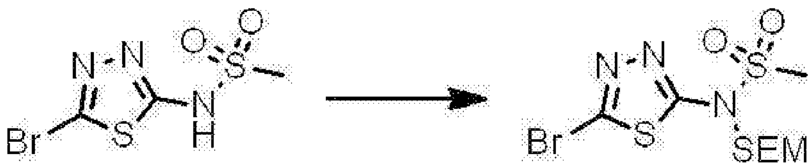

60% sodium hydride (15 mg) was added to a solution of N-(5-bromo-1,3,4-thiadiazol-2-yl)methane sulfonamide (100 mg) in N,N-dimethylformamide (10 mL) under ice-cooling, followed by stirring at 0°C for 15 minutes. 2-(Chloromethoxy)ethyltrimethylsilane (60 μL) was added to the reaction mixture, followed by stirring at room temperature for 30 minutes. After the reaction mixture was cooled to 0°C, methanol (2 drops) was added thereto, and a saturated sodium chloride aqueous solution and ethyl acetate were added thereto. The organic layer was collected by separation, and dried over anhydrous sodium sulfate, and the solvent was distilled off under reduced pressure. The obtained residue was purified by silica gel column chromatography (hexane-ethyl acetate), thereby obtaining N-(5-bromo-1,3,4-thiadiazol-2-yl)-N-((2-(trimethylsilyl)ethoxy)methyl)methane sulfonamide (100 mg) as oily substance.
MSm/z(M+H):388,390.

<0187-4>

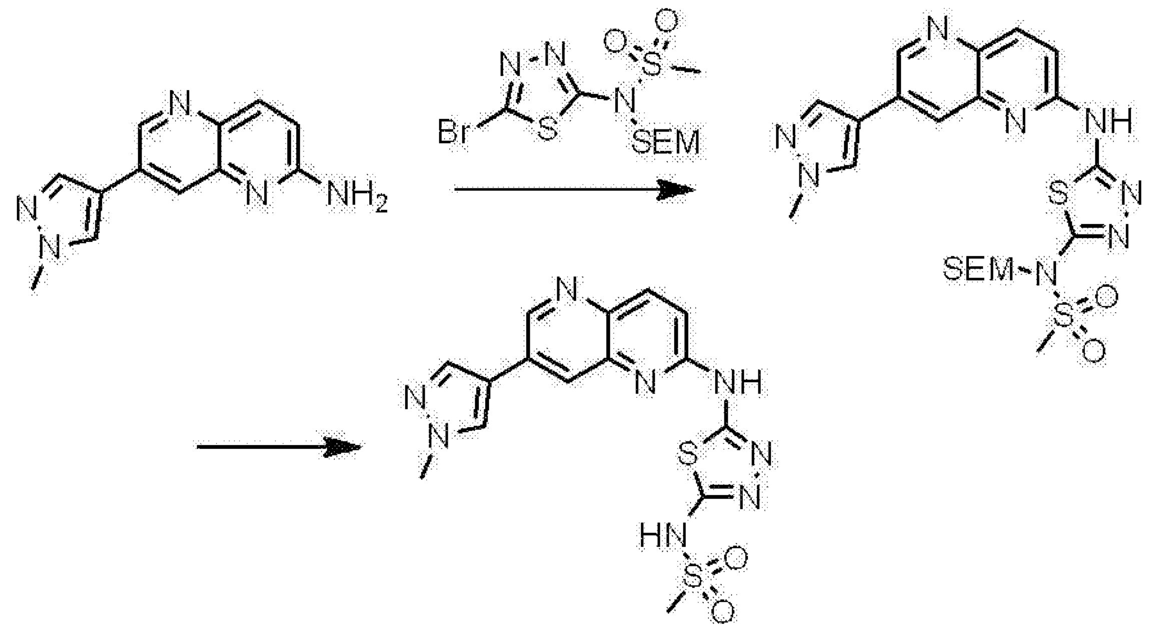

4,5-Bis(diphenylphosphino)-9,9-dimethylxanthene (10 mg), potassium tert-butoxide (20 mg), andtris(dibenzylideneacetone)dipalladium(0) (10 mg) were added to a solution of 7-(1-methyl-1H-pyrazol-4-yl)-1,5-naphthyridine-2-amine (25 mg) and N-(5-bromo-1,3,4-thiadiazol-2-yl)-N-((2-(trimethylsilyl)ethoxy)methyl)methane sulfonamide (45 mg) in 1,4-dioxane (1 mL), followed by stirring at 150°C for 2 hour using a microwave reaction apparatus. The reaction mixture was cooled to room temperature, and chloroform and water were added thereto. The organic layer was collected by separation, and the solvent was distilled off under reduced pressure. The obtained residue was purified by silica gel column chromatography (ethyl acetate-methanol), thereby obtaining N-(5-((7-(1-methyl-1H-pyrazol-4-yl)-1,5-naphthyridin-2-yl)amino)-1,3,4-thiadiazol-2-yl)-N-((2-(trimethylsilyl)ethoxy)methyl)methane sulfonamide (20 mg).

A 4 mol/L hydrogen chloride/1,4-dioxane solution (1 mL) was added to a solution of the obtained N-(5-((7-(1-methyl-1H-pyrazol-4-yl)-1,5-naphthyridin-2-yl)amino)-1,3,4-thiadiazol-2-yl)-N-((2-(trimethylsilyl)ethoxy)methyl)methane sulfonamide in ethanol (1 mL), followed by stirring at 65°C for 2 hours. The reaction mixture was cooled to room temperature, and the solvent was distilled off under reduced pressure. A saturated sodium hydrogen carbonate aqueous solution was added to the obtained residue, and the solvent was distilled off under reduced pressure. The obtained residue was purified by preparative reversed phase HPLC (a 0.1% formic acid aqueous solution-a 0.1% formic acid acetonitrile solution), thereby obtaining N-(5-((7-(1-methyl-1H-pyrazol-4-yl)-1,5-naphthyridin-2-yl)amino)-1,3,4-thiadiazol-2-yl)methane sulfonamide (0.5 mg) as a pale yellow solid.

$^1$H-NMR(DMSO-$d_6$)δ:11.10(1H,s),8.96(1H,d,J=2.3Hz),8.47(1H,s),8.13-8.09(2H,m),8.02(1H,d,J=1.7Hz),7.30(1H,d,J=9.2Hz),3.92(3H,s),2.74(3H,s).

MSm/z(M+H):403.

<Example 0188>

<0188-1>

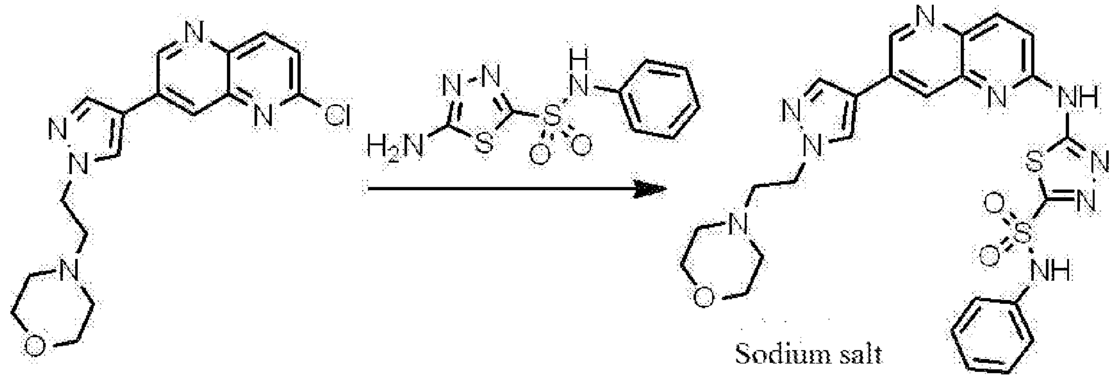


A 5-((7-(1-(2-morpholinoethyl)-1H-pyrazol-4-yl)-1,5-naphthyridin-2-yl)amino)-N-phenyl-1,3,4-thiadiazole-2-sulfonamide sodium salt (11.9 mg) was obtained as a pale yellow solid in the same manner as in Example 0176 except that 4-(2-(4-(6-chloro-1,5-naphthyridin-3-yl)-1H-pyrazol-1-yl)ethyl)morpholine was used instead of the 2-chloro-7-(1-methyl-1H-pyrazol-4-yl)-1,5-naphthyridine used in Example 0176.
$^1$H-NMR(DMSO-$d_6$)δ:12.18(1H,s),9.07(1H,d,J=1.7Hz),8.61(1H,s),8.25-8.20(3H,m),7.38(1H,d,J=8.9Hz),7.07-6.98(4H,m),6.70-6.63(1H,m),4.30(2H,t,J=6.6Hz),3.57(4H,t,J=4.5Hz),2.80(2H,t,J=6.6Hz),2.45(4H,t,J=4.6Hz).
MSm/z(M+H):564.

<Example 0189>

<0189-1>

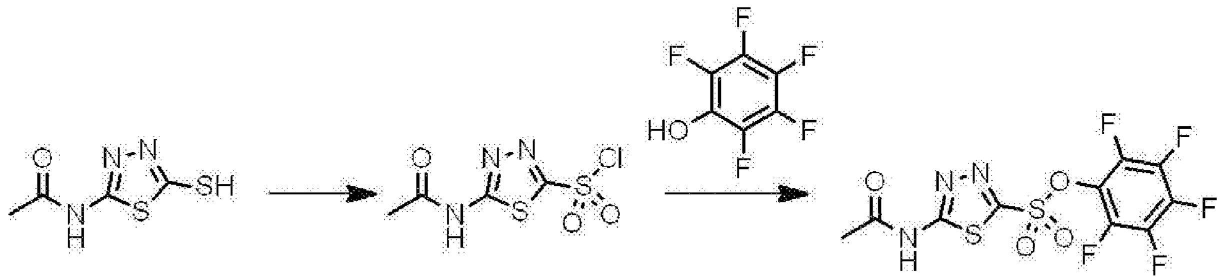

A solution of 5-acetamide-2-mercapto-1,3,4-thiadiazole (1.0 g) in dichloromethane (20 mL) was added dropwise to a mixture of dichloromethane (15 mL), 2 mol/L hydrochloric acid (15 mL), and a sodium hypochlorite solution (10 mL) at -10°C. A sodium hydrogen sulfite aqueous solution was added thereto until potassium iodide starch paper was decolorized. The organic layer was collected by separation, washed with a saturated sodium chloride aqueous solution, and dried over anhydrous sodium sulfate, then, pentafluorophenol (1.0 g) and triethylamine (2.0 mL) were added thereto under ice-cooling, followed by stirring for 5 minutes, and the solvent was distilled off under reduced pressure. The obtained residue was purified by silica gel column chromatography (hexane-ethyl acetate), thereby obtaining pentafluorophenyl 5-acetamide-1,3,4-thiadiazole-2-sulfonate (210 mg).
$^1$H-NMR($CDCl_3$)δ:11.61(1H,s),2.52(3H,s).

<0189-2>

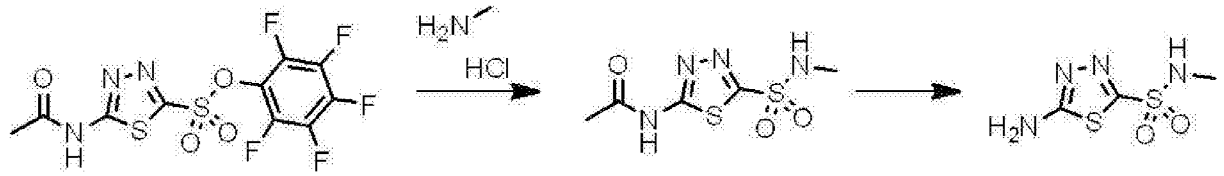


Methylamine hydrochloride (20 mg) and triethylamine (100 μL) were added to a solution of pentafluorophenyl 5-acetamide-1,3,4-thiadiazole-2-sulfonate (70 mg) in acetonitrile (1 mL), followed by stirring at room temperature for 30 minutes. The solvent was distilled off under reduced pressure, and the obtained residue was purified by silica gel column chromatography (chloroform-methanol), thereby obtaining N-(5-(N-methylsulfamoyl)-1,3,4-thiadiazol-2-yl)acetamide.

Hydrochloric acid (1 mL) was added to the obtained N-(5-(N-methylsulfamoyl)-1,3,4-thiadiazol-2-yl)acetamide, followed by stirring at 60°C for 1 hour. The reaction mixture was cooled to room temperature, and neutralized with a sodium hydroxide aqueous solution, and the solvent was distilled off under reduced pressure. The obtained residue was purified by silica gel column chromatography (chloroform-methanol), thereby obtaining 5-amino-N-methyl-1,3,4-thiadiazole-2-sulfonamide (37 mg).
$^1$H-NMR($CDCl_3$)δ:1.25(3H,s).
MSm/z(M+H):195.

<0189-3>

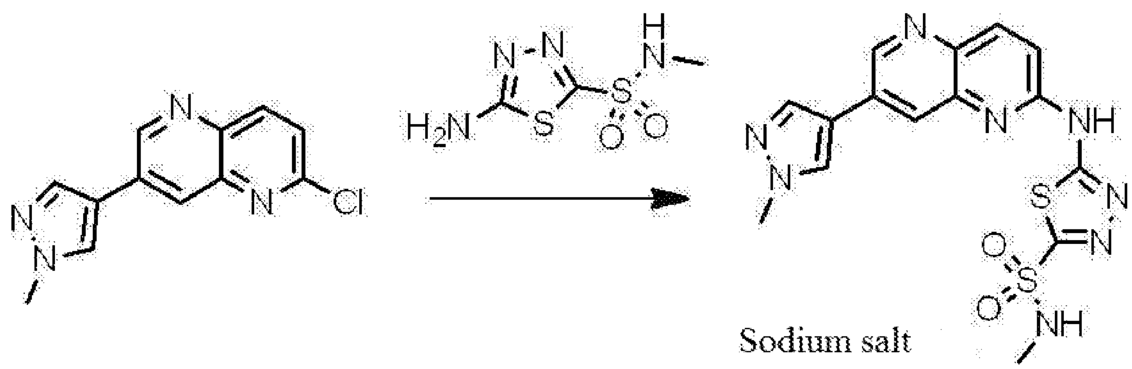


4,5-Bis(diphenylphosphino)-9,9-dimethylxanthene (7 mg), potassium tert-butoxide (30 mg), and tris(dibenzylideneacetone)dipalladium(0) (7 mg) were added to a solution of 2-chloro-7-(1-methyl-1H-pyrazol-4-yl)-1,5-naphthyridine (35 mg) and 5-amino-N-methyl-1,3,4-thiadiazole-2-sulfonamide (35 mg) in 1,4-dioxane (1 mL), followed by stirring at 150°C for 1 hour using a microwave reaction apparatus. The reaction mixture was cooled to room temperature, and chloroform and water were added thereto. Liquid-liquid separation was performed, and the water of the aqueous layer was distilled off under reduced pressure. The obtained residue was purified by preparative reversed phase HPLC (a 0.1% formic acid aqueous solution-a 0.1% formic acid acetonitrile solution), and purified by reversed silica gel chromatography (methanol-sodium hydrogen carbonate aqueous solution), thereby obtaining methyl ((5-((7-(1-methyl-1H-pyrazol-4-yl)-1,5-naphthyridin-2-yl)amino)-1,3,4-thiadiazol-2-yl)sulfonyl)amide sodium salt (3.2 mg) as a yellow solid.
$^1$H-NMR(DMSO-$d_6$)δ:8.83(1H,d,J=1.7Hz),8.46(1H,s),8.12(1H,s),8.06(1H,s),7.92(1H,d,J=8.9Hz),7.81(1H,s),7.15(1H,d,J =8.9Hz),3.91(3H,s),2.62(3H,s).
MSm/z(M+H):403.

<Example 0190>

<0190-1>

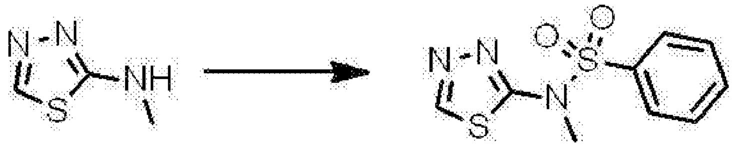

N-methyl-N-(1,3,4-thiadiazol-2-yl)benzene sulfonamide (264 mg) was obtained as oily substance in the same manner as in Example 0177 except that 2-(methylamino)-1,3,4-thiadiazole was used instead of the 2-amino-1,3,4-thiadiazole used in Example 0177.
$^1$H-NMR(DMSO-$d_6$)$\delta$:9.28(1H,s),7.88-7.78(3H,m),7.71-7.64(2H,m),3.44(3H,s).

<0190-2>

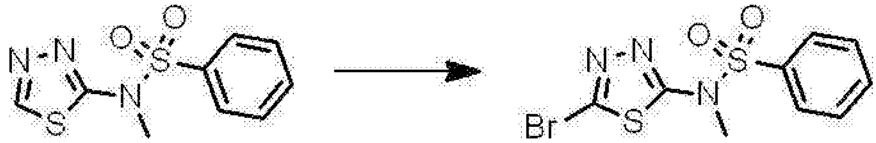

N-(5-bromo-1,3,4-thiadiazol-2-yl)-N-methylbenzene sulfonamide was obtained as oily substance in the same manner as in Example 0177 except that N-methyl-N-(1,3,4-thiadiazol-2-yl)benzene sulfonamide was used instead of the N-(1,3,4-thiadiazol-2-yl)benzene sulfonamide used in Example 0177.
$^1$H-NMR(DMSO-$d_6$)$\delta$:7.92-7.81(3H,m),7.72-7.63(2H,m),3.38(3H,s).

<0190-3>

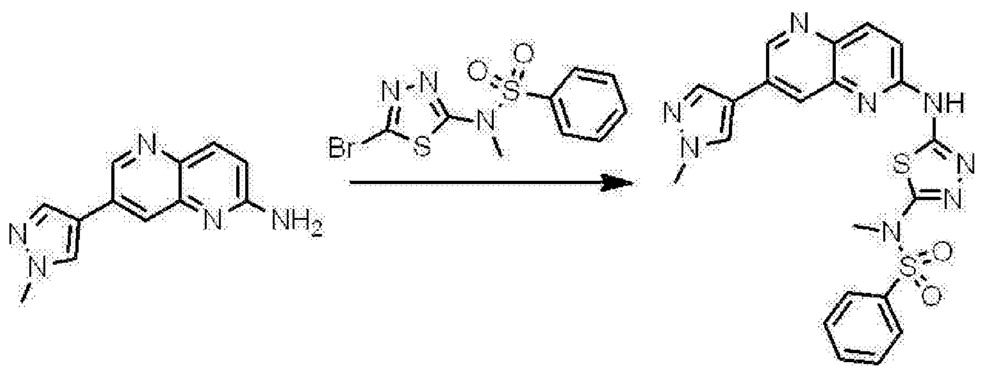

N-methyl-N-(5-((7-(1-methyl-1H-pyrazol-4-yl)-1,5-naphthyridin-2-yl)amino)-1,3,4-thiadiazol-2-yl)benzene sulfonamide (1.9 mg) was obtained as a pale yellow solid in the same manner as in Example 0177 except that N-(5-bromo-1,3,4-thiadiazol-2-yl)-N-methylbenzene sulfonamide was used instead of the N-(5-bromo-1,3,4-thiadiazol-2-yl)benzene sulfonamide used in Example 0177.
$^1$H-NMR(DMSO-$d_6$)$\delta$:12.15(1H,s),9.10(1H,d,J=2.0Hz),8.54(1H,s),8.29(1H,d,J=9.2Hz),8.24(1H,d,J=2.0Hz),8.21(1H,s),7.84-7.76(3H,m),7.70-7.63(2H,m),7.40(1H,d,J=9.2Hz),3.93(3H,s),3.34(3H,s).
MSm/z(M+H):479.

<Example 0191>

<0191-1>

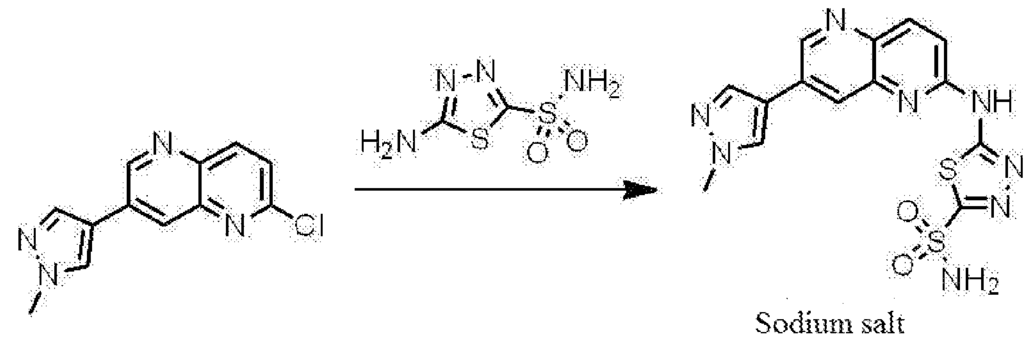


A ((5-((7-(1-methyl-1H-pyrazol-4-yl)-1,5-naphthyridin-2-yl)amino)-1,3,4-thiadiazol-2-yl)sulfonyl)amide sodium salt was obtained as a white solid in the same manner as in Example 0189 except that 2-amino-1,3,4-thiadiazole-5-sulfonamide was used instead of the 5-amino-N-methyl-1,3,4-thiadiazole-2-sulfonamide used in Example 0189.
$^1$H-NMR(DMSO-$d_6$)δ:8.73(1H,d,J=2.0Hz),8.42(1H,s),8.07(1H,s),7.96(1H,d,J=1.7Hz),7.80(1H,d,J=8.9Hz),7.53(2H,s),7.05(1H,d,J=8.9Hz),3.91(3H,s).
MSm/z(M+H):389.

<Example 0192>

<0192-1>

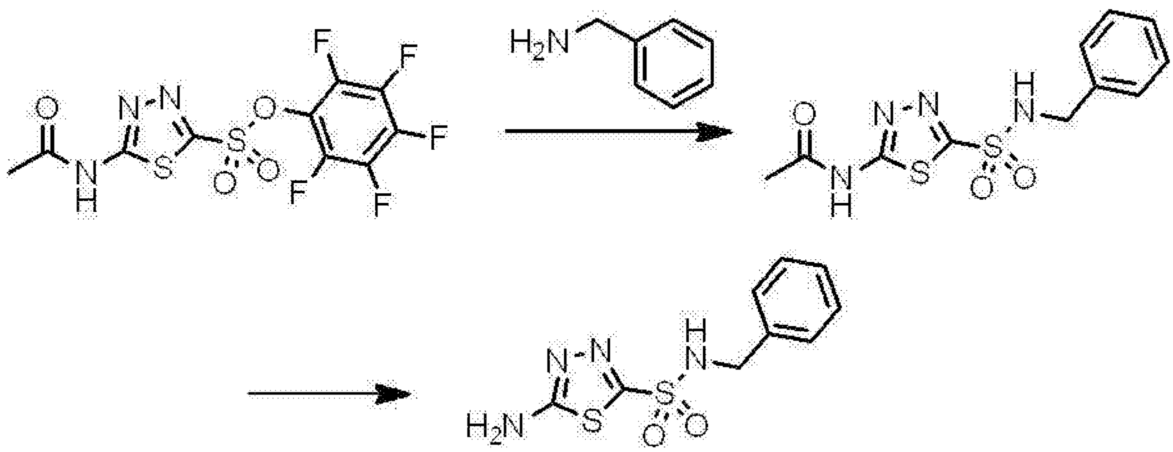

5-Amino-N-benzyl-1,3,4-thiadiazole-2-sulfonamide was obtained in the same manner as in Example 0189 except that benzylamine was used instead of the methylamine hydrochloride used in Example 0189.
MSm/z(M+H):271.

<0192-2>

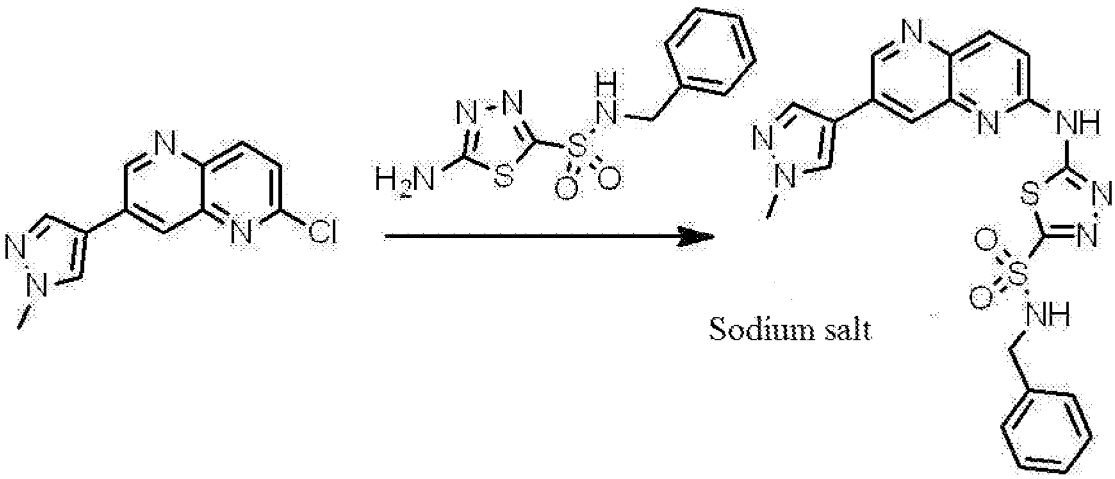


An N-benzyl-5-((7-(1-methyl-1H-pyrazol-4-yl)-1,5-naphthyridin-2-yl)amino)-1,3,4-thiadiazole-2-sulfonamide sodium salt was obtained as a yellow solid in the same manner as in Example 0189 except that 5-amino-N-benzyl-1,3,4-thiadiazole-2-sulfonamide was used instead of the 5-amino-N-methyl-1,3,4-thiadiazole-2-sulfonamide used in Example 0189.
$^1$H-NMR(DMSO-$d_6$)δ:8.75(1H,d,J=2.3Hz),8.43(1H,s),8.08(1H,d,J=4.6Hz),7.99(1H,d,J=1.7Hz),7.82(1H,d,J=8.9Hz),7.36-7.23(5H,m),7.08(1H,d,J=8.9Hz),4.19(2H,s),3.90(3H,s).
MSm/z(M+H):479.

<Example 0193>

<0193-1>

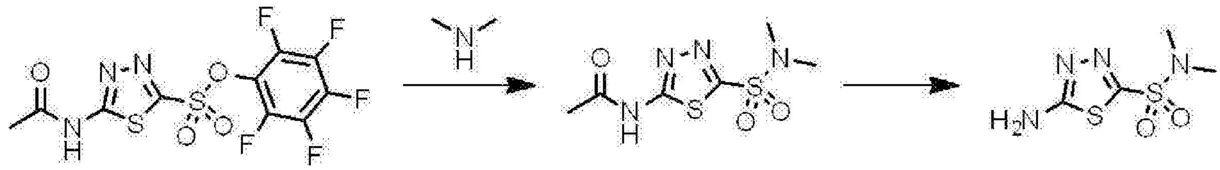

5-Amino-N,N-dimethyl-1,3,4-thiadiazole-2-sulfonamide was obtained in the same manner as in Example 0189 except that dimethylamine was used instead of the methylamine hydrochloride and triethylamine used in Example 0189.
$^1$H-NMR(DMSO-$d_6$)δ:8.03(2H,s),2.83(6H,s).

<0193-2>

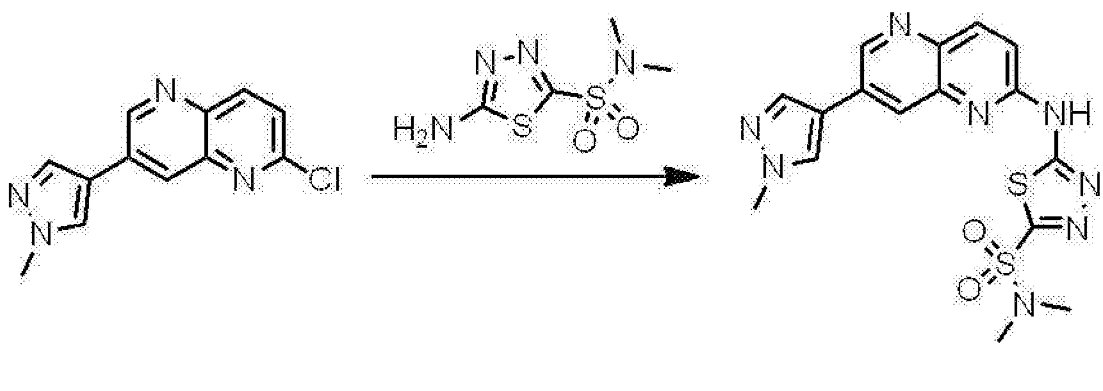

N,N-dimethyl-5-((7-(1-methyl-1H-pyrazol-4-yl)-1,5-naphthyridin-2-yl)amino)-1,3,4-thiadiazole-2-sulfonamide was obtained as a pale yellow solid in the same manner as in Example 0176 except that 5-amino-N,N-dimethyl-1,3,4-thiadiazole-2-sulfonamide was used instead of the 5-amino-N-phenyl-1,3,4-thiadiazole-2-sulfonamide used in Example 0176.
$^1$H-NMR(DMSO-$d_6$)δ:12.88(1H,s),9.14(1H,d,J=2.3Hz),8.58(1H,s),8.42(1H,d,J=1.3Hz),8.36(1H,d,J=8.9Hz),8.26(1H,s), 7.49(1H,d,J=8.9Hz),3.93(3H,s),2.93(6H,s).
MSm/z(M+H):417.

<Example 0194>

<0194-1>

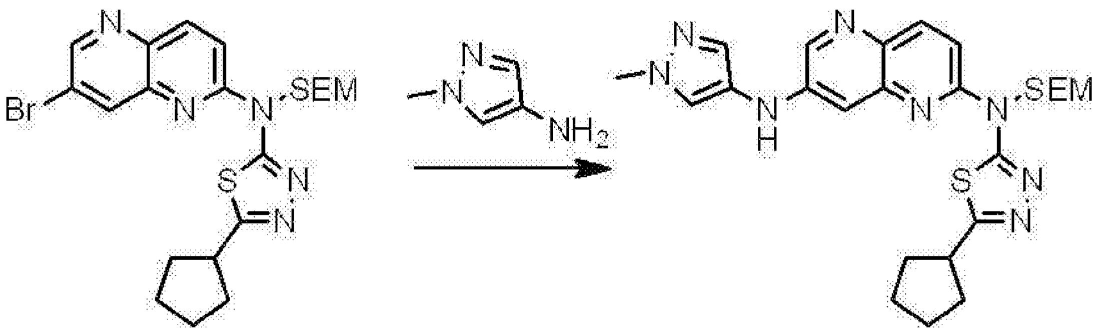

N$^2$-(5-cyclopentyl-1,3,4-thiadiazol-2-yl)-N$^7$-(1-methyl-1H-pyrazol-4-yl)-N$^2$-((2-(trimethylsilyl)ethoxy)methyl)-1,5-naphthyridine-2,7-diamine was obtained in the same manner as in Example 0182 except that N-(7-bromo-1,5-naphthyridin-2-yl)-5-cyclopentyl-N-((2-(trimethylsilyl)ethoxy)methyl)-1,3,4-thiadiazole-2-amine was used instead of the N-(7-bromo-1,5-naphthyridin-2-yl)-5-methyl-N-((2-(trimethylsilyl)ethoxy)methyl)-1,3,4-thiadiazole-2-amine used in Example 0182.
MSm/z(M+H):523.

<0194-2>

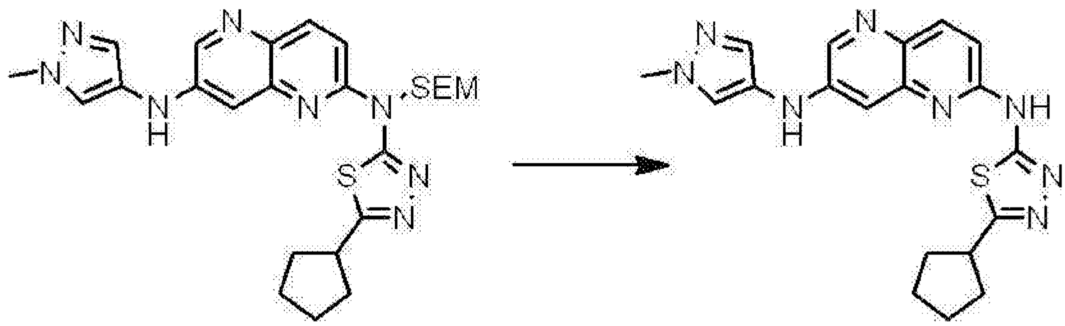

N$^2$-(5-cyclopentyl-1,3,4-thiadiazol-2-yl)-N$^7$-(-(1-methyl-1H-pyrazol-4-yl)-1,5-naphthyridine-2,7-diamine was obtained as a yellow solid in the same manner as in Example 0182 except that N$^2$-(5-cyclopentyl-1,3,4-thiadiazol-2-yl)-N$^7$-(-(1-methyl-1H-pyrazol-4-yl)-N$^2$-((2-(trimethylsilyl)ethoxy)methyl)-1,5-naphthyridine-2,7-diamine was used instead of the tert-butyl 4-((6-((5-methyl-1,3,4-thiadiazol-2-yl)((2-(trimethylsilyl)ethoxy)methyl)amino)-1,5-naphthyridin-3-yl)amino)-1H-pyrazolel-carboxylate used in Example 0182.
$^1$H-NMR(DMSO-$d_6$)δ:12.54(1H,s),11.88(1H,s),8.67(1H,d,J=2.0Hz),8.12(1H,d,J=8.9Hz),7.77(1H,s),7.53(1H,d,J=2.3Hz), 7.20(1H,d,J=8.9Hz),6.19(1H,s),3.44(3H,s),2.20-2.08(2H,m),1.92-1.62(7H,m).
MSm/z(M+H):393.

<Example 0195>

<0195-1>

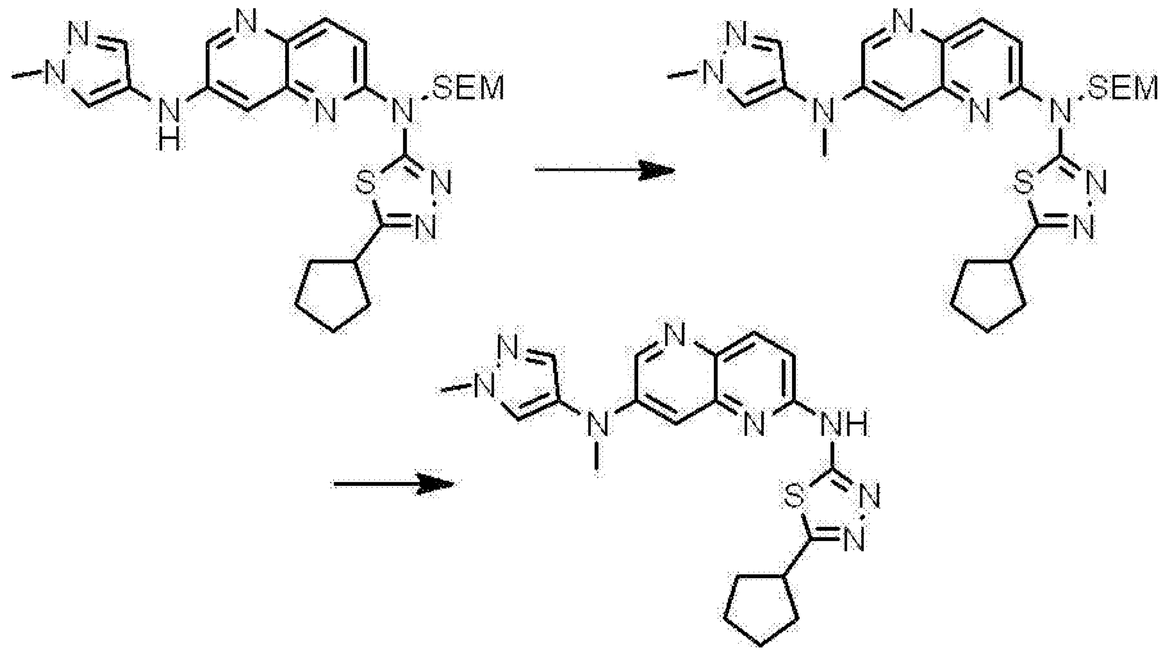

$N^2$-(5-cyclopentyl-1,3,4-thiadiazol-2-yl)-$N^7$-methyl-$N^7$-(1-methyl-1H-pyrazol-4-yl)-1,5-naphthyridine-2,7-diamine was obtained as a yellow solid in the same manner as in Example 0183 except that $N^2$-(5-cyclopentyl-1,3,4-thiadiazol-2-yl)-$N^7$-(-(1-methyl-1H-pyrazol-4-yl)-$N^2$-((2-(trimethylsilyl)ethoxy)methyl)-1,5-naphthyridine-2,7-diamine was used instead of the tert-butyl 4-((6-((5-methyl-1,3,4-thiadiazol-2-yl)((2-(trimethylsilyl)ethoxy)methyl)amino)-1,5-naphthyridin-3-yl)amino)-1H-pyrazole-1-carboxylate used in Example 0183.
$^1$H-NMR(DMSO-$d_6$)$\delta$:11.89(1H,s),8.68(1H,d,J=2.6Hz),8.12(1H,d,J=8.9Hz),7.70(1H,d,J=2.3Hz),7.55(1H,d,J=2.3Hz),7.20(1H,d,J=8.9Hz),6.14(1H,d,J=2.3Hz),3.81(3H,s),3.42(3H,s),2.21-2.06(2H,m),1.92-1.62(7H,m).
MSm/z(M+H):407.

<Example 0196>

<0196-1>

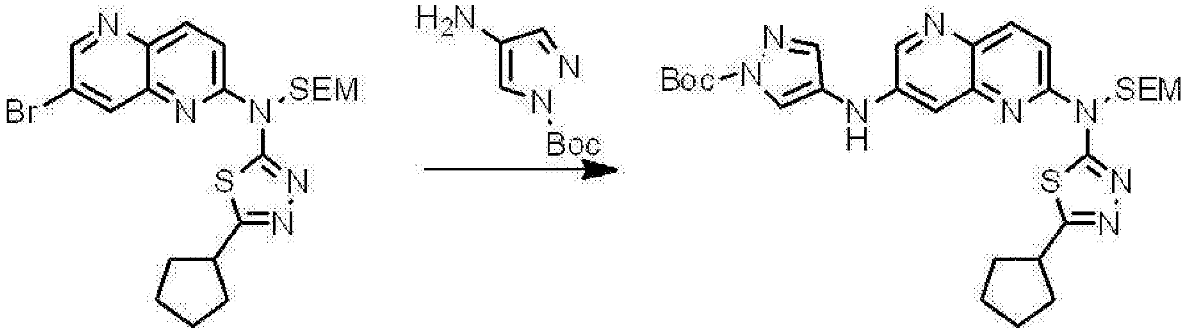

tert-Butyl 4-amino-1H-pyrazole-1-carboxylate (25 mg), 4,5-bis(diphenylphosphino)-9,9-dimethylxanthene (5 mg), cesium carbonate (45 mg), and tris(dibenzylideneacetone)dipalladium(0) (5 mg) were added to a solution of N-(7-bromo-1,5-naphthyridin-2-yl)-5-cyclopentyl-N-((2-(trimethylsilyl)ethoxy)methyl)-1,3,4-thiadiazole-2-amine (40 mg) in 1,4-dioxane (1 mL), followed by stirring at 100°C for 8 hours in a nitrogen atmosphere. 4,5-Bis(diphenylphosphino)-9,9-dimethylxanthene (5 mg) and tris(dibenzylideneacetone)dipalladium(0) (5 mg) were added thereto, followed by stirring at 100°C for 3 hours in a nitrogen atmosphere. The reaction mixture was cooled to room temperature, and ethyl acetate and water were added thereto. The organic layer was collected by separation, and the solvent was distilled off under reduced pressure. The obtained residue was purified by silica gel column chromatography (hexane-ethyl acetate, NH silica), thereby obtaining tert-butyl 4-((6-((5-cyclopentyl-1,3,4-thiadiazol-2-yl)((2-(trimethylsilyl)ethoxy)methyl)amino)-1,5-naphthyridin-3-yl)amino)-1H-pyrazole-1-carboxylate (40 mg).
MSm/z(M+H):609.

<0196-2>

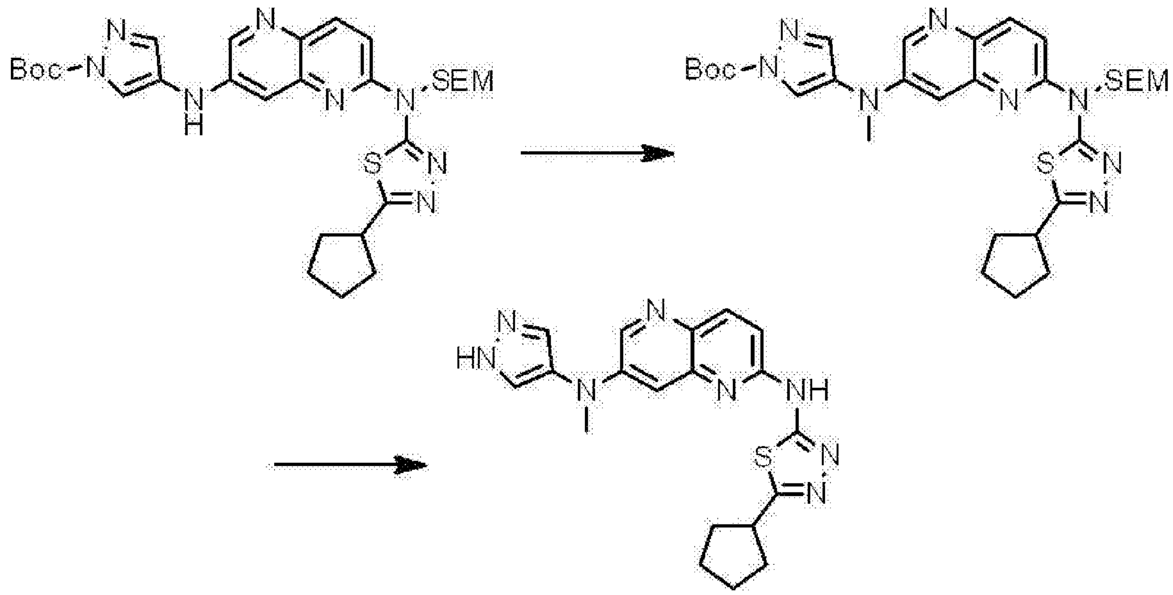

$N^2$-(5-cyclopentyl-1,3,4-thiadiazol-2-yl)-$N^7$-methyl-$N^7$-(1H-pyrazol-4-yl)-1,5-naphthyridine-2,7-diamine (5 mg) was obtained as a yellow solid in the same manner as in Example 0183 except that tert-butyl 4-((6-((5-cyclopentyl-1,3,4-thiadiazol-2-yl)((2-(trimethylsilyl)ethoxy)methyl)amino)-1,5-naphthyridin-3-yl)amino)-1H-pyrazole-1-carboxylate was used instead of the tert-butyl 4-((6-((5-methyl-1,3,4-thiadiazol-2-yl)((2-(trimethylsilyl)ethoxy)methyl)amino)-1,5-naphthyridin-3-yl)amino)-1H-pyrazole-1-carboxylate used in Example 0183.
$^1$H-NMRD(MSO-d$_6$)δ:12.04(1H,s),9.08(1H,d,J=2.0Hz),8.56(1H,s),8.32(1H,d,J=1.7Hz),8.24(2H,d,J=10.9Hz),7.39(1H,d,J=8.9Hz),2.14(3H,s),2.04-1.68(9H,m).
MSm/z(M+H):393.

<Example 0197>

<0197-1>

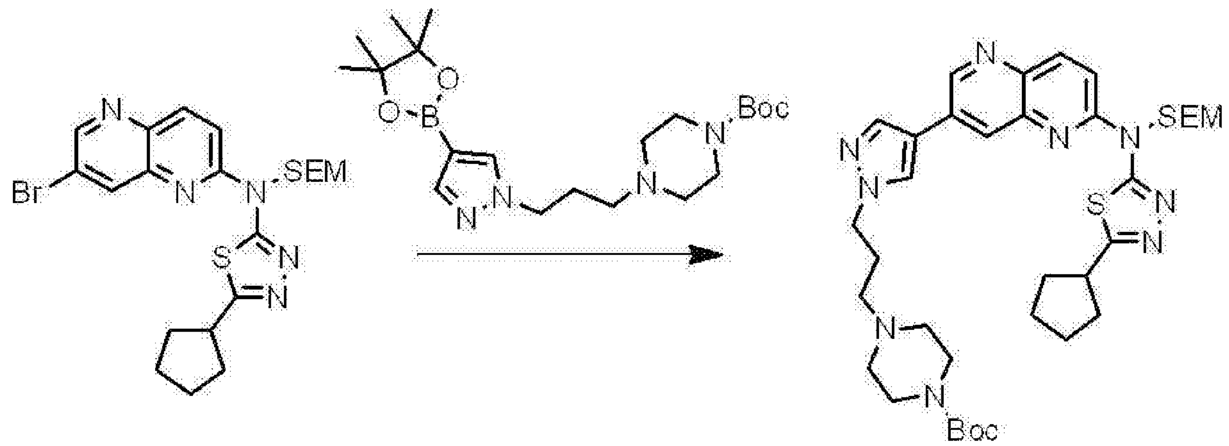

tert-Butyl 4-(3-(4-(4,4,5,5-tetramethyl-1,3,2-dioxaborolan-2-yl)-1H-pyrazol-1-yl)propyl)piperazine-1-carboxylate (30 mg), bis(di-tert-butyl (4-dimethylaminophenyl)phosphine)dichloropalladium(II) (5 mg), sodium carbonate (12 mg), and water (100 μL) were added to a solution of N-(7-bromo-1,5-naphthyridin-2-yl)-5-cyclopentyl-N-((2-(trimethylsilyl)ethoxy)methyl)-1,3,4-thiadiazole-2-amine (50 mg) in 1,4-dioxane (1 mL), followed by stirring at 80°C for 2 hours in a nitrogen atmosphere. The reaction mixture was cooled to room temperature, and water and ethyl acetate were added thereto. The organic layer was collected by separation, and dried over anhydrous sodium sulfate, and the solvent was distilled off under reduced pressure. The solid matter was collected by filtration, and hydrochloric acid (1 mL) was added thereto, followed by stirring at 90°C for 1 hour. The reaction mixture was cooled to room temperature, and the resultant product was neutralized with a sodium hydroxide aqueous solution. The solid matter was collected by filtration, and purified by silica gel column chromatography (chloroform-methanol, NH silica), thereby obtaining tert-butyl 4-(3-(4-(6-((5-cyclopentyl-1,3,4-thiadiazol-2-yl)((2-(trimethylsilyl)ethoxy)methyl)amino)-1,5-naphthyridin-3-yl)-1H-pyrazol-1-yl)propyl)piperazine-1-carboxylate (27 mg) as a yellow solid.
MSm/z(M+H):720.

<0197-2>

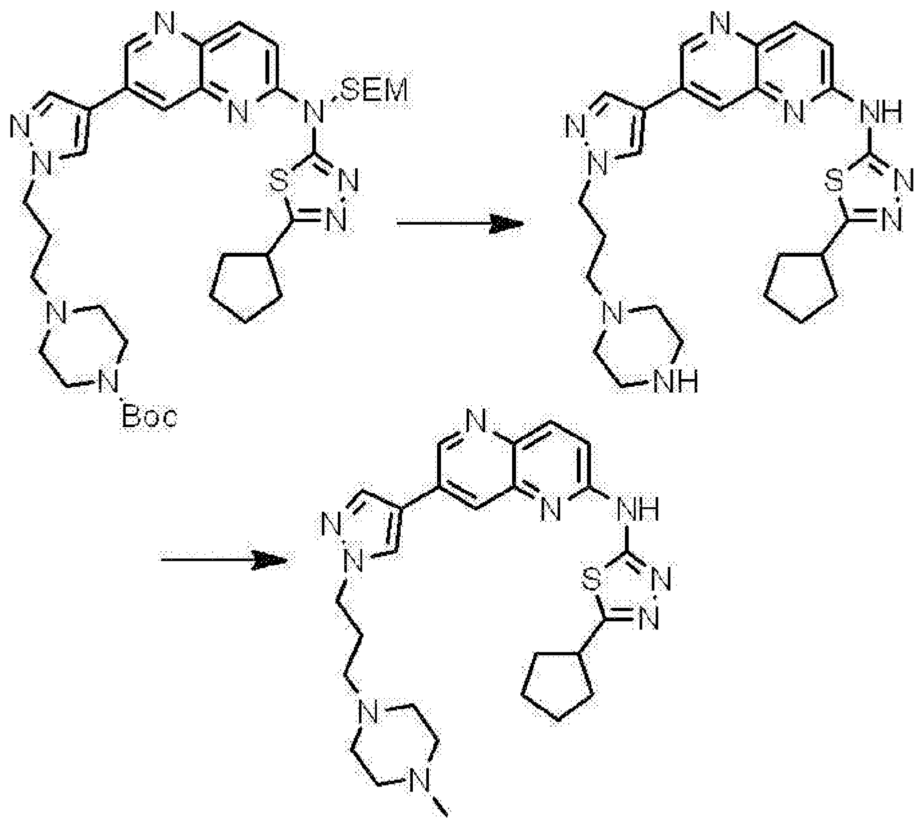

Methanol (1 mL) and hydrochloric acid (1 mL) were added to tert-butyl 4-(3-(4-(6-((5-cyclopentyl-1,3,4-thiadiazol-2-yl)((2-(trimethylsilyl)ethoxy)methyl)amino)-1,5-naphthyridin-3-yl)-1H-pyrazol-1-yl)propyl)piperazine-1-carboxylate (27 mg), followed by stirring at 60°C for 1 hour. The solvent was distilled off under reduced pressure, the resultant product was neutralized with a saturated sodium hydrogen carbonate aqueous solution, and a solution of methanol-chloroform was added thereto.

The organic layer was collected by separation, and dried over anhydrous sodium sulfate, and the solvent was distilled off under reduced pressure, thereby obtaining 5-cyclopentyl-N-(7-(1-(3-(piperazin-1-yl)propyl)-1H-pyrazol-4-yl)-1,5-naphthyridin-2-yl)-1,3,4-thiadiazole-2-amine.

A20% formaldehyde solution (5 $\mu$L) and sodium triacetoxyborohydride (12 mg) were added to a solution of the obtained 5-cyclopentyl-N-(7-(1-(3-(piperazin-1-yl)propyl)-1H-pyrazol-4-yl)-1,5-naphthyridin-2-yl)-1,3,4-thiadiazole-2-amine in methanol (600 $\mu$L) and chloroform (600 $\mu$L), followed by stirring at room temperature for 2 hours. A saturated sodium chloride aqueous solution and chloroform were added to the reaction mixture. The organic layer was collected by separation, and dried over anhydrous sodium sulfate, and the solvent was distilled off under reduced pressure. The obtained residue was purified by silica gel column chromatography (chloroform-methanol, NH silica), thereby obtaining 5-cyclopentyl-N-(7-(1-(3-(4-methylpiperazin-1-yl)propyl)-1H-pyrazol-4-yl)-1,5-naphthyridin-2-yl)-1,3,4-thiadiazole-2-amine (4 mg) as a yellow solid.
$^1$H-NMR(DMSO-$d_6$)$\delta$:12.04(1H,s),9.07(1H,d,J=2.0Hz),8.56(1H,s),8.32(1H,d,J=1.3Hz),8.26-8.22(2H,m),7.39(1H,d,J=8.9Hz),4.19(2H,t,J=7.1Hz),3.51-3.44(1H,m),2.74-2.71(1H,m),2.42-2.24(11H,m),2.14(3H,s),2.02-1.72(8H,m).
MSm/z(M+H):504.

<Example 0198>

<0198-1>

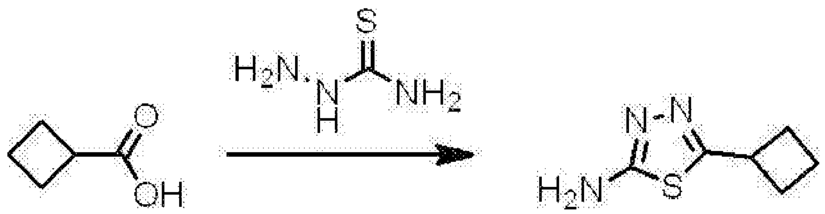

Cyclobutanecarboxylic acid (600 μL) was added to a solution of thiosemicarbazide (500 mg) in hydrochloric acid (2 mL), followed by stirring for 60 hours under heating to reflux. The reaction mixture was cooled to room temperature, and neutralized by the addition of a sodium hydroxide aqueous solution under ice-cooling. The solid matter was collected by filtration, thereby obtaining 2-amino-5-cyclobutyl-1,3,4-thiadiazole (650 mg).
MSm/z(M+H):156.

<0198-2>

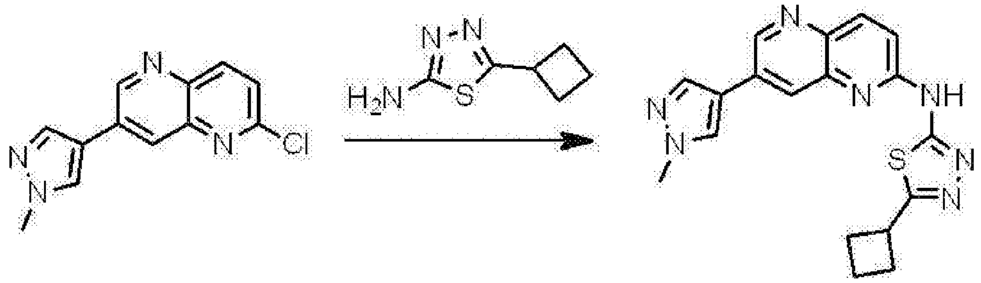

5-Cyclobutyl-N-(7-(1-methyl-1H-pyrazol-4-yl)-1,5-naphthyridin-2-yl)-1,3,4-thiadiazole-2-amine (16 mg) was obtained as a pale yellow solid in the same manner as in Example 0162 except that 2-amino-5-cyclobutyl-1,3,4-thiadiazole was used instead of the 2-amino-5-tert-butyl-1,3,4-thiadiazole used in Example 0162.
$^1$H-NMRD(MSO-$d_6$)δ:12.06(1H,s),9.07(1H,d,J=2.0Hz),8.53(1H,s),8.34(1H,d,J=1.7Hz),8.25(1H,d,J=9.2Hz),8.22(1H,s), 7.39(1H,d,J=8.9Hz),3.93(3H,s),2.45-2.27(5H,m),2.17-1.92(2H,m).
MSm/z(M+H):364.

<Example 0199>

<0199-1>

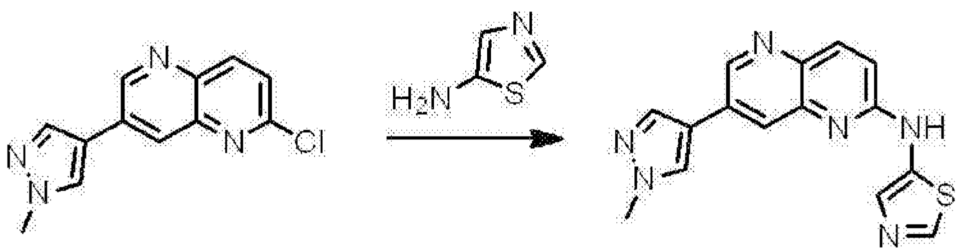

5-Aminothiazole (15 mg), 4,5-bis(diphenylphosphino)-9,9-dimethylxanthene (5 mg), cesium carbonate (45 mg), and tris(dibenzylideneacetone)dipalladium(0) (5 mg) were added to a solution of 2-chloro-7-(1-methyl-1H-pyrazol-4-yl)-1,5-naphthyridine (25 mg) in 1,4-dioxane (2 mL), followed by stirring at 100°C in a nitrogen atmosphere overnight. The reaction mixture was cooled to room temperature, and a mixed solvent of chloroform-methanol was added thereto. The obtained solution was purified by silica gel column chromatography (chloroform-methanol, NH silica), thereby obtaining N-(7-(1-methyl-1H-pyrazol-4-yl)-1,5-naphthyridin-2-yl)thiazole-5-amine (2 mg) as a brown solid.
$^1$H-NMR(DMSO-

$d_6$)δ:10.99(1H,s),8.97(1H,d,J=2.3Hz),8.56(1H,s),8.49(1H,s),8.21-8.11(3H,m),7.74(1H,d,J=0.7Hz),7.21(1H,d,J=8.9Hz), 3.91(3H,d,J=6.3Hz).
MSm/z(M+H):309.

<Example 0200>

<0200-1>

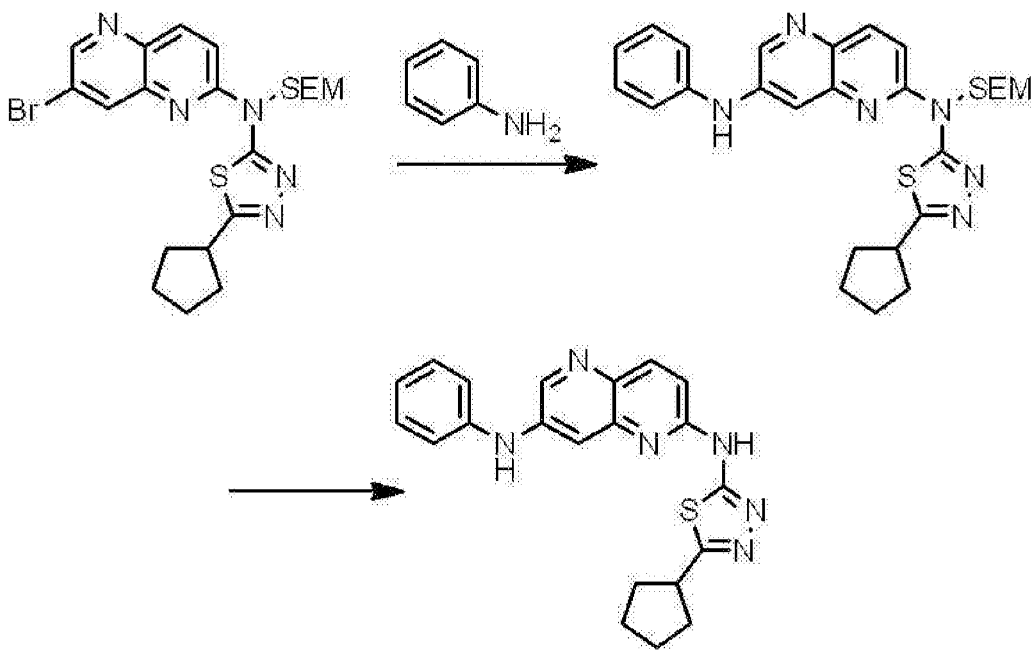

$N^2$-(5-cyclopentyl-1,3,4-thiadiazol-2-yl)-$N^7$-phenyl-1,5-naphthyridine-2,7-diamine was obtained as a yellow solid in the same manner as in Example 0175 except that aniline was used instead of the tert-butyl 4-amino-1H-pyrazole-1-carboxylate used in Example 0175.
$^1$H-NMRD(MSO-$d_6$)δ:11.88(1H,s),8.90(1H,s),8.56(1H,d,J=2.6Hz),8.11(1H,d,J=8.9Hz),7.59(1H,d,J=2.3Hz),7.39(2H,t,J=7.9Hz),7.28(2H,dd,J=8.4,1.2Hz),7.18(1H,d,J=9.2Hz),7.03(1H,t,J=7.3Hz),3.52-3.41(1H,m),2.17-2.05(2H,m),1.87-1.60(6H,m).
MSm/z(M+H):389.

<Example 0201>

<0201-1>

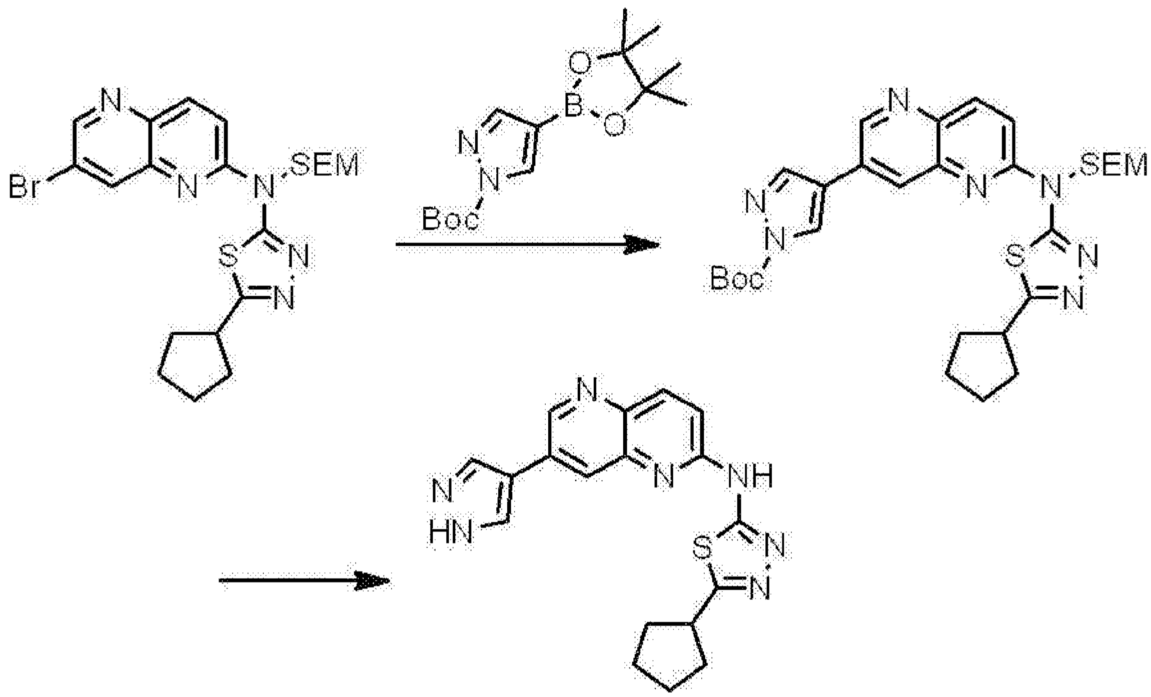

N-(7-(1H-pyrazol-4-yl)-1,5-naphthyridin-2-yl)-5-cyclopentyl-1,3,4-thiadiazole-2-amine was obtained as a yellow solid in the same manner as in Example 0197 except that tert-butyl=4-(4,4,5,5-tetramethyl-1,3,2-dioxaborolan-2-yl)-1H-pyrazole-1-carboxylate was used instead of the tert-butyl 4-(3-(4-(4,4,5,5-tetramethyl-1,3,2-dioxaborolan-2-yl)-1H-pyrazol-1-yl)propyl)piperazine-1-carboxylate used in Example 0197.
$^1$H-NMRD(DMSO-$d_6$)$\delta$:13.21(1H,s),12.03(1H,s),9.12(1H,d,J=2.0Hz),8.59(1H,s),8.36(1H,d,J=1.7Hz),8.29-8.22(2H,m),7.39(1H,d,J=9.2Hz),3.55-3.43(1H,m),2.29-2.12(2H,m),1.92-1.68(6H,m).
MSm/z(M+H):364.

<Example 0202>

<0202-1>

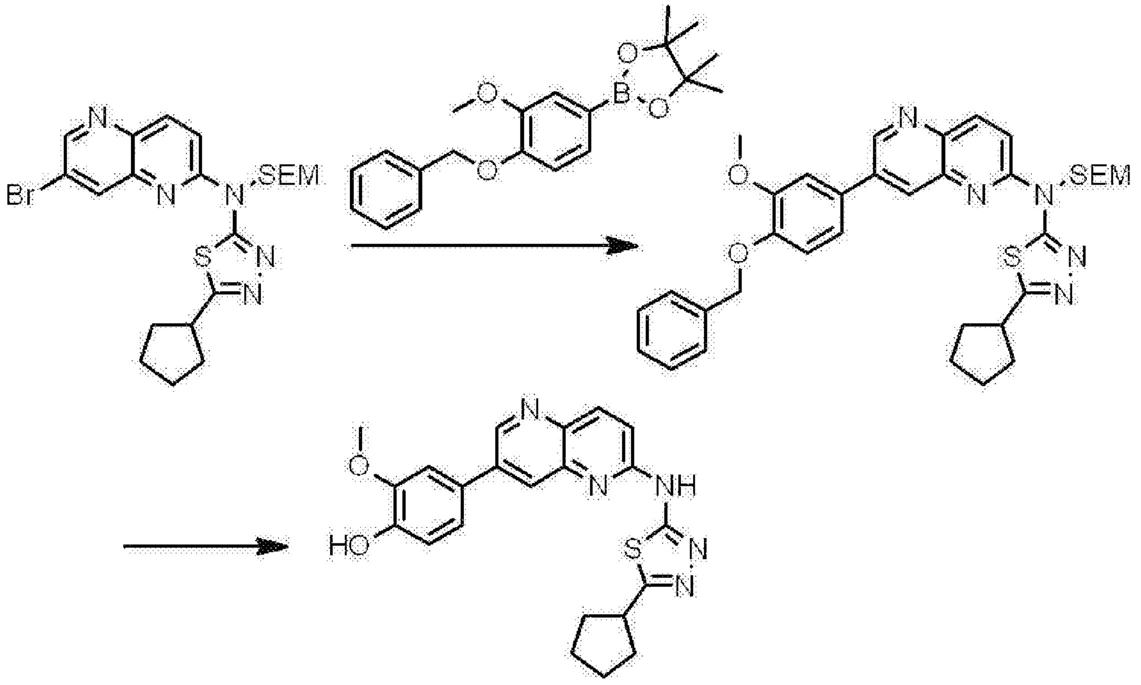

4-(6-((5-Cyclopentyl-1,3,4-thiadiazol-2-yl)amino)-1,5-naphthyridin-3-yl)-2-methoxyphenol was obtained as a yellow solid in the same manner as in Example 0197 except that 2-(4-benzyloxy-3-methoxyphenyl)-4,4,5,5-tetramethyl-1,3,2-dioxaborolane was used instead of the tert-butyl 4-(3-(4-(4,4,5,5-tetramethyl-1,3,2-dioxaborolan-2-yl)-1H-pyrazol-1-yl)propyl)piperazine-1-carboxylate used in Example 0197.

$^1$H-NMR(DMSO-$d_6$)$\delta$:12.06(1H,s),9.36(1H,s),9.09(1H,d,J=2.3Hz),8.34-8.27(2H,m),7.47-7.33(3H,m),6.95(1H,d,J=8.3Hz),3.93(3H,s),3.53-3.43(1H,m),2.20-2.09(2H,m),1.92-1.63(6H,m).

MSm/z(M+H):420.

<Example 0203>

<0203-1>

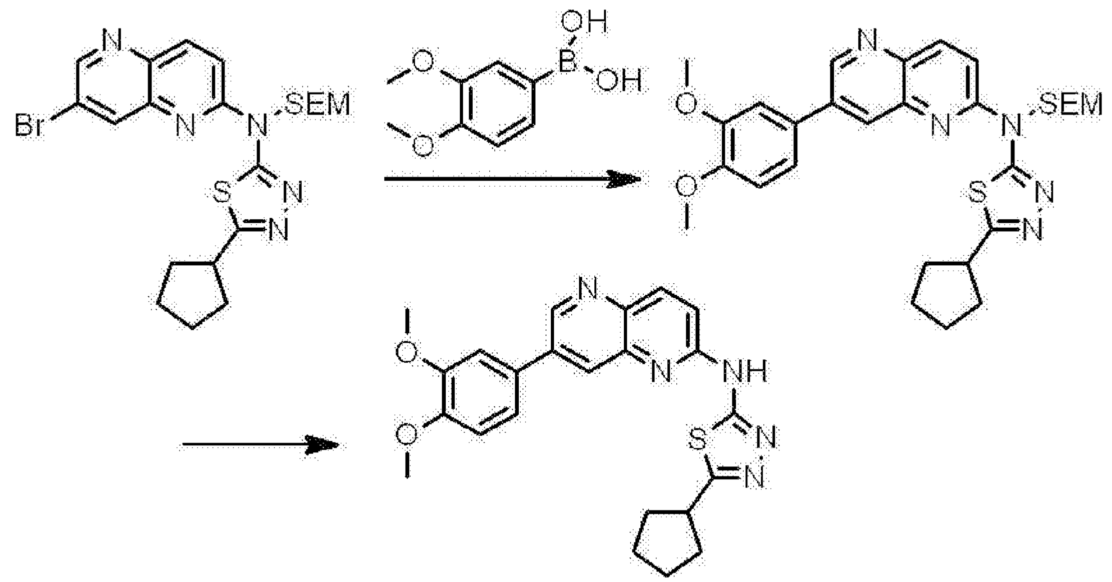

5-Cyclopentyl-N-(7-(3,4-dimethoxyphenyl)-1,5-naphthyridin-2-yl)-1,3,4-thiadiazole-2-amine was obtained as a pale yellow solid in the same manner as in Example 0197 except that 3,4-dimethoxyphenylboronic acid was used instead of the tert-butyl 4-(3-(4-(4,4,5,5-tetramethyl-1,3,2-dioxaborolan-2-yl)-1H-pyrazol-1-yl)propyl)piperazine-1-carboxylate used in Example 0197.
$^1$H-NMR(DMSO-$d_6$)δ:12.07(1H,s),9.12(1H,d,J=2.0Hz),8.39(1H,d,J=1.7Hz),8.31(1H,d,J=8.9Hz),7.51-7.43(3H,m),7.13(1H,d,J=8.6Hz),3.92(3H,s),3.84(3H,s),3.54-3.44(1H,m),2.22-2.10(2H,m),1.91-1.67(6H,m).
MSm/z(M+H):434.

<Example 0204>

<0204-1>

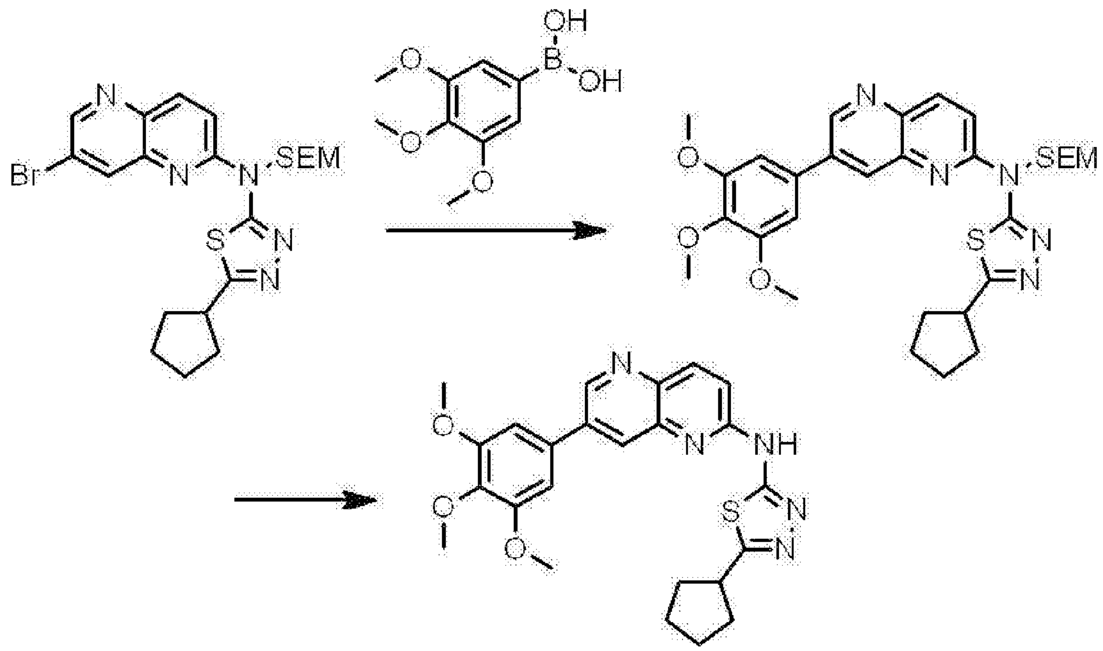

5-Cyclopentyl-N-(7-(3,4,5-trimethoxyphenyl)-1,5-naphthyridin-2-yl)-1,3,4-thiadiazole-2-amine was obtained as a yellow solid in the same manner as in Example 0197 except that 3,4,5-trimethoxyphenylboronic acid was used instead of the tert-butyl 4-(3-(4-(4,4,5,5-tetramethyl-1,3,2-dioxaborolan-2-yl)-1H-pyrazol-1-yl)propyl)piperazine-1-carboxylate used in Example 0197.
$^1$H-NMR(DMSO-$d_6$)δ:12.07(1H,s),9.12(1H,d,J=2.0Hz),8.39(1H,d,J=1.7Hz),8.31(1H,d,J=8.9Hz),7.51-7.43(3H,m),7.13(1H,d,J=8.6Hz),3.92(3H,s),3.84(3H,s),3.54-3.44(1H,m),2.22-2.10(2H,m),1.91-1.67(6H,m).
MSm/z(M+H):464.

<Example 0205>

<0205-1>

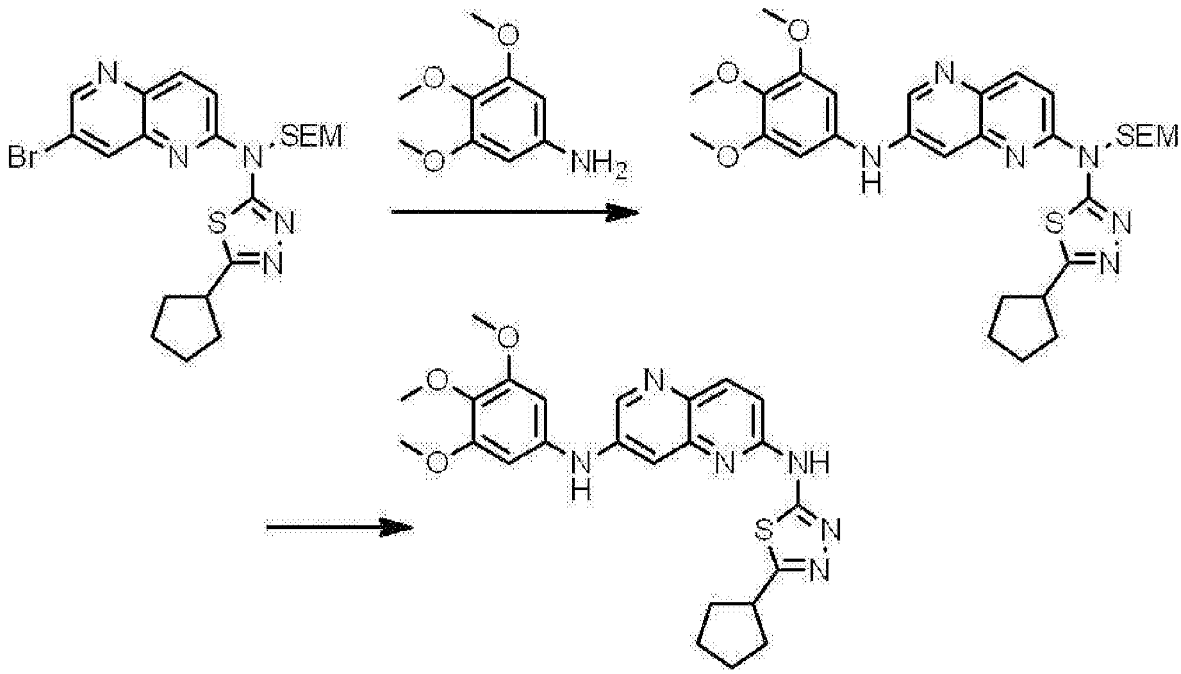

$N^2$-(5-cyclopentyl-1,3,4-thiadiazol-2-yl)-$N^7$-(3,4,5-trimethoxyphenyl)-1,5-naphthyridine-2,7-diamine was obtained as a yellow solid in the same manner as in Example 0175 except that 3,4,5-trimethoxyaniline was used instead of the tert-butyl=4-amino-1H-pyrazole-1-carboxylate used in Example 0175.
$^1$H-NMR(DMSO-$d_6$)$\delta$:11.88(1H,s),8.87(1H,s),8.53(1H,d,J=2.6Hz),8.10(1H,d,J=8.9Hz),7.68(1H,d,J=2.3Hz),7.15(1H,d,J =8.9Hz),6.60(2H,s),3.80(6H,s),3.66(3H,s),3.51-3.40(1H,m),2.16-2.08(2H,m),1.81-1.68(6H,m).
MSm/z(M+H):479.

<Example 0206>

<0206-1>

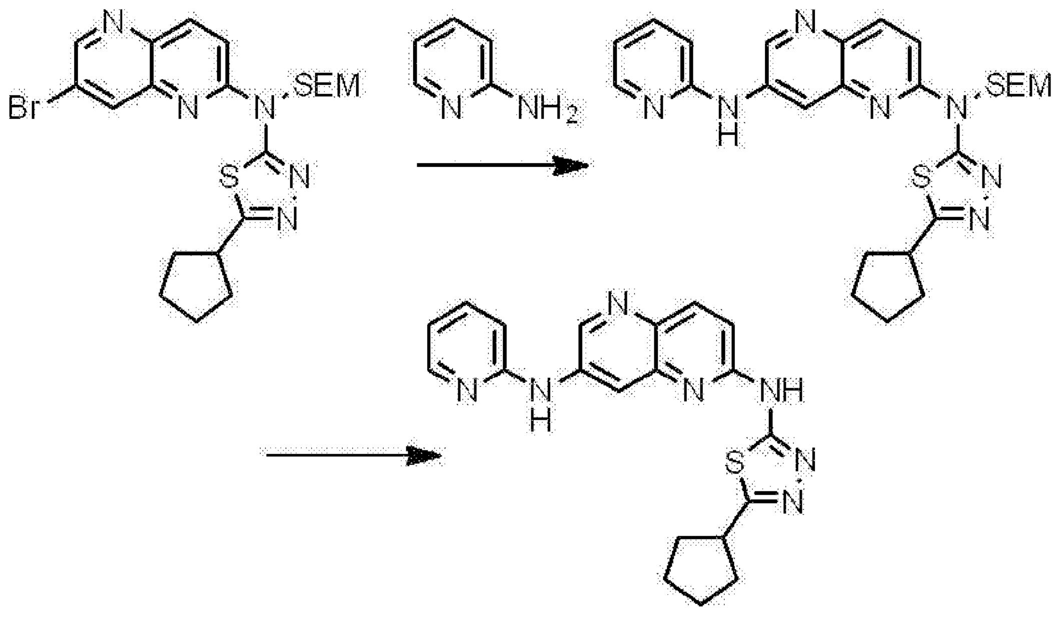

$N^2$-(5-cyclopentyl-1,3,4-thiadiazol-2-yl)-$N^7$-(pyridin-2-yl)-1,5-naphthyridine-2,7-diamine was obtained as a yellow solid in the same manner as in Example 0175 except that 2-aminopyridine was used instead of the tert-butyl=4-amino-1H-pyrazole-1-carboxylate used in Example 0175.
$^1$H-NMR(DMSO-$d_6$)$\delta$:11.92(1H,s),9.72(1H,s),8.91(1H,d),8.76(1H,d),8.33(1H,dd),8.15(1H,d),7.73-7.67(1H,m),7.24(1H,d),6.98(1H,d),6.91(1H,dd),3.56-3.46(1H,m),2.21-2.09(2H,m),1.91-1.68(6H,m).
MSm/z(M+H):390.

<Example 0207>

<0207-1>

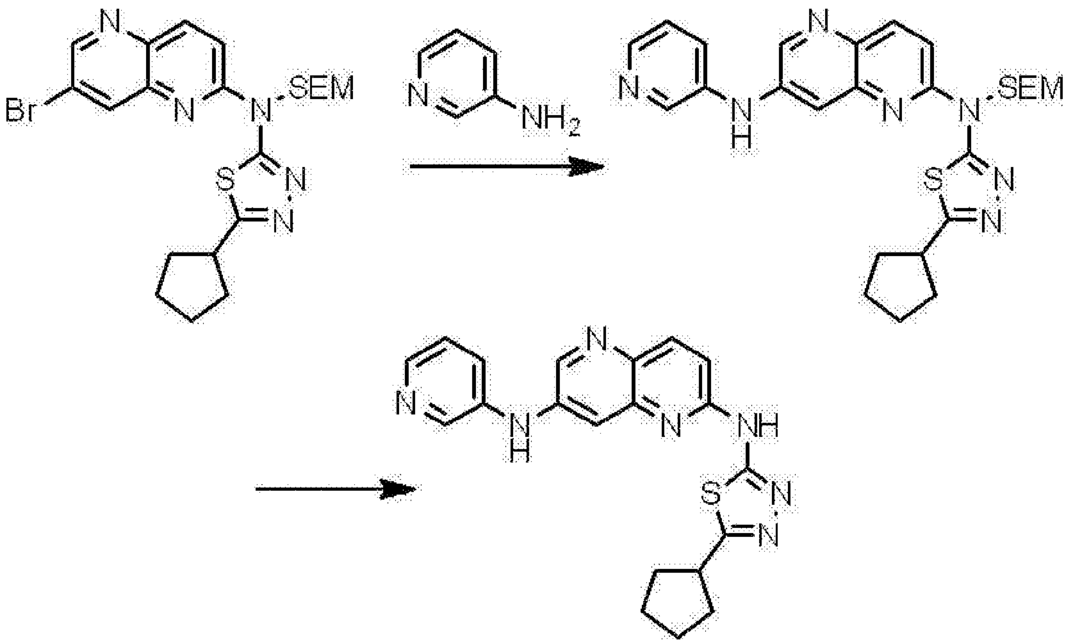

$N^2$-(5-cyclopentyl-1,3,4-thiadiazol-2-yl)-$N^7$-(pyridin-3-yl)-1,5-naphthyridine-2,7-diamine was obtained as a yellow solid in the same manner as in Example 0175 except that 3-aminopyridine was used instead of the tert-butyl=4-amino-1H-pyrazole-1-carboxylate used in Example 0175.
$^1$H-NMR(DMSO-$d_6$)$\delta$:11.92(1H,s),9.06(1H,s),8.59(1H,d,J=2.6Hz),8.54(1H,d,J=2.3Hz),8.23(1H,dd,J=4.6,1.3Hz),8.14(1H,d,J=8.9Hz),7.72(1H,dq,J=8.3,1.3Hz),7.62(1H,d,J=2.3Hz),7.40(1H,dd,J=8.3,4.6Hz),7.22(1H,d,J=8.9Hz),3.52-3.41(1H,m),2.17-2.09(2H,m),1.90-1.61(6H,m).
MSm/z(M+H):390.

<Example 0208>

<0208-1>

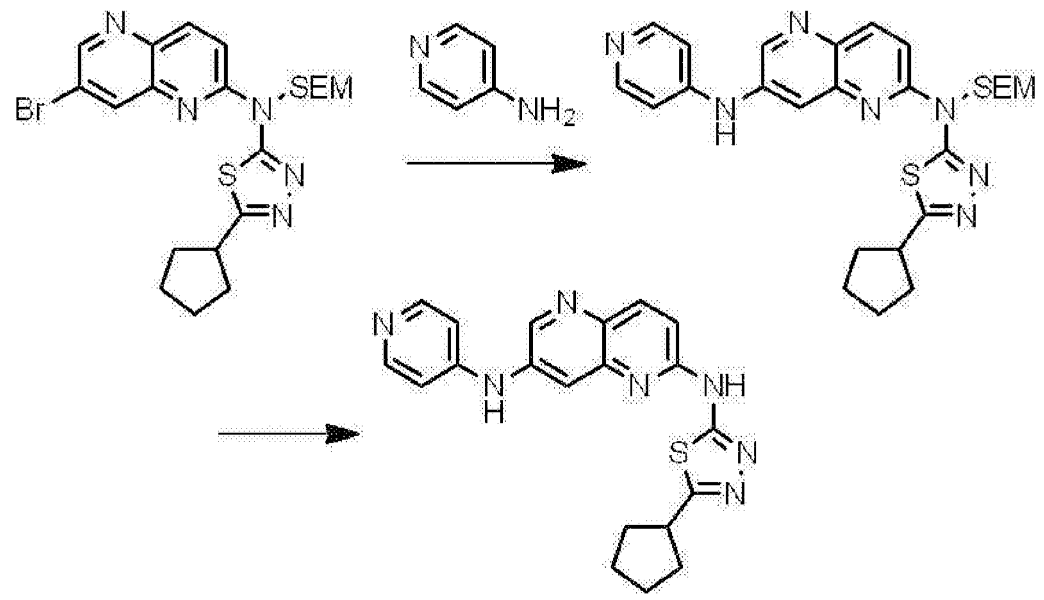

$N^2$-(5-cyclopentyl-1,3,4-thiadiazol-2-yl)-$N^7$-(pyridin-4-yl)-1,5-naphthyridine-2,7-diamine was obtained as a yellow solid in the same manner as in Example 0175 except that 4-aminopyridine was used instead of the tert-butyl=4-amino-1H-pyrazole-1-carboxylate used in Example 0175.
$^1$H-NMR(DMSO-$d_6$)6:12.00(1H,s),9.38(1H,s),8.67(1H,d,J=2.6Hz),8.35(2H,dd,J=5.0,1.3Hz),8.20(1H,d,J=8.9Hz),7.85(1H,d,J=2.3Hz),7.31(1H,d,J=8.9Hz),7.14-7.11(2H,m),3.57-3.39(1H,m),2.20-2.05(2H,m),1.91-1.62(6H,m).
MSm/z(M+H):390.

<Example 0209>

<0209-1>

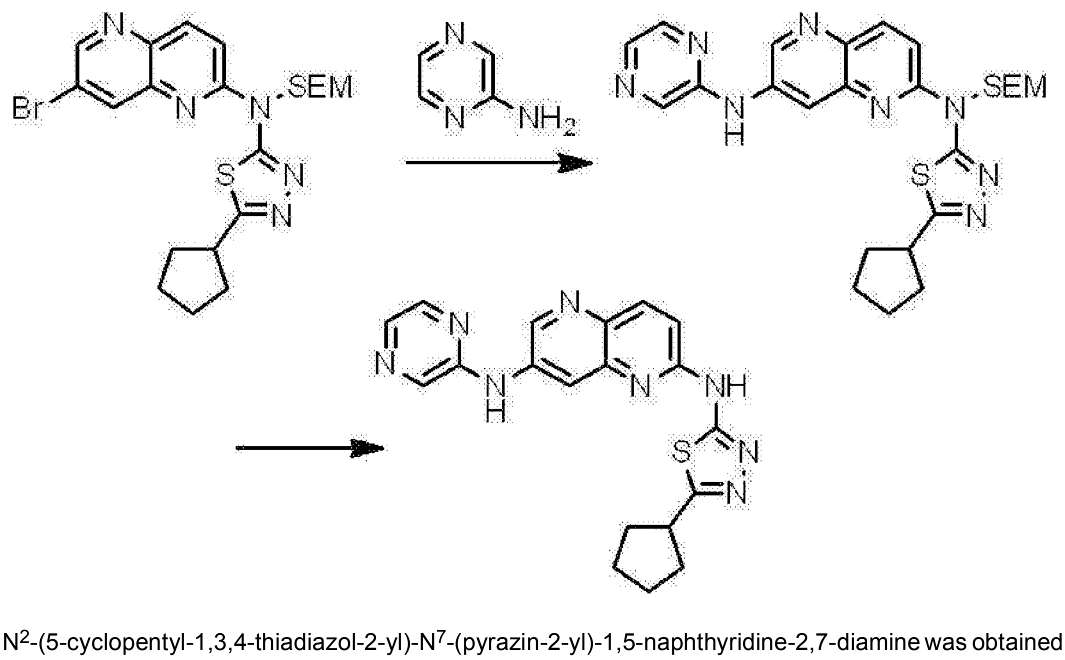

$N^2$-(5-cyclopentyl-1,3,4-thiadiazol-2-yl)-$N^7$-(pyrazin-2-yl)-1,5-naphthyridine-2,7-diamine was obtained as a yellow solid in the same manner as in Example 0175 except that 2-aminopyrazine was used instead of the tert-butyl=4-amino-1H-pyrazole-1-carboxylate used in Example 0175.
$^1$H-NMR(DMSO-$d_6$)$\delta$:11.98(1H,s),10.16(1H,s),8.91(1H,d,J=2.6Hz),8.71(1H,d,J=2.0Hz),8.38(1H,d,J=1.3Hz),8.32(1H,dd,J=2.8,1.5Hz),8.17(1H,d,J=8.9Hz),8.09(1H,d,J=2.6Hz),7.28(1H,d,J=8.9Hz),3.56-3.45(1H,m),2.21-2.09(2H,m),1.91-1.

68(6H,m).
MSm/z(M+H):391.

<Example 0210>

<0210-1>

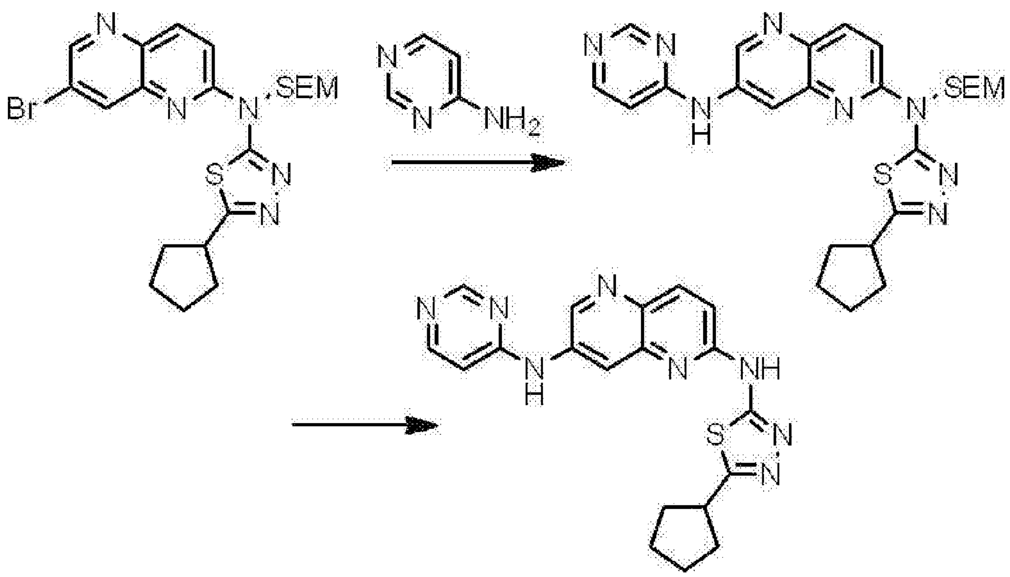

$N^2$-(5-cyclopentyl-1,3,4-thiadiazol-2-yl)-$N^7$-(pyrimidin-4-yl)-1,5-naphthyridine-2,7-diamine was obtained as a yellow solid in the same manner as in Example 0175 except that 4-aminopyrimidine was used instead of the tert-butyl=4-amino-1H-pyrazole-1-carboxylate used in Example 0175.
$^1$H-NMR(DMSO-$d_6$)$\delta$:11.94(1H,s),10.20(1H,s),8.97(1H,d,J=2.6Hz),8.82-8.70(2H,m),8.42(1H,d,J=5.6Hz),8.21(1H,d,J=8.9Hz),7.33(1H,d,J=8.9Hz),6.96(1H,dd,J=5.9,1.0Hz),3.5 5-3.43(1H,m),2.28-2.09(2H,m),1.90-1.68(6H,m).
MSm/z(M+H):391.

<Example 0211>

<0211-1>

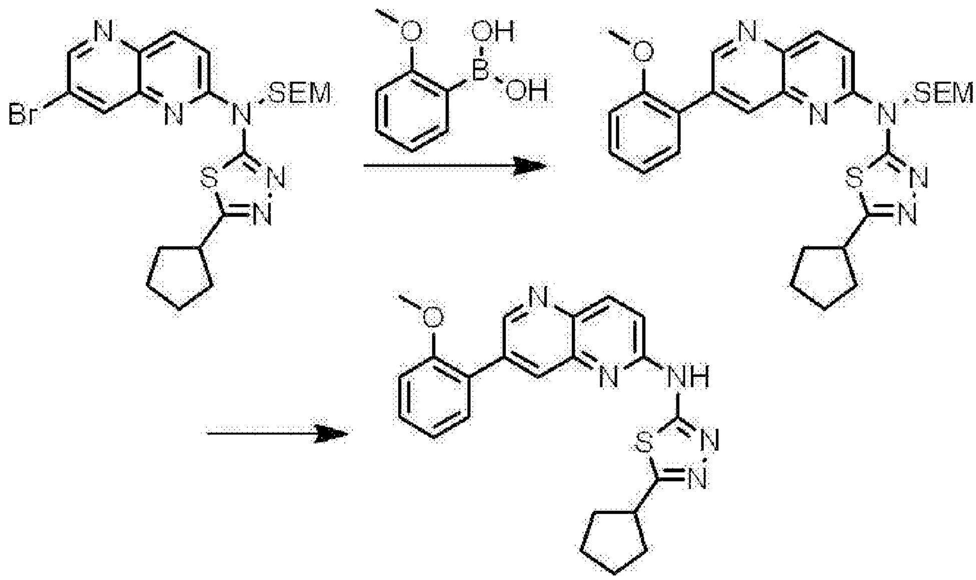

5-Cyclopentyl-N-(7-(2-methoxyphenyl)-1,5-naphthyridin-2-yl)-1,3,4-thiadiazole-2-amine was obtained as a yellow solid in the same manner as in Example 0197 except that 2-methoxyphenylboronic acid was used instead of the tert-butyl 4-(3-(4-(4,4,5,5-tetramethyl-1,3,2-dioxaborolan-2-yl)-1H-pyrazol-1-yl)propyl)piperazine-1-carboxylate used in Example 0197.
$^1$H-NMR(DMSO-$d_6$)δ:12.07(1H,s),8.88(1H,d,J=2.3Hz),8.32(1H,d,J=9.2Hz),8.22(1H,d,J=1.7Hz),7.55-7.45(3H,m),7.22(1H,d,J=7.6Hz),7.13(1H,t,J=7.4Hz),3.83(3H,s),3.52-3.42(1H,m),2.17-2.09(2H,m), 1.90-1.62(6H,m).
MSm/z(M+H):404.

<Example 0212>

<0212-1>

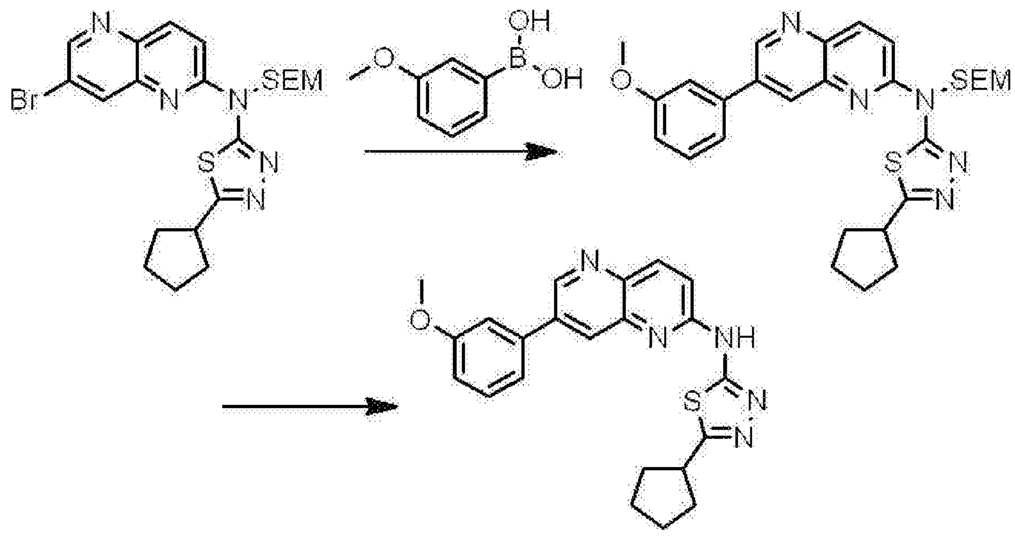

5-Cyclopentyl-N-(7-(3-methoxyphenyl)-1,5-naphthyridin-2-yl)-1,3,4-thiadiazole-2-amine was obtained as a yellow solid in the same manner as in Example 0197 except that 3-methoxyphenylboronic acid was used instead of the tert-butyl 4-(3-(4-(4,4,5,5-tetramethyl-1,3,2-dioxaborolan-2-yl)-1H-pyrazol-1-yl)propyl)piperazine-1-carboxylate used in Example 0197.
$^1$H-NMR(DMSO-

$d_6$)δ:12.10(1H,s),9.11(1H,d,J=2.0Hz),8.44-8.43(1H,m),8.33(1H,d,J=9.2Hz),7.50-7.46(4H,m),7.10-7.04(1H,m),3.89(3H,s),3.54-3.43(1H,m),2.20-2.10(2H,m),1.92-1.66(6H,m).
MSm/z(M+H):404.

<Example 0213>

<0213-1>

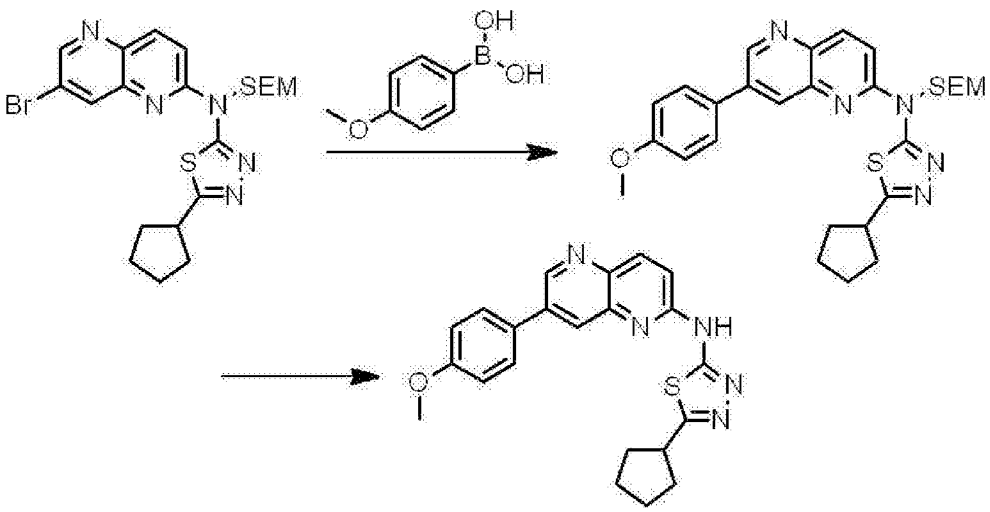

5-Cyclopentyl-N-(7-(4-methoxyphenyl)-1,5-naphthyridin-2-yl)-1,3,4-thiadiazole-2-amine was obtained as a yellow solid in the same manner as in Example 0197 except that 4-methoxyphenylboronic acid was used instead of the tert-butyl 4-(3-(4-(4,4,5,5-tetramethyl-1,3,2-dioxaborolan-2-yl)-1H-pyrazol-1-yl)propyl)piperazine-1-carboxylate used in Example 0197.
$^1$H-NMR(DMSO-$d_6$)δ:12.07(1H,s),9.10(1H,d,J=2.3Hz),8.37(1H,t,J=1.2Hz),8.30(1H,d,J=8.9Hz),7.91(2H,dd,J=6.9,2.0Hz ),7.45(1H,d,J=8.9Hz),7.13(2H,d,J=8.9Hz),3.85(3H,s),3.52-3.44(1H,m),2.21-2.13(2H,m),1.90-1.67(6H,m).
MSm/z(M+H):404.

<Example 0214>

<0214-1>

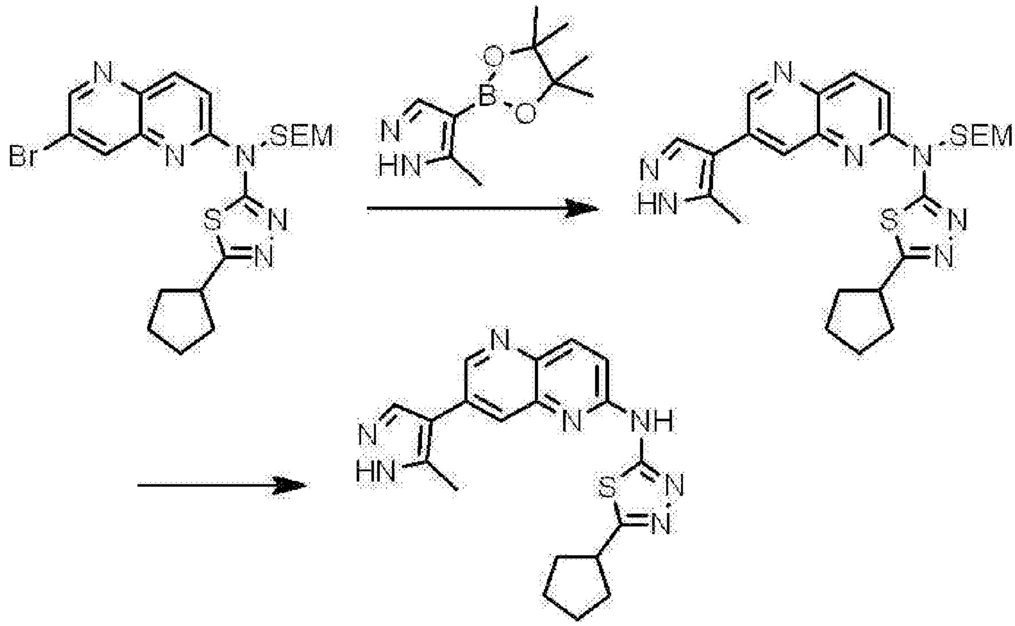

5-Cyclopentyl-N-(7-(5-methyl-1H-pyrazol-4-yl)-1,5-naphthyridin-2-yl)-1,3,4-thiadiazole-2-amine was obtained as a yellow solid in the same manner as in Example 0197 except that 3-methyl-1H-pyrazole-4-boronic acid pinacol ester was used instead of the tert-butyl 4-(3-(4-(4,4,5,5-tetramethyl-1,3,2-dioxaborolan-2-yl)-1H-pyrazol-1-yl)propyl)piperazine-1-carboxylate used in Example 0197.
$^1$H-NMR(DMSO-
$d_6$)δ:12.92(1H,s),12.04(1H,s),8.94(1H,d,J=1.7Hz),8.33-8.00(3H,m),7.41(1H,d,J=9.2Hz),3.54-3.43(1H,m),2.53(3H,s),2.20-2.12(2H,m),1.90-1.67(6H,m).
MSm/z(M+H):378.

<Example 0215>

<0215-1>

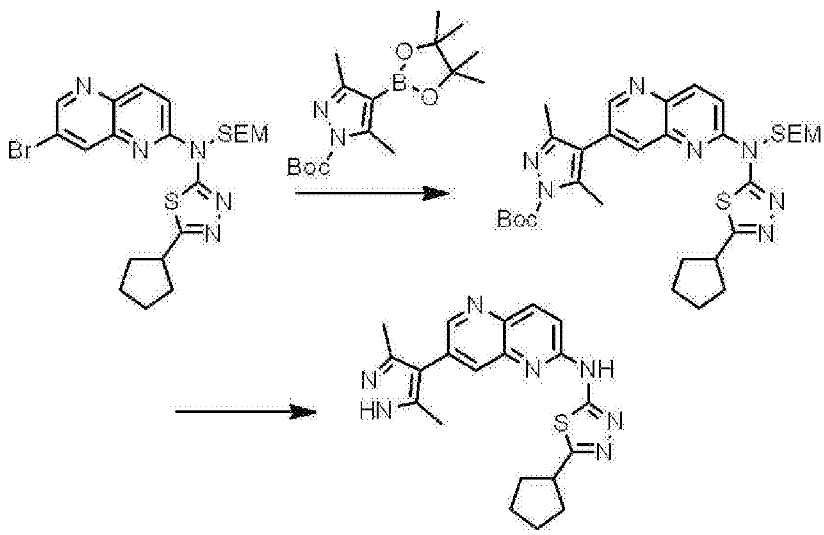

5-Cyclopentyl-N-(7-(3,5-dimethyl-1H-pyrazol-4-yl)-1,5-naphthyridin-2-yl)-1,3,4-thiadiazole-2-amine was obtained as a white solid in the same manner as in Example 0197 except that 1-tert-butoxycarbonyl-3,5-dimethylpyrazole-4-boronic acid pinacol ester was used instead of the tert-butyl 4-(3-(4-(4,4,5,5-tetramethyl-1,3,2-dioxaborolan-2-yl)-1H-pyrazole1-yl)propyl)piperazine-1-carboxylate used in Example 0197.

$^1$H-NMR(DMSO-$d_6$)$\delta$:12.52(1H,s),12.04(1H,s),8.67(1H,s),8.21(1H,d,J=8.6Hz),7.99(1H,s),7.38(1H,d,J=8.9Hz),3.47-3.40(1H,m),2.29(6H,s),2.18-2.07(2H,m),1.87-1.66(6H,m).
MSm/z(M+H):392.

<Example 0216>

<0216-1>

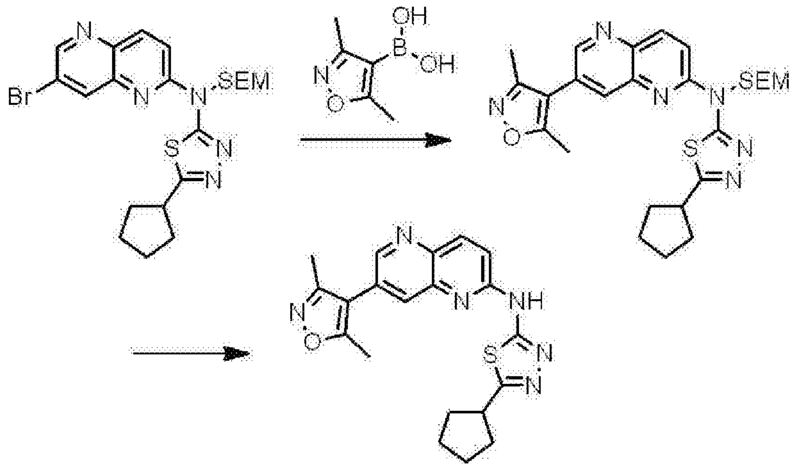

5-Cyclopentyl-N-(7-(3,5-dimethylisoxazol-4-yl)-1,5-naphthyridin-2-yl)-1,3,4-thiadiazole-2-amine was obtained as a pale yellow solid in the same manner as in Example 0197 except that 3,5-dimethylisoxazole-4-boronic acid was used instead of the tert-butyl 4-(3-(4-(4,4,5,5-tetramethyl-1,3,2-dioxaborolan-2-yl)-1H-pyrazole1-yl)propyl)piperazine-1-carboxylate used in Example 0197.
$^1$H-NMR($CD_3OD$)$\delta$:8.73(1H,d,J=2.0Hz),8.32-8.28(2H,m),7.47(1H,d,J=9.2Hz),3.54-3.49(1H,m),2.53(3H,s),2.36(3H,s),2.29-2.21(2H,m),1.95-1.75(6H,m).
MSm/z(M+H):393.

<Example 0217>

<0217-1>

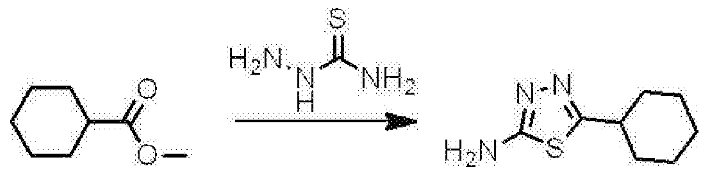

2-Amino-5-cyclohexyl-1,3,4-thiadiazole was obtained in the same manner as in Example 0198 except that methyl cyclohexanecarboxylate was used instead of the cyclobutanecarboxylic acid used in Example 0198.
MSm/z(M+H):184.

<0217-2>

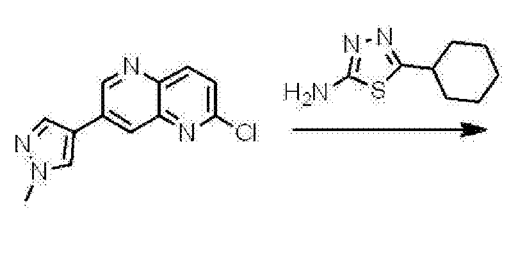

5-Cyclohexyl-N-(7-(1-methyl-1H-pyrazol-4-yl)-1,5-naphthyridin-2-yl)-1,3,4-thiadiazole-2-amine was obtained as a yellow solid in the same manner as in Example 0162 except that 2-amino-5-cyclohexyl-1,3,4-thiadiazole was used instead of the 2-amino-5-tert-butyl-1,3,4-thiadiazole used in Example 0162.
$^1$H-NMR(DMSO-$d_6$)δ:12.03(1H,s),9.07(1H,d,J=2.3Hz),8.53(1H,s),8.33(1H,d,J=1.7Hz),8.26-8.22(2H,m),7.39(1H,d,J=9.2Hz),3.93(3H,s),3.13-3.02(1H,m),2.17-2.08(2H,m),1.85-1.15(8H,m).
MSm/z(M+H):392.

<Example 0218>

<0218-1>

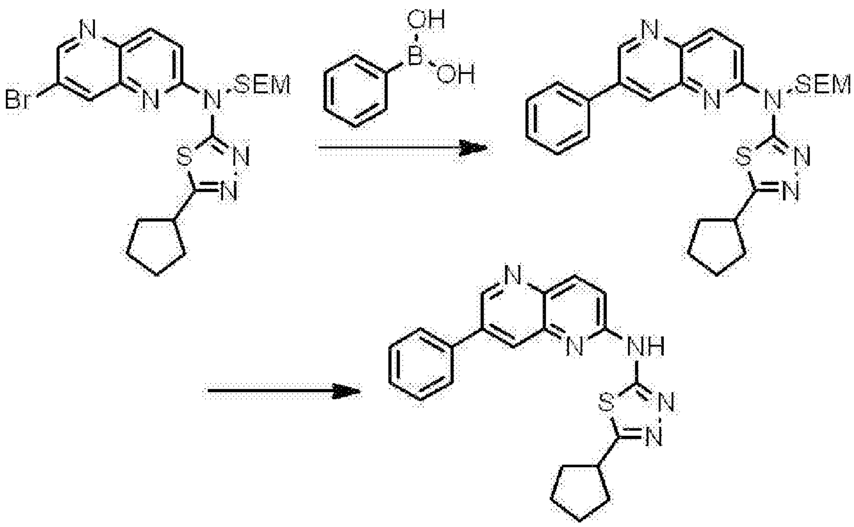

5-Cyclopentyl-N-(7-phenyl-1,5-naphthyridin-2-yl)-1,3,4-thiadiazole-2-amine was obtained as a pale yellow solid in the same manner as in Example 0197 except that phenylboronic acid was used instead of the tert-butyl 4-(3-(4-(4,4,5,5-tetramethyl-1,3,2-dioxaborolan-2-yl)-1H-pyrazol-1-yl)propyl)piperazine-1-carboxylate used in Example 0197.
$^1$H-NMR(DMSO-$d_6$)δ:12.10(1H,s),9.12(1H,d,J=2.0Hz),8.44(1H,d,J=1.7Hz),8.33(1H,d,J=9.2Hz),7.97-7.92(2H,m),7.61-7.55(2H,m),7.53-7.47(2H,m),3.57-3.44(1H,m),2.21-2.08(2H,m),1.92-1.64(6H,m).
MSm/z(M+H):374.

<Example 0219>

<0219-1>

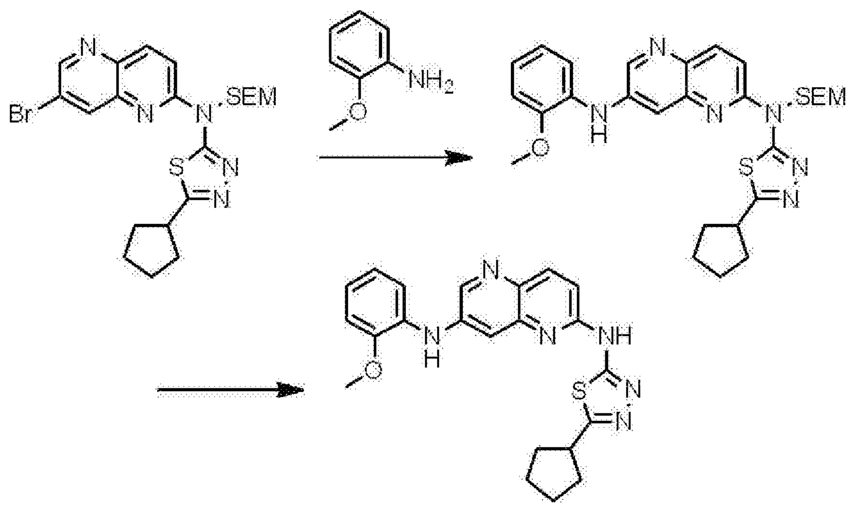

$N^2$-(5-cyclopentyl-1,3,4-thiadiazol-2-yl)-$N^7$-(2-methoxyphenyl)-1,5-naphthyridine-2,7-diamine was obtained as a yellow solid in the same manner as in Example 0175 except that o-anisidine was used instead of the tert-butyl 4-amino-1H-pyrazole-1-carboxylate used in Example 0175.
$^1$H-NMR(DMSO-$d_6$)$\delta$:11.84(1H,s),8.57(1H,d,J=2.3Hz),8.29(1H,s),8.09(1H,d,J=8.6Hz),7.38(1H,t,J=4.3Hz),7.33(1H,d,J=2.3Hz),7.16-7.09(3H,m),7.03-6.97(1H,m),3.84(3H,s),3.51-3.40(1H,m),2.16-2.07(2H,m),1.85-1.67(6H,m).
MSm/z(M+H):419.

<Example 0220>

<0220-1>

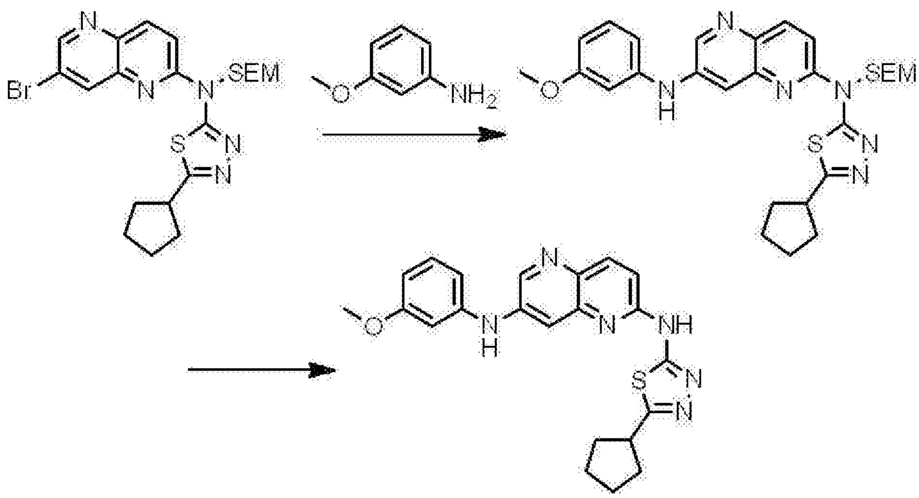

$N^2$-(5-cyclopentyl-1,3,4-thiadiazol-2-yl)-$N^7$-(3-methoxyphenyl)-1,5-naphthyridine-2,7-diamine was obtained as a yellow solid in the same manner as in Example 0175 except that m-anisidine was used instead of the tert-butyl 4-amino-1H-pyrazole-1-carboxylate used in Example 0175.
$^1$H-NMR(DMSO-$d_6$)$\delta$:11.89(1H,s),8.91(1H,s),8.55(1H,d,J=2.6Hz),8.11(1H,d,J=8.9Hz),7.64(1H,d,J=2.3Hz),7.29(1H,t,J=8.1Hz),7.18(1H,d,J=8.9Hz),6.84(2H,dt,J=12.1,3.4Hz),6.61(1H,dd,J=8.1,2.1Hz),3.78(3H,s),3.54-3.42(1H,m),2.17-2.08(2H,m),1.86-1.66(6H,m).
MSm/z(M+H):419.

<Example 0221>

<0221-1>

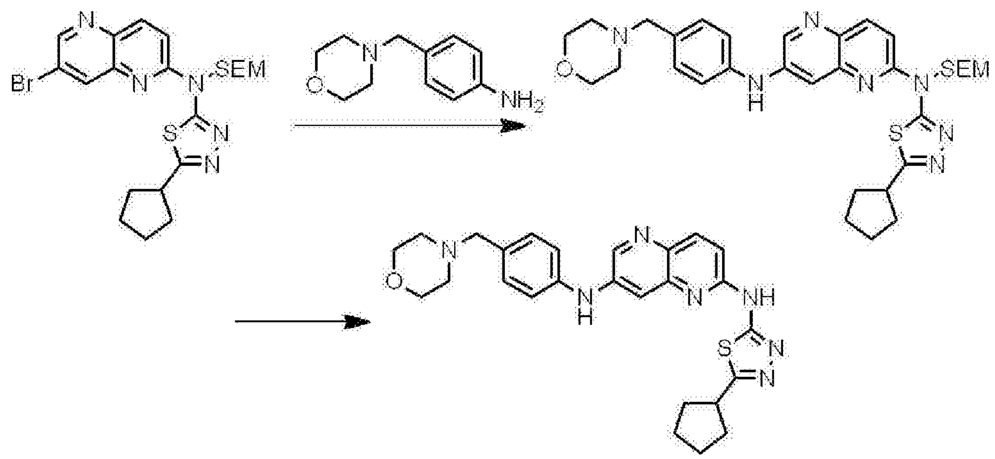

$N^2$-(5-cyclopentyl-1,3,4-thiadiazol-2-yl)-$N^7$-(4-(morpholinomethyl)phenyl)-1,5-naphthyridine-2,7-diamine was obtained as a yellow solid in the same manner as in Example 0175 except that 4-(morpholinomethyl)aniline was used instead of the tert-butyl 4-amino-1H-pyrazole-1-carboxylate used in Example 0175.
$^1$H-NMR(DMSO-$d_6$)$\delta$:11.87(1H,s),8.87(1H,s),8.55(1H,d,J=2.6Hz),8.10(1H,d,J=8.9Hz),7.56(1H,d,J=2.3Hz),7.31(2H,d,J =8.6Hz),7.23(2H,d,J=8.6Hz),7.17(1H,d,J=8.9Hz),3.61-3.56(4H,m),3.49-3.41(3H,m),2.40-2.35(4H,m),2.15-2.08(2H,m), 1.84-1.64(6H,m).
MSm/z(M+H):488.

<Example 0222>

<0222-1>

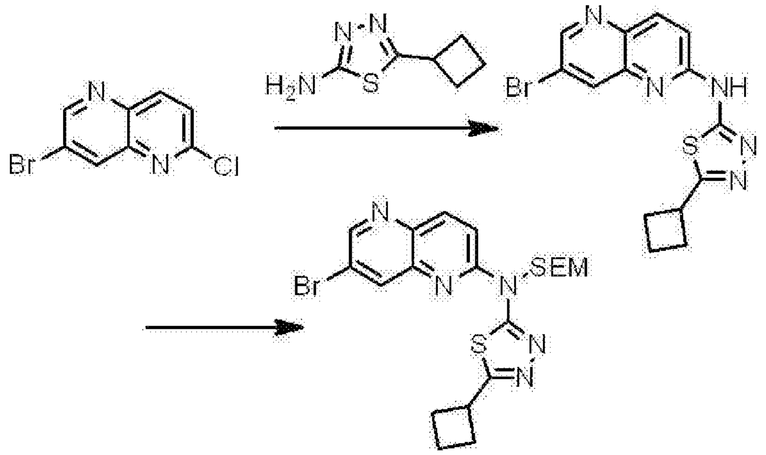

N-(7-bromo-1,5-naphthyridin-2-yl)-5-cyclobutyl-N-((2-(trimethylsilyl)ethoxy)methyl)-1,3,4-thiadiazole-2-amine was obtained as brown oily substance in the same manner as in Example 0175 except that 2-amino-5-cyclobutyl-1,3,4-thiadiazole was used instead of the 2-amino-5-cyclopentyl-1,3,4-thiadiazole used in Example 0175.
MSm/z(M+H):492,494.

<0222-2>

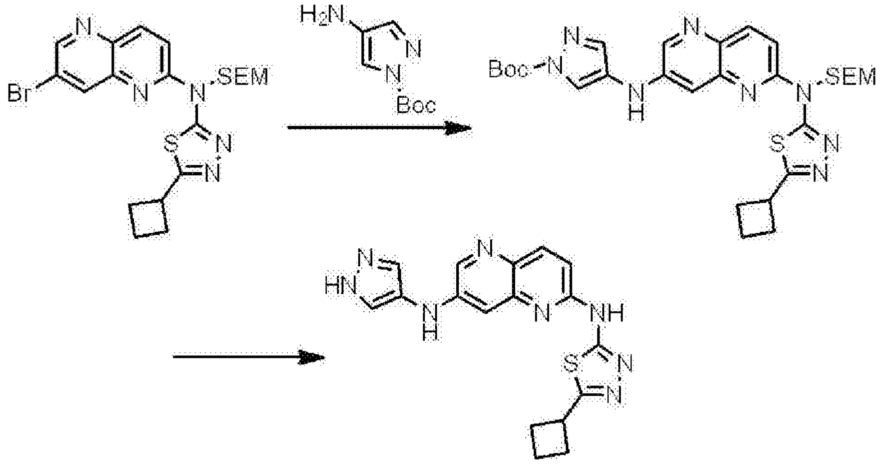

$N^2$-(5-cyclobutyl-1,3,4-thiadiazol-2-yl)-$N^7$-(1H-pyrazol-4-yl)-1,5-naphthyridine-2,7-diamine was obtained as a yellow solid in the same manner as in Example 0175 except that N-(7-bromo-1,5-naphthyridin-2-yl)-5-cyclobutyl-N-((2-(trimethylsilyl)ethoxy)methyl)-1,3,4-thiadiazole-2-amine was used instead of the N-(7-bromo-1,5-naphthyridin-2-yl)-5-cyclopentyl-N-((2-(trimethylsilyl)ethoxy)methyl)-1,3,4-thiadiazole-2-amine used in Example 0175.
$^1$H-NMR(DMSO-$d_6$)δ:12.30(1H,s),11.85(1H,s),9.28(1H,s),8.62(1H,d,J=2.3Hz),8.32(1H,d,J=2.0Hz),8.09(1H,d,J=8.9Hz), 7.69(1H,t,J=2.0Hz),7.15(1H,d,J=8.9Hz),5.95(1H,t,J=2.1Hz),4.04-3.87(1H,m),2.45-2.27(4H,m),2.16-1.90(2H,m).
MSm/z(M+H):365.

<Example 0223>

<0223-1>

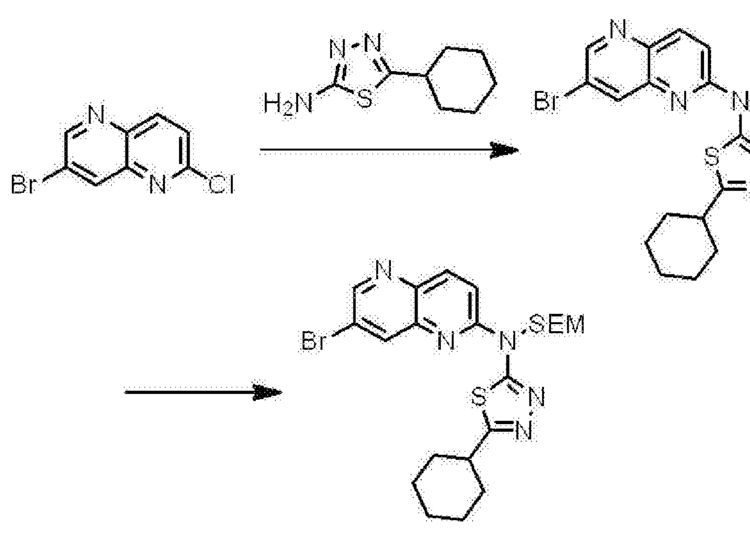

N-(7-bromo-1,5-naphthyridin-2-yl)-5-cyclohexyl-N-((2-(trimethylsilyl)ethoxy)methyl)-1,3,4-thiadiazole-2-amine was obtained as brown oily substance in the same manner as in Example 0175 except that 2-amino-5-cyclohexyl-1,3,4-thiadiazole was used instead of the 2-amino-5-cyclopentyl-1,3,4-thiadiazole used in Example 0175.
MSm/z(M+H):520,522.

<0223-2>

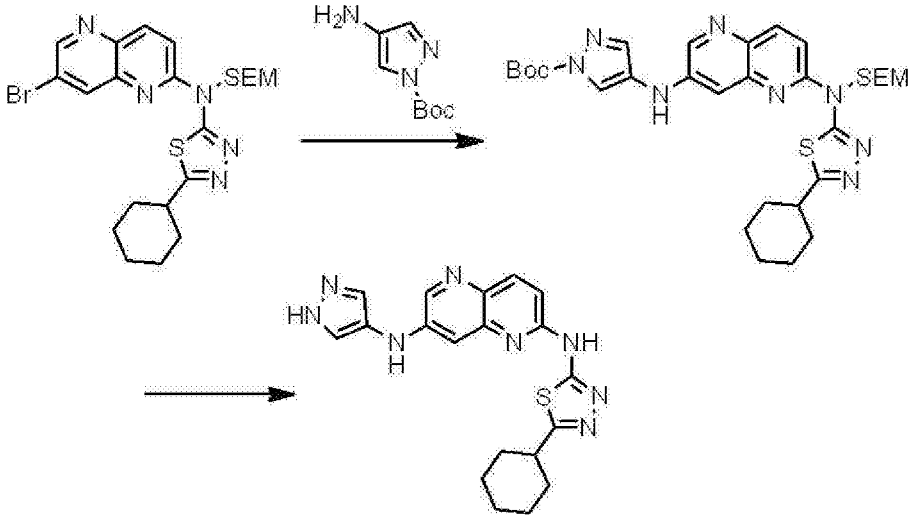

$N^2$-(5-cyclohexyl-1,3,4-thiadiazol-2-yl)-$N^7$-((1H-pyrazol-4-yl)-1,5-naphthyridine-2,7-diamine was obtained as a yellow solid in the same manner as in Example 0175 except that N-(7-bromo-1,5-naphthyridin-2-yl)-5-cyclohexyl-N-((2-(trimethylsilyl)ethoxy)methyl)-1,3,4-thiadiazole-2-amine was used instead of the N-(7-bromo-1,5-naphthyridin-2-yl)-5-cyclopentyl-N-((2-(trimethylsilyl)ethoxy)methyl)-1,3,4-thiadiazole-2-amine used in Example 0175.
$^1$H-NMR(DMSO-$d_6$)$\delta$:12.29(1H,s),11.83(1H,s),9.27(1H,s),8.62(1H,d,J=2.6Hz),8.31(1H,d,J=2.0Hz),8.08(1H,d,J=8.9Hz), 7.69(1H,t,J=2.0Hz),7.15(1H,d,J=8.9Hz),5.94(1H,t,J=2.3Hz),3.11-3.03(1H,m),2.16-2.04(2H,m),1.84-1.27(8H,m).
MSm/z(M+H):393.

<Example 0224>

<0224-1>

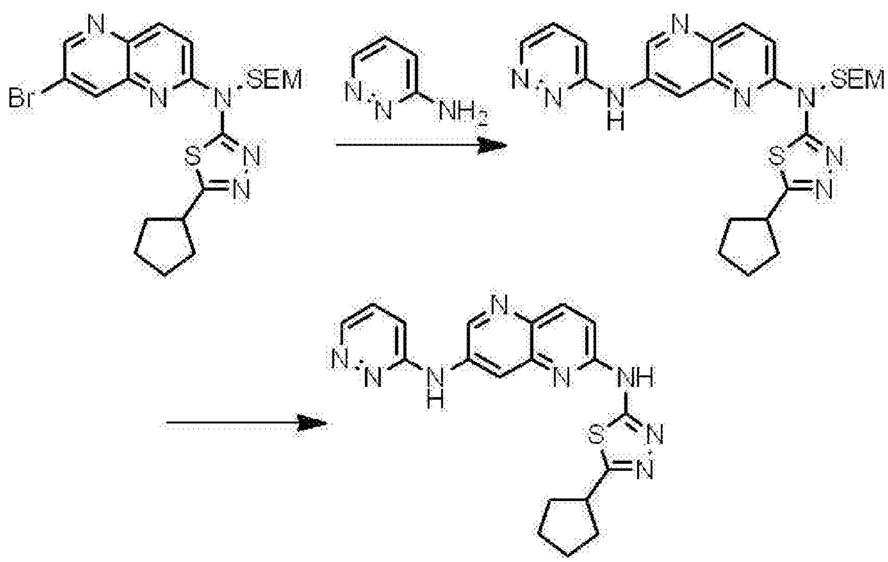

$N^2$-(5-cyclopentyl-1,3,4-thiadiazol-2-yl)-$N^7$-(pyridazin-3-yl)-1,5-naphthyridine-2,7-diamine was obtained as a yellow solid in the same manner as in Example 0175 except that 3-aminopyridazine was used instead of the tert-butyl

4-amino-1H-pyrazole-1-carboxylate used in Example 0175.
$^1$H-NMR(DMSO-$d_6$)δ:11.70(1H,s),9.02(1H,d,J=2.0Hz),8.49(1H,s),8.29(1H,d,J=1.7Hz),8.19(2H,dd,J=4.8,4.1Hz),7.31(1H,d,J=8.9Hz),3.92(3H,s),3.80-3.76(4H,m),3.48-3.42(4H,m).
MSm/z(M+H):391.

<Example 0225>

<0225-1>

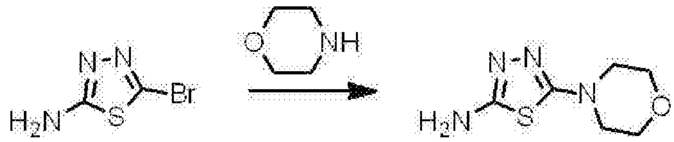

5-Morpholino-1,3,4-thiadiazole-2-amine was obtained in the same manner as in Example 0173 except that morpholine was used instead of the pyrrolidine used in Example 0173.
$^1$H-NMR(DMSO-$d_6$)δ:6.52(2H,s),3.67(4H,t,J=5.0Hz),3.20(4H,t,J=4.8Hz).

<0225-2>

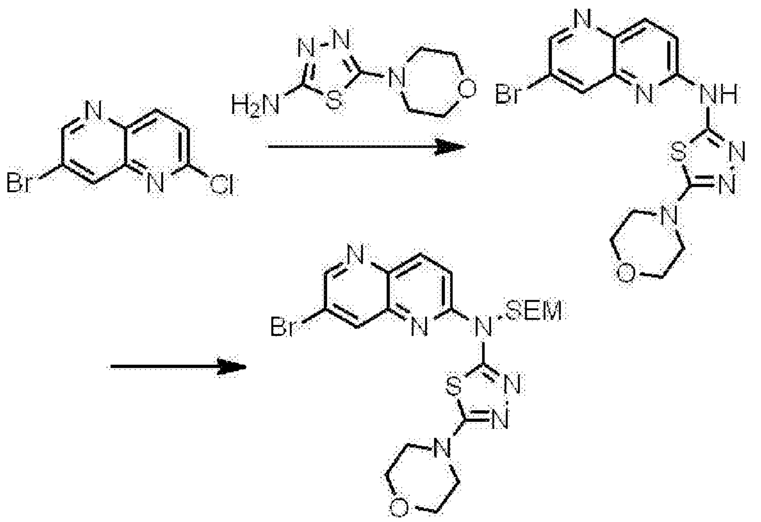

N-(7-bromo-1,5-naphthyridin-2-yl)-5-morpholino-N-((2-(trimethylsilyl)ethoxy)methyl)-1,3,4-thiadiazole-2-amine was obtained as brown oily substance in the same manner as in Example 0175 except that 5-morpholino-1,3,4-thiadiazole-2-amine was used instead of the 2-amino-5-cyclopentyl-1,3,4-thiadiazole used in Example 0175.
MSm/z(M+H):523,525.

<0225-3>

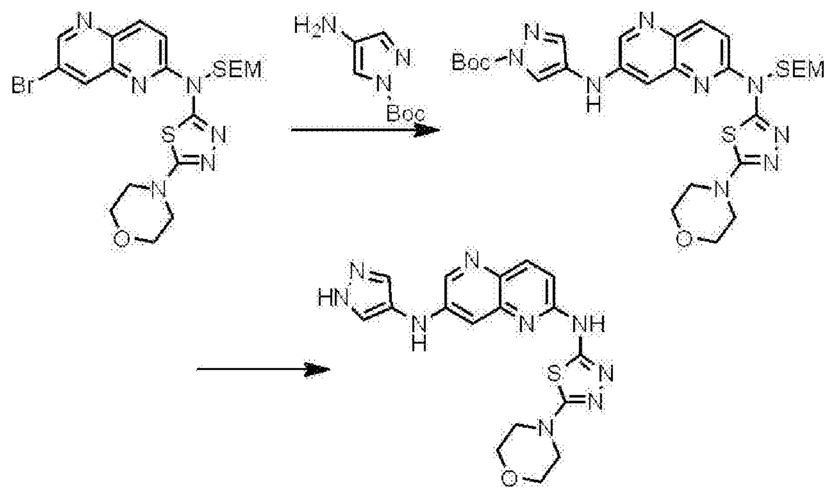

$N^2$-(5-morpholino-1,3,4-thiadiazol-2-yl)-$N^7$-(1H-pyrazol-4-yl)-1,5-naphthyridine-2,7-diamine was obtained as a yellow solid in the same manner as in Example 0175 except that N-(7-bromo-1,5-naphthyridin-2-yl)-5-morpholino-N-((2-(trimethylsilyl)ethoxy)methyl)-1,3,4-thiadiazole-2-amine was used instead of the N-(7-bromo-1,5-naphthyridin-2-yl)-5-cyclopentyl-N-((2-(trimethylsilyl)ethoxy)methyl)-1,3,4-thiadiazole-2-amine used in Example 0175.
$^1$H-NMR(DMSO-$d_6$)$\delta$:12.25(1H,s),11.48(1H,s),9.22(1H,s),8.61(1H,d,J=2.3Hz),8.22(1H,s),8.03(1H,d,J=8.9Hz),7.67(1H,s),7.07(1H,d,J=8.9Hz),5.94(1H,s),3.79-3.75(4H,m),3.43-3.39(4H,m).
MSm/z(M+H):396.

<Example 0226>

<0226-1>

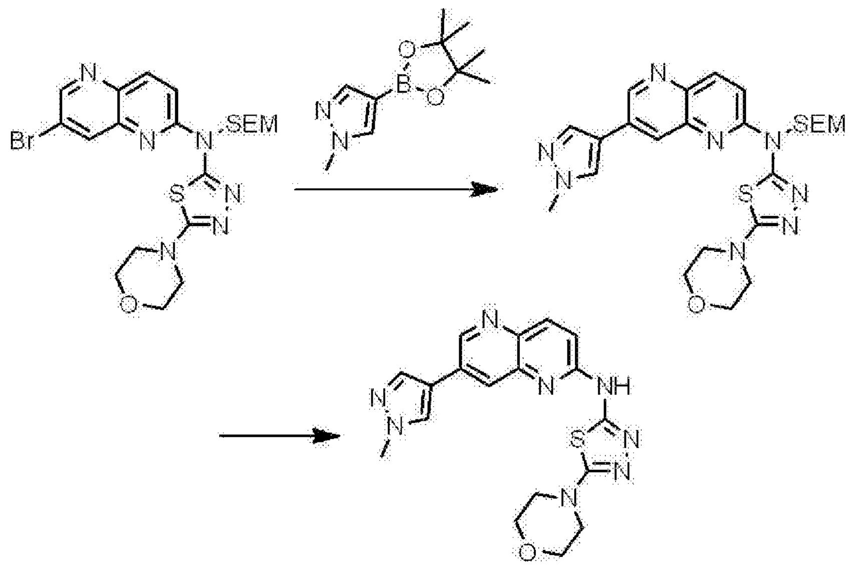

1-Methylpyrazole-4-boronic acid pinacol ester (10 mg), bis(di-tert-butyl (4-dimethylaminophenyl)phosphine)dichloropalladium(II) (5 mg), and 2 mol/L sodium carbonate aqueous solution (50 $\mu$L) were added to a solution of N-(7-bromo-1,5-naphthyridin-2-yl)-5-morpholino-N-((2-(trimethylsilyl)ethoxy)methyl)-1,3,4-thiadiazole-2-amine (20 mg) in 1,4-dioxane (1 mL), followed by stirring at 130°C for 0.5 hours using a microwave reaction apparatus. The reaction mixture was cooled to room temperature, and water and ethyl acetate were added thereto. The organic layer was collected by separation, and dried over anhydrous sodium sulfate, and the solvent was distilled off under reduced pressure. The solid matter was collected by filtration, thereby obtaining N-(7-(1-methyl-1H-pyrazol-4-yl)-1,5-naphthyridin-2-yl)-5-morpholino-N-((2-(trimethylsilyl)ethoxy)methyl)-1,3,4-thiadiazole-2-amine.

A4 mol/L hydrogen chloride/1,4-dioxane solution (1 mL) was added to the obtained N-(7-(1-methyl-1H-pyrazol-4-yl)-1,5-naphthyridin-2-yl)-5-morpholino-N-((2-(trimethylsilyl)ethoxy)methyl)-1,3,4-thiadiazole-2-amine, followed by allowing to stand at room temperature overnight. The solvent was distilled off under reduced pressure, and the obtained residue was purified by preparative thin layer silica gel chromatography (chloroform-methanol, NH silica), thereby obtaining N-(7 -(1-methyl-1H-pyrazol-4-yl)-1,5-naphthyridin-2-yl)-5-morpholino-1,3,4-thiadiazole-2-amine (9 mg) as a yellow solid.

$^1$H-NMR(DMSO-$d_6$)δ:11.70(1H,s),9.02(1H,d,J=2.0Hz),8.49(1H,s),8.29(1H,d,J=1.7Hz),8.19(2H,dd,J=4.8,4.1Hz),7.31(1H,d,J=8.9Hz),3.92(3H,s),3.80-3.76(4H,m),3.48-3.42(4H,m).

MSm/z(M+H):395.

<Example 0227>

<0227-1>

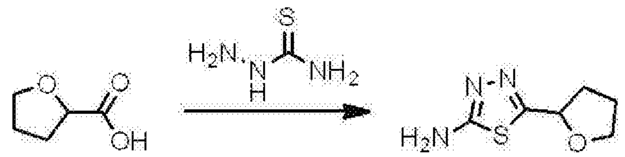

5-(Tetrahydrofuran-2-yl)-1,3,4-thiadiazole-2-amine was obtained in the same manner as in Example 0198 except that tetrahydrofuran-2-carboxylic acid was used instead of the cyclobutanecarboxylic acid used in Example 0198.

$^1$H-NMR(DMSO-$d_6$)δ:7.13(2H,s),5.03-4.95(1H,m),3.88-3.73(2H,m),2.32-2.21(1H,m),2.06-1.87(3H,m).

<0227-2>

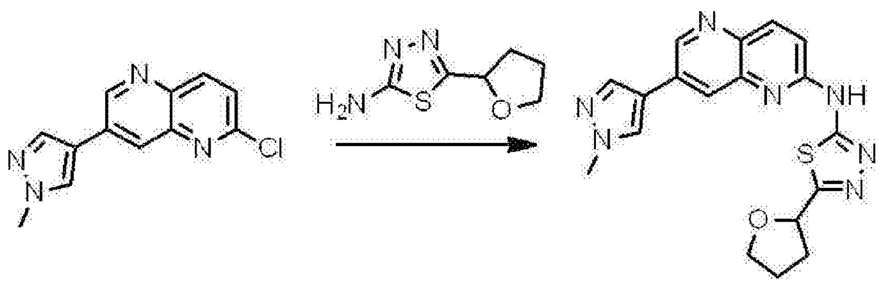

N-(7-(1-methyl-1H-pyrazol-4-yl)-1,5-naphthyridin-2-yl)-5-(tetrahydrofuran-2-yl)-1,3,4-thiadiazole-2-amine was obtained as a white solid in the same manner as in Example 0162 except that 5-(tetrahydrofuran-2-yl)-1,3,4-thiadiazole-2-amine was used instead of the 2-amino-5-tert-butyl-1,3,4-thiadiazole used in Example 0162.

$^1$H-NMR(DMSO-$d_6$)δ:12.12(1H,s),9.08(1H,d,J=2.0Hz),8.54(1H,d,J=4.0Hz),8.31(1H,d,J=2.0Hz),8.26(1H,d,J=9.2Hz),8.23(1H,d,J=0.7Hz),7.41(1H,d,J=8.9Hz),5.24(1H,dd,J=7.1,6.1Hz),4.06-3.98(1H,m),3.92(3H,s),3.90-3.85(1H,m),2.43-2.34(1H,m),2.26-2.17(1H,m),2.07-1.99(2H,m).

MSm/z(M+H):380.

<Example 0228>

<0228-1>

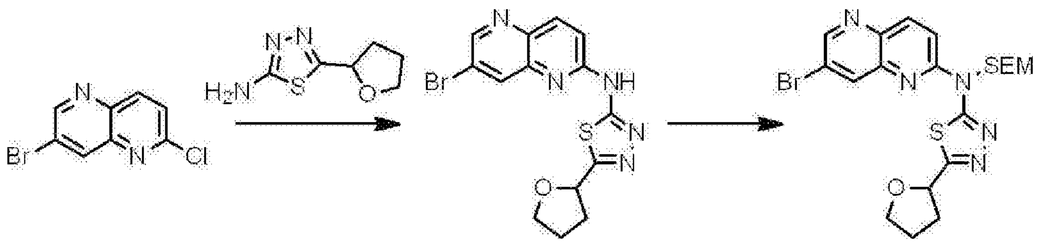

N-(7-bromo-1,5-naphthyridin-2-yl)-5-(tetrahydrofuran-2-yl)-N-((2-(trimethylsilyl)ethoxy)methyl)-1,3,4-thiadiazole-2-amine was obtained as brown oily substance in the same manner as in Example 0175 except that 5-(tetrahydrofuran-2-yl)-1,3,4-thiadiazole-2-amine was used instead of the 2-amino-5-cyclopentyl-1,3,4-thiadiazole used in Example 0175.
MSm/z(M+H):508,510.

<0228-2>

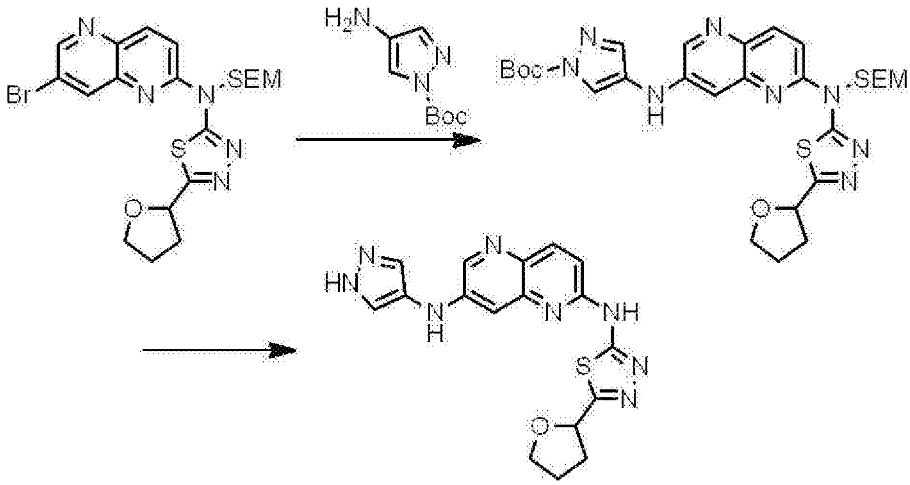

$N^7$-(1H-pyrazol-4-yl)-$N^2$-(5-(tetrahydrofuran-2-yl)-1,3,4-thiadiazol-2-yl)-1,5-naphthyridine-2,7-diamine was obtained as a yellow solid in the same manner as in Example 0175 except that N-(7-bromo-1,5-naphthyridin-2-yl)-5-(tetrahydrofuran-2-yl)-N-((2-(trimethylsilyl)ethoxy)methyl)-1,3,4-thiadiazole-2-amine was used instead of the N-(7-bromo-1,5-naphthyridin-2-yl)-5-cyclopentyl-N-((2-(trimethylsilyl)ethoxy)methyl)-1,3,4-thiadiazole-2-amine used in Example 0175.
$^1$H-NMR(DMSO-$d_6$)δ:12.35(1H,s),11.93(1H,s),9.32(1H,s),8.62(1H,d,J=2.3Hz),8.37(1H,s),8.10(1H,d,J=8.9Hz),7.69(1H,d,J=2.0Hz),7.16(1H,d,J=8.9Hz),5.94(1H,s),5.24(1H,dd,J=7.3,5.9Hz),4.05-3.84(2H,m),2.43-2.17(2H,m),2.05-1.96(2H,m).
MSm/z(M+H):381.

<Example 0229>

<0229-1>

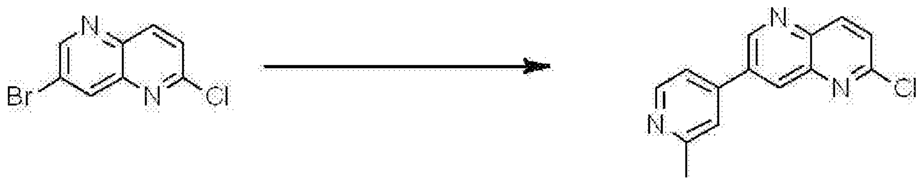

(2-Methylpyridin-4-yl)boronic acid (34 mg), bis(di-tert-butyl (4-dimethylaminophenyl)phosphine)dichloropalladium(II) (14 mg), and sodium carbonate (45 mg) were added to a mixture solution of 7-bromo-2-chloro-1,5-naphthyridine (50 mg) in 1,4-dioxane (5 mL)/water (0.5 mL), followed by stirring at 100°C for 2 hours in a nitrogen atmosphere. The reaction mixture was cooled to room temperature, the solvent was distilled off under reduced pressure, and the obtained residue was purified by silica gel column chromatography (hexane/ethyl acetate → ethyl acetate/methanol), thereby obtaining 2-chloro-7-(2-methylpyridin-4-yl)-1,5-naphthyridine (35 mg).
$^1$H-NMR(DMSO-$d_6$)δ:9.48(1H,d,J=2.0Hz),8.82(1H,d,J=2.0Hz),8.62(1H,d,J=5.3Hz),8.56(1H,d,J=8.6Hz),7.96-7.87(2H,m),7.82(1H,d,J=5.3Hz),2.59(3H,s).

<0229-2>

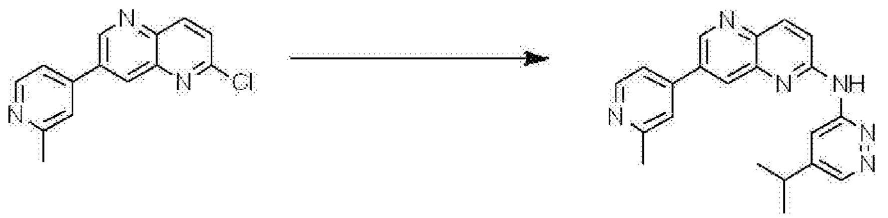

5-Isopropylpyridazine-3-amine (23 mg), tris(dibenzylideneacetone)dipalladium(0) (9 mg), 4,5-bis(diphenylphosphino)-9,9-dimethylxanthene (17 mg), and cesium carbonate (114 mg) were added to a solution of 2-chloro-7-(2-methylpyridin-4-yl)-1,5-naphthyridine (35 mg) in 1,4-dioxane (5 mL), followed by stirring at 140°C for 30 minutes using a microwave reaction apparatus. The reaction mixture was cooled to room temperature, and the solvent was distilled off under reduced pressure. The obtained residue was purified by silica gel column chromatography (hexane/ethyl acetate → ethyl acetate/methanol), and silica gel column chromatography (chloroform/methanol, NH silica), thereby obtaining N-(5-isopropylpyridazin-3-yl)-7-(2-methylpyridin-4-yl)-1,5-naphthyridine-2-amine (11 mg) as a white solid.
$^1$H-NMR(DMSO-$d_6$)δ:10.83(1H,s),9.14(1H,d,J=2.0Hz),8.89(1H,d,J=1.3Hz),8.77(1H,d,J=2.0Hz),8.61(1H,d,J=5.3Hz),8.48(1H,d,J=2.0Hz),8.32(1H,d,J=9.2Hz),7.87-7.78(2H,m),7.76(1H,d,J=5.3Hz),3.13-2.98(1H,m),2.60(3H,s),1.33(6H,d,J=7.3Hz).
MSm/z(M+H):357.

<Example 0230>

<0230-1>

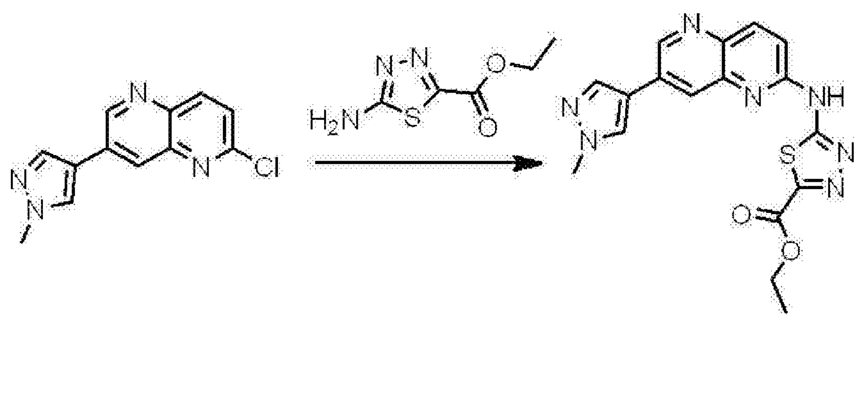

Ethyl 5-((7-(1-methyl-1H-pyrazol-4-yl)-1,5-naphthyridin-2-yl)amino)-1,3,4-thiadiazole-2-carboxylate was obtained as a white solid in the same manner as in Example 0162 except that ethyl 5-amino-1,3,4-thiadiazole-2-carboxylate was used instead of the 2-amino-5-tert-butyl-1,3,4-thiadiazole used in Example 0162.
$^1$H-NMR(DMSO-$d_6$)δ:12.76(1H,s),9.13(1H,d,J=2.0Hz),8.57(1H,s),8.39(1H,d,J=1.7Hz),8.34(1H,d,J=8.9Hz),8.25(1H,s), 7.48(1H,d,J=8.9Hz),4.45(2H,q,J=7.0Hz),3.93(3H,s),1.39(3H,t,J=7.1Hz).
MSm/z(M+H):382.

<Example 0231>

<0231-1>

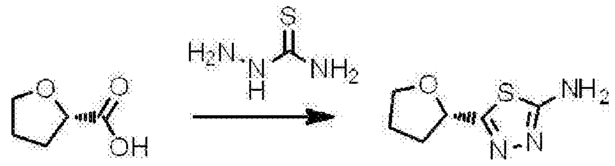

(S)-5-(tetrahydrofuran-2-yl)-1,3,4-thiadiazole-2-amine was obtained as a white solid in the same manner as in Example 0198 except that (S)-(-)-tetrahydrofuran-2-carboxylic acid was used instead of the cyclobutanecarboxylic acid used in Example 0198.
$^1$H-NMR(DMSO-$d_6$)δ:7.13(2H,s),5.02-4.95(1H,m),3.88-3.73(2H,m),2.31-2.20(1H,m),2.04-1.89(3H,m).

<0231-2>

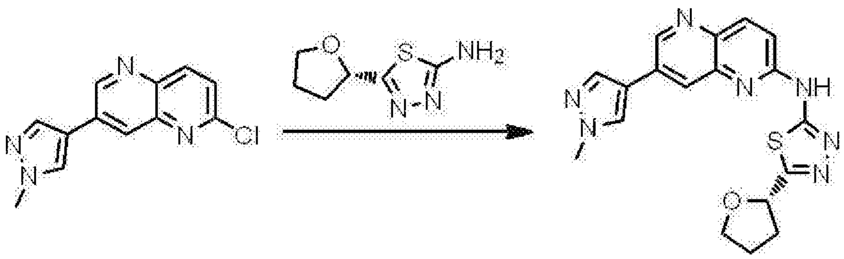

(S)-N-(7-(1-methyl-1H-pyrazol-4-yl)-1,5-naphthyridin-2-yl)-5-(tetrahydrofuran-2-yl)-1,3,4-thiadiazole-2-amine was obtained as a white solid in the same manner as in Example 0162 except that (S)-5-(tetrahydrofuran-2-yl)-1,3,4-thiadiazole-2-amine was used instead of the 2-amino-5-tert-butyl-1,3,4-thiadiazole used in Example 0162.
$^1$H-NMR(DMSO-$d_6$)δ:12.13(1H,s),9.08(1H,d,J=2.3Hz),8.55(1H,s),8.31(1H,d,J=1.7Hz),8.27(1H,d,J=9.2Hz),8.23(1H,d,J =0.7Hz),7.41(1H,d,J=9.2Hz),5.24(1H,dd,J=7.3,5.9Hz),4.06-3.85(5H,m),2.45-2.34(1H,m),2.28-2.14(1H,m),2.07-1.98(2 H,m).
MSm/z(M+H):380.

<Example 0232>

<0232-1>

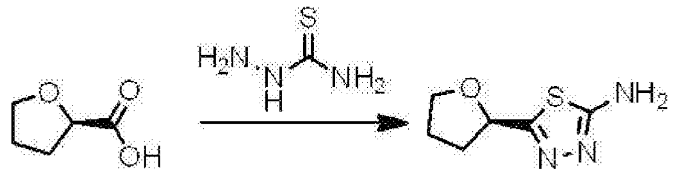

(R)-5-(tetrahydrofuran-2-yl)-1,3,4-thiadiazole-2-amine was obtained as a white solid in the same manner as in Example 0198 except that (R)-(+)-tetrahydrofuran-2-carboxylic acid was used instead of the cyclobutanecarboxylic acid used in Example 0198.
$^1$H-NMR(DMSO-$d_6$)δ:7.13(2H,s),5.02-4.95(1H,m),3.88-3.73(2H,m),2.29-2.21(1H,m),2.06-1.88(3H,m).

<0232-2>

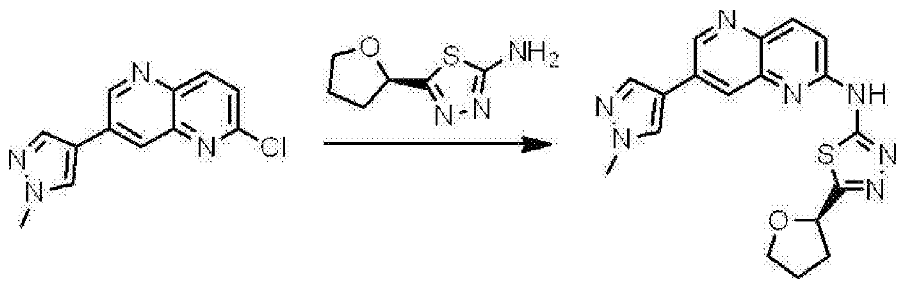

(R)-N-(7-(1-methyl-1H-pyrazol-4-yl)-1,5-naphthyridin-2-yl)-5-(tetrahydrofuran-2-yl)-1,3,4-thiadiazole-2-amine was obtained as a white solid in the same manner as in Example 0231 except that (R)-5-(tetrahydrofuran-2-yl)-1,3,4-thiadiazole-2-amine was used instead of the (S)-5-(tetrahydrofuran-2-yl)-1,3,4-thiadiazole-2-amine used in Example 0231.
$^1$H-NMR(DMSO-$d_6$)δ:12.12(1H,s),9.07(1H,d,J=2.0Hz),8.55(1H,s),8.30(1H,d,J=1.7Hz),8.25(1H,d,J=8.9Hz),8.23(1H,s), 7.40(1H,d,J=8.9Hz),5.24(1H,dd,J=7.1,6.1Hz),4.05-3.85(5H,m),2.45-2.34(1H,m),2.28-2.15(1H,m),2.07-1.96(2H,m).
MSm/z(M+H):380.

<Example 0233>

<0233-1>

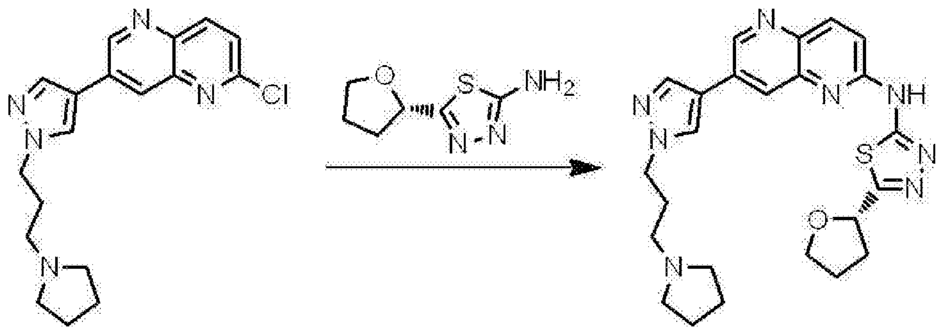

(S)-N-(7-(1-(3-(pyrrolidin-1-yl)propyl)-1H-pyrazol-4-yl)-1,5-naphthyridin-2-yl)-5-(tetrahydrofuran-2-yl)-1,3,4-thiadiazole-2-amine was obtained as a yellow solid in the same manner as in Example 0231 except that 2-chloro-7-(1-(3-(pyrrolidin-1-yl)propyl)-1H-pyrazol-4-yl)-1,5-naphthyridine was used instead of the 2-chloro-7-(1-methyl-1H-pyrazol-4-yl)-1,5-naphthyridine used in Example 0231, and 2-dicyclohexylphosphino-2',4',6'-triisopropylbiphenyl was used instead of the 4,5-bis(diphenylphosphino)-9,9-dimethylxanthene used in Example 0231.
$^1$H-NMR(DMSO-

$d_6$)δ:12.14(1H,s),9.07(1H,d,J=2.3Hz),8.59(1H,s),8.32-8.21(3H,m),7.39(1H,d,J=9.2Hz),5.23(1H,dd,J=7.1,6.1Hz),4.21(2H,t,J=7.1Hz),4.05-3.84(2H,m),2.46-2.36(7H,m),2.27-2.14(1H,m),2.09-1.96(4H,m),1.74-1.64(4H,m).
MSm/z(M+H):477.

<Example 0234>

<0234-1>

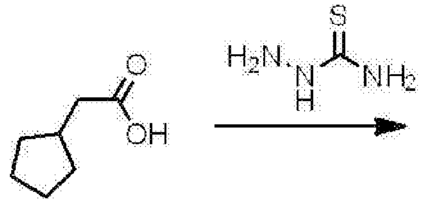

5-(Cyclopentylmethyl)-1,3,4-thiadiazole-2-amine was obtained as a white solid in the same manner as in Example 0198 except that cyclopentylacetic acid was used instead of the cyclobutanecarboxylic acid used in Example 0198.
MSm/z(M+H):184.

<0234-2>

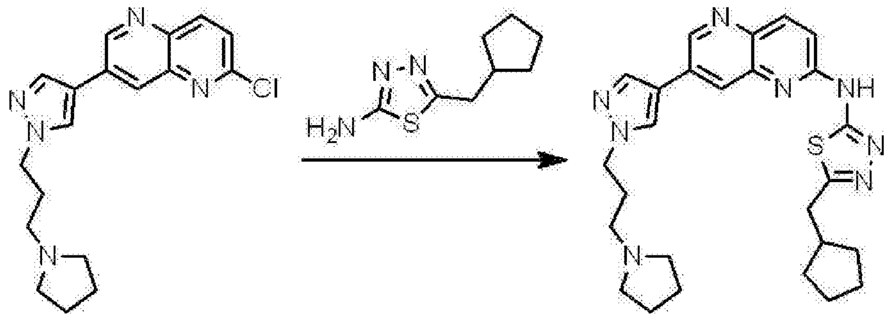

5-(cyclopentylmethyl)-N-(7-(1-(3-(pyrrolidin-1-yl)propyl)-1H-pyrazol-4-yl)-1,5-naphthyridin-2-yl)-1,3,4-thiadiazole-2-amine was obtained as a yellow solid in the same manner as in Example 0233 except that 5-(cyclopentylmethyl)-1,3,4-thiadiazole-2-amine was used instead of the (S)-5-(tetrahydrofuran-2-yl)-1,3,4-thiadiazole-2-amine used in Example 0233.
$^1$H-NMR(DMSO-$d_6$)δ:12.05(1H,s),9.07(1H,d,J=2.0Hz),8.57(1H,s),8.31(1H,d,J=1.7Hz),8.26(1H,s),8.22(1H,d,J=2.0Hz),7.39(1H,d,J=8.9Hz),4.21(2H,t,J=7.1Hz),3.00(2H,d,J=7.6Hz),2.45-2.37(5H,m),2.36-2.26(1H,m),2.07-1.96(2H,m),1.86-1.49(10H,m),1.35-1.21(3H,m).
MSm/z(M+H):489.

<Example 0235>

<0235-1>

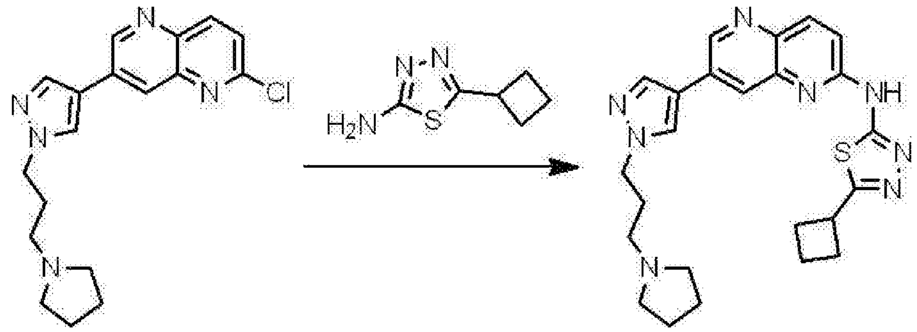

5-Cyclobutyl-N-(7-(1-(3-(pyrrolidin-1-yl)propyl)-1H-pyrazol-4-yl)-1,5-naphthyridin-2-yl)-1,3,4-thiadiazole-2-amine was obtained as a yellow solid in the same manner as in Example 0233 except that 2-amino-5-cyclobutyl-1,3,4-thiadiazole was used instead of the (S)-5-(tetrahydrofuran-2-yl)-1,3,4-thiadiazole-2-amine used in Example 0233.
$^1$H-NMR(DMSO-$d_6$)δ:12.02(1H,s),9.03(1H,d,J=1.7Hz),8.56(1H,s),8.29(1H,s),8.21(1H,s),8.18(1H,d,J=8.9Hz),7.34(1H,d ,J=8.9Hz),4.21(2H,t,J=6.9Hz),3.96-3.85(1H,m),2.46-2.19(10H,m),2.16-1.91(4H,m),1.71-1.67(4H,m).
MSm/z(M+H):461.

<Example 0236>

<0236-1>

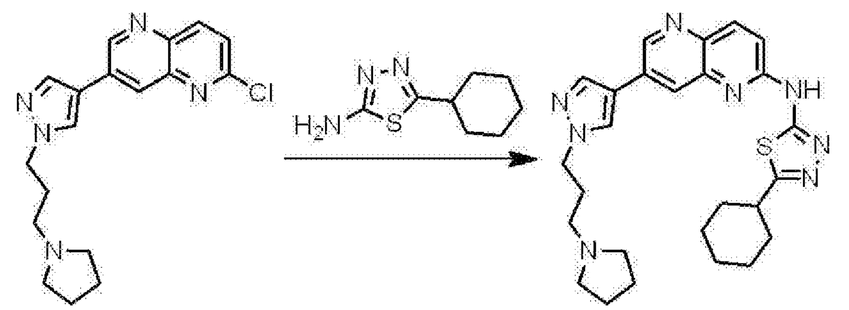

5-Cyclohexyl-N-(7-(1-(3-(pyrrolidin-1-yl)propyl)-1H-pyrazol-4-yl)-1,5-naphthyridin-2-yl)-1,3,4-thiadiazole-2-amine was obtained as a yellow solid in the same manner as in Example 0233 except that 2-amino-5-cyclohexyl-1,3,4-thiadiazole was used instead of the (S)-5-(tetrahydrofuran-2-yl)-1,3,4-thiadiazole-2-amine used in Example 0233.
$^1$H-NMR(DMSO-$d_6$)δ:12.05(1H,s),9.02(1H,d,J=1.7Hz),8.56(1H,s),8.32-8.16(3H,m),7.33(1H,d,J=9.2Hz),4.21(2H,t,J=7.1Hz),3.09-3.01(1H,m),2.44-2.38(6H,m),2.12-1.23(16H,m).
MSm/z(M+H):489.

<Example 0237>

<0237-1>

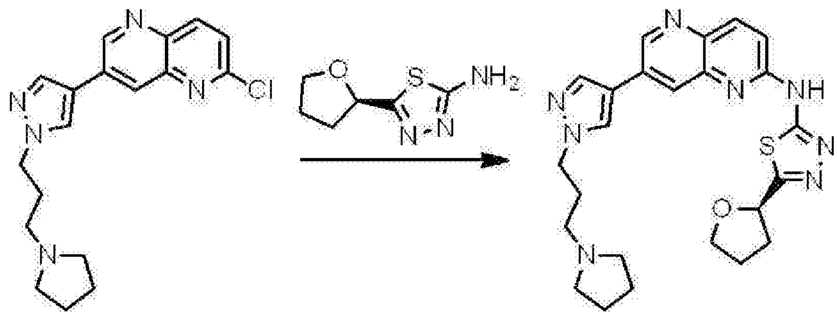

(R)-N-(7-(1-(3-(pyrrolidin-1-yl)propyl)-1H-pyrazol-4-yl)-1,5-naphthyridin-2-yl)-5-(tetrahydrofuran-2-yl)-1,3,4-thiadiazole-2-amine was obtained as a yellow solid in the same manner as in Example 0233 except that 2-chloro-7-(1-(3-(pyrrolidin-1-yl)propyl)-1H-pyrazol-4-yl)-1,5-naphthyridine was used instead of the 2-chloro-7-(1-methyl-1H-pyrazol-4-yl)-1,5-naphthyridine used in Example 0232.
$^1$H-NMR(DMSO-$d_6$)$\delta$:12.13(1H,s),9.09(1H,d,J=2.0Hz),8.60(1H,s),8.32(1H,d,J=1.7Hz),8.27(1H,d,J=9.6Hz),8.24(1H,s),7.41(1H,d,J=8.9Hz),5.24(1H,dd,J=7.3,5.9Hz),4.22(2H,t,J=6.9Hz),4.05-3.85(2H,m),244-2.15(8H,m),2.07-1.87(4H,m),1 .69(4H,s).
MSm/z(M+H):477.

<Example 0238>

<0238-1>

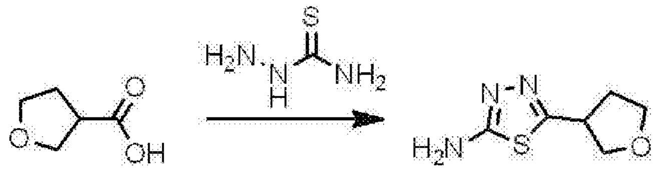

5-(Tetrahydrofuran-3-yl)-1,3,4-thiadiazole-2-amine was obtained as a white solid in the same manner as in Example 0198 except that (tetrahydrofuran-3-carboxylic acid was used instead of the cyclobutanecarboxylic acid used in Example 0198.
MSm/z(M+H):172.

<0238-2>

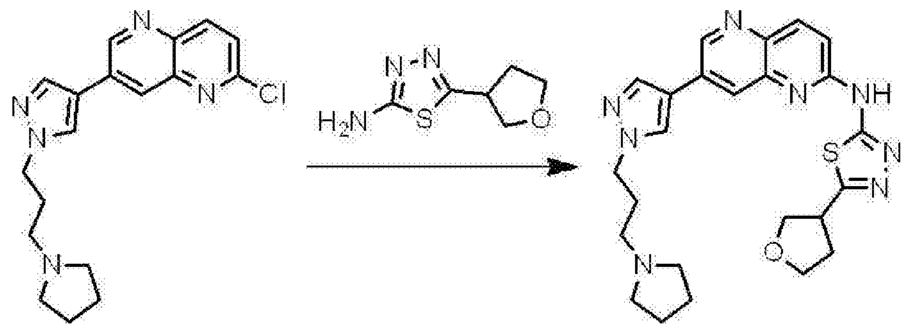

N-(7-(1-(3-(pyrrolidin-1 -yl)propyl)-1H-pyrazol-4-yl)-1,5-naphthyridin-2-yl)-5-(tetrahydrofuran-3-yl)-1,3,4-thiadiazole-2-amine was obtained as a yellow solid in the same manner as in Example 0233 except that 5-(tetrahydrofuran-3-yl)-1,3,4-thiadiazole-2-amine was used instead of the (S)-5-(tetrahydrofuran-2-yl)-1,3,4-thiadiazole-2-amine used in Example 0233.

$^{1}$H-NMR(DMSO-$d_6$)$\delta$:12.13(1H,s),9.09(1H,d,J=2.3Hz),8.57(1H,s),8.33(1H,d,J=1.7Hz),8.26(1H,d,J=9.2Hz),8.23(1H,s), 7.40(1H,d,J=8.9Hz),4.22(2H,t,J=6.9Hz),4.15-4.08(1H,m),4.02-3.82(4H,m),2.46-2.39(6H,m),2.35-2.19(2H,m),2.07-1.9 8(2H,m),1.74-1.66(4H,m).
MSm/z(M+H):477.

<Example 0239>

<0239-1>

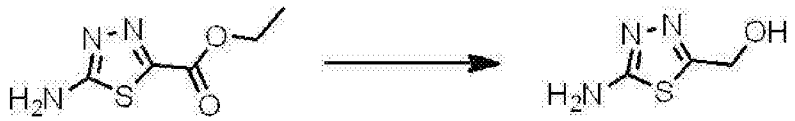

A solution of ethyl 5-amino-1,3,4-thiadiazole-2-carboxylate (340 mg) in tetrahydrofuran (10 mL) was cooled by ice, and lithium aluminum hydride (a 10% tetrahydrofuran solution, about 2.5 mol/L, 1 mL) was added dropwise thereto, followed by stirring at room temperature for 1 hour. After the reaction mixture was cooled by ice, ethyl acetate (2 mL) was added thereto, followed by stirring for 15 minutes under ice-cooling, and methanol (1 mL) was added thereto, followed by stirring for 5 minutes under ice-cooling. Anhydrous sodium sulfate aqueous solution (anhydrous sodium sulfate: 100 mg, water: 2 mL) was added to the reaction mixture, followed by neutralizing with 2 mol/L hydrochloric acid. The solid matter was separated by filtration, ethyl acetate was added thereto, and liquid-liquid separation was performed. The organic layer was collected by separation, and dried over anhydrous sodium sulfate, and the solvent was distilled off under reduced pressure. The obtained residue was purified by silica gel column chromatography (chloroform-methanol), thereby obtaining (5-amino-1,3,4-thiadiazol-2-yl)methanol (75 mg).
$^{1}$H-NMR(DMSO-$d_6$)$\delta$:7.07(2H,s),5.74(1H,t,J=6.1Hz),4.55(2H,d,J=5.9Hz).

<0239-2>

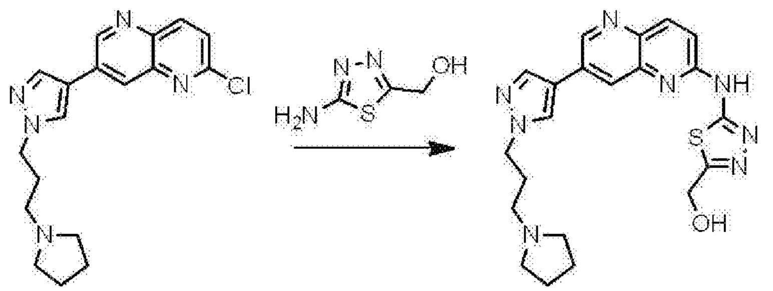

(5-((7-(1-(3-(Pyrrolidin-1-yl)propyl)-1H-pyrazol-4-yl)-1,5-naphthyridin-2-yl)amino)-1,3,4-thiadiazol-2-yl)methanol was obtained as a yellow solid in the same manner as in Example 0233 except that (5-amino-1,3,4-thiadiazol-2-yl)methanol was used instead of the (S)-5-(tetrahydrofuran-2-yl)-1,3,4-thiadiazole-2-amine used in Example 0233.
$^{1}$H-NMR(DMSO-$d_6$)$\delta$:12.09(1H,s),9.09(1H,d,J=2.0Hz),8.60(1H,s),8.32-8.25(2H,m),8.23(1H,s),7.41(1H,d,J=8.9Hz),5.98(1H,t,J=5.8Hz), 4.82(2H,d,J=5.9Hz),4.21(2H,t,J=6.9H z),2.47-2.40(6H,m),2.09-1.99(2H,m),1.70(4H,s).
MSm/z(M+H):437.

<Example 0240>

<0240-1>

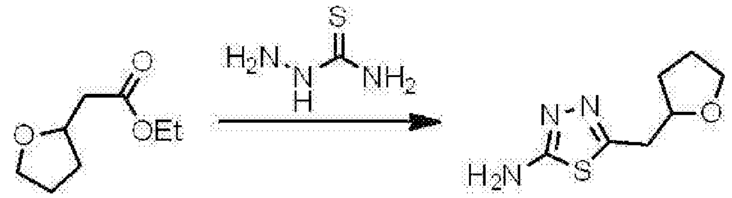

5-((Tetrahydrofuran-2-yl)methyl)-1,3,4-thiadiazole-2-amine was obtained as a white solid in the same manner as in Example 0198 except that ethyl (tetrahydrofuran-2-yl)acetate was used instead of the cyclobutanecarboxylic acid used in Example 0198.
$^1$H-NMR(DMSO-$d_6$)δ:6.98(2H,s),4.06-3.97(1H,m),3.80-3.73(1H,m),3.67-3.60(1H,m),3.04-2.89(2H,m),2.01-1.89(1H,m),1.85-1.76(2H,m),1.55-1.43(1H,m).

<0240-2>

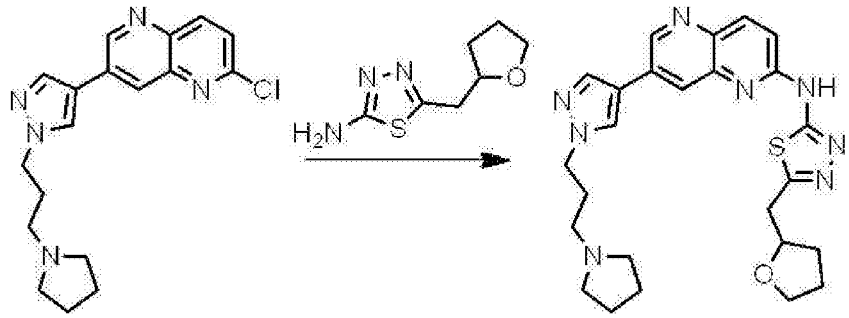

N-(7-(1-(3-(pyrrolidin-1-yl)propyl)-1H-pyrazol-4-yl)-1,5-naphthyridin-2-yl)-5-((tetrahydrofuran-2-yl)methyl)-1,3,4-thiadiazole-2-amine was obtained as a pale yellow solid in the same manner as in Example 0233 except that 5-((tetrahydrofuran-2-yl)methyl)-1,3,4-thiadiazole-2-amine was used instead of the (S)-5-(tetrahydrofuran-2-yl)-1,3,4-thiadiazole-2-amine used in Example 0233.
$^1$H-NMR(DMSO-$d_6$)δ:12.06(1H,s),9.01(1H,s),8.55(1H,s),8.19(3H,t,J=9.6Hz),7.33(1H,d,J=8.9Hz),4.23-4.15(3H,m),3.88-3.80(1H,m),3.71-3.63(1H,m),3.19-3.15(2H,m),2.45-2.37(6H,m),2.06-1.96(2H,m),1.89-1.80(2H,m),1.70-1.54(6H,m).
MSm/z(M+H):491.

<Example 0241>

<0241-1>

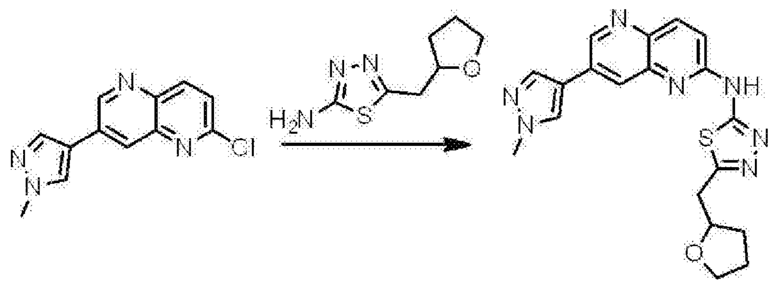

N-(7-(1-methyl-1H-pyrazol-4-yl)-1,5-naphthyridin-2-yl)-5-((tetrahydrofuran-2-yl)methyl)-1,3,4-thiadiazole-2-amine was obtained as a pale yellow solid in the same manner as in Example 0162 except that 5-((tetrahydrofuran-2-yl)methyl)-1,3,4-thiadiazole-2-amine was used instead of the 2-amino-5-tert-butyl-1,3,4-thiadiazole used in Example 0162.
$^1$H-NMR(DMSO-$d_6$)δ:12.04(1H,s),9.07(1H,d,J=2.3Hz),8.52(1H,s),8.29-8.23(2H,m),8.20(1H,s),7.40(1H,d,J=9.2Hz),4.24-4.15(1H,m),3.93(3H,s),3.88-3.80(1H,m),3.72-3.65(1H,m),3.20(2H,t,J=5.8Hz),2.07-1.97(1H,m),1.90-1.80(2H,m),1.66-1.57(1H,m).
MSm/z(M+H):394.

<Example 0242>

<0242-1>

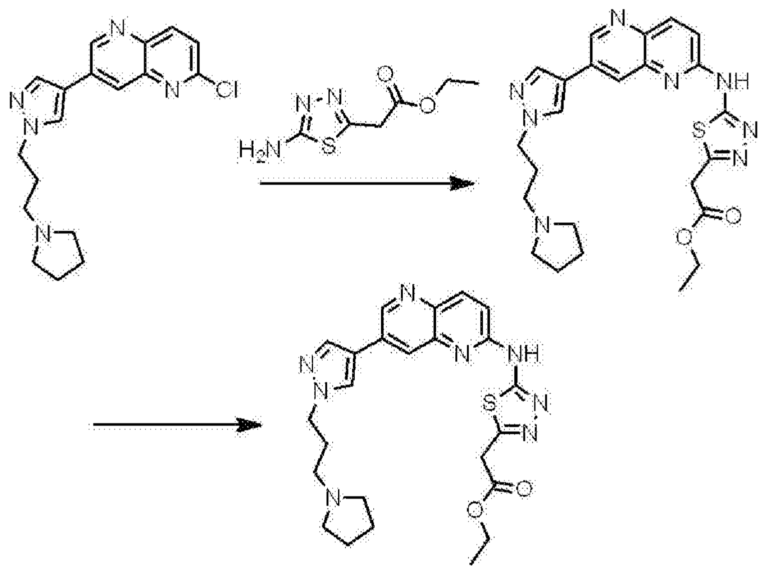

Ethyl (5-amino-1,3,4-thiadiazol-2-yl)acetate (15 mg), 2-dicyclohexylphosphino-2',4',6'-triisopropylbiphenyl (10 mg), cesium carbonate (90 mg), and tris(dibenzylideneacetone)dipalladium(0) (10 mg) were added to a solution of 2-chloro-7-(1-(3-(pyrrolidin-1-yl)propyl)-1H-pyrazol-4-yl)-1,5-naphthyridine (25 mg) in 1,4-dioxane (1 mL), followed by stirring at 150°C for 1 hour using a microwave reaction apparatus. After the reaction mixture was cooled to room temperature, a mixed solvent of chloroform-methanol was added thereto, and the resultant product was purified by silica gel column chromatography (chloroform-methanol, NH silica), and purified by preparative thin layer silica gel chromatography (chloroform-methanol, NH silica), thereby obtaining ethyl (5-((7-(1-(3-(pyrrolidin-1-yl)propyl)-1H-pyrazol-4-yl)-1,5-naphthyridin-2-yl)amino)-1,3,4-thiadiazol-2-yl)acetate (17 mg) as a yellow solid.
$^1$H-NMR(DMSO-$d_6$)δ:12.17(1H,s),9.09(1H,d,J=2.3Hz),8.58(1H,s),8.32-8.26(2H,m),8.21(1H,s),7.42(1H,d,J=8.9Hz),4.25-4.15(4H,m),3.71(3H,s),2.46-2.37(6H,m),2.06-1.96(2H,m),1.71-1.67(4H,m).
MSm/z(M+H):479.

<Example 0243>

<0243-1>

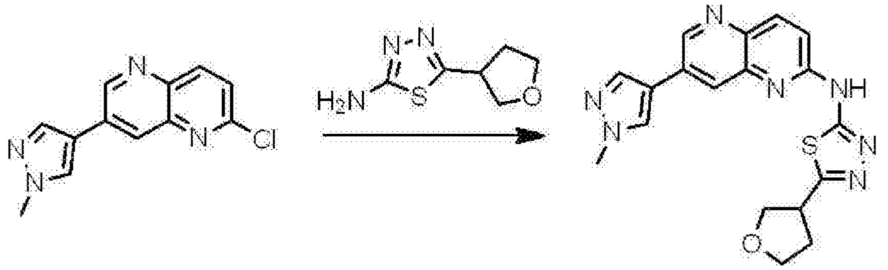

N-(7-(1-methyl-1H-pyrazol-4-yl)-1,5-naphthyridin-2-yl)-5-(tetrahydrofuran-3-yl)-1,3,4-thiadiazole-2-amine was obtained as a white solid in the same manner as in Example 0162 except that 5-(tetrahydrofuran-3-yl)-1,3,4-thiadiazole-2-amine was used instead of the 2-amino-5-tert-butyl-1,3,4-thiadiazole used in Example 0162.
$^1$H-NMR(DMSO-$d_6$)δ:12.12(1H,s),9.07(1H,d,J=2.0Hz),8.52(1H,s),8.32(1H,d,J=1.3Hz),8.26(1H,d,J=8.9Hz),8.21(1H,s),7.40(1H,d,J=8.9Hz),4.15-4.08(1H,m),4.02-3.82(7H,m),2.48-2.39(1H,m),2.31-2.19(1H,m).
MSm/z(M+H):380.

<Example 0244>

<0244-1>

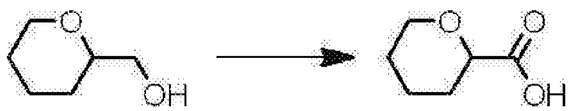

0.5 mol/L sodium hydroxide aqueous solution (20 mL) was added to tetrahydropyran-2-methanol (1.16 g), followed by stirring at room temperature for 5 minutes. Potassium permanganate (3.30 g) was added to the reaction mixture, followed by stirring at 90°C for 1 hour. Potassium permanganate (1.58 g) was added thereto, followed by stirring at 90°C for 30 minutes. After the reaction mixture was cooled to room temperature, the insolubles were filtered off using celite, and ethyl acetate was added thereto. After the aqueous layer was collected by separation, the aqueous layer was neutralized by the addition of hydrochloric acid under ice-cooling, and the water was distilled off under reduced pressure. The obtained residue was purified by silica gel column chromatography (chloroform-methanol), thereby obtaining tetrahydro-2H-pyran-2-carboxylic acid (150 mg) as colorless oily substance.
$^1$H-NMR($CDCl_3$)δ:4.16-4.08(1H,m),4.02-3.94(1H,m),3.60-3.42(2H,m),2.11-2.04(1H,m),1.97-1.90(1H,m),1.61-1.54(3H,m).

<0244-2>

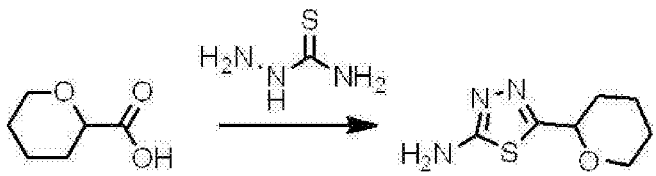

5-(Tetrahydro-2H-pyran-2-yl)-1,3,4-thiadiazole-2-amine was obtained as a white solid in the same manner as in Example 0198 except that tetrahydro-2H-pyran-2-carboxylic acid was used instead of the cyclobutanecarboxylic acid used in Example 0198.
MSm/z(M+H):186.

<0244-3>

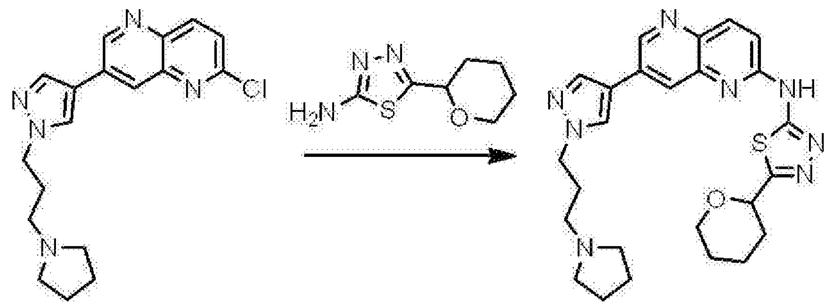

N-(7-(1-(3-(pyrrolidin-1-yl)propyl)-1H-pyrazol-4-yl)-1,5-naphthyridin-2-yl)-5-(tetrahydro-2H-pyran-2-yl)-1,3,4-thiadiazole-2-amine was obtained as a yellow solid in the same manner as in Example 0233 except that 5-(tetrahydro-2H-pyran-2-yl)-1,3,4-thiadiazole-2-amine was used instead of the (S)-5-(tetrahydrofuran-2-yl)-1,3,4-thiadiazole-2-amine used in Example 0233.
$^1$H-NMR(DMSO-$d_6$)δ:12.09(1H,s),9.02(1H,s),8.58(1H,s),8.29-8.15(3H,m),7.34(1H,d,J=8.9Hz),4.77(1H,t,J=5.0Hz),4.21(2H,t,J=6.9Hz),4.04(1H,d,J=11.6Hz),3.64(1 H,dd,J=14.4,10.4Hz),2.40(6H,t,J=7.1Hz),2.18-1.82(4H,m),1.81-1.55(8H,m).
MSm/z(M+H):491.

<Example 0245>

<0245-1>

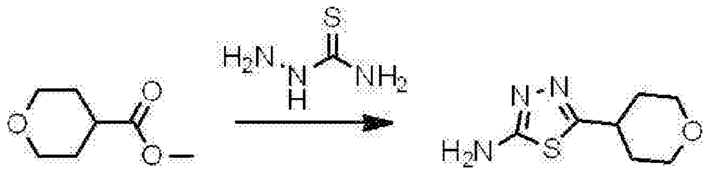

5-(Tetrahydro-2H-pyran-4-yl)-1,3,4-thiadiazole-2-amine was obtained as a white solid in the same manner as in Example 0198 except that methyl tetrahydropyran-4-carboxylate was used instead of the cyclobutanecarboxylic acid used in Example 0198.
MSm/z(M+H):186.

<0245-2>

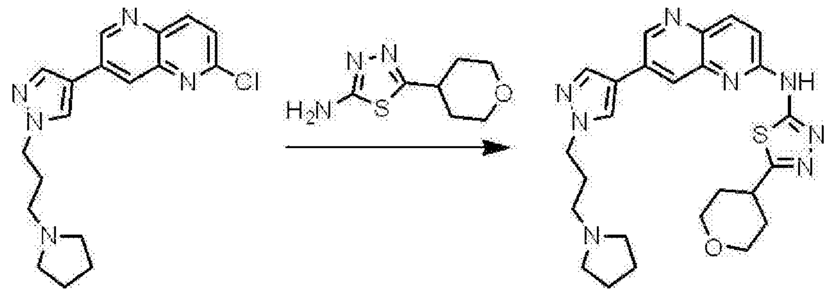

N-(7-(1-(3-(pyrrolidin-1-yl)propyl)-1H-pyrazol-4-yl)-1,5-naphthyridin-2-yl)-5-(tetrahydro-2H-pyran-4-yl)-1,3,4-thiadiazole-2-amine was obtained as a yellow solid in the same manner as in Example 0233 except that 5-(tetrahydro-2H-pyran-4-yl)-1,3,4-thiadiazole-2-amine was used instead of the (S)-5-(tetrahydrofuran-2-yl)-1,3,4-thiadiazole-2-amine used in Example 0233.
$^1$H-NMR(DMSO-$d_6$)δ:12.10(1H,s),9.09(1H,d,J=2.0Hz),8.57(1H,s),8.34(1H,d,J=1.3Hz),8.26(1H,d,J=9.6Hz),8.23(1H,s),

7.40(1H,d,J=8.9Hz),4.22(2H,t,J=7.1Hz),4.01-3.93(2H,m),3.56-3.46(2H,m),3.43-3.37(1H,m),2.47-2.36(6H,m),2.08-1.98(4H,m),1.92-1.78(2H,m),1.74-1.65(4H,m).
MSm/z(M+H):491.

<Example 0246>

<0246-1>

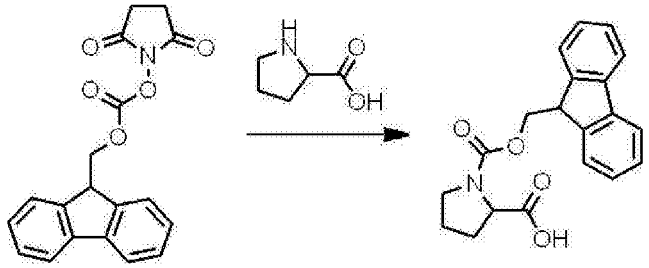

Water (5 mL) was added to pyrrolidine-2-carboxylic acid (230 mg) and sodium carbonate (500 mg), followed by stirring at room temperature for 5 minutes. A tetrahydrofuran solution (5 mL) of (9-fluorenylmethyl)succinimidyl carbonate (720 mg) was added to the reaction mixture, followed by stirring at room temperature for 2 hours. Ethyl acetate was added to the reaction mixture. The aqueous layer was neutralized by the addition of hydrochloric acid, extracted three times with ethyl acetate, and the organic layer was washed with a saturated sodium chloride aqueous solution. The organic layer was dried over anhydrous sodium sulfate, and the solvent was distilled off under reduced pressure, thereby obtaining 1-(((9H-fluoren-9-yl)methoxy)carbonyl)pyrrolidine-2-carboxylic acid (620 mg).
$^1$H-NMR(DMSO-$d_6$)δ:12.62(1H,s),7.93-7.87(2H,m),7.70-7.62(2H,m),7.45-7.29(4H,m),4.35-4.27(1H,m),4.21-4.13(2H,m),3.47-3.36(2H,m),2.59(1H,s),2.40-2.11(1H,m),1.92-1.80(3H,m).

<0246-2>

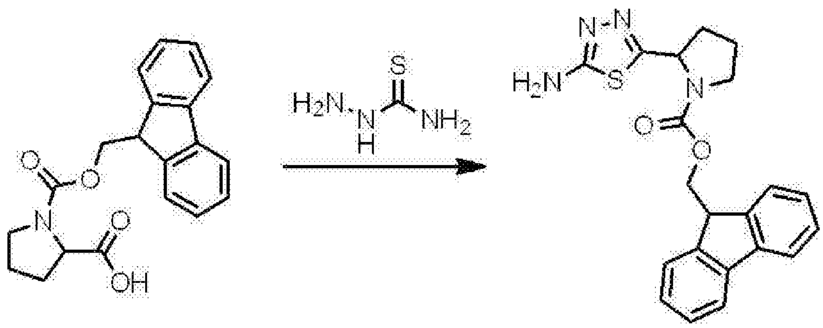

Phosphorus oxychloride (5 mL) was added to 1-(((9H-fluoren-9-yl)methoxy)carbonyl)pyrrolidine-2-carboxylic acid (505 mg) and thiosemicarbazide (175 mg), followed by stirring at 80°C for 2 hours. The reaction mixture was cooled to room temperature, and added dropwise to water. The resultant product was neutralized by the addition of a sodium hydroxide aqueous solution under ice-cooling, and ethyl acetate was added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate, NH silica), thereby obtaining (9H-fluoren-9-yl)methyl 2-(5-amino-1,3,4-thiadiazol-2-yl)pyrrolidine-1-carboxylate (238 mg).
MSm/z(M+H):393.

<0246-3>

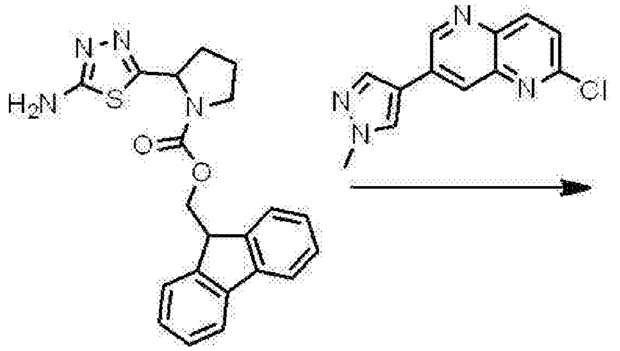

(9H-fluoren-9-yl)methyl 2-(5-amino-1,3,4-thiadiazol-2-yl)pyrrolidine-1-carboxylate (115 mg), 2-dicyclohexylphosphino-2',4',6'-triisopropylbiphenyl (20 mg), cesium carbonate (150 mg), and tris(dibenzylideneacetone)dipalladium(0) (20 mg) were added to a solution of 2-chloro-7-(1-methyl-1H-pyrazol-4-yl)-1,5-naphthyridine (75 mg) in 1,4-dioxane (1 mL), followed by stirring at 100°C for 3 hours in a nitrogen atmosphere. The reaction mixture was cooled to room temperature, and chloroform and methanol were added thereto. The obtained solution was purified by silica gel column chromatography (chloroform-methanol, NH silica), thereby obtaining N-(7-(1-methyl-1H-pyrazol-4-yl)-1,5-naphthyridin-2-yl)-5-(pyrrolidin-2-yl)-1,3,4-thiadiazole-2-amine (64 mg) as a yellow solid.
$^1$H-NMR(DMSO-$d_6$)δ:11.93(1H,s),9.06(1H,d,J=2.0Hz),8.54(1H,s),8.23(3H,dd,J=8.3,5.9Hz),7.40(1H,d,J=8.9Hz),4.54-4.47(1H,m),3.92(3H,s),3.02-2.90(2H,m),2.28-2.13(1H,m),2.01-1.90(1H,m),1.86-1.72(2H,m).
MSm/z(M+H):379.

<Example 0247>

<0247-1>

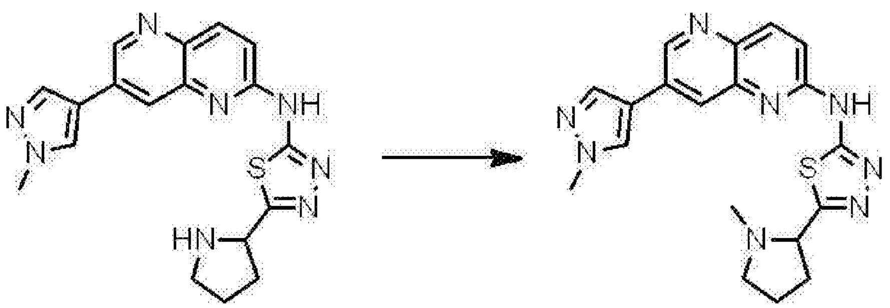

A20% formaldehyde solution (10 μL) and sodium triacetoxyborohydride (10 mg) were added to a solution of N-(7-(1-methyl-1H-pyrazol-4-yl)-1,5-naphthyridin-2-yl)-5-(pyrrolidin-2-yl)-1,3,4-thiadiazole-2-amine (17 mg) in methanol (1 mL), followed by stirring at room temperature for 30 minutes. A20% formaldehyde solution (10 μL) and sodium triacetoxyborohydride (5 mg) were added thereto, followed by stirring at room temperature for 30 minutes. The solvent was distilled off under reduced pressure, and water and ethyl acetate were added thereto. The organic layer was collected by separation, and dried over anhydrous sodium sulfate, and the solvent was distilled off under reduced pressure. The obtained residue was purified by silica gel column chromatography (chloroform-methanol, NH silica), thereby obtaining N-(7-(1-methyl-1H-pyrazol-4-yl)-1,5-naphthyridin-2-yl)-5-(1-methylpyrrolidin-2-yl)-1,3,4-thiadiazole-2-amine (5 mg) as a yellow solid.
$^1$H-NMR(DMSO-$d_6$)δ:12.06(1H,s),9.07(1H,d,J=2.0Hz),8.56(1H,s),8.25(3H,t,J=5.8Hz),7.41(1H,d,J=8.9Hz),3.93(3H,s),3 .67(1H,t,J=7.6Hz),2.38-2.25(5H,m),1.99-1.81(4H,m).

MSm/z(M+H):393.

<Example 0248>

<0248-1>

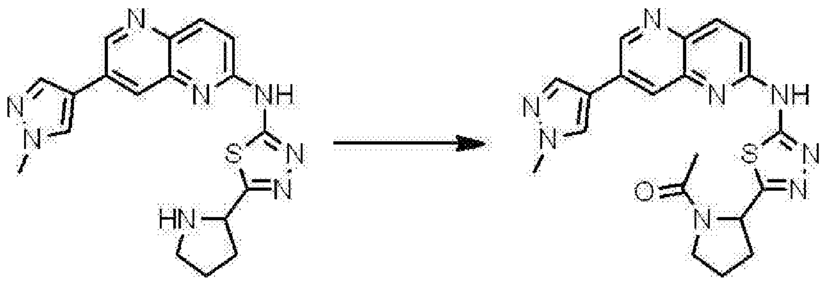

Pyridine (40 μL) and acetyl chloride (7 μL) were added to a solution of N-(7-(1-methyl-1H-pyrazol-4-yl)-1,5-naphthyridin-2-yl)-5-(pyrrolidin-2-yl)-1,3,4-thiadiazole-2-amine (15 mg) in dichloromethane (3 mL), followed by stirring at room temperature for 5 minutes. The reaction mixture was purified by silica gel column chromatography (chloroform-methanol, NH silica), thereby obtaining 1-(2-(5-((7-(1-methyl-1H-pyrazol-4-yl)-1,5-naphthyridin-2-yl)amino)-1,3,4-thiadiazol-2-yl)pyrrolidin-1-yl)ethanone (10 mg) as a yellow solid.
$^1$H-NMR(DMSO-$d_6$)δ:12.11(1H,s),9.06(1H,d,J=2.0Hz),8.50(1H,s),8.26-8.19(3H,m),7.40(1H,d,J=8.9Hz),5.50-5.31(1H,m),3.93(3H,s),3.78-3.46(2H,m),2.32-1.95(7H,m).
MSm/z(M+H):421.

<Example 0249>

<0249-1>

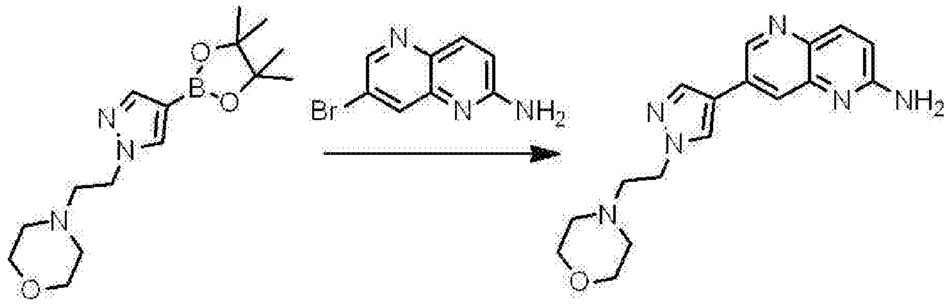

A mixture of 7-bromo-1,5-naphthyridine-2-amine (50 mg), sodium carbonate (47 mg), 1-(2-morpholinoethyl)-1H-pyrazole-4-boronic acid pinacol ester (137 mg), bis(tri-tert-butylphosphine)palladium(0) (11 mg), 1,4-dioxane (1 mL), and water (0.1 mL) was stirred at 140°C for 1 hour using a microwave reaction apparatus. The reaction mixture was cooled to room temperature, and purified by silica gel column chromatography (chloroform-methanol, NH silica), thereby obtaining 7-(1-(2-morpholinoethyl)-1H-pyrazol-4-yl)-1,5-naphthyridine-2-amine (49 mg) as a white solid.
$^1$H-NMR(DMSO-$d_6$)δ:8.78(1H,d,J=2.0Hz),8.41(1H,s),8.08(1H,s),7.89(2H,dd,J=7.1,5.8Hz),6.90(1H,d,J=8.9Hz),6.65(2H ,s),4.27(2H,t,J=6.6Hz),3.59-3.51(4H,m),2.75(2H,t,J=6.6Hz),2.43(4H,t,J=4.5Hz).

<0249-2>

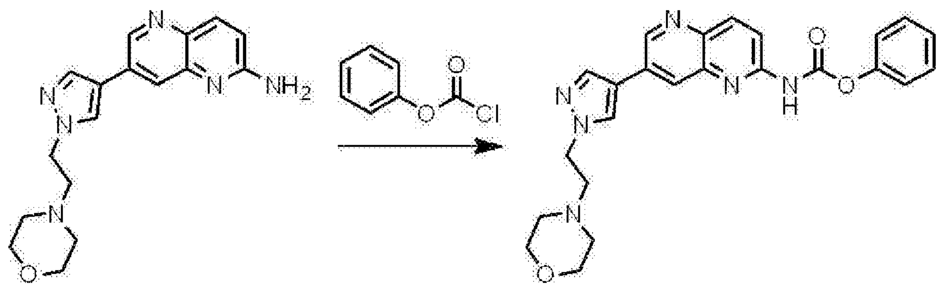

Phenyl chlorocarbonate (60 μL) was added to a solution of 7-(1-(2-morpholinoethyl)-1H-pyrazol-4-yl)-1,5-naphthyridine-2-amine (168 mg) in pyridine (10 mL), and candy-like lumps were dissolved using an ultrasonic cleaning machine. The reaction liquid was stirred at room temperature for 1 hour, and phenyl chlorocarbonate (20 μL) was added thereto, followed by stirring at room temperature for 1 hour. The solvent was distilled off under reduced pressure, and the obtained residue was purified by silica gel column chromatography (chloroform-methanol), thereby obtaining phenyl (7-(1-(2-morpholinoethyl)-1H-pyrazol-4-yl)-1,5-naphthyridin-2-yl)carbamate (84 mg).
$^1$H-NMR(DMSO-$d_6$)δ:11.21(1H,s),9.18(1H,d,J=2.0Hz),8.57(1H,s),8.38(1H,d,J=9.2Hz),8.27(1H,d,J=1.7Hz),8.23-8.18(2H,m), 7.49-7.42(2H,m), 7.33-7.25(3H,m),4.30(2H,t,J=6.4Hz),3.56(4H,t,J=4.6Hz),2.78(2H,t,J=6.6Hz),2.44(4H,t,J=4.5Hz).

<0249-3>

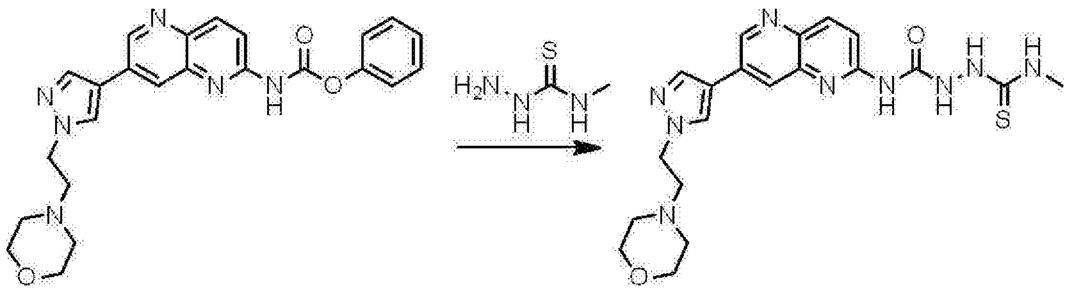

4-Methylthiosemicarbazide (7 mg) was added to a solution of phenyl (7-(1-(2-morpholinoethyl)-1H-pyrazol-4-yl)-1,5-naphthyridin-2-yl)carbamate (20 mg) in 1,4-dioxane (1.2 mL), followed by stirring at 70°C for 1 hour. The reaction mixture was cooled to room temperature, and the solvent was distilled off under reduced pressure. The obtained residue was washed with ethyl acetate, thereby obtaining 2-(methylcarbamothioyl)-N-(7-(1-(2-morpholinoethyl)-1H-pyrazol-4-yl)-1,5-naphthyridin-2-yl)hydrazinecarboxamide (20 mg).
MSm/z(M+H):456.

<0249-4>

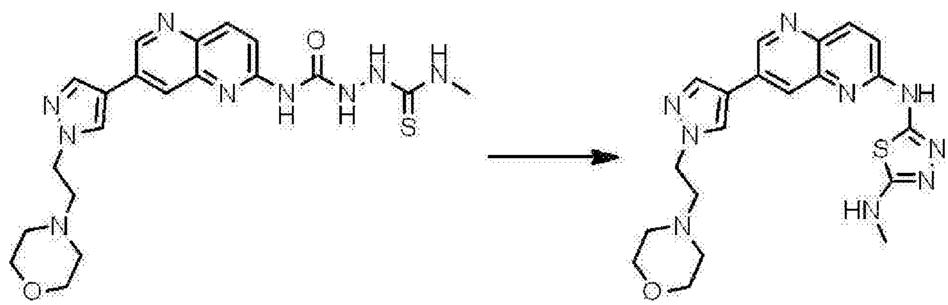

Phosphorus oxychloride (500 μL) was added to 2-(methylcarbamothioyl)-N-(7-(1-(2-morpholinoethyl)-1H-pyrazol-4-yl)-1,5-naphthyridin-2-yl)hydrazinecarboxamide (7 mg), followed by stirring at 80°C for 1 hour. The reaction mixture was cooled to room temperature, and added dropwise to water. The resultant product was neutralized by the addition of a sodium hydroxide aqueous solution under ice-cooling, and chloroform was added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (chloroform-methanol, NH silica), thereby obtaining $N^2$-methyl-$N^5$-(7-(1-(2-morpholinoethyl)-1H-pyrazol-4-yl)-1,5-naphthyridin-2-yl)-1,3,4-thiadiazole-2,5-diamine (1.4 mg) as a yellow solid.
$^1$H-NMR(DMSO-$d_6$)δ:11.52(1H,s),8.97(1H,s),8.51(1H,s),8.11(3H,t,J=7.3Hz),7.26(1H,d,J=9.2Hz),7.06(1H,s),4.29(2H,t,J =6.6Hz),3.56(4H,t,J=4.6Hz),2.87(3H,d,J=4.6Hz),2.78(2H,t,J=6.6Hz),2.44(4H,t,J=4.5Hz).
MSm/z(M+H):438.

<Example 0250>

<0250-1>

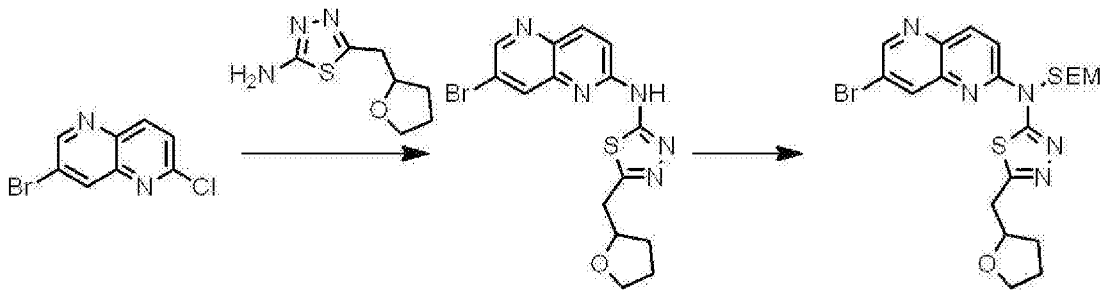

N-(7-bromo-1,5-naphthyridin-2-yl)-5-((tetrahydrofuran-2-yl)methyl)-N-((2-(trimethylsilyl)methoxy)methyl)-1,3,4-thiadiazole-2-amine was obtained as brown oily substance in the same manner as in Example 0175 except that 5-((tetrahydrofuran-2-yl)methyl)-1,3,4-thiadiazole-2-amine was used instead of the 2-amino-5-cyclopentyl-1,3,4-thiadiazole used in Example 0175.
MSm/z(M+H):522,524.

<0250-2>

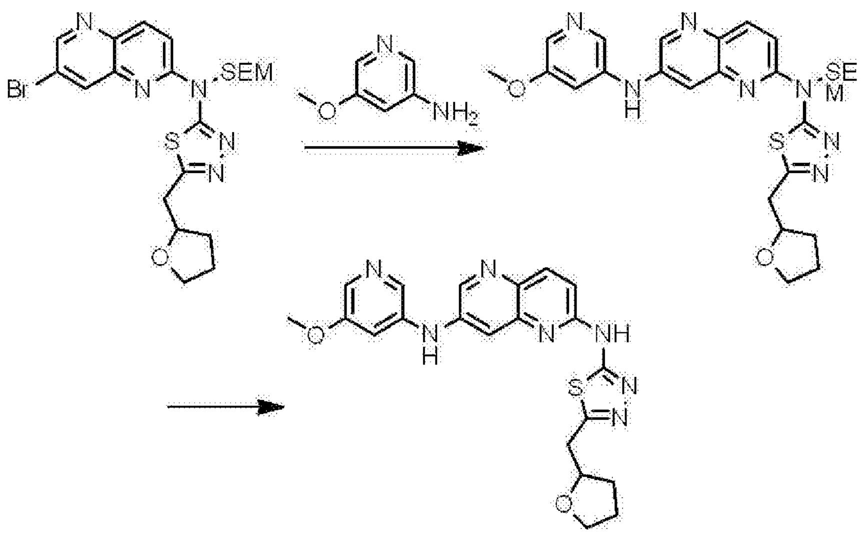

3-Amino-5-methoxypyridine (14 mg), 2-dicyclohexylphosphino-2',4',6'-triisopropylbiphenyl (5 mg), cesium carbonate (45 mg), and tris(dibenzylideneacetone)dipalladium(0) (5 mg) were added to a solution of N-(7-bromo-1,5-naphthyridin-2-yl)-5-((tetrahydrofuran-2-yl)methyl)-N-((2-(trimethylsilyl)methoxy)methyl)-1,3,4-thiadiazole-2-amine (58 mg) in 1,4-dioxane (2 mL), followed by stirring at 100°C in a nitrogen atmosphere overnight. The reaction mixture was cooled to room temperature, and the solvent was distilled off under reduced pressure, thereby obtaining $N^7$-(5-methoxypyridin-3-yl)-$N^2$-(5-((tetrahydrofuran-2-yl)methyl)-1,3,4-thiadiazol-2-yl)-$N^2$-((2-(trimethylsilyl)ethoxy)methyl)-1,5-naphthyridine-2,7-diamine.

A 4 mol/L hydrogen chloride/1,4-dioxane solution (2 mL) was added to the obtained N-(5-methoxypyridin-3-yl)-$N^2$-(5-((tetrahydrofuran-2-yl)methyl)-1,3,4-thiadiazol-2-yl)-$N^2$-((2-(trimethylsilyl)ethoxy)methyl)-1,5-naphthyridine-2,7-diamine, followed by stirring at room temperature for 1 hour. The solvent was distilled off under reduced pressure, the resultant product was neutralized with a saturated sodium hydrogen carbonate aqueous solution, and a chloroform-methanol solution was added thereto. The organic layer was collected by separation, the solvent was distilled off under reduced pressure, and the obtained residue was purified by silica gel column chromatography (ethyl acetate-methanol), thereby obtaining $N^7$-(5-methoxypyridin-3-yl)-$N^2$-(5-((tetrahydrofuran-2-yl)methyl)-1,3,4-thiadiazol-2-yl)-1,5-naphthyridine-2,7-diamine (7 mg) as a yellow solid.
$^1$H-NMR(DMSO-$d_6$)δ:11.92(1H,s),11.92-1.49(1H,m),9.11(1H,s),8.59(1H,d,J=2.6Hz),8.17-8.13(2H,m),7.98(1H,d,J=2.6Hz),7.64(1H,d,J=2.3Hz),7.27(1H,t,J=2.3Hz),7.22(1H,d,J=8.9Hz),4.18-4.10(1H,m),3.85(3H,s),3.84-3.78(1H,m),3.72-3.64(1H,m),3.22-3.13(1H,m),2.06-1.94(1H,m),1.90-1.78(2H,m),1.62-1.49(1H,m).
MSm/z(M+H):436.

<Example 0251>

<0251-1>

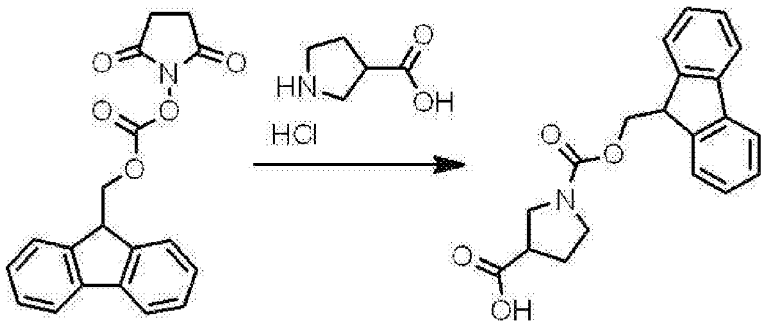


1-(((9H- fluoren-9-yl)methoxy)carbonyl)pyrrolidine-3-carboxylic acid was obtained in the same manner as in Example 0246 except that γ-DL-proline hydrochloride was used instead of the pyrrolidine-2-carboxylic acid used in Example 0246.
$^1$H-NMR(DMSO-$d_6$)δ:12.52(1H,s),7.96-7.87(2H,m),7.67-7.60(2H,m),7.47-7.27(4H,m),4.44-4.28(3H,m),3.51-3.43(2H,m),3.13-2.99(1H,m),2.59(2H,s),2.18-1.91(2H,m).

<0251-2>

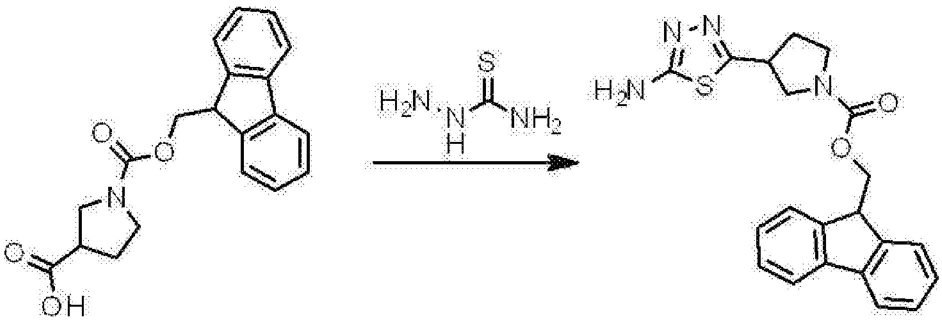


(9H-fluoren-9-yl)methyl 3-(5-amino-1,3,4-thiadiazol-2-yl)pyrrolidine-1-carboxylate was obtained in the same manner as in Example 0246 except that 1-(((9H-fluoren-9-yl)methoxy)carbonyl)pyrrolidine-3-carboxylic acid was used instead of the 1-(((9H-fluoren-9-yl)methoxy)carbonyl)pyrrolidine-2-carboxylic acid used in Example 0246.
MSm/z(M+H):393.

<0251-3>

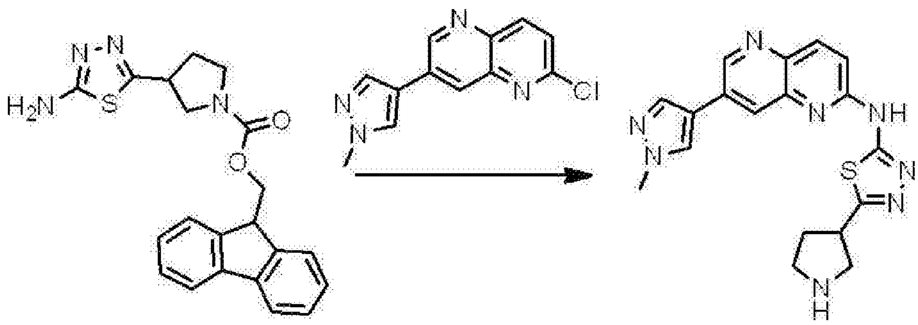

N-(7-(1-methyl-1H-pyrazol-4-yl)-1,5-naphthyridin-2-yl)-5-(pyrrolidin-3-yl)-1,3,4-thiadiazole-2-amine was obtained as a yellow solid in the same manner as in Example 0246 except that (9H-fluoren-9-yl)methyl 3-(5-amino-1,3,4-thiadiazol-2-yl)pyrrolidine-1-carboxylate was used instead of the (9H-fluoren-9-yl)methyl 2-(5-amino-1,3,4-thiadiazol-2-yl)pyrrolidine-1-carboxylate used in Example 0246.
$^1$H-NMR(DMSO-$d_6$)δ:9.08(1H,d,J=2.0Hz),8.52(1H,s),8.33(1H,d,J=1.7Hz),8.26(2H,d,J=7.9Hz),8.17(1H,t,J=12.4Hz),7.40(1H,d,J=8.9Hz),3.93(3H,s),3.84-3.75(1H,m),3.51-3.42(1H,m),3.27-3.08(3H,m),2.36(1H,td,J=13.3,7.6Hz),2.12(1H,m).
MSm/z(M+H):379.

<Example 0252>

<0252-1>

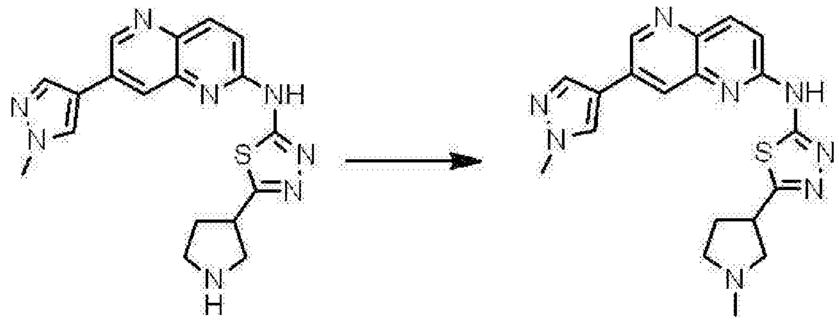

N-(7-(1-methyl-1H-pyrazol-4-yl)-1,5-naphthyridin-2-yl)-5-(1-methylpyrrolidin-3-yl)-1,3,4-thiadiazole-2-amine was obtained as a yellow solid in the same manner as in Example 0247 except that (N-(7-(1-methyl-1H-pyrazol-4-yl)-1,5-naphthyridin-2-yl)-5-(pyrrolidin-3-yl)-1,3,4-thiadiazole-2-amine was used instead of the N-(7-(1-methyl-1H-pyrazol-4-yl)-1,5-naphthyridin-2-yl)-5-(pyrrolidin-2-yl)-1,3,4-thiadiazole-2-amine used in Example 0247.
$^1$H-NMR(DMSO-$d_6$)δ:12.01(1H,s),9.07(1H,d,J=2.0Hz),8.52(1H,s),8.29-8.21(3H,m),7.40(1H,d,J=9.2Hz),3.93(3H,s),3.86-3.76(2H,m),3.01-2.93(1H,m),2.81-2.58(2H,m),2.43-2.24(4H,m),2.23-2.09(1H,m).
MSm/z(M+H):393.

<Example 0253>

<0253-1>

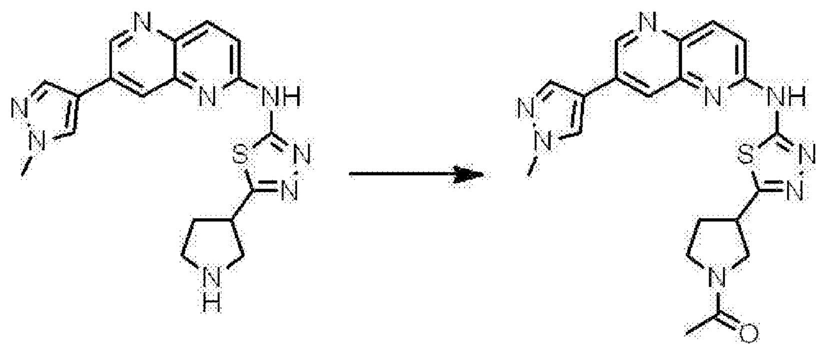

1-(3-(5-((7-(1-Methyl-1H-pyrazol-4-yl)-1,5-naphthyridin-2-yl)amino)-1,3,4-thiadiazol-2-yl)pyrrolidin-1-yl)ethanone was obtained as a yellow solid in the same manner as in Example 0248 except that (N-(7-(1-methyl-1H-pyrazol-4-yl)-1,5-naphthyridin-2-yl)-5-(pyrrolidin-3-yl)-1,3,4-thiadiazole-2-amine was used instead of the N-(7-(1-methyl-1H-pyrazol-4-yl)-1,5-naphthyridin-2-yl)-5-(pyrrolidin-2-yl)-1,3,4-thiadiazole-2-amine used in Example 0248.
$^1$H-NMR(DMSO-$d_6$)δ:9.09-9.06(1H,m),8.52(1H,s),8.33-8.31(1H,m),8.26(1H,d,J=9.2Hz),8.21(1H,s),7.40(1H,d,J=8.9Hz),4.01-3.95(1H,m),3.93(3H,s),3.89-3.75(2H,m),3.73-3.50(2H,m),3.06-2.92(1H,m),2.47-2.09(2H,m),2.01(2H,d,J=5.0Hz).
MSm/z(M+H):421.

<Example 0254>

<0254-1>

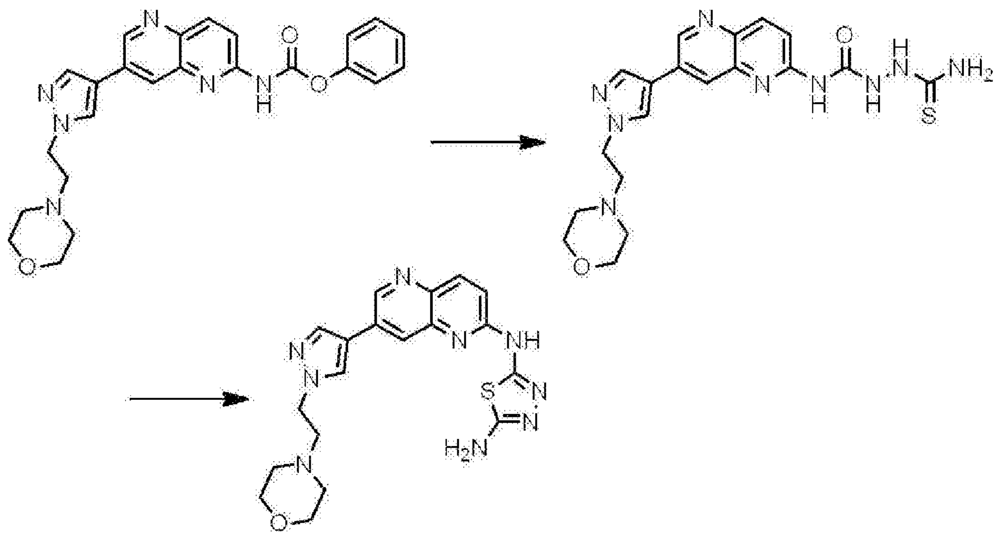

Thiosemicarbazide (7 mg) was added to a solution of phenyl ((7-(1-(2-morpholinoethyl)-1H-pyrazol-4-yl)-1,5-naphthyridin-2-yl)carbamate (43 mg) in 1,4-dioxane (1 mL), followed by stirring at 90°C for 3 hours. The reaction mixture was cooled to room temperature, and the solvent was distilled off under reduced pressure. The obtained residue was washed with ethyl acetate, thereby obtaining 2-carbamothioyl-N-(7-(1-(2-morpholinoethyl)-1H-pyrazol-4-yl)-1,5-naphthyridin-2-yl)hydrazinecarboxamide. Phosphorus oxychloride (1 mL) was added to the obtained 2-carbamothioyl-N-(7-(1-(2-morpholinoethyl)-1H-pyrazol-4-yl)-1,5-naphthyridin-2-yl)hydrazinecarboxamide, followed by stirring at 80°C for 1 hour. The reaction mixture was cooled to room temperature, and added dropwise to water. The resultant product was neutralized by the addition of a sodium hydroxide aqueous solution under ice-cooling, and chloroform was added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (chloroform-methanol, NH silica), thereby obtaining $N^2$-(7-(1-(2-morpholinoethyl)-1H-pyrazol-4-yl)-1,5-naphthyridin-2-yl)-1,3,4-thiadiazole-2,5-diamine (2 mg) as a yellow solid.
$^1$H-NMR(DMSO-$d_6$)δ:11.42(1H,s),9.00(1H,d,J=1.7Hz),8.51(1H,s),8.18-8.09(3H,m),7.31(1H,d,J=9.2Hz),6.61(2H,s),4.33-4.26(2H,m),3.59-3.53(4H,m),2.82-2.75(2H,m),2.47-2.41(4H,m).
MSm/z(M+H):424.

<Example 0255>

<0255-1>

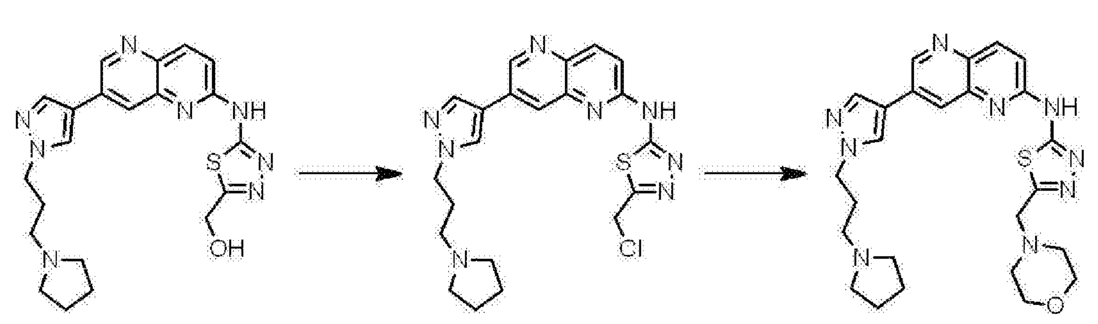

Methanesulfonyl chloride (20 μL) was added to a solution of (5-((7-(1-(3-(pyrrolidin-1-yl)propyl)-1H-pyrazol-4-yl)-1,5-naphthyridin-2-yl)amino)-1,3,4-thiadiazol-2-yl)methanol and pyridine (200 μL) in N,N-dimethylformamide (500 μL) under ice-cooling, followed by stirring at 0°C for 30 minutes. Morpholine (50 μL) was added to the reaction mixture, followed by stirring at 0°C for 1 hour. Morpholine (50 μL) was added thereto, followed by stirring at room temperature for 30 minutes. Ethyl acetate and water were added to the reaction mixture, and the solvent was distilled off under reduced pressure. The obtained residue was purified by silica gel column chromatography (chloroform-methanol, NH

silica), thereby obtaining 5-(morpholinomethyl)-N-(7-(1-(3-(pyrrolidin-1-yl)propyl)-1H-pyrazol-4-yl)-1,5-naphthyridin-2-yl)-1,3,4-thiadiazole-2-amine (4.5 mg) as a yellow solid.
$^1$H-NMR(DMSO-$d_6$)$\delta$:12.12(1H,s),9.09(1H,d,J=2.0Hz),8.59(1H,s),8.31(1H,d,J=2.0Hz),8.27(1H,d,J=9.2Hz),8.23(1H,s),7.41(1H,d,J=8.9Hz),4.22(2H,t,J=6.9Hz),3.89(2H,s),3.66-3.58(4H,m),3.44-3.38(2H,m),2.46-2.39(8H,m),2.07-1.98(2H,m),1.70(4H,s).
MSm/z(M+H):506.

<Example 0256>

<0256-1>

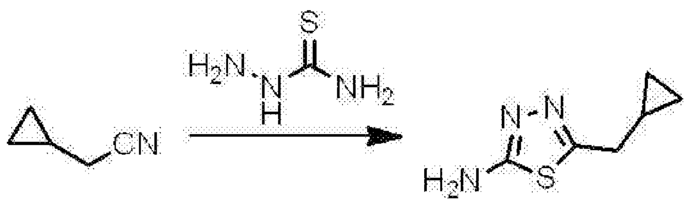

A solution of cyclopropylacetonitrile (486 mg) and thiosemicarbazide (455 mg) in trifluoroacetic acid (5 mL) was stirred at 65°C for 2 hours. The reaction mixture was cooled to room temperature, neutralized by the addition of a sodium hydroxide aqueous solution, and ethyl acetate was added thereto. The organic layer was collected by separation, washed with a saturated sodium chloride aqueous solution, and dried over anhydrous sodium sulfate, and the solvent was distilled off under reduced pressure. The obtained residue was washed with a hexane-ethyl acetate solution, thereby obtaining 5-(cyclopropylmethyl)-1,3,4-thiadiazole-2-amine (536 mg).
MSm/z(M+H):156.

<0256-2>

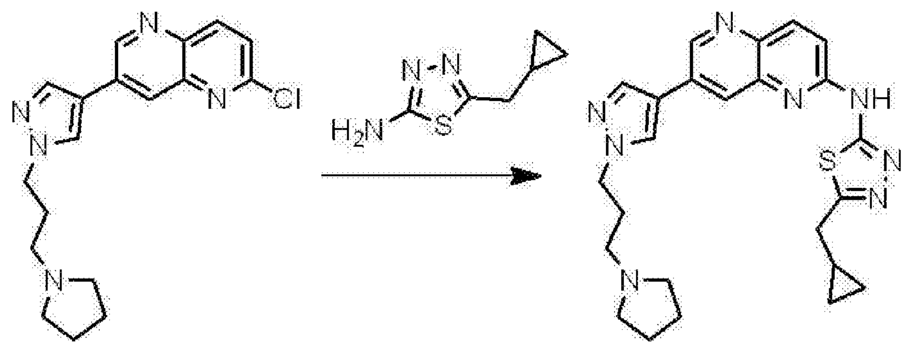

5-(Cyclopropylmethyl)-N-(7-(1-(3-(pyrrolidin-1-yl)propyl)-1H-pyrazol-4-yl)-1,5-naphthyridin-2-yl)-1,3,4-thiadiazole-2-amine was obtained as a yellow solid in the same manner as in Example 0233 except that 5-(cyclopropylmethyl)-1,3,4-thiadiazole-2-amine was used instead of the (S)-5-(tetrahydrofuran-2-yl)-1,3,4-thiadiazole-2-amine used in Example 0233.
$^1$H-NMR(DMSO-$d_6$)$\delta$:12.07(1H,s),9.08(1H,d,J=2.0Hz),8.56(1H,s),8.30(1H,d,J=1.7Hz),8.26(1H,d,J=9.2Hz),8.22(1H,s),7.40(1H,d,J=8.9Hz),4.22(2H,t,J=6.9Hz),2.93(2H,d,J=6.9Hz),2.48-2.40(5H,m),2.09-1.97(2H,m),1.75-1.66(4H,m),1.25-1.12(1H,m),1.24-1.12(1H,m),0.64-0.59(2H,m),0.40-0.33(2H,m).
MSm/z(M+H):461.

<Example 0257>

<0257-1>

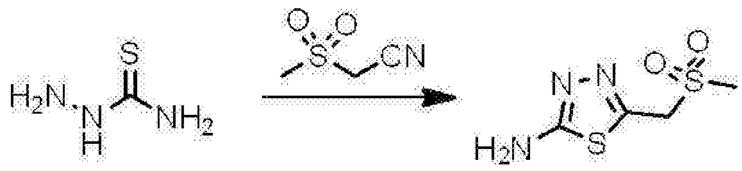

5-((Methylsulfonyl)methyl)-1,3,4-thiadiazole-2-amine was obtained in the same manner as in Example 0256 except that methylsulfonylacetonitrile was used instead of the cyclopropylacetonitrile used in Example 0256. MSm/z(M+H):194.

<0257-2>

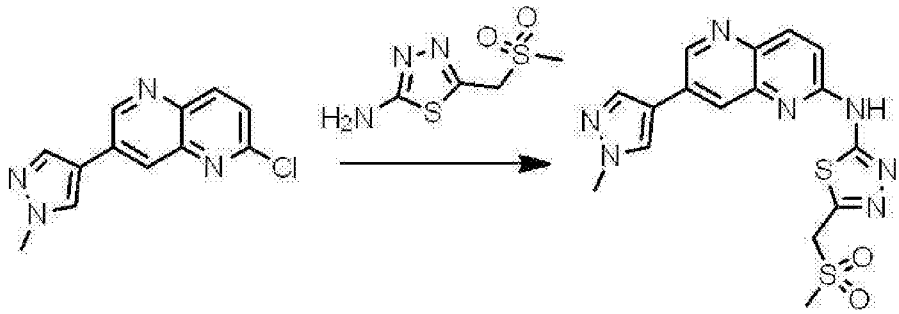

N-(7-(1-methyl-1H-pyrazol-4-yl)-1,5-naphthyridin-2-yl)-5-((methylsulfonyl)methyl)-1,3,4-thiadiazole-2-amine was obtained as a yellow solid in the same manner as in Example 0246 except that 5-((methylsulfonyl)methyl)-1,3,4-thiadiazole-2-amine was used instead of the (9H-fluoren-9-yl)methyl 2-(5-amino-1,3,4-thiadiazol-2-yl)pyrrolidine-1-carboxylate used in Example 0246.
$^1$H-NMR(DMSO-$d_6$)$\delta$:9.09(1H,d,J=2.0Hz),8.51(1H,s),8.37(1H,s),8.30(1H,d,J=8.9Hz),8.25(1H,d,J=1.7Hz),8.18(1H,s),7.45(1H,d,J=8.9Hz),5.08(2H,s),3.93(3H,s),3.13(3H,s).
MSm/z(M+H):402.

<Example 0258>

<0258-1>

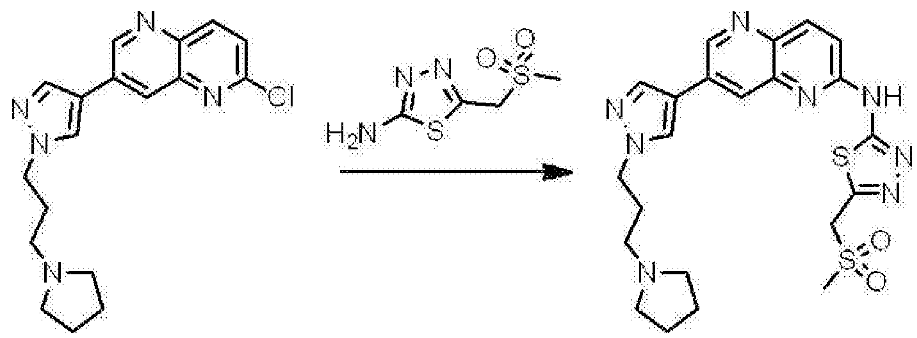

5-((Methylsulfonyl)methyl)-N-(7-(1-(3-(pyrrolidin-1-yl)propyl)-1H-pyrazol-4-yl)-1,5-naphthyridin-2-yl)-1,3,4-thiadiazole-2-amine was obtained as a pale yellow solid in the same manner as in Example 0233 except that 5-((methylsulfonyl)methyl)-1,3,4-thiadiazole-2-amine was used instead of the (S)-5-(tetrahydrofuran-2-yl)-1,3,4-thiadiazole-2-amine used in Example 0233.
$^1$H-NMR(DMSO-$d_6$)δ:9.08(1H,d,J=2.0Hz),8.56(1H,s),8.29-8.22(2H,m),8.18(1H,s),7.42(1H,d,J=8.9Hz),5.06(2H,s),4.21(2H,t,J=7.1Hz),3.58-3.39(2H,m),3.13(3H,s),2.44-2.37(5H,m),2.06-1.97(1H,m),1.71-1.67(4H,m),1.23(1H,s).
MSm/z(M+H):499.

<Example 0259>

<0259-1>

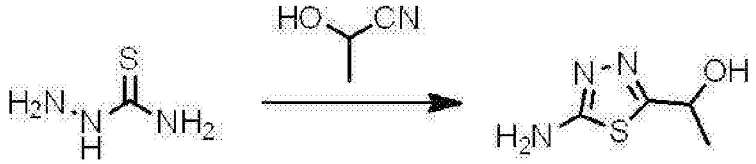

1-(5-Amino-1,3,4-thiadiazol-2-yl)ethanol was obtained in the same manner as in Example 0256 except that acetone cyanhydrin was used instead of the cyclopropylacetonitrile used in Example 0256.
MSm/z(M+H):146.

<0259-2>

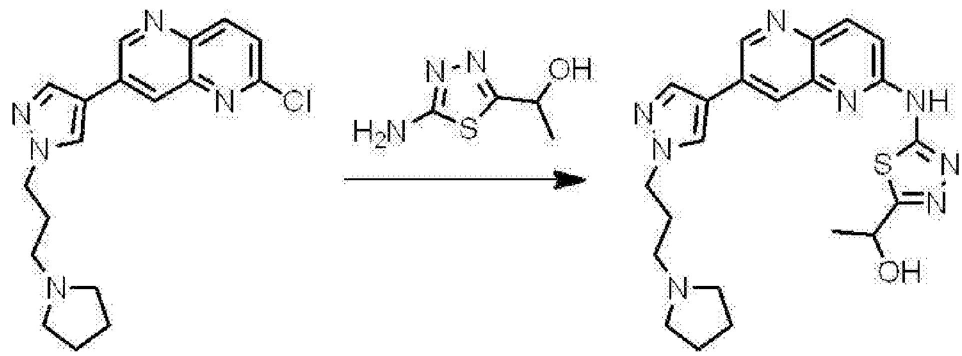

1-(5-((7-(1-(3-(Pyrrolidin-1-yl)propyl)-1H-pyrazol-4-yl)-1,5-naphthyridin-2-yl)amino)-1,3,4-thiadiazol-2-yl)ethanol was obtained as a brown solid in the same manner as in Example 0233 except that 1-(5-amino-1,3,4-thiadiazol-2-yl)ethanol was used instead of the (S)-5-(tetrahydrofuran-2-yl)-1,3,4-thiadiazole-2-amine used in Example 0233.
$^1$H-NMR(DMSO-$d_6$)δ:12.04(1H,s),9.09(1H,d,J=2.0Hz),8.60(1H,s),8.25(3H,dd,J=12.2,3.3Hz),7.41(1H,d,J=8.9Hz),6.14( 1H,d,J=5.0Hz),5.12-5.04(1H,m),4.25-4.16(2H,m),2.46-2.37(6H,m),2.06-1.96(2H,m),1.71-1.67(4H,m), 1.55(3H,d,J=6.6Hz).
MSm/z(M+H):451.

<Example 0260>

<0260-1>

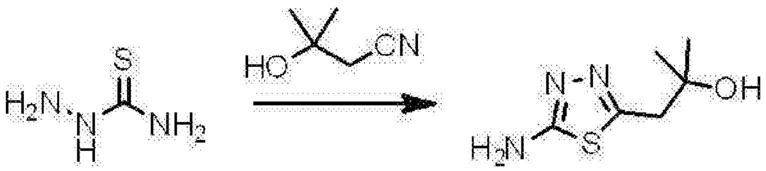

1-(5-Amino-1,3,4-thiadiazol-2-yl)-2-methylpropan-2-ol was obtained in the same manner as in Example 0256 except that 3-hydroxy-3-methylbutyronitrile was used instead of the cyclopropylacetonitrile used in Example 0256. MSm/z(M+H):174.

<0260-2>

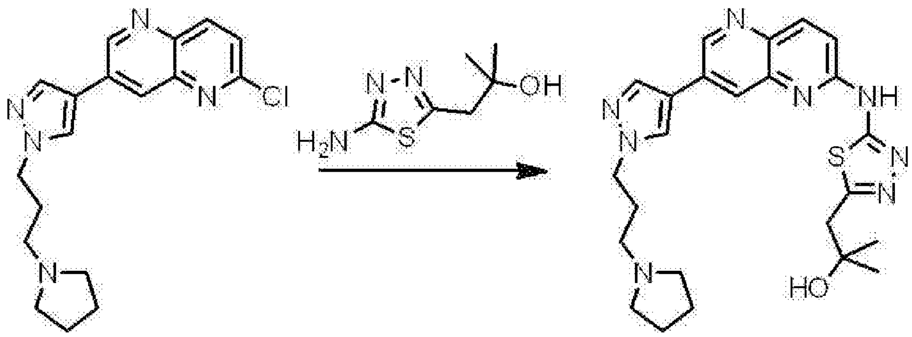

2-Methyl-1-(5-((7-(1-(3-(pyrrolidin-1-yl)propyl)-1H-pyrazol-4-yl)-1,5-naphthyridin-2-yl)amino)-1,3,4-thiadiazol-2-yl)propan-2-ol was obtained as a yellow solid in the same manner as in Example 0233 except that 1-(5-amino-1,3,4-thiadiazol-2-yl)-2-methylpropan-2-ol was used instead of the (S)-5-(tetrahydrofuran-2-yl)-1,3,4-thiadiazole-2-amine used in Example 0233.
$^1$H-NMR(DMSO-$d_6$)$\delta$:11.98(1H,s),9.07(1H,d,J=2.3Hz),8.55(1H,s),8.25(1H,d,J=9.2Hz),8.19(1H,d,J=1.7Hz),8.17(1H,s),7 .41(1H,d,J=8.9 Hz),4.91(1H,s),4.21(2H,t,J=6.9Hz),3.08(2H,s),2.45-2.37(6H,m),2.06-1.96(2H,m),1.71-1.67(4H,m),1.20(6H,s).
MSm/z(M+H):479.

<Example 0261>

<0261-1>

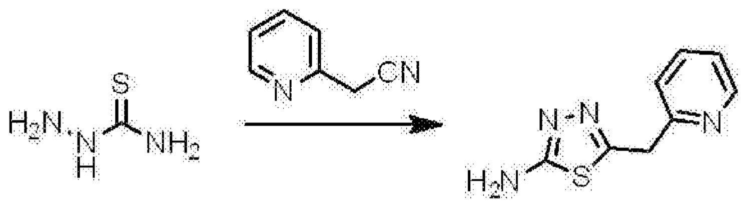

5-((Pyridin-2-yl)methyl)-1,3,4-thiadiazole-2-amine was obtained in the same manner as in Example 0256 except that (pyridin-2-yl)acetonitrile was used instead of the cyclopropylacetonitrile used in Example 0256. MSm/z(M+H):193.

<0261-2>

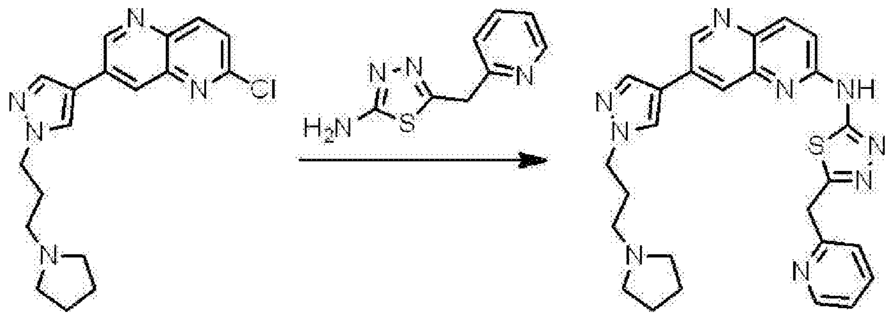

5-((Pyridin-2-yl)methyl)-N-(7-(1-(3-(pyrrolidin-1-yl)propyl)-1H-pyrazol-4-yl)-1,5-naphthyridin-2-yl)-1,3,4-thiadiazole-2-amine was obtained as a yellow solid in the same manner as in Example 0233 except that 5-((pyridin-2-yl)methyl)-1,3,4-thiadiazole-2-amine was used instead of the (S)-5-(tetrahydrofuran-2-yl)-1,3,4-thiadiazole-2-amine used in Example 0233.

$^1$H-NMR(DMSO-$d_6$)$\delta$:12.09(1H,s),9.08(1H,d,J=2.0Hz),8.57(2H,s),8.27(1H,s),8.25(1H,d,J=6.9Hz),8.21(1H,s),7.80(1H,td,J=7.7,1.9Hz),7.44(1H,d,J=7.6Hz),7.40(1H,d,J=8.9Hz),7.34-7.27(1H,m),4.54(2H,s),4.21(2H,t,J=6.9Hz),2.45-2.37(6H,m),2.05-1.96(2H,m),1.73-1.64(4H,m).

MSm/z(M+H):498.

<Example 0262>

<0262-1>

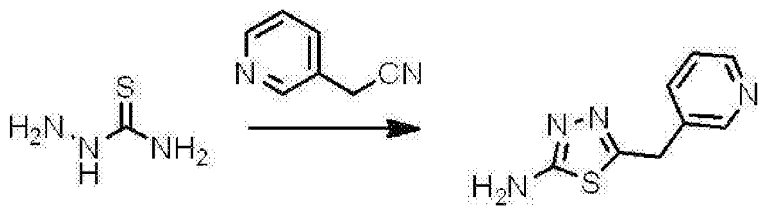

5-((Pyridin-3-yl)methyl)-1,3,4-thiadiazole-2-amine was obtained in the same manner as in Example 0256 except that (pyridin-3-yl)acetonitrile was used instead of the cyclopropylacetonitrile used in Example 0256.

MSm/z(M+H):193.

<0262-2>

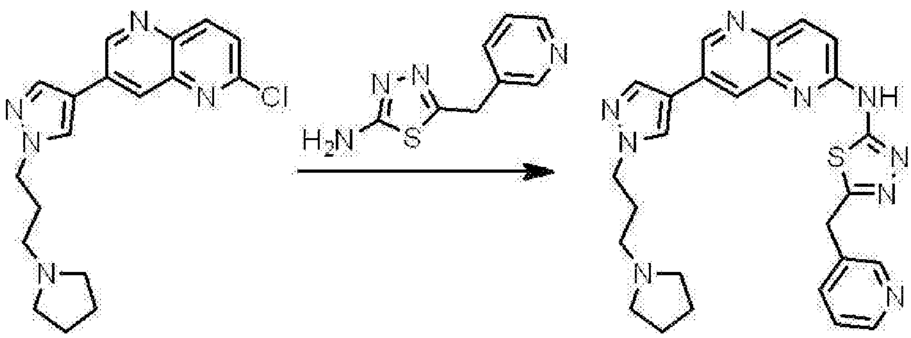

5-((Pyridin-3-yl)methyl)-N-(7-(1-(3-(pyrrolidin-1-yl)propyl)-1H-pyrazol-4-yl)-1,5-naphthyridin-2-yl)-1,3,4-thiadiazole-2-amine was obtained as a yellow solid in the same manner as in Example 0233 except that 5-((pyridin-3-yl)methyl)-1,3,4-thiadiazole-2-amine was used instead of the (S)-5-(tetrahydrofuran-2-yl)-1,3,4-thiadiazole-2-amine used in Example 0233.

$^1$H-NMR(DMSO-$d_6$)δ:12.14(1H,s),9.08(1H,d,J=2.0Hz),8.62(1H,d,J=1.7Hz),8.55(1H,s),8.50(1H,dd,J=5.0,1.7Hz),8.26(2H,t,J=5.3Hz),8.19(1H,s),7.77(1H,dt,J=7.9,2.0Hz),7.41-7.38(2H,m),4.45(2H,s),4.21(2H,t,J=7.1Hz),2.46-2.37(6H,m),2.05-1.96(2H,m),1.74-1.64(4H,m).
MSm/z(M+H):498.

<Example 0263>

<0263-1>

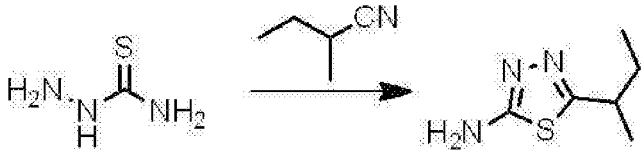

5-(sec-Butyl)-1,3,4-thiadiazole-2-amine was obtained in the same manner as in Example 0256 except that 2-methylbutyronitrile was used instead of the cyclopropylacetonitrile used in Example 0256.
MSm/z(M+H):158.

<0263-2>

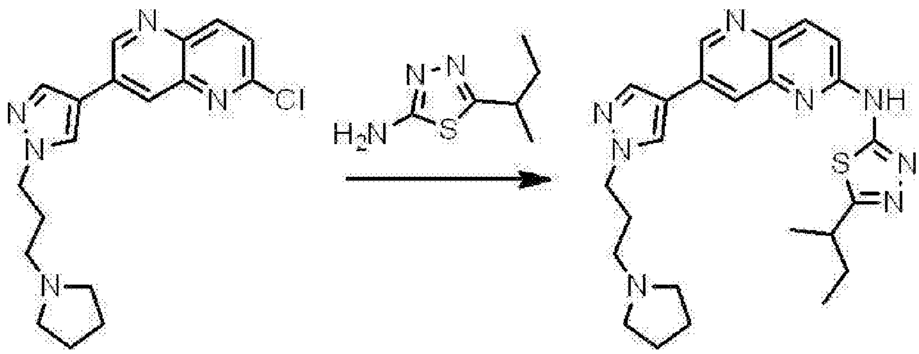

5-(sec-Butyl)-N-(7-(1-(3-(pyrrolidin-1-yl)propyl)-1H-pyrazol-4-yl)-1,5-naphthyridin-2-yl)-1,3,4-thiadiazole-2-amine was obtained as a pale yellow solid in the same manner as in Example 0233 except that 5-(sec-butyl)-1,3,4-thiadiazole-2-amine was used instead of the (S)-5-(tetrahydrofuran-2-yl)-1,3,4-thiadiazole-2-amine used in Example 0233.
$^1$H-NMR(DMSO-$d_6$)δ:12.05(1H,s),9.08(1H,d,J=2.3Hz),8.57(1H,s),8.33(1H,d,J=1.7Hz),8.25(2H,d,J=10.2Hz),7.40(1H,d,J=8.9Hz),4.22(2H,t,J=7.1Hz),3.23-3.11(1H,m),2.46-2.37(6H,m),2.06-1.95(2H,m),1.89-1.72(2H,m),1.71-1.62(4H,m),1.40(3H,d,J=6.9Hz),0.92(3H,t,J=7.4Hz).
MSm/z(M+H):463.

<Example 0264>

<0264-1>

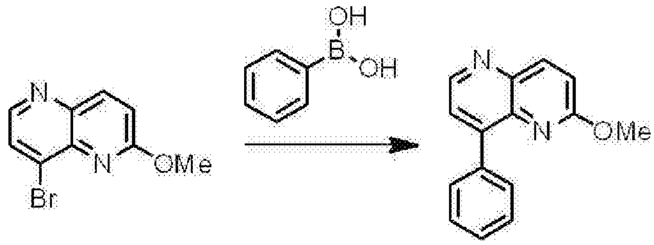

Phenylboronic acid (40 mg), bis(di-tert-butyl (4-dimethylaminophenyl)phosphine)dichloropalladium(II) (10 mg), sodium carbonate (45 mg), and water (200 μL) were added to a solution of 8-bromo-2-methoxy-1,5-naphthyridine (60 mg) in 1,4-dioxane (1 mL), followed by stirring at 120°C for 1 hour using a microwave reaction apparatus. The reaction mixture was cooled to room temperature, and ethyl acetate and a saturated sodium chloride aqueous solution were added thereto. The organic layer was collected by separation, and dried over anhydrous sodium sulfate, and the solvent was distilled off under reduced pressure. The obtained residue was purified by silica gel column chromatography (hexane-ethyl acetate), thereby obtaining 2-methoxy-8-phenyl-1,5-naphthyridine (50 mg).
$^1$H-NMR(DMSO-$d_6$)δ:8.85(1H,d,J=4.3Hz),8.33(1H,d,J=9.2Hz),7.94-7.91(2H,m),7.75(1H,d,J=4.6Hz),7.57-7.48(3H,m),7.30(1H,d,J=9.2Hz),3.91(3H,s).

<0264-2>

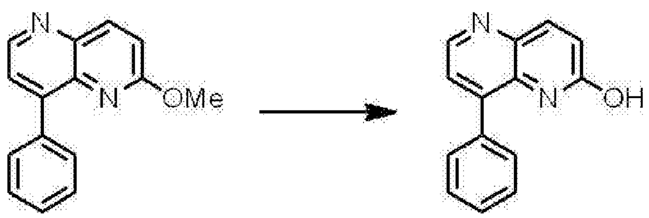

Hydrochloric acid (2 mL) was added to 2-methoxy-8-phenyl-1,5-naphthyridine (49 mg), followed by stirring at 80°C for 1 hour. The reaction mixture was cooled to room temperature, neutralized with a sodium hydroxide aqueous solution, and ethyl acetate was added thereto. The organic layer was collected by separation, and dried over anhydrous sodium sulfate, and the solvent was distilled off under reduced pressure, thereby obtaining 8-phenyl-1,5-naphthyridin-2-ol (45 mg).
$^1$H-NMR(DMSO-$d_6$)δ:10.40(1H,s),8.54(1H,d,J=4.6Hz),8.02(1H,d,J=9.9Hz),7.57-7.51(5H,m),7.43(1H,d,J=5.0Hz),6.81(1H,d,J=9.6Hz).

<0264-3>

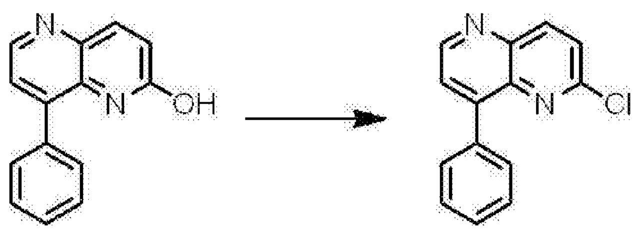

Phosphorus oxychloride (500 μL) and N,N-dimethylformamide (2 mL) were added to 8-phenyl-1,5-naphthyridin-2-ol (39 mg), followed by stirring at 80°C for 30 minutes. Phosphorus oxychloride (400 μL) was added thereto, followed by stirring at 80°C for 3 hours. The reaction mixture was cooled to room temperature, and added dropwise to water. The resultant product was neutralized by the addition of a sodium hydroxide aqueous solution, and ethyl acetate was added thereto. The organic layer was collected by separation, washed with a saturated sodium chloride aqueous solution, and

dried over anhydrous sodium sulfate, and the solvent was distilled off under reduced pressure. The obtained residue was purified by silica gel column chromatography (hexane-ethyl acetate), thereby obtaining 2-chloro-8-phenyl-1,5-naphthyridine (25 mg).
$^1$H-NMR(DMSO-$d_6$)δ:9.08(1H,d,J=4.6Hz),8.55(1H,d,J=8.9Hz),7.92-7.86(2H,m),7.80-7.75(2H,m),7.60-7.52(3H,m).

<0264-4>

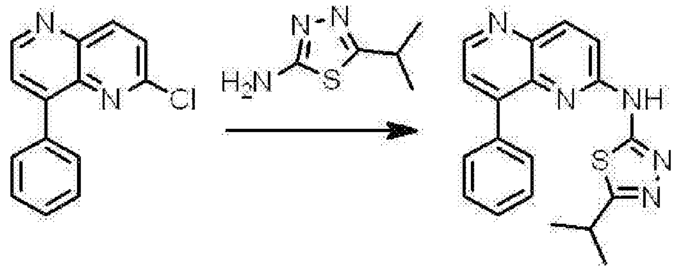

2-Amino-5-isopropyl-1,3,4-thiadiazole (8 mg), 2-dicyclohexylphosphino-2',4',6'-triisopropylbiphenyl (5 mg), cesium carbonate (15 mg), and tris(dibenzylideneacetone)dipalladium(0) (5 mg) were added to a solution of 2-chloro-8-phenyl-1,5-naphthyridine (10 mg) in 1,4-dioxane (1 mL), followed by stirring at 150°C for 45 minutes using a microwave reaction apparatus. After the reaction mixture was cooled to room temperature, a mixed solvent of chloroform-methanol was added thereto, and the obtained solution was purified by silica gel column chromatography (chloroform-methanol, NH silica), thereby obtaining 5-isopropyl-N-(8-phenyl-1,5-naphthyridin-2-yl)-1,3,4-thiadiazole-2-amine (3 mg) as a pale yellow solid.
$^1$H-NMR(DMSO-$d_6$)δ:11.99(1H,s),8.80(1H,d,J=4.6Hz),8.35(1H,d,J=8.9Hz),7.66-7.48(7H,m),3.21-3.10(1H,m),1.21(6H,d,J=7.2Hz).
MSm/z(M+H):348.

<Example 0265>

<0265-1>

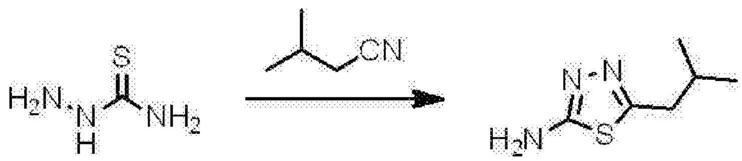

5-Isobutyl-1,3,4-thiadiazole-2-amine was obtained in the same manner as in Example 0256 except that isovaleronitrile was used instead of the cyclopropylacetonitrile used in Example 0256.
MSm/z(M+H):158.

<0265-2>

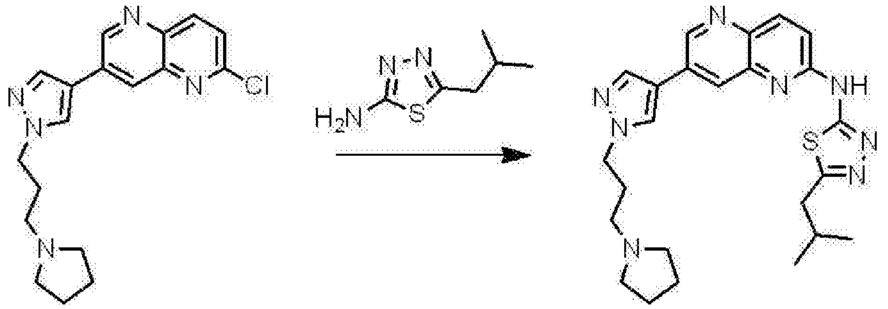

5-Isobutyl-N-(7-(1-(3-(pyrrolidin-1-yl)propyl)-1H-pyrazol-4-yl)-1,5-naphthyridin-2-yl)-1,3,4-thiadiazole-2-amine was obtained as a pale yellow solid in the same manner as in Example 0233 except that 5-isobutyl-1,3,4-thiadiazole-2-amine was used instead of the (S)-5-(tetrahydrofuran-2-yl)-1,3,4-thiadiazole-2-amine used in Example 0233.
$^1$H-NMR(DMSO-$d_6$)δ:12.06(1H,s),9.08(1H,d,J=2.0Hz),8.58(1H,s),8.32(1H,d,J=1.7Hz),8.25(1H,d,J=8.9Hz),8.23(1H,s), 7.40(1H,d,J=8.9Hz),4.22(2H,t,J=6.9Hz),2.89(2H,d,J=7.3Hz),2.20-1.97(9H,m),1.77-1.66(4H,m),0.99(6H,d,J=7.2Hz).
MSm/z(M+H):463.

<Example 0266>

<0266-1>

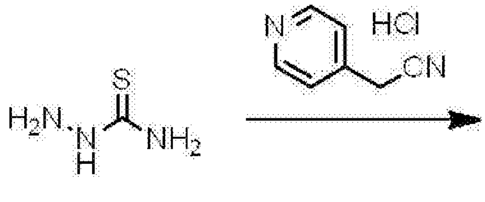


5-((Pyridin-4-yl)methyl)-1,3,4-thiadiazole-2-amine was obtained in the same manner as in Example 0256 except that (pyridin-4-yl)acetonitrile hydrochloride was used instead of the cyclopropylacetonitrile used in Example 0256.
MSm/z(M+H):193.

<0266-2>

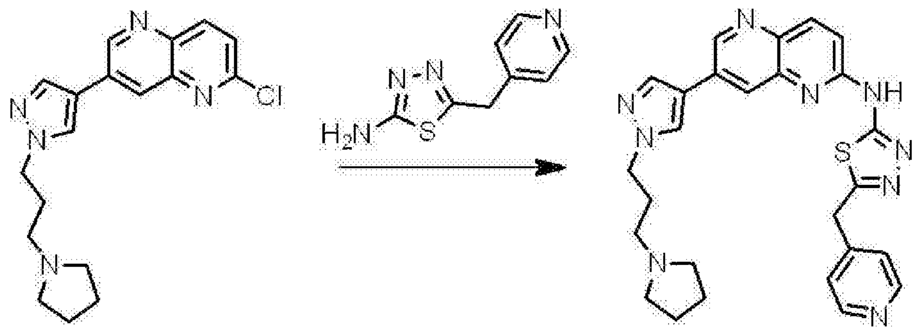

5-((Pyridin-4-yl)methyl)-N-(7-(1-(3-(pyrrolidin-1-yl)propyl)-1H-pyrazol-4-yl)-1,5-naphthyridin-2-yl)-1,3,4-thiadiazole-2-amine was obtained as a yellow solid in the same manner as in Example 0233 except that 5-((pyridin-4-yl)methyl)-1,3,4-thiadiazole-2-amine was used instead of the (S)-5-(tetrahydrofuran-2-yl)-1,3,4-thiadiazole-2-amine used in Example 0233.
$^1$H-NMR(DMSO-$d_6$)δ:12.19(1H,s),9.08(1H,d,J=2.3Hz),8.56-8.53(3H,m),8.29-8.21(3H,m),7.44-7.35(4H,m),4.46(2H,s),4.24(2H,t,J=6.8H

z),2.87-2.61(5H,m),2.13-2.04(2H,m),1.89-1.68(4H,m).
MSm/z(M+H):498.

<Example 0267>

<0267-1>

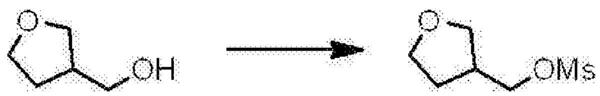

Triethylamine (800 μL) was added to a solution of (tetrahydrofuran-3-yl)methanol (410 mg) in dichloromethane (4 mL), and methanesulfonyl chloride (350 μL) was added dropwise thereto under ice-cooling, followed by stirring at 0°C for 30 minutes. Methanesulfonyl chloride (50 μL) was added thereto, followed by stirring at 0°C for 30 minutes. The reaction mixture was purified by silica gel column chromatography (hexane-ethyl acetate), thereby obtaining (tetrahydrofuran-3-yl)methyl methanesulfonate (540 mg).
$^1$H-NMR($CDCl_3$)δ:4.21-4.10(2H,m),3.88-3.63(4H,m),3.05(3H,s),2.75-2.64(1H,m),2.18-2.04(1H,m),1.76-1.62(1H,m).

<0267-2>

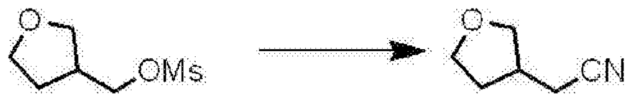

Sodium cyanide (70 mg) was added to a solution of (tetrahydrofuran-3-yl)methyl methanesulfonate (200 mg) in dimethylsulfoxide (3 mL), followed by stirring at 80°C overnight. The reaction mixture was cooled to room temperature, and ethyl acetate and a saturated sodium chloride aqueous solution were added thereto. The organic layer was collected by separation, and dried over anhydrous sodium sulfate, and the solvent was distilled off under reduced pressure. The obtained residue was purified by silica gel column chromatography (hexane-ethyl acetate), thereby obtaining 2-(tetrahydrofuran-3-yl)acetonitrile (80 mg).
$^1$H-NMR($CDCl_3$)δ:3.98-3.87(2H,m),3.84-3.77(1H,m),3.60-3.52(1H,m),2.65-2.56(1H,m),2.50-2.37(2H,m),2.25-2.14(1H,m),1.78-1.66(1H,m).

<0267-3>

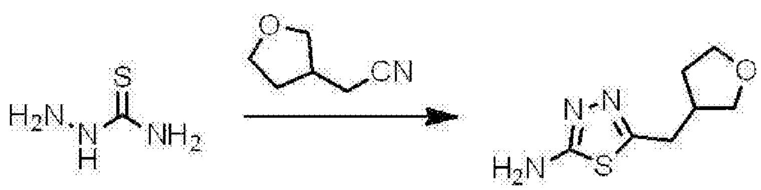

5-((Tetrahydrofuran-3-yl)methyl)-1,3,4-thiadiazole-2-amine was obtained in the same manner as in Example 0256 except that 2-(tetrahydrofuran-3-yl)acetonitrile was used instead of the cyclopropylacetonitrile used in Example 0256.
MSm/z(M+H):186.

<0267-4>

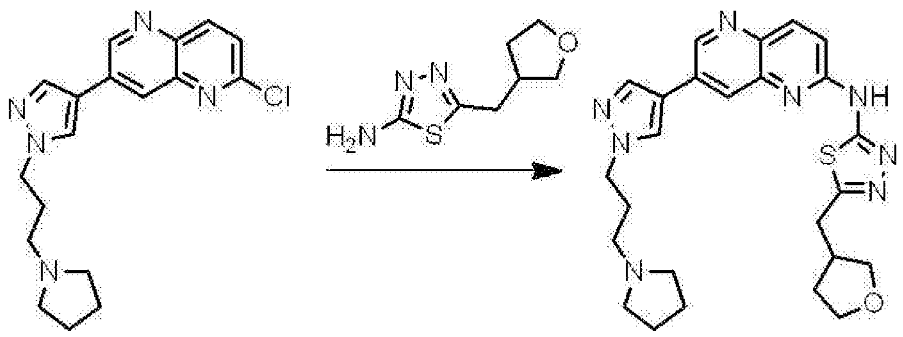

N-(7-(1-(3-(pyrrolidin-1-yl)propyl)-1H-pyrazol-4-yl)-1,5-naphthyridin-2-yl)-5-((tetrahydrofuran-3-yl)methyl)-1,3,4-thiadiazole-2-amine was obtained as a yellow solid in the same manner as in Example 0233 except that 5-((tetrahydrofuran-3-yl)methyl)-1,3,4-thiadiazole-2-amine was used instead of the (S)-5-(tetrahydrofuran-2-yl)-1,3,4-thiadiazole-2-amine used in Example 0233.
$^1$H-NMR(DMSO-$d_6$)$\delta$:12.08(1H,s),9.08(1H,d,J=2.3Hz),8.57(1H,s),8.33(1H,d,J=1.7Hz),8.26(1H,d,J=9.2Hz),8.22(1H,s),7.40(1H,d,J=9.2Hz),4.22(2H,t,J=6.9Hz),3.87-3.77(2H,m),3.73-3.64(1H,m),3.50-3.42(2H,m),3.10-3.06(2H,m),2.74-2.64(1H,m),2.46-2.38(6H,m),2.12-1.97(3H,m),1.73-1.60(4H,m).
MSm/z(M+H):491.

<Example 0268> (not in accordance with the present invention)

<0268-1>

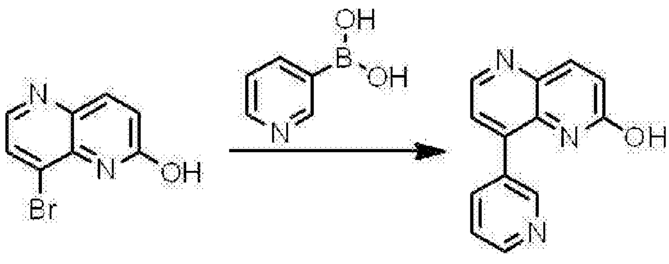

3-Pyridylboronic acid (35 mg), bis(di-tert-butyl (4-dimethylaminophenyl)phosphine)dichloropalladium(II) (5 mg), and a 2 mol/L sodium carbonate aqueous solution (100 μL) were added to a solution of 8-bromo-1,5-naphthyridin-2-ol (50 mg) in 1,4-dioxane (1.5 mL), followed by stirring at 130°C for 1 hour using a microwave reaction apparatus. The reaction mixture was cooled to room temperature, and ethyl acetate was added thereto. The obtained solution was purified by silica gel column chromatography (ethyl acetate-methanol, NH silica), thereby obtaining 8-(pyridin-3-yl)-1,5-naphthyridin-2-ol (33 mg).
$^1$H-NMR(DMSO-$d_6$)$\delta$:11.12(1H,s),8.68(2H,dd,J=5.0,1.7Hz),8.55(1H,d,J=4.3Hz),8.02(1H,d,J=10.2Hz),7.93(1H,d,J=7.6Hz),7.55(1H,ddd,J=7.8,4.9,0.7Hz),7.46(1H,d,J=4.3Hz),6.81(1H,d,J=9.2Hz).

<0268-2>

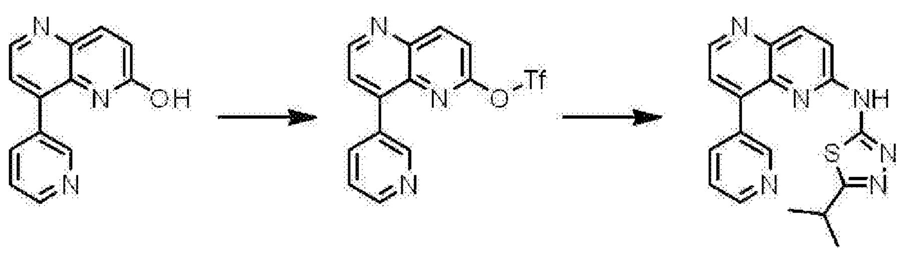

Triethylamine (50 μL) was added to a solution of 8-(pyridin-3-yl)-1,5-naphthyridin-2-ol (25 mg) in dichloromethane (2 mL), and trifluoromethanesulfonic aicd anhydride (20 μL) was added dropwise thereto under ice-cooling, followed by stirring at room temperature for 30 minutes. The reaction mixture was purified by silica gel column chromatography (hexane-ethyl acetate, NH silica), thereby obtaining (8-(pyridin-3-yl)-1,5-naphthyridin-2-yl) trifluoromethanesulfonate.

2-Amino-5-isopropyl-1,3,4-thiadiazole (14 mg), 2-dicyclohexylphosphino-2',4',6'-triisopropylbiphenyl (5 mg), cesium carbonate (20 mg), and tris(dibenzylideneacetone)dipalladium(0) (5 mg) were added to a solution of the obtained (8-(pyridin-3-yl)-1,5-naphthyridin-2-yl) trifluoromethanesulfonate in 1,4-dioxane (1.5 mL), followed by stirring at 150°C for 45 minutes using a microwave reaction apparatus. The reaction mixture was cooled to room temperature, and a mixed solvent of chloroform-methanol was added thereto. The obtained solution was purified by silica gel column chromatography (chloroform-methanol, NH silica), thereby obtaining 5-isopropyl-N-(8-(pyridin-3-yl)-1,5-naphthyridin-2-yl)-1,3,4-thiadiazole-2-amine (6 mg) as a pale yellow solid.

$^1$H-NMR(DMSO-$d_6$)δ:12.06(1H,s),8.87(1H,d,J=1.3Hz),8.84(1H,d,J=4.3Hz),8.73(1H,dd,J=5.0,1.7Hz),8.38(1H,d,J=8.9Hz),8.10-8.04(1H,m),7.71(1H,d,J=4.6Hz),7.65-7.58(1H,m),7.52(1H,d,J=9.2Hz),3.22-3.12(1H,m),1.24(6H,d,J=7.2Hz).

MSm/z(M+H):349.

<Example 0269> (not in accordance with the present invention)

<0269-1>

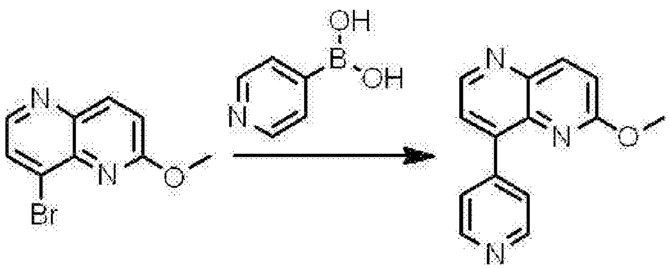

2-Methoxy-8-(pyridin-4-yl)-1,5-naphthyridine was obtained in the same manner as in Example 0264 except that 4-pyridylboronic acid was used instead of the phenylboronic acid used in Example 0264.

$^1$H-NMR(DMSO-$d_6$)δ:8.91(1H,d,J=4.3Hz),8.75-8.72(2H,m),8.37(1H,d,J=8.9Hz),7.93-7.91(2H,m),7.86-7.83(1H,m),7.34(1H,d,J=8.9Hz), 3.93(3H,s).

<0269-2>

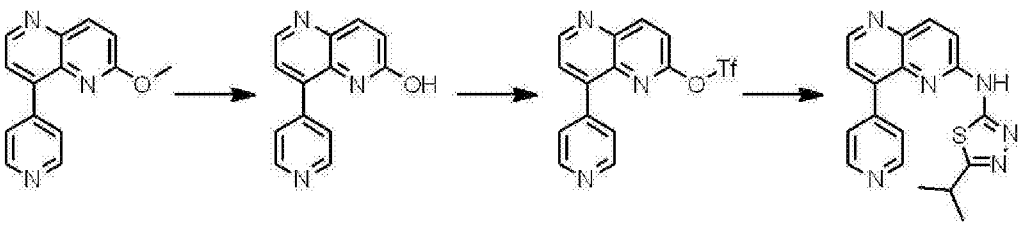

Hydrochloric acid (3 mL) was added to 2-methoxy-8-(pyridin-4-yl)-1,5-naphthyridine (90 mg), followed by stirring at 80°C for 3 hours. The reaction mixture was cooled to room temperature, neutralized with a sodium hydroxide aqueous solution, and the solvent was distilled off under reduced pressure, thereby obtaining 8-(pyridin-4-yl)-1,5-naphthyridine-2-ol. Triethylamine (300 μL) was added to a solution of the obtained 8-(pyridin-4-yl)-1,5-naphthyridin-2-ol in dichloromethane (3 mL), and trifluoromethanesulfonic aicd anhydride (150 μL) was added dropwise thereto under ice-cooling, followed by stirring at room temperature for 30 minutes. Water was added to the reaction mixture. The organic layer was collected by separation, and dried over anhydrous sodium sulfate, then, the solvent was distilled off under reduced

pressure, and the resultant product was purified by silica gel column chromatography (hexane-ethyl acetate, NH silica), thereby obtaining (8-(pyridin-4-yl)-1,5-naphthyridin-2-yl) trifluoromethanesulfonate. 2-Amino-5-isopropyl-1,3,4-thiadiazole (30 mg), 2-dicyclohexylphosphino-2',4',6'-triisopropylbiphenyl (10 mg), cesium carbonate (70 mg), and tris(dibenzylideneacetone)dipalladium(0) (10 mg) were added to a solution of the obtained (8-(pyridin-4-yl)-1,5-naphthyridin-2-yl) trifluoromethanesulfonate in 1,4-dioxane (2 mL), followed by stirring at 150°C for 30 minutes using a microwave reaction apparatus. The reaction mixture was cooled to room temperature, and purified by silica gel column chromatography (ethyl acetate-methanol, NH silica), thereby obtaining 5-isopropyl-N-(8-(pyridin-4-yl)-1,5-naphthyridin-2-yl)-1,3,4-thiadiazole-2-amine (23 mg) as a pale yellow solid.
$^1$H-NMR(DMSO-$d_6$)δ:12.07(1H,s),8.84(1H,d,J=4.6Hz),8.77(2H,dd,J=4.5,1.5Hz),8.38(1H,d,J=8.9Hz),7.68-7.65(3H,m),7.51(1H,d,J=9.2 Hz),3.24-3.09(1H,m),1.22(6H,d,J=7.2Hz).
MSm/z(M+H):349.

<Example 0270> (not in accordance with the present invention)

<0270-1>

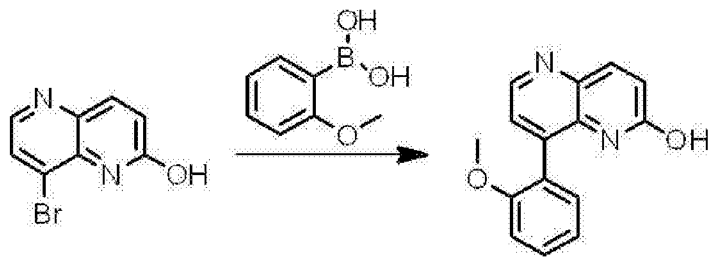

8-(2-Methoxyphenyl)-1,5-naphthyridin-2-ol was obtained in the same manner as in Example 0268 except that 2-methoxyphenylboronic acid was used instead of the 3-pyridylboronic acid used in Example 0268.
$^1$H-NMR(DMSO-$d_6$)δ:10.33(1H,s),8.49(1H,d,J=5.0Hz),7.99(1H,d,J=9.9Hz),7.53-7.47(1H,m),7.34(1H,d,J=4.6Hz),7.26(1H,dd,J=7.6,1.7 Hz),7.18(1H,d,J=8.3Hz),7.09(1H,dd,J=7.9,6.9H z),6.76(1H,d,J=9.6Hz),3.74(3H,s).

<0270-2>

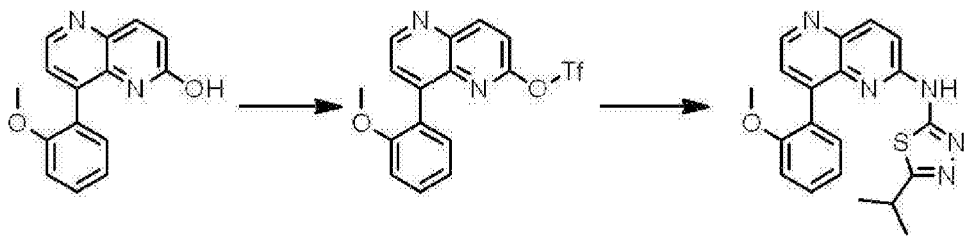

5-Isopropyl-N-(8-(2-methoxyphenyl)-1,5-naphthyridin-2-yl)-1,3,4-thiadiazole-2-amine was obtained as a pale yellow solid in the same manner as in Example 0268 except that 8-(2-methoxyphenyl)-1,5-naphthyridin-2-ol was used instead of the 8-(pyridin-3-yl)-1,5-naphthyridin-2-ol used in Example 0268.
$^1$H-NMR(DMSO-$d_6$)δ:12.00(1H,s),8.79(1H,d,J=4.3Hz),8.35(1H,d,J=8.9Hz),7.61(1H,d,J=4.3Hz),7.51-7.45(2H,m),7.23-7.20(1H,m),7.18-7.14(1H,m),7.12-7.06(1H,m),3.77(3H,s),3.20-3.08(1H,m),1.22(6H,d,J=7.2Hz).
MSm/z(M+H):378.

<Example 0271> (not in accordance with the present invention)

<0271-1>

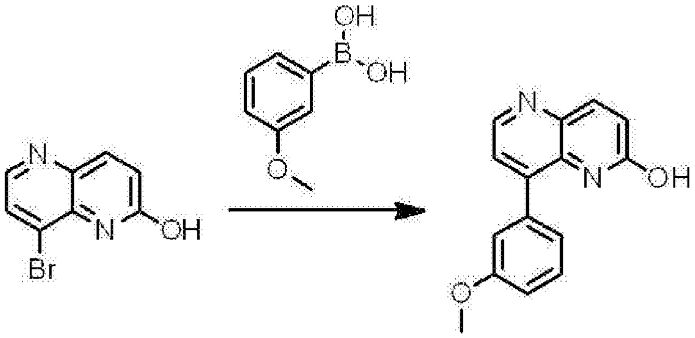

8-(3-Methoxyphenyl)-1,5-naphthyridin-2-ol was obtained in the same manner as in Example 0268 except that 3-methoxyphenylboronic acid was used instead of the 3-pyridylboronic acid used in Example 0268.
$^1$H-NMR(DMSO-$d_6$)δ:11.95(1H,s),8.77(1H,d,J=4.3Hz),8.33(1H,d,J=9.2Hz),7.55-7.49(2H,m),7.45(1H,d,J=9.2Hz),7.29(1H,dd,J=7.4,1.8 Hz),7.21(1H,d,J=7.9Hz),7.13(1H,td,J=7.3,0.8Hz ),3.59(3H,s).

<0271-2>

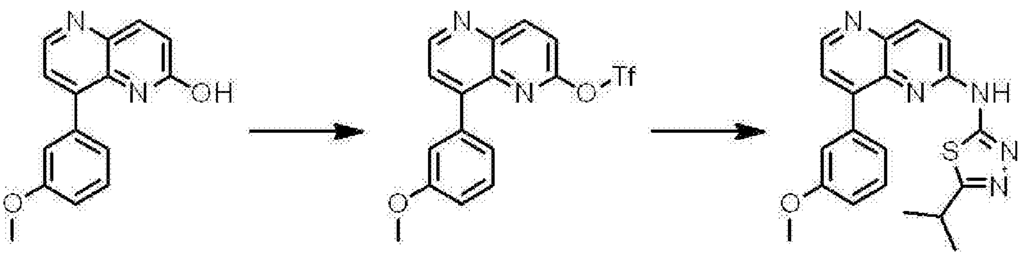

5-Isopropyl-N-(8-(3-methoxyphenyl)-1,5-naphthyridin-2-yl)-1,3,4-thiadiazole-2-amine was obtained as a pale yellow solid in the same manner as in Example 0268 except that 8-(3-methoxyphenyl)-1,5-naphthyridin-2-ol was used instead of the 8-(pyridin-3-yl)-1,5-naphthyridin-2-ol used in Example 0268.
$^1$H-NMR(DMSO-$d_6$)δ:11.95(1H,s),8.77(1H,d,J=4.3Hz),8.33(1H,d,J=9.2Hz),7.55-7.49(2H,m),7.45(1H,d,J=9.2Hz),7.29(1H,dd,J=7.4,1.8 Hz),7.21(1H,d,J=7.9Hz),7.13(1H,td,J=7.3,0.8Hz ),3.59(3H,s),3.20-3.07(1H,m),1.23(6H,d,J=7.2Hz).
MSm/z(M+H):378.

<Example 0272> (not in accordance with the present invention)

<0272-1>

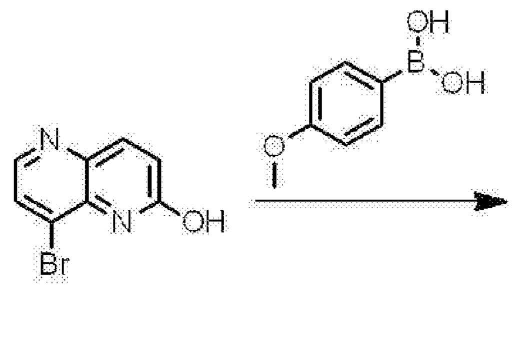

8-(4-Methoxyphenyl)-1,5-naphthyridin-2-ol was obtained in the same manner as in Example 0268 except that 4-methoxyphenylboronic acid was used instead of the 3-pyridylboronic acid used in Example 0268.
$^1$H-NMR(DMSO-$d_6$)δ:11.97(1H,s),8.76(1H,d,J=4.6Hz),8.33(1H,d,J=8.9Hz),7.61-7.57(3H,m),7.48(1H,d,J=9.2Hz),7.12(2H,d,J=8.9Hz),3.85(3H,s).

<0272-2>

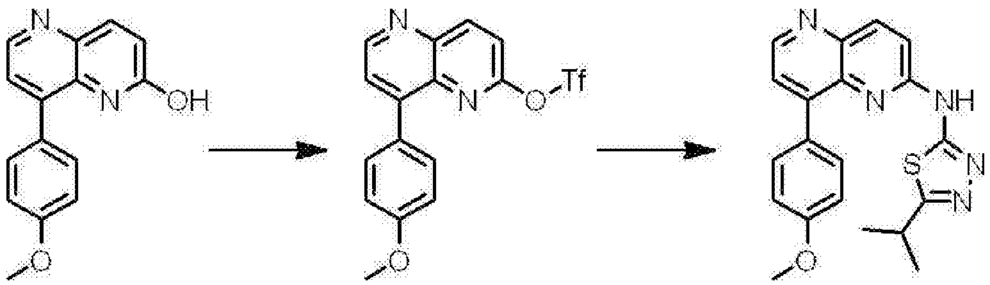

5-Isopropyl-N-(8-(4-methoxyphenyl)-1,5-naphthyridin-2-yl)-1,3,4-thiadiazole-2-amine was obtained as a pale yellow solid in the same manner as in Example 0268 except that 8-(4-methoxyphenyl)-1,5-naphthyridin-2-ol was used instead of the 8-(pyridin-3-yl)-1,5-naphthyridin-2-ol used in Example 0268.
$^1$H-NMR(DMSO-$d_6$)δ:11.97(1H,s),8.76(1H,d,J=4.6Hz),8.33(1H,d,J=8.9Hz),7.62-7.57(3H,m),7.48(1H,d,J=9.2Hz),7.15-7.09(2H,m),3.85(3H,s),3.24-3.12(1H,m),1.22(6H,d,J=7.2Hz).
MSm/z(M+H):378.

<Example 0273> (not in accordance with the present invention)

<0273-1>

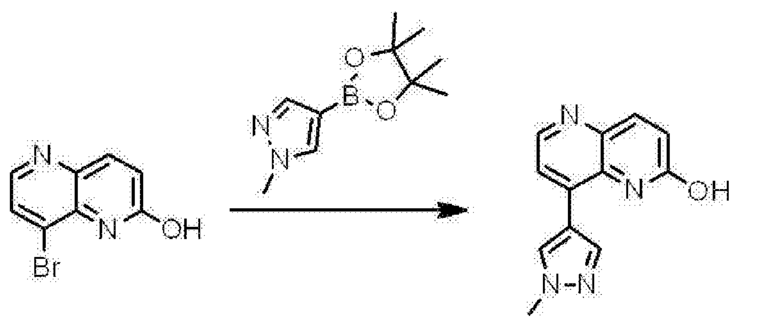

8-(1-Methyl-1H-pyrazol-4-yl)-1,5-naphthyridin-2-ol was obtained in the same manner as in Example 0268 except that 1-methylpyrazole-4-boronic acid pinacol ester was used instead of the 3-pyridylboronic acid used in Example 0268.
$^1$H-NMR(DMSO-$d_6$)δ:10.36(1H,s),8.50(2H,d,J=4.0Hz),8.03(2H,d,J=9.9Hz),7.59(1H,s),6.88(1H,s),3.93(3H,s).

<0273-2>

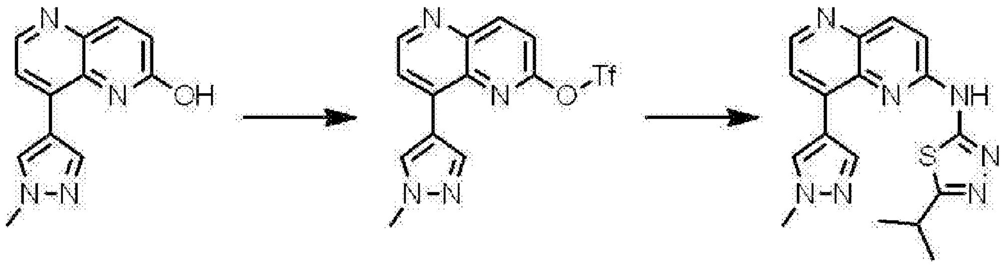

5-Isopropyl-N-(8-(1-methyl-1H-pyrazol-4-yl)-1,5-naphthyridin-2-yl)-1,3,4-thiadiazole-2-amine was obtained as a pale yellow solid in the same manner as in Example 0268 except that 8-(1-methyl-1H-pyrazol-4-yl)-1,5-naphthyridin-2-ol was used instead of the 8-(pyridin-3-yl)-1,5-naphthyridin-2-ol used in Example 0268.
$^1$H-NMR(DMSO-$d_6$)δ:
11.90(1H,s),8.68(1H,d,J=4.6Hz),8.50(1H,s),8.29(1H,d,J=8.9Hz),8.10(1H,s),7.70(1H,d,J=4.6Hz),7 .53(1H,d,J=9.2Hz), 3.96(3H,s),3.54-3.39(1H,m),1.34(6H,d,J=7.2Hz).
MSm/z(M+H):352.

<Example 0274> (not in accordance with the present invention)

<0274-1>

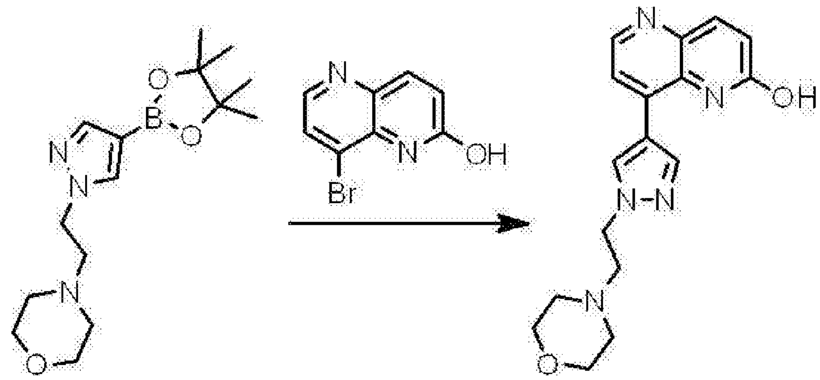

8-(1-(2-Morpholinoethyl)-1H-pyrazol-4-yl)-1,5-naphthyridin-2-ol was obtained in the same manner as in Example 0268 except that 1-(2-morpholinoethyl)-1H-pyrazole-4-boronic acid pinacol ester was used instead of the 3-pyridylboronic acid used in Example 0268.
$^1$H-NMR(DMSO-$d_6$)δ:10.23(1H,s),8.50(2H,d,J=4.6Hz),8.04(2H,d,J=9.6Hz),7.60(1H,s),6.90(1H,d,J=7.6Hz),4.31(2H,t,J =6.8Hz),3.57(4H,t,J=4.6Hz),2.79(2H,t,J=6.8Hz),2.45(4H,t,J=4.5Hz).

<0274-2>

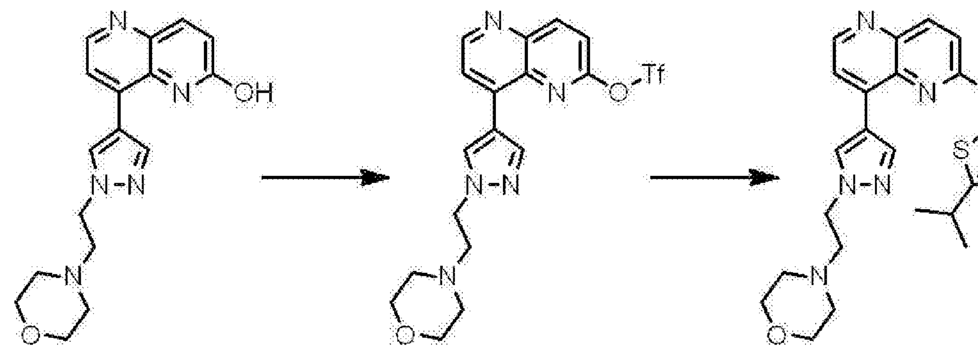

5-Isopropyl-N-(8-(1-(2-morpholinoethyl)-1H-pyrazol-4-yl)-1,5-naphthyridin-2-yl)-1,3,4-thiadiazole-2-amine was obtained as a pale yellow solid in the same manner as in Example 0268 except that 8-(1-(2-morpholinoethyl)-1H-pyrazol-4-yl)-1,5-naphthyridin-2-ol was used instead of the 8-(pyridin-3-yl)-1,5-naphthyridin-2-ol used in Example 0268.
$^1$H-NMR(DMSO-$d_6$)δ:
11.90(1H,s),8.69(1H,d,J=4.6Hz),8.55(1H,s),8.29(1H,d,J=8.9Hz),8.17(1H,s),7.70(1H,d,J=4.6Hz),7 .53(1H,d,J=9.2Hz), 4.34(2H,t,J=6.8Hz),3.60-3.50(4H,m),3.30-3.20(1H,m),2.79(2H,t,J=6.8Hz),2.46-2.39(4H,m),1.34(6H,d,J=7.2Hz).
MSm/z(M+H):451.

<Example 0275> (not in accordance with the present invention)

<0275-1>

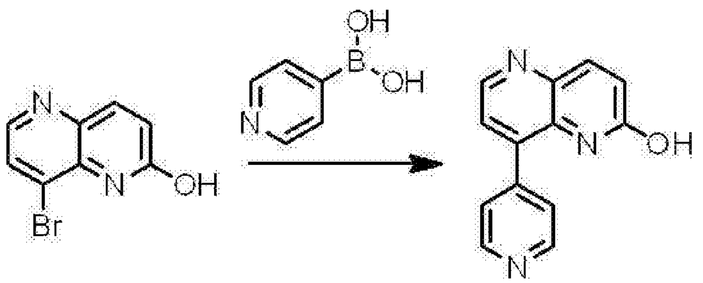

4-Pyridylboronic acid (130 mg), bis(di-tert-butyl (4-dimethylaminophenyl)phosphine)dichloropalladium(II) (10 mg), and a 2 mol/L sodium carbonate aqueous solution (500 μL) were added to a solution of 8-bromo-1,5-naphthyridin-2-ol (200 mg) in 1,4-dioxane (3 mL), followed by stirring at 130°C for 30 minutes using a microwave reaction apparatus. The reaction mixture was cooled to room temperature, and ethyl acetate was added thereto. The obtained solution was purified by silica gel column chromatography (ethyl acetate-methanol, NH silica), thereby obtaining 8-(pyridin-4-yl)-1,5-naphthyridin-2-ol (101 mg).
$^1$H-NMR(DMSO-
$d_6$)δ:10.98(1H,s),8.72(2H,dd,J=4.3,1.7Hz),8.57(1H,d,J=4.6Hz),8.03(1H,d,J=9.6Hz),7.54(2H,d,J=5.9H z),7.46(1H,d,J=4.6Hz),6.82(1H,d,J=9.6Hz).

<0275-2>

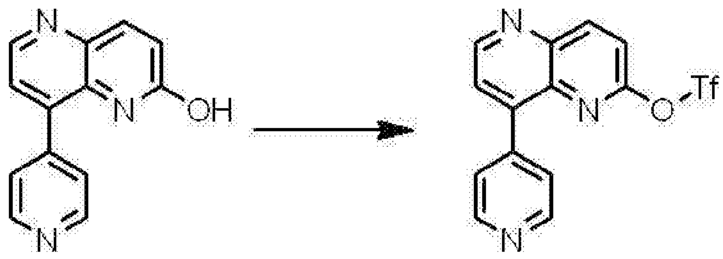

Triethylamine (300 μL) was added to a solution of 8-(pyridin-4-yl)-1,5-naphthyridin-2-ol (100 mg) in dichloromethane (3 mL), and trifluoromethanesulfonic aicd anhydride (150 μL) was added dropwise thereto under ice-cooling, followed by stirring at room temperature for 30 minutes. Trifluoromethanesulfonic aicd anhydride (50 μL) was added dropwise thereto under ice-cooling, followed by stirring for 30 minutes. Water was added to the reaction mixture. The organic layer was collected by separation, and dried over anhydrous sodium sulfate, then, the solvent was distilled off under reduced pressure, and the obtained residue was purified by silica gel column chromatography (hexane-ethyl acetate), thereby obtaining (8-(pyridin-4-yl)-1,5-naphthyridin-2-yl) trifluoromethanesulfonate (101 mg) as a brown solid.
$^1$H-NMR(DMSO-$d_6$)δ:9.24(1H,d,J=4.3Hz),8.88(1H,d,J=8.9Hz),8.76(2H,dd,J=4.3,1.7Hz),8.07(1H,d,J=4.6Hz),8.02(1H,d, J=8.9Hz),7.77(2H,dd,J=4.3,1.7Hz).

<0275-3>

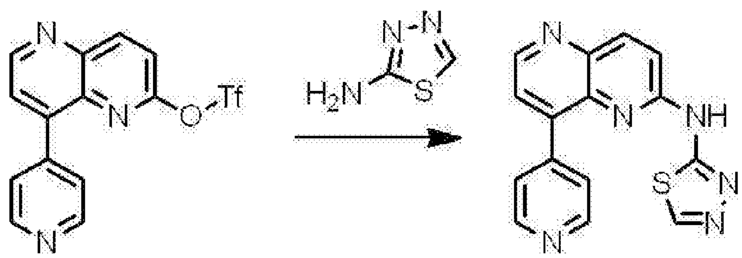

2-Aminothiadiazole (10 mg), 2-dicyclohexylphosphino-2',4',6'-triisopropylbiphenyl (5 mg), cesium carbonate (45 mg), and tris(dibenzylideneacetone)dipalladium(0) (5 mg) were added to a solution of (8-(pyridin-4-yl)-1,5-naphthyridin-2-yl) trifluoromethanesulfonate (20 mg) in 1,4-dioxane (1 mL), followed by stirring at 150°C for 45 minutes using a microwave reaction apparatus. The reaction mixture was cooled to room temperature, and a mixed solvent of chloroform-methanol was added thereto. The obtained solution was purified by silica gel column chromatography (ethyl acetate-methanol, NH silica), thereby obtaining N-(8-(pyridin-4-yl)-1,5-naphthyridin-2-yl)-1,3,4-thiadiazole-2-amine (8 mg) as a pale yellow solid.
$^1$H-NMR(DMSO-$d_6$)δ:12.26(1H,s),9.00(1H,s),8.86(1H,d,J=4.3Hz),8.77(2H,dd,J=4.3,1.7Hz),8.41(1H,d,J=9.2Hz),7.70(3H,t,J=3.0Hz),7.57(1H,d,J=9.2Hz).
MSm/z(M+H):307.

<Example 0276> (not in accordance with the present invention)

<0276-1>

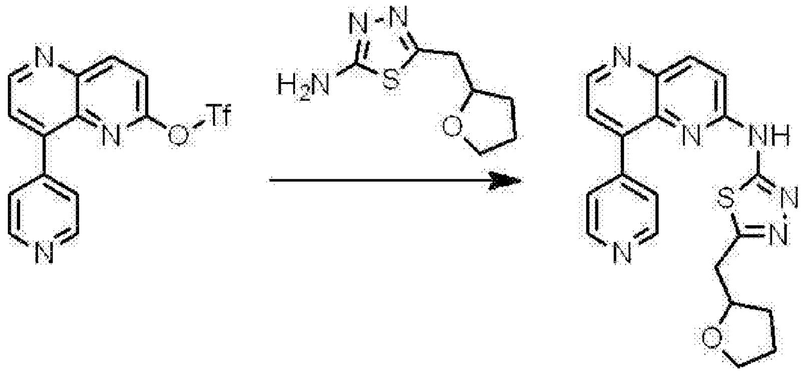

N-(8-(pyridin-4-yl)-1,5-naphthyridin-2-yl)-5-((tetrahydrofuran-2-yl)methyl)-1,3,4-thiadiazole-2-amine was obtained as a pale yellow solid in the same manner as in Example 0275 except that 5-((tetrahydrofuran-2-yl)methyl)-1,3,4-thiadiazole-2-amine was used instead of the 2-aminothiadiazole used in Example 0275.
$^{1}$H-NMR(DMSO-$d_6$)S:12.07(1H,s),8.85(1H,d,J=4.3Hz),8.76(2H,dd,J=4.5,1.5Hz),8.38(1H,d,J=9.2Hz),7.68(3H,dd,J=4.0, 2.0Hz),7.53(1H,d,J=8.9Hz),4.04-3.92(1H,m),3.80-3.63(2H,m),3.02-2.97(2H,m),2.01-1.79(3H,m),1.53-1.43(1H,m).
MSm/z(M+H):391.

<Example 0277>

<0277-1>

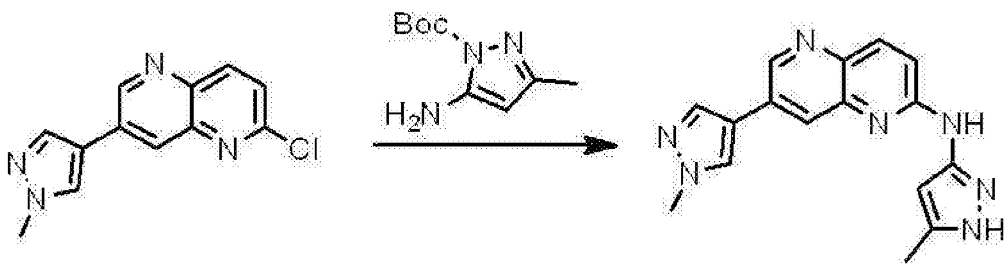

N-(5-methyl-1H-pyrazol-3-yl)-7-(1-methyl-1H-pyrazol-4-yl)-1,5-naphthyridine-2-amine was obtained as a white solid in the same manner as in Example 0246 except that tert-butyl 5-amino-3-methyl-1H-pyrazole-1-carboxylate was used instead of the (9H-fluoren-9-yl)methyl 2-(5-amino-1,3,4-thiadiazol-2-yl)pyrrolidine-1-carboxylate used in Example 0246.
$^{1}$H-NMR($CD_3OD$)$\delta$:7.62(1H,d,J=1.7Hz),7.03(1H,s),6.91(1H,d,J=9.6Hz),6.84(1H,s),6.62(1H,s),5.90(1H,d ,J=9.2Hz),4.57(1 H,s),2.48(3H,s),0.88(3H,s).
MSm/z(M+H):306.

<Example 0278> (not in accordance with the present invention)

<0278-1>

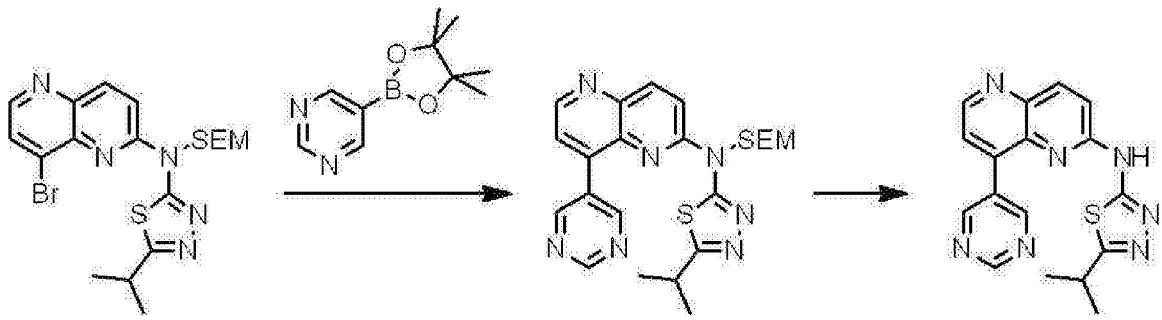

5-(4,4,5,5-Tetramethyl-1,3,2-dioxaborolan-2-yl)pyrimidine (5 mg), bis(di-tert-butyl (4-dimethylaminophenyl)phosphine)dichloropalladium(II) (3 mg), and a 2 mol/L sodium carbonate aqueous solution (50 μL) were added to a solution of N-(8-bromo-1,5-naphthyridin-2-yl)-5-isopropyl-N-((2-trimethylsilyl)ethoxy)methyl)-1,3,4-thiadiazole-2-amine (10 mg) in 1,4-dioxane (1 mL), followed by stirring at 130°C for 45 minutes using a microwave reaction apparatus. The reaction mixture was cooled to room temperature, and ethyl acetate and water were added thereto. The organic layer was collected by separation, and dried over anhydrous sodium sulfate, and the solvent was distilled off under reduced pressure. The obtained solution was purified by silica gel column chromatography (hexane-ethyl acetate, NH silica), thereby obtaining 5-isopropyl-N-(8-(pyrimidin-5-yl)-1,5-naphthyridin-2-yl)-N-((2-(trimethylsilyl)ethoxy)methyl)-1,3,4-thiadiazole-2-amine.

Trifluoroacetic acid (1 mL) and water (50 μL) were added to the obtained 5-isopropyl-N-(8-(pyrimidin-5-yl)-1,5-naphthyridin-2-yl)-N-((2-(trimethylsilyl)ethoxy)methyl)-1,3,4-thiadiazole-2-amine, followed by stirring at room temperature for 1 hour. After the solvent was distilled off under reduced pressure, triethylamine (2 drops) was added thereto, and the resultant product was purified by silica gel column chromatography (ethyl acetate-methanol, NH silica), thereby obtaining 5-isopropyl-N-(8-(pyrimidin-5-yl)-1,5-naphthyridin-2-yl)-1,3,4-thiadiazole-2-amine (0.58 mg).
$^1$H-NMR(DMSO-$d_6$)δ:12.14(1H,s),9.37(1H,s),9.15(2H,s),8.88(1H,d,J=4.6Hz),8.40(1H,d,J=8.9Hz),7.82(1H,d,J=4.3Hz),7.54(1H,d,J=9.2Hz),3.26-3.15(1H,m),1.27(6H,d,J=6.9Hz).
MSm/z(M+H):350.

<Example 0279> (not in accordance with the present invention)

<0279-1>

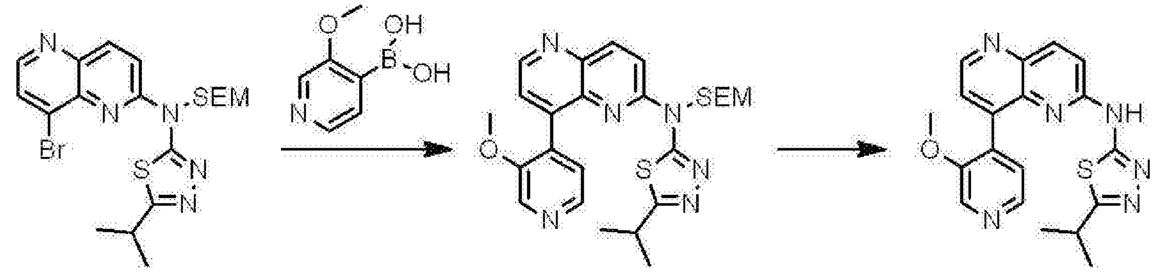

5-Isopropyl-N-(8-(3-methoxypyridin-4-yl)-1,5-naphthyridin-2-yl)-1,3,4-thiadiazole-2-amine was obtained as a pale yellow solid in the same manner as in Example 0278 except that 3-methoxypyridine-4-boronic acid was used instead of the 5-(4,4,5,5-tetramethyl-1,3,2-dioxaborolan-2-yl)pyrimidine used in Example 0278.
$^1$H-NMR(DMSO-$d_6$)δ:12.09(1H,s),8.83(1H,d,J=4.3Hz),8.39-8.33(2H,m),7.65(1H,d,J=4.3Hz),7.51(1H,d,J=9.2Hz),7.20(1H,dd,J=5.3,1.7 Hz),7.10(1H,d,J=1.3Hz),3.9 3(3H,d,J=1.7Hz),3.25-3.12(1H,m),1.23(6H,d,J=7.2Hz).
MSm/z(M+H):379.

<Example 0280> (not in accordance with the present invention)

<0280-1>

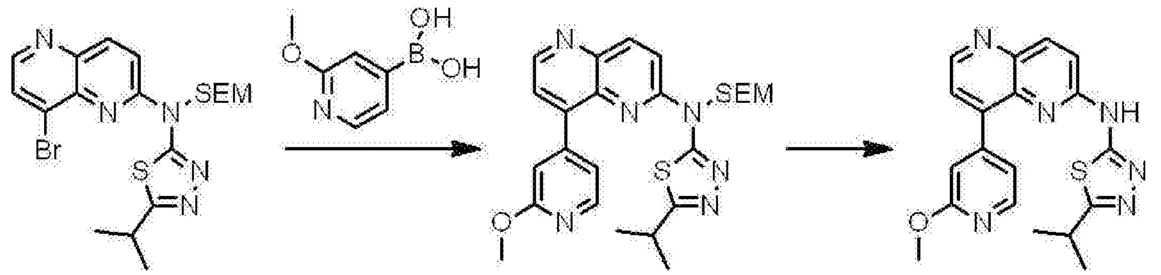

5-Isopropyl-N-(8-(2-methoxypyridin-4-yl)-1,5-naphthyridin-2-yl)-1,3,4-thiadiazole-2-amine was obtained as a pale yellow solid in the same manner as in Example 0278 except that 2-methoxypyridine-4-boronic acid was used instead of the -5-(4,4,5,5-tetramethyl-1,3,2-dioxaborolan-2-yl)pyrimidine used in Example 0278.
$^1$H-NMR(DMSO-$d_6$)δ:12.08(1H,s),8.82(1H,d,J=4.3Hz),8.61(1H,s),8.43(1H,d,J=4.6Hz),8.36(1H,d,J=8.9Hz),7.61(1H,d,J =4.3Hz),7.48(1H,d,J=9.2Hz),7.42(1H,d,J=5.0Hz),3.73(3H,s),3.20-3.11(1H,m),1.23(6H,d,J=6.9Hz).
MSm/z(M+H):379.

<Example 0281> (not in accordance with the present invention)

<0281-1>

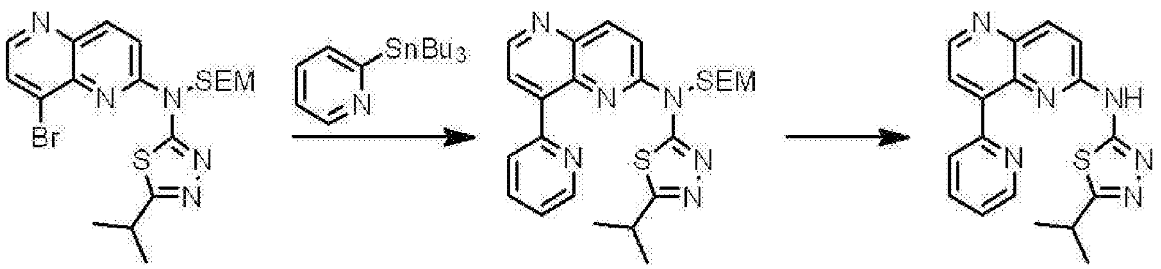

2-(Tributyltin)pyridine (15 μL), tetrakis(triphenylphosphine)palladium(0) (5 mg), and cesium carbonate (20 mg) were added to a solution of N-(8-bromo-1,5-naphthyridin-2-yl)-5-isopropyl-N-((2-trimethylsilyl)ethoxy)methyl)-1,3,4-thiadiazole-2-amine (10 mg) in 1,4-dioxane (1 mL), followed by stirring at 100°C for 30 minutes using a microwave reaction apparatus. The reaction mixture was cooled to room temperature, and ethyl acetate and water were added thereto. The organic layer was collected by separation, and dried over anhydrous sodium sulfate, and the solvent was distilled off under reduced pressure, thereby obtaining 5-isopropyl-N-(8-(pyridin-2-yl)-1,5-naphthyridin-2-yl)-N-((2-(trimethylsilyl)ethoxy)methyl)-1,3,4-thiadiazole-2-amine.

Trifluoroacetic acid (1 mL) and water (50 μL) were added to the obtained 5-isopropyl-N-(8-(pyridin-2-yl)-1,5-naphthyridin-2-yl)-N-((2-(trimethylsilyl)ethoxy)methyl)-1,3,4-thiadiazole-2-amine, followed by allowing to stand at room temperature overnight. The solvent was distilled off under reduced pressure, and ethyl acetate and a saturated sodium hydrogen carbonate aqueous solution were added thereto. The organic layer was collected by separation, and dried over anhydrous sodium sulfate, and the solvent was distilled off under reduced pressure. The obtained residue was purified by silica gel column chromatography (ethyl acetate-methanol, NH silica), thereby obtaining 5-isopropyl-N-(8-(pyridin-2-yl)-1,5-naphthyridin-2-yl)-1,3,4-thiadiazole-2-amine (2.5 mg).
$^1$H-NMR(DMSO-$d_6$)δ:12.05(1H,s),8.85(1H,d,J=4.3Hz),8.81(1H,d,J=5.0Hz),8.38(1H,d,J=8.9Hz),8.03-7.93(2H,m),7.77(1H,d,J=4.6Hz),7.58-7.55(1H,m),7.52(1H,d,J=9.2Hz),3.24-3.16(1H,m),1.23(6H,d,J=6.9Hz).
MSm/z(M+H):349.

<Example 0282> (not in accordance with the present invention)

<0282-1>

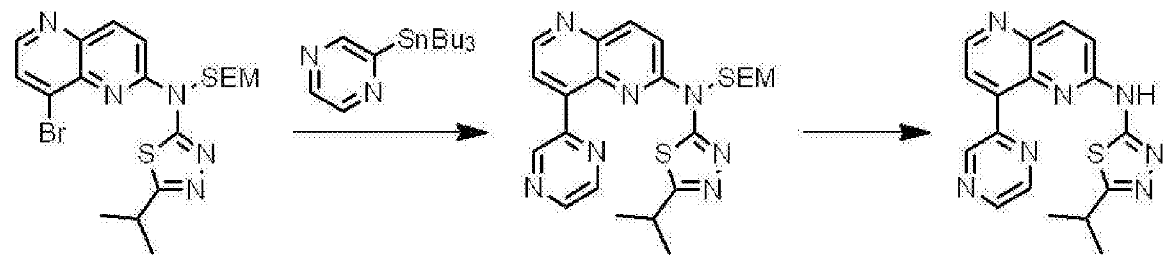

5-Isopropyl-N-(8-(pyrazin-2-yl)-1,5-naphthyridin-2-yl)-1,3,4-thiadiazole-2-amine was obtained as a pale yellow solid in the same manner as in Example 0281 except that 2-(tributyltin)pyrazine was used instead of the 2-(tributyltin)pyridine used in Example 0281.
$^1$H-NMR(DMSO-$d_6$)δ:12.13(1H,s),9.24(1H,d,J=1.7Hz),8.92-8.89(2H,m),8.82(1H,s),8.41(1H,d,J=9.2Hz),7.86(1H,d,J=4.6Hz),7.55(1H,d,J=8.9Hz),3.24-3.13(1H,m),1.26(6H,d,J=6.9Hz).
MSm/z(M+H):350.

<Example 0283> (not in accordance with the present invention)

<0283-1>

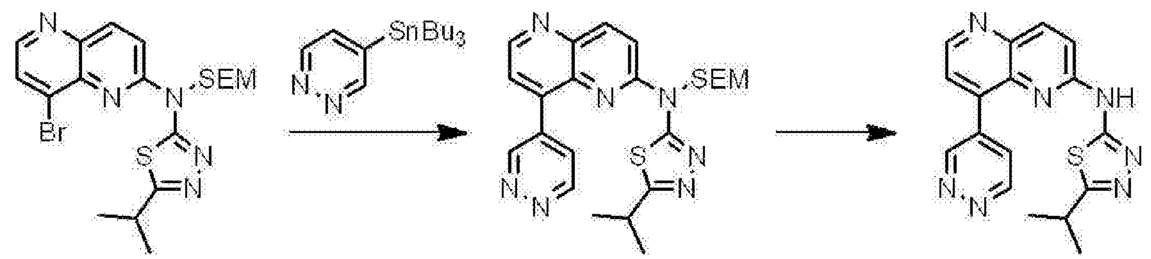

5-Isopropyl-N-(8-(pyridazin-4-yl)-1,5-naphthyridin-2-yl)-1,3,4-thiadiazole-2-amine was obtained as a pale yellow solid in the same manner as in Example 0281 except that 4-(tributyltin)pyridazine was used instead of the 2-(tributyltin)pyridine used in Example 0281.
$^1$H-NMR(DMSO-$d_6$)δ:12.15(1H,s),9.60-9.55(1H,m),9.49-9.45(1H,m),8.89(1H,d,J=4.3Hz),8.41(1H,d,J=8.9Hz),8.02-7.99(1H,m),7.80(1H,d,J=4.3Hz), 7.54(1H,d,J=9.2Hz),3.21-3.12(1H,m),1.23(6H,d,J=6.9Hz).
MSm/z(M+H):350.

<Example 0284> (not in accordance with the present invention)

<0284-1>

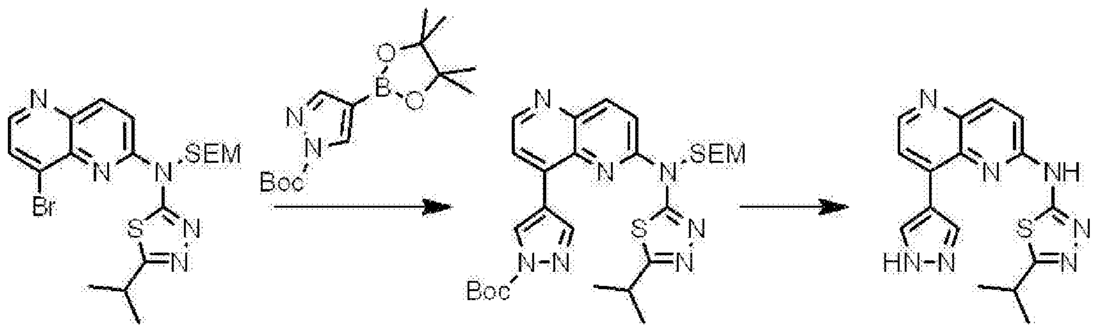

N-(8-(1H-pyrazol-4-yl)-1,5-naphthyridin-2-yl)-5-isopropyl-1,3,4-thiadiazole-2-amine was obtained as a pale yellow solid in the same manner as in Example 0278 except that 1-(tert-butoxycarbonyl)-4-(4,4,5,5-tetramethyl-1,3,2-dioxaborolan-2-yl)pyrazole was used instead of the 5-(4,4,5,5-tetramethyl-1,3,2-dioxaborolan-2-yl)pyrimidine used in Example 0278.
$^1$H-NMR(DMSO-$d_6$)δ:11.91(1H,s),8.70(1H,dd,J=4.5,3.1Hz),8.63(1H,s),8.30(1H,d,J=8.9Hz),7.76-7.69(2H,m),7.54(1H,dd,J=8.9,2.3Hz),3.32-3.21(1H,m),1.33(6H,d,J=6.9Hz).
MSm/z(M+H):338.

<Example 0285> (not in accordance with the present invention)

<0285-1>

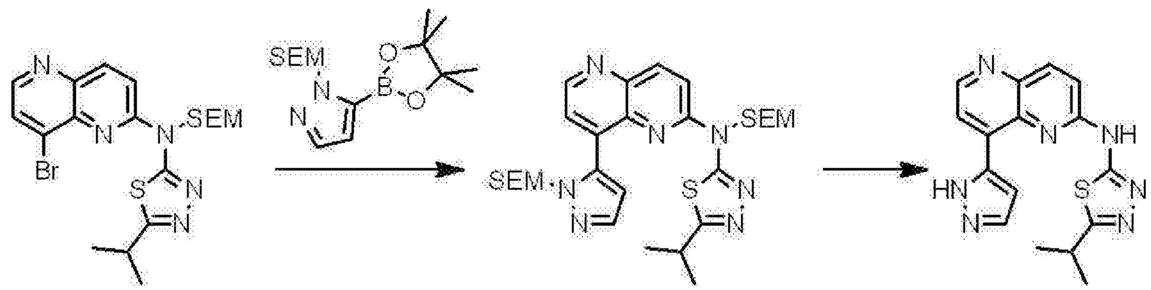

N-(8-(1H-pyrazol-5-yl)-1,5-naphthyridin-2-yl)-5-isopropyl-1,3,4-thiadiazole-2-amine was obtained as a pale yellow solid in the same manner as in Example 0278 except that 1-(2-(trimethylsilyl)ethoxy)methyl-5-(4,4,5,5-tetramethyl-1,3,2-dioxaborolan-2-yl)-1H-pyrazole was used instead of the 5-(4,4,5,5-tetramethyl-1,3,2-dioxaborolan-2-yl)pyrimidine used in Example 0278.
$^1$H-NMR(DMSO-$d_6$)δ:11.97(1H,s),8.76(1H,d,J=4.6Hz),8.36-8.31(1H,m),8.00(1H,d,J=2.3Hz),7.86(1H,d,J=4.3Hz),7.57-7.50(1H,m),7.13(1H,d,J=2.3Hz),3.25-3.20(1H,m),1.23(6H,d,J=6.9Hz).
MSm/z(M+H):338.

<Example 0286> (not in accordance with the present invention)

<0286-1>

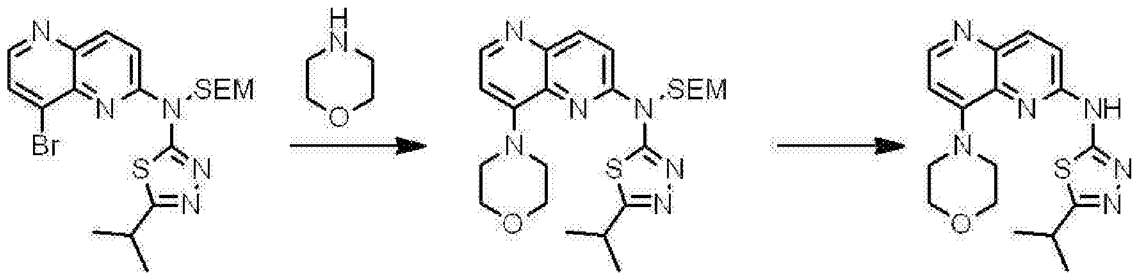

Morpholine (50 μL), tris(dibenzylideneacetone)palladium(0) (3.1 mg), 2-(dicyclohexylphosphino)-2',4',6'-triisopropylbiphenyl (5 mg), and cesium carbonate (30 mg) were added to a solution of N-(8-bromo-1,5-naphthyridin-2-yl)-5-isopropyl-N-((2-(trimethylsilyl)ethoxy)methyl)-1,3,4-thiadiazole-2-amine (15 mg) in 1,4-dioxane (1 mL), followed by stirring at 150°C for 30 minutes using a microwave reaction apparatus. Ethyl acetate and water were added to the reaction mixture. The organic layer was collected by separation, washed with a saturated sodium chloride aqueous solution, and dried over anhydrous sodium sulfate, and the solvent was distilled off under reduced pressure, thereby obtaining 5-isopropyl-N-(8-morpholino-1,5-naphthyridin-2-yl)-N-((2-(trimethylsilyl)ethoxy)methyl)-1,3,4-thiadiazole-2-amine.

Trifluoroacetic acid (1 mL) was added to the obtained 5-isopropyl-N-(8-morpholino-1,5-naphthyridin-2-yl)-N-((2-(trimethylsilyl)ethoxy)methyl)-1,3,4-thiadiazole-2-amine, followed by stirring at room temperature for 30 minutes. The solvent was distilled off under reduced pressure, and the obtained residue was purified by silica gel column chromatography (methanol-ethyl acetate, NH silica), thereby obtaining 5-isopropyl-N-(8-morpholino-1,5-naphthyridin-2-yl)-1,3,4-thiadiazole-2-amine (10 mg) as a yellow solid.

$^1$H-NMR(DMSO-$d_6$)δ:8.54(1H,d,J=5.0Hz),8.22(1H,d,J=8.9Hz),7.45(1H,d,J=9.2Hz),7.09(1H,d,J=5.3Hz),4.01-3.92(4H,m),3.47-3.38(5H,m),1.41(6H,d,J=6.9Hz).

MSm/z(M+H):357.

<Example 0287> (not in accordance with the present invention)

<0287-1>

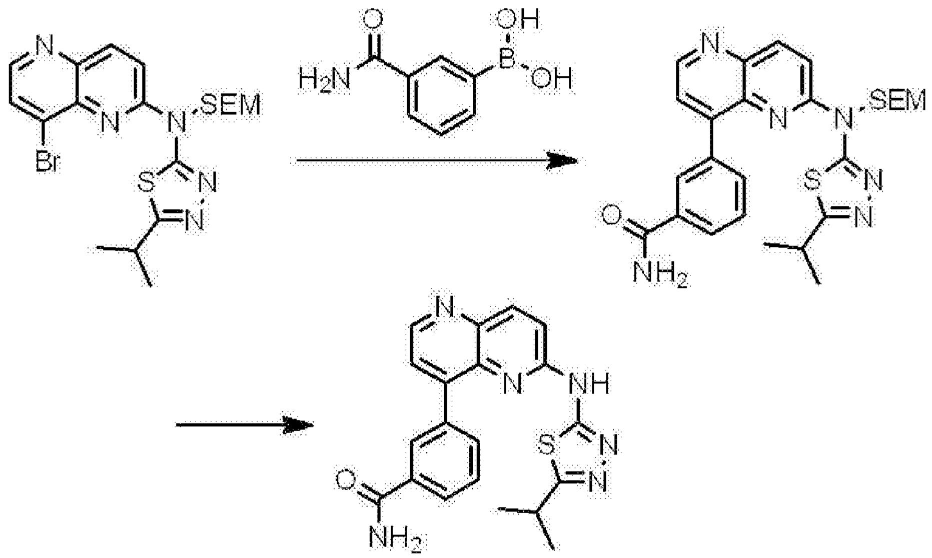

3-(6-((5-Isopropyl-1,3,4-thiadiazol-2-yl)amino)-1,5-naphthyridin-4-yl)benzamide was obtained as a white solid in the same manner as in Example 0278 except that (3-aminocarbonylphenyl)boronic acid was used instead of the 5-(4,4,5,5-tetramethyl-1,3,2-dioxaborolan-2-yl)pyrimidine used in Example 0278.

$^1$H-NMR(DMSO-$d_6$)δ:12.02(1H,s),8.82(1H,d,J=4.3Hz),8.37(1H,d,J=8.9Hz),8.19(1H,s),8.10-8.01(2H,m),7.79(1H,d,J=7.9Hz),7.69-7.63(2H,m),7.51(1H,d,J=9.2Hz),7.41(1H,s),3.15-3.08(1H,m),1.17(6H,d,J=6.9Hz).

MSm/z(M+H):391.

<Example 0288> (not in accordance with the present invention)

<0288-1>

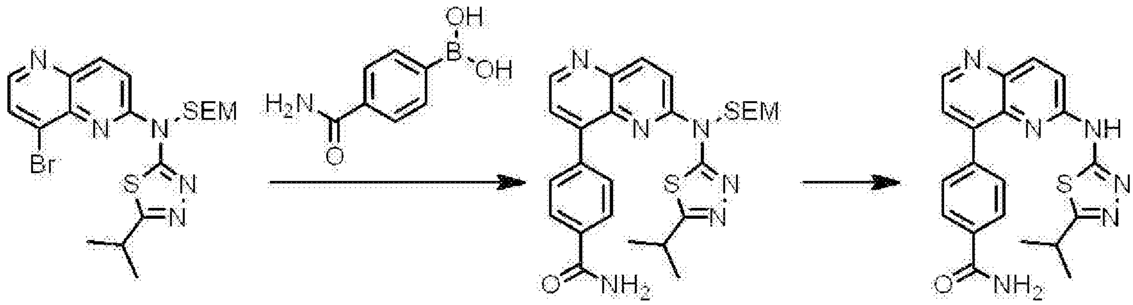

4-(6-((5-Isopropyl-1,3,4-thiadiazol-2-yl)amino)-1,5-naphthyridin-4-yl)benzamide was obtained as a white solid in the same manner as in Example 0278 except that (4-aminocarbonylphenyl)boronic acid was used instead of the 5-(4,4,5,5-tetramethyl-1,3,2-dioxaborolan-2-yl)pyrimidine used in Example 0278.
$^1$H-NMR(DMSO-$d_6$)δ:12.03(1H,s),8.82(1H,d,J=4.6Hz),8.36(1H,d,J=8.9Hz),8.10(2H,d,J=8.6Hz),7.70(2H,d,J=8.3Hz),7.64(1H,d,J=4.3Hz),7.50(1H,d,J=9.2Hz),7.46(2H,s),3.30-3.29(1H,m),1.17(6H,d,J=6.9Hz).
MSm/z(M+H):391.

<Example 0289> (not in accordance with the present invention)

<0289-1>

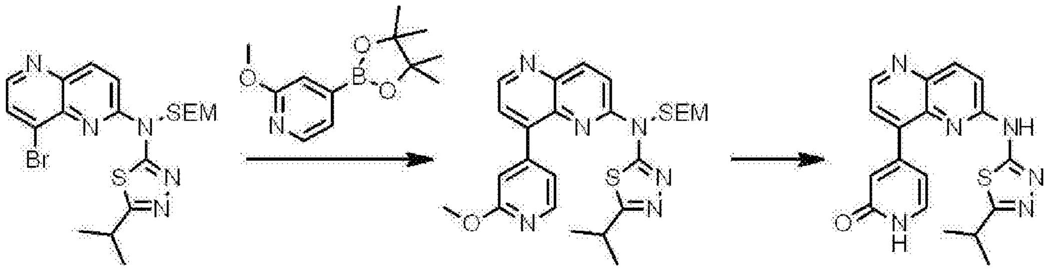

2-Methoxy-4-(4,4,5,5-tetramethyl-1,3,2-dioxaborolan-2-yl)pyridine (6 mg), bis(di-tert-butyl (4-dimethylaminophenyl)phosphine)dichloropalladium(II) (5 mg), and a 2 mol/L sodium carbonate aqueous solution (100 μL) were added to a solution of N-(8-bromo-1,5-naphthyridin-2-yl)-5-isopropyl-N-((2-trimethylsilyl)ethoxy)methyl)-1,3,4-thiadiazole-2-amine (10 mg) in 1,4-dioxane (1 mL), followed by stirring at 130°C for 45 minutes using a microwave reaction apparatus. The reaction mixture was cooled to room temperature, and ethyl acetate and water were added thereto. The organic layer was collected by separation, and dried over anhydrous sodium sulfate, and the solvent was distilled off under reduced pressure, thereby obtaining 5-isopropyl-N-(8-(2-methoxypyridin-4-yl)-1,5-naphthyridin-2-yl)-N-((2-(trimethylsilyl)ethoxy)methyl)-1,3,4-thiadiazole-2-amine.

Hydrobromic acid (1 mL) was added to the obtained 5-isopropyl-N-(8-(2-methoxypyridin-4-yl)-1,5-naphthyridin-2-yl)-N-((2-(trimethylsilyl)ethoxy)methyl)-1,3,4-thiadiazole-2-amine, followed by stirring at 80°C for 3 hours. The solvent was distilled off under reduced pressure, the resultant product was neutralized by the addition of a saturated sodium hydrogen carbonate aqueous solution, and ethyl acetate was added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-methanol, NH silica), thereby obtaining 4-(6-((5-isopropyl-1,3,4-thiadiazol-2-yl)amino)-1,5-naphthyridin-4-yl)pyridin-2(1H)-one (0.7 mg) as a pale yellow solid.
$^1$H-NMR(DMSO-d$_6$)δ:12.08(1H,s),8.81(1H,d,J=4.3Hz),8.36(1H,dd,J=9.2,2.0Hz),7.84(1H,d,J=6.9Hz),7.65(1H,d,J=4.6Hz),7.51(2H,dd,J=9.2,2.3Hz),6.67(1H,d,J=1.3Hz),6.46(1H,dd,J=6.9,2.0Hz),3.25-3.19(1H,m),1.26(6H,d,J=6.9Hz).
MSm/z(M+H):365.

<Example 0290>

<0290-1>

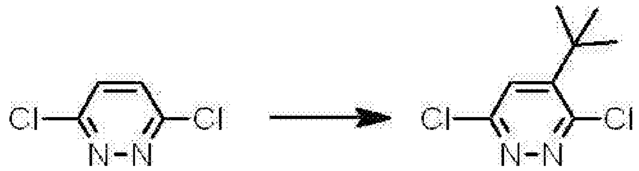

4-(tert-Butyl)-3,6-dichloropyridazine was obtained as pale yellow oily substance in the same manner as in Example 0014 except that pivalic acid was used instead of the isobutyric acid used in Example 0014.
$^1$H-NMR($CDCl_3$)δ:7.47(1H,s),1.49(9H,s).

<0290-2>

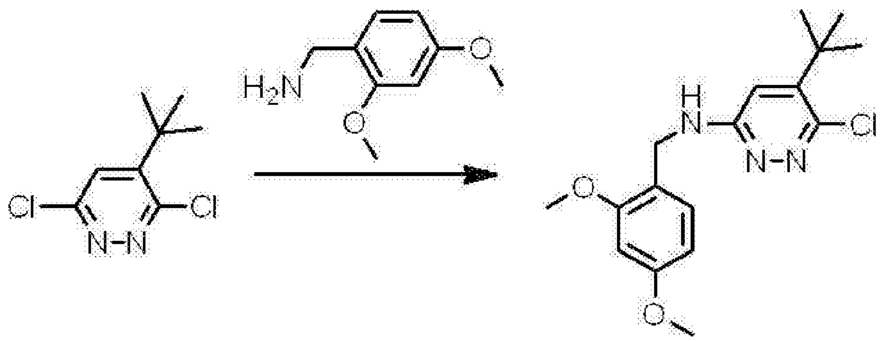

5-(tert-Butyl)-6-chloro-N-(2,4-dimethoxybenzyl)pyridazine-3-amine was obtained as a white solid in the same manner as in Example 0015 except that 4-(tert-butyl)-3,6-dichloropyridazine was used instead of the 3,6-dichloro-4-cyclobutylpyridazine used in Example 0015.
MSm/z(M+H):336.

<0290-3>

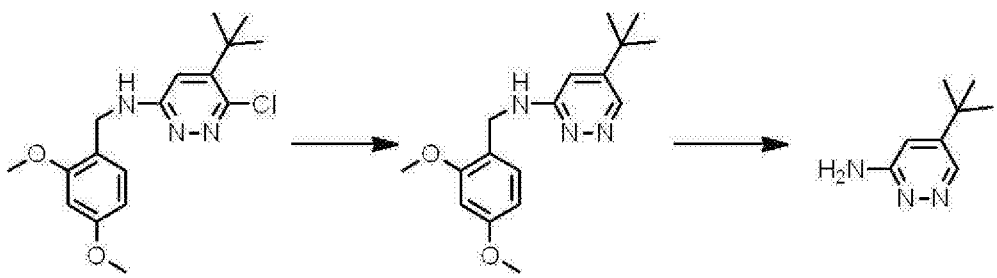

Acetic acid (400 μL) was added to a solution of 5-(tert-butyl)-6-chloro-N-(2,4-dimethoxybenzyl)pyridazine-3-amine (135 mg) in methanol (13 mL), and a hydrogenation reaction was performed using a flow-type hydrogenation reaction apparatus (30 bar, 1.0 mL/min, 55°C, 10% Pd/C). The solvent was distilled off under reduced pressure, thereby obtaining 5-(tert-butyl)-N-(2,4-dimethoxybenzyl)pyrazine-3 -amine.

Trifluoroacetic acid (1 mL) was added to the obtained 5-(tert-butyl)-N-(2,4-dimethoxybenzyl)pyrazine-3-amine, followed by stirring at room temperature for 30 minutes. The solvent was distilled off under reduced pressure, the resultant product was neutralized with a saturated sodium hydrogen carbonate aqueous solution, and chloroform was added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (chloroform-methanol, NH silica), thereby obtaining 5-(tert-butyl)pyridazine-3-amine (65 mg).
MSm/z(M+H):152.

<0290-4>

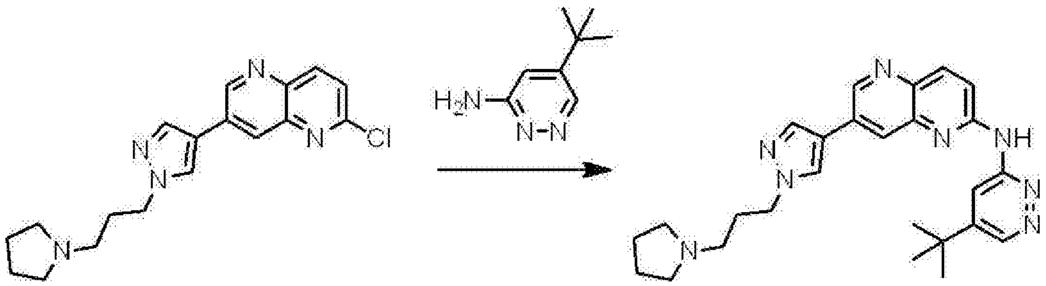

5-(tert-Butyl)pyridazine-3-amine (30 mg), 4,5-bis(diphenylphosphino)-9,9-dimethylxanthene (5 mg), cesium carbonate (45 mg), and tris(dibenzylideneacetone)dipalladium(0) (5 mg) were added to a solution of 2-chloro-7-(1-(3-(pyrrolidin-1-yl)propyl)-1H-pyrazol-4-yl)-1,5-naphthyridine (15 mg) in 1,4-dioxane (1 mL), followed by stirring at 150°C for 30 minutes using a microwave reaction apparatus. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-methanol, NH silica), thereby obtaining N-(5-(tert-butyl)pyridazin-3-yl)-7-(1-(3-(pyrrolidin-1-yl)propyl)-1H-pyrazol-4-yl)-1,5-naphthyridine-2-amine (1 mg) as a pale yellow solid.
$^1$H-NMR(DMSO-$d_6$)δ:10.68(1H,s),9.03(2H,dd,J=7.3,2.0Hz),8.76(1H,d,J=2.0Hz),8.49(1H,s),8.22(1H,d,J=9.2Hz),8.17(1H,s),8.11(1H,d,J=2.3Hz),7.75(1H,d,J=8.9Hz),4.22(2H,t,J=6.9Hz),2.46-2.35(6H,m),2.06-1.96(2H,m),1.73-1.65(4H,m),1.40(9H,s).
MSm/z(M+H):457.

<Example 0291>

<0291-1>

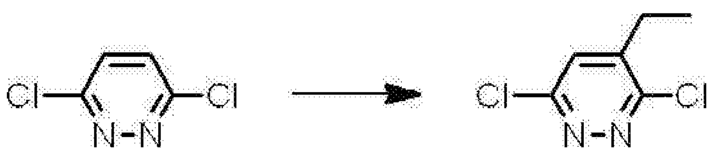

3,6-Dichloro-4-ethylpyridazine was obtained as pale yellow oily substance in the same manner as in Example 0014 except that propionic acid was used instead of the isobutyric acid used in Example 0014.
$^1$H-NMR($CDCl_3$)δ:7.38(1H,s),2.77(2H,q,J=7.2Hz),1.32(3H,t,J=7.2Hz).

<0291-2>

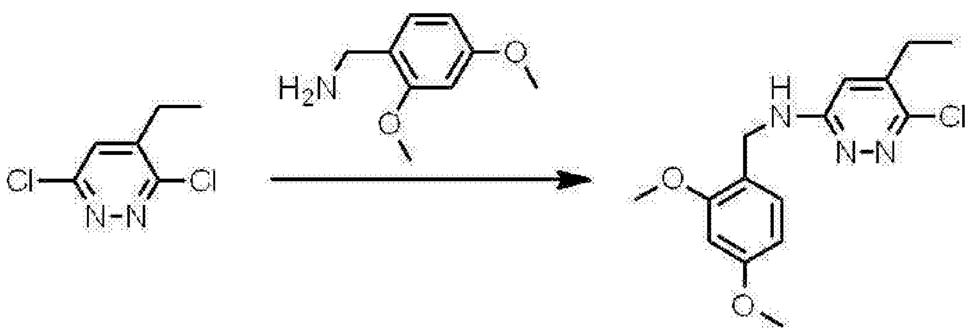

6-Chloro-N-(2,4-dimethoxybenzyl)-5-ethylpyridazine-3-amine was obtained as a brown solid in the same manner as in Example 0015 except that 3,6-dichloro-4-ethylpyridazine was used instead of the 3,6-dichloro-4-cyclobutylpyridazine used in Example 0015.
MSm/z(M+H):308.

<0291-3>

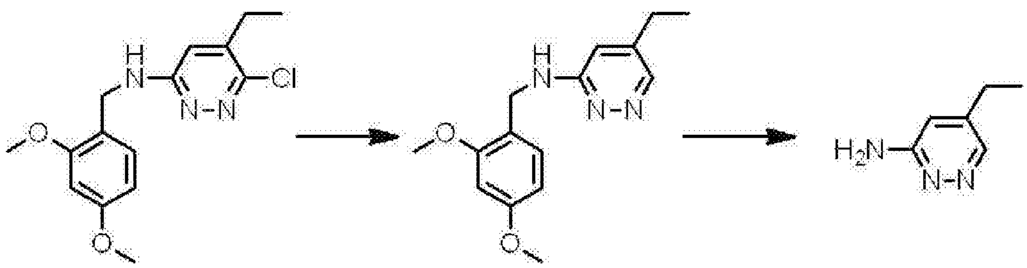

5-Ethylpyridazine-3-amine was obtained in the same manner as in Example 0290 except that 6-chloro-N-(2,4-dimethoxybenzyl)-5-ethylpyridazine-3-amine was used instead of the 6-chloro-N-(2,4-dimethoxybenzyl)-5-ethylpyridazine-3-amine used in Example 0290.
MSm/z(M+H):124.

<0291-4>

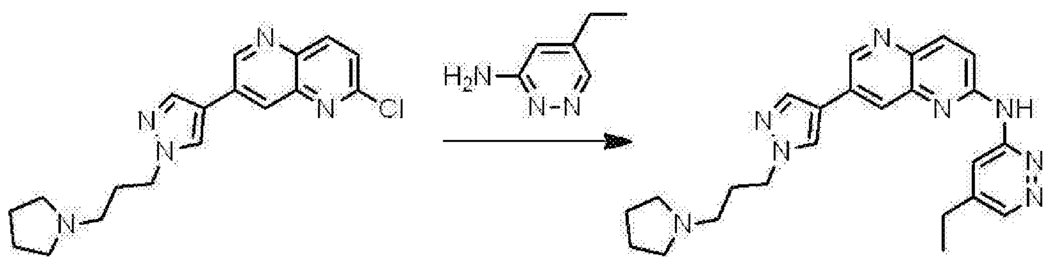

N-(5-ethylpyridazin-3-yl)-7-(1-(3-(pyrrolidin-1-yl)propyl)-1H-pyrazol-4-yl)-1,5-naphthyridine-2-amine was obtained in the same manner as in Example 0290 except that 5-ethylpyridazine-3-amine was used instead of the 5-(tert-butyl)pyridazine-3-amine used in Example 0290.
$^1$H-NMR(DMSO-$d_6$)$\delta$:10.70(1H,s),9.04(1H,d,J=2.0Hz),8.81(1H,d,J=2.0Hz),8.74(1H,s),8.51(1H,s),8.26(1H,d,J=1.7Hz),

8.23-8.17(2H,m),7.68(1H,d,J=9.2Hz),4.21(2H,t,J=6.9Hz),2.74(2H,q,J=7.7Hz),2.46-2.36(6H,m),2.05-1.96(2H,m),1.71-1.67(4H,m),1.31(3H,t,J=7.6Hz).
MSm/z(M+H):429.

<Example 0292>

<0292-1>

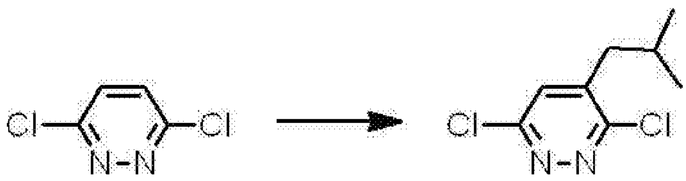

3,6-Dichloro-4-isobutylpyridazine was obtained as pale yellow oily substance in the same manner as in Example 0014 except that isovaleric acid was used instead of the isobutyric acid used in Example 0014.
$^1$H-NMR($CDCl_3$)δ:7.32(1H,s),2.60(2H,d,J=7.3Hz),2.12-1.98(1H,m),0.99(6H,d,J=6.6Hz).

<0292-2>

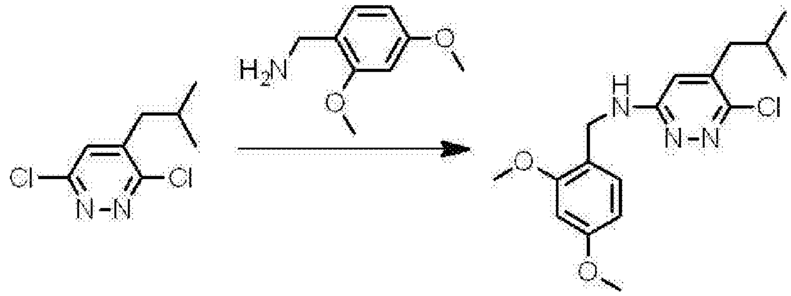

6-Chloro-N-(2,4-dimethoxybenzyl)-5-isobutylpyridazine-3-amine was obtained as a brown solid in the same manner as in Example 0015 except that 3,6-dichloro-4-isobutylpyridazine was used instead of the 3,6-dichloro-4-cyclobutylpyridazine used in Example 0015.
MSm/z(M+H):336.

<0292-3>

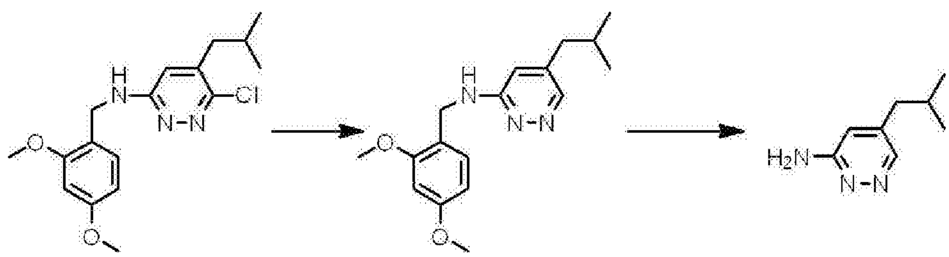

Acetic acid (2 mL) was added to a solution of 6-chloro-N-(2,4-dimethoxybenzyl)-5-isobutylpyridazine-3-amine (230 mg) in methanol (25 mL), and a hydrogenation reaction was performed using a flow-type hydrogenation reaction apparatus (20 bar, 1.0 mL/min, 50°C, 10% Pd/C). The solvent was distilled off under reduced pressure, thereby obtaining

N-(2,4-dimethoxybenzyl)-5-isobutylpyridazine-3 -amine.

Trifluoroacetic acid (2 mL) was added to the obtained N-(2,4-dimethoxybenzyl)-5-isobutylpyridazine-3-amine, followed by stirring at room temperature for 30 minutes. The solvent was distilled off under reduced pressure, the resultant product was neutralized with a saturated sodium hydrogen carbonate aqueous solution, and chloroform was added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (chloroform-methanol, NH silica), thereby obtaining 5-isobutylpyridazine-3-amine (55 mg).

MSm/z(M+H):152.

<0292-4>

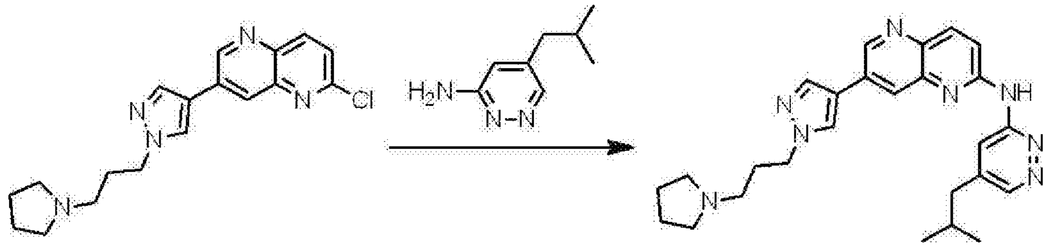

N-(5-isobutylpyridazin-3-yl)-7-(1-(3-(pyrrolidin-1-yl)propyl)-1H-pyrazol-4-yl)-1,5-naphthyridine-2-amine was obtained in the same manner as in Example 0290 except that 5-isobutylpyridazine-3-amine was used instead of the 5-(tert-butyl)pyridazine-3-amine used in Example 0290.

$^1$H-NMR(DMSO-$d_6$)δ:10.69(1H,s),9.04(1H,d,J=2.0Hz),8.76(1H,d,J=2.0Hz),8.64(1H,s),8.49(1H,s),8.21(2H,dd,J=5.4,3.5 Hz),8.17(1H,s),7.69(1H,d,J=9.2Hz),4.22(2H,t,J=6.9Hz),2.60(2H,d,J=7.3Hz),2.44-2.36(6H,m),2.04-1.98(3H,m),1.72-1.65(4H,m),0.99(6H,d,J=6.6Hz).

MSm/z(M+H):457.

<Example 0293>

<0293-1>

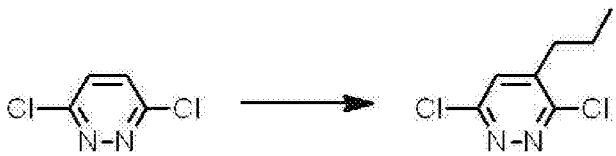

3,6-Dichloro-4-propylpyridazine was obtained as pale yellow oily substance in the same manner as in Example 0014 except that butyric acid was used instead of the isobutyric acid used in Example 0014.

$^1$H-NMR($CDCl_3$)δ:7.36(1H,s),2.70(2H,t,J=7.8Hz),1.79-1.65(2H,m),1.04(3H,t,J=7.2Hz).

<0293-2>

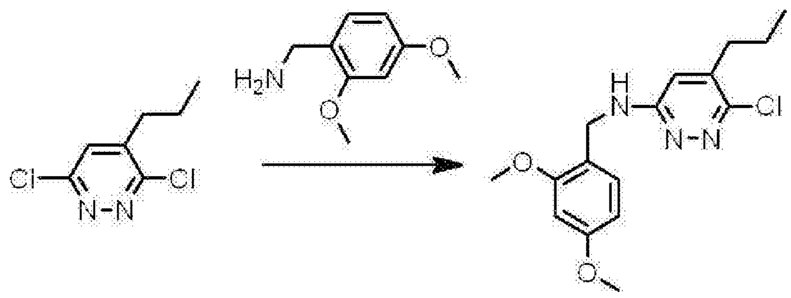

N-(2,4-dimethoxybenzyl)-5-propylpyridazine-3-amine was obtained as a brown solid in the same manner as in Example 0015 except that 3,6-dichloro-4-propylpyridazine was used instead of the 3,6-dichloro-4-cyclobutylpyridazinee used in Example 0015.
MSm/z(M+H):322.

<0293-3>

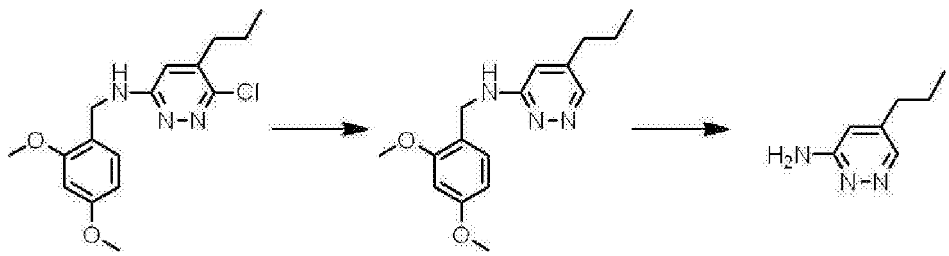

5-Propylpyridazine-3-amine was obtained in the same manner as in Example 0292 except that N-(2,4-dimethoxybenzyl)-5-propylpyridazine-3-amine was used instead of the 6-chloro-N-(2,4-dimethoxybenzyl)-5-ethylpyridazine-3-amine used in Example 0292.
MSm/z(M+H):138.

<0293-4>

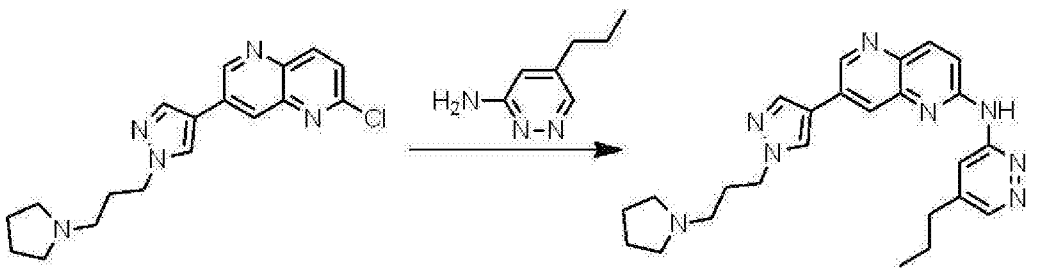

N-(5-propylpyridazin-3-yl)-7-(1-(3-(pyrrolidin-1-yl)propyl)-1H-pyrazol-4-yl)-1,5-naphthyridine-2-amine was obtained in the same manner as in Example 0290 except that 5-propylpyridazine-3-amine was used instead of the 5-(tert-butyl)pyridazine-3-amine used in Example 0290.
$^1$H-NMR(DMSO-$d_6$)$\delta$:10.69(1H,s),9.04(1H,d,J=2.0Hz),8.79(1H,d,J=2.0Hz),8.69(1H,s),8.51(1H,s),8.25(1H,d,J=1.3Hz), 8.21(1H,d,J=9.2Hz),8.19(1H,s),7.68(1H,d,J=9.2Hz),4.22(2H,t,J=6.9Hz),2.69(2H,t,J=7.6Hz),2.40(6H,t, J=7.1Hz),2.00(2H,dd,J=9.1,5.1Hz),1.76-1.67(6H,m),0.99(3H,t,J=7.4Hz).
MSm/z(M+H):443.

<Example 0294>

<0294-1>

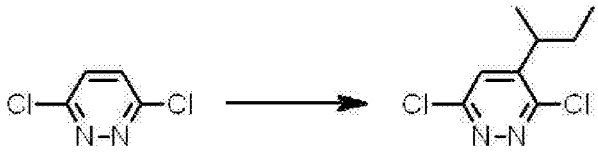

4-(sec-Butyl)-3,6-dichloropyridazine was obtained as pale yellow oily substance in the same manner as in Example 0014 except that 2-methylbutyric acid was used instead of the isobutyric acid used in Example 0014.
$^1$H-NMR($CDCl_3$)$\delta$:7.34(1H,s),3.07(1H,td,J=13.7,6.9Hz),1.78-1.54(2H,m),1.28(3H,d,J=6.9Hz),0.94(3H,t,J=7.4Hz).

<0294-2>

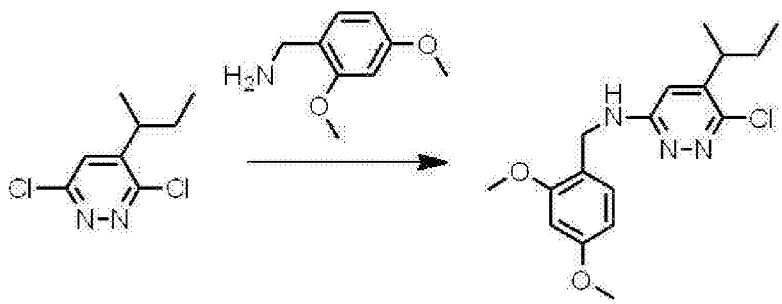

5-(sec-Butyl)-6-chloro-N-(2,4-dimethoxybenzyl)pyridazine-3-amine was obtained as a brown solid in the same manner as in Example 0015 except that 4-(sec-butyl)-3,6-dichloropyridazine was used instead of the 3,6-dichloro-4-cyclobutylpyridazine used in Example 0015.
MSm/z(M+H):336.

<0294-3>

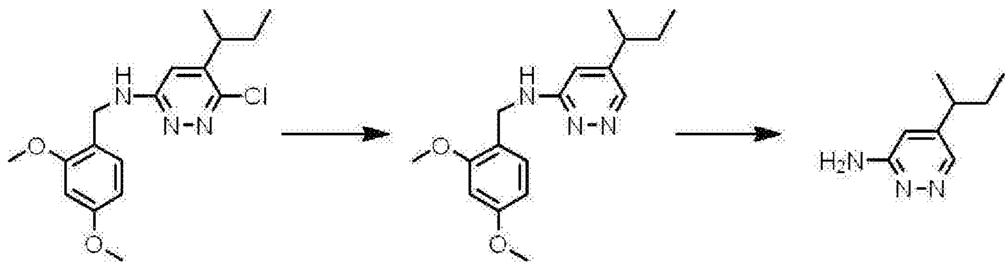

5-(sec-Butyl)pyridazine-3-amine was obtained in the same manner as in Example 0292 except that 5-(sec-butyl)-6-chloro-N-(2,4-dimethoxybenzyl)pyridazine-3-amine was used instead of the 6-chloro-N-(2,4-dimethoxybenzyl)-5-ethylpyridazine-3-amine used in Example 0292.
MSm/z(M+H):152.

<0294-4>

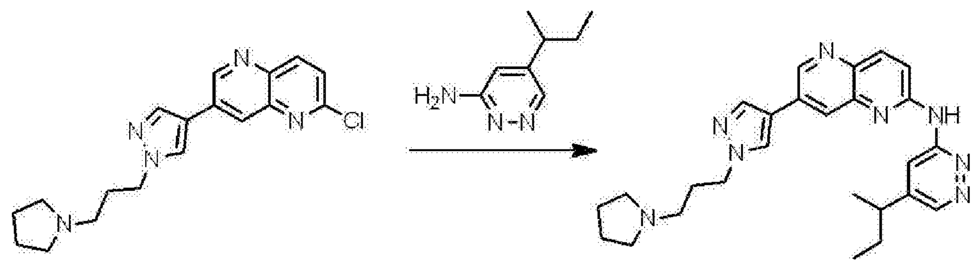

N-(5-(sec-butyl)pyridazin-3-yl)-7-(1-(3-(pyrrolidin-1-yl)propyl)-1H-pyrazol-4-yl)-1,5-naphthyridine-2-amine was obtained in the same manner as in Example 0290 except that 5-(sec-butyl)pyridazine-3-amine was used instead of the 5-(tert-butyl)pyridazine-3-amine used in Example 0290.
$^1$H-NMR(DMSO-$d_6$)δ:10.69(1H,s),9.04(1H,d,J=2.0Hz),8.82(1H,d,J=2.0Hz),8.68(1H,d,J=1.7Hz),8.50(1H,s),8.21(3H,dd, J=8.1,5.8Hz),7.70(1H,d,J=9.2Hz),4.22(2H,t,J=6.9Hz),2.81-2.73(1H,m),2.46-2.37(6H,m),2.04-1.96(2H,m),1.74-1.65(6 H,m),1.32(3H,d,J=6.9Hz),0.88(3H,t,J=7.3Hz).
MSm/z(M+H):457.

<Example 0295>

<0295-1>

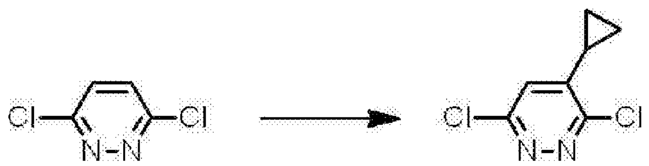

4-Cyclopropyl-3,6-dichloropyridazine was obtained as a white solid in the same manner as in Example 0014 except that cyclopropanecarboxylic acid was used instead of the isobutyric acid used in Example 0014.
$^1$H-NMR($CDCl_3$)δ:6.94(1H,s),2.25-2.17(1H,m),1.34-1.27(2H,m),0.90-0.83(2H,m).

<0295-2>

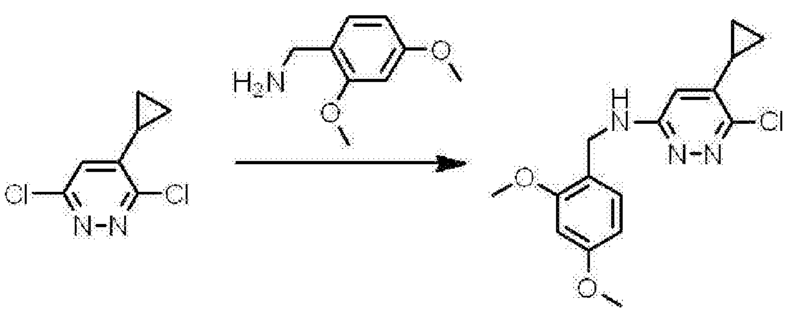

6-Chloro-5-cyclopropyl-N-(2,4-dimethoxybenzyl)pyridazine-3-amine was obtained in the same manner as in Example 0015 except that 4-cyclopropyl-3,6-dichloropyridazine was used instead of the 3,6-dichloro-4-cyclobutylpyridazine used in Example 0015.
MSm/z(M+H):320.

<0295-3>

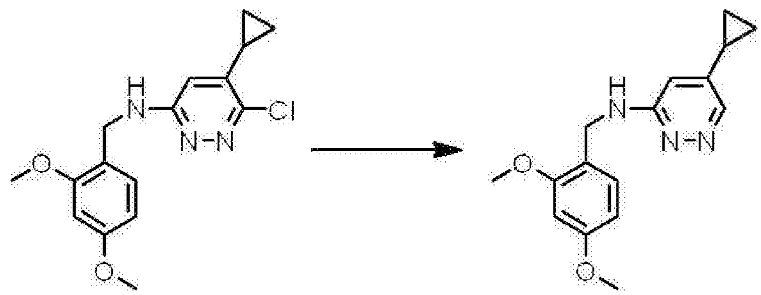

Ammonium formate (120 mg), triethylamine (200 μL), and tetrakis(triphenylphosphine)palladium(0) (50 mg) were added to a solution of 6-chloro-5-cyclopropyl-N-(2,4-dimethoxybenzyl)pyridazine-3-amine (300 mg) in 1,4-dioxane (1.2 mL), followed by stirring at 100°C for 3 hours in a nitrogen atmosphere. The reaction mixture was cooled to room temperature, and ethyl acetate was added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-methanol, NH silica), thereby obtaining 5-cyclopropyl-N-(2,4-dimethoxybenzyl)pyridazine-3-amine (235 mg).
MSm/z(M+H):286.

<0295-4>

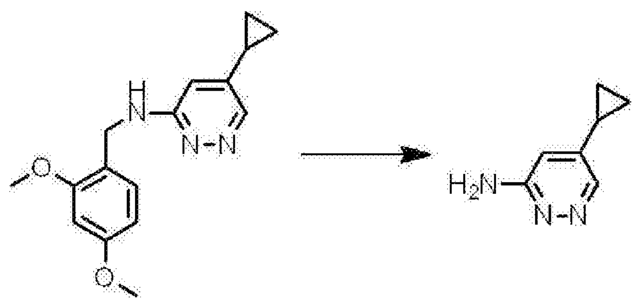

Trifluoroacetic acid (3 mL) was added to 5-cyclopropyl-N-(2,4-dimethoxybenzyl)pyridazine-3-amine (235 mg), followed by stirring at room temperature for 30 minutes. The solvent was distilled off under reduced pressure, the resultant product was neutralized with a saturated sodium hydrogen carbonate aqueous solution, and chloroform was added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (chloroform-methanol, NH silica), thereby obtaining 5-cyclopropylpyridazine-3-amine (105 mg).
MSm/z(M+H):136.

<0295-5>

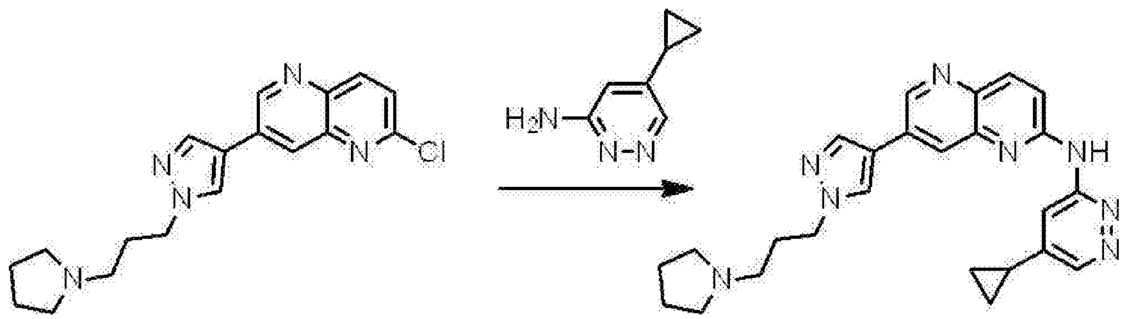

N-(5-cyclopropylpyridazin-3-yl)-7-(1-(3-(pyrrolidin-1-yl)propyl)-1H-pyrazol-4-yl)-1,5-naphthyridine-2-amine (7 mg) was obtained in the same manner as in Example 0290 except that 5-cyclopropylpyridazine-3-amine was used instead of the 5-(tert-butyl)pyridazine-3-amine used in Example 0290.
$^1$H-NMR(DMSO-$d_6$)δ:10.63(1H,s),9.04(1H,d,J=2.3Hz),8.64(1H,d,J=2.0Hz),8.60(1H,d,J=2.0Hz),8.51(1H,s),8.25(1H,d,J =1.7Hz),8.20(2H,d,J=9.2Hz),7.66(1H,d,J=9.2Hz),4.22(2H,t,J=6.9Hz),2.47-2.38(6H,m),2.13-1.97(3H,m),1.73-1.64(4H, m),1.23-1.17(2H,m),1.04-0.96(2H,m).
MSm/z(M+H):441.

<Example 0296>

<0296-1>

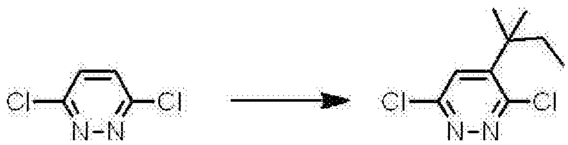

3,6-Dichloro-4-(tert-pentyl)pyridazine was obtained as pale yellow oily substance in the same manner as in Example 0014 except that 2,2-dimethylbutyric acid was used instead of the isobutyric acid used in Example 0014.
$^1$H-NMR($CDCl_3$)δ:7.42(1H,s),2.06-1.97(2H,m),1.45(6H,d,J=3.3Hz),0.74-0.66(3H,m).

<0296-2>

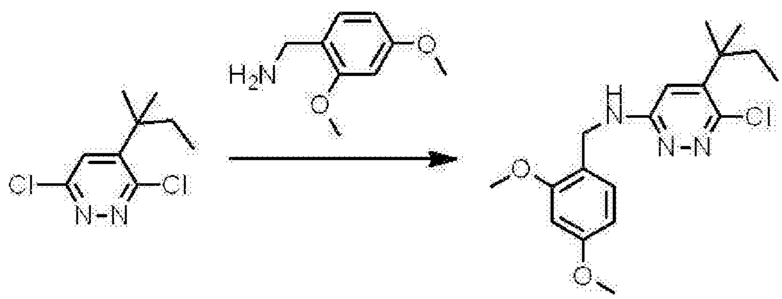

6-Chloro-N-(2,4-dimethoxybenzyl)-5-(tert-pentyl)pyridazine-3-amine was obtained as a brown solid in the same manner as in Example 0015 except that 3,6-dichloro-4-(tert-pentyl)pyridazine was used instead of the 3,6-dichloro-4-cyclobutylpyridazine used in Example 0015.
MSm/z(M+H):350.

<0296-3>

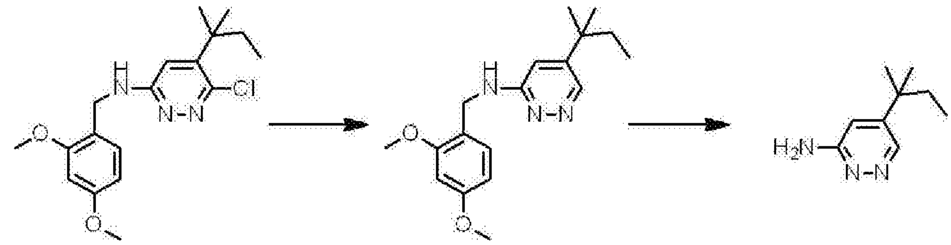

5-(tert-Pentyl)pyridazine-3-amine was obtained in the same manner as in Example 0292 except that 6-chloro-N-(2,4-dimethoxybenzyl)-5-(tert-pentyl)pyridazine-3-amine was used instead of the 6-chloro-N-(2,4-dimethoxybenzyl)-5-ethylpyridazine-3-amine used in Example 0292.
MSm/z(M+H):166.

<0296-4>

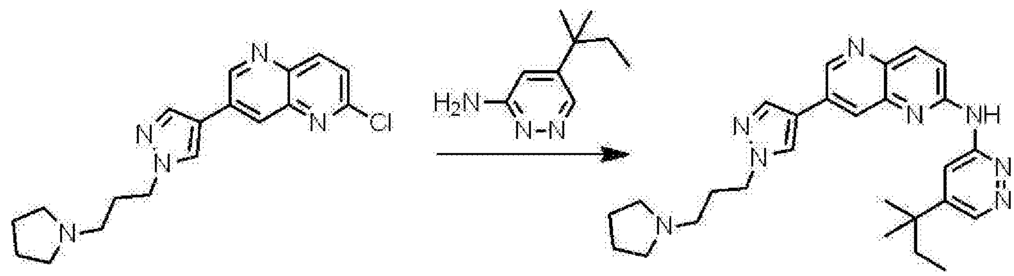

N-(5-(tert-pentyl)pyridazin-3-yl)-7-(1-(3-(pyrrolidin-1-yl)propyl)-1H-pyrazol-4-yl)-1,5-naphthyridine-2-amine was obtained in the same manner as in Example 0290 except that 5-(tert-pentyl)pyridazine-3-amine was used instead of the 5-(tert-butyl)pyridazine-3-amine used in Example 0290.
$^1$H-NMR(DMSO-$d_6$)δ:10.68(1H,s),9.04(1H,d,J=2.0Hz),8.95(1H,d,J=2.0Hz),8.72(1H,d,J=2.0Hz),8.49(1H,s),8.23(1H,d,J=9.2Hz),8.16(1H,s),8.09(1H,d,J=1.3Hz),7.76(1H,d,J=9.2Hz),4.22(2H,t,J=7.1Hz),2.45-2.37(6H,m),2.04-1.97(2H,m),1.79-1.68(6H,m),1.37(6H,s),0.74(3H,t,J=7.4Hz).
MSm/z(M+H):471.

<Example 0297>

<0297-1>

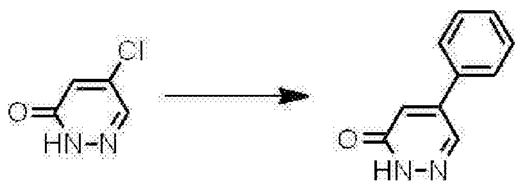

Phenylboronic acid (60 mg), bis(di-tert-butyl (4-dimethylaminophenyl)phosphine)dichloropalladium(II) (5 mg), and a 2 mol/L sodium carbonate aqueous solution (50 μL) were added to a solution of 5-chloropyridazin-3(2H)-one (50 mg) in 1,4-dioxane (1 mL), followed by stirring at 130°C for 30 minutes using a microwave reaction apparatus. The reaction mixture was cooled to room temperature, and ethyl acetate was added thereto. The organic layer was collected

by separation, washed with a saturated sodium chloride aqueous solution, and dried over anhydrous sodium sulfate, and the solvent was distilled off under reduced pressure. The obtained residue was purified by silica gel column chromatography (ethyl acetate-methanol, NH silica), thereby obtaining 5-phenylpyridazin-3(2H)-one (25 mg).
$^1$H-NMR(DMSO-$d_6$)δ:13.12(1H,s),8.31(1H,d,J=2.3Hz),7.85-7.79(2H,m),7.56-7.51(3H,m),7.14(1H,s).

<0297-2>

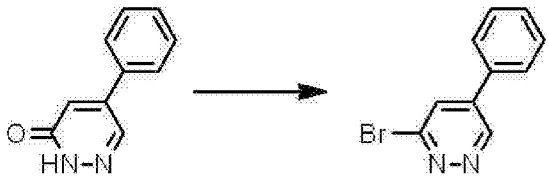

A mixture of 5-phenylpyridazin-3(2H)-one (25 mg) and phosphorous oxybromide (1 g) was stirred at 120°C for 30 minutes. The reaction mixture was added dropwise to a mixture solution of methanol-water (1:10), the resultant product was neutralized by the addition of a sodium hydroxide aqueous solution, and ethyl acetate was added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate), thereby obtaining 3-bromo-5-phenylpyridazine (25 mg).
$^1$H-NMR($CDCl_3$)δ:9.40(1H,d,J=2.0Hz),7.85(1H,d,J=2.0Hz),7.69-7.64(2H,m),7.61-7.53(3H,m).

<0297-3>

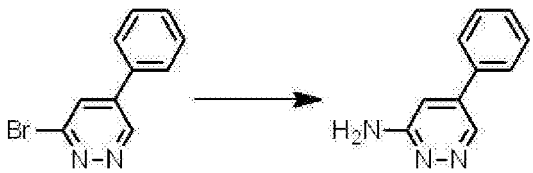

25% ammonia water (1 mL) and copper(I) oxide (10 mg) were added to a solution of 3-bromo-5-phenylpyridazine (60 mg) in ethylene glycol (1 mL), followed by stirring at 150°C for 45 minutes using a microwave reaction apparatus. The reaction mixture was cooled to room temperature, neutralized with 2 mol/L hydrochloric acid, and the resultant product was extracted three times with ethyl acetate. The organic layers were combined, washed with a saturated sodium chloride aqueous solution, and dried over anhydrous sodium sulfate, and the solvent was distilled off under reduced pressure. The obtained residue was purified by silica gel column chromatography (ethyl acetate-methanol, NH silica), thereby obtaining 5-phenylpyridazine-3-amine (45 mg).
MSm/z(M+H):172.

<0297-4>

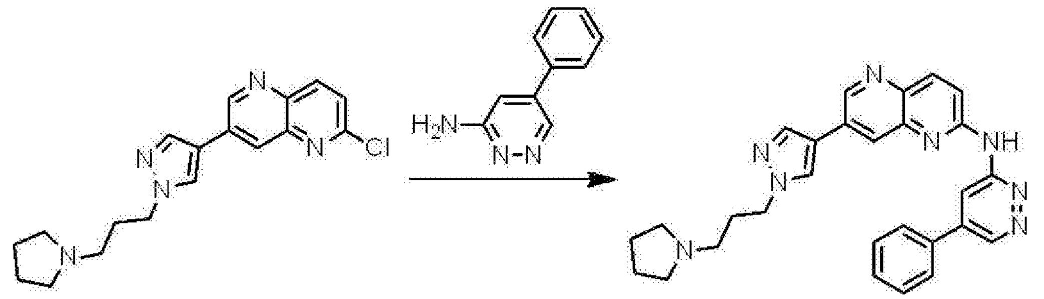

N-(5-phenylpyridazin-3-yl)-7-(1-(3-(pyrrolidin-1-yl)propyl)-1H-pyrazol-4-yl)-1,5-naphthyridine-2-amine was obtained in the same manner as in Example 0290 except that 5-phenylpyridazine-3-amine was used instead of the 5-(tert-butyl)pyridazine-3-amine used in Example 0290.
$^1$H-NMR(DMSO-$d_6$)δ:10.87(1H,s),9.28(1H,d,J=2.0Hz),9.06(2H,d,J=2.0Hz),8.52(1H,s),8.24(3H,dd,J=9.9,8.3Hz),7.97(2H,dd,J=8.3,1.3Hz),7.74(1H,d,J=9.2Hz),7.68-7.58(3H,m),4.21(2H,t,J=6.9Hz),2.45-2.36(6H,m),2.05-1.95(2H,m),1.70-1.66(4H,m).
MSm/z(M+H):477.

<Example 0298>

<0298-1>

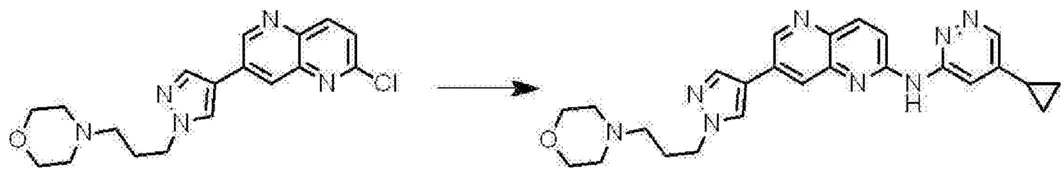

N-(5-cyclopropylpyridazin-3-yl)-7-(1-(3-morpholinopropyl)-1H-pyrazol-4-yl)-1,5-naphthyridine-2-amine was obtained as a white solid in the same manner as in Example 0001 except that 5-cyclopropylpyridazine-3-amine was used instead of the 1,3,4-thiadiazole-2-amine used in Example 0001.
$^1$H-NMR($CDCl_3$)δ:8.93(1H,d,J=1.8Hz),8.88(1H,brs),8.68-8.62(2H,m),8.23(1H,d,J=9.3Hz),8.09(1H,m),7.97(1H,s),7.87(1H,s),7.51(1H,d,J=9.3Hz),4.31(2H,m),3. 73(4H,m),2.50-2.34(6H,m),2.13(2H,m),2.01(1H,m),1.27(2H,m),1.03(2H,m).
MSm/z(M+H):457.

<Example 0299>

<0299-1>

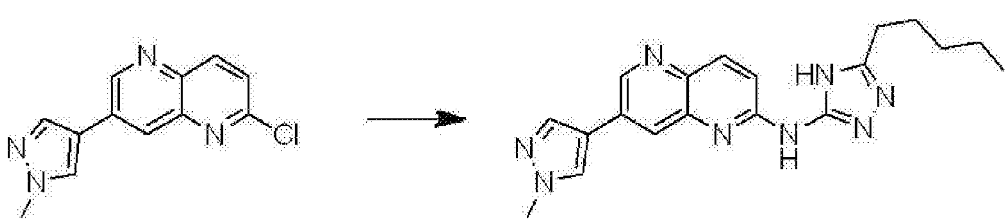

A mixture of 2-chloro-7-(1-methyl-1H-pyrazol-4-yl)-1,5-naphthyridine (45 mg), 5-pentyl-4H-1,2,4-triazole-3-amine (56.7 mg), tris(dibenzylideneacetone)dipalladium(0) (21.2 mg), 2-dicyclohexylphosphino-2',4',6'-triisopropylbiphenyl (35.1 mg), cesium carbonate (120 mg), and 1,4-dioxane (1 mL) was stirred at 100°C for 2 hours in a nitrogen atmosphere in a sealed tube. The reaction mixture was cooled to room temperature, purified by silica gel column

chromatography (chloroform-methanol, NH silica), and purified by preparative thin layer silica gel chromatography (chloroform-methanol), thereby obtaining 7-(1-methyl-1H-pyrazol-4-yl)-N-(5-pentyl-4H-1,2,4-triazol-3-yl)-1,5-naphthyridine-2-amine (4.5 mg) as a yellow solid.
$^1$H-NMR(DMSO-$d_6$)δ:13.16(1H,s),11.19(1H,s),9.00(1H,d,J=1.7Hz),8.79(1H,d,J=1.7Hz),8.36(1H,s),8.18(1H,d,J=9.2Hz),8.04(1H,s),7.28(1H,d,J=9.2Hz),3.94(3H,s),2.58(2H,t,J=7.6Hz),1.78-1.60(2H,m),1.42-1.25(4H,m),0.97-0.80(3H,m).
MSm/z(M+H):363.

<Example 0300>

<0300-1>

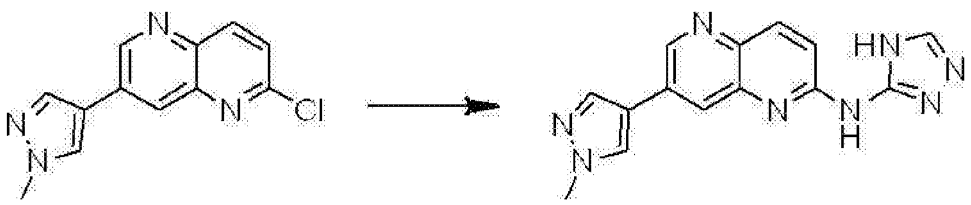

7-(1-Methyl-1H-pyrazol-4-yl)-N-(4H-1,2,4-triazol-3-yl)-1,5-naphthyridine-2-amine was obtained as a yellow solid in the same manner as in Example 0299 except that 4H-1,2,4-triazole-3-amine was used instead of the 5-pentyl-4H-1,2,4-triazole-3-amine used in Example 0299.
$^1$H-NMR(DMSO-$d_6$)δ:13.56(1H,s),11.28(1H,s),9.02(1H,d,J=2.0Hz),8.83(1H,d,J=2.0Hz),8.36(1H,s),8.20(1H,d,J=8.6Hz),8.05(1H,s),7.78(1H,d,J=1.3Hz),7.32(1H,d,J=8.6Hz),3.94(3H,s).
MSm/z(M+H):293.

<Example 0301>

<0301-1>

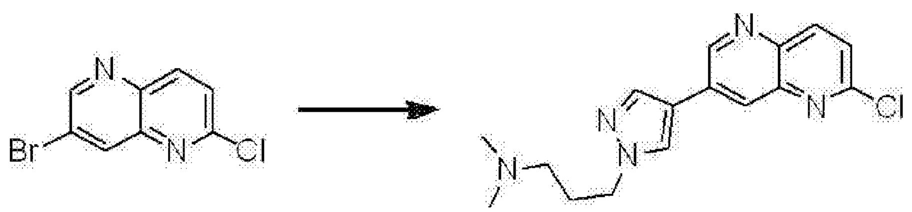

A mixture of 7-bromo-2-chloro-1,5-naphthyridine (0.50 g), N,N-dimethyl-3-(4-(4,4,5,5-tetramethyl-1,3,2-dioxaborolan-2-yl)-1H-pyrazol-1-yl)propane-1-amine (0.69 g), bis(di-tert-butyl (4-dimethylaminophenyl)phosphine)dichloropalladium (II) (0.145 g), sodium carbonate (0.43 g), 1,4-dioxane (3 mL), and water (0.3 mL) was stirred at 100°C for 2 hours in a nitrogen atmosphere in a sealed tube. The reaction mixture was cooled to room temperature, and purified by silica gel column chromatography (chloroform-methanol, NH silica), thereby obtaining 3-(4-(6-chloro-1,5-naphthyridin-3-yl)-1H-pyrazol-1-yl)-N,N-dimethylpropane-1-amine (0.35 g) as a yellow solid.
$^1$H-NMR(DMSO-$d_6$)δ:9.35(1H,d,J=2.0Hz),8.59(1H,s),8.54(1H,d,J=2.0Hz),8.43(1H,d,J=8.9Hz),8.26(1H,s),7.75(1H,d,J=8.9Hz),4.19(2H,t,J=6.9Hz),2.22(2H,t,J=6.9Hz),2.14(6H,s),2.05-1.87(2H,m).

<0301-2>

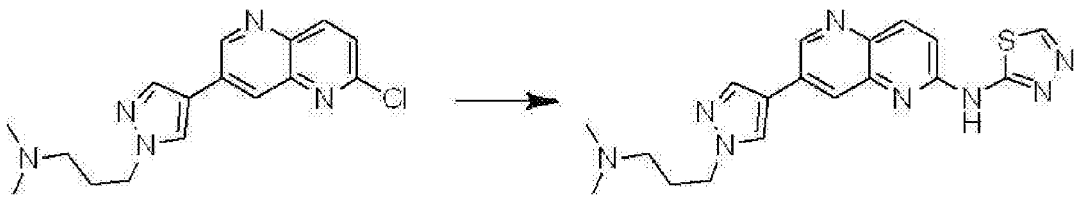

A mixture of 3-(4-(6-chloro-1,5-naphthyridin-3-yl)-1H-pyrazol-1-yl)-N,N-dimethylpropane-1-amine (50 mg), 1,3,4-thiadiazole-2-amine (17.6 mg), tris(dibenzylideneacetone)dipalladium(0) (2.9 mg), 4,5-bis(diphenylphosphino)-9,9-dimethylxanthene (3.7 mg), cesium carbonate (103 mg), and 1,4-dioxane (1 mL) was stirred at 150°C for 30 minutes in a nitrogen atmosphere using a microwave reaction apparatus. The reaction mixture was cooled to room temperature, and purified by silica gel column chromatography (chloroform-methanol, NH silica), thereby obtaining N-(7-(1-(3-(dimethylamino)propyl)-1H-pyrazol-4-yl)-1,5-naphthyridin-2-yl)-1,3,4-thiadiazole-2-amine (26.4 mg) as a yellow solid.
$^1$H-NMR(DMSO-$d_6$)$\delta$:12.23(1H,brs),9.17(1H,s),9.10(1H,d,J=2.0Hz),8.57(1H,s),8.34(1H,d,J=2.0Hz),8.28(1H,d,J=8.6Hz ),8.21(1H,s),7.44(1H,d,J=8.6Hz),4.19(2H,t,J=6.9Hz),2.22(2H,t,J=6.9Hz),2.14(6H,s),2.05-1.89(2H,m).
MSm/z(M+H):381.

<Example 0302>

<0302-1>

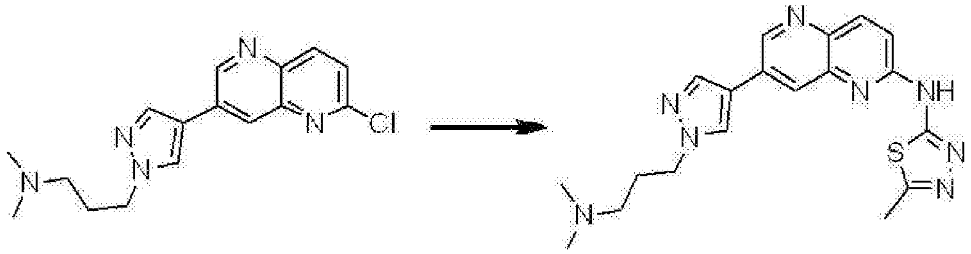

N-(7-(1-(3-(dimethylamino)propyl)-1H-pyrazol-4-yl)-1,5-naphthyridin-2-yl)-5-methyl-1,3,4-thiadiazole-2-amine was obtained as a white solid in the same manner as in Example 0301 except that 5-methyl-1,3,4-thiadiazole-2-amine was used instead of the 1,3,4-thiadiazole-2-amine used in Example 0301.
$^1$H-NMR(DMSO-$d_6$)$\delta$:12.03(1H,brs),9.08(1H,d,J=2.0Hz),8.58(1H,s),8.33(1H,d,J=2.0Hz),8.25(1H,d,J=8.9Hz),8.22(1H,s ),7.39(1H,d,J=8.9Hz),4.19(2H,t,J=6.9Hz),2.66(3H,s),2.22(2H,t,J=6.9Hz),2.15(6H,s),2.05-1.89(2H,m).
MSm/z(M+H):395.

<Example 0303>

<0303-1>

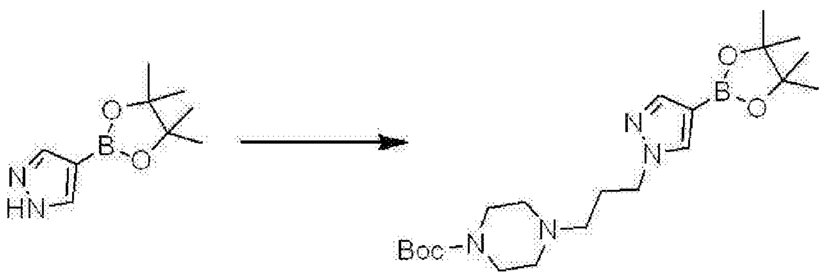

A mixture of 4-(4,4,5,5-tetramethyl-1,3,2-dioxaborolan-2-yl)-1H-pyrazole (0.473 g), tert-butyl 4-(3-bromopropyl)piperazine-1-carboxylate (0.818 g), potassium carbonate (0.61 g), and N,N-dimethylformamide (5 mL) was stirred at 80°C for 3 hours in a nitrogen atmosphere. After the reaction mixture was cooled to room temperature, diisopropyl ether was added thereto, the solid matter was filtered off, and the solvent was distilled off under reduced pressure. The obtained residue was purified by silica gel column chromatography (chloroform-methanol), thereby obtaining tert-butyl 4-(3-(4-(4,4,5,5-tetramethyl-1,3,2-dioxaborolan-2-yl)-1H-pyrazol-1-yl)propyl)piperazine-1-carboxylate (0.78 g) as yellow oily substance.
$^1$H-NMR($CDCl_3$)$\delta$:7.56(1H,s),7.45(1H,s),4.19(2H,t,J=6.9Hz),3.49-3.33(4H,m),2.44-2.26(6H,m),2.13-1.93(2H,m),1.51-1.37 (21H,m).

<0303-2>

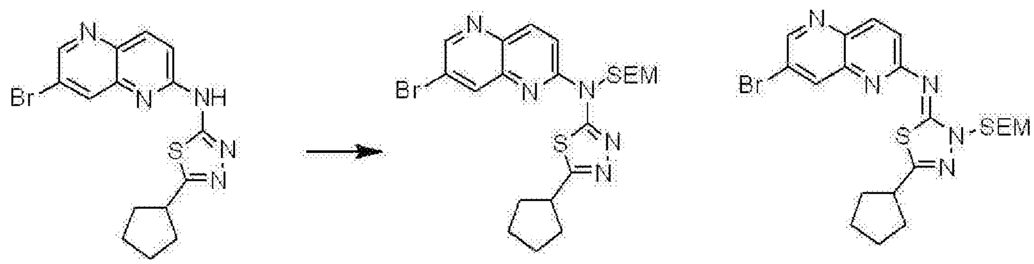

60% sodium hydride (40 mg) was added to a solution of N-(7-bromo-1,5-naphthyridin-2-yl)-5-cyclopentyl-1,3,4-thiadiazole-2-amine (0.25 g) and 2-(chloromethoxy)ethyltrimethylsilane (125 mg) in N-methylpyrrolidone (32 mL) under ice-cooling, followed by stirring at the same temperature for 1 hour, and stirring at room temperature for 1 hour. Ethyl acetate and water were added to the reaction mixture. The organic layer was collected by separation, washed with a saturated sodium chloride aqueous solution, and dried over anhydrous sodium sulfate. The obtained solution was purified by silica gel column chromatography (ethyl acetate-hexane), thereby obtaining N-(7-bromo-1,5-naphthyridin-2-yl)-5-cyclopentyl-N-((2-(trimethylsilyl)ethoxy)methyl)-1,3,4-thiadiazole-2-amine (601 mg) and (Z)-7-bromo-N-(5-cyclopentyl-3-((2-(trimethylsilyl)ethoxy)methyl)-1,3,4-thiadiazol-2(3H)-ylidene)-1,5-naphthyridine-2-amine (255 mg).
MSm/z(M+H):506,508, 506,508.

<0303-3>

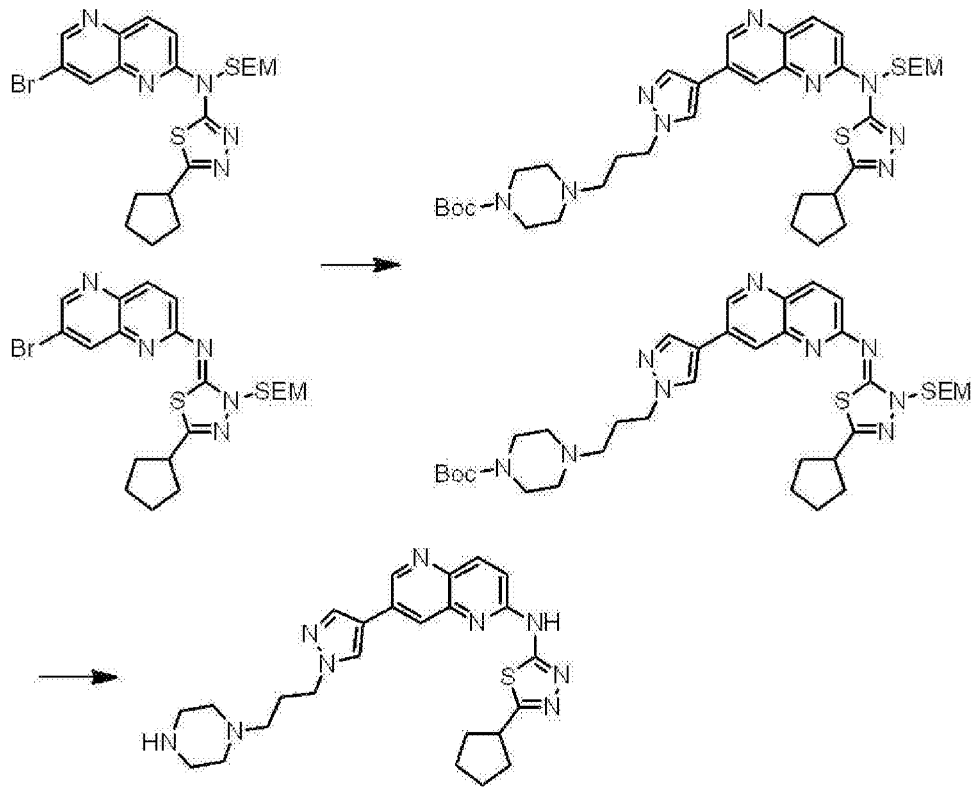

A mixture of a mixture (30 mg) of N-(7-bromo-1,5-naphthyridin-2-yl)-5-cyclopentyl-N-((2-(trimethylsilyl)ethoxy)methyl)-1,3,4-thiadiazole-2-amine and (Z)-7-bromo-N-(5-cyclopentyl-3-((2-(trimethylsilyl)ethoxy)methyl)-1,3,4-thiadiazol-2(3H)-ylidene)-1,5-naphthyridine-2-amine, tert-butyl 4-(3-(4-(4,4,5,5-tetramethyl-1,3,2-dioxaborolan-2-yl)-1H-pyrazol-1-yl)propyl)piperazine-1-carboxylate (29.9 mg), bis(di-tert-butyl (4-dimethylaminophenyl)phosphine)dichloropalladium (II) (4.2 mg), sodium carbonate (12.5 mg), 1,4-dioxane (1 mL), and water (0.1 mL) was stirred at 100°C for 4 hours in a nitrogen atmosphere in a sealed tube. The reaction mixture was cooled to room temperature, and purified by silica gel column chromatography (chloroform-methanol, NH silica), thereby obtaining a mixture (29.8 mg) of tert-butyl 4-(3-(4-(6-((5-cyclopentyl-1,3,4-thiadiazol-2-yl)((2-(trimethylsilyl)ethoxy)methyl)amino)-1,5-naphthyridin-3-yl)-1H-pyrazol-1-yl)propyl)piperazine-1-carboxylate and (Z)-tert-butyl 4-(3-(4-(6-((5-cyclopentyl-3-((2-(trimethylsilyl)ethoxy)methyl)-1,3,4-thiadiazol-2(3H)-ylidene)amino)-1,5-naphthyridin-3-yl)-1H-pyrazol-1-yl)propyl)piperazine-1-carboxylate as yellow oily substance.

A 4 mol/L hydrogen chloride/1,4-dioxane solution (1 mL) was added to a solution of the obtained mixture (29.8 mg) in methanol (1 mL) at room temperature, followed by stirring for 2 hours. The solvent was distilled off under reduced pressure, methanol and a saturated sodium hydrogen carbonate aqueous solution were added to the obtained residue in order to neutralize, and the water was distilled off under reduced pressure. The obtained residue was purified by silica gel column chromatography (chloroform-methanol, NH silica), thereby obtaining 5-cyclopentyl-N-(7-(1-(3-(piperazin-1-yl)propyl)-1H-pyrazol-4-yl)-1,5-naphthyridin-2-yl)-1,3,4-thiadiazole-2-amine (5.9 mg) as a yellow solid.

$^{1}$H-NMR(DMSO-$d_6$)δ:9.07(1H,d,J=2.3Hz),8.57(1H,s),8.32(1H,d,J=2.3Hz),8.28-8.20(2H,m),7.39(1H,d,J=9.2Hz),4.20(2H,t,J=6.9Hz),3.60-3.35(1H,m),2.69(4H,t,J=4.6Hz),2.38-1.61(16H,m).

MSm/z(M+H):490.

<Example 0304>

<0304-1>

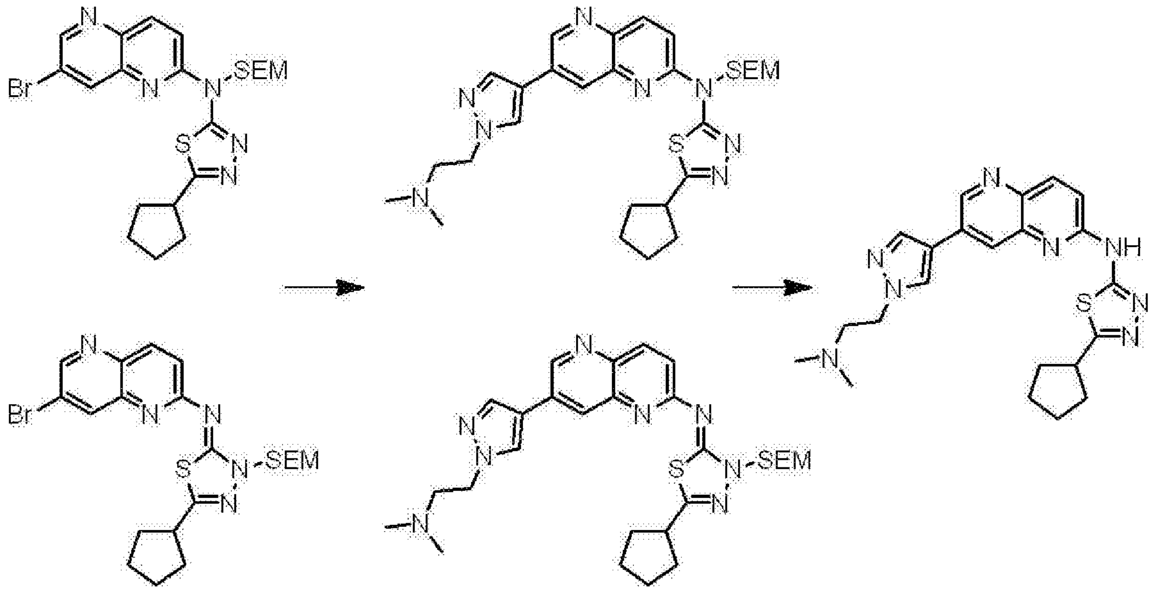

5-Cyclopentyl-N-(7-(1-(2-(dimethylamino)ethyl)-1H-pyrazol-4-yl)-1,5-naphthyridin-2-yl)-1,3,4-thiadiazole-2-amine was obtained as a yellow solid in the same manner as in Example 0303 except that N,N-dimethyl-2-(4-(4,4,5,5-tetramethyl-1,3,2-dioxaborolan-2-yl)-1H-pyrazol-1-yl)ethanamine was used instead of the tert-butyl 4-(3-(4-(4,4,5,5-tetramethyl-1,3,2-dioxaborolan-2-yl)-1H-pyrazol-1-yl)propyl)piperazine-1-carboxylate used in Example 0303.
$^1$H-NMR(DMSO-$d_6$)$\delta$:12.05(1H,s),9.07(1H,d,J=2.0Hz),8.57(1H,s),8.32(1H,d,J=2.0Hz),8.29-8.19(2H,m),7.39(1H,d,J=9.2Hz),4.26(2H,t,J=6.3Hz),3.55-3.40(1H,m),2.72(2H,t,J=6.6Hz),2.31-2.06(8H,m),1.98-1.59(6H,m).
MSm/z(M+H):435.

<Example 0305>

<0305-1>

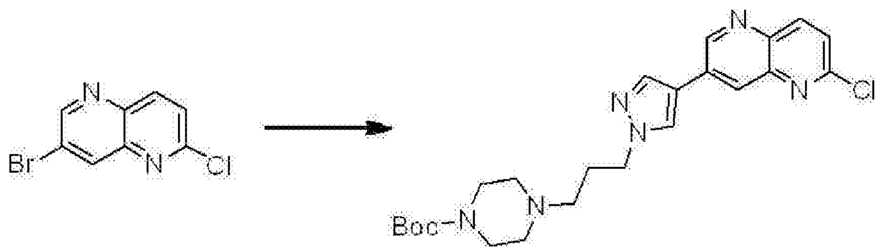

A mixture of 7-bromo-2-chloro-1,5-naphthyridine (100 mg), tert-butyl 4-(3-(4-(4,4,5,5-tetramethyl-1,3,2-dioxaborolan-2-yl)-1H-pyrazol-1-yl)propyl)piperazine-1-carboxylate (207 mg), bis(di-tert-butyl (4-dimethylaminophenyl)phosphine)dichloropalladium (II) (14.6 mg), sodium carbonate (87.1 mg), 1,4-dioxane (1 mL), and water (0.1 mL) was stirred at 140°C for 1 hour in a nitrogen atmosphere using a microwave reaction apparatus. The reaction mixture was cooled to room temperature, and purified by silica gel column chromatography (chloroform-methanol, NH silica), thereby obtaining tert-butyl 4-(3-(4-(6-chloro-1,5-naphthyridin-3-yl)-1H-pyrazol-1-yl)propyl)piperazine-1-carboxylate (199 mg) as yellow oily substance.
MSm/z(M+H):457,459.

<0305-2>

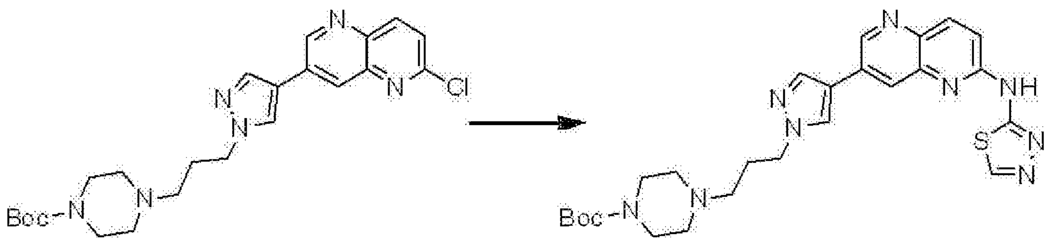

tert-Butyl 4-(3-(4-(6-((1,3,4-thiadiazol-2-yl)amino)-1,5-naphthyridin-3-yl)-1H-pyrazol-1-yl)propyl)piperazine-1-carboxylate was obtained as a yellow solid in the same manner as in Example 0301 except that tert-butyl 4-(3-(4-(6-chloro-1,5-naphthyridin-3-yl)-1H-pyrazol-1-yl)propyl)piperazine-1-carboxylate was used instead of the 3-(4-(6-chloro-1,5-naphthyridin-3-yl)-1H-pyrazol-1-yl)-N,N-dimethylpropane-1-amine used in Example 0301.
MSm/z(M+H):522.

<0305-3>

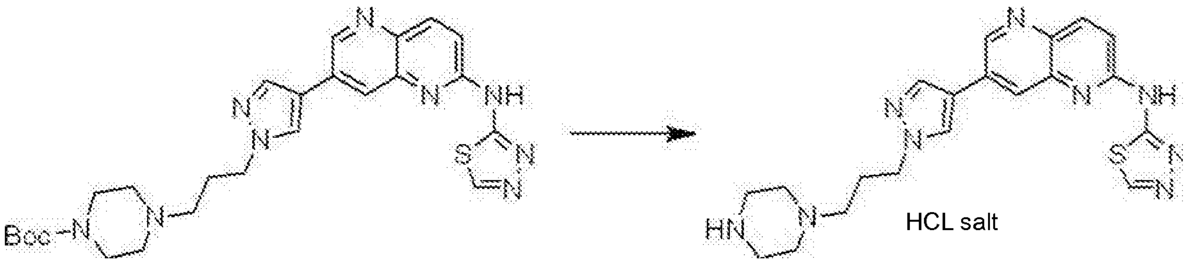


A 4 mol/L hydrogen chloride/1,4-dioxane solution (1 mL) was added to a solution of tert-butyl 4-(3-(4-(6-((1,3,4-thiadiazol-2-yl)amino)-1,5-naphthyridin-3-yl)-1H-pyrazol-1-yl)propyl)piperazine-1-carboxylate (59.8 mg) in methanol (1 mL) at room temperature, followed by stirring for 2 hours. The solid matter was collected by filtration, and washed with methanol, thereby obtaining N-(7-(1-(3-(piperazin-1-yl)propyl)-1H-pyrazol-4-yl)-1,5-naphthyridin-2-yl)-1,3,4-thiadiazole-2-amine hydrochloride (38.4 mg) as a yellow solid.
$^1$H-NMR(DMSO-$d_6$)$\delta$:9.46(2H,brs),9.20(1H,s),9.14(1H,d,J=2.0Hz),8.63(1H,s),8.40(1H,d,J=2.0Hz),8.35-8.25(2H,m),7.48(1H,d,=8.6Hz), 4.32(2H,t,J=6.6Hz),3.84-3.09(10H,m),2.43-2.22(2H,m).
MSm/z(M+H):422.

<Example 0306>

<0306-1>

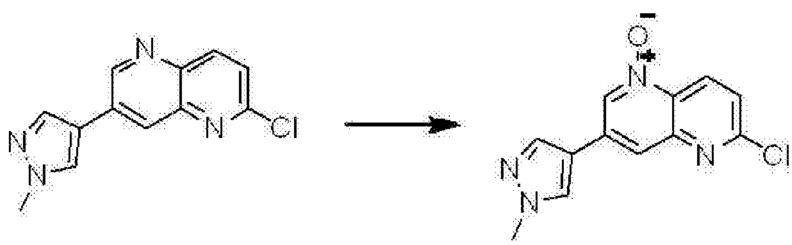

70% meta-chloroperoxybenzoic acid (200 mg) was added to a solution of 2-chloro-7-(1-methyl-1H-pyrazol-4-yl)-1,5-naphthyridine (200 mg) in dichloromethane (4 mL) at room temperature, followed by stirring at room temperature for 30 minutes. 70% meta-chloroperoxybenzoic acid (200 mg) was added thereto, followed by stirring at room temperature for 30 minutes. 70% meta-chloroperoxybenzoic acid (200 mg) was added thereto, followed by stirring at room temperature

for 30 minutes, and 70% meta-chloroperoxybenzoic acid (400 mg) was added thereto, followed by stirring at room temperature for 1 hour. A saturated sodium hydrogen carbonate aqueous solution and sodium sulfite were added to the reaction mixture, and ethyl acetate was added thereto. The organic layer was collected by separation, washed with a saturated sodium hydrogen carbonate aqueous solution and a saturated sodium chloride aqueous solution, and dried over anhydrous sodium sulfate, and the solvent was distilled off under reduced pressure, thereby obtaining 6-chloro-3-(1-methyl-1H-pyrazol-4-yl)-1,5-naphthyridine 1-oxide (240 mg) as a yellow solid.
MSm/z(M+H):261,263.

<0306-2>

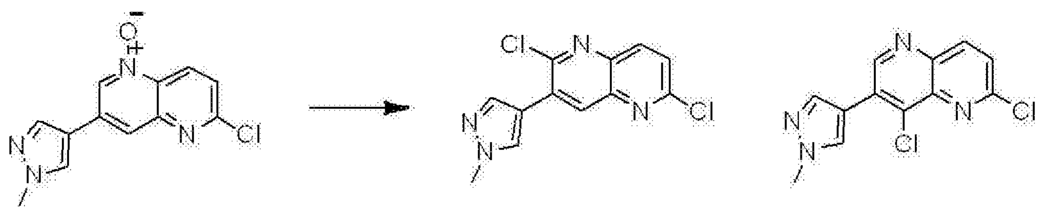

Phosphorus oxychloride (2 mL) was added to 6-chloro-3-(1-methyl-1H-pyrazol-4-yl)-1,5-naphthyridine 1-oxide (240 mg), followed by stirring at 100°C for 30 minutes. The reaction mixture was cooled to room temperature, and added dropwise to water. The resultant product was neutralized by the addition of sodium carbonate, and ethyl acetate was added thereto. The organic layer was collected by separation, washed with a saturated sodium chloride aqueous solution, and dried over anhydrous sodium sulfate, and the solvent was distilled off under reduced pressure, thereby obtaining a mixture (210 mg) of 2,6-dichloro-3-(1-methyl-1H-pyrazol-4-yl)-1,5-naphthyridine and 2,8-dichloro-7-(1-methyl-1H-pyrazol-4-yl)-1,5-naphthyridine as a yellow solid.
MSm/z(M+H):279,281.

<0306-3>

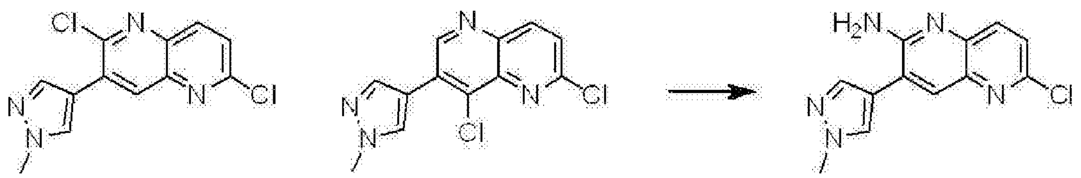

A mixture of a mixture (200 mg) of 2,6-dichloro-3-(1-methyl-1H-pyrazol-4-yl)-1,5-naphthyridine and 2,8-dichloro-7-(1-methyl-1H-pyrazol-4-yl)-1,5-naphthyridine, 1,4-dioxane (5 mL) and a 25% ammonia aqueous solution (5 mL) was stirred at 120°C for 2 hours, and stirred at 140°C for 2 hours using a microwave reaction apparatus. The reaction mixture was cooled to room temperature, and the solvent was distilled off under reduced pressure. The obtained residue was purified by silica gel column chromatography (chloroform-methanol, NH silica), thereby obtaining 6-chloro-3-(1-methyl-1H-pyrazol-4-yl)-1,5-naphthyridine-2-amine (46.9 mg) as a white solid.
MSm/z(M+H):260,262.

<0306-4>

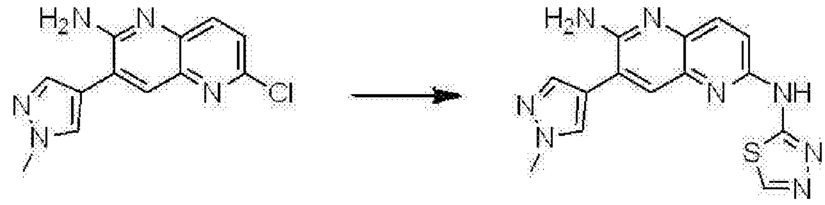

7-(1-methyl-1H-pyrazol-4-yl)-$N^2$-(1,3,4-thiadiazol-2-yl)-1,5-naphthyridine-2,6-diamine (1.7 mg) was obtained as a yellow solid in the same manner as in Example 0301 except that 6-chloro-3-(1-methyl-1H-pyrazol-4-yl)-1,5-naphthyridine-2-amine was used instead of the 3-(4-(6-chloro-1,5-naphthyridin-3-yl)-1H-pyrazol-1-yl)-N,N-dimethylpropane-1-amine used in Example 0301.
$^1$H-NMR(DMSO-$d_6$)δ:11.82(1H,s),9.03(1H,s),8.20(1H,s),7.94-7.79(3H,m),7.30(1H,d,J=9.2Hz),6.13(2H,brs),3.92(3H,s).
MSm/z(M+H):325.

<Example 0307>

<0307-1>

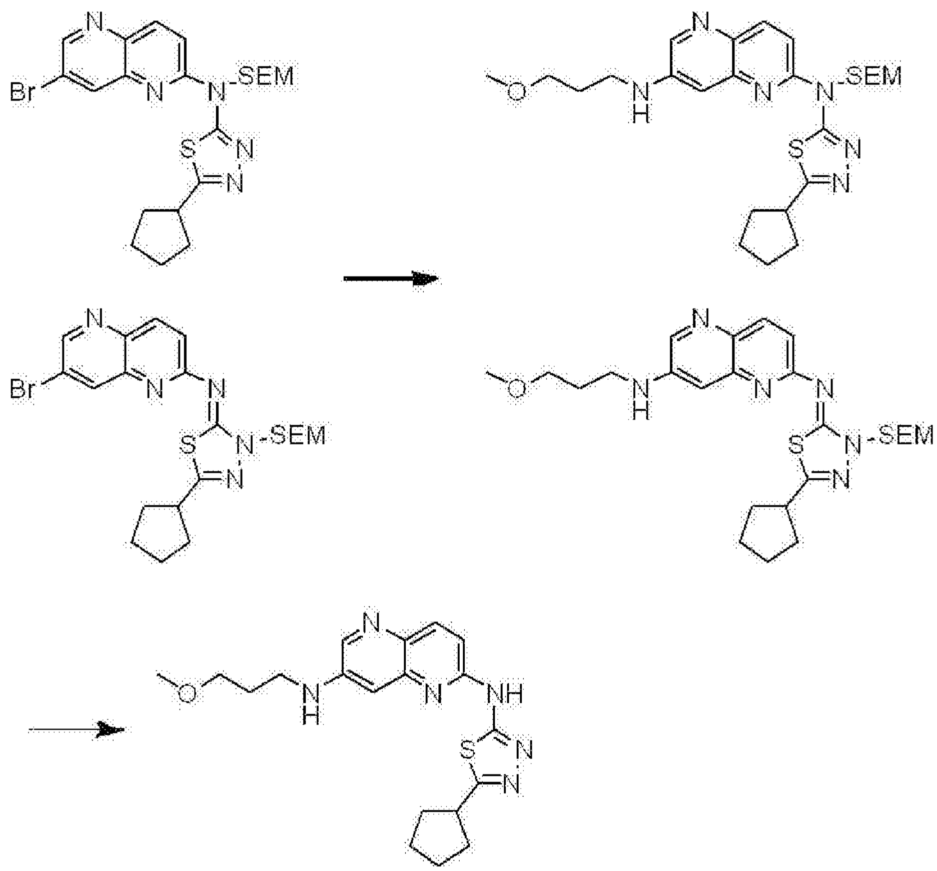

A mixture of a mixture (30 mg) of N-(7-bromo-1,5-naphthyridin-2-yl)-5-cyclopentyl-N-((2-(trimethylsilyl)ethoxy)methyl)-1,3,4-thiadiazole-2-amine and (Z)-7-bromo-N-(5-cyclopentyl-3-((2-(trimethylsilyl)ethoxy)methyl)-1,3,4-thiadiazol-2(3H)-ylidene)-1,5-naphthyridine-2-amine, 3-methoxypropane-1-amine (12.2 μL), tris(dibenzylideneacetone)dipalladium(0) (3.4 mg), 2-dicyclohexylphosphino-2',4',6'-triisopropylbiphenyl (5.6 mg), cesium carbonate (38.4 mg), and 1,4-dioxane (1 mL) was stirred at 100°C for 16 hours in a nitrogen atmosphere in a sealed tube. The reaction mixture was cooled to room temperature, and purified by silica gel column chromatography (hexane-ethyl acetate, NH silica), thereby obtaining a mixture (11.3 mg) of $N^2$-(5-cyclopentyl-1,3,4-thiadiazol-2-yl)-$N^7$-(3-methoxypropyl)-$N^2$-((2-(tri-

methylsilyl)ethoxy)methyl)-1,5-naphthyridine-2,7-diamine and (Z)-N$^{2}$-(5-cyclopentyl-3-((2-(trimethylsilyl)ethoxy)methyl)-1,3,4-thiadiazol-2(3H)-ylidene)-N$^{7}$-(3-methoxypropyl)-1,5-naphthyridine-2,7-diamine as yellow oily substance.

A 4 mol/L hydrogen chloride/1,4-dioxane solution (1 mL) was added to a solution of the obtained mixture (11.3 mg) in methanol (1 mL), followed by stirring for 1 hour and allowing to stand for 15 hours. The solvent was distilled off under reduced pressure, methanol (1 mL) and triethylamine (1 mL) were added to the obtained residue, and the solvent was distilled off under reduced pressure. The obtained residue was purified by silica gel column chromatography (chloroform-methanol, NH silica), thereby obtaining N$^{2}$-(5-cyclopentyl-1,3,4-thiadiazol-2-yl)-N$^{7}$-(3-methoxypropyl)-1,5-naphthyridine-2,7-diamine (5.7 mg) as a yellow solid.
$^{1}$H-NMR(DMSO-$d_6$)δ:11.75(1H,brs),8.30(1H,d,J=2.6Hz),8.01(1H,d,J=8.9Hz),7.03(1H,d,J=8.9Hz),6.92(1H,d,J=2.6Hz),6.61(1H,t,J=5.3Hz),3.54-3.39(3H,m),3.29(3H,s),3.27-3.15(2H,m),2.22-2.04(2H,m),1.94-1.60(8H,m).
MSm/z(M+H):385.

<Example 0308>

<0308-1>

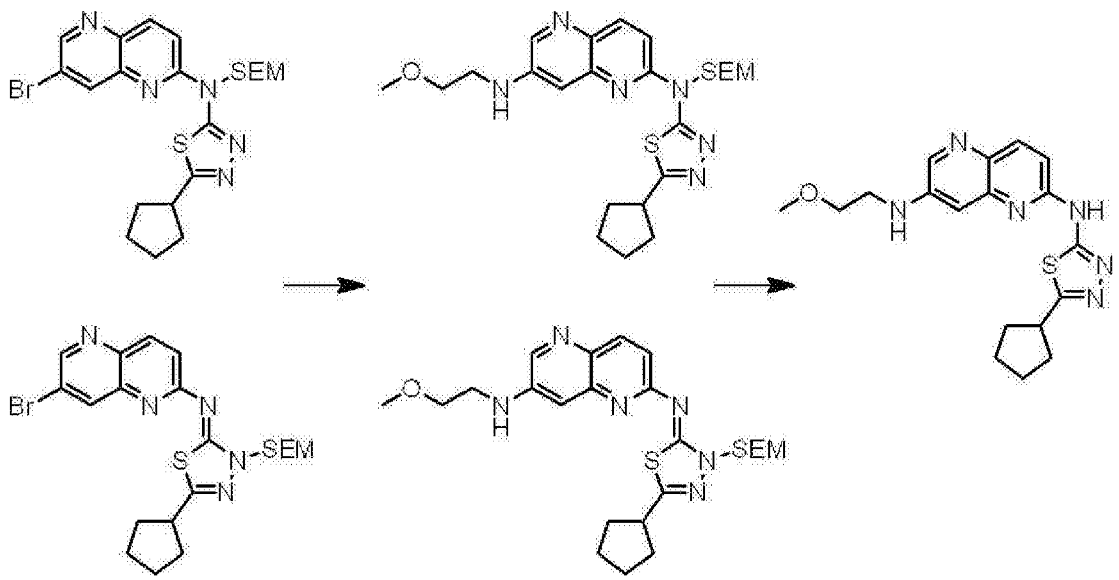

N$^{2}$-(5-cyclopentyl-1,3,4-thiadiazol-2-yl)-N$^{7}$-(2-methoxyethyl)-1,5-naphthyridine-2,7-diamine (3.1 mg) was obtained as a yellow solid in the same manner as in Example 0307 except that 2-methoxyethanamine was used instead of the 3-methoxypropane-1-amine used in Example 0307.
$^{1}$H-NMR(DMSO-$d_6$)δ:11.75(1H,s),8.36(1H,d,J=2.3Hz),8.01(1H,d,J=9.2Hz),7.03(1H,d,J=9.2Hz),6.96(1H,d,J=2.3Hz),6.63(1H,t,J=5.3Hz),3.59(2H,t,J=5.6Hz),3.53-3.41(1H,m),3.40-3.27(5H,m),2.23-2.03(2H,m),1.95-1.59(6H,m).
MSm/z(M+H):371.

<Example 0309>

<0309-1>

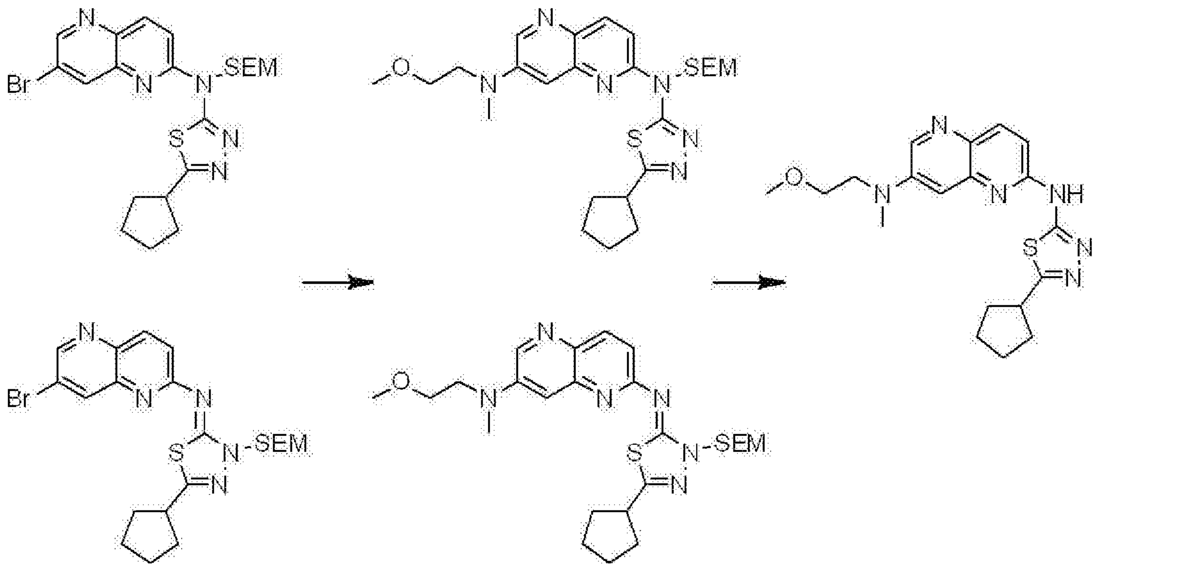

$N^2$-(5-cyclopentyl-1,3,4-thiadiazol-2-yl)-$N^7$-(2-methoxyethyl)-$N^7$-methyl-1,5-naphthyridine-2,7-diamine (4.5 mg) was obtained as a yellow solid in the same manner as in Example 0307 except that 2-methoxy-N-methylethanamine was used instead of the 3-methoxypropane-1-amine used in Example 0307.
$^1$H-NMR(DMSO-$d_6$)$\delta$:11.79(1H,s),8.55(1H,d,J=2.6Hz),8.05(1H,d,J=8.6Hz), 7.13-7.05(2H,m),3.73(2H,t,J=5.3Hz),3.57(2H,t,J=5.3Hz),3.53-3.39(1H,m),3.26(3H,s),3.09(3H,s),2.22-2.04(2H,m),1.93 -1.59(6H,m).
MSm/z(M+H):385.

<Example 0310>

<0310-1>

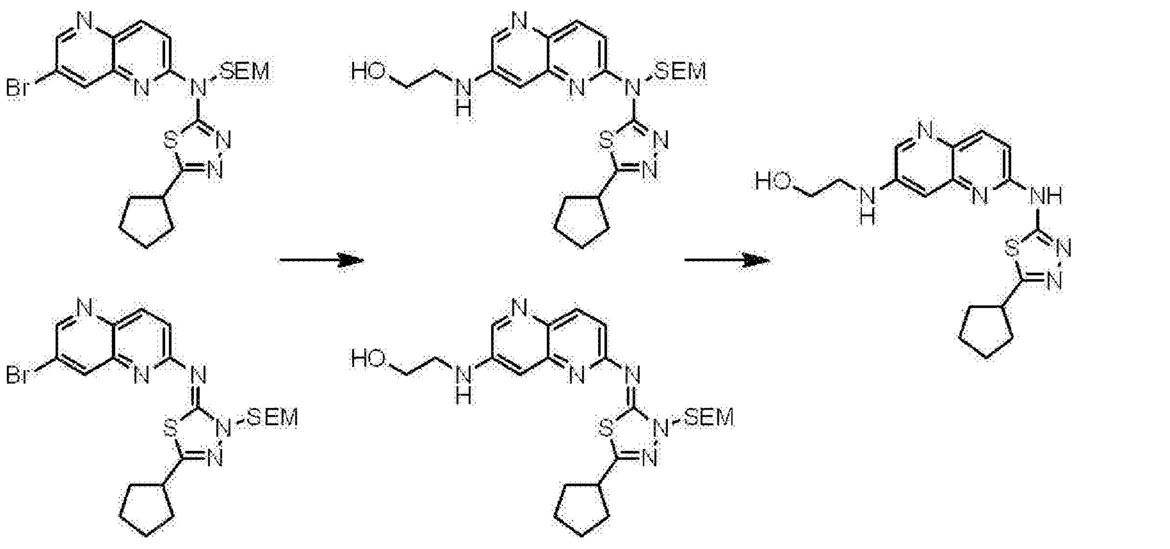

A mixture of a mixture (30 mg) of N-(7-bromo-1,5-naphthyridin-2-yl)-5-cyclopentyl-N-((2-(trimethylsilyl)ethoxy)methyl)-1,3,4-thiadiazole-2-amine and (Z)-7-bromo-N-(5-cyclopentyl-3-((2-(trimethylsilyl)ethoxy)methyl)-1,3,4-thiadiazol-2(3H)-ylidene)-1,5-naphthyridine-2-amine, 2-aminoethanol (7.1 μL), tris(dibenzylideneacetone)dipalladium(0) (3.4 mg), 2-dicyclohexylphosphino-2',4',6'-triisopropylbiphenyl (5.6 mg), cesium carbonate (38.4 mg), and 1,4-dioxane (1 mL) was stirred at 100°C for 16 hours in a nitrogen atmosphere in a sealed tube. The reaction mixture was cooled to room temperature, and the solvent was distilled off under reduced pressure, thereby obtaining a mixture of

2-((6-((5-cyclopentyl-1,3,4-thiadiazol-2-yl)((2-(trimethylsilyl)ethoxy)methyl)amino)-1,5-naphthyridin-3-yl)amino)ethanol and (Z)-2-((6-((5-cyclopentyl-3-((2-(trimethylsilyl)ethoxy)methyl)-1,3,4-thiadiazol-2(3H)-ylidene)amino)-1,5-naphthyridin-3-yl)amino)ethanol as yellow oily substance.

A 4 mol/L hydrogen chloride/1,4-dioxane solution (2 mL) was added to a solution of the obtained mixture in methanol (2 mL) at room temperature, followed by stirring for 45 minutes, allowing to stand for 15 hours, and stirring at 50°C for 1 hour. The reaction mixture was cooled to room temperature, and the solvent was distilled off under reduced pressure. Methanol (1 mL) and triethylamine (1 mL) were added to the obtained residue, and the solvent was distilled off under reduced pressure. The obtained residue was purified by silica gel column chromatography (chloroform-methanol, NH silica), thereby obtaining 2-((6-((5-cyclopentyl-1,3,4-thiadiazol-2-yl)amino)-1,5-naphthyridin-3-yl)amino)ethanol (0.7 mg) as a yellow solid.

$^1$H-NMR(DMSO-$d_6$)δ:11.73(1H,brs),8.34(1H,d,J=2.6Hz),8.00(1H,d,J=8.6Hz),7.03(1H,d,J=8.6Hz),6.94(1H,d,J=2.6Hz), 6.59(1H,t,J=5.6Hz),4.83(2H,t,J=5.6Hz),3.65(2H,dt,J=5.6,5.6Hz),3.53-3.39(1H,m),2.24-2.03(2H,m),1.96-1.54(6H,m).
MSm/z(M+H):357.

<Example 0311>

<0311-1>

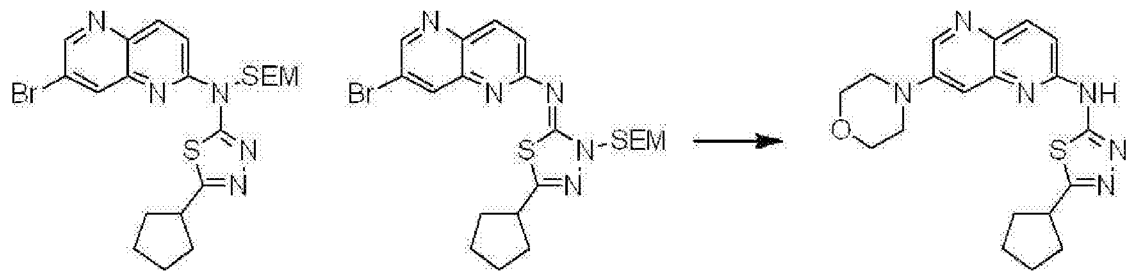

A mixture of a mixture (20 mg) of N-(7-bromo-1,5-naphthyridin-2-yl)-5-cyclopentyl-N-((2-(trimethylsilyl)ethoxy)methyl)-1,3,4-thiadiazole-2-amine and (Z)-7-bromo-N-(5-cyclopentyl-3-((2-(trimethylsilyl)ethoxy)methyl)-1,3,4-thiadiazol-2(3H)-ylidene)-1,5-naphthyridine-2-amine, morpholine (6.9 μL), tris(dibenzylideneacetone)dipalladium(0) (2.3 mg), 2-dicyclohexylphosphino-2',4',6'-triisopropylbiphenyl (3.8 mg), sodium tert-butoxide (7.6 mg), and 1,4-dioxane (1 mL) was stirred at 100°C for 4.5 hours in a nitrogen atmosphere in a sealed tube. The reaction mixture was cooled to room temperature, and methanol (1 mL) and a 4 mol/L hydrogen chloride/1,4-dioxane solution (1 mL) were added thereto, followed by stirring at 40°C for 15 hours. The reaction mixture was cooled to room temperature, and the solvent was distilled off under reduced pressure. Methanol and triethylamine were added to the obtained residue, and the solvent was distilled off under reduced pressure. The obtained residue was purified by silica gel column chromatography (chloroform-methanol, NH silica), thereby obtaining 5-cyclopentyl-N-(7-morpholino-1,5-naphthyridin-2-yl)-1,3,4-thiadiazole-2-amine (9.0 mg) as a yellow solid.

$^1$H-NMR(DMSO-$d_6$)δ:11.88(1H,brs),8.72(1H,d,J=2.6Hz),8.13(1H,d,J=9.2Hz),7.38(1H,d,J=2.6Hz),7.20(1H,d,J=9.2Hz), 3.87-3.75(4H,m),3.52-3.28(5H,m),2.22-2.06(2H,m),1.94-1.61(6H,m).
MSm/z(M+H):383.

<Example 0312>

<0312-1>

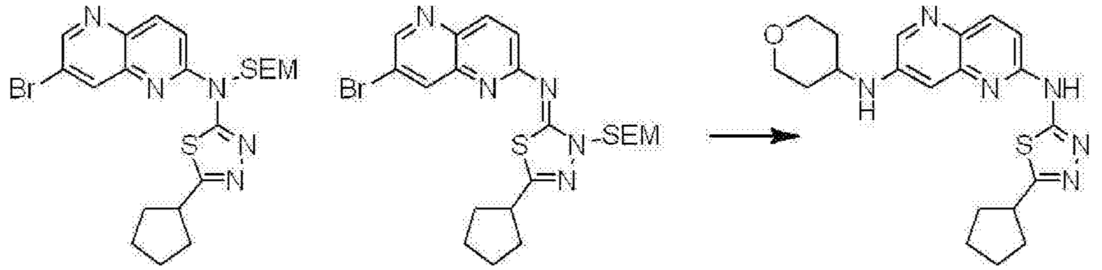

$N^2$-(5-cyclopentyl-1,3,4-thiadiazol-2-yl)-$N^7$-(tetrahydro-2H-pyran-4-yl)-1,5-naphthyridine-2,7-diamine was obtained as a white solid in the same manner as in Example 0311 except that tetrahydro-2H-pyran-4-amine was used instead of the morpholine used in Example 0311.
$^1$H-NMR(DMSO-$d_6$)δ:11.75(1H,s),8.32(1H,d,J=2.6Hz),8.00(1H,d,J=9.2Hz), 7.08-6.99(2H,m),6.54(1H,d,J=7.9Hz),3.96-3.83(2H,m),3.80-3.61(1H,m),3.58-3.28(3H,m),2.21-2.06(2H,m),2.03-1.60(8H,m),1.55-1.36(2H,m).
MSm/z(M+H):397.

<Example 0313>

<0313-1>

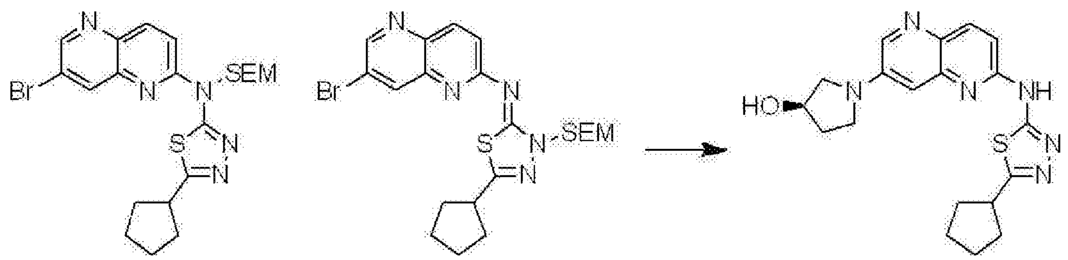

(R)-1-(6-((5-cyclopentyl-1,3,4-thiadiazol-2-yl)amino)-1,5-naphthyridin-3-yl)pyrrolidin-3-ol was obtained as a white solid in the same manner as in Example 0311 except that (R)-pyrrolidin-3-ol was used instead of the morpholine used in Example 0311.
$^1$H-NMR(DMSO-$d_6$)δ:11.78(1H,brs),8.35(1H,d,J=2.6Hz),8.05(1H,d,J=8.6Hz),7.06(1H,d,J=8.6Hz),6.94(1H,d,J=2.6Hz), 5.06(1H,d,J=4.0Hz),4.52-4.42(1H,m),3.66-3.20(5H,m),2.24-1.57(10H,m).
MSm/z(M+H):383.

<Example 0314>

<0314-1>

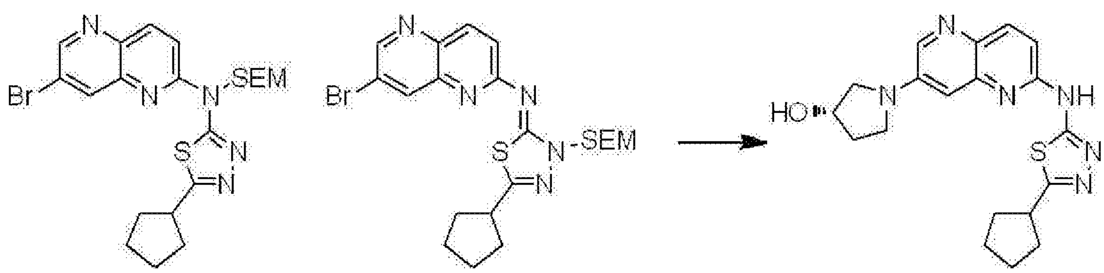

(S)-1-(6-((5-cyclopentyl-1,3,4-thiadiazol-2-yl)amino)-1,5-naphthyridin-3-yl)pyrrolidin-3-ol was obtained as a

white solid in the same manner as in Example 0311 except that (S)-pyrrolidin-3-ol was used instead of the morpholine used in Example 0311.
$^1$H-NMR(DMSO-$d_6$)δ:11.78(1H,brs),8.35(1H,d,J=2.6Hz),8.05(1H,d,J=8.6Hz),7.06(1H,d,J=8.6Hz),6.94(1H,d,J=2.6Hz), 5.06(1H,d,J=4.0Hz),4.52-4.42(1H,m),3.66-3.20(5H,m),2.24-1.57(10H,m).
MSm/z(M+H):383.

<Example 0315>

<0315-1>

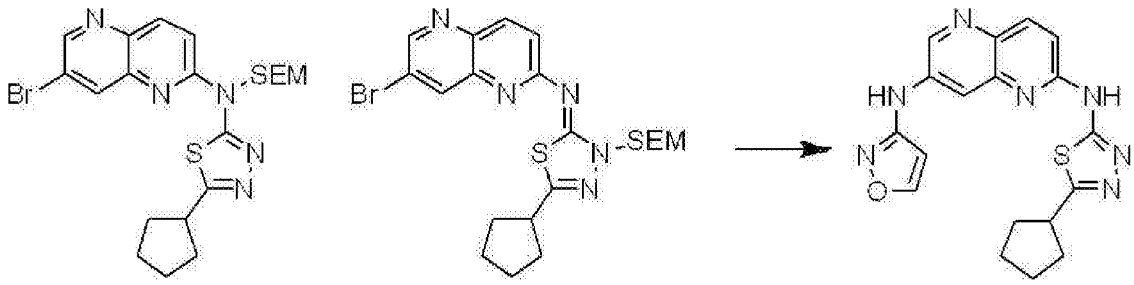

A mixture of N-(7-bromo-1,5-naphthyridin-2-yl)-5-cyclopentyl-N-((2-(trimethylsilyl)ethoxy)methyl)-1,3,4-thiadiazole-2-amine (30 mg), isoxazole-3-amine (9.9 mg), tris(dibenzylideneacetone)dipalladium(0) (3.4 mg), 2-dicyclohexylphosphino-2',4',6'-triisopropylbiphenyl (5.6 mg), cesium carbonate (38.4 mg), and 1,4-dioxane (1 mL) was stirred at 100°C for 14.5 hours in a nitrogen atmosphere in a sealed tube. The reaction mixture was cooled to room temperature, and methanol and a 4 mol/L hydrogen chloride/1,4-dioxane solution were added thereto, followed by stirring at room temperature for 2 hours, and stirring at 50°C for 30 minutes. The reaction mixture was cooled to room temperature, and the solvent was distilled off under reduced pressure. Methanol and triethylamine were added to the obtained residue, and the solvent was distilled off under reduced pressure. The obtained residue was purified by silica gel column chromatography (chloroform-methanol, NH silica), thereby obtaining $N^2$-(5-cyclopentyl-1,3,4-thiadiazol-2-yl)-$N^7$-(isoxazol-3-yl)-1,5-naphthyridine-2,7-diamine (4.4 mg) as a white solid.
$^1$H-NMR(DMSO-$d_6$)δ:11.95(1H,brs),10.00(1H,s),8.74(1H,d,J=1.3Hz),8.69(1H,d,J=2.6Hz),8.37(1H,d,J=2.6Hz),8.18(1H, d,J=9.2Hz),7.27(1H,d,J=9.2Hz),6.36(1H,d,J=1.3Hz),3.57-3.39(1H,m),2.24-2.06(2H,m),1.96-1.60(6H,m).
MSm/z(M+H):380.

<Example 0316>

<0316-1>

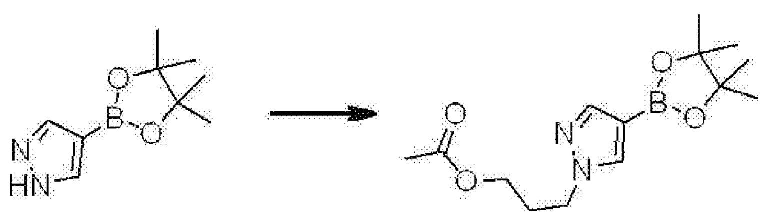

A mixture of 4-(4,4,5,5-tetramethyl-1,3,2-dioxaborolan-2-yl)-1H-pyrazole (0.867 g), 3-bromopropyl acetate (0.970 g), potassium carbonate (1.85 g), and acetonitrile (4 mL) was stirred at 65°C for 16.5 hours in a nitrogen atmosphere. The reaction mixture was cooled to room temperature, the solid matter was filtered off, and the solvent was distilled off under reduced pressure. The obtained residue was purified by silica gel column chromatography (chloroform-methanol), thereby obtaining 3-(4-(4,4,5,5-tetramethyl-1,3,2-dioxaborolan-2-yl)-1H-pyrazol-1-yl)propyl acetate (1.24 g) as yellow oily substance.
$^1$H-

NMR($CDCl_3$)δ:7.79(1H,s),7.69(1H,s),4.23(2H,t,J=6.6Hz),4.05(2H,t,J=5.9Hz),2.26-2.14(2H,m),2.05(3H,s),1.32(12H,s).

<0316-2>

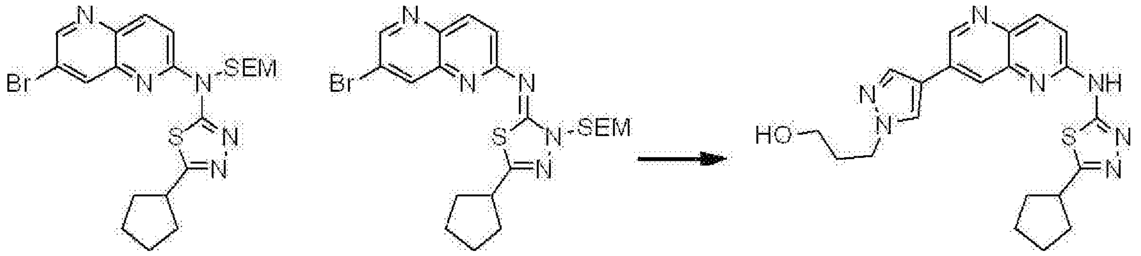

A mixture of a mixture (100 mg) of N-(7-bromo-1,5-naphthyridin-2-yl)-5-cyclopentyl-N-((2-(trimethylsilyl)ethoxy)methyl)-1,3,4-thiadiazole-2-amine and (Z)-7-bromo-N-(5-cyclopentyl-3-((2-(trimethylsilyl)ethoxy)methyl)-1,3,4-thiadiazol-2(3H)-ylidene)-1,5-naphthyridine-2-amine, 3-(4-(4,4,5,5-tetramethyl-1,3,2-dioxaborolan-2-yl)-1H-pyrazol-1-yl)propyl acetate (87.1 mg), bis(di-tert-butyl (4-dimethylaminophenyl)phosphine)dichloropalladium(II) (13.9 mg), sodium carbonate (41.8 mg), 1,4-dioxane (1 mL), and water (0.1 mL) was stirred at 150°C for 30 minutes in a nitrogen atmosphere using a microwave reaction apparatus. The reaction mixture was cooled to room temperature, and methanol (1 mL) and a 4 mol/L hydrogen chloride/1,4-dioxane solution (1 mL) were added thereto, followed by stirring at room temperature for 2 hours. The solvent was distilled off under reduced pressure, methanol and triethylamine were added to the obtained residue, and the solvent was distilled off under reduced pressure. The obtained residue was purified by silica gel column chromatography (chloroform-methanol, NH silica), thereby obtaining 3-(4-(6-((5-cyclopentyl-1,3,4-thiadiazol-2-yl)amino)-1,5-naphthyridin-3-yl)-1H-pyrazol-1-yl)propan-1-ol (20.1 mg) as a white solid.
$^1$H-NMR(DMSO-$d_6$)δ:12.03(1H,s),9.08(1H,d,J=2.0Hz),8.57(1H,s),8.33(1H,d,J=2.0Hz),8.28-8.21(2H,m),7.39(1H,d,J=9.2Hz),4.64(1H,t,J=5.0Hz),4.24(2H,t,J=7.3Hz),3.56-3.36(3H,m),2.25-1.62(10H,m).
MSm/z(M+H):422.

<Example 0317>

<0317-1>

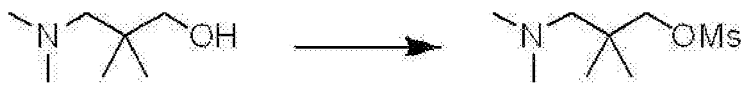

Methanesulfonyl chloride (0.326 mL) was added to a solution of 3-(dimethylamino)-2,2-dimethylpropan-1-ol (0.50 g) in pyridine (3.8 mL) in an ice bath, followed by stirring at room temperature for 30 minutes. A saturated sodium hydrogen carbonate aqueous solution was added to the reaction mixture, followed by stirring at room temperature for 20 minutes, and after sodium chloride was added thereto, ethyl acetate was added thereto. The organic layer was collected by separation, washed with a saturated sodium chloride aqueous solution, and dried over anhydrous sodium sulfate. The obtained solid matter was washed with ethyl acetate, thereby obtaining 3-(dimethylamino)-2,2-dimethylpropyl methanesulfonate (0.327 g) as a white solid.
$^1$H-NMR($CDCl_3$)δ:4.32(4H,s),3.49(6H,s),2.74(3H,s),1.46(6H,s).

<0317-2>

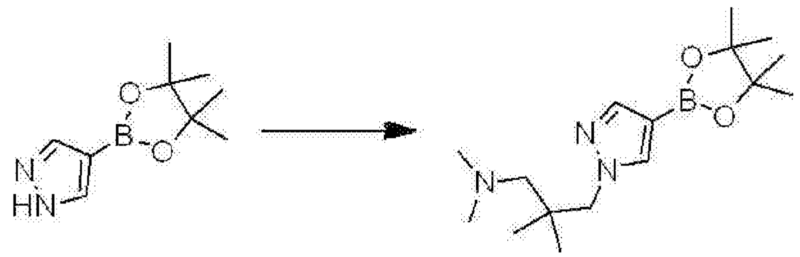

N,N,2,2-tetramethyl-3-(4-(4,4,5,5-tetramethyl-1,3,2-dioxaborolan-2-yl)-1H-pyrazol-1-yl)propane-1-amine was obtained as colorless oily substance in the same manner as in Example 0316 except that 3-(dimethylamino)-2,2-dimethylpropyl methanesulfonate was used instead of the 3-bromopropyl acetate used in Example 0316.
$^1$H-NMR($CDCl_3$)$\delta$:7.76(1H,s),7.65(1H,s),4.02(2H,s),2.31(6H,s),2.16(2H,s),1.32(12H,s),0.90(6H,s).

<0317-3>

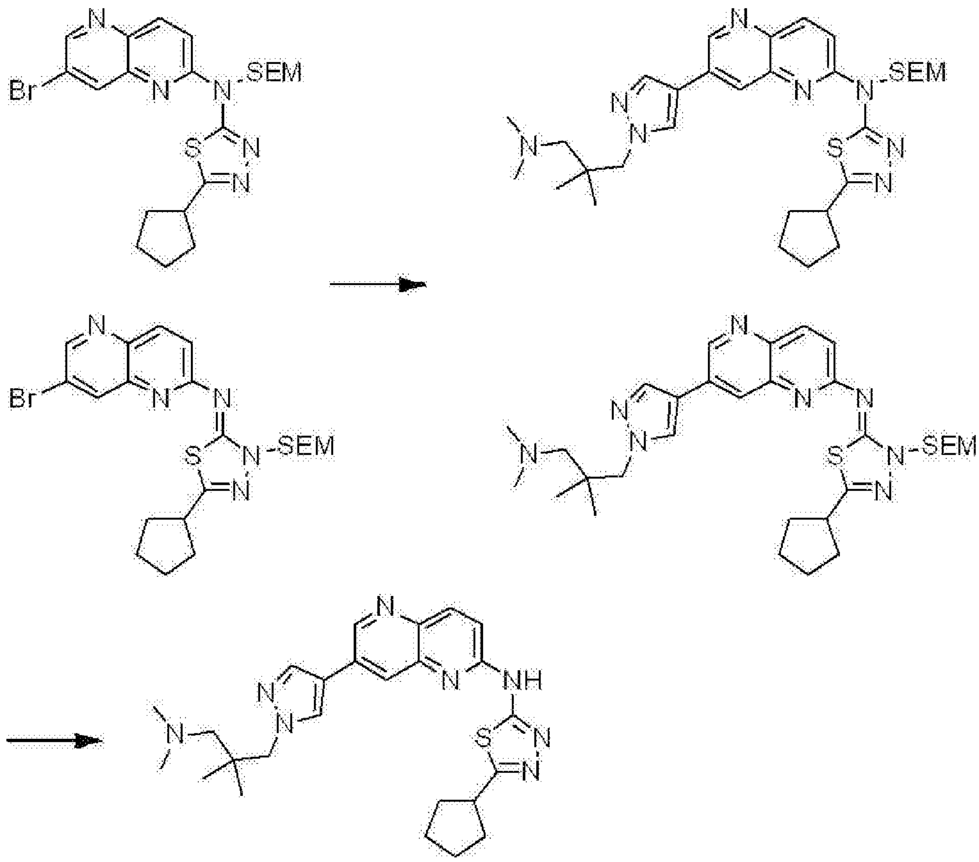

5-Cyclopentyl-N-(7-(1-(3-(dimethylamino)-2,2-dimethylpropyl)-1H-pyrazol-4-yl)-1,5-naphthyridin-2-yl)-1,3,4-thiadiazole-2-amine was obtained as a white solid in the same manner as in Example 0303 except that N,N,2,2-tetramethyl-3-(4-(4,4,5,5-tetramethyl-1,3,2-dioxaborolan-2-yl)-1H-pyrazol-1-yl)propane-1-amine was used instead of the tert-butyl 4-(3-(4-(4,4,5,5-tetramethyl-1,3,2-dioxaborolan-2-yl)-1H-pyrazol-1-yl)propyl)piperazine-1-carboxylate used in Example 0303.
$^1$H-NMR(DMSO-$d_6$)$\delta$:12.04(1H,brs),9.09(1H,d,J=1.7Hz),8.52(1H,s),8.34(1H,d,J=1.7Hz),8.28-8.20(2H,m),7.39(1H,d,J=9.2Hz),4.06(2H,s),3.54-3.37(1H,m),2.30(6H,s),2.25-2.05(4H,m),1.98-1.63(6H,m),0.89(6H,s).
MSm/z(M+H):477.

<Example 0318>

<0318-1>

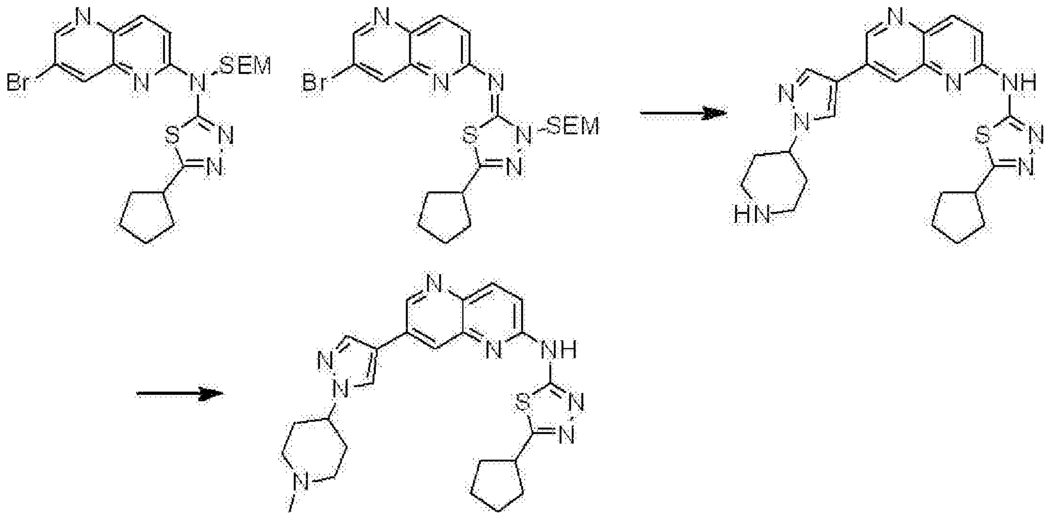

A mixture of a mixture (30 mg) of N-(7-bromo-1,5-naphthyridin-2-yl)-5-cyclopentyl-N-((2-(trimethylsilyl)ethoxy)methyl)-1,3,4-thiadiazole-2-amine and (Z)-7-bromo-N-(5-cyclopentyl-3-((2-(trimethylsilyl)ethoxy)methyl)-1,3,4-thiadiazol-2(3H)-ylidene)-1,5-naphthyridine-2-amine, bis(pinacolato)diboron (22.5 mg), (1,1'-bis(diphenylphosphino)feffocene)palladium(II) dichloride (4.8 mg), potassium acetate (11.6 mg), and 1,4-dioxane (1 mL) was stirred at 80°C for 6 hours in a nitrogen atmosphere in a sealed tube. Bis(pinacolato)diboron (22.5 mg), (1,1'-bis(diphenylphosphino)ferrocene)palladium(II) dichloride (4.8 mg), and potassium acetate (11.6 mg) were added to the reaction mixture, followed by stirring at 80°C for 14 hours. The reaction mixture was cooled to room temperature, and sodium carbonate (31.4 mg), tert-butyl 4-(4-iodo-1H-pyrazol-1-yl)piperidine-1-carboxylate (33.5 mg), bis(di-tert-butyl (4-dimethylaminophenyl)phosphine)dichloropalladium(II) (4.2 mg), and water (0.1 mL) were added thereto, followed by stirring at 100°C for 23 hours. The reaction mixture was cooled to room temperature, and methanol (1 mL) and a 4 mol/L hydrogen chloride/1,4-dioxane solution (1 mL) were added thereto, followed by stirring at room temperature for 2 hours, and stirring at 50°C for 1 hour. The reaction mixture was cooled to room temperature, and the solvent was distilled off under reduced pressure. Methanol and triethylamine were added to the obtained residue, the solvent was distilled off under reduced pressure, and the obtained residue was purified by silica gel column chromatography (chloroform-methanol, NH silica), thereby obtaining 5-cyclopentyl-N-(7-(1-(piperidin-4-yl)-1H-pyrazol-4-yl)-1,5-naphthyridin-2-yl)-1,3,4-thiadiazole-2-amine.

The obtained 5-cyclopentyl-N-(7-(1-(piperidin-4-yl)-1H-pyrazol-4-yl)-1,5-naphthyridin-2-yl)-1,3,4-thiadiazole-2-amine was dissolved in a mixture of methanol (0.5 mL) and dichloromethane (0.5 mL), and a 36 to 38% formaldehyde aqueous solution (0.5 mL) and sodium triacetoxyborohydride (20 mg) were added thereto at room temperature, followed by stirring for 2 hours. The solvent was distilled off under reduced pressure, and the obtained residue was purified by silica gel column chromatography (chloroform-methanol, NH silica), thereby obtaining 5-cyclopentyl-N-(7-(1-(1-methylpiperidin-4-yl)-1H-pyrazol-4-yl)-1,5-naphthyridin-2-yl)-1,3,4-thiadiazole-2-amine (3.5 mg) as a yellow solid.
$^1$H-NMR(DMSO-$d_6$)δ:12.04(1H,brs),9.09(1H,d,J=1.7Hz),8.65(1H,s),8.34(1H,d,J=1.7Hz),8.28-8.18(2H,m),7.38(1H,d,J=9.2Hz),4.27-4.07(1H,m),3.57-3.38(1H,m),2.98-2.80(2H,m),2.31-1.60(17H,m).
MSm/z(M+H):461.

<Example 0319>

<0319-1>

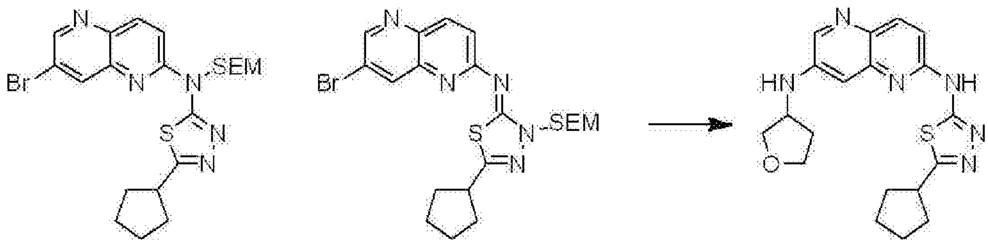

A mixture of a mixture (20 mg) of N-(7-bromo-1,5-naphthyridin-2-yl)-5-cyclopentyl-N-((2-(trimethylsilyl)ethoxy)methyl)-1,3,4-thiadiazole-2-amine and (Z)-7-bromo-N-(5-cyclopentyl-3-((2-(trimethylsilyl)ethoxy)methyl)-1,3,4-thiadiazol-2(3H)-ylidene)-1,5-naphthyridine-2-amine, tetrahydrofuran-3-amine (6.9 mg), tris(dibenzylideneacetone)dipalladium(0) (3.6 mg), 2-dicyclohexylphosphino-2',4',6'-triisopropylbiphenyl (3.8 mg), cesium carbonate (25.7 mg), and 1,4-dioxane (1 mL) was stirred at 100°C for 14 hours in a nitrogen atmosphere in a sealed tube. The reaction mixture was cooled to room temperature, and methanol (1 mL) and a 4 mol/L hydrogen chloride/1,4-dioxane solution (1 mL) were added thereto, followed by stirring at room temperature for 2 days. The solvent was distilled off under reduced pressure, triethylamine was added to the obtained residue, and the solvent was distilled off under reduced pressure. The obtained residue was purified by silica gel column chromatography (chloroform-methanol, NH silica), thereby obtaining $N^2$-(5-cyclopentyl-1,3,4-thiadiazol-2-yl)-$N^7$-(tetrahydrofuran-3-yl)-1,5-naphthyridine-2,7-diamine (2.0 mg) as a yellow solid.
$^1$H-NMR(DMSO-$d_6$)δ:11.76(1H,brs),8.31(1H,d,J=2.3Hz),8.01(1H,d,J=8.9Hz),7.05(1H,d,J=8.9Hz),6.94(1H,d,J=2.3Hz),6.84(1H,d,J=6.6Hz),4.27-4.14(1H,m),3.96(1H,dd,J=8.9,5.6Hz),3.92-3.83(1H,m),3.78(1H,td,J=8.3,5.5Hz),3.63(1H,dd,J=8.9,3.6Hz),3.52-3.38(1H,m),2.36-2.21(1H,m),2.20-2.04(2H,m),1.93-1.60(7H,m).
MSm/z(M+H):383.

<Example 0320>

<0320-1>

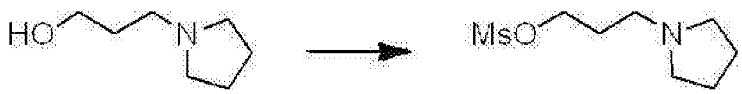

Pyridine (2.1 mL) and methanesulfonyl chloride (1.0 mL) were added to a solution of 3-(pyrrolidin-1-yl)propan-1-ol (1.14 g) in dichloromethane (18 mL) in an ice bath, followed by stirring for 30 minutes in an ice bath. A saturated sodium hydrogen carbonate aqueous solution was added to the reaction mixture, followed by stirring at room temperature for 30 minutes. The solvent was distilled off under reduced pressure, and the obtained residue was purified by silica gel column chromatography (ethyl acetate-methanol, NH silica), thereby obtaining 3-(pyrrolidin-1-yl)propyl methanesulfonate (1.60 g) as yellow oily substance.
$^1$H-NMR($CDCl_3$)δ:4.32(2H,t,J=6.6Hz),3.02(3H,s),2.56(2H,t,J=7.3Hz),2.53-2.44(4H,m),2.03-1.88(2H,m),1.86-1.71(4H,m).

<0320-2>

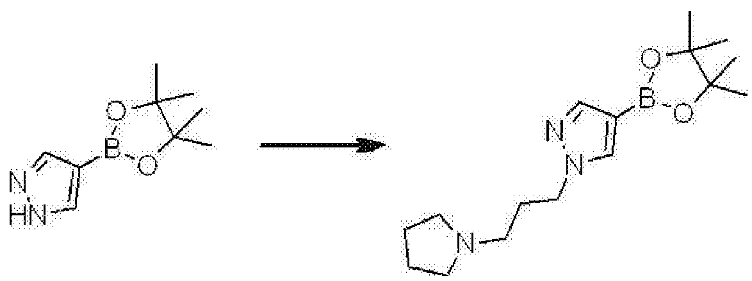

1-(3-(Pyrrolidin-1-yl)propyl)-4-(4,4,5,5-tetramethyl-1,3,2-dioxaborolan-2-yl)-1H-pyrazole was obtained as yellow oily substance in the same manner as in Example 0316 except that 3-(pyrrolidin-1-yl)propyl methanesulfonate was used instead of the 3-bromopropyl acetate used in Example 0316.
$^1$H-NMR($CDCl_3$)δ:7.78(1H,s),7.69(1H,s),4.20(2H,t,J=6.9Hz),2.53-2.37(6H,m),2.14-1.98(2H,m),1.84-1.69(4H,m),1.32(12H,s).

<0320-3>

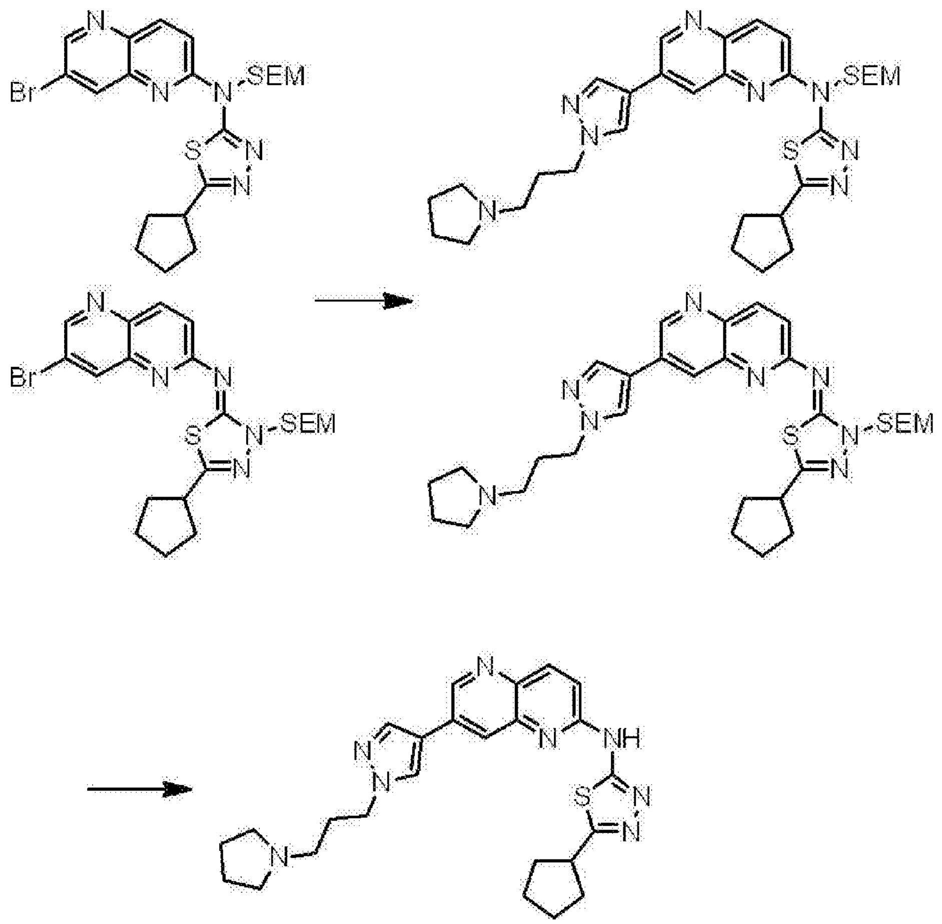

5-Cyclopentyl-N-(7-(1-(3-(pyrrolidin-1-yl)propyl)-1H-pyrazol-4-yl)-1,5-naphthyridin-2-yl)-1,3,4-thiadiazole-2-amine was obtained as a white solid in the same manner as in Example 0303 except that 1-(3-(pyrrolidin-1-yl)propyl)-4-(4,4,5,5-tetramethyl-1,3,2-dioxaborolan-2-yl)-1H-pyrazole was used instead of the tert-butyl 4-(3-(4-(4,4,5,5-tetramethyl-1,3,2-dioxaborolan-2-yl)-1H-pyrazol-1-yl)propyl)piperazine-1-carboxylate used in Example 0303.
$^1$H-NMR(DMSO-$d_6$)δ:12.04(1H,brs),9.08(1H,d,J=1.7Hz),8.57(1H,s),8.33(1H,d,J=1.7Hz),8.30-8.16(2H,m),7.39(1H,d,J=8.6Hz),4.22(2H,t,J=6.9Hz),3.57-3.34(1H,m),2.62-2.83(6H,m),2.26-1.52(14H,m).
MSm/z(M+H):475.

<Example 0321>

<0321-1>

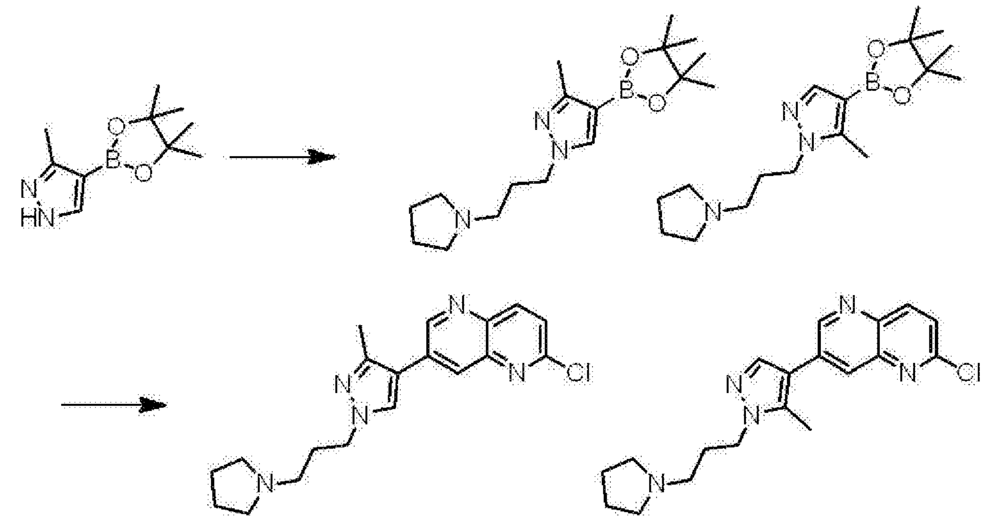

A mixture of 3-methyl-1-(3-(pyrrolidin-1-yl)propyl)-4-(4,4,5,5-tetramethyl-1,3,2-dioxaborolan-2-yl)-1H-pyrazole and 5-methyl-1-(3-(pyrrolidin-1-yl)propyl)-4-(4,4,5,5-tetramethyl-1,3,2-dioxaborolan-2-yl)-1H-pyrazole was obtained as colorless oily substance in the same manner as in Example 0316 except that 3-methyl-4-(4,4,5,5-tetramethyl-1,3,2-dioxaborolan-2-yl)-1H-pyrazole was used instead of the 4-(4,4,5,5-tetramethyl-1,3,2-dioxaborolan-2-yl)-1H-pyrazole used in Example 0316, and 1-(3-chloropropyl)pyrrolidine was used instead of the 3-bromopropyl acetate used in Example 0316.

A mixture of the obtained mixture (59.8 mg) of 3-methyl-1-(3-(pyrrolidin-1-yl)propyl)-4-(4,4,5,5-tetramethyl-1,3,2-dioxaborolan-2-yl)-1H-pyrazole and 5-methyl-1-(3-(pyrrolidin-1-yl)propyl)-4-(4,4,5,5-tetramethyl-1,3,2-dioxaborolan-2-yl)-1H-pyrazole, 7-bromo-2-chloro-1,5-naphthyridine (38.0 mg), bis(di-tert-butyl (4-dimethylaminophenyl)phosphine)dichloropalladium(II) (11.1 mg), sodium carbonate (33.1 mg), 1,4-dioxane (1 mL), and water (0.1 mL) was stirred at 100°C for 64 hours in a nitrogen atmosphere in a sealed tube. The reaction mixture was cooled to room temperature, and the solvent was distilled off under reduced pressure. After the obtained residue was purified by silica gel column chromatography (chloroform-methanol), the obtained residue was purified by preparative thin layer silica gel chromatography (chloroform-methanol, NH silica), thereby obtaining 2-chloro-7-(3-methyl-1-(3-(pyrrolidin-1-yl)propyl)-1H-pyrazol-4-yl)-1,5-naphthyridine (12.2 mg) as colorless oily substance and 2-chloro-7-(5-methyl-1-(3-(pyrrolidin-1-yl)propyl)-1H-pyrazol-4-yl)-1,5-naphthyridine (9.4 mg) as colorless oily substance.
2-Chloro-7-(3-methyl-1-(3-(pyrrolidin-1-yl)propyl)-1H-pyrazol-4-yl)-1,5-naphthyridine MSm/z(M+H):356,358.
2-Chloro-7-(5-methyl-1-(3-(pyrrolidin-1-yl)propyl)-1H-pyrazol-4-yl)-1,5-naphthyridine MSm/z(M+H):356,358.

<0321-3>

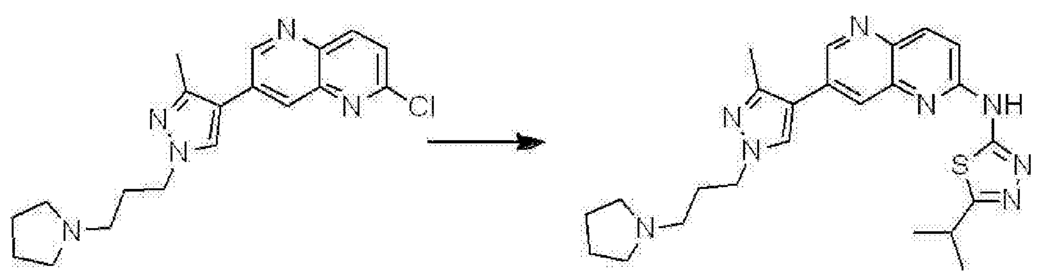

A mixture of 2-chloro-7-(3-methyl-1-(3-(pyrrolidin-1-yl)propyl)-1H-pyrazol-4-yl)-1,5-naphthyridine (12.2 mg), 5-isopropyl-1,3,4-thiadiazole-2-amine (9.8 mg), tris(dibenzylideneacetone)dipalladium(0) (3.1 mg), 2-dicyclohexylphosphino-2',4',6'-triisopropylbiphenyl (4.0 mg), cesium carbonate (22.4 mg), and 1,4-dioxane (0.5 mL) was stirred at 100°C for 13 hours in a nitrogen atmosphere in a sealed tube. The reaction mixture was cooled to room temperature, and the solvent was distilled off under reduced pressure. The obtained residue was purified by preparative thin layer silica gel chromatography (chloroform-methanol, NH silica), thereby obtaining 5-isopropyl-N-(7-(3-methyl-1-(3-(pyrrolidin-1-yl)propyl)-1H-pyrazol-4-yl)-1,5-naphthyridin-2-yl)-1,3,4-thiadiazole-2-amine (9.0 mg) as a yellow solid.

$^1$H-NMR(DMSO-$d_6$)δ:12.06(1H,brs),8.92(1H,d,J=2.0Hz),8.31(1H,s),8.27(1H,d,J=9.2Hz),8.16(1H,d,J=2.0Hz),7.42(1H,d ,J=9.2Hz),4.13(2H,t,J=6.9Hz),3.42-3.30(1H,m),2.47-2.35(9H,m),2.05-1.91(2H,m),1.75-1.63(4H,m),1.40(6H,d,J=7.3Hz).
MSm/z(M+H):463.

<Example 0322>

<0322-1>

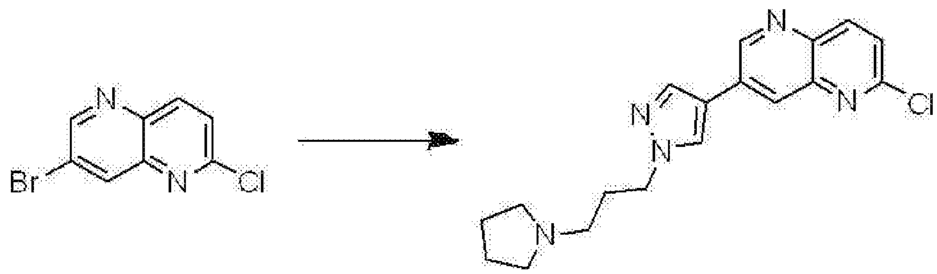

2-Chloro-7-(1-(3-(pyrrolidin-1-yl)propyl)-1H-pyrazol-4-yl)-1,5-naphthyridine was obtained as a white solid in the same manner as in Example 0301 except that 1-(3-(pyrrolidin-1-yl)propyl)-4-(4,4,5,5-tetramethyl-1,3,2-dioxaborolan-2-yl)-1H-pyrazole was used instead of the N,N-dimethyl-3-(4-(4,4,5,5-tetramethyl-1,3,2-dioxaborolan-2-yl)-1H-pyrazol-1-yl)propane-1-amine used in Example 0301.
MSm/z(M+H):342,344.

<0322-2>

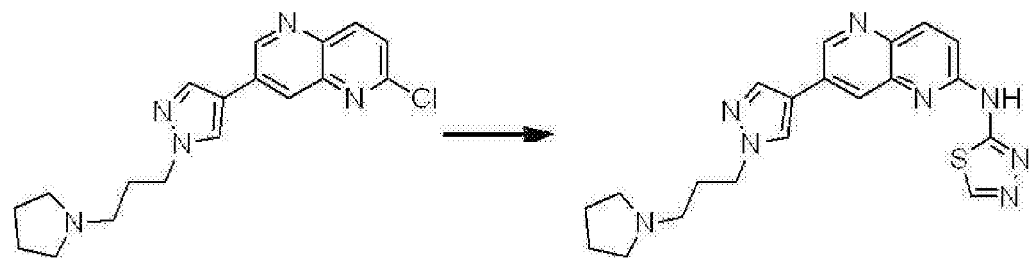

N-(7-(1-(3-(pyrrolidin-1-yl)propyl)-1H-pyrazol-4-yl)-1,5-naphthyridin-2-yl)-1,3,4-thiadiazole-2-amine was obtained as a white solid in the same manner as in Example 0301 except that 2-chloro-7-(1-(3-(pyrrolidin-1-yl)propyl)-1H-pyrazol-4-yl)-1,5-naphthyridine was used instead of the 3-(4-(6-chloro-1,5-naphthyridin-3-yl)-1H-pyrazol-1-yl)-N,N-dimethylpropane-1-amine used in Example 0301.
$^1$H-NMR(DMSO-$d_6$)δ:12.23(1H,brs),9.17(1H,s),9.10(1H,d,J=2.0Hz),8.57(1H,s),8.34(1H,d,J=2.0Hz),8.28(1H,d,J=9.2Hz ),8.21(1H,s),7.44(1H,d,J=9.2Hz),4.21(2H,t,J=7.3Hz),2.48-2.34(6H,m),2.10-1.94(2H,m),1.77-1.60(4H,m).
MSm/z(M+H):407.

<Example 0323>

<0323-1>

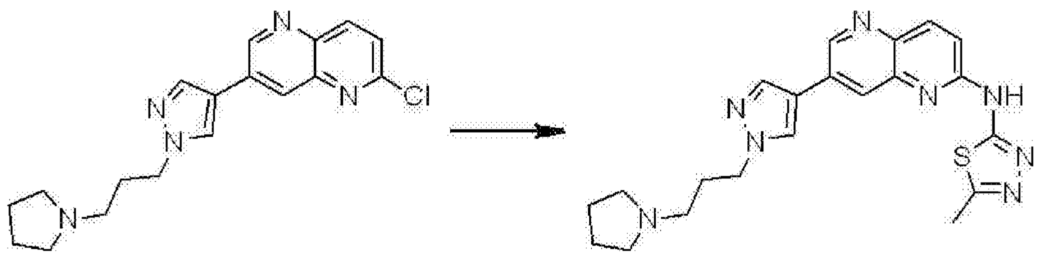

5-Methyl-N-(7-(1-(3-(pyrrolidin-1-yl)propyl)-1H-pyrazol-4-yl)-1,5-naphthyridin-2-yl)-1,3,4-thiadiazole-2-amine was obtained as a white solid in the same manner as in Example 0301 except that 2-chloro-7-(1-(3-(pyrrolidin-1-yl)propyl)-1H-pyrazol-4-yl)-1,5-naphthyridine was used instead of the 3-(4-(6-chloro-1,5-naphthyridin-3-yl)-1H-pyrazol-1-yl)-N,N-dimethylpropane-1-amine used in Example 0301, and 5-methyl-1,3,4-thiadiazole-2-amine was used instead of the 1,3,4-thiadiazole-2-amine used in Example 0301.
$^1$H-NMR(DMSO-$d_6$)$\delta$:12.02(1H,brs),9.08(1H,d,J=1.7Hz),8.58(1H,s),8.33(1H,d,J=1.7Hz),8.25(1H,d,J=8.6Hz),8.22(1H,s ),7.39(1H,d,J=8.6Hz),4.21(2H,t,J=6.9Hz),2.66(3H,s),2.49-2.34(6H,m),2.10-1.94(2H,m),1.77-1.60(4H,m).
MSm/z(M+H):421.

<Example 0324>

<0324-1>

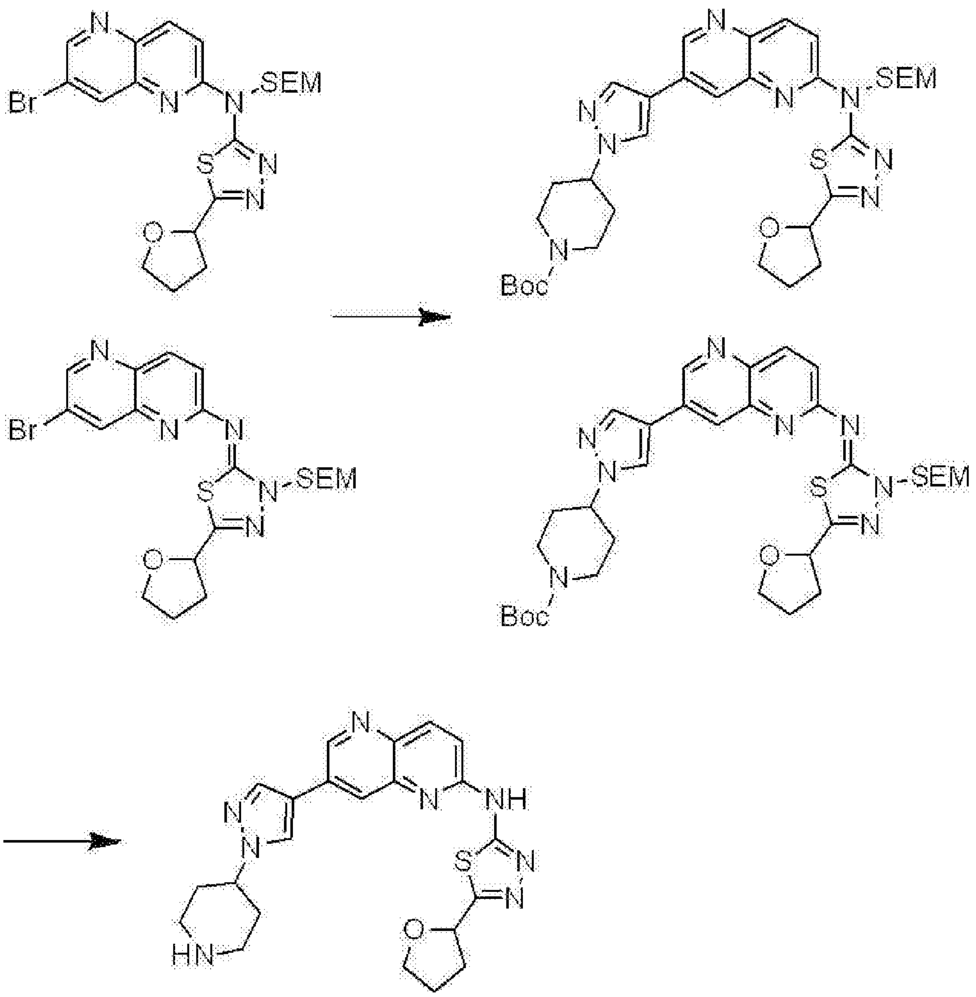

A solution of a mixture (30 mg) of N-(7-bromo-1,5-naphthyridin-2-yl)-5-(tetrahydrofuran-2-yl)-N-((2-(trimethylsilyl)ethoxy)methyl)-1,3,4-thiadiazole-2-amine and (Z)-7-bromo-N-(5-(tetrahydrofuran-2-yl)-3-((2-(trimethylsilyl)ethoxy)methyl)-1,3,4-thiadiazol-2(3H)-ylidene)-1,5-naphthyridine-2-amine, bis(pinacolato)diboron (22.5 mg), (1,1'-bis(diphenylphosphino)ferrocene)palladium(II) dichloride (4.8 mg), and potassium acetate (11.6 mg) in 1,4-dioxane (1 mL) was stirred at 100°C for 12 hours in a nitrogen atmosphere in a sealed tube. The reaction mixture was cooled to room temperature, and tert-butyl 4-(4-iodo-1H-pyrazol-1-yl)piperidine-1-carboxylate (33.4 mg), bis(di-tert-butyl (4-dimethylaminophenyl)phosphine)dichloropalladium(II) (4.2 mg), sodium carbonate (31.3 mg), and water (0.1 mL) were added thereto, followed by stirring at 120°C for 19 hours. The reaction mixture was cooled to room temperature, and purified by silica gel column chromatography (hexane-ethyl acetate, NH silica), thereby obtaining a mixture (61.2 mg) of tert-butyl 4-(4-(6-((5-(tetrahydrofuran-2-yl)-1,3,4-thiadiazol-2-yl)((2-(trimethylsilyl)ethoxy)methyl)amino)-1,5-naphthyridin-3-yl)-1H-pyrazol-1-yl)piperidine-1-carboxylate and (Z)-tert-butyl 4-(4-(6-((5-(tetrahydrofuran-2-yl)-3-((2-(trimethylsilyl)ethoxy)methyl)-1,3,4-thiadiazol-2(3H)-ylidene)amino)-1,5-naphthyridin-3-yl)-1H-pyrazol-1-yl)piperidine-1-carboxylate as brown oily substance.

A 4 mol/L hydrogen chloride/1,4-dioxane solution (1 mL) was added to a solution of the obtained mixture (20 mg) in ethanol (1 mL) at room temperature, followed by stirring for 2 hours. The solvent was distilled off under reduced pressure, triethylamine was added to the obtained residue, and the solvent was distilled off under reduced pressure. The obtained residue was purified by silica gel column chromatography (chloroform-methanol, NH silica), thereby obtaining N-(7-(1-(piperidin-4-yl)-1H-pyrazol-4-yl)-1,5-naphthyridin-2-yl)-5-(tetrahydrofuran-2-yl)-1,3,4-thiadiazole-2-amine (1.4 mg) as a yellow solid.

$^1$H-NMR(DMSO-$d_6$)δ:9.11(1H,d,J=2.0Hz),8.65(1H,s),8.34(1H,d,J=2.0Hz),8.30-8.21(2H,m),7.41(1H,d,J=9.2Hz),5.24(1H,dd,J=7.3,5.9Hz),4.36-4.20(1H,m),4.07-3.96(1H,m),3.95-3.83(1H,m),3.17-3.04(2H,m),2.75-2.60(2H,m),2.46-2.32(1H,m),2.30-2.13(1H,m),2.11-1.96(4H,m),1.96-1.79(2H,m).

MSm/z(M+H):449.

<Example 0325>

<0325-1>

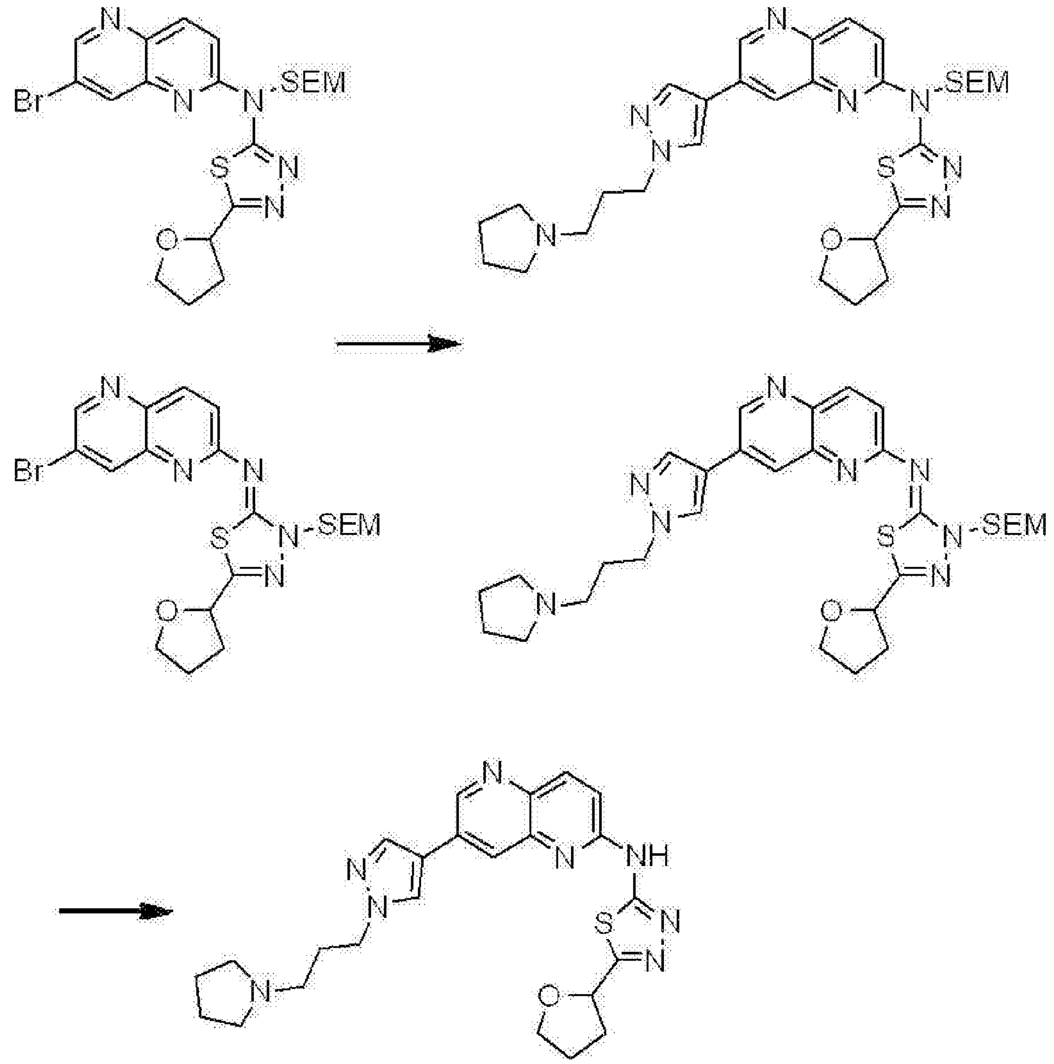

N-(7-(1-(3-(pyrrolidin-1-yl)propyl)-1H-pyrazol-4-yl)-1,5-naphthyridin-2-yl)-5-(tetrahydrofuran-2-yl)-1,3,4-thiadiazole-2-amine was obtained as a white solid in the same manner as in Example 0303 except that a mixture of N-(7-bromo-1,5-naphthyridin-2-yl)-5-(tetrahydrofuran-2-yl)-N-((2-(trimethylsilyl)ethoxy)methyl)-1,3,4-thiadiazole-2-amine and (Z)-7-bromo-N-(5-(tetrahydrofuran-2-yl)-3-((2-(trimethylsilyl)ethoxy)methyl)-1,3,4-thiadiazol-2(3H)-ylidene)-1,5-naphthyridine-2-amine was used instead of the mixture of N-(7-bromo-1,5-naphthyridin-2-yl)-5-cyclopentyl-N-((2-(trimethylsilyl)ethoxy)methyl)-1,3,4-thiadiazole-2-amine and (Z)-7-bromo-N-(5-cyclopentyl-3-((2-(trimethylsilyl)ethoxy)methyl)-1,3,4-thiadiazol-2(3H)-ylidene)-1,5-naphthyridine-2-amine used in Example 0303, and 1-(3-(pyrrolidin-1-yl)propyl)-4-(4,4,5,5-tetramethyl-1,3,2-dioxaborolan-2-yl)-1H-pyrazole was used instead of the tert-butyl 4-(3-(4-(4,4,5,5-tetramethyl-1,3,2-dioxaborolan-2-yl)-1H-pyrazol-1-yl)propyl)piperazine-1-carboxylate used in Example 0303.
$^1$H-NMR(DMSO-$d_6$)$\delta$:12.12(1H,brs),9.09(1H,d,J=1.7Hz),8.59(1H,s),8.32(1H,d,J=1.7Hz),8.27(1H,d,J=8.9Hz),8.24(1H,s ),7.41(1H,d,J=8.9Hz),5.24(1H,dd,J=7.3,5.9Hz),4.22(2H,t,J=6.9Hz),4.07-3.96(1H,m),3.94-3.83(1H,m),2.61-2.32(7H,m),2.31-2.13(1H,m),2.12-1.95(4H,m),1.79-1.58(4H,m).
MSm/z(M+H):477.

<Example 0326>

<0326-1>

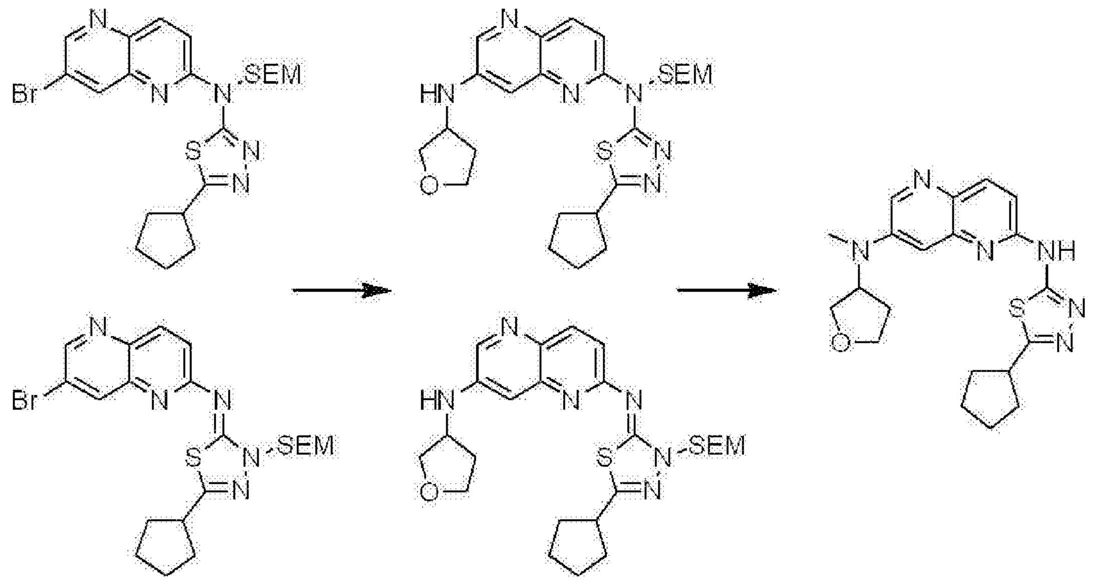

A mixture of a mixture (20 mg) of N-(7-bromo-1,5-naphthyridin-2-yl)-5-cyclopentyl-N-((2-(trimethylsilyl)ethoxy)methyl)-1,3,4-thiadiazole-2-amine and (Z)-7-bromo-N-(5-cyclopentyl-3-((2-(trimethylsilyl)ethoxy)methyl)-1,3,4-thiadiazol-2(3H)-ylidene)-1,5-naphthyridine-2-amine, tetrahydrofuran-3-amine (6.9 mg), tris(dibenzylideneacetone)dipalladium(0) (3.6 mg), 2-dicyclohexylphosphino-2',4',6'-triisopropylbiphenyl (3.8 mg), cesium carbonate (25.7 mg), and 1,4-dioxane (1 mL) was stirred at 100°C for 12 hours in a sealed tube. The reaction mixture was cooled to room temperature, and purified by silica gel column chromatography (hexane-ethyl acetate, NH silica), thereby obtaining a mixture (13.7 mg) of $N^2$-(5-cyclopentyl-1,3,4-thiadiazol-2-yl)-$N^7$-(tetrahydrofuran-3-yl)-$N^2$-((2-(trimethylsilyl)ethoxy)methyl)-1,5-naphthyridine-2,7-diamine and (Z)-$N^2$-(5-cyclopentyl-3-((2-(trimethylsilyl)ethoxy)methyl)-1,3,4-thiadiazol-2(3H)-ylidene)-$N^7$-(tetrahydrofuran-3-yl)-1,5-naphthyridine-2,7-diamine as yellow oily substance.

60% sodium hydride (1.6 mg) was added to a solution of the obtained mixture in tetrahydrofuran (1 mL) in an ice bath, followed by stirring at room temperature for 5 minutes, and iodomethane (6.6 mL) was added thereto in an ice bath. The reaction mixture was stirred at room temperature for 1 hour, and heated to reflux for 1 hour. The reaction mixture was cooled to room temperature, and methanol (1 mL) and a 4 mol/L hydrogen chloride/1,4-dioxane solution (1 mL) were added thereto, followed by stirring at room temperature for 2 hours. The solvent was distilled off under reduced pressure, triethylamine was added to the obtained residue, and the solvent was distilled off under reduced pressure. The obtained residue was purified by silica gel column chromatography (chloroform-methanol, NH silica), thereby obtaining $N^2$-(5-cyclopentyl-1,3,4-thiadiazol-2-yl)-$N^7$-methyl-$N^7$-(tetrahydrofuran-3-yl)-1,5-naphthyridine-2,7-diamine (7.6 mg) as a white solid.

$^1$H-NMR(DMSO-$d_6$)$\delta$:11.82(1H,s),8.66(1H,d,J=2.6Hz),8.08(1H,d,J=8.6Hz),7.22(1H,d,J=2.6Hz),7.12(1H,d,J=8.6Hz),4.95-4.78(1H,m),4.07-3.92(1H,m),3.89-3.75(2H,m),3.73-3.58(1H,m),3.55-3.38(1H,m),2.96(3H,s),2.39-2.23(1H,m),2.22-2.03(2H,m),2.01-1.56(7H,m).

MSm/z(M+H):397.

<Example 0327>

<0327-1>

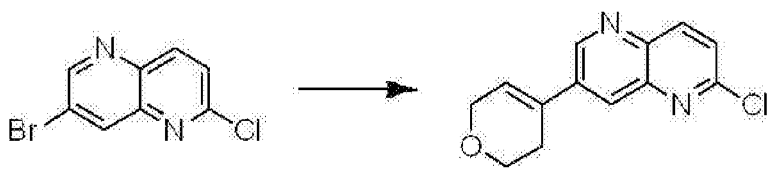

2-Chloro-7-(3,6-dihydro-2H-pyran-4-yl)-1,5-naphthyridine was obtained as a white solid in the same manner

as in Example 0301 except that 2-(3,6-dihydro-2H-pyran-4-yl)-4,4,5,5-tetramethyl-1,3,2-dioxaborolane was used instead of the N,N-dimethyl-3-(4-(4,4,5,5-tetramethyl-1,3,2-dioxaborolan-2-yl)-1H-pyrazol-1-yl)propane-1-amine used in Example 0301.
MSm/z(M+H):247.

<0327-2>

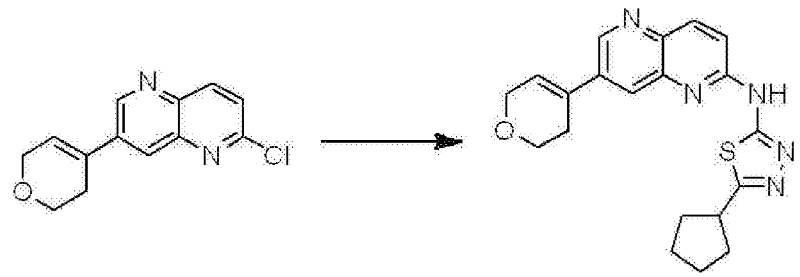

5-Cyclopentyl-N-(7-(3,6-dihydro-2H-pyran-4-yl)-1,5-naphthyridin-2-yl)-1,3,4-thiadiazole-2-amine was obtained as a white solid in the same manner as in Example 0301 except that 2-chloro-7-(3,6-dihydro-2H-pyran-4-yl)-1,5-naphthyridine was used instead of the 3-(4-(6-chloro-1,5-naphthyridin-3-yl)-1H-pyrazol-1-yl)-N,N-dimethylpropane-1-amine used in Example 0301, and 5-cyclopentyl-1,3,4-thiadiazole-2-amine was used instead of the 1,3,4-thiadiazole-2-amine used in Example 0301.
$^1$H-NMR(DMSO-$d_6$)δ:12.05(1H,s),8.98(1H,d,J=2.3Hz),8.27(1H,d,J=8.9Hz),8.10(1H,d,J=2.3Hz),7.43(1H,d,J=8.9Hz),6.72-6.64(1H,m),4.36-4.27(2H,m),3.90(2H,t,J=5.6Hz),3.57-3.39(1H,m),2.70-2.58(2H,m),2.23-2.04(2H,m),1.96-1.58(6H,m).
MSm/z(M+H):380.

<Example 0328>

<0328-1>

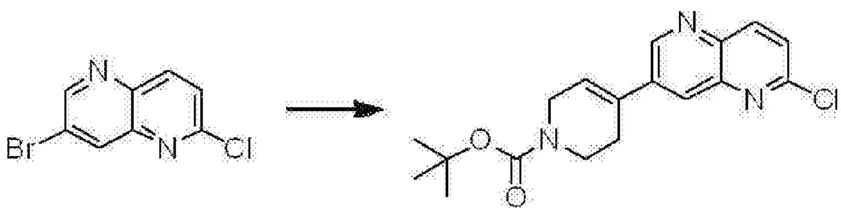

tert-Butyl 4-(6-chloro-1,5-naphthyridin-3-yl)-5,6-dihydropyridine-1(2H)-carboxylate was obtained as a white solid in the same manner as in Example 0298 except that tert-butyl 4-(4,4,5,5-tetramethyl-1,3,2-dioxaborolan-2-yl)-5,6-dihydropyridine-1(2H)-carboxylate was used instead of the N,N-dimethyl-3-(4-(4,4,5,5-tetramethyl-1,3,2-dioxaborolan-2-yl)-1H-pyrazol-1-yl)propane-1-amine used in Example 0301.
MSm/z(M+H):346.

<0328-2>

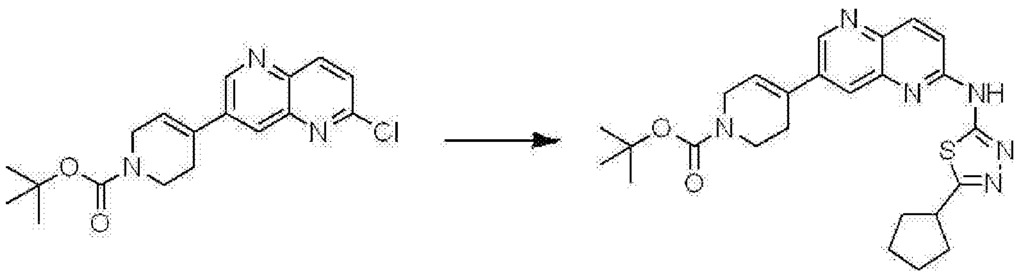

tert-Butyl 4-(6-((5-cyclopentyl-1,3,4-thiadiazol-2-yl)amino)-1,5-naphthyridin-3-yl)-5,6-dihydropyridine-1(2H)-carboxylate was obtained as a yellow solid in the same manner as in Example 0301 except that tert-butyl 4-(6-chloro-1,5-naphthyridin-3-yl)-5,6-dihydropyridine-1(2H)-carboxylate was used instead of the 3-(4-(6-chloro-1,5-naphthyridin-3-yl)-1H-pyrazol-1-yl)-N,N-dimethylpropane-1-amine used in Example 0301, and 5-cyclopentyl-1,3,4-thiadiazole-2-amine was used instead of the 1,3,4-thiadiazole-2-amine used in Example 0301.
$^1$H-NMR(DMSO-$d_6$)δ:12.05(1H,brs),8.95(1H,d,J=2.0Hz),8.27(1H,d,J=9.2Hz),8.11(1H,d,J=2.0Hz),7.43(1H,d,J=9.2Hz),6.65-6.46(1H,m),4.17-4.02(2H,m),3.68-3.55(2H,m),3.55-3.39(1H,m),2.71-2.59(2H,m),2.24-2.06(2H,m),1.96-1.61(6H,m),1.45(9H,s).
MSm/z(M+H):479.

<Example 0329>

<0329-1>

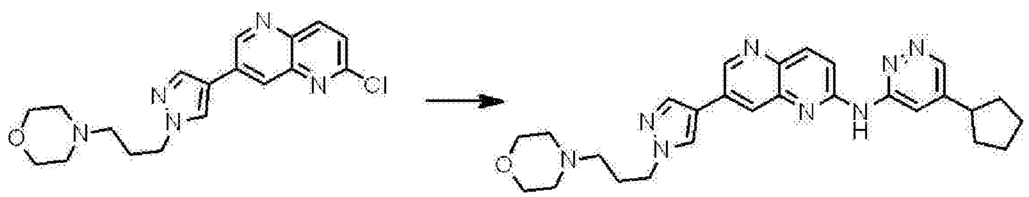

N-(5-cyclopentylpyridazin-3-yl)-7-(1-(3-morpholinopropyl)-1H-pyrazol-4-yl)-1,5-naphthyridine-2-amine was obtained as a white solid in the same manner as in Example 0001 except that 5-cyclopentylpyridazine-3-amine was used instead of the 1,3,4-thiadiazole-2-amine used in Example 0001.
$^1$H-NMR($CDCl_3$)δ:9.12(1H,brs),8.92(1H,d,J=1.8Hz),8.86(1H,m),8.82(1H,d,J=2.1Hz),8.24(1H,d,J=9.3Hz) ,8.09(1H,d,J=2.1Hz),7.97(1H,s),7.86(1H,s),7.58(1H,d,J=9.3Hz),4.31(2H,m),3.73(4H,m),3.15(1H,m),2 .50-2.34(6H,m),2.25(2H,m),2.13(2H,m),2.00-1.70(6H,m).
MSm/z(M+H):485.

<Example 0330>

<0330-1>

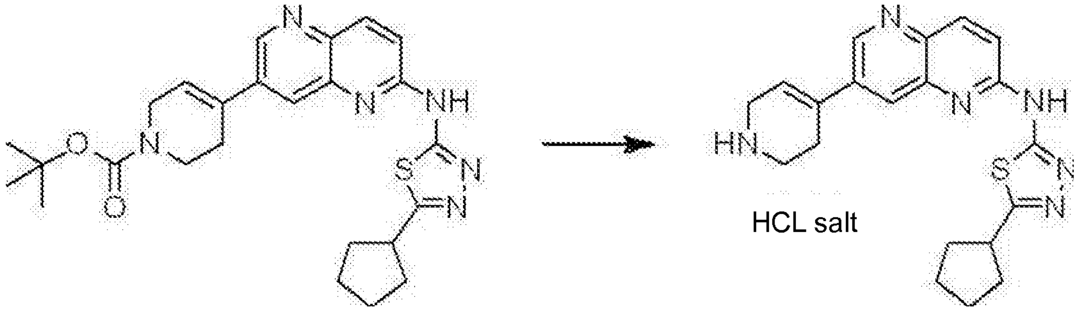


A 4 mol/L hydrogen chloride/1,4-dioxane solution (1 mL) was added to a solution of tert-butyl 4-(6-((5-cyclopentyl-1,3,4-thiadiazol-2-yl)amino)-1,5-naphthyridin-3-yl)-5,6-dihydropyridine-1(2H)-carboxylate (20 mg) in methanol (1 mL) at room temperature, followed by stirring for 1 hour. The solvent was distilled off under reduced pressure, and the obtained residue was washed with ethyl acetate, thereby obtaining 5-cyclopentyl-N-(7-(1,2,3,6-tetrahydropyridin-4-yl)-1,5-naphthyridin-2-yl)-1,3,4-thiadiazole-2-amine hydrochloride (12 mg) as a white solid.
$^1$H-NMR(DMSO-$d_6$)δ:9.13(2H,brs),9.00(1H,d,J=2.0Hz),8.30(1H,d,J=9.2Hz),8.18(1H,d,J=2.0Hz),7.47(1H,d,J=9.2Hz),6.65-6.58(1H,m),3.55-3.34(3H,m),2.94-2.82(2H,m),2.23-2.08(2H,m),1.94-1.59(8H,m).
MSm/z(M+H):379.

<Example 0331>

<0331-1>

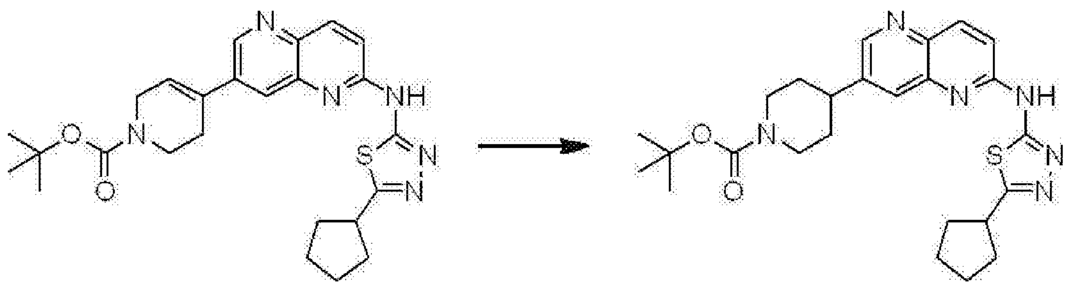

A mixture of tert-butyl 4-(6-((5-cyclopentyl-1,3,4-thiadiazol-2-yl)amino)-1,5-naphthyridin-3-yl)-5,6-dihydropyridine-1(2H)-carboxylate (30 mg), methanol (3 mL), and tetrahydrofuran (1 mL) was reacted using a flow-type hydrogenation reaction apparatus (atmospheric pressure, 1.0 mL/min, room temperature, 10% Pd/C). The solvent was distilled off under reduced pressure, and the obtained residue was washed with ethyl acetate, thereby obtaining tert-butyl 4-(6-((5-cyclopentyl-1,3,4-thiadiazol-2-yl)amino)-1,5-naphthyridin-3-yl)piperidine-1-carboxylate (8.3 mg) as a white solid.
$^1$H-NMR(DMSO-$d_6$)δ:12.02(1H,s),8.72(1H,d,J=2.3Hz),8.26(1H,d,J=9.2Hz),8.04(1H,d,J=2.3Hz),7.42(1H,d,J=9.2Hz),4.24-4.06(2H,m),3.56-3.38(1H,m),3.10-2.74(3H,m),2.22-2.01(2H,m),1.96-1.58(10H,m),1.44(9H,s).
MSm/z(M+H):481.

<Example 0332>

<0332-1>

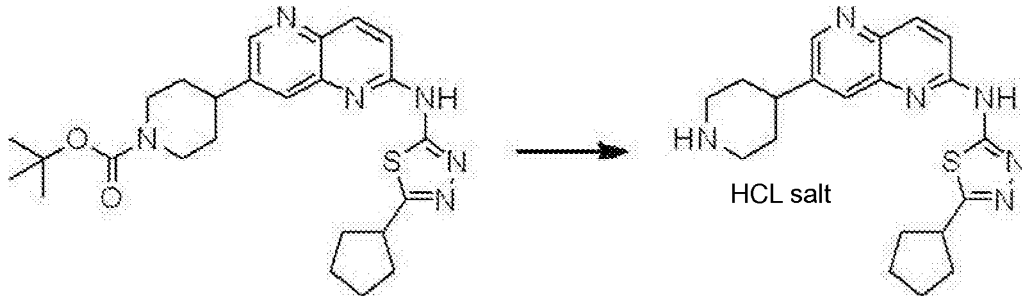


5-Cyclopentyl-N-(7-(piperidin-4-yl)-1,5-naphthyridin-2-yl)-1,3,4-thiadiazole-2-amine hydrochloride was obtained as a white solid in the same manner as in Example 0330 except that tert-butyl 4-(6-((5-cyclopentyl-1,3,4-thiadiazol-2-yl)amino)-1,5-naphthyridin-3-yl)piperidine-1-carboxylate was used instead of the tert-butyl 4-(6-((5-cyclopentyl-1,3,4-thiadiazol-2-yl)amino)-1,5-naphthyridin-3-yl)-5,6-dihydropyridine-1(2H)-carboxylate used in Example 0330.
$^1$H-NMR(DMSO-$d_6$)δ:12.11(1H,brs),8.85-8.40(3H,m),8.29(1H,d,J=8.9Hz),7.99(1H,d,J=2.0Hz),7.46(1H,d,J=8.9Hz),3.78-3.25(2H,m),3.24-2.96(3H,m),2.23-1.60(13H,m).
MSm/z(M+H):381.

<Example 0333>

<0333-1>

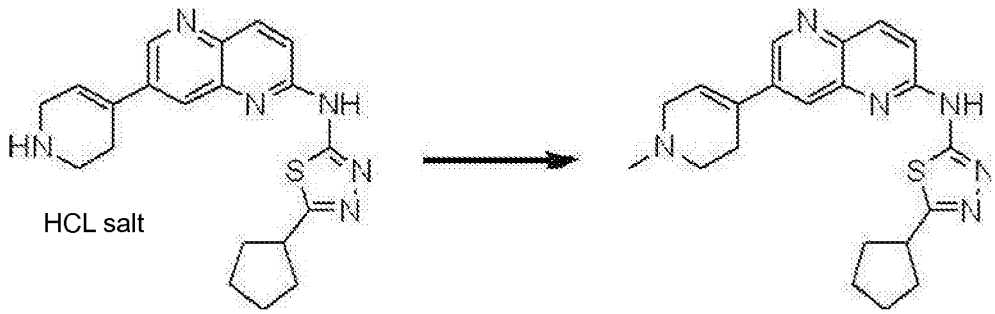


A36 to 38% formaldehyde aqueous solution (0.2 mL) and sodium triacetoxyborohydride (15.3 mg) were added to a solution of 5-cyclopentyl-N-(7-(1,2,3,6-tetrahydropyridin-4-yl)-1,5-naphthyridin-2-yl)-1,3,4-thiadiazole-2-amine hydrochloride (3.0 mg) in methanol (0.5 mL) and dichloromethane (0.5 mL) at room temperature, followed by stirring for 2 hours. The solvent was distilled off under reduced pressure, and the obtained residue was purified by silica gel column chromatography (chloroform-methanol, NH silica), thereby obtaining 5-cyclopentyl-N-(7-(1-methyl-1,2,3,6-tetrahydropyridin-4-yl)-1,5-naphthyridin-2-yl)-1,3,4-thiadiazole-2-amine (1.7 mg) as a white solid.
$^1$H-NMR(DMSO-$d_6$)δ:12.04(1H,brs),8.96(1H,d,J=2.3Hz),8.26(1H,d,J=9.2Hz),8.08(1H,d,J=2.3Hz),7.41(1H,d,J=9.2Hz),6.57(1H,bt,J=3.3Hz),3.56-3.39(1H,m),3.10(2H,bd,J=3.3Hz),2.71-2.59(4H,m),2.32(3H,s),2.24-2.06(2H,m),1.97-1.59(6H,m).
MSm/z(M+H):393.

<Example 0334>

<0334-1>

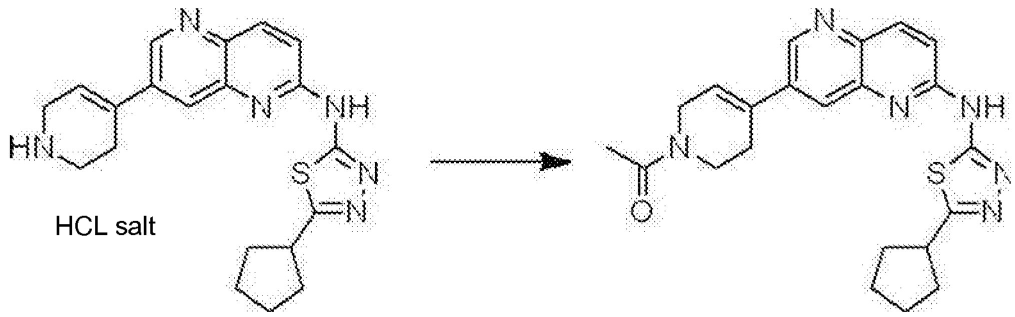


Acetyl chloride (2 μL) was added to a solution of 5-cyclopentyl-N-(7-(1,2,3,6-tetrahydropyridin-4-yl)-1,5-naphthyridin-2-yl)-1,3,4-thiadiazole-2-amine hydrochloride (4.0 mg) in pyridine (0.5 mL) in an ice bath, followed by stirring at room temperature for 1 hour. A saturated sodium hydrogen carbonate aqueous solution was added to the reaction mixture, and the solvent was distilled off under reduced pressure. The obtained residue was purified by silica gel column chromatography (chloroform-methanol, NH silica), thereby obtaining 1-(4-(6-((5-cyclopentyl-1,3,4-thiadiazol-2-yl)amino)-1,5-naphthyridin-3-yl)-5,6-dihydropyridine-1(2H)-yl)ethanone (1.4 mg) as a white solid.
$^1$H-NMR(DMSO-$d_6$)δ:12.06(1H,brs),8.99-8.93(1H,m),8.27(1H,d,J=9.2Hz),8.14-8.08(1H,m),7.43(1H,d,J=9.2Hz),6.62-6.54(1H,m),4.27-4.15(2H,m),3.77-3.66(2H,m),3.57-3.40(1H,m),2.78-2.59(2H,m),2.22-2.04(5H,m),1.94-1.62(6H,m).
MSm/z(M+H):421.

<Example 0335>

<0335-1>

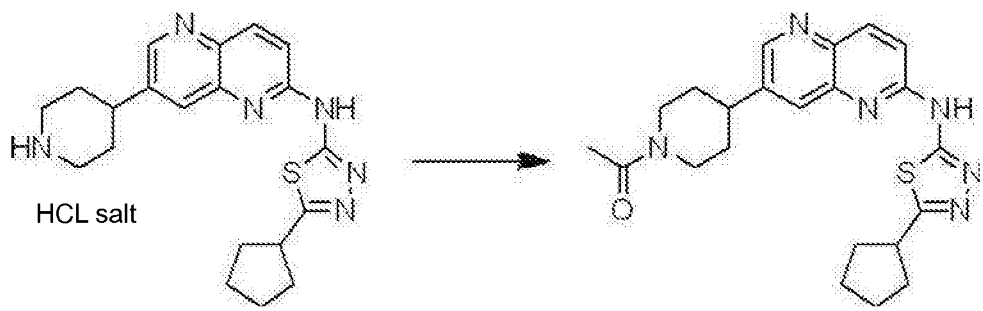


1-(4-(6-((5-Cyclopentyl-1,3,4-thiadiazol-2-yl)amino)-1,5-naphthyridin-3-yl)piperidin-1-yl)ethanone was obtained as a white solid in the same manner as in Example 0334 except that 5-cyclopentyl-N-(7-(piperidin-4-yl)-1,5-naphthyridin-2-yl)-1,3,4-thiadiazole-2-amine hydrochloride was used instead of the 5-cyclopentyl-N-(7-(1,2,3,6-tetrahydropyridin-4-yl)-1,5-naphthyridin-2-yl)-l,3,4-thiadiazole-2-amine hydrochloride used in Example 0334.
$^1$H-NMR(DMSO-$d_6$)δ:12.03(1H,brs),8.73(1H,d,J=2.0Hz),8.26(1H,d,J=8.9Hz),8.03(1H,d,J=2.0Hz),7.42(1H,d,J=8.9Hz), 4.66-4.53(1H,m),4.04-3.93(1H,m),3.55-3.39(1H,m),3.25-3.00(2H,m),2.71-2.56(1H,m),2.23-2.03(6H,m), 1.97-1.51(9H,m).
MSm/z(M+H):423.

<Example 0336>

<0336-1>

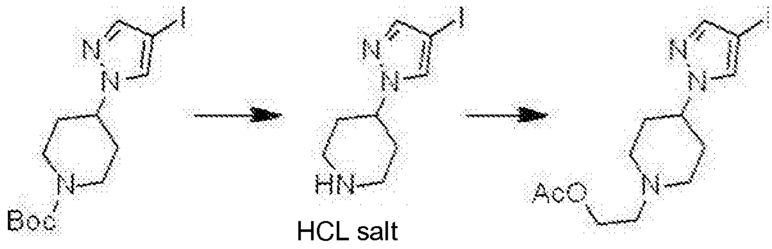


A4 mol/L hydrogen chloride/1,4-dioxane solution (1.5 mL) was added to a solution of tert-butyl 4-(4-iodo-1H-pyrazol-1-yl)piperidine-1-carboxylate (300 mg) in methanol (1.5 mL) at room temperature, followed by stirring for 1 hour. The solvent was distilled off under reduced pressure, hexane was added to the obtained residue, and the solid matter was collected by filtration, thereby obtaining 4-(4-iodo-1H-pyrazol-1-yl)piperidine hydrochloride (0.283 g) as a white solid.

60% sodium hydride (30.6 mg) was added to a solution of the obtained hydrochloride (100 mg) in N,N-dimethylformamide (1 mL) in an ice bath, followed by stirring at room temperature for 30 minutes. 2-Bromoethyl acetate (70 μL) was added to the reaction mixture at room temperature, followed by stirring at room temperature for 1 hour, and stirring at 80°C for 1 hour. The reaction mixture was cooled to room temperature, and water and ethyl acetate were added thereto. The organic layer was collected by separation, washed with water and a saturated sodium chloride aqueous solution, and dried over anhydrous sodium sulfate, and the solvent was distilled off under reduced pressure. The obtained residue was purified by silica gel column chromatography (hexane-ethyl acetate), thereby obtaining 2-(4-(4-iodo-1H-pyrazol-1-yl)piperidin-1-yl)ethyl acetate (62.1 mg) as colorless oily substance.
$^1$H-NMR($CDCl_3$)δ:7.50(1H,s),7.46(1H,s),4.20(2H,t,J=5.9Hz),3.11-2.99(2H,m),2.67(2H,t,J=5.9Hz),2.23(2H,td,J=11.7,2.4Hz),2.17-1.90(8H,m).

<0336-2>

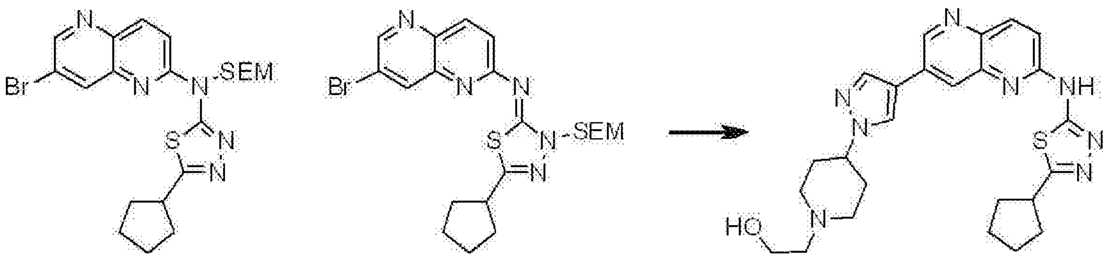

A mixture of a mixture (10 mg) of N-(7-bromo-1,5-naphthyridin-2-yl)-5-cyclopentyl-N-((2-(trimethylsilyl)ethoxy)methyl)-1,3,4-thiadiazole-2-amine and (Z)-7-bromo-N-(5-cyclopentyl-3-((2-(trimethylsilyl)ethoxy)methyl)-1,3,4-thiadiazol-2(3H)-ylidene)-1,5-naphthyridine-2-amine, bis(pinacolato)diboron (7.5 mg), (1,1'-bis(diphenylphosphino)ferrocene)palladium(II) dichloride (1.6 mg), potassium acetate (3.9 mg), and 1,4-dioxane (0.7 mL) was stirred at 100°C for 2 hours in a nitrogen atmosphere in a sealed tube. The reaction mixture was cooled to room temperature, and 2-(4-(4-iodo-1H-pyrazol-1-yl)piperidin-1-yl)ethyl acetate (8.6 mg), bis(di-tert-butyl (4-dimethylaminophenyl)phosphine)dichloropalladium(II) (1.4 mg), sodium carbonate (10.4 mg), and water (70 μL) were added thereto, followed by stirring at 100°C for 13 hours. The reaction mixture was cooled to room temperature, and methanol (1 mL) and a 4 mol/L hydrogen chloride/1,4-dioxane solution (1 mL) were added thereto, followed by stirring at room temperature for 2 hours. The solvent was distilled off under reduced pressure, triethylamine was added to the obtained residue, and the solvent was distilled off under reduced pressure. The obtained residue was purified by silica gel column chromatography (chloroform-methanol, NH silica), thereby obtaining 2-(4-(4-(6-((5-cyclopentyl-1,3,4-thiadiazol-2-yl)amino)-1,5-naphthyridin-3-yl)-1H-pyrazol-1-yl)piperidin-1-yl)ethanol (2.2 mg) as a white solid.
$^1$H-NMR(DMSO-$d_6$)δ:12.04(1H,s),9.09(1H,d,J=2.0Hz),8.66(1H,s),8.34(1H,d,J=2.0Hz),8.28-8.20(2H,m),7.39(1H,d,J=8.6Hz),4.43(1H,t,J=5.3Hz),4.27-4.11(1H,m),3.61-3.21(5H,m),3.07-2.93(2H,m),2.45(2H,t,J=6.3Hz),2.30-1.62(12H,m).
MSm/z(M+H):491.

<Example 0337>

<0337-1>

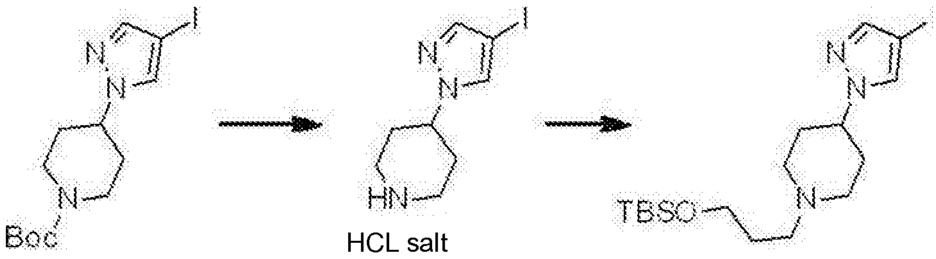


1-(3-((tert-Butyldimethylsilyl)oxy)propyl)-4-(4-iodo-1H-pyrazol-1-yl)piperidine was obtained as a white solid in the same manner as in Example 0336 except that (3-bromopropoxy) (tert-butyl)dimethylsilane was used instead of the 2-bromoethyl acetate used in Example 0336.
$^1$H-NMR($CDCl_3$)$\delta$:7.50(1H,s),7.47(1H,s),3.66(2H,t,J=6.3Hz),3.08-2.96(2H,m),2.50-2.39(2H,m),2.19-1.88(5H,m),1.78-1.64(2H,m),0.95-0.78(11H,m),0.05(6H,s).

<0337-2>

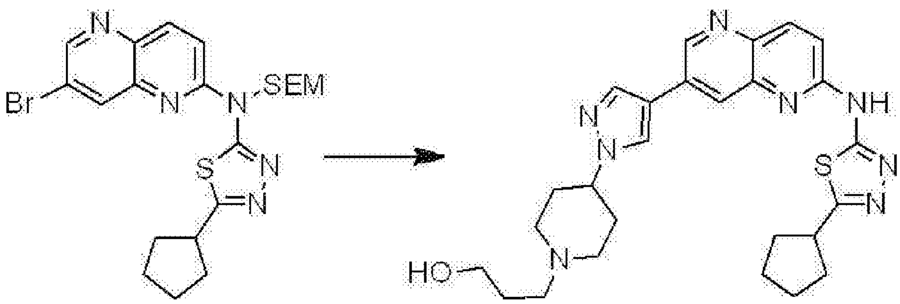

3-(4-(4-(6-((5-Cyclopentyl-1,3,4-thiadiazol-2-yl)amino)-1,5-naphthyridin-3-yl)-1H-pyrazol-1-yl)piperidin-1-yl)propan-1-ol was obtained as a white solid in the same manner as in Example 0337 except that 1-(3-((tert-butyldimethylsilyl)oxy)propyl)-4-(4-iodo-1H-pyrazol-1-yl)piperidine was used instead of the 2-(4-(4-iodo-1H-pyrazol-1-yl)piperidin-1-yl)ethyl acetate used in Example 0336.
$^1$H-NMR(DMSO-$d_6$)$\delta$:9.09-8.92(1H,m),8.68-8.59(1H,m),8.35-8.02(3H,m),7.41-7.19(1H,m),4.56-4.38(1H,m),4.29-4.10(1H,m),3.55-3.11(5H,m),3.07-2.89(2H,m),2.39(2H,t,J=7.3Hz),2.23-1.51(14H,m).
MSm/z(M+H):505.

<Example 0338>

<0338-1>

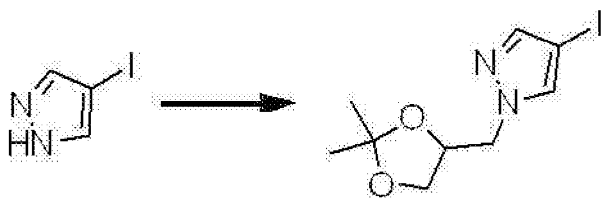

60% sodium hydride (74.4 mg) was added to a solution of 4-iodo-1H-pyrazole (300 mg) in N,N-dimethylformamide (2 mL) at room temperature, followed by stirring for 30 minutes. 4-(Chloromethyl)-2,2-dimethyl-1,3-dioxolane (0.439 mL) was added to the reaction mixture at room temperature, followed by stirring at room temperature for 1 hour, and stirring at 80°C for 3 hours. The reaction mixture was cooled to room temperature, and a saturated ammonium chloride aqueous solution and ethyl acetate were added thereto. The organic layer was collected by separation, and dried over anhydrous sodium sulfate, and the solvent was distilled off under reduced pressure. The obtained residue was purified by silica gel column chromatography (hexane-ethyl acetate), thereby obtaining 1-((2,2-dimethyl-1,3-dioxolan-4-yl)methyl)-4-iodo-1H-pyrazole (304 mg) as a white solid.
$^1$H-NMR($CDCl_3$)δ:7.54(1H,s),7.51(1H,s),4.48-4.37(1H,m),4.33-4.17(2H,m),4.07(1H,dd,J=8.6,6.6Hz),3.75(1H,dd,J=8.6,5.9Hz),1.38(3H,s),1.35(3H,s).

<0338-2>

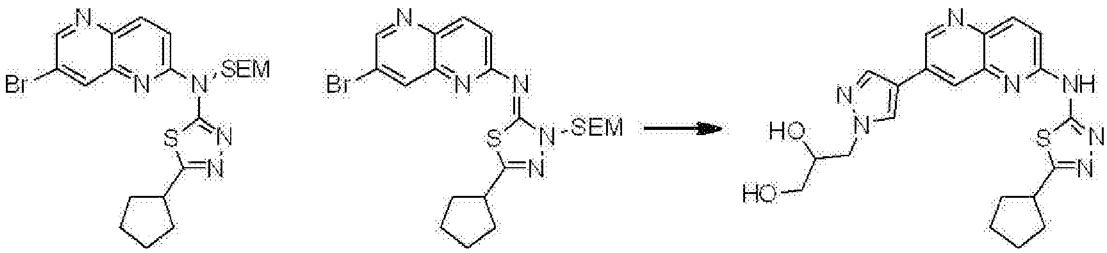

3-(4-(6-((5-Cyclopentyl-1,3,4-thiadiazol-2-yl)amino)-1,5-naphthyridin-3-yl)-1H-pyrazol-1-yl)propane-1,2-diol was obtained as a white solid in the same manner as in Example 0336 except that 1-((2,2-dimethyl-1,3-dioxolan-4-yl)methyl)-4-iodo-1H-pyrazole was used instead of the 2-(4-(4-iodo-1H-pyrazol-1-yl)piperidin-1-yl)ethyl acetate used in Example 0336.
$^1$H-NMR(DMSO-$d_6$)δ:12.03(1H,s),9.09(1H,d,J=2.0Hz),8.52(1H,s),8.34(1H,d,J=2.0Hz),8.28-8.20(2H,m),7.39(1H,d,J=9.2Hz),5.07(1H,d,J=5.3Hz),4.79(1H,t,J=5.6Hz),4.30(1H,dd,J=13.9,4.0Hz),4.05(1H,dd,J=13.9,7.9Hz),3.95-3.82(1H,m),3.57-3.21(3H,m),2.24-2.09(2H,m),1.97-1.62(6H,m).
MSm/z(M+H):438.

<Example 0339>

<0339-1>

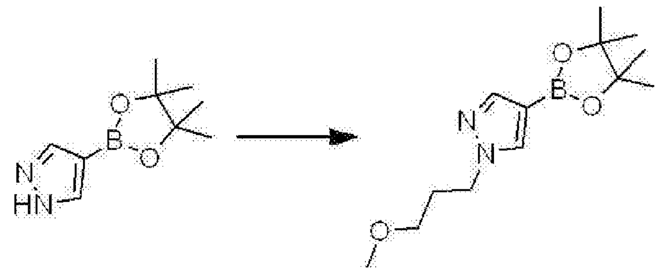

1-(3-Methoxypropyl)-4-(4,4,5,5-tetramethyl-1,3,2-dioxaborolan-2-yl)-1H-pyrazole was obtained as colorless oily substance in the same manner as in Example 0316 except that 1-bromo-3-methoxypropane was used instead of the 3-bromopropyl acetate used in Example 0316.
$^1$H-NMR($CDCl_3$)δ:7.84-7.76(1H,m),7.74-7.65(1H,m),4.30-4.19(2H,m),3.39-3.27(5H,m),2.20-2.06(2H,m),1.39-1.29(12H,m).

<0339-2>

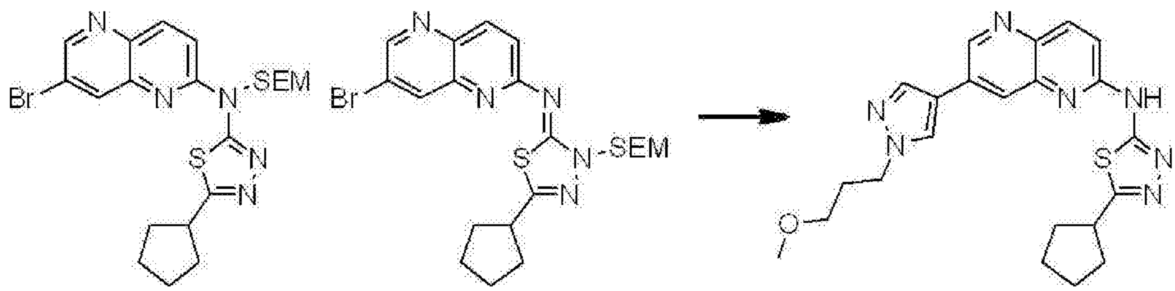

A mixture of a mixture (10 mg) of N-(7-bromo-1,5-naphthyridin-2-yl)-5-cyclopentyl-N-((2-(trimethylsilyl)ethoxy)methyl)-1,3,4-thiadiazole-2-amine and (Z)-7-bromo-N-(5-cyclopentyl-3-((2-(trimethylsilyl)ethoxy)methyl)-1,3,4-thiadiazol-2(3H)-ylidene)-1,5-naphthyridine-2-amine, 1-(3-methoxypropyl)-4-(4,4,5,5-tetramethyl-1,3,2-dioxaborolan-2-yl)-1H-pyrazole (7.9 mg), bis(di-tert-butyl (4-dimethylaminophenyl)phosphine)dichloropalladium(II) (1.4 mg), sodium carbonate (4.2 mg), 1,4-dioxane (1 mL), and water (0.1 mL) was stirred at 100°C for 12.5 hours in a nitrogen atmosphere in a sealed tube. The reaction mixture was cooled to room temperature, and methanol (1 mL) and a 4 mol/L hydrogen chloride/1,4-dioxane solution (1 mL) were added thereto, followed by stirring at room temperature for 2 hours. The solvent was distilled off under reduced pressure, triethylamine was added to the obtained residue, and the solvent was distilled off under reduced pressure. The obtained residue was purified by silica gel column chromatography (hexane-ethyl acetate), thereby obtaining 5-cyclopentyl-N-(7-(1-(3-methoxypropyl)-1H-pyrazol-4-yl)-1,5-naphthyridin-2-yl)-1,3,4-thiadiazole-2-amine (2.5 mg) as a white solid.
$^1$H-NMR(DMSO-$d_6$)δ:12.02(1H,brs),9.06(1H,s),8.57(1H,s),8.31(1H,s),8.27-8.15(2H,m),7.37(1H,d,J=8.6Hz),4.22(2H,t,J=6.9Hz), 3.57-3.12(6H,m),2.24-2.02(4H,m),1.96-1.62(6H,m).
MSm/z(M+H):436.

<Example 0340>

<0340-1>

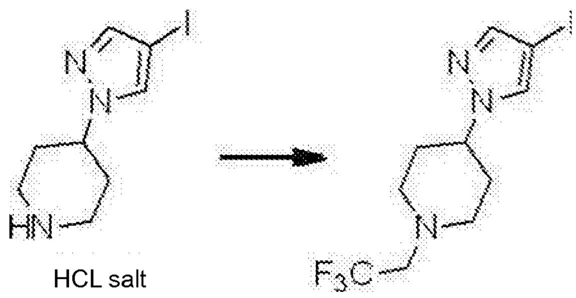


Triethylamine (123 μL) and 2,2,2-trifluoroethyl trifluoromethanesulfonate (102 mg) were added to a solution of 4-(4-iodo-1H-pyrazol-1-yl)piperidine hydrochloride (69 mg) in tetrahydrofuran (1 mL) at room temperature, followed by stirring at 80°C for 13.5 hours in a sealed tube. The reaction mixture was cooled to room temperature, and the solvent was distilled off under reduced pressure. The obtained residue was purified by silica gel column chromatography (hexane-ethyl acetate), thereby obtaining 4-(4-iodo-1H-pyrazol-1-yl)-1-(2,2,2-trifluoroethyl)piperidine (49.5 mg) as a white solid. $^1$H-NMR($CDCl_3$)δ:7.67-7.41(2H,m),4.32-4.05(1H,m),3.35-2.95(4H,m),2.82-2.49(2H,m),2.40-1.92(4H,m).

<0340-2>

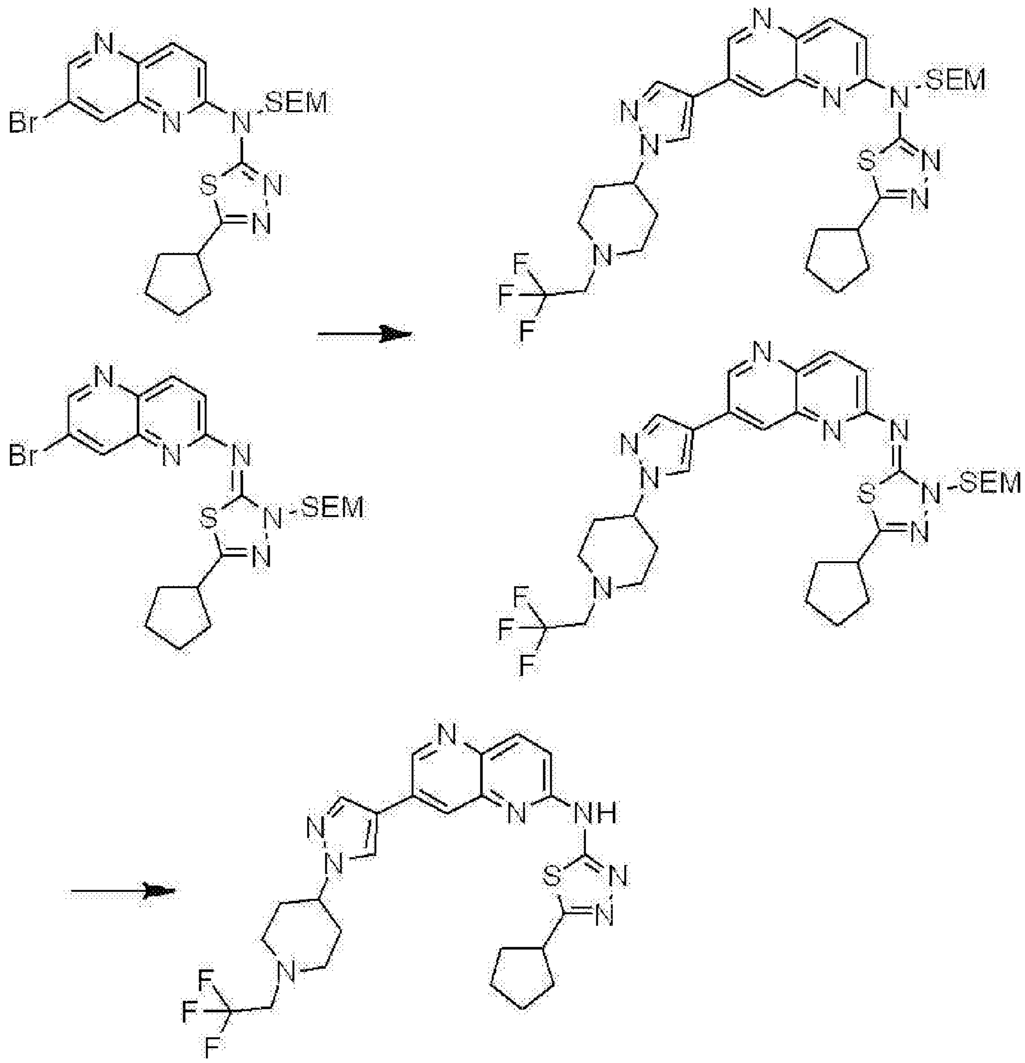

A mixture of a mixture (20 mg) of N-(7-bromo-1,5-naphthyridin-2-yl)-5-cyclopentyl-N-((2-(trimethylsilyl)ethoxy)methyl)-1,3,4-thiadiazole-2-amine and (Z)-7-bromo-N-(5-cyclopentyl-3-((2-(trimethylsilyl)ethoxy)methyl)-1,3,4-thiadiazol-2(3H)-ylidene)-1,5-naphthyridine-2-amine, bis(pinacolato)diboron (15.1 mg), (1,1'-bis(diphenylphosphino)ferrocene)palladium(II) dichloride (3.2 mg), potassium acetate (7.8 mg), and 1,4-dioxane (0.4 mL) was stirred at 80°C for 4 hours in a nitrogen atmosphere in a sealed tube. The reaction mixture was cooled to room temperature, and sodium carbonate (21.0 mg), bis(di-tert-butyl (4-dimethylaminophenyl)phosphine)dichloropalladium(II) (2.8 mg), 4-(4-iodo-1H-pyrazol-1-yl)-1-(2,2,2-trifluoroethyl)piperidine (17.0 mg), 1,4-dioxane (1 mL), and water (0.14 mL) were added thereto, followed by stirring at 120°C for 18 hours in a sealed tube. The reaction mixture was cooled to room temperature, and the solvent was distilled off under reduced pressure, thereby obtaining a mixture of 5-cyclopentyl-N-(7-(1-(1-(2,2,2-trifluoroethyl)piperidin-4-yl)-1H-pyrazol-4-yl)-1,5-naphthyridin-2-yl)-N-((2-(trimethylsilyl)ethoxy)methyl)-1,3,4-thiadiazole-2-amine and (Z)-N-(5-cyclopentyl-3-((2-(trimethylsilyl)ethoxy)methyl)-1,3,4-thiadiazol-2(3H)-ylidene)-7-(1-(1-(2,2,2-trifluoroethyl)piperidin-4-yl)-1H-pyrazol-4-yl)-1,5-naphthyridine-2-amine.

Methanol (1 mL) and a 4 mol/L hydrogen chloride/1,4-dioxane solution (1 mL) were added to the obtained mixture at room temperature, followed by stirring for 1 hour. The solvent was distilled off under reduced pressure, triethylamine was added to the obtained residue, and the solvent was distilled off under reduced pressure. The obtained residue was purified by silica gel column chromatography (chloroform-methanol, NH silica), thereby obtaining 5-cyclopentyl-N-(7-(1-(1-(2,2,2-trifluoroethyl)piperidin-4-yl)-1H-pyrazol-4-yl)-1,5-naphthyridin-2-yl)-1,3,4-thiadiazole-2-amine (5.6 mg) as a white solid.

$^1$H-NMR(DMSO-$d_6$)$\delta$:12.04(1H,brs),9.09(1H,d,J=2.0Hz),8.69(1H,s),8.35(1H,d,J=2.0Hz),8.28-8.20(2H,m),7.39(1H,d,J=8.6Hz),4.32-4.16(1H,m),3.57-3.18(3H,m),3.11-2.98(2H,m),2.67-2.45(2H,m),2.23-1.62(12H,m).

MSm/z(M+H):529.

<Example 0341>

<0341-1>

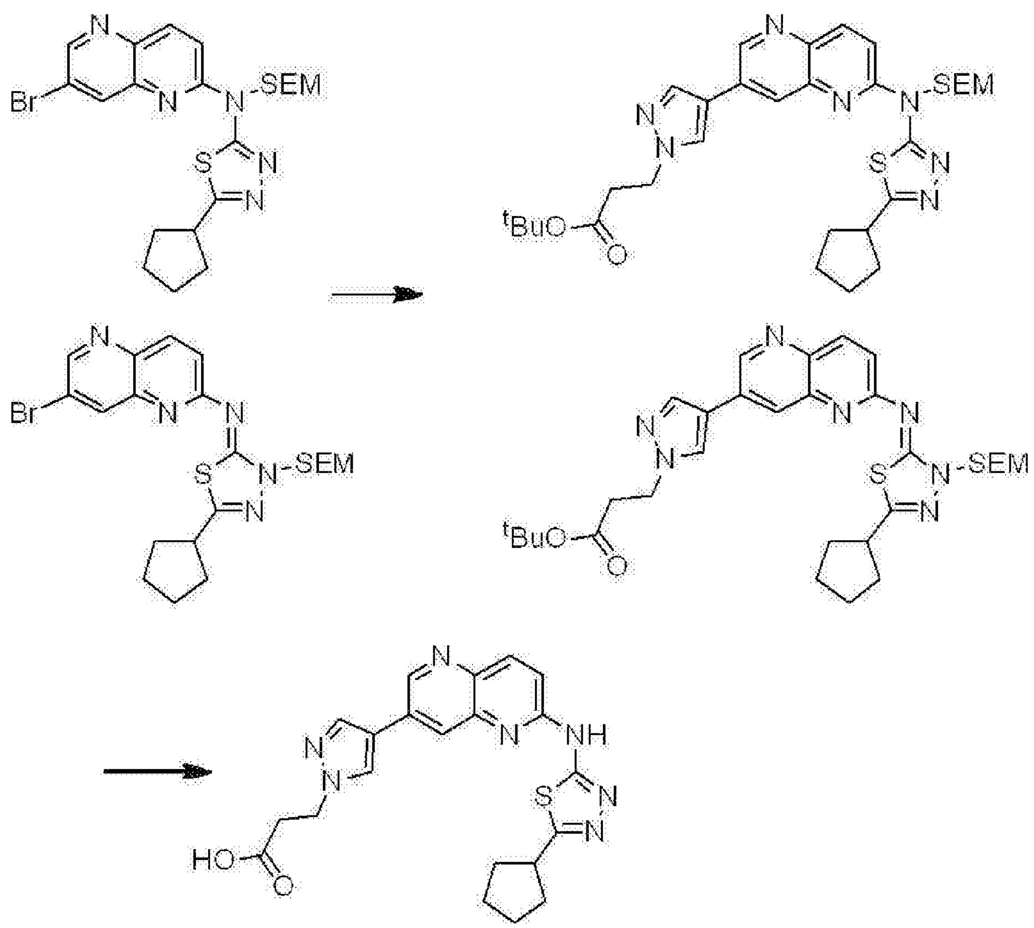

A mixture of a mixture (30 mg) of N-(7-bromo-1,5-naphthyridin-2-yl)-5-cyclopentyl-N-((2-(trimethylsilyl)ethoxy)methyl)-1,3,4-thiadiazole-2-amine and (Z)-7-bromo-N-(5-cyclopentyl-3-((2-(trimethylsilyl)ethoxy)methyl)-1,3,4-thiadiazol-2(3H)-ylidene)-1,5-naphthyridine-2-amine, tert-butyl 3-(4-(4,4,5,5-tetramethyl-1,3,2-dioxaborolan-2-yl)-1H-pyrazol-1-yl)propanoate (23.2 mg), bis(di-tert-butyl (4-dimethylaminophenyl)phosphine)dichloropalladium(II) (3.4 mg), sodium carbonate (10.2 mg), 1,4-dioxane (1 mL), and water (0.1 mL) was stirred at 120°C for 18 hours in a nitrogen atmosphere in a sealed tube. The reaction mixture was cooled to room temperature, and the solvent was distilled off under reduced pressure, thereby obtaining a mixture of tert-butyl 3-(4-(6-((5-cyclopentyl-1,3,4-thiadiazol-2-yl)((2-(trimethylsilyl)ethoxy)methyl)amino)-1,5-naphthyridin-3-yl)-1H-pyrazol-1-yl)propanoate and (Z)-tert-butyl 3-(4-(6-((5-cyclopentyl-3-((2-(trimethylsilyl)ethoxy)methyl)-1,3,4-thiadiazol-2(3H)-ylidene)amino)-1,5-naphthyridin-3-yl)-1H-pyrazol-1-yl)propanoate.

Methanol (1 mL) and a 4 mol/L hydrogen chloride/1,4-dioxane solution (1 mL) were added to the obtained mixture at room temperature, followed by stirring for 1 hour. The solvent was distilled off under reduced pressure, ethanol was added to the obtained residue, and the resultant product was neutralized with a saturated sodium hydrogen carbonate aqueous solution. The solvent was distilled off under reduced pressure, thereby obtaining 3-(4-(6-((5-cyclopentyl-1,3,4-thiadiazol-2-yl)amino)-1,5-naphthyridin-3-yl)-1H-pyrazol-1-yl)propionic acid (48.3 mg) as a yellow solid.
MSm/z(M+H):436.

<0341-2>

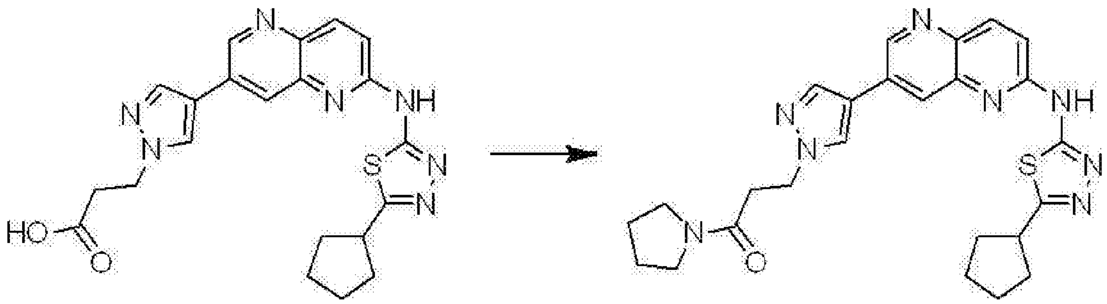

1,1'-Carbonyldiimidazole (29.8 mg) was added to a solution of 3-(4-(6-((5-cyclopentyl-1,3,4-thiadiazol-2-yl)amino)-1,5-naphthyridin-3-yl)-1H-pyrazol-1-yl)propionic acid (20 mg) in N,N-dimethylformamide (0.5 mL) at room temperature, followed by stirring for 1 hour. Pyrrolidine (38 μL) was added to the reaction mixture at room temperature, followed by stirring for 30 minutes, and after methanol was added thereto, the solvent was distilled off under reduced pressure. The obtained residue was purified by preparative thin layer silica gel chromatography (chloroform-methanol, NH silica), thereby obtaining 3-(4-(6-((5-cyclopentyl-1,3,4-thiadiazol-2-yl)amino)-1,5-naphthyridin-3-yl)-1H-pyrazol-1-yl)-1-(pyrrolidin-1-yl)propan-1-one (3.7 mg) as a white solid.
$^1$H-NMR(DMSO-$d_6$)δ:12.03(1H,brs),9.06(1H,d,J=2.0Hz),8.56(1H,s),8.31(1H,d,J=2.0Hz),8.27-8.19(2H,m),7.38(1H,d,J=9.2Hz),4.41(2H,t,J=6.9Hz),3.58-3.23(5H,m),2.89(2H,t,J=6.9Hz),2.23-2.08(2H,m),1.96-1.62(10H,m).
MSm/z(M+H):489.

<Example 0342>

<0342-1>

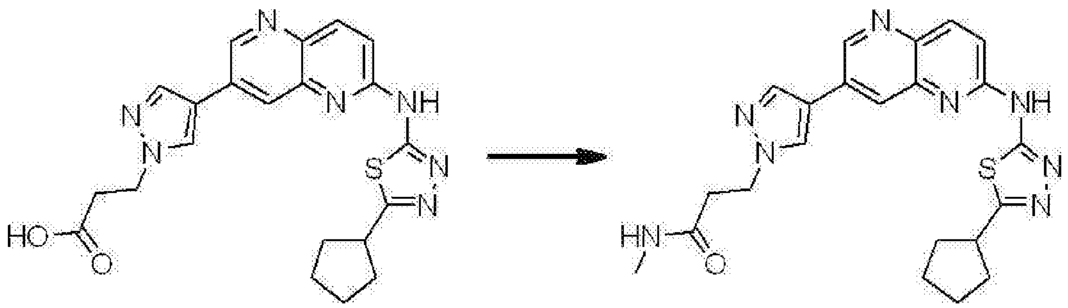

3-(4-(6-((5-cyclopentyl-1,3,4-thiadiazol-2-yl)amino)-1,5-naphthyridin-3-yl)-1H-pyrazol-1-yl)-N-methylpropanamide was obtained as a white solid in the same manner as in Example 0341 except that a 2 mol/L methylamine/tetrahydrofuran solution was used instead of the pyrrolidine used in Example 0341.

$^1$H-NMR(DMSO-$d_6$)$\delta$:12.03(1H,brs),9.06(1H,d,J=2.6Hz),8.52(1H,s),8.32(1H,d,J=2.0Hz),8.27-8.21(2H,m),7.91(1H,brd,J=4.6Hz),7.39(1H,d,J=9.2Hz),4.39(2H,t,J=6.9Hz),3.58-3.27(1H,m),2.71(2H,t,J=6.9Hz),2.57(3H,d,J=4.6Hz),2.24-2.09(2H,m),1.96-1.62(6H,m).

MSm/z(M+H):449.

<Example 0343>

<0343-1>

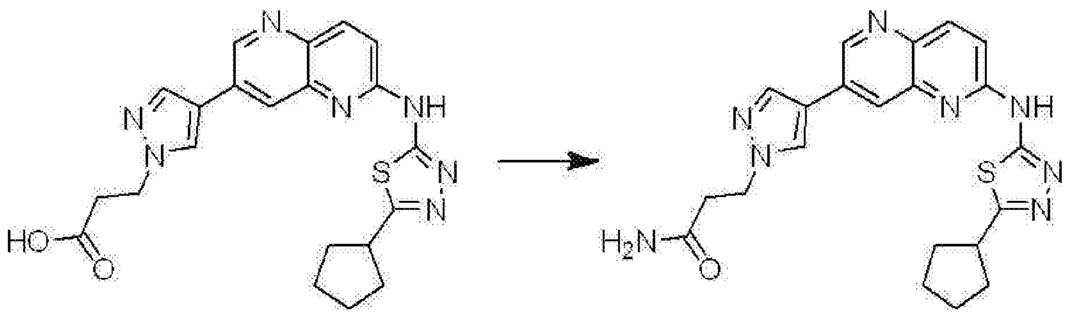

3-(4-(6-((5-cyclopentyl-1,3,4-thiadiazol-2-yl)amino)-1,5-naphthyridin-3-yl)-1H-pyrazol-1-yl)-N-propanamide was obtained as a white solid in the same manner as in Example 0341 except that a 0.5 mol/L ammonia/1,4-dioxane solution was used instead of the pyrrolidine used in Example 0341, and the stirring time of 30 minutes was changed to a stirring time of 56 hours.

$^1$H-NMR(DMSO-$d_6$)$\delta$:9.02(1H,brs),8.51(1H,s),8.28(1H,brs),8.25-8.13(2H,m),7.44(1H,brs),7.34(1H,brd,J=9.2Hz),7.00-6.86(1H,m),4.37(2H,t,J=6.9Hz),3.60-3.18(1H,m),2.71(2H,t,J=6.9Hz),2.24-2.06(2H,m),1.95-1.62(6H,m).

MSm/z(M+H):435.

<Example 0344>

<0344-1>

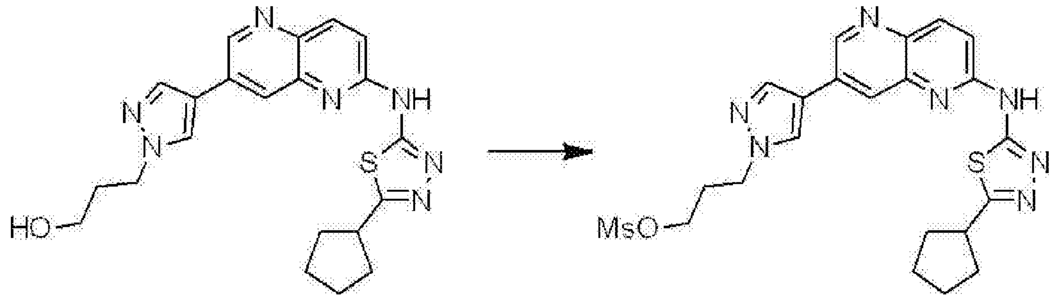

Methanesulfonyl chloride (110 μL) was added to a solution of 3-(4-(6-((5-cyclopentyl-1,3,4-thiadiazol-2-yl)amino)-1,5-naphthyridin-3-yl)-1H-pyrazol-1-yl)propan-1-ol (200 mg) in pyridine (2.4 mL) in an ice bath, followed by stirring at room temperature for 1.5 hours. A saturated sodium hydrogen carbonate aqueous solution was added to the reaction mixture, followed by stirring at room temperature for 30 minutes. The solvent was distilled off under reduced pressure, and the obtained residue was purified by silica gel column chromatography (chloroform-methanol), thereby obtaining 3-(4-(6-((5-cyclopentyl-1,3,4-thiadiazol-2-yl)amino)-1,5-naphthyridin-3-yl)-1H-pyrazol-1-yl)propyl methanesulfonate (173 mg) as a white solid.
MSm/z(M+H):500.

<0344-2>

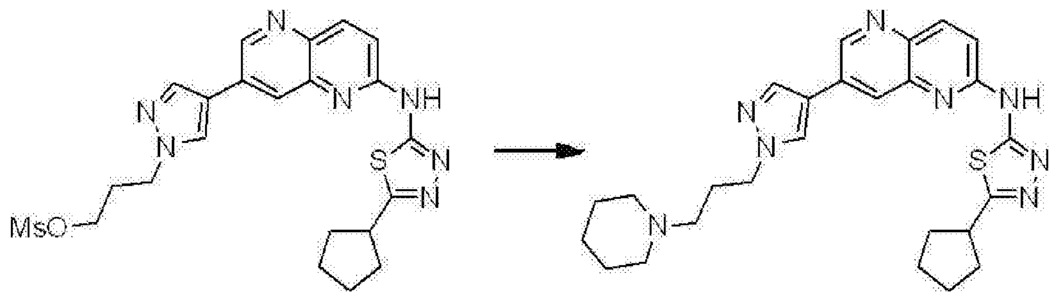

Piperidine (15.8 μL) was added to a mixture of 3-(4-(6-((5-cyclopentyl-1,3,4-thiadiazol-2-yl)amino)-1,5-naphthyridin-3-yl)-1H-pyrazol-1-yl)propyl methanesulfonate (30 mg), potassium carbonate (22.1 mg), and N,N-dimethylformamide (0.5 mL) at room temperature, followed by stirring at 80°C for 18 hours. The reaction mixture was cooled to room temperature, and the solvent was distilled off under reduced pressure. The obtained residue was purified by silica gel column chromatography (chloroform-methanol, NH silica), and purified by preparative thin layer silica gel chromatography (chloroform-methanol, NH silica), thereby obtaining 5-cyclopentyl-N-(7-(1-(3-(piperidin-1-yl)propyl)-1H-pyrazol-4-yl)-1,5-naphthyridin-2-yl)-1,3,4-thiadiazole-2-amine (5.3 mg) as a white solid.
$^1$H-NMR(DMSO-$d_6$)δ:9.07(1H,d,J=2.0Hz),8.56(1H,s),8.31(1H,d,J=2.0Hz),8.28-8.20(2H,m),7.39(1H,d,J=9.2Hz),4.19(2H,t,J=7.3Hz),3.58-3.25(1H,m),2.39-2.08(8H,m),2.07-1.63(8H,m), 1.59-1.32(6H,m).
MSm/z(M+H):489.

<Example 0345>

<0345-1>

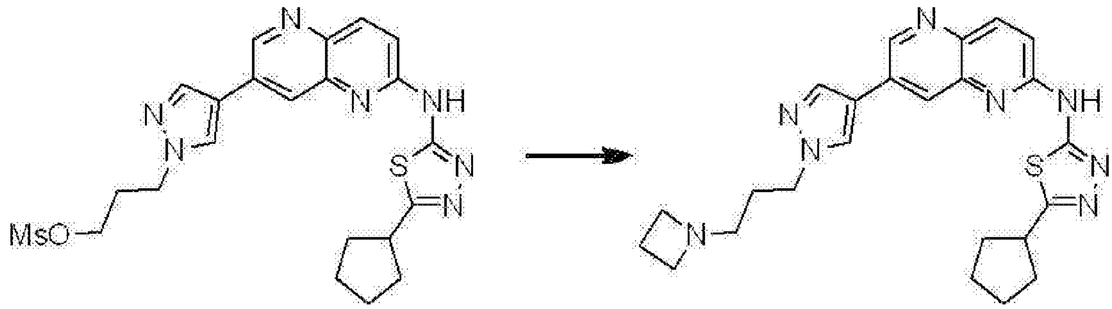

N-(7-(1-(3-(azetidin-1-yl)propyl)-1H-pyrazol-4-yl)-1,5-naphthyridin-2-yl)-5-cyclopentyl-1,3,4-thiadiazole-2-amine was obtained as a white solid in the same manner as in Example 0344 except that azetidine was used instead of the piperidine used in Example 0344.
$^1$H-NMR(DMSO-$d_6$)$\delta$:9.08(1H,d,J=2.0Hz),8.56(1H,s),8.33(1H,d,J=2.0Hz),8.28-8.20(2H,m),7.39(1H,d,J=9.2Hz),4.17(2H,t,J=7.3Hz),3.60-3.22(1H,m),3.08(4H,t,J=6.9Hz),2.33(2H,t,J=6.6Hz),2.24-2.09(2H,m),2.01-1.63(10H,m).
MSm/z(M+H):461.

<Example 0346>

<0346-1>

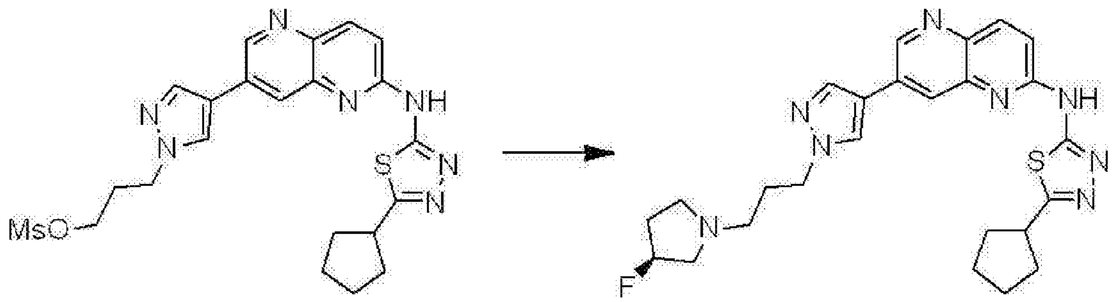

(S)-5-cyclopentyl-N-(7-(1-(3-(3-fluoropyrrolidin-1-yl)propyl)-1H-pyrazol-4-yl)-1,5-naphthyridin-2-yl)-1,3,4-thiadiazole-2-amine was obtained as a white solid in the same manner as in Example 0344 except that (S)-3-fluoropyrrolidine was used instead of the piperidine used in Example 0344.
$^1$H-NMR(DMSO-$d_6$)$\delta$:12.02(1H,brs),9.08(1H,d,J=2.0Hz),8.57(1H,s),8.32(1H,d,J=2.0Hz),8.29-8.19(2H,m),7.39(1H,d,J=8.6Hz),5.33-5.05(1H,m),4.22(2H,t,J=7.3Hz),3.62-3.27(1H,m),2.91-2.47(4H,m),2.43(2H,t,J=6.6Hz),2.36-1.63(12H,m).
MSm/z(M+H):493.

<Example 0347>

<0347-1>

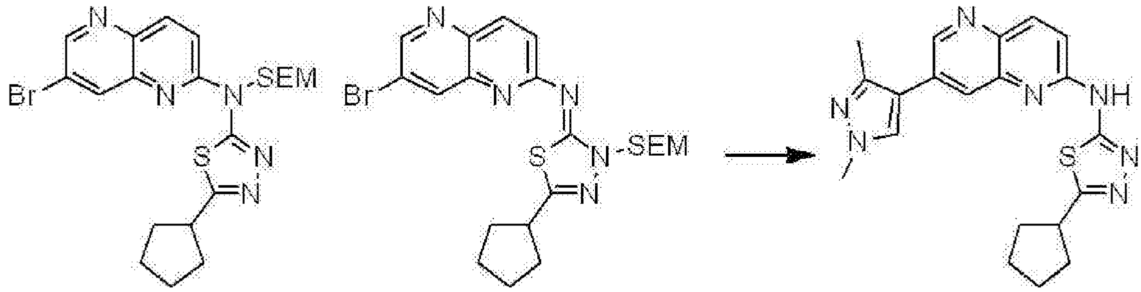

5-Cyclopentyl-N-(7-(1,3-dimethyl-1H-pyrazol-4-yl)-1,5-naphthyridin-2-yl)-1,3,4-thiadiazole-2-amine was obtained as a white solid in the same manner as in Example 0336 except that 4-bromo-1,3-dimethyl-1H-pyrazole was used instead of the 2-(4-(4-iodo-1H-pyrazol-1-yl)piperidin-1-yl)ethyl acetate used in Example 0336.
$^1$H-NMR(DMSO-$d_6$)$\delta$:12.05(1H,brs),8.91(1H,d,J=2.0Hz),8.30-8.24(2H,m),8.15(1H,d,J=2.0Hz),7.42(H,d,J=8.6Hz),3.85(3H,s),3.55-3.27(1H,m),2.43(3H,s),2.23-2.07(2H,m),1.95-1.62(6H,m).
MSm/z(M+H):392.

<Example 0348>

<0348-1>

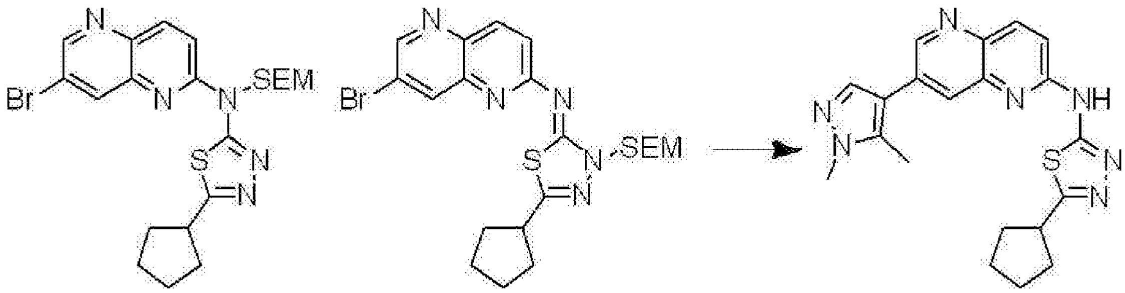

5-Cyclopentyl-N-(7-(1,5-dimethyl-1H-pyrazol-4-yl)-1,5-naphthyridin-2-yl)-1,3,4-thiadiazole-2-amine was obtained as a white solid in the same manner as in Example 0336 except that 4-bromo-1,5-dimethyl-1H-pyrazole was used instead of the 2-(4-(4-iodo-1H-pyrazol-1-yl)piperidin-1-yl)ethyl acetate used in Example 0336.
$^1$H-NMR(DMSO-$d_6$)$\delta$:12.05(1H,brs),8.87(1H,d,J=2.0Hz),8.28(1H,d,J=8.6Hz),8.13(1H,d,J=2.0Hz),7.89(1H,s),7.43(1H,d ,J=9.2Hz),3.85(3H,s),3.59-3.24(4H,m),2.23-2.07(2H,m),1.94-1.61(6H,m).
MSm/z(M+H):392.

<Examples 0349 and 0350>

<0349-1> and <0350-1>

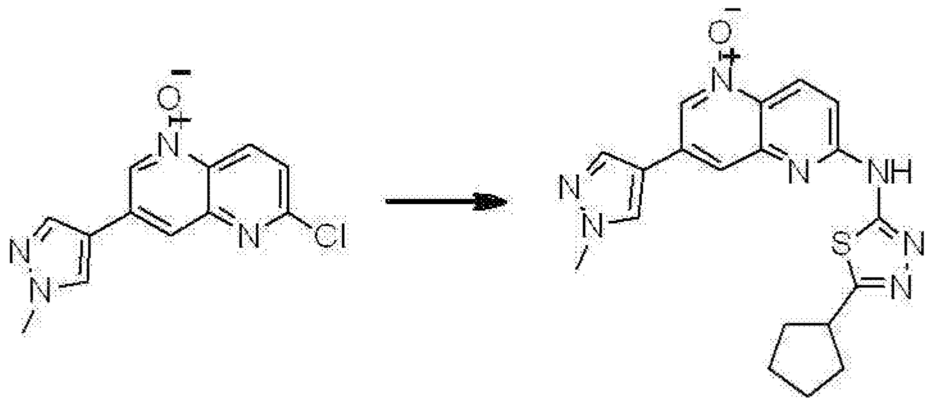

A mixture of 6-chloro-3-(1-methyl-1H-pyrazol-4-yl)-1,5-naphthyridine 1-oxide (230 mg), 5-cyclopentyl-1,3,4-thiadiazole-2-amine (223 mg), tris(dibenzylideneacetone)dipalladium(0) (80.8 mg), 2-dicyclohexylphosphino-2',4',6'-triisopropylbiphenyl (102 mg), cesium carbonate (573 mg), and 1,4-dioxane (3 mL) was stirred at 120°C for 17 hours in a nitrogen atmosphere in a sealed tube. The reaction mixture was cooled to room temperature, and water was added thereto. The solid matter was collected by filtration, and washed with water and ethyl acetate, thereby obtaining 6-((5-cyclopentyl-1,3,4-thiadiazol-2-yl)amino)-3-(1-methyl-1H-pyrazol-4-yl)-1,5-naphthyridine 1-oxide (415 mg) as a black solid.
MSm/z(M+H):394.

<0349-2> and <0350-2>

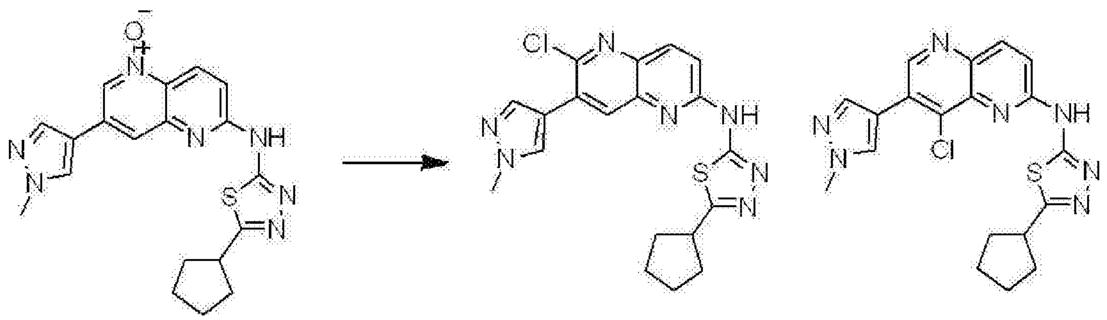

Phosphorus oxychloride (1 mL) was added to 6-((5-cyclopentyl-1,3,4-thiadiazol-2-yl)amino)-3-(1-methyl-1H-pyrazol-4-yl)-1,5-naphthyridine 1-oxide (415 mg), followed by stirring at 80°C for 2 hours. The reaction mixture was cooled to room temperature, and added dropwise to water in an ice bath, followed by stirring at room temperature for 30 minutes. The resultant product was neutralized by the addition of sodium carbonate. The solid matter was collected by filtration, and washed with water and ethyl acetate, thereby obtaining a yellow solid (260 mg).
The obtained yellow solid (30 mg) was purified by preparative thin layer silica gel chromatography (chloroform-methanol), thereby obtaining N-(6-chloro-7-(1-methyl-1H-pyrazol-4-yl)-1,5-naphthyridin-2-yl)-5-cyclopentyl-1,3,4-thiadiazole-2-amine (9.7 mg) as a white solid and N-(8-chloro-7-(1-methyl-1H-pyrazol-4-yl)-1,5-naphthyridin-2-yl)-5-cyclopentyl-1,3,4-thiadiazole-2-amine (3.5 mg) as a white solid.

<Example 0349>

N-(6-chloro-7-(1-methyl-1H-pyrazol-4-yl)-1,5-naphthyridin-2-yl)-5-cyclopentyl-1,3,4-thiadiazole-2-amine
$^1$H-NMR(DMSO-$d_6$)$\delta$:12.14(1H,brs),8.42(1H,s),8.37(1H,s),8.23(1H,d,J=8.9Hz),8.09(1H,s),7.47(1H,d,J=8.9Hz),3.95(3H ,s),3.53-3.31(1H,m),2.24-2.04(2H,m),1.95-1.58(6H,m).
MSm/z(M+H):412.

<Example 0350>

N-(8-chloro-7-(1-methyl-1H-pyrazol-4-yl)-1,5-naphthyridin-2-yl)-5-cyclopentyl-1,3,4-thiadiazole-2-amine $^1$H-NMR(DMSO-$d_6$)δ:12.30(1H,brs),9.02(1H,s),8.58(1H,s),8.34(1H,d,J=8.9Hz),8.21(1H,s),7.50(1H,d,J=8.9Hz),3.97(3H ,s),3.63-3.24(1H,m),2.24-2.07(2H,m),1.94-1.62(6H,m).
MSm/z(M+H):412.

<Example 0351>

<0351-1>

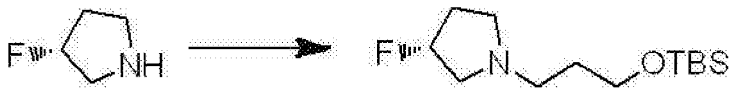

60% sodium hydride (96 mg) was added to a solution of (R)-3-fluoropyrrolidine hydrochloride (126 mg) in tetrahydrofuran (2 mL) in an ice bath, followed by stirring at room temperature for 30 minutes. (3-Bromopropoxy) (tert-butyl)dimethylsilane (348 μL) was added to the reaction mixture in an ice bath, followed by stirring at room temperature for 62 hours. A saturated sodium hydrogen carbonate aqueous solution and ethyl acetate were added to the reaction mixture. The organic layer was collected by separation, and dried over anhydrous sodium sulfate, and the solvent was distilled off under reduced pressure. The obtained residue was purified by silica gel column chromatography (chloroform-methanol), thereby obtaining (R)-1-(3-((tert-butyldimethylsilyl)oxy)propyl)-3-fluoropyrrolidine (322 mg) as colorless oily substance. $^1$H-NMR($CDCl_3$)δ:5.29-5.03(1H,m),3.67(2H,t,J=6.3Hz),2.94-2.61(3H,m),2.58-2.50(2H,m),2.46-2.36(1H,m),2.26-1.92(2H,m),1.79-1.68(2H,m),0.89(9H,s),0.05(6H,s).

<0351-2>

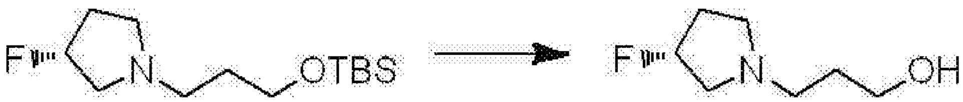

A4 mol/L hydrogen chloride/1,4-dioxane solution (1 mL) was added to a solution of (R)-1-(3-((tert-butyldimethylsilyl)oxy)propyl)-3-fluoropyrrolidine (0.322 g) in methanol (1 mL) at room temperature, followed by stirring for 30 minutes. The solvent was distilled off under reduced pressure, methanol (1 mL) and a saturated sodium hydrogen carbonate aqueous solution (1 mL) were added to the obtained residue, and the solvent was distilled off under reduced pressure. The obtained residue was purified by silica gel column chromatography (chloroform-methanol), thereby obtaining (R)-3-(3-fluoropyrrolidin-1-yl)propan-1-ol (0.101 mg) as colorless oily substance.
$^1$H-NMR($CDCl_3$)δ:5.29-5.02(1H,m),3.81(2H,t,J=5.3Hz),3.02-2.69(5H,m),2.58-2.47(1H,m),2.23-1.93(2H,m),1.79-1.68(2H,m).

<0351-3>

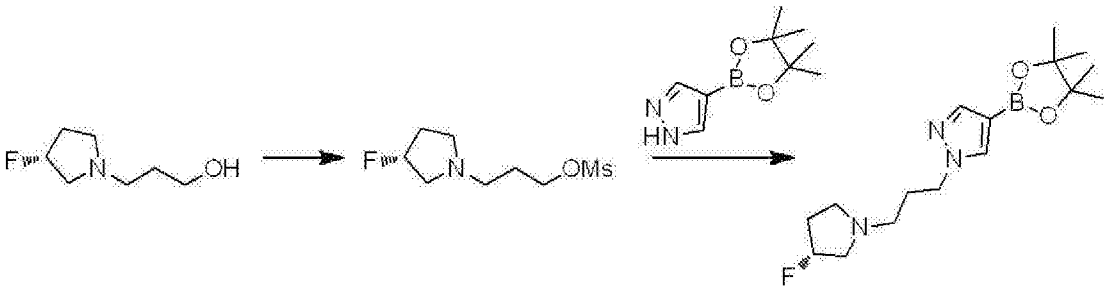

Pyridine (136 μL) and methanesulfonyl chloride (65.2 μL) were added to a solution of (R)-3-(3-fluoropyrrolidin-1-yl)propan-1-ol (80.1 mg) in dichloromethane (2 mL) in an ice bath, followed by stirring for 30 minutes. A saturated sodium hydrogen carbonate aquesou solution was added to the reaction mixture, followed by stirring at room temperature for 30 minutes, and ethyl acetate was added thereto. The organic layer was collected by separation, and dried over anhydrous sodium sulfate, and the solvent was distilled off under reduced pressure. The obtained residue was purified by silica gel column chromatography (ethyl acetate-methanol, NH silica), thereby obtaining (R)-3-(3-fluoropyrrolidin-1-yl)propyl methanesulfonate (106 mg) as yellow oily substance.

A mixture of 4-(4,4,5,5-tetramethyl-1,3,2-dioxaborolan-2-yl)-1H-pyrazole (60.9 mg), (R)-3-(3-fluoropyrrolidin-1-yl)propyl methanesulfonate (106 mg), potassium carbonate (86.8 mg), and acetonitrile (1 mL) was stirred at the external temperature of 80°C for 14 hours in a nitrogen atmosphere. The reaction mixture was cooled to room temperature, the solid matter was filtered off, and the solvent was distilled off under reduced pressure. The obtained residue was purified by silica gel column chromatography (chloroform-methanol), thereby obtaining (R)-1-(3-(3-fluoropyrrolidin-1-yl)propyl)-4-(4,4,5,5-tetramethyl-1,3,2-dioxaborolan-2-yl)-1H-pyrazole (58.1 mg) as yellow oily substance.

$^1$H-NMR($CDCl_3$)δ:7.78(1H,s),7.69(1H,s),5.28-5.02(1H,m),4.21(2H,t,J=6.9Hz),2.93-2.56(3H,m),2.50-2.31(3H,m),2.25-1.89(4H,m),1.32(12H,s).

<0351-4>

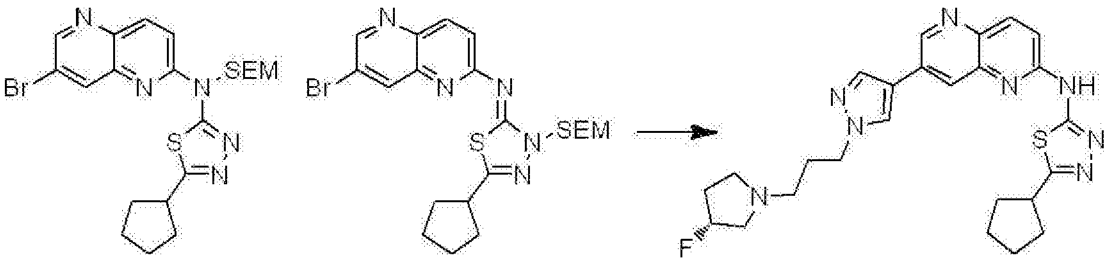

(R)-5-cyclopentyl-N-(7-(1-(3-(3-fluoropyrrolidin-1-yl)propyl)-1H-pyrazol-4-yl)-1,5-naphthyridin-2-yl)-1,3,4-thiadiazole-2-amine was obtained as a white solid in the same manner as in Example 0339 except that (R)-1-(3-(3-fluoropyrrolidin-1-yl)propyl)-4-(4,4,5,5-tetramethyl-1,3,2-dioxaborolan-2-yl)-1H-pyrazole was used instead of the 1-(3-methoxypropyl)-4-(4,4,5,5-tetramethyl-1,3,2-dioxaborolan-2-yl)-1H-pyrazole used in Example 0339.

$^1$H-NMR(DMSO-$d_6$)δ:12.05(1H,brs),9.08(1H,d,J=2.0Hz),8.58(1H,s),8.33(1H,d,J=2.0Hz),8.28-8.21(2H,m),7.39(1H,d,J=9.2Hz),5.34-5.05(1H,m),4.22(2H,t,J=6.9Hz),3.57-3.40(1H,m),2.94-2.38(6H,m),2.35-1.61(12H,m).

MSm/z(M+H):493.

<Example 0352>

<0352-1>

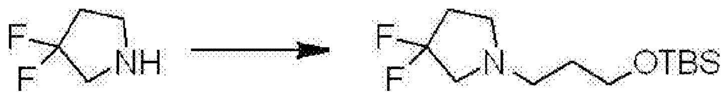

1-(3-((tert-Butyldimethylsilyl)oxy)propyl)-3,3-difluoropyrrolidine was obtained as colorless oily substance in the same manner as in Example 0351 except that 3,3-difluoropyrrolidine hydrochloride was used instead of the (R)-3-fluoropyrrolidine hydrochloride used in Example 0351.
$^1$H-NMR($CDCl_3$)δ:3.66(2H,t,J=6.3Hz),2.88(2H,t,J=13.2Hz),2.71(2H,t,J=7.3Hz),2.52(2H,t,J=7.3Hz),2.35-2.17(2H,m),1.75-1.63(2H,m),0.89(9H,s),0.05(6H,s).

<0352-2>

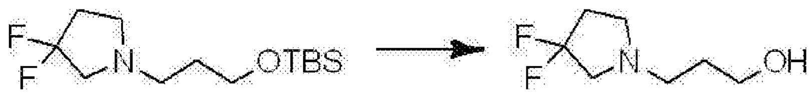

3-(3,3-Difluoropyrrolidin-1-yl)propan-1-ol was obtained as colorless oily substance in the same manner as in Example 0351 except that 1-(3-((tert-butyldimethylsilyl)oxy)propyl)-3,3-difluoropyrrolidine was used instead of the (R)-1-(3-((tert-butyldimethylsilyl)oxy)propyl)-3-fluoropyrrolidine used in Example 0351.
$^1$H-NMR($CDCl_3$)δ:3.96(1H,s),3.79(2H,t,J=5.6Hz),2.96(2H,t,J=12.9Hz),2.80(2H,t,J=6.4Hz),2.72(2H,t,J=6 .4Hz),2.37-2.18(2H,m),1.79-1.66(2H,m).

<0352-3>

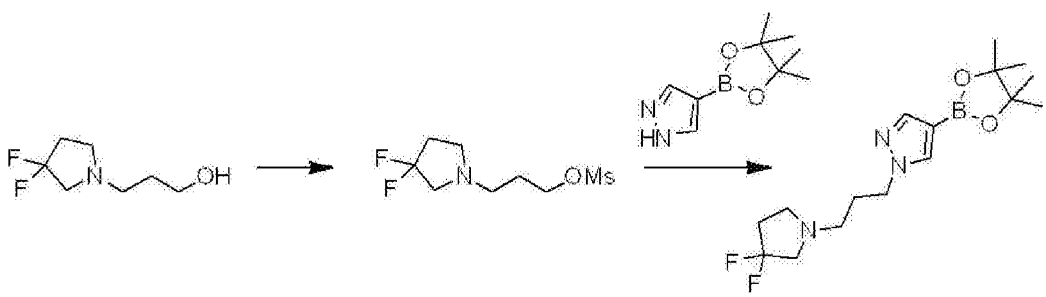

1-(3-(3,3-Difluoropyrrolidin-1-yl)propyl)-4-(4,4,5,5-tetramethyl-1,3,2-dioxaborolan-2-yl)-1H-pyrazole was obtained as yellow oily substance in the same manner as in Example 0351 except that 3-(3,3-difluoropyrrolidin-1-yl)propan-1-ol was used instead of the (R)-3-(3- fluoropyrrolidin-1-yl)propan-1-ol used in Example 0351.
$^1$H-NMR($CDCl_3$)δ:7.78(1H,s),7.68(1H,s),4.20(2H,t,J=6.6Hz),2.85(2H,t,J=11.6Hz),2.68(2H,t,J=6.9Hz),2.4 1(2H,t,J=6.9Hz),2.35-2.17(2H,m),2.08-1.96(2H,m),1.32(12H,s).

<0352-4>

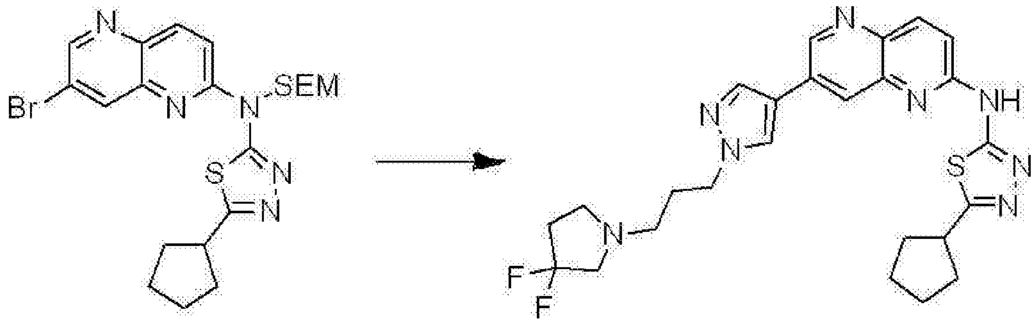

5-Cyclopentyl-N-(7-(1-(3-(3,3-difluoropyrrolidin-1-yl)propyl)-1H-pyrazol-4-yl)-1,5-naphthyridin-2-yl)-1,3,4-thiadiazole-2-amine (6.6 mg) was obtained as a white solid in the same manner as in Example 0339 except that 1-(3-(3,3-difluoropyrrolidin-1-yl)propyl)-4-(4,4,5,5-tetramethyl-1,3,2-dioxaborolan-2-yl)-1H-pyrazole was used instead of the 1-(3-methoxypropyl)-4-(4,4,5,5-tetramethyl-1,3,2-dioxaborolan-2-yl)-1H-pyrazole used in Example 0339.
$^1$H-NMR(DMSO-$d_6$)$\delta$:12.04(1H,s),9.08(1H,d,J=2.0Hz),8.58(1H,s),8.32(1H,d,J=2.0Hz),8.28-8.22(2H,m),7.39(1H,d,J=9.2Hz),4.22(2H,t,J=6.9Hz),3.55-3.41(1H,m),2.89(2H,t,J=13.2Hz),2.69(2H,t,J=7.1Hz),2.45(2H,t,J=7.1Hz),2.34-2.10(4H,m),2.07-1.63(8H,m).
MSm/z(M+H):511.

<Example 0353>

<0353-1>

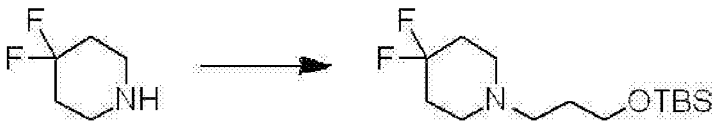

1-(3-((tert-Butyldimethylsilyl)oxy)propyl)-4,4-difluoropiperidine (179 mg) was obtained as colorless oily substance in the same manner as in Example 0351 except that 4,4-difluoropiperidine hydrochloride was used instead of the (R)-3-fluoropyrrolidine hydrochloride used in Example 0351.
$^1$H-NMR($CDCl_3$)$\delta$:3.65(2H,t,J=6.3Hz),2.59-2.42(6H,m),2.07-1.91(4H,m),1.76-1.63(2H,m),0.89(9H,s),0.05(6H,s).

<0353-2>

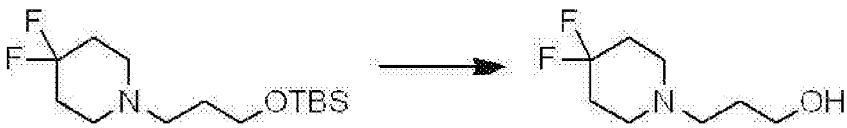

3-(4,4-Difluoropiperidin-1-yl)propan-1-ol was obtained as colorless oily substance in the same manner as in Example 0351 except that 1-(3-((tert-butyldimethylsilyl)oxy)propyl)-4,4-difluoropiperidine was used instead of the (R)-1-(3-((tert-butyldimethylsilyl)oxy)propyl)-3-fluoropyrrolidine used in Example 0351.
$^1$H-NMR($CDCl_3$)$\delta$:4.77(1H,brs),3.81(2H,t,J=5.3Hz),2.70-2.56(6H,m),2.09-1.91(4H,m),1.79-1.68(2H,m).

<0353-3>

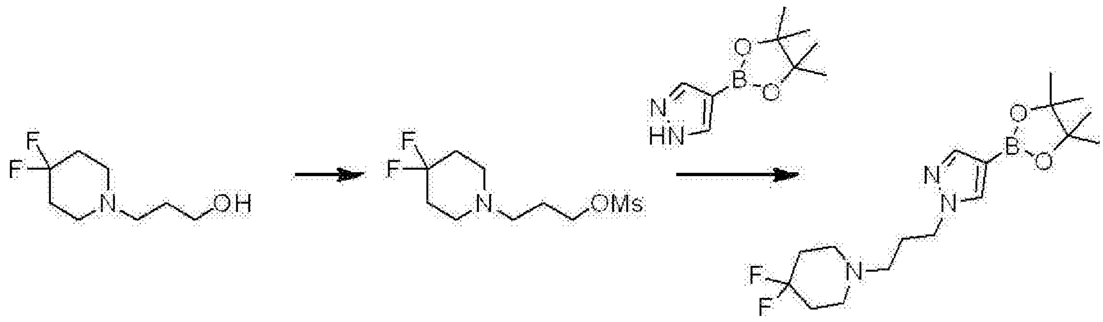

4,4-Difluoro-1-(3-(4-(4,4,5,5-tetramethyl-1,3,2-dioxaborolan-2-yl)-1H-pyrazol-1-yl)propyl)piperidine was obtained as yellow oily substance in the same manner as in Example 0351 except that 3-(4,4-difluoropiperidin-1-yl)propan-1-ol was used instead of the (R)-3-(3-fluoropyrrolidin-1-yl)propan-1-ol used in Example 0351.
$^1$H-NMR($CDCl_3$)δ:7.78(1H,s),7.68(1H,s),4.19(2H,t,J=6.9Hz),2.58-2.43(4H,m),2.35(2H,t,J=6.9Hz),2.10-1.89(6H,m),1.32(12H,s).

<0353-4>

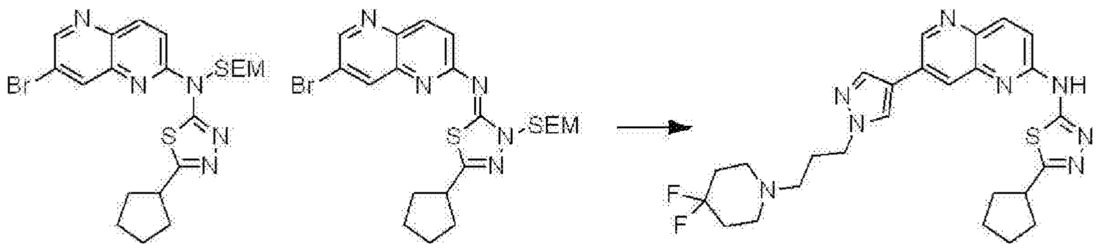

5-Cyclopentyl-N-(7-(1-(3-(4,4-difluoropiperidin-1-yl)propyl)-1H-pyrazol-4-yl)-1,5-naphthyridin-2-yl)-1,3,4-thiadiazole-2-amine was obtained as a white solid in the same manner as in Example 0339 except that 4,4-difluoro-1-(3-(4-(4,4,5,5-tetramethyl-1,3,2-dioxaborolan-2-yl)-1H-pyrazol-1-yl)propyl)piperidine was used instead of the 1-(3-methoxypropyl)-4-(4,4,5,5-tetramethyl-1,3,2-dioxaborolan-2-yl)-1H-pyrazole used in Example 0339.
$^1$H-NMR(DMSO-$d_6$)δ:12.04(1H,s),9.08(1H,d,J=2.0Hz),8.58(1H,s),8.32(1H,d,J=2.0Hz),8.28-8.21(2H,m),7.39(1H,d,J=9.2Hz),4.21(2H,t,J=6.6Hz),3.55-3.42(1H,m),2.35(2H,t,J=6.6Hz),2.23-1.62(18H,m).
MSm/z(M+H):525.

<Example 0354>

<0354-1>

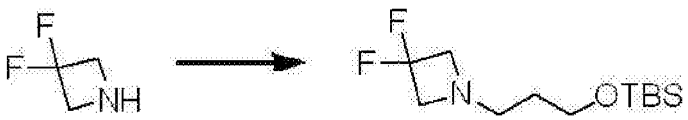

1-(3-((tert-Butyldimethylsilyl)oxy)propyl)-3,3-difluoroazetidine was obtained as colorless oily substance in the same manner as in Example 0351 except that 3,3-difluoroazetidine hydrochloride was used instead of the (R)-3-fluoropyrrolidine hydrochloride used for synthesizing the compound 0351-1 used in Example 0351.
$^1$H-NMR($CDCl_3$)δ:3.65(2H,t,J=6.3Hz),3.54(4H,t,J=11.9Hz),2.62(2H,t,J=6.9Hz),1.65-1.51(2H,m),0.89(9H,s),0.05(6H,s).

<0354-2>

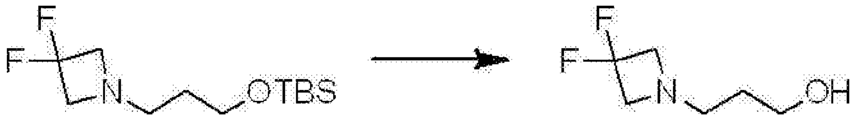

3-(3,3-Difluoroazetidin-1-yl)propan-1-ol was obtained as colorless oily substance in the same manner as in Example 0351 except that 1-(3-((tert-butyldimethylsilyl)oxy)propyl)-3,3-difluoroazetidine was used instead of the (R)-1-(3-((tert-butyldimethylsilyl)oxy)propyl)-3-fluoropyrrolidine used in Example 0351.
$^1$H-NMR($CDCl_3$)δ:3.76(2H,t,J=5.6Hz),3.61(4H,t,J=12.2Hz),2.81(2H,t,J=5.9Hz),1.68-1.58(2H,m).

<0354-3>

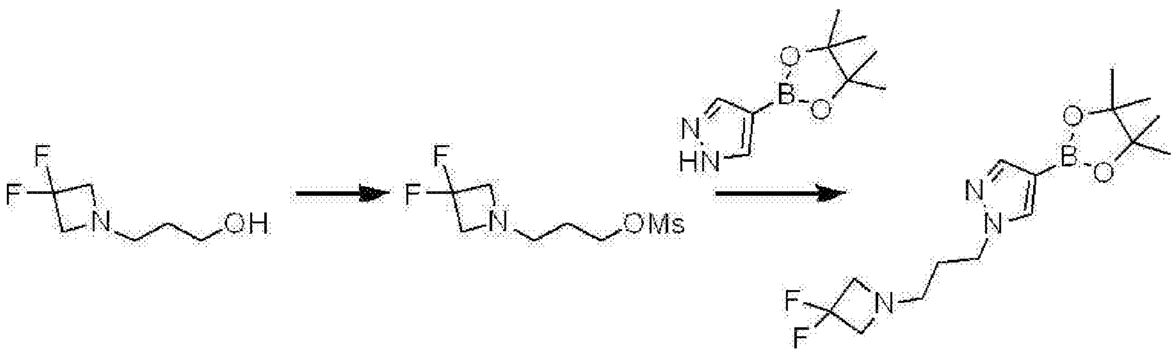

1-(3-(3,3-Difluoroazetidin-1-yl)propyl)-4-(4,4,5,5-tetramethyl-1,3,2-dioxaborolan-2-yl)-1H-pyrazole was obtained as brown oily substance in the same manner as in Example 0351 except that 3-(3,3-difluoroazetidin-1-yl)propan-1-ol was used instead of the (R)-3-(3-fluoropyrrolidin-1-yl)propan-1-ol used in Example 0351.
$^1$H-NMR($CDCl_3$)δ:7.78(1H,s),7.68(1H,s),4.19(2H,t,J=6.9Hz),3.53(4H,t,J=12.2Hz),2.50(2H,t,J=6.9Hz),1. 92(2H,t,J=6. 9Hz), 1.32(12H,s).

<0354-4>

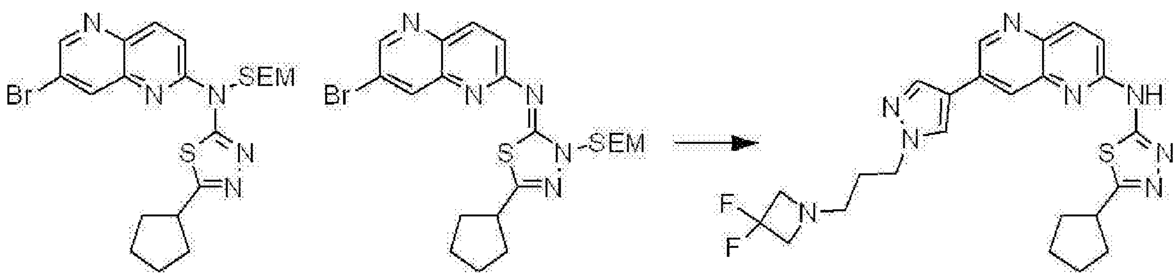

5-Cyclopentyl-N-(7-(1-(3-(3,3-difluoroazetidin-1-yl)propyl)-1H-pyrazol-4-yl)-1,5-naphthyridin-2-yl)-1,3,4-thiadiazole-2-amine was obtained as a yellow solid in the same manner as in Example 0339 except that 1-(3-(3,3-difluoroazetidin-1-yl)propyl)-4-(4,4,5,5-tetramethyl-1,3,2-dioxaborolan-2-yl)-1H-pyrazole was used instead of the 1-(3-methoxypropyl)-4-(4,4,5,5-tetramethyl-1,3,2-dioxaborolan-2-yl)-1H-pyrazole used in Example 0339.
$^1$H-NMR(DMSO-$d_6$)δ:12.04(1H,s),9.08(1H,d,J=2.0Hz),8.57(1H,s),8.33(1H,d,J=2.0Hz),8.28-8.21(2H,m),7.39(1H,d,J=9.2Hz),4.21(2H,t,J=6.9Hz),3.58(4H,t,J=12.6Hz),3.52-3.25(1H,m),2.62-2.46(2H,m),2.24-2.10(2H,m),1.97-1.62(8H,m).

MSm/z(M+H):497.

<Example 0355>

<0355-1>

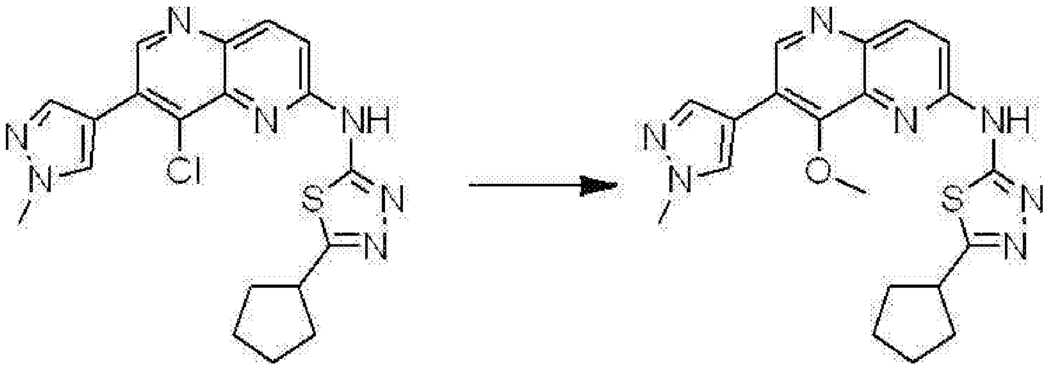

A 5 mol/L sodium methoxide/methanol solution (20 μL) was added to a solution of N-(8-chloro-7-(1-methyl-1H-pyrazol-4-yl)-1,5-naphthyridin-2-yl)-5-cyclopentyl-1,3,4-thiadiazole-2-amine (2.0 mg) in methanol (1 mL), followed by stirring at 150°C for 30 minutes using a microwave reaction apparatus. The reaction mixture was cooled to room temperature, and a 5 mol/L sodium methoxide/methanol solution (50 μL) was added thereto, followed by stirring at 150°C for 1 hour. The reaction mixture was cooled to room temperature, and a saturated ammonium chloride aqueous solution was added thereto. The solvent was distilled off under reduced pressure, and the obtained residue was purified by silica gel column chromatography (chloroform-methanol), thereby obtaining 5-cyclopentyl-N-(8-methoxy-7-(1-methyl-1H-pyrazol-4-yl)-1,5-naphthyridin-2-yl)-1,3,4-thiadiazole-2-amine (0.4 mg) as a yellow solid.
$^1$H-NMR(DMSO-$d_6$)δ:12.10(1H,brs),9.08(1H,s),8.47(1H,s),8.27(1H,d,J=8.9Hz),8.19(1H,s),7.42(1H,d,J=8.9Hz),4.12(3H ,s),3.95(3H,s),3.59-3.25(1H,m),2.26-2.10(2H,m),1.93-1.66(6H,m).
MSm/z(M+H):408.

<Example 0356>

<0356-1>

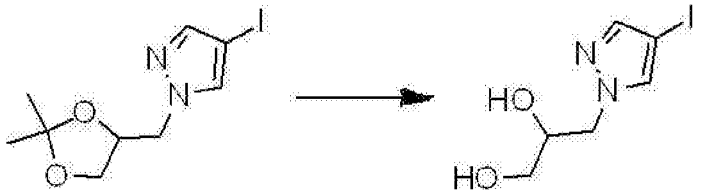

A4 mol/L hydrogen chloride/1,4-dioxane solution (1 mL) was added to a solution of 1-((2,2-dimethyl-1,3-dioxolan-4-yl)methyl)-4-iodo-1H-pyrazole (0.2 g) in methanol (1 mL) at room temperature, followed by stirring for 30 minutes. The solvent was distilled off under reduced pressure, thereby obtaining 3-(4-iodo-1H-pyrazol-1-yl)propane-1,2-diol (184 mg) as a white solid. MSm/z(M+H):269.

<0356-2>

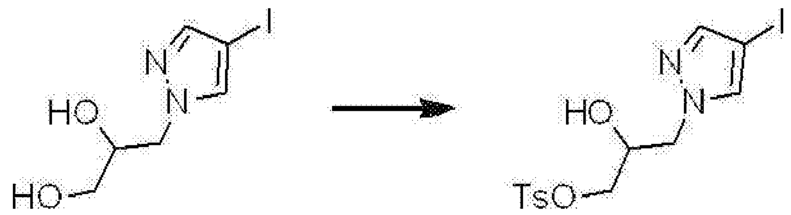

Toluenesulphonyl chloride (78.2 μL) was added to a solution of 3-(4-iodo-1H-pyrazol-1-yl)propane-1,2-diol (100 mg), and pyridine (60.3 μL) in dichloromethane (1.9 mL) in an ice bath, followed by stirring for 30 minutes, and stirring at room temperature for 30 minutes. Toluenesulphonyl chloride (78.2 μL) was added thereto, followed by stirring for 30 minutes. A saturated sodium hydrogen carbonate aqueous solution was added to the reaction mixture, followed by stirring at room temperature for 30 minutes, and ethyl acetate was added thereto. The organic layer was collected by separation, and dried over anhydrous sodium sulfate, then, the solvent was distilled off under reduced pressure, and the obtained residue was purified by silica gel column chromatography (chloroform-methanol), thereby obtaining 2-hydroxy-3-(4-iodo-1H-pyrazol-1-yl)propyl 4-methylbenzenesulfonate (120 mg) as yellow oily substance.
MSm/z(M+H):423.

<0356-3>

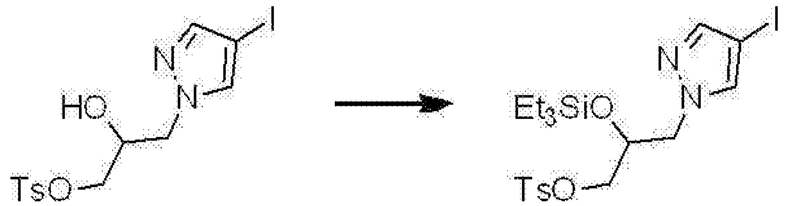

Triethylsilyl trifluoromethanesulfonate (127 μL) was added to a solution of 2-hydroxy-3-(4-iodo-1H-pyrazol-1-yl)propyl 4-methylbenzenesulfonate (120 mg) and 2,6-lutidine (130 μL) in dichloromethane (1.9 mL) at room temperature, followed by stirring for 30 minutes. A saturated sodium hydrogen carbonate aqueous solution and ethyl acetate were added to the reaction mixture. The organic layer was collected by separation, and dried over anhydrous sodium sulfate, and the solvent was distilled off under reduced pressure. The obtained residue was purified by silica gel column chromatography (hexane-ethyl acetate), thereby obtaining 3-(4-iodo-1H-pyrazol-1-yl)-2-((triethylsilyl)oxy)propyl 4-methylbenzenesulfonate (82.0 mg) as yellow oily substance.
MSm/z(M+H):537.

<0356-4>

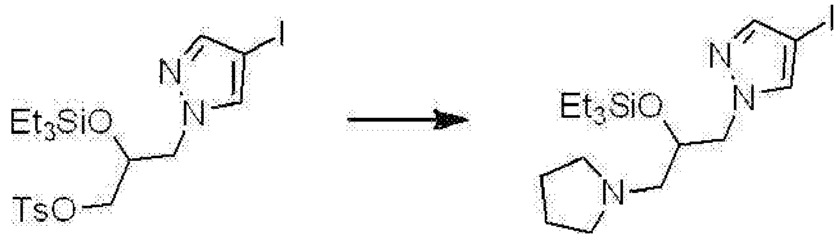

A mixture of 3-(4-iodo-1H-pyrazol-1-yl)-2-((triethylsilyl)oxy)propyl 4-methylbenzenesulfonate (82.0 mg), pyrrolidine (37.1 μL), triethylamine (64 μL), and tetrahydrofuran (1 mL) was stirred at 70°C for 2 hours, and stirred at 80°C for 16 hours. The reaction mixture was cooled to room temperature, and the solvent was distilled off under reduced pressure. The obtained residue was purified by silica gel column chromatography (chloroform-methanol), thereby obtaining 4-iodo-1-(3-(pyrrolidin-1-yl)-2-((triethylsilyl)oxy)propyl)-1H-pyrazole (27.7 mg) as brown oily substance.
MSm/z(M+H):436.

<0356-5>

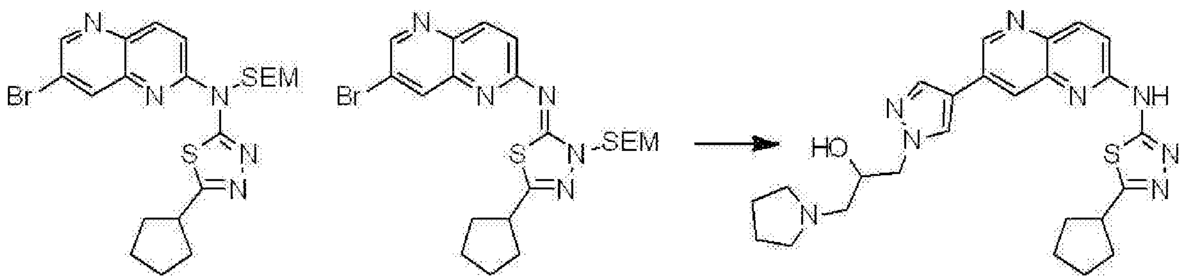

1-(4-(6-((5-Cyclopentyl-1,3,4-thiadiazol-2-yl)amino)-1,5-naphthyridin-3-yl)-1H-pyrazol-1-yl)-3-(pyrrolidin-1-yl)propan-2-ol was obtained as a yellow solid in the same manner as in Example 0336 except that 4-iodo-1-(3-(pyrrolidin-1-yl)-2-((triethylsilyl)oxy)propyl)-1H-pyrazole was used instead of the 2-(4-(4-iodo-1H-pyrazol-1-yl)piperidin-1-yl)ethyl acetate used in Example 0336. $^1$H-NMR(DMSO-$d_6$)$\delta$:12.03(1H,brs),9.09(1H,d,J=2.0Hz),8.52(1H,s),8.34(1H,d,J=2.0Hz),8.27-8.21(2H,m),7.39(1H,d,J=9.2Hz),5.01(1H,d,J=4.6Hz),4.30(1H,d,J=10.6Hz),4.14-3.92(2H,m),3.56-3.42(1H,m),2.69-2.36(6H,m),2.24-2.09(2H,m),1.95-1.62(10H,m).
MSm/z(M+H):491.

<Examples 0357 and 0358>

<0357-1 and 0358-1>

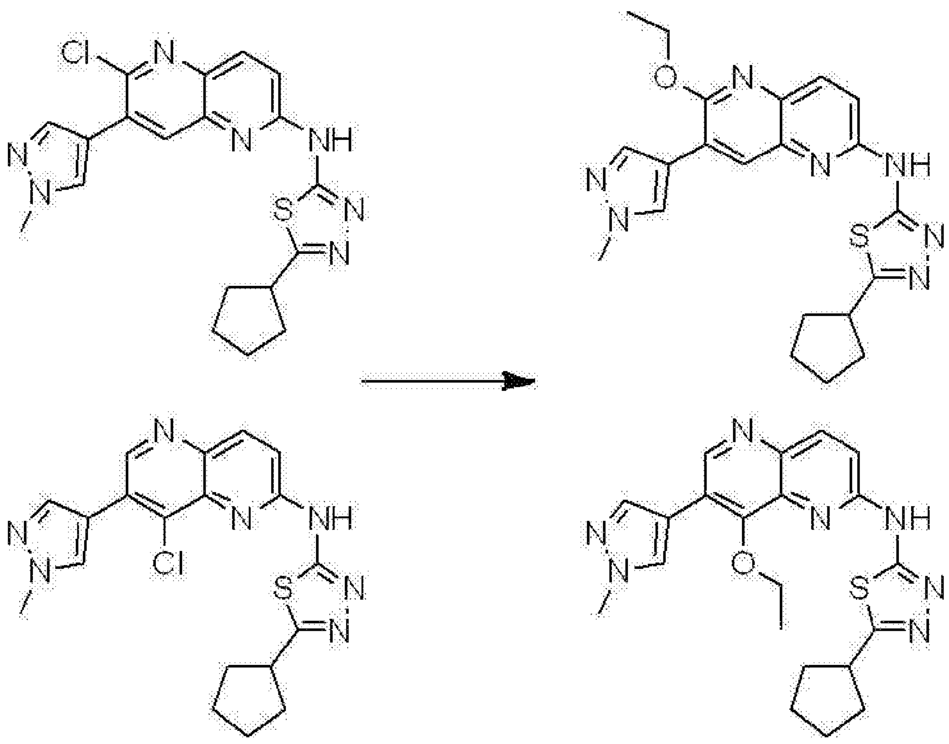

An ethanol solution (0.1 mL) of 20% sodium ethoxide was added to a solution of a mixture (30 mg) of N-(6-chloro-7-(1-methyl-1H-pyrazol-4-yl)-1,5-naphthyridin-2-yl)-5-cyclopentyl-1,3,4-thiadiazole-2-amine and N-(8-chloro-7-(1-methyl-1H-pyrazol-4-yl)-1,5-naphthyridin-2-yl)-5-cyclopentyl-1,3,4-thiadiazole-2-amine in ethanol (1ml), followed by stirring at 150°C for 1.5 hours using a microwave reaction apparatus. The reaction mixture was cooled to room

temperature, and a saturated ammonium chloride aqueous solution was added thereto. The solvent was distilled off under reduced pressure, the obtained residue was purified by silica gel column chromatography (chloroform-methanol), and purified by preparative thin layer silica gel chromatography (chloroform-methanol), thereby obtaining 5-cyclopentyl-N-(6-ethoxy-7-(1-methyl-1H-pyrazol-4-yl)-1,5-naphthyridin-2-yl)-1,3,4-thiadiazole-2-amine (2.0 mg) as a white solid and 5-cyclopentyl-N-(8-ethoxy-7-(1-methyl-1H-pyrazol-4-yl)-1,5-naphthyridin-2-yl)-1,3,4-thiadiazole-2-amine (3.1 mg) as a white solid.

<Example 0357>

5-Cyclopentyl-N-(6-ethoxy-7-(1-methyl-1H-pyrazol-4-yl)-1,5-naphthyridin-2-yl)-1,3,4-thiadiazole-2-amine
$^1$H-NMR(DMSO-$d_6$)δ:11.82(1H,s),8.39(1H,s),8.36(1H,s),8.22(1H,s),8.06(1H,d,J=8.6Hz),7.33(1H,d,J=8.6Hz),4.55(2H,q ,J=6.9Hz),3.94(3H,s),3.53-3.26(1H,m),2.23-2.07(2H,m),1.95-1.64(6H,m),1.48(3H,t,J=6.9Hz).
MSm/z(M+H):422.

<Example 0358>

5-Cyclopentyl-N-(8-ethoxy-7-(1-methyl-1H-pyrazol-4-yl)-1,5-naphthyridin-2-yl)-1,3,4-thiadiazole-2-amine
$^1$H-NMR(DMSO-$d_6$)8:12.10(1H,s),9.05(1H,s),8.43(1H,s),8.27(1H,d,J=8.9Hz),8.17(1H,s),7.41(1H,d,J=8.9Hz),4.34(2H,q ,J=6.9Hz),3.96(3H,s),3.59-3.44(1H,m),2.31-2.08(2H,m),1.96-1.63(6H,m),1.54(3H,t,J=6.9Hz).
MSm/z(M+H):422.

<Example 0359>

<0359-1>

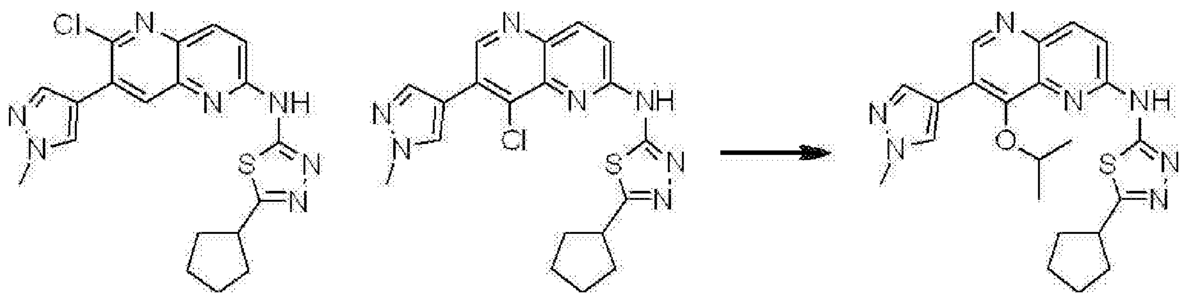

60% sodium hydride (29.1 mg) was added to 2-propanol (1 mL) at room temperature, and a mixture (30 mg) of N-(6-chloro-7-(1-methyl-1H-pyrazol-4-yl)-1,5-naphthyridin-2-yl)-5-cyclopentyl-1,3,4-thiadiazole-2-amine andN-(8-chloro-7-(1-methyl-1H-pyrazol-4-yl)-1,5-naphthyridin-2-yl)-5-cyclopentyl-1,3,4-thiadiazole-2-amine was added thereto at room temperature, followed by stirring at 150°C for 1.5 hours using a microwave reaction apparatus. The reaction mixture was cooled to room temperature, and a saturated ammonium chloride aqueous solution was added thereto. The solvent was distilled off under reduced pressure, the obtained residue was purified by silica gel column chromatography (chloroform-methanol), and purified by preparative thin layer silica gel chromatography (chloroform-methanol), thereby obtaining 5-cyclopentyl-N-(8-isopropyloxy-7-(1-methyl-1H-pyrazol-4-yl)-1,5-naphthyridin-2-yl)-1,3,4-thiadiazole-2-amine (1.8 mg) as a white solid.
$^1$H-NMR(DMSO-$d_6$)δ:12.06(1H,s),9.02(1H,s),8.43(1H,s),8.26(1H,d,J=9.2Hz),8.18(1H,s),7.41(1H,d,J=9.2Hz),5.52-5.39(1H,m),3.95(3H,s),3.58-3.43(1H,m),2.30-2.11(2H,m),1.93-1.64(6H,m),1.26(6H,d,J=6.6Hz).
MSm/z(M+H):436.

<Example 0360>

<0360-1>

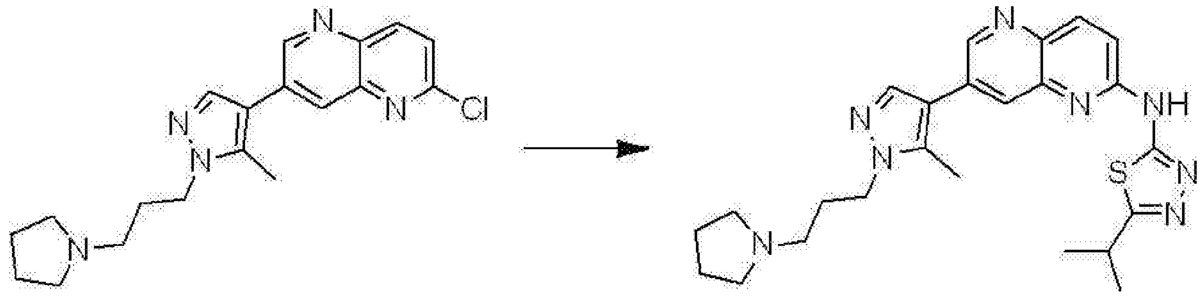

5-Isopropyl-N-(7-(5-methyl-1-(3-(pyrrolidin-1-yl)propyl)-1H-pyrazol-4-yl)-1,5-naphthyridin-2-yl)-1,3,4-thiadiazole-2-amine was obtained as a yellow solid in the same manner as in Example 0321 except that 2-chloro-7-(5-methyl-1-(3-(pyrrolidin-1-yl)propyl)-1H-pyrazol-4-yl)-1,5-naphthyridine was used instead of the 2-chloro-7-(3-methyl-1-(3-(pyrrolidin-1-yl)propyl)-1H-pyrazol-4-yl)-1,5-naphthyridine used in Example 0321.
$^1$H-NMR(DMSO-$d_6$)δ:12.07(1H,brs),8.88(1H,d,J=2.0Hz),8.28(1H,d,J=8.9Hz),8.15(1H,d,J=2.0Hz),7.92(1H,s),7.43(1H,d ,J=8.9Hz),4.18(2H,t,J=6.9),3.42-3.27(1H,m),2.58-2.34(9H,m),2.03-1.88(2H,m),1.76-1.62(4H,m), 1.40(6H,d,J=6.6Hz).
MSm/z(M+H):463.

<Example 0361>

<0361-1>

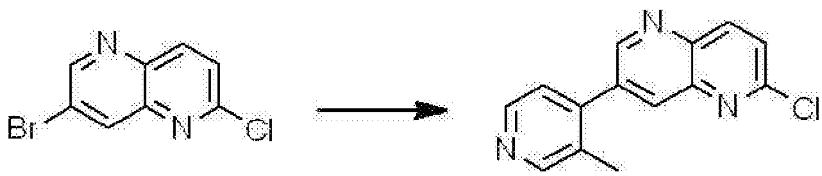

(3-Methylpyridin-4-yl)boronic acid (34 mg), bis(di-tert-butyl (4-dimethylaminophenyl)-phosphine)dichloropalladium(II) (14 mg), and sodium carbonate (45 mg) were added to a mixture solution of 7-bromo-2-chloro-1,5-naphthyridine (50 mg) in 1,4-dioxane (5 mL)/water (0.5 mL), followed by stirring at 100°C for 2 hours in a nitrogen atmosphere. The solvent was distilled off under reduced pressure, and the obtained residue was purified by silica gel column chromatography (hexane/ethyl acetate → ethyl acetate/methanol), thereby obtaining 2-chloro-7-(3-methylpyridin-4-yl)-1,5-naphthyridine (30 mg).
$^1$H-NMR(DMSO-$d_6$)δ:9.11(1H,d,J=2.0Hz),8.64-8.49(4H,m),7.93(1H,d,J=8.6Hz),7.48(1H,d,J=5.3Hz),2.34(3H,s).

<0361-2>

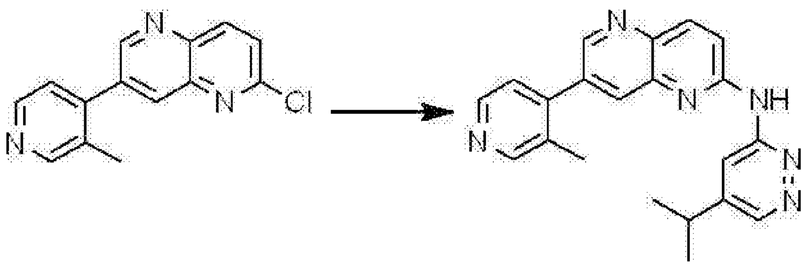

5-Isopropylpyridazine-3-amine (19 mg), tris(dibenzylideneacetone)dipalladium(0) (9 mg), 4,5-bis(diphenylphosphino)-9,9-dimethylxanthene (12 mg), and cesium carbonate (98 mg) were added to a solution of 2-chloro-7-(3-methylpyridin-4-yl)-1,5-naphthyridine (30 mg) in 1,4-dioxane (5 mL), followed by stirring at 140°C for 30 minutes using a microwave reaction apparatus. The solvent was distilled off under reduced pressure, the obtained residue was purified by silica gel column chromatography (hexane/ethyl acetate → ethyl acetate/methanol), and purified by silica gel column chromatography (chloroform/methanol, NH silica), thereby obtaining N-(5-isopropylpyridazin-3-yl)-7-(3-methylpyridin-4-yl)-1,5-naphthyridine-2-amine (13 mg) as a white solid.
$^1$H-NMR(DMSO-$d_6$)δ:10.83(1H,s),8.86(1H,d,J=2.0Hz),8.79(1H,d,J=2.0Hz),8.76(1H,d,J=2.0Hz),8.61(1H,s),8.55(1H,d,J=5.3Hz),8.33(1H,d,J=9.2Hz),8.20(1H,d,J=2.0Hz),7.83(1H,d,J=9.2Hz),7.46(1H,d,J=5.3Hz),3.09-2.94(1H,m),2.33(3H,s),1.29(6H,d,J=6.6Hz).
MSm/z(M+H):357.

<Example 0362>

<0362-1>

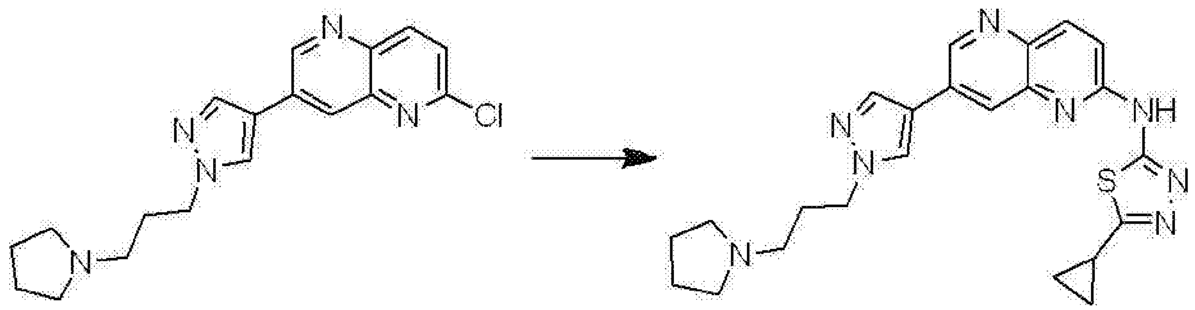

5-Cyclopropyl-N-(7-(1-(3-(pyrrolidin-1-yl)propyl)-1H-pyrazol-4-yl)-1,5-naphthyridin-2-yl)-1,3,4-thiadiazole-2-amine was obtained as a white solid in the same manner as in Example 0301 except that 2-chloro-7-(1-(3-(pyrrolidin-1-yl)propyl)-1H-pyrazol-4-yl)-1,5-naphthyridine was used instead of the 3-(4-(6-chloro-1,5-naphthyridin-3-yl)-1H-pyrazol-1-yl)-N,N-dimethylpropane-1-amine used in Example 0301, and 5-cyclopropyl-1,3,4-thiadiazole-2-amine was used instead of the 1,3,4-thiadiazole-2-amine used in Example 0301.
$^1$H-NMR(DMSO-$d_6$)δ:12.04(1H,brs),9.08(1H,d,J=2.1Hz),8.58(1H,s),8.33(1H,d,J=2.1Hz),8.27-8.21(2H,m),7.40(1H,d,J=9.3Hz),4.23(2H,t,J=7.2Hz),2.44-2.14(6H,m),2.12-1.88(6H,m),1.80-1.40(6H,m).
MSm/z(M+H):447.

<Example 0363>

<0363-1>

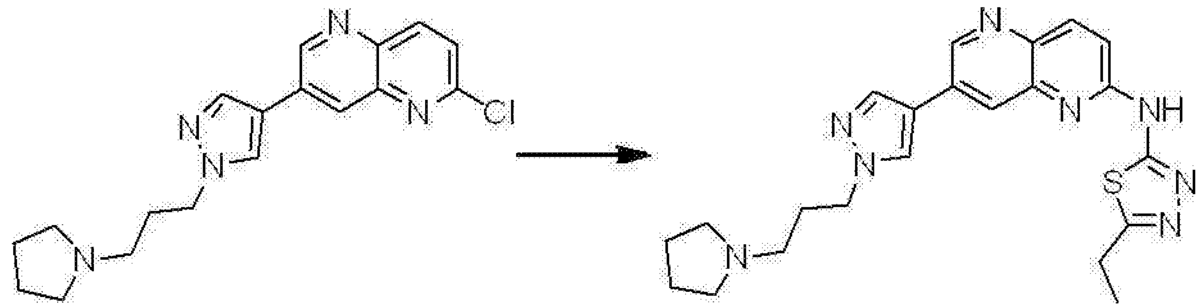

5-Ethyl-N-(7-(1-(3-(pyrrolidin-1-yl)propyl)-1H-pyrazol-4-yl)-1,5-naphthyridin-2-yl)-1,3,4-thiadiazole-2-amine was obtained as a white solid in the same manner as in Example 0301 except that 2-chloro-7-(1-(3-(pyrrolidin-1-yl)propyl)-1H-pyrazol-4-yl)-1,5-naphthyridine was used instead of the 3-(4-(6-chloro-1,5-naphthyridin-3-yl)-1H-pyrazol-1-yl)-N,N-dimethylpropane-1-amine used in Example 0301, and 5-ethyl-1,3,4-thiadiazole-2-amine was used instead of the 1,3,4-thiadiazole-2-amine used in Example 0301.
$^1$H-NMR(DMSO-$d_6$)$\delta$:12.05(1H,brs),9.08(1H,s),8.58(1H,s),8.33(1H,m),8.28-8.20(2H,m),7.40(1H,d,J=9.3Hz),4.22(2H,t,J=7.2Hz),3.03(2H,m),2.48-2.38(4H,m),2.02(2H,m),1.70(4H,m),1.38(3H,t,J=8.1Hz).
MSm/z(M+H):435.

<Example 0364>

<0364-1>

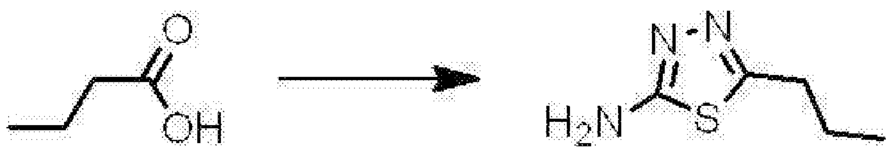

5-Propyl-1,3,4-thiadiazole-2-amine was obtained as a white solid in the same manner as in Example 0198 except that butyric acid was used instead of the cyclobutanecarboxylic acid used in Example 0198.
$^1$H-NMR(DMSO-$d_6$)$\delta$:6.98(2H,brs),2.76(2H,t,J=7.2Hz),1.62(2H,m),0.92(3H,t,J=7.5Hz).

<0364-2>

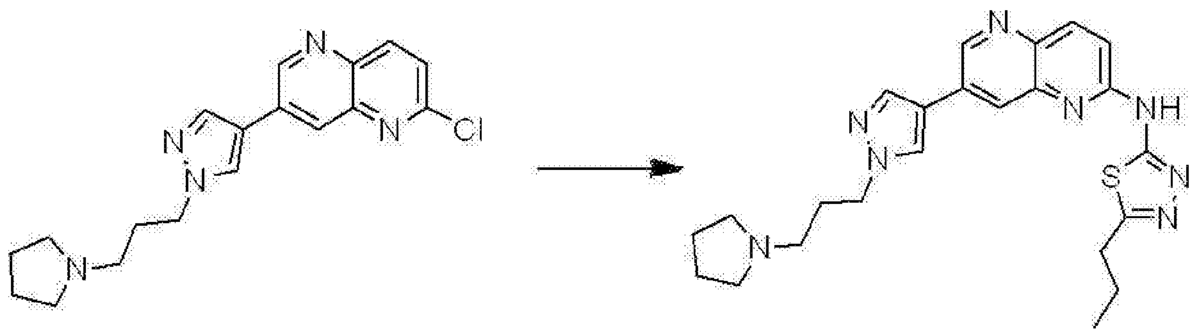

5-Propyl-N-(7-(1-(3-(pyrrolidin-1-yl)propyl)-1H-pyrazol-4-yl)-1,5-naphthyridin-2-yl)-1,3,4-thiadiazole-2-amine was obtained as a white solid in the same manner as in Example 0301 except that 2-chloro-7-(1-(3-(pyrrolidin-1-yl)propyl)-1H-pyrazol-4-yl)-1,5-naphthyridine was used instead of the 3-(4-(6-chloro-1,5-naphthyridin-3-yl)-1H-pyrazol-1-yl)-N,N-dimethylpropane-1-amine used in Example 0301, and 5-propyl-1,3,4-thiadiazole-2-amine was used instead of the 1,3,4-thiadiazole-2-amine used in Example 0301.

$^1$H-NMR(DMSO-$d_6$)δ:12.05(1H,brs),9.08(1H,s),8.58(1H,s),8.33(1H,m),8.28-8.22(2H,m),7.40(1H,d,J=9.0Hz),4.22(2H,t,J=7.2Hz),2.99(2H,m),2.03(2H,m),1.88-1.66(6H,m),1.38(3H,t,J=7.2Hz).
MSm/z(M+H):449.

<Example 0365>

<0365-1>

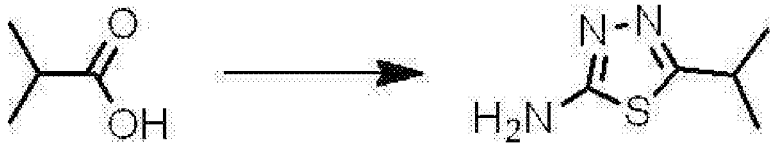

5-Isopropyl-1,3,4-thiadiazole-2-amine was obtained as a white solid in the same manner as in Example 0198 except that isobutyric acid was used instead of the cyclobutanecarboxylic acid used in Example 0198.
$^1$H-NMR(DMSO-$d_6$)δ:6.99(2H,brs),3.12(1H,m),0.92(6H,d,J=6.9Hz).

<0365-2>

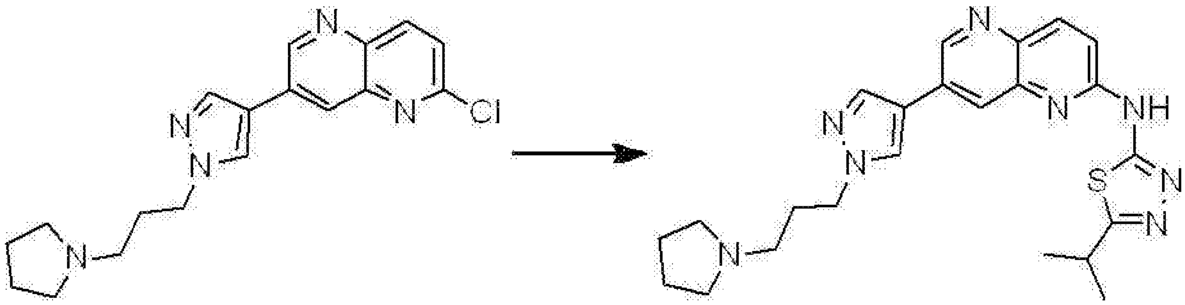

5-Isopropyl-N-(7-(1-(3-(pyrrolidin-1-yl)propyl)-1H-pyrazol-4-yl)-1,5-naphthyridin-2-yl)-1,3,4-thiadiazole-2-amine was obtained as a white solid in the same manner as in Example 0301 except that 2-chloro-7-(1-(3-(pyrrolidin-1-yl)propyl)-1H-pyrazol-4-yl)-1,5-naphthyridine was used instead of the 3-(4-(6-chloro-1,5-naphthyridin-3-yl)-1H-pyrazol-1-yl)-N,N-dimethylpropane-1-amine used in Example 0301, and 5-isopropyl-1,3,4-thiadiazole-2-amine was used instead of the 1,3,4-thiadiazole-2-amine used in Example 0301.
$^1$H-NMR(DMSO-$d_6$)δ:12.05(1H,brs),9.08(1H,d,J=2.7Hz),8.58(1H,s),8.33(1H,m),8.28-8.20(2H,m),7.39(1H,d,J=8.7Hz),4.22(2H,t,J=7.8Hz),2.46-2.36(4H,m),2.01(2H,m),1.69(4H,m),1.42(6H,d,J=7.2Hz).
MSm/z(M+H):449.

<Example 0366>

<0366-1>

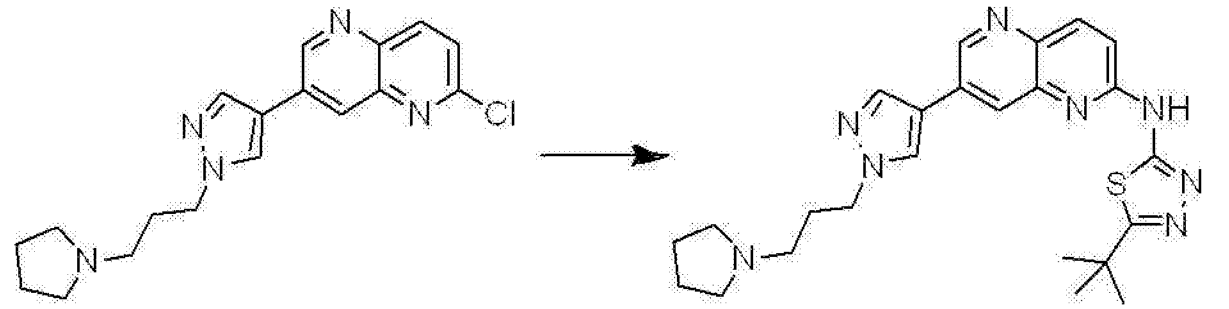

5-(tert-Butyl)-N-(7-(1-(3-(pyrrolidin-1-yl)propyl)-1H-pyrazol-4-yl)-1,5-naphthyridin-2-yl)-1,3,4-thiadiazole-2-amine was obtained as a white solid in the same manner as in Example 0301 except that 2-chloro-7-(1-(3-(pyrrolidin-1-yl)propyl)-1H-pyrazol-4-yl)-1,5-naphthyridine was used instead of the 3-(4-(6-chloro-1,5-naphthyridin-3-yl)-1H-pyrazol-1-yl)-N,N-dimethylpropane-1-amine used in Example 0301, and 5-(tert-butyl)-1,3,4-thiadiazole-2-amine was used instead of the 1,3,4-thiadiazole-2-amine used in Example 0301.
$^1$H-NMR(DMSO-$d_6$)δ:12.05(1H,brs),9.08(1H,d,J=2.7Hz),8.57(1H,s),8.33(1H,m),8.28-8.20(2H,m),7.39(1H,d,J=9.3Hz),4.22(2H,t,J=7.2Hz),2.46-2.36(4H,m),2.01(2H,m),1.69(4H,m),1.48(9H,s).
MSm/z(M+H):463.

<Example 0367>

<0367-1>

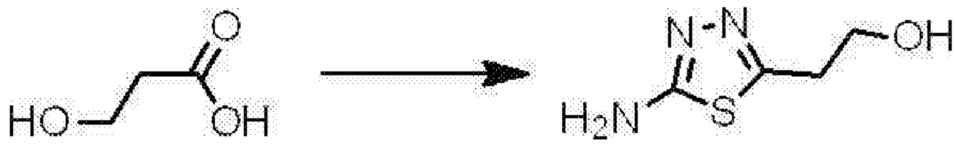

2-(5-Amino-1,3,4-thiadiazol-2-yl)ethanol was obtained as a white solid in the same manner as in Example 0198 except that 3-hydroxypropionic acid was used instead of the cyclobutanecarboxylic acid used in Example 0198.
$^1$H-NMR(DMSO-$d_6$)δ:7.33(1H,brs),7.08(2H,brs),3.18(1H,t,J=7.2Hz).

<0367-2>

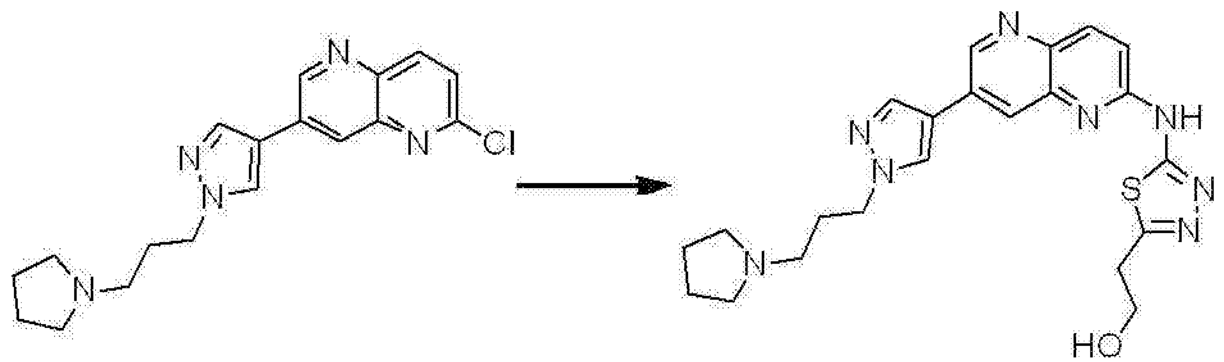

2-(5-((7-(1-(3-(Pyrrolidin-1-yl)propyl)-1H-pyrazol-4-yl)-1,5-naphthyridin-2-yl)amino)-1,3,4-thiadiazol-2-yl)ethanol was obtained as a white solid in the same manner as in Example 0301 except that 2-chloro-7-(1-(3-(pyrrolidin-1-yl)propyl)-1H-pyrazol-4-yl)-1,5-naphthyridine was used instead of the 3-(4-(6-chloro-1,5-naphthyridin-3-yl)-1H-pyrazol-1-yl)-N,N-dimethylpropane-1-amine used in Example 0301, and 2-(5-amino-1,3,4-thiadiazol-2-yl)ethanol was used instead of the 1,3,4-thiadiazole-2-amine used in Example 0301.
$^1$H-NMR(DMSO-$d_6$)δ:12.04(1H,brs),9.08(1H,d,J=2.1Hz),8.57(1H,s),8.30-8.18(3H,m),7.40(1H,d,J=8.7Hz),5.02(1H,m),4.22(2H,t,J=7.2Hz),3.80(2H,m),3.14(2H,m),2.44(4H,m), 2.02(2H,m),1.70(4H,m).
MSm/z(M+H):451.

<Example 0368>

<0368-1>

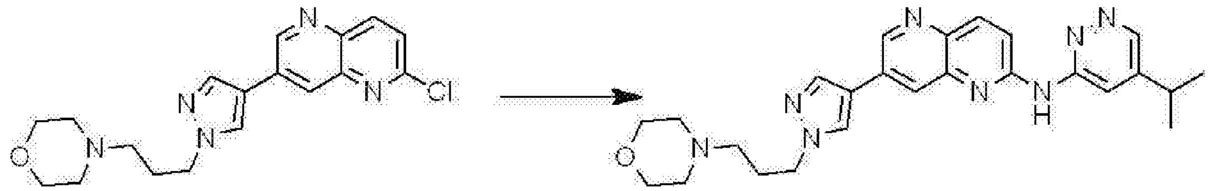

N-(5-isopropylpyridazin-3-yl)-7-(1-(3-morpholinopropyl)-1H-pyrazol-4-yl)-1,5-naphthyridine-2-amine was obtained as a white solid in the same manner as in Example 0001 except that 5-isopropylpyridazine-3-amine was used instead of the 1,3,4-thiadiazole-2-amine used in Example 0001.
$^1$H-NMR($CDCl_3$)δ:8.93(1H,d,J=2.1Hz),8.82(2H,s),8.68(1H,brs),8.24(1H,d,J=9.3Hz),8.10(1H,d,J=1.5Hz),7.97(1H,s),7.87(1H,s),7.51(1H,d,J=8.4Hz),4.31(2H,m),3.73(4H,m),3.06(1H,m),2.50-2.34(6H,m),2.13(2H,m),1.42(6H,d,J=6.6Hz).
MSm/z(M+H):459.

<Example 0369>

<0369-1>

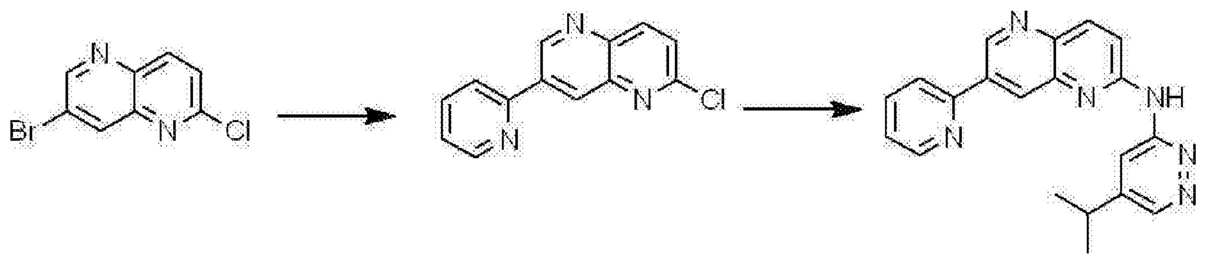

2-(Tributylstannyl)pyridine (79 μL) and tetrakis(triphenylphosphine)palladium(0) (46 mg) were added to a solution of 7-bromo-2-chloro-1,5-naphthyridine (50 mg) in 1,4-dioxane (5 mL), followed by stirring at 80°C for 2.5 hours in a nitrogen atmosphere, and the resultant product was refluxed for 6 hours. The solvent was distilled off under reduced pressure, and the obtained residue was purified by silica gel column chromatography (hexane/ethyl acetate), thereby obtaining 2-chloro-7-(pyridin-2-yl)-1,5-naphthyridine (54 mg).

5-Isopropylpyridazine-3-amine (36 mg), tris(dibenzylideneacetone)dipalladium(0) (18 mg), 4,5-bis(diphenylphosphino)-9,9-dimethylxanthene (23 mg), and cesium carbonate (179 mg) were added to a solution of the obtained 2-chloro-7-(pyridin-2-yl)-1,5-naphthyridine (54 mg) in 1,4-dioxane (5 mL), followed by stirring at 140°C for 30 minutes using a microwave reaction apparatus. The reaction mixture was purified by silica gel column chromatography (chloroform/methanol, NH silica), purified by preparative thin layer silica gel chromatography (chloroform/methanol, NH silica), and purified by preparative thin layer silica gel chromatography (chloroform/methanol), thereby obtaining N-(5-isopropylpyridazin-3-yl)-7-(pyridin-2-yl)-1,5-naphthyridine-2-amine (2 mg) as a white solid.
$^1$H-NMR(DMSO-$d_6$)δ:10.81(1H,s),9.46(1H,d,J=2.0Hz),8.88(1H,d,J=2.0Hz),8.82-8.76(2H,m),8.70(1H,brs),8.34-8.24(2H,m),8.05-7.96(1

H,m),7.80(1H,d,J=9.2Hz),7.53-7.43(1H,m),3.13-2.98(1H,m),1.33(6H,d,J=7.3Hz).
MSm/z(M+H):343.

<Example 0370>

<0370-1>

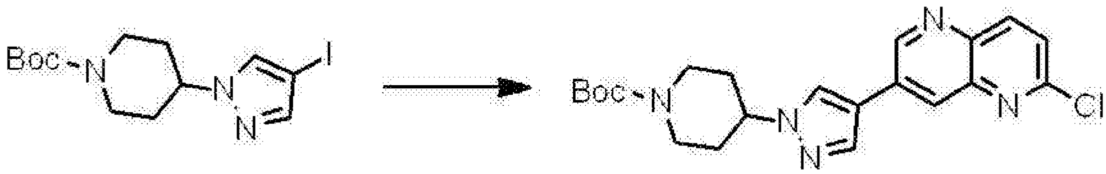

A mixture of 7-bromo-2-chloro-1,5-naphthyridine (300 mg), bis(pinacolato)diboron (469 mg), (1,1'-bis(diphenylphosphino)ferrocene)palladium(II) dichloride (100 mg), potassium acetate (241 mg), and 1,4-dioxane (12.3 mL) was stirred at 100°C for 3 hours in a nitrogen atmosphere. The reaction mixture was cooled to room temperature, and tert-butyl 4-(4-iodo-1H-pyrazol-1-yl)piperidine-1-carboxylate (557 mg), sodium carbonate (261 mg), bis(di-tert-butyl (4-dimethylaminophenyl)phosphine)dichloropalladium(II) (87 mg), and water (1.2 mL) were added thereto, followed by stirring at 110°C for 1 hour. The reaction mixture was cooled to room temperature, the solvent was distilled off under reduced pressure, and the obtained residue was purified by silica gel column chromatography (ethyl acetate-hexane, chloroform-methanol), thereby obtaining tert-butyl 4-(4-(6-chloro-1,5-naphthyridin-3-yl)-1H-pyrazol-1-yl)piperidine-1-carboxylate (149 mg) as a white solid.
MSm/z(M+H):414.

<0370-2>

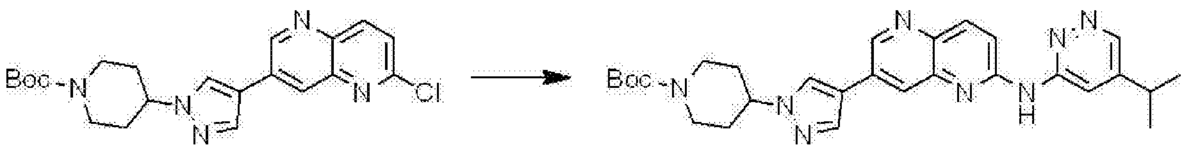

tert-Butyl 4-(4-(6-((5-isopropylpyridazin-3-yl)amino)-1,5-naphthyridin-3-yl)-1H-pyrazol-1-yl)piperidine-1-carboxylate (56 mg) was obtained as a white solid in the same manner as in Example 0001 except that tert-butyl 4-(4-(6-chloro-1,5-naphthyridin-3-yl)-1H-pyrazol-1-yl)piperidine-1-carboxylate was used instead of the 4-(3-(4-(6-chloro-1,5-naphthyridin-3-yl)-1H-pyrazol-1-yl)propyl)morpholine used in Example 0001, and 5-isopropylpyridazine-3-amine was used instead of the 1,3,4-thiadiazole-2-amine used in Example 0001.
MSm/z(M+H):515.

<0370-3>

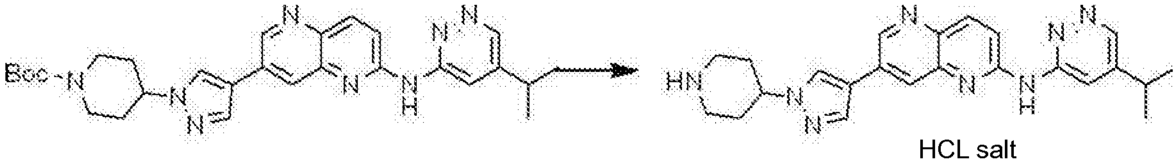


A 4 mol/L hydrogen chloride/1,4-dioxane solution (1 mL) was added to a solution of tert-butyl 4-(4-(6-((5-isopropylpyridazin-3-yl)amino)-1,5-naphthyridin-3-yl)-1H-pyrazol-1-yl)piperidine-1-carboxylate (50 mg) in methanol (1 mL) at room temperature, followed by stirring for 13 hours. The solvent was distilled off under reduced pressure, ethyl

acetate was added to the obtained residue, and the solid matter was collected by filtration, thereby obtaining N-(5-isopropylpyridazin-3-yl)-7-(1-(piperidin-4-yl)-1H-pyrazol-4-yl)-1,5-naphthyridine-2-amine hydrochloride (44.9 mg) as a yellow solid.
MSm/z(M+H):415.

<0370-4>

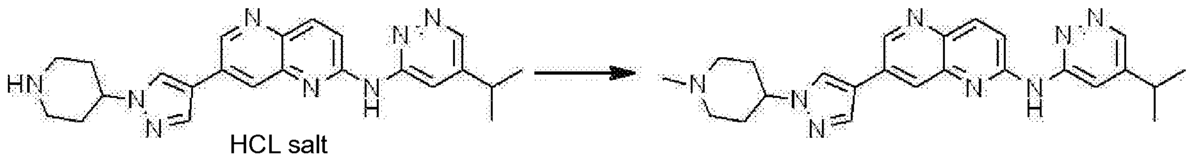


A 36 to 38% formaldehyde aqueous solution (0.33 mL) and sodium triacetoxyborohydride (103 mg) were added to a solution of N-(5-isopropylpyridazin-3-yl)-7-(1-(piperidin-4-yl)-1H-pyrazol-4-yl)-1,5-naphthyridine-2-amine hydrochloride (44.9 mg) in methanol (3.3 mL) and dichloromethane (3.3 mL) at room temperature, followed by stirring for 12 hours. Chloroform, a saturated sodium hydrogen carbonate aqueous solution, and a saturated sodium chloride aqueous solution were added to the reaction mixture. The organic layer was collected by separation, and dried over anhydrous sodium sulfate, and the solvent was distilled off under reduced pressure. The obtained residue was purified by silica gel column chromatography (chloroform-methanol, NH silica), thereby obtaining N-(5-isopropylpyridazin-3-yl)-7-(1-(1-methylpiperidin-4-yl)-1H-pyrazol-4-yl)-1,5-naphthyridine-2-amine (10.0 mg) as a white solid.
$^1$H-NMR(DMSO-$d_6$)$\delta$:8.96(1H,d,J=2.7Hz),8.85-8.78(2H,m),8.69(1H,brs),8.24(1H,d,J=8.7Hz),8.10(1H,m),7.97(1H,s),7.89(1H,s),7.49(1H,d,J=9.3Hz), 4.23(1H,m),3.10-2.96(3H,m),2.37(3H,s),2.30-2.08(6H,m),1.42(6H,d,J=7.2Hz).
MSm/z(M+H):429.

<Example 0371>

<0371-1>

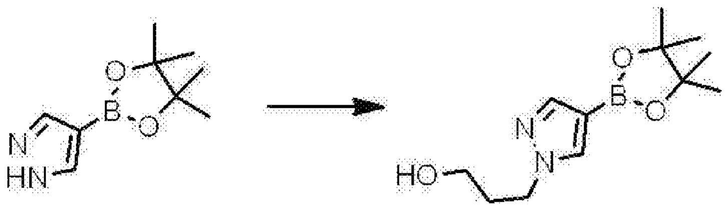

3-(4-(4,4,5,5-Tetramethyl-1,3,2-dioxaborolan-2-yl)-1H-pyrazol-1-yl)propan-1-ol was obtained as brown oily substance in the same manner as in Example 0316 except that 3-bromo-1-propanol was used instead of the 3-bromopropyl acetate used in Example 0316.
MSm/z(M+H):253.

<0371-2>

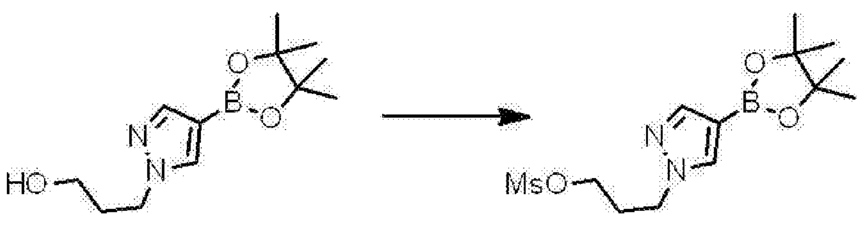

Triethylamine (550 μL) and methanesulfonyl chloride (168 μL) were added to a solution of 3-(4-(4,4,5,5-tetramethyl-1,3,2-dioxaborolan-2-yl)-1H-pyrazol-1-yl)propan-1-ol (500 mg) in dichloromethane (19.8 mL) in an ice bath, followed by stirring at room temperature for 15 minutes. A saturated sodium hydrogen carbonate aqueous solution and chloroform were added to the reaction mixture. The organic layer was collected by separation, washed with a saturated sodium chloride aqueous solution, and dried over anhydrous sodium sulfate, and the solvent was distilled off under reduced pressure. The obtained residue was purified by silica gel column chromatography (chloroform-methanol), thereby obtaining 3-(4-(4,4,5,5-tetramethyl-1,3,2-dioxaborolan-2-yl)-1H-pyrazol-1-yl)propyl methanesulfonate (667 mg) as brown oily substance.
MSm/z(M+H):331.

<0371-3>

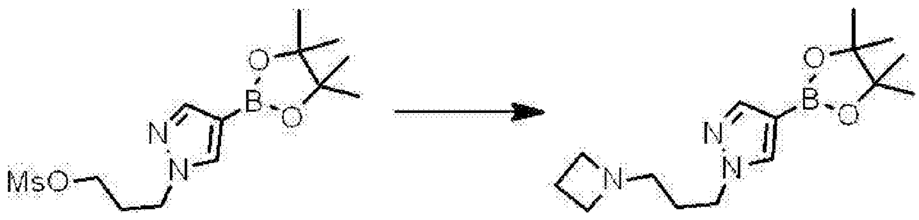

Azetidine (534 μL) was added to a mixture of 3-(4-(4,4,5,5-tetramethyl-1,3,2-dioxaborolan-2-yl)-1H-pyrazol-1-yl)propyl methanesulfonate (667 mg), cesium carbonate (1.29 g), sodium iodide (89 mg), and 1,4-dioxane (10 mL), followed by stirring at 80°C for 11 hours. The reaction mixture was cooled to room temperature, and the solvent was distilled off under reduced pressure, thereby obtaining 1-(3-(azetidin-1-yl)propyl)-4-(4,4,5,5-tetramethyl-1,3,2-dioxaborolan-2-yl)-1H-pyrazole (188 mg) as brown oily substance.
MSm/z(M+H):292.

<0371-4>

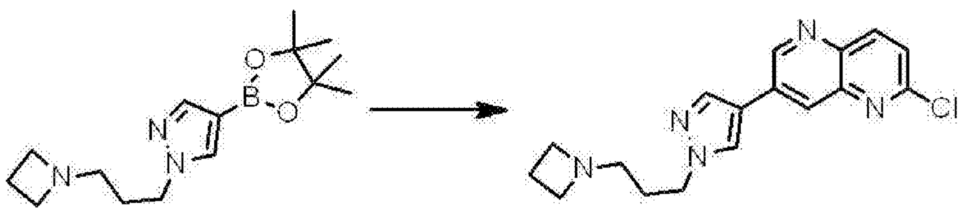

7-(1-(3-(Azetidin-1-yl)propyl)-1H-pyrazol-4-yl)-2-chloro-1,5-naphthyridine was obtained as yellow oily substance in the same manner as in Example 0001 except that 1-(3-(azetidin-1-yl)propyl)-4-(4,4,5,5-tetramethyl-1,3,2-dioxaborolan-2-yl)-1H-pyrazole was used instead of the 1-(3-morpholinopropyl)-1H-pyrazole-4-boronic acid pinacol ester used in Example 0001.
MSm/z(M+H):328.

<0371-5>

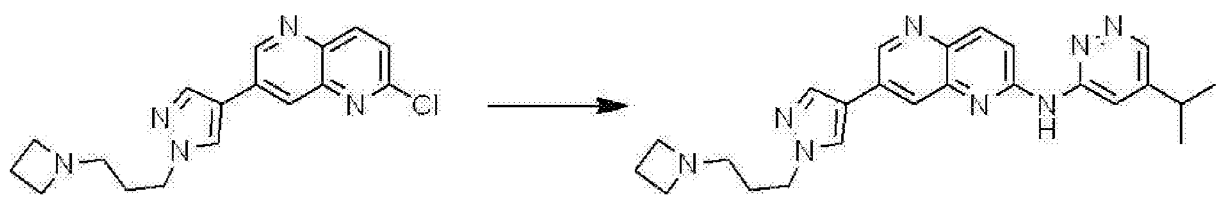

7-(1-(3-(Azetidin-1-yl)propyl)-1H-pyrazol-4-yl)-N-(5-isopropylpyridazin-3-yl)-1,5-naphthyridine-2-amine was obtained as a white solid in the same manner as in Example 0001 except that 5-isopropylpyridazine-3-amine was used instead of the 1,3,4-thiadiazole-2-amine used in Example 0001, and 7-(1-(3-(azetidin-1-yl)propyl)-1H-pyrazol-4-yl)-2-chloro-1,5-naphthyridine was used instead of the 4-(3-(4-(6-chloro-1,5-naphthyridin-3-yl)-1H-pyrazol-1-yl)propyl)morpholine used in Example 0001.
$^1H$-NMR($CDCl_3$)δ:8.94-8.88(2H,m),8.86(1H,d,J=1.2Hz),8.82(1H,d,J=1.8Hz),8.24(1H,d,J=9.3Hz),8.10(1H,d,J=1.2Hz),7.96(1H,s),7.88(1H,s),7.54(1H,d,J=9.3Hz),4.27(2H,t,J=6.6Hz),3.19(4H,m),3.06(1H,m),2.42(2H,m),2.08(2H, m), 1.96(2H,m), 1.42(6H,d,J=7.2Hz).
MSm/z(M+H):429.

<Example0372>

<0372-1>

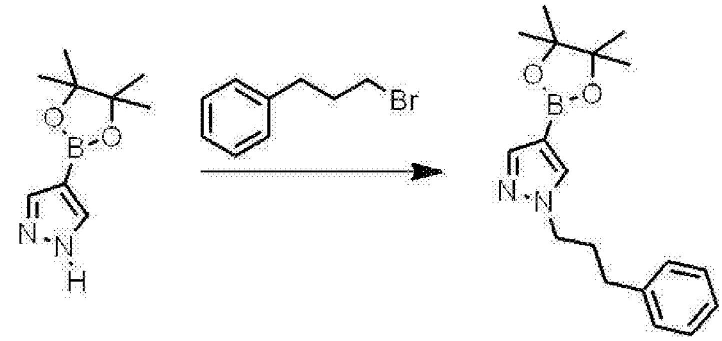

3-Phenylpropyl bromide (0.12 mL) was added to a suspension of 4-(4,4,5,5-tetramethyl-1,3,2-dioxaborolan-2-yl)-1H-pyrazole (100 mg) and potassium carbonate (144 mg) in acetonitrile (1 mL), followed by stirring at 80°C overnight. The reaction mixture was cooled to room temperature, the insolubles were filtered off, and the solvent was distilled off under reduced pressure. The obtained residue was purified by silica gel column chromatography (ethyl acetate-hexane), thereby obtaining 1-(3-phenylpropyl)-4-(4,4,5,5-tetramethyl-1,3,2-dioxaborolan-2-yl)-1H-pyrazole (61 mg).
MSm/z(M+H):313.

<0372-2>

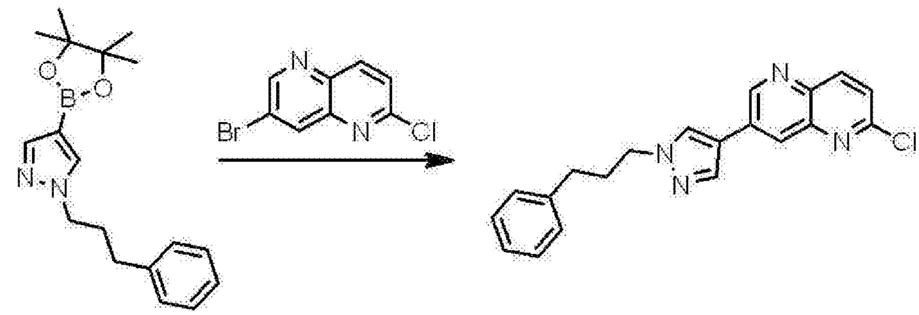

2-Chloro-7-(1-(3-phenylpropyl)-1H-pyrazol-4-yl)-1,5-naphthyridine was obtained as a white solid in the same manner as in Example 0322 except that 1-(3-phenylpropyl)-4-(4,4,5,5-tetramethyl-1,3,2-dioxaborolan-2-yl)-1H-pyrazole was used instead of the 1-(3-(pyrrolidin-1-yl)propyl)-4-(4,4,5,5-tetramethyl-1,3,2-dioxaborolan-2-yl)-1H-pyrazole used in Example 0322.
MSm/z(M+H):349.

<0372-3>

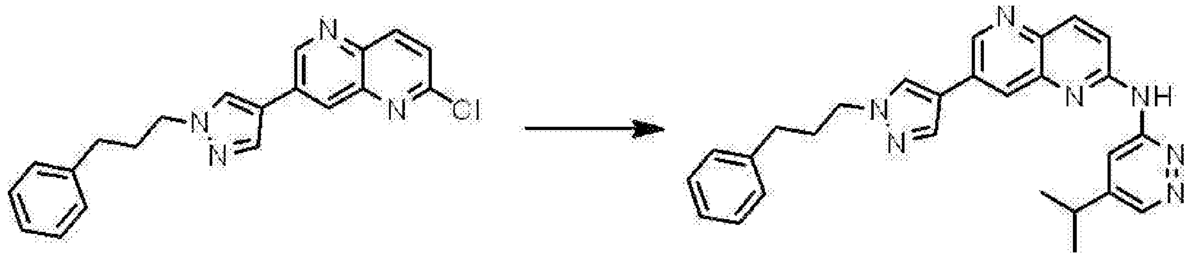

N-(5-isopropylpyridazin-3-yl)-7-(1-(3-phenylpropyl)-1H-pyrazol-4-yl)-1,5-naphthyridine-2-amine was obtained as a white solid in the same manner as in Example 0020 except that 2-chloro-7-(1-(3-phenylpropyl)-1H-pyrazol-4-yl)-1,5-naphthyridine was used instead of the 3-(4-(6-chloro-1,5-naphthyridin-3-yl)-1H-pyrazol-1-yl)-N,N-dimethylpropane-1-amine used in Example 0020.
$^1$H-NMR($CDCl_3$)δ:8.93(1H,d,J=2.0Hz),8.88-8.81(2H,m),8.25(1H,d,J=9.2Hz),8.11(1H,s),7.98(1H,s),7.81(1H,s),7.63-7.59(1H,m),7.33-7.31(2H,m),7.23-7.21(3H,m),4.23(2H,t,J=6.9Hz),3.07-3.05(1H,m),2.70(2H,t,J=7.3Hz),2.36-2.26(2H,m), 1.42(6H,d,J=7.3Hz).
MSm/z(M+H):450.

<Example 0373>

<0373-1>

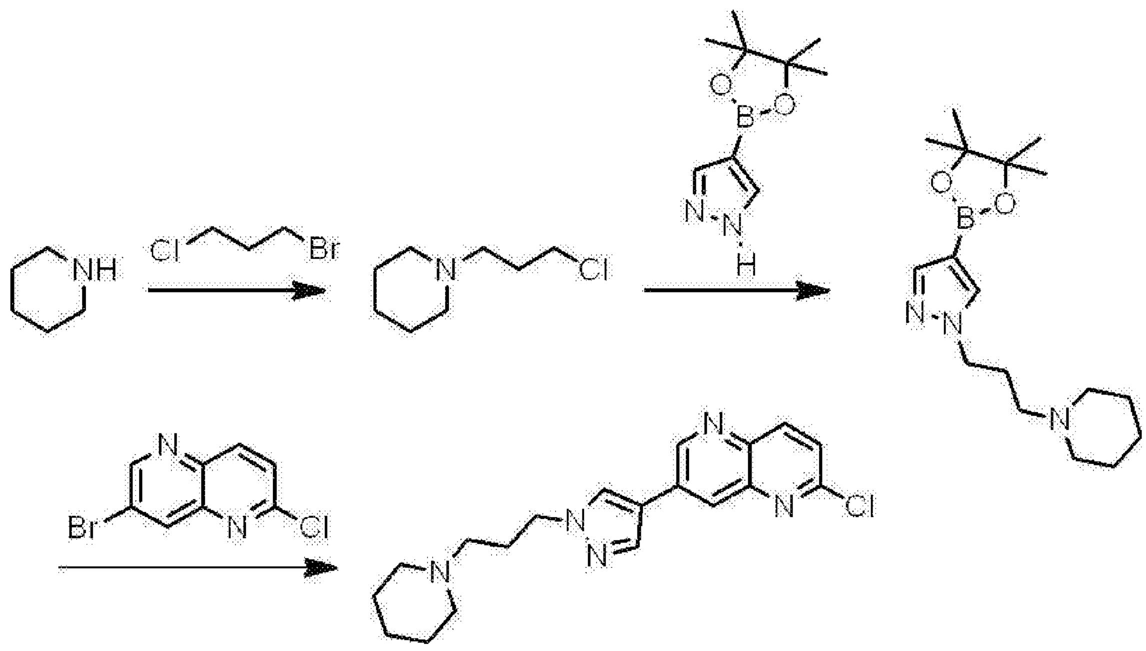

Piperidine (0.43 mL) was added to a solution of 1-bromo-3-chloropropane (0.32 mL) in toluene (1.6 mL), followed by stirring at 80°C for 2.5 hours. The reaction mixture was cooled to room temperature, ethyl acetate and water were added thereto, and 2 mol/L hydrochloric acid was added thereto. The aqueous layer was collected by separation, adjusted to pH 12 by the addition of a 2 mol/L sodium hydroxide aqueous solution, and ethyl acetate was added thereto. The organic layer was collected by separation, washed with a saturated sodium chloride aqueous solution, and dried over anhydrous sodium sulfate, and the solvent was distilled off under reduced pressure, thereby obtaining 1-(3-chloropropyl)piperidine (451 mg) as pale yellow oily substance. A mixture of the obtained 1-(3-chloropropyl)piperidine (180 mg), 4-(4,4,5,5-tetramethyl-1,3,2-dioxaborolan-2-yl)-1H-pyrazole (180 mg), cesium carbonate (610 mg), sodium iodide (28 mg), and 1,4-dioxane (1.9 mL) was stirred at 80°C overnight. The reaction mixture was cooled to room temperature, the insolubles were filtered off, and the solvent was distilled off under reduced pressure, thereby obtaining 1-(3-(4-(4,4,5,5-tetramethyl-1,3,2-dioxaborolan-2-yl)-1H-pyrazol-1-yl)propyl)piperidine.

7-Bromo-2-chloro-1,5-naphthyridine (50 mg), bis(di-tert-butyl (4-dimethylaminophenyl)phosphine)dichloropalladium(II) (14.7 mg), and sodium carbonate (44.1 mg) were added to a mixture solution of the obtained 1-(3-(4-(4,4,5,5-tetramethyl-1,3,2-dioxaborolan-2-yl)-1H-pyrazol-1-yl)propyl)piperidine (80 mg) in 1,4-dioxane (2 mL)/water (0.2 mL), followed by stirring at 100°C for 2 hours. After the reaction mixture was cooled to room temperature, the insolubles were filtered off using celite, and the filter cake was washed with ethyl acetate. The filtrate and the washings were combined, and the solvent was distilled off under reduced pressure. The obtained residue was purified by silica gel column chromatography (ethyl acetate-hexane, methanol-ethyl acetate, NH silica), thereby obtaining 2-chloro-7-(1-(3-(piperidin-1-yl)propyl)-1H-pyrazol-4-yl)-1,5-naphthyridine (45 mg)
MSm/z(M+H):356.

<0373-2>

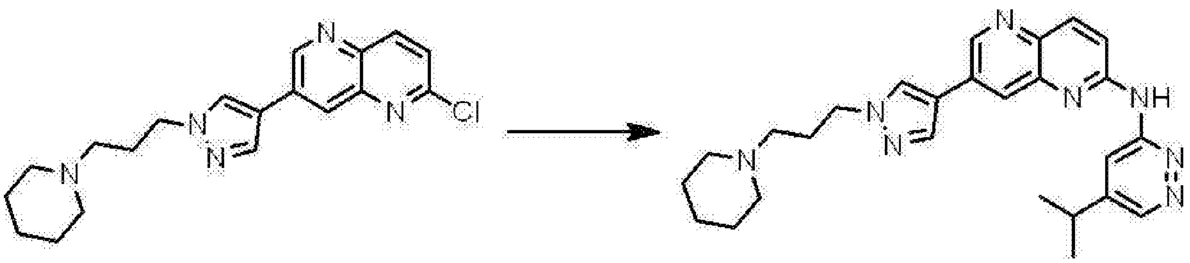

N-(5-isopropylpyridazin-3-yl)-7-(1-(3-(piperidin-1-yl)propyl)-1H-pyrazol-4-yl)-1,5-naphthyridine-2-amine was obtained as a pale yellow solid in the same manner as in Example 0372 except that 2-chloro-7-(1-(3-(piperidin-1-yl)propyl)-1H-pyrazol-4-yl)-1,5-naphthyridine was used instead of the 2-chloro-7-(1-(3-phenylpropyl)-1H-pyrazol-4-yl)-1,5-naphthyridine used in Example 0372.
$^1$H-NMR($CDCl_3$)δ:8.93(1H,d,J=2.0Hz),8.89(1H,d,J=2.0Hz),8.82(1H,d,J=2.0Hz),8.24(1H,d,J=9.2Hz),8.10 (1H,d,J=2.0Hz),7.96(1H,s),7.88(1H,s),7.58(1H,d,J=8.6Hz),4.29(2H,t,J=6.9Hz),3.11-3.02(1H,m),2.36-2.31(6H,m),2.14-2.10(2H,m),1.62-1.60(6H,m),1.43(6H,d,J=7.3Hz).
MSm/z(M+H):457.

<Example 0374>

<0374-1>

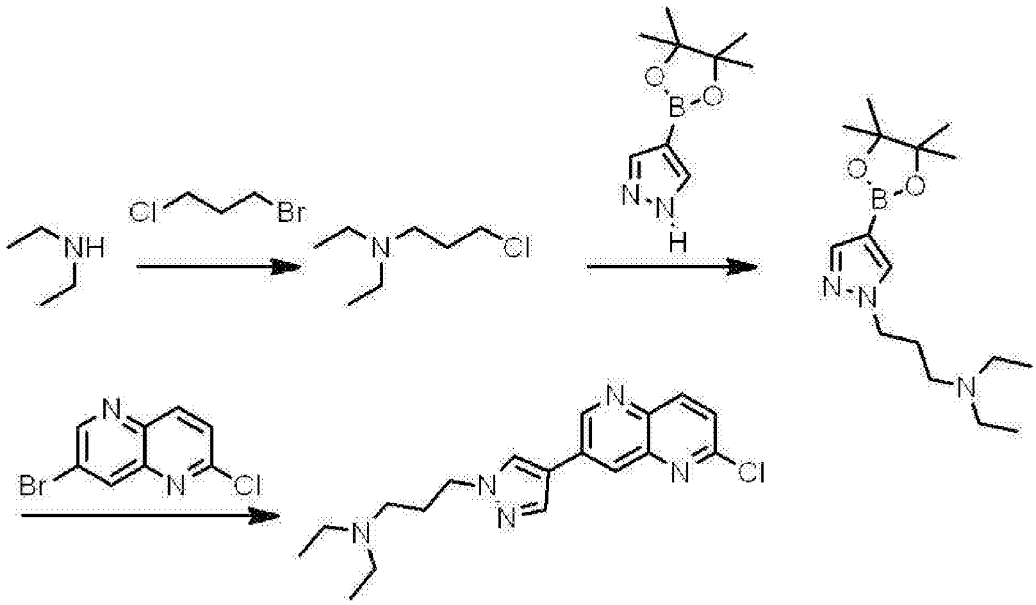

Diethylamine (0.62 mL) was added to a solution of 1-bromo-3-chloropropane (0.3 mL) in 1,4-dioxane (2 mL), followed by stirring at 50°C for 4.5 hours. The reaction mixture was cooled to room temperature, the insolubles were filtered off, and 4-(4,4,5,5-tetramethyl-1,3,2-dioxaborolan-2-yl)-1H-pyrazole (194 mg), cesium carbonate (651 mg), and sodium iodide (30 mg) were added thereto, followed by stirring at 80°C overnight. The reaction mixture was cooled to room temperature, the insolubles were filtered off, and the solvent was distilled off under reduced pressure, thereby obtaining N,N-diethyl-3-(4-(4,4,5,5-tetramethyl-1,3,2-dioxaborolan-2-yl)-1H-pyrazol-1-yl)propane-1-amine.

7-Bromo-2-chloro-1,5-naphthyridine (50 mg), bis(di-tert-butyl (4-dimethylaminophenyl)phosphine)dichloropalladium(II) (14.7 mg), and sodium carbonate (44.1 mg) were added to a mixture solution of the obtained N,N-diethyl-3-(4-(4,4,5,5-tetramethyl-1,3,2-dioxaborolan-2-yl)-1H-pyrazol-1-yl)propane-1-amine (80 mg) in 1,4-dioxane (2 mL)/water (0.2 mL), followed by stirring at 100°C for 2 hours. After the reaction mixture was cooled to room temperature, the insolubles were filtered off using celite, and the filter cake was washed with ethyl acetate. The filtrate and the washings were combined, and the solvent was distilled off under reduced pressure. The obtained residue was purified by silica gel column chromatography (ethyl acetate-hexane, NH silica), thereby obtaining 3-(4-(6-chloro-1,5-naphthyridin-3-yl)-1H-pyrazol-1-yl)-N,N-diethylpropane-1-amine (15 mg).
MSm/z(M+H):344.

<0374-2>

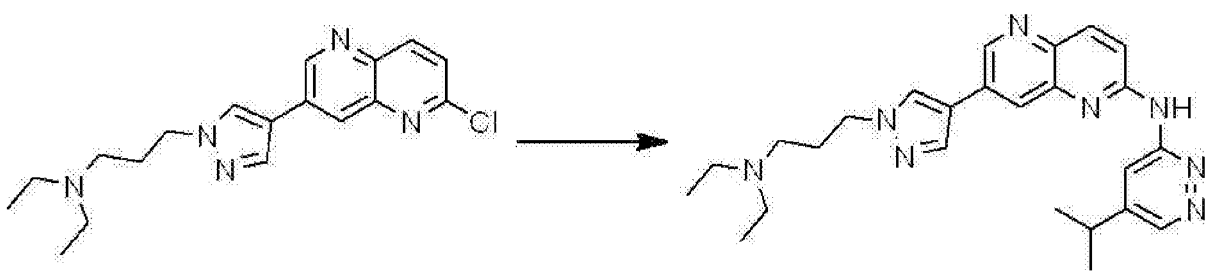

7-(1-(3-(Diethylamino)propyl)-1H-pyrazol-4-yl)-N-(5-isopropylpyridazin-3-yl)-1,5-naphthyridine-2-amine was obtained as a pale yellow solid in the same manner as in Example 0372 except that 3-(4-(6-chloro-1,5-naphthyridin-3-yl)-1H-pyrazol-1-yl)-N,N-diethylpropane-1-amine was used instead of the 2-chloro-7-(1-(3-phenylpropyl)-1H-pyrazol-4-yl)-1,5-naphthyridine used in Example 0372.
$^1$H-NMR($CDCl_3$)δ:8.93(1H,d,J=2.0Hz),8.83-8.82(2H,m),8.24(1H,d,J=9.2Hz),8.10(1H,d,J=1.3Hz),7.97(1H,s),7.89(1H,s),7.50(1H,d,J=9.2Hz),4.29( 2H,t,J=6.9Hz),3.07-3.05(1H,m),2.58-2.55(6H,m),2.15(2H,m),1.43-1.41(6H,m),1.04(6H,t,J=6.9

Hz).
MSm/z(M+H):445.

<Example 0375>

<0375-1>

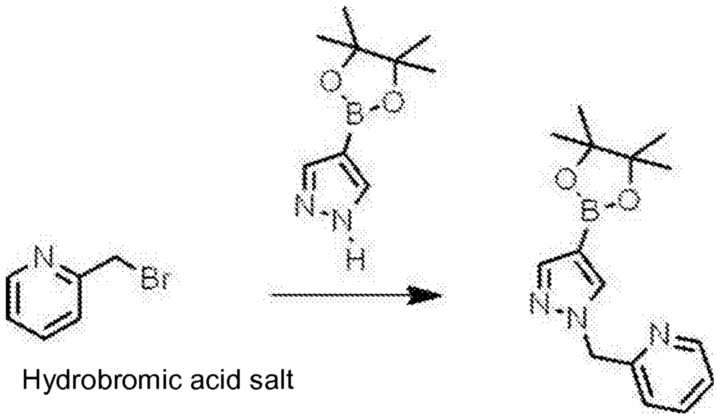


A suspension of 4-(4,4,5,5-tetramethyl-1,3,2-dioxaborolan-2-yl)-1H-pyrazole (195 mg), 2-(bromomethyl)pyridine hydrobromate (302 mg), and potassium carbonate (415 mg) in acetonitrile (2 mL) was stirred at 80°C overnight. The reaction mixture was cooled to room temperature, the insolubles were filtered off, and the solvent was distilled off under reduced pressure, thereby obtaining 2-((4-(4,4,5,5-tetramethyl-1,3,2-dioxaborolan-2-yl)-1H-pyrazol-1-yl)methyl)pyridine (284 mg) as brown oily substance.
MSm/z(M+H):286.

<0375-2>

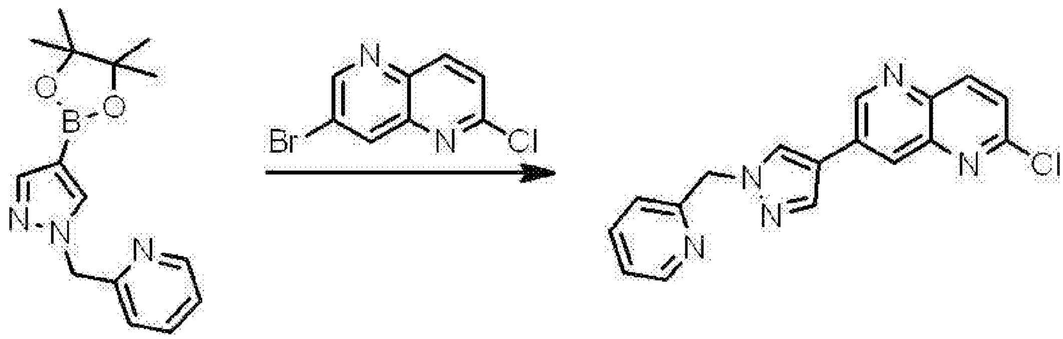

1,4-Dioxane (2 mL)/water (0.2 mL) was added to a mixture of 7-bromo-2-chloro-1,5-naphthyridine (75 mg), 2-((4-(4,4,5,5-tetramethyl-1,3,2-dioxaborolan-2-yl)-1H-pyrazol-1-yl)methyl)pyridine (105 mg), bis(di-tert-butyl (4-dimethylaminophenyl)phosphine)dichloropalladium(II) (21 mg), and sodium carbonate (65 mg), followed by stirring at 100°C for 2 hours. After the reaction mixture was cooled to room temperature, the insolubles were filtered off using celite, and the filter cake was washed with ethyl acetate. The filtrate and the washings were combined, and the solvent was distilled off under reduced pressure. The obtained residue was purified by silica gel column chromatography (ethyl acetate-hexane, methanol-ethyl acetate, NH silica), thereby obtaining 2-chloro-7-(1-(pyridin-2-ylmethyl)-1H-pyrazol-4-yl)-1,5-naphthyridine (64 mg) as a yellow solid.
MSm/z(M+H):322.

<0375-3>

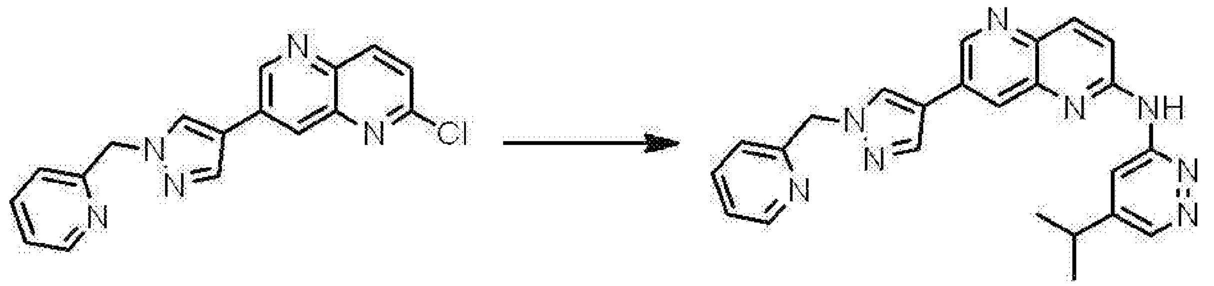

N-(5-isopropylpyridazin-3-yl)-7-(1-(pyridin-2-ylmethyl)-1H-pyrazol-4-yl)-1,5-naphthyridine-2-amine was obtained as a yellow solid in the same manner as in Example 0372 except that 2-chloro-7-(1-(pyridin-2-ylmethyl)-1H-pyrazol-4-yl)-1,5-naphthyridine was used instead of the 2-chloro-7-(1-(3-phenylpropyl)-1H-pyrazol-4-yl)-1,5-naphthyridine used in Example 0372.
$^1$H-NMR($CDCl_3$)$\delta$:8.94(1H,s),8.83-8.80(2H,m),8.63(2H,d,J=4.6Hz),8.24(1H,d,J=9.9Hz),8.13(1H,s),8.04(3H,m),7.72-7.70(1H,m),7.22(1H,d,J=7.9Hz),5.56-5.52(2H,m),3.07-3.05(1H,m),1.42(6H,d,J=6.6Hz).
MSm/z(M+H):423.

<Example 0376>

<0376-1>

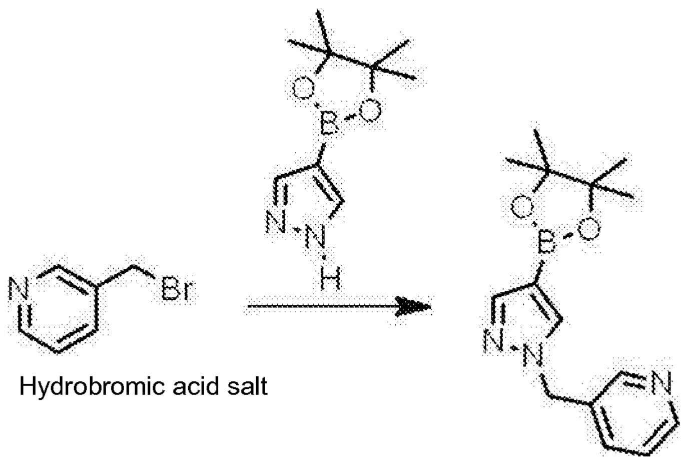


3-((4-(4,4,5,5-Tetramethyl-1,3,2-dioxaborolan-2-yl)-1H-pyrazol-1-yl)methyl)pyridine was obtained as an orange solid in the same manner as in Example 0375 except that 3-(bromomethyl)pyridine hydrobromate was used instead of the 2-(bromomethyl)pyridine hydrobromate used in Example 0375.
MSm/z(M+H):286.

<0376-2>

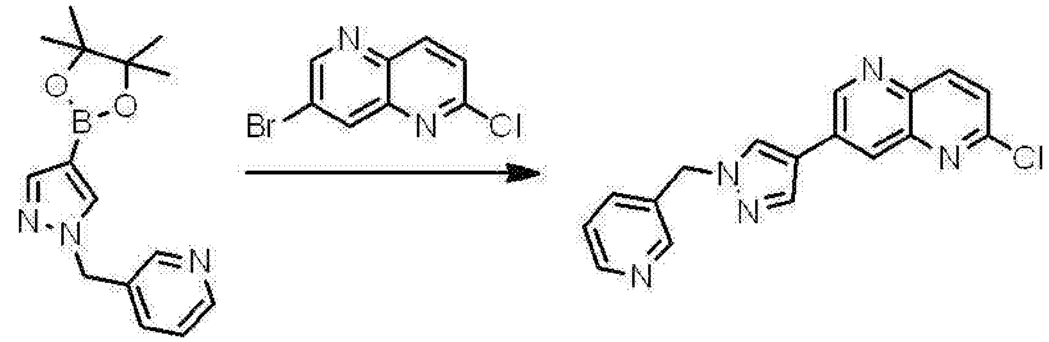

2-Chloro-7-(1-(pyridin-3-ylmethyl)-1H-pyrazol-4-yl)-1,5-naphthyridine was obtained as a pale yellow solid in the same manner as in Example 0375 except that 3-((4-(4,4,5,5-tetramethyl-1,3,2-dioxaborolan-2-yl)-1H-pyrazol-1-yl)methyl)pyridine was used instead of the 2-((4-(4,4,5,5-tetramethyl-1,3,2-dioxaborolan-2-yl)-1H-pyrazol-1-yl)methyl)pyridine used in Example 0375.
MSm/z(M+H):322.

<0376-3>

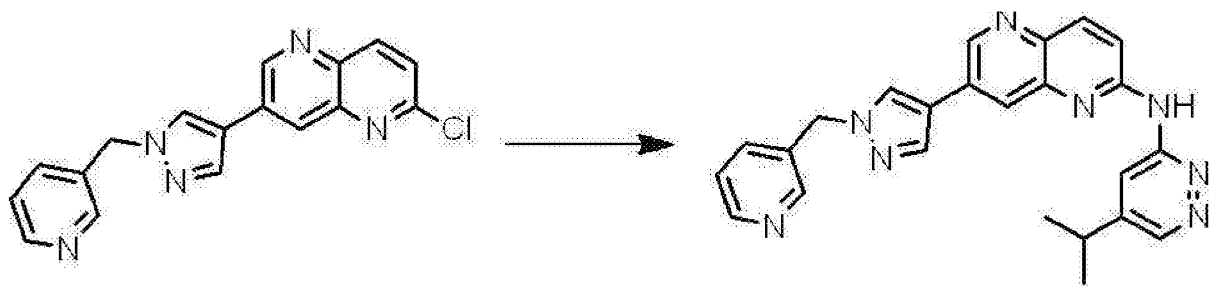

N-(5-isopropylpyridazin-3-yl)-7-(1-(pyridin-3-ylmethyl)-1H-pyrazol-4-yl)-1,5-naphthyridine-2-amine was obtained as a pale yellow solid in the same manner as in Example 0375 except that 2-chloro-7-(1-(pyridin-3-ylmethyl)-1H-pyrazol-4-yl)-1,5-naphthyridine was used instead of the 2-chloro-7-(1-(pyridin-2-ylmethyl)-1H-pyrazol-4-yl)-1,5-naphthyridine used in Example 0375.
$^1$H-NMR($CDCl_3$)δ:8.91(1H,d,J=2.0Hz),8.79(1H,s),8.64-8.62(2H,m),8.53-8.52(1H,m),8.24(1H,d,J=9.2Hz),8.10(1H,d,J=2.0Hz),8.02(1H,s),7.88(1H,s),7.65-7.58(2H,m),7.35-7.32(1H,m),5.44(2H,s),3.06-3.04(1H,m),1.41(6H,d,J=6.6Hz).
MSm/z(M+H):423.

<Example 0377>

<0377-1>

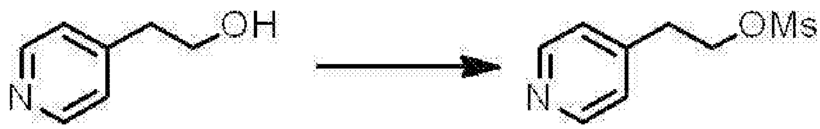

Triethylamine (1 mL) was added to a solution of 4-pyridineethanol (0.28 mL) in tetrahydrofuran (5.8 mL), and methanesulfonyl chloride (0.28 mL) was added thereto at 0°C, followed by stirring at room temperature for 1.5 hours. The insolubles were filtered off using celite, and the filter cake was washed with tetrahydrofuran. The filtrate and the washings were combined, and the solvent was distilled off under reduced pressure, thereby obtaining 2-(pyridin-4-yl)ethyl methanesulfonate (517 mg) as an orange solid.
MSm/z(M+H):202.

<0377-2>

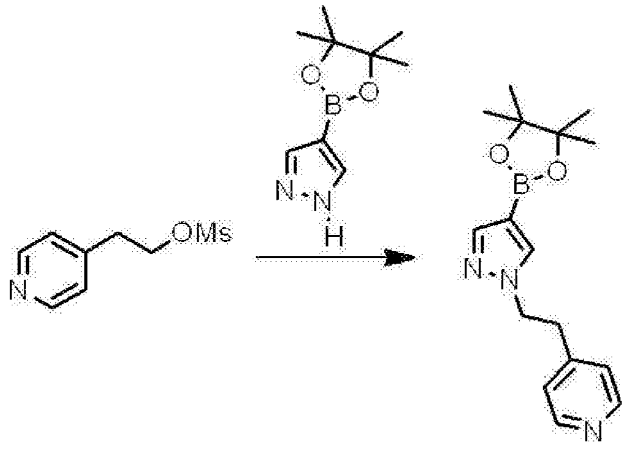

4-(2-(4-(4,4,5,5-Tetramethyl-1,3,2-dioxaborolan-2-yl)-1H-pyrazol-1-yl)ethyl)pyridine was obtained as colorless oily substance in the same manner as in Example 0372 except that 2-(pyridin-4-yl)ethyl methanesulfonate was used instead of the 3-phenylpropyl bromide used in Example 0372.
MSm/z(M+H):300.

<0377-3>

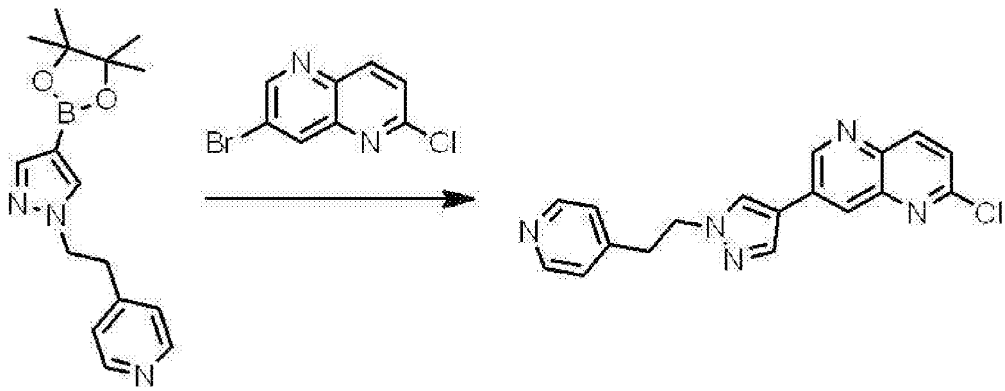

2-Chloro-7-(1-(2-(pyridin-4-yl)ethyl)-1H-pyrazol-4-yl)-1,5-naphthyridine was obtained as a white solid in the same manner as in Example 0375 except that 4-(2-(4-(4,4,5,5-tetramethyl-1,3,2-dioxaborolan-2-yl)-1H-pyrazol-1-yl)ethyl)pyridine was used instead of the 2-((4-(4,4,5,5-tetramethyl-1,3,2-dioxaborolan-2-yl)-1H-pyrazol-1-yl)methyl)pyridine used in Example 0375.
MSm/z(M+H):336.

<0377-4>

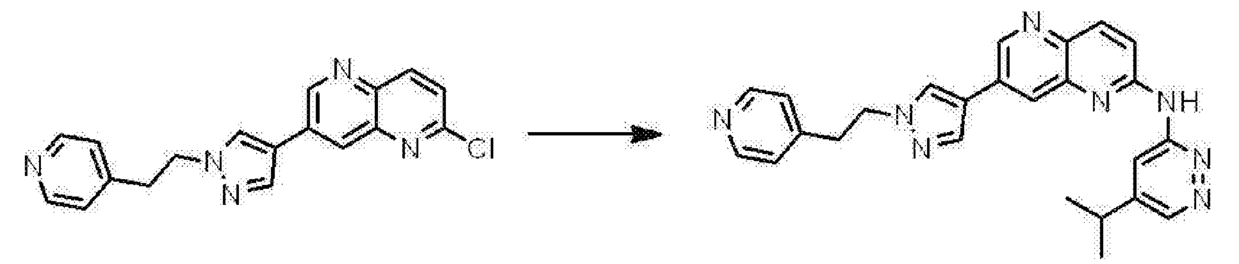

N-(5-isopropylpyridazin-3-yl)-7-(1-(2-(pyridin-4-yl)ethyl)-1H-pyrazol-4-yl)-1,5-naphthyridine-2-amine was obtained as a pale yellow solid in the same manner as in Example 0372 except that 2-chloro-7-(1-(2-(pyridin-4-yl)ethyl)-1H-pyrazol-4-yl)-1,5-naphthyridine was used instead of the 2-chloro-7-(1-(pyridin-2-ylmethyl)-1H-pyrazol-4-yl)-1,5-naphthyridine used in Example 0372.
$^1$H-NMR($CDCl_3$)δ:9.44(1H,s),8.88(2H,dd,J=9.2,2.0Hz),8.83(1H,d,J=2.0Hz),8.54-8.50(2H,m),8.24(1H,d,J=9.2Hz),8.06(1H,d,J=2.0Hz),8.00(1H,s),7.66-7.63(2H,m),7.07-7.06(2H,m),4.47(2H,t,J=6.9Hz),3.28(2H,t,J=6.9Hz),3.11-3.02(1H,m),1.43(6H,d,J=7.3Hz).
MSm/z(M+H):437.

<Example 0378>

<0378-1>

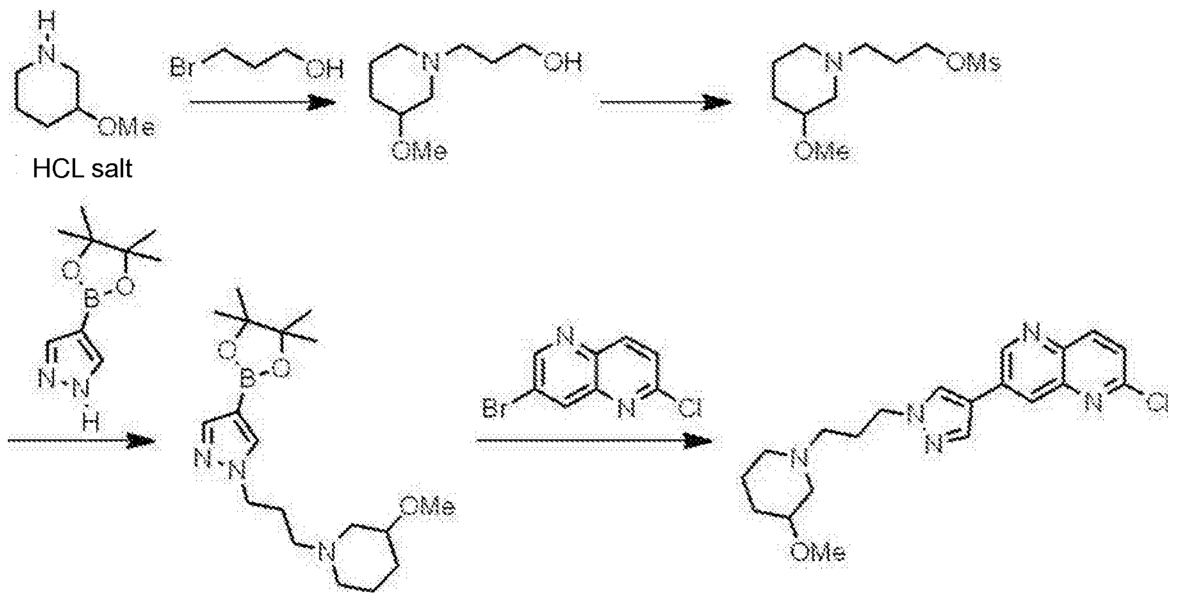


Potassium carbonate (0.82 g) and 3-bromo-1-propanol (0.27 mL) were added to a suspension of 3-methoxypiperidine hydrochloride in acetonitrile (4 mL), followed by stirring at 80°C for 15 hours. The reaction mixture was cooled to room temperature, the insolubles were filtered off, and the solvent was distilled off under reduced pressure, thereby obtaining 3-(3-methoxypiperidin-1-yl)propan-1-ol (400 mg) as brown oily substance.
Triethylamine (0.97 mL) was added to a suspension of the obtained 3-(3-methoxypiperidin-1-yl)propan-1-ol (400 mg) in tetrahydrofuran (5.5 mL), and methanesulfonyl chloride (0.27 mL) was added thereto at 0°C, followed by stirring at room temperature for 2 hours. The insolubles were filtered off using celite, and the solvent was distilled off under reduced pressure, thereby obtaining 3-(3-methoxypiperidin-1-yl)propyl methanesulfonate (668 mg) as brown oily substance.
4-(4,4,5,5-Tetramethyl-1,3,2-dioxaborolan-2-yl)-1H-pyrazole (195 mg) and potassium carbonate (279 mg) were added to a solution of the obtained 3-(3-methoxypiperidin-1-yl)propyl methanesulfonate (375 mg) in acetonitrile (2 mL), followed by stirring at 80°C for 17.5 hours. The reaction mixture was cooled to room temperature, the insolubles were

filtered off, and the solvent was distilled off under reduced pressure, thereby obtaining 3-methoxy-1-(3-(4-(4,4,5,5-tetramethyl-1,3,2-dioxaborolan-2-yl)-1H-pyrazol-1-yl)propyl)piperidine (393 mg) as brown oily substance.

7-Bromo-2-chloro-1,5-naphthyridine (50 mg), bis(di-tert-butyl (4-dimethylaminophenyl)phosphine)dichloropalladium(II) (15 mg), and sodium carbonate (44 mg) were added to a mixture solution of the obtained 3-methoxy-1-(3-(4-(4,4,5,5-tetramethyl-1,3,2-dioxaborolan-2-yl)-1H-pyrazol-1-yl)propyl)piperidine (181 mg) in 1,4-dioxane (2.1 mL)/water (0.21 mL), followed by stirring at 100°C for 2 hours. After the reaction mixture was cooled to room temperature, the insolubles were filtered off using celite, and the filter cake was washed with ethyl acetate. The filtrate and the washings were combined, and the solvent was distilled off under reduced pressure. The obtained residue was purified by silica gel column chromatography (ethyl acetate-hexane, methanol-ethyl acetate, NH silica), thereby obtaining 2-chloro-7-(1-(3-(3-methoxypiperidin-1-yl)propyl)-1H-pyrazol-4-yl)-1,5-naphthyridine (23 mg) as a white solid.
MSm/z(M+H):386.

<0378-2>

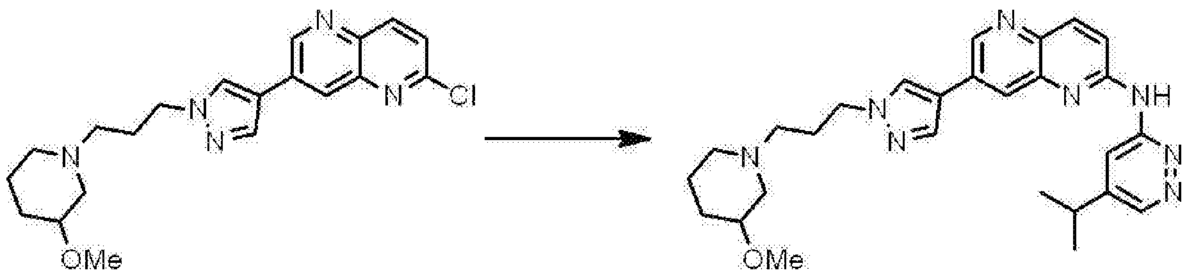

N-(5-isopropylpyridazin-3-yl)-7-(1-(3-(3-methoxypiperidin-1-yl)propyl)-1H-pyrazol-4-yl)-1,5-naphthyridine-2-amine was obtained as a pale yellow solid in the same manner as in Example 0375 except that 2-chloro-7-(1-(3-(3-methoxypiperidin-1-yl)propyl)-1H-pyrazol-4-yl)-1,5-naphthyridine was used instead of the 2-chloro-7-(1-(pyridin-2-ylmethyl)-1H-pyrazol-4-yl)-1,5-naphthyridine used in Example 0375.
$^1$H-NMR($CDCl_3$)δ:9.48(1H,s),8.93(2H,s),8.83-8.83(1H,m),8.25(1H,d,J=9.2Hz),8.11-8.10(1H,m),7.93(2H,d,J=20.5Hz),7.67(1H,d,J=9.2Hz),4.29(2H,t,J=6.9Hz),3.37(3H,s),3.35-3.29(1H,m),3.12-3.03(1H,m),2.86-2.82(1H,m),2.59-2.57(1H,m),2.38(2H,t,J=6.9Hz),2.25-1.74(8H,m),1.43(6H,d,J=7.3Hz).
MSm/z(M+H):487.

<Example 0379>

<0379-1>

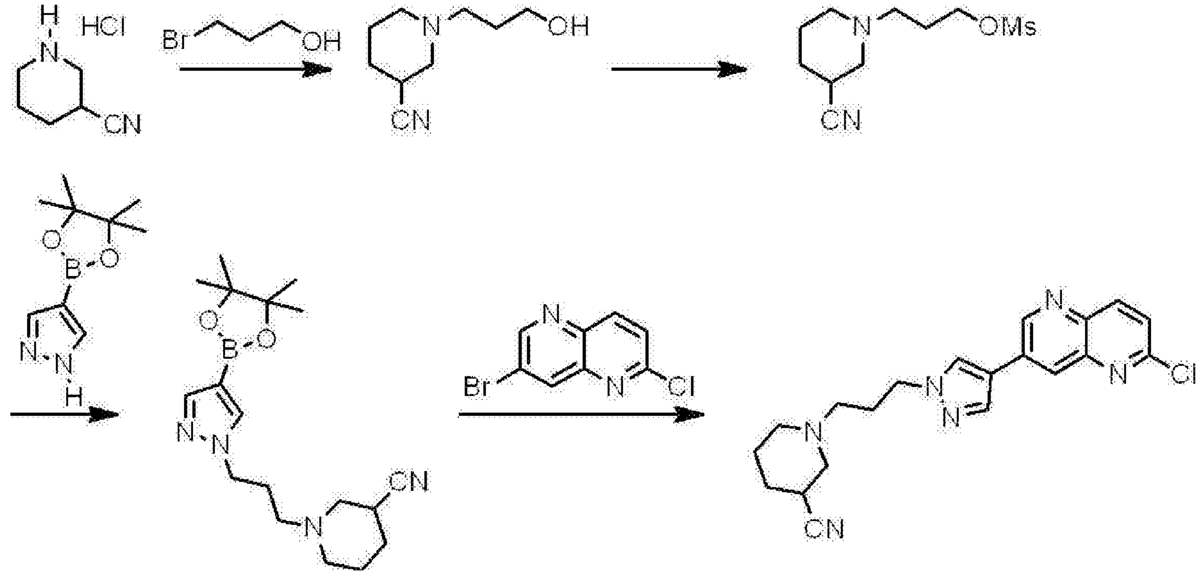

1-(3-(4-(6-Chloro-1,5-naphthyridin-3-yl)-1H-pyrazol-1-yl)propyl)piperidine-3-carbonitrile was obtained as colorless oily substance in the same manner as in Example 0378 except that 3-cyanopiperidine hydrochloride was used instead of the 3-methoxypiperidine hydrochloride used in Example 0378.
MSm/z(M+H):381.

<0379-2>

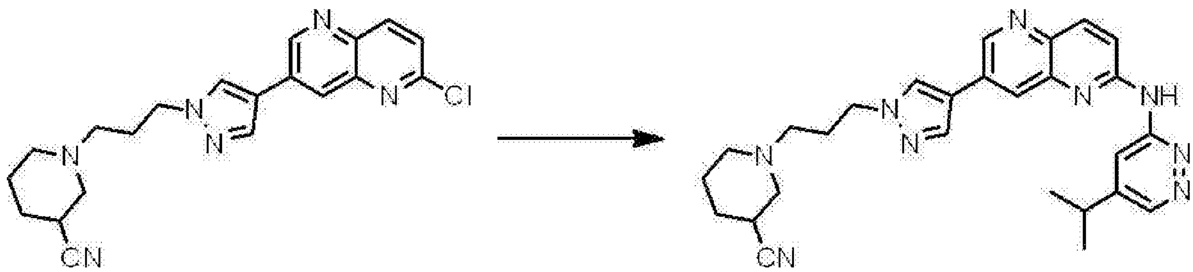

1-(3-(4-(6-((5-Isopropylpyridazin-3-yl)amino)-1,5-naphthyridin-3-yl)-1H-pyrazol-1-yl)propyl)piperidine-3-carbonitrile was obtained as a pale yellow solid in the same manner as in Example 0375 except that 1-(3-(4-(6-chloro-1,5-naphthyridin-3-yl)-1H-pyrazol-1-yl)propyl)piperidine-3-carbonitrile was used instead of the 2-chloro-7-(1-(pyridin-2-ylmethyl)-1H-pyrazol-4-yl)-1,5-naphthyridine used in Example 0375.
$^{1}$H-NMR($CDCl_3$)δ:8.97(1H,s),8.83-8.81(2H,m),8.26-8.23(1H,m),8.15(1H,s),8.04(1H,s),7.99(1H,s),7.45-7.42(1H,m),4.36-4.32(2H,m),3.07-3.05(1H,m),2.87-2.83(1H,m),2.67-1.64(12H,m),1.42(6H,d,J=6.6Hz).
MSm/z(M+H):482.

<Example 0380>

<0380-1>

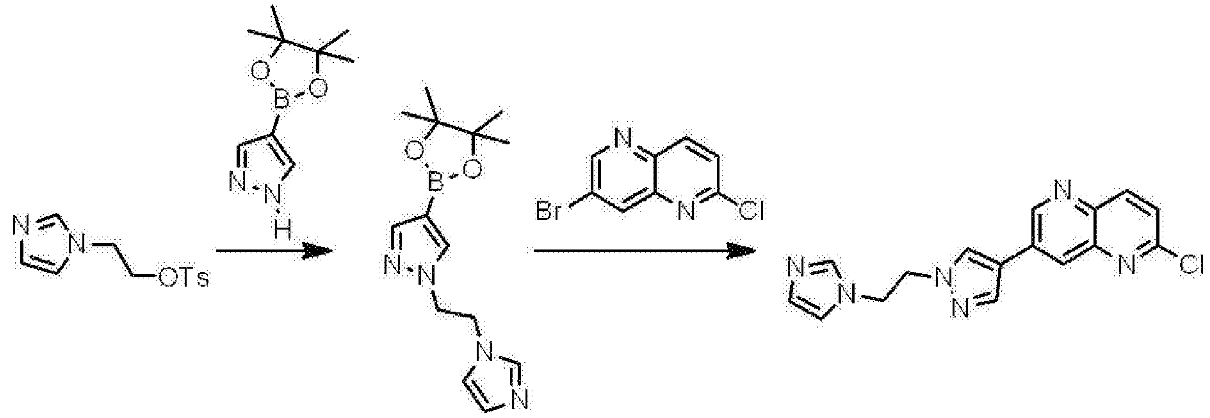

4-(4,4,5,5-Tetramethyl-1,3,2-dioxaborolan-2-yl)-1H-pyrazole (194 mg) and potassium carbonate (276 mg) were added to a solution of 2-(1H-imidazol-1-yl)ethyl 4-methylbenzenesulfonate (430 mg) in acetonitrile (2 mL), followed by stirring at 80°C for 17.5 hours. The reaction mixture was cooled to room temperature, and the insolubles were filtered off. The filtrate and the washings were combined, and the solvent was distilled off under reduced pressure, thereby obtaining 1-(2-(1H-imidazol-1-yl)ethyl)-4-(4,4,5,5-tetramethyl-1,3,2-dioxaborolan-2-yl)-1H-pyrazole (341 mg) as brown oily substance.

7-Bromo-2-chloro-1,5-naphthyridine (50 mg), bis(di-tert-butyl (4-dimethylaminophenyl)phosphine)dichloropalladium(II) (14 mg) and sodium carbonate (45 mg) were added to a mixture solution of the obtained 1-(2-(1H-imidazol-1-yl)ethyl)-4-(4,4,5,5-tetramethyl-1,3,2-dioxaborolan-2-yl)-1H-pyrazole (152 mg) in 1,4-dioxane (2.1 mL)/water (0.21 mL), followed by stirring at 100°C for 2 hours. After the reaction mixture was cooled to room temperature, the insolubles were filtered off using celite, and the filter cake was washed with ethyl acetate. The filtrate and the washings were combined, and the solvent was distilled off under reduced pressure. The obtained residue was purified by silica gel column chromatography (ethyl acetate-hexane, methanol-ethyl acetate, NH silica), thereby obtaining 7-(1-(2-(1H-imidazol-1-yl)ethyl)-1H-pyrazol-4-yl)-2-chloro-1,5-naphthyridine (20 mg) as a white solid.
MSm/z(M+H):325.

<0380-2>

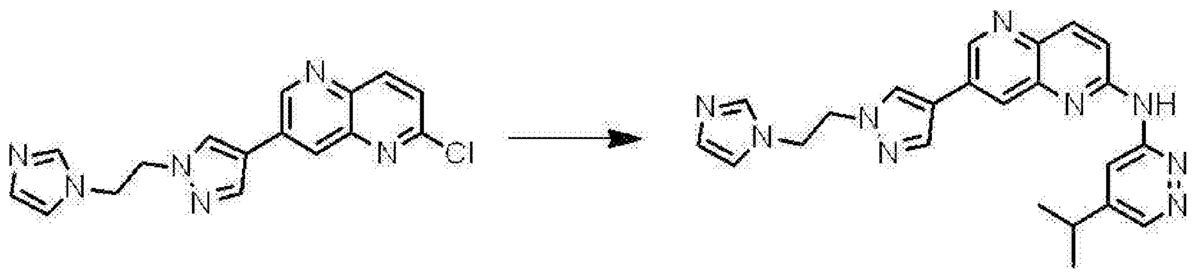

7-(1-(2-(1H-imidazol-1-yl)ethyl)-1H-pyrazol-4-yl)-N-(5-isopropylpyridazin-3-yl)-1,5-naphthyridine-2-amine was obtained as a brown solid in the same manner as in Example 0375 except that 7-(1-(2-(1H-imidazol-1-yl)ethyl)-1H-pyrazol-4-yl)-2-chloro-1,5-naphthyridine was used instead of the 2-chloro-7-(1-(pyridin-2-ylmethyl)-1H-pyrazol-4-yl)-1,5-naphthyridine used in Example 0375. $^1$H-NMR(CDCl$_3$)δ:8.84-8.82(3H,m),8.24(1H,d,J=8.6Hz),8.05-8.04(2H,m),7.55(1H,d,J=9.2Hz),7.49(1H,s),7.29(1H,s),7.04(1H,s),6.73(1H,s),4.55-4.49(4H,m),3.08-3.06(1H,m),1.44-1.42(6H,d).
MSm/z(M+H):426.

<Example 0381>

<0381-1>

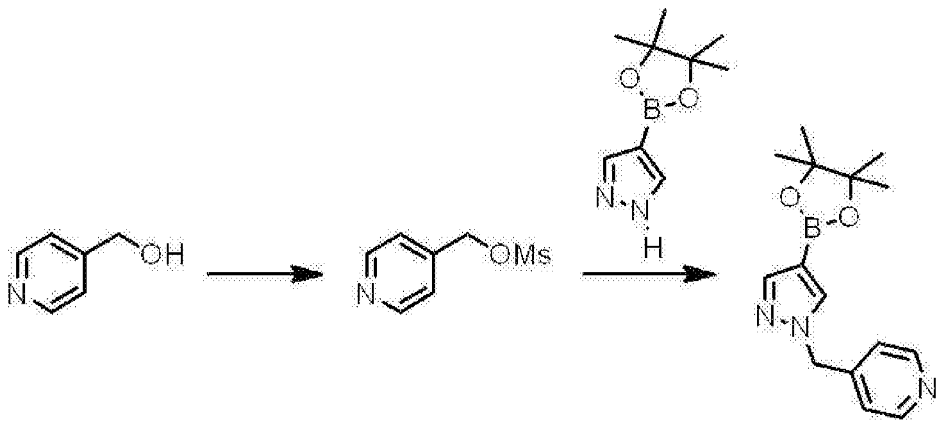

Triethylamine (0.15 mL) was added to a solution of 4-pyridinemethanol (102 mg) in tetrahydrofuran (2.2 mL), and methanesulfonyl chloride (0.08 mL) was added thereto at 0°C, followed by stirring at room temperature for 2 hours. The insolubles were filtered off using celite, and the filter cake was washed with tetrahydrofuran (5 mL). The filtrate and the washings were combined, thereby obtaining (pyridin-4-yl)methyl methanesulfonate as a yellow solution.

Acetonitrile (2 mL), 4-(4,4,5,5-tetramethyl-1,3,2-dioxaborolan-2-yl)-1H-pyrazole (148 mg), and potassium carbonate (211 mg) were added to the obtained yellow solution, followed by stirring at 80°C overnight. The reaction mixture was cooled to room temperature, the insolubles were filtered off, and the solvent was distilled off under reduced pressure. The obtained residue was purified by silica gel column chromatography (ethyl acetate-hexane, methanol-ethyl acetate), thereby obtaining 4-((4-(4,4,5,5-tetramethyl-1,3,2-dioxaborolan-2-yl)-1H-pyrazol-1-yl)methyl)pyridine (29 mg).
MSm/z(M+H):286.

<0381-2>

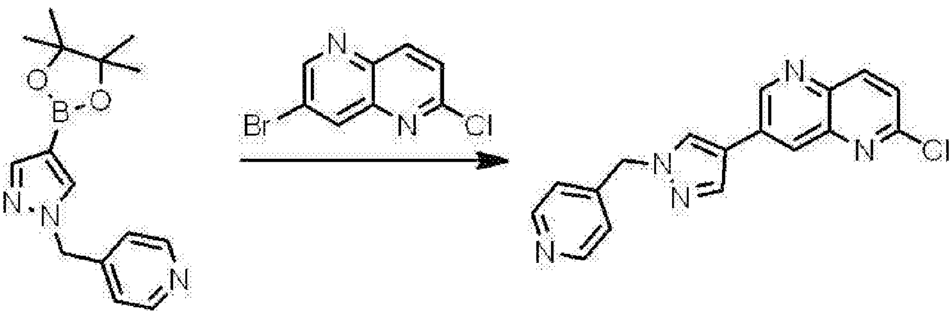

2-Chloro-7-(1-(pyridin-4-ylinethyl)-1H-pyrazol-4-yl)-1,5-naphthyridine (11 mg) was obtained as a pale yellow solid in the same manner as in Example 0375 except that 4-((4-(4,4,5,5-tetramethyl-1,3,2-dioxaborolan-2-yl)-1H-pyrazol-1-yl)methyl)pyridine was used instead of the 2-((4-(4,4,5,5-tetramethyl-1,3,2-dioxaborolan-2-yl)-1H-pyrazol-1-yl)methyl)pyridine used in Example 0375.
MSm/z(M+H):322.

<0381-3>

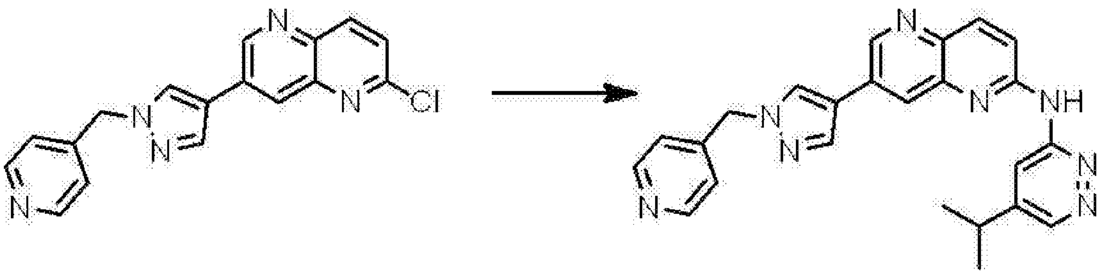

N-(5-isopropylpyridazin-3-yl)-7-(1-(pyridin-4-ylmethyl)-1H-pyrazol-4-yl)-1,5-naphthyridine-2-amine was obtained as a yellow solid in the same manner as in Example 0375 except that 2-chloro-7-(1-(pyridin-4-ylmethyl)-1H-pyrazol-4-yl)-1,5-naphthyridine was used instead of the 2-chloro-7-(1-(pyridin-2-ylmethyl)-1H-pyrazol-4-yl)-1,5-naphthyridine used in Example 0375. $^1$H-NMR($CDCl_3$)δ:8.94(1H,s),8.83-8.80(2H,m),8.63(2H,d,J=5.3Hz),8.27-8.24(1H,m),8.09(2H,d,J=18.5Hz),7.92(1H,s),7.54 (1H,d,J=8.6Hz),7.15(2H,d,J=5.3Hz),5.44(2H,s),3.06 -3.04(1H,m), 1.41(6H,d,J=7.3Hz).
MSm/z(M+H):423.

<Example 0382>

<0382-1>

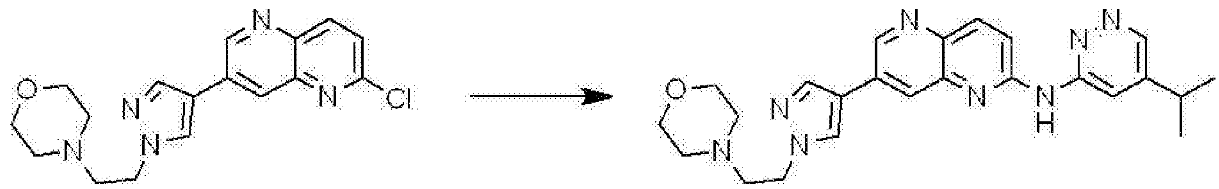

N-(5-isopropylpyridazin-3-yl)-7-(1-(2-morpholinoethyl)-1H-pyrazol-4-yl)-1,5-naphthyridine-2-amine was obtained as a white solid in the same manner as in Example 0001 except that 5-isopropylpyridazine-3-amine was used instead of the 1,3,4-thiadiazole-2-amine used in Example 0001, and 4-(2-(4-(6-chloro-1,5-naphthyridin-3-yl)-1H-pyrazol-1-yl)ethyl)morpholine was used instead of the 4-(3-(4-(6-chloro-1,5-naphthyridin-3-yl)-1H-pyrazol-1-yl)propyl)morpholine used in Example 0001.
$^1$H-NMR($CDCl_3$)δ:9.40(1H,brs),8.93(1H,d,J=2.1Hz),8.91(1H,d,J=2.1Hz),8.83(1H,d,J=1.8Hz),8.25(1H,d,J =8.7Hz),8.11(1H,d,J=2.4Hz),7.96(2H,s),7.66(1H,d,J=8.7Hz),4.35(2H,t,J=6.6Hz),3.73(4H,m),3.07(1H, m),2.89(2H,t,J=6.6Hz),2.54(4H,m),1.43(6H,d,J=6.6Hz).
MSm/z(M+H):445.

<Example 0383>

<0383-1>

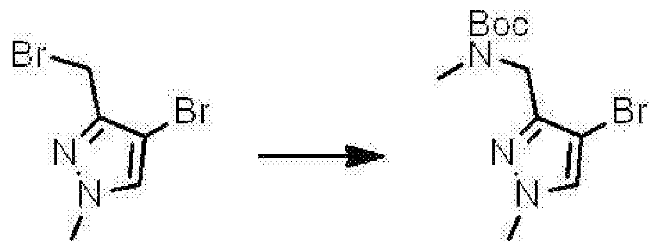

60% sodium hydride (47 mg) was added to a solution of 4-bromo-3-(bromomethyl)-1-methyl-1H-pyrazole (200 mg) and tert-butyl N-methylcarbamate (155 mg) in N-methylpyrrolidone (4 mL) under ice-cooling, followed by stirring at

the same temperature for 1 hour, and stirring at room temperature for 2 hours. Ethyl acetate and water were added to the reaction mixture. The organic layer was collected by separation, washed with water and a saturated sodium chloride aqueous solution, and dried over anhydrous sodium sulfate, and the solvent was distilled off under reduced pressure. The obtained residue was purified by silica gel column chromatography (methanol-ethyl acetate-hexane), thereby obtaining tert-butyl ((4-bromo-1-methyl-1H-pyrazol-3-yl)methyl) (methyl)carbamate (60 mg).
MSm/z(M+H):304,306.

<0383-2>

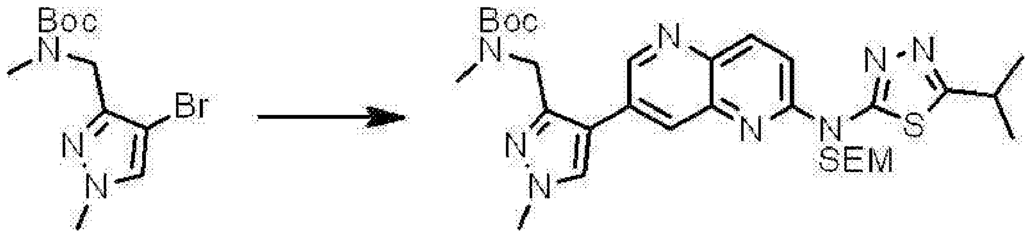

A mixture of N-(7-bromo-1,5-naphthyridin-2-yl)-5-isopropyl-N-((2-(trimethylsilyl)ethoxy)methyl)-1,3,4-thiadiazole-2-amine (20 mg), bis(pinacolato)diboron (157 mg), (1,1'-bis(diphenylphosphino)ferrocene)palladium(II) dichloride (3 mg), potassium acetate (8 mg), and 1,4-dioxane (1 mL) was stirred at 100°C for 2 hours in a nitrogen atmosphere. tert-Butyl ((4-bromo-1-methyl-1H-pyrazol-3-yl)methyl) (methyl)carbamate (13 mg), sodium carbonate (8 mg), bis(di-tert-butyl (4-dimethylaminophenyl)phosphine)dichloropalladium(II) (5 mg), and water (0.1 mL) were added to the reaction mixture, followed by stirring at 100°C for 4 hours. The reaction mixture was cooled to room temperature, the insolubles were filtered off using celite, and the solvent was distilled off under reduced pressure. The obtained residue was purified by silica gel column chromatography (methanol-ethyl acetate, NH silica), thereby obtaining tert-butyl ((4-(6-((5-isopropyl-1,3,4-thiadiazol-2-yl)((2-(trimethylsilyl)ethoxy)methyl)amino)-1,5-naphthyridin-3-yl)-1-methyl-1H-pyrazol-3-yl)methyl) (methyl)carbamate (4.3 mg).
MSm/z(M+H):625.

<0383-3>

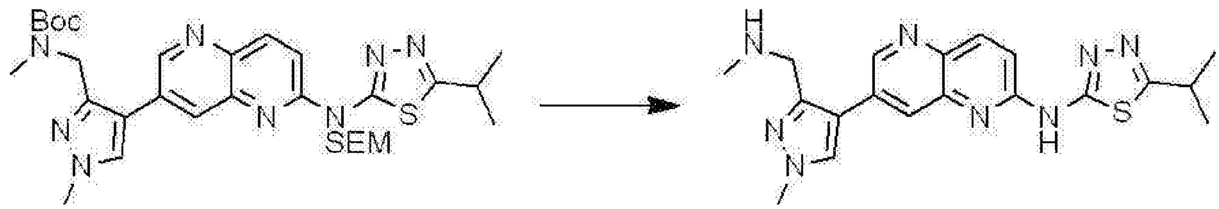

Water (0.2 mL) and trifluoroacetic acid (2 mL) were added to tert-butyl ((4-(6-((5-isopropyl-1,3,4-thiadiazol-2-yl)((2-(trimethylsilyl)ethoxy)methyl)amino)-1,5-naphthyridin-3-yl)-1-methyl-1H-pyrazol-3-yl)methyl) (methyl)carbamate (4.3 mg), followed by stirring at room temperature for 30 minutes. The solvent was distilled off under reduced pressure, and the obtained residue was purified by silica gel column chromatography (methanol-ethyl acetate, NH silica), thereby obtaining 5-isopropyl-N-(7-(1-methyl-3-((methylamino)methyl)-1H-pyrazol-4-yl)-1,5-naphthyridin-2-yl)-1,3,4-thiadiazole-2-amine (1.6 mg).
$^1$H-NMR($CDCl_3$/$CD_3OD$=4/1)δ:8.83(1H,d,J=2.1Hz),8.33(1H,d,J=2.1Hz),8.24(1H,d,J=8.4Hz),7.80(1H,s), 7.35(1H,d,J=8.4Hz),3.99(3H,s),3.94(2H,s),3.49-3.36(1H,m),2.54(3H,s),1.49(6H,d,J=7.2Hz).
MSm/z(M+H):395.

<Example 0384>

<0384-1>

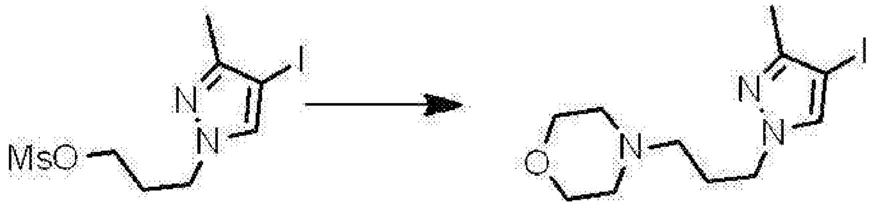

Morpholine (0.051 mL) was added to a mixture of 3-(4-iodo-3-methyl-1H-pyrazol-1-yl)propylmethanesulfonate (135 mg), potassium carbonate (108 mg), and acetonitrile (2 mL), followed by stirring at 50°C for 5 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining 4-(3-(4-iodo-3-methyl-1H-pyrazol-1-yl)propyl)morpholine (44 mg).
MSm/z(M+H):336.

<0384-2>

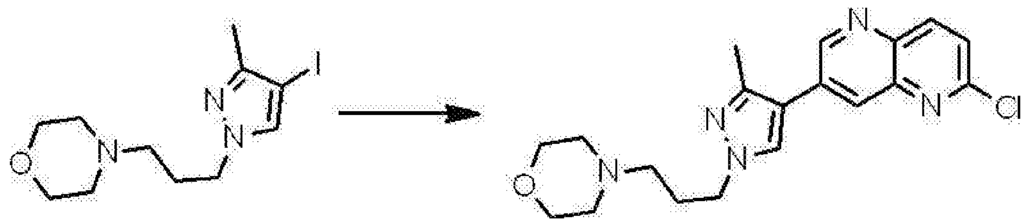

A mixture of 7-bromo-2-chloro-1,5-naphthyridine (32 mg), bis(pinacolato)diboron (50 mg), (1,1'-bis(diphenylphosphino)ferrocene)palladium(II) dichloride (11 mg), potassium acetate (26 mg), and 1,4-dioxane (1 mL) was stirred at 80°C for 2 hours in a nitrogen atmosphere. The reaction mixture was cooled to room temperature, and 4-(3-(4-iodo-3-methyl-1H-pyrazol-1-yl)propyl)morpholine (44 mg), sodium carbonate (28 mg), bis(di-tert-butyl (4-dimethylaminophenyl)phosphine)dichloropalladium(II) (9 mg), and water (0.1 mL) were added thereto, followed by stirring at 80°C for 2 hours. The reaction mixture was cooled to room temperature, the insolubles were filtered off using celite, and the solvent was distilled off under reduced pressure. The obtained residue was purified by silica gel column chromatography (methanol-ethyl acetate, NH silica), thereby obtaining 4-(3-(4-(6-chloro-1,5-naphthyridin-3-yl)-3-methyl-1H-pyrazol-1-yl)propyl)morpholine (10 mg).
MSm/z(M+H):372.

<0384-3>

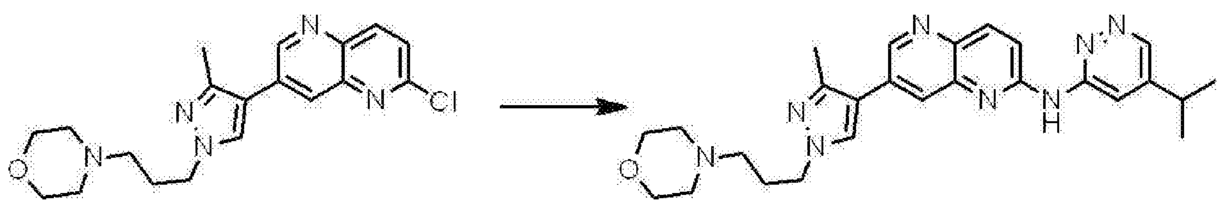

A mixture of 4-(3-(4-(6-chloro-1,5-naphthyridin-3-yl)-3-methyl-1H-pyrazol-1-yl)propyl)morpholine (10 mg), 5-isopropylpyridazine-3-amine (5.5 mg), tris(dibenzylideneacetone)dipalladium(0) (2.4 mg), 4,5-bis(diphenylphosphino)-9,9-dimethylxanthene (3.1 mg), cesium carbonate (22 mg), and 1,4-dioxane (1 mL) was stirred at 140°C for 30 minutes using a microwave reaction apparatus. The reaction mixture was cooled to room temperature, the insolubles were filtered off using celite, and purified by silica gel column chromatography (methanol-ethyl acetate, NH silica), thereby obtaining N-(5-isopropylpyridazin-3-yl)-7-(3-methyl-1-(3-morpholinopropyl)-1H-pyrazol-4-yl)-1,5-naphthyridine-2-amine (2.8 mg).
$^1$H-NMR($CDCl_3$/$CD_3OD$=4/1)δ:8.90(1H,brs),8.79(1H,d,J=1.8Hz),8.71(1H,brs),8.22(1H,d,J=8.7Hz),8.10( 1H,d,J=1.8Hz),7.78(1H,s),7.52(1H,d,J=8.7Hz),4.22(2H,t,J=6.9Hz),3.77-3.71(4H,m),3.10-2.99(1H,m),2.52-2.45(4H,m),2.51(3H,s),2.41(2H,t,J=7.2Hz),2.17-2.05(2H,m),1.41(6H,d,J=7.5Hz).
MSm/z(M+H):473.

<Example 0385>

<0385-1>

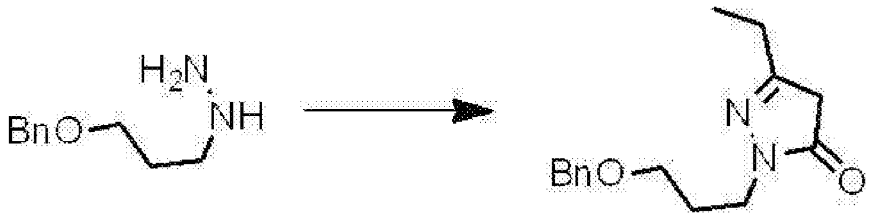

Ethyl 3-oxopentanoate (2.15 mL) was added to a solution of (3-(benzyloxy)propyl)hydrazine (2.27 g) in ethanol (12 mL), followed by stirring for 5 hours under heating to reflux. The reaction mixture was cooled to room temperature, and the solvent was distilled off under reduced pressure. The obtained residue was purified by silica gel column chromatography (ethyl acetate-hexane), thereby obtaining 1-(3-(benzyloxy)propyl)-3-ethyl-1H-pyrazol-5(4H)-one (994 mg).
MSm/z(M+H):261.

<0385-2>

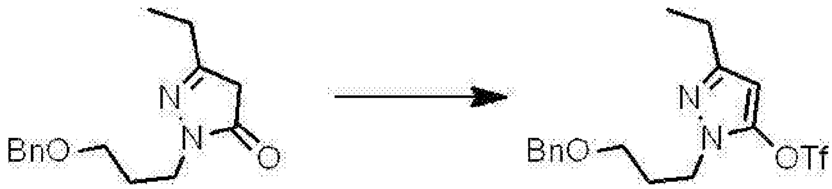

Trifluoromethanesulfonic aicd anhydride (0.937 mL) was added to a solution of 1-(3-(benzyloxy)propyl)-3-ethyl-1H-pyrazol-5(4H)-one (994 mg) and pyridine (0.553 mL) in dichloromethane (19 mL) under ice-cooling, followed by stirring at the same temperature for 1 hour. A saturated sodium hydrogen carbonate aqueous solution and dichloromethane were added to the reaction mixture. The organic layer was collected by separation, and dried over anhydrous sodium sulfate, and the solvent was distilled off under reduced pressure, thereby obtaining (1-(3-(benzyloxy)propyl)-3-ethyl-1H-pyrazol-5-yl) trifluoromethanesulfonate (1.43 g).
$^1$H-NMR($CDCl_3$)δ:7.37-2.50(5H,m),5.91(1H,s),4.49(2H,s),4.12(2H,t,J=7.2Hz),3.46(2H,t,J=6.0Hz),2.59(2H,q,J=7.2Hz),2.19-2.08(2H,m), 1.21 (3H,t,J=7.2Hz).

<0385-3>

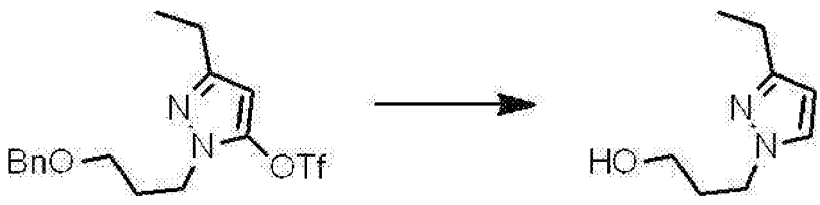

(1-(3-(Benzyloxy)propyl)-3-ethyl-1H-pyrazol-5-yl) trifluoromethanesulfonate (1.43 g) was added to a mixture of 20% palladium hydroxide-carbon (150 mg) and methanol (30 mL), followed by stirring for 5 hours in a hydrogen atmosphere. The insolubles were filtered off using celite, and the solvent was distilled off under reduced pressure, thereby obtaining 3-(3-ethyl-1H-pyrazol-1-yl)propan-1-ol (1.15 g).
$^1$H-NMR($CDCl_3$)$\delta$:7.79(1H,d,J=2.7Hz),6.42(1H,d,J=2.7Hz),4.60(2H,t,J=6.6Hz),3.73(2H,t,J=5.4Hz),2.88( 2H,q,J=8.1Hz),2.27-2.14(2H,m),1.26(3H,t,J=8.1Hz).

<0385-4>

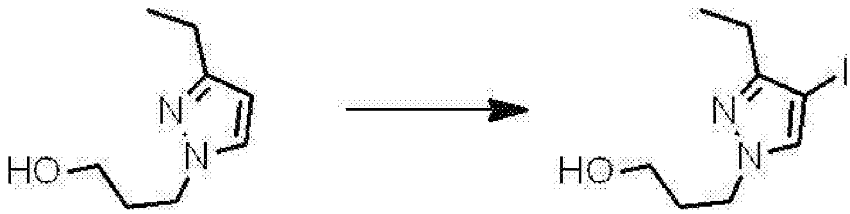

Iodine (557 mg) and ammonium cerium nitrate (1.20 g) were added to a solution of 3-(3-ethyl-1H-pyrazol-1-yl)propan-1-ol (1.15 g) in acetonitrile (8 mL), followed by stirring at room temperature for 16 hours. Ethyl acetate and water were added to the reaction mixture. The organic layer was collected by separation, washed sequentially with a 10% sodium hydrogen sulfite aqueous solution and a saturated sodium chloride aqueous solution, and dried over anhydrous sodium sulfate, and the solvent was distilled off under reduced pressure, thereby obtaining 3-(3-ethyl-4-iodo-1H-pyrazol-1-yl)propan-1-ol (821 mg).
MSm/z(M+H):281.

<0385-5>

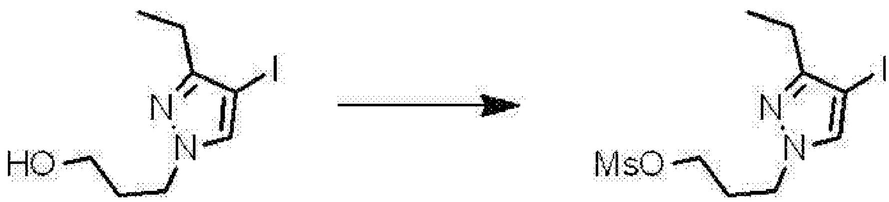

Methanesulfonyl chloride (0.342 mL) was added to a solution of 3-(3-ethyl-4-iodo-1H-pyrazol-1-yl)propan-1-ol(821 mg) and triethylamine (0.823 mL) in dichloromethane (15 mL) under ice-cooling, followed by stirring at the same temperature for 1 hour. Dichloromethane and water were added to the reaction mixture. The organic layer was collected by separation, and dried over anhydrous sodium sulfate, and the solvent was distilled off under reduced pressure, thereby obtaining 3-(3-ethyl-4-iodo-1H-pyrazol-1-yl)propyl methanesulfonate (1.00 g).
$^1$H-NMR($CDCl_3$)$\delta$:7.39(1H,s),4.20(2H,t,J=6.6Hz),4.21(2H,t,J=6.6Hz),3.03(3H,s),2.60(2H,q,J=7.8Hz),2.33-2.22(2H,m),1.22(3H,t,J=7.8Hz).

<0385-6>

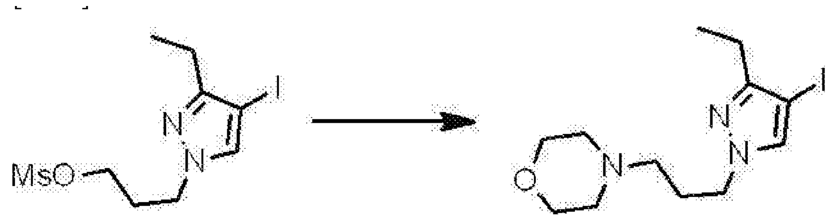

Morpholine (0.182 mL) was added to a mixture of 3-(3-ethyl-4-iodo-1H-pyrazol-1-yl)propyl methanesulfonate (500 mg), potassium carbonate (384 mg), and acetonitrile (7 mL), followed by stirring at 50°C for 5 hours. Ethyl acetate and water were added to the reaction mixture. The organic layer was collected by separation, washed with a saturated sodium chloride aqueous solution, and dried over anhydrous sodium sulfate, and the solvent was distilled off under reduced pressure, thereby obtaining 4-(3-(3-ethyl-4-iodo-1H-pyrazol-1-yl)propyl)morpholine (204 mg).
MSm/z(M+H):350.

<0385-7>

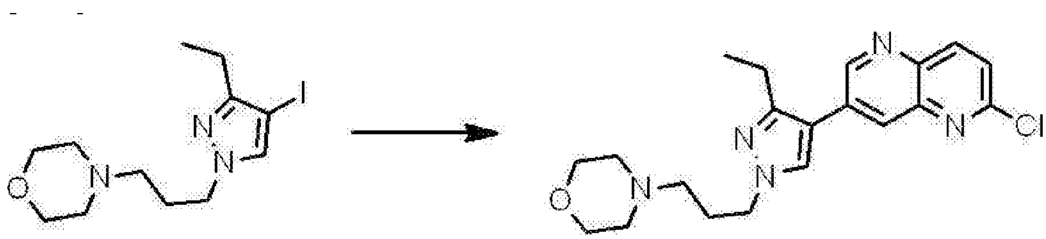

A mixture of 7-bromo-2-chloro-1,5-naphthyridine (142 mg), bis(pinacolato)diboron (223 mg), (1,1'-bis(diphenylphosphino)ferrocene)palladium(II) dichloride (47 mg), potassium acetate (115 mg), and 1,4-dioxane (3 mL) was stirred at 80°C for 2 hours in a nitrogen atmosphere. 4-(3-(3-ethyl-4-iodo-1H-pyrazol-1-yl)propyl)morpholine (204 mg), sodium carbonate (124 mg), bis(di-tert-butyl (4-dimethylaminophenyl)phosphine)dichloropalladium(II) (41 mg), and water (0.3 mL) were added thereto, followed by stirring at 80°C for 4 hours. The reaction mixture was cooled to room temperature, the insolubles were filtered off using celite, and the solvent was distilled off under reduced pressure. The obtained residue was purified by silica gel column chromatography (methanol-ethyl acetate, NH silica), thereby obtaining 4-(3-(4-(6-chloro-1,5-naphthyridin-3-yl)-3-ethyl-1H-pyrazol-1-yl)propyl)morpholine (18 mg).
MSm/z(M+H):386.

<0385-8>

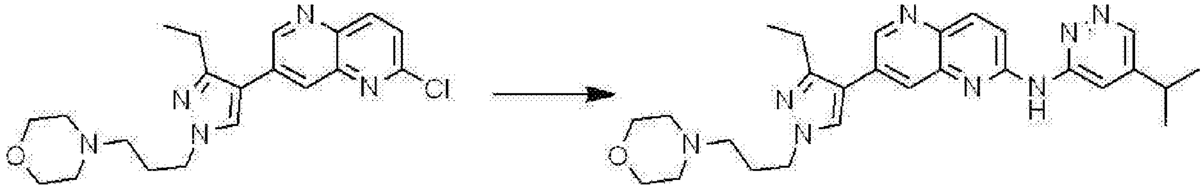

A mixture of 4-(3-(4-(6-chloro-1,5-naphthyridin-3-yl)-3-ethyl-1H-pyrazol-1-yl)propyl)morpholine (18 mg), 5-isopropylpyridazine-3-amine (9.6 mg), tris(dibenzylideneacetone)dipalladium(0) (4.2 mg), 4,5-bis(diphenylphosphino)-9,9-dimethylxanthene (5.4 mg), cesium carbonate (38 mg), and 1,4-dioxane (1 mL) was stirred at 140°C for 30 minutes using a microwave reaction apparatus. The reaction mixture was cooled to room temperature, the insolubles were filtered

off using celite, and the obtained solution was purified by silica gel column chromatography (methanol-ethyl acetate, NH silica), thereby obtaining 7-(3-ethyl-1-(3-morpholinopropyl)-1H-pyrazol-4-yl)-N-(5-isopropylpyridazin-3-yl)-1,5-naphthyridine-2-amine (6.9 mg).
$^1$H-NMR($CDCl_3$/$CD_3OD$=4/1)δ:8.94(1H,brs),8.76(1H,brs),8.71(1H,brs),8.22(1H,d,J=9.0Hz),8.08(1H,brs) ,7.74(1H,s),7.51(1H,d,J=9.0Hz),4.23(2H,t,J=7.2Hz),3.78-3.71(4H,m),3.10-2.98(1H,m),2.89(2H,q,J=7.2Hz),2.53-2.45(4H,m),2.42(2H,t,J=6.6Hz),2.19-2.05(2H,m),1.40(6H,d,J=6.6Hz),1.31(3H,t,J=7.2Hz).
MSm/z(M+H):487.

<Example 0386>

<0386-1>

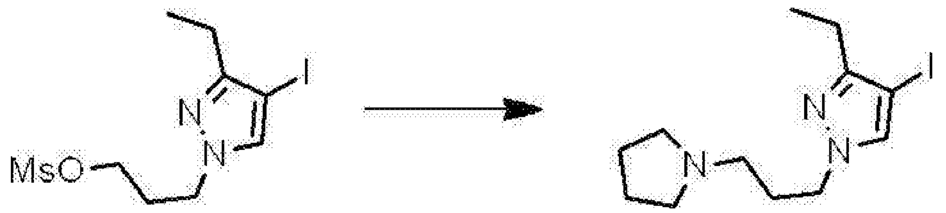

Pyrrolidine (0.172 mL) was added to a mixture of 3-(3-ethyl-4-iodo-1H-pyrazol-1-yl)propyl methanesulfonate (500 mg), potassium carbonate (384 mg), and acetonitrile (7 mL), followed by stirring at 50°C for 5 hours. Ethyl acetate and water were added to the reaction mixture. The organic layer was collected by separation, washed with a saturated sodium chloride aqueous solution, and dried over anhydrous sodium sulfate, and the solvent was distilled off under reduced pressure, thereby obtaining 3-ethyl-4-iodo-1-(3-(pyrrolidin-1-yl)propyl)-1H-pyrazole (250 mg).
MSm/z(M+H):334.

<0386-2>

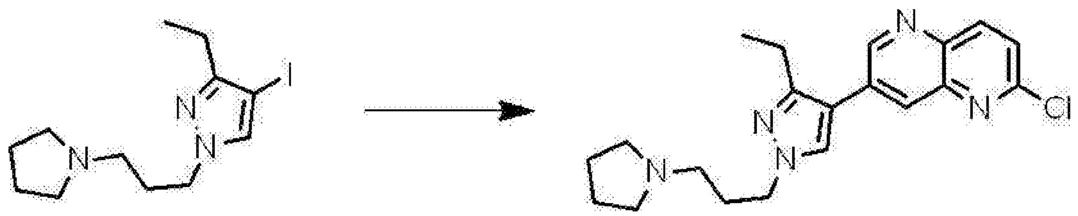

A mixture of 7-bromo-2-chloro-1,5-naphthyridine (182 mg), bis(pinacolato)diboron (286 mg), (1,1'-bis(diphenylphosphino)ferrocene)palladium(II) dichloride (61 mg), potassium acetate (147 mg), and 1,4-dioxane (4 mL) was stirred at 80°C for 2 hours in a nitrogen atmosphere. 3-Ethyl-4-iodo-1-(3-(pyrrolidin-1-yl)propyl)-1H-pyrazole (250 mg), sodium carbonate (158 mg), bis(di-tert-butyl (4-dimethylaminophenyl)phosphine)dichloropalladium(II) (53 mg), and water (0.4 mL) were added thereto, followed by stirring at 80°C for 3 hours. The reaction mixture was cooled to room temperature, the insolubles were filtered off using celite, and the solvent was distilled off under reduced pressure. The obtained residue was purified by silica gel column chromatography (methanol-ethyl acetate, NH silica), thereby obtaining 2-chloro-7-(3-ethyl-1-(3-(pyrrolidin-1-yl)propyl)-1H-pyrazol-4-yl)-1,5-naphthyridine (161 mg).
MSm/z(M+H):370.

<0386-3>

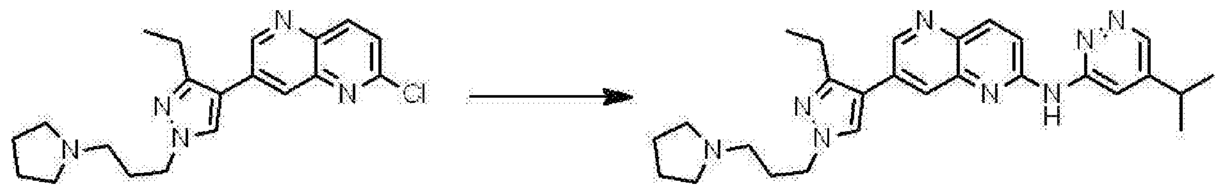

A mixture of 2-chloro-7-(3-ethyl-1-(3-(pyrrolidin-1-yl)propyl)-1H-pyrazol-4-yl)-1,5-naphthyridine (161 mg), 5-isopropylpyridazine-3-amine (9.6 mg), tris(dibenzylideneacetone)dipalladium(0) (4.2 mg), 4,5-bis(diphenylphosphino)-9,9-dimethylxanthene (5.4 mg), cesium carbonate (38 mg), and 1,4-dioxane (1 mL) was stirred at 140°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining 7-(3-ethyl-1-(3-(pyrrolidin-1-yl)propyl)-1H-pyrazol-4-yl)-N-(5-isopropylpyridazin-3-yl)-1,5-naphthyridine-2-amine (9.3 mg).
$^1$H-NMR($CDCl_3$/$CD_3OD$=4/1)δ:8.95(1H,brs),8.78(1H,d,J=2.1Hz),8.71(1H,m),8.22(1H,d,J=8.7Hz),8.09(1H,d,J=2.1Hz),7.77(1H,s),7.50(1H,d,J=8.7Hz),4.22(2H,t,J=7.5Hz),3.10-2.98(1H,m),2.89(2H,q,J=7.2Hz),2.65-2.48(6H,m),2.21-2.07(2H,m),1.88-1.80(4H,m),1.41(6H,d,J=7.5Hz),1.31(3H,t,J=7.2Hz).
MSm/z(M+H):471.

<Example 0387>

<0387-1>

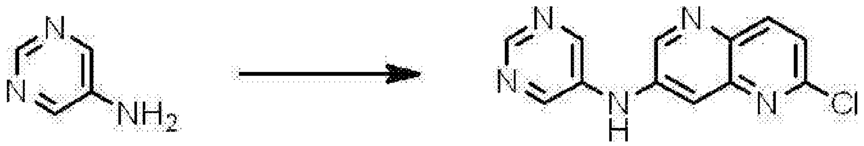

A mixture of 7-bromo-2-chloro-1,5-naphthyridine (100 mg), pyrimidine-5-amine (38 mg), tris(dibenzylideneacetone)dipalladium(0) (37 mg), 4,5-bis(diphenylphosphino)-9,9-dimethylxanthene (47 mg), cesium carbonate (267 mg), and 1,4-dioxane (2 mL) was stirred at 80°C for 6 hours. The reaction mixture was cooled to room temperature, the insolubles were filtered off using celite, and the obtained solution was purified by silica gel column chromatography (methanol-ethyl acetate), thereby obtaining 6-chloro-N-(pyrimidin-5-yl)-1,5-naphthyridine-3-amine (32 mg).
MSm/z(M+H):258.

<0387-2>

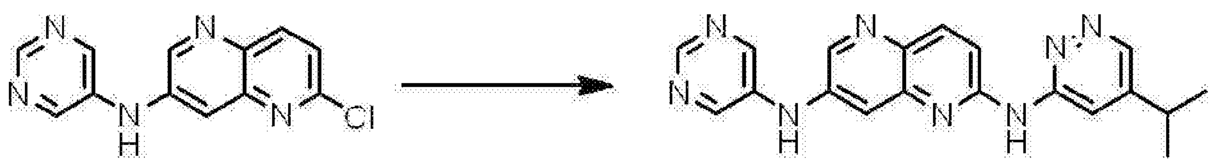

A mixture of 6-chloro-N-(pyrimidin-5-yl)-1,5-naphthyridine-3-amine (10 mg), 5-isopropylpyridazine-3-amine (8.0 mg), tris(dibenzylideneacetone)dipalladium(0) (3.5 mg), 4,5-bis(diphenylphosphino)-9,9-dimethylxanthene (4.5 mg), cesium carbonate (25 mg), and 1,4-dioxane (1 mL) was stirred at 140°C for 30 minutes using a microwave reaction apparatus. The reaction mixture was cooled to room temperature, and the solid matter was collected by filtration, thereby obtaining $N^2$-(5-isopropylpyridazin-3-yl)-$N^7$-(pyrimidin-5-yl)-1,5-naphthyridine-2,7-diamine (4.5 mg).
$^1$H-NMR(DMSO-$d_6$)δ:10.63(1H,s),9.17(1H,s),8.83(1H,d,J=1.8Hz),8.79(1H,s),8.77(2H,s),8.70(1H,d,J=1.8Hz),8.62(1H,d ,J=2.7Hz),8.14(1H,d,J=9.0Hz),7.67(1H,d,J=2.7Hz),7.58(1H,d,J=9.0Hz),3.07-2.93(1H,m),1.28(6H,d,J=7.2Hz).
MSm/z(M+H):359.

<Example 0388>

<0388-1>

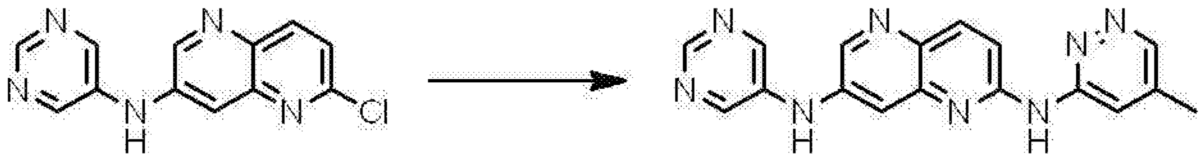

A mixture of 6-chloro-N-(pyrimidin-5-yl)-1,5-naphthyridine-3-amine (10 mg), 5-methylpyridazine-3-amine (6.4 mg), tris(dibenzylideneacetone)dipalladium(0) (3.5 mg), 4,5-bis(diphenylphosphino)-9,9-dimethylxanthene (4.5 mg), cesium carbonate (25 mg), and 1,4-dioxane (1 mL) was stirred at 140°C for 30 minutes using a microwave reaction apparatus. The reaction mixture was cooled to room temperature, and the solid matter was collected by filtration, thereby obtaining $N^2$-(5-methylpyridazin-3-yl)-$N^7$-(pyrimidin-5-yl)-1,5-naphthyridine-2,7-diamine (1.6 mg).
$^1$H-NMR($CDCl_3$/$CD_3OD$=4/1)$\delta$:8.81(1H,s),8.75(2H,s),8.73(1H,brs),8.64(1H,brs),8.51(1H,d,J=2.4Hz),8.12(1H,d,J=9.0Hz),7.83(1H,d,J=2.4Hz),7.36(1H,d,J=9.0Hz),2.45(3H,s).
MSm/z(M+H):331.

<Example 0389>

<0389-1>

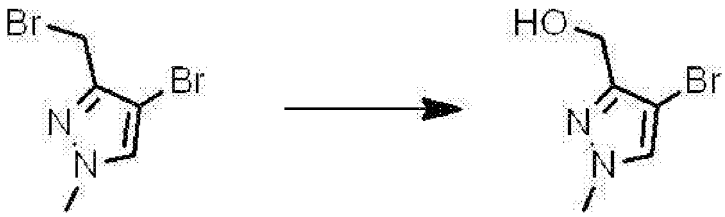

A solution of 4-bromo-3-(bromomethyl)-1-methyl-1H-pyrazole (521 mg) and potassium carbonate (850 mg) in 1,4-dioxane (3 mL) and water (6 mL) was stirred for 8 hours under heating to reflux. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining (4-bromo-1-methyl-1H-pyrazol-3-yl)methanol (334 mg).
MSm/z(M+H):191,193.

<0389-2>

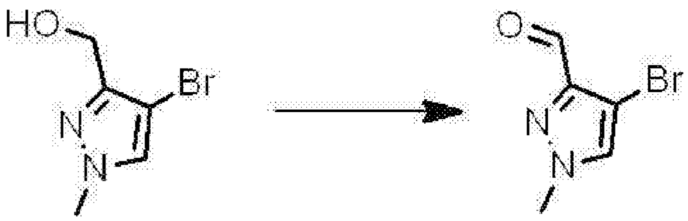

A mixture of (4-bromo-1-methyl-1H-pyrazol-3-yl)methanol (334 mg), manganese dioxide (756 mg), and dichloromethane (8 mL) was stirred at 50°C for 24 hours. The reaction mixture was cooled to room temperature, the insolubles were filtered off using celite, and the solvent was distilled off under reduced pressure, thereby obtaining 4-bromo-1-methyl-1H-pyrazole-3-carbaldehyde (271 mg).

MSm/z(M+H):189,191.

<0389-3>

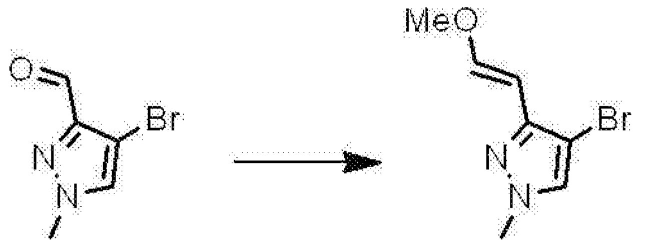

Potassium tert-butoxide (159 mg) was added to a solution of (methoxymethyl)triphenylphosphonium chloride (396 mg) in tetrahydrofuran (2 mL) under ice-cooling, followed by stirring at the same temperature for 30 minutes, and a solution of 4-bromo-1-methyl-1H-pyrazole-3-carbaldehyde (168 mg) in tetrahydrofuran (1 mL) was added to the reaction mixture, followed by stirring at room temperature for 4 hours. Water and ethyl acetate were added to the reaction mixture. The organic layer was collected by separation, washed with a saturated sodium chloride aqueous solution, and dried over anhydrous sodium sulfate, and the obtained solution was purified by silica gel column chromatography (ethyl acetate-hexane), thereby obtaining (E)-4-bromo-3-(2-methoxyvinyl)-1-methyl-1H-pyrazole (49 mg).
MSm/z(M+H):217.

<0389-4>

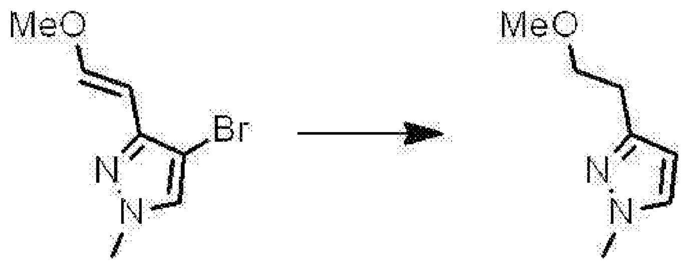

(E)-4-bromo-3-(2-methoxyvinyl)-1-methyl-1H-pyrazole (49 mg) was added to a mixture of 10% palladium-carbon (20 mg) and methanol (5 mL), followed by stirring for 4 hours in a hydrogen atmosphere. The insolubles were filtered off using celite, and the solvent was distilled off under reduced pressure, thereby obtaining 3-(2-methoxyethyl)-1-methyl-1H-pyrazole (46 mg).
$^1$H-NMR($CDCl_3$)δ:7.53(1H,brs),6.46(1H,brs),4.28(3H,s),3.76(2H,t,J=6.0Hz),3.38(3H,s),3.15(2H,t,J=6.0H z).

<0389-5>

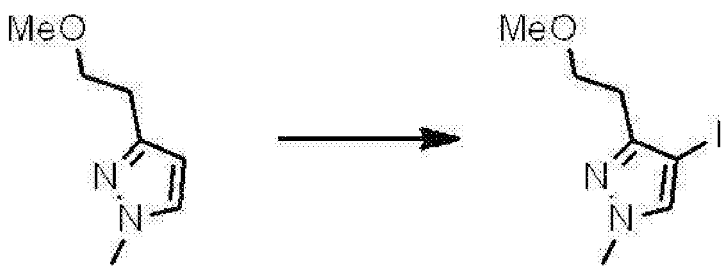

Iodine (34 mg) and ammonium cerium nitrate (74 mg) were added to a solution of 3-(2-methoxyethyl)-1-methyl-1H-pyrazole (46 mg) in acetonitrile (2 mL), followed by stirring at room temperature for 22 hours. Ethyl acetate and water were added to the reaction mixture. The organic layer was collected by separation, washed sequentially with a 10% sodium hydrogen sulfite aqueous solution and a saturated sodium chloride aqueous solution, and dried over anhydrous sodium sulfate, and the solvent was distilled off under reduced pressure, thereby obtaining 4-iodo-3-(2-methoxyethyl)-1-methyl-1H-pyrazole (35 mg).
MSm/z(M+H):267.

<0389-6>

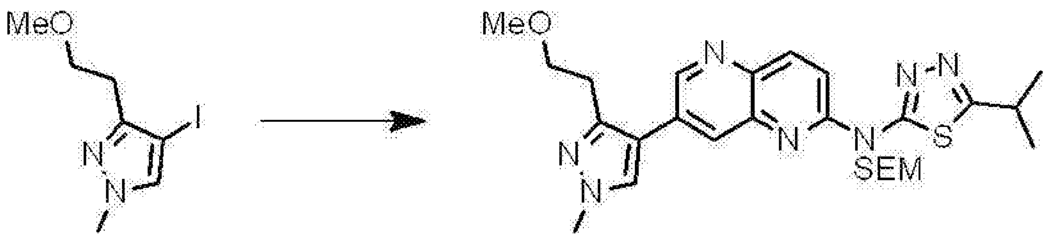

A mixture of N-(7-bromo-1,5-naphthyridin-2-yl)-5-isopropyl-N-((2-(trimethylsilyl)ethoxy)methyl)-1,3,4-thiadiazole-2-amine (20 mg), bis(pinacolato)diboron (13 mg), (1,1'-bis(diphenylphosphino)ferrocene)palladium(II) dichloride (3 mg), potassium acetate (8 mg), and 1,4-dioxane (1 mL) was stirred at 80°C for 2 hours in a nitrogen atmosphere. 4-Iodo-3-(2-methoxyethyl)-1-methyl-1H-pyrazole (11 mg), sodium carbonate (8 mg), bis(di-tert-butyl (4-dimethylaminophenyl)phosphine)dichloropalladium(II) (5 mg), and water (0.1 mL) were added thereto, followed by stirring at 80°C for 3 hours. The reaction mixture was cooled to room temperature, the insolubles were filtered off using celite, and the solvent was distilled off under reduced pressure. The obtained residue was purified by silica gel column chromatography (methanol-ethyl acetate-hexane), thereby obtaining 5-isopropyl-N-(7-(3-(2-methoxyethyl)-1-methyl-1H-pyrazol-4-yl)-1,5-naphthyridin-2-yl)-N-((2-(trimethylsilyl)ethoxy)methyl)-1,3,4-thiadiazole-2-amine (5.6 mg).
MSm/z(M+H):540.

<0389-7>

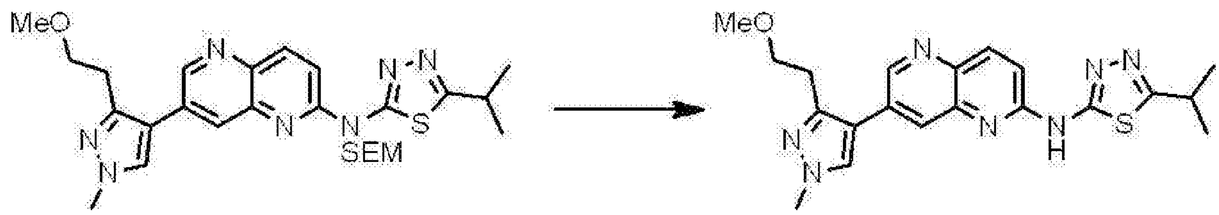

Water (0.2 mL) and trifluoroacetic acid (2 mL) were added to 5-isopropyl-N-(7-(3-(2-methoxyethyl)-1-methyl-1H-pyrazol-4-yl)-1,5-naphthyridin-2-yl)-N-((2-(trimethylsilyl)ethoxy)methyl)-1,3,4-thiadiazole-2-amine (5.6 mg), followed by stirring at room temperature for 30 minutes. The solvent was distilled off under reduced pressure, and the obtained residue was purified by silica gel column chromatography (methanol-ethyl acetate, NH silica), thereby obtaining 5-isopropyl-N-(7-(3-(2-methoxyethyl)-1-methyl-1H-pyrazol-4-yl)-1,5-naphthyridin-2-yl)-1,3,4-thiadiazole-2-amine (5.2 mg).
$^1$H-NMR($CDCl_3$/$CD_3OD$=4/1)δ:8.80(1H,brs),8.31(1H,brs),8.24(1H,d,J=9.0Hz),7.70(1H,s),7.35(1H,d,J=9.0Hz),3.97(3H,s),3.75(2H,t,J=7.5Hz),3.48-3.34(1H,m),3.40(3H,s),3.11(2H,t,J=7.5Hz),1.48(6H,d,J=6.6Hz).
MSm/z(M+H):410.

<Example 0390>

<0390-1>

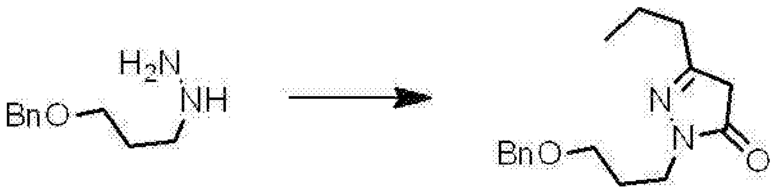

Ethyl 3-oxohexanoate (2.32 mL) was added to a solution of (3-(benzyloxy)propyl)hydrazine (2.19 g) in ethanol (12 mL), followed by stirring for 2 hours under heating to reflux. The reaction mixture was cooled to room temperature, and the solvent was distilled off under reduced pressure. The obtained residue was purified by silica gel column chromatography (ethyl acetate-hexane), thereby obtaining 1-(3-(benzyloxy)propyl)-3-propyl-1H-pyrazol-5(4H)-one (930 mg). MSm/z(M+H):275.

<0390-2>

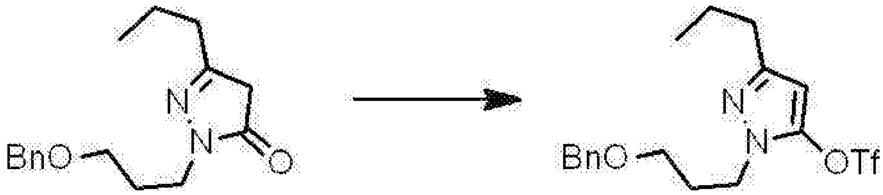

Trifluoromethanesulfonic aicd anhydride (0.834 mL) was added to a solution of 1-(3-(benzyloxy)propyl)-3-propyl-1H-pyrazol-5(4H)-one (930 mg) and pyridine (0.492 mL) in dichloromethane (17 mL) under ice-cooling, followed by stirring at the same temperature for 1 hour. A saturated sodium hydrogen carbonate aqueous solution and dichloromethane were added to the reaction mixture. The organic layer was collected by separation, and dried over anhydrous sodium sulfate, and the solvent was distilled off under reduced pressure, thereby obtaining (1-(3-(benzyloxy)propyl)-3-propyl-1H-pyrazol-5-yl) trifluoromethanesulfonate (1.41 g).
$^1$H-NMR($CDCl_3$)δ:7.36-7.24(5H,m),5.90(1H,s),4.48(2H,s),4.12(2H,t,J=6.6Hz),3.45(2H,t,J=6.0Hz),2.53(2H,t,J=8.1Hz),2.19-2.08(2H,m),1.75-1.55(2H,m),0.94(3H,t,J=7.2Hz).

<0390-3>

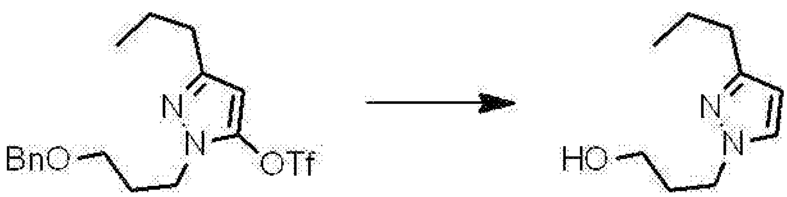

(1-(3-(Benzyloxy)propyl)-3-propyl-1H-pyrazol-5-yl) trifluoromethanesulfonate (1.41 g) was added to a mixture of 20% palladium hydroxide-carbon (100 mg) and methanol (15 mL), followed by stirring for 2 hours in a hydrogen atmosphere. The insolubles were filtered off using celite, and the solvent was distilled off under reduced pressure, thereby obtaining 3-(3-propyl-1H-pyrazol-1-yl)propan-1-ol (1.08 g).

$^1$H-NMR($CDCl_3$)δ:7.83(1H,d,J=2.7Hz),6.41(1H,d,J=2.7Hz),4.59(2H,t,J=6.6Hz),3.73(2H,t,J=5.7Hz),2.79( 2H,t,J=8.1Hz),2.25-2.14(2H,m),1.82-1.67(2H,m),1.00(3H,t,J=7.5Hz).

<0390-4>

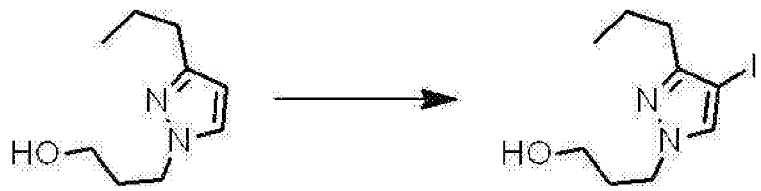

Iodine (516 mg) and ammonium cerium nitrate (1.12 g) were added to a solution of 3-(3-propyl-1H-pyrazol-1-yl)propan-1-ol (1.08 g) in acetonitrile (17 mL), followed by stirring at room temperature for 1 day. Ethyl acetate and water were added to the reaction mixture. The organic layer was collected by separation, washed sequentially with a 10% sodium hydrogen sulfite aqueous solution and a saturated sodium chloride aqueous solution, and dried over anhydrous sodium sulfate, and the solvent was distilled off under reduced pressure, thereby obtaining 3-(4-iodo-3-propyl-1H-pyrazol-1-yl)propan-1-ol (856 mg).
$^1$H-NMR($CDCl_3$)δ:7.36(1H,s),4.23(2H,t,J=6.0Hz),3.62(2H,t,J=6.0Hz),2.55(2H,t,J=7.8Hz),2.06-1.95(2H,m),1.76-1.59(2H,m),0.95(3H,t,J=7.5Hz).

<0390-5>

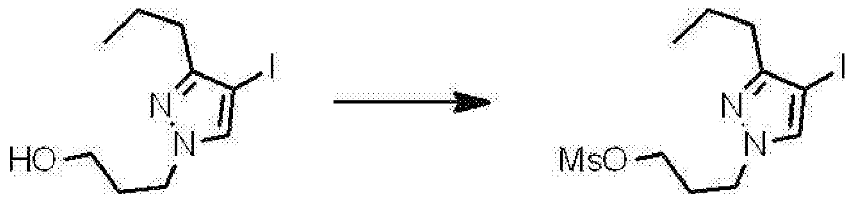

Methanesulfonyl chloride (0.192 mL) was added to a solution of 3-(4-iodo-3-propyl-1H-pyrazol-1-yl)propan-1-ol (856 mg) and triethylamine (0.460 mL) in dichloromethane (8 mL) under ice-cooling, followed by stirring at the same temperature for 1 hour. Dichloromethane and water were added to the reaction mixture. The organic layer was collected by separation, and dried over anhydrous sodium sulfate, and the solvent was distilled off under reduced pressure, thereby obtaining 3-(4-iodo-3-propyl-1H-pyrazol-1-yl)propyl methanesulfonate (539 mg).
$^1$H-NMR($CDCl_3$)δ:7.39(1H,s),4.24-4.17(4H,m),3.03(3H,s),2.55(2H,t,J=7.5Hz),2.33-2.22(2H,m),1.76-1.57(2H,m),0.96(3H,t,J=7.5Hz).

<0390-6>

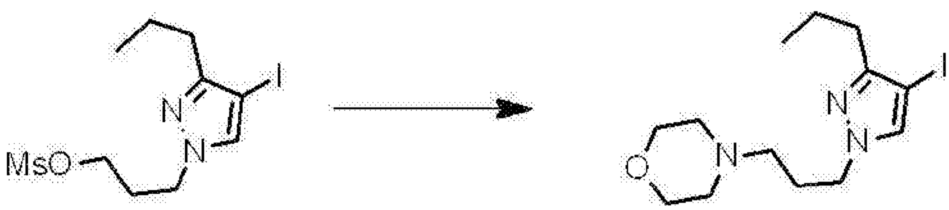

Morpholine (0.094 mL) was added to a mixture of 3-(4-iodo-3-propyl-1H-pyrazol-1-yl)propyl methanesulfonate (269 mg), potassium carbonate (199 mg), and acetonitrile (4 mL), followed by stirring at 50°C for 8 hours. The reaction mixture was cooled to room temperature, and ethyl acetate and water were added thereto. The organic layer was collected by separation, washed with a saturated sodium chloride aqueous solution, and dried over anhydrous sodium sulfate, and the solvent was distilled off under reduced pressure, thereby obtaining 4-(3-(4-iodo-3-propyl-1H-pyrazol-1-yl)propyl)morpholine (43 mg).
MSm/z(M+H):364.

<0390-7>

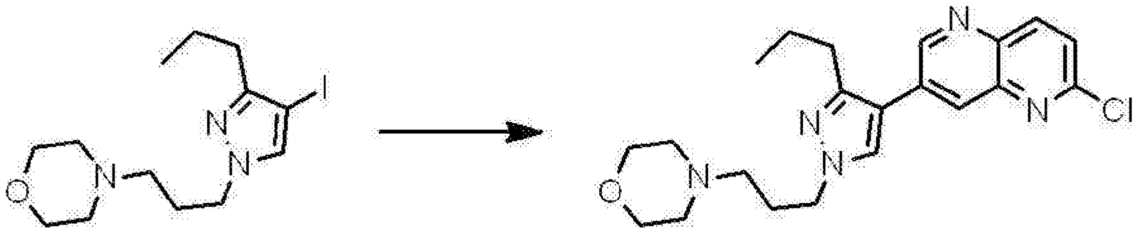

A mixture of 7-bromo-2-chloro-1,5-naphthyridine (29 mg), bis(pinacolato)diboron (36 mg), (1,1'-bis(diphenylphosphino)ferrocene)palladium(II) dichloride (10 mg), potassium acetate (23 mg), and 1,4-dioxane (1 mL) was stirred at 80°C for 2 hours in a nitrogen atmosphere. 4-(3-(4-iodo-3-propyl-1H-pyrazol-1-yl)propyl)morpholine (43 mg), sodium carbonate (25 mg), bis(di-tert-butyl (4-dimethylaminophenyl)phosphine)dichloropalladium(II) (8 mg), and water (0.1 mL) were added thereto, followed by stirring at 80°C for 3 hours. The reaction mixture was cooled to room temperature, the insolubles were filtered off using celite, and the solvent was distilled off under reduced pressure. The obtained residue was purified by silica gel column chromatography (methanol-ethyl acetate, NH silica), thereby obtaining 4-(3-(4-(6-chloro-1,5-naphthyridin-3-yl)-3-propyl-1H-pyrazol-1-yl)propyl)morpholine(11 mg).
MSm/z(M+H):400.

<0390-8>

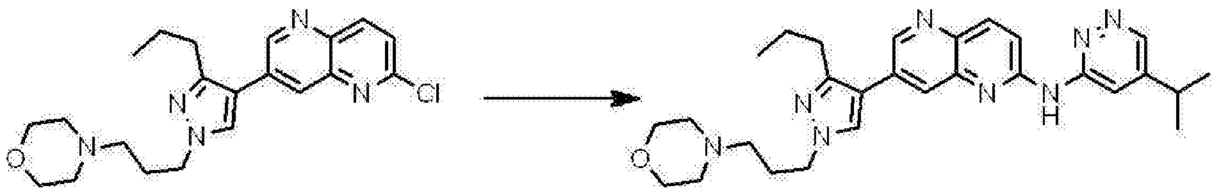

A mixture of 4-(3-(4-(6-chloro-1,5-naphthyridin-3-yl)-3-propyl-1H-pyrazol-1-yl)propyl)morpholine (11 mg), 5-isopropylpyridazine-3-amine (5.6 mg), tris (dibenzylideneacetone)dipalladium(0) (5 mg), 4,5-bis(diphenylphosphino)-9,9-dimethylxanthene (10 mg), cesium carbonate (22 mg), and 1,4-dioxane (1 mL) was stirred at 140°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining N-(5-isopropylpyridazin-3-yl)-7-(1-(3-morpholinopropyl)-3-propyl-1H-pyrazol-4-yl)-1,5-naphthyridine-2-amine (6.5 mg).
$^1$H-NMR($CDCl_3$/$CD_3OD$=4/1)δ:8.95(1H,brs),8.76(1H,d,J=1.8Hz),8.71(1H,brs),8.22(1H,d,J=9.0Hz),8.08( 1H,d,J=1.8Hz),7.74(1H,s),7.50(1H,d,J=9.0Hz),4.23(2H,t,J=6.6Hz),3.78-3.70(4H,m),3.10-2.98(1H,m),2.83(2H,t,J=7.5Hz),2.53-2.44(4H,m),2.41(2H,t,J=7.2Hz),2.18-2.06(2H,m),1.79-1.66(2H,m),1.40(6H,d,J=6.6Hz),0.99(3H,t,J=7.2Hz).
MSm/z(M+H):501.

<Example 0391>

<0391-1>

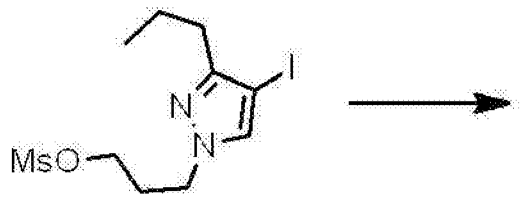

Pyrrolidine (0.089 mL) was added to a mixture of 3-(4-iodo-3-propyl-1H-pyrazol-1-yl)propyl methanesulfonate (269 mg), potassium carbonate (199 mg), and acetonitrile (4 mL), followed by stirring at 50°C for 1 day. Ethyl acetate and water were added to the reaction mixture. The organic layer was collected by separation, washed with a saturated sodium chloride aqueous solution, and dried over anhydrous sodium sulfate, and the solvent was distilled off under reduced pressure, thereby obtaining 4-iodo-3-propyl-1-(3-(pyrrolidin-1-yl)propyl)-1H-pyrazole (113 mg).
MSm/z(M+H):348.

<0391-2>

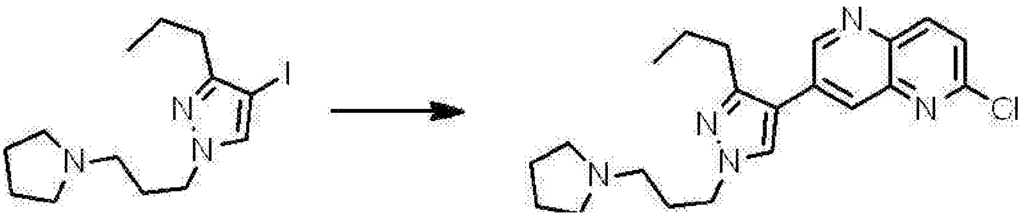

A mixture of 7-bromo-2-chloro-1,5-naphthyridine (79 mg), bis(pinacolato)diboron (99 mg), (1,1'-bis(diphenylphosphino)ferrocene)palladium(II) dichloride (26 mg), potassium acetate (64 mg), and 1,4-dioxane (2 mL) was stirred at 80°C for 2 hours in a nitrogen atmosphere. 4-Iodo-3-propyl-1-(3-(pyrrolidin-1-yl)propyl)-1H-pyrazole (113 mg), sodium carbonate (69 mg), bis(di-tert-butyl (4-dimethylaminophenyl)phosphine)dichloropalladium(II) (23 mg), and water (0.2 mL) were added thereto, followed by stirring at 80°C for 3 hours. The reaction mixture was cooled to room temperature, the insolubles were filtered off using celite, and the solvent was distilled off under reduced pressure. The obtained residue was purified by silica gel column chromatography (methanol-ethyl acetate, NH silica), thereby obtaining 2-chloro-7-(3-propyl-1-(3-(pyrrolidin-1-yl)propyl)-1H-pyrazol-4-yl)-1,5-naphthyridine (53 mg).
MSm/z(M+H):384.

<0391-3>

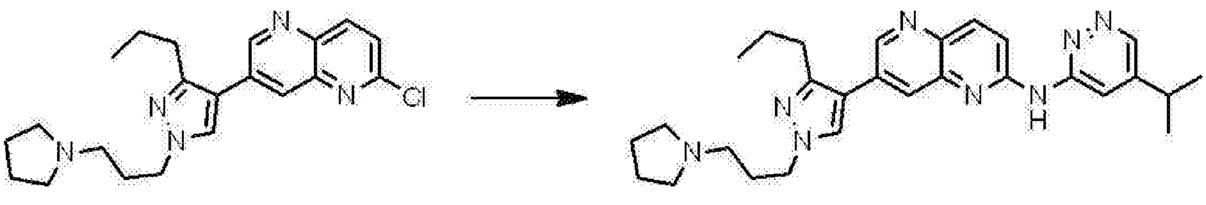

A mixture of 2-chloro-7-(3-propyl-1-(3-(pyrrolidin-1-yl)propyl)-1H-pyrazol-4-yl)-1,5-naphthyridine (27 mg), 5-isopropylpyridazine-3-amine (14 mg), tris(dibenzylideneacetone)dipalladium(0) (5 mg), 4,5-bis(diphenylphosphino)-9,9-dimethylxanthene (10 mg), cesium carbonate (57 mg), and 1,4-dioxane (1 mL) was stirred at 140°C for 30 minutes using a microwave reaction apparatus. The reaction mixture was cooled to room temperature, the insolubles were filtered off

using celite, and the obtained solution was purified by silica gel column chromatography (methanol-ethyl acetate, NH silica), thereby obtaining N-(5-isopropylpyridazin-3-yl)-7-(3-propyl-1-(3-(pyrrolidin-1-yl)propyl)-1H-pyrazol-4-yl)-1,5-naphthyridine-2-amine (6.5 mg).
$^1$H-NMR($CDCl_3$/$CD_3OD$=4/1)δ:8.96(1H,brs),8.77(1H,brs),8.71(1H,brs),8.22(1H,d,J=9.3Hz),8.08(1H,brs) ,7.77(1H,s),7.50(1H,d,J=9.3Hz),4.22(2H,t,J=6.6Hz),3.10-2.98(1H,m),2.83(2H,t,J=7.2Hz),2.61-2.47(6H,m),2.20-2.08(2H,m),1.88-1.68(6H,m),1.41(6H,d,J=6.6Hz),0.99(3H,t,J=7.2Hz).
MSm/z(M+H):485.

<Example 0392>

<0392-1>

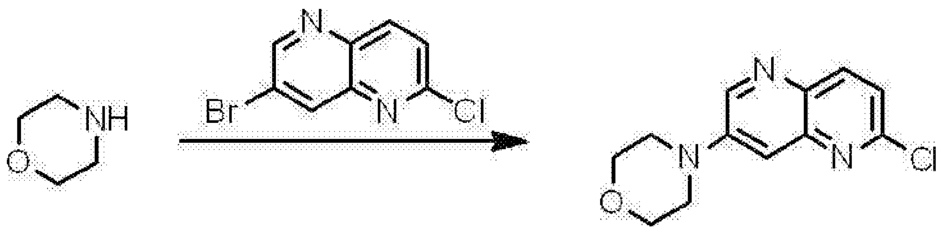

A mixture of 7-bromo-2-chloro-1,5-naphthyridine (50 mg), morpholine (18 mL), tris (dibenzylideneacetone)dipalladium(0) (18 mg), 4,5-bis(diphenylphosphino)-9,9-dimethylxanthene (24 mg), sodium tert-butoxide (39 mg), and 1,4-dioxane (2 mL) was stirred at 80°C for 3 hours. The reaction mixture was cooled to room temperature, the insolubles were filtered off using celite, and the obtained solution was purified by silica gel column chromatography (methanol-ethyl acetate), thereby obtaining 4-(6-chloro-1,5-naphthyridin-3-yl)morpholine (5.5 mg).
MSm/z(M+H):250.

<0392-2>

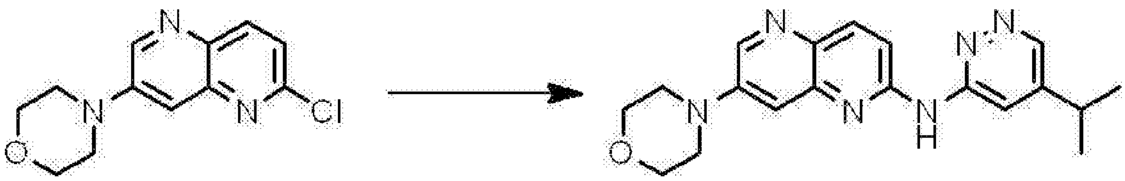

A mixture of 4-(6-chloro-1,5-naphthyridin-3-yl)morpholine (5.5 mg), 5-isopropylpyridazine-3-amine (4.5 mg), tris (dibenzylideneacetone)dipalladium(0) (5 mg), 4,5-bis(diphenylphosphino)-9,9-dimethylxanthene (10 mg), cesium carbonate (20 mg), and 1,4-dioxane (1 mL) was stirred at 140°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining N-(5-isopropylpyridazin-3-yl)-7-morpholino-1,5-naphthyridine-2-amine (5.4 mg).
$^1$H-NMR($CDCl_3$/$CD_3OD$=4/1)δ:8.78(1H,brs),8.69(1H,brs),8.56(1H,brs),8.11(1H,d,J=8.4Hz),7.35(1H,brs) ,7.34(1H,d,J=8.4Hz),3.99-3.93(4H,m),3.42-3.36(4H,m),3.10-2.97(1H,m),1.40(6H,d,J=6.6Hz).
MSm/z(M+H):351.

<Example 0393>

<0393-1>

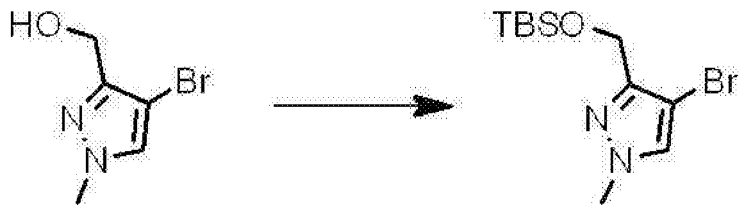

A solution of (4-bromo-1-methyl-1H-pyrazol-3-yl)methanol (325 mg), tert-butyldimethylsilyl chloride (307 mg), and imidazole (289 mg) in N,N-dimethylformamide (6 mL) was stirred at room temperature for 3 days. Ethyl acetate and water were added to the reaction mixture. The organic layer was collected by separation, washed with a saturated sodium chloride aqueous solution, and dried over anhydrous sodium sulfate. The obtained solution was purified by silica gel column chromatography (ethyl acetate-hexane), thereby obtaining 4-bromo-3-(((tert-butyldimethylsilyl)oxy)methyl)-1-methyl-1H-pyrazole (446 mg).
$^1$H-NMR(CDCl$_3$)$\delta$:7.32(1H,s),4.65(2H,s),3.85(3H,s),0.91(9H,s),0.11(6H,s).

<0393-2>

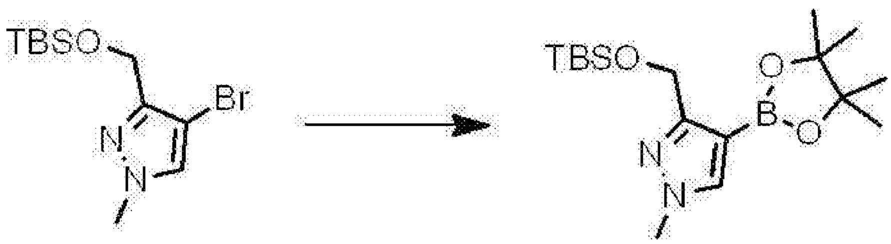

A 1.6 mol/L n-butyllithium-hexane solution (1.36 mL) was added to a solution of 4-bromo-3-(((tert-butyldimethylsilyl)oxy)methyl)-1-methyl-1H-pyrazole (446 mg) in tetrahydrofuran (7 mL) at - 80°C, followed by stirring at the same temperature for 30 minutes, and 2-isopropyloxy-4,4,5,5-tetramethyl-1,3,2-dioxaborolane (0.595 mL) was added thereto at the same temperature, followed by stirring while slowly heating to room temperature over a period of 2.5 hours. A saturated ammonium chloride aqueous solution and ethyl acetate were added to the reaction mixture. The organic layer was collected by separation, washed with a saturated sodium chloride aqueous solution, and dried over anhydrous sodium sulfate, and the solvent was distilled off under reduced pressure, thereby obtaining 3-(((tert-butyldimethylsilyl)oxy)methyl)-1-methyl-4-(4,4,5,5-tetramethyl-1,3,2-dioxaborolan-2-yl)-1H-pyrazole (687 mg).
$^1$H-NMR(CDCl$_3$)$\delta$:7.57(1H,s),4.79(2H,s),3.85(3H,s),1.24(12H,s),0.92(9H,s),0.11(6H,s).

<0393-3>

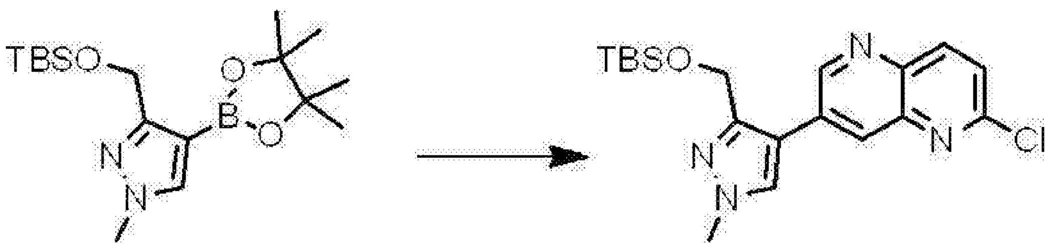

A mixture of 7-bromo-2-chloro-1,5-naphthyridine (80 mg), 3-(((tert-butyldimethylsilyl)oxy)methyl)-1-methyl-4-(4,4,5,5-tetramethyl-1,3,2-dioxaborolan-2-yl)-1H-pyrazole (231 mg), sodium carbonate (87 mg), bis(di-tert-butyl (4-dimethylaminophenyl)phosphine)dichloropalladium(II) (23 mg), water (0.3 mL), and 1,4-dioxane (3 mL) was stirred at 80°C for 2 hours. The reaction mixture was cooled to room temperature, the insolubles were filtered off using celite, and the solvent was distilled off under reduced pressure. The obtained residue was purified by silica gel column chromatography (methanol-ethyl acetate-hexane), thereby obtaining 7-(3-(((tert-butyldimethylsilyl)oxy)methyl)-1-methyl-1H-pyrazol-4-yl)-2-chloro-1,5-naphthyridine (76 mg).
MSm/z(M+H):389.

<0393-4>

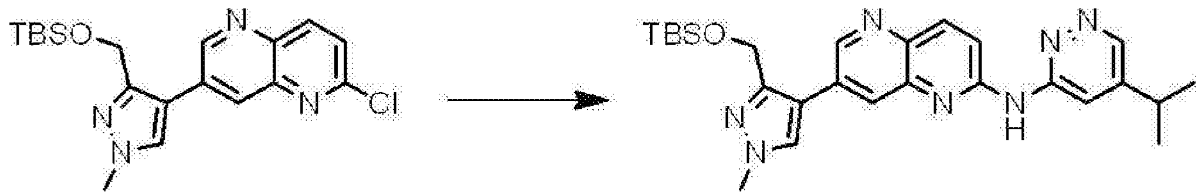

A mixture of 7-(3-(((tert-butyldimethylsilyl)oxy)methyl)-1-methyl-1H-pyrazol-4-yl)-2-chloro-1,5-naphthyridine (25 mg), 5-isopropylpyridazine-3-amine (13 mg), tris (dibenzylideneacetone)dipalladium(0) (5 mg), 4,5-bis(diphenylphosphino)-9,9-dimethylxanthene (10 mg), cesium carbonate (20 mg), and 1,4-dioxane (1 mL) was stirred at 140°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining 7-(3-(((tert-butyldimethylsilyl)oxy)methyl)-1-methyl-1H-pyrazol-4-yl)-N-(5-isopropylpyridazin-3-yl)-1,5-naphthyridine-2-amine (20 mg).
$^1$H-NMR($CDCl_3$)δ:8.95(1H,brs),8.86(1H,brs),8.71(1H,brs),8.27(1H,s),8.23(1H,d,J=9.0Hz),7.79(1H,s),7.5 1(1H,d,J=9.0Hz),4.85(2H,s),3.98(3H,s),3.10-2.98(1H,m),1.40(6H,d,J=6.6Hz),0.83(9H,s),0.08(6H,s).
MSm/z(M+H):490.

<Example 0394>

<0394-1>

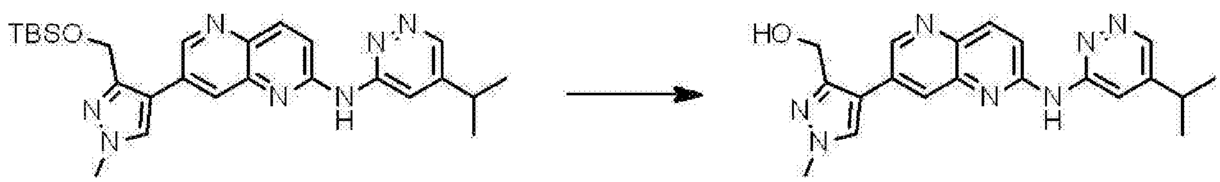

Water (0.1 mL) and trifluoroacetic acid (2 mL) were added to 7-(3-(((tert-butyldimethylsilyl)oxy)methyl)-1-methyl-1H-pyrazol-4-yl)-N-(5-isopropylpyridazin-3-yl)-1,5-naphthyridine-2-amine (20 mg), followed by stirring at room temperature for 1 hour. The solvent was distilled off under reduced pressure, and the obtained residue was purified by silica gel column chromatography (methanol-ethyl acetate, NH silica), thereby obtaining (4-(6-((5-isopropylpyridazin-3-yl)amino)-1,5-naphthyridin-3-yl)-1-methyl-1H-pyrazol-3-yl)methanol (5.9 mg).
$^1$H-NMR($CDCl_3$/$CD_3OD$=4/1)δ:8.90(1H,d,J=2.1Hz),8.87(1H,brs),8.71(1H,brs),8.36(1H,d,J=2.1Hz),8.22( 1H,d,J=9.3Hz),7.81(1H,s),7.55(1H,d,J=9.3Hz),4.76(2H,s),3.99(3H,s),3.10-2.97(1H,m),1.40(6H,d,J=6.6Hz).
MSm/z(M+H):376.

<Example 0395>

<0395-1>

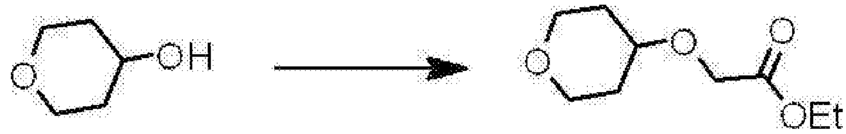

60% sodium hydride (318 mg) was added to a solution of tetrahydro-2H-pyran-4-ol (542 mg), ethyl bromoacetate (0.590 mL), and tetrahydrofuran (25 mL), under ice-cooling, followed by stirring at room temperature for 2.5 hours. A saturated ammonium chloride aqueous solution and ethyl acetate were added to the reaction mixture. The organic layer was collected by separation, washed sequentially with water and a saturated sodium chloride aqueous solution, and dried over anhydrous sodium sulfate. The obtained solution was purified by silica gel column chromatography (ethyl acetate-hexane), thereby obtaining ethyl 2-((tetrahydro-2H-pyran-4-yl)oxy)acetate (184 mg).
MSm/z(M+H):189.

<0395-2>

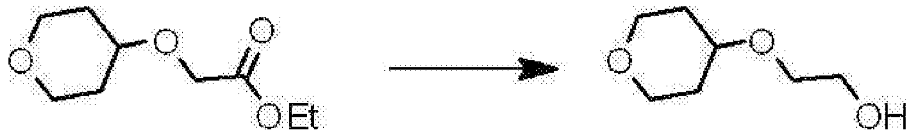

A solution of ethyl 2-((tetrahydro-2H-pyran-4-yl)oxy)acetate (184 mg) in tetrahydrofuran (10 mL) was added to a mixture of lithium aluminium hydride (185 mg) and tetrahydrofuran (10 mL) under ice-cooling, followed by stirring at room temperature for 1 hour, and a 3 mol/L potassium sodium tartrate aqueous solution (10 mL) was added thereto under ice-cooling, followed by stirring at room temperature for 1 day. Ethyl acetate was added to the reaction mixture, and the insolubles were filtered off using celite. The organic layer was collected by separation, washed with a saturated sodium chloride aqueous solution, and dried over anhydrous sodium sulfate, and the solvent was distilled off under reduced pressure, thereby obtaining 2-((tetrahydro-2H-pyran-4-yl)oxy)ethanol (95 mg).
MSm/z(M+H):147.

<0395-3>

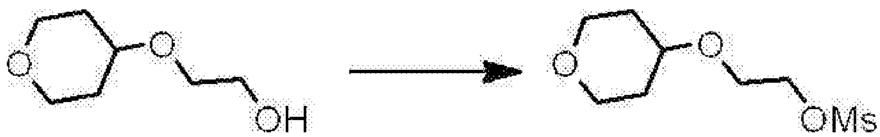

Methanesulfonyl chloride (0.076 mL) was added to a solution of 2-((tetrahydro-2H-pyran-4-yl)oxy)ethanol (95 mg) and triethylamine (0.183 mL) in dichloromethane (6 mL) under ice-cooling, followed by stirring at the same temperature for 30 minutes. Dichloromethane and water were added to the reaction mixture. The organic layer was collected by separation, and dried over anhydrous sodium sulfate, and the solvent was distilled off under reduced pressure, thereby obtaining 2-((tetrahydro-2H-pyran-4-yl)oxy)ethyl methanesulfonate (242 mg).
$^1$H-NMR($CDCl_3$)$\delta$:3.75(2H,t,J=4.5Hz),3.99-3.90(2H,m),3.74(2H,t,J=4.5Hz),3.60-3.39(3H,m),3.06(3H,s),1.96-1.82(2H,m), 1.66-1.52(2H,m).

<0395-4>

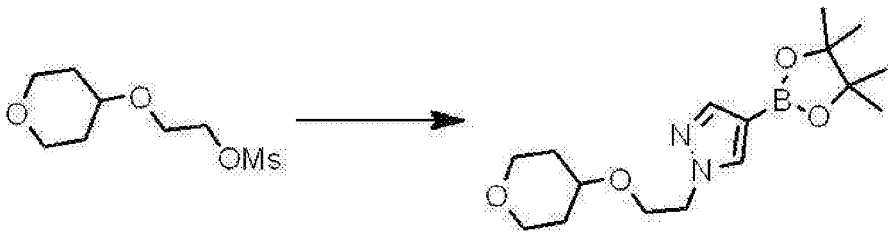

A mixture of 2-((tetrahydro-2H-pyran-4-yl)oxy)ethyl methanesulfonate (242 mg), 4-(4,4,5,5-tetramethyl-1,3,2-dioxaborolan-2-yl)-1H-pyrazole (80 mg), cesium carbonate (425 mg), acetonitrile (1 mL), and 1,4-dioxane (2 mL) was stirred at 80°C for 5 hours. The reaction mixture was cooled to room temperature, and ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate-hexane), thereby obtaining 1-(2-((tetrahydro-2H-pyran-4-yl)oxy)ethyl)-4-(4,4,5,5-tetramethyl-1,3,2-dioxaborolan-2-yl)-1H-pyrazole (108 mg).
MSm/z(M+H):323.

<0395-5>

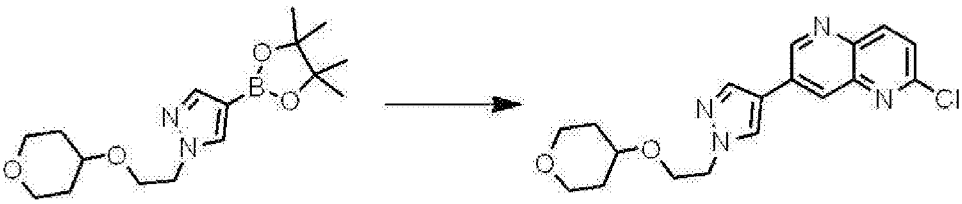

A mixture of 7-bromo-2-chloro-1,5-naphthyridine (82 mg), 1-(2-((tetrahydro-2H-pyran-4-yl)oxy)ethyl)-4-(4,4,5,5-tetramethyl-1,3,2-dioxaborolan-2-yl)-1H-pyrazole (108 mg), sodium carbonate (71 mg), bis(di-tert-butyl (4-dimethylaminophenyl)phosphine)dichloropalladium(II) (23 mg), water (0.3 mL), and 1,4-dioxane (3 mL) was stirred at 80°C for 4 hours. The reaction mixture was cooled to room temperature, the insolubles were filtered off using celite, and the solvent was distilled off under reduced pressure. The obtained residue was purified by silica gel column chromatography (methanol-ethyl acetate), thereby obtaining 2-chloro-7-(1-(2-((tetrahydro-2H-pyran-4-yl)oxy)ethyl)-1H-pyrazol-4-yl)-1,5-naphthyridine (26 mg).
MSm/z(M+H):359.

<0395-6>

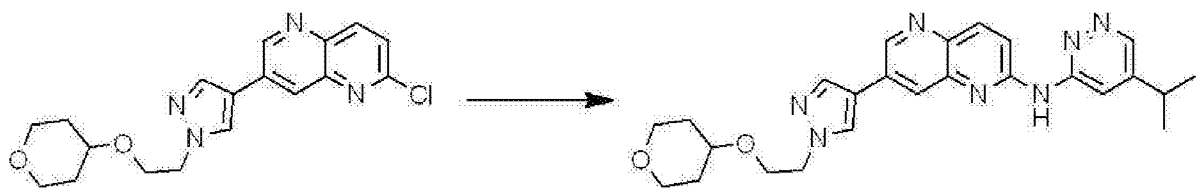

Amixture of 2-chloro-7-(1-(2-((tetrahydro-2H-pyran-4-yl)oxy)ethyl)-1H-pyrazol-4-yl)-1,5-naphthyridine (26 mg), 5-isopropylpyridazine-3-amine (6 mg), tris(dibenzylideneacetone)dipalladium(0) (5 mg), 4,5-bis(diphenylphosphino)-9,9-dimethylxanthene (10 mg), cesium carbonate (20 mg), and 1,4-dioxane (1 mL) was stirred at 140°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining N-(5-isopropylpyridazin-3-yl)-7-(1-(2-((tetrahydro-2H-pyran-4-yl)oxy)ethyl)-1H-pyrazol-4-yl)-1,5-naphthyridine-2-amine (8.0 mg).
$^1$H-NMR($CDCl_3$/$CD_3OD$=4/1)δ:8.88(2H,brs),8.72(1H,brs),8.20(1H,d,J=8.7Hz),8.14(1H,brs),8.05(1H,s),7.

99(1H,s),7.50(1H,d,J=8.7Hz),4.45-4.36(2H,m),3.94-3.81(4H,m),3.56-3.34(3H,m),3.16-2.98(1H,m),1.93-1.81(2H,m),1.64-1.46(2H,m),1.42(6H,d,J=7.2Hz).
MSm/z(M+H):460.

<Example 0396>

<0396-1>

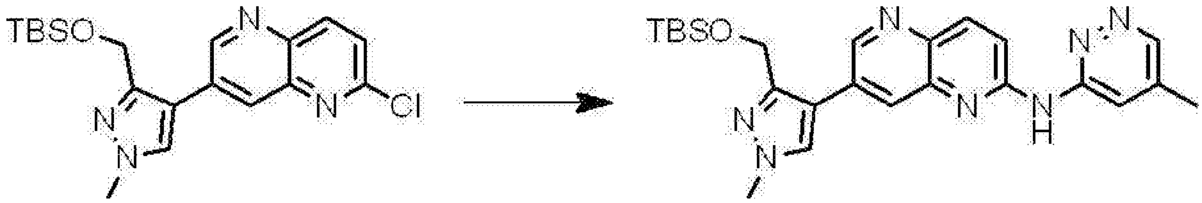

A mixture of 7-(3-(((tert-butyldimethylsilyl)oxy)methyl)-1-methyl-1H-pyrazol-4-yl)-2-chloro-1,5-naphthyridine (25 mg), 5-methylpyridazine-3-amine (11 mg), tris (dibenzylideneacetone)dipalladium(0) (5 mg), 4,5-bis(diphenylphosphino)-9,9-dimethylxanthene (10 mg), cesium carbonate (20 mg), and 1,4-dioxane (1 mL) was stirred at 140°C for 30 minutes using a microwave reaction apparatus. The reaction mixture was cooled to room temperature, the insolubles were filtered off using celite, and the obtained solution was purified by silica gel column chromatography (methanol-ethyl acetate, NH silica), thereby obtaining 7-(3-(((tert-butyldimethylsilyl)oxy)methyl)-1-methyl-1H-pyrazol-4-yl)-N-(5-methylpyridazin-3-yl)-1,5-naphthyridine-2-amine (12 mg).
$^1$H-NMR($CDCl_3$)δ:8.97(1H,brs),8.78(1H,brs),8.73(1H,brs),8.37(1H,s),8.26(1H,d,J=8.4Hz),7.71(1H,s),7.44(1H,d,J=8.4Hz),4.84(2H,s),3.98(3H,s),2.45(3H,s),0.87(9H,s),0.11(6H,s).
MSm/z(M+H):462.

<Example 0397>

<0397-1>

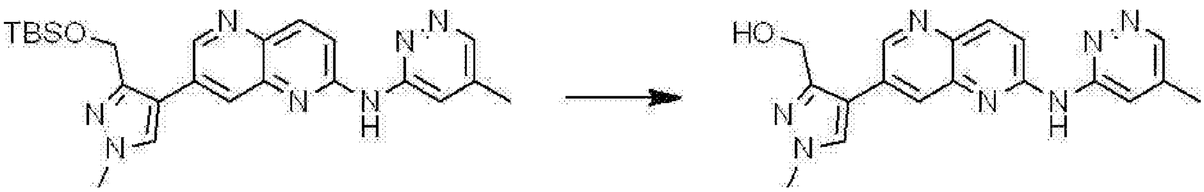

Water (0.1 mL) and trifluoroacetic acid (2 mL) were added to 7-(3-(((tert-butyldimethylsilyl)oxy)methyl)-1-methyl-1H-pyrazol-4-yl)-N-(5-methylpyridazin-3-yl)-1,5-naphthyridine-2-amine (12 mg), followed by stirring at room temperature for 3 hours. The solvent was distilled off under reduced pressure, and the obtained residue was purified by silica gel column chromatography (methanol-ethyl acetate, NH silica), thereby obtaining (1-methyl-4-(6-((5-methylpyridazin-3-yl)amino)-1,5-naphthyridin-3-yl)-1H-pyrazol-3-yl)methanol (9.6 mg).
$^1$H-NMR($CDCl_3$/$CD_3OD$=4/1)δ:8.90(1H,brs),8.79(1H,brs),8.64(1H,brs),8.35(1H,brs),8.20(1H,d,J=8.1Hz) ,7.80(1H,s),7.53(1H,d,J=8.1Hz),4.77(2H,s),3.36(3H,s),2.46(3H,s).
MSm/z(M+H):348.

<Example 0398>

<0398-1>

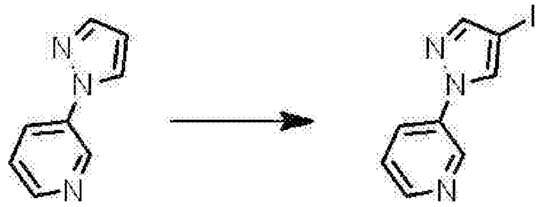

Iodine (208 mg) and ammonium cerium nitrate (450 mg) were added to a solution of 3-(1H-pyrazol-1-yl)pyridine (200 mg) in acetonitrile (3 mL), followed by stirring at room temperature for 13 hours, and stirring for 8 hours under heating to reflux. After the reaction mixture was cooled to room temperature, ethyl acetate and water were added thereto. The organic layer was collected by separation, washed sequentially with a 10% sodium hydrogen sulfite aqueous solution and a saturated sodium chloride aqueous solution, and dried over anhydrous sodium sulfate, and the solvent was distilled off under reduced pressure, thereby obtaining 3-(4-iodo-1H-pyrazol-1-yl)pyridine (333 mg).
MSm/z(M+H):272.

<0398-2>

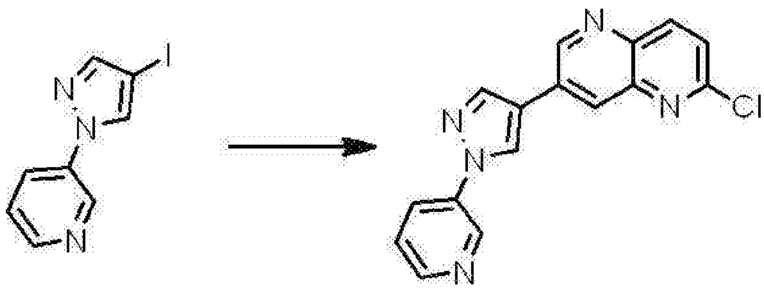

A mixture of 7-bromo-2-chloro-1,5-naphthyridine (50 mg), bis(pinacolato)diboron (62 mg), (1,1'-bis(diphenylphosphino)ferrocene)palladium(II) dichloride (16 mg), potassium acetate (40 mg), and 1,4-dioxane (2 mL) was stirred at 80°C for 2 hours in a nitrogen atmosphere. 3-(4-Iodo-1H-pyrazol-1-yl)pyridine (61 mg), sodium carbonate (43 mg), bis(di-tert-butyl (4-dimethylaminophenyl)phosphine)dichloropalladium(II) (17 mg), and water (0.2 mL) were added thereto, followed by stirring at 80°C for 8 hours. The reaction mixture was cooled to room temperature, the insolubles were filtered off using celite, and the solvent was distilled off under reduced pressure. The obtained residue was purified by silica gel column chromatography (methanol-ethyl acetate-hexane), thereby obtaining 2-chloro-7-(1-(pyridin-3-yl)-1H-pyrazol-4-yl)-1,5-naphthyridine (27 mg).
MSm/z(M+H):308.

<0398-3>

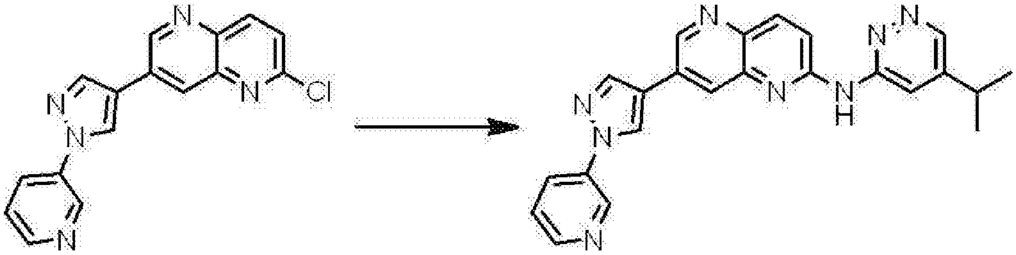

A mixture of 2-chloro-7-(1-(pyridin-3-yl)-1H-pyrazol-4-yl)-1,5-naphthyridine (13 mg), 5-isopropylpyridazine-3-amine (7.4 mg), tris (dibenzylideneacetone)dipalladium(0) (5 mg), 4,5-bis(diphenylphosphino)-9,9-dimethylxanthene (10 mg), cesium carbonate (20 mg), and 1,4-dioxane (1 mL) was stirred at 140°C for 30 minutes using a microwave reaction

apparatus. The reaction mixture was cooled to room temperature, and the solid matter was collected by filtration, thereby obtaining N-(5-isopropylpyridazin-3-yl)-7-(1-(pyridin-3-yl)-1H-pyrazol-4-yl)-1,5-naphthyridine-2-amine (12 mg).
$^1$H-NMR(DMSO-$d_6$)δ:10.74(1H,s),9.41(1H,s),9.21(1H,brs),9.19(1H,brs),8.88(1H,brs),8.72(1H,brs),8.63(1H,s),8.59(1H, d,J=4.2Hz),8.39(1H,brs),8.38-8.29(1H,m),8.27(1H,d,J=8.7Hz),7.75(1H,d,J=8.7Hz),7.66-7.58(1H,m),3.07-2.93(1H,m), 1.35(6H,d,J=6.6Hz).
MSm/z(M+H):409.

<Example 0399>

<0399-1>

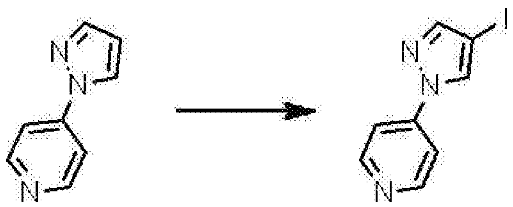

Iodine (569 mg) and ammonium cerium nitrate (1.22 g) were added to a solution of 4-(1H-pyrazol-1-yl)pyridine (544 mg) in acetonitrile (8 mL), followed by stirring for 3 hours under heating to reflux. The reaction mixture was cooled to room temperature, and ethyl acetate and water were added thereto. The organic layer was collected by separation, washed sequentially with a 10% sodium hydrogen sulfite aqueous solution and a saturated sodium chloride aqueous solution, and dried over anhydrous sodium sulfate. The obtained solution was purified by silica gel column chromatography (methanol-ethyl acetate-hexane), thereby obtaining 4-(4-iodo-1H-pyrazol-1-yl)pyridine (75 mg).
MSm/z(M+H):272.

<0399-2>

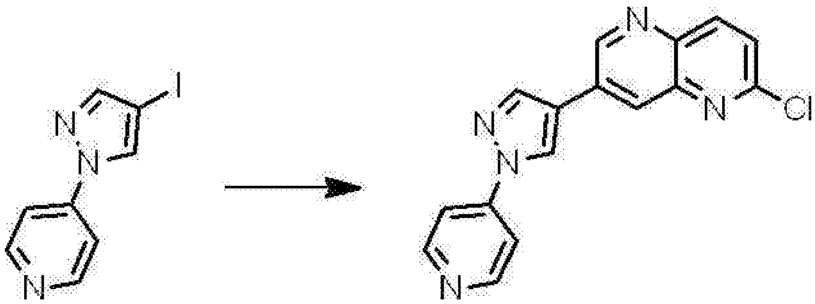

A mixture of 7-bromo-2-chloro-1,5-naphthyridine (48 mg), bis(pinacolato)diboron (60 mg), (1,1'-bis(diphenylphosphino)ferrocene)palladium(II) dichloride (16 mg), potassium acetate (39 mg), and 1,4-dioxane (2 mL) was stirred at 80°C for 2 hours in a nitrogen atmosphere. 4-(4-Iodo-1H-pyrazol-1-yl)pyridine (75 mg), sodium carbonate (42 mg), bis(di-tert-butyl (4-dimethylaminophenyl)phosphine)dichloropalladium(II) (14 mg), and water (0.2 mL) were added thereto, followed by stirring at 80°C for 8 hours. The reaction mixture was cooled to room temperature, the insolubles were filtered off using celite, and the solvent was distilled off under reduced pressure. The obtained residue was purified by silica gel column chromatography (methanol-ethyl acetate-hexane), thereby obtaining 2-chloro-7-(1-(pyridin-4-yl)-1H-pyrazol-4-yl)-1,5-naphthyridine (29 mg).
MSm/z(M+H):308.

<0399-3>

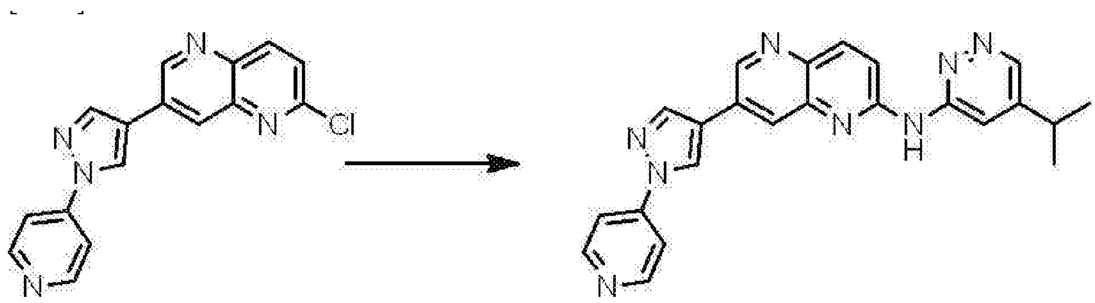

A mixture of 2-chloro-7-(1-(pyridin-4-yl)-1H-pyrazol-4-yl)-1,5-naphthyridine (14 mg), 5-isopropylpyridazine-3-amine (7.8 mg), tris(dibenzylideneacetone)dipalladium(0) (4.3 mg), 4,5-bis(diphenylphosphino)-9,9-dimethylxanthene (5.5 mg), cesium carbonate (39 mg), and 1,4-dioxane (1 mL) was stirred at 140°C for 30 minutes using a microwave reaction apparatus. The reaction mixture was cooled to room temperature, and the solid matter was collected by filtration, thereby obtaining N-(5-isopropylpyridazin-3-yl)-7-(1-(pyridin-4-yl)-1H-pyrazol-4-yl)-1,5-naphthyridine-2-amine (2.2 mg).
$^1$H-NMR(DMSO-$d_6$)δ:10.74(1H,s),9.52(1H,s),9.19(1H,brs),8.88(1H,brs),8.72(2H,brs),8.67(1H,brs),8.42(1H,brs),8.27(1H,d,J=7.8Hz),7.96(2H,brs),7.75(1H,d,J=7.8Hz),7.50-7.32(1H,m),3.07-2.93(1H,m),1.35(6H,d,J=6.0Hz).
MSm/z(M+H):409.

<Example 0400>

<0400-1>

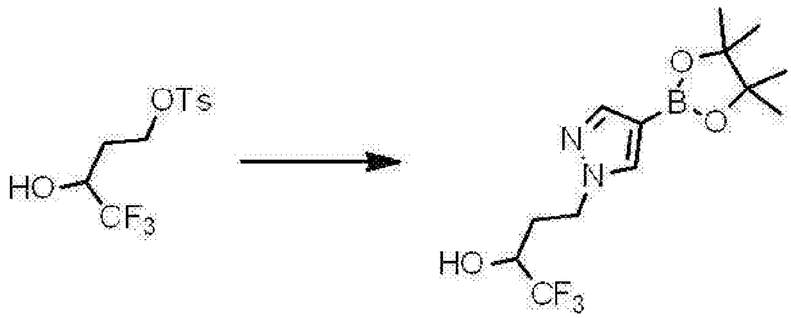

A mixture of (4,4,4-trifluoro-3-hydroxybutyl) 4-methylbenzenesulfonate (267 mg), 4-(4,4,5,5-tetramethyl-1,3,2-dioxaborolan-2-yl)-1H-pyrazole (100 mg), cesium carbonate (317 mg), acetonitrile (0.5 mL), and 1,4-dioxane (1 mL) was stirred at 80°C for 4 hours. After the reaction mixture was cooled to room temperature, ethyl acetate and water were added thereto. The organic layer was collected by separation, washed with a saturated sodium chloride aqueous solution, and dried over anhydrous sodium sulfate. The obtained solution was purified by silica gel column chromatography (methanol-ethyl acetate-hexane), thereby obtaining 1,1,1-trifluoro-4-(4-(4,4,5,5-tetramethyl-1,3,2-dioxaborolan-2-yl)-1H-pyrazol-1-yl)butan-2-ol(203 mg).
$^1$H-NMR($CDCl_3$)δ:7.79(1H,s),7.71(1H,s),4.51-4.25(2H,m),3.99-3.87(1H,m),2.32-1.82(2H,m),1.32(12H,s).

<0400-2>

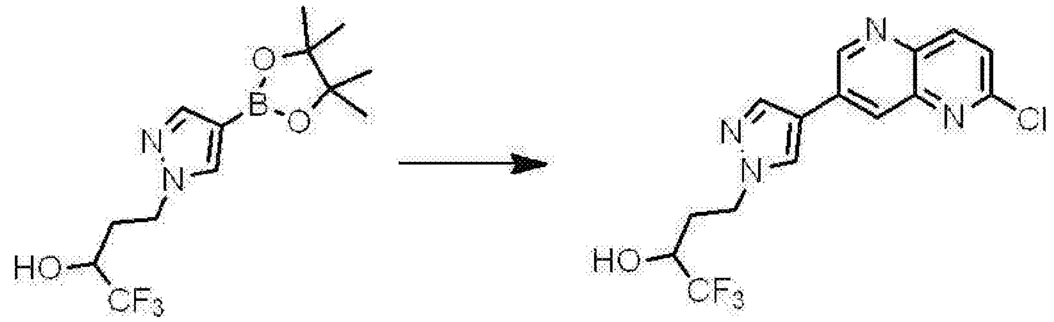

A mixture of 7-bromo-2-chloro-1,5-naphthyridine (51 mg), 1,1,1-trifluoro-4-(4-(4,4,5,5-tetramethyl-1,3,2-dioxaborolan-2-yl)-1H-pyrazol-1-yl)butan-2-ol (101 mg), sodium carbonate (56 mg), bis(di-tert-butyl (4-dimethylaminophenyl)phosphine)dichloropalladium(II) (15 mg), water (0.2 mL), and 1,4-dioxane (2 mL) was stirred at 80°C for 4 hours. The reaction mixture was cooled to room temperature, the insolubles were filtered off using celite, and the solvent was distilled off under reduced pressure. The obtained residue was purified by silica gel column chromatography (methanol-ethyl acetate), thereby obtaining 4-(4-(6-chloro-1,5-naphthyridin-3-yl)-1H-pyrazol-1-yl)-1,1,1-trifluorobutan-2-ol (12 mg).
MSm/z(M+H):357.

<0400-3>

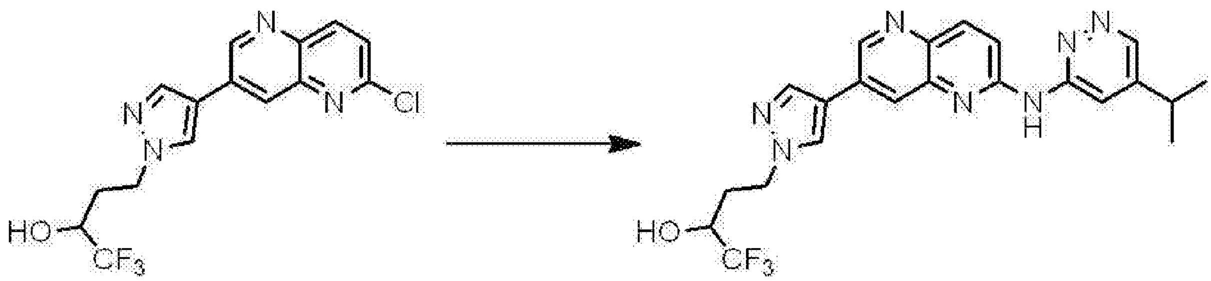

A mixture of 4-(4-(6-chloro-1,5-naphthyridin-3-yl)-1H-pyrazol-1-yl)-1,1,1-trifluorobutan-2-ol (12 mg), 5-isopropylpyridazine-3-amine (5.8 mg), tris(dibenzylideneacetone)dipalladium(0) (5 mg), 4,5-bis(diphenylphosphino)-9,9-dimethylxanthene (10 mg), cesium carbonate (20 mg), and 1,4-dioxane (1 mL) was stirred at 140°C for 30 minutes using a microwave reaction apparatus. The reaction mixture was cooled to room temperature, and the solid matter was collected by filtration, thereby obtaining 1,1,1-trifluoro-4-(4-(6-((5-isopropylpyridazin-3-yl)amino)-1,5-naphthyridin-3-yl)-1H-pyrazol-1-yl)butan-2-ol (6.0 mg).
$^1$H-NMR($CDCl_3$/$CD_3OD$=4/1)δ:8.89(1H,d,J=2.1Hz),8.83(1H,brs),8.72(1H,brs),8.21(1H,d,J=8.7Hz),8.17( 1H,brs),8.04(1H,s),8.01(1H,s),7.52(1H,d,J=8.7Hz),4.49-4.40(2H,m),3.93-3.75(1H,m),3.14-3.00(1H,m),2.42-2.00(2H,m),1.42(6H,d,J=7.2Hz).
MSm/z(M+H):458.

<Example 0401>

<0401-1>

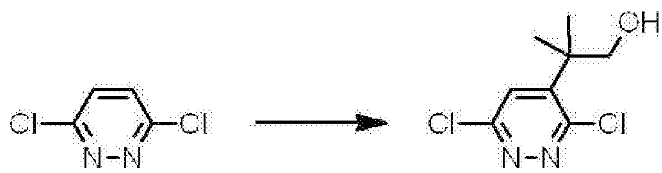

2-(3,6-Dichloropyridazin-4-yl)-2-methylpropan-1-ol was obtained as a white solid in the same manner as in Example 0014 except that 2,2-dimethyl-3-hydroxypropionic acid was used instead of the isobutyric acid used in Example 0014.
$^1$H-NMR($CDCl_3$)δ:7.58(1H,s),4.00(2H,s),1.48(6H,s).

<0401-2>

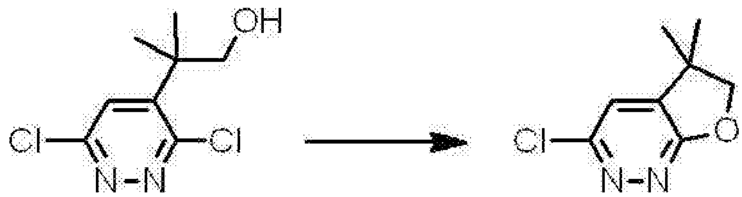

2,4-dimethoxybenzylamine (650 μL) and 1,8-diazabicyclo[5.4.0]undeca-7-ene (850 μL) were added to a solution of 2-(3,6-dichloropyridazin-4-yl)-2-methylpropan-1-ol (497 mg) in 1,4-dioxane (7 mL), followed by stirring at 150°C for 30 minutes using a microwave reaction apparatus. The reaction mixture was cooled to room temperature, and ethyl acetate was added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane, NH silica), thereby obtaining 3-chloro-5,5-dimethyl-5,6-dihydrofuro[2,3-c]pyridazine (165 mg) as a white solid.
$^1$H-NMR($CDCl_3$)δ:7.19(1H,s),4.41(2H,s),1.43(6H,s).

<0401-3>

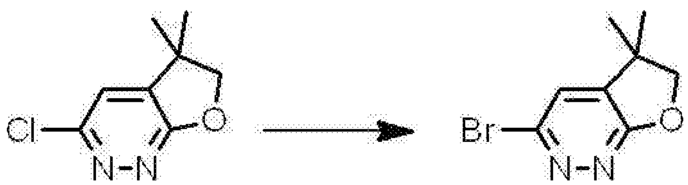

Phosphorous oxybromide (1.0 g) was added to 3-chloro-5,5-dimethyl-5,6-dihydrofuro[2,3-c]pyridazine (50 mg), followed by stirring at 100°C for 30 minutes. The reaction mixture was cooled to room temperature, added dropwise to a mixture solution of methanol-water (1:10), the resultant product was neutralized by the addition of a sodium hydroxide aqueous solution, and ethyl acetate was added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining 3-bromo-5,5-dimethyl-5,6-dihydrofuro[2,3-c]pyridazine (45 mg).
MSm/z(M+H):229,231.

<0401-3>

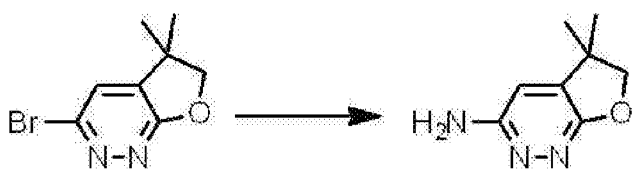

25% ammonia water (1 mL) and copper(I) oxide (5 mg) were added to a solution of 3-bromo-5,5-dimethyl-5,6-dihydrofuro[2,3-c]pyridazine (30 mg) in ethylene glycol (1 mL), followed by stirring at 150°C for 30 minutes using a microwave reaction apparatus. The reaction mixture was cooled to room temperature, and the solvent was distilled off under reduced pressure. The obtained residue was purified by silica gel column chromatography (ethyl acetate-methanol), thereby obtaining 5,5-dimethyl-5,6-dihydrofuro[2,3-c]pyridazine-3-amine (20 mg).
MSm/z(M+H):166.

<0401-4>

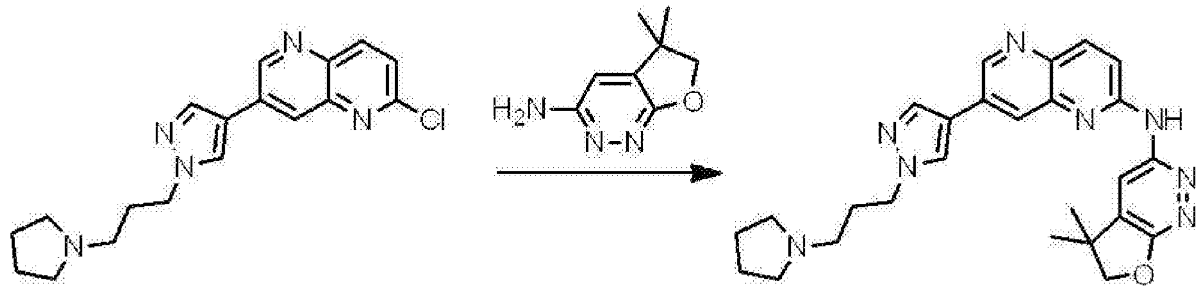

5,5-Dimethyl-N-(7-(1-(3-(pyrrolidin-1-yl)propyl)-1H-pyrazol-4-yl)-1,5-naphthyridin-2-yl)-5,6-dihydrofuro[2,3-c]pyridazine-3-amine was obtained as a pale yellow solid in the same manner as in Example 0290 except that 5,5-dimethyl-5,6-dihydrofuro[2,3-c]pyridazine-3-amine was used instead of the 5-(tert-butyl)pyridazine-3-amine used in Example 0290.
$^1$H-NMR(DMSO-$d_6$)δ:10.50(1H,s),9.01(1H,d,J=2.0Hz),8.63(1H,s),8.49(1H,s),8.20-8.15(3H,m),7.60(1H,d,J=8.9Hz),4.37(2H,s),4.23(2H,t,J=6.9Hz),3.62-3.45(6H,m),2.08-2.02(2H,m),1.77-1.71(4H,m),1.45(6H,s).
MSm/z(M+H):471.

<Example 0402>

<0402-1>

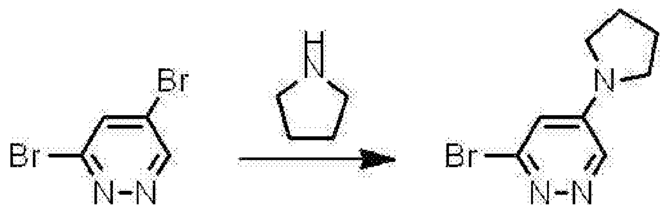

Pyrrolidine (100 μL) was added to a solution of 3,5-dibromopyridazine (30 mg) in tetrahydrofuran (1 mL), followed by stirring at room temperature overnight. The solvent was distilled off under reduced pressure, and the obtained residue was purified by silica gel column chromatography (ethyl acetate-methanol), thereby obtaining 3-bromo-5-(pyrrolidin-1-yl)pyridazine (20 mg).
MSm/z(M+H):228,230.

<0402-2>

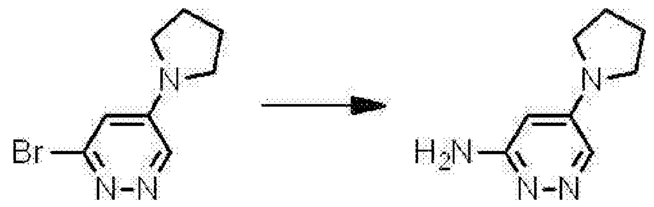

25% ammonia water (1 mL) and copper(I) oxide (5 mg) were added to a solution of 3-bromo-5-(pyrrolidin-1-yl)pyridazine (20 mg) in ethylene glycol (1 mL), followed by stirring at 150°C for 30 minutes using a microwave reaction apparatus. The reaction mixture was cooled to room temperature, and the solvent was distilled off under reduced pressure. The obtained residue was purified by silica gel column chromatography (ethyl acetate-methanol), thereby obtaining 5-(pyrrolidin-1-yl)pyridazine-3-amine (15 mg).
MSm/z(M+H):165.

<0402-3>

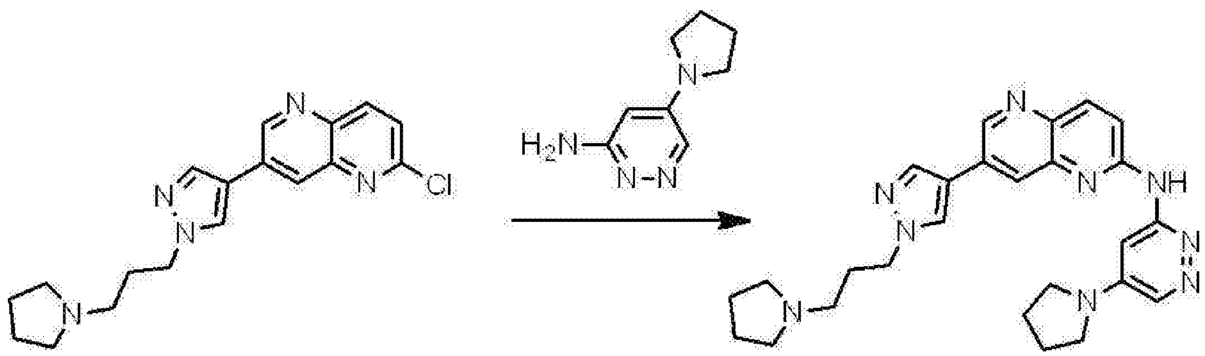

7-(1-(3-(Pyrrolidin-1-yl)propyl)-1H-pyrazol-4-yl)-N-(5-(pyrrolidin-1-yl)pyridazin-3-yl)-1,5-naphthyridine-2-amine was obtained as a pale yellow solid in the same manner as in Example 0290 except that 5-(pyrrolidin-1-yl)pyridazine-3-amine was used instead of the 5-(tert-butyl)pyridazine-3-amine used in Example 0290.
$^1$H-NMR(DMSO-$d_6$)δ:10.25(1H,s),9.00(1H,d,J=2.3Hz),8.49(1H,s),8.36(1H,d,J=2.6Hz),8.19-8.13(3H,m),7.90(1H,d,J=2.6Hz),7.64(1H,d,J=9.2Hz),4.21(2H,t,J=6.9Hz),3.50-3.41(4H,m),2.46-2.37(6H,m),2.05-1.98(6H,m),1.71-1.66(4H,m).
MSm/z(M+H):470.

<Example 0403>

<0403-1>

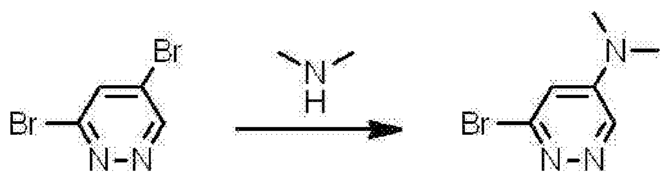

A 2.0 mol/L dimethylamine-tetrahydrofuran solution (200 μL) was added to a solution of 3,5-dibromopyridazine (30 mg) in tetrahydrofuran (1 mL), followed by stirring at room temperature overnight. The solvent was distilled off under reduced pressure, and the obtained residue was purified by silica gel column chromatography (ethyl acetate-methanol), thereby obtaining 6-bromo-N,N-dimethylpyridazine-4-amine (23 mg).
MSm/z(M+H): 202,204.

<0403-2>

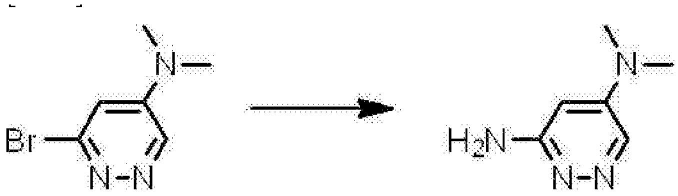

25% ammonia water (1 mL) and copper(I) oxide (5 mg) were added to a solution of 6-bromo-N,N-dimethylpyridazine-4-amine (23 mg) in ethylene glycol (1 mL), followed by stirring at 150°C for 30 minutes using a microwave reaction apparatus. The reaction mixture was cooled to room temperature, and the solvent was distilled off under reduced pressure. The obtained residue was purified by silica gel column chromatography (chloroform-methanol), thereby obtaining $N^5$,$N^5$-dimethylpyridazine-3,5-diamine (18 mg).
MSm/z(M+H):139.

<0403-3>

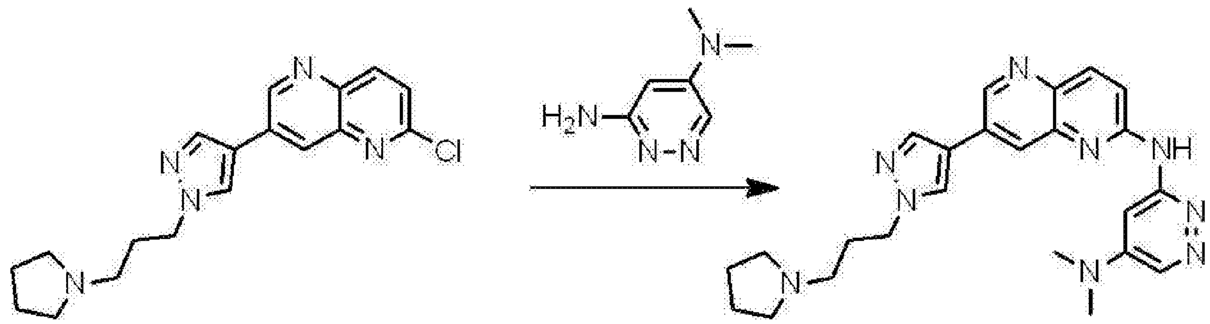

$N^5$,$N^5$-dimethyl-$N^3$-(7-(1-(3-(pyrrolidin-1-yl)propyl)-1H-pyrazol-4-yl)-1,5-naphthyridin-2-yl)pyridazine-3,5-diamine was obtained as a pale yellow solid in the same manner as in Example 0290 except that $N^5$,$N^5$-dimethylpyridazine-3,5-diamine was used instead of the 5-(tert-butyl)pyridazine-3-amine used in Example 0290.
$^1$H-NMR(DMSO-$d_6$)δ:10.29(1H,s),9.00(1H,d,J=2.0Hz),8.52(1H,d,J=2.6Hz),8.49(1H,s),8.18-8.14(3H,m),8.08(1H,d,J=2.6Hz),7.65(1H,d,J=9.2Hz),4.21(2H,t,J=6.9Hz),3.12(6H,s),2.44-2.37(6H,m),2.04-1.95(2H,m),1.72-1.65(4H,m).
MSm/z(M+H):444.

<Example 0404>

<0404-1>

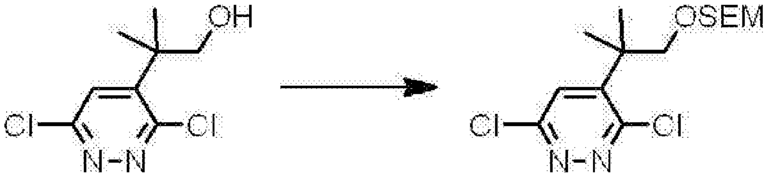

N,N-diisopropylethylamine (300 μL) was added to a solution of 2-(3,6-dichloropyridazin-4-yl)-2-methylpropan-1-ol (150 mg) in dichloromethane (3 mL), and 2-(chloromethoxy)ethyltrimethylsilane (150 μL) was added thereto under

ice-cooling, followed by stirring at room temperature for 30 minutes. Water was added to the reaction mixture. The organic layer was collected by separation, and dried over anhydrous sodium sulfate, and the solvent was distilled off under reduced pressure. The obtained residue was purified by silica gel column chromatography (hexane-ethyl acetate), thereby obtaining 3,6-dichloro-4-(2-methyl-1-((2-(trimethylsilyl)ethoxy)methoxy)propan-2-yl)pyridazine (170 mg) as brown oily substance.
MSm/z(M+H):351,353.

<0404-2>

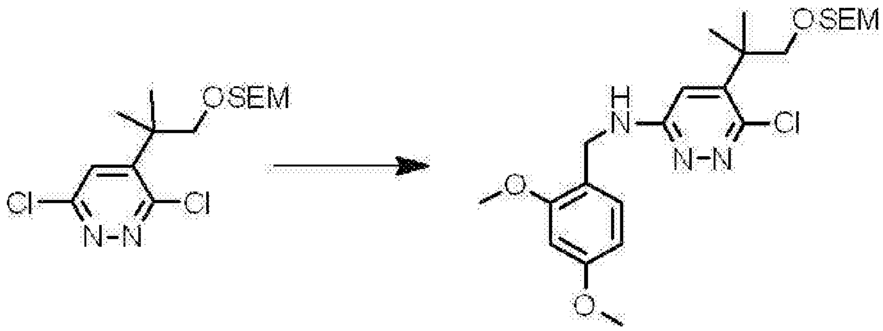

2,4-Dimethoxybenzylamine (200 μL) and 1,8-diazabicyclo[5.4.0]undeca-7-ene (400 μL) were added to a solution of 3,6-dichloro-4-(2-methyl-1-((2-(trimethylsilyl)ethoxy)methoxy)propan-2-yl)pyridazine (170 mg) in 1,4-dioxane (3 mL), followed by stirring at 150°C for 30 minutes using a microwave reaction apparatus. The reaction mixture was cooled to room temperature, and ethyl acetate was added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining 6-chloro-N-(2,4-dimethoxybenzyl)-5-(2-methyl-1-((2-(trimethylsilyl)ethoxy)methoxy)propan-2-yl)pyridazine-3-amine (110 mg) as brown oily substance.
MSm/z(M+H):482.

<0404-3>

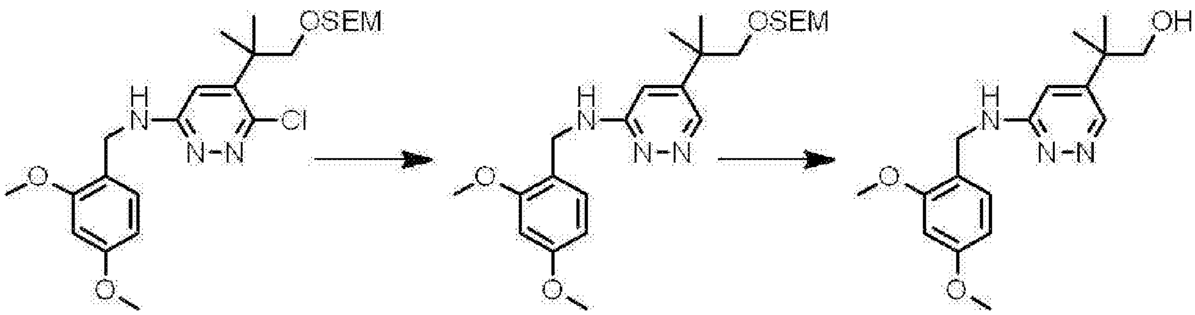

A mixture solution of 6-chloro-N-(2,4-dimethoxybenzyl)-5-(2-methyl-1-((2-(trimethylsilyl)ethoxy)methoxy)propan-2-yl)pyridazine-3-amine (110 mg) in methanol (10 mL)/acetic acid (1 mL) was reacted using a flow-type hydrogenation reaction apparatus (20 bar, 1.0 mL/min, 50°C, 10% Pd/C). The solvent was distilled off under reduced pressure, thereby obtaining N-(2,4-dimethoxybenzyl)-5-(2-methyl-1-((2-(trimethylsilyl)ethoxy)methoxy)propan-2-yl)pyridazine-3-amine as pale yellow oily substance.

A 4 mol/L hydrogen chloride/1,4-dioxane solution (5 mL) was added to the obtained N-(2,4-dimethoxybenzyl)-5-(2-methyl-1-((2-(trimethylsilyl)ethoxy)methoxy)propan-2-yl)pyridazine-3-amine, followed by stirring at room temperature for 30 minutes. The solvent was distilled off under reduced pressure, the obtained residue was neutralized by the addition of a saturated sodium hydrogen carbonate aqueous solution, and ethyl acetate was added thereto. The organic layer was collected by separation, washed with a saturated sodium chloride aqueous solution, and dried over anhydrous

sodium sulfate. The obtained solution was purified by silica gel column chromatography (hexane-ethyl acetate), thereby obtaining 2-(6-((2,4-dimethoxybenzyl)amino)pyridazin-4-yl)-2-methylpropan-1-ol (65 mg).
MSm/z(M+H):318.

<0404-4>

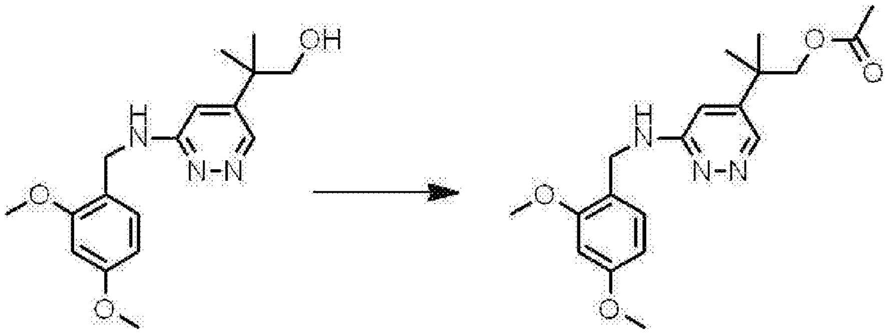

Acetic anhydride (100 μL) was added to a solution of 2-(6-((2,4-dimethoxybenzyl)amino)pyridazin-4-yl)-2-methylpropan-1-ol in acetic acid (500 μL), followed by stirring at room temperature for 30 minutes. The solvent was distilled off under reduced pressure, the obtained residue was neutralized by the addition of a saturated sodium hydrogen carbonate aqueous solution, and ethyl acetate was added thereto. The organic layer was collected by separation, washed with a saturated sodium chloride aqueous solution, and dried over anhydrous sodium sulfate. The obtained solution was purified by silica gel column chromatography (hexane-ethyl acetate), thereby obtaining 2-(6-((2,4-dimethoxybenzyl)amino)pyridazin-4-yl)-2-methylpropyl acetate (60 mg).
MSm/z(M+H):360.

<0404-5>

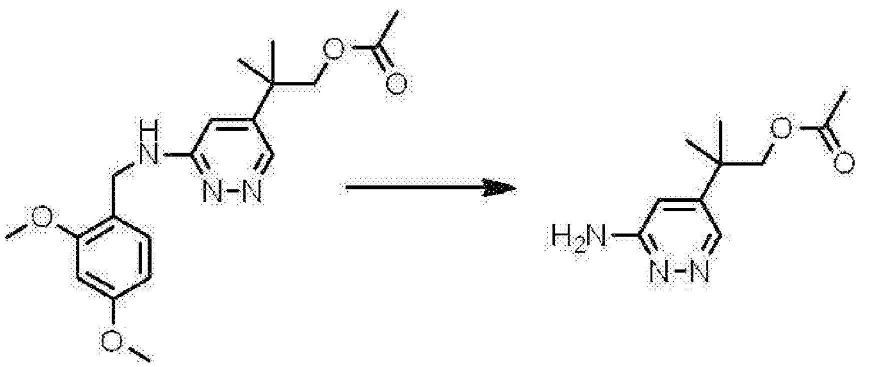

A solution of 2-(6-((2,4-dimethoxybenzyl)amino)pyridazin-4-yl)-2-methylpropyl acetate (60 mg) in trifluoroacetic acid (1 mL) was stirred at room temperature for 30 minutes. The solvent was distilled off under reduced pressure, triethylamine was added to the resultant product, and the solvent was distilled off under reduced pressure. The obtained residue was purified by silica gel column chromatography (hexane-ethyl acetate, NH silica), thereby obtaining 2-(6-aminopyridazin-4-yl)-2-methylpropyl acetate (30 mg).
MSm/z(M+H):210.

<0404-6>

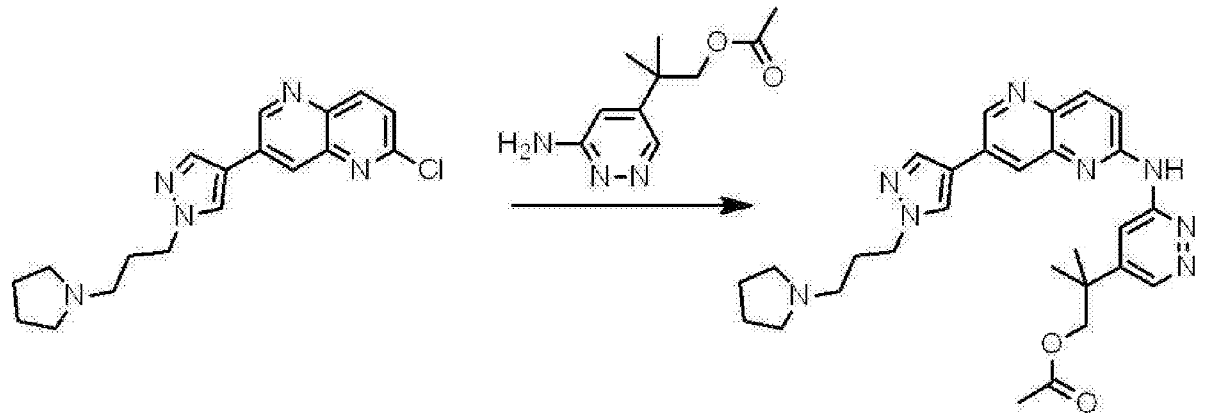

2-Methyl-2-(6-((7-(1-(3-(pyrrolidin-1-yl)propyl)-1H-pyrazol-4-yl)-1,5-naphthyridin-2-yl)amino)pyridazin-4-yl)propyl acetate was obtained as a pale yellow solid in the same manner as in Example 0290 except that 2-(6-aminopyridazin-4-yl)-2-methylpropyl acetate was used instead of the 5-(tert-butyl)pyridazine-3-amine used in Example 0290.
$^1$H-NMR(DMSO-$d_6$)δ:10.75(1H,s),9.05(1H,d,J=2.3Hz),9.02(1H,d,J=2.3Hz),8.89(1H,d,J=2.0Hz),8.50(1H,s),8.22(1H,d,J=9.2Hz),8.19(1H,s),8.12(1H,d,J=1.7Hz),7.70(1H,d,J=9.2Hz),4.29(2H,s),4.22(2H,t,J=6.9Hz),2.45-2.38(6H,m),2.04-1.96(5H,m),1.72-1.66(4H,m),1.42(6H,s).
MSm/z(M+H):515.

<Example 0405>

<0405-1>

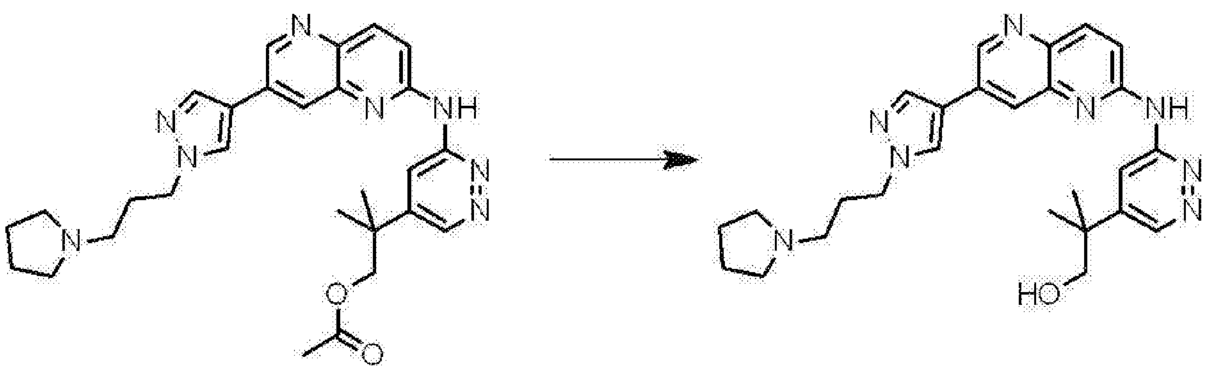

A2 mol/L sodium hydroxide aqueous solution (500 μL) wa added to a solution of 2-methyl-2-(6-((7-(1-(3-(pyrrolidin-1-yl)propyl)-1H-pyrazol-4-yl)-1,5-naphthyridin-2-yl)amino)pyridazin-4-yl)propyl acetate (8 mg) in methanol (500 μL), followed by stirring at room temperature for 30 minutes. The solvent was distilled off under reduced pressure, the obtained residue was neutralized by the addition of 2 mol/L hydrochloric acid, and ethyl acetate was added thereto. The organic layer was collected by separation, washed with a saturated sodium chloride aqueous solution, and dried over anhydrous sodium sulfate. The obtained solution was purified by silica gel column chromatography (ethyl acetate-methanol, NH silica), thereby obtaining 2-methyl-2-(6-((7-(1-(3-(pyrrolidin-1-yl)propyl)-1H-pyrazol-4-yl)-1,5-naphthyridin-2-yl)amino)pyridazin-4-yl)propan-1-ol(4.7 mg).
$^1$H-NMR(DMSO-$d_6$)δ:10.65(1H,s),9.04(1H,d,J=2.0Hz),8.95(1H,d,J=2.0Hz),8.76(1H,d,J=2.0Hz),8.49(1H,s),8.22(1H,d,J=9.2Hz),8.16(1H,s),8.10(1H,d,J=1.7Hz),7.76(1H,d,J=9.2Hz),4.93(1H,t,J=5.3Hz),4.22(2H,t,J=6.9Hz),3 .56(2H,d,J=5.3 Hz),2.45-2.38(6H,m),2.04-1.95(2H,m),1.71-1.67(4H,m),1.34(6H,s).
MSm/z(M+H):473.

<Example 0406>

<0406-1>

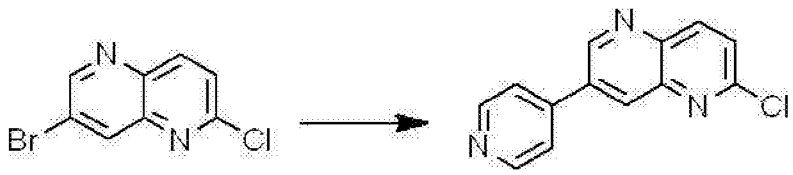

(Pyridin-4-yl)boronic acid (31 mg), bis(di-tert-butyl (4-dimethylaminophenyl)phosphine)dichloropalladium(II) (14 mg), and sodium carbonate (45 mg) were added to a mixture solution of 7-bromo-2-chloro-1,5-naphthyridine (50 mg) in 1,4-dioxane (5 mL)/water (0.5 mL), followed by stirring at 100°C for 2 hours in a nitrogen atmosphere. The reaction mixture was cooled to room temperature, and the solvent was distilled off under reduced pressure. The obtained residue was purified by silica gel column chromatography (hexane/ethyl acetate → ethyl acetate/methanol), and purified by silica gel column chromatography (hexane/ethyl acetate → ethyl acetate/methanol, NH silica), thereby obtaining 2-chloro-7-(pyridin-4-yl)-1,5-naphthyridine (32 mg).
$^1$H-NMR(DMSO-$d_6$)δ:9.50(1H,d,J=2.0Hz),8.86(1H,d,J=2.0Hz),8.77(2H,d,J=5.9Hz),8.57(1H,d,J=8.6Hz),8.03(2H,d,J=5.9Hz),7.92(1H,d,J=8.6Hz).

<0406-2>

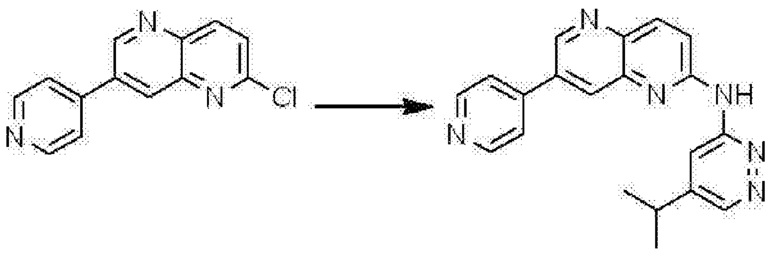

5-Isopropylpyridazine-3-amine (10 mg), tris(dibenzylideneacetone)dipalladium(0) (5 mg), 4,5-bis(diphenylphosphino)-9,9-dimethylxanthene (7 mg), and cesium carbonate (49 mg) were added to a solution of 2-chloro-7-(pyridin-4-yl)-1,5-naphthyridine (15 mg) in 1,4-dioxane (4 mL), followed by stirring at 140°C for 30 minutes using a microwave reaction apparatus. The reaction mixture was cooled to room temperature, purified by silica gel column chromatography (hexane/ethyl acetate → ethyl acetate/methanol), and purified by preparative thin layer silica gel chromatography (chloroform/methanol), thereby obtaining N-(5-isopropylpyridazin-3-yl)-7-(pyridin-4-yl)-1,5-naphthyridine-2-amine (4 mg) as a yellow solid.
$^1$H-NMR(DMSO-$d_6$)δ:10.84(1H,s),9.17(1H,d,J=2.0Hz),8.88(1H,d,J=2.0Hz),8.79(1H,s),8.75(2H,d,J=5.9Hz),8.50(1H,s),8.33(1H,d,J=9.2Hz),7.98(2H,d,J=5.9Hz),7.82(1H,d,J=9.2Hz),3.13-2.98(1H,m),1.33(6H,d,J=6.6Hz).
MSm/z(M+H):343.

<Example 0407>

<0407-1>

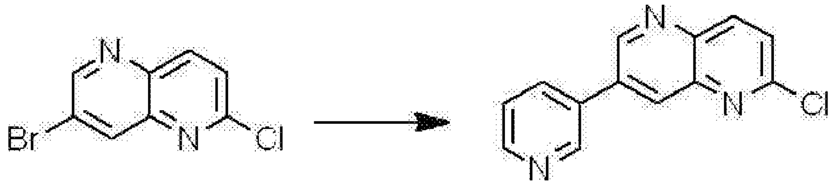

(Pyridin-3-yl)boronic acid (31 mg), bis(di-tert-butyl (4-dimethylaminophenyl)phosphine)dichloropalladium(II) (14 mg), and sodium carbonate (45 mg) were added to a mixture solution of 7-bromo-2-chloro-1,5-naphthyridine (50 mg) in 1,4-dioxane (5 mL)/water (0.5 mL), followed by stirring at 100°C for 2 hours in a nitrogen atmosphere. The reaction mixture was cooled to room temperature, and water and ethyl acetate were added thereto. The organic layer was collected by separation, and the solvent was distilled off under reduced pressure. The obtained residue was purified by silica gel column chromatography (hexane/ethyl acetate → ethyl acetate/methanol), thereby obtaining 2-chloro-7-(pyridin-3-yl)-1,5-naphthyridine (37 mg).
$^1$H-NMR(DMSO-$d_6$)δ:9.46(1H,d,J=2.6Hz),9.19(1H,d,J=1.3Hz),8.79(1H,d,J=2.6Hz),8.73-8.69(1H,m),8.55(1H,d,J=8.6Hz),8.43-8.38(1H,m),7.89(1H,d,J=8.6Hz),7.65-7.58(1H,m).

<0407-2>

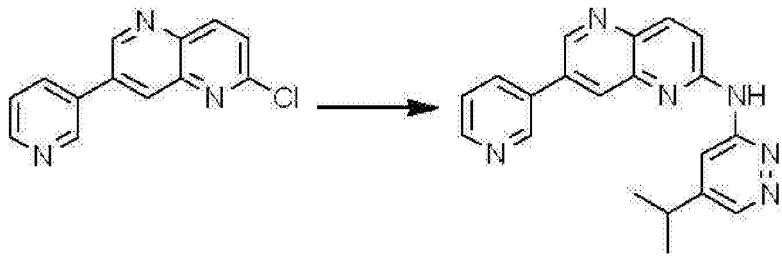

5-Isopropylpyridazine-3-amine (14 mg), tris(dibenzylideneacetone)dipalladium(0) (7 mg), 4,5-bis(diphenylphosphino)-9,9-dimethylxanthene (9 mg) and cesium carbonate (65 mg) were added to a solution of 2-chloro-7-(pyridin-3-yl)-1,5-naphthyridine (20 mg) in 1,4-dioxane (4 mL), followed by stirring at 140°C for 30 minutes using a microwave reaction apparatus. The reaction mixture was cooled to room temperature, and water and ethyl acetate were added thereto. The organic layer was collected by separation, and the solvent was distilled off under reduced pressure. The reaction residue was purified by silica gel column chromatography (hexane/ethyl acetate → ethyl acetate/methanol), purified by silica gel column chromatography (chloroform/methanol), purified by preparative thin layer silica gel chromatography (chloroform/methanol), and purified by preparative thin layer silica gel chromatography (chloroform/methanol), thereby obtaining N-(5-isopropylpyridazin-3-yl)-7-(pyridin-3-yl)-1,5-naphthyridine-2-amine (5 mg) as a white solid.
$^1$H-NMR(DMSO-$d_6$)δ:10.82(1H,s),9.16-9.11(2H,m),8.88(1H,d,J=2.0Hz),8.79(1H,d,J=2.0Hz),8.71-8.67(1H,m),8.46(1H,d,J=2.0Hz),8.38-8.29(2H,m),7.80(1H,d,J=9.2Hz),7.63-7.57(1H,m),3.12-2.97(1H,m),1.32(6H,d,J=6.6Hz).
MSm/z(M+H):343.

<Example 0408> (not in accordance with the present invention)

<0408-1>

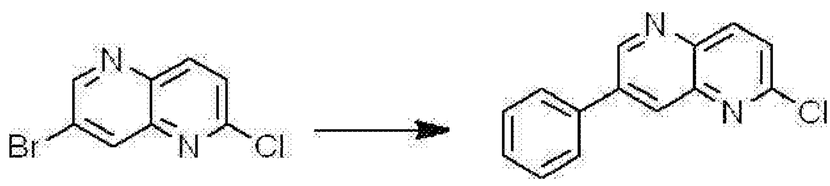

Phenylboronic acid (30 mg), bis(di-tert-butyl (4-dimethylaminophenyl)phosphine)dichloropalladium(II) (14 mg), and sodium carbonate (45 mg) were added to a mixture solution of 7-bromo-2-chloro-1,5-naphthyridine (50 mg) in 1,4-dioxane (5 mL)/water (0.5 mL), followed by stirring at 100°C for 2.5 hours in a nitrogen atmosphere. The reaction mixture was cooled to room temperature, and water and ethyl acetate were added thereto. The organic layer was collected by separation, the solvent was distilled off under reduced pressure, and the obtained residue was purified by silica gel column chromatography (hexane/ethyl acetate), thereby obtaining 2-chloro-7-phenyl-1,5-naphthyridine (55 mg).
$^1$H-NMR(DMSO-$d_6$)δ:9.42(1H,d,J=2.0Hz),8.65(1H,d,J=2.0Hz),8.53(1H,d,J=9.2Hz),8.00-7.95(2H,m),7.86(1H,d,J=9.2Hz),7.63-7.48(3H,m).

<0408-2>

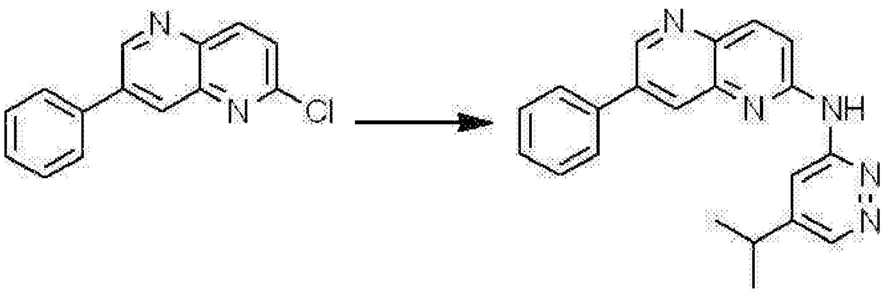

5-Isopropylpyridazine-3-amine (14 mg), tris(dibenzylideneacetone)dipalladium(0) (7 mg), 4,5-bis(diphenylphosphino)-9,9-dimethylxanthene (9 mg), and cesium carbonate (58 mg) were added to a solution of 2-chloro-7-phenyl-1,5-naphthyridine (20 mg) in 1,4-dioxane (4 mL), followed by stirring at 140°C for 30 minutes using a microwave reaction apparatus. The reaction mixture was cooled to room temperature, and water and ethyl acetate were added thereto. The organic layer was collected by separation, the solvent was distilled off under reduced pressure, and the obtained residue was purified by silica gel column chromatography (chloroform/methanol), purified by preparative thin layer silica gel chromatography (chloroform/methanol), and purified by preparative thin layer silica gel chromatography (chloroform/methanol, NH silica), thereby obtaining N-(5-isopropylpyridazin-3-yl)-7-phenyl-1,5-naphthyridine-2-amine (5 mg) as a pale yellow solid.
$^1$H-NMR(DMSO-$d_6$)δ:12.80(1H,s),11.10(1H,d,J=2.6Hz),10.89(1H,d,J=2.0Hz),10.81(1H,d,J=2.0Hz),10.37-10.29(2H,m),9.96-9.90(2H,m),9.80(1H,d,J=9.2Hz),9.64-9.47(3H,m),5.12-4.99(1H,m),3.34(6H,d,J=6.6Hz).
MSm/z(M+H):342.

<Example 0409>

<0409-1>

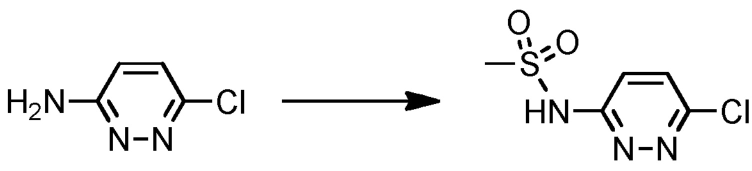

Methanesulfonyl chloride (0.6 mL) was added to a mixture of 6-chloropyridazine-3-amine (500 mg) and pyridine (5 mL) at a temperature of from 0°C to 5°C, followed by stirring at a temperature of from 0°C to 5°C for 15 minutes, and stirring at 40°C for 30 minutes. Methanesulfonyl chloride (0.1 mL) was added to the reaction mixture, followed by stirring at 40°C for 30 minutes. The solvent was distilled off under reduced pressure, and the obtained residue was purified by silica gel column chromatography (methanol-chloroform), thereby obtaining N-(6-chloropyridazin-3-yl)methane sulfonamide (257 mg).

MSm/z(M+H):208.

<0409-2>

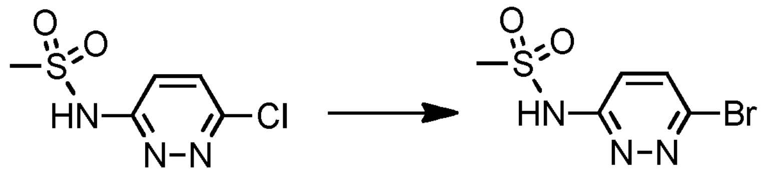

A mixture of N-(6-chloropyridazin-3-yl)methane sulfonamide (150 mg) and 48% hydrobromic acid (5 mL) was stirred at 80°C for 8.5 hours. The reaction mixture was cooled to room temperature, followed by allowing to stand overnight, and stirring at 100°C for 1 hour. The reaction mixture was cooled to room temperature, and the solvent was distilled off under reduced pressure. After the obtained residue was neutralized by the addition of a 1 mol/L sodium hydroxide aqueous solution, chloroform and methanol were added thereto, the insolubles were filtered off, and the solvent was distilled off under reduced pressure. The obtained residue was purified by silica gel column chromatography (hexane-ethyl acetate), thereby obtaining N-(6-bromopyridazin-3-yl)methane sulfonamide (95 mg).
MSm/z(M+H):254.

<0409-3>

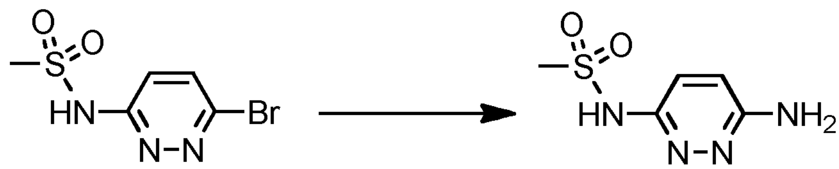

N-(6-aminopyridazin-3-yl)methane sulfonamide was obtained in the same manner as in Example 0403-2 except that N-(6-bromopyridazin-3-yl)methane sulfonamide was used instead of the 6-bromo-N,N-dimethylpyridazine-4-amine used in Example 0403-2.
MSm/z(M+H):189.

<0409-4>

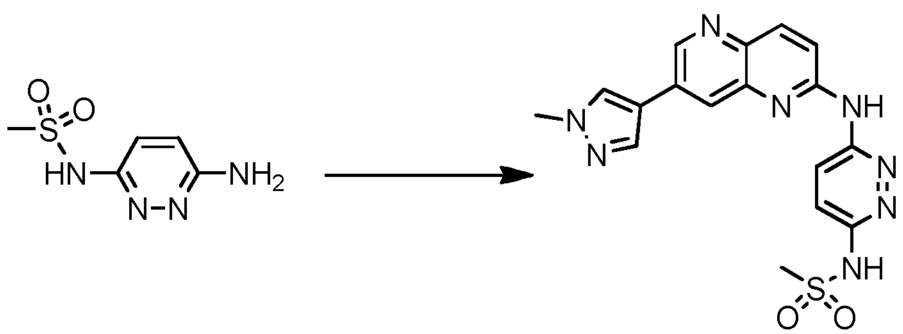

N-(6-((7-(1-methyl-1H-pyrazol-4-yl)-1,5-naphthyridin-2-yl)amino)pyridazin-3-yl)methane sulfonamide was obtained in the same manner as in Example 0145-1 except that N-(6-aminopyridazin-3-yl)methane sulfonamide was used instead of the 5-isopropylpyridazine-3-amine used in Example 0145-1, and 2-dicyclohexylphosphino-2',4',6'-triisopropylbiphenyl was used instead of the 4,5-bis(diphenylphosphino)-9,9-dimethylxanthene used in Example 0145-1.

$^1$H-NMR(DMSO-$d_6$)δ:10.57(1H,s),9.02(1H,d,J=2.0Hz),8.79(1H,d,J=9.2Hz),8.46(1H,s),8.24(1H,d,J=2.0Hz),8.19(1H,d,J=9.2Hz),8.15(1H,s), 7.57(2H,d,J=8.6Hz),3.91(3H,s),3.15(3H,s).
MSm/z(M+H):397.

<Example 0410> (not in accordance with the present invention)

<0410-1>

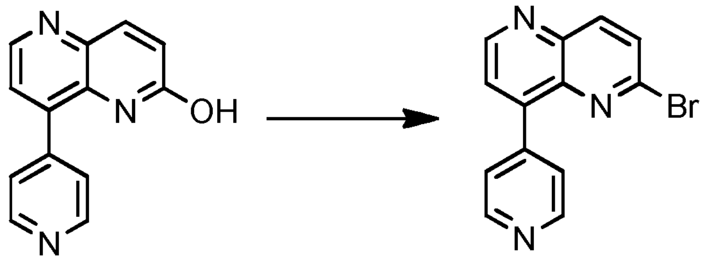

2-Bromo-8-(pyridin-4-yl)-1,5-naphthyridine was obtained in the same manner as in Example 0004-3 except that 8-(pyridin-4-yl)-1,5-naphthyridin-2-ol was used instead of the 8-bromo-2-methoxy-1,5-naphthyridine used in Example 0004-3.
MSm/z(M+H):286.

<0410-2>

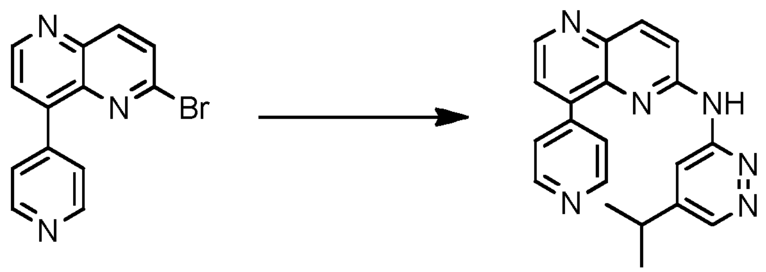

N-(5-isopropylpyridazin-3-yl)-8-(pyridin-4-yl)-1,5-naphthyridine-2-amine was obtained in the same manner as in Example 0145-1 except that 2-bromo-8-(pyridin-4-yl)-1,5-naphthyridine was used instead of the 2-chloro-7-(1-methyl-1H-pyrazol-4-yl)-1,5-naphthyridine used in Example 0145-1. $^1$H-NMR(DMSO-$d_6$)δ:10.80(1H,s),8.82(1H,d,J=4.6Hz),8.79(1H,d,J=2.0Hz),8.74-8.73(2H,m),8.34(1H,d,J=8.6Hz),8.25(1H,d,J=2.0Hz),7.74-7.71(3H,m),7.66(1H,d,J=4.6Hz),1.01(6H,d,J=7.3Hz).
MSm/z(M+H):343.

<Example 0411>

<0411-1>

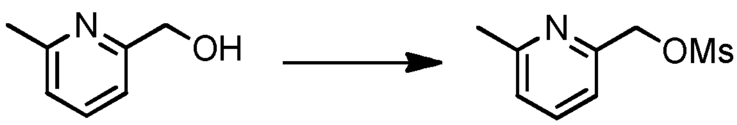

Triethylamine (1 mL) was added to a solution of (6-methylpyridin-2-yl)methanol (302 mg) in tetrahydrofuran (5.8 mL) at room temperature, and methanesulfonyl chloride (0.28 mL) was added thereto at a temperature of from 0°C to 5°C, followed by stirring at room temperature for 2 hours. The insolubles were filtered off using celite, and the solvent was distilled off under reduced pressure, thereby obtaining (6-methylpyridin-2-yl)methyl methanesulfonate (555 mg) as brown oily substance.
MSm/z(M+H):202.

<0411-2>

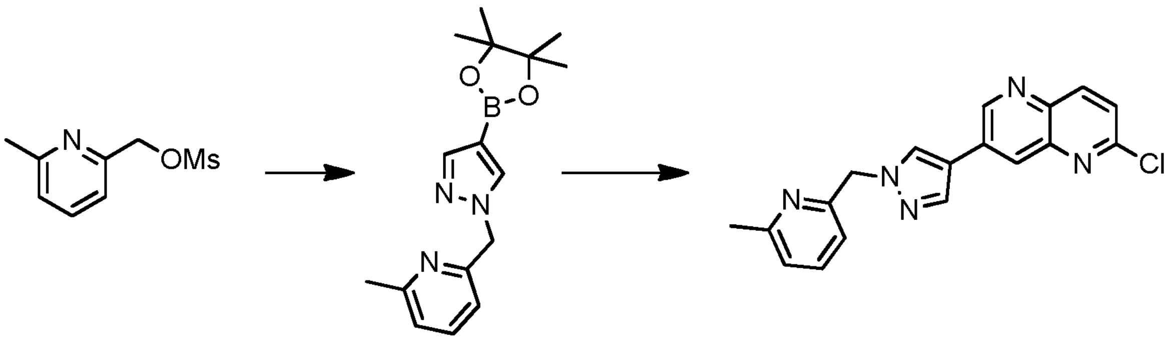

4-(4,4,5,5-Tetramethyl-1,3,2-dioxaborolan-2-yl)-1H-pyrazole (195 mg) and potassium carbonate (275 mg) were added to a solution of (6-methylpyridin-2-yl)methyl methanesulfonate (408 mg) in acetonitrile (2 mL), followed by stirring at 80°C for 4.5 hours. The resultant product was allowed to stand for 16 hours, followed by stirring at 80°C for 6 hours. The reaction mixture was cooled to room temperature, the insolubles were filtered off, and the solvent was distilled off under reduced pressure, thereby obtaining brown oily substance (385 mg). A mixture of the obtained brown oily substance (167 mg), 7-bromo-2-chloro-1,5-naphthyridine (50 mg), sodium carbonate (43 mg), bis(di-tert-butyl (4-dimethylaminophenyl)phosphine)dichloropalladium(II) (14 mg), 1,4-dioxane (2.1 mL), and water (0.21 mL) was stirred at 100°C for 1 hour in a nitrogen atmosphere. The reaction mixture was cooled to room temperature, the insolubles were filtered off, and the solvent was distilled off under reduced pressure. The obtained residue was purified by silica gel column chromatography (ethyl acetate-hexane), thereby obtaining 2-chloro-7-(1-((6-methylpyridin-2-yl)methyl)-1H-pyrazol-4-yl)-1,5-naphthyridine (73 mg) as a pale yellow solid.
MSm/z(M+H):336.

<0411-3>

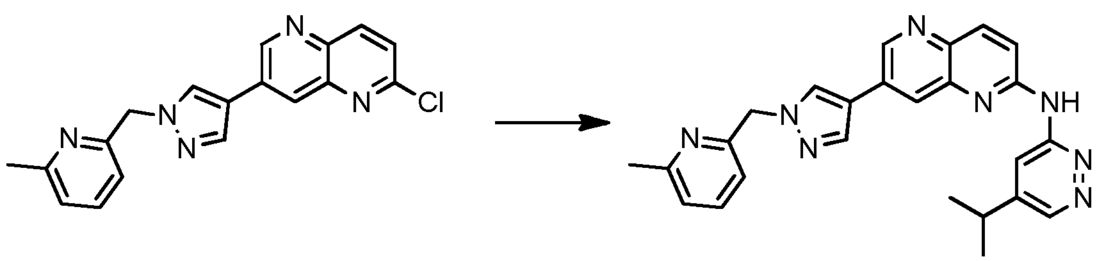

A mixture of 2-chloro-7-(1-((6-methylpyridin-2-yl)methyl)-1H-pyrazol-4-yl)-1,5-naphthyridine (36.3 mg), 5-isopropylpyridazine-3-amine (16.4 mg), tris(dibenzylideneacetone)dipalladium(0) (11.2 mg), 4,5-bis(diphenylphosphino)-9,9-dimethylxanthene (13.2 mg), cesium carbonate (70.5 mg), and 1,4-dioxane (1.5 mL) was stirred at 140°C for 30 minutes using a microwave reaction apparatus. The reaction mixture was cooled to room temperature, was purified by silica gel column chromatography (methanol-ethyl acetate, NH silica), the obtained solid matter was suspended in a mixed solvent of ethyl acetate-hexane, and the solid matter was collected by filtration, thereby obtaining N-(5-isopropylpyridazin-3-yl)-7-(1-((6-methylpyridin-2-yl)methyl)-1H-pyrazol-4-yl)-1,5-naphthyridine-2-amine (12.1 mg) as a yellow solid.

$^1$H-NMR($CDCl_3$)δ:8.94(1H,d,J=2.0Hz),8.81(2H,s),8.24(1H,d,J=9.2Hz),8.12(1H,d,J=2.0Hz),8.03(2H,d,J=1.3Hz),7.60(1H,d,J=2.0Hz),7.56(1H,d,J=7.9Hz),7.43(1H,d,J=8.6Hz),7.11(1H,d,J=7.9Hz),6.95(1H,d,J=7.9Hz),5.50(2H,s),3.06-3.02(1H,m),2.58(3H,s),1.41(6H,d,J=7.3Hz).
MSm/z(M+H):437.

<Example 0412>

<0412-1>

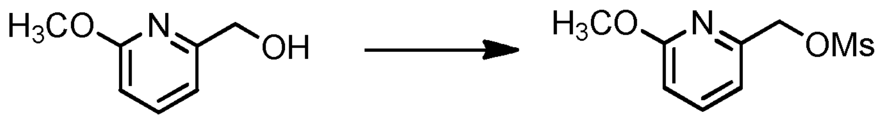

(6-Methoxypyridin-2-yl)methyl methanesulfonate was obtained as brown oily substance in the same manner as in Example 0411-1 except that (6-methoxypyridin-2-yl)methanol was used instead of the (6-methylpyridin-2-yl)methanol used in Example 0411-1.
MSm/z(M+H):218.

<0412-2>

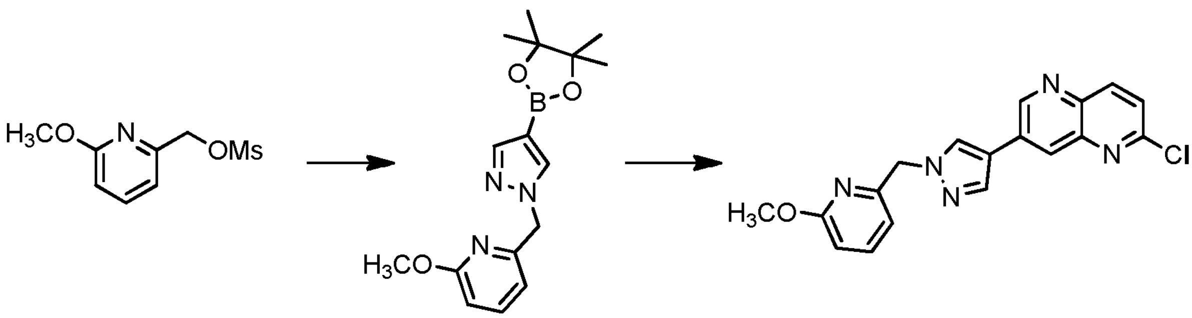

4-(4,4,5,5-Tetramethyl-1,3,2-dioxaborolan-2-yl)-1H-pyrazole (194 mg) and potassium carbonate (275 mg) were added to a solution of (6-methoxypyridin-2-yl)methyl methanesulfonate (432 mg) in acetonitrile (4 mL), followed by stirring at 80°C for 17.5 hours. The reaction mixture was cooled to room temperature, the insolubles were filtered off, and the solvent was distilled off under reduced pressure, thereby obtaining brown oily substance (366 mg).

A mixture of the obtained brown oily substance (157 mg), 7-bromo-2-chloro-1,5-naphthyridine (50 mg), sodium carbonate (44 mg), bis(di-tert-butyl (4-dimethylaminophenyl)phosphine)dichloropalladium(II) (16 mg), 1,4-dioxane (2.1 mL), and water (0.21 mL) was stirred at 100°C for 1 hour in a nitrogen atmosphere. The reaction mixture was cooled to room temperature, the insolubles were filtered off, and the solvent was distilled off under reduced pressure. The obtained solid matter was suspended in methanol-ethyl acetate-hexane, and the solid matter was collected by filtration, thereby obtaining 2-chloro-7-(1-((6-methoxypyridin-2-yl)methyl)-1H-pyrazol-4-yl)-1,5-naphthyridine (43 mg) as a pale yellow solid.
MSm/z(M+H):352.

<0412-3>

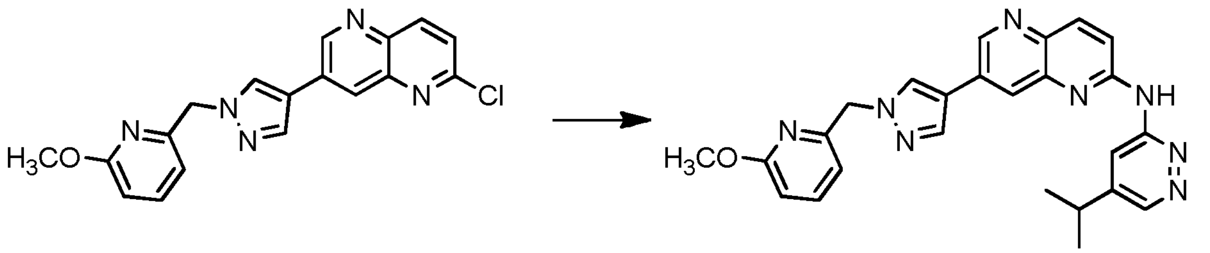

N-(5-isopropylpyridazin-3-yl)-7-(1-((6-methoxypyridin-2-yl)methyl)-1H-pyrazol-4-yl)-1,5-naphthyridine-2-amine was obtained as a yellow solid in the same manner as in Example 0411-3 except that 2-chloro-7-(1-((6-methoxypyridin-2-yl)methyl)-1H-pyrazol-4-yl)-1,5-naphthyridine was used instead of the 2-chloro-7-(1-((6-methylpyridin-2-yl)methyl)-1H-pyrazol-4-yl)-1,5-naphthyridine used in Example 0411-3.
$^1$H-NMR($CDCl_3$)$\delta$:8.95(1H,d,J=2.0Hz),8.81-8.80(2H,m),8.24(1H,d,J=9.2Hz),8.12(1H,d,J=2.0Hz),8.04(2H,d,J=13.2Hz),7.61-7.43(1H,m),6.73-6.69(3H,m),5.42(2H,s),3.93(3H,s),3.06-3.04(1H,m),1.41(6H,d,J=7.3Hz).
MSm/z(M+H):453.

<Example 0413>

<0413-1>

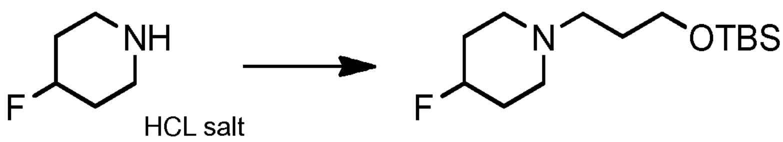


60% sodium hydride (211 mg) was added to a mixture of 4-fluoropiperidine hydrochloride (281 mg) and tetrahydrofuran (4 mL) under ice-cooling, followed by stirring at room temperature for 30 minutes. The reaction mixture was cooled by ice, and (3-bromopropoxy) (tert-butyl)dimethylsilane (0.7 mL) was added thereto, followed by stirring at room temperature for 42 hours. After the reaction mixture was cooled by ice, a saturated sodium hydrogen carbonate aqueous solution, water, and ethyl acetate were added thereto, and the organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate, NH silica), thereby obtaining 1-(3-((tert-butyldimethylsilyl)oxy)propyl)-4-fluoropiperidine (473 mg) as colorless oily substance.
$^1$H-NMR($CDCl_3$)$\delta$:3.69(2H,t,J=5.6Hz),3.60(1H,t,J=6.3Hz),3.47(2H,t,J=6.3Hz),2.53-2.51(2H,m),2.37-2.31(2H,m),1.99(2H,t,J=5.9Hz),1.82-1.80(2H,m),1.70-1.61(2H,m),0.85(9H,s),0.02(6H,s).

<0413-2>

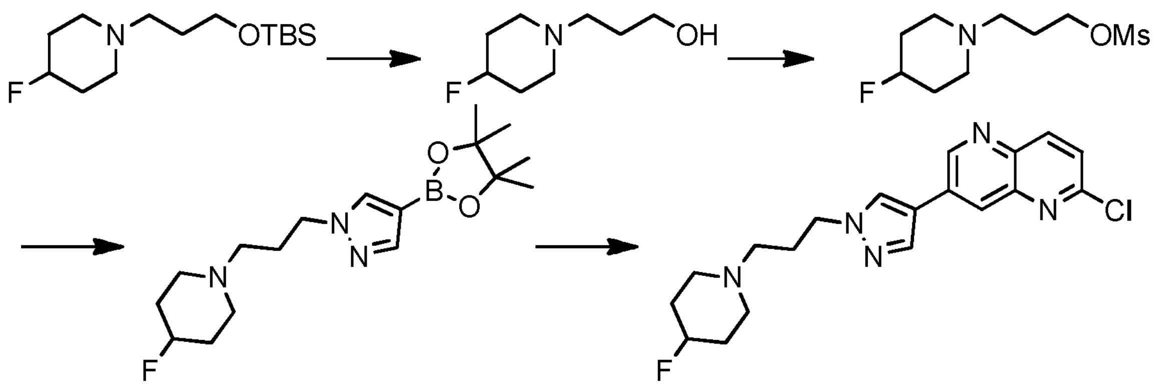

A 4 mol/L hydrogen chloride/1,4-dioxane solution (1 mL) was added to a mixture of 1-(3-((tert-butyldimethylsilyl)oxy)propyl)-4-fluoropiperidine (473 mg), and methanol (1 mL) at 0°C. The reaction mixture was heated to room temperature, followed by stirring for 30 minutes. The solvent was distilled off under reduced pressure, and methanol and a saturated sodium hydrogen carbonate aqueous solution were added to the obtained oily substance, followed by stirring at room temperature for 5 minutes. The solvent was distilled off under reduced pressure, methanesulfonyl chloride (0.2 mL) was added to a mixture of the obtained residue, tetrahydrofuran (4.1 mL), and triethylamine (0.72 mL), followed by stirring at room temperature for 3.5 hours. The insolubles were filtered off, and the solvent was distilled off under reduced pressure. 4-(4,4,5,5-tetramethyl-1,3,2-dioxaborolan-2-yl)-1H-pyrazole (196 mg) and potassium carbonate (281 mg) were added to a solution of the obtained residue in acetonitrile (4 mL), followed by stirring at 80°C for 17.5 hours. The reaction mixture was cooled to room temperature, the insolubles were filtered off, and the solvent was distilled off under reduced pressure. A mixture of the obtained residue, 7-bromo-2-chloro-1,5-naphthyridine (74.5 mg), sodium carbonate (65.7 mg), bis(di-tert-butyl (4-dimethylaminophenyl)phosphine)dichloropalladium(II) (22.8 mg), 1,4-dioxane (3 mL), and water (0.3 mL) was stirred at 80°C for 1 hour in a nitrogen atmosphere. The reaction mixture was cooled to room temperature, the insolubles were filtered off, and the solvent was distilled off under reduced pressure. The obtained residue was purified by silica gel column chromatography (hexane-ethyl acetate, NH silica), thereby obtaining 2-chloro-7-(1-(3-(4-fluoropiperidin-1-yl)propyl)-1H-pyrazol-4-yl)-1,5-naphthyridine (72 mg) as yellow oily substance.
MSm/z(M+H):374.

<0413-3>

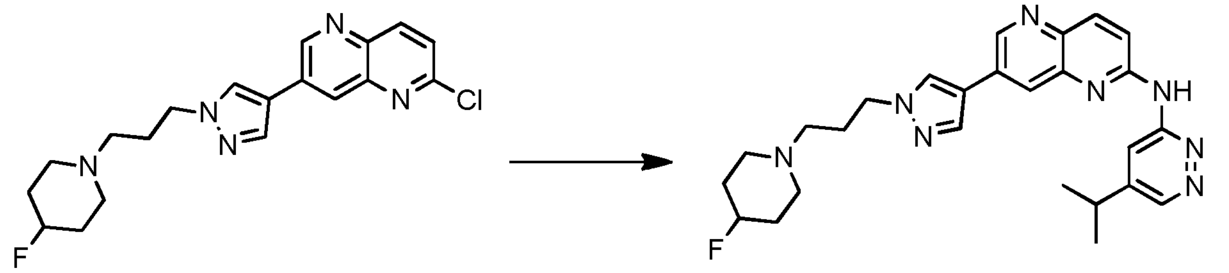

7-(1-(3-(4-Fluoropiperidin-1-yl)propyl)-1H-pyrazol-4-yl)-N-(5-isopropylpyridazin-3-yl)-1,5-naphthyridine-2-amine was obtained as a pale yellow solid in the same manner as in Example 0411-3 except that 2-chloro-7-(1-(3-(4-fluoropiperidin-1-yl)propyl)-1H-pyrazol-4-yl)-1,5-naphthyridine was used instead of the 2-chloro-7-(1-((6-methylpyridin-2-yl)methyl)-1H-pyrazol-4-yl)-1,5-naphthyridine used in Example 0411-3.
$^1$H-NMR($CDCl_3$)δ:8.93(1H,d,J=2.0Hz),8.84-8.81(2H,m),8.24(1H,d,J=8.6Hz),8.10(1H,d,J=2.0Hz),7.97(1H,s),7.86(1H,s),7.67-7.38(1H,m),4.76-4.60(1H,m),4.29(2H,t,J=6.9Hz),3.07-3.05(1H,m),2.58-2.56(2H,m),2.38-2.36(4H,m),2.14-2.12(2H,m),1.93-1.87(4H,m), 1.42(6H,d,J=6.6Hz).
MSm/z(M+H):475.

<Example 0414>

<0414-1>

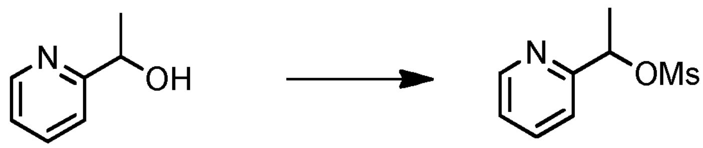

1-(Pyridin-2-yl)ethyl methanesulfonate was obtained as brown oily substance in the same manner as in Example 0411-1 except that 1-(pyridin-2-yl)ethanol was used instead of the (6-methylpyridin-2-yl)methanol used in Example 0411-1.
MSm/z(M+H):202.

<0414-2>

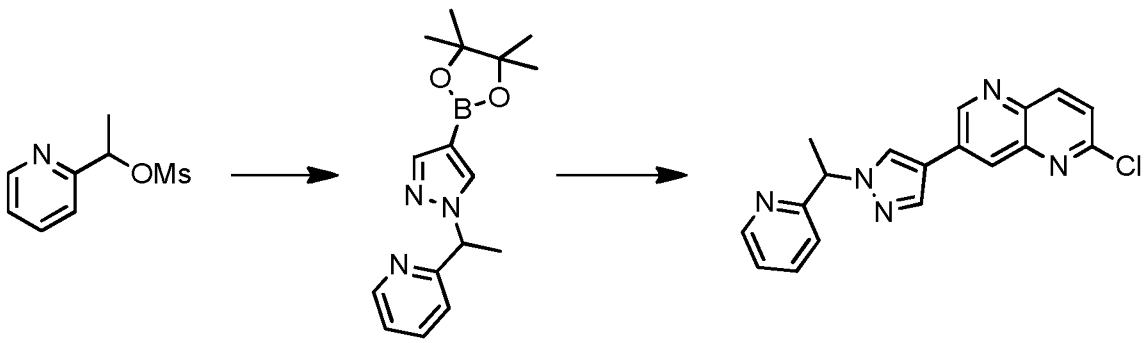

2-Chloro-7-(1-(1-(pyridin-2-yl)ethyl)-1H-pyrazol-4-yl)-1,5-naphthyridine was obtained as pale yellow oily substance in the same manner as in Example 0412-2 except that 1-(pyridin-2-yl)ethyl methanesulfonate was used instead of the (6-methoxypyridin-2-yl)methyl methanesulfonate used in Example 0412-2.
MSm/z(M+H):336.

<0414-3>

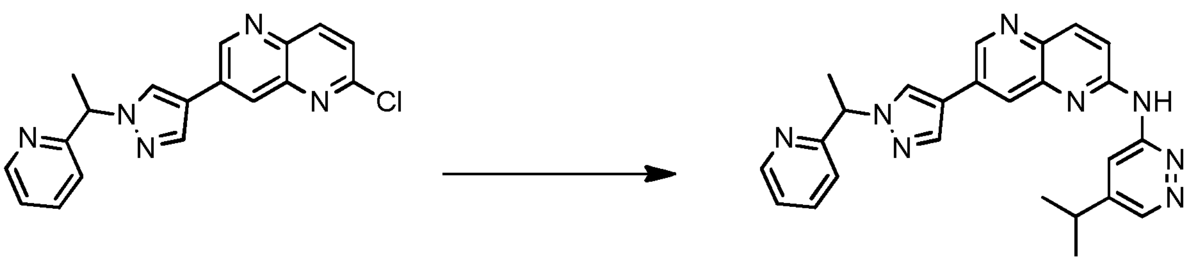

N-(5-isopropylpyridazin-3-yl)-7-(1-(1-(pyridin-2-yl)ethyl)-1H-pyrazol-4-yl)-1,5-naphthyridine-2-amine was obtained as a pale yellow solid in the same manner as in Example 0411-3 except that 2-chloro-7-(1-(1-(pyridin-2-yl)ethyl)-1H-pyrazol-4-yl)-1,5-naphthyridine was used instead of the 2-chloro-7-(1-((6-methylpyridin-2-yl)methyl)-1H-pyrazol-4-yl)-1,5-naphthyridine used in Example 0411-3.
$^1$H-NMR($CDCl_3$)δ:8.94(1H,d,J=2.0Hz),8.81(2H,s),8.63-8.61(1H,m),8.24(1H,d,J=9.2Hz),8.12(1H,d,J=1.3Hz),8.08(1H,s),8.

01(1H,s),7.70-7.68(1H,m),7.46(1H,d,J=8.6Hz),7.21-7.18(2H,m),5.73-5.71(1H,m),3.07-3.05(1H,m),2.05(3H,d,J=3.6Hz),1.42(6H,d,J=6.6Hz).
MSm/z(M+H):437.

<Example 0415>

<0415-1>

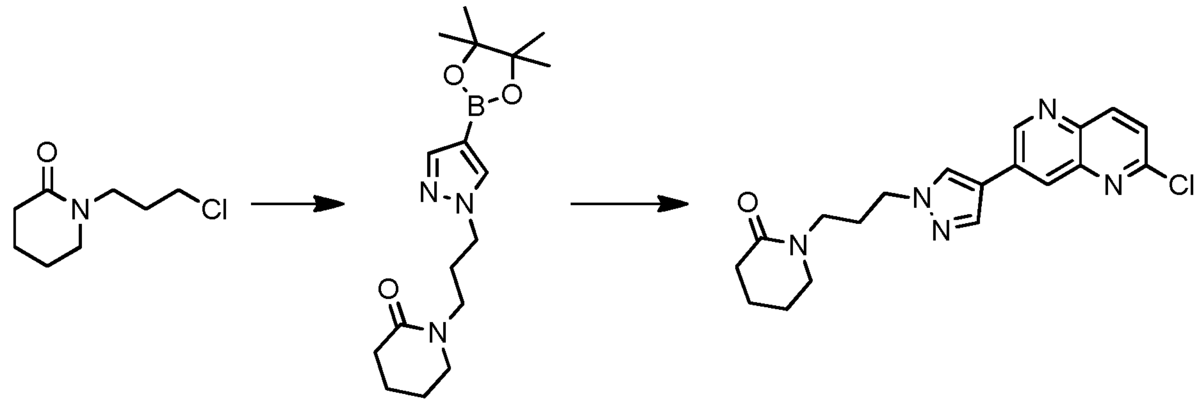

Sodium iodide (33 mg), cesium carbonate (701 mg), and 4-(4,4,5,5-tetramethyl-1,3,2-dioxaborolan-2-yl)-1H-pyrazole (231 mg) were added to a solution of 1-(3-chloropropyl)piperidin-2-one (189 mg) in 1,4-dioxane (3.6 mL), followed by stirring at 80°C for 16 hours. The reaction mixture was cooled to room temperature, the insolubles were filtered off, and the solvent was distilled off under reduced pressure, thereby obtaining brown oily substance (376 mg).

A mixture of the obtained brown oily substance (155 mg), 7-bromo-2-chloro-1,5-naphthyridine (76 mg), sodium carbonate (65 mg), bis(di-tert-butyl (4-dimethylaminophenyl)phosphine)dichloropalladium(II) (23 mg), 1,4-dioxane (3.1 mL), and water (0.31 mL) was stirred at 100°C for 1.5 hours in a nitrogen atmosphere. The reaction mixture was cooled to room temperature, the insolubles were filtered off, and the solvent was distilled off under reduced pressure. The obtained residue was purified by silica gel column chromatography (hexane-ethyl acetate-methanol, NH silica), thereby obtaining 1-(3-(4-(6-chloro-1,5-naphthyridin-3-yl)-1H-pyrazol-1-yl)propyl)piperidin-2-one (24 mg) as a pale yellow solid.
MSm/z(M+H):370.

<0415-2>

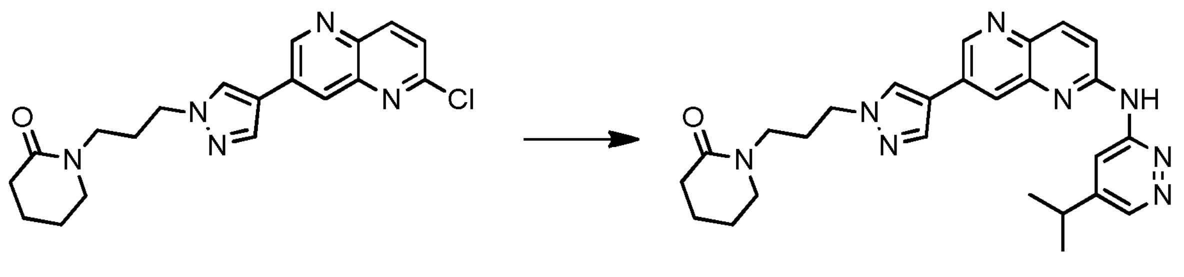

1-(3-(4-(6-((5-Isopropylpyridazin-3-yl)amino)-1,5-naphthyridin-3-yl)-1H-pyrazol-1-yl)propyl)piperidin-2-one was obtained as a yellow solid in the same manner as in Example 0411-3 except that 1-(3-(4-(6-chloro-1,5-naphthyridin-3-yl)-1H-pyrazol-1-yl)propyl)piperidin-2-one was used instead of the 2-chloro-7-(1-((6-methylpyridin-2-yl)methyl)-1H-pyrazol-4-yl)-1,5-naphthyridine used in Example 0411-3.
$^1$H-NMR($CDCl_3$)δ:9.05(1H,brs),8.94(1H,d,J=1.3Hz),8.85(2H,d,J=17.2Hz),8.24(1H,d,J=9.2Hz),8.11(1H,d ,J=1.3Hz),7.97(2H,d,J=1.3Hz),7.57(1H,d,J=9.2Hz),4.27(2H,t,J=6.6Hz),3.49(2H,t,J=6.6Hz),3.27(2H,t,J=5.3Hz),3.08-3.04(1H,m),2.35(2H,t,J=5.9Hz),2.25-2.21(2H,m),1.78-1.74(4H,m),1.43(6H,d,J=6.6Hz).
MSm/z(M+H):471.

<Example 0416>

<0416-1>

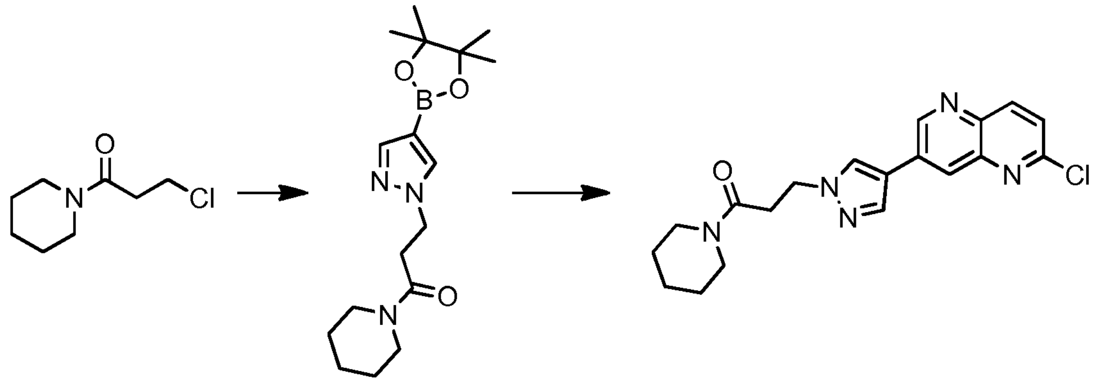

3-(4-(6-Chloro-1,5-naphthyridin-3-yl)-1H-pyrazol-1-yl)-1-(piperidin-1-yl)propan-1-one was obtained as a pale yellow solid in the same manner as in Example 0415-1 except that 3-chloro-1-(piperidin-1-yl)propan-1-one was used instead of the 1-(3-chloropropyl)piperidin-2-one used in Example 0415-1.
MSm/z(M+H):370.

<0416-2>

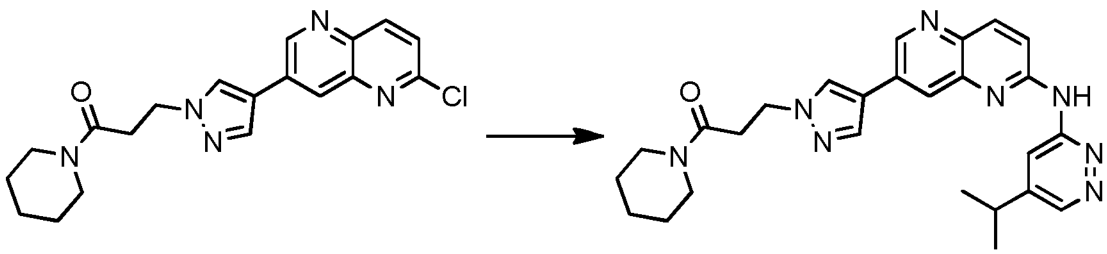

3-(4-(6-((5-isopropylpyridazin-3-yl)amino)-1,5-naphthyridin-3-yl)-1H-pyrazol-1-yl)-1-(piperidin-1-yl)propan-1-one was obtained as a yellow solid in the same manner as in Example 0411-3 except that 3-(4-(6-chloro-1,5-naphthyridin-3-yl)-1H-pyrazol-1-yl)-1-(piperidin-1-yl)propan-1-one was used instead of the 2-chloro-7-(1-((6-methylpyridin-2-yl)methyl)-1H-pyrazol-4-yl)-1,5-naphthyridine used in Example 0411-3.
$^1$H-NMR($CDCl_3$)δ:8.93(1H,d,J=2.0Hz),8.92-8.89(1H,m),8.83(1H,d,J=1.3Hz),8.24(1H,d,J=8.6Hz),8.10(1H,d,J=1.3Hz),8.00(1H,s),7.96(1H,s),7.61-7.58(1H,m),4.59(2H,t,J=6.3Hz),3.55(2H,t,J=5.3Hz),3.37(2H,t,J=5.3Hz),3.10-3.07(1H,m),2.97(2H,t,J=6.3Hz),1.60-1.53(6H,m),1.43(6H,d,J=7.3Hz).
MSm/z(M+H):471.

<Example 0417>

<0417-1>

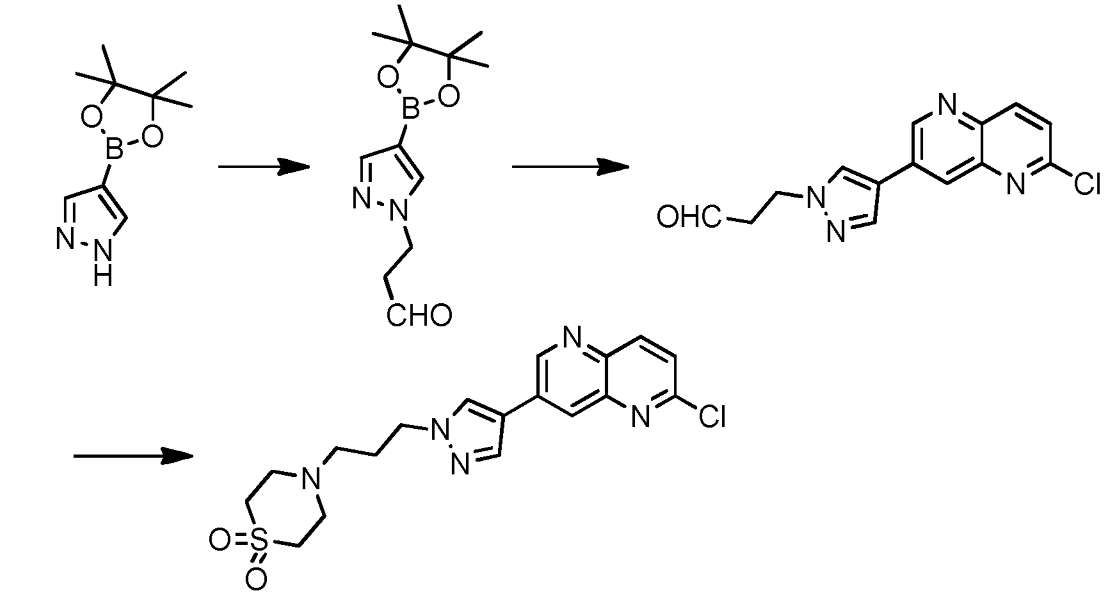

Hafnium chloride (IV) (82 mg) and acrolein (0.41 mL) were added to a solution of 4-(4,4,5,5-tetramethyl-1,3,2-dioxaborolan-2-yl)-1H-pyrazole (500 mg) in dichloromethane (13 mL), followed by stirring at room temperature for 5 hours. After water and ethyl acetate were added to the reaction mixture, the organic layer was collected by separation, washed with a saturated sodium chloride aqueous solution, and dried over anhydrous sodium sulfate, and the solvent was distilled off under reduced pressure, thereby obtaining pale yellow oily substance (359 mg).

A mixture of the obtained pale yellow oily substance (77 mg), 7-bromo-2-chloro-1,5-naphthyridine (51 mg), sodium carbonate (43 mg), bis(di-tert-butyl (4-dimethylaminophenyl)phosphine)dichloropalladium(II) (15 mg), 1,4-dioxane (2.1 mL), and water (0.21 mL) was stirred at 100°C for 1.5 hours in a nitrogen atmosphere. The reaction mixture was cooled to room temperature, the insolubles were filtered off, and the solvent was distilled off under reduced pressure, thereby obtaining a yellow solid (117 mg).

Thiomorpholine 1,1-dioxide (47 mg) and acetic acid (0.5 mL) were added to a solution of the obtained yellow solid (58 mg) in dichloromethane (1.5 mL), followed by stirring at room temperature for 1 hour in a nitrogen atmosphere. Sodium triacetoxyborohydride (218 mg) was added to the reaction mixture, followed by stirring at room temperature for 1 hour. After water and ethyl acetate were added to the reaction mixture, the organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-methanol, NH silica), thereby obtaining 4-(3-(4-(6-chloro-1,5-naphthyridin-3-yl)-1H-pyrazol-1-yl)propyl)thiomorpholine 1,1-dioxide (13 mg).
MSm/z(M+H):406.

<0417-2>

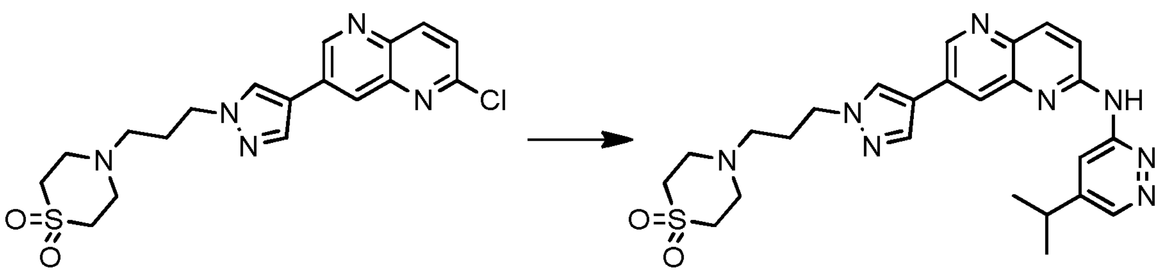

4-(3-(4-(6-((5-isopropylpyridazin-3-yl)amino)-1,5-naphthyridin-3-yl)-1H-pyrazol-1-yl)propyl)thiomorpholine 1,1-dioxide was obtained as a white solid in the same manner as in Example 0411-3 except that 4-(3-(4-(6-chloro-1,5-naphthyridin-3-yl)-1H-pyrazol-1-yl)propyl)thiomorpholine 1,1-dioxide was used instead of the 2-chloro-7-(1-((6-methylpyridin-2-yl)methyl)-1H-pyrazol-4-yl)-1,5-naphthyridine used in Example 0411-3.

$^1$H-NMR(DMSO-$d_6$)δ:10.70(1H,s),9.05(1H,d,J=2.0Hz),8.86(1H,d,J=2.0Hz),8.72(1H,s),8.53(1H,s),8.22-8.21(3H,m),7.70(1H,d,J=9.2Hz),4.23(2H,t,J=6.6Hz),3.11-3.09(4H,m),3.03(1H,t,J=6.9Hz),2.88-2.87(4H,m),2.46-2.44(2H,m),2.01-1.99(2H,m),1.33(6H,d,J=7.3Hz).
MSm/z(M+H):507.

<Example 0418>

<0418-1>

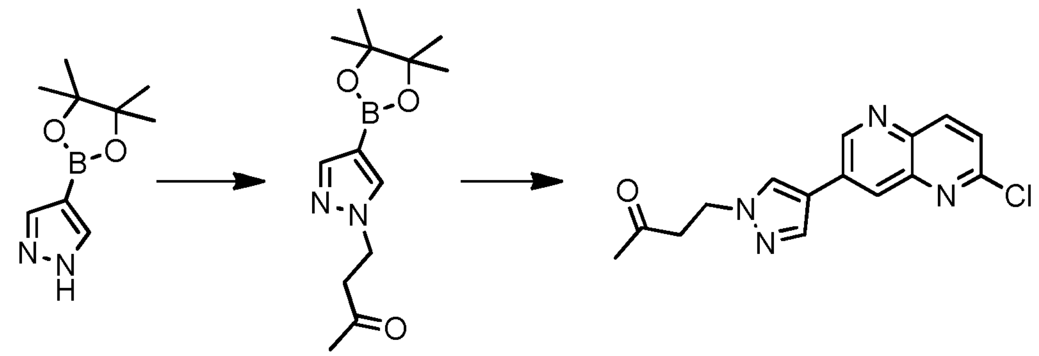

Hafnium chloride (IV) (49 mg) and methyl vinyl ketone (0.29 mL) were added to a solution of 4-(4,4,5,5-tetramethyl-1,3,2-dioxaborolan-2-yl)-1H-pyrazole (290 mg) in dichloromethane (7.5 mL), followed by stirring at room temperature for 5 hours. After water and ethyl acetate were added to the reaction mixture, the organic layer was collected by separation, washed with a saturated sodium chloride aqueous solution, and dried over anhydrous sodium sulfate, and the solvent was distilled off under reduced pressure, thereby obtaining brown oily substance (316 mg).

A mixture of the obtained brown oily substance (84 mg), 7-bromo-2-chloro-1,5-naphthyridine (51 mg), sodium carbonate (44 mg), bis(di-tert-butyl (4-dimethylaminophenyl)phosphine)dichloropalladium(II) (15 mg), 1,4-dioxane (2.1 mL), and water (0.21 mL) was stirred at 100°C for 4 hours in a nitrogen atmosphere. The reaction mixture was cooled to room temperature, and purified by silica gel column chromatography (hexane-ethyl acetate-methanol, NH silica), thereby obtaining 4-(4-(6-chloro-1,5-naphthyridin-3-yl)-1H-pyrazol-1-yl)butan-2-one (48 mg) as a pale yellow solid.
MSm/z(M+H):301.

<0418-2>

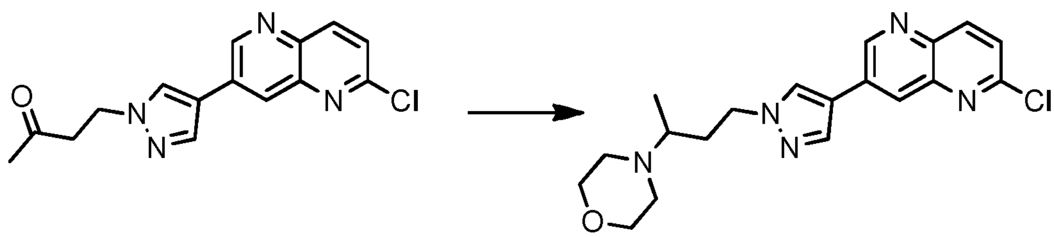

Morpholine (0.02 mL) and acetic acid (0.1 mL) were added to a solution of 4-(4-(6-chloro-1,5-naphthyridin-3-yl)-1H-pyrazol-1-yl)butan-2-one (24 mg) in dichloromethane (1 mL), followed by stirring at room temperature for 1 hour in a nitrogen atmosphere. Sodium triacetoxyborohydride (168 mg) was added to the reaction mixture, followed by stirring at room temperature for 1 hour. Morpholine (0.1 mL) was added to the reaction mixture, followed by stirring at room temperature for 1 hour. Morpholine (0.2 mL) was added to the reaction mixture, followed by stirring at room temperature for 1.5 hours. Morpholine (0.2 mL), sodium triacetoxyborohydride (89 mg), and acetic acid (0.5 mL) were added to the reaction mixture, followed by stirring at room temperature for 30 minutes. After water and ethyl acetate were added to the reaction mixture, the organic layer was collected by separation, washed with a saturated sodium chloride aqueous

solution, and dried over anhydrous sodium sulfate, and the solvent was distilled off under reduced pressure. The obtained residue was purified by silica gel column chromatography (hexane-ethyl acetate-methanol, NH silica), thereby obtaining 4-(4-(4-(6-chloro-1,5-naphthyridin-3-yl)-1H-pyrazol-1-yl)butan-2-yl)morpholine (12 mg) as colorless oily substance. MSm/z(M+H):372.

<0418-3>

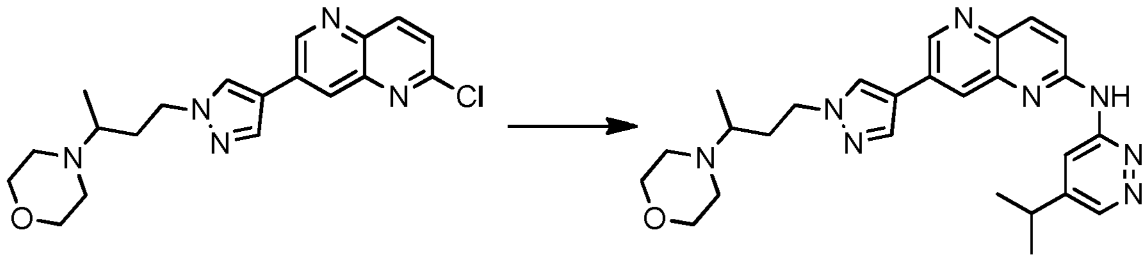

N-(5-isopropylpyridazin-3-yl)-7-(1-(3-morpholinobutyl)-1H-pyrazol-4-yl)-1,5-naphthyridine-2-amine was obtained as a yellow solid in the same manner as in Example 0411-3 except that 4-(4-(4-(6-chloro-1,5-naphthyridin-3-yl)-1H-pyrazol-1-yl)butan-2-yl)morpholine was used instead of the 2-chloro-7-(1-((6-methylpyridin-2-yl)methyl)-1H-pyrazol-4-yl)-1,5-naphthyridine used in Example 0411-3.
$^1$H-NMRpMSO-$d_6$)δ:10.70(1H,s),9.04(1H,d,J=2.0Hz),8.87(1H,d,J=2.0Hz),8.73(1H,d,J=2.0Hz),8.50(1H,s),8.22-8.19(3H,m),7.70(1H,d, J=9.2Hz),4.24-4.21(2H,m),3.62-3.57(4H,m),3.03(1H,t,J=6.9Hz),2.75-2.72(1H,m),2.33-2.30(2H,m),2.00-1.99(1H,m),1. 86-1.83(1H,m),1.33(6H,d,J=7.3Hz),1.23(2H,s),1.17(3H,d,J=7.3Hz).
MSm/z(M+H):473.

<Example 0419>

<0419-1>

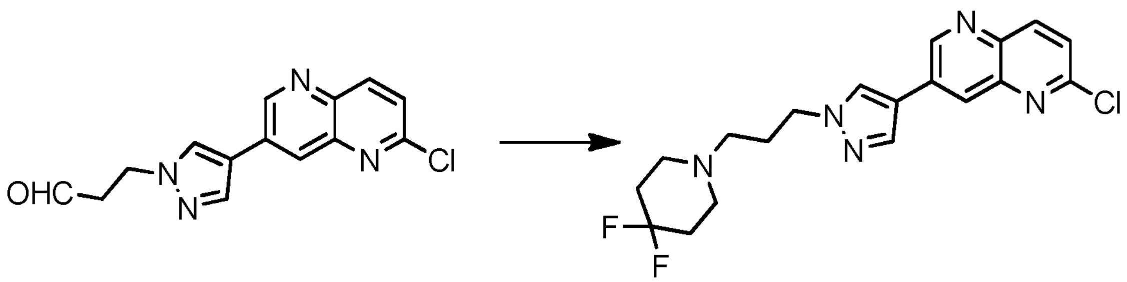

Triethylamine (0.07 mL) was added to a mixture of 4,4-difluoropiperidine hydrochloride (133 mg) and dichloromethane (0.5 mL), followed by stirring at room temperature for 10 minutes. 3-(4-(6-Chloro-1,5-naphthyridin-3-yl)-1H-pyrazol-1-yl)propanal (50 mg), dichloromethane (2 mL), and acetic acid (0.5 mL) were added to the reaction mixture, followed by stirring at room temperature for 30 minutes. Sodium triacetoxyborohydride (370 mg) was added to the reaction mixture, followed by stirring at room temperature for 1 hour. Methanol and acetone were added to the reaction mixture, and the solvent was distilled off under reduced pressure. Ethyl acetate and hexane were added to the obtained residue, the insolubles were filtered off, and the solvent was distilled off under reduced pressure. The obtained residue was purified by silica gel column chromatography (hexane-ethyl acetate-methanol, NH silica), thereby obtaining 2-chloro-7-(1-(3-(4,4-difluoropiperidin-1-yl)propyl)-1H-pyrazol-4-yl)-1,5-naphthyridine (14 mg) as a pale yellow solid.
MSm/z(M+H):392.

<0419-2>

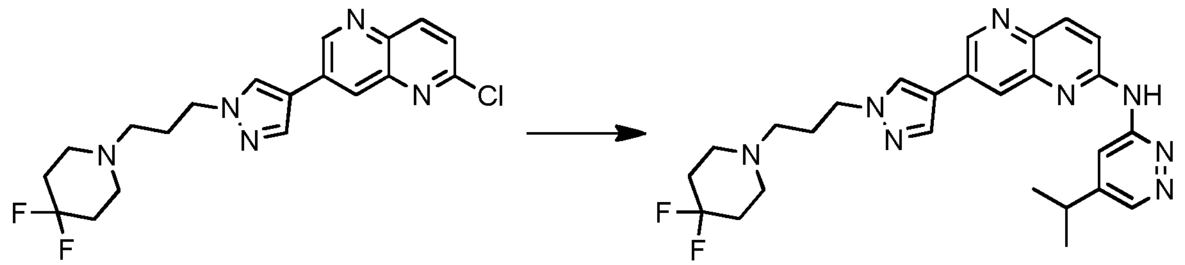

7-(1-(3-(4,4-Difluoropiperidin-1-yl)propyl)-1H-pyrazol-4-yl)-N-(5-isopropylpyridazin-3-yl)-1,5-naphthyridine-2-amine was obtained as a pale yellow solid in the same manner as in Example 0411-3 except that 2-chloro-7-(1-(3-(4,4-difluoropiperidin-1-yl)propyl)-1H-pyrazol-4-yl)-1,5-naphthyridine was used instead of the 2-chloro-7-(1-((6-methylpyridin-2-yl)methyl)-1H-pyrazol-4-yl)-1,5-naphthyridine used in Example 0411-3.
$^1$H-NMR($CDCl_3$)δ:8.93(1H,d,J=2.0Hz),8.84-8.82(2H,m),8.25(1H,d,J=9.2Hz),8.11-8.08(1H,m),7.97(1H,s),7.85(1H,s),7.56-7.54(1H,m),4.30(2H,t,J=6.6Hz),3.49(2H,s),3.06(1H,t,J=6.6Hz),2.56(4H,t,J=5.3Hz),2.43(2H,t,J=6.6Hz ),2.14-2.05(4H, m), 1.42(6H,d,J=6.6Hz).
MSm/z(M+H):493.

<Example 0420>

<0420-1>

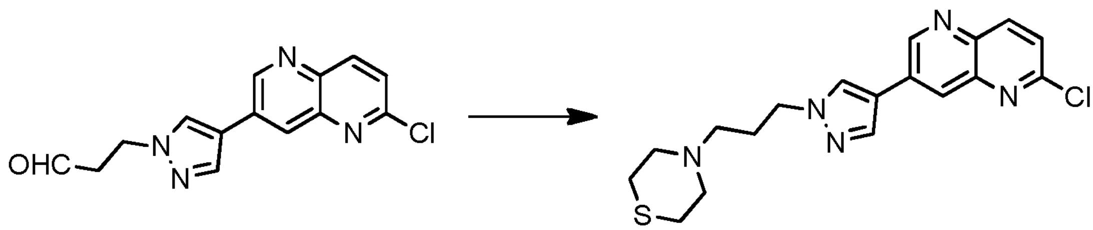

4-(3-(4-(6-Chloro-1,5-naphthyridin-3-yl)-1H-pyrazol-1-yl)propyl)thiomorpholine was obtained as a white solid in the same manner as in Example 0419-1 except that thiomorpholine was used instead of the 4,4-difluoropiperidine hydrochloride used in Example 0419-1. MSm/z(M+H):374.

<0420-2>

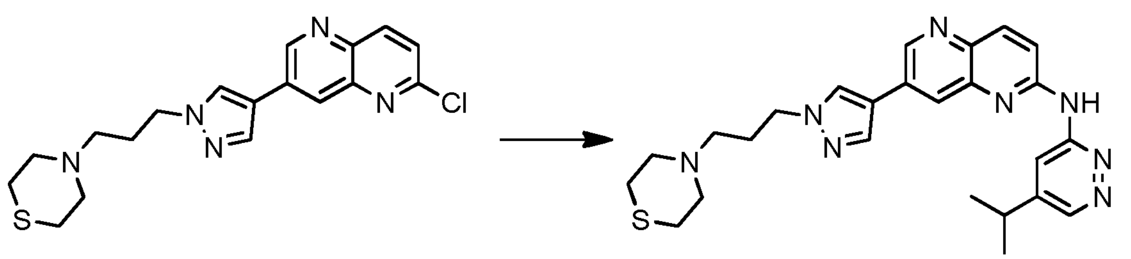

N-(5-isopropylpyridazin-3-yl)-7-(1-(3-thiomorpholinopropyl)-1H-pyrazol-4-yl)-1,5-naphthyridine-2-amine was obtained as a pale yellow solid in the same manner as in Example 0411-3 except that 4-(3-(4-(6-chloro-1,5-naphthyridin-3-yl)-1H-pyrazol-1-yl)propyl)thiomorpholine was used instead of the 2-chloro-7-(1-((6-methylpyridin-2-yl)methyl)-1H-pyrazol-4-yl)-1,5-naphthyridine used in Example 0411-3.
$^1$H-NMR($CDCl_3$)δ:9.22(1H,brs),8.92(1H,d,J=2.0Hz),8.88(1H,d,J=2.0Hz),8.83(1H,d,J=2.0Hz),8.25(1H,d,J =8.6Hz),8.10(1H,d,J=2.0Hz),7.97(1H,s),7.85(1H,s),7.62(1H,d,J=9.2Hz),4.28(2H,t,J=6.9Hz),3.06(1H,t ,J=6.9Hz),2.72-2.69(8H,m),2.39(2H,t,J=6.9Hz),2.15-2.05(2H,m),1.43(6H,d,J=7.3Hz).
MSm/z(M+H):475.

<Example 0421>

<0421-1>

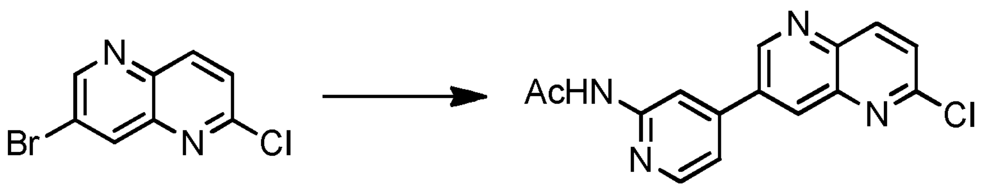

A mixture of 7-bromo-2-chloro-1,5-naphthyridine (48 mg), bis(pinacolato)diboron (58 mg), a 1,1'-bis(diphenylphosphino)ferrocenepalladium(II) dichloride-dichloromethane complex (16 mg), potassium acetate (42 mg), and 1,4-dioxane (1.9 mL) was stirred at 100°C for 2 hours. N-(4-bromopyridin-2-yl)acetamide (42 mg), sodium carbonate (45 mg), a bis(di-tert-butyl (4-dimethylaminophenyl)phosphine)dichloropalladium(II) (14 mg), 1,4-dioxane (0.3 mL), and water (0.19 mL)) were added to the reaction mixture, followed by stirring at 100°C for 2 hours. The reaction mixture was cooled to room temperature, and purified by silica gel column chromatography (hexane-ethyl acetate-methanol), thereby obtaining N-(4-(6-chloro-1,5-naphthyridin-3-yl)pyridin-2-yl)acetamide (12.6 mg) as a pale brown solid.
MSm/z(M+H):299.

<0421-2>

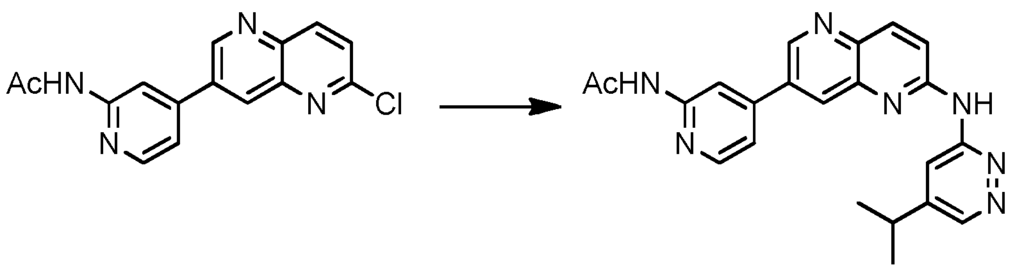

N-(4-(6-((5-isopropylpyridazin-3-yl)amino)-1,5-naphthyridin-3-yl)pyridin-2-yl)acetamide was obtained in the same manner as in Example 0411-3 except that N-(4-(6-chloro-1,5-naphthyridin-3-yl)pyridin-2-yl)acetamide was used instead of the 2-chloro-7-(1-((6-methylpyridin-2-yl)methyl)-1H-pyrazol-4-yl)-1,5-naphthyridine used in Example 0411-3.
$^1$H-NMR($CDCl_3$)δ:9.05(1H,d,J=2.0Hz),8.83(2H,s),8.63(1H,s),8.42(1H,d,J=5.3Hz),8.32(2H,d,J=8.6Hz),8. 06(1H,s),7.62(1H,t,J=4.6Hz),7.40(1H,dd,J=5.3,2.0Hz),3.08-3.05(1H,m),2.28(3H,s),1.42(6H,d,J=7.3Hz).
MSm/z(M+H):400.

<Example 0422>

<0422-1>

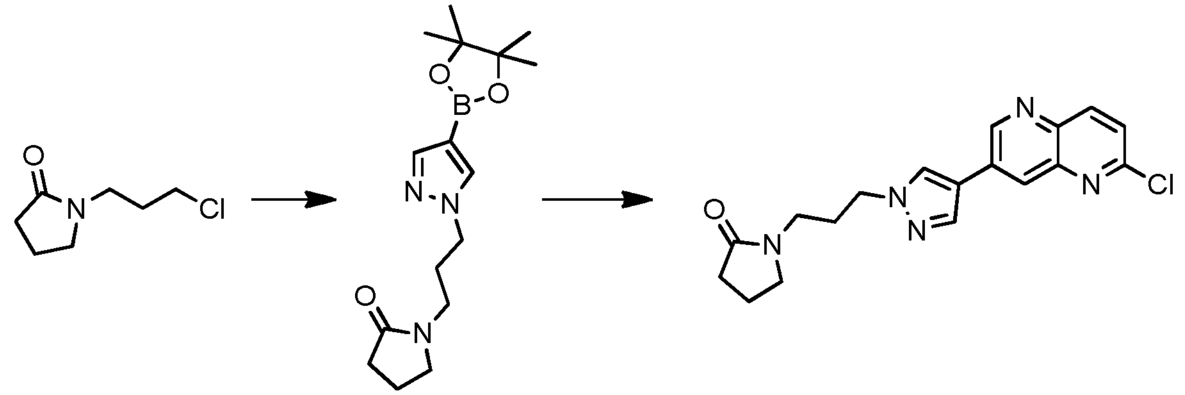

1-(3-(4-(6-Chloro-1,5-naphthyridin-3-yl)-1H-pyrazol-1-yl)propyl)pyrrolidin-2-one was obtained as pale yellow oily substance in the same manner as in Example 0415-1 except that 1-(3-chloropropyl)pyrrolidin-2-one was used instead of the used in Example 0415-1.
MSm/z(M+H):356.

<0422-2>

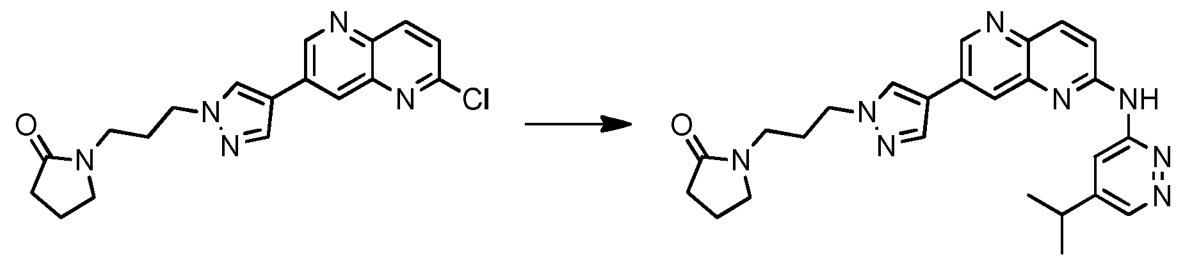

1-(3-(4-(6-((5-Isopropylpyridazin-3-yl)amino)-1,5-naphthyridin-3-yl)-1H-pyrazol-1-yl)propyl)pyrrolidin-2-one was obtained as a pale brown solid in the same manner as in Example 0411-3 except that 1-(3-(4-(6-chloro-1,5-naphthyridin-3-yl)-1H-pyrazol-1-yl)propyl)pyrrolidin-2-one was used instead of the 2-chloro-7-(1-((6-methylpyridin-2-yl)methyl)-1H-pyrazol-4-yl)-1,5-naphthyridine used in Example 0411-3.
$^1$H-NMR($CDCl_3$)δδ:8.95(1H,d,J=2.0Hz),8.81(2H,s),8.44(1H,brs),8.24(1H,d,J=9.2Hz),8.11(1H,d,J=2.0Hz) ,7.98(2H,d,J=3.3Hz),7.44(1H,d,J=9.2Hz),4.25(2H,t,J=6.6Hz),3.50-3.42(3H,m),3.06(1H,t,J=6.9Hz),2.43-2.37(3H,m),2.22-2.18(2H,m),2.06-2.01(2H,m),1.42(6H,d,J=6.6Hz).
MSm/z(M+H):457.

<Example 0423>

<0423-1>

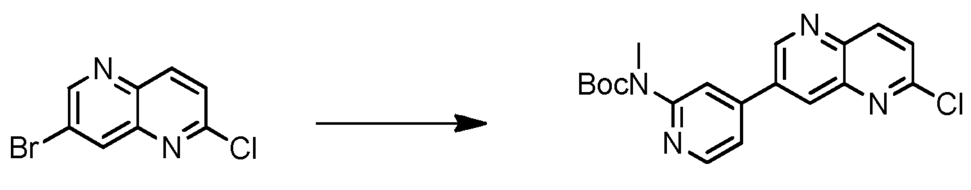

tert-Butyl (4-(6-chloro-1,5-naphthyridin-3-yl)pyridin-2-yl)(methyl)carbamate was obtained as a white solid in the same manner as in Example 0421-1 except that tert-butyl (4-bromopyridin-2-yl) (methyl)carbamate was used instead

of the N-(4-bromopyridin-2-yl)acetamide used in Example 0421-1.
MSm/z(M+H):371.

<0423-2>

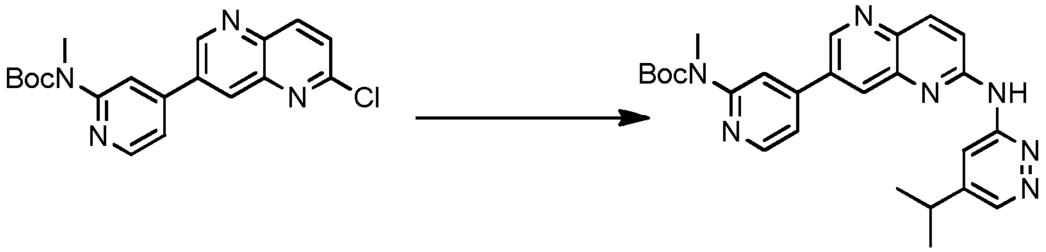

tert-Butyl (4-(6-((5-isopropylpyridazin-3-yl)amino)-1,5-naphthyridin-3-yl)pyridin-2-yl) (methyl)carbamate was obtained as a yellow solid in the same manner as in Example 0411-3 except that tert-butyl (4-(6-chloro-1,5-naphthyridin-3-yl)pyridin-2-yl) (methyl)carbamate was used instead of the 2-chloro-7-(1-((6-methylpyridin-2-yl)methyl)-1H-pyrazol-4-yl)-1,5-naphthyridine used in Example 0411-3.
MSm/z(M+H):472.

<0423-3>

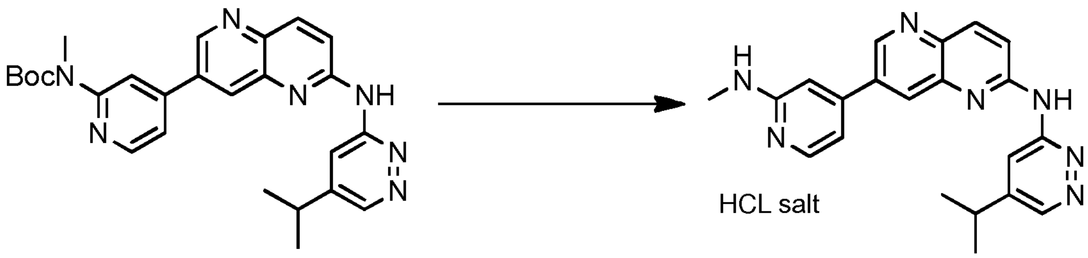


A 4.0 mol/L hydrogen chloride/1,4-dioxane solution (3 mL) was added to tert-butyl (4-(6-((5-isopropylpyridazin-3-yl)amino)-1,5-naphthyridin-3-yl)pyridin-2-yl) (methyl)carbamate (7.8 mg), followed by stirring at room temperature for 1 hour, and stirring at 50°C for 6 hours. The reaction mixture was cooled to room temperature, and hexane was added thereto. The solid matter was collected by filtration, thereby obtaining N-(5-isopropylpyridazin-3-yl)-7-(2-(methylamino)pyridin-4-yl)-1,5-naphthyridine-2-amine hydrochloride (5.2 mg) as a yellow solid.
$^1$H-NMR($CD_3OD$)$\delta$:9.33-9.32(1H,m),9.15(1H,s),8.99-8.98(1H,m),8.64(1H,d,J=9.2Hz),8.05(1H,d,J=6.6Hz),7.84(1H,s),7.74(1H,d,J=9.2Hz),7.49-7.44(3H,m),3.15(3H,s),3.07-3.07(1H,m),1.43(6H,d,J=6.6Hz).
MSm/z(M+H):372.

<Example 0424>

<0424-1>

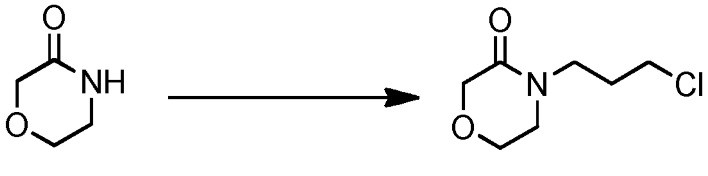

60% sodium hydride (105 mg) was added to a solution of morpholin-3-one (262 mg) in tetrahydrofuran (9.6 mL), followed by stirring at room temperature for 20 minutes in a nitrogen atmosphere. The reaction mixture was cooled by ice, and 1-bromo-3-chloropropane (0.31 mL) was added thereto, followed by stirring at room temperature for 15 hours. After water and ethyl acetate were added to the reaction mixture, the organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate-methanol), thereby obtaining 4-(3-chloropropyl)morpholin-3-one (50 mg) as pale yellow oily substance.
MSm/z(M+H):178.

<0424-2>

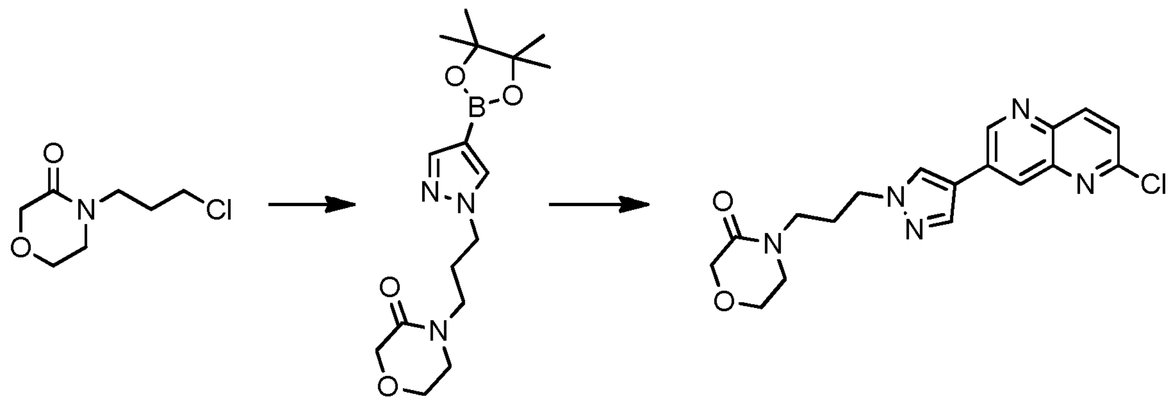

4-(3-(4-(6-Chloro-1,5-naphthyridin-3-yl)-1H-pyrazol-1-yl)propyl)morpholin-3-one was obtained as a pale yellow solid in the same manner as in Example 0415-1 except that 4-(3-chloropropyl)morpholin-3-one was used instead of the used in Example 0415-1.
MSm/z(M+H):372.

<0424-3>

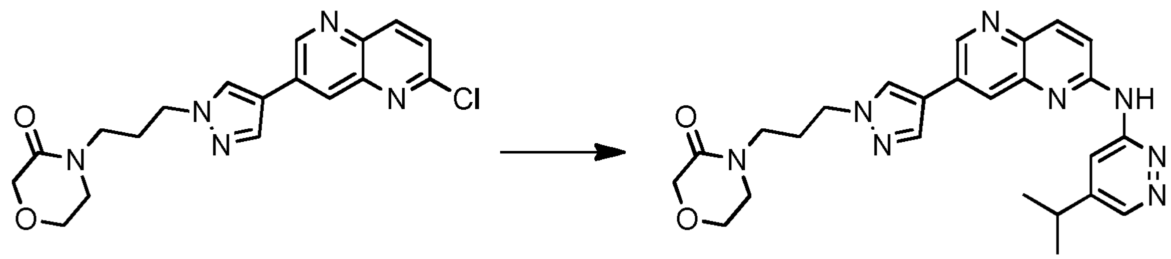

4-(3-(4-(6-((5-isopropylpyridazin-3-yl)amino)-1,5-naphthyridin-3-yl)-1H-pyrazol-1-yl)propyl)morpholin-3-one was obtained as a pale yellow solid in the same manner as in Example 0411-3 except that 4-(3-(4-(6-chloro-1,5-naphthyridin-3-yl)-1H-pyrazol-1-yl)propyl)morpholin-3-one was used instead of the 2-chloro-7-(1-((6-methylpyridin-2-yl)methyl)-1H-pyrazol-4-yl)-1,5-naphthyridine used in Example 0411-3.
$^1$H-NMR($CDCl_3$)δ:8.94(1H,d,J=2.0Hz),8.82(2H,s),8.24(1H,d,J=9.2Hz),8.11(1H,d,J=2.0Hz),7.97(2H,d,J=5.9Hz),7.50-7.47(1H,m),4.28(2H,t,J=6.6Hz),4.13(2H,s),3.85(2H,t,J=5.0Hz),3.54(2H,t,J=6.6Hz),3.38(2H,t,J=5.0Hz ),3.06(1H,t,J=6.9Hz),2.28(2H,t,J=6.6Hz),1.42(6H,d,J=6.6Hz).
MSm/z(M+H):473.

<Example 0425>

<0425-1>

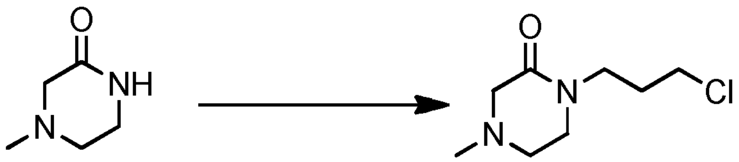

1-(3-Chloropropyl)-4-methylpiperazin-2-one was obtained as pale yellow oily substance in the same manner as in Example 0424-1 except that 4-methylpiperazin-2-one was used instead of the morpholin-3-one used in Example 0424-1.
MSm/z(M+H):191.

<0425-2>

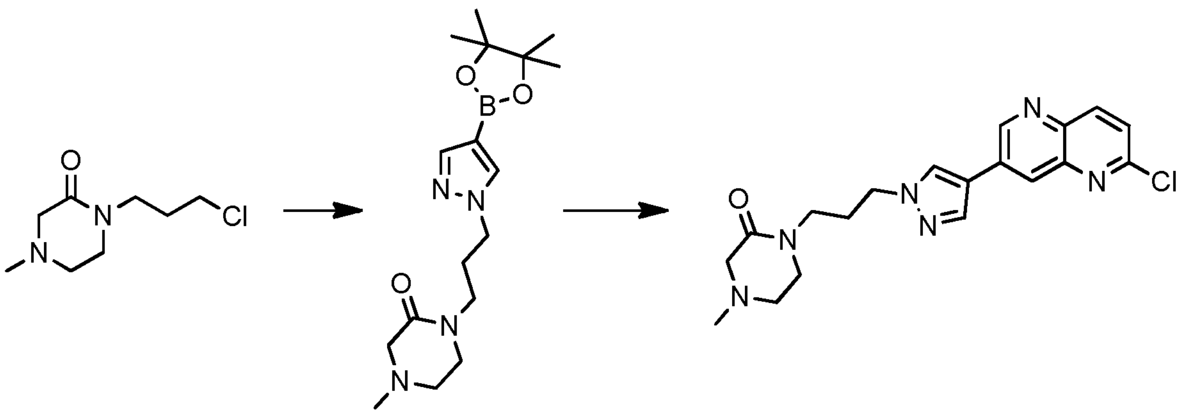

1-(3-(4-(6-Chloro-1,5-naphthyridin-3-yl)-1H-pyrazol-1-yl)propyl)-4-methylpiperazin-2-one was obtained in the same manner as in Example 0415-1 except that 1-(3-chloropropyl)-4-methylpiperazin-2-one was used instead of the used in Example 0415-1.
MSm/z(M+H):385.

<0425-3>

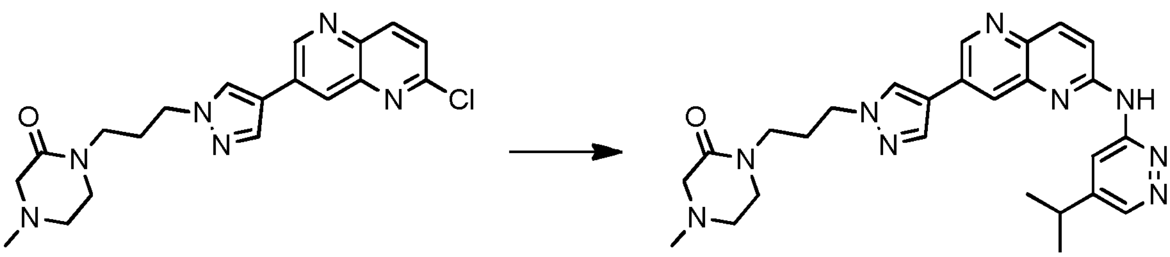

1-(3-(4-(6-((5-1sopropylpyridazin-3-yl)amino)-1,5-naphthyridin-3-yl)-1H-pyrazol-1-yl)propyl)-4-methylpiperazin-2-one was obtained as a brown solid in the same manner as in Example 0411-3 except that 1-(3-(4-(6-chloro-1,5-naphthyridin-3-yl)-1H-pyrazol-1-yl)propyl)-4-methylpiperazin-2-one was used instead of the 2-chloro-7-(1-((6-methylpyridin-2-yl)methyl)-1H-pyrazol-4-yl)-1,5-naphthyridine used in Example 0411-3.
$^{1}$H-NMR($CDCl_3$)δ:8.94(1H,d,J=2.0Hz),8.85(1H,s),8.82-8.82(2H,m),8.24(1H,d,J=9.2Hz),8.11(1H,d,J=1.3Hz),7.97(2H,s),7.52(1H,d,J=9.2Hz),4.26(2H,t,J=6.6Hz),3.50(2H,t,J=6.6Hz),3.35(2H,t,J=5.3Hz),3.08-3.07(3H,m),2.62(2H,t,J=5.6Hz),2.31(3H,s),2.25(2H,t,J=6.9Hz),1.43(6H,d,J=7.3Hz).
MSm/z(M+H):486.

<Example 0426>

<0426-1>

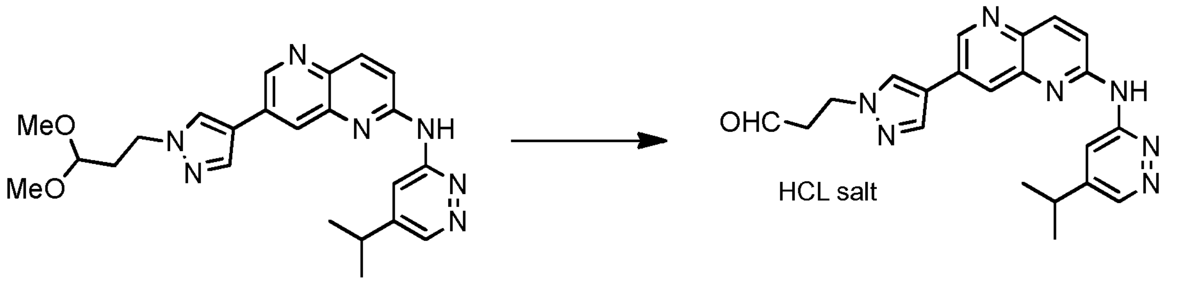


1 mol/L hydrochloric acid (15 mL) was added to a mixture of 7-(1-(3,3-dimethoxypropyl)-1H-pyrazol-4-yl)-N-(5-isopropylpyridazin-3-yl)-1,5-naphthyridine-2-amine (1.66 g) and 1,4-dioxane (30 mL), followed by stirring at 70°C for 1 hour. The solvent was distilled off under reduced pressure, thereby obtaining 3-(4-(6-((5-isopropylpyridazin-3-yl)amino)-1,5-naphthyridin-3-yl)-1H-pyrazol-1-yl)propanal hydrochloride (1.9 g) as a yellow solid.
MSm/z(M+H):388.

<0426-2>

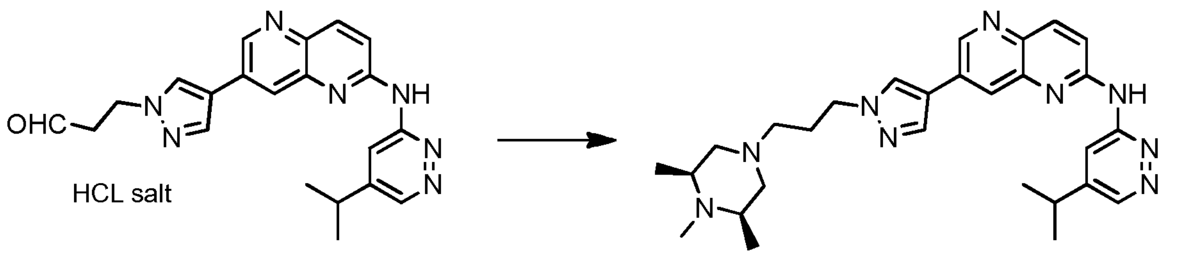


Triethylamine (6.7 μL) was added to a mixture of (2S,6R)-1,2,6-trimethylpiperazine trifluoroacetate (11.6 mg) and dichloromethane (0.5 mL), followed by stirring at room temperature for 15 minutes in a nitrogen atmosphere. 3-(4-(6-((5-Isopropylpyridazin-3-yl)amino)-1,5-naphthyridin-3-yl)-1H-pyrazol-1-yl)propanal hydrochloride (6.2 mg) and dichloromethane (2.0 mL) were added to the reaction mixture, followed by stirring at room temperature for 30 minutes. Sodium triacetoxyborohydride (35 mg) and acetic acid (0.5 mL) were added to the reaction mixture, followed by stirring at room temperature for 3 hours. The reaction mixture was purified by silica gel column chromatography (ethyl acetate-methanol, NH silica), thereby obtaining N-(5-isopropylpyridazin-3-yl)-7-(1-(3-((3S,5R)-3,4,5-trimethylpiperazin-1-yl)propyl)-1H-pyrazol-4-yl)-1,5-naphthyridine-2-amine (2.0 mg) as a yellow solid.
$^{1}$H-NMR($CDCl_3$)δ:8.91(2H,d,J=8.6Hz),8.80(1H,s),8.25(1H,d,J=8.6Hz),8.10(1H,d,J=1.3Hz),7.96(1H,s),7.86(1H,s),7.60(1H,d,J=9.2Hz),4.28(2H,t,J=6.6Hz),3.06(1H,t,J=6.6Hz),2.74(2H,d,J=9.9Hz),2.35-2.30(9H,m),2.17-2.10(2H,m),1.42(6H,d,J=7.3Hz),1.10(6H,d,J=6.6Hz).
MSm/z(M+H):500.

<Example 0427>

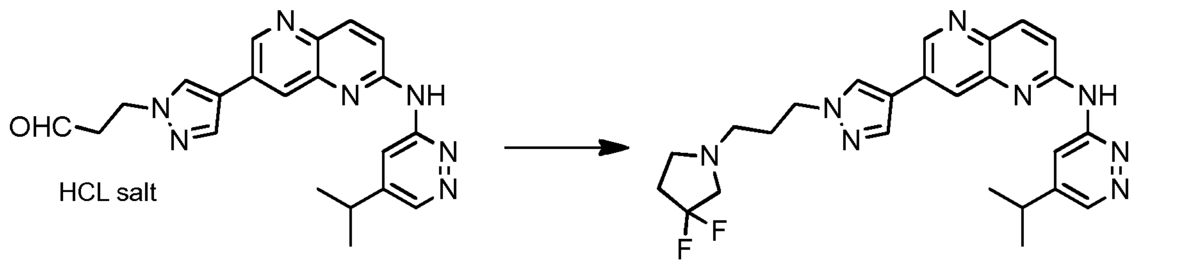


7-(1-(3-(3,3-difluoropyrrolidin-1-yl)propyl)-1H-pyrazol-4-yl)-N-(5-isopropylpyridazin-3-yl)-1,5-naphthyridine-2-amine was obtained as a yellow solid in the same manner as in Example 0426-2 except that 3,3-difluoropyrrolidine hydrochloride was used instead of the (2S,6R)-1,2,6-trimethylpiperazine trifluoroacetate used in Example 0426-2.
$^1$H-NMR($CDCl_3$)δ:8.93(1H,d,J=2.0Hz),8.89(1H,brs),8.74(1H,s),8.27-8.23(1H,m),8.10(1H,d,J=2.0Hz),8.08(1H,s),7.97(1H,d,J=4.6Hz),7.87(1H,d,J=2.0Hz),7.61-7.53(1H,m),4.31(2H,t,J=6.9Hz),3.20-2.86(8H,m),2.74-2.72(1H,m),2.43-2.26(2H,m),1.41 (6H,d,J=6.6Hz).
MSm/z(M+H):479.

<Example 0428>

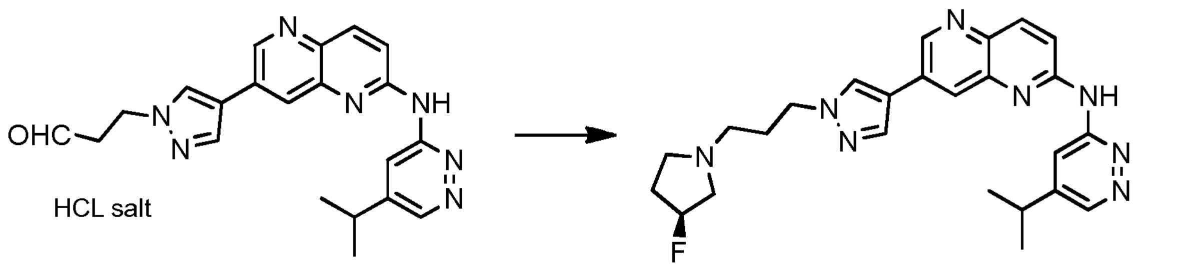


(S)-7-(1-(3-(3-fluoropyrrolidin-1-yl)propyl)-1H-pyrazol-4-yl)-N-(5-isopropylpyridazin-3-yl)-1,5-naphthyridine-2-amine was obtained as a pale yellow solid in the same manner as in Example 0426-2 except that (S)-3-fluoropyrrolidine hydrochloride was used instead of the (2S,6R)-1,2,6-trimethylpiperazine trifluoroacetate used in Example 0426-2.
$^1$H-NMR($CDCl_3$)δ:8.94-8.93(2H,m),8.75(1H,s),8.24(1H,d,J=9.2Hz),8.10-8.09(1H,m),7.97(1H,s),7.89(1H,s),7.58-7.55(1H,m),4.32(2H,t,J=6.9Hz),3.08-2.46(6H,m),2.18-2.16(6H,m),1.42(6H,d,J=7.3Hz).
MSm/z(M+H):461.

<Example 0429>

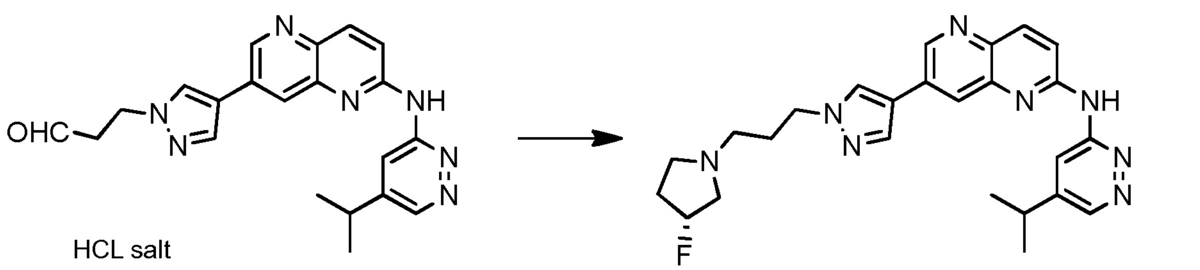

(R)-7-(1-(3-(3-fluoropyrrolidin-1-yl)propyl)-1H-pyrazol-4-yl)-N-(5-isopropylpyridazin-3-yl)-1,5-naphthyridine-2-amine was obtained as a pale yellow solid in the same manner as in Example 0426-2 except that (R)-3-fluoropyrrolidine hydrochloride was used instead of the (2S,6R)-1,2,6-trimethylpiperazine trifluoroacetate used in Example 0426-2. $^1$H-NMR($CDCl_3$)δ:8.90-8.83(3H,m),8.26-8.23(1H,m),8.10-8.08(2H,m),7.97(1H,s),7.91-7.87(1H,m),7.54(1H,d,J=9.2Hz),4.32(2H,t,J=6.6Hz),3.08-2.04(12H,m),1.42(6H,d,J=6.6Hz).
MSm/z(M+H):461.

<Example 0430>

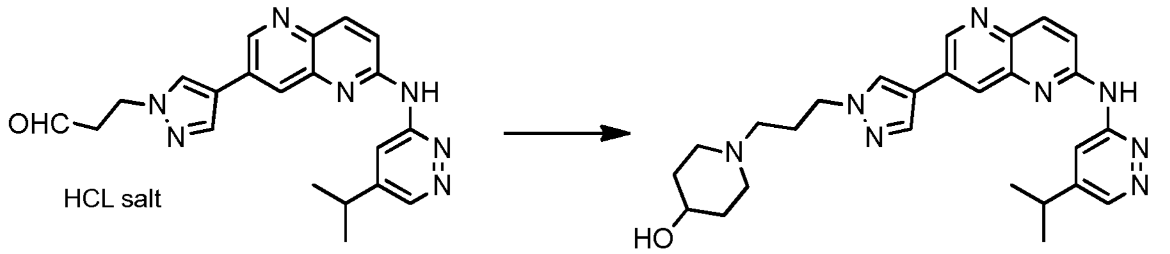


1-(3-(4-(6-((5-Isopropylpyridazin-3-yl)amino)-1,5-naphthyridin-3-yl)-1H-pyrazol-1-yl)propyl)piperidin-4-ol was obtained as a pale yellow solid in the same manner as in Example 0426-2 except that piperidin-4-one trifluoroacetate was used instead of the (2S,6R)-1,2,6-trimethylpiperazine trifluoroacetate used in Example 0426-2.
$^1$H-NMR($CDCl_3$)δ:8.93(2H,d,J=2.0Hz),8.75(1H,s),8.24(1H,d,J=9.2Hz),8.10(1H,d,J=2.0Hz),7.97(1H,s),7.89(1H,s),7.58(1H,d,J=9.2Hz),4.29(2H,t,J=6.6Hz),3.78(1H,t,J=4.0Hz),3.07-3.02(1H,m),2.87-2.74(2H,m),2.47(2H,t,J=7.3Hz),2.33-2.30(2H,m),2.18-2.16(2H,m),1.94-1.89(2H,m),1.71-1.65(2H,m), 1.42(6H,d,J=6.6Hz).
MSm/z(M+H):473.

<Example 0431>

<0431-1>

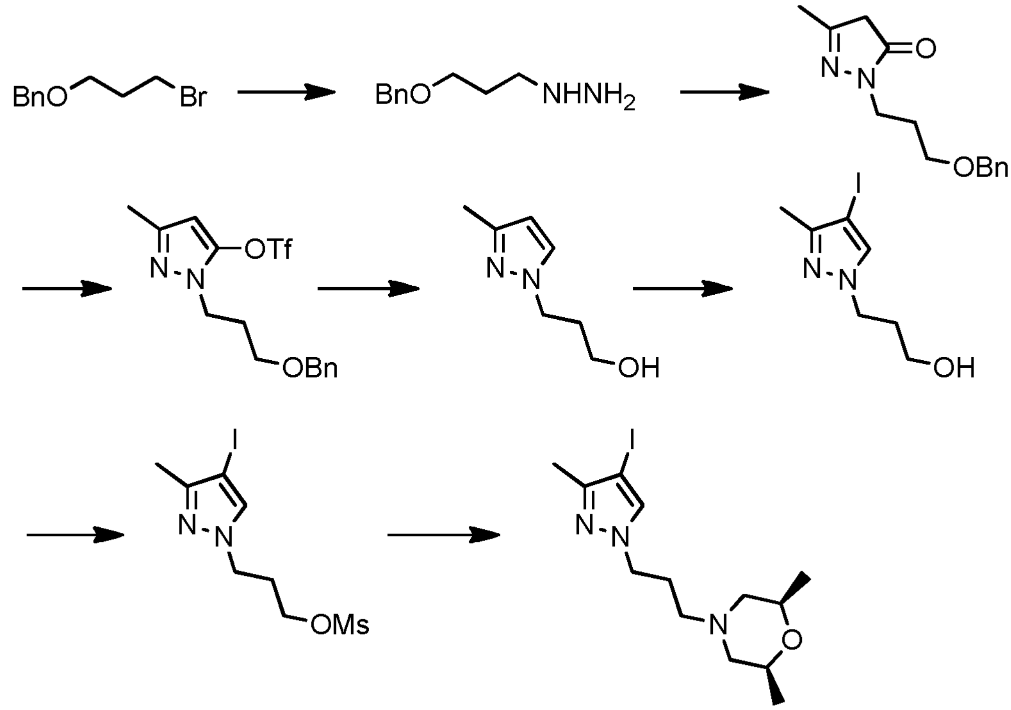

A solution of ((3-bromopropoxy)methyl)benzene (7.7 mL) in ethanol (10 mL) was added dropwise to hydrazine monohydrate (17 mL) at 65°C over a period of 1 hour, followed by stirring at 65°C for 3.5 hours. The reaction mixture was cooled to room temperature, passed through DOWEX™ MONOSPHERE™ 550A(OH) (product name, manufactured by Wako Pure Chemical Industries, Ltd.), and the solvent was distilled off under reduced pressure, thereby obtaining pale yellow oily substance (7.27 g).

Ethyl acetoacetate (2.8 mL) was added to a solution of the obtained pale yellow oily substance (7.27 g) in ethanol (22.4 mL), followed by stirring at room temperature for 1 hour. The solvent was distilled off under reduced pressure, tert-butyl methyl ether and a 2.0 mol/L sodium hydroxide aqueous solution were added to the obtained residue, and the aqueous layer was collected by separation. After a 3.0 mol/L potassium hydrogen sulfate aqueous solution and ethyl acetate were added to the aqueous layer, the organic layer was collected by separation, washed with a saturated sodium chloride aqueous solution, and dried over anhydrous sodium sulfate, and the solvent was distilled off under reduced pressure, thereby obtaining brown oily substance (3.5 g).

A solution of the obtained brown oily substance (3.5 g) in dichloromethane (71 mL) was cooled by ice, and pyridine (2.1 mL) and trifluoromethanesulfonic aicd anhydride (3.5 mL) were added thereto, followed by stirring at 0°C for 2 hours. After water and dichloromethane were added to the reaction mixture, the organic layer was collected by separation, washed with a saturated sodium chloride aqueous solution, and dried over anhydrous sodium sulfate, and the solvent was distilled off under reduced pressure, thereby obtaining brown oily substance (3.5 g). The obtained brown oily substance was purified by silica gel column chromatography (hexane-ethyl acetate), thereby obtaining brown oily substance (3.85 g).

A mixture of the obtained brown oily substance (3.85 g), 20% palladium hydroxide/carbon (0.20 g), and methanol (40 mL) was stirred at 50°C for 1 hour in a hydrogen (0.8 MPa) atmosphere. The insolubles were filtered off, and the solvent was distilled off under reduced pressure, thereby obtaining pale brown oily substance (2.2 g).

Iodine (1.55 g) and ammonium cerium(IV) nitrate (3.36 g) were added to a solution of the obtained brown oily substance (2.2 g) in acetonitrile (20 mL), followed by stirring at room temperature for 4 hours. The reaction mixture was allowed to stand for 14.5 hours, and stirred at room temperature for 2 hours. After water and ethyl acetate were added to the reaction mixture, the organic layer was collected by separation, washed with a saturated sodium chloride aqueous solution and a 10% sodium hydrogen sulfite aqueous solution, and dried over anhydrous sodium sulfate, and the solvent was distilled off under reduced pressure, thereby obtaining brown oily substance (1.66 g).

Triethylamine (1.74 mL) was added to a solution of the obtained brown oily substance (1.66 g) in dichloromethane (31 mL), followed by stirring for 10 minutes under ice-cooling. Methanesulfonyl chloride (0.73 mL) was added to the reaction mixture, followed by stirring at 0°C for 1 hour. After water was added to the reaction mixture, the organic layer was collected by separation, washed with a saturated sodium chloride aqueous solution, and dried over anhydrous sodium sulfate, and the solvent was distilled off under reduced pressure, thereby obtaining brown oily substance (2.24 g).

A mixture of the obtained brown oily substance (288 mg), potassium carbonate (230 mg), (2S,6R)-2,6-dimeth-

ylmorpholine (156 μL), and acetonitrile (4.2 mL) was stirred at 80°C for 16 hours. After the reaction mixture was cooled to room temperature, water and ethyl acetate were added thereto, the organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate, NH silica), thereby obtaining (2S,6R)-4-(3-(4-iodo-3-methyl-1H-pyrazol-1-yl)propyl)-2,6-dimethylmorpholine (29.8 mg) as pale yellow oily substance.
MSm/z(M+H):364.

<0431-2>

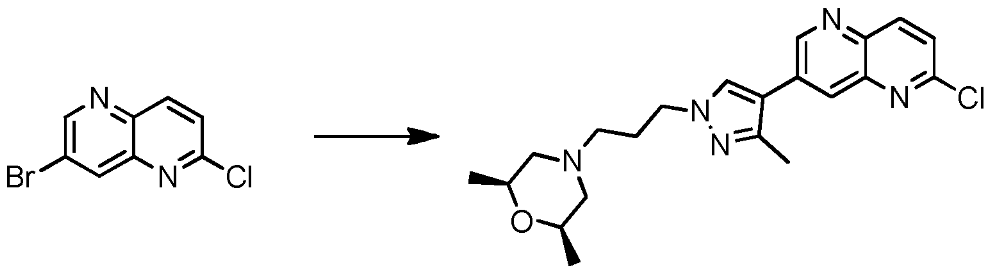

(2S,6R)-4-(3-(4-(6-chloro-1,5-naphthyridin-3-yl)-3-methyl-1H-pyrazol-1-yl)propyl)-2,6-dimethylmorpholine was obtained as a pale yellow solid in the same manner as in Example 0421-1 except that (2S,6R)-4-(3-(4-iodo-3-methyl-1H-pyrazol-1-yl)propyl)-2,6-dimethylmorpholine was used instead of the N-(4-bromopyridin-2-yl)acetamide used in Example 0421-1.
MSm/z(M+H):400.

<0431-3>

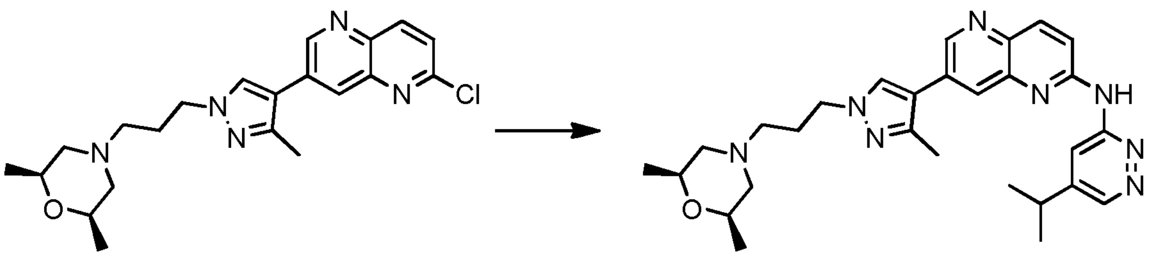

7-(1-(3-((2S,6R)-2,6-dimethylmorpholino)propyl)-3-methyl-1H-pyrazol-4-yl)-N-(5-isopropylpyridazin-3-yl)-1,5-naphthyridine-2-amine was obtained as a white solid in the same manner as in Example 0411-3 except that (2S,6R)-4-(3-(4-(6-chloro-1,5-naphthyridin-3-yl)-3-methyl-1H-pyrazol-1-yl)propyl)-2,6-dimethylmorpholine was used instead of the 2-chloro-7-(1-((6-methylpyridin-2-yl)methyl)-1H-pyrazol-4-yl)-1,5-naphthyridine used in Example 0411-3.
$^1$H-NMR($CDCl_3$)δ:8.85-8.80(3H,m),8.26(1H,d,J=8.6Hz),8.06(1H,s),7.69(1H,s),7.50(1H,d,J=9.2Hz),4.22(2H,t,J=6.6Hz),3.73-3.71(2H,m),3.03(1H,t,J=6.3Hz),2.76-2.73(2H,m),2.51(3H,s),2.39-2.36(2H,m),2.17-2.13(2H,m),1.40(6H,d,J=7.3Hz),1.17(6H,d,J=6.6Hz).
MSm/z(M+H):501.

<Example 0432>

<0432-1>

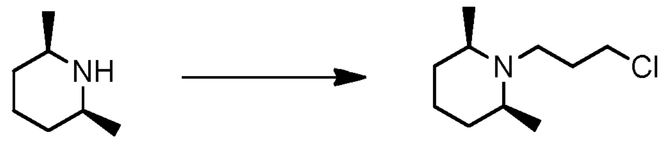

A mixture of (2S,6R)-2,6-dimethylpiperidine (0.27 mL), cesium carbonate (979 mg), 1-bromo-3-chloropropane (0.24 mL), and 1,4-dioxane (4 mL) was stirred at 80°C for 16 hours. The reaction mixture was cooled to room temperature, the insolubles were filtered off, and the solvent was distilled off under reduced pressure, thereby obtaining (2S,6R)-1-(3-chloropropyl)-2,6-dimethylpiperidine (56 mg) as pale yellow oily substance.
MSm/z(M+H):190.

<0432-2>

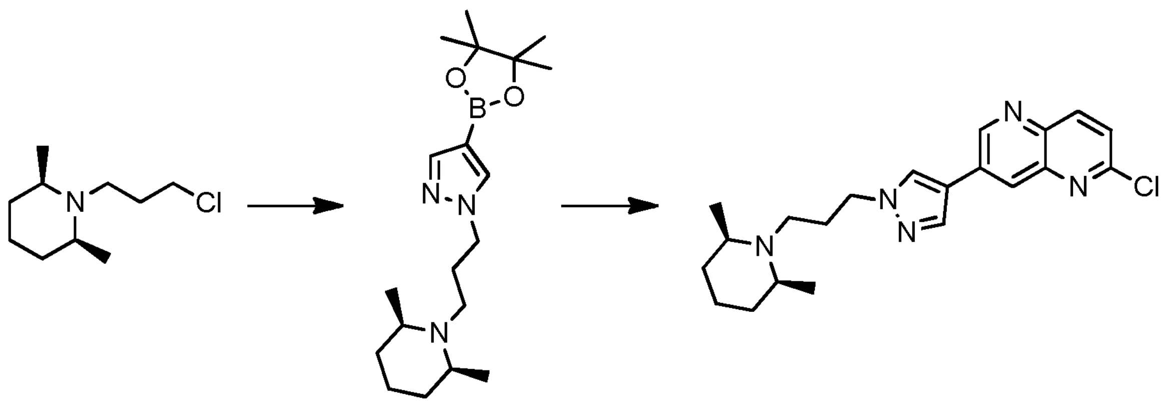

2-Chloro-7-(1-(3-((2R,6S)-2,6-dimethylpiperidin-1-yl)propyl)-1H-pyrazol-4-yl)-1,5-naphthyridine was obtained as a pale yellow solid in the same manner as in Example 0415-1 except that (2S,6R)-1-(3-chloropropyl)-2,6-dimethylpiperidine was used instead of the 1-(3-chloropropyl)piperidin-2-one used in Example 0415-1.
MSm/z(M+H):384.

<0432-3>

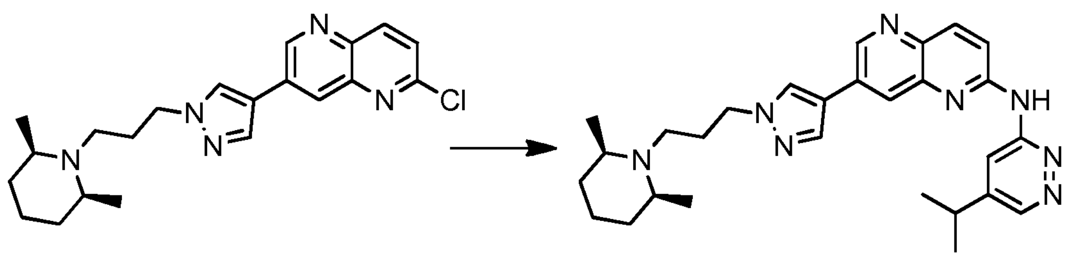

7-(1-(3-((2R,6S)-2,6-dimethylpiperidin-1-yl)propyl)-1H-pyrazol-4-yl)-N-(5-isopropylpyridazin-3-yl)-1,5-naphthyridine-2-amine was obtained in the same manner as in Example 0411-3 except that 2-chloro-7-(1-(3-((2R,6S)-2,6-dimethylpiperidin-1-yl)propyl)-1H-pyrazol-4-yl)-1,5-naphthyridine was used instead of the 2-chloro-7-(1-((6-methylpyridin-2-yl)methyl)-1H-pyrazol-4-yl)-1,5-naphthyridine used in Example 0411-3.
$^{1}$H-NMR($CDCl_3$)$\delta$:8.93(1H,d,J=2.0Hz),8.82(2H,s),8.24(1H,d,J=9.2Hz),8.10(1H,d,J=2.0Hz),7.97(1H,s),7.85(1H,s),7.50(1H,d,J=9.2Hz),6.55(1H,s),4.18(2H,t,J=6.9Hz),3.06(1H,t,J=6.9Hz),2.82-2.80(2H,m),2.46-2.43(2H,m),2.08-2.05(2H,m),1.42(6H,d,J=6.6Hz),1.29-1.26(4H,m),1.07(6H,d,J=6.6Hz).
MSm/z(M+H):485.

<Example 0433>

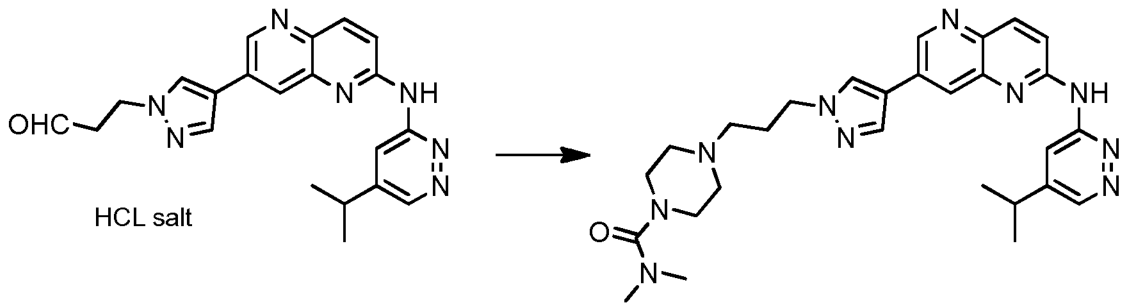

4-(3-(4-(6-((5-Isopropylpyridazin-3-yl)amino)-1,5-naphthyridin-3-yl)-1H-pyrazol-1-yl)propyl)-N,N-dimethylpiperazine-1-carboxamide was obtained in the same manner as in Example 0426-2 except that N,N-dimethylpiperazine-1-carboxamide was used instead of the (2S,6R)-1,2,6-trimethylpiperazinetrifluoroacetate used in Example 0426-2.
$^1$H-NMR($CDCl_3$)δ:9.13(1H,s),8.92(1H,d,J=2.0Hz),8.84(2H,d,J=11.2Hz),8.25(1H,d,J=9.2Hz),8.10(1H,s),7 .97(1H,s),7.86(1H,s),7.59(1H,d,J=8.6Hz),4.30(2H,t,J=6.9Hz),3.28(4H,t,J=5.0Hz),3.06-3.04(1H,m),2.82(6H,s),2.43-2.40(6H,m),2.23-2.12(2H,m),1.42(6H,d,J=6.6Hz).
MSm/z(M+H):529.

<Example 0434>

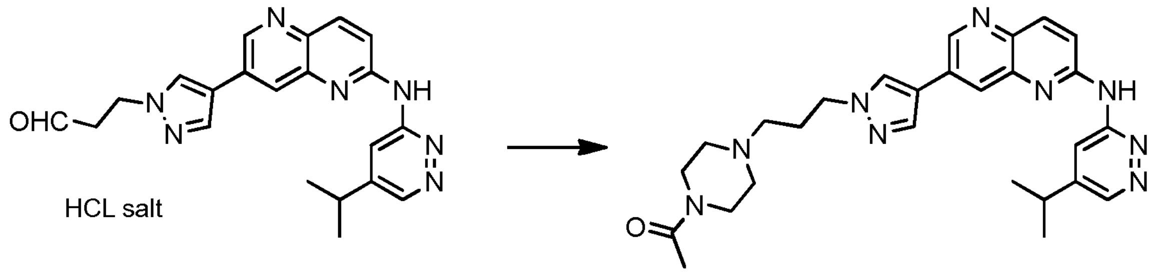

1-(4-(3-(4-(6-((5-isopropylpyridazin-3-yl)amino)-1,5-naphthyridin-3-yl)-1H-pyrazol-1-yl)propyl)piperazin-1-yl)ethanone was obtained in the same manner as in Example 0426-2 except that 1-(piperazin-1-yl)ethanone was used instead of the (2S,6R)-1,2,6-trimethylpiperazinetrifluoroacetate used in Example 0426-2.
$^1$H-NMR($CDCl_3$)δ:8.92(1H,d,J=2.0Hz),8.85-8.78(1H,m),8.24(1H,d,J=9.2Hz),8.10(1H,d,J=1.3Hz),7.97(1H,s),7.86(1H,s),7.56-7.53(1H,m),4.31(2H,t,J=6.9Hz),3.68-3.43(4H,m),3.40(3H,d,J=2.0Hz),3.05(1H,t,J=6.9Hz),2.90-2.83(2H,m),2.46-2.39(6H,m),1.41(6H,d,J=7.3Hz).
MSm/z(M+H):500.

<Example 0435>

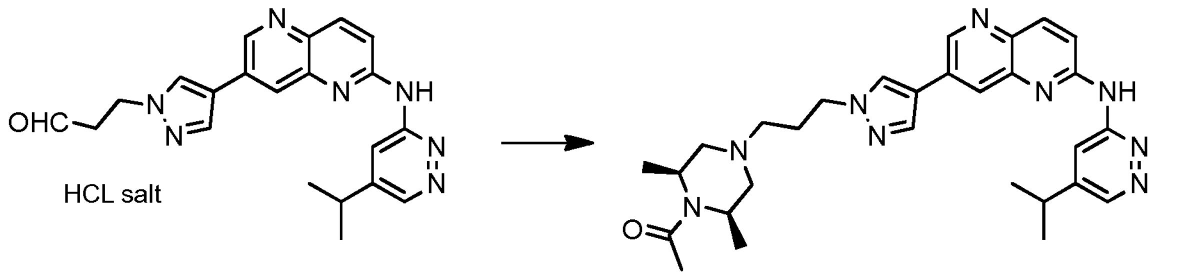

1-((2S,6R)-4-(3-(4-(6-((5-isopropylpyridazin-3-yl)amino)-1,5-naphthyridin-3-yl)-1H-pyrazol-1-yl)propyl)-2,6-dimethylpiperazin-1-yl)ethanone was obtained as a pale yellow solid in the same manner as in Example 0426-2 except that 1-((2S,6R)-2,6-dimethylpiperazin-1-yl)ethanone was used instead of the (2S,6R)-1,2,6-trimethylpiperazinetrifluoroacetate used in Example 0426-2.
$^1$H-NMR($CDCl_3$)δ:8.92-8.92(2H,m),8.79(1H,s),8.24(1H,d,J=9.2Hz),8.10-8.09(1H,m),7.99(1H,s),7.87(1H,s),7.66-7.63(1H,m),4.37(2H,t,J=6.9Hz),3.08-2.95(2H,m),2.70(2H,d,J=11.2Hz),2.35(2H,t,J=6.3Hz),2.19-2.08(8H,m),1.43-1.41(12H,m).
MSm/z(M+H):528.

<Example 0436>

<0436-1>

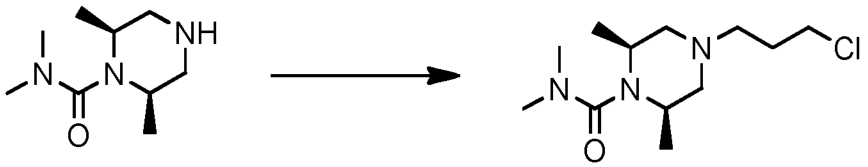

(2S,6R)-4-(3-chloropropyl)-N,N,2,6-tetramethylpiperazine-1-carboxamide was obtained as pale yellow oily substance in the same manner as in Example 0432-1 except that (2S,6R)-N,N,2,6-tetramethylpiperazine-1-carboxamide was used instead of the (2S,6R)-2,6-dimethylpiperidine used in Example 0432-1.
MSm/z(M+H):262.

<0436-2>

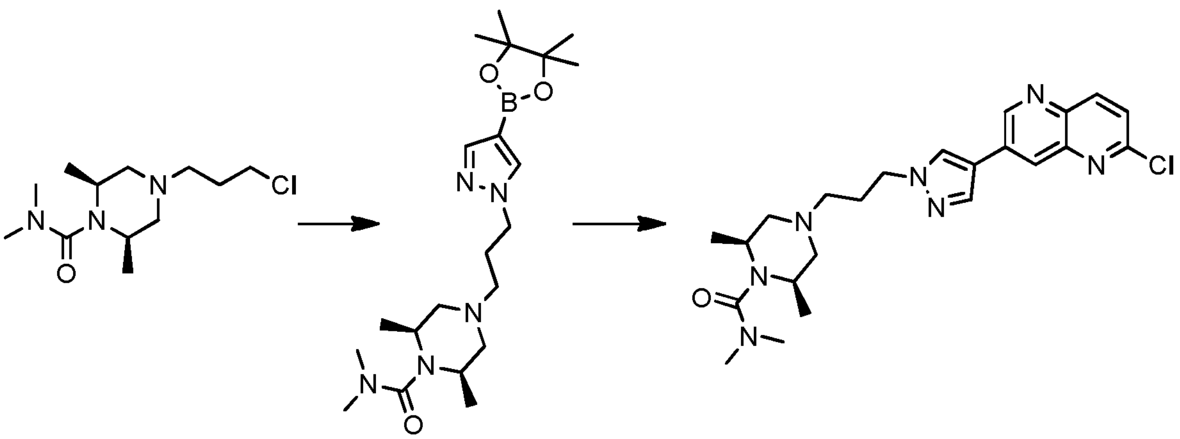

(2S,6R)-4-(3-(4-(6-chloro-1,5-naphthyridin-3-yl)-1H-pyrazol-1-yl)propyl)-N,N,2,6-tetramethylpiperazine-1-carboxamide was obtained as a pale yellow solid in the same manner as in Example 0415-1 except that (2S,6R)-4-(3-chloropropyl)-N,N,2,6-tetramethylpiperazine-1-carboxamide was used instead of the 1-(3-chloropropyl)piperidin-2-one used in Example 0415-1.
MSm/z(M+H):456.

<0436-3>

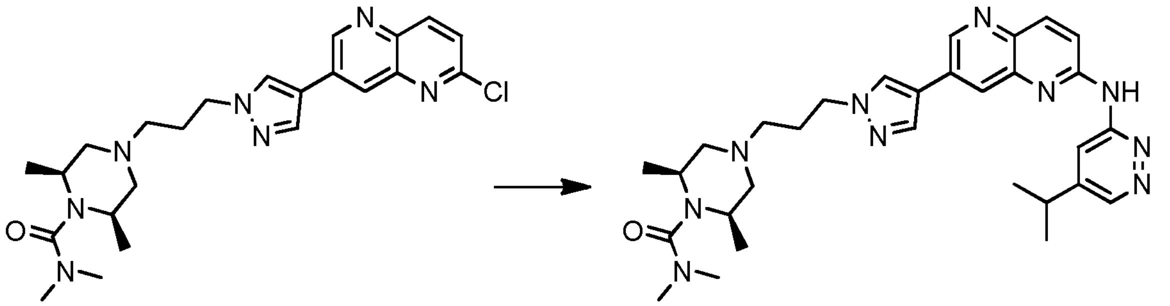

(2S,6R)-4-(3-(4-(6-((5-isopropylpyridazin-3-yl)amino)-1,5-naphthyridin-3-yl)-1H-pyrazol-1-yl)propyl)-N,N,2,6-tetramethylpiperazine-1-carboxamide was obtained in the same manner as in Example 0411-3 except that (2S,6R)-4-(3-(4-(6-chloro-1,5-naphthyridin-3-yl)-1H-pyrazol-1-yl)propyl)-N,N,2,6-tetramethylpiperazine-1-carboxamide was used instead of the 2-chloro-7-(1-((6-methylpyridin-2-yl)methyl)-1H-pyrazol-4-yl)-1,5-naphthyridine used in Example 0411-3.
$^1$H-NMR($CDCl_3$)δ:8.93(1H,d,J=2.0Hz),8.82(2H,s),8.64(1H,brs),8.27-8.24(1H,m),8.12-8.09(1H,m),8.00-7.97(1H,m),7.85(1H,s),7.52-7.49(1H,m),4.41-4.33(1H,m),4.20(2H,t,J=6.6Hz),3.87(1H,t,J=5.6Hz),3.47-3.45(2H,m),3.11-3.02(1H,m),2.80(6H,s),2.62-2.59(4H,m),2.17-2.06(2H,m),1.42(6H,d,J=7.3Hz),1.03(6H,d,J=4.6Hz).
MSm/z(M+H):557.

<Example 0437>

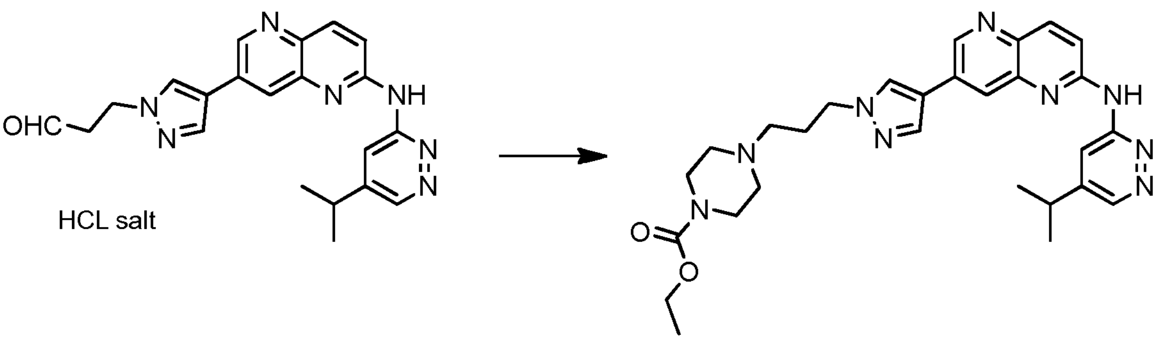


Ethyl 4-(3-(4-(6-((5-isopropylpyridazin-3-yl)amino)-1,5-naphthyridin-3-yl)-1H-pyrazol-1-yl)propyl)piperazine-1-carboxylate was obtained as a yellow solid in the same manner as in Example 0426-2 except that ethyl piperazine-1-carboxylate was used instead of the (2S,6R)-1,2,6-trimethylpiperazinetrifluoroacetate used in Example 0426-2.
$^1$H-NMR($CDCl_3$)δ:8.93-8.92(1H,m),8.80(2H,s),8.26-8.23(1H,m),8.09-8.08(1H,m),7.97(1H,s),7.86(1H,s),7.48-7.45(1H,m),4.30(2H,t,J=6.6Hz),4.14(2H,q,J=7.0Hz),3.50(4H,t,J=5.0Hz),3.06(1H,t,J=6.6Hz),2.41-2.38(6H,m),2.15-2.13(2H,m),1.42(6H,d,J=6.6Hz),1.26(3H,t,J=12.2Hz).
MSm/z(M+H):530.

<Example 0438>

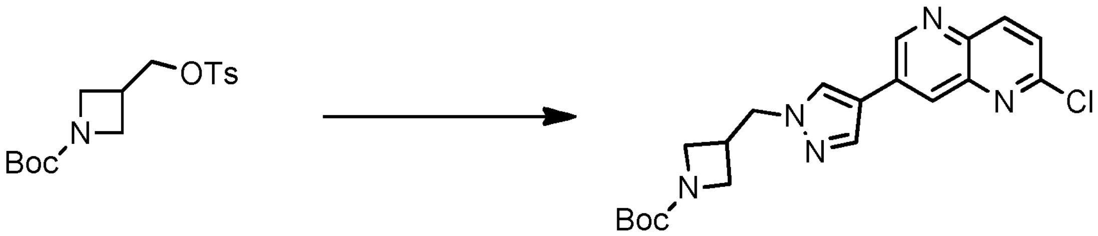

<0438-1>

A mixture of 2-chloro-7-(1H-pyrazol-4-yl)-1,5-naphthyridine (146 mg), tert-butyl 3-((paratoluenesulfonyloxy)methyl)azetidine-1-carboxylate (259 mg), potassium carbonate (174 mg), and N,N-dimethylformamide (2 mL) was stirred at 100°C for 1 hour. After the reaction mixture was cooled to room temperature, water and ethyl acetate were added thereto, the organic layer was collected by separation, washed with a saturated sodium chloride aqueous solution, and dried over anhydrous sodium sulfate, and the solvent was distilled off under reduced pressure, thereby obtaining tert-butyl 3-((4-(6-chloro-1,5-naphthyridin-3-yl)-1H-pyrazol-1-yl)methyl)azetidine-1-carboxylate (273 mg) as a pale brown solid.
MSm/z(M+H):400.

<0438-2>

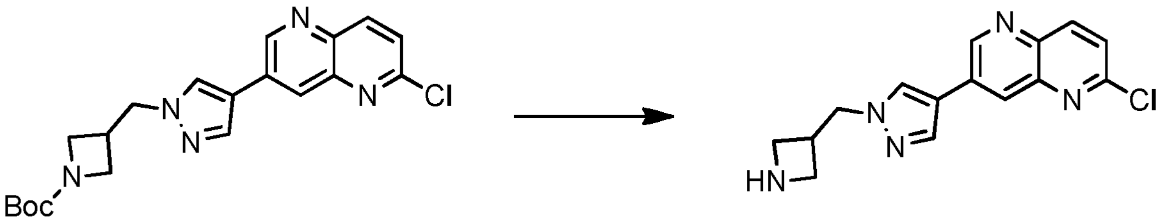

Trifluoroacetic acid (1.5 mL) was added to a mixture of tert-butyl 3-((4-(6-chloro-1,5-naphthyridin-3-yl)-1H-pyrazol-1-yl)methyl)azetidine-1-carboxylate (273 mg), and dichloromethane (3 mL), followed by stirring at room temperature for 30 minutes. The solvent was distilled off under reduced pressure, and the obtained residue was purified by silica gel column chromatography (ethyl acetate-methanol, NH silica), thereby obtaining 7-(1-(azetidin-3-ylmethyl)-1H-pyrazol-4-yl)-2-chloro-1,5-naphthyridine (104 mg) as a yellow solid.
MSm/z(M+H):300.

<0438-3>

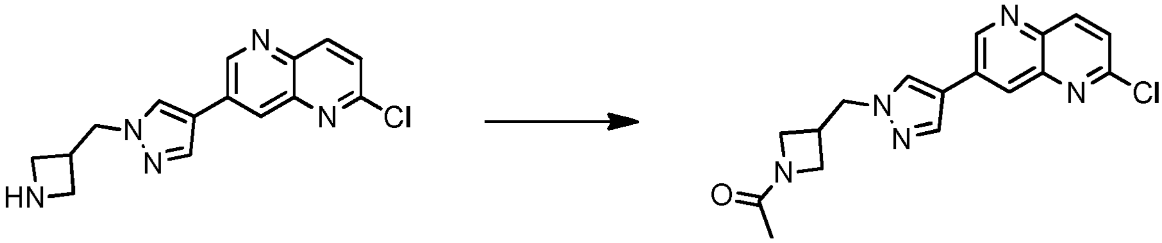

Acetyl chloride (13 μL) was added to a mixture of 7-(1-(azetidin-3-ylmethyl)-1H-pyrazol-4-yl)-2-chloro-1,5-naphthyridine (36 mg), triethylamine (33 μL), and dichloromethane (2.6 mL) under ice-cooling, followed by stirring for 1.5 hours. The reaction mixture was purified by silica gel column chromatography (hexane-ethyl acetate-methanol, NH silica), thereby obtaining 1-(3-((4-(6-chloro-1,5-naphthyridin-3-yl)-1H-pyrazol-1-yl)methyl)azetidin-1-yl)ethanone (9.7 mg) as a yellow solid.

MSm/z(M+H):342.

<0438-4>

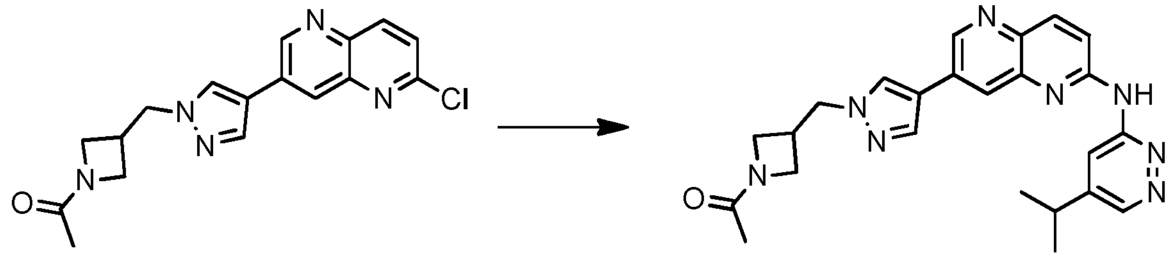

A mixture of 1-(3-((4-(6-chloro-1,5-naphthyridin-3-yl)-1H-pyrazol-1-yl)methyl)azetidin-1-yl)ethanone (9.7 mg), 5-isopropylpyridazine-3-amine (5 mg), potassium tert-butoxide (10.4 mg), and 1,4-dioxane (1 mL) was stirred at 110°C for 1 hour. The reaction mixture was cooled to room temperature, and purified by silica gel column chromatography (ethyl acetate-methanol, NH silica), thereby obtaining 1-(3-((4-(6-((5-isopropylpyridazin-3-yl)amino)-1,5-naphthyridin-3-yl)-1H-pyrazol-1-yl)methyl)azetidin-1-yl)ethanone (1.8 mg) as a pale yellow solid.
$^1$H-NMR($CDCl_3$)δ:8.94-8.91(2H,m),8.84-8.83(1H,m),8.26-8.23(1H,m),8.12-8.08(1H,m),7.97-7.96(1H,m),7.85-7.84(1H,m),7.71-7.69(1H,m),4.27-4.08(6H,m),3.07(1H,t,J=6.9Hz),2.05-2.01(1H,m),1.89(3H,s),1.43(6H,d,J=6.6Hz).
MSm/z(M+H):443.

<Example 0439>

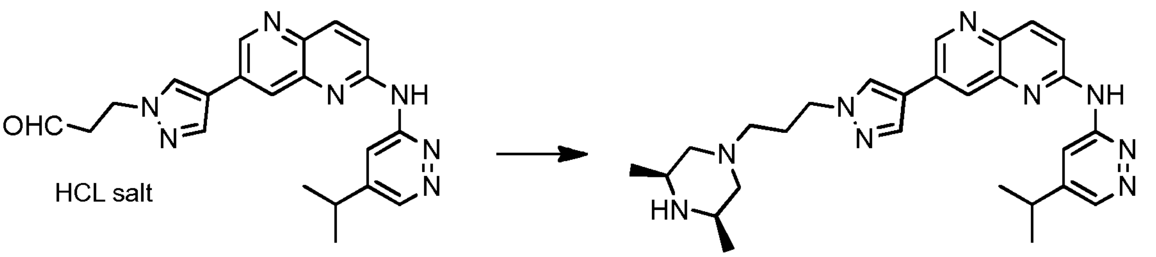


7-(1-(3-((3S,5R)-3,5-dimethylpiperazin-1-yl)propyl)-1H-pyrazol-4-yl)-N-(5-isopropylpyridazin-3-yl)-1,5-naphthyridine-2-amine was obtained as a yellow solid in the same manner as in Example 0426-2 except that (2S,6R)-2,6-dimethylpiperazine was used instead of the (2S,6R)-1,2,6-trimethylpiperazinetrifluoroacetate used in Example 0426-2.
$^1$H-NMR($CDCl_3$)δ:8.92(1H,d,J=2.6Hz),8.75(1H,s),8.24(1H,d,J=9.2Hz),8.10(1H,d,J=1.3Hz),7.97(1H,s),7.86(1H,s),7.57(1H,d,J=9.2Hz),4.28(2H,t,J=6.9Hz),3.08-2.99(3H,m),2.84-2.82(2H,m),2.42(2H,q,J=6.8Hz),2.15(2H,t,J=6.9Hz),1.73(2H,t,J=10.9Hz),1.41(6H,d,J=7.3Hz),1.11-1.09(6H,m).
MSm/z(M+H):486.

<Example 0440>

<0440-1>

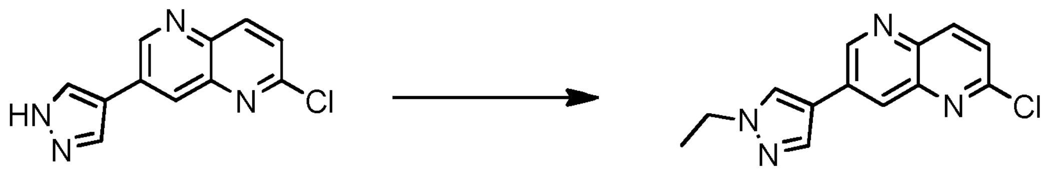

A mixture of 2-chloro-7-(1H-pyrazol-4-yl)-1,5-naphthyridine (49 mg), bromoethane (49 μL), cesium carbonate (91 mg), and N,N-dimethylformamide (1 mL) was stirred at 80°C for 2 hours. After the reaction mixture was cooled to room temperature, water and ethyl acetate were added thereto, the organic layer was collected by separation, washed with a saturated sodium chloride aqueous solution, and dried over anhydrous sodium sulfate, and the solvent was distilled off under reduced pressure, thereby obtaining 2-chloro-7-(1-ethyl-1H-pyrazol-4-yl)-1,5-naphthyridine (58 mg) as a brown solid.
MSm/z(M+H):259.

<0440-2>

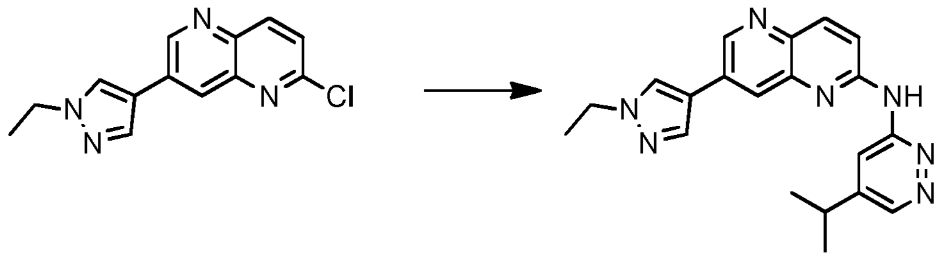

7-(1-Ethyl-1H-pyrazol-4-yl)-N-(5-isopropylpyridazin-3-yl)-1,5-naphthyridine-2-amine was obtained as a pale yellow solid in the same manner as in Example 0438-4 except that 2-chloro-7-(1-ethyl-1H-pyrazol-4-yl)-1,5-naphthyridine was used instead of the 1-(3-((4-(6-chloro-1,5-naphthyridin-3-yl)-1H-pyrazol-1-yl)methyl)azetidin-1-yl)ethanone used in Example 0438-4.
$^1$H-NMR($CD_3OD$)δ:8.97(1H,d,J=2.0Hz),8.85(1H,s),8.77(1H,d,J=2.0Hz),8.34(1H,s),8.29(1H,d,J=1.3Hz),8 .18(1H,d,J=9.2 Hz),8.10(1H,s),7.60(1H,d,J=9.2Hz),4.28(2H,q,J=7.3Hz),3.09(1H,t,J=6.6Hz),1.53(3H,t, J=7.3Hz),1.41(6H,d,J=7.3Hz).
MSm/z(M+H):360.

<Example 0441>

<0441-1>

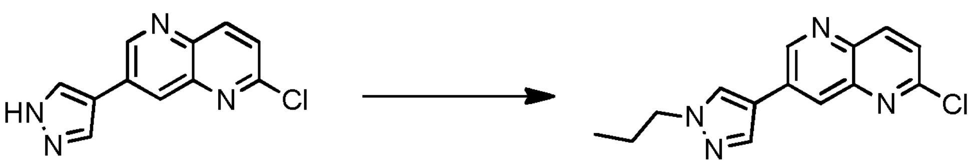

2-Chloro-7-(1-propyl-1H-pyrazol-4-yl)-1,5-naphthyridine was obtained as a brown solid in the same manner as in Example 440-1 except that 1-bromopropane was used instead of the bromoethane used in Example 440-1.
MSm/z(M+H):273.

<0441-2>

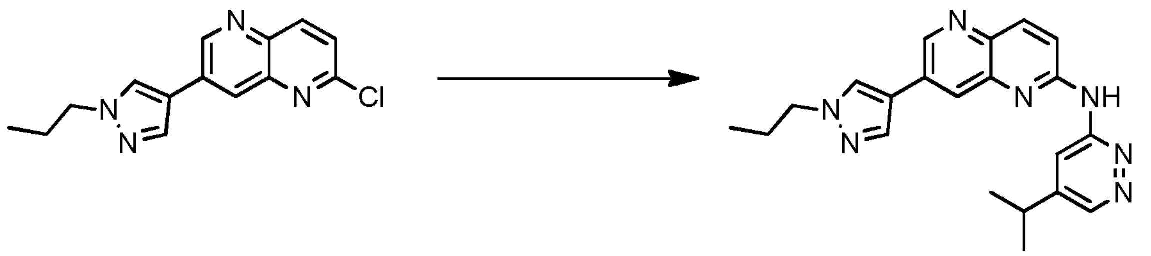

N-(5-isopropylpyridazin-3-yl)-7-(1-propyl-1H-pyrazol-4-yl)-1,5-naphthyridine-2-amine was obtained as a pale yellow solid in the same manner as in Example 0440-2 except that 2-chloro-7-(1-propyl-1H-pyrazol-4-yl)-1,5-naphthyridine was used instead of the 2-chloro-7-(1-ethyl-1H-pyrazol-4-yl)-1,5-naphthyridine used in Example 0440-2.
$^1$H-NMR($CD_3OD$)δ:8.97(1H,d,J=2.0Hz),8.85(1H,s),8.78(1H,d,J=2.0Hz),8.34(1H,s),8.30(1H,d,J=2.0Hz),8 .19(1H,d,J=9.2 Hz),8.11(1H,s),7.61(1H,d,J=9.2Hz),4.21(2H,t,J=7.3Hz),3.09(1H,t,J=6.9Hz),2.01-1.92(2H,m),1.42(6H,d,J=6.6Hz),0.96(3H,t,J=7.3Hz).
MSm/z(M+H):374.

<Example 0442>

<0442-1>

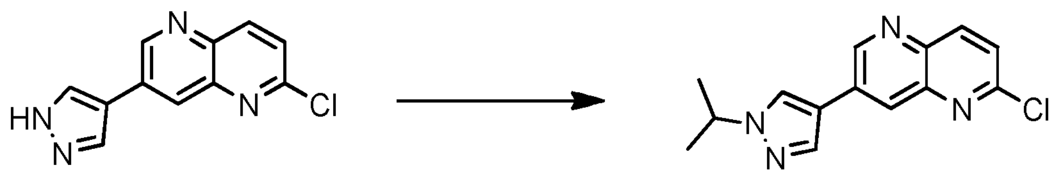

2-Chloro-7-(1-isopropyl-1H-pyrazol-4-yl)-1,5-naphthyridine was obtained as a brown solid in the same manner as in Example 0440-1 except that 2-bromopropane was used instead of the bromoethane used in Example 0440-1.
MSm/z(M+H):273.

<0442-2>

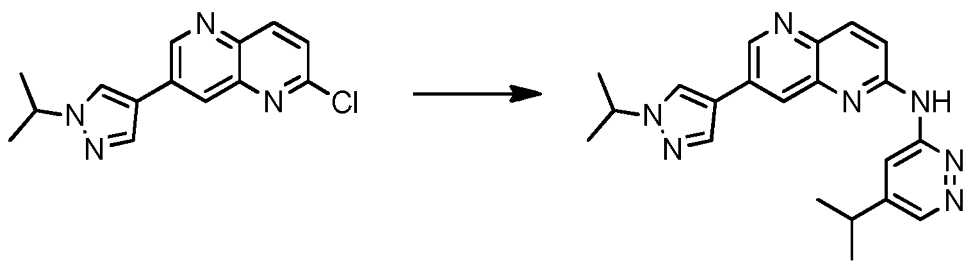

7-(1-Isopropyl-1H-pyrazol-4-yl)-N-(5-isopropylpyridazin-3-yl)-1,5-naphthyridine-2-amine was obtained as a pale yellow solid in the same manner as in Example 0440-2 except that 2-chloro-7-(1-isopropyl-1H-pyrazol-4-yl)-1,5-naphthyridine was used instead of the 2-chloro-7-(1-ethyl-1H-pyrazol-4-yl)-1,5-naphthyridine used in Example 0440-2.
$^1$H-NMR($CD_3OD$)δ:8.99(1H,d,J=2.0Hz),8.85(1H,s),8.78(1H,d,J=1.3Hz),8.39(1H,s),8.30(1H,d,J=1.3Hz),8 .19(1H,d,J=9.2 Hz),8.10(1H,s),7.61(1H,d,J=8.6Hz),4.63(1H,t,J=6.6Hz),3.10-3.04(1H,m),1.58(6H,d,J=6.6Hz),1.42(6H,d,J=7.3Hz).
MSm/z(M+H):374.

<Example 0443>

<0443-1>

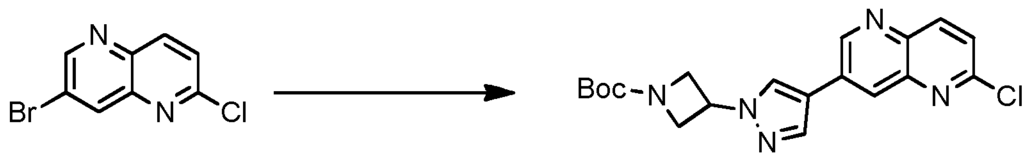

tert-Butyl 3-(4-(6-chloro-1,5-naphthyridin-3-yl)-1H-pyrazol-1-yl)azetidine-1-carboxylate was obtained as a pale yellow solid in the same manner as in Example 0421-1 except that tert-butyl 3-(4-bromo-1H-pyrazol-1-yl)azetidine-1-carboxylate was used instead of the N-(4-bromopyridin-2-yl)acetamide used in Example 0421-1.
MSm/z(M+H):386.

<0443-2>

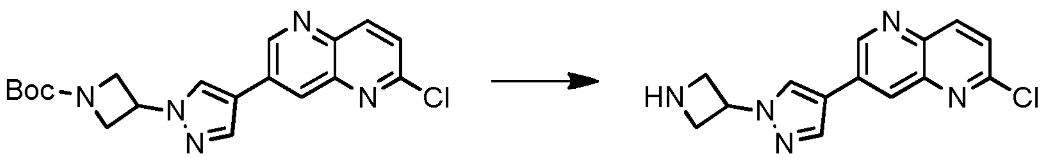

7-(1-(Azetidin-3-yl)-1H-pyrazol-4-yl)-2-chloro-1,5-naphthyridine was obtained as a pale yellow solid in the same manner as in Example 0438-2 except that tert-butyl 3-(4-(6-chloro-1,5-naphthyridin-3-yl)-1H-pyrazol-1-yl)azetidine-1-carboxylate was used instead of the tert-butyl 3-((4-(6-chloro-1,5-naphthyridin-3-yl)methyl)azetidine-1-carboxylate used in Example 0438-2.
MSm/z(M+H):286.

<0443-3>

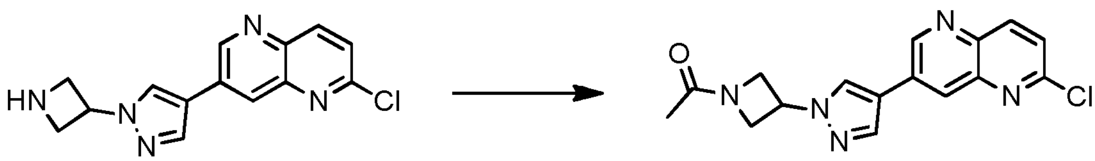

1-(3-(4-(6-Chloro-1,5-naphthyridin-3-yl)-1H-pyrazol-1-yl))azetidin-1-yl)ethanone was obtained as a white solid in the same manner as in Example 0438-3 except that 7-(1-(azetidin-3-yl)-1H-pyrazol-4-yl)-2-chloro-1,5-naphthyridine was used instead of the 7-(1-(azetidin-3-ylmethyl)-1H-pyrazol-4-yl)-2-chloro-1,5-naphthyridine used in Example 0438-3.
MSm/z(M+H):328.

<0443-4>

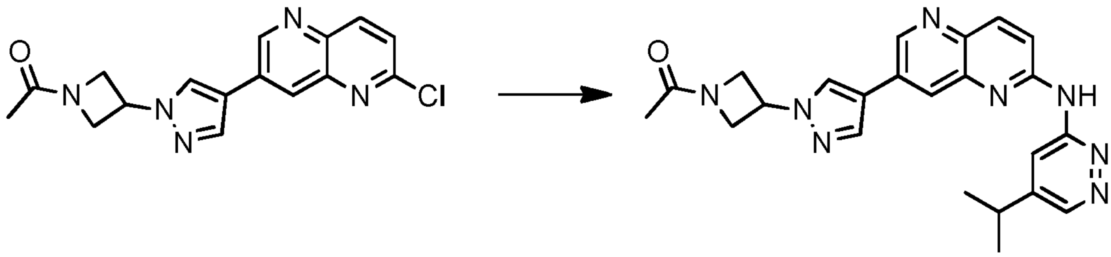

1-(3-(4-(6-((5-Isopropylpyridazin-3-yl)amino)-1,5-naphthyridin-3-yl)-1H-pyrazol-1-yl)azetidin-1-yl)ethanone was obtained as a pale brown solid in the same manner as in Example 0411-3 except that 1-(3-(4-(6-chloro-1,5-naphthyridin-3-yl)-1H-pyrazol-1-yl))azetidin-1-yl)ethanone was used instead of the 2-chloro-7-(1-((6-methylpyridin-2-yl)methyl)-1H-pyrazol-4-yl)-1,5-naphthyridine used in Example 0411-3.
$^1$H-NMR($CDCl_3$)δ:9.10(1H,brs),8.93(1H,d,J=2.6Hz),8.85-8.83(2H,m),8.26(1H,d,J=9.2Hz),8.12(1H,d,J=1.3Hz),8.07(1H,s),7.96(1H,s),7.62(1H,d,J=9.2Hz),5.24-5.19(1H,m),4.64-4.55(4H,m),3.07(1H,t,J=6.9Hz),1.99(3H,s),1.43(6H,d,J=6.6Hz).
MSm/z(M+H):429.

<Example 0444>

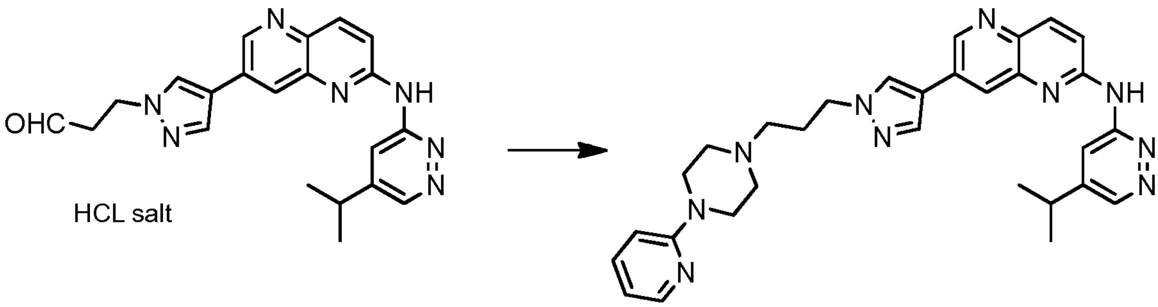


N-(5-isopropylpyridazin-3-yl)-7-(1-(3-(4-(pyridin-2-yl)piperazin-1-yl)propyl)-1H-pyrazol-4-yl)-1,5-naphthyridine-2-amine was obtained as a pale yellow solid in the same manner as in Example 0426-2 except that 1-(pyridin-2-yl)piperazine was used instead of the (2S,6R)-1,2,6-trimethylpiperazinetrifluoroacetate used in Example 0426-2.
$^1$H-NMR($CDCl_3$)δ:8.93(1H,d,J=2.6Hz),8.81(2H,s),8.24(1H,d,J=9.2Hz),8.20-8.19(1H,m),8.10(1H,d,J=2.0Hz),7.98(1H,s),7.89(1H,s),7.50-7.46(2H,m),6.66-6.61(2H,m),4.33(2H,t,J=6.9Hz),3.57(4H,t,J=5.0Hz),3.05(1H,t,J=6.9Hz),2.57(4H,t,J=5.0Hz),2.44(2H,t, J=6.9Hz),2.18(2H,t,J=6.9Hz),1.41(6H,d,J=6.6Hz).
MSm/z(M+H):535.

<Example 0445>

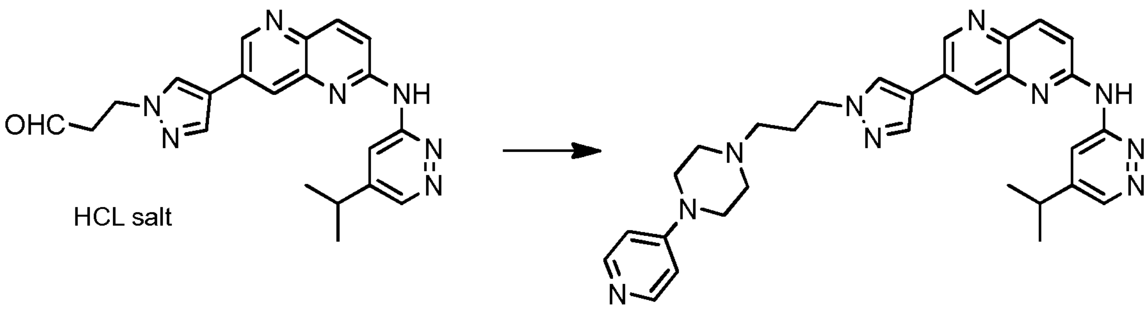

N-(5-isopropylpyridazin-3-yl)-7-(1-(3-(4-(pyridin-4-yl)piperazin-1-yl)propyl)-1H-pyrazol-4-yl)-1,5-naphthyridine-2-amine was obtained as a pale yellow solid in the same manner as in Example 0426-2 except that 1-(pyridin-4-yl)piperazine was used instead of the (2S,6R)-1,2,6-trimethylpiperazinetrifluoroacetate used in Example 0426-2. $^1$H-NMR($CDCl_3$)δ:8.94-8.91(2H,m),8.82(2H,s),8.27-8.25(3H,m),8.10(1H,s),7.98(1H,s),7.88(1H,s),7.59-7.56(1H,m),6.66(2H,t,J=3.3Hz),4.33(2H,t,J=6.9Hz),3.36(4H,t,J=5.0Hz),3.05(1H,t,J=6.6Hz),2.58(4H,t, J=5.0Hz),2.44(2H,t,J=6.9Hz),2.21-2.13(2H,m),1.42(6H,d,J=6.6Hz).
MSm/z(M+H):535.

<Example 0446>

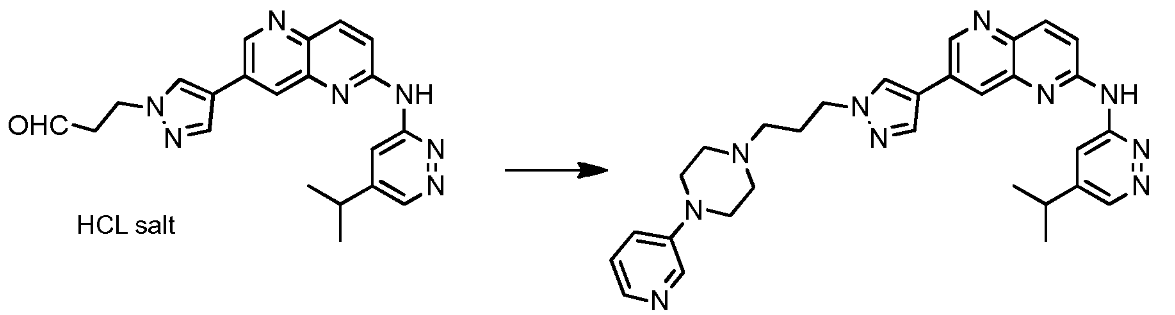


N-(5-isopropylpyridazin-3-yl)-7-(1-(3-(4-(pyridin-3-yl)piperazin-1-yl)propyl)-1H-pyrazol-4-yl)-1,5-naphthyridine-2-amine was obtained as a pale yellow solid in the same manner as in Example 0426-2 except that 1-(pyridin-3-yl)piperazine was used instead of the (2S,6R)-1,2,6-trimethylpiperazinetrifluoroacetate used in Example 0426-2. $^1$H-NMR($CDCl_3$)δ:8.93(1H,s),8.79-8.77(1H,m),8.32(1H,s),8.25(1H,d,J=8.6Hz),8.10-8.09(2H,m),7.98(1H,s),7.89(1H,s),7.50-7.47(1H,m),7.20-7.18(2H,m),4.33(2H,t,J=6.6Hz),3.27(4H,t,J=4.6Hz),3.03(1H,t,J=6.6Hz),2.66-2.63(4H,m),2.48-2.46(2H,m),2.21-2.17(2H,m),1.40(6H,d,J=6.6Hz).
MSm/z(M+H):535.

<Example 0447> (not in accordance with the present invention)

<0447-1>

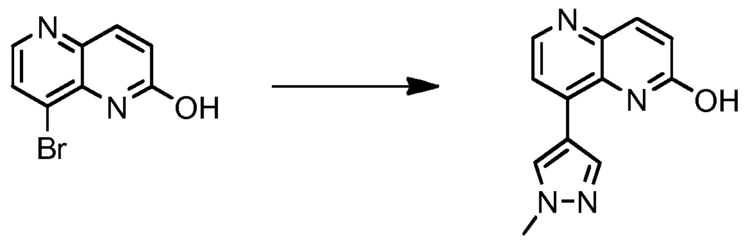

A mixture of 8-bromo-1,5-naphthyridin-2-ol (199 mg), 1-methyl-4-(4,4,5,5-tetramethyl-1,3,2-dioxaborolan-2-yl)-1H-pyrazole (202 mg), sodium carbonate (191 mg), bis(di-tert-butyl (4-dimethylaminophenyl)phosphine)dichloropalladium(II) (32 mg), 1,4-dioxane (3 mL), and water (0.3 mL) was stirred at 100°C for 1 hour in a nitrogen atmosphere. The reaction mixture was cooled to room temperature, water and ethyl acetate were added thereto, and the organic layer was collected by separation. The organic layer was purified by silica gel column chromatography (hexane-ethyl acetate-methanol), thereby obtaining 8-(1-methyl-1H-pyrazol-4-yl)-1,5-naphthyridin-2-ol (24.7 mg) as a white solid.
MSm/z(M+H):227.

<0447-2>

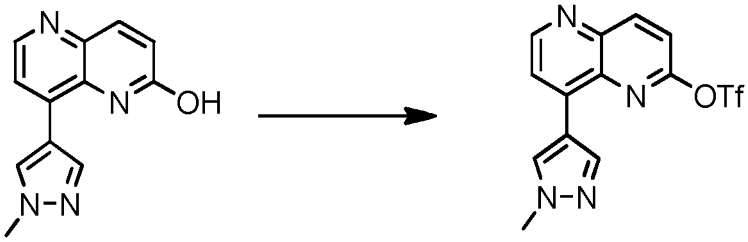

Triethylamine (0.1 mL) was added to a mixture of 8-(1-methyl-1H-pyrazol-4-yl)-1,5-naphthyridin-2-ol (24.7 mg) and dichloromethane (2 mL) at room temperature, and trifluoromethanesulfonic aicd anhydride (0.05 mL) was added thereto at a temperature of from 0°C to 5°C, followed by stirring at 0°C for 1.5 hours. The reaction mixture was purified by silica gel column chromatography (hexane-ethyl acetate-methanol), thereby obtaining 8-(1-methyl-1H-pyrazol-4-yl)-1,5-naphthyridin-2-yl trifluoromethanesulfonate (11.6 mg) as a yellowish green solid.
MSm/z(M+H):359.

<0447-3>

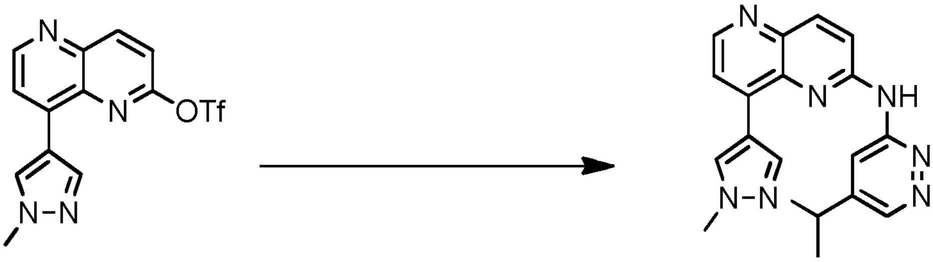

N-(5-isopropylpyridazin-3-yl)-8-(1-methyl-1H-pyrazol-4-yl)-1,5-naphthyridine-2-amine was obtained as a white solid in the same manner as in Example 0411-3 except that 8-(1-methyl-1H-pyrazol-4-yl)-1,5-naphthyridin-2-yl trifluoromethanesulfonate was used instead of the 2-chloro-7-(1-((6-methylpyridin-2-yl)methyl)-1H-pyrazol-4-yl)-1,5-naphthyridine used in Example 0411-3.
$^1$H-NMR(DMSO-$d_6$)δ:10.49(1H,s),8.91(1H,s),8.87(1H,d,J=2.0Hz),8.66(1H,d,J=4.6Hz),8.35(1H,s),8.27(1H,d,J=9.2Hz), 8.00(1H,s),7.95(1H,d,J=8.6Hz),7.83(1H,d,J=4.6Hz),3.95(3H,s),1.20(6H,d,J=7.3Hz).
MSm/z(M+H):346.

<Example 0448> (not in accordance with the present invention)

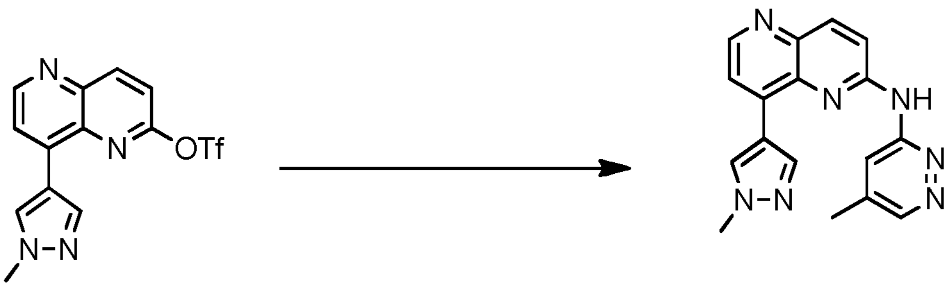

8-(1-Methyl-1H-pyrazol-4-yl)-N-(5-methylpyridazin-3-yl)-1,5-naphthyridine-2-amine was obtained as a yellow

solid in the same manner as in Example 0447-3 except that 5-methylpyridazine-3-amine was used instead of the 5-isopropylpyridazine-3-amine used in Example 0447-3. $^1$H-NMR(DMSO-$d_6$)$\delta$:10.56(1H,s),8.79-8.78(2H,m),8.65(1H,d,J=4.6Hz),8.33(1H,s),8.26-8.25(2H,m),7.82(2H,t,J=4.6Hz),3.96(3H,s),2.31(3H,s).
MSm/z(M+H):318.

<Example 0449>

<0449-1>

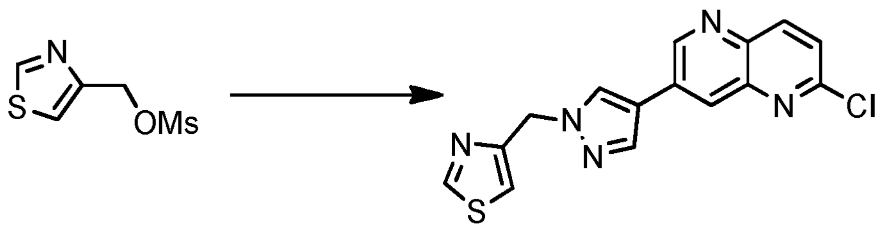

4-((4-(6-Chloro-1,5-naphthyridin-3-yl)-1H-pyrazol-1-yl)methyl)thiazole was obtained as a pale yellow solid in the same manner as in Example 0440-1 except that (thiazol-4-yl)methyl methanesulfonate was used instead of the bromoethane used in Example 0440-1.
MSm/z(M+H):328.

<0449-2>

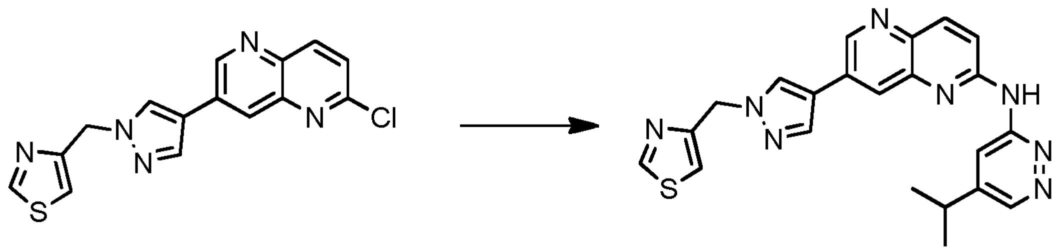

N-(5-isopropylpyridazin-3-yl)-7-(1-((thiazol-4-yl)methyl)-1H-pyrazol-4-yl)-1,5-naphthyridine-2-amine was obtained as a pale yellow solid in the same manner as in Example 0411-3 except that 4-((4-(6-chloro-1,5-naphthyridin-3-yl)-1H-pyrazol-1-yl)methyl)thiazole was used instead of the 2-chloro-7-(1-((6-methylpyridin-2-yl)methyl)-1H-pyrazol-4-yl)-1,5-naphthyridine used in Example 0411-3.
$^1$H-NMR($CD_3OD$)$\delta$:9.02(1H,d,J=2.0Hz),8.98(1H,d,J=2.6Hz),8.85(1H,s),8.77(1H,d,J=2.0Hz),8.44(1H,s),8 .31-8.30(1H,m),8.19-8.16(2H,m),7.61(1H,d,J=9.2Hz),7.55-7.55(1H,m),4.60(2H,s),3.08(1H,t,J=3.0Hz),1.41(6H,d,J=6.6Hz).
MSm/z(M+H):429.

<Example 0450>

<0450-1>

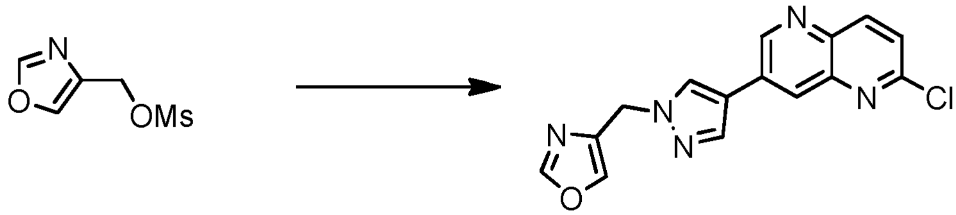

4-((4-(6-Chloro-1,5-naphthyridin-3-yl)-1H-pyrazol-1-yl)methyl)oxazole was obtained as a yellow solid in the same manner as in Example 0440-1 except that (oxazol-4-yl)methyl methanesulfonate was used instead of the bromoethane used in Example 0440-1.
MSm/z(M+H):312.

<0450-2>

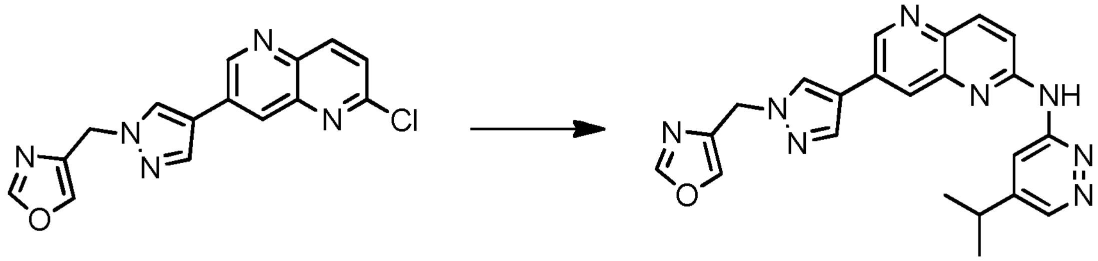

N-(5-isopropylpyridazin-3-yl)-7-(1-((oxazol-4-yl)methyl)-1H-pyrazol-4-yl)-1,5-naphthyridine-2-amine was obtained as a pale yellow solid in the same manner as in Example 0411-3 except that 4-((4-(6-chloro-1,5-naphthyridin-3-yl)-1H-pyrazol-1-yl)methyl)oxazole was used instead of the 2-chloro-7-(1-((6-methylpyridin-2-yl)methyl)-1H-pyrazol-4-yl)-1,5-naphthyridine used in Example 0411-3.
$^1$H-NMR(DMSO-$d_6$)$\delta$:10.70(1H,s),9.05(1H,d,J=2.0Hz),8.87(1H,d,J=2.0Hz),8.74(1H,d,J=1.3Hz),8.56(1H,s),8.40(1H,s), 8.24-8.18(4H,m),7.70(1H,d,J=9.2Hz),5.34(2H,s),3.04(1H,t,J=6.9Hz),1.33(6H,d,J=7.3Hz).
MSm/z(M+H):413.

<Example 0451>

<0451-1>

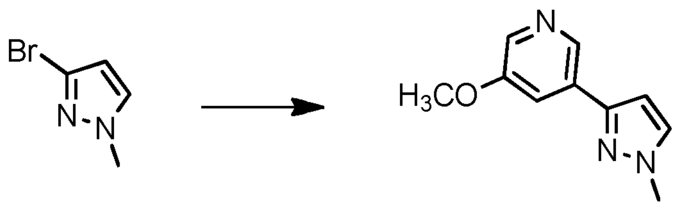

A mixture of 3-bromo-1-methyl-1H-pyrazole (112 mg), 3-methoxy-5-(4,4,5,5-tetramethyl-1,3,2-dioxaborolan-2-yl)pyridine (175 mg), sodium carbonate (133 mg), bis(di-tert-butyl (4-dimethylaminophenyl)phosphine)dichloropalladium(II) (21 mg), 1,4-dioxane (2.1 mL), and water (0.21 mL) was stirred at 100°C for 1 hour in a nitrogen atmosphere. The reaction mixture was cooled to room temperature, and purified by silica gel column chromatography (hexane-ethyl acetate-methanol), thereby obtaining 3-methoxy-5-(1-methyl-1H-pyrazol-3-yl)pyridine (45 mg) as a pale yellow solid.
MSm/z(M+H):190.

<0451-2>

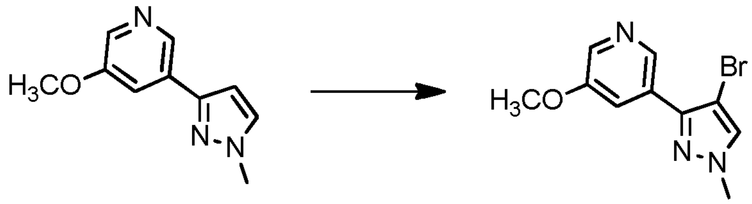

N-bromosuccinimide (23.4 mg) was added to a solution of 3-methoxy-5-(1-methyl-1H-pyrazol-3-yl)pyridine (22.3 mg) in N,N-dimethylformamide (1.2 mL), followed by stirring at room temperature for 1 hour. After a 10% sodium carbonate aqueous solution, a 10% sodium hydrogen sulfite aqueous solution, and ethyl acetate were added to the reaction mixture, the organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate), thereby obtaining 3-(4-bromo-1-methyl-1H-pyrazol-3-yl)-5-methoxypyridine (19 mg) as pale yellow oily substance.
MSm/z(M+H):268.

<0451-3>

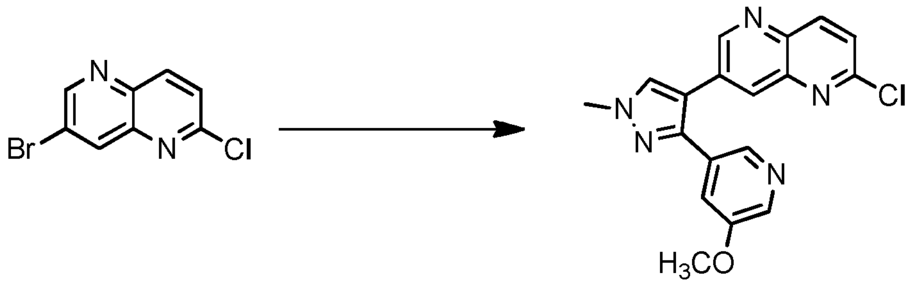

2-Chloro-7-(3-(5-methoxypyridin-3-yl)-1-methyl-1H-pyrazol-4-yl)-1,5-naphthyridine was obtained as a white solid in the same manner as in Example 0421-1 except that 3-(4-bromo-1-methyl-1H-pyrazol-3-yl)-5-methoxypyridine was used instead of the N-(4-bromopyridin-2-yl)acetamide used in Example 0421-1.
MSm/z(M+H):352.

<0451-4>

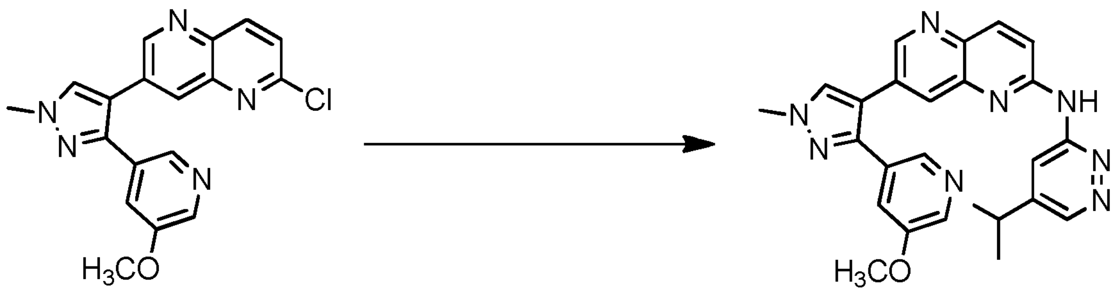

N-(5-isopropylpyridazin-3-yl)-7-(3-(5-methoxypyridin-3-yl)-1-methyl-1H-pyrazol-4-yl)-1,5-naphthyridine-2-amine was obtained as a pale yellow solid in the same manner as in Example 0411-3 except that 2-chloro-7-(3-(5-methoxypyridin-3-yl)-1-methyl-1H-pyrazol-4-yl)-1,5-naphthyridine was used instead of the 2-chloro-7-(1-((6-methylpyridin-2-yl)methyl)-1H-pyrazol-4-yl)-1,5-naphthyridine used in Example 0411-3.
$^1$H-NMR($CD_3OD$)$\delta$:8.80(1H,s),8.74(1H,d,J=1.3Hz),8.69(1H,d,J=2.0Hz),8.22-8.19(4H,m),7.98(1H,d,J=1.3Hz),7.60(1H,d,J=9.2Hz),7.55(1H,t,J=2.3Hz),4.06(3H,s),3.81(3H,s),3.01(1 H,t,J=6.9Hz),1.31(6H,d,J=7.3Hz).
MSm/z(M+H):453.

<Example 0452>

<0452-1>

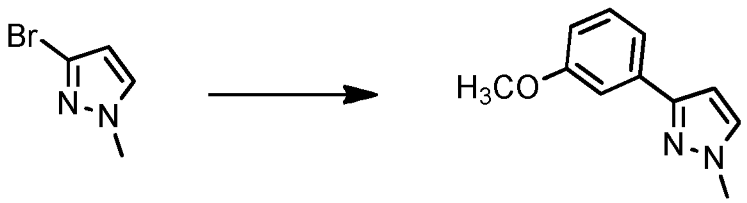

3-(3-Methoxyphenyl)-1-methyl-1H-pyrazole was obtained as pale yellow oily substance in the same manner as in Example 0451-1 except that 2-(3-methoxyphenyl)-4,4,5,5-tetramethyl-1,3,2-dioxaborolane was used instead of the 3-methoxy-5-(4,4,5,5-tetramethyl-1,3,2-dioxaborolan-2-yl)pyridine used in Example 0451-1.
MSm/z(M+H):189.

<0452-2>

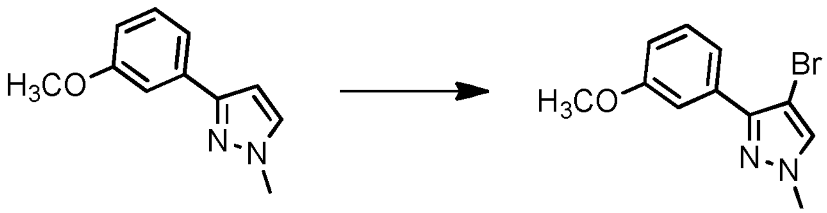

4-Bromo-3-(3-methoxyphenyl)-1-methyl-1H-pyrazole was obtained as yellow oily substance in the same manner as in Example 0451-2 except that 3-(3-methoxyphenyl)-1-methyl-1H-pyrazole was used instead of the 3-methoxy-5-(1-methyl-1H-pyrazol-3-yl)pyridine used in Example 0451-2.
MSm/z(M+H):269.

<0452-3>

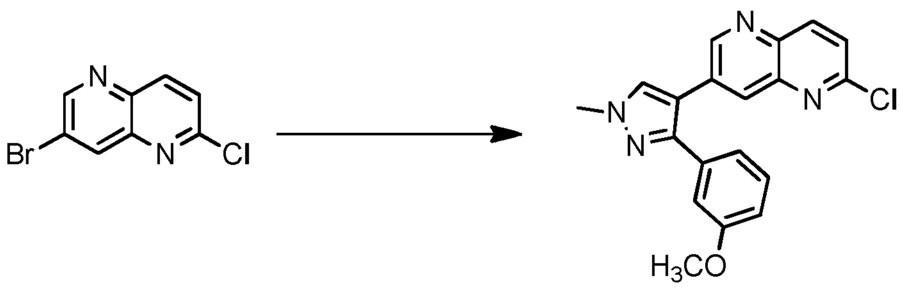

2-Chloro-7-(3-(3-methoxyphenyl)-1-methyl-1H-pyrazol-4-yl)-1,5-naphthyridine was obtained as a brown solid

in the same manner as in Example 0451-3 except that 4-bromo-3-(3-methoxyphenyl)-1-methyl-1H-pyrazole was used instead of the 3-(4-bromo-1-methyl)-1H-pyrazol-3-yl)-5-methoxypyridine used in Example 0451-3.
MSm/z(M+H):351.

<0452-4>

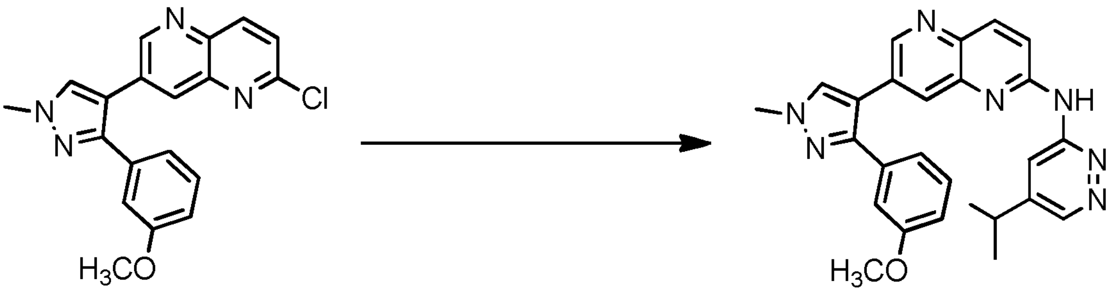

N-(5-isopropylpyridazin-3-yl)-7-(3-(3-methoxyphenyl)-1-methyl-1H-pyrazol-4-yl)-1,5-naphthyridine-2-amine was obtained as a white solid in the same manner as in Example 0451-4 except that 2-chloro-7-(3-(3-methoxyphenyl)-1-methyl-1H-pyrazol-4-yl)-1,5-naphthyridine was used instead of the 2-chloro-7-(3-(5-methoxypyridin-3-yl)-1-methyl-1H-pyrazol-4-yl)-1,5-naphthyridine used in Example 0451-4.
$^1$H-NMR($CDCl_3$)δ:9.37(1H,brs),8.86(1H,d,J=1.3Hz),8.79(1H,d,J=2.0Hz),8.70(1H,d,J=2.0Hz),8.23(1H,d,J =8.6Hz),7.97(1H,d,J=2.0Hz),7.70(1H,s),7.62(1H,t,J=4.6Hz),7.22(1H,d,J=8.6Hz),7.08-7.06(2H,m),6.88-6.86(1H,m),4.0 5(3H,s),3.73(3H,s),2.99(1H,t,J=6.9Hz),1.34(6H,d,J=6.6Hz).
MSm/z(M+H):452.

<Example 0453>

<0453-1>

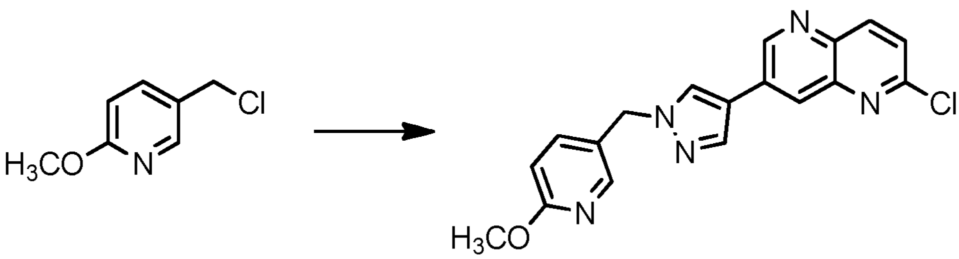

A mixture of 2-chloro-7-(1H-pyrazol-4-yl)-1,5-naphthyridine (100 mg), 5-(chloromethyl)-2-methoxypyridine (82 mg), cesium carbonate (227 mg), sodium iodide (21 mg), 1,4-dioxane (1.5 mL), and N,N-dimethylformamide (1.5 mL) was stirred at 100°C for 1.5 hours. After the reaction mixture was cooled to room temperature, water and ethyl acetate were added thereto, the organic layer was collected by separation, washed with a saturated sodium chloride aqueous solution, and dried over anhydrous sodium sulfate, and the solvent was distilled off under reduced pressure. Ethyl acetate was added to the obtained residue, and the solid matter was collected by filtration, thereby obtaining 2-chloro-7-(1-((6-methoxypyridin-3-yl)methyl)-1H-pyrazol-4-yl)-1,5-naphthyridine (70 mg) as a pale brown solid.
MSm/z(M+H):352.

<0453-2>

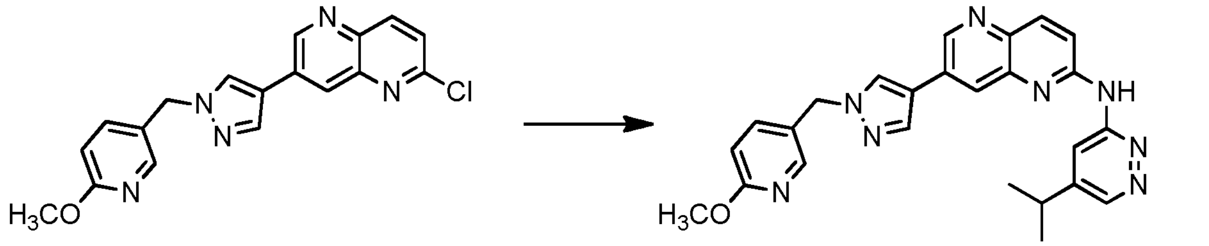

N-(5-isopropylpyridazin-3-yl)-7-(1-((6-methoxypyridin-3-yl)methyl)-1H-pyrazol-4-yl)-1,5-naphthyridine-2-amine was obtained as a pale yellow solid in the same manner as in Example 0411-3 except that 2-chloro-7-(1-((6-methoxypyridin-3-yl)methyl)-1H-pyrazol-4-yl)-1,5-naphthyridine was used instead of the 2-chloro-7-(1-((6-methylpyridin-2-yl)methyl)-1H-pyrazol-4-yl)-1,5-naphthyridine used in Example 0411-3.
$^1$H-NMR($CDCl_3$)δ:8.89(1H,d,J=2.0Hz),8.82-8.79(3H,m),8.24-8.20(2H,m),8.09-8.06(1H,m),7.99(1H,s),7.83(1H,s),7.60-7.51(2H,m),6.79-6.78(1H,m),5.34(2H,s),3.95(3H,s),1.41(6H,d,J=7.3Hz).
MSm/z(M+H):453.

<Example 0454>

<0454-1>

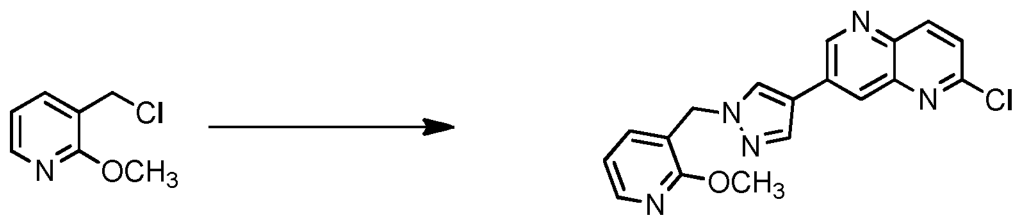

2-Chloro-7-(1-((2-methoxypyridin-3-yl)methyl)-1H-pyrazol-4-yl)-1,5-naphthyridine was obtained as a pale yellow solid in the same manner as in Example 0453-1 except that 3-(chloromethyl)-2-methoxypyridine was used instead of the 5-(chloromethyl)-2-methoxypyridine used in Example 0453-1.
MSm/z(M+H):352.

<0454-2>

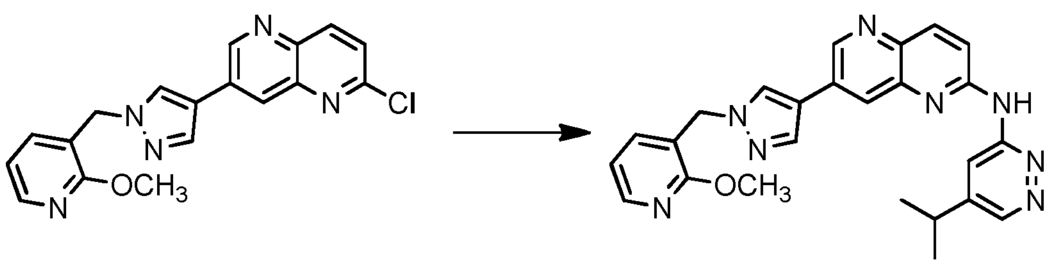

N-(5-isopropylpyridazin-3-yl)-7-(1-((2-methoxypyridin-3-yl)methyl)-1H-pyrazol-4-yl)-1,5-naphthyridine-2-amine was obtained as a pale yellow solid in the same manner as in Example 0411-3 except that 2-chloro-7-(1-((2-methoxypyridin-3-yl)methyl)-1H-pyrazol-4-yl)-1,5-naphthyridine was used instead of the 2-chloro-7-(1-((6-methylpyridin-2-yl)methyl)-1H-pyrazol-4-yl)-1,5-naphthyridine used in Example 0411-3.
$^1$H-NMR($CDCl_3$)δ:8.93(1H,d,J=2.0Hz),8.81(2H,s),8.73(1H,brs),8.26-8.23(1H,m),8.16-8.15(1H,m),8.12-8.09(1H,m),8.00(1H,s),7.94(1H,s),7.53-7.50(1H,m),7.41-7.39(1H,m),6.92-6.88(1H,m),5.38(2H,s),4.04(3H,s),3.04(1H,t,J=7.3Hz),1.41(6H,d,J=6.6Hz).
MSm/z(M+H):453.

<Example 0455>

<0455-1>

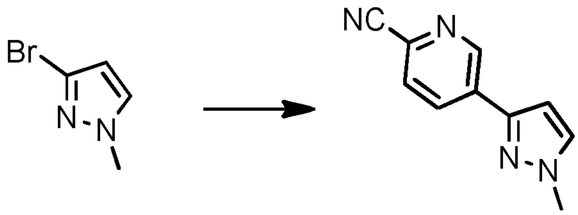

5-(1-Methyl-1H-pyrazol-3-yl)picolinonitrile was obtained as a pale yellow solid in the same manner as in Example 0451-1 except that 5-(4,4,5,5-tetramethyl-1,3,2-dioxaborolan-2-yl)picolinonitrile was used instead of the 3-methoxy-5-(4,4,5,5-tetramethyl-1,3,2-dioxaborolan-2-yl)pyridine used in Example 0451-1.
MSm/z(M+H):185.

<0455-2>

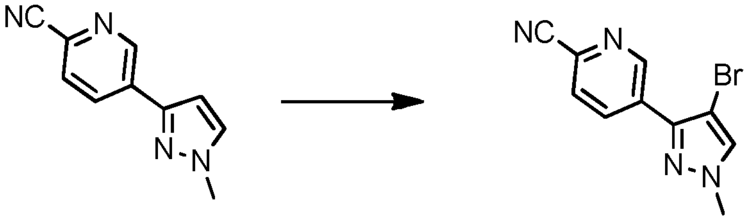

5-(4-Bromo-1-methyl-1H-pyrazol-3-yl)picolinonitrile was obtained as a pale yellow solid in the same manner as in Example 0451-2 except that 5-(1-methyl-1H-pyrazol-3-yl)picolinonitrile was used instead of the 3-methoxy-5-(1-methyl-1H-pyrazol-3-yl)pyridine used in Example 0451-2.
MSm/z(M+H):263.

<0455-3>

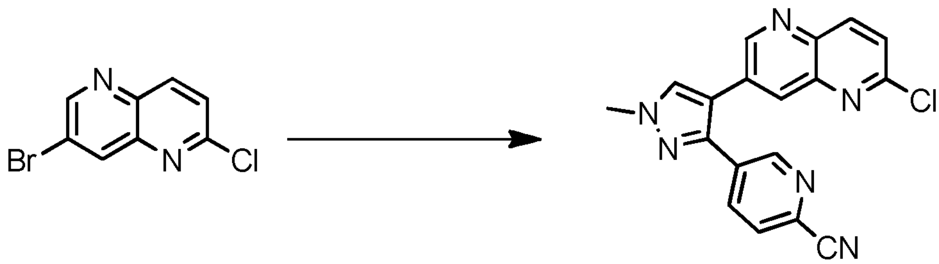

5-(4-(6-Chloro-1,5-naphthyridin-3-yl)-1-methyl-1H-pyrazol-3-yl)picolinonitrile was obtained as a pale yellow solid in the same manner as in Example 0451-3 except that 5-(4-bromo-1-methyl-1H-pyrazol-3-yl)picolinonitrile was used instead of the 3-(4-bromo-1-methyl-1H-pyrazol-3-yl)-5-methoxypyridine used in Example 0451-3.
MSm/z(M+H):347.

<0455-4>

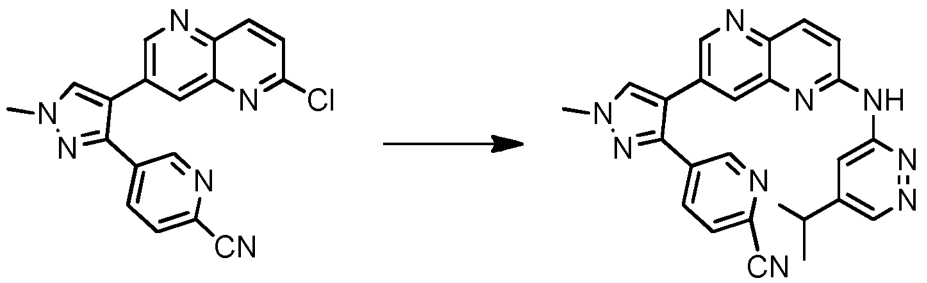

5-(4-(6-((5-Isopropylpyridazin-3-yl)amino)-1,5-naphthyridin-3-yl)-1-methyl-1H-pyrazol-3-yl)picolinonitrile was obtained as a pale brown solid in the same manner as in Example 0451-4 except that 5-(4-(6-chloro-1,5-naphthyridin-3-yl)-1-methyl-1H-pyrazol-3-yl)picolinonitrile was used instead of the 2-chloro-7-(3-(5-methoxypyridin-3-yl)-1-methyl-1H-pyrazol-4-yl)-1,5-naphthyridine used in Example 0451-4.
$^1$H-NMR($CDCl_3$)$\delta$:9.10(1H,brs),8.92-8.92(1H,m),8.80-8.77(2H,m),8.67(1H,d,J=2.0Hz),8.27(1H,d,J=9.2Hz),7.98-7.96(1H,m),7.92-7.92(1H,m),7.72(1H,s),7.68-7.61(2H,m),4.09(3H,s),2.99(1H,t,J=6.9Hz),1.34(6H,d,J=6.6Hz).
MSm/z(M+H):448.

<Example 0456>

<0456-1>

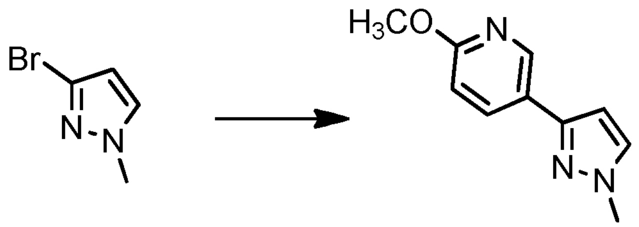

2-Methoxy-5-(1-methyl-1H-pyrazol-3-yl)pyridine was obtained as a pale yellow solid in the same manner as in Example 0451-1 except that 2-methoxy-5-(4,4,5,5-tetramethyl-1,3,2-dioxaborolan-2-yl)pyridine was used instead of the 3-methoxy-5-(4,4,5,5-tetramethyl-1,3,2-dioxaborolan-2-yl)pyridine used in Example 0451-1.
MSm/z(M+H):190.

<0456-2>

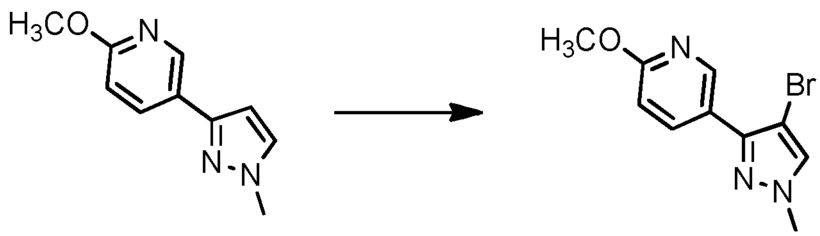

5-(4-Bromo-1-methyl-1H-pyrazol-3-yl)-2-methoxypyridine was obtained as brown oily substance in the same manner as in Example 0451-2 except that 2-methoxy-5-(1-methyl-1H-pyrazol-3-yl)pyridine was used instead of the 3-methoxy-5-(1-methyl-1H-pyrazol-3-yl)pyridine used in Example 0451-2.
MSm/z(M+H):268.

<0456-3>

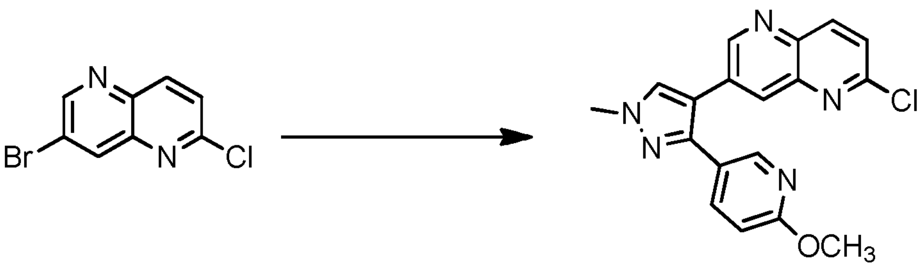

2-Chloro-7-(3-(6-methoxypyridin-3-yl)-1-methyl-1H-pyrazol-4-yl)-1,5-naphthyridine was obtained as a white solid in the same manner as in Example 0451-3 except that 5-(4-bromo-1-methyl-1H-pyrazol-3-yl)-2-methoxypyridine was used instead of the 3-(4-bromo-1-methyl-1H-pyrazol-3-yl)-5-methoxypyridine used in Example 0451-3.
MSm/z(M+H):352.

<0456-4>

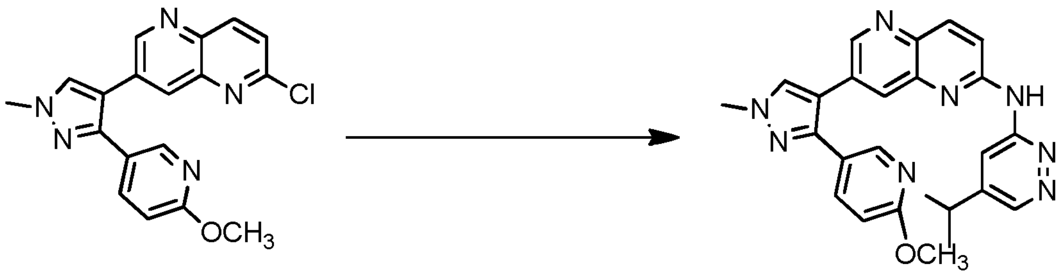

N-(5-isopropylpyridazin-3-yl)-7-(3-(6-methoxypyridin-3-yl)-1-methyl-1H-pyrazol-4-yl)-1,5-naphthyridine-2-amine was obtained as a pale yellow solid in the same manner as in Example 0451-4 except that 2-chloro-7-(3-(6-methoxypyridin-3-yl)-1-methyl-1H-pyrazol-4-yl)-1,5-naphthyridine was used instead of the 2-chloro-7-(3-(5-methoxypyridin-3-yl)-1-methyl-1H-pyrazol-4-yl)-1,5-naphthyridine used in Example 0451-4.
$^1$H-NMR($CDCl_3$)δ:8.99-8.96(1H,m),8.80(2H,d,J=5.3Hz),8.69-8.69(1H,m),8.28(1H,d,J=2.0Hz),8.23(1H,d,J=8.6Hz),7.95(1H,d,J=1.3Hz),7.77-7.73(1H,m),7.70(1H,s),7.56(1H,d,J=9.2Hz),6.75(1H,d,J=8.6Hz),4.05(3H,s),3.93(3H,s),2.98(1H,t,J=6. 9Hz),1.33(6H,d,J=7.3Hz).
MSm/z(M+H):453.

<Example 0457>

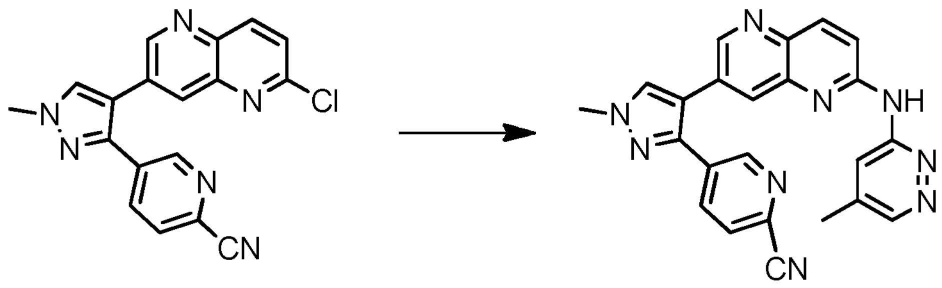

5-(1-Methyl-4-(6-((5-methylpyridazin-3-yl)amino)-1,5-naphthyridin-3-yl)-1H-pyrazol-3-yl)picolinonitrile was obtained as a pale yellow solid in the same manner as in Example 0455-4 except that 5-methylpyridazine-3-amine was used instead of the 5-isopropylpyridazine-3-amine used in Example 0455-4.
$^1$H-NMR($CDCl_3$)δ:8.90(1H,d,J=1.3Hz),8.74(1H,s),8.62(2H,d,J=19.2Hz),8.28-8.26(1H,m),8.01(1H,s),7.96(1H,dd,J=8.3,2.3 Hz),7.71(1H,s),7.63(1H,t,J=8.3Hz),4.09(3H,s),2.43(3H,s).
MSm/z(M+H):420.

<Example 0458>

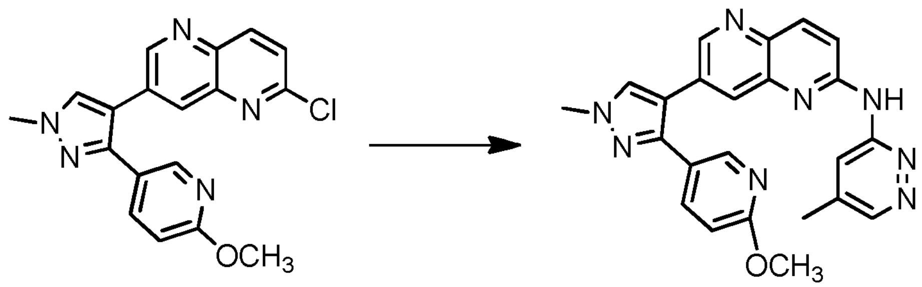

7-(3-(6-Methoxypyridin-3-yl)-1-methyl-1H-pyrazol-4-yl)-N-(5-methylpyridazin-3-yl)-1,5-naphthyridine-2-amine was obtained as a pale yellow solid in the same manner as in Example 0456-4 except that 5-methylpyridazine-3-amine was used instead of the 5-isopropylpyridazine-3-amine used in Example 0456-4.
$^1$H-NMR($CDCl_3$)δ:8.74(2H,s),8.66(1H,d,J=2.0Hz),8.27-8.23(2H,m),8.02(1H,d,J=2.0Hz),7.76-7.73(1H,m),7.69(1H,s),7.60(1H,d,J=9.2Hz),6.75(1H,d,J=8.6Hz),4.05(3H,s),3.93(3H,s),2.43(3H,s).
MSm/z(M+H):425.

<Example 0459>

<0459-1>

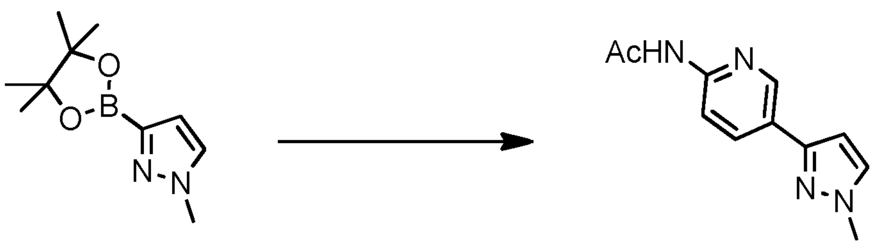

N-(5-(1-methyl-1H-pyrazol-3-yl)pyridin-2-yl)acetamide was obtained as a pale yellow solid in the same manner as in Example 0451-1 except that 1-methyl-3-(4,4,5,5-tetramethyl-1,3,2-dioxaborolan-2-yl)-1H-pyrazole was used instead of the 3-methoxy-5-(4,4,5,5-tetramethyl-1,3,2-dioxaborolan-2-yl)pyridine used in Example 0451-1, and N-(5-bromopyridin-2-yl)acetamide was used instead of the 3-bromo-1-methyl-1H-pyrazole used in Example 0451-1.
MSm/z(M+H):217.

<0459-2>

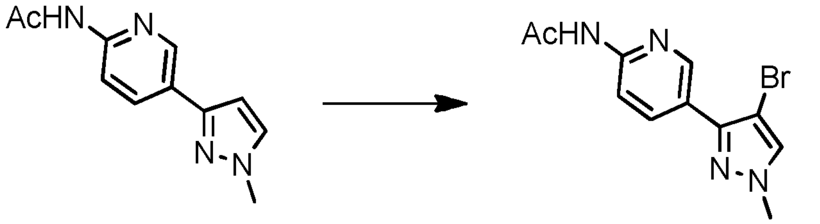

N-(5-(4-bromo-1-methyl-1H-pyrazol-3-yl)-pyridin-2-yl)acetamide was obtained as yellow oily substance in the same manner as in Example 0451-2 except that N-(5-(1-methyl-1H-pyrazol-3-yl)pyridin-2-yl)acetamide was used instead of the 3-methoxy-5-(1-methyl-1H-pyrazol-3-yl)pyridine used in Example 0451-2.
MSm/z(M+H):295.

<0459-3>

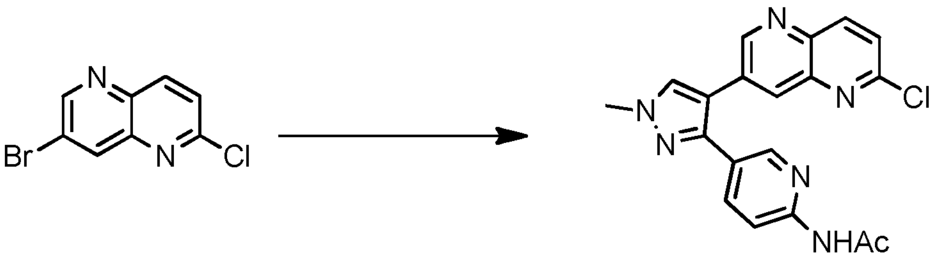

N-(5-(4-(6-chloro-1,5-naphthyridin-3-yl)-1-methyl-1H-pyrazol-3-yl)pyridin-2-yl)acetamide was obtained as a pale yellow solid in the same manner as in Example 0451-3 except that N-(5-(4-bromo-1-methyl-1H-pyrazol-3-yl)-pyridin-2-yl)acetamide was used instead of the 3-(4-bromo-1-methyl)-1H-pyrazol-3-yl)-5-methoxypyridine used in Example 0451-3.
MSm/z(M+H):379.

<0459-4>

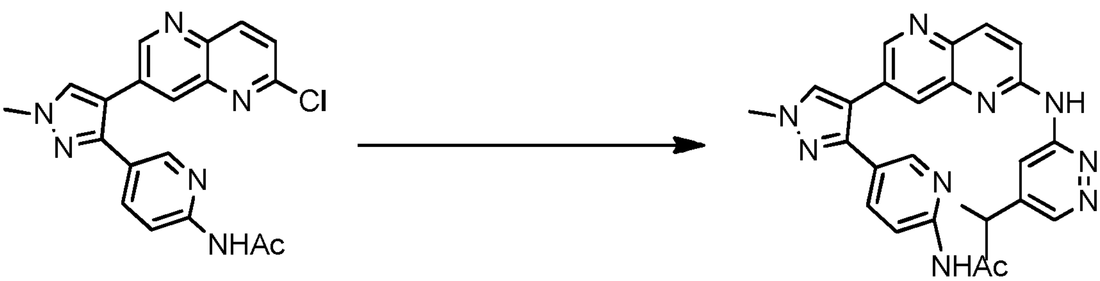

N-(5-(4-(6-((5-isopropylpyridazin-3-yl)amino)-1,5-naphthyridin-3-yl)-1-methyl-1H-pyrazol-3-yl)pyridin-2-yl)acetamide was obtained as a pale yellow solid in the same manner as in Example 0451-4 except that N-(5-(4-(6-chloro-1,5-naphthyridin-3-yl)-1-methyl-1H-pyrazol-3-yl)pyridin-2-yl)acetamide was used instead of the 2-chloro-7-(3-(5-methoxypyridin-3-yl)-1-methyl-1H-pyrazol-4-yl)-1,5-naphthyridine used in Example 0451-4.

$^1$H-NMR($CDCl_3$)δ:8.85(1H,brs),8.75-8.71(3H,m),8.52(1H,brs),8.42(1H,d,J=2.0Hz),8.25-8.22(1H,m),8.17-8.13(1H,m),7.95-7.92(2H,m),7.70(1H,s),4.07(3H,s),2.95-2.90(1H,m),2.24(3H,s),1.28(6H,d,J=6.6Hz).
MSm/z(M+H):480.

<Example 0460>

<0460-1>

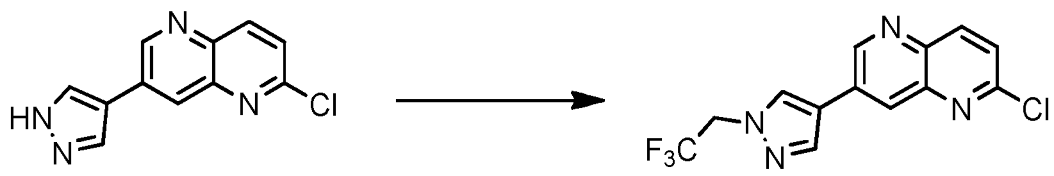

2-Chloro-7-(1-(2,2,2-trifluoroethyl)-1H-pyrazol-4-yl)-1,5-naphthyridine was obtained as a pale brown solid in the same manner as in Example 440-1 except that 2,2,2-trifluoroethyl trifluoromethanesulfonate was used instead of the bromoethane used in Example 440-1.
MSm/z(M+H):313.

<0460-2>

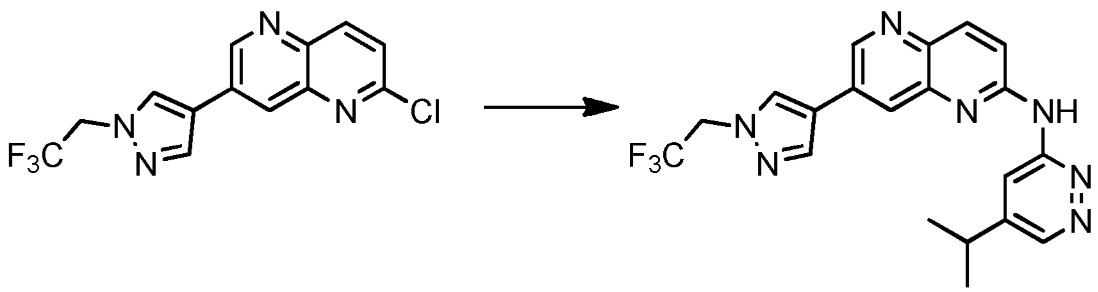

N-(5-isopropylpyridazin-3-yl)-7-(1-(2,2,2-trifluoroethyl)-1H-pyrazol-4-yl)-1,5-naphthyridine-2-amine was obtained as a pale yellow solid in the same manner as in Example 0440-2 except that 2-chloro-7-(1-(2,2,2-trifluoroethyl)-1H-pyrazol-4-yl)-1,5-naphthyridine was used instead of the 2-chloro-7-(1-ethyl-1H-pyrazol-4-yl)-1,5-naphthyridine used in Example 0440-2.
$^1$H-NMR(DMSO-$d_6$)δ:10.73(1H,s),9.07(1H,d,J=2.0Hz),8.87(1H,d,J=1.3Hz),8.75(1H,d,J=2.0Hz),8.63(1H,s),8.37(1H,s),8.28(1H,d,J=2.0Hz),8.24(1H,d,J=9.2Hz),7.72(1H,d,J=9.2Hz),5.24(2H,q,J=9.2Hz),3.04(1H,t,J=6.9Hz),1.34(6H,d,J=6.6Hz).
MSm/z(M+H):414.

<Example 0461>

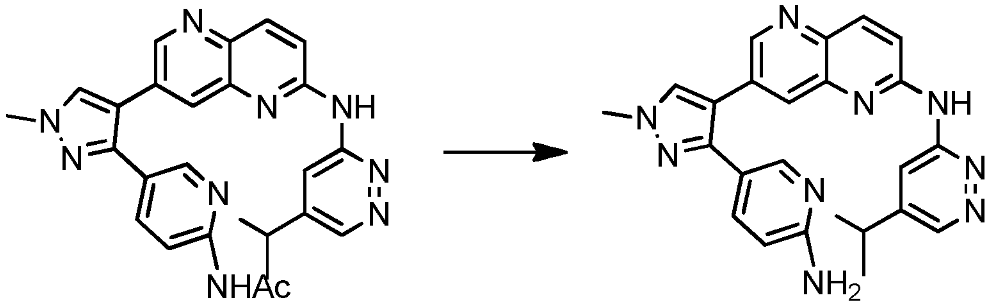

A3 mol/L sodium hydroxide aqueous solution (1 mL) was added to a solution of N-(5-(4-(6-((5-isopropylpyridazin-3-yl)amino)-1,5-naphthyridin-3 -yl)-1 -methyl-1H-pyrazol-3-yl)pyridin-2-yl)acetamide (1 mg) in 1,4-dioxane (1 mL), followed by stirring at 110°C for 3 hours. After the reaction mixture was cooled to room temperature, water and ethyl acetate were added thereto, the organic layer was collected by separation, washed with a saturated sodium chloride aqueous solution, and dried over anhydrous sodium sulfate, and the solvent was distilled off under reduced pressure, thereby obtaining 7-(3-(6-aminopyridin-3-yl)-1-methyl-1H-pyrazol-4-yl)-N-(5-isopropylpyridazin-3-yl)-1,5-naphthyridine-2-amine (1.1 mg) as a pale yellow solid.
$^1$H-NMR($CDCl_3$)$\delta$:8.85(1H,brs),8.75-8.71(3H,m),8.52(1H,brs),8.42(1H,d,J=2.0Hz),8.25-8.22(1H,m),8.17-8.13(1H,m),7.95-7.92(2H,m),7.70(1H,s),4.07(3H,s),2.95-2.90(1H,m),2.24(3H,s),1.28(6H,d,J=6.6Hz).
MSm/z(M+H):438.

<Example 0462>

<0462-1>

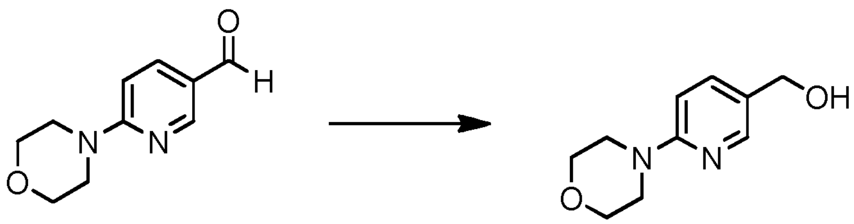

A mixture of 6-morpholinonicotinaldehyde (101.9 mg) and methanol (1.6 mL) was cooled to a temperature of from 0°C to 5°C, and, sodium borohydride (31 mg) was added thereto, followed by stirring at room temperature for 1 minutes in a nitrogen atmosphere. Acetone was added to the reaction mixture, and the solvent was distilled off under reduced pressure. After water and ethyl acetate were added to the obtained residue, the organic layer was collected by separation, washed with a saturated sodium chloride aqueous solution, and dried over anhydrous sodium sulfate, and the solvent was distilled off under reduced pressure, thereby obtaining (6-morpholinopyridin-3-yl)methanol (88 mg) as a white solid.
MSm/z(M+H):195.

<0462-2>

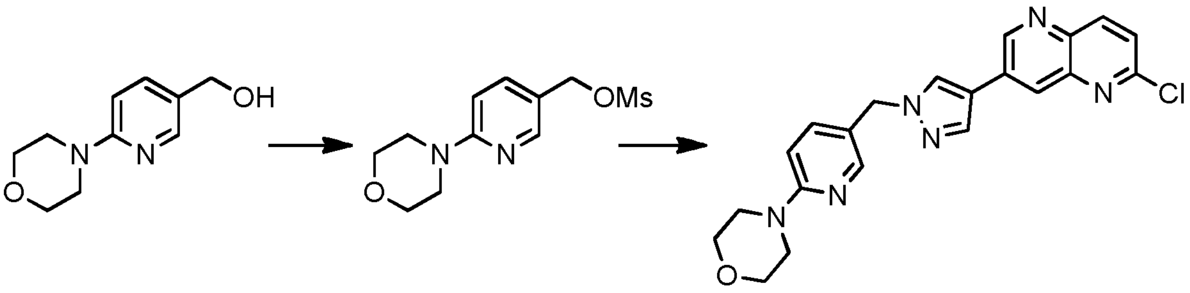

Methanesulfonyl chloride (42 μL) was added to a mixture of (6-morpholinopyridin-3-yl)methanol (88 mg), dichloromethane (2.3 mL), and triethylamine (95 μL) at a temperature of from 0°C to 5°C, followed by stirring for 1 hour. N,N-dimethylformamide (1.1 mL) was added to the reaction mixture, and the solvent was distilled off under reduced pressure.

1,4-Dioxane (1.1 mL), 2-chloro-7-(1H-pyrazol-4-yl)-1,5-naphthyridine (50 mg) and cesium carbonate (114 mg) were added to the obtained residue, followed by stirring at 100°C for 1 hour. After the reaction mixture was cooled to room temperature, water and ethyl acetate were added thereto, and the solid matter was collected by filtration, thereby obtaining 4-(5-((4-(6-chloro-1,5-naphthyridin-3-yl)-1H-pyrazol-1-yl)methyl)pyridin-2-yl)morpholine (53 mg) as a pale brown solid.
MSm/z(M+H):407.

<0462-3>

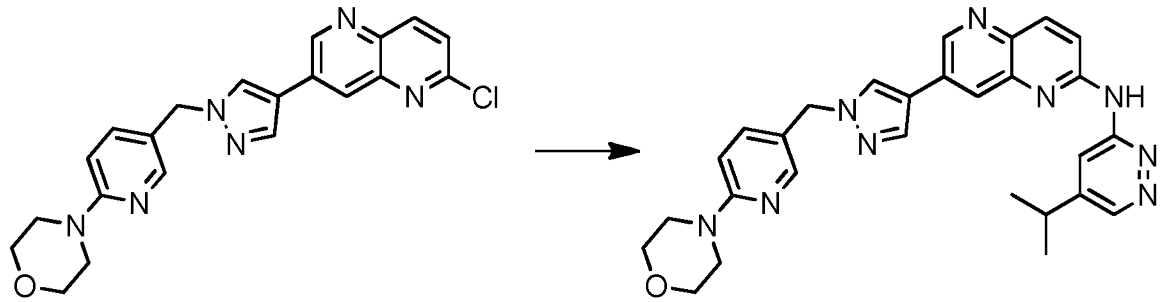

N-(5-isopropylpyridazin-3-yl)-7-(1-((6-morpholinopyridin-3-yl)methyl)-1H-pyrazol-4-yl)-1,5-naphthyridine-2-amine was obtained as a pale yellow solid in the same manner as in Example 0411-3 except that 4-(5-((4-(6-chloro-1,5-naphthyridin-3-yl)-1H-pyrazol-1-yl)methyl)pyridin-2-yl)morpholine was used instead of the 2-chloro-7-(1-((6-methylpyridin-2-yl)methyl)-1H-pyrazol-4-yl)-1,5-naphthyridine used in Example 0411-3.
$^1$H-NMR(DMSO-$d_6$)δ:10.69(1H,s),9.03(1H,d,J=2.0Hz),8.86(1H,d,J=2.0Hz),8.72(1H,d,J=2.0Hz),8.58(1H,s),8.21-8.20(4H,m),7.69(1H,d,J=9.2Hz),7.58(1H,dd,J=8.6,2.6Hz),6.84(1H,d,J=8.6Hz),5.28(2H,s),3.68(4H,t,J=4.6Hz),3.42(4H,t,J=5.0Hz),3.04(1H,t,J=10.0Hz),1.33(6H,d,J=7.3Hz).
MSm/z(M+H):508.

<Example 0463>

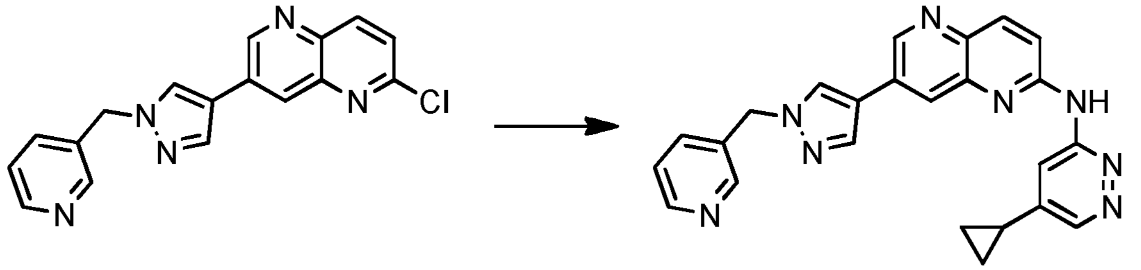

N-(5-cyclopropylpyridazin-3-yl)-7-(1-(pyridin-3-ylmethyl)-1H-pyrazol-4-yl)-1,5-naphthyridine-2-amine was obtained as a pale yellow solid in the same manner as in Example 0376-3 except that 5-cyclopropylpyridazine-3-amine was used instead of the 5-isopropylpyridazine-3-amine used in Example 0376-3.
$^1$H-NMR($CDCl_3$)δ:8.97-8.93(1H,m),8.64-8.54(3H,m),8.25-8.23(1H,m),8.11-8.09(1H,m),8.03(1H,s),7.91-7.88(1H,m),7.67-7.65(1H,m),7.61(1H,s),7.39-7.32(1H,m),5.44(2H,s),2.05-2.04(1H,m),1.28-0.86(4H,m).
MSm/z(M+H):421.

<Example 0464>

<0464-1>

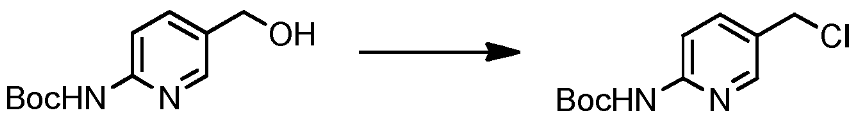

Methanesulfonyl chloride (0.14 mL) was added to a mixture of (tert-butyl (5-(hydroxymethyl)pyridin-2-yl)carbamate (133 mg), N,N-diisopropylethylamine (0.62 mL), and tetrahydrofuran (1.3 mL) at a temperature of from 0°C to 5°C, followed by stirring at room temperature for 6 hours. After water and ethyl acetate were added to the reaction mixture, the organic layer was collected by separation, washed with a saturated sodium chloride aqueous solution, and dried over anhydrous sodium sulfate, and the solvent was distilled off under reduced pressure, thereby obtaining tert-butyl (5-(chloromethyl)pyridin-2-yl)carbamate (158 mg) as a pale brown solid.
MSm/z(M+H):243.

<0464-2>

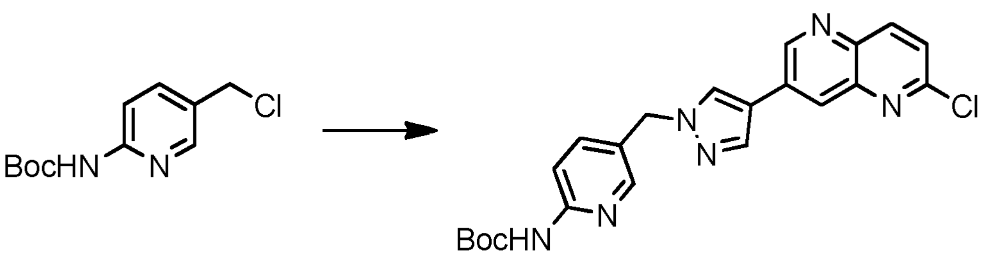

tert-Butyl (5-((4-(6-chloro-1,5-naphthyridin-3-yl)-1H-pyrazol-1-yl)methyl)pyridin-2-yl)carbamate was obtained in the same manner as in Example 0453-1 except that tert-butyl (5-(chloromethyl)pyridin-2-yl)carbamate was used instead of the 5-(chloromethyl)-2-methoxypyridine used in Example 0453-1.
MSm/z(M+H):437.

<0464-3>

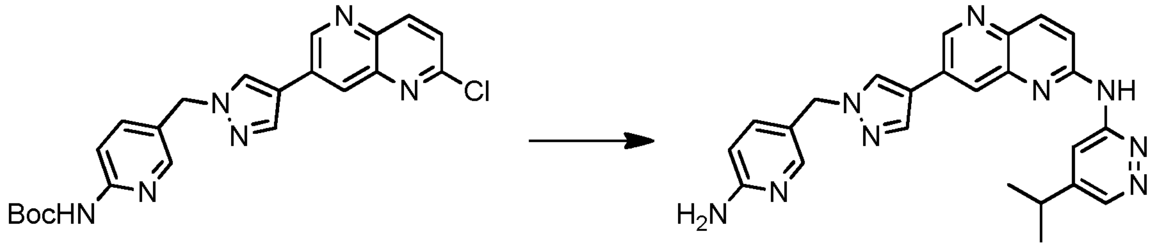

7-(1-((6-Aminopyridin-3-yl)methyl)-1H-pyrazol-4-yl)-N-(5-isopropylpyridazin-3-yl)-1,5-naphthyridine-2-amine was obtained as a pale yellow solid in the same manner as in Example 0411-3 except that tert-butyl (5-((4-(6-chloro-1,5-naphthyridin-3-yl)-1H-pyrazol-1-yl)methyl)pyridin-2-yl)carbamate was used instead of the 2-chloro-7-(1-((6-methylpyridin-2-yl)methyl)-1H-pyrazol-4-yl)-1,5-naphthyridine used in Example 0411-3.
$^1$H-NMR($CDCl_3$)δ:9.14(1H,brs),8.88-8.82(3H,m),8.23(1H,d,J=9.2Hz),8.13-8.06(2H,m),7.98(1H,s),7.81(1H,s),7.60-7.57(1H,m),7.46(1H,dd,J=8.3,2.3Hz),6.53(1H,d,J=8.6Hz),5.26(2H,s),3.08-2.89(1H,m),1.41(6H,d,J=6.6Hz).
MSm/z(M+H):438.

<Example 0465>

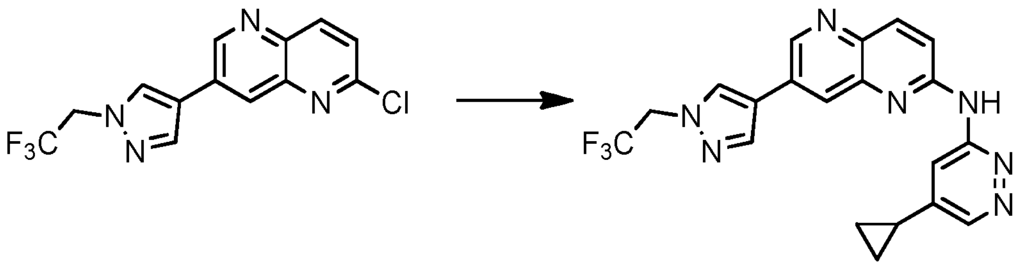

N-(5-cyclopropylpyridazin-3-yl)-7-(1-(2,2,2-trifluoroethyl)-1H-pyrazol-4-yl)-1,5-naphthyridine-2-amine was obtained as a pale yellow solid in the same manner as in Example 0460-2 except that 5-cyclopropylpyridazine-3-amine was used instead of the 5-isopropylpyridazine-3-amine used in Example 0460-2.
$^1$H-NMR(DMSO-$d_6$)δ:10.66(1H,s),9.07(1H,d,J=2.0Hz),8.65-8.61(3H,m),8.37(1H,s),8.32(1H,d,J=2.0Hz),8.22(1H,d,J=9.2Hz),7.68(1H,d,J=9.2Hz),5.24(2H,q,J=9.0 Hz),2.09(1H,s), 1.23-1.00(4H,m).
MSm/z(M+H):412.

<Example 0466>

<0466-1>

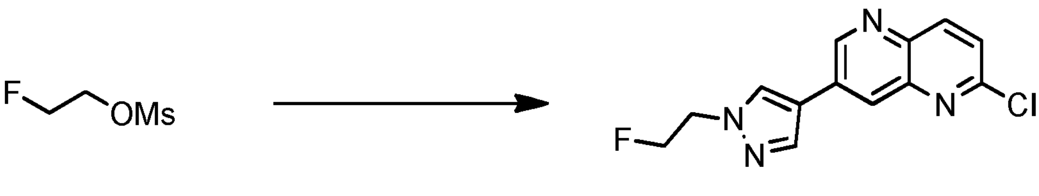

2-Chloro-7-(1-(2-fluoroethyl)-1H-pyrazol-4-yl)-1,5-naphthyridine was obtained as pale yellow oily substance in the same manner as in Example 0440-1 except that 2-fluoroethyl methanesulfonate was used instead of the bromoethane used in Example 0440-1.
MSm/z(M+H):277.

<0466-2>

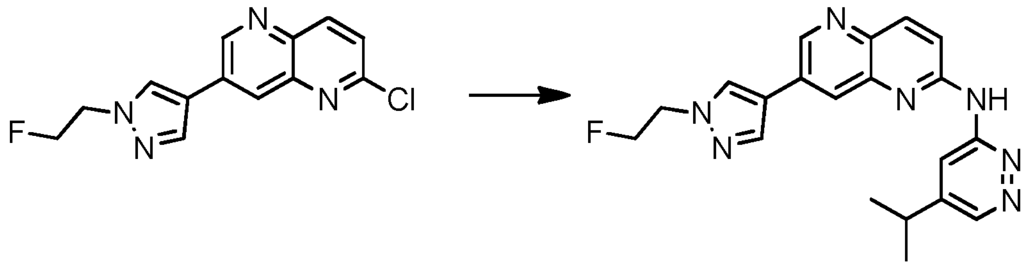

7-(1-(2-Fluoroethyl)-1H-pyrazol-4-yl)-N-(5-isopropylpyridazin-3-yl)-1,5-naphthyridine-2-amine was obtained as a pale yellow solid in the same manner as in Example 0440-2 except that 2-chloro-7-(1-(2-fluoroethyl)-1H-pyrazol-4-yl)-1,5-naphthyridine was used instead of the 2-chloro-7-(1-ethyl-1H-pyrazol-4-yl)-1,5-naphthyridine used in Example 0440-2.
$^1$H-NMR(DMSO-$d_6$)δ:10.71(1H,s),9.06(1H,d,J=2.6Hz),8.87(1H,d,J=2.0Hz),8.74(1H,d,J=2.0Hz),8.56(1H,s),8.25-8.22(3H,m),7.71(1H,d, J=9.2Hz),4.84(2H,dt,J=46.9,4.6Hz),4.52(2H,dt,J=27.7,4.6Hz),3.04(1H,t,J=6.9 Hz),1.34(6H,d,J=6.6Hz).
MSm/z(M+H):378.

<Example 0467>

<0467-1>

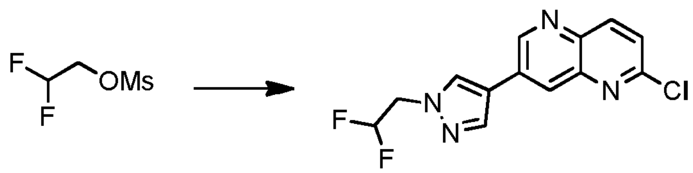

2-Chloro-7-(1-(2,2-difluoroethyl)-1H-pyrazol-4-yl)-1,5-naphthyridine was obtained as a pale yellow solid in the same manner as in Example 0440-1 except that 2,2-difluoroethyl methanesulfonate was used instead of the bromoethane used in Example 0440-1.
MSm/z(M+H):295.

<0467-2>

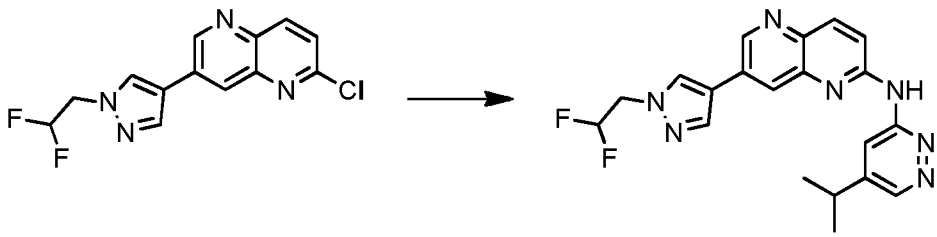

7-(1-(2,2-Difluoroethyl)-1H-pyrazol-4-yl)-N-(5-isopropylpyridazin-3-yl)-1,5-naphthyridine-2-amine was obtained as a pale yellow solid in the same manner as in Example 0440-2 except that 2-chloro-7-(1-(2,2-difluoroethyl)-1H-pyrazol-4-yl)-1,5-naphthyridine was used instead of the 2-chloro-7-(1-ethyl-1H-pyrazol-4-yl)-1,5-naphthyridine used in Example 0440-2.

$^1$H-NMR(DMSO-$d_6$)δ:10.72(1H,s),9.06(1H,d,J=2.0Hz),8.87(1H,d,J=2.0Hz),8.74(1H,d,J=1.3Hz),8.57(1H,s),8.28-8.24(3H,m),7.71(1H,d,J=8.6Hz),6.56-6.33(1H,m),4.72(2H,td,J=15.2,4.0Hz),3.04(1H,t,J=6.9Hz),1.33(6H,d,J=7.3Hz).
MSm/z(M+H):396.

<Example 0468>

<0468-1>

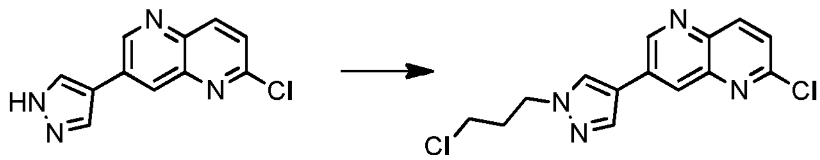

2-Chloro-7-(1-(3-chloropropyl)-1H-pyrazol-4-yl)-1,5-naphthyridine was obtained as a pale brown solid in the same manner as in Example 0440-1 except that 1-bromo-3-chloropropane was used instead of the bromoethane used in Example 0440-1.
MSm/z(M+H):307.

<0468-2>

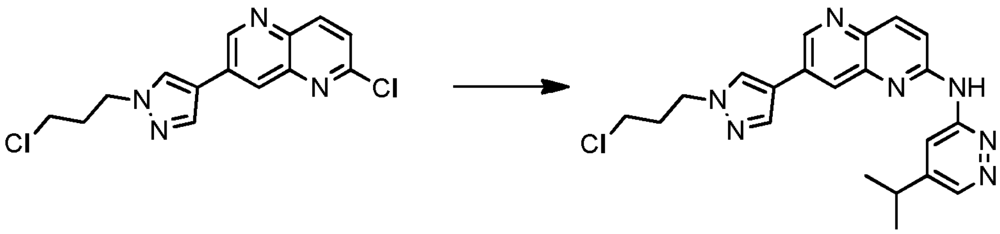

7-(1-(3-Chloropropyl)-1H-pyrazol-4-yl)-N-(5-isopropylpyridazin-3-yl)-1,5-naphthyridine-2-amine was obtained as a pale yellow solid in the same manner as in Example 0440-2 except that 2-chloro-7-(1-(3-chloropropyl)-1H-pyrazol-4-yl)-1,5-naphthyridine was used instead of the 2-chloro-7-(1-ethyl-1H-pyrazol-4-yl)-1,5-naphthyridine used in Example 0440-2.
$^1$H-NMR($CDCl_3$)δ:9.33(1H,brs),8.92-8.85(3H,m),8.25(1H,d,J=8.6Hz),8.11(1H,d,J=2.0Hz),7.95(2H,d,J=27.1Hz),7.65-7.62(1H,m),4.43(2H,t,J=6.6Hz),3.56(2H,t,J=5.9Hz),3.07(1H,t,J=6.6Hz),2.46-2.38(2H,m),1.43(6H,d,J=6.6Hz).
MSm/z(M+H):408.

<Example 0469>

<0469-1>

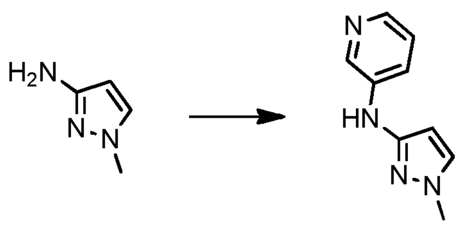

A mixture of 3-amino-1-methylpyrazole (46 μL), 3-bromopyridine (61 μL), tris(dibenzylideneacetone)dipalladium(0) (29.6 mg), 4,5-bis(diphenylphosphino)-9,9-dimethylxanthene (36.9 mg), cesium carbonate (408.5 mg), and 1,4-dioxane (3.2 mL) was stirred at 150°C for 30 minutes using a microwave reaction apparatus. The reaction mixture was cooled to room temperature, and purified by silica gel column chromatography (hexane-ethyl acetate, NH silica), thereby obtaining N-(1-methyl-1H-pyrazol-3-yl)pyridine-3-amine (106 mg) as a pale brown solid.
MSm/z(M+H):175.

<0469-2>

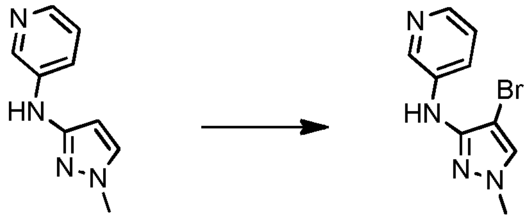

N-(4-bromo-1-methyl-1H-pyrazol-3-yl)pyridine-3-amine was obtained as colorless oily substance in the same manner as in Example 0451-2 except that N-(1-methyl-1H-pyrazol-3-yl)pyridine-3-amine was used instead of the 3-methoxy-5-(1-methyl-1H-pyrazol-3-yl)pyridine used in Example 0451-2.
MSm/z(M+H):253.

<0469-3>

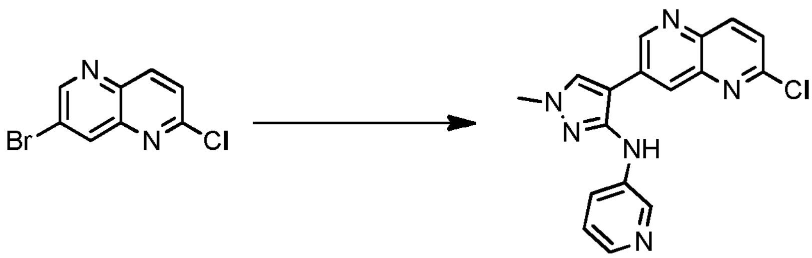

N-(4-(6-chloro-1,5-naphthyridin-3-yl)-1-methyl-1H-pyrazol-3-yl)pyridine-3-amine was obtained in the same manner as in Example 0451-3 except that N-(4-bromo-1-methyl-1H-pyrazol-3-yl)pyridine-3-amine was used instead of the 3-(4-bromo-1-methyl)-1H-pyrazol-3-yl)-5-methoxypyridine used in Example 0451-3.
MSm/z(M+H):337.

<0469-4>

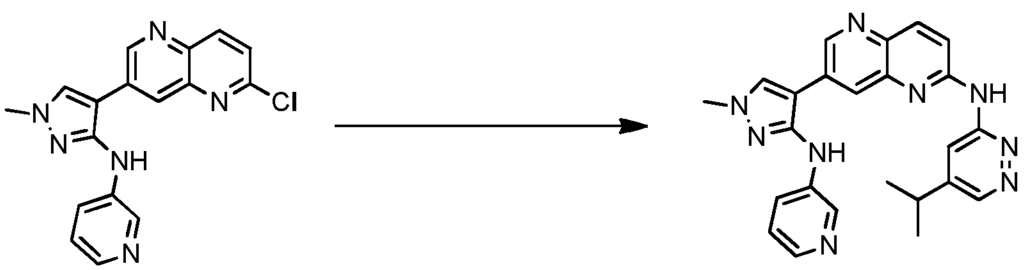

N-(5-isopropylpyridazin-3-yl)-7-(1-methyl-3-(pyridin-3-ylamino)-1H-pyrazol-4-yl)-1,5-naphthyridine-2-amine was obtained as a yellow solid in the same manner as in Example 0451-4 except that N-(4-(6-chloro-1,5-naphthyridin-3-yl)-1-methyl-1H-pyrazol-3-yl)pyridine-3-amine was used instead of the 2-chloro-7-(3-(5-methoxypyridin-3-yl)-1-methyl-1H-pyrazol-4-yl)-1,5-naphthyridine used in Example 0451-4.
$^1$H-NMR($CDCl_3$)δ:8.87(1H,d,J=2.0Hz),8.78-8.75(2H,m),8.43(1H,d,J=2.6Hz),8.23(1H,d,J=8.6Hz),8.12-8.11(2H,m), 7.67-7.61(2H,m),7.53(1H,d,J=9.2Hz),7.16-7.13(1H,m),6.00(1H,s),3.96(3H,s),2.99(1H,t,J=6.9Hz),1.36(6H,d,J=7.3Hz).
MSm/z(M+H):438.

<Example 0470>

<0470-1>

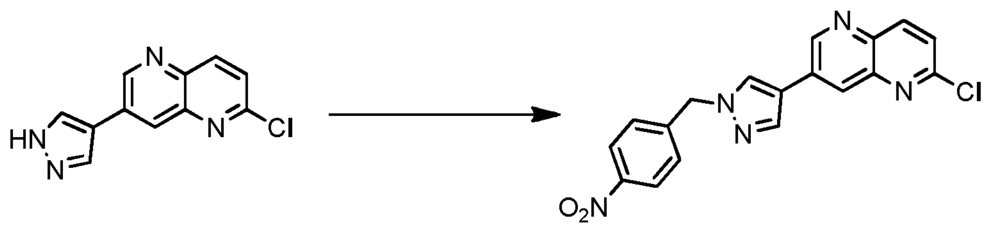

A mixture of 2-chloro-7-(1H-pyrazol-4-yl)-1,5-naphthyridine (101.6 mg), 1-(bromomethyl)-4-nitrobenzene (105.1 mg), potassium carbonate (119.2 mg), and N,N-dimethylformamide (2.2 mL) was stirred at 80°C for 1 hour. The reaction mixture was cooled to room temperature, water and ethyl acetate were added thereto, and the solid matter was collected by filtration, thereby obtaining 2-chloro-7-(1-(4-nitrobenzyl)-1H-pyrazol-4-yl)-1,5-naphthyridine (41 mg) as a pale yellow solid.
MSm/z(M+H):366.

<0470-2>

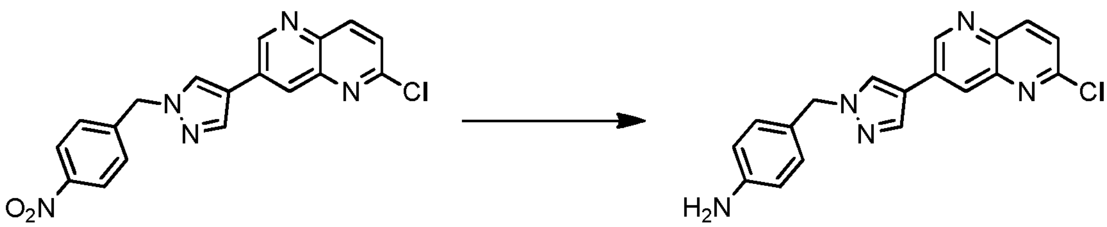

A mixture of reduced iron (21 mg), ammonium chloride (8.5 mg), 2-propanol (1.1 mL), and water (0.5 mL) was stirred at 50°C for 30 minutes. A mixture of 2-chloro-7-(1-(4-nitrobenzyl)-1H-pyrazol-4-yl)-1,5-naphthyridine and 2-propanol (3 mL) was added to the reaction mixture, followed by stirring at 50°C for 1.5 hours, and stirring at 70°C for 3.5 hours. The reaction mixture was cooled to room temperature, the insolubles were filtered off, and the solvent was distilled off under reduced pressure. The obtained residue was purified by silica gel column chromatography (hexane-ethyl acetate, NH silica), thereby obtaining 4-((4-(6-chloro-1,5-naphthyridin-3-yl)-1H-pyrazol-1-yl)methyl)aniline (16.2 mg) as a pale yellow solid.
MSm/z(M+H):336.

<0470-3>

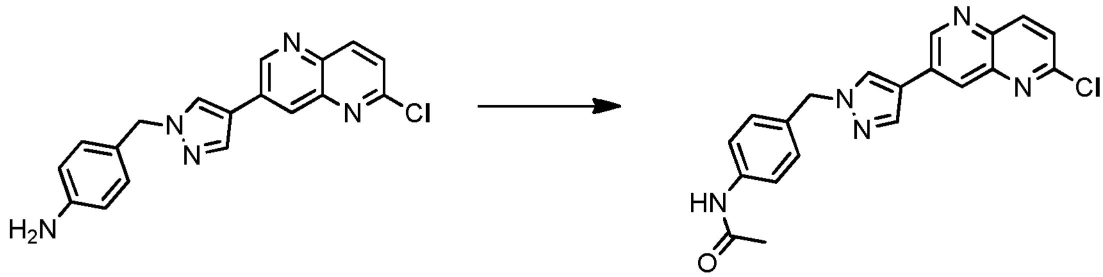

Acetic anhydride (5.5 μL) was added to a mixture of 4-((4-(6-chloro-1,5-naphthyridin-3-yl)-1H-pyrazol-1-yl)methyl)aniline (16.2 mg), and dichloromethane (1 mL), followed by stirring at room temperature for 1 hour in a nitrogen atmosphere. After water and dichloromethane were added to the reaction mixture, the organic layer was collected by separation, washed with a saturated sodium carbonate aqueous solution, and dried over anhydrous sodium sulfate, and the solvent was distilled off under reduced pressure, thereby obtaining N-(4-((4-(6-chloro-1,5-naphthyridin-3-yl)-1H-pyrazol-1-yl)methyl)phenyl)acetamide (19.2 mg) as a pale yellow solid.
MSm/z(M+H):378.

<0470-4>

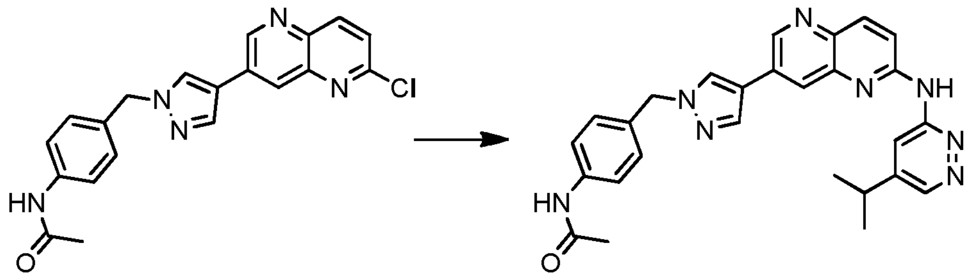

N-(4-((4-(6-((5-isopropylpyridazin-3-yl)amino)-1,5-naphthyridin-3-yl)-1H-pyrazol-1-yl)methyl)phenyl)acetamide was obtained as a yellow solid in the same manner as in Example 0411-3 except that N-(4-((4-(6-chloro-1,5-naphthyridin-3-yl)-1H-pyrazol-1-yl)methyl)phenyl)acetamide was used instead of the 2-chloro-7-(1-((6-methylpyridin-2-yl)methyl)-1H-pyrazol-4-yl)-1,5-naphthyridine used in Example 0411-3.
$^1$H-NMR($CDCl_3$)δ:8.90(1H,s),8.78(2H,d,J=11.2Hz),8.35(1H,s),8.23(1H,d,J=9.2Hz),8.08(1H,s),8.00(1H,s) ,7.82(1H,s),7.55-7.52(2H,m),7.43-7.41(1H,m),7.31-7.28(2H,m),7.17(1H,s),5.37(2H,s),3.04(1H,t,J=6.9Hz),2.19(3H,s),1.41(6H,d,J=7.3 Hz).
MSm/z(M+H):479.

<Example 0471>

<0471-1>

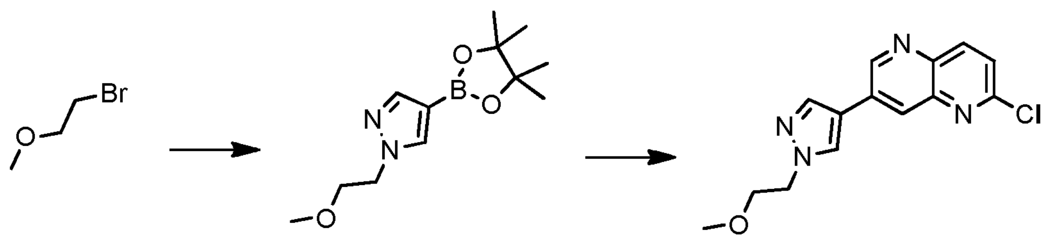

A suspension of 1-bromo-2-methoxyethane (0.064 mL), 4-(4,4,5,5-tetramethyl-1,3,2-dioxaborolan-2-yl)-1H-pyrazole (100 mg), and potassium carbonate (168 mg) in acetonitrile (2 mL) was stirred at 80°C for 7 hours. After the reaction mixture was cooled to room temperature, ethyl acetate and water were added thereto. The organic layer was collected by separation, and dried over anhydrous sodium sulfate, and the solvent was distilled off under reduced pressure, thereby obtaining 1-(2-methoxyethyl)-4-(4,4,5,5-tetramethyl-1,3,2-dioxaborolan-2-yl)-1H-pyrazole (60 mg).

A mixture of the obtained 1-(2-methoxyethyl)-4-(4,4,5,5-tetramethyl-1,3,2-dioxaborolan-2-yl)-1H-pyrazole (31 mg), 7-bromo-2-chloro-1,5-naphthyridine (20 mg), sodium carbonate (22 mg), bis(di-tert-butyl (4-dimethylaminophenyl)phosphine)dichloropalladium(II) (6 mg), water (0.2 mL), and 1,4-dioxane (2 mL) was stirred at 100°C for 2 hours. The reaction mixture was cooled to room temperature, the insolubles were filtered off, and the solvent was distilled off under reduced pressure. The obtained residue was purified by silica gel column chromatography (methanol-ethyl acetate), thereby obtaining 2-chloro-7-(1-(2-methoxyethyl)-1H-pyrazol-4-yl)-1,5-naphthyridine (8.1 mg).
MSm/z(M+H):289.

<0471-2>

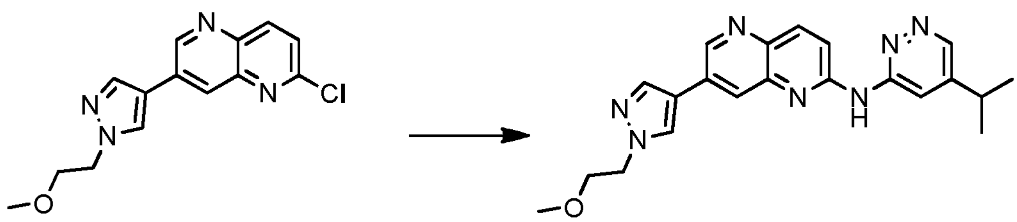

A suspension of 2-chloro-7-(1-(2-methoxyethyl)-1H-pyrazol-4-yl)-1,5-naphthyridine (8 mg), 5-isopropylpyridazine-3-amine (10 mg), tris(dibenzylideneacetone)dipalladium(0) (5 mg), 4,5-bis(diphenylphosphino)-9,9-dimethylxanthene (7 mg), and cesium carbonate (48 mg) in 1,4-dioxane (1 mL) was stirred at 140°C for 30 minutes using a microwave reaction apparatus. The reaction mixture was cooled to room temperature, the insolubles were filtered off, and the obtained solution was purified by silica gel column chromatography (methanol-ethyl acetate, NH silica), thereby obtaining N-(5-isopropylpyridazin-3-yl)-7-(1-(2-methoxyethyl)-1H-pyrazol-4-yl)-1,5-naphthyridine-2-amine (3.0 mg).
$^1$H-NMR($CDCl_3$/$CD_3OD$=4/1)δ:8.89(2H,brs),8.72(1H,brs),8.20(1H,d,J=8.7Hz),8.16(1H,d,J=2.1Hz),8.04( 1H,s),8.00(1H,s), 7.49(1H,d,J=8.7Hz),4.40(2H,t,J=4.5Hz),3.83(2H,t,J=4.5Hz),3.40(3H,s),3.15-3.00(1H,m),1.42(6H,d,J=6.6Hz).
MSm/z(M+H):390.

<Example 0472>

<0472-1>

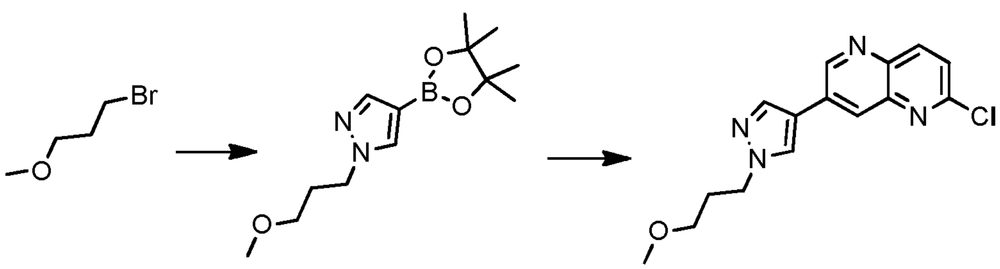

2-Chloro-7-(1-(3-methoxypropyl)-1H-pyrazol-4-yl)-1,5-naphthyridine was obtained in the same manner as in Example 0471-1 except that 1-bromo-3-methoxypropane was used instead of the 1-bromo-2-methoxyethane used in Example 0471-1.
MSm/z(M+H):303.

<0472-2>

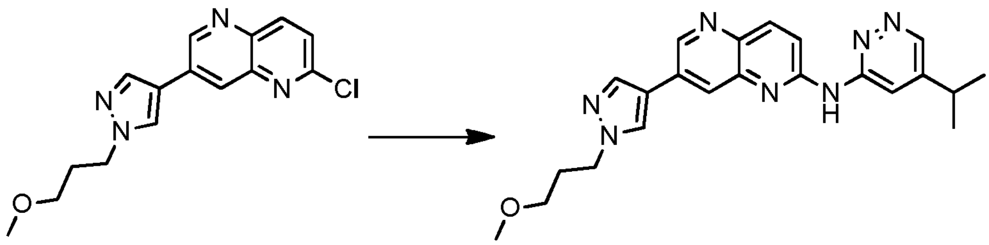

N-(5-isopropylpyridazin-3-yl)-7-(1-(3-methoxypropyl)-1H-pyrazol-4-yl)-1,5-naphthyridine-2-amine was obtained in the same manner as in Example 0471-2 except that 2-chloro-7-(1-(3-methoxypropyl)-1H-pyrazol-4-yl)-1,5-naphthyridine was used instead of the 2-chloro-7-(1-(2-methoxyethyl)-1H-pyrazol-4-yl)-1,5-naphthyridine used in Example 0471-2.

$^1$H-NMR($CDCl_3$/$CD_3OD$=4/1)δ:8.90(1H,brs),8.89(1H,d,J=2.1Hz),8.73(1H,m),8.21(1H,d,J=9.0Hz),8.15(1 H,d,J=2.1Hz),7.99(1H,s),7.98(1H,s),7.52(1H,d,J=9.0Hz),4.34(2H,t,J=7.5Hz),3.41(2H,t,J=6.0Hz),3.37( 3H,s),3.15-3.00 (1H,m),2.25-2.14(2H,m), 1.42(6H,d,J=6.6Hz).

MSm/z(M+H):404.

<Example 0473>

<0473-1>

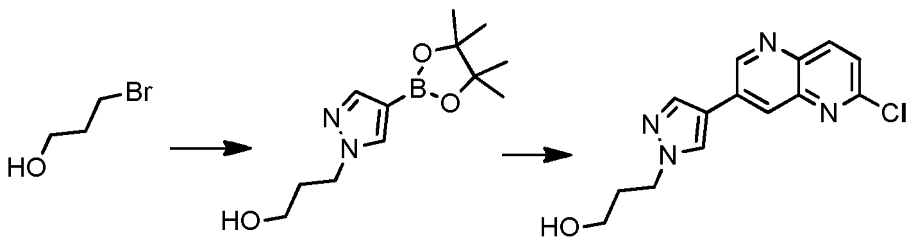

3-(4-(6-Chloro-1,5-naphthyridin-3-yl)-1H-pyrazol-1-yl)propan-1-ol was obtained in the same manner as in Example 0471-1 except that 3-bromopropan-1-ol was used instead of the 1-bromo-2-methoxyethane used in Example 0471-1.

MSm/z(M+H):289.

<0473-2>

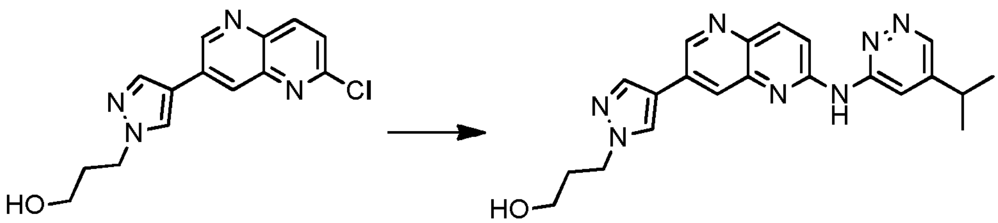

3-(4-(6-((5-Isopropylpyridazin-3-yl)amino)-1,5-naphthyridin-3-yl)-1H-pyrazol-1-yl)propan-1-ol was obtained in the same manner as in Example 0471-2 except that 3-(4-(6-chloro-1,5-naphthyridin-3-yl)-1H-pyrazol-1-yl)propan-1-ol was used instead of the 2-chloro-7-(1-(2-methoxyethyl)-1H-pyrazol-4-yl)-1,5-naphthyridine used in Example 0471-2.

$^1$H-NMR($CDCl_3$/$CD_3OD$=4/1)δ:8.88(2H,brs),8.72(1H,brs),8.20(1H,d,J=9.0Hz),8.15(1H,brs),8.04(1H,s),7.98(1H,s),7.50(1H,d,J=9.0Hz),4.37(2H,t,J=6.6Hz),3.63(2H,t,J=6.0Hz),3.14-3.00(1H,m),2.20-2.07(2H,m),1.42(6H,d,J=7.2Hz).
MSm/z(M+H):390.

<Example 0474>

<0474-1>

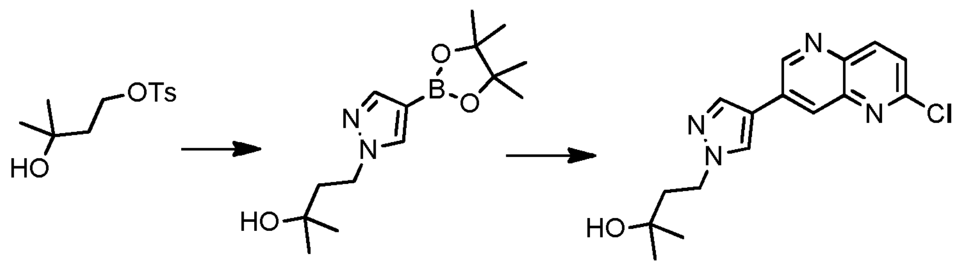

4-(4-(6-Chloro-1,5-naphthyridin-3-yl)-1H-pyrazol-1-yl)-2-methylbutan-2-ol was obtained in the same manner as in Example 0471-1 except that 3-hydroxy-3-methylbutyl 4-methylbenzenesulfonate was used instead of the 1-bromo-2-methoxyethane used in Example 0471-1.
MSm/z(M+H):317.

<0474-2>

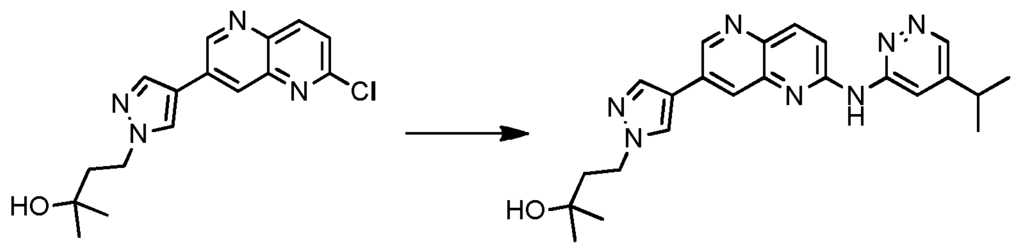

4-(4-(6-((5-Isopropylpyridazin-3-yl)amino)-1,5-naphthyridin-3-yl)-1H-pyrazol-1-yl)-2-methylbutan-2-ol was obtained in the same manner as in Example 0471-2 except that 4-(4-(6-chloro-1,5-naphthyridin-3-yl)-1H-pyrazol-1-yl)-2-methylbutan-2-ol was used instead of the 2-chloro-7-(1-(2-methoxyethyl)-1H-pyrazol-4-yl)-1,5-naphthyridine used in Example 0471-2.
$^1$H-NMR($CDCl_3$/$CD_3OD$=4/1)δ:8.88(2H,brs),8.72(1H,brs),8.20(1H,d,J=8.7Hz),8.14(1H,brs),8.02(1H,s),7.97(1H,s),7.50(1H,d,J=8.7Hz),4.42-4.33(2H,m),3.13-3.00(1H,m),2.15-2.08(2H,m),1.42(6H,d,J=6.6Hz),1.30(6H,s).
MSm/z(M+H):418.

<Example 0475>

<0475-1>

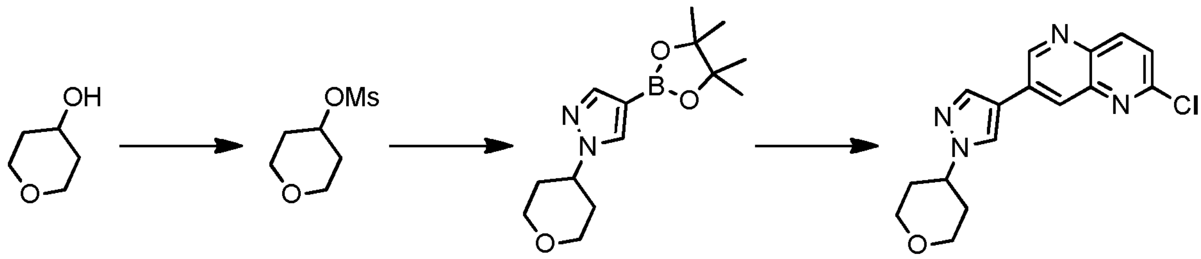

Methanesulfonyl chloride (0.138 mL) was added to a mixture of tetrahydro-2H-pyran-4-ol (166 mg), triethylamine (0.273 mL), and dichloromethane (8 mL) under ice-cooling, followed by stirring at the same temperature for 30 minutes. After water and dichloromethane were added to the reaction mixture, the organic layer was collected by separation, and dried over anhydrous sodium sulfate, and the solvent was distilled off under reduced pressure, thereby obtaining (tetrahydro-2H-pyran-4-yl) methanesulfonate (303 mg).

A suspension of the obtained (tetrahydro-2H-pyran-4-yl) methanesulfonate (303 mg), 4-(4,4,5,5-tetramethyl-1,3,2-dioxaborolan-2-yl)-1H-pyrazole (200 mg), and cesium carbonate (632 mg) in N-methylpyrrolidone (2 mL) was stirred at 100°C for 14 hours. After the reaction mixture was cooled to room temperature, ethyl acetate and water were added thereto. The organic layer was collected by separation, washed sequentially with water and a saturated sodium chloride aqueous solution, and dried over anhydrous sodium sulfate, and the solvent was distilled off under reduced pressure, thereby obtaining 1-(tetrahydro-2H-pyran-4-yl)-4-(4,4,5,5-tetramethyl-1,3,2-dioxaborolan-2-yl)-1H-pyrazole (118 mg).

A mixture of the obtained 1-(tetrahydro-2H-pyran-4-yl)-4-(4,4,5,5-tetramethyl-1,3,2-dioxaborolan-2-yl)-1H-pyrazole (34 mg), 7-bromo-2-chloro-1,5-naphthyridine (20 mg), sodium carbonate (22 mg), bis(di-tert-butyl (4-dimethylaminophenyl)phosphine)dichloropalladium(II) (6 mg), water (0.2 mL), and 1,4-dioxane (2 mL) was stirred at 100°C for 2 hours. The reaction mixture was cooled to room temperature, the insolubles were filtered off, and the solvent was distilled off under reduced pressure. The obtained residue was purified by silica gel column chromatography (methanol-ethyl acetate), thereby obtaining 2-chloro-7-(1-(tetrahydro-2H-pyran-4-yl)-1H-pyrazol-4-yl)-1,5-naphthyridine (15 mg).
MSm/z(M+H):315.

<0475-2>

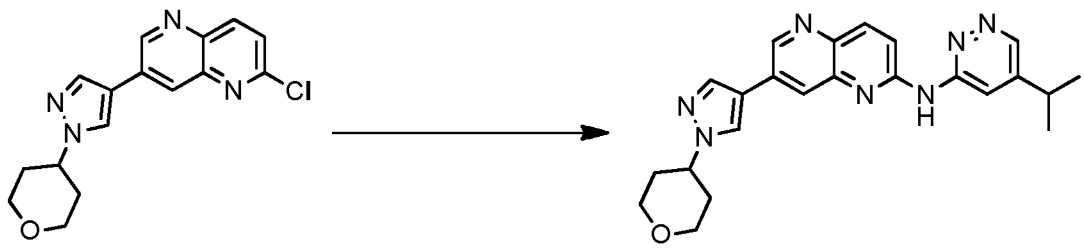

N-(5-isopropylpyridazin-3-yl)-7-(1-(tetrahydro-2H-pyran-4-yl)-1H-pyrazol-4-yl)-1,5-naphthyridine-2-amine was obtained in the same manner as in Example 0471-2 except that 2-chloro-7-(1-(tetrahydro-2H-pyran-4-yl)-1H-pyrazol-4-yl)-1,5-naphthyridine was used instead of the 2-chloro-7-(1-(2-methoxyethyl)-1H-pyrazol-4-yl)-1,5-naphthyridine used in Example 0471-2.
$^1$H-NMR($CDCl_3$/$CD_3OD$=4/1)$\delta$:8.88(2H,brs),8.72(1H,brs),8.20(1H,d,J=9.0Hz),8.15(1H,brs),8.04(1H,s),8.00(1H,s),7.51(1H,d,J=9.0Hz),4.54-4.40(1H,m),4.22-4.12(2H,m),3.70-3.56(2H,m),3.14-2.99(1H,m),2.25-2.13(4H,m),1.42(6H,d,J=7.2Hz).
MSm/z(M+H):416.

<Example 0476>

<0476-1>

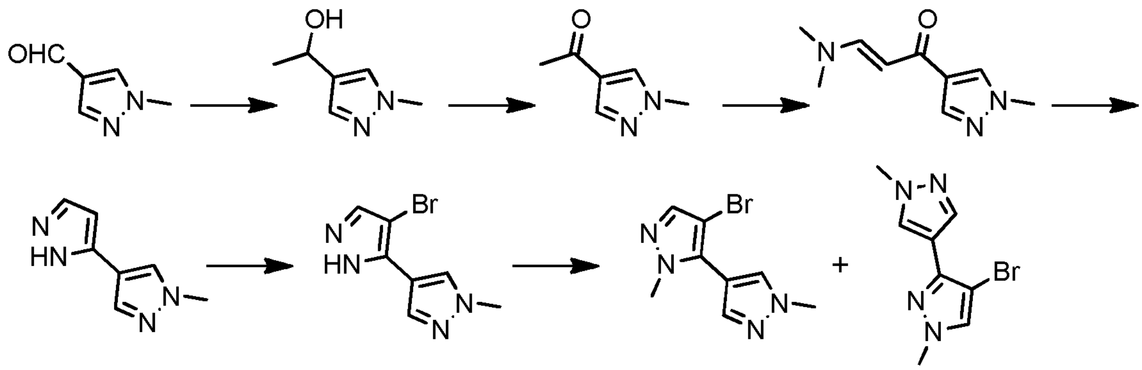

A 1 mol/L methyllithium/tetrahydrofuran solution (21 mL) was added to a mixture of 1-methyl-1H-pyrazole-4-carbaldehyde (465 mg) and tetrahydrofuran (20 mL) under ice-cooling, followed by stirring at the same temperature for 1 hour. After water and ethyl acetate were added to the reaction mixture, the organic layer was collected by separation, washed with a saturated sodium chloride aqueous solution, and dried over anhydrous sodium sulfate, and the solvent was distilled off under reduced pressure, thereby obtaining 1-(1-methyl-1H-pyrazol-4-yl)ethanol (377 mg).

A mixture of the obtained 1-(1-methyl-1H-pyrazol-4-yl)ethanol (377 mg), manganese dioxide (1.29 g), and dichloromethane (15 mL) was stirred for 12 hours under heating to reflux. The reaction mixture was cooled to room temperature, the insolubles were filtered off, and the solvent was distilled off under reduced pressure, thereby obtaining 1-(1-methyl-1H-pyrazol-4-yl)ethanone (365 mg).

A mixture of the obtained 1-(1-methyl-1H-pyrazol-4-yl)ethanone (365 mg) and N,N-dimethylformamide dimethyl acetal (2 mL) was stirred for 4 hours under heating to reflux. The reaction mixture was cooled to room temperature, and the solvent was distilled off under reduced pressure, thereby obtaining (E)-3-(dimethylamino)-1-(1-methyl-1H-pyrazol-4-yl)-2-propen-1-one (1.15 g).

Hydrazine monohydrate (0.172 mL) was added to a mixture of the obtained (E)-3-(dimethylamino)-1-(1-methyl-1H-pyrazol-4-yl)-2-propen-1-one (1.15 g) and ethanol (3 mL), followed by stirring at room temperature for 14 hours. The solvent of the reaction mixture was distilled off under reduced pressure, thereby obtaining 1'-methyl-1'H,2H-3,4'-bipyrazole (868 mg).

N-bromosuccinimide (575 mg) was added to a solution of the obtained 1'-methyl-1'H,2H-3,4'-bipyrazole (868 mg) in N,N-dimethylformamide (6 mL) under ice-cooling, followed by stirring at the same temperature for 1 hour, and iodomethane (0.732 mL) and 60% sodium hydride (505 mg) were added thereto under ice-cooling, followed by stirring at room temperature for 1.5 hours. Ethyl acetate and water were added to the reaction mixture. The organic layer was collected by separation, washed sequentially with water and a saturated sodium chloride aqueous solution, and dried over anhydrous sodium sulfate, and the solvent was distilled off under reduced pressure. The obtained residue was purified by silica gel column chromatography (ethyl acetate-hexane, NH silica), thereby obtaining 4-bromo-1',2-dimethyl-1'H,2H-3,4'-bipyrazole (142 mg) and 4-bromo-1,1'-dimethyl-1H,1'H-3,4'-bipyrazole (120 mg).
4-Bromo-1',2-dimethyl-1'H,2H-3,4'-bipyrazole
MSm/z(M+H):241.
4-Bromo-1,1'-dimethyl-1H,1'H-3,4'-bipyrazole
MSm/z(M+H):241.

<0476-2>

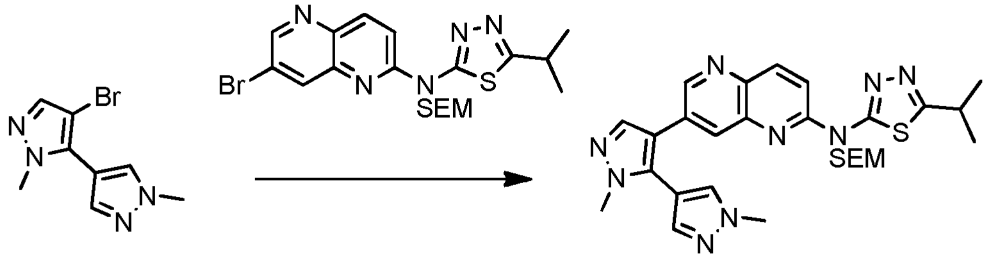

A suspension of N-(7-bromo-1,5-naphthyridin-2-yl)-5-isopropyl-N-((2-(trimethylsilyl)ethoxy)methyl)-1,3,4-thiadiazole-2-amine (20 mg), bis(pinacolato)diboron (15 mg), (1,1'-bis(diphenylphosphino)ferrocene)palladium(II) dichloride (3 mg), and potassium acetate (8 mg) in 1,4-dioxane (0.8 mL) was stirred at 100°C for 2 hours in a nitrogen atmosphere. 4-Bromo-1',2-dimethyl-1'H,2H-3,4'-bipyrazole (15 mg), water (0.1 mL), sodium carbonate (8 mg), and bis(di-tert-butyl (4-dimethylaminophenyl)phosphine)dichloropalladium(II) (5 mg) were added to the reaction mixture, followed by stirring at 100°C for 3 hours. The reaction mixture was cooled to room temperature, the insolubles were filtered off, and the obtained residue was purified by silica gel column chromatography (methanol-ethyl acetate), thereby obtaining N-(7-(1',2-dimethyl-1'H,2H-[3,4'-bipyrazole]-4-yl)-1,5-naphthyridin-2-yl)-5-isopropyl-N-((2-(trimethylsilyl)ethoxy)methyl)-1,3,4-thiadiazole-2-amine (13 mg).
MSm/z(M+H):562.

<0476-3>

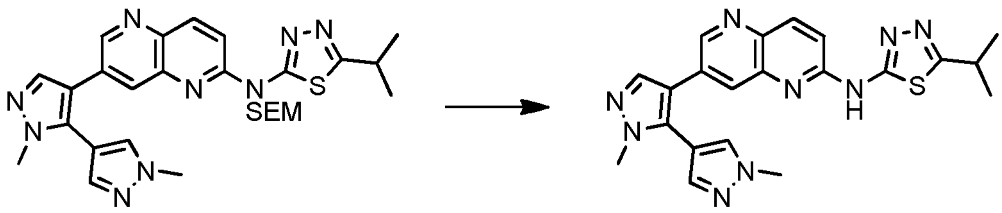

Water (0.1 mL) and trifluoroacetic acid (2 mL) were added to N-(7-(1',2-dimethyl-1'H,2H-[3,4'-bipyrazole]-4-yl)-1,5-naphthyridin-2-yl)-5-isopropyl-N-((2-(trimethylsilyl)ethoxy)methyl)-1,3,4-thiadiazole-2-amine (10 mg), followed by stirring at room temperature for 30 minutes. The solvent was distilled off under reduced pressure, and the obtained residue was purified by silica gel column chromatography (methanol-ethyl acetate, NH silica), thereby obtaining N-(7-(1',2-dimethyl-1'H,2H-[3,4'-bipyrazole]-4-yl)-1,5-naphthyridin-2-yl)-5-isopropyl-1,3,4-thiadiazole-2-amine (8.7 mg).
$^1$H-NMR($CDCl_3$/$CD_3OD$=4/1)$\delta$:8.58(1H,brs),8.19(1H,d,J=9.0Hz),8.17(1H,brs),7.89(1H,s),7.57(1H,s),7.53(1H,s),7.35(1H,d,J=9.0Hz),3.97(3H,s),3.89(3H,s),3.51-3.37(1H,m),1.50(6H,d,J=7.5Hz).
MSm/z(M+H):432.

<Example 0477>

<0477-1>

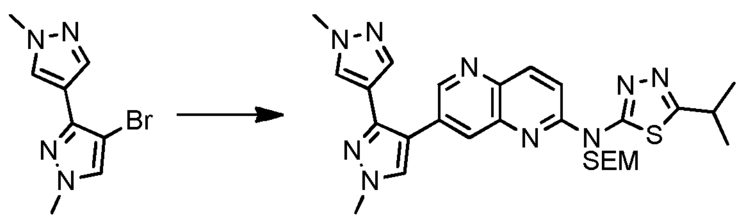

N-(7-(1,1'-dimethyl-1H,1'H-[3,4'-bipyrazole]-4-yl)-1,5-naphthyridin-2-yl)-5-isopropyl-N-((2-(trimethylsilyl)ethoxy)methyl)-1,3,4-thiadiazole-2-amine was obtained in the same manner as in Example 0476-2 except that 4-bromo-1,1'-dimethyl-1H,1'H-3,4'-bipyrazole was used instead of the 4-bromo-1',2-dimethyl-1'H,2H-3,4'-bipyrazole used in Example 0476-2.
MSm/z(M+H):562.

<0477-2>

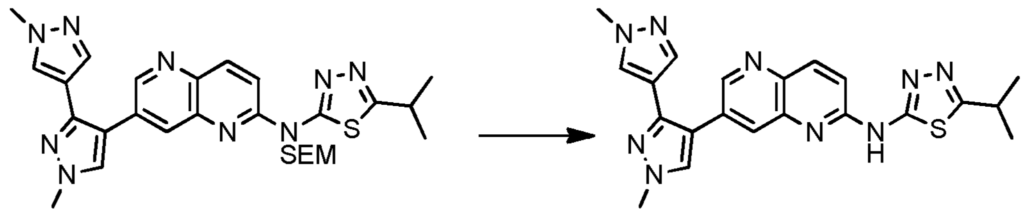

N-(7-(1,1'-dimethyl-1H,1'H-[3,4'-bipyrazole]-4-yl)-1,5-naphthyridin-2-yl)-5-isopropyl-1,3,4-thiadiazole-2-amine was obtained in the same manner as in Example 0476-3 except that N-(7-(1,1'-dimethyl-1H,1'H-[3,4'-bipyrazole]-4-yl)-1,5-naphthyridin-2-yl)-5-isopropyl-N-((2-(trimethylsilyl)ethoxy)methyl)-1,3,4-thiadiazole-2-amine was used instead of the N-(7-(1',2-dimethyl-1'H,2H-[3,4'-bipyrazole]-4-yl)-1,5-naphthyridin-2-yl)-5-isopropyl-N-((2-(trimethylsilyl)ethoxy)methyl)-1,3,4-thiadiazole-2-amine used in Example 0476-3.
$^1$H-NMR($CDCl_3$/$CD_3OD$=4/1)δ:8.70(1H,d,J=2.1Hz),8.25(1H,d,J=9.0Hz),8.24(1H,d,J=2.1Hz),7.72(1H,s),7.56(1H,s),7.53(1H,s),7.39(1H,d,J=9.0Hz),4.01(3H,s),3.88(3H,s),3.48-3.37(1H,m),1.48(6H,d,J=7.2Hz).
MSm/z(M+H):432.

<Example 0478>

<0478-1>

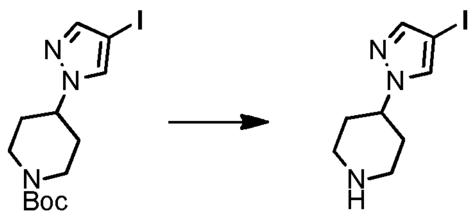

Water (1 mL) and trifluoroacetic acid (10 mL) were added to tert-butyl 4-(4-iodo-1H-pyrazol-1-yl)piperidine-1-carboxylate (745 mg), followed by stirring at room temperature for 1.5 hours. The solvent was distilled off under reduced pressure, and the obtained residue was purified by silica gel column chromatography (methanol-ethyl acetate, NH silica), thereby obtaining 4-(4-iodo-1H-pyrazol-1-yl)piperidine (411 mg).
MSm/z(M+H):278.

<0478-2>

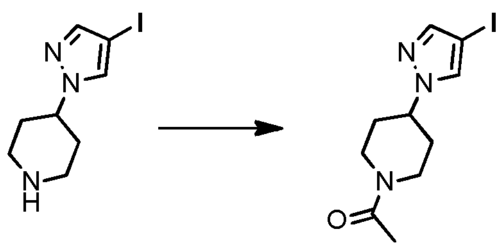

Acetyl chloride (0.051 mL) was added to a solution of 4-(4-iodo-1H-pyrazol-1-yl)piperidine (132 mg) and triethylamine (0.133 mL) in tetrahydrofuran (5 mL) under ice-cooling, followed by stirring at the same temperature for 2 hours. After water and ethyl acetate were added to the reaction mixture, the organic layer was collected by separation, washed with a saturated sodium chloride aqueous solution, and dried over anhydrous sodium sulfate, and the solvent was distilled off under reduced pressure, thereby obtaining 1-(4-(4-iodo-1H-pyrazol-1-yl)piperidin-1-yl)ethanone (150 mg).
MSm/z(M+H):320.

<0478-3>

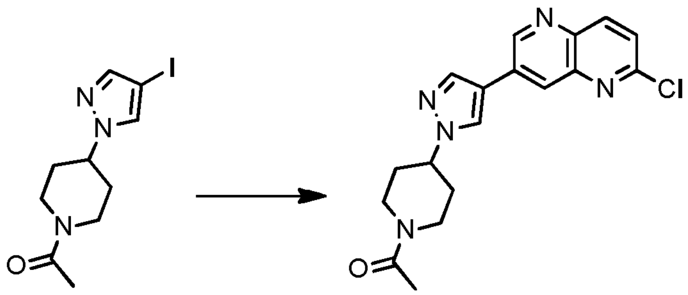

A suspension of 7-bromo-2-chloro-1,5-naphthyridine (48 mg), bis(pinacolato)diboron (60 mg), (1,1'-bis(diphenylphosphino)ferrocene)palladium(II) dichloride (16 mg), and potassium acetate (39 mg) in 1,4-dioxane (2 mL) was stirred at 80°C for 2 hours in a nitrogen atmosphere. 1-(4-(4-Iodo-1H-pyrazol-1-yl)piperidin-1-yl)ethanone (75 mg), sodium carbonate (42 mg) and bis(di-tert-butyl (4-dimethylaminophenyl)phosphine)dichloropalladium(II) (14 mg) were added to the reaction mixture, followed by stirring at 80°C for 3 hours. The reaction mixture was cooled to room temperature, the insolubles were filtered off, and the solvent was distilled off under reduced pressure. The obtained residue was purified by silica gel column chromatography (methanol-ethyl acetate), thereby obtaining 1-(4-(4-(6-chloro-1,5-naphthyridin-3-yl)-1H-pyrazol-1-yl)piperidin-1-yl)ethanone (35 mg).
MSm/z(M+H):356.

<0478-4>

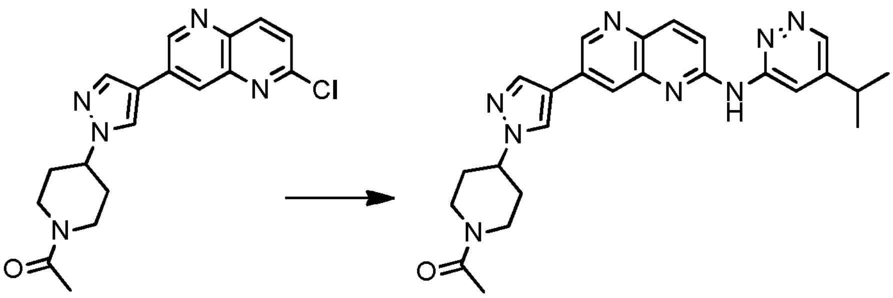

1-(4-(4-(6-((5-Isopropylpyridazin-3-yl)amino)-1,5-naphthyridin-3-yl)-1H-pyrazol-1-yl)piperidin-1-yl)ethanone was obtained in the same manner as in Example 0471-2 except that 1-(4-(4-(6-((5-isopropylpyridazin-3-yl)amino)-1,5-naphthyridin-3-yl)-1H-pyrazol-1-yl)piperidin-1-yl)ethanone was used instead of the 2-chloro-7-(1-(2-methoxyethyl)-1H-pyrazol-4-yl)-1,5-naphthyridine used in Example 0471-2.
$^1$H-NMR($CDCl_3$/$CD_3OD$=4/1):8.88,(2H,brs),8.73(1H,brs),8.10(1H,d,J=9.3Hz),8.15(1H,brs),8.04(1H,s),8.00(1H,s),7.52(1H,d,J=9.3Hz),4.79-4.71(1H,m),4.54-4.43(1H,m),4.15-4.02(1H,m),3.38-3.28(3H,m),3.12-3.02(1H,m),2.90-2.79(1H,m),2.38-2.22(1H,m),2.19(3H,s),2.15-1.98(1H,m),1.42(6H,d,J=7.4Hz).
MSm/z(M+H):457.

<Example 0479>

<0479-1>

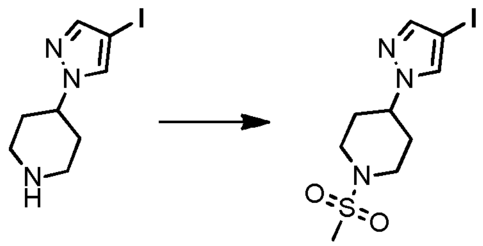

Methanesulfonyl chloride (0.057 mL) was added to a solution of 4-(4-iodo-1H-pyrazol-1-yl)piperidine (132 mg) and triethylamine (0.133 mL) in tetrahydrofuran (5 mL) under ice-cooling, followed by stirring at the same temperature for 2 hours. After water and ethyl acetate were added to the reaction mixture, the organic layer was collected by separation, washed with a saturated sodium chloride aqueous solution, and dried over anhydrous sodium sulfate, and the solvent was distilled off under reduced pressure, thereby obtaining 4-(4-iodo-1H-pyrazol-1-yl)-1-(methylsulfonyl)piperidine (151 mg).
MSm/z(M+H):356.

<0479-2>

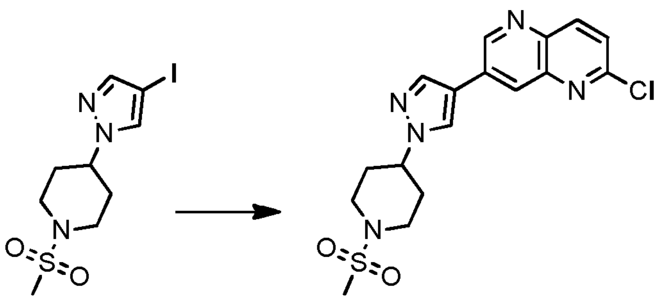

2-Chloro-7-(1-(1-(methylsulfonyl)piperidm-4-yl)-1H-pyrazol-4-yl)-1,5-naphthyridine was obtained in the same manner as in Example 0478-3 except that 4-(4-iodo-1H-pyrazol-1-yl)-1-(methylsulfonyl)piperidine was used instead of the 1-(4-(4-iodo-1H-pyrazol-1-yl)piperidin-1-yl)ethanone used in Example 0478-3.
MSm/z(M+H):392.

<0479-3>

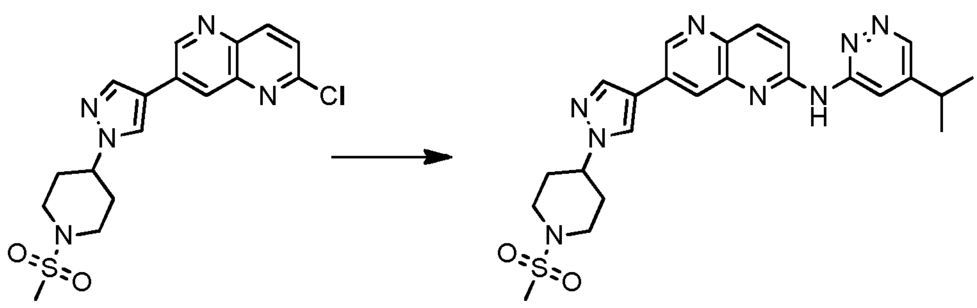

N-(5-isopropylpyridazin-3-yl)-7-(1-(1-(methylsulfonyl)piperidin-4-yl)-1H-pyrazol-4-yl)-1,5-naphthyridine-2-amine was obtained in the same manner as in Example 0471-2 except that 2-chloro-7-(1-(1-(methylsulfonyl)piperidin-4-yl)-1H-pyrazol-4-yl)-1,5-naphthyridine was used instead of the 2-chloro-7-(1-(2-methoxyethyl)-1H-pyrazol-4-yl)-1,5-naphthyridine used in Example 0471-2.
$^1$H-NMR($CDCl_3$/$CD_3OD$=4/1):8.88(2H,brs),8.73(1H,brs),8.21(1H,d,J=9.3Hz),8.16(1H,brs),8.06(1H,s),8.00(1H,s),7.51(1H,d,J=9.3Hz),4.48-4.33(1H,m),4.03-3.93(2H,m),3.14-2.94(3H,m),2.91(3H,s),2.42-2.15(4H,m),

1.42(6H,d,J=7.2Hz).
MSm/z(M+H):493.

<Example 0480>

<0480-1>

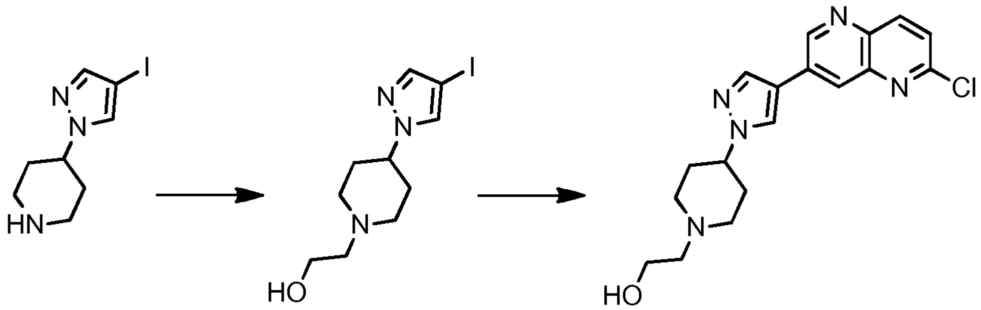

A mixture of 4-(4-iodo-1H-pyrazol-1-yl)piperidine (147 mg), 2-bromoethanol (0.042 mL), potassium carbonate (146 mg), tetrahydrofuran (5 mL), and 1,4-dioxane (0.5 mL) was stirred at 80°C for 2 hours. The reaction mixture was cooled to room temperature, the insolubles were filtered off, and the solvent was distilled off under reduced pressure, thereby obtaining 2-(4-(4-iodo-1H-pyrazol-1-yl)piperidin-1-yl)ethanol (250 mg).
MSm/z(M+H):322.
A suspension of 7-bromo-2-chloro-1,5-naphthyridine (48 mg), bis(pinacolato)diboron (60 mg), (1,1'-bis(diphenylphosphino)ferrocene)palladium(II) dichloride (16 mg), and potassium acetate (39 mg) in 1,4-dioxane (2 mL) was stirred at 80°C for 2 hours in a nitrogen atmosphere. 2-(4-(4-Iodo-1H-pyrazol-1-yl)piperidin-1-yl)ethanol (75 mg), sodium carbonate (42 mg) and bis(di-tert-butyl (4-dimethylaminophenyl)phosphine)dichloropalladium(II) (14 mg) were added to the reaction mixture, followed by stirring at 80°C for 3 hours. The reaction mixture was cooled to room temperature, the insolubles were filtered off, and the solvent was distilled off under reduced pressure. The obtained residue was purified by silica gel column chromatography (methanol-ethyl acetate), thereby obtaining 2-(4-(4-(6-chloro-1,5-naphthyridin-3-yl)-1H-pyrazol-1-yl)piperidin-1-yl)ethanol (30 mg).
MSm/z(M+H):358.

<0480-2>

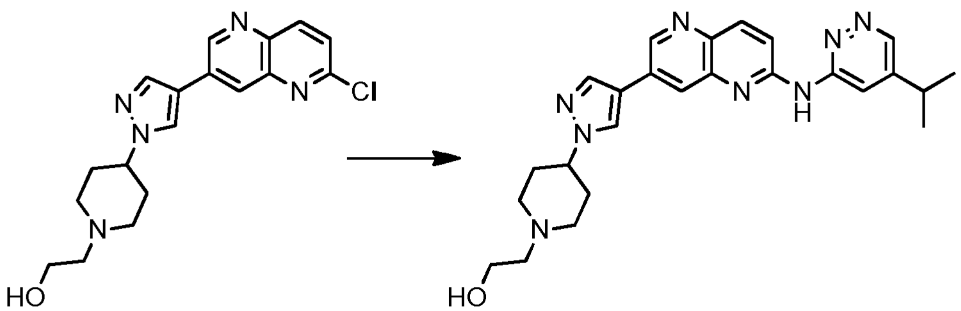

2-(4-(4-(6-((5-Isopropylpyridazin-3-yl)amino)-1,5-naphthyridin-3-yl)-1H-pyrazol-1-yl)piperidin-1-yl)ethanol was obtained in the same manner as in Example 0471-2 except that 2-(4-(4-(6-chloro-1,5-naphthyridin-3-yl)-1H-pyrazol-1-yl)piperidin-1-yl)ethanol was used instead of the 2-chloro-7-(1-(2-methoxyethyl)-1H-pyrazol-4-yl)-1,5-naphthyridine used in Example 0471-2.
$^1$H-NMR($CDCl_3$/$CD_3OD$=4/1):8.89(2H,brs),8.72(1H,brs),8.20(1H,d,J=9.3Hz),8.16(1H,brs),8.06(1H,s),7.99(1H,s),7.50(1H,d,J=9.3Hz),4.33-4.20(1H,m),3.71(2H,t,J=6.0Hz),3.20-3.00(3H,m),2.64(2H,t,J=6.0Hz),2.40-2.07(6H,m),1.42(6H,d,J=7.2Hz).
MSm/z(M+H):459.

<Example 0481>

<0481-1>

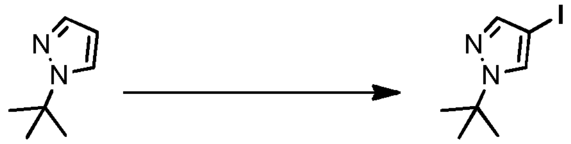

Iodine (1.59g) and ammonium cerium nitrate (3.45g) were added to a solution of 1-(tert-butyl)-1H-pyrazole (1.30 g) in acetonitrile (6 mL), followed by stirring at room temperature for 2 hours. After ethyl acetate and water were added to the reaction mixture, the organic layer was collected by separation, washed sequentially with a 10% sodium hydrogen sulfite aqueous solution and a saturated sodium chloride aqueous solution, and dried over anhydrous sodium sulfate, and the solvent was distilled off under reduced pressure, thereby obtaining 1-(tert-butyl)-4-iodo-1H-pyrazole (487 mg).
MSm/z(M+H):251.

<0481-2>

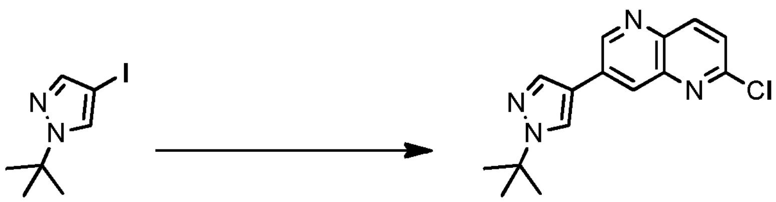

7-(1-(tert-Butyl)-1H-pyrazol-4-yl)-2-chloro-1,5-naphthyridine was obtained in the same manner as in Example 0478-3 except that 1-(tert-butyl)-4-iodo-1H-pyrazole was used instead of the 1-(4-(4-iodo-1H-pyrazol-1-yl)piperidin-1-yl)ethanone used in Example 0478-3.
MSm/z(M+H):287.

<0481-3>

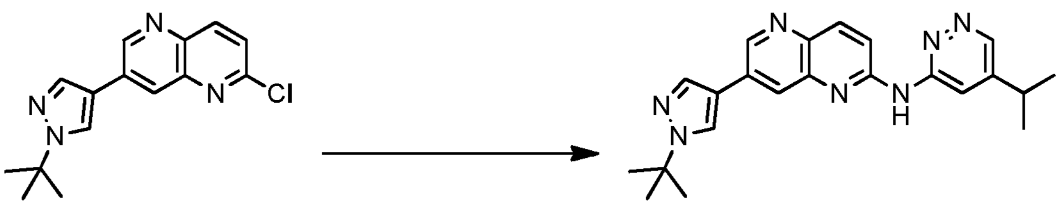

7-(1-(tert-Butyl)-1H-pyrazol-4-yl)-N-(5-isopropylpyridazin-3-yl)-1,5-naphthyridine-2-amine was obtained in the same manner as in Example 0471-2 except that 7-(1-(tert-butyl)-1H-pyrazol-4-yl)-2-chloro-1,5-naphthyridine was used instead of the 2-chloro-7-(1-(2-methoxyethyl)-1H-pyrazol-4-yl)-1,5-naphthyridine used in Example 0471-2.
$^1$H-NMR($CDCl_3$/$CD_3OD$=4/1):8.90(1H,d,J=1.8Hz),8.87(1H,brs),8.73(1H,brs),8.20(1H,d,J=9.3Hz),8.16(1 H,d,J=1.8Hz),8.07(1H,s),8.00(1H,s),7.50(1H,d,J=9.3Hz),3.15-2.98(1H,m),1.69(9H,s),1.42(6H,d,J=6.6Hz).
MSm/z(M+H):388.

<Example 0482>

<0482-1>

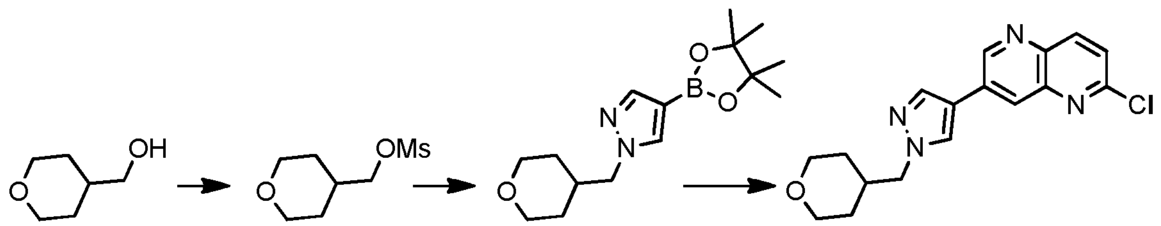

2-Chloro-7-(1-((tetrahydro-2H-pyran-4-yl)methyl)-1H-pyrazol-4-yl)-1,5-naphthyridine was obtained in the same manner as in Example 0475-1 except that (tetrahydro-2H-pyran-4-yl)methanol was used instead of the tetrahydro-2H-pyran-4-ol used in Example 0475-1.
MSm/z (M+H):329.

<0482-2>

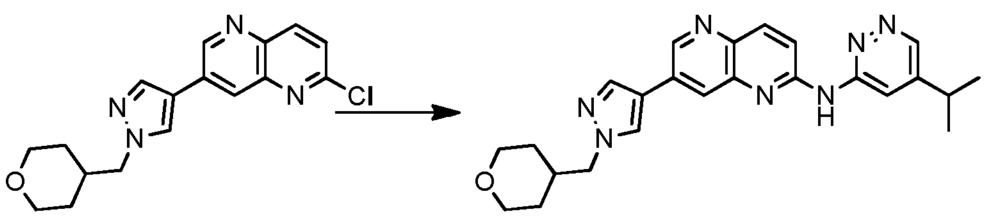

N-(5-isopropylpyridazin-3-yl)-7-(1-((tetrahydro-2H-pyran-4-yl)methyl)-1H-pyrazol-4-yl)-1,5-naphthyridine-2-amine was obtained in the same manner as in Example 0471-2 except that 2-chloro-7-(1-((tetrahydro-2H-pyran-4-yl)methyl)-1H-pyrazol-4-yl)-1,5-naphthyridine was used instead of the 2-chloro-7-(1-(2-methoxyethyl)-1H-pyrazol-4-yl)-1,5-naphthyridine used in Example 0471-2.
$^1$H-NMR($CDCl_3$/$CD_3OD$=4/1):8.88(2H,brs),8.73(1H,brs),8.21(1H,d,J=9.3Hz),8.15(1H,d,J=1.8Hz),8.00(1H,s),7.96(1H,s),7.52(1H,d,J=9.3Hz),4.12(2H,d,J=6.6Hz),3.48-3.35(4H,m),3.12-3.02(1H,m),2.30-2.20(1H,m),1.64-1.54(2H,m),1.52-1.37(2H,m),1.42(6H,d,J=7.2Hz).
MSm/z(M+H):430.

<Example 0483>

<0483-1>

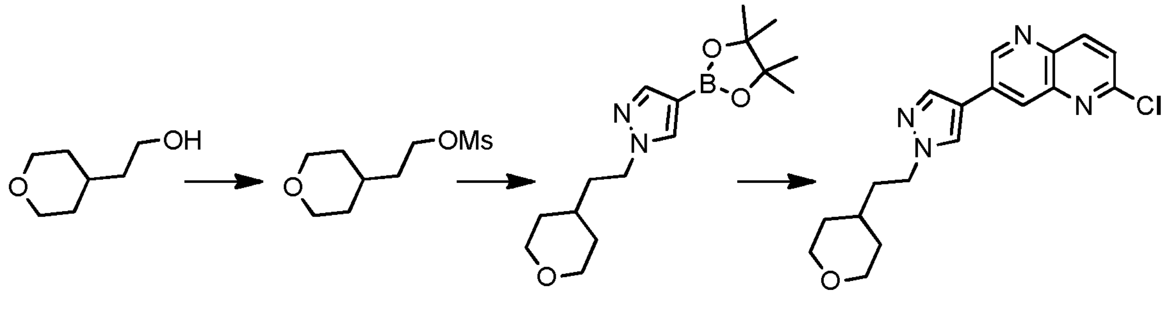

2-Chloro-7-(1-(2-(tetrahydro-2H-pyran-4-yl)ethyl)-1H-pyrazol-4-yl)-1,5-naphthyridine was obtained in the same manner as in Example 0475-1 except that 2-(tetrahydro-2H-pyran-4-yl)ethanol was used instead of the tetrahydro-2H-pyran-4-ol used in Example 0475-1.
MSm/z(M+H):343.

<0483-2>

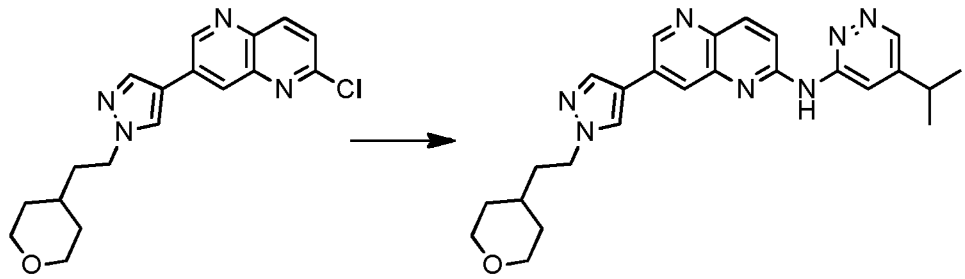

N-(5-isopropylpyridazin-3-yl)-7-(1-(2-(tetrahydro-2H-pyran-4-yl)ethyl)-1H-pyrazol-4-yl)-1,5-naphthyridine-2-amine was obtained in the same manner as in Example 0471-2 except that 2-chloro-7-(1-(2-(tetrahydro-2H-pyran-4-yl)ethyl)-1H-pyrazol-4-yl)-1,5-naphthyridine was used instead of the 2-chloro-7-(1-(2-methoxyethyl)-1H-pyrazol-4-yl)-1,5-naphthyridine used in Example 0471-2.
$^1$H-NMR($CDCl_3$/$CD_3OD$=4/1):8.88(2H,brs),8.72(1H,brs),8.20(1H,d,J=9.3Hz),8.15(1H,brs),7.98(1H,s),7.97(1H,s),7.51(1H,d,J=9.3Hz),4.29(2H,t,J=7.5Hz),3.47-3.34(4H,m),3.12-3.01(1H,m),1.98-1.88(2H,m),1.74-1.65(2H,m),1.65-1.52(1H,m),1.47-1.32(2H,m),1.42(6H,d,J=7.2Hz).
MSm/z (M+H):444.

<Example 0484>

<0484-1>

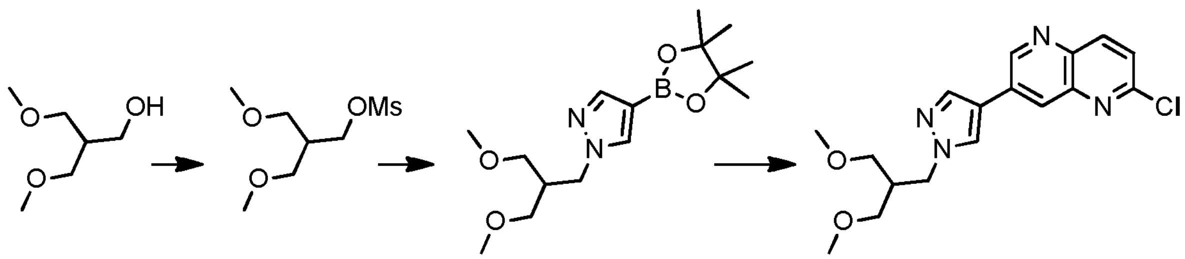

2-Chloro-7-(1-(3-methoxy-2-(methoxymethyl)propyl)-1H-pyrazol-4-yl)-1,5-naphthyridine was obtained in the same manner as in Example 0475-1 except that 3-methoxy-2-(methoxymethyl)propan-1-ol was used instead of the tetrahydro-2H-pyran-4-ol used in Example 0475-1.
MSm/z (M+H):347.

<0484-2>

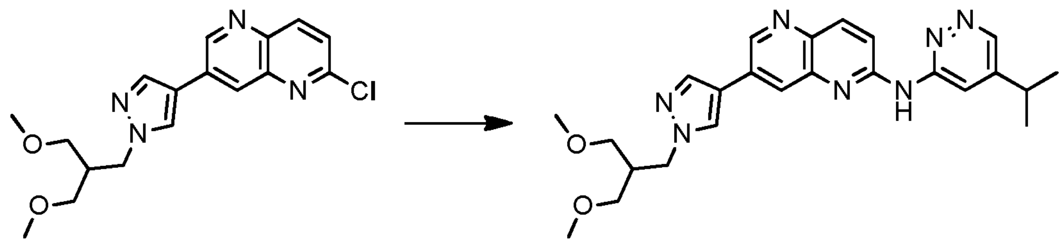

N-(5-isopropylpyridazin-3-yl)-7-(1-(3-methoxy-2-(methoxymethyl)propyl)-1H-pyrazol-4-yl)-1,5-naphthyridine-2-amine was obtained in the same manner as in Example 0471-2 except that 2-chloro-7-(1-(3-methoxy-2-(methoxymethyl)propyl)-1H-pyrazol-4-yl)-1,5-naphthyridine was used instead of the 2-chloro-7-(1-(2-methoxyethyl)-1H-pyrazol-4-yl)-1,5-naphthyridine used in Example 0471-2.
$^1$H-NMR($CDCl_3$/$CD_3OD$=4/1):8.90(1H,brs),8.89(1H,d,J=2.1Hz),8.72(1H,brs),8.20(1H,d,J=9.3Hz),8.14(1 H,brs),7.98(1H,s),7.95(1H,s),7.50(1H,d,J=9.3Hz),4.32(2H,d,J=7.2Hz),4.01(6H,s),3.39(4H,d,J=5.4Hz), 3.15-3.01(1H,m),2.59-2.44(1H,m),1.42(6H,d,J=6.6Hz).
MSm/z (M+H):448.

<Example 0485>

<0485-1>

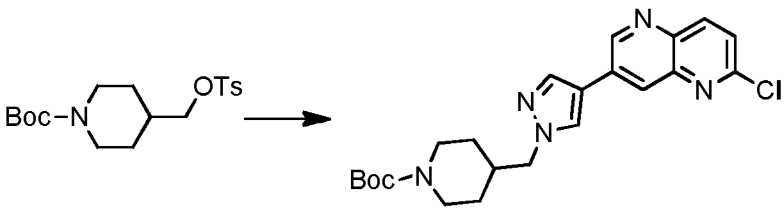

A suspension of tert-butyl 4-((paratoluenesulfonyloxy)methyl)piperidine-1-carboxylate (185 mg), 2-chloro-7-(1H-pyrazol-4-yl)-1,5-naphthyridine (115 mg), and cesium carbonate (326 mg) in N,N-dimethylformamide (1.5 mL) was stirred at 110°C for 1.5 hours. After the reaction mixture was cooled to room temperature, ethyl acetate and water were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining tert-butyl 4-((4-(6-chloro-1,5-naphthyridin-3-yl)-1H-pyrazol-1-yl)methyl)piperidine-1-carboxylate (142 mg) as a white solid.
MSm/z(M+H):428.

<0485-2>

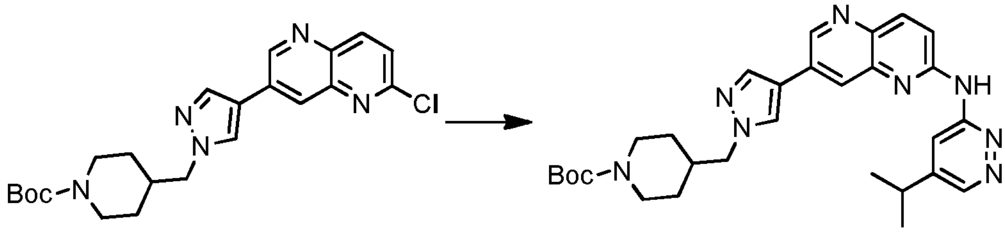

A suspension of tert-butyl 4-((4-(6-chloro-1,5-naphthyridin-3-yl)-1H-pyrazol-1-yl)methyl)piperidine-1-carboxylate (86 mg), 5-isopropylpyridazine-3-amine (30 mg), ((2-dicyclohexylphosphino-3,6-dimethoxy-2',4',6'-triisopropyl-1,1'-biphenyl)-2-(2'-amino-1,1'-biphenyl))palladium(II) methanesulfonate (BRETTPHOS PD G3 (product name, manufactured by Sigma-Aldrich Co. LLC.)) (4.5 mg), and cesium carbonate (130 mg) in 1,4-dioxane (1 mL) was stirred at 110°C

for 1 hour in a nitrogen atmosphere in a sealed tube. After the reaction mixture was cooled to room temperature, ethyl acetate and water were added thereto. The organic layer was collected by separation, washed with a saturated sodium chloride aqueous solution, and dried over anhydrous sodium sulfate, and the solvent was distilled off under reduced pressure. Ethyl acetate was added to the obtained residue, and the solid matter was collected by filtration, thereby obtaining tert-butyl 4-((4-(6-((5-isopropylpyridazin-3-yl)amino)-1,5-naphthyridin-3-yl)-1H-pyrazol-1-yl)methyl)piperidine-1-carboxylate (55 mg) as a white solid.
MSm/z(M+H):529.

<0485-3>

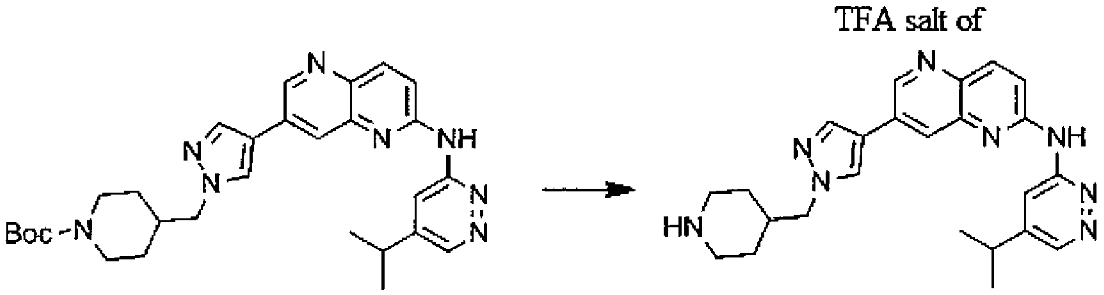


Trifluoroacetic acid (1 mL) was added to tert-butyl 4-((4-(6-((5-isopropylpyridazin-3-yl)amino)-1,5-naphthyridin-3-yl)-1H-pyrazol-1-yl)methyl)piperidine-1-carboxylate (50 mg), followed by stirring at room temperature for 30 minutes. The solvent was distilled off under reduced pressure, thereby obtaining trifluoroacetic acid salt of N-(5-isopropylpyridazin-3-yl)-7-(1-(piperidin-4-ylmethyl)-1H-pyrazol-4-yl)-1,5-naphthyridine-2-amine (39 mg) as a pale yellow solid. $^1$H-NMR(DMSO-$d_6$)$\delta$:9.16(1H,d,J=1.8Hz),9.02(1H,d,J=2.1Hz),8.65-8.54(3H,m),8.45-8.36(2H,m),8.25(1H,s),7.67(1H,d,J=9.0Hz),4.15(2H,d,J=6.6Hz),3.33-3.12(2H,m),3.17-3.09(1H,m),2.95-2.82(2H,m),2.25-2.15(1H,m),1.76-1.68(2H,m),1.49-1.32(8H,m).
MSm/z(M+H):429.

<Example 0486>

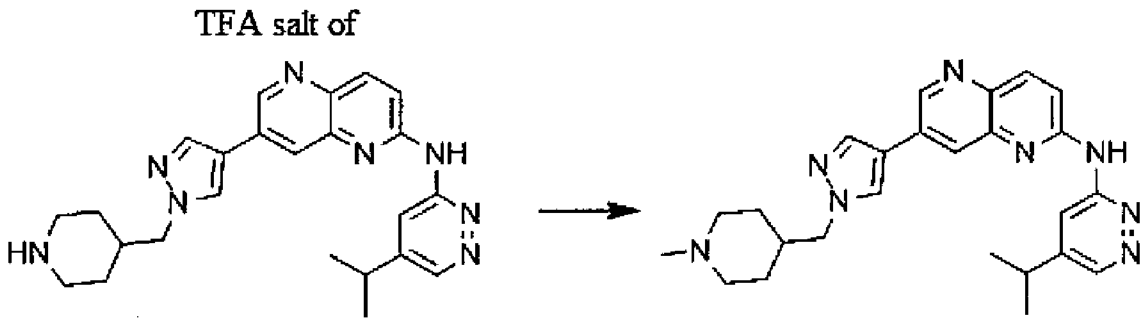


Triethylamine was added to a solution of N-(5-isopropylpyridazin-3-yl)-7-(1-(piperidin-4-ylmethyl)-1H-pyrazol-4-yl)-1,5-naphthyridine-2-amine, and trifluoroacetate (20 mg) in dichloromethane (0.25 mL) and methanol (0.25 mL), followed by adjusting to pH 8. A 37% (w/w) formaldehyde aqueous solution (0.1 mL) and sodium triacetoxyborohydride (40 mg) were added thereto, followed by stirring at room temperature for 30 minutes. The solvent was distilled off under reduced pressure, and the obtained residue was purified by silica gel column chromatography (ethyl acetate-methanol, NH silica), thereby obtaining N-(5-isopropylpyridazin-3-yl)-7-(1-((1-methylpiperidin-4-yl)methyl)-1H-pyrazol-4-yl)-1,5-naphthyridine-2-amine (13 mg) as a pale yellow solid.
$^1$H-NMR(DMSO-$d_6$)$\delta$:10.70(1H,s),9.04(1H,d,J=1.8Hz),8.87(1H,d,J=2.1Hz),8.73(1H,s),8.50(1H,s),8.22-8.18(3H,m),7.71(1H,d,J=9.3Hz),4.07(2H,d,J=6.3Hz),3.09-3.00(1H,m),2.78-2.72(2H,m),2.14(3H,s),1.87-1.78(3H,m),1.53-1.47(2H,m),1.33(6H,d,J=5.7H

z),1.29-1.22(2H,m).
MSm/z(M+H):443.

<Example 0487>

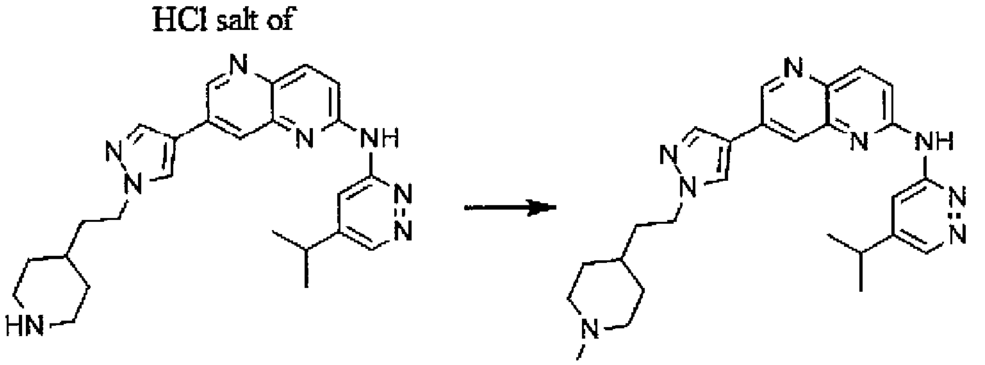


N-(5-isopropylpyridazin-3-yl)-7-(1-(2-(1-methylpiperidin-4-yl)ethyl)-1H-pyrazol-4-yl)-1,5-naphthyridine-2-amine was obtained as a white solid in the same manner as in Example 0486 except that hydrochloric acid salt of N-(5-isopropylpyridazin-3-yl)-7-(1-(2-(piperidin-4-yl)ethyl)-1H-pyrazol-4-yl)-1,5-naphthyridine-2-amine was used instead of the trifluoroacetic acid salt of N-(5-isopropylpyridazin-3-yl)-7-(1-(piperidin-4-yl)methyl-1H-pyrazol-4-yl)-1,5-naphthyridine-2-amine used in Example 0486.
$^1$H-NMR(DMSO-$d_6$)δ:10.71(1H,s),9.04(1H,d,J=1.8Hz),8.87(1H,d,J=2.1Hz),8.73(1H,s),8.53(1H,s),8.22-8.17(3H,m),7.72(1H,d,J=9.3Hz),4.22-4.17(2H,m),3.09-2.99(1H,m),2.75-2.69(2H,m),2.11(3H,s),1.81-1.73(4H,m),1.70-1.64(2H,m),1.34(6H,d,J=5.7Hz),1.24-1.15(3H,m).
MSm/z(M+H):457.

<Example 0488>

<0488-1>

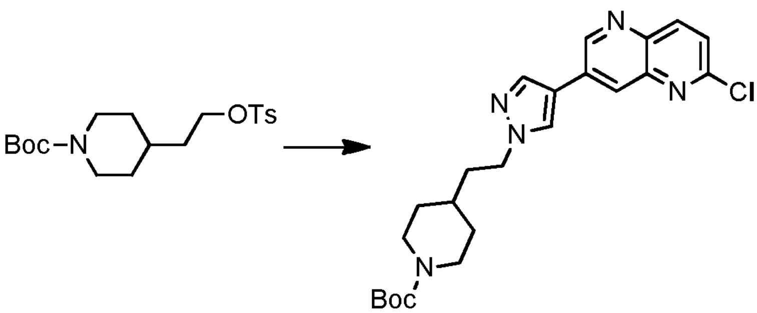

tert-Butyl 4-(2-(4-(6-chloro-1,5-naphthyridin-3-yl)-1H-pyrazol-1-yl)ethyl)piperidine-1-carboxylate was obtained as a white solid in the same manner as in Example 0485-1 except that tert-butyl 4-(2-(paratoluenesulfonyloxy)ethyl)piperidine-1-carboxylate was used instead of the tert-butyl 4-((paratoluenesulfonyloxy)methyl)piperidine-1-carboxylate used in Example 0485-1.
MSm/z (M+H):442.

<0488-2>

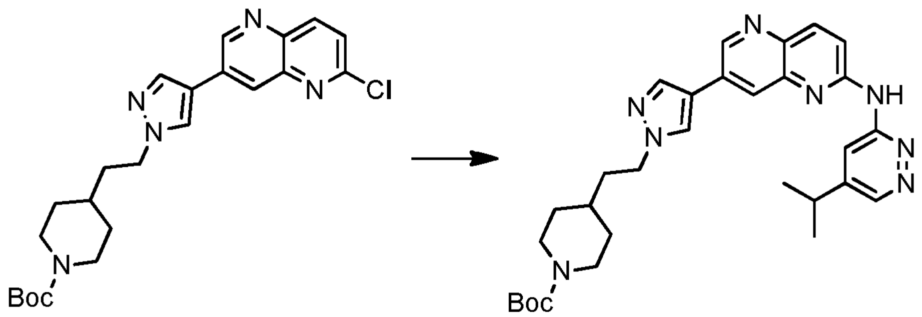

tert-Butyl 4-(2-(4-(6-((5-isopropylpyridazin-3-yl)amino)-1,5-naphthyridin-3-yl)-1H-pyrazol-1-yl)ethyl)piperidine-1-carboxylate was obtained as a white solid in the same manner as in Example 0485-2 except that tert-butyl 4-(2-(4-(6-chloro-1,5-naphthyridin-3-yl)-1H-pyrazol-1-yl)ethyl)piperidine-1-carboxylate was used instead of the tert-butyl 4-((4-(6-chloro-1,5-naphthyridin-3-yl)-1H-pyrazol-1-yl)methyl)piperidine-1-carboxylate used in Example 0485-2.
MSm/z(M+H):543.

<0488-3>

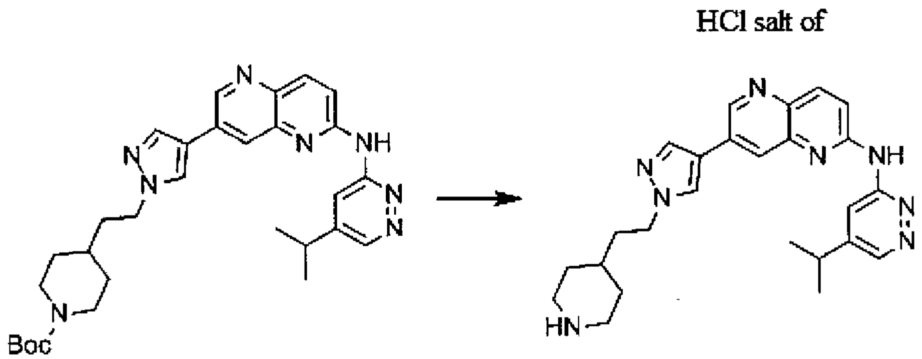


A 4 mol/L hydrogen chloride/1,4-dioxane solution (1 mL) was added to tert-butyl 4-(2-(4-(6-((5-isopropylpyridazin-3-yl)amino)-1,5-naphthyridin-3-yl)-1H-pyrazol-1-yl)ethyl)piperidine-1-carboxylate (54 mg), followed by stirring at room temperature for 1 hour. The solvent was distilled off under reduced pressure, thereby obtaining hydrochloric acid salt of N-(5-isopropylpyridazin-3-yl)-7-(1-(2-(piperidin-4-yl)ethyl)-1H-pyrazol-4-yl)-1,5-naphthyridine-2-amine (31 mg) as a white solid. $^1$H-NMR(DMSO-$d_6$)δ:9.29(1H,d,J=1.8Hz),9.13(1H,d,J=2.1Hz),9.00-8.95(2H,m),8.63(1H,s),8.55(1H,d,J=9.3Hz),8.39(1H,s),8.23(1H,s),7.88(1H,d,J=9.3Hz),4.30-4.22(2H,m),3.23-3.16(2H,m),2.88-2.73(2H,m),1.89-1.78(4H,m),1.56-1.32(10H,m).
MSm/z(M+H):443.

<Example 0489>

<0489-1>

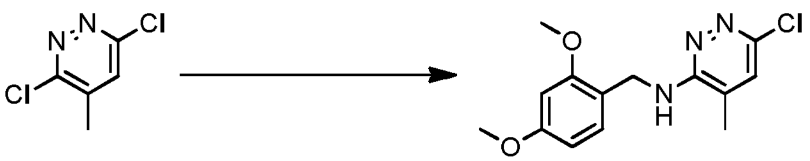

6-Chloro-N-(2,4-dimethoxybenzyl)-4-methylpyridazine-3-amine was obtained in the same manner as in Example 0014-2 except that 3,6-dichloro-4-methylpyridazine was used instead of the 3,6-dichloro-4-isopropylpyridazine used in Example 0014-2.

MSm/z(M+H):294.

<0489-2>

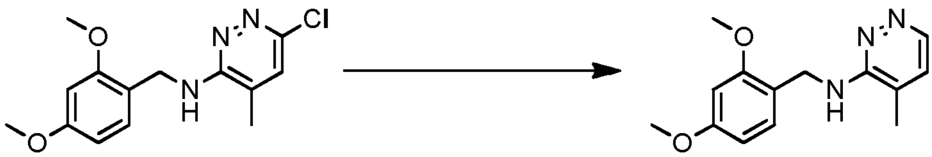

N-(2,4-dimethoxybenzyl)-4-methylpyridazine-3-amine was obtained in the same manner as in Example 0295-3 except that 6-chloro-N-(2,4-dimethoxybenzyl)-4-methylpyridazine-3-amine was used instead of the 6-chloro-5-cyclopropyl-N-(2,4-dimethoxybenzyl)pyridazine-3-amine used in Example 0295-3.
MSm/z (M+H):260.

<0489-3>

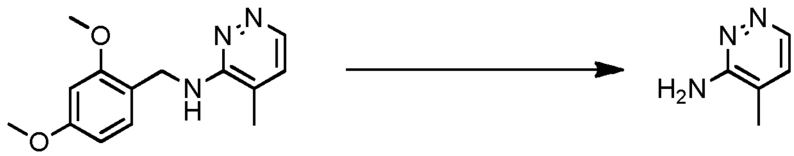

4-Methylpyridazine-3-amine was obtained in the same manner as in Example 0295-4 except that N-(2,4-dimethoxybenzyl)-4-methylpyridazine-3-amine was used instead of the 5-cyclopropyl-N-(2,4-dimethoxybenzyl)pyridazine-3-amine used in Example 0295-4.
MSm/z(M+H):110.

<0489-4>

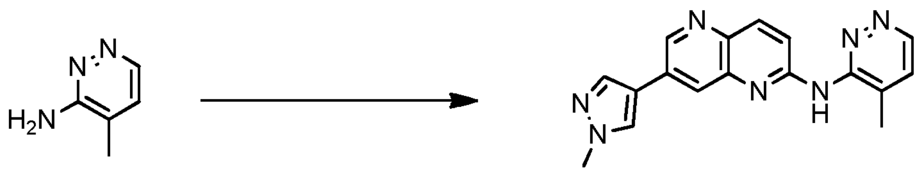

7-(1-Methyl-1H-pyrazol-4-yl)-N-(4-methylpyridazin-3-yl)-1,5-naphthyridine-2-amine was obtained in the same manner as in Example 0471-2 except that 4-methylpyridazine-3-amine was used instead of the 5-isopropylpyridazine-3-amine used in Example 0471-2, and 2-chloro-7-(1-methyl-1H-pyrazol-4-yl)-1,5-naphthyridine was used instead of the 2-chloro-7-(1-(2-methoxyethyl)-1H-pyrazol-4-yl)-1,5-naphthyridine used in Example 0471-2.
$^{1}$H-NMR($CDCl_3$):8.96(1H,d,J=2.1Hz),8.81(1H,d,J=9.3Hz),8.34(1H,d,J=9.3Hz),8.11(1H,d,J=9.3Hz),8.09( 1H,s),7.94(1H,s),7.82(1H,s),7.40(1H,d,J=9.3Hz),4.02(3H,s),2.52(3H,s).
MSm/z(M+H):318.

<Example 0490>

<0490-1>

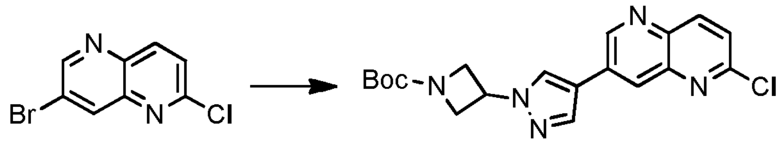

A suspension of 7-bromo-2-chloro-1,5-naphthyridine (183 mg), bis(pinacolato)diboron (190 mg), (1,1'-bis(diphenylphosphino)ferrocene)palladium(II) dichloride (46 mg), and potassium acetate (147 mg) in 1,4-dioxane (2 mL) was stirred at 100°C for 1 hour in a nitrogen atmosphere. tert-Butyl 3-(4-bromo-1H-pyrazol-1-yl)azetidine-1-carboxylate (151 mg), sodium carbonate (106 mg) and bis(di-tert-butyl (4-dimethylaminophenyl)phosphine)dichloropalladium(II) (18 mg) were added to the reaction mixture, followed by stirring at 110°C for 1 hour. After the reaction mixture was cooled to room temperature, ethyl acetate and water were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining tert-butyl 3-(4-(6-chloro-1,5-naphthyridin-3-yl)-1H-pyrazol-1-yl)azetidine-1-carboxylate (63 mg) as a white solid.
MSm/z(M+H):386.

<0490-2>

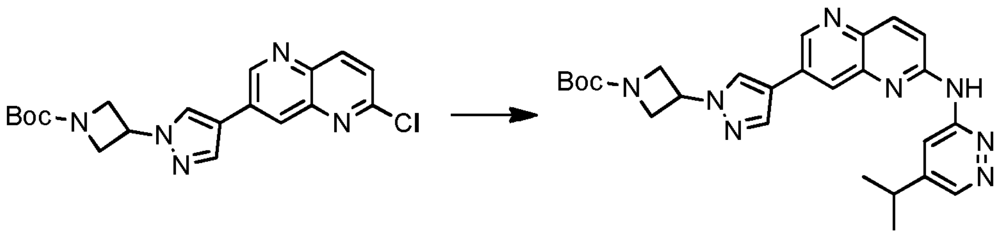

tert-Butyl 3-(4-(6-((5-isopropylpyridazin-3-yl)amino)-1,5-naphthyridin-3-yl)-1H-pyrazol-1-yl)azetidine-1-carboxylate was obtained as a white solid in the same manner as in Example 0485-2 except that tert-butyl 3-(4-(6-chloro-1,5-naphthyridin-3-yl)-1H-pyrazol-1-yl)azetidine-1-carboxylate was used instead of the tert-butyl 4-((4-(6-chloro-1,5-naphthyridin-3-yl)-1H-pyrazol-1-yl)methyl)piperidine-1-carboxylate used in Example 0485-2.
MSm/z(M+H):487.

<0490-3>

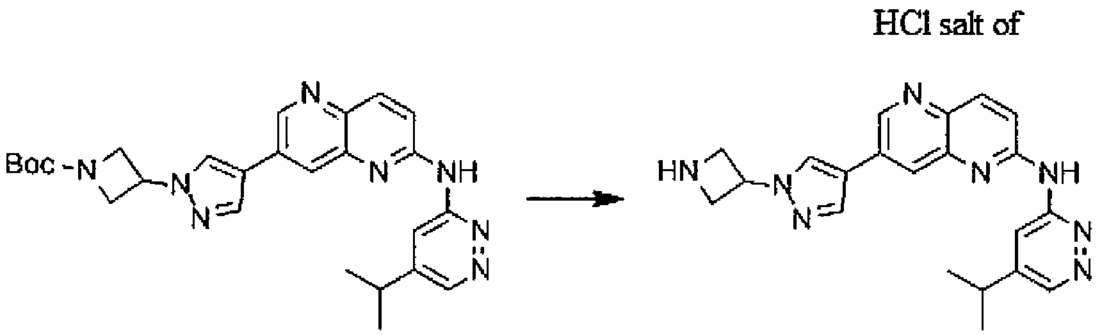


Hydrochloric acid salt of 7-(1-(azetidin-3-yl)-1H-pyrazol-4-yl)-N-(5-isopropylpyridazin-3-yl)-1,5-naphthyridine-2-amine was obtained as a pale yellow solid in the same manner as in Example 0488-3 except that tert-butyl 3-(4-(6-((5-isopropylpyridazin-3-yl)amino)-1,5-naphthyridin-3-yl)-1H-pyrazol-1-yl)azetidine-1-carboxylate was used instead of the tert-butyl 4-(2-(4-(6-((5-isopropylpyridazin-3-yl)amino)-1,5-naphthyridin-3-yl)-1H-pyrazol-1-yl)ethyl)piperidine-1-carboxylate used in Example 0488-3.

MSm/z (M+H):387.

<0490-4>

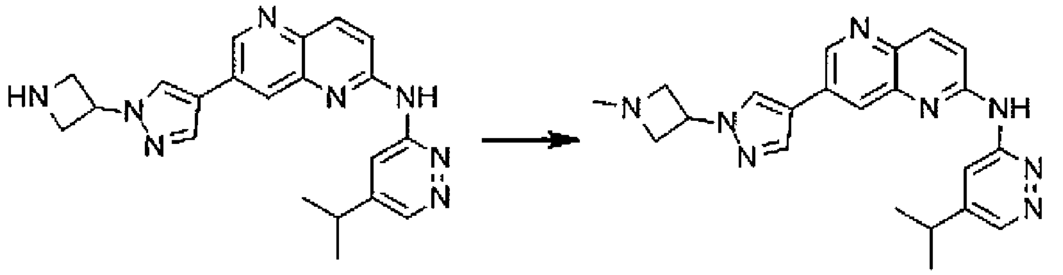

N-(5-isopropylpyridazin-3-yl)-7-(1-(1-methylazetidin-3-yl)-1H-pyrazol-4-yl)-1,5-naphthyridine-2-amine was obtained as a white solid in the same manner as in Example 0486 except that hydrochloric acid salt of 7-(1-(azetidin-3-yl)-1H-pyrazol-4-yl)-N-(5-isopropylpyridazin-3-yl)-1,5-naphthyridine-2-amine was used instead of the trifluoroacetic acid salt of N-(5-isopropylpyridazin-3-yl)-7-(1-(piperidin-4-ylmethyl)-1H-pyrazol-4-yl)-1,5-naphthyridine-2-amine used in Example 0486.
$^1$H-NMR(DMSO-$d_6$)$\delta$:10.70(1H,s),9.06(1H,d,J=1.8Hz),8.87(1H,d,J=2.1Hz),8.72(1H,s),8.67(1H,s),8.27-8.19(3H,m),7.71(1H,d,J=9.3Hz), 5.06-4.95(1H,m),3.77-3.71(2H,m),3.47-3.40(2H,m),3.08-2.90(1H,m),2.35(3H,s),1.34(6H,d,J=6.6Hz).
MSm/z(M+H):401.

<Example 0491>

<0491-1>

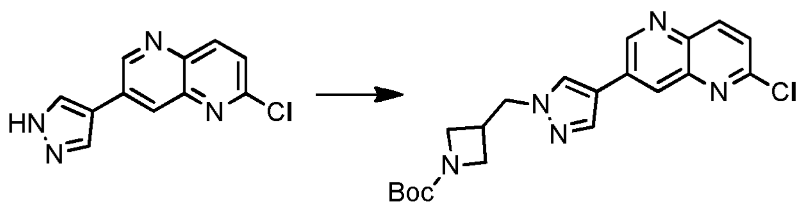

A suspension of 2-chloro-7-(1H-pyrazol-4-yl)-1,5-naphthyridine (115 mg), tert-butyl 3-((paratoluenesulfonyloxy)methyl)azetidine-1-carboxylate (205 mg), and potassium carbonate (138 mg) in N,N-dimethylformamide (2 mL) was stirred at 100°C for 1.5 hours. After the reaction mixture was cooled to room temperature, ethyl acetate and water were added thereto. The organic layer was collected by separation, washed with a saturated sodium chloride aqueous solution, and dried over anhydrous sodium sulfate, and the solvent was distilled off under reduced pressure. Ethyl acetate was added to the obtained residue, and the solid matter was collected by filtration, thereby obtaining tert-butyl 3-((4-(6-chloro-1,5-naphthyridin-3-yl)-1H-pyrazol-1-yl)methyl)azetidine-1-carboxylate (72 mg) as a white solid.
MSm/z(M+H):400.

<0491-2>

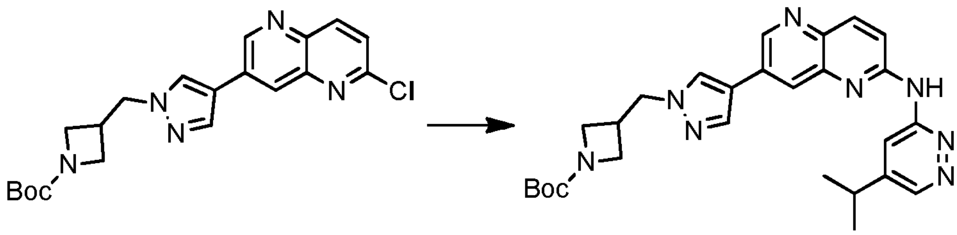

tert-Butyl 3-((4-(6-((5-isopropylpyridazin-3-yl)amino)-1,5-naphthyridin-3-yl)-1H-pyrazol-1-yl)methyl)azetidine-1-carboxylate was obtained as a white solid in the same manner as in Example 0485-2 except that tert-butyl 3-((4-(6-chloro-1,5-naphthyridin-3-yl)-1H-pyrazol-1-yl)methyl)azetidine-1-carboxylate was used instead of the tert-butyl 4-((4-(6-chloro-1,5-naphthyridin-3-yl)-1H-pyrazol-1-yl)methyl)piperidine-1-carboxylate used in Example 0485-2.
MSm/z(M+H):501.

<0491-3>

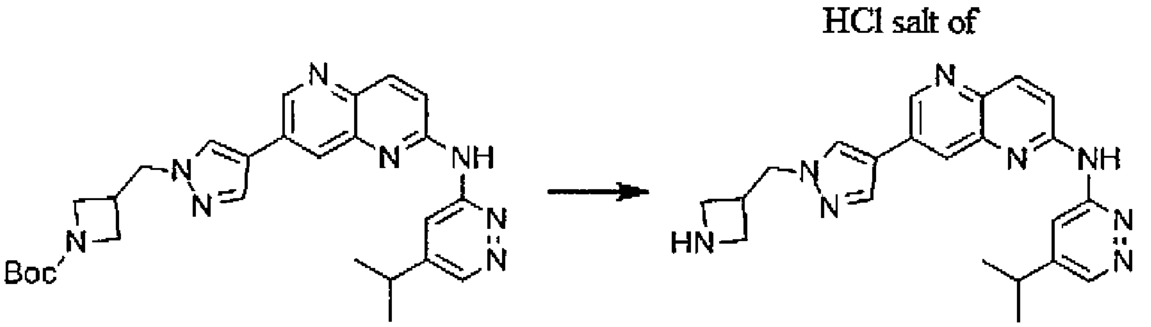


Hydrochloric acid salt of 7-(1-(azetidin-3-ylmethyl)-1H-pyrazol-4-yl)-N-(5-isopropylpyridazin-3-yl)-1,5-naphthyridine-2-amine was obtained as a pale yellow solid in the same manner as in Example 0488-3 except that tert-butyl 3-((4-(6-((5-isopropylpyridazin-3-yl)amino)-1,5-naphthyridin-3-yl)-1H-pyrazol-1-yl)methyl)azetidine-1-carboxylate was used instead of the tert-butyl 4-(2-(4-(6-((5-isopropylpyridazin-3-yl)amino)-1,5-naphthyridin-3-yl)-1H-pyrazol-1-yl)ethyl)piperidine-1-carboxylate used in Example 0488-3.
MSm/z(M+H):401.

<0491-4>

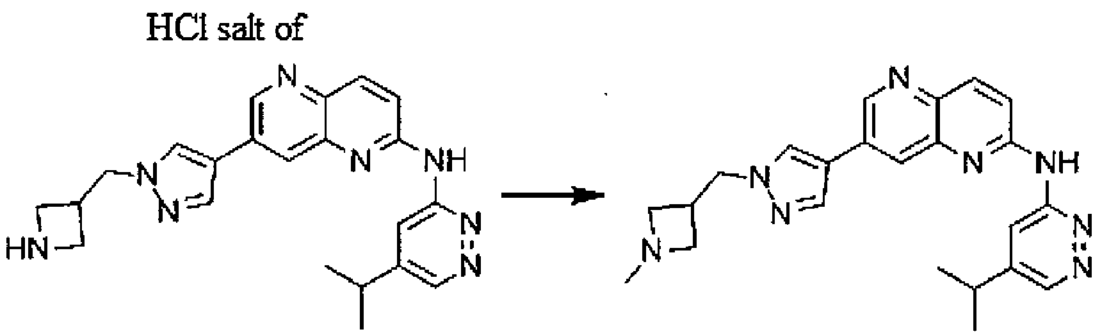


N-(5-isopropylpyridazin-3-yl)-7-(1-((1-methylazetidin-3-yl)methyl)-1H-pyrazol-4-yl)-1,5-naphthyridine-2-amine was obtained as a white solid in the same manner as in Example 0486 except that hydrochloric acid salt of 7-(1-(azetidin-3-ylmethyl)-1H-pyrazol-4-yl)-N-(5-isopropylpyridazin-3-yl)-1,5-naphthyridine-2-amine was used instead of the trifluoroacetic acid salt of N-(5-isopropylpyridazin-3-yl)-7-(1-(piperidin-4-ylmethyl)-1H-pyrazol-4-yl)-1,5-naphthyridine-2-amine

used in Example 0486.
$^1$H-NMR(DMSO-$d_6$)δ:10.71(1H,s),9.04(1H,d,J=1.8Hz),8.87(1H,d,J=2.1Hz),8.72(1H,s),8.53(1H,s),8.22-8.17(3H,m),7.71(1H,d,J=9.3Hz), 4.36(2H,d,J=7.2Hz),3.25-3.18(2H,m),3.09-2.93(3H,m),2.85-2.76(1H,m),2.19(3H,s),1.34(6H,d,J=7.2Hz).
MSm/z(M+H):415.

<Example 0492>

<0492-1>

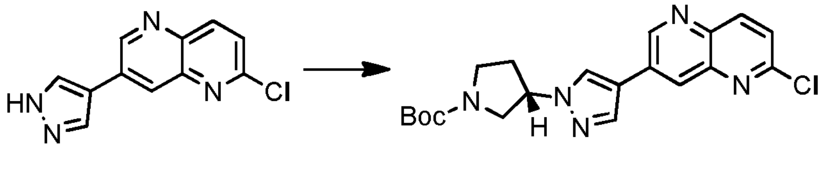

(S)-tert-butyl 3-(4-(6-chloro-1,5-naphthyridin-3-yl)-1H-pyrazol-1-yl)pyrrolidine-1-carboxylate was obtained as a white solid in the same manner as in Example 0491-1 except that (R)-tert-butyl 3-(paratoluenesulfonyloxy)pyrrolidine-1-carboxylate was used instead of the tert-butyl 3-((paratoluenesulfonyloxy)methyl)azetidine-1-carboxylate used in Example 0491-1.
MSm/z(M+H):400.

<0492-2>

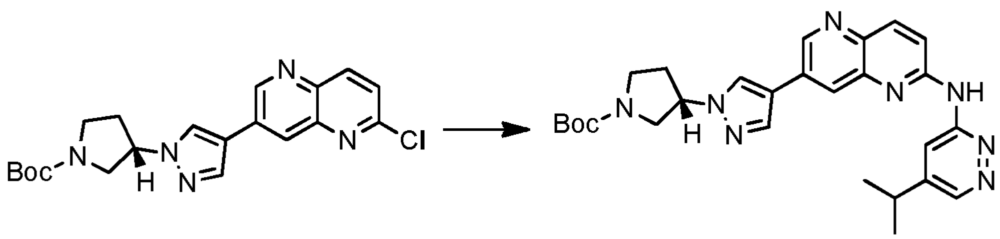

(S)-tert-butyl 3-(4-(6-((5-isopropylpyridazin-3-yl)amino)-1,5-naphthyridin-3-yl)-1H-pyrazol-1-yl)pyrrolidine-1-carboxylate was obtained as a white solid in the same manner as in Example 0485-2 except that (S)-tert-butyl 3-(4-(6-chloro-1,5-naphthyridin-3-yl)-1H-pyrazol-1-yl)pyrrolidine-1-carboxylate was used instead of the tert-butyl 4-((4-(6-chloro-1,5-naphthyridin-3-yl)-1H-pyrazol-1-yl)methyl)piperidine-1-carboxylate used in Example 0485-2.
MSm/z(M+H):501.

<0492-3>

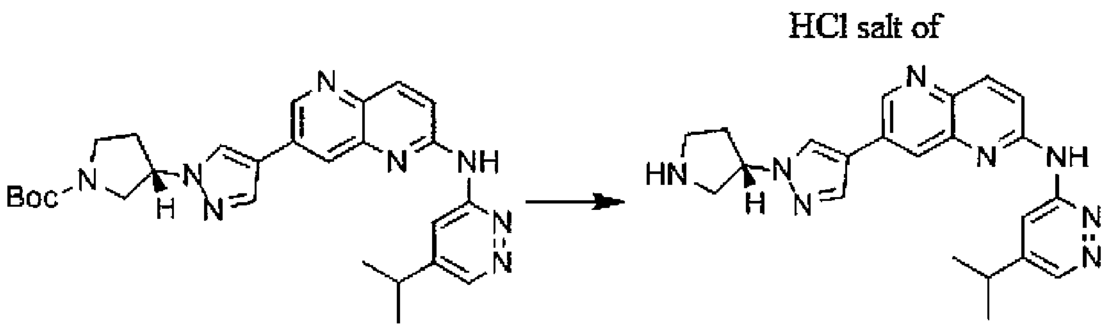

Hydrochloric acid salt of (S)-N-(5-isopropylpyridazin-3-yl)-7-(1-(pyrrolidin-3-yl)-1H-pyrazol-4-yl)-1,5-naphthyridine-2-amine was obtained as a pale yellow solid in the same manner as in Example 0488-3 except that (S)-tert-butyl 3-(4-(6-((5-isopropylpyridazin-3-yl)amino)-1,5-naphthyridin-3-yl)-1H-pyrazol-1-yl)pyrrolidine-1-carboxylate was used instead of the tert-butyl 4-(2-(4-(6-((5-isopropylpyridazin-3-yl)amino)-1,5-naphthyridin-3-yl)-1H-pyrazol-1-yl)ethyl)piperidine-1-carboxylate used in Example 0488-3.
MSm/z(M+H):401.

<0492-4>

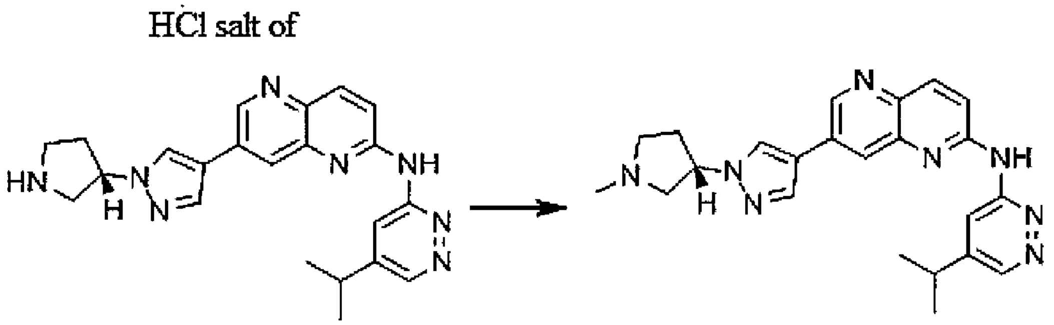

(S)-N-(5-isopropylpyridazin-3-yl)-7-(1-(1-methylpyrrolidin-3-yl)-1H-pyrazol-4-yl)-1,5-naphthyridine-2-amine was obtained as a white solid in the same manner as in Example 0486 except that hydrochloric acid salt of (S)-N-(5-isopropylpyridazin-3-yl)-7-(1-(pyrrolidin-3-yl)-1H-pyrazol-4-yl)-1,5-naphthyridine-2-amine was used instead of the trifluoroacetic acid salt of N-(5-isopropylpyridazin-3-yl)-7-(1-(piperidin-4-ylmethyl)-1H-pyrazol-4-yl)-1,5-naphthyridine-2-amine used in Example 0486.
$^1$H-NMR(DMSO-$d_6$)δ:10.70(1H,s),9.06(1H,d,J=1.8Hz),8.87(1H,d,J=2.1Hz),8.73(1H,s),8.57(1H,s),8.29-8.18(3H,m),7.71(1H,d,J=9.3Hz),5.01-4.93(1H,m),3.09-2.98(1H,m),2.97-2.87(1H,m),2.85-2.76(2H,m),2.59-2.51(1H,m),2.44-2.35(1H,m),2.32(3H,s),2.24-2.12(1H,m),1.34(6H,d,J=6.6Hz).
MSm/z(M+H):415.

<Example 0493>

<0493-1>

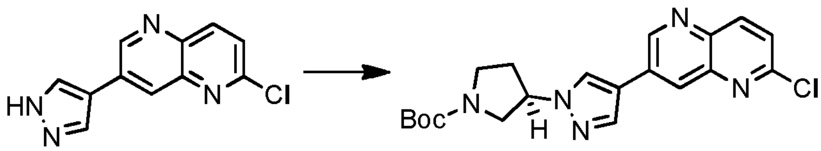

(R)-tert-butyl 3-(4-(6-chloro-1,5-naphthyridin-3-yl)-1H-pyrazol-1-yl)pyrrolidine-1-carboxylate was obtained as a pale yellow solid in the same manner as in Example 0491-1 except that (S)-tert-butyl 3-(paratoluenesulfonyloxy)pyrrolidine-1-carboxylate was used instead of the tert-butyl 3-((paratoluenesulfonyloxy)methyl)azetidine-1-carboxylate used in Example 0491-1.
MSm/z(M+H):400.

<0493-2>

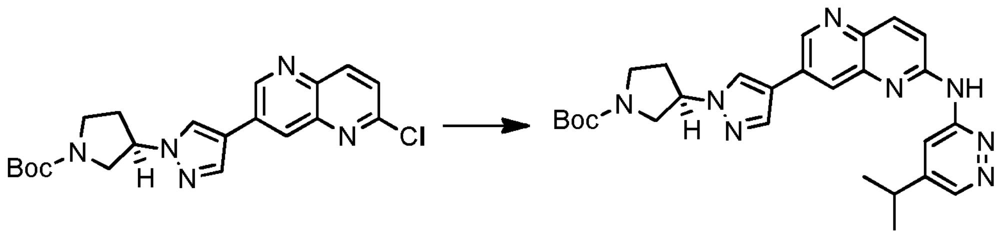

(R)-tert-butyl 3-(4-(6-((5-isopropylpyridazin-3-yl)amino)-1,5-naphthyridin-3-yl)-1H-pyrazol-1-yl)pyrrolidine-1-carboxylate was obtained as a white solid in the same manner as in Example 0485-2 except that (R)-tert-butyl 3-(4-(6-chloro-1,5-naphthyridin-3-yl)-1H-pyrazol-1-yl)pyrrolidine-1-carboxylate was used instead of the tert-butyl 4-((4-(6-chloro-1,5-naphthyridin-3-yl)-1H-pyrazol-1-yl)methyl)piperidine-1-carboxylate used in Example 0485-2.
MSm/z(M+H):501.

<0493-3>

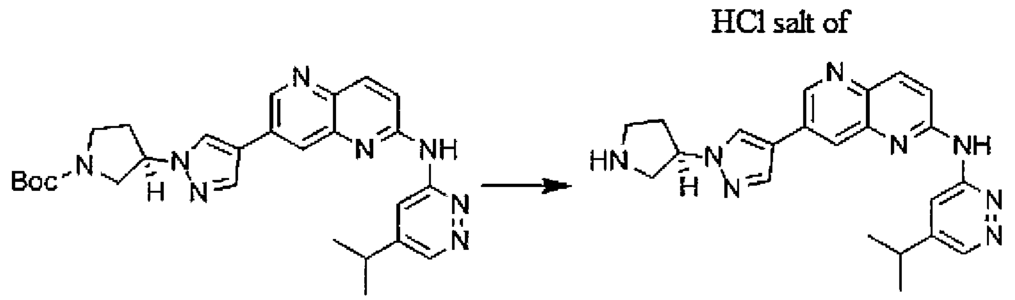


Hydrochloric acid salt of (R)-N-(5-isopropylpyridazin-3-yl)-7-(1-(pyrrolidin-3-yl)-1H-pyrazol-4-yl)-1,5-naphthyridine-2-amine was obtained as a pale yellow solid in the same manner as in Example 0488-3 except that (R)-tert-butyl 3-(4-(6-((5-isopropylpyridazin-3-yl)amino)-1,5-naphthyridin-3-yl)-1H-pyrazol-1-yl)pyrrolidine-1-carboxylate was used instead of the tert-butyl 4-(2-(4-(6-((5-isopropylpyridazin-3-yl)amino)-1,5-naphthyridin-3-yl)-1H-pyrazol-1-yl)ethyl)piperidine-1-carboxylate used in Example 0488-3.
MSm/z(M+H):401.

<0493-4>

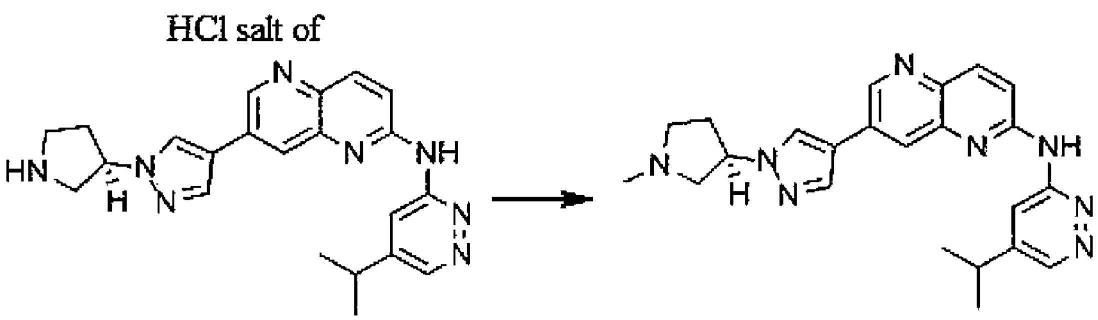


(R)-N-(5-isopropylpyridazin-3-yl)-7-(1-(1-methylpyrrolidin-3-yl)-1H-pyrazol-4-yl)-1,5-naphthyridine-2-amine was obtained as a pale yellow solid in the same manner as in Example 0486 except that hydrochloric acid salt of (R)-N-(5-isopropylpyridazin-3-yl)-7-(1-(pyrrolidin-3-yl)-1H-pyrazol-4-yl)-1,5-naphthyridine-2-amine was used instead of the trifluoroacetic acid salt of N-(5-isopropylpyridazin-3-yl)-7-(1-(piperidin-4-ylmethyl)-1H-pyrazol-4-yl)-1,5-naphthyridine-2-amine used in Example 0486.
$^1$H-NMR(DMSO-

$d_6$)δ:10.70(1H,s),9.06(1H,d,J=1.8Hz),8.87(1H,d,J=2.1Hz),8.73(1H,s),8.57(1H,s),8.29-8.18(3H,m),7.71(1H,d,J=9.3Hz),5.01-4.93(1H,m),3.10-2.98(1H,m),2.97-2.87(1H,m),2.85-2.76(2H,m),2.58-2.51(1H,m),2.44-2.35(1H,m),2.32(3H,s),2.24-2.14(1H,m),1.34(6H,d,J=6.6Hz).
MSm/z(M+H):415.

<Example 0494>

<0494-1>

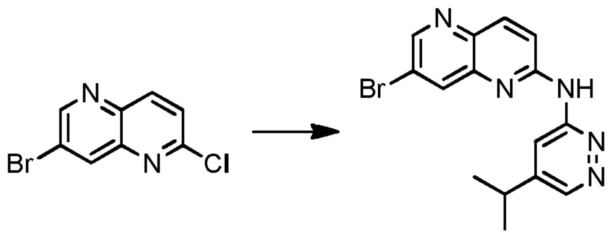

A suspension of 7-bromo-2-chloro-1,5-naphthyridine (1.22 g), 5-isopropylpyridazine-3-amine (755 mg), and potassium tert-butoxide (1.23 g) in N,N-dimethylformamide (10 mL) was stirred at room temperature for 1 hour. After ethyl acetate and water were added to the reaction mixture, the organic layer was collected by separation, washed with a saturated sodium chloride aqueous solution, and dried over anhydrous sodium sulfate, and the solvent was distilled off under reduced pressure. Toluene was added to the obtained residue, and the solid matter was collected by filtration, thereby obtaining 7-bromo-N-(5-isopropylpyridazin-3-yl)-1,5-naphthyridine-2-amine (0.92 g) as a white solid.
MSm/z(M+H):346.

<0494-2>

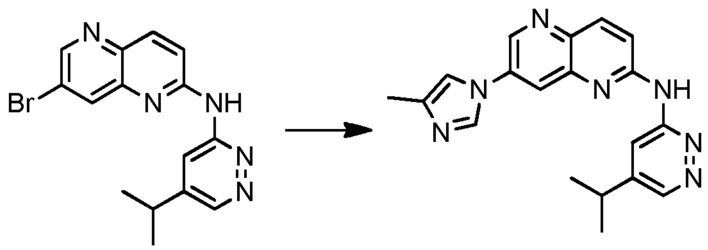

A suspension of tris(dibenzylideneacetone)dipalladium(0) (4.5 mg), 2-(di-tert-butylphosphino)-3,4,5,6-tetramethyl-2',4',6'-triisopropyl-1,1'-biphenyl (4.8 mg), and potassium phosphate (63 mg) in toluene (1 mL) was stirred at 120°C for 3 minutes in a nitrogen atmosphere. 7-Bromo-N-(5-isopropylpyridazin-3-yl)-1,5-naphthyridine-2-amine (10 mg) and 4-methyl-1H-imidazole (7 mg) were added to the reaction mixture, followed by stirring at 120°C for 4 hours. The insolubles were filtered off, and the solvent was distilled off under reduced pressure. The obtained residue was purified by silica gel column chromatography (ethyl acetate-methanol), thereby obtaining N-(5-isopropylpyridazin-3-yl)-7-(4-methyl-1H-imidazol-1-yl)-1,5-naphthyridine-2-amine (0.9 mg) as a white solid.
$^1$H-NMR($CDCl_3$)δ:9.65(1H,s),8.90-8.83(3H,m),8.34(1H,d,J=9.3Hz),8.01(1H,d,J=1.8Hz),8.95(1H,d,J=1.2Hz),7.80(1H,d,J=9.3Hz),7.18(1 H,s),3.12-3.01(1H,m),2.37(3H,s),1.42(6H,d,J=7.2Hz).
MSm/z(M+H):346.

<Example 0495>

<0495-1>

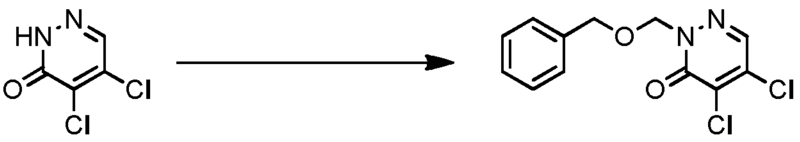

((Chloromethoxy)methyl)benzene (2.17 mL) was added to a mixture of 4,5-dichloropyridazin-3(2H)-one (2.00 g), 1,8-diazabicyclo[5.4.0]undeca-7-ene (1.85 mL), and N-methylpyrrolidone (24 mL) under ice-cooling, followed by stirring at room temperature for 1 hour. After water and ethyl acetate were added to the reaction mixture, the organic layer was collected by separation, washed sequentially with water and a saturated sodium chloride aqueous solution, and dried over anhydrous sodium sulfate, and the solvent was distilled off under reduced pressure, thereby obtaining 2-((benzyloxy)methyl)-4,5-dichloropyridazin-3(2H)-one (2.82 g).
MSm/z(M+H):285.

<0495-2>

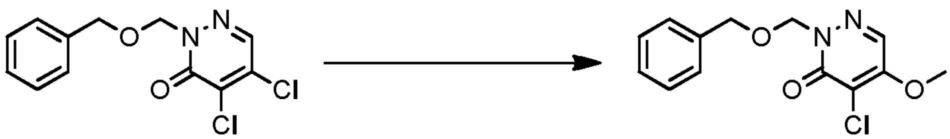

A mixture of 2-((benzyloxy)methyl)-4,5-dichloropyridazin-3(2H)-one (1.82 g), potassium carbonate (4.41 g), and methanol (13 mL) was stirred for 1 hour under heating to reflux. After the reaction mixture was cooled to room temperature, the insolubles were filtered off, and the solvent was distilled off under reduced pressure. The obtained solid matter was washed with water and ethyl acetate, thereby obtaining 2-((benzyloxy)methyl)-4-chloro-5-methoxypyridazin-3(2H)-one (942 mg).
MSm/z(M+H):281.

<0495-3>

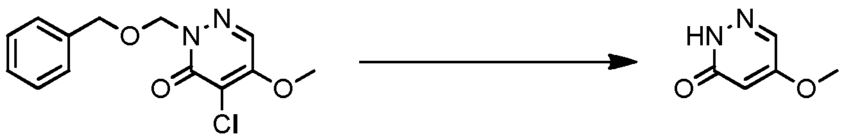

A mixture of 10% palladium/carbon (100 mg), 2-((benzyloxy)methyl)-4-chloro-5-methoxypyridazin-3(2H)-one (1.34 g), acetic acid (10 mL), and methanol (30 mL) was stirred at 50°C for 10 hours in a hydrogen atmosphere (0.8 MPa). The insolubles were filtered off, and the solvent was distilled off under reduced pressure, thereby obtaining 5-methoxypyridazin-3(2H)-one (611 mg).
MSm/z(M+H):127.

<0495-4>

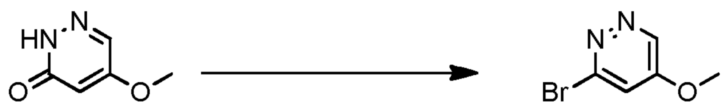

A mixture of 5-methoxypyridazin-3(2H)-one (703 mg), phosphoryl bromide (4.79 g), and acetonitrile (55 mL) was stirred for 1 hour under heating to reflux. The reaction mixture was added dropwise to a sodium carbonate aqueous

solution under ice-cooling, and ethyl acetate was added thereto. The organic layer was collected by separation, washed with a saturated sodium chloride aqueous solution, and dried over anhydrous sodium sulfate, and the solvent was distilled off under reduced pressure. The obtained residue was purified by silica gel chromatography (ethyl acetate-hexane), thereby obtaining 3-bromo-5-methoxypyridazine (175 mg).
MSm/z(M+H):189.

<0495-5>

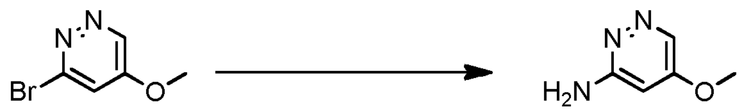

25% ammonia water (2 mL) and copper(I) oxide (61 mg) were added to a solution of 3-bromo-5-methoxypyridazine (81 mg) in 1,4-dioxane (2 mL), followed by stirring at 120°C for 30 minutes using a microwave reaction apparatus. After the reaction mixture was cooled to room temperature, the insolubles were filtered off, and the solvent was distilled off under reduced pressure. The obtained residue was purified by silica gel column chromatography (ethyl acetate-methanol, NH silica), thereby obtaining 5-methoxypyridazine-3-amine (16 mg).
MSm/z(M+H):126.

<0495-6>

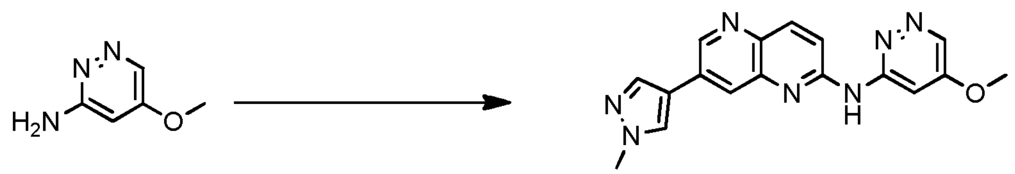

N-(5-methoxypyridazin-3-yl)-7-(1-methyl-1H-pyrazol-4-yl)-1,5-naphthyridine-2-amine was obtained in the same manner as in Example 0489-4 except that 5-methoxypyridazine-3-amine was used instead of the 4-methylpyridazine-3-amine used in Example 0489-4.
$^1$H-NMR($CDCl_3$/$CD_3OD$=4/1)δ:8.92(1H,d,J=2.4Hz),8.73(1H,brs),8.65(1H,brs),8.24(1H,d,J=9.3Hz),8.08( 1H,d,J=2.4Hz),7.95(1H,s),7.86(1H,s),7.60(1H,d,J=9.3Hz),4.08(3H,s),4.02(3H,s).
MSm/z(M+H):334.

<Example 0496>

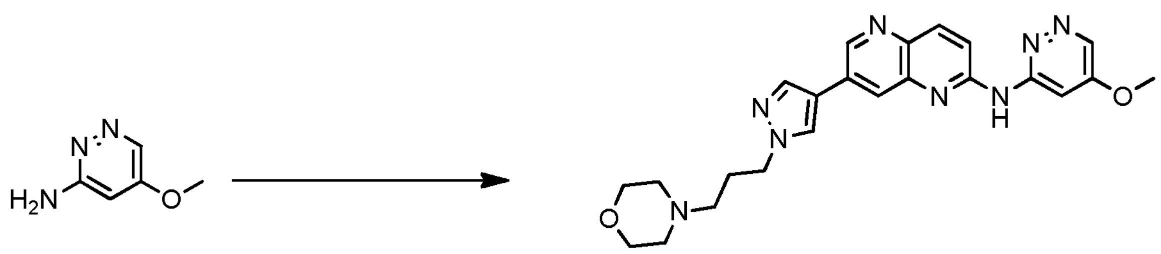

N-(5-methoxypyridazin-3-yl)-7-(1-(3-morpholinopropyl)-1H-pyrazol-4-yl)-1,5-naphthyridine-2-amine was obtained in the same manner as in Example 0495 except that 4-(3-(4-(6-chloro-1,5-naphthyridin-3-yl)-1H-pyrazol-1-yl)propyl)morpholine was used instead of the 2-chloro-7-(1-methyl-1H-pyrazol-4-yl)-1,5-naphthyridine used in Example 0495.
$^1$H-

NMR($CDCl_3$/$CD_3OD$=4/1)δ:8.92(1H,d,J=2.4Hz),8.73(1H,brs),8.65(1H,brs),8.24(1H,d,J=9.3Hz),8.08( 1H,d,J=2.4Hz),7.95(1H,s),7.86(1H,s),7.60(1H,d,J=9.3Hz),4.30(2H,t,J=6.9Hz),4.07(3H,s),3.76-3.70(4H,m),2.49-2.42(4H,m),2.38(2H,t,J=6.9Hz),2.19-2.09(2H,m).
MSm/z(M+H):447.

<Example 0497>

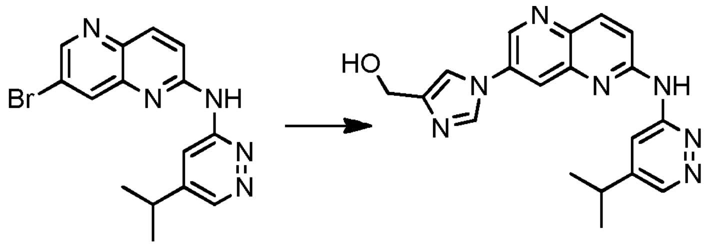

A suspension of 7-bromo-N-(5-isopropylpyridazin-3-yl)-1,5-naphthyridine-2-amine (17 mg), (1H-imidazol-4-yl)methanol (20 mg), copper iodide (3.1 mg), quinolin-8-ol (2.4 mg), and cesium carbonate (98 mg) in N,N-dimethylformamide (1 mL) was stirred at 120°C for 7 hours in a nitrogen atmosphere. The insolubles were filtered off, and the solvent was distilled off under reduced pressure. The obtained residue was purified by silica gel column chromatography (ethyl acetate-methanol, NH silica), thereby obtaining (1-(6-((5-isopropylpyridazin-3-yl)amino)-1,5-naphthyridin-3-yl)-1H-imidazol-4-yl)methanol (3.6 mg) as a white solid.
$^1$H-NMR(DMSO-$d_6$)δ:10.76(1H,s),9.19(1H,d,J=2.1Hz),8.96(1H,d,J=2.7Hz),8.91-8.83(2H,m),8.46(1H,s),8.44-8.42(1H,m),7.78-7.69(1H,m),7.45(1H,d,J=9.3Hz),5.87(2H,s),3.27(1H,s),3.10-3.03(1H,m),1.48(6H,d,J=7.2Hz).
MSm/z(M+H):362.

<Example 0498>

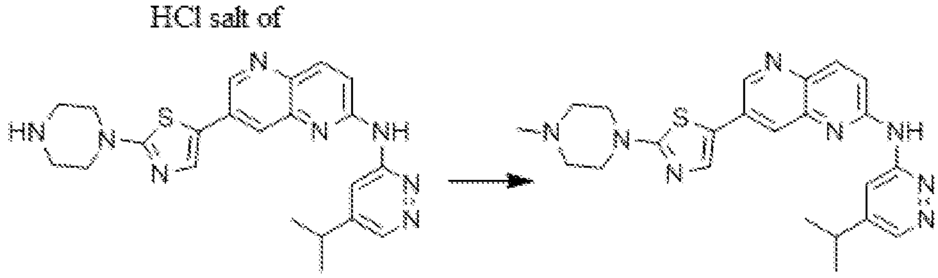


N-(5-isopropylpyridazin-3-yl)-7-(2-(4-methylpiperazin-1-yl)thiazol-5-yl)-1,5-naphthyridine-2-amine was obtained as a pale yellow solid in the same manner as in Example 0486 except that hydrochloric acid salt of N-(5-isopropylpyridazin-3-yl)-7-(2-(piperazin-1-yl)thiazol-5-yl)-1,5-naphthyridine-2-amine was used instead of the trifluoroacetic acid salt of N-(5-isopropylpyridazin-3-yl)-7-(1-(piperidin-4-ylmethyl)-1H-pyrazol-4-yl)-1,5-naphthyridine-2-amine used in Example 0486.
$^1$H-NMR(DMSO-$d_6$)δ:10.73(1H,s),9.01(1H,d,J=1.8Hz),8.87(1H,d,J=2.1Hz),8.72(1H,s),8.21(1H,d,J=8.7Hz),8.00(1H,s),7.94(1H,d,J=1.8Hz),7.69(1H,d,J=9.0Hz),3.54-3.50(4H,m),3.08-2.98(1H,m),2.47-2.41(4H,m),2.25(3H,s),1.33(6H,d,J=7.2Hz).
MSm/z(M+H):447.

<Example 0499>

<0499-1>

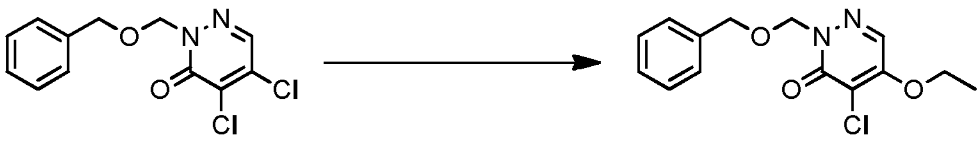

2-((Benzyloxy)methyl)-4-chloro-5-ethoxypyridazin-3(2H)-one was obtained in the same manner as in Example 0495-2 except that ethanol was used instead of the methanol used in Example 0495-2.
MSm/z(M+H):295.

<0499-2>

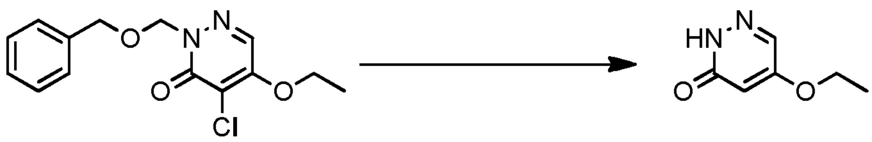

5-Ethoxypyridazin-3(2H)-one was obtained in the same manner as in Example 0495-3 except that 2-((benzyloxy)methyl)-4-chloro-5-ethoxypyridazin-3(2H)-one was used instead of the 2-((benzyloxy)methyl)-4-chloro-5-methoxypyridazin-3(2H)-one used in Example 0495-3.
MSm/z(M+H):141.

<0499-3>

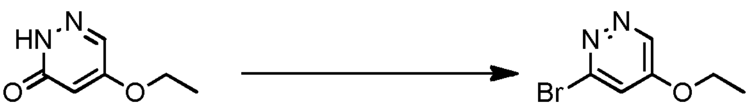

3-Bromo-5-ethoxypyridazine was obtained in the same manner as in Example 0495-4 except that 5-ethoxypyridazin-3(2H)-one was used instead of the 5-methoxypyridazin-3(2H)-one used in Example 0495-4.
MSm/z(M+H):203.

<0499-4>

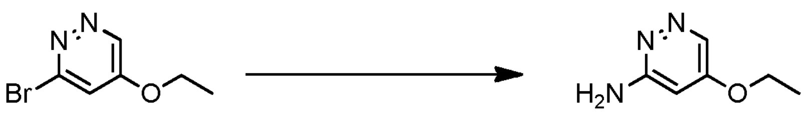

5-Ethoxypyridazine-3-amine was obtained in the same manner as in Example 0495-5 except that 3-bromo-5-ethoxypyridazine was used instead of the 3-bromo-5-methoxypyridazine used in Example 0495-5.
MSm/z(M+H):140.

<0499-5>

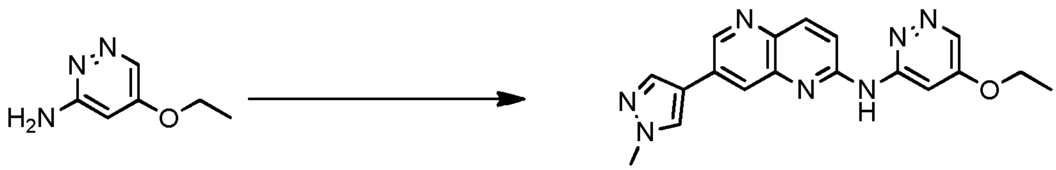

N-(5-ethoxypyridazin-3-yl)-7-(1-methyl-1H-pyrazol-4-yl)-1,5-naphthyridine-2-amine was obtained in the same manner as in Example 0489 except that 5-ethoxypyridazine-3-amine was used instead of the 4-methylpyridazine-3-amine used in Example 0489-4.
$^1$H-NMR(DMSO-$d_6$)$\delta$:10.71(1H,brs),9.04(1H,d,J=2.1Hz),8.65(1H,d,J=2.7Hz),8.50(1H,d,J=2.7Hz),8.47(1H,d),8.22(1H,d ,J=2.1Hz),8.21(1H,d,J=9.3Hz),8.19(1H,s),7.66(1H,d,J=9.3Hz),4.33(2H,q,J=6.9Hz),3.92(3H,s),1.46(3 H,t,J=6.9Hz).
MSm/z(M+H):348.

<Example 0500>

<0500-1>

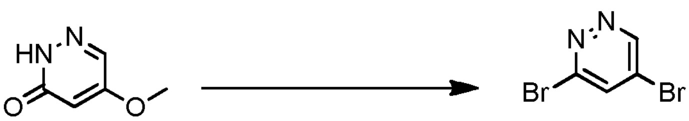

A mixture of 5-methoxypyridazin-3(2H)-one (2.35 g), phosphoryl bromide (16.0 g), and acetonitrile (90 mL) was stirred for 6 hour under reflux. The reaction mixture was added dropwise to a sodium carbonate aqueous solution under ice-cooling, and ethyl acetate was added thereto. The organic layer was collected by separation, washed with a saturated sodium chloride aqueous solution, and dried over anhydrous sodium sulfate, and the solvent was distilled off under reduced pressure. The obtained residue was purified by silica gel chromatography (ethyl acetate-hexane), thereby obtaining 3,5-dibromopyridazine (1.67 g).
MSm/z(M+H):237.

<0500-2>

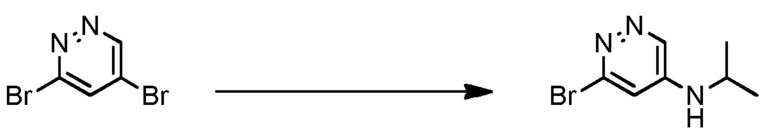

Isopropylamine (6 mL) was added to 3,5-dibromopyridazine (148 mg), followed by stirring at room temperature for 16 hours. After the solvent was distilled off under reduced pressure, water and ethyl acetate were added thereto, the organic layer was collected by separation, washed with a saturated sodium chloride aqueous solution, and dried over anhydrous sodium sulfate, and the solvent was distilled off under reduced pressure, thereby obtaining 6-bromo-N-isopropylpyridazine-4-amine (137 mg).
MSm/z(M+H):216.

<0500-3>

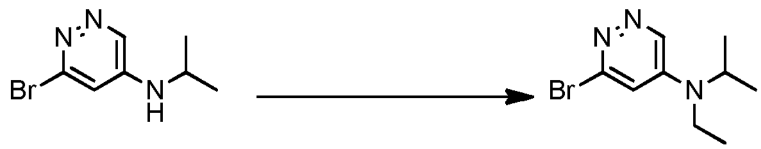

60% sodium hydride (19 mg) was added to a mixture of 6-bromo-N-isopropylpyridazine-4-amine (77 mg), iodoethane (0.050 mL), and N-methylpyrrolidone (2 mL) under ice-cooling, followed by stirring at the same temperature for 1 hour. After water and ethyl acetate were added to the reaction mixture, the organic layer was collected by separation, washed sequentially with water and a saturated sodium chloride aqueous solution, and dried over anhydrous sodium sulfate, and the solvent was distilled off under reduced pressure. The obtained residue was purified by silica gel chromatography (ethyl acetate-hexane), thereby obtaining 6-bromo-N-ethyl-N-isopropylpyridazine-4-amine (22 mg).
MSm/z(M+H):244.

<0500-4>

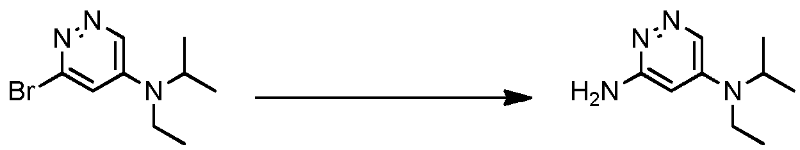

$N^5$-ethyl-$N^5$-isopropylpyridazine-3,5-diamine was obtained in the same manner as in Example 0495-5 except that 6-bromo-N-ethyl-N-isopropylpyridazine-4-amine was used instead of the 3-bromo-5-methoxypyridazine used in Example 0495-5.
MSm/z(M+H):181.

<0500-5>

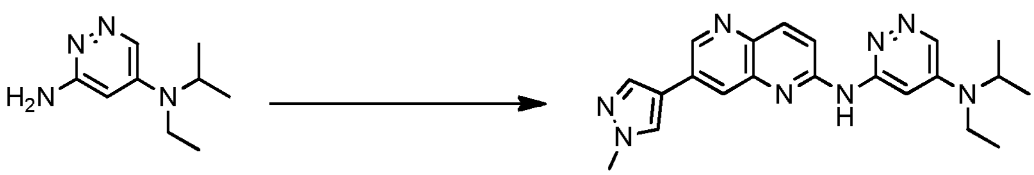

$N^5$-ethyl-$N^5$-isopropyl-$N^3$-(7-(1-methyl-1H-pyrazol-4-yl)-1,5-naphthyridin-2-yl)pyridazine-3,5-diamine was obtained in the same manner as in Example 0489-4 except that $N^5$-ethyl-$N^5$-isopropylpyridazine-3,5-diamine was used instead of the 4-methylpyridazine-3-amine used in Example 0489-4.
$^1$H-NMR($CDCl_3$)δ:10.12(1H,brs),8.86(1H,d,J=1.8Hz),8.53(1H,brs),8.48(1H,brs),8.19(1H,d,J=9.3Hz),8.01 (1H,d,J=1.8Hz),7.89(1H,s),7.78(1H,s),7.43(1H,d,J=9.3Hz),4.36-4.20(1H,m),4.02(3H,s),3.48(2H,q,J=7.2Hz),1.41(3H,t, J=7.2Hz),1.37(6H,d,J=6.6Hz).
MSm/z(M+H):389.

<Example 0501>

<0501-1>

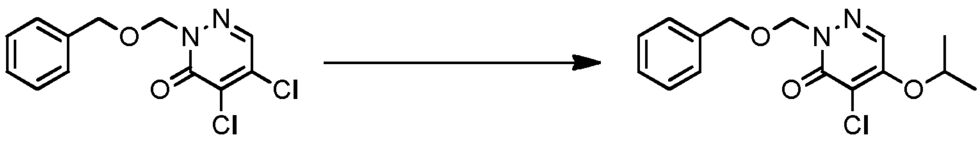

60% sodium hydride (141 mg) was added to a mixture of 2-((benzyloxy)methyl)-4,5-dichloropyridazin-3(2H)-one (506 mg), 2-propanol (0.678 mL), and tetrahydrofuran (8 mL) under ice-cooling, followed by stirring at the same temperature for 5 hours. After water and ethyl acetate were added to the reaction mixture, the organic layer was collected by separation, washed sequentially with water and a saturated sodium chloride aqueous solution, and dried over anhydrous sodium sulfate, and the solvent was distilled off under reduced pressure. The obtained residue was purified by silica gel chromatography (ethyl acetate-hexane), thereby obtaining 2-((benzyloxy)methyl)-4-chloro-5-isopropoxypyridazin-3(2H)-one (140 mg).
MSm/z(M+H):309.

<0501-2>

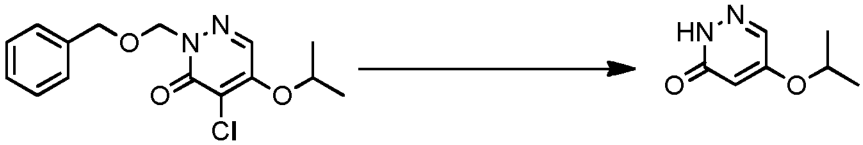

5-Isopropoxypyridazin-3(2H)-one was obtained in the same manner as in Example 0495-3 except that 2-((benzyloxy)methyl)-4-chloro-5-isopropoxypyridazin-3(2H)-one was used instead of the 2-((benzyloxy)methyl)-4-chloro-5-methoxypyridazin-3(2H)-one used in Example 0495-3.
MSm/z(M+H):155.

<0501-3>

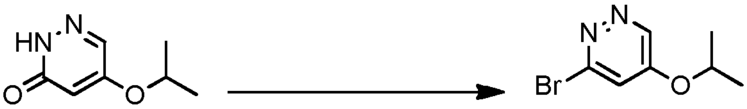

3-Bromo-5-isopropoxypyridazine was obtained in the same manner as in Example 0495-4 except that 5-isopropoxypyridazin-3(2H)-one was used instead of the 5-methoxypyridazin-3(2H)-one used in Example 0495-4.
MSm/z(M+H):217.

<0501-4>

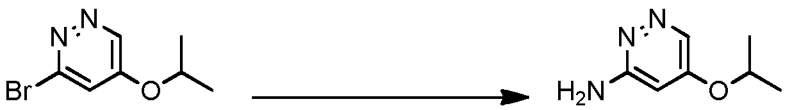

5-Isopropoxypyridazine-3-amine was obtained in the same manner as in Example 0495-5 except that 3-bromo-5-isopropoxypyridazine was used instead of the 3-bromo-5-methoxypyridazine used in Example 0495-5.
MSm/z(M+H):154.

<0501-5>

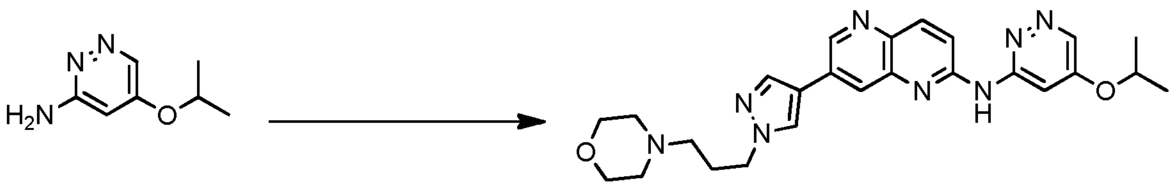

N-(5-isopropoxypyridazin-3-yl)-7-(1-(3-morpholinopropyl)-1H-pyrazol-4-yl)-1,5-naphthyridine-2-amine was obtained in the same manner as in Example 0496 except that 5-isopropoxypyridazine-3-amine was used instead of the 5-methoxypyridazine-3-amine used in Example 0496.
$^1$H-NMR($CDCl_3$/$CD_3OD$=4/1)$\delta$:9.16(1H,d,J=9.3Hz),8.96(1H,d,J=2.1Hz),8.68(1H,J=6.0Hz),8.36(1H,d,J=9.3Hz),8.13(1H,d,J=2.1Hz),7.96(1H,s),7.85(1H,s),6.75(1H,d,J=6.0Hz),4.84-4.72(1H,m),4.30(2H,t,J=6.6Hz),3.76-3.69(4H,m),2.48-2.41(4H,m),2.37(2H,t,J=6.6Hz),2.11(2H,t,J=6.6Hz),1.51(6H,d,J=6.0Hz).
MSm/z(M+H):475.

<Example 0502>

<0502-1>

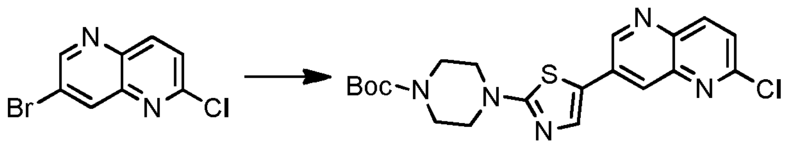

tert-Butyl 4-(5-(6-chloro-1,5-naphthyridin-3-yl)thiazol-2-yl)piperazine-1-carboxylate was obtained as a pale yellow solid in the same manner as in Example 0490-1 except that tert-butyl 4-(5-bromothiazol-2-yl)piperazine-1-carboxylate was used instead of the tert-butyl 3-(4-bromo-1H-pyrazol-1-yl)azetidine-1-carboxylate used in Example 0490-1.
MSm/z(M+H):432.

<0502-2>

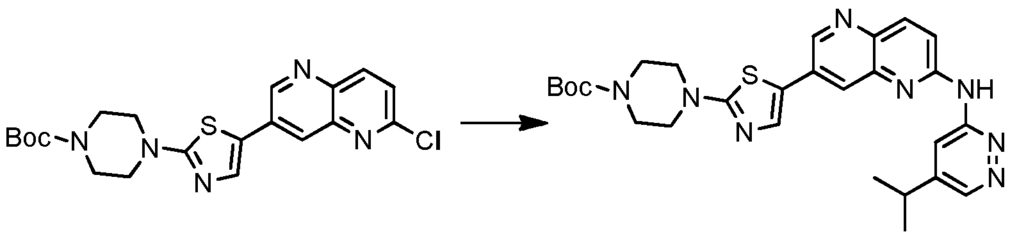

tert-Butyl 4-(5-(6-((5-isopropylpyridazin-3-yl)amino)-1,5-naphthyridin-3-yl)thiazol-2-yl)piperazine-1-carboxylate was obtained as a pale yellow solid in the same manner as in Example 0485-2 except that tert-butyl 4-(5-(6-chloro-1,5-naphthyridin-3-yl)thiazol-2-yl)piperazine-1-carboxylate was used instead of the tert-butyl 4-((4-(6-chloro-1,5-naphthyridin-3-yl)-1H-pyrazol-1-yl)methyl)piperidine-1-carboxylate used in Example 0485-2.
MSm/z(M+H):533.

<0502-3>

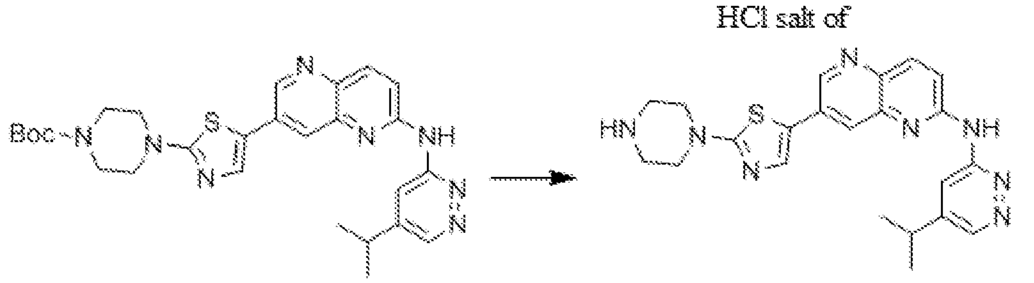


Hydrochloric acid salt of N-(5-isopropylpyridazin-3-yl)-7-(2-(piperazin-1-yl)thiazol-5-yl)-1,5-naphthyridine-2-amine was obtained as a pale yellow solid in the same manner as in Example 0488-3 except that tert-butyl 4-(5-(6-((5-isopropylpyridazin-3-yl)amino)-1,5-naphthyridin-3-yl)thiazol-2-yl)piperazine-1-carboxylate was used instead of the tert-butyl 4-(2-(4-(6-((5-isopropylpyridazin-3-yl)amino)-1,5-naphthyridin-3-yl)-1H-pyrazol-1-yl)ethyl)piperidine-1-carboxylate used in Example 0488-3.
$^1$H-NMR(DMSO-d$_6$)δ:9.92(1H,s),9.26(1H,d,J=2.7Hz),9.09(1H,d,J=2.1Hz),8.74(1H,s),8.49(1H,d,J=8.7Hz),8.39(1H,s),8.13(1H,s),7.86(1H,d,J=9.3Hz),3.88-3.80(4H,m),3.30-3.11(5H,m),1.34(6H,d,J=7.2Hz).
MSm/z(M+H):433.

<Example 0503>

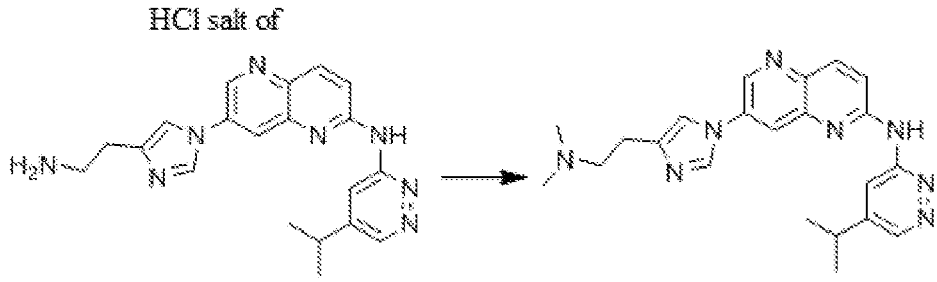


7-(4-(2-(Dimethylamino)ethyl)-1H-imidazol-1-yl)-N-(5-isopropylpyridazin-3-yl)-1,5-naphthyridine-2-amine was obtained as a white solid in the same manner as in Example 0486 except that hydrochloric acid salt of 7-(4-(2-aminoethyl)-1H-imidazol-1-yl)-N-(5-isopropylpyridazin-3-yl)-1,5-naphthyridine-2-amine was used instead of the trifluoroacetic acid salt of N-(5-isopropylpyridazin-3-yl)-7-(1-(piperidin-4-ylmethyl)-1H-pyrazol-4-yl)-1,5-naphthyridine-2-amine used in Example 0486.
$^1$H-NMR(DMSO-d$_6$)δ:10.83(1H,s),9.11(1H,d,J=2.1Hz),8.88(1H,d,J=2.1Hz),8.74(1H,d,J=1.8Hz),8.46(1H,s),8.33-8.27(2H,m),7.80-7.74(2H,m),3.09-2.97(1H,m),2.74-2.66(2H,m),2.60-2.54(2H,m),2.21(6H,s),1.33(6H,d,J=7.2Hz).
MSm/z(M+H):403.

<Example 0504>

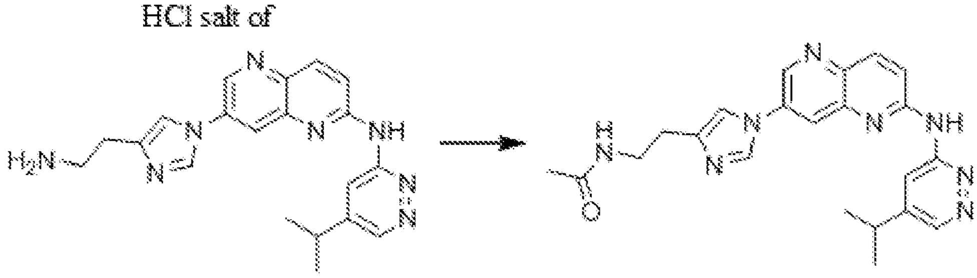

Triethylamine was added to a solution of hydrochloric acid salt of 7-(4-(2-aminoethyl)-1H-imidazol-1-yl)-N-(5-isopropylpyridazin-3-yl)-1,5-naphthyridine-2-amine (10 mg) in acetonitrile (0.8 mL) and methanol (0.2 mL), followed by adjusting to pH 8. Acetic anhydride (0.02 mL) was added thereto, followed by stirring at room temperature for 30 minutes. The solvent was distilled off under reduced pressure, and the obtained residue was purified by silica gel column chromatography (ethyl acetate-methanol, NH silica), thereby obtaining N-(2-(1-(6-((5-isopropylpyridazin-3-yl)amino)-1,5-naphthyridin-3-yl)-1H-imidazol-4-yl)ethyl)acetamide (3.7 mg) as a white solid.
$^1$H-NMR(DMSO-$d_6$)δ:10.84(1H,s),9.11(1H,d,J=2.7Hz),8.88(1H,d,J=2.1Hz),8.74(1H,d,J=1.8Hz),8.49(1H,s),8.33-8.27(2H,m),7.99-7.92(1H,m),7.82-7.74(2H,m),3.90-3.35(2H,m),3.10-2.98(1H,m),2.74-2.65(2H,m),1.82(3H,s),1.33(6H,d,J=6.6Hz).
MSm/z(M+H):417.

<Example 0505>

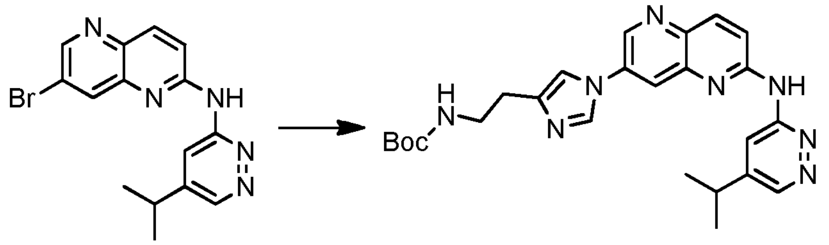

tert-Butyl (2-(1-(6-((5-isopropylpyridazin-3-yl)amino)-1,5-naphthyridin-3-yl)-1H-imidazol-4-yl)ethyl)carbamate was obtained as a white solid in the same manner as in Example 0494 except that tert-butyl (2-(1H-imidazol-4-yl)ethyl)carbamate was used instead of the 4-methyl-1H-imidazole used in Example 0494-2.
$^1$H-NMR(DMSO-$d_6$)δ:10.84(1H,s),9.11(1H,d,J=2.7Hz),8.88(1H,d,J=2.1Hz),8.74(1H,d,J=1.8Hz),8.49(1H,s),8.33-8.27(2H,m),7.81-7.72(2H,m),6.96-6.88(1H,m),3.30-3.23(2H,m),3.10-2.98(1H,m),2. 73-2.65(2H,m),1.39(9H,s),1.33(6H,d,J=7.2Hz).
MSm/z(M+H):475.

<Example 0506>

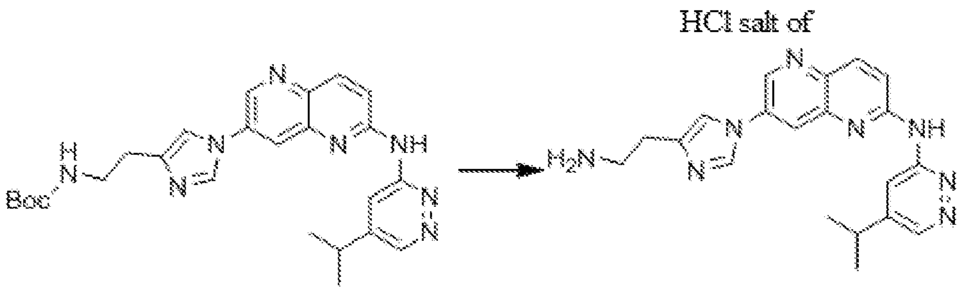


Hydrochloric acid salt of 7-(4-(2-aminoethyl)-1H-imidazol-1-yl)-N-(5-isopropylpyridazin-3-yl)-1,5-naphthyridine-2-amine was obtained as a white solid in the same manner as in Example 0488 except that tert-butyl (2-(1-(6-((5-isopropylpyridazin-3-yl)amino)-1,5-naphthyridin-3-yl)-1H-imidazol-4-yl)ethyl)carbamate was used instead of the tert-butyl 4-(2-(4-(6-((5-isopropylpyridazin-3-yl)amino)-1,5-naphthyridin-3-yl)-1H-pyrazol-1-yl)ethyl)piperidine-1-carboxylate used in Example 0488-3.
$^1$H-NMR(DMSO-$d_6$)δ:9.95(1H,s),9.33(1H,d,J=2.7Hz),9.15-9.09(2H,m),8.61-8.55(2H,m),8.51-8.47(1H,m),8.40(1H,s),8.00(1H,d,J=9.0Hz),3.33-3.13(5H,m),1.34(6H,d,J=7.2Hz).
MSm/z(M+H):375.

<Example 0507>

<0507-1>

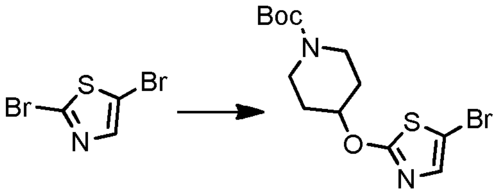

60% sodium hydride (200 mg) was added to a solution of 2,5-dibromothiazole (0.97 g) in N,N-dimethylformamide (10 mL), followed by stirring at room temperature for 5 minutes. tert-Butyl 4-hydroxypiperidine-1-carboxylate (1.01 g) was added thereto, followed by stirring at 70°C for 1 hour. After the reaction mixture was cooled to room temperature, ethyl acetate and water were added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining tert-butyl 4-((5-bromothiazol-2-yl)oxy)piperidine-1-carboxylate (1.16 g) as a white solid.
MSm/z(M+H):365.

<0507-2>

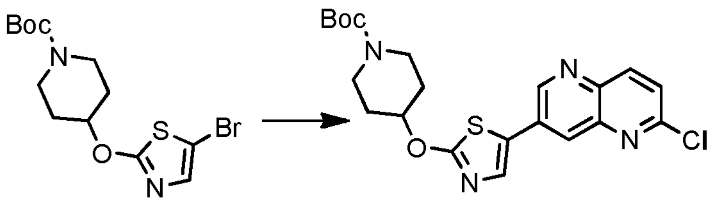

tert-Butyl 4-((5-(6-chloro-1,5-naphthyridin-3-yl)thiazol-2-yl)oxy)piperidine-1-carboxylate was obtained as a pale yellow solid in the same manner as in Example 0490-1 except that tert-butyl 4-((5-bromothiazol-2-yl)oxy)piperidine-1-carboxylate was used instead of the tert-butyl 3-(4-bromo-1H-pyrazol-1-yl)azetidine-1-carboxylate used in Example 0490-1.
MSm/z(M+H):447.

<0507-3>

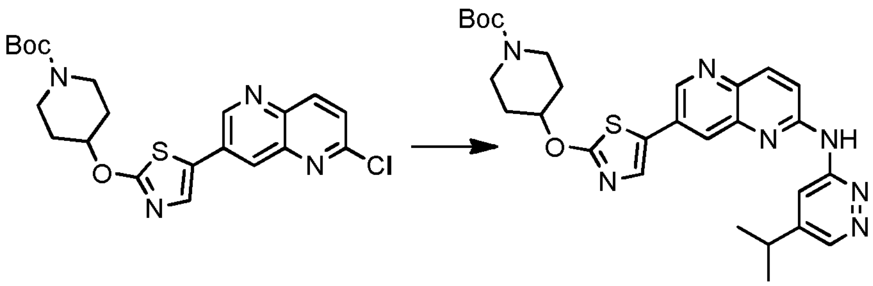

tert-Butyl 4-((5-(6-((5-isopropylpyridazin-3-yl)amino)-1,5-naphthyridin-3-yl)thiazol-2-yl)oxy)piperidine-1-carboxylate was obtained as a pale yellow solid in the same manner as in Example 0485-2 except that tert-butyl 4-((5-(6-chloro-1,5-naphthyridin-3-yl)thiazol-2-yl)oxy)piperidine-1-carboxylate was used instead of the tert-butyl 4-((4-(6-chloro-1,5-naphthyridin-3-yl)-1H-pyrazol-1-yl)methyl)piperidine-1-carboxylate used in Example 0485-2.
MSm/z(M+H):548.

<0507-4>

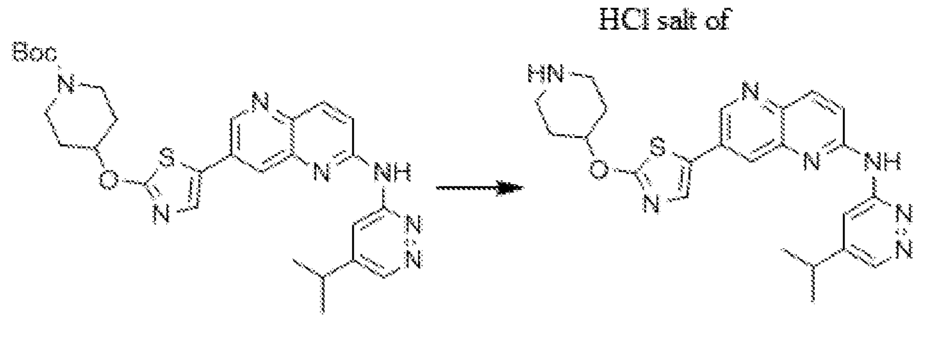


Hydrochloric acid salt of N-(5-isopropylpyridazin-3-yl)-7-(2-(piperidin-4-yloxy)thiazol-5-yl)-1,5-naphthyridine-2-amine was obtained as a pale yellow solid in the same manner as in Example 0488-3 except that tert-butyl 4-((5-(6-((5-isopropylpyridazin-3-yl)amino)-1,5-naphthyridin-3-yl)thiazol-2-yl)oxy)piperidine-1-carboxylate was used instead of the tert-butyl 4-(2-(4-(6-((5-isopropylpyridazin-3-yl)amino)-1,5-naphthyridin-3-yl)-1H-pyrazol-1-yl)ethyl)piperidine-1-carboxylate used in Example 0488-3.
MSm/z(M+H):448.

<0507-5>

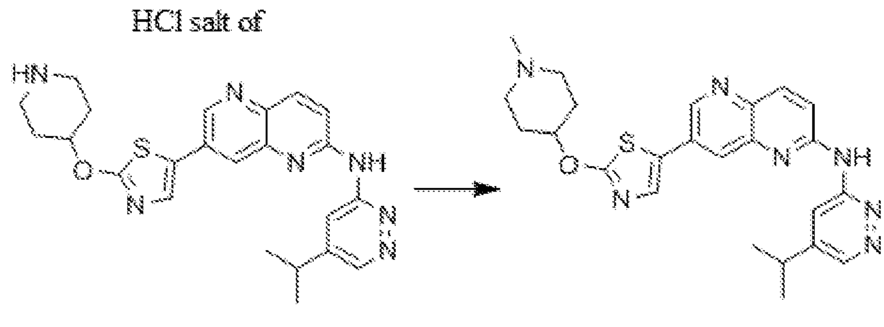


N-(5-isopropylpyridazin-3-yl)-7-(2-((1-methylpiperidin-4-yl)oxy)thiazol-5-yl)-1,5-naphthyridine-2-amine was obtained as a pale yellow solid in the same manner as in Example 0486 except that hydrochloric acid salt of N-(5-isopropylpyridazin-3-yl)-7-(2-(piperidin-4-yloxy)thiazol-5-yl)-1,5-naphthyridine-2-amine was used instead of the trifluoroacetic acid salt of N-(5-isopropylpyridazin-3-yl)-7-(1-(piperidin-4-ylmethyl)-1H-pyrazol-4-yl)-1,5-naphthyridine-2-amine used in Example 0486.
$^1$H-NMR(DMSO-$d_6$)δ:10.78(1H,s),9.02(1H,d,J=1.8Hz),8.88(1H,d,J=2.1Hz),8.72(1H,s),8.24(1H,d,J=9.3Hz),8.10(1H,s),7.99(1H,s),7.74(1H,d,J=9.3Hz),5.09-5.03(1H,m),3.08-3.00(1H,m),2.80-2.81(2H,m),2.62-2.56(2H,m),2.34(3H,s),2.10-2.00(2H,m),1.99-1.85(2H,m),1.33(6H,d,J=7.2Hz).
MSm/z(M+H):462.

<Example 0508>

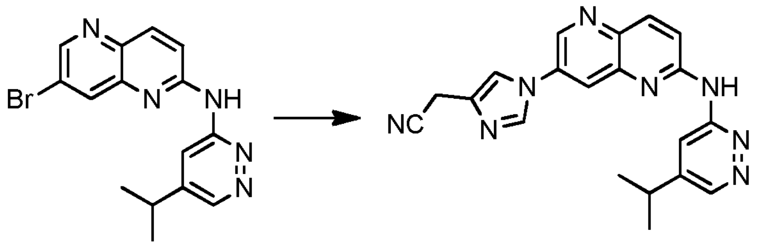

2-(1-(6-((5-Isopropylpyridazin-3-yl)amino)-1,5-naphthyridin-3-yl)-1H-imidazol-4-yl)acetonitrile was obtained as a white solid in the same manner as in Example 0494-2 except that 2-(1H-imidazol-4-yl)acetonitrile was used instead of the 4-methyl-1H-imidazole used in Example 0494-2.
$^1$H-NMR(DMSO-$d_6$)δ:10.87(1H,s),9.12(1H,d,J=2.7Hz),8.89(1H,d,J=2.1Hz),8.75(1H,d,J=2.1Hz),8.38(1H,d,J=1.8Hz),8.31(1H,d,J=9.3Hz),7.99(1H,s),7.79(1H,d,J=9.3Hz),7.06(1H,s),3.83(2H,s),3.09-2.99(1H,m),1.33(6H,d,J=7.2Hz).
MSm/z(M+H):371.

<Example 0509>

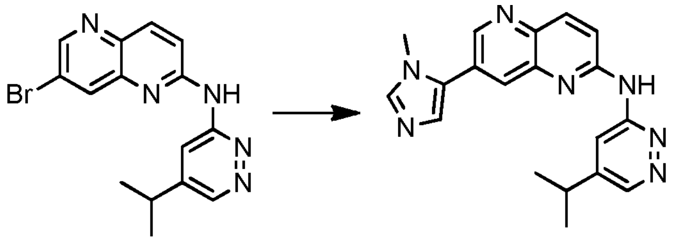

N-(5-isopropylpyridazin-3-yl)-7-(1-methyl-1H-imidazol-5-yl)-1,5-naphthyridine-2-amine was obtained as a white solid in the same manner as in Example 0490-1 except that 7-bromo-N-(5-isopropylpyridazin-3-yl)-1,5-naphthyridine-2-amine was used instead of the 7-bromo-2-chloro-1,5-naphthyridine used in Example 0490-1, and 5-bromo-1-methyl-1H-imidazole was used instead of the tert-butyl 3-(4-bromo-1H-pyrazol-1-yl)azetidine-1-carboxylate used in Example 0490-1.
$^1$H-NMR(DMSO-$d_6$)δ:10.79(1H,s),8.91(1H,d,J=1.8Hz),8.86(1H,s),8.73(1H,s),8.29(1H,d,J=9.3Hz),8.22(1H,d,J=1.2Hz),7.86(1H,s),7.80(1H,d,J=9.3Hz),7.39(1H,s),3.84(3H,s),3.08-2.97(1H,m),1.31(6H,d,J=7.2Hz).
MSm/z(M+H):346.

<Example 0510>

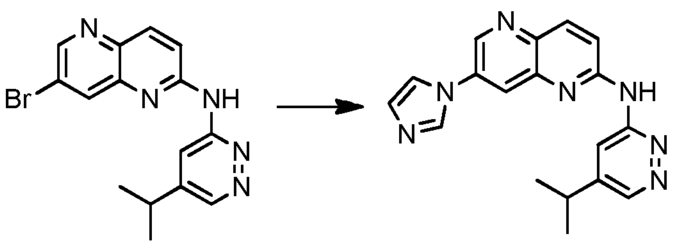

7-(IH-imidazol-1-yl)-N-(5-isopropylpyridazin-3-yl)-1,5-naphthyridine-2-amine was obtained as a white solid in the same manner as in Example 0494-2 except that 1H-imidazole was used instead of the 4-methyl-1H-imidazole used in Example 0494-2.
$^1$H-NMR(DMSO-$d_6$)δ:10.85(1H,s),9.14(1H,d,J=2.7Hz),8.89(1H,d,J=2.1Hz),8.75(1H,d,J=2.1Hz),8.57(1H,s),8.38(1H,d,J=2.1Hz),8.31(1H,d,J=8.7Hz),8.06(1H,s),7.78(1H,d,J=9.0Hz),7.21(1H,s),3.09-2.99(1H,m),1.33(6H,d,J=7.5Hz).

MSm/z(M+H):332.

<Example 0511>

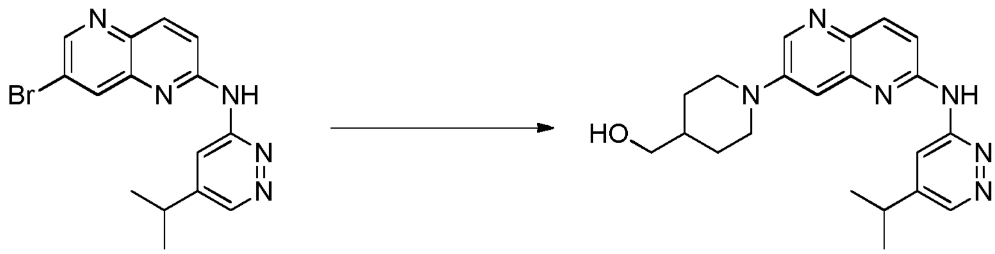

(1-(6-((5-Isopropylpyridazin-3-yl)amino)-1,5-naphthyridin-3-yl)piperidin-4-yl)methanol was obtained as a yellow solid in the same manner as in Example 0846 except that 4-piperidinemethanol was used instead of the pyrrolidine used in Example 0846.
$^1$H-NMR($CDCl_3$)δ:8.80-8.75(1H,m),8.75-8.71(1H,m),8.66-8.63(1H,m),8.53-8.47(1H,m),8.13(1H,d,J=8.6Hz),7.33-7.30(1H,m),7.27-7.25(1H,m),3.98-3.91(2H,m),3.62-3.55(2H,m),3.06-2.94(1H,m),3.00-2.89(2H,m),1.98-1.91(2H,m),1.82-1.72(1H,m),1.57-1.38(2H,m),1.51-1.40(1H,m),1.39(6H,d,J=7.3Hz).
MSm/z(M+H):379.

<Example 0512>

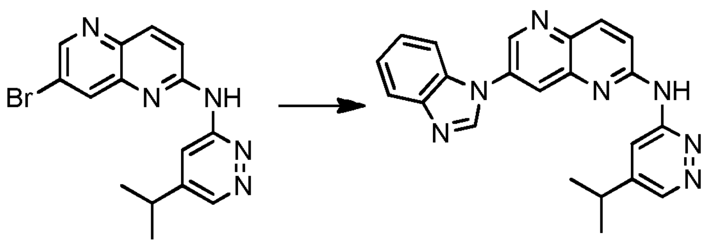

7-(1H-benzo[d]imidazol-1-yl)-N-(5-isopropylpyridazin-3-yl)-1,5-naphthyridine-2-amine was obtained as a white solid in the same manner as in Example 0494-2 except that 1H- benzo[d]imidazole was used instead of the 4-methyl-1H-imidazole used in Example 0494-2.
$^1$H-NMR(DMSO-$d_6$)δ:10.91(1H,s),9.10(1H,d,J=2.7Hz),8.80(1H,d,J=2.1Hz),8.79-8.75(2H,m),8.48(1H,d,J=9.0Hz),8.39(1H,d,J=9.0Hz),7.88-7.81(2H,m),7.78-7.72(1H,m),7.41-7.35(2H,m),3.07-2.97(1H,m),1.30(6H,d,J=7.5Hz).
MSm/z(M+H):382.

<Example 0513>

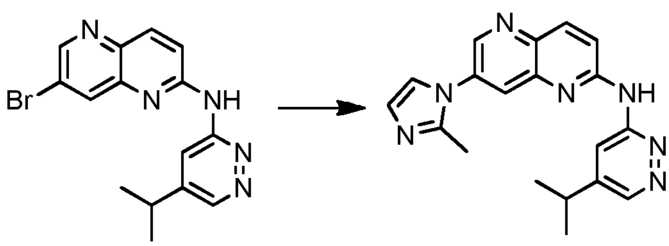

N-(5-isopropylpyridazin-3-yl)-7-(2-methyl-1H-imidazol-1-yl)-1,5-naphthyridine-2-amine was obtained as a white solid in the same manner as in Example 0494-2 except that 2-methyl-1H-imidazole was used instead of the 4-methyl-1H-imidazole used in Example 0494-2.
1H-NMR(DMSO-$d_6$)δ:10.90(1H,s),8.88-8.83(2H,m),8.75(1H,d,J=1.8Hz),8.35(1H,d,J=9.6Hz),8.29(1H,d,J=2.7Hz),7.84(1H,d,J=9.3Hz),7.54(1 H,s),7.02(1H,s),3.08-2.95(1H,m),2.39(3H,s),1.31(6H,d,J=6.6Hz).
MSm/z(M+H):346.

<Example 0514>

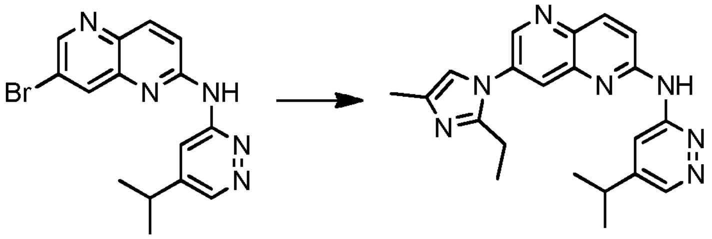

7-(2-Ethyl-4-methyl-1H-imidazol-1-yl)-N-(5-isopropylpyridazin-3-yl)-1,5-naphthyridine-2-amine was obtained as a white solid in the same manner as in Example 0494-2 except that 2-ethyl-4-methyl-1H-imidazole was used instead of the 4-methyl-1H-imidazole used in Example 0494-2.
1H-NMR(DMSO-$d_6$)δ:10.89 (1H,s),8.87(1H,d,J=1.8Hz),8.80-8.75(2H,m),8.34(1H,d,J=9.3Hz),8.21(1H,d,J=2.1Hz),7.82(1H,d,J=9.0Hz),7.19(1H,s),3.07-2.96(1H,m),2.67(2H,q,J=7.2Hz),2.16(3H,s),1.30(6H,d,J=6.6Hz),1.18(3H,t,J=7.2Hz).
MSm/z(M+H):374.

<Example 0515>

<0515-1>

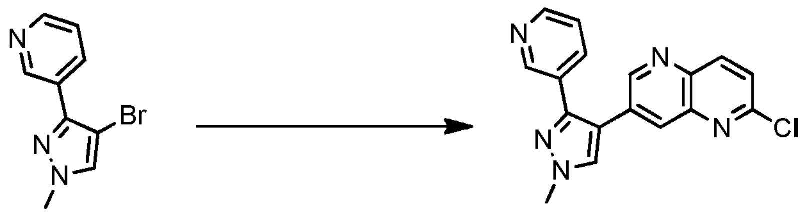

2-Chloro-7-(1-methyl-3-(pyridin-3-yl)-1H-pyrazol-4-yl)-1,5-naphthyridine was obtained in the same manner as in Example 0478-3 except that 3-(4-bromo-1-methyl-1H-pyrazol-3-yl)pyridine was used instead of the 1-(4-(4-iodo-1H-pyrazol-1-yl)piperidin-1-yl)ethanone used in Example 0478-3.
MSm/z(M+H):322.

<0515-2>

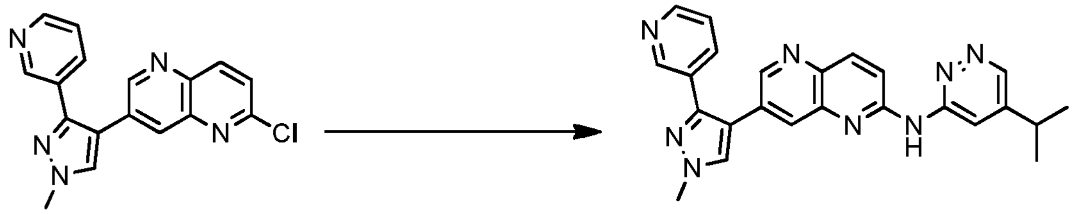

N-(5-isopropylpyridazin-3-yl)-7-(1-methyl-3-(pyridin-3-yl)-1H-pyrazol-4-yl)-1,5-naphthyridine-2-amine was obtained in the same manner as in Example 0471-2 except that 2-chloro-7-(1-methyl-3-(pyridin-3-yl)-1H-pyrazol-4-yl)-1,5-naphthyridine was used instead of the 2-chloro-7-(1-(2-methoxyethyl)-1H-pyrazol-4-yl)-1,5-naphthyridine used in Example 0471-2.
$^1$H-NMR($CDCl_3$)δ:10.14(1H,brs),8.91(1H,d,J=1.4Hz),8.80(1H,s),8.79(1H,s),8.67(1H,d,J=2.7Hz),8.59-8.52(2H,m),8.24(1H,d,J=9.3Hz),7.86-7.79(2H,m),7.72(1H,s),7.27(1H,d,J=9.3Hz),4.08(3H,s),3.04-2.92(1H,m),1.34(6H,d,J=6.6Hz).
MSm/z(M+H):423.

<Example 0516>

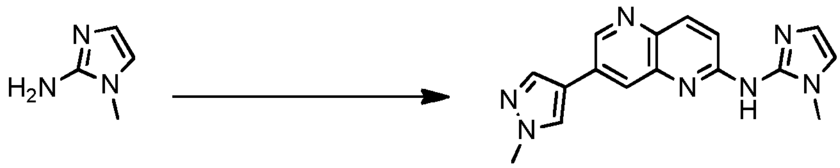

N-(1-methyl-1H-imidazol-2-yl)-7-(1-methyl-1H-pyrazol-4-yl)-1,5-naphthyridine-2-amine was obtained in the same manner as in Example 0489-4 except that 1-methyl-1H-imidazole-2-amine was used instead of the 4-methylpyridazine-3-amine used in Example 0489-4.
$^1$H-NMR($CDCl_3$)δ:8.60(1H,brs),7.89(1H,s),7.78(1H,s),7.71(1H,d,J=9.3Hz),7.61(1H,brs),7.00(1H,brs),6.94(1H,brs),6.74(1H,brs),4.00(3H,s),3.68(3H,s).
MSm/z(M+H):306.

<Example 0517>

<0517-1>

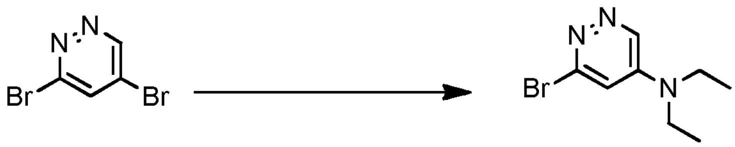

A mixture of 3,5-dibromopyridazine (150 mg), diethylamine (2 mL), and tetrahydrofuran (1 mL) was stirred at room temperature for 6 hours. The solvent was distilled off under reduced pressure, and the obtained residue was purified by silica gel chromatography (ethyl acetate-hexane), thereby obtaining 6-bromo-N,N-diethylpyridazine-4-amine (60 mg).
MSm/z(M+H):230.

<0517-2>

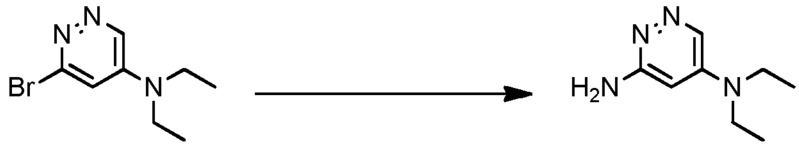

$N^5$,$N^5$-diethylpyridazine-3,5-diamine was obtained in the same manner as in Example 0495-5 except that 6-bromo-N,N-diethylpyridazine-4-amine was used instead of the 3-bromo-5-methoxypyridazine used in Example 0495-5.
MSm/z(M+H):167.

<0517-3>

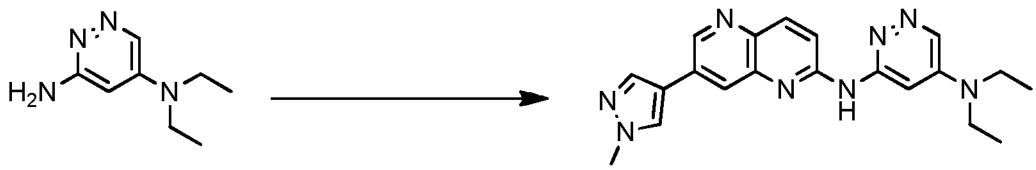

$N^5$,$N^5$-diethyl-$N^3$-(7-(1-methyl-1H-pyrazol-4-yl)-1,5-naphthyridin-2-yl)pyridazine-3,5-diamine was obtained in the same manner as in Example 0489-4 except that $N^5$,$N^5$-diethylpyridazine-3,5-diamine was used instead of the 4-methylpyridazine-3-amine used in Example 0489-4.
$^1$H-NMR($CDCl_3$)δ:9.66(1H,brs),8.86(1H,d,J=1.8Hz),8.46(1H,brs),8.37(1H,brs),8.18(1H,d,J=9.3Hz),8.01( 1H,d,J=1.8Hz),7.90(1H,s),7.79(1H,s),7.63(1H,d,J=9.3Hz),4.02(3H,s),3.54(4H,q,J=6.6Hz),1.36(6H,t,J =6.6Hz).
MSm/z(M+H):375.

<Example 0518>

<0518-1>

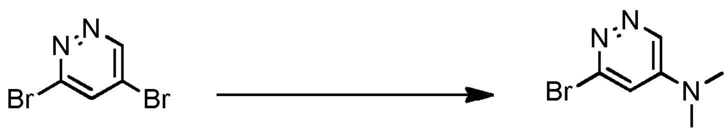

6-Bromo-N,N-dimethylpyridazine-4-amine was obtained in the same manner as in Example 0517-1 except that dimethylamine was used instead of the diethylamine used in Example 0517-1.
MSm/z(M+H):202.

<0518-2>

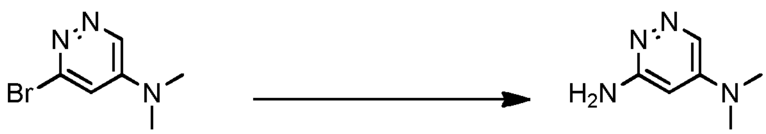

$N^5$,$N^5$-dimethylpyridazine-3,5-diamine was obtained in the same manner as in Example 0495-5 except that 6-bromo-N,N-dimethylpyridazine-4-amine was used instead of the 3-bromo-5-methoxypyridazine used in Example 0495-5.
MSm/z(M+H):139.

<0518-3>

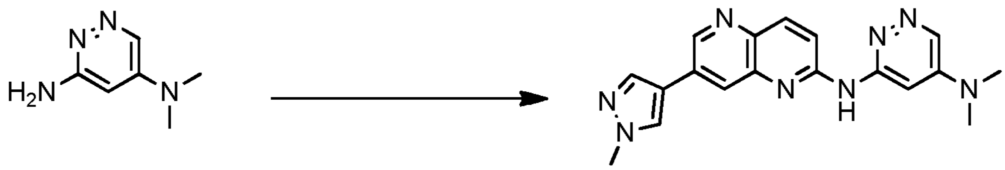

$N^5$,$N^5$-dimethyl-$N^3$-(7-(1-methyl-1H-pyrazol-4-yl)-1,5-naphthyridin-2-yl)pyridazine-3,5-diamine was obtained in the same manner as in Example 0489-4 except that $N^5$,$N^5$-dimethylpyridazine-3,5-diamine was used instead of the 4-methylpyridazine-3-amine used in Example 0489-4.
$^1$H-NMR($CDCl_3$/$CD_3OD$=4/1)δ:8.84(1H,d,J=2.1Hz),8.38(1H,d,J=2.1Hz),8.16(1H,d,J=9.3Hz),8.06(1H,d,J =2.1Hz),7.92(1H,s),7.90(1H,s),7.89(1H,s),7.42(1H,d,J=9.3Hz),4.02(3H,s),3.20(6H,s).
MSm/z(M+H):347.

<Example 0519>

<0519-1>

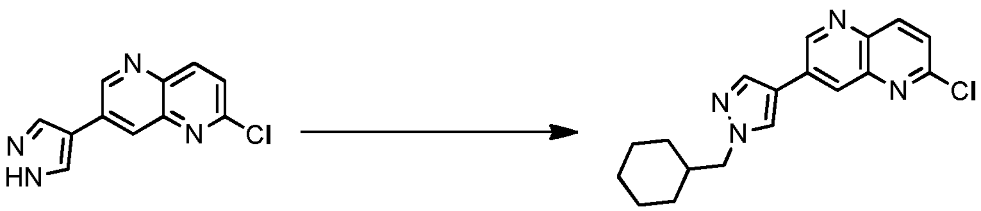

A mixture of 2-chloro-7-(1H-pyrazol-4-yl)-1,5-naphthyridine (20 mg), (bromomethyl)cyclohexane (0.024 mL), cesium carbonate (56 mg), and N,N-dimethylformamide was stirred for 2 hours under heating to reflux. After water and ethyl acetate were added to the reaction mixture, the organic layer was collected by separation, washed sequentially with water and a saturated sodium aqueous solution, and dried over anhydrous sodium sulfate, and the solvent was distilled off under reduced pressure, thereby obtaining 2-chloro-7-(1-(cyclohexylmethyl)-1H-pyrazol-4-yl)-1,5-naphthyridine (31 mg).
MSm/z(M+H):327.

<0519-2>

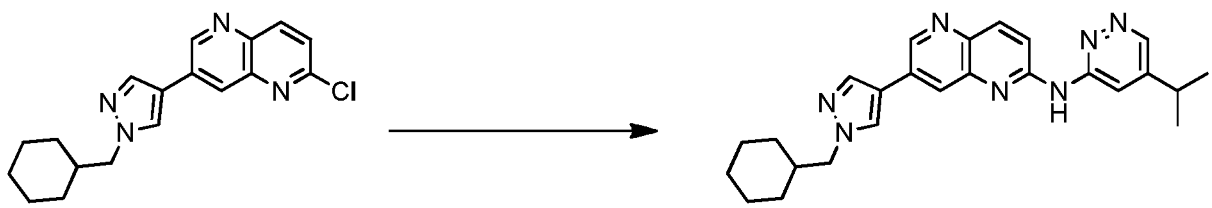

7-(1-(Cyclohexylmethyl)-1H-pyrazol-4-yl)-N-(5-isopropylpyridazin-3-yl)-1,5-naphthyridine-2-amine was obtained in the same manner as in Example 0471-2 except that 2-chloro-7-(1-(cyclohexylmethyl)-1H-pyrazol-4-yl)-1,5-naphthyridine was used instead of the 2-chloro-7-(1-(2-methoxyethyl)-1H-pyrazol-4-yl)-1,5-naphthyridine used in Example 0471-2.
$^1$H-NMR($CDCl_3$)δ:8.93(1H,d,J=2.1Hz),8.82(2H,s),8.24(1H,d,J=9.0Hz),8.10(1H,d,J=2.1Hz),7.96(1H,s),7.80(1H,s),7.49(1H,d,J=9.0Hz),4.04(2H,d,J=7.2Hz),3.16-2.95(1H,m),2.05-1.88(1H,m),1.82-1.63(4H,m),1.42(6H,d,J=7.5

Hz),1.36-1.15(4H,m),1.11-0.93(2H,m).
MSm/z(M+H):428.

<Example 0520>

<0520-1>

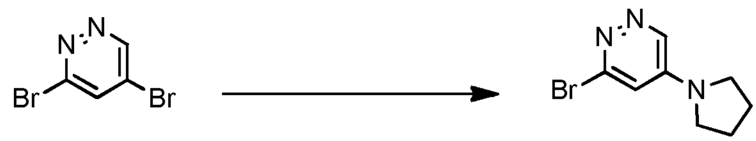

3-Bromo-5-(pyrrolidin-1-yl)pyridazine was obtained in the same manner as in Example 0517-1 except that pyrrolidine was used instead of the diethylamine used in Example 0517-1.
MSm/z(M+H):228.

<0520-2>

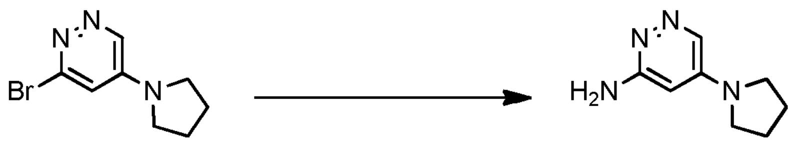

5-(Pyrrolidin-1-yl)pyridazine-3-amine was obtained in the same manner as in Example 0495-5 except that 3-bromo-5-(pyrrolidin-1-yl)pyridazine was used instead of the 3-bromo-5-methoxypyridazine used in Example 0495-5.
MSm/z(M+H):165.

<0520-3>

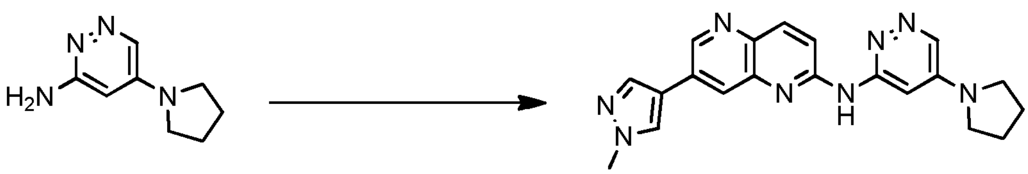

7-(1-Methyl-1H-pyrazol-4-yl)-N-(5-(pyrrolidin-1-yl)pyridazin-3-yl)-1,5-naphthyridine-2-amine was obtained in the same manner as in Example 0489-4 except that 5-(pyrrolidin-1-yl)pyridazine-3-amine was used instead of the 4-methylpyridazine-3-amine used in Example 0489-4.
$^1$H-NMR(DMSO-$d_6$)$\delta$:10.26(1H,brs),8.99(1H,d,J=2.1Hz),8.45(1H,s),8.35(1H,d,J=2.7Hz),8.18(1H,s),8.15(1H,d,J=9.3Hz ),8.14(1H,d,J=2.7Hz),7.90(1H,d,J=2.1Hz),7.65(1H,d,J=9.3Hz),3.92(3H,s),3.49-3.38(4H,m),2.06-1.98(4H,m).
MSm/z(M+H):373.

<Example 0521>

<0521-1>

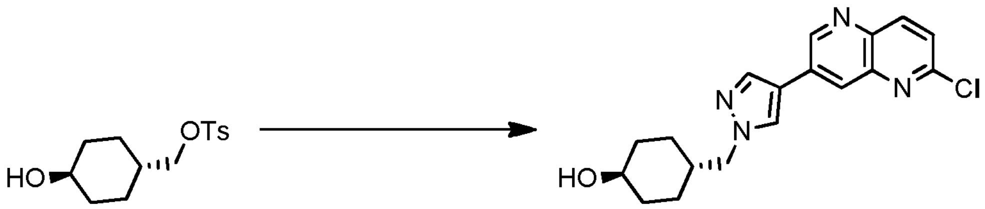

(1r,4r)-4-((4-(6-Chloro-1,5-naphthyridin-3-yl)-1H-pyrazol-1-yl)methyl)cyclohexanol was obtained in the same manner as in Example 0519-1 except that ((1r,4r)-4-hydroxycyclohexyl)methyl 4-methylbenzenesulfonate was used instead of the (bromomethyl)cyclohexane used in Example 0519-1.
MSm/z(M+H):343.

<0521-2>

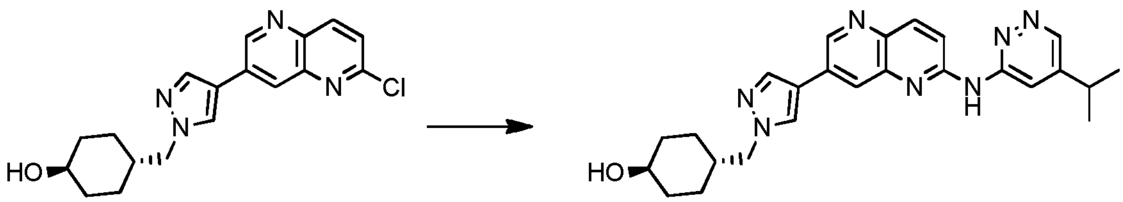

(1r,4r)-4-((4-(6-((5-Isopropylpyridazin-3-yl)amino)-1,5-naphthyridin-3-yl)-1H-pyrazol-1-yl)methyl)cyclohexanol was obtained in the same manner as in Example 0471-2 except that (1r,4r)-4-((4-(6-chloro-1,5-naphthyridin-3-yl)-1H-pyrazol-1-yl)methyl)cyclohexanol was used instead of the 2-chloro-7-(1-(2-methoxyethyl)-1H-pyrazol-4-yl)-1,5-naphthyridine used in Example 0471-2.
$^1$H-NMR($CDCl_3$)δ:8.93(1H,d,J=2.1Hz),8.85(1H,brs),8.81(1H,brs),8.25(1H,d,J=9.3Hz),8.10(1H,brs),7.97( 1H,s),7.81(1H,s),7.54(1H,d,J=9.3Hz),4.06(2H,d,J=7.2Hz),3.68(1H,m),3.54(1H,m),3.13-2.99(1H,m),2.08-1.54(5H,m),1.42(6H,d,J=6.6Hz),1.38-1.04(4H,m).
MSm/z(M+H):444.

<Example 0522>

<0522-1>

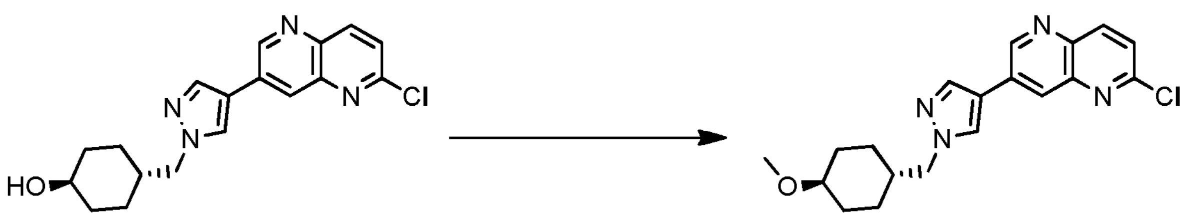

60% sodium hydride (6 mg) was added to a mixture of (1r,4r)-4-((4-(6-chloro-1,5-naphthyridin-3-yl)-1H-pyrazol-1-yl)methyl)cyclohexanol (10 mg), iodomethane (0.008 mL), andN,N-dimethylformamide (1 mL) under ice-cooling, followed by stirring at the same temperature for 2 hours. After water and ethyl acetate were added to the reaction mixture, the organic layer was collected by separation, washed sequentially with water and a saturated sodium aqueous solution, and dried over anhydrous sodium sulfate, and the solvent was distilled off under reduced pressure, thereby obtaining 2-chloro-7-(1-(((1r,4r)-4-methoxycyclohexyl)methyl)-1H-pyrazol-4-yl)-1,5-naphthyridine (17 mg).
MSm/z(M+H):357.

<0522-2>

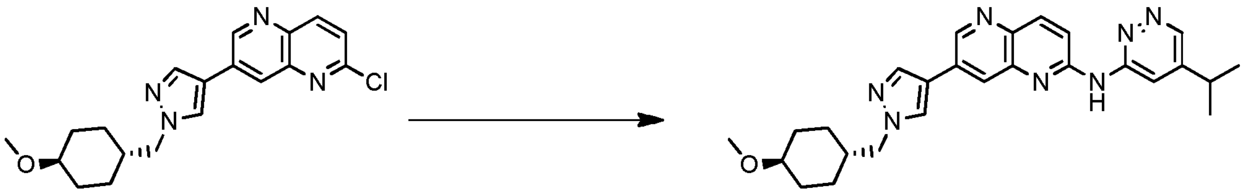

N-(5-isopropylpyridazin-3-yl)-7-(1-(((1r,4r)-4-methoxycyclohexyl)methyl)-1H-pyrazol-4-yl)-1,5-naphthyridine-2-amine was obtained in the same manner as in Example 0471-2 except that 2-chloro-7-(1-(((1r,4r)-4-methoxycyclohexyl)methyl)-1H-pyrazol-4-yl)-1,5-naphthyridine was used instead of the 2-chloro-7-(1-(2-methoxyethyl)-1H-pyrazol-4-yl)-1,5-naphthyridine used in Example 0471-2.
$^1$H-NMR($CDCl_3$)$\delta$:8.93(1H,d,J=2.1Hz),8.81(2H,brs),8.24(1H,d,J=9.3Hz),8.11(1H,d,J=2.1Hz),7.97(1H,s,),7.81(1H,s),7.48(1H,d,J=9.3Hz),4.78(2H,brs),4.06(2H,d,J=7.2Hz),3.35(3H,s),3.19-3.00(1H,m),2.15-2.07(2H,m),1.82-1.73(2H,m),1.42(6H,d,J=6.6Hz),1.35-1.03(5H,m).
MSm/z(M+H):458.

<Example 0523>

<0523-1>

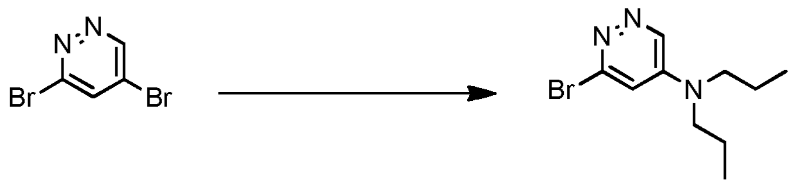

Dipropylamine (0.5 mL) was added to 3,5-dibromopyridazine (50 mg), followed by stirring at 80°C for 3 hours. The reaction mixture was purified by silica gel chromatography (methanol-ethyl acetate-hexane), thereby obtaining 6-bromo-N,N-dipropylpyridazine-4-amine (42 mg).
MSm/z(M+H):258.

<0523-2>

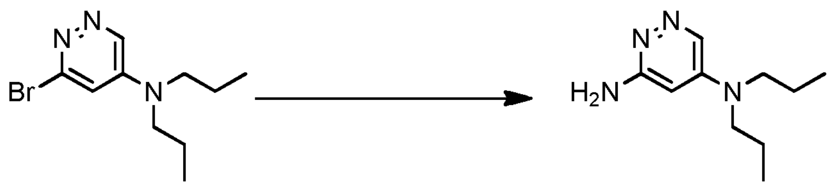

$N^5$,$N^5$-dipropylpyridazine-3,5-diamine was obtained in the same manner as in Example 0495-5 except that 6-bromo-N,N-dipropylpyridazine-4-amine was used instead of the 3-bromo-5-methoxypyridazine used in Example 0495-5.
MSm/z(M+H):195.

<0523-3>

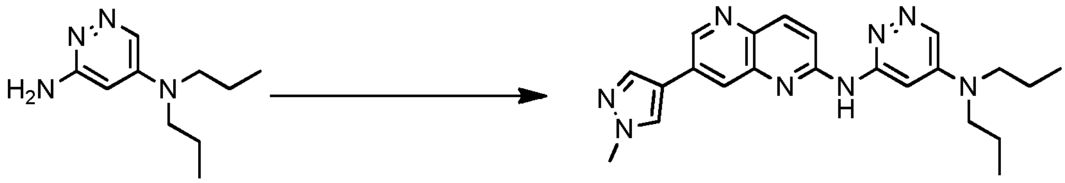

$N^3$-(7-(1-methyl-1H-pyrazol-4-yl)-1,5-naphthyridin-2-yl)-$N^5N^5$-dipropylpyridazine-3,5-diamine was obtained in the same manner as in Example 0489-4 except that $N^5$,$N^5$-dipropylpyridazine-3,5-diamine was used instead of the 4-methylpyridazine-3-amine used in Example 0489-4.
$^1$H-NMR($CDCl_3$)δ:8.87(1H,brs),8.43(1H,brs),8.39(1H,brs),8.18(1H,d,J=9.3Hz),8.03(1H,d,J=brs),7.89(1H ,s),7.78(1H,s),7.54(1H,d,J=9.3Hz),4.02(3H,s),3.43(4H,t,J=7.8Hz),1.88-1.72(4H,m),1.26(6H,t,J=7.5Hz).
MSm/z(M+H):403.

<Example 0524>

<0524-1>

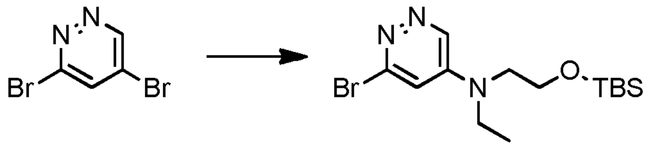

6-Bromo-N-(2-((tert-butyldimethylsilyl)oxy)ethyl)-N-ethylpyridazine-4-amine was obtained in the same manner as in Example 0517-1 except that N-(2-((tert-butyldimethylsilyl)oxy)ethyl)ethan-1-amine was used instead of the diethylamine used in Example 0517-1.
MSm/z(M+H):360.

<0524-2>

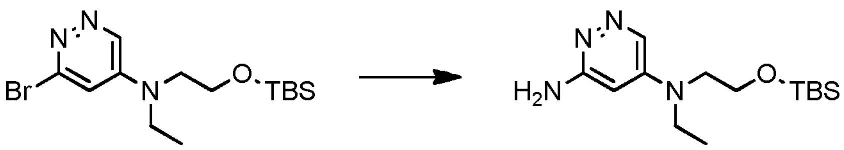

$N^5$-(2-((tert-butyldimethylsilyl)oxy)ethyl)-$N^5$-ethylpyridazine-3,5-diamine was obtained in the same manner as in Example 0495-5 except that 6-bromo-N-(2-((tert-butyldimethylsilyl)oxy)ethyl)-N-ethylpyridazine-4-amine was used instead of the 3-bromo-5-methoxypyridazine used in Example 0495-5.
MSm/z(M+H):297.

<0524-3>

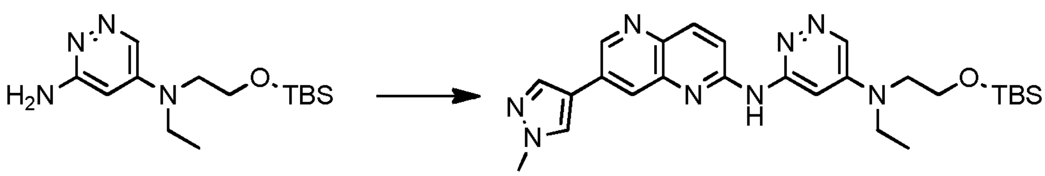

$N^5$-(2-((tert-butyldimethylsilyl)oxy)ethyl)-$N^5$-ethyl-$N^3$-(7-(1-methyl-1H-pyrazol-4-yl)-1,5-naphthyridin-2-yl)pyridazine-3,5-diamine was obtained in the same manner as in Example 0489-4 except that $N^5$-(2-((tert-butyldimethylsilyl)oxy)ethyl)-$N^5$-ethylpyridazine-3,5-diamine was used instead of the 4-methylpyridazine-3-amine used in Example 0489-4.
$^1$H-NMR($CDCl_3$)δ:8.86(1H,brs),8.51(1H,brs),8.39(1H,brs),8.19(1H,d,J=8.7Hz),8.00(1H,d,J=1.8Hz),7.89( 1H,s),7.78(1H,s),7.20(1H,d,J=8.7Hz),4.02(3H,s),3.92(2H,t,J=5.1Hz),3.67(4H,m),1.37(3H,t,J=6.6Hz), 0.86(9H,s),0.31(6H,s).
MSm/z(M+H):505.

<Example 0525>

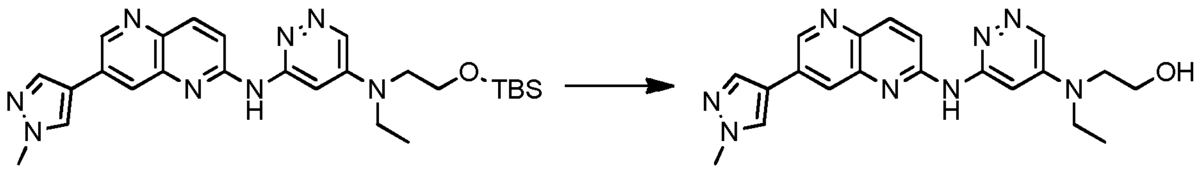

12 mol/L hydrochloric acid (0.2 mL) was added to a solution of $N^5$-(2-((tertbutyldimethylsilyl)oxy)ethyl)-$N^5$-ethyl-$N^3$-(7-(1-methyl-1H-pyrazol-4-yl)-1,5-naphthyridin-2-yl)pyridazine-3,5-diamine (8.3 mg) in methanol (1.4 mL), followed by stirring at room temperature for 3 hours. The solvent was distilled off under reduced pressure, ethyl acetate was added to the obtained residue, and the solid matter was collected by filtration, thereby obtaining hydrochloric acid salt of 2-(ethyl (6-((7-(1-methyl-1H-pyrazol-4-yl)-1,5-naphthyridin-2-yl)amino)pyridazin-4-yl)amino)ethanol (5.1 mg).
$^1$H-NMR(DMSO-$d_6$)δ:9.18(1H,d,J=2.4Hz),8.83(1H,m),8.76(1H,d,J=2.4Hz),8.47(1H,s),8.43(1H,s),8.41(1H,d,J=9.0Hz),8 .16(1H,s),7.46( 1H,d,J=9.0Hz),3.94(3H,s),3.86-3.26(6H,m),1.23(3H,t,J=6.6Hz).
MSm/z(M+H):391.

<Example 0526>

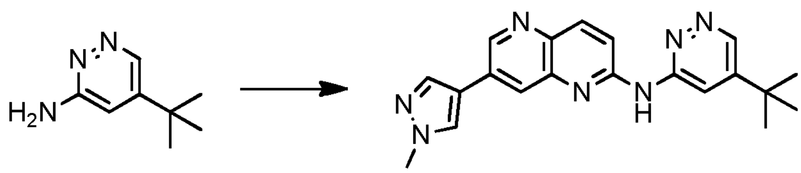

N-(5-(tert-butyl)pyridazin-3-yl)-7-(1-methyl-1H-pyrazol-4-yl)-1,5-naphthyridine-2-amine was obtained in the same manner as in Example 0489-4 except that 5-(tert-butyl)pyridazine-3-amine was used instead of the 4-methylpyridazine-3-amine used in Example 0489-4.
$^1$H-NMR(DMSO-$d_6$)δ:10.68(1H,s),9.03(1H,d,J=2.1Hz),9.02(1H,J=2.1Hz),8.76(1H,d,J=2.1Hz),8.45(1H,s,),8.22(1H,d,J= 9.3Hz),8.16(1H,s),8.10(d,J=2.1Hz),7.75(1H,d,J=9.3Hz),3.93(3H,s),1.40(9H,s).
MSm/z(M+H):360.

<Example 0527>

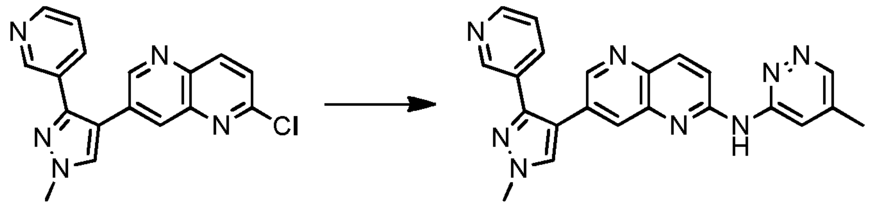

7-(1-Methyl-3-(pyridin-3-yl)-1H-pyrazol-4-yl)-N-(5-methylpyridazin-3-yl)-1,5-naphthyridine-2-amine was obtained in the same manner as in Example 0515-2 except that 5-methylpyridazine-3-amine was used instead of the 5-isopropylpyridazine-3-amine used in Example 0515-2.
$^1$H-NMR($CDCl_3$)$\delta$:8.80(1H,d,J=2.1Hz),8.72(1H,brs),8.64(1H,d,J=2.7Hz),8.57(1H,dd,J=5.1,2.7Hz),8.47(2 H,brs),8.23(1H,d,J=9.0Hz),8.00(1H,d,J=2.1Hz),7.81(1H,dt,J=5.1,2.1Hz),7.71(1H,d,J=9.0Hz),7.71(1H, s),4.07(3H,s),2.42(3H,s).
MSm/z(M+H):395.

<Example 0528>

<0528-1>

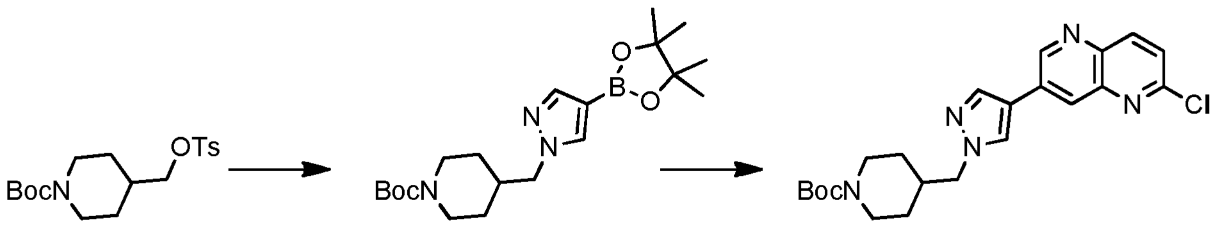

tert-Butyl 4-((4-(6-chloro-1,5-naphthyridin-3-yl)-1H-pyrazol-1 -yl)methyl)piperidine-1 - carboxylate was obtained in the same manner as in Example 0471-1 except that tert-butyl 4-((tosyloxy)methyl)piperidine-1-carboxylate was used instead of the 1-bromo-2-methoxyethane used in Example 0471-1.
MSm/z(M+H):428.

<0528-2>

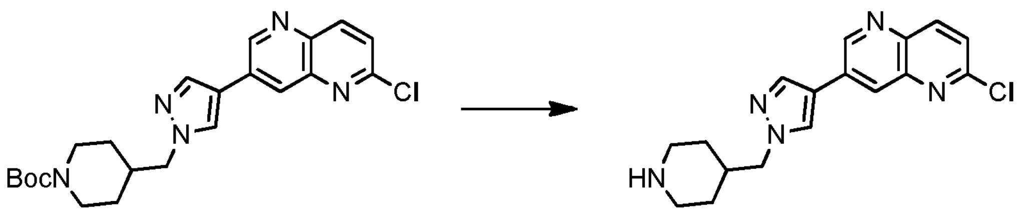

Trifluoroacetic acid (2 mL) was added to a mixture of tert-butyl 4-((4-(6-chloro-1,5-naphthyridin-3-yl)-1H-pyrazol-1-yl)methyl)piperidine-1-carboxylate (1.51 g), water (0.1 mL), and dichloromethane (1 mL), followed by stirring at room temperature for 2 hours. The solvent was distilled off under reduced pressure, the obtained residue was purified by silica gel column chromatography (methanol-ethyl acetate, NH silica), thereby obtaining 2-chloro-7-(1-(piperidin-4-ylmethyl)-1H-pyrazol-4-yl)-1,5-naphthyridine (540 mg).
MSm/z(M+H):328.

<0528-3>

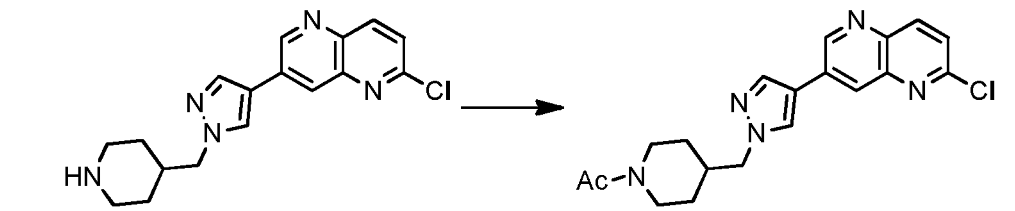

Acetyl chloride (0.008 mL) was added to a solution of 2-chloro-7-(1-(piperidin-4-ylmethyl)-1H-pyrazol-4-yl)-1,5-naphthyridine (23 mg), and triethylamine (0.020 mL) in dichloromethane (1 mL) under ice-cooling, followed by stirring at the same temperature for 2 hours. The reaction mixture was purified by silica gel column chromatography (methanol-ethyl acetate, NH silica), thereby obtaining 1-(4-((4-(6-chloro-1,5-naphthyridin-3-yl)-1H-pyrazol-1-yl)methyl)piperidin-1-yl)ethanone (8.5 mg).
MSm/z(M+H):370.

<0528-4>

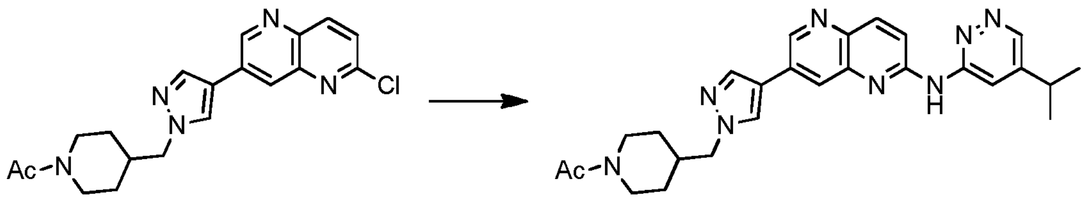

1-(4-((4-(6-((5-Isopropylpyridazin-3-yl)amino)-1,5-naphthyridin-3-yl)-1H-pyrazol-1-yl)methyl)piperidin-1-yl)ethanone was obtained in the same manner as in Example 0471-2 except that 1-(4-((4-(6-chloro-1,5-naphthyridin-3-yl)-1H-pyrazol-1-yl)methyl)piperidin-1-yl)ethanone was used instead of the 2-chloro-7-(1-(2-methoxyethyl)-1H-pyrazol-4-yl)-1,5-naphthyridine used in Example 0471-2.
1H-NMR($CDCl_3$)δ:9.72(1H,brs),8.94-8.91(2H,m),8.84(1H,d,J=1.8Hz),8.25(1H,d,J=9.3Hz),8.11(1H,d,J=2.1Hz),7.98(1H,s,),7.81(1H,s),7.74( 1H,J=9.3Hz),4.74-4.64(1H,m),4.15-4.06(2H,m),3.91-3.80(1H,m),3.13-2.99(2H,m),2.62-2.49(1H,m),2.35-2.18(1H,m),2.09(3H,s),1.77-1.60(2H,m),1.43(6H,d,J=6.6Hz),1.36-1.18(2H,m).
MSm/z(M+H):471.

<Example 0529>

<0529-1>

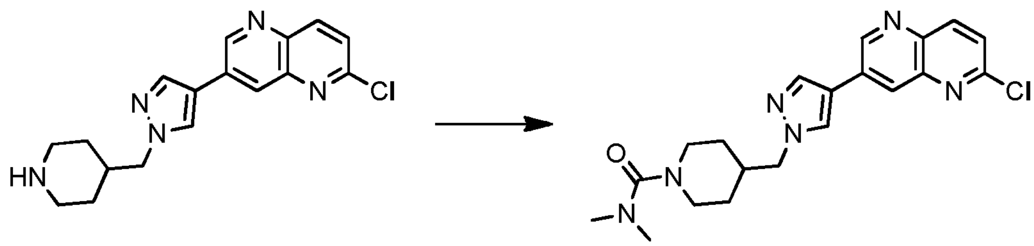

4-((4-(6-Chloro-1,5-naphthyridin-3-yl)-1H-pyrazol-1-yl)methyl)-N,N-dimethylpiperidine-1-carboxamide was obtained in the same manner as in Example 0528-3 except that dimethylcarbamoyl chloride was used instead of the acetyl chloride used in Example 0528-3.
MSm/z(M+H):399.

<0529-2>

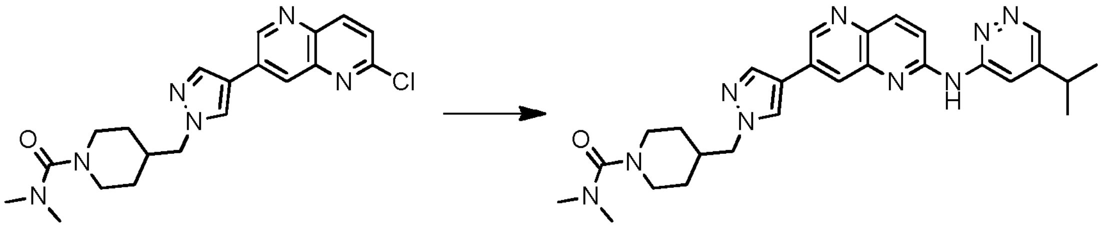

4-((4-(6-((5-Isopropylpyridazin-3-yl)amino)-1,5-naphthyridin-3-yl)-1H-pyrazol-1-yl)methyl)-N,N-dimethylpiperidine-1-carboxamide was obtained in the same manner as in Example 0471-2 except that 4-((4-(6-chloro-1,5-naphthyridin-3-yl)-1H-pyrazol-1-yl)methyl)-N,N-dimethylpiperidine-1-carboxamide was used instead of the 2-chloro-7-(1-(2-methoxyethyl)-1H-pyrazol-4-yl)-1,5-naphthyridine used in Example 0471-2.
$^1$H-NMR($CDCl_3$)δ:10.01(1H,brs),8.97(1H,d,J=2.1Hz),8.92(1H,d,J=2.1Hz),8.84(1H,d,J=2.1Hz),8.25(1H,d ,J=9.3Hz),8.10(1H,d,J=2.1Hz),7.98(1H,s),7.81(1H,s),7.79(1H,d,J=9.3Hz),4.10(2H,d,J=7.5Hz),3.76-3.66(2H,m),3.13-3.02(1H,m),2.78-2.70(2H,m),2.82(6H,s),2.25-2.13(1H,m),1.72-1.60(2H,m),1.44(6H,d,J=6.6Hz),1.41-1.23(2H,m).
MSm/z(M+H):500.

<Example 0530>

<0530-1>

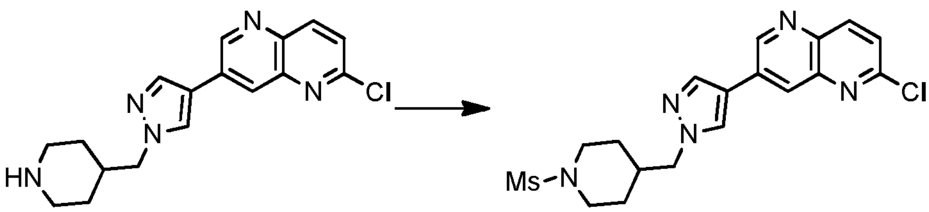

2-Chloro-7-(1-((1-(methylsulfonyl)piperidin-4-yl)methyl)-1H-pyrazol-4-yl)-1,5-naphthyridine was obtained in the same manner as in Example 0528-3 except that methanesulfonyl chloride was used instead of the acetyl chloride used in Example 0528-3.
MSm/z(M+H):406.

<0530-2>

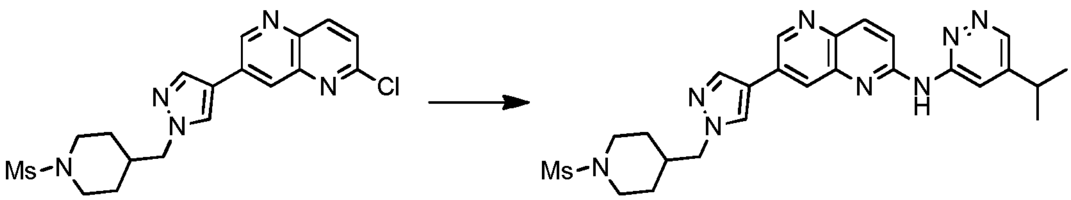

N-(5-isopropylpyridazin-3-yl)-7-(1-((1-(methylsulfonyl)piperidin-4-yl)methyl)-1H-pyrazol-4-yl)-1,5-naphthyridine-2-amine was obtained in the same manner as in Example 0471-2 except that 2-chloro-7-(1-((1-(methylsulfonyl)piperidin-4-yl)methyl)-1H-pyrazol-4-yl)-1,5-naphthyridine was used instead of the 2-chloro-7-(1-(2-methoxyethyl)-1H-pyrazol-4-yl)-1,5-naphthyridine used in Example 0471-2.

$^1$H-NMR($CDCl_3$/$CD_3OD$=4/1)δ:8.88(2H,brs),8.72(1H,brs),8.20(1H,d,J=9.3Hz),8.15(1H,brs),8.01(1H,s),7.98(1H,s),7.51(1H,d,J=9.3Hz),4.19-4.12(2H,m),3.88-7.79(2H,m),3.13-2.99(1H,m),2.81(3H,s),2.78-2.65(2H,m),2.23-2.06(1H,m),1.83-1.73(2H,m),1.42(6H,d,J=6.6Hz),1.29-1.23(2H,m).
MSm/z(M+H):507.

<Example 0531>

<0531-1>

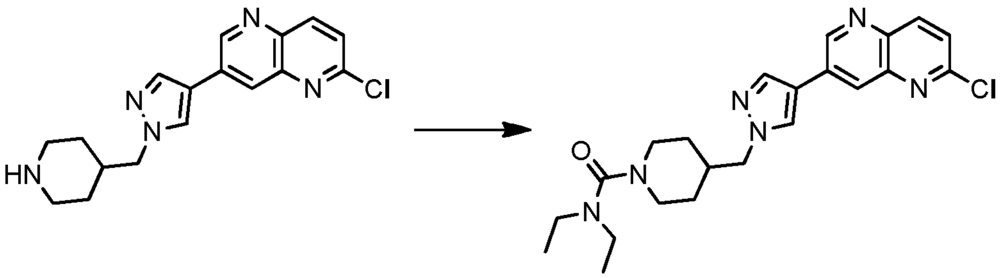

4-((4-(6-Chloro-1,5-naphthyridin-3-yl)-1H-pyrazol-1-yl)methyl)-N,N-diethylpiperidine-1-carboxamide was obtained in the same manner as in Example 0528-3 except that diethylcarbamoyl chloride was used instead of the acetyl chloride used in Example 0528-3.
MSm/z(M+H):427.

<0531-2>

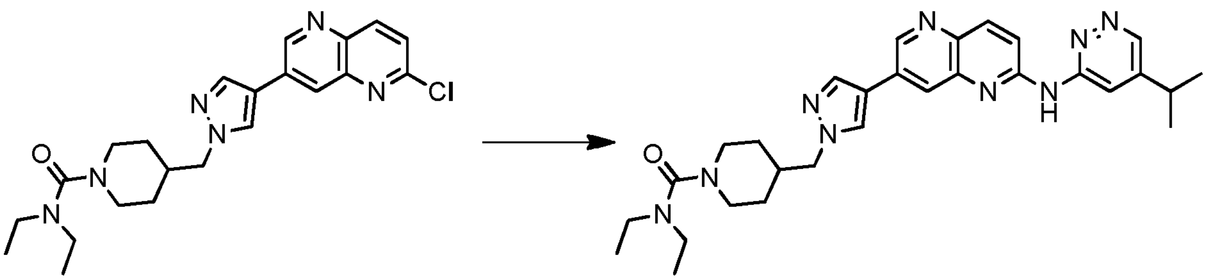

N,N-diethyl-4-((4-(6-((5-isopropylpyridazin-3-yl)amino)-1,5-naphthyridin-3-yl)-1H-pyrazol-1-yl)methyl)piperidine-1-carboxamide was obtained in the same manner as in Example 0471-2 except that 4-((4-(6-chloro-1,5-naphthyridin-3-yl)-1H-pyrazol-1-yl)methyl)-N,N-diethylpiperidine-1-carboxamide was used instead of the 2-chloro-7-(1-(2-methoxyethyl)-1H-pyrazol-4-yl)-1,5-naphthyridine used in Example 0471-2.
$^1$H-NMR(DMSO-$d_6$)δ:10.69(1H,s),9.04(1H,d,J=2.1Hz),8.87(1H,d,J=2.1Hz),8.73(1H,brs),8.49(1H,s),8.24-8.19(3H,m),7.70(1H,d,J=9.3Hz),4.09(2H,d,J=7.2Hz),3.48-3.02(9H,m),2.14-1.90(1H,m),1.58-1.46(2H,m),1.33(6H,d,J=7.2Hz),1.27-1.14(2H,m),1.02(6H,t,J=6.6Hz).
MSm/z(M+H):528.

<Example 0532>

<0532-1>

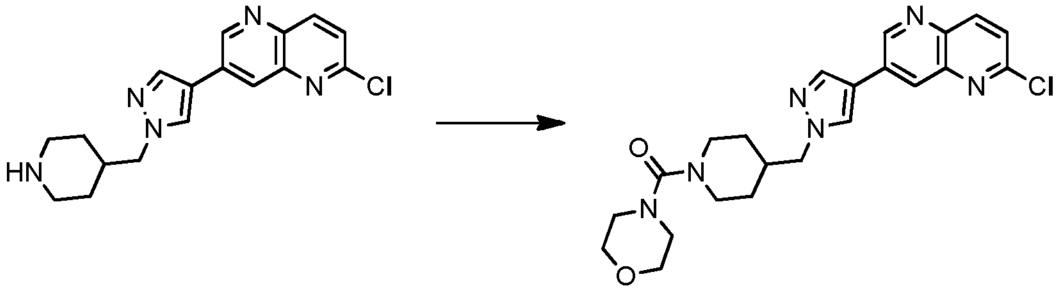

(4-((4-(6-Chloro-1,5-naphthyridin-3-yl)-1H-pyrazol-1-yl)methyl)piperidin-1-yl) (morpholino)methanone was obtained in the same manner as in Example 0528-3 except that morpholine-4-carbonyl chloride was used instead of the acetyl chloride used in Example 0528-3. MSm/z(M+H):441.

<0532-2>

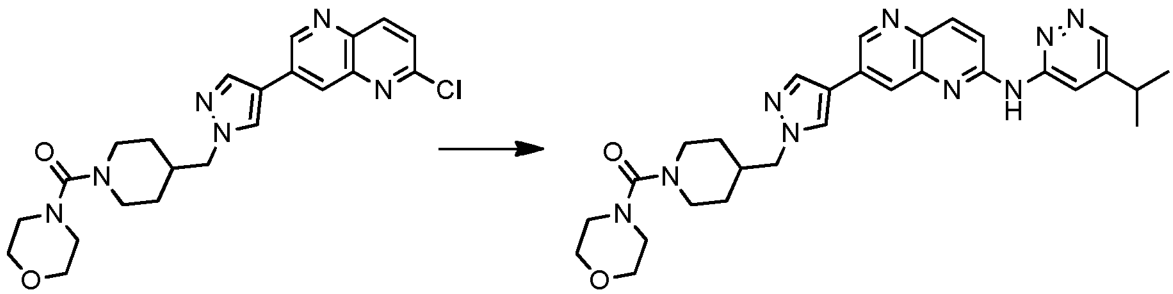

(4-((4-(6-((5-Isopropylpyridazin-3-yl)amino)-1,5-naphthyridin-3-yl)-1H-pyrazol-1-yl)methyl)piperidin-1-yl) (morpholino)methanone was obtained in the same manner as in Example 0471-2 except that (4-((4-(6-chloro-1,5-naphthyridin-3-yl)-1H-pyrazol-1-yl)methyl)piperidin-1-yl) (morpholino)methanone was used instead of the 2-chloro-7-(1-(2-methoxyethyl)-1H-pyrazol-4-yl)-1,5-naphthyridine used in Example 0471-2.
$^1$H-NMR($CDCl_3$)$\delta$:9.70(1H,brs),8.93(1H,d,J=2.1Hz),8.92(1H,d,J=2.1Hz),8.83(1H,d,J=2.1Hz),8.25(1H,d,J =9.3Hz),8.10(1H,d,J=2.1Hz),7.98(1H,s,),7.80(1H,s),7.73(1H,d,J=9.3Hz),4.10(2H,d,J=7.2Hz),3.80-3.70(2H,m),3.67(4H ,t,J=4.5Hz),3.25(4H,t,J=4.5Hz),3.13-3.02(1H,m),2.86-2.73(2H,m),2.28-2.10(1H,m),1.72-1.62(2H,m),1.43(6H,d,J=7.2H z),1.38-1.24(2H,m).
MSm/z(M+H):542.

<Example 0533>

<0533-1>

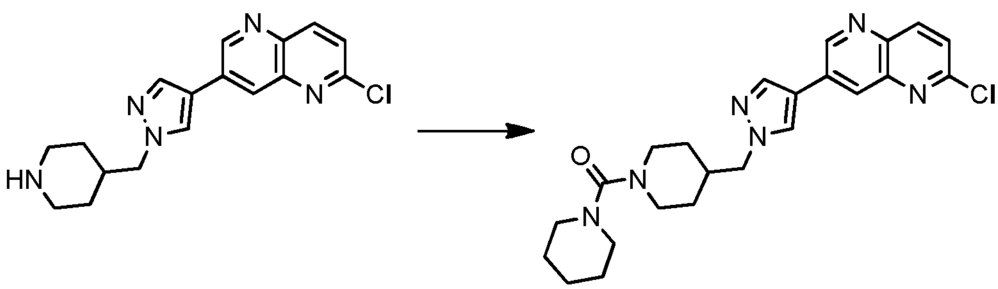

(4-((4-(6-Chloro-1,5-naphthyridin-3-yl)-1H-pyrazol-1-yl)methyl)piperidin-1-yl)(piperidin-1-yl)methanone was obtained in the same manner as in Example 0528-3 except that piperidine-1-carbonyl chloride was used instead of the

acetyl chloride used in Example 0528-3.
MSm/z(M+H):439.

<0533-2>

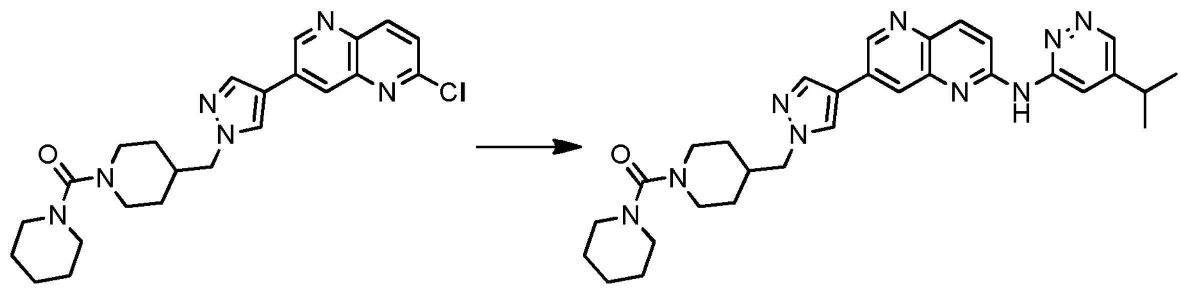

(4-((4-(6-((5-Isopropylpyridazin-3-yl)amino)-1,5-naphthyridin-3-yl)-1H-pyrazol-1-yl)methyl)piperidin-1-yl) (piperidin-1-yl)methanone was obtained in the same manner as in Example 0471-2 except that (4-((4-(6-chloro-1,5-naphthyridin-3-yl)-1H-pyrazol-1-yl)methyl)piperidin-1-yl) (piperidin-1-yl)methanone was used instead of the 2-chloro-7-(1-(2-methoxyethyl)-1H-pyrazol-4-yl)-1,5-naphthyridine used in Example 0471-2.
$^1$H-NMR($CDCl_3$)$\delta$:9.98(1H,brs),8.96(1H,d,J=2.1Hz),8.92(1H,d,J=2.1Hz),8.83(1H,d,J=2.1Hz),8.25(1H,d,J =9.3Hz),8.10(1H,d,J=2.1Hz),7.97(1H,s),7.81(1H,s),7.79(1H,d,J=9.3Hz),4.10(2H,d,J=6.6Hz),3.75-3.66(2H,m),3.22-3.14(4H,m),3.13-3.02(1H,m),2.82-2.70(2H,m),2.26-2.09(1H,m),1.72-1.58(2H,m),1.43(6H,d,J=6.6Hz),1.40-1.23(4H,m).
MSm/z(M+H):540.

<Example 0534>

<0534-1>

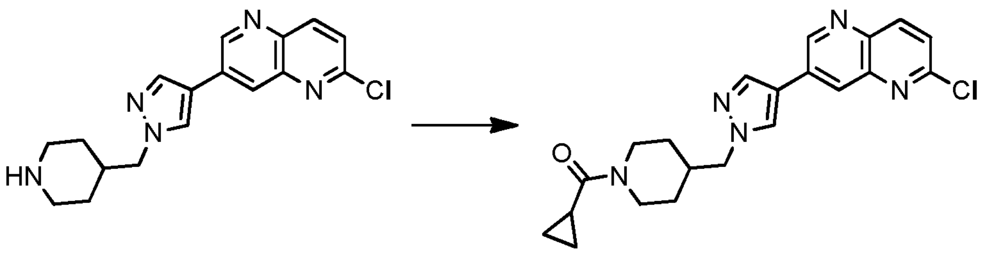

(4-((4-(6-Chloro-1,5-naphthyridin-3-yl)-1H-pyrazol-1 -yl)methyl)piperidin-1 -yl) (cyclopropyl)methanone was obtained in the same manner as in Example 0528-3 except that cyclopropanecarbonyl chloride was used instead of the acetyl chloride used in Example 0528-3.
MSm/z(M+H):396.

<0534-2>

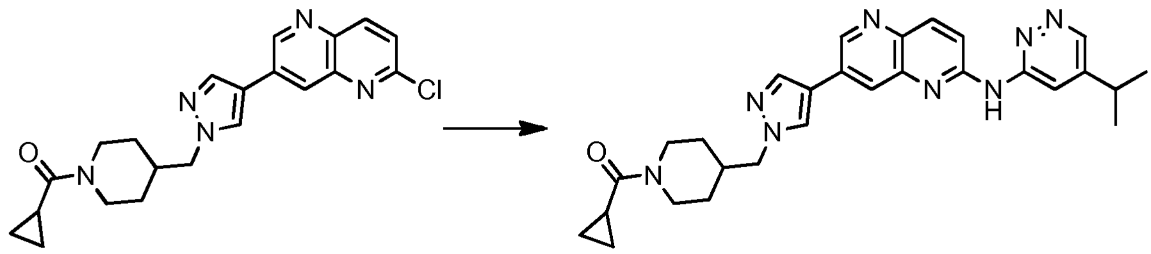

Cyclopropyl (4-((4-(6-((5-isopropylpyridazin-3-yl)amino)-1,5-naphthyridin-3-yl)-1H-pyrazol-1-yl)methyl)piperidin-1-yl)methanone was obtained in the same manner as in Example 0471-2 except that (4-((4-(6-chloro-1,5-naphthyridin-3-yl)-1H-pyrazol-1-yl)methyl)piperidin-1-yl) (cyclopropyl)methanone was used instead of the 2-chloro-7-(1-(2-methoxyethyl)-1H-pyrazol-4-yl)-1,5-naphthyridine used in Example 0471-2.
$^1$H-NMR($CDCl_3$)$\delta$:9.38(1H,brs),8.92(1H,d,J=2.1Hz),8.88(1H,brs),8.83(1H,d,J=2.1Hz),8.25(1H,d,J=9.0Hz),8.11(1H,d,J=2.1Hz),7.98(1H,s),7.81(1H,s),7.67(1H,d,J=9.0Hz),4.78-4.60(2H,m),4.33-4.06(4H,m),3.13-3.01(1H,m),2.68-2.52(1H,m),2.37-2.20(1H,m),1.80-1.66(2H,m),1.43(6H,d,J=6.6Hz),1.36-1.23(2H,m),1.01-0.94(2H,m),0.79-0.71(2H,m).
MSm/z(M+H):497.

<Example 0535>

<0535-1>

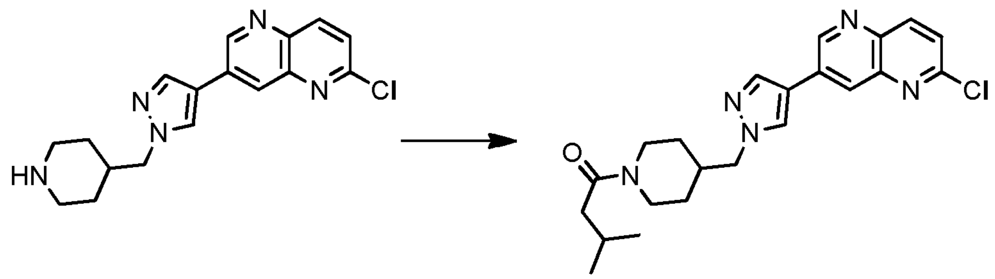

1-(4-((4-(6-Chloro-1,5-naphthyridin-3-yl)-1H-pyrazol-1-yl)methyl)piperidin-1-yl)-3-methylbutan-1 -one was obtained in the same manner as in Example 0528-3 except that 3-methylbutanoyl chloride was used instead of the acetyl chloride used in Example 0528-3.
MSm/z(M+H):412.

<0535-2>

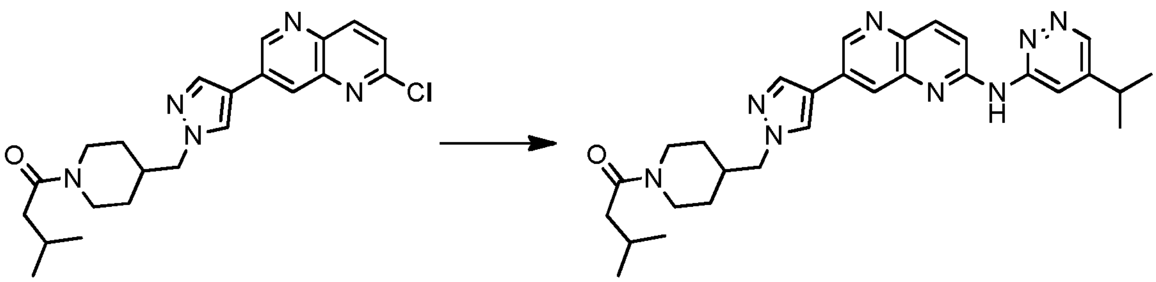

1-(4-((4-(6-((5-Isopropylpyridazin-3-yl)amino)-1,5-naphthyridin-3-yl)-1H-pyrazol-1-yl)methyl)piperidin-1-yl)-3-methylbutan-1-one was obtained in the same manner as in Example 0471-2 except that 1-(4-((4-(6-chloro-1,5-naphthyridin-3-yl)-1H-pyrazol-1-yl)methyl)piperidin-1-yl)-3-methylbutan-1-one was used instead of the 2-chloro-7-(1-(2-methoxyethyl)-1H-pyrazol-4-yl)-1,5-naphthyridine used in Example 0471-2.

$^1$H-NMR($CDCl_3$)δ:8.92(1H,d,J=2.1Hz),8.87(1H,brs),8.83(1H,d,J=2.1Hz),8.25(1H,d,J=9.3Hz),8.10(1H,d,J =2.1Hz),7.98(1H,s),7.81(1H,s),7.64(1H,d,J=9.3Hz),4.78-4.64(2H,m),4.15-4.07(2H,m),3.98-3.87(2H,m),3.13-2.97(1H, m),2.62-2.49(1H,m),2.21(2H,d,J=6.6Hz),2.18-2.03(1H,m),1.76-1.67(2H,m),1.43(6H,d,J=7.2Hz),1.35-1.18(2H,m),0.97( 6H,d,J=6.0Hz).
MSm/z(M+H):513.

<Example 0536>

<0536-1>

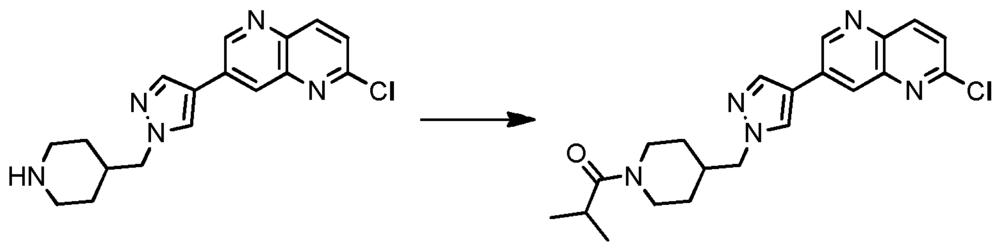

1-(4-((4-(6-Chloro-1,5-naphthyridin-3-yl)-1H-pyrazol-1-yl)methyl)piperidin-1-yl)-2-methylpropan-1-one was obtained in the same manner as in Example 0528-3 except that isobutyryl chloride was used instead of the acetyl chloride used in Example 0528-3.
MSm/z(M+H):398.

<0536-2>

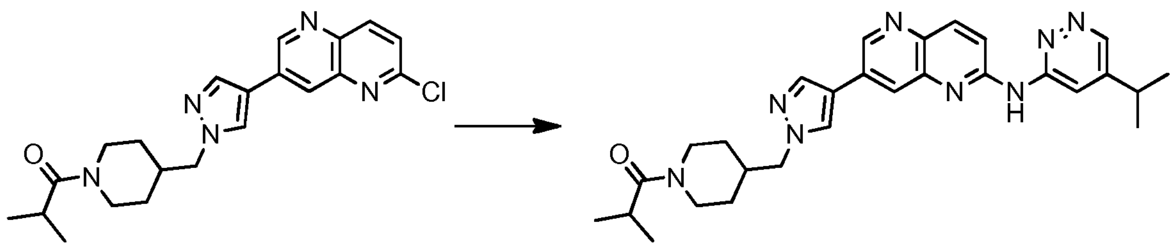

1-(4-((4-(6-((5-Isopropylpyridazin-3-yl)amino)-1,5-naphthyridin-3-yl)-1H-pyrazol-1-yl)methyl)piperidin-1-yl)-2-methylpropan-1-one was obtained in the same manner as in Example 0471-2 except that 1-(4-((4-(6-chloro-1,5-naphthyridin-3-yl)-1H-pyrazol-1-yl)methyl)piperidin-1-yl)-2-methylpropan-1-one was used instead of the 2-chloro-7-(1-(2-methoxyethyl)-1H-pyrazol-4-yl)-1,5-naphthyridine used in Example 0471-2.
$^1$H-NMR($CDCl_3$)δ:9.48(1H,brs),8.92(1H,d,J=2.1Hz),8.91(1H,d,J=2.1Hz),8.83(1H,brs),8.25(1H,d,J=9.3H z),8.10(1H,brs),7.98(1H,s),7.81(1H,s),7.69(1H,d,J=9.3Hz),4.78-4.64(2H,m),4.14-4.06(2H,m),4.03-3.93(2H,m),3.13-2. 96(1H,m),2.88-2.74(1H,m),2.36-2.20(1H,m),1.78-1.67(2H,m),1.43(6H,d,J=6.6Hz),1.26(6H,d,J=6.3Hz),1.32-1.09(2H,m ).
MSm/z(M+H):499.

<Example 0537>

<0537-1>

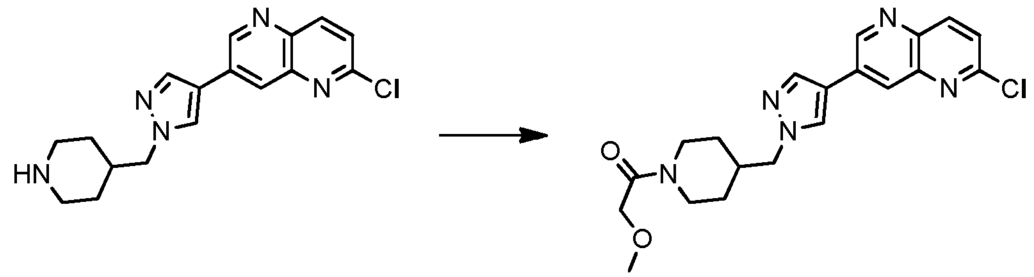

1-(4-((4-(6-Chloro-1,5-naphthyridin-3-yl)-1H-pyrazol-1-yl)methyl)piperidin-1-yl)-2-methoxyethanone was obtained in the same manner as in Example 0528-3 except that 2-methoxyacetyl chloride was used instead of the acetyl chloride used in Example 0528-3.
MSm/z(M+H):400.

<0537-2>

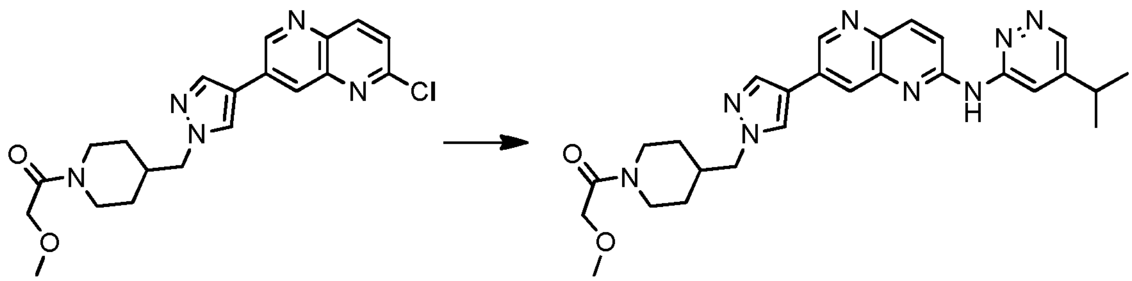

1-(4-((4-(6-((5-Isopropylpyridazin-3-yl)amino)-1,5-naphthyridin-3-yl)-1H-pyrazol-1-yl)methyl)piperidin-1-yl)-2-methoxyethanone was obtained in the same manner as in Example 0471-2 except that 1-(4-((4-(6-chloro-1,5-naphthyridin-3-yl)-1H-pyrazol-1-yl)methyl)piperidin-1-yl)-2-methoxyethanone was used instead of the 2-chloro-7-(1-(2-methoxyethyl)-1H-pyrazol-4-yl)-1,5-naphthyridine used in Example 0471-2.
$^1$H-NMR($CDCl_3$)$\delta$:8.92(1H,d,J=2.1Hz),8.90(1H,brs),8.83(1H,d,J=2.1Hz),8.25(1H,d,J=9.3Hz),8.10(1H,d,J =2.1Hz),7.98(1H,s),7.81(1H,s),7.69(1H,d,J=9.3Hz),4.71-4.59(1H,m),4.18-4.02(2H,m),4.11(2H,s),3.98-3.88(1H,m),3.42(3H,s),3.13-2.94(2H,m),2.68-2.54(1H,m),2.36-2.20(1H,m),1.78-1.67(2H,m),1.43(6H,d,J=6.6Hz),1.36-1.22(2H,m).
MSm/z(M+H):501.

<Example 0538>

<0538-1>

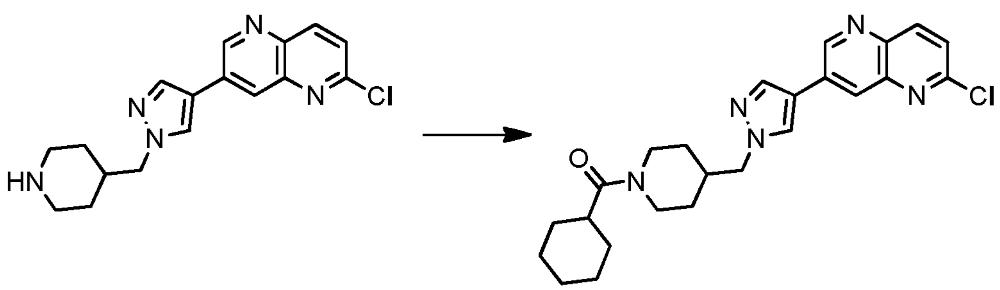

(4-((4-(6-Chloro-1,5-naphthyridin-3-yl)-1H-pyrazol-1 -yl)methyl)piperidin-1 -yl) (cyclohexyl)methanone was obtained in the same manner as in Example 0528-3 except that cyclohexanecarbonyl chloride was used instead of the acetyl chloride used in Example 0528-3.
MSm/z(M+H):438.

<0538-2>

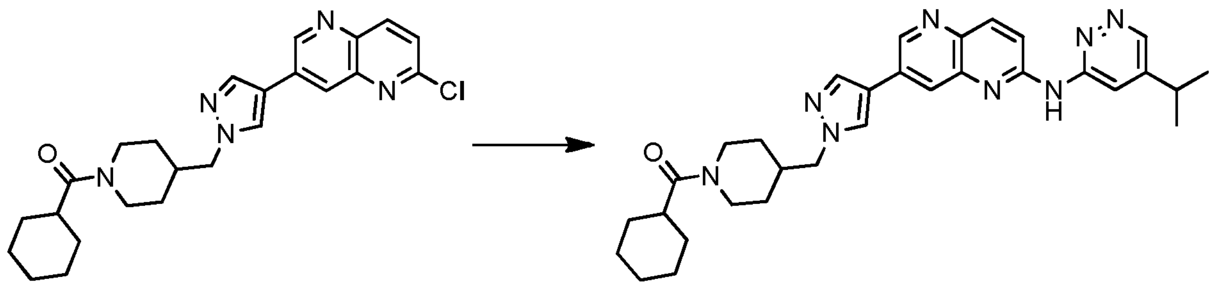

Cyclohexyl (4-((4-(6-((5-isopropylpyridazin-3-yl)amino)-1,5-naphthyridin-3-yl)-1H-pyrazol-1-yl)methyl)piperidin-1-yl)methanone was obtained in the same manner as in Example 0471-2 except that (4-((4-(6-chloro-1,5-naphthyridin-3-yl)-1H-pyrazol-1-yl)methyl)piperidin-1-yl) (cyclohexyl)methanone was used instead of the 2-chloro-7-(1-(2-methoxyethyl)-1H-pyrazol-4-yl)-1,5-naphthyridine used in Example 0471-2.
$^1$H-NMR($CDCl_3$)δ:8.93(1H,d,J=2.1Hz),8.89(1H,brs),8.83(2H,brs),8.25(1H,d,J=9.3Hz),8.10(1H,d,J=2.1Hz),7.98(1H,s)7.81(1H,s),7.56(1H,d,J=9.3Hz),4.78-4.64(1H,m),4.14-4.06(2H,m),4.02-3.90(2H,m),3.12-2.95(2H,m),2.59-2.40(1H,m),2.34-2.18(1H,m),1.85-1.63(8H,m),1.42(6H,d,J=6.6Hz),1.35-1.16(6H,m).
MSm/z(M+H):539.

<Example 0539>

<0539-1>

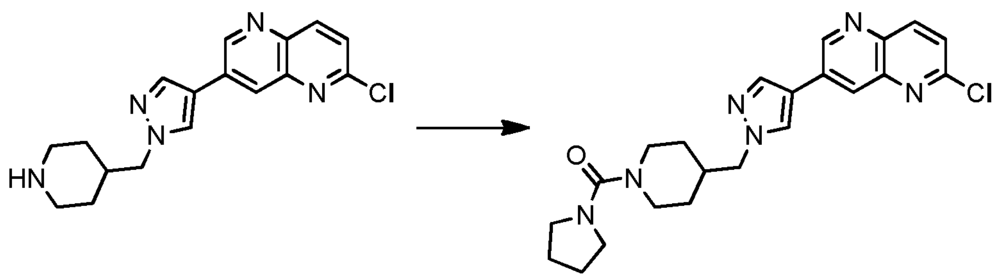

(4-((4-(6-Chloro-1,5-naphthyridin-3-yl)-1H-pyrazol-1 -yl)methyl)piperidin-1 -yl) (pyrrolidin-1-yl)methanone was obtained in the same manner as in Example 0528-3 except that pyrrolidine-1-carbonyl chloride was used instead of the acetyl chloride used in Example 0528-3.
MSm/z(M+H):425.

<0539-2>

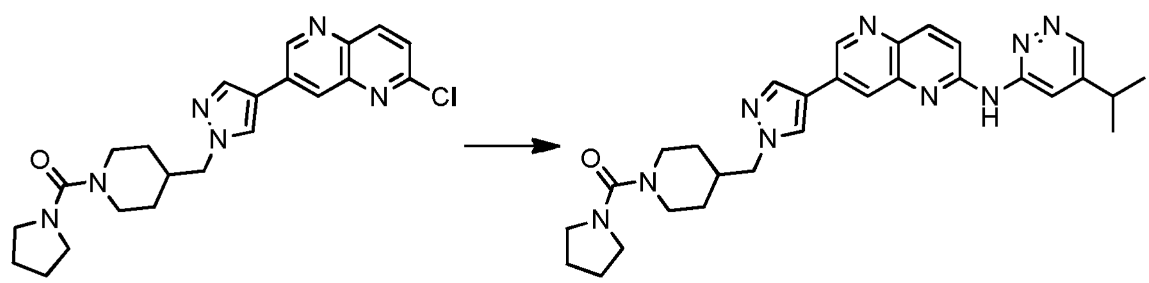

(4-((4-(6-((5-Isopropylpyridazin-3-yl)amino)-1,5-naphthyridin-3-yl)-1H-pyrazol-1-yl)methyl)piperidin-1-yl) (pyrrolidin-1-yl)methanone was obtained in the same manner as in Example 0471-2 except that (4-((4-(6-chloro-1,5-naphthyridin-3-yl)-1H-pyrazol-1-yl)methyl)piperidin-1-yl) (pyrrolidin-1-yl)methanone was used instead of the 2-chloro-7-(1-(2-methoxyethyl)-1H-pyrazol-4-yl)-1,5-naphthyridine used in Example 0471-2.
$^1$H-NMR($CDCl_3$)$\delta$:10.07(1H,brs),8.96(1H,brs),8.92(1H,brs),8.83(1H,d,J=2.1Hz),8.25(1H,d,J=8.4Hz),8.10(1H,d,J=2.1Hz),7.97(1H,s),7.81(1H,s),7.79(1H,d,J=8.4Hz),4.10(2H,d,J=7.2Hz),3.86-3.76(2H,m),3.39-3.31(4H,m),3.13-3.02(1H,m),2.88-2.69(2H,m),2.27-2.10(1H,m),1.86-1.60(6H,m),1.43(6H,d,J=7.2Hz),1.41-1.23(2H,m).
MSm/z(M+H):526.

<Example 0540>

<0540-1>

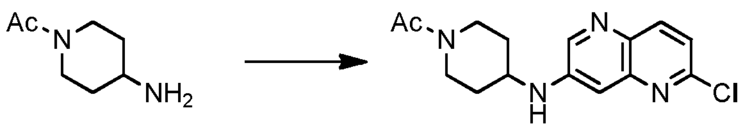

A suspension of 7-bromo-2-chloro-1,5-naphthyridine (44 mg), 1-(4-aminopiperidin-1-yl)ethanone (26 mg), tris(dibenzylideneacetone)dipalladium(0) (15 mg), 4,5-bis(diphenylphosphino)-9,9-dimethylxanthene (30 mg), and sodium tert-butoxide (50 mg) in 1,4-dioxane (2 mL) was stirred at 80°C for 4 hours. The reaction mixture was cooled to room temperature, and purified by silica gel column chromatography (methanol-ethyl acetate), thereby obtaining 1-(4-((6-chloro-1,5-naphthyridin-3-yl)amino)piperidin-1-yl)ethanone (8.1 mg).
MSm/z(M+H):305.

<0540-2>

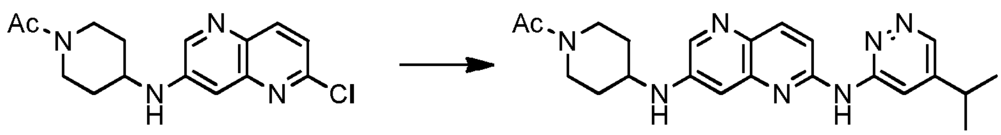

1-(4-((6-((5-Isopropylpyridazin-3-yl)amino)-1,5-naphthyridin-3-yl)amino)piperidin-1-yl)ethanone was obtained in the same manner as in Example 0471-2 except that 1-(4-((6-chloro-1,5-naphthyridin-3-yl)amino)piperidin-1-yl)ethanone was used instead of the 2-chloro-7-(1-(2-methoxyethyl)-1H-pyrazol-4-yl)-1,5-naphthyridine used in Example 0471-2.
$^1$H-NMR($CDCl_3$)$\delta$:8.83(1H,brs),8.78(1H,d,J=2.1Hz),8.64(1H,d,J=2.1Hz),8.27(1H,d,J=2.7Hz),8.10(1H,d,J=9.3Hz),7.37(1H,d,J=9.3Hz),7.03(1H,d,J=2.7Hz),4.61-4.50(1H,m),3.94-3.82(1H,m),3.74-3.60(1H,m),3.35-3.21(1H,m),3.07-2.81(2H,m),2.28-2.13(2H,m),2.14(3H,s),1.55-1.41(2H,m),1.40(6H,d,J=6.6Hz).
MSm/z(M+H):406.

<Example 0541>

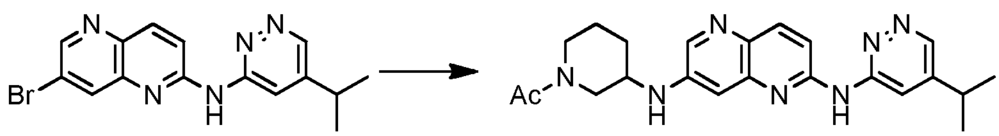

A suspension of 7-bromo-N-(5-isopropylpyridazin-3-yl)-1,5-naphthyridine-2-amine (10 mg), 1-(3-aminopiperidin-1-yl)ethanone (6.2 mg), tris(dibenzylideneacetone)dipalladium(0) (5 mg), 2-(dicyclohexylphosphino)-2',4',6'-triisopropylbiphenyl (10 mg), and sodium tert-butoxide (30 mg) in 1,4-dioxane (1 mL) was stirred at 140°C for 30 minutes using a microwave reaction apparatus. The reaction mixture was cooled to room temperature, and purified by silica gel column chromatography (methanol-ethyl acetate, NH silica), thereby obtaining 1-(3-((6-((5-isopropylpyridazin-3-yl)amino)-1,5-naphthyridin-3-yl)amino)piperidin-1-yl)ethanone (3.9 mg).
$^1H$-NMR($CDCl_3$)δ:8.77(1H,brs),8.70(1H,s),8.29-8.24(1H,m),8.13-8.05(1H,m),7.26-7.19(1H,m),7.11-7.02(1H,m),4.28-4.23 (1H,m),3.93-3.85(1H,m),3.71-3.21(3H,m),3.07-2.94(1H,m),2.17(3H,s),1.91-1.58(2H,m),1.39(6H,d,J=6.6Hz),1.38-1.24 (2H,m).
MSm/z(M+H):406.

<Example 0542>

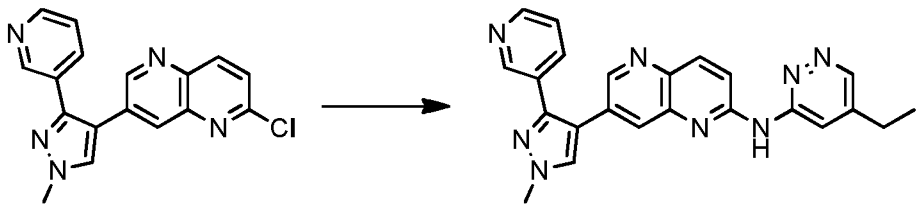

N-(5-ethylpyridazin-3-yl)-7-(1-methyl-3-(pyridin-3-yl)-1H-pyrazol-4-yl)-1,5-naphthyridine-2-amine was obtained in the same manner as in Example 0515-2 except that 5-ethylpyridazine-3-amine was used instead of the 5-isopropylpyridazine-3-amine used in Example 0515-2.
$^1H$-NMR($CDCl_3$)δ:9.65(1H,brs),8.80(2H,brs),8.76(1H,d,J=2.1Hz),8.66(1H,d,J=2.1Hz),8.57(1H,dd,J=5.4, 2.1Hz),8.24(1H,d,J=9.3Hz),7.95(1H,d,J=2.1Hz),7.81(1H,dt,J=7.8,2.1Hz),7.73(1H,d,J=9.3Hz),7.71(1H ,s),7.31-7.24(1 H,m),4.07(3H,s),2.73(2H,q,J=7.2Hz),1.33(3H,t,J=7.2Hz).
MSm/z(M+H):409.

<Example 0543>

<0543-1>

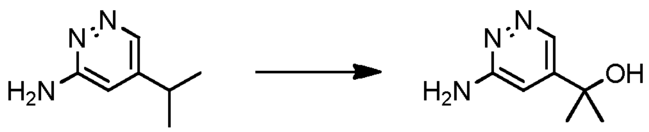

A mixture of 5-isopropylpyridazine-3-amine (300 mg), potassium permanganate (1.38 g), water (2 mL), and tert-butyl alcohol (10 mL) was stirred at 50°C for 10 hours. The reaction mixture was cooled to room temperature, the insolubles were filtered off, and the solvent was distilled off under reduced pressure. The obtained residue was purified by silica gel column chromatography (methanol-ethyl acetate, NH silica), thereby obtaining 2-(6-aminopyridazin-4-yl)propan-2-ol (96 mg).
MSm/z(M+H):154.

<0543-2>

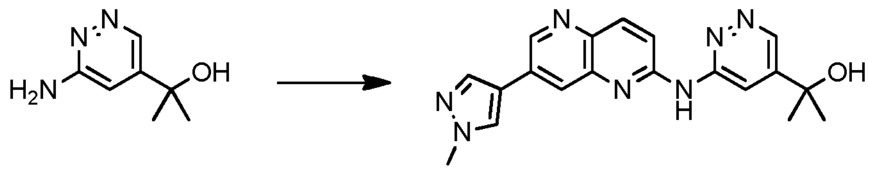

2-(6-((7-(1-Methyl-1H-pyrazol-4-yl)-1,5-naphthyridin-2-yl)amino)pyridazin-4-yl)propan-2-ol was obtained in the same manner as in Example 0489-4 except that 2-(6-aminopyridazin-4-yl)propan-2-ol was used instead of the 4-methylpyridazine-3-amine used in Example 0489-4.
$^1$H-NMR($CDCl_3$/$CD_3OD$=4/1)δ:9.06(1H,brs),8.91(1H,brs),8.88(1H,d,J=2.1Hz),8.22(1H,d,J=2.1Hz),8.19( 1H,d,J=9.3Hz),7.98(1H,s),7.97(1H,s),7.54(1H,d,J=9.3Hz),4.02(3H,s),1.66(6H,s).
MSm/z(M+H):362.

<Example 0544>

<0544-1>

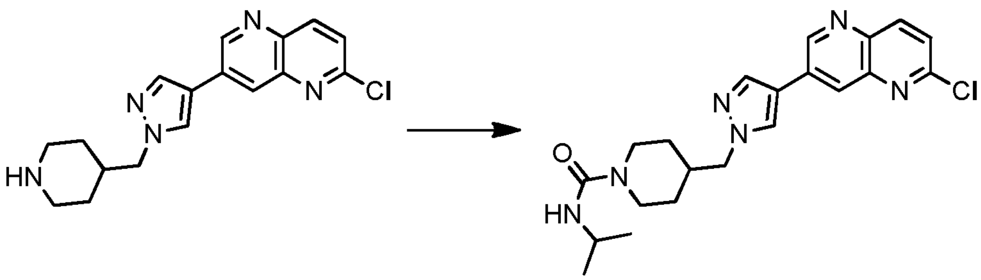

(4-((4-(6-Chloro-1,5-naphthyridin-3-yl)-1H-pyrazol-1 -yl)methyl)-N-isopropylpiperidine-1 - carboxamide was obtained in the same manner as in Example 0528-3 except that 2-isocyanatopropane was used instead of the acetyl chloride used in Example 0528-3.
MSm/z(M+H):413.

<0544-2>

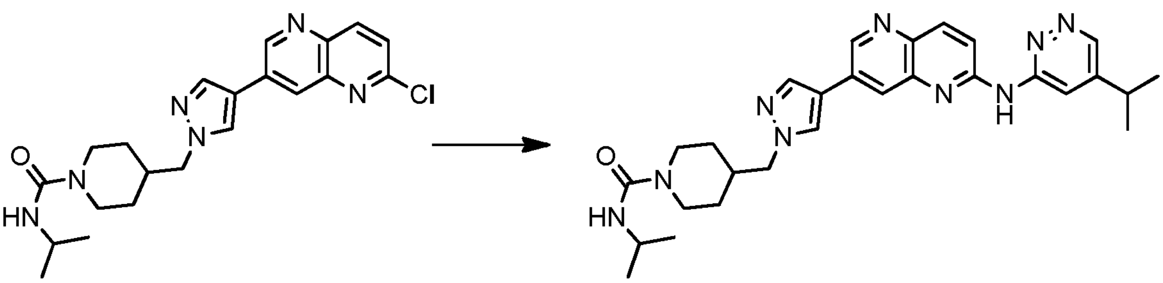

A suspension of (4-((4-(6-chloro-1,5-naphthyridin-3-yl)-1H-pyrazol-1-yl)methyl)-N-isopropylpiperidine-1-carboxamide (22 mg), 5-isopropylpyridazine-3-amine (9.1 mg), and potassium tert-butoxide (19 mg) in 1,4-dioxane (1 mL) was stirred at 110°C for 1 hour. The reaction mixture was cooled to room temperature, and purified by silica gel column chromatography (methanol-ethyl acetate), thereby obtaining N-isopropyl-4-((4-(6-((5-isopropylpyridazin-3-yl)amino)-1,5-naphthyridin-3-yl)-1H-pyrazol-1-yl)methyl)piperidine-1-carboxamide (7.6 mg).
$^1$H-NMR($CDCl_3$)δ:9.80(1H,brs),8.94(1H,brs),8.92(1H,d,J=2.1Hz),8.83(1H,brs),8.25(1H,d,J=8.4Hz),8.10( 1H,d,J=2.1Hz),7.97(1H,s),7.81(1H,s),7.75(1H,d,J=8.4Hz),4.23-4.18(1H,m),4.10(2H,d,J=7.2Hz),4.01-3.90(3H,m),3.13-2.99(1H,m),2.85-2.70(2H,m),2.27-2.10(1H,m),1.73-1.56(2H,m), 1.43(6H,d,J=6.6Hz), 1.39-1.18(2H,m), 1.15(6H,d,J=6.0Hz). MSm/z(M+H):514.

<Example 0545>

<0545-1>

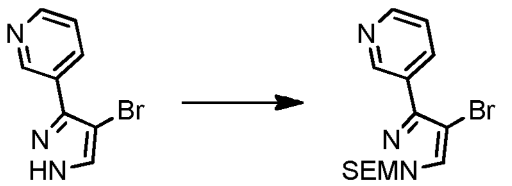

(2-(Chloromethoxy)ethyl)trimethylsilane (0.188 mL) was added to a mixture of 3-(4-bromo-1H-pyrazol-3-yl)pyridine (200 mg), N,N-diisopropylethylamine (0.200 mL), and dichloromethane (9 mL), followed by stirring at room temperature for 3 hours. After water and ethyl acetate were added to the reaction mixture, the organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining 3-(4-bromo-1-((2-(trimethylsilyl)ethoxy)methyl)-1H-pyrazol-3-yl)pyridine (141 mg).
MSm/z(M+H):354.

<0545-2>

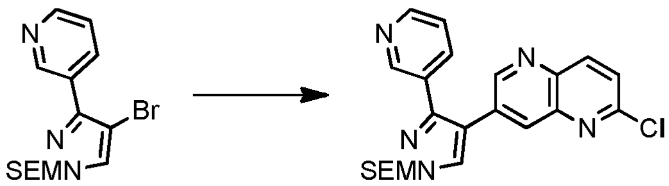

2-Chloro-7-(3-(pyridin-3-yl)-1-((2-(trimethylsilyl)ethoxy)methyl)-1H-pyrazol-4-yl)-1,5-naphthyridine was obtained in the same manner as in Example 0478-3 except that 3-(4-bromo-1-((2-(trimethylsilyl)ethoxy)methyl)-1H-pyrazol-3-yl)pyridine was used instead of the 1-(4-(4-iodo-1H-pyrazol-1-yl)piperidin-1-yl)ethanone used in Example 0478-3.
MSm/z(M+H):438.

<0545-3>

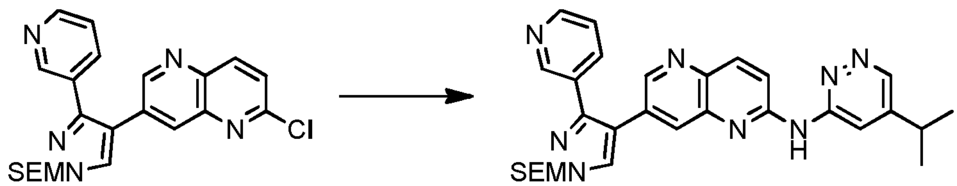

N-(5-isopropylpyridazin-3-yl)-7-(3-(pyridin-3-yl)-1-((2-(trimethylsilyl)ethoxy)methyl)-1H-pyrazol-4-yl)-1,5-naphthyridine-2-amine was obtained in the same manner as in Example 0544-2 except that 2-chloro-7-(3-(pyridin-3-yl)-1-((2-(trimethylsilyl)ethoxy)methyl)-1H-pyrazol-4-yl)-1,5-naphthyridine was used instead of the 4-((4-(6-chloro-1,5-naphthyridin-3-yl)-1H-pyrazol-1-yl)methyl)-N-isopropylpiperidine-1-carboxamide used in Example 0544-2.
MSm/z(M+H):539.

<0545-4>

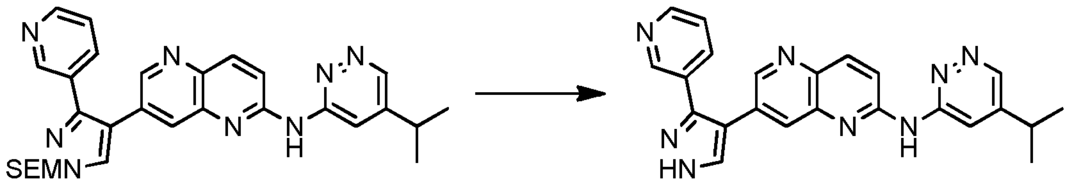

Hydrochloric acid salt of N-(5-isopropylpyridazin-3-yl)-7-(3-(pyridin-3-yl)-1H-pyrazol-4-yl)-1,5-naphthyridine-2-amine was obtained in the same manner as in Example 0525-1 except that N-(5-isopropylpyridazin-3-yl)-7-(3-(pyridin-3-yl)-1-((2-(trimethylsilyl)ethoxy)methyl)-1H-pyrazol-4-yl)-1,5-naphthyridine-2-amine was used instead of the $N^5$-(2-((tert-butyldimethylsilyl)oxy)ethyl)-$N^3$-(7-(1-methyl-1H-pyrazol-4-yl)-1,5-naphthyridin-2-yl)-$N^5$-propylpyridazine-3,5-diamine used in Example 0525-1.
$^1$H-NMR(DMSO-$d_6$)δ:8.97(1H,brs),8.88-8.65(2H,m),8.79(1H,d,J=2.1Hz),8.44(1H,brs),8.38(1H,s),8.37(1H,d,J=7.5Hz),8.27(1H,brs),8.15(1H,d, J=7.5Hz),7.74(1H,d,J=7.5Hz),7.73-7.63(1H,m),3.15-2.97(1H,m),1.27(6H,d,J=6.6Hz).
MSm/z(M+H):409.

<Example 0546>

<0546-1>

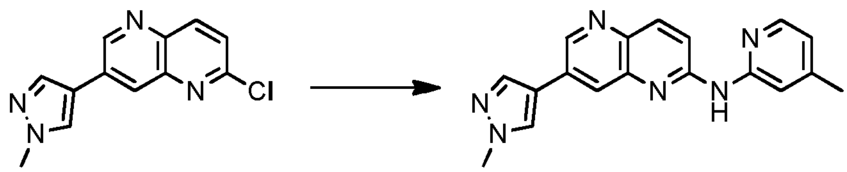

7-(1-Methyl-1H-pyrazol-4-yl)-N-(4-methylpyridin-2-yl)-1,5-naphthyridine-2-amine was obtained in the same manner as in Example 0489-4 except that 4-methylpyridine-2-amine was used instead of the 4-methylpyridazine-3-amine used in Example 0489-4.
$^1$H-NMR($CDCl_3$)δ:8.89(1H,d,J=2.1Hz),8.15(1H,d,J=5.4Hz),8.13(1H,d,J=9.3Hz),8.14(1H,d,J=2.1Hz),8.11(1H,brs),7.95(1H,s),7.83(1H,s),7.48(1H,d,J=9.3Hz),6.81(1H,d,J=5.4Hz),4.02(3H,s),2.44(3H,s).
MSm/z(M+H):317.

<Example 0547>

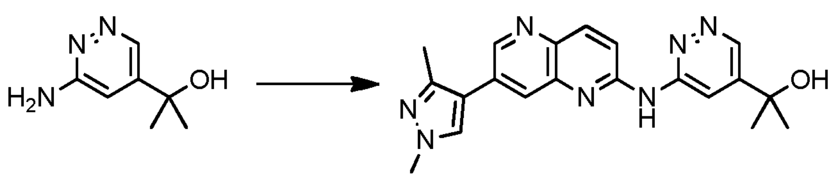

2-(6-((7-(1,3-Dimethyl-1H-pyrazol-4-yl)-1,5-naphthyridin-2-yl)amino)pyridazin-4-yl)propan-2-ol was obtained in the same manner as in Example 0543-2 except that 2-chloro-7-(1,3-dimethyl-1H-pyrazol-4-yl)-1,5-naphthyridine was used instead of the 2-chloro-7-(1-methyl-1H-pyrazol-4-yl)-1,5-naphthyridine used in Example 0543-2.
$^1$H-NMR($CDCl_3$/$CD_3OD$=4/1)δ:9.02(1H,brs),8.93(1H,brs),8.78(1H,d,J=1.8Hz),8.23(1H,d,J=9.3Hz),8.13( 1H,brs),7.76(1H,s),7.58(1H,d,J=9.3Hz),3.94(3H,s),2.49(3H,s),1.64(6H,s).

MSm/z(M+H):376.

<Example 0548>

<0548-1>

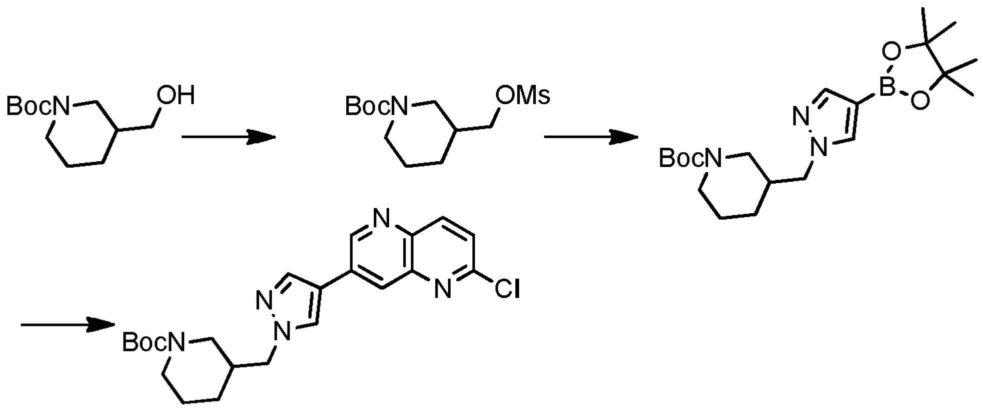

tert-Butyl 3-((4-(6-chloro-1,5-naphthyridin-3-yl)-1H-pyrazol-1-yl)methyl)piperidine-1-carboxylate was obtained in the same manner as in Example 0475-1 except that tert-butyl 3-(hydroxymethyl)piperidine-1-carboxylate was used instead of the tetrahydro-2H-pyran-4-ol used in Example 0475-1.
MSm/z(M+H):428.

<0548-2>

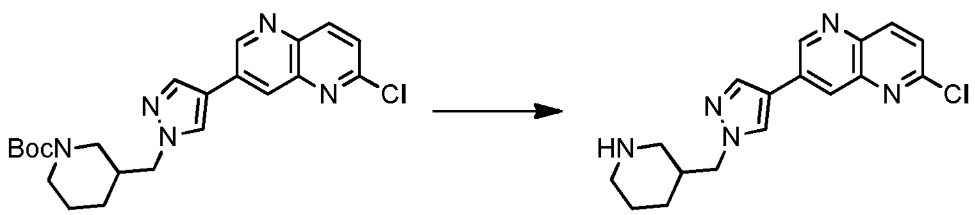

2-Chloro-7-(1-(piperidin-3-ylmethyl)-1H-pyrazol-4-yl)-1,5-naphthyridine was obtained in the same manner as in Example 0528-2 except that tert-butyl 3-((4-(6-chloro-1,5-naphthyridin-3-yl)-1H-pyrazol-1-yl)methyl)piperidine-1-carboxylate was used instead of the tert-butyl 4-((4-(6-chloro-1,5-naphthyridin-3-yl)-1H-pyrazol-1-yl)methyl)piperidine-1-carboxylate used in Example 0528-2.
MSm/z(M+H):328.

<0548-3>

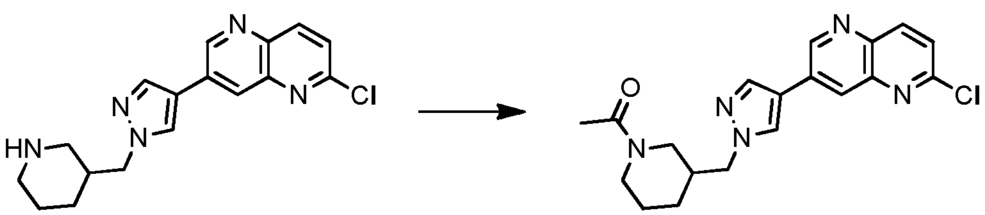

1-(3-((4-(6-Chloro-1,5-naphthyridin-3-yl)-1H-pyrazol-1-yl)methyl)piperidin-1-yl)ethanone was obtained in the

same manner as in Example 0528-3 except that 2-chloro-7-(1-(piperidin-3-ylmethyl)-1H-pyrazol-4-yl)-1,5-naphthyridine was used instead of the 2-chloro-7-(1-(piperidin-4-ylmethyl)-1H-pyrazol-4-yl)-1,5-naphthyridine used in Example 0528-3. MSm/z(M+H):370.

<0548-4>

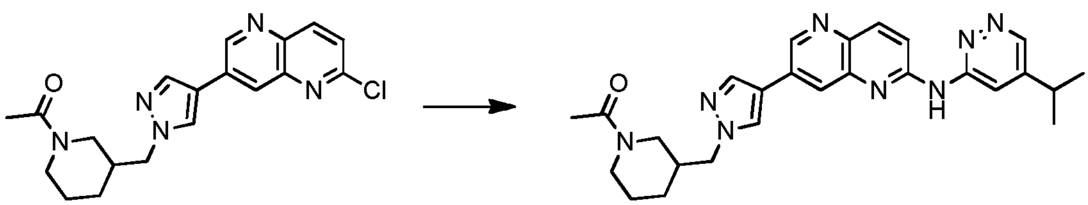

1-(3-((4-(6-((5-Isopropylpyridazin-3-yl)amino)-1,5-naphthyridin-3-yl)-1H-pyrazol-1-yl)methyl)piperidin-1-yl)ethanone was obtained in the same manner as in Example 0544-2 except that 1-(3-((4-(6-chloro-1,5-naphthyridin-3-yl)-1H-pyrazol-1-yl)methyl)piperidin-1-yl)ethanone was used instead of the (4-((4-(6-chloro-1,5-naphthyridin-3-yl)-1H-pyrazol-1-yl)methyl)-N-isopropylpiperidine-1-carboxamide used in Example 0544-2.
$^1$H-NMR($CDCl_3$)δ:8.92(1H,d,J=2.1Hz),8.90(1H,brs),8.82(1H,brs),8.24(1H,d,J=3.1Hz),8.10(1H,brs),7.98( 1H,d,J=11.1Hz), 7.86(1H,d,J=11.1Hz), 7.75-7.55(1H,m),4.33-4.03(3H,m),3.75-3.58(1H,m),3.24-2.74(3H,m),2.29-2.14(1H,m),2.12(3H,s),1.94-1.70(2H,m),1.43(6H,d,J=6.6Hz),1.40-1.25(2H,m).
MSm/z(M+H):471.

<Example 0549>

<0549-1>

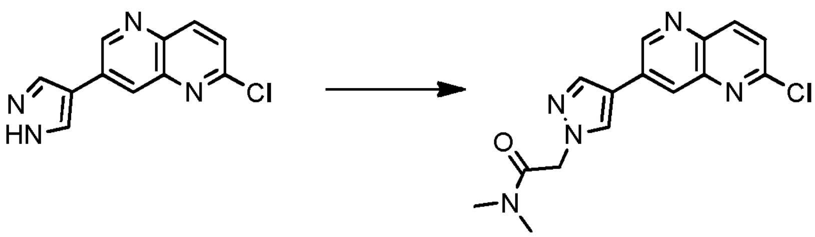

A suspension of 2-chloro-7-(1H-pyrazol-4-yl)-1,5-naphthyridine (20 mg), 2-chloro-N,N-dimethylacetamide (0.013 mL), and cesium carbonate (56 mg) in 1,4-dioxane (1 mL) was stirred at 80°C for 4 hours. The reaction mixture was cooled to room temperature, and purified by silica gel column chromatography (methanol-ethyl acetate), thereby obtaining 2-(4-(6-chloro-1,5-naphthyridin-3-yl)-1H-pyrazol-1-yl)-N,N-dimethylacetamide (20 mg).
MSm/z(M+H):316.

<0549-2>

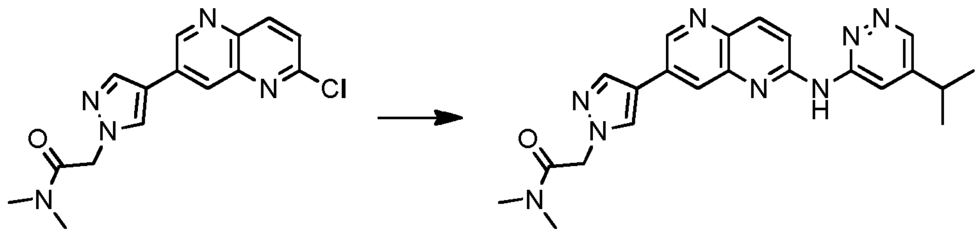

2-(4-(6-((5-Isopropylpyridazin-3-yl)amino)-1,5-naphthyridin-3-yl)-1H-pyrazol-1-yl)-N,N-dimethylacetamide was obtained in the same manner as in Example 0544-2 except that 2-(4-(6-chloro-1,5-naphthyridin-3-yl)-1H-pyrazol-1-yl)-N,N-dimethylacetamide was used instead of the (4-((4-(6-chloro-1,5-naphthyridin-3-yl)-1H-pyrazol-1-yl)methyl)-N-isopropylpiperidine-1-carboxamide used in Example 0544-2.
$^1$H-NMR($CDCl_3$)δ:8.94(1H,d,J=2.1Hz),8.88(1H,brs),8.81(1H,brs),8.24(1H,d,J=9.3Hz),8.11(1H,brs),8.02( 1H,s),7.99(1H,s),7.52(1H,d,J=9.3Hz),5.09(2H,s),3.16(3H,s),3.14-3.01(1H,m),3.04(3H,s),1.42(6H,d,J=7.5Hz).
MSm/z(M+H):417.

<Example 0550>

<0550-1>

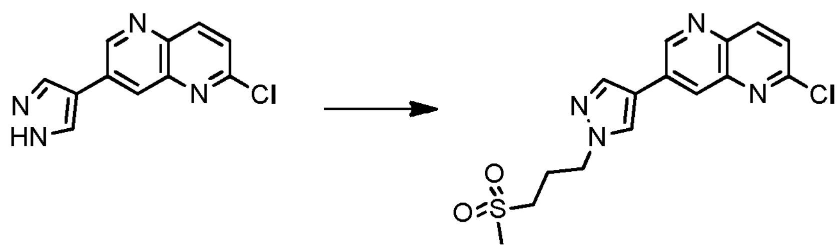

2-Chloro-7-(1-(3-(methylsulfonyl)propyl)-1H-pyrazol-4-yl)-1,5-naphthyridine was obtained in the same manner as in Example 0549-1 except that 3-(methylsulfonyl)propyl 4-methylbenzenesulfonate was used instead of the 2-chloro-N,N-dimethylacetamide used in Example 0549-1.
MSm/z(M+H):351.

<0550-2>

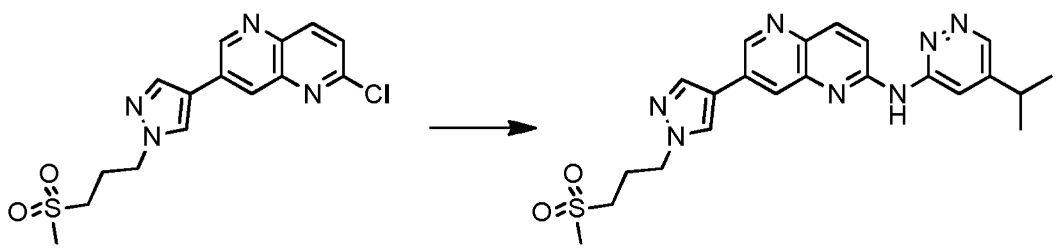

N-(5-isopropylpyridazin-3-yl)-7-(1-(3-(methylsulfonyl)propyl)-1H-pyrazol-4-yl)-1,5-naphthyridine-2-amine was obtained in the same manner as in Example 0544-2 except that 2-chloro-7-(1-(3-(methylsulfonyl)propyl)-1H-pyrazol-4-yl)-1,5-naphthyridine was used instead of the (4-((4-(6-chloro-1,5-naphthyridin-3-yl)-1H-pyrazol-1-yl)methyl)-N-isopropylpiperidine-1-carboxamide used in Example 0544-2.
$^1$H-NMR(DMSO-$d_6$)δ:10.71(1H,brs),9.05(1H,d,J=2.1Hz),8.87(1H,d,J=2.1Hz),8.74(1H,brs),8.54(1H,s),8.24(1H,d,J=2.1Hz),8.22(1H,d,J=9.0Hz),7.70(1H,d,J=9.0Hz),4.32(2H,t,J=6.9Hz),3.23-3.13(1H,m),3.00(3H,s),2.78-2.70(2H,m),2.33-2.22(2H,m),1.33(6H,d,J=7.2Hz).

MSm/z(M+H):452.

<Example 0551>

<0551-1>

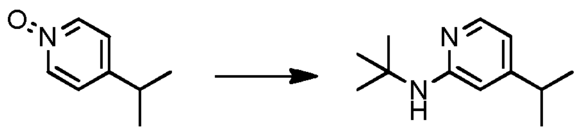

Paratoluenesulfonyl chloride (2.66 g) was added to a mixture of 4-isopropylpyridine 1-oxide (0.96 g), tert-butylamine (7.3 mL), and chloroform (35 mL) under ice-cooling, followed by stirring at room temperature for 4 hours. A4 mol/L sodium hydroxide aqueous solution was added to the reaction mixture, the organic layer was collected by separation, and dried over anhydrous sodium sulfate, and the solvent was distilled off under reduced pressure. The obtained residue was purified by silica gel column chromatography (methanol-ethyl acetate-hexane), thereby obtaining N-(tert-butyl)-4-isopropylpyridine-2-amine (931 mg).
MSm/z(M+H):193.

<0551-2>

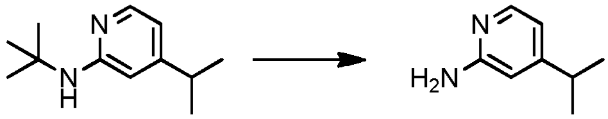

Trifluoroacetic acid (5 mL) was added to a mixture of N-(tert-butyl)-4-isopropylpyridine-2-amine (931 mg) and water (0.25 mL), followed by stirring at room temperature for 2 hours. The solvent was distilled off under reduced pressure, and the obtained residue was purified by silica gel column chromatography (methanol-ethyl acetate), thereby obtaining 4-isopropylpyridine-2-amine (475 mg).
MSm/z(M+H):137.

<0551-3>

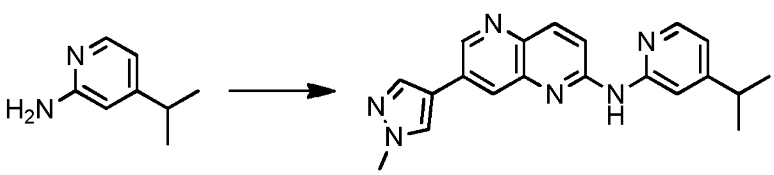

N-(4-isopropylpyridin-2-yl)-7-(1-methyl-1H-pyrazol-4-yl)-1,5-naphthyridine-2-amine was obtained in the same manner as in Example 0489-4 except that 4-isopropylpyridine-2-amine was used instead of the 4-methylpyridazine-3-amine used in Example 0489-4.
$^{1}$H-NMR($CDCl_3$)$\delta$:8.89(1H,d,J=1.8Hz),8.22-8.09(4H,m),7.94(1H,s),7.82(1H,s),7.52(1H,d,J=8.7Hz),6.86(1H,dd,J=5.1,1.8Hz),4.02(3H,s),3.07-2.92(1H,m),1.34(6H,d,J=7.2Hz).
MSm/z(M+H):345.

<Example 0552>

<0552-1>

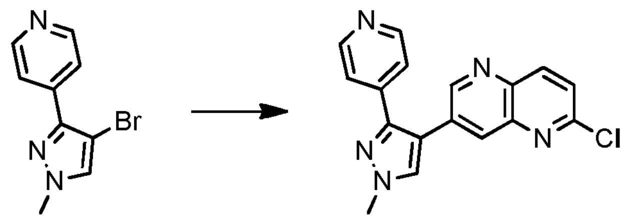

2-Chloro-7-(1-methyl-3-(pyridin-4-yl)-1H-pyrazol-4-yl)-1,5-naphthyridine was obtained in the same manner as in Example 0478-3 except that 4-(4-bromo-1-methyl-1H-pyrazol-3-yl)pyridine was used instead of the 1-(4-(4-iodo-1H-pyrazol-1-yl)piperidin-1-yl)ethanone used in Example 0478-3.
MSm/z(M+H):322.

<0552-2>

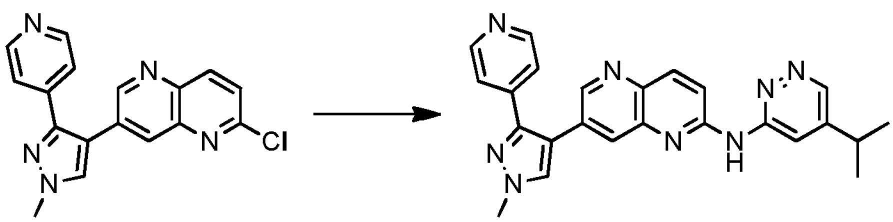

N-(5-isopropylpyridazin-3-yl)-7-(1-methyl-3-(pyridin-4-yl)-1H-pyrazol-4-yl)-1,5-naphthyridine-2-amine was obtained in the same manner as in Example 0544-2 except that 2-chloro-7-(1-methyl-3-(pyridin-4-yl)-1H-pyrazol-4-yl)-1,5-naphthyridine was used instead of the 4-((4-(6-chloro-1,5-naphthyridin-3-yl)-1H-pyrazol-1-yl)methyl)-N-isopropylpiperidine-1-carboxamide used in Example 0544-2.
$^1$H-NMR($CDCl_3$)δ:9.86(1H,brs),8.89(1H,brs),8.81(1H,d,J=2.1Hz),8.68(1H,d,J=2.1Hz),8.57(2H,dd,J=4.5, 1.8Hz),8.27(1H,d,J=9.3Hz),7.97(1H,d,J=2.1Hz),7.81(1H,d,J=9.3Hz),7.69(1H,s),7.45(2H,d,,J=4.5,1.8 Hz),4.07(3H,s),3.08-2.92(1H,m),1.34(6H,d,J=7.2Hz).
MSm/z(M+H):423.

<Example 0553>

<0553-1>

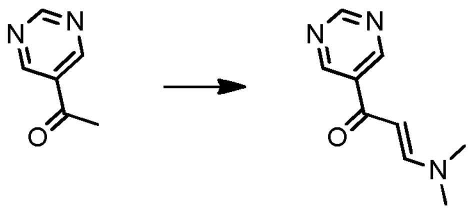

A mixture of 1-(pyrimidin-5-yl)ethanone (525 mg) and N,N-dimethylformamide dimethyl acetal (3 mL) was stirred for 2 hours under heating to reflux. The reaction mixture was cooled to room temperature, and the solvent was distilled off under reduced pressure. tert-Butyl methyl ether was added to the obtained residue, and the solid matter was collected by filtration, thereby obtaining (E)-3-(dimethylamino)-1-(pyrimidin-5-yl)prop-2-en-1-one (657 mg).

MSm/z(M+H):178.

<0553-2>

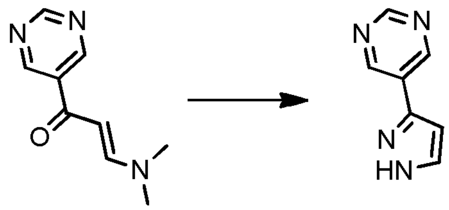

Hydrazine monohydrate (0.198 mL) was added to a mixture of (E)-3-(dimethylamino)-1-(pyrimidin-5-yl)prop-2-en-1-one (657 mg) and ethanol (4 mL), followed by stirring at room temperature for 1 hour. The reaction mixture was distilled off under reduced pressure, thereby obtaining 5-(1H-pyrazol-3-yl)pyrimidine (711 mg).
MSm/z(M+H):147.

<0553-3>

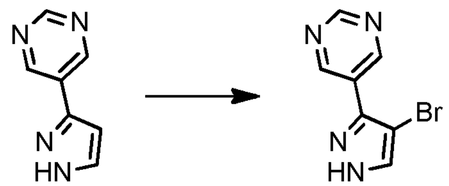

N-bromosuccinimide (724 mg) was added to a solution of 5-(1H-pyrazol-3-yl)pyrimidine (711 mg) in N,N-dimethylformamide (7 mL) under ice-cooling, followed by stirring at the same temperature for 1 hour. After ethyl acetate and water were added to the reaction mixture, the organic layer was collected by separation, washed sequentially with water and a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining 5-(4-bromo-1H-pyrazol-3-yl)pyrimidine (63 mg).
MSm/z(M+H):225.

<0553-4>

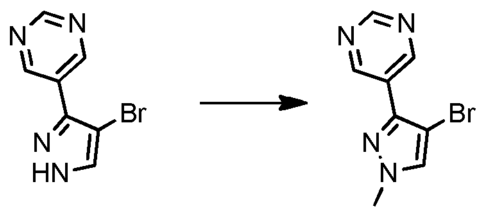

60% sodium hydride (34 mg) was added to a solution of 5-(4-bromo-1H-pyrazol-3-yl)pyrimidine (63 mg) and iodomethane (0.035 mL) in N,N-dimethylformamide (2 mL) under ice-cooling, followed by stirring at room temperature for 1 hour. After ethyl acetate and water were added to the reaction mixture, the organic layer was collected by separation, washed sequentially with water and a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining 5-(4-bromo-1-methyl-1H-pyrazol-3-yl)pyrimidine (34 mg).
MSm/z(M+H):239.

<0553-5>

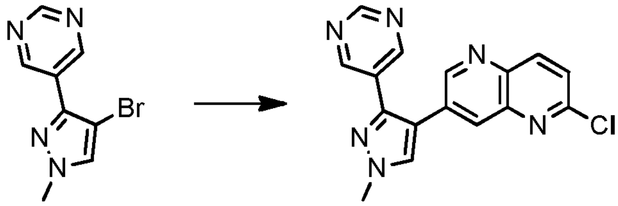

2-Chloro-7-(1-methyl-3-(pyrimidin-5-yl)-1H-pyrazol-4-yl)-1,5-naphthyridine was obtained in the same manner as in Example 0478-3 except that 5-(4-bromo-1-methyl-1H-pyrazol-3-yl)pyrimidine was used instead of the 1-(4-(4-iodo-1H-pyrazol-1-yl)piperidin-1-yl)ethanone used in Example 0478-3.
MSm/z(M+H):323.

<0553-6>

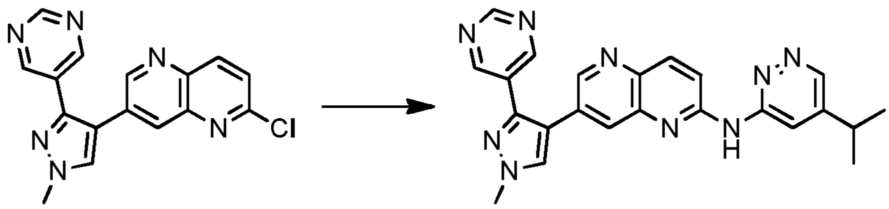

N-(5-isopropylpyridazin-3-yl)-7-(1-methyl-3-(pyrimidin-5-yl)-1H-pyrazol-4-yl)-1,5-naphthyridine-2-amine was obtained in the same manner as in Example 0544-2 except that 2-chloro-7-(1-methyl-3-(pyrimidin-5-yl)-1H-pyrazol-4-yl)-1,5-naphthyridine was used instead of the 4-((4-(6-chloro-1,5-naphthyridin-3-yl)-1H-pyrazol-1-yl)methyl)-N-isopropylpiperidine-1-carboxamide used in Example 0544-2.
$^1$H-NMR(DMSO-$d_6$)δ:10.76(1H,brs),9.18(1H,s),8.87(2H,s),8.83(1H,d,J=1.8Hz),8.71(1H,d,J=1.8Hz),8.64(1H,d,J=1.8Hz ),8.41(1H,s),8.25(1H,d,J=9.3Hz),7.85(1H,d,J=1.8Hz),7.74(1H,d,J=9.3Hz),4.02(3H,s),3.04-2.88(1H,m),1.23(6H,d,J=7.2Hz).
MSm/z(M+H):424.

<Example 0554>

<0554-1>

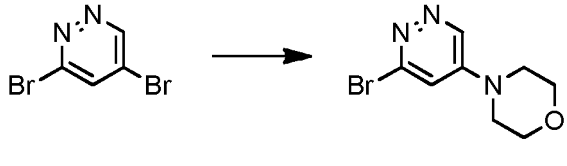

4-(6-Bromopyridazin-4-yl)morpholine was obtained in the same manner as in Example 0517-1 except that morpholine was used instead of the diethylamine used in Example 0517-1.
MSm/z(M+H):244.

<0554-2>

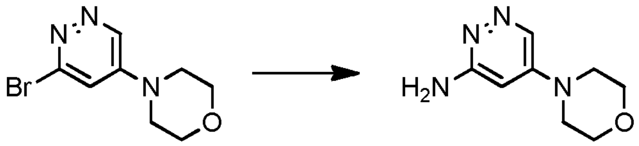

5-Morpholinopyridazine-3-amine was obtained in the same manner as in Example 0495-5 except that 4-(6-bromopyridazin-4-yl)morpholine was used instead of the 3-bromo-5-methoxypyridazine used in Example 0495-5. MSm/z(M+H):181.

<0554-3>

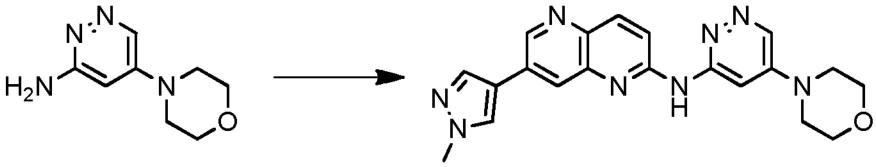

A suspension of 2-chloro-7-(1-methyl-1H-pyrazol-4-yl)-1,5-naphthyridine (13 mg), 5-morpholinopyridazine-3-amine (5.6 mg), sodiumtert-amyl oxide (10 mg), and anhydrous sodium sulfate (50 mg) in 1,4-dioxane (1 mL) was stirred at 110°C for 2 hours. The reaction mixture was cooled to room temperature, and purified by silica gel column chromatography (methanol-ethyl acetate, NH silica), thereby obtaining 7-(1-methyl-1H-pyrazol-4-yl)-N-(5-morpholinopyridazin-3-yl)-1,5-naphthyridine-2-amine (6.6 mg).
$^1$H-NMR($CDCl_3$/$CD_3OD$=4/1)δ:8.86(1H,d,J=2.1Hz),8.52(1H,d,J=2.1Hz),8.31(1H,brs),8.18(1H,d,J=8.7Hz ),8.08(1H,d,J=2.1Hz),7.94(2H,s),7.47(1H,d,J=8.7Hz),4.03(3H,s),3.95(4H,t,J=5.1Hz),3.52(4H,t,J=5.1H z).
MSm/z(M+H):389.

<Example 0555>

<0555-1>

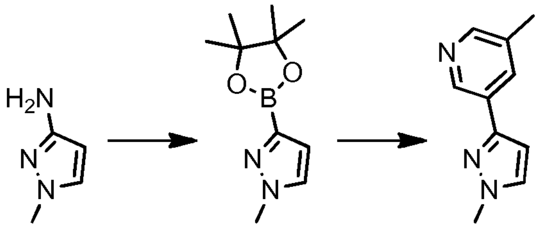

A mixture of 1-methyl-1H-pyrazole-3-amine (200 mg), bis(pinacolato)diboron (575 mg), tert-butyl nitrite (0.364 mL), and acetonitrile (5 mL) was stirred for 2 hours under heating to reflux. After the reaction mixture was cooled to room temperature, a 10% sodium hydrogen sulfite aqueous solution was added thereto, the organic layer was collected by separation, washed with a saturated sodium chloride aqueous solution, and dried over anhydrous sodium sulfate, and the solvent was distilled off under reduced pressure, thereby obtaining 1-methyl-3-(4,4,5,5-tetramethyl-1,3,2-dioxaborolan-2-yl)-1H-pyrazole (834 mg).

A mixture of the obtained 1-methyl-3-(4,4,5,5-tetramethyl-1,3,2-dioxaborolan-2-yl)-1H-pyrazole (834 mg), 3-bromo-5-methylpyridine (0.238 mL), sodium carbonate (543 mg), bis(di-tert-butyl (4-dimethylaminophenyl)phosphine)dichloropalladium(II) (72 mg), water (1 mL), and 1,4-dioxane (10 mL) was stirred at 100°C for 3 hours. The reaction mixture was cooled to room temperature, the insolubles were filtered off, and the obtained residue was purified by silica gel column chromatography (methanol-ethyl acetate), thereby obtaining 3-methyl-5-(1-methyl-1H-pyrazol-3-yl)pyridine (53 mg).

MSm/z(M+H):174.

<0555-2>

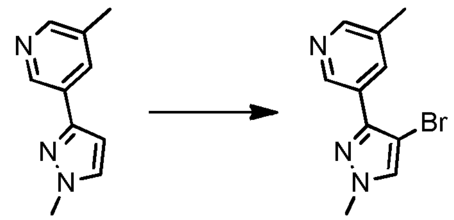

N-bromosuccinimide (60 mg) was added to a solution of 3-methyl-5-(1-methyl-1H-pyrazol-3-yl)pyridine (53 mg) in N,N-dimethylformamide (1.5 mL), followed by stirring at room temperature for 1 hour. Ethyl acetate and a 10% sodium hydrogen sulfite aqueous solution were added to the reaction mixture. The organic layer was collected by separation, washed sequentially with water and a saturated sodium chloride aqueous solution, and dried over anhydrous sodium sulfate, and the solvent was distilled off under reduced pressure. The obtained residue was purified by silica gel column chromatography (methanol-ethyl acetate), thereby obtaining 3-(4-bromo-1-methyl-1H-pyrazol-3-yl)-5-methylpyridine (35 mg).
MSm/z(M+H):252.

<0555-3>

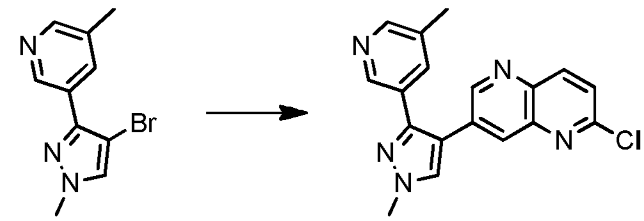

2-Chloro-7-(1-methyl-3-(5-methylpyridin-3-yl)-1H-pyrazol-4-yl)-1,5-naphthyridine was obtained in the same manner as in Example 0478-3 except that 3-(4-bromo-1-methyl-1H-pyrazol-3-yl)-5-methylpyridine was used instead of the 1-(4-(4-iodo-1H-pyrazol-1-yl)piperidin-1-yl)ethanone used in Example 0478-3.
MSm/z(M+H):336.

<0555-4>

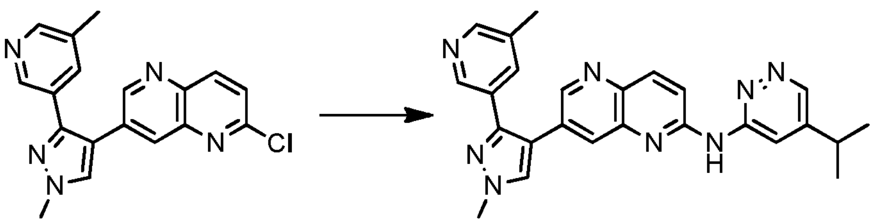

A suspension of 2-chloro-7-(1-methyl-3-(5-methylpyridin-3-yl)-1H-pyrazol-4-yl)-1,5-naphthyridine (13 mg), 5-isopropylpyridazine-3-amine (7.9 mg), sodium tert-amyl oxide (19 mg), and anhydrous sodium sulfate (50 mg) in 1,4-dioxane (1 mL) was stirred at 110°C for 1 hour. The reaction mixture was cooled to room temperature, and purified by silica gel column chromatography (methanol-ethyl acetate, NH silica), thereby obtaining N-(5-isopropylpyridazin-3-yl)-7-(1-methyl-3-(5-methylpyridin-3-yl)-1H-pyrazol-4-yl)-1,5-naphthyridine-2-amine (10 mg).
$^{1}$H-NMR($CDCl_3$)$\delta$:10.35(1H,brs),8.94(1H,brs),8.80(1H,brs),8.66(1H,d,J=2.1Hz),8.52(1H,d,J=2.1Hz),8.40

(1H,d,J=2.1Hz),8.24(1H,d,J=9.0Hz),7.92(1H,d,J=2.1Hz),7.88(1H,d,J=9.0Hz),7.71(2H,brs),4.06(3H,s), 3.06-2.92(1H,m),2.31(3H,s),1.34(6H,d,J=7.2Hz).
MSm/z(M+H):437.

<Example 0556>

<0556-1>

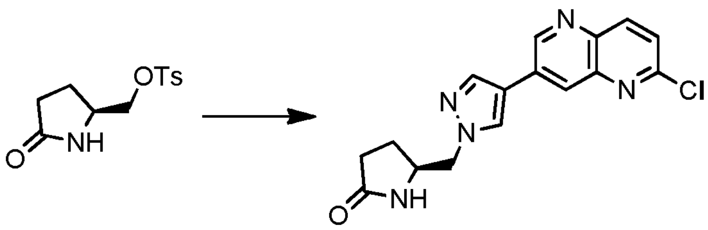

(S)-5-((4-(6-chloro-1,5-naphthyridin-3-yl)-1H-pyrazol-1-yl)methyl)pyrrolidin-2-one was obtained in the same manner as in Example 0549-1 except that (S)-(5-oxopyrrolidin-2-yl)methyl 4-methylbenzenesulfonate was used instead of the 2-chloro-N,N-dimethylacetamide used in Example 0549-1.
MSm/z(M+H):328.

<0556-2>

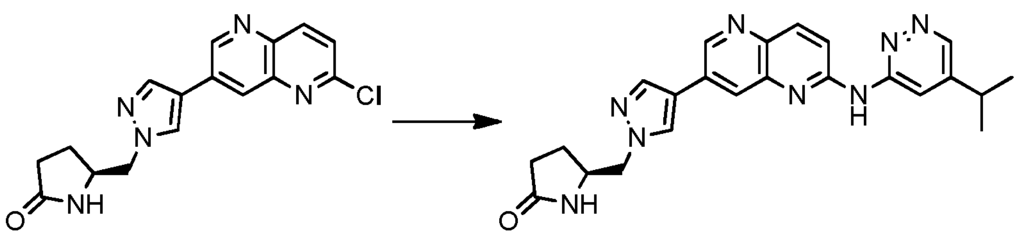

(S)-5-((4-(6-((5-isopropylpyridazin-3-yl)amino)-1,5-naphthyridin-3-yl)-1H-pyrazol-1-yl)methyl)pyrrolidin-2-one was obtained in the same manner as in Example 0555-4 except that (S)-5-((4-(6-chloro-1,5-naphthyridin-3-yl)-1H-pyrazol-1-yl)methyl)pyrrolidin-2-one was used instead of the 2-chloro-7-(1-methyl-3-(5-methylpyridin-3-yl)-1H-pyrazol-4-yl)-1,5-naphthyridine used in Example 0555-4.
$^1$H-NMR($CDCl_3$/$CD_3OD$=4/1)δ:8.85(2H,brs),8.72(1H,brs),8.20(1H,d,J=9.3Hz),8.15(1H,brs),8.04(1H,s),8.02(1H,s),7.51(1H,d,J=9.3Hz),4.38-4.13(3H,m),3.15-2.98(1H,m),2.42-1.94(4H,m),1.42(6H,d,J=7.2Hz).
MSm/z(M+H):429.

<Example 0557>

<0557-1>

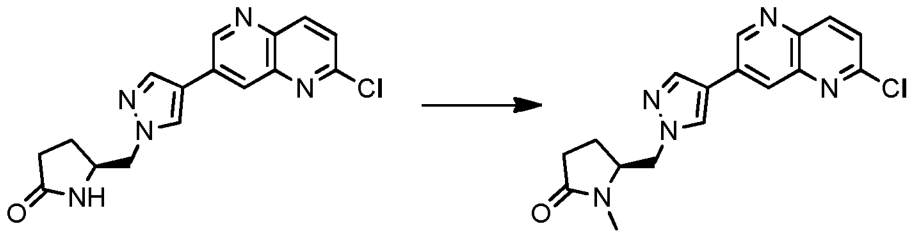

60% sodium hydride (16 mg) was added to a solution of (S)-5-((4-(6-chloro-1,5-naphthyridin-3-yl)-1H-pyrazol-1-yl)methyl)pyrrolidin-2-one (31 mg) and iodomethane (0.012 mL) in N,N-dimethylformamide (1 mL) under ice-cooling, followed by stirring at the same temperature for 1 hour. After ethyl acetate and water were added to the reaction mixture, the organic layer was collected by separation, washed sequentially with water and a saturated sodium chloride aqueous solution, and dried over anhydrous sodium sulfate, and the solvent was distilled off under reduced pressure. The obtained residue was purified by silica gel column chromatography (ethyl acetate-hexane, NH silica), thereby obtaining (S)-5-((4-(6-chloro-1,5-naphthyridin-3-yl)-1H-pyrazol-1-yl)methyl)-1-methylpyrrolidin-2-one (25 mg).
MSm/z(M+H):342.

<0557-2>

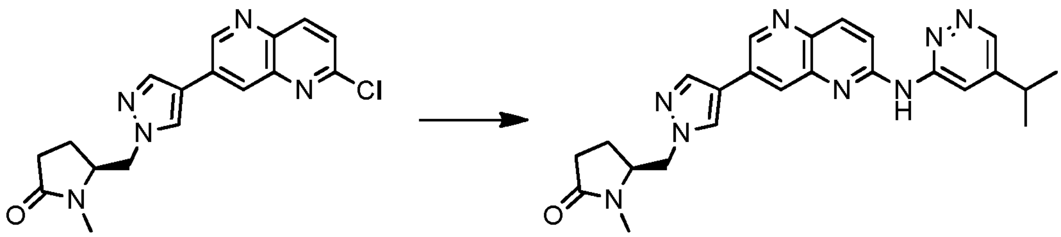

(S)-5-((4-(6-((5-isopropylpyridazin-3-yl)amino)-1,5-naphthyridin-3-yl)-1H-pyrazol-1-yl)methyl)-1-methylpyrrolidin-2-one was obtained in the same manner as in Example 0555-4 except that (S)-5-((4-(6-chloro-1,5-naphthyridin-3-yl)-1H-pyrazol-1-yl)methyl)-1-methylpyrrolidin-2-one was used instead of the 2-chloro-7-(1-methyl-3-(5-methylpyridin-3-yl)-1H-pyrazol-4-yl)-1,5-naphthyridine used in Example 0555-4.
$^1$H-NMR($CDCl_3$/$CD_3OD$=4/1)δ:8.88(2H,brs),8.73(1H,brs),8.21(1H,d,J=9.3Hz),8.16(1H,d,J=1.8Hz),8.03( 1H,s),7.99(1H,s), 7.52(1H,d,J=9.3Hz),4.41(2H,d,J=4.5Hz),4.17-4.06(1H,m),3.16-3.00(1H,m),2.89(3H,s),2.35-1.96(4H,m),1.42(6H,d,J=6 .6Hz).
MSm/z(M+H):443.

<Example 0558>

<0558-1>

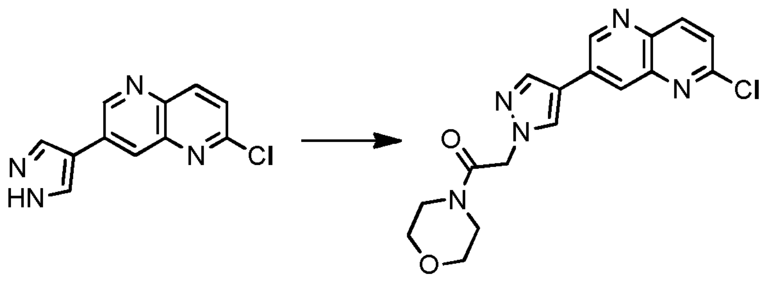

2-(4-(6-Chloro-1,5-naphthyridin-3-yl)-1H-pyrazol-1-yl)-1-morpholinoethanone was obtained in the same manner as in Example 0549-1 except that 2-chloro-1-morpholinoethanone was used instead of the 2-chloro-N,N-dimethylacetamide used in Example 0549-1.
MSm/z(M+H):358.

<0558-2>

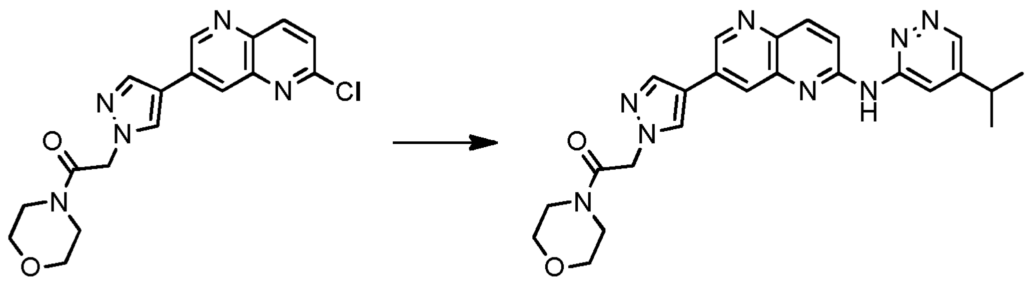

2-(4-(6-((5-Isopropylpyridazin-3-yl)amino)-1,5-naphthyridin-3-yl)-1H-pyrazol-1-yl)-1-morpholinoethanone was obtained in the same manner as in Example 0471-2 except that 2-(4-(6-chloro-1,5-naphthyridin-3-yl)-1H-pyrazol-1-yl)-1-morpholinoethanone was used instead of the 2-chloro-7-(1-(2-methoxyethyl)-1H-pyrazol-4-yl)-1,5-naphthyridine used in Example 0471-2.
$^1$H-NMR($CDCl_3$)$\delta$:9.04(1H,brs),8.94(1H,d,J=2.1Hz),8.88(1H,d,J=2.1Hz),8.82(1H,d,J=2.1Hz),8.24(1H,d,J =9.0Hz),8.12(1H,d,J=2.1Hz),8.01(1H,s),8.00(1H,s),7.56(1H,d,J=9.0Hz),5.09(2H,s),3.76-3.60(8H,m),3.14-3.00(1H,m), 1.42(6H,d,J=6.6Hz).
MSm/z(M+H):459.

<Example 0559>

<0559-1>

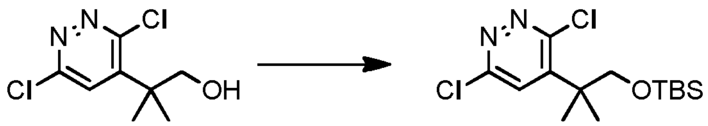

A mixture of 2-(3,6-dichloropyridazin-4-yl)-2-methylpropan-1-ol (969 mg), tert-butyldimethylchlorosilane (726 mg), imidazole (745 mg), N,N-dimethylpyridine-4-amine (53 mg), and N,N-dimethylformamide (8 mL) was stirred at room temperature for 16 hours. After ethyl acetate and water were added to the reaction mixture, the organic layer was collected by separation, washed sequentially with water and a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining 4-(1-((tert-butyldimethylsilyl)oxy)-2-methylpropan-2-yl)-3,6-dichloropyridazine (981 mg).
MSm/z(M+H):335.

<0559-2>

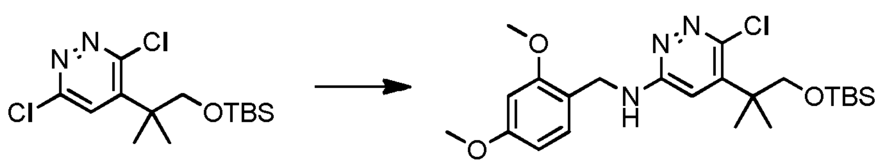

A mixture of 4-(1-((tert-butyldimethylsilyl)oxy)-2-methylpropan-2-yl)-3,6-dichloropyridazine (981 mg), 2,4-dimethoxybenzylamine (0.526 mL), 1,8-diazabicyclo[5.4.0]undeca-7-ene (0.654 mL), and toluene (3 mL) was stirred for 12 hours under heating to reflux. The reaction mixture was cooled to room temperature, ethyl acetate and a saturated sodium chloride aqueous solution were added thereto, and the organic layer was collected by separation. The organic layer was dried over anhydrous sodium sulfate, and the solvent was distilled off under reduced pressure. The obtained residue was purified by silica gel column chromatography (ethyl acetate-hexane), thereby obtaining 5-(1-((tert-butyldimethylsilyl)oxy)-2-methylpropan-2-yl)-6-chloro-N-(2,4-dimethoxybenzyl)pyridazine-3-amine (380 mg).
MSm/z(M+H):466.

<0559-3>

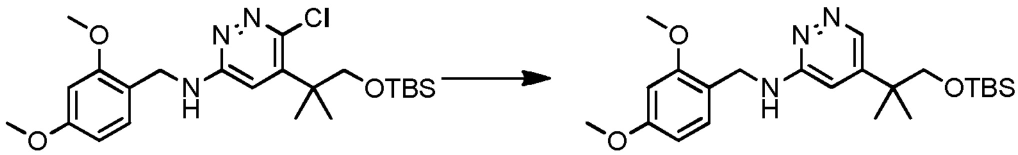

A mixture of 5-(1-((tert-butyldimethylsilyl)oxy)-2-methylpropan-2-yl)-6-chloro-N-(2,4-dimethoxybenzyl)pyridazine-3-amine (380 mg), 10% palladium/carbon (50 mg), ammonium formate (256 mg), ammonium chloride (217 mg), and methanol (4 mL) was stirred for 3 hours under heating to reflux. After the reaction mixture was cooled to room temperature, ethyl acetate was added thereto, the insolubles were filtered off, and the solvent was distilled off under reduced pressure. The obtained residue was purified by silica gel column chromatography (ethyl acetate-hexane), thereby obtaining 5-(1-((tert-butyldimethylsilyl)oxy)-2-methylpropan-2-yl)-N-(2,4-dimethoxybenzyl)pyridazine-3-amine (204 mg).
MSm/z(M+H):432.

<0559-4>

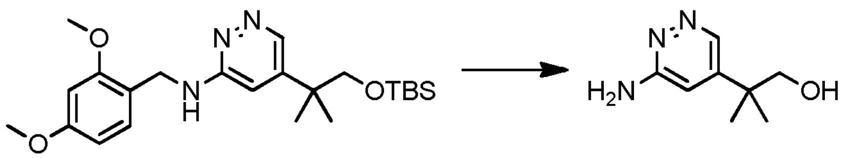

Trifluoroacetic acid (2 mL) was added to a mixture of 5-(1-((tert-butyldimethylsilyl)oxy)-2-methylpropan-2-yl)-N-(2,4-dimethoxybenzyl)pyridazine-3-amine (90 mg), and water (0.2 mL), followed by stirring at room temperature for 1 hour. The solvent was distilled off under reduced pressure, and the obtained residue was purified by silica gel column chromatography (methanol-ethyl acetate-hexane, NH silica), thereby obtaining 2-(6-aminopyridazin-4-yl)-2-methylpropan-1-ol (30 mg).
MSm/z(M+H):168.

<0559-5>

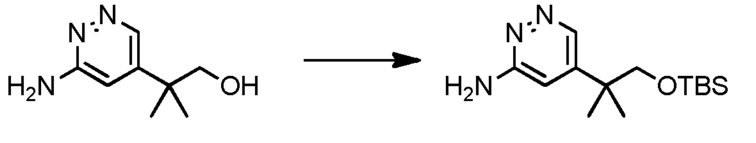

A mixture of 2-(6-aminopyridazin-4-yl)-2-methylpropan-1-ol (23 mg), tert-butyldimethylchlorosilane (11 mg), imidazole (23 mg), and N,N-dimethylformamide (2 mL) was stirred at room temperature for 15 hours. After ethyl acetate and water were added to the reaction mixture, the organic layer was collected by separation, washed sequentially with water and a saturated sodium chloride aqueous solution, and dried over anhydrous sodium sulfate, and the solvent was distilled off under reduced pressure. The obtained residue was purified by silica gel column chromatography (ethyl

acetate-hexane), thereby obtaining 5-(1-((tert-butyldimethylsilyl)oxy)-2-methylpropan-2-yl)pyridazine-3-amine (34 mg). MSm/z(M+H):282.

<0559-6>

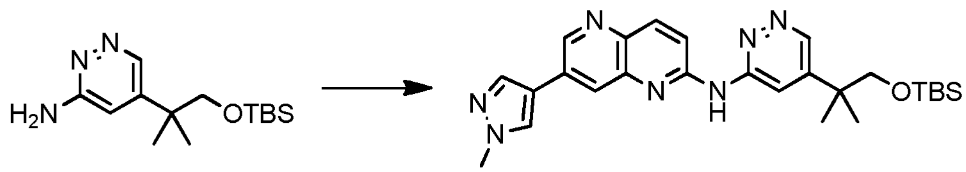

N-(5-(1-((tert-butyldimethylsilyl)oxy)-2-methylpropan-2-yl)pyridazin-3-yl)-7-(1-methyl-1H-pyrazol-4-yl)-1,5-naphthyridine-2-amine was obtained in the same manner as in Example 0554-3 except that 5-(1-((tert-butyldimethylsilyl)oxy)-2-methylpropan-2-yl)pyridazine-3-amine was used instead of the 5-morpholinopyridazine-3-amine used in Example 0554-3.
$^1$H-NMR($CDCl_3$)δ:9.11(1H,brs),8.98(1H,brs),8.96(1H,brs),8.92(1H,d,J=2.1Hz),8.24(1H,d,J=9.3Hz),8.06( 1H,d,J=2.1Hz),7.93(1H,s),7.81(1H,s),7.60(1H,d,J=9.3Hz),4.02(3H,s),3.68(2H,s),1.45(6H,s),0.82(9H,s ),0.01(6H,s).
MSm/z(M+H):490.

<Example 0560>

<0560-1>

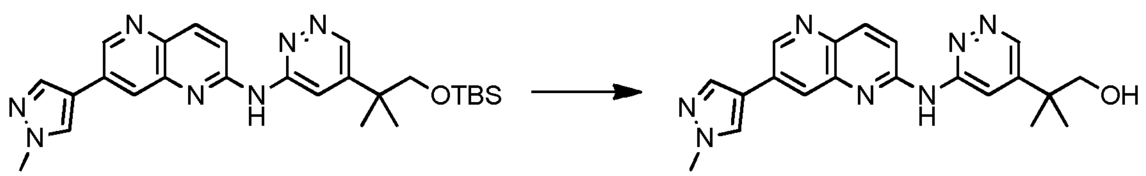

Concentrated hydrochloric acid (0.4 mL) was added to a mixture of N-(5-(1-((tertbutyldimethylsilyl)oxy)-2-methylpropan-2-yl)pyridazin-3-yl)-7-(1-methyl-1H-pyrazol-4-yl)-1,5-naphthyridine-2-amine (18 mg) and methanol (2.8 mL), followed by stirring at room temperature for 1 hour. The solvent of the reaction mixture was distilled off under reduced pressure, the solid matter was collected by filtration, and washed with ethyl acetate, thereby obtaining 2-methyl-2-(6-((7-(1-methyl-1H-pyrazol-4-yl)-1,5-naphthyridin-2-yl)amino)pyridazin-4-yl)propan-1-ol (15 mg).
$^1$H-NMR(DMSO-$d_6$)δ:9.17(1H,d,J=2.1Hz),9.13(1H,brs),8.59(1H,brs),8.48(1H,s),8.41(1H,d,J=9.0Hz),8.38(1H,s),8.17(1H,s),7.69(1H,d,J=9.0Hz),3.94(3H,s),3.58(2H,s),4.35(6H,s).
MSm/z(M+H):376.

<Example 0561>

<0561-1>

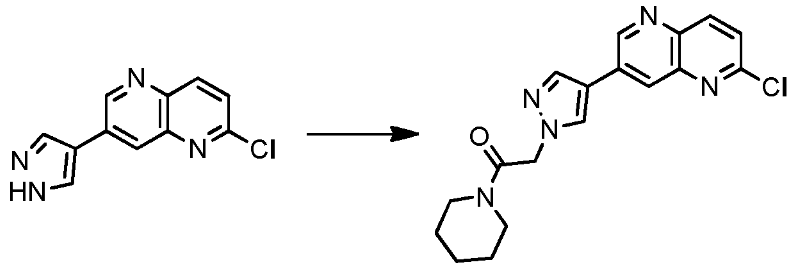

2-(4-(6-Chloro-1,5-naphthyridin-3-yl)-1H-pyrazol-1-yl)-1-(piperidin-1-yl)ethanone was obtained in the same manner as in Example 0549-1 except that 2-chloro-1-(piperidin-1-yl)ethanone was used instead of the 2-chloro-N,N-dimethylacetamide used in Example 0549-1.
MSm/z(M+H):356.

<0561-2>

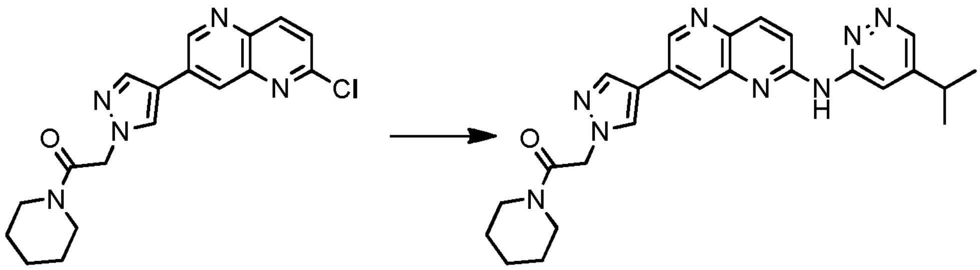

2-(4-(6-((5-Isopropylpyridazin-3-yl)amino)-1,5-naphthyridin-3-yl)-1H-pyrazol-1-yl)-1-(piperidin-1-yl)ethanone was obtained in the same manner as in Example 0471-2 except that 2-(4-(6-chloro-1,5-naphthyridin-3-yl)-1H-pyrazol-1-yl)-1-(piperidin-1-yl)ethanone was used instead of the 2-chloro-7-(1-(2-methoxyethyl)-1H-pyrazol-4-yl)-1,5-naphthyridine used in Example 0471-2.
$^1$H-NMR($CDCl_3$)δ:8.95(1H,d,J=1.8Hz),8.84(1H,brs),8.81(1H,brs),8.24(1H,d,J=9.0Hz),8.12(1H,brs),8.02( 1H,s),8.00(1H,s),7.45(1H,d,J=9.0Hz),5.09(2H,s),3.61(2H,t,J=5.4Hz),3.52(2H,t,J=5.4Hz),3.14-2.98(1H,m),1.75-1.54(6H,m),1.42(6H,d,J=7.2Hz).
MSm/z(M+H):457.

<Example 0562>

<0562-1>

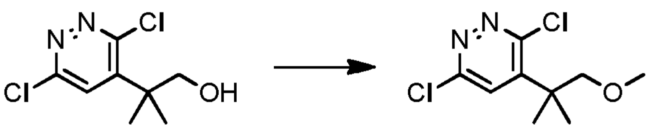

60% sodium hydride (72 mg) was added to a solution of 2-(3,6-dichloropyridazin-4-yl)-2-methylpropan-1-ol (200 mg) and iodomethane (0.112 mL) in N,N-dimethylformamide (5 mL) under ice-cooling, followed by stirring at the same temperature for 2 hours. After ethyl acetate and water were added to the reaction mixture, the organic layer was collected by separation, washed sequentially with water and a saturated sodium chloride aqueous solution, and dried over anhydrous sodium sulfate, and the solvent was distilled off under reduced pressure. The obtained residue was purified by silica gel column chromatography (ethyl acetate-hexane, NH silica), thereby obtaining 3,6-dichloro-4-(1-methoxy-2-methylpropan-2-yl)pyridazine (42 mg).
MSm/z(M+H):235.

<0562-2>

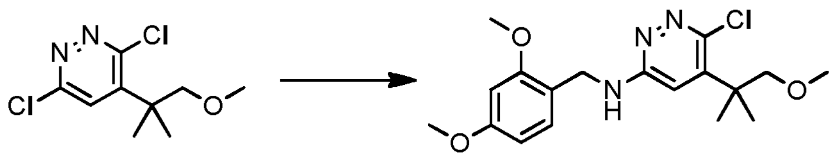

6-Chloro-N-(2,4-dimethoxybenzyl)-5-(1-methoxy-2-methylpropan-2-yl)pyridazine-3-amine was obtained in the same manner as in Example 0559-2 except that 3,6-dichloro-4-(1-methoxy-2-methylpropan-2-yl)pyridazine was used instead of the 4-(1-((tert-butyldimethylsilyl)oxy)-2-methylpropan-2-yl)-3,6-dichloropyridazine used in Example 0559-2. MSm/z(M+H):366.

<0562-3>

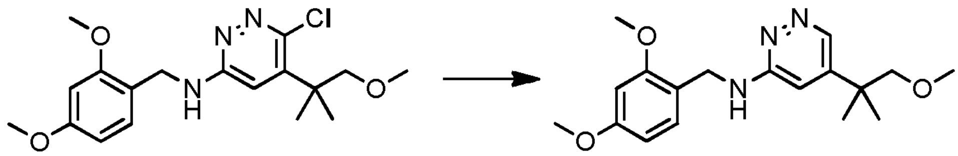

N-(2,4-dimethoxybenzyl)-5-(1-methoxy-2-methylpropan-2-yl)pyridazine-3-amine was obtained in the same manner as in Example 0559-3 except that 6-chloro-N-(2,4-dimethoxybenzyl)-5-(1-methoxy-2-methylpropan-2-yl)pyridazine-3-amine was used instead of the 5-(1-((tertbutyldimethylsilyl)oxy)-2-methylpropan-2-yl)-6-chloro-N-(2,4-dimethoxybenzyl)pyridazine-3-amine used in Example 0559-3.
MSm/z(M+H):332.

<0562-4>

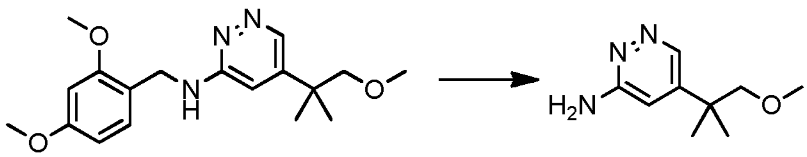

5-(1-Methoxy-2-methylpropan-2-yl)pyridazine-3-amine was obtained in the same manner as in Example 0559-4 except that N-(2,4-dimethoxybenzyl)-5-(1-methoxy-2-methylpropan-2-yl)pyridazine-3-amine was used instead of the 5-(1-((tert-butyldimethylsilyl)oxy)-2-methylpropan-2-yl)-N-(2,4-dimethoxybenzyl)pyridazine-3-amine used in Example 0559-4.
MSm/z(M+H):182.

<0562-5>

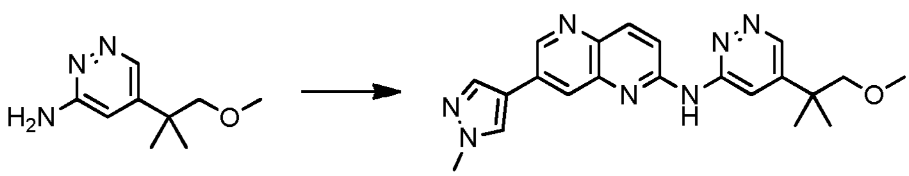

N-(5-(1-methoxy-2-methylpropan-2-yl)pyridazin-3-yl)-7-(1-methyl-1H-pyrazol-4-yl)-1,5-naphthyridine-2-amine was obtained in the same manner as in Example 0554-3 except that 5-(1-methoxy-2-methylpropan-2-yl)pyridazine-3-amine was used instead of the 5-morpholinopyridazine-3-amine used in Example 0554-3.
$^1$H-NMR($CDCl_3$/$CD_3OD$=4/1)δ:9.00(1H,brs),8.87(2H,brs),8.20(1H,d,J=9.0Hz),8.11(1H,brs),7.94(2H,brs) ,7.54(1H,d,J=9.0 Hz),4.03(3H,d),3.54(2H,s),3.37(3H,s),1.46(6H,s).
MSm/z(M+H):390.

<Example 0563>

<0563-1>

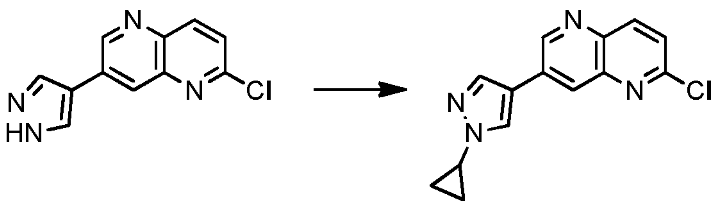

A mixture of 2-chloro-7-(1H-pyrazol-4-yl)-1,5-naphthyridine (20 mg), cyclopropylboronic acid monohydrate (18 mg), copper(II) acetate (47 mg), N,N-dimethylpyridine-4-amine (42 mg), pyridine (0.028 mL), triethylamine (0.061 mL), and 1,4-dioxane (1 mL) was stirred at 120°C for 30 minutes using a microwave reaction apparatus. The reaction mixture was cooled to room temperature, the insolubles were filtered off, and the obtained solution was purified by silica gel column chromatography (methanol-ethyl acetate-hexane), thereby obtaining 2-chloro-7-(1-cyclopropyl-1H-pyrazol-4-yl)-1,5-naphthyridine (2.2 mg).
MSm/z(M+H):271.

<0563-2>

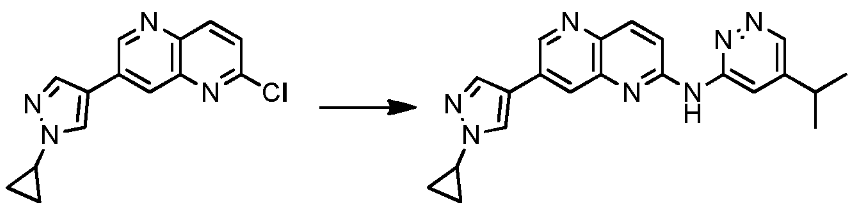

7-(1-Cyclopropyl-1H-pyrazol-4-yl)-N-(5-isopropylpyridazin-3-yl)-1,5-naphthyridine-2-amine was obtained in the same manner as in Example 0555-4 except that 2-chloro-7-(1-cyclopropyl-1H-pyrazol-4-yl)-1,5-naphthyridine was used instead of the 2-chloro-7-(1-methyl-3-(5-methylpyridin-3-yl)-1H-pyrazol-4-yl)-1,5-naphthyridine used in Example 0555-4.
$^1$H-NMR($CDCl_3$/$CD_3OD$=4/1)δ:8.87(2H,brs),8.72(1H,brs),8.20(1H,d,J=9.0Hz),8.13(1H,d,J=2.1Hz),8.01( 1H,s),7.95(1H,s), 7.49(1H,d,J=9.0Hz),3.75-3.67(1H,m),3.15-2.98(1H,m),1.42(6H,d,J=7.2Hz),1.31-1.10(4H,m).
MSm/z(M+H):372.

<Example 0564>

<0564-1>

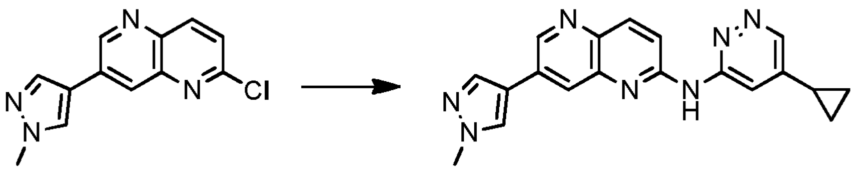

N-(5-cyclopropylpyridazin-3-yl)-7-(1-methyl-1H-pyrazol-4-yl)-1,5-naphthyridine-2-amine was obtained in the same manner as in Example 0554-3 except that 5-cyclopropylpyridazine-3-amine was used instead of the 5-morpholinopyridazine-3-amine used in Example 0554-3.
$^1$H-NMR($CDCl_3$/$CD_3OD$=4/1)δ:8.87(1H,d,J=2.1Hz),8.69(1H,brs),8.55(1H,brs),8.19(1H,d,J=9.0Hz),8.15( 1H,d,J=2.1Hz),7.97(2H,brs),7.47(1H,d,J=9.0Hz),4.03(3H,s),2.10-1.96(1H,m),1.36-1.21(2H,m),1.07-0.98(2H,m).
MSm/z(M+H):344.

<Example 0565>

<0565-1>

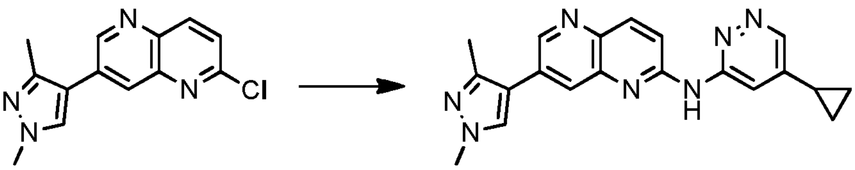

N-(5-cyclopropylpyridazin-3-yl)-7-(1,3-dimethyl-1H-pyrazol-4-yl)-1,5-naphthyridine-2-amine was obtained in the same manner as in Example 0564-1 except that 2-chloro-7-(1,3-dimethyl-1H-pyrazol-4-yl)-1,5-naphthyridine was used instead of the 2-chloro-7-(1-methyl-1H-pyrazol-4-yl)-1,5-naphthyridine used in Example 0564-1.
$^1$H-NMR($CDCl_3$/$CD_3OD$=4/1)δ:8.77(1H,d,J=1.8Hz),8.66(1H,brs),8.57(1H,brs),8.21(1H,d,J=9.0Hz),8.09( 1H,d,J=1.8Hz),7.75(1H,s),7.49(1H,d,J=9.0Hz),3.95(3H,s),2.50(3H,s),2.05-1.94(1H,m),1.36-1.23(2H,m),1.05-0.97(2H,m).
MSm/z(M+H):358.

<Example 0566>

<0566-1>

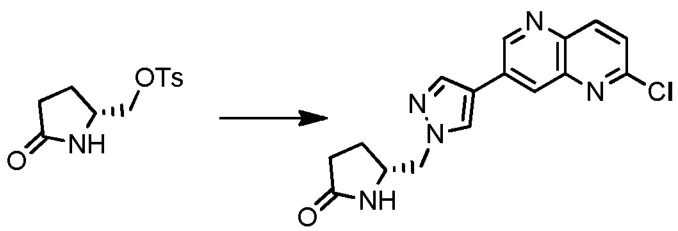

(R)-5-((4-(6-chloro-1,5-naphthyridin-3-yl)-1H-pyrazol-1-yl)methyl)pyrrolidin-2-one was obtained in the same manner as in Example 0549-1 except that (R)-(5-oxopyrrolidin-2-yl)methyl 4-methylbenzenesulfonate was used instead of the 2-chloro-N,N-dimethylacetamide used in Example 0549-1.
MSm/z(M+H):328.

<0566-2>

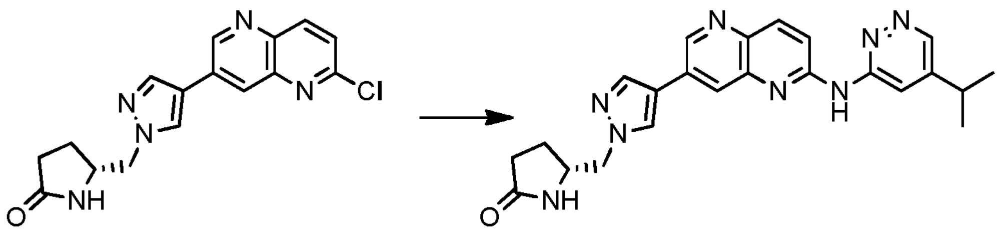

(R)-5-((4-(6-((5-isopropylpyridazin-3-yl)amino)-1,5-naphthyridin-3-yl)-1H-pyrazol-1-yl)methyl)pyrrolidin-2-one was obtained in the same manner as in Example 0555-4 except that (R)-5-((4-(6-chloro-1,5-naphthyridin-3-yl)-1H-pyrazol-1-yl)methyl)pyrrolidin-2-one was used instead of the 2-chloro-7-(1-methyl-3-(5-methylpyridin-3-yl)-1H-pyrazol-4-yl)-1,5-naphthyridine used in Example 0555-4.
$^1$H-NMR($CDCl_3$/$CD_3OD$=4/1)δ:8.88(1H,d,J=1.8Hz),8.85(1H,brs),8.71(1H,brs),8.20(1H,d,J=9.0Hz),8.16( 1H,brs),8.04(1H,s),8.02(1H,s),7.51(1H,d,J=9.0Hz),4.38-4.13(3H,m),3.15-2.98(1H,m),2.42-1.94(4H,m),1.42(6H,d,J=6.6Hz).
MSm/z(M+H):429.

<Example 0567>

<0567-1>

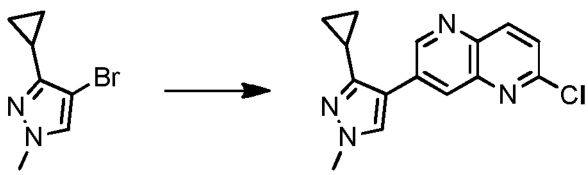

2-Chloro-7-(3-cyclopropyl-1-methyl-1H-pyrazol-4-yl)-1,5-naphthyridine was obtained in the same manner as in Example 0478-3 except that 4-bromo-3-cyclopropyl-1-methyl-1H-pyrazole was used instead of the 1-(4-(4-iodo-1H-pyrazol-1-yl)piperidin-1-yl)ethanone used in Example 0478-3.
MSm/z(M+H):285.

<0567-2>

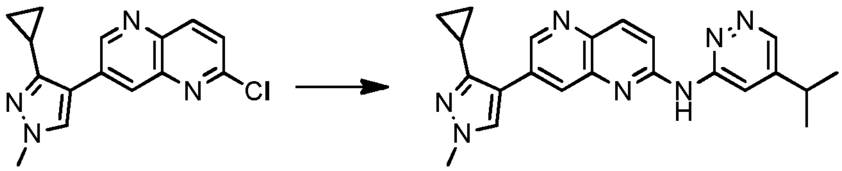

7-(3-Cyclopropyl-1-methyl-1H-pyrazol-4-yl)-N-(5-isopropylpyridazin-3-yl)-1,5-naphthyridine-2-amine was obtained in the same manner as in Example 0555-4 except that 2-chloro-7-(3-cyclopropyl-1-methyl-1H-pyrazol-4-yl)-1,5-naphthyridine was used instead of the 2-chloro-7-(1-methyl-3-(5-methylpyridin-3-yl)-1H-pyrazol-4-yl)-1,5-naphthyridine used in Example 0555-4.
$^1$H-NMR($CDCl_3$)δ:9.13(1H,brs),8.97(1H,d,J=2.1Hz),8.94(1H,brs),8.81(1H,brs),8.29(1H,brs),8.26(1H,d,J =9.0Hz),7.63(1H,s),7.60(1H,s),3.91(3H,s),3.11-2.95(1H,m),2.09-1.94(1H,m),1.41(6H,d,J=7.2Hz),1.05-0.96(4H,m).
MSm/z(M+H):386.

<Example 0568>

<0568-1>

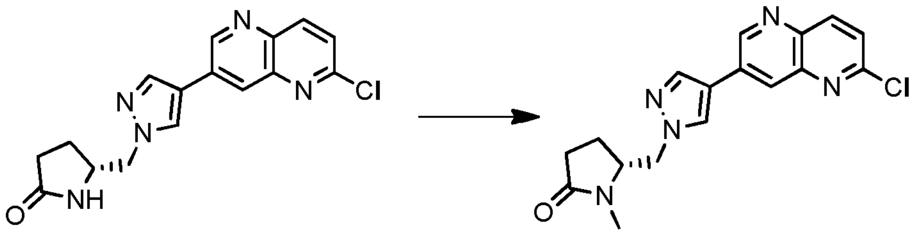

(R)-5-((4-(6-chloro-1,5-naphthyridin-3-yl)-1H-pyrazol-1-yl)methyl)-1-methylpyrrolidin-2-one was obtained in the same manner as in Example 0557-1 except that (R)-5-((4-(6-chloro-1,5-naphthyridin-3-yl)-1H-pyrazol-1-yl)methyl)pyrrolidin-2-one was used instead of the (S)-5-((4-(6-chloro-1,5-naphthyridin-3-yl)-1H-pyrazol-1-yl)methyl)pyrrolidin-2-one used in Example 0557-1.
MSm/z(M+H):342.

<0568-2>

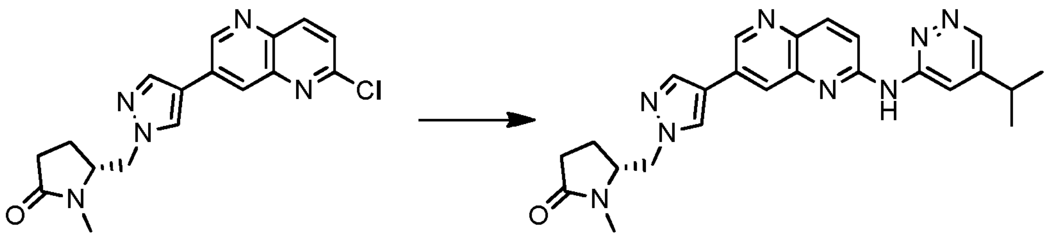

(R)-5-((4-(6-((5-isopropylpyridazin-3-yl)amino)-1,5-naphthyridin-3-yl)-1H-pyrazol-1-yl)methyl)-1-methylpyrrolidin-2-one was obtained in the same manner as in Example 0555-4 except that (R)-5-((4-(6-chloro-1,5-naphthyridin-3-yl)-1H-pyrazol-1-yl)methyl)-1-methylpyrrolidin-2-one was used instead of the 2-chloro-7-(1-methyl-3-(5-methylpyridin-3-yl)-1H-pyrazol-4-yl)-1,5-naphthyridine used in Example 0555-4.
$^1$H-NMR($CDCl_3$/$CD_3OD$=4/1)δ:8.88(2H,brs),8.72(1H,brs),8.20(1H,d,J=9.3Hz),8.16(1H,d,J=2.1Hz),8.03( 1H,s),7.99(1H,s), 7.51(1H,d,J=9.3Hz),4.41(2H,d,J=4.5Hz),4.17-4.06(1H,m),3.16-3.00(1H,m),2.89(3H,s),2.35-1.96(4H,m),1.42(6H,d,J=6 .6Hz).
MSm/z(M+H):443.

<Example 0569>

<0569-1>

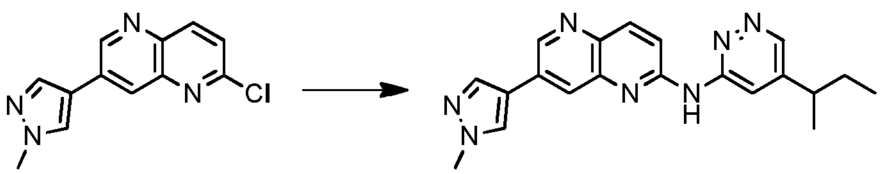

N-(5-(sec-butyl)pyridazin-3-yl)-7-(1-methyl-1H-pyrazol-4-yl)-1,5-naphthyridine-2-amine was obtained in the

same manner as in Example 0554-3 except that 5-(sec-butyl)pyridazine-3-amine was used instead of the 5-morpholinopyridazine-3-amine used in Example 0554-3.
$^1$H-NMR(DMSO-$d_6$)δ:10.70(1H,s),9.03(1H,d,J=2.1Hz),8.82(1H,d,J=2.1Hz),8.69(1H,brs),8.46(1H,s),8.24-8.16(3H,m),7.70(1H,d,J=9.0Hz),3.93(3H,s),2.85-2.69(1H,m),1.77-1.62(2H,m),1.32(3H,d,J=6.6Hz),8.79(3H,t,J=7.2Hz).
MSm/z(M+H):360.

<Example 0570>

<0570-1>

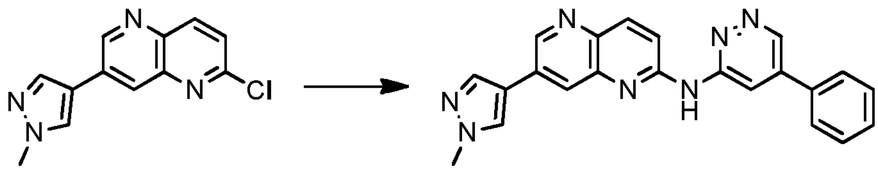

7-(1-Methyl-1H-pyrazol-4-yl)-N-(5-phenylpyridazin-3-yl)-1,5-naphthyridine-2-amine was obtained in the same manner as in Example 0554-3 except that 5-phenylpyridazine-3-amine was used instead of the 5-morpholinopyridazine-3-amine used in Example 0554-3.
$^1$H-NMR($CDCl_3$/$CD_3OD$=4/1)δ:9.24(1H,brs),9.08(1H,brs),8.88(1H,brs),8.22(1H,d,J=9.0Hz),8.17(1H,brs) ,7.95(2H,s),7.81(2H,d,J=6.6Hz),7.67-7.55(3H,m),7.52(1H,d,J=9.0Hz),4.02(3H,s).
MSm/z(M+H):380.

<Example 0571>

<0571-1>

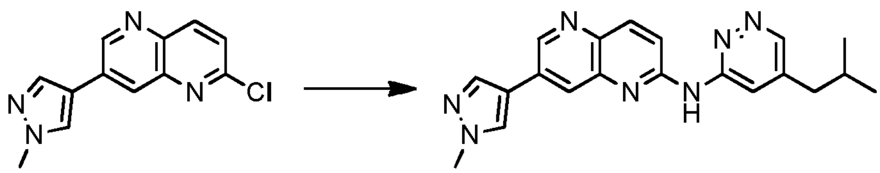

N-(5-isobutylpyridazin-3-yl)-7-(1-methyl-1H-pyrazol-4-yl)-1,5-naphthyridine-2-amine was obtained in the same manner as in Example 0554-3 except that 5-isobutylpyridazine-3-amine was used instead of the 5-morpholinopyridazine-3-amine used in Example 0554-3.
$^1$H-NMR($CDCl_3$/$CD_3OD$=4/1)δ:8.87(1H,d,J=1.8Hz),8.77(1H,brs),8.64(1H,brs),8.20(1H,d,J=8.7Hz),8.15( 1H,d,J=1.8Hz),7.96(1H,s),7.95(1H,s),7.50(1H,d,J=8.7Hz),4.03(3H,s),2.63(2H,d,J=7.5Hz),2.15-2.00(1H,m),1.05(3H,d,J=6.6Hz).
MSm/z(M+H):360.

<Example 0572>

<0572-1>

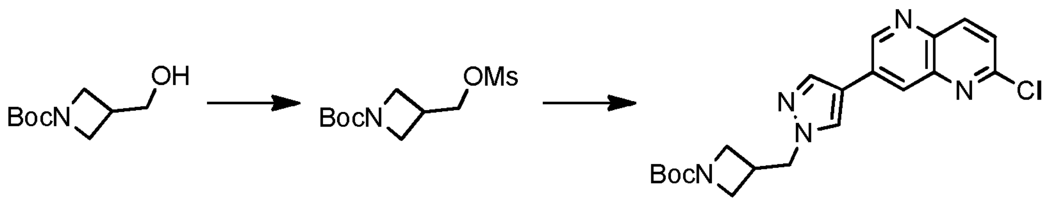

Methanesulfonyl chloride (0.051 mL) was added to a mixture of tert-butyl 3-(hydroxymethyl)azetidine-1-carboxylate (81 mg), triethylamine (0.121 mL), and dichloromethane (4 mL) under ice-cooling, followed by stirring at the same temperature for 30 minutes. After water and dichloromethane were added the reaction mixture, the organic layer was collected by separation, and dried over anhydrous sodium sulfate, and the solvent was distilled off under reduced pressure, thereby obtaining tert-butyl 3-(((methylsulfonyl)oxy)methyl)azetidine-1-carboxylate (159 mg).

A suspension of tert-butyl 3-(((methylsulfonyl)oxy)methyl)azetidine-1-carboxylate (115 mg), 2-chloro-7-(1H-pyrazol-4-yl)-1,5-naphthyridine (50 mg), and cesium carbonate (211 mg) in N,N-dimethylformamide (1 mL) was stirred at 80°C for 1 hour. After the reaction mixture was cooled to room temperature, ethyl acetate and water were added thereto. The organic layer was collected by separation, washed sequentially with water and a saturated sodium chloride aqueous solution, and dried over anhydrous sodium sulfate, the solvent was distilled off under reduced pressure, and the obtained residue was purified by silica gel column chromatography (methanol-ethyl acetate-hexane), thereby obtaining tert-butyl 3-((4-(6-chloro-1,5-naphthyridin-3-yl)-1H-pyrazol-1-yl)methyl)azetidine-1-carboxylate (45 mg).
MSm/z(M+H):400.

<0572-2>

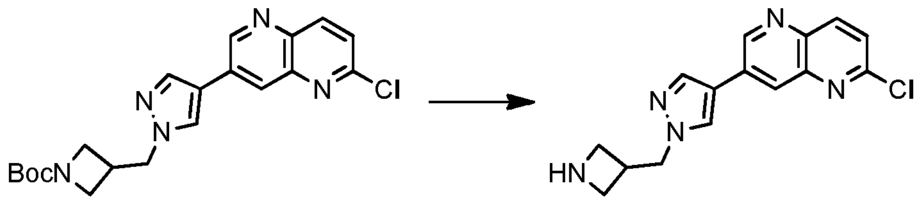

Trifluoroacetic acid (2 mL) was added to a mixture of tert-butyl 3-((4-(6-chloro-1,5-naphthyridin-3-yl)-1H-pyrazol-1-yl)methyl)azetidine-1-carboxylate (45 mg), and water (0.1 mL), followed by stirring at room temperature for 2 hours. The solvent was distilled off under reduced pressure, the obtained residue was purified by silica gel column chromatography (methanol-ethyl acetate, NH silica), thereby obtaining 7-(1-(azetidin-3-ylmethyl)-1H-pyrazol-4-yl)-2-chloro-1,5-naphthyridine (55 mg).
MSm/z(M+H):300.

<0572-3>

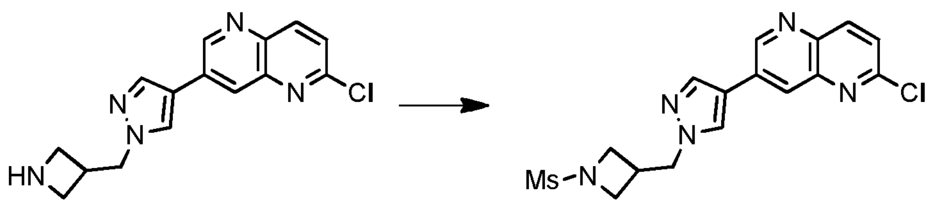

Methanesulfonyl chloride (0.018 mL) was added to a mixture of 7-(1-(azetidin-3-ylmethyl)-1H-pyrazol-4-yl)-2-chloro-1,5-naphthyridine (55 mg), triethylamine (0.048 mL), and dichloromethane (1 mL) under ice-cooling, followed by stirring at room temperature for 1 hour. The reaction mixture was purified by silica gel column chromatography (methanol-ethyl acetate-hexane), thereby obtaining 2-chloro-7-(1-((1-(methylsulfonyl)azetidin-3-yl)methyl)-1H-pyrazol-4-yl)-1,5-naphthyridine (5.4 mg).

MSm/z(M+H):378.

<0572-4>

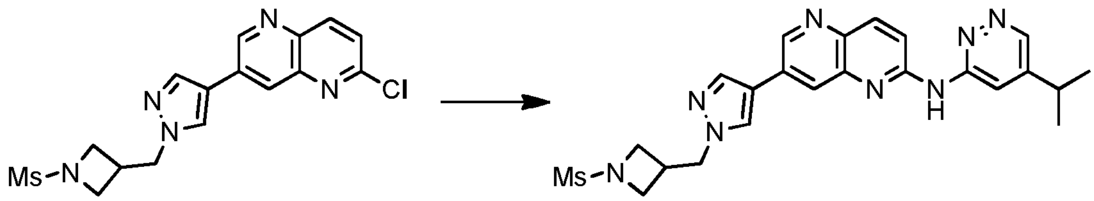

N-(5-isopropylpyridazin-3-yl)-7-(1-((1-(methylsulfonyl)azetidin-3-yl)methyl)-1H-pyrazol-4-yl)-1,5-naphthyridine-2-amine was obtained in the same manner as in Example 0555-4 except that 2-chloro-7-(1-((1-(methylsulfonyl)azetidin-3-yl)methyl)-1H-pyrazol-4-yl)-1,5-naphthyridine was used instead of the 2-chloro-7-(1-methyl-3-(5-methylpyridin-3-yl)-1H-pyrazol-4-yl)-1,5-naphthyridine used in Example 0555-4.
$^1$H-NMR($CDCl_3$/$CD_3OD$=4/1)δ:8.87(2H,brs),8.72(1H,brs),8.20(1H,d,J=9.0Hz),8.15(1H,brs),8.04(1H,s),8.00(1H,s),7.51(1H,d,J=9.0Hz),4.98(2H,d,J=6.6Hz),4.13-4.04(2H,m),3.90-3.82(2H,m),3.32-3.15(1H,m),3.14-2.98(1H,m),2.89(3H,s),1.42(6H,d,J=6.6Hz).
MSm/z(M+H):479.

<Example 0573>

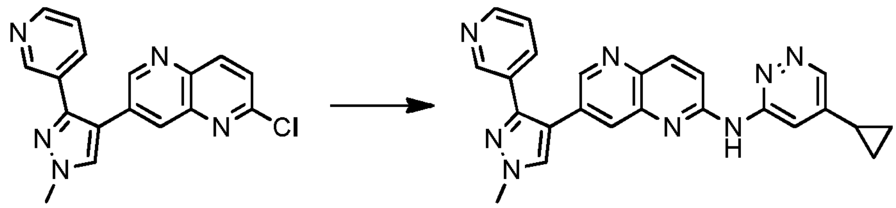

N-(5-cyclopropylpyridazin-3-yl)-7-(1-methyl-3-(pyridin-3-yl)-1H-pyrazol-4-yl)-1,5-naphthyridine-2-amine was obtained in the same manner as in Example 0564-1 except that 2-chloro-7-(1-methyl-3-(pyridin-3-yl)-1H-pyrazol-4-yl)-1,5-naphthyridine was used instead of the 2-chloro-7-(1-methyl-1H-pyrazol-4-yl)-1,5-naphthyridine used in Example 0564-1.
$^1$H-NMR($CDCl_3$/$CD_3OD$=4/1)δ:8.73(1H,d,J=1.8Hz),8.62-8.52(4H,m),8.19(1H,d,J=9.0Hz),7.93(1H,d,J=1.8Hz),7.92-7.83(3H,m),7.48(1H,d,J=9.0Hz),4.09(3H,s),1.99-1.88(1H,m),1.28-1.20(2H,m),0.31-0.82(2H,m).
MSm/z(M+H):421.

<Example 0574>

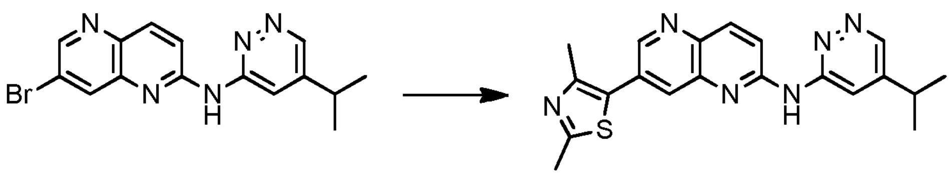

A mixture of 7-bromo-N-(5-isopropylpyridazin-3-yl)-1,5-naphthyridine-2-amine (20 mg), (2,4-dimethylthiazol-5-yl)boronic acid (20 mg), sodium carbonate (18 mg), bis(di-tert-butyl (4-dimethylaminophenyl)phosphine)dichloropalladium(II) (2 mg), water (0.1 mL), and 1,4-dioxane (1 mL) was stirred at 100°C for 2 hours. The reaction mixture was

cooled to room temperature, the insolubles were filtered off, and the solvent was distilled off under reduced pressure. The obtained residue was purified by silica gel column chromatography (methanol-ethyl acetate), thereby obtaining 7-(2,4-dimethylthiazol-5-yl)-N-(5-isopropylpyridazin-3-yl)-1,5-naphthyridine-2-amine (5.9 mg).
$^1$H-NMR($CDCl_3$)$\delta$:10.31(1H,brs),9.01(1H,brs),8.84(1H,brs),8.83(1H,d,J=1.8Hz),8.29(1H,d,J=9.3Hz),8.10 (1H,d,J=1.8Hz),7.93(1H,d,J=9.3Hz),3.12-3.00(1H,m),2.75(3H,s),2.57(3H,s),1.41(6H,d,J=6.6Hz).
MSm/z(M+H):377.

<Example 0575>

<0575-1>

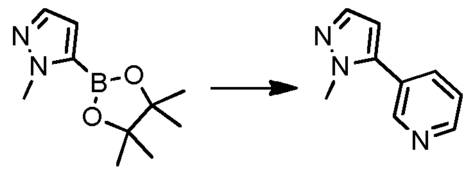

A mixture of 1-methyl-5-(4,4,5,5-tetramethyl-1,3,2-dioxaborolan-2-yl)-1H-pyrazole (500 mg), 3-bromopyridine (0.254 mL), sodium carbonate (635 mg), bis(di-tert-butyl (4-dimethylaminophenyl)phosphine)dichloropalladium(II) (85 mg), water (1.2 mL), and 1,4-dioxane (12 mL) was stirred at 100°C for 2 hours. The reaction mixture was cooled to room temperature, the insolubles were filtered off, and the solvent was distilled off under reduced pressure. The obtained residue was purified by silica gel column chromatography (methanol-ethyl acetate), thereby obtaining 3-(1-methyl-1H-pyrazol-5-yl)pyridine (277 mg).
MSm/z(M+H):160.

<0575-2>

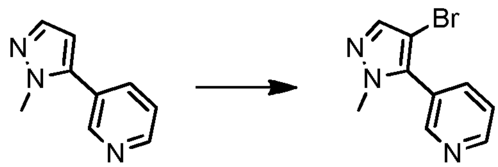

3-(4-Bromo-1-methyl-1H-pyrazol-5-yl)pyridine was obtained in the same manner as in Example 0555-2 except that 3-(1-methyl-1H-pyrazol-5-yl)pyridine was used instead of the 3-methyl-5-(1-methyl-1H-pyrazol-3-yl)pyridine used in Example 0555-2.
MSm/z(M+H):238.

<0575-3>

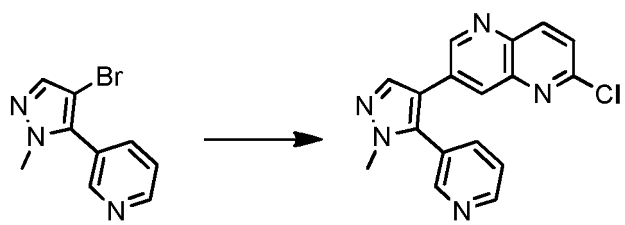

2-Chloro-7-(1-methyl-5-(pyridin-3-yl)-1H-pyrazol-4-yl)-1,5-naphthyridine was obtained in the same manner as in Example 0478-3 except that 3-(4-bromo-1-methyl-1H-pyrazol-5-yl)pyridine was used instead of the 1-(4-(4-iodo-1H-pyrazol-1-yl)piperidin-1-yl)ethanone used in Example 0478-3.
MSm/z(M+H):322.

<0575-4>

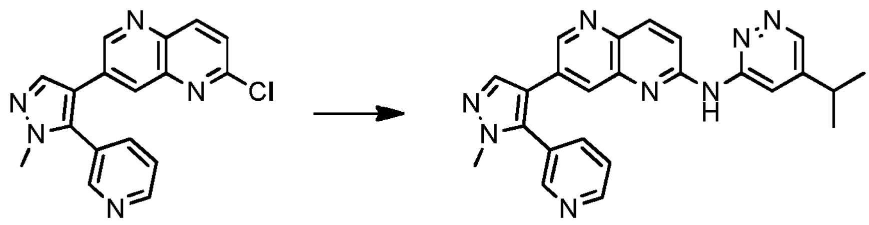

N-(5-isopropylpyridazin-3-yl)-7-(1-methyl-5-(pyridin-3-yl)-1H-pyrazol-4-yl)-1,5-naphthyridine-2-amine was obtained in the same manner as in Example 0555-4 except that 2-chloro-7-(1-methyl-5-(pyridin-3-yl)-1H-pyrazol-4-yl)-1,5-naphthyridine was used instead of the 2-chloro-7-(1-methyl-3-(5-methylpyridin-3-yl)-1H-pyrazol-4-yl)-1,5-naphthyridine used in Example 0555-4.
$^1$H-NMR(DMSO-$d_6$)δ:10.69(1H,brs),8.82(1H,d,J=1.2Hz),8.75-8.72(2H,m),8.66(1H,d,J=1.2Hz),8.55(1H,d,J=1.2Hz),8.19(1H,s),8.18(1H,d,J=9.0Hz),8.03-7.97(1H,m),7.68(1H,d,J=9.0Hz),7.63-7.57(2H,m),3.78(3H,s),3.04-2.88(1H,m),1.25(6H,d,J=6.6Hz).
MSm/z(M+H):423.

<Example 0576>

<0576-1>

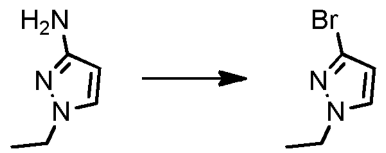

A solution of sodium nitrite (315 mg) in water (0.4 mL) and copper(II) bromide (1.48 g) were added to 1-ethyl-1H-pyrazole-3-amine (461 mg) in 48% hydrobromic acid under ice cooling, followed by stirring at room temperature for 3 hours. Sodium carbonate (4.0 g) was added to the reaction mixture, and the insolubles were filtered off. Ethyl acetate and a saturated sodium chloride aqueous solution were added to the filtrate, the organic layer was collected by separation, and dried over anhydrous sodium sulfate. The solvent was distilled off under reduced pressure, and the obtained residue was purified by silica gel column chromatography (ethyl acetate-hexane), thereby obtaining 3-bromo-1-ethyl-1H-pyrazole (530 mg).
MSm/z(M+H):175.

<0576-2>

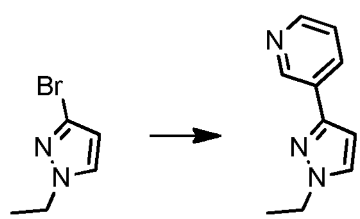

A mixture of 3-bromo-1-ethyl-1H-pyrazole (220 mg), 3-pyridineboronic acid (170 mg), sodium carbonate (331

mg), bis(di-tert-butyl (4-dimethylaminophenyl)phosphine)dichloropalladium(II) (44 mg), water (0.6 mL), and 1,4-dioxane (6 mL) was stirred at 100°C for 5 hours. The reaction mixture was cooled to room temperature, the insolubles were filtered off, and the solvent was distilled off under reduced pressure. The obtained residue was purified by silica gel column chromatography (methanol-ethyl acetate-hexane), thereby obtaining 3-(1-ethyl-1H-pyrazol-3-yl)pyridine (53 mg).
MSm/z(M+H):174.

<0576-3>

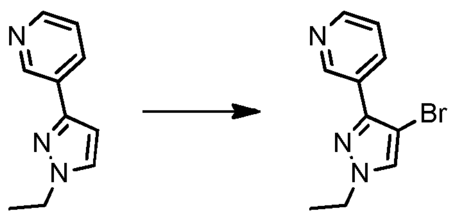

3-(4-Bromo-1-ethyl-1H-pyrazol-3-yl)pyridine was obtained in the same manner as in Example 0555-2 except that 3-(1-ethyl-1H-pyrazol-3-yl)pyridine was used instead of the 3-methyl-5-(1-methyl-1H-pyrazol-3-yl)pyridine used in Example 0555-2.
MSm/z(M+H):252.

<0576-4>

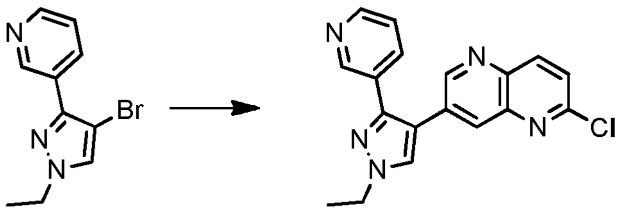

2-Chloro-7-(1-ethyl-3-(pyridin-3-yl)-1H-pyrazol-4-yl)-1,5-naphthyridine was obtained in the same manner as in Example 0478-3 except that 3-(4-bromo-1-ethyl-1H-pyrazol-3-yl)pyridine was used instead of the 1-(4-(4-iodo-1H-pyrazol-1-yl)piperidin-1-yl)ethanone used in Example 0478-3.
MSm/z(M+H):336.

<0576-5>

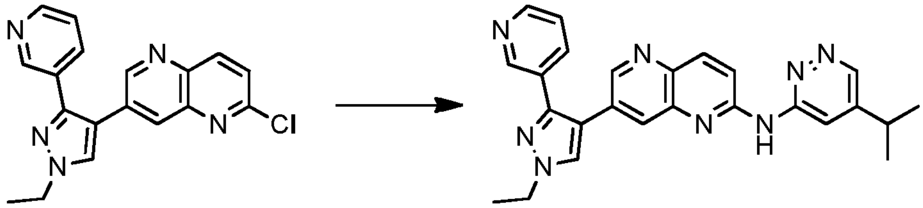

7-(1-Ethyl-3-(pyridin-3-yl)-1H-pyrazol-4-yl)-N-(5-isopropylpyridazin-3-yl)-1,5-naphthyridine-2-amine was obtained in the same manner as in Example 0555-4 except that 2-chloro-7-(1-ethyl-3-(pyridin-3-yl)-1H-pyrazol-4-yl)-1,5-naphthyridine was used instead of the 2-chloro-7-(1-methyl-3-(5-methylpyridin-3-yl)-1H-pyrazol-4-yl)-1,5-naphthyridine used in Example 0555-4.
$^{1}$H-NMR(DMSO-d$_6$)δ:10.72(1H,s),8.83(1H,d,J=2.1Hz),8.70(1H,d,J=2.1Hz),8.63(1H,brs),8.62(1H,brs),8.56(1H,dd,J=4.5 ,2.1Hz),8.42(1

H,s),8.23(1H,d,J=9.0Hz),7.88-7.81(2H,m),7.72(1H,d,J=9.0Hz),7.43(1H,dd,J=7.8,4.2Hz),4.29(2H,q,J=4.2Hz),3.02-2.86 (1H,m),1.51(3H,t,J=4.2Hz),1.22(6H,d,J=6.6Hz).
MSm/z(M+H):437.

<Example 0577>

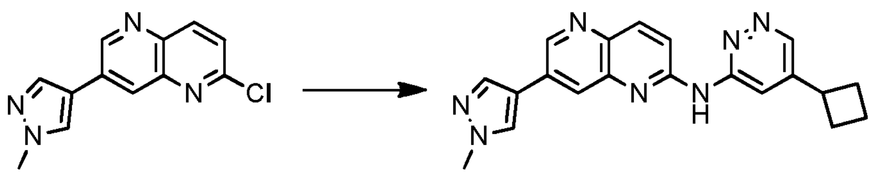

N-(5-cyclobutylpyridazin-3-yl)-7-(1-methyl-1H-pyrazol-4-yl)-1,5-naphthyridine-2-amine was obtained in the same manner as in Example 0554-3 except that 5-cyclobutylpyridazine-3-amine was used instead of the 5-morpholinopyridazine-3-amine used in Example 0554-3.
$^1$H-NMR(DMSO-$d_6$)δ:10.70(1H,s),9.03(1H,d,J=2.1Hz),8.82(1H,d,J=2.1Hz),8.68(1H,brs),8.46(1H,s),8.22(1H,d,J=9.0Hz ),8.19(1H,d,J=2 .1Hz),8.17(1H,s),7.70(1H,d,J=9.0Hz),3.92(3H,s),3.75-3.59(1H,m),2.47-2.38(2H,m),2.32-1.88(4H,m).
MSm/z(M+H):358.

<Example 0578>

<0578-1>

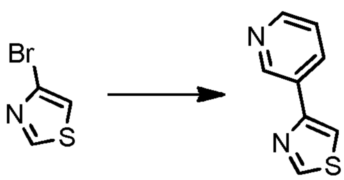

4-(Pyridin-3-yl)thiazole was obtained in the same manner as in Example 0576-2 except that 4-bromothiazole was used instead of the 3-bromo-1-ethyl-1H-pyrazole used in Example 0576-2.
MSm/z(M+H):163.

<0578-2>

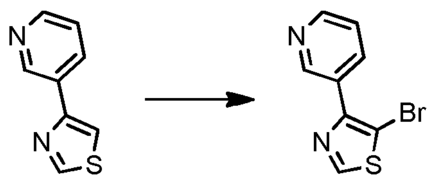

Bromine (0.097 mL) was added to a solution of 4-(pyridin-3-yl)thiazole (254 mg) in acetic acid (1.6 mL) under ice cooling, followed by stirring at 60°C for 4 hours. After a 4 mol/L sodium hydroxide aqueous solution, sodium hydrogen sulfite, sodium carbonate, and ethyl acetate were added to the reaction mixture, the organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining 5-bromo-4-(pyridin-3-yl)thiazole (180 mg).
MSm/z(M+H):241.

<0578-3>

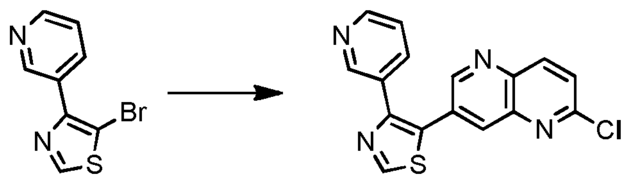

5-(6-Chloro-1,5-naphthyridin-3-yl)-4-(pyridin-3-yl)thiazole was obtained in the same manner as in Example 0478-3 except that 5-bromo-4-(pyridin-3-yl)thiazole was used instead of the 1-(4-(4-iodo-1H-pyrazol-1-yl)piperidin-1-yl)ethanone used in Example 0478-3.
MSm/z(M+H):325.

<0578-4>

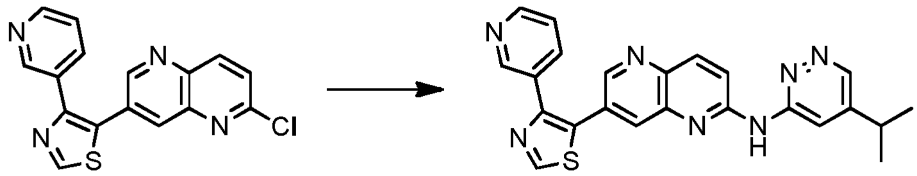

N-(5-isopropylpyridazin-3-yl)-7-(4-(pyridin-3-yl)thiazol-5-yl)-1,5-naphthyridine-2-amine was obtained in the same manner as in Example 0555-4 except that 5-(6-chloro-1,5-naphthyridin-3-yl)-4-(pyridin-3-yl)thiazole was used instead of the 2-chloro-7-(1-methyl-3-(5-methylpyridin-3-yl)-1H-pyrazol-4-yl)-1,5-naphthyridine used in Example 0555-4.
$^1$H-NMR(DMSO-$d_6$)$\delta$:10.83(1H,s),9.39(1H,s),8.85(1H,d,J=2.1Hz),8.69-8.63(3H,m),8.57(1H,d,,J=4.5,2.1Hz),8.28(1H,d,J=9.0Hz),8.11(1H,brs),7.91(1H,dt,J=7.8,2.1Hz),7.80(1 H,d,J=9.0Hz),7.47-7.36(1H,m),3.08-2.88(1H,m),1.25(6H,d,J=6.6Hz).
MSm/z(M+H):426.

<Example 0579>

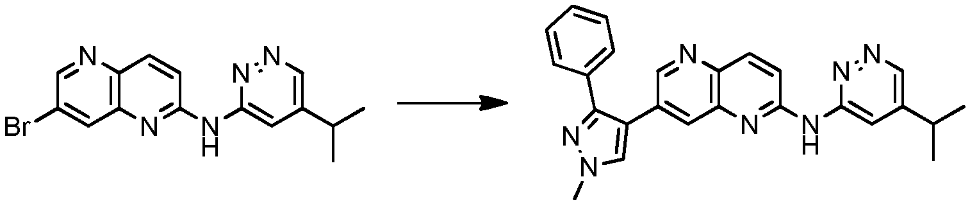

N-(5-isopropylpyridazin-3-yl)-7-(1-methyl-3-phenyl-1H-pyrazol-4-yl)-1,5-naphthyridine-2-amine was obtained in the same manner as in Example 0574-1 except that 1-methyl-3-phenyl-4-(4,4,5,5-tetramethyl-1,3,2-dioxaborolan-2-yl)-1H-pyrazole was used instead of the (2,4-dimethylthiazol-5-yl)boronic acid used in Example 0574-1.
$^1$H-NMR($CDCl_3$)$\delta$:10.02(1H,brs),8.92(1H,brs),8.80(1H,brs),8.69(1H,d,J=2.1Hz),8.23(1H,d,J=9.0Hz),7.95(1H,d,J=2.1Hz),7.77(1H,d,J=9.0Hz),7.69(1H,s),7.55-7.49(2H,m),7.39-7.30(3H,m),4.05(3H,s),3.08-2.91(1H,m),1.34(6H,d,J=7.2Hz).
MSm/z(M+H):422.

<Example 0580>

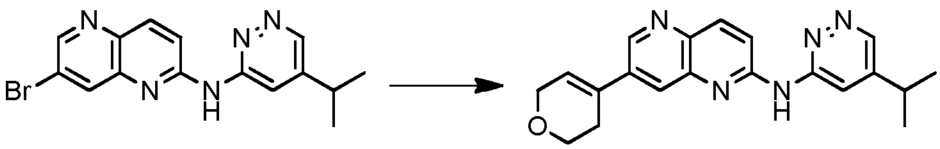

7-(3,6-Dihydro-2H-pyran-4-yl)-N-(5-isopropylpyridazin-3-yl)-1,5-naphthyridine-2-amine was obtained in the same manner as in Example 0574-1 except that (3,6-dihydro-2H-pyran-4-yl)boronic acid was used instead of the (2,4-dimethylthiazol-5-yl)boronic acid used in Example 0574-1.
$^1$H-NMR(DMSO-$d_6$)δ:10.71(1H,s),8.95(1H,d,J=2.1Hz),8.86(1H,d,J=2.1Hz),8.72(1H,d,J=2.1Hz),8.23(1H,d,J=9.0Hz),8.01(1H,brs),7.74(1H,d,J=9.0Hz),6.64(1H,brs),4.31(2H,brs),3.89(2H,t,J=5.7Hz),3.12-2.95(1H,m),2.61(2H,brs),1.31(6H,d,J=6.6Hz).
MSm/z(M+H):348.

<Example 0581>

<0581-1>

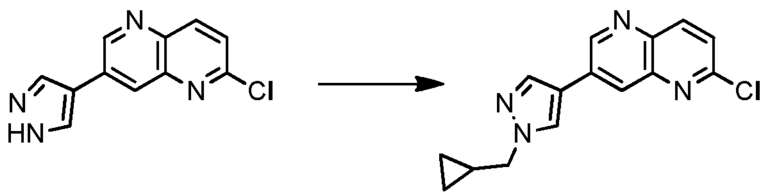

2-Chloro-7-(1-(cyclopropylmethyl)-1H-pyrazol-4-yl)-1,5-naphthyridine was obtained in the same manner as in Example 0549-1 except that (bromomethyl)cyclopropane was used instead of the 2-chloro-N,N-dimethylacetamide used in Example 0549-1.
MSm/z(M+H):285.

<0581-2>

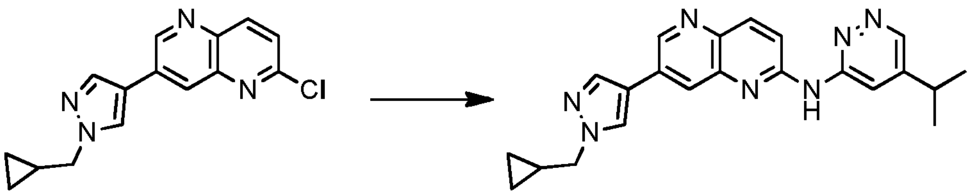

7-(1-(Cyclopropylmethyl)-1H-pyrazol-4-yl)-N-(5-isopropylpyridazin-3-yl)-1,5-naphthyridine-2-amine was obtained in the same manner as in Example 0555-4 except that 2-chloro-7-(1-(cyclopropylmethyl)-1H-pyrazol-4-yl)-1,5-naphthyridine was used instead of the 2-chloro-7-(1-methyl-3-(5-methylpyridin-3-yl)-1H-pyrazol-4-yl)-1,5-naphthyridine used in Example 0555-4.
$^1$H-NMR(DMSO-$d_6$)δ:10.69(1H,s),9.05(1H,d,J=1.8Hz),8.87(1H,d,J=1.8Hz),8.72(1H,s),8.54(1H,s),8.24-8.17(3H,m),7.70(1H,d,J=9.0Hz),4.05(2H,d,J=7.2Hz),3.10-2.95(1H,m),2.20-1.99(1H,m),1.33(6H,d,J=6.6Hz),0.61-0.54(2H,m),0.46-0.39(2H,m).
MSm/z(M+H):386.

<Example 0582>

<0582-1>

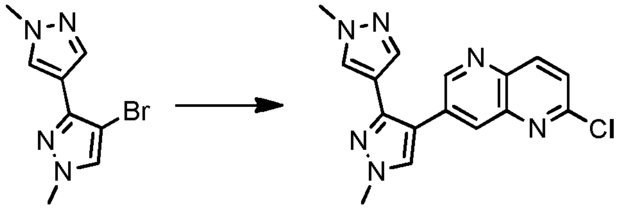

2-Chloro-7-(1,1'-dimethyl-1H,1'H-[3,4'-bipyrazole]-4-yl)-1,5-naphthyridine was obtained in the same manner as in Example 0478-3 except that 4-bromo-1,1'-dimethyl-1H,1'H-3,4'-bipyrazole was used instead of the 1-(4-(4-iodo-1H-pyrazol-1-yl)piperidin-1-yl)ethanone used in Example 0478-3.
MSm/z(M+H):325.

<0582-2>

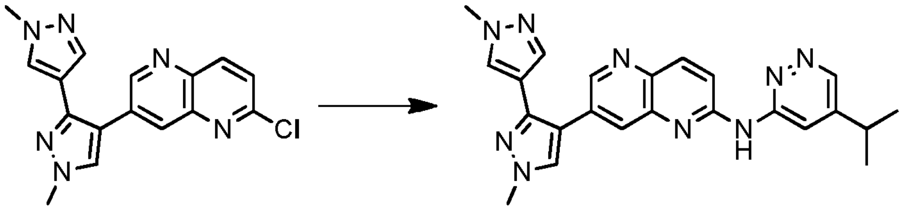

7-(1,1'-Dimethyl-1H,1'H-[3,4'-bipyrazole]-4-yl)-N-(5-isopropylpyridazin-3-yl)-1,5-naphthyridine-2-amine was obtained in the same manner as in Example 0555-4 except that 2-chloro-7-(1,1'-dimethyl-1H,1'H-[3,4'-bipyrazole]-4-yl)-1,5-naphthyridine was used instead of the 2-chloro-7-(1-methyl-3-(5-methylpyridin-3-yl)-1H-pyrazol-4-yl)-1,5-naphthyridine used in Example 0555-4.
$^1$H-NMR(DMSO-$d_6$)δ:10.73(1H,s),8.84(1H,d,J=2.1Hz),8.71(1H,2.1Hz),8.68(1H,d,J=2.1Hz),8.24(1H,d,J=9.0Hz),8.18(1H,s),8.01(1H,d,J=2.1Hz),7.78(1H,d,J=2.1Hz),7.76(1H,d,J=9.0Hz),7.45(1H,s),3.91(3H,s),3.82(3H,s),3.05-2.90(IH,m),1.27(6H,d,J=6.6Hz).
MSm/z(M+H):426.

<Example 0583>

<0583-1>

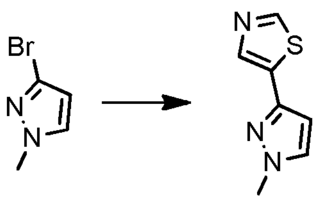

A suspension of 5-bromothiazole (200 mg), bis(pinacolato)diboron (371 mg), (1,1'-bis(diphenylphosphino)ferrocene)palladium(II) dichloride (98 mg) and potassium acetate (296 mg) in 1,4-dioxane (6 mL) was stirred at 100°C for 2 hours in a nitrogen atmosphere. 3-Bromo-1-methyl-1H-pyrazole (194 mg), water (0.6 mL), sodium carbonate (320 mg), and bis(di-tert-butyl (4-dimethylaminophenyl)phosphine)dichloropalladium(II) (42 mg) were added to the reaction

mixture, followed by stirring at 100°C for 2 hours. The reaction mixture was cooled to room temperature, the insolubles were filtered off, and the obtained residue was purified by silica gel column chromatography (methanol-ethyl acetate), thereby obtaining 5-(1-methyl-1H-pyrazol-3-yl)thiazole (16 mg).
MSm/z(M+H):166.

<0583-2>

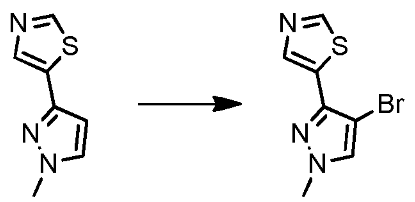

5-(4-Bromo-1-methyl-1H-pyrazol-3-yl)thiazole was obtained in the same manner as in Example 0555-2 except that 5-(1-methyl-1H-pyrazol-3-yl)thiazole was used instead of the 3-methyl-5-(1-methyl-1H-pyrazol-3-yl)pyridine used in Example 0555-2.
MSm/z(M+H):244.

<0583-3>

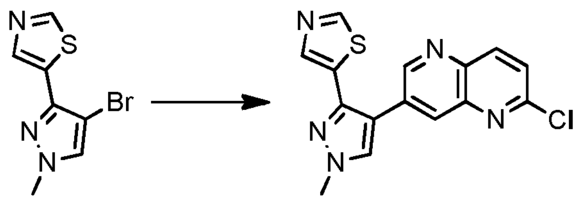

5-(4-(6-Chloro-1,5-naphthyridin-3-yl)-1-methyl-1H-pyrazol-3-yl)thiazole was obtained in the same manner as in Example 0478-3 except that 5-(4-bromo-1-methyl-1H-pyrazol-3-yl)thiazole was used instead of the 1-(4-(4-iodo-1H-pyrazol-1-yl)piperidin-1-yl)ethanone used in Example 0478-3.
MSm/z(M+H):328.

<0583-4>

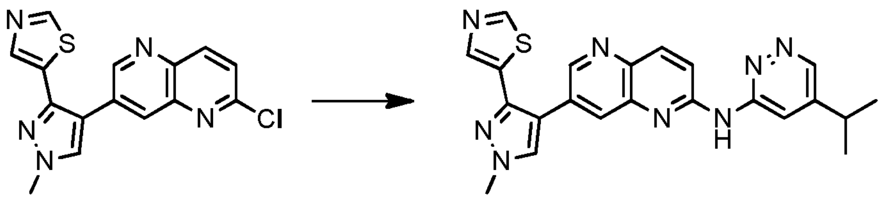

N-(5-isopropylpyridazin-3-yl)-7-(1-methyl-3-(thiazol-5-yl)-1H-pyrazol-4-yl)-1,5-naphthyridine-2-amine was obtained in the same manner as in Example 0555-4 except that 5-(4-(6-chloro-1,5-naphthyridin-3-yl)-1-methyl-1H-pyrazol-3-yl)thiazole was used instead of the 2-chloro-7-(1-methyl-3-(5-methylpyridin-3-yl)-1H-pyrazol-4-yl)-1,5-naphthyridine used in Example 0555-4.
$^1$H-NMR(DMSO-$d_6$)$\delta$:10.76(1H,s),9.07(1H,s),8.84(1H,d,J=2.1Hz),8.72(1H,d,J=2.1Hz),8.68(1H,d,J=2.1Hz),8.29(1H,s), 8.27(1H,d,J=9.0Hz),8.00(1H,d,J=2.1Hz),7.82(1H,s),7.77(1H,d,J=9.0Hz),3.97(3H,s),3.08-2.90(1H,m),1.26(6H,d,J=6.6 Hz).

MSm/z(M+H):429.

<Example 0584>

<0584-1>

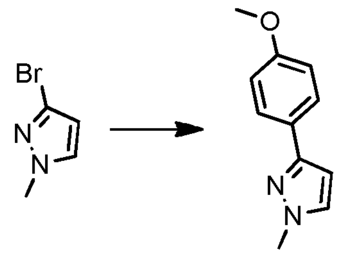

A mixture of 3-bromo-1-methyl-1H-pyrazole (200 mg), 4-methoxyphenylboronic acid (188 mg), sodium carbonate (328 mg), bis(di-tert-butyl (4-dimethylaminophenyl)phosphine)dichloropalladium(II) (43 mg), water (0.6 mL), and 1,4-dioxane (6 mL) was stirred at 100°C for 4 hours. The reaction mixture was cooled to room temperature, the insolubles were filtered off, and the solvent was distilled off under reduced pressure. The obtained residue was purified by silica gel column chromatography (methanol-ethyl acetate-hexane), thereby obtaining 3-(4-methoxyphenyl)-1-methyl-1H-pyrazole (200 mg).
MSm/z(M+H):189.

<0584-2>

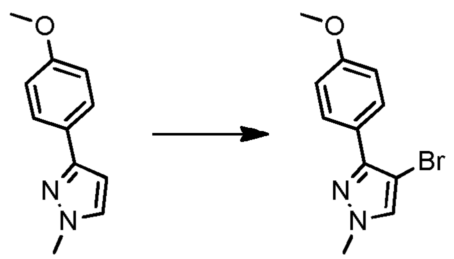

4-Bromo-3-(4-methoxyphenyl)-1-methyl-1H-pyrazole was obtained in the same manner as in Example 0555-2 except that 3-(4-methoxyphenyl)-1-methyl-1H-pyrazole was used instead of the 3-methyl-5-(1-methyl-1H-pyrazol-3-yl)pyridine used in Example 0555-2.
MSm/z(M+H):267.

<0584-3>

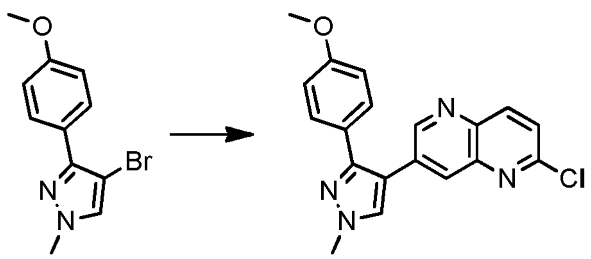

2-Chloro-7-(3-(4-methoxyphenyl)-1-methyl-1H-pyrazol-4-yl)-1,5-naphthyridine was obtained in the same manner as in Example 0478-3 except that 4-bromo-3-(4-methoxyphenyl)-1-methyl-1H-pyrazole was used instead of the 1-(4-(4-iodo-1H-pyrazol-1-yl)piperidin-1-yl)ethanone used in Example 0478-3.
MSm/z(M+H):351.

<0584-4>

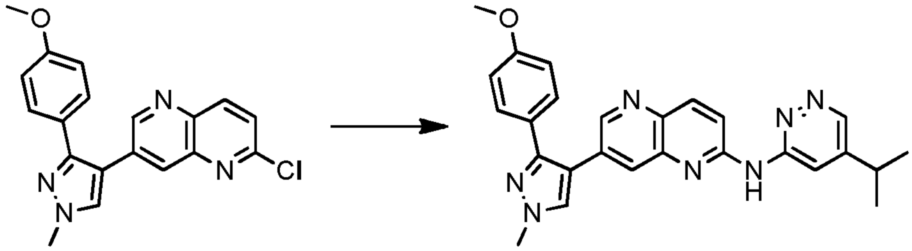

N-(5-isopropylpyridazin-3-yl)-7-(3-(4-methoxyphenyl)-1-methyl-1H-pyrazol-4-yl)-1,5-naphthyridine-2-amine was obtained in the same manner as in Example 0555-4 except that 2-chloro-7-(3-(4-methoxyphenyl)-1-methyl-1H-pyrazol-4-yl)-1,5-naphthyridine was used instead of the 2-chloro-7-(1-methyl-3-(5-methylpyridin-3-yl)-1H-pyrazol-4-yl)-1,5-naphthyridine used in Example 0555-4.
$^1$H-NMR(CDCl$_3$)δ:8.74(2H,brs),8.70(1H,d,J=1.8Hz),8.23(1H,d,J=8.4Hz),7.97(1H,brs),7.67(1H,s),7.50(1 H,d,J=8.4Hz),7.43(2H,d,J=8.7Hz),6.88(2H,d,J=8.7Hz),4.03(3H,s),3.80(3H,s),3.07-2.89(1H,m),1.33(6H,d,J=6.6Hz).
MSm/z(M+H):452.

<Example 0585>

<0585-1>

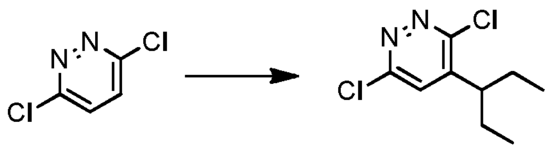

Potassium peroxodisulfate (1.81 g) was added to a mixture of 3,6-dichloropyridazine (1 g), sulfuric acid (1.4 mL), 2-ethylbutanoic acid (1.10 mL), silver nitrate (228 mg), and water (33 mL) at 70°C, followed by stirring at the same temperature for 30 minutes. After the reaction mixture was cooled by ice, sodium carbonate (10 g) and sodium chloride (1 g) were added thereto, followed by stirring at room temperature for 30 minutes. The insolubles were filtered off, and ethyl acetate was added thereto. The organic layer was collected by separation, washed with a saturated sodium chloride aqueous solution, and dried over anhydrous sodium sulfate, and the solvent was distilled off under reduced pressure, thereby obtaining 3,6-dichloro-4-(pentan-3-yl)pyridazine (1.07 g).
MSm/z(M+H):219.

<0585-2>

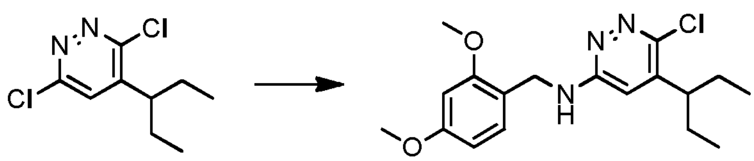

6-Chloro-N-(2,4-dimethoxybenzyl)-5-(pentan-3-yl)pyridazine-3-amine was obtained in the same manner as in Example 0559-2 except that 3,6-dichloro-4-(pentan-3-yl)pyridazine was used instead of the 4-(1-((tert-butyldimethylsilyl)oxy)-2-methylpropan-2-yl)-3,6-dichloropyridazine used in Example 0559-2.
MSm/z(M+H):350.

<0585-3>

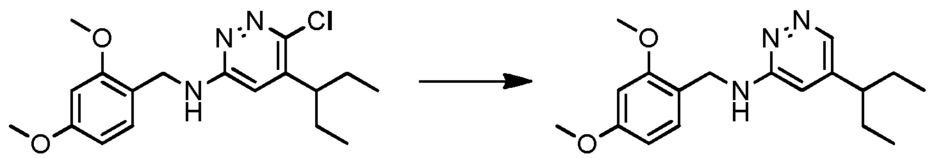

N-(2,4-dimethoxybenzyl)-5-(pentan-3-yl)pyridazine-3-amine was obtained in the same manner as in Example 0559-3 except that 6-chloro-N-(2,4-dimethoxybenzyl)-5-(pentan-3-yl)pyridazine-3-amine was used instead of the 5-(1-((tert-butyldimethylsilyl)oxy)-2-methylpropan-2-yl)-6-chloro-N-(2,4-dimethoxybenzyl)pyridazine-3-amine used in Example 0559-3.
MSm/z(M+H):316.

<0585-4>

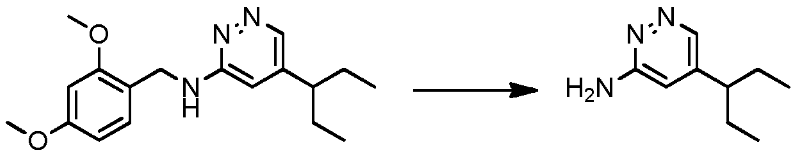

5-(Pentan-3-yl)pyridazine-3-amine was obtained in the same manner as in Example 0559-4 except that N-(2,4-dimethoxybenzyl)-5-(pentan-3-yl)pyridazine-3-amine was used instead of the 5-(1-((tert-butyldimethylsilyl)oxy)-2-methylpropan-2-yl)-N-(2,4-dimethoxybenzyl)pyridazine-3-amine used in Example 0559-4.
MSm/z(M+H):166.

<0585-5>

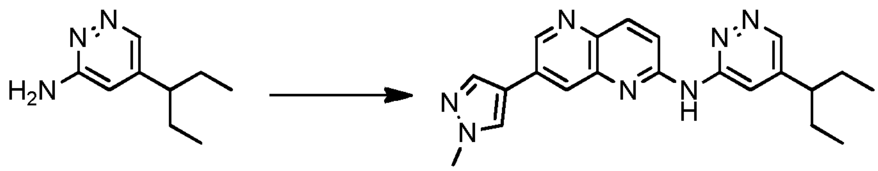

7-(1-Methyl-1H-pyrazol-4-yl)-N-(5-(pentan-3-yl)pyridazin-3-yl)-1,5-naphthyridine-2-amine was obtained in the same manner as in Example 0554-3 except that 5-(pentan-3-yl)pyridazine-3-amine was used instead of the 5-morpholinopyridazine3-amine used in Example 0554-3.
$^1$H-NMR(DMSO-$d_6$)δ:10.68(1H,s),9.03(1H,d,J=1.8Hz),8.78(1H,d,J=1.8Hz),8.63(1H,d,J=1.8Hz),8.45(1H,s),8.22(1H,d,J=9.0Hz),8.16(2H,brs),7.72(1H,d,J=9.0Hz),3.93(3H,s),3.52-3.30(1H,m),1.94-1.54(4H,m),0.83(6H,t,J=7.2Hz).
MSm/z(M+H):374.

<Example 0586>

<0586-1>

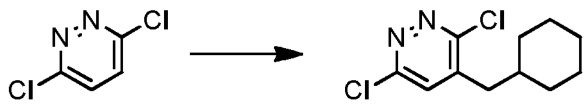

3,6-Dichloro-4-(cyclohexylmethyl)pyridazine was obtained in the same manner as in Example 0585-1 except that 2-cyclohexylacetic acid was used instead of the 2-ethylbutanoic acid used in Example 0585-1.
MSm/z(M+H):245.

<0586-2>

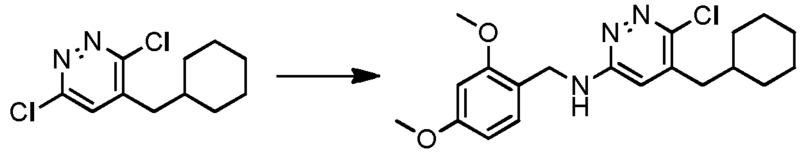

6-Chloro-5-(cyclohexylmethyl)-N-(2,4-dimethoxybenzyl)pyridazine-3-am was obtained in the same manner as in Example 0559-2 except that 3,6-dichloro-4-(cyclohexylmethyl)pyridazine was used instead of the 4-(1-((tert-butyldimethylsilyl)oxy)-2-methylpropan-2-yl)-3,6-dichloropyridazine used in Example 0559-2.
MSm/z(M+H):376.

<0586-3>

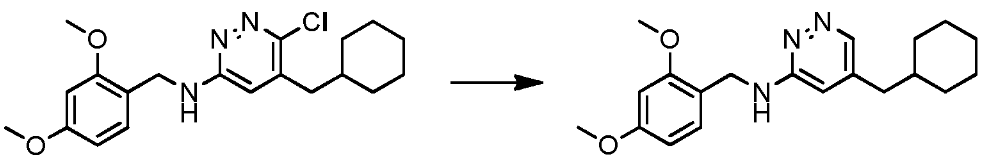

5-(Cyclohexylmethyl)-N-(2,4-dimethoxybenzyl)pyridazine-3-amine was obtained in the same manner as in Example 0559-3 except that 6-chloro-5-(cyclohexylmethyl)-N-(2,4-dimethoxybenzyl)pyridazine-3-amine was used instead of the 5-(1-((tert-butyldimethylsilyl)oxy)-2-methylpropan-2-yl)-6-chloro-N-(2,4-dimethoxybenzyl)pyridazine-3-amine used in Example 0559-3.
MSm/z(M+H):342.

<0586-4>

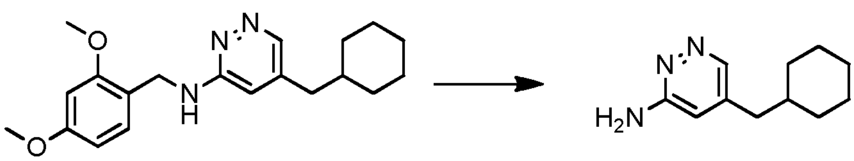

5-(Cyclohexylmethyl)pyridazine-3-amine was obtained in the same manner as in Example 0559-4 except that 5-(cyclohexylmethyl)-N-(2,4-dimethoxybenzyl)pyridazine-3-amine was used instead of the 5-(1-((tert-butyldimethylsilyl)oxy)-2-methylpropan-2-yl)-N-(2,4-dimethoxybenzyl)pyridazine-3-amine used in Example 0559-4.
MSm/z(M+H):192.

<0586-5>

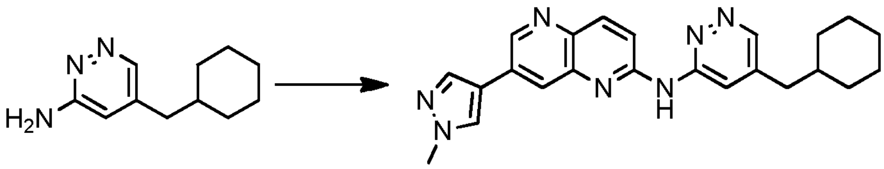

N-(5-(cyclohexylmethyl)pyridazin-3-yl)-7-(1-methyl-1H-pyrazol-4-yl)-1,5-naphthyridine-2-amine was obtained in the same manner as in Example 0554-3 except that 5-(cyclohexylmethyl)pyridazine-3-amine was used instead of the 5-morpholinopyridazine-3-amine used in Example 0554-3.
$^1$H-NMR(DMSO-$d_6$)δ:10.67(1H,s),9.03(1H,d,J=2.1Hz),8.75(1H,d,J=2.1Hz),8.61(1H,brs),8.44(1H,s),8.21(1H,d,J=9.3Hz ),8.19(1H,s),8.13(1H,s),7.70(1H,d,J=9.3Hz),3.93(3H,s),2.60(2H,d,J=6.6Hz),1.75-1.55(6H,m),1.34-0.91(5H,m).
MSm/z(M+H):400.

<Example 0587>

<0587-1>

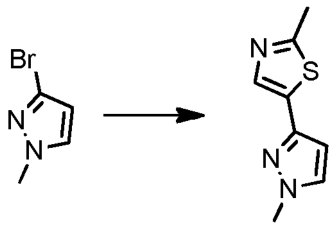

2-Methyl-5-(1-methyl-1H-pyrazol-3-yl)thiazole was obtained in the same manner as in Example 0583-1 except that 5-bromo-2-methylthiazole was used instead of the 5-bromothiazole used in Example 0583-1.
MSm/z(M+H):180.

<0587-2>

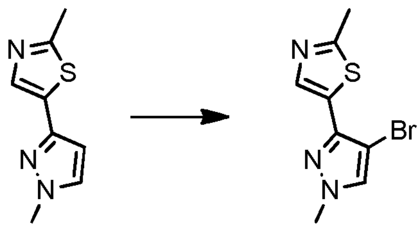

5-(4-Bromo-1-methyl-1H-pyrazol-3-yl)-2-methylthiazole was obtained in the same manner as in Example 0555-2 except that 2-methyl-5-(1-methyl-1H-pyrazol-3-yl)thiazole was used instead of the 3-methyl-5-(1-methyl-1H-pyrazol-3-yl)pyridine used in Example 0555-2.
MSm/z(M+H):258.

<0587-3>

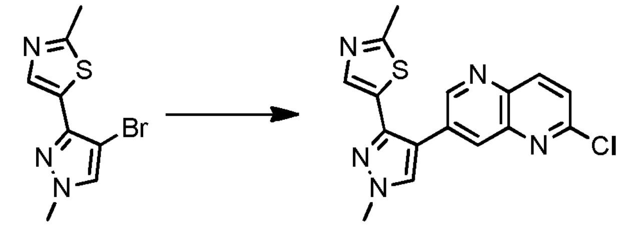

5-(4-(6-Chloro-1,5-naphthyridin-3-yl)-1-methyl-1H-pyrazol-3-yl)-2-methylthiazole was obtained in the same manner as in Example 0478-3 except that 5-(4-bromo-1-methyl-1H-pyrazol-3-yl)-2-methylthiazole was used instead of the 1-(4-(4-iodo-1H-pyrazol-1-yl)piperidin-1-yl)ethanone used in Example 0478-3.
MSm/z(M+H):342.

<0587-4>

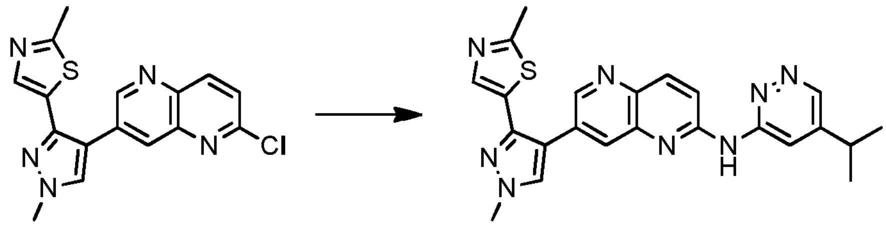

N-(5-isopropylpyridazin-3-yl)-7-(1-methyl-3-(2-methylthiazol-5-yl)-1H-pyrazol-4-yl)-1,5-naphthyridine-2-amine was obtained in the same manner as in Example 0555-4 except that 5-(4-(6-chloro-1,5-naphthyridin-3-yl)-1-methyl-1H-pyrazol-3-yl)-2-methylthiazole was used instead of the 2-chloro-7-(1-methyl-3-(5-methylpyridin-3-yl)-1H-pyrazol-4-yl)-1,5-naphthyridine used in Example 0555-4.
$^1$H-NMR($CDCl_3$)δ:8.85(1H,brs),8.77(1H,brs),8.75(1H,d,J=2.1Hz),8.28(1H,d,J=9.0Hz),8.07(1H,d,J=2.1H z),7.67(1H,d,J=9.0Hz),7.61(1H,s),7.58(1H,s),4.02(3H,s),3.07-2.94(1H,m),2.67(3H,s),1.37(6H,d,J=7.2Hz).
MSm/z(M+H):443.

<Example 0588>

<0588-1>

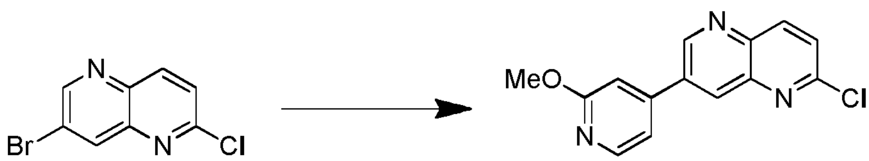

2-Chloro-7-(2-methoxypyridin-4-yl)-1,5-naphthyridine was obtained as a white solid in the same manner as in Example 0646-1 except that (2-methoxypyridin-4-yl)boronic acid was used instead of the 1-(tert-butoxycarbonyl)-4-(4,4,5,5-tetramethyl-1,3,2-dioxaborolan-2-yl)pyrazole used in Example 0646-1.
MSm/z(M+H):272.

<0588-2>

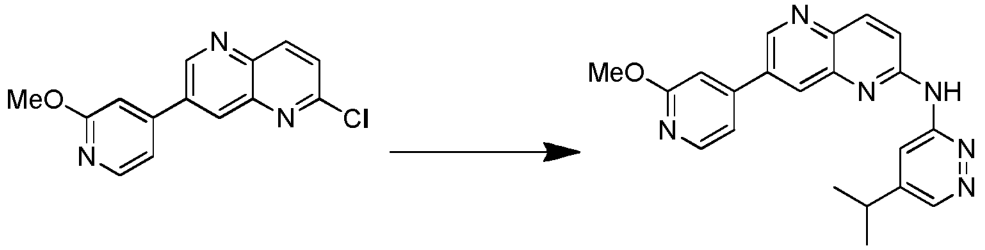

N-(5-isopropylpyridazin-3-yl)-7-(2-methoxypyridin-4-yl)-1,5-naphthyridine-2-amine was obtained as a white solid in the same manner as in Example 0411-3 except that 2-chloro-7-(2-methoxypyridin-4-yl)-1,5-naphthyridine was used instead of the 2-chloro-7-(1-((6-methylpyridin-2-yl)methyl)-1H-pyrazol-4-yl)-1,5-naphthyridine used in Example 0411-3.
$^1$H-NMR(DMSO-$d_6$)δ:10.84(1H,s),9.14(1H,d,J=2.0Hz),8.88(1H,d,J=2.0Hz),8.79(1H,d,J=1.3Hz),8.47(1H,d,J=2.0Hz),8.34-8.31(2H,m),7.82(1H,d,J=9.2Hz),7.56(1H,dd,J=5.3,1.3Hz),7.40(1H,s),3.94(3H,s),3.07-3.05(1H,m),1.33(6H,d,J=7.3Hz).
MSm/z(M+H):373.

<Example 0589>

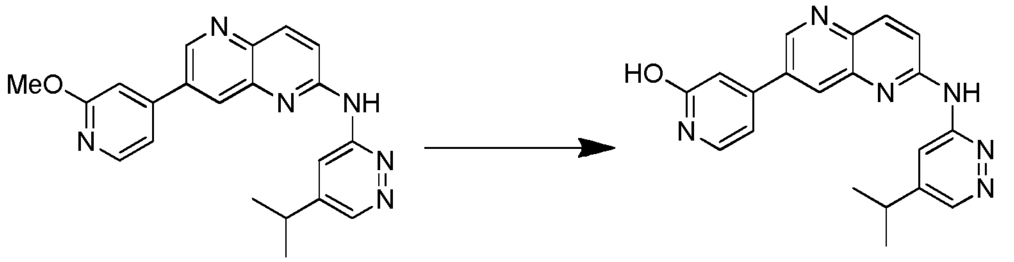

A mixture of N-(5-isopropylpyridazin-3-yl)-7-(2-methoxypyridin-4-yl)-1,5-naphthyridine-2-amine (29 mg), and 48% hydrobromic acid (2 mL) was stirred at 80°C for 9 hours. A saturated sodium hydrogen carbonate aqueous solution was added to the reaction mixture, and the solvent was distilled off under reduced pressure. The obtained residue was purified by silica gel column chromatography (hexane-ethyl acetate, methanol-chloroform), thereby obtaining 4-(6-((5-isopropylpyridazin-3-yl)amino)-1,5-naphthyridin-3-yl)pyridin-2-ol (7 mg) as a white solid.
$^1$H-NMR(DMSO-$d_6$)δ:11.77(1H,s),10.83(1H,s),9.05(1H,d,J=2.0Hz),8.88(1H,d,J=2.0Hz),8.79(1H,d,J=1.3Hz),8.38(1H,d,J=2.0Hz),8.30(1H,d,J=9.2Hz),7.80(1H,d,J=9.2Hz),7.55(1H,d,J=7.3Hz),6.87(1H,d,J=1.3Hz),6.73(1H,d ,J=7.3Hz),3.07-3.04(1H,m),1.33(6H,d,J=7.3Hz).
MSm/z(M+H):359.

<Example 0590>

<0590-1>

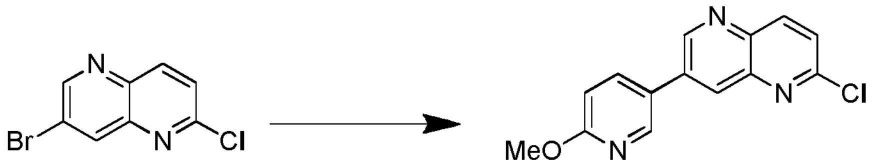

2-Chloro-7-(6-methoxypyridin-3-yl)-1,5-naphthyridine was obtained in the same manner as in Example 0646-1 except that (6-methoxypyridin-3-yl)boronic acid was used instead of the 1-(tert-butoxycarbonyl)-4-(4,4,5,5-tetramethyl-1,3,2-dioxaborolan-2-yl)pyrazole used in Example 0646-1.
MSm/z(M+H):272.

<0590-2>

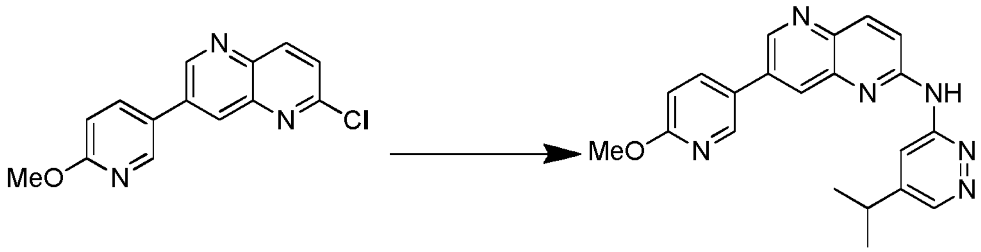

N-(5-isopropylpyridazin-3-yl)-7-(6-methoxypyridin-3-yl)-1,5-naphthyridine-2-amine was obtained as a white solid in the same manner as in Example 0411-3 except that 2-chloro-7-(6-methoxypyridin-3-yl)-1,5-naphthyridine was used instead of the 2-chloro-7-(1-((6-methylpyridin-2-yl)methyl)-1H-pyrazol-4-yl)-1,5-naphthyridine used in Example 0411-3.
$^1$H-NMR(DMSO-$d_6$)$\delta$:10.78(1H,s),9.09(1H,d,J=2.0Hz),8.87(1H,d,J=2.0Hz),8.78(1H,d,J=2.0Hz),8.76(1H,d,J=2.6Hz),8.36(1H,d,J=2.0Hz),8.30(1H,d,J=2.6Hz),8.27(1H,d,J=2.0Hz),7.77(1H,d,J=8.9Hz),7.02(1H,d,J=8.9Hz),3.94(3H,s),3.09-2.98(1H,m),1.32(6H,d,J=6.6Hz).
MSm/z(M+H):373.

<Example 0591>

<0591-1>

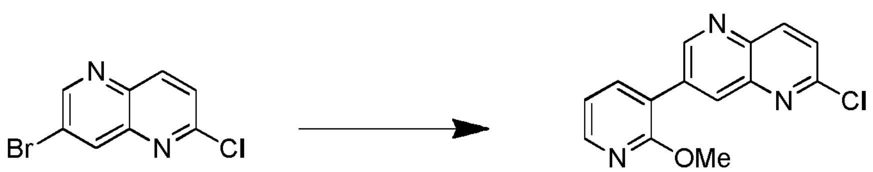

2-Chloro-7-(2-methoxypyridin-3-yl)-1,5-naphthyridine was obtained in the same manner as in Example 0646-1 except that (2-methoxypyridin-3-yl)boronic acid was used instead of the 1-(tert-butoxycarbonyl)-4-(4,4,5,5-tetramethyl-1,3,2-dioxaborolan-2-yl)pyrazole used in Example 0646-1.
MSm/z(M+H):272.

<0591-2>

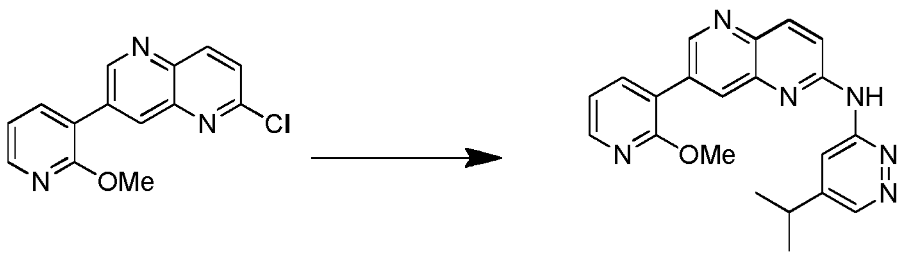

N-(5-isopropylpyridazin-3-yl)-7-(2-methoxypyridin-3-yl)-1,5-naphthyridine-2-amine was obtained as a white solid in the same manner as in Example 0411-3 except that 2-chloro-7-(2-methoxypyridin-3-yl)-1,5-naphthyridine was used instead of the 2-chloro-7-(1-((6-methylpyridin-2-yl)methyl)-1H-pyrazol-4-yl)-1,5-naphthyridine used in Example 0411-3. $^1$H-NMR(DMSO-$d_6$)δ:10.78(1H,s),8.93(1H,d,J=2.0Hz),8.86(1H,d,J=2.0Hz),8.74(1H,d,J=1.3Hz),8.31-8.28(3H,m),8.03(1H,dd,J=7.3,2.0 Hz),7.80(1H,d,J=9.2Hz),7.20(1H,dd,J=7.3,4.6Hz),3.94(3H,s),3.06-2.97(1H,m),1.30(6H,d,J=7.3Hz).
MSm/z(M+H):373.

<Example 0592>

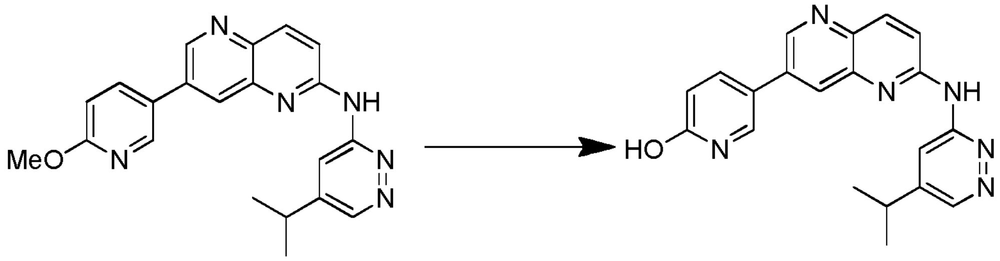

5-(6-((5-Isopropylpyridazin-3-yl)amino)-1,5-naphthyridin-3-yl)pyridin-2-ol was obtained as a pale yellow solid cin the same manner as in Example 0589 except that N-(5-isopropylpyridazin-3-yl)-7-(6-methoxypyridin-3-yl)-1,5-naphthyridine-2-amine was used instead of the N-(5-isopropylpyridazin-3-yl)-7-(2-methoxypyridin-4-yl)-1,5-naphthyridine-2-amine used in Example 0589.
$^1$H-NMR(DMSO-$d_6$)δ:12.03(1H,s),10.74(1H,s),9.01(1H,d,J=2.6Hz),8.87(1H,d,J=1.3Hz),8.77(1H,d,J=1.3Hz),8.26-8.23(2H,m),8.11-8.05 (2H,m),7.73(1H,d,J=9.2Hz),6.52(1H,d,J=10.6Hz),3.08-2.98(1H,m),1.32(6H,d,J=6.6Hz).
MSm/z(M+H):359.

<Example 0593>

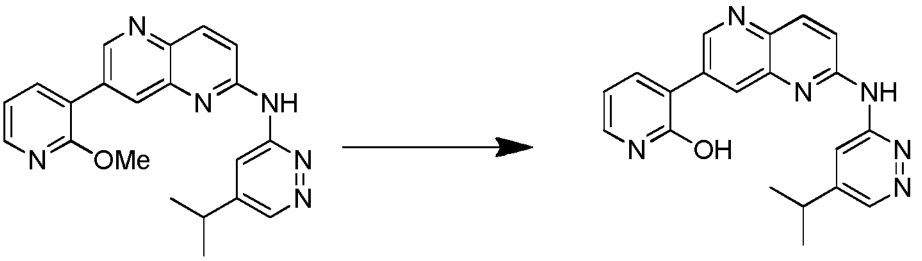

3-(6-((5-Isopropylpyridazin-3-yl)amino)-1,5-naphthyridin-3-yl)pyridin-2-ol was obtained as a pale yellow solid in the same manner as in Example 0589 except that N-(5-isopropylpyridazin-3-yl)-7-(2-methoxypyridin-3-yl)-1,5-naphthyridine-2-amine was used instead of the N-(5-isopropylpyridazin-3-yl)-7-(2-methoxypyridin-4-yl)-1,5-naphthyridine-2-amine used in Example 0589.
$^1$H-NMR(DMSO-d$_6$)δ:11.99(1H,s),10.73(1H,s),9.06(1H,d,J=2.0Hz),8.86(1H,d,J=2.0Hz),8.76(1H,d,J=1.3Hz),8.51(1H,d,J=1.3Hz),8.26(1H,d,J=9.2Hz),7.98(1H,dd,J=6.9,2.2Hz),7.76(1H,d,J=9.2Hz),7.52(1H,dd,J=5.9,2.2Hz),6.40(1H,dd,J=6.9,5.9Hz),3.06-2.97(1H,m),1.31(6H,d,J=6.6Hz).
MSm/z(M+H):359.

<Example 0594>

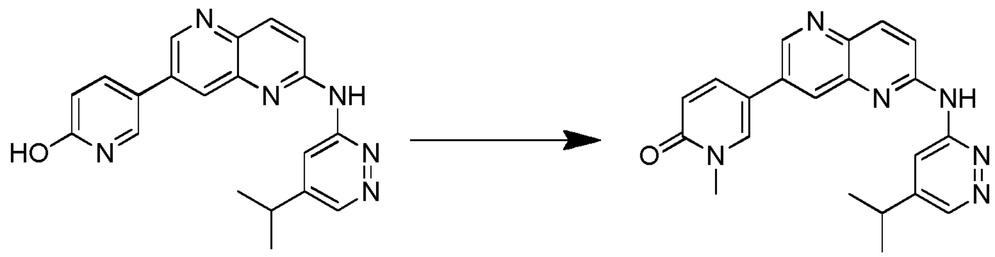

A mixture of 5-(6-((5-isopropylpyridazin-3-yl)amino)-1,5-naphthyridin-3-yl)pyridin-2-ol (10 mg), acetone (5 mL), iodomethane (3 μL), and potassium carbonate (12 mg) was stirred at room temperature for 5.5 hours. Methanol (1 mL) was added to the reaction mixture, followed by stirring at room temperature for 18 hours. The solvent was distilled off under reduced pressure, and the obtained residue was purified by silica gel column chromatography (chloroform-methanol), thereby obtaining 5-(6-((5-isopropylpyridazin-3-yl)amino)-1,5-naphthyridin-3-yl)-1-methylpyridin-2(1H)-one (7 mg) as a pale yellow solid.
$^1$H-NMR(DMSO-d$_6$)δ:10.75(1H,s),9.02(1H,d,J=2.0Hz),8.87(1H,d,J=2.0Hz),8.75(1H,d,J=2.0Hz),8.48(1H,d,J=2.6Hz),8.28-8.24(2H,m),8.08(1H,dd,J=9.6,2.6Hz),7.74(1H,d,J=9.2Hz),6.58(1H,d,J=9.2Hz),3.57(3H,s),3.08-2.99(1H,m),1.33(6H,d,J=6.6Hz).
MSm/z(M+H):373.

<Example 0595>

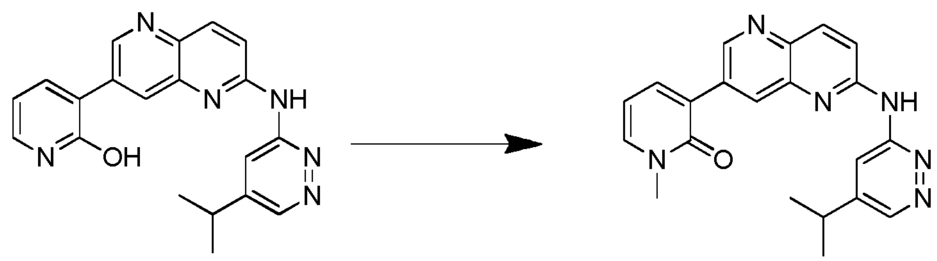

3-(6-((5-Isopropylpyridazin-3-yl)amino)-1,5-naphthyridin-3-yl)-1-methylpyridin-2(1H)-one was obtained as a pale yellow solid in the same manner as in Example 0594 except that 3-(6-((5-isopropylpyridazin-3-yl)amino)-1,5-naphthyridin-3-yl)pyridin-2-ol was used instead of the 5-(6-((5-isopropylpyridazin-3-yl)amino)-1,5-naphthyridin-3-yl)pyridin-2-ol used in Example 0594.
$^1$H-NMR(DMSO-d$_6$)δ:10.74(1H,s),9.03(1H,d,J=2.0Hz),8.86(1H,d,J=2.0Hz),8.74(1H,d,J=2.0Hz),8.46(1H,d,J=2.0Hz),8.26(1H,d,J=9.2Hz),7.94(1H,dd,J=6.9,2.3Hz),7.87(1H,dd,J=6.6,2.3Hz),7.77(1H,d,J=9.2Hz),6.43(1H,dd,J=6.9,6.6Hz),3.57(3H,s),3.06-2.97(1H,m),1.31(6H,d,J=6.6Hz).
MSm/z(M+H):373.

<Example 0596>

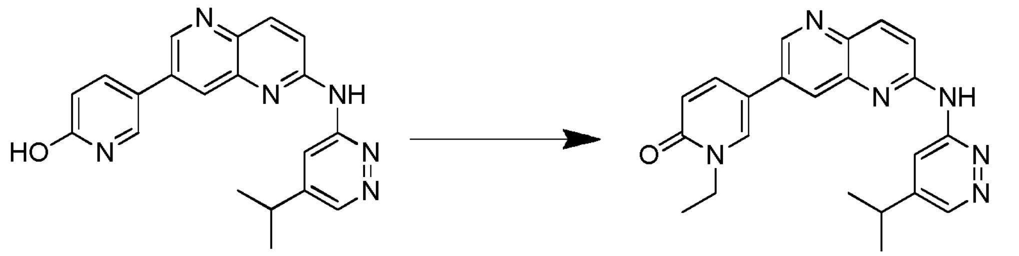

A mixture of 5-(6-((5-isopropylpyridazin-3-yl)amino)-1,5-naphthyridin-3-yl)pyridin-2-ol (20 mg), acetone (2 mL), methanol (2 mL), potassium carbonate (25 mg), and iodoethane (7 μL) was stirred at room temperature for 17.5 hours, and stirred for 5 hours under heating to reflux. Iodoethane (7 μL) was added to the reaction mixture, followed by stirring for 3 hours under heating to reflux. The reaction mixture was cooled to room temperature, and the solvent was distilled off under reduced pressure. The obtained residue was purified by silica gel column chromatography (hexane-ethyl acetate-methanol), and purified by silica gel column chromatography (chloroform-methanol, NH silica), thereby obtaining 1-ethyl-5-(6-((5-isopropylpyridazin-3-yl)amino)-1,5-naphthyridin-3-yl)pyridin-2(1H)-one (7 mg) as a pale yellow solid.
$^1$H-NMR(DMSO-$d_6$)δ:10.74(1H,s),9.04(1H,d,J=2.0Hz),8.87(1H,d,J=2.0Hz),8.74(1H,d,J=2.0Hz),8.46(1H,d,J=2.6Hz),8.28-8.24(2H,m),8.06(1H,dd,J=9.2,2.6Hz),7.74(1H,d,J=9.2Hz),6.57(1H,d,J=9.2Hz),4.05(2H,q,J=7.3Hz),3.09-2.99(1H,m),1.34-1.27(9H,m).
MSm/z(M+H):387.

<Example 0597>

<0597-1>

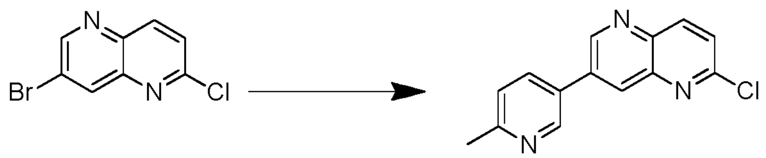

2-Chloro-7-(6-methylpyridin-3-yl)-1,5-naphthyridine was obtained in the same manner as in Example 0421-1 except that 5-bromo-2-methylpyridine was used instead of the N-(4-bromopyridin-2-yl)acetamide used in Example 0421-1.
MSm/z(M+H):256.

<0597-2>

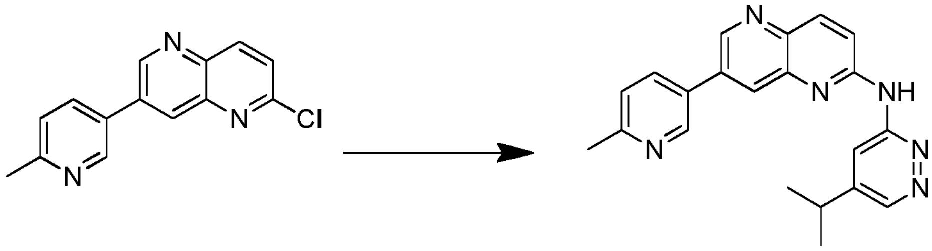

N-(5-isopropylpyridazin-3-yl)-7-(6-methylpyridin-3-yl)-1,5-naphthyridine-2-amine was obtained as a pale yellow solid in the same manner as in Example 0411-3 except that 2-chloro-7-(6-methylpyridin-3-yl)-1,5-naphthyridine was used instead of the 2-chloro-7-(1-((6-methylpyridin-2-yl)methyl)-1H-pyrazol-4-yl)-1,5-naphthyridine used in Example 0411-3.
$^1$H-NMR(DMSO-$d_6$)δ:10.80(1H,s),9.10(1H,d,J=2.0Hz),9.00(1H,d,J=2.0Hz),8.87(1H,d,J=2.0Hz),8.78(1H,d,J=2.0Hz),8.41(1H,d,J=2.0Hz),8.31(1H,d,J=9.2Hz),8.23(1H,dd,J=7.9,2.0Hz),7.79(1H,d,J=9.2Hz),7.45(1H,d,J=7.9Hz),3.06-3.03(1H,m),2.57(3H,s),1.32(6H,d,J=7.3Hz).
MSm/z(M+H):357.

<Example 0598>

<0598-1>

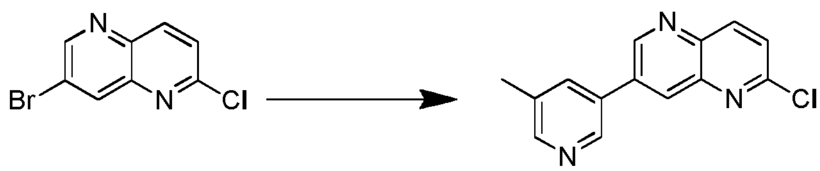

2-Chloro-7-(5-methylpyridin-3-yl)-1,5-naphthyridine was obtained in the same manner as in Example 0421-1 except that 3-bromo-5-methylpyridine was used instead of the N-(4-bromopyridin-2-yl)acetamide used in Example 0421-1.
MSm/z(M+H):256.

<0598-2>

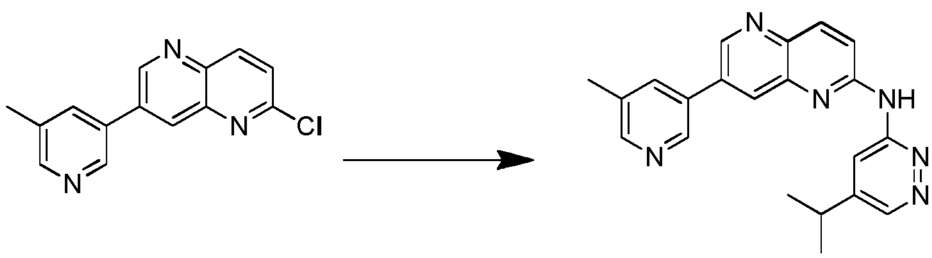

N-(5-isopropylpyridazin-3-yl)-7-(5-methylpyridin-3-yl)-1,5-naphthyridine-2-amine was obtained as a pale yellow solid in the same manner as in Example 0411-3 except that 2-chloro-7-(5-methylpyridin-3-yl)-1,5-naphthyridine was used instead of the 2-chloro-7-(1-((6-methylpyridin-2-yl)methyl)-1H-pyrazol-4-yl)-1,5-naphthyridine used in Example 0411-3.
$^1$H-NMR(DMSO-$d_6$)δ:10.80(1H,s),9.11(1H,d,J=2.0Hz),8.93(1H,d,J=2.0Hz),8.88(1H,d,J=2.0Hz),8.78(1H,d,J=2.0Hz),8.53(1H,d,J=1.3Hz),8.44(1H,d,J=1.3Hz),8.31(1H,d,J=9.2Hz),8.17(1H,s),7.80(1H,d,J=9.2Hz),3.10-3.01(1H,m),2.43(3H,s),1.32(6H,d,J=6.6Hz).
MSm/z(M+H):357.

<Example 0599>

<0599-1>

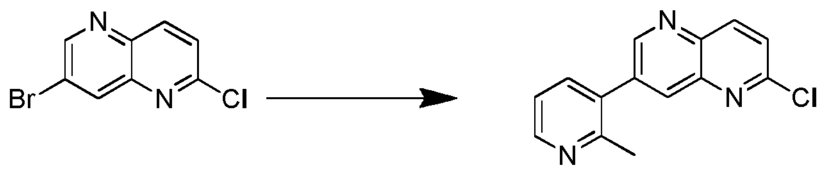

2-Chloro-7-(2-methylpyridin-3-yl)-1,5-naphthyridine was obtained in the same manner as in Example 0421-1 except that 3-bromo-2-methylpyridine was used instead of the N-(4-bromopyridin-2-yl)acetamide used in Example 0421-1.
MSm/z(M+H):256.

<0599-2>

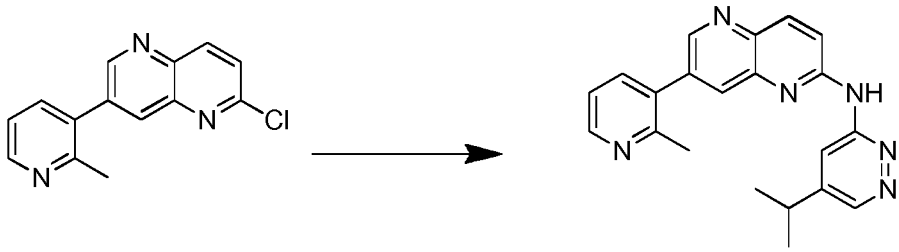

N-(5-isopropylpyridazin-3-yl)-7-(2-methylpyridin-3-yl)-1,5-naphthyridine-2-amine was obtained as a pale yellow solid in the same manner as in Example 0411-3 except that 2-chloro-7-(2-methylpyridin-3-yl)-1,5-naphthyridine was used instead of the 2-chloro-7-(1-((6-methylpyridin-2-yl)methyl)-1H-pyrazol-4-yl)-1,5-naphthyridine used in Example 0411-3.
$^1$H-NMR(DMSO-$d_6$)$\delta$:10.81(1H,s),8.86(1H,d,J=2.0Hz),8.78(1H,d,J=2.0Hz),8.76(1H,d,J=1.3Hz),8.57(1H,dd,J=4.8,1.3Hz),8.32(1H,d,J=9.2Hz),8.18(1H,d,J=2.0Hz),7.85-7.80(2H,m),7.40(1H,dd,J=7.6,4.8Hz),3.06-2.95(1H,m),2.50(3H,s), 1.29(6H,d,J=6.6Hz).
MSm/z(M+H):357.

<Example 0600>

<0600-1>

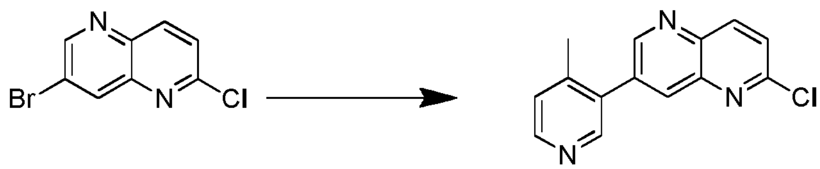

2-Chloro-7-(4-methylpyridin-3-yl)-1,5-naphthyridine was obtained in the same manner as in Example 0421-1 except that 3-bromo-4-methylpyridine was used instead of the N-(4-bromopyridin-2-yl)acetamide used in Example 0421-1.
MSm/z(M+H):256.

<0600-2>

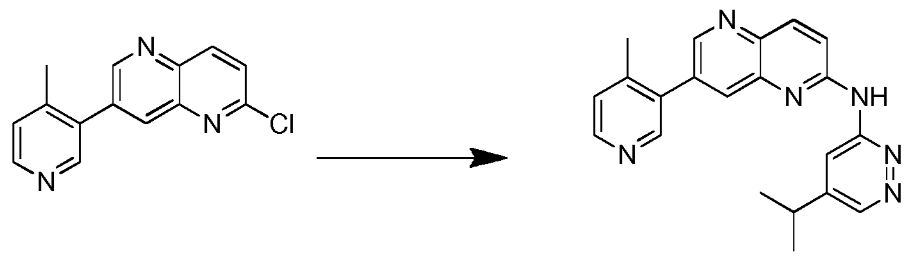

N-(5-isopropylpyridazin-3-yl)-7-(4-methylpyridin-3-yl)-1,5-naphthyridine-2-amine was obtained as a pale yellow solid in the same manner as in Example 0411-3 except that 2-chloro-7-(4-methylpyridin-3-yl)-1,5-naphthyridine was used instead of the 2-chloro-7-(1-((6-methylpyridin-2-yl)methyl)-1H-pyrazol-4-yl)-1,5-naphthyridine used in Example 0411-3.
$^1$H-NMR(DMSO-$d_6$)δ:10.82(1H,s),8.86(1H,d,J=2.0Hz),8.79(1H,d,J=2.0Hz),8.77(1H,d,J=2.0Hz),8.56(1H,s),8.54(1H,d,J =5.3Hz),8.33(1H,d,J=9.2Hz),8.20(1H,d,J=2.0Hz),7.82(1H,d,J=9.2Hz),7.44(1H,d,J=5.3Hz),3.06-2.95(1H,m),2.35(3H,s) ,1.29(6H,d,J=7.3Hz).
MSm/z(M+H):357.

<Example 0601>

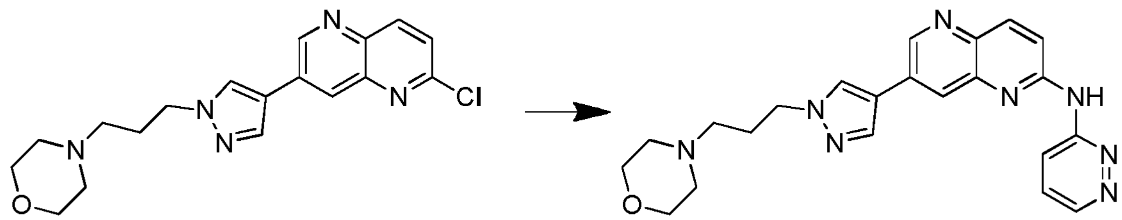

7-(1-(3-Morpholinopropyl)-1H-pyrazol-4-yl)-N-(pyridazin-3-yl)-1,5-naphthyridine-2-amine was obtained as a pale yellow solid in the same manner as in Example 0368-1 except that pyridazine-3-amine was used instead of the 5-isopropylpyridazine-3-amine used in Example 0368-1.
$^1$H-NMR(DMSO-$d_6$)δ:10.81(1H,s),9.05(1H,d,J=2.0Hz),8.94(1H,dd,J=9.2,1.3Hz),8.88(1H,dd,J=4.6,1.3Hz),8.53(1H,s),8.29(1H,d,J=2.0Hz),8.22(1H,d,J=9.2Hz),8.18(1H,s),7.70-7.63(2H,m),4.20(2H,t,J=6.9Hz),3.58(4H,t,J=4.6Hz),2.36-2.27(6H,m),2.05-1.96(2H,m).
MSm/z(M+H):417.

<Example 0602>

<0602-1>

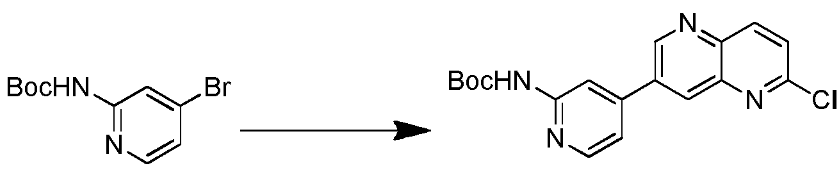

tert-Butyl (4-(6-chloro-1,5-naphthyridin-3-yl)pyridin-2-yl)carbamate was obtained in the same manner as in Example 0421-1 except that tert-butyl (4-bromopyridin-2-yl)carbamate was used instead of the N-(4-bromopyridin-2-yl)acetamide used in Example 0421-1.
MSm/z(M+H):301.

<0602-2>

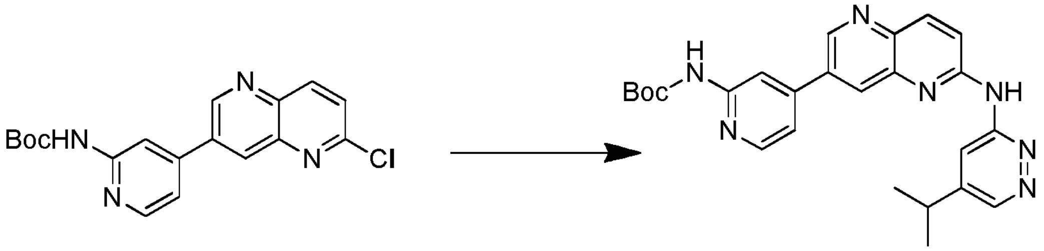

tert-Butyl (4-(6-((5-isopropylpyridazin-3-yl)amino)-1,5-naphthyridin-3-yl)pyridin-2-yl)carbamate was obtained in the same manner as in Example 0411-3 except that tert-butyl (4-(6-chloro-1,5-naphthyridin-3-yl)pyridin-2-yl)carbamate was used instead of the 2-chloro-7-(1-((6-methylpyridin-2-yl)methyl)-1H-pyrazol-4-yl)-1,5-naphthyridine used in Example 0411-3.
MSm/z(M+H):458.

<0602-3>

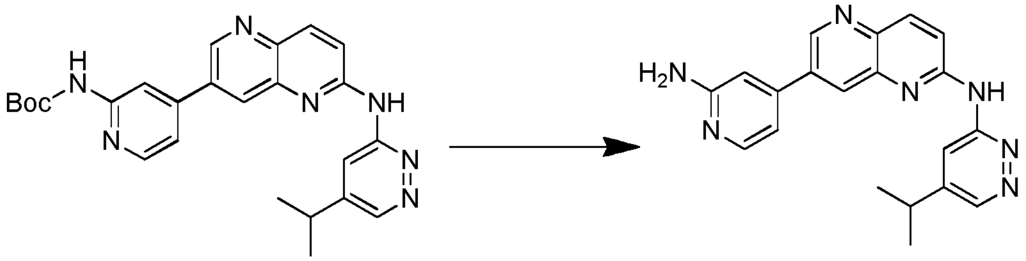

A mixture of tert-butyl (4-(6-((5-isopropylpyridazin-3-yl)amino)-1,5-naphthyridin-3-yl)pyridin-2-yl)carbamate (19 mg), 1,4-dioxane (2 mL), and 2 mol/L hydrochloric acid (1 mL) was stirred at 80°C for 1 hour. A saturated sodium hydrogen carbonate aqueous solution and water were added to the reaction mixture, and the solid matter was collected by filtration. The obtained solid matter was purified by preparative thin layer silica gel column chromatography (chloroform-methanol, NH silica), thereby obtaining 7-(2-aminopyridin-4-yl)-N-(5-isopropylpyridazin-3-yl)-1,5-naphthyridine-2-amine (7 mg) as a pale yellow solid.
$^1$H-NMR(DMSO-$d_6$)δ:10.82(1H,s),9.00(1H,d,J=2.0Hz),8.88(1H,d,J=2.0Hz),8.78(1H,d,J=2.0Hz),8.33-8.28(2H,m),8.07(1H,d,J=5.3Hz),7.80(1H,d,J=9.2Hz),7.00(1H,dd,J=5.3,2.0Hz),6.88(1H,s),6.12(2H,s), 3.10-3.01(1H,m),1.32(6H,d,J=6.6Hz).
MSm/z(M+H):358.

<Example 0603>

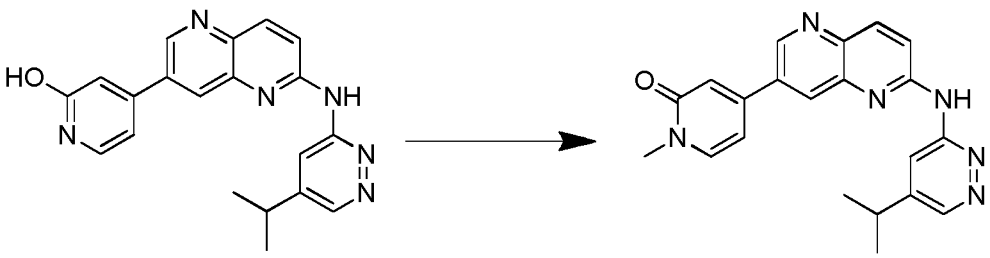

A mixture of 4-(6-((5-isopropylpyridazin-3-yl)amino)-1,5-naphthyridin-3-yl)pyridin-2-ol (10 mg), acetone (1 mL), methanol (1 mL), potassium carbonate (12 mg), and iodomethane (3 μL) was stirred at room temperature for 3.5 hours. The solvent was distilled off under reduced pressure, and the obtained residue was purified by preparative thin layer silica gel column chromatography (chloroform-methanol, NH silica), thereby obtaining 4-(6-((5-isopropylpyridazin-3-yl)amino-1,5-naphthyridin-3-yl)-1-methylpyridin-2(1H)-one (6 mg) as a pale yellow solid.
$^1$H-NMR(DMSO-$d_6$)δ:10.83(1H,s),9.07(1H,d,J=2.0Hz),8.88(1H,d,J=2.0Hz),8.79(1H,d,J=2.0Hz),8.40(1H,d,J=2.0Hz),8.30(1H,d,J=8.9Hz),7.88(1H,d,J=7.3Hz),7.81(1H,d,J=8.9Hz),6.97(1H,d,J=2.0Hz),6.81(1H,dd,J=7.3,2.0Hz),3.50(3H,s),3.11-3.01(1H,m),1.33(6H,d,J=6.6Hz).
MSm/z(M+H):373.

<Example 0604>

<0604-1>

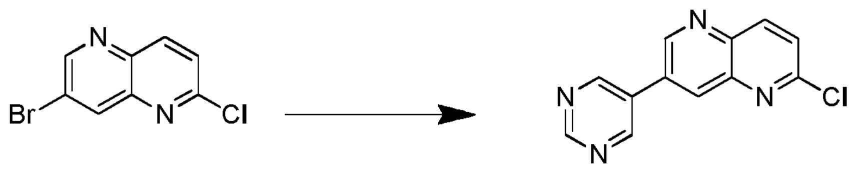

2-Chloro-7-(pyrimidin-5-yl)-1,5-naphthyridine was obtained in the same manner as in Example 0421-1 except that 5-bromopyrimidine was used instead of the N-(4-bromopyridin-2-yl)acetamide used in Example 0421-1.
MSm/z(M+H):243.

<0604-2>

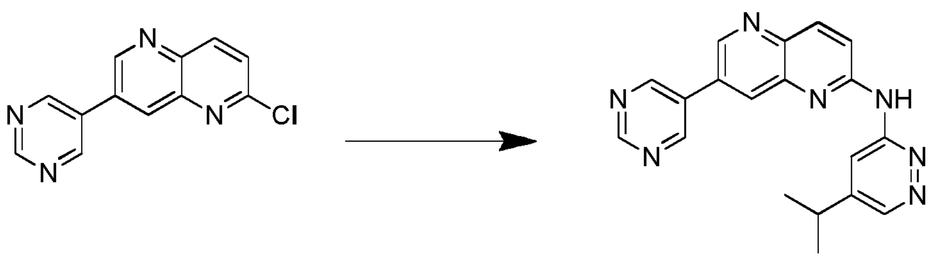

N-(5-isopropylpyridazin-3-yl)-7-(pyrimidin-5-yl)-1,5-naphthyridine-2-amine was obtained in the same manner as in Example 0411-3 except that 2-chloro-7-(pyrimidin-5-yl)-1,5-naphthyridine was used instead of the 2-chloro-7-(1-((6-methylpyridin-2-yl)methyl)-1H-pyrazol-4-yl)-1,5-naphthyridine used in Example 0411-3.
$^1$H-NMRpMSO-$d_6$)δ:10.84(1H,s),9.40(2H,s),9.30(1H,s),9.18(1H,d,J=2.0Hz),8.88(1H,d,J=2.0Hz),8.79(1H,d,J=2.0Hz),8.58(1H,d,J=2.0Hz),8.33(1H,d,J=9.2Hz),7.82(1H,d,J=9.2Hz),3.09-3.00(1H,m),1.33(6H,d,J=7.3Hz).
MSm/z(M+H):344.

<Example 0605>

<0605-1>

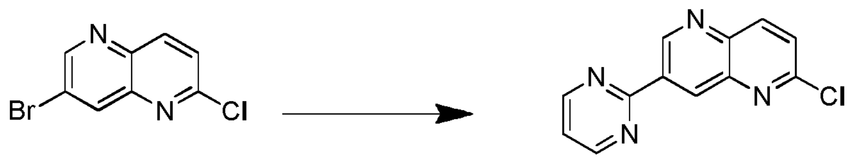

2-Chloro-7-(pyrimidin-2-yl)-1,5-naphthyridine was obtained in the same manner as in Example 0421-1 except that 2-bromopyrimidine was used instead of the N-(4-bromopyridin-2-yl)acetamide used in Example 0421-1.
MSm/z(M+H):243.

<0605-2>

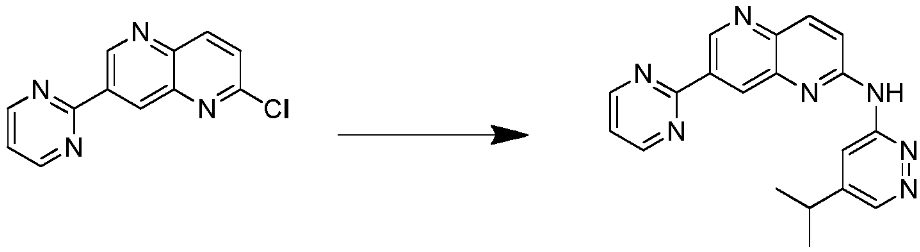

N-(5-isopropylpyridazin-3-yl)-7-(pyrimidin-2-yl)-1,5-naphthyridine-2-amine was obtained as a pale yellow solid in the same manner as in Example 0411-3 except that 2-chloro-7-(pyrimidin-2-yl)-1,5-naphthyridine was used instead of the 2-chloro-7-(1-((6-methylpyridin-2-yl)methyl)-1H-pyrazol-4-yl)-1,5-naphthyridine used in Example 0411-3.
$^1$H-NMR(DMSO-$d_6$)$\delta$:10.86(1H,s),9.67(1H,d,J=2.0Hz),9.05(2H,d,J=5.3Hz),8.95(1H,d,J=2.0Hz),8.89(1H,d,J=2.0Hz),8.81(1H,d,J=2.0Hz),8.34(1H,d,J=9.2Hz),7.84(1H,d,J=9.2Hz),7.59(1H,dd,J=5.3,5.3Hz),3.11-3.01(1H,m),1.34(6H,d,J=7.3Hz).
MSm/z(M+H):344.

<Example 0606>

<0606-1>

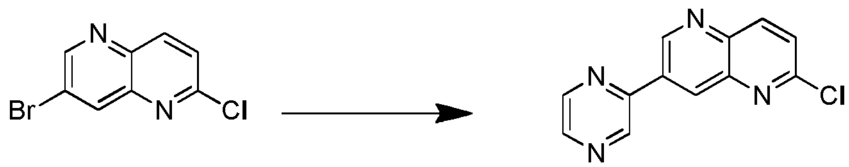

2-Chloro-7-(pyrazin-2-yl)-1,5-naphthyridine was obtained in the same manner as in Example 0421-1 except that 2-bromopyrazine was used instead of the N-(4-bromopyridin-2-yl)acetamide used in Example 0421-1.
MSm/z(M+H):243.

<0606-2>

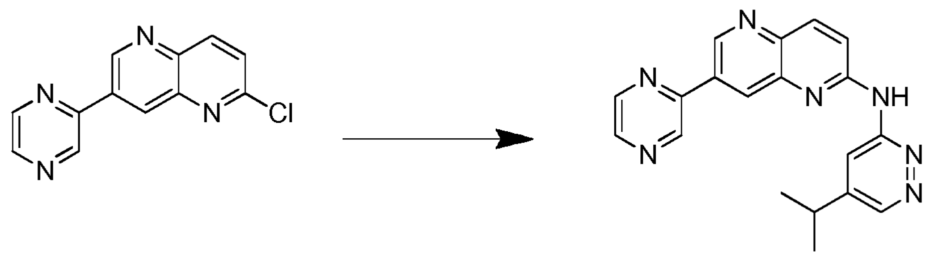

N-(5-isopropylpyridazin-3-yl)-7-(pyrazin-2-yl)-1,5-naphthyridine-2-amine was obtained as a white solid in the same manner as in Example 0411-3 except that 2-chloro-7-(pyrazin-2-yl)-1,5-naphthyridine was used instead of the 2-chloro-7-(1-((6-methylpyridin-2-yl)methyl)-1H-pyrazol-4-yl)-1,5-naphthyridine used in Example 0411-3.
$^1$H-NMR(DMSO-$d_6$)δ:10.85(1H,s),9.56(1H,d,J=2.0Hz),9.47(1H,d,J=2.0Hz),8.89(1H,d,J=2.0Hz),8.87-8.84(1H,m),8.80(2H,s),8.75(1H,d,J=2.0Hz),8.33(1H,d,J=9.2Hz),7.83(1H,d,J=9.2Hz),3.10-3.01(1H,m), 1.34(6H,d,J=6.6Hz).
MSm/z(M+H):344.

<Example 0607>

<0607-1>

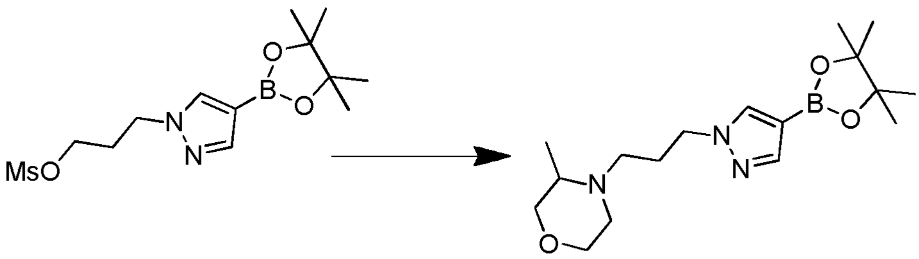

A mixture of 3-(4-(4,4,5,5-tetramethyl-1,3,2-dioxaborolan-2-yl)-1H-pyrazol-1-yl)propyl methanesulfonate (200 mg), 1,4-dioxane (5 mL), 3-methylmorpholine (247 mg), cesium carbonate (398 mg), and sodium iodide (27 mg) was stirred at 80°C for 5 hours. After the reaction mixture was cooled to room temperature, water and ethyl acetate were added thereto, the organic layer was collected by separation, washed with a saturated sodium chloride aqueous solution, and dried over anhydrous sodium sulfate, and the solvent was distilled off under reduced pressure, thereby obtaining 3-methyl-4-(3-(4-(4,4,5,5-tetramethyl-1,3,2-dioxaborolan-2-yl)-1H-pyrazol-1-yl)propyl)morpholine (64 mg).
MSm/z(M+H):336.

<0607-2>

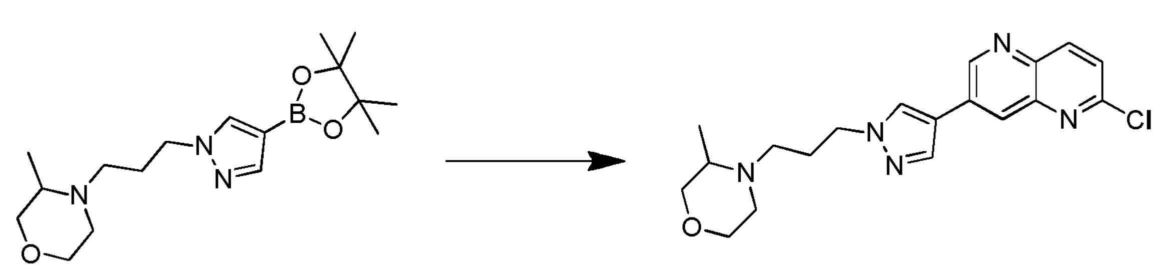

A mixture of 7-bromo-2-chloro-1,5-naphthyridine (46 mg), 3-methyl-4-(3-(4-(4,4,5,5-tetramethyl-1,3,2-dioxaborolan-2-yl)-1H-pyrazol-1-yl)propyl)morpholine (64 mg), sodium carbonate (40 mg), bis(di-tert-butyl (4-dimethylaminophenyl)phosphine)dichloropalladium(II) (7 mg), 1,4-dioxane (5 mL), and water (1 mL) was stirred at 80°C for 5 hour

in a nitrogen atmosphere. The reaction mixture was cooled to room temperature, and the solvent was distilled off under reduced pressure. The obtained residue was purified by silica gel column chromatography (hexane-ethyl acetate-methanol), thereby obtaining 4-(3-(4-(6-chloro-1,5-naphthyridin-3-yl)-1H-pyrazol-1-yl)propyl)-3-methylmorpholine (77 mg).
MSm/z(M+H):372.

<0607-3>

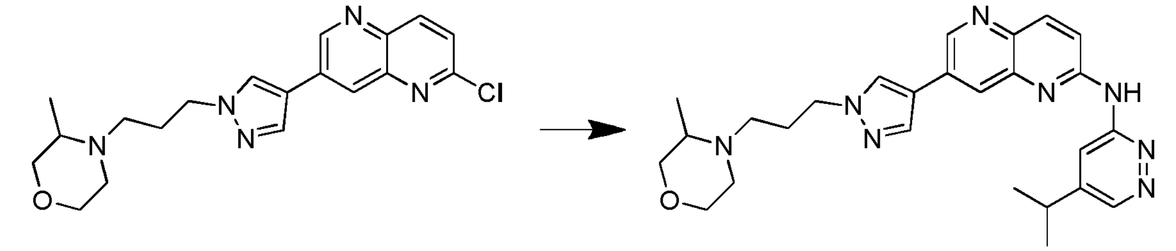

N-(5-isopropylpyridazin-3-yl)-7-(1-(3-(3-methylmorpholino)propyl)-1H-pyrazol-4-yl)-1,5-naphthyridine-2-amine was obtained as a yellow solid in the same manner as in Example 0411-3 except that 4-(3-(4-(6-chloro-1,5-naphthyridin-3-yl)-1H-pyrazol-1-yl)propyl)-3-methylmorpholine was used instead of the 2-chloro-7-(1-((6-methylpyridin-2-yl)methyl)-1H-pyrazol-4-yl)-1,5-naphthyridine used in Example 0411-3.
$^1$H-NMR(DMSO-$d_6$)δ:10.70(1H,s),9.04(1H,d,J=2.0Hz),8.86(1H,d,J=2.0Hz),8.73(1H,d,J=2.0Hz),8.52(1H,s),8.23-8.18(3H,m),7.69(1H,d, J=9.2Hz),4.20(2H,t,J=6.9Hz),3.70-3046(3H,m),3.13-2.99(2H,m),2.74-2.64(2H,m),2.31-2.26(1H,m),2.20-2.11(2H,m),2.02-1.97(2H,m),1.33(6H,d,J=7.3Hz),0.85(3H,d,J=6.3Hz).
MSm/z(M+H):473.

<Example 0608>

<0608-1>

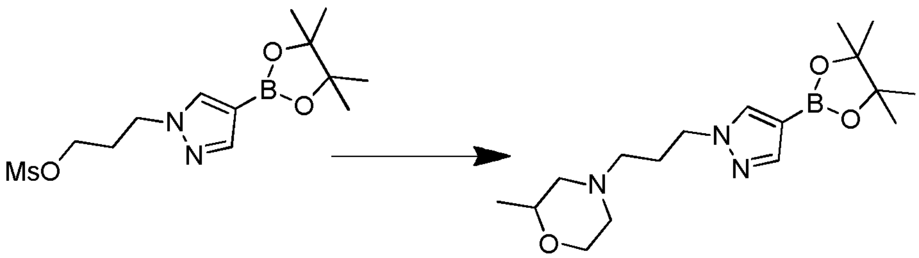

2-Methyl-4-(3-(4-(4,4,5,5-tetramethyl-1,3,2-dioxaborolan-2-yl)-1H-pyrazol-1-yl)propyl)morpholine was obtained in the same manner as in Example 0607-1 except that 2-methylmorpholine was used instead of the 3-methylmorpholine used in Example 0607-1.
MSm/z(M+H):336.

<0608-2>

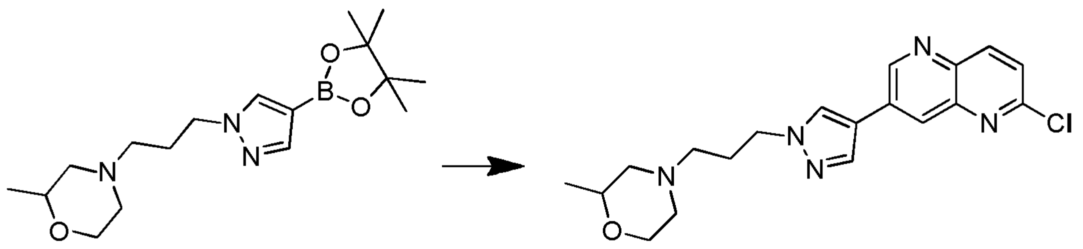

4-(3-(4-(6-Chloro-1,5-naphthyridin-3-yl)-1H-pyrazol-1-yl)propyl)-2-methylmorpholine was obtained in the same manner as in Example 607-2 except that 2-methyl-4-(3-(4-(4,4,5,5-tetramethyl-1,3,2-dioxaborolan-2-yl)-1H-pyrazol-1-yl)propyl)morpholine was used instead of the 3-methyl-4-(3-(4-(4,4,5,5-tetramethyl-1,3,2-dioxaborolan-2-yl)-1H-pyrazol-1-yl)propyl)morpholine used in Example 607-2.
MSm/z(M+H):372.

<0608-3>

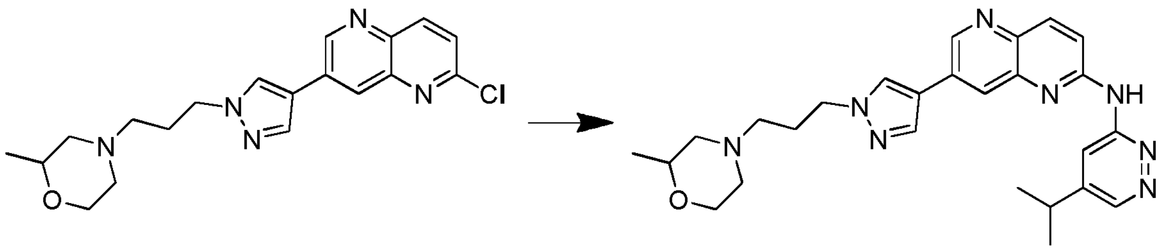

N-(5-isopropylpyridazin-3-yl)-7-(1-(3-(2-methylmorpholino)propyl)-1H-pyrazol-4-yl)-1,5-naphthyridine-2-amine was obtained as a yellow solid in the same manner as in Example 0411-3 except that 4-(3-(4-(6-chloro-1,5-naphthyridin-3-yl)-1H-pyrazol-1-yl)propyl)-2-methylmorpholine was used instead of the 2-chloro-7-(1-((6-methylpyridin-2-yl)methyl)-1H-pyrazol-4-yl)-1,5-naphthyridine used in Example 0411-3.
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.51(1H,s),8.23-8.19(3H,m),7.70(1H,d,J=9.2Hz),4.21(2H,t,J=6.9Hz),3.73(1H,d,J=9.9Hz),3.54-3.45(2H,m),3.08-2.99(1H,m),2.76-2.63(2H,m),2.28(2H,t,J=6.9Hz),2.05-1.89(3H,m),1.64(1H,t,J=10.2Hz),1.33(6H,d,J=7.3Hz),1.03(3H,d,J=5.9Hz).
MSm/z(M+H):473.

<Examples 0609 and 0610>

<0609-1 and 0610-1>

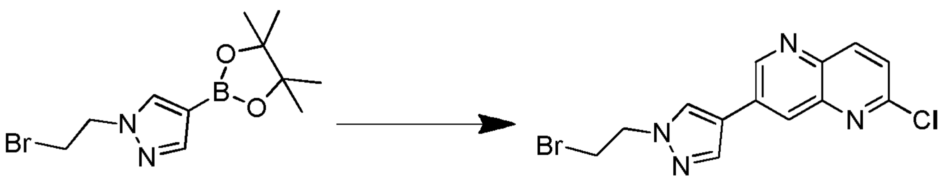

7-(1-(2-Bromoethyl)-1H-pyrazol-4-yl)-2-chloro-1,5-naphthyridine was obtained in the same manner as in Example 0607-2 except that 1-(2-bromoethyl)-4-(4,4,5,5-tetramethyl-1,3,2-dioxaborolan-2-yl)-1H-pyrazole was used instead of the 3-methyl-4-(3-(4-(4,4,5,5-tetramethyl-1,3,2-dioxaborolan-2-yl)-1H-pyrazol-1-yl)propyl)morpholine used in Example 0607-2.
MSm/z(M+H):339.

<0609-2 and 0610-2>

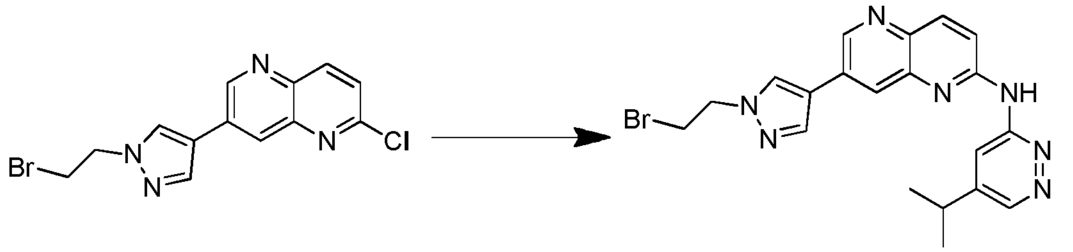

7-(1-(2-Bromoethyl)-1H-pyrazol-4-yl)-N-(5-isopropylpyridazin-3-yl)-1,5-naphthyridine-2-amine was obtained in the same manner as in Example 0411-3 except that 7-(1-(2-bromoethyl)-1H-pyrazol-4-yl)-2-chloro-1,5-naphthyridine was used instead of the 2-chloro-7-(1-((6-methylpyridin-2-yl)methyl)-1H-pyrazol-4-yl)-1,5-naphthyridine used in Example 0411-3.
MSm/z(M+H):438.

<0609-3 and 0610-3>

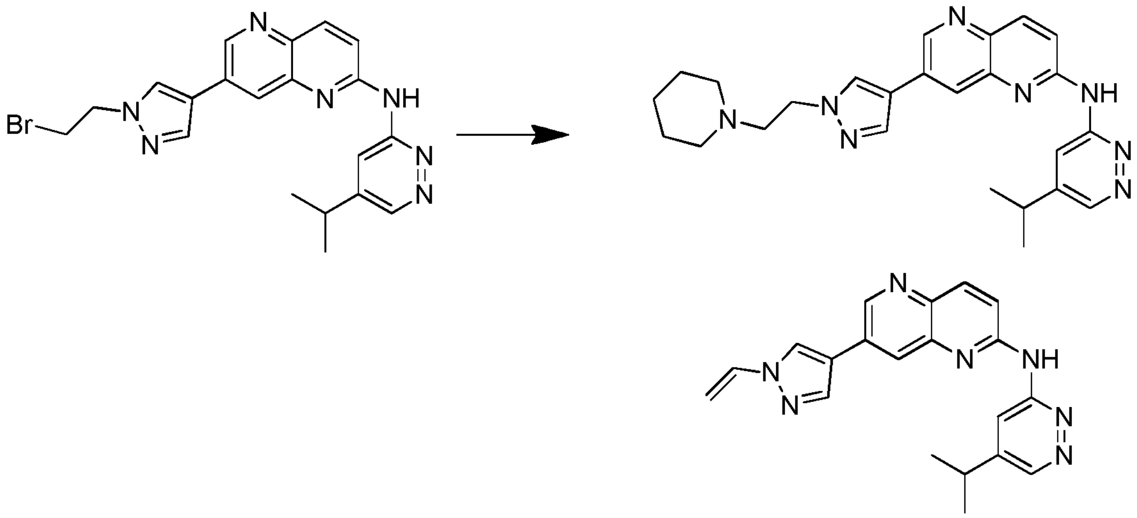

Amixture of 7-(1-(2-bromoethyl)-1H-pyrazol-4-yl)-N-(5-isopropylpyridazin-3-yl)-1,5-naphthyridine-2-amine (20 mg), 1,4-dioxane (5 mL), piperidine (10 μL), and cesium carbonate (33 mg) was stirred at 80°C for 2.5 hours. Sodium iodide (2.2 mg) was added to the reaction mixture, followed by stirring at 100°C for 3 hours. Piperidine (10 μL) was added to the reaction mixture, followed by stirring at 100°C for 1 hour. The reaction mixture was allowed to stand overnight, and piperidine (10 μL) was added thereto, followed by stirring at 120°C for 30 minutes using a microwave reaction apparatus. The reaction mixture was purified by silica gel column chromatography (hexane-ethyl acetate-methanol, NH silica), thereby obtaining N-(5-isopropylpyridazin-3-yl)-7-(1-(2-(piperidin-1-yl)ethyl)-1H-pyrazol-4-yl)-1,5-naphthyridine-2-amine (2 mg) as a pale yellow solid, and N-(5-isopropylpyridazin-3-yl)-7-(1-vinyl-1H-pyrazol-4-yl)-1,5-naphthyridine-2-amine (2 mg) as a pale yellow solid.

<Example 0609>

N-(5-isopropylpyridazin-3-yl)-7-(1-(2-(piperidin-1-yl)ethyl)-1H-pyrazol-4-yl)-1,5-naphthyridine-2-amine
$^1$H-NMR(DMSO-$d_6$)δ:10.71(1H,s),9.03(1H,d,J=2.0Hz),8.86(1H,d,J=2.0Hz),8.75(1H,d,J=2.0Hz),8.50(1H,s),8.23-8.17(3H,m),7.69(1H,d,J=9.2Hz),4.27(2H,t,J=6.6Hz),3.08-2.98(1H,m),2.72(2H,t,J=6.6Hz),2A1(4H,t,J=5.3Hz),1A9(4H,t,J=5.3Hz),1.43-1.36(2

H,m), 1.33(6H,d,J=7.3Hz).
MSm/z(M+H):443.

<Example 0610>

N-(5-isopropylpyridazin-3-yl)-7-(1-vinyl-1H-pyrazol-4-yl)-1,5-naphthyridine-2-amine
$^1$H-NMR(DMSO-$d_6$)$\delta$:10.72(1H,s),9.10(1H,d,J=2.0Hz),8.88(1H,s),8.88(1H,d,J=2.0Hz),8.73(1H,d,J=1.3Hz),8.42(1H,s),8.31(1H,d,J=1.3Hz),8.24(1H,d,J=9.2Hz),7.73(1H,d,J=9.2Hz),7.30(1H,dd,J=15.3,8.7Hz),5.69(1H,d,J=15.3Hz),4.97(1H,d,J=8.7Hz),3.09-2.99(1H,m),1.34(6H,d,J=6.6Hz).
MSm/z(M+H):358.

<Example 0611>

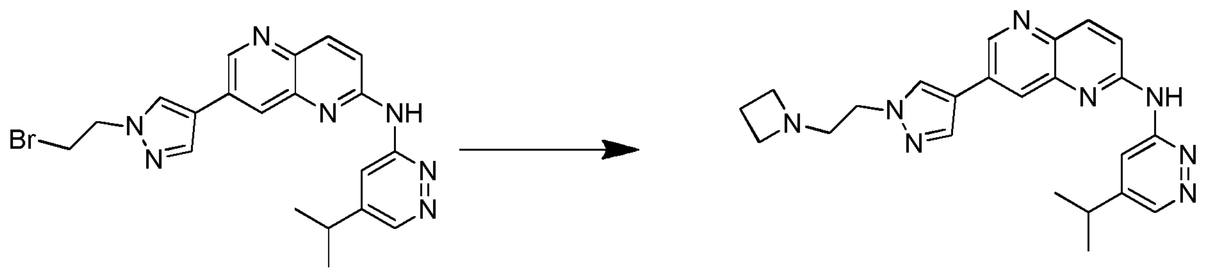

7-(1-(2-(Azetidin-1-yl)ethyl)-1H-pyrazol-4-yl)-N-(5-isopropylpyridazin-3-yl)-1,5-naphthyridine-2-amine was obtained as a pale yellow solid in the same manner as in Example 0609-3 except that azetidine was used instead of the piperidine used in Example 0609-3.
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.49(1H,s),8.24-8.19(2H,m),8.18(1H,s),7.70(1H,d,J=9.2Hz),4.10(2H,t,J=6.3Hz),3.12-2.99(5H,m),2.79(2H,t,J=6.3Hz),1.98-1.88(2H,m),1.34(6H,d,J=6.6Hz).
MSm/z(M+H):415.

<Example 0612>

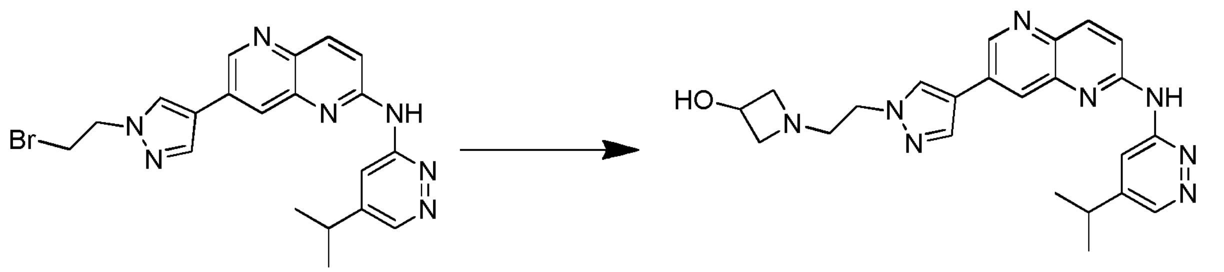

1-(2-(4-(6-((5-Isopropylpyridazin-3-yl)amino)-1,5-naphthyridin-3-yl)-1H-pyrazol-1-yl)ethyl)azetidin-3-ol was obtained as a pale yellow solid in the same manner as in Example 0609-3 except that 3-hydroxyazetidine hydrochloride was used instead of the piperidine used in Example 0609-3.
$^1$H-NMR(DMSO-$d_6$)$\delta$:10.70(1H,s),9.04(1H,d,J=2.0Hz),8.87(1H,d,J=2.0Hz),8.73(1H,d,J=2.0Hz),8.49(1H,s),8.23-8.17(3H,m),7.70(1H,d,J=9.2Hz),5.27(1H,brs),4.12(2H,t,J=6.1Hz),3.51-3.45(2H,m),3.39-3.26(1H,m),3.09-2.99(1H,m),2.83(2H,t,J=6.1Hz),2.76-2.72(2H,m),1.34(6H,d,J=6.6Hz).
MSm/z(M+H):431.

<Example 0613>

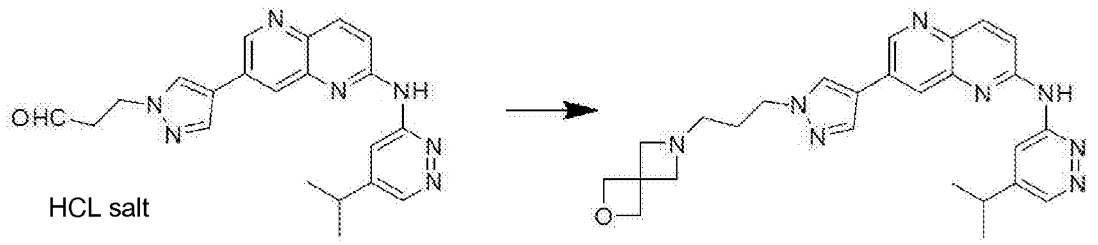

7-(1-(3-(2-Oxa-6-azaspiro[3.3]heptan-6-yl)propyl)-1H-pyrazol-4-yl)-N-(5-isopropylpyridazin-3-yl)-1,5-naphthyridine-2-amine was obtained as a pale yellow solid in the same manner as in Example 0426-2 except that 2-oxa-6-azaspiro[3.3]heptane oxalate was used instead of the (2S,6R)-1,2,6-trimethylpiperazine trifluoroacetate used in Example 0426-2.
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.49(1H,s),8.23-8.17(3H,m),7.70(1H,d,J=9.2Hz),4.59(4H,s),4.16(2H,t,J=6.9Hz),3.24(4H,s),3.09-2.99(1H,m),2.30(2H,t,J=6.9Hz),1.85-1.75(2H,m),1.34(6H,d,J=6.6Hz).
MSm/z(M+H):471.

<Examples 0614 and 0615>

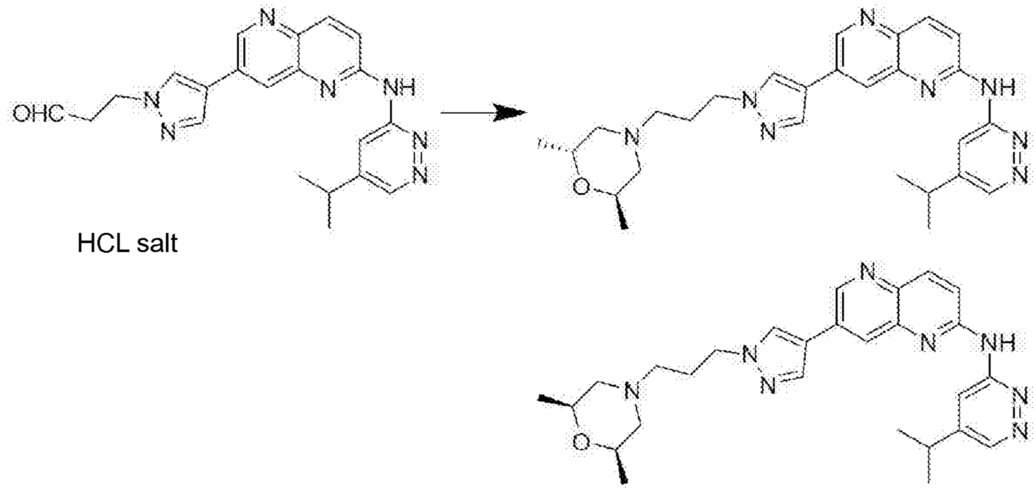

7-(1-(3-((2R,6R)-2,6-dimethylmorpholino)propyl)-1H-pyrazol-4-yl)-N-(5-isopropylpyridazin-3-yl)-1,5-naphthyridine-2-amine as a pale yellow solid and 7-(1-(3-((2S,6R)-2,6-dimethylmorpholino)propyl)-1H-pyrazol-4-yl)-N-(5-isopropylpyridazin-3-yl)-1,5-naphthyridine-2-amine as a pale yellow solid were obtained in the same manner as in Example 0426-2 except that 2,6-dimethylmorpholine was used instead of the (2S,6R)-1,2,6-trimethylpiperazine trifluoroacetate used in Example 0426-2.

<Example 0614>

7-(1-(3-((2R,6R)-2,6-dimethylmorpholino)propyl)-1H-pyrazol-4-yl)-N-(5-isopropylpyridazin-3-yl)-1,5-naphthyridine-2-amine
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.49(1H,s),8.23-8.19(3H,m),7.70(1H,d,J=9.2Hz),4.22(2H,t,J=6.9Hz),3.95-3.86(2H,m),3.09-2.98(1H,m),2.40-2.35(2H,m),2.21(2H,t,J=5.9Hz),2.09-1.97(4H,m),1.33(6H,d,J=7.2Hz),1.14(6H,d,J=6.6Hz).

MSm/z(M+H):487.

<Example 0615>

7-(1-(3-((2S,6R)-2,6-dimethylmorpholino)propyl)-1H-pyrazol-4-yl)-N-(5-isopropylpyridazin-3-yl)-1,5-naphthyridine-2-amine
$^1$H-NMR(DMSO-$d_6$)$\delta$:10.70(1H,s),9.04(1H,d,J=2.0Hz),8.87(1H,d,J=2.0Hz),8.73(1H,d,J=2.0Hz),8.51(1H,s),8.23-8.18(3H,m),7.70(1H,d,J=9.2Hz),4.20(2H,t,J=6.9Hz),3.60-3.51(2H,m),3.08-2.99(1H,m),2.72(2H,d,J=9.9Hz),2.27(2H,t,J=6.9Hz),2.05-1.97(2H,m),1.55(2H,t,J=10.6Hz),1.33(6H,d,J=7.3Hz),1.03(6H,d,J=6.6Hz).
MSm/z(M+H):487.

<Examples 0616 and 0617>

<0616-1 and 0617-1>

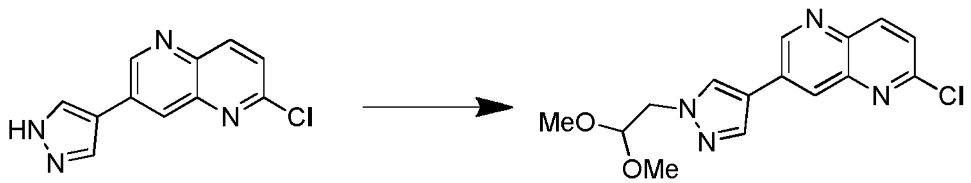

2-Chloro-7-(1-(2,2-dimethoxyethyl)-1H-pyrazol-4-yl)-1,5-naphthyridine was obtained in the same manner as in Example 0646-2 except that 2-bromo-1,1-dimethoxyethane was used instead of the 3-bromo-1,1-dimethoxypropane used in Example 0646-2.
MSm/z(M+H):319.

<0616-2 and 0617-2>

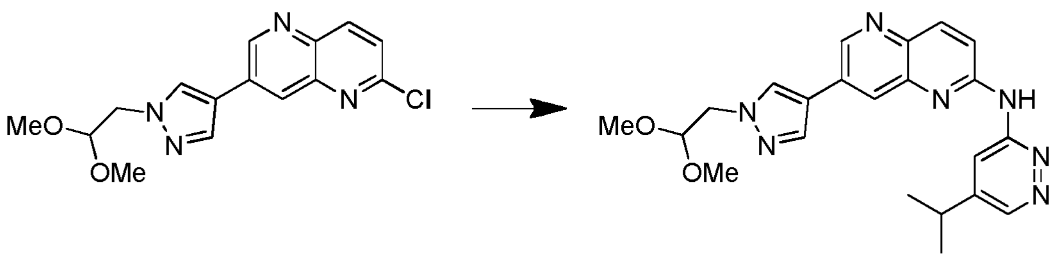

7-(1-(2,2-Dimethoxyethyl)-1H-pyrazol-4-yl)-N-(5-isopropylpyridazin-3-yl)-1,5-naphthyridine-2-amine was obtained in the same manner as in Example 0646-3 except that 2-chloro-7-(1-(2,2-dimethoxyethyl)-1H-pyrazol-4-yl)-1,5-naphthyridine was used instead of the 2-chloro-7-(1-(2,2-dimethoxypropyl)-1H-pyrazol-4-yl)-1,5-naphthyridine used in Example 0646-3.
MSm/z(M+H):420.

<0616-3 and 0617-3>

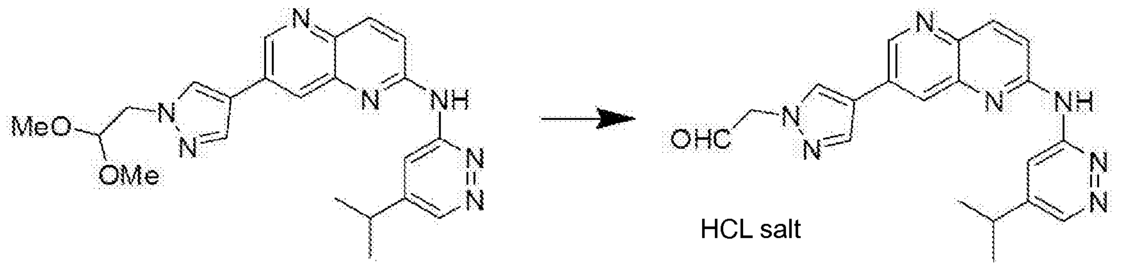


2-(4-(6-((5-Isopropylpyridazin-3-yl)amino)-1,5-naphthyridin-3-yl)-1H-pyrazol-1-yl)acetaldehyde hydrochloride was obtained in the same manner as in Example 0426-1 except that 7-(1-(2,2-dimethoxyethyl)-1H-pyrazol-4-yl)-N-(5-isopropylpyridazin-3-yl)-1,5-naphthyridine-2-amine was used instead of the 7-(1-(3,3-dimethoxypropyl)-1H-pyrazol-4-yl)-N-(5-isopropylpyridazin-3-yl)-1,5-naphthyridine-2-amine used in Example 0426-1.
$^1$H-NMR(DMSO-$d_6$)δ:10.73(1H,d,J=15.9Hz),9.30(1H,dd,J=14.9,2.3Hz),8.88(1H,dd,J=8.6,2.0Hz),8.77-8.75(2H,m),8.62-8.20(4H,m),7.76-7.70(1H,m),6.54(1H,s),4.18-4.15(2H,m),3.09-3.06(1H,m), 1.35(6H,d,J=2.0Hz).

<0616-4 and 0617-4>

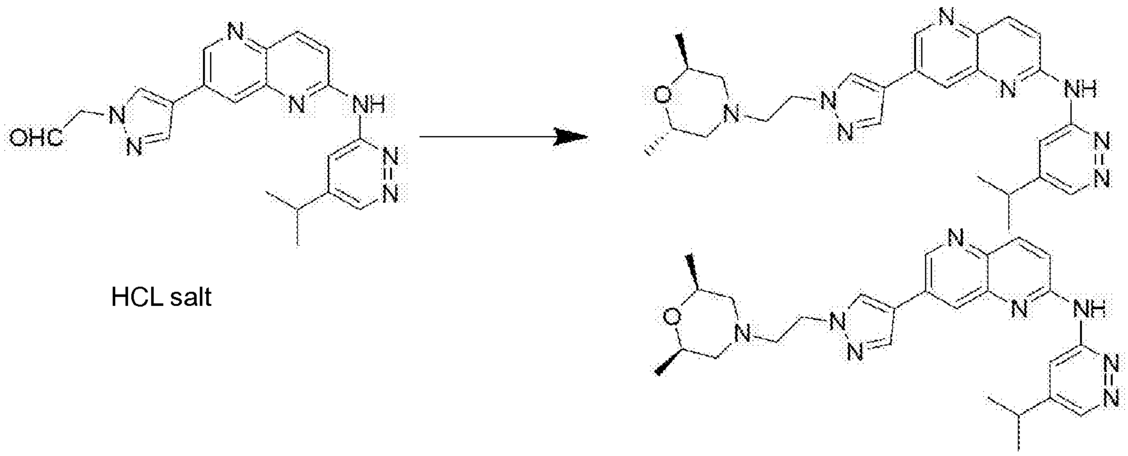


7-(1-(2-((2S,6S)-2,6-dimethylmorpholino)ethyl)-1H-pyrazol-4-yl)-N-(5-isopropylpyridazin-3-yl)-1,5-naphthyridine-2-amine as a pale yellow solid and 7-(1-(2-((2S,6R)-2,6-dimethylmorpholino)ethyl)-1H-pyrazol-4-yl)-N-(5-isopropylpyridazin-3-yl)-1,5-naphthyridine-2-amine as a pale yellow solid were obtained in the same manner as in Example 0614-1 except that 2-(4-(6-((5-isopropylpyridazin-3-yl)amino)-1,5-naphthyridin-3-yl)-1H-pyrazol-1-yl)acetaldehyde hydrochloride was used instead of the 3-(4-(6-((5-isopropylpyridazin-3-yl)amino)-1,5-naphthyridin-3-yl)-1H-pyrazol-1-yl)propanal hydrochloride used in Examples 0614-1 and 0615-1.

<Example 0616>

7-(1-(2-((2S,6S)-2,6-dimethylmorpholino)ethyl)-1H-pyrazol-4-yl)-N-(5-isopropylpyridazin-3-yl)-1,5-naphthyridine-2-amine
$^1$H-NMR($CDCl_3$)δ:9.51(1H,s),8.93(2H,d,J=2.0Hz),8.83(1H,s),8.25(1H,d,J=8.9Hz),8.10(1H,d,J=2.0Hz),7.98(1H,s), 7.96(1H,s), 7.67(1H,d,J=8.9Hz),4.32(2H,t,J=5.9Hz),4.07-3.98(2H,m),3.11-3.02(1H,m),2.88-2.71(2H,m),2.58-2.52(2H,m),2.25-2.17(2H,m), 1.43(6H,d,J=6.6Hz), 1.24(6H,d,J=6.6Hz).
MSm/z(M+H):473.

<Example 0617>

7-(1-(2-((2S,6R)-2,6-dimethylmorpholino)ethyl)-1H-pyrazol-4-yl)-N-(5-isopropylpyridazin-3-yl)-1,5-naphthyrid-

ine-2-amine
$^1$H-NMR($CDCl_3$)δ:9.78(1H,s),8.95(1H,s),8.92(1H,d,J=2.0Hz),8.83(1H,s),8.25(1H,d,J=9.2Hz),8.10(1H,d,J =2.0Hz), 7.96(2H,s), 7.73(1H,d,J=9.2Hz),4.34(2H,t,J=6.3Hz),3.73-3.64(2H,m),3.12-3.02(1H,m),2.86(2H,t,J=6.3Hz),2.74(2H,d,J=10.6Hz),1.89(2H,t,J=10.6Hz),1.43(6H,d,J=6.6Hz),1.17(6 H,d,J=6.6Hz).
MSm/z(M+H):473.

<Example 0618>

<0618-1>

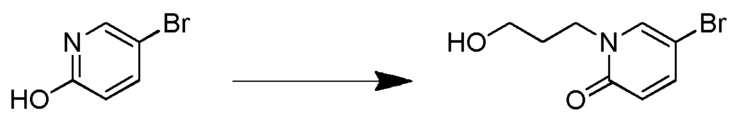

3-Bromopropan-l-ol (151 μL) and potassium carbonate (477 mg) were added to a solution of 5-bromopyridin-2-ol (200 mg) in methanol (5 mL), followed by stirring for 1.5 hours under reflux. Sodium iodide (17 mg) and 3-bromo-propan-1-ol (100 μL) were added to the reaction mixture, followed by stirring for 11 hours under reflux. The reaction mixture was cooled to room temperature, and the solvent was distilled off under reduced pressure. The obtained residue was purified by silica gel column chromatography (hexane-ethyl acetate-methanol), thereby obtaining 5-bromo-1-(3-hydroxypropyl)pyridin-2(1H)-one (250 mg).
MSm/z(M+H):232.

<0618-2>

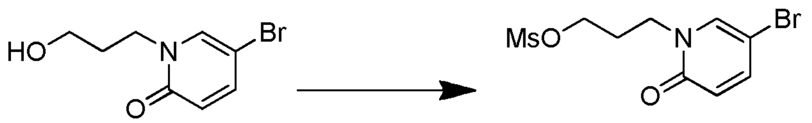

Methanesulfonyl chloride (91 μL) was added to a mixture of 5-bromo-1-(3-hydroxypropyl)pyridin-2(1H)-one (180 mg), dichloromethane (5 mL), and triethylamine (324 μL) at a temperature of from 0°C to 5°C, followed by stirring for 1.5 hours. After water was added to the reaction mixture, the organic layer was collected by separation, washed with a saturated sodium chloride aqueous solution, and dried over anhydrous sodium sulfate, and the solvent was distilled off under reduced pressure, thereby obtaining 3-(5-bromo-2-oxopyridine-1(2H)-yl)propyl methanesulfonate (259 mg).
MSm/z(M+H):312.

<0618-3>

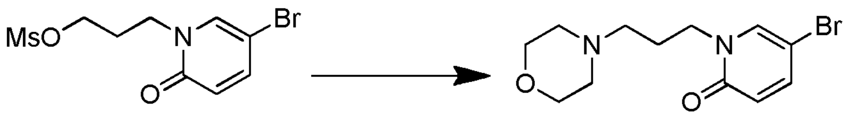

A mixture of 3-(5-bromo-2-oxopyridin-1(2H)-yl)propyl methanesulfonate (259 mg), 1,4-dioxane (5 mL), morpholine (295 μL), cesium carbonate (547 mg), and sodium iodide (37 mg) was stirred at 80°C for 3 hours. Ethyl acetate was added to the reaction mixture, the insolubles were filtered off, and the solvent was distilled off under reduced pressure, thereby obtaining 5-bromo-1-(3-morpholinopropyl)pyridin-2(1H)-one (258 mg).
MSm/z(M+H):303.

<0618-4>

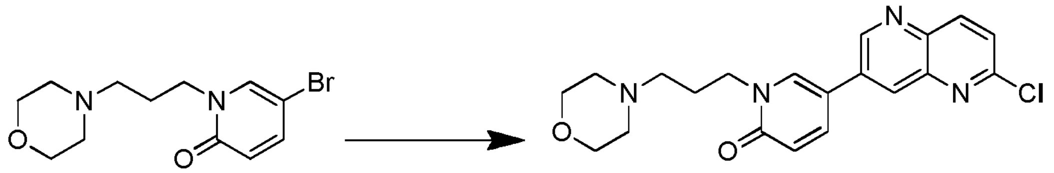

5-(6-Chloro-1,5-naphthyridin-3-yl)-1-(3-morpholinopropyl)pyridin-2(1H)-one was obtained in the same manner as in Example 0421-1 except that, 5-bromo-1-(3-morpholinopropyl)pyridin-2(1H)-one was used instead of the N-(4-bromopyridin-2-yl)acetamide used in Example 0421-1.
MSm/z(M+H):385.

<0618-5>

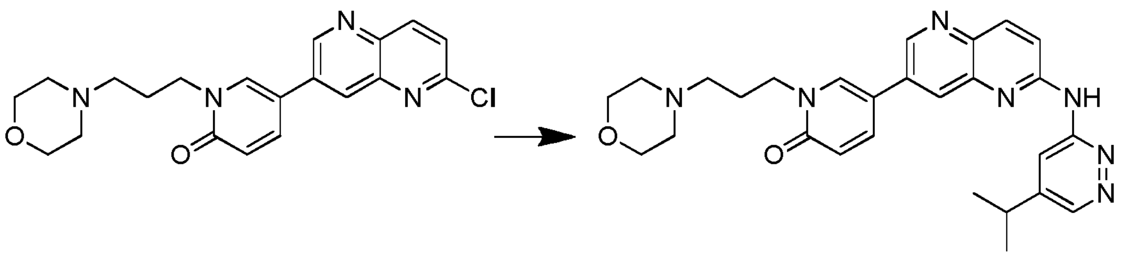

5-(6-((5-isopropylpyridazin-3-yl)amino)-1,5-naphthyridin-3-yl)-1-(3-morpholinopropyl)pyridin-2(1H)-one was obtained as a pale yellow solid in the same manner as in Example 0411-3 except that 5-(6-chloro-1,5-naphthyridin-3-yl)-1-(3-morpholinopropyl)pyridin-2(1H)-one was used instead of the 2-chloro-7-(1-((6-methylpyridin-2-yl)methyl)-1H-pyrazol-4-yl)-1,5-naphthyridine used in Example 0411-3.
$^1$H-NMR(DMSO-$d_6$)$\delta$:10.75(1H,s),9.04(1H,d,J=2.0Hz),8.87(1H,d,J=2.0Hz),8.75(1H,d,J=2.0Hz),8.46(1H,d,J=2.0Hz),8.28-8.24(2H,m),8.07(1H,dd,J=9.2,2.0Hz),7.73(1H,d,J=9.2Hz),6.56(1H,d,J=9.2Hz),4.05(2H,t,J=6.9Hz),3.54(4H,t,J=4.3Hz),3.08-2.99(1H,m),2.36-2.27(6H,m),1.90(2H,t,J=6.9Hz),1.32(6H,d,J=7.3Hz).
MSm/z(M+H):486.

<Example 0619>

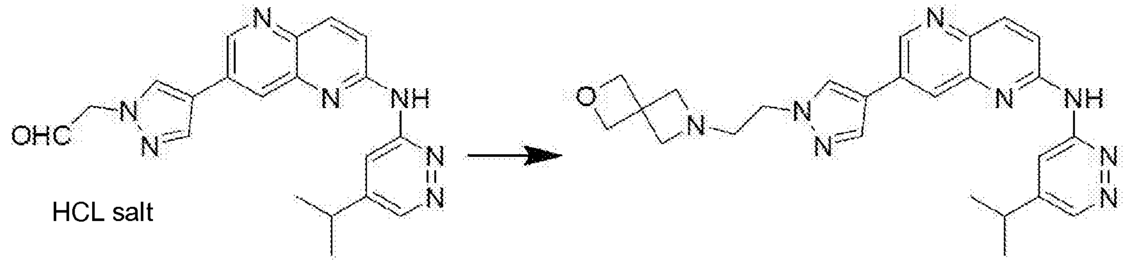

7-(1-(2-(2-Oxa-6-azaspiro[3.3]heptan-6-yl)ethyl)-1H-pyrazol-4-yl)-N-(5-isopropylpyridazin-3-yl)-1,5-naphthyridine-2-amine was obtained as a pale yellow solid in the same manner as in Example 0613-1 except that 2-(4-(6-((5-isopropylpyridazin-3-yl)amino)-1,5-naphthyridin-3-yl)-1H-pyrazol-1-yl)acetaldehyde hydrochloride was used instead of the 3-(4-(6-((5-isopropylpyridazin-3-yl)amino)-1,5-naphthyridin-3-yl)-1H-pyrazol-1-yl)propanal hydrochloride used in Ex-

ample 0613-1.
$^1$H-NMR($CDCl_3$)δ:9.23(1H,s),8.93(1H,d,J=2.0Hz),8.86-8.76(2H,m),8.25(1H,d,J=8.9Hz),8.11(1H,d,J=2.0Hz),7.98(1H,s),7.89(1H,s),7.52(1H,d,J=8.9Hz),4.71( 4H,s),4.23-4.16(2H,m),3.36(4H,s),3.11-3.03(1H,m),2.96-2.90(2H,m),1.43(6H,d,J=7.3Hz).
MSm/z(M+H):457.

<Example 0620>

<0620-1>

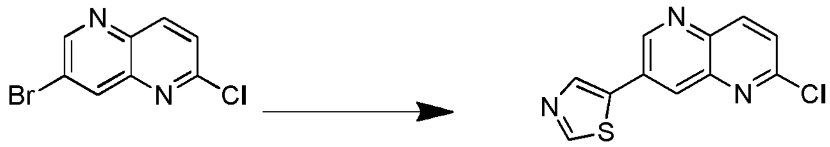

5-(6-Chloro-1,5-naphthyridin-3-yl)thiazole was obtained in the same manner as in Example 0421-1 except that 5-bromothiazole was used instead of the N-(4-bromopyridin-2-yl)acetamide used in Example 0421-1.
MSm/z(M+H):248.

<0620-2>

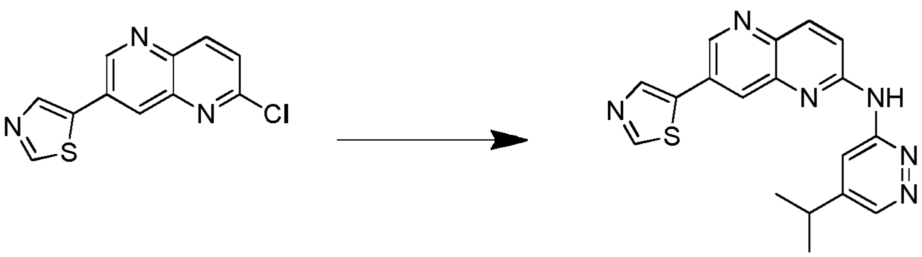

N-(5-isopropylpyridazin-3-yl)-7-(thiazol-5-yl)-1,5-naphthyridine-2-amine was obtained as a pale yellow solid in the same manner as in Example 0411-3 except that 5-(6-chloro-1,5-naphthyridin-3-yl)thiazole was used instead of the 2-chloro-7-(1-((6-methylpyridin-2-yl)methyl)-1H-pyrazol-4-yl)-1,5-naphthyridine used in Example 0411-3.
$^1$H-NMR(DMSO-$d_6$)δ:10.82(1H,s),9.25(1H,s),9.12(1H,d,J=2.6Hz),8.88(1H,d,J=2.0Hz),8.75(1H,d,J=2.0Hz),8.67(1H,s),8.34(1H,d,J=2.6Hz),8.28(1H,d,J=9.2Hz),7.78(1H,d,J=9.2Hz),3.11-3.01(1H,m),1.33(6H,d,J=7.3Hz).
MSm/z(M+H):349.

<Example 0621>

<0621-1>

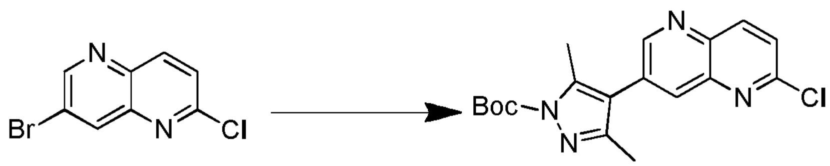

tert-Butyl 4-(6-chloro-1,5-naphthyridin-3-yl)-3,5-dimethyl-1H-pyrazole-1-carboxylate was obtained in the same

manner as in Example 607-2 except that tert-butyl 3,5-dimethyl-4-(4,4,5,5-tetramethyl-1,3,2-dioxaborolan-2-yl)-1H-pyrazole-1-carboxylate was used instead of the 3-methyl-4-(3-(4-(4,4,5,5-tetramethyl-1,3,2-dioxaborolan-2-yl)-1H-pyrazol-1-yl)propyl)morpholine used in Example 607-2.
MSm/z(M+H):359.

<0621-2>

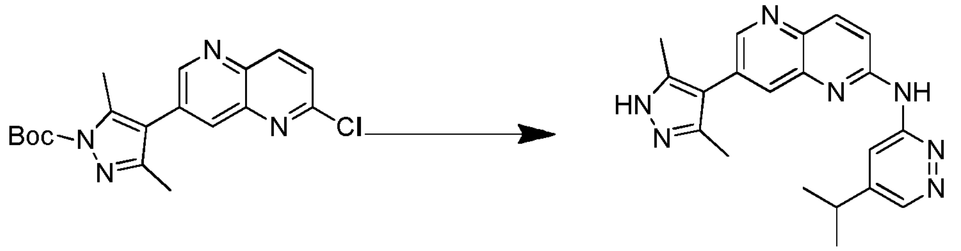

7-(3,5-Dimethyl-1H-pyrazol-4-yl)-N-(5-isopropylpyridazin-3-yl)-1,5-naphthyridine-2-amine was obtained as a yellow solid in the same manner as in Example 0411-3 except that ttert-butyl 4-(6-chloro-1,5-naphthyridin-3-yl)-3,5-dimethyl-1H-pyrazole-1-carboxylate was used instead of the 2-chloro-7-(1-((6-methylpyridin-2-yl)methyl)-1H-pyrazol-4-yl)-1,5-naphthyridine used in Example 0411-3.
$^1$H-NMR(DMSO-$d_6$)δ:12.54(1H,s),10.73(1H,s),8.84(1H,d,J=2.0Hz),8.73(1H,d,J=2.0Hz),8.70(1H,d,J=2.0Hz),8.26(1H,d,J=9.2Hz),7.95(1H,d,J=2.0Hz),7.78(1H,d,J=9.2Hz),3.03-2.97(1H,m),2.33(3H,s),2.27(3H,s),1.30(6H,d,J=7.3Hz).
MSm/z(M+H):360.

<Example 0622>

<0622-1>

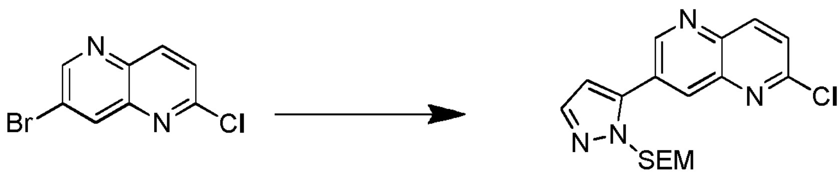

2-Chloro-7-(1-((2-(trimethylsilyl)ethoxy)methyl)-1H-pyrazol-5-yl)-1,5-naphthyridine was obtained in the same manner as in Example 0607-2 except that 5-(4,4,5,5-tetramethyl-1,3,2-dioxaborolan-2-yl)-1-((2-(trimethylsilyl)ethoxy)methyl)-1H-pyrazole was used instead of the 3-methyl-4-(3-(4-(4,4,5,5-tetramethyl-1,3,2-dioxaborolan-2-yl)-1H-pyrazol-1-yl)propylmorpholine used in Example 0607-2.
MSm/z(M+H):361.

<0622-2>

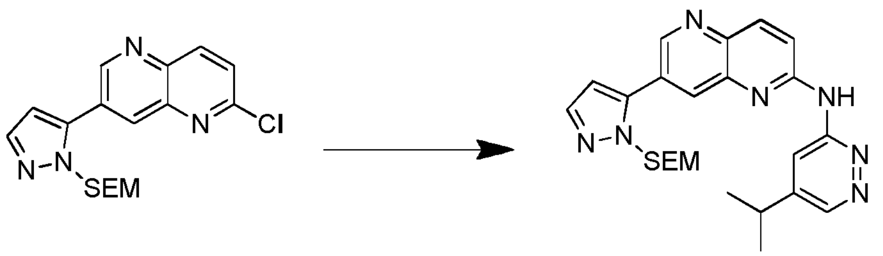

N-(5-isopropylpyridazin-3-yl)-7-(1-((2-(trimethylsilyl)ethoxy)methyl)-1H-pyrazol-5-yl)-1,5-naphthyridine-2-amine was obtained in the same manner as in Example 0411-3 except that 2-chloro-7-(1-((2-(trimethylsilyl)ethoxy)methyl)-1H-pyrazol-5-yl)-1,5-naphthyridine was used instead of the 2-chloro-7-(1-((6-methylpyridin-2-yl)methyl)-1H-pyrazol-4-yl)-1,5-naphthyridine used in Example 0411-3.
MSm/z(M+H):462.

<0622-3>

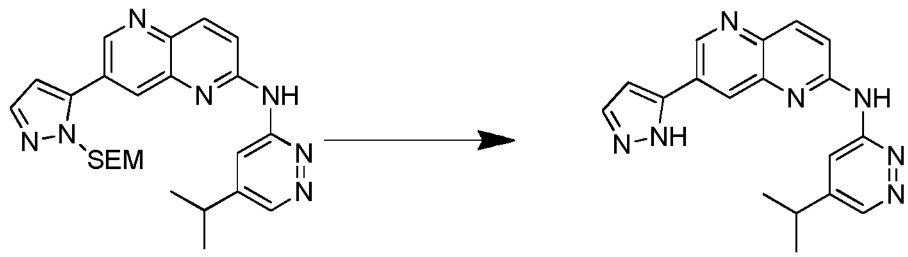

A mixture ofN-(5-isopropylpyridazin-3-yl)-7-(1-((2-(trimethylsilyl)ethoxy)methyl)-1H-pyrazol-5-yl)-1,5-naphthyridine-2-amine (75 mg), 1,4-dioxane (2 mL), and 2 mol/L hydrochloric acid (1 mL) was stirred at 80°C for 3.5 hours. After the reaction mixture was cooled to room temperature, a saturated sodium hydrogen carbonate aqueous solution and ethyl acetate were added thereto, and the organic layer was collected by separation, washed with a saturated sodium chloride aqueous solution, and dried over anhydrous sodium sulfate, and the solvent was distilled off under reduced pressure. The obtained residue was purified by silica gel column chromatography (chloroform-methanol, NH silica), thereby obtaining N-(5-isopropylpyridazin-3-yl)-7-(1H-pyrazol-5-yl)-1,5-naphthyridine-2-amine (21 mg)) as a yellow solid.
$^1$H-NMR(DMSO-$d_6$)$\delta$:13.19(1H,s),10.74(1H,s),9.25(1H,d,J=2.0Hz),8.87(1H,d,J=2.0Hz),8.77(1H,d,J=2.0Hz),8.42(1H,s) ,8.26(1H,d,J=9.2Hz),7.92(1H,s),7.74(1H,d,J=9.2Hz),7.05(1H,s),3.09-3.00(1H,m),1.33(6H,d,J=6.6Hz).
MSm/z(M+H):332.

<Example 0623>

<0623-1>

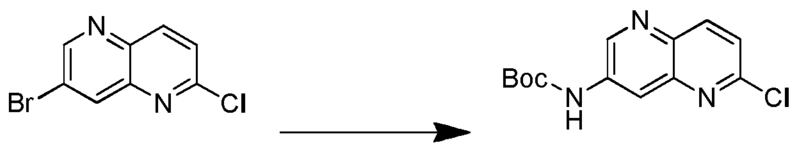

A mixture of 7-bromo-2-chloro-1,5-naphthyridine (50 mg), tert-butyl carbamate (32 mg), tris(dibenzylideneacetone)dipalladium(0) (18 mg), 4,5-bis(diphenylphosphino)-9,9-dimethylxanthene (23 mg), cesium carbonate (187 mg), and 1,4-dioxane (4 mL) was stirred at 120°C for 9 hours in a nitrogen atmosphere. The reaction mixture was cooled to room temperature, and the solvent was distilled off under reduced pressure. The obtained residue was purified by silica

gel column chromatography (hexane-ethyl acetate-methanol), thereby obtaining tert-butyl (6-chloro-1,5-naphthyridin-3-yl)carbamate (47 mg).
MSm/z(M+H):280.

<0623-2>

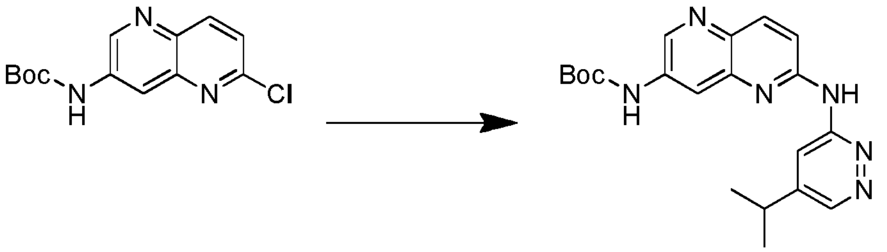

tert-Butyl (6-((5-isopropylpyridazin-3-yl)amino)-1,5-naphthyridin-3-yl)carbamate was obtained in the same manner as in Example 0646-3 except that tert-butyl (6-chloro-1,5-naphthyridin-3-yl)carbamate was used instead of the 2-chloro-7-(1-(2,2-dimethoxypropyl)-1H-pyrazol-4-yl)-1,5-naphthyridine used in Example 0646-3.
MSm/z(M+H):381.

<0623-3>

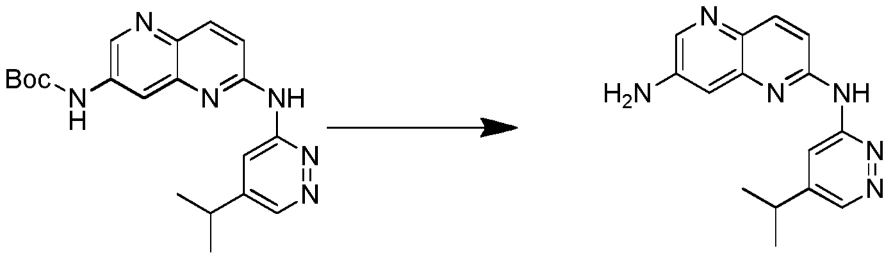

A mixture of tert-butyl (6-((5-isopropylpyridazin-3-yl)amino)-1,5-naphthyridin-3-yl)carbamate (16 mg), 1,4-dioxane (1 mL), and 2 mol/L hydrochloric acid (0.5 mL) was stirred at 80°C for 1 hour. After the reaction mixture was cooled to room temperature, a saturated sodium hydrogen carbonate aqueous solution and ethyl acetate were added thereto, the organic layer was collected by separation, washed with a saturated sodium chloride aqueous solution, and dried over anhydrous sodium sulfate, and the solvent was distilled off under reduced pressure, thereby obtaining $N^2$-(5-isopropylpyridazin-3-yl)-1,5-naphthyridine-2,7-diamine (8 mg) as a yellow solid.
$^1$H-NMR(DMSO-$d_6$)$\delta$:10.44(1H,s),8.79(1H,d,J=2.0Hz),8.74(1H,d,J=2.0Hz),8.24(1H,d,J=2.0Hz),7.97(1H,d,J=9.2Hz),7.34(1H,d,J=9.2Hz),7.02(1H,d,J=2.0Hz),5.91(2H,s),3.02-2.92(1H,m),1.30(6H,d,J=7.6Hz).
MSm/z(M+H):281.

<Example 0624>

<0624-1>

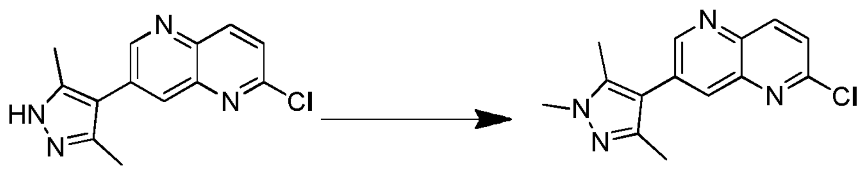

A mixture of 2-chloro-7-(3,5-dimethyl-1H-pyrazol-4-yl)-1,5-naphthyridine (34 mg), 1,4-dioxane (2 mL), potassium carbonate (54 mg), and iodomethane (16 μL) was stirred at room temperature for 1 hour. Iodomethane (16 μL) and N,N-dimethylformamide (1 mL) were added to the reaction mixture, followed by stirring at 80°C for 6 hours. The reaction mixture was cooled to room temperature, and the solvent was distilled off under reduced pressure. The obtained residue was purified by silica gel column chromatography (chloroform-methanol), thereby obtaining 2-chloro-7-(1,3,5-trimethyl-1H-pyrazol-4-yl)-1,5-naphthyridine (20 mg).
MSm/z(M+H):273.

<0624-2>

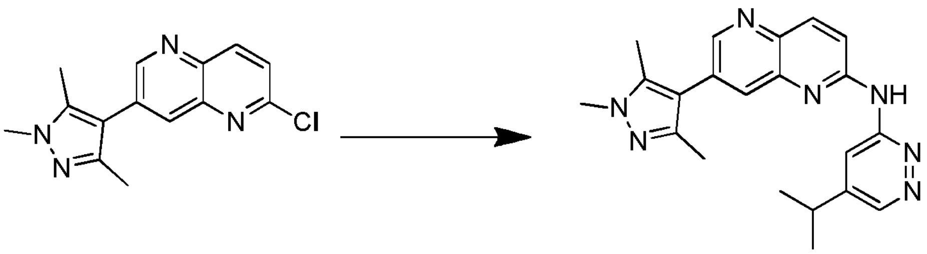

N-(5-isopropylpyridazin-3-yl)-7-(1,3,5-trimethyl-1H-pyrazol-4-yl)-1,5-naphthyridine-2-amine was obtained as a yellow solid in the same manner as in Example 0646-3 except that 2-chloro-7-(1,3,5-trimethyl-1H-pyrazol-4-yl)-1,5-naphthyridine was used instead of the 2-chloro-7-(1-(2,2-dimethoxypropyl)-1H-pyrazol-4-yl)-1,5-naphthyridine used in Example 0646-3.
$^1$H-NMRpMSO-$d_6$)δ:10.74(1H,s),8.85(1H,d,J=2.0Hz),8.70(1H,d,J=2.0Hz),8.68(1H,d,J=2.0Hz),8.27(1H,d,J=9.2Hz),7.92(1H,d,J=2.0Hz),7.78(1H,d,J=9.2Hz),3.76(3H,s),3.02-2.99(1H,m),2.31(3H,s),2.22(3H,s),1.29(6H,d,J=7.3Hz).
MSm/z(M+H):374.

<Example 0625>

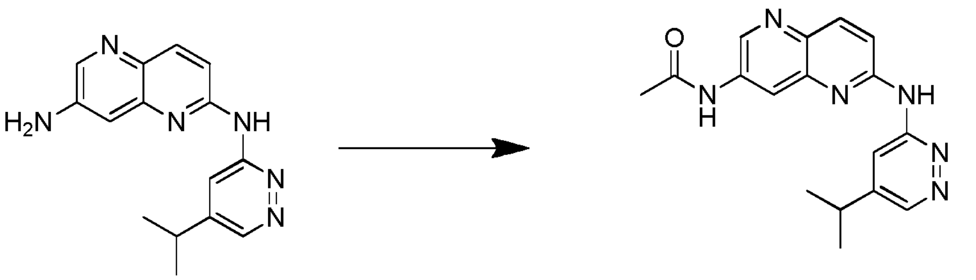

A mixture of $N^2$-(5-isopropylpyridazin-3-yl)-1,5-naphthyridine-2,7-diamine (5 mg), pyridine (1 mL), acetic anhydride (6 μL), and N,N-dimethylpyridine-4-amine (0.2 mg) was stirred at room temperature for 1 hour. The solvent was distilled off under reduced pressure, and the obtained residue was purified by preparative thin layer silica gel column chromatography (chloroform-methanol, NH silica), thereby obtaining N-(6-((5-isopropylpyridazin-3-yl)amino)-1,5-naphthyridin-3-yl)acetamide (6 mg) as a pale yellow solid.
$^1$H-NMRpMSO-$d_6$)δ:10.69(1H,s),10.47(1H,s),8.84(1H,d,J=2.0Hz),8.81(1H,d,J=2.0Hz),8.72(1H,d,J=2.0Hz),8.46(1H,d,J=2.0Hz),8.17(1H,d,J=9.2Hz),7.65(1H,d,J=9.2Hz),3.05-2.95(1H,m),2.15(3H,s),1.31(6H,d,J=6.6Hz).

MSm/z(M+H):323.

<Example 0626>

<0626-1>

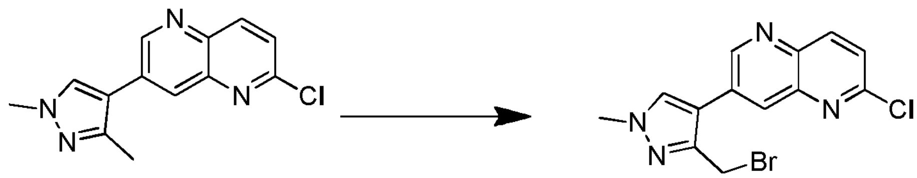

A mixture of 2-chloro-7-(1,3-dimethyl-1H-pyrazol-4-yl)-1,5-naphthyridine (50 mg), chlorobenzene (2 mL), N-bromosuccinimide (37 mg), and azobisisobutyronitrile (3 mg) was stirred at 80°C for 2.5 hours. Azobisisobutyronitrile (19 mg) was added to the reaction mixture, followed by stirring at 80°C for 7 hours. After the reaction mixture was cooled to room temperature, ethyl acetate and a saturated sodium hydrogen carbonate aqueous solution were added thereto, and the organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate-methanol), thereby obtaining 7-(3-(bromomethyl)-1-methyl-1H-pyrazol-4-yl)-2-chloro-1,5-naphthyridine (45 mg).
MSm/z(M+H):339.

<0626-2>

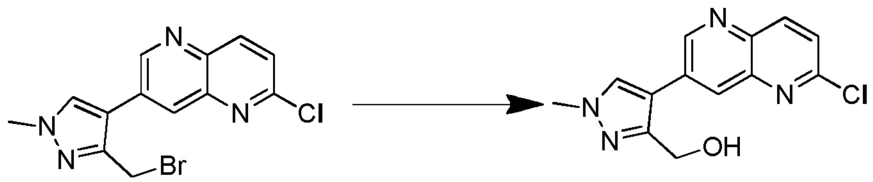

A mixture of 7-(3-(bromomethyl)-1-methyl-1H-pyrazol-4-yl)-2-chloro-1,5-naphthyridine (218 mg), 1,4-dioxane (10 mL), water (5 mL), and potassium carbonate (270 mg) was stirred at 70°C for 1 hour, stirred at 80°C for 2.5 hours, and stirred at 90°C for 30 minutes. After the reaction mixture was cooled to room temperature, ethyl acetate and water were added thereto, the organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate-methanol), thereby obtaining (4-(6-chloro-1,5-naphthyridin-3-yl)-1-methyl-1H-pyrazol-3-yl)methanol (48 mg).
$^{1}$H-NMR(DMSO-$d_6$)δ:9.30(1H,d,J=2.0Hz),8.62(1H,d,J=2.0Hz),8.45(1H,d,J=8.6Hz),8.42(1H,s),7.78-7.75(1H,m),5.44(1H,t,J=5.0Hz),4.59(2H,d,J=4.6Hz),3.89(3H,s).

<0626-3>

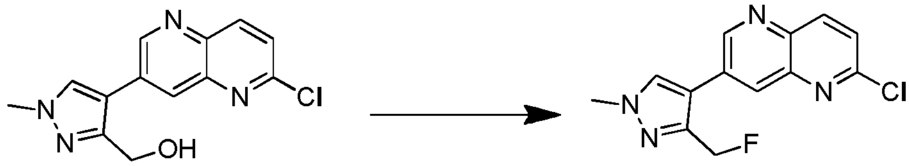

Bis(2-methoxyethyl)aminosulfur trifluoride (37 μL) was added to a mixture of (4-(6-chloro-1,5-naphthyridin-3-yl)-1-methyl-1H-pyrazol-3-yl)methanol (50 mg), and dichloromethane (4 mL) at a temperature of from 0°C to 5°C, followed by stirring at 0°C for 2.5 hours. After ethyl acetate and a saturated sodium hydrogen carbonate aqueous solution were added to the reaction mixture, the organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate-methanol), thereby obtaining 2-chloro-7-(3-(fluoromethyl)-1-methyl-1H-pyrazol-4-yl)-1,5-naphthyridine (44 mg).
MSm/z(M+H):277.

<0626-4>

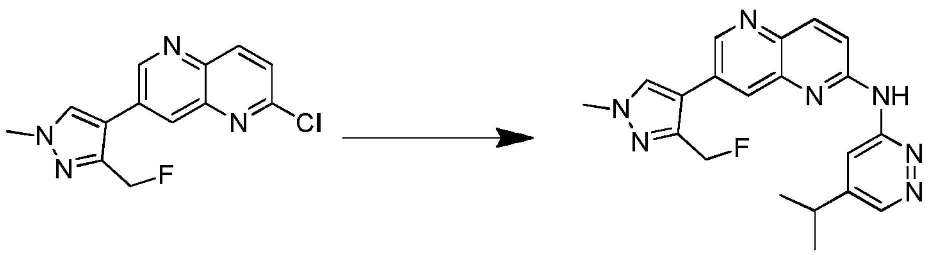

7-(3-(Fluoromethyl)-1-methyl-1H-pyrazol-4-yl)-N-(5-isopropylpyridazin-3-yl)-1,5-naphthyridine-2-amine was obtained as a white solid in the same manner as in Example 0646-3 except that 2-chloro-7-(3-(fluoromethyl)-1-methyl-1H-pyrazol-4-yl)-1,5-naphthyridine was used instead of the 2-chloro-7-(1-(2,2-dimethoxypropyl)-1H-pyrazol-4-yl)-1,5-naphthyridine used in Example 0646-3.
$^1$H-NMRpMSO-d$_6$)δ:10.77(1H,s),8.92(1H,d,J=2.0Hz),8.85(1H,d,J=2.0Hz),8.77(1H,d,J=2.0Hz),8.42(1H,s),8.27(1H,d,J =9.2Hz),8.17(1H,d,J=2.0Hz),7.76(1H,d,J=9.2Hz),5.53(2H,d,J=49.5Hz),3.95(3H,d,J=1.3Hz),3.05-2.95(1H,m),1.31(6H, d,J=7.3Hz).
MSm/z(M+H):378.

<Examples 0627 and 0628>

<0627-1 and 0628-1>

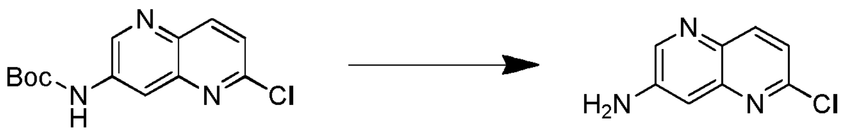

6-Chloro-1,5-naphthyridine-3-amine was obtained in the same manner as in Example 0623-3 except that tert-butyl (6-chloro-1,5-naphthyridin-3-yl)carbamate was used instead of the tert-butyl (6-((5-isopropylpyridazin-3-yl)amino)-1,5-naphthyridin-3-yl)carbamate used in Example 0623-3.
$^1$H-NMR(DMSO-d$_6$)δ:8.51(1H,d,J=2.6Hz),8.15(1H,d,J=8.6Hz),7.33(1H,d,J=8.6Hz),7.08(1H,d,J=2.6Hz),6.31(2H,s).

<0627-2 and 0628-2>

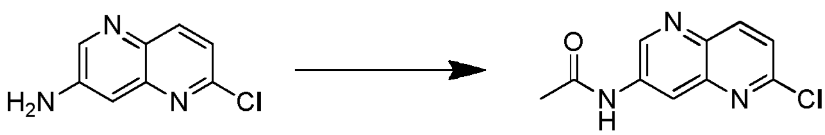

N-(6-chloro-1,5-naphthyridin-3-yl)acetamide was obtained in the same manner as in Example 0625-1 except that 6-chloro-1,5-naphthyridine-3-amine was used instead of the $N^2$-(5-isopropylpyridazin-3-yl)-1,5-naphthyridine-2,7-diamine used in Example 0625.
MSm/z(M+H):222.

<0627-3 and 0628-3>

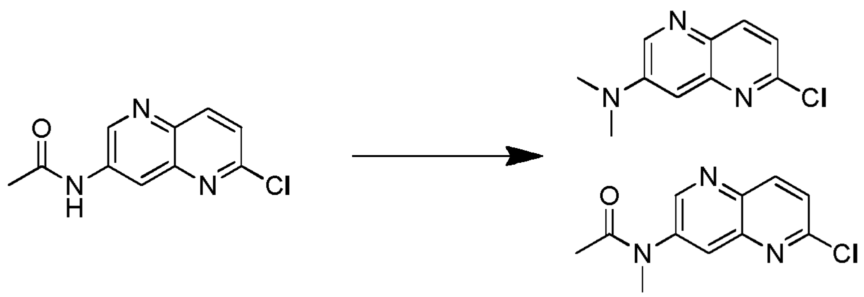

A mixture of N-(6-chloro-1,5-naphthyridin-3-yl)acetamide (16 mg), N,N-dimethylformamide (1 mL), iodomethane (7 μL), and 60% sodium hydride (3 mg) was stirred at room temperature for 1 hour. Iodomethane (7 μL) and 60% sodium hydride (3 mg) were added to the reaction mixture, followed by stirring at room temperature for 3 hours. The solvent was distilled off under reduced pressure, and the obtained residue was purified by silica gel column chromatography (hexane-ethyl acetate-methanol), thereby obtaining 6-chloro-N,N-dimethyl-1,5-naphthyridine-3-amine (10 mg) and N-(6-chloro-1,5-naphthyridin-3-yl)-N-methylacetamide (7 mg).
6-Chloro-N,N-dimethyl-1,5-naphthyridine-3-amine
MSm/z(M+H):208.
N-(6-chloro-1,5-naphthyridin-3-yl)-N-methylacetamide
MSm/z(M+H):236.

<0627-4>

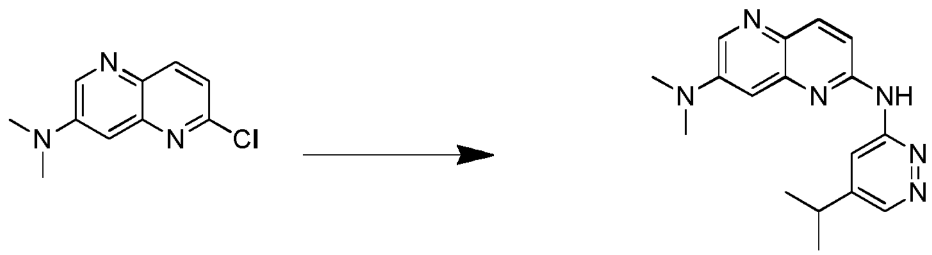

$N^2$-(5-isopropylpyridazin-3-yl)-$N^7$,$N^7$-dimethyl-1,5-naphthyridine-2,7-diamine was obtained as a yellow solid in the same manner as in Example 0646-3 except that 6-chloro-N,N-dimethyl-1,5-naphthyridine-3-amine was used instead of the 2-chloro-7-(1-(2,2-dimethoxypropyl)-1H-pyrazol-4-yl)-1,5-naphthyridine used in Example 0646-3.
$^1$H-NMR(DMSO-$d_6$)δ:10.48(1H,s),8.82(1H,d,J=2.0Hz),8.70(1H,d,J=2.0Hz),8.53(1H,d,J=2.6Hz),8.05(1H,d,J=9.2Hz),7.43(1H,d,J=9.2Hz),7.05(1H,d,J=2.6Hz),3.09(6H,s),3.05-2.96(1H,m),1.30(6H,d,J=6.6Hz).
MSm/z(M+H):309.

<0628-4>

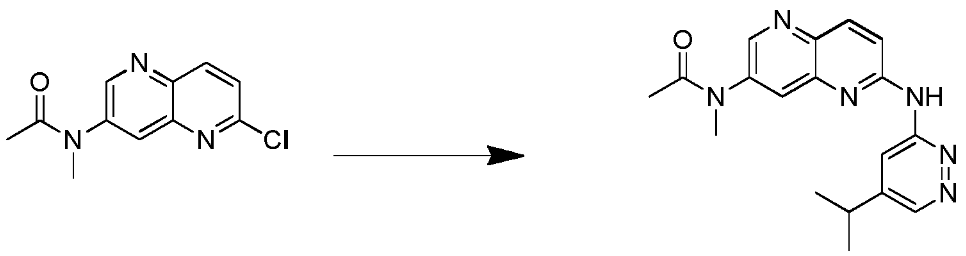

N-(6-((5-isopropylpyridazin-3-yl)amino)-1,5-naphthyridin-3-yl)-N-methylacetamide was obtained as a pale yellow solid in the same manner as in Example 0646-3 except that N-(6-chloro-1,5-naphthyridin-3-yl)-N-methylacetamide was used instead of the 2-chloro-7-(1-(2,2-dimethoxypropyl)-1H-pyrazol-4-yl)-1,5-naphthyridine used in Example 0646-3.
$^1$H-NMR(DMSO-$d_6$)δ:10.81(1H,s),8.87(1H,d,J=2.0Hz),8.74(2H,s),8.28(1H,d,J=9.2Hz),8.16(1H,s),7.77(1H,d,J=9.2Hz), 3.33(3H,s),3.06-2.97(1H,m),2.50(3H,s), 1.31(6H,d,J=6.6Hz).
MSm/z(M+H):337.

<Example 0629>

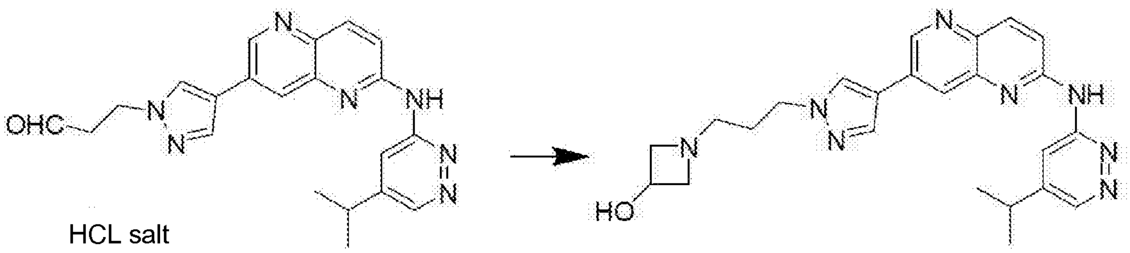


1-(3-(4-(6-((5-Isopropylpyridazin-3-yl)amino)-1,5-naphthyridin-3-yl)-1H-pyrazol-1-yl)propyl)azetidin-3-ol was obtained as a pale yellow solid in the same manner as in Example 0426-2 except that 3-hydroxyazetidine hydrochloride was used instead of the (2S,6R)-1,2,6-trimethylpiperazine trifluoroacetate used in Example 0426-2.
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.50(1H,s),8.23-8.17(3H,m),7.70(1H,d, J=9.2Hz),5.26(1H,s),4.20-4.14(3H,m),3.54-3.48(2H,m),3.08-2.99(1H,m),2.67-2.61(2H,m),2.36(2H,t,J=6.6Hz),1.87-1.78(2H,m),1.33(6H,d,J=7.3Hz).
MSm/z(M+H):445.

<Example 0630>

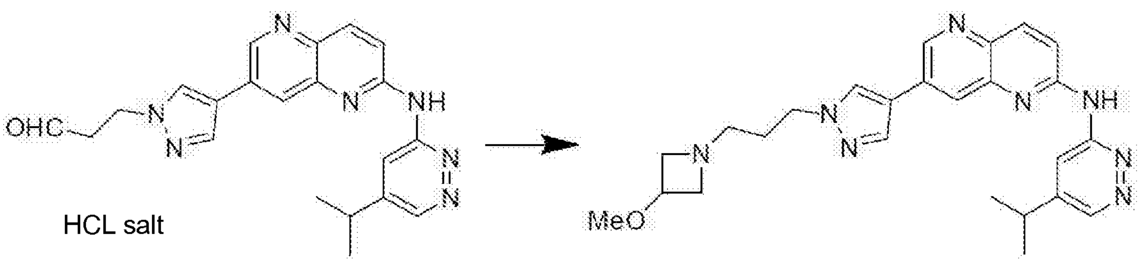


N-(5-isopropylpyridazin-3-yl)-7-(1-(3-(3-methoxyazetidin-1-yl)propyl)-1H-pyrazol-4-yl)-1,5-naphthyridine-2-amine was obtained as a pale yellow solid in the same manner as in Example 0426-2 except that 3-methoxyazetidine hydrochloride was used instead of the (2S,6R)-1,2,6-trimethylpiperazine trifluoroacetate used in Example 0426-2.
$^1$H-NMR(DMSO-

d6)δ:10.70(1H,s),9.04(1H,d,J=2.0Hz),8.87(1H,d,J=2.0Hz),8.73(1H,d,J=2.0Hz),8.50(1H,s),8.23-8.18(3H,m),7.70(1H,d, J=9.2Hz),4.18(2H,t,J=6.7Hz),3.98-3.91(1H,m),3.52-3.46(2H,m),3.14(3H,s),3.08-2.99(1H,m),2.76-2.72(2H,m),2.39(2H, t,J=6.7Hz),1.88-1.79(2H,m), 1.33(6H,d,J=6.6Hz).
MSm/z(M+H):459.

<Example 0631>

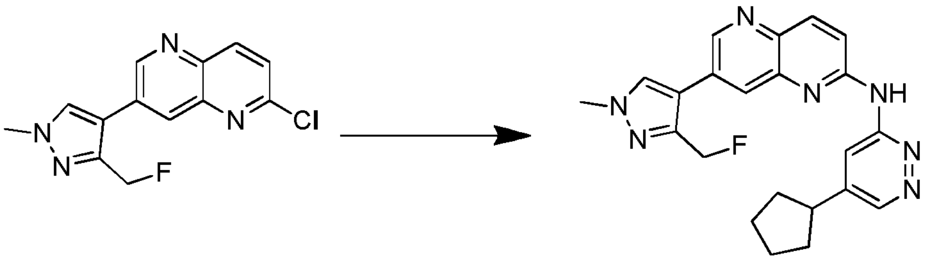

N-(5-cyclopentylpyridazin-3-yl)-7-(3-(fluoromethyl)-1-methyl-1H-pyrazol-4-yl)-1,5-naphthyridine-2-amine was obtained as a pale yellow solid in the same manner as in Example 0626-4 except that 5-cyclopentylpyridazine-3-amine was used instead of the 5-isopropylpyridazine-3-amine used in Example 0626-4.
$^1$H-NMR(DMSO-d$_6$)δ:10.76(1H,s),8.91(1H,d,J=2.0Hz),8.82(1H,d,J=2.0Hz),8.79(1H,d,J=2.0Hz),8.42(1H,s),8.26(1H,d,J =9.2Hz),8.15(1H,d,J=2.0Hz), 7.75(1H,d,J=9.2Hz),5.52(2H,d,J=49.5Hz),3.95(3H,d,J=1.3Hz),3.14-3.06(1H,m),2.18-2.08(2H,m),1.88-1.80(2H,m),1.7 8-1.64(4H,m).
MSm/z(M+H):404.

<Example 0632>

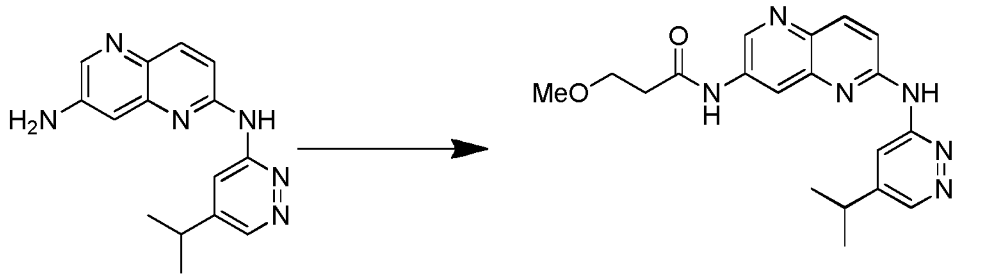

A mixture of N$^2$-(5-isopropylpyridazin-3-yl)-1,5-naphthyridine-2,7-diamine (15 mg), N,N-dimethylformamide (1 mL), 3-methoxypropionic acid (9 μL), O-(7-azabenzotriazol-1-yl)-N,N,N',N'-tetramethyluronium hexafluorophosphate (30 mg), and N,N-diisopropylethylamine (34 μL) was stirred at room temperature for 24 hours. The solvent was distilled off under reduced pressure, and the obtained residue was purified by preparative thin layer silica gel column chromatography (chloroform-methanol, NH silica), thereby obtaining N-(6-((5-isopropylpyridazin-3-yl)amino)-1,5-naphthyridin-3-yl)-3-methoxypropanamide (6 mg) as a white solid.
$^1$H-NMR(DMSO-d$_6$)δ:10.69(1H,s),10.49(1H,s),8.85(1H,d,J=2.0Hz),8.83(1H,d,J=2.0Hz),8.72(1H,d,J=2.0Hz),8.49(1H,d, J=2.0Hz),8.17(1H,d,J=9.2Hz),7.65(1H,d,J=9.2Hz),3.67(2H,t,J=5.9Hz),3.27(3H,s),3.05-2.95(1H,m),2.66(2H,t,J=5.9Hz) ,1.32(6H,d,J=7.3Hz).
MSm/z(M+H):367.

<Example 0633>

<0633-1>

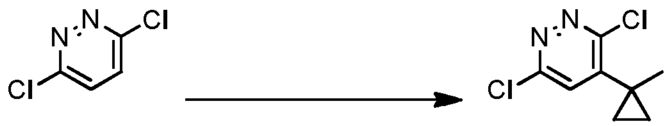

3,6-Dichloro-4-(1-methylcyclopropyl)pyridazine was obtained in the same manner as in Example 0585-1 except that 1-methylcyclopropanecarboxylic acid was used instead of the 2-ethylbutanoic acid used in Example 0585-1. MSm/z(M+H):203.

<0633-2>

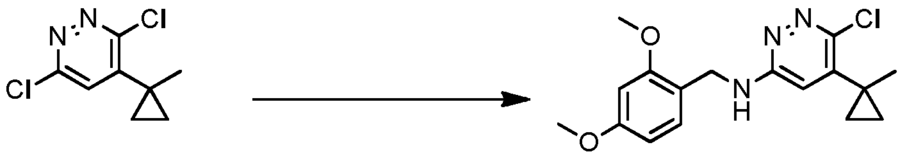

6-Chloro-N-(2,4-dimethoxybenzyl)-5-(1-methylcyclopropyl)pyridazine-3-amine was obtained in the same manner as in Example 0559-2 except that 3,6-dichloro-4-(1-methylcyclopropyl)pyridazine was used instead of the 4-(1-((tert-butyldimethylsilyl)oxy)-2-methylpropan-2-yl)-3,6-dichloropyridazine used in Example 0559-2.
MSm/z(M+H):334.

<0633-3>

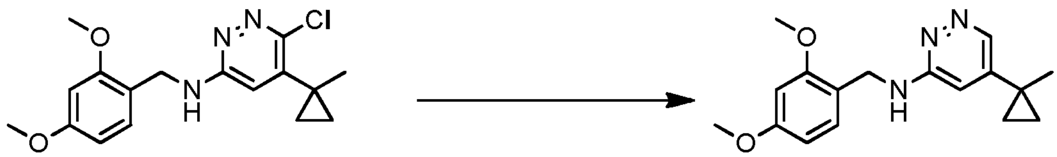

A mixture of 6-chloro-N-(2,4-dimethoxybenzyl)-5-(1-methylcyclopropyl)pyridazine-3-amine (1.98 g), ammonium formate (739 mg), tetrakis(triphenylphosphine)palladium(0) (338 mg), triethylamine (1.23 mL), and 1,2-dimethoxyethane (10 mL) was stirred at 120°C for 4 hours using a microwave reaction apparatus. After the reaction mixture was cooled to room temperature, water and ethyl acetate were added thereto, the organic layer was collected by separation, washed with a saturated sodium chloride aqueous solution, and dried over anhydrous sodium sulfate, and the solvent was distilled off under reduced pressure. The obtained residue was purified by silica gel column chromatography (methanol-ethyl acetate-hexane), thereby obtaining N-(2,4-dimethoxybenzyl)-5-(1-methylcyclopropyl)pyridazine-3-amine (431 mg).
MSm/z(M+H):300.

<0633-4>

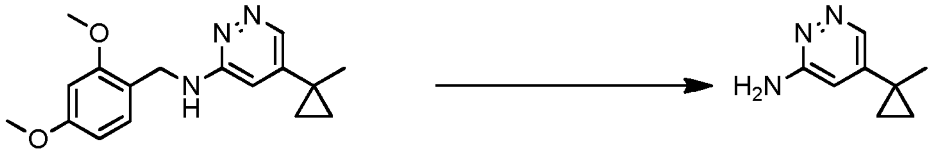

5-(1-Methylcyclopropyl)pyridazine-3-amine was obtained in the same manner as in Example 0559-4 except that N-(2,4-dimethoxybenzyl)-5-(1-methylcyclopropyl)pyridazine-3-amine was used instead of the 5-(1-((tert-butyldimethylsilyl)oxy)-2-methylpropan-2-yl)-N-(2,4-dimethoxybenzyl)pyridazine-3-amine used in Example 0559-4.
MSm/z(M+H):150.

<0633-5>

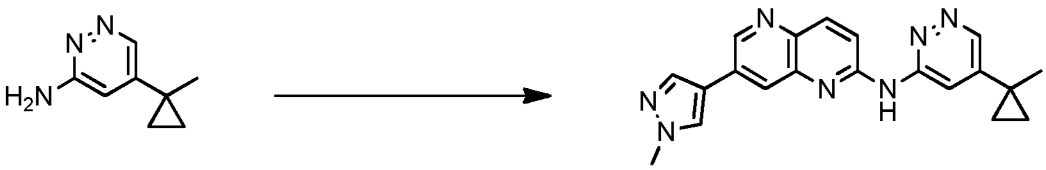

7-(1-Methyl-1H-pyrazol-4-yl)-N-(5-(1-methylcyclopropyl)pyridazin-3-yl)-1,5-naphthyridine-2-amine was obtained in the same manner as in Example 0489-4 except that 5-(1-methylcyclopropyl)pyridazine-3-amine was used instead of the 4-methylpyridazine-3-amine used in Example 0489-4.
$^1$H-NMR($CDCl_3$)$\delta$:8.87(1H,d,J=1.8Hz),8.83(1H,brs),8.57(1H,brs),8.19(1H,d,J=8.7Hz),8.10(1H,brs),7.95(2H,brs),7.49(1H,d,J=8.7Hz),4.03(3H,s),1.60(3H,s),1.22-1.15(2H,m),1.14-1.06(2H,m).
MSm/z(M+H):358.

<Example 0634>

<0634-1>

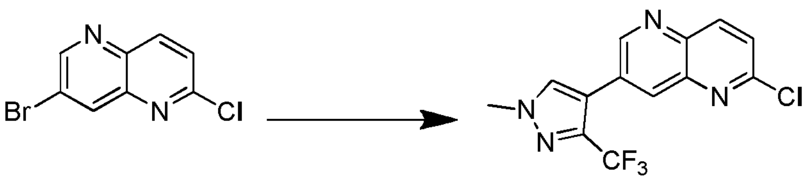

2-Chloro-7-(1-methyl-3-(trifluoromethyl)-1H-pyrazol-4-yl)-1,5-naphthyridine was obtained in the same manner as in Example 0421-1 except that 4-bromo-1-methyl-3-(trifluoromethyl)-1H-pyrazole was used instead of the N-(4-bromopyridin-2-yl)acetamide used in Example 0421-1.
MSm/z(M+H):313.

<0634-2>

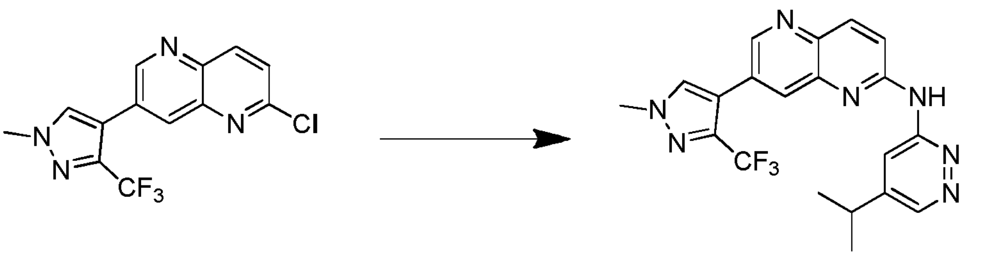

N-(5-isopropylpyridazin-3-yl)-7-(1-methyl-3-(trifluoromethyl)-1H-pyrazol-4-yl)-1,5-naphthyridine-2-amine was obtained as a pale yellow solid in the same manner as in Example 0646-3 except that 2-chloro-7-(1-methyl-3-(trifluoromethyl)-1H-pyrazol-4-yl)-1,5-naphthyridine was used instead of the 2-chloro-7-(1-(2,2-dimethoxypropyl)-1H-pyrazol-4-yl)-1,5-naphthyridine used in Example 0646-3.
$^1$H-NMR(DMSO-$d_6$)$\delta$:10.82(1H,s),8.86(1H,d,J=2.0Hz),8.80(1H,d,J=2.0Hz),8.77(1H,d,J=2.0Hz),8.49(1H,s),8.29(1H,d,J =9.2Hz),8.10(1H,d,J=2.0Hz),7.79(1H,d,J=9.2Hz),4.02(3H,s),3.06-2.96(1H,m),1.30(6H,d,J=7.3Hz).
MSm/z(M+H):414.

<Example 0635>

<0635-1>

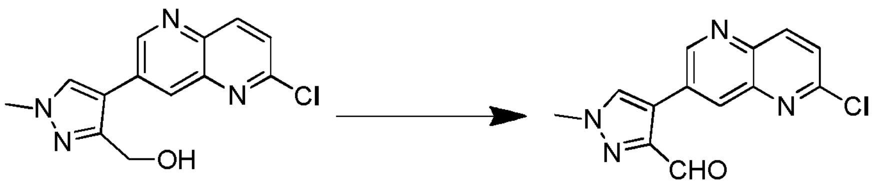

A mixture of (4-(6-chloro-1,5-naphthyridin-3-yl)-1-methyl-1H-pyrazol-3-yl)methanol (16 mg), ethyl acetate (5 mL), and manganese dioxide (16 mg) was stirred for 4.5 hours under reflux. Manganese dioxide (16 mg) was added to the reaction mixture, followed by stirring for 10.5 hours under reflux. The reaction mixture was cooled to room temperature, the insolubles were filtered off, and the solvent was distilled off under reduced pressure, thereby obtaining 4-(6-chloro-1,5-naphthyridin-3-yl)-1-methyl-1H-pyrazole-3-carbaldehyde (17 mg).
MSm/z(M+H):273.

<0635-2>

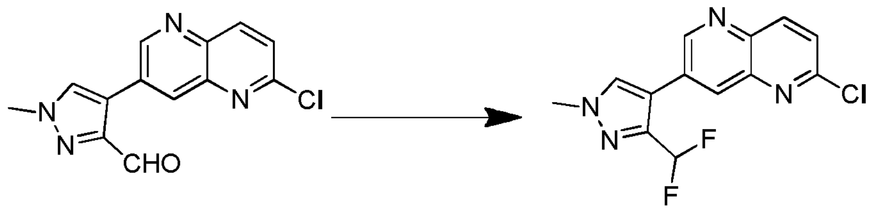

2-Chloro-7-(3-(difluoromethyl)-1-methyl-1H-pyrazol-4-yl)-1,5-naphthyridine was obtained in the same manner as in Example 0626-3 except that 4-(6-chloro-1,5-naphthyridin-3-yl)-1-methyl-1H-pyrazole-3-carbaldehyde was used instead of the (4-(6-chloro-1,5-naphthyridin-3-yl)-1-methyl-1H-pyrazol-3-yl)methanol used in Example 0626-3.
MSm/z(M+H):295.

<0635-3>

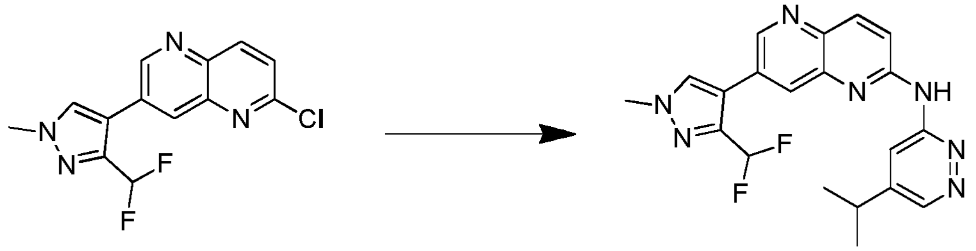

7-(3-(Difluoromethyl)-1-methyl-1H-pyrazol-4-yl)-N-(5-isopropylpyridazin-3-yl)-1,5-naphthyridine-2-amine was obtained as a pale yellow solid in the same manner as in Example 0646-3 except that 2-chloro-7-(3-(difluoromethyl)-1-methyl-1H-pyrazol-4-yl)-1,5-naphthyridine was used instead of the 2-chloro-7-(1-(2,2-dimethoxypropyl)-1H-pyrazol-4-yl)-1,5-naphthyridine used in Example 0646-3.
$^1$H-NMR(DMSO-$d_6$)δ:10.79(1H,s),8.91(1H,d,J=2.0Hz),8.86(1H,d,J=2.0Hz),8.78(1H,d,J=2.0Hz),8.49(1H,s),8.27(1H,d,J=9.2Hz),8.22(1H,d,J=2.0Hz),7.76(1H,d,J=9.2Hz),7.19(1H,t,J=53.5Hz),3.98(3H,s),3.05-2.96(1H,m),1.31(6H,d,J=6.6Hz).
MSm/z(M+H):396.

<Example 0636>

<0636-1>

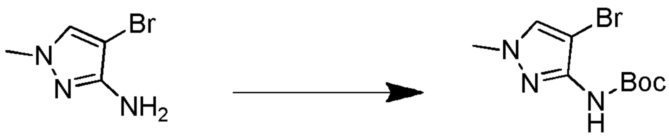

A mixture of 4-bromo-1-methyl-1H-pyrazole-3-amine (150 mg), di-tert-butyl dicarbonate (664 μL), N,N-dimethylpyridine-4-amine (11 mg), and tetrahydrofuran (5 mL) was stirred for 2.5 hours under reflux. The reaction mixture was cooled to room temperature, followed by allowing to stand overnight, and the reaction mixture was stirred for 1.5 hours under reflux. The reaction mixture was cooled to room temperature, ethyl acetate and water were added thereto, and the solvent was distilled off under reduced pressure. Ethanol (5 mL) and a 20% sodium hydroxide aqueous solution (2 mL) were added to the obtained residue, followed by stirring at room temperature for 8 hours. After water and ethyl acetate were added to the reaction mixture, the organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate), thereby obtaining tert-butyl (4-bromo-1-methyl-1H-pyrazol-3-yl)carbamate (198 mg).
$^1$H-NMR(DMSO-$d_6$)δ:8.70(1H,s),7.84(1H,s),3.75(3H,s),1.41(9H,s).

<0636-2>

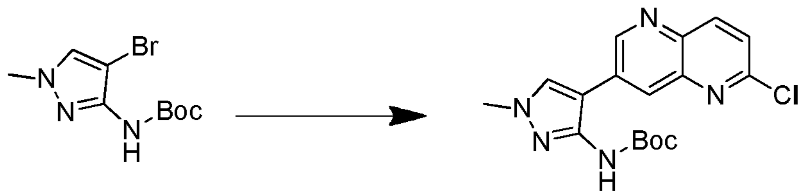

tert-Butyl (4-(6-chloro-1,5-naphthyridin-3-yl)-1-methyl-1H-pyrazol-3-yl)carbamate was obtained in the same manner as in Example 0421-1 except that tert-butyl (4-bromo-1-methyl-1H-pyrazol-3-yl)carbamate was used instead of the N-(4-bromopyridin-2-yl)acetamide used in Example 0421-1.
MSm/z(M+H):360.

<0636-3>

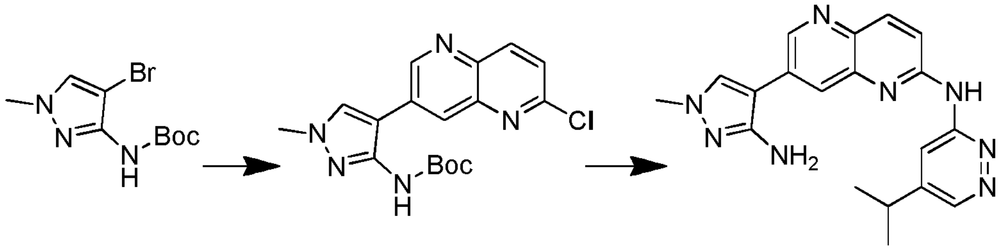

A solid matter was obtained in the same manner as in Example 0646-3 except that tert-butyl (4-bromo-1-methyl-1H-pyrazol-3-yl)carbamate was used instead of the N-(4-bromopyridin-2-yl)acetamide used in Example 0421-1.

A mixture of the obtained solid matter, 1,4-dioxane (1 mL), and 2 mol/L hydrochloric acid (0.5 mL) was stirred at 80°C for 3 hours. The reaction mixture was cooled to room temperature, a saturated sodium hydrogen carbonate aqueous solution was added thereto, and the solvent was distilled off under reduced pressure. The obtained residue was purified by preparative thin layer silica gel column chromatography (chloroform-methanol, NH silica), thereby obtaining 7-(3-amino-1-methyl-1H-pyrazol-4-yl)-N-(5-isopropylpyridazin-3-yl)-1,5-naphthyridine-2-amine (0.6 mg) as a pale yellow solid.
$^1$H-NMR(DMSO-$d_6$)δ:10.66(1H,s),8.92(1H,d,J=2.0Hz),8.85(1H,d,J=2.0Hz),8.77(1H,d,J=2.0Hz),8.19(1H,d,J=9.2Hz),8.16(1H,d,J=2.0Hz),8.05(1H,s),7.66(1H,d,J=9.2Hz),4.97(2H,s),3.69(3H,s),3.05-2.97(1H,m), 1.32(6H,d,J=6.6Hz).
MSm/z(M+H):361.

<Example 0637>

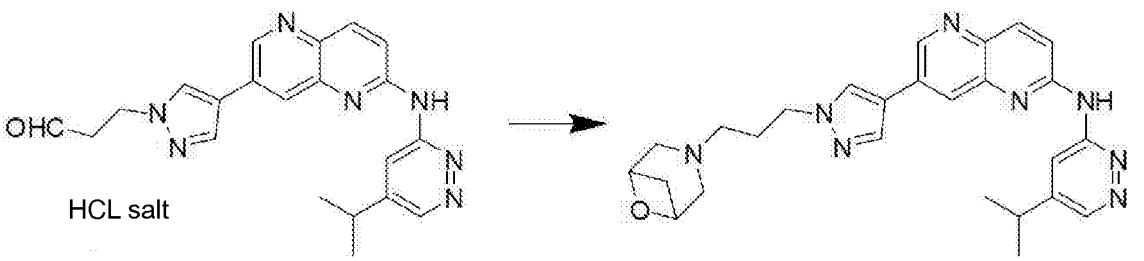


7-(1-(3-(6-Oxa-3-azabicyclo[3.1.1]heptan-3-yl)propyl)-1H-pyrazol-4-yl)-N-(5-isopropylpyridazin-3-yl)-1,5-naphthyridine-2-amine was obtained as a pale yellow solid in the same manner as in Example 0426-2 except that 6-oxa-3-azabicyclo[3.1.1]heptane hydrochloride was used instead of the (2S,6R)-1,2,6-trimethylpiperazine trifluoroacetate used in Example 0426-2.

$^1$H-NMR(DMSO-$d_6$)$\delta$:10.70(1H,s),9.04(1H,d,J=2.0Hz),8.87(1H,d,J=2.0Hz),8.73(1H,d,J=2.0Hz),8.51(1H,s),8.32-8.19(3H,m),7.70(1H,d,J=9.2Hz),4.43(2H,d,J=5.9Hz),4.25(2H,t,J=6.6Hz),3.07-2.99(3H,m),2.88-2.81(1H,m),2.58-2.51(4H,m),2.26(1H,d,J=7.9Hz),2.10-2.00(2H,m),1.33(6H,d,J=7.3Hz).
MSm/z(M+H):471.

<Example 0638> (not in accordance with the present invention)

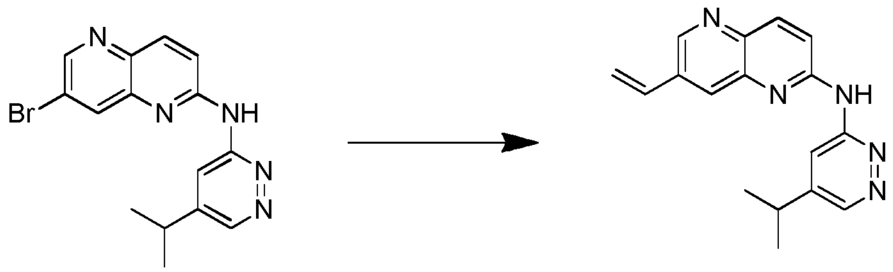

A mixture of 7-bromo-N-(5-isopropylpyridazin-3-yl)-1,5-naphthyridine-2-amine (15 mg), 4,4,5,5-tetramethyl-2-vinyl-1,3,2-dioxaborolane (8 μL), sodium carbonate (11 mg), bis(di-tert-butyl (4-dimethylaminophenyl)phosphine)dichloropalladium(II) (1 mg), 1,4-dioxane (2 mL), and water (0.2 mL) was stirred at 100°C for 6 hours in a nitrogen atmosphere. The reaction mixture was cooled to room temperature, and the solvent was distilled off under reduced pressure. The obtained residue was purified by silica gel column chromatography (hexane-ethyl acetate-methanol), thereby obtaining N-(5-isopropylpyridazin-3-yl)-7-vinyl-1,5-naphthyridine-2-amine (9 mg) as a pale yellow solid.
$^1$H-NMR(DMSO-$d_6$)$\delta$:10.74(1H,s),8.94(1H,d,J=2.0Hz),8.86(1H,d,J=2.0Hz),8.74(1H,d,J=2.0Hz),8.24(1H,d,J=9.2Hz),8.1 3(1H,s),7.73(1H,d,J=9.2Hz),6.99(1H,dd,J=17.8, 11.2Hz),6.21(1H,d,J=17.8Hz),5.54(1H,d,J=11.2Hz),3. 08-2.97(1H,m),1.32(6H,d,J=6.6Hz).
MSm/z(M+H):292.

<Example 0639> (not in accordance with the present invention)

<0639-1>

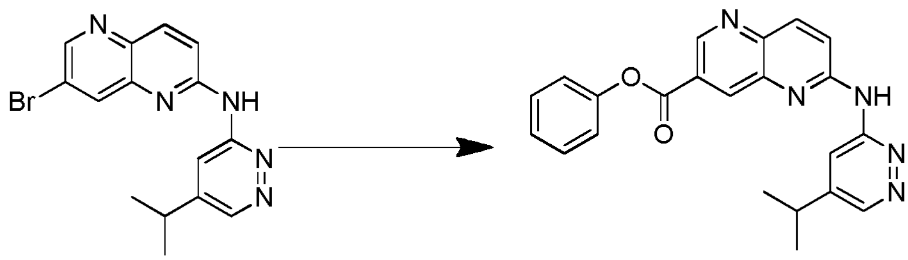

A mixture of 7-bromo-N-(5-isopropylpyridazin-3-yl)-1,5-naphthyridine-2-amine (200 mg), phenyl formate (126 μL), palladium acetate (4 mg), tri-tert-butylphosphonium tetrafluoroborate (20 mg), triethylamine (169 μL), and N,N-dimethylformamide (6 mL) was stirred at 150°C for 30 minutes using a microwave reaction apparatus. The reaction mixture was cooled to room temperature, water was added thereto, and the solid matter was collected by filtration. The obtained solid matter was suspended by the addition of ethanol, and the solvent was distilled off under reduced pressure, thereby obtaining phenyl 6-((5-isopropylpyridazin-3-yl)amino)-1,5-naphthyridine-3-carboxylate (118 mg). MSm/z(M+H):386.

<0639-2>

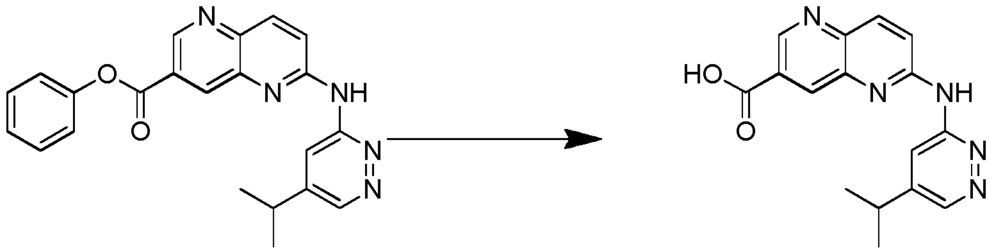

A mixture of phenyl 6-((5-isopropylpyridazin-3-yl)amino)-1,5-naphthyridine-3-carboxylate (116 mg), methanol (3 mL), and a 2 mol/L sodium hydroxide aqueous solution (1 mL) was stirred at room temperature for 17 hours. 2 mol/ hydrochloric acid was added to the reaction mixture, and the solvent was distilled off under reduced pressure. The obtained residue was purified by silica gel column chromatography (hexane-ethyl acetate-methanol), thereby obtaining 6-((5-isopropylpyridazin-3-yl)amino)-1,5-naphthyridine-3-carboxylic acid (91 mg) as a pale yellow solid.
$^1$H-NMR(DMSO-$d_6$)$\delta$:10.68(1H,s),9.14(1H,d,J=2.0Hz),8.84(1H,d,J=2.0Hz),8.80(1H,d,J=2.0Hz),8.35(1H,s),8.22(1H,d,J =9.2Hz),7.73(1H,d,J=9.2Hz),3.08-2.98(1H,m),1.32(6H,d,J=6.6Hz).
MSm/z(M+H):310.

<Example 0640> (not in accordance with the present invention)

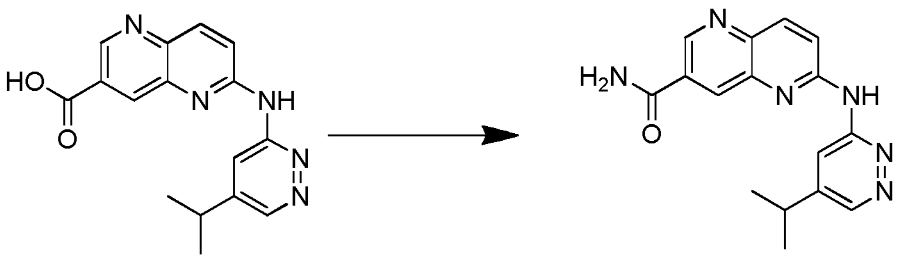

A mixture of 6-((5-isopropylpyridazin-3-yl)amino)-1,5-naphthyridine-3-carboxylic acid (40 mg), ammonium chloride (3 mg), 1-ethyl-3-(3-dimethylaminopropyl)carbodiimide hydrochloride (7 mg), 1-hydroxybenzotriazole (5 mg), N,N-diisopropylethylamine (16 $\mu$L), and N,N-dimethylformamide (1 mL) was stirred at room temperature for 17.5 hours. The solvent was distilled off under reduced pressure, and the obtained residue was purified by preparative thin layer silica gel column chromatography (chloroform-methanol, NH silica), thereby obtaining 6-((5-isopropylpyridazin-3-yl)amino)-1,5-naphthyridine-3-carboxamide (5 mg) as a pale yellow solid.
$^1$H-NMR(DMSO-$d_6$)$\delta$:10.85(1H,s),9.13(1H,d,J=2.0Hz),8.88(1H,d,J=2.0Hz),8.77(1H,d,J=2.0Hz),8.54(1H,d,J=2.0Hz),8.42(1H,s),8.31(1H,d,J=9.2Hz),7.83(1H,d,J=9.2Hz),7.77(1H,s),3.08-3.00(1H,m),1.33(6H,d,J=6.6Hz).
MSm/z(M+H):309.

<Example 0641>

<0641-1>

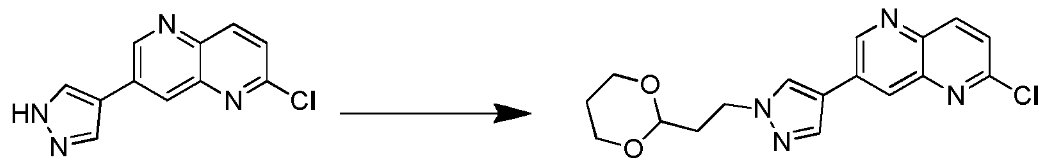

A mixture of 2-chloro-7-(1H-pyrazol-4-yl)-1,5-naphthyridine (500 mg), 2-(2-bromoethyl)-1,3-dioxane (439 μL), potassium carbonate (900 mg), and N,N-dimethylformamide (4 mL) was stirred at 80°C for 8.5 hours. The reaction mixture was cooled to room temperature, water was added thereto, and the solid matter was collected by filtration. Ethyl acetate and hexane were added to the obtained solid matter, and the solid matter was collected by filtration, thereby obtaining 7-(1-(2-(1,3-dioxan-2-yl)ethyl)-1H-pyrazol-4-yl)-2-chloro-1,5-naphthyridine (572 mg).
MSm/z(M+H):345.

<0641-2>

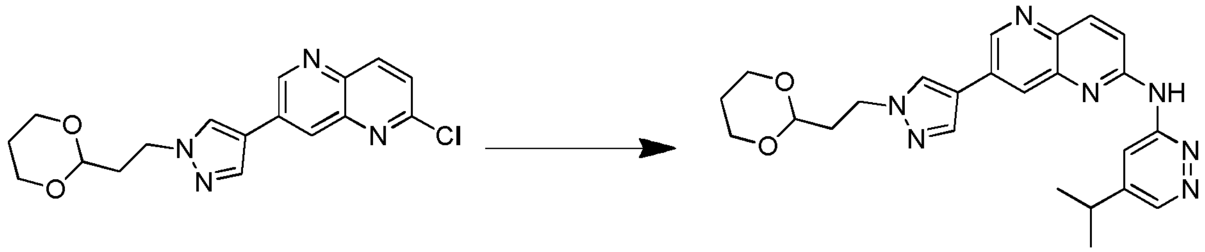

7-(1-(2-(1,3-Dioxan-2-yl)ethyl)-1H-pyrazol-4-yl)-N-(5-isopropylpyridazin-3-yl)-1,5-naphthyridine-2-amine was obtained as a pale yellow solid in the same manner as in Example 0411-3 except that 7-(1-(2-(1,3-dioxan-2-yl)ethyl)-1H-pyrazol-4-yl)-2-chloro-1,5-naphthyridine was used instead of the 2-chloro-7-(1-((6-methylpyridin-2-yl)methyl)-1H-pyrazol-4-yl)-1,5-naphthyridine used in Example 0411-3.
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.52(1H,s),8.23-8.19(3H,m),7.70(1H,d, J=9.2Hz),4.58(1H,t,J=5.3Hz),4.23(2H,t,J=7.3Hz),4.03(2H,dd,J=10.6,4.6Hz),3. 75-3.66(2H,m),3.08-2.98(1H,m),2.12-2.03(2H,m), 1.95-1.80(2H,m), 1.33(6H,d,J=7.3Hz).
MSm/z(M+H):446.

<Example 0642> (not in accordance with the present invention)

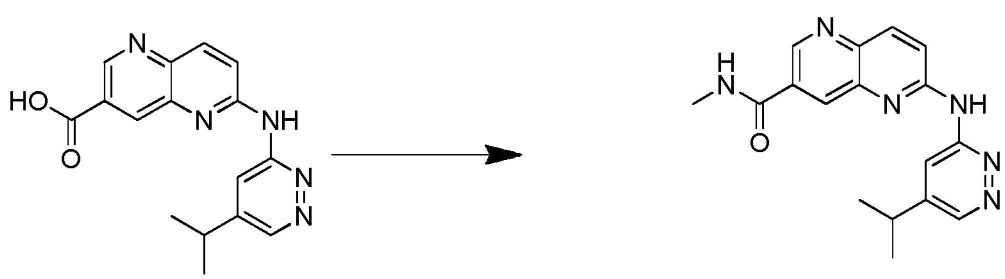

6-((5-Isopropylpyridazin-3-yl)amino)-N-methyl-1,5-naphthyridine-3-carboxamide was obtained as a pale yellow solid in the same manner as in Example 0640-3 except that methylamine hydrochloride was used instead of the ammonium chloride used in Example 0640-3.
$^1$H-NMR(DMSO-$d_6$)δ:10.85(1H,s),9.10(1H,d,J=2.0Hz),8.90-8.84(2H,m),8.76(1H,d,J=2.0Hz),8.49(1H,d,J=2.0Hz),8.31(1H,d,J=9.2Hz),7. 83(1H,d,J=9.2Hz),3.08-2.98(1H,m),2.87(3H,d,J=4.0Hz),1.33(6H,d,J=6.6Hz).

MSm/z(M+H):323.

<Example 0643> (not in accordance with the present invention)

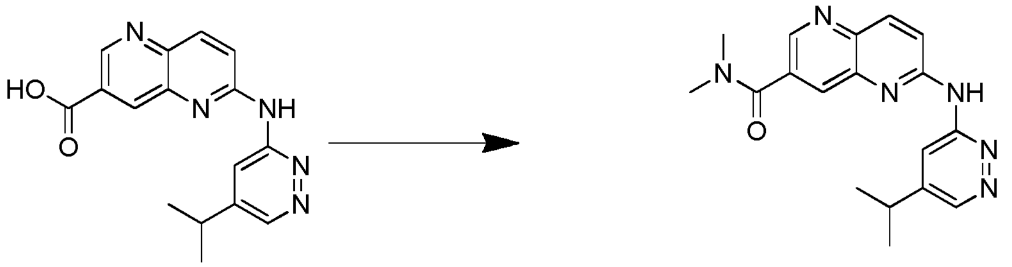

6-((5-Isopropylpyridazin-3-yl)amino)-N,N-dimethyl-1,5-naphthyridine-3-carboxamide was obtained as a pale yellow solid in the same manner as in Example 0640-3 except that dimethylamine hydrochloride was used instead of the ammonium chloride used in Example 0640-3.
$^1$H-NMR(DMSO-$d_6$)δ:10.84(1H,s),8.87(1H,d,J=2.0Hz),8.76(1H,d,J=2.0Hz),8.73(1H,d,J=2.0Hz),8.29(1H,d,J=9.2Hz),8.16(1H,d,J=2.0Hz),7.82(1H,d,J=9.2Hz),3.07(3H,s),3.07-2.99(1H,m),2.99(3H,s),1.31(6H,d,J=7.2Hz).
MSm/z(M+H):337.

<Example 0644>

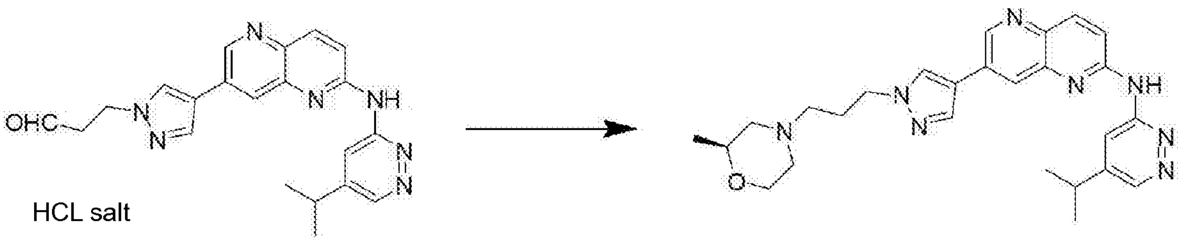

HCL salt

(S)-N-(5-isopropylpyridazin-3-yl)-7-(1-(3-(2-methylmorpholino)propyl)-1H-pyrazol-4-yl)-1,5-naphthyridine-2-amine was obtained as a pale yellow solid in the same manner as in Example 0426-2 except that (S)-2-methylmorpholine was used instead of the (2S,6R)-1,2,6-trimethylpiperazine trifluoroacetate used in Example 0426-2.
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.51(1H,s),8.23-8.19(3H,m),7.70(1H,d,J=8.6Hz),4.21(2H,t,J=6.9Hz),3.73(1H,d,J=9.2Hz),3.54-3.46(2H,m),3.08-2.98(1H,m),2.75-2.64(2H,m),2.28(2H,t,J=6.9Hz),2.05-1.90(3H,m),1.64(1H,t,J=10.6Hz),1.33(6H,d,J=6.6Hz),1.03(3H,d,J=6.6Hz).
MSm/z(M+H):473.

<Example 0645>

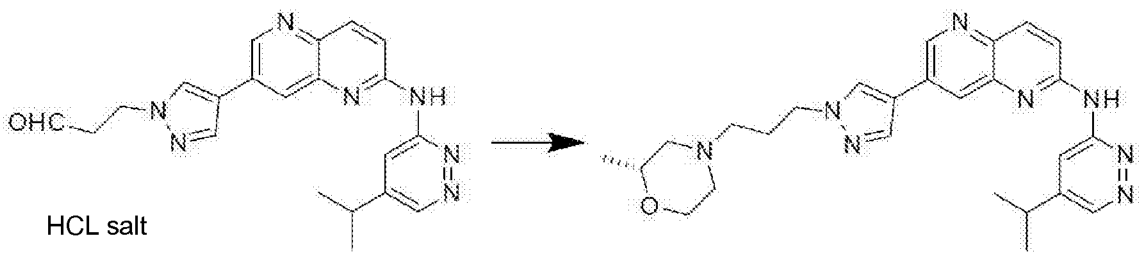

HCL salt

(R)-N-(5-isopropylpyridazin-3-yl)-7-(1-(3-(2-methylmorpholino)propyl)-1H-pyrazol-4-yl)-1,5-naphthyridine-2-amine was obtained as a pale yellow solid in the same manner as in Example 0426-2 except that (R)-2-methylmorpholine was used instead of the (2S,6R)-1,2,6-trimethylpiperazine trifluoroacetate used in Example 0426-2.
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.51(1H,s),8.23-8.19(3H,m),7.70(1H,d,J=9.2Hz),4.21(2H,t,J=6.9Hz),3.73(1H,d,J=11.2Hz),3.54-3.45(2H,m),3.08-3.00(1H,m),2.75-2.64(2H,m),2.28(2H,t,J=6.9Hz),2.05-1.90(3H,m), 1.67-1.57(1H,m), 1.33(6H,d,J=6.6Hz), 1.03(3H,d,J=6.6Hz).
MSm/z(M+H):473.

<Example 0646>

<0646-1>

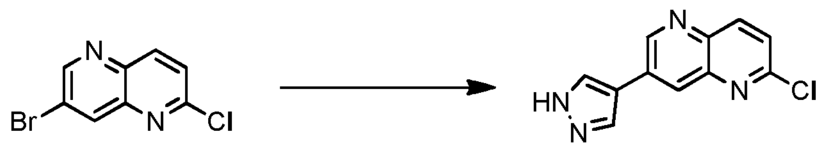

A mixture of 7-bromo-2-chloro-1,5-naphthyridine (5.0 g), 1-tert-butoxycarbonyl-4-(4,4,5,5-tetramethyl-1,3,2-dioxaborolan-2-yl)pyrazole (6.04 g), sodium carbonate (4.4 g), bis(di-tert-butyl (4-dimethylaminophenyl)phosphine)dichloropalladium(II) (366 mg), 1,4-dioxane (24 mL), and water (2.4 mL) was stirred at 110°C for 5 hours in a nitrogen atmosphere. The reaction mixture was cooled to room temperature, water was added thereto, and the solid matter was collected by filtration. The obtained solid matter was suspended by the addition of ethyl acetate, and the solid matter was collected by filtration, thereby obtaining 2-chloro-7-(1H-pyrazol-4-yl)-1,5-naphthyridine (2.6 g) as a pale brown solid.
MSm/z(M+H):231.

<0646-2>

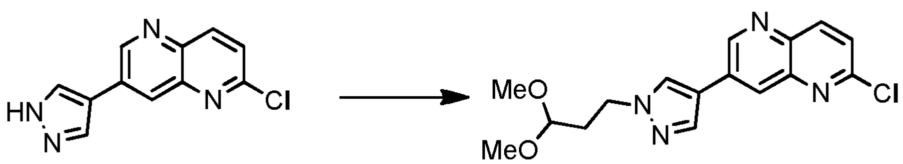

A mixture of 2-chloro-7-(1H-pyrazol-4-yl)-1,5-naphthyridine (2.5 g), potassium carbonate (3.0 g), 3-bromo-1,1-dimethoxypropane (2.2 mL), and N,N-dimethylformamide (10.8 mL) was stirred at 80°C for 2 hours. The reaction mixture was cooled to room temperature, water was added thereto, and the solid matter was collected by filtration, thereby obtaining 2-chloro-7-(1-(3,3-dimethoxypropyl)-1H-pyrazol-4-yl)-1,5-naphthyridine (3.17 g) as a pale brown solid.
MSm/z(M+H):333.

<0646-3>

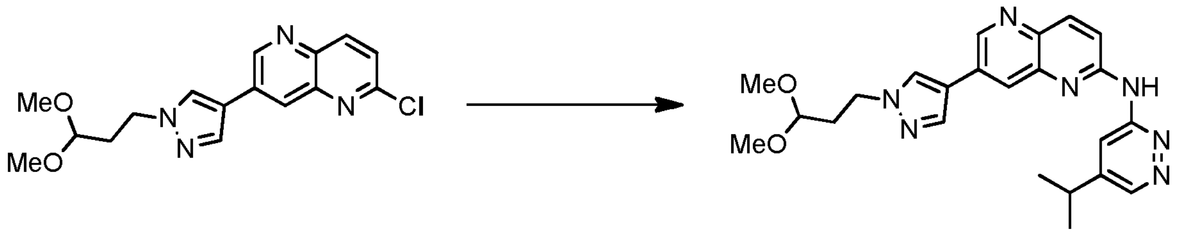

A mixture of 2-chloro-7-(1-(3,3-dimethoxypropyl)-1H-pyrazol-4-yl)-1,5-naphthyridine (3.1 g), 5-isopropylpyridazine-3-amine (1.4 g), [(2-di-cyclohexylphosphino-3,6-dimethoxy-2',4',6'-triisopropyl-1,1'-biphenyl)-2-(2'-amino-1,1'-biphenyl)]palladium(II)methanesulfonate (BRETTPHOS-PD-G3 (product name, manufactured by Sigma-Aldrich Co. LLC.)) (422 mg), cesium carbonate (7.6 g), and 1,4-dioxane (93 mL) was stirred at 110°C for 4 hours in a nitrogen atmosphere. The reaction mixture was cooled to room temperature, and the solvent was distilled off under reduced pressure. The obtained residue was purified by silica gel column chromatography (methanol-chloroform, NH silica), thereby obtaining 7-(1-(3,3-dimethoxypropyl)-1H-pyrazol-4-yl)-N-(5-isopropylpyridazin-3-yl)-1,5-naphthyridine-2-amine (1.66 g) as a pale yellow solid.
$^1$H-NMR($CDCl_3$)$\delta$:9.77(1H,s),8.94(2H,dd,J=10.2,1. 7Hz),8.84(1H,d,J=2.0Hz),8.25(1H,d,J=9.2Hz),8.10(1 H,d,J=1.3Hz),7.98(1H,s),7.85(1H,s),7.74(1H,d,J=9.2Hz),4.38(1H,t,J=5.6Hz),4.30(2H,t,J=6.9Hz),3.37( 6H,s),3.13-3.03 (1H,m),2.26(2H,q,J=6.6Hz),1.43(6H,d,J=6.6Hz).
MSm/z(M+H):434.

<Example 0647> (not in accordance with the present invention)

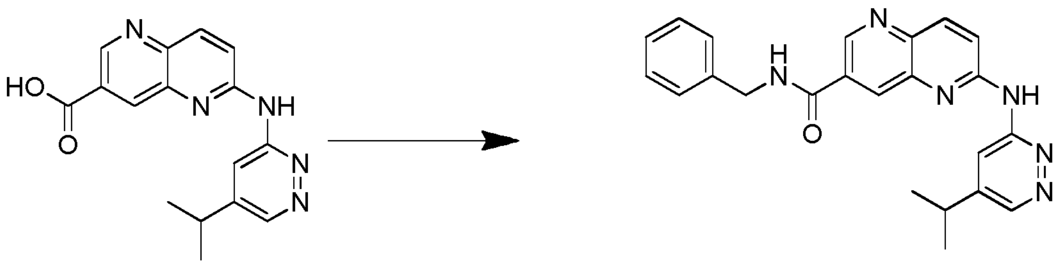

A mixture of 6-((5-isopropylpyridazin-3-yl)amino)-1,5-naphthyridine-3-carboxylic acid (15 mg), benzylamine (9 μL), 1-ethyl-3-(3-dimethylaminopropyl)carboimide hydrochloride (30 mg), N,N-diisopropylethylamine (34 μL), and N,N-dimethylformamide (1 mL) was stirred at room temperature for 2 hours. The solvent was distilled off under reduced pressure, and the obtained residue was purified by preparative thin layer silica gel column chromatography (chloroform-methanol, NH silica), thereby obtaining N-benzyl-6-((5-isopropylpyridazin-3-yl)amino)-1,5-naphthyridine-3-carboxamide (3 mg) as a white solid.
$^1$H-NMR(DMSO-d$_6$)$\delta$:10.86(1H,s),9.48(1H,dd,J=6.0,6.0Hz),9.16(1H,d,J=2.0Hz),8.89(1H,d,J=2.0Hz),8.75(1H,d,J=2.0H z),8.56(1H,d,J=2.0Hz),8.32(1H,d,J=9.2Hz),7.84(1H,d,J=9.2Hz),7.40-7.33(3H,m),7.28(1H,d,J=6.0Hz),4.57(2H,d,J=6.0 Hz),3.07-2.98(1H,m),1.32(6H,d,J=7.3Hz).
MSm/z(M+H):399.

<Example 0648> (not in accordance with the present invention)

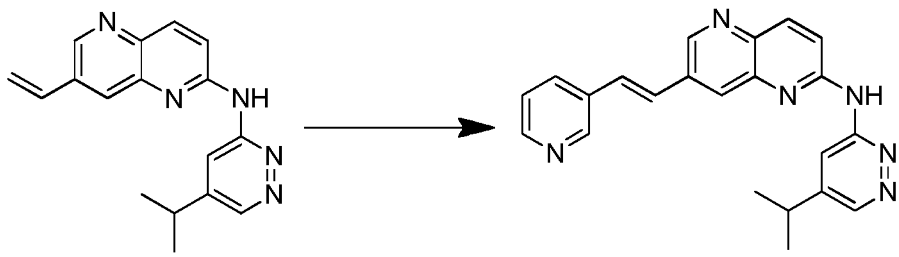

A mixture of N-(5-isopropylpyridazin-3-yl)-7-vinyl-1,5-naphthyridine-2-amine (15 mg), 3-bromopyridine (10 μL), palladium acetate (1 mg), tri(o-tolyl)phosphine (1 mg), triethylamine (14 μL), and N,N-dimethylformamide (1 mL) was stirred at 150°C for 1 hour using a microwave reaction apparatus. The reaction mixture was cooled to room temperature, water was added thereto, and the solid matter was collected by filtration, thereby obtaining (E)-N-(5-isopropylpyridazin-3-yl)-7-(2-(pyridin-3-yl)vinyl)-1,5-naphthyridine-2-amine (11 mg) as a pale yellow solid.
$^1$H-NMR(DMSO-$d_6$)δ:10.76(1H,s),9.10(1H,d,J=2.0Hz),8.87(2H,d,J=2.0Hz),8.76(1H,d,J=2.0Hz),8.53-8.50(1H,m),8.27-8.23(2H,m),8.16-8.11(1H,m),7.74(1H,d,J=9.2Hz),7.67(2H,s),7.49-7.45(1H,m),3.06-3.00(1H,m),1.34(6H,d,J=7.3Hz).
MSm/z(M+H):369.

<Example 0649>

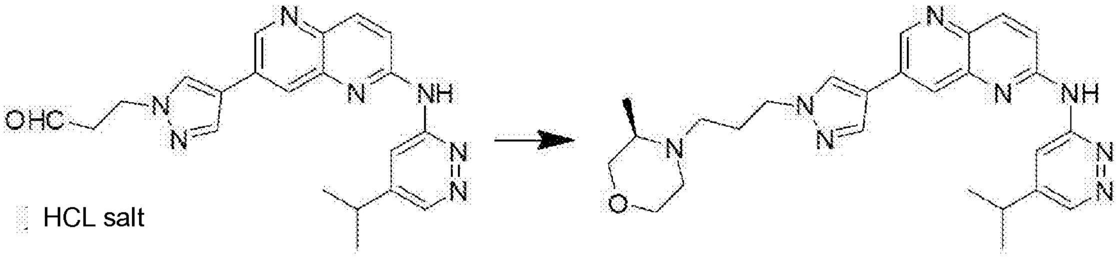

(R)-N-(5-isopropylpyridazin-3-yl)-7-(1-(3-(3-methylmorpholino)propyl)-1H-pyrazol-4-yl)-1,5-naphthyridine-2-amine was obtained as a pale yellow solid in the same manner as in Example 0426-2 except that (R)-3-methylmorpholine was used instead of the (2S,6R)-1,2,6-trimethylpiperazine trifluoroacetate used in Example 0426-2.
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.52(1H,s),8.32-8.19(3H,m),7.69(1H,d,J=9.2Hz),4.20(2H,t,J=6.6Hz),3.71-3.65(1H,m),3.60-3.45(2H,m),3.17-2.99(2H,m),2.74-2.63(2H,m),2.33-2.26(1H,m),2.19-2.10(2H,m),2.03-1.96(2H,m), 1.34(6H,d,J=6.6Hz),0.85(3H,d,J=5.9Hz).
MSm/z(M+H):473.

<Example 0650>

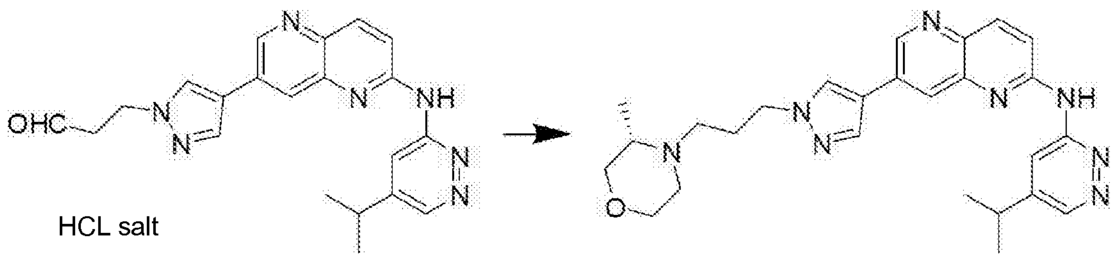

(S)-N-(5-isopropylpyridazin-3-yl)-7-(1-(3-(3-methylmorpholino)propyl)-1H-pyrazol-4-yl)-1,5-naphthyridine-2-amine was obtained as a pale yellow solid in the same manner as in Example 0426-2 except that (S)-3-methylmorpholine was used instead of the (2S,6R)-1,2,6-trimethylpiperazine trifluoroacetate used in Example 0426-2.
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.52(1H,s),8.32-8.18(3H,m),7.69(1H,d,J=9.2Hz),4.20(2H,t,J=6.6Hz),3.70-3.46(3H,m),3.18-3.00(2H,m),2.75-2.63(2H,m),2.32-2.26(1H,m),2.19-2.10(2H,m),2.04-1.96(2H,m),1.33(6H,d,J=7.3Hz),0.85(3H,d,J=6.6Hz).
MSm/z(M+H):473.

<Example 0651>

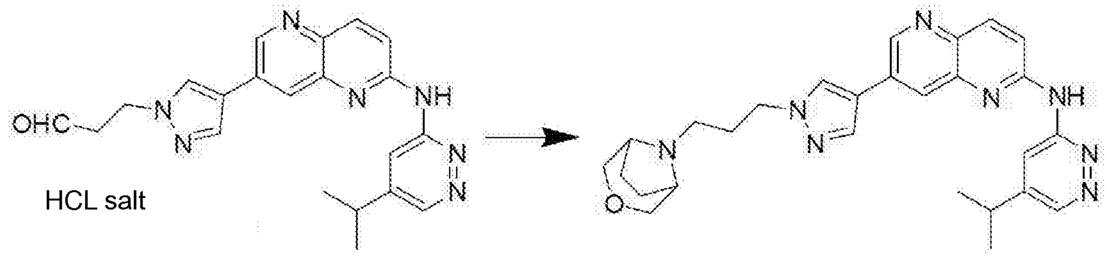


7-(1-(3-(3-oxa-8-azabicyclo[3.2.1]octan-8-yl)propyl)-1H-pyrazol-4-yl)-N-(5-isopropylpyridazin-3-yl)-1,5-naphthyridine-2-amine was obtained as a pale yellow solid in the same manner as in Example 0426-2 except that 3-oxa-8-azabicyclo[3.2.1]octane was used instead of the (2S,6R)-1,2,6-trimethylpiperazine trifluoroacetate used in Example 0426-2.
$^1$H-NMR(DMSO-$d_6$)δ:10.70(1H,s),9.05(1H,d,J=2.0Hz),8.87(1H,d,J=2.0Hz),8.73(1H,d,J=2.0Hz),8.52(1H,s),8.31-8.17(3H,m),7.70(1H,d,J=9.2Hz),4.27(2H,t,J=6.9Hz),3.56-3.38(5H,m),3.08-2.99(2H,m),2.22(2H,t,J=6.9Hz),2.01-1.92(2H,m),1.82-1.74(2H,m),1.72-1.65(2H,m),1.33(6H,d,J=6.6Hz).
MSm/z(M+H):485.

<Example 0652> (not in accordance with the present invention)

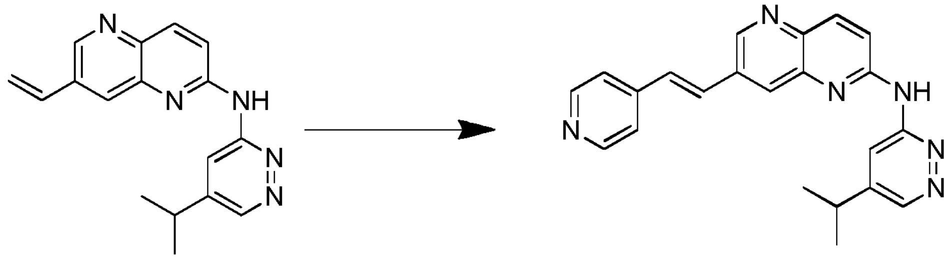

(E)-N-(5-isopropylpyridazin-3-yl)-7-(2-(pyridin-4-yl)vinyl)-1,5-naphthyridine-2-amine was obtained as a pale yellow solid in the same manner as in Example 0648-1 except that 4-bromopyridine hydrochloride was used instead of the 3-bromopyridine used in Example 0648-1.
$^1$H-NMR(DMSO-$d_6$)δ:10.78(1H,s),9.12(1H,d,J=2.0Hz),8.88(1H,d,J=2.0Hz),8.76(1H,d,J=2.0Hz),8.61(2H,d,J=5.9Hz),8.30(1H,d,J=2.0Hz),8.26(1H,d,J=9.2Hz),7.78-7.73(2H,m),7.68-7.60(3H,m),3.10-3.00(1H,m),1.34(6H,d,J=6.6Hz).
MSm/z(M+H):369.

<Example 0653>

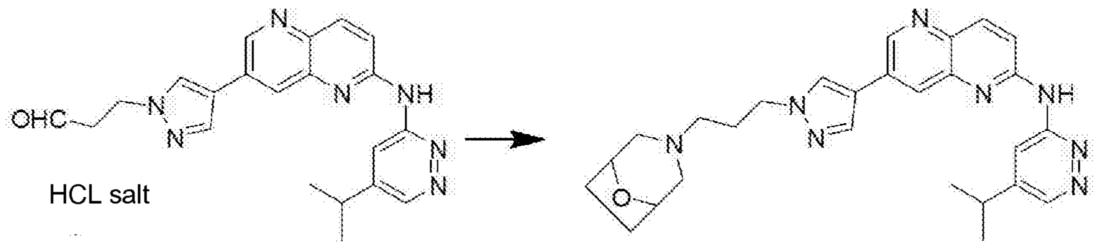


7-(1-(3-(8-Oxa-3-azabicyclo[3.2.1]octan-3-yl)propyl)-1H-pyrazol-4-yl)-N-(5-isopropylpyridazin-3-yl)-1,5-naphthyridine-2-amine was obtained as a pale yellow solid in the same manner as in Example 0426-2 except that 8-oxa-3-azabicyclo[3.2.1]octane was used instead of the (2S,6R)-1,2,6-trimethylpiperazine trifluoroacetate used in Example 0426-2.
$^1$H-NMR(DMSO-$d_6$)6:10.70(1H,s),9.04(1H,d,J=2.0Hz),8.87(1H,d,J=2.0Hz),8.73(1H,d,J=2.0Hz),8.48(1H,s),8.23-8.18(3H,m),7.70(1H,d,J=8.6Hz),4.25-4.18(4H,m),3.08-3.00(1H,m),2.58-2.53(2H,m),2.23(2H,t,J=6.6Hz),2.11(2H,d,J=9.2Hz),1.96(2H,t,J=6.6 Hz),1.90-1.85(2H,m),1.75-1.69(2H,m),1.34(6H,d,J=7.2Hz).
MSm/z(M+H):485.

<Example 0654> (not in accordance with the present invention)

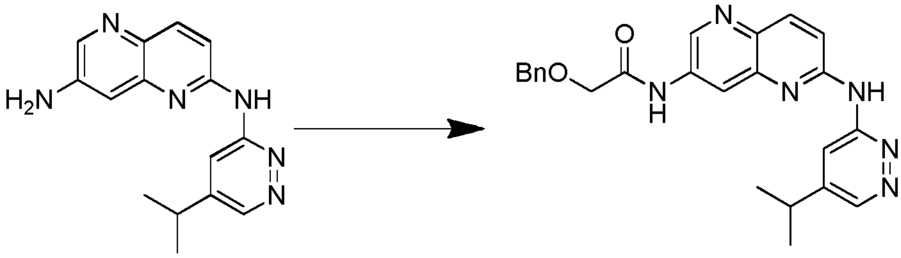

$N^2$-(5-isopropylpyridazin-3-yl)-1,5-naphthyridine-2,7-diamine (20 mg) was added to a mixture of 2-(benzyloxy)acetic acid (49 μL), dichloromethane (5 mL), oxalyl chloride (33 μL), and N,N-dimethylformamide (1 drop), followed by stirring at room temperature for 1.5 hours, and stirring at 60°C for 2 hours. The reaction mixture was cooled to room temperature, and purified by silica gel column chromatography (hexane-ethyl acetate-methanol), thereby obtaining 2-(benzyloxy)-N-(6-((5-isopropylpyridazin-3-yl)amino)-1,5-naphthyridin-3-yl)acetamide (6 mg) as a pale yellow solid.
$^1$H-NMR(DMSO-$d_6$)δ:10.71(1H,s),10.38(1H,s),8.94(1H,d,J=2.0Hz),8.85(1H,d,J=2.0Hz),8.73(1H,d,J=2.0Hz),8.50(1H,d,J=2.0Hz),8.19(1H,d,J=9.2Hz),7.67(1H,d,J=9.2Hz),7.46-7.31(5H,m),4.67(2H,s),4.20(2H,s),3.05-2.95(1H,m),1.31(6H,d,J=6.6Hz).
MSm/z(M+H):429.

<Example 0655> (not in accordance with the present invention)

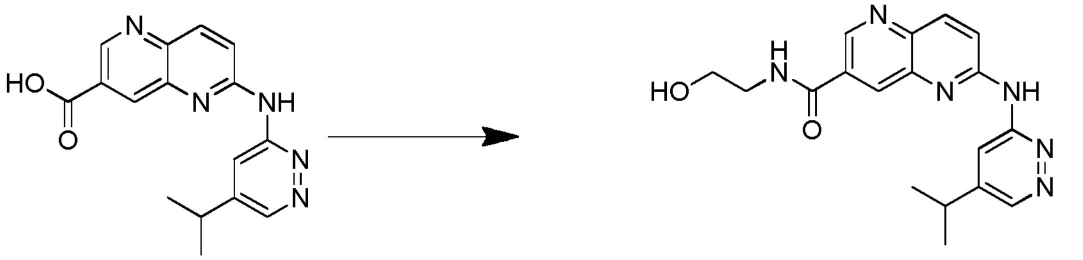

N-(2-hydroxyethyl)-6-((5-isopropylpyridazin-3-yl)amino)-1,5-naphthyridine-3-carboxamide was obtained as a pale yellow solid in the same manner as in Example 0647-1 except that 2-aminoethanol was used instead of the benzylamine used in Example 0647-1.
$^1$H-NMR(DMSO-$d_6$)δ:10.85(1H,s),9.12(1H,d,J=2.0Hz),8.94-8.87(2H,m),8.75(1H,d,J=2.0Hz),8.52(1H,d,J=2.0Hz),8.31(1H,d,J=8.9Hz),7.83(1H,d,J=8.9Hz),4.80(1 H,t,J=5.6Hz),3.61-3.54(2H,m),3.44-3.38(2H,m),3.06-3.00(1H,m),1.33(6H,d,J=6.6Hz).
MSm/z(M+H):353.

<Example 0656> (not in accordance with the present invention)

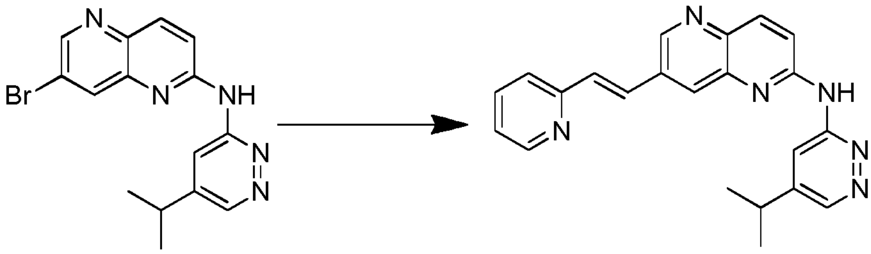

A mixture of 7-bromo-N-(5-isopropylpyridazin-3-yl)-1,5-naphthyridine-2-amine (20 mg), 2-vinylpyridine (13 μL), palladium acetate (1 mg), tri(o-tolyl)phosphine (5 mg), triethylamine (17 μL), and N,N-dimethylformamide (1 mL) was stirred at 150°C for 1 hour using a microwave reaction apparatus. The reaction mixture was cooled to room temperature, water was added thereto, and the solid matter was collected by filtration. The obtained solid matter was purified by silica gel column chromatography (hexane-ethyl acetate-methanol), thereby obtaining (E)-N-(5-isopropylpyridazin-3-yl)-7-(2-(pyridin-2-yl)vinyl)-1,5-naphthyridine-2-amine (11 mg) as a pale yellow solid.
$^1$H-NMR(DMSO-$d_6$)δ:10.76(1H,s),9.13(1H,d,J=2.0Hz),8.87(1H,d,J=2.0Hz),8.77(1H,d,J=2.0Hz),8.63(1H,d,J=4.6Hz),8.34(1H,d,J=2.0Hz),8.26(1H,d,J=8.6Hz),7.96-7.90(1H,m),7.89-7.81(1H,m),7.77-7.62(3H,m),7.33(1H,dd,J=7.3,4.6Hz),3.09-3.00(1H,m),1.34(6H,d,J=6.6Hz).
MSm/z(M+H):369.

<Example 0657>

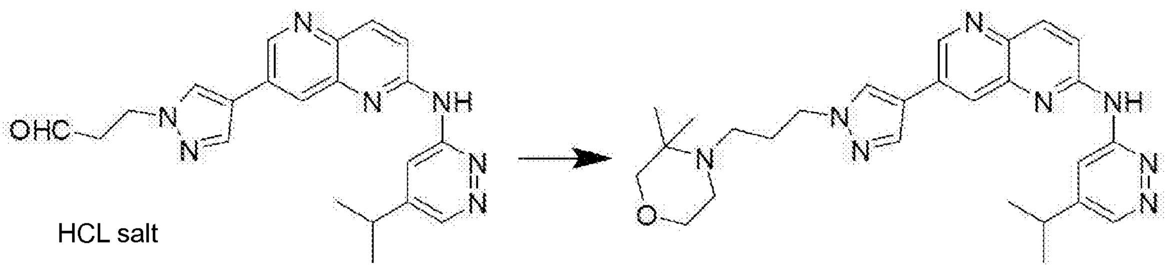

HCL salt

7-(1-(3-(3,3-Dimethylmorpholino)propyl)-1H-pyrazol-4-yl)-N-(5-isopropylpyridazin-3-yl)-1,5-naphthyridine-2-amine was obtained as a pale yellow solid in the same manner as in Example 0426-2 except that 3,3-dimethylmorpholine was used instead of the (2S,6R)-1,2,6-trimethylpiperazine trifluoroacetate used in Example 0426-2.
$^1$H-NMR($CDCl_3$)δ:9.18(1H,s),8.93(1H,d,J=2.0Hz),8.87(1H,s),8.81(1H,s),8.24(1H,d,J=8.9Hz),8.10(1H,d,J=2.0Hz),7.97(1H,s),7.88(1H,s),7.58(1H,d,J=8.9Hz),4.29(2H,t,J=6.9Hz),3.78(2H,brs),3.35(2H,brs),3.11-3.01(1H,m),2.62-2.40(2H,m),2.10(2H,brs),1.42(6H,d,J=6.6Hz), 1.25(2H,s),1.00(6H,s).
MSm/z(M+H):487.

<Example 0658>

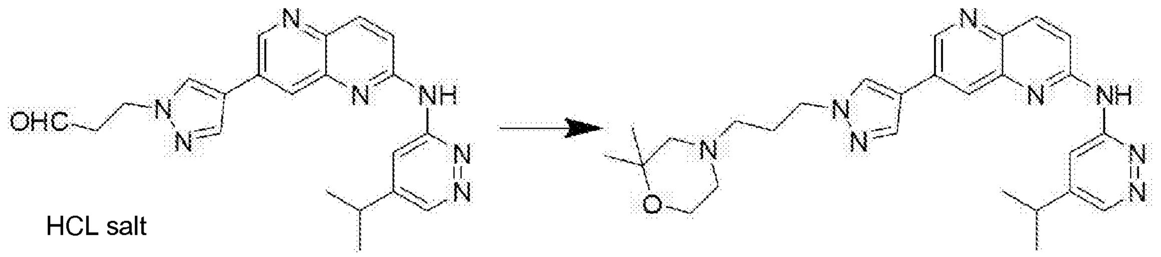

HCL salt

7-(1-(3-(2,2-Dimethylmorpholino)propyl)-1H-pyrazol-4-yl)-N-(5-isopropylpyridazin-3-yl)-1,5-naphthyridine-2-amine was obtained as a pale yellow solid in the same manner as in Example 0426-2 except that 2,2-dimethylmorpholine was used instead of the (2S,6R)-1,2,6-trimethylpiperazine trifluoroacetate used in Example 0426-2.
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.49(1H,s),8.32-8.18(3H,m),7.70(1H,d,J=9.2Hz),4.23(2H,t,J=6.9Hz),3.61(2H,t,J=5.0Hz),3.08-2.99(1H,m),2.28-2.21(4H,m),2.13(2H,s),2.05-1.95(2H,m),1.33(6H,d,J=6.6Hz),1.17(6H,s).
MSm/z(M+H):488.

<Example 0659>

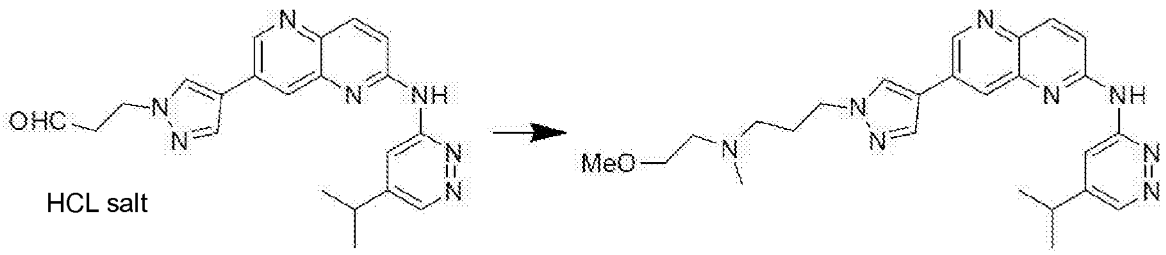

HCL salt

N-(5-isopropylpyridazin-3-yl)-7-(1-(3-((2-methoxyethyl) (methyl)amino)propyl)-1H-pyrazol-4-yl)-1,5-naphthyridine-2-amine was obtained as a pale yellow solid in the same manner as in Example 0426-2 except that 2-methoxy-N-methylethanamine was used instead of the (2S,6R)-1,2,6-trimethylpiperazine trifluoroacetate used in Example 0426-2.
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.50(1H,s),8.24-8.18(3H,m),7.70(lH,d,J=9.2Hz),4.20(2H,t,J=6.9Hz),3.40(2H,t,J=5.9Hz),3.23(3H,s),3.08-3.00(1H,m),2.50-2.46(2H,m),2.33(2H,t,J=6.9Hz),2.19(3H,s),2.01-1.93(2H,m),1.33(6H,d,J=6.6Hz).
MSm/z(M+H):461.

<Example 0660>

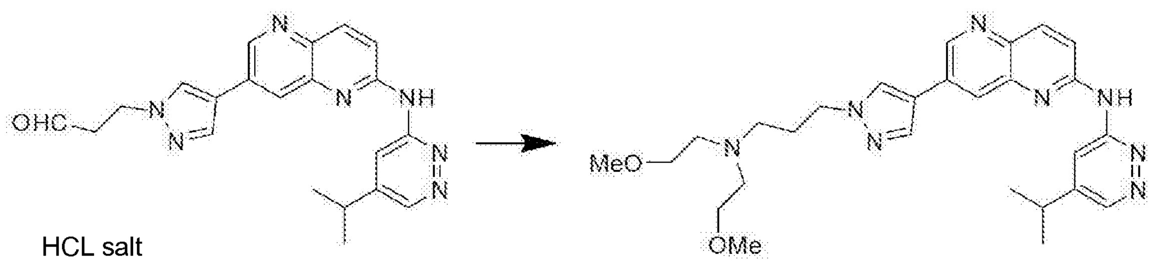

HCL salt

7-(1-(3-(Bis(2-methoxyethyl)amino)propyl)-1H-pyrazol-4-yl)-N-(5-isopropylpyridazin-3-yl)-1,5-naphthyridine-2-amine was obtained as a pale yellow solid in the same manner as in Example 0426-2 except that bis(2-methoxyethyl)amine was used instead of the (2S,6R)-1,2,6-trimethylpiperazine trifluoroacetate used in Example 0426-2.
$^1$H-NMR(DMSO-$d_6$)$\delta$:10.70(1H,s),9.05(1H,d,J=2.0Hz),8.87(1H,d,J=2.0Hz),8.73(1H,d,J=2.0Hz),8.50(1H,s),8.23-8.18(3H,m),7.69(1H,d,J=9.2Hz),4.20(2H,t,J=6.9Hz),3.38-3.30(6H,m),3.21(6H,s),3.08-2.98(1H,m),2.61(4H,t,J=5.9Hz),1.99-1.92(2H,m),1.33(6H,d,J=6.6Hz).
MSm/z(M+H):505.

<Example 0661>

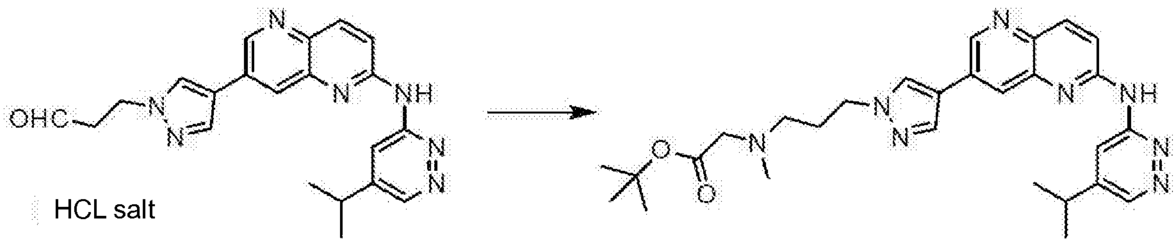


tert-Butyl 2-((3-(4-(6-((5-isopropylpyridazin-3-yl)amino)-1,5-naphthyridin-3-yl)-1H-pyrazol-1-yl)propyl)(methyl)amino)acetate was obtained as a pale yellow solid in the same manner as in Example 0426-2 except that tert-butyl 2-(methylamino)acetate was used instead of the (2S,6R)-1,2,6-trimethylpiperazine trifluoroacetate used in Example 0426-2.
$^1$H-NMR(DMSO-$d_6$)$\delta$:10.70(1H,s),9.04(1H,d,J=2.0Hz),8.87(1H,d,J=2.0Hz),8.73(1H,d,J=2.0Hz),8.51(1H,s),8.24-8.18(3H,m),7.69(1H,d,J=9.2Hz),4.20(2H,t,J=6.9Hz),3.15(2H,s),3.08-2.99(1H,m),2.47-2.43(2H,m),2.28(3H,s),2.01-1.92(2H,m),1.39(9H,s),1.33(6H,d,J=6.6Hz).
MSm/z(M+H):517.

<Example 0662>

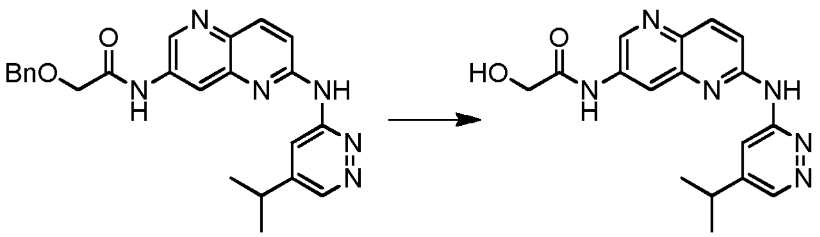

A mixture of 2-(benzyloxy)-N-(6-((5-isopropylpyridazin-3-yl)amino)-1,5-naphthyridin-3-yl)acetamide (35 mg), 10% palladium-carbon (11 mg), ammonium formate (20 mg), and methanol (5 mL) was stirred for 9 hours under reflux. The reaction mixture was cooled to room temperature, the insolubles were filtered off, and the solvent was distilled off under reduced pressure. The obtained residue was purified by silica gel column chromatography (hexane-ethyl acetate-methanol, NH silica), thereby obtaining 2-hydroxy-N-(6-((5-isopropylpyridazin-3-yl)amino)-1,5-naphthyridin-3-yl)acetamide (3 mg) as a pale yellow solid.
$^1$H-NMR(DMSO-$d_6$)$\delta$:10.70(1H,s),10.28(1H,s),9.02(1H,d,J=2.0Hz),8.84(1H,d,J=2.0Hz),8.73(1H,d,J=2.0Hz),8.57(1H,d,J=2.0Hz),8.18(1H,d,J=9.2Hz),7.66(1H,d,J=9.2Hz),5.82(1H,t,J=4.6Hz),4.10(2H,d,J=4.6Hz),3.04-2.94(1H,m),1.31(6H,d,J=7.3Hz).
MSm/z(M+H):339.

<Example 0663> (not in accordance with the present invention)

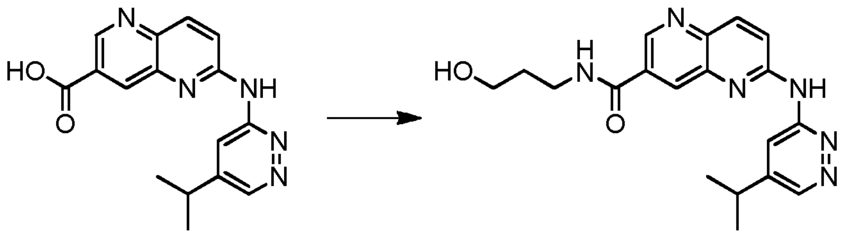

N-(3-hydroxypropyl)-6-((5-isopropylpyridazin-3-yl)amino)-1,5-naphthyridine-3-carboxamide was obtained as a pale yellow solid in the same manner as in Example 0647-1 except that 3-aminopropan-1-ol was used instead of the benzylamine used in Example 0647-1.
$^1$H-NMR(DMSO-ds)δ:10.85(1H,s),9.10(1H,d,J=2.0Hz),8.89-8.86(2H,m),8.75(1H,d,J=2.0Hz),8.49(1H,d,J=2.0Hz),8.31(1H,d,J=9.2Hz),7.83(1H,d,J=9.2Hz),4.51(1 H,t,J=5.3Hz),3.53-3.46(2H,m),3.42-3.35(2H,m),3.08-2.99(1H,m),1.78-1.69(2H,m),1.33(6H,d,J=7.3Hz).
MSm/z(M+H):367.

<Example 0664> (not in accordance with the present invention)

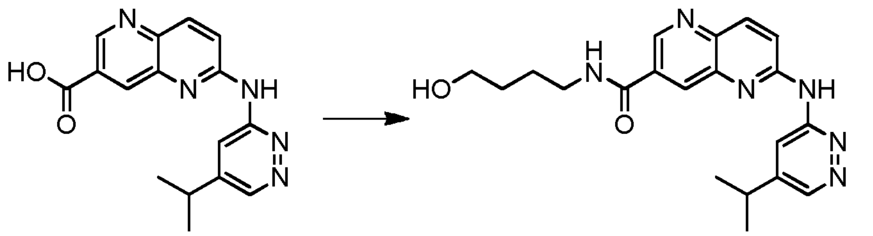

N-(4-hydroxybutyl)-6-((5-isopropylpyridazin-3-yl)amino)-1,5-naphthyridine-3-carboxamide was obtained as a pale yellow solid in the same manner as in Example 0647-1 except that 4-aminobutan-1-ol was used instead of the benzylamine used in Example 0647-1.
$^1$H-NMR(DMSO-$d_6$)δ:10.85(1H,s),9.10(1H,d,J=2.0Hz),8.91-8.87(2H,m),8.75(1H,d,J=2.0Hz),8.48(1H,d,J=2.0Hz),8.31(1H,d,J=9.2Hz),7.83(1H,d,J=9.2Hz),4.43(1 H,t,J=5.3Hz),3.49-3.39(2H,m),3.38-3.26(2H,m),3.08-2.99(1H,m),1.64-1.57(2H,m),1.54-1.45(2H,m),1.33(6H,d,J=6.6Hz).
MSm/z(M+H):381.

<Example 0665>

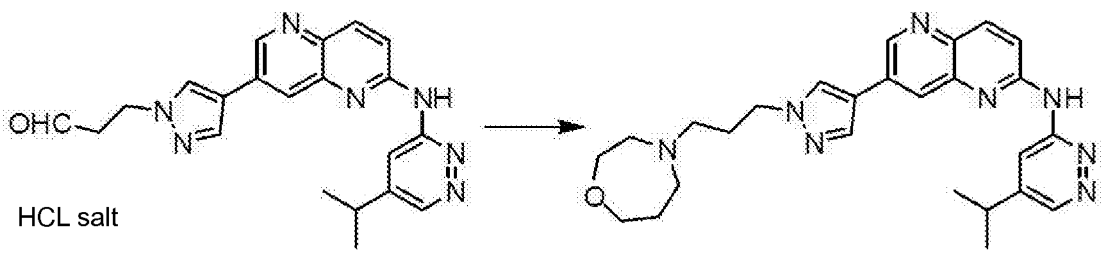

7-(1-(3-(1,4-Oxazepan-4-yl)propyl)-1H-pyrazol-4-yl)-N-(5-isopropylpyridazin-3-yl)-1,5-naphthyridine-2-amine was obtained as a pale yellow solid in the same manner as in Example 0426-2 except that 1,4-oxazepane was used instead of the (2S,6R)-1,2,6-trimethylpiperazine trifluoroacetate used in Example 0426-2.
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.50(1H,s),8.24-8.18(3H,m),7.70(1H,d,J=9.2Hz),4.22(2H,t,J=6.9Hz),3.67(2H,t,J=5.9Hz),3.63-3.59(2H,m),3.08-2.99(1H,m),2.65-2.58(4H,m),2.45(2H,t,J=6.9Hz),2.03-1.94(2H,m),1.84-1.76(2H,m),1.33(6H,d,J=7.3Hz).
MSm/z(M+H):473.

<Example 0666>

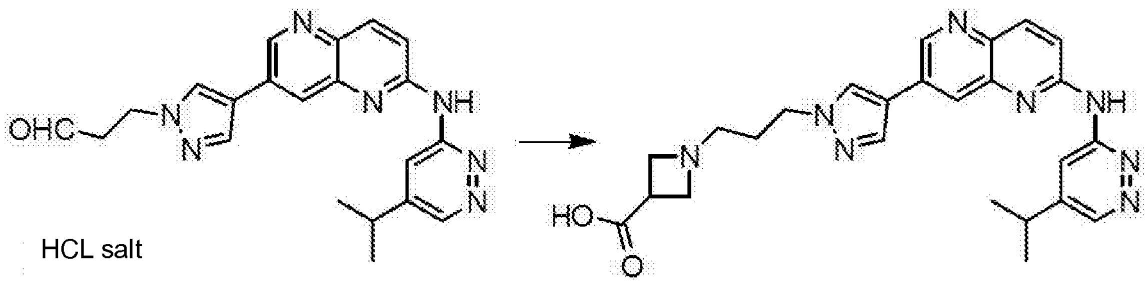


1-(3-(4-(6-((5-Isopropylpyridazin-3-yl)amino)-1,5-naphthyridin-3-yl)-1H-pyrazol-1-yl)propyl)azetidine-3-carboxylic acid was obtained as a pale yellow solid in the same manner as in Example 0426-2 except that azetidine-3-carboxylic acid was used instead of the (2S,6R)-1,2,6-trimethylpiperazine trifluoroacetate used in Example 0426-2.
$^1$H-NMR(DMSO-$d_6$)δ:10.70(1H,s),9.04(1H,d,J=2.0Hz),8.86(1H,d,J=2.0Hz),8.73(1H,d,J=2.0Hz),8.50(1H,s),8.23-8.17(3H,m),7.70(1H,d,J=9.2Hz),4.17(2H,t,J=7.3Hz),3.81-3.78(1H,m),3.05-3.01(2H,m),2.98-2.88(1H,m),2.35-2.28(2H,m),1.84-1.78(2H,m),1.33(6H,d,J=6.6Hz),0.83(2H,t,J=7.3Hz).
MSm/z(M+H):473.

<Example 0667>

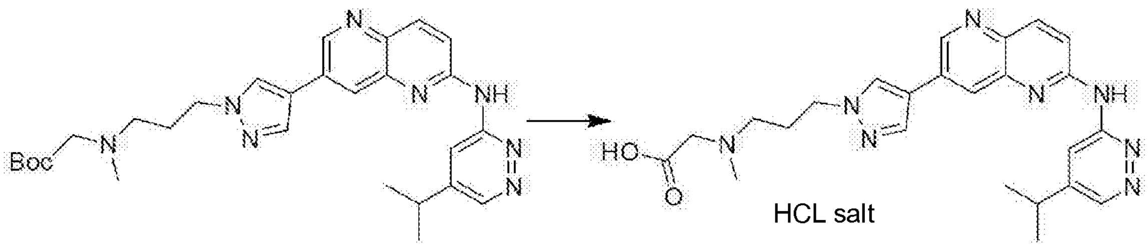


A mixture of tert-butyl 2-((3-(4-(6-((5-isopropylpyridazin-3-yl)amino)-1,5-naphthyridin-3-yl)-1H-pyrazol-1-yl)propyl)(methyl)amino)acetate (15 mg), 1,4-dioxane (2 mL), and 2 mol/L hydrochloric acid (0.5 mL) was stirred at 80°C for 1.5 hours. The reaction mixture was cooled to room temperature, and the solvent was distilled off under reduced pressure, thereby obtaining hydrochloride (12 mg) of 2-((3-(4-(6-((5-isopropylpyridazin-3-yl)amino)-1,5-naphthyridin-3-yl)-1H-pyrazol-1-yl)propyl) (methyl)amino)acetic acid as a yellow solid.
$^1$H-NMR(DMSO-$d_6$)δ:9.95(1H,brs),9.15(1H,d,J=2.0Hz),9.00(1H,d,J=2.0Hz),8.56(2H,s),8.49-8.46(1H,m),8.36(1H,d,J=8.9Hz),8.26(1H,s),7.72(1H,d,J=8.9Hz),4.30(2H,t,J=6.6Hz),4.14(2H,s),3.24-3.15(2H,m),3.15-3.08(1H,m),2.85(3H,s),2.29-2.25(2H,m),1.34(6H,d,J=6.6Hz).
MSm/z(M+H):461.

<Example 0668> (not in accordance with the present invention)

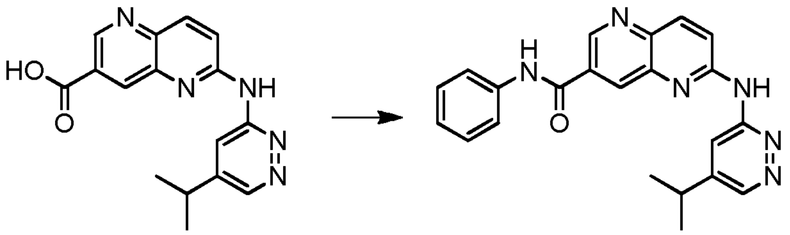

6-((5-Isopropylpyridazin-3-yl)amino)-N-phenyl-1,5-naphthyridine-3-carboxamide was obtained as a white solid in the same manner as in Example 0647-1 except that aniline was used instead of the benzylamine used in Example 0647-1.
$^1$H-NMR(DMSO-$d_6$)δ:10.89(1H,s),10.68(1H,s),9.18(1H,d,J=2.0Hz),8.90(1H,d,J=2.0Hz),8.78(1H,d,J=2.0Hz),8.64(1H,d, J=2.0Hz),8.35(1H,d,J=9.2Hz), 7.88-7.81(3H,m),7.40(2H,dd,J=7.6,7.6Hz),7.15(1H,dd,J=7.6,7.6Hz),3.10-3.00(1H,m),1.33(6H,d,J=7.3Hz).
MSm/z(M+H):385.

<Example 0669> (not in accordance with the present invention)

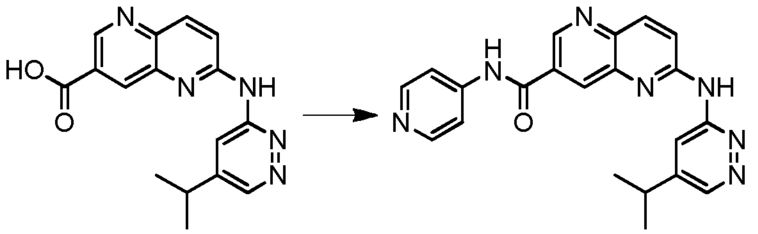

6-((5-Isopropylpyridazin-3-yl)amino)-N-(pyridin-4-yl)-1,5-naphthyridine-3-carboxamide was obtained as a white solid in the same manner as in Example 0647-1 except that 4-aminopyridine was used instead of the benzylamine used in Example 0647-1.
$^1$H-NMR(DMSO-$d_6$)δ:11.02(1H,s),10.91(1H,s),9.18(1H,d,J=2.0Hz),8.90(1H,d,J=2.0Hz),8.77(1H,d,J=2.0Hz),8.67(1H,d, J=2.0Hz),8.54(1H,d,J=1.3Hz),8.52(1H,d,J=1.3Hz),8.36(1H,d,J=9.2Hz),7.88(1H,d,J=9.2Hz),7.84-7.81(2H,m),3.09-3.00 (1H,m),1.33(6H,d,J=7.3Hz).
MSm/z(M+H):386.

<Example 0670>

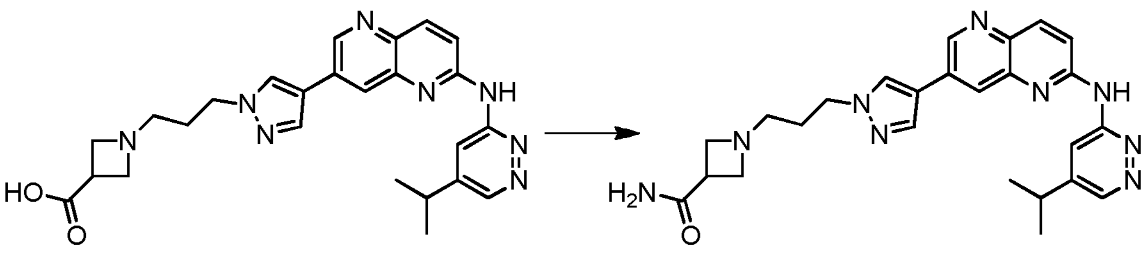

1-(3-(4-(6-((5-Isopropylpyridazin-3-yl)amino)-1,5-naphthyridin-3-yl)-1H-pyrazol-1-yl)propyl)azetidine-3-carboxamide was obtained as a pale yellow solid in the same manner as in Example 0640-3 except that 1-(3-(4-(6-((5-isopropylpyridazin-3-yl)amino)-1,5-naphthyridin-3-yl)-1H-pyrazol-1-yl)propyl)azetidine-3-carboxylic acid was used instead of the 6-((5-isopropylpyridazin-3-yl)amino)-1,5-naphthyridine-3-carboxylic acid used in Example 0640-3.
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.50(1H,s),8.24-8.17(3H,m),7.70(1H,d,J=9.2Hz),7.28(1H,s),6.84(1H,s),4.17(2H,t,J=6.9Hz),3.38-3.25(1H,m),3.10-2.95(5H,m),2.33(2H,t,J=7.3Hz),1.87-1.79(2H,m),1.33(6H,d,J=6.6Hz).
MSm/z(M+H):472.

<Example 0671>

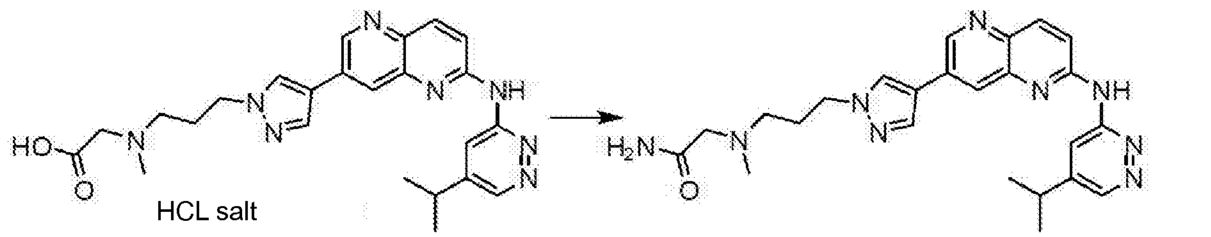


2-((3-(4-(6-((5-Isopropylpyridazin-3-yl)amino)-1,5-naphthyridin-3-yl)-1H-pyrazol-1-yl)propyl) (methyl)amino)acetamide was obtained as a pale yellow solid in the same manner as in Example 0640-3 except that hydrochloride of 2-((3-(4-(6-((5-isopropylpyridazin-3-yl)amino)-1,5-naphthyridin-3-yl)-1H-pyrazol-1-yl)propyl)(methyl)amino)acetic acid was used instead of the 6-((5-isopropylpyridazin-3-yl)amino)-1,5-naphthyridine-3-carboxylic acid used in Example 0640-3.
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.53(1H,s),8.23-8.18(3H,m),7.70(1H,d,J=9.2Hz),7.22(1H,s),7.13(1H,s),4.25(2H,t,J=6.9Hz),3.08-2.99(1H,m),2.86(2H,s),2.37(2H,t,J=6.6Hz),2.21(3H,s),2.03-1.96(2H,m),1.33(6H,d,J=7.3Hz).
MSm/z(M+H):460.

<Example 0672>

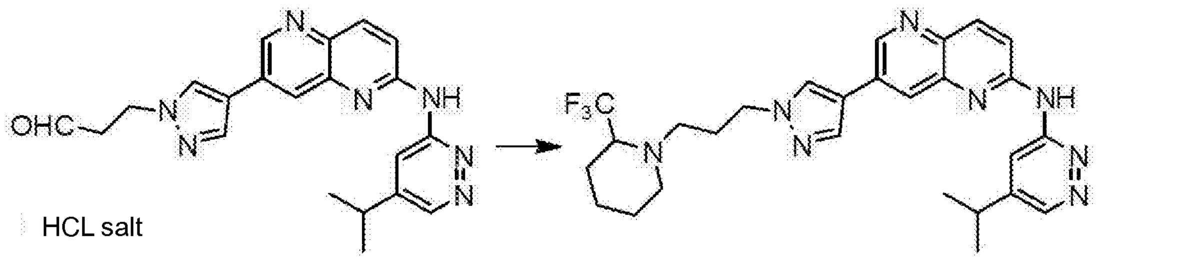


N-(5-isopropylpyridazin-3-yl)-7-(1-(3-(2-(trifluoromethyl)piperidin-1-yl)propyl)-1H-pyrazol-4-yl)-1,5-naphthyridine-2-amine was obtained as a pale yellow solid in the same manner as in Example 0426-2 except that 2-(trifluoromethyl)piperidine was used instead of the (2S,6R)-1,2,6-trimethylpiperazine trifluoroacetate used in Example 0426-2.
$^1$H-NMR(DMSO-$d_6$)δ:10.70(1H,s),9.04(1H,d,J=2.0Hz),8.87(1H,d,J=2.0Hz),8.74(1H,d,J=2.0Hz),8.50(1H,s),8.24-8.19(3H,m),7.69(1H,d,J=9.2Hz),4.20(2H,t,J=6.9Hz),3.08-2.98(lH,m),2.78-2.59(5H,m),2.02-1.96(2H,m),1.80-1.65(2H,m),1.51(4H,brs),1.33(6H,d,J=7.3Hz).
MSm/z(M+H):525.

<Example 0673>

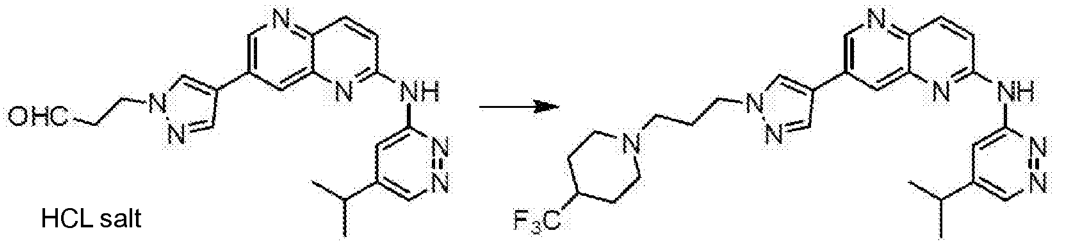


N-(5-isopropylpyridazin-3-yl)-7-(1-(3-(4-(trifluoromethyl)piperidin-1-yl)propyl)-1H-pyrazol-4-yl)-1,5-naphthyridine-2-amine was obtained as a pale yellow solid in the same manner as in Example 0426-2 except that 4-(trifluoromethyl)piperidine was used instead of the (2S,6R)-1,2,6-trimethylpiperazine trifluoroacetate used in Example 0426-2.

$^1$H-NMR(DMSO-$d_6$)δ:10.70(1H,s),9.04(1H,d,J=2.0Hz),8.87(1H,d,J=2.0Hz),8.73(1H,d,J=2.0Hz),8.50(1H,s),8.23-8.18(3H,m),7.69(1H,d,J=9.2Hz),4.20(2H,t,J=6.9Hz),3.08-2.98(1H,m),2.95-2.88(2H,m),2.32-2.22(3H,m),1.99(2H,t,J=6.6Hz),1.89(2H,t,J=10.9Hz),1.79-1.73(2H,m),1.53-1.42(2H,m),1.33(6H,d,J=6.6Hz).
MSm/z(M+H):525.

<Example 0674>

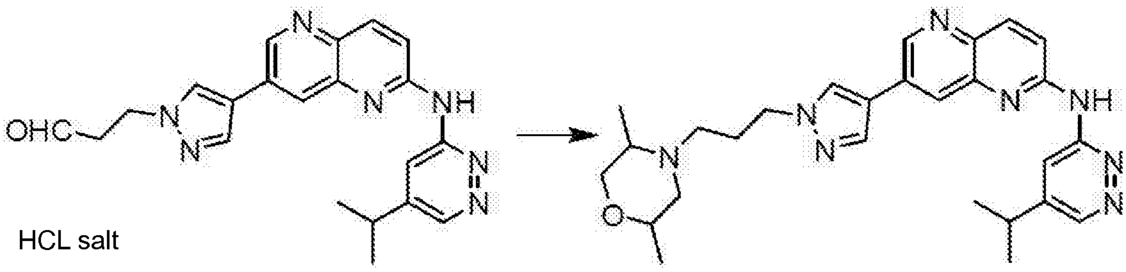


7-(1-(3-(2,5-Dimethylmorpholino)propyl)-1H-pyrazol-4-yl)-N-(5-isopropylpyridazin-3-yl)-1,5-naphthyridine-2-amine was obtained as a pale yellow solid in the same manner as in Example 0426-2 except that 2,5-dimethylmorpholine was used instead of the (2S,6R)-1,2,6-trimethylpiperazine trifluoroacetate used in Example 0426-2.
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.50(1H,s),8.23-8.18(3H,m),7.69(1H,d,J=9.2Hz),4.21(2H,t,J=6.9Hz),3.64-3.40(4H,m),3.07-2.99(1H,m),2.74-2.68(1H,m),2.43-2.31(2H,m),2.20-2.12(1H,m),2.03-1.93(2H,m),1.33(6H,d,J=7.2Hz),1.06(3H,d,J=6.6Hz),0.91(3H,d,J=6.6Hz).
MSm/z(M+H):487.

<Example 0675> (not in accordance with the present invention)

<0675-1>

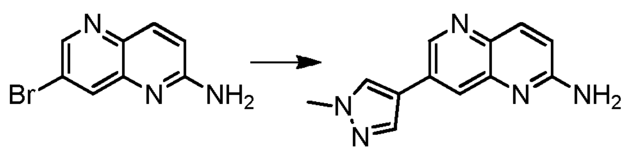

A mixture of 7-bromo-1,5-naphthyridine-2-amine (20 mg), 1-methyl-4-(4,4,5,5-tetramethyl-1,3,2-dioxaborolan-2-yl)-1H-pyrazole (154 mg), sodium carbonate (142 mg), bis(di-tert-butyl (4-dimethylaminophenyl)phosphine)dichloropalladium(II) (21 mg), 1,4-dioxane (5 mL), and water (1 mL) was stirred at 110°C for 2 hours in a nitrogen atmosphere. The reaction mixture was cooled to room temperature, and the solvent was distilled off under reduced pressure. The obtained residue was purified by silica gel column chromatography (hexane-ethyl acetate-methanol, NH silica), thereby obtaining 7-(1-methyl-1H-pyrazol-4-yl)-1,5-naphthyridine-2-amine (150 mg).
MSm/z(M+H):226.

<0675-2>

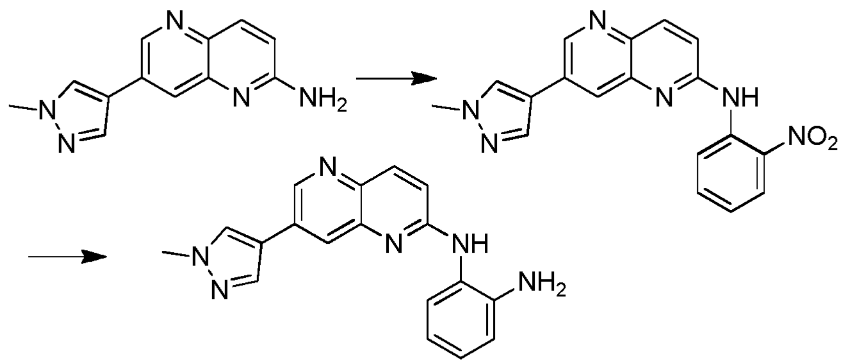

A mixture of 7-(1-methyl-1H-pyrazol-4-yl)-1,5-naphthyridine-2-amine (10 mg), 1-fluoro-2-nitrobenzene (8 μL), potassium carbonate (22 mg), and N,N-dimethylformamide (1 mL) was stirred at 90°C for 3 hours, stirred at 110°C for 3 hours, and stirred at 140°C for 2 hours. The reaction mixture was cooled to room temperature, and the solvent was distilled off under reduced pressure. The obtained residue was purified by silica gel column chromatography (hexane-ethyl acetate-methanol), thereby obtaining a solid matter.

A mixture of the obtained solid matter, sodium formate (8 mg), 10% palladium-carbon (4 mg), and methanol (3 mL) was stirred at 80°C for 1 hour. The reaction mixture was cooled to room temperature, the insolubles were filtered off, and the solvent was distilled off under reduced pressure. The obtained residue was purified by silica gel column chromatography (hexane-ethyl acetate-methanol, NH silica), thereby obtaining $N^1$-(7-(1-methyl-1H-pyrazol-4-yl)-1,5-naphthyridin-2-yl)benzene-1,2-diamine (3 mg) as a brown solid.
$^1$H-NMR(DMSO-$d_6$)δ:8.85(1H,d,J=2.0Hz),8.57(1H,s),8.41(1H,s),8.10(1H,s),8.00-7.97(2H,m),7.49(1H,d,J=6.6Hz),7.10(1H,d,J=9.2Hz),6.97-6.90(1H,m),6.79(1H,dd,J=7.2,1.5Hz),6.65-6.59(1H,m),4.93(2H,s),3.89(3H,s).
MSm/z(M+H):317.

<Example 0676>

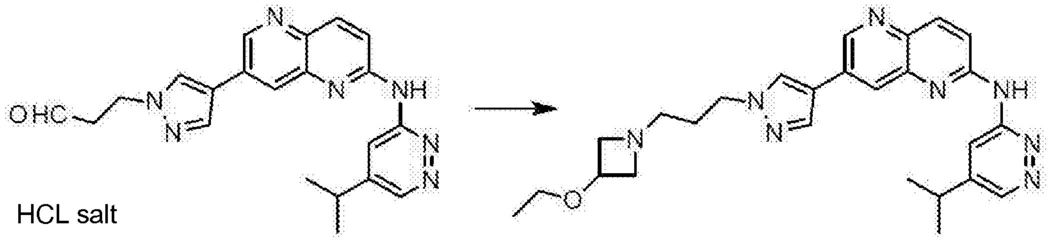


N-(5-isopropylpyridazin-3-yl)-7-(1-(3-(3-ethoxyazetidin-1-yl)propyl)-1H-pyrazol-4-yl)-1,5-naphthyridine-2-amine was obtained as a pale yellow solid in the same manner as in Example 0426-2 except that 3-ethoxyazetidine hydrochloride was used instead of the (2S,6R)-1,2,6-trimethylpiperazine trifluoroacetate used in Example 0426-2.
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.50(1H,s),8.23-8.18(3H,m),7.70(1H,d,J=9.2Hz),4.17(2H,t,J=6.9Hz),4.06-3.98(1H,m),3.54-3.48(2H,m),3.35-3.27(2H,m),3.08-3.00(1H,m),2.75-2.70(2H,m),2.38(2H,t,J=6.6Hz),1.87-1.80(2H,m),1.34(6H,d,J=7.9Hz),1.08(3H,t,J=6.9Hz).
MSm/z(M+H):473.

<Example 0677> (not in accordance with the present invention)

<0677-1>

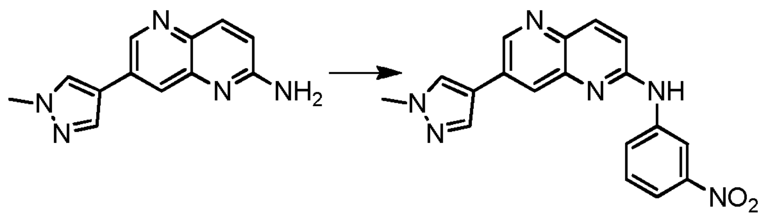

A mixture of 7-(1-methyl-1H-pyrazol-4-yl)-1,5-naphthyridine-2-amine (20 mg), 1-fluoro-3-nitrobenzene (20 mg), [(2-di-cyclohexylphosphino-3,6-dimethoxy-2',4',6'-triisopropyl-1,1'-biphenyl)-2-(2'-amino-1,1'-biphenyl)]palladium(II)methanesulfonate (BRETTPHOS-PD-G3 (product name, manufactured by Sigma-Aldrich Co. LLC.)) (5 mg), cesium carbonate (81 mg), and 1,4-dioxane (4 mL) was stirred at 110°C for 3 hours in a nitrogen atmosphere. The reaction mixture was cooled to room temperature, and the solvent was distilled off under reduced pressure. The obtained residue was purified by silica gel column chromatography (hexane-ethyl acetate-methanol), thereby obtaining 7-(1-methyl-1H-pyrazol-4-yl)-N-(3-nitrophenyl)-1,5-naphthyridine-2-amine (27 mg).
MSm/z(M+H):347.

<0677-2>

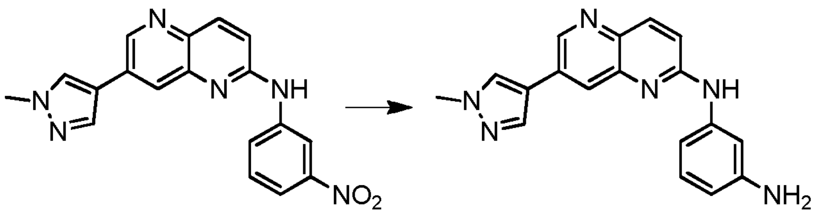

A mixture of 7-(1-methyl-1H-pyrazol-4-yl)-N-(3-nitrophenyl)-1,5-naphthyridine-2-amine (27 mg), 10% palladium-carbon (8 mg), ammonium formate (8 mg), and methanol (5 mL) was stirred for 3 hours under reflux. The reaction mixture was cooled to room temperature, the insolubles were filtered off, and the solvent was distilled off under reduced pressure, thereby obtaining $N^1$-(7-(1-methyl-1H-pyrazol-4-yl)-1,5-naphthyridin-2-yl)benzene-1,3-diamine (16 mg) as a yellow solid.
$^1$H-NMR(DMSO-$d_6$)δ:9.31(1H,s),8.88(1H,d,J=2.0Hz),8.42(1H,s),8.14-8.11(2H,m),8.00(1H,d,J=9.2Hz),7.28(1H,d,J=2.0Hz),7.20-7.14(2H,m),6.97(1H,dd,J=7.9,7.6Hz),6.23(1H,d,J=7.6Hz),5.03(2H,s),3.91(3H,s).
MSm/z(M+H):317.

<Example 0678>

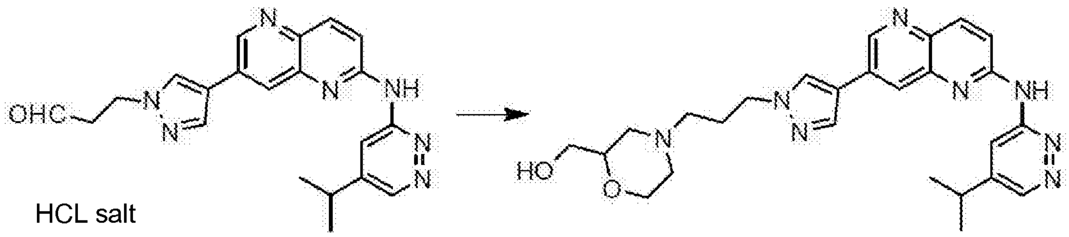


(4-(3-(4-(6-((5-Isopropylpyridazin-3-yl)amino)-1,5-naphthyridin-3-yl)-1H-pyrazol-1-yl)propyl)morpholin-2-yl)methanol was obtained as a pale yellow solid in the same manner as in Example 0426-2 except that (morpholin-2-yl)methanol hydrochloride was used instead of the (2S,6R)-1,2,6-trimethylpiperazine trifluoroacetate used in Example 0426-2.
$^1$H-NMR(DMSO-$d_6$)$\delta$:10.70(1H,s),9.04(1H,d,J=2.0Hz),8.87(1H,d,J=2.0Hz),8.73(1H,d,J=2.0Hz),8.51(1H,s),8.23-8.19(3H,m),7.70(1H,d,J=9.2Hz),4.64(1H,t,J=5.6Hz),4.22(2H,t,J=6.9Hz),3.76(1H,d,J=10.6Hz),3.54-3.37(3H,m),3.08-3.00(1H,m),2.82-2.65(3H,m),2.30(2H,t,J=6.6Hz),2.05-1.94(3H,m),1.71(1H,t,J=11.4Hz),1.33(6H,d,J=6.6Hz).
MSm/z(M+H):489.

<Example 0679>

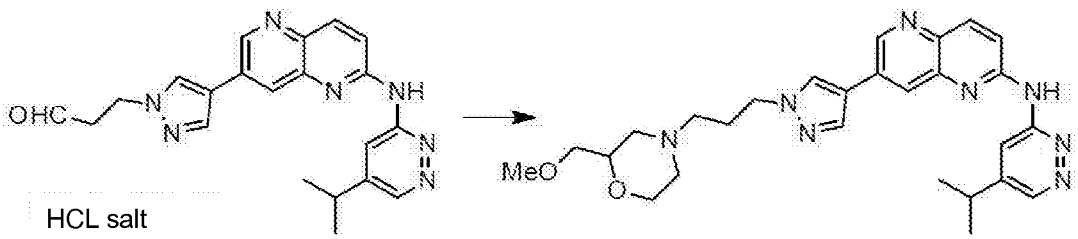


N-(5-isopropylpyridazin-3-yl)-7-(1-(3-(2-(methoxymethyl)morpholino)propyl)-1H-pyrazol-4-yl)-1,5-naphthyridine-2-amine was obtained as a pale yellow solid in the same manner as in Example 0426-2 except that 2-(methoxymethyl)morpholine hydrochloride was used instead of the (2S,6R)-1,2,6-trimethylpiperazine trifluoroacetate used in Example 0426-2.
$^1$H-NMR(DMSO-$d_6$)$\delta$:10.70(1H,s),9.04(1H,d,J=2.0Hz),8.87(1H,d,J=2.0Hz),8.73(1H,d,J=2.0Hz),8.51(1H,s),8.23-8.18(3H,m),7.70(1H,d,J=9.2Hz),4.21(2H,t,J=6.9Hz),3.76(1H,d,J=10.6Hz),3.59-3.46(2H,m),3.29-3.24(2H,m),3.22(3H,s),3.08-2.99(1H,m),2.76-2.64(2H,m),2.29(2H,t,J=6.9Hz),2.05-1.93(3H,m),1.75(1H,t,J=10.6Hz),1.33(6H,d,J=6.6Hz).
MSm/z(M+H):503.

<Example 0680>

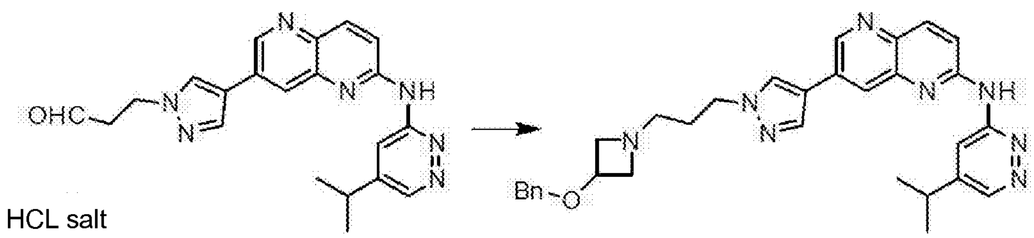


7-(1-(3-(3-(Benzyloxy)azetidin-1-yl)propyl)-1H-pyrazol-4-yl)-N-(5-isopropylpyridazin-3-yl)-1,5-naphthyridine-2-amine was obtained as a pale yellow solid in the same manner as in Example 0426-2 except that 3-(benzyloxy)azetidine hydrochloride was used instead of the (2S,6R)-1,2,6-trimethylpiperazine trifluoroacetate used in Example 0426-2.

$^1$H-NMR(DMSO-$d_6$)δ:10.70(1H,s),9.04(1H,d,J=2.0Hz),8.87(1H,d,J=2.0Hz),8.73(1H,s),8.49(1H,s),8.23-8.17(3H,m), 7.70(1H,d,J=9.2Hz),
7.35-7.27(5H,m),4.38(2H,s),4.31-4.11(3H,m),3.52-3.42(2H,m),3.08-2.99(1H,m),2.76(2H,t,J=6.9Hz),2.39(2H,t,J=6.6Hz ),1.86-1.80(2H,m),1.33(6H,d,J=6.6Hz).
MSm/z(M+H):535.

<Example 0681>

<0681-1>

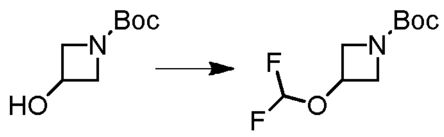

60% sodium hydride (42 mg) was added to a mixture of tert-butyl 3-hydroxyazetidine-1-carboxylate (150 mg), sodium 2-chloro-2,2-difluoroacetate (200 mg), and N,N-dimethylformamide (2 mL), followed by stirring at room temperature for 2 hours, stirring at 60°C for 1 hour, and stirring at 80°C for 4 hours. After the reaction mixture was cooled to room temperature, water and ethyl acetate were added thereto, the organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate-methanol), thereby obtaining tert-butyl 3-(difluoromethoxy)azetidine-1-carboxylate (24 mg).
$^1$H-NMR(DMSO-$d_6$)δ:5.20-5.17(1H,m),4.19-4.16(2H,m),3.80-3.76(2H,m),1.39(9H,s).

<0681-2>

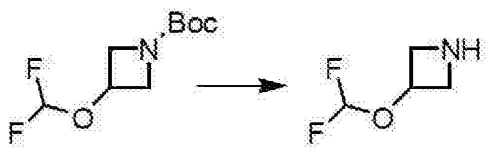

HCL salt

3-(Difluoromethoxy)azetidine hydrochloride was obtained in the same manner as in Example 0667-1 except that tert-butyl 3-(difluoromethoxy)azetidine-1-carboxylate was used instead of the tert-butyl 2-((3-(4-(6-((5-isopropylpyridazin-3-yl)amino)-1,5-naphthyridin-3-yl)-1H-pyrazol-1-yl)propyl) (methyl)amino)acetate used in Example 0667.
$^1$H-NMR(DMSO-$d_6$)δ:5.02-5.00(1H,m),4.52-4.49(2H,m),4.27-4.23(2H,m).

<0681-3>

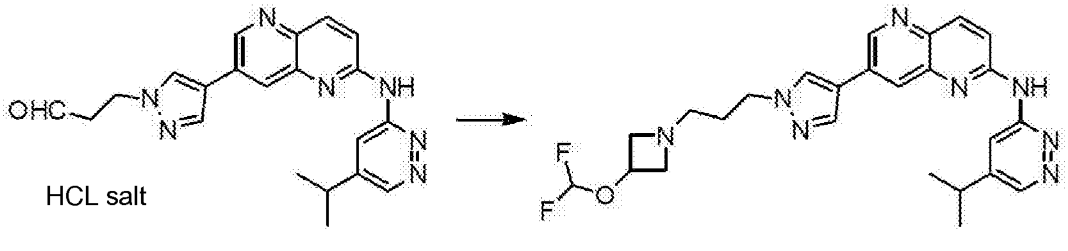


7-(1-(3-(3-(Difluoromethoxy)azetidin-1-yl)propyl)-1H-pyrazol-4-yl)-N-(5-isopropylpyridazin-3-yl)-1,5-naphthyridine-2-amine was obtained as a pale yellow solid in the same manner as in Example 0426-2 except that 3-(difluoromethoxy)azetidine hydrochloride was used instead of the (2S,6R)-1,2,6-trimethylpiperazine trifluoroacetate used in Example 0426-2.
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.50(1H,s),8.23-8.18(3H,m),7.70(1H,d,J=9.2Hz),6.67(1H,t,J=75.3Hz),4.72-4.65(1H,m),4.18(2H,t,J=6.9Hz),3.61-3.55(2H,m),3.08-2.98(1H,m),2.97-2.91(2H,m),2.42(2H,t,J=6.9Hz),1.88-1.81(2H,m), 1.33(6H,d,J=6.6Hz).
MSm/z(M+H):495.

<Example 0682>

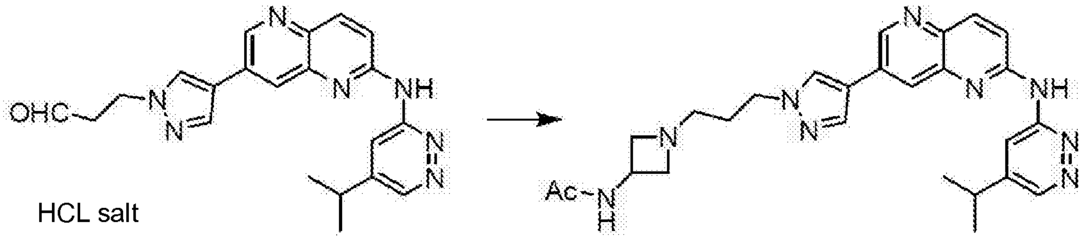


N-(1-(3-(4-(6-((5-isopropylpyridazin-3-yl)amino)-1,5-naphthyridin-3-yl)-1H-pyrazol-1-yl)propyl)azetidin-3-yl)acetamide was obtained as a pale yellow solid in the same manner as in Example 0426-2 except that N-(azetidin-3-yl)acetamide hydrochloride was used instead of the (2S,6R)-1,2,6-trimethylpiperazine trifluoroacetate used in Example 0426-2.
$^1$H-NMR(DMSO-$d_6$)δ:10.70(1H,s),9.04(1H,d,J=2.0Hz),8.87(1H,d,J=2.0Hz),8.73(1H,brs),8.50(1H,s),8.27-8.17(3H,m),7.69(1H,d,J=9.2Hz),4.28-4.16(3H,m),3.69-3.59(1H,m),3.49(2H,t,J=7.3Hz),3.08-2.99(1H,m),2.76-2.72(2H,m),2.36(2H,t,J=6.9Hz),1.86-1.81(2H,m),1.77(3H,s),1.33(6H,d,J=7.2Hz).
MSm/z(M+H):486.

<Example 0683>

<0683-1>

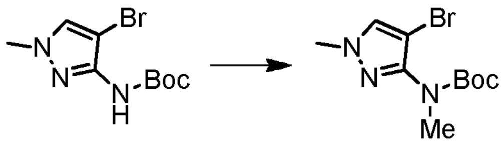

60% sodium hydride (21 mg) was added to a mixture of tert-butyl (4-bromo-1-methyl-1H-pyrazol-3-yl)carbamate (130 mg), tetrahydrofuran (10 mL), and iodomethane (35 μL), followed by stirring at room temperature for 3 hours. After water and ethyl acetate were added to the reaction mixture, the organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate-methanol), thereby obtaining tert-butyl (4-bromo-1-methyl-1H-pyrazol-3-yl) (methyl)carbamate (133 mg).
$^1$H-NMR(DMSO-$d_6$)δ:7.88(1H,s),3.78(3H,s),3.04(3H,s),1.37(9H,s).

<0683-2>

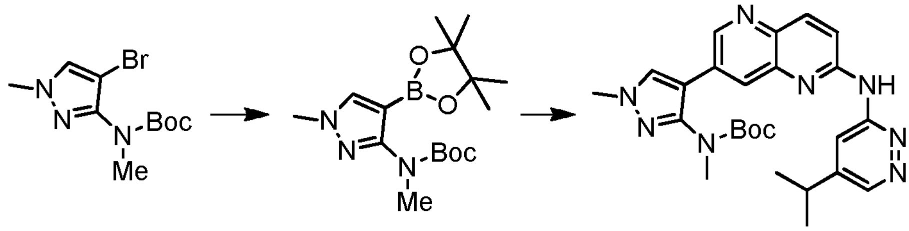

A mixture of tert-butyl (4-bromo-1-methyl-1H-pyrazol-3-yl) (methyl)carbamate (133 mg), bis(pinacolato)diboron (140 mg), a [1,1'-bis(diphenylphosphino)ferrocene]palladium(II) dichloride-dichloromethane adduct (41 mg), potassium acetate (90 mg), and 1,4-dioxane (5 mL) was stirred at 80°C for 24 hours in a nitrogen atmosphere. The reaction mixture was cooled to room temperature, the insolubles were filtered off, and the solvent was distilled off under reduced pressure, thereby obtaining residue.

A mixture of the obtained residue, 7-bromo-N-(5-isopropylpyridazin-3-yl)-1,5-naphthyridine-2-amine (158 mg), sodium carbonate (122 mg), bis(di-tert-butyl (4-dimethylaminophenyl)phosphine)dichloropalladium(II) (14 mg), 1,4-dioxane (5 mL), and water (1 mL) was stirred at 110°C for 6 hours in a nitrogen atmosphere. The reaction mixture was cooled to room temperature, water was added thereto, and the solid matter was collected by filtration. Chloroform and methanol were added to the obtained solid matter, the insolubles were filtered off, and the solvent was distilled off under reduced pressure. The obtained residue was purified sequentially by silica gel column chromatography (hexane-ethyl acetate-methanol), preparative thin layer silica gel column chromatography (chloroform-methanol), and preparative thin layer silica gel column chromatography (chloroform-methanol, NH silica), thereby obtaining tert-butyl (4-(6-((5-isopropylpyridazin-3-yl)amino)-1,5-naphthyridin-3-yl)-1-methyl-1H-pyrazol-3-yl) (methyl)carbamate (3 mg).
MSm/z(M+H):475.

<0683-3>

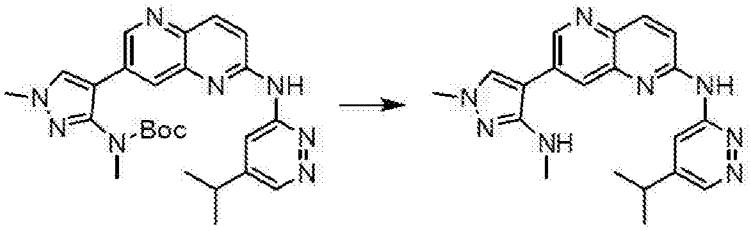

HCL salt

N-(5-isopropylpyridazin-3-yl)-7-(1-methyl-3-(methylamino)-1H-pyrazol-4-yl)-1,5-naphthyridine-2-amine hydrochloride was obtained in the same manner as in Example 0667-1 except that tert-butyl (4-(6-((5-isopropylpyridazin-3-yl)amino)-1,5-naphthyridin-3-yl)-1-methyl-1H-pyrazol-3-yl) (methyl)carbamate was used instead of the tert-butyl 2-((3-(4-(6-((5-isopropylpyridazin-3-yl)amino)-1,5-naphthyridin-3-yl)-1H-pyrazol-1-yl)propyl) (methyl)amino)acetate used in Example 0667-1.
$^1$H-NMR(DMSO-$d_6$)δ:9.07-9.04(2H,m),8.60(1H,s),8.44(1H,d,J=9.2Hz),8.30(1H,brs),8.18(1H,s),7.72(1H,d,J=9.2Hz),3.17(3H,s),2.81(3H,s),2.76-2.71(1H,m),1.33(6H,d,J=7.3Hz).
MSm/z(M+H):375.

<Example 0684>

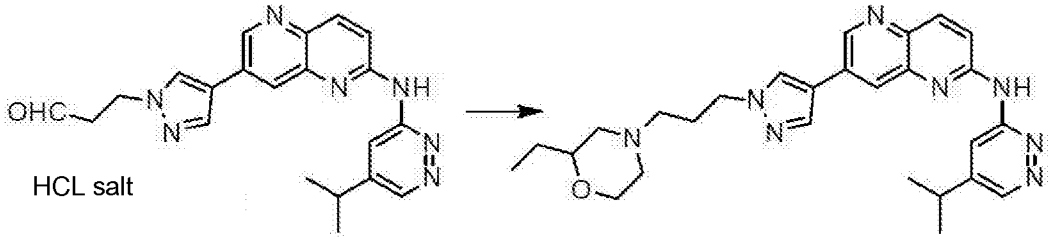


7-(1-(3-(2-Ethylmorpholino)propyl)-1H-pyrazol-4-yl)-N-(5-isopropylpyridazin-3-yl)-1,5-naphthyridine-2-amine was obtained as a pale yellow solid in the same manner as in Example 0426-2 except that 2-ethylmorpholine hydrochloride was used instead of the (2S,6R)-1,2,6-trimethylpiperazine trifluoroacetate used in Example 0426-2.
$^1$H-NMR(DMSO-$d_6$)$\delta$:10.70(1H,s),9.04(1H,d,J=2.0Hz),8.87(1H,d,J=2.0Hz),8.73(1H,d,J=2.0Hz),8.51(1H,s),8.23-8.18(3H,m),7.69(1H,d, J=9.2Hz),4.21(2H,t,J=6.9Hz),3.78-3.72(1H,m),3.51-3.45(2H,m),3.08-2.99(1H,m),2.74-2.63(3H,m),2.28(2H,t,J=6.9Hz), 2.03-1.90(3H,m),1.65(1H,t,J=10.2Hz),1.40-1.32(7H,m),0.85(3H,t,J=7.6Hz).
MSm/z(M+H):487.

<Example 0685>

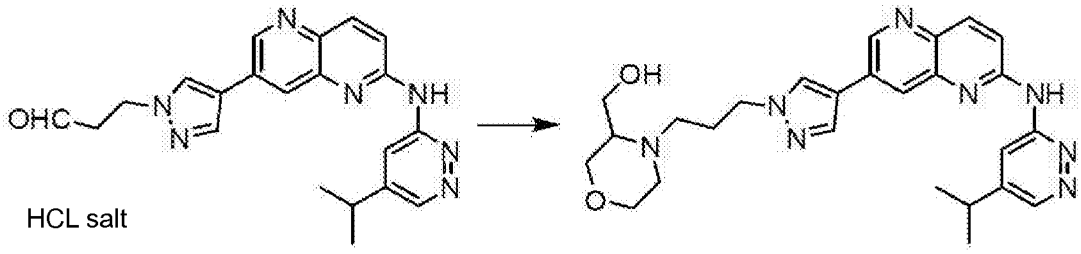


(4-(3-(4-(6-((5-isopropylpyridazin-3-yl)amino)-1,5-naphthyridin-3-yl)-1H-pyrazol-1-yl)propyl)morpholin-3-yl)methanol was obtained as a pale yellow solid in the same manner as in Example 0426-2 except that (morpholin-3-yl)methanol was used instead of the (2S,6R)-1,2,6-trimethylpiperazine trifluoroacetate used in Example 0426-2.
$^1$H-NMR(DMSO-$d_6$)$\delta$:10.70(1H,s),9.05(1H,d,J=2.0Hz),8.87(1H,d,J=2.0Hz),8.73(1H,d,J=2.0Hz),8.52(1H,s),8.23-8.19(3H,m),7.70(1H,d, J=9.2Hz),4.54-4.50(1H,m),4.21(2H,t,J=6.6Hz),3.73-3.46(4H,m),3.08-3.00(1H,m),2.77-2.70(3H,m),2.31-2.15(4H,m),2.02-1.95(2H,m),1.33(6H,d,J=6.6Hz).
MSm/z(M+H):489.

<Example 0686>

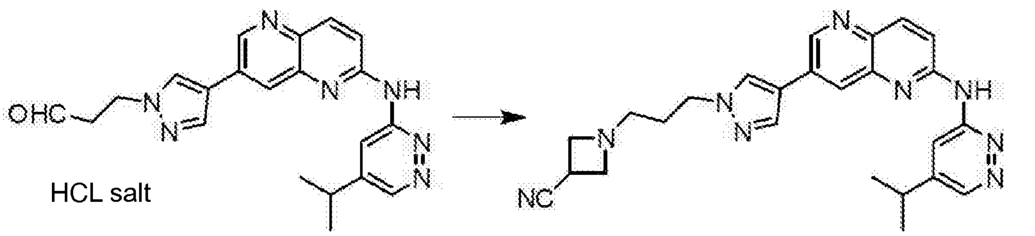


1-(3-(4-(6-((5-Isopropylpyridazin-3-yl)amino)-1,5-naphthyridin-3-yl)-1H-pyrazol-1-yl)propyl)azetidine-3-carbonitrile was obtained as a pale yellow solid in the same manner as in Example 0426-2 except that azetidine-3-carbonitrile hydrochloride was used instead of the (2S,6R)-1,2,6-trimethylpiperazine trifluoroacetate used in Example 0426-2.
$^1$H-NMR(DMSO-

d$_6$)δ:10.70(1H,s),9.04(1H,d,J=2.0Hz),8.87(1H,d,J=2.0Hz),8.73(1H,d,J=2.0Hz),8.50(1H,s),8.23-8.18(3H,m),7.70(1H,d, J=9.2Hz),4.18(2H,t,J=6.9Hz),3.46-3.30(3H,m),3.28-3.22(2H,m),3.09-2.99(1H,m),2.39(2H,t,J=6.9Hz),1.88-1.78(2H,m), 1.33(6H,d,J=7.2Hz).
MSm/z(M+H):454.

<Example 0687>

<0687-1>

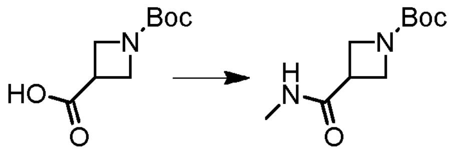

tert-Butyl 3-(methylcarbamoyl)azetidine-1-carboxylate was obtained in the same manner as in Example 0643-1 except that 1-(tert-butoxycarbonyl)azetidine-3-carboxylic acid was used instead of the 6-((5-isopropylpyridazin-3-yl)amino)-1,5-naphthyridine-3-carboxylic acid used in Example 0642.
$^1$H-NMR(DMSO-d$_6$)δ:3.88-3.84(4H,m),3.23-3.19(1H,m),2.59(3H,d,J=4.6Hz), 1.37(9H,s).

<0687-2>

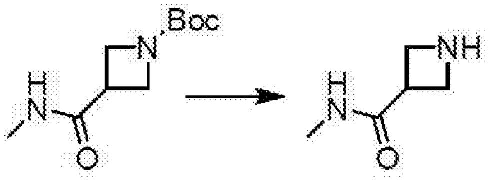

HCL salt

A mixture of tert-butyl 3-(methylcarbamoyl)azetidine-1-carboxylate (155 mg), 1,4-dioxane (2 mL), and a 4.0 mol/L hydrogen chloride/1,4-dioxane solution (1 mL) was stirred at room temperature for 15 hours. The solvent was distilled off under reduced pressure, thereby obtaining N-methylazetidine-3-carboxamide hydrochloride (127 mg).
$^1$H-NMR(DMSO-d$_6$)δ:4.00-3.96(4H,m),3.10-3.07(1H,m),2.63(3H,d,J=2.6Hz).

<0687-3>

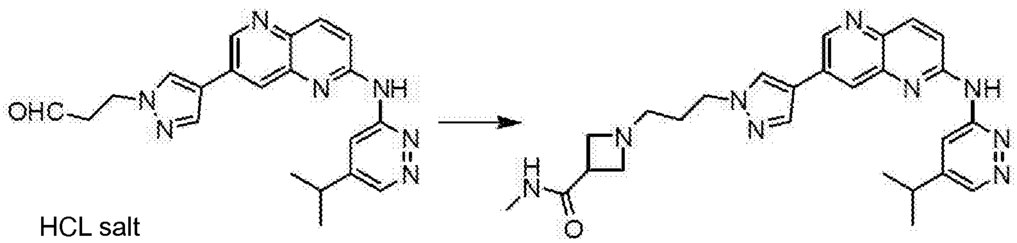

HCL salt

1-(3-(4-(6-((5-Isopropylpyridazin-3-yl)amino)-1,5-naphthyridin-3-yl)-1H-pyrazol-1-yl)propyl)-N-methylazetidine-3-carboxamide was obtained as a pale yellow solid in the same manner as in Example 0426-2 except that N-methylazetidine-3-carboxamide hydrochloride was used instead of the (2S,6R)-1,2,6-trimethylpiperazine trifluoroacetate used in Example 0426-2.
$^1$H-NMR(DMSO-

$d_6$)δ:10.70(1H,s),9.04(1H,d,J=2.0Hz),8.87(1H,d,J=2.0Hz),8.73(1H,d,J=2.0Hz),8.50(1H,s),8.38(1H,brs ),8.23-8.19(2H, m),7.70(1H,d,J=8.6Hz),4.23(2H,t,J=6.9Hz),3.54-3.26(5H,m),3.09-3.00(1H,m),2.67(3H,d,J=4.6Hz),2.53-2.45(2H,m),2.00-1.94(2H,m),1.33(6H,d,J=7.3Hz).
MSm/z(M+H):486.

<Example 0688>

<0688-1>

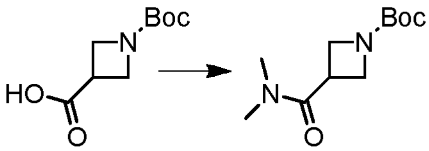

tert-Butyl 3-(dimethylcarbamoyl)azetidine-1-carboxylate was obtained in the same manner as in Example 0687-1 except that dimethylamine hydrochloride was used instead of the methylamine hydrochloride used in Example 0687-1.
$^1$H-NMR(DMSO-$d_6$)δ:3.95-3.91(4H,m),3.65-3.60(1H,m),2.82(6H,d,J=1.3Hz),1.37(9H,s).

<0688-2>

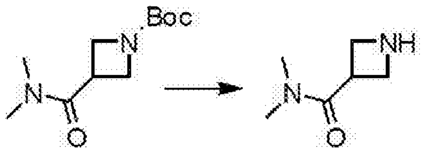

HCL salt

N,N-dimethylazetidine-3-carboxamide hydrochloride was obtained in the same manner as in Example 0687-2 except that tert-butyl 3-(dimethylcarbamoyl)azetidine-1-carboxylate was used instead of the tert-butyl 3-(methylcarbamoyl)azetidine-1-carboxylate used in Example 0687-2.
$^1$H-NMR(DMSO-$d_6$)δ:4.06-4.04(4H,m),3.90-3.80(1H,m),2.84(6H,d,J=8.6Hz).

<0688-3>

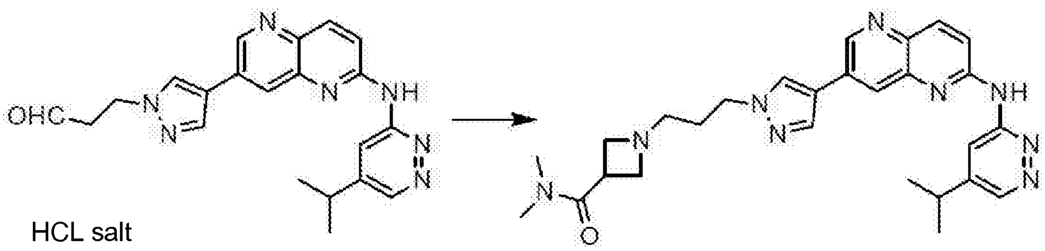

HCL salt

1-(3-(4-(6-((5-Isopropylpyridazin-3-yl)amino)-1,5-naphthyridin-3-yl)-1H-pyrazol-1-yl)propyl)-N,N-dimethylazetidine-3-carboxamide was obtained as a pale yellow solid in the same manner as in Example 0426-2 except that N,N-dimethylazetidine-3-carboxamide hydrochloride was used instead of the (2S,6R)-1,2,6-trimethylpiperazine trifluoroacetate used in Example 0426-2.
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.50(1H,s),8.23-8.18(3H,m),7.70(1H,d,J=8.6Hz),4.18(2H,t,J=6.9Hz),3.49-3.43(2H,m),3.28-3.16(1H,m),3.07 - 2.98(3H,m),2.82(3H,s),2.79(3H,s),2.33(2H,t,J=6.9Hz),1.85-1.77(2H,m),1.33(6H,d,J=6.6Hz). MSm/z(M+H):500.

<Example 0689>

<0689-1>

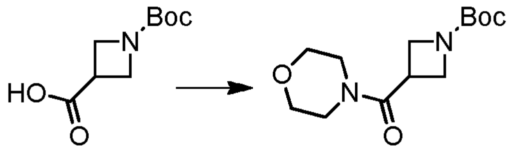

tert-Butyl 3-((morpholin-4-yl)carbonyl)azetidine-1-carboxylate was obtained in the same manner as in Example 0687-1 except that morpholine was used instead of the methylamine hydrochloride used in Example 0687-1. $^1$H-NMR(DMSO-$d_6$)δ:3.98-3.95(4H,m),3.65-3.61(1H,m),3.55-3.53(4H,m),3.45(2H,t,J=4.6Hz),3.25(2H,t,J=4.6Hz),1.37(9H,s).

<0689-2>

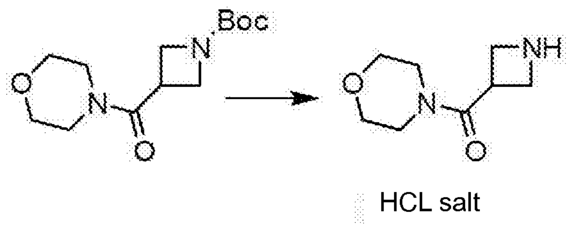

(Azetidin-3-yl) (morpholino)methanone hydrochloride was obtained in the same manner as in Example 0687-2 except that tert-butyl 3-((morpholin-4-yl)carbonyl)azetidine-1-carboxylate was used instead of the tert-butyl 3-(methylcarbamoyl)azetidine-1-carboxylate used in Example 0687-2. $^1$H-NMR(DMSO-$d_6$)δ:4.11-4.02(4H,m),3.95-3.40(4H,m),3.25-3.24(5H,m).

<0689-3>

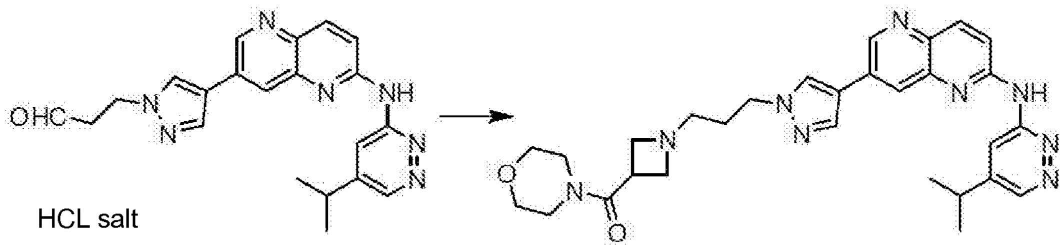

(1-(3-(4-(6-((5-Isopropylpyridazin-3-yl)amino)-1,5-naphthyridin-3-yl)-1H-pyrazol-1-yl)propyl)azetidin-3-yl) (morpholino)methanone was obtained as a pale yellow solid in the same manner as in Example 0426-2 except that (azetidin-3-yl) (morpholino)methanone hydrochloride was used instead of the (2S,6R)-1,2,6-trimethylpiperazine trifluoroacetate used in Example 0426-2. $^1$H-NMR(DMSO-$d_6$)δ:10.70(1H,s),9.04(1H,d,J=2.0Hz),8.87(1H,d,J=2.0Hz),8.73(1H,d,J=2.0Hz),8.50(1H,s),8.23-8.18(3H,m),7.70(1H,d,J=9.2Hz),4.17(2H,t,J=6.9Hz),3.52-3.49(4H,m),3.44-3.41(4H,m),3.31-3.24(3H,m),3.13-2.99(3H,m),2.34(2H,t,J=6.6Hz), 1.87-1.79(2H,m),1.33(6H,d,J=6.6Hz).
MSm/z(M+H):542.

<Example 0690>

<0690-1>

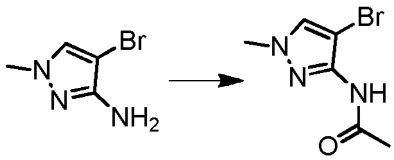

A mixture of 4-bromo-1-methyl-1H-pyrazole-3-amine (150 mg), pyridine (2 mL), acetic anhydride (241 μL), and N,N-dimethylpyridine-4-amine (11 mg) was stirred at room temperature for 3 hours. The solvent was distilled off under reduced pressure, and methanol (3 mL) and a 20%sodium hydroxide aqueous solution (1 mL) were added to the obtained residue, followed by stirring at room temperature for 3 hours. After water and ethyl acetate were added to the reaction mixture, the organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate-methanol), thereby obtaining N-(4-bromo-1-methyl-1H-pyrazol-3-yl)acetamide (79 mg).
MSm/z(M+H):218.

<0690-2>

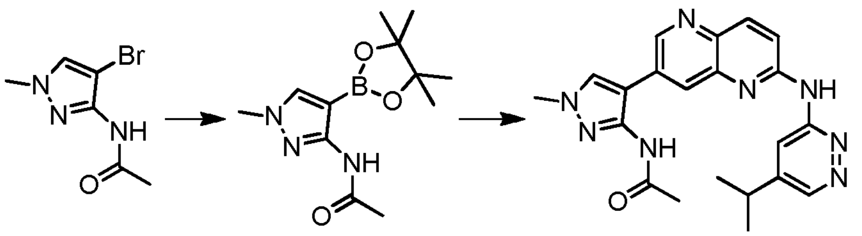

N-(4-(6-((5-isopropylpyridazin-3-yl)amino)-1,5-naphthyridin-3-yl)-1-methyl-1H-pyrazol-3-yl)acetamide was obtained as a yellow solid in the same manner as in Example 0683-2 except that N-(4-bromo-1-methyl-1H-pyrazol-3-yl)acetamide was used instead of the tert-butyl (4-bromo-1-methyl-1H-pyrazol-3-yl) (methyl)carbamate used in Example 0683-2.
$^1$H-NMR(DMSO-$d_6$)δ:10.73(1H,s),9.92(1H,s),8.86-8.84(2H,m),8.76(1H,s),8.34-8.32(1H,m),8.21(1H,d,J=9.2Hz),8.05(1H,s),7.70(1H,d,J =9.2Hz),3.87(3H,s),3.04-2.95(1H,m),2.04(3H,s),1.32(6H,d,J=6.6Hz).
MSm/z(M+H):403.

<Example 0691>

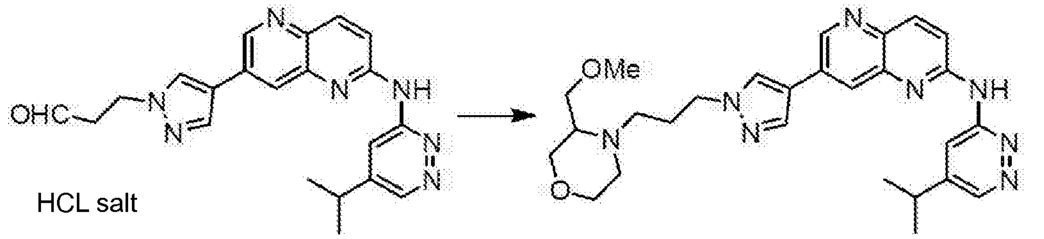


N-(5-isopropylpyridazin-3-yl)-7-(1-(3-(3-(methoxymethyl)morpholino)propyl)-1H-pyrazol-4-yl)-1,5-naphthyridine-2-amine was obtained as a pale yellow solid in the same manner as in Example 0426-2 except that 3-(methoxymethyl)morpholine hydrochloride was used instead of the (2S,6R)-1,2,6-trimethylpiperazine trifluoroacetate used in Example 0426-2.
$^1$H-NMR(DMSO-$d_6$)$\delta$:10.70(1H,s),9.05(1H,d,J=2.0Hz),8.87(1H,d,J=2.0Hz),8.73(1H,d,J=2.0Hz),8.52(1H,s),8.23-8.19(3H,m),7.69(1H,d,J=9.2Hz),4.21(2H,t,J=6.6Hz),3.69-3.62(2H,m),3.52-3.20(3H,m),3.14(3H,s),3.08-2.98(1H,m),2.74-2.65(2H,m),2.58-2.48(2H,m),2.28-2.18(2H,m),2.03-1.96(2H,m),1.33(6H,d,J=6.6Hz).
MSm/z(M+H):503.

<Example 0692>

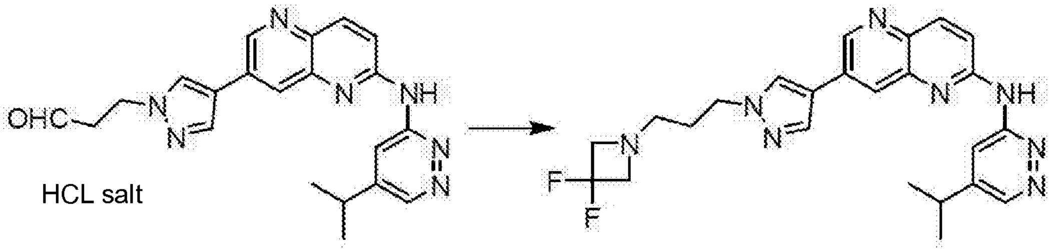


7-(1-(3-(3,3-Difluoroazetidin-1-yl)propyl)-1H-pyrazol-4-yl)-N-(5-isopropylpyridazin-3-yl)-1,5-naphthyridine-2-amine was obtained as a pale yellow solid in the same manner as in Example 0426-2 except that 3,3-difluoroazetidine hydrochloride was used instead of the (2S,6R)-1,2,6-trimethylpiperazine trifluoroacetate used in Example 0426-2.
$^1$H-NMR(DMSO-$d_6$)$\delta$:10.69(1H,s),9.04(1H,d,J=2.0Hz),8.87(1H,d,J=2.0Hz),8.73(1H,d,J=2.0Hz),8.51(1H,s),8.23-8.19(3H,m),7.70(1H,d,J=9.2Hz),4.21(2H,t,J=6.9Hz),3.61-3.20(4H,m),3.09-3.00(1H,m),2.58-2.48(2H,m),1.93-1.85(2H,m),1.33(6H,d,J=7.3Hz).
MSm/z(M+H):465.

<Example 0693>

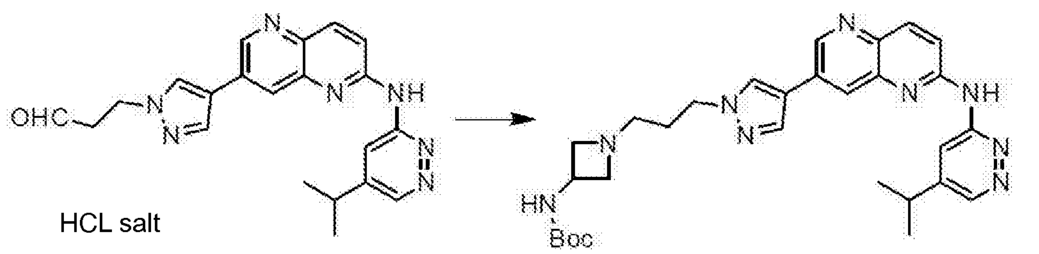


tert-Butyl (1-(3-(4-(6-((5-isopropylpyridazin-3-yl)amino)-1,5-naphthyridin-3-yl)-1H-pyrazol-1-yl)propyl)azetidin-3-yl)carbamate was obtained as a pale yellow solid in the same manner as in Example 0426-2 except that tert-butyl (azetidin-3-yl)carbamate was used instead of the (2S,6R)-1,2,6-trimethylpiperazine trifluoroacetate used in Example 0426-2.

$^1$H-NMR(DMSO-$d_6$)6:10.69(1H,s),9.04(1H,d,J=2.0Hz),8.86(1H,d,J=2.0Hz),8.73(1H,d,J=2.0Hz),8.49(1H,s),8.30(1H,s), 8.23-8.18(2H,m),7.70(1H,d,J=9.2Hz),7.29(1H,d,J=7.9Hz),4.17(2H,t,J=6.9Hz),3.72-3.18(2H,m),3.08-2.99(1H,m),2.75-2.69(2H,m),2.58-2.48(2H,m),2.34(1H,t,J=6.9Hz),1.84-1.79(2H,m),1.36(9H,s),1.33(6H,d,J=7.3Hz).
MSm/z(M+H):544.

<Example 0694>

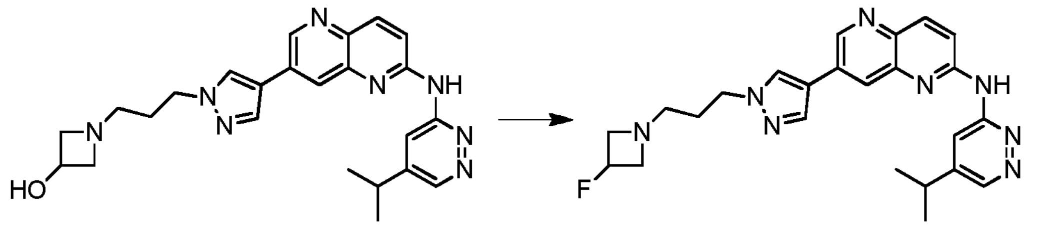

7-(1-(3-(3-Fluoroazetidin-1-yl)propyl)-1H-pyrazol-4-yl)-N-(5-isopropylpyridazin-3-yl)-1,5-naphthyridine-2-amine was obtained in the same manner as in Example 0626-3 except that 1-(3-(4-(6-((5-isopropylpyridazin-3-yl)amino)-1,5-naphthyridin-3-yl)-1H-pyrazol-1-yl)propyl)azetidin-3-ol was used instead of the 4-(6-chloro-1,5-naphthyridin-3-yl)-1-methyl-1H-pyrazol-3-yl)methanol used in Example 0626-3.
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.50(1H,s),8.23-8.19(2H,m),7.70(1H,d, J=9.2Hz),5.27-5.02(1H,m),4.18(2H,t,J=6.9Hz),3.64-3.25(2H,m),3.12-2.99(3H,m),2.46-2.43(2H,m),1.90-1.82(2H,m),1.33(6H,d,J=6.9Hz).
MSm/z(M+H):447.

<Example 0695>

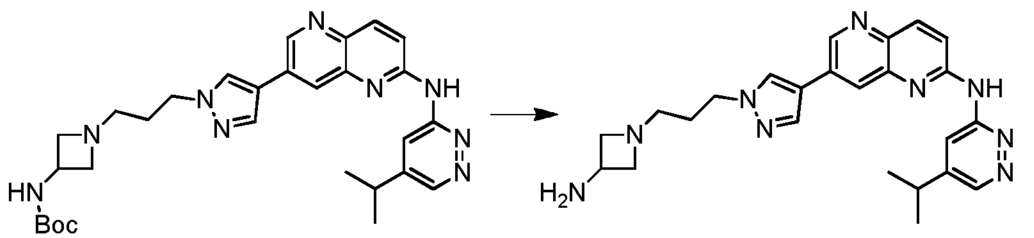

A mixture of tert-butyl (1-(3-(4-(6-((5-isopropylpyridazin-3-yl)amino)-1,5-naphthyridin-3-yl)-1H-pyrazol-1-yl)propyl)azetidin-3-yl)carbamate (17 mg), 1,4-dioxane (2 mL), methanol (1 mL), and a 4.0 mol/L hydrogen chloride/1,4-dioxane solution (1 mL) was stirred at room temperature for 1.5 hours. The solvent was distilled off under reduced pressure, a saturated sodium hydrogen carbonate aqueous solution was added to the obtained residue, and the solvent was distilled off under reduced pressure. The obtained residue was purified by preparative thin layer silica gel column chromatography (chloroform-methanol, NH silica), thereby obtaining 7-(1-(3-(3-aminoazetidin-1-yl)propyl)-1H-pyrazol-4-yl)-N-(5-isopropylpyridazin-3-yl)-1,5-naphthyridine-2-amine (10 mg) as a pale yellow solid.
$^1$H-NMR(DMSO-$d_6$)δ:10.70(1H,s),9.04(1H,d,J=2.0Hz),8.87(1H,d,J=2.0Hz),8.73(1H,d,J=2.0Hz),8.50(1H,s),8.23-8.18(3H,m), 7.70(1H,d,J=9.2Hz),4.17(2H,t,J=6.9Hz),3.51-3046(2H,m),3.39-3.30(2H,m),3.08-2.99(1H,m),2.51-2.45(2H,m),2.33(2H, t,J=6.9Hz),1.86-1.77(3H,m),1.33(6H,d,J=7.2Hz).
MSm/z(M+H):444.

<Example 0696>

<0696-1>

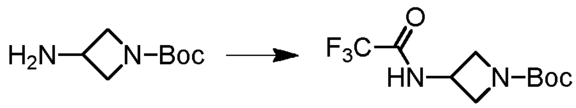

A mixture of tert-butyl 3-aminoazetidine-1-carboxylate (100 mg), triethylamine (322 μL), trifluoroacetic anhydride (244 mg), and dichloromethane (4 mL) was stirred at room temperature for 2 hours. The reaction mixture was purified by silica gel column chromatography (hexane-ethyl acetate-methanol), thereby obtaining tert-butyl 3-(2,2,2-trifluoroacetamide)azetidine-1-carboxylate (157 mg).
$^1$H-NMR(DMSO-$d_6$)δ:10.00(1H,s),4.54-4.50(1H,m),4.09(2H,t,J=8.3Hz),3.83(2H,t,J=8.6Hz),1.38(9H,s).

<0696-2>

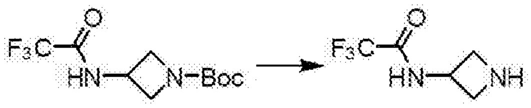

HCL salt

N-(azetidin-3-yl)-2,2,2-trifluoroacetamide hydrochloride was obtained in the same manner as in Example 0687-2 except that tert-butyl 3-(2,2,2-trifluoroacetamide)azetidine-1-carboxylate was used instead of the tert-butyl 3-(methylcarbamoyl)azetidine-1-carboxylate used in Example 0687-2.
$^1$H-NMR(DMSO-$d_6$)6:10.00(1H,brs),4.52-4.49(1H,m),4.13-4.08(2H,m),3.84-3.82(2H,m).

<0696-3>

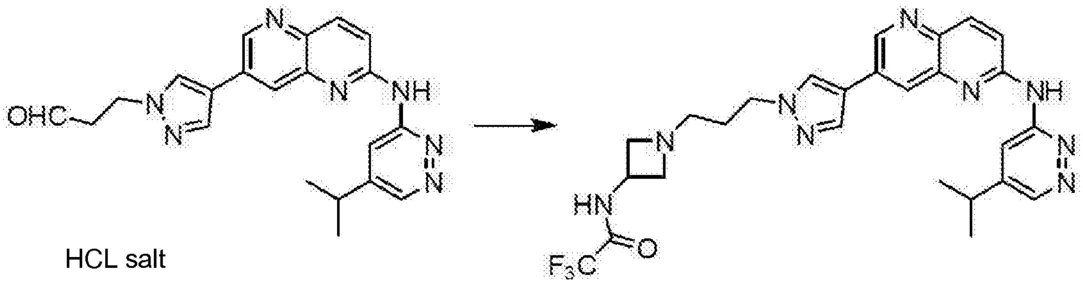

HCL salt

2,2,2-Trifluoro-N-(1-(3-(4-(6-((5-isopropylpyridazin-3-yl)amino)-1,5-naphthyridin-3-yl)-1H-pyrazol-1-yl)propyl)azetidin-3-yl)acetamide was obtained as a pale yellow solid in the same manner as in Example 0426-2 except that N-(azetidin-3-yl)-2,2,2-trifluoroacetamide hydrochloride was used instead of the (2S,6R)-1,2,6-trimethylpiperazine trifluoroacetate used in Example 0426-2.
$^1$H-NMR(DMSO-$d_6$)δ:10.70(1H,s),9.82(1H,brs),9.04(1H,d,J=2.1Hz),8.87(1H,d,J=2.1Hz),8.73(1H,d,J=2.1Hz),8.50(1H,s ),8.22(1H,d,J=9.3Hz),8.21(1H,d,J=2.1Hz),8.19(1H,s),
7.70(1H,d,J=9.3Hz),4.36-4.26(1H,m),4.19(2H,t,J=6.9Hz),3.54(2H,t,J=7.5Hz),3.11-2.96(1H,m),2.96(2H,t,J=7.5Hz),2.40(2H,t,J=7.5Hz),1.90-1.78(2H,m),1.33(6H,d,J=7.2Hz).
MSm/z(M+H):540.

<Example 0697>

<0697-1>

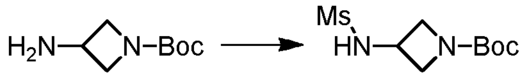

A mixture of tert-butyl 3-aminoazetidine-1-carboxylate (100 mg), triethylamine (322 μL), methanesulfonyl chloride (90 μL), and dichloromethane (4 mL) was stirred at room temperature for 2 hours. The reaction mixture was purified by silica gel column chromatography (hexane-ethyl acetate-methanol), thereby obtaining tert-butyl 3-(methane sulfonamide)azetidine-1-carboxylate (149 mg).
$^1$H-NMR(DMSO-$d_6$)δ:7.81(1H,s),4.11-4.08(3H,m),3.74-3.71(2H,m),2.89(3H,s),1.37(9H,s).

<0697-2>

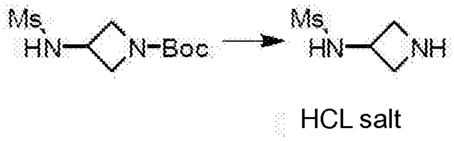


N-(azetidin-3-yl)methane sulfonamide hydrochloride was obtained in the same manner as in Example 0687-2 except that tert-butyl 3-(methane sulfonamide)azetidine-1-carboxylate was used instead of the tert-butyl 3-(methylcarbamoyl)azetidine-1-carboxylate used in Example 0687-2.

<0697-3>

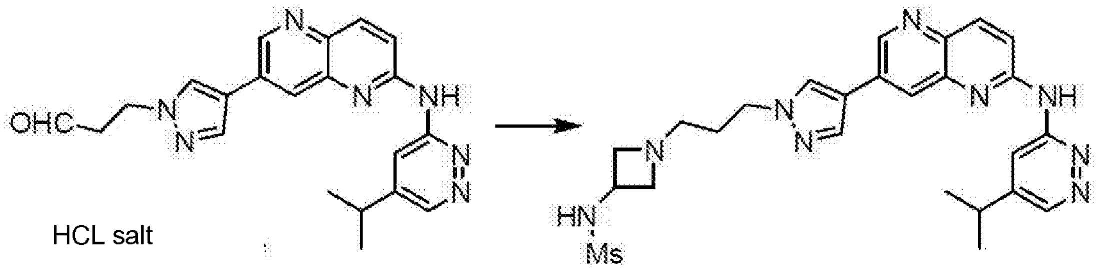


N-(1-(3-(4-(6-((5-isopropylpyridazin-3-yl)amino)-1,5-naphthyridin-3-yl)-1H-pyrazol-1-yl)propyl)azetidin-3-yl)methane sulfonamide was obtained as a pale yellow solid in the same manner as in Example 0426-2 except that N-(azetidin-3-yl)methane sulfonamide hydrochloride was used instead of the (2S,6R)-1,2,6-trimethylpiperazine trifluoroacetate used in Example 0426-2.
$^1$H-NMR(DMSO-$d_6$)δ:10.70(1H,s),9.04(1H,d,J=2.1Hz),8.87(1H,d,J=2.1Hz),8.73(1H,d,J=1.2Hz),8.51(1H,s),8.22(1H,d,J =9.3Hz),8.21(1H,d,J=1.2Hz),8.18(1H,s),7.70(1H,d,J=9.3Hz),7.58(1H,d,J=9.0Hz),4.17(2H,t,J=7.2Hz), 3.93-3.78(1H,m),3.59(2H,t,J=7.5Hz),3.11-2.96(1H,m),2.85(3H,s),2.77(2H,t,J=7.5Hz),2.36(2H,t,J=7.5Hz),1.90-1.76(2H ,m),1.33(6H,d,J=7.2Hz).
MSm/z(M+H):522.

<Example 0698>

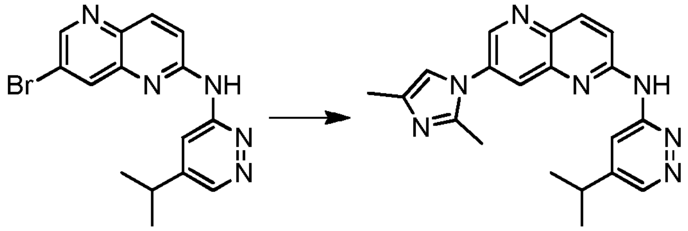

A mixture of tris(dibenzylideneacetone)dipalladium(0) (27 mg), 2-di-tert-butylphosphino-3,4,5,6-tetramethyl-2',4',6'-triisopropyl-1,1'-biphenyl (29 mg), tripotassium phosphate (96 mg), and toluene (1.5 mL) was stirred at 110°C for 5 minutes in a nitrogen atmosphere. 7-Bromo-N-(5-isopropylpyridazin-3-yl)-1,5-naphthyridine-2-amine (50 mg) and 2,4-dimethyl-1H-imidazole (43 mg) were added to the reaction mixture, followed by stirring at 110°C for 4 hours. The reaction mixture was cooled to room temperature, and the solvent was distilled off under reduced pressure. The obtained residue was purified sequentially by silica gel column chromatography (hexane-ethyl acetate-methanol), preparative thin layer silica gel column chromatography (chloroform-methanol, NH silica), and preparative thin layer silica gel column chromatography (chloroform-methanol), thereby obtaining 7-(2,4-dimethyl-1H-imidazol-1 -yl)-N-(5-isopropylpyridazin-3 -yl)-1,5-naphthyridine-2-amine (10 mg) as a pale yellow solid.
$^1$H-NMR(DMSO-d$_6$)δ:10.87(1H,s),8.87(1H,d,J=1.8Hz),8.82(1H,d,J=1.8Hz),8.33(1H,d,J=9.0Hz),8.31(1H,brs),8.22(1H,brs),7.82(1H,d,J=9.0Hz),7.21(1H,s),3.10-2.95(1H,m),2.34(3H,s),2.08(3H,s),1.30(6H,d,J=6.6Hz).
MSm/z(M+H):360.

<Example 0699>

<0699-1>

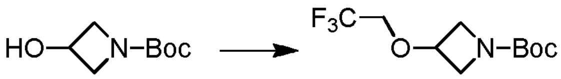

A mixture of tert-butyl 3-hydroxyazetidine-1-carboxylate (150 mg), 3,3,3-trifluoroethyl trifluoromethanesulfonate (188 μL), N,N-dimethylformamide (2 mL), and 60% sodium hydride (42 mg) was stirred at room temperature for 3 hours. After water and ethyl acetate were added to the reaction mixture, the organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate-methanol), thereby obtaining tert-butyl 3-(2,2,2-trifluoroethoxy)azetidine-1-carboxylate (114 mg).
$^1$H-NMR(DMSO-d$_6$)δ:4.44-4.41(1H,m),4.13-4.01(4H,m),3.69(2H,d,J=9.9Hz),1.37(9H,s).

<0699-2>

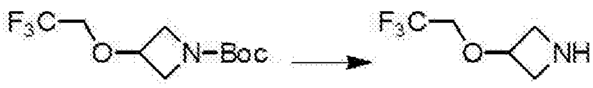

HCL salt

3-(2,2,2-Trifluoroethoxy)azetidine hydrochloride was obtained in the same manner as in Example 0667-1 except that tert-butyl 3-(2,2,2-trifluoroethoxy)azetidine-1-carboxylate was used instead of the tert-butyl 2-((3-(4-(6-((5-isopropylpyridazin-3-yl)amino)-1,5-naphthyridin-3-yl)-1H-pyrazol-1-yl)propyl) (methyl)amino)acetate used in Example 0667.
$^1$H-NMR(DMSO-d$_6$)δ:4.56-4.52(1H,m),4.21-4.11(4H,m),3.87-3.83(2H,m).

<0699-3>

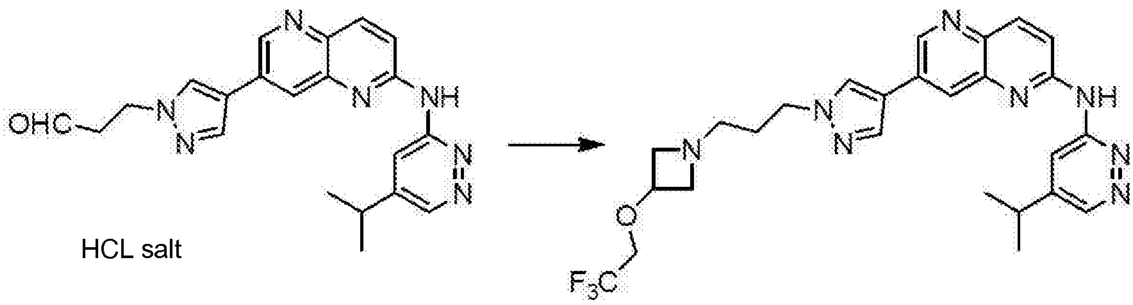


N-(5-isopropylpyridazin-3-yl)-7-(1-(3-(3-(2,2,2-trifluoroethoxy)azetidin-1-yl)propyl)-1H-pyrazol-4-yl)-1,5-naphthyridine-2-amine was obtained as a pale yellow solid in the same manner as in Example 0426-2 except that 3-(2,2,2-trifluoroethoxy)azetidine hydrochloride was used instead of the (2S,6R)-1,2,6-trimethylpiperazine trifluoroacetate used in Example 0426-2.
$^1$H-NMR(DMSO-$d_6$)δ:10.70(1H,s),9.04(1H,d,J=2.1Hz),8.87(1H,d,J=2.1Hz),8.73(1H,brs),8.49(1H,s),8.22(1H,d,J=9.0Hz ),8.21(1H,brs),8.18(1H,s),7.70(1H,d,J=9.0Hz),4.30-4.16(1H,m),4.17(2H,t,J=7.2Hz),4.03(2H,q,J=6.6Hz),3.52(2H,dd,J=7.8,5.7Hz),3.11-2.96(1H,m),2.81(2H,dd,J=7.8,5.7Hz),2.39(2H,t,J=6.6Hz),1.90-1.77(2H,m),1.33(6H,d,J=6.6Hz).
MSm/z(M+H):527.

<Example 0700>

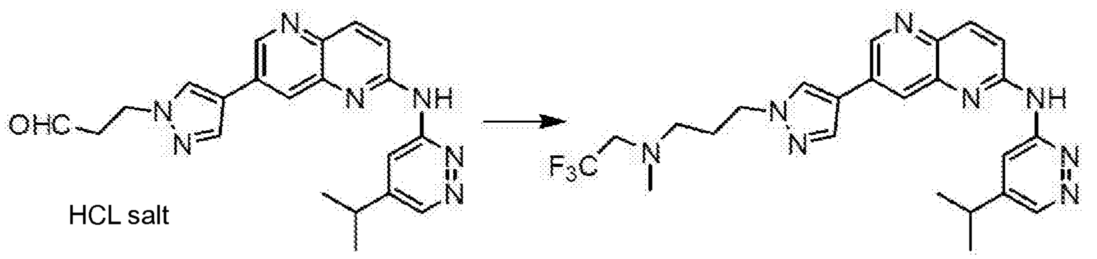


N-(5-isopropylpyridazin-3-yl)-7-(1-(3-(methyl(2,2,2-trifluoroethyl)amino)propyl)-1H-pyrazol-4-yl)-1,5-naphthyridine-2-amine was obtained as a pale yellow solid in the same manner as in Example 0426-2 except that 2,2,2-trifluoro-N-methylethanamine hydrochloride was used instead of the (2S,6R)-1,2,6-trimethylpiperazine trifluoroacetate used in Example 0426-2.
$^1$H-NMR(DMSO-$d_6$)δ:10.70(1H,s),9.04(1H,d,J=2.1Hz),8.87(1H,d,J=2.1Hz),8.74(1H,d,J=2.1Hz),8.48(1H,s),8.22(1H,d,J=9.0Hz),8.21(1H,brs)8.20(1H,brs),7.70(1H,d,J=9.0Hz),4.20(2H,t,J=6.6Hz),3.28-3.10(2H,m),3.11-2.96(1H,m),2.54(2H,t,J=7.2Hz),2.37(3H,s),2.06-1.95(2H,m),1.33(6H,d,J=7.2Hz).
MSm/z(M+H):485.

<Example 0701>

<0701-1>

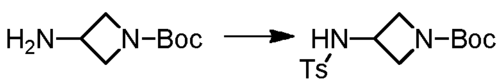

tert-Butyl 3-(4-methylphenylsulfonamide)azetidine-1-carboxylate was obtained in the same manner as in Example 0697-1 except that paratoluenesulphonyl chloride was used instead of the methanesulfonyl chloride used in Example 0697-1.

$^1$H-NMR(DMSO-$d_6$)δ:8.26(1H,brs),7.66(2H,d,J=8.6Hz),7.41(2H,d,J=7.9Hz),4.01-3.98(1H,m),3.85-3.82(2H,m),3.44-3.41(2H,m),2.40(3H,s),1.32(9H,s).

<0701-2>

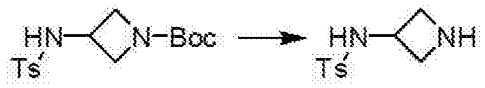

HCL salt

N-(azetidin-3-yl)-4-methylbenzene sulfonamide hydrochloride was obtained in the same manner as in Example 0667-1 except that tert-butyl 3-(4-methylphenylsulfonamide)azetidine-1-carboxylate was used instead of the tert-butyl 2-((3-(4-(6-((5-isopropylpyridazin-3-yl)amino)-1,5-naphthyridin-3-yl)-1H-pyrazol-1-yl)propyl) (methyl)amino)acetate used in Example 0667-1.
$^1$H-NMR(DMSO-$d_6$)δ:7.69(2H,d,J=8.6Hz),7.43(2H,d,J=7.9Hz),4.13-4.10(1H,m),3.89(2H,t,J=9.6Hz),3.69(2H,t,J=11.6Hz),2.40(3H,s).

<0701-3>

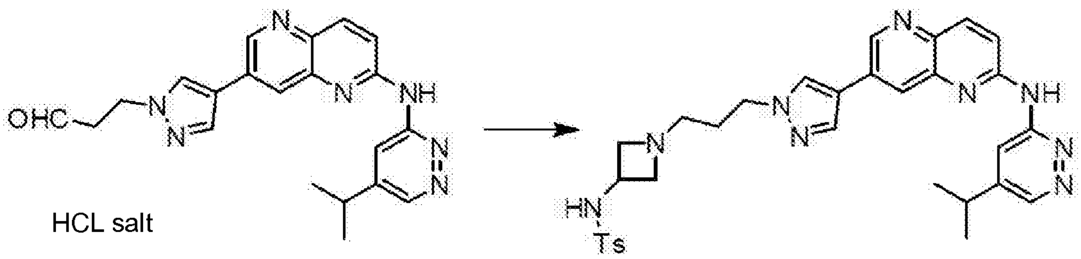

HCL salt

N-(1-(3-(4-(6-((5-isopropylpyridazin-3-yl)amino)-1,5-naphthyridin-3-yl)-1H-pyrazol-1-yl)propyl)azetidin-3-yl)-4-methylbenzene sulfonamide was obtained as a pale yellow solid in the same manner as in Example 0426-2 except that N-(azetidin-3-yl)-4-methylbenzene sulfonamide hydrochloride was used instead of the (2S,6R)-1,2,6-trimethylpiperazine trifluoroacetate used in Example 0426-2.
$^1$H-NMR(DMSO-$d_6$)δ:10.69(1H,s),9.02(1H,d,J=2.1Hz),8.87(1H,d,J=2.1Hz),8.72(1H,brs),8.45(1H,s),8.21(1H,d,J=9.0Hz ),8.19(1H,s),8.16(1H,s),7.70(1H,d,J=9.0Hz),7.66(2H,d,J=8.1Hz),7.38(2H,d,J=8.1Hz),4.11(2H,t,J=6.6 Hz),3.79-3.65(1H,m),3.52-3.20(2H,m),3.11-2.96(1H,m),2.60-2.51(2H,m),2.37(3H,s),2.29-2.20(2H,m),1.82-1.67(2H,m),1.33(6H,d,J=7.2Hz).
MSm/z(M+H):598.

<Example 0702>

<0702-1>

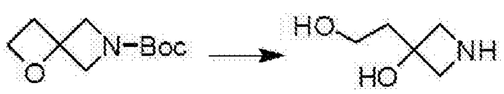

HCL salt

3-(2-Hydroxyethyl)azetidin-3-ol hydrochloride was obtained in the same manner as in Example 0667-1 except that tert-butyl 1-oxa-6-azaspiro[3.3]heptane-6-carboxylate was used instead of the tert-butyl 2-((3-(4-(6-((5-isopropylpyridazin-3-yl)amino)-1,5-naphthyridin-3-yl)-1H-pyrazol-1-yl)propyl) (methyl)amino)acetate used in Example 0667-1.
$^1$H-NMR(DMSO-$d_6$)δ:3.94-3.93(2H,m),3.84-3.79(2H,m),3.70(2H,t,J=7.3Hz),2.24(2H,t,J=7.3Hz).

<0702-2>

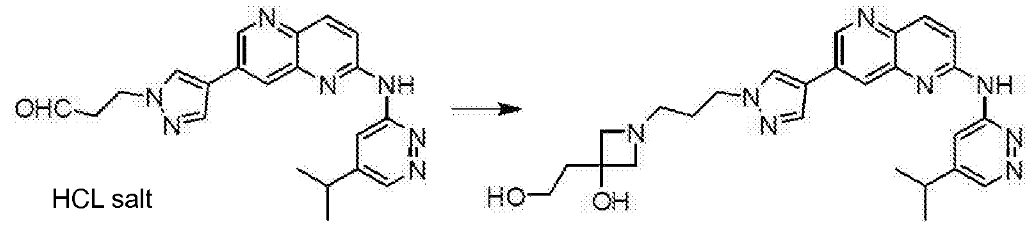

3-(2-Hydroxyethyl)-1-(3-(4-(6-((5-isopropylpyridazin-3-yl)amino)-1,5-naphthyridin-3-yl)-1H-pyrazol-1-yl)propyl)azetidin-3-ol was obtained as a pale yellow solid in the same manner as in Example 0426-2 except that 3-(2-hydroxyethyl)azetidin-3-ol hydrochloride was used instead of the (2S,6R)-1,2,6-trimethylpiperazine trifluoroacetate used in Example 0426-2.
$^1$H-NMR(DMSO-$d_6$)δ:10.69(1H,s),9.04(1H,d,J=2.1Hz),8.87(1H,d,J=2.1Hz),8.72(1H,brs),8.50(1H,s),8.22(1H,d,J=9.0Hz ),8.21(1H,brs),8.19(1H,s),8.70(1H,d,J=9.0Hz),4.64(1H,brs),4.49(1H,brs),4.21(2H,t,J=6.6Hz),3.47-3.37(2H,m),3.11-2.96(lH,m),2.64-2.44(4H,m),2.35(2H,t,J=7.2Hz),2.03-1.90(2H,m),1.87-1.74(1H,m),1.62-1.51(1H,m),1.33(6H,d,J=6.6Hz).
MSm/z(M+H):489.

<Example 0703>

<0703-1>

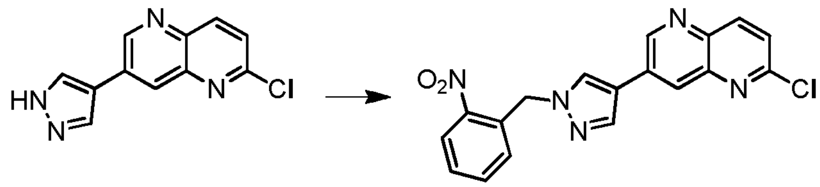

A mixture of 2-chloro-7-(1H-pyrazol-4-yl)-1,5-naphthyridine (100 mg), 1-(bromomethyl)-2-nitrobenzene (112 mg), potassium carbonate (119 mg), and N,N-dimethylformamide (4 mL) was stirred at 60°C for 2 hours. The reaction mixture was cooled to room temperature, water was added thereto, and the solid matter was collected by filtration. The obtained solid matter was purified by silica gel column chromatography (hexane-ethyl acetate- methanol), thereby obtaining 2-chloro-7-(1-(2-nitrobenzyl)-1H-pyrazol-4-yl)-1,5-naphthyridine (134 mg).
MSm/z(M+H):366.

<0703-2>

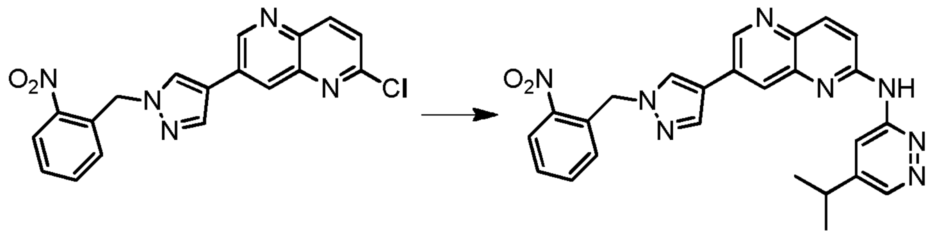

N-(5-isopropylpyridazin-3-yl)-7-(1-(2-nitrobenzyl)-1H-pyrazol-4-yl)-1,5-naphthyridine-2-amine was obtained as a pale yellow solid in the same manner as in Example 0646-3 except that 2-chloro-7-(1-(2-nitrobenzyl)-1H-pyrazol-4-yl)-1,5-naphthyridine was used instead of the 2-chloro-7-(1-(2,2-dimethoxypropyl)-1H-pyrazol-4-yl)-1,5-naphthyridine used in Example 0646-3.
$^1$H-NMR(DMSO-$d_6$)δ:10.71(1H,s),9.06(1H,d,J=1.8Hz),8.86(1H,d,J=1.8Hz),8.74(1H,d,J=1.8Hz),8.64(1H,s),8.32(1H,s), 8.25(1H,brs),8.23(1H,d,J=9.0Hz),8.14(1H,d,J=6.9Hz),7.74(1H,t,J=6.9Hz),7.71(1H,d,J=9.0Hz),7.61(1 H,t,J=6.9Hz),7.01(1H,d,J=6.9Hz),5.81(2H,s),3.11-2.96(1H,m),1.32(6H,d,J=6.6Hz).
MSm/z(M+H):467.

<Example 0704>

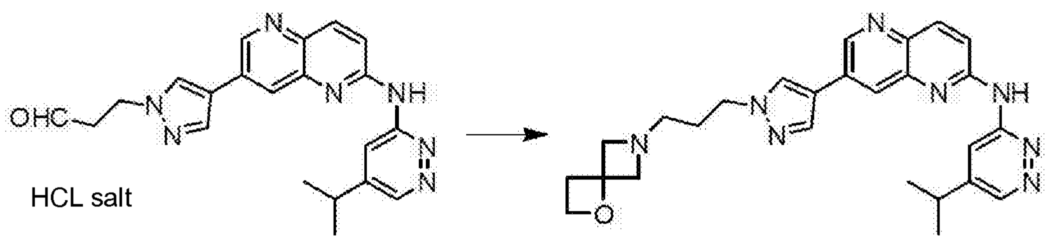


7-(1-(3-(1-Oxa-6-azaspiro[3.3]heptan-6-yl)propyl)-1H-pyrazol-4-yl)-N-(5-isopropylpyridazin-3-yl)-1,5-naphthyridine-2-amine was obtained as a pale yellow solid in the same manner as in Example 0426-2 except that 1-oxa-6-azaspiro[3.3]heptanetrifluoroacetate was used instead of the (2S,6R)-1,2,6-trimethylpiperazine trifluoroacetate used in Example 0426-2.
$^1$H-NMR(DMSO-$d_6$)δ:10.70(1H,s),9.04(1H,d,J=2.1Hz),8.87(1H,d,J=2.1Hz),8.73(1H,d,J=2.1Hz),8.49(1H,s),8.21(1H,d,J =9.0Hz),8.21(1H,d,J=2.1Hz),8.18(1H,s),7.70(1H,d,J=9.0Hz),4.36(2H,t,J=7.5Hz),4.16(2H,t,J=6.9Hz),3 .52-3.46(2H,m), 3.11-2.96(1H,m),2.99-2.95(2H,m),2.78-2.70(2H,m),2.34(2H,t,J=6.6Hz),1.90-1.75(2H,m),1.33(6H,d,J=7.2Hz).
MSm/z(M+H):471.

<Example 0705>

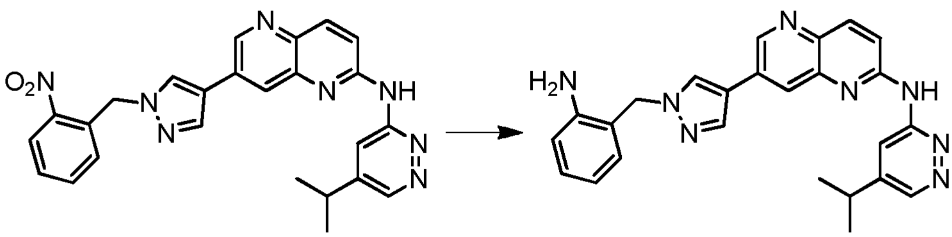

A mixture of N-(5-isopropylpyridazin-3-yl)-7-(1-(2-nitrobenzyl)-1H-pyrazol-4-yl)-1,5-naphthyridine-2-amine (50 mg), reduced iron (18 mg), ammonium chloride (6 mg), ethanol (4 mL), and water (2 mL) was stirred at 60°C for 6 hours. The reaction mixture was cooled to room temperature, the insolubles were filtered off, and the solvent was distilled off under reduced pressure. The obtained residue was purified sequentially by silica gel column chromatography (hexane-ethyl acetate-methanol, NH silica), and silica gel column chromatography (chloroform-methanol, NH silica), thereby obtaining 7-(1-(2-aminobenzyl)-1H-pyrazol-4-yl)-N-(5-isopropylpyridazin-3-yl)-1,5-naphthyridine-2-amine (11 mg) as a pale yellow solid.
$^1$H-NMR(DMSO-$d_6$)δ:10.70(1H,s),9.03(1H,d,J=1.8Hz),8.87(1H,d,J=1.8Hz),8.73(1H,brs),8.57(1H,s),8.24(1H,s),8.21(1H ,d,J=9.0Hz),8.21(1H,brs),8.70(1H,d,J=9.0Hz),7.02(1H,t,J=7.8Hz),6.92(1H,d,J=7.8Hz),6.69(1H,d,J=7.8Hz),6.55(1H,t,J=7.8Hz),5.26(2H,s),3.11-2.96(1H,m),1.33(6H,d,J=6.6Hz).
MSm/z(M+H):437.

<Example 0706>

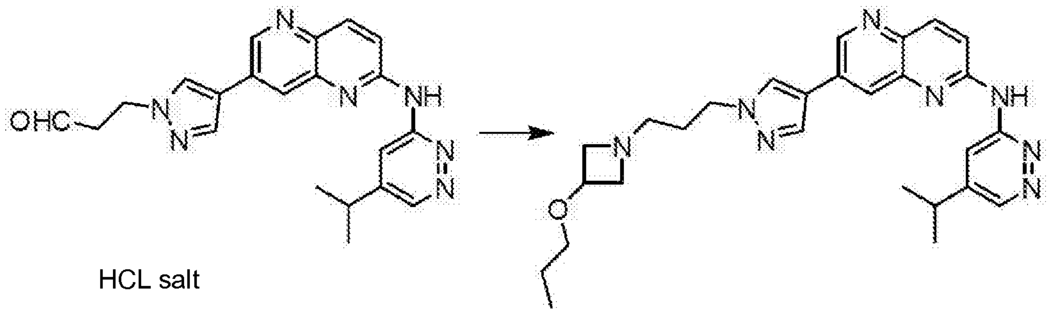

HCL salt

N-(5-isopropylpyridazin-3-yl)-7-(1-(3-(3-propoxyazetidin-1-yl)propyl)-1H-pyrazol-4-yl)-1,5-naphthyridine-2-amine was obtained as a pale yellow solid in the same manner as in Example 0426-2 except that 3-propoxyazetidine hydrochloride was used instead of the (2S,6R)-1,2,6-trimethylpiperazine trifluoroacetate used in Example 0426-2.
$^1$H-NMR(DMSO-$d_6$)δ:10.68(1H,s),9.04(1H,d,J=1.8Hz),8.87(1H,d,J=1.8Hz),8.73(1H,d,J=1.8Hz),8.49(1H,s),8.22(1H,d,J=9.3Hz),8.21(1H,d,J=1.8Hz),8.18(1H,s),7.70(1H,d,J=9.3Hz),4.18(2H,t,J=6.9Hz),4.06-3.93(1H,m),3.59-3.46(2H,m),3.24(2H,t,J=6.6Hz),3.11-2.96(1H,m),2.75-2.68(2H,m),2.38(2H,t,J=6.6Hz),1.90-1.77(2H,m),1.53-1.36(2H,m),1.33(6H,d,J=7.2Hz),0.84(3H,t,J=7.2Hz).
MSm/z(M+H):487.

<Example 0707>

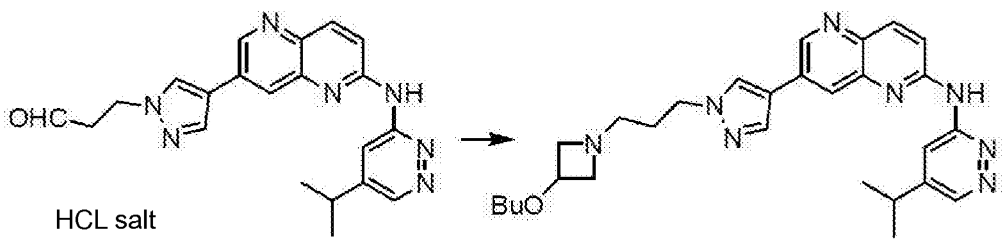

HCL salt

7-(1-(3-(3-Butoxyazetidin-1-yl)propyl)-1H-pyrazol-4-yl)-N-(5-isopropylpyridazin-3-yl)-1,5-naphthyridine-2-amine was obtained as a pale yellow solid in the same manner as in Example 0426-2 except that 3-butoxyazetidine hydrochloride was used instead of the (2S,6R)-1,2,6-trimethylpiperazine trifluoroacetate used in Example 0426-2.
$^1$H-NMR(DMSO-$d_6$)δ:10.69(1H,s),9.04(1H,d,J=1.8Hz),8.87(1H,d,J=1.8Hz),8.73(1H,d,J=1.2Hz),8.49(1H,s),8.22(1H,d,J=9.3Hz),8.21(1H,d,J=1.2Hz),8.18(1H,s),7.70(1H,d,J=9.3Hz),4.18(2H,t,J=6.9Hz),4.05-3.94(1H,m),3.55-3.47(2H,m),3.27(2H,t,J=6.6Hz),3.11-2.96(1H,m),2.75-2.67(2H,m),2.38(2H,t,J=6.6Hz),1.90-1.77(2H,m),1.50-1.37(2H,m),1.35-1.15(2H,m),1.33(6H,d,J=6.6Hz),0.58(3H,t,J=7.2Hz).
MSm/z(M+H):501.

<Example 0708>

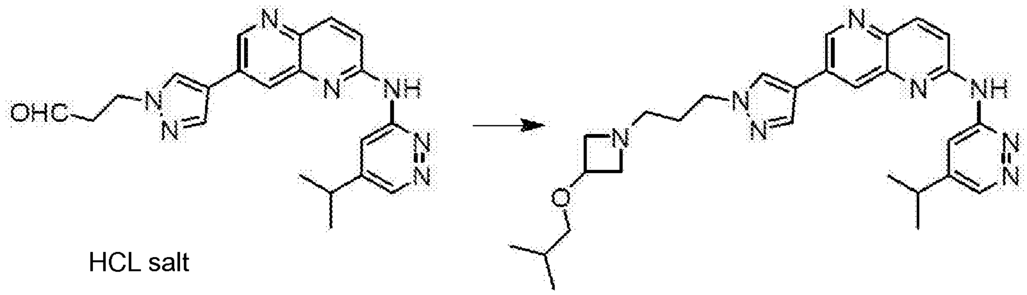

HCL salt

7-(1-(3-(3-Isobutoxyazetidin-1-yl)propyl)-1H-pyrazol-4-yl)-N-(5-isopropylpyridazin-3-yl)-1,5-naphthyridine-2-amine was obtained as a pale yellow solid in the same manner as in Example 0426-2 except that 3-isobutoxyazetidine hydrochloride was used instead of the (2S,6R)-1,2,6-trimethylpiperazine trifluoroacetate used in Example 0426-2.
$^1$H-NMR(DMSO-$d_6$)δ:10.68(1H,s),9.04(1H,d,J=1.8Hz),8.87(1H,d,J=1.8Hz),8.73(1H,brs),8.49(1H,s),8.22(1H,d,J=9.3Hz ),8.21(1H,d,J=1.8Hz),8.18(1H,s),7.70(1H,d,J=9.3Hz),4.18(2H,t,J=7.2Hz),4.05-3.94(1H,m),3.59-3.50(2H,m),3.11-2.96(1H,m),3.05(2H,d,J=6.6Hz),2.75-2.67(2H,m),2.38(2H,d,J=6.6Hz),1.90-1.63(3H,m),1.33(6H,d,J=7.2Hz),0.84(6H,d,J=6.6Hz).
MSm/z(M+H):501.

<Example 0709>

<0709-1>

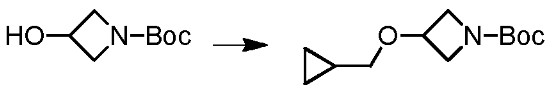

tert-Butyl 3-(cyclopropylmethoxy)azetidine-1-carboxylate was obtained in the same manner as in Example 0699-1 except that (bromomethyl)cyclopropane was used instead of the 3,3,3-trifluoropropyl trifluoromethanesulfonate used in Example 0699-1.
$^1$H-NMR(DMSO-$d_6$)δ:4.24-4.21(1H,m),3.99(2H,t,J=7.3Hz),3.65(2H,dd,J=10.0,5.0Hz),3.18(2H,d,J=6.6Hz),1.37(9H,s),0.98-0.95(1H,m),0.49-0.43(2H,m),0.16(2H,td,J=5.1,4.0Hz).

<0709-2>

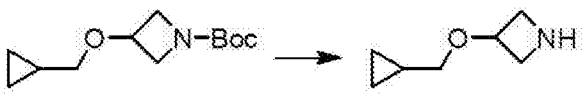

HCL salt

3-(Cyclopropylmethoxy)azetidine hydrochloride was obtained in the same manner as in Example 0667-1 except that tert-butyl 3-(cyclopropylmethoxy)azetidine-1-carboxylate was used instead of the tert-butyl 2-((3-(4-(6-((5-isopropylpyridazin-3-yl)amino)-1,5-naphthyridin-3-yl)-1H-pyrazol-1-yl)propyl) (methyl)amino)acetate used in Example 0667.
$^1$H-NMR(DMSO-$d_6$)δ:4.39-4.31(1H,m),4.14-4.10(2H,m),3.86-3.76(2H,m),3.24(2H,d,J=6.6Hz),0.98-0.94(1H,m),0.51-0.45(2H,m),0.20-0.16(2H,m).

<0709-3>

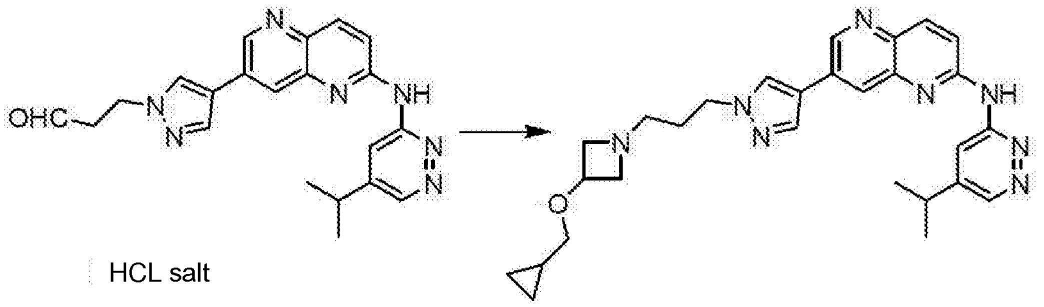


7-(1-(3-(3-(Cyclopropylmethoxy)azetidin-1-yl)propyl)-1H-pyrazol-4-yl)-N-(5-isopropylpyridazin-3-yl)-1,5-naphthyridine-2-amine was obtained as a pale yellow solid in the same manner as in Example 0426-2 except that 3-(cyclopropylmethoxy)azetidine hydrochloride was used instead of the (2S,6R)-1,2,6-trimethylpiperazine trifluoroacetate used in Example 0426-2.
$^1$H-NMR(DMSO-$d_6$)$\delta$:10.69(1H,s),9.04(1H,d,J=1.8Hz),8.87(1H,d,J=1.8Hz),8.72(1H,brs),8.49(1H,s),8.22(1H,d,J=9.0Hz ),8.21(1H,d,J=1.8Hz),8.18(1H,s),7.70(1H,d,J=9.0Hz),4.18(2H,t,J=7.2Hz),4.10-3.97(1H,m),3.61-3.49(2H,m),3.13(2H,d,J=7.2Hz),3.11-2.96(1H,m),2.77-2.70(2H,m),2.38(2H,t,J=6.6Hz),1.91-1.76(2H,m),1.33(6H,d,J=6.6Hz),1.00-0.83(1H,m),0.49-0.41(2H,m),0.18-0.10(2H,m).
MSm/z(M+H):499.

<Example 0710>

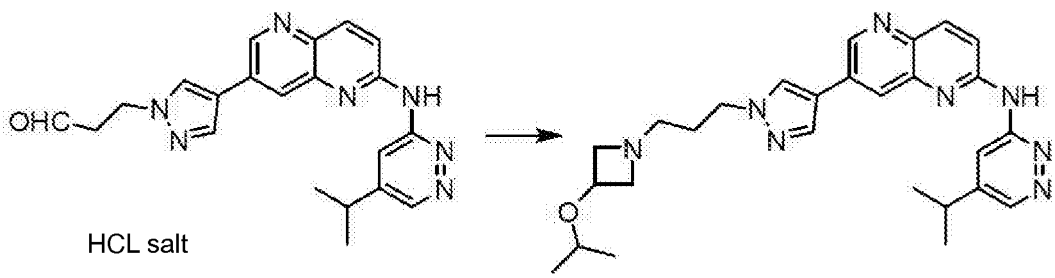


7-(1-(3-(3-Isopropoxyazetidin-1-yl)propyl)-1H-pyrazol-4-yl)-N-(5-isopropylpyridazin-3-yl)-1,5-naphthyridine-2-amine was obtained as a pale yellow solid in the same manner as in Example 0426-2 except that 3-isopropoxyazetidine hydrochloride was used instead of the (2S,6R)-1,2,6-trimethylpiperazine trifluoroacetate used in Example 0426-2.
$^1$H-NMR(DMSO-$d_6$)$\delta$:10.68(1H,s),9.04(1H,d,J=1.8Hz),8.86(1H,d,J=1.8Hz),8.72(1H,brs),8.49(1H,s),8.22(1H,d,J=9.3Hz ),8.21(1H,brs),8.18(1H,s),7.70(1H,d,J=9.3Hz),4.17(2H,t,J=6.9Hz),4.12-4.00(1H,m),3.59-3.49(3H,m),3.12-2.96(1H,m),2.71-2.63(2H,m),2.37(2H,t,J=6.6Hz),1.90-1.76(2H,m),1.33(6H,d,J=7.2Hz),1.04(6H,d,J=6.0Hz).
MSm/z(M+H):487.

<Example 0711>

<0711-1>

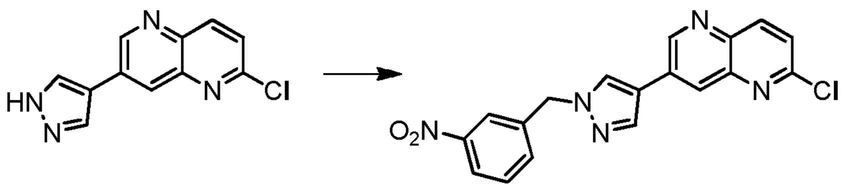

2-Chloro-7-(1-(3-nitrobenzyl)-1H-pyrazol-4-yl)-1,5-naphthyridine was obtained in the same manner as in Example 0703-1 except that 1-(chloromethyl)-3-nitrobenzene was used instead of the 1-(bromomethyl)-2-nitrobenzene used in Example 0703-1.
MSm/z(M+H):366.

<0711-2>

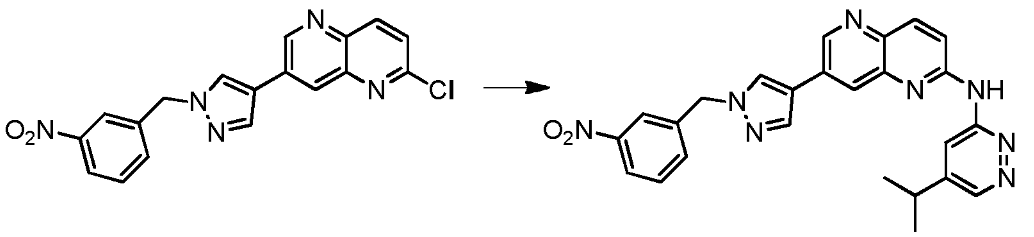

N-(5-isopropylpyridazin-3-yl)-7-(1-(3-nitrobenzyl)-1H-pyrazol-4-yl)-1,5-naphthyridine-2-amine was obtained as a yellow solid in the same manner as in Example 0426-3 except that 2-chloro-7-(1-(3-nitrobenzyl)-1H-pyrazol-4-yl)-1,5-naphthyridine was used instead of the 2-chloro-7-(1-(2,2-dimethoxypropyl)-1H-pyrazol-4-yl)-1,5-naphthyridine used in Example 0646-3.
$^1$H-NMR(DMSO-$d_6$)δ:10.71(1H,s),9.05(1H,d,J=2.1Hz),8.87(1H,d,J=2.1Hz),8.73(1H,brs),8.69(1H,s),8.29(1H,s),8.26-8.16(4H,m),7.80-7.65(3H,m),5.60(2H,s),3.11-2.95(1H,m),1.33(6H,d,J=7.2Hz).
MSm/z(M+H):467.

<Example 0712>

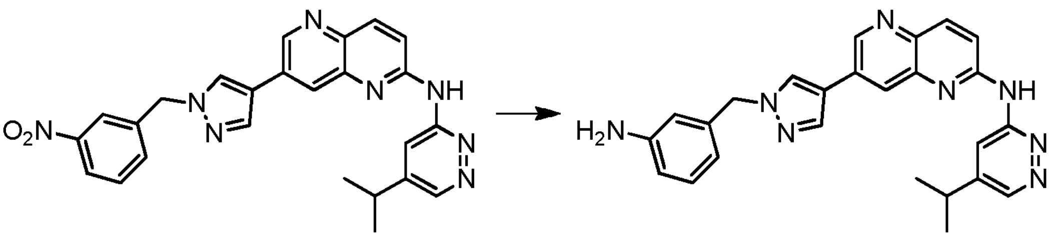

7-(1-(3-Aminobenzyl)-1H-pyrazol-4-yl)-N-(5-isopropylpyridazin-3-yl)-1,5-naphthyridine-2-amine was obtained as a pale yellow solid in the same manner as in Example 0705-1 except that N-(5-isopropylpyridazin-3-yl)-7-(1-(3-nitrobenzyl)-1H-pyrazol-4-yl)-1,5-naphthyridine-2-amine was used instead of the N-(5-isopropylpyridazin-3-yl)-7-(1-(2-nitrobenzyl)-1H-pyrazol-4-yl)-1,5-naphthyridine-2-amine used in Example 0705.
$^1$H-NMR(DMSO-$d_6$)δ:10.69(1H,s),9.05(1H,d,J=2.1Hz),8.86(1H,d,J=2.1Hz),8.73(1H,brs),8.58(1H,s),8.22(2H,brs),8.21( 1H,d,J9.0Hz),7.70(1H,d,J=9.0Hz),6.98(1H,t,J=8.1Hz),6.51-6.42(3H,m),5.23(2H,s),5.11(2H,brs),3.12-2.95(1H,m),1.33(6H,d,J=6.6Hz).
MSm/z(M+H):437.

<Example 0713>

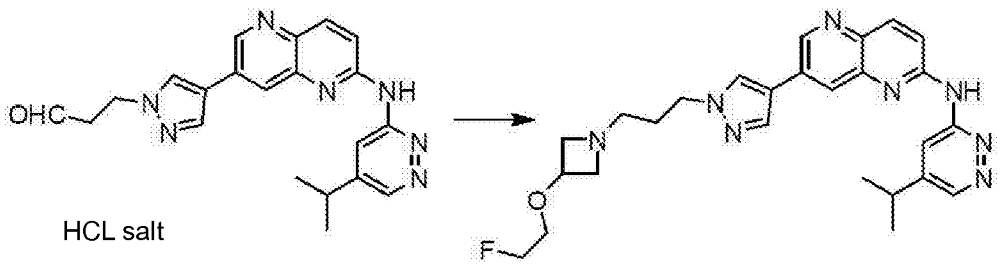

7-(1-(3-(3-(2-Fluoroethoxy)azetidin-1-yl)propyl)-1H-pyrazol-4-yl)-N-(5-isopropylpyridazin-3-yl)-1,5-naphthyridine-2-amine was obtained as a pale yellow solid in the same manner as in Example 0426-2 except that 3-(2-fluoroethoxy)azetidine hydrochloride was used instead of the (2S,6R)-1,2,6-trimethylpiperazine trifluoroacetate used in Example 0426-2.
$^1$H-NMR(DMSO-$d_6$)δ:10.69(1H,s),9.04(1H,d,J=2.1Hz),8.87(1H,d,J=2.1Hz),8.73(1H,brs),8.49(1H,s),8.21(1H,d,J=9.3Hz ),8.20(1H,d,J=2.1Hz),8.18(1H,s),7.70(1H,d,J=9.3Hz),4.60-4.38(2H,m),4.23-4.04(3H,m),3.64-3.44(4H,m),3.11-2.96(1H,m),2.81-2.71(2H,m),2.39(2H,t,J=6.6Hz),1.90-1.78(2H,m),1.33(6H,d,J=6.6Hz).
MSm/z(M+H):491.

<Example 0714>

<0714-1>

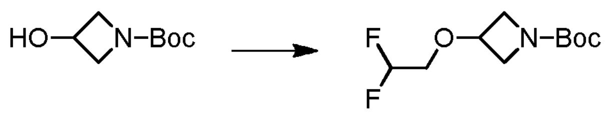

tert-Butyl 3-(2,2-difluoroethoxy)azetidine-1-carboxylate was obtained in the same manner as in Example 0699-1 except that 2,2-difluoroethyl 4-methylbenzenesulfonate was used instead of the 3,3,3-trifluoropropyl trifluoromethanesulfonate used in Example 0699-1.
$^1$H-NMR(DMSO-$d_6$)δ:4.35-4.33(1H,m),4.03-4.00(3H,m),3.70-3.65(5H,m),1.37(9H,s).

<0714-2>

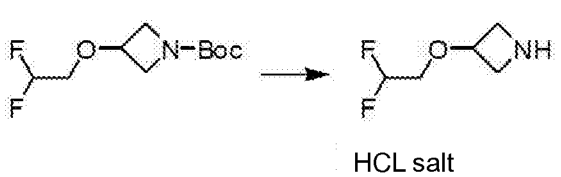

3-(2,2-Difluoroethoxy)azetidine hydrochloride was obtained in the same manner as in Example 0667-1 except that tert-butyl 3-(2,2-difluoroethoxy)azetidine-1-carboxylate was used instead of the tert-butyl2-((3-(4-(6-((5-isopropylpyridazin-3-yl)amino)-1,5-naphthyridin-3-yl)-1H-pyrazol-1-yl)propyl) (methyl)amino)acetate used in Example 0667.
$^1$H-NMR(DMSO-$d_6$)δ:4.48-4.44(1H,m),4.14-4.12(2H,m),3.97(1H,brs),3.84-3.69(4H,m).

<0714-3>

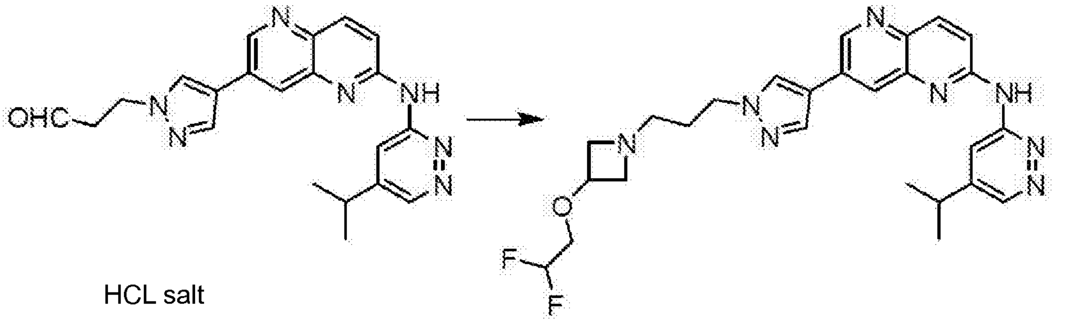


7-(1-(3-(3-(2,2-Difluoroethoxy)azetidin-1-yl)propyl)-1H-pyrazol-4-yl)-N-(5-isopropylpyridazin-3-yl)-1,5-naphthyridine-2-amine was obtained in the same manner as in Example 0426-2 except that 3-(2,2-difluoroethoxy)azetidine hydrochloride was used instead of the (2S,6R)-1,2,6-trimethylpiperazine trifluoroacetate used in Example 0426-2.
$^1$H-NMR(DMSO-$d_6$)δ:10.69(1H,s),9.04(1H,d,J=2.1Hz),8.87(1H,d,J=2.1Hz),8.73(1H,d,J=2.1Hz),8.49(1H,s),8.22(1H,d,J=9.3Hz),8.21(1H,d,J=2.1Hz),8.18(1H,s),7.70(1H,d,J=9.3Hz),6.33-5.90(1H,m),4.22-4.07(3H,m),3.72-3.47(4H,m),3.11-2.97(1H,m),2.82-2.75(2H,m),2.43-2.35(2H,m),1.93-1.76(2H,m),1.33(6H,d,J=7.2Hz).
MSm/z(M+H):509.

<Example 0715>

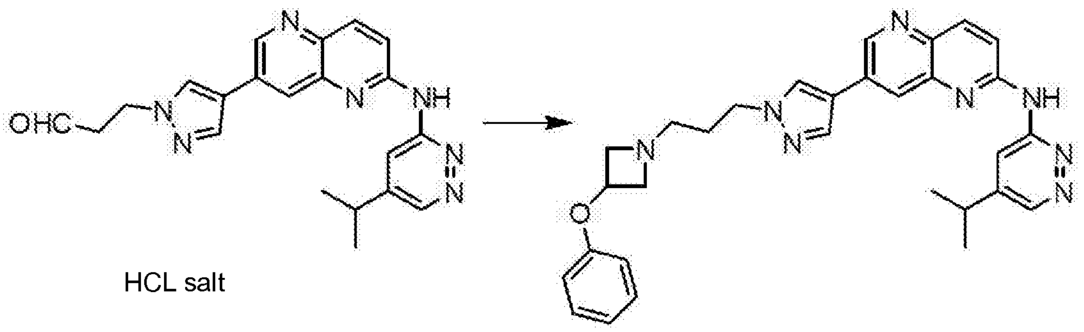


N-(5-isopropylpyridazin-3-yl)-7-(1-(3-(3-phenoxyazetidin-1-yl)propyl)-1H-pyrazol-4-yl)-1,5-naphthyridine-2-amine was obtained as a pale yellow solid in the same manner as in Example 0426-2 except that 3-phenoxyazetidine hydrochloride was used instead of the (2S,6R)-1,2,6-trimethylpiperazine trifluoroacetate used in Example 0426-2.
$^1$H-NMR(DMSO-$d_6$)δ:10.69(1H,s),9.04(1H,d,J=2.1Hz),8.86(1H,d,J=2.1Hz),8.73(1H,brs),8.24-8.16(4H,m),7.70(1H,d,J=9.3Hz),7.31-7.23(2H,m),6.93(1H,t,J=7.5Hz),6.86-6.79(2H,m),4.84-4.73(1H,m),4.20(2H,t,J=7.2Hz),3.76(2H,t,J=7.2Hz),3.11-2.92(3H,m),2.50-2.43(2H,m),1.95-1.80(2H,m),1.33(6H,d,J=6.6Hz).
MSm/z(M+H):521.

<Example 0716>

<0716-1>

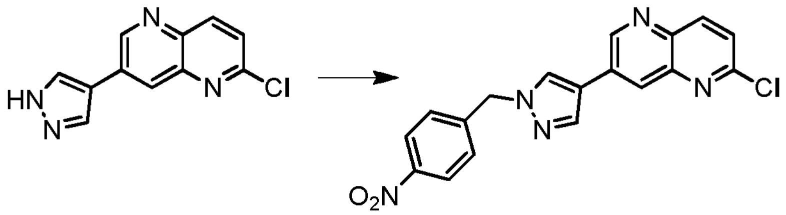

2-Chloro-7-(1-(4-nitrobenzyl)-1H-pyrazol-4-yl)-1,5-naphthyridine was obtained in the same manner as in Example 0703-1 except that 1-(bromomethyl)-4-nitrobenzene was used instead of the 1-(bromomethyl)-2-nitrobenzene used in Example 0703-1.
MSm/z(M+H):366.

<0716-2>

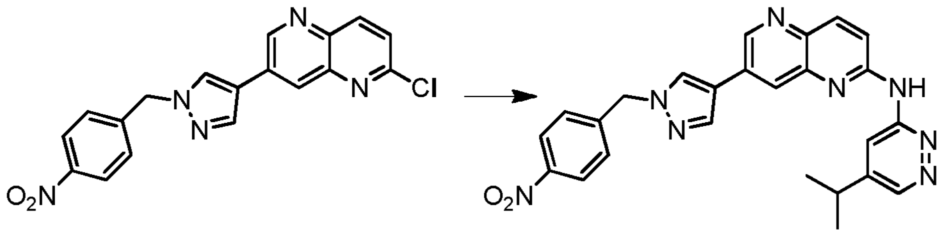

N-(5-isopropylpyridazin-3-yl)-7-(1-(4-nitrobenzyl)-1H-pyrazol-4-yl)-1,5-naphthyridine-2-amine was obtained as a yellow solid in the same manner as in Example 0646-3 except that 2-chloro-7-(1-(4-nitrobenzyl)-1H-pyrazol-4-yl)-1,5-naphthyridine was used instead of the 2-chloro-7-(1-(2,2-dimethoxypropyl)-1H-pyrazol-4-yl)-1,5-naphthyridine used in Example 0646-3.
$^1$H-NMR(DMSO-$d_6$)δ:10.70(1H,s),9.06(1H,d,J=1.8Hz),8.87(1H,d,J=1.8Hz),8.73(1H,d,J=1.8Hz),8.69(1H,s),8.30(1H,s),8.25(2H,d,J=8.4Hz),8.24(1H,d,J=1.8Hz),8.22(1H,d,J=9.0Hz),7.71(1H,d,J=9.0Hz),7.53(2H,d,J=8.4Hz) ,5.60(2H,s),3.1 1-2.95(1H,m),1.33(6H,d,J=7.2Hz).
MSm/z(M+H):467.

<Example 0717>

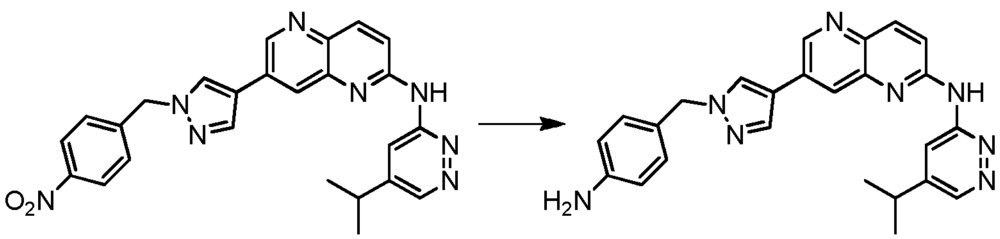

7-(1-(4-Aminobenzyl)-1H-pyrazol-4-yl)-N-(5-isopropylpyridazin-3-yl)-1,5-naphthyridine-2-amine was obtained as a yellow solid in the same manner as in Example 0705 except that N-(5-isopropylpyridazin-3-yl)-7-(1-(4-nitrobenzyl)-1H-pyrazol-4-yl)-1,5-naphthyridine-2-amine was used instead of the N-(5-isopropylpyridazin-3-yl)-7-(1-(2-nitrobenzyl)-1H-pyrazol-4-yl)-1,5-naphthyridine-2-amine used in Example 0705.
$^1$H-NMR(DMSO-$d_6$)δ:10.69(1H,s),9.03(1H,d,J=1.8Hz),8.86(1H,d,J=1.8Hz),8.72(1H,brs),8.51(1H,s),8.21(1H,d,J=8.7Hz ),8.20(1H,d,J=1 .8Hz),8.17(1H,s),7.69(1H,d,J=8.7Hz),7.04(2H,d,J=8.1Hz),6.53(2H,d,J=8.1Hz),5.17(2 H,s),5.10(2H,s),3.11-2.96(1H,m),1.33(6H,d,J=7.8Hz).
MSm/z(M+H):437.

<Example 0718>

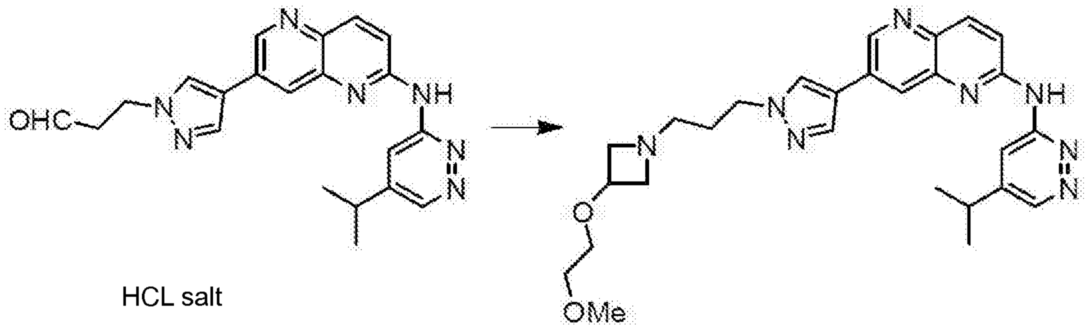

HCL salt

N-(5-isopropylpyridazin-3-yl)-7-(1-(3-(3-(2-methoxyethoxy)azetidin-1-yl)propyl)-1H-pyrazol-4-yl)-1,5-naphthyridine-2-amine was obtained as a pale yellow solid in the same manner as in Example 0426-2 except that 3-(2-methoxyethoxy)azetidine hydrochloride was used instead of the (2S,6R)-1,2,6-trimethylpiperazine trifluoroacetate used in Example 0426-2.
$^1$H-NMR(DMSO-$d_6$)δ:10.69(1H,s),9.04(1H,d,J=1.8Hz),8.86(1H,d,J=1.2Hz),8.73(1H,d,J=1.2Hz),8.49(1H,s),8.21(1H,d,J =9.0Hz),8.21(1H,d,J=1.8Hz),8.18(1H,s),7.70(1H,d,J=9.0H),4.17(2H,d,J=7.2Hz),4.11-3.97(1H,m),3.58-3.28(6H,m),3.22(3H,s),3.11-2.96(1H,m),2.73(2H,dd,J=6.0,2.1Hz),2.38(2H,d,J=6.6Hz),1.90-1.78(2H,m),1.33(6H,d,J=6.6Hz).
MSm/z(M+H):503.

<Example 0719>

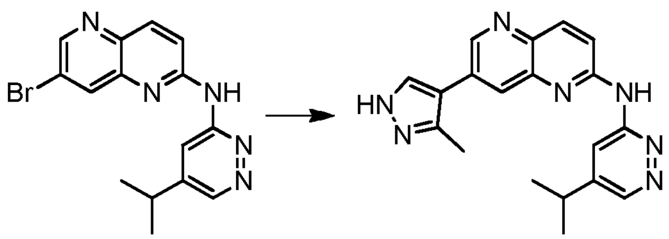

N-(5-isopropylpyridazin-3-yl)-7-(3-methyl-1H-pyrazol-4-yl)-1,5-naphthyridine-2-amine was obtained as a pale yellow solid in the same manner as in Example 0638-1 except that 3-methyl-4-(4,4,5,5-tetramethyl-1,3,2-dioxaborolan-2-yl)-1H-pyrazole was used instead of the 4,4,5,5-tetramethyl-2-vinyl-1,3,2-dioxaborolane used in Example 0638-1.
$^1$H-NMR(DMSO-$d_6$)δ:12.89(1H,brs),10.71(1H,s),8.93(1H,d,J=2.1Hz),8.85(1H,d,J=2.1Hz),8.73(1H,brs),8.24(1H,d,J=9.3 Hz),8.08(2H,brs),7.74(1H,d,J=9.3Hz),3.10-2.94(1H,m),2.50(3H,s),1.31(6H,d,J=7.2Hz).
MSm/z(M+H):346.

<Example 0720>

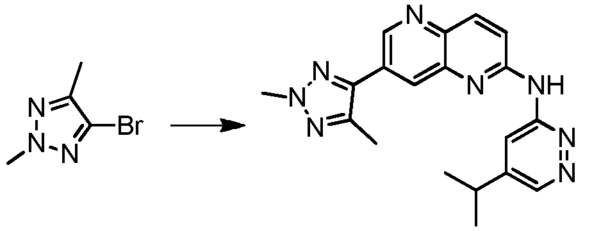

7-(2,5-Dimethyl-2H-1,2,3-triazol-4-yl)-N-(5-isopropylpyridazin-3-yl)-1,5-naphthyridine-2-amine was obtained as a pale yellow solid in the same manner as in Example 0421-1 except that 4-bromo-2,5-dimethyl-2H-1,2,3-triazole was used instead of the N-(4-bromopyridin-2-yl)acetamide used in Example 0421-1, and 7-bromo-N-(5-isopropylpyridazin-3-yl)-1,5-naphthyridine-2-amine was used instead of the 7-bromo-2-chloro-1,5-naphthyridine used in Example 0421-1.
$^1$H-NMR(DMSO-$d_6$)δ:10.80(1H,s),9.08(1H,d,J=2.1Hz),8.87(1H,d,J=2.1Hz),8.75(1H,brs),8.30(1H,brs),8.29(1H,d,J=8.1 Hz),7.81(1H,d,J=8.1Hz),4.20(3H,s),3.13-2.96(1H,m),2.54(3H,s),1.31(6H,d,J=7.2Hz).
MSm/z(M+H):361.

<Example 0721>

<0721-1>

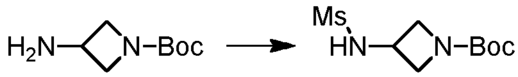

tert-Butyl 3-(methyl sulfonamide)azetidine-1-carboxylate was obtained in the same manner as in Example 0618-2 except that tert-butyl 3-aminoazetidine-1-carboxylate was used instead of the 5-bromo-1-(3-hydroxypropyl)pyridin-2(1H)-one used in Example 0618-2.
$^1$H-NMR(DMSO-$d_6$)δ:4.24-4.22(1H,m),4.13-4.07(3H,m),3.73-3.72(2H,m),2.89(3H,s),1.37(9H,s).

<0721-2>

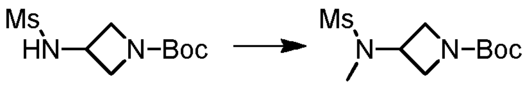

A mixture of tert-butyl 3-(methyl sulfonamide)azetidine-1-carboxylate (262 mg), iodomethane (98 μL), 60% sodium hydride (46 mg), and N,N-dimethylformamide (3 mL) was stirred at room temperature for 1 hour. After water and ethyl acetate were added to the reaction mixture, the organic layer was collected by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate-methanol), thereby obtaining tert-butyl 3-(N-methylmethyl sulfonamide)azetidine-1-carboxylate (214 mg).
$^1$H-NMR(DMSO-$d_6$)δ:4.51-4.46(1H,m),4.07-3.97(4H,m),2.86(3H,s),2.82(3H,s),1.38(9H,s).

<0721-3>

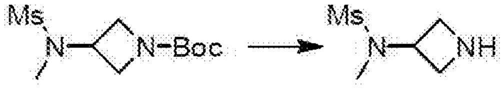

TFA salt

A mixture of tert-butyl 3-(N-methylmethyl sulfonamide)azetidine-1-carboxylate (214 mg), dichloromethane (4 mL), and trifluoroacetic acid (1.2 mL) was stirred at room temperature for 2 hours. The solvent was distilled off under reduced pressure, thereby obtaining N-(azetidin-3-yl)-N-methylmethane sulfonamide trifluoroacetate (347 mg).
$^1$H-NMR(DMSO-$d_6$)$\delta$:4.20-4.09(4H,m),3.44(1H,q,J=7.0Hz),2.94(3H,s),2.85(3H,s).

<0721-4>

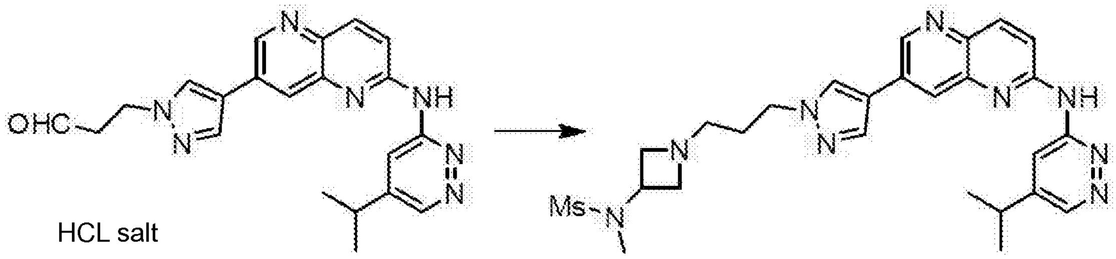

N-(1-(3-(4-(6-((5-isopropylpyridazin-3-yl)amino)-1,5-naphthyridin-3-yl)-1H-pyrazol-1-yl)propyl)azetidin-3-yl)-N-methylmethane sulfonamide was obtained as a pale yellow solid in the same manner as in Example 0426-2 except that N-(azetidin-3-yl)-N-methylmethane sulfonamide trifluoroacetate was used instead of the (2S,6R)-1,2,6-trimethylpiperazine trifluoroacetate used in Example 0426-2.
$^1$H-NMR(DMSO-$d_6$)$\delta$:10.70(1H,s),9.04(1H,d,J=2.1Hz),8.87(1H,d,J=2.1Hz),8.73(1H,brs),8.49(1H,s),8.31(1H,s),8.21(1H ,d,J=8.4Hz),8.18(1H,s),7.70(1H,d,J=8.4Hz),4.18(2H,t,J=7.2Hz),4.16-4.05(1H,m),3.46(2H,t,J=7.2Hz),3.09-2.96(3H,m),2.84(3H,s),2.79(3H,s),2.40(2H,t,J=7.2Hz),1.92-1.78(2H,m), 1.33(6H,d,J=7.2Hz).
MSm/z(M+H):536.

<Example 0722>

<0722-1>

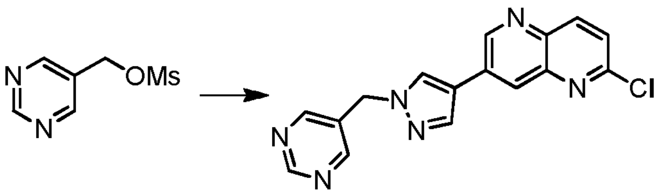

2-Chloro-7-(1-(pyrimidin-5-ylmethyl)-1H-pyrazol-4-yl)-1,5-naphthyridine was obtained in the same manner as in Example 0703-1 except that pyrimidin-5-ylmethyl methanesulfonate was used instead of the 1-(bromomethyl)-2-nitrobenzene used in Example 0703-1.
MSm/z(M+H):323.

<0722-2>

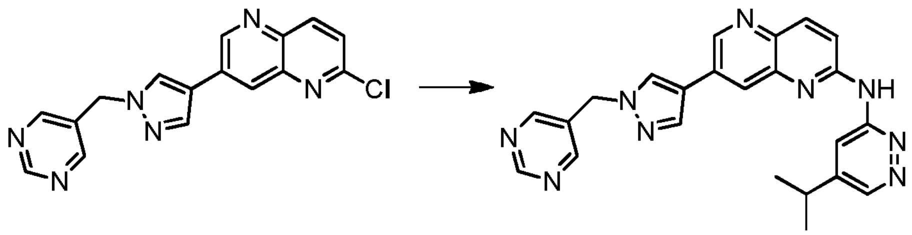

N-(5-isopropylpyridazin-3-yl)-7-(1-(pyrimidin-5-ylmethyl)-1H-pyrazol-4-yl)-1,5-naphthyridine-2-amine was obtained as a pale yellow solid in the same manner as in Example 0646-3 except that 2-chloro-7-(1-(pyrimidin-5-ylmethyl)-1H-pyrazol-4-yl)-1,5-naphthyridine was used instead of the 2-chloro-7-(1-(2,2-dimethoxypropyl)-1H-pyrazol-4-yl)-1,5-naphthyridine used in Example 0646-3.
$^1$H-NMR(DMSO-$d_6$)δ:10.70(1H,s),9.16(1H,s),9.05(1H,d,J=2.1Hz),8.87(1H,d,J=2.1Hz),8.83(2H,s),8.73(1H,d,J=2.1Hz), 8.67(1H,s),8.27(1H,s),8.24(1H,d,J=2.1Hz),8.22(1H,d,J=9.3Hz),7.71(1H,d,J=9.3Hz),5.52(2H,s),3.13-2.96(1H,m),1.33(6H,d,J=6.6Hz).
MSm/z(M+H):424.

<Example 0723>

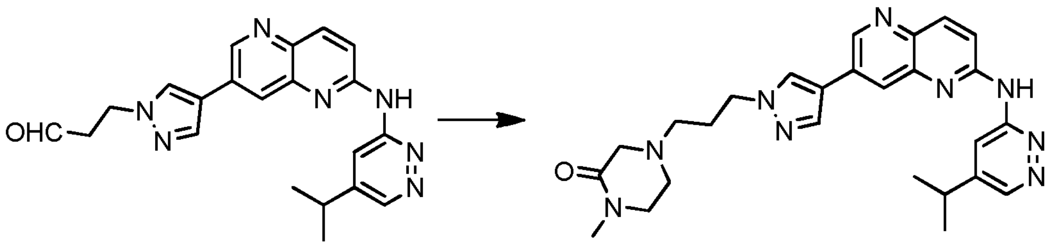

Triethylamine was added to a solution of hydrochloride (23 mg) of 1-methylpiperazin-2-one in dichloromethane (1 mL), followed by adjusting to pH 8. 3-(4-(6-((5-Isopropylpyridazin-3-yl)amino)-1,5-naphthyridin-3-yl)-1H-pyrazol-1-yl)propanal (19 mg), acetic acid (0.01 mL), and sodium triacetoxyborohydride (42 mg) were added thereto, followed by stirring at room temperature for 1 hour. The solvent was distilled off under reduced pressure, and the obtained residue was purified by silica gel column chromatography (ethyl acetate-methanol, NH silica), thereby obtaining 4-(3-(4-(6-((5-isopropylpyridazin-3-yl)amino)-1,5-naphthyridin-3-yl)-1H-pyrazol-1-yl)propyl)-1-methylpiperazin-2-one (4.4 mg) as a white solid.
$^1$H-NMR(DMSO-$d_6$)δ:10.70(1H,s),9.05(1H,d,J=1.8Hz),8.86(1H,d,J=2.1Hz),8.72(1H,s),8.52(1H,s),8.24-8.18(3H,m),7.70(1H,d,J=9.3Hz), 4.24-4.17(2H,m),3.30-3.25(2H,m),3.09-2.98(1H,m),2.87-2.83(2H,m),2.80(3H,s),2.66-2.60(2H,m),2.40-2.32(2H,m),2.07-1.98(2H,m),1.34(6H,d,J=7.2Hz).
MSm/z(M+H):486.

<Example 0724>

<0724-1>

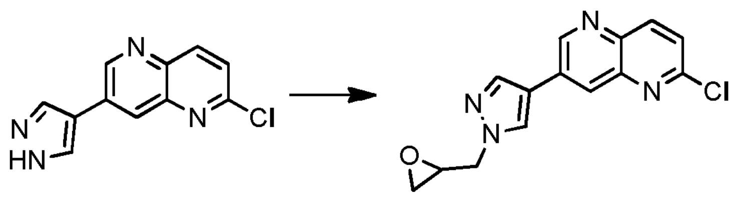

A suspension of 2-chloro-7-(1H-pyrazol-4-yl)-1,5-naphthyridine (460 mg), 2-(bromomethyl)oxirane (0.825 mL), and cesium carbonate (651 mg) in N,N-dimethylformamide (3 mL) was stirred at 50°C for 1 hour. After the reaction mixture was cooled to room temperature, ethyl acetate and water were added thereto. The organic layer was collected by separation, washed with a saturated sodium chloride aqueous solution, and dried over anhydrous sodium sulfate, and the solvent was distilled off under reduced pressure, thereby obtaining 2-chloro-7-(1-(oxiran-2-ylmethyl)-1H-pyrazol-4-yl)-1,5-naphthyridine (561 mg) as pale yellow oily substance.
MSm/z(M+H):287.

<0724-2>

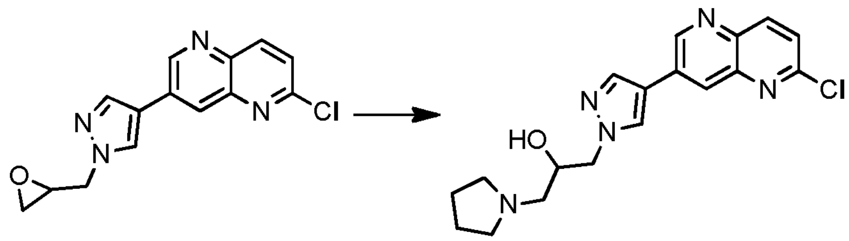

A solution of 2-chloro-7-(1-(oxiran-2-ylmethyl)-1H-pyrazol-4-yl)-1,5-naphthyridine (561 mg), and pyrrolidine (0.41 mL) in 1,4-dioxane (3 mL) was stirred at 70°C for 1 hour. The solvent was distilled off under reduced pressure, and the obtained residue was purified by silica gel column chromatography (hexane-ethyl acetate, NH silica), thereby obtaining 1-(4-(6-chloro-1,5-naphthyridin-3-yl)-1H-pyrazol-1-yl)-3-(pyrrolidin-1-yl)propan-2-ol (192 mg) as a white solid.
MSm/z(M+H):358.

<0724-3>

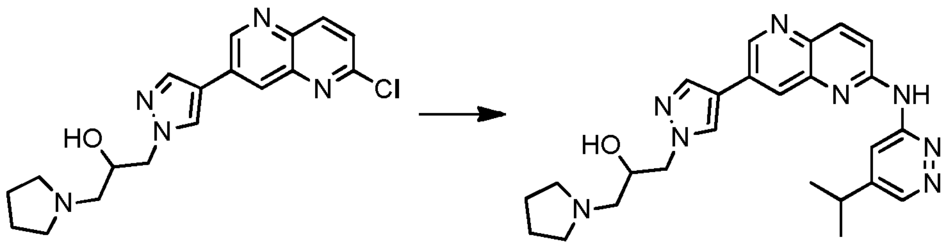

1-(4-(6-((5-Isopropylpyridazin-3-yl)amino)-1,5-naphthyridin-3-yl)-1H-pyrazol-1-yl)-3-(pyrrolidin-1-yl)propan-2-ol was obtained as a white solid in the same manner as in Example 0485-2 except that 1-(4-(6-chloro-1,5-naphthyridin-3-yl)-1H-pyrazol-1-yl)-3-(pyrrolidin-1-yl)propan-2-ol was used instead of the tert-butyl 4-((4-(6-chloro-1,5-naphthyridin-3-yl)-1H-pyrazol-1-yl)methyl)piperidine-1-carboxylate used in Example 0485-2.
$^1$H-NMR(DMSO-$d_6$)$\delta$:10.70(1H,s),9.06(1H,d,J=1.8Hz),8.86(1H,d,J=1.8Hz),8.74(1H,s),8.45(1H,s),8.24-8.18(3H,m),7.70(1H,d,J=9.3Hz), 5.04-4.90(1H,m),4.34-4.28(1H,m),4.10-3.90(2H,m),3.48-3.31(2H,m),3.09-2.98(1H,m),2.46-2.37(4H,m),1.72-1.51(4H, m),1.34(6H,d,J=7.2Hz).
MSm/z(M+H):459.

<Example 0725>

<0725-1>

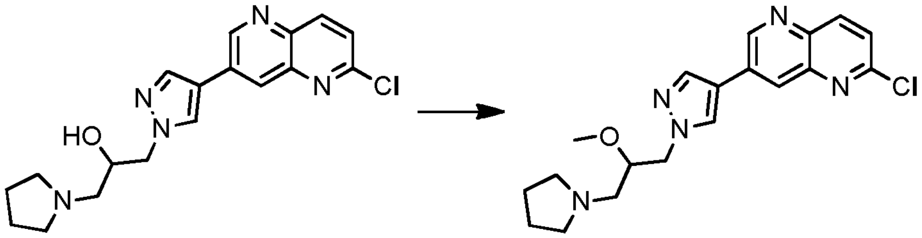

A suspension of 1-(4-(6-chloro-1,5-naphthyridin-3-yl)-1H-pyrazol-1-yl)-3-(pyrrolidin-1-yl)propan-2-ol (72 mg), and 60% sodium hydride (9.6 mg) in tetrahydrofuran (0.3 mL) was stirred at room temperature for 5 minutes. Methyl iodide (0.015 mL) was added thereto, followed by stirring at room temperature for 1 hour. The solvent was distilled off under reduced pressure, and the obtained residue was purified by silica gel column chromatography (ethyl acetate-hexane, NH silica), thereby obtaining 2-chloro-7-(1-(2-methoxy-3-(pyrrolidin-1-yl)propyl)-1H-pyrazol-4-yl)-1,5-naphthyridine (44 mg) as colorless oily substance.
MSm/z(M+H):372.

<0725-2>

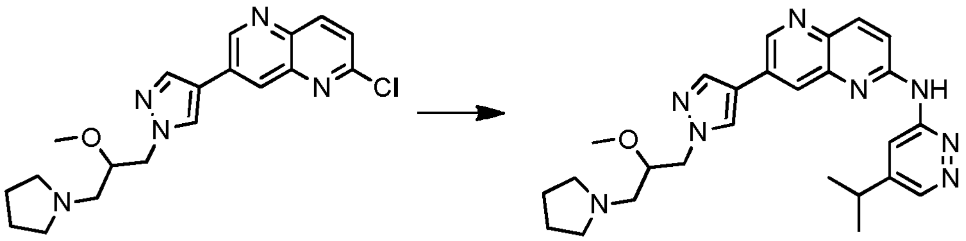

N-(5-isopropylpyridazin-3-yl)-7-(1-(2-methoxy-3-(pyrrolidin-1-yl)propyl)-1H-pyrazol-4-yl)-1,5-naphthyridine-2-amine was obtained as a white solid in the same manner as in Example 0485 except that 2-chloro-7-(1-(2-methoxy-3-(pyrrolidin-1-yl)propyl)-1H-pyrazol-4-yl)-1,5-naphthyridine was used instead of the tert-butyl 4-((4-(6-chloro-1,5-naphthyridin-3-yl)-1H-pyrazol-1-yl)methyl)piperidine-1-carboxylate used in Example 0485.
$^1$H-NMR(DMSO-$d_6$)δ:10.71(1H,s),9.06(1H,d,J=1.8Hz),8.86(1H,d,J=1.8Hz),8.74(1H,s),8.47(1H,s),8.24-8.19(3H,m),7.71(1H,d,J=9.3Hz),4.41-4.33(1H,m),4.24-4.51(1H,m),3.77-3.71(1H,m),3.48-3.31(2H,m),3.23(3H,s),3.09-2.99(1H,m),2.62-2.39(4H,m),1.72-1.52(4H,m),1.34(6H,d,J=7.2Hz).
MSm/z(M+H):473.

<Example 0726>

<0726-1>

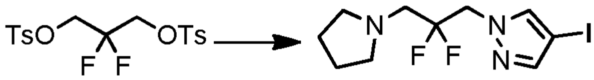

A suspension of (2,2-difluoropropane-1,3-diyl) bis(paratoluenesulfonate) (1.01 g), 4-iodo-1H-pyrazole (388 mg), and cesium carbonate (782 mg) in N,N-dimethylformamide (2 mL) was stirred at 70°C for 1.5 hours. After the reaction mixture was cooled to room temperature, ethyl acetate and water were added thereto. The organic layer was collected by separation, washed with a saturated sodium chloride aqueous solution, and dried over anhydrous sodium sulfate, and the solvent was distilled off under reduced pressure. Pyrrolidine (1.5 mL) was added to the obtained residue, followed by stirring at 100°C for 5 hours in a sealed tube. The solvent was distilled off under reduced pressure, and the obtained residue was purified by silica gel column chromatography (ethyl acetate-hexane, NH silica), thereby obtaining 1-(2,2-difluoro-3-(pyrrolidin-1-yl)propyl)-4-iodo-1H-pyrazole (240 mg) as colorless oily substance.

MSm/z(M+H):342.

<0726-2>

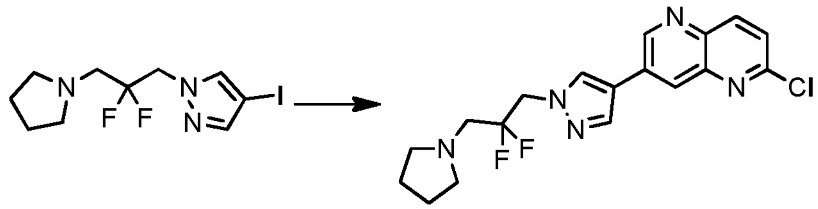

2-Chloro-7-(1-(2,2-difluoro-3-(pyrrolidin-1-yl)propyl)-1H-pyrazol-4-yl)-1,5-naphthyridine was obtained as a white solid in the same manner as in Example 0490 except that 1-(2,2-difluoro-3-(pyrrolidin-1-yl)propyl)-4-iodo-1H-pyrazole was used instead of the tert-butyl 3-(4-bromo-1H-pyrazol-1-yl)azetidine-1-carboxylate used in Example 0490.
MSm/z(M+H):378.

<0726-3>

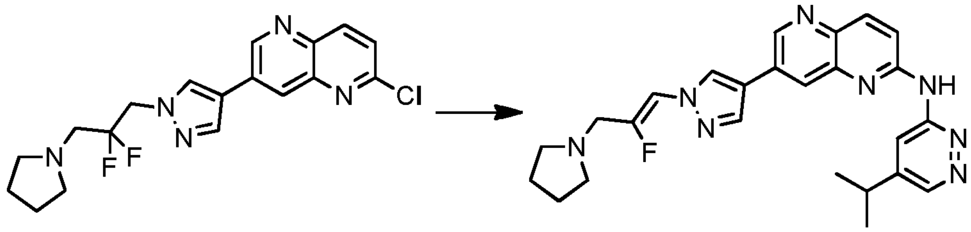

A suspension of 2-chloro-7-(1-(2,2-difluoro-3-(pyrrolidin-1-yl)propyl)-1H-pyrazol-4-yl)-1,5-naphthyridine (76 mg), 5-isopropylpyridazine-3-amine (55 mg), and potassium tert-butoxide (49 mg) in 1,4-dioxane (0.5 mL) was stirred at 120°C for 4 hours in a sealed tube. The insolubles were filtered off, and the solvent was distilled off under reduced pressure. The obtained residue was purified by preparative thin layer silica gel chromatography (ethyl acetate-methanol), thereby obtaining (Z)-7-(1-(2-fluoro-3-(pyrrolidin-1-yl)-1-propen-1-yl)-1H-pyrazol-4-yl)-N-(5-isopropylpyridazin-3-yl)-1,5-naphthyridine-2-amine (7.9 mg) as a white solid.
$^1$H-NMR(DMSO-$d_6$)$\delta$:10.74(1H,s),9.16(1H,d,J=2.1Hz),8.89-8.83(2H,m),8.74(1H,s),8.50(1H,s),8.39(1H,d,J=1.5Hz),8.25(1H,d,J=9.3Hz),7.73(1H,d,J=9.3Hz),7.25( 1H,d,J=28.2Hz),4.09-3.97(2H,m),3.70-3.60(2H,m),3.15-3.00(3H,m),1.94-1.88(4H,m),1.33(6H,d,J=7.2Hz).
MSm/z(M+H):459.

<Example 0727>

<0727-1>

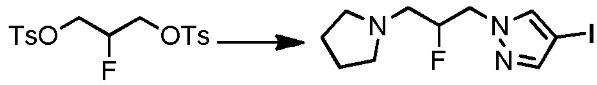

1-(2-Fluoro-3-(pyrrolidin-1-yl)propyl)-4-iodo-1H-pyrazole was obtained as colorless oily substance in the same manner as in Example 0726 except that (2-fluoropropane-1,3-diyl) bis(paratoluenesulfonate) was used instead of the (2,2-difluoropropane-1,3-diyl) bis(paratoluenesulfonate) in Example 0726.
MSm/z(M+H):324.

<0727-2>

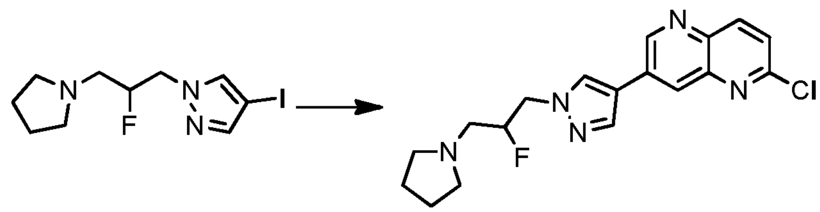

2-Chloro-7-(1-(2-fluoro-3-(pyrrolidin-1-yl)propyl)-1H-pyrazol-4-yl)-1,5-naphthyridine was obtained as a white solid in the same manner as in Example 0490 except that 1-(2-fluoro-3-(pyrrolidin-1-yl)propyl)-4-iodo-1H-pyrazole was used instead of the tert-butyl 3-(4-bromo-1H-pyrazol-1-yl)azetidine-1-carboxylate used in Example 0490.
MSm/z(M+H):360.

<0727-3>

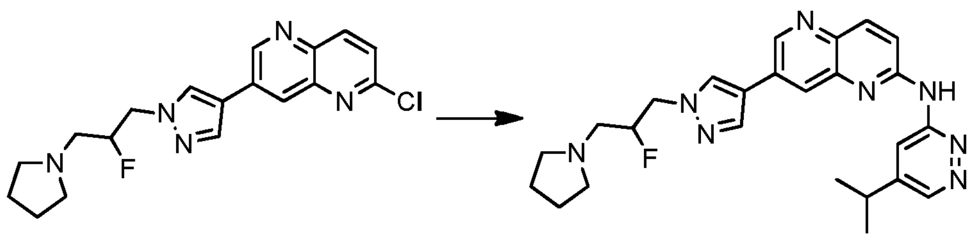

7-(1-(2-Fluoro-3-(pyrrolidin-1-yl)propyl)-1H-pyrazol-4-yl)-N-(5-isopropylpyridazin-3-yl)-1,5-naphthyridine-2-amine was obtained as a white solid in the same manner as in Example 0485 except that 2-chloro-7-(1-(2-fluoro-3-(pyrrolidin-1-yl)propyl)-1H-pyrazol-4-yl)-1,5-naphthyridine was used instead of the tert-butyl 4-((4-(6-chloro-1,5-naphthyridin-3-yl)-1H-pyrazol-1-yl)methyl)piperidine-1-carboxylate used in Example 0485.
$^1$H-NMR(DMSO-$d_6$)δ:10.71(1H,s),9.06(1H,d,J=2.1Hz),8.87(1H,d,J=1.8Hz),8.74(1H,s),8.55(1H,s),8.28-8.20(3H,m),7.71(1H,d,J=9.3Hz), 5.17(1H,d,J=48.9Hz),4.62-4.44(2H,m),3.14-2.73(7H,m),1.90-1.71(4H,m), 1.34(6H,d,J=7.2Hz).
MSm/z(M+H):461.

<Example 0728>

<0728-1>

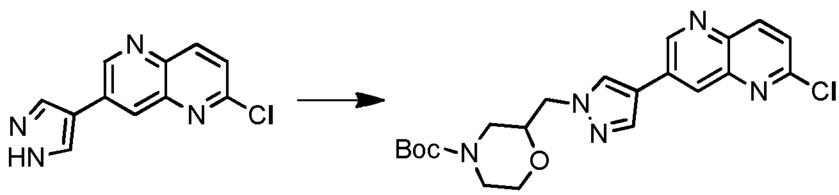

A suspension of 2-chloro-7-(1H-pyrazol-4-yl)-1,5-naphthyridine (231 mg), tert-butyl 2-((paratoluenesulfonyloxy)methyl)morpholine-4-carboxylate (371 mg), and potassium carbonate (166 mg) in N,N-dimethylformamide (1 mL) was stirred at 90°C for 2 hours. After the reaction mixture was cooled to room temperature, ethyl acetate and water were added thereto. The organic layer was collected by separation, washed with a saturated sodium chloride aqueous solution, and dried over anhydrous sodium sulfate, and the solvent was distilled off under reduced pressure. The obtained residue

was purified by silica gel column chromatography (ethyl acetate-methanol), thereby obtaining tert-butyl 2-((4-(6-chloro-1,5-naphthyridin-3-yl)-1H-pyrazol-1-yl)methyl)morpholine-4-carboxylate (360 mg) as colorless oily substance. MSm/z(M+H):430.

<0728-2>

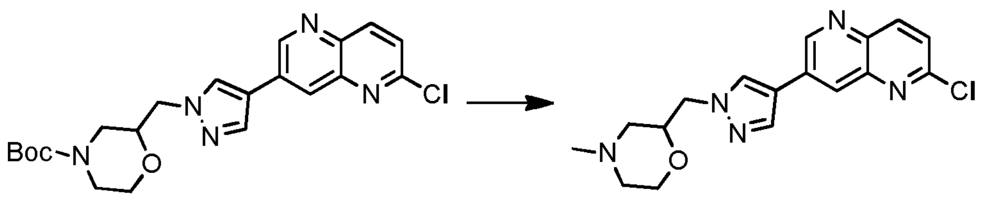

A4 mol/L hydrogen chloride/1,4-dioxane solution (1 mL) and methanol (0.2 mL) were added to tert-butyl 2-((4-(6-chloro-1,5-naphthyridin-3-yl)-1H-pyrazol-1-yl)methyl)morpholine-4-carboxylate (360 mg), followed by stirring at room temperature for 1 hour, and the solvent was distilled off under reduced pressure. Triethylamine was added to a solution of the obtained residue in dichloromethane (1 mL), followed by adjusting to pH 8. A 37% (w/w) formaldehyde aqueous solution (0.2 mL), acetic acid (0.01 mL), and sodium triacetoxyborohydride (212 mg) was added thereto, followed by stirring at room temperature for 1 hour. The solvent was distilled off under reduced pressure, and the obtained residue was purified by silica gel column chromatography (ethyl acetate-methanol, NH silica), thereby obtaining 2-((4-(6-chloro-1,5-naphthyridin-3-yl)-1H-pyrazol-1-yl)methyl)-4-methylmorpholine (280 mg) as colorless oily substance.
MSm/z(M+H):344.

<0728-3>

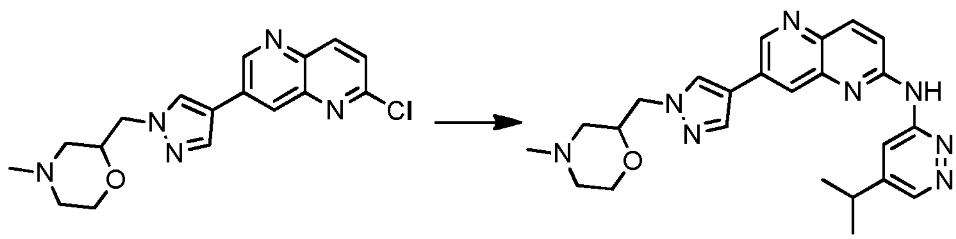

N-(5-isopropylpyridazin-3-yl)-7-(1-((4-methylmorpholin-2-yl)methyl)-1H-pyrazol-4-yl)-1,5-naphthyridine-2-amine was obtained as a white solid in the same manner as in Example 0726 except that 2-((4-(6-chloro-1,5-naphthyridin-3-yl)-1H-pyrazol-1-yl)methyl)-4-methylmorpholine was used instead of the 2-chloro-7-(1-(2,2-difluoro-3-(pyrrolidin-1-yl)propyl)-1H-pyrazol-4-yl)-1,5-naphthyridine used in Example 0726.
$^1$H-NMR(DMSO-$d_6$)δ:10.71(1H,s),9.05(1H,d,J=2.1Hz),8.87(1H,d,J=1.8Hz),8.74(1H,s),8.48(1H,s),8.24-8.20(3H,m),7.71(1H,d,J=9.3Hz),4.24(2H,d,J=5.7Hz),3.92-3.77(2H,m),3.53-3.43(1H,m),3.11-2.90(1H,m),2.73-2.56(2H,m),2.18(3H,s),2.02-1.94(1H,m)1.83-1.72(1H,m),1.34(6H,d,J=7.2Hz).
MSm/z(M+H):445.

<Example 0729>

<0729-1>

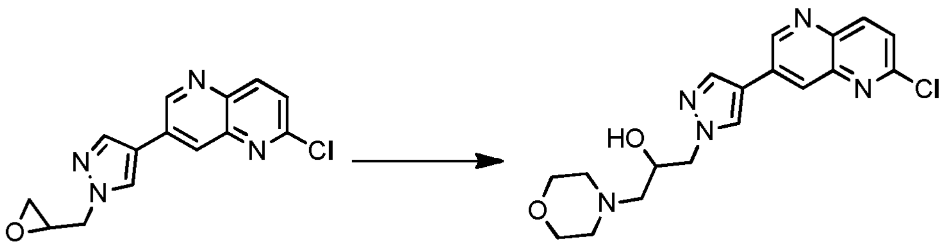

1-(4-(6-Chloro-1 ,5-naphthyridin-3-yl)-1 H-pyrazol-1 -yl)-3-morpholinopropan-2-ol was obtained as a white solid in the same manner as in Example 0724-2 except that morpholine was used instead of the pyrrolidine used in Example 0724-2.
MSm/z(M+H):374.

<0729-2>

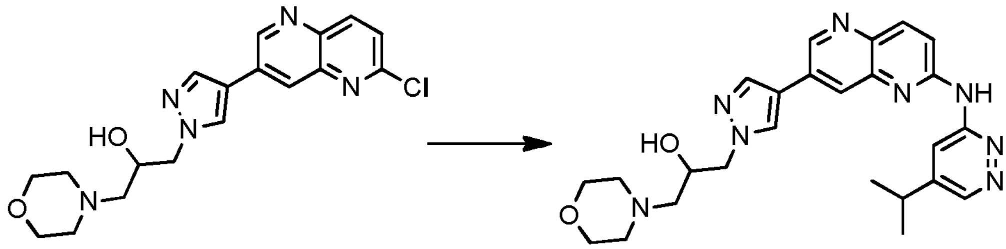

5-Isopropylpyridazine-3-amine (27 mg), cesium carbonate (65 mg), and ((2-dicyclohexylphosphino-3,6-dimethoxy-2',4',6'-triisopropyl-1,1'-biphenyl)-2-(2'-amino-1,1'-biphenyl))palladium(II) methanesulfonate (BRETTPHOS-PD-G3, (product name, manufactured by Sigma-Aldrich Co. LLC.)) (9 mg) were added to a solution of 1-(4-(6-chloro-1,5-naphthyridin-3-yl)-1H-pyrazol-1-yl)-3-morpholinopropan-2-ol (37 mg) in 1,4-dioxane (0.5 mL), followed by stirring at 110°C for 3 hours. Furthermore, 5-isopropylpyridazine-3-amine (27 mg) and ((2-dicyclohexylphosphino-3,6-dimethoxy-2',4',6'-triisopropyl-1,1'-biphenyl)-2-(2'-amino-1,1'-biphenyl))palladium(II) methanesulfonate (BRETTPHOS-PD-G3, (product name, manufactured by Sigma-Aldrich Co. LLC.)) (9 mg) were added thereto, followed by stirring at 110°C for 5 hours. The reaction mixture was cooled to room temperature, the solvent was distilled off under reduced pressure, and the obtained residue was purified by silica gel column chromatography (ethyl acetate-methanol, NH silica), thereby obtaining 1-(4-(6-((5-isopropylpyridazin-3-yl)amino)-1,5-naphthyridin-3-yl)-1H-pyrazol-1-yl)-3-morpholinopropan-2-ol (32 mg) as a pale yellow solid.
$^1$H-NMR($CDCl_3$)$\delta$:9.39(1H,s),8.94(1H,d,J=2.0Hz),8.91(1H,d,J=2.0Hz),8.83(1H,d,J=2.0Hz),8.25(1H,d,J=9.2Hz),8.12(1H,d,J=2.0Hz),8.01(1H,s),7.98(1H,s),7.65(1H,d,J=9.2Hz),4.39(1H,d,J=10.6Hz),4.25-4.11(2H,m),3.80-3.67(5H,m),3.12-3.03(1H,m),2.69-2.62(2H,m),2.51-2.32(4H,m),1.43(6H,d,J=6.6Hz).
MSm/z(M+H):475.

<Example 0730>

<0730-1>

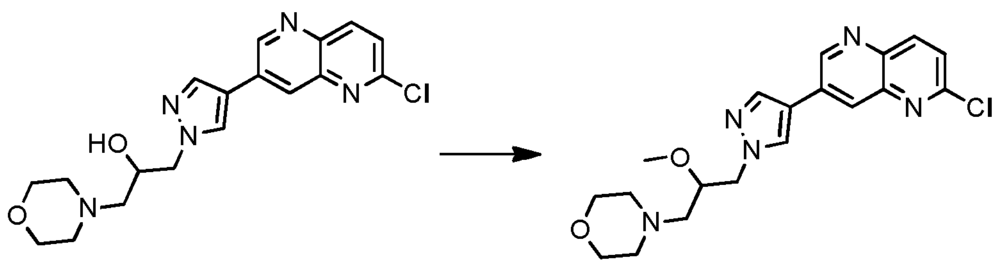

4-(3-(4-(6-Chloro-1,5-naphthyridin-3-yl)-1H-pyrazol-1-yl)-2-methoxypropyl)morpholine was obtained as a pale yellow solid in the same manner as in Example 0725-1 except that 1-(4-(6-chloro-1,5-naphthyridin-3-yl)-1H-pyrazol-1-yl)-3-morpholinopropan-2-ol was used instead of the 1-(4-(6-chloro-1,5-naphthyridin-3-yl)-1H-pyrazol-1-yl)-3-(pyrrolidin-1-yl)propan-2-ol used in Example 0725-1.
MSm/z(M+H):388.

<0730-2>

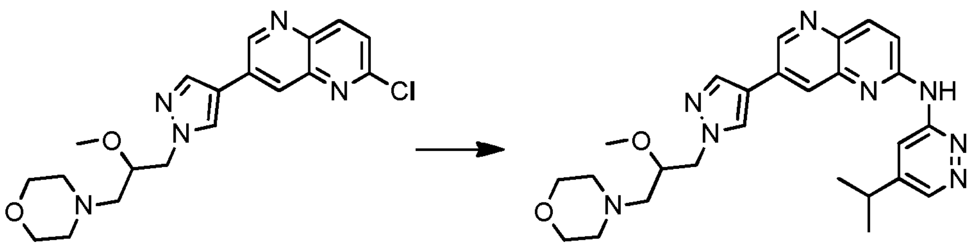

N-(5-isopropylpyridazin-3-yl)-7-(1-(2-methoxy-3-morpholinopropyl)-1H-pyrazol-4-yl)-1,5-naphthyridine-2-amine was obtained as a red solid in the same manner as in Example 0729-2 except that 4-(3-(4-(6-chloro-1,5-naphthyridin-3-yl)-1H-pyrazol-1-yl)-2-methoxypropyl)morpholine was used instead of the 1-(4-(6-chloro-1,5-naphthyridin-3-yl)-1H-pyrazol-1-yl)-3-morpholinopropan-2-ol used in Example 0729-2.
$^1$H-NMR($CDCl_3$)6:8.94-8.89(2H,m),8.84(1H,s),8.81(1H,s),8.25(1H,d,J=8.6Hz),8.11(1H,s),7.98(1H,s),7.95(1H,s),7.53(1H,d,J=8.6Hz),4.50(1H,dd,J=14.0,3.6Hz),4.23(1H,dd,J=14.0,7.3Hz),3.83-3.70(5H,m),3.36(3H,s),3.12-3.01(1H,m),2.58-2.42(6H,m),1.42(6H,d,J=6.6Hz).
MSm/z(M+H):489.

<Example 0731>

<0731-1>

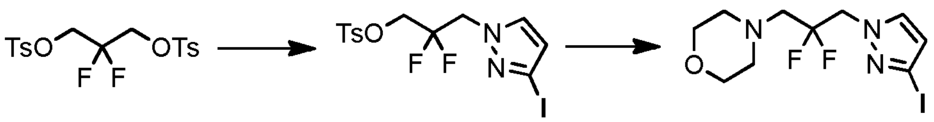

Cesium carbonate (163 mg), N,N-dimethylformamide (1 mL), and 3-iodo-1H-pyrazole (155 mg) were added to (2,2-difluoropropane-1,3-diyl) bis(4-methylbenzenesulfonate) (420 mg), followed by stirring at 70°C for 4 hours. The reaction mixture was cooled to room temperature, and water and ethyl acetate were added thereto. The organic layer was collected by separation, washed with a saturated sodium chloride aqueous solution, and dried over anhydrous sodium sulfate, and the solvent was distilled off under reduced pressure. Morpholine (1.5 mL) was added to the obtained residue, followed by stirring at 100°C for 4 hours. The reaction mixture was cooled to room temperature, the solvent was distilled off under reduced pressure, and the obtained residue was purified by silica gel column chromatography (hexane-ethyl acetate, NH silica), thereby obtaining 4-(2,2-difluoro-3-(3-iodo-1H-pyrazol-1-yl)propyl)morpholine (268 mg) as colorless oily substance.
MSm/z(M+H):358.

<0731-2>

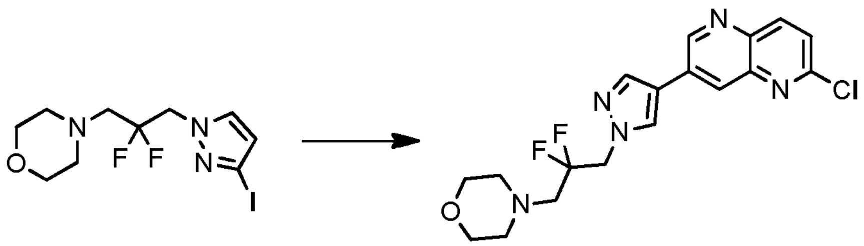

4-(3-(4-(6-Chloro-1,5-naphthyridin-3-yl)-1H-pyrazol-1-yl)-2,2-difluoropropyl)morpholine was obtained as a pale yellow solid in the same manner as in Example 0385-7 except that 4-(2,2-difluoro-3-(3-iodo-1H-pyrazol-1-yl)propyl)morpholine was used instead of the 4-(3-(3-ethyl-4-iodo-1H-pyrazol-1-yl)propyl)morpholine used in Example 0385-7.
MSm/z(M+H):394.

<0731-3>

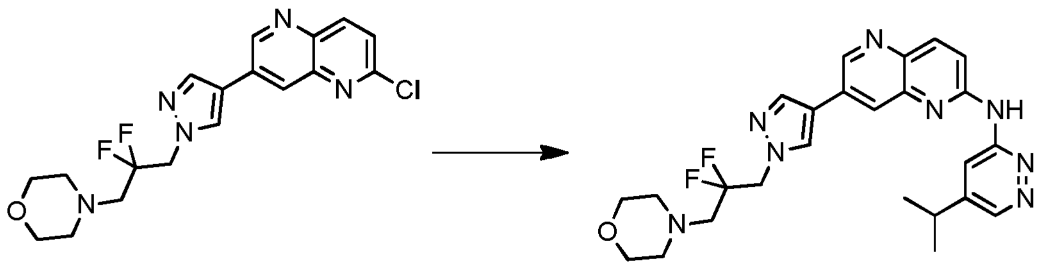

7-(1-(2,2-Difluoro-3-morpholinopropyl)-1H-pyrazol-4-yl)-N-(5-isopropylpyridazin-3-yl)-1,5-naphthyridine-2-amine was obtained as a white solid in the same manner as in Example 0729-2 except that 4-(3-(4-(6-chloro-1,5-naphthyridin-3-yl)-1H-pyrazol-1-yl)-2,2-difluoropropyl)morpholine was used instead of the 1-(4-(6-chloro-1,5-naphthyridin-3-yl)-1H-pyrazol-1-yl)-3-morpholinopropan-2-ol used in Example 0729-2.
$^1$H-NMR($CDCl_3$)δ:8.93-8.80(3H,m),8.25(1H,d,J=9.2Hz),8.11(1H,d,J=1.3Hz),8.00(1H,s),7.97(1H,s),7.65(1H,s),4.74(2H,t,J=12 .6Hz),3.75(4H,t,J=4.6Hz),3.11-3.01(1H,m),2.76-2.65(6H,m),1.42(6H,d,J=6.6Hz).
MSm/z(M+H):495.

<Example 0732>

<0732-1>

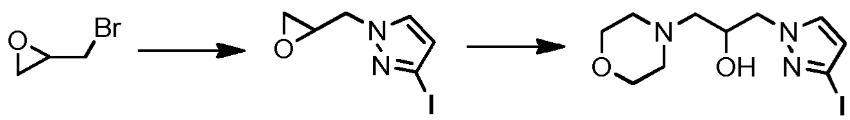

Cesium carbonate (13 g), epibromohydrin (4.92 mL), and N,N-dimethylformamide (15 mL) were added to 3-iodo-1H-pyrazole (3.88 g), followed by stirring at 50°C for 1 hour. The reaction mixture was cooled to room temperature, and water and ethyl acetate were added thereto. The organic layer was collected by separation, washed with a saturated sodium chloride aqueous solution, and dried over anhydrous sodium sulfate, and the solvent was distilled off under reduced pressure.

1,4-Dioxane (20 mL) and morpholine (2.6 mL) were added to the obtained residue, followed by stirring at 90°C for 1 hour. The reaction mixture was cooled to room temperature, the solvent was distilled off under reduced pressure, and the obtained residue was purified by silica gel column chromatography (hexane-ethyl acetate-methanol), thereby obtaining 1-(3-iodo-1H-pyrazol-1-yl)-3-morpholinopropan-2-ol (2.0 g) as a white solid.
MSm/z(M+H):338.

<0732-2>

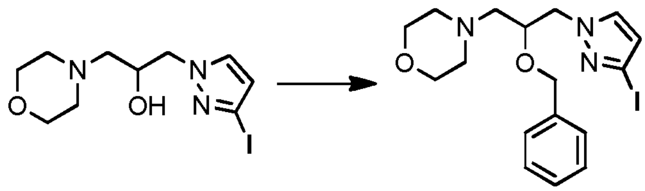

60% sodium hydride (14 mg) was added to a solution of 1-(3-iodo-1H-pyrazol-1-yl)-3-morpholinopropan-2-ol (100 mg) in N,N-dimethylformamide (1 mL) under ice-cooling, followed by stirring for 1 hour. Benzyl bromide (53 μL) was added to the reaction mixture, followed by stirring at room temperature for 1.5 hours. After water was added to the reaction mixture, the resultant product was extracted with ethyl acetate, and the organic layer was washed with a saturated sodium chloride aqueous solution, and dried over anhydrous sodium sulfate. The solvent was distilled off under reduced pressure, and the obtained residue was purified by silica gel column chromatography (hexane-ethyl acetate), thereby obtaining 4-(2-(benzyloxy)-3-(3-iodo-1H-pyrazol-1-yl)propyl)morpholine (60 mg) as colorless oily substance.
MSm/z(M+H):428.

<0732-3>

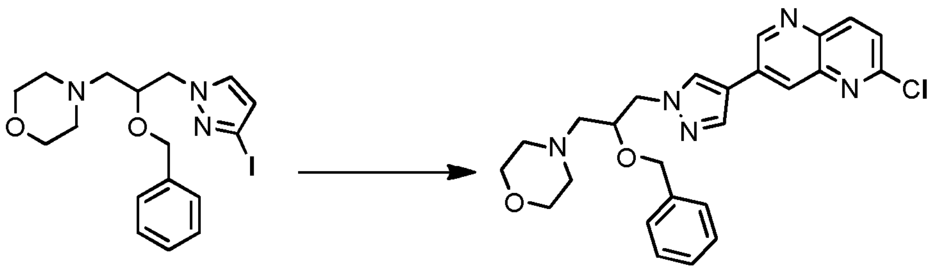

4-(2-(Benzyloxy)-3-(4-(6-chloro-1,5-naphthyridin-3-yl)-1H-pyrazol-1-yl)propyl)morpholine was obtained as a pale yellow solid in the same manner as in Example 0385-7 except that 4-(2-(benzyloxy)-3-(3-iodo-1H-pyrazol-1-yl)propyl)morpholine was used instead of the 4-(3-(3-ethyl-4-iodo-1H-pyrazol-1-yl)propyl)morpholine used in Example 0385-7.
MSm/z(M+H):464.

<0732-4>

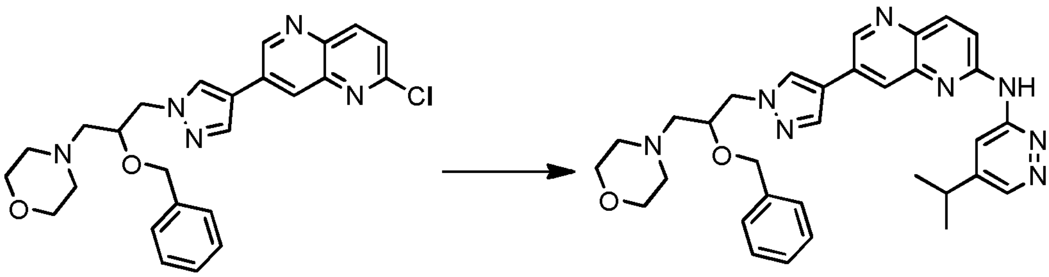

7-(1-(2-(Benzyloxy)-3-morpholinopropyl)-1H-pyrazol-4-yl)-N-(5-isopropylpyridazin-3-yl)-was obtained as a white solid in the same manner as in Example 0729-2 except that 4-(2-(benzyloxy)-3-(4-(6-chloro-1,5-naphthyridin-3-yl)-1H-pyrazol-1-yl)propyl)morpholine was used instead of the 1-(4-(6-chloro-1,5-naphthyridin-3-yl)-1H-pyrazol-1-yl)-3-morpholinopropan-2-ol used in Example 0729-2.

$^1$H-NMR($CDCl_3$)δ:9.12(1H,s),8.91(1H,d,J=2.0Hz),8.88(1H,s),8.83(1H,s),8.26(1H,d,J=8.6Hz),8.11(1H,d,J =1.3Hz),7.98(1H,s),7.92(1H,s),7.61-7.59(1H,m),7.23-7.19(5H,m),4.59-4.50(2H,m),4.36(1H,d,J=11.9Hz),4.23(1H,dd,J =14.5,7.9Hz),4.05-4.02(1H,m),3.72(4H,t,J=4.6Hz),3.11-3.02(1H,m),2.56-2.48(6H,m),1.42(6H,d,J=7.3Hz).
MSm/z(M+H):565.

<Example 0733>

<0733-1>

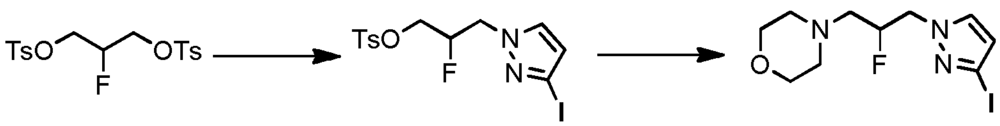

4-(2-Fluoro-3-(3-iodo-1H-pyrazol-1-yl)propyl)morpholine was obtained as a brown solid in the same manner as in Example 0731-1 except that (2-fluoropropane-1,3-diyl) bis(4-methylbenzenesulfonate) was used instead of the 2,2-difluoropropane-1,3-diyl bis(4-methylbenzenesulfonate) in Example 0731-1.
MSm/z(M+H):340.

<0733-2>

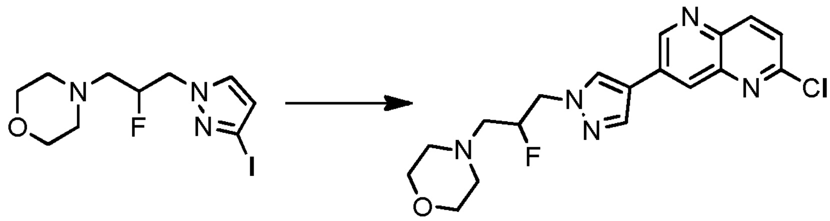

4-(3-(4-(6-Chloro-1,5-naphthyridin-3-yl)-1H-pyrazol-1-yl)-2-fluoropropyl)morpholine was obtained as a yellow solid in the same manner as in Example 0385-7 except that 4-(2-fluoro-3-(3-iodo-1H-pyrazol-1-yl)propyl)morpholine was used instead of the 4-(3-(3-ethyl-4-iodo-1H-pyrazol-1-yl)propyl)morpholine used in Example 0385-7.
MSm/z(M+H):376.

<0733-3>

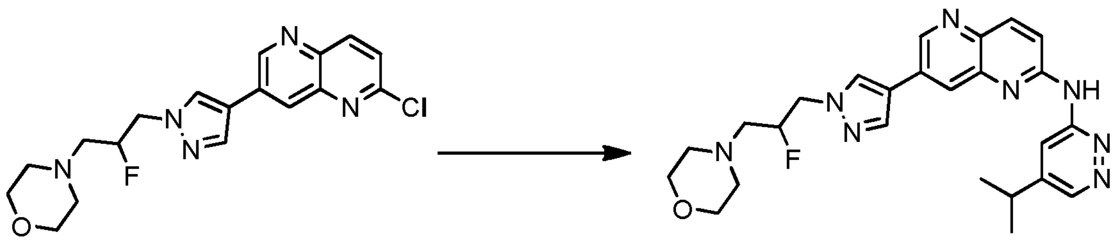

7-(1-(2-Fluoro-3-morpholinopropyl)-1H-pyrazol-4-yl)-N-(5-isopropylpyridazin-3-yl)-1,5-naphthyridine-2-amine was obtained as a white solid in the same manner as in Example 0729-2 except that 4-(3-(4-(6-chloro-1,5-naphthyridin-3-yl)-1H-pyrazol-1-yl)-2-fluoropropyl)morpholine was used instead of the 1-(4-(6-chloro-1,5-naphthyridin-3-yl)-1H-pyrazol-1-yl)-3-morpholinopropan-2-ol used in Example 0729-2.
$^1$H-NMR($CDCl_3$)δ:9.22(1H,s),8.93(1H,d,J=2.0Hz),8.88(1H,s),8.83(1H,s),8.25(1H,d,J=8.6Hz),8.12(1H,d,J =2.0Hz),8.00(1H,s),7.95(1H,s),7.62(1H,d,J=8.6Hz),5.08(1H,d,J=48.2Hz),4.65-4.42(2H,m),3.74(4H,t,J=4.6Hz),3.08-3.

06(1H,m),2.70(1H,t,J=5.0Hz),2.61-2.57(5H,m),1.43(6H,d,J=6.6Hz).
MSm/z(M+H):477.

<Example 0734>

<0734-1>

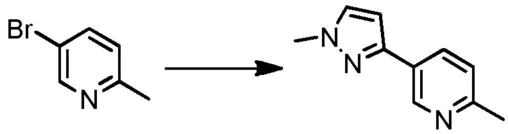

A mixture of 5-bromo-2-methylpyridine (258 mg), bis(pinacolato)diboron (396 mg), potassium acetate (282 mg), (1,1'-bis(diphenylphosphino)ferrocene)palladium(II) dichloride-dichloromethane adduct (49 mg), and 1,4-dioxane (5 mL) was stirred at 100°C for 2.5 hours. 3-Bromo-1-methyl-1H-pyrazole (200 mg), sodium carbonate (254 mg), bis(di-tert-butyl (4-dimethylaminophenyl)phosphine)dichloropalladium(II) (42 mg), and water (0.5 mL) were added thereto, followed by stirring at 100°C for 6 hours. The reaction mixture was cooled to room temperature, the solvent was distilled off under reduced pressure, and the obtained residue was purified by silica gel column chromatography (hexane-ethyl acetate, NH silica), thereby obtaining 2-methyl-5-(1-methyl-1H-pyrazol-3-yl)pyridine (190 mg) as yellow oily substance.
MSm/z(M+H):174.

<0734-2>

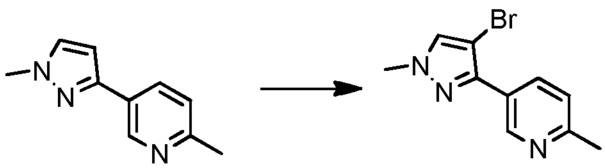

N-bromosuccinimide (129 mg) was added to a solution of 2-methyl-5-(1-methyl-1H-pyrazol-3-yl)pyridine (190 mg) in N,N-dimethylformamide (1 mL) under ice-cooling, followed by stirring at the same temperature for 0.5 hours. After ethyl acetate and a 10% sodium hydrogen sulfite aqueous solution were added to the obtained reaction mixture, the organic layer was collected by separation, washed with a saturated sodium chloride aqueous solution, and dried over anhydrous sodium sulfate. The solvent was distilled off under reduced pressure, and the obtained residue was purified by silica gel column chromatography (ethyl acetate-hexane), thereby obtaining 5-(4-bromo-1-methyl-1H-pyrazol-3-yl)-2-methylpyridine (160 mg) as yellow oily substance.
MSm/z(M+H):252.

<0734-3>

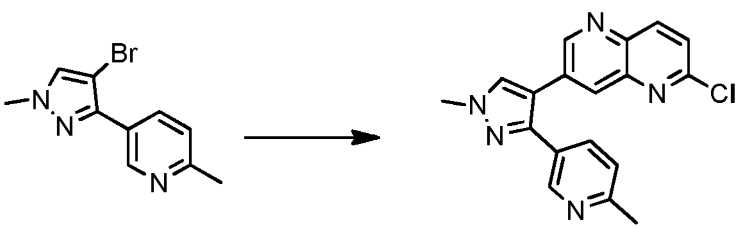

2-Chloro-7-(1-methyl-3-(6-methylpyridin-3-yl)-1H-pyrazol-4-yl)-1,5-naphthyridine was obtained as a white solid

in the same manner as in Example 0385-7 except that 5-(4-bromo-1-methyl-1H-pyrazol-3-yl)-2-methylpyridine was used instead of the 4-(3-(3-ethyl-4-iodo-1H-pyrazol-1-yl)propyl)morpholine used in Example 0385-7.
MSm/z(M+H):336.

<0734-4>

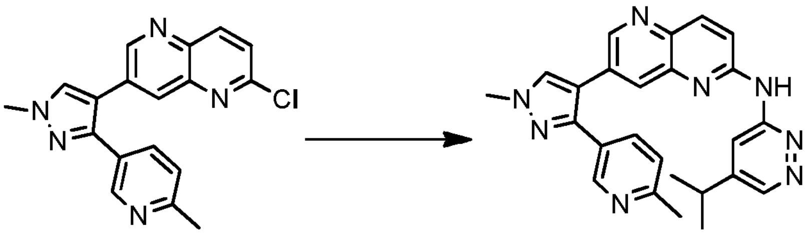

N-(5-isopropylpyridazin-3-yl)-7-(1-methyl-3-(6-methylpyridin-3-yl)-1H-pyrazol-4-yl)-1,5-naphthyridine-2-amine was obtained as a white solid in the same manner as in Example 0729-2 except that 2-chloro-7-(1-methyl-3-(6-methylpyridin-3-yl)-1H-pyrazol-4-yl)-1,5-naphthyridine was used instead of the 1-(4-(6-chloro-1,5-naphthyridin-3-yl)-1H-pyrazol-1-yl)-3-morpholinopropan-2-ol used in Example 0729-2.
$^1$H-NMR($CDCl_3$)δ:9.78(1H,s),8.90(1H,s),8.80(1H,d,J=2.0Hz),8.66(2H,s),8.24(1H,d,J=8.6Hz),7.94(1H,d,J =2.0Hz),7.77-7.67(3H,m),7.13(1H,d,J=8.6Hz),4.07(3H,s),3.04-2.95(1H,m),2.56(3H,s),1.34(6H,d,J=7.3Hz).
MSm/z(M+H):437.

<Example 0735>

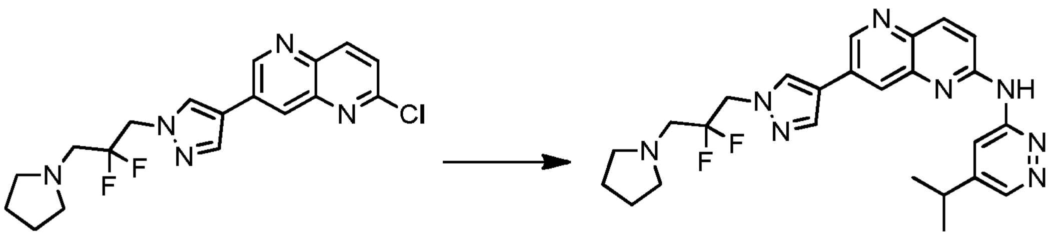

7-(1-(2,2-Difluoro-3-(pyrrolidin-1-yl)propyl)-1H-pyrazol-4-yl)-N-(5-isopropylpyridazin-3-yl)-1,5-naphthyridine-2-amine was obtained as a white solid in the same manner as in Example 0729-2 except that 2-chloro-7-(1-(2,2-difluoro-3-(pyrrolidin-1-yl)propyl)-1H-pyrazol-4-yl)-1,5-naphthyridine was used instead of the 1-(4-(6-chloro-1,5-naphthyridin-3-yl)-1H-pyrazol-1-yl)-3-morpholinopropan-2-ol used in Example 0729-2.
$^1$H-NMR($CDCl_3$)δ:8.94(1H,s),8.82(2H,s),8.72(1H,s),8.25(1H,d,J=8.9Hz),8.12(1H,s),8.01(1H,s),7.98(1H,s ),7.52(1H,d,J=8 .9Hz),4.72(2H,t,J=12.9Hz),3.06(1H,s),2.87(2H,t,J=13.9Hz),2.69(4H,t,J=3.6Hz),1.82(4 H,t,J=3.6Hz),1.42(6H,d,J=6.6Hz).
MSm/z(M+H):479.

<Example 0736>

<0736-1>

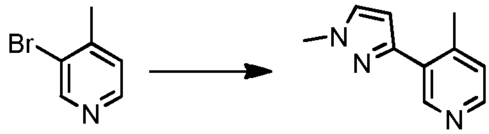

4-Methyl-3-(1-methyl-1H-pyrazol-3-yl)pyridine was obtained as yellow oily substance in the same manner as in Example 0734-1 except that 3-bromo-4-methylpyridine was used instead of the 5-bromo-2-methylpyridine used in Example 0734-1.
MSm/z(M+H):174.

<0736-2>

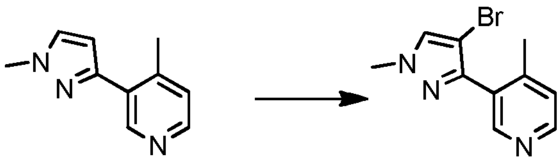

3-(4-Bromo-1-methyl-1H-pyrazol-3-yl)-4-methylpyridine was obtained as pale brown oily substance in the same manner as in Example 0734-2 except that 4-methyl-3-(1-methyl-1H-pyrazol-3-yl)pyridine was used instead of the 2-methyl-5-(1-methyl-1H-pyrazol-3-yl)pyridine used in Example 0734-2.
MSm/z(M+H):254.

<0736-3>

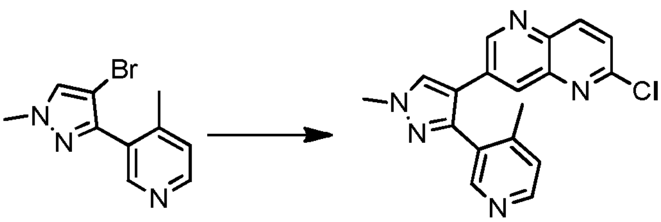

2-Chloro-7-(1-methyl-3-(4-methylpyridin-3-yl)-1H-pyrazol-4-yl)-1,5-naphthyridine was obtained as a yellow solid in the same manner as in Example 0385-7 except that 3-(4-bromo-1-methyl-1H-pyrazol-3-yl)-4-methylpyridine was used instead of the 4-(3-(3-ethyl-4-iodo-1H-pyrazol-1-yl)propyl)morpholine used in Example 0385-7.
MSm/z(M+H):336.

<0736-4>

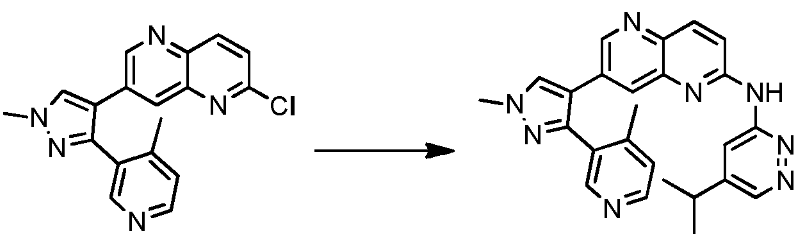

N-(5-isopropylpyridazin-3-yl)-7-(1-methyl-3-(4-methylpyridin-3-yl)-1H-pyrazol-4-yl)-1,5-naphthyridine-2-amine

was obtained as a pale red solid in the same manner as in Example 0729-2 except that 2-chloro-7-(1-methyl-3-(4-methylpyridin-3-yl)-1H-pyrazol-4-yl)-1,5-naphthyridine was used instead of the 1-(4-(6-chloro-1,5-naphthyridin-3-yl)-1H-pyrazol-1-yl)-3-morpholinopropan-2-ol used in Example 0729-2.
$^1$H-NMR($CDCl_3$)δ:9.73(1H,s),8.83(1H,s),8.79(1H,s),8.64(1H,s),8.57(1H,s),8.50(1H,d,J=4.6Hz),8.19(1H, d,J=9.5Hz),7.86(1H,s),7.68(1H,d,J=9.5Hz),7.67(1H,s),7.17(1H,d,J=4.6Hz),4.09(3H,s),3.04-2.95(1H,m),2.14(3H,s),1.36(6H,d,J=6.6Hz).
MSm/z(M+H):437.

<Example 0737>

<0737-1>

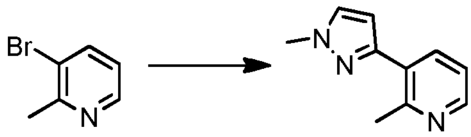

2-Methyl-3-(1-methyl-1H-pyrazol-3-yl)pyridine was obtained as colorless oily substance in the same manner as in Example 0734-1 except that 3-bromo-2-methylpyridine was used instead of the 5-bromo-2-methylpyridine used in Example 0734-1.
MSm/z(M+H):174.

<0737-2>

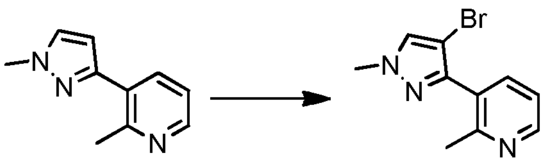

3-(4-Bromo-1-methyl-1H-pyrazol-3-yl)-2-methylpyridine was obtained as colorless oily substance in the same manner as in Example 0734-2 except that 2-methyl-3-(1-methyl-1H-pyrazol-3-yl)pyridine was used instead of the 2-methyl-5-(1-methyl-1H-pyrazol-3-yl)pyridine used in Example 0734-2.
MSm/z(M+H):254.

<0737-3>

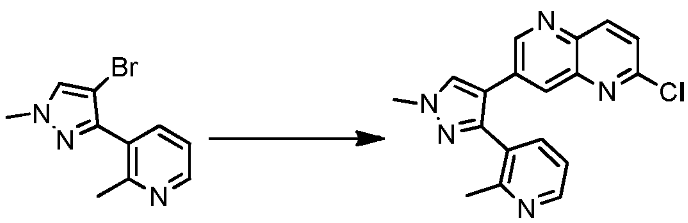

2-Chloro-7-(1-methyl-3-(2-methylpyridin-3-yl)-1H-pyrazol-4-yl)-1,5-naphthyridine was obtained as a yellow solid in the same manner as in Example 0385-7 except that 3-(4-bromo-1-methyl-1H-pyrazol-3-yl)-2-methylpyridine was used instead of the 4-(3-(3-ethyl-4-iodo-1H-pyrazol-1-yl)propyl)morpholine used in Example 0385-7.
MSm/z(M+H):336.

<0737-4>

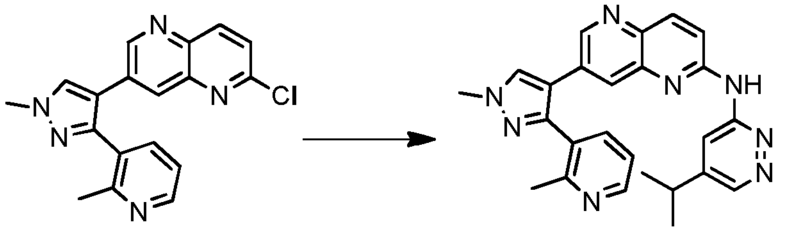

N-(5-isopropylpyridazin-3-yl)-7-(1-methyl-3-(2-methylpyridin-3-yl)-1H-pyrazol-4-yl)-1,5-naphthyridine-2-amine was obtained as a white solid in the same manner as in Example 0729-2 except that 2-chloro-7-(1-methyl-3-(2-methylpyridin-3-yl)-1H-pyrazol-4-yl)-1,5-naphthyridine was used instead of the 1-(4-(6-chloro-1,5-naphthyridin-3-yl)-1H-pyrazol-1-yl)-3-morpholinopropan-2-ol used in Example 0729-2.
$^1$H-NMR($CDCl_3$)$\delta$:9.37(1H,s),8.79(2H,s),8.65(1H,d,J=2.0Hz),8.56(1H,dd,J=4.8,2.0Hz),8.19(0H,d,J=9.2Hz),7.85(1H,s),7.69-7.66(2H,m),7.60(1H,d,J=9.2Hz),7.19(1H,dd,J=7.6,4.8Hz),4.08(3H,s),3.04-2.94(1H,m),2.36(3H,s),1.36(6H,d,J=7.3Hz).
MSm/z(M+H):437.

<Example 0738>

<0738-1>

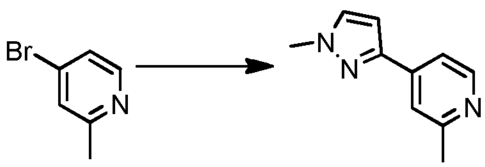

2-Methyl-4-(1-methyl-1H-pyrazol-3-yl)pyridine was obtained as pale yellow oily substance in the same manner as in Example 0734-1 except that 4-bromo-2-methylpyridine was used instead of the 5-bromo-2-methylpyridine used in Example 0734-1.
MSm/z(M+H):174.

<0738-2>

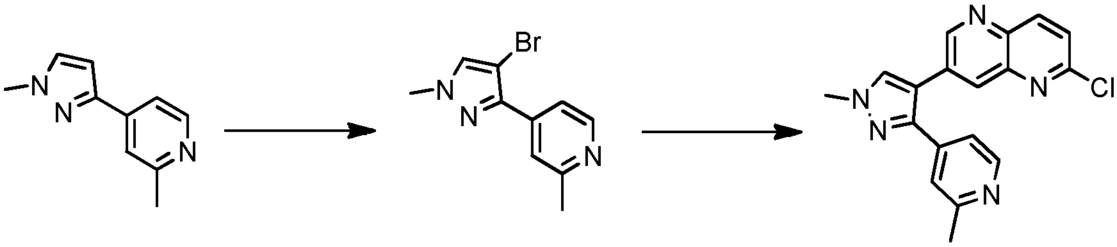

4-(4-Bromo-1-methyl-1H-pyrazol-3-yl)-2-methylpyridine was obtained as colorless oily substance in the same manner as in Example 0734-2 except that 2-methyl-4-(1-methyl-1H-pyrazol-3-yl)pyridine was used instead of the 2-methyl-5-(1-methyl-1H-pyrazol-3-yl)pyridine used in Example 0734-2.
2-Chloro-7-(1-methyl-3-(2-methylpyridin-4-yl)-1H-pyrazol-4-yl)-1,5-naphthyridine was obtained as a grey solid in the same manner as in Example 0385-7 except that 4-(4-bromo-1-methyl-1H-pyrazol-3-yl)-2-methylpyridine was used

instead of the 4-(3-(3-ethyl-4-iodo-1H-pyrazol-1-yl)propyl)morpholine used in Example 0385-7.
MSm/z(M+H):336.

<0738-3>

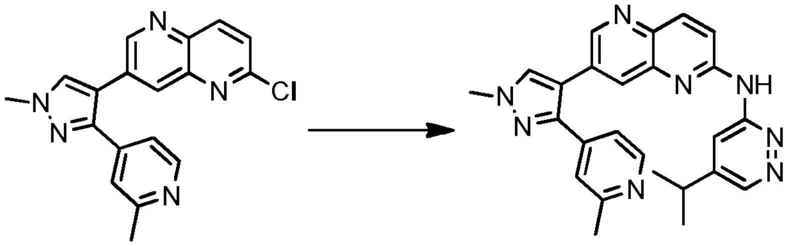

N-(5-isopropylpyridazin-3-yl)-7-(1-methyl-3-(2-methylpyridin-4-yl)-1H-pyrazol-4-yl)-1,5-naphthyridine-2-amine was obtained as a pale yellow solid in the same manner as in Example 0729-2 except that 2-chloro-7-(1-methyl-3-(2-methylpyridin-4-yl)-1H-pyrazol-4-yl)-1,5-naphthyridine was used instead of the 1-(4-(6-chloro-1,5-naphthyridin-3-yl)-1H-pyrazol-1-yl)-3-morpholinopropan-2-ol used in Example 0729-2.
$^1$H-NMR($CDCl_3$)δ:9.40(1H,s),8.84(1H,s),8.81(1H,s),8.68(1H,d,J=2.0Hz),8.42(1H,d,J=5.3Hz),8.27(1H,d,J =9.2Hz),7.97(1H,d,J=2.0Hz),7.71(1H,d,J=9.2Hz),7.69(1H,s),7.41(1H,s),7.16(1H,d,J=5.3Hz),4.07(3H, s),3.05-2.95(1H,m),2.53(3H,s),1.35(6H,d,J=7.3Hz).
MSm/z(M+H):437.

<Example 0739>

<0739-1>

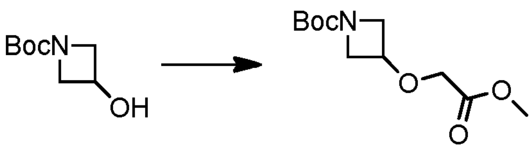

60% sodium hydride (144 mg) was added to a solution of tert-butyl 3-hydroxyazetidine-1-carboxylate (520 mg) in N,N-dimethylformamide (5 mL), followed by stirring at room temperature for 0.5 hours. 2-Methyl bromoacetate (389 μL) was added thereto, followed by stirring at room temperature for 1 hour, and stirring at 50°C for 3 hours. After water and ethyl acetate were added to the reaction mixture, the organic layer was collected by separation, washed with a saturated sodium chloride aqueous solution, and dried over anhydrous sodium sulfate. The solvent was distilled off under reduced pressure, and the obtained residue was purified by silica gel column chromatography (ethyl acetate-hexane), thereby obtaining tert-butyl 3-(2-methoxy-2-oxoethoxy)azetidine-1-carboxylate (570 mg) as colorless oily substance.

<0739-2>

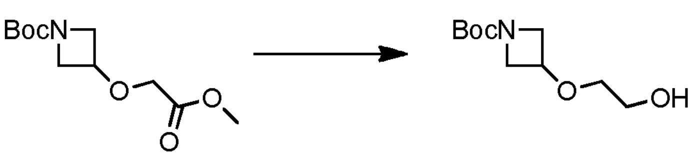

A3 mol/L lithium borohydride/tetrahydrofuran solution (1.4 mL) was added to a solution of tert-butyl 3-(2-methoxy-2-oxoethoxy)azetidine-1-carboxylate (500 mg) in tetrahydrofuran (10 mL), followed by stirring at room temperature

for 40 minutes. After water, a saturated citric acid aqueous solution, and ethyl acetate were added sequentially to the reaction mixture, the organic layer was collected by separation, washed with a saturated sodium chloride aqueous solution, and dried over anhydrous sodium sulfate. The solvent was distilled off under reduced pressure, thereby obtaining tert-butyl 3-(2-hydroxyethoxy)azetidine-1-carboxylate (281 mg) as colorless oily substance.

<0739-3>

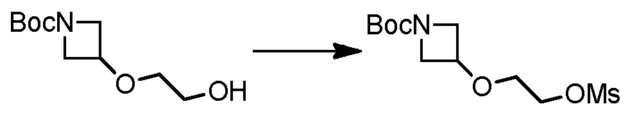

Triethylamine (345 μL) and methanesulfonyl chloride (192 μL) were added to a solution of tert-butyl 3-(2-hydroxyethoxy)azetidine-1-carboxylate (270 mg) in methylene chloride (6 mL) under ice-cooling, followed by stirring at the same temperature for 15 minutes, and stirring at 50°C for 15 minutes. The reaction mixture was purified by silica gel column chromatography (ethyl acetate-hexane), thereby obtaining tert-butyl 3-(2-((methylsulfonyl)oxy)ethoxy)azetidine-1-carboxylate (290 mg) as colorless oily substance.

<0739-4>

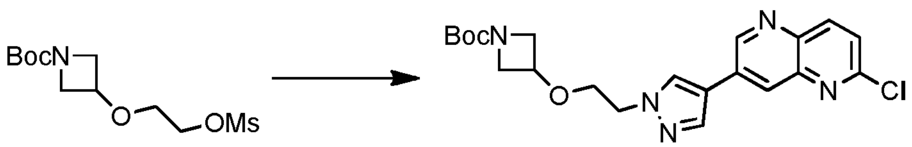

2-Chloro-7-(1H-pyrazol-4-yl)-1,5-naphthyridine (277 mg), cesium carbonate (391 mg), and N,N-dimethylformamide (3 mL) were added to tert-butyl 3-(2-((methylsulfonyl)oxy)ethoxy)azetidine-1-carboxylate (290 mg), followed by stirring at 60°C for 1.5 hours. After the reaction mixture was cooled to room temperature, water and ethyl acetate were added thereto, the organic layer was collected by separation, washed with a saturated sodium chloride aqueous solution, and dried over anhydrous sodium sulfate. The solvent was distilled off under reduced pressure, and the obtained residue was purified by silica gel column chromatography (ethyl acetate-hexane-methanol), thereby obtaining tert-butyl 3-(2-(4-(6-chloro-1,5-naphthyridin-3-yl)-1H-pyrazol-1 -yl)ethoxy)azetidine-1 - carboxylate (325 mg) as colorless oily substance.
MSm/z(M+H):430.

<0739-5>

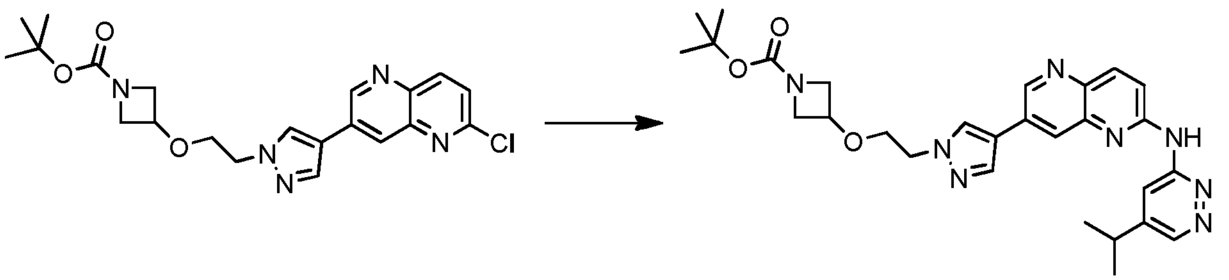

tert-Butyl 3-(2-(4-(6-((5-isopropylpyridazin-3-yl)amino)-1,5-naphthyridin-3-yl)-1H-pyrazol-1-yl)ethoxy)azetidine-1-carboxylate was obtained as a white solid in the same manner as in Example 0729-2 except that tert-butyl 3-(2-(4-(6-chloro-1,5-naphthyridin-3-yl)-1H-pyrazol-1-yl)ethoxy)azetidine-1-carboxylate was used instead of the 1-(4-(6-chloro-1,5-naphthyridin-3-yl)-1H-pyrazol-1-yl)-3-morpholinopropan-2-ol used in Example 0729-2.
$^1$H-NMR($CDCl_3$)δ:9.11(1H,s),8.94(1H,d,J=1.5Hz),8.87(1H,s),8.82(1H,s),8.25(1H,d,J=9.2Hz),8.12(1H,d,J

=1.5Hz),7.99(1H,s),7.93(1H,s),7.60(1H,d,J=9.2Hz),4.40(2H,t,J=5.3Hz),4.25-4.17(1H,m),4.06(1H,d,J=6.6Hz),4.03(1H,d,J=6.6Hz),3.84-3.75(4H,m),3.11-3.01(1H,m),1.42(6H,d,J=6.6Hz),1.40(9H,s).
MSm/z(M+H):531.

<Example 0740>

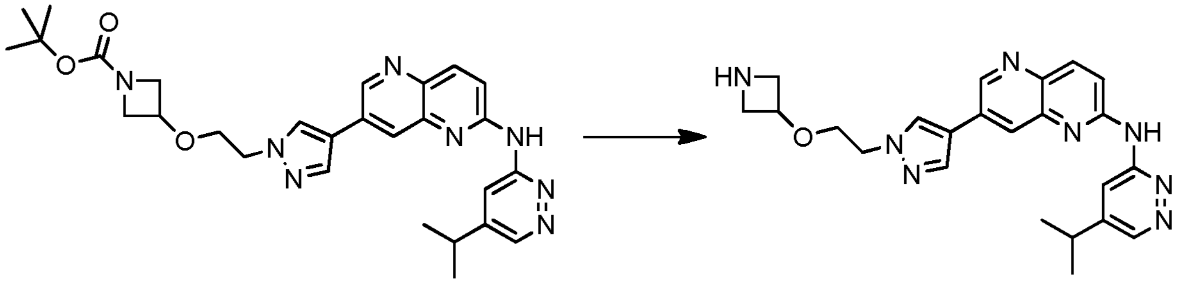

Trifluoroacetic acid (1 mL) was added to tert-butyl3-(2-(4-(6-((5-isopropylpyridazin-3-yl)amino)-1,5-naphthyridin-3-yl)-1H-pyrazol-1-yl)ethoxy)azetidine-1-carboxylate (50 mg), followed by stirring at room temperature for 20 minutes. The solvent was distilled off under reduced pressure, and the obtained residue was purified by silica gel column chromatography (ethyl acetate-methanol, NH silica), thereby obtaining 7-(1-(2-(azetidin-3-yloxy)ethyl)-1H-pyrazol-4-yl)-N-(5-isopropylpyridazin-3-yl)-1,5-naphthyridine-2-amine (8 mg) as a pale yellow solid.
$^1$H-NMR($CDCl_3$)δ:8.94(1H,s),8.82(2H,d,J=2.0Hz),8.74(1H,s),8.25(1H,d,J=9.2Hz),8.12(1H,d,J=2.0Hz),7.98(1H,s),7.96(1H,s),7.51(1H,d,J=9.2Hz),4.39(2H,t,J=5.0Hz),4.33-4.31(1H,m),3.80(2H,t,J=5.0Hz),3.70-3.64(2H,m),3.58-3.49(2H,m),3.10-3.01(1H,m),1.42(6H,d,J=7.3Hz).
MSm/z(M+H):431.

<Example 0741>

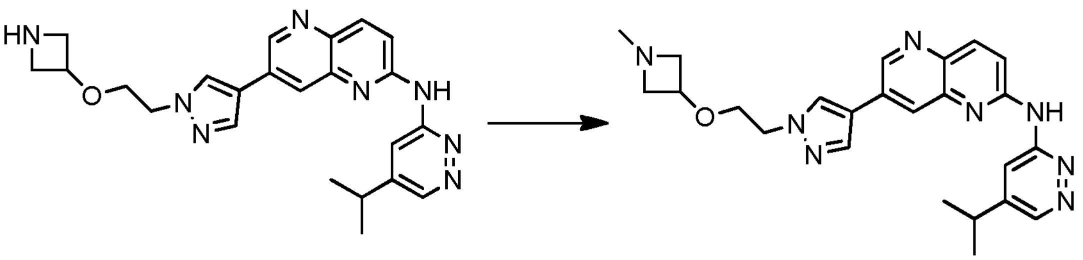

A 36.0% to 38.0% formaldehyde aqueous solution (3 μL) and sodium triacetoxyborohydride (6 mg) were added to a suspension of 7-(1-(2-(azetidin-3-yloxy)ethyl)-1H-pyrazol-4-yl)-N-(5-isopropylpyridazin-3-yl)-1,5-naphthyridine-2-amine (4 mg) in methylene chloride (0.3 mL), followed by stirring at room temperature for 20 minutes. Water was added to the reaction mixture, the solvent was distilled off under reduced pressure, and the obtained residue was purified by silica gel column chromatography (ethyl acetate-methanol, NH silica), thereby obtaining N-(5-isopropylpyridazin-3-yl)-7-(1-(2-((1-methylazetidin-3-yl)oxy)ethyl)-1H-pyrazol-4-yl)-1,5-naphthyridine-2-amine(3.74mg) as a white solid.
$^1$H-NMR($CDCl_3$)δ:10.02(1H,s),8.98(1H,s),8.93(1H,d,J=2.0Hz),8.83(1H,s),8.25(1H,d,J=8.3Hz),8.11(1H,d,J=2.0Hz),7.98(1H,s),7.95(1H,s),7.78(1H,d,J=8.3Hz),4.38(2H,t,J=5.3Hz),4.12-4.04(1H,m),3.79(2H,t,J=5.3Hz),3.59(2H,td,J=6.2,2.0Hz),3.12-3.03(1H,m),2.88(2H,td,J=6.2,2.0Hz),2.32(3H,s),1.43(6H,d,J=6.6Hz).
MSm/z(M+H):445.

<Example 0742>

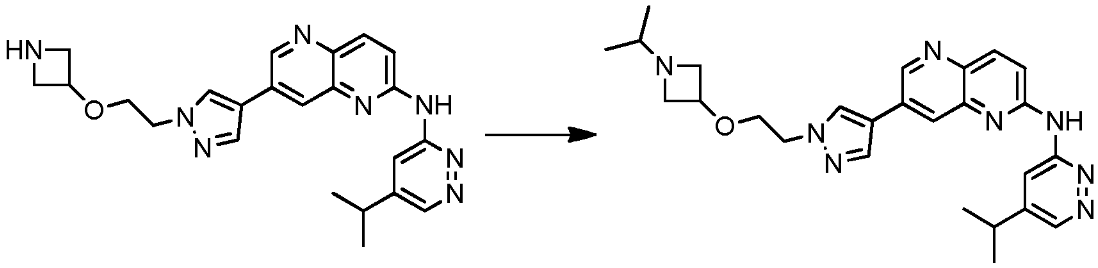

7-(1-(2-((1-Isopropylazetidin-3-yl)oxy)ethyl)-1H-pyrazol-4-yl)-N-(5-isopropylpyridazin-3-yl)-1,5-naphthyridine-2-amine was obtained as a white solid in the same manner as in Example 0741 except that acetone was used instead of the 36.0% to 38.0% formaldehyde aqueous solution used in Example 0741.
$^1$H-NMR($CDCl_3$)δ:9.23(1H,s),8.94(1H,d,J=2.0Hz),8.89(1H,s),8.83(1H,s),8.25(1H,d,J=9.2Hz),8.11(1H,d,J =2.0Hz),7.98(1H,s),7.95(1H,s),7.62(1H,d,J=9.2Hz),4.38(2H,t,J=5.0Hz),4.13-4.05(1H,m),3.80(2H,t,J=5.0Hz),3.62-3.53 (2H,m),3.11-3.01(1H,m),2.89-2.82(2H,m),2.33-2.25(1H,m),1.43(6H,d,J=6.6Hz),0.92(6H,d,J=5.9Hz).
MSm/z(M+H):473.

<Example 0743>

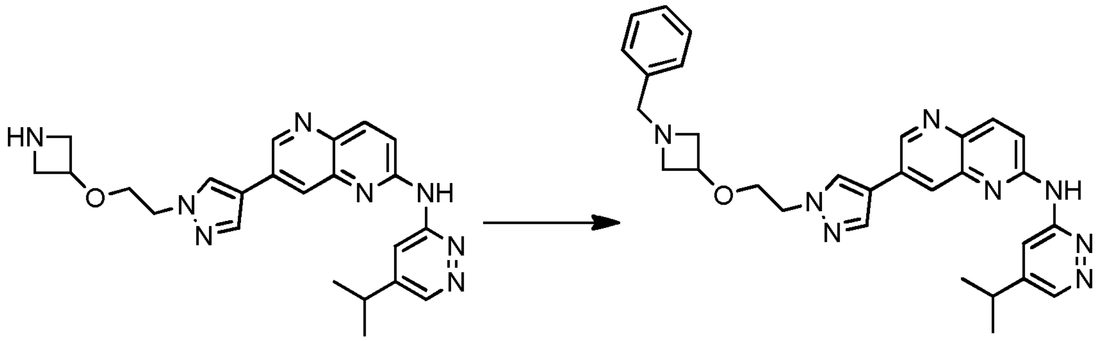

7-(1-(2-((1-Benzylazetidin-3-yl)oxy)ethyl)-1H-pyrazol-4-yl)-N-(5-isopropylpyridazin-3-yl)-was obtained as a white solid in the same manner as in Example 0741 except that benzaldehyde was used instead of the 36.0% to 38.0% formaldehyde aqueous solution used in Example 0741.
$^1$H-NMR($CDCl_3$)δ:9.46(1H,s),8.94(1H,s),8.92(1H,s),8.83(1H,s),8.25(1H,d,J=9.2Hz),8.11(1H,s),7.98(1H,s ),7.95(1H,s),7.66(1H,d,J=9.2Hz),7.25-7.19(5H,m),4.38(2H,t,J=5.1Hz),4.18-4.10(1H,m),3.79(2H,t,J=5.1Hz),3.59-3.55(4H,m),3.11-3.01 (1H,m),2.95-2.89(2H,m),1.42(6H,d,J=6.6Hz).
MSm/z(M+H):521.

<Example 0744>

<0744-1>

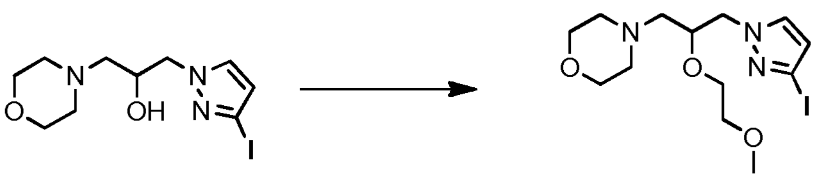

4-(3-(3-Iodo-1H-pyrazol-1-yl)-2-(2-methoxyethoxy)propyl)morpholine was obtained in the same manner as in Example 0732-2 except that 2-bromoethyl methyl ether was used instead of the benzyl bromide used in Example 0732-2.

MSm/z(M+H):396.

<0744-2>

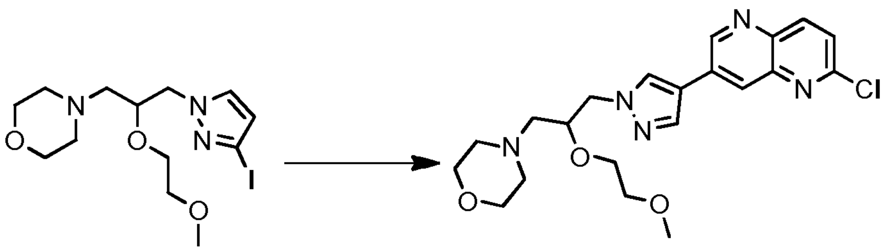

4-(3-(4-(6-Chloro-1,5-naphthyridin-3-yl)-1H-pyrazol-1-yl)-2-(2-methoxyethoxy)propyl)morpholine was obtained in the same manner as in Example 0385-7 except that 4-(3-(3-iodo-1H-pyrazol-1-yl)-2-(2-methoxyethoxy)propyl)morpholine was used instead of the 4-(3-(3-ethyl-4-iodo-1H-pyrazol-1-yl)propyl)morpholine used in Example 0385-7. MSm/z(M+H):432.

<0744-3>

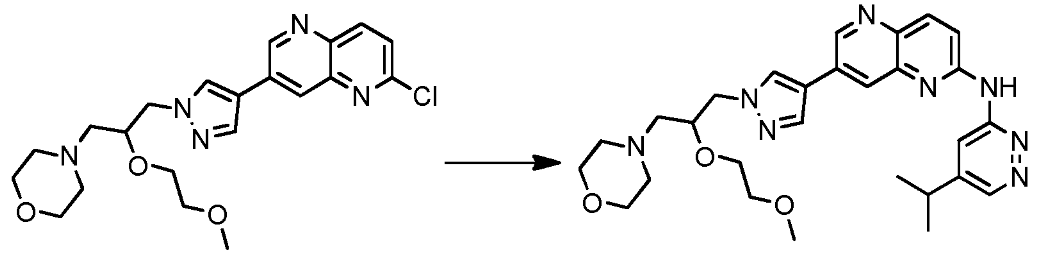

N-(5-isopropylpyridazin-3-yl)-7-(1-(2-(2-methoxyethoxy)-3-morpholinopropyl)-1H-pyrazol-4-yl)-1,5-naphthyridine-2-amine was obtained as a pale yellow solid in the same manner as in Example 0729-2 except that 4-(3-(4-(6-chloro-1,5-naphthyridin-3-yl)-1H-pyrazol-1-yl)-2-(2-methoxyethoxy)propyl)morpholine was used instead of the 1-(4-(6-chloro-1,5-naphthyridin-3-yl)-1H-pyrazol-1-yl)-3-morpholinopropan-2-ol used in Example 0729-2.
$^1$H-NMR($CDCl_3$)$\delta$:9.63(1H,s),8.94(2H,s),8.83(1H,s),8.25(1H,d,J=8.5Hz),8.11(1H,s),8.08(1H,s),7.96(1H,s ),7.70(1H,d,J=8.5Hz),4.53(1H,dd,J=13.9,3.3Hz),4.22(1H,dd,J=13.9,7.9Hz),3.97-3.87(1H,m),3.75-3.66(5H,m),3.50-3.38(3H,m),3.33(3H,s),3.11-3.02(1H,m),2.58-2.45(6H,m),1.43(6H,d,J=6.6Hz).
MSm/z(M+H):533.

<Example 0745>

<0745-1>

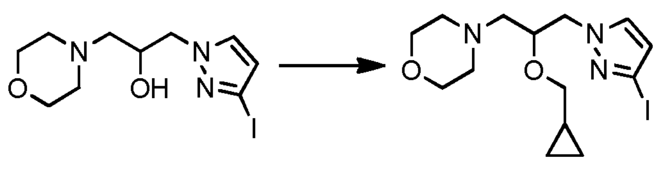

4-(2-(Cyclopropylmethoxy)-3-(3-iodo-1H-pyrazol-1-yl)propyl)morpholine was obtained in the same manner as in Example 0732-2 except that (bromomethyl)cyclopropane was used instead of the benzyl bromide used in Example

0732-2.
MSm/z(M+H):392.

<0745-2>

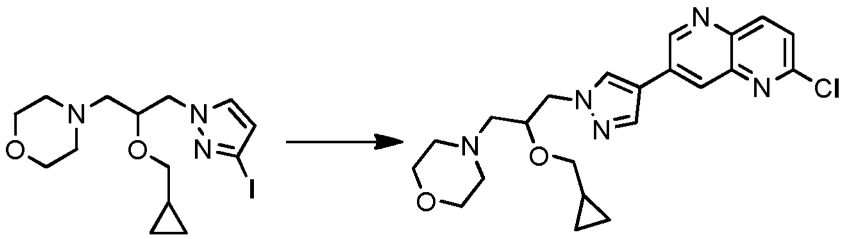

4-(3-(4-(6-Chloro-1,5-naphthyridin-3-yl)-1H-pyrazol-1-yl)-2-(cyclopropylmethoxy)propyl)morpholine was obtained in the same manner as in Example 0385-7 except that 4-(2-(cyclopropylmethoxy)-3-(3-iodo-1H-pyrazol-1-yl)propyl)morpholine was used instead of the 4-(3-(3-ethyl-4-iodo-1H-pyrazol-1-yl)propyl)morpholine used in Example 0385-7.
MSm/z(M+H):428.

<0745-3>

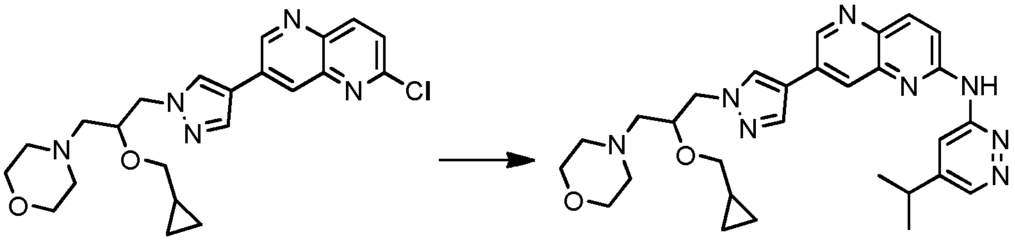

7-(1-(2-(Cyclopropylmethoxy)-3-morpholinopropyl)-1H-pyrazol-4-yl)-N-(5-isopropylpyridazin-3-yl)-1,5-naphthyridine-2-amine was obtained as a pale yellow solid in the same manner as in Example 0729-2 except that 4-(3-(4-(6-chloro-1,5-naphthyridin-3-yl)-1H-pyrazol-1-yl)-2-(cyclopropylmethoxy)propyl)morpholine was used instead of the 1-(4-(6-chloro-1,5-naphthyridin-3-yl)-1H-pyrazol-1-yl)-3-morpholinopropan-2-ol used in Example 0729-2.
$^1$H-NMR($CDCl_3$)$\delta$:9.27(1H,s),8.94(1H,d,J=1.7Hz),8.90(1H,s),8.83(1H,s),8.25(1H,d,J=8.6Hz),8.11(1H,d,J =1.7Hz),8.00(1H,s),7.97(1H,s),7.62(1H,d,J=8.6Hz),4.52(1H,dd,J=14.0,3.3Hz),4.19(1H,dd,J=14.0,8.3 Hz),3.93-3.84(1H,m),3.73(4H,t,J=4.6Hz),3.38(1H,dd,J=10.2,6.9Hz),3.12-3.02(2H,m),2.56-2.44(6H,m),1.42(6H,d,J=6.6Hz),0.98-0.91(1H,m),0.49-0.44(2H,m),0.13-0.07(2H,m).
MSm/z(M+H):529.

<Example 0746>

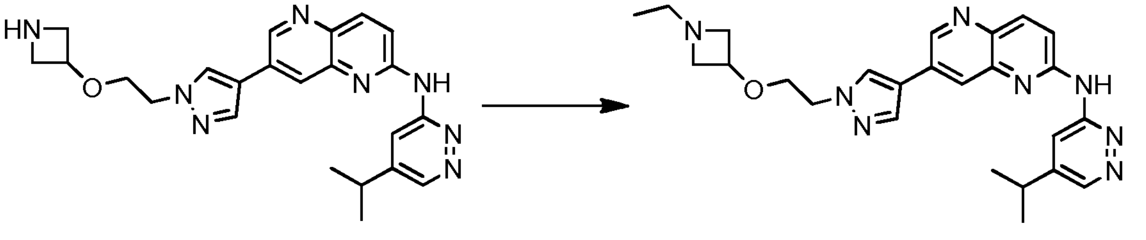

7-(1-(2-((1-Ethylazetidin-3-yl)oxy)ethyl)-1H-pyrazol-4-yl)-N-(5-isopropylpyridazin-3-yl)-1,5-naphthyridine-2-amine was obtained as a pale yellow solid in the same manner as in Example 0741 except that acetaldehyde was used instead of the 36.0% to 38.0% formaldehyde aqueous solution used in Example 0741.

$^1$H-NMR($CDCl_3$)δ:8.98-8.88(2H,m),8.83(1H,s),8.25(1H,d,J=8.6Hz),8.11(1H,s),7.98(1H,s),7.95(1H,s),7.78-7.60(1H,m),4.38(2H,t,J=5.2Hz),4.15-4.07(1H,m),3.80(2H,t,J=5.2Hz),3.57(2H,dd,J=6.9,3.5Hz),3.12-3.02(1H,m),2.82(2H,dd,J=6.9,3.5Hz),2.44(2H,q,J=7.3Hz),1.43(6H,d,J=6.6Hz),0.94(3H,t,J=7.3Hz).

MSm/z(M+H):459.

<Example 0747>

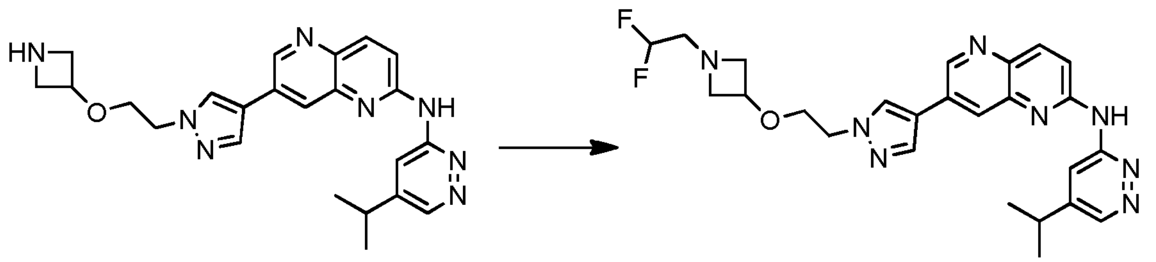

Cesium carbonate (6 mg) and 2,2-difluoroethyl trifluoromethanesulfonate (3 mg) were added to a suspension of 7-(1-(2-(azetidin-3-yloxy)ethyl)-1H-pyrazol-4-yl)-N-(5-isopropylpyridazin-3-yl)-1,5-naphthyridine-2-amine (5 mg) in tetrahydrofuran (0.5 mL), followed by stirring at room temperature for 80 minutes. Water (0.5 mL) was added to the reaction mixture, the solvent was distilled off under reduced pressure, and the obtained residue was purified by silica gel column chromatography (ethyl acetate-hexane-methanol, NH silica), thereby obtaining 7-(1-(2-((1-(2,2-difluoroethyl)azetidin-3-yl)oxy)ethyl)-1H-pyrazol-4-yl)-N-(5-isopropylpyridazin-3-yl)-1,5-naphthyridine-2-amine (3 mg) as a pale yellow solid.

$^1$H-NMR($CDCl_3$)δ:8.94(1H,d,J=2.0Hz),8.88-8.78(2H,m),8.25(1H,d,J=9.2Hz),8.11(1H,s),7.98(1H,s),7.94(1H,s),7.59(1H,d,J=9.2Hz),5.72(1H,tt,J=55.8,4.3Hz),4.38(2H,t,J=5.0Hz),4.17-4.09(1H,m),3.80(2H,t,J=5.0Hz),3.66(2H,dd,J=8.3,6.3Hz),3.08-3.03(3H,m),2.78(2H,td,J=15.0,4.3Hz),1.42(6H,d,J=7.3Hz).

MSm/z(M+H):495.

<Example 0748>

<0748-1>

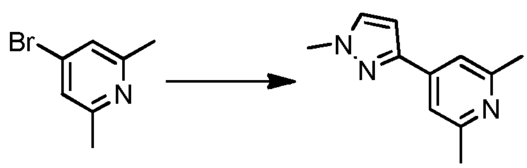

2,6-Dimethyl-4-(1-methyl-1H-pyrazol-3-yl)pyridine was obtained in the same manner as in Example 0734-1 except that 4-bromo-2,6-dimethylpyridine was used instead of the 5-bromo-2-methylpyridine used in Example 0734-1.

MSm/z(M+H):188.

<0748-2>

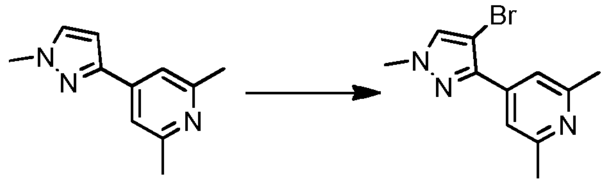

4-(4-Bromo-1-methyl-1H-pyrazol-3-yl)-2,6-dimethylpyridine was obtained as a pale brown solid in the same manner as in Example 0734-2 except that 2,6-dimethyl-4-(1-methyl-1H-pyrazol-3-yl)pyridine was used instead of the 2-methyl-5-(1-methyl-1H-pyrazol-3-yl)pyridine used in Example 0734-2.
MSm/z(M+H):266.

<0748-3>

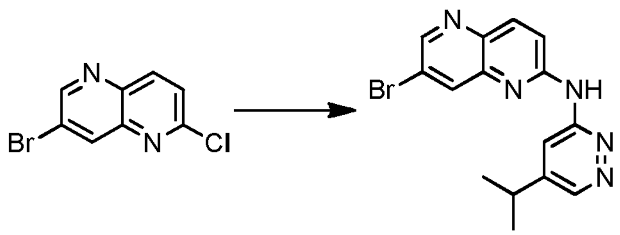

5-Isopropylpyridazine-3-amine (411 mg) and sodium tert-pentaoxide (726 mg) were added to a solution of 7-bromo-2-chloro-1,5-naphthyridine (729 mg) in N,N-dimethylacetamide (10 mL), followed by stirring at 80°C for 10 minutes, and stirring at 100°C for 50 minutes. After the reaction mixture was cooled to room temperature, water was added thereto, the precipitated solid was collected by filtration, and washed with ethyl acetate, thereby obtaining 7-bromo-N-(5-isopropylpyridazin-3-yl)-1,5-naphthyridine-2-amine (616 mg).
MSm/z(M+H):346.

<0748-4>

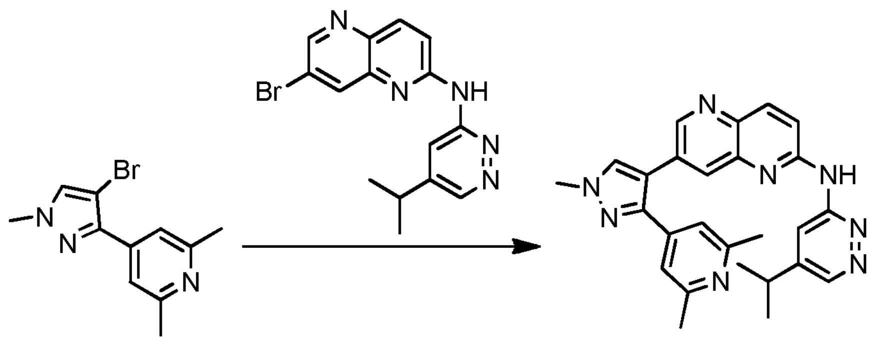

7-(3-(2,6-Dimethylpyridin-4-yl)-1-methyl-1H-pyrazol-4-yl)-N-(5-isopropylpyridazin-3-yl)-1,5-naphthyridine-2-amine was obtained in the same manner as in Example 385-7 except that 7-bromo-N-(5-isopropylpyridazin-3-yl)-1,5-naphthyridine-2-amine was used instead of the 7-bromo-2-chloro-1,5-naphthyridine used in Example 0385-7, and 4-(4-bromo-1-methyl-1H-pyrazol-3-yl)-2,6-dimethylpyridine was used instead of the 4-(3-(3-ethyl-4-iodo-1H-pyrazol-1-yl)propyl)morpholine used in Example 0385-7.
$^1$H-NMR($CDCl_3$)$\delta$:9.53(1H,s),8.85(1H,s),8.80(1H,s),8.67(1H,d,J=2.0Hz),8.26(1H,d,J=9.2Hz),7.97(1H,d,J =2.0Hz),7.71(1H,d,J=9.2Hz),7.69(1H,s),7.11(2H,s),4.07(3H,s),3.05-2.95(1H,m),2.46(6H,s),1.35(6H,d,J=7.3Hz).
MSm/z(M+H):451.

<Example 0749>

<0749-1>

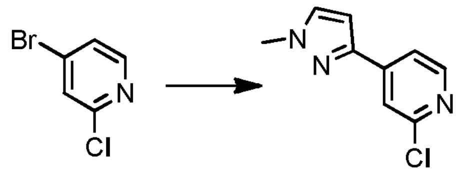

2-Chloro-4-(1-methyl-1H-pyrazol-3-yl)pyridine was obtained as pale yellow oily substance in the same manner as in Example 0734-1 except that 4-bromo-2-chloropyridine was used instead of the 5-bromo-2-methylpyridine used in Example 0734-1.
MSm/z(M+H):194.

<0749-2>

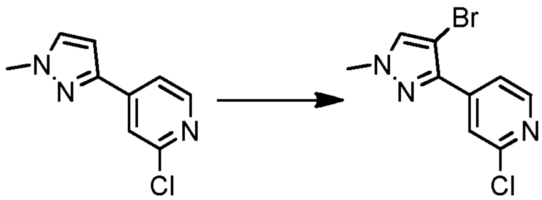

4-(4-Bromo-1-methyl-1H-pyrazol-3-yl)-2-chloropyridine was obtained as a white solid in the same manner as in Example 0734-2 except that 2-chloro-4-(1-methyl-1H-pyrazol-3-yl)pyridine was used instead of the 2-methyl-5-(1-methyl-1H-pyrazol-3-yl)pyridine used in Example 0734-2.
MSm/z(M+H):274.

<0749-3>

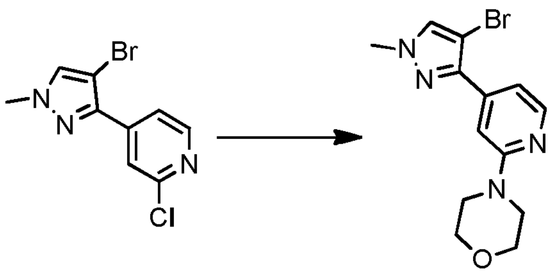

Morpholine (1 mL) was added to 4-(4-bromo-1-methyl-1H-pyrazol-3-yl)-2-chloropyridine (100 mg), followed by stirring at 150°C for 90 minutes using a microwave reaction apparatus. The reaction mixture was cooled to room temperature, the solvent was distilled off under reduced pressure, and the obtained residue was purified by silica gel column chromatography (ethyl acetate-hexane, NH silica), thereby obtaining 4-(4-(4-bromo-1-methyl-1H-pyrazol-3-yl)pyridin-2-yl)morpholine (41 mg).
MSm/z(M+H):325.

<0749-4>

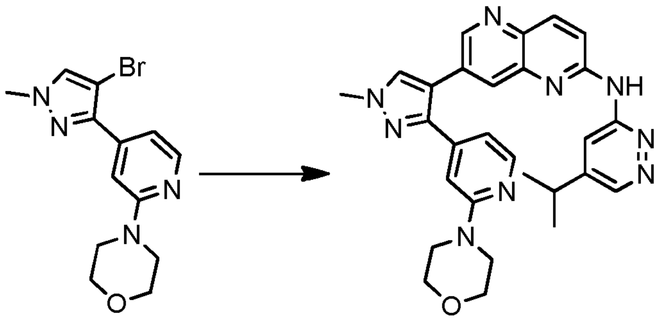

N-(5-isopropylpyridazin-3-yl)-7-(1-methyl-3-(2-morpholinopyridin-4-yl)-1H-pyrazol-4-yl)-1,5-naphthyridine-2-amine was obtained as a pale yellow solid in the same manner as in Example 0385-7 except that 7-bromo-N-(5-isopropylpyridazin-3-yl)-1,5-naphthyridine-2-amine was used instead of the 7-bromo-2-chloro-1,5-naphthyridine used in Example 0385-7, and 4-(4-(4-bromo-1-methyl-1H-pyrazol-3-yl)pyridin-2-yl)morpholine was used instead of the 4-(3-(3-ethyl-4-iodo-1H-pyrazol-1-yl)propyl)morpholine used in Example 0385-7.
$^1$H-NMR($CDCl_3$)δ:9.23(1H,brs),8.85-8.77(2H,m),8.69(1H,d,J=2.0Hz),8.26(1H,d,J=8.6Hz),8.12(1H,d,J=5.3Hz),8.00(1H,d,J=2.0Hz),7.70-7.66(2H,m),6.87(1H,s),6.71(1H,d,J=5.3Hz),4.06(3H,s),3.75(4H,t,J=4.6Hz),3.44(4H,t,J=4.6Hz),3.05-2.95(1H,m),1.36(6H,d,J=7.3Hz).
MSm/z(M+H):508.

<Example 0750>

<0750-1>

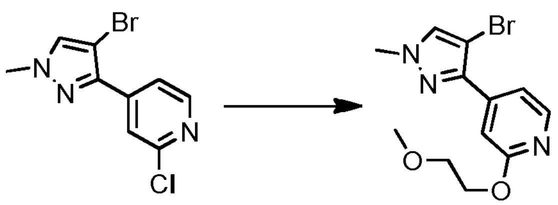

60% sodium hydride (28 mg) was added to a solution of 2-methoxyethanol (54 μL) in N,N-dimethylacetamide (1 mL) under ice-cooling, followed by stirring for 1 hour. 4-(4-Bromo-1-methyl-1H-pyrazol-3-yl)-2-chloropyridine (75 mg) was added to the reaction mixture, followed by stirring at 100°C for 3 hours. After the reaction mixture was cooled to room temperature, water was added thereto, the resultant product was extracted with ethyl acetate, and the organic layer was washed with a saturated sodium chloride aqueous solution, and dried over anhydrous sodium sulfate. The solvent was distilled off under reduced pressure, and the obtained residue was purified by silica gel column chromatography (hexane-ethyl acetate), thereby obtaining 4-(4-bromo-1-methyl-1H-pyrazol-3-yl)-2-(2-methoxyethoxy)pyridine (32 mg) as colorless oily substance.
MSm/z(M+H):314.

<0750-2>

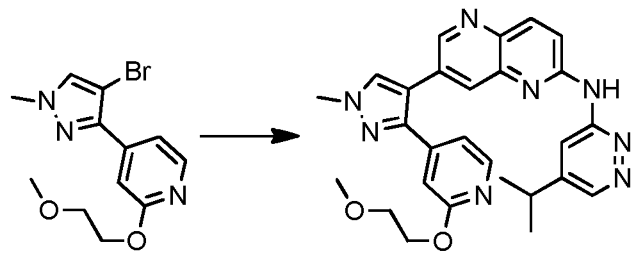

N-(5-isopropylpyridazin-3-yl)-7-(3-(2-(2-methoxyethoxy)pyridin-4-yl)-1-methyl-1H-pyrazol-4-yl)-1,5-naphthyridine-2-amine was obtained as a pale yellow solid in the same manner as in Example 0385-7 except that 7-bromo-N-(5-isopropylpyridazin-3-yl)-1,5-naphthyridine-2-amine was used instead of the 7-bromo-2-chloro-1,5-naphthyridine used in Example 0385-7, and 4-(4-bromo-1-methyl-1H-pyrazol-3-yl)-2-(2-methoxyethoxy)pyridine was used instead of the 4-(3-(3-ethyl-4-iodo-1H-pyrazol-1-yl)propyl)morpholine used in Example 0385-7.
$^1$H-NMR($CDCl_3$)$\delta$:9.13(1H,s),8.82(1H,s),8.80(1H,s),8.66(1H,d,J=1.3Hz),8.26(1H,d,J=9.2Hz),8.08(1H,d,J =4.9Hz),7.98(1H,d,J=1.3Hz),7.66(1H,s),7.63(1H,d,J=9.2Hz),7.01(1H,dd,J=4.9,1.3Hz),6.96(1H,s),4.45 (2H,t,J=4.6Hz),4.05(3H,s),3.70(2H,t,J=4.6Hz),3.38(3H,s),3.03-2.96(1H,m),1.35(6H,d,J=7.3Hz).
MSm/z(M+H):497.

<Example 0751>

<0751-1>

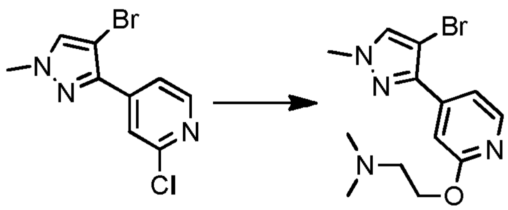

2-((4-(4-Bromo-1-methyl-1H-pyrazol-3-yl)pyridin-2-yl)oxy)-N,N-dimethylethanamine was obtained as a pale brown solid in the same manner as in Example 0750-1 except that 2-dimethylaminoethanol was used instead of the 2-methoxyethanol used in Example 0750-1.
MSm/z(M+H):325.

<0751-2>

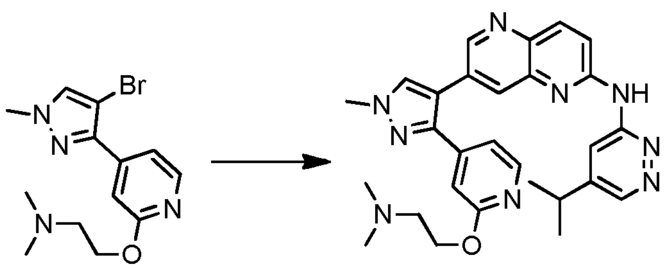

7-(3-(2-(2-(Dimethylamino)ethoxy)pyridin-4-yl)-1-methyl-1H-pyrazol-4-yl)-N-(5-isopropylpyridazin-3-yl)-1,5-naphthyridine-2-amine was obtained as a brown solid in the same manner as in Example 0385-7 except that 7-bromo-N-(5-isopropylpyridazin-3-yl)-1,5-naphthyridine-2-amine was used instead of the 7-bromo-2-chloro-1,5-naphthyridine used in Example 0385-7, and 2-((4-(4-bromo-1-methyl-1H-pyrazol-3-yl)pyridin-2-yl)oxy)-N,N-dimethylethanamine was used instead of the 4-(3-(3-ethyl-4-iodo-1H-pyrazol-1-yl)propyl)morpholine used in Example 0385-7.

$^1$H-NMR($CDCl_3$)δ:9.25(1H,s),8.84(1H,s),8.80(1H,s),8.66(1H,d,J=1.5Hz),8.26(1H,d,J=8.6Hz),8.09(1H,d,J =5.6Hz),7.98(1H,d,J=1.5Hz),7.69-7.63(2H,m),7.00(1H,dd,J=5.6,1.5Hz),6.94(1H,s),4.39(2H,t,J=5.9Hz),4.05(3H,s),3.03-2.96(1H,m),2.67(2H,t,J=5.9Hz),2.28(6H,s),1.35(6H,d,J=6.6Hz).
MSm/z(M+H):510.

<Example 0752>

<0752-1>

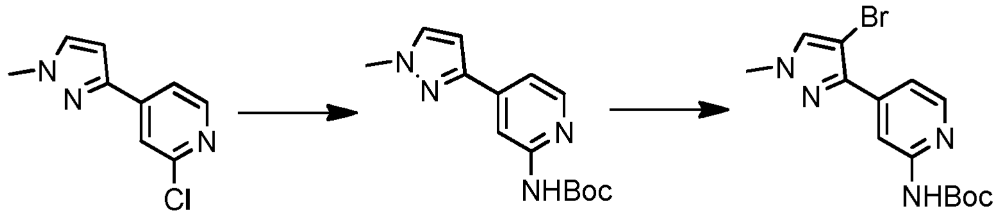

Sodium tert-butoxide (115 mg) and ((2-dicyclohexylphosphino-3,6-dimethoxy-2',4',6'-triisopropyl-1,1'-biphenyl)-2-(2'-amino-1,1'-biphenyl))palladium(II) methanesulfonate (BRETTPHOS-PD-G3, (product name, manufactured by Sigma-Aldrich Co. LLC.)) (45 mg) were added to a solution of 2-chloro-4-(1-methyl-1H-pyrazol-3-yl)pyridine (190 mg) and tert-butyl carbamate (140 mg) in 1,4-dioxane (5 mL), followed by stirring at 100°C for 2 hours. The reaction mixture was cooled to room temperature, the solvent was distilled off under reduced pressure, and the obtained residue was purified by silica gel column chromatography (hexane-ethyl acetate), thereby obtaining tert-butyl (4-(1-methyl-1H-pyrazol-3-yl)pyridin-2-yl)carbamate.

N-bromosuccinimide (215 mg) was added to a solution of the obtained tert-butyl (4-(1-methyl-1H-pyrazol-3-yl)pyridin-2-yl)carbamate in N,N-dimethylformamide (2 mL), followed by stirring at room temperature for 50 minutes. After water was added to the reaction mixture, the resultant product was extracted with ethyl acetate, and the organic layer was washed with a saturated sodium chloride aqueous solution, and dried over anhydrous sodium sulfate. The solvent was distilled off under reduced pressure, and the obtained residue was purified by silica gel column chromatography (hexane-ethyl acetate), thereby obtaining tert-butyl (4-(4-bromo-1-methyl-1H-pyrazol-3-yl)pyridin-2-yl)carbamate (22 mg) as yellow oily substance.
MSm/z(M+H):353.

<0752-2>

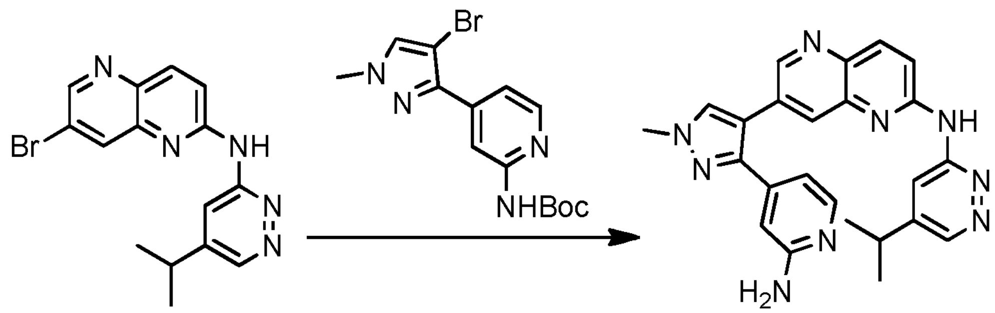

Bis(pinacolato)diboron (295 mg), a (1,1'-bis(diphenylphosphino)ferrocene)palladium(II) dichloride-dichloromethane adduct (47 mg), and potassium acetate (171 mg) were added to a solution of 7-bromo-N-(5-isopropylpyridazin-3-yl)-1,5-naphthyridine-2-amine (200 mg) in 1,4-dioxane (5 mL), followed by stirring at 80°C for 1.5 hours. A (1,1'-bis(diphenylphosphino)ferrocene)palladium(II) dichloride-dichloromethane adduct (20 mg) was added to the reaction mixture, followed by stirring at 90°C for 2 hours. The reaction mixture (0.75 mL) was collected by separation, followed by cooling to room temperature, and 1,4-dioxane (2.25 mL), tert-butyl (4-(4-bromo-1-methyl-1H-pyrazol-3-yl)pyridin-2-yl)carbamate (22 mg), sodium carbonate (15 mg), bis(di-tert-butyl (4-dimethylaminophenyl)phosphine)dichloropalladium(II) (4 mg), and water (0.3 mL) were added thereto, followed by stirring at 100°C for 2 hours. The reaction mixture

was cooled to room temperature, the solvent was distilled off under reduced pressure, and trifluoroacetic acid (1 mL) was added to the obtained residue, followed by allowing to stand for 5 minutes. The solvent was distilled off under reduced pressure, and the obtained residue was purified by silica gel column chromatography (hexane-ethyl acetate-methanol, NH silica), thereby obtaining 7-(3-(2-aminopyridin-4-yl)-1-methyl-1H-pyrazol-4-yl)-N-(5-isopropylpyridazin-3-yl)-1,5-naphthyridine-2-amine (1 mg) as a brown solid.
$^1$H-NMR($CD_3OD$)δ:8.82(1H,s),8.74(1H,d,J=1.5Hz),8.67(1H,d,J=1.5Hz),8.19(1H,d,J=9.2Hz),8.11(1H,s),8 .04(1H,d,J=1.5 Hz),7.88(1H,d,J=5.3Hz),7.62(1H,d,J=9.2Hz),6.70(1H,s),6.67(1H,dd,J=5.3,1.5Hz),4.03( 3H,s),3.09-2.98(1H,m),1.33(6 H,d,J=7.3Hz).
MSm/z(M+H):438.

<Example 0753>

<0753-1>

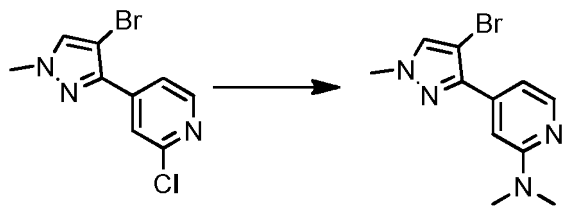

A 50% dimethylamine aqueous solution (1 mL) and 1,4-dioxane (0.5 mL) were added to 4-(4-bromo-1-methyl-1H-pyrazol-3-yl)-2-chloropyridine (75 mg), followed by stirring at 160°C for 1 hour using a microwave reaction apparatus. The reaction mixture was cooled to room temperature, the solvent was distilled off under reduced pressure, and the obtained residue was purified by silica gel column chromatography (hexane-ethyl acetate, NH silica), thereby obtaining 4-(4-bromo-1-methyl-1H-pyrazol-3-yl)-N,N-dimethylpyridine-2-amine (41 mg).
MSm/z(M+H):281.

<0753-2>

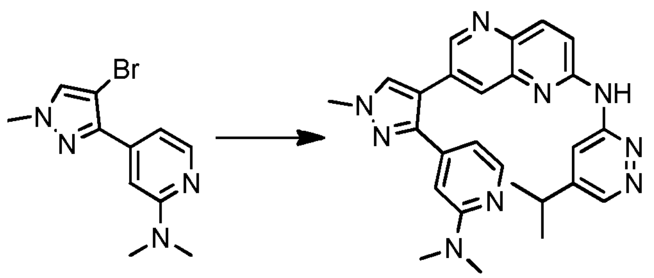

7-(3-(2-(Dimethylamino)pyridin-4-yl)-1-methyl-1H-pyrazol-4-yl)-N-(5-isopropylpyridazin-3-yl)-1,5-naphthyridine-2-amine was obtained as a yellow solid in the same manner as in Example 0385-7 except that 7-bromo-N-(5-isopropylpyridazin-3-yl)-1,5-naphthyridine-2-amine was used instead of the 7-bromo-2-chloro-1,5-naphthyridine used in Example 0385-7, and 4-(4-bromo-1-methyl-1H-pyrazol-3-yl)-N,N-dimethylpyridine-2-amine was used instead of the 4-(3-(3-ethyl-4-iodo-1H-pyrazol-1-yl)propyl)morpholine used in Example 0385-7.
$^1$H-NMR($CDCl_3$)δ:10.00(1H,brs),8.93(1H,s),8.81(1H,s),8.70(1H,d,J=1.6Hz),8.25(1H,d,J=8.6Hz),8.11(1H ,d,J=5.3Hz),8.01 (1H,d,J=1.6Hz),7.81(1H,d,J=8.6Hz),7.67(1H,s),6.68(1H,s),6.65(1H,d,J=5.3Hz),4.06( 3H,s),3.05-2.94(7H,m),1.36(6H,d ,J=7.2Hz).
MSm/z(M+H):466.

<Example 0754>

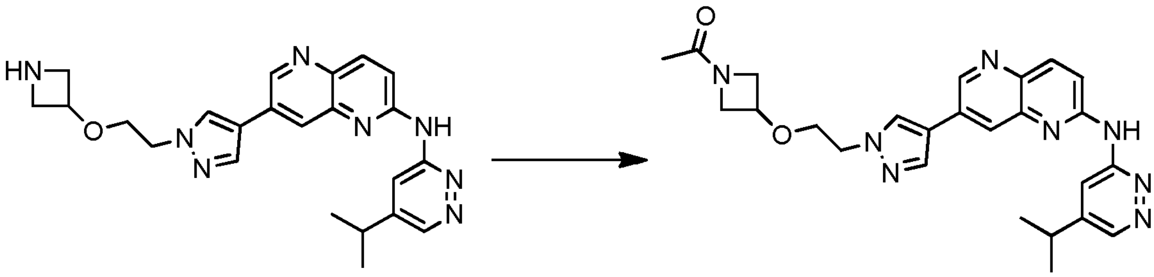

Triethylamine (2 μL) and acetic anhydride (1 μL) were added to a suspension of 7-(1-(2-(azetidin-3-yloxy)ethyl)-1H-pyrazol-4-yl)-N-(5-isopropylpyridazin-3-yl)-1,5-naphthyridine-2-amine (4 mg) in tetrahydrofuran (0.5 mL), followed by stirring at room temperature for 1 hour. The solvent was distilled off under reduced pressure, and the obtained residue was purified by silica gel column chromatography (hexane-ethyl acetate, NH silica), thereby obtaining 1-(3-(2-(4-(6-((5-isopropylpyridazin-3-yl)amino)-1,5-naphthyridin-3-yl)-1H-pyrazol-1-yl)ethoxy)azetidin-1-yl)ethanone (2 mg) as a pale yellow solid.
$^1$H-NMR($CDCl_3$)δ:9.42(1H,s),8.93(1H,d,J=1.6Hz),8.89(1H,s),8.83(1H,s),8.25(1H,d,J=8.9Hz),8.12(1H,d,J =1.6Hz),8.00(1H,s),7.92(1H,s),7.67(1H,d,J=8.9Hz),4.41(2H,t,J=5.3Hz),4.31-4.20(2H,m),4.17-4.10(1H,m),3.95-3.90(1 H,m),3.87-3.81(3H,m),3.12-3.02(1H,m),1.84(3H,s),1.43(6H,d,J=6.6Hz).
MSm/z(M+H):473.

<Example 0755>

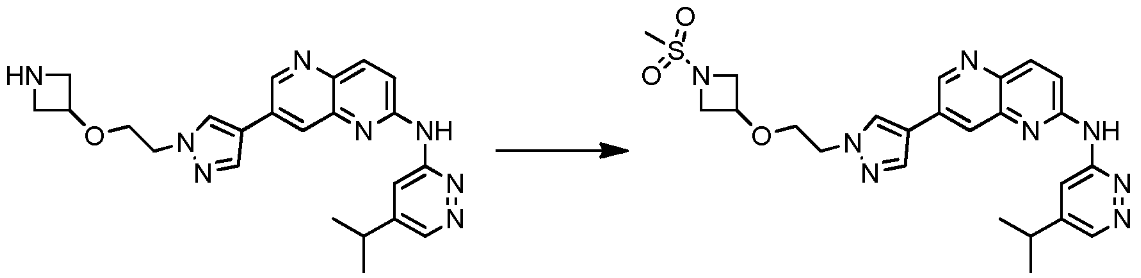

Triethylamine (2 μL) and methanesulfonyl chloride (1 μL) were added to a suspension of 7-(1-(2-(azetidin-3-yloxy)ethyl)-1H-pyrazol-4-yl)-N-(5-isopropylpyridazin-3-yl)-1,5-naphthyridine-2-amine (4 mg) in methylene chloride (0.5 mL), followed by stirring at room temperature for 1 hour. The reaction mixture was purified by silica gel column chromatography (hexane-ethyl acetate, NH silica), thereby obtaining N-(5-isopropylpyridazin-3-yl)-7-(1-(2-((1-(methylsulfonyl)azetidin-3-yl)oxy)ethyl)-1H-pyrazol-4-yl)-1,5-naphthyridine-2-amine (3 mg) as a pale yellow solid.
$^1$H-NMR($CDCl_3$)δ:9.85(1H,s),8.94(1H,s),8.93(1H,d,J=1.6Hz),8.83(1H,s),8.25(1H,d,J=8.9Hz),8.11(1H,d,J =1.6Hz),7.99(1H,s),7.92(1H,s),7.76(1H,d,J=8.9Hz),4.40(2H,t,J=5.3Hz),4.28-4.20(1H,m),4.07-4.00(2H,m),3.86(2H,t,J= 5.3Hz),3.79(2H,dd,J=9.2,4.6Hz),3.12-3.03(1H,m),2.80(3H,s),1.43(6H,d,J=7.3Hz).
MSm/z(M+H):509.

<Example 0756>

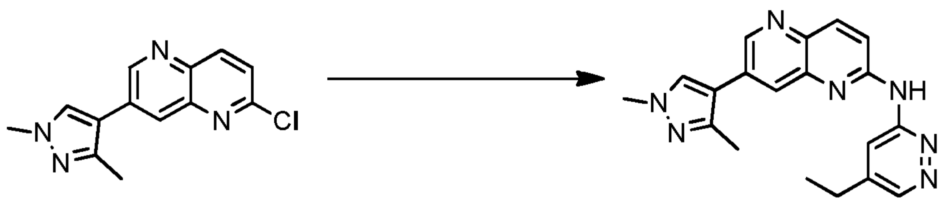

7-(1,3-Dimethyl-1H-pyrazol-4-yl)-N-(5-ethylpyridazin-3-yl)-1,5-naphthyridine-2-amine was obtained in the same manner as in Example 0757-2 except that 5-ethylpyridazine-3-amine was used instead of the 5-isopropylpyridazine-3-amine used in Example 0757-2.
$^1$H-NMR(DMSO-$d_6$)δ:10.72(1H,s),8.88(1H,d,J=2.0Hz),8.80(1H,d,J=2.0Hz),8.70(1H,d,J=1.3Hz),8.24-8.23(2H,m),8.09(1H,d,J=2.0Hz),7.74(1H,d,J=9.2Hz),3.85(3H,s),2.73(2H,q,J=7.5Hz),2.42(3H,s),1.31( 3H,t,J=7.5Hz).
MSm/z(M+H):346.

<Example 0757>

<0757-1>

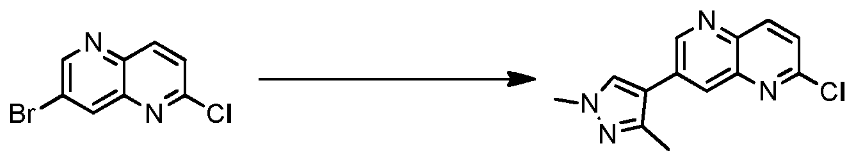

A mixture of 7-bromo-2-chloro-1,5-naphthyridine (600 mg), 1,3-dimethyl-4-(4,4,5,5-tetramethyl-1,3,2-dioxaborolan-2-yl)-1H-pyrazole (442 mg), bis(di-tert-butyl (4-dimethylaminophenyl)phosphine)dichloropalladium(II) (174 mg), sodium carbonate (522 mg), 1,4-dioxane (24 mL), and water (2.4 mL) was stirred at 100°C for 3 hours in a nitrogen atmosphere. The reaction mixture was cooled to room temperature, and the solvent was distilled off under reduced pressure. The reaction residue was purified by silica gel column chromatography (chloroform-methanol, NH silica), thereby obtaining 2-chloro-7-(1,3-dimethyl-1H-pyrazol-4-yl)-1,5-naphthyridine (412 mg) as a white solid.
MSm/z(M+H):259.

<0757-2>

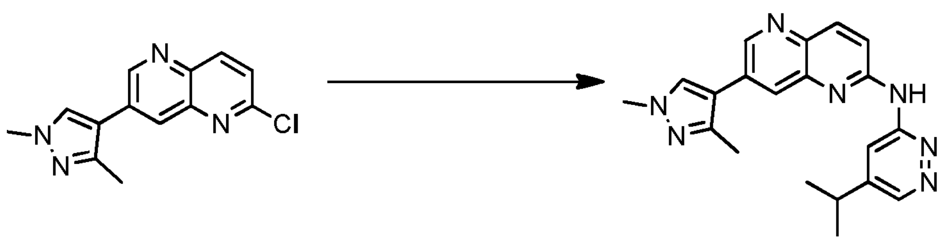

A mixture of 2-chloro-7-(1,3-dimethyl-1H-pyrazol-4-yl)-1,5-naphthyridine (30 mg), 5-isopropylpyridazine-3-amine (24 mg), tris(dibenzylideneacetone)dipalladium(0) (11 mg), 4,5-bis(diphenylphosphino)-9,9-dimethylxanthene (13 mg), cesium carbonate (76 mg), and 1,4-dioxane (1.2 mL) was stirred at 140°C for 30 minutes using a microwave reaction apparatus. The reaction mixture was cooled to room temperature, and the solvent was distilled off under reduced pressure. The obtained residue was purified by silica gel column chromatography (chloroform-methanol, NH silica), and purified by silica gel column chromatography (chloroform-methanol), thereby obtaining 7-(1,3-dimethyl-1H-pyrazol-4-yl)-N-(5-isopropylpyridazin-3-yl)-1,5-naphthyridine-2-amine (17 mg) as a white solid.
$^1$H-NMR(DMSO-$d_6$)δ:10.72(1H,s),8.88(1H,d,J=2.0Hz),8.85(1H,d,J=1.3Hz),8.74(1H,d,J=1.3Hz),8.24-8.23(2H,m),8.06(1H,d,J=2.0Hz),7.

74(1H,d,J=9.2Hz),3.85(3H,s),3.03-3.00(1H,m),2.42(3H,s),1.31(6H,d,J=7.3Hz).
MSm/z(M+H):360.

<Example 0758>

<0758-1>

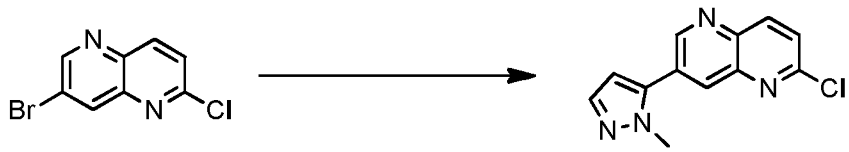

2-Chloro-7-(1-methyl-1H-pyrazol-5-yl)-1,5-naphthyridine was obtained in the same manner as in Example 0757-1 except that 1-methyl-5-(4,4,5,5-tetramethyl-1,3,2-dioxaborolan-2-yl)-1H-pyrazole was used instead of the 1,3-dimethyl-4-(4,4,5,5-tetramethyl-1,3,2-dioxaborolan-2-yl)-1H-pyrazole used in Example 0757-1.
MSm/z(M+H):245.

<0758-2>

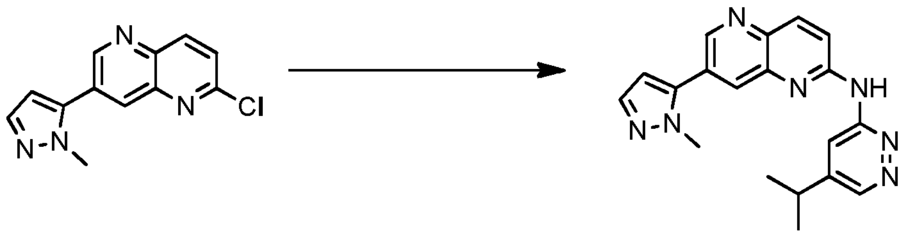

N-(5-isopropylpyridazin-3-yl)-7-(1-methyl-1H-pyrazol-5-yl)-1,5-naphthyridine-2-amine was obtained as a white solid in the same manner as in Example 0757-2 except that 2-chloro-7-(1-methyl-1H-pyrazol-5-yl)-1,5-naphthyridine was used instead of the 2-chloro-7-(1,3-dimethyl-1H-pyrazol-4-yl)-1,5-naphthyridine used in Example 0757-2.
$^1$H-NMR($CDCl_3$)δ:9.21(1H,s),8.87-8.84(3H,m),8.32(1H,d,J=9.2Hz),8.13(1H,d,J=2.0Hz),7.74(1H,d,J=9.2Hz),7.63(1H,d,J=2.0Hz),6.53(1 H,d,J=2.0Hz),4.02(3H,s),3.03-3.00(1H,m),1.40(6H,d,J=7.3Hz).
MSm/z(M+H):346.

<Example 0759>

<0759-1>

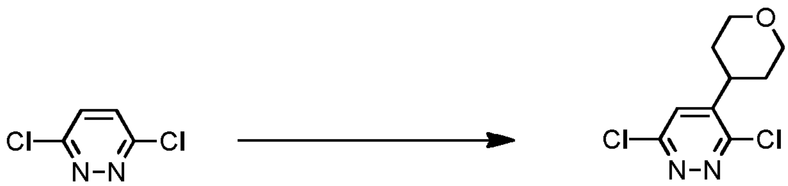

3,6-Dichloro-4-(tetrahydro-2H-pyran-4-yl)pyridazine was obtained as a white solid in the same manner as in Example 0014-1 except that tetrahydro-2H-pyran-4-carboxylic acid was used instead of the isobutyric acid used in Example 0014-1.
MSm/z(M+H):233.

<0759-2>

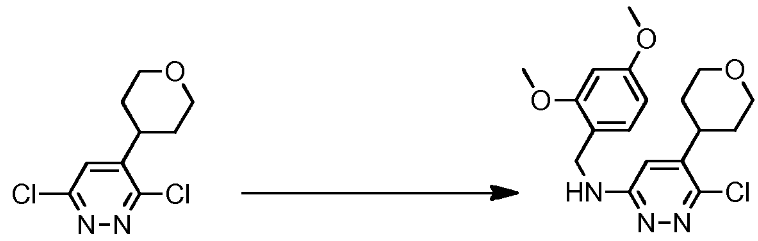

6-Chloro-N-(2,4-dimethoxybenzyl)-5-(tetrahydro-2H-pyran-4-yl)pyridazine-3-amine was obtained as pale yellow oily substance in the same manner as in Example 0014-2 except that 3,6-dichloro-4-(tetrahydro-2H-pyran-4-yl)pyridazine was used instead of the 3,6-dichloro-4-isopropylpyridazine used in Example 0014-2.
MSm/z(M+H):364.

<0759-3>

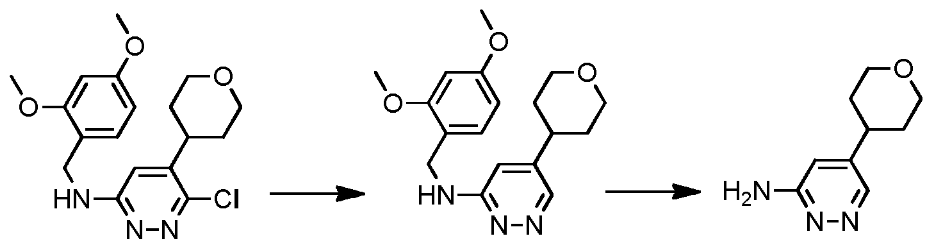

5-(Tetrahydro-2H-pyran-4-yl)pyridazine-3-amine was obtained as pale yellow oily substance in the same manner as in Example 0014-3 except that 6-chloro-N-(2,4-dimethoxybenzyl)-5-(tetrahydro-2H-pyran-4-yl)pyridazine-3-amine was used instead of the 6-chloro-N-(2,4-dimethoxybenzyl)-5-isopropylpyridazine-3-amine used in Example 0014-3.
MSm/z(M+H):180.

<0759-4>

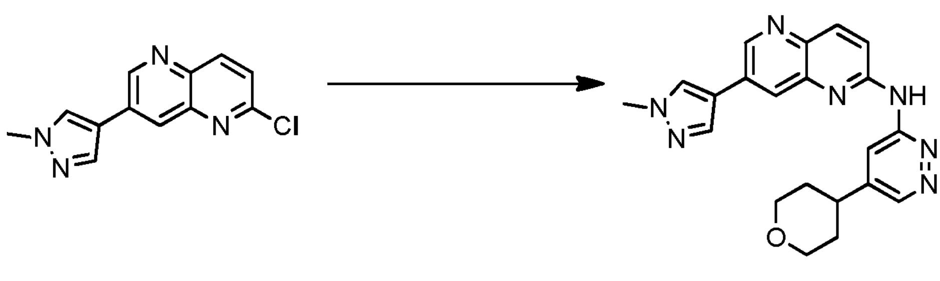

7-(1-methyl-1H-pyrazol-4-yl)-N-(5-(tetrahydro-2H-pyran-4-yl)pyridazin-3-yl)-1,5-naphthyridine-2-amine was obtained as a white solid in the same manner as in Example 0757-2 except that 2-chloro-7-(1-methyl-1H-pyrazol-4-yl)-1,5-naphthyridine was used instead of the 2-chloro-7-(1,3-dimethyl-1H-pyrazol-4-yl)-1,5-naphthyridine used in Example 0757-2, and 5-(tetrahydro-2H-pyran-4-yl)pyridazine-3-amine was used instead of the 5-isopropylpyridazine-3-amine used in Example 0757-2.
$^1$H-NMR(DMSO-$d_6$)δ:10.71(1H,s),9.04(1H,d,J=2.0Hz),8.89(1H,d,J=2.0Hz),8.65(1H,d,J=1.3Hz),8.47(1H,s),8.22-8.20(3H,m),7.73(1H,d,J=9.2Hz),4.04-4.00(2H,m),3.93(3H,s),3.55-3.47(2H,m),2.98-2.94(1H,s),1.85-1.78(4H,m).

MSm/z(M+H):388.

<Example 0760>

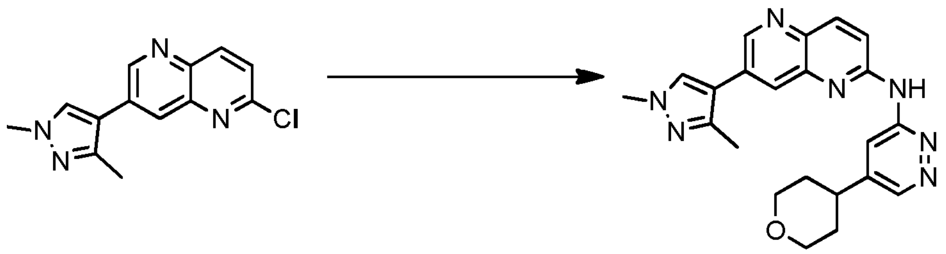

7-(1,3-Dimethyl-1H-pyrazol-4-yl)-N-(5-(tetrahydro-2H-pyran-4-yl)pyridazin-3-yl)-1,5-naphthyridine-2-amine was obtained as a white solid in the same manner as in Example 0759-4 except that 2-chloro-7-(1,3-dimethyl-1H-pyrazol-4-yl)-1,5-naphthyridine was used instead of the 2-chloro-7-(1-methyl-1H-pyrazol-4-yl)-1,5-naphthyridine used in Example 0759-4.
$^1$H-NMR(DMSO-$d_6$)$\delta$:10.74(1H,s),9.04(1H,d,J=2.0Hz),8.88-8.87(2H,m),8.69(1H,s),8.25-8.24(2H,m),7.76(1H,d,J=9.2Hz),4.01-3.98(2H,m),3.85(3H,s),3.53-3.41(2H,m),2.97-2.93(1H,s),2.42(3H,s)1.87-1.83(4H,m).
MSm/z(M+H):402.

<Example 0761>

<0761-1>

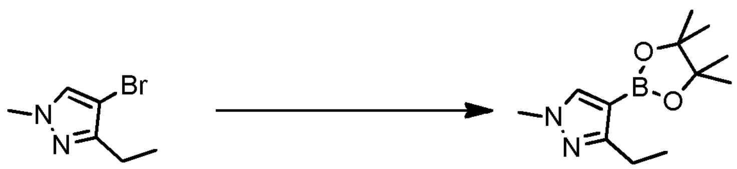

3-Ethyl-1-methyl-4-(4,4,5,5-tetramethyl-1,3,2-dioxaborolan-2-yl)-1H-pyrazole was obtained in the same manner as in Example 0124-2 except that 4-bromo-3-ethyl-1-methyl-1H-pyrazole was used instead of the 4-bromo-1-methyl-3-phenyl-1H-pyrazole used in Example 0124-2.
MSm/z(M+H):237.

<0761-2>

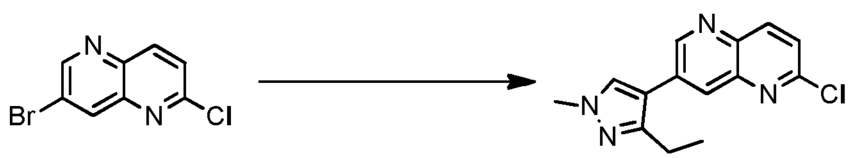

2-Chloro-7-(3-ethyl-1-methyl-1H-pyrazol-4-yl)-1,5-naphthyridine was obtained in the same manner as in Example 0757-1 except that 3-ethyl-1-methyl-4-(4,4,5,5-tetramethyl-1,3,2-dioxaborolan-2-yl)-1H-pyrazole was used instead of the 1,3-dimethyl-4-(4,4,5,5-tetramethyl-1,3,2-dioxaborolan-2-yl)-1H-pyrazole used in Example 0757-1.
MSm/z(M+H):273.

<0761-3>

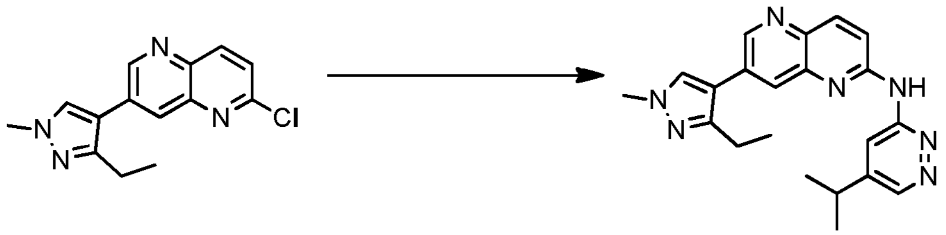

7-(3-Ethyl-1-methyl-1H-pyrazol-4-yl)-N-(5-isopropylpyridazin-3-yl)-1,5-naphthyridine-2-amine was obtained in the same manner as in Example 0757-2 except that 2-chloro-7-(3-ethyl-1-methyl-1H-pyrazol-4-yl)-1,5-naphthyridine was used instead of the 2-chloro-7-(1,3-dimethyl-1H-pyrazol-4-yl)-1,5-naphthyridine used in Example 0757-2.
$^1$H-NMR(DMSO-$d_6$)δ:10.74(1H,s),8.87-8.84(2H,m),8.77(1H,s),8.24-8.22(2H,m),8.02(1H,d,J=2.0Hz),7.73(1H,d,J=8.6Hz),3.86(3H,s),3.03-3.00(1H,m),2.82(2H,d,J=7.5Hz),1.31(6H,d,J=7.6Hz),1.23(3H,d,J=7.6Hz).
MSm/z(M+H):374.

<Example 0762>

<0762-1>

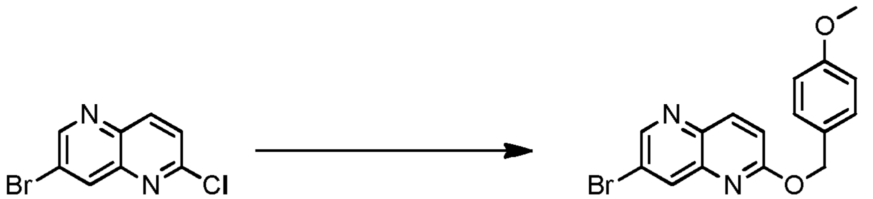

60% sodium hydride (136 mg) was added to a mixture of 7-bromo-2-chloro-1,5-naphthyridine (300 mg), (4-methoxyphenyl)methanol, and N-methylpyrrolidone (12.3 mL) at a temperature of from 0°C to 5°C, followed by stirring at room temperature for 2 hours in a nitrogen atmosphere. After water and ethyl acetate were added to the reaction mixture, the organic layer was washed sequentially with 0.01 mol/L hydrochloric acid and with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining 7-bromo-2-((4-methoxybenzyl)oxy)-1,5-naphthyridine (204 mg) as a white solid.
MSm/z(M+H):345.

<0762-2>

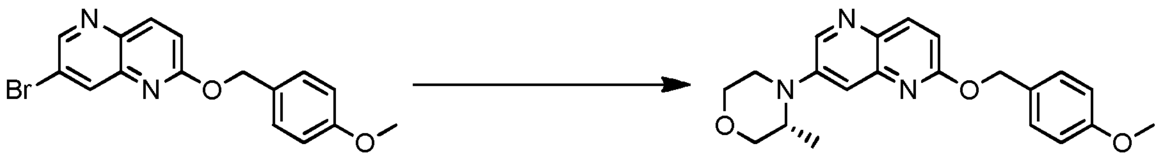

A mixture of 7-bromo-2-((4-methoxybenzyl)oxy)-1,5-naphthyridine (50 mg), (R)-3-methylmorpholine (14.7 mg), tris(dibenzylideneacetone)dipalladium(0) (11 mg), 2-dicyclohexylphosphino-2',4',6'-triisopropylbiphenyl (13 mg), sodium tert-butoxide (76 mg), and 1,4-dioxane (1.2 mL) was stirred at 100°C for 12 hours in a nitrogen atmosphere. The reaction mixture was cooled to room temperature, and the solvent was distilled off under reduced pressure. The obtained residue

was purified by silica gel column chromatography (hexane-ethyl acetate), thereby obtaining (R)-4-(6-((4-methoxybenzyl)oxy)-1,5-naphthyridin-3-yl)-3-methylmorpholine (20.2 mg) as a white solid.
MSm/z(M+H):366.

<0762-3>

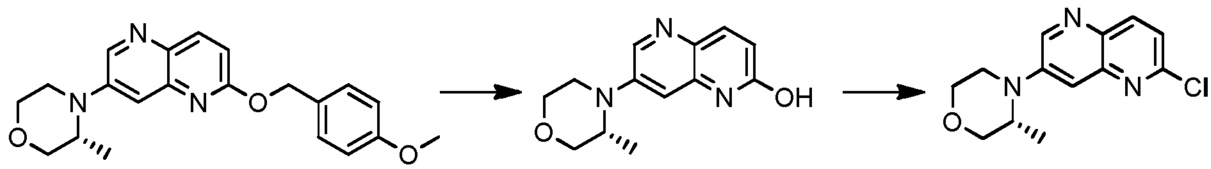

A mixture of (R)-4-(6-((4-methoxybenzyl)oxy)-1,5-naphthyridin-3-yl)-3-methylmorpholine (23.8 mg), trifluoroacetic acid (1.2 mL), and water (0.1 mL) was stirred at room temperature for 15 minutes. The solvent was distilled off under reduced pressure, and the obtained residue was purified by silica gel column chromatography (chloroform-methanol, NH silica), thereby obtaining (R)-7-(3-methylmorpholino)-1,5-naphthyridin-2-ol (14.7 mg).

A mixture of (R)-7-(3-methylmorpholino)-1,5-naphthyridin-2-ol (14.7 mg) and phosphorus oxychloride (1.0 mL) was stirred at 140°C for 30 minutes using a microwave reaction apparatus. The reaction mixture was cooled to room temperature, and the solvent was distilled off under reduced pressure. After water and ethyl acetate were added to the obtained residue, the organic layer was washed sequentially with a saturated sodium hydrogen carbonate aqueous solution and with a saturated sodium chloride aqueous solution, and dried over anhydrous sodium sulfate, and the solvent was distilled off under reduced pressure. The obtained residue was purified by silica gel column chromatography (chloroform-methanol, NH silica), thereby obtaining (R)-4-(6-chloro-1,5-naphthyridin-3-yl)-3-methylmorpholine (14.3 mg) as a white solid.
MSm/z(M+H):264.

<0762-4>

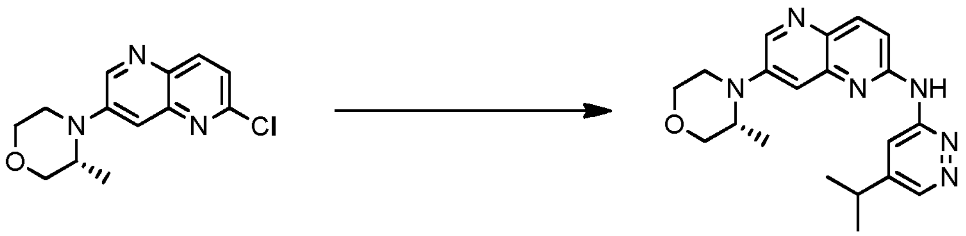

A mixture of (R)-4-(6-chloro-1,5-naphthyridin-3-yl)-3-methylmorpholine (12 mg), sodium tert-butoxide (22 mg), anhydrous sodium sulfate (12 mg), 5-isopropylpyridazine-3-amine (9.3 mg), and 1,4-dioxane (0.5 mL) was stirred at 100°C for 30 minutes using a microwave reaction apparatus. The reaction mixture was cooled to room temperature, and sodium tert-butoxide (22 mg) and 5-isopropylpyridazine-3-amine (9.3 mg) were added thereto, followed by stirring at 150°C for 30 minutes using a microwave reaction apparatus. The reaction mixture was cooled to room temperature, and the solvent was distilled off under reduced pressure. The obtained residue was purified by silica gel column chromatography (chloroform-methanol, NH silica), thereby obtaining (R)-N-(5-isopropylpyridazin-3-yl)-7-(3-methylmorpholino)-1,5-naphthyridine-2-amine (4.9 mg) as a white solid.
$^1$H-NMR(DMSO-$d_6$)$\delta$:10.54(1H,s),8.83(1H,d,J=2.0Hz),8.69(1H,d,J=2.1Hz),8.67(1H,d,J=2.7Hz),8.08(1H,d,J=9.2Hz),7.50(1H,d,J=9.2Hz),7.23(1H,d,J=2.6Hz),4.23-4.21(1H,m),4.02-3.98(1H,m),3.79-3.75(2H,m),3.65-3.61(1H,m),3.49-3.40(1H,m),3.24-3.31(1H,m),3.06-2.96(1H,m),1.30(6H,t,J=6.6Hz),1.11(3H,t,J=6.6Hz).
MSm/z(M+H):365.

<Example 0763>

<0763-1>

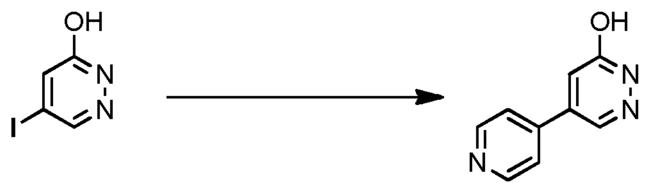

5-(Pyridin-4-yl)pyridazin-3-ol was obtained in the same manner as in Example 0757-1 except that 5-iodopyridazin-3-ol was used instead of the 7-bromo-2-chloro-1,5-naphthyridine used in Example 0757-1, and pyridine-4-boronic acid was used instead of the 1,3-dimethyl-4-(4,4,5,5-tetramethyl-1,3,2-dioxaborolan-2-yl)-1H-pyrazole used in Example 0757-1.
MSm/z(M+H):174.

<0763-2>

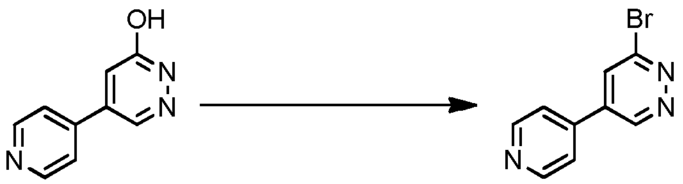

3-Bromo-5-(pyridin-4-yl)pyridazine was obtained in the same manner as in Example 0297-2 except that 5-(pyridin-4-yl)pyridazin-3-ol was used instead of the 5-phenylpyridazin-3(2H)-one used in Example 0297-2, and a mixture of phosphorous oxybromide and acetonitrile was used instead of the phosphorous oxybromide used in Example 0297-2.
MSm/z(M+H):236.

<0763-3>

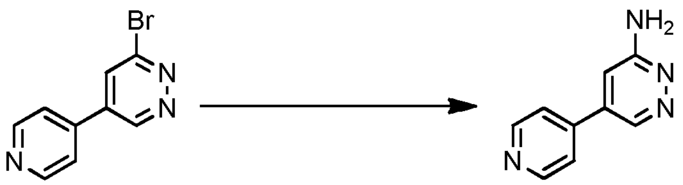

5-(Pyridin-4-yl)pyridazine-3-amine was obtained in the same manner as in Example 0297-3 except that 3-bromo-5-(pyridin-4-yl)pyridazine was used instead of the 3-bromo-5-phenylpyridazine used in Example 0297-3, and 1,4-dioxane was used instead of the ethylene glycol used in Example 0297-3.
MSm/z(M+H):173.

<0763-4>

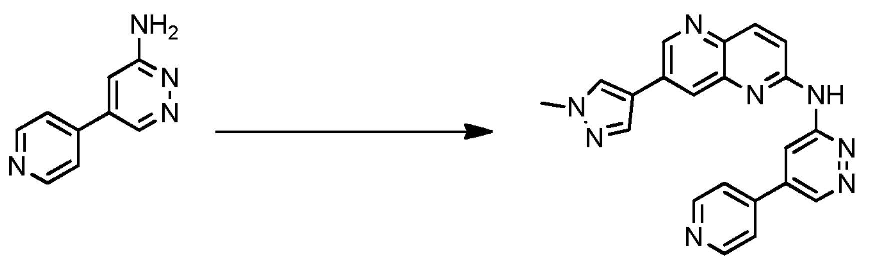

7-(1-Methyl-1H-pyrazol-4-yl)-N-(5-(pyridin-4-yl)pyridazin-3-yl)-1,5-naphthyridine-2-amine was obtained in the same manner as in Example 0145-1 except that 5-(pyridin-4-yl)pyridazine-3-amine was used instead of the 5-isopropylpyridazine-3-amine used in Example 0145-1.

$^1$H-NMR(DMSO-$d_6$)δ:10.99(1H,s),9.36(1H,d,J=2.0Hz),9.18(1H,d,J=2.0Hz),9.06(1H,d,J=2.0Hz),8.84(2H,d,J=5.9Hz),8.48(1H,s),8.29-8.24(2H,m),8.21(1H,m),7.97(2H,d,J=5.9Hz),7.71(1H,d,J=8.6Hz),3.92(1H,s).

MSm/z(M+H):381.

<Example 0764>

<0764-1>

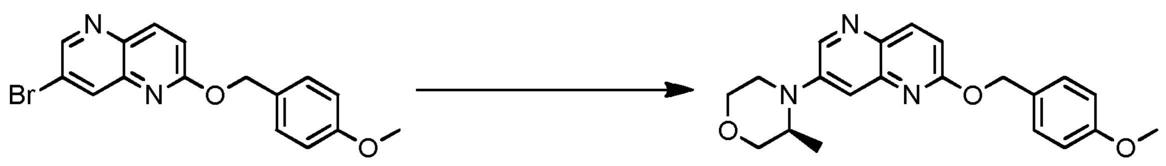

(S)-4-(6-((4-methoxybenzyl)oxy)-1,5-naphthyridin-3-yl)-3-methylmorpholine was obtained in the same manner as in Example 0762-2 except that (S)-3-methylmorpholine was used instead of the (R)-3-methylmorpholine used in Example 0762-2.

MSm/z(M+H):366.

<0764-2>

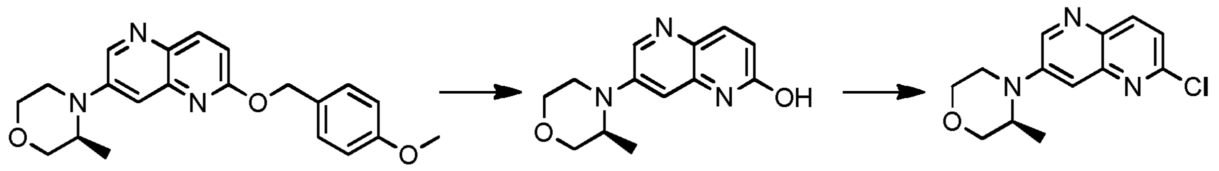

(S)-4-(6-chloro-1,5-naphthyridin-3-yl)-3-methylmorpholine was obtained in the same manner as in Example 0762-3 except that (S)-4-(6-((4-methoxybenzyl)oxy)-1,5-naphthyridin-3-yl)-3-methylmorpholine was used instead of the (R)-4-(6-((4-methoxybenzyl)oxy)-1,5-naphthyridin-3-yl)-3-methylmorpholine used in Example 0762-3.

MSm/z(M+H):264.

<0764-3>

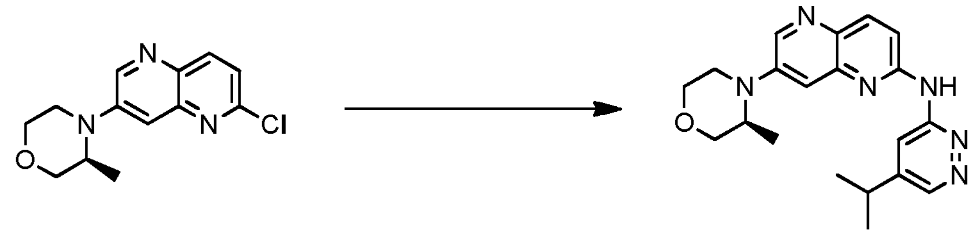

(S)-N-(5-isopropylpyridazin-3-yl)-7-(3-methylmorpholino)-1,5-naphthyridine-2-amine was obtained in the same manner as in Example 0757-2 except that (S)-4-(6-chloro-1,5-naphthyridin-3-yl)-3-methylmorpholine was used instead of the 2-chloro-7-(1,3-dimethyl-1H-pyrazol-4-yl)-1,5-naphthyridine used in Example 0757-2.
$^1$H-NMR(DMSO-$d_6$)δ:10.54(1H,s),8.83(1H,d,J=2.0Hz),8.69(1H,d,J=2.0Hz),8.66(1H,d,J=2.6Hz),8.08(1H,d,J=9.2Hz),7.50(1H,d,J=9.2Hz),7.28(1H,d,J=2.6Hz),4.23-4.21(1H,m),4.02-3.98(1H,m),3.79-3.74(2H,m),3.65-3.60(1H,m),3.48-3.39(1H,m),3.22-3.14(1H,m),3.05-2.96(1H,m),1.30(6H,t,J=7.3Hz),1.10(3H,t,J=6.6Hz).
MSm/z(M+H):365.

<Example 0765>

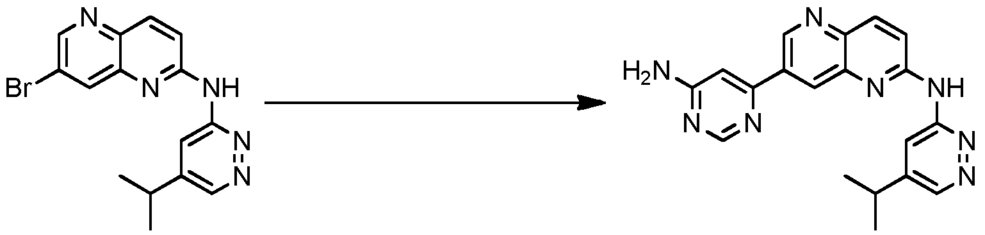

7-(6-Aminopyrimidin-4-yl)-N-(5-isopropylpyridazin-3-yl)-1,5-naphthyridine-2-amine was obtained in the same manner as in Example 0385-7 except that 7-bromo-N-(5-isopropylpyridazin-3-yl)-1,5-naphthyridine-2-amine was used instead of the 7-bromo-2-chloro-1,5-naphthyridine used in Example 0385-7, and 6-chloropyrimidine-4-amine was used instead of the 4-(3-(3-ethyl-4-iodo-1H-pyrazol-1-yl)propyl)morpholine used in Example 0385-7.
$^1$H-NMR(DMSO-$d_6$)δ:10.83(1H,s),9.29(1H,d,J=2.0Hz),8.88(1H,d,J=2.0Hz),8.79(1H,d,J=2.0Hz),8.60(1H,d,J=2.0Hz),8.55(1H,s),8.31(1H,d,J=9.2Hz),7.81(1H,d,J=9.2Hz),7.15-7.05(3H,m),3.10-3.01(1H,m),1.33(6H,t,J=6.6Hz).
MSm/z(M+H):359.

<Example 0766>

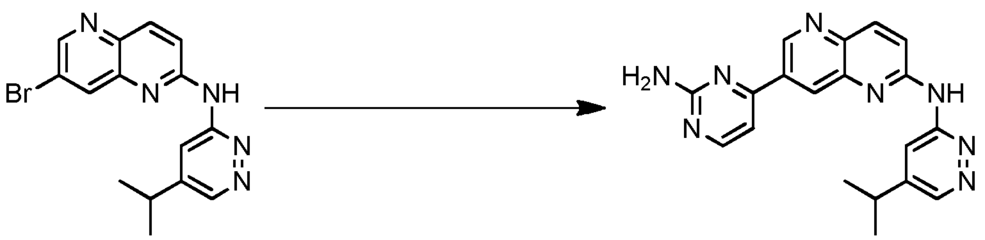

7-(2-Aminopyrimidin-4-yl)-N-(5-isopropylpyridazin-3-yl)-1,5-naphthyridine-2-amine was obtained in the same manner as in Example 0385-7 except that 7-bromo-N-(5-isopropylpyridazin-3-yl)-1,5-naphthyridine-2-amine was used instead of the 7-bromo-2-chloro-1,5-naphthyridine used in Example 0385-7, and 4-chloropyrimidine-2-amine was used instead of the 4-(3-(3-ethyl-4-iodo-1H-pyrazol-1-yl)propyl)morpholine used in Example 0385-7.
$^1$H-NMR(DMSO-

$d_6$)δ:10.82(1H,s),9.38(1H,d,J=2.6Hz),8.89(1H,d,J=2.0Hz),8.75(1H,d,J=1.3Hz),8.68(1H,d,J=1.3Hz),8.43(1H,d,J=5.3Hz),8.32(1H,d,J=8.6Hz)7.83(1H,d,J=8.6Hz),7.41(1H,d,J=5.3Hz),6.86(2H,s),3.09-3.00(1H,m),1.34(6H,d,J=6.6Hz).
MSm/z(M+H):359.

<Example 0767>

<0767-1>

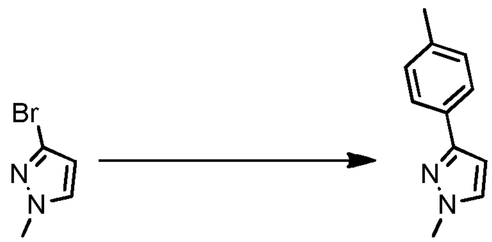

1-Methyl-3-(4-methylphenyl)-1H-pyrazole was obtained in the same manner as in Example 0584-1 except that 4-methylphenylboronic acid was used instead of the 4-methoxyphenylboronic acid used in Example 0584-1.
MSm/z(M+H):173.

<0767-2>

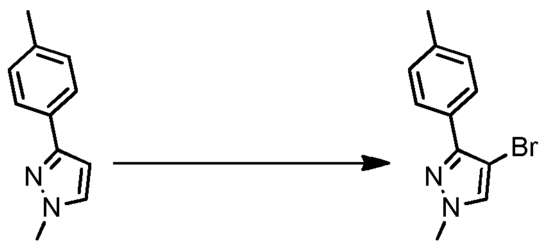

4-Bromo-1-methyl-3-(4-methylphenyl)-1H-pyrazole was obtained in the same manner as in Example 0555-2 except that 1-methyl-3-(4-methylphenyl)-1H-pyrazole was used instead of the 3-methyl-5-(1-methyl-1H-pyrazol-3-yl)pyridine used in Example 0555-2.
MSm/z(M+H):251.

<0767-3>

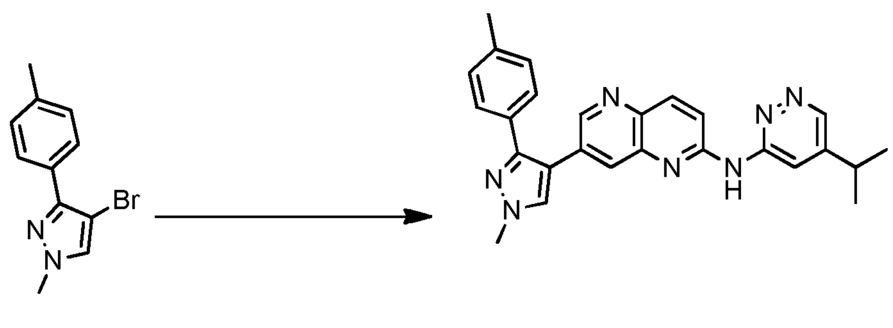

A suspension of 7-bromo-N-(5-isopropylpyridazin-3-yl)-1,5-naphthyridine-2-amine (20 mg), bis(pinacolato)diboron (29 mg), (1,1'-bis(diphenylphosphino)ferrocene)palladium(II) dichloride (5 mg), and potassium acetate (17 mg) in 1,4-dioxane (1 mL) was stirred at 100°C for 1 hour in a nitrogen atmosphere. 4-Bromo-1-methyl-3-(4-methylphenyl)-1H-pyrazole (22 mg), water (0.1 mL), sodium carbonate (18 mg), and bis(di-tert-butyl (4-dimethylaminophenyl)phosphine)dichloropalladium(II) (4 mg) were added to the reaction mixture, followed by stirring at 100°C for 2 hours. The reaction mixture was cooled to room temperature, insolubles were filtered off, and the solvent was distilled off under reduced pressure. The reaction residue was purified by silica gel column chromatography (methanol-ethyl acetate-hexane, NH silica), thereby obtaining N-(5-isopropylpyridazin-3-yl)-7-(1-methyl-3-(4-methylphenyl)-1H-pyrazol-4-yl)-1,5-naphthyridine-2-amine (0.6 mg).
$^1$H-NMR($CDCl_3$)δ:8.76(2H,brs),8.68(1H,d,J=1.8Hz),8.22(1H,d,J=8.4Hz),7.97(1H,brs),7.68(1H,s),7.51(1 H,d,J=8.4Hz),7.39(2H,d,J=8.7Hz),7.14(2H,d,J=8.7Hz),4.04(3H,s),3.07-2.89(1H,m),2.35(3H,s),1.34(6H,d,J=6.6Hz).
MSm/z(M+H):436.

<Example 0768>

<0768-1>

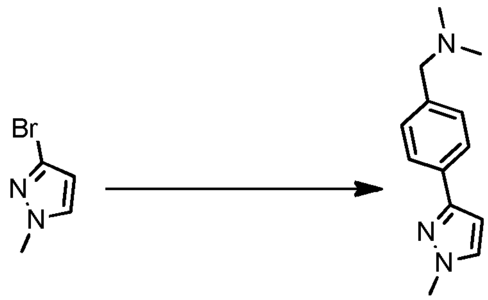

N,N-dimethyl-1-(4-(1-methyl-1H-pyrazol-3-yl)phenyl)methanamine was obtained in the same manner as in Example 0584-1 except that N,N-dimethyl-1-(4-(4,4,5,5-tetramethyl-1,3,2-dioxaborolan-2-yl)phenyl)methanamine was used instead of the 4-methoxyphenylboronic acid used in Example 0584-1.
MSm/z(M+H):216.

<0768-2>

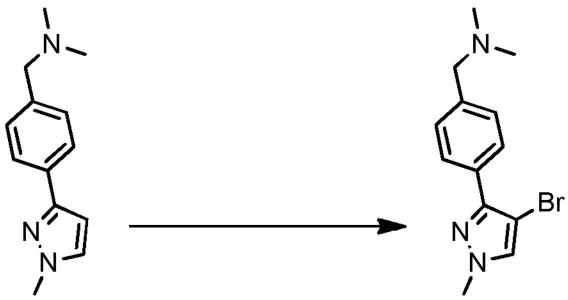

1-(4-(4-Bromo-1-methyl-1H-pyrazol-3-yl)phenyl)-N,N-dimethylmethanamine was obtained in the same manner as in Example 0555-2 except that N,N-dimethyl-1-(4-(1-methyl-1H-pyrazol-3-yl)phenyl)methanamine was used instead of the 3-methyl-5-(1-methyl-1H-pyrazol-3-yl)pyridine used in Example 0555-2.
MSm/z(M+H):294.

<0768-3>

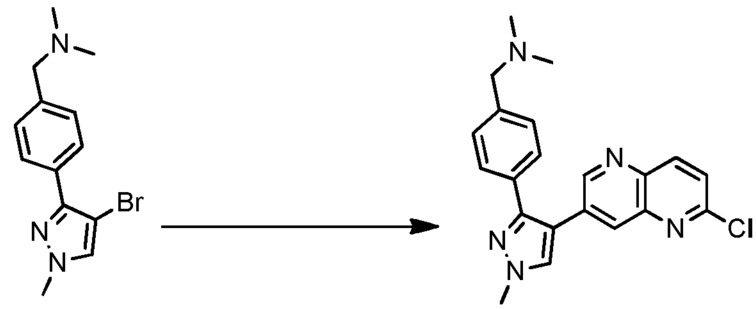

1-(4-(4-(6-Chloro-1,5-naphthyridin-3-yl)-1-methyl-1H-pyrazol-3-yl)phenyl)-N,N-dimethylmethanamine was obtained in the same manner as in Example 0478-3 except that 1-(4-(4-bromo-1-methyl-1H-pyrazol-3-yl)phenyl)-N,N-dimethylmethanamine was used instead of the 1-(4-(4-iodo-1H-pyrazol-1-yl)piperidin-1-yl)ethanone used in Example 0478-3.
MSm/z(M+H):378.

<0768-4>

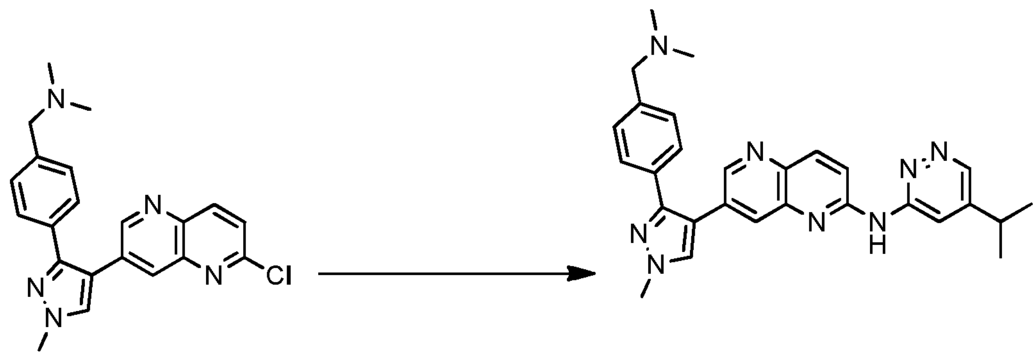

7-(3-(4-((Dimethylamino)methyl)phenyl)-1-methyl-1H-pyrazol-4-yl)-N-(5-isopropylpyridazin-3-yl)-1,5-naphthyridine-2-amine was obtained in the same manner as in Example 0555-4 except that 1-(4-(4-(6-chloro-1,5-naphthyridin-3-yl)-1-methyl-1H-pyrazol-3-yl)phenyl)-N,N-dimethylmethanamine was used instead of the 2-chloro-7-(1-methyl-3-(5-methylpyridin-3-yl)-1H-pyrazol-4-yl)-1,5-naphthyridine used in Example 0555-4.
$^1$H-NMR($CDCl_3$)δ:8.95(1H,brs),8.78(1H,brs),8.65(1H,d,J=2.1Hz),8.23(1H,d,J=9.3Hz),7.98(1H,brs),7.67( 1H,s),7.57(1H,d, J=9.3Hz),7.48(2H,d,J=7.8Hz),7.29(2H,d,J=7.8Hz),4.05(3H,s),3.50(2H,s),3.08-2.90(1H,m),2.29(6H,s),1.34(6H,d,J=7.5 Hz).
MSm/z(M+H):479.

<Example 0769>

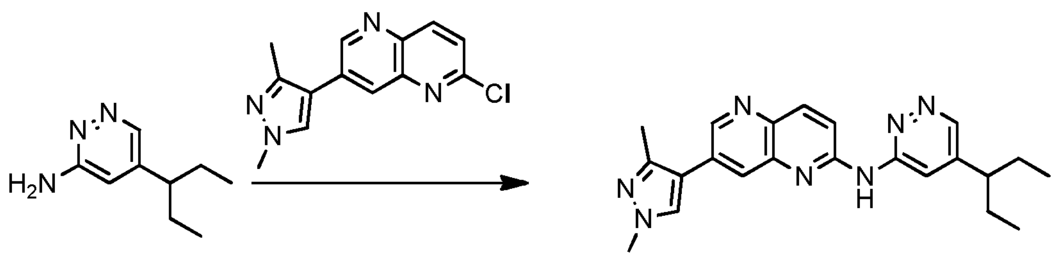

A suspension of 2-chloro-7-(1,3-dimethyl-1H-pyrazol-4-yl)-1,5-naphthyridine (22 mg), 5-(pentan-3-yl)pyridazine-3-amine (14 mg), sodium tert-amyl oxide (28 mg), and anhydrous sodium sulfate (50 mg) in 1,4-dioxane (1 mL) was stirred at 110°C for 7 hours. The reaction mixture was cooled to room temperature, and purified by silica gel column chromatography (methanol-ethyl acetate-hexane, NH silica), thereby obtaining 7-(1,3-dimethyl-1H-pyrazol-4-yl)-N-(5-(pentan-3-yl)pyridazin-3-yl)-1,5-naphthyridine-2-amine (9.8 mg).
$^1$H-NMR(DMSO-$d_6$)δ:10.70(1H,s),8.88(1H,d,J=2.1Hz),8.77(1H,s),8.62(1H,brs),8.24(1H,d,J=9.3Hz),8.23(1H,s),8.00(1H ,brs),7.72(1H,d,J=9.3Hz),3.84(3H,s),2.40(3H,s),2.56-2.51(1H,m),1.84-1.56(4H,m),0.83(6H,t,J=7.2Hz).
MSm/z(M+H):388.

<Example 0770>

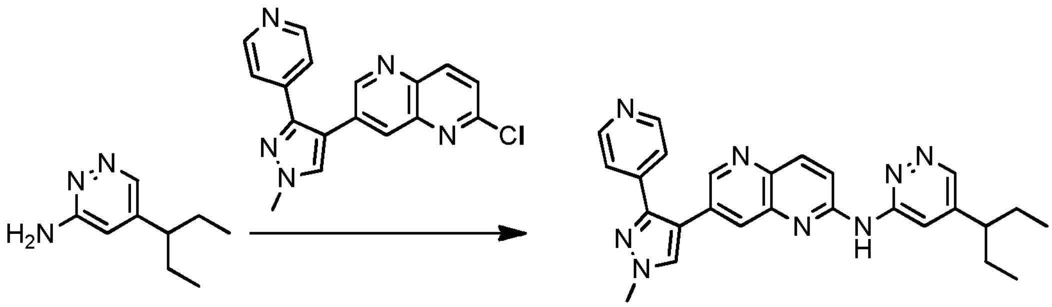

7-(1-Methyl-3-(pyridin-4-yl)-1H-pyrazol-4-yl)-N-(5-(pentan-3-yl)pyridazin-3-yl)-1,5-naphthyridine-2-amine was obtained in the same manner as in Example 0769 except that 2-chloro-7-(1-methyl-3-(pyridin-4-yl)-1H-pyrazol-4-yl)-1,5-naphthyridine was used instead of the 2-chloro-7-(1,3-dimethyl-1H-pyrazol-4-yl)-1,5-naphthyridine used in Example 0769.
$^1$H-NMR($CDCl_3$)δ:9.45(1H,brs),8.72(2H,brs),8.69(1H,brs),8.56(2H,dd,J=4.8,1.8Hz),8.27(1H,d,J=9.0Hz), 7.94(1H,d,J=1.8Hz),7.73(1H,d,J=9.0Hz),7.69(1H,s),7.45(2H,dd,J=4.8,1.8Hz),4.07(3H,s),2.52-2.36(1H,m),1.88-1.48(4H,m),0.85(6H,t,J=7.8Hz).
MSm/z(M+H):451.

<Example 0771>

<0771-1>

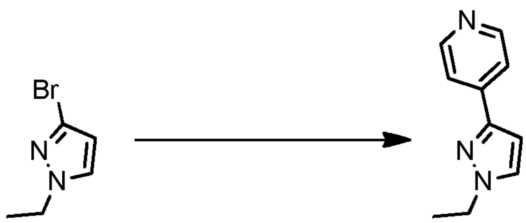

4-(1-Ethyl-1H-pyrazol-3-yl)pyridine was obtained in the same manner as in Example 0576-2 except that 4-pyridineboronic acid was used instead of the 3-pyridineboronic acid used in Example 0576-2.
MSm/z(M+H):174.

<0771-2>

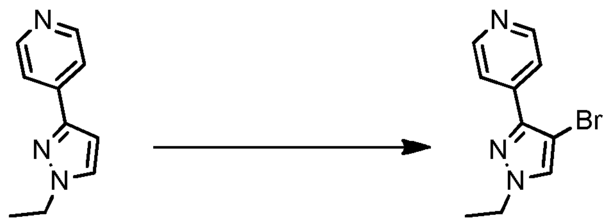

4-(4-Bromo-1-ethyl-1H-pyrazol-3-yl)pyridine was obtained in the same manner as in Example 0555-2 except that 4-(1-ethyl-1H-pyrazol-3-yl)pyridine was used instead of the 3-methyl-5-(1-methyl-1H-pyrazol-3-yl)pyridine used in Example 0555-2.
MSm/z(M+H):252.

<0771-3>

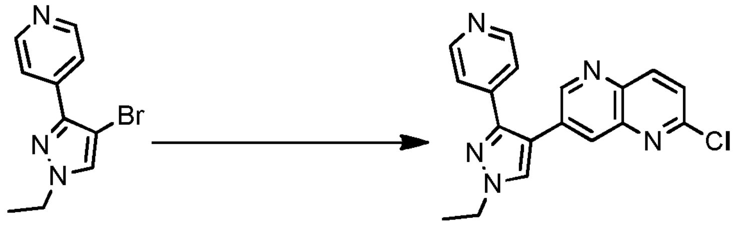

2-Chloro-7-(1-ethyl-3-(pyridin-4-yl)-1H-pyrazol-4-yl)-1,5-naphthyridine was obtained in the same manner as in Example 0478-3 except that 4-(4-bromo-1-ethyl-1H-pyrazol-3-yl)pyridine was used instead of the 1-(4-(4-iodo-1H-pyrazol-1-yl)piperidin-1-yl)ethanone used in Example 0478-3.
MSm/z(M+H):336.

<0771-4>

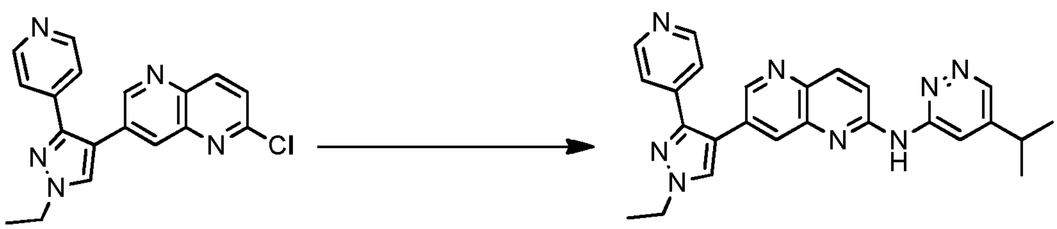

7-(1-Ethyl-3-(pyridin-4-yl)-1H-pyrazol-4-yl)-N-(5-isopropylpyridazin-3-yl)-1,5-naphthyridine-2-amine was obtained in the same manner as in Example 0555-4 except that 2-chloro-7-(1-ethyl-3-(pyridin-4-yl)-1H-pyrazol-4-yl)-1,5-naphthyridine was used instead of the 2-chloro-7-(1-methyl-3-(5-methylpyridin-3-yl)-1H-pyrazol-4-yl)-1,5-naphthyridine used in Example 0555-4.
$^1$H-NMR($CDCl_3$)δ:9.13(1H,brs),8.80(2H,brs),8.69(1H,brs),8.55(2H,dd,J=4.8,1.5Hz),8.27(1H,d,J=9.3Hz), 7.97(1H,d,J=1.8Hz),7.72(1H,s),7.65(1H,d,J=9.3Hz),7.46(2H,dd,J=4.8,1.5Hz),4.33(2H,q,J=7.2Hz),3.0 8-2.90(1H,m),1.64(3H,t,J=7.2Hz),1.33(6H,d,J=7.2Hz).
MSm/z(M+H):437.

<Example 0772>

<0772-1>

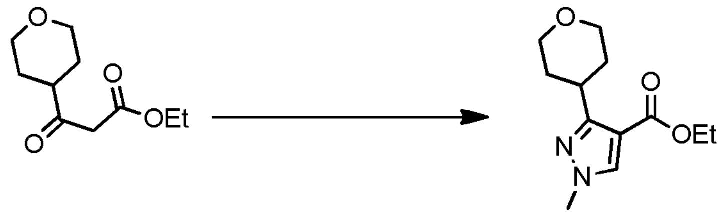

Ethyl 1-methyl-3-(tetrahydro-2H-pyran-4-yl)-1H-pyrazole-4-carboxylate was obtained in the same manner as in Example 0115-1 except that ethyl 3-oxo-3-(tetrahydro-2H-pyran-4-yl)propanoate was used instead of the ethyl 3-oxoheptanoate used in Example 0115-1.
MSm/z(M+H):239.

<0772-2>

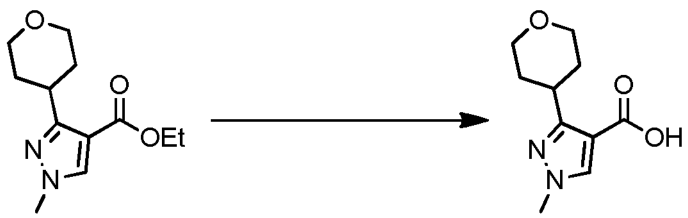

1-Methyl-3-(tetrahydro-2H-pyran-4-yl)-1H-pyrazole-4-carboxylic acid was obtained in the same manner as in Example 0115-2 except that ethyl 1-methyl-3-(tetrahydro-2H-pyran-4-yl)-1H-pyrazole-4-carboxylate was used instead of the ethyl 3-butyl-1-methyl-1H-pyrazole-4-carboxylate used in Example 0115-2.
MSm/z(M+H):211.

<0772-3>

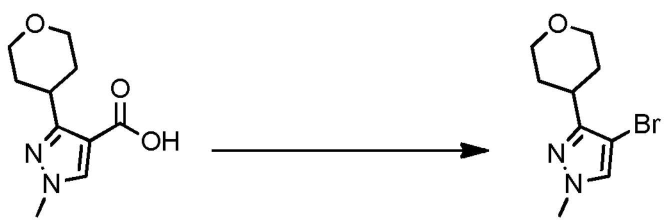

4-Bromo-1-methyl-3-(tetrahydro-2H-pyran-4-yl)-1H-pyrazole was obtained in the same manner as in Example 0115-3 except that 1-methyl-3-(tetrahydro-2H-pyran-4-yl)-1H-pyrazole-4-carboxylic acid was used instead of the 3-butyl-1-methyl-1H-pyrazole-4-carboxylic acid used in Example 0115-3.
$^1$H-NMR($CDCl_3$)$\delta$:7.31(1H,s),4.09-4.01(2H,m),3.84(3H,s),3.53(2H,t,J=9.0,2.7Hz),2.98-2.86(1H,m),2.04-1.76(4H,m).

<0772-4>

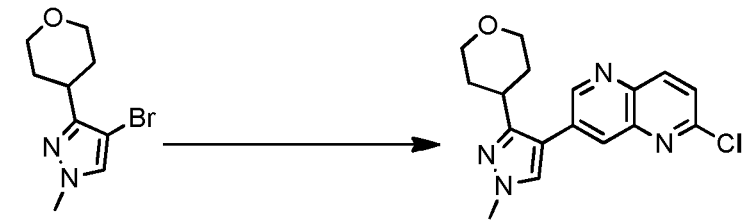

2-Chloro-7-(1-methyl-3-(tetrahydro-2H-pyran-4-yl)-1H-pyrazol-4-yl)-1,5-naphthyridine was obtained in the same manner as in Example 0478-3 except that 4-bromo-1-methyl-3-(tetrahydro-2H-pyran-4-yl)-1H-pyrazole was used instead of the 1-(4-(4-iodo-1H-pyrazol-1-yl)piperidin-1-yl)ethanone used in Example 0478-3.
MSm/z(M+H):329.

<0772-5>

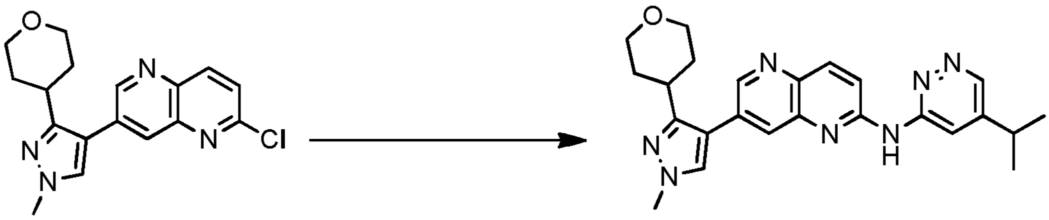

N-(5-isopropylpyridazin-3-yl)-7-(1-methyl-3-(tetrahydro-2H-pyran-4-yl)-1H-pyrazol-4-yl)-1,5-naphthyridine-2-amine was obtained in the same manner as in Example 0555-4 except that 2-chloro-7-(1-methyl-3-(tetrahydro-2H-pyran-4-yl)-1H-pyrazol-4-yl)-1,5-naphthyridine was used instead of the 2-chloro-7-(1-methyl-3-(5-methylpyridin-3-yl)-1H-pyrazol-4-yl)-1,5-naphthyridine used in Example 0555-4.
$^1$H-NMR($CDCl_3$)δ:8.87(1H,brs),8.78(2H,brs),8.27(1H,d,J=9.0Hz),8.00(1H,brs),7.56(1H,s),7.54(1H,d,J=9.0Hz),4.08-4.01(2H,m),3.96(3H,s),3.54-3.43(2H,m),3.08-2.94(1H,m),2.88-2.74(1H,m),2.14-1.94(2H,m),1.88-1.77(2H,m),1.40(6H,d,J=7.5Hz).
MSm/z(M+H):430.

<Example 0773>

<0773-1>

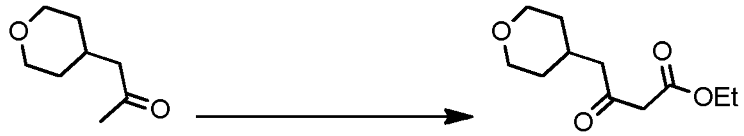

60% sodium hydride (668 mg) was added to a mixture of 1-(tetrahydro-2H-pyran-4-yl)propan-2-one (1.18 g), diethyl carbonate (1.36 mL), and tetrahydrofuran (8 mL) at room temperature, followed by stirring for 4 hours under reflux. After the reaction mixture was cooled to room temperature, a 3 mol/L potassium hydrogen sulfate aqueous solution and ethyl acetate were added thereto, and the organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining ethyl 3-oxo-4-(tetrahydro-2H-pyran-4-yl)butanoate (1.13 g).
MSm/z(M+H):215.

<0773-2>

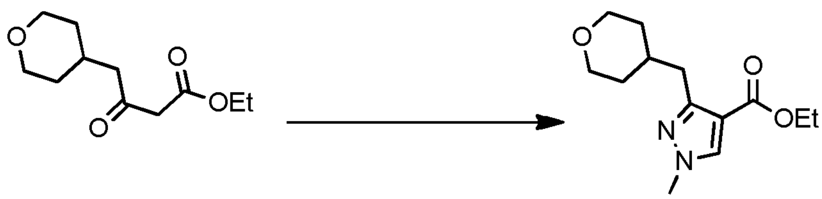

Ethyl 1-methyl-3-((tetrahydro-2H-pyran-4-yl)methyl)-1H-pyrazole-4-carboxylate was obtained in the same manner as in Example 0115-1 except that ethyl 3-oxo-4-(tetrahydro-2H-pyran-4-yl)butanoate was used instead of the ethyl 3-oxoheptanoate used in Example 0115-1.
MSm/z(M+H):253.

<0773-3>

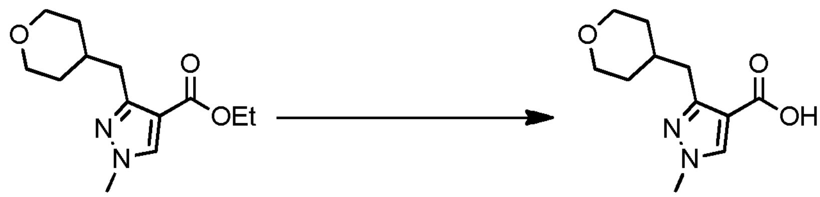

1-Methyl-3-((tetrahydro-2H-pyran-4-yl)methyl)-1H-pyrazole-4-carboxylic acid was obtained in the same manner as in Example 0115-2 except that ethyl 1-methyl-3-((tetrahydro-2H-pyran-4-yl)methyl)-1H-pyrazole-4-carboxylate was used instead of the ethyl 3-butyl-1-methyl-1H-pyrazole-4-carboxylate used in Example 0115-2.
MSm/z(M+H):225.

<0773-4>

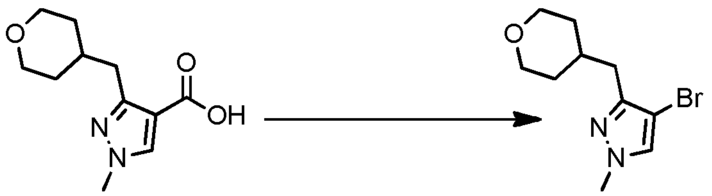

4-Bromo-1-methyl-3-((tetrahydro-2H-pyran-4-yl)methyl)-1H-pyrazole was obtained in the same manner as in Example 0115-3 except that 1-methyl-3-((tetrahy dro-2H-pyran-4-yl)methyl)-1H-pyrazole-4-carboxylic acid was used instead of the 3-butyl-1-methyl-1H-pyrazole-4-carboxylic acid used in Example 0115-3.
$^1$H-NMR($CDCl_3$)$\delta$:7.31(1H,s),3.98-3.91(2H,m),3.83(3H,s),3.42-3.31(2H,m),2.54(2H,d,J=6.6Hz),1.99-1.83(1H,m),1.65-1.56(2H,m),1.47-1.31(2H,m).

<0773-5>

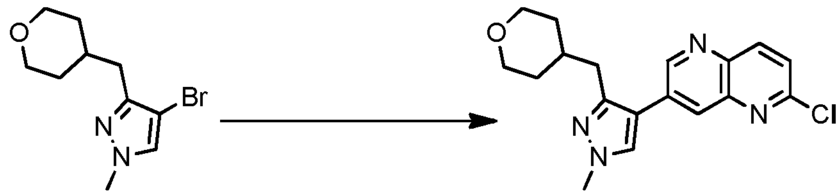

2-Chloro-7-(1-methyl-3-((tetrahydro-2H-pyran-4-yl)methyl)-1H-pyrazol-4-yl)-1,5-naphthyridine was obtained in the same manner as in Example 0478-3 except that 4-bromo-1-methyl-3-((tetrahydro-2H-pyran-4-yl)methyl)-1H-pyrazole was used instead of the 1-(4-(4-iodo-1H-pyrazol-1-yl)piperidin-1-yl)ethanone used in Example 0478-3.
MSm/z(M+H):343.

<0773-6>

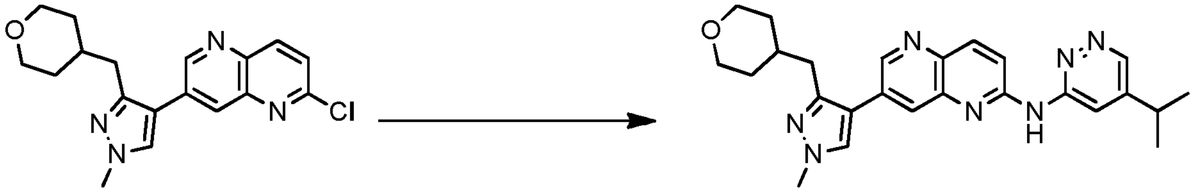

N-(5-isopropylpyridazin-3-yl)-7-(1-methyl-3-((tetrahydro-2H-pyran-4-yl)methyl)-1H-pyrazol-4-yl)-1,5-naphthyridine-2-amine was obtained in the same manner as in Example 0555-4 except that 2-chloro-7-(1-methyl-3-((tetrahydro-2H-pyran-4-yl)methyl)-1H-pyrazol-4-yl)-1,5-naphthyridine was used instead of the 2-chloro-7-(1-methyl-3-(5-methylpyridin-3-yl)-1H-pyrazol-4-yl)-1,5-naphthyridine used in Example 0555-4.
$^1$H-NMR($CDCl_3$)δ:8.82(1H,s),8.80(1H,d,J=2.1Hz),8.62(1H,brs),8.27(1H,d,J=9.3Hz),8.03(1H,brs),7.59(1J ,s),7.52(1H,d,J=9.3Hz),3.96(3H,s),3.94-3.85(2H,m),3.38-3.25(2H,m),3.11-2.93(1H,m),2.79(2H,d,J=7.2Hz),2.03-1.85(1H,m),1.68-1.58(2H,m),1.45-1.29(2H,m),1.40(6H,d,J=7.2Hz).
MSm/z(M+H):444.

<Example 0774>

<0774-1>

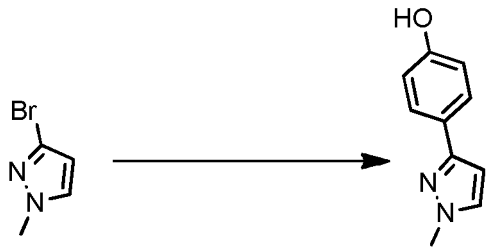

4-(1-Methyl-1H-pyrazol-3-yl)phenol was obtained in the same manner as in Example 0584-1 except that (4-hydroxyphenyl)boronic acid was used instead of the 3-pyridineboronic acid used in Example 0584-1.
MSm/z(M+H):175.

<0774-2>

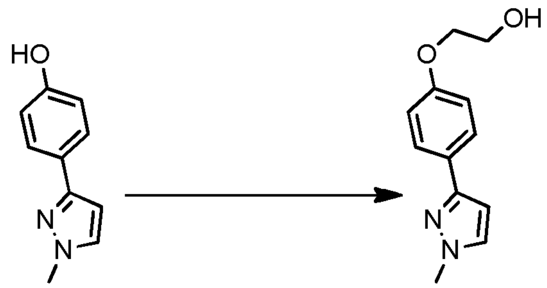

A suspension of 4-(1-methyl-1H-pyrazol-3-yl)phenol (263 mg), 2-bromoethanol (0.129 mL), and potassium carbonate (417 mg) in N,N-dimethylformamide (3 mL) was stirred at 120°C for 19 hours. After the reaction mixture was cooled to room temperature, ethyl acetate and water were added thereto. The organic layer was collected by separation, and dried over anhydrous sodium sulfate, and the solvent was distilled off under reduced pressure. The obtained residue was purified by silica gel column chromatography (methanol-ethyl acetate-hexane), thereby obtaining 2-(4-(1-methyl-1H-pyrazol-3-yl)phenoxy)ethanol (274 mg).
MSm/z(M+H):219.

<0774-3>

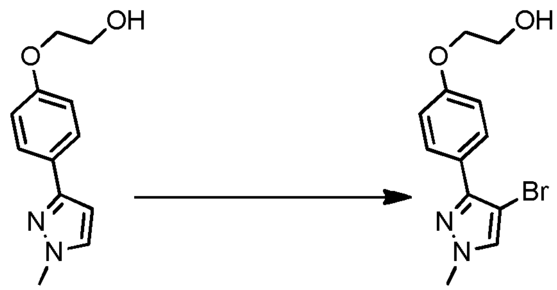

2-(4-(4-Bromo-1-methyl-1H-pyrazol-3-yl)phenoxy)ethanol was obtained in the same manner as in Example 0555-2 except that 2-(4-(1-methyl-1H-pyrazol-3-yl)phenoxy)ethanol was used instead of the 3-methyl-5-(1-methyl-1H-pyrazol-3-yl)pyridine used in Example 0555-2.
MSm/z(M+H):297.

<0774-4>

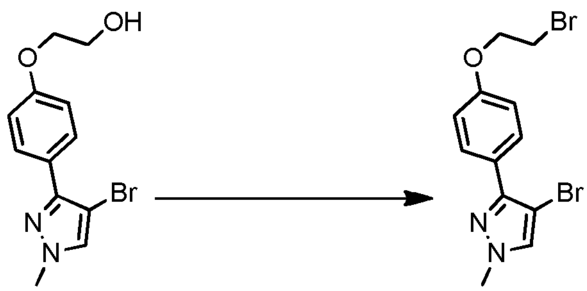

Triphenylphosphine (490 mg) was added to a mixture of 2-(4-(4-bromo-1-methyl-1H-pyrazol-3-yl)phenoxy)ethanol (237 mg), carbon tetrabromide (621 mg), and dichloromethane (3 mL) under ice-cooling, followed by stirring at room temperature for 3 hours. The solvent of the reaction mixture was distilled off under reduced pressure, and the obtained residue was purified by silica gel column chromatography (ethyl acetate-hexane), thereby obtaining 4-bromo-3-(4-(2-bromoethoxy)phenyl)-1-methyl-1H-pyrazole (74 mg).
MSm/z(M+H):359.

<0774-5>

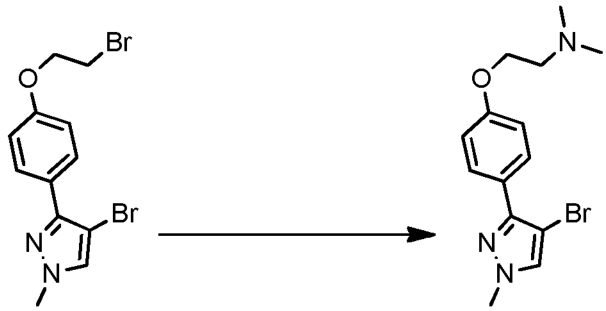

50% dimethylamine aquaous solution (0.5 mL) was added to a mixture of 4-bromo-3-(4-(2-bromoethoxy)phenyl)-1-methyl-1H-pyrazole (74 mg) and tetrahydrofuran (2 mL) at room temperature, followed by stirring at 50°C for 8 hours. The solvent of the reaction mixture was distilled off under reduced pressure, thereby obtaining 2-(4-(4-bromo-1-methyl-1H-pyrazol-3-yl)phenoxy)-N,N-dimethylethanamine (93 mg).
MSm/z(M+H):324.

<0774-6>

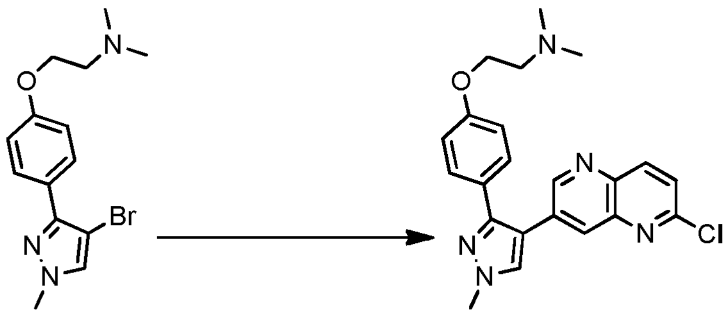

2-(4-(4-(6-Chloro-1,5-naphthyridin-3-yl)-1-methyl-1H-pyrazol-3-yl)phenoxy)-N,N-dimethylethanamine was obtained in the same manner as in Example 0478-3 except that 2-(4-(4-bromo-1-methyl-1H-pyrazol-3-yl)phenoxy)-N,N-dimethylethanamine was used instead of the 1-(4-(4-iodo-1H-pyrazol-1-yl)piperidin-1-yl)ethanone used in Example 0478-3.
MSm/z(M+H):408.

<0774-7>

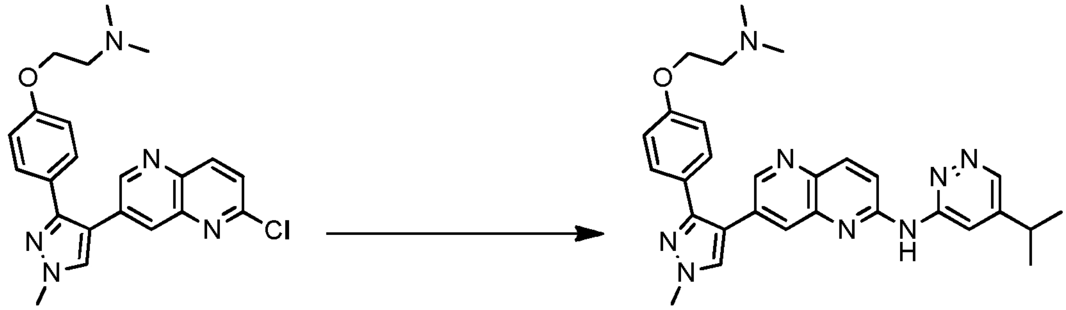

7-(3-(4-(2-(Dimethylamino)ethoxy)phenyl)-1-methyl-1H-pyrazol-4-yl)-N-(5-isopropylpyridazin-3-yl)-1,5-naphthyridine-2-amine was obtained in the same manner as in Example 0555-4 except that 2-(4-(4-(6-chloro-1,5-naphthyridin-3-yl)-1-methyl-1H-pyrazol-3-yl)phenoxy)-N,N-dimethylethanamine was used instead of the 2-chloro-7-(1-methyl-3-(5-methylpyridin-3-yl)-1H-pyrazol-4-yl)-1,5-naphthyridine used in Example 0555-4.
$^1$H-NMR($CDCl_3$)δ:9.49(1H,brs),8.87(1H,brs),8.79(1H,d,J=2.1Hz),8.67(1H,d,J=2.1Hz),8.23(1H,d,J=9.0Hz),7.97(1H,brs),7.68(1H,d,J=9.0Hz),7.66(1H,s),7.43(2H,d,J=8.7Hz),6.89(2H,d,J=8.7Hz),4.05(2H,t,J=6 .0Hz),4.03(3H,s),3.08-2.90(1H,m),2.72(2H,t,J=6.0Hz),2.32(6H,s),1.34(6H,d,J=6.6Hz).
MSm/z(M+H):509.

<Example 0775>

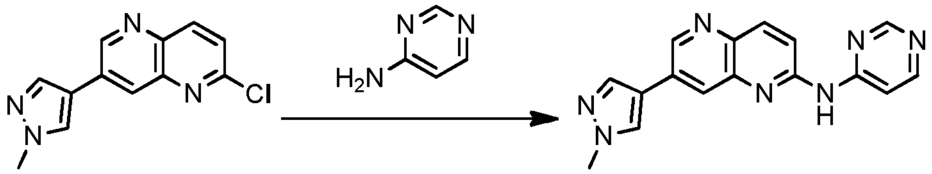

7-(1-Methyl-1H-pyrazol-4-yl)-N-(pyrimidin-4-yl)-1,5-naphthyridine-2-amine was obtained in the same manner as in Example 0489-4 except that pyrimidine-4-amine was used instead of the 4-methylpyridazine-3-amine used in Example 0489-4.
$^1$H-NMR($CDCl_3$/$CD_3OD$=4/1)δ:8.93(1H,d,J=1.8Hz),8.78(1H,s),8.61(1H,d,J=6.0Hz),8.53(1H,d,J=6.0Hz),8.28(1H,d,J=1.8Hz),8.23(1H,d,J=9.3Hz),7.99(1H,s),7.98(1H,s),7.58(1H,d,J=9.3Hz),4.03(3H,s).
MSm/z(M+H):304.

<Example 0776>

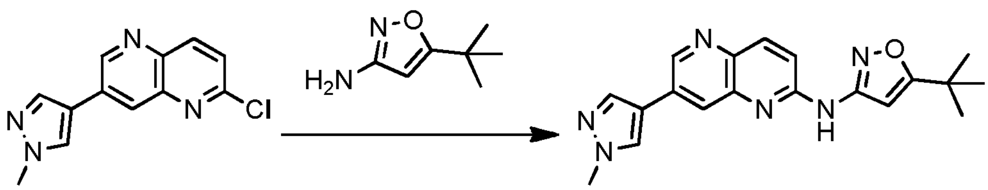

5-(tert-Butyl)-N-(7-(1-methyl-1H-pyrazol-4-yl)-1,5-naphthyridin-2-yl)isoxazole-3-amine was obtained in the same manner as in Example 0554-3 except that 5-(tert-butyl)isoxazole-3-amine was used instead of the 5-morpholinopyridazine-3-amine used in Example 0554-3.
$^1$H-NMR($CDCl_3$)δ:9.05(1H,d,J=2.1Hz),8.77(1H,d,J=2.1Hz),8.05(1H,d,J=9.3Hz),8.00(1H,s),7.90(1H,s),7.79(1H,d,J=9.3Hz),6.72(1H,brs),4.02(3H,s),1.59(9H,s).
MSm/z(M+H):349.

<Example 0777>

<0777-1>

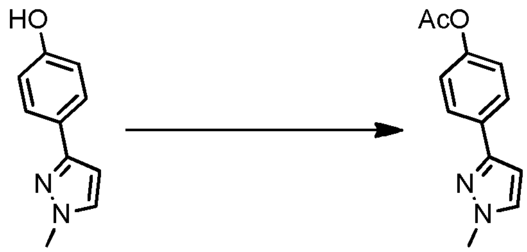

Acetyl chloride (0.143 mL) was added to a mixture of 4-(1-methyl-1H-pyrazol-3-yl)phenol (236 mg), triethylamine (0.283 mL), and tetrahydrofuran (7 mL) under ice-cooling, followed by stirring at room temperature for 1 hour. After water and ethyl acetate were added to the reaction mixture, the organic layer was collected by separation, washed with a saturated sodium chloride aqueous solution, and dried over anhydrous sodium sulfate, and the solvent was distilled off under reduced pressure. The obtained residue was purified by silica gel column chromatography (ethyl acetate-hexane), thereby obtaining 4-(1-methyl-1H-pyrazol-3-yl)phenyl acetate (249 mg).
MSm/z(M+H):217.

<0777-2>

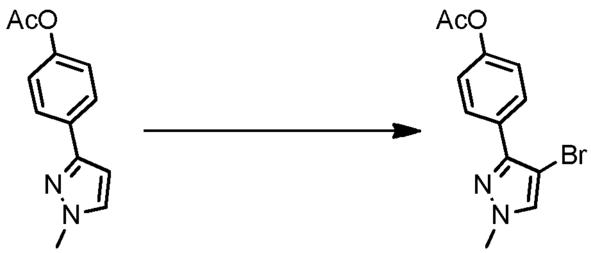

4-(4-Bromo-1-methyl-1H-pyrazol-3-yl)phenyl acetate was obtained in the same manner as in Example 0555-2 except that 4-(1-methyl-1H-pyrazol-3-yl)phenyl acetate was used instead of the 3-methyl-5-(1-methyl-1H-pyrazol-3-yl)pyridine used in Example 0555-2.
MSm/z(M+H):295.

<0777-3>

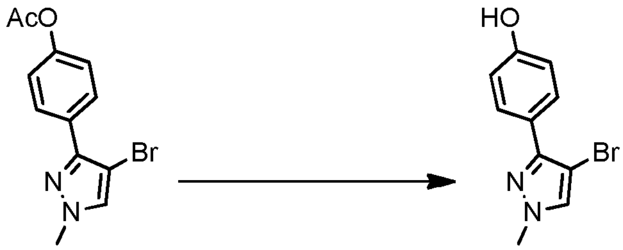

A 4 mol/L sodium hydroxide aqueous solution (0.6 mL) was added to a solution of 4-(4-bromo-1-methyl-1H-pyrazol-3-yl)phenyl acetate (381 mg) in tetrahydrofuran (2 mL), followed by stirring at room temperature for 3 hours. The reaction mixture was purified by silica gel column chromatography (ethyl acetate-hexane), thereby obtaining 4-(4-bromo-1-methyl-1H-pyrazol-3-yl)phenol (243 mg).

MSm/z(M+H):253.

<0777-4>

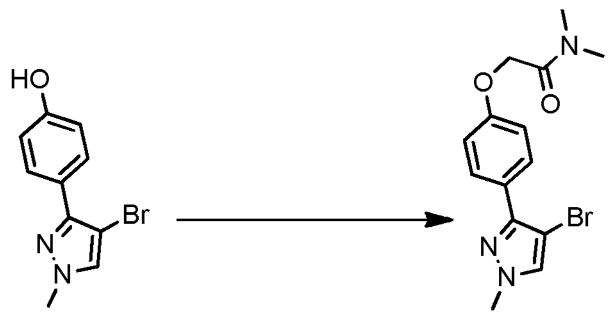

A mixture of 4-(4-bromo-1-methyl-1H-pyrazol-3-yl)phenol (45 mg), 2-chloro-N,N-dimethylacetamide (0.063 mL), cesium carbonate (252 mg), sodium iodide (77 mg), acetonitrile (1 mL), and tetrahydrofuran (0.5 mL) was stirred at 50°C for 19 hours. The reaction mixture was cooled to room temperature, and purified by silica gel column chromatography (methanol-ethyl acetate-hexane), thereby obtaining 2-(4-(4-bromo-1-methyl-1H-pyrazol-3-yl)phenoxy)-N,N-dimethylacetamide (67 mg).
MSm/z(M+H):338.

<0777-5>

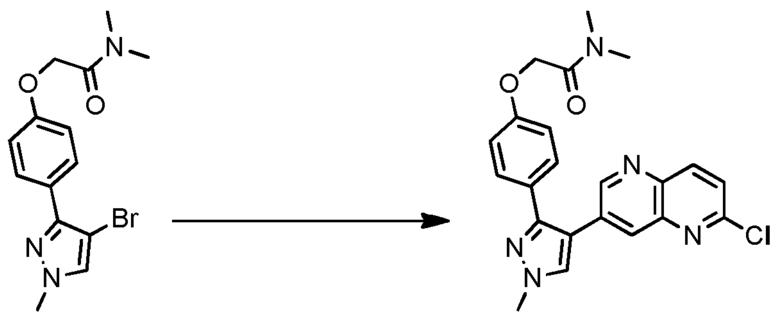

2-(4-(4-(6-Chloro-1,5-naphthyridin-3-yl)-1-methyl-1H-pyrazol-3-yl)phenoxy)-N,N-dimethylacetamide was obtained in the same manner as in Example 0478-3 except that 2-(4-(4-bromo-1-methyl-1H-pyrazol-3-yl)phenoxy)-N,N-dimethylacetamide was used instead of the 1-(4-(4-iodo-1H-pyrazol-1-yl)piperidin-1-yl)ethanone used in Example 0478-3.
MSm/z(M+H):422.

<0777-6>

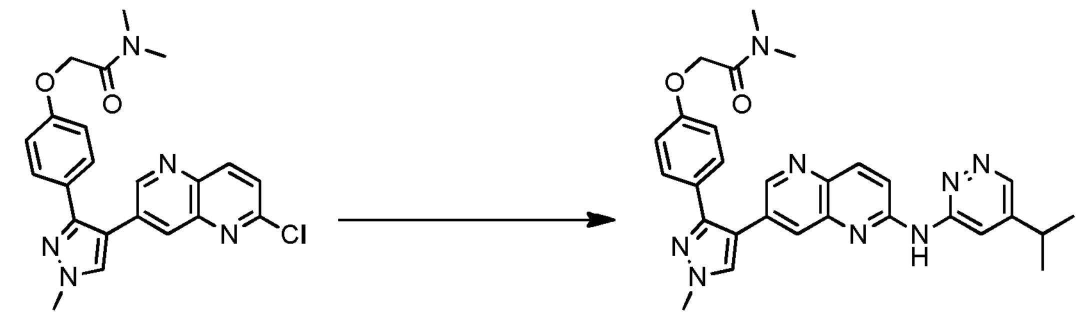

2-(4-(4-(6-((5-Isopropylpyridazin-3-yl)amino)-1,5-naphthyridin-3-yl)-1-methyl-1H-pyrazol-3-yl)phenoxy)-N,N-dimethylacetamide was obtained in the same manner as in Example 0555-4 except that 2-(4-(4-(6-chloro-1,5-naphthyridin-3-yl)-1-methyl-1H-pyrazol-3-yl)phenoxy)-N,N-dimethylacetamide was used instead of the 2-chloro-7-(1-methyl-3-(5-methylpyridin-3-yl)-1H-pyrazol-4-yl)-1,5-naphthyridine used in Example 0555-4.
$^1$H-NMR($CDCl_3$)δ:9.35(1H,brs),8.84(1H,brs),8.79(1H,d,J=2.1Hz),8.66(1H,d,J=2.1Hz),8.23(1H,d,J=8.7Hz),7.98(1H,d,J=2.1Hz),7.67(1H,d,J=8.7Hz),7.65(1H,s),7.44(2H,d,J=8.7Hz),6.92(2H,d,J=8.7Hz),4.66(2H,s),4.03(3H,s),3.10-2.94(1H,m),3.07(3H,s),2.96(3H,s),1.35(6H,d,J=7.2Hz).
MSm/z(M+H):523.

<Example 0778>

<0778-1>

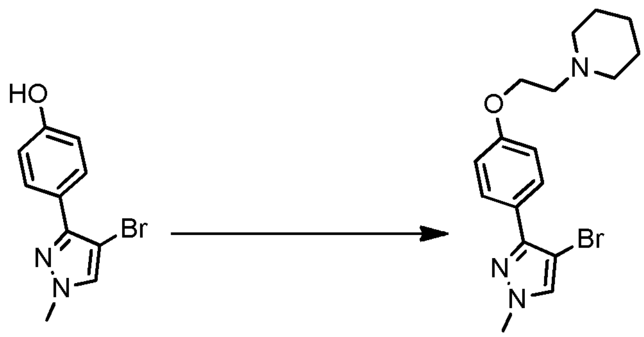

A 2.2 mol/L diethyl azodicarboxylate toluene solution (0.195 mL) was added to a mixture of 4-(4-bromo-1-methyl-1H-pyrazol-3-yl)phenol (50 mg), 2-(piperidin-1-yl)ethanol (0.056 mL), triphenylphosphine (112 mg), and tetrahydrofuran (3 mL) under ice-cooling, followed by stirring at room temperature for 3 hours. The reaction mixture was purified by silica gel column chromatography (methanol-ethyl acetate-hexane), thereby obtaining 1-(2-(4-(-methyl-1H-pyrazol-3-yl)phenoxy)ethyl)piperidine (70 mg).
MSm/z(M+H):364.

<0778-2>

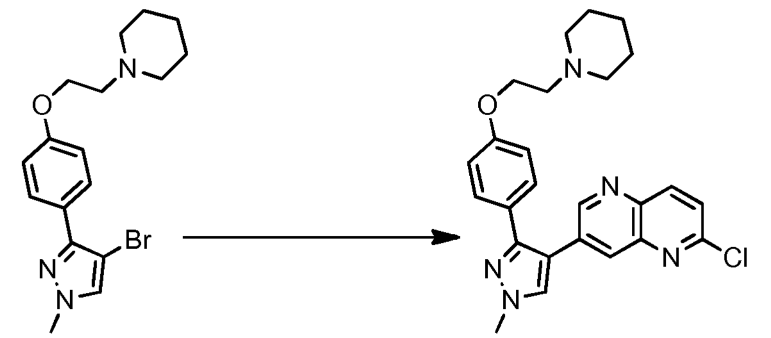

2-Chloro-7-(1-methyl-3-(4-(2-(piperidin-1-yl)ethoxy)phenyl)-1H-pyrazol-4-yl)-1,5-naphthyridine was obtained in the same manner as in Example 0478-3 except that 1-(2-(4-(4-bromo-1-methyl-1H-pyrazol-3-yl)phenoxy)ethyl)piperidine was used instead of the 1-(4-(4-iodo-1H-pyrazol-1-yl)piperidin-1-yl)ethanone used in Example 0478-3.
MSm/z(M+H):448.

<0778-3>

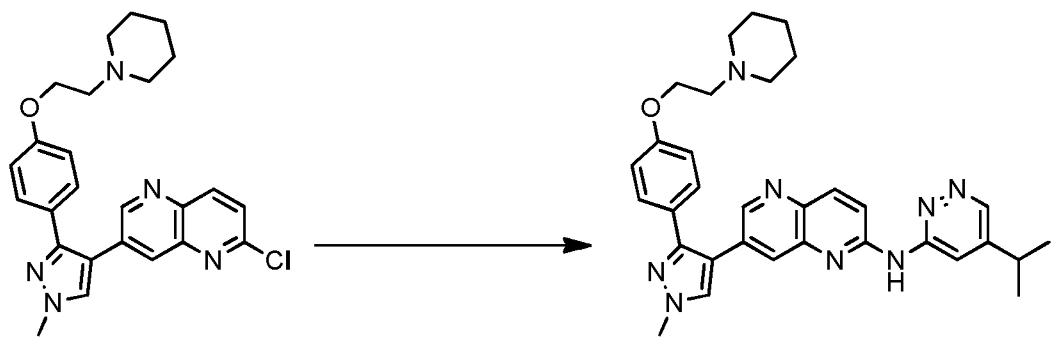

N-(5-isopropylpyridazin-3-yl)-7-(1-methyl-3-(4-(2-(piperidin-1-yl)ethoxy)phenyl)-1H-pyrazol-4-yl)-1,5-naphthyridine-2-amine was obtained in the same manner as in Example 0555-4 except that 2-chloro-7-(1-methyl-3-(4-(2-(piperidin-1-yl)ethoxy)phenyl)-1H-pyrazol-4-yl)-1,5-naphthyridine was used instead of the 2-chloro-7-(1-methyl-3-(5-methylpyridin-3-yl)-1H-pyrazol-4-yl)-1,5-naphthyridine used in Example 0555-4.
$^1$H-NMR($CDCl_3$)δ:9.23(1H,brs),8.84(1H,brs),8.78(1H,brs),8.66(1H,brs),8.23(1H,d,J=9.3Hz),7.97(1H,brs) ,7.66(1H,s),7.63 (1H,d,J=9.3Hz),7.42(2H,d,J=9.0Hz),6.87(2H,d,J=9.0Hz),4.15(2H,t,J=6.0Hz),4.03(3H, s),3.07-2.90(1H,m),2.86(2H,brs),2.60(4H,brs),1.82-1.42(6H,m),1.34(6H,d,J=7.2Hz).
MSm/z(M+H):549.

<Example 0779>

<0779-1>

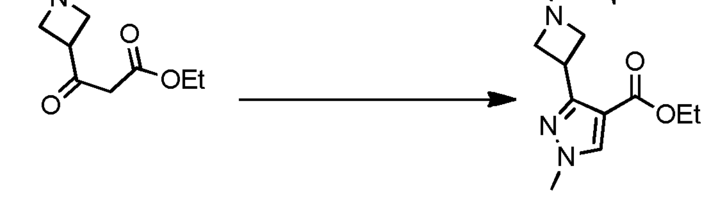

Ethyl 3-(1-(tert-butoxycarbonyl)azetidin-3-yl)-1-methyl-1H-pyrazole-4-carboxylate was obtained in the same manner as in Example 0115-1 except that tert-butyl 3-(3-ethoxy-3-oxopropanoyl)azetidine-1-carboxylate was used instead of the ethyl 3-oxoheptanoate used in Example 0115-1.
MSm/z(M+H):310.

<0779-2>

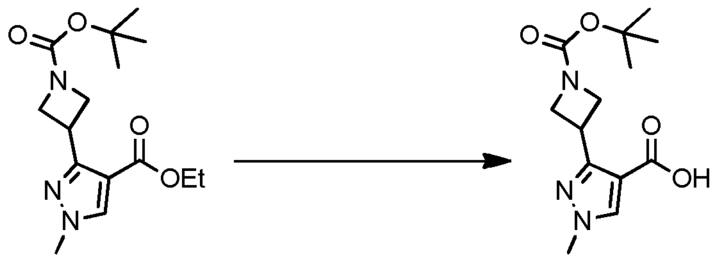

3-(1-(tert-Butoxycarbonyl)azetidin-3-yl)-1-methyl-1H-pyrazole-4-carboxylic acid was obtained in the same manner as in Example 0115-2 except that ethyl 3-(1-(tert-butoxycarbonyl)azetidin-3-yl)-1-methyl-1H-pyrazole-4-carboxylate was used instead of the ethyl 3-butyl-1-methyl-1H-pyrazole-4-carboxylate used in Example 0115-2.
MSm/z(M+H):282.

<0779-3>

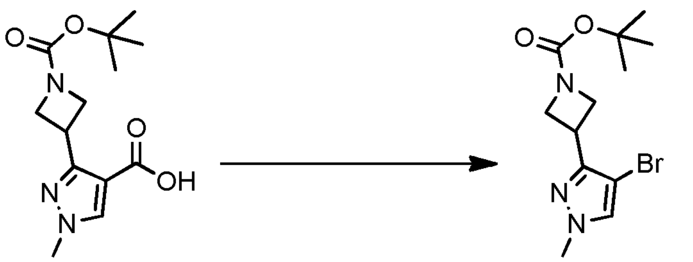

tert-Butyl 3-(4-bromo-1-methyl-1H-pyrazol-3-yl)azetidine-1-carboxylate was obtained in the same manner as in Example 0115-3 except that 3-(1-(tert-butoxycarbonyl)azetidin-3-yl)-1-methyl-1H-pyrazole-4-carboxylic acid was used instead of the 3-butyl-1-methyl-1H-pyrazole-4-carboxylic acid used in Example 0115-3.
MSm/z(M+H):316.

<0779-4>

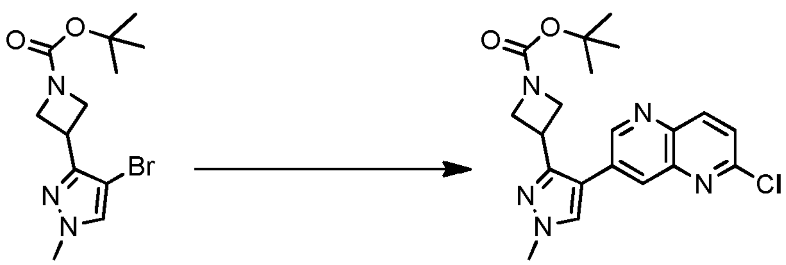

tert-Butyl 3-(4-(6-chloro-1,5-naphthyridin-3-yl)-1-methyl-1H-pyrazol-3-yl)azetidine-1-carboxylate was obtained

in the same manner as in Example 0478-3 except that tert-butyl 3-(4-bromo-1-methyl-1H-pyrazol-3-yl)azetidine-1-carboxylate was used instead of the 1-(4-(4-iodo-1H-pyrazol-1-yl)piperidin-1-yl)ethanone used in Example 0478-3.
MSm/z(M+H):400.

<0779-5>

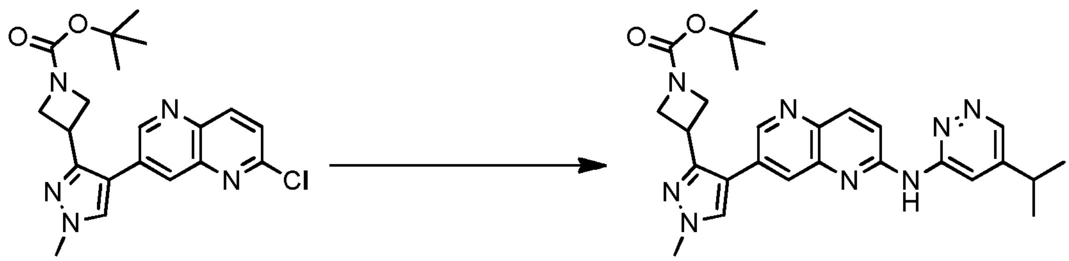

tert-Butyl 3-(4-(6-((5-isopropylpyridazin-3-yl)amino)-1,5-naphthyridin-3-yl)-1-methyl-1H-pyrazol-3-yl)azetidine-1-carboxylate was obtained in the same manner as in Example 0555-4 except that tert-butyl 3-(4-(6-chloro-1,5-naphthyridin-3-yl)-1-methyl-1H-pyrazol-3-yl)azetidine-1-carboxylate was used instead of the 2-chloro-7-(1-methyl-3-(5-methylpyridin-3-yl)-1H-pyrazol-4-yl)-1,5-naphthyridine used in Example 0555-4.
$^1$H-NMR($CDCl_3$)$\delta$:8.81(2H,brs),8.70(1H,brs),8.26(1H,d,J=8.7Hz),7.86(1H,d,J=2.1Hz),7.65(1H,s),7.52(1H,d,J=8.7Hz),4.76-4.58(1H,m),4.34-4.23(4H,m),3.99(3H,s),3.11-2.95(1H,m),1.43(9H,s),1.41(6H,d,J=6.6Hz).
MSm/z(M+H):501.

<Example 0780>

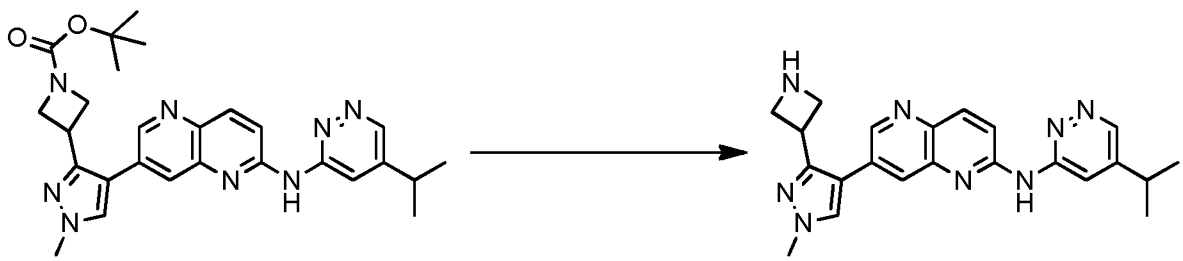

Trifluoroacetic acid (1 mL) was added to a mixture of tert-butyl 3-(4-(6-((5-isopropylpyridazin-3-yl)amino)-1,5-naphthyridin-3-yl)-1-methyl-1H-pyrazol-3-yl)azetidine-1-carboxylate (3 mg), and water (0.05 mL), followed by stirring at room temperature for 1 hour. The solvent of the reaction mixture was distilled off under reduced pressure, and the obtained residue was purified by silica gel column chromatography (methanol-ethyl acetate, NH silica), thereby obtaining 7-(3-(azetidin-3-yl)-1-methyl-1H-pyrazol-4-yl)-N-(5-isopropylpyridazin-3-yl)-1,5-naphthyridine-2-amine (1 mg).
$^1$H-NMR($CDCl_3$)$\delta$:8.86(1H,brs),8.81(1H,brs),8.71(1H,d,J=1.8Hz),8.26(1H,d,J=9.0Hz),7.89(1H,brs),7.62( 1H,s),7.58(1H,d,J=9.0Hz),4.29-4.16(1H,m),4.11(2H,t,J=7.2Hz),4.00(3H,s),3.91(2H,t,J=7.2Hz),3.10-2.95(1H,m),1.41(6H,d,J=7.2Hz).
MSm/z(M+H):401.

<Example 0781>

<0781-1>

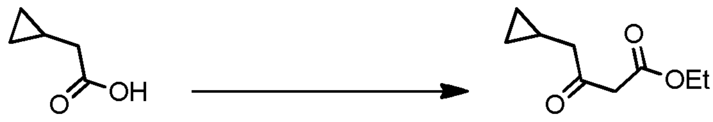

Carbonyldiimidazole (1.94 g) was added to a mixture of 2-cyclopropylacetic acid (0.93 mL), Meldrum's acid (2.16 g), N,N-dimethylpyridine-4-amine (1.83 g), and dichloromethane (30 mL) under ice-cooling, followed by stirring at room temperature for 15 hours. After 1 mol/L hydrochloric acid was added to the reaction mixture, the organic layer was collected by separation, washed with a saturated sodium chloride aqueous solution, and dried over anhydrous sodium sulfate, and the solvent was distilled off under reduced pressure. Ethanol (20 mL) was added to the obtained residue, followed by stirring for 7 hours under reflux. The solvent was distilled off under reduced pressure, and the obtained residue was purified by silica gel column chromatography (ethyl acetate-hexane), thereby obtaining ethyl 4-cyclopropyl-3-oxobutanoate (528 mg).
$^1H$-NMR($CDCl_3$)$\delta$:4.20(2H,q,J=7.2Hz),3.49(2H,s),2.42(2H,d,J=6.6Hz),1.28(3H,t,J=7.2Hz),1.08-0.87(1H,m),0.64-0.51(2H,m),0.18-0.11(2H,m).

<0781-2>

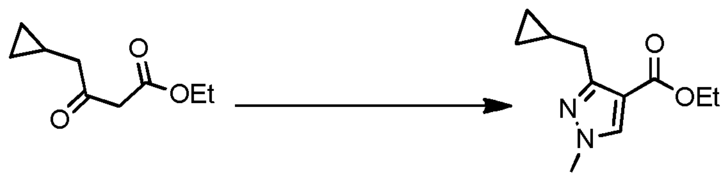

Ethyl 3-(cyclopropylmethyl)-1-methyl-1H-pyrazole-4-carboxylate was obtained in the same manner as in Example 0115-1 except that 4-cyclopropyl-3-oxobutanoate was used instead of the ethyl 3-oxoheptanoate used in Example 0115-1.
MSm/z(M+H):209.

<0781-3>

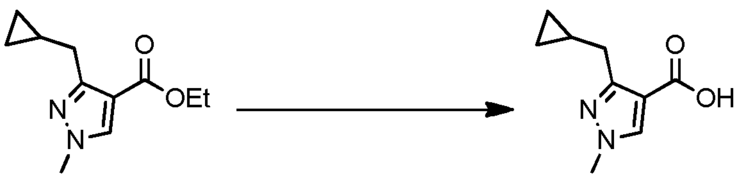

3-(Cyclopropylmethyl)-1-methyl-1H-pyrazole-4-carboxylic acid was obtained in the same manner as in Example 0115-2 except that ethyl 3-(cyclopropylmethyl)-1-methyl-1H-pyrazole-4-carboxylate was used instead of the ethyl 3-butyl-1-methyl-1H-pyrazole-4-carboxylate used in Example 0115-2.
MSm/z(M+H):181.

<0781-4>

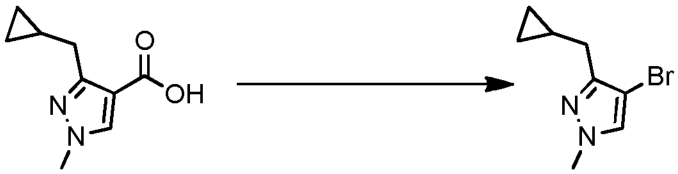

4-Bromo-3-(cyclopropylmethyl)-1-methyl-1H-pyrazole was obtained in the same manner as in Example 0115-3 except that 3-(cyclopropylmethyl)-1-methyl-1H-pyrazole-4-carboxylic acid was used instead of the 3-butyl-1-methyl-1H-pyrazole-4-carboxylic acid used in Example 0115-3.
$^1$H-NMR($CDCl_3$)δ:7.31(1H,s),3.84(3H,s),2.52(2H,d,J=6.6Hz),1.16-1.02(1H,m),0.52-0.44(2H,m),0.27-0.21(2H,m).

<0781-5>

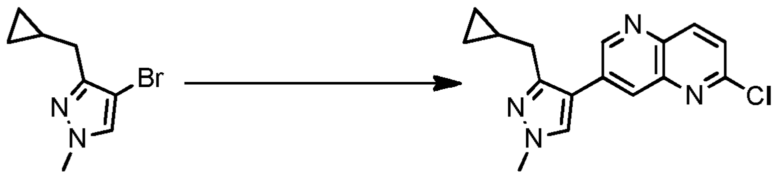

2-Chloro-7-(3-(cyclopropylmethyl)-1-methyl-1H-pyrazol-4-yl)-1,5-naphthyridine was obtained in the same manner as in Example 0478-3 except that 4-bromo-3-(cyclopropylmethyl)-1-methyl-1H-pyrazole was used instead of the 1-(4-(4-iodo-1H-pyrazol-1-yl)piperidin-1-yl)ethanone used in Example 0478-3.
MSm/z(M+H):299.

<0781-6>

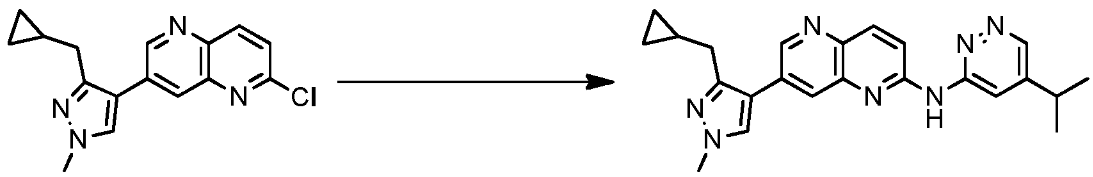

7-(3-(Cyclopropylmethyl)-1-methyl-1H-pyrazol-4-yl)-N-(5-isopropylpyridazin-3-yl)-1,5-naphthyridine-2-amine was obtained in the same manner as in Example 0555-4 except that 2-chloro-7-(3-(cyclopropylmethyl)-1-methyl-1H-pyrazol-4-yl)-1,5-naphthyridine was used instead of the 2-chloro-7-(1-methyl-3-(5-methylpyridin-3-yl)-1H-pyrazol-4-yl)-1,5-naphthyridine used in Example 0555-4.
$^1$H-NMR($CDCl_3$)δ:8.89(1H,brs),8.84(1H,d,J=2.1Hz),8.80(1H,d,J=2.1Hz),8.26(1H,d,J=9.0Hz),8.11(1H,d,J =2.1Hz),7.62(1H,s),7.53(1H,d,J=9.0Hz),3.97(3H,s),3.11-2.95(1H,m),2.81(2H,d,J=6.6Hz),1.40(6H,d,J=6.6Hz),1.17-1.02(1H,m),0.52-0.44(2H,m),0.23-0.16(2H,m).
MSm/z(M+H):400.

<Example 0782>

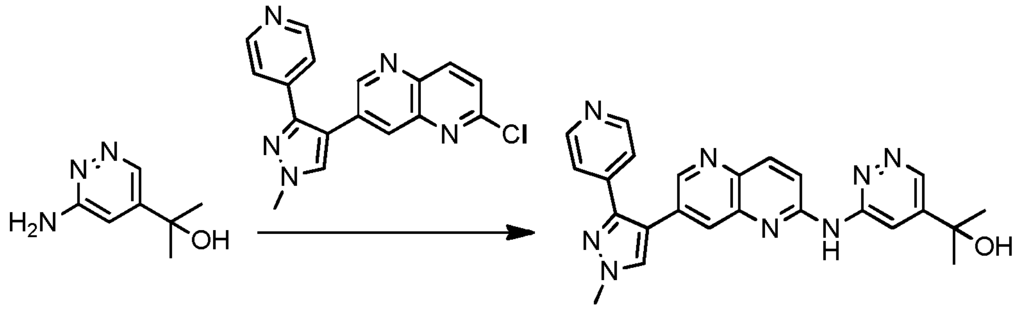

2-(6-((7-(1-Methyl-3-(pyridin-4-yl)-1H-pyrazol-4-yl)-1,5-naphthyridin-2-yl)amino)pyridazin-4-yl)propan-2-ol was obtained in the same manner as in Example 0543-2 except that 2-chloro-7-(1-methyl-3-(pyridin-4-yl)-1H-pyrazol-4-yl)-1,5-naphthyridine was used instead of the 2-chloro-7-(1-methyl-1H-pyrazol-4-yl)-1,5-naphthyridine used in Example 0543-2.

$^1$H-NMR($CDCl_3$/$CD_3OD$=4/1)δ:8.93(2H,brs),8.63(1H,d,J=1.8Hz),8.51(2H,dd,J=4.8,1.8Hz),8.23(1H,d,J=9.3Hz),8.00(1H,d,J=1.8Hz),7.83(1H,s),7.59(1H,d,J=9.3Hz),7.50(2H,dd,J=4.8,1.8Hz),4.05(3H,s),1.57( 6H,s).

MSm/z(M+H):439.

<Example 0783>

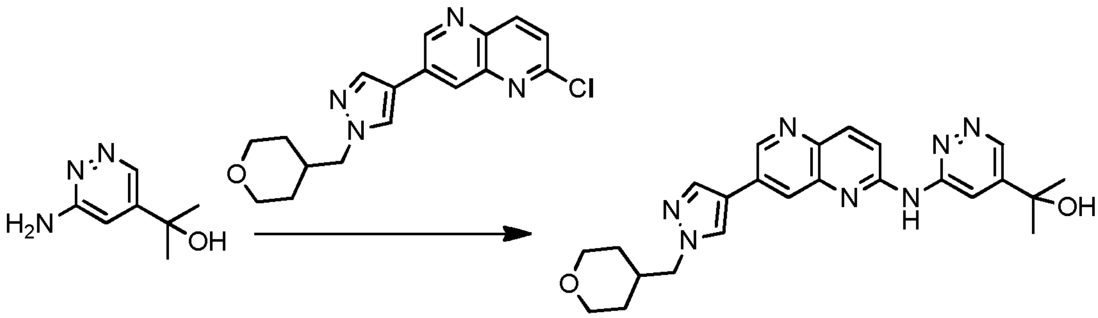

2-(6-((7-(1-((Tetrahydro-2H-pyran-4-yl)methyl)-1H-pyrazol-4-yl)-1,5-naphthyridin-2-yl)amino)pyridazin-4-yl)propan-2-ol was obtained in the same manner as in Example 0543-2 except that 2-chloro-7-(1-((tetrahydro-2H-pyran-4-yl)methyl)-1H-pyrazol-4-yl)-1,5-naphthyridine was used instead of the 2-chloro-7-(1-methyl-1H-pyrazol-4-yl)-1,5-naphthyridine used in Example 0543-2.

$^1$H-NMR($CDCl_3$/$CD_3OD$=4/1)δ:9.05(1H,brs),8.90(1H,brs),8.89(1H,d,J=2.1Hz),8.23(1H,d,J=2.1Hz),8.21( 1H,d,J=9.3Hz),8.00(1H,s),7.99(1H,s),7.55(1H,d,J=9.3Hz),4.12(2H,d,J=7.2Hz),4.01(2H,dd,J=11.1,3.6 Hz),3.43(2H,td,J=11.1,3.6Hz),2.32-2.15(1H,m),1.72-1.34(4H,m),1.66(6H,s).

MSm/z(M+H):446.

<Example 0784>

<0784-1>

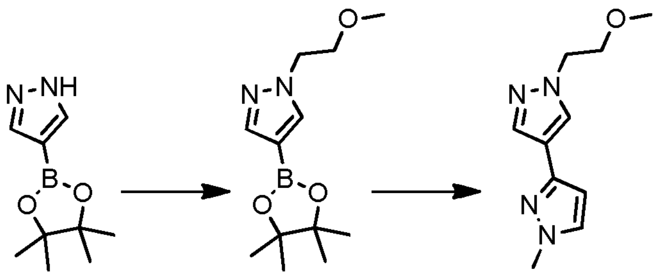

A mixture of 4-(4,4,5,5-tetramethyl-1,3,2-dioxaborolan-2-yl)-1H-pyrazole (300 mg), 1-bromo-2-methoxyethane (0.252 mL), cesium carbonate (954 mg), acetonitrile (4 mL), and 1,2-dimethoxyethane (2 mL) was stirred at 80°C for 4 hours. The reaction mixture was cooled to room temperature, and insolubles were filtered off. The solvent was distilled off under reduced pressure, thereby obtaining 1-(2-methoxyethyl)-4-(4,4,5,5-tetramethyl-1,3,2-dioxaborolan-2-yl)-1H-pyrazole (429 mg).

A mixture of the obtained 1-(2-methoxyethyl)-4-(4,4,5,5-tetramethyl-1,3,2-dioxaborolan-2-yl)-1H-pyrazole (429 mg), 3-bromo-1-methyl-1H-pyrazole (200 mg), sodium carbonate (323 mg), bis(di-tert-butyl (4-dimethylaminophenyl)phosphine)dichloropalladium(II) (43 mg), water (1 mL), and 1,2-dimethoxyethane (5 mL) was stirred at 80°C for 2 hours. The reaction mixture was cooled to room temperature, insolubles were filtered off, and the solvent was distilled off under reduced pressure. The obtained residue was purified by silica gel column chromatography (methanol-ethyl acetate), thereby obtaining 1'-(2-methoxyethyl)-1-methyl-1H,1'H-3,4'-bipyrazole (198 mg).
MSm/z(M+H):207.

<0784-2>

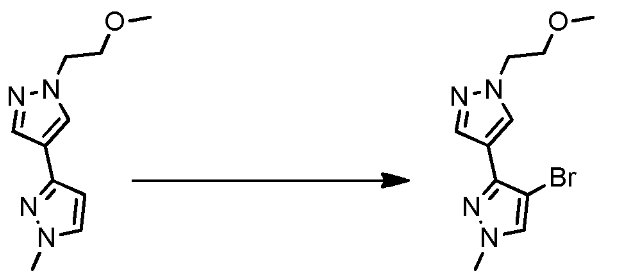

4-Bromo-1'-(2-methoxyethyl)-1-methyl-1H,1'H-3,4'-bipyrazole was obtained in the same manner as in Example 0555-2 except that 1'-(2-methoxyethyl)-1-methyl-1H,1'H-3,4'-bipyrazole was used instead of the 3-methyl-5-(1-methyl-1H-pyrazol-3-yl)pyridine used in Example 0555-2.
MSm/z(M+H):285.

<0784-3>

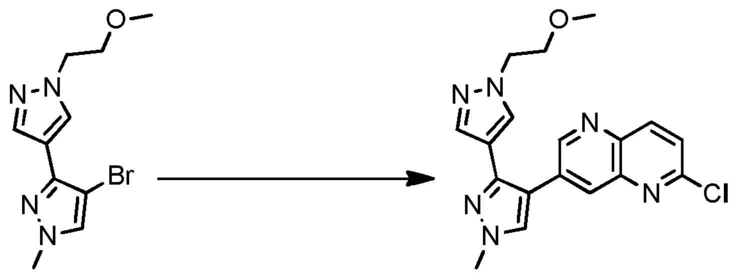

2-Chloro-7-(1'-(2-methoxyethyl)-1-methyl-1H,1'H-[3,4'-bipyrazole]-4-yl)-1,5-naphthyridine was obtained in the same manner as in Example 0478-3 except that 4-bromo-1'-(2-methoxyethyl)-1-methyl-1H,1'H-3,4'-bipyrazole was used

instead of the 1-(4-(4-iodo-1H-pyrazol-1-yl)piperidin-1-yl)ethanone used in Example 0478-3.
MSm/z(M+H):369.

<0784-4>

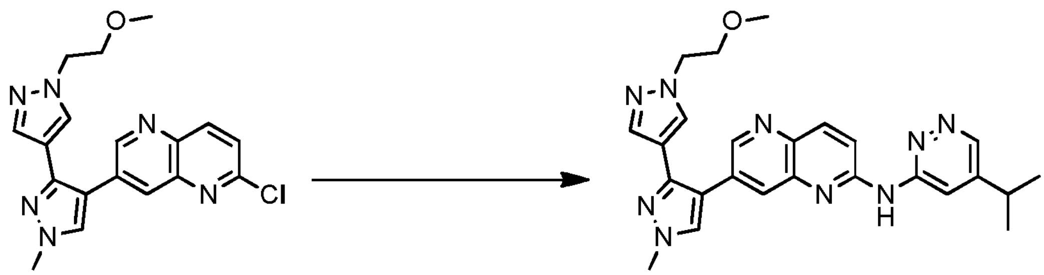

N-(5-isopropylpyridazin-3-yl)-7-(1'-(2-methoxyethyl)-1-methyl-1H,1'H-[3,4'-bipyrazole]-4-yl)-1,5-naphthyridine-2-amine was obtained in the same manner as in Example 0555-4 except that 2-chloro-7-(1'-(2-methoxyethyl)-1-methyl-1H,1'H-[3,4'-bipyrazole]-4-yl)-1,5-naphthyridine was used instead of the 2-chloro-7-(1-methyl-3-(5-methylpyridin-3-yl)-1H-pyrazol-4-yl)-1,5-naphthyridine used in Example 0555-4.
$^1$H-NMR($CDCl_3$)δ:8.83(1H,brs),8.78(1H,brs),8.76(1H,d,J=1.8Hz),8.26(1H,d,J=8.4Hz),8.06(1H,d,J=1.8Hz),7.64(1H,s),7.61(1H,d,J=8.4Hz),7.59(1H,s),7.57(1H,s),4.23(2H,t,J=5.4Hz),4.01(3H,s),3.69(2H,t,J=5.4Hz),3.24(3H,s),3.09-2.93(1H,m),1.37(6H,d,J=6.6Hz).
MSm/z(M+H):470.

<Example 0785>

<0785-1>

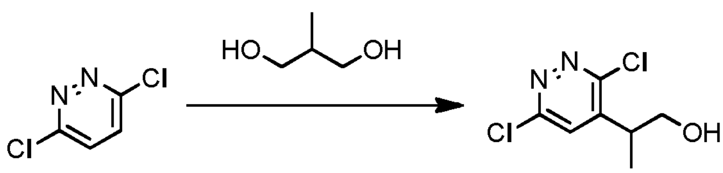

Potassium peroxodisulfate (6.72 g) was added to a mixture of 3,6-dichloropyridazine (2 g), trifluoroacetic acid (1.22 mL), 2-methylpropane-1,3-diol (2.65 g), silver nitrate (3.07 mg), and water (14 mL) at 80°C, followed by stirring at the same temperature for 30 minutes. After the reaction mixture was cooled on ice, sodium carbonate (10 g) and sodium chloride (1 g) were added thereto, followed by stirring at room temperature for 30 minutes. Insolubles were filtered off, and ethyl acetate was added thereto. The organic layer was collected by separation, washed with a saturated sodium chloride aqueous solution, and dried over anhydrous sodium sulfate, and the solvent was distilled off under reduced pressure. The obtained residue was purified by silica gel column chromatography (methanol-ethyl acetate), thereby obtaining 2-(3,6-dichloropyridazin-4-yl)propan-1-ol (1.93 g).
MSm/z(M+H):207.

<0785-2>

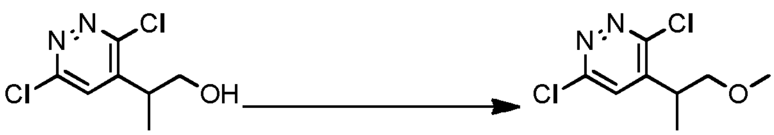

3,6-Dichloro-4-(1-methoxypropan-2-yl)pyridazine was obtained in the same manner as in Example 0562-1 except that 2-(3,6-dichloropyridazin-4-yl)propan-1-ol was used instead of the 2-(3,6-dichloropyridazin-4-yl)-2-methylpropan-1-ol used in Example 0562-1.
MSm/z(M+H):221.

<0785-3>

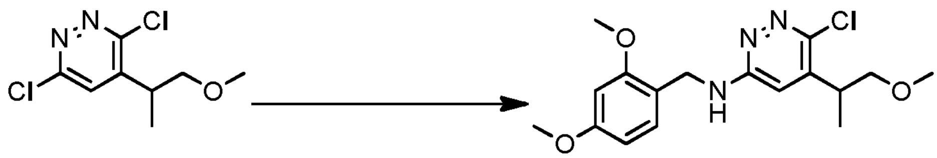

6-Chloro-N-(2,4-dimethoxybenzyl)-5-(1-methoxypropan-2-yl)pyridazine-3-amine was obtained in the same manner as in Example 0559-2 except that 3,6-dichloro-4-(1-methoxypropan-2-yl)pyridazine was used instead of the 4-(1-((tert-butyldimethylsilyl)oxy)-2-methylpropan-2-yl)-3,6-dichloropyridazine used in Example 0559-2.
MSm/z(M+H):352.

<0785-4>

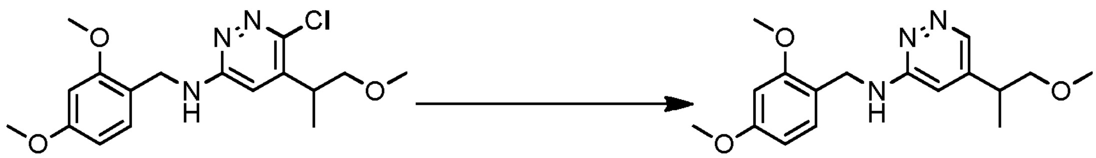

N-(2,4-dimethoxybenzyl)-5-(1-methoxypropan-2-yl)pyridazine-3-amine was obtained in the same manner as in Example 0559-3 except that 6-chloro-N-(2,4-dimethoxybenzyl)-5-(1-methoxypropan-2-yl)pyridazine-3-amine was used instead of the 5-(1-((tert-butyldimethylsilyl)oxy)-2-methylpropan-2-yl)-6-chloro-N-(2,4-dimethoxybenzyl)pyridazine-3-amine used in Example 0559-3.
MSm/z(M+H):318.

<0785-5>

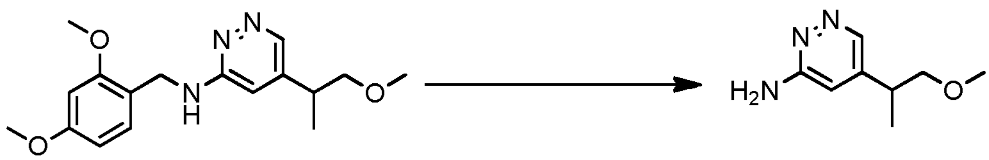

5-(1-Methoxypropan-2-yl)pyridazine-3-amine was obtained in the same manner as in Example 0559-4 except that N-(2,4-dimethoxybenzyl)-5-(1-methoxypropan-2-yl)pyridazine-3-amine was used instead of the 5-(1-((tert-butyldimethylsilyl)oxy)-2-methylpropan-2-yl)-N-(2,4-dimethoxybenzyl)pyridazine-3-amine used in Example 0559-4.
MSm/z(M+H):168.

<0785-6>

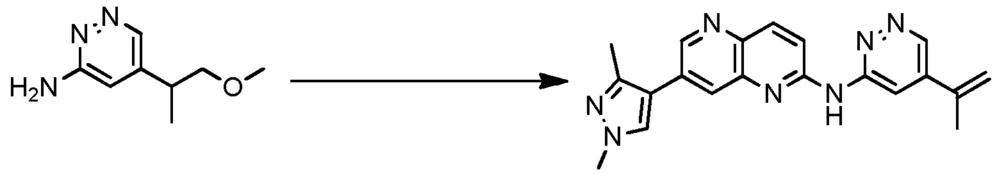

7-(1,3-Dimethyl-1H-pyrazol-4-yl)-N-(5-(prop-1-en-2-yl)pyridazin-3-yl)-1,5-naphthyridine-2-amine was obtained in the same manner as in Example 0769 except that 5-(1-methoxypropan-2-yl)pyridazine-3-amine was used instead of the 5-(pentan-3-yl)pyridazine-3-amine used in Example 0769.

$^1$H-NMR($CDCl_3$/$CD_3OD$=4/1)δ:9.09(1H,brs),8.93(1H,brs),8.78(1H,d,J=1.8Hz),8.23(1H,d,J=9.3Hz),8.09( 1H,brs),7.76(1H, s),7.52(1H,d,J=9.3Hz),5.89(1H,brs),5.51(1H,brs),3.95(3H,s),2.50(3H,s),2.28(3H,s).

MSm/z(M+H):358.

<Example 0786>

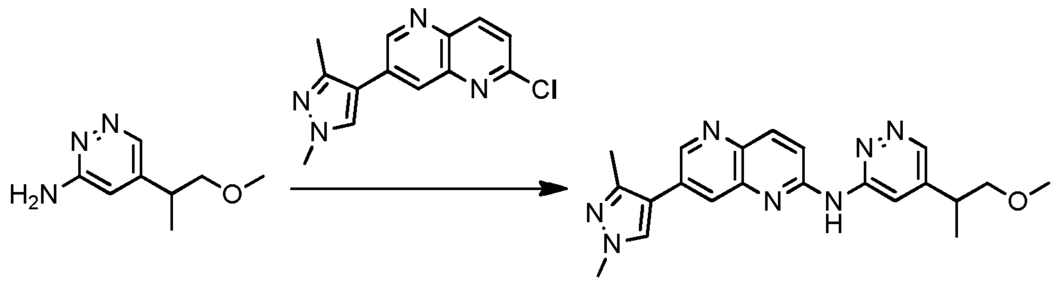

A suspension of 2-chloro-7-(1,3-dimethyl-1H-pyrazol-4-yl)-1,5-naphthyridine (10 mg), 5-(1-methoxypropan-2-yl)pyridazine-3-amine (3.5 mg), tris(dibenzylideneacetone)dipalladium(0) (5 mg), 4,5-bis(diphenylphosphino)-9,9-dimethylxanthene (10 mg), and cesium carbonate (40 mg) in 1,4-dioxane (1 mL) was stirred at 140°C for 30 minutes using a microwave reaction apparatus. The reaction mixture was cooled to room temperature, insolubles were filtered off, and the obtained solution was purified by silica gel column chromatography (methanol-ethyl acetate, NH silica), thereby obtaining 7-(1,3-dimethyl-1H-pyrazol-4-yl)-N-(5-(1-methoxypropan-2-yl)pyridazin-3-yl)-1,5-naphthyridine-2-amine (3.9 mg).

$^1$H-NMR($CDCl_3$)δ:9.83(1H,brs),8.98(1H,brs),8.84(2H,brs),8.27(1H,d,J=9.0Hz),8.06(1H,brs),7.79(1H,d,J =9.0Hz),7.64(1H,s),3.95(3H,s),3.61(2H,d,J=6.6Hz),3.37(3H,s),3.22-3.09(1H,m),2.51(3H,s),1.43(3H,d,J=7.2Hz).

MSm/z(M+H):390.

<Example 0787>

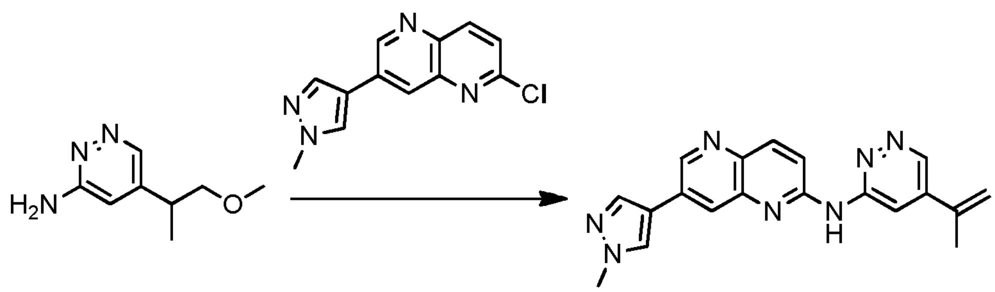

7-(1-Methyl-1H-pyrazol-4-yl)-N-(5-(prop-1-en-2-yl)pyridazin-3-yl)-1,5-naphthyridine-2-amine was obtained in the same manner as in Example 0554-3 except that 5-(1-methoxypropan-2-yl)pyridazine-3-amine was used instead of the 5-morpholinopyridazine-3-amine used in Example 0554-3.

$^1$H-NMR($CDCl_3$/$CD_3OD$=4/1):9.04(1H,brs),8.93(1H,brs),8.88(2H,brs),8.21(1H,d,J=9.3Hz),8.12(1H,brs), 7.95(1H,brs),7.50(1H,d,J=9.3Hz),5.83(1H,brs),5.52(1H,brs),4.03(3H,s),2.30(3H,s).
MSm/z(M+H):344.

<Example 0788>

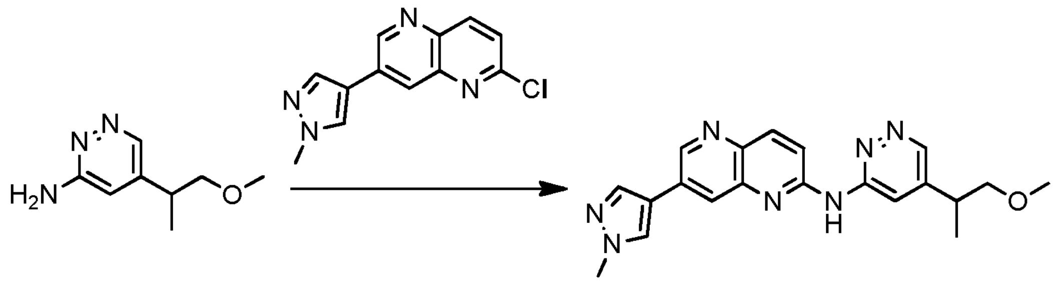

A suspension of 2-chloro-7-(1-methyl-1H-pyrazol-4-yl)-1,5-naphthyridine (10 mg), 5-(1-methoxypropan-2-yl)pyridazine-3-amine (3.5 mg), ((2-dicyclohexylphosphino-3,6-dimethoxy-2',4',6'-triisopropyl-1,1'-biphenyl)-2-(2'-amino-1,1'-biphenyl))palladium(II)methanesulfonate (BRETTPHOS-PD-G3, (product name, manufactured by Sigma-Aldrich Co. LLC.)) (5 mg), and cesium carbonate (14 mg) in 1,4-dioxane (1 mL) was stirred at 90°C for 2 hours. The reaction mixture was cooled to room temperature, and purified by silica gel column chromatography (methanol-ethyl acetate, NH silica), thereby obtaining N-(5-(1-methoxypropan-2-yl)pyridazin-3-yl)-7-(1-methyl-1H-pyrazol-4-yl)-1,5-naphthyridine-2-amine (2.1 mg).
$^1$H-NMR($CDCl_3$)δ:9.44(1H,brs),8.97(1H,brs),8.92(1H,d,J=2.1Hz),8.84(1H,brs),8.25(1H,d,J=9.3Hz),8.10( 1H,brs),7.95(1H, s),7.84(1H,s),7.67(1H,d,J=9.3Hz),4.03(3H,s),3.62(2H,d,J=6.6Hz),3.39(3H,s),3.25-3.11(1H,m),2.17(3H,s),1.44(3H,d,J =7.2Hz).
MSm/z(M+H):376.

<Example 0789>

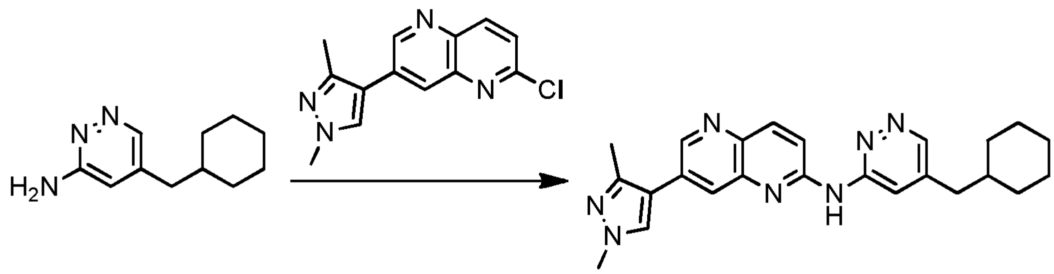

N-(5-(cyclohexylmethyl)pyridazin-3-yl)-7-(1,3-dimethyl-1H-pyrazol-4-yl)-1,5-naphthyridine-2-amine was obtained in the same manner as in Example 0769 except that 5-(cyclohexylmethyl)pyridazine-3-amine was used instead of the 5-(pentan-3-yl)pyridazine-3-amine used in Example 0769.
$^1$H-NMR($CDCl_3$/$CD_3OD$=4/1)δ:8.78(1H,d,J=2.1Hz),8.76(1H,brs),8.62(1H,brs),8.21(1H,d,J=9.3Hz),8.10( 1H,d,J=2.1Hz),7.74(1H,s),7.51(1H,d,J=9.3Hz),3.95(3H,s),2.61(2H,d,J=7.2Hz),2.50(3H,s),1.85-1.61(8H,m),1.35-1.00(3H,m).
MSm/z(M+H):414.

<Example 0790>

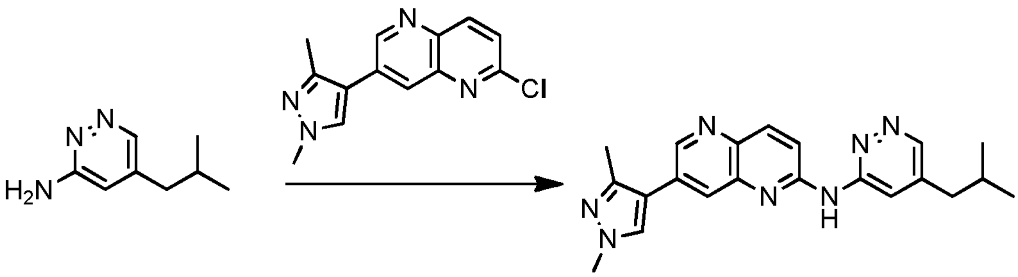

7-(1,3-Dimethyl-1H-pyrazol-4-yl)-N-(5-isobutylpyridazin-3-yl)-1,5-naphthyridine-2-amine was obtained in the same manner as in Example 0769 except that 5-isobutylpyridazine-3-amine was used instead of the 5-(pentan-3-yl)pyridazine-3-amine used in Example 0769.
$^1$H-NMR($CDCl_3$)δ:9.37(1H,brs),8.84(1H,d,J=1.8Hz),8.77(1H,brs),8.72(1H,brs),8.27(1H,d,J=9.3Hz),8.05( 1H,brs),7.66(1H,d,J=9.3Hz),7.65(1H,s),3.95(3H,s),2.60(2H,d,J=7.2Hz),2.50(3H,s),2.15-2.00(1H,m),1.04(6H,d,J=6.9Hz).
MSm/z(M+H):374.

<Example 0791>

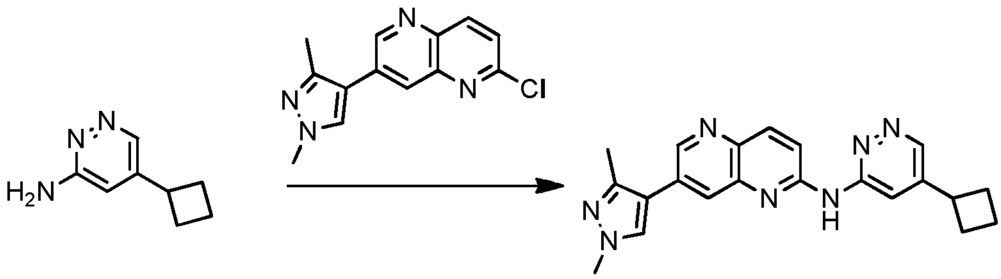

N-(5-cyclobutylpyridazin-3-yl)-7-(1,3-dimethyl-1H-pyrazol-4-yl)-1,5-naphthyridine-2-amine was obtained in the same manner as in Example 0769 except that 5-cyclobutylpyridazine-3-amine was used instead of the 5-(pentan-3-yl)pyridazine-3-amine used in Example 0769.
$^1$H-NMR($CDCl_3$/$CD_3OD$=4/1)δ:8.90(1H,brs),8.78(1H,d,J=1.8Hz),8.65(1H,brs),8.22(1H,d,J=9.3.Hz),8.10( 1H,d,J=1.8Hz),7.75(1H,s),7.51(1H,d,J=9.3Hz),3.94(3H,s),3.75-3.56(1H,m),2.59-2.47(2H,m),2.50(3H,s),2.38-2.11(4H,m).
MSm/z(M+H):372.

<Example 0792>

<0792-1>

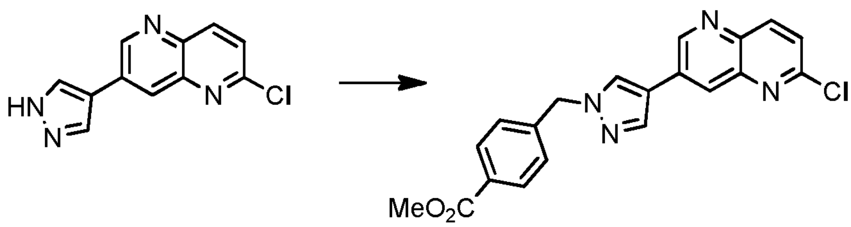

Methyl 4-((4-(6-chloro-1,5-naphthyridin-3-yl)-1H-pyrazol-1-yl)methyl)benzoate was obtained as a pale brown solid in the same manner as in Example 0440-1 except that methyl 4-(bromomethyl)benzoate was used instead of the bromoethane used in Example 0440-1.

MSm/z(M+H):379.

<0792-2>

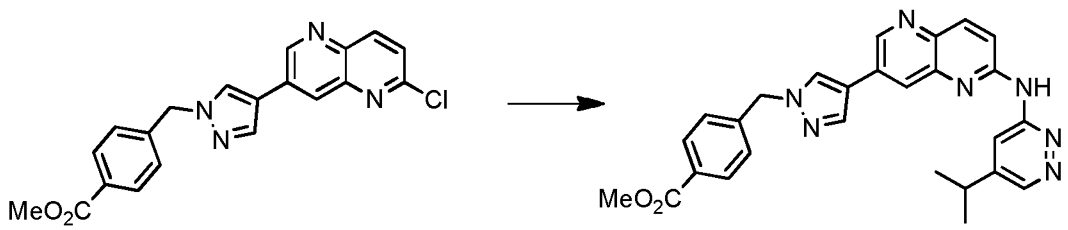

Methyl 4-((4-(6-((5-isopropylpyridazin-3-yl)amino)-1,5-naphthyridin-3-yl)-1H-pyrazol-1-yl)methyl)benzoate was obtained as a pale yellow solid in the same manner as in Example 0411-3 except that methyl 4-((4-(6-chloro-1,5-naphthyridin-3-yl)-1H-pyrazol-1-yl)methyl)benzoate was used instead of the 2-chloro-7-(1-((6-methylpyridin-2-yl)methyl)-1H-pyrazol-4-yl)-1,5-naphthyridine used in Example 0411-3.
$^1$H-NMR(DMSO-$d_6$)$\delta$:10.70(1H,s),9.06(1H,d,J=2.0Hz),8.86(1H,d,J=2.0Hz),8.73(1H,d,J=2.0Hz),8.67(1H,s),8.26-8.22(3H,m),7.97(2H,d, J=4.3Hz),7.71(1H,d,J=9.2Hz),7.41(2H,d,J=8.6Hz),5.53(2H,s),3.84(3H,s),3.04-3.02(1H,m),1.33(6H,d,J=6.6Hz).
MSm/z(M+H):480.

<Example 0793>

<0793-1>

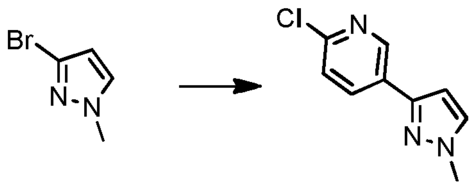

2-Chloro-5-(1-methyl-1H-pyrazol-3-yl)pyridine was obtained as a yellow solid in the same manner as in Example 0451-1 except that 2-chloro-5-(4,4,5,5-tetramethyl-1,3,2-dioxaborolan-2-yl)pyridine was used instead of the 3-methoxy-5-(4,4,5,5-tetramethyl-1,3,2-dioxaborolan-2-yl)pyridine used in Example 0451-1.
MSm/z(M+H):194.

<0793-2>

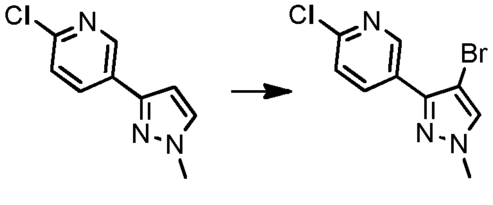

5-(4-bromo-1-methyl-1H-pyrazol-3-yl)-2-chloropyridine was obtained as brown oily substance in the same manner as in Example 0451-2 except that 2-chloro-5-(1-methyl-1H-pyrazol-3-yl)pyridine was used instead of the 3-methoxy-5-(1-methyl-1H-pyrazol-3-yl)pyridine used in Example 0451-2.
MSm/z(M+H):274.

<0793-3>

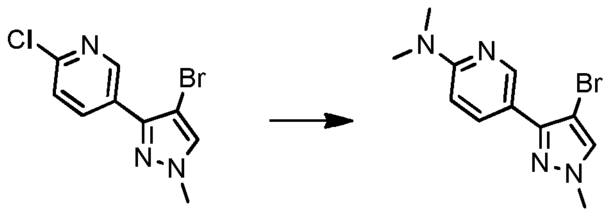

A mixture of 5-(4-bromo-1-methyl-1H-pyrazol-3-yl)-2-chloropyridine (288 mg), 1,4-dioxane (2 mL), and a 50%dimethylamine aqueous solution (1.5 mL) was stirred at 160°C for 1 hour using a microwave reaction apparatus. The reaction mixture was cooled to room temperature, and the solvent was distilled off under reduced pressure. The obtained residue was purified by silica gel column chromatography (hexane-ethyl acetate-methanol, NH silica), thereby obtaining 5-(4-bromo-1-methyl-1H-pyrazol-3-yl)-N,N-dimethylpyridine-2-amine (81 mg) as colorless oily substance.
MSm/z(M+H):281.

<0793-4>

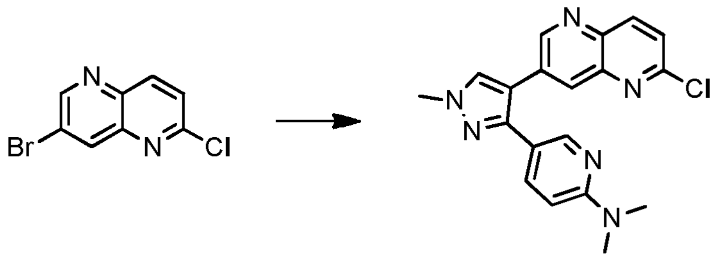

5-(4-(6-Chloro-1,5-naphthyridin-3-yl)-1-methyl-1H-pyrazol-3-yl)-N,N-dimethylpyridine-2-amine was obtained as a pale yellow solid in the same manner as in Example 0451-3 except that 5-(4-bromo-1-methyl-1H-pyrazol-3-yl)-N,N-dimethylpyridine-2-amine was used instead of the 3-(4-bromo-1-methyl)-1H-pyrazol-3-yl)-5-methoxypyridine used in Example 0451-3.
MSm/z(M+H):365.

<0793-5>

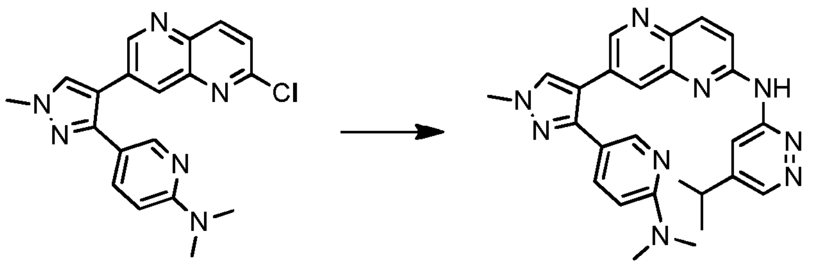

7-(3-(6-(Dimethylamino)pyridin-3-yl)-1-methyl-1H-pyrazol-4-yl)-N-(5-isopropylpyridazin-3-yl)-1,5-naphthyridine-2-amine was obtained as a pale yellow solid in the same manner as in Example 0451-4 except that 5-(4-(6-chloro-1,5-naphthyridin-3-yl)-1-methyl-1H-pyrazol-3-yl)-N,N-dimethylpyridine-2-amine was used instead of the 2-chloro-7-(3-(5-methoxypyridin-3-yl)-1-methyl-1H-pyrazol-4-yl)-1,5-naphthyridine used in Example 0451-4.
$^1$H-

NMR($CDCl_3$)δ:8.81-8.78(2H,m),8.70(1H,d,J=2.0Hz),8.27(1H,d,J=2.6Hz),8.22(1H,d,J=9.2Hz),8.00(1H,d,J=1.3Hz),7.65 -7.60(2H,m),7.51(1H,brs),6.50(1H,d,J=8.6Hz),4.03(3H,s),3.08(6H,s),3.03-2.93(1H,m),1.33(6H,d,J=6.6Hz).
MSm/z(M+H):466.

<Example 0794>

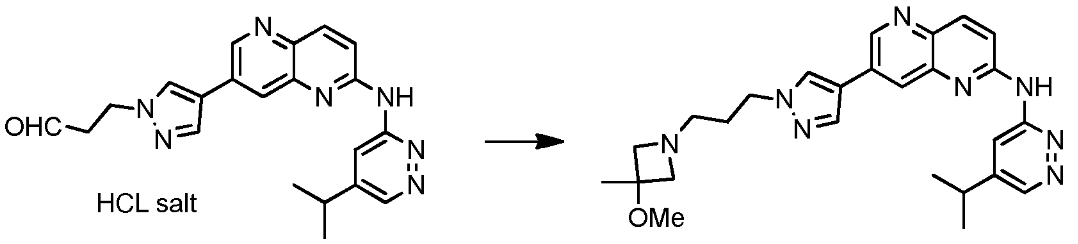


N-(5-isopropylpyridazin-3-yl)-7-(1-(3-(3-methoxy-3-methylazetidin-1-yl)propyl)-1H-pyrazol-4-yl)-1,5-naphthyridine-2-amine was obtained as a white solid in the same manner as in Example 0426-2 except that 3-methoxy-3-methylazetidine hydrochloride was used instead of the (2S,6R)-1,2,6-trimethylpiperazine trifluoroacetate used in Example 0426-2.
$^1$H-NMR($CDCl_3$)δ:8.93(1H,d,J=2.0Hz),8.82(2H,m),8.69(1H,brs),8.24(1H,d,J=9.2Hz),8.10(1H,d,J=2.0Hz) ,7.95(1H,s),7.86 (1H,s),7.50(1H,d,J=8.6Hz),4.28(2H,t,J=6.9Hz),3.23-3.19(5H,m),3.08-3.00(3H,m),2.52(2H,t,J=6.9Hz),1.99(2H,t,J=6.9 Hz),1.47(3H,s),1.42(6H,d,J=6.6Hz).
MSm/z(M+H):473.

<Example 0795>

<0795-1>

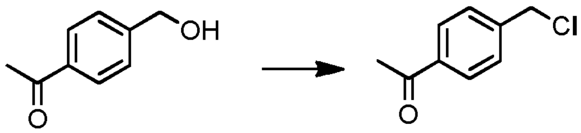

1-(4-(Chloromethyl)phenyl)ethanone was obtained as brown oily substance in the same manner as in Example 0464-1 except that 1-(4-(hydroxymethyl)phenyl)ethanone was used instead of the tert-butyl (5-(hydroxymethyl)pyridin-2-yl)carbamate used in Example 0464-1.
MSm/z(M+H):169.

<0795-2>

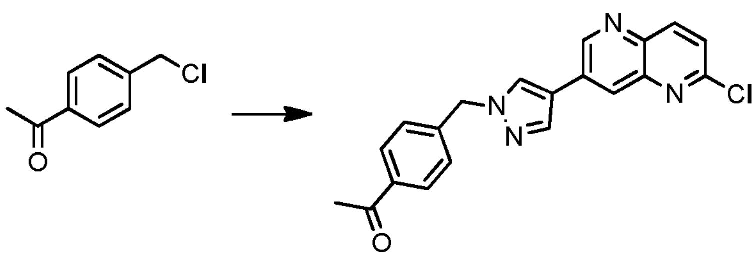

1-(4-((4-(6-Chloro-1,5-naphthyridin-3-yl)-1H-pyrazol-1-yl)methyl)phenyl)ethanone was obtained as a pale

brown solid in the same manner as in Example 0440-1 except that 1-(4-(chloromethyl)phenyl)ethanone was used instead of the bromoethane used in Example 0440-1.
MSm/z(M+H):363.

<0795-3>

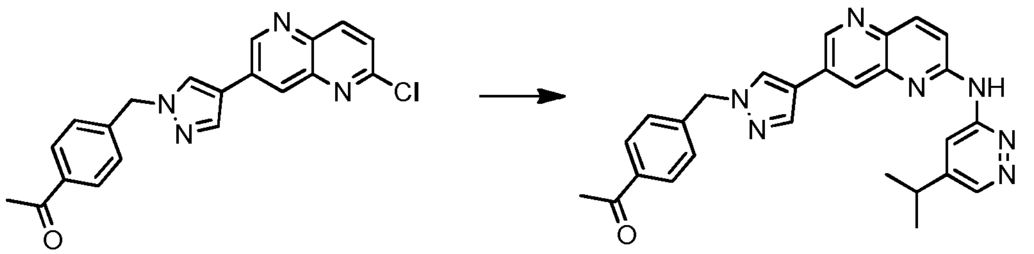

1-(4-((4-(6-((5-Isopropylpyridazin-3-yl)amino)-1,5-naphthyridin-3-yl)-1H-pyrazol-1-yl)methyl)phenyl)ethanone was obtained as a pale yellow solid in the same manner as in Example 0411-3 except that 1-(4-((4-(6-chloro-1,5-naphthyridin-3-yl)-1H-pyrazol-1-yl)methyl)phenyl)ethanone was used instead of the 2-chloro-7-(1-((6-methylpyridin-2-yl)methyl)-1H-pyrazol-4-yl)-1,5-naphthyridine used in Example 0411-3.
$^1$H-NMR($CDCl_3$)δ:8.92(1H,s),8.81(1H,s),8.76(1H,s),8.24(1H,d,J=9.2Hz),8.10(1H,s),8.04(1H,s),7.98(3H, d,J=7.9Hz),7.89(1H,s),7.38(2H,d,J=7.9Hz),5.48(2H,s),3.06-3.03(1H,m),2.60(3H,s),1.41(6H,d,J=6.6Hz).
MSm/z(M+H):464.

<Example 0796>

<0796-1>

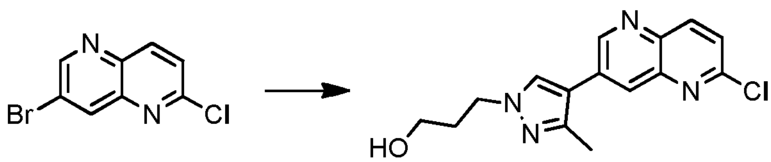

3-(4-(6-Chloro-1,5-naphthyridin-3-yl)-3-methyl-1H-pyrazol-1-yl)propan-1-ol was obtained as a brown solid in the same manner as in Example 0451-3 except that 3-(4-iodo-3-methyl-1H-pyrazol-1-yl)propan-1-ol was used instead of the 3-(4-bromo-1-methyl-1H-pyrazol-3-yl)-5-methoxypyridine used in Example 0451-3.
MSm/z(M+H):303.

<0796-2>

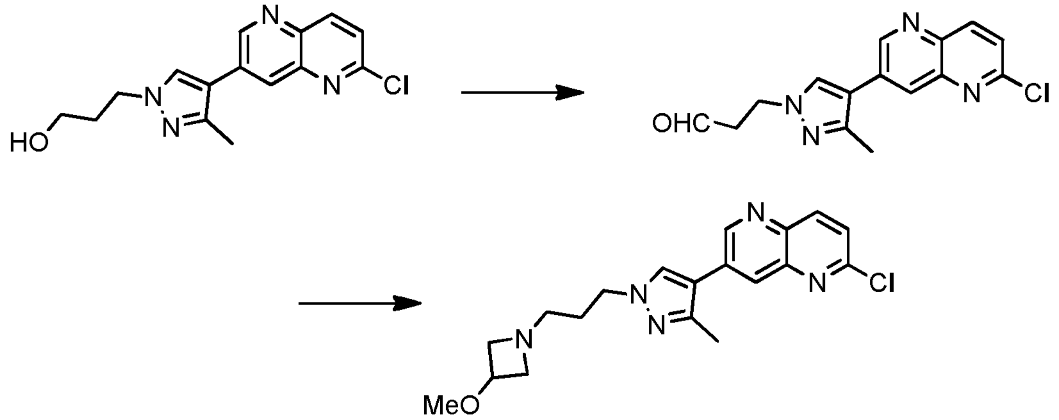

A solution of 3-(4-(6-chloro-1,5-naphthyridin-3-yl)-3-methyl-1H-pyrazol-1-yl)propan-1-ol (36 mg) in dichloromethane (1.5 mL) was added to a mixture of Dess-Martin periodinane (59 mg) and dichloromethane (1 mL) at room temperature, followed by stirring at room temperature for 7.5 hours. Ethyl acetate, a saturated sodium carbonate aqueous solution, and a saturated sodium hydrogen carbonate aqueous solution were added to the reaction mixture, followed by stirring at room temperature for 30 minutes. After water was added to the reaction mixture, the organic layer was collected by separation, washed with a saturated sodium chloride aqueous solution, and dried over anhydrous sodium sulfate, and the solvent was distilled off under reduced pressure, thereby obtaining a pale brown solid (33 mg).

A mixture of the obtained pale brown solid (16 mg), 3-methoxyazetidine hydrochloride (13 mg), triethylamine (23 μL), and dichloromethane (0.5 mL) was stirred at room temperature for 30 minutes in a nitrogen atmosphere. Acetic acid (0.1 mL) was added to the reaction mixture, followed by stirring at room temperature for 1 hour. Sodium triacetoxyborohydride (55 mg) was added to the reaction mixture, followed by stirring at room temperature for 1 hour. The solvent was distilled off under reduced pressure, a saturated sodium hydrogen carbonate aqueous solution was added to the obtained residue, and the solvent was distilled off under reduced pressure. The obtained residue was purified by silica gel column chromatography (hexane-ethyl acetate-methanol, NH silica), thereby obtaining 2-chloro-7-(1-(3-(3-methoxyazetidin-1-yl)propyl)-3-methyl-1H-pyrazol-4-yl)-1,5-naphthyridine (7.2 mg) as colorless oily substance.
MSm/z(M+H):372.

<0796-3>

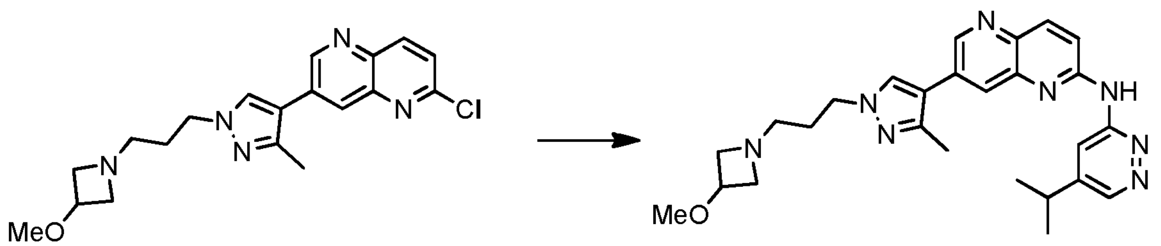

N-(5-isopropylpyridazin-3-yl)-7-(1-(3-(3-methoxyazetidin-1-yl)propyl)-3-methyl-1H-pyrazol-4-yl)-1,5-naphthyridine-2-amine was obtained as a pale brown solid in the same manner as in Example 0411-3 except that 2-chloro-7-(1-(3-(3-methoxyazetidin-1-yl)propyl)-3-methyl-1H-pyrazol-4-yl)-1,5-naphthyridine was used instead of the 2-chloro-7-(1-((6-methylpyridin-2-yl)methyl)-1H-pyrazol-4-yl)-1,5-naphthyridine used in Example 0411-1.
$^1$H-NMR($CDCl_3$)δ:9.10(1H,brs),8.89(1H,s),8.84-8.83(2H,m),8.26(1H,d,J=9.2Hz),8.06(1H,s),7.68(1H,s),7.61(1H,d,J=8.6Hz),4.19(2H,t,J=6.9Hz),4.06-4.04(1H,m),3.64-3.61(2H,m),3.27(3H,s),3.05-3.03(1H,m),2.90(2H,t,J=6.9Hz),2.55-2.51(5H,m),1.99-1.96(2H,m), 1.41 (6H,d,J=6.6Hz).
MSm/z(M+H):473.

<Example 0797>

<0797-1>

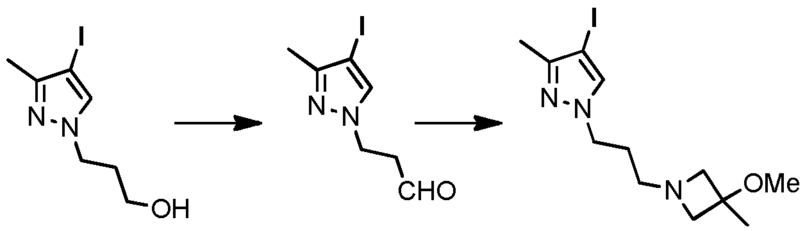

4-Iodo-1-(3-(3-methoxy-3-methylazetidin-1-yl)propyl)-3 -methyl-1H-pyrazole was obtained in the same manner as in Example 0796-2 except that 3-(4-iodo-3-methyl-1H-pyrazol-1-yl)propan-1-ol was used instead of the 3-(4-(6-chloro-1,5-naphthyridin-3-yl)-3-methyl-1H-pyrazol-1-yl)propan-1-ol used in Example 0796-2, and 3-methoxy-3-methylazetidine hydrochloride was used instead of the 3-methoxyazetidine hydrochloride used in Example 0796-2.
MSm/z(M+H):350.

<0797-2>

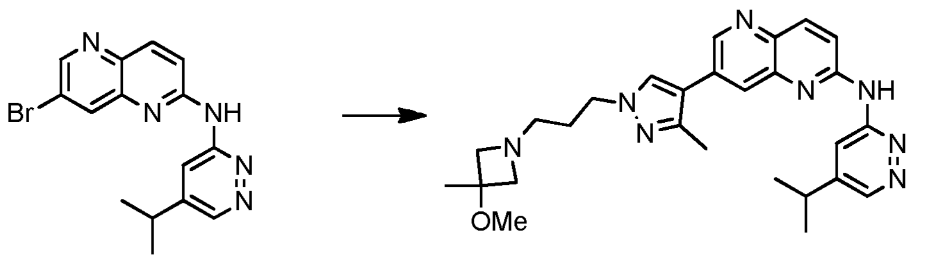

N-(5-isopropylpyridazin-3-yl)-7-(1-(3-(3-methoxy-3-methylazetidin-1-yl)propyl)-3-methyl-1H-pyrazol-4-yl)-1,5-naphthyridine-2-amine was obtained as a yellow solid in the same manner as in Example 0421-1 except that 4-iodo-1-(3-(3-methoxy-3-methylazetidin-1-yl)propyl)-3-methyl-1H-pyrazole was used instead of the N-(4-bromopyridin-2-yl)acetamide used in Example 0421-1, and 7-bromo-N-(5-isopropylpyridazin-3-yl)-1,5-naphthyridine-2-amine was used instead of the 7-bromo-2-chloro-1,5-naphthyridine used in Example 0421-1.
$^1$H-NMR($CDCl_3$)δ:8.85(1H,d,J=2.0Hz),8.80-8.77(2H,m),8.32(1H,s),8.26(1H,d,J=9.2Hz),8.07(1H,s),7.71(1H,s),7.47(1H,d, J=9.2Hz),4.21(2H,t,J=6. 6Hz),3.59-3.57(2H,m),3.26-3.23(2H,m),3.20(3H,s),3.04-3.01(1H,m),2.76(2H,t,J=7.3Hz),2.51(3H,s),2.08(2H,t,J=6.9Hz ),1.50(3H,s),1.40(6H,d,J=7.3Hz).
MSm/z(M+H):487.

<Example 0798>

<0798-1>

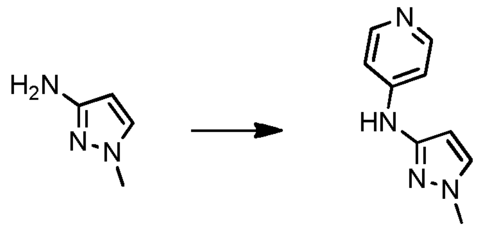

N-(1-methyl-1H-pyrazol-3-yl)pyridine-4-amine was obtained as a a yellow solid in the same manner as in Example 0469-1 except that 4-bromopyridine was used instead of the 3-bromopyridine used in Example 0469-1.
MSm/z(M+H):175.

<0798-2>

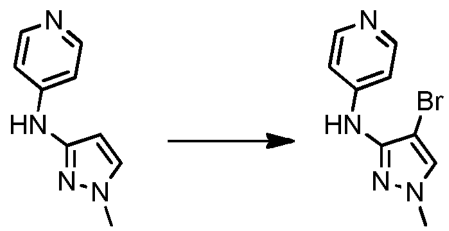

N-(4-bromo-1-methyl-1H-pyrazol-3-yl)pyridine-4-amine was obtained as pale yellow oily substance in the same manner as in Example 0451-2 except that N-(1-methyl-1H-pyrazol-3-yl)pyridine-4-amine was used instead of the 3-methoxy-5-(1-methyl-1H-pyrazol-3-yl)pyridine used in Example 0451-2.
MSm/z(M+H):253.

<0798-3>

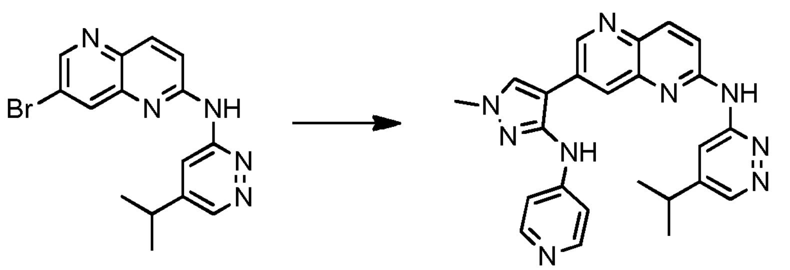

N-(5-isopropylpyridazin-3-yl)-7-(1-methyl-3-(pyridin-4-ylamino)-1H-pyrazol-4-yl)-1,5-naphthyridine-2-amine was obtained as a yellow solid in the same manner as in Example 0797-2 except that N-(4-bromo-1-methyl-1H-pyrazol-3-yl)pyridine-4-amine was used instead of the 4-iodo-1-(3-(3-methoxy-3-methylazetidin-1-yl)propyl)-3-methyl-1H-pyrazole used in Example 0797-2. $^1$H-NMR($CDCl_3$)δ:8.86(1H,d,J=2.0Hz),8.79(1H,d,J=2.0Hz),8.68(1H,s),8.31-8.30(3H,m),8.23(1H,d,J=8.6Hz),8.09(1H,s),7.72(1H,s),7.41(1H,d,J=9.2Hz),6.99-6.99(1H,m),1.35(6H,d,J=7.3Hz).
MSm/z(M+H):438.

<Example 0799>

<0799-1>

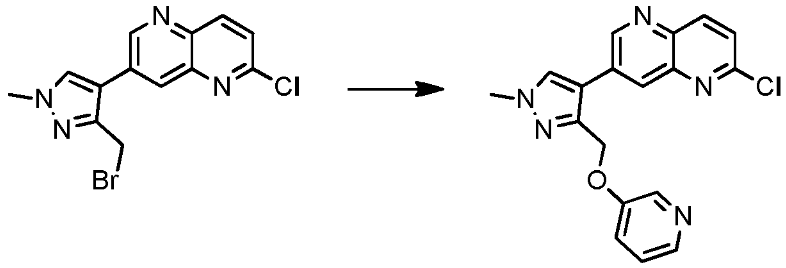

A mixture of 7-(3-(bromomethyl)-1-methyl-1H-pyrazol-4-yl)-2-chloro-1,5-naphthyridine (30 mg), pyridin-3-ol (9.8 mg), potassium carbonate (39 mg), and acetonitrile (1 mL) was stirred at room temperature for 3 hours, and stirred at 50°C for 1 hour. The reaction mixture was cooled to room temperature, and purified by silica gel column chromatography (hexane-ethyl acetate-methanol, NH silica), thereby obtaining 2-chloro-7-(1-methyl-3-((pyridin-3-yloxy)methyl)-1H-pyrazol-4-yl)-1,5-naphthyridine (4.6 mg) as a white solid.
MSm/z(M+H):352.

<0799-2>

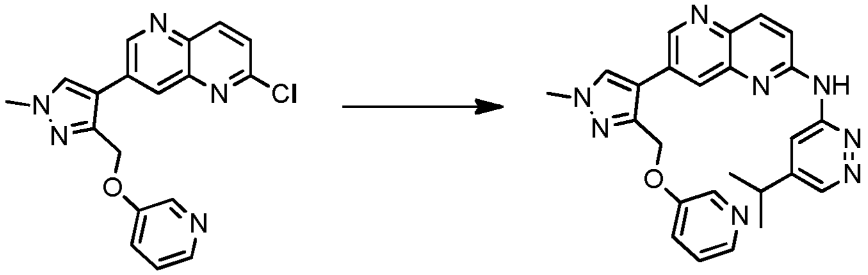

A mixture of 2-chloro-7-(1-methyl-3-((pyridin-3-yloxy)methyl)-1H-pyrazol-4-yl)-1,5-naphthyridine (4.6 mg), 5-isopropylpyridazine-3-amine (2.1 mg), sodium tert-amyl oxide (4.3 mg), anhydrous sodium sulfate (50 mg), and 1,4-dioxane (2 mL) was stirred for 6.5 hours under reflux. The reaction mixture was cooled to room temperature, and purified by silica gel column chromatography (methanol-ethyl acetate, NH silica), thereby obtaining N-(5-isopropylpyridazin-3-yl)-7-(1-methyl-3-((pyridin-3-yloxy)methyl)-1H-pyrazol-4-yl)-1,5-naphthyridine-2-amine (2.4 mg) as a white solid.
$^1$H-NMR($CDCl_3$)δ:8.90(1H,s),8.78(2H,s),8.62(1H,brs),8.48(1H,d,J=3.3Hz),8.26-8.25(3H,m),7.75(1H,s),7.52(1H,d,J=9.2Hz),7.44-7.40(1H,m),7.22-7.21(1H,m),5.24(2H,s),4.03(3H,s),2.96-2.87(1H,m),1.32(6H,d,J=6.6Hz).
MSm/z(M+H):453.

<Example 0800>

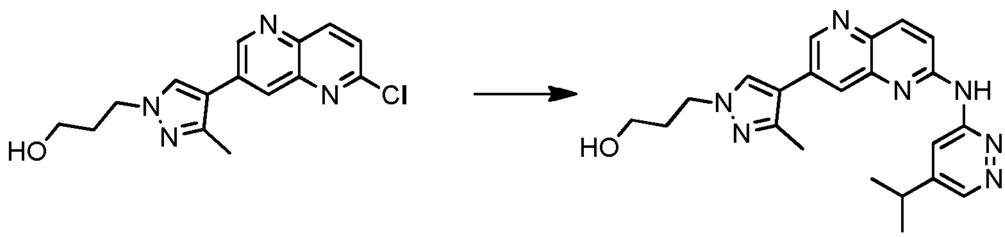

3-(4-(6-((5-Isopropylpyridazin-3-yl)amino)-1,5-naphthyridin-3-yl)-3-methyl-1H-pyrazol-1-yl)propan-1 -ol was obtained as a pale yellow solid in the same manner as in Example 0411-3 except that 3-(4-(6-chloro-1,5-naphthyridin-3-yl)-3-methyl-1H-pyrazol-1-yl)propan-1-ol was used instead of the 2-chloro-7-(1-((6-methylpyridin-2-yl)methyl)-1H-pyrazol-4-yl)-1,5-naphthyridine used in Example 0411-3.

$^1$H-NMR($CDCl_3$)δ:8.85(1H,d,J=2.0Hz),8.81(2H,s),8.49-8.45(1H,m),8.26(1H,d,J=8.6Hz),8.07(1H,s),7.70(1H,s),7.49(1H,d, J=8.6Hz),4.33(2H,t,J=6.3Hz),3.73(2 H,t,J=5.6Hz),3.04-3.02(1H,m),2.51(3H,s),2.17-2.09(2H,m),1.40(6H,d,J=6.6Hz). MSm/z(M+H):404.

<Example 0801>

<0801-1>

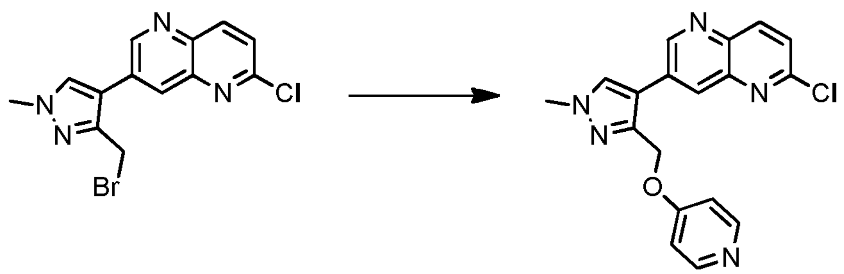

2-Chloro-7-(1-methyl-3-((pyridin-4-yloxy)methyl)-1H-pyrazol-4-yl)-1,5-naphthyridine was obtained in the same manner as in Example 0799-1 except that pyridin-4-ol was used instead of the pyridin-3-ol used in Example 0799-1. MSm/z(M+H):352.

<0801-2>

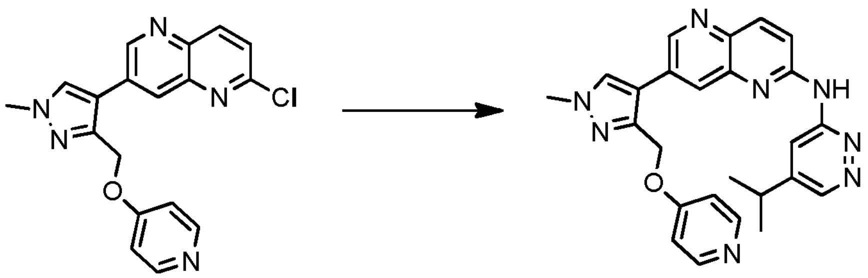

N-(5-isopropylpyridazin-3-yl)-7-(1-methyl-3-((pyridin-4-yloxy)methyl)-1H-pyrazol-4-yl)-1,5-naphthyridine-2-amine was obtained as a pale yellow solid in the same manner as in Example 0799-2 except that 2-chloro-7-(1-methyl-3-((pyridin-4-yloxy)methyl)-1H-pyrazol-4-yl)-1,5-naphthyridine was used instead of the 2-chloro-7-(1-methyl-3-((pyridin-3-yloxy)methyl)-1H-pyrazol-4-yl)-1,5-naphthyridine used in Example 0799-2.
$^1$H-NMR($CDCl_3$)8:9.75(1H,brs),8.82(2H,d,J=8.6Hz),8.68(1H,d,J=2.0Hz),8.29(1H,d,J=9.2Hz),7.88-7.85(2H,m),7.66(1H,s), 7.33(2H,d,J=7.9Hz),6.31(2H,d,J=7.3Hz),5.05(2H,s),4.03(3H,s),3.09-2.99(1H,m), 1.40(6H,d,J=6.6Hz). MSm/z(M+H):453.

<Example 0802>

<0802-1>

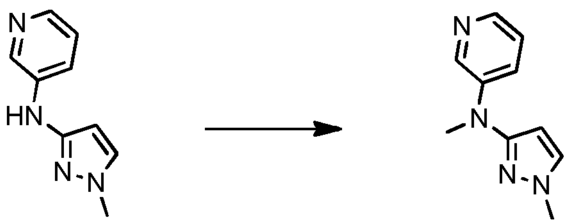

A solution of N-(1-methyl-1H-pyrazol-3-yl)pyridine-3-amine in N,N-dimethylformamide (1.9 mL) was cooled to a temperature of from 0°C to 5°C, and 60% sodium hydride (30 mg) was added thereto in a nitrogen atmosphere, followed by stirring for 30 minutes. Iodomethane (43 $\mu$L) was added to the reaction mixture, followed by stirring at room temperature for 3 hours. After water and ethyl acetate were added to the reaction mixture, the organic layer was collected by separation, washed with a saturated sodium chloride aqueous solution, and dried over anhydrous sodium sulfate, and the solvent was distilled off under reduced pressure, thereby obtaining N-methyl-N-(1-methyl-1H-pyrazol-3-yl)pyridine-3-amine (88 mg) as yellow oily substance.
MSm/z(M+H):189.

<0802-2>

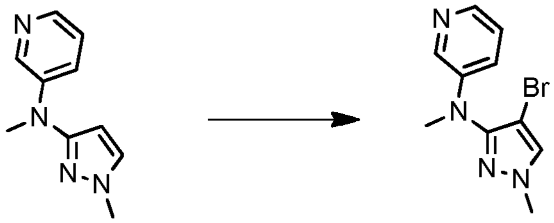

N-(4-bromo-1-methyl-1H-pyrazol-3-yl)-N-methylpyridine-3-amine was obtained as colorless oily substance in the same manner as in Example 0451-2 except that N-methyl-N-(1-methyl-1H-pyrazol-3-yl)pyridine-3-amine was used instead of the 3-methoxy-5-(1-methyl-1H-pyrazol-3-yl)pyridine used in Example 0451-2.
MSm/z(M+H):267.

<0802-3>

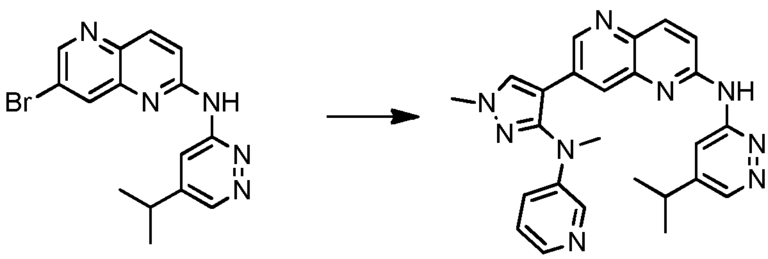

N-(5-isopropylpyridazin-3-yl)-7-(1-methyl-3-(methyl (pyridin-3-yl)amino)-1H-pyrazol-4-yl)-1,5-naphthyridine-2-amine was obtained as a pale green solid in the same manner as in Example 0797-2 except that N-(4-bromo-1-methyl-1H-pyrazol-3-yl)-N-methylpyridine-3-amine was used instead of the 4-iodo-1-(3-(3-methoxy-3-methylazetidin-1-yl)propyl)-3-methyl-1H-pyrazole used in Example 0797-2.
$^1$H-NMR($CDCl_3$)$\delta$:8.83(1H,d,J=2.0Hz),8.78(2H,s),8.19-8.17(2H,m),8.03-8.01(2H,m),7.84(1H,s),7.52(1H,d,J=9.2Hz),7.08-7.06(2H,m),3.99(3H,s),3.37(3H,s),2.99-2.97(1H,m),1.35(6H,d,J=7.3Hz).
MSm/z(M+H):452.

<Example 0803>

<0803-1>

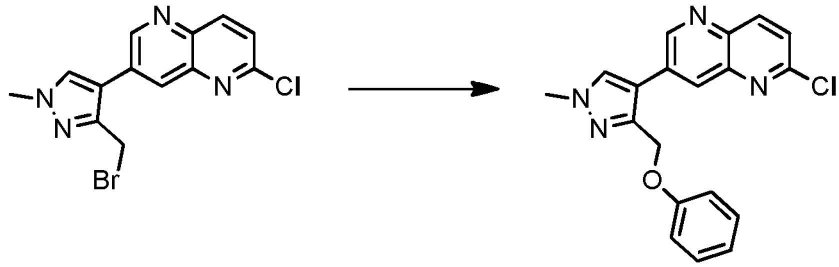

2-Chloro-7-(1-methyl-3-(phenoxymethyl)-1H-pyrazol-4-yl)-1,5-naphthyridine was obtained in the same manner as in Example 0799-1 except that phenol was used instead of the pyridin-3-ol used in Example 0799-1.
MSm/z(M+H):351.

<0803-2>

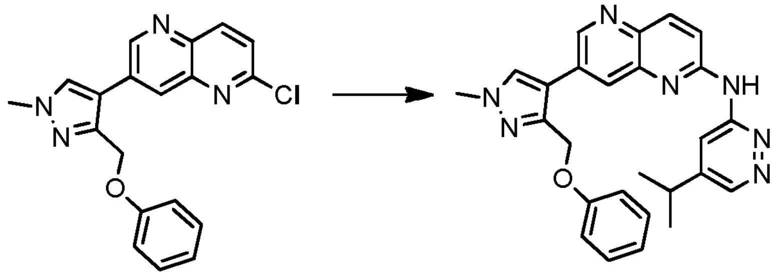

N-(5-isopropylpyridazin-3-yl)-7-(1-methyl-3-(phenoxymethyl)-1H-pyrazol-4-yl)-1,5-naphthyridine-2-amine was obtained as a pale brown solid in the same manner as in Example 0411-3 except that 2-chloro-7-(1-methyl-3-(phenoxymethyl)-1H-pyrazol-4-yl)-1,5-naphthyridine was used instead of the 2-chloro-7-(1-((6-methylpyridin-2-yl)methyl)-1H-pyrazol-4-yl)-1,5-naphthyridine used in Example 0411-3.
$^{1}$H-NMR($CDCl_3$)δ:8.92(1H,d,J=2.0Hz),8.78(2H,d,J=4.6Hz),8.61(1H,brs),8.27-8.24(2H,m),7.74(1H,s),7.48(1H,d,J=9.2Hz), 7.31-7.28(2H,m),7.09(2H,d,J=7.9Hz),6.97(1H,t,J=7.3Hz),5.19(2H,s),4.03(3H,s),2.88-2.86(1H,m), 1.30(6H,d,J=7.3Hz).
MSm/z(M+H):452.

<Example 0804>

<0804-1>

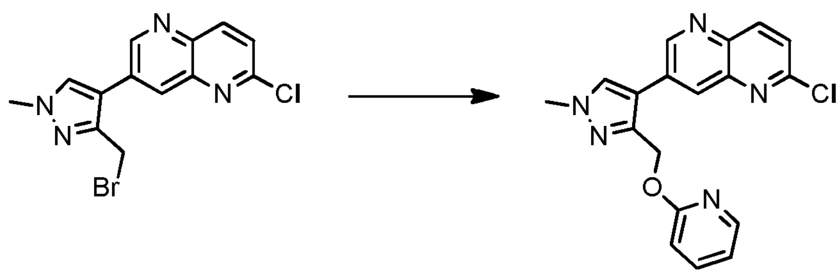

2-Chloro-7-(1-methyl-3-((pyridin-2-yloxy)methyl)-1H-pyrazol-4-yl)-1,5-naphthyridine was obtained in the same manner as in Example 0799-1 except that 2-pyridone was used instead of the pyridin-3-ol used in Example 0799-1. MSm/z(M+H):352.

<0804-2>

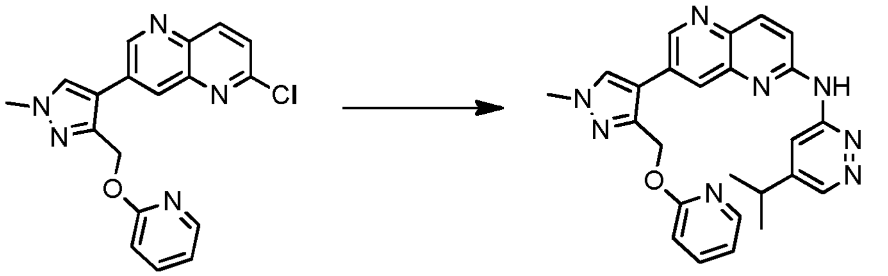

N-(5-isopropylpyridazin-3-yl)-7-(1-methyl-3-((pyridin-2-yloxy)methyl)-1H-pyrazol-4-yl)-1,5-naphthyridine-2-amine was obtained as a pale yellow solid in the same manner as in Example 0799-2 except that 2-chloro-7-(1-methyl-3-((pyridin-2-yloxy)methyl)-1H-pyrazol-4-yl)-1,5-naphthyridine was used instead of the 2-chloro-7-(1-methyl-3-((pyridin-3-yloxy)methyl)-1H-pyrazol-4-yl)-1,5-naphthyridine used in Example 0799-2.

$^1$H-NMR($CDCl_3$)δ:9.47(1H,brs),8.93(1H,s),8.82(1H,s),8.74(1H,d,J=2.0Hz),8.25-8.22(2H,m),7.71(1H,d,J=9.2Hz),7.67(1H,s),7.42-7.41(1H,m),6.53(1H,d,J=9.2Hz),6.11(1H,t,J=6.3Hz),5.35(2H,s),3.98(3H,s),3.12-3.10(1H,m),1.41(6H,d,J=6.6Hz).

MSm/z(M+H):453.

<Example 0805>

<0805-1>

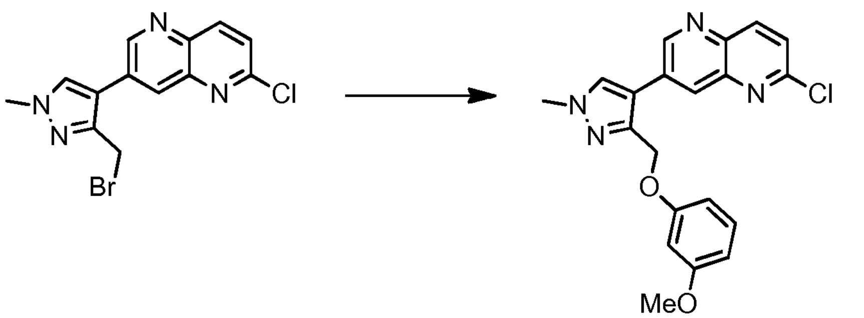

2-Chloro-7-(3-((3-methoxyphenoxy)methyl)-1-methyl-1H-pyrazol-4-yl)-1,5-naphthyridine was obtained in the same manner as in Example 0799-1 except that 3-methoxyphenol was used instead of the pyridin-3-ol used in Example 0799-1.

MSm/z(M+H):381.

<0805-2>

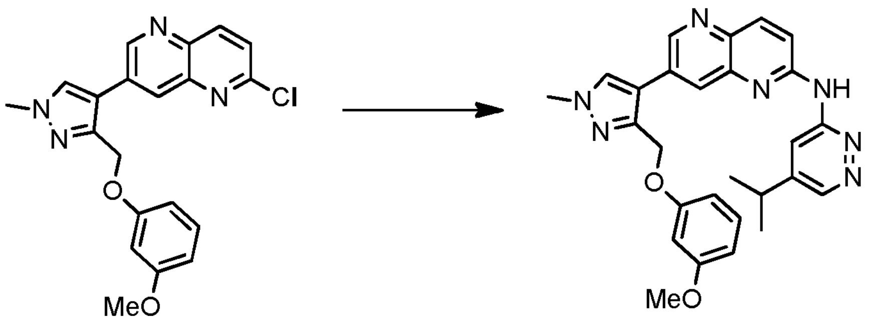

N-(5-isopropylpyridazin-3-yl)-7-(3-((3-methoxyphenoxy)methyl)-1-methyl-1H-pyrazol-4-yl)-1,5-naphthyridine-2-amine was obtained as a pale yellow solid in the same manner as in Example 0799-2 except that 2-chloro-7-(3-((3-methoxyphenoxy)methyl)-1-methyl-1H-pyrazol-4-yl)-1,5-naphthyridine was used instead of the 2-chloro-7-(1-methyl-3-((pyridin-3-yloxy)methyl)-1H-pyrazol-4-yl)-1,5-naphthyridine used in Example 0799-2.
$^1$H-NMR($CDCl_3$)δ:9.15(H,brs),8.91(1H,d,J=2.0Hz),8.82(2H,d,J=19.2Hz),8.25(2H,d,J=7.9Hz),7.74(1H,s ),7.61-7.60(1H,m),7.18(1H,t,J=8.3Hz),6.70-6.64(2H,m),6.53(H,dd,J=8.3,2.3Hz),5.17(2H,s),4.03(3H,s),3.76(3H,s),2.92-2.83(1H,m),1.31(6H,d,J=6.6Hz).
MSm/z(M+H):482.

<Example 0806>

<0806-1>

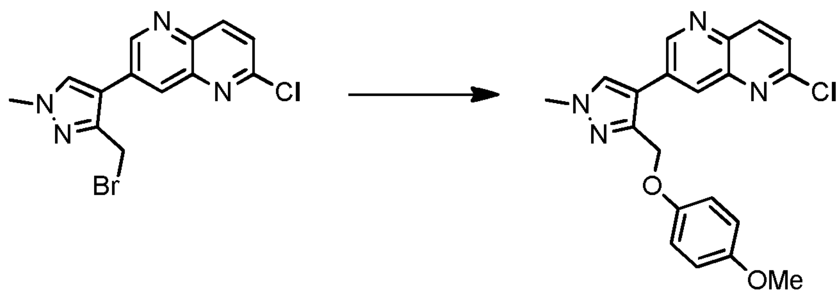

2-Chloro-7-(3-((4-methoxyphenoxy)methyl)-1-methyl-1H-pyrazol-4-yl)-1,5-naphthyridine was obtained in the same manner as in Example 0799-1 except that 4-methoxyphenol was used instead of the pyridin-3-ol used in Example 0799-1.
MSm/z(M+H):381.

<0806-2>

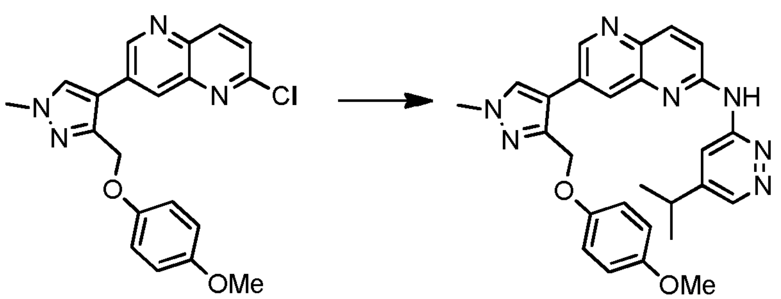

N-(5-isopropylpyridazin-3-yl)-7-(3-((4-methoxyphenoxy)methyl)-1-methyl-1H-pyrazol-4-yl)-1,5-naphthyridine-2-amine was obtained as a white solid in the same manner as in Example 0411-3 except that 2-chloro-7-(3-((4-methoxyphenoxy)methyl)-1-methyl-1H-pyrazol-4-yl)-1,5-naphthyridine was used instead of the 2-chloro-7-(1-((6-methylpyridin-2-yl)methyl)-1H-pyrazol-4-yl)-1,5-naphthyridine used in Example 0411-3.
$^1$H-NMR($CDCl_3$)δ:9.18(1H,brs),8.92(1H,d,J=2.0Hz),8.86(1H,s),8.78(1H,d,J=2.0Hz),8.30(1H,s),8.25(1H, d,J=8.6Hz),7.74(1H,s),7.61(1H,d,J=8.6Hz),7.03-7.01(2H,m),6.83-6.80(2H,m),5.13(2H,s),4.02(3H,s),3. 76(3H,s), 1.31(6H,d,J=6.6Hz).
MSm/z(M+H):482.

<Example 0807>

<0807-1>

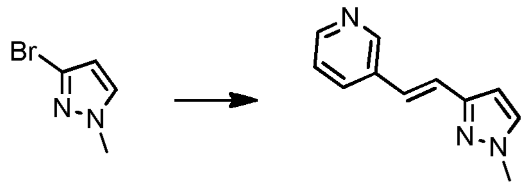

A mixture of 3-bromo-1-methyl-1H-pyrazole (100 mg), 3-vinylpyridine (100 μL), triethylamine (173 μL), palladium acetate(II) (14 mg), tri(o-tolyl)phosphine (76 mg), and N,N-dimethylformamide (3.1 mL) was stirred at 140°C for 30 minutes using a microwave reaction apparatus. The reaction mixture was cooled to room temperature, and purified by silica gel column chromatography (hexane-ethyl acetate-methanol, NH silica), thereby obtaining (E)-3-(2-(1-methyl-1H-pyrazol-3-yl)vinyl)pyridine (18 mg) as pale yellow oily substance.
MSm/z(M+H):186.

<0807-2>

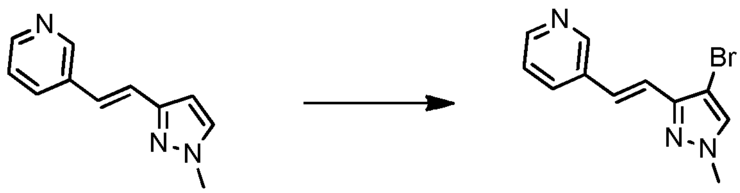

(E)-3-(2-(4-bromo-1-methyl-1H-pyrazol-3-yl)vinyl)pyridine was obtained as colorless oily substance in the same manner as in Example 0451-2 except that (E)-3-(2-(1-methyl-1H-pyrazol-3-yl)vinyl)pyridine was used instead of the 3-methoxy-5-(1-methyl-1H-pyrazol-3-yl)pyridine used in Example 0451-2.
MSm/z(M+H):264.

<0807-3>

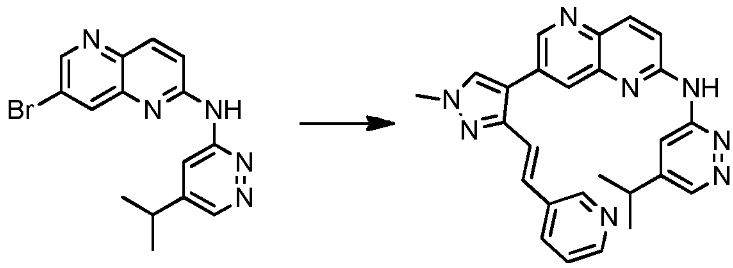

(E)-N-(5-isopropylpyridazin-3-yl)-7-(1-methyl-3-(2-(pyridin-3-yl)vinyl)-1H-pyrazol-4-yl)-1,5-naphthyridine-2-amine was obtained as a pale yellow solid in the same manner as in Example 0797-2 except that (E)-3-(2-(4-bromo-1-methyl-1H-pyrazol-3-yl)vinyl)pyridine was used instead of the 4-iodo-1-(3-(3-methoxy-3-methylazetidin-1-yl)propyl)-3-methyl-1H-pyrazole used in Example 0797-2.
$^{1}$H-NMR($CDCl_3$)δ:9.03(1H,brs),8.86-8.85(2H,m),8.80(1H,d,J=2.0Hz),8.69(1H,d,J=2.0Hz),8.48(1H,dd,J=4.6,1.3Hz),8.30(1H,d,J=9.2Hz),8.09(1H,d,J=2.0Hz),7.80(1H,d,J=7.9Hz),7.65-7.61(2H,m),7.41(1H,d,J=16.5Hz),7.23-7.18(2H,m),4.06(3H,s),3.02-2.89(1H,m),1.30(6H,d,J=6.6Hz).
MSm/z(M+H):449.

<Example 0808>

<0808-1>

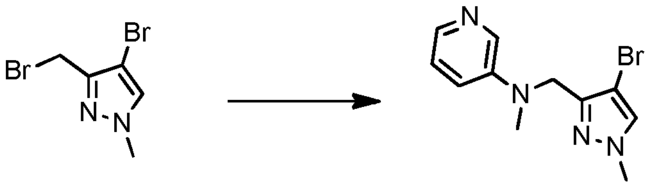

N-((4-bromo-1-methyl-1H-pyrazol-3-yl)methyl)-N-methylpyridine-3-amine was obtained as a brown solid in the same manner as in Example 0799-1 except that N-methylpyridine-3-amine was used instead of the pyridin-3-ol used in Example 0799-1, and 4-bromo-3-(bromomethyl)-1-methyl-1H-pyrazole was used instead of the 7-(3-(bromomethyl)-1-methyl-1H-pyrazol-4-yl)-2-chloro- 1,5-naphthyridine used in Example 0799-1.
MSm/z(M+H):281.

<0808-2>

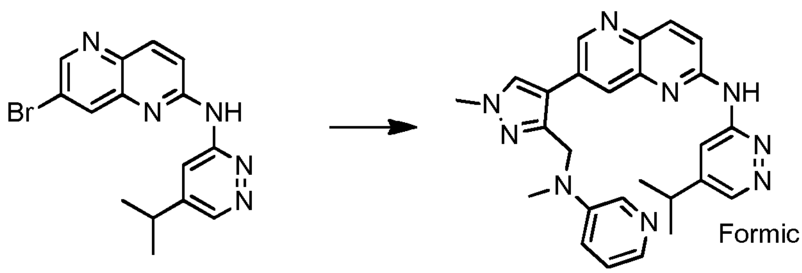

Formic acid salt

N-(5-isopropylpyridazin-3-yl)-7-(1-methyl-3-((methyl (pyridin-3-yl)amino)methyl)-1H-pyrazol-4-yl)-1,5-naphthyridine-2-amin was obtained as a yellow solid in the same manner as in Example 0797-2 except that N-((4-bromo-1-methyl-1H-pyrazol-3-yl)methyl)-N-methylpyridine-3-amine was used instead of the 4-iodo-1-(3-(3-methoxy-3-methylazetidin-1-yl)propyl)-3-methyl-1H-pyrazole used in Example 0797-2.

$^1H$-NMR($CD_3OD$)δ:8.79(1H,s),8.71-8.69(2H,m),8.32(2H,s),8.24(1H,t,J=4.6Hz),8.12-8.11(2H,m),8.00-7.97(2H,m),7.71(1H,d,J=8.6Hz),7.58-7.47(1H,m),5.91(2H,s),3.99(3H,s),3.07-3.03(1H,m), 1.38(6H,d,J=6.6Hz).
MSm/z(M+H):466.

<Example 0809>

<0809-1>

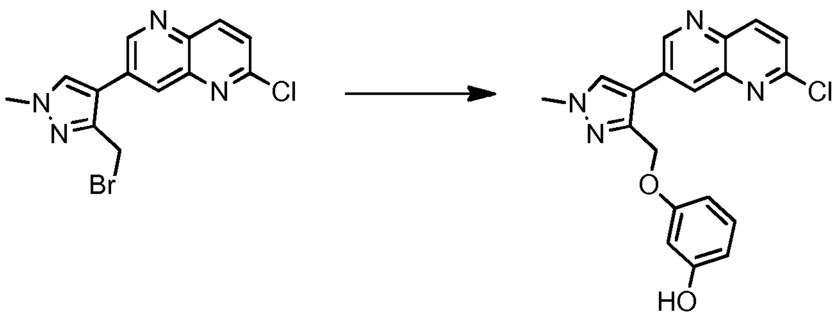

3-((4-(6-Chloro-1,5-naphthyridin-3-yl)-1-methyl-1H-pyrazol-3-yl)methoxy)phenol was obtained as a pale yellow solid in the same manner as in Example 0799-1 except that 3-hydroxyphenyl acetate was used instead of the pyridin-3-ol used in Example 0799-1.
MSm/z(M+H):367.

<0809-2>

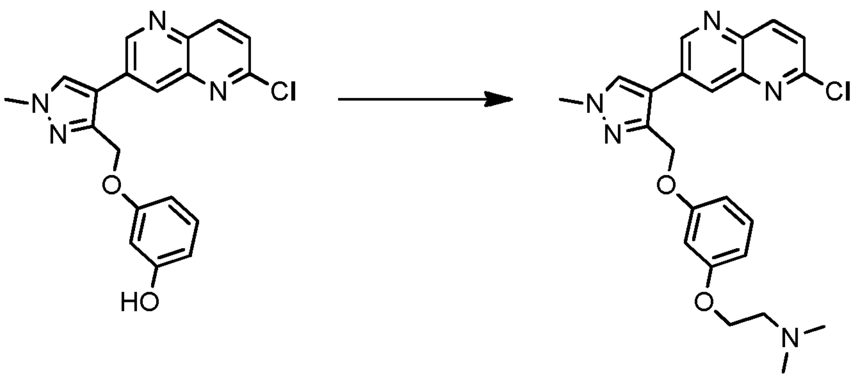

A 40% diethyl azodicarboxylate/toluene solution (5.8 μL) was added to a mixture of 3-((4-(6-chloro-1,5-naphthyridin-3-yl)-1-methyl-1H-pyrazol-3-yl)methoxy)phenol (23 mg), triphenylphosphine (34 mg), 2-(dimethylamino)ethanol (9.6 μL), and tetrahydrofuran (1 mL), followed by stirring at room temperature for 0.5 hours in a nitrogen atmosphere. Triphenylphosphine (35.1 mg) and a 40% diethyl azodicarboxylate/toluene solution (5.8 μL) were added to the reaction mixture, followed by stirring at room temperature for 1 hour. Triphenylphosphine (66.9 mg) and a 40% diethyl azodicarboxylate/toluene solution (11.6 μL) were added to the reaction mixture, followed by stirring at room temperature for 1 hour. Water was added to the reaction mixture, and the resultant product was purified by silica gel column chromatography (hexane-ethyl acetate-methanol, NH silica), thereby obtaining 2-(3-((4-(6-chloro-1,5-naphthyridin-3-yl)-1-methyl-1H-pyrazol-3-yl)methoxy)phenoxy)-N,N-dimethylethanamine (4.5 mg) as a white solid.
MSm/z(M+H):438.

<0809-3>

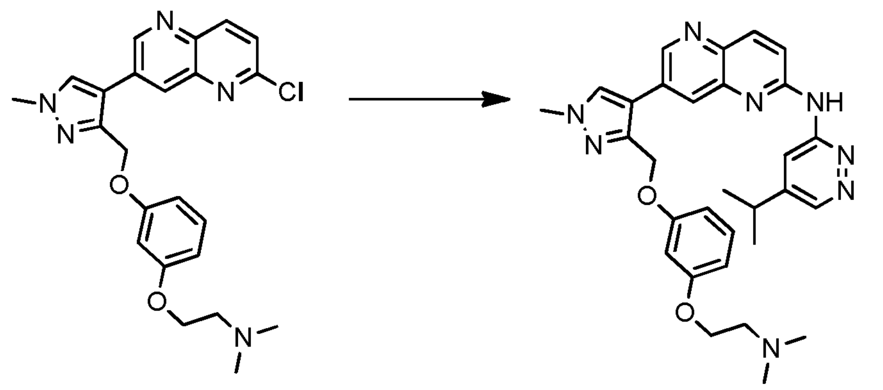

7-(3-((3-(2-(dimethylamino)ethoxy)phenoxy)methyl)-1-methyl-1H-pyrazol-4-yl)-N-(5-isopropylpyridazin-3-yl)-1,5-naphthyridine-2-amine was obtained as a white solid in the same manner as in Example 0799-2 except that 2-(3-((4-(6-chloro-1,5-naphthyridin-3-yl)-1-methyl-1H-pyrazol-3-yl)methoxy)phenoxy)-N,N-dimethylethanamine was used instead of the 2-chloro-7-(1-methyl-3-((pyridin-3-yloxy)methyl)-1H-pyrazol-4-yl)-1,5-naphthyridine used in Example 0799-2.
$^1$H-NMR($CDCl_3$)δ:8.91(1H,d,J=2.0Hz),8.80(1H,d,J=11.2Hz),8.26-8.23(2H,m),7.74(1H,s),7.61-7.58(1H,m),7.19-7.08(1H,m),6.68-6.66(2H,m),6.54-6.44(2H,m),5.15(2H,s),4.07-4.00(5H,m),2.93-2.84(1H,m),2.74-2.70(2H,m),2.32(6H,s),1.31(6H,d,J=6.6Hz).
MSm/z(M+H):539.

<Example 0810>

<0810-1>

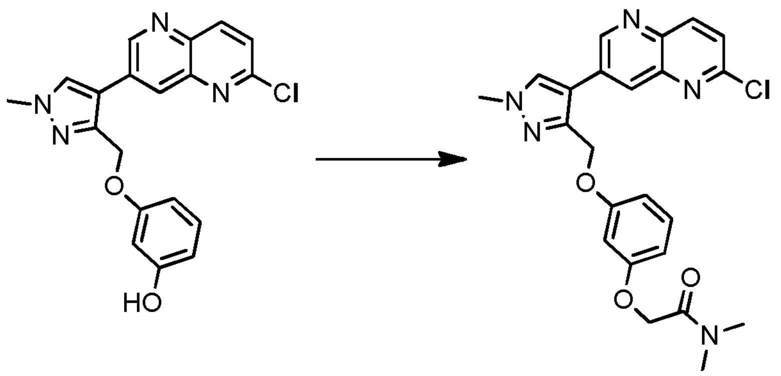

2-(3-((4-(6-Chloro-1,5-naphthyridin-3-yl)-1-methyl-1H-pyrazol-3-yl)methoxy)phenoxy)-N,N-dimethylacetamide was obtained as a white solid in the same manner as in Example 0799-1 except that 3-((4-(6-chloro-1,5-naphthyridin-3-yl)-1-methyl-1H-pyrazol-3-yl)methoxy)phenol was used instead of the pyridin-3-ol used in Example 0799-1, and 2-chloro-N,N-dimethylacetamide was used instead of the 7-(3-(bromomethyl)-1-methyl-1H-pyrazol-4-yl)-2-chloro-1,5-naphthyridine used in Example 0799-1.
MSm/z(M+H):452.

<0810-2>

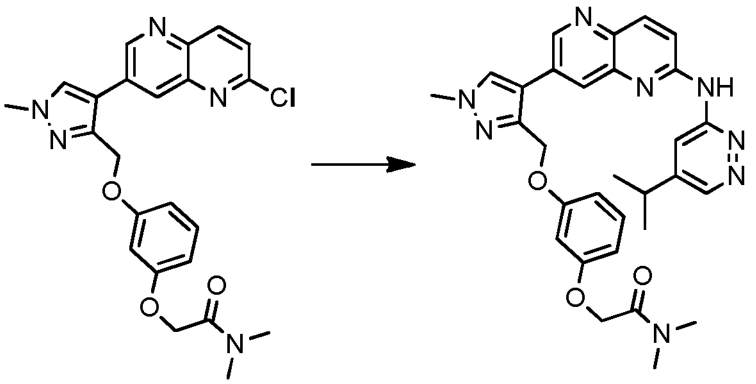

2-(3-((4-(6-((5-isopropylpyridazin-3-yl)amino)-1,5-naphthyridin-3-yl)-1-methyl-1H-pyrazol-3-yl)methoxy)phenoxy)-N,N-dimethylacetamide was obtained as a pale brown solid in the same manner as in Example 0799-2 except that 2-(3-((4-(6-chloro-1,5-naphthyridin-3-yl)-1-methyl-1H-pyrazol-3-yl)methoxy)phenoxy)-N,N-dimethylacetamide was used instead of the 2-chloro-7-(1-methyl-3-((pyridin-3-yloxy)methyl)-1H-pyrazol-4-yl)-1,5-naphthyridine used in Example 0799-2.

$^1$H-NMR($CDCl_3$)δ:8.91(1H,s),8.77(1H,s),8.70(1H,brs),8.26-8.23(2H,m),7.73(1H,d,J=5.9Hz),7.59-7.56(1H,m),7.18(2H,t,J=8.3Hz),6.73-6.71(2H,m),6.60-6.57(1H,m),5.16(2H,s),4.65(2H,s),4.03(3H,s),3.06(3H,s),2.97(3H,s),2.92-2.86(1H,m),1.31-1.23(6H,m).
MSm/z(M+H):553.

<Example 0811>

<0811-1>

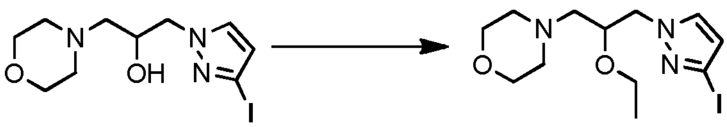

4-(2-Ethoxy-3-(3-iodo-1H-pyrazol-1-yl)propyl)morpholine was obtained in the same manner as in Example 0732-2 except that ethyl iodide was used instead of the benzyl bromide used in Example 0732-2.
MSm/z(M+H):366.

<0811-2>

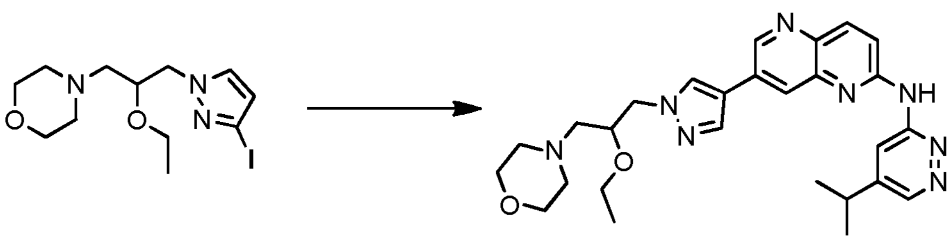

7-(1-(2-Ethoxy-3-morpholinopropyl)-1H-pyrazol-4-yl)-N-(5-isopropylpyridazin-3-yl)-1,5-naphthyridine-2-amine was obtained as a pale yellow solid in the same manner as in Example 0385-7 except that 7-bromo-N-(5-isopropylpyridazin-3-yl)-1,5-naphthyridine-2-amine was used instead of the 7-bromo-2-chloro-1,5-naphthyridine used in Example 0385-7, and 4-(2-ethoxy-3-(3-iodo-1H-pyrazol-1-yl)propyl)morpholine was used instead of the 4-(3-(3-ethyl-4-iodo-1H-pyrazol-1-yl)propyl)morpholine used in Example 0385-7.

$^1H$-NMR($CDCl_3$)δ:9.05(1H,brs),8.93(1H,d,J=2.0Hz),8.86(1H,s),8.81(1H,s),8.24(1H,d,J=8.9Hz),8.10(1H,s ),7.97(2H,s),7.54(1H,d,J=8.9Hz),4.51(1H,dd,J=14.0,4.0Hz),4.19(1H,dd,J=14.0,7.6Hz),3.90-3.80(1H,m),3.73(4H,t,J=4.6Hz),3.65-3.56(1H,m),3.39-3.31(1H,m),3.09-3.02(1H,m),2.56-2.46(6H,m), 1.41(6H,d,J=7.2Hz), 1.11(3H,t,J=6.9Hz).
MSm/z(M+H):503.

<Example 0812>

<0812-1>

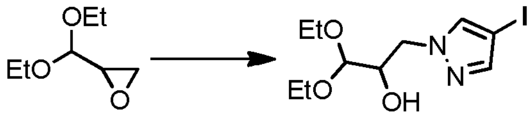

Cesium carbonate (424 mg), N,N-dimethylformamide (2 mL), and 3-iodo-1H-pyrazole (233 mg) were added to 2-(diethoxymethyl)oxirane (146 mg), followed by stirring at 90°C for 1.5 hours. The reaction mixture was cooled to room temperature, water was added thereto, and the resultant product was extracted three times with ethyl acetate. An organic layer thus obtained was washed with a saturated sodium chloride aqueous solution, and dried over anhydrous sodium sulfate, and the solvent was distilled off under reduced pressure. The obtained residue was purified by silica gel column chromatography (hexane-ethyl acetate), thereby obtaining 1,1-diethoxy-3-(4-iodo-1H-pyrazol-1-yl)propan-2-ol (350 mg) as colorless oily substance.
MSm/z(M+H):341.

<0812-2>

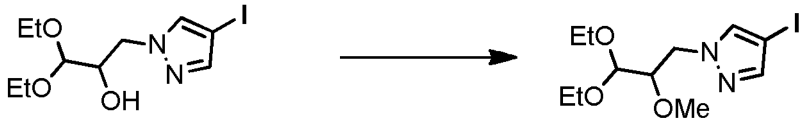

1-(3,3-Diethoxy-2-methoxypropyl)-4-iodo-1H-pyrazole was obtained as colorless oily substance in the same manner as in Example 0725-1 except that 1,1-diethoxy-3-(4-iodo-1H-pyrazol-1-yl)propan-2-ol was used instead of the 1-(4-(6-chloro-1,5-naphthyridin-3-yl)-1H-pyrazol-1-yl)-3-(pyrrolidin-1-yl)propan-2-ol used in Example 0725-1.
MSm/z(M+H):355.

<0812-3>

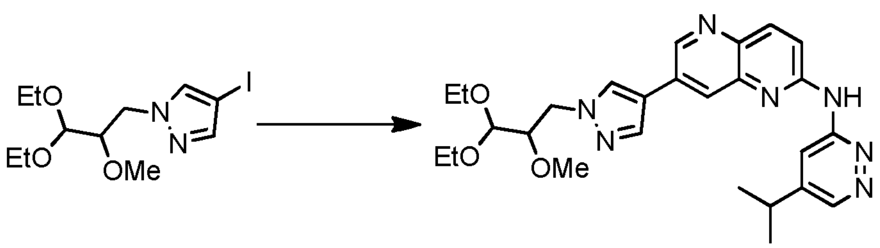

7-(1-(3,3-Diethoxy-2-methoxypropyl)-1H-pyrazol-4-yl)-N-(5-isopropylpyridazin-3-yl)-1,5-naphthyridine-2-amine was obtained as a pale yellow solid in the same manner as in Example 0385-7 except that 7-bromo-N-(5-isopropylpyridazin-3-yl)-1,5-naphthyridine-2-amine was used instead of the 7-bromo-2-chloro-1,5-naphthyridine used in Example 0385-7, and 1-(3,3-diethoxy-2-methoxypropyl)-4-iodo-1H-pyrazole was used instead of the 4-(3-(3-ethyl-4-iodo-1H-pyrazol-1-yl)propyl)morpholine used in Example 0385-7.

MSm/z(M+H):492.

<0812-4>

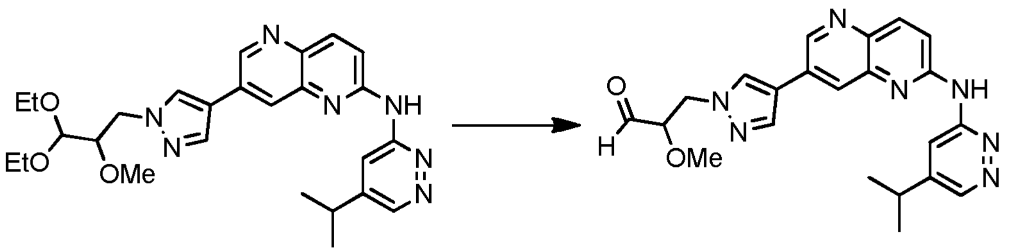

1 mol/L hydrochloric acid (1 mL) was added to a solution of 7-(1-(3,3-diethoxy-2-methoxypropyl)-1H-pyrazol-4-yl)-N-(5-isopropylpyridazin-3-yl)-1,5-naphthyridine-2-amine (50 mg) in 1,4-dioxane (1 mL), followed by stirring at 70°C for 0.5 hours. The reaction mixture was cooled to room temperature, and the solvent was distilled off under reduced pressure. The obtained residue was dissolved in 2-propanol (1 mL), an ethyl acetate/chloroform (1/1) solution (1 mL) was added thereto, and the precipitated solid was collected by filtration, thereby obtaining 3-(4-(6-((5-isopropylpyridazin-3-yl)amino)-1,5-naphthyridin-3-yl)-1H-pyrazol-1-yl)-2-methoxypropanal hydrochloride (50 mg).
MSm/z(M+H):450. (methanol adduct)

<0812-5>

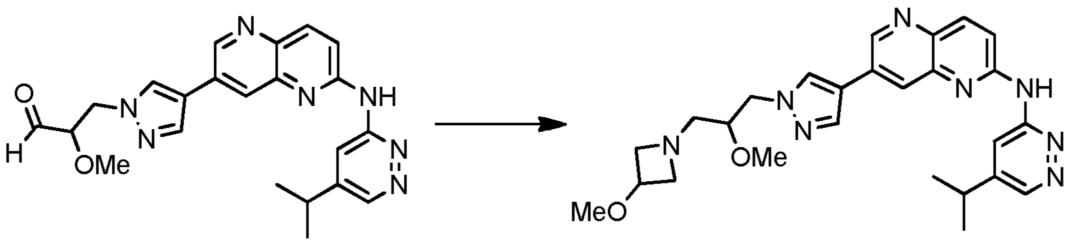

3-Methoxyazetidine hydrochloride (2 mg), triethylamine (4 $\mu$L), and sodium triacetoxyborohydride (3 mg) were added to a mixture of 3-(4-(6-((5-isopropylpyridazin-3-yl)amino)-1,5-naphthyridin-3-yl)-1H-pyrazol-1-yl)-2-methoxypropanal hydrochloride (5 mg) and dichloromethane (0.5 mL), followed by stirring at room temperature for 3 hours. Sodium triacetoxyborohydride (3 mg) was added to the reaction mixture, followed by stirring at room temperature for 1.5 hours. A drop of water was added to the reaction mixture, and the solvent was distilled off under reduced pressure. The obtained residue was purified by silica gel column chromatography (hexane-ethyl acetate, NH silica), thereby obtaining N-(5-isopropylpyridazin-3-yl)-7-(1-(2-methoxy-3-(3-methoxyazetidin-1-yl)propyl)-1H-pyrazol-4-yl)-1,5-naphthyridine-2-amine (2.7 mg) as a pale yellow solid.
$^1$H-NMR($CDCl_3$)$\delta$:9.35(1H,brs),8.93(1H,d,J=2.0Hz),8.90(1H,s),8.82(1H,s),8.25(1H,d,J=8.6Hz),8.11(1H,s ),7.97(1H,s),7.93(1H,s),7.62(1H,d,J=8.6Hz),4.39(1H,dd,J=13.9,4.0Hz),4.24-4.14(1H,m),4.11-4.03(1H,m),3.74-3.69(2H,m),3.66-3.60(1H,m),3.33(3H,s),3.27(3H,s),3.12-2.99(3H,m),2.69-2.54(2H,m), 1.43(6H,d,J=6.6Hz).
MSm/z(M+H):489.

<Example 0813>

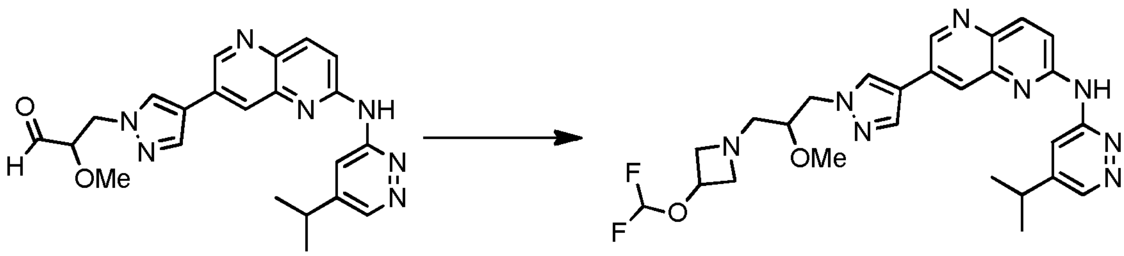

7-(1-(3-(3-(Difluoromethoxy)azetidin-1-yl)-2-methoxypropyl)-1H-pyrazol-4-yl)-N-(5-isopropylpyridazin-3-yl)-1,5-naphthyridine-2-amine was obtained as a pale yellow solid in the same manner as in Example 0812-5 except that trifluoroacetic acid salt of 3-(difluoromethoxy)azetidine was used instead of the 3-methoxyazetidine hydrochloride used in Example 0812-5.

$^1$H-NMR($CDCl_3$)δ:9.49(1H,s),8.95-8.89(2H,m),8.83(1H,s),8.25(1H,d,J=8.6Hz),8.11(1H,d,J=1.3Hz),7.97(1H,s),7.92(1H,s), 7.66(1H,d,J=9. 2Hz),6.18(1H,t,J=73.7Hz),4.83-4.75(1H,m),4.38(1H,dd,J=14.2,4.3Hz),4.22(1H,dd,J=14.2,7.3Hz),3.80-3.73(2H,m),3.66-3.59(1H,m),3.33(3H,s),3.15(2H,t,J=7.3Hz),3.09-3.04(1H,m),2.70-2.53(2H,m),1.43(6H,d,J=7.3Hz).

MSm/z(M+H):525.

<Example 0814>

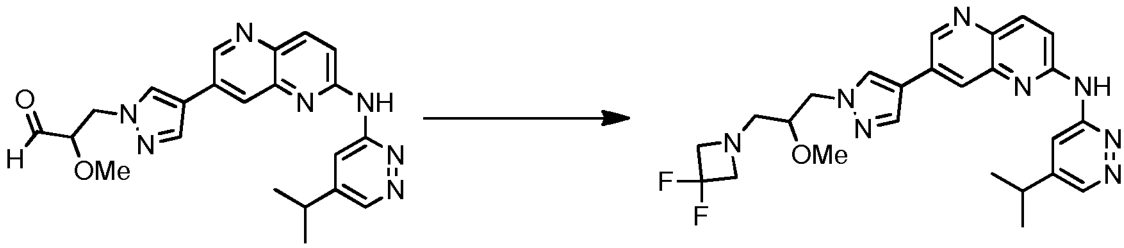

3,3-Difluoroazetidine hydrochloride (4 mg), methanol (450 μL), acetic acid (50 μL), and 2-picoline borane (3 mg) were added to 3-(4-(6-((5-isopropylpyridazin-3-yl)amino)-1,5-naphthyridin-3-yl)-1H-pyrazol-1-yl)-2-methoxypropanal hydrochloride (5 mg), followed by stirring at room temperature for 6 hours. The solvent of the reaction mixture was distilled off under reduced pressure. The obtained residue was purified by silica gel column chromatography (hexane-ethyl acetate, NH silica), thereby obtaining 7-(1-(3-(3,3-difluoroazetidin-1-yl)-2-methoxypropyl)-1H-pyrazol-4-yl)-N-(5-isopropylpyridazin-3-yl)-1,5-naphthyridine-2-amine (2.2 mg) as a pale yellow solid.

$^1$H-NMR($CDCl_3$)δ:9.40(1H,s),8.94(1H,d,J=2.0Hz),8.90(1H,s),8.83(1H,d,J=2.0Hz),8.25(1H,d,J=9.2Hz),8. 11(1H,d,J=2.0Hz),7.98(1H,s),7.92(1H,s),7.65(1H,d,J=9.2Hz),4.39(1H,dd,J=14.2,4.6Hz),4.26(1H,dd,J =14.2,6.6Hz),3.72-3.63(5H,m),3.34(3H,s),3.12-3.02(1H,m),2.78(1H,dd,J=12.9,4.6Hz),2.65-2.58(1H,m),1.43(6H,d,J=7. 3Hz).

MSm/z(M+H):495.

<Example 0815>

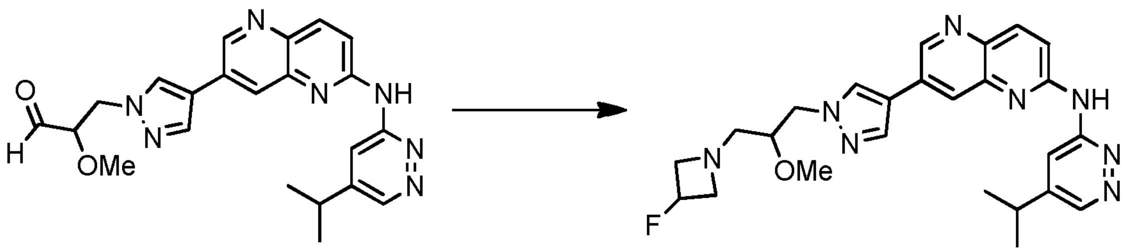

7-(1-(3-(3-Fluoroazetidin-1-yl)-2-methoxypropyl)-1H-pyrazol-4-yl)-N-(5-isopropylpyridazin-3-yl)-1,5-naphthyridine-2-amine was obtained as a white solid in the same manner as in Example 0814 except that 3-fluoroazetidine hydrochloride was used instead of the 3,3-difluoroazetidine hydrochloride used in Example 0814.
$^1$H-NMR($CDCl_3$)δ:8.94(1H,s),8.84-8.80(3H,m),8.24(1H,d,J=9.2Hz),8.11(1H,s),7.98(1H,s),7.93(1H,s),7.52(1H,d,J=9.2Hz), 5.14(1H,dt,J=57.2,5.1Hz),4.39(1H,dd,J=14.2,4.0Hz),4.22(1H,dd,J=14.2,6.9Hz),3.80-3.71(2H,m),3.67-3.60(1H,m),3.34(3H,s),3.31-3.18(2H,m),3.09-3.00(1H,m),2.72-2.54(2H,m),1.42(6H,d,J=7.3Hz).
MSm/z(M+H):477.

<Example 0816>

<0816-1>

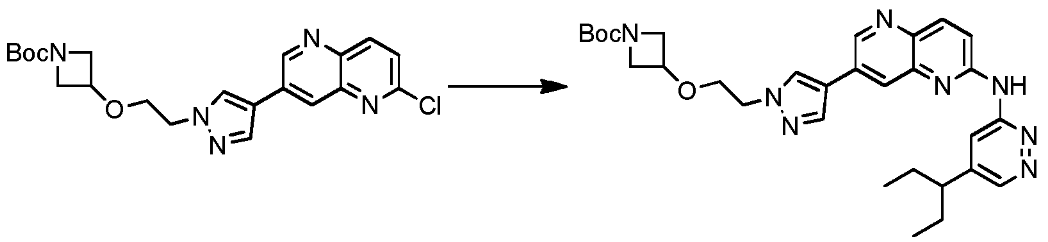

tert-Butyl 3-(2-(4-(6-((5-(pentan-3-yl)pyridazin-3-yl)amino)-1,5-naphthyridin-3-yl)-1H-pyrazol-1-yl)ethoxy)azetidine-1-carboxylate was obtained as a pale yellow solid in the same manner as in Example 0729-2 except that tert-butyl 3-(2-(4-(6-chloro-1,5-naphthyridin-3-yl)-1H-pyrazol-1-yl)ethoxy)azetidine-1-carboxylate was used instead of the 1-(4-(6-chloro-1,5-naphthyridin-3-yl)-1H-pyrazol-1-yl)-3-morpholinopropan-2-ol used in Example 0729-2, and 5-(pentan-3-yl)pyridazine-3-amine was used instead of the 5-isopropylpyridazine-3-amine used in Example 0729-2.
MSm/z(M+H):559.

<0816-2>

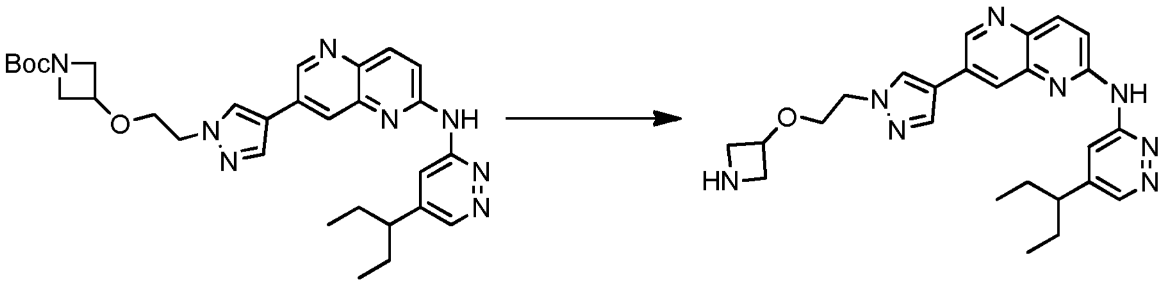

7-(1-(2-(Azetidin-3-yloxy)ethyl)-1H-pyrazol-4-yl)-N-(5-pentan-3-yl)pyridazin-3-yl)-1,5-naphthyridine-2-amine was obtained as a yellow solid in the same manner as in Example 0740 except that tert-butyl 3-(2-(4-(6-((5-(pentan-3-yl)pyridazin-3-yl)amino)-1,5-naphthyridin-3-yl)-1H-pyrazol-1-yl)ethoxy)azetidine-1-carboxylate was used instead of the tert-butyl 3-(2-(4-(6-((5-isopropylpyridazin-3-yl)amino)-1,5-naphthyridin-3-yl)-1H-pyrazol-1-yl)ethoxy)azetidine-1-carboxylate used in Example 0740.
$^1$H-NMR($CDCl_3$)δ:9.06(1H,brs),8.94(1H,d,J=2.0Hz),8.76(1H,s),8.74(1H,s),8.25(1H,d,J=8.9Hz),8.09(1H, d,J=2.0Hz),7.98(1H,s),7.96(1H,s),7.60(1H,d,J=8.9Hz),4.39(2H,t,J=5.1Hz),4.35-4.28(1H,m),3.80(2H,t,J=5.1Hz),3.70-3.64(2H,m),3.55(2H,t,J=7.6Hz),2.57-2.48(1H,m),1.92-1.68(4H,m),0.93(6H,t,J=7.6Hz).
MSm/z(M+H):459.

<Example 0817>

<0817-1>

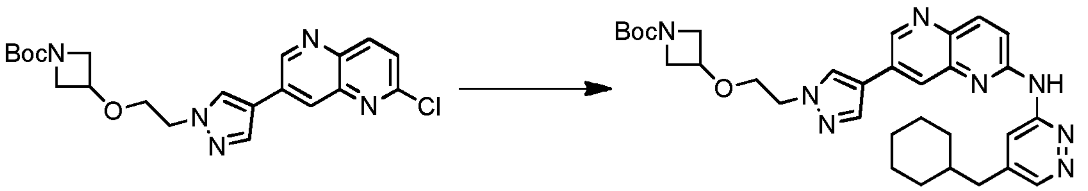

tert-Butyl 3-(2-(4-(6-((5-(cyclohexylmethyl)pyridazin-3-yl)amino)-1,5-naphthyridin-3-yl)-1H-pyrazol-1-yl)ethoxy)azetidine-1-carboxylate was obtained as a pale yellow solid in the same manner as in Example 0729-2 except that tert-butyl 3-(2-(4-(6-chloro-1,5-naphthyridin-3-yl)-1H-pyrazol-1-yl)ethoxy)azetidine-1-carboxylate was used instead of the 1-(4-(6-chloro-1,5-naphthyridin-3-yl)-1H-pyrazol-1-yl)-3-morpholinopropan-2-ol used in Example 0729-2, and 5-(cyclohexylmethyl)pyridazine-3-amine was used instead of the 5-isopropylpyridazine-3-amine used in Example 0729-2.
MSm/z(M+H):585.

<0817-2>

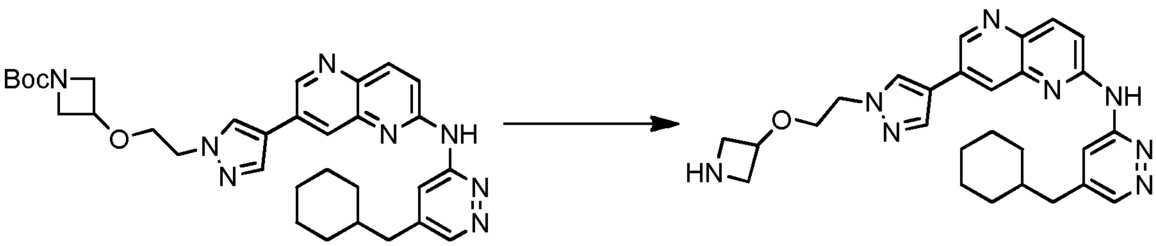

7-(1-(2-(Azetidin-3-yloxy)ethyl)-1H-pyrazol-4-yl)-N-(5-(cyclohexylmethyl)pyridazin-3-yl)-1,5-naphthyridine-2-amine was obtained as a pale yellow solid in the same manner as in Example 0740 except that tert-butyl 3-(2-(4-(6-((5-(cyclohexylmethyl)pyridazin-3-yl)amino)-1,5-naphthyridin-3-yl)-1H-pyrazol-1-yl)ethoxy)azetidine-1-carboxylate was used instead of the tert-butyl 3-(2-(4-(6-((5-isopropylpyridazin-3-yl)amino)-1,5-naphthyridin-3-yl)-1H-pyrazol-1-yl)ethoxy)azetidine-1-carboxylate used in Example 0740.
$^1$H-NMR($CDCl_3$)$\delta$:8.94(1H,d,J=2.0Hz),8.86(1H,brs),8.71(1H,s),8.70(1H,s),8.24(1H,d,J=9.2Hz),8.13(1H, d,J=2.0Hz),7.98(1H,s),7.96(1H,s),7.53(1H,d,J=9.2Hz),4.40-4.28(3H,m),3.80(2H,t,J=5.0Hz),3.67(2H,t,J=7.9Hz),3.58-3.50(2H,m),2.62(2H,d,J=6.6Hz),1.83-1.67(7H,m),1.32-1.05(5H,m).
MSm/z(M+H):485.

<Example 0818>

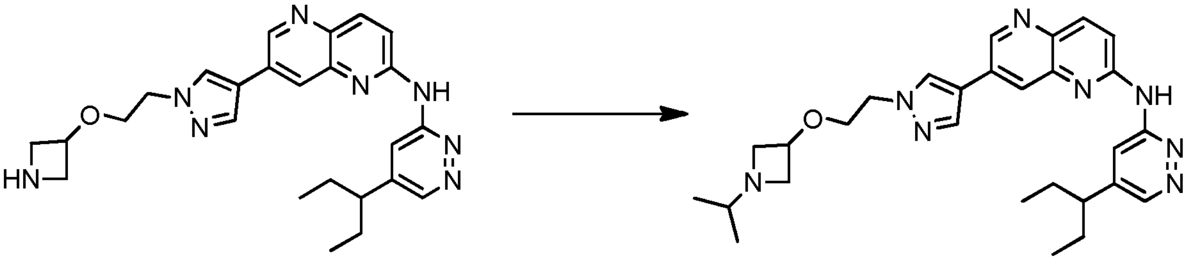

7-(1-(2-((1-Isopropylazetidin-3-yl)oxy)ethyl)-1H-pyrazol-4-yl)-N-(5-(pentan- 3-yl)pyridazin-3-yl)-1,5-naphthyridine-2-amine was obtained as a pale yellow solid in the same manner as in Example 0741 except that 7-(1-(2-(azetidin-3-yloxy)ethyl)-1H-pyrazol-4-yl)-N-(5-(pentan-3-yl)pyridazin-3-yl)-1,5-naphthyridine-2-amine was used instead of the 7-(1-(2-(azetidin-3-yloxy)ethyl)-1H-pyrazol-4-yl)-N-(5-isopropylpyridazin-3-yl)-1,5-naphthyridine-2-amine used in Example 0741, and acetone was used instead of the formaldehyde aqueous solution used in Example 0741.
$^1$H-NMR($CDCl_3$)δ:8.93(1H,d,J=2.0Hz),8.81(1H,brs),8.75(1H,s),8.25(1H,d,J=9.2Hz),8.09(1H,d,J=2.0Hz), 7.98(1H,s),7.94(1H,s),7.67(1H,brs),4.38(2H,t,J=5.3Hz),4.11-4.02(1H,m),3.80(2H,t,J=5.3Hz),3.59-3.52(2H,m),2.86-2.80(2H,m),2.57-2.48(1H,m),2.31-2.22(1H,m),1.92-1.70(4H,m),0.96-0.89(12H,m).
MSm/z(M+H):501.

<Example 0819>

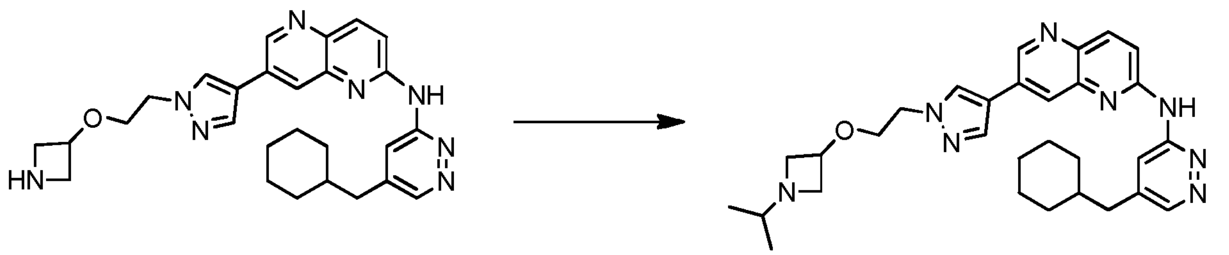

N-(5-(cyclohexylmethyl)pyridazin-3-yl)-7-(1-(2-((1-isopropylazetidin-3-yl)oxy)ethyl)-1H-pyrazol-4-yl)-1,5-naphthyridine-2-amine was obtained as a pale yellow solid in the same manner as in Example 0741 except that 7-(1-(2-(azetidin-3-yloxy)ethyl)-1H-pyrazol-4-yl)-N-(5-(cyclohexylmethyl)pyridazin-3-yl)-1,5-naphthyridine-2-amine was used instead of the 7-(1-(2-(azetidin-3-yloxy)ethyl)-1H-pyrazol-4-yl)-N-(5-isopropylpyridazin-3-yl)-1,5-naphthyridine-2- used in Example 0741, and acetone was used instead of the formaldehyde aqueous solution used in Example 0741.
$^1$H-NMR($CDCl_3$)δ:8.93(1H,d,J=2.0Hz),8.81(1H,brs),8.74(1H,d,J=2.0Hz),8.25(1H,d,J=9.2Hz),8.13(1H,d,J =2.0Hz),7.98(1H,s),7.95(1H,s),7.74(1H,brs),4.38(2H,t,J=5.3Hz),4.11-4.03(1H,m),3.80(2H,t,J=5.3Hz),3.58-3.52(2H,m) ,2.85-2.79(2H,m),2.64(2H,d,J=6.6Hz),2.30-2.21(1H,m),1.85-1.74(6H,m),1.32-1.05(5H,m),0.90(6H,d,J=5.9Hz).
MSm/z(M+H):527.

<Example 0820>

<0820-1>

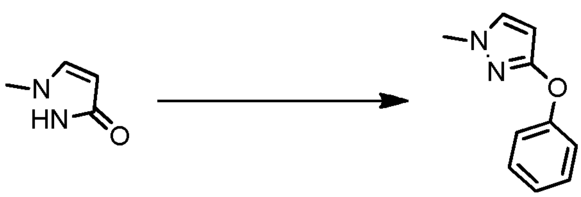

Methylene chloride (10 mL), phenylboronic acid (300 mg), triethylamine (220 μL), pyridine (254 μL), copper(II) acetate (445 mg), and molecular sieve 4A(300 mg) were added to 1-methyl-1H-pyrazol-3(2H)-one (160 mg), followed by stirring at room temperature for 18 hours. After water was added to the reaction mixture, the resultant product was extracted three times with methylene chloride, an organic layer thus obtained was washed with a saturated sodium chloride aqueous solution, and dried over anhydrous sodium sulfate. The solvent was distilled off under reduced pressure, and the obtained residue was purified by silica gel column chromatography (hexane-ethyl acetate), thereby obtaining 1-methyl-3-phenoxy-1H-pyrazole (27 mg) as colorless oily substance.
MSm/z(M+H):175.

<0820-2>

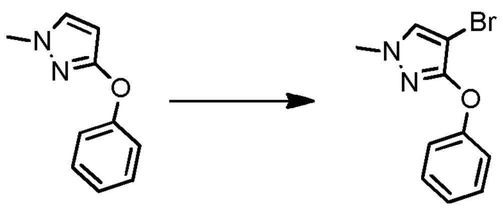

4-Bromo-1-methyl-3-phenoxy-1H-pyrazole was obtained as pale yellow oily substance in the same manner as in Example 0734-2 except that 1-methyl-3-phenoxy-1H-pyrazole was used instead of the 2-methyl-5-(1-methyl-1H-pyrazol-3-yl)pyridine used in Example 0734-2.
MSm/z(M+H):253.

<0820-3>

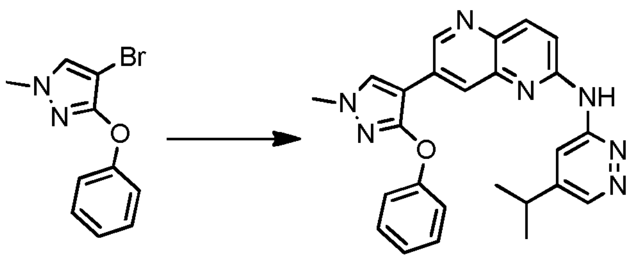

N-(5-isopropylpyridazin-3-yl)-7-(1-methyl-3-phenoxy-1H-pyrazol-4-yl)-1,5-naphthyridine-2-amine was obtained as a white solid in the same manner as in Example 0385-7 except that 7-bromo-N-(5-isopropylpyridazin-3-yl)-1,5-naphthyridine-2-amine was used instead of the 7-bromo-2-chloro-1,5-naphthyridine used in Example 0385-7, and 4-bromo-1-methyl-3-phenoxy-1H-pyrazole was used instead of the 4-(3-(3-ethyl-4-iodo-1H-pyrazol-1-yl)propyl)morpholine used in Example 0385-7.
$^1$H-NMR($CDCl_3$)δ:9.00(1H,s),8.81(1H,s),8.77(1H,s),8.35(1H,s),8.20(1H,d,J=8.6Hz),7.80(1H,s),7.36-7.30(2H,m), 7.22-7.19(3H,m), 7.12-7.06(2H,m),3.92(3H,s),3.05-2.95(1H,m), 1.36(6H,d,J=7.3Hz).
MSm/z(M+H):438.

<Example 0821>

<0821-1>

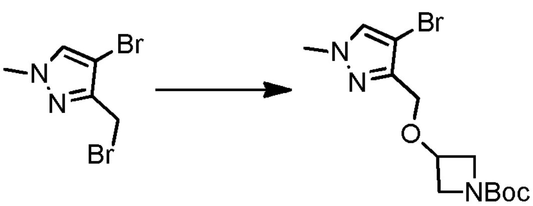

60% sodium hydride (83 mg) was added to a solution of tert-butyl 3-hydroxyazetidine-1-carboxylate (286 mg) in tetrahydrofuran (5 mL) under ice-cooling, followed by stirring for 0.5 hours. 4-Bromo-3-(bromomethyl)-1-methyl-1H-pyrazole (350 mg) was added to the reaction mixture, followed by stirring at room temperature for 1 hour, and stirring at 50°C for 1.5 hours. Furthermore, N,N-dimethylformamide (5 mL) was added thereto, followed by stirring at 50°C for

1 hour. After a 20% citric acid aqueous solution was added to the reaction mixture, the resultant product was extracted three times with ethyl acetate, an organic layer thus obtained was washed with a saturated sodium chloride aqueous solution, and dried over anhydrous sodium sulfate. The solvent was distilled off under reduced pressure, and the obtained residue was purified by silica gel column chromatography (hexane-acetone), thereby obtaining tert-butyl 3-((4-bromo-1-methyl-1H-pyrazol-3-yl)methoxy)azetidine-1-carboxylate (530 mg).
MSm/z(M+H):346.

<0821-2>

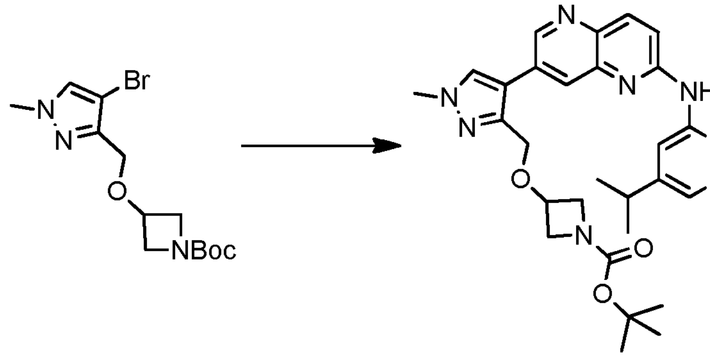

tert-Butyl 3-((4-(6-((5-isopropylpyridazin-3-yl)amino)-1,5-naphthyridin-3-yl)-1-methyl-1H-pyrazol-3-yl)methoxy)azetidine-1-carboxylate was obtained as a pale yellow solid in the same manner as in Example 0385-7 except that 7-bromo-N-(5-isopropylpyridazin-3-yl)-1,5-naphthyridine-2-amine was used instead of the 7-bromo-2-chloro-1,5-naphthyridine used in Example 0385-7, and tert-butyl 3-((4-bromo-1-methyl-1H-pyrazol-3-yl)methoxy)azetidine-1-carboxylate was used instead of the 4-(3-(3-ethyl-4-iodo-1H-pyrazol-1-yl)propyl)morpholine used in Example 0385-7.
$^1$H-NMR($CDCl_3$)δ:9.50(1H,brs),8.90(1H,d,J=2.0Hz),8.80(2H,brs),8.33(1H,s),8.28(1H,d,J=9.2Hz),7.84(1H,d,J=9.2Hz),7.73(1H,s),4.60(2H,s),4.51-4.42(1H,m),4.14-4.05(2H,m),4.00(3H,s),3.92(2H,dd,J=9.6,4.3Hz),3.07-2.98(1H,m),1.42(9H,s),1.41(6H,d,J=6.9Hz).
MSm/z(M+H):531.

<Example 0822>

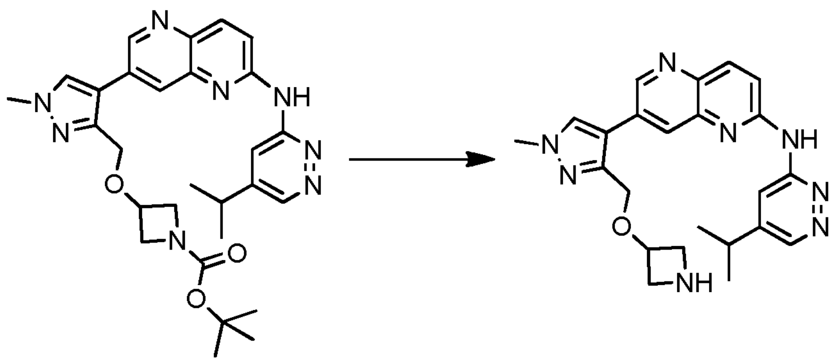

7-(3-((Azetidin-3-yloxy)methyl)-1-methyl-1H-pyrazol-4-yl)-N-(5-isopropylpyridazin-3-yl)-1,5-naphthyridine-2-amine was obtained in the same manner as in Example 0740 except that tert-butyl 3-((4-(6-((5-isopropylpyridazm-3-yl)amino)-1,5-naphthyridm-3-yl)-1-methyl-1H-pyrazol-3-yl)methoxy)azetidme-1-carboxylate was used instead of the tert-butyl 3-(2-(4-(6-((5-isopropylpyridazin-3-yl)amino)-1,5-naphthyridm-3-yl)-1H-pyrazol-1-yl)ethoxy)azetidine-1-carboxylate used in Example 0740.
$^1$H-NMR($CDCl_3$)8:9.00(1H,s),8.93(1H,s),8.83(1H,s),8.30-8.25(2H,m),7.89(1H,brs),7.71(1H,s),4.57(2H,s),4.57-4.51(1H,m),3.99(3H,s),3.68(4H,d,J=6.0Hz),3.09-3.00(1H,m),1.42(6H,d,J=7.9Hz).
MSm/z(M+H):431.

<Example 0823>

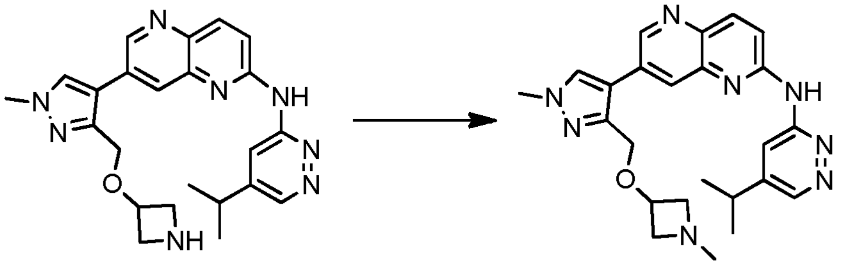

N-(5-isopropylpyridazin-3-yl)-7-(1-methyl-3-(((1-methylazetidin-3-yl)oxy)methyl)-1H-pyrazol-4-yl)-1,5-naphthyridine-2-amine was obtained as a white solid in the same manner as in Example 0741 except that 7-(3-((azetidin-3-yloxy)methyl)-1-methyl-1H-pyrazol-4-yl)-N-(5-isopropylpyridazin-3-yl)-1,5-naphthyridine-2-amine was used instead of the 7-(1-(2-(azetidin-3-yloxy)ethyl)-1H-pyrazol-4-yl)-N-(5-isopropylpyridazin-3-yl)-1,5-naphthyridine-2-amine used in Example 0741.
$^1$H-NMR($CDCl_3$)δ:9.73(1H,brs),8.96(1H,s),8.92(1H,d,J=2.0Hz),8.83(1H,d,J=2.0Hz),8.28(1H,d,J=9.2Hz), 8.26(1H,s),7.76(1H,d,J=9.2Hz),7.70(1H,s),4.56(2H,s),4.34-4.26(1H,m),4.00(3H,s),3.66(2H,dd,J=7.9,6.6Hz),3.09-3.00 (1H,m),2.94(2H,dd,J=8.3,6.6Hz),2.32(3H,s),1.42(6H,d,J=6.6Hz).
MSm/z(M+H):445.

<Example 0824>

<0824-1>

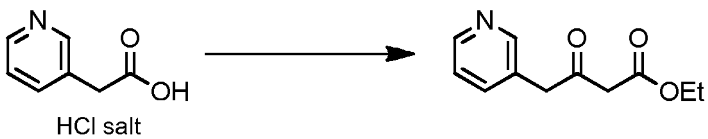


Methylene chloride (50 mL), triethylamine (2.09 mL), and 1,1'-carbonyldiimidazole (2.43 g) were added to 2-(pyridin-3-yl)acetic acid hydrochloride (1.73 g), followed by stirring at room temperature for 1.5 hours. A methylene chloride solution (50 mL) of Meldrum's acid (1.73 g) and pyridine (1.63 mL) was added to the reaction mixture under ice-cooling, followed by stirring at room temperature for 20 hours. After water was added to the reaction mixture, the resultant product was extracted two times with methylene chloride, and an organic layer thus obtained was washed with a saturated sodium chloride aqueous solution. The organic layer was dried over anhydrous sodium sulfate, and the solvent was distilled off under reduced pressure. Ethanol (100 mL) was added to the obtained residue, followed by heating under reflux for 4 days. The solvent of the reaction mixture was distilled off under reduced pressure, and the obtained residue was purified by silica gel column chromatography (hexane-ethyl acetate), thereby obtaining ethyl 3-oxo-4-(pyridin-3-yl)butanoate (480 mg) as yellow oily substance.
MSm/z(M+H):208.

<0824-2>

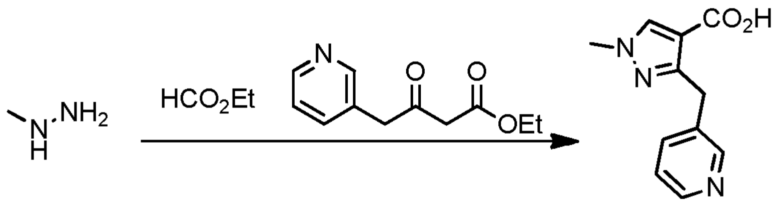


Ethyl formate (317 µL) was added to a solution of methylhydrazine (135 µL) in ethanol (1 mL), followed by heating under reflux for 3 hours under stirring. Ethyl 3-oxo-4-(pyridin-3-yl)butanoate (480 mg) was added to the reaction mixture, followed by heating under reflux for 3 hours under stirring. Furthermore, a 20% sodium ethoxide-ethanol solution (1 mL) was added thereto, followed by heating under reflux for 2 hours under stirring. A 5 mol/L sodium hydroxide aqueous solution (1 mL) was added to the reaction mixture, followed by stirring at room temperature for 1 hour. The solvent of the reaction mixture was distilled off under reduced pressure, a 4 mol/L hydrogen chloride/1,4-dioxane solution (2 mL) and ethanol (2 mL) were added to the obtained residue, and the solvent was distilled off under reduced pressure. Ethanol (2 mL) was added to the obtained residue, and the precipitated solid was collected by filtration, thereby obtaining 1-methyl-3-(pyridin-3-ylmethyl)-1H-pyrazole-4-carboxylic acid (220 mg) as a pale yellow solid.
MSm/z(M+H):218.

<0824-3>

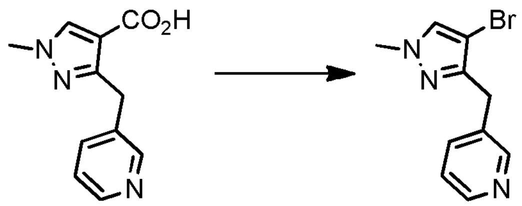

Sodium hydrogen carbonate (252 mg), N,N-dimethylformamide (3 mL), and N-bromosuccinimide (178 mg) were added to 1-methyl-3-(pyridin-3-ylmethyl)-1H-pyrazole-4-carboxylic acid (220 mg), followed by stirring at room temperature for 1.5 hours. After a 1 mol/L sodium hydroxide aqueous solution was added to the reaction mixture, the resultant product was extracted three times with ethyl acetate, an organic layer thus obtained was washed with a saturated sodium chloride aqueous solution, and dried over anhydrous sodium sulfate. The solvent was distilled off under reduced pressure, and the obtained residue was purified by silica gel column chromatography (hexane-ethyl acetate), thereby obtaining 3-((4-bromo-1-methyl-1H-pyrazol-3-yl)methyl)pyridine (100 mg) as brown oily substance.
MSm/z(M+H):252.

<0824-4>

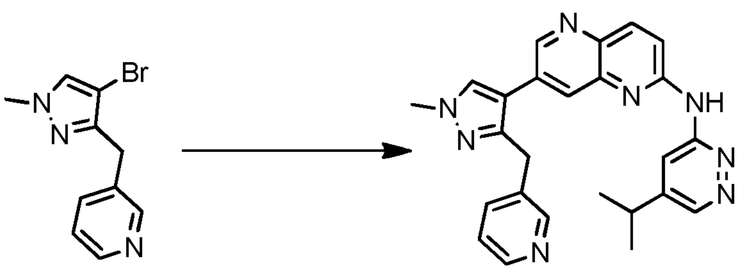

N-(5-isopropylpyridazin-3-yl)-7-(1-methyl-3-(pyridin-3-ylmethyl)-1H-pyrazol-4-yl)-1,5-naphthyridine-2-amine was obtained as a pale yellow solid in the same manner as in Example 0385-7 except that 7-bromo-N-(5-isopropylpyridazin-3-yl)-1,5-naphthyridine-2-amine was used instead of the 7-bromo-2-chloro-1,5-naphthyridine used in Example 0385-7, and 3-((4-bromo-1-methyl-1H-pyrazol-3-yl)methyl)pyridine was used instead of the 4-(3-(3-ethyl-4-iodo-1H-pyrazol-1-yl)propyl)morpholine used in Example 0385-7.
$^1$H-NMR($CDCl_3$)δ:8.81(2H,brs),8.73(1H,d,J=2.0Hz),8.50(1H,d,J=2.0Hz),8A2(1H,d,J=5.3Hz),8.24(1H,d,J =9.2Hz),7.94(1H,s),7.68-7.61(2H,m),7.53(1H,d,J=7.3Hz),7.19-7.14(1H,m),4.21(2H,s),4.00(3H,s),3.06-2.98(1H,m),1.3 7(6H,d,J=7.3Hz).
MSm/z(M+H):437.

<Example 0825>

<0825-1>

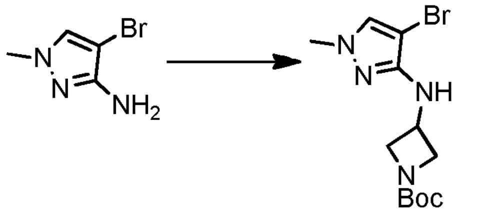

tert-Butyl 3 -((4-bromo-1 -methyl-1 H-pyrazol-3 -yl)amino)azetidine-1 -carboxylate was obtained as a yellow solid in the same manner as in Example 0814 except that tert-butyl 3-oxoazetidine-1-carboxylate was used instead of the 3-(4-(6-((5-isopropylpyridazin-3-yl)amino)-1,5-naphthyridin-3-yl)-1H-pyrazol-1-yl)-2-methoxypropanal hydrochloride used in Example 0814, and 4-bromo-1-methyl-1H-pyrazole-3-amine was used instead of the 3,3-difluoroazetidine hydrochloride used in Example 0814.
MSm/z(M+H):331.

<0825-2>

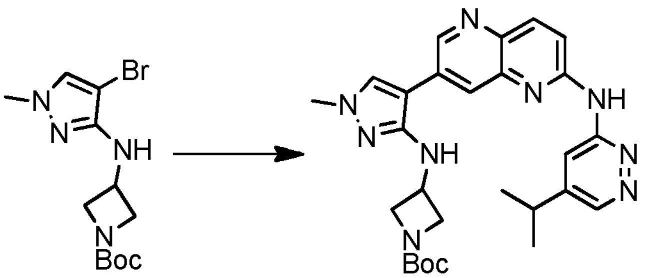

tert-Butyl 3-((4-(6-((5-isopropylpyridazin-3-yl)amino)-1,5-naphthyridin-3-yl)-1-methyl-1H-pyrazol-3-yl)amino)azetidine-1-carboxylate was obtained as a yellow solid in the same manner as in Example 0385-7 except that 7-bromo-N-(5-isopropylpyridazin-3-yl)-1,5-naphthyridine-2-amine was used instead of the 7-bromo-2-chloro-1,5-naphthyridine used in Example 0385-7, and tert-butyl 3-((4-bromo-1-methyl-1H-pyrazol-3-yl)amino)azetidine-1-carboxylate was used instead of the 4-(3-(3-ethyl-4-iodo-1H-pyrazol-1-yl)propyl)morpholine used in Example 0385-7.
$^1$H-NMR($CDCl_3$)δ:9.52(1H,brs),8.93(1H,brs),8.84(1H,s),8.77(1H,s),8.24(1H,d,J=8.2Hz),8.11(1H,s),7.69( 1H,d,J=8.2Hz),7 .52(1H,s),4.50-4.44(1H,m),4.32(2H,t,J=7.9Hz),4.19-4.10(1H,m),3.83(3H,s),3.82-3.76(2H,m),3.07-2.97(1H,m),1.43(9H ,s),1.41(6H,d,J=7.2Hz).
MSm/z(M+H):516.

<Example 0826>

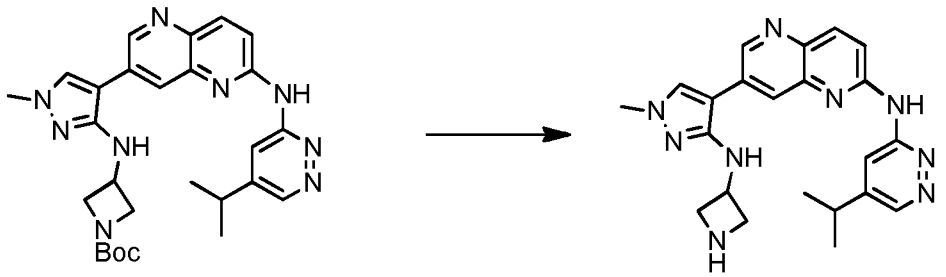

7-(3-(Azetidin-3-ylamino)-1-methyl-1H-pyrazol-4-yl)-N-(5-isopropylpyridazin-3-yl)-1,5-naphthyridine-2-amine was obtained as a white solid in the same manner as in Example 0740 except that tert-butyl 3-((4-(6-((5-isopropylpyridazin-3-yl)amino)-1,5-naphthyridin-3-yl)-1-methyl-1H-pyrazol-3-yl)amino)azetidine-1-carboxylate was used instead of the tert-butyl 3-(2-(4-(6-((5-isopropylpyridazin-3-yl)amino)-1,5-naphthyridin-3-yl)-1H-pyrazol-1-yl)ethoxy)azetidine-1-carboxylate used in Example 0740.
$^1$H-NMR($CDCl_3$)$\delta$:9.25(1H,brs),8.88(1H,s),8.86(1H,d,J=2.0Hz),8.74(1H,d,J=2.0Hz),8.24(1H,d,J=8.6Hz), 8.12(1H,d,J=2.0Hz),7.61(1H,d,J=8.6Hz),7.50(1H,s),4.69-4.60(1H,m),4.36(1H,d,J=8.6Hz),4.03(2H,t,J=8.3Hz),3.83(3H, s),3.58(2H,t,J=7.6Hz),3.04-2.95(1H,m),1.38(6H,d,J=6.6Hz).
MSm/z(M+H):416.

<Example 0827>

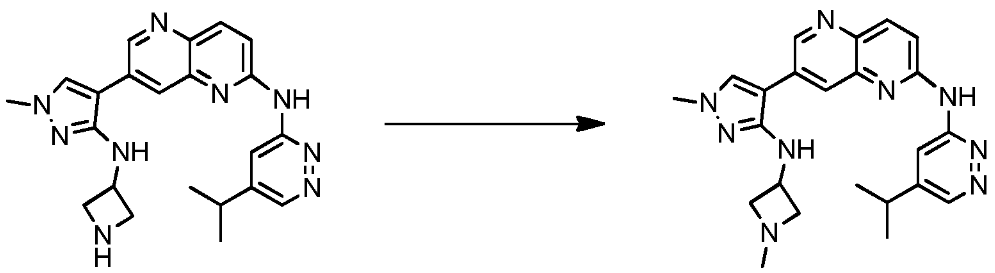

N-(5-isopropylpyridazin-3-yl)-7-(1-methyl-3-((1-methylazetidin-3-yl)amino)-1H-pyrazol-4-yl)-1,5-naphthyridine-2-amine was obtained as a white solid in the same manner as in Example 0741 except that 7-(3-(azetidin-3-ylamino)-1-methyl-1H-pyrazol-4-yl)-N-(5-isopropylpyridazin-3-yl)-1,5-naphthyridine-2-amine was used instead of the 7-(1-(2-(azetidin-3-yloxy)ethyl)-1H-pyrazol-4-yl)-N-(5-isopropylpyridazin-3-yl)-1,5-naphthyridine-2-amine used in Example 0741.
$^1$H-NMR($CDCl_3$)$\delta$:8.91(1H,s),8.85(1H,d,J=2.0Hz),8.81(1H,d,J=2.0Hz),8.24(1H,d,J=9.2Hz),8.11(1H,d,J= 2.0Hz),7.61(1H,brs),7.49(1H,s),4.38-4.31(1H,m),3.83(3H,s),3.80-3.75(2H,m),3.64(1H,t,J=4.3Hz),3.09-2.96(3H,m),2.3 6(3H,s),1.41(6H,d,J=7.3Hz).
MSm/z(M+H):430.

<Example 0828>

<0828-1>

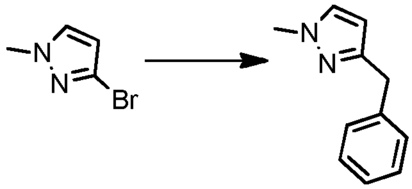

(1,3-Diisopropylimidazol-2-ylidene) (3-chloropyridyl)palladium(II) dichloride (34 mg), tetrahydrofuran (5 mL),

and a 0.9 mol/L benzyl bromide-tetrahydrofuran solution (1.7 mL) were added to 3-bromo-1-methyl-1H-pyrazole (80 mg), followed by stirring at room temperature for 4 hours. Water was added to the liquid reaction mixture, and the solvent was distilled off under reduced pressure. The obtained residue was purified by silica gel column chromatography (hexane-ethyl acetate), thereby obtaining 3-benzyl-1-methyl-1H-pyrazole (41 mg) as colorless oily substance.
MSm/z(M+H):173.

<0828-2>

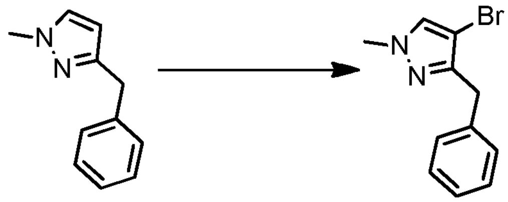

3-Benzyl-4-bromo-1-methyl-1H-pyrazole was obtained as colorless oily substance in the same manner as in Example 0734-2 except that 3-benzyl-1-methyl-1H-pyrazole was used instead of the 2-methyl-5-(1-methyl-1H-pyrazol-3-yl)pyridine used in Example 0734-2.
MSm/z(M+H):253.

<0828-3>

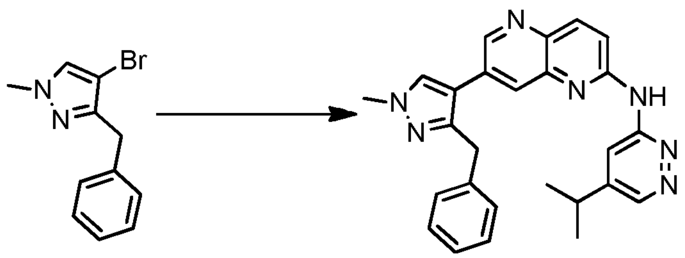

7-(3-Benzyl-1-methyl-1H-pyrazol-4-yl)-N-(5-isopropylpyridazin-3-yl)-1,5-naphthyridine-2-amine was obtained as a white solid in the same manner as in Example 0385-7 except that 7-bromo-N-(5-isopropylpyridazin-3-yl)-1,5-naphthyridine-2-amine was used instead of the 7-bromo-2-chloro-1,5-naphthyridine used in Example 0385-7, and 3-benzyl-4-bromo-1-methyl-1H-pyrazole was used instead of the 4-(3-(3-ethyl-4-iodo-1H-pyrazol-1-yl)propyl)morpholine used in Example 0385-7.
$^1$H-NMR($CDCl_3$)δ:9.01(1H,brs),8.80(2H,s),8.74(1H,d,J=2.0Hz),8.23(1H,d,J=8.9Hz),7.97(1H,s),7.66(1H,s ), 7.56(1H,d,J=8.9Hz),7.25-7.21(4H,m), 7.19-7.13(1H,m),4.21(2H,s),4.00(3H,s),3.04-2.95(1H,m),1.37(6H,d,J=6.9Hz).
MSm/z(M+H):436.

<Example 0829>

<0829-1>

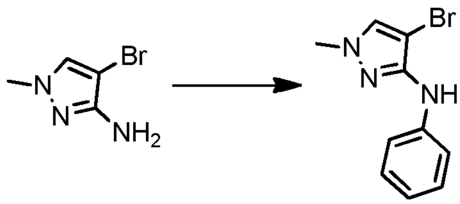

4-Bromo-1-methyl-N-phenyl-1H-pyrazole-3-amine was obtained as pale brown oily substance in the same manner as in Example 0729-2 except that iodobenzene was used instead of the 1-(4-(6-chloro-1,5-naphthyridin-3-yl)-1H-pyrazol-1-yl)-3-morpholinopropan-2-ol used in Example 0729-2, and 4-bromo-1-methyl-1H-pyrazole-3-amine was used instead of the 5-isopropylpyridazine-3-amine used in Example 0729-2.
MSm/z(M+H):252.

<0829-2>

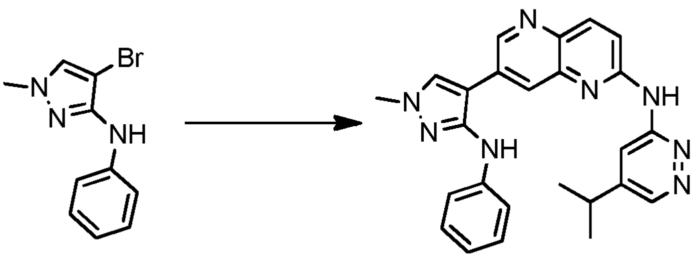

N-(5-isopropylpyridazin-3-yl)-7-(1-methyl-3-(phenylamino)-1H-pyrazol-4-yl)-1,5-naphthyridine-2-amine was obtained as a white solid in the same manner as in Example 0385-7 except that 7-bromo-N-(5-isopropylpyridazin-3-yl)-1,5-naphthyridine-2-amine was used instead of the 7-bromo-2-chloro-1,5-naphthyridine used in Example 0385-7, and 4-bromo-1-methyl-N-phenyl-1H-pyrazole-3-amine was used instead of the 4-(3-(3-ethyl-4-iodo-1H-pyrazol-1-yl)propyl)morpholine used in Example 0385-7.
$^1$H-NMR($CDCl_3$)δ:9.08(1H,brs),8.89(H,d,J=2.OHz),8.83(1H,s),8.79(1H,s),8.22(1H,d,J=9.2Hz),8.15(1H,s ),7.68(1H,s),7.56 (1H,d,J=9.2Hz),7.20(2H,d,J=7.3Hz),7.12(2H,d,J=7.3Hz),6.84(1H,dd,J=7.3,7.3Hz),5.94(1H,s),3.95(3H,s),3.04-2.94(1H,m),1.36(6H,d,J=7.2Hz).
MSm/z(M+H):437.

<Example 0830>

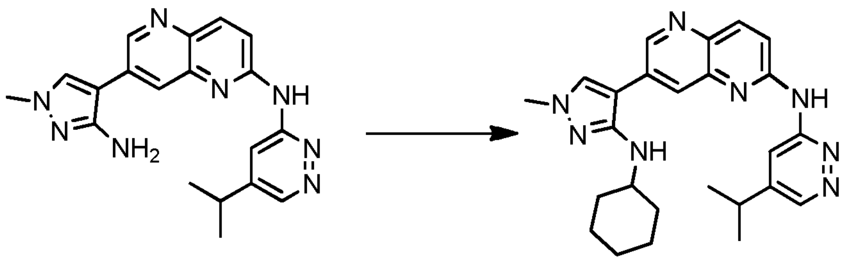

Cyclohexanone (2.1 μL), methanol (0.5 mL), acetic acid (50 μL), and 2-picoline borane (3.2 mg) were added to 7-(3-amino-1-methyl-1H-pyrazol-4-yl)-N-(5-isopropylpyridazin-3-yl)-1,5-naphthyridine-2-amine (3.6 mg), followed by stirring at room temperature for 1 hour. The solvent of the reaction mixture was distilled off under reduced pressure. The obtained residue was purified by silica gel column chromatography (hexane-ethyl acetate, NH silica), thereby obtaining 7-(3-(cyclohexylamino)-1-methyl-1H-pyrazol-4-yl)-N-(5-isopropylpyridazin-3-yl)-1,5-naphthyridine-2-amine (3.1 mg) as

a pale yellow solid.
$^1$H-NMR($CDCl_3$)δ:8.93(1H,s),8.85(1H,d,J=2.0Hz),8.80(1H,s),8.23(1H,d,J=9.2Hz),8.12(1H,s),7.52-7.46(2H,m),3.84(3H,s),3.68-3.62(1H,m),3.58-3.48(1H,m),3.07-2.98(1H,m),2.14(2H,d,J=13.2Hz),1.78-1.70(4H,m),1.41(6H,d,J=6.6Hz),1.27-1.15(4H,m).
MSm/z(M+H):443.

<Example 0831>

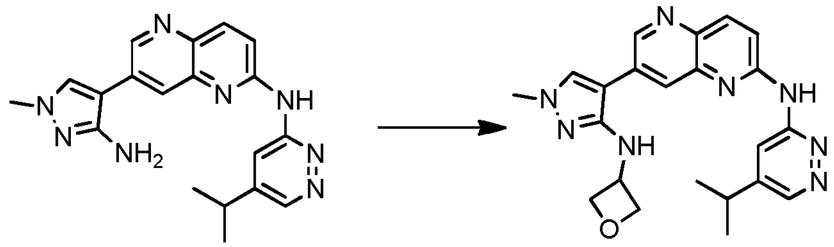

N-(5-isopropylpyridazin-3-yl)-7-(1-methyl-3-(oxetan-3-ylamino)-1H-pyrazol-4-yl)-1,5-naphthyridine-2-amine was obtained as a pale yellow solid in the same manner as in Example 0830 except that oxetan-3-one was used instead of the cyclohexanone used in Example 0830.
$^1$H-NMR($CDCl_3$)δ:8.87(1H,d,J=2.0Hz),8.81(2H,brs),8.67(1H,brs),8.25(1H,d,J=8.6Hz),8.13(1H,s),7.54-7.48(2H,m),5.03(2H,t,J=6.9Hz),4.93-4.83(1H,m),4.57(2H,t,J=6.3Hz),4.24(1H,d,J=7.9Hz),3.82(3H,s),3.08-2.98(1H,m),1.40(6H,d,J=7.3Hz).
MSm/z(M+H):417.

<Example 0832>

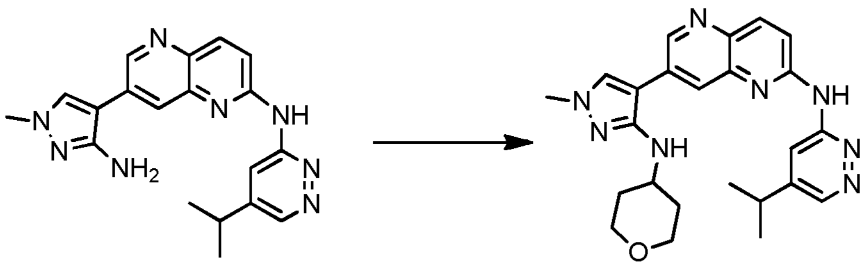

N-(5-isopropylpyridazin-3-yl)-7-(1-methyl-3-((tetrahydro-2H-pyran-4-yl)amino)-1H-pyrazol-4-yl)-1,5-naphthyridine-2-amine was obtained as a pale yellow solid in the same manner as in Example 0830 except that dihydro-2H-pyran-4(3H)-one was used instead of the cyclohexanone used in Example 0830.
$^1$H-NMR($CDCl_3$)δ:9.26(1H,brs),8.94(1H,brs),8.85(1H,d,J=2.0Hz),8.81(1H,brs),8.24(1H,d,J=9.2Hz),8.11( 1H,d,J=2.0Hz),7.61(1H,d,J=9.2Hz),7.49(1H,s),4.03-3.96(2H,m),3.84(3H,s),3.81-3.63(2H,m),3.60-3.50(2H,m),3.08-2.99(1H,m),2.14(2H,d,J=11.2Hz),1.54-1.46(2H,m),1.41(6H,d,J=7.3Hz).
MSm/z(M+H):445.

<Example 0833>

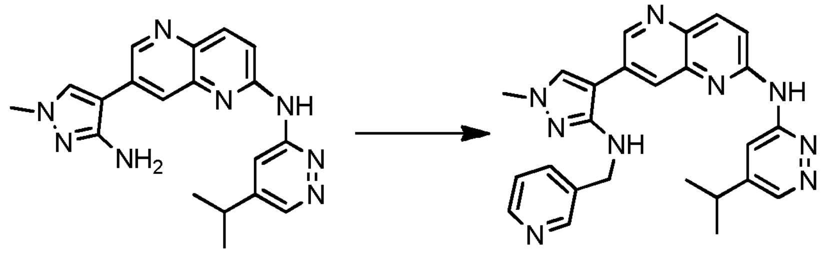

N-(5-isopropylpyridazin-3-yl)-7-(1-methyl-3-((pyridin-3-ylmethyl)amino)-1H-pyrazol-4-yl)-1,5-naphthyridine-2-amine was obtained as a pale yellow solid in the same manner as in Example 0830 except that nicotinaldehyde was used instead of the cyclohexanone used in Example 0830.
$^1$H-NMR($CDCl_3$)δ:9.36(1H,brs),8.87(1H,d,J=2.0Hz),8.77(2H,brs),8.67(1H,d,J=2.0Hz),8.52(1H,d,J=4.6Hz),8.23(1H,d,J=9.2Hz),8.08(1H,d,J=2.0Hz),7.78(1H,d,J=7.9Hz),7.66(1H,d,J=9.2Hz),7.53(1H,s),7.29-7.24(1H,m),4.53(2H,d,J=5.6Hz),4.12(1H,t,J=5.6Hz),3.85(3H,s),3.05-2.96(1H,m),1.37(6H,d,J=6.6Hz).
MSm/z(M+H):452.

<Example 0834>

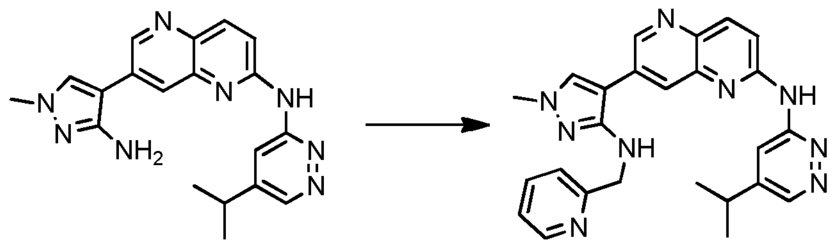

N-(5-isopropylpyridazin-3-yl)-7-(1-methyl-3-((pyridin-2-ylmethyl)amino)-1H-pyrazol-4-yl)-1,5-naphthyridine-2-amine was obtained as a yellow solid in the same manner as in Example 0830 except that picolinaldehyde was used instead of the cyclohexanone used in Example 0830.
$^1$H-NMR($CDCl_3$)δ:9.52(1H,brs),8.92(2H,brs),8.78(1H,brs),8.54(1H,d,J=4.6Hz),8.25(1H,d,J=8.2Hz),8.19( 1H,s),7.70-7.62(2H,m),7.53(1H,s),7.38(1H,d,J=8.2Hz),7.17(1H,dd,J=5.9,4.6Hz),5.19(1H,brs),4.65(2H,d,J=4.0Hz ),3.86(3H,s),3.04-2.94(1H,m),1.33(6H,d,J=6.6Hz).
MSm/z(M+H):452.

<Example 0835>

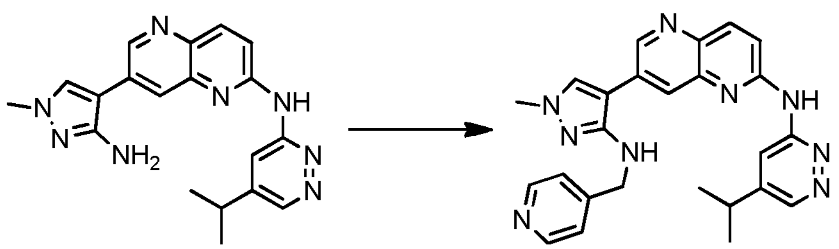

N-(5-isopropylpyridazin-3-yl)-7-(1-methyl-3-((pyridin-4-ylmethyl)amino)-1H-pyrazol-4-yl)-1,5-naphthyridine-2-amine was obtained as a yellow solid in the same manner as in Example 0830 except that isonicotinaldehyde was used instead of the cyclohexanone used in Example 0830.
$^1$H-NMR($CDCl_3$)δ:8.89(1H,d,J=1.3Hz),8.77(2H,brs),8.55(2H,brs),8.25(1H,d,J=8.6Hz),8.12(1H,s),7.74(1H,d,J=8.6Hz),7.53(1H,s),7.36(2H,d,J=4.6Hz),4.55(2H,d,J=6.1Hz),4.23(1H,t,J=6.1Hz),3.83(3H,s),3.06

-2.97(1H,m),1.38(6H,d,J=6.9Hz).
MSm/z(M+H):452.

<Example 0836>

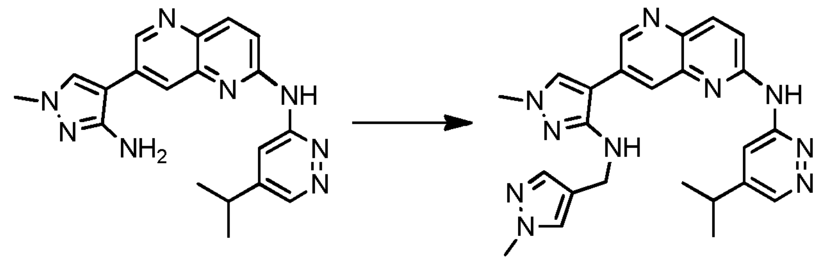

N-(5-isopropylpyridazin-3-yl)-7-(1-methyl-3-(((1-methyl-1H-pyrazol-4-yl)methyl)amino)-1H-pyrazol-4-yl)-1,5-naphthyridine-2-amine was obtained as a yellow solid in the same manner as in Example 0830 except that 1-methyl-1H-pyrazole-4-carbaldehyde was used instead of the cyclohexanone used in Example 0830.
$^1$H-NMR($CDCl_3$)δ:9.12(1H,d,J=1.3Hz),9.00(1H,brs),8.86(1H,s),8.81(1H,s),8.29(1H,s),8.24(1H,d,J=8.6Hz ),7.59(1H,d,J=8.6Hz),7.58(1H,s),7.31(1H,s),7.21(1H,s),4.04(3H,s),3.86(2H,s),3.81(3H,s),3.09-3.00(1H,m),1.40(6H,d,J=7.3Hz).
MSm/z(M+H):455.

<Examples 0837 and 0838>

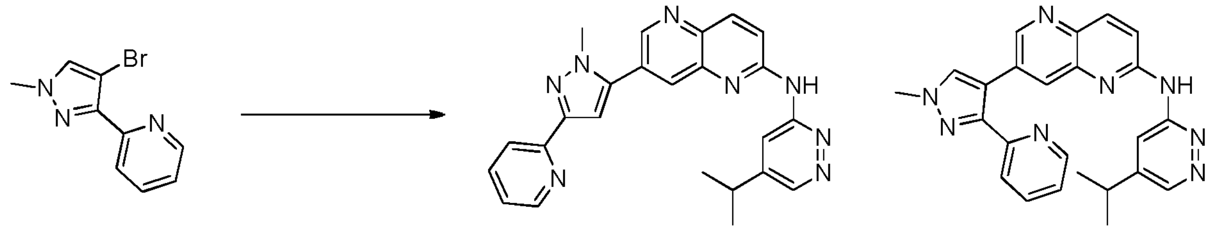

2.6 mol/L n-butyllithium (0.24 mL) was added to a mixture of 2-(4-bromo-1-methyl-1H-pyrazol-3-yl)pyridine (100 mg) and tetrahydrofuran (2 mL) at -78°C, followed by stirring at the same temperature for 1 hour. 2-Isopropoxy-4,4,5,5-tetramethyl-1,3,2-dioxaborolane (0.17 mL) was added thereto, followed by stirring at the same temperature for 1 hour. After an ammonium chloride aqueous solution and ethyl acetate were added to the reaction mixture, an organic layer was collected therefrom by separation, washed sequentially with water and a saturated sodium chloride aqueous solution, and dried over anhydrous sodium sulfate, and the solvent was distilled off under reduced pressure, thereby obtaining brown oily substance (128 mg).
A mixture of the brown oily substance (33 mg), 7-bromo-N-(5-isopropylpyridazin-3-yl)-1,5-naphthyridine-2-amine (20 mg), sodium carbonate (18 mg), bis(di-tert-butyl (4-dimethylaminophenyl)phosphine)dichloropalladium(II) (4 mg), water (0.1 mL), and 1,4-dioxane (1 mL) was stirred at 100°C for 7 hours. The reaction mixture was cooled to room temperature, the insolubles were filtered off, and the solvent was distilled off under reduced pressure. The obtained residue was purified by silica gel column chromatography (methanol-ethyl acetate), thereby obtaining N-(5-isopropylpyridazin-3-yl)-7-(1-methyl-3-(pyridin-2-yl)-1H-pyrazol-5-yl)-1,5-naphthyridine-2-amine (1 mg) as a yellow solid and N-(5-isopropylpyridazin-3-yl)-7-(1-methyl-3-(pyridin-2-yl)-1H-pyrazol-4-yl)-1,5-naphthyridine-2-amine (7 mg) as a yellow solid.

<Example 0837>

N-(5-isopropylpyridazin-3-yl)-7-(1-methyl-3-(pyridin-2-yl)-1H-pyrazol-5-yl)-1,5-naphthyridine-2-amine
$^1$H-

NMR($CDCl_3$)δ:9.80(1H,s),9.00-8.96(1H,m),8.92(1H,d,J=2.0Hz),8.85(1H,d,J=2.0Hz),8.70-8.65(1H,m),8.33(1H,d,J=9.2 Hz),8.21(1H,d,J=2.0Hz),8.01(1H,d,J=7.9Hz),7.82(1H,d,J=9.2Hz),7.80-7.75(1H,m),7.26-7.22(1H,m),7.17(1H,s),4.09(3 H,s),3.10-3.01(1H,m),1.42(6H,d,J=7.3Hz).
MSm/z(M+H):423.

<Example 0838>

N-(5-isopropylpyridazin-3-yl)-7-(1-methyl-3-(pyridm-2-yl)-1H-pyrazol-4-yl)-1,5-naphthyridine-2-amine
$^1$H-
NMR($CDCl_3$)δ:9.94(1H,s),8.97-8.94(1H,m),8.82-8.79(1H,m),8.76(1H,d,J=2.0Hz),8.61-8.56(1H,m),8.25(1H,d,J=9.2Hz),8.08(1H,d,J=2.0Hz),7.76(1H,d,J=9.2Hz),7.72-7.65(1H,m),7.70(1H,s),7.67-7.65(1H,m),7.24-7.18(1H,m),4.08(3H,s),3.04-2.95(1H,m),1.36(6H,d,J=6.6Hz).
MSm/z(M+H):423.

<Example 0839>

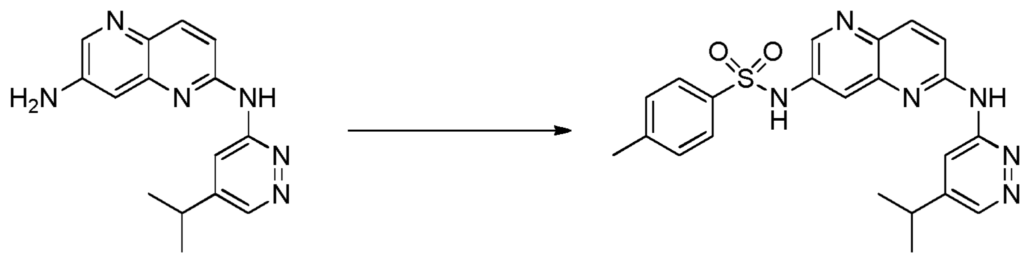

A mixture of N$^2$-(5-isopropylpyridazin-3-yl)-1,5-naphthyridine-2,7-diamine (5 mg), paratoluenesulphonyl chloride (7 mg), pyridine (4 μL), and dichloromethane (0.5 mL) was stirred at room temperature for 6 hours. The solvent of the reaction mixture was distilled off under reduced pressure. The obtained residue was purified by silica gel column chromatography (methanol-ethyl acetate), thereby obtaining N-(6-((5-isopropylpyridazin-3-yl)amino)-1,5-naphthyridin-3-yl)-4-methylbenzene sulfonamide (8 mg) as a yellow solid.
$^1$H-
NMR($CDCl_3$)δ:9.28(1H,s),8.94-8.90(1H,m),8.82(1H,d,J=2.0Hz),8.53(1H,d,J=2.0Hz),8.15(1H,d,J=9.2Hz),7.97(1H,d,J=2.0Hz),7.77(2H,d,J=7.9Hz),7.51(1H,d,J=8.6Hz),7.26-7.23(1H,m),7.25(2H,d,J=7.9Hz),3.12-3.03(1H,m),2.37(3H,s),1.44(6H,d,J=6.6Hz).
MSm/z(M+H):435.

<Example 0840>

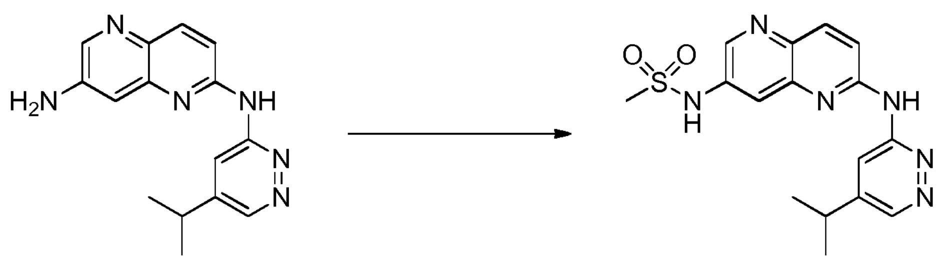

N-(6-((5-isopropylpyridazin-3-yl)amino)-1,5-naphthyridin-3-yl)methane sulfonamide was obtained as a yellow solid in the same manner as in Example 0839 except that methanesulfonyl chloride was used instead of the paratoluenesulphonyl chloride used in Example 0839.
$^1$H-

NMR($CDCl_3$)δ:8.97(1H,s),8.87-8.84(1H,m),8.85-8.81(1H,m),8.67(1H,d,J=2.0Hz),8.23(1H,d,J=8.6Hz),8.08(1H,d,J=2.0 Hz),7.53(1H,d,J=8.6Hz),7.30-7.25(1H,m),3.16(3H,s),3.11-3.02(1H,m),1.41(6H,d,J=7.3Hz).
MSm/z(M+H):359.

<Example 0841>

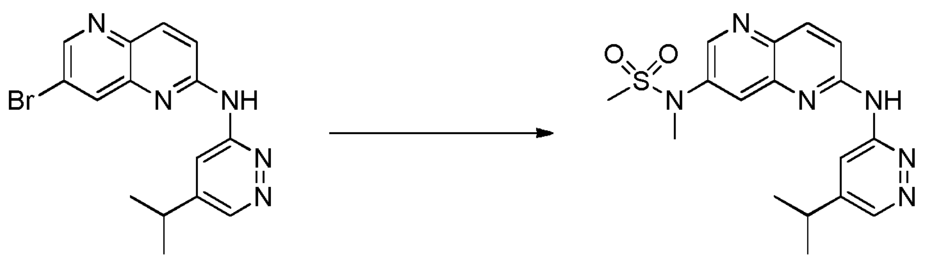

A mixture of 7-bromo-N-(5-isopropylpyridazin-3-yl)-1,5-naphthyridine-2-amine (10 mg), N-methylmethane sulfonamide (68 μL), tris(dibenzylideneacetone)dipalladium(0) (3 mg), 2-di-tert-butylphosphino-3,4,5,6-tetramethyl-2',4',6'-triisopropyl-1,1'-biphenyl (4 mg), potassium carbonate (20 mg), and tert-amyl alcohol (0.5 mL) was stirred at 100°C for 5 hours. The reaction mixture was cooled to room temperature, the insolubles were filtered off, and the solvent was distilled off under reduced pressure. The obtained residue was purified by silica gel column chromatography (methanol-ethyl acetate), thereby obtaining N-(6-((5-isopropylpyridazin-3-yl)amino)-1,5-naphthyridin-3-yl)-N-methylmethane sulfonamide (2 mg) as a yellow solid.
$^1H$-NMR($CDCl_3$)δ:9.82(1H,s),8.91-8.87(1H,m),8.86-8.83(1H,m),8.84-8.82(1H,m),8.27(1H,d,J=8.6Hz),8.07-8.04(1H,m),7.80(1H,d,J=8.6Hz),3.50(3H,s),3.10-3.02(1H,m),2.98(3H,s), 1.42(6H,d,J=6.6Hz).
MSm/z(M+H):373.

<Example 0842>

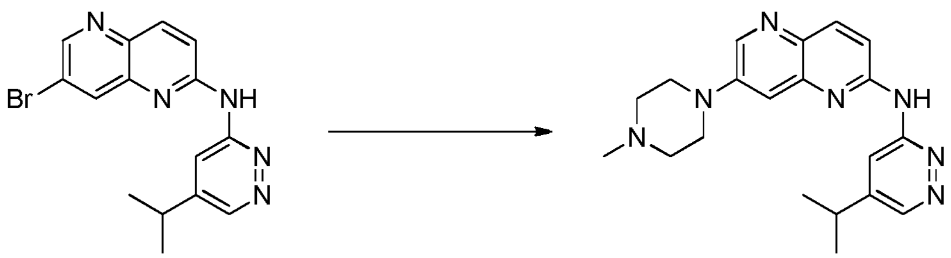

N-(5-isopropylpyridazin-3-yl)-7-(4-methylpiperazin-1-yl)-1,5-naphthyridine-2-amine was obtained as a yellow solid in the same manner as in Example 0846 except that 1-methylpiperazine was used instead of the pyrrolidine used in Example 0846, and 2-(dicyclohexylphosphino)-3,6-dimethoxy-2',4',6'-triisopropyl-1,1'-biphenyl was used instead of the 2-dicyclohexylphosphino-2',6'-diisopropoxybiphenyl used in Example 0846.
$^1H$-NMR($CDCl_3$)δ:9.18(1H,s),8.82-8.78(2H,m),8.64(1H,d,J=2.6Hz),8.14(1H,d,J=8.6Hz),7.45(1H,d,J=8.6Hz),7.32(1H,d,J=2.6Hz),3.44-3.39(4H,m),3.09-2.98(1H,m),2.68-2.62(4H,m),2.40(3H,s),1.40(6H,d,J=7.3Hz).
MSm/z(M+H):364.

<Example 0843>

<0843-1>

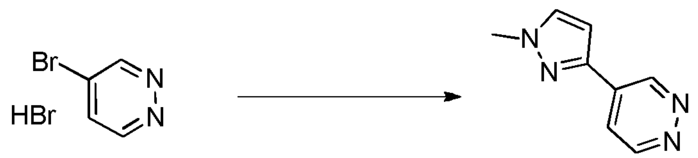

A mixture of 4-bromopyridazine hydrobromate (100 mg), bis(pinacolato)diboron (117 mg), potassium acetate (165 mg), (tris(dibenzylideneacetone)dipalladium(0) (19 mg), 2-dicyclohexylphosphino-2',4',6'-triisopropylbiphenyl (40 mg), and 1,4-dioxane (0.5 mL) was stirred at 110°C for 0.5 hours. 3-Bromo-1-methyl-1H-pyrazole (74 mg), potassium phosphate (713 mg), water (0.2 mL), and 1,4-dioxane (0.5 mL) were added thereto, followed by stirring at 110°C for 0.5 hours. The reaction mixture was cooled to room temperature, the solvent was distilled off under reduced pressure, and the obtained residue was purified by silica gel column chromatography (methanol-ethyl acetate), thereby obtaining 4-(1-methyl-1H-pyrazol-3-yl)pyridazine (15 mg) as a yellow solid.
MSm/z(M+H):161.

<0843-2>

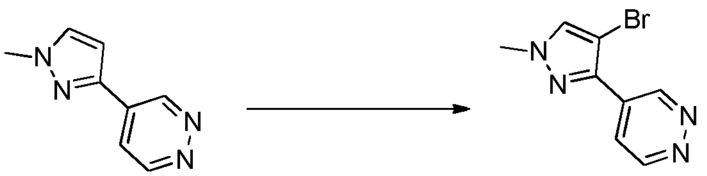

A mixture of 4-(1-methyl-1H-pyrazol-3-yl)pyridazine (15 mg), N-bromosuccinimide (18 mg), and acetonitrile (1 mL) was stirred at room temperature for 5 hours. The solvent of the reaction mixture was distilled off under reduced pressure. The obtained residue was purified by silica gel column chromatography (methanol-ethyl acetate), thereby obtaining 4-(4-bromo-1-methyl-1H-pyrazol-3-yl)pyridazine (11 mg) as a yellow solid.
MSm/z(M+H):239.

<0843-3>

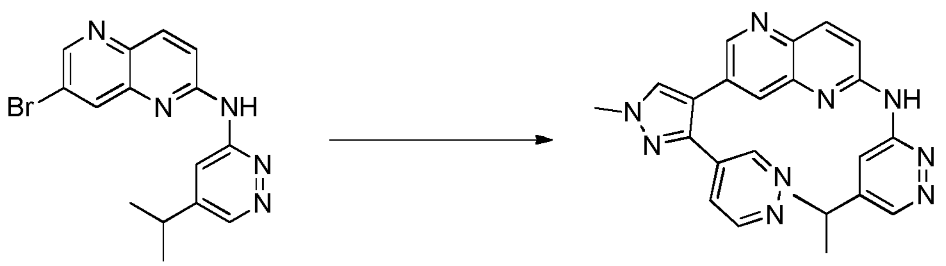

A mixture of 7-bromo-N-(5-isopropylpyridazin-3-yl)-1,5-naphthyridine-2-amine (17 mg), bis(pinacolato)diboron (14 mg), potassium acetate (9 mg), (tris(dibenzylideneacetone) dipalladium(0) (4 mg), 2-dicyclohexylphosphino-2',4',6'-triisopropylbiphenyl (9 mg), and 1,4-dioxane (0.4 mL) was stirred at 100°C for 1 hour. 4-(4-Bromo-1-methyl-1H-pyrazol-3-yl)pyridazine (13 mg), potassium phosphate (41 mg), water (0.2 mL), and 1,4-dioxane (0.4 mL) were added thereto, followed by stirring at 100°C for 2 hours. The reaction mixture was cooled to room temperature, the solvent was distilled off under reduced pressure, and the obtained residue was purified by silica gel column chromatography (methanol-ethyl acetate), thereby obtaining N-(5-isopropylpyridazin-3-yl)-7-(1-methyl-3-(pyridazin-4-yl)-1H-pyrazol-4-yl)-1,5-naphthyridine-2-amine (15 mg) as a yellow solid.
$^1$H-NMR($CDCl_3$)δ:9.45-9.42(1H,m),9.10(1H,d,J=5.3Hz),8.95(1H,s),8.82-8.78(1H,m),8.78-8.74(1H,m),8.69(1H,d,J=2.0Hz),8.29(1H,d,J=9.2Hz),7.96(1H,d,J=2.0Hz),7.70(1H,s),7.63(1H,d,J=9.2 Hz),7.56(1H,dd,J=5.3,2.0Hz),4.10(3H,s),3.03-2.94(1H,m),1.33(6H,d,J=6.6Hz).

MSm/z(M+H):424.

<Example 0844>

<0844-1>

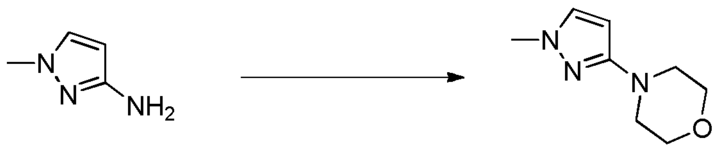

A mixture of 1-methyl-1H-pyrazole-3-amine (1 g), bis(2-bromoethyl)ether (1.2 g), potassium carbonate (4.2 g), and N,N-dimethylformamide (20 mL) was stirred at 150°C for 1 hour. The solvent of the reaction mixture was distilled off under reduced pressure, and ethyl acetate and water were added thereto. An organic layer was collected therefrom by separation, and dried over anhydrous sodium sulfate, and the solvent was distilled off under reduced pressure. The obtained residue was purified by silica gel column chromatography (methanol-ethyl acetate), thereby obtaining 4-(1-methyl-1H-pyrazol-3-yl)morpholine (249 mg).
MSm/z(M+H):168.

<0844-2>

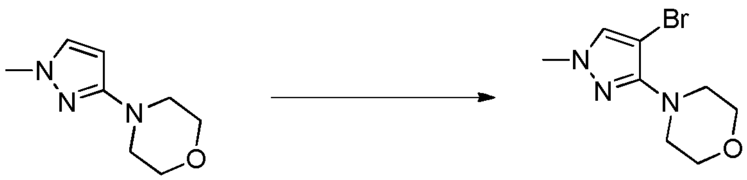

4-(4-Bromo-1-methyl-1H-pyrazol-3-yl)morpholine was obtained in the same manner as in Example 0843-2 except that 4-(1-methyl-1H-pyrazol-3-yl)morpholine was used instead of the 4-(1-methyl-1H-pyrazol-3-yl)pyridazine used in Example 0843-2.
MSm/z(M+H):246.

<0844-3>

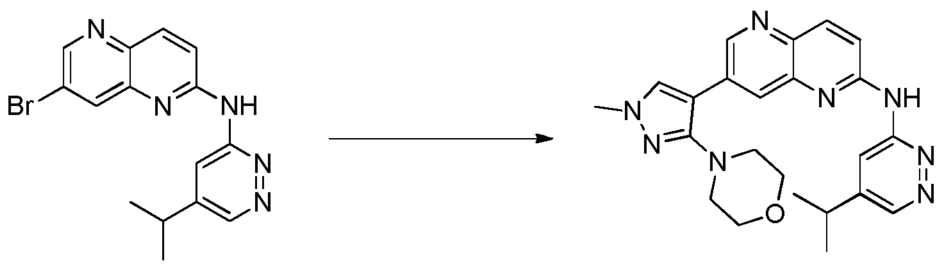

N-(5-isopropylpyridazin-3-yl)-7-(1-methyl-3-morpholino-1H-pyrazol-4-yl)-1,5-naphthyridine-2-amine was obtained in the same manner as in Example 0843-3 except that 4-(4-bromo-1-methyl-1H-pyrazol-3-yl)morpholine was used instead of the 4-(4-bromo-1-methyl-1H-pyrazol-3-yl)pyridazine used in Example 0843-3.
$^1$H-NMR($CDCl_3$)$\delta$:9.21(1H,s),9.02-9.01(1H,m),9.01-8.99(1H,m),8.82-8.81(1H,m),8.38-8.36(1H,m),8.23(1H,d,J=9.2Hz),7.60(1H,s),7.56(1H,d,J=9.2Hz),3.88-3.84(4H,m),3.88(3H,s),3.14-3.10(4H,m),3.09-2.99(1H,m),1.44(6H,d,J=6.6Hz).

MSm/z(M+H):431.

<Example 0845>

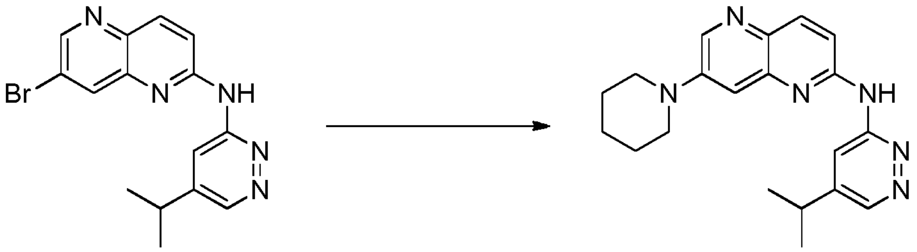

N-(5-isopropylpyridazin-3-yl)-7-(piperidin-1-yl)-1,5-naphthyridine-2-amine was obtained as a yellow solid in the same manner as in Example 0846 except that piperidine was used instead of the pyrrolidine used in Example 0846, and 2-(dicyclohexylphosphino)-3,6-dimethoxy-2',4',6'-triisopropyl-1,1'-biphenyl was used instead of the 2-dicyclohexylphosphino-2',6'-diisopropoxybiphenyl used in Example 0846.
$^1$H-NMR($CDCl_3$)δ:8.79-8.75(2H,m),8.69(1H,s),8.64(1H,d,J=2.6Hz),8.12(1H,d,J=8.6Hz), 7.34-7.30(1H,m),7.32-7.26(1H,m),3.40-3.34(4H,m),3.07-2.98(1H,m),1.83-1.75(4H,m),1.71-1.64(2H,m), 1.39(6H,d,J=7.3Hz).
MSm/z(M+H):349.

<Example 0846>

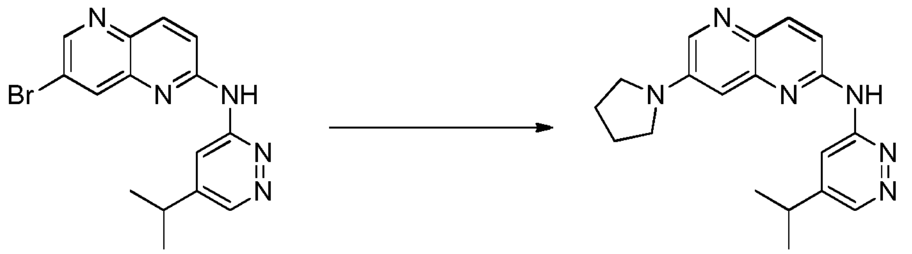

A mixture of 7-bromo-N-(5-isopropylpyridazin-3-yl)-1,5-naphthyridine-2-amine (15 mg), pyrrolidine (11 μL), tris(dibenzylideneacetone)dipalladium(0) (4 mg), 2-dicyclohexylphosphino-2',6'-diisopropoxybiphenyl (4 mg), sodium tert-butoxide (13 mg), and tert-amyl alcohol (0.5 mL) was stirred at 130°C for 30 minutes using a microwave reaction apparatus. The reaction mixture was cooled to room temperature, the insolubles were filtered off, and the solvent was distilled off under reduced pressure. The obtained residue was purified by silica gel column chromatography (methanol-ethyl acetate), thereby obtaining N-(5-isopropylpyridazin-3-yl)-7-(pyrrolidin-1-yl)-1,5-naphthyridine-2-amine (12 mg) as a yellow solid.
$^1$H-NMR($CDCl_3$)δ:8.79-8.75(2H,m),8.68(1H,s),8.37(1H,d,J=2.6Hz),8.10(1H,d,J=8.6Hz),7.22(1H,d,J=8.6Hz),6.96(1H,d,J=2.6 Hz),3.52-3.45(4H,m),3.06-2.97(1H,m),2.14-2.09(4H,m),1.39(6H,d,J=7.3Hz).
MSm/z(M+H):335.

<Example 0847>

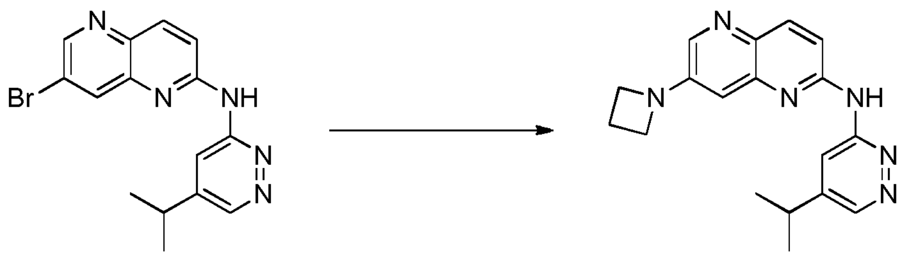

7-(Azetidin-1-yl)-N-(5-isopropylpyridazin-3-yl)-1,5-naphthyridine-2-amine was obtained as a yellow solid in the same manner as in Example 0846 except that azetidine was used instead of the pyrrolidine used in Example 0846.
$^1$H-NMR($CDCl_3$)δ:8.79-8.76(2H,m),8.75(1H,s),8.18-8.14(1H,m),8.11(1H,d,J=8.6Hz),7.30-7.26(1H,m),6.88-6.84(1H,m),4.13-4.09(4H,m),3.06-2.98(1H,m),2.56-2.48(2H,m),1.39(6H,d,J=7.3Hz).
MSm/z(M+H):321.

<Example 0848>

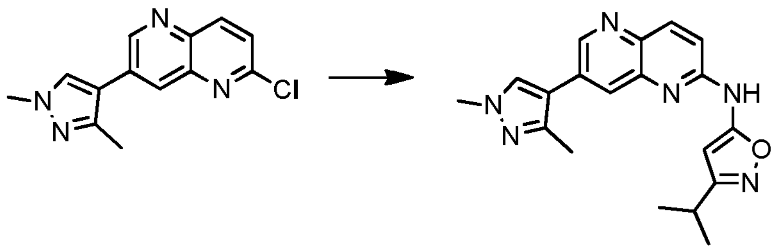

A mixture of 2-chloro-7-(1,3-dimethyl-1H-pyrazol-4-yl)-1,5-naphthyridine (20 mg), 3-isopropylisoxazole-5-amine (10 mg), tris(dibenzylideneacetone)dipalladium(0) (4 mg), 2-(dicyclohexylphosphino)-3,6-dimethoxy-2',4',6'-triisopropyl-1,1'-biphenyl (4 mg), sodium tert-butoxide (11 mg), and 1,4-dioxane (0.6 mL) was stirred at 140°C for 30 minutes using a microwave reaction apparatus. The reaction mixture was cooled to room temperature, the insolubles were filtered off, and the solvent was distilled off under reduced pressure. The obtained residue was purified by silica gel column chromatography (ethyl acetate-methanol, chloroform-methanol, NH silica), thereby obtaining N-(7-(1,3-dimethyl-1H-pyrazol-4-yl)-1,5-naphthyridin-2-yl)-3-isopropylisoxazole-5-amine (0.8 mg) as a pale yellow solid.
$^1$H-NMR(DMSO-$d_6$)δ:8.88(1H,d,J=2.0Hz),8.22(3H,d,J=9.9Hz),7.31(1H,d,J=8.6Hz),6.70(1H,s),6.55(1H,s),3.85(3H,s),3.03-2.94(1H,m),2.43(3H,s), 1.29(6H,d,J=6.6Hz).
MSm/z(M+H):349.

<Examples 0849 and 0850>

<0849-1 and 0850-1>

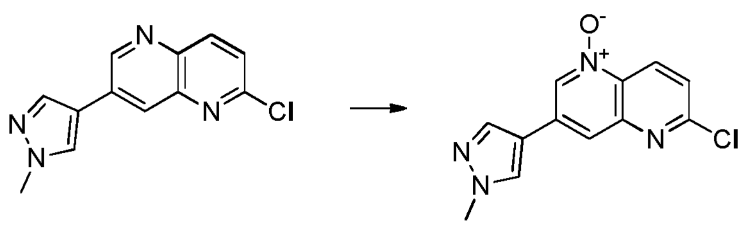

65% meta-chloroperoxybenzoic acid (672 mg) was added to a mixture of 2-chloro-7-(1-methyl-1H-pyrazol-4-yl)-1,5-naphthyridine (310 mg) and dichloromethane (10 mL) at a temperature of from 0°C to 5°C in a nitrogen atmosphere,

followed by stirring at room temperature overnight. A sodium sulfite aqueous solution and a saturated sodium hydrogen carbonate aqueous solution were added to the reaction mixture, and the resultant product was extracted two times with chloroform. An organic layer obtained therefrom was dried over anhydrous sodium sulfate, and the solvent was distilled off under reduced pressure. The obtained residue was purified by silica gel column chromatography (hexane-ethyl acetate, chloroform-methanol), thereby obtaining 6-chloro-3-(1-methyl-1H-pyrazol-4-yl)-1,5-naphthyridine 1-oxide (341 mg) as a pale yellow solid.
MSm/z(M+H):261.

<0849-2 and 0850-2>

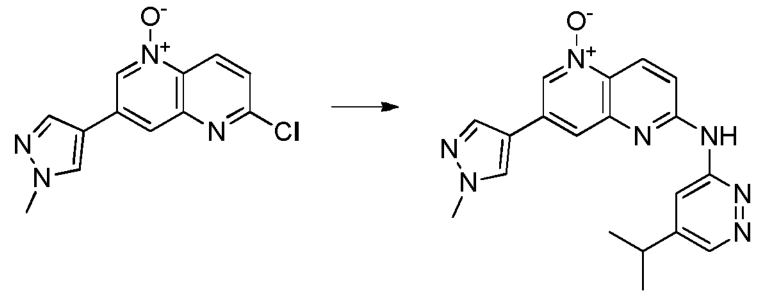

A mixture of 6-chloro-3-(1-methyl-1H-pyrazol-4-yl)-1,5-naphthyridine 1-oxide (270 mg), 2-(dicyclohexylphosphino)-3,6-dimethoxy-2',4',6'-triisopropyl-1,1'-biphenyl (56 mg), tris(dibenzylideneacetone)dipalladium(0) (48 mg), cesium carbonate (1.01 g), and 1,4-dioxane (10 mL) was stirred at 100°C for 8 hours in a nitrogen atmosphere in a sealed tube. The reaction mixture was cooled to room temperature, and a saturated ammonium chloride aqueous solution and chloroform were added thereto. An organic layer was collected therefrom by separation, and dried over anhydrous sodium sulfate, and the solvent was distilled off under reduced pressure. The obtained residue was purified by silica gel column chromatography (hexane-ethyl acetate, chloroform-methanol), thereby obtaining 6-((5-isopropylpyridazin-3-yl)amino)-3-(1-methyl-1H-pyrazol-4-yl)-1 ,5-naphthyridine 1-oxide (103 mg) as a brown solid.
MSm/z(M+H):362.

<0849-3 and 0850-3>

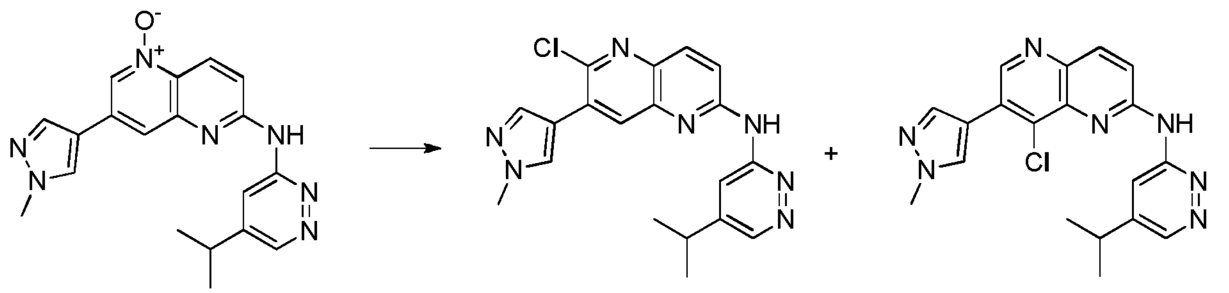

A solution of 6-((5-isopropylpyridazin-3-yl)amino)-3-(1-methyl-1H-pyrazol-4-yl)-1,5-naphthyridine 1-oxide (103 mg) in phosphorus oxychloride (4 g) was stirred at 80°C for 2 hours. The reaction mixture was cooled to room temperature, and added dropwise to water. The obtained solution was neutralized with sodium hydrogen carbonate, and the resultant product was extracted with chloroform. An organic layer thus obtained was dried over anhydrous sodium sulfate, and the solvent was distilled off under reduced pressure. The obtained residue was purified by silica gel column chromatography (hexane-ethyl acetate, chloroform-methanol), thereby obtaining a mixture of 6-chloro-N-(5-isopropylpyridazin-3-yl)-7-(1-methyl-1H-pyrazol-4-yl)-1,5-naphthyridine-2-amine and 8-chloro-N-(5-isopropylpyridazin-3-yl)-7-(1-methyl-1H-pyrazol-4-yl)-1,5-naphthyridine-2-amine as a yellow solid.
MSm/z(M+H):380.

<0849-4 and 0850-4>

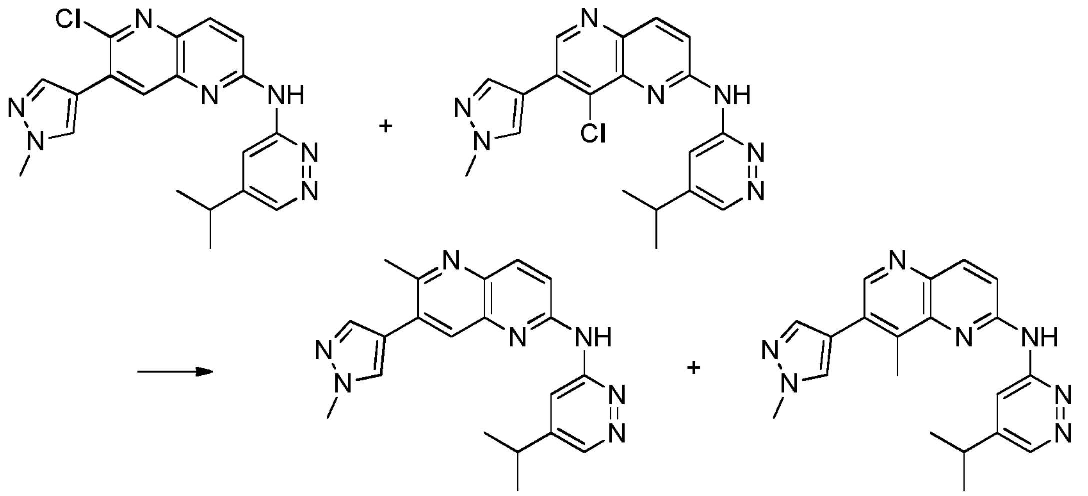

2,4,6-Trimethylboroxine (54 μL) and water (two drops) were added to a mixture of a mixture (30 mg) of 6-chloro-N-(5-isopropylpyridazin-3-yl)-7-(1-methyl-1H-pyrazol-4-yl)-1,5-naphthyridine-2-amine and 8-chloro-N-(5-isopropylpyridazin-3-yl)-7-(1-methyl-1H-pyrazol-4-yl)-1,5-naphthyridine-2-amine, potassium phosphate (48 mg), a [1,1'-bis(diphenylphosphino)ferrocene]palladium(II) dichloride-dichloromethane adduct (12 mg), and 1,2-dimethoxyethane (1.5 mL), followed by stirring at 150°C for 30 minutes using a microwave reaction apparatus. The reaction mixture was cooled to room temperature, poured into a saturated ammonium chloride aqueous solution, and the resultant product was extracted with chloroform. An organic layer obtained therefrom was dried over anhydrous sodium sulfate, and the solvent was distilled off under reduced pressure. The obtained residue was purified sequentially by silica gel column chromatography (hexane-ethyl acetate, chloroform-methanol) and preparative reversed phase chromatography (0.1% formic acid-water, 0.1% formic acid-acetonitrile), thereby obtaining N-(5-isopropylpyridazin-3-yl)-6-methyl-7-(1-methyl-1H-pyrazol-4-yl)-1,5-naphthyridine-2-amine (12 mg) as a pale yellow solid and N-(5-isopropylpyridazin-3-yl)-8-methyl-7-(1-methyl-1H-pyrazol-4-yl)-1,5-naphthyridine-2-amine (3 mg) as a pale yellow solid.

<Example 0849>

N-(5-isopropylpyridazin-3-yl)-6-methyl-7-(1-methyl-1H-pyrazol-4-yl)-1,5-naphthyridine-2-amine
$^1$H-NMR($CDCl_3$)$\delta$:9.10-8.96(1H,brs),8.86(1H,s),8.80(1H,s),8.20(1H,d,J=8.9Hz),7.99(1H,s),7.77(1H,s),7.63(1H,s),7.57(1H,d,J =8.9Hz),4.04(3H,s),3.08-2.98(1H,m),2.79(3H,s),1.40(6H,d,J=6.7Hz).
MSm/z(M+H):360.

<Example 0850>

N-(5-isopropylpyridazin-3-yl)-8-methyl-7-(1-methyl-1H-pyrazol-4-yl)-1,5-naphthyridine-2-amine
$^1$H-NMR($CDCl_3$)$\delta$:9.38(1H,s),9.05(1H,s),8.81(1H,d,J=2.0Hz),8.73(1H,s),8.25(1H,d,J=8.6Hz),7.77(1H,s),7.65(1H,s),7.57(1H,d,J=8.6Hz),4.04(3H,s),3.08-2.98(1H,m),2.87(3H,s),1.41(6H,d,J=6.8Hz).
MSm/z(M+H):360.

<Examples 0851 and 0852>

<0851-1 and 0852-1>

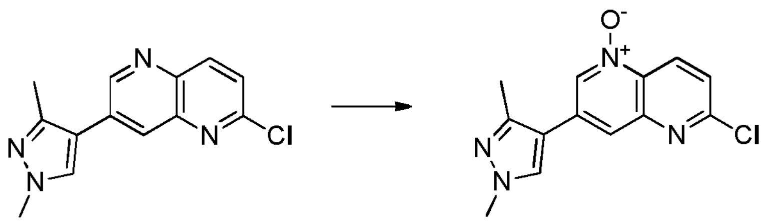

6-Chloro-3-(1,3-dimethyl-1H-pyrazol-4-yl)-1,5-naphthyridine 1-oxide was obtained as a yellow solid in the same manner as in Example 0849-1 except that 2-chloro-7-(1,3-dimethyl-1H-pyrazol-4-yl)-1,5-naphthyridine was used instead of the 2-chloro-7-(1-methyl-1H-pyrazol-4-yl)-1,5-naphthyridine used in Example 0849-1.
MSm/z(M+H):275.

<0851-2 and 0852-2>

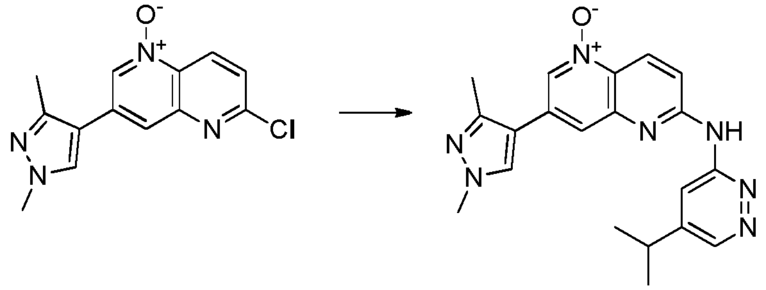

3-(1,3-Dimethyl-1H-pyrazol-4-yl)-6-((5-isopropylpyridazin-3-yl)amino)-1,5-naphthyridine 1-oxide was obtained as a green solid in the same manner as in Example 0849-2 except that 6-chloro-3-(1,3-dimethyl-1H-pyrazol-4-yl)-1,5-naphthyridine 1-oxide was used instead of the 6-chloro-3-(1-methyl-1H-pyrazol-4-yl)-1,5-naphthyridine 1-oxide used in Example 0849-2.
MSm/z(M+H):376.

<0851-3 and 0852-3>

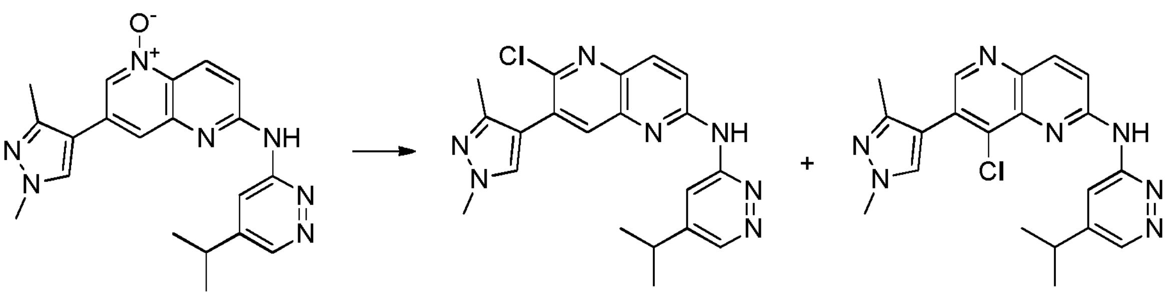

A mixture of 6-chloro-7-(1,3-dimethyl-1H-pyrazol-4-yl)-N-(5-isopropylpyridazin-3-yl)-1,5-naphthyridine-2-amine and 8-chloro-7-(1,3-dimethyl-1H-pyrazol-4-yl)-N-(5-isopropylpyridazin-3-yl)-1,5-naphthyridine-2-amine was obtained as a green solid in the same manner as in Example 0849-3 except that 3-(1,3-dimethyl-1H-pyrazol-4-yl)-6-((5-isopropylpyridazin-3-yl)amino)-1,5-naphthyridine 1-oxide was used instead of the 6-((5-isopropylpyridazin-3-yl)amino)-3-(1-methyl-1H-pyrazol-4-yl)-1,5-naphthyridine 1-oxide used in Example 0849-3.
MSm/z(M+H):394.

<0851-4 and 0852-4>

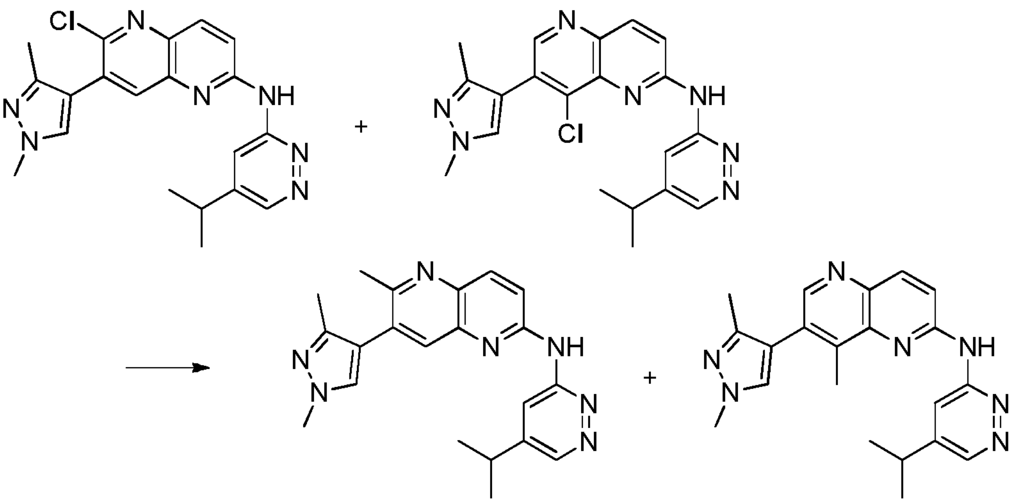

7(1,3-Dimethyl-1H-pyrazol-4-yl)-N-(5-isopropylpyridazin-3-yl)-6-methyl-1,5-naphthyridine-2-amine as a pale yellow solid and 7-(1,3-dimethyl-1H-pyrazol-4-yl)-N-(5-isopropylpyridazin-3-yl)-8-methyl-1,5-naphthyridine-2-amine as a pale yellow solid were obtained in the same manner as in Example 0849-4 except that a mixture of 6-chloro-7-(1,3-dimethyl-1H-pyrazol-4-yl)-N-(5-isopropylpyridazin-3-yl)-1,5-naphthyridine-2-amine and 8-chloro-7-(1,3-dimethyl-1H-pyrazol-4-yl)-N-(5-isopropylpyridazin-3-yl)-1,5-naphthyridine-2-amine was used instead of the mixture of 6-chloro-N-(5-isopropylpyridazin-3-yl)-7-(1-methyl-1H-pyrazol-4-yl)-1,5-naphthyridine-2-amine and 8-chloro-N-(5-isopropylpyridazin-3-yl)-7-(1-methyl-1H-pyrazol-4-yl)-1,5-naphthyridine-2-amine used in Example 0849-4.

<Example 0851>

7-(1,3-Dimethyl-1H-pyrazol-4-yl)-N-(5-isopropylpyridazin-3-yl)-6-methyl-1,5-naphthyridine-2-amine
$^1$H-NMR($CDCl_3$)δ:8.88-8.78(3H,m),8.22(1H,d,J=9.2Hz),7.88(1H,s),7.55(1H,d,J=9.2Hz),7.40(1H,s),7.26(9H,s),3.96(3H,s),3.0 5-2.95(1H,m,J=6.6Hz),2.63(3H,s),2.24(3H,s),1.38(6H,d,J=6.6Hz).
MSm/z(M+H):374.

<Example 0852>

7-(1,3-Dimethyl-1H-pyrazol-4-yl)-N-(5-isopropylpyridazin-3-yl)-8-methyl-1,5-naphthyridine-2-amine
$^1$H-NMR($CDCl_3$)δ:9.43-9.35(1H,brs),9.07(1H,s),8.81(1H,s),8.59(1H,s),8.28(1H,d,J=9.2Hz),7.59(1H,d,J=9.2Hz),7.40(1H,s),3.9 6(3H,s),3.02(1H,quint,J=6.9Hz),2.72(3H,s),2.23(3H,s),1.40(6H,d,J=6.6Hz).
MSm/z(M+H):374.

<Example 0853>

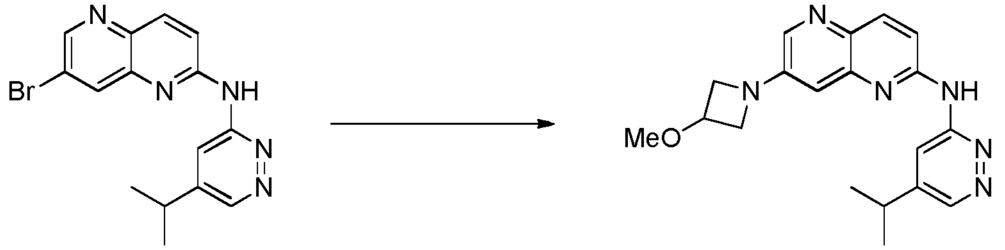

N-(5-isopropylpyridazin-3-yl)-7-(3-methoxyazetidin-1-yl)-1,5-naphthyridine-2-amine was obtained as a yellow solid in the same manner as in Example 0846 except that 3-methoxyazetidine hydrochloride was used instead of the pyrrolidine used in Example 0846.

$^1$H-NMR($CDCl_3$)δ:10.00(1H,s),8.92-8.89(1H,m),8.81-8.78(1H,m),8.17-8.15(1H,m),8.12(1H,d,J=8.6Hz),7.54(1H,d,J=8.6Hz),6.91-6.88(1H,m),4.48-4.41(1H,m),4.31(2H,dd,J=7.9,6.6Hz),3.94(2H,dd,J=7.9,4.6Hz),3.38(3H,s),3.08-2.98(1H,m), 1.40(6H,d,J=7.3Hz).

MSm/z(M+H):351.

<Example 0854>

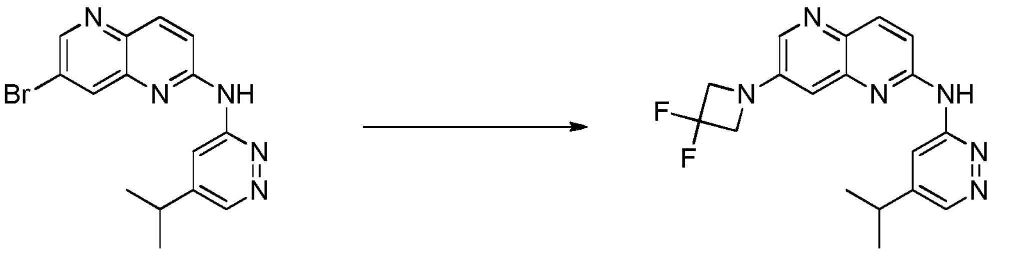

7-(3,3-Difluoroazetidin-1-yl)-N-(5-isopropylpyridazin-3-yl)-1,5-naphthyridine-2-amine was obtained as a yellow solid in the same manner as in Example 0846 except that 3,3-difluoroazetidine hydrochloride was used instead of the pyrrolidine used in Example 0846.

$^1$H-NMR($CDCl_3$)δ:9.57(1H,s),8.87-8.84(1H,m),8.83-8.78(1H,m),8.19(1H,d,J=3.3Hz),8.16(1H,d,J=8.6Hz),7.53(1H,d,J=8.6Hz),6.99(1H,d,J=3.3Hz),4.50-4.41(4H,m),3.09-3.00(1H,m), 1.40(6H,d,J=6.6Hz).

MSm/z(M+H):357.

<Example 0855>

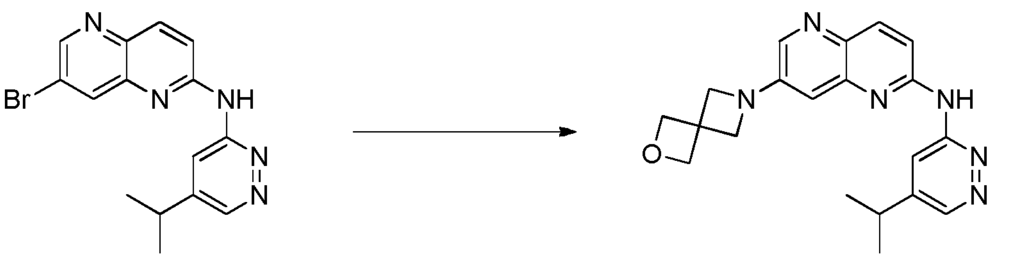

N-(5-isopropylpyridazm-3-yl)-7-(2-oxa-6-azaspiro[3.3]heptan-6-yl)-1,5-naphthyridine-2-amine was obtained as a yellow solid in the same manner as in Example 0846 except that 2-oxa-6-azaspiro[3.3]heptane was used instead of the pyrrolidine used in Example 0846.

$^1$H-NMR($CDCl_3$)δ:8.80-8.77(1H,m),8.78-8.76(1H,m),8.76-8.74(1H,m),8.15(1H,d,J=2.6Hz),8.12(1H,d,J=9.2Hz),7.31-7.27(1H,m),6.89(1H,d,J=2.6Hz),4.91(4H,s),4.26(4H,s),3.06-2.98(1H,m),1.40(6H,d,J=7.3Hz).

MSm/z(M+H):363.

<Example 0856>

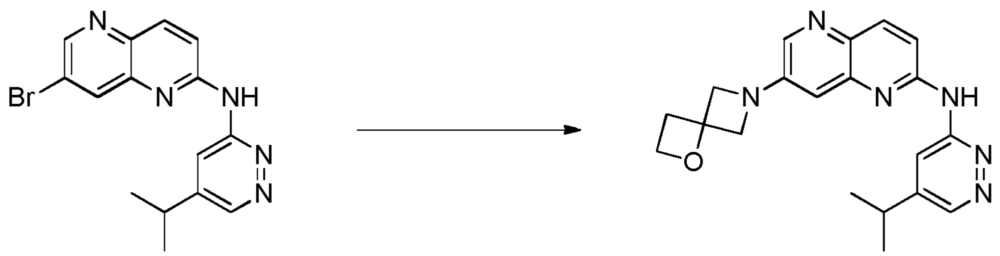

N-(5-isopropylpyridazin-3-yl)-7-(1-oxa-6-azaspiro[3.3]heptan-6-yl)-1,5-naphthyridine-2-amine was obtained as a yellow solid in the same manner as in Example 0846 except that trifluoroacetic acid salt of 1-oxa-6-azaspiro[3.3]heptane was used instead of the pyrrolidine used in Example 0846.

$^1H$-NMR($CDCl_3$)δ:8.79-8.75(1H,m),8.75-8.71(1H,m),8.73-8.68(1H,m),8.17-8.14(1H,m),8.11(1H,d,J=8.6Hz),7.28(1H,d,J=8.6Hz),6.91-6.88(1H,m),4.62(2H,t,J=7.3Hz),4.32(2H,d,J=9.2Hz),4.24(2H,d,J=9.2Hz),3.04-2.98(1H,m),3.00(2H,t,J=7.3Hz),1.39(6H,d,J=6.6Hz).

MSm/z(M+H):363.

<Example 0857>

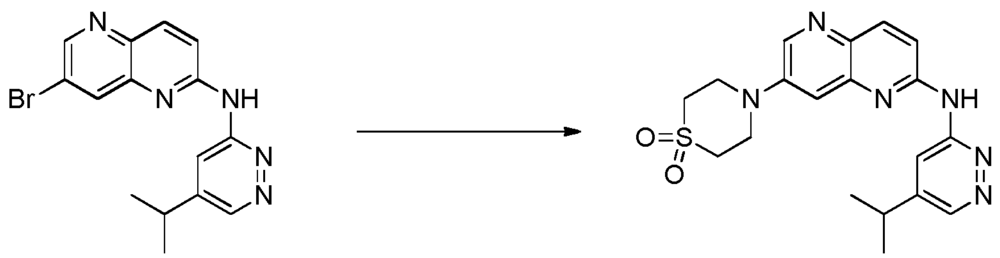

4-(6-((5-Isopropylpyridazin-3-yl)amino)-1,5-naphthyridin-3-yl)thiomorpholine 1,1-dioxide was obtained as a yellow solid in the same manner as in Example 0846 except that thiomorpholine 1,1-dioxide was used instead of the pyrrolidine used in Example 0846.

$^1H$-NMR($CDCl_3$)δ:8.88-8.83(1H,m),8.83-8.79(1H,m),8.79-8.75(1H,m),8.61(1H,d,J=2.6Hz),8.11(1H,d,J=9.2Hz),7.52(1H,d,J=9.2Hz),7041-7.38(1H,m),4.04-4.01(4H,m),3.25-3.22(4H,m),3.04-2.98(1H,m),1.40(6H,d,J=6.6Hz).

MSm/z(M+H):399.

<Example 0858>

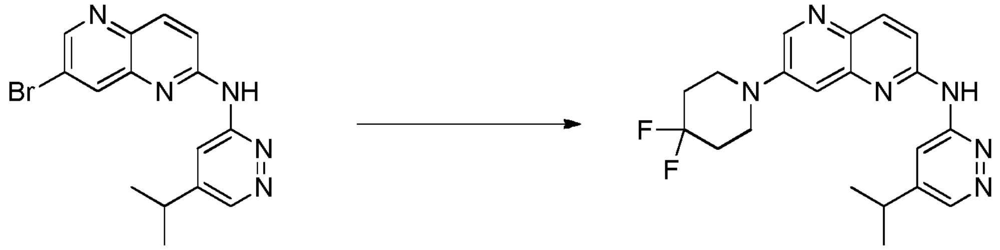

7-(4,4-Difluoropiperidin-1-yl)-N-(5-isopropylpyridazin-3-yl)-1,5-naphthyridine-2-amine was obtained as a yellow solid in the same manner as in Example 0846 except that 4,4-difluoropiperidine hydrochloride was used instead of the pyrrolidine used in Example 0846.

$^1H$-NMR($CDCl_3$)δ:9.17(1H,s),8.82-8.78(1H,m),8.80-8.77(1H,m),8.63(1H,d,J=2.6Hz),8.15(1H,d,J=8.6Hz),7.48(1H,d,J=8.6 Hz),7.36(1H,d,J=2.6Hz),3.58-3.52(4H,m),3.08-2.99(1H,m),2.27-2.14(4H,m),1.40(6H,d,J=7.4Hz).
MSm/z(M+H):385.

<Example 0859>

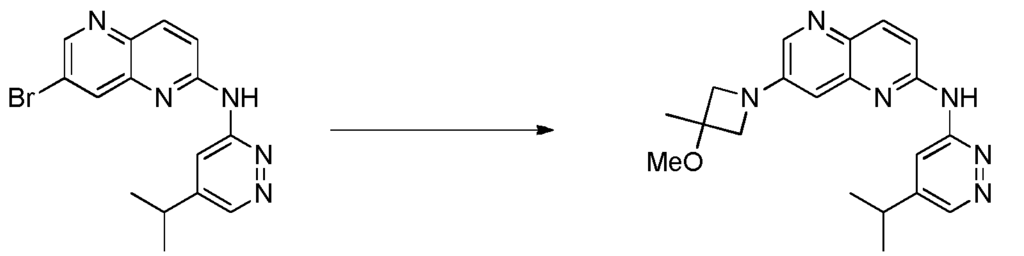

N-(5-isopropylpyridazin-3-yl)-7-(3-methoxy-3-methylazetidin-1-yl)-1,5-naphthyridine-2-amine was obtained as a yellow solid in the same manner as in Example 0846 except that 3-methoxy-3-methylazetidine hydrochloride was used instead of the pyrrolidine used in Example 0846.
$^1H$-NMR($CDCl_3$)δ:9.32(1H,s),8.85-8.82(1H,m),8.80-8.77(1H,m),8.17(1H,d,J=2.6Hz),8.12(1H,d,J=8.6Hz),7.39(1H,d,J=8.6 Hz),6.92-6.89(1H,m),4.04(2H,d,J=7.9Hz),3.92(2H,d,J=7.9Hz),3.33(3H,s),3.07-2.98(1H,m),1.62(3H,s),1.39(6H,d,J=7.3 Hz).
MSm/z(M+H):365.

<Example 0860>

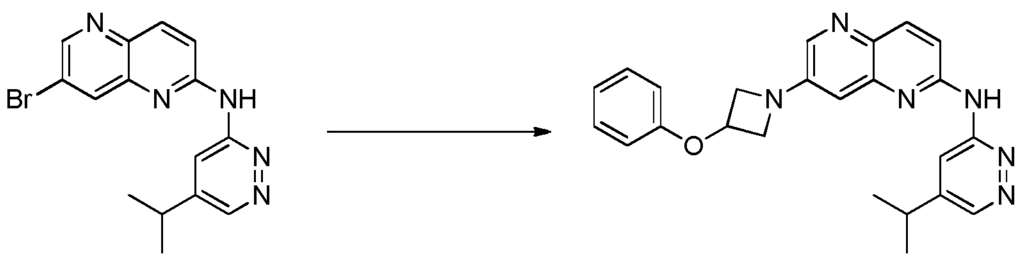

N-(5-isopropylpyridazin-3-yl)-7-(3-phenoxyazetidin-1-yl)-1,5-naphthyridine-2-amine was obtained as a yellow solid in the same manner as in Example 0846 except that 3-phenoxyazetidine hydrochloride was used instead of the pyrrolidine used in Example 0846.
$^1H$-NMR($CDCl_3$)δ:8.90(1H,s),8.82-8.79(1H,m),8.80-8.76(1H,m),8.20(1H,d,J=2.6Hz),8.13(1H,d,J=8.6Hz), 7.36-7.32(1H,m), 7.34-7.31(2H,m),7.03(1H,t,J=7.3Hz),6.95-6.91(1H,m),6.84(2H,d,J=7.3Hz),5.24-5.17(1H,m),4.55(2H,dd,J=8.6,5.2Hz), 4.15(2H,dd,J=8.6,4.0Hz),3.06-2.98(1H,m),1.39(6H,d,J=6.6Hz).
MSm/z(M+H):413.

<Example 0861>

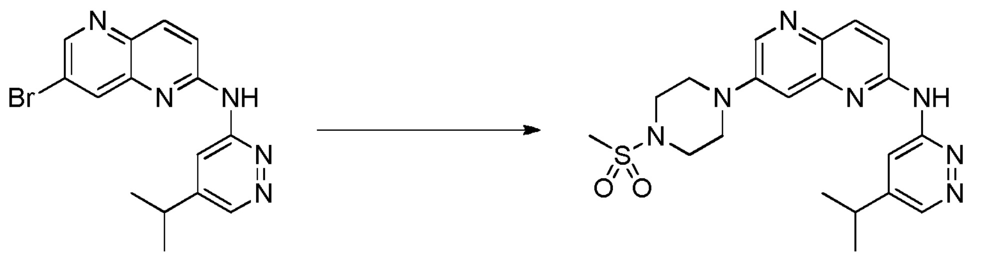

N-(5-isopropylpyridazin-3-yl)-7-(4-(methylsulfonyl)piperazin-1-yl)-1,5-naphthyridine-2-amine was obtained as a yellow solid in the same manner as in Example 0846 except that 1-(methylsulfonyl)piperazine was used instead of the pyrrolidine used in Example 0846.
$^1$H-NMR($CDCl_3$)δ:8.80(1H,s),8.74-8.70(1H,m),8.70-8.65(1H,m),8.62(1H,d,J=2.6Hz),8.16(1H,d,J=9.2Hz),7.40(1H,d,J=9.2Hz),7.35(1H,d,J=2.6Hz),3.51-3.48(4H,m),3.49-3.46(4H,m),3.08-2.98(1H,m),2.88(3H,s),1.40(6H,d,J=6.6Hz).
MSm/z(M+H):428.

<Example 0862>

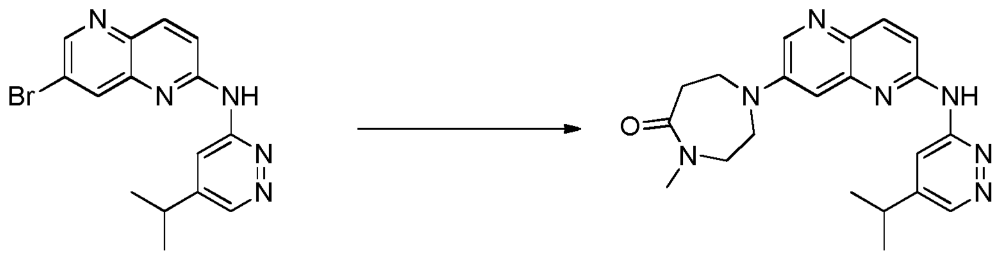

1-(6-((5-Isopropylpyridazin-3-yl)amino)-1,5-naphthyridin-3-yl)-4-methyl-1,4-diazepan-5-one was obtained as a yellow solid in the same manner as in Example 0846 except that 4-methyl-1,4-diazepan-5-one was used instead of the pyrrolidine used in Example 0846.
$^1$H-NMR($CDCl_3$)δ:9.36(1H,s),8.81-8.78(1H,m),8.79-8.76(1H,m),8.60-8.56(1H,m),8.15(1H,d,J=9.2Hz),7.51(1H,d,J=8.6Hz),7.29-7.26(1H,m),3.71-3.68(2H,m),3.69-3.66(2H,m),3.67-3.61(2H,m),3.08-2.98(1H,m),3.08(3H,s),2.93-2.88(2H,m),1.40(6H,d,J=6.6Hz).
MSm/z(M+H):392.

<Example 0863>

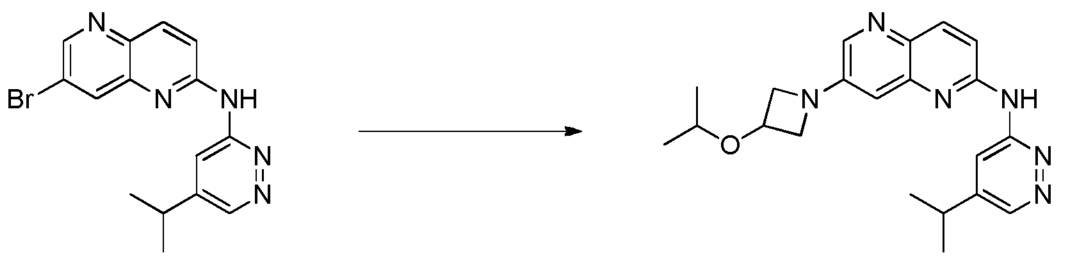

7-(3-Isopropoxyazetidin-1-yl)-N-(5-isopropylpyridazin-3-yl)-1,5-naphthyridine-2-amine was obtained as a yellow solid in the same manner as in Example 0846 except that 3-isopropoxyazetidine hydrochloride was used instead of the pyrrolidine used in Example 0846.

$^1$H-NMR(CDCl$_3$)δ:9.72(1H,s),8.89-8.87(1H,m),8.79(1H,d,J=2.0Hz),8.16(1H,d,J=2.6Hz),8.12(1H,d,J=9.2Hz),7.48(1H,d,J=8.6Hz),6.90(1H,d,J=2.6Hz),4.63-4.56(1H,m),4.34(2H,dd,J=7.9,7.9Hz),3.91(2H,dd,J=7.9,5.3Hz),3.75-3.66(1H,m),3.08-2.99(1H,m),1.40(6H,d,J=6.6Hz),1.21(6H,d,J=5.9Hz).
MSm/z(M+H):379.

<Example 0864>

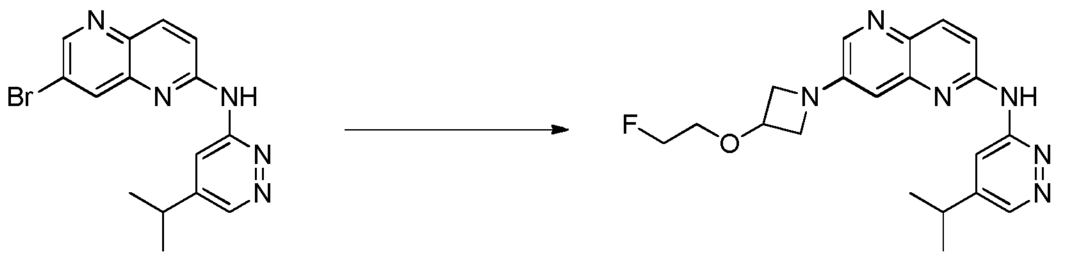

7-(3-(2-Fluoroethoxy)azetidin-1-yl)-N-(5-isopropylpyridazin-3-yl)-1,5-naphthyridine-2-amine was obtained as a yellow solid in the same manner as in Example 0846 except that 3-(2-fluoroethoxy)azetidine hydrochloride was used instead of the pyrrolidine used in Example 0846.
$^1$H-NMR(CDCl$_3$)δ:9.75(1H,s),8.88(1H,s),8.79(1H,d,J=2.0Hz),8.16(1H,d,J=2.6Hz),8.12(1H,d,J=9.2Hz),7.49(1H,d,J=9.2Hz),6.91(1H,d,J=2.6Hz),4.65-4.58(1H,m),4.61(2H,dt,J=46.8,4.0Hz),4.34(2H,dd,J=8.6,8.6Hz),3.99(2H,dd,J=8.6,4.6Hz),3.76(2H,dt,J =29.7,4.0Hz),3.08-2.99(1H,m),1.40(6H,d,J=7.3Hz).
MSm/z(M+H):383.

<Example 0865>

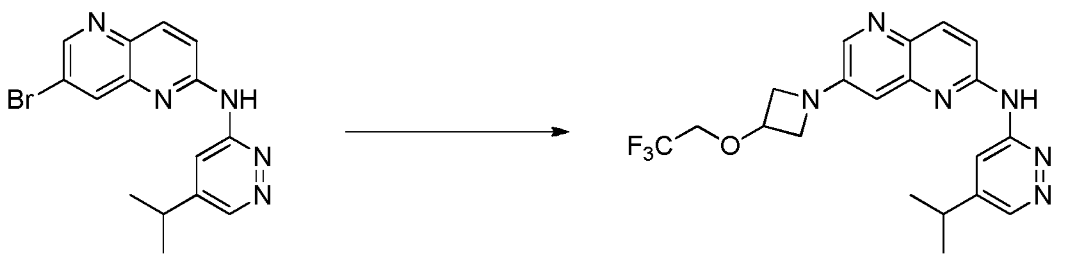

N-(5-isopropylpyridazin-3-yl)-7-(3-(2,2,2-trifluoroethoxy)azetidin-1-yl)-1,5-naphthyridine-2-amine was obtained as a yellow solid in the same manner as in Example 0846 except that 3-(2,2,2-trifluoroethoxy)azetidine hydrochloride was used instead of the pyrrolidine used in Example 0846.
$^1$H-NMR(CDCl$_3$)δ:9.35(1H,s),8.85-8.82(1H,m),8.81-8.78(1H,m),8.17(1H,d,J=2.6Hz),8.13(1H,d,J=8.6Hz),7.43(1H,d,J=8.6Hz),6.92(1H,d,J=2.6Hz),4.71-4.64(1H,m),4.37(2H,dd,J=8.6,8.6Hz),4.02(2H,dd,J=8.6,4.3Hz),3.95-3.88(2H,m),3.08-2.99(1H,m),1.40(6H,d,J=7.3Hz).
MSm/z(M+H):419.

<Example 0866>

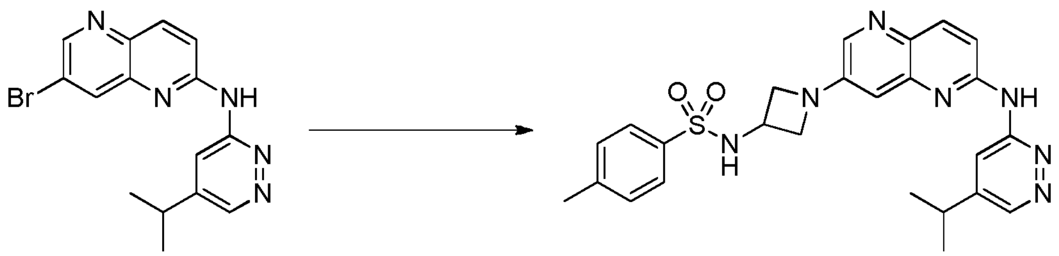

N-(1-(6-((5-isopropylpyridazin-3-yl)amino)-1,5-naphthyridin-3-yl)azetidin-3-yl)-4-methylbenzene sulfonamide was obtained as a yellow solid in the same manner as in Example 0846 except that N-(azetidin-3-yl)-4-methylbenzene sulfonamide hydrochloride was used instead of the pyrrolidine used in Example 0846.
$^1$H-NMR(DMSO-d$_6$)$\delta$:10.52(1H,s),8.83-8.82(1H,m),8.65-8.64(1H,m),8.45-8.33(1H,m),8.08(1H,d,J=2.6Hz),8.05(1H,d,J=9.2Hz),7.73(2H,d,J=7.9Hz),7.45(2H,d,J=7.9Hz),7.44(1H,d,J=9.2Hz),6.76(1H,d,J=2.6Hz),4.29-4.20(1H,m),4.17(2H,dd,J=8.6,8.6Hz),3.64(2H,dd,J=8.6,5.3Hz),3.05-2.96(1H,m),2.43(3H,s),1.29(6H,d,J=6.6Hz).
MSm/z(M+H):490.

<Example 0867>

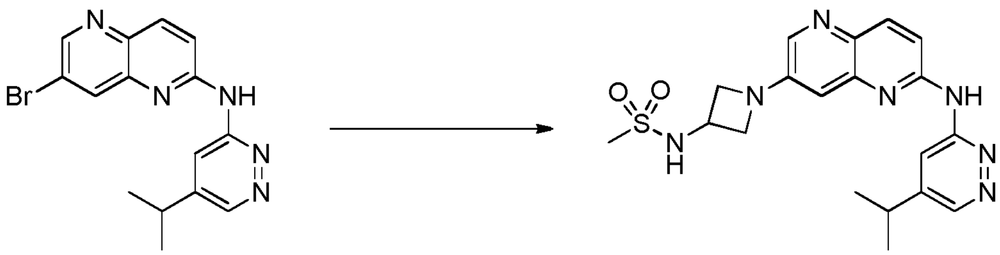

N-(1-(6-((5-isopropylpyridazin-3-yl)amino)-1,5-naphthyridin-3-yl)azetidin-3-yl)methane sulfonamide was obtained as a yellow solid in the same manner as in Example 0846 except that N-(azetidin-3-yl)methane sulfonamide hydrochloride was used instead of the pyrrolidine used in Example 0846.
$^1$H-NMR(DMSO-d$_6$)$\delta$:10.53(1H,s),8.83(1H,d,J=2.0Hz),8.67(1H,d,J=1.3Hz),8.18(1H,d,J=2.6Hz),8.07(1H,d,J=9.2Hz),7.92(1H,d,J=5.9Hz),7.46(1H,d,J=9.2Hz),6.88(1H,d,J=2.6Hz),4.44-4.39(2H,m),3.89-3.85(1H,m),3.40-3.36(2H,m),3.04-2.98(1H,m),2.97(3H,s), 1.31(6H,d,J=6.6Hz).
MSm/z(M+H):414.

<Example 0868>

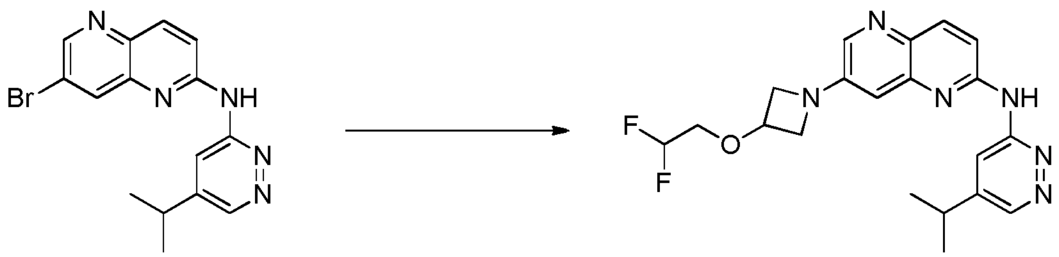

7-(3-(2,2-Difluoroethoxy)azetidin-1-yl)-N-(5-isopropylpyridazin-3-yl)-1,5-naphthyridine-2-amine was obtained

as a yellow solid in the same manner as in Example 0846 except that 3-(2,2-difluoroethoxy)azetidine hydrochloride was used instead of the pyrrolidine used in Example 0846.
$^1$H-NMR($CDCL_3$)δ:9.31(1H,s),8.84-8.81(1H,m),8.79(1H,d,J=2.0Hz),8.17(1H,d,J=2.6Hz),8.12(1H,d,J=8.6Hz), 7.41(1H,d,J=8.6Hz),6.91(1 H,d,J=2.6Hz),5.92(1H,tt,J=55.2,4.0Hz),4.67-4.57(1H,m),4.35(2H,dd,J=8.6,8.6Hz),3.99(2H,dd,J=8.6,4.6Hz),3.74(2H,td ,J=13.9,4.0Hz),3.08-2.99(1H,m),1.40(6H,d,J=6.6Hz).
MSm/z(M+H):401.

<Example 0869>

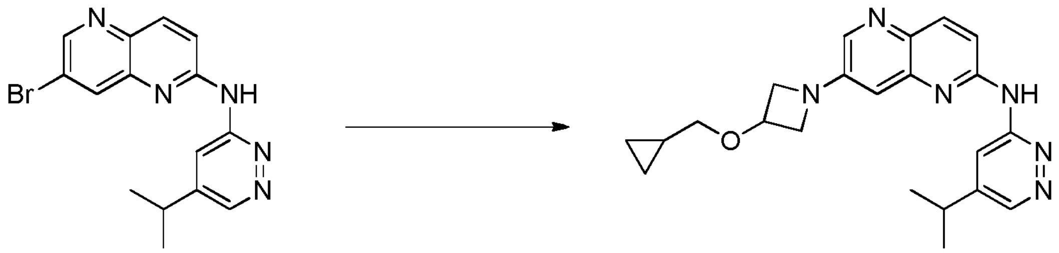

7-(3-(Cyclopropylmethoxy)azetidin-1-yl)-N-(5-isopropylpyridazin-3-yl)-1,5-naphthyridine-2-amine was obtained as a yellow solid in the same manner as in Example 0846 except that 3-(cyclopropylmethoxy)azetidine hydrochloride was used instead of the pyrrolidine used in Example 0846.
$^1$H-NMR($CDCl_3$)δ:8.90(1H,s),8.80-8.76(1H,m),8.78-8.76(1H,m),8.18-8.15(1H,m),8.11(1H,d,J=8.6Hz),7.32(1H,d,J=8.6Hz) ,6.91-6.87(1H,m),4.60-4.52(1H,m),4.33(2H,dd,J=8.6,8.6Hz),3.97(2H,dd,J=8.6,4.6Hz),3.34(2H,d,J=6.6Hz),3.06-2.98(1 H,m),1.39(6H,d,J=6.6Hz),1.13-1.03(1H,m),0.63-0.57(2H,m),0.29-0.21(2H,m).
MSm/z(M+H):391.

<Example 0870>

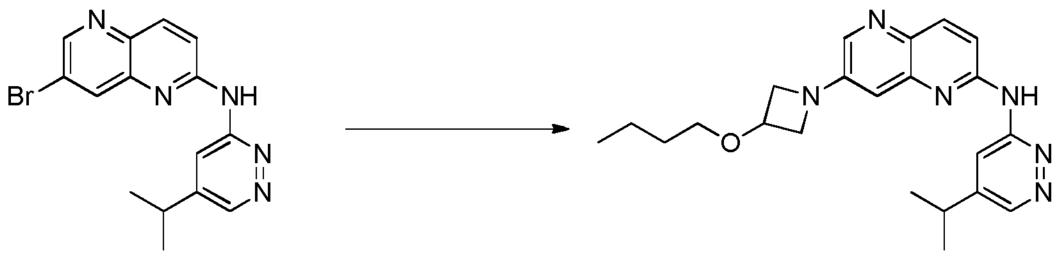

7-(3-Butoxyazetidin-1-yl)-N-(5-isopropylpyridazin-3-yl)-1,5-naphthyridine-2-amine was obtained as a yellow solid in the same manner as in Example 0846 except that 3-butoxyazetidine hydrochloride was used instead of the pyrrolidine used in Example 0846.
$^1$H-NMR($CDCl_3$)δ:9.70(1H,s),8.89-8.86(1H,m),8.80-8.77(1H,m),8.16(1H,d,J=2.0Hz),8.12(1H,d,J=8.6Hz),7.48(1H,d,J=8.6 Hz),6.91-6.88(1H,m),4.55-4.47(1H,m),4.31(2H,dd,J=8.6,8.6Hz),3.93(2H,dd,J=8.6,4.6Hz),3.46(2H,t,J=6.6Hz),3.08-2.9 9(1H,m),1.65-1.56(2H,m),1.45-1.35(2H,m),1.40(6H,d,J=7.0Hz),0.94(3H,t,J=7.3Hz).
MSm/z(M+H):393.

<Example 0871>

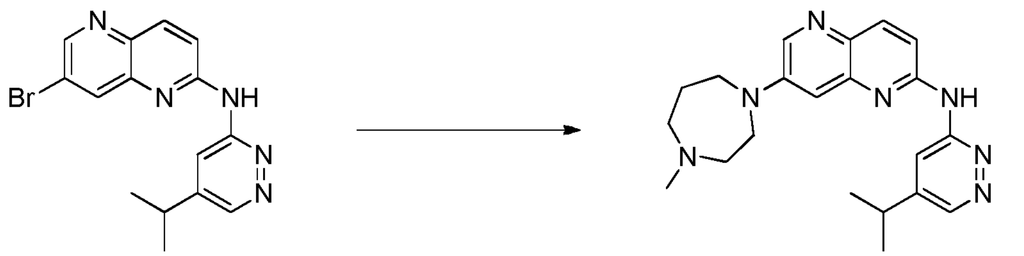

N-(5-isopropylpyridazin-3-yl)-7-(4-methyl-1,4-diazepan-1-yl)-1,5-naphthyridine-2-amine was obtained as a yellow solid in the same manner as in Example 0846 except that 1-methyl-1,4-diazepane was used instead of the pyrrolidine used in Example 0846.
$^1$H-NMR($CDCl_3$)δ:8.78-8.76(1H,m),8.74-8.71(1H,m),8.49(1H,d,J=2.6Hz),8.09(1H,d,J=8.6Hz),7.28-7.25(1H,m),7.27-7.22(1H,m),7.10(1H,d,J=2.6Hz),3.76-3.72(2H,m),3.70-3.64(2H,m),3.07-2.97(1H,m),2.83-2.77(2H,m),2.63-2.58(2H,m),2.41(3H,s),2.14-2.06(2H,m),1.39(6H,d,J=7.3Hz).
MSm/z(M+H):378.

<Example 0872>

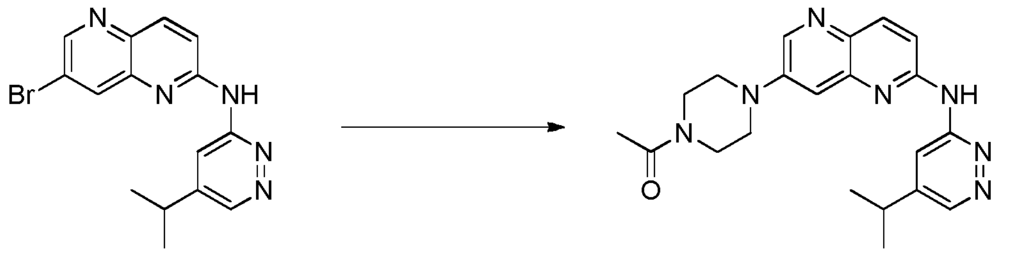

1-(4-(6-((5-Isopropylpyridazin-3-yl)amino)-1,5-naphthyridin-3-yl)piperazin-1-yl)ethanone was obtained as a yellow solid in the same manner as in Example 0846 except that 1-(piperazin-1-yl)ethanone was used instead of the pyrrolidine used in Example 0846.
$^1$H-NMR($CDCl_3$)δ:9.56(1H,s),8.83-8.80(1H,m),8.81-8.78(1H,m),8.62(1H,d,J=2.0Hz),8.16(1H,d,J=8.6Hz),7.56(1H,d,J=8.6Hz),7.33(1H,d,J=2.0Hz),3.90-3.84(2H,m),3.76-3.70(2H,m),3.43-3.33(4H,m),3.08-2.99(1H,m),2.19(3H,s),1.40(6H,d,J=6.6Hz).
MSm/z(M+H):392.

<Example 0873>

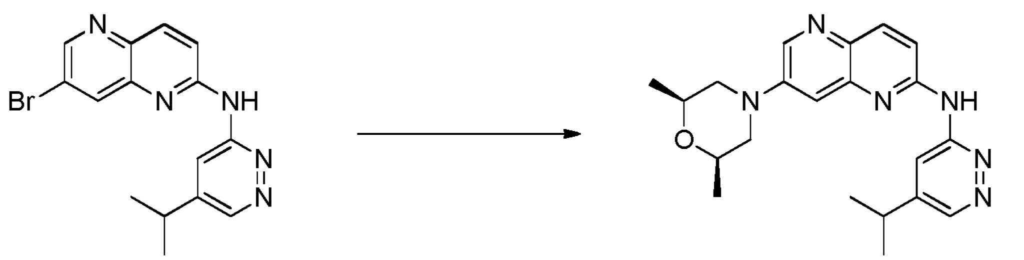

7-(cis-2,6-Dimethylmorpholino)-N-(5-isopropylpyridazin-3-yl)-1,5-naphthyridine-2-amine was obtained as a yellow solid in the same manner as in Example 0846 except that cis-2,6-dimethylmorpholine was used instead of the

pyrrolidine used in Example 0846.
$^1$H-NMR($CDCl_3$)δ:9.66(1H,s),8.84-8.80(1H,m),8.82-8.78(1H,m),8.61(1H,d,J=2.6Hz),8.15(1H,d,J=8.6Hz),7.57(1H,d,J=8.6Hz),7.30(1H,d,J=2.6Hz),3.93-3.84(2H,m),3.65(2H,d,J=10.6Hz),3.10-3.01(1H,m),2.61(2H,dd,J=10.6,10.6Hz),1.42(6H,d,J=6.6Hz),1.32(6H,d,J=6.6Hz).
MSm/z(M+H):379.

<Example 0874>

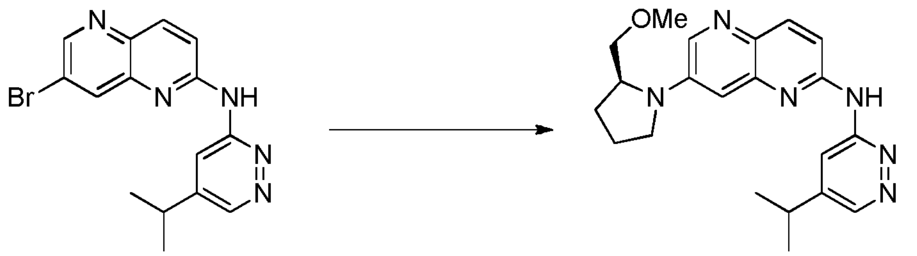

(S)-N-(5-isopropylpyridazin-3-yl)-7-(2-(methoxymethyl)pyrrolidin-1-yl)-1,5-naphthyridine-2-amine was obtained as a yellow solid in the same manner as in Example 0846 except that (S)-2-(methoxymethyl)pyrrolidine was used instead of the pyrrolidine used in Example 0846.
$^1$H-NMR($CDCl_3$)δ:9.37(1H,s),8.87-8.84(1H,m),8.78(1H,d,J=2.6Hz),8.45(1H,d,J=2.6Hz),8.10(1H,d,J=8.6Hz),7.38(1H,d,J=8.6Hz),7.07(1H,d,J=2.6Hz),4.16-4.09(1H,m),3.65-3.60(1H,m),3.61-3.54(1H,m),3.41(3H,s),3.38-3.28(2H,m),3.07-2.97(1H,m),2.17-2.03(4H,m),1.40(6H,d,J=7.3Hz).
MSm/z(M+H):379.

<Examples 0875 and 0876>

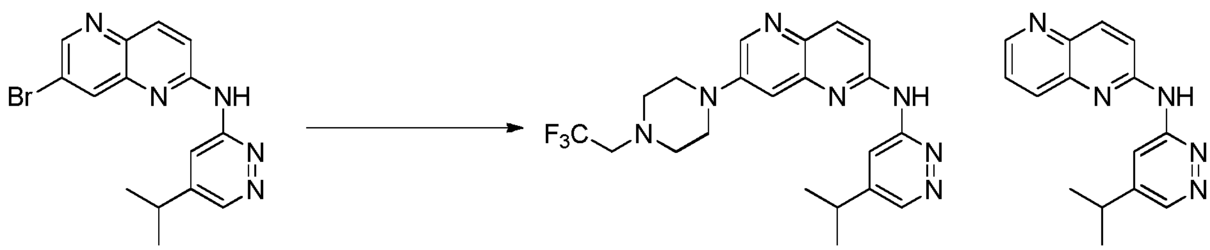

N-(5-isopropylpyridazin-3-yl)-7-(4-(2,2,2-trifluoroethyl)piperazin-1-yl)-1,5-naphthyridine-2-amine as a white solid and N-(5-isopropylpyridazin-3-yl)-1,5-naphthyridine-2-amine as a white solid were obtained in the same manner as in Example 0846 except that 1-(2,2,2-trifluoroethyl)piperazine was used instead of the pyrrolidine used in Example 0846.

<Example 0875>

N-(5-isopropylpyridazin-3-yl)-7-(4-(2,2,2-trifluoroethyl)piperazin-1-yl)-1,5-naphthyridine-2-amine
$^1$H-NMR($CDCl_3$)δ:8.79-8.77(1H,m),8.72(1H,d,J=2.0Hz),8.63(1H,d,J=2.0Hz),8.50(1H,s),8.14(1H,d,J=9.2Hz),7.32-7.29(1H,m),7.32(1H,d,J=9.2Hz),3.45-3.39(4H,m),3.09(2H,q,J=9.3Hz),3.06-2.98(1H,m),2.96-2.89(4H,m),1.39(6H,d,J=7.3Hz).
MSm/z(M+H):432.

<Example 0876> (not in accordance with the present invention)

N-(5-isopropylpyridazin-3-yl)-1,5-naphthyridine-2-amine
$^1$H-NMR($CDCl_3$)δ:9.40(1H,s),8.98-8.95(1H,m),8.82(1H,d,J=2.0Hz),8.80-8.77(1H,m),8.29(1H,d,J=8.6Hz),8.13(1H,d,J=8.6 Hz),7.70(1H,d,J=8.6Hz),7.59-7.54(1H,m),3.09-3.00(1H,m),1.41(6H,d,J=6.6Hz).
MSm/z(M+H):266.

<Example 0877>

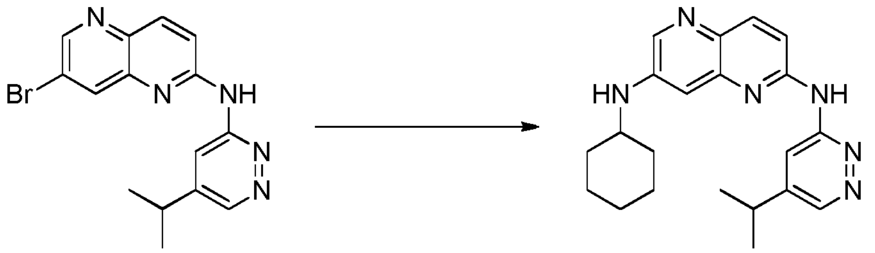

N$^7$-cyclohexyl-N$^2$-(5-isopropylpyridazin-3-yl)-1,5-naphthyridine-2,7-diamine was obtained as a yellow solid in the same manner as in Example 0846 except that cyclohexylamine was used instead of the pyrrolidine used in Example 0846, and 2-(dicyclohexylphosphino)-3,6-dimethoxy-2',4',6'-triisopropyl-1,1'-biphenyl was used instead of the 2-dicyclohexylphosphino-2',6'-diisopropoxybiphenyl used in Example 0846.
$^1$H-NMR($CDCl_3$)δ:9.49(1H,s),8.81-8.79(1H,m),8.79-8.76(1H,m),8.23(1H,d,J=2.6Hz),8.08(1H,d,J=9.2Hz),7.43(1H,d,J=9.2 Hz),7.00(1H,d,J=2.6Hz),4.05(1 H,d,J=7.3Hz),3.45-3.37(1H,m),3.07-2.98(1H,m),2.17-2.11(2H,m),1.85-1.78(2H,m),1.74-1.67(2H,m),1.50-1.39(2H,m), 1.40(6H,d,J=6.6Hz),1.33-1.22(2H,m).
MSm/z(M+H):363.

<Example 0878>

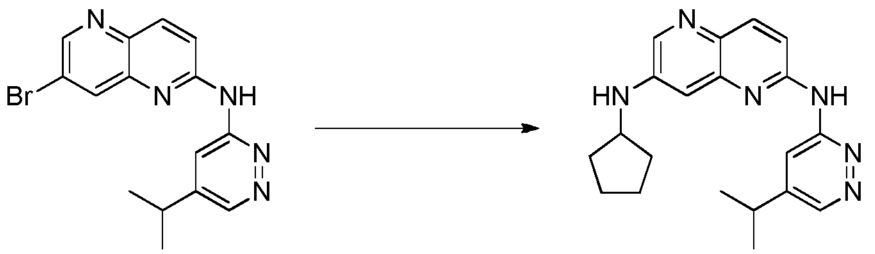

N$^7$-cyclopentyl-N$^2$-(5-isopropylpyridazin-3-yl)-1,5-naphthyridine-2,7-diamine was obtained as a yellow solid in the same manner as in Example 0846 except that cyclopentylamine was used instead of the pyrrolidine used in Example 0846, and 2-(dicyclohexylphosphino)-3,6-dimethoxy-2',4',6'-triisopropyl-1,1'-biphenyl was used instead of the 2-dicyclohexylphosphino-2',6'-diisopropoxybiphenyl used in Example 0846.
$^1$H-NMR($CDCl_3$)δ:9.56(1H,s),8.86-8.82(1H,m),8.78(1H,d,J=2.0Hz),8.24(1H,d,J=2.6Hz),8.09(1H,d,J=9.2Hz),7.43(1H,d,J=9.2Hz),7.02(1 H,d,J=2.6Hz),4.15(1H,d,J=5.9Hz),3.96-3.88(1H,m),3.07-2.98(1H,m),2.17-2.07(2H,m),1.79-1.68(4H,m),1.69-1.56(2H, m),1.40(6H,d,J=6.6Hz).
MSm/z(M+H):349.

<Example 0879>

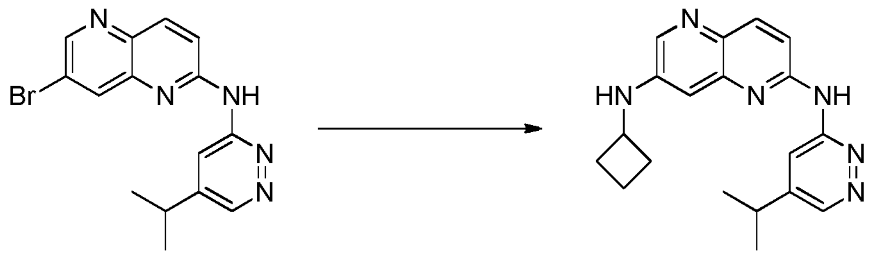

$N^7$-cyclobutyl-$N^2$-(5-isopropylpyridazin-3-yl)-1,5-naphthyridine-2,7-diamine was obtained as a yellow solid in the same manner as in Example 0846 except that cyclobutylamine was used instead of the pyrrolidine used in Example 0846, and 2-(dicyclohexylphosphino)-3,6-dimethoxy-2',4',6'-triisopropyl-1,1'-biphenyl was used instead of the 2-dicyclohexylphosphino-2',6'-diisopropoxybiphenyl used in Example 0846.
$^1$H-NMR($CDCl_3$)δ:9.79(1H,s),8.90-8.86(1H,m),8.79(1H,d,J=2.0Hz),8.23(1H,d,J=2.6Hz),8.09(1H,d,J=8.6Hz),7.48(1H,d,J=8.6Hz),6.93(1H,d,J=2.6Hz),4.33(1H,d,J=5.9Hz),4.10-4.01(1H,m),3.08-2.98(1H,m),2.58-2.47(2H,m),2.03-1.84(4H,m),1.41(6H,d,J=6.0Hz).
MSm/z(M+H):335.

<Example 0880>

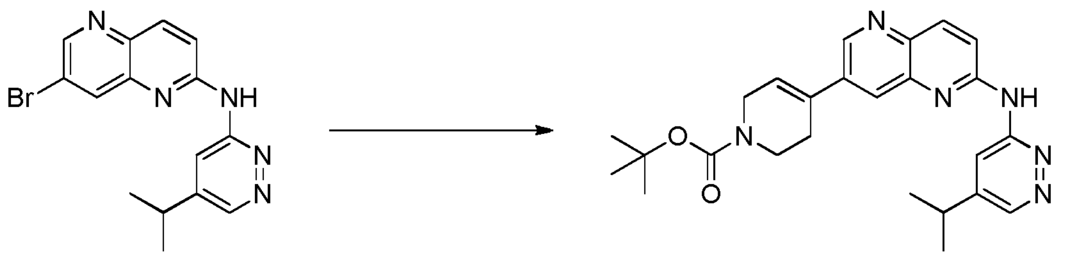

A mixture of 7-bromo-N-(5-isopropylpyridazin-3-yl)-1,5-naphthyridine-2-amine (40 mg), tert-butyl 4-(4,4,5,5-tetramethyl-1,3,2-dioxaborolan-2-yl)-5,6-dihydropyridine-1(2H)-carboxylate (72 mg), potassium phosphate (99 mg), (tris(dibenzylideneacetone)dipalladium(0) (11 mg), 2-dicyclohexylphosphino-2',4',6'-triisopropylbiphenyl (22 mg), tert-amyl alcohol (0.8 mL), and water (0.2 mL) was stirred at 100°C for 1 hour. The reaction mixture was cooled to room temperature, the solvent was distilled off under reduced pressure, and the obtained residue was purified by silica gel column chromatography (methanol-ethyl acetate), thereby obtaining tert-butyl 4-(6-((5-isopropylpyridazin-3-yl)amino)-1,5-naphthyridin-3-yl)-5,6-dihydropyridine-1(2H)-carboxylate (36 mg) as a yellow solid.
$^1$H-NMR($CDCl_3$)δ:9.21(1H,s),8.89-8.87(1H,m),8.88-8.86(1H,m),8.82(1H,d,J=2.0Hz),8.25(1H,d,J=8.6Hz),7.96(1H,d,J=2.0Hz),7.63(1H,d,J=8.6Hz),6.35-6.31(1H,m),4.21-4.16(2H,m),3.76-3.70(2H,m),3.10-2.99(1H,m),2.69-2.65(2H,m),1.52(9H,s),1.42(6H,d,J=7.3Hz).
MSm/z(M+H):447.

<Example 0881>

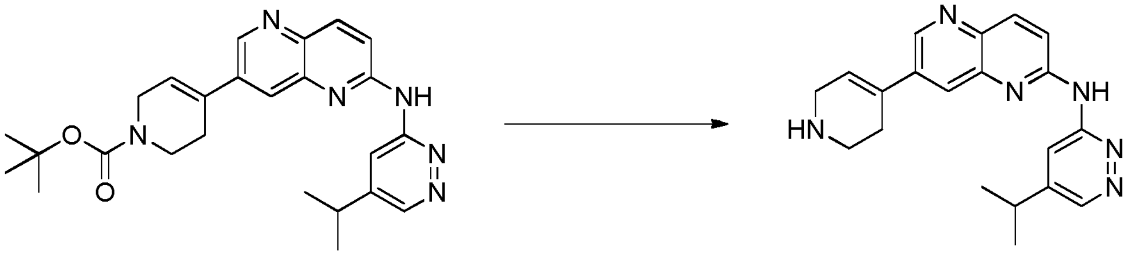

N-(5-isopropylpyridazin-3-yl)-7-(1,2,3,6-tetrahydropyridin-4-yl)-1,5-naphthyridine-2-amine was obtained as a yellow solid in the same manner as in Example 0905 except that tert-butyl 4-(6-((5-isopropylpyndazin-3-yl)amino)-1,5-naphthyridin-3-yl)-5,6-dihydropyridine-1(2H)-carboxylate was used instead of the tert-butyl 3-((6-((5-isopropylpyridazin-3-yl)amino)-1,5-naphthyridin-3-yl)amino)azetidine-1-carboxylate used in Example 0905.

$^1$H-NMR($CDCl_3$)δ:8.91-8.88(1H,m),8.86-8.80(1H,m),8.82-8.77(1H,m),8.24(1H,d,J=8.6Hz),8.10-8.05(1H,m),7.96(1H,d,J=2.0Hz),7.68(1H,d,J=8.6Hz),6.45-6.42(1H,m),3.66-3.62(2H,m),3.22-3.18(2H,m),3.08-3.00(1H,m),2.64-2.56(2H,m),2.05-1.97(1H,m),1.41(6H,d,J=6.6Hz).

MSm/z(M+H):347.

<Example 0882>

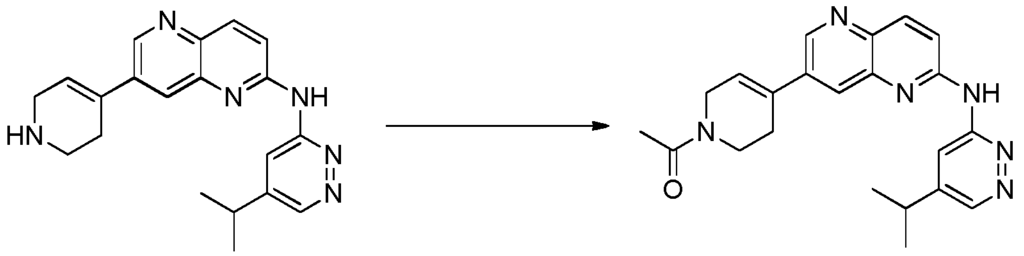

A mixture of N-(5-isopropylpyridazin-3-yl)-7-(1,2,3,6-tetrahydropyridin-4-yl)-1,5-naphthyridine-2-amine (13 mg), acetyl chloride (11 μL), N,N-diisopropylethylamine (79 μL), and dichloromethane (1 mL) was stirred at room temperature for 3 hours. A 25% sodium hydroxide aqueous solution was added to the reaction mixture, and the solvent was distilled off under reduced pressure. The obtained residue was purified by silica gel column chromatography (methanol-ethyl acetate), thereby obtaining 1-(4-(6-((5-isopropylpyridazin-3-yl)amino)-1,5-naphthyridin-3-yl)-5,6-dihydropyridine-1(2H)-yl)ethanone (3 mg) as a yellow solid.

$^1$H-NMR($CDCl_3$)δ:8.89-8.85(1H,m),8.83-8.81(1H,m),8.81-8.79(1H,m),8.80-8.77(1H,m),8.25(1H,d,J=9.2Hz),7.98-7.94(1H,m),7.52(1H,d,J=9.2Hz),6.35-6.30(1H,m),4.35-4.25(2H,m),3.90-3.77(2H,m),3.08-3.00(1H,m),2.79-2.65(2H,m),2.20(3H,s),1.41(6H,d,J=6.6Hz).

MSm/z(M+H):389.

<Example 0883>

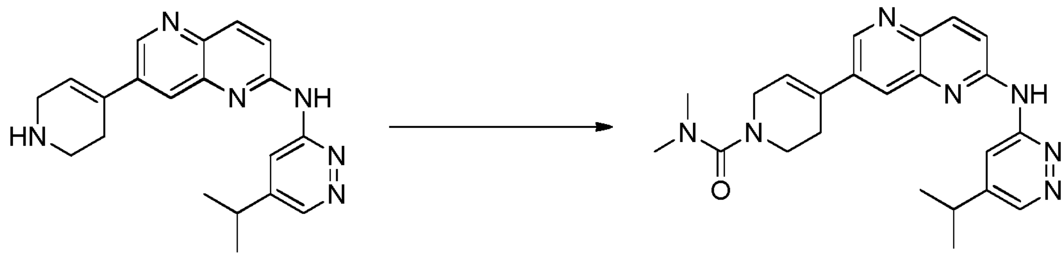

4-(6-((5-Isopropylpyridazin-3-yl)amino)-1,5-naphthyridin-3-yl)-N,N-dimethyl-5,6-dihydropyridine-1(2H)-carboxamide was obtained as a yellow solid in the same manner as in Example 0882 except that dimethylcarbamoyl chloride was used instead of the acetyl chloride used in Example 0882.
$^1$H-NMR($CDCl_3$)δ:9.02-8.99(1H,m),8.88-8.82(1H,m),8.87(1H,d,J=2.0Hz),8.83-8.80(1H,m),8.25(1H,d,J=8.6Hz),7.97(1H,d,J=2.0Hz),7.88(1H,d,J=8.6Hz),6.36-6.33(1H,m),4.07-4.02(2H,m),3.58-3.53(2H,m),3.11-3.02(1H,m),2.90(6H,s),2.78-2.69(2H,m),1.43(6H,d,J=6.6Hz).
MSm/z(M+H):418.

<Example 0884>

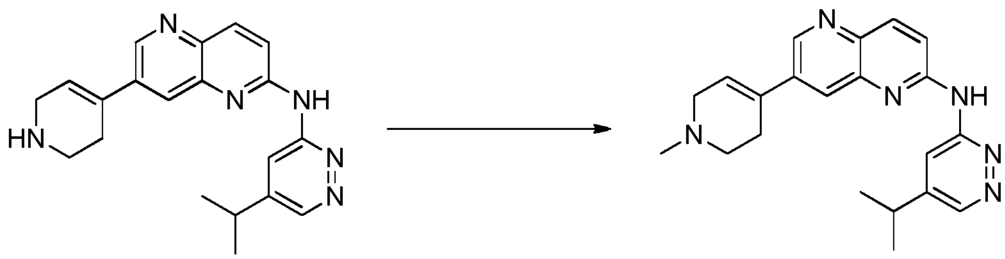

A mixture of N-(5-isopropylpyridazin-3-yl)-7-(1,2,3,6-tetrahydropyridin-4-yl)-1,5-naphthyridine-2-amine (13 mg), paraformaldehyde (20 mg), sodium triacetoxyborohydride (33 mg), acetic acid (1 μL), and methanol (0.5 mL) was stirred at room temperature for 3 hours. A saturated sodium hydrogen carbonate aqueous solution was added to the reaction mixture, and the solvent was distilled off under reduced pressure. The obtained residue was purified by silica gel column chromatography (methanol-ethyl acetate), thereby obtaining N-(5-isopropylpyridazin-3-yl)-7-(1-methyl-1,2,3,6-tetrahydropyridin-4-yl)-1,5-naphthyridine-2-amine (6 mg) as a yellow solid.
$^1$H-NMR($CDCl_3$)δ:9.89(1H,s),8.98-8.95(1H,m),8.88(1H,d,J=2.6Hz),8.82(1H,d,J=2.6Hz),8.23(1H,d,J=9.2Hz),7.96(1H,d,J=2.6Hz),7.76(1H,d,J=9.2Hz),6.37-6.34(1H,m),3.23-3.20(2H,m),3.09-3.00(2H,m),3.03-2.94(1H,m),2.77-2.72(2H,m),2.46(3H,s),1.42(6H,d,J=6.0Hz).
MSm/z(M+H):361.

<Example 0885>

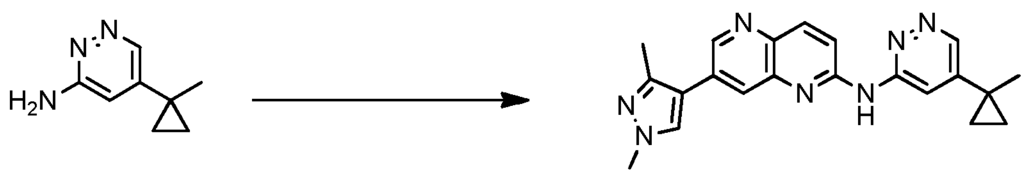

7-(1,3-Dimethyl-1H-pyrazol-4-yl)-N-(5-(1-methylcyclopropyl)pyridazin-3-yl)-1,5-naphthyridine-2-amine was

obtained in the same manner as in Example 0786 except that 5-(1-methylcyclopropyl)pyridazine-3-amine was used instead of the 5-(1-methoxypropan-2-yl)pyridazine-3-amine used in Example 0786.
$^1$H-NMR(DMSO-$d_6$)δ:10.68(1H,s),8.89(1H,d,J=2.1Hz),8.73(1H,d,J=2.1Hz),8.62(1H,d,J=2.1Hz),8.25(1H,s),8.24(1H,d,J =9.3Hz),8.01(1H,d,J=2.1Hz),7.73(1H,d,J=9.3Hz),3.84(3H,s),2.41(3H,s),1.51(3H,s),1.19-1.13(2H,m),1.06-1.01(2H,m).
MSm/z(M+H):372.

<Example 0886>

<0886-1>

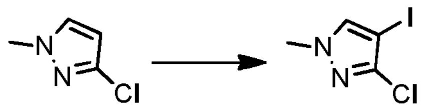

N-iodosuccinimide (225 mg) was added to a solution of 3-chloro-1-methyl-1H-pyrazole (116 mg) in acetonitrile (10 mL), followed by stirring at room temperature for 1 hour, and further stirring at 40°C for 3 hours. The solvent of the reaction mixture was distilled off under reduced pressure, and the obtained residue was purified by silica gel column chromatography (hexane-ethyl acetate), thereby obtaining 3-chloro-4-iodo-1-methyl-1H-pyrazole (84 mg) as colorless oily substance.
MSm/z(M+H):243.

<0886-2>

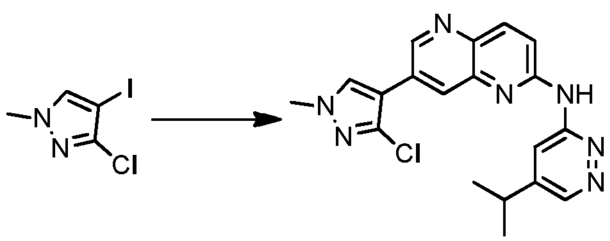

7-(3-Chloro-1-methyl-1H-pyrazol-4-yl)-N-(5-isopropylpyridazin-3-yl)-1,5-naphthyridine-2-amine was obtained as a pale yellow solid in the same manner as in Example 0935-3 except that 3-chloro-4-iodo-1-methyl-1H-pyrazole was used instead of the 1-(4-bromo-1-methyl-1H-pyrazol-3-yl)-4-methyl-1,4-diazepan-5-one used in Example 0935-3.
$^1$H-NMR($CDCl_3$)δ:8.93(1H,brs),8.89(1H,d,J=2.0Hz),8.72(1H,brs),8.40(1H,d,J=2.0Hz),8.22(1H,d,J=9.2H z),7.94(1H,s),7.56(1H,d,J=9.2Hz),3.99(3H,s),3.10-3.01(1H,m),1.42(6H,d,J=7.3Hz).
MSm/z(M+H):380.

<Example 0887>

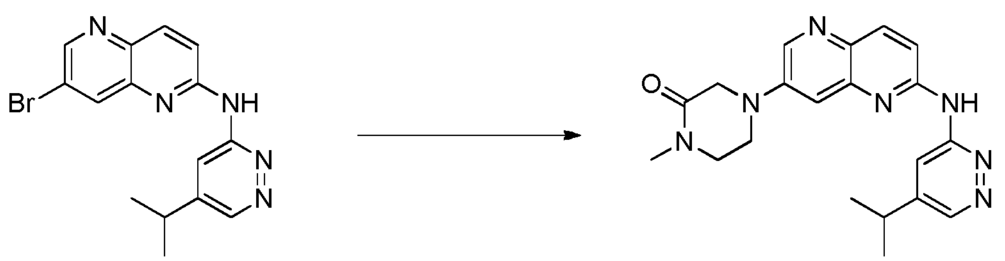

4-(6-((5-Isopropylpyridazin-3-yl)amino)-1,5-naphthyridin-3-yl)-1-methylpiperazin-2-one was obtained as a yellow solid in the same manner as in Example 0846 except that 1-methylpiperazin-2-one was used instead of the pyrrolidine used in Example 0846.
$^1$H-NMR($CDCl_3$)δ:9.69(1H,s),8.91-8.88(1H,m),8.83-8.80(1H,m),8.59(1H,d,J=2.6Hz),8.15(1H,d,J=9.2Hz),7.56(1H,d,J=9.2Hz),7.30(1H,d,J=2.6Hz),4.06(2H,s),3.74-3.71(2H,m),3.61-3.57(2H,m),3.11-3.00(1H,m),3.10(3H,s),1.42(6H,d,J=7.3Hz).
MSm/z(M+H):378.

<Example 0888>

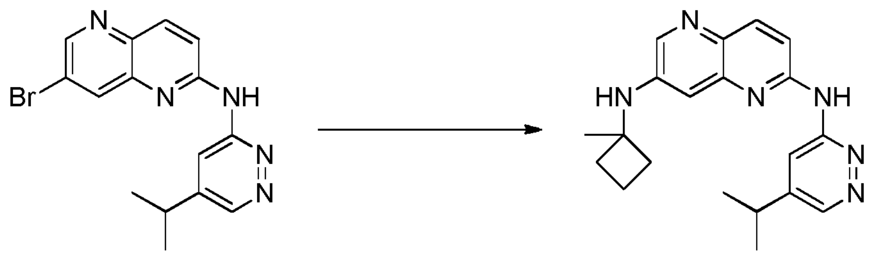

$N^2$-(5-isopropylpyridazin-3-yl)-$N^7$-(1-methylcyclobutyl)-1,5-naphthyridine-2,7-diamine was obtained as a yellow solid in the same manner as in Example 0846 except that 1-methylcyclobutaneamine hydrochloride was used instead of the pyrrolidine used in Example 0846, and 2-(dicyclohexylphosphino)-3,6-dimethoxy-2',4',6'-triisopropyl-1,1'-biphenyl was used instead of the 2-dicyclohexylphosphino-2',6'-diisopropoxybiphenyl used in Example 0846.
$^1$H-NMR($CDCl_3$)δ:8.77-8.75(1H,m),8.75-8.73(1H,m),8.46-8.43(1H,m),8.19(1H,d,J=2.6Hz),8.07(1H,d,J=9.2Hz),7.21(1H,d,J=9.2Hz),6.91(1H,d,J=2.6Hz),4.30(1H,s),3.04-2.95(1H,m),2.38-2.28(2H,m),2.24-2.15(2H,m),2.05-1.98(2H,m),1.58(3H,s),1.39(6H,d,J=6.5Hz).
MSm/z(M+H):349.

<Example 0889>

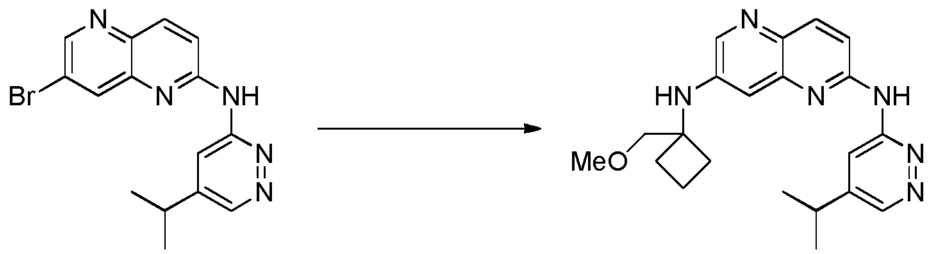

$N^2$-(5-isopropylpyridazin-3-yl)-$N^7$-(1-(methoxymethyl)cyclobutyl)-1,5-naphthyridine-2,7-diamine was obtained as a yellow solid in the same manner as in Example 0846 except that 1-(methoxymethyl)cyclobutaneamine hydrochloride was used instead of the pyrrolidine used in Example 0846, and 2-(dicyclohexylphosphino)-3,6-dimethoxy-2',4',6'-triisopropyl-1,1'-biphenyl was used instead of the 2-dicyclohexylphosphino-2',6'-diisopropoxybiphenyl used in Example 0846.
$^1$H-NMR($CDCl_3$)δ:8.69-8.68(1H,m),8.63-8.62(1H,m),8.16(1H,d,J=2.6Hz),8.10-8.06(1H,m),8.01(1H,d,J=9.2Hz),7.10(1H,d,J=9.2Hz),6.88(1H,d,J=2.6Hz),4.42-4.39(1H,m),3.62(2H,s),3.30(3H,s),2.97-2.88(1H,m),2.32-2.23(2H,m),2.25-2.18(2H,m),1.98-1.89(2H,m),1.31(6H,d,J=6.6Hz).
MSm/z(M+H):379.

<Example 0890>

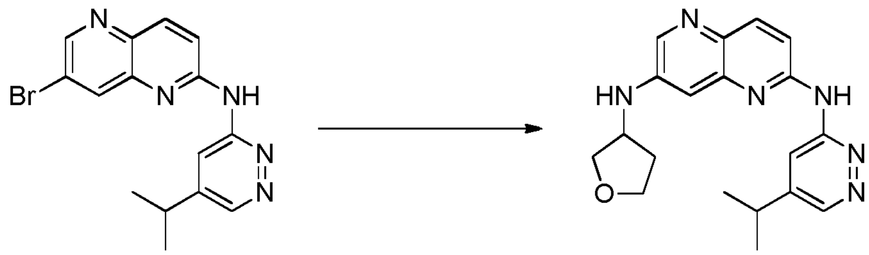

$N^2$-(5-isopropylpyridazin-3-yl)-$N^7$-(tetrahydrofuran-3-yl)-1,5-naphthyridine-2,7-diamine was obtained as a yellow solid in the same manner as in Example 0846 except that tetrahydrofuran-3-amine hydrochloride was used instead of the pyrrolidine used in Example 0846, and 2-(dicyclohexylphosphino)-3,6-dimethoxy-2',4',6'-triisopropyl-1,1'-biphenyl was used instead of the 2-dicyclohexylphosphino-2',6'-diisopropoxybiphenyl used in Example 0846.
$^1$H-NMR($CDCl_3$)$\delta$:9.00(1H,s),8.80-8.76(1H,m),8.78-8.74(1H,m),8.27(1H,d,J=2.0Hz),8.11(1H,d,J=8.6Hz),7.36(1H,d,J=8.6 Hz),7.00(1H,d,J=2.0Hz),4.36(1H ,d,J=6.6Hz),4.25-4.20(1H,m),4.08-4.01(1H,m),4.06-4.00(1H,m),3.95-3.88(1H,m),3.84(1H,dd,J=9.2,2.6Hz),3.07-2.98(1H,m),2.43-2.34(1H,m),2.03-1.96(1H,m),1.40(6H,d,J=6.6Hz).
MSm/z(M+H):351.

<Example 0891>

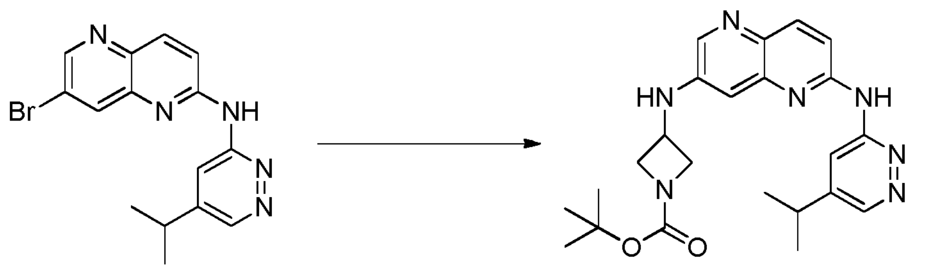

tert-Butyl 3-((6-((5-isopropylpyridazin-3-yl)amino)-1,5-naphthyridin-3-yl)amino)azetidine-1-carboxylate was obtained as a yellow solid in the same manner as in Example 0846 except that tert-butyl 3-aminoazetidine-1-carboxylate was used instead of the pyrrolidine used in Example 0846, and 2-(dicyclohexylphosphino)-3,6-dimethoxy-2',4',6'-triisopropyl-1,1'-biphenyl was used instead of the 2-dicyclohexylphosphino-2',6'-diisopropoxybiphenyl used in Example 0846.
$^1$H-NMR($CDCl_3$)$\delta$:8.80-8.77(1H,m),8.74-8.71(1H,m),8.71-8.68(1H,m),8.29(1H,d,J=2.0Hz),8.12(1H,d,J=8.6Hz),7.33(1H,d,J=8.6Hz),6.85(1H,d,J=2.0Hz),4.53(1H,d,J=5.3Hz),4.42-4.34(1H,m),4.41(2H,dd,J=8.6,8.6Hz),3.86(2H,dd,J=8.6,4.0Hz),3.06-2.98(1H,m),1,45(9H,s),1,40(6H,d,J=7.3Hz).
MSm/z(M+H):436.

<Example 0892>

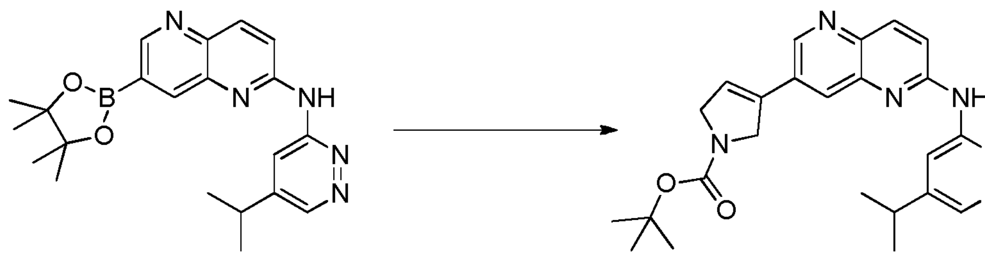

A mixture of N-(5-isopropylpyridazin-3-yl)-7-(4,4,5,5-tetramethyl-1,3,2-dioxaborolan-2-yl)-1,5-naphthyridine-2-amine (62 mg), tert-butyl 3-(((trifluoromethyl)sulfonyl)oxy)-2,5-dihydro-1H-pyrrole-1-carboxylate (50 mg), potassium phosphate (100 mg), (tris(dibenzylideneacetone)dipalladium(0) (15 mg), 2-dicyclohexylphosphino-2',4',6'-triisopropylbiphenyl (30 mg), tert-amyl alcohol (0.8 mL), and water (0.2 mL) was stirred at 100°C for 1 hour. The reaction mixture was cooled to room temperature, the solvent was distilled off under reduced pressure, and the obtained residue was purified by silica gel column chromatography (methanol-ethyl acetate), thereby obtaining tert-butyl 3-(6-((5-isopropylpyridazin-3-yl)amino)-1,5-naphthyridin-3-yl)-2,5-dihydro-1H-pyrrole-1-carboxylate (44 mg) as a yellow solid.
$^1$H-NMR($CDCl_3$)δ:8.98-8.95(1H,m),8.93-8.90(1H,m),8.89-8.86(1H,m),8.84-8.81(1H,m),8.24(1H,d,J=8.6Hz),7.88-7.85(1H,m),7.57(1H,d,J=8.6Hz),6.51-6.45(1H,m),4.68-4.63(2H,m),4.44-4.39(2H,m),3.10-3.01(1H,m),1.54(9H,s),1.41(6H,d,J=7.3Hz).
MSm/z(M+H):433.

<Example 0893>

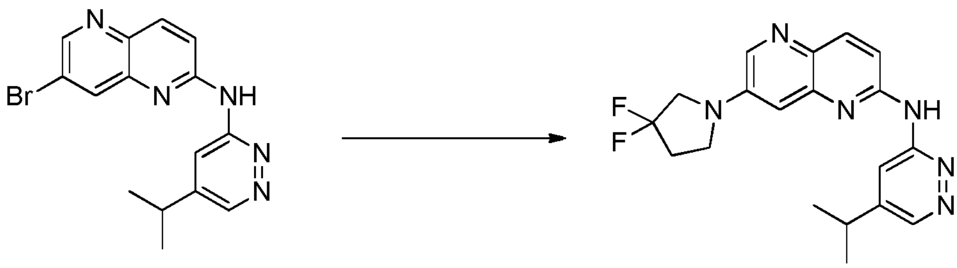

7-(3,3-Difluoropyrrolidin-1-yl)-N-(5-isopropylpyridazin-3-yl)-1,5-naphthyridine-2-amine was obtained as a yellow solid in the same manner as in Example 0846 except that 3,3-difluoropyrrolidine hydrochloride was used instead of the pyrrolidine used in Example 0846.
$^1$H-NMR($CDCl_3$)δ:8.89(1H,s),8.81-8.78(1H,m),8.79-8.76(1H,m),8.33(1H,d,J=2.6Hz),8.14(1H,d,J=9.2Hz),7.34(1H,d,J=9.2Hz),7.00(1H,d,J=2.6Hz),3.92-3.82(2H,m),3.78-3.72(2H,m),3.08-2.98(1H,m),2.67-2.53(2H,m),1.40(6H,d,J=6.5Hz).
MSm/z(M+H):371.

<Example 0894>

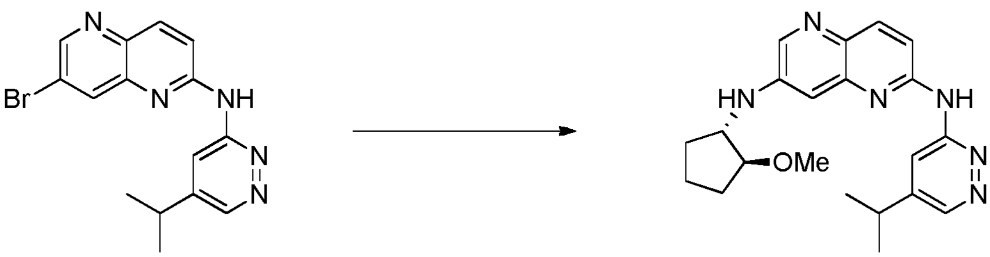

$N^2$-(5-isopropylpyridazin-3-yl)-$N^7$-((1S,2S)-2-methoxycyclopentyl)-1,5-naphthyridine-2,7-diamine was obtained as a yellow solid in the same manner as in Example 0846 except that (1S,2S)-2-methoxycyclopentaneamine was used instead of the pyrrolidine used in Example 0846, and 2-(dicyclohexylphosphino)-3,6-dimethoxy-2',4',6'-triisopropyl-1,1'-biphenyl was used instead of the 2-dicyclohexylphosphino-2',6'-diisopropoxybiphenyl used in Example 0846.
$^1$H-NMR($CDCl_3$)δ:8.83(1H,d,J=2.0Hz),8.76(1H,d,J=2.0Hz),8.26(1H,d,J=2.6Hz),8.22(1H,s),8.08(1H,d,J=8.6Hz),7.16(1H,d,J=2.6Hz),7.12(1H,d,J=8.6Hz),4.11(1H,d,J=5.9Hz),3.85-3.82(1H,m),3.74-3.71(1H,m),3.42(3H,s),3.04-2.97(1H,m),2.28-2.21(2H,m),2.02-1.93(2H,m),1.95-1.84(2H,m),1.39(6H,d,J=7.3Hz).
MSm/z(M+H):379.

<Example 0895>

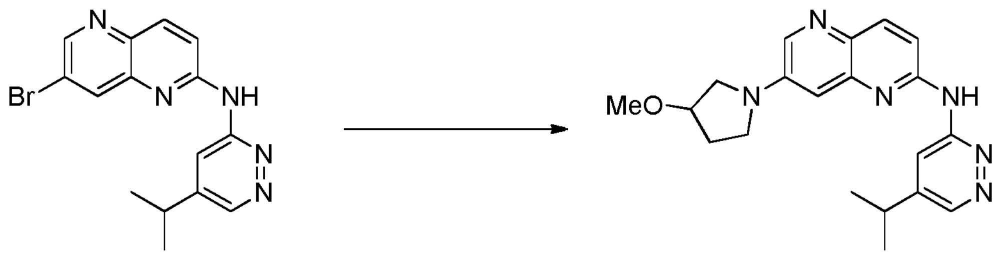

N-(5-isopropylpyridazin-3-yl)-7-(3-methoxypyrrolidin-1-yl)-1,5-naphthyridine-2-amine was obtained as a yellow solid in the same manner as in Example 0846 except that 3-methoxypyrrolidine hydrochloride was used instead of the pyrrolidine used in Example 0846.
$^1$H-NMR($CDCl_3$)δ:8.78-8.75(1H,m),8.77-8.75(1H,m),8.75-8.72(1H,m),8.36(1H,d,J=2.6Hz),8.10(1H,d,J=8.6Hz),7.22(1H,d,J=8.6Hz),6.97(1H,d,J=2.6Hz),4.22-4.16(1H,m),3.68-3.59(2H,m),3.59-3.51(2H,m),3.42(3H,s),3.05-2.98(1H,m),2.29-2.21(2H,m),1.39(6H,d,J=6.6Hz).
MSm/z(M+H):365.

<Example 0896>

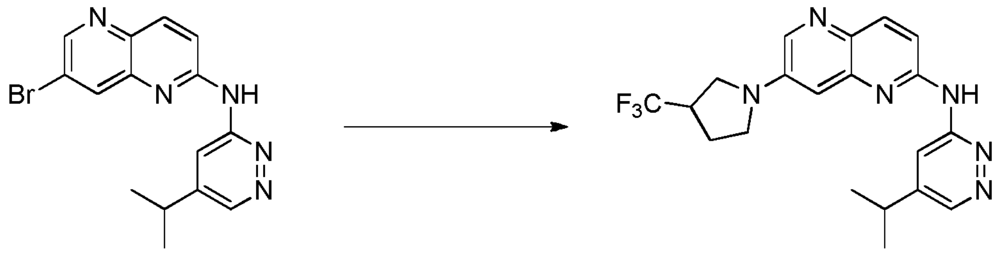

N-(5-isopropylpyridazin-3-yl)-7-(3-(trifluoromethyl)pyrrolidin-1-yl)-1,5-naphthyridine-2-amine was obtained as a yellow solid in the same manner as in Example 0846 except that 3-(trifluoromethyl)pyrrolidine hydrochloride was used instead of the pyrrolidine used in Example 0846.
$^1$H-NMR($CDCl_3$)δ:9.52(1H,s),8.87-8.84(1H,m),8.80(1H,d,J=2.0Hz),8.36(1H,d,J=2.0Hz),8.13(1H,d,J=9.2Hz),7.47-7.41(1H,m),7.02(1H,d,J=2.0Hz),3.79-3.72(2H,m),3.68-3.55(2H,m),3.22-3.13(1H,m),3.09-3.00(1H,m),2.43-2.26(2H,m),1.41(6H,d,J=6.6Hz).
MSm/z(M+H):403.

<Examples 0897 and 0898>

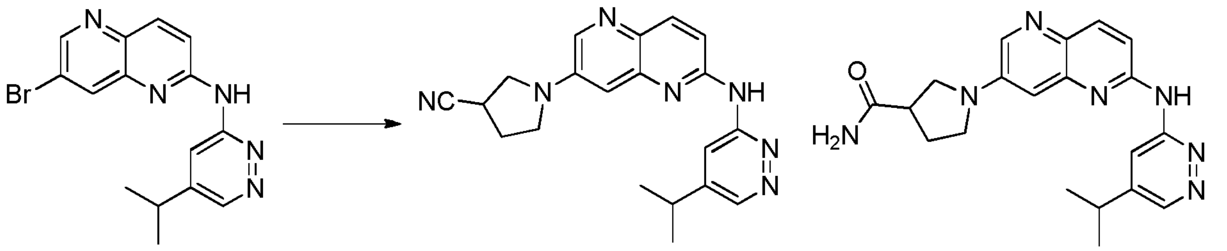

1-(6-((5-Isopropylpyridazin-3-yl)amino)-1,5-naphthyridin-3-yl)pyrrolidine-3-carbonitrile as a yellow solid and 1-(6-((5-isopropylpyridazin-3-yl)amino)-1,5-naphthyridin-3-yl)pyrrolidine-3-carboxamide as a yellow solid were obtained in the same manner as in Example 0846 except that pyrrolidine-3-carbonitrile hydrochloride was used instead of the pyrrolidine used in Example 0846.

<Example 0897>

1-(6-((5-Isopropylpyridazin-3-yl)amino)-1,5-naphthyridin-3-yl)pyrrolidine-3-carbonitrile
$^1$H-NMR($CDCl_3$)δ:8.80-8.76(1H,m),8.73-8.70(1H,m),8.40-8.36(1H,m),8.37-8.34(1H,m),8.13(1H,d,J=9.2Hz),7.23(1H,d,J=7.9Hz),7.03-7.00(1H,m),3.89-3.78(2H,m),3.79-3.72(1H,m),3.69-3.58(1H,m),3.42-3.34(1H,m),3.05-2.98(1H,m),2.56-2.47(2H,m),1.39(6H,d,J=7.3Hz).
MSm/z(M+H):360.

<Example 0898>

1-(6-((5-Isopropylpyridazin-3-yl)amino)-1,5-naphthyridin-3-yl)pyrrolidine-3-carboxamide
$^1$H-NMR($CDCl_3$)δ:8.77-8.73(1H,m),8.63-8.60(1H,m),8.62-8.58(1H,m),8.36-8.33(1H,m),8.09(1H,d,J=9.2Hz),7.21(1H,d,J=9.2Hz),6.99-6.96(1H,m),5.72-5.68(1H,m),5.57-5.53(1H,m),3.79-3.72(2H,m),3.71-3.67(1H,m),3.59-3.51(1H,m),3.25-3.15(1H,m),3.06-2.98(1H,m),2.44-2.37(2H,m),1.38(6H,d,J=6.6Hz).
MSm/z(M+H):378.

<Example 0899>

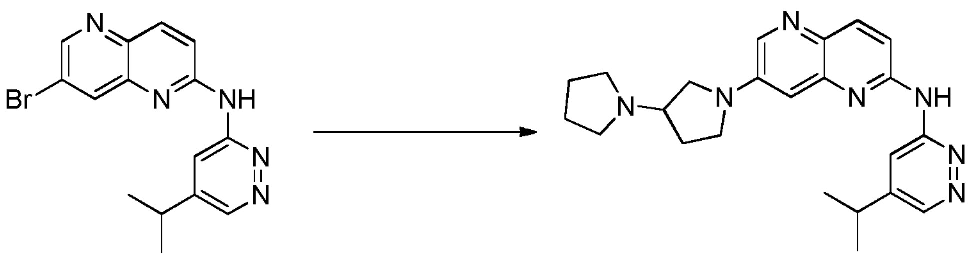

7-([1,3'-Bipyrrolidine]-1'-yl)-N-(5-isopropylpyridazin-3-yl)-1,5-naphthyridine-2-amine was obtained as a yellow solid in the same manner as in Example 0846 except that 1,3'-bipyrrolidine was used instead of the pyrrolidine used in Example 0846.
$^1$H-NMR($CDCl_3$)δ:9.16(1H,s),8.79-8.76(1H,m),8.78(1H,d,J=2.6Hz),8.34(1H,d,J=2.6Hz),8.10(1H,d,J=9.2Hz),7.33(1H,d,J=9.2Hz),6.95(1H,d,J=2.6Hz),3.71-3.61(2H,m),3.54-3.47(1H,m),3.42-3.35(1H,m),3.07-2.93(2H,m),2.69-2.60(4H,m),2.31-2.26(1H,m),2.14-2.03(1H,m),1.88-1.84(4H,m),1.39(6H,d,J=7.3Hz).
MSm/z(M+H):404.

<Example 0900>

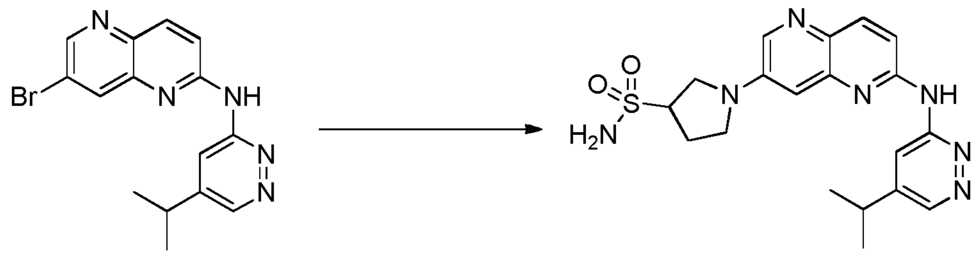

1-(6-((5-Isopropylpyridazin-3-yl)amino)-1,5-naphthyridin-3-yl)pyrrolidine-3-sulfonamide was obtained as a yellow solid in the same manner as in Example 0846 except that pyrrolidine-3-sulfonamide was used instead of the pyrrolidine used in Example 0846.
$^1$H-NMR($CD_3OD$)δ:8.80-8.76(1H,m),8.76-8.72(1H,m),8.34(1H,d,J=2.6Hz),8.04(1H,d,J=9.2Hz),7.33(1H,d,J=9.2Hz),7.11(1H,d,J=2.6Hz),4.09-4.01(1H,m),3.92-3.86(2H,m),3.77-3.71(1H,m),3.62-3.59(1H,m),3.08-3.02(1H,m),2.60-2.54(2H,m),1.40(6H,d,J=7.6Hz).
MSm/z(M+H):414.

<Example 0901>

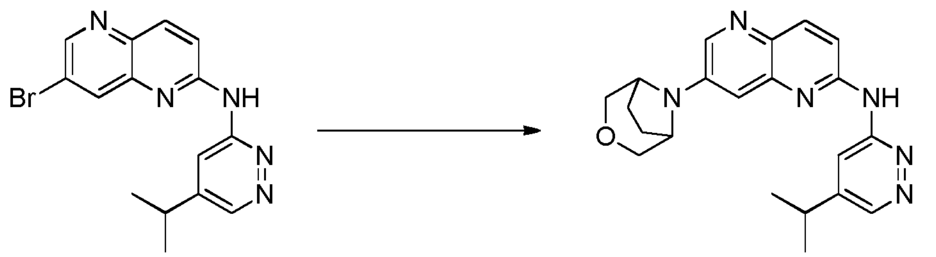

7-(3-Oxa-8-azabicyclo[3.2.1]octan-8-yl)-N-(5-isopropylpyridazin-3-yl)-1,5-naphthyridine-2-amine was obtained as a yellow solid in the same manner as in Example 0846 except that 3-oxa-8-azabicyclo[3.2.1]octane was used instead of the pyrrolidine used in Example 0846.
$^1$H-NMR($CDCl_3$)δ:8.82-8.78(1H,m),8.80-8.76(1H,m),8.70-8.67(1H,m),8.51(1H,d,J=2.0Hz),8.12(1H,d,J=8.6Hz),7.28(1H,d,J=8.6Hz),7.19(1H,d,J=2.0Hz),4.28(2H,s),3.97(2H,d,J=11.2Hz),3.63(2H,d,J=11.2Hz),3.06-2.96(1H,m),2.21-2.05(4H,m),1.39(6H,d,J=6.6Hz).
MSm/z(M+H):377.

<Example 0902>

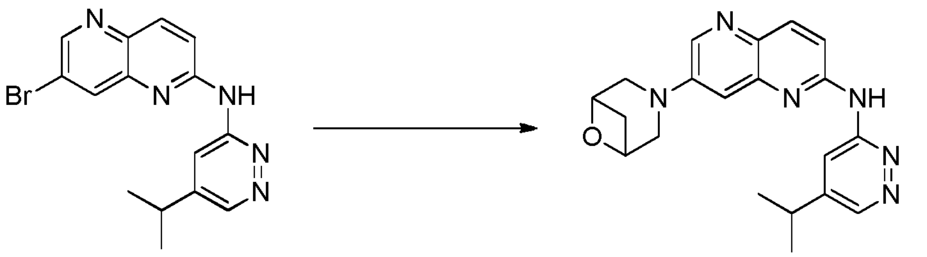

7-(6-Oxa-3-azabicyclo[3.1.1]heptan-3-yl)-N-(5-isopropylpyridazin-3-yl)-1,5-naphthyridine-2-amine was obtained as a yellow solid in the same manner as in Example 0846 except that 6-oxa-3-azabicyclo[3.1.1]heptane was used instead of the pyrrolidine used in Example 0846.
$^1$H-NMR($CDCl_3$)δ:9.63-9.60(1H,m),8.89-8.85(1H,m),8.80-8.77(1H,m),8.56-8.53(1H,m),8.15(1H,d,J=8.6Hz),7.47(1H,d,J=8.6Hz),7.20-7.17(1H,m),4.86(2H,d,J=7.6Hz),3.82-3.72(4H,m),3.36(1H,dt,J=8.6,7.6Hz),3.06-2.98(1H,m),2.10(1H,d,J=8.6Hz),1.40(6H,d,J=7.3Hz).
MSm/z(M+H):363.

<Example 0903>

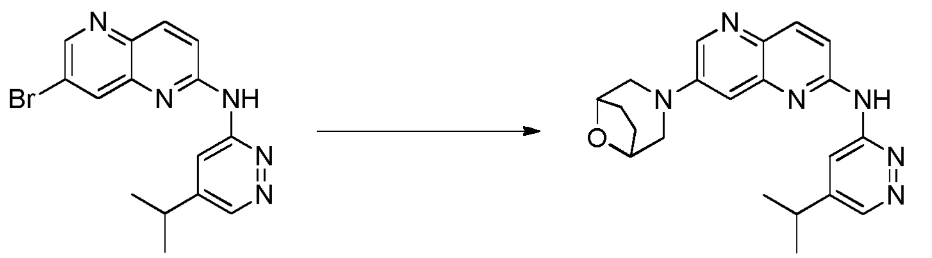

7-(8-Oxa-3-azabicyclo[3.2.1]octan-3-yl)-N-(5-isopropylpyridazin-3-yl)-1,5-naphthyridine-2-amine was obtained as a yellow solid in the same manner as in Example 0846 except that 8-oxa-3-azabicyclo[3.2.1]octane was used instead of the pyrrolidine used in Example 0846.
$^1$H-NMR($CDCl_3$)δ:9.40(1H,s),8.85-8.81(1H,m),8.81-8.79(1H,m),8.55(1H,d,J=2.6Hz),8.14(1H,d,J=9.2Hz),7.47(1H,d,J=9.2Hz),7.22(1H,d,J=2.6Hz),4.60-4.57(2H,m),3.52(2H,d,J=11.9Hz),3.22(2H,dd,J=11.9,2.3Hz),3.09-2.99(1H,m),2.09-1.99(4H,m),1.40(6H,d,J=7.0Hz).
MSm/z(M+H):377.

<Example 0904>

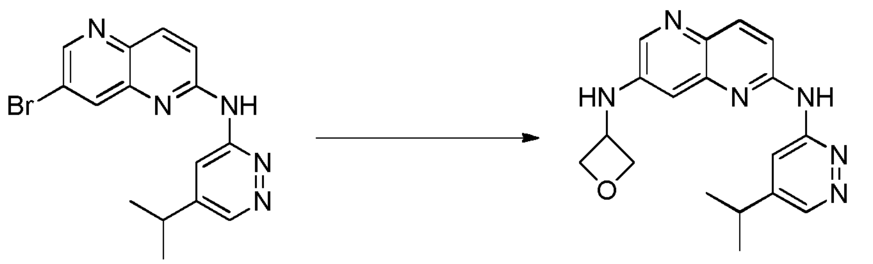

$N^2$-(5-isopropylpyridazin-3-yl)-$N^7$-(oxetan-3-yl)-1,5-naphthyridine-2,7-diamine was obtained as a yellow solid in the same manner as in Example 0846 except that oxetane-3-amine was used instead of the pyrrolidine used in

Example 0846, and 2-(dicyclohexylphosphino)-3,6-dimethoxy-2',4',6'-triisopropyl-1,1'-biphenyl was used instead of the 2-dicyclohexylphosphino-2',6'-diisopropoxybiphenyl used in Example 0846.
$^1$H-NMR($CDCl_3$)δ:9.63(1H,s),8.85-8.82(1H,m),8.82-8.78(1H,m),8.30(1H,d,J=2.6Hz),8.12(1H,d,J=9.2Hz),7.50(1H,d,J=9.2Hz),6.82(1H,d,J=2.6Hz),5.10(2H,dd,J=8.6,8.6Hz),4.81-4.73(1H,m),4.72-4.63(1H,m),4.67-4.61(2H,m),3.08-2.99(1H,m),1.41(6H,d,J=7.3Hz).
MSm/z(M+H):337.

<Example 0905>

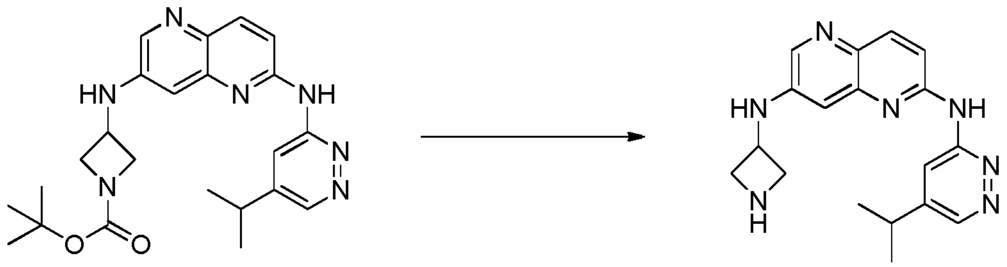

A 4 mol/L hydrogen chloride/1,4-dioxane solution (2 mL) and methanol (2 mL) were added to tert-butyl 3-((6-((5-isopropylpyridazin-3-yl)amino)-1,5-naphthyridin-3-yl)amino)azetidine-1-carboxylate (25 mg), followed by stirring at room temperature for 20 minutes. A saturated sodium hydrogen carbonate aqueous solution was added thereto, the solvent was distilled off under reduced pressure, and the obtained residue was purified by silica gel column chromatography (ethyl acetate-methanol, NH silica), thereby obtaining $N^7$-(azetidin-3-yl)-$N^2$-(5-isopropylpyridazin-3-yl)-1,5-naphthyridine-2,7-diamine (3 mg) as a pale yellow solid.
$^1$H-NMR($CDCl_3$)δ:8.90(1H,s),8.80-8.78(1H,m),8.78-8.76(1H,m),8.27(1H,d,J=2.6Hz),8.10(1H,d,J=9.2Hz),7.32(1H,d,J=9.2Hz),6.89(1H,d,J=2.6Hz),4.58-4.54(1H,m),4.54-4.48(1H,m),4.09-4.02(2H,m),3.65-3.59(2H,m),3.63-3.61(1H,m),3.07-2.97(1H,m),1.40(6H,d,J=6.6Hz).
MSm/z(M+H):336.

<Example 0906>

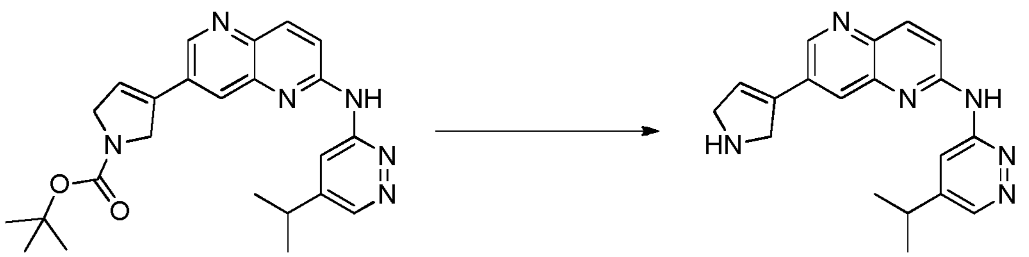

7-(2,5-Dihydro-1H-pyrrol-3-yl)-N-(5-isopropylpyridazin-3-yl)-1,5-naphthyridine-2-amine was obtained as a yellow solid in the same manner as in Example 0905 except that tert-butyl 3-(6-((5-isopropylpyridazin-3-yl)amino)-1,5-naphthyridin-3-yl)-2,5-dihydro-1H-pyrrole-1-carboxylate was used instead of the tert-butyl 3-((6-((5-isopropylpyridazin-3-yl)amino)-1,5-naphthyridin-3-yl)amino)azetidine-1-carboxylate used in Example 0905.
$^1$H-NMR($CDCl_3$)δ:10.64(1H,s),9.02-9.00(1H,m),8.93(1H,d,J=2.0Hz),8.84(1H,d,J=2.0Hz),8.25(1H,d,J=9.2Hz),7.94(1H,d,J=9.2Hz),7.86(1H,d,J=2.0Hz),6.57(1H,t,J=2.0Hz),4.31-4.26(2H,m),4.09-4.05(2H,m),3.12-3.03(1H,m),1.88-1.85(1H,m),1.43(6H,d,J=6.6Hz).
MSm/z(M+H):333.

<Example 0907>

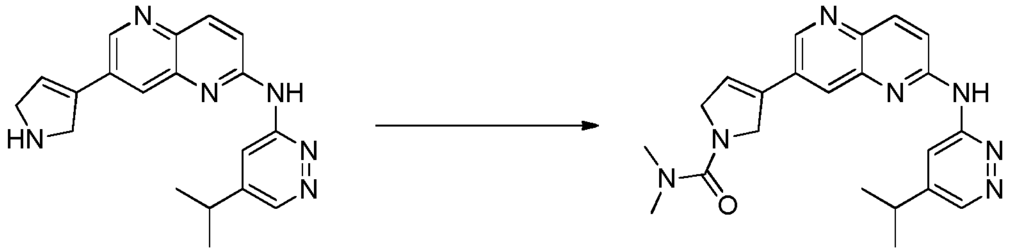

3-(6-((5-Isopropylpyridazin-3-yl)amino)-1,5-naphthyridin-3-yl)-N,N-dimethyl-2,5-dihydro-1H-pyrrole-1-carboxamide was obtained as a yellow solid in the same manner as in Example 0882 except that 7-(2,5-dihydro-1H-pyrrol-3-yl)-N-(5-isopropylpyridazin-3-yl)-1,5-naphthyridine-2-amine was used instead of the N-(5-isopropylpyridazin-3-yl)-7-(1,2,3,6-tetrahydropyridin-4-yl)-1,5-naphthyridine-2-amine used in Example 0882, and dimethylcarbamoyl chloride was used instead of the acetyl chloride used in Example 0882.

$^1$H-NMR($CDCl_3$)δ:9.81(1H,s),8.94(1H,d,J=2.0Hz),8.93-8.89(1H,m),8.84(1H,d,J=2.0Hz),8.25(1H,d,J=9.2Hz),7.92(1H,d,J=2.0Hz),7.78(1H,d,J=9.2Hz),6.47-6.43(1H,m),4.82-4.77(2H,m),4.52-4.49(2H,m),3.15-3.05(1H,m),2.96(6H,s),1.43(6H,d,J=7.3Hz).

MSm/z(M+H):404.

<Example 0908>

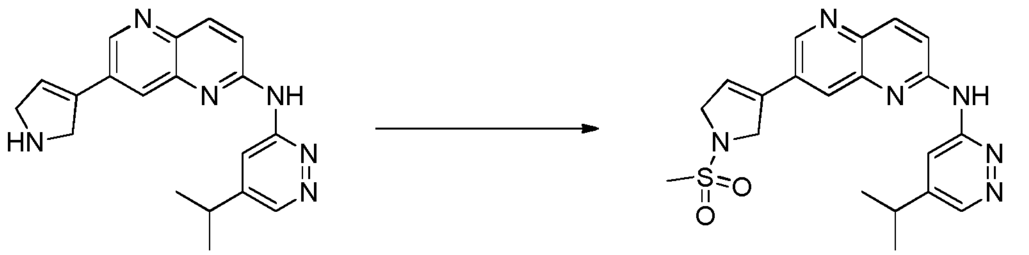

N-(5-Isopropylpyridazin-3-yl)-7-(1-(methylsulfonyl)-2,5-dihydro-1H-pyrrol-3-yl)-1,5-naphthyridine-2-amine was obtained as a yellow solid in the same manner as in Example 0839 except that 7-(2,5-dihydro-1H-pyrrol-3-yl)-N-(5-isopropylpyridazin-3-yl)-1,5-naphthyridine-2-amine was used instead of the $N^2$-(5-isopropylpyridazin-3-yl)-1,5-naphthyridine-2,7-diamine used in Example 0839, and methanesulfonyl chloride was used instead of the paratoluenesulphonyl chloride used in Example 0839.

$^1$H-NMR($CDCl_3$)δ:8.93(1H,d,J=2.0Hz),8.83(1H,d,J=2.0Hz),8.77(1H,d,J=2.0Hz),8.74-8.70(1H,m),8.25(1H,d,J=8.6Hz),7.86-7.84(1H,m),7.56(1H,d,J=8.6Hz),6.50-6.47(1H,m),4.74-4.70(2H,m),4.50-4.46(2H,m),3.14-3.02(1H,m),2.97(3H,s),1.42(6H,d,J=6.9Hz).

MSm/z(M+H):411.

<Example 0909>

<0909-1>

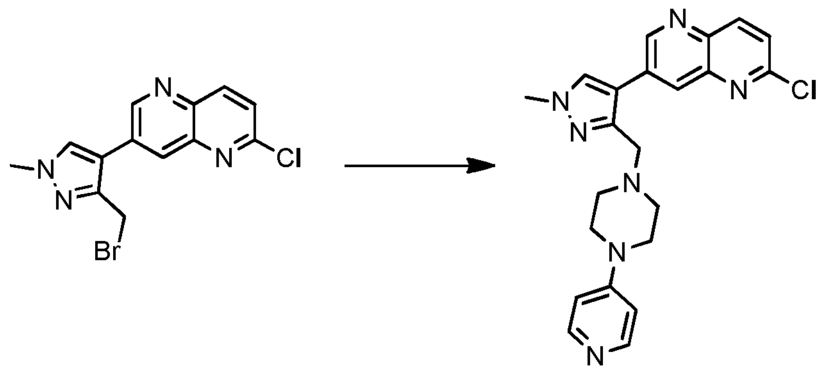

2-Chloro-7-(1-methyl-3-((4-(pyridin-4-yl)piperazin-1-yl)methyl)-1H-pyrazol-4-yl)-1,5-naphthyridine was obtained as a white solid in the same manner as in Example 0799-1 except that 1-(pyridin-4-yl)piperazine was used instead of the pyridin-3-ol used in Example 0799-1.
MSm/z(M+H):420.

<0909-2>

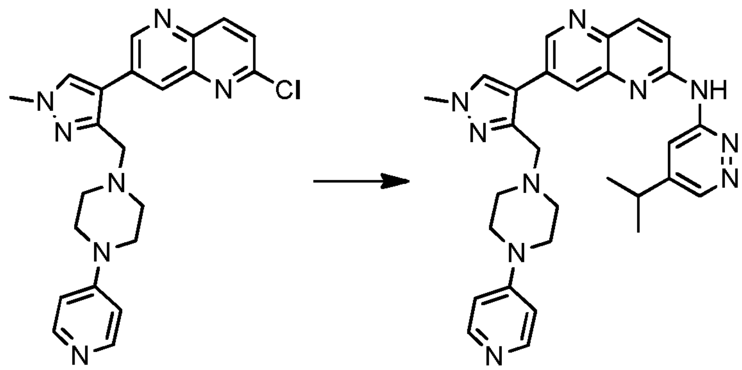

N-(5-isopropylpyridazin-3-yl)-7-(1-methyl-3-((4-(pyridin-4-yl)piperazin-1-yl)methyl)-1H-pyrazol-4-yl)-1,5-naphthyridine-2-amine was obtained as a yellow solid in the same manner as in Example 0799-2 except that 2-chloro-7-(1-methyl-3-((4-(pyridin-4-yl)piperazin-1-yl)methyl)-1H-pyrazol-4-yl)-1,5-naphthyridine was used instead of the 2-chloro-7-(1-methyl-3-((pyridin-3-yloxy)methyl)-1H-pyrazol-4-yl)-1,5-naphthyridine used in Example 0799-2.
$^1$H-NMR($CDCl_3$)δ:9.07(1H,s),8.80-8.77(1H,m),8.28-8.25(4H,m),7.72-7.66(2H,m),6.67-6.62(2H,m),3.99(3H,d,J=7.9Hz),3.70(2H,s),3.36-3.32(4H,m),3.02-2.94(1H,m),2.71-2.68(4H,m),1.34(6H,d,J=6.6Hz).
MSm/z(M+H):521.

<Example 0910>

<0910-1>

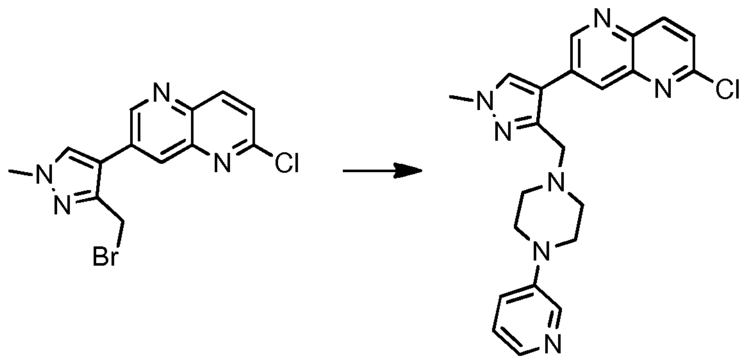

2-Chloro-7-(1-methyl-3-((4-(pyridin-3-yl)piperazin-1-yl)methyl)-1H-pyrazol-4-yl)-1,5-naphthyridine was obtained as a white solid in the same manner as in Example 0799-1 except that 1-(pyridin-3-yl)piperazine was used instead of the pyridin-3-ol used in Example 0799-1.
MSm/z(M+H):420.

<0910-2>

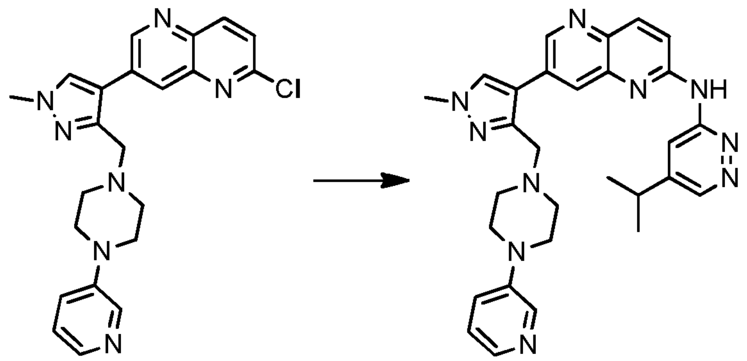

N-(5-isopropylpyridazin-3-yl)-7-(1-methyl-3-((4-(pyridin-3-yl)piperazin-1-yl)methyl)-1H-pyrazol-4-yl)-1,5-naphthyridine-2-amine was obtained as a yellow solid in the same manner as in Example 0799-2 except that 2-chloro-7-(1-methyl-3-((4-(pyridin-3-yl)piperazin-1-yl)methyl)-1H-pyrazol-4-yl)-1,5-naphthyridine was used instead of the 2-chloro-7-(1-methyl-3-((pyridin-3-yloxy)methyl)-1H-pyrazol-4-yl)-1,5-naphthyridine used in Example 0799-2.
$^1$H-NMR($CDCl_3$)δ:9.09(1H,d,J=2.0Hz),8.79(1H,d,J=2.0Hz),8.70(1H,s),8.53(2H,d,J=2.0Hz),8.30-8.27(1H,m),8.09-8.08(1H,m),7.71(1H,s),7.16-7.13(3H,m),6.55(1H,d,J=1.3Hz),4.01(3H,s),3.72(2H,s),3.24-3.22(4H,m),2.98-2.96(1H,m),2.76-2.75(4H,m),1.34(6H,d,J=7.3Hz).
MSm/z(M+H):521.

<Example 0911>

<0911-1>

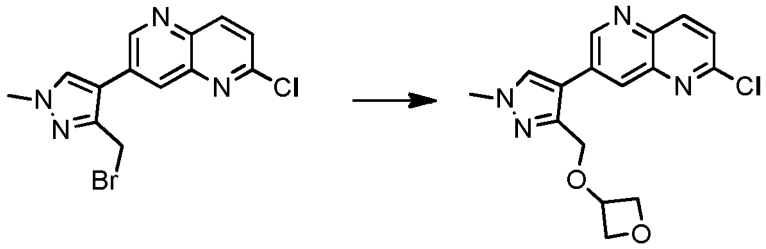

60% sodium hydride (5.8 mg) was added to a solution of oxetan-3-ol (6.5 mg) in N,N-dimethylformamide (1 mL) in a nitrogen atmosphere, followed by stirring for 30 minutes. 7-(3-(Bromomethyl)-1-methyl-1H-pyrazol-4-yl)-2-chloro-1,5-naphthyridine (20 mg) was added to the reaction mixture, followed by stirring at room temperature for 1 hour. The reaction mixture was purified by silica gel column chromatography (hexane-ethyl acetate-methanol, NH silica), thereby obtaining 2-chloro-7-(1-methyl-3-((oxetan-3-yloxy)methyl)-1H-pyrazol-4-yl)-1,5-naphthyridine (9.6 mg) as a pale yellow solid.
MSm/z(M+H):331.

<0911-2>

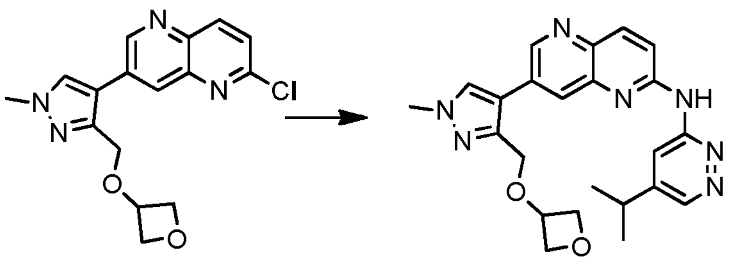

N-(5-isopropylpyridazin-3-yl)-7-(1-methyl-3-((oxetan-3-yloxy)methyl)-1H-pyrazol-4-yl)-1,5-naphthyridine-2-amine was obtained as a pale yellow solid in the same manner as in Example 0799-2 except that 2-chloro-7-(1-methyl-3-((oxetan-3-yloxy)methyl)-1H-pyrazol-4-yl)-1,5-naphthyridine was used instead of the 2-chloro-7-(1-methyl-3-((pyridin-3-yloxy)methyl)-1H-pyrazol-4-yl)-1,5-naphthyridine used in Example 0799-2, and 1,4-dioxane and 2-methyltetrahydrofuran were used instead of the 1,4-dioxane used in Example 0799-2.
$^1$H-NMR($CDCl_3$)δ:8.92(1H,d,J=2.0Hz),8.86(1H,s),8.81(1H,s),8.63(1H,brs),8.33(1H,s),8.27(1H,d,J=8.6Hz ),7.73(1H,s),7.55-7.52(1H,m),4.83-4.62(5H,m),4.60(2H,s),4.01(3H,s),3.08-2.99(1H,m),1.41(6H,d,J=7.3Hz).
MSm/z(M+H):432.

<Example 0912>

<0912-1>

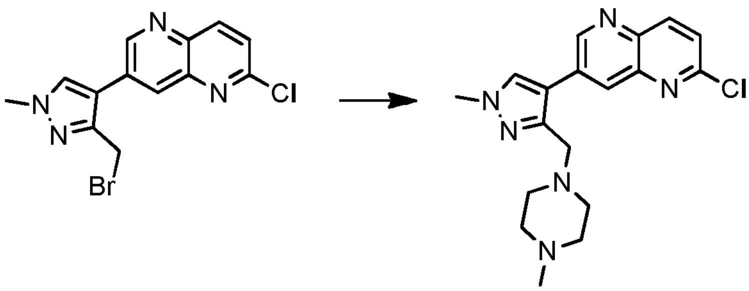

2-Chloro-7-(1-methyl-3-((4-methylpiperazin-1-yl)methyl)-1H-pyrazol-4-yl)-1,5-naphthyridine was obtained as a white solid in the same manner as in Example 0799-1 except that 1-methylpiperazine was used instead of the pyridin-3-ol used in Example 0799-1.
MSm/z(M+H):357.

<0912-2>

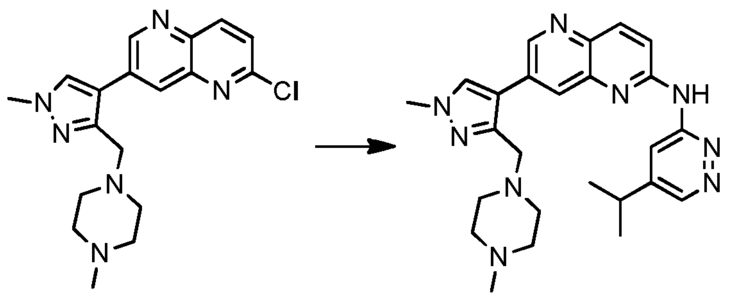

N-(5-isopropylpyridazin-3-yl)-7-(1-methyl-3-((4-methylpiperazin-1-yl)methyl)-1H-pyrazol-4-yl)-1,5-naphthyridine-2-amine was obtained as a pale yellow solid in the same manner as in Example 0799-2 except that 2-chloro-7-(1-methyl-3-((4-methylpiperazin-1-yl)methyl)-1H-pyrazol-4-yl)-1,5-naphthyridine was used instead of the 2-chloro-7-(1-methyl-3-((pyridin-3-yloxy)methyl)-1H-pyrazol-4-yl)-1,5-naphthyridine used in Example 0799-2.
$^1$H-NMR($CDCl_3$)δ:9.11(1H,d,J=2.0Hz),8.81(1H,s),8.75-8.72(1H,m),8.27(1H,d,J=9.2Hz),8.20(1H,s),7.70(1H,s),7.50-7.47(1H,m),3.99(3H,s),3.66(2H,s),3.04-3.02(1H,m),2.61-2.47(8H,m),2.27(3H,s),1.40(6H,d,J=7.3Hz).
MSm/z(M+H):458.

<Example 0913>

<0913-1>

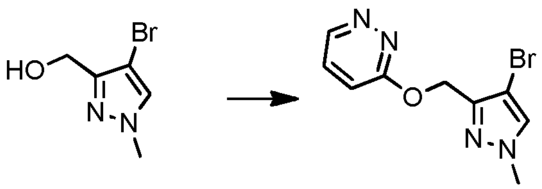

3-((4-Bromo-1-methyl-1H-pyrazol-3-yl)methoxy)pyridazine was obtained as a white solid in the same manner as in Example 0809-2 except that (4-bromo-1-methyl-1H-pyrazol-3-yl)methanol was used instead of the 3-((4-(6-chloro-1,5-naphthyridin-3-yl)-1-methyl-1H-pyrazol-3-yl)methoxy)phenol used in Example 0809-2, and pyridazin-3(2H)-one was used instead of the 2-(dimethylamino)ethanol used in Example 0809-2.
MSm/z(M+H):269.

<0913-2>

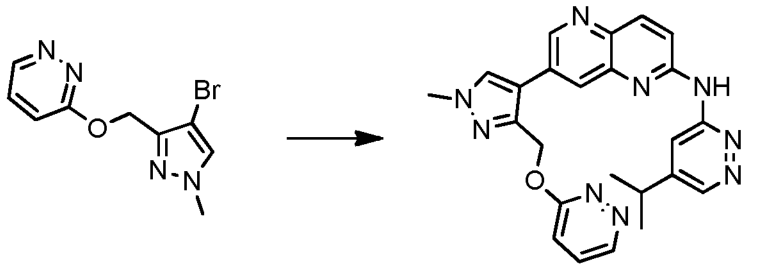

N-(5-isopropylpyridazin-3-yl)-7-(1-methyl-3-((pyridazin-3-yloxy)methyl)-1H-pyrazol-4-yl)-1,5-naphthyridine-2-amine was obtained as a yellow solid in the same manner as in Example 0797-2 except that 3-((4-bromo-1-methyl-1H-pyrazol-3-yl)methoxy)pyridazine was used instead of the 4-iodo-1-(3-(3-methoxy-3-methylazetidin-1-yl)propyl)-3-methyl-1H-pyrazole used in Example 0797-2.
$^1$H-NMR($CDCl_3$)δ:8.89(1H,s),8.81(2H,d,J=7.3Hz),8.26-8.24(2H,m),7.78-7.48(3H,m),7.12-7.09(1H,m),6.90-6.88(1H,m),5.57(2H,s),3.97(3H,s),3.06-3.00(1H,m),1.40(6H,d,J=6.6Hz).
MSm/z(M+H):454.

<Examples 0914 and 0915>

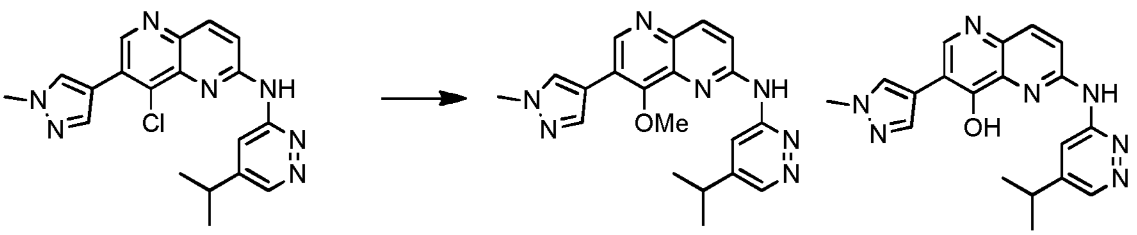

N-(5-isopropylpyridazin-3-yl)-8-methoxy-7-(1-methyl-1H-pyrazol-4-yl)-1,5-naphthyridine-2-amine as a pale yellow solid and 6-((5-isopropylpyridazin-3-yl)amino)-3-(1-methyl-1H-pyrazol-4-yl)-1,5-naphthyridin-4-ol as a pale yellow solid were obtained in the same manner as in Example 0355-1 except that 8-chloro-N-(5-isopropylpyridazin-3-yl)-7-(1-methyl-1H-pyrazol-4-yl)-1,5-naphthyridine-2-amine was used instead of the N-(8-chloro-7-(1-methyl-1H-pyrazol-4-yl)-1,5-naphthyridin-2-yl)-5-cyclopentyl-1,3,4-thiadiazole-2-amine used in Example 0355-1.

<Example 0914>

N-(5-isopropylpyridazin-3-yl)-8-methoxy-7-(1-methyl-1H-pyrazol-4-yl)-1,5-naphthyridine-2-amine
$^1$H-NMR($CD_3OD$)δ:8.95(2H,s),8.78(1H,s),8.31(1H,s),8.28(1H,brs),8.19(1H,d,J=9.2Hz),8.13(1H,s),7.60-7.57(1H,m),4.23(3H,s),4.00(3H,s),3.09-3.06(1H,m),1.42(6H,d,J=3.3Hz).
MSm/z(M+H):376.

<Example 0915>

6-((5-Isopropylpyridazin-3-yl)amino)-3-(1-methyl-1H-pyrazol-4-yl)-1,5-naphthyridine-4-ol
$^1$H-NMR($CD_3OD$)δ:8.71(1H,s),8.52(1H,brs),8.41(2H,s),8.02(3H,d,J=17.2Hz),3.96(3H,s),3.10-3.03(1H,m),1.39(6H,d,J=6.6 Hz).
MSm/z(M+H):362.

<Example 0916>

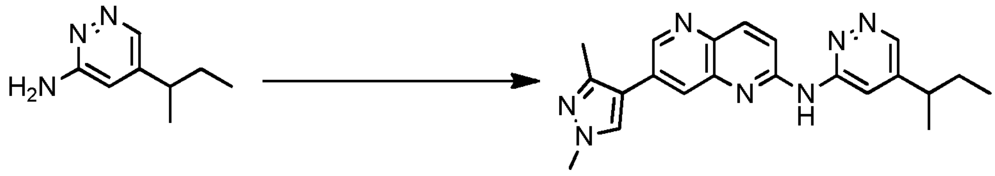

N-(5-(butan-2-yl)pyridazin-3-yl)-7-(1,3-dimethyl-1H-pyrazol-4-yl)-1,5-naphthyridine-2-amine was obtained in the same manner as in Example 0769 except that 5-(butan-2-yl)pyridazine-3-amine was used instead of the 5-(pentan-3-yl)pyridazine-3-amine used in Example 0769.
$^1$H-NMR($CDCl_3$)δ:8.84(1H,d,J=1.8Hz),8.79(1H,brs),8.76(1H,brs),8.26(1H,d,J=9.0Hz),8.05(1H,brs),7.65( 1H,s),7.58(1H,d, J=9.0Hz),3.95(3H,s),2.84-2.68(1H,m),2.51(3H,s),1.82-1.68(2H,m),1.38(3H,d,J=6.6Hz),0.96(3H,t,J=7.2Hz).
MSm/z(M+H):374.

<Example 0917>

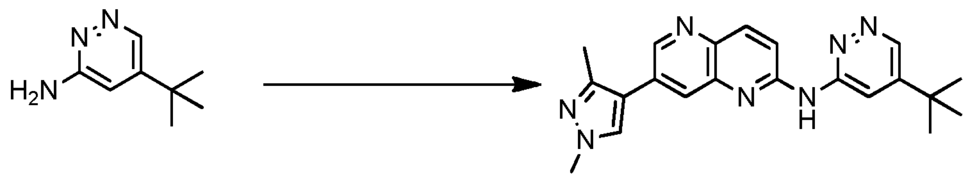

N-(5-(tert-butyl)pyridazin-3-yl)-7-(1,3-dimethyl-1H-pyrazol-4-yl)-1,5-naphthyridine-2-amine was obtained in the same manner as in Example 0769 except that 5-(tert-butyl)pyridazine-3-amine was used instead of the 5-(pentan-3-yl)pyridazine-3-amine used in Example 0769.
$^1$H-NMR($CDCl_3$)δ:9.04(1H,brs),8.96(1H,brs),8.84(1H,d,J=1.8Hz),8.26(1H,d,J=9.3Hz),8.04(1H,brs),7.65( 1H,s),7.58(1H,d, J=9.3Hz),3.95(3H,s),2.50(3H,s),1.46(9H,s).
MSm/z(M+H):374.

<Example 0918>

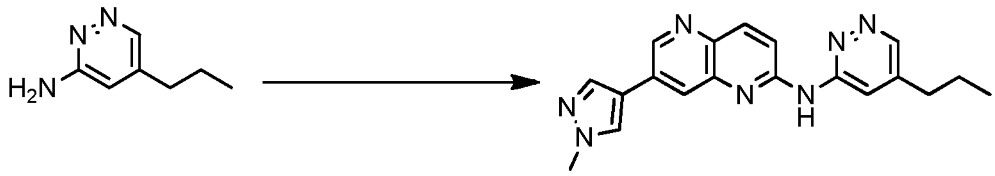

7-(1-Methyl-1H-pyrazol-4-yl)-N-(5-propylpyridazin-3-yl)-1,5-naphthyridine-2-amine was obtained in the same manner as in Example 0554-3 except that 5-propylpyridazine-3-amine was used instead of the 5-morpholinopyridazine-3-amine used in Example 0554-3.
$^1$H-NMR($CDCl_3$/$CD_3OD$=4/1)δ:8.88(1H,d,J=2.1Hz),8.81(1H,brs),8.66(1H,brs),8.20(1H,d,J=9.0Hz),8.17( 1H,brs),7.97(2H, brs),7.48(1H,d,J=9.0Hz),4.07(3H,s),2.74(2H,t,J=7.2Hz),1.87-1.77(2H,m),1.08(3H,t,J=7.2Hz).
MSm/z(M+H):346.

<Example 0919>

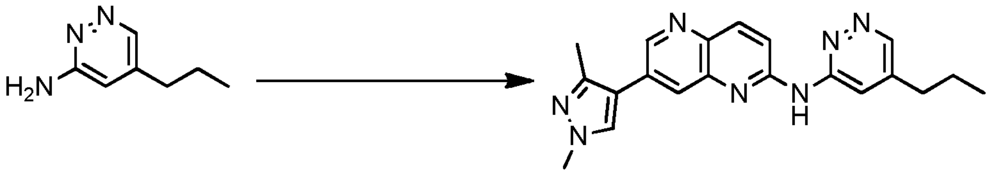

7-(1,3-Dimethyl-1H-pyrazol-4-yl)-N-(5-propylpyridazin-3-yl)-1,5-naphthyridine-2-amine was obtained in the same manner as in Example 0769 except that 5-propylpyridazine-3-amine was used instead of the 5-(pentan-3-yl)pyridazine-3-amine used in Example 0769.
$^1$H-NMR($CDCl_3$/$CD_3OD$=4/1)δ:8.81(1H,brs),8.78(1H,d,J=2.1Hz),8.66(1H,brs),8.22(1H,d,J=9.3Hz),8.11( 1H,d,J=2.1Hz),7.75(1H,s),7.51(1H,d,J=9.3Hz),3.94(3H,s),2.72(2H,t,J=7.2Hz),2.50(3H,s),1.86-1.76(2H,m),1.07(3H,t,J=7.2Hz).
MSm/z(M+H):360.

<Example 0920>

<0920-1>

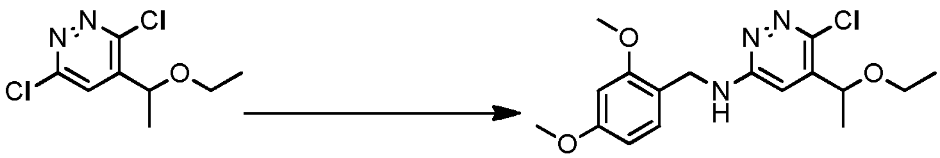

A suspension of 3,6-dichloro-4-(1-ethoxyethyl)pyridazine (240 mg), 2,4-dimethoxybenzylamine (0.280 mL), and potassium carbonate (298 mg) in pentan-2-ol (1 mL) was stirred for 17 hours under reflux. The reaction mixture was cooled to room temperature, the insolubles were filtered off, and the solvent was distilled off under reduced pressure. The obtained residue was purified by silica gel column chromatography (ethyl acetate-hexane), thereby obtaining 6-chloro-N-(2,4-dimethoxybenzyl)-5-(1-ethoxyethyl)pyridazine-3-amine (176 mg).
MSm/z(M+H):352.

<0920-2>

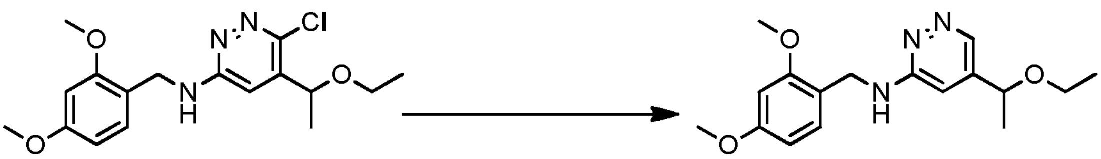

N-(2,4-dimethoxybenzyl)-5-(1-ethoxyethyl)pyridazine-3-amine was obtained in the same manner as in Example 0559-3 except that 6-chloro-N-(2,4-dimethoxybenzyl)-5-(1-ethoxyethyl)pyridazine-3-amine was used instead of the 5-(1-((tert-butyldimethylsilyl)oxy)-2-methylpropan-2-yl)-6-chloro-N-(2,4-dimethoxybenzyl)pyridazine-3-amine used in Example 0559-3.
MSm/z(M+H):318.

<0920-3>

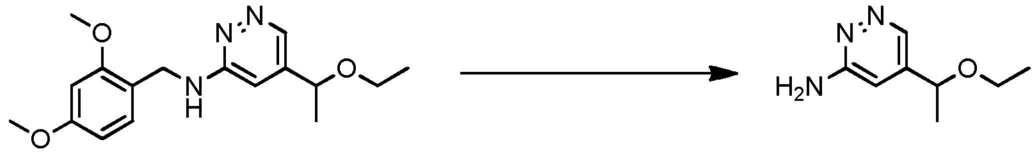

5-(1-Ethoxyethyl)pyridazine-3-amine was obtained in the same manner as in Example 0559-4 except that N-(2,4-dimethoxybenzyl)-5-(1-ethoxyethyl)pyridazine-3-amine was used instead of the 5-(1-((tert-butyldimethylsilyl)oxy)-2-methylpropan-2-yl)-N-(2,4-dimethoxybenzyl)pyridazine-3-amine used in Example 0559-4.
MSm/z(M+H):168.

<0920-4>

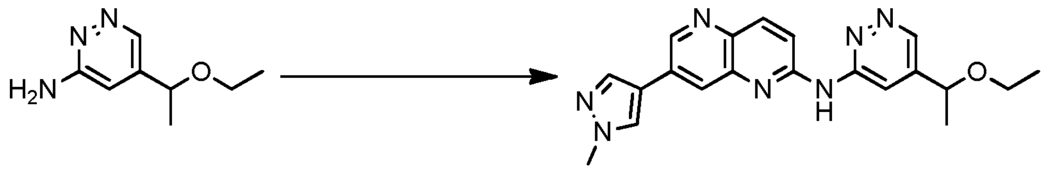

N-(5-(1-ethoxyethyl)pyridazin-3-yl)-7-(1-methyl-1H-pyrazol-4-yl)-1,5-naphthyridine-2-amine was obtained in the same manner as in Example 0554-3 except that 5-(1-ethoxyethyl)pyridazine-3-amine was used instead of the 5-morpholinopyridazine-3-amine used in Example 0554-3.
$^1$H-NMR($CDCl_3$)δ:9.79(1H,brs),9.07(1H,brs),8.93(1H,d,J=2.1Hz),8.88(1H,brs),8.26(1H,d,J=9.0Hz),8.13( 1H,brs),7.95(1H,s),7.84(1H,s),7.72(1H,d,J=9.0Hz),4.59(1H,q,J=6.6Hz),4.02(3H,s),3.65-3.49(2H,m),1.58(3H,d,J=6.6Hz),1.35(3H,t,J=6.6Hz).
MSm/z(M+H):376.

<Example 0921>

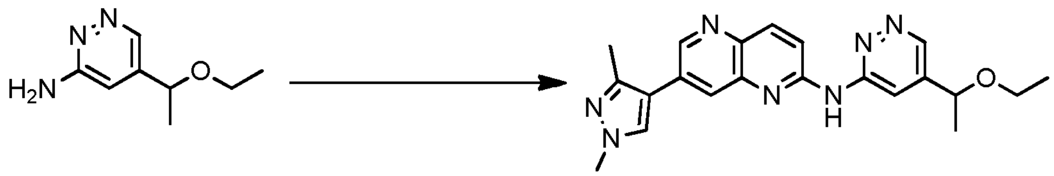

7-(1,3-Dimethyl-1H-pyrazol-4-yl)-N-(5-(1-ethoxyethyl)pyridazin-3-yl)-1,5-naphthyridine-2-amine was obtained in the same manner as in Example 0769 except that 5-(1-ethoxyethyl)pyridazine-3-amine was used instead of the 5-(pentan-3-yl)pyridazine-3-amine used in Example 0769.
$^1$H-NMR($CDCl_3$)δ:9.27(1H,brs),8.96(1H,brs),8.85(1H,brs),8.41(1H,brs),8.28(1H,d,J=9.3Hz),8.08(1H,brs) ,7.65(1H,d,J=9.3Hz),7.64(1H,s),4.55(1H,q,J=6.6Hz),3.95(3H,s),3.60-3.48(2H,m),2.50(3H,s),1.55(3H,t,J=6.6Hz),1.31(3H,t,J=6.6Hz).
MSm/z(M+H):390.

<Example 0922>

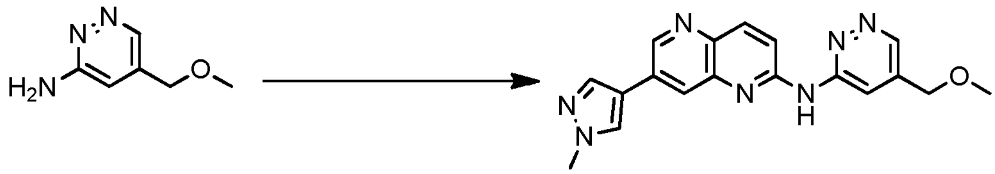

N-(5-(methoxymethyl)pyridazin-3-yl)-7-(1-methyl-1H-pyrazol-4-yl)-1,5-naphthyridine-2-amine was obtained in the same manner as in Example 0554-3 except that 5-(methoxymethyl)pyridazine-3-amine was used instead of the 5-morpholinopyridazine-3-amine used in Example 0554-3.
$^1$H-NMR($CDCl_3$)$\delta$:8.93(1H,d,J=1.8Hz),8.91(1H,brs),8.87(1H,brs),8.25(1H,d,J=9.0Hz),8.14(1H,brs),7.96(1H,s),7.84(1H,s),7.49(1H,d,J=9.0Hz),4.63(2H,s),4.02(3H,s),3.54(3H,s).
MSm/z(M+H):348.

<Example 0923>

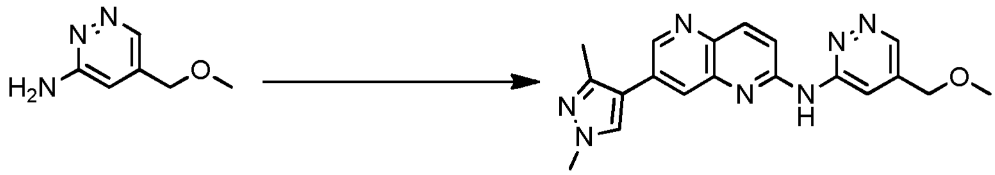

7-(1,3-Dimethyl-1H-pyrazol-4-yl)-N-(5-(methoxymethyl)pyridazin-3-yl)-1,5-naphthyridine-2-amine was obtained in the same manner as in Example 0769 except that 5-(methoxymethyl)pyridazine-3-amine was used instead of the 5-(pentan-3-yl)pyridazine-3-amine used in Example 0769.
$^1$H-NMR($CDCl_3$)$\delta$:8.90(1H,brs),8.85(2H,brs),8.27(1H,d,J=9.0Hz),8.09(1H,brs),7.65(1H,s),7.56(1H,d,J=9.0Hz),4.60(2H,s),3.95(3H,s),3.54(3H,s),2.52(3H,s).
MSm/z(M+H):362.

<Example 0924>

<0924-1>

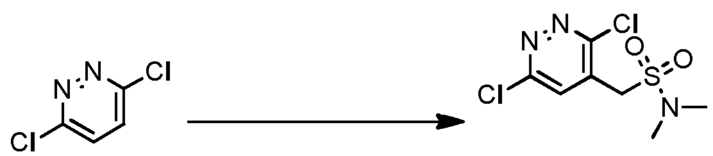

Potassium tert-butoxide (8.88 g) was added to a mixture of 3,6-dichloropyridazine (2.94 g), 1-chloro-N,N-dimethylmethane sulfonamide (2.95 g), and tetrahydrofuran (100 mL) under ice-cooling, followed by stirring at the same temperature for 2 hours. After a saturated ammonium chloride aqueous solution and ethyl acetate were added to the reaction mixture, an organic layer was collected therefrom by separation, washed with a saturated sodium chloride aqueous solution, and dried over anhydrous sodium sulfate, and the solvent was distilled off under reduced pressure. The obtained residue was purified by silica gel chromatography (ethyl acetate-hexane), thereby obtaining 1-(3,6-dichloropyridazin-4-yl)-N,N-dimethylmethane sulfonamide (751 mg).
MSm/z(M+H):270.

<0924-2>

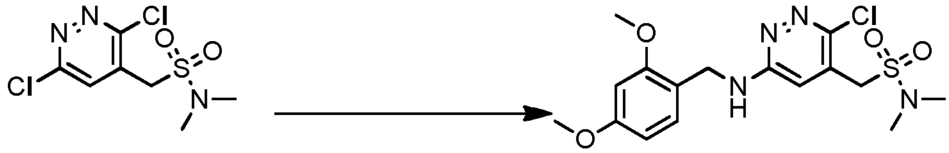

1-(3-Chloro-6-((2,4-dimethoxybenzyl)amino)pyridazin-4-yl)-N,N-dimethylmethane sulfonamide was obtained in the same manner as in Example 0920-1 except that 1-(3,6-dichloropyridazin-4-yl)-N,N-dimethylmethane sulfonamide was used instead of the 3,6-dichloro-4-(1-ethoxyethyl)pyridazine used in Example 0920-1.
MSm/z(M+H):401.

<0924-3>

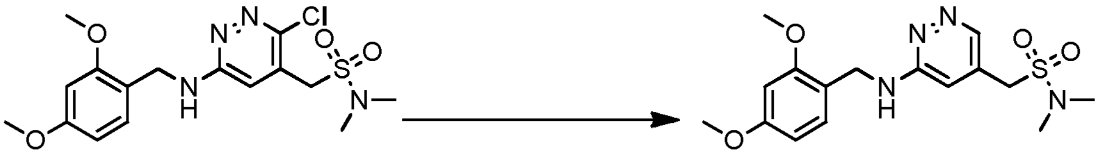

1-(6-((2,4-Dimethoxybenzyl)amino)pyridazin-4-yl)-N,N-dimethylmethane sulfonamide was obtained in the same manner as in Example 0559-3 except that 1-(3-chloro-6-((2,4-dimethoxybenzyl)amino)pyridazin-4-yl)-N,N-dimethylmethane sulfonamide was used instead of the 5-(1-((tert-butyldimethylsilyl)oxy)-2-methylpropan-2-yl)-6-chloro-N-(2,4-dimethoxybenzyl)pyridazine-3-amine used in Example 0559-3.
MSm/z(M+H):367.

<0924-4>

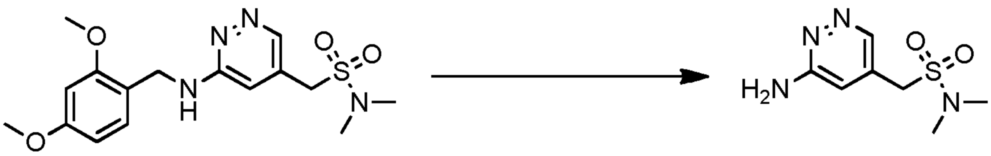

1-(6-Aminopyridazin-4-yl)-N,N-dimethylmethane sulfonamide was obtained in the same manner as in Example 0559-4 except that 1-(6-((2,4-dimethoxybenzyl)amino)pyridazin-4-yl)-N,N-dimethylmethane sulfonamide was used instead of the 5-(1-((tert-butyldimethylsilyl)oxy)-2-methylpropan-2-yl)-N-(2,4-dimethoxybenzyl)pyridazine-3-amine used in Example 0559-4.
MSm/z(M+H):217.

<0924-5>

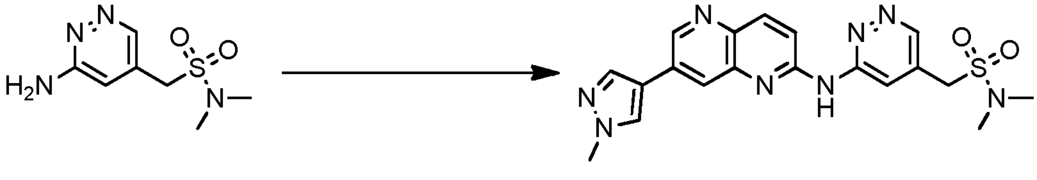

N,N-dimethyl-1-(6-((7-(1-methyl-1H-pyrazol-4-yl)-1,5-naphthyridin-2-yl)amino)pyridazin-4-yl)methane sulfonamide was obtained in the same manner as in Example 0489-4 except that 1-(6-aminopyridazin-4-yl)-N,N-dimethylmethane sulfonamide was used instead of the 4-methylpyridazine-3-amine used in Example 0489-4.
$^1$H-NMR(DMSO-

$d_6$)δ:10.91(1H,s),9.05(1H,d,J=2.1Hz),8.99(1H,brs),8.91(1H,d,J=2.1Hz),8.40(1H,s),8.25(1H,d,J=9.0Hz ),8.24(1H,d,J=2.1Hz),8.08(1H,s),7.69(1H,d,J=9.0Hz),4.68(2H,s),3.93(3H,s),2.85(6H,s).
MSm/z(M+H):425.

<Example 0925>

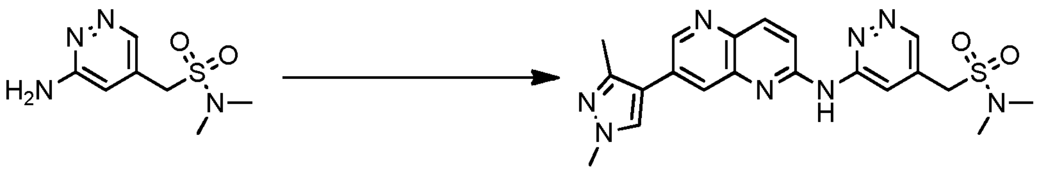

1-(6-((7-(1,3-Dimethyl-1H-pyrazol-4-yl)-1,5-naphthyridin-2-yl)amino)pyridazin-4-yl)-N,N-dimethylmethane sulfonamide was obtained in the same manner as in Example 0786 except that 1-(6-aminopyridazin-4-yl)-N,N-dimethylmethane sulfonamide was used instead of the 5-(1-methoxypropan-2-yl)pyridazine-3-amine used in Example 0786.
$^1$H-NMR(DMSO-$d_6$)δ:10.93(1H,s),9.09(1H,brs),8.89(2H,brs),8.26(1H,d,J=9.0Hz),8.22(1H,s),8.16(1H,brs),7.67(1H,d,J=9.0Hz),4.68(2H,s),3.84(3H,s),2.83(6H,s),2.42(3H,s).
MSm/z(M+H):439.

<Example 0926>

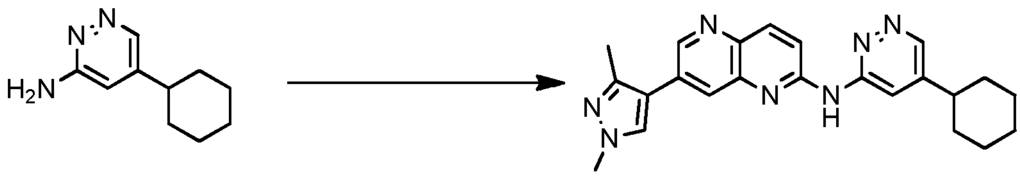

N-(5-cyclohexylpyridazin-3-yl)-7-(1,3-dimethyl-1H-pyrazol-4-yl)-1,5-naphthyridine-2-amine was obtained in the same manner as in Example 0769 except that 5-cyclohexylpyridazine-3-amine was used instead of the 5-(pentan-3-yl)pyridazine-3-amine used in Example 0769.
$^1$H-NMR($CDCl_3$)δ:8.85(1H,brs),8.77(1H,brs),8.73(1H,brs),8.26(1H,d,J=8.7Hz),8.08(1H,d,J=1.8Hz),7.66( 1H,s),7.47(1H,d,J=8.7Hz),3.95(3H,s),2.70-2.55(1H,m),2.51(3H,s),2.08-1.79(6H,m),1.62-1.20(4H,m).
MSm/z(M+H):362.

<Example 0927>

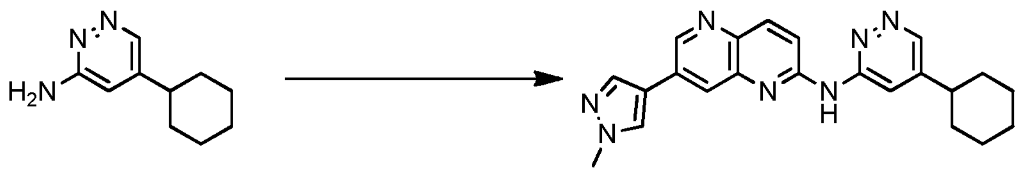

N-(5-cyclohexylpyridazin-3-yl)-7-(1-methyl-1H-pyrazol-4-yl)-1,5-naphthyridine-2-amine was obtained in the same manner as in Example 0489-4 except that 5-cyclohexylpyridazine-3-amine was used instead of the 4-methylpyridazine-3-amine used in Example 0489-4.
$^1$H-NMR($CDCl_3$)δ:9.66(1H,brs),8.92(1H,d,J=1.8Hz),8.85(1H,brs),8.10(1H,d,J=1.8Hz),8.25(1H,d,J=9.3Hz),8.12(1H,d,J=1.8Hz),7.95(1H,s),7.83(1H,s),7.73(1H,d,J=9.3Hz),4.03(3H,s),2.75-2.60(1H,m),2.10-1.79(6H,m),1.66-1

.24(4H,m).
MSm/z(M+H):386.

<Example 0928>

<0928-1>

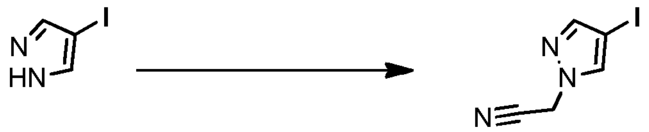

A mixture of 4-iodo-1H-pyrazole (1.00 g), bromoacetonitrile (1.03 mL), cesium carbonate (3.35 g), acetonitrile (4 mL), and 1,4-dioxane (2 mL) was stirred at 80°C for 3 hours. The reaction mixture was cooled to room temperature, and the insolubles were filtered off. The solvent was distilled off under reduced pressure, and the obtained residue was purified by silica gel column chromatography (ethyl acetate-hexane), thereby obtaining 2-(4-iodo-1H-pyrazol-1-yl)acetonitrile (791 mg).
$^1$H-NMR($CDCl_3$)$\delta$:7.61(1H,s),7.60(1H,brs),5.08(2H,s).

<0928-2>

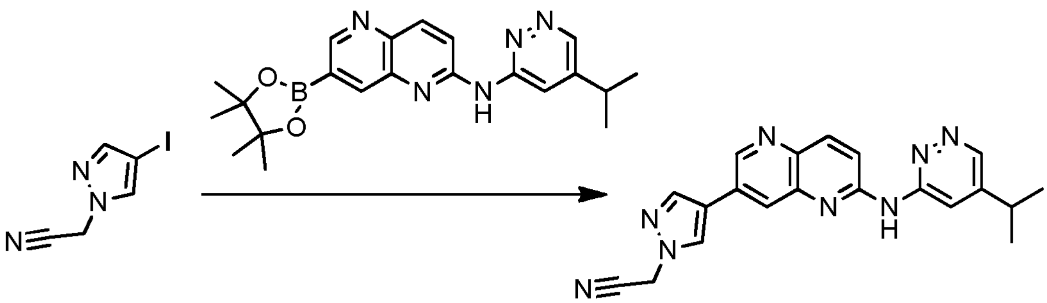

Amixture of 2-(4-iodo-1H-pyrazol-1-yl)acetonitrile (9 mg), N-(5-isopropylpyridazin-3-yl)-7-(4,4,5,5-tetramethyl-1,3,2-dioxaborolan-2-yl)-1,5-naphthyridine-2-amine (10)mg, sodium carbonate (7 mg), bis(di-tert-butyl (4-dimethylaminophenyl)phosphine)dichloropalladium(II) (2 mg), water (0.1 mL), and 1,2-dimethoxyethane (0.5 mL) was stirred at 90°C for 2 hours. The reaction mixture was cooled to room temperature, the insolubles were filtered off, and the solvent was distilled off under reduced pressure. The obtained residue was purified by silica gel column chromatography (methanol-ethyl acetate), thereby obtaining 2-(4-(6-((5-isopropylpyridazin-3-yl)amino)-1,5-naphthyridin-3-yl)-1H-pyrazol-1-yl)acetonitrile (2.0 mg).
$^1$H-NMR($CDCl_3$)$\delta$:8.92(1H,brs),8.82(2H,brs),8.26(1H,d,J=8.7Hz),8.12(1H,brs),8.05(1H,s),8.02(1H,s),7.6 3(1H,d,J=8.7Hz),5.20(2H,s),3.15-2.96(1H,m),1.42(6H,d,J=6.6Hz).
MSm/z(M+H):371.

<Example 0929> (not in accordance with the present invention)

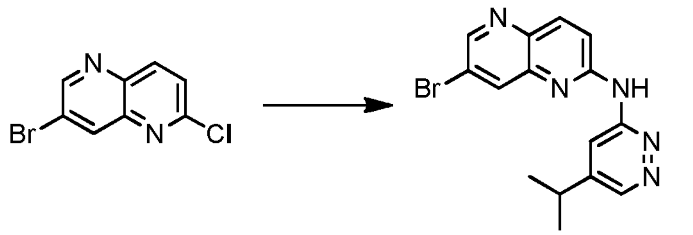

N,N-dimethylacetamide (75 mL), 5-isopropylpyridazine-3-amine (5.12 g) and sodium tert-amyl oxide (9.08 g) were added to 7-bromo-2-chloro-1,5-naphthyridine (9.58 g), followed by stirring at 80°C for 1 hour. The reaction mixture was poured into water (300 mL), and the precipitated solid was collected by filtration and washed with ethanol, thereby obtaining 7-bromo-N-(5-isopropylpyridazin-3-yl)-1,5-naphthyridine-2-amine (7.70 g) as a brown solid.
$^{1}$H-NMR(DMSO-$d_6$)$\delta$:10.89(1H,s),8.88(1H,d,J=2.0Hz),8.80(1H,d,J=2.0Hz),8.73(1H,d,J=2.0Hz),8.48(1H,d,J=2.0Hz),8.27(1H,d,J=9.2Hz),7.79(1H,d,J=9.2Hz),3.09-3.00(1H,m),1.32(6H,d,J=6.6Hz).
MSm/z(M+H):344.

<Example 0930>

<0930-1>

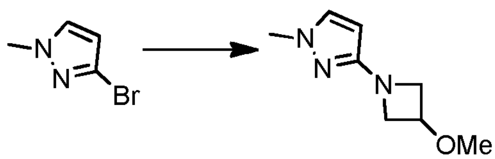

3-Methoxyazetidine hydrochloride (160 mg), sodium tert-butoxide (250 mg), 1,4-dioxane (10 mL), and ((2-dicyclohexylphosphino-3,6-dimethoxy-2',4',6'-triisopropyl-1,1'-biphenyl)-2-(2'-amino-1,1'-biphenyl))palladium(II) methanesulfonate (BRETTPHOS-PD-G3 (trade name, manufactured by Sigma-Aldrich Co. LLC.)) (40 mg) were added to 3-bromo-1-methyl-1H-pyrazole (160 mg), followed by stirring at 80°C for 2 hours. The reaction mixture was cooled to room temperature, the solvent was distilled off under reduced pressure, and the obtained residue was purified by silica gel column chromatography (hexane-ethyl acetate-methanol), thereby obtaining 3-(3-methoxyazetidin-1-yl)-1-methyl-1H-pyrazole (41 mg) as yellow oily substance.
MSm/z(M+H):168.

<0930-2>

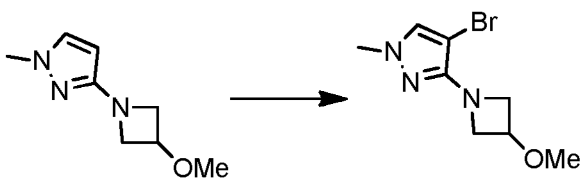

4-Bromo-3-(3-methoxyazetidin-1-yl)-1-methyl-1H-pyrazole was obtained as a yellow solid in the same manner as in Example 0734-2 except that 3-(3-methoxyazetidin-1-yl)-1-methyl-1H-pyrazole was used instead of the 2-methyl-5-(1-methyl-1H-pyrazol-3-yl)pyridine used in Example 0734-2.
MSm/z(M+H):248.

<0930-3>

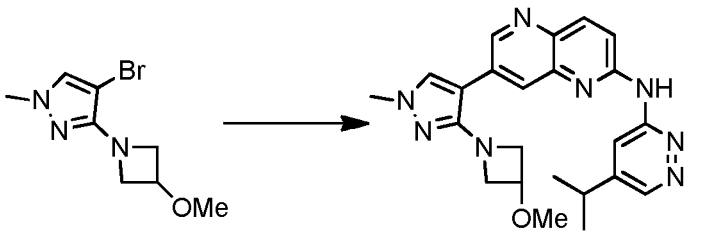

N-(5-isopropylpyridazin-3-yl)-7-(3-(3-methoxyazetidin-1-yl)-1-methyl-1H-pyrazol-4-yl)-1,5-naphthyridine-2-amine was obtained as a yellow solid in the same manner as in Example 0385-7 except that 7-bromo-N-(5-isopropylpyridazin-3-yl)-1,5-naphthyridine-2-amine was used instead of the 7-bromo-2-chloro-1,5-naphthyridine used in Example 0385-7, and 4-bromo-3-(3-methoxyazetidin-1-yl)-1-methyl-1H-pyrazole was used instead of the 4-(3-(3-ethyl-4-iodo-1H-pyrazol-1-yl)propyl)morpholine used in Example 0385-7.
$^1$H-NMR($CDCl_3$)$\delta$:9.66(1H,s),8.97(1H,s),8.86(1H,d,J=2.0Hz),8.82(1H,d,J=2.0Hz),8.24(1H,d,J=9.2Hz),8.05(1H,d,J=2.0Hz),7.70(1H,d,J=9.2Hz),7.49(1H,s),4.29-4.22(1H,m),4.12-4.07(2H,m),3.86(3H,s),3.73(2H,dd,J=8.6,4.6Hz),3.24(3H,s),3.11-3.02(1H,m),1.43(6H,d,J=7.3Hz).
MSm/z(M+H):431.

<Example 0931>

<0931-1>

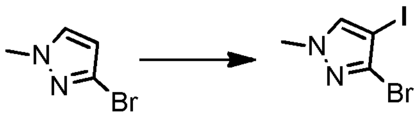

3-Bromo-4-iodo-1-methyl-1H-pyrazole was obtained as colorless oily substance in the same manner as in Example 0886-1 except that 3-bromo-1-methyl-1H-pyrazole was used instead of the 3-chloro-1-methyl-1H-pyrazole used in Example 0886-1.
MSm/z(M+H):286.

<0931-2>

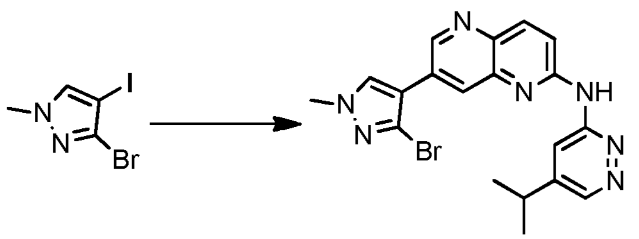

7-(3-Bromo-1-methyl-1H-pyrazol-4-yl)-N-(5-isopropylpyridazin-3-yl)-1,5-naphthyridine-2-amine was obtained as a pale yellow solid in the same manner as in Example 0935-3 except that 3-bromo-4-iodo-1-methyl-1H-pyrazole was used instead of the 1-(4-bromo-1-methyl-1H-pyrazol-3-yl)-4-methyl-1,4-diazepan-5-one used in Example 0935-3.
$^1$H-NMR($CDCl_3$)$\delta$:8.96(1H,brs),8.88(1H,d,J=2.0Hz),8.72(1H,brs),8.41(1H,d,J=2.0Hz),8.22(1H,d,J=9.2Hz),7.85(1H,s),7.55(1H,d,J=9.2Hz),4.01(3H,s),3.10-3.01(1H,m),1.41(6H,d,J=7.3Hz).
MSm/z(M+H):424.

<Example 0932>

<0932-1>

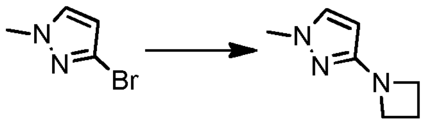

3-(Azetidin-1-yl)-1-methyl-1H-pyrazole was obtained as yellow oily substance in the same manner as in Example 0930-1 except that azetidine was used instead of the 3-methoxyazetidine hydrochloride used in Example 0930-1.
MSm/z(M+H):138.

<0932-2>

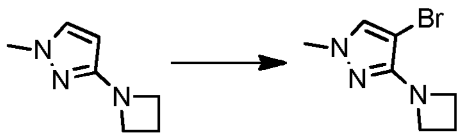

3-(Azetidin-1-yl)-4-bromo-1-methyl-1H-pyrazole was obtained as yellow oily substance in the same manner as in Example 0734-2 except that 3-(azetidin-1-yl)-1-methyl-1H-pyrazole was used instead of the 2-methyl-5-(1-methyl-1H-pyrazol-3-yl)pyridine used in Example 0734-2.
MSm/z(M+H):216.

<0932-3>

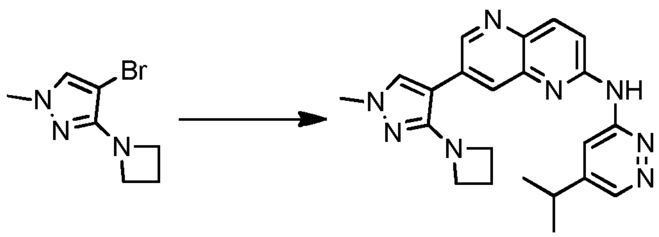

7-(3-(Azetidin-1-yl)-1-methyl-1H-pyrazol-4-yl)-N-(5-isopropylpyridazin-3-yl)-1,5-naphthyridine-2-amine was obtained as a yellow solid in the same manner as in Example 0385-7 except that 7-bromo-N-(5-isopropylpyridazin-3-yl)-1,5-naphthyridine-2-amine was used instead of the 7-bromo-2-chloro-1,5-naphthyridine used in Example 0385-7, and 3-(azetidin-1-yl)-4-bromo-1-methyl-1H-pyrazole was used instead of the 4-(3-(3-ethyl-4-iodo-1H-pyrazol-1-yl)propyl)morpholine used in Example 0385-7.
$^1$H-NMR($CDCl_3$)δ:9.85(1H,brs),9.01(1H,brs),8.87(1H,d,J=2.0Hz),8.82(1H,d,J=2.0Hz),8.24(1H,d,J=8.6Hz),8.08(1H,d,J=2.0Hz),7.71(1H,d,J=8.6Hz),7.49(1H,s),3.91(3H,s),3.87(4H,d,J=7.2Hz),3.11-3.02(1H,m),2.34-2.24(2H,m), 1.43(6H,d,J=6.6Hz).
MSm/z(M+H):401.

<Example 0933>

<0933-1>

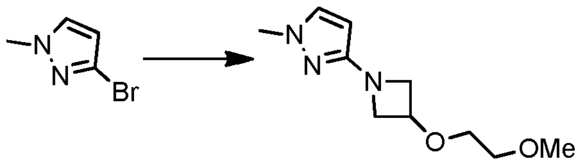

3-(3-(2-Methoxyethoxy)azetidin-1-yl)-1-methyl-1H-pyrazole was obtained as yellow oily substance in the same manner as in Example 0930-1 except that 3-(2-methoxyethoxy)azetidine hydrochloride was used instead of the 3-methoxyazetidine hydrochloride used in Example 0930-1.
MSm/z(M+H):212.

<0933-2>

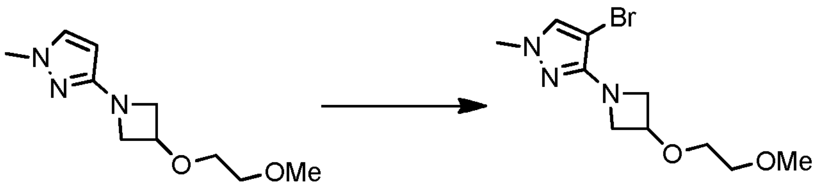

4-Bromo-3-(3-(2-methoxyethoxy)azetidin-1-yl)-1-methyl-1H-pyrazole was obtained as yellow oily substance in the same manner as in Example 0734-2 except that 3-(3-(2-methoxyethoxy)azetidin-1-yl)-1-methyl-1H-pyrazole was used instead of the 2-methyl-5-(1-methyl-1H-pyrazol-3-yl)pyridine used in Example 0734-2.
MSm/z(M+H):290.

<0933-3>

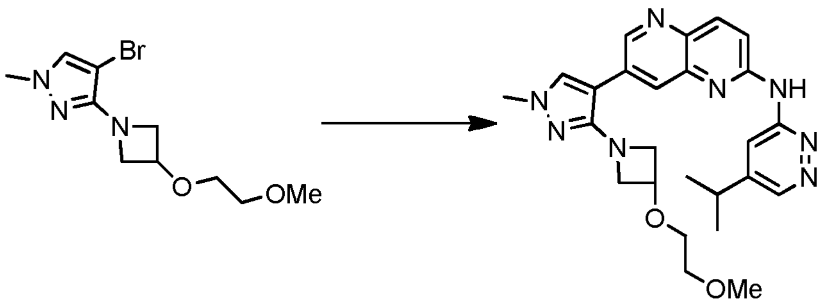

N-(5-isopropylpyridazin-3-yl)-7-(3-(3-(2-methoxyethoxy)azetidin-1-yl)-1-methyl-1H-pyrazol-4-yl)-1,5-naphthyridine-2-amine was obtained as a yellow solid in the same manner as in Example 0385-7 except that 7-bromo-N-(5-isopropylpyridazin-3-yl)-1,5-naphthyridine-2-amine was used instead of the 7-bromo-2-chloro-1,5-naphthyridine used in Example 0385-7, and 4-bromo-3-(3-(2-methoxyethoxy)azetidin-1-yl)-1-methyl-1H-pyrazole was used instead of the 4-(3-(3-ethyl-4-iodo-1H-pyrazol-1-yl)propyl)morpholine used in Example 0385-7.
$^1$H-NMR($CDCl_3$)$\delta$:8.94(1H,brs),8.86(1H,d,J=2.0Hz),8.82(1H,d,J=2.0Hz),8.25(1H,d,J=9.2Hz),8.05(1H,d,J =2.0Hz),7.74(1H,brs),7.49(1H,s),4.43-4.35(1H,m),4.10(2H,t,J=7.6Hz),3.85(3H,s),3.77(2H,dd,J=8.6,5.3Hz),3.53-3.47(4H,m),3.33(3H,s),3.11-3.02(1H,m),1.43(6H,d,J=7.3Hz).
MSm/z(M+H):475.

<Example 0934>

<0934-1>

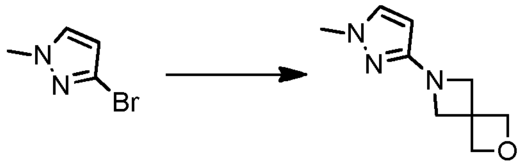

6-(1-Methyl-1H-pyrazol-3-yl)-2-oxa-6-azaspiro[3.3]heptane was obtained as yellow oily substance in the same manner as in Example 0930-1 except that 2-oxa-6-azaspiro[3.3]heptane was used instead of the 3-methoxyazetidine hydrochloride used in Example 0930-1.
MSm/z(M+H):180.

<0934-2>

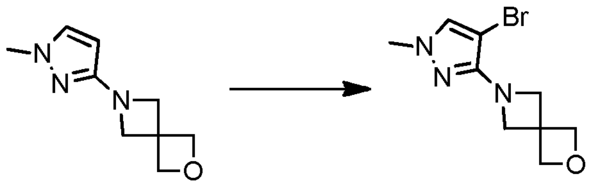

6-(4-Bromo-1-methyl-1H-pyrazol-3-yl)-2-oxa-6-azaspiro[3.3]heptane was obtained as a white solid in the same manner as in Example 0734-2 except that 6-(1-methyl-1H-pyrazol-3-yl)-2-oxa-6-azaspiro[3.3]heptane was used instead of the 2-methyl-5-(1-methyl-1H-pyrazol-3-yl)pyridine used in Example 0734-2.
MSm/z(M+H):258.

<0934-3>

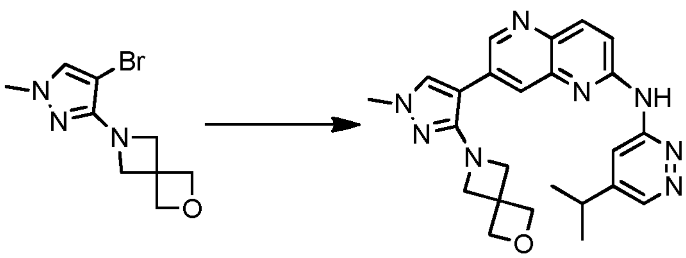

N-(5-isopropylpyridazin-3-yl)-7-(1-methyl-3-(2-oxa-6-azaspiro[3.3]heptan-6-yl)-1H-pyrazol-4-yl)-1,5-naphthyridine-2-amine was obtained as a yellow solid in the same manner as in Example 0385-7 except that 7-bromo-N-(5-isopropylpyridazin-3-yl)-1,5-naphthyridine-2-amine was used instead of the 7-bromo-2-chloro-1,5-naphthyridine used in Example 0385-7, and 6-(4-bromo-1-methyl-1H-pyrazol-3-yl)-2-oxa-6-azaspiro[3.3]heptane was used instead of the 4-(3-(3-ethyl-4-iodo-1H-pyrazol-1-yl)propyl)morpholine used in Example 0385-7.
$^1$H-NMR($CDCl_3$)δ:10.04(1H,brs),8.98(1H,brs),8.85(1H,d,J=2.0Hz),8.83(1H,d,J=2.0Hz),8.26(1H,d,J=8.6 Hz),8.02(1H,d,J=2.0Hz),7.80(1H,d,J=8.6Hz),7.49(1H,s),4.77(4H,d,J=4.6Hz),4.03(4H,s),3.87(3H,d,J= 9.2Hz),3.10-3.01(1H,m),1.42(6H,d,J=6.6Hz).
MSm/z(M+H):443.

<Example 0935>

<0935-1>

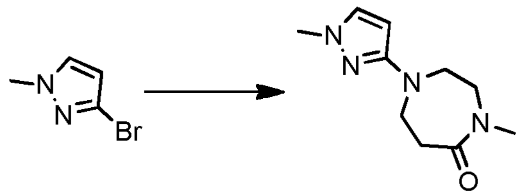

4-Methyl-1-(1-methyl-1H-pyrazol-3-yl)-1,4-diazepan-5-one was obtained as a yellow solid in the same manner as in Example 0930-1 except that 4-methyl-1,4-diazepan-5-one was used instead of the 3-methoxyazetidine hydrochloride used in Example 0930-1.
MSm/z(M+H):209.

<0935-2>

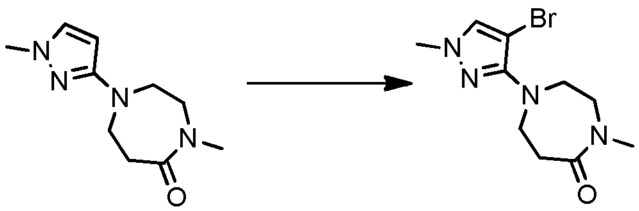

1-(4-Bromo-1-methyl-1H-pyrazol-3-yl)-4-methyl-1,4-diazepan-5-one was obtained as a yellow solid in the same manner as in Example 0734-2 except that 4-methyl-1-(1-methyl-1H-pyrazol-3-yl)-1,4-diazepan-5-one was used instead of the 2-methyl-5-(1-methyl-1H-pyrazol-3-yl)pyridine used in Example 0734-2.
MSm/z(M+H):287.

<0935-3>

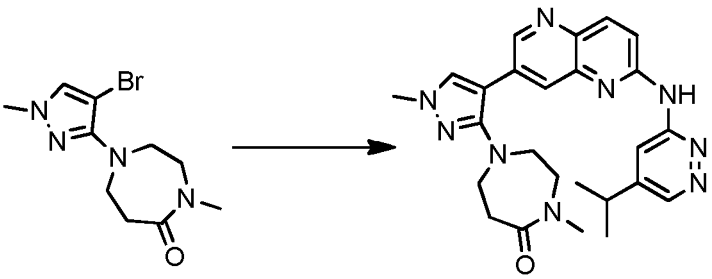

N-(5-isopropylpyridazin-3-yl)-7-(4,4,5,5-tetramethyl-1,3,2-dioxaborolan-2-yl)-1,5-naphthyridine-2-amine (13 mg), sodium carbonate (10 mg), 1,4-dioxane (0.9 mL), water (0.1 mL), and (2-dicyclohexylphosphino-2',4',6'-triisopropyl-1,1'-biphenyl) (2-(2'-amino-1,1'-biphenyl))palladium(II) methanesulfonate (XPHOS-PD-G3 (trade name, manufactured by Sigma-Aldrich Co. LLC.)) (2 mg) were added to 1-(4-bromo-1-methyl-1H-pyrazol-3-yl)-4-methyl-1,4-diazepan-5-one (9 mg), followed by stirring at 90°C for 76 hours. The solvent of the reaction mixture was distilled off under reduced pressure, and the residue was purified by silica gel column chromatography (hexane-ethyl acetate-methanol, NH silica), thereby obtaining 1-(4-(6-((5-isopropylpyridazin-3-yl)amino)-1,5-naphthyridin-3-yl)-1-methyl-1H-pyrazol-3-yl)-4-methyl-1,4-diazepan-5-one (1.8 mg) as a yellow solid.
$^1$H-NMR($CDCl_3$)δ:9.35(1H,brs),9.02(1H,brs),8.93(1H,brs),8.80(1H,brs),8.33(1H,brs),8.25(1H,d,J=9.2Hz) ,7.65(1H,brs),7.60(1H,s),3.87(3H,s),3.61(2H,t,J=4.8Hz),3.33-3.26(4H,m),3.06-2.98(1H,m),3.02(3H,s),2.83(2H,t,J=4.8Hz),1.40(6H,d,J=7.3Hz).
MSm/z(M+H):472.

<Example 0936>

<0936-1>

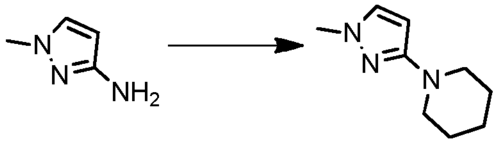

1,5-Dibromopentane (136 μL), cesium carbonate (814 mg), and N,N-dimethylacetamide (3 mL) were added to 1-methyl-1H-pyrazole-3-amine (97 mg), followed by stirring at 90°C for 3 hours. After water was added to the reaction mixture, the resultant product was extracted three times with ethyl acetate, washed with a saturated sodium chloride aqueous solution, and dried over anhydrous sodium sulfate. The solvent of was distilled off under reduced pressure,and the residue was purified by silica gel column chromatography (hexane-ethyl acetate), thereby obtaining 1-(1-methyl-1H-pyrazol-3-yl)piperidine (91 mg) as yellow oily substance.
MSm/z(M+H):166.

<0936-2>

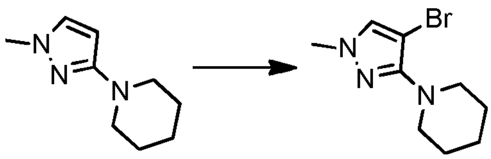

1-(4-Bromo-1-methyl-1H-pyrazol-3-yl)piperidine was obtained as red oily substance in the same manner as in Example 0734-2 except that 1-(1-methyl-1H-pyrazol-3-yl)piperidine was used instead of the 2-methyl-5-(1-methyl-1H-pyrazol-3-yl)pyridine used in Example 0734-2.
MSm/z(M+H):244.

<0936-3>

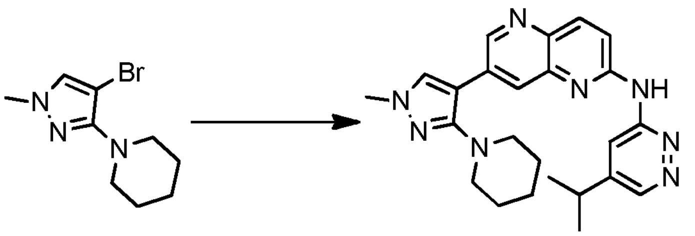

N-(5-isopropylpyridazin-3-yl)-7-(1-methyl-3-(piperidin-1-yl)-1H-pyrazol-4-yl)-1,5-naphthyridine-2-amine was obtained as a pale yellow solid in the same manner as in Example 0935-3 except that 1-(4-bromo-1-methyl-1H-pyrazol-3-yl)piperidine was used instead of the 1-(4-bromo-1-methyl-1H-pyrazol-3-yl)-4-methyl-1,4-diazepan-5-one used in Example 0935-3.
$^1$H-NMR($CDCl_3$)δ:9.04(1H,brs),9.00(1H,d,J=2.0Hz),8.89(1H,brs),8.79(1H,brs),8.47(1H,brs),8.22(1H,d,J =8.9Hz),7.61(1H,s),7.44(1H,d,J=8.9Hz),3.87(3H,s),3.09-2.97(5H,m),1.79-1.71(4H,m),1.69-1.60(2H,m),1.42(6H,d,J=7.3Hz).
MSm/z(M+H):429.

<Example 0937>

<0937-1>

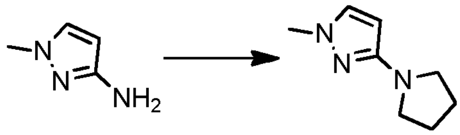

1-Methyl-3-(pyrrolidin-1-yl)-1H-pyrazole was obtained as yellow oily substance in the same manner as in Example 0936-1 except that 1,4-dibromobutane was used instead of the 1,5-dibromopentane used in Example 0936-1.
MSm/z(M+H):152.

<0937-2>

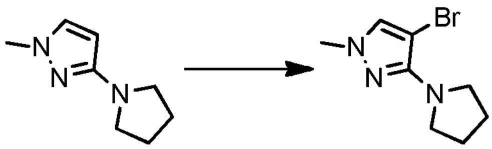

4-Bromo-1-methyl-3-(pyrrolidin-1-yl)-1H-pyrazole was obtained as yellow oily substance in the same manner as in Example 0734-2 except that 1-methyl-3-(pyrrolidin-1-yl)-1H-pyrazole was used instead of the 2-methyl-5-(1-methyl-1H-pyrazol-3-yl)pyridine used in Example 0734-2.
MSm/z(M+H):230.

<0937-3>

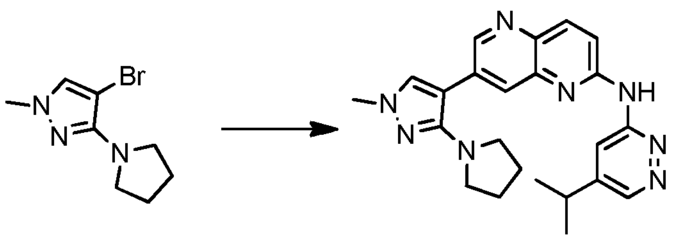

N-(5-isopropylpyridazin-3-yl)-7-(1-methyl-3-(pyrrolidin-1-yl)-1H-pyrazol-4-yl)-1,5-naphthyridine-2-amine was obtained as a yellow solid in the same manner as in Example 0935-3 except that 4-bromo-1-methyl-3-(pyrrolidin-1-yl)-1H-pyrazole was used instead of the 1-(4-bromo-1-methyl-1H-pyrazol-3-yl)-4-methyl-1,4-diazepan-5-one used in Example 0935-3.
$^1$H-NMR($CDCl_3$)δ:9.20(1H,brs),8.94(1H,d,J=2.0Hz),8.88(1H,d,J=2.0Hz),8.80(1H,d,J=2.0Hz),8.25(1H,d,J=8.6Hz),8.08(1H,d,J=2.0Hz),7.58(1H,d,J=8.6Hz),7.46(1H,s),3.86(3H,s),3.22(4H,t,J=6.6Hz),3.08-2.99(1H,m),1.90-1.86(4H,m),1.40(6H,d,J=6.6Hz).
MSm/z(M+H):415.

<Example 0938>

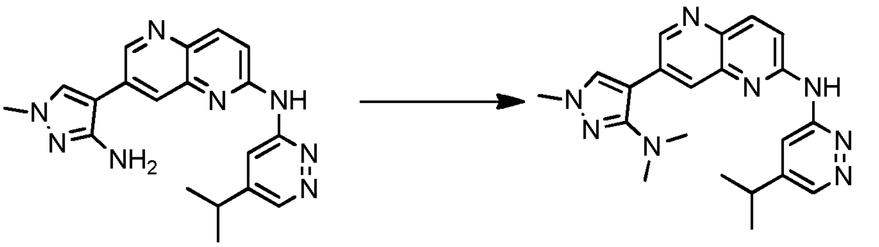

7-(3-(Dimethylamino)-1-methyl-1H-pyrazol-4-yl)-N-(5-isopropylpyridazin-3-yl)-1,5-naphthyridine-2-amine was obtained as a pale yellow solid in the same manner as in Example 0741 except that 7-(3-amino-1-methyl-1H-pyrazol-4-yl)-N-(5-isopropylpyridazin-3-yl)-1,5-naphthyridine-2-amine was used instead of the 7-(1-(2-(azetidin-3-yloxy)ethyl)-1H-pyrazol-4-yl)-N-(5-isopropylpyridazin-3-yl)-1,5-naphthyridine-2-amine used in Example 0741.
$^1$H-NMR($CDCl_3$)δ:9.24(1H,brs),9.00(1H,d,J=2.0Hz),8.93(1H,brs),8.81(1H,brs),8.26(1H,brs),8.24(1H,d,J =9.2Hz),7.61(1H,d,J=9.2Hz),7.54(1H,s),3.86(3H,s),3.09-3.00(1H,m),2.79(6H,s),1.41(6H,d,J=6.6Hz).
MSm/z(M+H):389.

<Example 0939>

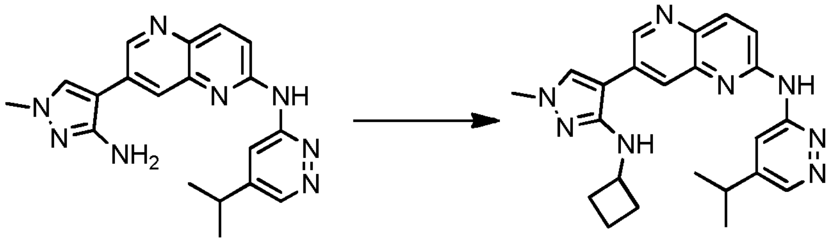

7-(3-(Cyclobutylamino)-1-methyl-1H-pyrazol-4-yl)-N-(5-isopropylpyridazin-3-yl)-1,5-naphthyridine-2-amine was obtained as a pale yellow solid in the same manner as in Example 0830 except that cyclobutanone was used instead of the cyclohexanone used in Example 0830.
$^1$H-NMR($CDCl_3$)δ:8.91(2H,brs),8.85(1H,d,J=2.0Hz),8.80(1H,brs),8.24(1H,d,J=9.2Hz),8.12(1H,brs),7.53( 1H,d,J=9.2Hz),7.49(1H,s),4.24-4.15(1H,m),3.93(1H,d,J=7.3Hz),3.84(3H,s),3.08-2.99(1H,m),2.50-2.42(2H,m),1.89-1.69(4H,m),1.41(6H,d,J=7.3Hz).
MSm/z(M+H):415.

<Example 0940>

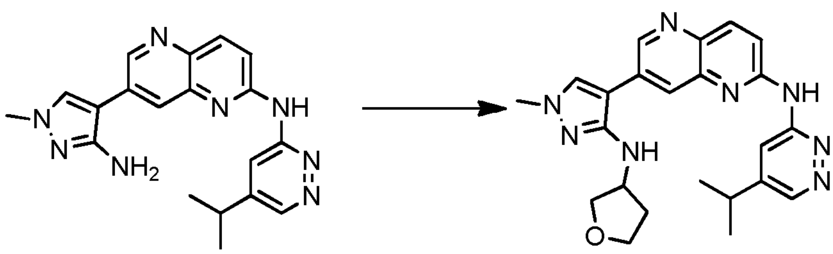

N-(5-isopropylpyridazin-3-yl)-7-(1-methyl-3-((tetrahydrofuran-3-yl)amino)-1H-pyrazol-4-yl)-1,5-naphthyridine-2-amine was obtained as a pale yellow solid in the same manner as in Example 0830 except that dihydrofuran-3(2H)-one was used instead of the cyclohexanone used in Example 0830.
$^1$H-NMR($CDCl_3$)δ:9.86(1H,brs),8.98(1H,brs),8.83(1H,d,J=2.0Hz),8.77(1H,brs),8.22(1H,d,J=8.9Hz),8.09( 1H,d,J=2.0Hz),7.71(1H,d,J=8.9Hz),7.51(1H,s),4.41-4.32(1H,m),4.05-3.95(3H,m),3.91-3.79(5H,m),3.07-2.98(1H,m),2.39-2.26(2H,m),1.

41(6H,d,J=7.2Hz).
MSm/z(M+H):431.

<Example 0941>

<0941-1>

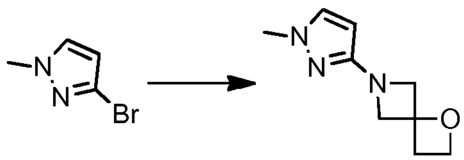

6-(1-Methyl-1H-pyrazol-3-yl)-1-oxa-6-azaspiro[3.3]heptane was obtained as yellow oily substance in the same manner as in Example 0930-1 except that 1-oxa-6-azaspiro[3.3]heptane was used instead of the 3-methoxyazetidine hydrochloride used in Example 0930-1.
MSm/z(M+H):180.

<0941-2>

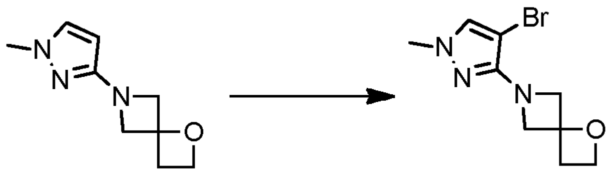

6-(4-Bromo-1-methyl-1H-pyrazol-3-yl)-1-oxa-6-azaspiro[3.3]heptane was obtained as pale yellow oily substance in the same manner as in Example 0734-2 except that 6-(1-methyl-1H-pyrazol-3-yl)-1-oxa-6-azaspiro[3.3]heptane was used instead of the 2-methyl-5-(1-methyl-1H-pyrazol-3-yl)pyridine used in Example 0734-2.
MSm/z(M+H):260.

<0941-3>

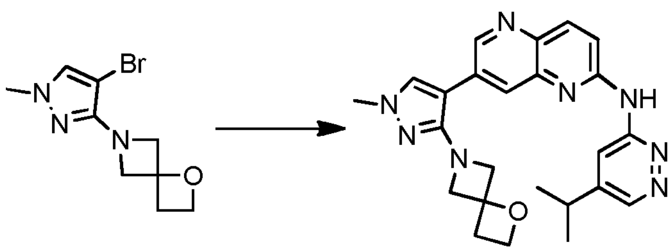

N-(5-isopropylpyridazin-3-yl)-7-(1-methyl-3-(1-oxa-6-azaspiro[3.3]heptan-6-yl)-1H-pyrazol-4-yl)-1,5-naphthyridine-2-amine was obtained as a yellow solid in the same manner as in Example 0935-3 except that 6-(4-bromo-1-methyl-1H-pyrazol-3-yl)-1-oxa-6-azaspiro[3.3]heptane was used instead of the 1-(4-bromo-1-methyl-1H-pyrazol-3-yl)-4-methyl-1,4-diazepan-5-one used in Example 0935-3.
$^1$H-NMR($CDCl_3$)δ:9.19(1H,brs),8.92(1H,brs),8.86(1H,d,J=2.0Hz),8.82(1H,d,J=2.0Hz),8.25(1H,d,J=9.2Hz),8.03(1H,d,J=2.0Hz),7.60(1H,d,J=9.2Hz),7.48(1H,s),4.48(2H,t,J=7.3Hz),4.12-4.00(4H,m),3.85(3H,s),3.10-3.00(1H,m),2.87(2H,t,J=7.3Hz),1.41(6H,d,J=7.3Hz).
MSm/z(M+H):443.

<Example 0942>

<0942-1>

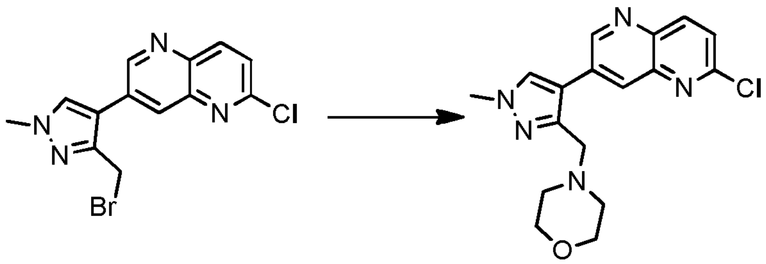

Morpholine (6 μL), potassium carbonate (16 mg), and acetonitrile (1 mL) were added to 7-(3-(bromomethyl)-1-methyl-1H-pyrazol-4-yl)-2-chloro-1,5-naphthyridine (20 mg), followed by stirring at 50°C for 1 hour. The solvent of the reaction mixture was distilled off under reduced pressure, and the residue was purified by silica gel column chromatography (hexane-ethyl acetate-methanol), thereby obtaining 4-((4-(6-chloro-1,5-naphthyridin-3-yl)-1-methyl-1H-pyrazol-3-yl)methyl)morpholine (7 mg) as a white solid.
MSm/z(M+H):344.

<0942-2>

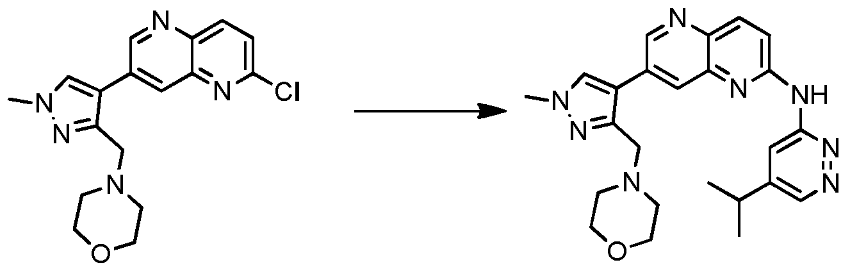

N-(5-isopropylpyridazin-3-yl)-7-(1-methyl-3-(morpholinomethyl)-1H-pyrazol-4-yl)-1,5-naphthyridine-2-amine was obtained as a white solid in the same manner as in Example 0729-2 except that 4-((4-(6-chloro-1,5-naphthyridin-3-yl)-1-methyl-1H-pyrazol-3-yl)methyl)morpholine was used instead of the 1-(4-(6-chloro-1,5-naphthyridin-3-yl)-1H-pyrazol-1-yl)-3-morpholinopropan-2-ol used in Example 0729-2.
$^1$H-NMR($CDCl_3$)δ:10.15(1H,brs),9.06(1H,d,J=2.0Hz),8.94(1H,s),8.84(1H,d,J=2.0Hz),8.28(1H,d,J=8.9Hz ),8.26(1H,s),7.85(1H,d,J=8.9Hz),7.69(1H,s),3.99(3H,s),3.72(4H,t,J=4.0Hz),3.65(2H,s),3.08-2.99(1H,m),2.58(4H,brs),1.42(6H,d,J=6.6Hz).
MSm/z(M+H):445.

<Example 0943>

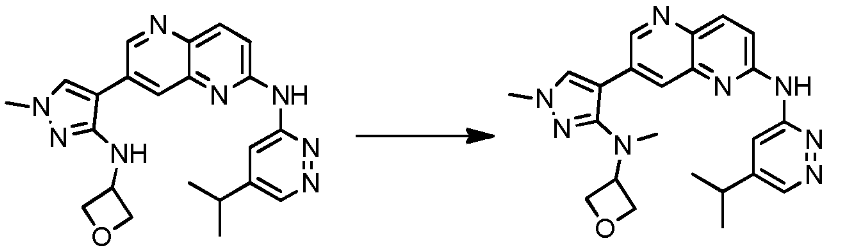

N-(5-isopropylpyridazin-3-yl)-7-(1-methyl-3-(methyl (oxetan-3-yl)amino)-1H-pyrazol-4-yl)-1,5-naphthyridine-2-amine was obtained as a pale yellow solid in the same manner as in Example 0830 except that N-(5-isopropylpyridazin-3-yl)-7-(1-methyl-3-(oxetan-3-ylamino)-1H-pyrazol-4-yl)-1,5-naphthyridine-2-amine was used instead of the 7-(3-amino-1-methyl-1H-pyrazol-4-yl)-N-(5-isopropylpyridazin-3-yl)-1,5-naphthyridine-2-amine used in Example 0830, and a 36.0% to 38.0%-formaldehyde aqueous solution was used instead of the cyclohexanone used in Example 0830.
$^1$H-NMR($CDCl_3$)δ:9.04(1H,d,J=2.0Hz),8.94(1H,brs),8.81(1H,d,J=2.0Hz),8.35(1H,d,J=2.0Hz),8.25(1H,d,J =9.2Hz),7.61-7.54(2H,m),4.83(2H,t,J=6.9Hz),4.70(2H,t,J=6.3Hz),4.59-4.50(1H,m),3.83(3H,s),3.09-3.00(1H,m),2.63(3 H,s),1.43(6H,d,J=6.6Hz).
MSm/z(M+H):431.

<Example 0944>

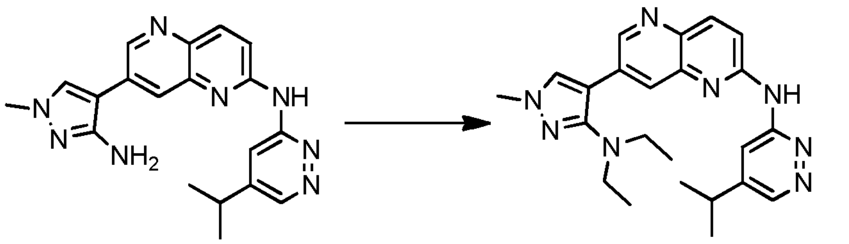

7-(3-(Diethylamino)-1-methyl-1H-pyrazol-4-yl)-N-(5-isopropylpyridazin-3-yl)-1,5-naphthyridine-2-amine was obtained as a pale yellow solid in the same manner as in Example 0830 except that acetaldehyde was used instead of the cyclohexanone used in Example 0830.
$^1$H-NMR($CDCl_3$)δ:9.03(1H,d,J=2.0Hz),8.99(1H,brs),8.79(1H,brs),8.40(1H,brs),8.23(1H,d,J=8.6Hz),7.58( 1H,s),7.52(1H,br s),3.87(3H,s),3.15(4H,q,J=7.3Hz),3.07-2.98(1H,m),1.41(6H,d,J=7.3Hz),1.10(6H,t,J=7.3Hz).
MSm/z(M+H):417.

<Example 0945>

<0945-1>

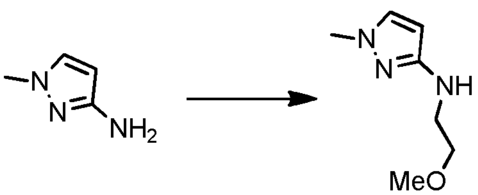

N-(2-methoxyethyl)-1-methyl-1H-pyrazole-3-amine was obtained as yellow oily substance in the same manner as in Example 0936-1 except that 1-bromo-2-methoxyethane was used instead of the 1,5-dibromopentane used in Example 0936-1.

MSm/z(M+H):156.

<0945-2>

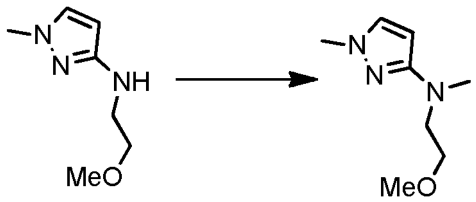

N-(2-methoxyethyl)-N,1-dimethyl-1H-pyrazole-3-amine was obtained as pale yellow oily substance in the same manner as in Example 0741 except that N-(2-methoxyethyl)-1-methyl-1H-pyrazole-3-amine was used instead of the 7-(1-(2-(azetidin-3-yloxy)ethyl)-1H-pyrazol-4-yl)-N-(5-isopropylpyridazin-3-yl)-1,5-naphthyridine-2-amine used in Example 0741.
MSm/z(M+H):170.

<0945-3>

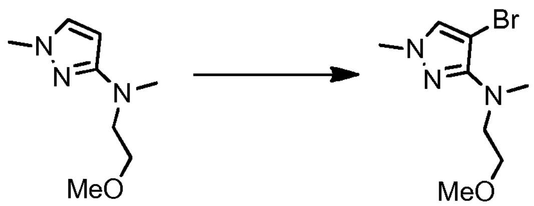

4-Bromo-N-(2-methoxyethyl)-N,1-dimethyl-1H-pyrazole-3-amine was obtained as pale yellow oily substance in the same manner as in Example 0734-2 except that N-(2-methoxyethyl)-N,1-dimethyl-1H-pyrazole-3-amine was used instead of the 2-methyl-5-(1-methyl-1H-pyrazol-3-yl)pyridine used in Example 0734-2.
MSm/z(M+H):250.

<0945-4>

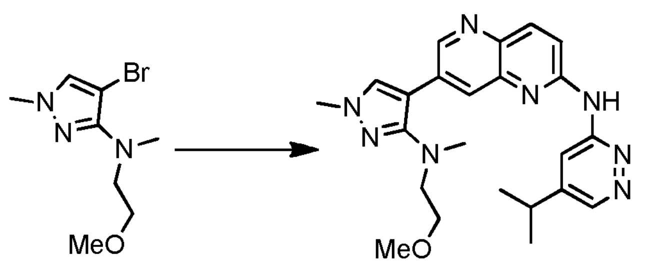

N-(5-isopropylpyridazin-3-yl)-7-(3-((2-methoxyethyl) (methyl)amino)-1-methyl-1H-pyrazol-4-yl)-1,5-naphthyridine-2-amine was obtained as a pale yellow solid in the same manner as in Example 0935-3 except that 4-bromo-N-(2-methoxyethyl)-N,1-dimethyl-1H-pyrazole-3-amine was used instead of the 1-(4-bromo-1-methyl-1H-pyrazol-3-yl)-4-methyl-1,4-diazepan-5-one used in Example 0935-3.
$^1$H-NMR($CDCl_3$)δ:9.07(1H,brs),9.00(1H,d,J=2.0Hz),8.93(1H,d,J=2.0Hz),8.81(1H,d,J=2.0Hz),8.25(1H,s), 8.23(1H,d,J=8.6Hz),7.57(1H,d,J=8.6Hz),7.53(1H,s),3.86(3H,s),3.58(2H,t,J=6.3Hz),3.31-3.26(5H,m),3.09-2.99(1H,m), 2.85(3H,s),1.42(6H,d,J=6.6Hz).

MSm/z(M+H):433.

<Example 0946>

<0946-1>

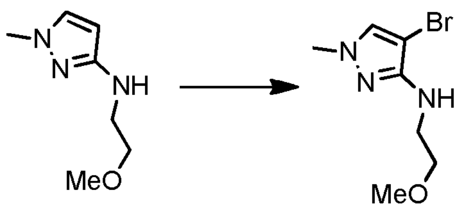

4-Bromo-N-(2-methoxyethyl)-1-methyl-1H-pyrazole-3-amine was obtained as pale yellow oily substance in the same manner as in Example 0734-2 except that N-(2-methoxyethyl)-1-methyl-1H-pyrazole-3-amine was used instead of the 2-methyl-5-(1-methyl-1H-pyrazol-3-yl)pyridine used in Example 0734-2.
MSm/z(M+H):234.

<0946-2>

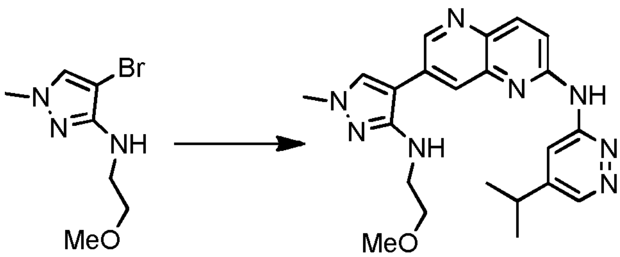

N-(5-isopropylpyridazin-3-yl)-7-(3-((2-methoxyethyl)amino)-1-methyl-1H-pyrazol-4-yl)-1,5-naphthyridine-2-amine was obtained as a yellow solid in the same manner as in Example 0935-3 except that 4-bromo-N-(2-methoxyethyl)-1-methyl-1H-pyrazole-3-amine was used instead of the 1-(4-bromo-1-methyl-1H-pyrazol-3-yl)-4-methyl-1,4-diazepan-5-one used in Example 0935-3.
$^1$H-NMR($CDCl_3$)$\delta$:9.59(1H,brs),8.96(1H,brs),8.86(1H,d,J=2.0Hz),8.82(1H,d,J=2.0Hz),8.24(1H,d,J=8.9H z),8.10(1H,d,J=2.0Hz),7.67(1H,d,J=8.9Hz),7.49(1H,s),4.24(1H,t,J=5.9Hz),3.84(3H,s),3.64(2H,t,J=5.0 Hz),3.54-3.47(2H,m),3.36(3H,s),3.09-3.00(1H,m),1.41(6H,d,J=7.3Hz).
MSm/z(M+H):419.

<Example 0947>

<0947-1>

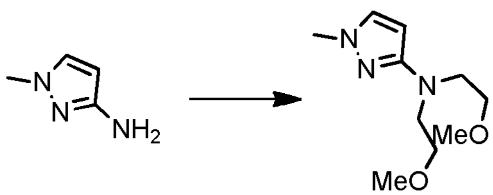

N,N-bis(2-methoxyethyl)-1-methyl-1H-pyrazole-3-amine was obtained as yellow oily substance in the same manner as in Example 0936-1 except that 1-bromo-2-methoxyethane was used instead of the 1,5-dibromopentane used in Example 0936-1.
MSm/z(M+H):214.

<0947-2>

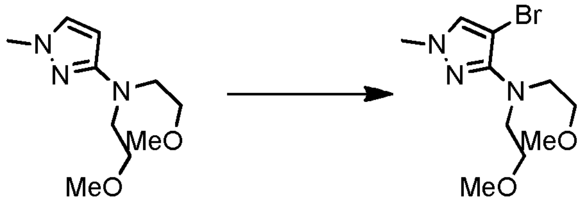

4-Bromo-N,N-bis(2-methoxyethyl)-1-methyl-1H-pyrazole-3-amine was obtained as pale yellow oily substance in the same manner as in Example 0734-2 except that N,N-bis(2-methoxyethyl)-1-methyl-1H-pyrazole-3-amine was used instead of the 2-methyl-5-(1-methyl-1H-pyrazol-3-yl)pyridine used in Example 0734-2.
MSm/z(M+H):292.

<0947-3>

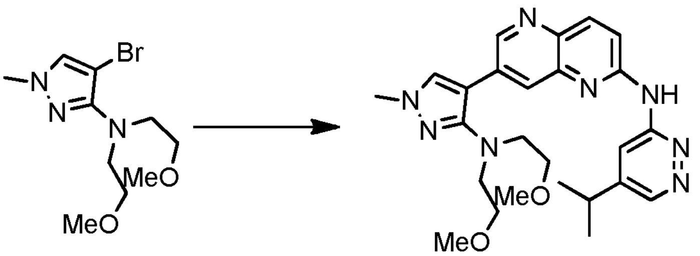

7-(3-(Bis(2-methoxyethyl)amino)-1-methyl-1H-pyrazol-4-yl)-N-(5-isopropylpyridazin-3-yl)-1,5-naphthyridine-2-amine was obtained as a yellow solid in the same manner as in Example 0935-3 except that 4-bromo-N,N-bis(2-methoxyethyl)-1-methyl-1H-pyrazole-3-amine was used instead of the 1-(4-bromo-1-methyl-1H-pyrazol-3-yl)-4-methyl-1,4-diazepan-5-one used in Example 0935-3.
$^1$H-NMR(CDCl$_3$)$\delta$:9.34(1H,brs),9.03(1H,d,J=2.0Hz),8.97(1H,brs),8.81(1H,d,J=2.0Hz),8.30(1H,d,J=2.0Hz),8.24(1H,d,J=9.2Hz),7.61(1H,brs),7.54(1H,s),3.86(3H,s),3.53(4H,t,J=6.0Hz),3.37(4H,t,J=6.0Hz),3.27(6H,s),3.09-3.00(1H,m),1.42(6H,d,J=7.3Hz).
MSm/z(M+H):477.

<Example 0948>

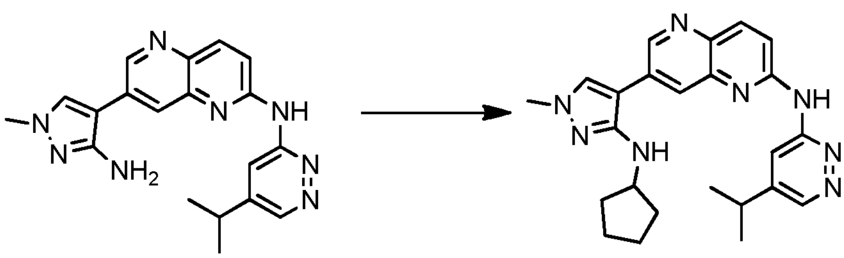

7-(3-(Cyclopentylamino)-1-methyl-1H-pyrazol-4-yl)-N-(5-isopropylpyridazin-3-yl)-1,5-naphthyridine-2-amine was obtained as a pale yellow solid in the same manner as in Example 0830 except that cyclopentanone was used instead of the cyclohexanone used in Example 0830.
$^1$H-NMR($CDCl_3$)δ:9.68(1H,brs),9.02(1H,brs),8.85(1H,d,J=2.0Hz),8.81(1H,brs),8.23(1H,d,J=8.6Hz),8.11( 1H,brs),7.69(1H,brs),7.49(1H,s),4.09-4.02(1H,m),3.84(3H,s),3.73(1H,brs),3.09-2.99(1H,m),2.11-2.04(2H,m),1.75-1.56(6H,m),1.41(6H,d,J=6.9Hz).
MSm/z(M+H):429.

<Example 0949>

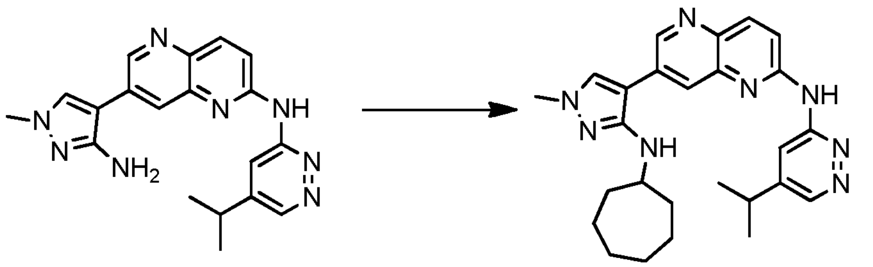

7-(3-(Cycloheptylamino)-1-methyl-1H-pyrazol-4-yl)-N-(5-isopropylpyridazin-3-yl)-1,5-naphthyridine-2-amine was obtained as a pale yellow solid in the same manner as in Example 0830 except that cycloheptanone was used instead of the cyclohexanone used in Example 0830.
$^1$H-NMR($CDCl_3$)δ:8.97(1H,brs),8.85(1H,d,J=2.0Hz),8.80(1H,brs),8.23(1H,d,J=8.6Hz),8.12(1H,brs),7.55( 1H,brs),7.48(1H,s),3.84(3H,s),3.74(1H,brs),3.07-2.98(1H,m),2.12-2.05(2H,m),1.66-1,49(10H,m),1.42(6H,d,J=7.3Hz).
MSm/z(M+H):457.

<Example 0950>

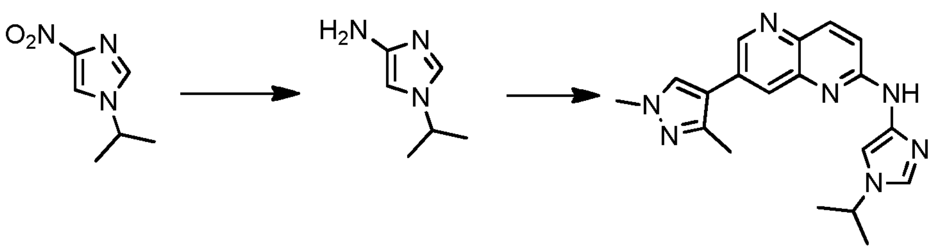

A mixture of 1-isopropyl-4-nitro-1H-imidazole (18 mg) and 2-methyltetrahydrofuran (3 mL) was reacted for 3 minutes using a flow-type hydrogenation reaction apparatus (1 bar, 1.0 mL/min, 30°C, 10% Pd/C). A mixture of tris(dibenzylideneacetone)dipalladium(0) (5 mg), 2-(dicyclohexylphosphino)-3,6-dimethoxy-2',4',6'-triisopropyl-1,1'-biphenyl (6 mg), and 2-methyltetrahydrofuran (0.5 mL)) which had been stirred at 120°C for 5 minutes was added to the reaction mixture, followed by stirring at 80°C for 3 hours. The reaction mixture was cooled to room temperature, the insolubles were filtered off, and the solvent was distilled off under reduced pressure. The obtained residue was purified by silica gel column chromatography (ethyl acetate-methanol, NH silica), thereby obtaining 7-(1,3-dimethyl-1H-pyrazol-4-yl)-N-(1-isopropyl-1H-imidazol-4-yl)-1,5-naphthyridine-2-amine (2.4 mg) as a yellow solid.
$^1$H-NMR(DMSO-$d_6$)δ:10.00(1H,brs),8.71(1H,d,J=2.0Hz),8.17(1H,s),8.08(1H,s),7.99(1H,d,J=9.2Hz),7.82(1H,s),7.71(1H ,brs),7.27(1H,d,J=9.2Hz),4.50-4.41(1H,m),3.84(3H,s),2.41(3H,s),1.48(6H,d,J=6.6Hz).
MSm/z(M+H):348.

<Example 0951>

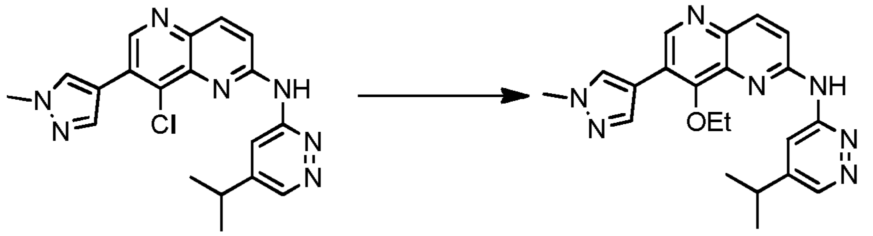

8-Ethoxy-N-(5-isopropylpyridazin-3-yl)-7-(1-methyl-1H-pyrazol-4-yl)-1,5-naphthyridine-2-amine was obtained as a a brown solid in the same manner as in Example 0914 except that a 20% sodium ethoxide/ethanol solution was used instead of the 5 mol/L sodium methoxide/methanol solution used in Example 0914, and ethanol was used instead of the methanol used in Example 0914.
$^1$H-NMR($CD_3OD$)δ:8.92(1H,s),8.75(2H,d,J=19.8Hz),8.31(1H,s),8.19-8.16(2H,m),7.63(1H,d,J=9.2Hz),4.63(2H,q,J=7.3Hz),4.00(3H,s),3.08-3.06(1H,m),1.41(9H,m).
MSm/z(M+H):380.

<Example 0952>

<0952-1>

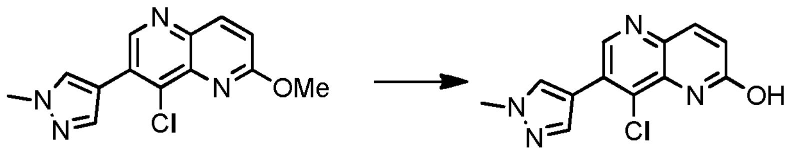

A mixture of 8-chloro-2-methoxy-7-(1-methyl-1H-pyrazol-4-yl)-1,5-naphthyridine (374 mg), 1,4-dioxane (8 mL), and 6 mol/L hydrochloric acid (5 mL) was stirred at 100°C for 3.5 hours. The reaction mixture was cooled to room temperature, and the solvent was distilled off under reduced pressure. Water was added to the obtained residue, and the resultant product was neutralized with sodium hydrogen carbonate. The solid matter was collected by filtration, and washed with water and chloroform, thereby obtaining 8-chloro-7-(1-methyl-1H-pyrazol-4-yl)-1,5-naphthyridin-2-ol (264 mg) as a white solid.
MSm/z(M+H):261.

<0952-2>

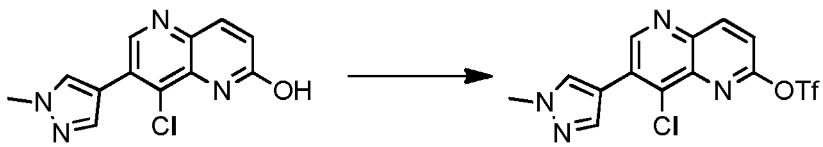

Trifluoromethanesulfonic acid anhydride (255 μL) was added to a mixture of 8-chloro-7-(1-methyl-1H-pyrazol-4-yl)-1,5-naphthyridin-2-ol (264 mg), triethylamine (0.43 mL), and dichloromethane (10 mL) at a temperature of from 0°C to 5°C, followed by stirring at room temperature for 1 hour. After a saturated sodium hydrogen carbonate aqueous solution and ethyl acetate were added to the reaction mixture, an organic layer was collected therefrom by separation, washed with a saturated sodium chloride aqueous solution, and dried over anhydrous sodium sulfate, and the solvent was distilled off under reduced pressure. The obtained residue was purified by silica gel column chromatography (hexane-ethyl acetate-methanol), thereby obtaining 8-chloro-7-(1-methyl-1H-pyrazol-4-yl)-1,5-naphthyridin-2-yl trifluoromethanesulfonate (320 mg) as a pale brown solid.
MSm/z(M+H):393.

<0952-3>

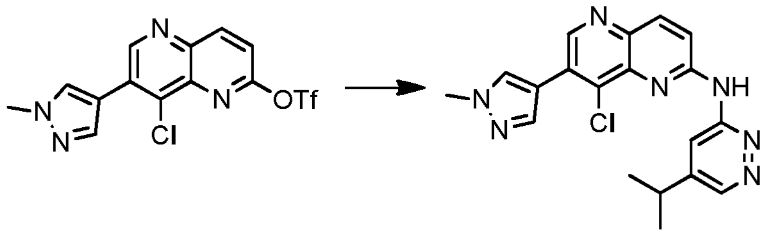

8-Chloro-N-(5-isopropylpyridazin-3-yl)-7-(1-methyl-1H-pyrazol-4-yl)-1,5-naphthyridine-2-amine was obtained as a pale yellow solid in the same manner as in Example 0646-3 except that 8-chloro-7-(1-methyl-1H-pyrazol-4-yl)-1,5-naphthyridin-2-yl trifluoromethanesulfonate was used instead of the 2-chloro-7-(1-(3,3-dimethoxypropyl)-1H-pyrazol-4-yl)-1,5-naphthyridine used in Example 0646-3.
$^1$H-NMR(DMSO-$d_6$)δ:11.05(1H,s),9.26(1H,d,J=2.0Hz),8.99(1H,s),8.88(1H,d,J=2.0Hz),8.57(1H,s),8.28(1H,d,J=9.2Hz),8 .19(1H,s),7.64(1H,d,J=9.2Hz),3.97(3H,s),3.07-2.98(1H,m),1.34(6H,d,J=6.6Hz).
MSm/z(M+H):380.

<Example 0953>

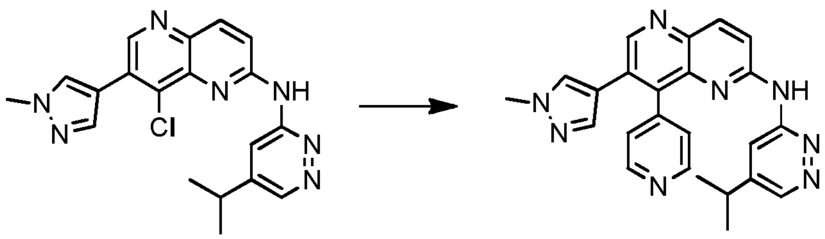

A mixture of 8-chloro-N-(5-isopropylpyridazin-3-yl)-7-(1-methyl-1H-pyrazol-4-yl)-1,5-naphthyridine-2-amine (9.6 mg), 4-pyridylboronic acid (6.9 mg), 1,1'-bis(diphenylphosphino)ferrocenepalladium(II) dichloride-dichloromethane complex (2.3 mg), tripotassium phosphate (17.1 mg), 1,2-dimethoxyethane (1.9 mL), and water (0.1 mL) was stirred at 130°C for 1 hour using a microwave reaction apparatus. The reaction mixture was cooled to room temperature, and purified by silica gel column chromatography (methanol-ethyl acetate, NH silica), thereby obtaining N-(5-isopropylpyridazin-3-yl)-7-(1-methyl-1H-pyrazol-4-yl)-8-(pyridin-4-yl)-1,5-naphthyridine-2-amine (4.6 mg) as a brown solid.
$^1$H-NMR($CD_3OD$)δ:8.91(1H,s),8.73-8.72(2H,m),8.66(1H,s),8.26(1H,d,J=9.2Hz),8.02(1H,s),7.54-7.51(4H,m),7.13(1H,s),3.83(3H,s),2.40-2.38(1H,m),1.16(6H,d,J=6.6Hz).
MSm/z(M+H):423.

<Example 0954>

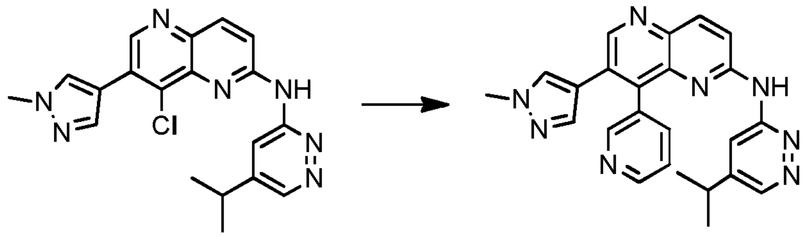

N-(5-isopropylpyridazin-3-yl)-7-(1-methyl-1H-pyrazol-4-yl)-8-(pyridin-3-yl)-1,5-naphthyridine-2-amine was obtained as a brown solid in the same manner as in Example 0953 except that 3-pyridylboronic acid was used instead of the 4-pyridylboronic acid used in Example 0953.
$^1$H-NMR($CD_3OD$)δ:8.92(1H,s),8.71-8.66(2H,m),8.55(1H,s),8.27(1H,d,J=9.2Hz),8.02(1H,s),7.91-7.87(1H,m),7.65-7.46(3H,m),7.06(1H,s),3.83(3H,s),2.48-2.45(1H,m),1.16(6H,d,J=7.3Hz).
MSm/z(M+H):423.

<Example 0955>

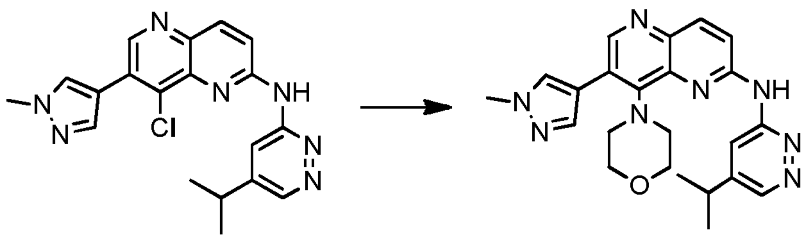

A mixture of 8-chloro-N-(5-isopropylpyridazin-3-yl)-7-(1-methyl-1H-pyrazol-4-yl)-1,5-naphthyridine-2-amine (5.3 mg) and morpholine (1 mL) was stirred at 150°C for 1.5 hours, and further stirred at 160°C for 14 hours, using a microwave reaction apparatus. The reaction mixture was cooled to room temperature, and purified by silica gel column chromatography (methanol-ethyl acetate, NH silica), thereby obtaining N-(5-isopropylpyridazin-3-yl)-7-(1-methyl-1H-pyrazol-4-yl)-8-morpholino-1,5-naphthyridine-2-amine (2.0 mg) as a pale brown solid.
$^1$H-NMR($CD_3OD$)δ:8.80(1H,d,J=2.0Hz),8.65(1H,s),8.22(1H,s),8.19-8.17(2H,m),8.02(1H,s),7.77(1H,d,J=9.2Hz),4.01(3H,s),3.80-3.79(4H,m),3.51-3.48(4H,m),3.19-3.14(1H,m),1.37(6H,d,J=6.6Hz).
MSm/z(M+H):431.

Next, the utility of the compound of the invention will be described in the following Test Examples.

Test Example 1: PI3Kα Enzyme Assay

In a PI3Kα enzyme assay, a product (manufactured by Carna Biosciences, Inc.) obtained by biotinylating a human PI3Kα protein produced by using a baculovirus expression system was used. In addition, in the detection of enzyme activity, ADP-GLO KINASE ASSAY (trade name, manufactured by PROMEGA Corporation) was used.

10 μL of a reaction liquid (4.5 nmol/L PI3Kα, 40 mmol/L Tris-HCl, 20 mmol/L $MgCl_2$, 0.1% BSA, pH 7.5) including the human PI3Kα protein and a test compound having a predetermined concentration was allowed to stand at room temperature for 15 minutes. Then, 5 μL of a mixture liquid of L-α-phosphatidylinositol 4,5-diphosphate (PIP2) (manufactured by Sigma-Aldrich Co. LLC.) and ATP (manufactured by Sigma-Aldrich Co. LLC.), which are substrates, was added thereto such that final concentrations thereof became 50 μmol/L and 100 μmol/L, respectively, and an enzyme reaction was performed by allowing to stand at 25°C for 60 minutes.

After the reaction, the enzyme reaction was stopped by adding 15 μL of an ADP-GLO REAGENT (trade name, manufactured by PROMEGA Corporation) thereto and allowing to stand at room temperature for 60 minutes. Then, 30 μL of a KINASE DETECTION REAGENT (trade name, manufactured by PROMEGA Corporation) was added thereto, followed by allowing to stand at room temperature for 40 minutes, and ADP produced by the enzyme reaction was quantified by emission intensity.

ENVISION (trade name, manufactured by Perkin Elmer Inc.) was used for the measurement of the emission

intensity.

Test Example 2: ERK2 Enzyme Assay (1)

In an ERK2 enzyme assay, GLUTATHIONE S-TRANSFERASE (GST) FUSION HUMAN ERK2 PROTEIN (manufactured by Carna Biosciences, Inc.), which was produced by using an E. coli expression system, was used.

12 μL of a reaction liquid (2.5 nmol/L ERK2, 100 mmol/L Hepes, 10 mmol/L $MgCl_2$, 1.5 mmol/L DTT, 0.003% Brij35, pH 7.5) including ERK2 protein and a test compound having a predetermined concentration was allowed to stand at room temperature for 15 minutes. Then, 3 μL of a mixture liquid of FL-PEPTIDE8 (5-FAM-IPTSPITTTYFFFKKK-COOH) (manufactured by Caliper Life Sciences), which is substrate peptide, and ATP (manufactured by Sigma-Aldrich Co. LLC.) was added thereto such that the final concentrations thereof became 4.25 μmol/L and 43.1 μmol/L, respectively, and an enzyme reaction was performed by allowing to stand at 25°C for 120 minutes.

After the reaction, the enzyme reaction was stopped by adding 30 μL of a reaction stopper liquid (100 mmol/L HEPES, 11.2 mmol/L EDTA, 5.6% Brij35, pH 7.5) including a 0.5% COATING REAGENT 8 (trade name, manufactured by Perkin Elmer Inc.) thereto. Then, phosphorylated peptide and non-phosphorylated peptide were separated using LABCHIP EZ READER (trade name, manufactured by Caliper Life Sciences), and the porportion of the phosphorylated peptide was quantified.

Test Example 2: ERK2 Enzyme Assay (2)

In an ERK2 enzyme assay, GLUTATHIONE S-TRANSFERASE (GST) FUSION HUMAN ERK2 PROTEIN (manufactured by Carna Biosciences, Inc.), which was produced by using an E. coli expression system, was used.

12 μL of a reaction liquid (1.25 nmol/L ERK2, 100 mmol/L Hepes, 10 mmol/L $MgCl_2$, 1.5 mmol/L DTT, 0.003% Brij35, pH 7.5) including ERK2 protein and a test compound having a predetermined concentration was allowed to stand at room temperature for 15 minutes. Then, 3 μL of a mixture liquid of FL-PEPTIDE8 (5-FAM-IPTSPITTTYFFFKKK-COOH) (manufactured by Caliper Life Sciences), which is substrate peptide, and ATP (manufactured by Sigma-Aldrich Co. LLC.) was added thereto such that the final concentrations thereof became 4.25 μmol/L and 1 mmol/L, respectively, and an enzyme reaction was performed by allowing to stand at 25°C for 120 minutes.

After the reaction, the enzyme reaction was stopped by adding 30 μL of a reaction stopper liquid (100 mmol/L HEPES, 11.2 mmol/L EDTA, 5.6% Brij35, pH 7.5) including a 0.5% COATING REAGENT 8 (trade name, manufactured by Perkin Elmer Inc.) thereto. Then, phosphorylated peptide and non-phosphorylated peptide were separated using LABCHIP EZ READER (trade name, manufactured by Caliper Life Sciences), and the porportion of the phosphorylated peptide was quantified.

The inhibitory activities of the compounds were evaluated using $IC_{50}$ (50% inhibitory concentration). The $IC_{50}$ was calculated using XLFIT (trade name, manufactured by ID Business Solutions).

The results are shown in Tables 1-1 to 1-10.

Evaluation Criteria

| | |
|---|---|
| +++ | $IC_{50}$ < 1 μmol/L |
| ++ | 1 μmol/L ≤ $IC_{50}$ < 10 μmol/L |
| + | 10 μmol/L ≤ $IC_{50}$ < 50 μmol/L |

[Table 1-1]

| Example No. | PI3Kα enzyme inhibitory activity | ERK2 enzyme inhibitory activity | | Example No. | PI3Kα enzyme inhibitory activity | ERK2 enzyme inhibitory activity | |
|---|---|---|---|---|---|---|---|
| | | (1) | (2) | | | (1) | (2) |
| 0001 | +++ | +++ | | 0051 | ++ | +++ | |
| 0002 | +++ | +++ | | 0052 | ++ | +++ | |
| 0003 | ++ | +++ | | 0053 | ++ | +++ | |
| 0004* | +++ | +++ | | 0054 | +++ | +++ | |
| 0005* | +++ | | | 0055 | ++ | +++ | |
| 0006* | +++ | +++ | | 0056 | + | +++ | |
| 0007* | +++ | +++ | | 0057 | + | ++ | |
| 0008* | +++ | ++ | | 0058 | ++ | +++ | |

(continued)

| Example No. | PI3Kα enzyme inhibitory activity | ERK2 enzyme inhibitory activity | | Example No. | PI3Kα enzyme inhibitory activity | ERK2 enzyme inhibitory activity | |
|---|---|---|---|---|---|---|---|
| | | (1) | (2) | | | (1) | (2) |
| 0009* | ++ | ++ | | 0059 | ++ | +++ | |
| 0010 | ++ | +++ | | 0060 | + | +++ | |
| 0011* | +++ | ++ | | 0061 | ++ | +++ | |
| 0012* | +++ | ++ | | 0062 | ++ | +++ | |
| 0013* | +++ | ++ | | 0063 | +++ | +++ | |
| 0014 | +++ | +++ | +++ | 0064 | ++ | +++ | |
| 0015 | +++ | +++ | +++ | 0065 | ++ | +++ | |
| 0016 | +++ | +++ | +++ | 0066 | +++ | +++ | |
| 0017 | +++ | +++ | +++ | 0067 | +++ | +++ | |
| 0018 | +++ | +++ | +++ | 0068 | ++ | +++ | |
| 0019 | +++ | +++ | | 0069 | + | ++ | |
| 0020 | +++ | +++ | | 0070 | +++ | +++ | |
| 0021 | +++ | +++ | +++ | 0071 | +++ | +++ | +++ |
| 0022 | ++ | + | | 0072 | +++ | +++ | +++ |
| 0023 | ++ | ++ | | 0073 | +++ | +++ | |
| 0024 | ++ | + | | 0074 | +++ | +++ | |
| 0025 | ++ | +++ | +++ | 0075 | +++ | ++ | |
| 0026 | +++ | +++ | +++ | 0076 | +++ | +++ | |
| 0027 | +++ | +++ | +++ | 0077 | ++ | ++ | |
| 0028 | | +++ | | 0078 | +++ | +++ | |
| 0029 | +++ | +++ | | 0079 | ++ | +++ | |
| 0030 | +++ | +++ | | 0080 | | +++ | |
| 0031 | | ++ | | 0081 | ++ | +++ | |
| 0032 | +++ | +++ | +++ | 0082 | +++ | +++ | |
| 0033 | ++ | ++ | | 0083 | +++ | +++ | |
| 0034 | ++ | | | 0084 | +++ | +++ | +++ |
| 0035 | +++ | ++ | | 0085 | +++ | +++ | +++ |
| 0036 | ++ | ++ | | 0086 | +++ | +++ | +++ |
| 0037 | ++ | +++ | | 0087 | +++ | +++ | +++ |
| 0038 | + | ++ | | 0088 | +++ | +++ | +++ |
| 0039 | | +++ | | 0089 | +++ | +++ | +++ |
| 0040 | + | +++ | | 0090 | +++ | +++ | +++ |
| 0041 | + | +++ | | 0091 | +++ | +++ | +++ |
| 0042* | | + | | 0092 | ++ | +++ | +++ |
| 0043* | ++ | +++ | | 0093 | ++ | +++ | + |
| 0044 | | ++ | | 0094 | +++ | + | + |
| 0045 | + | +++ | | 0095 | +++ | +++ | ++ |
| 0046 | ++ | +++ | | 0096 | +++ | +++ | +++ |
| 0047 | +++ | | | 0097 | +++ | +++ | |
| 0048 | ++ | +++ | | 0098 | +++ | +++ | |
| 0049 | | ++ | | 0099 | +++ | +++ | |
| 0050 | ++ | +++ | | 0100 | ++ | +++ | |

*not in accordance with the present invention

[Table 1-2]

| Example No. | PI3Kα enzyme inhibitory activity | ERK2 enzyme inhibitory activity (1) | ERK2 enzyme inhibitory activity (2) | Example No. | PI3Kα enzyme inhibitory activity | ERK2 enzyme inhibitory activity (1) | ERK2 enzyme inhibitory activity (2) |
|---|---|---|---|---|---|---|---|
| 0101 | +++ | +++ | | 0151 | + | +++ | ++ |
| 0102 | +++ | +++ | | 0152 | ++ | +++ | |
| 0103 | +++ | +++ | | 0153 | ++ | +++ | |
| 0104 | ++ | +++ | | 0154 | ++ | +++ | |
| 0105 | +++ | +++ | | 0155 | ++ | | |
| 0106 | +++ | +++ | | 0156 | +++ | +++ | |
| 0107 | +++ | +++ | | 0157 | +++ | +++ | +++ |
| 0108 | ++ | +++ | | 0158 | +++ | +++ | ++ |
| 0109 | +++ | ++ | | 0159 | +++ | +++ | +++ |
| 0110 | +++ | +++ | | 0160 | +++ | +++ | +++ |
| 0111 | | +++ | +++ | 0161 | +++ | +++ | +++ |
| 0112 | +++ | +++ | +++ | 0162 | +++ | +++ | +++ |
| 0113 | | +++ | +++ | 0163 | +++ | +++ | +++ |
| 0114 | +++ | +++ | +++ | 0164 | | +++ | |
| 0115 | +++ | +++ | +++ | 0165 | + | +++ | |
| 0116 | +++ | +++ | +++ | 0166 | ++ | +++ | |
| 0117 | +++ | +++ | +++ | 0167 | +++ | +++ | +++ |
| 0118 | +++ | +++ | +++ | 0168 | | +++ | |
| 0119 | +++ | +++ | | 0169 | | +++ | +++ |
| 0120 | +++ | +++ | +++ | 0170 | ++ | +++ | +++ |
| 0121 | +++ | +++ | +++ | 0171 | + | +++ | |
| 0122 | +++ | +++ | +++ | 0172 | +++ | +++ | +++ |
| 0123 | +++ | +++ | | 0173 | ++ | +++ | +++ |
| 0124 | +++ | +++ | ++ | 0174 | | +++ | |
| 0125 | +++ | +++ | | 0175 | +++ | +++ | |
| 0126 | +++ | +++ | | 0176 | +++ | +++ | |
| 0127 | ++ | +++ | | 0177 | +++ | +++ | |
| 0128 | +++ | +++ | | 0178 | +++ | +++ | |
| 0129 | +++ | +++ | ++ | 0179 | +++ | +++ | |
| 0130 | +++ | +++ | +++ | 0180 | ++ | +++ | |
| 0131 | +++ | +++ | +++ | 0181 | +++ | +++ | |
| 0132 | +++ | +++ | +++ | 0182 | ++ | +++ | |
| 0133 | +++ | +++ | +++ | 0183 | ++ | +++ | |
| 0134 | +++ | +++ | +++ | 0184 | ++ | +++ | |
| 0135 | +++ | +++ | ++ | 0185 | +++ | +++ | |
| 0136 | +++ | +++ | | 0186 | +++ | +++ | |
| 0137 | +++ | +++ | +++ | 0187 | +++ | +++ | |
| 0138 | +++ | +++ | +++ | 0188 | +++ | +++ | |
| 0139 | +++ | +++ | +++ | 0189 | +++ | +++ | +++ |
| 0140 | +++ | +++ | +++ | 0190 | ++ | +++ | |
| 0141 | ++ | ++ | | 0191 | +++ | +++ | |
| 0142 | +++ | +++ | +++ | 0192 | +++ | +++ | |
| 0143 | ++ | +++ | | 0193 | +++ | +++ | |
| 0144 | +++ | +++ | | 0194 | ++ | +++ | |
| 0145 | +++ | +++ | +++ | 0195 | ++ | +++ | |
| 0146 | +++ | +++ | | 0196 | ++ | +++ | |
| 0147 | +++ | +++ | +++ | 0197 | +++ | +++ | |
| 0148 | +++ | +++ | +++ | 0198 | | +++ | |

(continued)

| Example No. | PI3Kα enzyme inhibitory activity | ERK2 enzyme inhibitory activity (1) | (2) | Example No. | PI3Kα enzyme inhibitory activity | ERK2 enzyme inhibitory activity (1) | (2) |
|---|---|---|---|---|---|---|---|
| 0149 | | ++ | | 0199 | ++ | ++ | |
| 0150 | +++ | +++ | ++ | 0200 | ++ | ++ | |

[Table 1-3]

| Example No. | PI3Kα enzyme inhibitory activity | ERK2 enzyme inhibitory activity (1) | (2) | Example No. | PI3Kα enzyme inhibitory activity | ERK2 enzyme inhibitory activity (1) | (2) |
|---|---|---|---|---|---|---|---|
| 0201 | ++ | +++ | | 0251 | ++ | +++ | +++ |
| 0202 | + | +++ | | 0252 | ++ | +++ | +++ |
| 0203 | | +++ | | 0253 | ++ | +++ | +++ |
| 0204 | | +++ | | 0254 | +++ | +++ | |
| 0205 | +++ | +++ | | 0255 | ++ | +++ | |
| 0206 | + | +++ | | 0256 | +++ | +++ | |
| 0207 | +++ | +++ | | 0257 | ++ | +++ | +++ |
| 0208 | +++ | +++ | | 0258 | +++ | +++ | +++ |
| 0209 | +++ | +++ | | 0259 | +++ | +++ | |
| 0210 | +++ | +++ | | 0260 | +++ | +++ | |
| 0211 | | +++ | | 0261 | +++ | +++ | +++ |
| 0212 | | +++ | | 0262 | +++ | +++ | +++ |
| 0213 | | +++ | | 0263 | +++ | +++ | |
| 0214 | ++ | +++ | +++ | 0264 | ++ | +++ | |
| 0215 | ++ | +++ | +++ | 0265 | +++ | +++ | +++ |
| 0216 | | +++ | | 0266 | +++ | +++ | +++ |
| 0217 | | +++ | +++ | 0267 | +++ | +++ | +++ |
| 0218 | | +++ | | 0268* | +++ | +++ | |
| 0219 | + | ++ | | 0269* | +++ | +++ | |
| 0220 | +++ | ++ | | 0270* | +++ | +++ | |
| 0221 | +++ | +++ | | 0271* | | +++ | |
| 0222 | +++ | +++ | | 0272* | ++ | +++ | |
| 0223 | ++ | +++ | | 0273* | +++ | +++ | |
| 0224 | +++ | +++ | | 0274* | +++ | ++ | |
| 0225 | ++ | +++ | | 0275* | +++ | +++ | |
| 0226 | ++ | +++ | +++ | 0276* | +++ | +++ | ++ |
| 0227 | +++ | +++ | +++ | 0277 | ++ | ++ | |
| 0228 | +++ | +++ | | 0278* | +++ | +++ | |
| 0229 | +++ | +++ | | 0279* | +++ | +++ | |
| 0230 | | +++ | | 0280* | + | ++ | |
| 0231 | +++ | +++ | | 0281* | ++ | ++ | |
| 0232 | +++ | +++ | | 0282* | ++ | ++ | |
| 0233 | +++ | +++ | | 0283* | +++ | +++ | |
| 0234 | +++ | +++ | | 0284* | +++ | ++ | |
| 0235 | +++ | +++ | +++ | 0285* | +++ | +++ | |
| 0236 | +++ | +++ | +++ | 0286* | ++ | ++ | |
| 0237 | +++ | +++ | | 0287* | +++ | +++ | |
| 0238 | +++ | +++ | | 0288* | +++ | +++ | |
| 0239 | +++ | +++ | | 0289* | +++ | ++ | |

(continued)

| Example No. | PI3Kα enzyme inhibitory activity | ERK2 enzyme inhibitory activity (1) | (2) | Example No. | PI3Kα enzyme inhibitory activity | ERK2 enzyme inhibitory activity (1) | (2) |
|---|---|---|---|---|---|---|---|
| 0240 | +++ | +++ | +++ | 0290 | +++ | +++ | +++ |
| 0241 | ++ | +++ | +++ | 0291 | +++ | +++ | +++ |
| 0242 | +++ | +++ | +++ | 0292 | +++ | +++ | +++ |
| 0243 | +++ | +++ | +++ | 0293 | +++ | +++ | +++ |
| 0244 | +++ | +++ | | 0294 | +++ | +++ | +++ |
| 0245 | +++ | +++ | | 0295 | +++ | +++ | +++ |
| 0246 | ++ | +++ | +++ | 0296 | +++ | +++ | |
| 0247 | ++ | +++ | +++ | 0297 | +++ | +++ | |
| 0248 | ++ | +++ | +++ | 0298 | +++ | +++ | |
| 0249 | +++ | +++ | | 0299 | + | +++ | |
| 0250 | +++ | +++ | +++ | 0300 | ++ | +++ | |

*not in accordance with the present invention

[Table 1-4]

| Example No. | PI3Kα enzyme inhibitory activity | ERK2 enzyme inhibitory activity (1) | (2) | Example No. | PI3Kα enzyme inhibitory activity | ERK2 enzyme inhibitory activity (1) | (2) |
|---|---|---|---|---|---|---|---|
| 0301 | +++ | +++ | | 0351 | +++ | +++ | +++ |
| 0302 | +++ | +++ | | 0352 | +++ | +++ | +++ |
| 0303 | +++ | +++ | +++ | 0353 | +++ | +++ | +++ |
| 0304 | +++ | +++ | | 0354 | +++ | +++ | +++ |
| 0305 | +++ | +++ | | 0355 | +++ | +++ | +++ |
| 0306 | +++ | +++ | | 0356 | +++ | +++ | +++ |
| 0307 | +++ | +++ | | 0357 | | ++ | ++ |
| 0308 | ++ | +++ | | 0358 | +++ | +++ | +++ |
| 0309 | ++ | +++ | | 0359 | +++ | +++ | ++ |
| 0310 | ++ | +++ | | 0360 | +++ | +++ | +++ |
| 0311 | ++ | +++ | +++ | 0361 | ++ | +++ | |
| 0312 | +++ | +++ | | 0362 | +++ | +++ | |
| 0313 | ++ | +++ | | 0363 | +++ | +++ | |
| 0314 | ++ | +++ | | 0364 | +++ | +++ | +++ |
| 0315 | ++ | +++ | | 0365 | +++ | +++ | +++ |
| 0316 | +++ | +++ | +++ | 0366 | +++ | +++ | |
| 0317 | +++ | +++ | | 0367 | +++ | +++ | |
| 0318 | +++ | +++ | +++ | 0368 | +++ | +++ | +++ |
| 0319 | +++ | +++ | | 0369 | ++ | +++ | |
| 0320 | +++ | +++ | +++ | 0370 | +++ | +++ | +++ |
| 0321 | +++ | +++ | +++ | 0371 | +++ | +++ | |
| 0322 | +++ | +++ | +++ | 0372 | ++ | +++ | +++ |
| 0323 | +++ | +++ | | 0373 | +++ | +++ | |
| 0324 | +++ | +++ | | 0374 | +++ | +++ | |
| 0325 | +++ | +++ | | 0375 | +++ | +++ | |
| 0326 | +++ | +++ | | 0376 | +++ | +++ | |
| 0327 | ++ | +++ | +++ | 0377 | +++ | +++ | |
| 0328 | | +++ | | 0378 | +++ | +++ | |
| 0329 | +++ | +++ | | 0379 | +++ | +++ | |

(continued)

| Example No. | PI3Kα enzyme inhibitory activity | ERK2 enzyme inhibitory activity | | Example No. | PI3Kα enzyme inhibitory activity | ERK2 enzyme inhibitory activity | |
|---|---|---|---|---|---|---|---|
| | | (1) | (2) | | | (1) | (2) |
| 0330 | ++ | +++ | | 0380 | +++ | +++ | |
| 0331 | | ++ | | 0381 | +++ | +++ | |
| 0332 | + | +++ | | 0382 | +++ | +++ | +++ |
| 0333 | ++ | +++ | | 0383 | ++ | +++ | +++ |
| 0334 | +++ | +++ | | 0384 | +++ | +++ | +++ |
| 0335 | + | +++ | | 0385 | +++ | +++ | |
| 0336 | +++ | +++ | +++ | 0386 | +++ | +++ | |
| 0337 | +++ | +++ | +++ | 0387 | +++ | +++ | |
| 0338 | +++ | +++ | +++ | 0388 | ++ | +++ | |
| 0339 | +++ | +++ | +++ | 0389 | +++ | +++ | |
| 0340 | | +++ | | 0390 | +++ | +++ | |
| 0341 | +++ | +++ | +++ | 0391 | +++ | +++ | |
| 0342 | +++ | +++ | +++ | 0392 | +++ | +++ | |
| 0343 | +++ | +++ | +++ | 0393 | ++ | +++ | |
| 0344 | +++ | +++ | +++ | 0394 | +++ | +++ | |
| 0345 | +++ | +++ | +++ | 0395 | +++ | +++ | |
| 0346 | +++ | +++ | +++ | 0396 | + | +++ | |
| 0347 | +++ | +++ | +++ | 0397 | +++ | +++ | |
| 0348 | ++ | +++ | +++ | 0398 | +++ | +++ | |
| 0349 | | +++ | ++ | 0399 | ++ | +++ | |
| 0350 | ++ | +++ | +++ | 0400 | +++ | +++ | |

*not in accordance with the present invention

[Table 1-5]

| Example No. | PI3Kα enzyme inhibitory activity | ERK2 enzyme inhibitory activity | | Example No. | PI3Kα enzyme inhibitory activity | ERK2 enzyme inhibitory activity | |
|---|---|---|---|---|---|---|---|
| | | (1) | (2) | | | (1) | (2) |
| 0401 | +++ | +++ | +++ | 0451 | +++ | | +++ |
| 0402 | +++ | +++ | +++ | 0452 | +++ | | +++ |
| 0403 | +++ | +++ | +++ | 0453 | +++ | | +++ |
| 0404 | +++ | +++ | | 0454 | +++ | | +++ |
| 0405 | +++ | +++ | | 0455 | +++ | | +++ |
| 0406 | +++ | +++ | | 0456 | +++ | | +++ |
| 0407 | ++ | +++ | | 0457 | +++ | | + |
| 0408* | | +++ | | 0458 | +++ | | + |
| 0409 | +++ | ++ | | 0459 | +++ | | +++ |
| 0410* | +++ | | +++ | 0460 | +++ | | +++ |
| 0411 | +++ | | +++ | 0461 | +++ | | +++ |
| 0412 | +++ | | +++ | 0462 | +++ | | +++ |
| 0413 | +++ | | +++ | 0463 | +++ | | +++ |
| 0414 | +++ | | +++ | 0464 | +++ | | +++ |
| 0415 | +++ | | +++ | 0465 | +++ | | +++ |
| 0416 | +++ | | +++ | 0466 | +++ | | +++ |
| 0417 | +++ | | +++ | 0467 | +++ | | +++ |
| 0418 | +++ | | +++ | 0468 | +++ | | +++ |
| 0419 | +++ | | +++ | 0469 | +++ | | +++ |

(continued)

| Example No. | PI3Kα enzyme inhibitory activity | ERK2 enzyme inhibitory activity | | Example No. | PI3Kα enzyme inhibitory activity | ERK2 enzyme inhibitory activity | |
|---|---|---|---|---|---|---|---|
| | | (1) | (2) | | | (1) | (2) |
| 0420 | +++ | | +++ | 0470 | +++ | | +++ |
| 0421 | +++ | | +++ | 0471 | +++ | +++ | +++ |
| 0422 | +++ | | +++ | 0472 | +++ | +++ | +++ |
| 0423 | +++ | | +++ | 0473 | +++ | +++ | +++ |
| 0424 | +++ | | +++ | 0474 | +++ | +++ | +++ |
| 0425 | +++ | | +++ | 0475 | +++ | +++ | +++ |
| 0426 | +++ | | +++ | 0476 | +++ | +++ | |
| 0427 | +++ | | +++ | 0477 | +++ | +++ | |
| 0428 | +++ | | +++ | 0478 | +++ | | +++ |
| 0429 | +++ | | +++ | 0479 | +++ | | +++ |
| 0430 | +++ | | +++ | 0480 | +++ | | +++ |
| 0431 | +++ | | +++ | 0481 | ++ | | +++ |
| 0432 | +++ | | +++ | 0482 | +++ | | +++ |
| 0433 | +++ | | +++ | 0483 | +++ | | +++ |
| 0434 | +++ | | +++ | 0484 | +++ | | +++ |
| 0435 | +++ | | +++ | 0485 | +++ | | +++ |
| 0436 | +++ | | +++ | 0486 | +++ | | +++ |
| 0437 | +++ | | +++ | 0487 | +++ | | +++ |
| 0438 | +++ | | +++ | 0488 | +++ | | +++ |
| 0439 | +++ | | +++ | 0489 | + | | ++ |
| 0440 | +++ | | +++ | 0490 | +++ | | +++ |
| 0441 | +++ | | +++ | 0491 | +++ | | +++ |
| 0442 | +++ | | +++ | 0492 | +++ | | +++ |
| 0443 | +++ | | +++ | 0493 | +++ | | +++ |
| 0444 | +++ | | +++ | 0494 | +++ | | +++ |
| 0445 | +++ | | +++ | 0495 | +++ | | +++ |
| 0446 | +++ | | +++ | 0496 | +++ | | +++ |
| 0447* | +++ | | ++ | 0497 | ++ | | +++ |
| 0448* | +++ | | ++ | 0498 | +++ | | +++ |
| 0449 | +++ | | +++ | 0499 | +++ | | +++ |
| 0450 | +++ | | +++ | 0500 | +++ | | +++ |

*not in accordance with the present invention

[Table 1-6]

| Example No. | PI3Kα enzyme inhibitory activity | ERK2 enzyme inhibitory activity | | Example No. | PI3Kα enzyme inhibitory activity | ERK2 enzyme inhibitory activity | |
|---|---|---|---|---|---|---|---|
| | | (1) | (2) | | | (1) | (2) |
| 0501 | ++ | | ++ | 0551 | +++ | | + |
| 0502 | +++ | | +++ | 0552 | +++ | | +++ |
| 0503 | +++ | | +++ | 0553 | +++ | | +++ |
| 0504 | +++ | | +++ | 0554 | +++ | | +++ |
| 0505 | ++ | | +++ | 0555 | +++ | | +++ |
| 0506 | ++ | | +++ | 0556 | +++ | | +++ |
| 0507 | +++ | | +++ | 0557 | +++ | | +++ |
| 0508 | +++ | | +++ | 0558 | +++ | | +++ |
| 0509 | ++ | | +++ | 0559 | + | | +++ |

(continued)

| Example No. | PI3Kα enzyme inhibitory activity | ERK2 enzyme inhibitory activity | | Example No. | PI3Kα enzyme inhibitory activity | ERK2 enzyme inhibitory activity | |
|---|---|---|---|---|---|---|---|
| | | (1) | (2) | | | (1) | (2) |
| 0510 | ++ | | +++ | 0560 | +++ | | +++ |
| 0511 | ++ | | +++ | 0561 | +++ | | +++ |
| 0512 | ++ | | +++ | 0562 | +++ | | +++ |
| 0513 | | | +++ | 0563 | +++ | | +++ |
| 0514 | ++ | | +++ | 0564 | +++ | | +++ |
| 0515 | +++ | | +++ | 0565 | +++ | | +++ |
| 0516 | + | | | 0566 | +++ | | +++ |
| 0517 | +++ | | +++ | 0567 | +++ | | +++ |
| 0518 | +++ | | +++ | 0568 | +++ | | +++ |
| 0519 | ++ | | +++ | 0569 | +++ | | +++ |
| 0520 | ++ | | +++ | 0570 | | | +++ |
| 0521 | +++ | | +++ | 0571 | +++ | | +++ |
| 0522 | +++ | | +++ | 0572 | +++ | | +++ |
| 0523 | +++ | | +++ | 0573 | +++ | | +++ |
| 0524 | + | | +++ | 0574 | ++ | | +++ |
| 0525 | +++ | | +++ | 0575 | +++ | | +++ |
| 0526 | +++ | | +++ | 0576 | +++ | | +++ |
| 0527 | +++ | | ++ | 0577 | +++ | | +++ |
| 0528 | +++ | | +++ | 0578 | +++ | | +++ |
| 0529 | +++ | | +++ | 0579 | +++ | | +++ |
| 0530 | +++ | | +++ | 0580 | +++ | | +++ |
| 0531 | +++ | | +++ | 0581 | +++ | | +++ |
| 0532 | +++ | | +++ | 0582 | +++ | | +++ |
| 0533 | +++ | | +++ | 0583 | +++ | | +++ |
| 0534 | +++ | | +++ | 0584 | +++ | | +++ |
| 0535 | +++ | | +++ | 0585 | +++ | | +++ |
| 0536 | +++ | | +++ | 0586 | +++ | | +++ |
| 0537 | +++ | | +++ | 0587 | +++ | | +++ |
| 0538 | +++ | | +++ | 0588 | ++ | +++ | +++ |
| 0539 | +++ | | +++ | 0589 | +++ | +++ | +++ |
| 0540 | +++ | | ++ | 0590 | ++ | | +++ |
| 0541 | +++ | | ++ | 0591 | ++ | | +++ |
| 0542 | +++ | | +++ | 0592 | +++ | | +++ |
| 0543 | +++ | | +++ | 0593 | ++ | | +++ |
| 0544 | +++ | | +++ | 0594 | +++ | | +++ |
| 0545 | +++ | | +++ | 0595 | ++ | | +++ |
| 0546 | +++ | | | 0596 | +++ | | +++ |
| 0547 | +++ | | +++ | 0597 | ++ | | +++ |
| 0548 | +++ | | +++ | 0598 | ++ | | +++ |
| 0549 | +++ | | +++ | 0599 | ++ | | +++ |
| 0550 | +++ | | +++ | 0600 | ++ | | +++ |

[Table 1-7]

| Example No. | PI3Kα enzyme inhibitory activity | ERK2 enzyme inhibitory activity | | Example No. | PI3Kα enzyme inhibitory activity | ERK2 enzyme inhibitory activity | |
|---|---|---|---|---|---|---|---|
| | | (1) | (2) | | | (1) | (2) |
| 0601 | ++ | | +++ | 0651 | +++ | | +++ |
| 0602 | +++ | | +++ | 0652* | +++ | | +++ |
| 0603 | +++ | | +++ | 0653 | +++ | | +++ |
| 0604 | + | | +++ | 0654* | +++ | | ++ |
| 0605 | +++ | | +++ | 0655* | ++ | | ++ |
| 0606 | ++ | | +++ | 0656* | +++ | | ++ |
| 0607 | +++ | | +++ | 0657 | +++ | | +++ |
| 0608 | +++ | | +++ | 0658 | +++ | | +++ |
| 0609 | +++ | | +++ | 0659 | +++ | | +++ |
| 0610 | +++ | | +++ | 0660 | +++ | | +++ |
| 0611 | +++ | | +++ | 0661 | +++ | | +++ |
| 0612 | +++ | | +++ | 0662 | +++ | | ++ |
| 0613 | +++ | | +++ | 0663* | ++ | | ++ |
| 0614 | +++ | | +++ | 0664* | ++ | | ++ |
| 0615 | +++ | | +++ | 0665 | +++ | | +++ |
| 0616 | +++ | | +++ | 0666 | +++ | | +++ |
| 0617 | +++ | | +++ | 0667 | +++ | | +++ |
| 0618 | +++ | | +++ | 0668* | | | ++ |
| 0619 | +++ | | +++ | 0669* | ++ | | ++ |
| 0620 | +++ | | +++ | 0670 | +++ | | +++ |
| 0621 | ++ | | +++ | 0671 | +++ | | +++ |
| 0622 | +++ | | +++ | 0672 | +++ | | +++ |
| 0623 | ++ | | ++ | 0673 | +++ | | +++ |
| 0624 | ++ | | +++ | 0674 | +++ | | +++ |
| 0625 | +++ | | ++ | 0675* | + | | |
| 0626 | +++ | | +++ | 0676 | +++ | | +++ |
| 0627 | ++ | | +++ | 0677* | ++ | | |
| 0628 | + | | +++ | 0678 | +++ | | +++ |
| 0629 | +++ | | +++ | 0679 | +++ | | +++ |
| 0630 | +++ | | +++ | 0680 | +++ | | +++ |
| 0631 | +++ | | +++ | 0681 | +++ | | +++ |
| 0632 | +++ | | ++ | 0682 | +++ | | +++ |
| 0633 | +++ | | +++ | 0683 | +++ | | +++ |
| 0634 | +++ | | +++ | 0684 | +++ | | +++ |
| 0635 | +++ | | +++ | 0685 | +++ | | +++ |
| 0636 | +++ | | +++ | 0686 | +++ | | +++ |
| 0637 | +++ | | +++ | 0687 | +++ | | +++ |
| 0638* | ++ | | +++ | 0688 | +++ | | +++ |
| 0639* | ++ | | + | 0689 | +++ | | +++ |
| 0640* | ++ | | +++ | 0690 | +++ | | +++ |
| 0641 | +++ | | +++ | 0691 | +++ | | +++ |
| 0642* | ++ | | +++ | 0692 | +++ | | +++ |
| 0643* | | | + | 0693 | +++ | | +++ |
| 0644 | +++ | | +++ | 0694 | +++ | | +++ |
| 0645 | +++ | | +++ | 0695 | +++ | | +++ |
| 0646 | +++ | | +++ | 0696 | +++ | | +++ |
| 0647* | | | ++ | 0697 | +++ | | +++ |

(continued)

| Example No. | PI3Kα enzyme inhibitory activity | ERK2 enzyme inhibitory activity | | Example No. | PI3Kα enzyme inhibitory activity | ERK2 enzyme inhibitory activity | |
|---|---|---|---|---|---|---|---|
| | | (1) | (2) | | | (1) | (2) |
| 0648* | +++ | | +++ | 0698 | ++ | | +++ |
| 0649 | +++ | | +++ | 0699 | +++ | | +++ |
| 0650 | +++ | | +++ | 0700 | +++ | | +++ |

*not in accordance with the present invention

[Table 1-8]

| Example No. | PI3Kα enzyme inhibitory activity | ERK2 enzyme inhibitory activity | | Example No. | PI3Kα enzyme inhibitory activity | ERK2 enzyme inhibitory activity | |
|---|---|---|---|---|---|---|---|
| | | (1) | (2) | | | (1) | (2) |
| 0701 | +++ | | +++ | 0751 | +++ | | +++ |
| 0702 | +++ | | +++ | 0752 | +++ | | +++ |
| 0703 | +++ | | +++ | 0753 | +++ | | |
| 0704 | +++ | | +++ | 0754 | +++ | | +++ |
| 0705 | +++ | | +++ | 0755 | +++ | | +++ |
| 0706 | +++ | | +++ | 0756 | +++ | | +++ |
| 0707 | +++ | | +++ | 0757 | +++ | | +++ |
| 0708 | +++ | | +++ | 0758 | ++ | | +++ |
| 0709 | +++ | | +++ | 0759 | +++ | | +++ |
| 0710 | +++ | | +++ | 0760 | +++ | | +++ |
| 0711 | +++ | | +++ | 0761 | +++ | | +++ |
| 0712 | +++ | | +++ | 0762 | +++ | | +++ |
| 0713 | +++ | | +++ | 0763 | | | +++ |
| 0714 | +++ | | +++ | 0764 | +++ | | +++ |
| 0715 | +++ | | +++ | 0765 | +++ | | +++ |
| 0716 | +++ | | +++ | 0766 | +++ | | +++ |
| 0717 | +++ | | +++ | 0767 | +++ | | +++ |
| 0718 | +++ | | +++ | 0768 | +++ | | ++ |
| 0719 | +++ | | +++ | 0769 | +++ | | +++ |
| 0720 | +++ | | +++ | 0770 | +++ | | +++ |
| 0721 | +++ | | +++ | 0771 | +++ | | +++ |
| 0722 | +++ | | +++ | 0772 | +++ | | +++ |
| 0723 | +++ | | +++ | 0773 | +++ | | +++ |
| 0724 | +++ | | +++ | 0774 | +++ | | +++ |
| 0725 | +++ | | +++ | 0775 | +++ | | |
| 0726 | +++ | | +++ | 0776 | + | | |
| 0727 | +++ | | +++ | 0777 | +++ | | +++ |
| 0728 | +++ | | +++ | 0778 | +++ | | +++ |
| 0729 | +++ | | +++ | 0779 | +++ | | +++ |
| 0730 | +++ | | +++ | 0780 | +++ | | +++ |
| 0731 | +++ | | +++ | 0781 | +++ | | +++ |
| 0732 | +++ | | +++ | 0782 | +++ | | +++ |
| 0733 | +++ | | +++ | 0783 | +++ | | +++ |
| 0734 | +++ | | +++ | 0784 | +++ | | +++ |
| 0735 | +++ | | +++ | 0785 | +++ | | +++ |
| 0736 | +++ | | ++ | 0786 | +++ | | +++ |
| 0737 | +++ | | +++ | 0787 | +++ | | +++ |

(continued)

| Example No. | PI3Kα enzyme inhibitory activity | ERK2 enzyme inhibitory activity | | Example No. | PI3Kα enzyme inhibitory activity | ERK2 enzyme inhibitory activity | |
|---|---|---|---|---|---|---|---|
| | | (1) | (2) | | | (1) | (2) |
| 0738 | +++ | | +++ | 0788 | +++ | | +++ |
| 0739 | ++ | | +++ | 0789 | +++ | | +++ |
| 0740 | +++ | | +++ | 0790 | +++ | | +++ |
| 0741 | +++ | | +++ | 0791 | +++ | | +++ |
| 0742 | +++ | | +++ | 0792 | +++ | | +++ |
| 0743 | +++ | | +++ | 0793 | +++ | | ++ |
| 0744 | +++ | | +++ | 0794 | +++ | | +++ |
| 0745 | +++ | | +++ | 0795 | +++ | | +++ |
| 0746 | +++ | | +++ | 0796 | +++ | | +++ |
| 0747 | +++ | | +++ | 0797 | +++ | | +++ |
| 0748 | +++ | | +++ | 0798 | +++ | | +++ |
| 0749 | +++ | | +++ | 0799 | +++ | | +++ |
| 0750 | +++ | | +++ | 0800 | +++ | | +++ |

[Table 1-9]

| Example No. | PI3Kα enzyme inhibitory activity | ERK2 enzyme inhibitory activity | | Example No. | PI3Kα enzyme inhibitory activity | ERK2 enzyme inhibitory activity | |
|---|---|---|---|---|---|---|---|
| | | (1) | (2) | | | (1) | (2) |
| 0801 | ++ | | +++ | 0851 | ++ | | +++ |
| 0802 | +++ | | +++ | 0852 | +++ | | +++ |
| 0803 | +++ | | +++ | 0853 | +++ | | +++ |
| 0804 | +++ | | +++ | 0854 | +++ | | +++ |
| 0805 | +++ | | +++ | 0855 | +++ | | +++ |
| 0806 | +++ | | +++ | 0856 | ++ | | +++ |
| 0807 | +++ | | +++ | 0857 | ++ | | +++ |
| 0808 | ++ | | +++ | 0858 | ++ | | +++ |
| 0809 | +++ | | +++ | 0859 | ++ | | +++ |
| 0810 | +++ | | +++ | 0860 | ++ | | ++ |
| 0811 | +++ | | +++ | 0861 | +++ | | +++ |
| 0812 | +++ | | +++ | 0862 | +++ | | +++ |
| 0813 | +++ | | +++ | 0863 | ++ | | +++ |
| 0814 | +++ | | +++ | 0864 | +++ | | +++ |
| 0815 | +++ | | +++ | 0865 | ++ | | ++ |
| 0816 | +++ | | +++ | 0866 | ++ | | |
| 0817 | +++ | | +++ | 0867 | +++ | | +++ |
| 0818 | +++ | | +++ | 0868 | ++ | | +++ |
| 0819 | +++ | | +++ | 0869 | ++ | | +++ |
| 0820 | +++ | | +++ | 0870 | ++ | | ++ |
| 0821 | +++ | | +++ | 0871 | ++ | | ++ |
| 0822 | +++ | | +++ | 0872 | +++ | | +++ |
| 0823 | +++ | | +++ | 0873 | +++ | | +++ |
| 0824 | +++ | | +++ | 0874 | +++ | | +++ |
| 0825 | +++ | | +++ | 0875 | ++ | | +++ |
| 0826 | +++ | | +++ | 0876* | ++ | | ++ |
| 0827 | +++ | | +++ | 0877 | +++ | | ++ |
| 0828 | +++ | | +++ | 0878 | +++ | | ++ |

(continued)

| Example No. | PI3Kα enzyme inhibitory activity | ERK2 enzyme inhibitory activity (1) | ERK2 enzyme inhibitory activity (2) | Example No. | PI3Kα enzyme inhibitory activity | ERK2 enzyme inhibitory activity (1) | ERK2 enzyme inhibitory activity (2) |
|---|---|---|---|---|---|---|---|
| 0829 | +++ | | +++ | 0879 | +++ | | +++ |
| 0830 | +++ | | +++ | 0880 | +++ | | +++ |
| 0831 | +++ | | +++ | 0881 | ++ | | +++ |
| 0832 | +++ | | +++ | 0882 | +++ | | +++ |
| 0833 | +++ | | +++ | 0883 | +++ | | +++ |
| 0834 | +++ | | +++ | 0884 | ++ | | +++ |
| 0835 | +++ | | +++ | 0885 | +++ | | +++ |
| 0836 | +++ | | ++ | 0886 | +++ | | +++ |
| 0837 | ++ | | +++ | 0887 | +++ | | ++ |
| 0838 | +++ | | +++ | 0888 | ++ | | ++ |
| 0839 | + | | ++ | 0889 | + | | + |
| 0840 | ++ | | ++ | 0890 | +++ | | ++ |
| 0841 | ++ | | ++ | 0891 | ++ | | +++ |
| 0842 | ++ | | ++ | 0892 | ++ | | ++ |
| 0843 | +++ | | +++ | 0893 | +++ | | +++ |
| 0844 | +++ | | +++ | 0894 | +++ | | ++ |
| 0845 | ++ | | +++ | 0895 | ++ | | +++ |
| 0846 | ++ | | +++ | 0896 | ++ | | ++ |
| 0847 | ++ | | +++ | 0897 | +++ | | +++ |
| 0848 | ++ | | ++ | 0898 | +++ | | ++ |
| 0849 | ++ | | +++ | 0899 | ++ | | ++ |
| 0850 | +++ | | +++ | 0900 | +++ | | ++ |

*not in accordance with the present invention

[Table 1-10]

| Example No. | PI3Kα enzyme inhibitory activity | ERK2 enzyme inhibitory activity (1) | ERK2 enzyme inhibitory activity (2) | Example No. | PI3Kα enzyme inhibitory activity | ERK2 enzyme inhibitory activity (1) | ERK2 enzyme inhibitory activity (2) |
|---|---|---|---|---|---|---|---|
| 0901 | ++ | | +++ | 0929* | ++ | | +++ |
| 0902 | +++ | | +++ | 0930 | +++ | | +++ |
| 0903 | +++ | | +++ | 0931 | +++ | | +++ |
| 0904 | +++ | | ++ | 0932 | +++ | | +++ |
| 0905 | ++ | | ++ | 0933 | +++ | | +++ |
| 0906 | ++ | | +++ | 0934 | +++ | | +++ |
| 0907 | +++ | | +++ | 0935 | +++ | | ++ |
| 0908 | +++ | | +++ | 0936 | +++ | | +++ |
| 0909 | +++ | | +++ | 0937 | +++ | | +++ |
| 0910 | +++ | | +++ | 0938 | +++ | | +++ |
| 0911 | +++ | | +++ | 0939 | +++ | | +++ |
| 0912 | +++ | | +++ | 0940 | +++ | | +++ |
| 0913 | +++ | | +++ | 0941 | +++ | | +++ |
| 0914 | +++ | | +++ | 0942 | +++ | | +++ |
| 0915 | | | + | 0943 | +++ | | +++ |
| 0916 | +++ | | +++ | 0944 | +++ | | +++ |
| 0917 | +++ | | +++ | 0945 | +++ | | +++ |
| 0918 | +++ | | +++ | 0946 | +++ | | +++ |

(continued)

| Example No. | PI3Kα enzyme inhibitory activity | ERK2 enzyme inhibitory activity (1) | (2) | Example No. | PI3Kα enzyme inhibitory activity | ERK2 enzyme inhibitory activity (1) | (2) |
|---|---|---|---|---|---|---|---|
| 0919 | +++ | | +++ | 0947 | ++ | | ++ |
| 0920 | +++ | | +++ | 0948 | +++ | | +++ |
| 0921 | +++ | | +++ | 0949 | ++ | | ++ |
| 0922 | ++ | | +++ | 0950 | +++ | | ++ |
| 0923 | +++ | | +++ | 0951 | +++ | | +++ |
| 0924 | +++ | | +++ | 0952 | +++ | | +++ |
| 0925 | +++ | | +++ | 0953 | +++ | | +++ |
| 0926 | +++ | | +++ | 0954 | +++ | | +++ |
| 0927 | +++ | | +++ | 0955 | +++ | | +++ |
| 0928 | +++ | | +++ | | | | |

*not in accordance with the present invention

The compound of the invention exhibited excellent inhibitory activity with respect to the PI3K-AKT pathway and/or the Ras-Raf-MEK-ERK pathway.

Test Example 3: CHO Cell Toxicity Test

In a CHO cell toxicity test, the following was used.

Cell: CHO-K1 cells (derived from Chinese hamster ovary)

Test medium: F-12 MEDIUM + FCS (manufactured by Biowest) having a final concentration of 2%

Detection reagent: CELLTITER-GLO™ LUMINESCENT CELL VIABILITY ASSAY (trade name, manufactured by PROMEGA Corporation)

The CHO-K1 cells were seeded in a 96-well microplate for culture at 5,000 cells/well, and cultured in a $CO_2$ incubator (37°C) for 24 hours. An evaluation compound (final concentration: from 25.00 μmol/L to 0.20 μmol/L, common ratio: 2.8 points) was added thereto, followed by culturing for 24 hours, and the number of viable cells was calculated from emission intensity by CELLTITER-GLO™ (trade name, manufactured by PROMEGA Corporation). In the measurement of the emission intensity, VARIOSKAN FLASH (manufactured by Thermo Fisher Scientific Inc.) was used.

An inhibition ratio was calculated from the following equation, and a concentration ($IC_{50}$ value) of compound at which the number of viable cells was suppressed to be equal to or less than 50% was determined.

$$\text{Inhibition ratio (\%)} = 100 - (A/B) \times 100$$

A: Emission intensity in the presence of a test compound
B: Emission intensity in the absence of a test compound

$IC_{50}$ values of the compounds of Example Nos. 0001, 0015, 0018, 0019, 0027, 0037, 0097, 0122, 0128, 0130, 0137, 0139, 0140, 0145, 0148, 0179, 0214, 0222, 0226, 0227, 0231, 0232, 0233, 0237, 0238, 0240, 0241, 0243, 0245, 0247, 0250, 0291, 0293, 0298, 0301, 0302, 0316, 0321, 0322, 0325, 0338, 0341, 0347, 0348, 0352, 0355, 0362, 0363, 0365, 0368, 0373, 0374, 0382, 0384, 0390, 0395, 0400, 0401, 0403, 0405, 0406, 0413, 0417, 0418, 0420, 0422, 0424, 0426, 0430, 0431, 0432, 0439, 0443, 0450, 0455, 0456, 0460, 0462, 0465, 0473, 0474, 0479, 0482, 0486, 0487, 0490, 0491, 0492, 0493, 0500, 0510, 0515, 0517, 0530, 0545, 0549, 0550, 0552, 0557, 0567, 0572, 0573, 0576, 0583, 0613, 0614, 0615, 0626, 0629, 0630, 0631, 0635, 0637, 0644, 0645, 0649, 0650, 0651, 0653, 0657, 0659, 0665, 0678, 0679, 0682, 0685, 0686, 0687, 0688, 0691, 0694, 0695, 0697, 0712, 0723, 0724, 0725, 0733, 0737, 0738, 0756, 0757, 0761, 0773, 0781, 0795, 0812, and 0919 were equal to or greater than 25 μmol/L.

The compound of the invention exhibited excellent safety.

INDUSTRIAL APPLICABILITY

The nitrogen-containing heterocyclic compound or salt thereof of the invention has excellent inhibitory activity with respect to the PI3K-AKT pathway and/or the Ras-Raf-MEK-ERK pathway, and is useful for a treatment such as prevention of or cure for a disease such as a malignant tumor, a cell proliferative disease, an allergic disease, an autoimmune disease, a neurodegenerative disease, a circulatory system disease, an inflammatory disease, an endocrine disorder, a metabolic disorder, or an infection.

## Claims

**1.** A nitrogen-containing heterocyclic compound represented by the following Formula [1] or salt thereof:

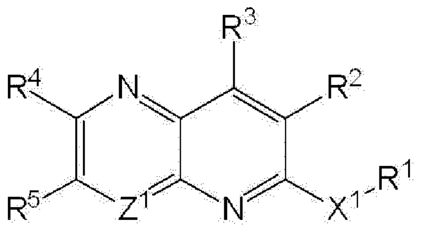

[1]

wherein, in Formula [1]:

$Z^1$ represents $CR^6$;
$R^6$ represents a hydrogen atom, a halogen atom, a hydroxyl group, a $C_{1-6}$ alkyl group, a $C_{2-6}$ alkenyl group, a $C_{2-6}$ alkynyl group, a $C_{3-8}$ cycloalkyl group, an aryl group which may be substituted with one or more substituents selected from a substituent group $\alpha_2$, a $C_{1-6}$ alkoxy group, an aryloxy group, a $C_{1-6}$ alkylthio group, an arylthio group, a heteroaryl group which may be substituted with one or more substituents selected from the substituent group $\alpha_2$, a heteroaryloxy group, a heteroarylthio group, a heterocyclyl group which may be substituted with one or more substituents selected from the substituent group $\alpha_2$, or $NR^7R^8$;
each of $R^7$ and $R^8$ independently represents a hydrogen atom, a $C_{1-6}$ alkyl group, a $C_{2-6}$ alkenyl group, a $C_{2-6}$ alkynyl group, a $C_{3-8}$ cycloalkyl group, an aryl group which may be substituted with one or more substituents selected from the substituent group $\alpha_2$, a heteroaryl group which may be substituted with one or more substituents selected from the substituent group $\alpha_2$, or a heterocyclyl group which may be substituted with one or more substituents selected from the substituent group $\alpha_2$;
the substituent group $\alpha_2$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with one or more substituents selected from a substituent group $\beta_2$, a $C_{1-6}$ alkyl group which may be substituted with one or more substituents selected from the substituent group $\beta_2$, a $C_{1-6}$ alkoxy group which may be substituted with one or more substituents selected from the substituent group $\beta_2$, and an oxo group;
the substituent group $\beta_2$ consists of a halogen atom, a $C_{1-6}$ alkyl group which may be substituted with one or more substituents selected from a substituent group $\gamma_2$, a $C_{3-8}$ cycloalkyl group which may be substituted with one or more substituents selected from the substituent group $\gamma_2$, an aryl group which may be substituted with one or more substituents selected from the substituent group $\gamma_2$, a heteroaryl group which may be substituted with one or more substituents selected from the substituent group $\gamma_2$, and a heterocyclyl group which may be substituted with one or more substituents selected from the substituent group $\gamma_2$; and
the substituent group $\gamma_2$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group

which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a $C_{1-6}$ alkyl group which may be substituted with a halogen atom, a $C_{1-6}$ alkoxy group which may be substituted with a halogen atom, and an oxo group;

$X^1$ represents $NR^9$;

$R^9$ represents a hydrogen atom;

$R^1$ represents a monocyclic nitrogen-containing heteroaryl group which may be substituted with one or more substituents selected from a substituent group $A_1$, a monocyclic nitrogen- and oxygen-containing heteroaryl group which may be substituted with one or more substituents selected from the substituent group $A_1$, a monocyclic nitrogen- and sulfur-containing heteroaryl group which may be substituted with one or more substituents selected from the substituent group $A_1$, a bicyclic nitrogen-containing heteroaryl group which may be substituted with one or more substituents selected from the substituent group $A_1$, a bicyclic nitrogen- and oxygen-containing heteroaryl group which may be substituted with one or more substituents selected from the substituent group $A_1$, or a bicyclic nitrogen- and sulfur-containing heteroaryl group which may be substituted with one or more substituents selected from the substituent group $A_1$;

the substituent group $A_1$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with one or more substituents selected from a substituent group $B_1$, a sulfamoyl group which may be substituted with one or more substituents selected from the substituent group $B_1$, a $C_{1-6}$ alkyl group which may be substituted with one or more substituents selected from the substituent group $B_1$, a $C_{2-6}$ alkenyl group which may be substituted with one or more substituents selected from the substituent group $B_1$, a $C_{3-8}$ cycloalkyl group which may be substituted with one or more substituents selected from the substituent group $B_1$, an aryl group which may be substituted with one or more substituents selected from the substituent group $B_1$, a $C_{1-6}$ alkoxy group which may be substituted with one or more substituents selected from the substituent group $B_1$, a $C_{3-8}$ cycloalkoxy group which may be substituted with one or more substituents selected from the substituent group $B_1$, an aryloxy group which may be substituted with one or more substituents selected from the substituent group $B_1$, a $C_{1-6}$ alkylthio group which may be substituted with one or more substituents selected from the substituent group $B_1$, an arylthio group which may be substituted with one or more substituents selected from the substituent group $B_1$, a $C_{1-6}$ alkylamino group which may be substituted with one or more substituents selected from the substituent group $B_1$, a di($C_{1-6}$ alkyl)amino group which may be substituted with one or more substituents selected from the substituent group $B_1$, an acyl group which may be substituted with one or more substituents selected from the substituent group $B_1$, a heteroaryl group which may be substituted with one or more substituents selected from the substituent group $B_1$, a heterocyclyl group which may be substituted with one or more substituents selected from the substituent group $B_1$, and an oxo group;

the substituent group $B_1$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with one or more substituents selected from a substituent group $C_1$, a sulfamoyl group which may be substituted with one or more substituents selected from the substituent group $C_1$, a $C_{1-6}$ alkyl group which may be substituted with one or more substituents selected from the substituent group $C_1$, a $C_{3-8}$ cycloalkyl group which may be substituted with one or more substituents selected from the substituent group $C_1$, an aryl group which may be substituted with one or more substituents selected from the substituent group $C_1$, a $C_{1-6}$ alkoxy group which may be substituted with one or more substituents selected from the substituent group $C_1$, a $C_{1-6}$ alkylamino

group which may be substituted with one or more substituents selected from the substituent group $C_1$, a di($C_{1-6}$ alkyl)amino group which may be substituted with one or more substituents selected from the substituent group $C_1$, a $C_{1-6}$ alkylsulfonyl group which may be substituted with one or more substituents selected from the substituent group $C_1$, an arylsulfonyl group which may be substituted with one or more substituents selected from the substituent group $C_1$, an acyl group which may be substituted with one or more substituents selected from the substituent group $C_1$, a hetero aryl group which may be substituted with one or more substituents selected from the substituent group $C_1$, a heterocyclyl group which may be substituted with one or more substituents selected from the substituent group $C_1$, and an oxo group; and

the substituent group $C_1$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with a $C_{1-6}$ alkyl group, a $C_{1-6}$ alkyl group which may be substituted with a halogen atom, an aryl group which may be substituted with a halogen atom or a $C_{1-6}$ alkyl group, a $C_{1-6}$ alkoxy group which may be substituted with a halogen atom, and an oxo group;

$R^2$ represents a hydrogen atom;

$R^3$ represents a hydrogen atom;

$R^4$ represents a hydrogen atom;

$R^5$ represents a heteroaryl group which may be substituted with one or more substituents selected from the substituent group $A_3$, a heteroaryloxy group which may be substituted with one or more substituents selected from the substituent group $A_3$, a heteroarylthio group which may be substituted with one or more substituents selected from the substituent group $A_3$, a heterocyclyl group which may be substituted with one or more substituents selected from the substituent group $A_3$, or $NR^{12}R^{13}$;

each of $R^{12}$ and $R^{13}$ independently represents a hydrogen atom, a $C_{1-6}$ alkyl group which may be substituted with one or more substituents selected from the substituent group $A_3$, a $C_{2-6}$ alkenyl group which may be substituted with one or more substituents selected from the substituent group $A_3$, a $C_{2-6}$ alkynyl group which may be substituted with one or more substituents selected from the substituent group $A_3$, a $C_{3-8}$ cycloalkyl group which may be substituted with one or more substituents selected from the substituent group $A_3$, an aryl group which may be substituted with one or more substituents selected from the substituent group $A_3$, a heteroaryl group which may be substituted with one or more substituents selected from the substituent group $A_3$, a heterocyclyl group which may be substituted with one or more substituents selected from the substituent group $A_3$, or an amino-protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group;

the substituent group $A_3$ consists of a halogen atom, a hydroxyl group , an amino group , an amino group which may be substituted with one or more substituents selected from a substituent group $E_3$, a carboxyl group , a cyano group, a carbamoyl group which may be substituted with one or more substituents selected from a substituent group $B_3$, a sulfamoyl group which may be substituted with one or more substituents selected from the substituent group $B_3$, a $C_{1-6}$ alkyl group which may be substituted with one or more substituents selected from the substituent group $B_3$, a $C_{2-6}$ alkenyl group which may be substituted with one or more substituents selected from the substituent group $B_3$, a $C_{3-8}$ cycloalkyl group which may be substituted with one or more substituents selected from the substituent group $B_3$, an aryl group which may be substituted with one or more substituents selected from the substituent group $B_3$, a $C_{1-6}$ alkoxy group which may be substituted with one or more substituents selected from the substituent group $B_3$, an aryloxy group which may be substituted with one or more substituents selected from the substituent group $B_3$, a $C_{1-6}$ alkylthio group which may be substituted with one or more substituents selected from the substituent group $B_3$, an arylthio group which may be substituted with one or more substituents selected from the substituent group $B_3$, a $C_{1-6}$ alkyl amino group which may be substituted with one or more substituents selected from the substituent group $B_3$, a di($C_{1-6}$ alkyl)amino group which may be substituted with one or more substituents selected from the substituent group $B_3$, a $C_{1-6}$ alkylsulfonyl group which may be substituted with one or more substituents selected from the substituent group $B_3$, an arylsulfonyl group which may be substituted with one or more substituents selected from the substituent group $B_3$, an acyl group which may be substituted with one or more substituents selected from the substituent group $B_3$, a heteroaryl group which may be substituted with one or more substituents selected from the substituent

group $B_3$, a heterocyclyl group which may be substituted with one or more substituents selected from the substituent group $B_3$, a heterocyclyloxy group which may be substituted with one or more substituents selected from the substituent group $B_3$, and an oxo group;
the substituent group $B_3$ consists of a halogen atom, a hydroxyl group which may be protected by a protecting group selected from a $C_{1-6}$ alkyl group, a $C_{1-6}$ alkenyl group,
an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with one or more substituents selected from a substituent group $C_3$, a $C_{1-6}$ alkyl group which may be substituted with one or more substituents selected from the substituent group $C_3$, a $C_{3-8}$ cycloalkyl group which may be substituted with one or more substituents selected from the substituent group $C_3$, an aryl group which may be substituted with one or more substituents selected from the substituent group $C_3$, a $C_{1-6}$ alkoxy group which may be substituted with one or more substituents selected from the substituent group $C_3$, an aryloxy group which may be substituted with one or more substituents selected from the substituent group $C_3$, a $C_{1-6}$ alkylamino group which may be substituted with one or more substituents selected from the substituent group $C_3$, a di($C_{1-6}$ alkyl)amino group which may be substituted with one or more substituents selected from the substituent group $C_3$, a $C_{1-6}$ alkylsulfonyl group which may be substituted with one or more substituents selected from the substituent group $C_3$, an arylsulfonyl group which may be substituted with one or more substituents selected from the substituent group $C_3$, an acyl group which may be substituted with one or more substituents selected from the substituent group $C_3$, a heteroaryl group which may be substituted with one or more substituents selected from the substituent group $C_3$, a heterocyclyl group which may be substituted with one or more substituents selected from the substituent group $C_3$, a heteroaryloxy group which may be substituted with one or more substituents selected from the substituent group $C_3$, a heterocyclyloxy group which may be substituted with one or more substituents selected from the substituent group $C_3$, and an oxo group;
the substituent group $C_3$ consists of a halogen atom, a hydroxyl group , an amino group , a carboxyl group , a nitro group, cyano group, a carbamoyl group which may be substituted with one or more substituents selected from a substituent group $D_3$, a $C_{1-6}$ alkyl group which may be substituted with one or more substituents selected from the substituent group $D_3$, a $C_{3-8}$ cycloalkyl group which may be substituted with one or more substituents selected from the substituent group $D_3$, an aryl group which may be substituted with one or more substituents selected from the substituent group $D_3$, a $C_{1-6}$ alkoxy group which may be substituted with one or more substituents selected from the substituent group $D_3$, an aryloxy group which may be substituted with one or more substituents selected from the substituent group $D_3$, a $C_{1-6}$ alkylamino group which may be substituted with one or more substituents selected from the substituent group $D_3$, a di($C_{1-6}$ alkyl)amino group which may be substituted with one or more substituents selected from the substituent group $D_3$, a $C_{1-6}$ alkylsulfonyl group which may be substituted with one or more substituents selected from the substituent group $D_3$, an acyl group which may be substituted with one or more substituents selected from the substituent group $D_3$, a heteroaryl group which may be substituted with one or more substituents selected from the substituent group $D_3$, a heterocyclyl group which may be substituted with one or more substituents selected from the substituent group $D_3$, a silyl group which may be substituted with one or more substituents selected from the substituent group $D_3$, and an oxo group;
the substituent group $D_3$ consists of a halogen atom, a hydroxyl group , a $C_{1-6}$ alkyl group, a $C_{3-8}$ cycloalkyl group, an aryl group, a $C_{1-6}$ alkoxy group, a $C_{1-6}$ alkylamino group, a di($C_{1-6}$ alkyl)amino group, a $C_{1-6}$ alkylsulfonyl group, a heteroaryl group, a heterocyclyl group, and an oxo group;
the substituent group $E_3$ consists of a $C_{3-8}$ cycloalkyl group which may be substituted with one or more substituents selected from a substituent group $F_3$, an aryl group which may be substituted with one or more substituents selected from the substituent group $F_3$, a heteroaryl group which may be substituted with one or more substituents selected from the substituent group $F_3$, and a heterocyclyl group which may be substituted with one or more substituents selected from the substituent group $F_3$; and
the substituent group $F_3$ consists of a halogen atom, a carboxyl group, and a $C_{1-6}$ alkyl group.

**2.** The nitrogen-containing heterocyclic compound or salt thereof according to claim 1, wherein:
$R^1$ represents a pyrazolyl group which may be substituted with one or more substituents selected from the substituent group $A_1$, an imidazolyl group which may be substituted with one or more substituents selected from the substituent

group $A_1$, a triazolyl group which may be substituted with one or more substituents selected from the substituent group $A_1$, a thiazolyl group which may be substituted with one or more substituents selected from the substituent group $A_1$, an oxadiazolyl group which may be substituted with one or more substituents selected from the substituent group $A_1$, a thiadiazolyl group which may be substituted with one or more substituents selected from the substituent group $A_1$, a pyridyl group which may be substituted with one or more substituents selected from the substituent group $A_1$, or a pyridazinyl group which may be substituted with one or more substituents selected from the substituent group $A_1$.

**3.** The nitrogen-containing heterocyclic compound or salt thereof according to claim 1 or 2, wherein:

$R^1$ represents a pyrazolyl group which may be substituted with one or more substituents selected from a substituent group $\alpha_1$, an imidazolyl group which may be substituted with one or more substituents selected from the substituent group $\alpha_1$, a triazolyl group which may be substituted with one or more substituents selected from the substituent group $\alpha_1$, a thiazolyl group which may be substituted with one or more substituents selected from the substituent group $\alpha_1$, an oxadiazolyl group which may be substituted with one or more substituents selected from the substituent group $\alpha_1$, a thiadiazolyl group which may be substituted with one or more substituents selected from the substituent group $\alpha_1$, a pyridyl group which may be substituted with one or more substituents selected from the substituent group $\alpha_1$, or a pyridazinyl group which may be substituted with one or more substituents selected from the substituent group $\alpha_1$;

the substituent group $\alpha_1$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with one or more substituents selected from a substituent group $\beta_1$, a sulfamoyl group which may be substituted with one or more substituents selected from the substituent group $\beta_1$, a $C_{1-6}$ alkyl group which may be substituted with one or more substituents selected from the substituent group $\beta_1$, a $C_{3-8}$ cycloalkyl group which may be substituted with one or more substituents selected from the substituent group $\beta_1$, an aryl group which may be substituted with one or more substituents selected from the substituent group $\beta_1$, a $C_{1-6}$ alkoxy group which may be substituted with one or more substituents selected from the substituent group $\beta_1$, an aryloxy group which may be substituted with one or more substituents selected from the substituent group $\beta_1$, a $C_{1-6}$ alkylthio group which may be substituted with one or more substituents selected from the substituent group $\beta_1$, an arylthio group which may be substituted with one or more substituents selected from the substituent group $\beta_1$, a $C_{1-6}$ alkylamino group which may be substituted with one or more substituents selected from the substituent group $\beta_1$, a di($C_{1-6}$ alkyl)amino group which may be substituted with one or more substituents selected from the substituent group $\beta_1$, an acyl group which may be substituted with one or more substituents selected from the substituent group $\beta_1$, a heteroaryl group which may be substituted with one or more substituents selected from the substituent group $\beta_1$, a heterocyclyl group which may be substituted with one or more substituents selected from the substituent group $\beta_1$, and an oxo group;

the substituent group $\beta_1$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with one or more substituents selected from a substituent group $\gamma_1$, a $C_{1-6}$ alkyl group which may be substituted with one or more substituents selected from the substituent group $\gamma_1$, a $C_{3-8}$ cycloalkyl group which may be substituted with one or more substituents selected from the substituent group $\gamma_1$, an aryl group which may be substituted with one or more substituents selected from the substituent group $\gamma_1$, a $C_{1-6}$ alkoxy group which may be substituted with one or more substituents selected from the substituent group $\gamma_1$, a $C_{1-6}$ alkylamino group which may be substituted with one or more substituents selected from the substituent group $\gamma_1$, a di($C_{1-6}$

alkyl)amino group which may be substituted with one or more substituents selected from the substituent group $\gamma_1$, a $C_{1-6}$ alkylsulfonyl group which may be substituted with one or more substituents selected from the substituent group $\gamma_1$, an arylsulfonyl group which may be substituted with one or more substituents selected from the substituent group $\gamma_1$, an acyl group which may be substituted with one or more substituents selected from the substituent group $\gamma_1$, a heteroaryl group which may be substituted with one or more substituents selected from the substituent group $\gamma_1$, a heterocyclyl group which may be substituted with one or more substituents selected from the substituent group $\gamma_1$, and an oxo group; and

the substituent group $\gamma_1$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with a $C_{1-6}$ alkyl group, a $C_{1-6}$ alkyl group which may be substituted with a halogen atom, a $C_{1-6}$ alkoxy group which may be substituted with a halogen atom, and an oxo group.

**4.** The nitrogen-containing heterocyclic compound or salt thereof according to any one of claims 1 to 3, wherein;
$Z^1$ represents CH;
$R^5$ represents a heteroaryl group which may be substituted with one or more substituents selected from the substituent group $A_3$, a heterocyclyl group which may be substituted with one or more substituents selected from the substituent group $A_3$, or $NR^{12b}R^{13b}$;
$R^{12b}$ represents a hydrogen atom or a $C_{1-6}$ alkyl group;
$R^{13b}$ represents a $C_{3-8}$ cycloalkyl group which may be substituted with one or more substituents selected from the substituent group $A_3$, an aryl group which may be substituted with one or more substituents selected from the substituent group $A_3$, a heteroaryl group which may be substituted with one or more substituents selected from the substituent group $A_3$, or a heterocyclyl group which may be substituted with one or more substituents selected from the substituent group $A_3$.

**5.** The nitrogen-containing heterocyclic compound or salt thereof according to any one of claims 1 to 3, wherein:

$Z^1$ represents CH;
$R^5$ represents a heteroaryl group which may be substituted with one or more substituents selected from the substituent group $\alpha_3$, a heterocyclyl group which may be substituted with one or more substituents selected from the substituent group $\alpha_3$, or $NR^{12b}R^{13c}$;
$R^{12b}$ represents a hydrogen atom or a $C_{1-6}$ alkyl group;
$R^{13c}$ represents a $C_{3-8}$ cycloalkyl group which may be substituted with one or more substituents selected from the substituent group $\alpha_3$, an aryl group which may be substituted with one or more substituents selected from the substituent group $\alpha_3$, a heteroaryl group which may be substituted with one or more substituents selected from the substituent group $\alpha_3$, or a heterocyclyl group which may be substituted with one or more substituents selected from the substituent group $\alpha_3$;
the substituent group $\alpha_3$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with one or more substituents selected from a substituent group $\beta_3$, a $C_{1-6}$ alkyl group which may be substituted with one or more substituents selected from the substituent group $\beta_3$, a $C_{3-8}$ cycloalkyl group which may be substituted with one or more substituents selected from the substituent group $\beta_3$, an aryl group which may be substituted with one or more substituents selected from the substituent group $\beta_3$, a $C_{1-6}$ alkoxy group which may be substituted with one or more substituents selected from the substituent group $\beta_3$, an aryloxy group which may be substituted with one or more substituents selected from the substituent group $\beta_3$, a $C_{1-6}$ alkylthio group

which may be substituted with one or more substituents selected from the substituent group $\beta_3$, an arylthio group which may be substituted with one or more substituents selected from the substituent group $\beta_3$, a $C_{1-6}$ alkylamino group which may be substituted with one or more substituents selected from the substituent group $\beta_3$, a di($C_{1-6}$ alkyl)amino group which may be substituted with one or more substituents selected from the substituent group $\beta_3$, a $C_{1-6}$ alkylsulfonyl group which may be substituted with one or more substituents selected from the substituent group $\beta_3$, an arylsulfonyl group which may be substituted with one or more substituents selected from the substituent group $\beta_3$, an acyl group which may be substituted with one or more substituents selected from the substituent group $\beta_3$, a heteroaryl group which may be substituted with one or more substituents selected from the substituent group $\beta_3$, a heterocyclyl group which may be substituted with one or more substituents selected from the substituent group $\beta_3$, a heterocyclyloxy group which may be substituted with one or more substituents selected from the substituent group $\beta_3$, and an oxo group;

the substituent group $\beta_3$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with one or more substituents selected from a substituent group $\gamma_3$, a $C_{1-6}$ alkyl group which may be substituted with one or more substituents selected from the substituent group $\gamma_3$, a $C_{3-8}$ cycloalkyl group which may be substituted with one or more substituents selected from the substituent group $\gamma_3$, an aryl group which may be substituted with one or more substituents selected from the substituent group $\gamma_3$, a $C_{1-6}$ alkoxy group which may be substituted with one or more substituents selected from the substituent group $\gamma_3$, a $C_{1-6}$ alkylamino group which may be substituted with one or more substituents selected from the substituent group $\gamma_3$, a di($C_{1-6}$ alkyl)amino group which may be substituted with one or more substituents selected from the substituent group $\gamma_3$, a $C_{1-6}$ alkylsulfonyl group which may be substituted with one or more substituents selected from the substituent group $\gamma_3$, an arylsulfonyl group which may be substituted with one or more substituents selected from the substituent group $\gamma_3$, a heteroaryl group which may be substituted with one or more substituents selected from the substituent group $\gamma_3$, a heterocyclyl group which may be substituted with one or more substituents selected from the substituent group $\gamma_3$, a heterocyclyloxy group which may be substituted with one or more substituents selected from the substituent group $\gamma_3$, and an oxo group; and

the substituent group $\gamma_3$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a $C_{1-6}$ alkyl group which may be substituted with a halogen atom, a $C_{1-6}$ alkoxy group which may be substituted with a halogen atom, and an oxo group.

**6.** The nitrogen-containing heterocyclic compound or salt thereof according to any one of claims 1 to 3, wherein:

$Z^1$ represents CH;

$R^5$ represents a pyrazolyl group which may be substituted with one or more substituents selected from the substituent group $\alpha_3$, an isoxazolyl group which may be substituted with one or more substituents selected from the substituent group $\alpha_3$, a pyridyl group which may be substituted with one or more substituents selected from the substituent group $\alpha_3$, a pyrimidinyl group which may be substituted with one or more substituents selected from the substituent group $\alpha_3$, a pyrazinyl group which may be substituted with one or more substituents selected from the substituent group $\alpha_3$, a pyridazinyl group which may be substituted with one or more substituents selected from the substituent group $\alpha_3$, an azetidinyl which may be substituted with one or more substituents selected from the substituent group $\alpha_3$, a pyrrolidinyl group which may be substituted with one or more substituents selected from the substituent group $\alpha_3$, a piperidinyl group which may be substituted with one or more substituents selected from the substituent group $\alpha_3$, a piperazinyl group which may be substituted with one or more substituents selected from the substituent group $\alpha_3$, a morpholinyl group which may be substituted with

one or more substituents selected from the substituent group $\alpha_3$, a homopiperazinyl group which may be substituted with one or more substituents selected from the substituent group $\alpha_3$, or $NR^{12b}R^{13d}$;
$R^{12b}$ represents a hydrogen atom or a $C_{1-6}$ alkyl group;
$R^{13d}$ represents a phenyl group which may be substituted with one or more substituents selected from the substituent group $\alpha_3$, a pyrazolyl group which may be substituted with one or more substituents selected from the substituent group $\alpha_3$, an isoxazolyl group which may be substituted with one or more substituents selected from the substituent group $\alpha_3$, a pyridyl group which may be substituted with one or more substituents selected from the substituent group $\alpha_3$, a pyrimidinyl group which may be substituted with one or more substituents selected from the substituent group $\alpha_3$, a pyrazinyl group which may be substituted with one or more substituents selected from the substituent group $\alpha_3$, a pyridazinyl group which may be substituted with one or more substituents selected from the substituent group $\alpha_3$, a pyrrolidinyl group which may be substituted with one or more substituents selected from the substituent group $\alpha_3$, a piperidinyl group which may be substituted with one or more substituents selected from the substituent group $\alpha_3$, or a tetrahydropyridyl group which may be substituted with one or more substituents selected from the substituent group $\alpha_3$;
the substituent group $\alpha_3$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with one or more substituents selected from a substituent group $\beta_3$, a $C_{1-6}$ alkyl group which may be substituted with one or more substituents selected from the substituent group $\beta_3$, a $C_{3-8}$ cycloalkyl group which may be substituted with one or more substituents selected from the substituent group $\beta_3$, an aryl group which may be substituted with one or more substituents selected from the substituent group $\beta_3$, a $C_{1-6}$ alkoxy group which may be substituted with one or more substituents selected from the substituent group $\beta_3$, an aryloxy group which may be substituted with one or more substituents selected from the substituent group $\beta_3$, a $C_{1-6}$ alkylthio group which may be substituted with one or more substituents selected from the substituent group $\beta_3$, an arylthio group which may be substituted with one or more substituents selected from the substituent group $\beta_3$, a $C_{1-6}$ alkylamino group which may be substituted with one or more substituents selected from the substituent group $\beta_3$, a di($C_{1-6}$ alkyl)amino group which may be substituted with one or more substituents selected from the substituent group $\beta_3$, a $C_{1-6}$ alkylsulfonyl group which may be substituted with one or more substituents selected from the substituent group $\beta_3$, an arylsulfonyl group which may be substituted with one or more substituents selected from the substituent group $\beta_3$, an acyl group which may be substituted with one or more substituents selected from the substituent group $\beta_3$, a heteroaryl group which may be substituted with one or more substituents selected from the substituent group $\beta_3$, a heterocyclyl group which may be substituted with one or more substituents selected from the substituent group $\beta_3$, a heterocyclyloxy group which may be substituted with one or more substituents selected from the substituent group $\beta_3$, and an oxo group;
the substituent group $\beta_3$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with one or more substituents selected from a substituent group $\gamma_3$, a $C_{1-6}$ alkyl group which may be substituted with one or more substituents selected from the substituent group $\gamma_3$, a $C_{3-8}$ cycloalkyl group which may be substituted with one or more substituents selected from the substituent group $\gamma_3$, an aryl group which may be substituted with one or more substituents selected from the substituent group $\gamma_3$, a $C_{1-6}$ alkoxy group which may be substituted with one or more substituents selected from the substituent group $\gamma_3$, a $C_{1-6}$ alkylamino group which may be substituted with one or more substituents selected from the substituent group $\gamma_3$, a di($C_{1-6}$ alkyl)amino group which may be substituted with one or more substituents selected from the substituent group $\gamma_3$, a $C_{1-6}$ alkylsulfonyl group which may be substituted with one or more substituents selected from the substituent group $\gamma_3$, an arylsulfonyl group which may be substituted with one or more substituents selected from the

substituent group $\gamma_3$, a heteroaryl group which may be substituted with one or more substituents selected from the substituent group $\gamma_3$, a heterocyclyl group which may be substituted with one or more substituents selected from the substituent group $\gamma_3$, a heterocyclyloxy group which may be substituted with one or more substituents selected from the substituent group $\gamma_3$, and an oxo group; and

the substituent group $\gamma_3$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a $C_{1-6}$ alkyl group which may be substituted with a halogen atom, a $C_{1-6}$ alkoxy group which may be substituted with a halogen atom, and an oxo group.

**7.** A nitrogen-containing heterocyclic compound represented by the following Formula [1a] or a salt thereof:

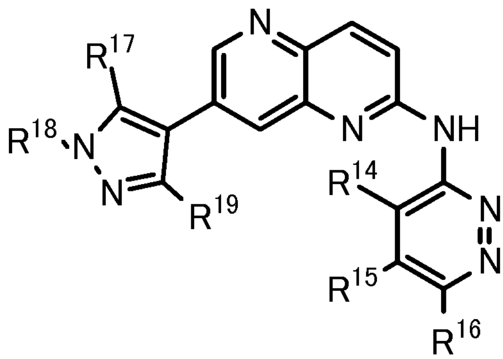

[1a]

wherein, in Formula [1a]:

each of $R^{14}$, $R^{15}$, and $R^{16}$ independently represents a hydrogen atom, a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with one or more substituents selected from a substituent group $B_1$, a sulfamoyl group which may be substituted with one or more substituents selected from the substituent group $B_1$, a $C_{1-6}$ alkyl group which may be substituted with one or more substituents selected from the substituent group $B_1$, a $C_{2-6}$ alkenyl group which may be substituted with one or more substituents selected from the substituent group $B_1$, a $C_{3-8}$ cycloalkyl group which may be substituted with one or more substituents selected from the substituent group $B_1$, an aryl group which may be substituted with one or more substituents selected from the substituent group $B_1$, a $C_{1-6}$ alkoxy group which may be substituted with one or more substituents selected from the substituent group $B_1$, a $C_{3-8}$ cycloalkoxy group which may be substituted with one or more substituents selected from the substituent group $B_1$, an aryloxy group which may be substituted with one or more substituents selected from the substituent group $B_1$, a $C_{1-6}$ alkylthio group which may be substituted with one or more substituents selected from the substituent group $B_1$, an arylthio group which may be substituted with one or more substituents selected from the substituent group $B_1$, a $C_{1-6}$ alkylamino group which may be substituted with one or more substituents selected from the substituent group $B_1$, a di($C_{1-6}$ alkyl)amino group which may be substituted with one or more substituents selected from the substituent group $B_1$, an acyl group which may be substituted with one or more substituents selected from the substituent group $B_1$, a heteroaryl group which may be substituted with one or more substituents selected from the substituent group $B_1$, or a heterocyclyl group which may be substituted with one or more substituents selected from the substituent group Bi;

each of $R^{17}$, $R^{18}$, and $R^{19}$ independently represents a hydrogen atom, a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, an amino group which may be substituted with one or more substituents selected from a substituent group $E_3$, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with one or more substituents selected from a substituent group $B_3$, a sulfamoyl group which may be substituted with one or more substituents selected from the substituent group $B_3$, a $C_{1-6}$ alkyl group which may be substituted with one or more substituents selected from the substituent group $B_3$, a $C_{2-6}$ alkenyl group which may be substituted with one or more substituents selected from the substituent group $B_3$, a $C_{3-8}$ cycloalkyl group which may be substituted with one or more substituents selected from the substituent group $B_3$, an aryl group which may be substituted with one or more substituents selected from the substituent group $B_3$, a $C_{1-6}$ alkoxy group which may be substituted with one or more substituents selected from the substituent group $B_3$, an aryloxy group which may be substituted with one or more substituents selected from the substituent group $B_3$, a $C_{1-6}$ alkylthio group which may be substituted with one or more substituents selected from the substituent group $B_3$, an arylthio group which may be substituted with one or more substituents selected from the substituent group $B_3$, a $C_{1-6}$ alkyl amino group which may be substituted with one or more substituents selected from the substituent group $B_3$, a di($C_{1-6}$ alkyl)amino group which may be substituted with one or more substituents selected from the substituent group $B_3$, a $C_{1-6}$ alkylsulfonyl group which may be substituted with one or more substituents selected from the substituent group $B_3$, an arylsulfonyl group which may be substituted with one or more substituents selected from the substituent group $B_3$, an acyl group which may be substituted with one or more substituents selected from the substituent group $B_3$, a heteroaryl group which may be substituted with one or more substituents selected from the substituent group $B_3$, a heterocyclyl group which may be substituted with one or more substituents selected from the substituent group $B_3$, or a heterocyclyloxy group which may be substituted with one or more substituents selected from the substituent group $B_3$;

the substituent group $B_1$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with one or more substituents selected from a substituent group $C_1$, a sulfamoyl group which may be substituted with one or more substituents selected from the substituent group $C_1$, a $C_{1-6}$ alkyl group which may be substituted with one or more substituents selected from the substituent group $C_1$, a $C_{3-8}$ cycloalkyl group which may be substituted with one or more substituents selected from the substituent group $C_1$, an aryl group which may be substituted with one or more substituents selected from the substituent group $C_1$, a $C_{1-6}$ alkoxy group which may be substituted with one or more substituents selected from the substituent group $C_1$, a $C_{1-6}$ alkylamino group which may be substituted with one or more substituents selected from the substituent group $C_1$, a di($C_{1-6}$ alkyl)amino group which may be substituted with one or more substituents selected from the substituent group $C_1$, a $C_{1-6}$ alkylsulfonyl group which may be substituted with one or more substituents selected from the substituent group $C_1$, an arylsulfonyl group which may be substituted with one or more substituents selected from the substituent group $C_1$, an acyl group which may be substituted with one or more substituents selected from the substituent group $C_1$, a heteroaryl group which may be substituted with one or more substituents selected from the substituent group $C_1$, a heterocyclyl group which may be substituted with one or more substituents selected from the substituent group $C_1$, and an oxo group;

the substituent group $C_1$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group,

a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a cyano group, a carbamoyl group which may be substituted with a $C_{1-6}$ alkyl group, a $C_{1-6}$ alkyl group which may be substituted with a halogen atom, an aryl group which may be substituted with a halogen atom or a $C_{1-6}$ alkyl group, a $C_{1-6}$ alkoxy group which may be substituted with a halogen atom, and an oxo group;

the substituent group $B_3$ consists of a halogen atom, a hydroxyl group , an amino group , a carboxyl group , a cyano group, a carbamoyl group which may be substituted with one or more substituents selected from a substituent group $C_3$, a $C_{1-6}$ alkyl group which may be substituted with one or more substituents selected from the substituent group $C_3$, a $C_{3-8}$ cycloalkyl group which may be substituted with one or more substituents selected from the substituent group $C_3$, an aryl group which may be substituted with one or more substituents selected from the substituent group $C_3$, a $C_{1-6}$ alkoxy group which may be substituted with one or more substituents selected from the substituent group $C_3$, an aryloxy group which may be substituted with one or more substituents selected from the substituent group $C_3$, a $C_{1-6}$ alkylamino group which may be substituted with one or more substituents selected from the substituent group $C_3$, a di($C_{1-6}$ alkyl)amino group which may be substituted with one or more substituents selected from the substituent group $C_3$, a $C_{1-6}$ alkylsulfonyl group which may be substituted with one or more substituents selected from the substituent group $C_3$, an arylsulfonyl group which may be substituted with one or more substituents selected from the substituent group $C_3$, an acyl group which may be substituted with one or more substituents selected from the substituent group $C_3$, a heteroaryl group which may be substituted with one or more substituents selected from the substituent group $C_3$, a heterocyclyl group which may be substituted with one or more substituents selected from the substituent group $C_3$, a heteroaryloxy group which may be substituted with one or more substituents selected from the substituent group $C_3$, a heterocyclyloxy group which may be substituted with one or more substituents selected from the substituent group $C_3$, and an oxo group;

the substituent group $C_3$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, an amino group which may be protected by a protecting group selected from an aryl $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, an aryloxycarbonyl group, a $C_{1-6}$ alkylsulfonyl group, an arylsulfonyl group, and a silyl group, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, a nitro group, cyano group, a carbamoyl group which may be substituted with one or more substituents selected from a substituent group $D_3$, a $C_{1-6}$ alkyl group which may be substituted with one or more substituents selected from the substituent group $D_3$, a $C_{3-8}$ cycloalkyl group which may be substituted with one or more substituents selected from the substituent group $D_3$, an aryl group which may be substituted with one or more substituents selected from the substituent group $D_3$, a $C_{1-6}$ alkoxy group which may be substituted with one or more substituents selected from the substituent group $D_3$, an aryloxy group which may be substituted with one or more substituents selected from the substituent group $D_3$, a $C_{1-6}$ alkylamino group which may be substituted with one or more substituents selected from the substituent group $D_3$, a di($C_{1-6}$ alkyl)amino group which may be substituted with one or more substituents selected from the substituent group $D_3$, a $C_{1-6}$ alkylsulfonyl group which may be substituted with one or more substituents selected from the substituent group $D_3$, an acyl group which may be substituted with one or more substituents selected from the substituent group $D_3$, a heteroaryl group which may be substituted with one or more substituents selected from the substituent group $D_3$, a heterocyclyl group which may be substituted with one or more substituents selected from the substituent group $D_3$, a silyl group which may be substituted with one or more substituents selected from the substituent group $D_3$, and an oxo group;

the substituent group $D_3$ consists of a halogen atom, a hydroxyl group which may be protected by a protecting group selected from a $C_{1-6}$ alkyl group, a $C_{2-6}$ alkenyl group, an ar $C_{1-6}$ alkyl group, a $C_{1-6}$ alkoxy $C_{1-6}$ alkyl group, an acyl group, a $C_{1-6}$ alkoxycarbonyl group, an ar $C_{1-6}$ alkoxycarbonyl group, a $C_{1-6}$ alkylsulfonyl group, an arylsulfonyl group, a silyl group, a tetrahydrofuranyl group, and a tetrahydropyranyl group, a $C_{1-6}$ alkyl group, a $C_{3-8}$ cycloalkyl group, an aryl group, a $C_{1-6}$ alkoxy group, a $C_{1-6}$ alkylamino group, a di($C_{1-6}$ alkyl)amino group, a $C_{1-6}$ alkylsulfonyl group, a heteroaryl group, a heterocyclyl group, and an oxo group;

the substituent group $E_3$ consists of a $C_{3-8}$ cycloalkyl group which may be substituted with one or more substituents selected from a substituent group $F_3$, an aryl group which may be substituted with one or more substituents selected from the substituent group $F_3$, a heteroaryl group which may be substituted with one or more substituents selected from the substituent group $F_3$, and a heterocyclyl group which may be substituted with one or more substituents selected from the substituent group $F_3$; and

the substituent group $F_3$ consists of a halogen atom, a carboxyl group which may be protected by a protecting group selected from a $C_{1-6}$ alkyl group, a $C_{2-6}$ alkenyl group, an aryl group, an aryl $C_{1-6}$ alkyl group, a $C_{1-6}$ alkoxy $C_{1-6}$ alkyl group, and a silyl group, and a $C_{1-6}$ alkyl group.

**8.** The nitrogen-containing heterocyclic compound or salt thereof according to claim 7, wherein each of $R^{14}$, $R^{16}$ and $R^{17}$ represents a hydrogen atom.

**9.** The nitrogen-containing heterocyclic compound or salt thereof according to claim 1, wherein the compound is any one of the compounds set forth below:

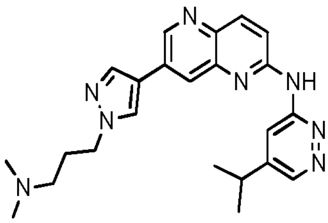
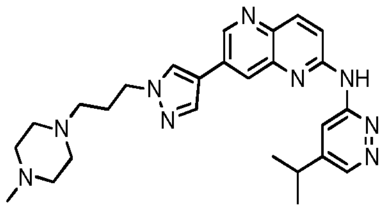
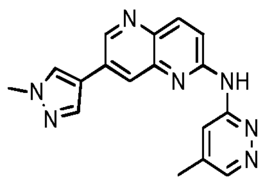
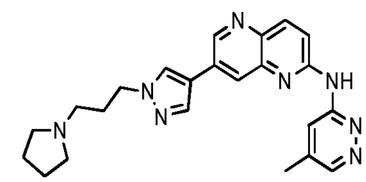
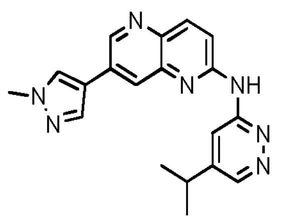
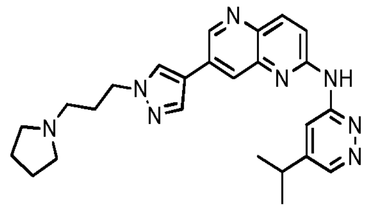
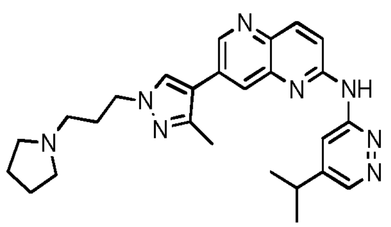
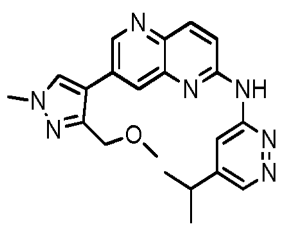
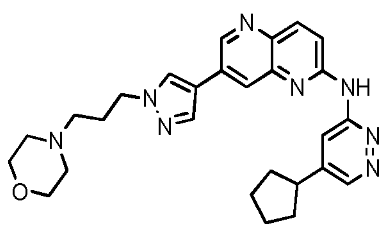
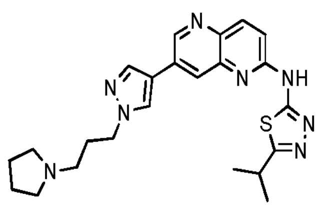
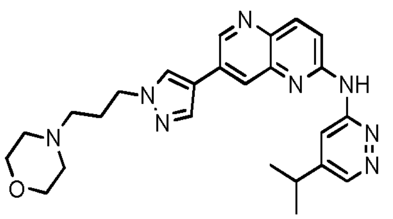
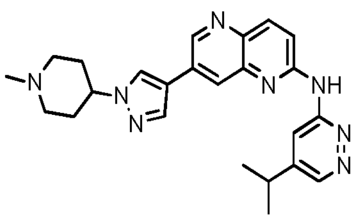
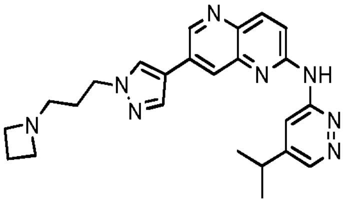
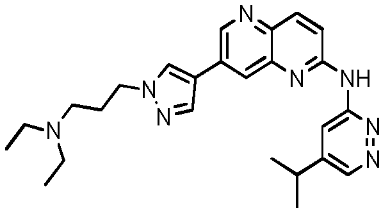
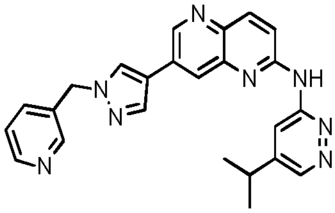

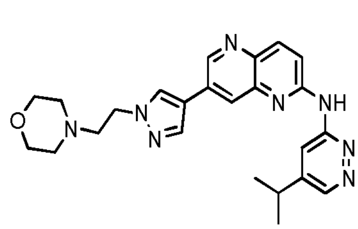
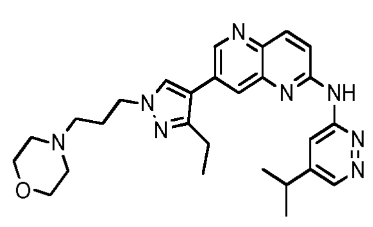
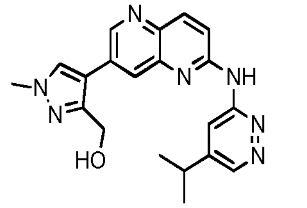

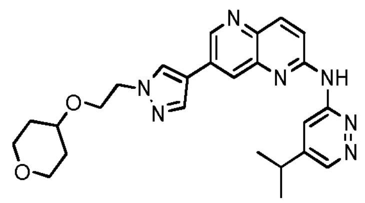
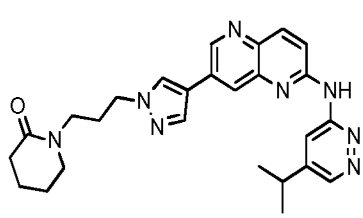
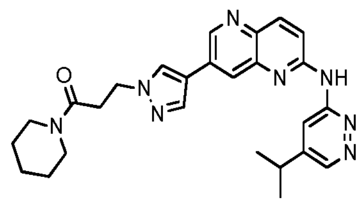

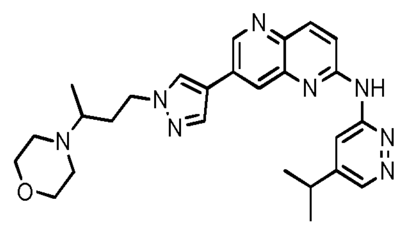
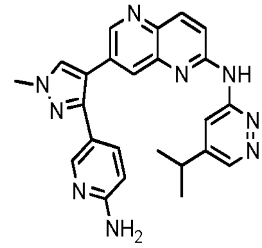
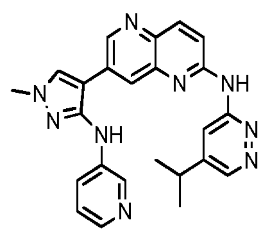

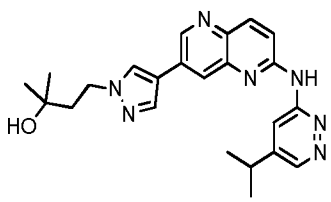
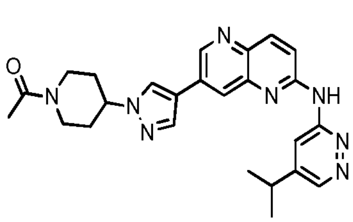
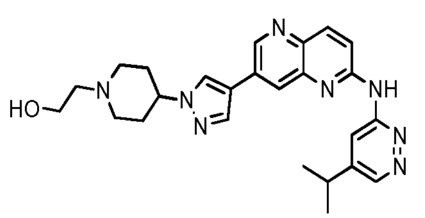

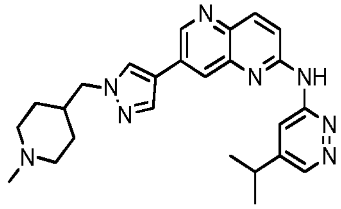
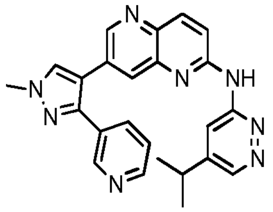
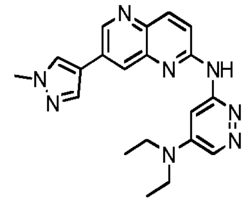

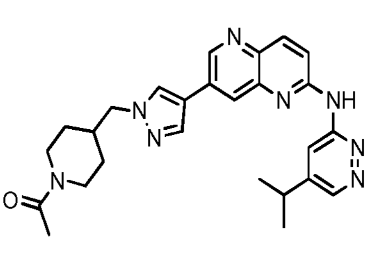
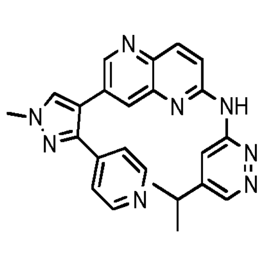
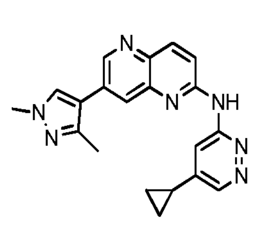

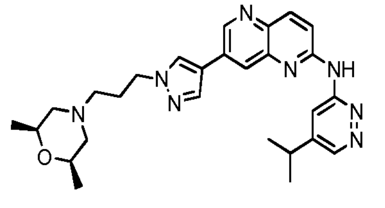
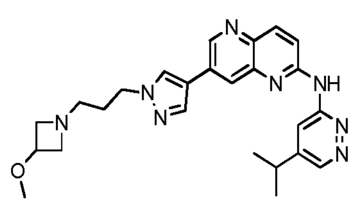
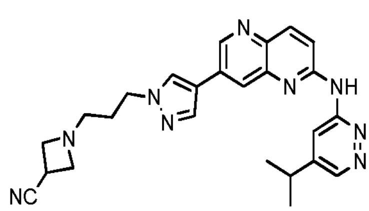

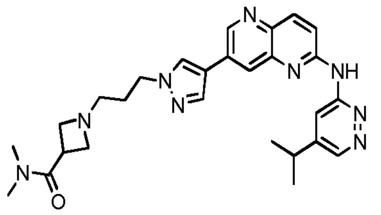
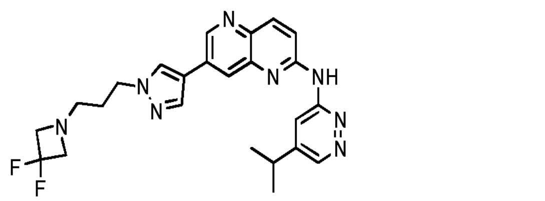

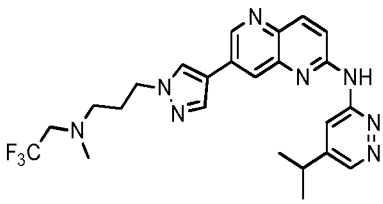
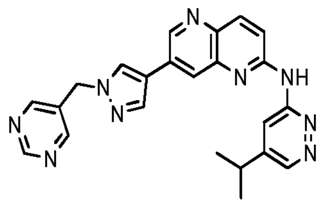
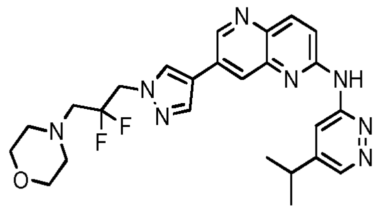

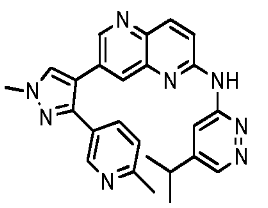
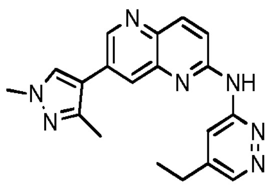
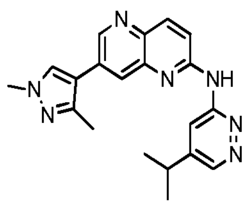

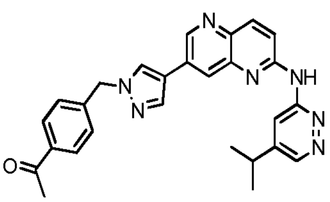
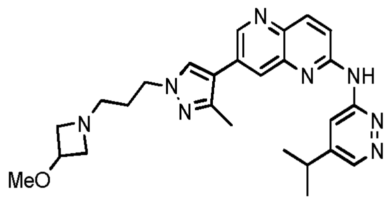
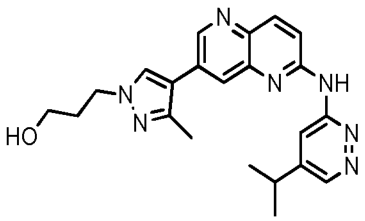

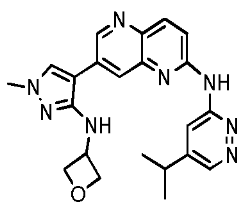
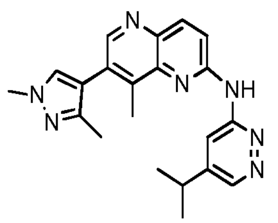

**10.** A pharmaceutical composition comprising the nitrogen-containing heterocyclic compound or salt thereof according to any one of claims 1 to 9.

**11.** The nitrogen-containing heterocyclic compound or salt thereof according to any one of claims 1 to 9, or the pharmaceutical composition of claim 10 for use in the treatment of a disease.

**12.** The nitrogen-containing heterocyclic compound or salt thereof according to any one of claims 1 to 9 or the pharmaceutical composition according to claim 10 for use in the treatment of a disease, wherein the disease is preferably selected from the group consisting of a malignant tumor, a cell proliferative disease, an allergic disease, an autoimmune disease, a neurodegenerative disease, a circulatory system disease, an inflammatory disease, an endocrine disorder, a metabolic disorder, and an infection.

## Patentansprüche

**1.** Stickstoffhaltige heterocyclische Verbindung, die durch die nachstehende Formel [1] dargestellt ist, oder ein Salz davon:

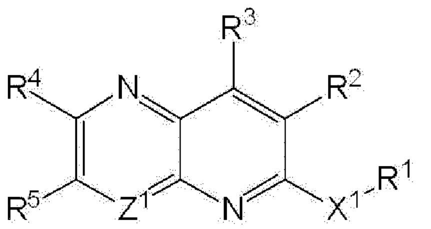

[1]

worin in Formel [1]:

$Z^1$ $CR^6$ darstellt;

$R^6$ ein Wasserstoffatom, ein Halogenatom, eine Hydroxylgruppe, eine $C_{1-6}$-Alkylgruppe, eine $C_{2-6}$-Alkenylgruppe, eine $C_{2-6}$-Alkinylgruppe, eine $C_{3-8}$-Cycloalkylgruppe, eine Arylgruppe, die mit einem oder mehreren Substituenten substituiert sein kann, ausgewählt aus einer Substituentengruppe $\alpha_2$, eine $C_{1-6}$-Alkoxygruppe, eine Aryloxygruppe, eine $C_{1-6}$-Alkylthiogruppe, eine Arylthiogruppe, eine Heteroarylgruppe, die mit einem oder mehreren Substituenten substituiert sein kann, ausgewählt aus der Substituentengruppe $\alpha_2$, eine Heteroaryloxygruppe, eine Heteroarylthiogruppe, eine Heterocyclylgruppe, die mit einem oder mehreren Substituenten substituiert sein kann, ausgewählt aus der Substituentengruppe $\alpha_2$, oder $NR^7R^8$ darstellt;

jedes aus $R^7$ und $R^8$ unabhängig ein Wasserstoffatom, eine $C_{1-6}$-Alkylgruppe, eine $C_{2-6}$-Alkenylgruppe, eine $C_{2-6}$-Alkinylgruppe, eine $C_{3-8}$-Cycloalkylgruppe, eine Arylgruppe, die mit einem oder mehreren Substituenten substituiert sein kann, ausgewählt aus der Substituentengruppe $\alpha_2$, eine Heteroarylgruppe, die mit einem oder mehreren Substituenten substituiert sein kann, ausgewählt aus der Substituentengruppe $\alpha_2$, oder eine Heterocyclylgruppe, die mit einem oder mehreren Substituenten substituiert sein kann, ausgewählt aus der Substituentengruppe $\alpha_2$, darstellt;

die Substituentengruppe $\alpha_2$ aus einem Halogenatom, einer Hydroxylgruppe, die durch eine Schutzgruppe geschützt sein kann, ausgewählt aus einer $C_{1-6}$-Alkylgruppe, einer $C_{2-6}$-Alkenylgruppe, einer Ar-C-$_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonygruppe, einer Silylgruppe, einer Tetrahydrofuranylgruppe und einer Tetrahydropyranylgruppe, einer Aminogruppe, die durch eine Schutzgruppe geschützt sein kann, ausgewählt aus einer Aryl-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, einer Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, einer Cyanogruppe, einer Carbamoylgruppe, die mit einem oder mehreren Substituenten substituiert sein kann, ausgewählt aus einer Substituentengruppe $\beta_2$, eine $C_{1-6}$-Alkylgruppe, die mit einem oder mehreren Substituenten substituiert sein kann, ausgewählt aus der Substituentengruppe $\beta_2$, einer $C_{1-6}$-Alkoxygruppe, die mit einem oder mehreren Substituenten substituiert sein kann, ausgewählt aus der Substituentengruppe $\beta_2$, und einer Oxogruppe besteht;

die Substituentengruppe $\beta_2$ aus einem Halogenatom, einer $C_{1-6}$-Alkylgruppe, die mit einem oder mehreren Substituenten substituiert sein kann, ausgewählt aus einer Substituentengruppe $\gamma_2$, einer $C_{3-8}$-Cycloalkylgruppe, die mit einem oder mehreren Substituenten substituiert sein kann, ausgewählt aus der Substituentengruppe $\gamma_2$, einer Arylgruppe, die mit einem oder mehreren Substituenten substituiert sein kann, ausgewählt aus der Substituentengruppe $\gamma_2$, einer Heteroarylgruppe, die mit einem oder mehreren Substituenten substituiert sein kann, ausgewählt aus der Substituentengruppe $\gamma_2$, und einer Heterocyclylgruppe, die mit einem oder mehreren Substituenten substituiert sein kann, ausgewählt aus der Substituentengruppe $\gamma_2$, besteht; und

die Substituentengruppe $\gamma_2$ aus einem Halogenatom, einer Hydroxylgruppe, die durch eine Schutzgruppe geschützt sein kann, ausgewählt aus einer $C_{1-6}$-Alkylgruppe, einer $C_{2-6}$-Alkenylgruppe, einer Ar-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonylgruppe, einer Silylgruppe, einer Tetrahydrofuranylgruppe und einer Tetrahydropyranylgruppe, einer Aminogruppe, die durch eine Schutzgruppe geschützt sein kann, ausgewählt aus einer Aryl-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, einer Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, einer Cyanogruppe, einer $C_{1-6}$-Alkylgruppe, die mit einem Halogenatom substituiert sein kann, einer $C_{1-6}$-Alkoxygruppe,

die mit einem Halogenatom substituiert sein kann, und einer Oxogruppe besteht;
$X^1$ $NR^9$ darstellt;
$R^9$ ein Wasserstoffatom darstellt;
$R^1$ eine monocyclische stickstoffhaltige Heteroarylgruppe, die mit einem oder mehreren Substituenten substituiert sein kann, ausgewählt aus einer Substituentengruppe $A_1$, eine monocyclische stickstoff- und sauerstoffhaltige Heteroarylgruppe, die mit einem oder mehreren Substituenten substituiert sein kann, ausgewählt aus der Substituentengruppe $A_1$, eine monocyclische stickstoff- und schwefelhaltige Heteroarylgruppe, die mit einem oder mehreren Substituenten substituiert sein kann, ausgewählt aus der Substituentengruppe $A_1$, eine bicyclische stickstoffhaltige Heteroarylgruppe, die mit einem oder mehreren Substituenten substituiert sein kann, ausgewählt aus der Substituentengruppe $A_1$, eine bicyclische stickstoff- und sauerstoffhaltige Heteroarylgruppe, die mit einem oder mehreren Substituenten substituiert sein kann, ausgewählt aus der Substituentengruppe $A_1$, oder eine bicyclische stickstoff- und schwefelhaltige Heteroarylgruppe, die mit einem oder mehreren Substituenten substituiert sein kann, ausgewählt aus der Substituentengruppe $A_1$, darstellt;
die Substituentengruppe $A_1$ aus einem Halogenatom, einer Hydroxylgruppe, die durch eine Schutzgruppe geschützt sein kann, ausgewählt aus einer $C_{1-6}$-Alkylgruppe, einer $C_{2-6}$-Alkenylgruppe, einer Ar-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonylgruppe, einer Silylgruppe, einer Tetrahydrofuranylgruppe und einer Tetrahydropyranylgruppe, einer Aminogruppe, die durch eine Schutzgruppe geschützt sein kann, ausgewählt aus einer Aryl-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, einer Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, einer Cyanogruppe, einer Carbamoylgruppe, die mit einem oder mehreren Substituenten substituiert sein kann, ausgewählt aus einer Substituentengruppe $B_1$, einer Sulfamoylgruppe, die mit einem oder mehreren Substituenten substituiert sein kann, ausgewählt aus der Substituentengruppe $B_1$, einer $C_{1-6}$-Alkylgruppe, die mit einem oder mehreren Substituenten substituiert sein kann, ausgewählt aus der Substituentengruppe $B_1$, einer $C_{2-6}$-Alkenylgruppe, die mit einem oder mehreren Substituenten substituiert sein kann, ausgewählt aus der Substituentengruppe $B_1$, einer $C_{3-8}$-Cycloalkylgruppe, die mit einem oder mehreren Substituenten substituiert sein kann, ausgewählt aus der Substituentengruppe $B_1$, einer Arylgruppe, die mit einem oder mehreren Substituenten substituiert sein kann, ausgewählt aus der Substituentengruppe $B_1$, einer $C_{1-6}$-Alkoxygruppe, die mit einem oder mehreren Substituenten substituiert sein kann, ausgewählt aus der Substituentengruppe $B_1$, einer $C_{3-8}$-Cycloalkoxygruppe, die mit einem oder mehreren Substituenten substituiert sein kann, ausgewählt aus der Substituentengruppe $B_1$, einer Aryloxygruppe, die mit einem oder mehreren Substituenten substituiert sein kann, ausgewählt aus der Substituentengruppe $B_1$, einer $C_{1-6}$-Alkylthiogruppe, die mit einem oder mehreren Substituenten substituiert sein kann, ausgewählt aus der Substituentengruppe $B_1$, einer Arylthiogruppe, die mit einem oder mehreren Substituenten substituiert sein kann, ausgewählt aus der Substituentengruppe $B_1$, einer $C_{1-6}$-Alkylaminogruppe, die mit einem oder mehreren Substituenten substituiert sein kann, ausgewählt aus der Substituentengruppe $B_1$, einer Di($C_{1-6}$-alkyl)aminogruppe, die mit einem oder mehreren Substituenten substituiert sein kann, ausgewählt aus der Substituentengruppe $B_1$, einer Acylgruppe, die mit einem oder mehreren Substituenten substituiert sein kann, ausgewählt aus der Substituentengruppe $B_1$, einer Heteroarylgruppe, die mit einem oder mehreren Substituenten substituiert sein kann, ausgewählt aus der Substituentengruppe $B_1$, einer Heterocyclylgruppe, die mit einem oder mehreren Substituenten substituiert sein kann, ausgewählt aus der Substituentengruppe $B_1$, und einer Oxogruppe besteht;
die Substituentengruppe $B_1$ aus einem Halogenatom, einer Hydroxylgruppe, die durch eine Schutzgruppe geschützt sein kann, ausgewählt aus einer $C_{1-6}$-Alkylgruppe, einer $C_{2-6}$-Alkenylgruppe, einer Ar-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonylgruppe, einer Silylgruppe, einer Tetrahydrofuranylgruppe und einer Tetrahydropyranylgruppe, einer Aminogruppe, die durch eine Schutzgruppe geschützt sein kann, ausgewählt aus einer Aryl-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, einer Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, einer Cyanogruppe, einer Carbamoylgruppe, die mit einem oder mehreren Substituenten substituiert sein kann, ausgewählt aus einer Substituentengruppe $C_1$, einer Sulfamoylgruppe, die mit einem oder mehreren Substituenten substituiert sein kann, ausgewählt aus der Substituentengruppe $C_1$, einer $C_{1-6}$-Alkylgruppe, die mit einem oder mehreren Substituenten substituiert sein kann, ausgewählt aus der Substituentengruppe $C_1$, einer $C_{3-8}$-Cyclo-

alkylgruppe, die mit einem oder mehreren Substituenten substituiert sein kann, ausgewählt aus der Substituentengruppe $C_1$, einer Arylgruppe, die mit einem oder mehreren Substituenten substituiert sein kann, ausgewählt aus der Substituentengruppe $C_1$, einer $C_{1-6}$-Alkoxygruppe, die mit einem oder mehreren Substituenten substituiert sein kann, ausgewählt aus der Substituentengruppe $C_1$, einer $C_{1-6}$-Alkylaminogruppe, die mit einem oder mehreren Substituenten substituiert sein kann, ausgewählt aus der Substituentengruppe $C_1$, einer Di ($C_{1-6}$-alkyl) aminogruppe, die mit einem oder mehreren Substituenten substituiert sein kann, ausgewählt aus der Substituentengruppe $C_1$, einer $C_{1-6}$-Alkylsulfonylgruppe, die mit einem oder mehreren Substituenten substituiert sein kann, ausgewählt aus der Substituentengruppe $C_1$, einer Arylsulfonylgruppe, die mit einem oder mehreren Substituenten substituiert sein kann, ausgewählt aus der Substituentengruppe $C_1$, einer Acylgruppe, die mit einem oder mehreren Substituenten substituiert sein kann, ausgewählt aus der Substituentengruppe $C_1$, einer Heteroarylgruppe, die mit einem oder mehreren Substituenten substituiert sein kann, ausgewählt aus der Substituentengruppe $C_1$, einer Heterocyclylgruppe, die mit einem oder mehreren Substituenten substituiert sein kann, ausgewählt aus der Substituentengruppe $C_1$, und einer Oxogruppe besteht; und

die Substituentengruppe $C_1$ aus einem Halogenatom, einer Hydroxylgruppe, die durch eine Schutzgruppe geschützt sein kann, ausgewählt aus einer $C_{1-6}$-Alkylgruppe, einer $C_{2-6}$-Alkenylgruppe, einer Ar-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonylgruppe, einer Silylgruppe, einer Tetrahydrofuranylgruppe und einer Tetrahydropyranylgruppe, einer Aminogruppe, die durch eine Schutzgruppe geschützt sein kann, ausgewählt aus einer Aryl-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, einer Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, einer Cyanogruppe, einer Carbamoylgruppe, die mit einer $C_{1-6}$-Alkylgruppe substituiert sein kann, einer $C_{1-6}$-Alkylgruppe, die mit einem Halogenatom substituiert sein kann, einer Arylgruppe, die mit einem Halogenatom oder einer $C_{1-6}$-Alkylgruppe substituiert sein kann, einer $C_{1-6}$-Alkoxygruppe, die mit einem Halogenatom substituiert sein kann, und einer Oxogruppe besteht;

$R^2$ ein Wasserstoffatom darstellt;

$R^3$ ein Wasserstoffatom darstellt;

$R^4$ ein Wasserstoffatom darstellt;

$R^5$ eine Heteroarylgruppe, die mit einem oder mehreren Substituenten substituiert sein kann, ausgewählt aus der Substituentengruppe $A_3$, eine Heteroaryloxygruppe, die mit einem oder mehreren Substituenten substituiert sein kann, ausgewählt aus der Substituentengruppe $A_3$, eine Heteroarylthiogruppe, die mit einem oder mehreren Substituenten substituiert sein kann, ausgewählt aus der Substituentengruppe $A_3$, eine Heterocyclylgruppe, die mit einem oder mehreren Substituenten substituiert sein kann, ausgewählt aus der Substituentengruppe $A_3$, oder $NR^{12}R^{13}$ darstellt;

jedes aus $R^{12}$ und $R^{13}$ unabhängig ein Wasserstoffatom, eine $C_{1-6}$-Alkylgruppe, die mit einem oder mehreren Substituenten substituiert sein kann, ausgewählt aus der Substituentengruppe $A_3$, eine $C_{2-6}$-Alkenylgruppe, die mit einem oder mehreren Substituenten substituiert sein kann, ausgewählt aus der Substituentengruppe $A_3$, eine $C_{2-6}$-Alkinylgruppe, die mit einem oder mehreren Substituenten substituiert sein kann, ausgewählt aus der Substituentengruppe $A_3$, eine $C_{3-8}$-Cycloalkylgruppe, die mit einem oder mehreren Substituenten substituiert sein kann, ausgewählt aus der Substituentengruppe $A_3$, eine Arylgruppe, die mit einem oder mehreren Substituenten substituiert sein kann, ausgewählt aus der Substituentengruppe $A_3$, eine Heteroarylgruppe, die mit einem oder mehreren Substituenten substituiert sein kann, ausgewählt aus der Substituentengruppe $A_3$, eine Heterocyclylgruppe, die mit einem oder mehreren Substituenten substituiert sein kann, ausgewählt aus der Substituentengruppe $A_3$, oder eine Aminoschutzgruppe, ausgewählt aus einer Aryl-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, darstellt;

die Substituentengruppe $A_3$ aus einem Halogenatom, einer Hydroxylgruppe, einer Aminogruppe, einer Aminogruppe, die mit einem oder mehreren Substituenten substituiert sein kann, ausgewählt aus einer Substituentengruppe $E_3$, einer Carboxylgruppe, einer Cyanogruppe, einer Carbamoylgruppe, die mit einem oder mehreren Substituenten substituiert sein kann, ausgewählt aus einer Substituentengruppe $B_3$, einer Sulfamoylgruppe, die mit einem oder mehreren Substituenten substituiert sein kann, ausgewählt aus der Substituentengruppe $B_3$, einer $C_{1-6}$-Alkylgruppe, die mit einem oder mehreren Substituenten substituiert sein kann, ausgewählt aus der Substituentengruppe $B_3$, einer $C_{2-6}$-Alkenylgruppe, die mit einem oder mehreren Substituenten substituiert sein kann, ausgewählt aus der Substituentengruppe $B_3$, einer $C_{3-8}$-Cycloalkylgruppe, die mit einem oder mehreren Substituenten substituiert sein kann, ausgewählt aus der Substituentengruppe $B_3$, einer Arylgruppe, die

mit einem oder mehreren Substituenten substituiert sein kann, ausgewählt aus der Substituentengruppe $B_3$, einer $C_{1-6}$-Alkoxygruppe, die mit einem oder mehreren Substituenten substituiert sein kann, ausgewählt aus der Substituentengruppe $B_3$, einer Aryloxygruppe, die mit einem oder mehreren Substituenten substituiert sein kann, ausgewählt aus der Substituentengruppe $B_3$, einer $C_{1-6}$-Alkylthiogruppe, die mit einem oder mehreren Substituenten substituiert sein kann, ausgewählt aus der Substituentengruppe $B_3$, einer Arylthiogruppe, die mit einem oder mehreren Substituenten substituiert sein kann, ausgewählt aus der Substituentengruppe $B_3$, einer $C_{1-6}$-Alkylaminogruppe, die mit einem oder mehreren Substituenten substituiert sein kann, ausgewählt aus der Substituentengruppe $B_3$, einer Di($C_{1-6}$-alkyl)aminogruppe, die mit einem oder mehreren Substituenten substituiert sein kann, ausgewählt aus der Substituentengruppe $B_3$, einer $C_{1-6}$-Alkylsulfonylgruppe, die mit einem oder mehreren Substituenten substituiert sein kann, ausgewählt aus der Substituentengruppe $B_3$, einer Arylsulfonylgruppe, die mit einem oder mehreren Substituenten substituiert sein kann, ausgewählt aus der Substituentengruppe $B_3$, einer Acylgruppe, die mit einem oder mehreren Substituenten substituiert sein kann, ausgewählt aus der Substituentengruppe $B_3$, einer Heteroarylgruppe, die mit einem oder mehreren Substituenten substituiert sein kann, ausgewählt aus der Substituentengruppe $B_3$, einer Heterocyclylgruppe, die mit einem oder mehreren Substituenten substituiert sein kann, ausgewählt aus der Substituentengruppe $B_3$, einer Heterocyclyloxygruppe, die mit einem oder mehreren Substituenten substituiert sein kann, ausgewählt aus der Substituentengruppe $B_3$, und einer Oxogruppe besteht;

die Substituentengruppe $B_3$ aus einem Halogenatom, einer Hydroxylgruppe, die durch eine Schutzgruppe geschützt sein kann, ausgewählt aus einer $C_{1-6}$-Alkylgruppe, einer $C_{1-6}$-Alkenylgruppe, einer Ar-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonylgruppe, einer Silylgruppe, einer Tetrahydrofuranylgruppe und einer Tetrahydropyranylgruppe, einer Aminogruppe, die durch eine Schutzgruppe geschützt sein kann, ausgewählt aus einer Aryl-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, einer Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, einer Cyanogruppe, einer Carbamoylgruppe, die mit einem oder mehreren Substituenten substituiert sein kann, ausgewählt aus einer Substituentengruppe $C_3$, einer $C_{1-6}$-Alkylgruppe, die mit einem oder mehreren Substituenten substituiert sein kann, ausgewählt aus der Substituentengruppe $C_3$, einer $C_{3-8}$-Cycloalkylgruppe, die mit einem oder mehreren Substituenten substituiert sein kann, ausgewählt aus der Substituentengruppe $C_3$, einer Arylgruppe, die mit einem oder mehreren Substituenten substituiert sein kann, ausgewählt aus der Substituentengruppe $C_3$, einer $C_{1-6}$-Alkoxygruppe, die mit einem oder mehreren Substituenten substituiert sein kann, ausgewählt aus der Substituentengruppe $C_3$, einer Aryloxygruppe, die mit einem oder mehreren Substituenten substituiert sein kann, ausgewählt aus der Substituentengruppe $C_3$, einer $C_{1-6}$-Alkylaminogruppe, die mit einem oder mehreren Substituenten substituiert sein kann, ausgewählt aus der Substituentengruppe $C_3$, einer Di($C_{1-6}$-alkyl)aminogruppe, die mit einem oder mehreren Substituenten substituiert sein kann, ausgewählt aus der Substituentengruppe $C_3$, einer $C_{1-6}$-Alkylsulfonylgruppe, die mit einem oder mehreren Substituenten substituiert sein kann, ausgewählt aus der Substituentengruppe $C_3$, einer Arylsulfonylgruppe, die mit einem oder mehreren Substituenten substituiert sein kann, ausgewählt aus der Substituentengruppe $C_3$, einer Acylgruppe, die mit einem oder mehreren Substituenten substituiert sein kann, ausgewählt aus der Substituentengruppe $C_3$, einer Heteroarylgruppe, die mit einem oder mehreren Substituenten substituiert sein kann, ausgewählt aus der Substituentengruppe $C_3$, einer Heterocyclylgruppe, die mit einem oder mehreren Substituenten substituiert sein kann, ausgewählt aus der Substituentengruppe $C_3$, einer Heteroaryloxygruppe, die mit einem oder mehreren Substituenten substituiert sein kann, ausgewählt aus der Substituentengruppe $C_3$, einer Heterocyclyloxygruppe, die mit einem oder mehreren Substituenten substituiert sein kann, ausgewählt aus der Substituentengruppe $C_3$, und einer Oxogruppe besteht;

die Substituentengruppe $C_3$ aus einem Halogenatom, einer Hydroxylgruppe, einer Aminogruppe, einer Carboxylgruppe, einer Nitrogruppe, einer Cyanogruppe, einer Carbamoylgruppe die mit einem oder mehreren Substituenten substituiert sein kann, ausgewählt aus einer Substituentengruppe $D_3$, einer $C_{1-6}$-Alkylgruppe, die mit einem oder mehreren Substituenten substituiert sein kann, ausgewählt aus der Substituentengruppe $D_3$, einer $C_{3-8}$-Cycloalkylgruppe, die mit einem oder mehreren Substituenten substituiert sein kann, ausgewählt aus der Substituentengruppe $D_3$, einer Arylgruppe, die mit einem oder mehreren Substituenten substituiert sein kann, ausgewählt aus der Substituentengruppe $D_3$, einer $C_{1-6}$-Alkoxygruppe, die mit einem oder mehreren Substituenten substituiert sein kann, ausgewählt aus der Substituentengruppe $D_3$, einer Aryloxygruppe, die mit einem oder mehreren Substituenten substituiert sein kann, ausgewählt aus der Substituentengruppe $D_3$, einer $C_{1-6}$-Alkylaminogruppe, die mit einem oder mehreren Substituenten substituiert sein kann, ausgewählt aus der Substituentengruppe $D_3$, einer Di ($C_{1-6}$-alkyl) aminogruppe, die mit einem oder mehreren Substituenten substituiert

sein kann, ausgewählt aus der Substituentengruppe $D_3$, einer $C_{1-6}$-Alkylsulfonylgruppe, die mit einem oder mehreren Substituenten substituiert sein kann, ausgewählt aus der Substituentengruppe $D_3$, einer Acylgruppe, die mit einem oder mehreren Substituenten substituiert sein kann, ausgewählt aus der Substituentengruppe $D_3$, einer Heteroarylgruppe, die mit einem oder mehreren Substituenten substituiert sein kann, ausgewählt aus der Substituentengruppe $D_3$, einer Heterocyclylgruppe, die mit einem oder mehreren Substituenten substituiert sein kann, ausgewählt aus der Substituentengruppe $D_3$, einer Silylgruppe, die mit einem oder mehreren Substituenten substituiert sein kann, ausgewählt aus der Substituentengruppe $D_3$, und einer Oxogruppe besteht;

die Substituentengruppe $D_3$ aus einem Halogenatom, einer Hydroxylgruppe, einer $C_{1-6}$-Alkylgruppe, einer $C_{3-8}$-Cycloalkylgruppe, einer Arylgruppe, einer $C_{1-6}$-Alkoxygruppe, einer $C_{1-6}$-Alkylaminogruppe, einer Di ($C_{1-6}$-alkyl) aminogruppe, einer $C_{1-6}$-Alkylsulfonylgruppe, einer Heteroarylgruppe, einer Heterocyclylgruppe und einer Oxogruppe besteht;

die Substituentengruppe $E_3$ aus einer $C_{3-8}$-Cycloalkylgruppe, die mit einem oder mehreren Substituenten substituiert sein kann, ausgewählt aus einer Substituentengruppe $F_3$, einer Arylgruppe, die mit einem oder mehreren Substituenten substituiert sein kann, ausgewählt aus der Substituentengruppe $F_3$, einer Heteroarylgruppe, die mit einem oder mehreren Substituenten substituiert sein kann, ausgewählt aus der Substituentengruppe $F_3$, und einer Heterocyclylgruppe, die mit einem oder mehreren Substituenten substituiert sein kann, ausgewählt aus der Substituentengruppe $F_3$, besteht; und

die Substituentengruppe $F_3$ aus einem Halogenatom, einer Carboxylgruppe und einer $C_{1-6}$-Alkylgruppe besteht.

**2.** Stickstoffhaltige heterocyclische Verbindung oder Salz davon gemäß Anspruch 1, worin:
$R^1$ eine Pyrazolylgruppe, die mit einem oder mehreren Substituenten substituiert sein kann, ausgewählt aus der Substituentengruppe $A_1$, eine Imidazolylgruppe, die mit einem oder mehreren Substituenten substituiert sein kann, ausgewählt aus der Substituentengruppe $A_1$, eine Triazolylgruppe, die mit einem oder mehreren Substituenten substituiert sein kann, ausgewählt aus der Substituentengruppe $A_1$, eine Thiazolylgruppe, die mit einem oder mehreren Substituenten substituiert sein kann, ausgewählt aus der Substituentengruppe $A_1$, einer Oxadiazolylgruppe, die mit einem oder mehreren Substituenten substituiert sein kann, ausgewählt aus der Substituentengruppe $A_1$, eine Thiadiazolylgruppe, die mit einem oder mehreren Substituenten substituiert sein kann, ausgewählt aus der Substituentengruppe $A_1$, eine Pyridylgruppe, die mit einem oder mehreren Substituenten substituiert sein kann, ausgewählt aus der Substituentengruppe $A_1$, oder eine Pyridazinylgruppe, die mit einem oder mehreren Substituenten substituiert sein kann, ausgewählt aus der Substituentengruppe $A_1$, darstellt.

**3.** Stickstoffhaltige heterocyclische Verbindung oder Salz davon gemäß Anspruch 1 oder 2, worin:

$R^1$ eine Pyrazolylgruppe, die mit einem oder mehreren Substituenten substituiert sein kann, ausgewählt aus einer Substituentengruppe $\alpha_1$, eine Imidazolylgruppe, die mit einem oder mehreren Substituenten substituiert sein kann, ausgewählt aus der Substituentengruppe $\alpha_1$, eine Triazolylgruppe, die mit einem oder mehreren Substituenten substituiert sein kann, ausgewählt aus der Substituentengruppe $\alpha_1$, eine Thiazolylgruppe, die mit einem oder mehreren Substituenten substituiert sein kann, ausgewählt aus der Substituentengruppe $\alpha_1$, eine Oxadiazolylgruppe, die mit einem oder mehreren Substituenten substituiert sein kann, ausgewählt aus der Substituentengruppe $\alpha_1$, eine Thiadiazolylgruppe, die mit einem oder mehreren Substituenten substituiert sein kann, ausgewählt aus der Substituentengruppe $\alpha_1$, eine Pyridylgruppe, die mit einem oder mehreren Substituenten substituiert sein kann, ausgewählt aus der Substituentengruppe $\alpha_1$, oder eine Pyridazinylgruppe, die mit einem oder mehreren Substituenten substituiert sein kann, ausgewählt aus der Substituentengruppe $\alpha_1$, darstellt;

die Substituentengruppe $\alpha_1$ aus einem Halogenatom, einer Hydroxylgruppe, die durch eine Schutzgruppe geschützt sein kann, ausgewählt aus einer $C_{1-6}$-Alkylgruppe, einer $C_{2-6}$-Alkenylgruppe, einer Ar-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonylgruppe, einer Silylgruppe, einer Tetrahydrofuranylgruppe und einer Tetrahydropyranylgruppe, einer Aminogruppe, die durch eine Schutzgruppe geschützt sein kann, ausgewählt aus einer Aryl-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, einer Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, einer Cyanogruppe, einer Carbamoylgruppe, die mit einem oder mehreren Substituenten substituiert sein kann, ausgewählt aus einer Substituentengruppe $\beta_1$, einer Sulfamoylgruppe, die mit einem oder mehreren Substituenten substituiert sein kann, ausgewählt aus der Substituentengruppe $\beta_1$, einer $C_{1-6}$-Alkylgruppe, die mit einem oder mehreren Substituenten substituiert sein kann, ausgewählt aus der Substituentengruppe $\beta1$, einer $C_{3-8}$-Cyclo-

alkylgruppe, die mit einem oder mehreren Substituenten substituiert sein kann, ausgewählt aus der Substituentengruppe $\beta_1$, einer Arylgruppe, die mit einem oder mehreren Substituenten substituiert sein kann, ausgewählt aus der Substituentengruppe $\beta_1$, einer $C_{1-6}$-Alkoxygruppe, die mit einem oder mehreren Substituenten substituiert sein kann, ausgewählt aus der Substituentengruppe $\beta_1$, einer Aryloxygruppe, die mit einem oder mehreren Substituenten substituiert sein kann, ausgewählt aus der Substituentengruppe $\beta_1$, einer $C_{1-6}$-Alkylthiogruppe, die mit einem oder mehreren Substituenten substituiert sein kann, ausgewählt aus der Substituentengruppe $\beta_1$, einer Arylthiogruppe, die mit einem oder mehreren Substituenten substituiert sein kann, ausgewählt aus der Substituentengruppe $\beta_1$, einer $C_{1-6}$-Alkylaminogruppe, die mit einem oder mehreren Substituenten substituiert sein kann, ausgewählt aus der Substituentengruppe $\beta_1$, einer Di($C_{1-6}$-alkyl)aminogruppe, die mit einem oder mehreren Substituenten substituiert sein kann, ausgewählt aus der Substituentengruppe $\beta_1$, einer Acylgruppe, die mit einem oder mehreren Substituenten substituiert sein kann, ausgewählt aus der Substituentengruppe $\beta_1$, einer Heteroarylgruppe, die mit einem oder mehreren Substituenten substituiert sein kann, ausgewählt aus der Substituentengruppe $\beta_1$, einer Heterocyclylgruppe, die mit einem oder mehreren Substituenten substituiert sein kann, ausgewählt aus der Substituentengruppe $\beta_1$, und einer Oxogruppe besteht;
die Substituentengruppe $\beta_1$ aus einem Halogenatom, einer Hydroxylgruppe, die durch eine Schutzgruppe geschützt sein kann, ausgewählt aus einer $C_{1-6}$-Alkylgruppe, einer $C_{2-6}$-Alkenylgruppe, einer Ar-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonylgruppe, einer Silylgruppe, einer Tetrahydrofuranylgruppe und einer Tetrahydropyranylgruppe, einer Aminogruppe, die durch eine Schutzgruppe geschützt sein kann, ausgewählt aus einer Aryl-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, einer Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, einer Cyanogruppe, einer Carbamoylgruppe, die mit einem oder mehreren Substituenten substituiert sein kann, ausgewählt aus einer Substituentengruppe $\gamma_1$, einer $C_{1-6}$-Alkylgruppe, die mit einem oder mehreren Substituenten substituiert sein kann, ausgewählt aus der Substituentengruppe $\gamma_1$, einer $C_{3-8}$-Cycloalkylgruppe, die mit einem oder mehreren Substituenten substituiert sein kann, ausgewählt aus der Substituentengruppe $\gamma_1$, einer Arylgruppe, die mit einem oder mehreren Substituenten substituiert sein kann, ausgewählt aus der Substituentengruppe $\gamma_1$, einer $C_{1-6}$-Alkoxygruppe, die mit einem oder mehreren Substituenten substituiert sein kann, ausgewählt aus der Substituentengruppe $\gamma_1$, einer $C_{1-6}$-Alkylaminogruppe, die mit einem oder mehreren Substituenten substituiert sein kann, ausgewählt aus der Substituentengruppe $\gamma_1$, einer Di($C_{1-6}$-alkyl)aminogruppe, die mit einem oder mehreren Substituenten substituiert sein kann, ausgewählt aus der Substituentengruppe $\gamma_1$, einer $C_{1-6}$-Alkylsulfonylgruppe, die mit einem oder mehreren Substituenten substituiert sein kann, ausgewählt aus der Substituentengruppe $\gamma_1$, einer Arylsulfonylgruppe, die mit einem oder mehreren Substituenten substituiert sein kann, ausgewählt aus der Substituentengruppe $\gamma_1$, einer Acylgruppe, die mit einem oder mehreren Substituenten substituiert sein kann, ausgewählt aus der Substituentengruppe $\gamma_1$, einer Heteroarylgruppe, die mit einem oder mehreren Substituenten substituiert sein kann, ausgewählt aus der Substituentengruppe $\gamma_1$, einer Heterocyclylgruppe, die mit einem oder mehreren Substituenten substituiert sein kann, ausgewählt aus der Substituentengruppe $\gamma_1$, und einer Oxogruppe besteht; und
die Substituentengruppe $\gamma_1$ aus einem Halogenatom, einer Hydroxylgruppe, die durch eine Schutzgruppe geschützt sein kann, ausgewählt aus einer $C_{1-6}$-Alkylgruppe, einer $C_{2-6}$-Alkenylgruppe, einer Ar-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonylgruppe, einer Silylgruppe, einer Tetrahydrofuranylgruppe und einer Tetrahydropyranylgruppe, einer Aminogruppe, die durch eine Schutzgruppe geschützt sein kann, ausgewählt aus einer Aryl-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, einer Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, einer Cyanogruppe, einer Carbamoylgruppe, die mit einer $C_{1-6}$-Alkylgruppe substituiert sein kann, eine $C_{1-6}$-Alkylgruppe, die mit einem Halogenatom substituiert sein kann, einer $C_{1-6}$-Alkoxygruppe, die mit einem Halogenatom substituiert sein kann, und einer Oxogruppe besteht.

**4.** Stickstoffhaltige heterocyclische Verbindung oder Salz davon gemäß mindestens einem der Ansprüche 1 bis 3, worin:

$Z^1$ CH darstellt;
$R^5$ eine Heteroarylgruppe, die mit einem oder mehreren Substituenten substituiert sein kann, ausgewählt aus

der Substituentengruppe $A_3$, eine Heterocyclylgruppe, die mit einem oder mehreren Substituenten substituiert sein kann, ausgewählt aus der Substituentengruppe $A_3$, oder $NR^{12b}R^{13b}$ darstellt;
$R^{12b}$ ein Wasserstoffatom oder eine $C_{1-6}$-Alkylgruppe darstellt;
$R^{13b}$ eine $C_{3-8}$-Cycloalkylgruppe, die mit einem oder mehreren Substituenten substituiert sein kann, ausgewählt aus der Substituentengruppe $A_3$, eine Arylgruppe, die mit einem oder mehreren Substituenten substituiert sein kann, ausgewählt aus der Substituentengruppe $A_3$, eine Heteroarylgruppe, die mit einem oder mehreren Substituenten substituiert sein kann, ausgewählt aus der Substituentengruppe $A_3$, oder eine Heterocyclylgruppe, die mit einem oder mehreren Substituenten substituiert sein kann, ausgewählt aus der Substituentengruppe $A_3$, darstellt.

**5.** Stickstoffhaltige heterocyclische Verbindung oder Salz davon gemäß mindestens einem der Ansprüche 1 bis 3, worin:

$Z^1$ CH darstellt;
$R^5$ eine Heteroarylgruppe, die mit einem oder mehreren Substituenten substituiert sein kann, ausgewählt aus der Substituentengruppe $\alpha_3$, eine Heterocyclylgruppe, die mit einem oder mehreren Substituenten substituiert sein kann, ausgewählt aus der Substituentengruppe $\alpha_3$, oder $NR^{12b}R^{13c}$ darstellt;
$R^{12b}$ ein Wasserstoffatom oder eine $C_{1-6}$-Alkylgruppe darstellt;
$R^{13c}$ eine $C_{3-8}$-Cycloalkylgruppe, die mit einem oder mehreren Substituenten substituiert sein kann, ausgewählt aus der Substituentengruppe $\alpha_3$, eine Arylgruppe, die mit einem oder mehreren Substituenten substituiert sein kann, ausgewählt aus der Substituentengruppe $\alpha_3$, eine Heteroarylgruppe, die mit einem oder mehreren Substituenten substituiert sein kann, ausgewählt aus der Substituentengruppe $\alpha_3$, oder eine Heterocyclylgruppe, die mit einem oder mehreren Substituenten substituiert sein kann, ausgewählt aus der Substituentengruppe $\alpha_3$, darstellt;
die Substituentengruppe $\alpha_3$ aus einem Halogenatom, einer Hydroxylgruppe, die durch eine Schutzgruppe geschützt sein kann, ausgewählt aus einer $C_{1-6}$-Alkylgruppe, einer $C_{2-6}$-Alkenylgruppe, einer Ar-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonylgruppe, einer Silylgruppe, einer Tetrahydrofuranylgruppe und einer Tetrahydropyranylgruppe, einer Aminogruppe, die durch eine Schutzgruppe geschützt sein kann, ausgewählt aus einer Aryl-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, einer Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, einer Cyanogruppe, einer Carbamoylgruppe, die mit einem oder mehreren Substituenten substituiert sein kann, ausgewählt aus einer Substituentengruppe $\beta_3$, einer $C_{1-6}$-Alkylgruppe, die mit einem oder mehreren Substituenten substituiert sein kann, ausgewählt aus der Substituentengruppe $\beta_3$, einer $C_{3-8}$-Cycloalkylgruppe, die mit einem oder mehreren Substituenten substituiert sein kann, ausgewählt aus der Substituentengruppe $\beta_3$, einer Arylgruppe, die mit einem oder mehreren Substituenten substituiert sein kann, ausgewählt aus der Substituentengruppe $\beta_3$, einer $C_{1-6}$-Alkoxygruppe, die mit einem oder mehreren Substituenten substituiert sein kann, ausgewählt aus der Substituentengruppe $\beta_3$, einer Aryloxygruppe, die mit einem oder mehreren Substituenten substituiert sein kann, ausgewählt aus der Substituentengruppe $\beta_3$, einer $C_{1-6}$-Alkylthiogruppe, die mit einem oder mehreren Substituenten substituiert sein kann, ausgewählt aus der Substituentengruppe $\beta_3$, einer Arylthiogruppe, die mit einem oder mehreren Substituenten substituiert sein kann, ausgewählt aus der Substituentengruppe $\beta_3$, einer $C_{1-6}$-Alkylaminogruppe, die mit einem oder mehreren Substituenten substituiert sein kann, ausgewählt aus der Substituentengruppe $\beta_3$, einer Di ($C_{1-6}$-alkyl) aminogruppe, die mit einem oder mehreren Substituenten substituiert sein kann, ausgewählt aus der Substituentengruppe $\beta_3$, einer $C_{1-6}$-Alkylsulfonylgruppe, die mit einem oder mehreren Substituenten substituiert sein kann, ausgewählt aus der Substituentengruppe $\beta_3$, einer Arylsulfonylgruppe, die mit einem oder mehreren Substituenten substituiert sein kann, ausgewählt aus der Substituentengruppe $\beta_3$, einer Acylgruppe, die mit einem oder mehreren Substituenten substituiert sein kann, ausgewählt aus der Substituentengruppe $\beta_3$, einer Heteroarylgruppe, die mit einem oder mehreren Substituenten substituiert sein kann, ausgewählt aus der Substituentengruppe $\beta_3$, einer Heterocyclylgruppe, die mit einem oder mehreren Substituenten substituiert sein kann, ausgewählt aus der Substituentengruppe $\beta_3$, einer Heterocyclyloxygruppe, die mit einem oder mehreren Substituenten substituiert sein kann, ausgewählt aus der Substituentengruppe $\beta_3$, und einer Oxogruppe besteht;
die Substituentengruppe $\beta_3$ aus einem Halogenatom, einer Hydroxylgruppe, die durch eine Schutzgruppe geschützt sein kann, ausgewählt aus einer $C_{1-6}$-Alkylgruppe, einer $C_{2-6}$-Alkenylgruppe, einer Ar-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonylgruppe, einer Silylgruppe, einer Tetrahydrofuranylgruppe und einer Tetrahydropyranylgruppe, einer Aminogruppe, die durch eine Schutzgruppe geschützt sein kann, ausgewählt aus einer Aryl-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, einer Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, einer Cyanogruppe, einer Carbamoylgruppe, die mit einem oder mehreren Substituenten substituiert sein kann, ausgewählt aus einer Substituentengruppe $\gamma_3$, einer $C_{1-6}$-Alkylgruppe, die mit einem oder mehreren Substituenten substituiert sein kann, ausgewählt aus der Substituentengruppe $\gamma_3$, einer $C_{3-8}$-Cycloalkylgruppe, die mit einem oder mehreren Substituenten substituiert sein kann, ausgewählt aus der Substituentengruppe $\gamma_3$, einer Arylgruppe, die mit einem oder mehreren Substituenten substituiert sein kann, ausgewählt aus der Substituentengruppe $\gamma_3$, einer $C_{1-6}$-Alkoxygruppe, die mit einem oder mehreren Substituenten substituiert sein kann, ausgewählt aus der Substituentengruppe $\gamma_3$, einer $C_{1-6}$-Alkylaminogruppe, die mit einem oder mehreren Substituenten substituiert sein kann, ausgewählt aus der Substituentengruppe $\gamma_3$, einer Di($C_{1-6}$-alkyl)aminogruppe, die mit einem oder mehreren Substituenten substituiert sein kann, ausgewählt aus der Substituentengruppe $\gamma_3$, einer $C_{1-6}$-Alkylsulfonylgruppe, die mit einem oder mehreren Substituenten substituiert sein kann, ausgewählt aus der Substituentengruppe $\gamma_3$, einer Arylsulfonylgruppe, die mit einem oder mehreren Substituenten substituiert sein kann, ausgewählt aus der Substituentengruppe $\gamma_3$, einer Heteroarylgruppe, die mit einem oder mehreren Substituenten substituiert sein kann, ausgewählt aus der Substituentengruppe $\gamma_3$, einer Heterocyclylgruppe, die mit einem oder mehreren Substituenten substituiert sein kann, ausgewählt aus der Substituentengruppe $\gamma_3$, einer Heterocyclyloxygruppe, die mit einem oder mehreren Substituenten substituiert sein kann, ausgewählt aus der Substituentengruppe $\gamma_3$, und einer Oxogruppe besteht; und

die Substituentengruppe $\gamma_3$ aus einem Halogenatom, einer Hydroxylgruppe, die durch eine Schutzgruppe geschützt sein kann, ausgewählt aus einer $C_{1-6}$-Alkylgruppe, einer $C_{2-6}$-Alkenylgruppe, einer Ar-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonylgruppe, einer Silylgruppe, einer Tetrahydrofuranylgruppe und einer Tetrahydropyranylgruppe, einer Aminogruppe, die durch eine Schutzgruppe geschützt sein kann, ausgewählt aus einer Aryl-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, einer Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, einer Cyanogruppe, einer $C_{1-6}$-Alkylgruppe, die mit einem Halogenatom substituiert sein kann, einer $C_{1-6}$-Alkoxygruppe, die mit einem Halogenatom substituiert sein kann, und einer Oxogruppe besteht.

**6.** Stickstoffhaltige heterocyclische Verbindung oder Salz davon gemäß mindestens einem der Ansprüche 1 bis 3, worin:

$Z^1$ CH darstellt;

$R^5$ eine Pyrazolylgruppe, die mit einem oder mehreren Substituenten substituiert sein kann, ausgewählt aus der Substituentengruppe $\alpha_3$, eine Isoxazolylgruppe, die mit einem oder mehreren Substituenten substituiert sein kann, ausgewählt aus der Substituentengruppe $\alpha_3$, eine Pyridylgruppe, die mit einem oder mehreren Substituenten substituiert sein kann, ausgewählt aus der Substituentengruppe $\alpha_3$, eine Pyrimidinylgruppe, die mit einem oder mehreren Substituenten substituiert sein kann, ausgewählt aus der Substituentengruppe $\alpha_3$, eine Pyrazinylgruppe, die mit einem oder mehreren Substituenten substituiert sein kann, ausgewählt aus der Substituentengruppe $\alpha_3$, eine Pyridazinylgruppe, die mit einem oder mehreren Substituenten substituiert sein kann, ausgewählt aus der Substituentengruppe $\alpha_3$, ein Azetidinyl, das mit einem oder mehreren Substituenten substituiert sein kann, ausgewählt aus der Substituentengruppe $\alpha_3$, eine Pyrrolidinylgruppe, die mit einem oder mehreren Substituenten substituiert sein kann, ausgewählt aus der Substituentengruppe $\alpha_3$, eine Piperidinylgruppe, die mit einem oder mehreren Substituenten substituiert sein kann, ausgewählt aus der Substituentengruppe $\alpha_3$, eine Piperazinylgruppe, die mit einem oder mehreren Substituenten substituiert sein kann, ausgewählt aus der Substituentengruppe $\alpha_3$, eine Morpholinylgruppe, die mit einem oder mehreren Substituenten substituiert sein kann, ausgewählt aus der Substituentengruppe $\alpha_3$, eine Homopiperazinylgruppe, die mit einem oder mehreren Substituenten substituiert sein kann, ausgewählt aus der Substituentengruppe $\alpha_3$, oder $NR^{12b}R^{13d}$ darstellt;

$R^{12b}$ ein Wasserstoffatom oder eine $C_{1-6}$-Alkylgruppe darstellt;

$R^{13d}$ eine Phenylgruppe, die mit einem oder mehreren Substituenten substituiert sein kann, ausgewählt aus der Substituentengruppe $\alpha_3$, eine Pyrazolylgruppe, die mit einem oder mehreren Substituenten substituiert sein kann, ausgewählt aus der Substituentengruppe $\alpha_3$, eine Isoxazolylgruppe, die mit einem oder mehreren Substituenten substituiert sein kann, ausgewählt aus der Substituentengruppe $\alpha_3$, eine Pyridylgruppe, die mit einem oder mehreren Substituenten substituiert sein kann, ausgewählt aus der Substituentengruppe $\alpha_3$, eine Pyrimidinylgruppe, die mit einem oder mehreren Substituenten substituiert sein kann, ausgewählt aus der Substituentengruppe $\alpha_3$, eine Pyrazinylgruppe, die mit einem oder mehreren Substituenten substituiert sein kann, ausgewählt aus der Substituentengruppe $\alpha_3$, eine Pyridazinylgruppe, die mit einem oder mehreren Substituenten substituiert sein kann, ausgewählt aus der Substituentengruppe $\alpha_3$, einer Pyrrolidinylgruppe, die mit einem oder mehreren Substituenten substituiert sein kann, ausgewählt aus der Substituentengruppe $\alpha_3$, einer Piperidinylgruppe, die mit einem oder mehreren Substituenten substituiert sein kann, ausgewählt aus der Substituentengruppe $\alpha_3$, oder eine Tetrahydropyridylgruppe, die mit einem oder mehreren Substituenten substituiert sein kann, ausgewählt aus der Substituentengruppe $\alpha_3$, darstellt;

die Substituentengruppe $\alpha_3$ aus einem Halogenatom, einer Hydroxylgruppe, die durch eine Schutzgruppe geschützt sein kann, ausgewählt aus einer $C_{1-6}$-Alkylgruppe, einer $C_{2-6}$-Alkenylgruppe, einer Ar-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonylgruppe, einer Silylgruppe, einer Tetrahydrofuranylgruppe und einer Tetrahydropyranylgruppe, einer Aminogruppe, die durch eine Schutzgruppe geschützt sein kann, ausgewählt aus einer Aryl-$C_{1-6}$-alkylgruppe, einer $C_{i-6}$-Alkoxy-$C_{i-6}$-alkylgruppe, einer Acylgruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, einer Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, einer Cyanogruppe, einer Carbamoylgruppe, die mit einem oder mehreren Substituenten substituiert sein kann, ausgewählt aus einer Substituentengruppe $\beta_3$, einer $C_{1-6}$-Alkylgruppe, die mit einem oder mehreren Substituenten substituiert sein kann, ausgewählt aus der Substituentengruppe $\beta_3$, einer $C_{3-8}$-Cycloalkylgruppe, die mit einem oder mehreren Substituenten substituiert sein kann, ausgewählt aus der Substituentengruppe $\beta_3$, einer Arylgruppe, die mit einem oder mehreren Substituenten substituiert sein kann, ausgewählt aus der Substituentengruppe $\beta_3$, einer $C_{1-6}$-Alkoxygruppe, die mit einem oder mehreren Substituenten substituiert sein kann, ausgewählt aus der Substituentengruppe $\beta_3$, einer Aryloxygruppe, die mit einem oder mehreren Substituenten substituiert sein kann, ausgewählt aus der Substituentengruppe $\beta_3$, einer $C_{1-6}$-Alkylthiogruppe, die mit einem oder mehreren Substituenten substituiert sein kann, ausgewählt aus der Substituentengruppe $\beta_3$, einer Arylthiogruppe, die mit einem oder mehreren Substituenten substituiert sein kann, ausgewählt aus der Substituentengruppe $\beta_3$, einer $C_{1-6}$-Alkylaminogruppe, die mit einem oder mehreren Substituenten substituiert sein kann, ausgewählt aus der Substituentengruppe $\beta_3$, einer Di ($C_{1-6}$-alkyl) aminogruppe, die mit einem oder mehreren Substituenten substituiert sein kann, ausgewählt aus der Substituentengruppe $\beta_3$, einer $C_{1-6}$-Alkylsulfonylgruppe, die mit einem oder mehreren Substituenten substituiert sein kann, ausgewählt aus der Substituentengruppe $\beta_3$, einer Arylsulfonylgruppe, die mit einem oder mehreren Substituenten substituiert sein kann, ausgewählt aus der Substituentengruppe $\beta_3$, einer Acylgruppe, die mit einem oder mehreren Substituenten substituiert sein kann, ausgewählt aus der Substituentengruppe $\beta_3$, einer Heteroarylgruppe, die mit einem oder mehreren Substituenten substituiert sein kann, ausgewählt aus der Substituentengruppe $\beta_3$, einer Heterocyclylgruppe, die mit einem oder mehreren Substituenten substituiert sein kann, ausgewählt aus der Substituentengruppe $\beta_3$, einer Heterocyclyloxygruppe, die mit einem oder mehreren Substituenten substituiert sein kann, ausgewählt aus der Substituentengruppe $\beta_3$, und einer Oxogruppe besteht;

die Substituentengruppe $\beta_3$ aus einem Halogenatom, einer Hydroxylgruppe, die durch eine Schutzgruppe geschützt sein kann, ausgewählt aus einer $C_{1-6}$-Alkylgruppe, einer $C_{2-6}$-Alkenylgruppe, einer Ar-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonylgruppe, einer Silylgruppe, einer Tetrahydrofuranylgruppe und einer Tetrahydropyranylgruppe, einer Aminogruppe, die durch eine Schutzgruppe geschützt sein kann, ausgewählt aus einer Aryl-$C_{1-6}$-alkylgruppe, einer $C_{i-6}$-Alkoxy-$C_{i-6}$-alkylgruppe, einer Acylgruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, einer Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, einer Cyanogruppe, einer Carbamoylgruppe, die mit einem oder mehreren Substituenten substituiert sein kann, ausgewählt aus einer Substituentengruppe $\gamma_3$, einer $C_{1-6}$-Alkylgruppe, die mit einem oder mehreren Substituenten substituiert sein kann, ausgewählt aus der Substituentengruppe $\gamma_3$, einer $C_{3-8}$-Cycloalkylgruppe, die mit einem oder mehreren Substituenten substituiert sein kann, ausgewählt aus der Substituentengruppe $\gamma_3$, einer Arylgruppe, die mit einem oder mehreren Substituenten substituiert sein kann, ausgewählt aus der Substituentengruppe $\gamma_3$, einer $C_{1-6}$-Alkoxygruppe, die mit einem oder mehreren Substituenten substituiert sein kann, ausgewählt aus der Substituentengruppe $\gamma_3$, einer $C_{1-6}$-Alkylaminogruppe, die mit einem oder mehreren Substituenten

substituiert sein kann, ausgewählt aus der Substituentengruppe $\gamma_3$, einer Di($C_{1-6}$-alkyl)aminogruppe, die mit einem oder mehreren Substituenten substituiert sein kann, ausgewählt aus der Substituentengruppe $\gamma_3$, einer $C_{1-6}$-Alkylsulfonylgruppe, die mit einem oder mehreren Substituenten substituiert sein kann, ausgewählt aus der Substituentengruppe $\gamma_3$, einer Arylsulfonylgruppe, die mit einem oder mehreren Substituenten substituiert sein kann, ausgewählt aus der Substituentengruppe $\gamma_3$, einer Heteroarylgruppe, die mit einem oder mehreren Substituenten substituiert sein kann, ausgewählt aus der Substituentengruppe $\gamma_3$, einer Heterocyclylgruppe, die mit einem oder mehreren Substituenten substituiert sein kann, ausgewählt aus der Substituentengruppe $\gamma_3$, einer Heterocyclyloxygruppe, die mit einem oder mehreren Substituenten substituiert sein kann, ausgewählt aus der Substituentengruppe $\gamma_3$, und einer Oxogruppe besteht; und

die Substituentengruppe $\gamma_3$ aus einem Halogenatom, einer Hydroxylgruppe, die durch eine Schutzgruppe geschützt sein kann, ausgewählt aus einer $C_{1-6}$-Alkylgruppe, einer $C_{2-6}$-Alkenylgruppe, einer Ar-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonylgruppe, einer Silylgruppe, einer Tetrahydrofuranylgruppe und einer Tetrahydropyranylgruppe, einer Aminogruppe, die durch eine Schutzgruppe geschützt sein kann, ausgewählt aus einer Aryl-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, einer Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, einer Cyanogruppe, einer $C_{1-6}$-Alkylgruppe, die mit einem Halogenatom substituiert sein kann, einer $C_{1-6}$-Alkoxygruppe, die mit einem Halogenatom substituiert sein kann, und einer Oxogruppe besteht.

**7.** Stickstoffhaltige heterocyclische Verbindung, die durch die nachstehende Formel [1a] dargestellt ist, oder ein Salz davon:

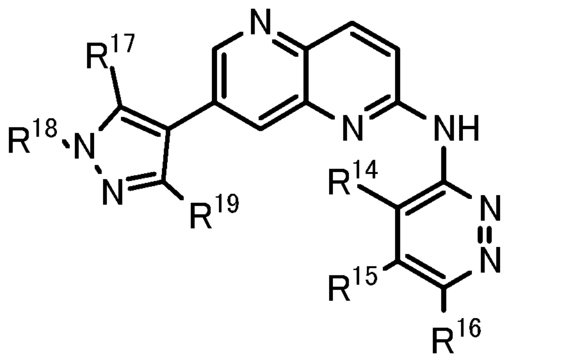

[1a]

worin in Formel [1a]:

jedes aus $R^{14}$, $R^{15}$ und $R^{16}$ unabhängig ein Wasserstoffatom, ein Halogenatom, eine Hydroxylgruppe, die durch eine Schutzgruppe geschützt sein kann, ausgewählt aus einer $C_{1-6}$-Alkylgruppe, einer $C_{2-6}$-Alkenylgruppe, einer Ar-$C_{1-6}$-alkylgruppe, einer $C_{i-6}$-Alkoxy-$C_{i-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonylgruppe, einer Silylgruppe, einer Tetrahydrofuranylgruppe und einer Tetrahydropyranylgruppe, eine Aminogruppe, die durch eine Schutzgruppe geschützt sein kann, ausgewählt aus einer Aryl-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, eine Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, eine Cyanogruppe, eine Carbamoylgruppe, die mit einem oder mehreren Substituenten substituiert sein kann, ausgewählt aus einer Substituentengruppe $B_1$, eine Sulfamoylgruppe, die mit einem oder mehreren Substituenten substituiert sein kann, ausgewählt aus der Substituentengruppe $B_1$, eine $C_{1-6}$-Alkylgruppe, die mit einem oder mehreren Substituenten substituiert sein kann, ausgewählt aus der Substituentengruppe $B_1$, eine $C_{2-6}$-Alkenylgruppe, die mit einem oder mehreren Substituenten substituiert sein kann, ausgewählt aus der Substituentengruppe $B_1$, eine $C_{3-8}$-Cycloalkylgruppe, die mit einem oder mehreren Substituenten substituiert sein kann, ausgewählt aus der Substituentengruppe $B_1$, eine Arylgruppe, die mit einem oder mehreren Substituenten substituiert sein kann, ausgewählt aus der Substituentengruppe $B_1$, eine $C_{1-6}$-Alkoxygruppe, die mit einem oder

mehreren Substituenten substituiert sein kann, ausgewählt aus der Substituentengruppe $B_1$, eine $C_{3-8}$-Cycloalkoxygruppe, die mit einem oder mehreren Substituenten substituiert sein kann, ausgewählt aus der Substituentengruppe $B_1$, eine Aryloxygruppe, die mit einem oder mehreren Substituenten substituiert sein kann, ausgewählt aus der Substituentengruppe $B_1$, eine $C_{1-6}$-Alkylthiogruppe, die mit einem oder mehreren Substituenten substituiert sein kann, ausgewählt aus der Substituentengruppe $B_1$, eine Arylthiogruppe, die mit einem oder mehreren Substituenten substituiert sein kann, ausgewählt aus der Substituentengruppe $B_1$, eine $C_{1-6}$-Alkylaminogruppe, die mit einem oder mehreren Substituenten substituiert sein kann, ausgewählt aus der Substituentengruppe $B_1$, eine Di($C_{1-6}$-alkyl)aminogruppe, die mit einem oder mehreren Substituenten substituiert sein kann, ausgewählt aus der Substituentengruppe $B_1$, eine Acylgruppe, die mit einem oder mehreren Substituenten substituiert sein kann, ausgewählt aus der Substituentengruppe $B_1$, eine Heteroarylgruppe, die mit einem oder mehreren Substituenten substituiert sein kann, ausgewählt aus der Substituentengruppe $B_1$, oder eine Heterocyclylgruppe, die mit einem oder mehreren Substituenten substituiert sein kann, ausgewählt aus der Substituentengruppe $B_1$, darstellt;

jedes aus $R^{17}$, $R^{18}$ und $R^{19}$ unabhängig ein Wasserstoffatom, ein Halogenatom, eine Hydroxylgruppe, die durch eine Schutzgruppe geschützt sein kann, ausgewählt aus einer $C_{1-6}$-Alkylgruppe, einer $C_{2-6}$-Alkenylgruppe, einer Ar-$C_{1-6}$-alkylgruppe, einer $C_{i-6}$-Alkoxy-$C_{i-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonylgruppe, einer Silylgruppe, einer Tetrahydrofuranylgruppe und einer Tetrahydropyranylgruppe, eine Aminogruppe, die durch eine Schutzgruppe geschützt sein kann, ausgewählt aus einer Aryl-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, eine Aminogruppe, die mit einem oder mehreren Substituenten substituiert sein kann, ausgewählt aus einer Substituentengruppe $E_3$, eine Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, eine Cyanogruppe, eine Carbamoylgruppe, die mit einem oder mehreren Substituenten substituiert sein kann, ausgewählt aus einer Substituentengruppe $B_3$, eine Sulfamoylgruppe, die mit einem oder mehreren Substituenten substituiert sein kann, ausgewählt aus der Substituentengruppe $B_3$, eine $C_{1-6}$-Alkylgruppe, die mit einem oder mehreren Substituenten substituiert sein kann, ausgewählt aus der Substituentengruppe $B_3$, eine $C_{2-6}$-Alkenylgruppe, die mit einem oder mehreren Substituenten substituiert sein kann, ausgewählt aus der Substituentengruppe $B_3$, eine $C_{3-8}$-Cycloalkylgruppe, die mit einem oder mehreren Substituenten substituiert sein kann, ausgewählt aus der Substituentengruppe $B_3$, eine Arylgruppe, die mit einem oder mehreren Substituenten substituiert sein kann, ausgewählt aus der Substituentengruppe $B_3$, eine $C_{1-6}$-Alkoxygruppe, die mit einem oder mehreren Substituenten substituiert sein kann, ausgewählt aus der Substituentengruppe $B_3$, eine Aryloxygruppe, die mit einem oder mehreren Substituenten substituiert sein kann, ausgewählt aus der Substituentengruppe $B_3$, eine $C_{1-6}$-Alkylthiogruppe, die mit einem oder mehreren Substituenten substituiert sein kann, ausgewählt aus der Substituentengruppe $B_3$, eine Arylthiogruppe, die mit einem oder mehreren Substituenten substituiert sein kann, ausgewählt aus der Substituentengruppe $B_3$, eine $C_{1-6}$-Alkylaminogruppe, die mit einem oder mehreren Substituenten substituiert sein kann, ausgewählt aus der Substituentengruppe $B_3$, eine Di($C_{1-6}$-alkyl)aminogruppe, die mit einem oder mehreren Substituenten substituiert sein kann, ausgewählt aus der Substituentengruppe $B_3$, eine $C_{1-6}$-Alkylsulfonylgruppe, die mit einem oder mehreren Substituenten substituiert sein kann, ausgewählt aus der Substituentengruppe $B_3$, eine Arylsulfonylgruppe, die mit einem oder mehreren Substituenten substituiert sein kann, ausgewählt aus der Substituentengruppe $B_3$, eine Acylgruppe, die mit einem oder mehreren Substituenten substituiert sein kann, ausgewählt aus der Substituentengruppe $B_3$, eine Heteroarylgruppe, die mit einem oder mehreren Substituenten substituiert sein kann, ausgewählt aus der Substituentengruppe $B_3$, eine Heterocyclylgruppe, die mit einem oder mehreren Substituenten substituiert sein kann, ausgewählt aus der Substituentengruppe $B_3$, oder eine Heterocyclyloxygruppe, die mit einem oder mehreren Substituenten substituiert sein kann, ausgewählt aus der Substituentengruppe $B_3$, darstellt;

die Substituentengruppe $B_1$ aus einem Halogenatom, einer Hydroxylgruppe, die durch eine Schutzgruppe geschützt sein kann, ausgewählt aus einer $C_{1-6}$-Alkylgruppe, einer $C_{2-6}$-Alkenylgruppe, einer Ar-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonylgruppe, einer Silylgruppe, einer Tetrahydrofuranylgruppe und einer Tetrahydropyranylgruppe, einer Aminogruppe, die durch eine Schutzgruppe geschützt sein kann, ausgewählt aus einer Aryl-$C_{1-6}$-alkylgruppe, einer $C_{i-6}$-Alkoxy-$C_{i-6}$-alkylgruppe, einer Acylgruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, einer Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, einer Cyanogruppe, einer Carbamoylgruppe, die mit einem oder mehreren Substituenten substituiert sein kann, ausgewählt aus einer Substituentengruppe $C_1$, einer Sulfamoylgruppe, die mit einem oder mehreren Substituenten substituiert sein kann, ausgewählt aus der Substituentengruppe $C_1$, einer $C_{1-6}$-Alkylgruppe, die mit einem oder mehreren Substituenten substituiert sein kann, ausgewählt aus der Substituentengruppe $C_1$, einer $C_{3-8}$-Cycloalkylgruppe, die mit einem oder mehreren Substituenten substituiert sein kann, ausgewählt aus der Substituentengruppe $C_1$, einer Arylgruppe, die mit einem oder mehreren Substituenten substituiert sein kann, ausgewählt aus der Substituentengruppe $C_1$, einer $C_{1-6}$-Alkoxygruppe, die mit einem oder mehreren Substituenten substituiert sein kann, ausgewählt aus der Substituentengruppe $C_1$, einer $C_{1-6}$-Alkylaminogruppe, die mit einem oder mehreren Substituenten substituiert sein kann, ausgewählt aus der Substituentengruppe $C_1$, einer Di($C_{1-6}$-alkyl)aminogruppe, die mit einem oder mehreren Substituenten substituiert sein kann, ausgewählt aus der Substituentengruppe $C_1$, einer $C_{1-6}$-Alkylsulfonylgruppe, die mit einem oder mehreren Substituenten substituiert sein kann, ausgewählt aus der Substituentengruppe $C_1$, einer Arylsulfonylgruppe, die mit einem oder mehreren Substituenten substituiert sein kann, ausgewählt aus der Substituentengruppe $C_1$, einer Acylgruppe, die mit einem oder mehreren Substituenten substituiert sein kann, ausgewählt aus der Substituentengruppe $C_1$, einer Heteroarylgruppe, die mit einem oder mehreren Substituenten substituiert sein kann, ausgewählt aus der Substituentengruppe $C_1$, einer Heterocyclylgruppe, die mit einem oder mehreren Substituenten substituiert sein kann, ausgewählt aus der Substituentengruppe $C_1$, und einer Oxogruppe besteht;

die Substituentengruppe $C_1$ aus einem Halogenatom, einer Hydroxylgruppe, die durch eine Schutzgruppe geschützt sein kann, ausgewählt aus einer $C_{1-6}$-Alkylgruppe, einer $C_{2-6}$-Alkenylgruppe, einer Ar-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonylgruppe, einer Silylgruppe, einer Tetrahydrofuranylgruppe und einer Tetrahydropyranylgruppe, einer Aminogruppe, die durch eine Schutzgruppe geschützt sein kann, ausgewählt aus einer Aryl-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, einer Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, einer Cyanogruppe, einer Carbamoylgruppe, die mit einer $C_{1-6}$-Alkylgruppe substituiert sein kann, einer $C_{1-6}$-Alkylgruppe, die mit einem Halogenatom substituiert sein kann, einer Arylgruppe, die mit einem Halogenatom oder einer $C_{1-6}$-Alkylgruppe substituiert sein kann, einer $C_{1-6}$-Alkoxygruppe, die mit einem Halogenatom substituiert sein kann, und einer Oxogruppe besteht;

die Substituentengruppe $B_3$ aus einem Halogenatom, einer Hydroxylgruppe, einer Aminogruppe, einer Carboxylgruppe, einer Cyanogruppe, einer Carbamoylgruppe, die mit einem oder mehreren Substituenten substituiert sein kann, ausgewählt aus einer Substituentengruppe $C_3$, einer $C_{1-6}$-Alkylgruppe, die mit einem oder mehreren Substituenten substituiert sein kann, ausgewählt aus der Substituentengruppe $C_3$, einer $C_{3-8}$-Cycloalkylgruppe, die mit einem oder mehreren Substituenten substituiert sein kann, ausgewählt aus der Substituentengruppe $C_3$, einer Arylgruppe, die mit einem oder mehreren Substituenten substituiert sein kann, ausgewählt aus der Substituentengruppe $C_3$, einer $C_{1-6}$-Alkoxygruppe, die mit einem oder mehreren Substituenten substituiert sein kann, ausgewählt aus der Substituentengruppe $C_3$, einer Aryloxygruppe, die mit einem oder mehreren Substituenten substituiert sein kann, ausgewählt aus der Substituentengruppe $C_3$, einer $C_{1-6}$-Alkylaminogruppe, die mit einem oder mehreren Substituenten substituiert sein kann, ausgewählt aus der Substituentengruppe $C_3$, einer Di($C_{1-6}$-alkyl)aminogruppe, die mit einem oder mehreren Substituenten substituiert sein kann, ausgewählt aus der Substituentengruppe $C_3$, einer $C_{1-6}$-Alkylsulfonylgruppe, die mit einem oder mehreren Substituenten substituiert sein kann, ausgewählt aus der Substituentengruppe $C_3$, einer Arylsulfonylgruppe, die mit einem oder mehreren Substituenten substituiert sein kann, ausgewählt aus der Substituentengruppe $C_3$, einer Acylgruppe, die mit einem oder mehreren Substituenten substituiert sein kann, ausgewählt aus der Substituentengruppe $C_3$, einer Heteroarylgruppe, die mit einem oder mehreren Substituenten substituiert sein kann, ausgewählt aus der Substituentengruppe $C_3$, einer Heterocyclylgruppe, die mit einem oder mehreren Substituenten substituiert sein kann, ausgewählt aus der Substituentengruppe $C_3$, einer Heteroaryloxygruppe, die mit einem oder mehreren Substituenten substituiert sein kann, ausgewählt aus der Substituentengruppe $C_3$, einer Heterocyclyloxygruppe, die mit einem oder mehreren Substituenten substituiert sein kann, ausgewählt aus der Substituentengruppe $C_3$, und einer Oxogruppe besteht;

die Substituentengruppe $C_3$ aus einem Halogenatom, einer Hydroxylgruppe, die durch eine Schutzgruppe geschützt sein kann, ausgewählt aus einer $C_{1-6}$-Alkylgruppe, einer $C_{2-6}$-Alkenylgruppe, einer Ar-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonylgruppe, einer Silylgruppe, einer Tetrahydrofuranylgruppe und einer Tetrahydropyranylgruppe, einer Aminogruppe, die durch eine Schutzgruppe geschützt sein kann, ausgewählt aus einer Aryl-$C_{1-6}$-alkylgruppe, einer $C_{i-6}$-Alkoxy-$C_{i-6}$-alkylgruppe, einer Acyl-

gruppe, einer $C_{1-6}$-Alkoxycarbonylgruppe, einer Ar-$C_{1-6}$-alkoxycarbonylgruppe, einer Aryloxycarbonylgruppe, einer $C_{1-6}$-Alkylsulfonylgruppe, einer Arylsulfonylgruppe und einer Silylgruppe, einer Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, einer Nitrogruppe, einer Cyanogruppe, einer Carbamoylgruppe, die mit einem oder mehreren Substituenten substituiert sein kann, ausgewählt aus einer Substituentengruppe $D_3$, einer $C_{1-6}$-Alkylgruppe, die mit einem oder mehreren Substituenten substituiert sein kann, ausgewählt aus der Substituentengruppe $D_3$, einer $C_{3-8}$-Cycloalkylgruppe, die mit einem oder mehreren Substituenten substituiert sein kann, ausgewählt aus der Substituentengruppe $D_3$, einer Arylgruppe, die mit einem oder mehreren Substituenten substituiert sein kann, ausgewählt aus der Substituentengruppe $D_3$, einer $C_{1-6}$-Alkoxygruppe, die mit einem oder mehreren Substituenten substituiert sein kann, ausgewählt aus der Substituentengruppe $D_3$, einer Aryloxygruppe, die mit einem oder mehreren Substituenten substituiert sein kann, ausgewählt aus der Substituentengruppe $D_3$, einer $C_{1-6}$-Alkylaminogruppe, die mit einem oder mehreren Substituenten substituiert sein kann, ausgewählt aus der Substituentengruppe $D_3$, einer Di($C_{1-6}$-alkyl)aminogruppe, die mit einem oder mehreren Substituenten substituiert sein kann, ausgewählt aus der Substituentengruppe $D_3$, einer $C_{1-6}$-Alkylsulfonylgruppe, die mit einem oder mehreren Substituenten substituiert sein kann, ausgewählt aus der Substituentengruppe $D_3$, einer Acylgruppe, die mit einem oder mehreren Substituenten substituiert sein kann, ausgewählt aus der Substituentengruppe $D_3$, einer Heteroarylgruppe, die mit einem oder mehreren Substituenten substituiert sein kann, ausgewählt aus der Substituentengruppe $D_3$, einer Heterocyclylgruppe, die mit einem oder mehreren Substituenten substituiert sein kann, ausgewählt aus der Substituentengruppe $D_3$, einer Silylgruppe, die mit einem oder mehreren Substituenten substituiert sein kann, ausgewählt aus der Substituentengruppe $D_3$, und einer Oxogruppe besteht;

die Substituentengruppe $D_3$ aus einem Halogenatom, einer Hydroxylgruppe, die durch eine Schutzgruppe geschützt sein kann, ausgewählt aus einer $C_{1-6}$-Alkylgruppe, einer $C_{2-6}$-Alkenylgruppe, einer Ar-$C_{1-6}$-alkylgruppe, einer $C_{1-6}$-Alkoxy-$C_{1-6}$-alkylgruppe, einer Acylgruppe, einer $C_{1-6}$-Alkoxycarbonylgruppe, einer Ar-$C_{1-6}$-alkoxycarbonylgruppe, einer $C_{1-6}$-Alkylsulfonylgruppe, einer Arylsulfonylgruppe, einer Silylgruppe, einer Tetrahydrofuranylgruppe und einer Tetrahydropyranylgruppe, einer $C_{1-6}$-Alkylgruppe, einer $C_{3-8}$-Cycloalkylgruppe, einer Arylgruppe, einer $C_{1-6}$-Alkoxygruppe, einer $C_{1-6}$-Alkylaminogruppe, einer Di($C_{1-6}$-alkyl)aminogruppe, einer $C_{1-6}$-Alkylsulfonylgruppe, einer Heteroarylgruppe, einer Heterocyclylgruppe und einer Oxogruppe besteht;

die Substituentengruppe $E_3$ aus einer $C_{3-8}$-Cycloalkylgruppe, die mit einem oder mehreren Substituenten substituiert sein kann, ausgewählt aus einer Substituentengruppe $F_3$, einer Arylgruppe, die mit einem oder mehreren Substituenten substituiert sein kann, ausgewählt aus der Substituentengruppe $F_3$, einer Heteroarylgruppe, die mit einem oder mehreren Substituenten substituiert sein kann, ausgewählt aus der Substituentengruppe $F_3$, und einer Heterocyclylgruppe, die mit einem oder mehreren Substituenten substituiert sein kann, ausgewählt aus der Substituentengruppe $F_3$, besteht; und

die Substituentengruppe $F_3$ aus einem Halogenatom, einer Carboxylgruppe, die durch eine Schutzgruppe geschützt sein kann, ausgewählt aus einer $C_{1-6}$-Alkylgruppe, einer $C_{2-6}$-Alkenylgruppe, einer Arylgruppe, einer Aryl-$C_{1-6}$-alkylgruppe, einer $C_{1-6}$-Alkoxy-$C_{1-6}$-alkylgruppe und einer Silylgruppe, und einer $C_{1-6}$-Alkylgruppe besteht.

**8.** Stickstoffhaltige heterocyclische Verbindung oder Salz davon gemäß Anspruch 7, worin jedes aus $R^{14}$, $R^{16}$ und $R^{17}$ ein Wasserstoffatom darstellt.

**9.** Stickstoffhaltige heterocyclische Verbindung oder Salz davon gemäß Anspruch 1, die Verbindung eine der nachstehend aufgeführten Verbindungen ist:

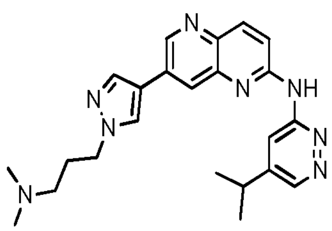
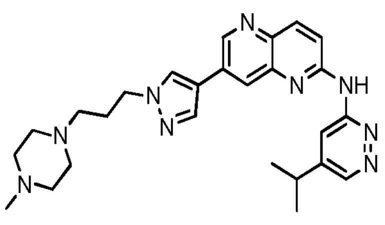
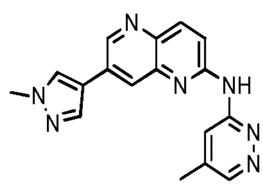

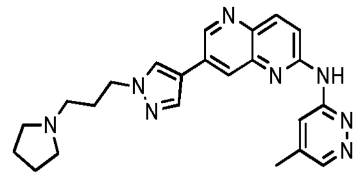
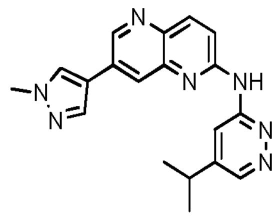
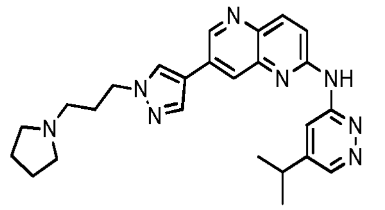

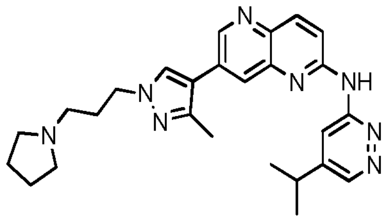
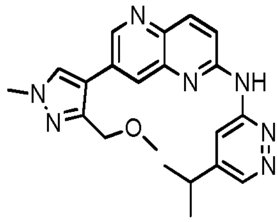
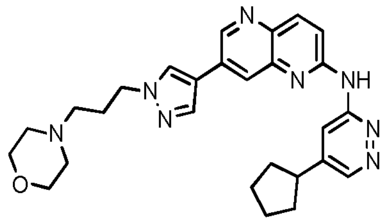

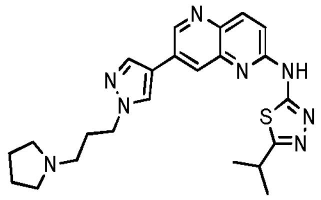
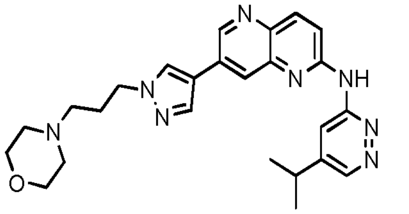
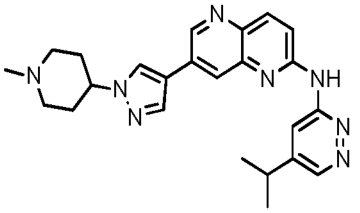

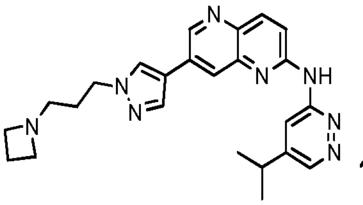
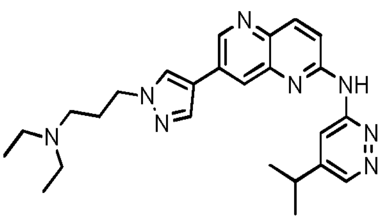
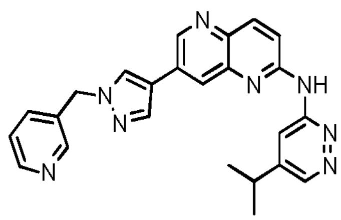

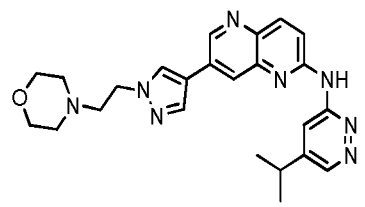
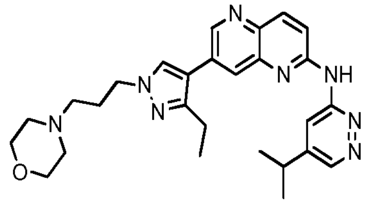
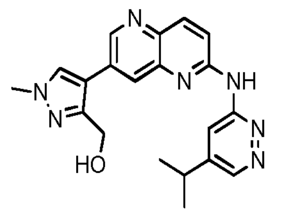

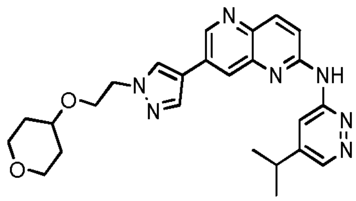
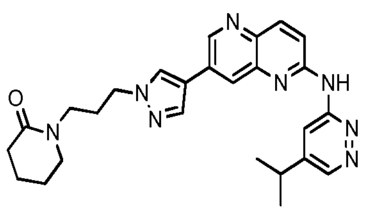
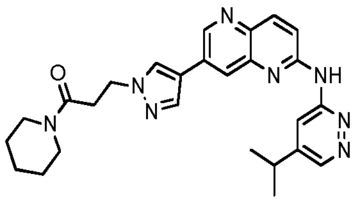

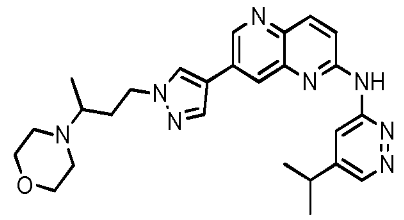
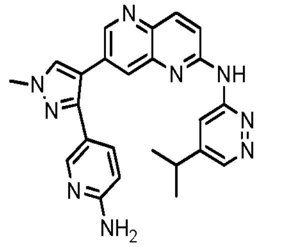
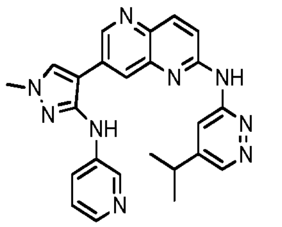
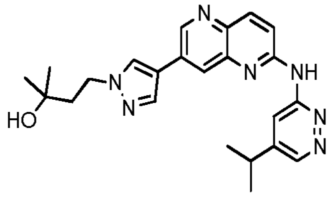
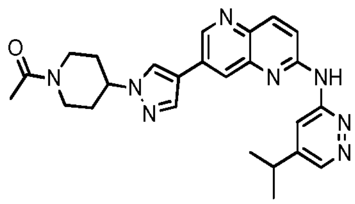
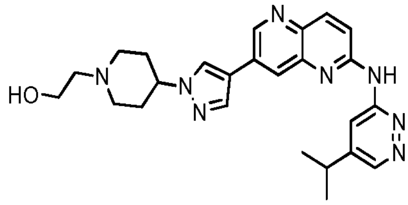
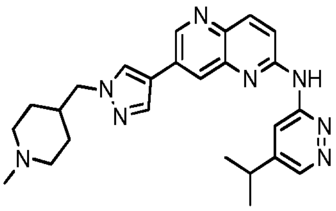
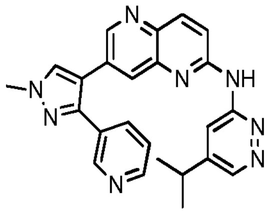
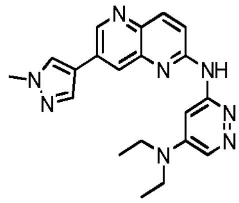
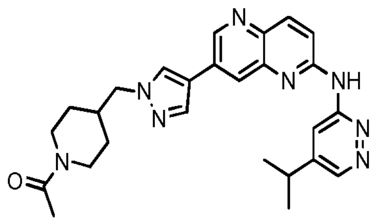
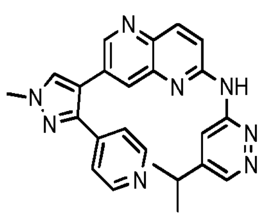
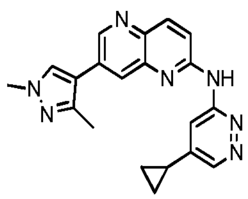
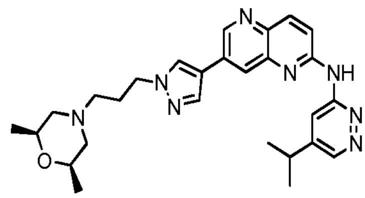
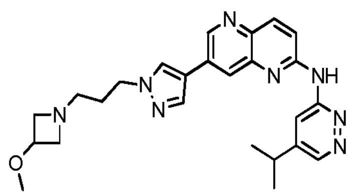
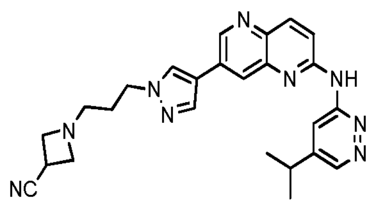
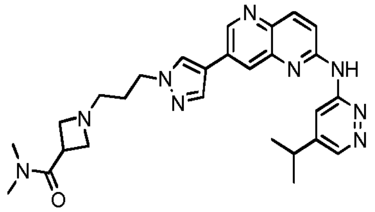
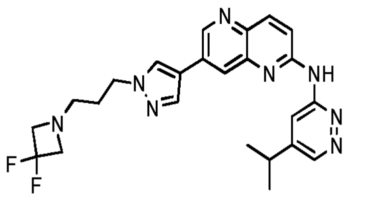
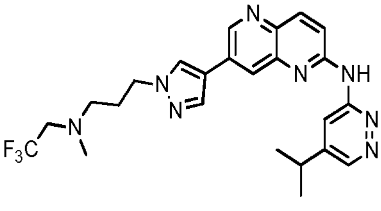
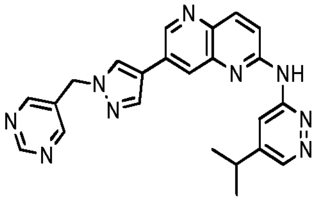
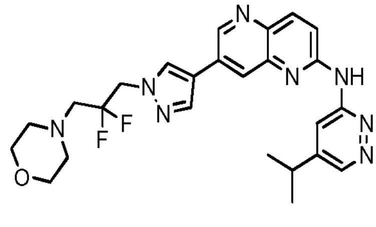

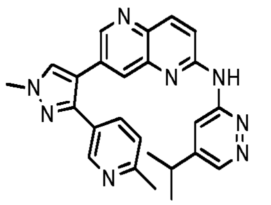
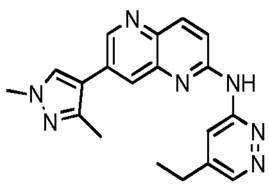
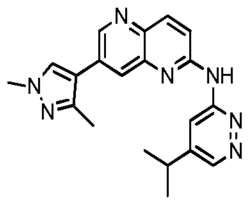

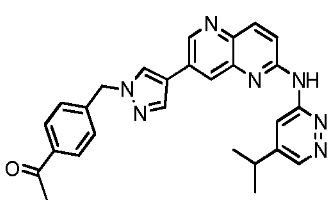
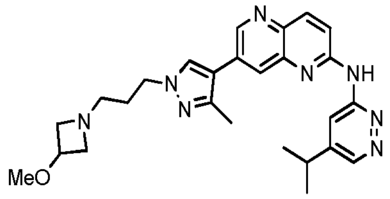
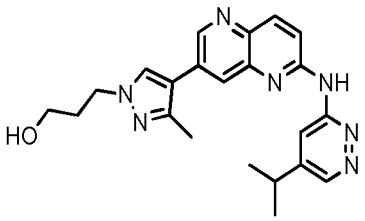

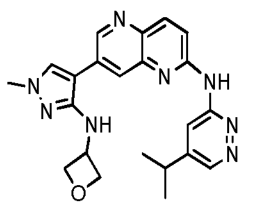
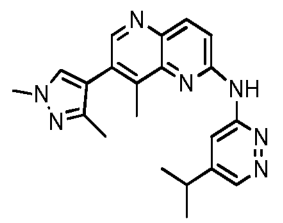

**10.** Pharmazeutische Zusammensetzung, umfassend die stickstoffhaltige heterocyclische Verbindung oder ein Salz davon gemäß mindestens einem der Ansprüche 1 bis 9.

**11.** Stickstoffhaltige heterocyclische Verbindung oder ein Salz davon gemäß mindestens einem der Ansprüche 1 bis 9 oder pharmazeutische Zusammensetzung gemäß Anspruch 10 zur Verwendung bei der Behandlung einer Erkrankung.

**12.** Stickstoffhaltige heterocyclische Verbindung oder ein Salz davon gemäß mindestens einem der Ansprüche 1 bis 9 oder pharmazeutische Zusammensetzung gemäß Anspruch 10 zur Verwendung bei der Behandlung einer Erkrankung, worin die Krankheit vorzugsweise ausgewählt ist aus der Gruppe, bestehend aus einem bösartigen Tumor, einer zellproliferativen Erkrankung, einer allergischen Erkrankung, einer Autoimmunerkrankung, einer neurodegenerativen Erkrankung, einer Kreislauferkrankung, einer entzündlichen Erkrankung, einer endokrinen Erkrankung, einer Stoffwechselstörung und einer Infektion.

**Revendications**

**1.** Composé hétérocyclique azoté représenté par la Formule [1] suivante ou un sel de celui-ci :

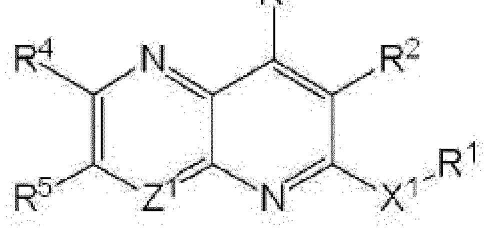

[1]

dans laquelle, dans la Formule [1] :

$Z^1$ représente $CR^6$ ;
$R^6$ représente un atome d'hydrogène, un atome d'halogène, un groupe hydroxyle, un groupe alkyle en $C_1$ à

$C_6$, un groupe alcényle en $C_2$ à $C_6$, un groupe alcynyle en $C_2$ à $C_6$, un groupe cycloalkyle en $C_3$ à $C_8$, un groupe aryle qui peut être substitué avec un ou plusieurs substituants sélectionnés dans un groupe $\alpha_2$ de substituants, un groupe alcoxy en $C_1$ à $C_6$, un groupe aryloxy, un groupe alkylthio en $C_1$ à $C_6$, un groupe arylthio, un groupe hétéroaryle qui peut être substitué avec un ou plusieurs substituants sélectionnés dans le groupe $\alpha_2$ de substituants, un groupe hétéroaryloxy, un groupe hétéroarylthio, un groupe hétérocyclyle qui peut être substitué avec un ou plusieurs substituants sélectionnés dans le groupe $\alpha_2$ de substituants, ou $NR^7R^8$ ;

chacun parmi $R^7$ et $R^8$ représente indépendamment un atome d'hydrogène, un groupe alkyle en $C_1$ à $C_6$, un groupe alcényle en $C_2$ à $C_6$, un groupe alcynyle en $C_2$ à $C_6$, un groupe cycloalkyle en $C_3$ à $C_8$, un groupe aryle qui peut être substitué avec un ou plusieurs substituants sélectionnés dans le groupe $\alpha_2$ de substituants, un groupe hétéroaryle qui peut être substitué avec un ou plusieurs substituants sélectionnés dans le groupe $\alpha_2$ de substituants, ou un groupe hétérocyclyle qui peut être substitué avec un ou plusieurs substituants sélectionnés dans le groupe $\alpha_2$ de substituants ;

le groupe $\alpha_2$ de substituants consiste en un atome d'halogène, un groupe hydroxyle qui peut être protégé par un groupe protecteur sélectionné parmi un groupe alkyle en $C_1$ à $C_6$, un groupe alcényle en $C_2$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe amino qui peut être protégé par un groupe protecteur sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe aryloxycarbonyle, un groupe alkylsulfonyle en $C_1$ à $C_6$, un groupe arylsulfonyle, et un groupe silyle, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, un groupe cyano, un groupe carbamoyle qui peut être substitué avec un ou plusieurs substituants sélectionnés dans un groupe $\beta_2$ de substituants, un groupe alkyle en $C_1$ à $C_6$ qui peut être substitué avec un ou plusieurs substituants sélectionnés dans le groupe $\beta_2$ de substituants, un groupe alcoxy en $C_1$ à $C_6$ qui peut être substitué avec un ou plusieurs substituants sélectionnés dans le groupe $\beta_2$ de substituants, et un groupe oxo ;

le groupe $\beta_2$ de substituants consiste en un atome d'halogène, un groupe alkyle en $C_1$ à $C_6$ qui peut être substitué avec un ou plusieurs substituants sélectionnés dans un groupe $\gamma_2$ de substituants, un groupe cycloalkyle en $C_3$ à $C_8$ qui peut être substitué avec un ou plusieurs substituants sélectionnés dans le groupe $\gamma_2$ de substituants, un groupe aryle qui peut être substitué avec un ou plusieurs substituants sélectionnés dans le groupe $\gamma_2$ de substituants, un groupe hétéroaryle qui peut être substitué avec un ou plusieurs substituants sélectionnés dans le groupe $\gamma_2$ de substituants, et un groupe hétérocyclyle qui peut être substitué avec un ou plusieurs substituants sélectionnés dans le groupe $\gamma_2$ de substituants ; et

le groupe $\gamma_2$ de substituants consiste en un atome d'halogène, un groupe hydroxyle qui peut être protégé par un groupe protecteur sélectionné parmi un groupe alkyle en $C_1$ à $C_6$, un groupe alcényle en $C_2$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe amino qui peut être protégé par un groupe protecteur sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe aryloxycarbonyle, un groupe alkylsulfonyle en $C_1$ à $C_6$, un groupe arylsulfonyle, et un groupe silyle, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, un groupe cyano, un groupe alkyle en $C_1$ à $C_6$ qui peut être substitué avec un atome d'halogène, un groupe alcoxy en $C_1$ à $C_6$ qui peut être substitué avec un atome d'halogène, et un groupe oxo ;

$X^1$ représente $NR^9$ ;

$R^9$ représente un atome d'hydrogène ;

$R^1$ représente un groupe hétéroaryle azoté monocyclique qui peut être substitué avec un ou plusieurs substituants sélectionnés dans un groupe $A_1$ de substituants, un groupe hétéroaryle monocyclique oxygéné et azoté qui peut être substitué avec un ou plusieurs substituants sélectionnés dans le groupe $A_1$ de substituants, un groupe hétéroaryle monocyclique azoté et soufré qui peut être substitué avec un ou plusieurs substituants sélectionnés dans un groupe $A_1$ de substituants, un groupe hétéroaryle bicyclique azoté qui peut être substitué avec un ou plusieurs substituants sélectionnés dans le groupe $A_1$ de substituants, un groupe hétéroaryle bicyclique oxygéné et azoté qui peut être substitué avec un ou plusieurs substituants sélectionnés dans un groupe $A_1$ de substituants, ou un groupe hétéroaryle bicyclique azoté et soufré qui peut être substitué avec un ou plusieurs substituants sélectionnés dans le groupe $A_1$ de substituants ;

le groupe $A_1$ de substituants consiste en un atome d'halogène, un groupe hydroxyle qui peut être protégé par un groupe protecteur sélectionné parmi un groupe alkyle en $C_1$ à $C_6$, un groupe alcényle en $C_2$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe amino qui peut être protégé par un groupe protecteur sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe aryloxycarbonyle, un groupe alkylsulfonyle en $C_1$ à $C_6$, un groupe arylsulfonyle, et un groupe silyle, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, un groupe cyano, un groupe carbamoyle qui peut être substitué avec un ou plusieurs substituants sélectionnés dans un groupe $B_1$ de substituants, un groupe sulfamoyle qui peut être substitué avec un ou plusieurs substituants sélectionnés dans le groupe $B_1$ de substituants, un groupe alkyle en $C_1$ à $C_6$ qui peut être substitué avec un ou plusieurs substituants sélectionnés dans le groupe $B_1$ de substituants, un groupe alcényle en $C_2$ à $C_6$ qui peut être substitué avec un ou plusieurs substituants sélectionnés dans le groupe $B_1$ de substituants, un groupe cycloalkyle en $C_3$ à $C_8$ qui peut être substitué avec un ou plusieurs substituants sélectionnés dans le groupe $B_1$ de substituants, un groupe aryle qui peut être substitué avec un ou plusieurs substituants sélectionnés dans le groupe $B_1$ de substituants, un groupe alcoxy en $C_1$ à $C_6$ qui peut être substitué avec un ou plusieurs substituants sélectionnés dans le groupe $B_1$ de substituants, un groupe cycloalcoxy en $C_3$ à $C_8$ qui peut être substitué avec un ou plusieurs substituants sélectionnés dans le groupe $B_1$ de substituants, un groupe aryloxy qui peut être substitué avec un ou plusieurs substituants sélectionnés dans le groupe $B_1$ de substituants, un groupe alkylthio en $C_1$ à $C_6$ qui peut être substitué avec un ou plusieurs substituants sélectionnés dans le groupe $B_1$ de substituants, un groupe arylthio qui peut être substitué avec un ou plusieurs substituants sélectionnés dans le groupe $B_1$ de substituants, un groupe alkylamino en $C_1$ à $C_6$ qui peut être substitué avec un ou plusieurs substituants sélectionnés dans le groupe $B_1$ de substituants, un groupe di(alkyle en $C_1$ à $C_6$)amino qui peut être substitué avec un ou plusieurs substituants sélectionnés dans le groupe $B_1$ de substituants, un groupe acyle qui peut être substitué avec un ou plusieurs substituants sélectionnés dans le groupe $B_1$ de substituants, un groupe hétéroaryle qui peut être substitué avec un ou plusieurs substituants sélectionnés dans un groupe $B_1$ de substituants, un groupe hétérocyclyle qui peut être substitué avec un ou plusieurs substituants sélectionnés dans le groupe $B_1$ de substituants, et un groupe oxo ;

le groupe $B_1$ de substituants consiste en un atome d'halogène, un groupe hydroxyle qui peut être protégé par un groupe protecteur sélectionné parmi un groupe alkyle en $C_1$ à $C_6$, un groupe alcényle en $C_2$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe amino qui peut être protégé par un groupe protecteur sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe aryloxycarbonyle, un groupe alkylsulfonyle en $C_1$ à $C_6$, un groupe arylsulfonyle, et un groupe silyle, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, un groupe cyano, un groupe carbamoyle qui peut être substitué avec un ou plusieurs substituants sélectionnés dans un groupe $C_1$ de substituants, un groupe sulfamoyle qui peut être substitué avec un ou plusieurs substituants sélectionnés dans le groupe $C_1$ de substituants, un groupe alkyle en $C_1$ à $C_6$ qui peut être substitué avec un ou plusieurs substituants sélectionnés dans le groupe $C_1$ de substituants, un groupe cycloalkyle en $C_3$ à $C_8$ qui peut être substitué avec un ou plusieurs substituants sélectionnés dans le groupe $C_1$ de substituants, un groupe aryle qui peut être substitué avec un ou plusieurs substituants sélectionnés dans le groupe $C_1$ de substituants, un groupe alcoxy en $C_1$ à $C_6$ qui peut être substitué avec un ou plusieurs substituants sélectionnés dans le groupe $C_1$ de substituants, un groupe alkylamino en $C_1$ à $C_6$ qui peut être substitué avec un ou plusieurs substituants sélectionnés dans le groupe $C_1$ de substituants, un groupe di(alkyle en $C_1$ à $C_6$)amino qui peut être substitué avec un ou plusieurs substituants sélectionnés dans le groupe $C_1$ de substituants, un groupe alkylsulfonyle en $C_1$ à $C_6$ qui peut être substitué avec un ou plusieurs substituants sélectionnés dans le groupe $C_1$ de substituants, un groupe arylsulfonyle qui peut être substitué avec un ou plusieurs substituants sélectionnés dans le groupe $C_1$ de substituants, un groupe acyle qui peut être substitué avec un ou plusieurs substituants sélectionnés dans le groupe $C_1$ de substituants, un groupe hétéroaryle qui peut être substitué avec un ou plusieurs substituants sélectionnés dans le groupe $C_1$ de substituants, un groupe hétérocyclyle qui peut être substitué avec un ou plusieurs substituants sélectionnés dans le groupe $C_1$ de substituants, et un groupe oxo ; et

le groupe $C_1$ de substituants consiste en un atome d'halogène, un groupe hydroxyle qui peut être protégé par un groupe protecteur sélectionné parmi un groupe alkyle en $C_1$ à $C_6$, un groupe alcényle en $C_2$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe amino qui peut être protégé par un groupe protecteur sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe aryloxycarbonyle, un groupe alkylsulfonyle en $C_1$ à $C_6$, un groupe arylsulfonyle, et un groupe silyle, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, un groupe cyano, un groupe carbamoyle qui peut être substitué avec un groupe alkyle en $C_1$ à $C_6$, un groupe alkyle en $C_1$ à $C_6$ qui peut être substitué avec un atome d'halogène, un groupe aryle qui peut être substitué avec un atome d'halogène ou un groupe alkyle en $C_1$ à $C_6$, un groupe alcoxy en $C_1$ à $C_6$ qui peut être substitué avec un atome d'halogène, et un groupe oxo ;

$R^2$ représente un atome d'hydrogène ;

$R^3$ représente un atome d'hydrogène ;

$R^4$ représente un atome d'hydrogène ;

$R^5$ représente un groupe hétéroaryle qui peut être substitué avec un ou plusieurs substituants sélectionnés dans le groupe $A_3$ de substituants, un groupe hétéroaryloxy qui peut être substitué avec un ou plusieurs substituants sélectionnés dans le groupe $A_3$ de substituants, un groupe hétéroarylthio qui peut être substitué avec un ou plusieurs substituants sélectionnés dans le groupe $A_3$ de substituants, un groupe hétérocyclyle qui peut être substitué avec un ou plusieurs substituants sélectionnés dans le groupe $A_3$ de substituants, ou $NR^{12}R^{13}$ ;

chacun parmi $R^{12}$ et $R^{13}$ représente indépendamment un atome d'hydrogène, un groupe alkyle en $C_1$ à $C_6$ qui peut être substitué avec un ou plusieurs substituants sélectionnés dans le groupe $A_3$ de substituants, un groupe alcényle en $C_2$ à $C_6$ qui peut être substitué avec un ou plusieurs substituants sélectionnés dans le groupe $A_3$ de substituants, un groupe alcynyle en $C_2$ à $C_6$ qui peut être substitué avec un ou plusieurs substituants sélectionnés dans le groupe $A_3$ de substituants, un groupe cycloalkyle en $C_3$ à Cg qui peut être substitué avec un ou plusieurs substituants sélectionnés dans le groupe $A_3$ de substituants, un groupe aryle qui peut être substitué avec un ou plusieurs substituants sélectionnés dans le groupe $A_3$ de substituants, un groupe hétéroaryle qui peut être substitué avec un ou plusieurs substituants sélectionnés dans le groupe $A_3$ de substituants, un groupe hétérocyclyle qui peut être substitué avec un ou plusieurs substituants sélectionnés dans le groupe $A_3$ de substituants, ou un groupe protecteur d'amino sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe aryloxycarbonyle, un groupe alkylsulfonyle en $C_1$ à $C_6$, un groupe arylsulfonyle, et un groupe silyle ;

le groupe $A_3$ de substituants consiste en un atome d'halogène, un groupe hydroxyle, un groupe amino, un groupe amino qui peut être substitué avec un ou plusieurs substituants sélectionnés dans un groupe $E_3$ de substituants, un groupe carboxyle, un groupe cyano, un groupe carbamoyle qui peut être substitué avec un ou plusieurs substituants sélectionnés parmi un groupe $B_3$ de substituants, un groupe sulfamoyle qui peut être substitué avec un ou plusieurs substituants sélectionnés dans le groupe $B_3$ de substituants, un groupe alkyle en $C_1$ à $C_6$ qui peut être substitué avec un ou plusieurs substituants sélectionnés dans le groupe $B_3$ de substituants, un groupe alcényle en $C_2$ à $C_6$ qui peut être substitué avec un ou plusieurs substituants sélectionnés dans le groupe $B_3$ de substituants, un groupe cycloalkyle en $C_3$ à Cg qui peut être substitué avec un ou plusieurs substituants sélectionnés dans le groupe $B_3$ de substituants, un groupe aryle qui peut être substitué avec un ou plusieurs substituants sélectionnés dans le groupe $B_3$ de substituants, un groupe alcoxy en $C_1$ à $C_6$ qui peut être substitué avec un ou plusieurs substituants sélectionnés dans le groupe $B_3$ de substituants, un groupe aryloxy qui peut être substitué avec un ou plusieurs substituants sélectionnés dans le groupe $B_3$ de substituants, un groupe alkylthio en $C_1$ à $C_6$ qui peut être substitué avec un ou plusieurs substituants sélectionnés dans le groupe $B_3$ de substituants, un groupe arylthio qui peut être substitué avec un ou plusieurs substituants sélectionnés dans le groupe $B_3$ de substituants, un groupe alkylamino en $C_1$ à $C_6$ qui peut être substitué avec un ou plusieurs substituants sélectionnés dans le groupe $B_3$ de substituants, un groupe di(alkyle en $C_1$ à $C_6$)amino qui peut être substitué avec un ou plusieurs substituants sélectionnés dans le groupe $B_3$ de substituants, un groupe alkylsulfonyle en $C_1$ à $C_6$ qui peut être substitué avec un ou plusieurs substituants sélectionnés dans le groupe $B_3$ de substituants, un groupe arylsulfonyle qui peut être substitué avec un ou plusieurs substituants sélectionnés dans le groupe $B_3$ de substituants, un groupe acyle qui peut être substitué avec un ou plusieurs substituants sélectionnés dans le groupe $B_3$ de substituants, un groupe hétéroaryle qui peut être substitué avec un ou plusieurs substituants sélectionnés dans le groupe $B_3$ de substituants, un groupe hétérocyclyle qui peut

être substitué avec un ou plusieurs substituants sélectionnés dans le groupe $B_3$ de substituants, un groupe hétérocyclyloxy qui peut être substitué avec un ou plusieurs substituants sélectionnés dans le groupe $B_3$ de substituants, et un groupe oxo ;

le groupe $B_3$ de substituants consiste en un atome d'halogène, un groupe hydroxyle qui peut être protégé par un groupe protecteur sélectionné parmi un groupe alkyle en $C_1$ à $C_6$, un groupe alcényle en $C_1$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe amino qui peut être protégé par un groupe protecteur sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe aryloxycarbonyle, un groupe alkylsulfonyle en $C_1$ à $C_6$, un groupe arylsulfonyle, et un groupe silyle, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, un groupe cyano, un groupe carbamoyle qui peut être substitué avec un ou plusieurs substituants sélectionnés dans un groupe $C_3$ de substituants, un groupe alkyle en $C_1$ à $C_6$ qui peut être substitué avec un ou plusieurs substituants sélectionnés dans le groupe $C_3$ de substituants, un groupe cycloalkyle en $C_3$ à Cg qui peut être substitué avec un ou plusieurs substituants sélectionnés dans le groupe $C_3$ de substituants, un groupe aryle qui peut être substitué avec un ou plusieurs substituants sélectionnés dans le groupe $C_3$ de substituants, un groupe alcoxy en $C_1$ à $C_6$ qui peut être substitué avec un ou plusieurs substituants sélectionnés dans le groupe $C_3$ de substituants, un groupe aryloxy qui peut être substitué avec un ou plusieurs substituants sélectionnés dans le groupe $C_3$ de substituants, un groupe alkylamino en $C_1$ à $C_6$ qui peut être substitué avec un ou plusieurs substituants sélectionnés dans le groupe $C_3$ de substituants, un groupe di(alkyle en $C_1$ à $C_6$)amino qui peut être substitué avec un ou plusieurs substituants sélectionnés dans le groupe $C_3$ de substituants, un groupe alkylsulfonyle en $C_1$ à $C_6$ qui peut être substitué avec un ou plusieurs substituants sélectionnés dans le groupe $C_3$ de substituants, un groupe arylsulfonyle qui peut être substitué avec un ou plusieurs substituants sélectionnés dans le groupe $C_3$ de substituants, un groupe acyle qui peut être substitué avec un ou plusieurs substituants sélectionnés dans le groupe $C_3$ de substituants, un groupe hétéroaryle qui peut être substitué avec un ou plusieurs substituants sélectionnés dans un groupe $C_3$ de substituants, un groupe hétérocyclyle qui peut être substitué avec un ou plusieurs substituants sélectionnés dans le groupe $C_3$ de substituants, un groupe hétéroaryloxy qui peut être substitué avec un ou plusieurs substituants sélectionnés dans un groupe $C_3$ de substituants, un groupe hétérocyclyloxy qui peut être substitué avec un ou plusieurs substituants sélectionnés dans le groupe $C_3$ de substituants, et un groupe oxo ;

le groupe $C_3$ de substituants consiste en un atome d'halogène, un groupe hydroxyle, un groupe amino, un groupe carboxyle, un groupe nitro, un groupe cyano, un groupe carbamoyle qui peut être substitué avec un ou plusieurs substituants sélectionnés dans un groupe $D_3$ de substituants, un groupe alkyle en $C_1$ à $C_6$ qui peut être substitué avec un ou plusieurs substituants sélectionnés dans le groupe $D_3$ de substituants, un groupe cycloalkyle en $C_3$ à $C_8$ qui peut être substitué avec un ou plusieurs substituants sélectionnés dans le groupe $D_3$ de substituants, un groupe aryle qui peut être substitué avec un ou plusieurs substituants sélectionnés dans le groupe $D_3$ de substituants, un groupe alcoxy en $C_1$ à $C_6$ qui peut être substitué avec un ou plusieurs substituants sélectionnés dans le groupe $D_3$ de substituants, un groupe aryloxy qui peut être substitué avec un ou plusieurs substituants sélectionnés dans le groupe $D_3$ de substituants, un groupe alkylamino en $C_1$ à $C_6$ qui peut être substitué avec un ou plusieurs substituants sélectionnés dans le groupe $D_3$ de substituants, un groupe di(alkyle en $C_1$ à $C_6$)amino qui peut être substitué avec un ou plusieurs substituants sélectionnés dans le groupe $D_3$ de substituants, un groupe alkylsulfonyle en $C_1$ à $C_6$ qui peut être substitué avec un ou plusieurs substituants sélectionnés dans le groupe $D_3$ de substituants, un groupe acyle qui peut être substitué avec un ou plusieurs substituants sélectionnés dans le groupe $D_3$ de substituants, un groupe hétéroaryle qui peut être substitué avec un ou plusieurs substituants sélectionnés dans le groupe $D_3$ de substituants, un groupe hétérocyclyle qui peut être substitué avec un ou plusieurs substituants sélectionnés dans le groupe $D_3$ de substituants, un groupe silyle qui peut être substitué avec un ou plusieurs substituants sélectionnés dans le groupe $D_3$ de substituants, et un groupe oxo ;

le groupe $D_3$ de substituants consiste en un atome d'halogène, un groupe hydroxyle, un groupe alkyle en $C_1$ à $C_6$, un groupe cycloalkyle en $C_3$ à $C_8$, un groupe aryle, un groupe alcoxy en $C_1$ à $C_6$, un groupe alkylamino en $C_1$ à $C_6$, un groupe di(alkyle en $C_1$ à $C_6$)amino, un groupe alkylsulfonyle en $C_1$ à $C_6$, un groupe hétéroaryle, un groupe hétérocyclyle, et un groupe oxo ;

le groupe $E_3$ de substituants consiste en un groupe cycloalkyle en $C_3$ à $C_8$ qui peut être substitué avec un ou plusieurs substituants sélectionnés dans un groupe $F_3$ de substituants, un groupe aryle qui peut être substitué avec un ou plusieurs substituants sélectionnés dans le groupe $F_3$ de substituants, un groupe hétéroaryle qui peut être substitué avec un ou plusieurs substituants sélectionnés dans le groupe $F_3$ de substituants, et un

groupe hétérocyclyle qui peut être substitué avec un ou plusieurs substituants sélectionnés dans le groupe $F_3$ de substituants ; et

le groupe $F_3$ de substituants consiste en un atome d'halogène, un groupe carboxyle, et un groupe alkyle en $C_1$ à $C_6$.

**2.** Composé hétérocyclique azoté ou sel de celui-ci selon la revendication 1, dans lequel :
$R^1$ représente un groupe pyrazolyle qui peut être substitué avec un ou plusieurs substituants sélectionnés dans le groupe $A_1$ de substituants, un groupe imidazolyle qui peut être substitué avec un ou plusieurs substituants sélectionnés dans le groupe $A_1$ de substituants, un groupe triazolyle qui peut être substitué avec un ou plusieurs substituants sélectionnés dans le groupe $A_1$ de substituants, un groupe thiazolyle qui peut être substitué avec un ou plusieurs substituants sélectionnés dans le groupe $A_1$ de substituants, un groupe oxadiazolyle qui peut être substitué avec un ou plusieurs substituants sélectionnés dans le groupe $A_1$ de substituants, un groupe thiadiazolyle qui peut être substitué avec un ou plusieurs substituants sélectionnés dans le groupe $A_1$ de substituants, un groupe pyridyle qui peut être substitué avec un ou plusieurs substituants sélectionnés dans le groupe $A_1$ de substituants, ou un groupe pyridazinyle qui peut être substitué avec un ou plusieurs substituants sélectionnés dans le groupe $A_1$ de substituants.

**3.** Composé hétérocyclique azoté ou sel de celui-ci selon la revendication 1 ou 2, dans lequel :

$R^1$ représente un groupe pyrazolyle qui peut être substitué avec un ou plusieurs substituants sélectionnés dans un groupe $\alpha_1$ de substituants, un groupe imidazolyle qui peut être substitué avec un ou plusieurs substituants sélectionnés dans le groupe $\alpha_1$ de substituants, un groupe triazolyle qui peut être substitué avec un ou plusieurs substituants sélectionnés dans le groupe $\alpha_1$ de substituants, un groupe thiazolyle qui peut être substitué avec un ou plusieurs substituants sélectionnés dans le groupe $\alpha_1$ de substituants, un groupe oxadiazolyle qui peut être substitué avec un ou plusieurs substituants sélectionnés dans le groupe $\alpha_1$ de substituants, un groupe thiadiazolyle qui peut être substitué avec un ou plusieurs substituants sélectionnés dans le groupe $\alpha_1$ de substituants, un groupe pyridyle qui peut être substitué avec un ou plusieurs substituants sélectionnés dans le groupe $\alpha_1$ de substituants, ou un groupe pyridazinyle qui peut être substitué avec un ou plusieurs substituants sélectionnés dans le groupe $\alpha_1$ de substituants ;

le groupe $\alpha_1$ de substituants consiste en un atome d'halogène, un groupe hydroxyle qui peut être protégé par un groupe protecteur sélectionné parmi un groupe alkyle en $C_1$ à $C_6$, un groupe alcényle en $C_2$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe amino qui peut être protégé par un groupe protecteur sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe aryloxycarbonyle, un groupe alkylsulfonyle en $C_1$ à $C_6$, un groupe arylsulfonyle, et un groupe silyle, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, un groupe cyano, un groupe carbamoyle qui peut être substitué avec un ou plusieurs substituants sélectionnés dans un groupe $\beta_1$ de substituants, un groupe sulfamoyle qui peut être substitué avec un ou plusieurs substituants sélectionnés dans le groupe $\beta_1$ de substituants, un groupe alkyle en $C_1$ à $C_6$ qui peut être substitué avec un ou plusieurs substituants sélectionnés dans le groupe $\beta_1$ de substituants, un groupe cycloalkyle en $C_3$ à $C_8$ qui peut être substitué avec un ou plusieurs substituants sélectionnés dans le groupe $\beta_1$ de substituants, un groupe aryle qui peut être substitué avec un ou plusieurs substituants sélectionnés dans le groupe $\beta_1$ de substituants, un groupe alcoxy en $C_1$ à $C_6$ qui peut être substitué avec un ou plusieurs substituants sélectionnés dans le groupe $\beta_1$ de substituants, un groupe aryloxy qui peut être substitué avec un ou plusieurs substituants sélectionnés dans le groupe $\beta_1$ de substituants, un groupe alkylthio en $C_1$ à $C_6$ qui peut être substitué avec un ou plusieurs substituants sélectionnés dans le groupe $\beta_1$ de substituants, un groupe arylthio qui peut être substitué avec un ou plusieurs substituants sélectionnés dans le groupe $\beta_1$ de substituants, un groupe alkylamino en $C_1$ à $C_6$ qui peut être substitué avec un ou plusieurs substituants sélectionnés dans le groupe $\beta_1$ de substituants, un groupe di(alkyle en $C_1$ à $C_6$)amino qui peut être substitué avec un ou plusieurs substituants sélectionnés dans le groupe $\beta_1$ de substituants, un groupe acyle qui peut être substitué avec un ou plusieurs substituants sélectionnés dans le groupe $\beta_1$ de substituants, un groupe hétéroaryle qui peut être substitué avec un ou plusieurs substituants sélectionnés dans un groupe $\beta_1$ de substituants, un groupe hétérocyclyle qui peut être substitué avec un ou plusieurs substituants sélectionnés dans le groupe $\beta_1$ de substituants, et un groupe oxo ;

le groupe $\beta_1$ de substituants consiste en un atome d'halogène, un groupe hydroxyle qui peut être protégé par

un groupe protecteur sélectionné parmi un groupe alkyle en $C_1$ à $C_6$, un groupe alcényle en $C_2$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe amino qui peut être protégé par un groupe protecteur sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe aryloxycarbonyle, un groupe alkylsulfonyle en $C_1$ à $C_6$, un groupe arylsulfonyle, et un groupe silyle, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, un groupe cyano, un groupe carbamoyle qui peut être substitué avec un ou plusieurs substituants sélectionnés dans un groupe $\gamma_1$ de substituants, un groupe alkyle en $C_1$ à $C_6$ qui peut être substitué avec un ou plusieurs substituants sélectionnés dans le groupe $\gamma_1$ de substituants, un groupe cycloalkyle en $C_3$ à $C_8$ qui peut être substitué avec un ou plusieurs substituants sélectionnés dans le groupe $\gamma_1$ de substituants, un groupe aryle qui peut être substitué avec un ou plusieurs substituants sélectionnés dans le groupe $\gamma_1$ de substituants, un groupe alcoxy en $C_1$ à $C_6$ qui peut être substitué avec un ou plusieurs substituants sélectionnés dans le groupe $\gamma_1$ de substituants, un groupe alkylamino en $C_1$ à $C_6$ qui peut être substitué avec un ou plusieurs substituants sélectionnés dans le groupe $\gamma_1$ de substituants, un groupe di(alkyle en $C_1$ à $C_6$)amino qui peut être substitué avec un ou plusieurs substituants sélectionnés dans le groupe $\gamma_1$ de substituants, un groupe alkylsulfonyle en $C_1$ à $C_6$ qui peut être substitué avec un ou plusieurs substituants sélectionnés dans le groupe $\gamma_1$ de substituants, un groupe arylsulfonyle qui peut être substitué avec un ou plusieurs substituants sélectionnés dans le groupe $\gamma_1$ de substituants, un groupe acyle qui peut être substitué avec un ou plusieurs substituants sélectionnés dans le groupe $\gamma_1$ de substituants, un groupe hétéroaryle qui peut être substitué avec un ou plusieurs substituants sélectionnés dans le groupe $\gamma_1$ de substituants, un groupe hétérocyclyle qui peut être substitué avec un ou plusieurs substituants sélectionnés dans le groupe $\gamma_1$ de substituants, et un groupe oxo ; et

le groupe $\gamma_1$ de substituants consiste en un atome d'halogène, un groupe hydroxyle qui peut être protégé par un groupe protecteur sélectionné parmi un groupe alkyle en $C_1$ à $C_6$, un groupe alcényle en $C_2$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe amino qui peut être protégé par un groupe protecteur sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe aryloxycarbonyle, un groupe alkylsulfonyle en $C_1$ à $C_6$, un groupe arylsulfonyle, et un groupe silyle, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, un groupe cyano, un groupe carbamoyle qui peut être substitué avec un groupe alkyle en $C_1$ à $C_6$, un groupe alkyle en $C_1$ à $C_6$ qui peut être substitué avec un atome d'halogène, un groupe alcoxy en $C_1$ à $C_6$ qui peut être substitué avec un atome d'halogène, et un groupe oxo.

**4.** Composé hétérocyclique azoté ou sel de celui-ci selon l'une quelconque des revendications 1 à 3, dans lequel :

$Z^1$ représente CH ;
$R^5$ représente un groupe hétéroaryle qui peut être substitué avec un ou plusieurs substituants sélectionnés dans le groupe $A_3$ de substituants, un groupe hétérocyclyle qui peut être substitué avec un ou plusieurs substituants sélectionnés dans le groupe $A_3$ de substituants, ou $NR^{12b}R^{13b}$ ;
$R^{12b}$ représente un atome d'hydrogène ou un groupe alkyle en $C_1$ à $C_6$ ;
$R^{13b}$ représente un groupe cycloalkyle en $C_3$ à $C_8$ qui peut être substitué avec un ou plusieurs substituants sélectionnés dans le groupe $A_3$ de substituants, un groupe aryle qui peut être substitué avec un ou plusieurs substituants sélectionnés dans le groupe $A_3$ de substituants, un groupe hétéroaryle qui peut être substitué avec un ou plusieurs substituants sélectionnés dans le groupe $A_3$ de substituants, ou un groupe hétérocyclyle qui peut être substitué avec un ou plusieurs substituants sélectionnés dans le groupe $A_3$ de substituants.

**5.** Composé hétérocyclique azoté ou sel de celui-ci selon l'une quelconque des revendications 1 à 3, dans lequel :

$Z^1$ représente CH ;
$R^5$ représente un groupe hétéroaryle qui peut être substitué avec un ou plusieurs substituants sélectionnés dans le groupe $\alpha_3$ de substituants, un groupe hétérocyclyle qui peut être substitué avec un ou plusieurs substituants sélectionnés dans le groupe $\alpha_3$ de substituants, ou $NR^{12b}R^{13c}$ ;
$R^{12b}$ représente un atome d'hydrogène ou un groupe alkyle en $C_1$ à $C_6$ ;
$R^{13c}$ représente un groupe cycloalkyle en $C_3$ à $C_8$ qui peut être substitué avec un ou plusieurs substituants sélectionnés dans le groupe $\alpha_3$ de substituants, un groupe aryle qui peut être substitué avec un ou plusieurs substituants sélectionnés dans le groupe $\alpha_3$ de substituants, un groupe hétéroaryle qui peut être substitué avec un ou plusieurs substituants sélectionnés dans le groupe $\alpha_3$ de substituants, ou un groupe hétérocyclyle qui peut être substitué avec un ou plusieurs substituants sélectionnés dans le groupe $\alpha_3$ de substituants ;
le groupe $\alpha_3$ de substituants consiste en un atome d'halogène, un groupe hydroxyle qui peut être protégé par un groupe protecteur sélectionné parmi un groupe alkyle en $C_1$ à $C_6$, un groupe alcényle en $C_2$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe amino qui peut être protégé par un groupe protecteur sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe aryloxycarbonyle, un groupe alkylsulfonyle en $C_1$ à $C_6$, un groupe arylsulfonyle, et un groupe silyle, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, un groupe cyano, un groupe carbamoyle qui peut être substitué avec un ou plusieurs substituants sélectionnés dans un groupe $\beta_3$ de substituants, un groupe alkyle en $C_1$ à $C_6$ qui peut être substitué avec un ou plusieurs substituants sélectionnés dans le groupe $\beta_3$ de substituants, un groupe cycloalkyle en $C_3$ à $C_8$ qui peut être substitué avec un ou plusieurs substituants sélectionnés dans le groupe $\beta_3$ de substituants, un groupe aryle qui peut être substitué avec un ou plusieurs substituants sélectionnés dans le groupe $\beta_3$ de substituants, un groupe alcoxy en $C_1$ à $C_6$ qui peut être substitué avec un ou plusieurs substituants sélectionnés dans le groupe $\beta_3$ de substituants, un groupe aryloxy qui peut être substitué avec un ou plusieurs substituants sélectionnés dans le groupe $\beta_3$ de substituants, un groupe alkylthio en $C_1$ à $C_6$ qui peut être substitué avec un ou plusieurs substituants sélectionnés dans le groupe $\beta_3$ de substituants, un groupe arylthio qui peut être substitué avec un ou plusieurs substituants sélectionnés dans le groupe $\beta_3$ de substituants, un groupe alkylamino en $C_1$ à $C_6$ qui peut être substitué avec un ou plusieurs substituants sélectionnés dans le groupe $\beta_3$ de substituants, un groupe di(alkyle en $C_1$ à $C_6$)amino qui peut être substitué avec un ou plusieurs substituants sélectionnés dans le groupe $\beta_3$ de substituants, un groupe alkylsulfonyle en $C_1$ à $C_6$ qui peut être substitué avec un ou plusieurs substituants sélectionnés dans le groupe $\beta_3$ de substituants, un groupe arylsulfonyle qui peut être substitué avec un ou plusieurs substituants sélectionnés dans le groupe $\beta_3$ de substituants, un groupe acyle qui peut être substitué avec un ou plusieurs substituants sélectionnés dans le groupe $\beta_3$ de substituants, un groupe hétéroaryle qui peut être substitué avec un ou plusieurs substituants sélectionnés dans le groupe $\beta_3$ de substituants, un groupe hétérocyclyle qui peut être substitué avec un ou plusieurs substituants sélectionnés dans le groupe $\beta_3$ de substituants, un groupe hétérocyclyloxy qui peut être substitué avec un ou plusieurs substituants sélectionnés dans le groupe $\beta_3$ de substituants, et un groupe oxo ;
le groupe $\beta_3$ de substituants consiste en un atome d'halogène, un groupe hydroxyle qui peut être protégé par un groupe protecteur sélectionné parmi un groupe alkyle en $C_1$ à $C_6$, un groupe alcényle en $C_2$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe amino qui peut être protégé par un groupe protecteur sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe aryloxycarbonyle, un groupe alkylsulfonyle en $C_1$ à $C_6$, un groupe arylsulfonyle, et un groupe silyle, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, un groupe cyano, un groupe carbamoyle qui peut être substitué avec un ou plusieurs substituants sélectionnés dans un groupe $\gamma_3$ de substituants, un groupe alkyle en $C_1$ à $C_6$ qui peut être substitué avec un ou plusieurs substituants sélectionnés dans le groupe $\gamma_3$ de substituants, un groupe cycloalkyle en $C_3$ à $C_8$ qui peut être substitué avec un ou plusieurs substituants sélectionnés dans le groupe $\gamma_3$ de substituants, un groupe aryle qui peut être substitué avec un ou plusieurs substituants sélectionnés dans le groupe $\gamma_3$ de substituants, un groupe alcoxy en $C_1$ à $C_6$ qui peut être substitué avec un ou plusieurs substituants sélectionnés dans le groupe $\gamma_3$ de substituants, un groupe alkylamino en $C_1$ à $C_6$ qui peut être substitué avec un ou plusieurs substituants sélectionnés dans le groupe $\gamma_3$ de substituants, un groupe di(alkyle en $C_1$ à $C_6$)amino qui peut être substitué avec un ou plusieurs substituants sélectionnés dans le groupe $\gamma_3$ de substituants, un groupe alkylsulfonyle en $C_1$ à $C_6$ qui peut être substitué

avec un ou plusieurs substituants sélectionnés dans le groupe $\gamma_3$ de substituants, un groupe arylsulfonyle qui peut être substitué avec un ou plusieurs substituants sélectionnés dans le groupe $\gamma_3$ de substituants, un groupe hétéroaryle qui peut être substitué avec un ou plusieurs substituants sélectionnés dans le groupe $\gamma_3$ de substituants, un groupe hétérocyclyle qui peut être substitué avec un ou plusieurs substituants sélectionnés dans le groupe $\gamma_3$ de substituants, un groupe hétérocyclyloxy qui peut être substitué avec un ou plusieurs substituants sélectionnés dans le groupe $\gamma_3$ de substituants, et un groupe oxo ; et
le groupe $\gamma_3$ de substituants consiste en un atome d'halogène, un groupe hydroxyle qui peut être protégé par un groupe protecteur sélectionné parmi un groupe alkyle en $C_1$ à $C_6$, un groupe alcényle en $C_2$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe amino qui peut être protégé par un groupe protecteur sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe aryloxycarbonyle, un groupe alkylsulfonyle en $C_1$ à $C_6$, un groupe arylsulfonyle, et un groupe silyle, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, un groupe cyano, un groupe alkyle en $C_1$ à $C_6$ qui peut être substitué avec un atome d'halogène, un groupe alcoxy en $C_1$ à $C_6$ qui peut être substitué avec un atome d'halogène, et un groupe oxo.

**6.** Composé hétérocyclique azoté ou sel de celui-ci selon l'une quelconque des revendications 1 à 3, dans lequel :

$Z^1$ représente CH ;
$R^5$ représente un groupe pyrazolyle qui peut être substitué avec un ou plusieurs substituants sélectionnés dans le groupe $\alpha_3$ de substituants, un groupe isoxazolyle qui peut être substitué avec un ou plusieurs substituants sélectionnés dans le groupe $\alpha_3$ de substituants, un groupe pyridyle qui peut être substitué avec un ou plusieurs substituants sélectionnés dans le groupe $\alpha_3$ de substituants, un groupe pyrimidinyle qui peut être substitué avec un ou plusieurs substituants sélectionnés dans le groupe $\alpha_3$ de substituants, un groupe pyrazinyle qui peut être substitué avec un ou plusieurs substituants sélectionnés dans le groupe $\alpha_3$ de substituants, un groupe pyridazinyle qui peut être substitué avec un ou plusieurs substituants sélectionnés dans le groupe $\alpha_3$ de substituants, un azétidinyle qui peut être substitué avec un ou plusieurs substituants sélectionnés dans le groupe $\beta_3$ de substituants, un groupe pyrrolidinyle qui peut être substitué avec un ou plusieurs substituants sélectionnés dans le groupe $\alpha_3$ de substituants, un groupe pipéridinyle qui peut être substitué avec un ou plusieurs substituants sélectionnés dans le groupe $\alpha_3$ de substituants, un groupe pipérazinyle qui peut être substitué avec un ou plusieurs substituants sélectionnés dans le groupe $\alpha_3$ de substituants, un groupe morpholinyle qui peut être substitué avec un ou plusieurs substituants sélectionnés dans le groupe $\alpha_3$ de substituants, un groupe homopipérazinyle qui peut être substitué avec un ou plusieurs substituants sélectionnés dans le groupe $\alpha_3$ de substituants, ou $NR^{12b}R^{13d}$ ;
$R^{12b}$ représente un atome d'hydrogène ou un groupe alkyle en $C_1$ à $C_6$ ;
$R^{13d}$ représente un groupe phényle qui peut être substitué avec un ou plusieurs substituants sélectionnés dans le groupe $\alpha_3$ de substituants, un groupe pyrazolyle qui peut être substitué avec un ou plusieurs substituants sélectionnés dans le groupe $\alpha_3$ de substituants, un groupe isoxazolyle qui peut être substitué avec un ou plusieurs substituants sélectionnés dans le groupe $\alpha_3$ de substituants, un groupe pyridyle qui peut être substitué avec un ou plusieurs substituants sélectionnés dans le groupe $\alpha_3$ de substituants, un groupe pyrimidinyle qui peut être substitué avec un ou plusieurs substituants sélectionnés dans le groupe $\alpha_3$ de substituants, un groupe pyrazinyle qui peut être substitué avec un ou plusieurs substituants sélectionnés dans le groupe $\alpha_3$ de substituants, un groupe pyridazinyle qui peut être substitué avec un ou plusieurs substituants sélectionnés dans le groupe $\alpha_3$ de substituants, un groupe pyrrolidinyle qui peut être substitué avec un ou plusieurs substituants sélectionnés dans le groupe $\alpha_3$ de substituants, un groupe pipéridinyle qui peut être substitué avec un ou plusieurs substituants sélectionnés dans le groupe $\alpha_3$ de substituants, ou un groupe tétrahydropyridyle qui peut être substitué avec un ou plusieurs substituants sélectionnés dans le groupe $\alpha_3$ de substituants ;
le groupe $\alpha_3$ de substituants consiste en un atome d'halogène, un groupe hydroxyle qui peut être protégé par un groupe protecteur sélectionné parmi un groupe alkyle en $C_1$ à $C_6$, un groupe alcényle en $C_2$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe amino qui peut être protégé par un groupe protecteur sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-

alcoxycarbonyle en $C_1$ à $C_6$, un groupe aryloxycarbonyle, un groupe alkylsulfonyle en $C_1$ à $C_6$, un groupe arylsulfonyle, et un groupe silyle, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, un groupe cyano, un groupe carbamoyle qui peut être substitué avec un ou plusieurs substituants sélectionnés dans un groupe $\beta_3$ de substituants, un groupe alkyle en $C_1$ à $C_6$ qui peut être substitué avec un ou plusieurs substituants sélectionnés dans le groupe $\beta_3$ de substituants, un groupe cycloalkyle en $C_3$ à $C_8$ qui peut être substitué avec un ou plusieurs substituants sélectionnés dans le groupe $\beta_3$ de substituants, un groupe aryle qui peut être substitué avec un ou plusieurs substituants sélectionnés dans le groupe $\beta_3$ de substituants, un groupe alcoxy en $C_1$ à $C_6$ qui peut être substitué avec un ou plusieurs substituants sélectionnés dans le groupe $\beta_3$ de substituants, un groupe aryloxy qui peut être substitué avec un ou plusieurs substituants sélectionnés dans le groupe $\beta_3$ de substituants, un groupe alkylthio en $C_1$ à $C_6$ qui peut être substitué avec un ou plusieurs substituants sélectionnés dans le groupe $\beta_3$ de substituants, un groupe arylthio qui peut être substitué avec un ou plusieurs substituants sélectionnés dans le groupe $\beta_3$ de substituants, un groupe alkylamino en $C_1$ à $C_6$ qui peut être substitué avec un ou plusieurs substituants sélectionnés dans le groupe $\beta_3$ de substituants, un groupe di(alkyle en $C_1$ à $C_6$)amino qui peut être substitué avec un ou plusieurs substituants sélectionnés dans le groupe $\beta_3$ de substituants, un groupe alkylsulfonyle en $C_1$ à $C_6$ qui peut être substitué avec un ou plusieurs substituants sélectionnés dans le groupe $\beta_3$ de substituants, un groupe arylsulfonyle qui peut être substitué avec un ou plusieurs substituants sélectionnés dans le groupe $\beta_3$ de substituants, un groupe acyle qui peut être substitué avec un ou plusieurs substituants sélectionnés dans le groupe $\beta_3$ de substituants, un groupe hétéroaryle qui peut être substitué avec un ou plusieurs substituants sélectionnés dans le groupe $\beta_3$ de substituants, un groupe hétérocyclyle qui peut être substitué avec un ou plusieurs substituants sélectionnés dans le groupe $\beta_3$ de substituants, un groupe hétérocyclyloxy qui peut être substitué avec un ou plusieurs substituants sélectionnés dans le groupe $\beta_3$ de substituants, et un groupe oxo ;

le groupe $\beta_3$ de substituants consiste en un atome d'halogène, un groupe hydroxyle qui peut être protégé par un groupe protecteur sélectionné parmi un groupe alkyle en $C_1$ à $C_6$, un groupe alcényle en $C_2$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe amino qui peut être protégé par un groupe protecteur sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe aryloxycarbonyle, un groupe alkylsulfonyle en $C_1$ à $C_6$, un groupe arylsulfonyle, et un groupe silyle, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, un groupe cyano, un groupe carbamoyle qui peut être substitué avec un ou plusieurs substituants sélectionnés dans un groupe $\gamma_3$ de substituants, un groupe alkyle en $C_1$ à $C_6$ qui peut être substitué avec un ou plusieurs substituants sélectionnés dans le groupe $\gamma_3$ de substituants, un groupe cycloalkyle en $C_3$ à $C_8$ qui peut être substitué avec un ou plusieurs substituants sélectionnés dans le groupe $\gamma_3$ de substituants, un groupe aryle qui peut être substitué avec un ou plusieurs substituants sélectionnés dans le groupe $\gamma_3$ de substituants, un groupe alcoxy en $C_1$ à $C_6$ qui peut être substitué avec un ou plusieurs substituants sélectionnés dans le groupe $\gamma_3$ de substituants, un groupe alkylamino en $C_1$ à $C_6$ qui peut être substitué avec un ou plusieurs substituants sélectionnés dans le groupe $\gamma_3$ de substituants, un groupe di(alkyle en $C_1$ à $C_6$)amino qui peut être substitué avec un ou plusieurs substituants sélectionnés dans le groupe $\gamma_3$ de substituants, un groupe alkylsulfonyle en $C_1$ à $C_6$ qui peut être substitué avec un ou plusieurs substituants sélectionnés dans le groupe $\gamma_3$ de substituants, un groupe arylsulfonyle qui peut être substitué avec un ou plusieurs substituants sélectionnés dans le groupe $\gamma_3$ de substituants, un groupe hétéroaryle qui peut être substitué avec un ou plusieurs substituants sélectionnés dans le groupe $\gamma_3$ de substituants, un groupe hétérocyclyle qui peut être substitué avec un ou plusieurs substituants sélectionnés dans le groupe $\gamma_3$ de substituants, un groupe hétérocyclyloxy qui peut être substitué avec un ou plusieurs substituants sélectionnés dans le groupe $\gamma_3$ de substituants, et un groupe oxo ; et

le groupe $\gamma_3$ de substituants consiste en un atome d'halogène, un groupe hydroxyle qui peut être protégé par un groupe protecteur sélectionné parmi un groupe alkyle en $C_1$ à $C_6$, un groupe alcényle en $C_2$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe amino qui peut être protégé par un groupe protecteur sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe aryloxycarbonyle, un groupe alkylsulfonyle en $C_1$ à $C_6$, un groupe

arylsulfonyle, et un groupe silyle, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, un groupe cyano, un groupe alkyle en $C_1$ à $C_6$ qui peut être substitué avec un atome d'halogène, un groupe alcoxy en $C_1$ à $C_6$ qui peut être substitué avec un atome d'halogène, et un groupe oxo.

**7.** Composé hétérocyclique azoté représenté par la Formule [1a] suivante ou un sel de celui-ci :

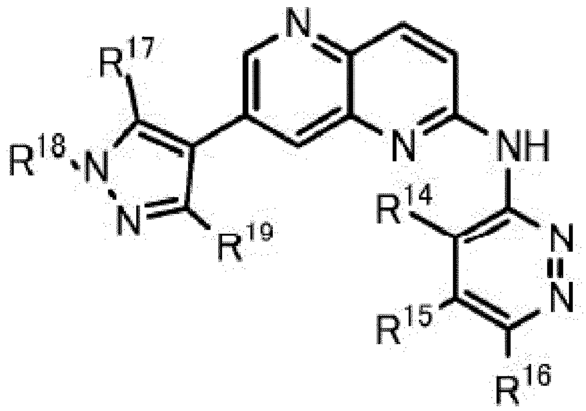

[1a]

dans laquelle, dans la formule [1a] :

chacun parmi $R^{14}$, $R^{15}$, et $R^{16}$ représente indépendamment un atome d'hydrogène, un atome d'halogène, un groupe hydroxyle qui peut être protégé par un groupe protecteur sélectionné parmi un groupe alkyle en $C_1$ à $C_6$, un groupe alcényle en $C_2$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe amino qui peut être protégé par un groupe protecteur sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe aryloxycarbonyle, un groupe alkylsulfonyle en $C_1$ à $C_6$, un groupe arylsulfonyle, et un groupe silyle, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, un groupe cyano, un groupe carbamoyle qui peut être substitué avec un ou plusieurs substituants sélectionnés dans un groupe $B_1$ de substituants, un groupe sulfamoyle qui peut être substitué avec un ou plusieurs substituants sélectionnés dans le groupe $B_1$ de substituants, un groupe alkyle en $C_1$ à $C_6$ qui peut être substitué avec un ou plusieurs substituants sélectionnés dans le groupe $B_1$ de substituants, un groupe alcényle en $C_2$ à $C_6$ qui peut être substitué avec un ou plusieurs substituants sélectionnés dans le groupe $B_1$ de substituants, un groupe cycloalkyle en $C_3$ à $C_8$ qui peut être substitué avec un ou plusieurs substituants sélectionnés dans le groupe $B_1$ de substituants, un groupe aryle qui peut être substitué avec un ou plusieurs substituants sélectionnés dans le groupe $B_1$ de substituants, un groupe alcoxy en $C_1$ à $C_6$ qui peut être substitué avec un ou plusieurs substituants sélectionnés dans le groupe $B_1$ de substituants, un groupe cycloalcoxy en $C_3$ à $C_8$ qui peut être substitué avec un ou plusieurs substituants sélectionnés dans le groupe $B_1$ de substituants, un groupe aryloxy qui peut être substitué avec un ou plusieurs substituants sélectionnés dans le groupe $B_1$ de substituants, un groupe alkylthio en $C_1$ à $C_6$ qui peut être substitué avec un ou plusieurs substituants sélectionnés dans le groupe $B_1$ de substituants, un groupe arylthio qui peut être substitué avec un ou plusieurs substituants sélectionnés dans le groupe $B_1$ de substituants, un groupe alkylamino en $C_1$ à $C_6$ qui peut être substitué avec un ou plusieurs substituants sélectionnés dans le groupe $B_1$ de substituants, un groupe di(alkyle en $C_1$ à $C_6$)amino qui peut être substitué avec un ou plusieurs substituants sélectionnés dans le groupe $B_1$ de substituants, un groupe acyle qui peut être substitué avec un ou plusieurs substituants sélectionnés dans le groupe $B_1$ de substituants, un groupe hétéroaryle qui peut être substitué avec un ou plusieurs substituants sélectionnés dans un groupe $B_1$ de substituants, ou un groupe hétérocyclyle qui peut être substitué avec un ou plusieurs substituants sélectionnés dans le groupe $B_1$ de substituants ;

chacun parmi $R^{17}$, $R^{18}$, et $R^{19}$ représente indépendamment un atome d'hydrogène, un atome d'halogène, un groupe hydroxyle qui peut être protégé par un groupe protecteur sélectionné parmi un groupe alkyle en $C_1$ à

$C_6$, un groupe alcényle en $C_2$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe amino qui peut être protégé par un groupe protecteur sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe aryloxycarbonyle, un groupe alkylsulfonyle en $C_1$ à $C_6$, un groupe arylsulfonyle, et un groupe silyle, un groupe amino qui peut être substitué avec un ou plusieurs substituants sélectionnés dans un groupe $E_3$ de substituants, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, un groupe cyano, un groupe carbamoyle qui peut être substitué avec un ou plusieurs substituants sélectionnés dans un groupe $B_3$ de substituants, un groupe sulfamoyle qui peut être substitué avec un ou plusieurs substituants sélectionnés dans le groupe $B_3$ de substituants, un groupe alkyle en $C_1$ à $C_6$ qui peut être substitué avec un ou plusieurs substituants sélectionnés dans le groupe $B_3$ de substituants, un groupe alcényle en $C_2$ à $C_6$ qui peut être substitué avec un ou plusieurs substituants sélectionnés dans le groupe $B_3$ de substituants, un groupe cycloalkyle en $C_3$ à Cg qui peut être substitué avec un ou plusieurs substituants sélectionnés dans le groupe $B_3$ de substituants, un groupe aryle qui peut être substitué avec un ou plusieurs substituants sélectionnés dans le groupe $B_3$ de substituants, un groupe alcoxy en $C_1$ à $C_6$ qui peut être substitué avec un ou plusieurs substituants sélectionnés dans le groupe $B_3$ de substituants, un groupe aryloxy qui peut être substitué avec un ou plusieurs substituants sélectionnés dans le groupe $B_3$ de substituants, un groupe alkylthio en $C_1$ à $C_6$ qui peut être substitué avec un ou plusieurs substituants sélectionnés dans le groupe $B_3$ de substituants, un groupe arylthio qui peut être substitué avec un ou plusieurs substituants sélectionnés dans le groupe $B_3$ de substituants, un groupe alkylamino en $C_1$ à $C_6$ qui peut être substitué avec un ou plusieurs substituants sélectionnés dans le groupe $B_3$ de substituants, un groupe di(alkyle en $C_1$ à $C_6$)amino qui peut être substitué avec un ou plusieurs substituants sélectionnés dans le groupe $B_3$ de substituants, un groupe alkylsulfonyle en $C_1$ à $C_6$ qui peut être substitué avec un ou plusieurs substituants sélectionnés dans le groupe $B_3$ de substituants, un groupe arylsulfonyle qui peut être substitué avec un ou plusieurs substituants sélectionnés dans le groupe $B_3$ de substituants, un groupe acyle qui peut être substitué avec un ou plusieurs substituants sélectionnés dans le groupe $B_3$ de substituants, un groupe hétéroaryle qui peut être substitué avec un ou plusieurs substituants sélectionnés dans un groupe $B_3$ de substituants, un groupe hétérocyclyle qui peut être substitué avec un ou plusieurs substituants sélectionnés dans le groupe $B_3$ de substituants, ou un groupe hétérocyclyloxy qui peut être substitué avec un ou plusieurs substituants sélectionnés dans le groupe $B_3$ de substituants ;

le groupe $B_1$ de substituants consiste en un atome d'halogène, un groupe hydroxyle qui peut être protégé par un groupe protecteur sélectionné parmi un groupe alkyle en $C_1$ à $C_6$, un groupe alcényle en $C_2$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe amino qui peut être protégé par un groupe protecteur sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe aryloxycarbonyle, un groupe alkylsulfonyle en $C_1$ à $C_6$, un groupe arylsulfonyle, et un groupe silyle, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, un groupe cyano, un groupe carbamoyle qui peut être substitué avec un ou plusieurs substituants sélectionnés dans un groupe $C_1$ de substituants, un groupe sulfamoyle qui peut être substitué avec un ou plusieurs substituants sélectionnés dans le groupe $C_1$ de substituants, un groupe alkyle en $C_1$ à $C_6$ qui peut être substitué avec un ou plusieurs substituants sélectionnés dans le groupe $C_1$ de substituants, un groupe cycloalkyle en $C_3$ à $C_8$ qui peut être substitué avec un ou plusieurs substituants sélectionnés dans le groupe $C_1$ de substituants, un groupe aryle qui peut être substitué avec un ou plusieurs substituants sélectionnés dans le groupe $C_1$ de substituants, un groupe alcoxy en $C_1$ à $C_6$ qui peut être substitué avec un ou plusieurs substituants sélectionnés dans le groupe $C_1$ de substituants, un groupe alkylamino en $C_1$ à $C_6$ qui peut être substitué avec un ou plusieurs substituants sélectionnés dans le groupe $C_1$ de substituants, un groupe di(alkyle en $C_1$ à $C_6$)amino qui peut être substitué avec un ou plusieurs substituants sélectionnés dans le groupe $C_1$ de substituants, un groupe alkylsulfonyle en $C_1$ à $C_6$ qui peut être substitué avec un ou plusieurs substituants sélectionnés dans le groupe $C_1$ de substituants, un groupe arylsulfonyle qui peut être substitué avec un ou plusieurs substituants sélectionnés dans le groupe $C_1$ de substituants, un groupe acyle qui peut être substitué avec un ou plusieurs substituants sélectionnés dans le groupe $C_1$ de substituants, un groupe hétéroaryle qui peut être substitué avec un ou plusieurs substituants

sélectionnés dans le groupe $C_1$ de substituants, un groupe hétérocyclyle qui peut être substitué avec un ou plusieurs substituants sélectionnés dans le groupe $C_1$ de substituants, et un groupe oxo ;

le groupe $C_1$ de substituants consiste en un atome d'halogène, un groupe hydroxyle qui peut être protégé par un groupe protecteur sélectionné parmi un groupe alkyle en $C_1$ à $C_6$, un groupe alcényle en $C_2$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe amino qui peut être protégé par un groupe protecteur sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe aryloxycarbonyle, un groupe alkylsulfonyle en $C_1$ à $C_6$, un groupe arylsulfonyle, et un groupe silyle, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, un groupe cyano, un groupe carbamoyle qui peut être substitué avec un groupe alkyle en $C_1$ à $C_6$, un groupe alkyle en $C_1$ à $C_6$ qui peut être substitué avec un atome d'halogène, un groupe aryle qui peut être substitué avec un atome d'halogène ou un groupe alkyle en $C_1$ à $C_6$, un groupe alcoxy en $C_1$ à $C_6$ qui peut être substitué avec un atome d'halogène, et un groupe oxo ;

le groupe $B_3$ de substituants consiste en un atome d'halogène, un groupe hydroxyle, un groupe amino, un groupe carboxyle, un groupe cyano, un groupe carbamoyle qui peut être substitué avec un ou plusieurs substituants sélectionnés dans un groupe $C_3$ de substituants, un groupe alkyle en $C_1$ à $C_6$ qui peut être substitué avec un ou plusieurs substituants sélectionnés dans le groupe $C_3$ de substituants, un groupe cycloalkyle en $C_3$ à $C_8$ qui peut être substitué avec un ou plusieurs substituants sélectionnés dans le groupe $C_3$ de substituants, un groupe aryle qui peut être substitué avec un ou plusieurs substituants sélectionnés dans le groupe $C_3$ de substituants, un groupe alcoxy en $C_1$ à $C_6$ qui peut être substitué avec un ou plusieurs substituants sélectionnés dans le groupe $C_3$ de substituants, un groupe aryloxy qui peut être substitué avec un ou plusieurs substituants sélectionnés dans le groupe $C_3$ de substituants, un groupe alkylamino en $C_1$ à $C_6$ qui peut être substitué avec un ou plusieurs substituants sélectionnés dans le groupe $C_3$ de substituants, un groupe di(alkyle en $C_1$ à $C_6$)amino qui peut être substitué avec un ou plusieurs substituants sélectionnés dans le groupe $C_3$ de substituants, un groupe alkylsulfonyle en $C_1$ à $C_6$ qui peut être substitué avec un ou plusieurs substituants sélectionnés dans le groupe $C_3$ de substituants, un groupe arylsulfonyle qui peut être substitué avec un ou plusieurs substituants sélectionnés dans le groupe $C_3$ de substituants, un groupe acyle qui peut être substitué avec un ou plusieurs substituants sélectionnés dans le groupe $C_3$ de substituants, un groupe hétéroaryle qui peut être substitué avec un ou plusieurs substituants sélectionnés dans un groupe $C_3$ de substituants, un groupe hétérocyclyle qui peut être substitué avec un ou plusieurs substituants sélectionnés dans le groupe $C_3$ de substituants, un groupe hétéroaryloxy qui peut être substitué avec un ou plusieurs substituants sélectionnés dans un groupe $C_3$ de substituants, un groupe hétérocyclyloxy qui peut être substitué avec un ou plusieurs substituants sélectionnés dans le groupe $C_3$ de substituants, et un groupe oxo ;

le groupe $C_3$ de substituants consiste en un atome d'halogène, un groupe hydroxyle qui peut être protégé par un groupe protecteur sélectionné parmi un groupe alkyle en $C_1$ à $C_6$, un groupe alcényle en $C_2$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe amino qui peut être protégé par un groupe protecteur sélectionné parmi un groupe aryl-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe aryloxycarbonyle, un groupe alkylsulfonyle en $C_1$ à $C_6$, un groupe arylsulfonyle, et un groupe silyle, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, un groupe nitro, un groupe cyano, un groupe carbamoyle qui peut être substitué avec un ou plusieurs substituants sélectionnés dans un groupe $D_3$ de substituants, un groupe alkyle en $C_1$ à $C_6$ qui peut être substitué avec un ou plusieurs substituants sélectionnés dans le groupe $D_3$ de substituants, un groupe cycloalkyle en $C_3$ à $C_8$ qui peut être substitué avec un ou plusieurs substituants sélectionnés dans le groupe $D_3$ de substituants, un groupe aryle qui peut être substitué avec un ou plusieurs substituants sélectionnés dans le groupe $D_3$ de substituants, un groupe alcoxy en $C_1$ à $C_6$ qui peut être substitué avec un ou plusieurs substituants sélectionnés dans le groupe $D_3$ de substituants, un groupe aryloxy qui peut être substitué avec un ou plusieurs substituants sélectionnés dans le groupe $D_3$ de substituants, un groupe alkylamino en $C_1$ à $C_6$ qui peut être substitué avec un ou plusieurs substituants sélectionnés dans le groupe $D_3$ de substituants, un groupe di(alkyle en $C_1$ à $C_6$)amino qui peut être substitué avec un ou plusieurs substituants sélectionnés dans le groupe $D_3$ de substituants, un groupe alkylsulfonyle en

$C_1$ à $C_6$ qui peut être substitué avec un ou plusieurs substituants sélectionnés dans le groupe $D_3$ de substituants, un groupe acyle qui peut être substitué avec un ou plusieurs substituants sélectionnés dans le groupe $D_3$ de substituants, un groupe hétéroaryle qui peut être substitué avec un ou plusieurs substituants sélectionnés dans le groupe $D_3$ de substituants, un groupe hétérocyclyle qui peut être substitué avec un ou plusieurs substituants sélectionnés dans le groupe $D_3$ de substituants, un groupe silyle qui peut être substitué avec un ou plusieurs substituants sélectionnés dans le groupe $D_3$ de substituants, et un groupe oxo ;
le groupe $D_3$ de substituants consiste en un atome d'halogène, un groupe hydroxyle qui peut être protégé par un groupe protecteur sélectionné parmi un groupe alkyle en $C_1$ à $C_6$, un groupe alcényle en $C_2$ à $C_6$, un groupe ar-alkyle en $C_1$ à $C_6$, un groupe alcoxy en $C_1$ à $C_6$-alkyle en $C_1$ à $C_6$, un groupe acyle, un groupe alcoxycarbonyle en $C_1$ à $C_6$, un groupe ar-alcoxycarbonyle en $C_1$ à $C_6$, un groupe alkylsulfonyle en $C_1$ à $C_6$, un groupe arylsulfonyle, un groupe silyle, un groupe tétrahydrofuranyle, et un groupe tétrahydropyranyle, un groupe alkyle en $C_1$ à $C_6$, un groupe cycloalkyle en $C_3$ à $C_8$, un groupe aryle, un groupe alcoxy en $C_1$ à $C_6$, un groupe alkylamino en $C_1$ à $C_6$, un groupe di(alkyle en $C_1$ à $C_6$)amino, un groupe alkylsulfonyle en $C_1$ à $C_6$, un groupe hétéroaryle, un groupe hétérocyclyle, et un groupe oxo ;
le groupe $E_3$ de substituants consiste en un groupe cycloalkyle en $C_3$ à $C_8$ qui peut être substitué avec un ou plusieurs substituants sélectionnés dans un groupe $F_3$ de substituants, un groupe aryle qui peut être substitué avec un ou plusieurs substituants sélectionnés dans le groupe $F_3$ de substituants, un groupe hétéroaryle qui peut être substitué avec un ou plusieurs substituants sélectionnés dans le groupe $F_3$ de substituants, et un groupe hétérocyclyle qui peut être substitué avec un ou plusieurs substituants sélectionnés dans le groupe $F_3$ de substituants ; et
le groupe $F_3$ de substituants consiste en un atome d'halogène, un groupe carboxyle qui peut être protégé par un groupe protecteur sélectionné parmi un groupe alkyle en $C_1$ à $C_6$, un groupe alcényle en $C_2$ à $C_6$, un groupe aryle, un groupe aryl-alkyle en $C_1$ à $C_6$, un groupe alcoxy en $C_1$ à $C_6$-alkyle en $C_1$ à $C_6$, et un groupe silyle, et un groupe alkyle en $C_1$ à $C_6$.

**8.** Composé hétérocyclique azoté ou sel de celui-ci selon la revendication 7, dans lequel chacun parmi $R^{14}$, $R^{16}$ et $R^{17}$ représente un atome d'hydrogène.

**9.** Composé hétérocyclique azoté ou sel de celui-ci selon la revendication 1, dans lequel le composé est l'un quelconque des composés représentés ci-dessous :

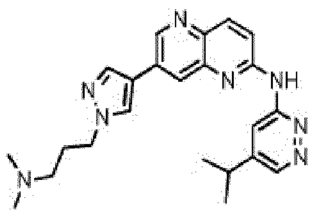
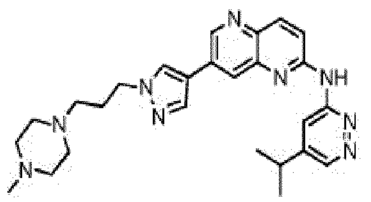
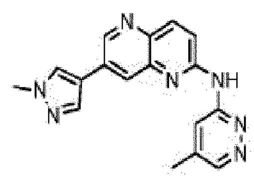

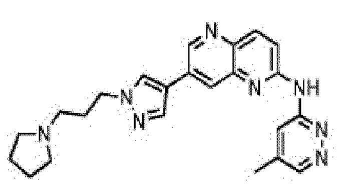
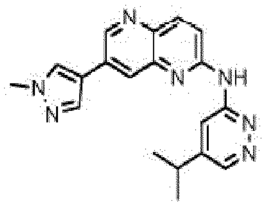
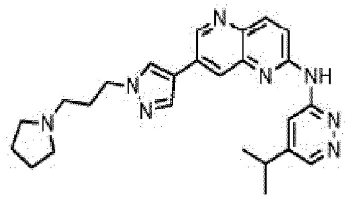

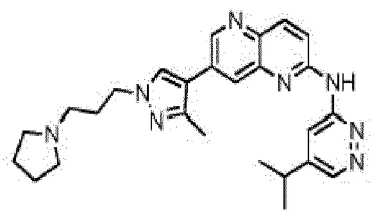
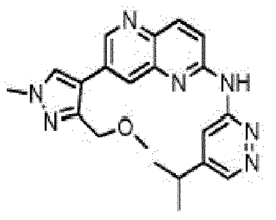
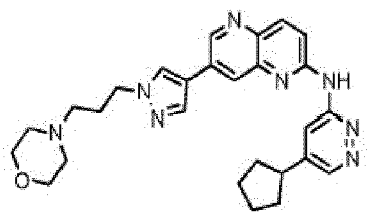

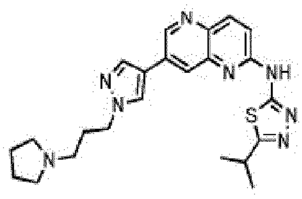
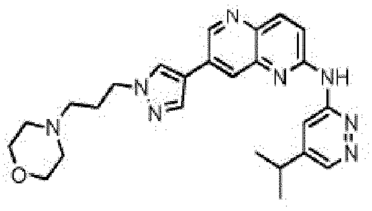
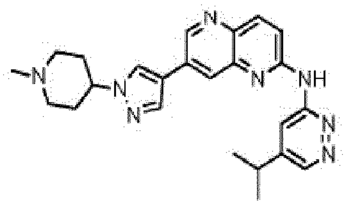
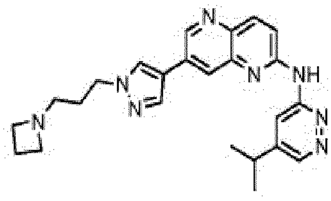
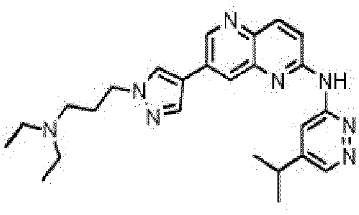
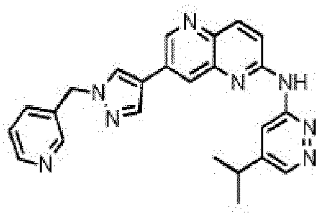
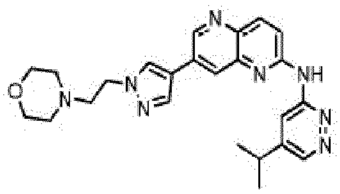
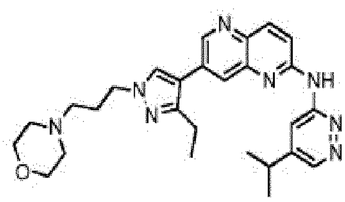
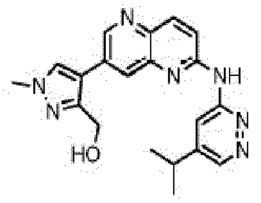
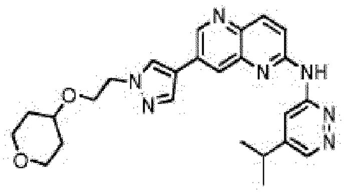
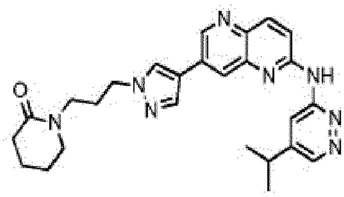
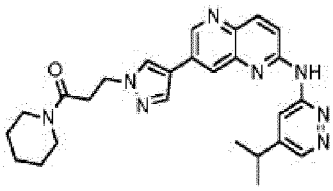
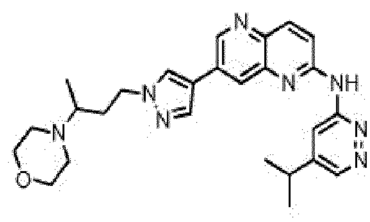
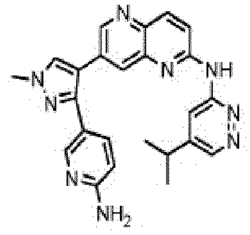
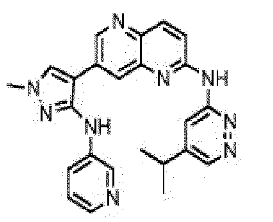
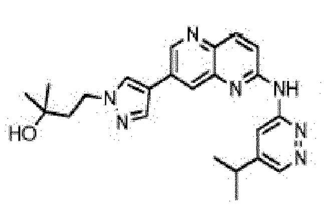
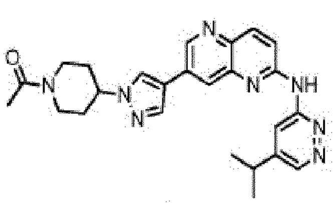
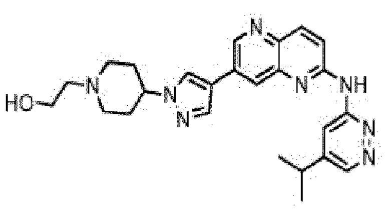
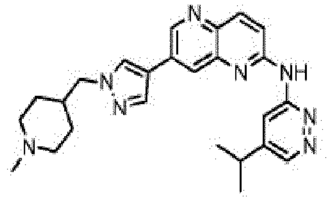
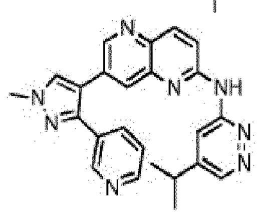
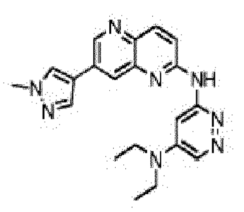

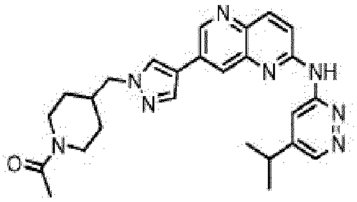
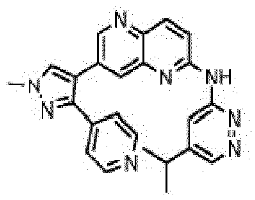
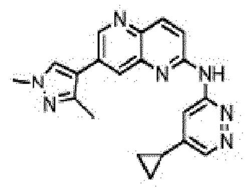

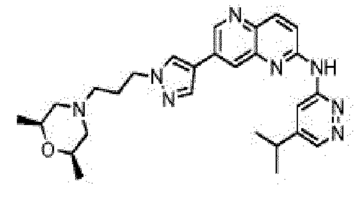
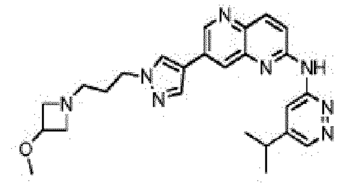
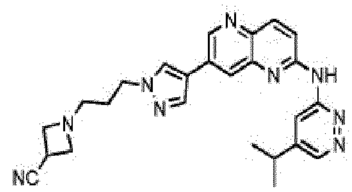

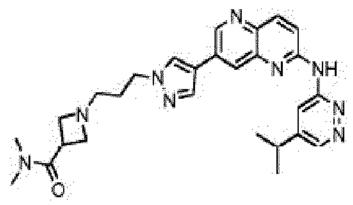
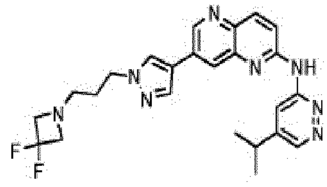

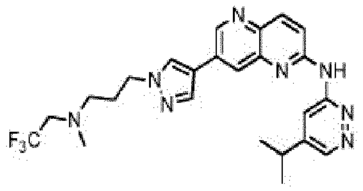
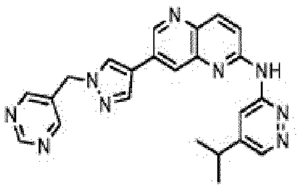
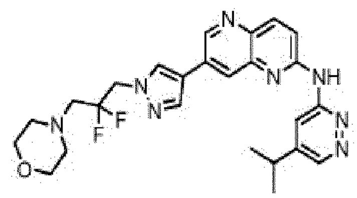

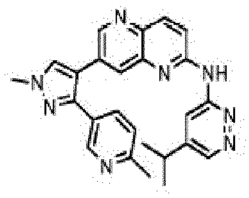
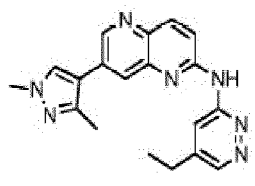
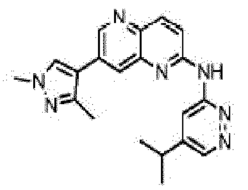

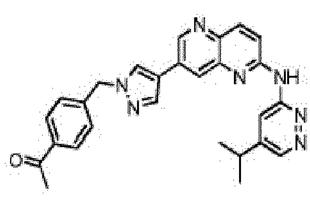
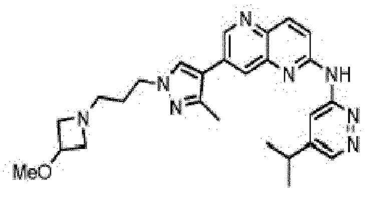
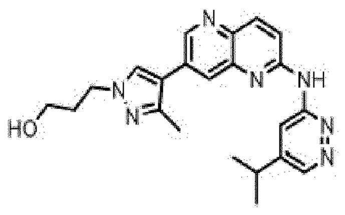

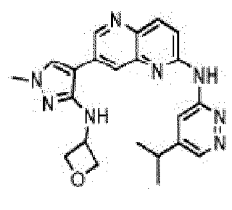
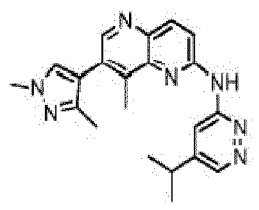

**10.** Composition pharmaceutique comprenant le composé hétérocyclique azoté ou un sel de celui-ci selon l'une quelconque des revendications 1 à 9.

**11.** Composé hétérocyclique azoté ou sel de celui-ci selon l'une quelconque des revendications 1 à 9, ou la composition pharmaceutique selon la revendication 10 pour une utilisation dans le traitement d'une maladie.

**12.** Composé hétérocyclique azoté ou sel de celui-ci selon l'une quelconque des revendications 1 à 9 ou la composition pharmaceutique selon la revendication 10 pour une utilisation dans le traitement d'une maladie, dans lequel la maladie est sélectionnée de préférence dans le groupe consistant en une tumeur maligne, une maladie proliférative cellulaire, une maladie allergique, une maladie auto-immune, une maladie neurodégénérative, une maladie du système circulatoire, une maladie inflammatoire, un trouble endocrinien, un trouble métabolique, et une infection.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011064250 A
- JP 2009515854 A
- EP 1990342 A
- WO 2009155156 A
- WO 9917759 A
- WO 2014074657 A
- JP 2003206290 A


### Non-patent literature cited in the description


- *Nature Reviews Drug Discovery,* 2009, vol. 8 (8), 627-644
- *Biochimica et Biophysica Acta,* 2008, vol. 1784 (1), 159-185
- *ChemMedChem,* 2011, vol. 6 (1), 38-48
- *Cancer Biology & Therapy,* 2008, vol. 7 (2), 307-315
- *Nature medicine,* 2008, vol. 14 (12), 1351-1356
- **W. GREENE et al.** Protective Groups in Organic Synthesis. John Wiley & Sons, INC, 2007, 16-366
- **W. GREENE et al.** Protective Groups in Organic Synthesis. John Wiley & Sons, INC, 2007, 696-926
- **W. GREENE et al.** Protective Groups in Organic Synthesis. John Wiley & Sons, INC, 2007, 533-646
- *The Journal of Organic Chemistry,* 1995, vol. 60, 7508-7510
- **M. SCHLOSSER et al.** Organometallics in Synthesis. John Wiley & Sons, INC, 2002, 1123-1217